(19) Europäisches Patentamt
European Patent Office
Office européen des brevets

(11) EP 2 213 749 A1

(12) EUROPEAN PATENT APPLICATION

(43) Date of publication:
**04.08.2010 Bulletin 2010/31**

(51) Int Cl.:
***C12Q 1/68*** *(2006.01)*

(21) Application number: **09010002.5**

(22) Date of filing: **02.12.2005**

(84) Designated Contracting States:
**AT BE BG CH CY CZ DE DK EE ES FI FR GB GR HU IE IS IT LI LT LU LV MC NL PL PT RO SE SI SK TR**

(30) Priority:
**02.12.2004 US 632426 P**
**02.12.2004 US 633250 P**
**14.03.2005 US 662220 P**
**03.10.2005 US 723125 P**
**03.10.2005 US 723054 P**
**30.11.2005 US 740736 P**

(62) Document number(s) of the earlier application(s) in accordance with Art. 76 EPC:
**05853011.4 / 1 831 399**

(27) Previously filed application:
**02.12.2005 PCT/US2005/043974**

(71) Applicant: **Epigenomics AG**
**10178 Berlin (DE)**

(72) Inventors:
- **Cottrell, Susan**
  **Seattle, WA 98115 (US)**
- **Model, Fabian**
  **12683 Berlin (DE)**
- **Haefliger, Carolina**
  **4052 Basel (CH)**
- **Weiss, Gunter**
  **10437 Berlin (DE)**
- **Distler, Jürgen**
  **12163 Berlin (DE)**
- **Sledziewski, Andrew Z.**
  **Shoreline, WA 98177 (US)**
- **Song, Xiaoling**
  **Woodinville, WA 98072 (US)**
- **Skillman, Thomas L.**
  **Kennydale, WA 98056 (US)**
- **Thomas, Jeffrey G.**
  **Oakland, CA 94618 (US)**

(74) Representative: **Krauss, Jan**
**Forrester & Boehmert**
**Pettenkoferstrasse 20-22**
**80336 München (DE)**

Remarks:
This application was filed on 03-08-2009 as a divisional application to the application mentioned under INID code 62.

# (54) Methods and nucleic acids for the analysis of gene expression associated with the prognosis of prostate cell proliferative disorders

(57) Particular aspects provide novel methods and compositions (*e.g.*, nucleic acids, kits, etc.) having substantial utility for providing a prognosis of prostate cell proliferative disorders. In particular aspects, this is achieved by the analysis of the expression status of a panel of genes, or subsets thereof.

EP 2 213 749 A1

Sequence listing

<110> Epigenomics AG
Cottrell, Susan
Model, Fabian
Haefliger, Carolina
Weiss, Gunter
Distler, Juergen
Sledziewski, Andrew Z.
Song, Xiaoling
Skillman, Tom
Thomas, Jeff

<120> METHODS AND NUCLEIC ACIDS FOR THE ANALYSIS OF GENE EXPRESSION ASSOCIATED WITH THE PROGNOSIS OF PROSTATE CELL PROLIFERATIVE DISORDERS

<130> 47675-160

<150> US 60/632,426
<151> 2004-12-02

<150> US 60/633,250
<151> 2004-12-02

<150> US 60/662,220
<151> 2005-03-14

<150> US 60/723,125
<151> 2005-10-03

<150> US 60/723,054
<151> 2005-10-03

<150>
<151> 2005-11-30

<160> 1006

<210> 1
<211> 7572
<212> DNA
<213> Homo Sapiens

<400> 1

```
tgcatgagca cggatgccac gagggtatcc agcgagtggg aagggagtcg ccctacatgt      60
tttcacatcg atttgtaaaa ctgttttgaa agggtatttc tagatccatt tgttccaaat     120
gcagcaaaat tcatccgata tcgctcacaa tgcatgcacg ggttatctta catgtaatag     180
ccatcgtatt aaccaaccca gctgatactg agatggtgaa tggggcgggg gagcagggag     240
gaatatgtcg cgcatgaaaa actccgggtg taaaagtcaa tgcagaactc actccccgag     300
gaaccgaaaa agacattcac gactctcaag tggagagagg atagaaagtc aagtggcggg     360
ggtcgcgacc agtgatcggg atttcagtct gatctcagta cccagcggaa caggaccata     420
acctaaagaa atgagctttt tccggagcaa agagcggccc cgatttcaaa gtagaaaaaa     480
taaactgctt gggggtggaa gatggcgagg ggcgggggggc ggggagggag gcgggtcgcg     540
gcgctggctc cggggtccgc ggcacggcct cctctgccgc gctgcccagg gcccggaccc     600
tgcccggcgc tgcgcccacc gcgagggtgc cagacccgcc gcgccgccgc cgcgctctcc     660
cagccgcggc cccctccccc gccgcttcct cttgctctcc cagccccttc ccccacgtgt     720
ggatgacgtc aaaattccgc gaaaaagccg cgtggtggct ccccgagcgg aggcgcgatt     780
ccgccgccca gcggccccct cccgggggcg cctggagggg gagaagggcg gaggcggccg     840
gttccttctc ctcccgggag gcaggacccc ccgacgccga ccgccggacg ccccccgccc     900
caaagcttat tggaaaattc acttttgtaa agcaaatgta tttccagagc tattttcggc     960
```

```
cgcgtgaggc gtgtcctaag ctgaatcaga caggaagagg gggaagttcg ggtcttttaa    1020
tttttttttt ttccgaaggg aggggagtga gatgctaggt gggtgacaga cggcaggcgc    1080
tcgccttctt aactcacgcc tgtcgcatct gccgcctcag taatccagcc ccgtccaagc    1140
cgaaattcgc cgaagggagt gcggatgcac aggcctggcg gactctgccc ccctccagaa    1200
cgcagcggcc cagcgccccg gcgggcgcgg ctgcgaccag agggtcccgg aagcgagtga    1260
acacctgcaa tcgcactgcc cgtccccacc caccctgctc ccccgtgctc tccgcttccc    1320
gacgttttcc tcttctcctt gtccgcattt ttctactttg cctgcactcc ctccttcctc    1380
ttgatgtgcc tcctatgtgt cccctcggat ttatgtgtcc cctcgcattt tgccagtcgg    1440
gttttcggtt ttgattgacc gtccatccct ccacggagaa acacaaacac agctccactc    1500
tttgggggag ccgaggggaa ggcagtggct cccatttctg agcctgaact cagtcactac    1560
ccgctcccca cctggcctag gcgcccctgc gcggagaagg cgggactcga actcgcgctg    1620
ctcccgggcc ttgagccgac cgcggaatca cctggctggg aggcatcctc caggtaagct    1680
tgggagtatg tgtgcttagt gctgcaggct cctgcagaaa agtgcctata aacacccaac    1740
accctgctgc accctccacc tccaggcttt gtacactttc caaccgaaac tccaaaacgc    1800
tagcgtagag ggtggggagc cggccggaaa aagaataaaa gtccaataaa actggcgttc    1860
gctaaagttt atcaccagtc ctacatggga tatatatata tgtatttttt tttccgtgaa    1920
gggtgaaaag gagataagga agaaccaaca atctaccccc tcccgccgcc ccccaccccc    1980
gcgccacggt gatcagtttg gacttcaaag ccagagcaca ggctcttgcg ctttttcttg    2040
aaaccgaagt ctacactgaa agaaagtgtg cacttttgcc tagaaggcaa catgcgtttt    2100
cccgcgtgct aggtggagtg cattttaaca agacattagg gttttaacac atggctggag    2160
tggcgaccaa aagggaaaac tcagtttcca gtccaagcct cctagagaca ttcctgccaa    2220
cctccgcacc ctctcacgcc ccaccccacg tgtgagagtc tgcaaaacca ccggggattg    2280
gattcgatgg cgagcttcac gctcgggaac agtcagtaat cggaagggga agtggacagg    2340
ggaacttcaa gaggcgagcc tgccacgcgg gaagcgcccg aacttgcggg tctccatgaa    2400
tgcagagggc gccgggaagg gggggcatcc ggccgcgacc ctctctgccc ctcccattcg    2460
ctgccccccct ccccgctgga atttctctgt aaagcaagac ggagtagggg gaggggggaga    2520
gggaaggggc gagagggccc tcggctcact cccgagacgt gaggactcgc cacccaggca    2580
ttctcctcgg ggtgggctgg gccccgggac gaccacccgc ttcttcctcg cccccgctgc    2640
ccccacttcg ggagacccag agctctggat gcctttcccc ggagaagggg gggtgtgcgg    2700
agtcggggtg gaagagacct tgctcgcaga gctatatcaa gtgatgtcca gaggctggga    2760
gccccggcgg cctctgtccc ttgcctgtcg ggttagattt atactttaaa aatacctccc    2820
gccctccctc cttctctcgc ctctccccgc tgcaactttc tttgatccgc tcaaaggtgg    2880
cttaggtgaa attggagtaa ttcccttatg gggtcttaa aatgtaagtg aatgtcctta    2940
tccggggtga ctcaaaagct taagtcggga agcccaacgt gactaaaacc aataggtgat    3000
tgttcggggc cgactgtgtg cgggtgtaca cggtattcgg cccgggtgtc atccgcggcg    3060
ctggactgtt tcattttgag tttgcaactt gggtttttca gcgagctttt tttcttcctg    3120
aaagctaatg gcttccacag caattagaca ttttcctcgc ccgccccttc cctccccttt    3180
ctttacatat aggagatggg atactcattc ccgctgctat tgataaggtc ggaggcggcc    3240
gggcctctcc ccagctttcg cccgccccag cgcccgctct ccctccgccc tcccttggct    3300
tccttttgat gtagtgggga acgcgtccta ctaaaaaaaa aaaaaaaaaa aaaaaaaaaa    3360
agtaatctgc ccggtaacaa tcagcgcgca gtagcaggag ccccagagct attggctatg    3420
caaatagagg gaggggagac ggcgccccaa actcttcctc accctttttaa agcgatatcc    3480
cctcctttcc cccccaccac cccttccgcc ccaccctcgt ttaaagaggc tggctccggg    3540
gcctgagtta atcgcttgca cctctagttt attcgctccc ctcctccgcc ttgcagggaa    3600
cctagtgtac ggctcaccca gcccgcgccc caccccgcct tgctggctct ccgcgcccct    3660
gcccgggccc cctctctcgg tgagggaggc actcagtcgg cctcggtgtg cccagagagc    3720
tcgagccacg ccatgcccgc tgcacgtgcc agcttggcca gcacatcagg gcgctggtct    3780
ctccccttcc tcctggagtg aaatacacca aagggcgcgg tgggggtggg gggtgacggg    3840
aggaaggagg tgaagaaacg ccaccagatc gtatctcctg taaagacagc cttgactcaa    3900
gcatgcgtta gagcacgtgt cagggccgac cgtgctggcg gcgacttcac cgcagtcggc    3960
tcccagggag aaagcctggc gagtgaggcg cgaaaccgga ggggtcggcg aggatgcggg    4020
cgaaggaccg agcgtggagg cctcatgcct ccggggaaag gaaggggtgg tggtgtttgc    4080
gcagggggag cgagggggag ccggacctaa tccctcactc gccccctccc cctcccgggc    4140
catttcctag aaagctgcat cggtgtggcc acgctcagcg cagacacctc gggcggcttg    4200
tcagcagatg caggggcgag gaagcgggtt tttcctgcgt ggccgctggc cgcgggggaa    4260
ccgctgggag ccctgccccc ggcctgcggc ggccctagac gctgcaccgc gtcgccccac    4320
ggcgcccgaa gagcccccag aaacacgatg gtttctgctc gaggatcaca ttctatccct    4380
ccagagaagc acccccccttc cttcctaata cccacctctc cctccctctt cttcctctgc    4440
acacactctg cagggggggg cagaagggac gttgttctgg tccctttaat cggggctttc    4500
gaaacagctt cgaagttatc aggaacacag acttcaggga catgaccttt atctctgggt    4560
atgcgaggtt gctattttct aaaatcaccc cctcccttat ttttcactta agggacctat    4620
```

```
ttctaaattg tctgaggtca ccccatcttc agataatcta ccctacattc ctggatctta    4680
aatacaaggg caggaggatt aggatccgtt ttgaagaagc caaagttgga gggtcgtatt    4740
ttggcgtgct acacctacag aatgagtgaa attagagggc agaaatagga gtcggtagtt    4800
ttttgtgggt tgccctgtcc ggggcccctg gcatgcaggg ctggatggag ggagaggggt    4860
ggggggtggc gggggaccgc gtttgaagtt gggtcgggcc agctgctgtt ctccttaata    4920
acgagagggg aaaaggaggg agggagggag agattgaaag gaggagggga ggaccgggag    4980
gggaggaaag gggaggagga accagagcgg ggagcgcggg gagagggagg agagctaact    5040
gcccagccag cttgcgtcac cgcttcagag cggagaagag cgagcagggg agagcgagac    5100
cagttttaag gggaggaccg gtgcgagtga ggcagccccg aggctctgct cgcccaccac    5160
ccaatcctcg cctcccttct gctccacctt ctctctctgc cctcacctct cccccgaaaa    5220
ccccctattt agccaaagga aggaggtcag gggaacgctc tcccctcccc ttccaaaaaa    5280
caaaaacaga aaaacctttt tccaggccgg ggaaagcagg agggagaggg gccgccgggc    5340
tggccatgga gctgctgtgc cacgaggtgg acccggtccg cagggccgtg cgggaccgca    5400
acctgctccg agacgaccgc gtcctgcaga acctgctcac catcgaggag cgctaccttc    5460
cgcagtgctc ctacttcaag tgcgtgcaga aggacatcca accctacatg cgcagaatgg    5520
tggccacctg gatgctggag gtaggtcggg gggtggcgct cgccaggagc caggacccct    5580
ccggatgctc gggtccccgg ccggagccct aaacctggga gagggcaatc cccgcgccgg    5640
cctcccggct cctgtgcggg agtttaccgc gcgccttctg gcgagacgcg tggctttatt    5700
tctgttcctc tccagataaa ctggggaggc agaggggggga ggaaaatctg ggagaagcga    5760
ggctgtcctg ggcgggggta ggggagcatc ccgcgcgcgt gttcctgcat gtggctgcct    5820
cttcttccca cccccctcgc gacctgtctt ttgcgaagcc gccgcggctg cttgcgctgc    5880
ggccaggaag agagtcgggg cctgaaatcg ggaccccgag tagaaaggca acccccccca    5940
aaaggccaga gcaaattcgt cttggcctca ggtccccgcc tgtggtcgcg actccgcgct    6000
ggcacttcac cggggaggtg gagggaggag ggagaaggag agaaacgggg aattcgggag    6060
ccccggaagt cccattgaag aaacgcgtgt ttcaggggaa cccaaaagaa ccgcttcttg    6120
cccctcactc caagtctttg cccagcgagc cgtgtgccca cgtatgcaca gctctggacc    6180
tgccgtggtt tcgccatgtt gctttgcaaa ctccctttgg aaaggctggg aaacgtcgcc    6240
cgcttaccct cgcaccccat tatcccggcc accccacttc taatcgtgcc ctctccccca    6300
ccccccacct cacgaagatt acttacagtt tggttttcca gcttcctcgg tgcggagatt    6360
tgggtggggt aggaaggggg ggaggggtg tgtgtagggg ggataccggg tcacccctga    6420
aggagaggtg ggagcccccc gtgcctcttt ccagggctct ctgctcccgt ctttcccctc    6480
gctcgtcacc gcgttcccta gtttctgtcc ttgtgggccg cagccggaca ctcccgccgc    6540
ggcgctcacc ccttcaagtt tcctcccggg atccaaagcc ccaggggtcg gaatacagac    6600
ctttctggca cccgaaacct ctgtttcggg aagcctggct cttctttggg gttttcacgc    6660
caaaagggct tttctaggat atttcctcga tttttaatta ttttttgaaa ccatcccccc    6720
ctgctccgac tccagctgcc gaggctgcgg cgctcttcct cctctcctcc ctccccctct    6780
cttccccacc tctcctcacc cgcccccccca aaagcggccc ctgggccgca gggacccccc    6840
cagacatttt ttccaattgt cgggaatgat agaggaggat ctggggatcc ggatgagacc    6900
aagaagtgga cccccggagc ctccagaata tttttattga ttttttgaaa agatgacgaa    6960
atccaaaaaa gagtgagtga gttagagtgc gcgaaggagt agccaaaggg agcgtcgcgg    7020
agctgccgcg gctgctgcgc acttctccga ctccccgttt tggagctgta gttcaccccc    7080
ttttatagga tccctgggaa taccaaagca ctgatgggct attctgattc actccagttt    7140
cctcatcttt gttctttatt cttatcacgc attctggtcc cctccccctc ccacaaaaaa    7200
aaattaattt tttttgtttc gatagattac gctttttttat tctttttctc ttttgctgat    7260
gctatgctct ccaccccccgc cccccaaccc tttcccactc ccattatagg tctgtgagga    7320
acagaagtgc gaagaagagg tcttccctct ggccatgaat tacctggacc gtttcttggc    7380
tggggtcccg actccgaagt cccatctgca actcctgggt gctgtctgca tgttcctggc    7440
ctccaaactc aaagagacca gcccgctgac cgcggagaag ctgtgcattt acaccgacaa    7500
ctccatcaag cctcaggagc tgctggtaat gaccggcccc ttcctccctt cctttctgcg    7560
attcccgctt tc                                                        7572
```

<210> 2
<211> 3491
<212> DNA
<213> Homo Sapiens

<400> 2

```
gctggtctcg aactcccgac ctcaggtgat tccccccgcc ttgatctccc aaagtgaagg      60
gattacaagg cgtgaggcac cgcgcccggc cgcttctgaa taatttcgat caaaatttat     120
attcgatatt tattccaaca tacaccacag atttccactg ataatccctc ctagtaagaa     180
```

```
agataagctc catccaggta tctgtgaatt ggaggctaag tagtcccagc acatcttaca     240
tttctttaag actccctttt tatcccaaac gttcgtaaat tttgtatctg ataaagagca     300
tacttccatc taatacaaat atgttccccc cttcagatct tctcagcatt cgagagatct     360
gtacgcgcgt ggctcctcat tcctcttcct tggcttccca agcccccagg gcgtcgccag     420
gaggaggtct gtgattacaa accccttctg aaaactcccc aggaagcctc cccttttttcc    480
ggagaatcga agcgctacct gattccaatt cccctgcaaa cttcgtcctc cagagtcgcc     540
cgccatcccc tgctcccgct gcagaccctc tacccacctg gatcggcctc cgaccgtaac     600
tattcggtgc gttgggcagc gcccccgcct ccagcagcgc ccgcacctcc tctacccgac     660
cccgggccgc ggccgtggcc agccagtcag ccgaaggctc catgctgctc cccgccgccg     720
gctccatgct gctccccgcc gcccgctgcc tgctctcccc ctctccgcag ccgccgagcg     780
cacgcggtcc gccccaccct ctggtgacca gccagcccct cctctttctt cctccggtgc     840
tggcggaaga gccccctccg accctgtccc tcaaatcctc tggagggacc gcggtatctt     900
tccaggcaag gggacgccgt gagcgagtgc tcggaggagg tgctattaac tccgagcact     960
tagcgaatgt ggcacccctg aagtcgcccc aggttgggtc tcccccgggg gcaccagccg    1020
gaagcagccc tcgccagagc cagcgttggc aaggaaggag gactgggctc ctccccacct    1080
gcccccccaca ccgccctccg gcctccctgc tcccagccgc gctcccccgc ctgccagcaa   1140
aggcgtgttt gagtgcgttc actctgttaa aaagaaatcc gcccccgccc cgtttccttc    1200
ctccgcgata caaccttcct aactgccaaa ttgaatcggg gtgtttggtg tcatagggaa    1260
agtatggctt cttcttttaa tcataagaaa aagcaaaact attctttcct agttgtgaga    1320
gccccaccga gaatcgaaat cacctgtacg actagaaagt gtccccctac cccctcaacc    1380
cttgattttc aggagcgcgg ggttcactaa gtcagaaacc ctagttcaaa ggattccttt    1440
tggagagtcg gactgctctc tccttcccct ccccttcccc tcctgcgtgt aaaacggctg    1500
tctggggcaa gggtttctca gacgtgtaca ttgcctggta taagagcaga ctctgaaaag    1560
atgaggttta tttaatacgg acgggggaga attctgcctg taggcagata ggaaaatggg    1620
gagggagtca ttggaaggac ggactccatt ctcaaagtca taattcctag accagaaaaa    1680
gtgctcagtg ttctagaagc agagttgcac agtgatccaa agaccagctt caaatactgt    1740
cctgtctcct tcacacttct cacatttctc tttcctactg aaaatacctt gcatttttcg    1800
taattataaa gggggaaggg aatatgagtg ccccctgctt tataggggtt gttgtgagtt    1860
taaatgatgt attaatacat ataagcctta agaacagtgc cacacatcct aagctaatac    1920
ctgttagctc ttgaattatc cgctttgagg actggcttgc aatcttgttt tgaggcatag    1980
aaagaaaatg ctttggagca ggacgcggtg gctcacacct gtaatcccag cactttggga    2040
agccgaggcg ggcagatcac ctgaggtcag gagttcgagg ccagcttggc caaaatggtg    2100
acaccccgtc tctactaaaa atacaaaaat tagctggcca tggtggcgca cgtgtgtaat    2160
tccagctact caggaggctg aggcaggaga atcgcttgaa cccgggaggc agaggttgca    2220
gtaagccgag atcgcgccac caccctccag cctgggtgac agaatgagac tccgactcaa    2280
aaaaaaaaaa aaaaatgctt tggatagaat tatcactatt acataaaagg aaagtccgga    2340
tgcggtggct cacgtctata atcccagcat tctgggaggc cgagacaggc ggatcacctg    2400
aggccaggag ttcgagacaa gcctgaccaa catggcgaaa ccctgtctct actaaaaaat    2460
acaaaattag cggggcttgg tggcgcatgc ctgtaatccc agctactcgg aggctgatgt    2520
aggagaatcg cttgaaccca ggagaaggcg gaggttgcag tgagccgaga tcgcgccatt    2580
gcactccagc ctgggagaca agagcgaaac ttggtctcaa gaaaaaaaga aagaaagaaa    2640
gaaagaaaga ccaagaagaa cttactccct gaaaagatta tgggcaccct ccaccacccct   2700
cacttacaaa gaaaagttaa acagcactaa agagtataac aagcgcaagg aggtaaaagt    2760
tctaattttt cctgtgacta ctacttttta agcttatcaa aaacatgtac tacgttttaa    2820
aaaatggatt gctcagactt tgctgatgcc ttaagcacat gcttaatctg cctactggat    2880
aatccagctc tgtttaaaag ttatatttca atccctggtt gacttaaacc ttgtagaccc    2940
agtatatctt gtacttttag tgtctgcttg attttaaaac atgtagtttt taaaatgaag    3000
ccaatgaaaa caatttggga tgtcaagtat gttattaaaa tctacaatgc attactgtac    3060
catttatatt ttcctcgggg tacctctcaa ttagctgtgt agcaatgata gggaaaattc    3120
aaactatcga taaataaaat tattttagtt tagtttaaga tattttatga tggaggagga    3180
agaaagtggt tgccaggatg ggagggaggg aacacatttc catcactaat acaatggttc    3240
tttctttttg tttgtttgtt ttgtgtgttt ttttgagatg gagtttcgct ctgttgccca    3300
ggctggagtg aaatggcacc gcacgatctc tctcggttca ctgcaacctc cgcctcccgg    3360
gttcgagcaa ttctcctgcc tcagcctccc gagtagctgg gattacaggc acatgctgcc    3420
atacccagct aatttttgta ttattagtag agacggggtt tcactatgtt ggctaggctt    3480
gtctcgaact c                                                         3491
```

<210> 3
<211> 1145
<212> DNA
<213> Homo Sapiens

<400> 3

```
gggcacggag gcactgcgcc acccagggca agacctcgcc ctctctccag ctcctctccc      60
aggatatcca acatcctgtg aaacccagag atcttgctcc agccggattc agagaaattt     120
agcgggaaag gagaggccaa aggctgaacc caatggtgca aggttttacg gttcggtcat     180
cctctgtcct gacgccgcgg ggccagcggg agaagaaagc cagtgcgtct ctgggcgcag     240
gggccagtgg ggctcggagg cacaggcacc ccgcgacact ccaggttccc cgacccacgt     300
ccctggcagc cccgattatt tacagcctca gcagagcacg gggcgggggc agaggggccc     360
gcccgggagg gctgctactt cttaaaacct ctgcgggctg cttagtcaca gcccccccttg    420
cttgggtgtg tccttcgctc gctccctccc tccgtcttag gtcactgttt tcaacctcga     480
ataaaaactg cagccaactt ccgaggcagc ctcattgccc agcggacccc agcctctgcc     540
aggttcggtc cgccatcctc gtcccgtcct ccgccggccc ctgccccgcg cccagggatc     600
ctccagctcc tttcgcccgc gccctccgtt cgctccggac accatggaca agttttggtg     660
gcacgcagcc tggggactct gcctcgtgcc gctgagcctg gcgcagatcg gtgagtgccc     720
gccgcagcct gggcagcaag atgggtgcgg ggtgctcagc gcggacccgg cggcagcccc     780
tccggctgag tcggccctgg gggactggag tcaagtgagc tgtctgcgaa gtgcattggg     840
ctccggaaag cagggctggg atttgcgcta aaccgttgga gaatgtgtct gtggaagcac     900
catttggttg aaagaaaaag agaaagagaa gaaagtttgt tgggcaggct gccggcgcgc     960
agttttgggc gaggtcgcta gagctgcagc acatggcaga aagtaaccgt tctcccggat    1020
gcgcacagtc gttgtctgga ctaacaggct cctgtgccca agggctcgcc aagccccacc    1080
gggctgtgtc taggcagggc agagctgggc ggggcagaga ctggggctgg aacagggcga    1140
gtggc                                                                1145
```

<210> 4
<211> 2221
<212> DNA
<213> Homo Sapiens

<400> 4

```
caatgacgat gtgcagcagg tctcccagag ccaagctggc gatcaagata ttgggaccgt      60
ttcgcatgca cttgttcttg tagataattc tcagaagtgt ggagttcccg atgatcccca     120
gcacgaacac aaggcaggac acaaccgtgt tgatgtattt gaaagtctcc ttgatctcga     180
tgggtccttg gcacgggggga ggggagatgg tgcgtggcgg agatcctgcc gtcctgtctc    240
ctttaggcac ctccgcaggt gccaacgacc gcgccagact ggcgttggaa cccttgggcc     300
ataaggtctt agtgggtggc gtcattatct ctgcggtttg caaaagcgga gtggccctgt     360
caggcgggaa gcctctctcc tctccccaga tccgcgacag gccgcaggca agaaccagcg     420
caaccagggc gcgtccgcac agacttggag gcggctgcat gctgctacct gctccagaag     480
gcgtccggtg gccgctccgc agtttcagag cctagagaca agcagaggaa ggaagacagg     540
acacttgggt cagctgcccg agccaagtcg ctgcaaacgc taataccgcc cgcagcctct     600
tcgccagtat ccacgctcaa aagtaactca agtttgcgcg ccagtgggaa acttggcgct     660
catgactcgc cagcgcgggt cgaaactcct tcctgatgcc ctctcagctg tttttcttcc     720
cccgcgtggc caggaggggt aaataatatg cagtggggcg gggcgttctg gaaggggtgt     780
gccagggagg gaatgatggg ggtcccaggc aaagctccga actctttcac gtaacggggag    840
gaatacagac acgtcttagt taagcgtgcg tgggaaccgc ggaattaagg cacctagagg     900
ccaagggctt gtgtcaagct ctgcattttg ccccgcaggg gaacctctat accccgcgat     960
tgaactcgaa agactcctcc cggcggatga aagccgctac tccctggctg gctgagctac    1020
agctcccgca gcgcgcccag gagtgcgccg gagattcgga aacccgcaga gacttctcaa    1080
gtcagcagga acttggaaac cgctgttccc tccaaacggc ttcaaacact cgcgctcctt    1140
cctttaccca acctaatttt aatttgccct ctctataggc ttaattttta ttttttttaaa   1200
atcccaaact agtaagccaa accgaagccc cagaataagg ttcaatcaat gattttaatt    1260
caacaccaag cccgaatgtt tacctctcgg atctgacaaa ccagatgcag agcgacgaat    1320
ggagaccacc ttcccgggag gcggaaccca gctgggttcc agcctgctct gggagaggag    1380
cacgcctccc ttgagctgca ggcgggtccg gctctgacga aacgctgcga gaatgccaga    1440
cagtgtagcc tccaccgttt gctcggggtc tctgctgcca tcagacaagt acttttattc    1500
attcatccct tcccatcaat caccgccaga ctcctcccgc gccttccgac cccgctgcaa    1560
cctttcccct tgggcgatgc ctggacagca tcagtagtag ttgcccgagg gagggggggca   1620
gtcctcgtcc ggggacaggt cccaaaaggc tgtttatttg ttagagttct acgctcttca    1680
aatgaaccca aatcaagggc aagttatcac gcaccatgtg acacccggca gctccagact    1740
agaacaaggc tctgcactga acgtgataat tgaactttta atgccaagtg tcacttttct    1800
```

```
ttcaggtaaa tgttccgccg ctgtgatatt taaggttggt ttgtgggcgc tgctctttgc    1860
tctgcttcat tttgccctga agtttcattt caagccaaca ttaaccccaa cacggaacag    1920
ctcgatcttt tcttcccagg ccctctcact gccccgagag aggattacaa gtactcctca    1980
ttttccccag tacagctctt ctcctctctt cctgccacag tatcggtgtt aggcaatctc    2040
aggcattttt atactgtacc tatgacagac ttgatgctta tcggagacat gaaaataatc    2100
agaaagctgc catgcaaatt caacatgata tttatttacc aggtagagca atacaataag    2160
gtctttctat ccattcttga gctccatgtt taccgcagag aggaaaataa cagcgactgg    2220
t                                                                    2221
```

<210> 5
<211> 2172
<212> DNA
<213> Homo Sapiens

<400> 5

```
atacggcagg tgctcaataa acgctagctc ctattggcat tactatcatc actggcgcta      60
atgtattcac ttattgtagt gaggcagaac gtctttgtgg ttacgagtgt gaccaggctg     120
acccactatt aagaggtgga ggacagggaa ggccgtacag tgctccatca caaggaaaag     180
aggatcagca cgttctcttc tgggaaggga aaaaaaaaga gaaggaaatt gaaagtacac     240
ggccgatctt cccccaatta caaagcactg gactcacggg agggtatgcg ggggactgtg     300
caagcataca ggggtgtggg ggaggaaggc caatgaacag atccactgca aagggagggg     360
tgatgaggct catcgtctga ggggaagggg agcagaaaac cttacagact cctgcaccac     420
agtgagagga gagaaaagcc atcacacaga ctgattcaaa gcagagaggg aaaaggctat     480
ggactgagct actgaaaata gggagatgta aagcactaaa ctaacccact attaaaaagg     540
agagaacccc aagcgctaga gcgacagtct acgaaaaaga ggagacaggg caatggactg     600
atagctaaag ggacgtgaat ccaaacatga agactgatcc actataaaat ggtggtggtg     660
gtggtggggg ggggggtgg ttttgaaaaa agccatggac tgatgcattg gggactgga      720
ggaggcagga atacaaactc aggtagactc actgttaaag agggatgcgg aggagtgcgg     780
cgccacggat tgattcgcta cgatgtacag gaagggtgga ataaataagg ccacggacgg     840
gtctcctgaa agttgaggct aggagaaatc caaacactca gatacaccta ctattaaggg     900
ggctgaacac tgtcacaggc tagctcccta taagtggggg tgggtggggg gcgggtgggg     960
gtaggggtag ggggaaagcc tatggacgag tccaccctaa gttggcgtgg ggaaaaacat    1020
aagcacgcag accgatccat tatacaacgg gagggggagg ttaggccacg gtcagatcca    1080
ctataaggga cgaagggctt gagcaatgcc acacgcaggt ccaccagccg tggtggtggc    1140
ggcggggagt cgaacaccag caggccaccg gccaagccac atcctctgcg cgggggcggg    1200
aaggatacgt ggggtcacgg actgggccct ccaccctcca ggcccagagc tcacctgtgt    1260
gtagcgtggc ggtggcgttg gcaatgttgg cagctccggg gggcggggc tccatacgcg     1320
gccccaccgc cccccaggcc tgggttccga ggcggcggtg gaggtggtgg tggcggtggc    1380
ggcggcagca cctagaagcc gcccctgcgt ttccctacag ctcacgtggg cccgaggagg    1440
aggagcccaa tgtcccgccg ctcgctgatt ggccaaagcg ctattaatcg gccgcaaggc    1500
ctcgaggggt tgggggacgg gcccttccct acagagctgt gccgtgattg gcgtaagggg    1560
aaatgatgga acgctcacgg cccctggagt cccgaggagg cgcgttcggc cttcacggtg    1620
ctccacgccg attggctcaa ggactgacgg actgtgagca actgaaaagg ctcggggcct    1680
gtgtcggggg ggcgggggtc tgcctcacac tgattggctt acatgggatt gatggaagac    1740
agcccccaag gacgggggtg ggtggccctg tttttctctg attggcggac gagggacttc    1800
tagacccctg ccaaaacacc aagggggcgg tgtcgcacgc catgctgctc agcggaagca    1860
gggcttgtat agaaatgggc gtagcagccg gcgggagtgg gtcggactgg gctccgcgta    1920
ggcgggcagg gcagctctac ggttagacaa gaccatagag ctgggtaaag acgaatttag    1980
aacacagcgg aggtaggagg gcaggatggc tgtcaggcac acgaaagagc attgagtggc    2040
agaaacgaaa tgctttcaga gggcagggtt gaactgcggc cagagttaaa aaggggagga    2100
acctggctct gcattgattg gcggtggctg gactcaacct agaatagggg cccgactagg    2160
gagagcggga tc                                                        2172
```

<210> 6
<211> 3289
<212> DNA
<213> Homo Sapiens

<400> 6

```
tctgtgttta cgtattatgt gtgtttttcc gtttccctcc ctttttatga gtgagaaaaa      60
aaagcgccta aattcccacc aacataaacc aatgacatac aatgatgaaa ttctgttttc     120
acctctgcct gtgacaggga atgcaaaaat agcaagtggc ccagttccac gaatccccgc     180
ctcctgccct ccccgctcct gccgggttgg atctctaaga atggaggtta gcgcacagcc     240
tcgcgcgggc cgctcagccc ccggacccgg cggatgatgt caggcgacgg gagcggccgc     300
ggccgggccg gggaggccgc ggcccagggg agctgggagg gagggtggcc tcgccaggcc     360
gacggcgcgc ccggccgcgc ggcgttgcct ggagacggcc ctggcggcgc tgtgttgctg     420
taaacagccg cttccctgtt actatctata gcaggatctc ctggctcccg ggcgcggcgg     480
ctggaggcag gtctgcggtc cggctccccg cgcgccccga actatccgcc cgccggcctc     540
atcctgcctc gcccctctcc aggtgcccac cgcgggcccc gacccccggg cccgaagagt     600
ggagaaggga agaccggggc tgtgcgggga catgcgtctt cgcgccctgg aggtggccag     660
cgcgctgggg ctgagccccg gcagcgtgac cccggctgtc ctacgcagca gggcaggaga     720
ttggggggcg tggcacactc tggagcacct tgcctcccca aagccccgtg ttccaggacg     780
tggagccgct cctggggtcc cagcagtcga ggtattccgc ccaggcgcag ctggacactg     840
tccttccagc cccgtcctc caccctccaa gtccgcgctg gaaaatcacc cgctgcgggc      900
tcccgtaagc acagcttcct ggcgggaccg aaccagccct cagcgcagat ttgagttccc     960
cgcaggaagc acaccccgcc ttgtcatccc gaactgacca ccctgcccac ataaccacac    1020
ctcgcactcc ctacccctgg ggcccagctc agaaccgggc agacaccccc ttcaaatgtc    1080
ttcgcacgta ggttttgcac agtgtttatc tgctggtgtc tcagggattt gacagtttcc    1140
ttaatattcc cacacatggc cgagaaaaat aaataaataa atgcgctgtc ttctttaaaa    1200
aaataaataa ataaagtacc cagtatcgta aagtaggtta tcgtattctc ttattttgga    1260
tcctccactt tctgcttcca aacgcaggaa cagtgctagt attgctcgag cccgagggct    1320
ggaggttagg ggatgaaggt ctgcttccac gctttgcact gaattagggc tagaattggg    1380
gatgggggta ggggcgcatt ccttcgggag ccgaggctta agtcctcggg gtcctgtact    1440
cgatgccgtt tctcctatct ctgagcctca gaactgtctt cagtttccgt acaagggtaa    1500
aaaggcgctc tctgccccat cccccccgac ctcgggaaca agggtccgca ttgaaccagg    1560
tgcgaatgtt ctctctcatt ctgcgccgtt cccgcctccc ctcccccagc cgcggccccc    1620
gcctcccccc gcactgcacc ctcggtgttg gctgcagccc gcgagcagtt cccgtcaatc    1680
cctcccccct tacacaggat gtccatatta ggacatctgc gtcagcaggt ttccacggcc    1740
tttccctgta gccctggggg gagccatccc cgaaacccct catcttgggg ggcccacgag    1800
acctctgaga caggaactgc gaaatgctca cgagattagg acacgcgcca aggcgggggc    1860
agggagctgc gagcgctggg gacgcagccg ggcggccgca gaagcgccca ggcccgcgcg    1920
ccacccctct ggcgccaccg tggttgagcc cgtgacgttt acactcattc ataaaacgct    1980
tgttataaaa gcagtggctg cggcgcctcg tactccaacc gcatctgcag cgagcatctg    2040
agaagccaag actgagccgg cggccgcggc gcagcgaacg agcagtgacc gtgctcctac    2100
ccagctctgc tccacagcgc ccacctgtct ccgcccctcg gcccctcgcc cggctttgcc    2160
taaccgccac gatgatgttc tcgggcttca acgcagacta cgaggcgtca tcctcccgct    2220
gcagcagcgc gtccccggcc ggggatagcc tctcttacta ccactcaccc gcagactcct    2280
tctccagcat gggctcgcct gtcaacgcgc aggtaaggct ggcttcccgt cgccgcgggg    2340
ccgggggctt ggggtcgcgg aggaggagac accgggcggg acgctccagt agatgagtag    2400
ggggctccct tgtgcctgga gggaggctgc cgtggccgga gcggtgccgg ctcgggggct    2460
cgggacttgc tctgagcgca cgcacgcttg ccatagtaag aattggttcc cccttcggga    2520
ggcaggttcg ttctgagcaa cctctggtct gcactccagg acggatctct gacattagct    2580
ggagcagacg tgtcccaagc acaaactcgc taactagagc ctggcttctc cggggaggtg    2640

gcagaaagcg gcaatccccc ctcccccggc agcctggagc acggaggagg gatgagggag    2700
gagggtgcag cgggcgggtg tgtaaggcag tttcattgat aaaaagcgag ttcattctgg    2760
agactccgga gcggcgcctg cgtcagcgca gacgtcaggg atatttataa caaacccccct   2820
ttcaagcaag tgatgctgaa gggataacgg gaacgcagcg gcaggatgga agagacaggc    2880
actgcgctgc ggaatgcctg ggaggaaaag ggggagacct ttcatccagg atgagggaca    2940
tttaagatga aatgtccgtg gcaggatcgt ttctcttcac tgctgcatgc ggcactggga    3000
actcgcccca cctgtgtccg gaacctgctc gctcacgtcg gctttcccct tctgttttgt    3060
tctaggactt ctgcacggac ctggccgtct ccagtgccaa cttcattccc acggtcactg    3120
ccatctcgac cagtccggac ctgcagtggc tggtgcagcc cgccctcgtc tcctccgtgg    3180
ccccatcgca gaccagagcc cctcaccctt tcggagtccc cgccccctcc gctggggctt    3240
actccagggc tggcgttgtg aagaccatga caggaggccg agcgcagag                3289
```

<210> 7
<211> 3812
<212> DNA

<213> Homo Sapiens

<400> 7

```
agcgagaccg catggtctca cttataagtg ggagctgaac aatgagaaca catggtcaca      60
tggcggcgat caacacacac tggtgcctgt tgagcggggt gctggggagg gagagtacca     120
ggaagaatag ctaagggata ctgggcttaa tacctgggtg atgggatgat ctgtacagca     180
aaccatcatg gcgcacacac ctatgtaaca aacctgcaca tcctctacat gtaccccaga     240
acttcaaata aaagttggac ggccaggcgt ggtggctcac gcctgtaatc ccagcacttt     300
gggaagccga ggcgtgcaga tcacctaagg tcaggagttc gagaccagcc cggccaacat     360
ggtgaaaccc cgtctctact aaaaatacaa aaatcagcca gatgtggcac gcacctataa     420
ttccacctac tcgggaggct gaagcagaat tgcttgaacc cgagaggcgg aggttgcagt     480
gagccgccga gatcgcgcca ctgcactcca gcctgggcca cagcgtgaga ctacgtcata     540
aaataaaata aaataacaca aaataaaata aaataaaata aaataaaata aaataaaata     600
aaataaaata aaataaaaaa ataaaataaa ataaaataaa ataaagcaat ttcctttcct     660
ctaagcggcc tccacccctc tcccctgccc tgtgaagcgg gtgtgcaagc tccgggatcg     720

cagcggtctt agggaatttc cccccgcgat gtcccggcgc gccagttcgc tgcgcacact     780
tcgctgcggt cctcttcctg ctgtctgttt actccctagg ccccgctggg gacctgggaa     840
agagggaaag gcttccccgg ccagctgcgc ggcgactccg gggactccag ggcgcccctc     900
tgcggccgac gcccggggtg cagcggccgc cggggctggg gccggcggga gtccgcggga     960
ccctccagaa gagcggccgg cgccgtgact cagcactggg gcggagcggg gcgggaccac    1020
ccttataagg ctcggaggcc gcgaggcctt cgctggagtt tcgccgccgc agtcttcgcc    1080
accagtgagt acgcgcggcc cgcgtccccg gggatggggc tcagagctcc cagcatgggg    1140
ccaacccgca gcatcaggcc cgggctcccg gcagggctcc tcgcccacct cgagacccgg    1200
gacgggggcc taggggaccc aggacgtccc cagtgccgtt agcggctttc agggggcccg    1260
gagcgcctcg gggagggatg ggaccccggg ggcggggagg gggggcagac tgcgctcacc    1320
gcgccttggc atcctccccc gggctccagc aaacttttct ttgttcgctg cagtgccgcc    1380
ctacaccgtg gtctatttcc cagttcgagg taggagcatg tgtctggcag ggaagggagg    1440
caggggctgg ggctgcagcc cacagcccct cgcccacccg gagagatccg aaccccctta    1500
tccctccgtc gtgtggcttt taccccgggc ctccttcctg ttccccgcct ctcccgccat    1560
gcctgctccc cgccccagtg ttgtgtgaaa tcttcggagg aacctgtttc cctgttccct    1620
ccctgcactc ctgacccctc cccgggttgc tgcgaggcgg agtcggcccg gtccccacat    1680
ctcgtacttc tccctccccg caggccgctg cgcggccctg cgcatgctgc tggcagatca    1740
gggccagagc tggaaggagg aggtggtgac cgtggagacg tggcaggagg gctcactcaa    1800
agcctcctgc gtaagtgacc atgcccgggc aaggggaggg ggtgctgggc cttaggggggc    1860
tgtgactagg atcgggggac gcccaagctc agtgcccctc cctgagccat gcctccccca    1920
acagctatac gggcagctcc ccaagttcca ggacggagac ctcaccctgt accagtccaa    1980
taccatcctg cgtcacctgg gccgcaccct tggtgagtct tgaacctcca agtccagggc    2040
aggcatgggc aagcctctgc ccccggagcc cttttgttta aatcagctgc cccgcagccc    2100
tctggagtgg aggaaactga gacccactga ggttacgtag tttgcccaag gtcaagcctg    2160
ggtgcctgca atccttgccc tgtgccaggc tgcctcccag gtgtcaggtg agctctgagc    2220
acctgctgtg tggcagtctc tcatccttcc acgcacatcc tcttcccctc ctcccaggct    2280
ggggctcaca gacagccccc tggttggccc atccccagtg actgtgtgtt gatcaggcgc    2340
ccagtcacgc ggcctgctcc cctccaccca accccagggc tctatgggaa ggaccagcag    2400
gaggcagccc tggtggacat ggtgaatgac ggcgtggagg acctccgctg caaatacatc    2460
tccctcatct acaccaacta tgtgagcatc tgcaccaggg ttgggcactg gggctgaac     2520
aaagaaaggg gcttcttgtg ccctcacccc ccttacccct caggtggctt gggctgaccc    2580
cttcttgggt cagggtgcag ggctgggtc agctctgggc caggggccca ggggcctggg     2640
acaagacaca acctgcaccc ttattgcctg ggacatcaac cagccaagta acgggtcatg    2700
ggggcgagtg caaggacaga gacctccagc aactggtggt ttctgatctc ctggggtggc    2760
gagggcttcc tggagtagcc agaggtggag gaggatttgt cgccagtttc tggatggagg    2820
tgctggcact tttagctgag gaaaatatgc agacacagag cacatttggg gacctgggac    2880
cagttcagca gaggcagcgt gtgtgcgcgt gcgtgtgcat gtgtgtgcgt gtgtgtgtgt    2940
acgcttgcat ttgtgtcggg tgggtaagga gatagagatg ggcgggcagt aggcccaggt    3000
cccgaaggcc ttgaacccac tggtttggag tctcctaagg gcaatggggg ccattgagaa    3060
gtctgaacag ggctgtgtct gaatgtgagg tctagaagga tcctccagag aagccagctc    3120
taaagctttt gcaatcatct ggtgagagaa cccagcaagg atggacaggc agaatggaat    3180
agagatgagt tggcagctga agtggacagg atttggtact agcctggttg tggggagcaa    3240
gcagaggaga atctgggact ctggtgtctg gcctggggca gacgggggtg tctcaggggc    3300
tgggagggat gagagtagga tgatacatgg tggtgtctgg caggaggcgg gcaaggatga    3360
```

```
ctatgtgaag gcactgcccg ggcaactgaa gccttttgag accctgctgt cccagaacca    3420
gggaggcaag accttcattg tgggagacca ggtgagcatc tggccccatg ctgttccttc    3480
ctcgccaccc tctgcttcca gatggacaca ggtgtgagcc atttgtttag caaagcagag    3540
cagacctagg ggatgggctt aggccctctg cccccaattc ctccagcctg ctcccgctgg    3600
ctgagtccct ggccccccctg ccctgcagat ctccttcgct gactacaacc tgctggactt    3660
gctgctgatc catgaggtcc tagcccctgg ctgcctggat gcgttccccc tgctctcagc    3720
atatgtgggg cgcctcagtg cccggcccaa gctcaaggcc ttcctggcct cccctgagta    3780
cgtgaacctc cccatcaatg gcaacgggaa ac                                  3812
```

<210> 8
<211> 1267
<212> DNA
<213> Homo Sapiens

<400> 8

```
atgaatctcg aaagctggat tgcagagcaa acgagtgcag tcaattcagc caggggcttg      60
caagagggag aaagagaaaa agactgtgga atggaaagtt tcccaaccca agcctttccc     120
aaggggtagc cattctctgt tctacagttt agggcttgca tgtgcttttt ctggagtgga     180
aaaatacata agttataagg aatttaacag acagaaaggc gcacagagga atttaaagtg     240
tgggctgggg ggcgaggcgg tgggcgggag gcgagcgggc gcaggcggaa caccgttttc     300
caagctaagc cgccgcaaat aaaaaggcgt aaagggagag aagttggtgc tcaacgtgag     360
ccaggagcag cgtcccggct cctcccctgc tcattttaaa agcacttctt gtattgtttt     420
taaggtgaga aataggaaag aaaacgccgg cttgtgcgct cgctgcctgc ctctctggct     480
gtctgctttt gcagggctgc tgggagtttt taagctctgt gagaatcctg ggagttggtg     540
atgtcagact agttgggtca tttgaaggtt agcagcccgg gtagggttca ccgaaagttc     600
actcgcatat attaggcaat tcaatctttc attctgtgtg acagaagtag taggaagtga     660
gctgttcaga ggcaggaggg tctattcttt gccaaagggg ggaccagaat tcccccatgc     720
gagctgtttg aggactggga tgccgagaac gcgagcgatc cgagcagggt ttgtctgggc     780
accgtcgggg taggatccgg aacgcattcg gaaggctttt tgcaagcatt tacttggaag     840
gagaacttgg gatctttctg ggaacccccc gccccggctg gattggccga gcaagcctgg     900
aaaatggtaa atgatcattt ggatcaatta caggctttta gctggcttgt ctgtcataat     960
tcatgattcg ggctgggaa aaagaccaac agcctacgtg ccaaaaaagg ggcagagttt    1020
gatggagttg ggtggacttt tctatgccat ttgcctccac acctagagga taagcacttt    1080
tgcagacatt cagtgcaagg gagatcatgt ttgactgtat ggatgttctg tcagtgagtc    1140
ctgggcaaat cctggatttc tacactgcga gtccgtcttc ctgcatgctc caggagaaag    1200
ctctcaaagc atgcttcagt ggattgaccc aaaccgaatg gcagcatcgg cacactgctc    1260
aatgtag                                                             1267
```

<210> 9
<211> 1266
<212> DNA
<213> Homo Sapiens

<400> 9

```
agtcacgtag cttctctatt cggagagaag tcggagtact gggatagacc caggaggtca      60
gagcggaaac tctgcccggg tgcgtggaac cggagtcccc ggtgcgcggc gccaggtact     120
cacctgtatg gctgagcgcc aggaccgcgc acagcagcag ggcgcgggcg agcatcgcag     180
cggcgggcag ggcgcggcgc gggggtaggc tttgctgtct gagggcgtct ggctgtggag     240
ctgaaggagg cgctgctgag gagttcctgg acgtgctcct gacgctcact gcaagtcgta     300
tgacaattgg tcgctaaccg agagaacctt ccttttata agactgaaaa ccaagcccat      360
gtgacgaaat gactgtttct ttccgccttt tcgtaccccc cacaaatttt tccctcctct     420
cccttaaaa aaattgcgta agcccggtgg gggcagggtt ttttacccac ggaaatgaga      480
aaatcggaaa cccaggaagc tgccccaatt tgggagcaga gggggtagtc cccactctcc     540
tgtctgatcc ctccctctcc tccccgagtt ccaccgcccc aggcgcacag gtttccgcca     600
gatgtctttt cttcttcgca gtctttgccc gagcgcttcc gagagccagt tctggactga     660
tcgccttgga tgggataccg gggggagggca gaaggacact tggcttcctc tccaggaatc    720
tgagcggccc tgaggtccgg gggcgcaggg aatcccctct cccgccgccg ccgccgtgtc     780
tggtctgtac gtctttagag ggtcgaggaa gtcacgtcgg gacagactgg ggcgagtaag     840
gttaagaaag gctgacatgt tttatgtttt agtgacgacg cttaataggc tgtatatctg     900
```

ctctatatgc agcacataca tacatagctt tttaaaaaac tcttattttg tggaatgaaa 960
tagctacctt cagtgtacat agctgtaatt tatctttgta gctaagttgc tttcaacaga 1020
agaaatactg ttctccgtac cttcaccccc tccttgtttc ttggaaagag aggcgggaaa 1080
ggtaaattct cctcataata ctggtcctaa gcagttaccc tgtaaatagt taatgtgagc 1140
tccacgggtc accaatataa agtttcctgc cttctgatgg acaaaggaag cggcgatggc 1200
cagaatttgc agggacgcta aatgtccaaa acgtatgcct taaggcattt ctctccctga 1260
tgcgtg 1266

<210> 10
<211> 4511
<212> DNA
<213> Homo Sapiens

<400> 10

ggagcattcg aacgcggaaa tcgaggtgct agtccaaact gctcggtcgg ctttagtcat 60
agctggataa tgcccggctc aggtctacca caagccatac agctgctttt tccgtgttca 120
acctgtctgt gacagaaacc aagggggccc cggcacccag catctaggcg gtggaatcgg 180
ggtcttacgc acggttccgc gggcaggtcc ccggccagga cccgcgggga gccacgtagc 240
caggagggtg gggctgccca ccgacccagg acgcggcaac ggaccgggga gggcggagct 300
ccagcgaccg cttcccctcc cgcccgccgg caccccctgg ctcccacctg gtcccggcgc 360
ggcctgcgag ctagcgaggt tcgcgcggtg aagtactgct cgagctccga gtccgagtcc 420
tcttggctgc agtagccact gctcgtcgtg ctgctccagg tcatttcgaa agaaggcgcc 480
tccgcctcgc ccatagccgt acccgcccgt cccccagtcc tgcgcgtccg tagccgccaa 540
ccaccgcccc ggtcgcgtgc gtgcgtgtac gcgtgtcagt gtgcgcgtgc gcccgggcca 600
gagccgcgcc gcaaccgtta agactgaaac gtagatcgcc gggatctagc tcttgtctca 660
ttggggcagg aacgccgggg cggggacacg cacgcttcgc ccccaggaat gacctcatcg 720
ctccggagct ccactcacag accccaccta ccacagggaa cgggggcggg tgccagcgtc 780
cgggcaagcg cacaagagtg gcctctggcc ggaggcgagg gcgggaaggt gcgggaagtg 840
cgcgtgcgcg gagcctgggt cagcctgggc ccgggtccgc ttgcagcggg tggagtactt 900
gcggagccgg caatccaggc tcccctccca gcccccgcgc agaattagcc tctctgtgcc 960
gccgggaaat cggcaattag aacgctcctt gcgcgcggca cccaggcagc cctcgagaat 1020
gcctgcactg tggcctgccc atcctcgccc ttcccatacg ccctcggccc cgcgctcacc 1080
acgttcgtgt cccgctccac cgcgggttcc cagcccaggt cccgggggccc gcaacagtcc 1140
aggcagacga gcgcgcggca gcggtagtgg caggtgaact tgcaatctgc agagaggcct 1200
ggcggtgagg cggaggagct ccaggtcggg gaaatgtccc ggagattgaa gggaagcccc 1260
agggagaggg ccgctgctcg ccaggctccg caggcccgac ctatctcagt gggttacctc 1320
acactgctac gcggactcta atgttggcca cctgggcgtc tggaaaccgg ccggaaggcc 1380
acaggcagag aggcctgctc aacagttgga tctctatcgc ctagcacaga acttcccctt 1440
tcctcattgg caattaaaaa aacaacaaca aaaaactgcg tcttgctttt gtcacccagg 1500
ctggagtgca atggcgcgat ttcggctcac cgcaacctcc gcctcctggg ttcaagcgat 1560
tcttctgcct cagcctcctg agtacctagg attacaggcg cccgccacca tgcccagcta 1620
atttttgtat ttttagtaga gacggggttt caccatgtta gccaggctgg tctcaaactc 1680
ctgatctcag gtgatccacc cgcctcggcc ttccaaagtg ctgggactac aggcttgagc 1740
caccgcaccc ggcccttcac tgggaacgta tatggaatac atctgcccat ttacttgaag 1800
gaaaaactaa acacctttaa cctacgtctg ccctgtggtt gtcacctgtc tctactcccc 1860

tcagaccaag acactggtct ctatacactc taatccttcg ccttcactct cccctctacc 1920
cactccagcc aggcttgcct cttcctccag gaaactgccc gggacagggt cctcagcgat 1980
ctgtgtacta ccaaatggaa tccagtgttc cattctccat tctcaccccc tcagcatcat 2040
ttgaagcttg ctccctttga ctcccagggg ctacactctc ccagttttcc tcctaccccc 2100
tgcagctcct gctcagctcc tttgcagatt ctgactcaac ttccatatct cacgatgaag 2160
tctgggctca gtcctgatca ctggcctggt ctgtctacat tcatctgccc cagatccacg 2220
gctgaaacac tgacctaaac cctcagacta gatcctccgt gccagtacct tcactaggat 2280
gtctaaaaga cgtttcaagt gaacatggcc aaaatttaat tccctttttct tcagcctcac 2340
tgctacactt gcccagcttc ctctttgcag caaaaatggc cactaggctc ccagttactg 2400
gagacaaaag cccaaactta tctttgattt ctcccttgtc tctacctctg ataaacatgc 2460
ccaaatcatc ctgcttctta tctccatggc tactttattt ctctttgaga acgctgcaat 2520
gtcccagcct tgttcttttt tttttttttt tttttttga gacagagtct cactctgtcg 2580
ccaaggctgg agggcagtgg cacgatctcg gctcactgca acctccgcct cctgtgttca 2640
agcaattctc ccacctcagc ctcccgagta gctgggatta caggcacccg ccactacgcc 2700

```
tggctcattt tttttatttt tttagtagac atgaggtttc accatgttgg ccaggctggt    2760
cttgaactcc tgacctcagg tgatccaccc gcctccgcct tccgaagtgc tgggattaca    2820
ggcatgagcc accgcgctcg gccccttgtt cattctttgc attctgtcac aactttgtgc    2880
tccccccagc tgaatttgtg atgtcctctt gtaccggatg agagggtctc catgcacaca    2940
cagacctggg acactatcca tccacaagtt cctaaatagg ccagagcagt gatgctcaac    3000
ccagactcca tgttacaata atttggggag tttttaaaat ttactgatgc ctagggtcca    3060
ctcccagcag ttgattcaac aggtctgcgg tgggatccag gctagcgggg aggactgtaa    3120
aagcacccct ggtgattcca gctggtgtct acccagggga gagcaacctt tgcttgctgg    3180
cgattcccag gggtgcagaa ggactgctgg gtgtgtggct gcgtgcatat tttagcatct    3240
gattcactgg gtcagaaaag ggtgtttgct aaataaagac tcaacaaaac tcctgcttgc    3300
agggggccca ccaaaggttc taaatttttc caggctccct cccataggtg gtaatttccc    3360
ttcaccctaa aggttctgga gggggtcatg agtgtttgag aagaggcaag cctgggaaga    3420
tggactccga ggacagtagg cacaaaccct ttctcaagaa gggccaaggc attttaaaga    3480
taagaaactt aaaatcagcg tatttttaca tataagcagc cacctctgct catctgtggc    3540
ccagatacga gtggagtgcg acaagggata aaccattttc gcgcactctt cagcgatggg    3600
gcgaaagtaa cggacctagt cctcgggagc tgtccccgcc gaccccctct gccgcgactt    3660
gacccgcggc gactgcgctg ccccttggct gccccttccg ctctcgtagg cgcgcggggc    3720
cactactcac gcgcgcactg caggcctttg cgcacgacgc cccagatgaa gtcgccacag    3780
aggtcgcacc acgtgtgcgt ggcgggcccc gcgggctgga agcggtggcc acggccaggg    3840
accagctgcc gtgtggggtt gcacgcggtg ccccgcgcga tgcgcagcgc gttggcacgc    3900
tccagccggg tgcggccctt cccagcgcgc ccagcgggtg ccagctcccg cagctcaatg    3960
agctcaggct cccccgacat ggcccggttg ggcccgtgct tcgctggctt tgggcgctag    4020
caagcgcggg ccgggcgggg ccacagggcg ggccccgact tcagcgcctc ccccaggatc    4080
cagactgggc ggcgggaagg agctgaggag agccgcgcaa tggaaacctg ggtgcaggga    4140
ctgtggggcc cgaaggcggg gctgggcgcg ctctcgcaga gcccccccg ccttgccctt     4200
ccttccctcc ttcgtcccct cctcacaccc caccccggac ggccacaacg acggcgaccg    4260
caaagcacca cgcggagata cccgtgtttc tggaggccag ctttactgtg ctagaggaag    4320
agggtcccca catccggccc tggccctcct ggtccggttt gctgaagcaa cacacttggc    4380
ctacccactg ggtggggcag gaagtctcga gccttcactt ggggtgagga ggagggagat    4440
cggtcagcag ctttaccgcc cgctctgctc tccactgcgg agactggggc tccggcagag    4500
gctggaccgt g                                                         4511
```

<210> 11
<211> 1775
<212> DNA
<213> Homo Sapiens

<400> 11

```
acaccgagtt acgaaactta gagcaaaagt aaaactttct aatggaatgc ccatgtaaat      60
actagaaata taacaaaata aggcaaagtc tgtcctccaa aatgttgcct accacaccaa     120
aacacaatcc tgctttttaa acagcgagta aaattaaatc aaccttatct tcctaaatag     180
aagtccactc aaagtttttt cacaaaaatg acattcactt gacttaatat tgtccttcca     240
ataagaataa cacgcagggt gttagagggc agttgttgga ctcctcttca gcaaataaag     300
gagaccagtt agagcttagc ttggtctgcc caggaaagga ggaacgcagc ttgctgacat     360
ggctaaggaa tgccaattaa tacattttaa aacccctatc atcttgcctg agacgtgtac     420
atcttcccag gccctcaaag ttcaatcaga aagtgtattc aactccattg tcatttcacc     480
catagtgggg aagtccatcg aggaaaacca tagggaaata aaatgttttt cagttattca     540
tattagcaca atcaacccag agctcacggc acaaactaaa taagttaact tgccagtgtc     600
ttgaatgaag acatttaatc aaagcatgca ttttccaact ttccagtagc tttcaggaca     660
gttggttttg gtgatcgttt tttggtcttt tattgttctc aacacgactg gtttctcacc     720
gcaggatttc aaacaaaatg agacaattaa accacacctc gagcgaaggg gcgcttacct     780
tgcagataaa cacaccggcc ttgccttctc taaaatgcgg acacgtgctt ttcccgcatt     840
aggggggggtc tcccggcgcg cgccccgccg ccacctgttg aggaaagcga gcgcacctcc    900
tgcagctcag gctccgggcg ccagccctgc cccgcagccc cagagcccgt cgcagctcgg     960
gtggtccctc cccggcccag cgctcgccgc ctgctcttcg ccctgcaagt ttcaagaggc    1020
agttatttct cgcagcctcc gcgcttgcaa ctgcctcctg gcgggggagt gggtgtccaa    1080
aaagccagca gctggagaaa ctgaaaagat cacaagcgac ttaacgataa gccccttctt    1140
ccttttaaag accgagagga gggtagaggg gagtagtgcc tgagcccacg tgaccgagcc    1200
agggagcccg acggtctcag gaacgcccga cgccgcgcgt gacctctaag tgggagcacc    1260
ctcgaaccga ctcctggtcc acccacaagg atagtggcgc acagatggcg ctccccgcag    1320
```

ccccagtctc agatttaaga ggtctggagt agggcctgag aatatgcatt tccaaccagg 1380
tcctggggga tgccgacact gatacagcca gtctggggac cacacttcga ggatcacgtc 1440
ctcagcccct gactcacata agtgtcattc agaacagatg tctgattcta aggagccagt 1500
ggtgaaacca gagaggcttt gggtttgtca aatccccagc agcaaacgta acctcgggcc 1560
ctggagtggc aaagccgtgg actagaggtg gagggagtgg gttctcagct ctcaagaggt 1620
cactgggaag gcctttcgcg ttagatcaaa gatcccaggt acttcctcga actcacttga 1680
agtggcaccg gggagacctg tgcctccggc cacggcgccc ttctccgctg gaggcacctg 1740
cccacccctc ttctcgggcg aaggcccctc gcgcc 1775

<210> 12
<211> 1223
<212> DNA
<213> Homo Sapiens

<400> 12

cccatgtgct ctactcgggg ttcggagtaa acaaaaccga cctaaaaata attttgaatg 60
atagaaaggg ggcattttct ttatccaagg gggtgaggca ggctgaagat gggtctctac 120
tccctccaag accatggagg gcgtcggggt gtcaccccag ctgtgaaaaa gggacccagg 180
acccgcagat gctggcgagg aacaggggtg cggagcatga gtcggctttg caccacgccc 240
tggggtctcc agcactgaag cgttcagtac catgcagagc agctgggagc gggtcccgct 300
gggcggggcc gagctgcgcg cgaccctcgg cgcgctcggg gaggcccaga caggggtggc 360
ctctctggcc tccgctcccg cgggctctat gacaccgcac tggctcgcgg gacggggcgg 420
ggtcgggtga gggggaggag acagccccac gctttgcgac tcgcggtgac ccctacgcgg 480
aactctctcg cggtaattcg aagtaccgcg cctgcgtgct gcagtagcgc ctggtggcgg 540
tggcagtttg cccgcgggtg tgtgaaggga gacagtgtgg aggccacagg gtactcgcca 600
cgatgagcag caccttagct aagatcgcgg agatagaagc agaggtaatg gacgcagctg 660
gcggttcact cttagtctga gcattccttc tttggtggcg tcgctccttc ctgttctcct 720
ttgagccgcg tcaggaccgg gcctagattc cgcgacttct ctcagcgagg cccgtgttcc 780
gactgccctc tgccacgatt aggagtcccc gactctcctc agtcagggcc ccctttcccc 840
tcgtcccgcg tctacgctct cggactcccc tttgcccggg cccgcgttcc gcacccctgc 900
accttaggcc cttagtccgt tccccctccc agtgtttggc tcctgttctg atgttgtttc 960
tttccgtttt gctctgagaa acactgaatc ctgtccctta ttccatctct gacttggtca 1020
gatagttcag gatctttcct catcatcctt cgctgcgtcc cccttattcc ccctttttccc 1080
agtgtctacc ctaatgaccc cctgattcag agtatttgct cccggtaatc ggatctggaa 1140
gtaaaatgac atctgtgtgg gagcagttta ttccgcctgg atagtgtgtg attccattgc 1200
gtgcggccct gagagcgctt tac 1223

<210> 13
<211> 2183
<212> DNA
<213> Homo Sapiens

<400> 13

aagggagaag gacgggggcg ctgtctggtg actgttcgcg tgattccttc aaggcggcga 60
ccacctcctg ggattcgctc tccagccgcg ccgttcgtac tcgggtcccg cgacccctcc 120
ccgctccccc ctcgccccag cgatgccaag acgctgaacc gcagggacga ggctctgggg 180
acaaagtcac cccgcggcgc cctttcctgc caagccgcgg gctcctggcg gtcagaaagc 240
gccccaggcc cacccctgcg ggcccggctt tccctcctca cctaaggctc cggggccacg 300
agtcccccat cctgcgcccc tcccgcgcct cccccatccc aaaccccagc agcgtcgctg 360
cctctcccgg aaacaagccc gctgagcaaa ggcggaggaa aaccttgggc cttccgctct 420
gattggtctc cccccgccga gcggggcgag gcagcgattg gctgccgggc gttgtcagtc 480
gccgaggcca gagccatcgc cggggaagcg cagccgggct cgggtgcttc ggctgccgcc 540
gcggctgctc ggctagtgca gctgggcgag ctcgcgcggg cgccgccgcc gcctctgctg 600
ctgcccaccc cgccgcgccg ccggcgctgc cggtcttgca ggcggccgcc ggggaggggc 660
ggccgagaga gcggagcacg aggaggcggg ggcggcagag aagtgatgct ggcgccgggg 720
atcggggcag cggcagcgga gcagcagcat cttcgggacc ctggctgcag cgtccctgtg 780
gcccgcgcgc cagcccagcg gccggagacc acgcgccccg cgggtaaggt tctgtccggc 840
agcgcccgcg acgcccccgg gacggctggc gtggtctttg ggggttacga ggcgcgtggt 900
ggccggggca tcccgacgaa ggtgttggga cccggactgc gtgtcccgac gctgggctgt 960

gctactgccc gcgcccgtgc ggctccagct ggtgcgttct cccttccgcg ctcacagact 1020
ttgacagcgc ggggatggca ttgtgtgagt gtggatgtgg atgcgtgtgt gcgcgcgcgc 1080
ggcttggggc actttgcagc gagaacagtg ggtcccctct gcgtggcccg acgtccttca 1140
aggacccaag ctgggctgga gtgagtagga tgctgttggc tcggcgggag gtagaacgca 1200
cggagcgcac gggaaagtcc accgtggctc gtaaagagcc gagcaaattg aactttattg 1260
cggtgattaa aaggagctgt ggacctggcg cgccagggga gaggcgcgtt ccacgaggct 1320
ccccaaagtc ccactttgcc ccggggactt cggaggggaa gggaagagag ggaaaagtaa 1380
ctgttggtcc acattgctga agaaaaggag gaaagagaag ttgggggtgg gggtgggggt 1440
ggaggttact cttgggggag tggcgacttt taggtgtctg aagccccgcg tgggaggctt 1500
gctacacttt tctggagcgc agacttgagg atggagagtc ccactctgag gtactggtag 1560
cggctcctcc tgcctggtgg cggcgtaggg cgcagcggga cagcttggtg gaggcagagc 1620
ggggctccga cgcccgacgg cccggcgtcc aggtggggcc actgcctccc caggccgctt 1680
tggcccgctg acctcccagg ttgagacccg gcctggtgcg cctctggcag ctgccaaatg 1740
cctggcggct ccgctttccg acaaagttca ggcccgctgg ggtctggtcc atcagaccta 1800
ggacacgtta accacgcaga gtaaccaggt tagggtaccc agctgcctcc agagcgtttt 1860
tgtttcatat tagattatct tatttgataa agctgttgag agttccaccc gtgctgcaaa 1920
gccttccgca gaaggcgaat tctttgcgct gctgcaaatg gatggtgcta ctgggccaga 1980
tccgaatctc cgtagttcag tgcagtgggt tctccatccc tgctcaggac gcacaaactt 2040
actcttggcg attgttttct atgtggctcc ttgtctccgt gggagtatct gcttaggaaa 2100
gttcttgaaa tacctcaata ctaaggagct ctccccactg tctcagatgg ggcgaggctc 2160
cggtacttga ctggcgttgg ggt 2183

<210> 14
<211> 8030
<212> DNA
<213> Homo Sapiens

<400> 14

tctgcgcaac ctcagctagc ttgaatcttc ggaggcaaac agagcgcaac ctgctttgga 60
agaatcttgt tacgtttaag gctttatgcc gggtgttggc ctctctgtct tcaactaccc 120
ctccaccccg actcggactg cagaaatctc caactctctg tcccccagtt ccactcccac 180
ctccagacct ggggttgcca aacaacggaa attctaggaa gacgtaggtg cggtttttaa 240
agccttggct tgtgagcgtt ctcaggctgg ccggggcgct ggtcttttca gaaacaaggt 300
ttgaatatgc aggtgtcagc caggattcct tgtcgtctgc ccaccgccgt ttctctcccg 360
ggatttgaga gaagcgggag tgtgtgtgtg tgtgtgtgtg tgtgtgtgtg cgcgtgtgtg 420
tgtctcgtgt taggagaaag gtccgtggtt gccgtctcag ctggtttgct tactctctgc 480
ccccaggaga agtttgctca cttgggacac cagcgaccct cctcacctcc acctcaccgg 540
ttctacactc tccccacttc tttccaagtc gctgatgcag aaagcacttg gtcaccttga 600
aacggcagct cccggaattc tagttttgtt ttgcaatcta gcagggctct tcggaagcct 660
cataactgag atttatgggc tcaccgggta attaaatgat gttattgtgt taactttgca 720
tgcattactg ctgcccgcgc cgcccgtggg ccgccggccg gccgcagctc gctggcgacg 780
agggcactac agttgctctg accgcgtaga ttatgcatgt cccggcctcg ggaatttacc 840
atgcattaga atacattagc gcctgcattt taaaaggcta aactattggc tcccagctag 900
ggactctcgg taagtggctt gttagtgacg agtgtttgtc tatactggca catagcggag 960
tcttttgctc ccggcttact cgcctccagg aaagctttgg ggtgaggcga aggcgattga 1020
agcaatgccc cttcccccag atcgcagctg ctcagggggg acacagcacg gcatctttca 1080
ccgaatctct ctcgctcgct cgcactccag cctccctctc cccagcgacc cccccacctt 1140
ctcctccctc cctctccttg acgtttgatt ccagtagcaa aggaggtaaa aaaggcaccg 1200
agccgtcagc caaacctgaa aagtgcggcc ccgccccctc cacagccact ggtagcttcc 1260
cgtggaaggc ccgcctcccg gggcagctgc ggcctcggag tggttgcgct tggcgcccgt 1320
cgggcgtggc cccgccccag gtccgggagg gtaggttggc tgccccggcg agcggcagag 1380
cccttctgga cagctcccgc tcacccaaac agaagacgtc ggcgccggag cgggctcgga 1440
catggcgagg ctgcgagccg gcccgagcgg cggggcccgg tgatccctcc ctccctcccc 1500
gtcccctccc ctctcccgca cgcacgcccc gtccgccccc accccgcccc caccccgggc 1560
gagcccgccc gcagcccggg gcgcacaccc gcacgcgcac tcctctccac tcactcccgc 1620
gcccgccccc actcccgcag ccgagccccg ccacgcgcgc cttgcccgcc cgccggccgc 1680
ccccgccgcc cccgccgccc ccgggccctg atggactgaa tgaaggctgc ctacaccgcc 1740
tatcgatgcc tcaccaaaga cctagaaggc tgcgccatga acccggagct gacaatggaa 1800
agtctgggca ctttgcacgg gccggccggc ggcggcagtg gcgggggcgg cggcgggggc 1860
ggcgggggcg gcggcggggg cccgggccat gagcaggagc tgctggccag ccccagcccc 1920

caccacgcgg gccgcggcgc cgctggctcg ctgcggggcc ctccgccgcc tccaaccgcg 1980
caccaggagc tgggcacggc ggcagcggcg gcagcggcgg cgtcgcgctc ggccatggtc 2040
accagcatgg cctcgatcct ggacggcggc gactaccggc ccgagctctc catcccgctg 2100
caccacgcca tgagcatgtc ctgcgactcg tctccgcctg gcatgggcat gagcaacacc 2160
tacaccacgc tgacaccgct ccagccgctg ccacccatct ccaccgtgtc tgacaagttc 2220
caccaccctc acccgcacca ccatccgcac caccaccacc accaccacca ccagcgcctg 2280
tccggcaacg tcagcggcag cttcaccctc atgcgcgacg agcgcgggct cccggccatg 2340
aacaacctct acagtcccta caaggagatg cccggcatga gccagagcct gtccccgctg 2400
gccgccacgc cgctgggcaa cgggctaggc ggcctccaca acgcgcagca gagtctgccc 2460
aactacggtc cgccgggcca cgacaaaatg ctcagcccca acttcgacgc gcaccacact 2520
gccatgctga cccgcggtga gcaacacctg tcccgcggcc tgggcacccc acctgcggcc 2580
atgatgtcgc acctgaacgg cctgcaccac ccgggccaca ctcagtctca cgggccggtg 2640
ctggcaccca gtcgcgagcg gccaccctcg tcctcatcgg gctcgcaggt ggccacgtcg 2700
ggccagctgg aagaaatcaa caccaaagag gtggcccagc gcatcacagc ggagctgaag 2760
cgctacagta tcccccaggc gatctttgcg cagagggtgc tgtgccggtc tcaggggact 2820
ctctccgacc tgctccggaa tccaaaaccg tggagtaaac tcaaatctgg cagggagacc 2880
ttccgcagga tgtggaagtg gcttcaggag cccgagttcc agcgcatgtc cgccttacgc 2940
ctggcaggta aggccggggc tagccagggg ccaggctgct gggaagaggg ctccgggtcc 3000
ggtgcttgtg gcccaagtct gcgcgccgag tcacttctct tgattctttc cttctctttc 3060
ctatacacgt cctctttctt ctcgttttta tttcttcttc cattttctct ttctcttccg 3120
ctcttcccct actttccctt ctccctttc tttttctttc ttactctctc cttgtccctg 3180
agctttcatt gaccgacccc cccccatttc attcgccctc ccctcaatgt gccaaccttt 3240
gccctatttc cgatcttccc aggtactggg aggcgggatg gggtgtgcg ttttcctcta 3300
ggagccctgt ctttccaaga cccacagaaa ccaggacctg cccttattca aaaccccatg 3360
cacttcaagt ctcttttaga caacacattt caattttccg ggctgactag tctccctgtg 3420
cagaggcagt tgagaggctt tgctctgcag agggaaaaga gctctctact ctcccaccca 3480
ccatataggc aaacttattt ggtcattggc tgaaggcaca gccttgcccc cgcggggaac 3540
cggcggccag gatacaacag cgctcctgga gcccatctct ggccttggcg ttggcgcagg 3600
gactttctga ccgggcttga ggggctcggg ccagctccaa tgtcactacc tacagcgagg 3660
gcagggtgta aggttgagaa ggtcacattc accgctttgg gaggacgtgg gagaagagac 3720
tgaggtggaa agcgctttgc cttgctcacc ggccgtcctt gccccggtcc cagcgtttgc 3780
tgggatttgc caggatttgc cggggctccg ggagaccctg agcactcgca ggaagaggtg 3840
ctgagaaatt aaaaattcag gttagttaat gcatccctgc cgccggctgc aggctccgcc 3900
tttgcattaa gcgggcgctg attgtgcgcg cctggcgacc gcggggagga ctggcggccc 3960
gcgggagggg acgggtagag gcgcgggtta cattgttctg gagccggctc ggctctttgt 4020
gcctcctcta gcggccaagc tgcgaggtac agccctctat tgttctagga gcacagaaac 4080
ctcctgtgtg ggcggcgggt gcgcgagcta gagggaaaga tgcagtagtt actgcgactg 4140
gcacgcagtt gcgcgctttt gtgcgcacgg accccgcgcg gtgtgcgtgg cgactgcgct 4200
gcccctagga gcaagccacg ggcccagagg ggcaaaatgt ccaggtcccc cgctgggaag 4260
gacacactat accctatggc aagccagggt gggcgacttc ccatggatcg ggtggagggg 4320
ggtatctttc aggatcggcg ggcggtctag gggaacaatt cgtggtggcg atgatttgca 4380
tagcgcgggt cttgggatgc gcgcggttcc gagccagcct cgcacagctc gcttccggag 4440
ctgcgagctc aggtttccac ccccgatccc ccgggctttc ctcgcaccgc tgagcccagc 4500
ttgtggggtg cactcgacca acgcccgaca gggctgggga atgtgacagg cagcaggttc 4560
acccgggctt ggggaggggg agtttccgct ttgacagcat tttcctttgc cgtctgctgg 4620
tggattccta ttcccagtcg gtaatcgccc cgcagtgttg atctaagaag gtaaagaaaa 4680
ctaggtttcc ctgcaaagag cctcccccaa atcggcggac tccggatact ttgagtggat 4740
ttagaaattt atgtaatctt tctcctttag tttatttttc atcctctcct acagttttct 4800
ctgatttgct gttggttcgg ggcaagataa agcagccagt agagagcgat aataatagcg 4860
gcgggaaatg aactggagac tggctgacag ttcttaacat tttgtcatag atccccccga 4920
atgtcccagg ctgtctctgg tgggttttag tacccgccgg cttcttgggc accggggacc 4980
agaaggaact tggcagctgg tcttaggggt acagttaaag gcaggatgac agctattctc 5040
ctgctcatct cagagcgctg ccgccccctc atgccggtcg cgcaaagaac acagctttta 5100
aaaaacacgt gccttctgcc catataggtc tgaaagtgat gaggaaagta atgcttcgcc 5160
tattagcgag tttcagcttt taaaatgatc ccaagcgttg ctgagatgag aaagcgtggc 5220
atcccggggg tcctcagccc cacccgcgcc catggtgcaa gtctgcaggg acaggcccgg 5280
gacagcactg cccacgctgc tagattttcc gcagaggatc gctgaagctg ccttcgtggg 5340
agacagaatg cctcctccag cgagtggaaa aggcctgctg aggaccccgc tttgctcgag 5400
cattcaaatg tgtgtctgtt ttattaccct gggttgaaaa gggacaagag ctttagcctt 5460
tttatctggc cattttatca gcaactacaa gtgtgttgag tggttattat tacataggag 5520
gcttttcagt ttggggtcag tagatcagtc tcttcagaca ctgatgcaga agctgggact 5580

ggtaagtagg tattatgtgc tcggagcgct aggggacagg agcaaatgga gaagaaaagc 5640
ggaggctttc tccgcccgga gtatcgatcg gaatccccgc cggtacgccg cagagggccc 5700
tcgccgttgg gccccggggg tttaacaagc ccagccgctc cgcaggcggc tcggccggac 5760
tctcagaccg gtgcctggaa gacaccgtcc ctgcccccct cccgccaaac ctgcctcttc 5820
tctttctctc ataggttata ggttcccttt ctctctcatt ttggccccgc cccccgggtcc 5880
tgccaaacag ccaagcaggc cggggtttag gggctcaga atgaagaggt ctgatttggc 5940
cagcgccggc aaagctcacc cttaggcgag gtcacaacag aggcaggtcc ttcctgccca 6000
gcctgccggt gtagtcacag ccaagggtgg cacttgaaag gaaaagggag aaaacttcgg 6060
agaaatttag attgccccaa cgttagattt cagagaaatt gactccaaat gcacggattc 6120
gttcggaaag ggcggctaag tggcaggtgg ttgcaacccc gcccggtcgg gccttcgcag 6180
aggttcccca agaccagccc ttgcagggcg gttttcagca acctgacaag aggcggccaa 6240
gacaaatttc tgcgggttcg agcacacact ctcgggcgtt gggccccaga gacctctaaa 6300
ccaagcacaa acaagaaggg agtgagagaa cccaggctag aacttgcacg ggcatcccac 6360
tgaggaaaag cgaggcctcg gtggcaggca tgttttcttc cgacgcccga aaatcgagcc 6420
gagcgcccga ctacatttac tgcagaggtt tccgcctcca gtgagcccgg atcccccagc 6480
ggcctgcccg gagctggtct ccagtccccg ccgtagtccg acgcacggcc ctctcctggc 6540
agcaagctcc cagcggccag tctgaagcca attctgttca ggcggccgag ggcccttagc 6600
caacccacca tgatgtcgcc tgggccacct gatgcccgca gcggcgggac acggcccggg 6660
cagtgcgcag tggctcctgc taggggcacc gcgtgcgtgc ttgtctcccg ctgcgccggg 6720
gacgtccttg ggtgacacgg gccgctgggc acctcccaag ccgaggaaac ggacccccctt 6780
cgcagagtct cgcgcccacc ccccaacctc ccacctcgtt tctcgctgct agggctcccg 6840
actcagccca cctctcctgg cggtttagtt agggatcaga gctggagagg ctgaacgcaa 6900
cccgtgccag tacggaacag acgatatgtt tgcctgctag ctgcttggat gaataattga 6960
aaagttcgct gcagtctgtg cttcgtcaag tcccgggtgc cgggagaaca ccttcccaac 7020
acgcatcagg gtgggcggga gcgggcagag gaggcgggac ccgagggagg agagtgaacc 7080
cgagcaggag aagcagccca ggcagccagg cgccctcgat gcgagaggct gggcatttat 7140
ttttattcca ggctttccac tgtgtggtta tgtcactttc tcaaacaaat gtgtatatgg 7200
agggagatcg atgctgataa tgtttagaag attaaaagag cattaatgct ggcaacaata 7260
acgtaaacgt gtggacccag atttcattga tctggaactt gatccggcgc gtttccagta 7320
agcccgacgg cgcgctcttc ccagcagagc gctcaccagc gccacggccc cgcggttttc 7380
cagcggtgcc gcttcgccag ctctgcgcgg gttctcccgt ctgaccgcag ctcctccccc 7440
gcgaggcccc agcccgcctt acttccccga ggtttctcc tcctctcgcg gggctctctg 7500
ccctctgcac cccctccccc gacctctgca ccacccgccc ctgtgcgcac acaccgctac 7560
ttgcgcttcc ggcgatccgc ctgggcggct gggtccgcga agccaatgcg ctgaacggtg 7620
cccgagtctt cctaactatc ctgtgcttgg ccgttgccac tgggccctgg tgactaagcc 7680
caagtttgaa aatgacgtgg ctaaagctgc ctgctaacgg cagagcttaa tcagccgcag 7740
atcccctcct atcctcatcg gttctcgtac taaaaaggct cacgcgcaga ctttttacgc 7800
aggtgcattc gttggacaat taaacgtggc cctagtaaca aaagcctgag cttcatccct 7860
ccgagtagca ggctctgcgc tggactggtc gggtctaaca gggagaatct tgtgtcctac 7920
cttggctggg acaggaagta agagaagcaa actggggaac ccctgcccca ccccttccca 7980
ccccctggac gtcctgggcc gaggcccggg tcactggccc atcgcgggta 8030

<210> 15
<211> 2225
<212> DNA
<213> Homo Sapiens

<400> 15

cacctgggtg caggcgggct gagtccgaaa agagagtcag cgaagggaga taggggtggg 60
gccgttttat aggatttggg aaggtaatgg aaaattacag tcaaaggggg ttgttctctg 120
gtgggcaggg gtgaatctca caaagtacat tctcaagggt ggggagaatt acaaataacc 180
ttcttaaggg tggggaagat tacaaagtac attgatcagt tagggtgggg caggaacaaa 240
tcacaatggt ggaatgtcat cagttaaggc tgtttttact tcttttgtgg atcttcagtt 300
actttaggcc atctggatgt atacctgcaa gtcacagggg atgcgatggc ctggcctggg 360
atgcgatggc ctggcctgac aactattacc tatgttatgt ttattatttt aagctttatt 420
attactattt tatttatttt attttatttt ccttccacac acccgtttcc accctggaga 480
ggccagatga gccagactcc agggaggcct agaagtgggc aaggggaaac gggaaaggag 540
gaagatggta tgggtgtgcc tggttagggg tgggagtgct ggacggagtt cgggacaaga 600
ggggctctgc agccattggc acacaatgcc tgggagtccc tgctggtgct gggatcatcc 660
cagtgagccc tgggagggaa ctgaagaccc ccaattacca atgcatctgt tttcaaaacc 720

```
gacgggggga aggacatgcc taggttcaag gatacgtgca ggcttggatg actccgggcc     780
attagggagc ctccggagca ccttgatcct cagacgggcc tgatgaaacg agcatctgat     840
tcagcaggcc tgggttcggg cccgagaacc tgcgtctccc gcgagttccc gcgaggcaag     900
tgctgcaggt gcggggccag gagctaggtt tcgtttctgc gcccggagcc gccctcagca     960
cagggtctgt gagtttcatt tcttcgcggc gcggggcggg gctgggcgcg gggtgaaaga    1020
ggcgaagcga gagcggaggc cgcactccag cactgcgcag ggaccggtga gtgtcgcttc    1080
tgggggcagc gcccagtaac cgcgctagga gcgcggagaa gggcattggg agagcggcgt    1140
tcgtggcgga gactagcgct ccggagcacg ggcacgacgg gggcaccttc tcggctgcta    1200
gtaactaaca ataataataa tcataatcat agcaagggcg ctgatgggcg ggctcggagc    1260
acgcctgatt ctggttccca ccaggctgcc caggctcctg atgacgcatc agaaacatcc    1320
ccctaacccg cggccttcct gcaggagagg ttgggaaggg gtgggggacg gggctcgggg    1380
gaggtctccg agggactcta gtaagcgggg aagggcgccg ggaaagtttc agatccacgg    1440
ctgcgcgggc cacgagccca cccgaacgcc gaccactgct ttccgtcgac ttctatttcc    1500
tgggaacgcg cgaaagcaaa cccaagtcag actgcggagg tcgctgggga gggaaggttc    1560
aaggagttct cgccgatcct gctgaataaa gggggttccg agctgggccg agatggggca    1620
tgcgcgggaa gacccctgcc cgctgttccc ccccaccgcc ccagtggatg ccatgcctgg    1680
ggcctccccg gcgcgtgggg ctgacgcacc ctcggggtcc atcgtagttg gccgggatcg    1740
tggagtgggt gcggtggacg aagggaggca ggacagtccc gggggtggca gaaggagccc    1800
gggcacagct gagacctgcg ctcccatccc accaacactc acagcaggtg ctgccgagct    1860
gggcaattgg gatggcccaa gttatttggt taaattttaa atcacgtttg ttactgggaa    1920
gtagagtcca gtgatgctaa ccgcgcctct acctccacca ccggtgtcag tcccaaaggg    1980
ctcctaaaat ggctgtgtca tctttcagcc ttggaccgca gttgccggcc aggaatccca    2040
gtgtcacggt ggacacgcct ccctcgcgcc cttgccgccc acctgctcac ccagctcagg    2100
ggctttggta ggtagcagtg catttggtct aaagggcaag atgttctctc ttttattcat    2160
aacaaattta aataccagca gggtttgggg ggaaaaacgc tttcagaaga aaaggtgaat    2220
gtcag                                                                2225
```

<210> 16
<211> 3377
<212> DNA
<213> Homo Sapiens

<400> 16

```
cacccgcggt tctatgagtc tgtactgaag tcggatatcc ataatatttt ccccaacatc      60
ctatcatttt tctcccttga caaccatgtt ttgagaactt gaagcaacag atatgaagag     120
tcttaaggga ttgcatcaat gtacgctaag attagtctct accacatagt ccaatgacac     180
ggttttaggt tcagaaggga tgctctacac ccacagaatc ccaccgagtt caggtgccag     240
ctcagagaac tcatcacctt cctgtttccc ttcaagagtt aactgatggg atagtatgca     300
aagcacatgc gttcttgctg ccaaaatagc agccaaaact gcctgcctag atgtgtaaga     360
tcccatgaaa tgtcttggca aaagctagtt taggagtgtt ggccaaggtg ctgaagcgaa     420
gcaaggggag tcaggaccac ctttgccact gaatctcccc tcccccacct ctttttaaag     480
acagcgagct tattcatttt gacaagatac cctaggattt caaatctgac ataattttgg     540
agaaagcagt gcgtgtgtgt gtgtgtgtgt gtgtgtgtgt gtgtggtgta gaagggtatc     600
tgggatacat aatctttata gaaattctag tgccacaaaa attctaggag gatctacatt     660
taaaaaaaca cacaacagtt taagctgttt attccaattg aacgctgtgt gtttccatca     720
gatcctcagt gagattcagt ccaatgagtc aattgcaaaa taaagttttt gtcaagggaa     780
tggcagaaaa tgacaaaacc acaattctga atgatcattc aaaagtaaaa agtactttct     840
actactgtgt ggagatttc ttatctctac ttttactcga attgaatctt ttcttctaat      900
gttcttttga acagtttgtc tttggtgtcc tgaaaatgtg cacgctgata gcagtaccac     960
atgctgtccc tggcacagag tgagctaaca gttgaaccac ctctgggtaa ctgcggactg    1020
cagctagtgt aagtgcaaga gaaggcaccc tggccaccaa ttcgtttcac actccaatca    1080
cccccgcccg ctctacaccc cctgggcacc cgatgcctct tccctctgcc aggcaatttg    1140
cttccgttct gttcaacttt ccgacctctg gctttagaga atagtcctgg ctgtgggaac    1200
tggctctggg aagcttttgg cgccctcctc attggtgact ggcaagagta acactttcac    1260
actcttaaaa agagccgttt agttcctaat gatattttct ggcaaaaaag aaaatgaaac    1320
acacaacccg gattccggtc caccaaacgc aggccaagag cgcggctgct gagccccggt    1380
cttgctgtga ggctgttgtt cccactcgct tcccgtttcc actagagttg atttgtctct    1440
tgccacctct ccccggcagt gtggacctac tgcaacgcag gaacatctgg gttggtatgg    1500
ataggatcct agcatcagat gctggctgct gccccaggga cttcccgcgg aaatcaccca    1560
gcacttagaa gccgtttcaa taaggaagta accagtaaag cattgctggt gggagaaccc    1620
```

cgcgaaacat cttagaaaaa tatcagaaac cgctggagac ctaatgaccc acgtctcctc 1680
cagcctgtgt tcctcagccc tttctggagg agtattaagt ggtttgtggg tttttaccct 1740
gcggtgggag ggctgttcca agtacatcta tttctgcgat agttaacgtc gggggccccc 1800
agctctaacc agggcgagga ccacggactg catccctctc tctcagcgca ctggtctgtt 1860
tactggaggg tgagagggac gcgcagacct ccggactcag ggttctgtt ctgtagatcc 1920
ccccaccccca cccggggtct ctggcactcg tatcacttag caccagtgtg actggcggtg 1980
cattccggcc ccctcgcgcc ccattaaagc ccctgcacat cctctcccac gctgcgcttc 2040
tgctacccac gcagctgctc ctctgagcgt cccgcgtgca caaacgcgcc cccggtcctt 2100
acgcgtcggt ggcgctgcct cgctgaggtg gcgccggggg agcccgcaga ggcggcgggg 2160
gagggagtgc ggaggagagg acccgtgagc tgcggggacc tgggcccgcc gagcttttgg 2220
ggaagctggg ctccgttccc cccgggcgcc tggggcgcgc gggaaggcgc ggaggacgcg 2280
ccgcactcac ccgctgctgc tgccggctgc tgactgccac cgcggcggcg gacgcggcgc 2340
tgtgccggag ctcggccgcc tgcccaggtc tcaacaggct ccgggtgaac ctgcgggtcc 2400
gctcaccggc catcgccggt cacgccaatc gcgccgcggg cccggggcgc gcctcccgcc 2460
ggtcttcccc gcgccaacct tctctatcca cccgccgttc aggaagcgga actcggagcc 2520
ttcccccccc tcttcctcgc tcaactcgca agaggcgggt ttttctctct tgcacccccca 2580
ctcttcccac cccccttccct cctctgaagc ttctcgaaga ctttccaaac tctgcgctcc 2640
cccgggcgcg cgcggccaac tcggctggcg gccgggccct gcagcggcgc atctggagcg 2700
ggcgtcgcgg gacgcgtgtg gggcggctcc tgcccgcgcc gcccacacgg gctcagctgg 2760
ccggccgggg ctgtccacgc cgcccggggg aactgcaggc tgcgggggag ggcgggggc 2820
ggccgccgag ggctccgggg cggggcctgt ccaaggccgt cgcctgtcca aggccgtcag 2880
cctcatttgg aggttgctgg gagagttgga gccctggatg catttgccgt ttagacttct 2940
attttctggg tccggctagt gggtgggggc caagctcggt tcgcccttcc accctcggga 3000
aggggtcaga ggcttattaa agatctggtg gctgggatcc cagcagcatc gcccaagcca 3060
gcgtttggaa ggctcccttg ccagaagtcc atgttcaggg ccttggaata agcttcccca 3120
ttcattcact ccttcaatga gtatttatta agcgcctact atatgccagc tcacactgtt 3180
ctggaaactg aggatacgga gaggaactta gcaactacac tttaaggctg tgtagagctg 3240
ataggttttt tcggggtagg ggtgggagac agcaagtaac caagcgaaca aataaacaaa 3300
tacagagaaa tcaaggagac agtttaagct actaacacga actcaaaacc aataaatcag 3360
cgttctgggg gtaagag 3377

<210> 17
<211> 3043
<212> DNA
<213> Homo Sapiens

<400> 17

aaaattcaaa cctataattt gaggtacctg ggaagccgct ttcccagggc ggaatggtca 60
cggaaactgg gtaggctggg ggccttcttg gggaatgagg gagtggagga gctctttgtc 120
cctcctgcta gatcagaaag agaaacgtct caagaatctg ggcctccatc ttctaggcgt 180
ctcccttgga ggcccttcag ggaccgccca cagcgctttc tctcagcctc ctcccccccc 240
ccatcccctc ccccacccgc cccgtctcgc gcccgccccg cccctcggcc cgcggggttc 300
caaggacggt tggtcgcccc cgccacagtc actcggccgc tcagagcggc ggggcgcacg 360
gggtcgagag agccagctct agggtttggg gctgggaact gaagcctggc gtgaaggaag 420
tgggagcccg ggccgaggag gcgaagggga aaggaaaagc gaggggaacc tgagcgggag 480
ggccctgaga ggagcgggag gctgcgggaa ggggaggcct ggcactcctg ggggtcatgg 540
gggtcggggc gcggctcccg gcctgggagg gcgcgggtcc tccccggcag cgccgcccgc 600
tggcccagct acgcgtgttg tgcgctgcga ggccggcggg ggtcccgctg ggcccggggg 660
tgtcctcggg ggccgcttgc gcccagccat ggtagggcgt cccccggtga aatggggtcc 720
gaggcgggcc ccgaccccgc gtcggcgctg cggaccgtcc gggagctgca gccgcgggtg 780
cccggtgctc ggtttgtagg cagtgtcatt agctgattgt actgtggtgg ttacaatcac 840
taactccact gccatcaaaa caaggcacag catcaccgcc gcccggccgg gaagaagacg 900
ccggctcggg cagcccgcag ccttcgagag aagatgcctg agaagcgcgg cgtcggcgtg 960
ggtcctgcgc agcctgcccc gcgagcgccc gctgcaagtg cgaggaaacc cgcggtttct 1020
ccagatacag ttaaactgtt agctctctct aggagtcaca gaagatgaaa cagtctcatg 1080
ccaggaaagc aaaatccctg gaggtgaagc ccctccatcc atgtaacggt taatactgta 1140
tgctgtgatt cactgtgtct atttgccatc gtctagtaga gtattcacca agctagcaac 1200
tcagttgagc tccaactcaa ccaatgaatt gcctgcctgt cacaacgtgt tggggtacca 1260
acttgagact gcaatttttt ctatgagtct agttactagg cagtgtagtt agctgattgc 1320
taatagtacc aatcactaac cacacggcca ggtaaaaaga tttgggaatt cgtccaaatg 1380

```
agctgcctgt gcatcatcaa tgtgcgtggg gaagaggggt gttggaaaat gctgatttca    1440
tccattgcct attaattgct cagccaaaag aaaaaaatca acatttcagc tactaagttt    1500
acaatgtatg taatgtgtat gtatgtgggg ttttgttttg ttttttttttc aatattcctt   1560
caggctctta accaaaattt tagatataag ggggaatatg attttttttct tagctgactg   1620
atgtatgtta ttatatgaac atgtgattat taacttcttg agactatatt gttagtaata    1680
ttttgaaagt aatattgtta gtaatatttc gaaagaataa agtgccataa agacataaat    1740
tgtggttatt gctatgtgtc catttccttt gctaaattga ctttatgatg tccatctcag    1800
ttcctgccac agtaatctta acttttctat caatgaagtg gttaagttgt tcctgccatg    1860
acaaaaatct cccgggaaag ctactcatac acacagactt gttaatactt ttataagaaa    1920
tacattaaca aaatttttca tacttctttt ttccatgaag agactctgtt cactcattaa    1980
gatgtatccc ttcttttaag taatcattta caactgttgg agtctatgca acttttagtg    2040
ggagtgatta gtaaatgcac ttacacttta aatccagctt gtttcctaag atttaaagga    2100
ttcagaattt taaaatcaca gaaactgtac acatgggcac gtatccgccc agttcaggcc    2160
tctagctccc aagggctagg ctttcaggcg tgaattctcc agcgcaacgt tctcagaact    2220
tccacctagc aggtgctgtc cctggcaatc tggtttggaa gtggggattg ggcaatggg     2280
gctgaggaat ttaaagttta tttagctatc tttctggggg gaggggagag gatgtggcag    2340
cccactgagg atataagaag ctaaactact cccccactga ctccgacagg acacaaccca    2400
aagttgacgt tttgactctc ccatatccaa gatttggact ggtcctaaaa cctgatcacc    2460
gcaggggtgg gcttccgacg gcccccacgc gcgtggcgga tcccaacacc tacctaggaa    2520
accgagggca gccggcaggg gctggtacca acttggcggt tctcggaggg gcggggcagg    2580
ggcggggcgg ggggagggga gaggacgctt gcagaagcgt cctcagttgc catggaaacg    2640
ggacccagcg aagaacctag cggccgaaaa gagtcccagg aaatgtgccc cccgggatta    2700
ctggtatttg ctggctcctc ggaacaagat gccaacttgg ctaagcagtt ctggatctcg    2760
gcgtcgatgt atccccctag cgaatctcag ctggtgctgc gcagagacag cagtcagcgt    2820
ctgccggtgg cgcggcccag gaggagcaga gggtctggtg agtagaatag cggcctcctg    2880
tcagtcatat ctgcctcacc gtaactggcc tgcaggtacc agagcccttt ccaggagaaa    2940
ctgcaatggt atatacaaat agttataagt acatacaaat gcatgtataa atatttcctg    3000
aacccttcca gcaacccttc cacccgaaaa ggatcagtgg tat                      3043
```

<210> 18
<211> 4908
<212> DNA
<213> Homo Sapiens

<400> 18

```
ctagaggtca cgtcggccac ccttttctgc ctgcaatgcc tcagacacac atgctctgta      60
acccagaaat ctgtggccaa gaaaggtgcc aagacccgca gaaaagctgg gacacaggaa     120
agagaaacga aggctgacaa cagatctcac gggggtgagg gagattaggc caatcagaga     180
agacagcaca cgccagcctc agatgcagaa caactggacc tggacctaac aagctgagta     240
caagtggaga cgcaaccaga aaagattgaa gtgaaatcct caagcaaaaa aaaaaaaaaa     300
aaaaaaaaaa ggatggagca gtagtcagag gggatgccac ccctacaaaa gacaaacaaa     360
ttgatggaat ggaaccttgc agggagagag aggaccagca gagagaggga ggcgtaggaa     420
aatgaggagt ggggcacaaa ggtggggctc tcagggagaa gaggagtcga ttttagaggg     480
ggagaagaaa aggacacaga gctggctgta cagagaaaag gggccaaggc agagaagatg     540
gcgggatggt gtgagacgga gttcacagaa atacccagag aaggcgacga ttacacagag     600
gagcccccag aggggcgaac tcttgaggaa gatgaacgcg gagggtggag ccagccagaa     660
attcagaaaa actagaggcg tccccggtag cgaaggaagc cgggaagggg ccaggctatc     720
agggcatgaa gcacgggaga aaatgggtcc gaagagggag agcgtccgag agaaaaggaa     780
aatcagagat taggaaaatc acaaccgaca caaagacaag aagagacaca aggaaagggg     840
tgcaatctga ggaaggaggg cccggtaagc aacccggtaa tcacagcgct gggagagaac     900
gcataaaaga agggtagctc aggagttgtg ggcaccggga agagacataa cagaaaacgc     960
aggcctccac tcagcaaaaa caagccacgc gcgaagtcga ggtacaaggc aagggaaggc    1020
cagaaacgag gcaacccgtg cacagaccgg gcttggtcaa cgccccaggg gcggggccag    1080
gcgggccgtt cctgggggc ggggccagcc tcggtccaat aaggggtact cgacgcccca     1140
ttggccacct gcctcgaaag gggaagaca gtctgggcgg gcaggacgtg cggagggaga     1200
ggcagcggtg gcgcgaggct caactcgaag cgctattggt gggactgata gtcttgtgcg    1260
ccaagaagct ggcagaaggg aaggggcggg acatcagttc aggatctcaa accgcattgg    1320
tcgtctgcct cggctgagag aggcgatgct tgaagttcat tggtctttgt gagacagggt    1380
aagaaaagca gcgcgagctt ggcggttcct ctggccactt tctcacagtg tctttgggcg    1440
tcttcttgac tgaatctgac tccattggag gctgtggtaa ccgtgaacta gctgcacgaa    1500
```

```
cacggtccct cctgcctttc cgtcctcctc cctcttagcc ggactcccta acccaggcat     1560
cccctctcct ccccctcgcg cccctcccgc cctcgcgccc cctctcccct tccctgcctc     1620
gcgggcccct tggcgcgttc ccggttcccc actcgctccc tggggcacgg gaagggggga     1680
gggggacatg catgaaatta attgttggta gtggccacag gagcaggaag cgctggacac     1740
agtgacttca catgtttaca caaaaacctg aataaggtgt gagcgcaaga gagacattag     1800
tgccccaagg agaaggtttt tggcccagat cagggcctag cacatgacag ggctcagta      1860
accatttgtg gaatgaatga aggagtgaag gaaaataact tgaaagtggg gcaggtggcg     1920
ggtgcggtgg ctcatgcctg taatcccaac actttgggag gccgaagcag gcaggtcgct     1980
tgaggcaggt caggagttca agaccagcct ggccaacatg gtgaaacccc atctctacta     2040
aaaatacaaa aattagacgg gcgtggtggc acaggcctgt aatcccaggt actcgggagg     2100
ctgaggcagg agaattgctt gaacccagga ggcggaggtt gcagtgagcc gggattgcac     2160
tactgcacta cagcctgggc tacagagtga gactccatct caaaacaaaa aaaaagaaag     2220
aaagaaagtg ggacagggga gccaagcata gtggctccag cctgtagtcc cagctactgg     2280
ggaggctgcg gcaggaggat tgcttgagct caggaggtgg aggctgcagt gagccatgat     2340
cacgccactg cgctccagcc tgggcgacag agcgagaccc tgtcaataaa aaaaaaaaag     2400
aaagtggagc agggaatcca tgagaataag gtaattattg ggggtgcaag agagggttct     2460
gatttaaagg ggtacaagaa caaaataaga ctaaaggaat gcaaggaggc agttgctttg     2520
cagggcatag atgagactaa gaattgaaat tgaactaaga attgaaaggg gcataaaact     2580
actggtaggc attggatgaa tgcatgagag tggggtgagc attgttgaag aggatgccag     2640
gaaacactgt tgggacggaa cagggtgctt gtgaaaaggg gatgtaggac tcgctgaagg     2700
ggtatgtgag gacatgagta ggttggaacc taggatgtgt gtgtgggtgg gaaagtgttt     2760
ggattgaaag aaaagaatgt ttgaattgaa agaatacaga atgtcgggcg cagcagctca     2820
tgcctctgat cctagcactg tgggaggcca aggcaggagg atcgcttgag cccaggagtt     2880
cgagaccaga ccagcctggg aaacataatg agaccttgtc tctataaaaa taaaaataaa     2940
caattaaaaa aaaatttttt ttttgagaca gagtcttgct ccgtcaccca ggctaaagtg     3000
cagtggcgcg atcatggctc actgccagct ctgcctcccg ggttcacgcc attctcctgc     3060
ctcagcctct ggagtagctg ggactacagg cgcccgccac cacgcccagc taatttttg      3120
tatttttagt agagacaggg tttcactgtg ttagccagga tggtctcgat ctcctgacct     3180
tgtgatccac ccgccttggc ctcccaaagt gctgggatta taggcttgag ccaccgcacc     3240
cggcctaaaa atttttaaat ttaaaaaaag gggggacggg tgcagtggct catggctgta     3300
attccagcac tttgggaggc cgagacaggc agatcacttg aggccaggag ttcgagacca     3360
gcctggccaa catggtgaaa caccatctct actaaacata caaaaataaa aaaaaatagc     3420
cgggcatggt ggctcttgcc tgtaatccca gctactcagg aggctgaggc aggagaatcg     3480
cttgaacccg ggaggtggag gttgcagcaa gccaagatcg tgccactgca ctccagcctg     3540
ggagacagag tgagactcca tctcaaaaaa aaaaaaaaaa aaaaagaaag aacacagaag     3600
aggaagagga gaggggtttc tgaagaagga agatacctga gaaatgtcat ggggtaaaga     3660
ggggagaaag ctgggaaaga agccagaatc agggcggggt ctaaagaggg gcaggatgga     3720
gacctgcagg tgtggtctcc agggcaggag ggttgttagg ggcaggccca cctggctatg     3780
cttaccttcc actcctgccc ctagatccat ggaacccagt gacatgcctg ctggctacca     3840
ctgcccctta gactctgccc cctgggatga gaccagagac ccccagagca cagagctgat     3900
ccccaggaga gccatcagcc gctctccaac ctgcgcccgc tgccgcaacc atggtgtcac     3960
cgcccatctc aagggccaca agcgcctctg cctcttccag gcttgcgagt gtcacaaatg     4020
tgtcctcatc ctgtgagtat gaggtctggg aggggaggag gatgagcctc ctccaaaggg     4080
tggctgacct ttgacttgca actcccact tttagggagc gccgcagggt catggctgcc      4140
caggtggcct tgcgtaggca gcaggaggcg cagctaaaga agcacctgat gaggagaggg     4200
gaagcctctc ccaaagctcc caaccacttc agaaagggaa ccactcagcc acaggtcccc     4260
tgtgagtgtc tctgaccagc gatggggcag gagtttgggt acaggaggca ggtatgggaa     4320
aaagaggccc agtcctgctg ctgaattggt gtacgacctt gaactaaggg tttctactcc     4380
ctcggcctca gctgtcccag cgttgagaga atgcagcgag ttctgtgggt caaaaatggg     4440
ctagaggccg ggcacagtgg ctcacgcctg taatccgagc actttgggag gctgaggcag     4500
gcagaccacg aggtcaagag atcgagacca tcctggccaa catggtgaaa ccccgtctct     4560
actaaaaata caaaaattag ctgggcttgg tggcgcatgc ctgtagtccc agctactcgg     4620
gaagctgagg caggagaatc acttgaaccc aggaggcgga ggttgtagtg agccaagatc     4680
acgccactgc actccagcct gggcgacaca gcaagactcc atctcaaaaa aaaaaaatga     4740
gctagatggg ggagatgaga actgtctggg gttggggtag gatagggtc aggagaaagg      4800
gctcattaga gctctccctg gtccttcaca gctggaaagg agaacatagc accccagcct     4860
cagacccccc atggggcagt cctgctggca ccgacacccc ccgggaag                  4908
```

<210> 19
<211> 7563
<212> DNA

<213> Homo Sapiens

<400> 19

```
gccacggcgc tcggccaaca ttaaatctta agtcttaaaa ataatcctct ttggttccat      60
gtctcacttc cagacatact gatgtggtga acctgtctcc acagctttgg gtggccctgc     120
tcccagggca gcttgatacc tggcttgcat cctggcttcc tggggctaga gtcacacact     180
ggctgcttta ctgctctggg tcaagggagc tgacctaccc cagctctgct gagcgttggg     240
cttcacaggg actctctgtg gtgcctcaca ccctcggagg cttttgcact ctgcgccttt     300
ataagggcac taattccctt catgaaggct ctgttctcag aacctagtca cctcccaaag     360
gcccatatcc aaatactatc aattgaagat tagctttcaa tatgtaattt tttggggaga     420
caaaaacatt cagaccataa caaccctaat actatctata actacattta atatactatg     480
aactcaatac ttcaaagaca ttgtcagatt ggattaaaac aaatgttcta atgcttacat     540
gggacccaca ttaaatacaa agacatagaa agatttaaag taaaaagatt ttttaaaaaa     600
agctgttcca tgataatatt aatggaaaaa cagttaatgt agctgtaatg atatcaaaca     660
aaagtggcca catacagcaa gtaggaaatg gctgggcgcg gtggctcatg ccggtaatcc     720
cagcactttg ggaggccgag gagggcggat cacgaggtca ggagatcgag accatcctgg     780
ctaacatggt gaaaccccat ctctaataaa aatagaaaa aattagccag gcgtggtggc      840
aggcgcctgt agtcccagct actcaggagg ctgaggtagg agaatggcgt gaacccggga     900
ggtggggctt gcagtgagcc aagatcgttc cattgcactc cagcttgggc gacagagcga     960
gactctgtct caaaaaaaaa aaaaacaaaa aaagagaaaa aagaaataga aacctagtta    1020
aagatgttat aatgataaaa ggtaatatat acagataata ttataattat atgtgatata    1080
ttctatatta taatgttaaa tgtatgatca tttgataaaa tcaaataaca tgatgataaa    1140
atcattataa ttaggctatc aagatgaaag ggtaaattcc acaggaaaat atagcgatct    1200
taaactcgtg taccccttaat aaaacagcat caaattatgt agtgaaaatt ggccagtcat   1260
ggtggcttgt gcctgtaatc ccagcacttt gggaggccaa ggcaggcgga tcgcttgagg    1320
tcagaagttc gagaccagtc tggccaacat ggtgaaactc catctccaca aaaaatacaa    1380
aaaaaaagaa aattagctgg gcgtggtggt gtgcccctgt agtcccagct actcgggagg    1440
ctgaggcagg agaatcactt gaacctggga ggcacaggct cccaccacca cacttcagcc    1500
tgggcaacag agtgagtgag actctatctc aaaaaaaaaa aaaagtaaaa ttacgtagta    1560
caagttgaca gcatataatg aaagcgtaag tagagaaatt cacactcatt aattgaaatt    1620
cccccccccc gtcctttcag taactaatga gacagttaga taaaaatatc agcagaagcc    1680
aggcacggtg gctcacacat gtaatcccag cactttagga ggctgaggtt ggtggttcgc    1740
ttgagctcag gaactcaaga ccagcctggg caacatggtg agatcctgcc tctacgaaaa    1800
atacaaaaaa ttagcctggt gtggtgatgc gcacctgtaa tcccagctac taggggtgct    1860
gagaggggat gattacttgt gcccaggagg ttgaggctgc agtgagctgc aattgcacca    1920
ctgtacttca gcctgggtga caaagcaaga cccagtctca aaaaaaaaga aaaaaaatca    1980
atagagaaat ttgaacaaca tagtaagttt gactagttga catatgtaga gttttgcacc    2040
caacaagtgc aaaacaattt ttcaagtaca catgaaataa ttagaaaata actatatact    2100
ggggagtaag tttcaaaaaa tatctatgaa ttgaagtcat attgagtatg ttctcagttt    2160
acagtgtaat tcaactggaa gtcaacaata aaaaggctag aaaatctcca aacggttgaa    2220
gatgatcaac tatatacata catgtgtata tatacacata aacatatatg gacacacgtg    2280
tatacatacg tgtatataca cgtatataca catgtgcata tgtgtataca aacattcaca    2340
cgtgtataca cacgtgtata aactatatac atgtgcatat atacacacat acacacatgt    2400
gtgtatatat gtgtgtatat acacacgtgt gtatgtgtat atacacgtgt gcatatgtgt    2460
gtgtatatac acacaagtgt gcatatatgt gtgtatatac actgtgtatg tgtgtgtata    2520
tacacgtgca tatgtgtctg tatatacacg tgtgtatatg tgcgtgtata tatgcgtgtg    2580
tatatatgtg tgtgtatata cacgtgtgtg tatatatgtg tatgtataca cgtatgtgta    2640
tatagttgat catcttcaac cgtttggaga ttttctatta tatatatttt ttctttaaca    2700
ttatatggtg ttttatcata tagtgtttct tacggacata gcttaagaat catctgttat    2760
tttgtttcac agaaagtgtg aaactgcagg ggagccgatt tgtatagatc ctggtaaaaa    2820
gataattgag taggtaaatt ccagggagct tttggacagt ggatccccct gtcagtggtt    2880
ttggagtccc tagtttcagt tactcgtggt caactgtggt ctaaaaatat taaacagaaa    2940
attccaggaa taaacagttc ctaggttttc aattgtgttg acttctgatt ggcgtgatga    3000
gatccagcca tcttactcct tcccacagaa agtgagtcct ccctttgccc agcgtttccc    3060
tgctgtctac aatatccacc tgttagtcac ttcacaatct gggttattag atgactgtgg    3120
caatgaccag tgtggtgtct tcaagttgcc ttgattttac tgacttttg cccttatttc     3180
tctctggctc caaagtgcaa gagaagtgat gctggcaatt cagatatgcc aaagagaagc    3240
tgcaaatgcc tcctttaagt taaaaggtaa aaattctgga cttaataagg aaagaaaaaa    3300
aatcatatgc agaggttgcc aagatctact aaaaacaaat ctatccacga aaatgtgaag    3360
aagaaaaaag aaatgcatgc tagttttgct gttgcatcat ttttggatgg aagaacagaa    3420
```

acatgttccc attgaccgca atgggtacta tccatggttt caggcatcca ctggggctct 3480
tggaacctat cccccatagg tatggggggg ctactgtatt tctggcccca cagtcttcta 3540
ctgccgactt taggtctctc tggatctcag gcccccttct ctaagatgca tcctagagga 3600
ccaaaaatac actttatttg ggcttcgcct gcttttgtgg aagggtagtt tactagagga 3660
tataatctcg tgttttaatt tgctctctct cctaaaggaa atgtggagaa aaaaaaaaag 3720
cagaaattgg aaataaccaa tatttagttt atttcattcg attcttaggg gaactggtga 3780
ggagcctaag atgattttcc cttcctagag aaagaatcca aagtccaggg aaatagcgac 3840
aggggagttc aagactgccc ctgctagtcc ttccttggct actctccgct gcgatcgcag 3900
gatagctctc attagcagga gaatcgggca agtgtgtgga taagtagaga gtgtgttgaa 3960
caacttgtaa cgttttatga aatacgcatt gtcatggttc cctaaaaggc tttgcggaag 4020
ccgtttgtct ttactaatca agtctttact tacacaaaag tagaagtaga agtagtttta 4080
gaaaacatac taacaatctt ctatcccctt gaagaccaga gtagcagaaa acaggtgatt 4140
tgcattataa aattgcactc actttttcct cctttcagat ttcacattac attagcctat 4200
ttgtgttacg gtgtataaaa aatggaacag gcgcctccac tacattgttc tcctttaaaa 4260
atagatcact tacaccctaa ctttgttttc cttaaattcg attcttaaca ggagagcttt 4320
ctattatttc agatggagtg aggttgcacg actgggatgg aagaaaggaa tcccttaaat 4380
ttgggggaat ttctgttctc tgttctaaga ccattttact tggggtgtgg gggtgggcgc 4440
ggcggtcagg gcagtggaac gcagtcgcgg ctgcgccatc cctgcacttc caggcgcgcg 4500
ggagggaccg gcggggacgc gagctgcgga ctctggcgaa ctcgggggag gcagacaggg 4560
ggaggcggac acccagccgg caggcgtctc agcctccccg cagccggcgg gcttttctcc 4620
tgacagctcc aggaaaggca gaccccttcc ccagccagcc aggtaaggta aagactgctg 4680
ttgagcttgc tgttactgag ggcgcacaga ccctggggag accgaagctt gccactgcgg 4740
gattctgtgg ggtaacctgg gtctacggaa gtttcctgaa agaggggaga agggtttgca 4800
tttttcctat ggaggattct tctctctcta gcatttcgtt tgatgtattc aactggtaga 4860
agtgagattt caacaggtag cagagagcgc tcacgtggag gaggtttggg gcgccgcggc 4920
gccaccccca cccctcctcg ggaccgcgcc tatttctaaa gttacacgtc gacgaactaa 4980
cctatgcttt aaattcctct ttccagcccc gtgagtccgc ggcgacattg ggccgtgggg 5040
tggctgggaa cggtcccctc ctccggaaaa accagagaac ggcttggaga gctgaaacga 5100
gcgtccgcga gcaggtccgt gcagaaccgg gcttcaggac cgctgagctc cgtagggcgt 5160
ccttgggggga cgccaggtcg ccggctcctc tgccctcgtt gagatggaca acgcctcgtt 5220
ctcggagccc tggcccgcca acgcatcggg cccggacccg gcgctgagct gctccaacgc 5280
gtcgactctg gcgccgctgc cggcgccgct ggcggtggct gtaccagttg tctacgcggt 5340
gatctgcgcc gtgggtctgg cgggcaactc cgccgtgctg tacgtgttgc tgcgggcgcc 5400
ccgcatgaag accgtcacca acctgttcat cctcaacctg gccatcgccg acgagctctt 5460
cacgctggtg ctgcccatca acatcgccga cttcctgctg cggcagtggc ccttcgggga 5520
gctcatgtgc aagctcatcg tggctatcga ccagtacaac accttctcca gcctctactt 5580
cctcaccgtc atgagcgccg accgctacct ggtggtgttg gccactgcgg agtcgcgccg 5640
ggtggccggc cgcacctaca gcgccgcgcg cgcggtgagc ctggccgtgt gggggatcgt 5700
cacactcgtc gtgctgccct tcgcagtctt cgcccggcta gacgacgagc agggccggcg 5760
ccagtgcgtg ctagtctttc cgcagcccga ggccttctgg tggcgcgcga gccgcctcta 5820
cacgctcgtg ctgggcttcg ccatccccgt gtccaccatc tgtgtcctct ataccaccct 5880
gctgtgccgg ctgcatgcca tgcggctgga cagccacgcc aaggccctgg agcgcgccaa 5940
gaagcgggtg accttcctgg tggtggcaat cctggcggtg tgcctcctct gctggacgcc 6000
ctaccacctg agcaccgtgg tggcgctcac caccgacctc ccgcagacgc cgctggtcat 6060
cgctatctcc tacttcatca ccagcctgag ctacgccaac agctgcctca accccttcct 6120
ctacgccttc ctggacgcca gcttccgcag gaacctccgc cagctgataa cttgccgcgc 6180
ggcagcctga ctcccccagc gtccggctcc gcaactgccc gccactcctg gccagcgagg 6240
gaggagccgg cgccagagtg cgggaccaga caggccgcct aggcctcctg ggaaaccga 6300
ctcgcgcccc atacccgacc tagcagatcg gaagcgctgc gactgtgccc gcaggttgac 6360
cttgccaagc cctccaggtg atgcgcggcc atgccgggtg aggagaactg aggctgagat 6420
cgccacactg agggctccct aaagccgagg tggaggaaga ggagggtaga ggaggagggc 6480
ggtattgctg ggaaccgccc cctccctgcc ctgctccctg ctgccccacc cgagccctgg 6540
cagtctggaa cctggctgga gtcggaactc tccgcgcctt cgaagggagg cccgaggacg 6600
ctgccggcgc ccccaggcgt cggctccgcg cggcccctgc tgtagctggc gcacagcccc 6660
ggcgggtccc cttgtcctcc gcgcgctctg ggaagacggt caggcgcgcg cgtttggcgc 6720
ccacccctgt gagacccgct tggcctggcg ccgcgggcgg ggaccgctgc cgaggccgcc 6780
agcgcctctt caactgggga aaacacaaca actcgggatg ccccatcgga gtgtgtatac 6840
acctgtgtgt gtgtgtgtgt ggtgtgcgcg cgtttgcgga ggcatgaaat gcggggaaag 6900
aggctggcgc ttggcagtga ctgcctagaa tatgatacga atagtgatcc ttctttctgt 6960
ctgcccctac tccttttctt tgccttttcc ctttcctatc cgtgatctcc cctgaaaatg 7020
tcagactccc ggagcaagga acccggagta gcccgggcgc gcggttccgg cgccccctgg 7080

aggagccagc ggccgagtgc ttagcgtcgg aggatcgcgg ggctttgtgg tggagcactc 7140
cggctgagcg cgcttcacag cgctgtggca cactggtcat cgttgcttat agaggatgcc 7200
aagccccacc cccattcccc caattggcct cgtctcttgt cttacttaga tcagtttgtt 7260
gtaactgttc atgtgtgtac tgtttttcag gactgaaaac tttataaata atgtacatgt 7320
cctaatctca cgagttgcca tgaagatcaa ataagataaa aagaaatgcg atctattctc 7380
ttgtgttccc tttctcctct gcttccttgg ctccgcctta gggagagcag gaagcaggga 7440
tgaccgacgg cttcaggttt tctctgctgt acttttcaca gggagctgtg gattacctgg 7500
gctttattcc ttacttgtag acggcatccg gtaggtagtc ccggtaaacc gggtaaatac 7560
tgg 7563

<210> 20
<211> 5849
<212> DNA
<213> Homo Sapiens

<400> 20

attgcaatgg cacgatctcg gcttaccaca acctccgcct cccgggttga agcgattctc 60
ctgcctcagc ctcccaagta gctgggatta caggcatgca ccaccacact ccgctaattt 120
tgtattttta gtagagacgg ggtttctcca tgttggtcag gttggtctcg gacccccgac 180
ctccggtgat ccgcccgcct cgacctccca tagggctggg tttacaggcg tgaggcacta 240
cgcccggcca taatttttaa acatttttct gttggcacct gtccggacca tggatttaaa 300
tgatctacta acatgatgag aacccaacgg ggatacttca aaagagacaa atgaatatac 360
aggtggcccg aaatgatttt ataaagtcct ctaacaacca cacttctttc cagagtgcca 420
aagaatttgg ctcatttaag gtgttcattt ttcaaaaaat ggttatacaa atgcaaactt 480
cattcaaatc aaaccttacc ttgagcttgt taaatgccat gtttagcatc actcaaggct 540
tataccaatt gttgttttaa aaaattacat agtgctttaa gtatttttac atcttaaata 600
caaaggaata attctaactg tggaatcaga gctgtcttcc taagacaaaa agacaaagac 660
aaatatcaca attaactatt aactggagaa acaccttttc aaacgtccct gtaacaactg 720
gatgtgttgg acagcataac caaactcatt aagaatagtg tgaaaaacat caccattacc 780
tagttctcac acacagatac cttcttaaac tatcgaaacc tgtatctgaa tatacgaagc 840
attaagaatc tgcttttgaa aaggaaatga atatctgaag gtcattttct tttcaatctt 900
ttctcatact aataatcact gttaatatcc acttttaaaa aatcgcctct atcaggcacc 960
cgggaattgc ttaacaaaag ccacttaatg aaaccacagt tcagacaaaa aaaaaaactt 1020
aactgcatag aaaatattta tgagtttact ttcctaactc atacaattgc gtccttgttt 1080
ccaaagcctt gcaattcaca tcagcgggaa caaaaaaatc taacacacag cagaaagctg 1140
gctctctaaa agaaagcatt ttttttaaa ggcaatgaag gaccaaaaac tctttctttt 1200
aaaaagatct tagaagttcc tcctccagag aagacagaaa acactgacaa accgcacatt 1260
gtgcagatgt tcagccccga aatgcagcct cgcagccctt cactccgcag tccaccggtt 1320
gcatctgcac ctccgggcag cgctcctgca gggcgcaaac acccagagac ccagggaccc 1380
ggcagccccg cgcaggcctc ccgcgtgcac cgctcagcga gcgggtcttt cgcagacggc 1440
gggatgcccc aggagaccgc gaggccggcg cagcccggcg agctcccgga gggaaggcgg 1500
cgtgtcagaa aggatcggac cccagcaatg gccccggatc ggtcccgcgc ggtcgcctcc 1560
ttttcccgct ccgccgccgg gaccccccta gccagtgctg aatttcccaa ctgggaagga 1620
ccgagtcact ccaccctccg ggaagtcgcg cgctcatccg ttttcggcag cgccggagcc 1680
cgagccgggg accgggcgga cacccacaga gtccgcaccg cagcgcgctg cacgctctcg 1740
cccccgcccg ccggcccgcg ccccagcgaa ctaaccgcaa agttggcccg aagtggcgac 1800
ggcgctctcg tggagctcac ccgccgtctc agcgcgcccc cgagcaggga ccgggcctgc 1860
cggggccctc gccgggctcg cgcgtccgca tggtacctgc cgcacgagcc gctcccgcgc 1920
cctctccccg cgcccggccg cccagctcgg cgccggcgcc tcacgctccc cgagcgcagg 1980
agatccctcc gccccggccc cgcccgccgg cgccggcgct catttctcgg gcgcggaaac 2040
tcgagcccac tttccccggc tgccggctct gggaccgcgg gctcggccac gcagtcccgc 2100
ccggacccaa agagccccag tccctgcgaa caccaggtcc gtgctgggag acgagaaggc 2160
gcccagcggg gtctagcgcg gctccgaggg gcagggcgag gcttgtgatg ccgaggttag 2220
ttacggccag ccacaggcgc tgtgcaagga attggggaaa gctccctgcc aagaaaggga 2280
aacgttagct tagctaaaaa aaaaaaaaaa aaaaaaaggc tggggcctaa tgagaaagct 2340
tcccagaggc atccctttag cttttccata gccttctggg agcccctgaa tctgcatttg 2400
aatggcgcgg ctctgggtcc gcgaaggttt ggtgggctga agtcgtcctt caggtctcca 2460
aaggaatgca gctaggtcgg aatctacttc ctccttcaat ccccagaaaa tggaaacgct 2520
tcccccgcga ccttttctg ccgagttaga ccccaggccc tccccgccgc gggcgcctgg 2580
cctcccccgc tgacaccccg gacgcccagg cctgtgggac aaaaggcagt cgcggcgtcc 2640

```
cgcgggcttc ctagcccggg agtcggcccc aggggcccca aagcgtgacc ccaggccgac    2700
aactgcaagt tcctaggcag aggggaagcc ccagcgccga gaagcccgct ccgagaactc    2760
cccgcgccgg gcgcccagcc tgagcgtccc gggcgcggag cccatccccg gtgcgggcag    2820
ggctccagcc gcaggctcgg cgcagggcgg actcggccga agcccggagg gccggaggcg    2880
ctgggacgca gcccggacaa gagggcggac ttgatctcgg aaagtttgtg ggactcaggc    2940
attttaacac tgggaagaca aagagatccg atttccctgg gactggtgag gggagcttgg    3000
cggggagggg aacggcaggg cagtcaagtg tgaaagagaa tttcctgggt tcaaagttaa    3060
cgcagtcacc ttataagcca tcttagaggt gaccggaaaa aataataata ataaaggtgt    3120
ctagcaggtt gtggctggga aggaaaaata atgcctgcaa gatgagtgtg ccgattcgta    3180
gccctatatt ccctgcccac cagttggaat acacatagat tcacacatcc agcaaatgca    3240
aactccatca tccaaagacg tgtttgtccc ggccataatt gctccaagtg cttacaggtc    3300
aatttcatac atggaacacc tcgaaagtgg gtgacctgat ctccaagccc aaccggcttg    3360
tgccgcttgc cgaattgtaa atctcagcaa aacccaaaca caccggaact ccagccgttt    3420
cctgggagcg aggaagcggg agtaatgcta aaaggggaaa agaagaacag taaaaaaagt    3480
acaaagatac cattccctga cgtaaaatca cgggattttt tttaaatggc acaagagaat    3540
tcctggagaa gattaccatt aattaataag taataacgct tcctagggct tactagatgt    3600
tttacatctg tctgggaatc ccgattttcc aggctccctg agaaatctat ctgcctggaa    3660
gaagttgcac ttcgtcgcca caggagccgc cgagagccag ggatggagat ttcagactta    3720
ggactctgcc acgattctac tgatccttaa gcgccaacag aacgaaatgg acatgcctat    3780
tagctaccac aaatcgaatt attttatatg agagagagaa atgtggttca agttccacat    3840
ttgtccttta aataatcgac cctccctaac cctgtcccca aaggctcgtg gaaattaggg    3900
agggggttgg ggagacggtt ccagaaacaa aacgctttcc tccagcacgg cctctacatc    3960
cagtccaccc aagctgattt ttgtaactca tataaagaat gggcccgggt gggtggtcag    4020
tgaagactgg ggtgagcaag cgcccacgtc gccgagcgcc ggggaggtcc aggttcgcct    4080
ccgcacagaa ccctgcaagg agcgcgcctc gccaggtgcc cggactagag ggcgctgcgc    4140
ccctgcaaat ctcgcggccc cgatgcgccc tccctaggca ccattgctat aataagttac    4200
attcaaaata atgcacaaac agcatcgcct ctcaagttct ttttaacagt gctctatatg    4260
tattttatga actcccaaag ctatgcatac gaatctttca tcgccgatcc gcacaaagcc    4320
agagggatgc gcagctcctg gagccgagaa cccgtagggc gcacacgctt ccccgcactc    4380
gcttagagcc acacacccca cgtaatttca ttaacgttta aacttaaatg ccaaaaggca    4440
tgcacgattt gaacaaagaa ctcaccagtc tagccgagtc ctcggccctg ggctctaaag    4500
tcctcggcag ctccgtgatc ccctcgcggt gtagatatgt gttaagagcc gggtttgaag    4560
taccaacttt gcctccccgg gatcttcagc atgtccctaa ccagagtgcc aacgtccgtg    4620
ccgccgggac ccaggcggcc gcatctccgg aggcttccga ccagggcaaa cttaagcacc    4680
aaacgcaggc gaaaagagag tgcgcggggc cggaacgagg gcccgggagc agccaagttt    4740
gtcaggacgt gcccaggcag ccagccgccg agttgaggag ttgcggccgc cggctgcggc    4800
gactcgctgc ctctttcccc actccctccc ccttggaggc ggtgggggag gggaggtgac    4860
gtcagcgccc cgcctcgccc cccgccccgg gcgcggacgg gattctccgg ctgggcgcgc    4920
caccccgaca gtggcagcag cggccgtgga ggctgcgggg gtcccggcct ctgcggtctc    4980
tctggccggc cgcagctgtg gcgcgggtga aacgcccgcc ctcctccttc ggctgcgtct    5040
cccgggcgcc ggcgagcgcg ggcgttcctc gcactcagcg cctggagccg cctggagccc    5100
cgctgcgcgc acttccccgg ctcgcggagc cctccggcg gcgtttccca cgccgcggac    5160
tgcaggtgat gctcttcctc ttctgcagcc gaaattcgtc gcgatgtgtc gcctgccaga    5220
tttttgctgg gcggaagcat gaacagtctc tgcagactgg cctggacgac ttgtactttg    5280
tggccctcca acttctggat gtgaattttt tttcacgact tattattatg ctcttcgtgg    5340
tctttgtgtt gaaaatcacg gagctgtatt tttattttca aattctttcc caatgctcct    5400
cttgtcttga ccgtactctg aagcttataa ggttctgaga ggagggggcg aagggatagg    5460
ggaggagcca gttcctaatg tgtcttacaa gtcatcccta gtgatagggg ctaatacgca    5520
aatcactgtc tcctcctcga aataatatcc atttagataa atgcaaacac tgttgaggtt    5580
taatttataa agaatgcgtc gtctttatgt aagtgtccag cctccaggaa aatatctgct    5640
gtggaatata tttttaatgc cactaaagta ccaaaatacc tccccgaaca gcgtgaaatt    5700
ccgagggagc catttctgtg agttgacatt gtccccagaa gattgtcatt aacattcaag    5760
atccttgcaa aagggggctg ggggcggttc tatcatattt gcatccgttg gtataaatat    5820
gttgatcagt ttagctggtc gttcttta                                       5849
```

<210> 21
<211> 12201
<212> DNA
<213> Homo Sapiens

<400> 21

```
caataactgt gcagttatcg tttccgcacg ctgccaacaa ggcagacaga tgcagaggaa      60
acgtccttac aattttctgg aatgtagaca cgaagcgcaa attgaacctc tggcagatgg     120
cagaaaaggt gacttgacaa tgagtttgaa acaattaaag tgatattctc attcattcat     180
ttccacaatt ttattcattt cgaggaaagc ccagaggatt ggaacatgga tataaaggag     240
gacaacctaa gactgcaagt ttcatcttcc gaaaggctgc atttataagc aggctgccag     300
aggctgcgct ttctttctca ctctttgcat ccaggagcct ggaggatctt gctgcctacc     360
ccccgcccca cgccaacccc atgagacagg gacaccgccg ccccttgttt agtaatcctc     420
gggttgtctg atcactatcc tctaggccag cgattggatt cacctcttct ttcgcgtact     480
tacgggcaac cgaggccatc cgaggccctg gtcccgaacg aacttgagta gcgagagatt     540
cacaataaag aactaagtgt ggcctcccag gggctgcgct cgccatccag ggactgaaag     600
agacctgcgg gatgattttc tgttttcctc tagcaggaat cagaacaagg ggcgtagatt     660
cggttcaatt tttctgcttc ggtcaatggg aagatgaaat agaaaaattc tctttcactc     720
gcaacacgga gacttagggg gctgtctagc tagaaggttg gcaaactgca aaacggcggg     780
gcagggggcc aaggggacgg ttggggtctc tttgtttagc tcttctctca tcccaggact     840
caggctaggc taaatttcta gcgccgtgtg tgcttgaaag cagacaggtg ggattcttgg     900
aaagaacata cacaacacag cttctgagcc ccagaaggac cttaagacag gagttcctga     960
gccctgaaca agaggctacc tagggtaaag cgattgatct ccgcgcggtg cggctgagaa    1020
gagaaggcgt tgttgacctt tcggggagca agttaataac tcagccgagt tgatgaagtg    1080
acgttctttc tgaaaggggt cgtctcctgg gcccctttc tcagtcggca tttagcaggt     1140
agggaggcag ggcaaattct tctctccacg tgaagtcaca aagaattgat gagtttcaac    1200
agcatggatc tgaagtcgta gataattcta aacacacccg cgccagagtt cacactcttg    1260
ttcatctctg cggagttgga gcagacagcg accttcaaaa ttgtccctaa aatctctctc    1320
gaactgaccg ggcatttact gggatttgag gggtttttct tcagcttcca aactactgtt    1380
acatgcagaa tatactggta gatgccacca atgattgccc acctctcttt aaaagttgcc    1440
ccgtccacgc accattcagc ttacactaaa ctgtcccgac atgccttagc cgtcagttta    1500
attcataaag tagctgtaac tcttgacatc acctccgcag cccacccgag gactccttcc    1560
caggacacct tatgaggctg ttttccgagg gctgaattct gagacttaaa ggaaggttac    1620
ttgaggcatc ctggtatatt tccgggcttc caactccctc ccctgcttgg ctggtaattt    1680
tctgctgctc ctcctcctcc cccacttgac ctcctccgag gtaccttcgc ctcccctccc    1740
ccgctcgtcc ccagtaaatc tccagcgggt gggggaaatc aatcctttgg cgctaaagct    1800
aaactcagag ctgcccgttg gacaggattg cagtggggta aatggtgtcc gtcctttcag    1860
aaagaatgat caactacatg gaggtaactt gctctgggtt tcctgagatc tcgggcggac    1920
ccgtccgtgt tttgccagat tcagtgcaaa tgtagatttc agctgaaccg gtcaggactt    1980
gagagatagc tgtttcactc tccggactgc aaccacttcc gcgagaatct gatacattta    2040
atgaaagctt tgccaaatgt acgggtcagc gcgtccttgg gccaccacca cttgggtcag    2100
cacactgcac tcactagcgc caacctgggc tccacgcagc agaactcggt taaaaatcgt    2160
ctgttcctgg tcacttagga gttctagggg gcagggggaa gcaagaaaaa ggtttcactt    2220
gacactgcca gccgcttttg ataagtcata gtaaatactt tcagaagcac aattttaaag    2280
ggagaataca aatttccgtc ctcgcttggc cgaacgcctc cctgcaatca actgcggacc    2340
ttcgcatgct caggactgaa ctgaggtttg tggtttcgca gctgccccga tgccgggagc    2400
atgaaattct atctcggctg agaaccgagc aacagaccgc acaagaacgg tgccttctgg    2460
cctgtcgtct gccaagaaat accaggaaag ctggtgtcag aaacaactac ctgaactcag    2520
aaagtaaggc taggaagctc tgcgtgcaga gaaaattctt gctaattcac ccagactgtt    2580
ttatctggtt aataaagcaa ttgacactca acaaaaccac aatcgcagca ggagtcctag    2640
gcatcagaat aagaatcaca actaaaggtt tgttgttgtt aacaactgcc tttattatca    2700
ttgcttagta tttttttaag taaaaggaaa gaaaaagaga atcatttcat acccactggt    2760
atctcttcct tttctctgaa attagagcac agattctcct gattgttcct caataagcca    2820
ccaccaccaa cgggaagaga cgcaaattgg ttctaaacac ccacaccccg ttcgcggtgg    2880
aatataaatg gttaatcgta actcccttta aacgcacata attttatctg acccttggac    2940
atgattaaag atactgagga tttatatagc cttattctac atcattagag actacacaga    3000
aaagataaaa acgggaaagg ggatacatac gcatctactc aatatccatt tttagaggta    3060
aataaaatct tcctgtttca ttttggcccc caaattatat gatgtggaca tcagaaaata    3120
taaatccttc agttatacat attttttcat ctgaatacct tactcagtaa aactggcagt    3180
gtaacttatt ctactctttc aaacctgatt ctgatgtgag ctaaacatat agcatcatct    3240
ccctgggaat aaattctcaa atttatacct ccattggaaa gctgatcaaa gcccctcttc    3300
ccttcccagt cttggttaga tgagaaggaa taagctgtct tactgtttgt gatttcccaa    3360
agaaacaact taaaattcat ttatatctgc cagaggttat tgggagataa ataaatgcta    3420
aatgtgtaat tttacctttc aattgcaatt aaaacctatt tagatttttg aaatgtagct    3480
tgagagcatg atgtttgtgc aggaatctgt atctgacatt aagtttgatg tggaatgttt    3540
aaatggtaac taaagcagag aaagcaacag tacacaaaaa cacaagtcta agagcccatc    3600
```

aggaggttgg gtgtaacagc cacattttgg aaggtgtata caagggcaca tgggcagatg 3660
ccacagactc cagaggaact taaatttcag tatccctttt atattgaaag acactgtcag 3720
atatattgag gcagcctgga ggtgatgggg actacactta ataactactc atactcctca 3780
ttttctcagt aaaggaatca aaccagagat tcacactatt gccgttttca aggatatatt 3840
tttgacctaa caatatacaa aggtcaaaac gagtcctttt ttgagaggag agggtctaat 3900
ccccgctttg aacacaagag gtcgctcctg tgatcaagta ttacggataa attgctcatc 3960
agttttatgt atacaaaatt ttacagatat atttgatgca ctaaatccac atacagcaat 4020
cagctgagga aatcataata atccatgatc aatttagaac ttcaaggtaa agtaaaacta 4080
caaagcctgc tattctaact tggattttaa tgcaaaatgt gtcatgatac atgcactgaa 4140
cttcagagca tgtccatcat ggtggggtcc ctcaaacgtc tccttttaat gctggtatct 4200
tcaccatgcg tcttgcttct atcagtttag gaacttatta agaaaatact tgtttacaac 4260
aatggtgcag aatgaaaaga ggaaaatgtt gtcttcacca ttaaggtgaa attgtctatt 4320

actaaaggtt tcccccaagc cgcatgcaag cttttctact tggaagtgct atcatctgca 4380
ataattcttt gtaatagctt attggtttag ggagttaaaa taccacactg tgggtgggag 4440
gtgggggctg gtttgaagga ggatggatag tgagggtgct ttgtggatct caaatgtaag 4500
tcatcaggct tctggtggct tctggtgttt aattccatct tataaaagtg gcatgatgga 4560
atatgactag gtaaaactct attttctggc attgtggtgg tatctttttt tttttttttt 4620
tttactaggg tcaacatgtt tatctctatg ggaaacacac acaaatacta ggaagatttg 4680
cttcgtattg agattaacag catcagcttt gcaaaagcag cctgacaaaa ggctccatac 4740
atctaatacc agagactaga aacagaccaa aaaaaaaaaa aaaaaaaaac acacaccaca 4800
caactggtaa acttatcaac ctaggtctat ccaactttcg ctttcataca cgcgctctaa 4860
aacaacttat aataaaaatg gaagggaaga cagatccaat ttgaaatcct ccttgagaaa 4920
aaacaaatca aaactagttc ctggggaaga aagcctcagc taggtcagga ggaaacttac 4980
gcatctttga ttccctttcc gctttggaga ggcattaccc gttcaagccc agccaatgcg 5040
gccggccagg atttacagct cttatcaagg gttagatttt gcgaaagata taaagaaagg 5100
agtttcctaa cacaccgggt cttctcacag caacaagaca ccgaaattaa gccttctatg 5160
gtttgtgtct gtaggacttt ttagataaaa ctggcagccc cattagataa gaaatggctt 5220
tttagaagct ttgggggaag cgcgggggtt cttcgtggcc ttcctgtgac tgtctttggg 5280
agaccgtaac agatgatggg aacatgtaag aatgattgaa gaaggttacc ggcgagttgc 5340
acctaacagt cttctctctc caagtagttt ctcgagagaa aattatatat taacgcctgg 5400
ggacgtgaaa taggtctttc actgttctgt aataaatccc tccctactcg cgcccttaga 5460
ctaatgcatt tttaaaaaat ctccacaaag tcatggagac ttgtccttat aattaccaaa 5520
ataccacagc tagttgcaca agtttgctta aaaacaaagg gcaggagacc agcagtgaga 5580
agaattgaac agtatataaa aatcttacaa actttagact actgaaagat catttcgctc 5640
tttggcttcg ggttagtgag cttcaattct agagttaaac tgagaagcag acctcgcggg 5700
tctgaaagac aaaaagtcag tccgcggagt caacttcggt ttaccggtgc gttccaacct 5760
gattgtccca acagcaagaa gcgccccttt cctcccaccc aacgttttta gatatctgag 5820
gttgggggag acggagtaat ggtgtagcag tcaggaaaca gttcctctgg gtttaatcat 5880
cccattcccg attcttcttc ccctagcgcg ccgagcgcag ccagttctgg aggaactagc 5940
cctccggagc agccaaggca ggccaggccc cggggatgtg caccacggcg tggggaccaa 6000
accaagctga acggcctgct ccaagttccc cccttttcct agagcccggg agggacctcg 6060
tcaacaggta gacctaccca ccagccactc gccaccggcg gcagaaagtg agccccgcgc 6120
gcaacgcggc cagccggact gcggggaccc caggagcgca gggcggagga gcagcgccac 6180
aggaggcgag ggcgcaggcg gcgcggccgg gaaggaacgc gggaggggac agaaggaaga 6240
ggaagaggag gagagggagg ccagagccag aacagcccgg cagcccgagc ttcgggggag 6300
aacggcctga gccccgagca agttgcctcg ggagccctaa tcctctcccg ctggctcgcc 6360
gagcggtcag tggcgctcag cggcggcgag gctgaaatat gataatcaga acagctgcgc 6420
cgcgcgccct gcagccaatg ggcgcggcgc tcgcctgacg tccccgcgcg ctgcgtcaga 6480
ccaatggcga tggagctgag ttggagcaga gaagtttgag taagagataa ggaagagagg 6540
tgcccgagcc gcgccgagtc tgccgccgcc gcagcgcctc cgctccgcca actccgccgg 6600
cttaaattgg actcctagat ccgcgagggc gcggcgcagc cgagcagcgg ctctttcagc 6660
attggcaacc ccaggggcca atatttccca cttagccaca gctccagcat cctctctgtg 6720
ggctgttcac caactgtaca accaccattt cactgtggac attactccct cttacagata 6780
tgggagacat gggagatcca ccaaaaagta agaggctatt ttaccttgtg gggctcggtg 6840
tgctgttctt gtgcggggtt ctctctcagg cacaggctga ggtgccaagg gctctttgga 6900
gttggagtca ttgcctggag aaagagaaaa ggtggctttt tcttgttgcc gccacgcctg 6960
catgcttact gtcggttctt atcttcggga aactgattgt accttgtgtg tgaattcgcc 7020
tgtgtgccct ccaaagctct agctttctgg tgctaagcgg tgatttcctc ctggggaatc 7080
ctgagctctc cgagaaggtt attatgttgc aaaggtctgc ctgcacagtc aatgcccaga 7140
gatgtgaatt agcattagac ttgcaaaaga gaacgagtga caactgtatt tatgcctgct 7200

cttgctaaca atatccagtc ctgtgtgcta tttaagagcg cgcttcacgg aaaatataga 7260
catccctgcg ttcacttaac gcttctagtc aaaacctttt ctttgacttg acttatccat 7320
aatctttccc aatgattata gcaaagagga agggggggg gagaaataca aaatgagcgg 7380
gtttgattgc gtgctaggcg tacaaatgta gactattcca atctgcattt tacatatatt 7440
ccacctcctt ttaaaaatga gtcaaggttt tgatggcaca tttcaattac catcccaaag 7500
tgcaatgctc taaaaaaaaa aaaaaagaaa gaaagaaaga aagaaaaaaa cctcccagag 7560
tacgccctat aagagaacga cactaaaagt gtgtttatct ctgtaggaag taaacggtta 7620
gtcaatcatg tatttatttt catttcagaa aaacgtctga tttccctatg tgttggttgc 7680
ggcaatcaga ttcacgatca gtatattctg agggtttctc cggatttgga atggcatgcg 7740
gcatgtttga aatgtgcgga gtgtaatcag tatttggacg agagctgtac atgctttgtt 7800
agggatggga aaacctactg taaaagagat tatatcaggt atggcattta cacttctttc 7860
ttaattttgt gggatttccc tgaatctccc cactctttat gtattatttg gtgtggcttt 7920
gtctttttgt gaagtttgcc tcagtgtagt catacaagcc aaagttaccc tgtacatgtg 7980
ttaaaaaaat caagctatgc tgttcatttc attctttagt tgagaaaaac aaaaaccctt 8040
aacagtggta ttcataattc cggggtattg aggcttgttt aattactctt ggagtttatg 8100
atgcacaaat tattttcctc tttcaccctc cccttacaa aacaaaattt taaaaagatg 8160
gagaagtttg gatttttagc tttaaaatag ggttgatttt tgttgtatag tgcagtgttc 8220
tgtttgtttt agtccttttt aaaattagta gcttacaaat tctttggtgg catcaatgca 8280
ataggtgaaa taaaagtttg accgaagcat gtttagagat gtactttgaa agagcgagta 8340
caggtattgc tccttttatt tttggggtaa gacctccttc tgagaaaaat ttaaaaccaa 8400
cctaaatatt ccttggaaaa aacaccggaa acttaatctt tttaaatatt aaccctttgg 8460
tgacatctaa ctgtctcttc tttcttatct tatctgagct gatgaattag agcagatcaa 8520
attgcccatc atctgtctac gaacaattgg tatatttaga taattgaaca gcttcctttc 8580
tcacattaaa atctggtaac tgataaaatg agcgaattgt ccaaattgac aagactgaaa 8640
caacatagga actttctgag tttggttttg ttgttttgga gagtttttgt tttttttttc 8700
ctccaattta ttctgcaaca cgttttgcta atctcaagtt tcctctgact tgtgtgtatg 8760
tatcagaaac tttgttttct gccttagaaa gccagtagtc tctaaagaaa attgtattca 8820
ttttattaac aaacagaaga gacatcagca tcattattat gttaaataat agcaaaatat 8880
cactttttaa atgtccggtg gctattaaca agtaattaat tagcttttgt taggcaaatg 8940
gtttctggag cttgagaact tttattaaag tttagttaag atttaatata cagtcacagt 9000
ttgctcctgc tcacttagta tccagcattt ttttcttctt ttttaaaaat catgacacag 9060
agagtataat cttggtagat aaaattaacc tggttggggg aggttaatac ttcggagagg 9120
gagtgaaagg aagtaaggga agtcggggta caggaagggg gagggatttt ctaaattgtt 9180
tggtcaccgc caaagtcaag tcttcaccct atgaaatgga agatctcaca ttgagtaggc 9240
ggagggagga aaaacttttg agtccacctt ctaacctctg acaaatgagc gttttcattg 9300
tttactagat tggtgtgtaa acgcaagatt ctagagaagg agagcccact tcaggagtat 9360
ctttactgct atggaaatag tattttgctc aattgcacac aggcttgcat gtgcctaatt 9420
ctggatacac acatgtgtag aaggaactaa tcatttttac cttctcttca ctctctctca 9480
actctgtgtg tgtgtgtgtg tgtgtgtgtg tgtgtgtgtg taatcttgta gttgtaaaag 9540
cagaacagac tggacagtta gatttccaca tctctccttg gagaagcagg atgcctcctc 9600
ctgttatgtg gatcttttcc tctctcttcc attctttctg ttcgtaggaa tgccccagct 9660
tctgttattc ctgaaagatg gagaaggggc cagggaagtg cagcctagat ggaacctata 9720
aagattgtcc cttggtaagg aaaggccagg agtgagaaag accttagaag cgggtctttg 9780
catttttttc attctggtca tggttttcaa gaaaattgaa atgaggtaga tgattcagca 9840
acttgaaaaa gattgaggga acagacgcag attttttttaa aaaaataata atacaaggaa 9900
gaatggagag gaaattttct gttaacattg ctgcctgaag aaaatcttta gttggagaaa 9960
gactggaaag tacttgtgca aaaggagatg tggaaactct cagaggtttc attttgttat 10020
tctgcttgtt tatttgtgag tgtttgcaaa ccgagtgggg tgacaatccc cttctcctac 10080
ctcctttttt cttggaagga ggactttttg ttgcagtttt agacatttct agcagcagaa 10140
attgtgggat agggaagtga aagtgttggt gtcggtggcc accagagtct ttctggattc 10200
cttcctgcca agatctgcaa gatcaacact gggattgatt gctagagcag cagcccgagt 10260
ttggaaccca tcaatacatt ttctgtggta caagctaggt gttttgagct aagagttacc 10320
aactaagaca gaggttcatc ggaaaggaaa cgggagtaaa agaaagggag gagggaggga 10380
ggggaaaaga gagatggggg aaggaagaga gacagggaag gagagagcag ggtttcattt 10440
ctgtccttct gtttccaact tctgtttgga aatgctgttt acttggggcg tcttgcccgg 10500
gatcttgggc cagggaagtg ccggcctgaa gtgacccccct cttcctgtac ttctctcccc 10560
gctctgggcc gcctccgctc ccccctcccc cgcacaggtt gtacgggatc aaatgcgcca 10620
agtgcagcat cggcttcagc aagaacgact tcgtgatgcg tgcccgctcc aaggtgtatc 10680
acatcgagtg tttccgctgt gtggcctgca gccgccagct catccctggg gacgaatttg 10740
cgcttcggga ggacggtctc ttctgccgag cagaccacga tgtggtggag agggccagtc 10800
taggcgctgg cgacccgctc agtcccctgc atccagcgcg gccactgcaa atggcaggta 10860

```
ctcctctgcc cggctcgggt aggcaggcgc caggttaagc cagcctgtgt gccagcggcc   10920
acaacaacta tggtagctac aggggtggtc gtagtgtttg cctgcagtta aatgaagtgt   10980
tctgtatgca atttgcgctg tgctctgctc ctttgcagca aggttcaatg cactcactgt   11040
ctcccttgat tccccgagca cacctacacc gtctgtgtgt ctctatatgg ttacacataa   11100
atgtacacca cttgtgtaca cgtgtataca cacgcccaaa cattacttcc agttcgctct   11160
ggcctccaaa ccttggcttg ctgaaaacgg gcttcagctc ccagccaggt attctcctgc   11220
tgcctaatta aaggggcgga gccccgggtc cctggagctt catcctttaa cccaatgaag   11280
gaagcttagg tggcctgaag tcatttagtc tcccaaatcc tttttccttg tgagttgctt   11340
cacactcgaa attttttttt taattttttt atctttctgt gagagaacag gactgaaaag   11400
atacagtttt aaaaactgca ggccattgca cagagttgta atataaaact gtcaacaagc   11460
ttatctgcag taattgcctt ttaaagggag cctgcttctt taaatcattc attctatatg   11520
atttggtgag aatttcatct tcaggcccat ggttgtagct ctaaattgac cccataggtg   11580
ttggcctgac cctagggggt tgtagaaggt gcaggatttg tatcatgtag ataagaggac   11640
tcattcccaa ggaagaggag tggaaacaca gcaaggttgg ccgggaccaa agcagtgggt   11700
tagaaggtgg acagtgtttc caaacctgac ttcctgccat gaatagatct accccttttgc  11760
agttttaaag tatcaattcc cactaaacac tgaaggtgag gaaactatag ccctccctta   11820
cccttctgcc ttctggcagc tctaagaatt ctgttcaggg ggatttgtga ctagtttgca   11880
ccgggggcacg gctggggtgg tgctcctgtt cagtggagcc tgcactctgc ttgtggggaa  11940
gcacagagga agctaaaata ccgagaggga ggcgggggac atctcccagc caccgtttat   12000
ctagagccta ggcagctcaa cagagtttcc gttttccact gcttgggatc agcccatctc   12060
aggaacatcc atgtattacc ttagatttaa tactaagagc agggattgga gatatggcag   12120
aaatagcgaa tctcttcagc cccttcacat gactgtcctc tcggactgaa gttcaaggcg   12180
ttctggcaga gttctcgacc t                                              12201
```

<210> 22
<211> 22692
<212> DNA
<213> Homo Sapiens

<400> 22

```
caggcaaccg gtgaccaaaa acactttcag gcagtatcga gtgctaggaa aagggggctt      60
cggggaggtg agtgaacatt cacgttttta attgaaagtt cttacattca agtcaccttt     120
cctgtccctt ctaaatcaac ctaaagggtt ggcccacggg tccccccgggg gcaccagtgg    180
ctcaatgtgg gccccggggg cagtgagggt gggagagagt cagtggcctt ggggctatca     240
ggtgtgtcta tggtggaaag aggttgtgga gctgggcagg tgagacagac gtgctctcct     300
gccagcctca ggcaggatcc agcctgcaga ggacaaatgg gcagccctgg tcgtgtgtgg     360
ggaggagatg tgagggcgtg aggaggctgg gcaaccttgg ccactcagtg cctggatgac     420
gctgtcatgc aagattcgag atttcgttgt acattacccg atcttcccag catgtgaggc     480
aagccttgtt attcccattt tagagatgag gaaagctgag gtccagaatg gttaggtgac     540
ccagccaagg gcgcgcagac aatgtgaggc agagctaaga cttaagccca aatgctttgg     600
ttcccgctta tctggattat gaagtcagtg ttcttgagga tctcctatca ctgcctccca     660
aaacaaactt ctctaccggc agccggtagg agaaaatcct gttgccctgg gaatggaacg     720
gtacttgggg ccccagtcag ttatctaagc cttggtgtga gtggctcatt ctgccccttc     780
cagggctggg aatccttgaa ggagaggagg agggagggca agggcccagg acagctgtgg     840
gccccaggcc aggcatcact gggtcctggg agcctggagc actcatgaag ccaggcaggg     900
ccggctctga ccccatccat tctctacctg ggaggcctgt ggtccccgcc ctggaggagc     960
tcggggcagg cctccacggt gctcctgcca ccctggtttc tttcttgcac tgcaggtctg    1020
tgcctgccag gttcgggcca cgggtaaaat gtatgcctgc aagcgcttgg agaagaagag    1080
gatcaaaaag aggaaagggg agtccatggc cctcaatgag aagcagatcc tcgagaaggt    1140
caacagtcag tttgtggtga gtgagcatct gggcccagtg accggctcgc ccttctgtgg    1200
actggggctt ccctccctcc ggaagggcgt ggtcctctaa tgcggccggt ccccacccct    1260
gggaagggga atgccagtgg cagcgctgag ctacagaaag gccgcaagac attcctccat    1320
cacacggccc attgtggtcg actgtggctg gctttgtccc atccccagag ccggccagtg    1380
cctggctccc tgctgtgcat gagaccgacg cctctgttct cctggaccac tttgtaaact    1440
cctcagtgga caaagaaagt tcacctgggc ccaggctggg agctgtgggt tccacagacc    1500
ctctaggctg gtgccatcgt ggtggggacc ttagtgccag catcctggaa tggcactatc    1560
aagtctgtgt gcattgtgtt agtcagtcta ggctgggccc tgctgcagta acacatttgc    1620
cccaaaccct aagtagctaa atacagcaga ggttcatctg tcactcacct gaagtccaat    1680
cagattgggc caccctcctc catctaacag ctgccccatc tggagccttg aaagtgagtg    1740
ctaggggttc cacacagaga ctttccactg cctctactgg acagatgttc ctcagtttca    1800
```

```
cccacagtct gccagccaga accagtcaac acagagacaa gttagtttca ggggaggctg    1860
ggaaatgtga agtgcatgga tatttggtca gctctaatat cttctgctac acactgttat    1920
aaaaaataaa gctgggtgca gtggtacatg cttgtagtcc cagctactca gaaggctgag    1980
gcaggaggat tgcttgaacc caggatttcg aggctgcagt gaactatgat cgtgccactg    2040
caccccagcc tgggtgacac agcaagacct catctctaaa actaaataca tgcatacatt    2100
aaaaataata tatacacaag gtaaaaattt aaacagcaca gaaggataca caataaaacg    2160
aaaagcttcc ctctttgagt aatcactcca ggcatgtttt attcatgcac aggctttatt    2220
attcttagcc aatgggatct tcctgtacct cttatggagg aatgtttttc ccttacattg    2280
tagatctttt tctccatcca gacctctagt cacacccctt tttaaagttg ggcatcattc    2340
tcctgaagag ataaagcata atccatatag tcagtcccca tggatggaaa ttgttttcca    2400
tctttaaacc actacagggc cgggcacggt ggctcacgcc tgtaatctca gcactttggg    2460
aggtcgaggc agacagatca cttgagatca ggagttcaag atcaacctgg ccaacatggc    2520
aaaaccccat ttctactaaa aatacaaaaa ttacctgggc atggtggtgg gcgcctgtaa    2580
ttccagctac ttgggaggct gaggcatgag aatcacttga acctgggagg cagagattgc    2640
agtgagctga gatcatgcca ctgcactcca gcctgggtga cagagtgaga ctttgttaaa    2700
aacaaaacaa aacaaacaaa caaaaccact acagaccagt ggcactccca gaggatggaa    2760
gtttgcatct tgggatctca tcccaggcca cccccagctg ccagccttgt cgggggtgca    2820
ggtgtgggca gtgccatggt atgtggagat gaggcagatg aggctaactt cagccatcat    2880
ttcagctgcg ggccttcttg agtccgaaac cttcctcccc actgccagcc gcttcagcag    2940
ggacccctgg atgctgccta tggagccttg tctctgaagg gacagtgctg gcgggttctg    3000
ctcagggaag aggatcatgt gattcacaaa gctgggtggc acgtctttaa gggccttcag    3060
ttacgatggc tgcaccttct ttaactgtgc cccatccacg gccccggcag ggagttccca    3120
cgatgtcacc tcctggatcc ctttctcggt gatcacctgg gcagatgtgg gagaaagggg    3180
acagctgcag ccattgcagc agaagcacag tccccatgag cttgctcatc cttggctgcc    3240
attttatctc ctttcctcaa aaatagctga tgttgatgca ccagttttac ggcacagcca    3300
gttcccagcc tggcccacga tggccgggtt tcatgtgtgc cacctcccta tgtggagaag    3360
gattaccttc tggtgtaatt tgcccctcct gccccaccat ggccttgctt cacagagcct    3420
tgggtgtctg gactccacca gcagaggact agacccccctc tcttggtggc cctttgccaa    3480
gtggaccagt gtattagtcc attttcacac tgctgattaa gatataactg agactgagca    3540
atttacaaaa gaaagaggtt tgatggactt atggttccat gtggctgggg aggcctcaca    3600
atcatggtgg aatgtgaaag gcacttctta catggcggtg gcaagagaag aatgagaacc    3660
aagtgaaagg ggtttcccct tataaaacca tcagatctca tgagacttat tcactactat    3720
gagaacagta taggagaaac tgcccacaat tcaattatct cccaccgggt ccctcccaca    3780
acacatagga attatgggag tacaattcaa gatgagattt gggtggggac acagcccaac    3840
catatcattc tgcccctggc cctcccaaa tctcatgacc tcacatttca aaaccaatca    3900
tgccttccca acagtccccc aaaatcttaa ctcatttcag cattaactca aaagtccata    3960
catagtccaa agtcccatct gagacaaagc aagtcctttc cgcctacgag cccgtaaaat    4020
caaaagcaag ttagttactt cctagataca atgtgggtac aggcattggg taaatacagc    4080
tgttccaaat gggtgaaatt ggccaaaaca aaggggctac aggccccatg caagtcagaa    4140
atccagcagg gcagtcaaat cttaaagctc taaaatgatc tctttgacct catgtctcac    4200
atccagatca tgccgatgcc aagacgtggg ttcccacggt cttgggcagc tccatccctg    4260
tggctttgca gggtaccgtc tccctcctgg ctgctttcat gggctggcat tgagtgtctg    4320
caggttttcc aggcaaacag tgcaagctgt cagtggatct accattctgg ggtctggagg    4380
acagtggccc tcttctgaca gctccaatag gcaatgcccc agtagggact ctgtgtgggg    4440
tctctgaccc cacatttccc ttctgcactg ccctagcaga gggtctccat gagggccccg    4500
cccctgcagc aaacttctac ctgagcattc aggtgttttc acacatcttc tgaaatctag    4560
gcggaggttc ccaaacctca attcttgact tctgtgcact cgcaggctca acaccacatg    4620
gaagctgcca aggcttgggg cttccaccat ggctcgagca ctgcattggc ccctttcagc    4680
cacggatgga gcagctggga ctcagggcac tgggcaccaa gtcccttggc tgcacacagc    4740
acggggaccc tggactcagc ccgtgaaacc actttttcct cctaggcccc tgggcctgtg    4800
atgggagggg ctgctgtgaa gacctctgac atgccctgta gacattttcc ccatggtctt    4860
ggggattaac attcagctcc ttgttactta tgcaaattta ggcagccagc ctgaatttct    4920
cctcagaaaa tgggattttc ttttctattg cattgtcagg ctgcaaactt tccaaacctt    4980
tatgctctcc ttcccttaca aaactgaatg cctttaacag cacctatgtc accccttgag    5040
tgctttgttg cttagaaatt tcttctgtga gataccctaa atcatctctc tcaagttcaa    5100
agtttcaaaa atctctaggg caggggcaaa atgccagcag tttctttgct aaaagataac    5160
aagagtcacc tttgctccag ttcccaacaa gttcctcgtt tccatctgag accacctcag    5220
cctggacttt attatccata tcactttcag cattttgggc aaagccattc aacaagtctc    5280
taggaagttc caaactttct cacattttcc tatcttctga gcccttcaaa ctcttccaac    5340
ctctacctgt taccggttcc aaagtcatgt ctaccttttt gggtattttt ttcagcagtg    5400
cctcactctt ctggtaccaa tttactctat tagtccgttt tcacactgct gataaagaca    5460
```

tacctgagac tgggcaattt acaaaagaaa gaggtttaat ggacttacag ttccacgtgg 5520
ctggggaggc cccacaatca tggcagaagg tgaaaggcac ttcttacatg gcggtagcaa 5580
gagaagaatg agaaccaagt gaaatgggct tccccttata aaaccatcag gtctgtgaga 5640
catattcact accacaagag cagtatggga gaaactgccc ccatgagtca attgtctccc 5700
actggtccct cccacaacac atgggaatta tgggagtaca attcaagatg agatttgggt 5760
ggggacacag agccaaacca tatcaactag gcacatcctg catgcaagag aaagccttta 5820
ccaggccatg ttccacttcc caaagcccca ctgcccctcc agctcaccct tacttggccc 5880
tgtgagaatg gctcccatac ccaaaagggc aagtgagccc tgcttttctg actggccaag 5940
aaggcaccct ccagtcttcc ccagctgatg cacacatgtg cttctgtgga ggaagcacag 6000
acgccattct gttggattca ctgggaggtc ggggtgctag cctgccctaa cccgccttgg 6060
ccttctcacc ttgatccact gaaagggagg agcaggtgtg agccccttat cagccaagga 6120
ggccgcaggc agggtcaccg cagaggccat ctgggtgcag gggagcaggg tgaggctcct 6180
ctgttccctc acaccccagc tcccaggatc ctgaggaggg aagtggaagt gagtggccat 6240
tgtcacaacc tccccatggc actgttcttg tgctcccagg tcaacctggc ctatgcctac 6300
gagaccaagg atgcactgtg cttggtcctg accatcatga atgggggtga cctgaagttc 6360

cacatctaca acatgggcaa ccctggcttc gaggaggagc gggccttgtt ttatgcggca 6420
gagatcctct gcggcttaga agacctccac cgtgagaaca ccgtctaccg gtgagtggaa 6480
ggcaccaagg acttccaagt ctaagtccga gggggtgggg ccagcccctc cacaccctcg 6540
ggcaggtggt tgccatcgct ctgggaatca gccctggtca gcaccagggg aggatgcaga 6600
gtcagacaga ctttccactc caggaagctg gggcctccgg cactgaggaa gcgaggtggg 6660
ccaccctctg gccagtgttc tccagcctcc aaagtttagg gaagccaagc aagtgaaatg 6720
atgttcctga aactgactgc tccttcaagt caaggaggac gggccgtgac cactgctccc 6780
tccagttgcc tccatcccag cagccaggga agctgggagg gggcctggtg gccgctatct 6840
gggatcagtc ctgggaagac ttgggtcggg tcactgccca caagcagaat cggagtggcc 6900
catggtccca ctccatgctg tcctcagctt gctctggtct tcaccccctc tccccaccca 6960
cctcaatccc acagggacct ggacattgta gggccagggg tccagattcc actcacaatg 7020
agcagtgccc ttcagatttt tctcccagtc cttctgaaag aattttgaaa aatctctgtg 7080
atgctgcatt ttaagtagac atttgaaata ttccatcata ttgaaatagt ggcaattttt 7140
gatatattga aattgaacag ttacattaca ctcctcaggg gatccagtgg aatctctagt 7200
tgctgtagcc aattgatttc cacctgcatt tatccaaaaa atgcacagaa agctctttag 7260
tgatgaggaa ttttctatca tttccttcac tctctccatc cccccagaat gttaataata 7320
tattttttg cctgaaagtc tttcactgat tagcgtacat ctctgaaaca aatatataga 7380
tacatacaag agtttttgaa gttctccttg accgtaagtc tctaagtgtt aaaataactt 7440
ctctggccgg ttgtggtggt tcacacctgt aatcccagca ctttgggagg ctggggcagg 7500
cggatcacct gaggtcagtg gtttgagatc agcctggcca acgtggtgaa accctgtctc 7560
tactaaaaat acaaaaagta gccggatgcg gtggcacgcg cctgtagtcc cagatacttg 7620
ggaggctgag gctaaagaat cgctttaacc tggaggttgc agtgagtcaa gatcaccact 7680
gcactccagc ctgggcaaca gagtgagact ccgtctcaaa ataaaataaa ataacttctt 7740
ctgtcgagtt gccattgtaa ttagtagtag atctcatttc taatcagtcc tttggaagca 7800
ggggttttga catctggagt gggtcgctac agtcatactc gggaggggag gaggctgctt 7860
ttcttctgta acttgggaga agagttattg agaaagggca gatgggaggg agatcccaaa 7920
agatgccagg acagagcatc ctgatgtgaa gaaatccaaa cccttcaaac tgcggacagg 7980
agggaacccc ctgctcggtc ctggcagacc ccgacacgca gcccagtttg aagacctttg 8040
cagtggagct catcttggga ccaagaaggt tttgaaaaca agaaagaatg gtcttgatga 8100
catgcccttc cccccatacc atccccccat ttatgtttct gttttgtttt cattcaaatg 8160
aaaagagtca aaattttccc taagggttga acaaagaatt ctctacgtga ttgcctttta 8220
tttaaaaaga aaaaagaaag ccctccaagt tccagctcgt cttcttccgg gggaaagaga 8280
accacagtgc ctcctccctg gctgtgacca ctcaccacgg cctctctttg ggctccgttc 8340
tggccagtgc ccactagtga gctgccatgc tagcctggtg gcagccggct tgttgagatg 8400
atggctgctg ccttatttg gaattgttaa cacatcaaaa gaatgccatt taaataacag 8460
caacaaaagt attttatgat aaataaggat acacaaacac caattatcat ttgatgaggc 8520
tcactggctg gatttgggta cattaaccac tccgcatgag gacagctgcc atcagttaaa 8580
taaacaaagc tgggtggttg agaactggag cctccatcca gatcctgtgt actgtttgcc 8640
ccatggctta actgagttgc acacactctc ctcttggctg ttcttttacc catagagaag 8700
accttttcct ataaaagcag ctcctctgct ggctctcccc tcatcctggg aatgcagagg 8760
ctcccttact tgtgagcaag atcctgtcat caagagagga gacattaggg acaggataag 8820
gcagccacac ctatgccatg ccccgtagct gttctttctc ccaggctgta gcgtagctgg 8880
tgttggggga tatgccagtc tcctgcaggc cccatcccaa agggggtctt tttcctgtgt 8940
ccccaccttc tcccctttga tcctctcttc ccaattgcac agctcccttc attccaggcc 9000
ctgttctgcc ttcagggctt cctgagacct gcagggtctt ctcagccttg gagtatcctc 9060

```
agacctgcag tcattgatgt gtgaacaggg gccatgactg gccttttcct aactttccca     9120
gtcaccctcc aggattggga aatgcctgtg gcaaacggca gtgttgcttt aaattactgg     9180
aaggagaaaa attagctgaa gatcattttg actcaaacca gacagcctga gccaggaggt     9240
gggaaggaaa cacactgtgg ccactcaggc ctgattagcc cgaggggtcg acccagatgc     9300
cagtgtatcc tacctgccca gaatgcccac actctgatgc ttgttgtttt tcctttcaga     9360
gatctgaaac ctgaaaacat cctgttagat gattatggta agtcttttcc tactcggtag     9420
ctgaggtgag cattgcaacc ccaagaaagc aaagggcctc ccagggattc tcagagaggg     9480
ctggggaagc tgaccacggg ccccactccc actgctcagt gctgggtacc tggggaaggg     9540
ggaaagctgt gagtgtgaac agagctccgc ttggtgaaca ggcagtgttc agagagactt     9600
tgaaattcag agtttttgaa attccctttg accataaggc tctaagtgtt aaaataactt     9660
ctggccgggc gtggtctcta cagtgcactg aacaaggagt ccagaatttc agagatgctg     9720
ggcaaatact taacttccat caagttctcc acctctcatc ttgtttgtaa aatggaaaca     9780
aagcgtcctg ccctgccctg cccacccaga aggaagaggg tggtggagag gcttctacat     9840
gacagcttga gagggaattg cttgtcagtc tagccagctg taaatgtctc aggcattacg     9900
tttgtgcatt accatcatta ttttctcttg gctgtggaca ctgaataata agaacaacca     9960
cagtacccct cacagctgtt tagcctcaca cctggaaaat agttcctata ccttcttttt    10020
tttttttga gacagagtct cacttgctca ggctagagtg caatggcgtg atctcggcct    10080
actgcaacct ctgcctccca ggttcaagtg attctcctgc ctcagcctcc caagtagctg    10140
ggattacagg tgcacgccac cacacctggc tagtttttgt attttttttt tttttgaga    10200
tgtagtttta ctcttgttgc ccaggctgga gtgcagtggt gcaatctcaa ctcagtgcaa    10260
cctctacctc ccaagttcaa gtgattctgc tgcctcagcc tcccaagtag ctgagattac    10320
aggcacgtgc caccacgccc ggctaatttt tgtatctttg gtagaaatgg ggttttgcca    10380
tgttgaccag gctggtctca gactcctgac ctcaagtgat ctgccctcct tggcctccct    10440
atgtgctggg attacaggca tgagccaccg gtttccacac ctttgatctg actagatctt    10500
cccacctgca cagagaaggg caaggcagca tctctgtgcc catttaacag atgacaaaac    10560
tgagcctcag gggagccatc ctgccaaagt cacccagcag catcagtggg gagctgaggg    10620
cagaaactgg gtctcctccc accaggcccc agaacctggc agccccccagt agagggaggg    10680
gagaaaccgg gtcgctgatg acctgaagag aggtctgcat gggatactgc cctgtggagg    10740
ggcggtcgga gggcccaggc ctgccgcccc tctctggatg tgcccggtgc ctcccttggt    10800
ctaagaacca agcccacgct gctcctctgc tatcctgact gtcagccccc accgagccag    10860
ggagtccaca cggctccagc ctggcggggt ctcgacactg gcccttgctg acatctaggc    10920
tggagggatg ggggtggggc cctggtgtcc ataggcaggg aagggaaggg gacgtgttcc    10980
ttcctgtggg gtaagttgtg gccgcgaccc tgcccaggtg ggctgtggga gggacaaaga    11040
ccaaagtgga gcctcagctc agcgtctccc gctctggggg ctcagtcctg ggctgtgagc    11100
tgggattcat ctccaggctc tgccaggtca gaccagatgt ttcccttctc cgtttctcta    11160
aagtgaaaaa caaccaatct ccagagacca acaaggcagg ccggggagct gcttcccaaa    11220
tcgttttata attttttccc cttaagcaaa ttgttgcata atgccagaag aacctcacat    11280
ttttcccatg aagtaatccc agaaccaagc caagattctc tgacctcagc gttccaggaa    11340
gcccattcag cctttgacta ggcaggtcgg tcaacgtcca gccgggtcca ggcatcctat    11400
tgcatcttta tgttccactc ctgaagagtg ggcattttcc tgtgtcctcc cacacctgca    11460
ctctcccaag gctcttgtgg tcactctgag atggcagatt ggggtccctg ttgtccttgg    11520
acagatgaga atgccgagag ctcgtatgcc ttgcccaagg gcacacagca aggccgggtg    11580
cccatgcggc tgtcccaggg acccactgac cctgctgtcc ccctcaggcc acattaggat    11640
ctcagacctg ggcttggctg tgaagatccc cgagggagac ctgatccgcg gccgggtggg    11700
cactgttggc tacatgggtg agtgctgggc tgcctgtgtc aatgcacctt gagacccacc    11760
acctgctcac cgccggccga gcccccagag cctgcccgca gagcctctcg cctcttcatg    11820
cactgctgat cacacacaga gtcacagtct ctgcagggct gctgggggc ggccttagcc    11880
agggggaggg ggagcacagg ggccccagat gtgatgactg ctctgagctc tctgcactcc    11940
caggccccca gccccacctg tgtccaaagc caagccactc ttaggggtcg gggcctggca    12000
tacagcgctg ctaggggagg ccacattgaa gcaggggtct gggaggagag gcctttgaga    12060
ggaaggaggt aattcacaag agcccgcaaa tgagaggcgc gcacagctgc tgtgtcacca    12120
agcatgattc cagtagagag cagccttggg ctcctgccac ctttcctcag tctgctctga    12180
gaccagcgtc ctgggggttg cagggaagag acccagagtt aagtcctgtg ccctagaatc    12240
ccccgtccaa ttgggggcac gccctgggct tcctcctgtg catttcacac acctaccctc    12300
cttcccctga gaggcgcgtg cccggggagc aggcacgctg ccgccctcta gtcaggctgg    12360
tactgacagc agctgcacag gaggtgtgcc ttgtctctgg tgctagaggg tccttttcag    12420
attgcatgac atttggcaac ctgtggagtg tgtgtctgtg tgtatgtgag tgtgtgtgtg    12480
cccacatgaa tgctgatcga tgattgatag gtaggcgggt gggcgggtgg atggatggtt    12540
ggatggatga tggatgatgg atgggtggat gatagatctg cctacctgtg tgtgcatttg    12600
cctgggtctc cttttgcaga gtgttccatg catctgtctg ccccggctct tgtgtgcaaa    12660
ccagcacatg tgtggatgca tttccctcca gacatacgtg ctgatgctgc agaactatct    12720
```

```
tttctgtatt tctatggtaa atctgtaatt cggtttatca aagtggtaca gaagacatag   12780
caagagattg tttgaattac agataaattt aagatcatct ttaatttggg tagatacacc   12840
tttattcatt gaaacaaata tactgatagc acctgttctg tgccaggtgt catattgtta   12900
cggtagtgaa taaagtagct catgttctag gcgaggggac atcaagagtg caggacacct   12960
ccatccacgg tggtcagagt tctaaagcta cagtcaaagt ccttcggggg cacagaggag   13020
agtcatcagg gcaagctccc tggagggggt ggtattcaca gcagttggtg gggtaagagt   13080
tggtggcggc catgaaactc caggccttct gtgagcagcg ccaccagctg tctccctcct   13140
cctcactcct ctctcctctc ctctgccccc agctccagag gtcctgaaca accagaggta   13200
cggcctgagc cccgactact ggggccttgg ctgcctcatc tatgagatga tcgagggcca   13260
gtcgccgttc cgcggccgca aggagaaggt gaagcgggag gaggtggacc gccgggtcct   13320
ggagacggag gaggtgtact cccacaagtt ctccgaggag gccaagtcca tctgcaagat   13380
ggtgagctcc tggtggccag atgccaccct caagctggtg gctcccctcc ctgggcctgg   13440
ccccagtctg tccccagaac agcaaacagg ctgaagggac aggggtctga gcatggggggt   13500
gggggttgca gcccaccaag ctagagttga gggcactcct tgctgtggac tggggtggtc   13560
agcggagact tccaggagaa agcctgggtg gggcagggac acccaggaac caagggaaga   13620
ggggtgaggg gaacagtgtg gaacaccctc acatccagcc tgtggggggg tccacacagg   13680
gcggagaaga gatggtctga gctggtgccc cagacacaaa gcaaccttgg gccaagcccc   13740
ggcccctgcc cgtgggcctc ctgctccctc cacctctgga tcctaaggtg ttggtttaag   13800
acgaagggct acagggggtg ggggcgacga tttcagtctt tgaacatctt ataacaaagt   13860
tttccaccac tagatggact gctggtcacg ccagcctggc tcctgcatga cagggtctag   13920
ggatagtgag gtcagaccca cccaaaagct gggtgggtgg ggccagatgg ggggactgga   13980
agccagataa ggagtgacag gagccattgg aagtggggga tatgggagtg gagtccgccc   14040
gtgcacccct gccactgggc cgccccatgc cggccctcag gcccttccct gggaaccagg   14100
actagtggct ggctttgtgg ggagatggcc acttcctgct gctgcctggc tctcagcccc   14160
caagtccagc ccatgccccc ttggggccct cctccttatg gtccccagca cagctgtgct   14220
ggcaccccca ctgcaagcag ctgccctcta atgccagcct catagggaca ggagggggccg   14280
gagcacccag ctccacaatt gccagcccac agacttcctc tcctctccct ggagccacag   14340
atgtaccccg tatcccatgt acccggagcc acagatgggc aaaccgagcc tgggaggggg   14400
gagccacact tcccacccct ctacatctgc aggcactttt ggggcagatt cagcgtgtgc   14460
attgacaacc caggcaactc ccggagttta ggaagcagca gtcctgggag ttgattaaaa   14520
gtcgcaatgc ttctgctcag gaagacctga gatctgcgcc gcaagcggcc ccaggagggt   14580
ctggctcggc tcctcacccc agaggcccct ctcctgcagc cagacacctc tgcttggccc   14640
cttcctgtca ccaacctcat cctcacatca cagagctgtg ggaagaagtc ctgtgtgtac   14700
agcagaccca cactctaacc ccacagctgt tggcccgcca tgcagctggg gcctcagaga   14760
aagccacatg gtcatcgcag ccagggctag ggtgggggga ccggaggagc agtgcagcac   14820
tggtcgggta aatggaaatg tgggtgtgag ccatacttgg ctgtctcatt gggagatatc   14880
tgtcaggtgc gacaaaacga gacaagggaa agaagaaggg gtcagacagc cgtgggcaga   14940
gcatcaccaa atgtcccagg tcagggccag gcaatcagag aagcaggtct tgagagatct   15000
cagcagggat atgggacgta ctaagaggat gacgaggcgg agagggcccc agtcccaccc   15060
ccagacccca gacacccacc ttggcccctg gctttggagc tgggatctcc tgctgcagcc   15120
tggaccaggg tgctgctgtc cctctgctgc cccctggtgg acactgagag aaagcctgct   15180
gctcccggag gacctgtccc gtgggtacaa aaccactgtc ctctggaagc ctctggcctt   15240
gctgcggctt cccttacaaa tgatctttgg tcttgggagt ctagtcttag gcctccttca   15300
cgcccttgac tgcagcatct ccggcatcta ggcctacctt gtcccctcca gctggtggca   15360
gctcaggaca ctgcagagcc tgacttgcca ggagacctcg caggtcctgg gttcacaggg   15420
ctatttctag gctctggtgg ctccagccct gcctgcctaa gagggtccct ccacaatcag   15480
tgaggaagcc agtctctggc ccccaggtct gtcccccatt ctaccttagc agccgcagaa   15540
cccactcccc ctcctctgga gcccagaaat tcacacatct caccactttg tctcctgagc   15600
ccagtccatg ggcccagacc tcagcctcca tcttccccaa acccctgctc agtcccagca   15660
ctgggcctta gccccccctcc agctccctct tgcctttcag ccgtcttggt ctcacttgtc   15720
cttccagccc atctgtaccc ctcctccagg aagcccgctc tgcctgcccc acccatccca   15780
gctcacacct tctcttcctc tcacttctcc tggtgtccac ctgatagcat gattgcaaac   15840
tctttacctg acgatgtggc tgcaaactgt tgactggatg ccagggtttc aaatggcagc   15900
atataggcgg gtgttcaacc tgcacacggt ttttcaaaag aagtaaatgg taggttcatt   15960
gccaagattt agaaatgagg agatttcatg taataacctg gattttctgg cttctcttga   16020
aaagcagaag ccccaggccc tggctgccgt cccacctggc aggagctgac acatagcccg   16080
gcccggccca cccagcccac ccctcgcctt tctcatcctc gtttgtgcca ccttcctggc   16140
cagctgctgc agcccctgca gaaggagact tgcctctgag ggactgctta gaacagtgca   16200
gcacctgggc ccagcccaac ccagtgcctt gagcctttgt tgaacagtga agcccccaga   16260
gagagaagca gaggcatgag tgccagaccc tgagccgcca gcctggtggt ccgtgggggc   16320
gttaaggggc aacactgagc gactgagtgc tctgctttgt cctttcaagg gtggaacctg   16380
```

gtgctggccc tgatgccacc ttcgattcct ttgtttgggg ggcagccccc tccagaccct 16440
gagccccaag aggccagtct ggctgctcac tctccccggc gtccccagcc ggtgcagtgc 16500
ctgccccaca aggaccagca ggaagtgtac atgggatgaa tatgagcaaa gcgcacaggg 16560
cattgggaaa agacaaggct gggcccctac cccaagcagt ctgctcctct ggattttagc 16620
ctcaaagcag acacttagta gacaccaact gcgtacagct gcagcgctca gtaaggcaga 16680
ggagaagaga gaatcacgag gctggggcac tgggagtgca ggtcaggggc cggggccctg 16740
tggtcgtgtg gctggccatg gctagtcaag gccccctgcg caggtctagc cctctggcct 16800
cattcatagc ctcatcccgt ctcacctggg tgtctgctcc caaagactct tcctctccta 16860
gtaaccttcc tgcctcgtgg ttctgatgaa gacactctcc tacctcaacc atccctggct 16920
ccttctacct ccagatgaaa atcccgccac ctgacctggc cttcaggatc ctctatcatc 16980
tctccctgac cagccccctc cccttgaccc atctctgcac cctcctcccg ctttctaagt 17040
caaacgtgtt ttgggtgttc cccagactca ccctgcacgt cctgccactc tgctttgctg 17100
gtgcagttcc ctctacctgt ataccccctcc ccctacccac accttgaaag cctatttgtc 17160
ctgcaagact caacttaaat acctcttccc cgtgcagctc cctgacctcc agctggcagc 17220
cagtcatgcc ccctgccccc ggggtgcccg atctgtatca ccttgatgcg tcctctgagc 17280
atgattagcc ccccatgagt ctgagcactc ctagaggcca agtgcctcgg tggtgccttc 17340
tctgtcagga aggggcttgc acccaggagg tacaactctg caaattagct gagtatgtgc 17400
acgagacaga gctattgatg cagtccttgc cttttgttgc ttaccctaca cagaagaggt 17460
gacagtgact cgacacccat taatgagtgc ccaccataac ccaggaacga ggtctgtaat 17520
cctgagagaa acaagattcc tgctctcctg tcccatcagg catctagcca ggctgtgagt 17580
gtgtgagcac agatgcacgc caagactcag aggcagccct gaagcaagac ctttgcaggg 17640
tggggcagcg tgtcttgggt tcctaggcat ggtgcagaca ctgtggaggc aggaggtccg 17700
gacaggagga gaggcccagg ggacgtggct tcatggggct ctctgtttca gctgctcacg 17760
aaagatgcga agcagaggct gggctgccag gaggaggggg ctgcagaggt caagagacac 17820
cccttcttca ggaacatgaa cttcaagcgc ttagaagccg ggatgttgga ccctcccttc 17880
gttccagacg tgagtagcct cccccagccc ccagcagctc ccttcacagg gtacccaggg 17940
ctgcccccga gtgctgcctc acagtgagct ggtgcagagt gtggggcaca tcgtgtcttt 18000
gtggggacca gggtcccctc ccggccactc ctcacctaag ctgcagatga acttcacgcc 18060
aggcagtgca gaagaggcgt gggccaggga gcccacattg gctgtccaga gacttgggca 18120
ctttcccagg tctgggggtc ctggacagat tccttctaag cgtcagtttc ctccctgtcc 18180
agtgaaacca gtaacactca tgcctcccac ctgcctggga gatttgaggg tgaaatgaga 18240
actgaatggg gaatgctttc aagagcattg acggccacta cattcagttc atcttgacca 18300
gcatgcatgc atcgagcact actgttgacg ccaggttctg ggccaggcag aaacataaga 18360
ggcccctccc ccagcagctc acattgcagg gagttgcatc ttcccaggag gtccccaggt 18420
cccccagtcg gtgtgtgagg caagattcca gctgaggtag aattgtcctt gaaagtcccc 18480
tacatggtct ataaatcatg agctctcagc tggatcagag gcccaagaga tgcagaccag 18540
gcagaggctg ggtgatctgt gcgggatacg tgctgtgatt ccggggcctg ctctgtttaa 18600
agtcagggag tcagaagcca ggaaggcctg ctgtgcttcg gctcctgcca cgctcccgcg 18660
gatgacccct tgctggcagg gggactgggc cggggactat ggaggccgtg ggagcagcag 18720
tgtgctgcag gcgaatgact gcatctccag agagctcatg tgcacctctc ttcccaattc 18780
cgtgtccaat ggctttgcgt cggtagctta cagtagatct ggtggtagca tctgcacagc 18840
agcaatttgc aaatgctaca aatcagggcc tctgcgtccc cctctcccag aaccagtggt 18900
tatcacttac cagtaccgcc ctgagtggag gcaggtgggg accgtccttg gaactctggc 18960
accccctata catggggatt ccctggtcct ctgggacaga aggtggcttg ggaagctgct 19020
aggtgcggat cagtgtccat ggcaaccaca ccacagaccc cgtctggcca gggatggcat 19080
cagtgtgatg ggtaatcagg gaatgctttt tggagaaaca cgggggccac tgagaatata 19140
aagagtaggc cgggcacggt ggctcatgcc tataatccca gcacttggga aggctgaggc 19200
agtcgggtta ggagttcgag accagcctga ccaacgtggt gaaaccccat ctctactaaa 19260
atacaaaatt aaccaggctt gggggcacac acctataatc ccagctacct gggaggctga 19320
ggcaggagaa tcacttgaac ccaggaggca gaggttgcag tgagctgaga ttgtaccact 19380
gcactccagc ctgggcgacg gagcgagact gcatctcaaa aaataaaaag tgaaagtcac 19440
tgaatgctgc aaactcaatc tgcagatgag cttgagcagg caggggggagg atggtaatca 19500
cagcattgct ctcgaccctg gctgcctggc gaaccgcctg gggagcatga acaacactcg 19560
ccttggatgc agtccccagg gagtctgggg taagactcgg gcttcaggag ttttgaaacc 19620
tccccagctg attctagcgt agagccaggg ttgagagtca ctattatggg aagtgccaca 19680
tgcaaggagt gtgttgtttg ttgtttgttt ttagtgccaa gggctttggg agtggccact 19740
ccttggtgtt ccaggaaacc acatagtgtt gagtgcatga acatcacggt tgtgccattg 19800
ggctgatgtc cgaaattgga gaggatgtgg gcatccagac tggggggtgtg gcaaaggttt 19860
ggaacccggg tggccatcct tgtggcctct gggctaccta ttgctatggt gacgggctga 19920
gattagtcat cgcattggag ctattcctct accttccagc acactccagg tcccgtgcca 19980
ggccccaagg gagatcccat ggcacacaaa acagttggtt gtttccagtc tgaagaggag 20040

```
caggggtgtc agcaaaaccc cttaaataga agtagaaata cgagtcacaa gagaagagaa   20100
caggcagcct ccgggaagat gggagagtag cggggcctcc gtgcgtgcac ccaggcatgg   20160
ctggagaaaa gcgctccagg ccctggaagc agcatgtgga aggaggcggc tctgagggtc   20220
agagcgacca actgtcctgc tttgcctggg actgtcctgc ttttagcact gaaagtcctg   20280
tgtcctgggg agacagggac agttggtcac cctgggagtg accctggcca cttcgaatca   20340
gggcattagg aggatgaggt agcagagccc cgcaaagcgc acagcctgac aaggtgcaca   20400
gagcagccca ccggtgccct cctgcctcag ccgtgtaccc tgtggcccag ggcctctgtt   20460
cctggcatcc gaggcctcag gtcagcaaag gttgcaggcc ctggagtgcg aagccgctgt   20520
gagctgggga ctttgccttt tccccactgg ccctccccag gctgcacgcc aacccccagt   20580
catccctgcc agccccccac agtcatcccg ccccccacag tcttccctgc cagcccccca   20640
cagtcttccc tgccagcccc cccagtcgtc cctgccagcc ccccacagtc atccctgcca   20700
gccccccacc atcgtccctg ccagcccccc accgtcgtcc ctgccagccc ccccacagtca  20760
tccatgccag ccccccacag tcatcccatc cccacaccac tgtcggggct gctgccacat   20820
tcctaaacca cgagcactgt gagtgacatc atctttttca cttccattga ctcttttttt   20880
aaaataaaaa tcaatgtatt ttaaaaggaa acctttccaa ggaaataata gccatgaaag   20940
aggggtttgg gtgtgccagg taactttctg ttccattaca gatttttaaa atacgctcat   21000
ttcttaacat tcctctgcaa gccacccagg gccagcttcg tgagcttgac ctggcacccc   21060
ctagggcacc ctgggtcccc acacctggct cagtgccctc tgttgccatc ttaaaattct   21120
aaatgtggaa catggaccct gcgttttcat tctgcgctga gcatgtgaat gatgtggtct   21180
tgcttccagc taaaagtccg ctgagttcca cggtggcgtg caccccgggc tttgggaact   21240
agagaagggt cgcagctctg caactggaca cccctgcccg cacccctgct tccgtccacg   21300
tgcacttggg cccatggttt gctttgctgg ggacagcatg gccttgagcc ttgagctgga   21360
gaccagagca gactggaggg gagagtccca gaaagtgccg agccctgaga ccattgctag   21420
aaccttcctg gcatggcgga gcccatgctg agccggtgca ctgattcaca accttcgtat   21480
tagccaaatg tcaggagact ataagcagga ggtagcctct accctccagt gacagatgtg   21540
tcaccaggtc tggggatccc ttcacacccc acctggggct gtctctataa aggcctcatc   21600
tccagaggct ctgggaagcc tccccacacc aaccttgaac tcttagaggg cagggtccac   21660
ccaggttcat ctctgccctg gcatagagca ggccccccagt gcacagctga gggacgagta  21720

aggcaaaaga tatgcctacc ataataatac cagctcccag ttacgggcgc caggctcggt   21780
ggccagcact gacagcagcc ccatcgccca ggtgcctcgt tgtccccatt ttgccggtaa   21840
ggaaacagga cataggcctc aggggatatg agacttcccc aaagttccta agccccacag   21900
ctgggactct agcccacgtc tgtccagtat gggtaaggga gtgatggcag gaatgagccc   21960
tgggctgggc acccaggtgc cccgagctcc tgtgtcaccc cagccagggt ccccgtcatg   22020
gatctgagag agggctgcac tgtcatctgg aggggtcgca caaaaaaacc acaggccatc   22080
atctgaggtg gacaggaagc ccagccaagc cactggggcc gacccctccc ctggactcac   22140
ctgcatctga gccacacacc ctccagccac tacccatagc agttctgggg gtgtgtcctg   22200
ggaagactcc cttctgctcc ccaaaacccc aaggcctggc tcggggccac tggagccgca   22260
ggcgggacat atgtgtgacc ggccctctgc ccctggcagc cccgcgctgt gtactgtaag   22320
gacgtgctgg acatcgagca gttctccact gtgaagggcg tcaatctgga ccacacagac   22380
gacgacttct actccaagtt ctccacgggc tctgtgtcca tcccatggca aaacgaggtg   22440
agcagggcag accacttgct ttggtctggg tgggagggag ggactgacgg gtggaaggag   22500
gcgtcgggaa tatgagtttg gcggcaggag gctgagcgca tggtttctgt ttctccatg    22560
aaggcagcac acaaaagctg tcagtggcca agtagggagc tgtagccacc acgggccagt   22620
cattgacggg gagcctcgcc cagcccctga gacctggagg gcgtgtggct caggggcagg   22680
tgaggccagg gg                                                         22692
```

<210> 23
<211> 5096
<212> DNA
<213> Homo Sapiens

<400> 23

```
gaggccgagg cgggcggatc acctgaggtt gggagttcga gaacagcctg accaacatgg      60
agaagcccca tctctacaaa aaatacaaaa ttagccaggt gtggtggtgc atgcctgtaa     120
tcccagctac ttggcaggct gaggtagaag aagagcttga acccgggagg cggaggttgc     180
ggtgagccga gatcgcgcca ttgaactcca gcctgggcaa caacagtgaa actctgtcta     240
aaaaaaaaaa atgcccataa ctgctattcc acaagaaagg aaaaagccct caaagagata     300
cataaaaagg acattcatag acaaaagaaa tctcatattt aggaggctac tagactgatt     360
ataaaacata cccggattcc aaaagtatca gaacattagg agagaaaaaa aaaaaatcag     420
```

```
gccaggcacg gtggcttacg cttataatcc cagcactttt ggaggctgag gtagatggat     480
cacctgaggt caggagctca agaccagcct ggccaacatg gtgaaatcct gtctgtatta     540
aaaattagcc gggcatggtg gcaggcgcct gtaatcctag ctactcagga ggctgaggca     600
ggagaatcac ttgaacccgg gaggcagagg ttgcagtgag ccatgatcgt gccgctgcac     660
tccagcctgg gcaacaagag caaaactctg tctcaaaaaa aaaaaaaaaa aagaagaaga     720
agaaaagaaa agaaaatcat tgttaattct tatcacatct tgttaaacat acatcttgcc     780
atatcatctg ccccagcctc aaggccactg gcatctcctg aagcaatcac caaatgttcc     840
catcacccaa gaatagagag ctaatggtga caactaaccc atatagagca ctgaaaggag     900
ccaggctctg tctgcccttt cctgcatagt aaccttgtaa cagcactaca aggtaggtac     960
tgttattatc tccattttac aaataagaaa actggagcac aggctgggcg cggtggctca    1020
cgcctgtaat cccactactt tgggaggcgg aggtgggtgc atcacttgag gtcaggagtt    1080
caagaccagc ctggtcaaca tggggaaacc ccatctctac taaaagcatg aaaaaattag    1140
cctggcgtgg tggtatacat ctgtaatccc agctacttcg gaggctgagg caggataatc    1200
gcttgaaccc gaaaggttga ggttgcagtt agctgagata gagcctctgc actcaggcct    1260
gggtgacaga atgagactct gtcaggaagg aaggtgacag aatgagactc cgtcaggagg    1320
gagggaggga gggagagaaa agagggaggg agactggagc acagagaagt taattgactt    1380
gtccaagaag ggagggaggg agggagagag ggagaccgga gcacagagaa gtgacttgtc    1440
caggaaggaa ggaaggaagg caggaaggaa ggaagggagg gagggaagga aggaagggag    1500
ggagggaggg agggaaaaga gggaaggaga ctggagcaca gagaagttaa gtgacttgtc    1560
caagaaggaa gggagggagg gagactgaag cacagagaag tgacttgtcc aggaaggaag    1620
gaaggcagga aggaagggag gggcggaggg agggaactgg agcacagaga actgatttgt    1680
ccaaggtcac ccagcagaag gaagggagga aggaagggag gaaggaaggg agggagggag    1740
actggagcac agagaagtta agtgacttgt ccaagacgga cggacacgga cggaaggaag    1800
gaacaaagaa ggagggaggg agggagggag ggaactggag tacagataac tgacttgtgg    1860
aaggaaggac ggaaggaagg agagagggaa cgaaggaagg agagagggaa ggaaggaagg    1920
aaggagagag agggaaggaa ggaaggagag agggagagag ggagggaagg gagggaactg    1980
gagcacagag aactgacttg tccaaggtca cccagccagg atttgagtcc agaatgtgag    2040
tgcccttagc cattacactg aattgcctcc cagctgagaa cttttaacaa atgtatcagg    2100
acatacacaa ctggatgcta caaggtggtt ttcggccaac tttccccttt aaacacatgc    2160
atcaatttca aactatttat atgataaaaa atgcttacac ctttatcgat ctcgttttta    2220
aaatggtatg gcttttcagg actatctttc acaatcgtta aagggaaatt cgctttgtta    2280
atatgaaaaa aaaatcaaat atctgtttca tgagttgtca gtggccaacg ctgtttagac    2340
tacggcttga acgcatcacc ccttaaagat catcttgtgg ccttgggcaa attgcttagc    2400
cttctttgtt tctccagcgg caaagtggat ataacaatag taccatctcc taaagtggtg    2460
aggattagct gatattatcc atataaattg ttatgcacag tacctgacac agagcgagtg    2520
cccaataaat gtctttcact atttaggtcc tgggttctct catttccaga gtgcaagtcc    2580
tccagcaacc acagaaacgc tttctagagt cgctgcccac tccttaaaaa catttgtccc    2640
tacttcgcac agagcatagt gataccacaa acaggcttcc ctggacccca gccctggggg    2700
tggctatatc acctcgctca ggcccaggaa gagagagccg gctctggtct cccacccggc    2760
agcagcacaa aacttaacca ggatttctca cttcggtcaa ctcttgaatc accgcgccca    2820
gtctggctgg ctcggcagca gatggaatga gggcgccggc gccaggaagt ggcgctcacc    2880
cacctgccca gcccgaggtg ggcactcctc cttcacagca tccccgggga cctcgtccct    2940
ccaccgcgga acgccccaga ggaccgactt aatagtgcca cagggtgggt ttccccccaa    3000
atcctccaaa atacatcgcc acgatttcga aacgggagcc gagggagacg caccaagtgc    3060
ttcccgacgc caggtgaagc gcaccatacc tggtgtgcgt gttgagcccc gaaccgaagt    3120
ccgtgcggcc gccacctctg cctccctgct gggggcctca gagagtgtgc tccttgtcca    3180
cgtgcgcgca gggcccgcag ccgcccccac gccccagcgg agctgcagca cgccccttcc    3240
actcaccagg tgaagagttt gccttttatc ctgcgcagca ggctggagaa ctcggccgcc    3300
gctccctccg cgaggggctc tggggcgccg gggcccgctg gcgagtctgc ctccatcggc    3360
gcccttgcgc gtctccggcg cccaaaaccc ccgaagtgcc gggtcccgga agacgggagg    3420
cggtgcctgt gcggccggct ggggctccgc tctggccctg cgctccctcc ctcgctctct    3480
tgctcgcgtc cctctcgctc ctccgctcgg ctccttcagc gaccaatcat taactgtcaa    3540
gccctaaggg ccagacaata catttgcaga ttacaacctg gcacctccag ggtggcagga    3600
ggagaaaggg caattttttt ttcgtcttgg cagccgagcc ttattgttga gcggcaccca    3660
gtgtgccttc aggtttttag ggttccttgt ttgaaatcac aagatacagt gacacgaggt    3720
tattgtaggt gattcctaag ctaagaagcg gtgagtgagc agtgagttag aagcagtgag    3780
tgagccggga ctctcctaac tggaaggact ggctgacccc tcctcttccc tcccactctc    3840

cctccctctg gctgatttgt acaaaatcgc ttcattaaaa taactgaagg ctggcagggc    3900
gcgttggctc acacctgtaa tcccaacgct ttgggaggcc gagacgggtg gatcacctga    3960
ggtcaggagt tcgagaccag actgggaaat atggtgaaac cccgtctcta ctaaaaatac    4020
```

aaaaattagc cgggcgtggt gacgggtgcc tgtagtccca gctactcgag aggctgaggc 4080
aggagaatcg cttgaaccca ggaggcggcg gttgtgagcc aagatcgcgc ctttgcactc 4140
cagcctgggc aacagaggga gacgtcgtct taaaaaaaaa aaaaaaaaaa aaaaaaacct 4200
gaaggcgctt caggtctgtg tcgcttttcc ctgcaaaaca tgggtaagat cctaaaaaga 4260
tcttggaaac gtgcctttgt ggcgtggatg gaggcgtggc tcattcctcg cggtgtgttg 4320
gttttgtctt tcagtattca gttgtctccg ccgtgccagg gagagagacg gtgccaaccc 4380
ccggcgctcg gcttggatgc cctgccctgg ccacgccttc cgaggcttcc acgggctaaa 4440
gagggattaa gagacactaa gctggggtcg aaaagttcct gtcactgacc ccctgcttcc 4500
ggaaaggagg agatccagaa aacaaaagaa aaggtgttgg gaccttttat tttttcctgg 4560
ccacctgctt tggggggttgg ggtgggtcaa aataaacgta ggttcaaata gacaggctgt 4620
gcagacgagc tggcaattct gtaagaggag tctaagaaaa aaaacagctt tcgaaaatga 4680
aatattactt caggaacccc aagtttagca gctgcaggaa cctcaaaaaa acctttgaaa 4740
ctaagaacgt ggtcatccgt agagactaat taagtattat aatacaagca tattatgaaa 4800
caagaggaca ttagaagcac atttaagatg tcacttttgt aaagtaaaca actcatttaa 4860
ccctgcatat tgacaacgtt gaattctacc tgagccctgt gctcctggaa agtaatgaca 4920
attaagaaac cttcctaggc cgggcgcggt ggctgacgcc tgtaatccca gtgctttggg 4980
agaccgaagc gggcggatta ctcctgaggt caggagttcg agaccagcct gaccaacatg 5040
gcgaaatccc gtctctacta aaactacaaa aattagcccg gcgttttggt gtgtgc 5096

<210> 24
<211> 3072
<212> DNA
<213> Homo Sapiens

<400> 24

ttttaattta gcgggacggt ctgtattttt atttgctaaa tctggcaact tttggggttg 60
gggtttgggg cccggcaacc tttggggtgg gaggtctttg tgtagagccc aacctgcccg 120
ccaaggccca ctcaccggag aagaactggc tgatggaggt gggcaggagc gtgaggaagg 180
ccaaggggtg ggcgaaggag atggaaagga cagcggagat gtaggccacg ccggccacca 240
tggagtagag gcaggccagg gaggtgtaga ggcagaacag gacgaagttg cgcatgttcc 300
tgctgccgat gcagttgccg gtgaagaaac agtgatggtc gtgcctcagg gtgactctgg 360
cgcacactcg gcagaagtgg gtgctaggtg aggggcatgg agtcttcctg gccgaggccc 420
cctggcaggc ccccaggtcg tctggggagt tctggatgac aaggacgtaa ttgcccaggg 480
cgttggccga gaggaatagg aagagcgccc cgtggagcag ggcgggcgag aagagccggg 540
cggccgcggg gtcctcgcgc atgctgggca ggaagaggaa gagctgcagc acgaaggtca 600
ccagggagat gcacaagaag taggcggggg ccaccacgtt gagcagccgc agggccagca 660
tcctcgatta cattcctgcg ggcccggggg tgagaagagg actgactgac tacggcgtcc 720
agggggcgcc ctcgcccgct cctccgtgcc gcgcgtctgg ggcactgcac gcgactccac 780
gcccccaacc ccctgtcccg cggatttccc ctccccagtt ccctaacccc ggatcaggca 840
ggccagagac ccgagcccgc agcggcctcg ggtctaggcc ccacctgggg gtgagtgttg 900
cctggggttt tgagcgagct cggcgccttg gcgcggcaga gtctggcaca ggagggaggg 960
actcgtttgc agtctttagg agttgtgagg gtggggggca ccgggggata ggactcctcc 1020
cctctgtgga tgtggaaaca agcccaggtt tgggatttct ggttcagacc tccctaggag 1080
gagaaaggga aggatttggg gtcgggggca ggagaatcag cggcacttga cctgctggaa 1140
atcctgcttc acactcccct ttctccgtcc ctgcgtcccc acccacacac acatcttggg 1200
cagcactcag ggacctcacc aggttaccca aggctctttg gaggtatgtg tgcgtgaggg 1260
accacgatgt gtgtgtgtgt gtccttgtct gtgactctgt acgtgtaatc tgtgtgcgct 1320
tgagtatctg tatttgtgta tgtatatatc tgcgtgtaca gacacgcagc ctctgctatc 1380
tagctttaaa aaaatcaatt gaaacaatcg atacactcac actcgctctc gggcaccggg 1440
tgaggtgggg gcgcaaccag aatacctgtg tcagcgggtc ctggccagca catctctcca 1500
acgctggctg cccgggctat ttctttgcga agagcgtttt catttgaggc gaaattaaaa 1560
tccccccttc gcgcccgcct cccgccctcc tgctgagaga aacccaaaca accctcatgg 1620
cgccgaaatc ctttccatgc cgacaagccc gccctgggcg cacgagtgga tgcttggcca 1680
gcctttcctg ggatcgaccc cgccgccgtg ctcagccctc accacgtccc catcccaccc 1740
cacgcctcac agccggggtt cctggccagc ttcggaagcc accgagaaat aggattccgt 1800
gcgcccgaga gaacttttcc aggggctaag gaatcggcca gccggaggcg cgagaaaagt 1860
tctgggaagg cggttgcacc taggatgggt ggatgccacg gggcctccgt ccaggctgtc 1920
ccgtccgcac gggtcgactg gtcaccttgg aatccccttt gcaggtccca gcgccccccg 1980
ggaacccgca gcctccgcgg agagcgtggg cctctcccta ccgctggggc gcagcgcagt 2040
gcacgcctga gggtggtcgc cgggggctgg gcacgccccc agtcctgcgc cgccgggggc 2100

tgcggcggtg ctgcccaccc cagagagccc tcggcctggg gctccggcga agcaagtgcc 2160
ttcccggcgc cggtcgccag gggggcgcgg gagcagccag atgcgccgca gcgctgggaa 2220
ggcggcgaag gacaggggct aggggagtga ggggcgctcg gcaggcagcc tcagccctgg 2280
ccctgcgcgg gagaagggac agcagagacc gcccgtgggg ccccggggtg tagggagctg 2340
tccgttcagc cctggcgccc gcctcgcccg cggcagaggg cggcacagcc ggagccttgg 2400
aaagaccggc agcgccggca gccgcgggct tctcggccac tgcctcccgg acgcacggga 2460
agccgccccc cgcgccgccg ccgccgcact gccgccgtcg cagaggggtg aggaaatcaa 2520
ctcaccgagc tctggtcgcc gacaagagga gccccggacg ccggctctcg ccctgcccga 2580
ggctgcaaag ttgtggactc ggcccggctg gctcgcagcc tgcgcttcgc ttcgggaact 2640
gggcaagtag cggggatgtg gggaggaggg agcgggcagc tgctggcttc ccacttgggc 2700
gccagcgagt aagggccagg aagcggcggg gatggcagct gggcaccccc cagccccgcc 2760
acccctccct ccgctcctgg gggcggtgct ctggccgcca gtctcagcat cgtggacttg 2820
ccccctcctc ccctctcgat tccccttgag cgggctgggg cgccctgcct ggtttcagcg 2880
cccgttccag ctgggagatt gggattcagc cctgatgtgg attctccagc catcatcctg 2940
cccttccctc tcggctaggt cccacgacct tccctgctct ccactcggac tgaaggaacc 3000
ctggggggctt gagaggtcag cttgccaggg caacacccat gaagatcagc gtcatctcct 3060
cggaagggct gt 3072

<210> 25
<211> 3845
<212> DNA
<213> Homo Sapiens

<400> 25

ccgcctccca cgttcaagca attctcctgc ctcagcctcc cgagtagcta agattacagg 60
catgtgccat cacgcccagc taatttttct gtttttgta gagatgggtt tcaccatgtt 120
ggccaggatg gtctcgatct cctgacctcg tgatctgccc acctcggcct cccaaagtgc 180
tgggaataca ggcgtaagcc actgtgccca gcctacattt tatttttcga ttgactcccg 240
tgatcagagt caacttccca tgttatttta tgtgaatatg tatcaatgaa ctattagaaa 300
ttgcaatgtt tttgtaggtc aaaacaattc aatattgaca attttatatg attcagccta 360
atacatcatt ttttccatca gccatattgc atgttattat ttatatatac gtttatttag 420
ttagcctatc tgttctctgt gaggaacatt acacagtttc tgagattttg ttacgataga 480
agtcagcatt gataagcctc tgcgcacctg ttttaatgta ggatcattgg gaactgagca 540
gtagcttaca gggcttggga ggggatttaa ccatttagtc aacctccact cacatcaccc 600
tcccttcttc gataatgggc aattgagaaa gggcaaatgt gtgtaaaaac tatgcagatg 660
cagtttcagg attattttct tcttttctgc agtgtctttt cccaagctac atcagcgtct 720
aaattttgga gttactgatg cttccgttat ttctgcagat agaatattat gtcgtaaata 780
atgtttactt gtccattagt attatataaa agtgcctgtt tctccccact ccagctatca 840
ttgattattg gtaaaaattt taatgtatcc aattctaata ggcaaagaaa ggctctccta 900
cttagatctt aggtgggggt ctaagttcat atttttgttt tacaaaaatt gaatagccag 960
tgtttttgag ttgaatgatc tatccattcc gtataggaga gtttaattat attgtgattt 1020
tgggggagat aattttcaca atttaccccc tcaaaatgta gaaagtagaa aaagggtaga 1080
aaaatcgcac caaatttgaa caatgtagca tgcttttaga gttagggata ttttcttttg 1140
gaaggatttc aagtctggcg tatttgcatt gattgtggtt atggcgcatt taacttttcc 1200
aaaccagctt ttttgtaccg cacataggaa gacctgaggg tgagtgtaaa ttaggattgt 1260
ttggccagtg gtgtgtgcaa aaatttattg tagaaccaag aattatttct ttagtatttt 1320
acacatttta aaaaacaggt agacatgtcc attgatccca ggttgcttat ggtttctaga 1380
ggccccctct cattgcaaca gtgctgtggg gccctagggg tccagtagcc ccctccccc 1440
aggtcattcc ggtgagggag ggagctggga cccctgcact gcggcaaaca agcacgcctg 1500
cgcggccgca gaggcaggct ggcgcgcatg ctcaggcggg gatgtgtgcg aagcctgccg 1560
ctgctgcagc gagtctggcg cagagtggag cggccgccgg agatgcctga cgcatctgtc 1620
tgaggagcgg tcagtgacgc gatggagcgg gcaaggtcag ctgtgccggt ggcttctctc 1680
aagagacagc ctggggagcg gccactttta ttcatcagat attccaagtt tttaggactt 1740
ggagtactga ataaacggaa tttgggccct aaagtccttt gttctggaga accagatccg 1800
gaatgttcag aggcttgctg ttgtgccgtt ctgccccgat ggtatcctgt ccgctcgcat 1860
tggggcgcgt cccccatccg cccccaactg tggtgtcgcg acaggtccta ttgcgggtgt 1920
ctgcggtggg aagggcggtg gtgactggga gcatgcgggg taaccgcagt gggcaaggga 1980
catgggtgga ggtgggtaca tcagggtgat tgcagttccg tgtggcgagg gtacgtgggg 2040
ggaccagtgc atagggattt taggcggagg taggtatatt ggagtgattg tggcggggcg 2100
aaggtacgtg aagtgatcgg tatttagggg gtgttgagcg caggtaggtg tataatagtg 2160

```
accactgcgt ggtggagcag ggtacgtggg gcgactgggg ggggaattcg tcgcagtgac    2220
cgaggcgagg aggctatggc agtggaccag ggggatgagt cggtgtgaca gtggtggggg    2280
ttgtgttggt ggtcgcggcg cggggagagt ccgtaggaag tggctgggag gtagggggaa    2340
ggcggcgaca gtgggtattg gcggcggtgg gcattggcag cgggtaggca tggcggtggt    2400
ggactttggc ggcggtaggc atggcggcgg tgggcatggc atggaggcgg tgggcatggc    2460
ggccgcgggg cctgtcgctg tccggagtga ctaagggacg ctgaatgatt gctgtgtaga    2520
ggagggggca tcagaagggg cagtagcaca gtccgcagcc tttaattttc ctgtgacctg    2580
cgatggtcat ctggtttgcc tactgtggtg gtggtgcttt tttattaaaa ctgcgcaatg    2640
cctacactgc cgcaggggct gcagaaatgc gtggaatcct tgcgtaccct ttaggaaccc    2700
tcgctttaga ggctatggca taagaagact ggaatgatgt gcagaacttg catttacaga    2760
tcttttgact gataggccag gtgatgcctg gggttactt ttttttcatt gtttctctct    2820
gttgggaacc aggtcttgga aggctatgtc gaaataacct ggggtctgt gttgcgtcac    2880
tgccattgtg cagctccttc aagatggccg ccgctgcagc ggcttagatc tgcgcaagcg    2940
cttcagcggg ggggtggccg cttcctccct gtagagccgc cagtggggag ggggcaggtg    3000
gtgtgtgttc agcttctgct gtttcggagt ttcagccgta ccctctttag ggtgcagtgg    3060
taaggagagg aagccggcca aggtgggcgg ctgcccccctc cccaaagtgc tgcttgggaa    3120
aggatgcagg ttgcgcagac gcagcagagg tgacagtcgc ctccgcttgc agtggggcga    3180
gacgtggggc aggggagcga gggcttacgc agcttttgcc tggaaggaat ggcagccctc    3240
cccttcacct ggctgataca gcagctgttc ctttccggtt gtggcgcagt agagggggga    3300
ggagggagat gggttggcgc aggctccgcc tagcaagctt ggcagccacc ccttacccac    3360
ggtgcagcag tagaggactg gaggggaatg tggctagagg ccaggcattt gtaccacctc    3420
cgcctgtgtg ggatggcagc tgcactgggc tactgctttt cagctctgca gtaaactaga    3480
gtggtggcgg cctggaggcc atcaaaggtg cagccacccc aagacctgct gtgctgtttc    3540
agcaagcctc cattaatgat agagatttta aattggtttt ctgactccct ggaaaatcgt    3600
accttttcat ttttgtagtt cctctcttgg ttgtattgtg cgctaattct ctattttcaa    3660
actggacttg ttaacccctt ttttttaact ccctgtagtc tttgatttcc attcgacaca    3720
tttaataaat gtcaccattt aagattcagt tatccttctt aatctctgga tagtccattc    3780
tgatgcactg catactttcc ttaaatgggt tgggggagag gacatcgtga tggctcttcc    3840
cgact                                                                3845
```

<210> 26
<211> 18463
<212> DNA
<213> Homo Sapiens

<400> 26

```
tttgagtagg cgtgtatttc gcagacaggt ttagcgcctt ctgcctgcca ggcgccgagt      60
tggatggtag gaatacaccc caccgggcaa gacctggtgc ctagcctttt aaggcatcag     120
tcactgggct gaaagagaga aacaagccaa aaaacggaaa aactagttag aagtccagca     180
catgctttaa ggaagccagc aggctgcagt gtgagagaat tagcaaaagc gtatctgact     240
tagacagggg tgttggaagc agctccccaa aagtggcgtt gcccttgaaa cgcagctcag     300
gatttgcatc tatgtgcagg aagccttaca gcacgagagg ttcatacagg gacaaggcgc     360

caaggggggac tgaggggcac gaggggactg agaccacccc tggcctgcat cgctgctgtg     420
gctcgggtcc tggtgaccca ggagcaggga gaacagagag ctccgttcac atctgagaca     480
tcagagggag agatgttgta ttagtccgtg ttcatgctgc tgataaagac atacctgaga     540
ctgggcaact taccaaagaa agaggtttaa tggacttaaa cagttccacc tgtgggggaa     600
ggcctcacaa tcatggcaga aggcaggagg agcaagtcaa gtcttacatg gatggcagca     660
ggcaaagaga gagggagctt gtgcagggaa actccccctt ataaaactgt cagatcttgt     720
gagactcatt cactatcacg agaacggcat ggaaaagatc tgcccccatg attcaattac     780
ctcccaccag gtccctctta cagcacgtgg gaattcaaga tgagatgtgg gtggggacgc     840
agccaaaccg tatcagattc tcaaaggtca gacccagcag gtgttgttac agcaaagaga     900
attgccgggc acagtggcct gacatgggcg ggagccctgg tttgagctct ggacacggaa     960
gggacttaga ggaggctccg gctgctcgtt ggtgctgaag gttgagacct ggaaaggtga    1020
tggggggtgc aggcaaggca gaaaggaaac ttctaggccc aggcagaagc atcatgctgc    1080
ttacaccttc ctgggaagtg gcatccgcgg gggtggccag caagacctcc tggctatggg    1140
acttttaaga tgcatccttc catgctgttt tcccagatat cctggctatc ctgctgaccg    1200
acgtactttt gctgctacaa gaaaaagatc agaaatacgt ctttgcttct gtggtatgta    1260
tcctgtctct tcagacgaag ggtcggctgg gtgctgtggc tcacgtctat aatcccagca    1320
ctttgggagg ccgaggtggg cagatcactt gaggtcaaga gttgaagtcc agcctggcca    1380
```

acatggtgaa acctcacctc tactaaaaat acaaaaatta gccgggcatg gtggcccatg 1440
tctgtagtcc cagctactca ggaggctgag gcaggataat cacctgaacc caggaggtga 1500
aggttgcagt gagctgagat tgtgccactg ctctccagca agactgcgcc actgctctcc 1560
cgcctgggca acagagcgag actctatctc aaaacaaaaa aacaaaacaa acaaacaaaa 1620
aacagacgaa gggccctgct tgctgtgacc ttgttgggag gttggctgaa actgcccatg 1680
gaaggggcac ctgtgagttt tccaggccca catagctggg attacaggcg cccgccacca 1740
cgcctggcta attttttgta tttttagtag agacggggtt tcaccatgtt ggccaggctg 1800
gtctccatct tctaacctca agtgatcctc ccgccttggc ctcccaaagt gatgggatta 1860
cagtcgtgcg ccaccgtgcc cagctgctgg tgtgttctta accagtgtat ttgtttgctg 1920
aggctgctgt aaacaaagta ccatggactg ggtggtttag accacataaa tttattgtct 1980
catgtttctg caggctgaaa gtccaagatg aaggtatcag cagggttggt ttcttctgag 2040
gccccttggc ttgtaggcag ccatgtgctc atctgttctc tgtgtcctcg agtggccatc 2100
cctctgtgtg tgcctctgtg ctaatctcct cttgttagaa ggatgccagt cagattggat 2160
ttgggcccgc ccatatgatc tcattttgcc ataatcacct ctcttttttt tttttttttt 2220
tttttttttt gagacagtct cactctgtcg cccaggctgg agtgtgcaat gacacaatct 2280
cgactcactg caacctccac ctcccgagtt caagtgattc tcctgcctca gcctcctgag 2340
tagctgggat tacaggcacc tgccgccaca cccggctact ttttgtattt ttagtagaga 2400
tggggtttca ccatgttggc caggctggtc ttgaactcct gaccttaagt gatctgcctg 2460
cctcagcctc ccaaagtgct gggattacag gagtgagcca ccacacccgc acattaatca 2520
cctctttaaa ggccccatct ccaaagacag ccacattctg aggtgctagg gctcaggact 2580
tcatatgaat ttgtggggaa cacagtgtag cccactctag ccagttaaaa tgtgcatttt 2640
gctggtctgt ggaagcattg tctcttgaag gatgcgtgac agactagaaa taccggtggc 2700
tctgggtggc ctgaggcaca agacagagat gggtttatct gttgttatat actacatacc 2760
gttgtatctt ttgagctttc tcgtgtgtat gtgcattgcc gactatttaa aaaaaaattt 2820
ttaagctgat gaaattgaca cattatcggg atactaaggg gcaagatggc tcactcctgt 2880
catctcagca ctttgggagg ccgagatagg aggatcactt gagcctagga cttcaagacc 2940
agcctgggca acatagtgag atcccatctc tacaaaaaat acaaaaatta gctgggcctg 3000
gcggcacgtg cctataatcc cagctactca ggaggctgag gtgggaggat cacttgagcc 3060
caggaggtca aggctgcagg gaacaatgat tgcaccactg cactacagcc tgggtgacag 3120
agcaagacct ggcctccaag atagtttaaa aaaaaaaaaa aaaaaaaaaa ggtttctagg 3180
atttcactgc aatagtatct tttttccccc agatggcaaa atatttccgc tttgaattgt 3240
ttgaaaagaa atgaggagcg tgacccccac tcttagttcc cctcaccagg cccttgacct 3300
ccctgctgtt tgggtgctgt ggggtaaagg gtgacctccc caatgcccctc tactcatgca 3360
ggactcaaag ccacccgtca tctcgttaca aaagctcatc gtgagggaag tggccaacga 3420
ggagaaagcg atgtttctga tcagcgcctc cttgcaaggg ccggagatgt atgaaatcta 3480
cacgagctcc aaagaggaca ggaacgcctg gatggcccac atccaaaggg ctgtggagag 3540
gtgaggaggg cctgggggtc tccccaccca ctgtgttcct tccgctgatt ggtccaccct 3600
tggcttcgac cgttggccat cagctgtgac cttgaactcc ctcatcccag cttgggaccc 3660
atgacgccat tccagctcct tctgatgact caacctgttg gacgttgagg ccaacgggcc 3720
cagaaaggca gctgtaacct gggcctttct gcatgcgtgg gccactgcac ctgccatggc 3780
gggctctctg cattctaggg ccagatttgt tggagcaggg catggcatct tgtccttcct 3840
ccttgggccg agcacccctc agggcccaga accagctggc ttcccctccc tctctttatt 3900
ttttattttt ttgagacaga gtcttgctct gtcacccagg ctggagtgca gtgatgcaat 3960
ctcggctcac tgcagcctcc gcctcccagg ttcaaacgat tctgctgcct cagcctcccc 4020
gagtagctgg gattacaggc gtgagccact gcgcccagcc tattttgttt tgttttgttt 4080
tgttttttag agtctggctc ttgctctatc acccaggctg gagtgcgctg gcacaatcat 4140
agcttattgc aacccaaact cctgggctca agccatccta ccccctcggc ctcccaagta 4200
gcttgaacta caggcataca ccaccacacc cagctagatt ttttattttt gtagagatgg 4260
ggcctcactg tgttgcccag gctggtcttg aactcctggc ctcaagccat cctcctgtct 4320
tggcctccca aagtgttggg attacaggcg tgagccacca tgcctgtcca ctccctctct 4380
tgattcccgg aggcttcctg gtgccttaat ctggttcaga ctcccccatc cacagtagct 4440
gtagctctac ggggcttgca gccacccacc tgcacctggc aggtcctctg caagcccgct 4500
gtcccctgga ctcctcctct aagtcacagg caggcatgta aagcctcttc ctggttatgc 4560
cctggcgctt tggggcggga tgaatgtcag tggctctctc tcttcccctc ctcgtcctcc 4620
tgcaccacga acaagctaaa tcactcccaa agtcagagac ggtcgtggcg gctggttctg 4680
cagaaccagc gtctgtgccg aggctggcct gcctgggaag cacagttacc cacccgactc 4740
ctctccctgc agctgccctg acgaggagga ggggcccttc agcctgcccg aagaggaaag 4800
gaaggtggtc gaggcccgcg ccacgagact ccgggacttt caaggtgagc gggagacagc 4860
gtctgggcac accccttgtg tggtgagccc tgggccctcc atcaggactg gccacagagg 4920
gtgaactgcc ccccaggtga cccggtgttt tcccgtgggc tgtgccatgt cctgcagctc 4980
tgtgaagcat tagaacgggc tgtgccatga catagcgatc tccagaggtc acttagccct 5040

ttgggggtat gtgggagagg gagggaggag agcccagagg acctgagccc cttccagagc 5100
cgaccacaca gccacacacg ccaccaccac cccatcctcg tgcagccagg agctcaacag 5160
ggcatcccag tcccatacga aggaatagca cagggtcccc acagcctcct tccagagctt 5220
cctcctccct gtttaattac acgattgaga tgtaattcac acaccacagc actcaccctc 5280
ccaagcgtgg ggttcagggg catttcagtg catttccaat tatgcagcca gaatcatgtt 5340
aactttagaa catcccacca ccccagaaag aagccccgtc ccccttaaca gtcactcccc 5400
accccatgcc ccaaccctgg caacaggaat ctacttcctg tccctgtaga ttggcctgcc 5460
ctggacattc cacataaatg gagtcacacc gcgcggcctt ttgggtctgg ctcctctcga 5520
ggtgtggtat taaggttcgc ccgcgctcag tgagcacttt gtcaagaaca attcctggtg 5580
ccctgtagcc tgcggcaagc tcccttcttc cctttcactg tctgtatttt tttttatttt 5640
atcttctttt ttttttttaa acagagcctc gctctgtcac caggctggaa tgcagtggcg 5700
tgatctcggc ttactgcaag ctctgcctat caggtccaag agattctcct ccctcagcct 5760
cccgagtagc tgggactata ggcacccgcc accacgcccc actaattttt gtgtttttag 5820
tagagatggg gtttcaccat gttggccagg atggtctcaa tctctcgacc tcgtgatctg 5880
cctgcctcgg cctcccaaag tgctgggatt acaggcgtga gccaccgctc ccggccctgt 5940
attattcatt tctaaattgg ataatactca aacaataagt ggcctggtat ggtggctcat 6000
gcctgtaatc cctgcacttt gggaggctga ggcagatgga tcactggaag ccaggagttt 6060
gagaccagcc tggacaacat ggcaaaaccc tgtctctaca aaaaatttaa aaattaggct 6120
gggtgcggtg gctcacgcct gtaatcccag cactttggga ggccaaagca ggtggatcat 6180
ttgagatcag gagtttgaga ccagcctggc caagatggca aaaccccgtt tctactaaaa 6240
atacaaaaaa ttagccaggc atggtggtgc gtgcctatag tcccagctac tcgggaggct 6300
gaggcaggag aatcgcttga acccgggagg tggaggttgc agtgagccga gattgtgcca 6360
ctgcactcca gcctgggcga cagagcgaga ctccttctca aaagaagaag gaaggaaaga 6420
aagaaaggga ggatggaaaa ttcagttttc taaggctcta agttggtccc agactaataa 6480
atgggcctga tacaatcctc actcacaccc tactgtgttt gattttattt tatttctttt 6540
agagacagga tcttgctttg ttggccaggc tggagtacag tactgcagtc atagctcact 6600
gcagccttga actcctgggc tcaagcgatc ctcctgcctc atttcccagg catgcaccac 6660
cacaaccagc taattttta tttgtagaga cagagtctca ttatgttgtc caggctggtc 6720
tcgaactcca ggccgcaaga gtcctcctac cttggcttcc ctaagtactg ggatcgcagg 6780
cctgagccac cacagctaac ctgattttta taacaagggg gaaaataatc agaggacaag 6840
gccatgccct cccctacctc ccaggaatgc aggacacaag ggggcagcct acctcagggc 6900
agggccagcg gggttcctca tccttaggcc agtccccggg gctcagatga ttccagggaa 6960
ggccgacccg gctgactgcc acctccacca tcactctgca gagcggttga gcatgaaaga 7020
ccagctgatc gcacagagcc tcctagagaa acagcagatc tacctggaga tggccgagat 7080
gggcggcctc gaagacctgc cccagccccg aggcctattc cgtggagggg acccatccga 7140
gaccctgcag gggagctaa ttctcaagtc ggccatgagc gagagtaagt tggctgccca 7200
cacctcaagg gtgcagtctt gccggggtgg gctcctcagg gaaccccagg ccaggctcac 7260
ggctcattgt ggccgacacg gcaccctgtc caggacaggc ttctatgtgg gggggcccca 7320
ggagcagcac tgaccgcccc ccggtgcctg tgagagccag aagggcctta gacataatct 7380
gggccaagcc gctccttgca gacataaagc actttctccc cgttcaacaa ccgctctgat 7440
gctccctgca tgcagaggct ctaagccggg ccgtggggag gatctgaagt tgactaagac 7500
tggttcccta cctaccagag gttcctcttc ttcctgggag gtgggtcctg gtgatatccc 7560
cagaaaaaca cacgcacact ccctcagtgg gtccccacgc acagctctaa cacagccgcc 7620
aggcccctgg gctggccgct tgagcaggca gtcagcgccc agtctgctct ttcttttttt 7680
cttttctttt tttttttttt ttttgagatg gagtctcgct ttgtcaccca ggctggagtg 7740
cagtggcgtg atctcggctc actgcaacct ccacctcctg ggtttaagta attctgcctc 7800
agcctcctgg gtagctggga ttacaggcac ccaccaccac gcccagctaa tttttgtatt 7860
tttcgtagag atggggtttc accatgttgg ccaggctggt ctcgaactcc tgacctcaga 7920
tgatccaccc tcctctgcct cccaaggtgc tgggattata ggcgtgagcc accgcgccct 7980
gcctcaatct gctctttcaa cagtgaggga agccgactca gcccctgcct tccagagcct 8040
ttccgcagaa taagacggca gagacggggg tcagtctttc tccctccagg cccactgaca 8100
tccttccacc cggctccagt ggccattgtt cttggccccc tggggacact ctcatatccc 8160
gcaccttggc ctggaatgtt cttgtcccca cctctgtgcc tggccacagc ctgttggtcc 8220
tagagggctt tctgtagaca gcccttcccc acaaagtcac tctcccatat ggccattgtc 8280
acacaatcag tgtatggtca ttgtcatata acacaccaag tgtttcctgc tggccctggg 8340
cagggccaca tccagcattc gccagcccct ggcccagggc ggcctggtgg aaggagctgc 8400
aggagcctgt acccgccctc cccatggctg ccccacagcc ctgtcctctc acttagacgc 8460
agattggcct gtcctggtgg ccatacctga agtaagggtg tgcgcaggga cagtggggct 8520
gaaatcccac ggcacacaga agggggcagg cgatcaccac cccagtgagt ccctccgtcc 8580
acccgggtct cgctgcccca gcgctccgta ttcccccagc acacttccgc agggcgaccc 8640
gtgcctcctg tcccccttcct tccacagtcg agggcatcca gagcctgatc tgcaggcagc 8700

tgggcagcgc caacggccag gcggaagacg gaggcagctc cacaggcccg cccaggaggg 8760
ctgagacctt cgcgggctac gactgcacaa acagccccac caagagtaag agcggggccg 8820
tctcccctcc tgcctccagg gccgcccctc aggatgcaca ttaacttgta tggggtttcc 8880
tagaactttc tttttgttg ttgttgagac agagtcttgc tctgtcaccc aggctggagt 8940
gcagtggcgc aatctcagct ctctgcaacc tctgcctcct gggttcaagt gattctcccg 9000
cctcagccta ccaagtagct gggactataa gcacacgcca ccatgcccgg ctactttttg 9060
tatttttagt agaaatgggg tttcaccatg ttggccaggc tggtctcgaa ctcctgactt 9120
caggtgatcc gcccgcctca gcctcccaaa gtgctgagat tgcaggtgtg agccaccgca 9180
cccagccgat tttctggaac cttctgaaac cctccagaca tcgttggttg tgttgtgaca 9240
gcagcaccca gaccagcctg cccctctcca gcaggcgtct gtgctgcaga cgccaccatt 9300
cgggcctagc ctgggtttcc tacctgggca ggggctgggg gtggactggg aagcttcccc 9360
ctccccacgg gatggcagca tcctcatgcc cctggcttgg ggttctcaga tggcagtttc 9420
aagaagaaag tcagcagcac tgaccccagg ccccgagact ggcgaggccc cccaaacagc 9480
ccggacttga agctcagtga cagtgacatt cctgggagct ctgaggaatc gccgcaggtg 9540
gtacgtggat atccatttgc tcggtacagt ctgagtcgtc ataaggattc atgtgtgtga 9600
gcagcttctg ctggggttgt ccagttttag tcttattagc atcgacaagc attgtttgtt 9660
ttgtggtgaa taacagtatc tcagcccaga ttaatgcact ggcaggtgat ttcttcctgc 9720
agcctcctgg gaaaagcaaa gaggtcccca agaggaaggc tggggagggg acaggctcag 9780
ggcactcacc atggcttcat caacaggcga ggaacccccct acagggacca ccagctttca 9840
gccggggcag gtgtggtggt gcctaccaga tgccagatgt ccccggctat tcctttgcct 9900
ttgaatcaga aggccaggtg cctactcggc cctgcccagg tcagggggtc agaggccaag 9960
gccagttgcc tctgatcagt tctgaggctt ggacttgccc ctgtggccag agctggcacg 10020
caggcctcat agccaagccc actctacctc ccaggaggcc ccagcccctc gttttccaaa 10080
cacccagatt tgtacaagtc agctgaccag cagggcctgt gttcccagtc atgacccggt 10140
cctgggtcct tcctgttatc cacactaagg gatgtatttt caataataag caggggccgg 10200
gtgcagtgcc tcacacccat aatcccagtg ttttgggaga ccgaggtggg aggatcactt 10260
tttttttttt tttttggaga caaggtctca ctctgtcacc caggcttgag ggcagtggcg 10320
tgatcacagc tcacacagcc tctaactcct aggctcaagc aatcctccca cctcagcctc 10380
ccaagtagct gaaactacag gtgcgcgccc ccacacctga ctaatttttt aaatgtttgg 10440
tagatacagg gtctcactat gttgcccagg ctggtttcca actcctgggc tcaagcaatc 10500
ctcctgcctg ggcctcccaa agtgttggga ttataggcaa ggccgccata cctggccttg 10560
ggaggatcac ttgaggccat gagtttgaga ccagcctggg caacatagca agaccacatg 10620
tcgacaaaat atttttaaaa tagctgatcc tttggctggg tgcggtggct catgcctgta 10680
atcccagcac ttcaggaggc cgactcgggt gatcacttga gtccagggtt tgagaccagc 10740
ctggccaaca tggtgaaacc ctgtctctac taaaaatata aaaattagct ggacgtggtg 10800
gtggatacct gtaatcccag ctactcagga ggctaaggtg ggagaatcgc ttgaacctag 10860
gaggtggagg ttgcagtgag ccgagattgc accactgcac tctagcctgg gcgaccaagc 10920
aagactttgt ttcaaaaaag ttaaaaatag ctgctgggtg tggtggctca cgcctgtaat 10980
cccagcactt tgggaagcca aggcaggtgg atcacctgag gtcaggaatt cgagaccagc 11040
ctgaccaaca tggtgaaacc ccgcctctac caaatataca aaattagcca ggcgtggtgg 11100
cacattcgag taatcccagc tatttgggag gctgaggcag gagaatcact tgaaccagga 11160
aggtggtggt ttcagtgagc tgagatcgtg ccattgcact ccagcctggg caacgagcaa 11220
aactccatct caaaaaaaaa aaaaaaaaaa attaaaaata gctgatcctg gtggtgcaca 11280
cctgtagtcc cagctactca ggaggccgag gtgggaggat caggccacta cactctagag 11340
caaggccctg tctcaaaaaa aaaaaaaaga agccaggcat ggtggctcat gcctgtaatc 11400
ccagcacttt gggaggccga ggcgggcgga tcacctgagg tcaggagttt gcaaccagcc 11460
tggccaacat ggtgaaactc cgtttctact aaaaatacac aaaattagcc aggcgtggag 11520
gcttgagcct ataatcccaa ttactcggga ggttgaggca ggagaattgc ttgaacccgg 11580
gagctggagg gtgcagtgag ccaagatcgc gccactgcac tccagcttgg gcaacaagag 11640
cagaattccg tctcaaaaaa aaaaaaaaaa aaaaaagtta aaaaaaaaga agaagaagaa 11700
gaaggcttga gtctagtcgg atgggtcttg agccactctc tctggtttga cggtgtcctc 11760
ttcccaggtg gaggcgccag gcacggaatc cgatccccgt ctgcccaccg tcctggagtc 11820
ggaggtaggc gcccgcgggt ctccatctcc ccagggcctt gtgcacgcgc gtgtggcctc 11880
agcccgataa ctagcatcaa tctccctctc ctctccgcag cttgtccagc ggatccagac 11940
actgtcccag ctgctcctga accttcaggt acaggggcgg ggtggggccg gccacgcgtg 12000
ccctttcctg gttggctggg gcgcaggtgc ggctctcact cgcctggccc tggccctccg 12060
caggcggtaa tcgcccacca ggacagctat gtggagacgc agcgggctgc catccaggag 12120
cgggagaagc agttccggct gcagtcgacg cgtgggaacc tgctgctgga gcaggagcgg 12180
caacgcaact tcgagaagca gcgggaggag cgcgcggccc tggagaagct gcagagccag 12240
ctgcggcacg agcagcagcg ctgggagcgc gagcgccagt ggcagcacca ggagctggag 12300
cgtgcgggcg cgcggctgca ggagcgcgag ggcgaggcgc ggcagctacg cgagcggctg 12360

gagcaggagc gggccgagct ggagcgccag cgccaggcct accagcacga cctggagcgg 12420
ctgcgcgagg cccagcgtgc cgtggagcgc gagcgggagc gcctggagct gctgcgccgc 12480
ctcaagaagc agaacaccgc gccaggcgcg ctgccgcccg acacactggc cgaggtgagc 12540
gcgcagcagc cagtgtgcgc aggttggggg tgaccggttt gcacgtgcat ttgcacgagt 12600
gcatgcaggt gacagggttc gcgggtgcat gcgtgcagga gtgtaagagc aaacggcaca 12660
agataccctt gcattaacag ctcatgccgc agtacagacc tttctcggag ctggctaaat 12720
gcttaacaga acgctgttgc tcaaaacatg ccataaatgt tagaaacttc ttaaaaaaaa 12780
aatgcactcc tgtaatccca gcactttggg tggtggaggc aggcggattg cttgagggca 12840
ggagttccag gccagcctgg gtaacatggc gaaactccat ctctacaaaa aaatacaaaa 12900
atgagctagg catggtggca tgcacctgtg gtccccgcta cttggaatgc tgaggtggga 12960
ggatcacttg agcccgggag aatgaggctg ccatgagccg tgatcacgcc actgcactcc 13020
agcctgggca acagagtgag acatgagccg tgatcatgcc actgcactcc agcctgggca 13080
acagagtgag accccgtctc aaaaaaaaaa aaaaaatgaa gctgggtgcc atggtgtgca 13140
ccctagctac tcgggaggct gaggccggag gattgcttga ggctaggtat ttgagaccag 13200
cctgggccac gtagtgagac ctcatctcta aaaaaaaaat gcaaaaatta gcagggcgtg 13260
gtggcacata cctgtaatcc aaactacttg ggaggctgag gcagggggat catttgagcc 13320
caagaggctc aagagctatg attgtgccac tgcattctag cctgggtgac agagcaagac 13380
cgtgtctctg aaaacaaatc tataaaccat gggttctgtg aacagatgtg gacacggatg 13440
tgtgagatgt ttgtatatga gtgtgtgaca gtatgggtgc cactgagtgt caccacaagt 13500
ctgcacattt agcaggggtt ggggacgggc aatgaccagc agctccttca ggctgcacct 13560
ggctctgtcc agaacaggag aggtcgaggg tctcctgtgc acgaccctcg gggctctcc 13620
agaggccgca cagcaggaac ctcacattgg atgtatctgc tgttgtcccc tcaggcccag 13680
cccccaagcc accctcccag cttcaacggg gaagggctgg agggccctcg tgtgagcatg 13740
ctgccatccg gcgtggggcc agagtacgca gagcgccccg aggtggctcg ccgggacagc 13800
gcccccaccg agaaccggct ggccaagagc gatgtgccca tccagctgct cagcgccacc 13860
aaccagttcc agaggcaggc ggccgtgcag cagcagatcc ccaccaagct ggcggcctcc 13920
accaagggtg gcaaggacaa gggcggcaag agcaggggct ctcagcgctg ggagagctca 13980
ggtgagccgg ccccacccct tcgcctgggc ctggaaggtg caactggtca ggctctagcg 14040
gctcgctggg agccaggaaa ttcgggacac ctgtgccatc ctctggagag gaaaaccaga 14100
aggcacagct ggcatcgtgg ctgaggccac cccacacagg gccgtgtttg gtctccaggc 14160
ccaaaactga gatgatccac cctgcatctt ctccacctca accctcaaaa cagtgggatg 14220
gggaccagga gccttagaaa gggacacggg caagaaggca gagatccctg gccacgtggg 14280
atcagcacag ccgggacagg gacaggtgca ggcgcctctg ctcattgtcc cagttccaca 14340
gagccgttcc acagagtggg acacggagcc cagagcagcc ccagtgtttc cacggaggat 14400
gtccaggagc tcctctgggg gacacctccc tgggggacaa ccagggatgt tctgggatgt 14460
tctctgaaga ttaagggtgt gtcccgtggt cactagggcc tgtgctattt ccacaggagg 14520
ctcagcctcc tctccagatg ccgggtgact ctgcaggtcc ttccgcaggg aggttggggc 14580
cgttctccct gaatctgggg cttagcatct gagcagctca ggtcacggga tccaggccgc 14640
ccgtgggaag tgtcaggtgg gggtggccag gcccctctgc tctcggaggc tgccctggcg 14700
ggtggggaca gctggccagc ctggagctgg cccaaatacc ttctctcttc cagcgtcctt 14760
cgacctgaag cagcagctgc tgctcaacaa gctcatgggg aaagatgaga gcacctcacg 14820
gaaccgccgc tcgctgagcc ctatcctgcc cggcagacac agtcctgcgc cccccaccagg 14880
tgagccccca cccccctgaca tgagcccagt cccagggcag cggggctgcg gcaccacccg 14940
gcgactgctc agtctgaacc ctctctctgt tccagaccct ggcttccccg ccccgagccc 15000
accgccagct gacagcccct ccgagggctt ctctctcaag gccgggggca cagccctcct 15060
gcccgggccc ccagctccct cgccactgcc ggccacacca ctcagcgcca aggaggacgc 15120
cagcaaagaa gacgtcatct tcttctaaaa gggccgtgac tcaaggtgca aggcccctcc 15180
ctgccctgcc caccccttcct gctctctggg gacccccatg gggtcaccat gcccacccag 15240
ctgtcccctc ctcttcccta gcaaaccact gatgaccgcc tggcaggggc cagcctgtcg 15300
gtgctctggg ccttgcagct gtttctgtag ggttagcggt ggtgccgggg tcactttctg 15360
aatctctttt tttttttttc aaaaaggaaa gtttttaatg gaaagttgag ccagaactaa 15420
accagggagc tgtctgaaat catagcaccc catccgggtg gcggggagat caactccgag 15480
ctgtttttcc gaggcagtga ggaacggtgc cggctctgca cggagctgag gacaggacag 15540
accttgcttt gagaaggagc tgccggccgg ggccacgctc cacagccgcc gcgcgacagt 15600
ggagccaagg gttagggcac caggaggggc caggtggcgt cggcagcatc tgtccccaga 15660
atcaggcaga atccacttcc caaacagagc cccacgcagg ttcaccatga acctcagggt 15720

cagggaatga gccaggcacg gggcatgggg cagagagggc cacggggcag ggcccactga 15780
gggaacatca gtggccctcc agtcaggttc tgtgggtttg gaagcccatc gtgaaagggg 15840
ctgacctttg cccctttta cttggcattg gttttgaaac cagctgtttc ccaaactctg 15900
cttcccaagg gcaaccgttg ctgttcacac gctcagcctg tctgggggag cgggcctcta 15960

```
gcttcagcca gggcgggtac acaccctggg cacagggtcc tcagcccccg ggaaatgagc   16020
tcccagggct ggcgtcccac cttccaggtg ggggctggca catcacagac tgtcgagagc   16080
gccatgtccc agggcatgca gaggatgcac ctagagacgt tgcagcaagt ggacaagtgg   16140
ccgctgtgcg ggcccctcgc ttgtagtgag ctgttgcagc ttacggtccg ttccctggag   16200
gggtggagga aggaggtgtt gggcagcatc aaaggtgctg ggacatccca gggtggtgag   16260
atccatccac gatccagctc cggtggagaa agggcccatg tcaagccttg ttctgcaccc   16320
caagcattgg tggtaggact gggtcctggc tgatcgtcct tgttcccagt ggggtacatg   16380
tgagcccctg ccagggccaa gtccttctcc cgaacccagg gtcctgggaa ctgcagatcc   16440
cgggggggatt cagcccttct cccactgtgc tggcagaggc actcctgtga cgctgaatac   16500
agtgaacagg gacattcccg ccactcgggg acagatgggc acaagggagg ggaaactcca   16560
tcaggaagtg ctcccctggg cagaggcgcc cactgggtgc tgtgggctca ggagggggcg   16620
gggcaggagc tggtgccaac cgggaaccag agccccacag ccatacagcc cattggtgac   16680
aaggtcctga gaacacagtg gccaggtgtc cccaggctcc tggcccctcc gacgacctca   16740
actctgccca gcccggtccc tggccatcag cgacgctgtc cgcccccgt cagatcccat   16800
gtgtgccatg tttatcatca gtgttttgta tttttgtact gagtatcgga gcactttaca   16860
gaagctgact gtacattcct gttctgttgt gaagagaaca ttcccagacc ctggcaccct   16920
cctgagccgg cgtgtgccgg tccagccctc cgagatgcca caattccttg gatgggggag   16980
aagttcaagg aatttctgct cggccacgcg gtgggaaccc cgcgtccccg ccatgtggca   17040
gaggggtctc agtcgtgcta ggcatcgggc ggcagcgccg acagcccttc cctcgccagt   17100
gcccctcggc cactcctggg ttggagcccg attttatttg taaagttgac agtcgagcaa   17160
atgttcctat tttcgtggga tctgcacacg tctttgtcag ttgtggtcat gatcttagtc   17220
acctgctaat tatttttaca atgattacaa catttcctca ctgcgggata tttctgaccc   17280
gctttagaac ttaagacctg attctagcaa taaacgtgtc cgagatgagc ggtgactggc   17340
gtgcacgttc ttggaggttt tatttgagga ttttcttctg cccaaacgtt gctgcaggag   17400
tagcaggttc tgttggggggc tccccatggc tcctgcccct cagctcgctc acctgccctc   17460
cgtgtcctgc ttaacgctct gaagacccct tcaggtccag tccccaggag ttgttggggg   17520
ttttgttttt gtttttgttt ctgttttttt ggtttgtttg tttttttgag acagagtctt   17580
gctctgtcgc ccaggctgga gaccagtggc aggttctcag ctcactgcaa cctctgcctc   17640
ccgggttcaa gcacttctcc tgcctcagcc tcctgagtag ctgggattac aggtgtgcac   17700
caccacgccc agctaatttt tgtatttttt tagtagacat ggggtttcgc catgttggcg   17760
aggctggtct cgaactcctg acctcaagtg atctgcccgc ctcagcctcc caaagtgctg   17820
ggattacggg cacagggcag gagtggggcg tggaggtgcc tgtggagggt cctgcccacc   17880
gggtacactt caggctttgg tggcgcctcc acgaaaatct ttttgctttt aatccacaat   17940
aaccgcaaag gctggaactt tcacttcctc catctggggt gagaggcagc ttggccatga   18000
gtatgaaatc atcatttcca cctggtctga gtcccggtcc agtgaggcga ggaccctgct   18060
gtttcaacag agggtttccc atcacaaggg gactaggtgt aaagtcgatc actagctcac   18120
taggaagcgc aaaaagggaa ctctcaggca gcactgtcca ccaggaagat gatgcaggcc   18180
acgtgtggaa tttttcattt ttagtagcca tattaaaaaa gtaaaaggag ggccgggcac   18240
agtggctcat ccctgtaatc ccagcatttt ggggaggcgg gggctgaggc aggaggatag   18300
cttgagccca ggagttcaag accagcctgg gcaacatagt gaaacccctt cacagaaaaa   18360
ttaaaaaatt agccaggttg gccgggcgca gtggctcacg cgtgtaatcc cagcactttg   18420
ggaagccaag gtgggcagat tacgaggtca ggagatcgag acc                      18463
```

```
<210> 27
<211> 15597
<212> DNA
<213> Homo Sapiens

<400> 27

ttttgtcgct ggcaaccccc tctcgaaagc gtaaacgctc ccagggagcc accaggaaca      60
gatgtggatg ggcagctccc acccagggca cagctgtcat tcactcggct cagaactggg     120
aagccggccg ggcacagtgg ctcacgcctg taatcccagc actttgggag gccgaggcgg     180
gcaggtcacc taaggtcagg agttcaagac cagcctgacg aacatggtga aacctcatct     240
ctactaaaaa tacaaaaaat tagccaggca tggtggtgga cacctgtaat cccagctact     300
caggaggcta aggcaggaga atcacttgaa cccaggaggc agaggttgca gcgagccgag     360
attgcgcctt tgcgctccag cctggcaaca gagggagact ccttctcaaa acaacaaaac     420
aaaaacgaac tgtgacgccc atctaagcag gaggcactcc ccatgcaaag gcatagcgtg     480
ttctctggaa caggcgcctg ggagaaccga tccactcacc tgttgtgcag ccttgtcggc     540
gagcagcgcg aattccacag acaggccttt cagagcctgc ttcagggacc cccacgtgct     600
gctattcaga gcggcccagg agggcagcgg ggtcgtgtca gaccctattg cactgaggga     660
```

```
gcaagcacac aggagaggag acactcaagg cctggagctc acctggcagt cgagtctggc     720
atcccccccg acagatgtcc tccacggcaa cctgcgtgac cccgtcctag gaccctgctg     780
ctcaaagagt ggcctgtggg gaccctggga gcccgggaga aagggtggcc ctggggctgc     840
acctcaggac ctgctgcgtc tcttcttccc aaccagcccc caggtgacat gtgtgcacag     900
gtgacgggca ctgctggggc aagcagagtc ccgggtgggg tctcccctcc cctcaggcaa     960
aggaagcgct gccagcccat ggtctgttct tccccaagtc aaagggatct tttttttttt    1020
tgagacggag tctcgctgta tcacccaggc tggagtgcag tggcgcgatc tcggctcact    1080
gcaacctccg cctcctaggt tcaagcaatt ctctgcctca gcctcccgag tagctgagat    1140
gacaggcacc cgccaccacg cccagagaac ttttttttgt atttttagta gagacgggtt    1200
tcaccatctt ggccaggctg atcttgaact cctgacctcg tgatccaccc acctcggcct    1260
cccaaagtgc tgggattaca ggtgtgagcc accacgcctg gcctgggatc tttttttaaa    1320
gtctcatcct gacagtgaaa aaactaccag actagaaact ggtgagcatt tactcgtgct    1380
cagaaatccg tgtttcaggg ccgaaattct cccgtgtttc ttccaaatat gaccccccat    1440
gtgcctgagc cactttctgc cccttctgag atgaaacagg tcaggatgct ggttattacg    1500
gtaaacgcac tttccgccca agcccttcac cagcatggat tcccgtccca ccgcgcgtgc    1560
ccctgcagaa gctgaaaggt cacctacctt agctccttcc cgaatatgag aagatctgcc    1620
gcattgtcga tggcccgcga tgcgatccgc tcggccctgt cgcgaagctt gtgaaagcta    1680
ttgtagatgt tccggatcag ctcccggctg atggcaaact gagcctggat gtcagctggg    1740
aggaagtcct gacatcatga tggggggaga gacactgtgt tagtcgctgg ggagcacctg    1800
tcagacggca gcagccggtc cccagaagc tgagccacgg gagacacagc aggtccatga     1860
gccaccccgg gagccccact cctccaggcc tgcagggatc actctgtgtg tccaaaaaag    1920
aaacaaaaac catcatcacc tgcatgccac aaacacaaag acaaatgatc ccatgggatg    1980
aggaccacag agtccactga aatctcatca cactggagga cctatccaaa aacactcaaa    2040
taaggccagg ggcagtcact cacacctgta atcccagcac tttggaggct gaggcaggtg    2100
gatcacctca ggtcaggagt tcaagaccag cctgaccaat atggtgaaac cccatctcca    2160
ctaaaaatac aaaaattagc tgggcatggc ggcatgcacc tgtaatccca gctactccag    2220
aggctgagac aggagaattg cttgaatccg ggaggcagag gttgcagtga gctgagatcg    2280
cgcaaccaca ctccagtttg ggcgacagaa cgagactctg tctcaaaaca aacaaaccaa    2340
caaaacactc gaataataat cttgccgggc gcagtggctc acgcctgtaa tcccagcact    2400
ttgggaggcc gagtagggcg aatcacgagg taaggagatc gagactatgg tgaaacccca    2460
tctctactaa aaatacaaaa aattagccga gcctggtggt gggcacctag tcccagctac    2520
ttgggaggct gaggcaggag aatggcatga accccggagg cggagcttgc agtgagctgc    2580
gatcacacca ctgcacccca gcctgggcaa cagagcgaga ctccgtctca aaaactaaca    2640
atactaatct ttagctggat acagtgactc acacctgtac cccagcgctc tgggagactg    2700
aggcaagagg attgctagag cctgggagtt taagaacagc ctgggcaaca tagtgaaacc    2760
ccatctctcc caaaaaatca aaaaattagc caggcgtggt ggagcgcctg tggtcccagg    2820
tgcttacaga gggctgaggc aggaggatca cttgagccca ggagttccat gctgcaagga    2880
gccatgatca caacactgca ctccagcctg ggaccctgt ccccagaaaa caacaaaaat     2940
aaaaacaaac aatcttcaag ttaaacacaa tgaacataaa ttcctgaaag actctccaca    3000
tggggggaag gcctcttcta tgccgcgttt attcccaaag actttttata agatttgata    3060
ctaactatca gagttcagaa aacagaaagc tcagagagta aaatgaaggt aaaagatttg    3120
gccaggcatg gtggctcatg cctttaatcc cagtactttg gggggccatg ccaggaggat    3180
tgcttgaggc caagagttca agaccagcct gggcaacata gtgagacttc atctatacca    3240
aaaacaaaca aacaaatcag ccaagtactg tggtgcatgc ctgtggtccc agctattctg    3300
ggaggctgaa gtgggagaat tgcttgaact caggaggtcg aggctgcaat gacctacgat    3360
tgcaccactg cactatagcc tagaggacag agcaagacct tgtctcaaaa aataaaataa    3420
aatagatttt taatttttgt atgaagaagc aggttttttt ctggtttttt tgttgttgtt    3480
gttgtcgtct ttgacacaaa gtctcactct gttacccagg ctggagtgca atggcacgac    3540
ctgggctcac tgcagcctct gcctcctggg ttcaagcagt cctcttgcct cagcctccca    3600
aatagctggg attacaggtg cctgccacca agcctggcta attttttttt tcttttttttt   3660
gatggtgtct ctgtcgtcca ggctggagtg cagtggcgca atcttggctc actccaacct    3720
cttcctccta ggttcaagca attctcctgc ctcagcctcc tgagtagctg gaattacagg    3780
tgcacgccac cgcacccagc taatttttgt atttttagta gaggcggggt ttcaccatgt    3840
tagtcaggct agtcttgaac tcctgacctc gtgatccacc cgcctcggcc tcccaaagtg    3900
ctaggattac aggcgtaagc caccacgccc ggccggtttt ttggggtttg ttttgctttt    3960
ttttgttttc ctttttttgtt ttgtattttt attagagatg gggttttgct atgttggcca  4020
ggctggtctc aaactcctga cctcaagtga tccgacagcc tcagcctccc aaagtgctgg    4080
gattacagga aggagccacc gcgccagcct gaagaagagg tttaaaaaga aaaaaccaaa    4140
aacaaaaaaa ctaccaagta gctaccttag tctttttttgg aaaaagacca aactttaaac   4200
atttaataat aaacaaatgt aacaatgaat ccccagaaat ctgaaaaggt gctgacacaa    4260
gcatccgtct gtctgagtca cagaaaaggg cagagggcac gaggtgcctg gagggcctga    4320
```

gggcagagct cagccgcaac ccggccctgc tgcttagagg ctcggggcct tggcaaatga 4380
cagcctccct gagcctgttt ctccatccgt aaaacaggcc tcccagccct gtgtcggagc 4440
aactgaaacg agggttaaat gaggaaacca tgcgacagat gtcagcataa tccctgccac 4500
gcagaggagg caaaatcact ttgaagggtt aaaacttgtt tctctggtta aaaagggatg 4560
tactcttcaa agttagaaaa ataccttggc cctctccctc cccctccccc tccccctccc 4620
cctctccctc tccctcaccc cacagtctcc ctctcatgcg gagccgaagc tggactgtac 4680
tgctgccatc tcggctcact gcaacctccc tgcctgattc tcctgcctca gtctgccgaa 4740
tgcctgcgat tgcaggcacg cgccgccacg cctgactggt tttggtggag acggggtttc 4800
gctgtgttgg ccgggccggt ctccagcccc taaccgcgag tgatccgcca acctcggcct 4860
cccgaggtgc cgggattgca gacggagtct cgttcactca gtgctcaatg gtgcccaggc 4920
tggagtgcag tgacgtgatc tcggctcact acaacctaca cctcccagcc gcctgccttg 4980
gcctcccaaa gtgccgagac tgcagcctct gcccggccgc caccccgtct gggaagtgag 5040
gagtgtctct gcctggccgc ccatcgtctg ggatgtgagg agcccctctg cctggctgcc 5100
cagtctggaa agtgaggagc gtctccgccc ggccgccatc ccatctagga agtgaggagc 5160
gcctcttccc agccgccatc acatctagga agtgaggagc gtctctgccc ggccgcccat 5220
agtctgagat gtggggagcg cctctgcccc gccgccccat ctgggatgtg aggagcgcct 5280
ctgcccggcc gagaccccgt ctgggaggtg aggagcgtct ctgcccggcc agccaccccg 5340
tccgggaggg agatgggggg gtcagcccca ccacctggcc agccgccccg tccgggaggg 5400
aggtgggggg gtcagccccc cgcctggcca gccgccccat ccgggaggga ggtggggggc 5460
gtcagccctc cgcccggcca gccgccccat ctgggatgtg aggagcgcct ctgcccggcg 5520
agaccccgtc tgggaggtga ggagcgtctc tgcccggccg ccccgtctga gaagtgagga 5580
gaccctctgc ctggcaacca ccccgtctga gaagtgagga gcccctccgc ccggcagctg 5640
ccctgtctga gaagtgagga gcctctccgc ccggcagcca ccccatctgg gaagtgagga 5700
gcgtctccgc ccggcagcca ccccatccgg gagggaggtg gggggggtca gcccccccgcc 5760
cggccagccg ccccatccgg gagggaggtg gggggtcagc ccccccgccc ggccagccgt 5820
gccatccggg agggaggtgg gggggtcagc cccccgcctg gccagccgcc ctgtccggga 5880
gggaggtggg ggggtcagcc ctccgcccag ccagccgccc cgtctgggag gtgaggggcg 5940
cctctgcccg gccgccccta ctgggaagtg aggagcccct ctgcccagcc agccgccccg 6000
tccgggaggg aggtgggggg ggtcagcccc ccagcccggc cagccgcccc gtccgggagg 6060
tgaggggcgc ctctgcccgg ccgcccctac tgggaagtga ggaggccctc tgcccggcca 6120
gccgccccgt ccgggaggga ggtggggggg tcagcccccc gcccggccag ccgccccgtc 6180
cgggagggag gtgggggggg gtcagccccc ctgcctggcc agccgccccg tccgggaggt 6240
gaggggcgcc tctgcccggc caccccctact gggaagtgag gagcccctct gcccggccac 6300
caccccgtct gggaggtgtg cccaacagct cattgagaac gggccaggat gacaatggcg 6360
gctttgtgga atagaaaggc gggaaaggtg gggaaaagat tgagaaatcg gatggttgcc 6420
gtgtctgtgt agaaagaagt agacatggga gacttttcat tttgttctgt actaagaaaa 6480
attcctctgc cttgggatcc tgttgatctg tgaccttacc cccaaccctg tgctgtgtcc 6540
actcagggtt aaatggatta agggcggtgc aggatgtgct ttgttaaaca gatgcttgaa 6600
ggcagcatgc tcgttaagag tcatcaccac tccctaatct caagtaatca gggacacaaa 6660
cactgcggaa ggccgcaggg tcctctgcct aggaaaacca gagacctttg ttcacttgtt 6720
tatctgctga ccttccctcc actattgtcc catgaccctg ccaaatcccc ctctgcgaga 6780
aacacccaag aatgatcaat aaaaaaataa attaaaaaaa aaaaaaaaga aaaaaaaaag 6840
aaaaatacct tggccctggt agccagctta cagttcagga attcgtcccc gacgcactgt 6900
gctgactcct ttaacttgtt ctgcacatcc tgcaaaagga aaacacacag cttcaccctc 6960
ttctactagc agcaagaaag ctgctgtggg gtatgggtca ctgtggagcg ggaggtcgtc 7020
tttcctcagg agaaacacat ttccatatgt tggctttgac attagtattc tgcaatctct 7080
tgactctccc ttagatgttt ttctcaccaa acaggacagc ctggcttcac agcaggagca 7140
ccacaaacct gcagcctctc tccagaggca cagccactcc atccgcagac ggggggcagc 7200
tctccagaga aacagccact ccatccacag accaggggcg gctctgcaga gaaacggccc 7260
ctccatccac agacccgggg cggcgtcagc cccagagata cctgagaccc tggtagcttc 7320
caaaaacgca atagaacagg gcaaaagtgt cttccttcct gggaatgcat ccatggcgcc 7380
agggacacca gagcccaggg agctgctggg cagagggaca ggcccagccc ctggcaccgg 7440
agccctctca tctgaaagag gcctcctggg caaagccgtg ccttaaagtc aggagccacc 7500
caggagatac aaggggaaac cctaatatgc agctgtttgc gtgtccagga attctcaggt 7560
ttcagagatg ccttaaagat catctgactc aactctcatt ttacaaaaaa aaaaaaaaaa 7620
aaaaaaaaaa agacctgagg cccagcatgg tgcgtgcctg tagtcccagc tactcagagc 7680
ctgaggcggg aggatcgctg gagcccaggg gttcagggcc agcctgggta acatagtgag 7740
accccatctc taaaaacaga aagcaagcaa gaaaactgag gccgggaagt gacagagaat 7800
gatacctgtg ggaaggccac agggaacgtc ctgcccccag aacagggata ttgcccacct 7860
gtgacccact gcacccctgc ccaccgcccc tcccaggtcc tgccacattc ctctgctggc 7920
aatgcagatc gcctccaaag gaaggtttcc aaccccaggg ctcaaaagat cccactgcct 7980

```
gctccagaca tccccggggg catcccaggg acatctcagg cacaggggga aggcccctct    8040
gcctccgaga ggaacgaaga caaaaggaac caaggcttgt gtgaagtcag cctgggagag    8100
ggggtgtctt cctgcagcag ggacaaggac agaggccgtc aggtccccat tcaagacggg    8160
gtttccctga aatagaggag agcctggaaa cctctattcc agtggagccc ccgcccctcc    8220
ccgcacaggg agagggtcct gcaggcacag gtggaggaga agcacaggac ccgccttccc    8280
tggaggggct ggcagctgct tcctcctcca ggaaacagga gctgggctgc agggttgttt    8340
tttgtttttt tgagacaggg tctcactctg tcgcccaggc tggagtgcag tggcacaatc    8400
acagctcact gcaagctcca cctcctgggt tcaagcgatt ctcatgcctc agcctcccaa    8460
gtgactggga ttacaggtgt gcaccaccat gcctggcctc agttttgttt tggtagagac    8520
gaagtctcgc tatgttgtcc aggctggtct caaacgcctg ggcttaaggg accatccagc    8580
ctcggcctct ccgagcgctg ggattagagg cgtgggccac tgtacctgcg aactgtagct    8640
tcttgacaca caataaggct gtgccgcagc agctgggagg agggaagccc cactcagacc    8700
tctgacggag tgcatgtaca acaagttaat tcccggatgc aggtgtgggc tgtggctctg    8760
acaaggaggc tcagcctgtg ttccagtggt ccttagtcag acagggcctt gttttaagtc    8820
aacacttctt cccatctgga gtctctggca cctccctaaa ccctccaaac catccagcct    8880
aaatcctcaa agtgaccaca gagatccatg atctccacgc ggatttacaa aatcctgcaa    8940
agtcaacacc agggacacct ctgaaaatca cattcagact gttatctgga aaggaaagaa    9000
gtcttgtcca tgtcattcct gctgggcgtc caaacaaggg accaggagga taagaaacca    9060
cacctgagag aggaaaaggc gggtccggag gctcggggac tccccggggc cgggacatgg    9120
gcagggcaga cacactcacc gagccgctga aggacaggaa gagcttgagg accacatcct    9180
cggagaacag gggtgtcgc gccaccaggt tgacgaagcg cttcagggct ctcctcctgg     9240
cctcgatgaa ctccctgtca gctggaggag cacacggggt cactggacag agtccagggc    9300
gccggccccc atcttctgct ctgggcgagt tctcaaaact ctccctagag gtggcagagg    9360
gcccagcggc tagcagaggg cactggctgg gcccccagtg ctgctcagac aatgggtctg    9420
aaaaggccgc ccagagcaat aacctcaccc ccagaggccc cctccagcca cacacacttg    9480
ggtccaggag ctgccaagga aaaccacaga tctgcttgtt tccatcggga gtcctgggaa    9540
ccacgtgagt ggtcacttta atcacctccg ttaaagacat cttccataat cccctcctgg    9600
cacttctcac tgcaaacaaa tgggttttgc tacgctctaa cactaacgca acccaggtct    9660
tcttttcttt tttttttttt tgagacagag tttcgctcct gttgcccagg ctggagtgca    9720
gtggcgcaat ctcggctcac tgctacctcc gccttccagg ttcaagcgat tctcctgtct    9780
cagcctctgg aattagctgg gattacaggg gcccgccaac atgcctggct aatttttgta    9840
tttttagtag agatggggtt tcaccatgtt ggtcacgctg gtctccaact cctgacctca    9900
ggtgatccac ctgcctcggc ctcccaaagt gctgatatta caggcgtaag ccaccgcgcc    9960
cggccgagtt tttctattca ctgtgcttat aacttactct gcaaaggatg caattactca   10020
ttgctttcat tcgggttaaa tattttcact ccaaggggaa aaaaagtaaa agcatatact   10080
gatgcattca ggacatttca atctgaacac aaacaaaaat ggtatttaaa tacttttttt   10140
gagacggagt ttcactcttg tcgcccaggc tggagtgcag tggtgtcatc tcggctcact   10200
gcaacctcta actcccaggt tcaagcgatt ctcctgcctc agcctcccaa gtagctggga   10260
ttacaggcac ccgccaccac acctggctaa tgtttatact tttattagag aggaggtttc   10320
accatgttgg tcatgctggt ctcgaactcc tgacctcagg tgatccgccc acctcagcct   10380
cccaaagtgc tgggattaca ggcatgagcc accagcgccc agcctttaaa tactattttt   10440
aatgtattaa gaaatacagc caggagtgtg gctcacacct gtaatcccag cctcttggga   10500
ggctgaggag ggcagatcac ttgaggccaa ggagttctac atcagcctgg ccagcgtagc   10560
gaaaccctgt ctctgaaaaa taccataaaa gaggccaggc gcagtggctc acacctgtaa   10620
tcccagcact ttgggaggct gaggcaggtg gatcatgagg tcaggagatt gagaccatcc   10680
taacacggtg aaaccccatc tccactaaaa atacaaaaaa aaataattag ccaggcgtgg   10740
tggcgggcgc ctgtagtccc agctacttgg gagactcagg caggagaatg gcgtgaaccc   10800
ggcaggcgga gcttgcagtg agccgagacc gcaccactgc actccagcct gggtgacacg   10860
gcgagtcttc gtctcaaaaa caaaaacaaa aacaaaaaac accataaaag aggccaggtg   10920
cggtggctca cacctgtaat cccagcactt tgggaggctg aggtgcgcgg atcacgaggt   10980
caggagattg agaccatcct ggctaacacg gtgaaacccc gtctctacta aaaatacaaa   11040
aaaattagcc aggcgtggtg gcgggcgcct gtagtcccag ctacgcggga ggctgaggca   11100
ggagaatggc gtgaacccgg gaggcggagc ttgccatgag ccgagattgc accactgcac   11160
tccagcctgg gcgacagagc gagactccat gtaaaaaaaa aaaaaaaaca ccataaaaga   11220
aagaaaacaa acaatacagg taggtggctg gggctccctc ctggctcagc actggcaggc   11280
ctcttccgat gaggctgcac ttccttaaat gcgcaagcgg cccagcgcgc gtgtgcagtt   11340
tccaggcgct gaacgccagc cagcgctcat caactcggat gcccaggcct gagccagcat   11400
cacacatccc tgtcgcgtcc tctgctctct gctgccctga gcgcccgtgt gcccttcagc   11460
gtgagctgaa tgatctgctc tgtggccttc acatgcggcg gggtgaaaat cacatcatca   11520

gaccctgggc tgcttgctca gtgaccgatg agaggcagaa gcatttatgc atcatcgtac   11580
```

```
gatttcagat gcggggaact ggcgactgtg ttagctccca tgctgcgtcc ccgtccagga   11640
cacggtgact gtggaagggc tgggaacggc agaaagccat gatcagctct aagcactcat   11700
ttccccagca tgttttattc agaatttgag gaacgggctg agggagagac caccagtgag   11760
atcagagctg gcagccgcca tcccaagctc tgcagcccag gggacacag gcctggggag   11820
cagctctgag gtcgtctgcg atctcccacc tggcccgagg ccatcttcag gcagagaggt   11880
gaaggtgagg cagccgtgtg ctgcttttc tctcctgcaa gatgcagctg acatttccta   11940
cttgcacaac tcagttctct tccatgaggc caccgagcgc tcccatggtg cccatggctc   12000
aggctgccat ggctttgcag atgcaggcag ggcaatgcca gccaggtgag cacacagtgc   12060
tcctggcagc cctctcggga gctctcggga gccctggcca ctgcactctg gagaggagaa   12120
ctagggttct ctttcacacc tgcacagcaa gggcttcccc acctgtcagt catgcagctg   12180
aaggctctgg tgggccaagg ctgccgggtg ccagtctccc tgcctgcacc aaggctgggc   12240
tcaagccaca gctctgtccc agatgccaaa aggctggagt ttcccccgca gccctgcacc   12300
tgccccggtc tacagtgggg tccatccctg agccaggacc acagggtcca ggagatgggc   12360
tcgctccctc cgcccccggt gggcagcacg cctggcttta cctcccagca ttctcttggg   12420
tggcagggca ggcaccatac ggtaggggaa cttgtgcagg agcatctcct ggaagaccac   12480
gaagtcattg taccgtctgt ataccgagga cttgaagcgc tgcaagagaa gggtcggtgc   12540
ttagatccga cgttggaaac tatgctgtcg cgtcacttcc cctcaacaga gcccgggaaa   12600
acgacagcac caagtgcatc ttctctcacc aggcctttac taaatggaga aaccctgtga   12660
cggcttcgta tatggaaata caacttttaa actttgtata gctcttaggc cgggtgcggt   12720
ggctcccgcc tgtaatctca gcattctggg aggttgaggc gggtgaatca cctgaggtca   12780
ggagttaaag agaccagcct ggccaacatg gcaaaatccc atctctacta aaaatacaaa   12840
aattagctgg gcatggtggt gggcggctgt agtcccagct actttggagc ctgaggcagg   12900
agaatccctt gaaccctgga gacagaggtt gcagtgatcc aagatcgtgc cactgcactc   12960
tagcctggac gacaaagcga gaccttgtct taaaaaaatt aaaaaaggcc gggcgcggtg   13020
gctcacgcct gcattcccag cactttggga ggccgagaca ggcggatcat gaggtcagga   13080
gatcgagacc atcctggcca acacggtgaa acctcgtctc tactaaaaac acaaaaaatt   13140
agccgggcgt ggtggtgggc acctgtagtc ccagctactc aggaagctga ggcaggagaa   13200
tggcgtgaac ccgggaggca gagcttgcgg tgagctgaga tcacgccact gcactccagc   13260
ctgggcggca gagcgagact ccatttcaaa aaaaaaaaaa aaactaaaca aatggtatga   13320
agcatagtgg cttcccacct ggtcagtgac tggcccgtta cacacacatc agggtgacca   13380
aactgccccc ttgacaaatg ttccctatgt ggcaatgaat ttacttaagg gacgggcagc   13440
ttagtaagac tggacaaaag gccagccgct ggctccctgg caggcaacgg cacgtctcac   13500
acgctcagag ttgcctcacc cccaggctgc caggccccag aaggaagggg tggctcgctc   13560
tgctcccccg tggcatccca ccagctggtc cacatccccg actgcggccc agagggccgg   13620
gaaccacggc aaagccagac agcaaggcgg ctctgacacg gtcctccaag ggagtcctcc   13680
cctctgaccc gctttgtcct ttggtagcag tactgacaac cattcggacc gtgtggccac   13740
aggctctgga acacagtgaa gcacagcaga gcagacggca gggaggggag accggctcgc   13800
ttgtccttaa ggccaggtgc agtggctcat gcctgtgagc ctagcacttt ggcgggccaa   13860
agtgagagga ctgcttgagc ccaggagttc gagaccagct gagacaacat agcaagtctc   13920
catttgtact ttaaaaaaaa aaaaaaaaaa aagactgggc atggtggctc acgcctgtaa   13980
tcccagcact ttgggaaacc aaggcaggag gaccacttga gcccaggagc tcaagaccag   14040
cctggacaat atagtgagat cccatctcta caaataattt aaaaaactgc ctggtgtggt   14100
ggcatccgcc tgtggttcca gctactcagg agactgaggt gggagggttg cttgagcctg   14160
ggaggttgag gctgtagtga gctatgatcg tgccactgca ctccagcctg gttgacagag   14220
caataccctg tctctttaaa aacaagcaaa caaaaagaca caacttttac ccaggaccat   14280
ctgctcatga cgcttttcaa acaagcccct ggcagtgccg tccggccggg gctgggccag   14340
ggccgccctc tcccacacag gagtgggccc tgctctgtct gtgcgaatcc cacaagccct   14400
gctgactcca cacctcccaa ggacagtgtt agctcatcaa ttactcaatg cccaggatga   14460
acaatgaaaa ctcccatcag aatctgtcgt aatggaactg agacaacgaa gcaagggaca   14520
gagagagcca cggtgttgct ccagacgggc atcaggcact cggtgtctgt gtctcacctg   14580
cccaatatcg ggatgcccag caacaggagg gccgtgttat taaaggggat tagagtgaca   14640
catattgatc ctgccatgca aatttcattc actgtttggg gtccggagtc gatggggctg   14700
acattctaat cctcgggggt ttcccactat tacaaagcga ggctggtgtg gatcccgctt   14760
tgtgctctgt ggaccgtccg cgggagattc ctaagaaaca ggcctccagg ccgggcgcgg   14820
tggctcacgc cagtaatccc agcactttgg gaggccgagg cgggtggatc ataaggtcag   14880
gagatcaaga ccaccctggc taacatggtg aaaccctgtc tctactaaaa aaaaaaatac   14940
aaaaaaattt gccaggcgtg gtggcgggca cctgtcatcc cagctacttg ggaggctgag   15000
gcaggagaat cgcttgaacc cgggaggcag agtttgcagt gagccgagat catgccactg   15060
cactccagcc tgggcgacag agcaagactc cacatcgggg aaaaaaaaaa aagaaacagg   15120
actccagagc acagtggtgg ggaggacaaa ctggaccagg ggtgaccagg tggggacgtg   15180
gctgggccag agtgaggtgc ggggagcaga ggtgtggaaa agtcacctcc gcagcctgac   15240
```

```
tctgctgcga gttctggatc ttgcctgtaa gccagccaca cagactcacc cttcagcctt   15300
ctcctgtcac accactcctg ccctgagatg ttgagacgtg accctttctt ccccctcctg   15360
ccccgacaca cacacaattt gccagttacc tggctggaaa cctcatactc cacatgcttc   15420
aggaagaggc ccttcttctc cggaatgagc tccacctgca cggtgtccct ggccagcagc   15480
tcctgcaggg tgtgggacag cagcagcggg ttcccctgcg gcatctgcat tcgactgggg   15540
gctgggacct gctgcacgat ggcctggggc tcgatggcct ggggtgtcgg cagatct      15597
```

<210> 28
<211> 3143
<212> DNA
<213> Homo Sapiens

<400> 28

```
ctattcaaat ggggccgcgt cccgattatc agtgcaatgt gatcacttga ttttaaacgc      60
aactatgcgt ccaaatggct gagtgcaaga ggtcggccgg agatagggcc gagtgcgctg     120
ccccaagctg cgatccctgc caagcactca cggacaatgc actggtttcc tccagggagc     180
gtcttccatc cagggcagca gtaggagtgg aatctggagc cgcacacgtt gggcctgtga     240
tggacaagcg cggtcacgta acagataggt agagggatgc agcaacatat aaacggtctt     300
cgtaagcaat gccccgagcg agtagatttc aggacaacaa acaaatcaaa attacacttc     360
aaattattgg agatcgggag gcagacgtgg aagcaaaaga gctagatgct ggaatgcagg     420
agggtcagaa atgtctaaac actgcctttc atctgtcgtc ccttgcctgc ctttttaaag     480
ggcgaaaaga ggcatggaaa catataattt gctactattc tagcttccag atgcagagaa     540
caaagggacc attccgttcc aaagaatcct tgccctcctt cccaaatcca ttcctccttg     600
gtcaactgtt actttggccc cgacgtgtca aggaaagcgc gtcgcaaggt ggagcgctcg     660
ggttgacgat gagaaggtga gctgtgaacc ctgcacacgt cctcaaacaa aaggctcacg     720
agcggcggcc ccgacgtggg gagcaagcag gcttctctgg cccctgcagc aggcggaccc     780

aggcccgcct gccgccggac attcagaacc tgttcccgca gcgcgagctc tgcacacgct     840
ctgcgatcgg cacccctgga ccccctaacc acccgccgcc caaccccccgg agagagggct    900
ttccgcccgc tgagctgcgg ggttgggaag gaaaaggggg acgcgctcct gggggtcttt     960
ggatattctg gcaaaggggg ctgcagctct aggctccagc taaagggtct gggacggagt    1020
gggccagaaa gccgccaaac tgaggattcc cccctccccc aagccggagc cctaggtgcg    1080
gagccgcttg cccacttacc ctcggagcac gtcctgctgt cctcgccggc ggacgcggct    1140
ggccactgcg gcaccctcct cgcgatactc gggcgctaga aacccgcctt cagagcctgc    1200
tgtagcggac cgaacctgtt gcggcggcgg ctggggccgg ggcggcttgg gcggaggagg    1260
ctgaggctgg ccggccgtgc cctgcgccca gagcaccaca cagcccagcc acaggaagta    1320
gagctggaga cacagcctcc gtcttctccc catcgccggc gccgaaagcg cgcggccgta    1380
gacccgcgga gagggagtga tcaaagacaa aatctgcgcg cctcagaaaa gagtcagggt    1440
ctaataagcc cttcgtcggc tccggggact ccctcgggct cggctccct gctctagctg     1500
gagacctcga cagagcgccg gcccccctgac tgcccgcgaa gcgagacgcg gggcgccggg   1560
tctagcgcag tgagcggcga ggcgcggcgg aggtgcagcc ggcagccccg agcggtacac    1620
gttgcataac cggcctaaag ccccgagcga ctccaggacc gtcagcgggc gggggaggaa    1680
attacaaaag ccctggcgta aaatttcaaa aaatgtgaac ctcaaaagaa aaaagggcct    1740
gcacgcagag ctcggcggat tcccgtgcgc tccgaagacg gatattggaa agctgcaaaa    1800
gtcaccagaa agaaacaggc aagcaaacaa aagtcgcccc cagaagaaga ggagggttcc    1860
ctccgggctc gctcggagtc ccacagggca acgaagcgcg ggtagcggct gcggagccgg    1920
gcggaggtgc gcggggccgg ggcgtgcggc cagcaagaga gagggcggga ggctgggcgc    1980
tggagaagat gcggcggtgg agggcgcggc gcgggggttt tagaggcccg gcggggcgcg    2040
gggcgggccg gcgggcggcg gctgtcagtg gggcgcggc cgtccacacg tggggctcaa     2100
cccggactga gaggccggcg cagagtgggc ggggacagag taagggagag atcgggactg    2160
aggaacgagg ggaaccaggg ttgggtgagg ggaagaggaa ggaagggggg gagggcgaag    2220
ggagggaaga ggacggagat tggggagaag gggggcgaac ccgagaggaa ggccgggctg    2280
gaagagggac ttggaaagag ggattggcga gcccgggtgc gagaggaaat gagcggggga    2340
tgggggcgaa gttagggcaa agagagaagt gggaaaggag agggagggcc aaggtccggg    2400
cgccgagagg aggggcccgg ggggccggct ggagggggcg aggcgctagg cggctttgct    2460
tcccgtcccc gtcaattggc cgctagctct gttttgactg cctgtacaaa gcgagtagac    2520
ggcgggggat ggggcgatta gggaccggat ttgggaaagg aggcagcagc cccttcccct    2580
ccccctccgt cctcgcccgg ccgcggacca gggactaggg tgcctaagcg cgccaggctt    2640
ggccggaccc gcagctgaac tgacccgcag ctgaacttcc ccgccggaca gaggccgcaa    2700
ggttgaggat gagaacgggt ctttctgtag ggtcccggcc cctgacctgg gatgggggga    2760
```

```
ttcgcagtca gccaggaata gaggctcacg gggctgttgg ttgcagcctc aatccaagaa    2820
ggaggagacc tcccctttgc cctcctaccc ccaccgcccc accgggagcg gcgtcagtct    2880
gcgggaccaa attaggggct gggagtttcc agattgaaat gcgccctcca ccaccaagag    2940
ggagctcacg cggccgcggg gggccgaggc agccagccgg cgccgcggtc ctggggagtc    3000
cgtgcagggg tgtgctctgg tgtgaaacct ggcaggactc tgcctgggct tgcatgcagg    3060
gaggggttag agactctgaa atgtactagc gggcagggtg gtgacaaggc gggtaaatga    3120
ggctcggggc atgcccttgc ttc                                            3143
```

<210> 29
<211> 6676
<212> DNA
<213> Homo Sapiens

<400> 29

```
gaccaaacga aatatcgtaa cacaaggcga ggcaggcttg tgggaaccgg tccccagcgg      60
gcggcggcag agcggggagg attggagggg ccagggctgg gggtggcacg ggctcttggg     120
ccgctgggct cctctgggcg ggctcagggc tggaaggcgc tgccagctgt agccaggctc     180
atacttttca atttgttgtc cttcttccac ttcatgcgcc ggttctggaa ccagatcttg     240
atctggcgct cggacaggca gagtgcgtgg gcgatctcga tgcgccgtcg ccgggtcagg     300
tagcggttga agtggaactc cttttccagc tccagggtct ggtagcgggt atacgcggtc     360
cgggcccttt tcccgtccgg cccggtcata tctggagcag atagaggaaa gccgcaaggc     420
aacattattc cggttaaggg aggggggcact gggtgggagg gggcggggga gcaggaccca    480
ggcgtcccgg cacccagctc accacagctc caatcctctc tactccagat gcccacattc     540
aagctcgatt tctgcacccc tttctactgc atccccccact tctgcaggca gccagtgcgc    600
ccggctttgt ctcttgctcg ctcctagtgc ctcacccttg gctgggctca ggcggccgct     660
cgccccggga gggaggagag cggttgcact tttgccccgg aaagctcctt ttactgttca     720
ccccttcttt gctagcgaca gtctcaaaat ttcaggggta agcaggaccc ttccccacat     780
tatatgaacc ccatccttta tcctaaagct ggttcagctg cgctaataaa aacctaagcc     840
gaagctggaa acaaattatt aaaaataccc ccctcttaac agagagaccc tttttggctc     900
tgtctacgaa gctatgaggc ccctcttaga tacttctccc ccttagaaaa atgaatctat     960
atttagggta aagccaagct ctccttgtcc ccccgatccc accccaaaac gcagagaagg    1020
aaagccgaga agacaaaaaa gagagagaga attaccatgg ctgatgtgaa gcttcctcat    1080
ccaggggaat atttgcggag tctgcccctc gggcgcggct gtggaggtgg ccatgggctc    1140
tggctgcgcc cgagctaggc tggggctgct tagctggctt gccgcttcct caggctccga    1200
ggacgcgctg gcctcgtcta tttcggtgaa attggcgctg gagctggctg aggtcgcctg    1260
gtcggagggg gacgaagcag agggcttggc gccgtggctg tcgccgttgg tgcagggcag    1320
ggactcgggc gaggacaggg agcagctcga agccgcttgc ctgaagcggg gctcctgggc    1380
gggcgcgggg aaggcgcgcg agctctcgcc caccgcccca aagtggctgg aggaggccga    1440
ggagcggttg acgctgaggt ccatcccatt gtaattgtag ccgtaagagc cggtgtgcat    1500
ggcagcggga tccctgtaag agccgctcag agagctgcca ctgccataat ttagcaactg    1560
atagtccggg ccatttggat aacgccccga gaaggagttt acaaagtacg agctcatttg    1620
ggtgattttg gagggcttga tttgtggatc gtggtcgtta taaccctgtg cttcacgatt    1680
tatgatgtat taatgaatta tagcgatgca ctgtacttcg ttttgctatg tatgcggcca    1740
aatatggggg gggggttgga agggggggtgg ggggcgttag ggaatcacgt gcttttgttg   1800
accagtcgta aattctcgct gatgacctca agaggtaatt catgctctat ggttacaaat    1860
aatgacgatc cgagaatcgt tagggccgat tcaatgcgag cctccgagag agggggaaaa    1920
aaggagaagg gggagaaaca gagagaaggt tggctttggc ctctgagcag agcgcgccac    1980
ctcccggcga ccgacgggag cgcgggcgtg agaccgccat ggcggcgggc ggctgcctcc    2040
ctgctggctc cctgcaggct ccctgcagtc gccgggagag aaagaaaccc ggccaaggct    2100
cagccacagg gttgagcctt ttagagcagg ggttgggctt tttacccact ttcttgtggg    2160
tggggttagc ctctctattt ggttctttct ccctacatca tcattgtcgt tattatttta    2220
actttggatg agaagatcaa agatagagtt gcttttccct ttcttgggct ttgcaagtct    2280
ccgaattgga ggcggaaatg ggggaagggt tggaagttgt gtgtgaaaaa caattattgc    2340
aacgggtttt ccccaggagc ttagtccggc ctgcatcctg gtggaactgc tgttaccggt    2400
ccacagcgcc caggccgagc cggcagggct gggcgggagg cttgaaaaag aaggagaagc    2460

gttcacttct cctgggccac ttggaagttc gaaatcccct tccggacctc cttggtggtc    2520
cttagcccag gcgtggggag tgacagagca ggacagggac cccagaaacc gagcacagct    2580
ggggctaagc ggcctagttg ccgaaaagac tgcagcggcc accgcgtctg ctgcttagtt    2640
tgagtagtaa catgtgtctg agagtttgta aagcgggatc ccttcccctc ctcctcggct    2700
```

```
attgcattcg gaggccccac tacaggtccc tcactcgcag ctaggcagtt tgagaatttg 2760
cctggtggca gccgccttcc cggaaaaggg gttccaagga cccgcatgct ttgctgagca 2820
cgctgggcgg ccagagaccg ccgcagctgg tctcctatta ggaggggact attttcttta 2880
aaaataagaa aggaagcgag agggcggagg aataaaggaa aacagtgacc gcagcttgcc 2940
ttctgggtgc cggtgcaagc agagagagaa tgagccattc tgggttgaga ggatgacact 3000
aaaagcccca tcacctggcc tcctgccctg ggtctccaa ggagcaaggg aaagttgtgg 3060
ccttcggcct gtgcctggct ggtggcttta cattccccta cttgaggctg atctcctact 3120
tgaggctgat ctgctacttg aggtgggaag gggcacctgg agccttcagc tgcagggtga 3180
gagataccca gtggccccat ttttcagtct ccagagccac aggccaggag cggagggcca 3240
aggctggcga aaaaacctgg ccatgtggac gggccaaaga ccaggggtcg ctgcgaaggt 3300
gaggacagaa aagcgctgca gaggccccca gactatggct ccgtctccgc cagagagctt 3360
taggggcctc agtggctccc ttagtgctgt gtatacacga ggaaaggcta gagaatgaga 3420
gggacacagc acctctccat cccccaagtc tgcacgggga gaagcggctg tgagcctcag 3480
caccagggaa acgcagcgct ggggcctggg caggactgat cgctccagcc tttatctcag 3540
ctgtaacttc ccagtcgaac agggctcgct tcggcgggcc agggctctcg cctttgctga 3600
agaattctgg ctcaggaaag atggagaggc tgggggttga ggagagagga aaaaatggca 3660
ggggaggatt ggaggtgacc gagcgtcgag ttttcacatc tttattattg ttgttgttgt 3720
tgttattatt attattatta tcatcatcat catcatcatc atcatcgaag tatttcacgt 3780
ccagagctaa gacaagacta caaacacaac acatagaaaa ttaataaaat agaactttgt 3840
tttcttctga ggctcctctt cttacttcta ggtagtatgt gctccttcca gtggctttgg 3900
ggaggggggtg ggagagacga caggtctggg atcagggagt cttcaaggcc cccgctgagg 3960
ggacagcgaa tctaccattg aaccgtgcac gggggacatg gacaaaatga gacgcgacag 4020
ggacaagagc attttgctct gcttccagga aacatcaagt gagtccgctc ctcagttctc 4080
accaggaagc ctgatcaggc tgaggcgagt tcctcgggaa ggaagggcgc gcgggatgct 4140
gggtgggctc gagtagtgag gcctcagagc acccgccggg agctgtgcgg gcgggggcgt 4200
ccaggagagc gctgggcccc gcctgtctgc gccggagagc aggtctcctg gctccccccac 4260
ccgagagcct tccttcccgg gtctctctga cgccctcggc tccccacagg cctttcccct 4320
cgcgtcctcc ctccctttcc agcaccttca ctcggcctgt ttttcttcct cctcctcggc 4380
actgagctga gacgcgctga gcagtttgct ctccttttttc cacttcatgc gtcggttctg 4440
gaaccatatc ttgatctgcc tctccgtcag gcacagggcg tgcgcgatct cgatgcgccg 4500
ccgccgcgtc aggtagcgat tgtagtgaaa ctccttctcc agctccagcg tctggtaacg 4560
tgtgtatgtc tggcggcctc gccggccgct gggcccaaag gaggaacctg ttacgcagag 4620
tggagatgct gaggcctgcg gtcaccgggc ccaggacccc ctcccctagt cgaccctcga 4680
acacagactc cagccagtac cgggatgccc tctattctgc cggctccctt cccccgtttc 4740
gcactcctcc agcgccccct ccagattccc tcctagtctc ctaggcctgt gccgtctgtc 4800
tagactctag atgggggagg ggaggagcag tttgaactcc cacctgagcc tgggggggagg 4860
ggctggtcag gtgtgtctct tcctagttgc atcttgtctt tccctccctt tccatccatc 4920
ttgttcccac cccccgtcat ccccccaacc caatgataaa tccaggccgt taatccgtaa 4980
tgacgtagat cgatccatag tccacattaa cggctcctca ctttcgagtc cggctaatgg 5040
acatcagttg ggacttaagg ccaacaaata atccaacctg agaccccgcg cctgtttctc 5100
cctctctcgc tccgcctgct ccctctctct tccttcccct tattcctcct ccctcctctg 5160
actaccaccc ctttcctggt gcctcatctt gccccagctc cccacccaca gggaaacaca 5220
gtcccagaca gactcaactc tttctcttca gcgtcacccc ctaccccctt gcatgacgca 5280
ctccgttttt aatggagccg tctttggttg gggaacccta ccagggctgg gagaggggggg 5340
ttgggggctc aaagtaaagc caagccagtg actgcaggct cctgctccct tccctgtcct 5400
ggaaccccgg ctctggccag gcgccccagg agagcacact tgctccccat ggctcccagt 5460
gcggctggga ctttgcaaac tttgtaactc ctgcctaata ggggacacag cgagagaccc 5520
ccggccggcg cccaatcttc ttctatctcc taactgagat gcccacccag caccctgtgg 5580
gagattgggg caggggggcgc tcactagttc tgtgtcccgg ggtgggcgcc caggggagcc 5640
ggggcgacgg cgacgggaag tctcactcac tgttgcacga attcatccgc tgcatccacg 5700
ggtagaccgg agtggagcac ttctgctctt ctgtctcgcc gaacacgctc ttgtcctgcg 5760
cgcagtccga cttccgcggc tcggggtgga acgggggctg ctcgtcggcg ccggagagtg 5820
cgcaggccga ctctttctcg cggtagaagg ccggcgccgg cccgtagtcg cagggcgccg 5880
ctcggccgta gccaccgccc gccggcgggt aataggagga agtggcaaag cccttgtcct 5940
ggcccggccc tggcccgtag ggcgcggggt aatgtctcag cgggtccgca tagcccgacg 6000
aatagagcgg tagctggccc aggaaggact cctgcccgct ggccagagtg acggggaagg 6060
tggagttcac gaaataggaa ctcattggga ggggaggcgc gcgcggccgc tgctgctccg 6120
ccgggtttat gatttgttgt gttttatagt ccgagcgccg cgcctattag tagtatatcc 6180
gagattgggt tttagctgag gggggatggc gaggtgccag tgagggccag aaggaaatac 6240
aaccatctga tccaacactg accaatggca gaggcaggaa ttgtcaaata gcacccagga 6300
ggagcgcagc ccgcacgagg gacccgcgca caaacctatc aacctttttt tttttcctcc 6360
```

```
ttctccttcg cctttctcct ctctcccccc aacaacacaa cgcgtcctgt tccacctacg    6420
taggatctgt gaaacaggct cagtgccttt gagggaggag ggaacgttta gattgagacc    6480
accccactcc cgggtgatta aataaatatg tctctccccc accccacgca ggattggaaa    6540
aagttaattg tgttttcaga aaagttaacc cctgcgttgc ttcgacccct tctctccggg    6600
gagggttggg taaatgtctc cgaatcgcct tgagagagaa ctaaagaagg tttgtttcta    6660
gggttttgtg tcggta                                                    6676
```

<210> 30
<211> 11656
<212> DNA
<213> Homo Sapiens

<400> 30

```
gtcaccgcct ggcaggggct cgctcctttc tctggtgctg ctcattcgcg aggcctgaga      60
cgcggaccgt caccttcccc gggcccagcc tggcctcaat gcaggcctca gagagaggga     120
caaaacccgg cccgcctccc tcccctcccc aaggcgacca ccagtgtcct gggggtccac     180
agggcctctc cagctctgat ccgagttccc ggctgcagct ggtttgggtc tgtgggggcc     240
tcgcgcaggg aagcggaggc cccttctcta ggctcctaca tcctcctggc ctgccccagc     300
agagctagcg gccctccgag acagctgggc ctcgcagctc ccagccgagg cctgtcgctg     360
ggagaaagaa gaatgaatga agggcggaag gggcggggag gggcggggcg cgccgcgcca     420
gctcgcggcg cccccaggcc gggccgcagc ggggcgtggc ctctagaggc gtggccttgg     480
gcgtgggggc ggggttttct ggtgggggcg gggccgcgca gggccgttcc cgccgccggc     540
cgcccgcgcc ccgccccccgt gccgcagctc acaggccccg cctcggtccg cccctccgct     600
cgctccccaa gccgccgcgg cgccgagccg gtttccccgc cggtgtccga gaggcgcccc     660
cggcccggcc cggcccggcc cgcgccctcc gcccccgcct ccccgggccg gcggcggtgg     720
gcgagctcgc gggcccggcc gcccccagcc ccagccccgc cgggccccgc cccccgtcga     780
gtgcatgagg ttgacgctac tttgttgcac ctggagggaa gaacgtatgg gagaggaagg     840
tgcgcgggcc gcggggtgtg gggcgagggc ctggaggggg tgcccggggc agcgtggggc     900
acgggagggg gccgggtctg ccaggaggcc gcgcccctgc ctcctccggg atgagctcgt     960
ccttacgaag cccgcaggcc cctccctgtc cccctcccgc ccgggatccc cctccccggc    1020
ccccggcgag ctgccctcct gcgggtctgg gggcccctgg accctttttc ctcctcccac    1080
gtccccccgc gaaggactcc cagacactgc ccaccccgcg tcggcctcca tccgcgtgct    1140
ctgtccacca cccgggcctc gccctggggc caccctttat ccagtctcgg aagaaagagc    1200
ggctggggaa gccgcagccc cgggtcccag tggccgccgg gcgcctgccc ggctctgtga    1260
ccttgagcca ggcgctgact tcctggtcct cagtttcccc ttctgtacat ttggaaactg    1320
ggtagttgcc cccccggtgt cggtgattgg gggccagatg ggtagagcgg agataggcgt    1380
ccaggaagcc ggaggccgtg tactgcggga gcctcatcca ctctccctgt ccgtgcccca    1440
aacccggtgc ctgccctcag tcttggctgg gagcatgact catcctaacc tcctctttag    1500
ccccttctcc ctcactgggg cccaaggcgc agtactgcac tgcagttagg gttcaaggac    1560
tcccccagcc taggacaggg tctgggggcc cctccttgga tctccttcgc tgacctgtca    1620
cttagatcca cctggcccca aggcagggcc tgactccaca cctccccctg ccaccaactc    1680
ttcccaggcc catgaaaacc tgattggggt aggggcccac cttcctgtag cccctgccta    1740
cctaaggtac ctgcgtcttc acagagggtc aggctgttgt ggccttggga cctagctatg    1800
tgactgggca agccatgcca tctctggggc tcagtctccc cttctgtaca gtggagaggg    1860
gcaggtctgg ggcattttcc agggcccacc agctccaagg gtgccaggcc ccaaggatga    1920
ctaagcatgc ctgtggctgg ctagaggagg tgccaggcct ccctgggaca ggtgtctggg    1980
agtacccagg tctgcagccc cctccccttg ccaagccagg gcattcattg ccaaggatct    2040
gttagggccg gcacctccag gcttcctgcc cttgacctcc cagctggctt cagcccagga    2100
tgcactaatc cagccctgtc cagtccctgc ctttgaaggg ccctcttagt acttcttcct    2160
gggcaggaga gggaagaaag gaggctgtga taggaatgtc acccactgcc ttatccctaa    2220
agccactgct tcctttctcc tcatttacct tgccagatcc aatgctatag cgggaggatg    2280
gacctgatcc tcctcctaag ctgatacata gggaaacagg gccagagaag cttggcaacc    2340
tagtcagtat ctcagcaaga ctcaggccag cgccctttct tctcctattt ggcacagcga    2400
ctgccctgcc tgggcgctgc acatgtgcag tgtgcgagga ttggtgcagg tgtaggtata    2460
tgtggggtgg gcagggcaag ctgggcctgc accagatcac acttcctgag aatgcttccc    2520
aactcccttc ccaccctgca ggaagcgagt tgcccgtgtg tgcaagctgc ggccagagga    2580
tctatgatgg ccagtacctc caggccctga acgcggactg gcacgcagac tgcttcaggt    2640
agggtggggt gcccagggcc tgtgttgccc taaacaaggc ctgccagaga ggacaggctg    2700
gtcaaggaat gggggaggcc gggatatgcc tcctggtgcc gtcccctatt gtgacttcgt    2760
ggccttaatt taccatttat gacatgaggt gttttgacta gaaaatccct acaggccttc    2820
```

```
ctgttgtcat tttatttatc tatttttttt tctttttgag acggagtctc gctctgtcac    2880
ccaggctgga gtacagtggt gcgatcttgg ctcattgcaa cctcttcctc ctgggctcac    2940
gcagttctcc tgtgtcagcc tctggagtag ctgggattac aggcgtgcac caccacgccc    3000
agctaatttt tgtattttta gtagagacgg gttttgccat gttagccagg ctggtctgaa    3060
acttctgacc tcaagtgatc ttcccacctc agcctcccaa agtgctggga tgacagacat    3120
aaaccaccgc tcctggcctc attttatttt cttttatgta tttttctttt ttcgaaatgg    3180
tcttgctctg ttgcccaggc tggagtgcag tggtgccatc tcggctcatt gcaacctcca    3240
tctcccgggc taaagtgatc ctcctacttc agcctcccga gtagctggga ttataggtat    3300
acaccacaat gctcagctaa ttttttaaat tttgtgtaaa gacagggtct cactattgag    3360
acccaggctg gtcttgaact tgtgacctca agcaatcctc ctgccttggc ctccgaaagt    3420
gctaggctta caggcgtgag ctaacgcctt ggcctctgtt gtcatcctag atctctgaga    3480
tctaaatctt agagaggatg ggagagacct ccaattgagc cagtgcctgc aattcagccc    3540
cctgctggca cccagacagg gggaagagtt ggaaggaatg tccctcctgc cttctgggtg    3600
ttcatgctct tgcagggagg gaagacaaac caggccttaa gggaaaccag gccaccctca    3660
gtgtcttcca ggctgcttgc gaacatgcat aacccagtca caccagcccc agtgtccaga    3720
cacacaccca caggtaggaa gaaagtaggg tcagggttgt ggcggaggat aaagagtaca    3780
tgaggacctg aaggtcaccc agtaggacca tcctgagaag ccaggagcag gggtctacct    3840
gccttgagcc agagcagggc cagagcaggg gtctcaaagg atgtgagatt tcctgggtag    3900
aaaagtagag tggaggtggg gcgtggtggc tcacacctat aatcccatca ctttttgggg    3960
ctgaggtggg cagatcactt gagttcagga gttcgagaca agcctgggca atatggcaac    4020
accctgtctc cactgaaaat acaaaaaatt agccgggcgt ggtggcgcat gcctgtagtc    4080
ccagctactc aagaggctga ggtggcaggg ttacttgagc ctgggaggtg gaggctgcag    4140
tgagctatga tcgcaccact gcactctagc ctgggcaata gagcgagacc cagtctcaat    4200
ttttaaaaaa gaaagaaaga aaaacaaatg gtgtgggaga gaattacagg catagtcacc    4260
aaacagcaag gttcagggga gaaaactcca taaaagggta gaaggtgaag cttctgggat    4320
gcccagcagg ggtcaagaca tccaccacta ggactttatt ttaggcttct gccttggttt    4380
attttttggt ttttggtttt tttgagacag tcttgttgtg tcgcccaggc tggggagcag    4440
tggcgcgatc cctcctcact gcaacctccg cctcccaggt tcaagcgatt ctcctgcttc    4500
agcctcccaa gtagctggga ttacaggtgt gcaccaccac gcccggctaa gttttgtatt    4560
ttcagtagag atagggtttt gccatgttgg ccaggctggt ctcgaactcc tgacctcaag    4620
tgatctgccc gcctcagcct cccaaaatgc tggaattaca ggcatgagcc actgcacctg    4680
gcctcggttt gttttttttgt ttcttctttt cttttttttt acacagggtc ttgctgtgtc    4740
acccaggctg gcgtgcagtg gtgagatcat agcccactgt agcctccagc tccaactggt    4800
tcaagcgatc cttctgactc agcctcccaa agtgctggga ttacaagcat aagccaccat    4860
gcccagcctg ttttttcttt tttaggaata acgtctaacg ttttctaaca ttcagtaagg    4920
gacaacccct gttctaagta ctttgcatag ttagatatta gtgctgtctt tgttttgcca    4980
gagagaaaat tgggacacag agaggttaat tctcttgatg aaagtcacac agccagtgag    5040
tgaaatgaac acactcagtg tggctgaaag gagacagaca gcatgccctg ggattctgca    5100
tcaggtgctc agaaagaggc cttcgggggg caagagggct ctcaacaggc agaggaaacc    5160
atctgcacag cggtgggatg gtgcggactg ctgagggaac aggaacagtt cccttggaag    5220
gaacagaata agctgaggga tccaacaaga aacaaagttg agaccgattc gtgaagggcc    5280
ttgaatgcca agataaggag tttcagaagt caggatgggg gtggtggctc atccccgtaa    5340
tcccagcact ttgggaggcc gaggcaggca gatcacttga ccccaggagc ttgagaccag    5400
cctggccaac gtggtgaaac cccgtctct actaaatatt caaaaattag ccaggcatgg    5460
tggcacatga ctgtaatcca agctactcgg gaggctaagg aaggagaatc acttgaacct    5520
gggaggcgga ggctgcagtg agctgagatc acgtcactgc actccagcct gggagacaga    5580
gcgagactcc atctcaaaaa aaaaaaaaaa aaaaaataga agggagtcgg cagaaagcca    5640
gggaggggct ggggtgacat gctgttgaag aatgccatcc cagtgggccg gtggtggtat    5700
ctaggcaggg aagggactgt cccagtaact caagggtctg agctcatagg acctgacctg    5760
ggacagtgac tgaggatgga gagaatttca ggcagaaggg acagtttttg gtgagtattt    5820
gtcatattgg ctaccatgca ttgagcactc ttcatgctaa tttgttaaat cttcatcata    5880
actctatgag ggactgtatg tgcccagttt gcatgggaga aacagagatt ccatgcaatc    5940
aagtgcctcg ctgaaggttg taacatctag agctgggact aaaaccttct cactccacat    6000
cgccacagag taggaaaggc aggggctggc ggtggcacat gcctataatc ccagcccttt    6060
gggaggcgga ggcaggtgga tctcttgagc ccaggagttt gagaccagtg tgggcaacat    6120
agtgaaacct tgtctctaca aaaaaattag ctgagcatgg tggtggtgcc tgtagtccca    6180
gctactcaag ggcgctgaca tgggagggtt gcttgagcct gggaggtgga ggttgcagtg    6240
agctatgatc acaccactgc aagccagcct gggtgacaga gtgaaatccc atctcaaaaa    6300
aaagaaagaa aggaagaaag aaaaagacag gggcttcggg gagggcatgg gcactggcga    6360
atggcagggt ggaacctgaa gccatctggt tttctaacct gggcactggg gagttggtgg    6420
tttgttgact ctgatggaat tgggggtcat gttggggagg agacatgctc atctgtgttg    6480
```

```
agctggaggg gacatgggct atccatggtg gctgtgtcct gcccagagct agccatggga    6540
gcctgagtcc agttggaggt aggaaagtca gaaaaaacgg ccgcctcgga gctggccctg    6600
agatggtgag tgggatttgt gatagggcca agacgaatga agggaagaac tttggggacc    6660
cctgtgtctg cggtgagggg ggagatggag ccttgggtga tggagagagg gtcaggagta    6720
gagccacaga agccacagga gggaagccgt gttacaggat gggtgtacct ggctttggag    6780
tggcctgtcc caaatcactc accaggagag gggtgagtcc ccaggtcagg gcagtaaaga    6840
ggaggcatgt ttgtgctgtc cctggtgtag tgaaactcaa gaaggaagcc aggtgcagtg    6900
gctcacgcct gtaatcccag cactttggga ggccaaggca ggcagatcac ctgaggtcgg    6960
gagtttgaga ccagtctggc caacatggtg aaaccccatc tctactaaaa atacaaaaat    7020
tagccgggcc tgttggtggg cgcctgtaat cccagctact caggaggctg aggcagaaga    7080
atcgcttgaa cccgagaggc agtgattgca gtgagtcaag atcgcgccac tgcactctag    7140
cctgggtgac agagcaagac tccatctcga aaaaaaaaag tctcaatatg gggaaagatc    7200
cactagaagt aagagccatg gcttctacct cgtggcttgt gggtgtgata ctcccaacag    7260
tccccaaagc tggtggtcct caccgcgtga cagtgagcag agcagctcag agggggtcac    7320
tgctcacctg ggtgcatggc tgaccacagc caggctggct ctcagtggga tgcccaaggt    7380
gctagactct gcttagtctc cctcgggccc tgggcttgag gcattgggcc cggcccagac    7440
ctcatttcat gcactgagac ctttgttcca gggcccctca cccctctgaa ggtgttcggg    7500
caggggcaat gtgataaggc catgaggggt ctgcagcctc cagcccccact ggggaggtgg    7560
ccagtgattt ccactttcct ggcccctctg catgcccctc ccagtggaac ttcctagggt    7620
ccctgagtca gtcacttgca aataattatg gcgtgcccac tctgcattag gcccctctca    7680
caacaaccca gtaagggggt gctatttatt tattaaagcg attttttttt tgagtctcgc    7740
tctgtcgccc aggctggagt gcggtggcgc aatctcggct tactgcaagc tctgcctccc    7800
gggttcacac cattctcctg cctcagcctc ccaagtagct gggactacag gcgcccacca    7860
ccacacccgg ctaatttttt tttgtttgtt tgtattttta gtacagacga ggtttcgctg    7920
tgtgagccag gatggtctcg atctcctgac ctcgtgatcc gcccacctca gcctcccaaa    7980
gtgctgggat tacaggcgtg agccaccgtg cccggcaata ttaaagcgat tttaaggcca    8040
aggctggtaa ctcacgcctg taatcccagc actttgggag gctgaggcag gaggactgct    8100
tgaggccagg agtttgagat caacctaggc aacatagtga gactccatct ctacaaaaaa    8160
attagccagg cgtggtggtg cgtacctgta gtcccagcta ctcaggaggc tgagatggga    8220
ggatcatttg aacccaggat gtcgaagctg cagtgagctg tgatcacgcc actgcactct    8280
ggcctgggca acagagcgag acactgtctc aaatttttaa aaagcgattt tacaaatgag    8340
gtgcagagtt cagtcacttg ccaaaagtct cacagcgcgt gaggagtaga atcaggactc    8400
gaaccgaggc agcctggctt cagagcctac agtgtaacca cagcttagtc ccacacctcc    8460
cagaccaaca gggtccctgc cttctagtgg gcaagacact cagtgaacaa atgtagtgtc    8520
aggtattggg ggacagcact ctcaggaagt gatgtttaag ggacagaatt gaagggagca    8580
gtgtttagag gatgtcgggg gtagggccgg tgcatgtgca aaggccttgg ggtgggaatg    8640
tgcttggcac aactgaggac cacaaagcca gcgtgcggga gtgcagtcag tggccagggg    8700
tgcatagagc cttgtgggcc ccgtggaagg tgccgttggc tgtacagctt tttttttttt    8760
tttttttttt ttttttgag acagagtctc gcttttgttg cccaggctgg agtgcagtgg    8820
cgtgatctca gctcactgca acctccgcct cccgggttca agcgattctc ctgcctcagc    8880
ttcctggtag ctgggactac aggcgcccac caccacacct ggctaatttt tgtgttttta    8940
atagagacgg ggtttcacca tgttagccag gctggcctca aactcctgac ctcaagcgat    9000
ctgtctacct cagcctccca aagtgctggg gttacaggca tgagccactg cgcacaggca    9060
gctgtgcatc tttgaatgtc ataacctgag catctgagag ctgctcctgt cccctggccc    9120
ctgctcttga ggaagtccca cgctgatagg acagacaggg tcataagtgc tgtgatgggg    9180
gcctgcaggc tgctggaggg ctcagccggg accagatgct gcccctcttt gtagagtggg    9240
acaattgctg caggcccatg ggacctctgg tattagccct gagggttgtc actccggggc    9300
ctgccccttt ctgtgttctg acctcccagc cccttgcagg ccctgcctcc cggaaggtta    9360
tgaccaggct tggactggtc caggcttccc tttggctcac atactgcctc tgcgaggtcc    9420
cctccaggaa gcctcctgtg cacaaccccc agggctgccg catccctggt agcatctcct    9480
tggcagctgg gtgggctggc cctgggcaag gagggctgag catgctgctg gcctgtgggg    9540
ttggagcagc ggcgggatgc aacctccctt tcttcagggg accttttgg cgaagacaaa    9600
ctgtccatag gaagtcgacc tctgttccct tgggggcagc agtggaagag cgagctgctt    9660
ttgagcttgt ccctgtcccc agagaagcct gaggccttca gtgccgttgc cagggccgag    9720
gctgaggagc ctacagcgtg tgttcaggac tgagggccag ggacgggcca caggctccct    9780
gcctggggtc caagcctaga tcgctcgctc cccacccgca ccaaagccca ggcaaagggt    9840
gcttcagcca cttcctgttg caggctcaga ccaagtcccc tggcacccac gcggctgcag    9900
cctcctcctg tgcgctgcag ccacgctggc cccaccctct gcagcctcca atcctgagcc    9960
cctgagggag gatggggaag cagctggtct ggccacccct gccctccctt agacctccag    10020

agcccccagt gtagccacag aggatgctgt tggcttcagc cccaagaaga cgccgcttcc    10080
```

```
tccagagggc taagtaagtg ggaatccccc tccctacttg tcctgggctc caggcagggc   10140
ccctggtgta aggcctgggg ctggaagccg acccacctag gtccaggctc tggggcagaa   10200
ctgaaactcc ttggttactg tcggctgcag cctgggagca ggccactgcc aaagctgtgg   10260
gtccttccag gacagtctcc ccatgaggcc ggtcctccac ctgctgtttc ttcacacctg   10320
gtggccaggg atgtggccct gggtagaacg atgattctcc actcctgtca ttatggaagc   10380
caccgctgtc tcccagccca gccagccacc tgggctgcag agcacccctt tcatgccctc   10440
cgggtgcctc ccccttctcc tgccccagcc tggctttgtc ctaccctgct ctcagggagg   10500
ggtaccctgg agtggggcca gggcatggct ctcccccgag ggagttcctc tctggctgtc   10560
cccagggcag ctctgcacag cctcagtacc tggcgcacct ccccttgacat ccttcttagg   10620
gacagtcagg cactctgtgt ggggcactca agagagccag gcccgtcagc ctctagctcc   10680
tgccagaatg caggcctgag ggtgaggggg cggggcaggg gcagggacag gaactccggc   10740
gtgctctcca tccgcaaagg ttcactgagg ccccgagccc cagccactga gccaccaagt   10800
cagcctgggc caggcctggg tgccctgtct gcaatggagg cagagacggg gtctcggggc   10860
agttctgagg atgctgggtg cacagcgggg gcctcgccgg caggaatcac ttatgctctc   10920
tcctgggcca agctttgtgg atgcccagcc tggggccgcg gggagctggc aggtcagtgg   10980
cagacactgg tgggcagacc tagtgtctgg tagaacaggc atcaaggaag tggtgaccgg   11040
agggaagcca agtgcactca aaccctcggg tgagtcatca ccgccgggtc tttcacagct   11100
gctgaaagtg agcaacagtg atgaaggttt gtgagtttct gcgtgagcga gtgaatggac   11160
cagtagcagt ttccaggttg tggaagagcg ttccctcccc gggatgggga cacttggtta   11220
cagcaattcc taatccccca cccacccacc gcccactgca gaggtatgcg ggggccctgc   11280
ttcctgcagg caggagtgag gggcactcct gtgatgtggc acccctgtga ccgaggtcat   11340
gtgtgatcgg tgtaagggca ggaagcgagt cattggtctg caccaggcgt gggggcttct   11400
gcgagggcag gacccaaagt cggcctggcc tcccggctgc agcactcctt tccctttcga   11460
attaggttag agccctggga cgggaggtgc cctgtagacc accccccctca ccaacttccg   11520
tcctccgccc cacccccgcg gtgatccggt gaactgccgg ccccctgctg tgcaccgagt   11580
ggggcagtga ccctgacgtg gcgtctcctg ccgcccctgc caccgccacc acctccggtg   11640
gcccagcctc cgcatt                                                   11656
```

<210> 31
<211> 13705
<212> DNA
<213> Homo Sapiens

<400> 31

```
gacgggaaag ggagggcgag ggctgggggc ggggaccgca tgaaatggag gcgcccgatc      60
gcgaaggggg ccctccccgc gcggccacgg ggaggagcct ggggaggcca gcgtgggtgg     120
cagctccagg gaggctgaga gcaccgctag gcctggggct gtgacgctgg gcggacccgg     180
cagggtctgg aactcccttc tggctggtct gggttggggt ggtgggaggg gagaggtcgg     240
accgcttccc tgcagccctt ttccactgag ccaagagggg cctcgaggga ggacggaggc     300
agggaaggaa cagccctggc ctcatccagc tcacccgcag ataccccacc catatataga     360
tctgaagtca cgtaacacac ctcgattctg acatacaccc caacaaacca gacgcaccag     420
ttcacggtca ccccaactcg cagacactac ccattcagat ggacacttca aaacttagaa     480
tctttagctc acaaagatac accccctact cagaggcata tggcatacct cagacacggc     540
caattcgaag atgtaaaccc caccacacat aaacatccag cctacacccc aactcaaaca     600
cacactctga atcgtaagcc ctaaagcaca aacacaccaa gccatacaaa cccttgattc     660
acgacgcccg ggcacacaga tgcaactcaa ctcaagcaac accccagctc accatggacg     720
ttccctctca gatatacccc aaactcagaa gcccagcacc ccaagctcac agaaacgccc     780
taggacaatc ctgtgctgtt acatgtatgt agtggagcag agacaccttt gtcctagcct     840
gggggtttct tcaagtgagt ccaggcctct ctagataaac taccaccaga tcagggagag     900
gagtgaaccc actggatgag ccactccctt ctcctaccgg caccagcccc tacgtatctg     960
agccctcggt tgcgggccat ggagctggcg cctgggaacg ggacctgctc acagggagcc    1020
agaaagcagt ttctagctcc agctttgcgc taactcgctg ccagaccttg gaaaagctta    1080
gtccctctca ggcccctctc cctccgggtt cctgcagcgc agcagcaccg ggaaggtctt    1140
tttggctgtc cacgcggcgg gggagcagtg tggagtgtac cccatccaa tccctgtccc     1200
gtgcagggac ccccgcaact cccgccttcc accccagcgc acactggagg aggggaactg    1260
aaggcaaacg caggggccgg ggccgcctgc tcgcgttttc cagagccggg tcccctcccc    1320
cgccgtgcgc agtcgcgacc gcacgcatgt gcgcatgttc cgggagagcc tgcgcgtggg    1380
gcctggggag ggggcggtcc ccaggctgag tcacgagagc ccgggcttcc gtggcaggag    1440
ccgggggtcg gcaagcccca gccctgccct ccctccgact ctggactccg tcgccgactg    1500
ggtcctccat ccttcccctc cagcgacttc taccctgccc cgccccccac cttgagcagc    1560
```

```
tccctaggga agtcgccgcc atcattgagg ccctccaggt tcccgatgaa gtccccgcag    1620
gtcatgcgct tcccgatgtt ctaaggaagg gaacggaggc ctgtgagttt ggagggccct    1680
tggcctccca ggtcctgcat gtctggccag gggtttgttt tccggctcct gcccgcccgc    1740
ctccactcac atggccgtgg agatccgtgt tgagcagcat gagcgcacag gtcagcgtgt    1800
gggcgccgtc taggggagag ctgactttca gccttggctg cctcccaggg acccttccca    1860
cagccatctt ccccaacctc ccatttcatc cccacccccct ttggccggca acttcctacc   1920
tcctgaaccc cgctagtaaa ccctccccac aaagagctcc tcttccagca gatccgtcag    1980
caaggacccg tccctggctc cctcaagtac tgagggctag ttgccccaac gtactagttg    2040
ccccaacgtt cccttgcctc taagcctgga ccacgagaaa gtgaaccgca gcacagtcct    2100
gtagaccagt gcagccaaac agaatcccct ctccagccaa gcactggcct gcacaggcat    2160
gcaaactcct gcctcttcac aacacactca accctagcaa tatgcctgcc cacatgtata    2220
gttccacata catgcagcca tgacagcgct gtctgcacac atgcaagttc acatgcctac    2280
acgcatgtac atccattccc tgctgtacac acactcatgc tcctgtacac acacacaggg    2340
cccagcccat tttgccactg tggccagcta aggacacccc tgctctcgga actcctcacc    2400
ctctgaggac agggcttcag gattgcactg gaagtatcgc tgggagaagt gggccagcac    2460
gcgctctcgt tcctgggtct cacccattaa ggccagctcc ttcagaaaca ccctggagaa    2520
ggggcaagag agggttgcct agcaaccagg agtttccagc ctctgtctct ggggtgacag    2580
gaggctttga gagtacagca gctggggtgg gaaggggagg agagaaaggg gatgagggta    2640
tgcaccctga ccctccaccc atcacccccta cccaaggtcc ctttaccagg cagctctggc   2700
ccttggccat gcccagccat cttgggaagg gtgatgtcag gacggcttgt gggggacaga    2760
ggggctcaaa ggaggcatcc tggggcccta acacgaaccc cggagagtgg gggctgtcct    2820
ccaaggggtc aacacccacc tgagagcttg gtccagagtc atgcccgtga agacaaagaa    2880
cttgaggtac tccccagcca ccagtttgct gaagtcattg ctgtgggcag ggcagagaga    2940
caggtagggt ctcaagggga agtgcagggg gtcaggtggg gattacccaa tgaccagggg    3000
acattagatg gagagctgcc ttcaggatgc attccctctg cccccatcct cagtcccagc    3060
ctagtggctt tacttcttgc ccaggtgccg ggccacatcg gccttcctga agccatctag    3120
tcggtacagc ctcttggcca ggcgctgcgc agcctccagg tccgctttct gcccattgga    3180
caaggtgtct gtgcttccca gggccagccg ctctgtgctg tccagctctg agtctgagtc    3240
ggacaccagc tggctcaggg gtggttcgct gccggggtag aacaccagct caggctgggg    3300
cagggtcctt aggagccatc cagaccctcc ttctgcctcc aggcccagaa gatggagaag    3360
gggagggagt ggagggacat ggggaaagca ttcttctttt ttccagggaa ggagattaga    3420
ttctctccat cagcctactc catcattacc acctctctgt ggaagtcctg cctcaaatct    3480
aacccacatc cgtcttgctg ctgtgggagt tctggctgct tcctcaagct cccaggggcc    3540
tacatttgct attagacata gattatctgt tcccaccctg gccctacaca gaacatagtc    3600
ctttgtctct tagactatgg acaaagctgg aatagatggg ctaccacgga ggaactgtgg    3660
agggccaggg aaagatttga gggcagcagg gatactggtc cgaaggcttt ccccctcctg    3720
ctttttaggg agaggaacct ctccttggtg ctccccctct gcccacacag aagcaggctc    3780
tggagttagg gggttaaggg tcggggtgca taccctcatt ccctttcttt cctccccttc    3840
ccaccccagc tgctctactc gcaaagccac ctgtcagccc agagacagag gccggaaact    3900
cctggttgct aggcaacccg gtctccctgg ccaccccctc ctgttgccat ggcttccatg    3960
actgagaggg tagctggcag aggggagagc agaaaggtta gactgtgaaa ggacttcctg    4020
gagctggacg agagatgcct gattaggcag gggtgacatg ggagggaggc agtgggagag    4080
gctggtggct gcatctttgg tgaagccttc aggctgggtg agggtaaggt cacaagaatt    4140
ctgagcaggc taaagactct gccaagggaa gaaagggccc tccaggagag caagaagggt    4200
attctcctgg gatccttcaa tgctgaatca tgctttctca agccactgga cttgagacca    4260
agaggggtgc tgtgagggac tctccctcac agctaatggc agtagtcctc caaataggaa    4320
aacaggatct gcatccctta accaccccta ctcaggggtc aggaatataa accaatcctc    4380
acactcagga tctgtccctt acagcagctg tttctggagc cctgggggcc tcaagtctga    4440
gggacaaaag gaattactta cctgccaagg ggtgctgccc ccagcccaag gctgtcctct    4500
gagtggcagg gactaggggg ctccctccct ggggccagct tggctctggc ctctgcctcc    4560
tcctcctccc ctctctgtgt ccaaggacca tcagcagcag agctggtacc agaatccggt    4620
tcaagagggg caagtggggc gggagctggt gggtcgggcc ggggtgcagg gggttgggga    4680
ggcagctcaa aggtgaagaa ggggctctgg gttgggccag gtgaggccaa ggcctccagc    4740
gaggcgaggc tggtatagga ggtgcctttg gcccggtgtg actccaggat ggcttcgaac    4800
acacaactga aagagtcagg cccatcagcc gaggggctcg tcccaggtag aaagggcaca    4860
ggtgacttga gaggcatccg gctccctggc ctgcaggggc agggagaatc tctgattagg    4920
ggcccagatc acagggctgg ggcaggattt cactgaactc tcacactgac tctgctaagg    4980
ggattatcat cccccttttca ctggctccag cccactaaca aaaaagagcc tctagggtag   5040
ggcgccaagc tattcagtgc cccctggggc acaccagccc taccagtccc cagcacccca    5100
tcccctccct ccctgctaat ctccctttct cctccttttc cgtgatccgc ttcccttctg    5160
ccatcccatt tctatttcta gcaacatctt aggagaagat gaggctccaa gcgacactgg    5220
```

ggtgtgagac ggaaaggaat gtccccacac tccccactct cccaccagga tccaggatcc 5280
agactcatgc tcctgtgttc ctgggcttga ctgtggcctc tggcctctca ccctgccccc 5340
tgttctgcta gaacccctgg cctctttgct ccccacatcc ccagtgttct gccaagcctc 5400
aggcctctgt ctagaatctc ctgctataca cactgccccg ccagccccac acccatctct 5460
atcccaggcc ctttgagccc ggctctgcct gtgggccagt gcgaaggagc aagccgcttc 5520
cctctcccac ttccccctcc cacttcccca ctcaccgggc cccttcagag gcctcaaaca 5580
cctcgtcgtc cacatcttct tccccacctg cctcatcgtc gtcctcatcc tcattgccac 5640
tgccgaggct gggatgaggg ccagggagtg ggccgggccg tggggcaggt gggtaggcag 5700
tgcctggtgg gccctcagag ctgggctgac tttcgatggc cgagtcggcc tcctcccgct 5760
cagccagcac ctcatcgatg tcagtctcgc ggtagcacct caggggcacc gtgtccaggt 5820
ctgtctcgga gtacttggct gctcgcccca cagccacccc agagccctgc cttgagccca 5880
ccccaggtgg agatgggggg gcctgctctg gggcttagc acctgtagtg tgcataggca 5940
tccaggtcag gaggttatcc acgtcagctc cctgtcctaa ttctgctcta ggatctctgc 6000
ccactgcctc atattgacag gaagttcttt cactgtctag ccaccattcc tcctgctaca 6060
tacttagctt gttcctggtg ccagtggagc tcattttaaa tcaagttctt gtcaaatggc 6120
tttgaggagc tgggggaaga ccagcctttt tctgtctttt ttatttattt atttatttat 6180
tttgagacag ggtctcactg tgtcactgag gctgaagtgc agtggtgcaa tcataactca 6240
ctgcagcctt gactgcctgg actcaaggga tcctcccacc caacctcccc agtagctagg 6300
actataggtg cacaccacta catccagcta attaaatttt tggtagagac ttgcttgttg 6360
tccaggctgg tcttggactc ctggctttga gcagtcctcc tgccttggcc tcccaaaata 6420
ctggggccac tgagccctgc tgagcctctt ttctactcca tggaatgccc ctcaatatcc 6480
ttctgtcccc tccctccatt actcagcttc ctccaggtgc cctgcccagt cctatcctcc 6540
agcctcccat catagcgcag atggaaagat tgaggcctgg ggagacattg gctgactcaa 6600
gagccaattc caaggattgg gggaagtttg aaggagggct ccctactgag cctcagctgc 6660
tgtggggaag gccctgccct gttctccctg cctcagtccc aggccttacc tgagctgggg 6720
gggtccaagg agccatagaa gaattcccat ttggctctag cgattctctg ggcatgagag 6780
gagacttccc ggggggagga ccaggtatcc ccctgagcca acgagggaga cacaggcacc 6840
cagagataaa gccagacata caaggacaca agaatatggg acagaaacag aaattaaaac 6900
atgcatcgac agagatggag gttcagagac acagagacaa acacagaggg caagagagag 6960
gcagatttag aacagattaa aggattcaga gtgaacagca gacaagccca tgtctgacag 7020
gaggctgagc cttgcccctt cactgggggc ccagggagcc caggggaagt tgcatacctg 7080
gttcaggaat ccagtgtcag caggtcctcc gaagagtgtg gccaggcgct caggggggcc 7140
ccccagccca ttggggagag aggagtaaag gccatctgct ccaacctggg gtggggctgg 7200
cggcccatgt acaaggttcc ggctgccagg tagggccgag cctcctctgg cggggagtgg 7260
gtctgatgtg gatgcttcca gccgtaactt ccggttggag ccaggcccaa gggctggggt 7320
gggcctggga ggagaaaccc cacccagatc ccagctccga ctcaagcccc ctggagcagg 7380
tagcccattc agtggcctca cactggcctt ctccacaaag cggaagatga ccacagagct 7440
ctgggccccg ggtgggggct gcccagtggg tgaagagggt gcccagggtg agggagcaac 7500
acggggtgag ggggggccac gcagaggtgt acagggtgct gtcacacgtc ccagttcccg 7560
gcctcgagga cctggacctg ccactcgccg tagcaaggag cctgtgctgc catacatgct 7620
ggctgggggg ctctggggca ccgggccttc ggggagccag cggcgtgggc atcgtggtgg 7680
ggagatggca cagtcgcctt ccgagcagaa gcgcatggca ccctgggcca tgctggggcc 7740
gggggtcagg ctgggggggc agggatggcg aggccaggcg gggagtaagg gggctgcatg 7800
ctcttcagac aggcctgtaa gagaggaagg ggagcatggg gtgaactgcc aaggagttag 7860
atggatagga cagcagagat agggaaggga aagttggcta gggcctctga gtatcaggga 7920
cctagcacac cctggtaata tgggatcatt gatcccacgc ctggggacta cttccataga 7980
ggcctgacga gaaaagactt atacacccag tgggcccctg gctcctttcc ttctgcctga 8040
ggccctgagc atcaccctgg gaggagagag gaagcaactt caaagttgag tgagcccgga 8100
gttcttatgg cactttggag ctacttctag aataggggaa gttggtaccc caccctctgg 8160
aaaaggggtg tcagccagct gtcaccatca tacagccctg tgacccctat agtctctctt 8220
atggaggccc ctcactcagg gaagggaaac tcaggcctcc tggaaggaga gggaagagga 8280
acaggtgtta tatagggatg ctgaagggct gagaggcaca gcatggaagt ggggagaaaa 8340
gcctgagcag aggctcctac ccccaatata tacatactga gtgggaaagg gacattcttc 8400
tttttttttt ttttttttga gatggagttt cgcccctgct gcccaggctg gagtggaatg 8460
gcgctatctc agctcactgc aacctccgcc tcccaagttc aagtgattct cctgcctcag 8520
actcctgaat agcttggatt acaggcgctc agcactatgc ctggctaatt tttgtatttt 8580
tagtagagat ggagtttcac catgttgtcc aggctagttt caaactcctg acctcagggg 8640
atccacccac tttggcctcc caaagtgttg ggattacagg cgtgagccac cgcacccggc 8700
cagggacatt cttttcttca tacaggtaca gacaggcacc tacacacaca caaacacaca 8760
cacacacaca cacacacaca cacgcacaca catacataag gacaaatatg tcagtacaca 8820
gttaaacaca ttcataggca acaccctctg gcacacacac agacacacac acacacacac 8880

```
tgctcaaaga cacaccctca tgcactctca cagtcataac aagtatgtgt gtaatgaaaa    8940
ggcacatgct tgttttacac acacaccatc atcaaacagg aacacacaaa gaggtaccca    9000
aatatataca caagctgtca ttctccagtg cacacagagg tccaggtgta tcagacactc    9060
acagagacct actgctcaca gtcacataca catacagatg tacacataca gatgtacaca    9120
ctcaagcagg gacacacacc gtcccctccc ccccacctac tggggtctct tgaggggaat    9180
ctgcacccat attcctctcc tctccctctc tccccagggg taagacagat cccgcccagg    9240
agagaccata accaggtaaa ggggcctctg gtggcaatgc aggggagggg cagaggtcag    9300
gagtagcaac cctgccaggc aggttccgga agctcaacgg ggtcccagcg catacccggt    9360
gcagccccaa ctcaatgcca cctcagtact caccgctgct cgcccagagc tgagaccgag    9420
gagagacaga ggagaggctg ggcccagctg agctgtgaag gcagcgcggc tcccgtttgc    9480
tccaggcccg cccgcctctc agtccctccc cctacccccct acgcgccgct caggcaacgc    9540
taatcccccc caccccccgc cctctcccag ggcccagagc cccggaggct gccagccccc    9600
cttgtcgcta ggtccccgct gggctctccc gccaacctaa gacccgcccc agacaaggag    9660
gagggcgcat ggaacccctg gttctattcc ccagaccctc acgtgcccca ccccacttgt    9720
gtccacgtct ctctcgccca ccgcccccttg agccacacgt aaggcccggc tgcacacgcg    9780
tgtcccgctc cagaccccac gcgtgtccct aggaggctgc cacatgacct gttacatgcc    9840
acacgcgctc ccgcccccac gagcacgcgg ccgcgccaag cacacggtat gggtgtagac    9900
tggacccgcc ccctaaatcc aggagaccat caactatgga aaggagatct agggaacacc    9960
gtcttgaacc cgccaggggt tttgagtctc ggacccagga gatccaaccc tgaccacccct   10020
cccaggatgc agcaggggga tgttaaatca gacttcagga agaacttcct tgctgcctga   10080
gttaggtgtc gtcacgcctg cccaggggta agaggttaga cgggatgaac tagggaccgg   10140
aagacgtggg ggaccggcaa cccttgtgcc cttggccagg gtagaagggg gccctgctat   10200
tttaagaaag agccgctgga gttactatgg caactactct ctttgggagg gccagggcgg   10260
gcagatggtg acctcggaag gaagaagggg tctgttcggg cagtcagggg gcgcgaggac   10320
gtgcgaggcg ggggcgcgcg gggctcaccg cggggagggg cggcgcggac tccgcgtggt   10380
gctgaagggg acagcgccag atttgtcgca ctgcgcaggc gcagaggcgc gaacaaagag   10440
gggaggggga cgaggcgcgc ggccggaggt ggtgcgcgct cacgtggctc tatctttgca   10500
ggggtctgtc ctcccagcta ccccctccct aagcaggctg aggaagaaga gatagcaagc   10560
cctcacctaa cttgacccct ttcttgtctc tccccgctgc tagcttctcc cttctcgctg   10620
ccttgcgggg tggggaaacg tggaaggaat tctgggaaaa ccagatgtct gggttctcta   10680
ttcccgtctg gcttgattct ggacaagggg cttcctctcc agggcctcgg tttccccatc   10740
tttaaaatgg actgatagca tagacctgcc cacctttcag aggtgcccga agccttgacg   10800
agataacaga tgagaagtgg ttttcgttgt gatgagccag ctgaagcggt tgcaccggag   10860
cgccagaccg tcagcggtgc agtggctctc gcgtccttca catcccccca cacccatccc   10920
gtggatgatg gggaaggca ctactcctgg ggttccccga agcggatcct gggactggga   10980
cctggggacg cggtgagagg cagccctgaa gctgagtcca gaggtctggg gcgtcattgt   11040
cccagggcgg acccagcccc agaccctaca actaaaccca aggccagccg agaagcacta   11100
ccactggcgg gctgagctga gctctcattc ttcttaggac tctggacctt tggaggaaat   11160
gaaagggacc cagttggaaa tatagtcttt cttcgcgccc cctacatctc tctcccccgg   11220
cggaaacgcg gcccaaactg ttacgaagtg gaagaggctg gatagctctt cataattaat   11280
caggaatggg aggtttgagc aggtttactt gatgagggaa acattatagc ctcgccaatt   11340
cattcattat ttcaacaatt acttattgaa cacctgctac gtggcagaca gtaagagcct   11400
ggatagtttc tccccgtttt tcaaagtcgc taaacgcgac cagagggcag aggagaggcc   11460
ggtaacctaa gagtcctggc tccgcccccct ccgcgtttgg ccttgcctgc tcctcccacc   11520
accacttcgc tcagaggcgg ctactggctc aagaggacga cagaagccag tctctgatgc   11580
ccaccgcatt ctccagcttc aggcctcaag ccctgtagcc gagaaggcga ggcctaatgt   11640
tacaccaccg gtctgttccg cctccgtttc ggggtccacc cgaaaagctt tcagatcgga   11700
tcctcggtga gacggcactc gattaaatag gtctgtctct acaggccaag gaggcgggtt   11760
tggtgcggcc actccaaggc caaagcgaga aaatcttact gcgcacgcgc aatagacagc   11820
cgatggagcc accgatggag ccgtccggag gggagcaaga gcccggagcc gtcaggtacc   11880
gagcaccgga ggggccgaga gggaagagga acctgaggga ggggagccga gccggggctg   11940
ggtctggggg ccgctcttgg gagctggggc gcaggaacat aacggaggcg gactcgcagg   12000
taatagtggc cccgggacgc cagtgccaac gccccctttac tcgcaggttc ctggacctgc   12060
cctgggaaga cgtgctgctc ccacacgtcc tgaaccgggt cccgctgcgc cagctgctcc   12120
ggctgcagcg cgttagccgg gccttccggt cgctggtgca gcttcacctg gccgggctgc   12180
gtcgcttcga tgccgcgcag gtgagccggg ggctgaagcc ccgcccctgc ctgggtaccg   12240
ccccgcctct agcccagccc ccagccccgc agtatgccct tctggacccc cttgggccgc   12300
cggggctgcc cggaagggat ccacgcacca aagggcaaat actcggtagc gactcagagg   12360
gaaagtgggg tctctcctgg gagagcagga ggctgccaga aaagaactca ggtcaggggt   12420
gcataggcgg ctgaggagtg cgggacgggc tgagagttgg ggtgcctccc cgcccgcagg   12480
tgggtccgca gatcccgcgg gccgcattgg cccggctgct gcgggatgcc gaggggctgc   12540
```

aggagctggc actggcgccg tgtcacgaat ggctgtcaga cgaggacctg gtgccggtgc 12600

tggcgcggaa tccgcagctg cggagtgtgg cgttgggcgg ctgcgggcaa ctgagtcgcc 12660
gggcgcttgg ggctttggcc gagggctgcc cacgcctgca gcgcctgtcg ctcgcgcact 12720
gtgactgggt ggacgggctg gcgctgcgcg gcctcgctga tcgctgcccg gccctggagg 12780
agctggatct caccgcctgc cgccagctca aggacgaggc catcgtgtac ctggcgcaga 12840
ggcgcggcgc tggtctccgc agcctctctc tggccgtcaa cgccaacgtg gggdacgccg 12900
cggttcaaga gttggctcgg aactgcccag aactccacca ccttgacctc accggctgcc 12960
tccgcgtcgg aagcgacggt gtcaggtgcc tgggcctgat agggcgggag gagggcagtc 13020
acttagcctc tgctggtatg gggcgggctg tagttgttaa aaggccggac tgagggttct 13080
gaatcttcct gcaaaccaca gcaccccat tctgaacaga aagggcctct aggattgcct 13140
aggccagaga gcccatagtt taaaaacttt tttcaaccaa tgtttacaca ttgggagatt 13200
tcacatagaa agtcgacttc tggcttttct tcaacaaggg ggagatcttg caatcctagg 13260
cggagctgag ggccaggttc ccccttaaa tgagacagga agtctccagc tcactggaag 13320
ccaatccagg cgcctccctt gtgtagggac ggaaaaagta tgcccctgcc ccgagctgga 13380
gggagaccgg gattttgccg gagccagtcc gaggctgagt gagcccgggc ctccacctta 13440
ccccaccaaa ggtgtggtgg cctcatgtcc ctagcctcac cctgctcctc cctcaggaca 13500
ttggccgagt actgccccgt gctgcgttcg ctgcgggtgc ggcactgcca ccatgtggcg 13560
gagtccagcc tgagccgctt gcggaagcgc ggcgtggaca tcgacgtgga gccgccgctg 13620
caccaggccc tggtgctgct gcaggatatg gcgggcttcg cacctttgt caacctgcag 13680
gtctgacccg cctgccaatc ggagc 13705

<210> 32
<211> 3574
<212> DNA
<213> Homo Sapiens

<400> 32

cacaccgccc ctcccggtga gtcactctat ttcaagcatc ctccccatc acatgatcct 60
gttttatttc ttcattgcac taagaatacc tgaaatgtat atgcctacat ttatatagtc 120
tatttccccc tgagaacgga agcttcatag aaggtgaaac aaatcttgtt agttgctgcc 180
agtcgcacag taagccctct gtagaaaatg tttattggcc acattttttt aaaaagcgaa 240
taaacctcaa gattaagacc tcaaaccaaa ctattccagt ttccaattgc ttacagcgac 300
atactgagaa atcgagtgac tctcaagaga aaggccattt tgaggctggg gtcgaaccaa 360
aatccagggg gtccacacca ggtcagtacc ccggaattgg ctgttttgca agatttcggg 420
agtaccaaat tggcatgaga catttgacaa cgaggccagc catgcaccga gctcaggttt 480
tatttccgtt gtccttctga cgtcagaaaa agcacgagtt gctgcacagt ttcttttatc 540
aaggtgacaa attacaccct cgctgggccc tgcattctgc aaaccataaa tttattttcc 600
tcctccccct tatattgaaa aatgtaaaaa cccactgcag agaggtcaaa accccttcca 660
aacgcaaata aataccgttt tctagcagag gcgtcttttt tcacttttgc tacacacatc 720
accgcggatt cgtccgagaa agtacgtgat cctggcggca gttaggtttc taacagagaa 780
cgagggtttg tacaaacccc gggtccgcgc cgcagagcaa gacaaaggac tctgaattgt 840
agcaccacct tccccagccc aaggcagcct gtgaaaggca ctgggagcgg cgtgacgcgg 900
gtaccgcgaa attccttttg ctgccatatg cacttgtgcc ttctctaact acagttcagc 960
acaaacctca ggcgggcgat ttggcaggca aacggtgccc aggcagccgg gtaacctact 1020
gggtcccagt gggtgctggg gagccccatg cgccaggagg tggataattt agggggcaag 1080
gggctcgcac aagggcgaag atgcgctctc ctcccggcac cgacccacgc gtttaaaggc 1140
ctctgcggca acgtttaggg acgcgaacta ggtgccgggg gttggggagg aggtgggact 1200
ggaagcgaga gcagtctgcg cccaagaccg cgcaagaggt ggccttttgt tcgctggctc 1260
cagcacatcc cggctctcga aaaaacacta cctctgtccc ctgccacccct ctgcatttta 1320
gaaaatcctc ttcagggcag gaaaggacag tgagtcactg caagcatcct ttagtaattt 1380
ctccccgcgc cctcctactt ccccgcgtta cagaggggca gagcaccagg aaggacggca 1440
aacatgcccc ggtttagtgg tggtccgcgg ccgctaccca gccggccgca cgcagcaccc 1500
cccggacatc acacgatctc ctctgggcct tgcggacaca ggaaattttc accaaaggcc 1560
ggggattcaa acactcccag aacacggtcg cgccctcggg tcagctgcgc tctccctcca 1620
gacgtgcgtg gttcctggct ctcgtaaaaa tggaaccaga tctggtacca cactctgcca 1680
cagccacagg aaaacacacc aaagtcacag gaaaacaaac caaaaagtaa gccacagccc 1740
acgcaaaggt gaactcgggg gccctggcgc ttcctcccgc tgccgcgtgc gttcgggaag 1800
cacaggagac ctggactttt cctctcctaa aagcaacatc cgaaagcatc gggcacagcc 1860
gagctctctc tgctttcacg cccgcagaga tgatttctac gttacctcct cctccgacat 1920

```
ctaaatctcc ccttgccgct cgcccagccc tccccgggaa cggataatca ccttacctcc    1980
ggaaataaag ggaaaggtgg caaaaggcga tgtggaggtg tccgcccttc cggggttttt    2040
accctcggca gtttgatgtc ctttgtgtca aggtctggct gcggaggccg ggaaaatgtg    2100
gcccccgtca gtaagggttg ggcagggagc ttggcgtggc ctggcggatt tcggaggaag    2160
gtgaagggcg gaggctcctg gcgaccttct ggcggagtgg cgtggccgcc ccagtccgcg    2220
ctcgcctggc tctcctcctt tccgggccga ttgcatcaga atctcccctc accaccaacc    2280
cccactcaca ctctgctcgc cggcctcgca cttacatcga catgcaccgg cgctgagggt    2340
cacgaatcgg agtcattctc ctactggggg acgcgtggcc cttccgcaac catggcttgt    2400
gatggtttaa cgcggacagg ccattcctcc ccgtcccgcg cgcggtctcc tcccctcctg    2460
tgcgcccgct ctttaaccaa agctgcgtgt cagtgagccg tgccggtcac atggtgtcac    2520
tgctggcggc cgcaccttgc tgcctccctc ttgcagctct ctgacgatgc agcgaccgcg    2580
gctggcggag attgagcaac ggggaaaggg tgagggaggg gttgcggggc gcgcgtgtgc    2640
ccggcgcact gaagctggca ccgggagcag aggctgcgcg ccgccctggc acctgcccct    2700
ctccctcccg gggcccggcg gcgtgttcgg aacccagctg cggctgcagc tggagaatga    2760
gagcccttag ggatgggggt ggggcggcta aaggggcacc ggcggaggac aggagggctg    2820
tgcgttccgc tggggcctgc attttgggta cgccagagcc ccgccggggc cgaaggagcg    2880
agggagttgc attgtgggaa gggcgcagca tgaggatggc cgcgggagtg gcaggtccga    2940
gcgcagcccg gagagccgcg ggcagcgcct gggactacgg gcagcgttgg agcggagggc    3000
tggccctggg cacagcctgc agatggcccg ggttggcggg aaggggaccg aagtggcagg    3060
ggcggcgggg agctcgggca cctttaaaaa ccctttgtcc ctaacctgaa gcgggaggca    3120
cctcccctct tccggatcca gcacccgcag acctggtcag cccttcctga gatctgccct    3180
gtggagctct gctttgggga ctgggtgtga ggtgaaccag aaggatttgc tgagcaccga    3240
gaaagcaccg gcctaggcgc aaagagccgg gggccagctc ttggagggcc tggagacccc    3300
acgtggtccc gaccgaagga cctggtgcgt ccaggttaac ggagctgggg cgctccaagt    3360
ccgggggacg agggagcggc atggagggga gcaggggcaa ctgggcctgg ttccctttttt    3420
tcccgcttac tctttgtttc agacacagcc ccctttcact ggctgggcac ttgcttgcag    3480
aggagttgtg tcttaaaatg gcgacaggaa atcccacctg cgcctcagcc aattagactg    3540
tcatagcgcg aatttgcagg gtggaaacgc gagc                                3574
```

<210> 33
<211> 3506
<212> DNA
<213> Homo Sapiens

<400> 33

```
cagcggtaaa gctctgccca cgttaagtaa caaaggataa gttagtcttt gttgtgatca      60
ctttgttgta ctgataagct acgtatttct actcaaggat tcaaattctc acctttctca     120
agaattgggc caaaaccgat aaactaaact tatttacggt ccactgatta aaggttgttg     180
cataataagt tcttgctatg ttcagcagtt ggattcacag cgccagaaac ctataactgc     240
ttgactttcc tccccactac actgcgaaaa ttgcccctta aatgtaacta accctaaaac     300
ctcaacagta tcgtggccag gcgtggtggc tcactactgt aataccaaca ttaggcatag     360
gcgaggggat tgaggccagg atatcgaaac tagcctggga aacacacgga gacccggtct     420
ttggaaaaat aattagcctt gcgtggtggt gggcgcgagg ttccggctaa tcgggaggct     480
acagtgagcc atgatgacac tgcactacag tctgcgcgac ggcccatgtc agtaagctct     540
ggagcacctg aaacaagttg tgttgggtat tttatttact ggagagcgat tagtgactga     600
tgcctactta cagcgactag agacgcatgc tccgatagca gcacaaactc agcaggcgcg     660
aacaaatggt aaagagaaac tgggcaaaca agcatcacgg ctcctcagct gagaaagtgg     720
gggccctaaa aagggccttt tgttgataga aagggacgct caaccaccga aaccgtagag     780
ggtgcggccc tggcgcttga gcgcgtagac cacatccatg gcggtgaccg tcttgcgctt     840
ggcgtgctct gtataggtca cggcgtcccg gatcacgttc tccaggaaca ccttcagcac     900
cccgcgagtc tcctcgtaga tgaggccgga gatgcgcttc acgccgccgc ggcgagcaag     960
gcgccggatg gccggcttgg tgatgccctg gatattgtcg cgcagtactt tacggtggcg    1020
cttagcgccg cctttgccaa gacccttccc gcctttgccg cggccagaca tgacgagcaa    1080
gaggagtctc acccaacgct ttgtgaggac tctggcctga ggcagcgcct ttatacgaca    1140
gttggcggac cgaactgaga acctgaaaga agtcggcggg aagtcccgcc ccggtggggg    1200
aggggaaatc taaagggcca aaccgaaata gggggaaaaa aaaagcgagc ttcttgtttc    1260
cgtgttctga attttgtaac gtgcatagta ttttgttacc acgttatgag gctttaaaaa    1320
attgcttttg aacgcagaag atatacatca atactgtggg aaatacaaga aaggacaaga    1380
aattaagaaa ctacaatgtt atcccatcac acaggctagt taatcatgta ttttgcagag    1440
cagttgcaca tatttttcca agaaaatgta tacagtgttg tatatggagt tttgtaacct    1500
```

```
ccttatattg attataattt aaccaatttc tattaaagag ataaaagtga tgttttggtg    1560
tctatgtttc ttaggaatta tcaatagtta taatcagttc cccagcaatt ttttaatcgg    1620
ctgtatttta aaaataatgt tttccacatt caacataaat gtactttttc tctatacttg    1680
ggaccaatat tgaaatttat gattttatta caccaaaatt taaattttat tacattaata    1740
tttaaaattg tattagaggt ctcatgattt ggtactacgg gtctccgcat tatttccttt    1800
ccaaatttcc taatctgttt caccaaggtt tctggacaac tttagagacc ttttgtgaag    1860
tttgaataaa atctcttcga gattttgata attgcattag ctttaggact taattggaat    1920
agaattaaaa tccttaaaac aagctcttat aactagaaaa ttggtgtttg taggttttgt    1980
gtgtggggtt tttttttttt tttggaagga gtctcgctct gtcgtcaggc cggagggcag    2040
tggtgcaatc tcggctcact gcaacctcca cctcccgggt tcaagcgatt ctcatgcctc    2100
aacctccgga ttagcgcgga ctacaggcat gcgccaccac gcccagctaa ttttttgtat    2160
ttttagtaaa gaacaagttt caccatgttg gacaggatgc tcttgatctc ttgacatcgt    2220
gatccgcccg cctcagcctc ccaaagtgct gggattacag gcgtgagccg cttcgcttgg    2280
acgcttgtag gtttttaaaa agacactttt atatgaccca actaaagatt tgttatcaac    2340
cattatggag caactttgat tccgtatatt tgattttctt tcttattaat aaatacaggg    2400
ttatactctg aatttttttt tttttttttt tttttgtgga gacggagtct cgccctttcg    2460
ctcaggctgg agtgcaatgg cccaatcttg gctcactgca acctccacct cccgggttca    2520
agcgattctc ccacctcagc ctcccctgag tagctgggat tacaggcact caccaccacg    2580
cccggctaat ttttttgtat ctttagtaga gacggggttt caccatgttg gccaggctag    2640
tctccaactc ccgacctcgt gatccatatg cctcggcctc ccaatgtgct gggattacag    2700
gcttgagcca ctgcgctcgg ccactctgaa attattttaa catcaatgaa aattataaaa    2760
ctcctataac tattctaata taatctctta gtattcaatt ctttgtttag aaagtagtta    2820
agagttgaaa ctctggaaat agaaaacttt ggttttagat taaatgttta cctttcagac    2880
tcgagctgac ttgttaccga tcagacacga ggtgacttgt ttattctttt taaatctttg    2940
ctcatctaca aagttagaat aggatagtga cggtactgtt ggggtgcaga atgattcccc    3000
aaaatgtggt tcttgggcat gctgagcgct tttgaacatt gaaaggcctc agaaataagc    3060
ttcagaatca aagcccctct aacttcgtct tctttcccat acatgcgaag ggactctgac    3120
atttcctaat cggaccaaga aaatttctta ccagaagtaa caattgcctt ctatcccctc    3180
cctgttattt cattattgca gaaaagaata ctgaatatgg atttttcaag ataatgtctg    3240
cctctcggtc tcatttaaat taccaagaca tactaggtgc tgtggctcct cccactaatc    3300
ccagcactgt gggaggtcga ggcaggtgga tcccttgagc tcaggagttc gagaccagcc    3360
tggccaacat ggcgaatccc tgtctctaca aaatatacaa aaaattagcc aggtggtgtc    3420
acatgcctgt aatcccagct acttgggagg ctgaggcagg agaatcactt gaacctggga    3480
ggcggaggtt gcagtgagcc gagatt                                         3506
```

<210> 34
<211> 22489
<212> DNA
<213> Homo Sapiens

<400> 34

```
ggccgctggg agacactgtg gtcagtgggg cgggggggctc gggcacttgt ccccagatct      60
ccggccacct caagtgacag gaaggggggc cagtgaggac atgaacagtg gccgccccca     120
ccctcaaggg ctggcctcac tgccccttca tctctgcctg gcccagcagc tctgggagca     180
gccaccctgc agcccatgcg tgtggggacc ctgccgtgcc ctgagctgtc gaagcagcct     240
cccactgggt gctgtgctgg gcccctgtcc ccatcctgca gggaggaagc acagctgggg     300
gagtggcccc ccgagggccc ctgggagctc agatcctacc cctggaggca gccctgggga     360

ggcccggtgg agactctgct cttctgctga agctgctgct gaagagtgac aggcctgccc     420
agcgccgggg gagacccggg actctcagag ggagtgcagg acagggcagg gcagggcagg     480
gccatgattc ccggtgacgg ccaggctgcc atgggccgaa ctgccatctc tgggcctgcg     540
ggcaggcgct cagctcccca ggcctcattt tcctcaccta aggaatggga cacgcacacc     600
ttcgccccca gggtggtgca gagaacagga cggtgcctcc gagcagtgcc ttcaacgccg     660
tctggggcct gggaatgcac caggacttga taaataaaca ccagctctca acacctcgcc     720
ctctgcaagg agaaggccag gaacgtcccg tttagagaga aggcgagtcc acgagcagca     780
gacagtcggt gcttaacagg tgctcgctct tcttcagagc cttagggtgc cagctggcca     840
gagtgagcct cgctgaccag ggagctgtgg cagccggccg gggtcggcc cctggggcag     900
gtgggatgcc tgtggggccg gcgtaggctc aggctttgtc tgggagtgcc ctgccccgag     960
cactggcctg gacacctgag ctgagcctca gacaggcgtt tcggggccga cccggcagga    1020
gcagccgcta tccccgggca gctgagccca gcagcgaggg ctggtagaac gatctagacg    1080
```

```
aaacactgaa cttaatagca aaaccacttc tttcgaggga aaacacgatc cgcagccagc 1140
cggcgcgtac acatggggcc tggaaaagaa gagaattccg gagacacaaa cggccctacg 1200
cgtcgccgcc tcggcccggc tgctaagtta ttaaactcct gcacagggaa aagcgataaa 1260
gtaaacatcc acctctcccg tcgctctcag aagtgaaagg ttcctgagat gggaaatgca 1320
gacggcctgc agagtgctcc atccaaataa aatcatcaga tgcttgaaga ccctgggaga 1380
aatgggccca gatgaatcac ctgcgcaaag ccacttagca cttcgcaggg gccccgaagc 1440
ccggtgtcgg caaggtgtcc gtcgctggag caggggtggc tgatggatct ggtgcggggt 1500
gggcgggagg agtcgcagac tcagatgtag gaggtttggt ggggccactg attcacgtct 1560
ggcccggggc tctgtccctg ggtcgggag gggaccaggc tggggaagtt ggggaggggg 1620
tttctccctt ctctttccca gctgccaggc cgtcagtggg agacacaggt cttggggacc 1680
aggccctgca accccttctg atcccgacgt ctgagactgg cctggtgctg gctctgaggc 1740
ccctgtgcaa cctcagctcg ggctggctgc ggaggacctt tgagggtttc ccggcaaggc 1800
cttctccctc tcagctggcc atggcacata ggaccccact cctccgtatg cccagggcag 1860
gcatcgctaa tcgagccctg cactccctcc aacccctttgc atcagggccc accaggccca 1920
cccacttctg tgcccagacc acctggccga tgggcggccc tgccccagga ggaacagggg 1980
tcttcctgct tccccagagc tcccgctctg ggtcctgcat ccacgtctcg gatgtgccgc 2040
cctccccaca cctgggagcc acgccgaccc cctctgcgct ttcctgggga gacacctctc 2100
tgcaccagag atcggccccc cggggtctgg gcgtgggggc ggggatttgg atgttggggg 2160
tgcagaggcc atggcctcgt catcttctct gactctgcca tgagagcatg accagggcct 2220
gttggccccc tgggccctga cgtccccggg cccggcacct ccctggaagc cctacccatt 2280
ctcctctttt cctttctca caaggaccct ttcaccgtaa tcctgcatga cctcatctta 2340
gctgattaaa tttgcaaaaa tggccaggct cggtggctca tgcctgtaat cccagcactt 2400
tgggaggctg aggcaggcag atcacctgag gtcaggagtt caagaccagc ccggccaaca 2460
tggcgaaacc ctgtctctac taaaaataca aaaaatagcc gggcgtggtg gcaggcgcct 2520
ataatcccag ctactcagga ggctgaggca ggagaatcag ttgaacctgg gaggcggagg 2580
ttgcaatgag ctgagataac gccattgcac tccagccagg gcaacagagt aagactccat 2640
ctcaataaat aaataaataa atttgcaaag atgccatctc cgtgtaaggt cacacgctga 2700
ggttctgggt ggatatgaat ctgggggaca ccgttcagcc caggacagga accaactgta 2760
gcagggcagc tgtcccgggc acacagtctg actggcactt accccctggca ggtcccccgg 2820
agcctggccc ctccaccatg gggcgggggc tccagttact tccctggggg agtccctaac 2880
ctgaggtctc ctgtgccggg gtctagtcag ggtgggggct gcaccccatc cctggagcag 2940
gtatgctggg ccctgatagc aagctgggga gggttccagg gccagaggag gactcagggg 3000
ctgaaggcag tgatgggtcc agcaggcagc agggagggca gggcagggga ggggtgtcaa 3060
ggcccataag cgagaggagg caggagggag gacatggtac ccacagctgt ggtggaggag 3120
ggagggtgtc cacaaggtgg gtccccagtt tggagtctca ggcaccctcc gctgtggcca 3180
ccgactgctg gggaattgat cagcctcata gcatggcatg gagttgggcc aggcagggct 3240
ggatgcccgc caggggcact gcaggtgggc taccaggggc tggcctgggg ttcagctgtc 3300
acccagctga cacgggatca ccaatggctg ccatggctca ggcatccctc tcctgccacg 3360
ctggggacac tgtgacacag gctctcaggt cagtgaggac agtgtgggtg tcggagcctg 3420
gagggagggg agtggcatcg gggaggcccc cgccgagcct ggacagtggg gcaggtggaa 3480
gggtgtgtgc ccggccgtgc actttgggtc tggtgagcgt ggctgagggg cacagctgct 3540
cttggtcggg gctgcagtga gtgctcagcc cacttggcca caggctggcc accagggact 3600
tggcgtctgc tacccacagg actgaggact ccaacagcag tcccagccca ggccccgaaa 3660
agttgagcag agaagggcag gaggcccccg tatccctggg acccatcctt ggcaggctgg 3720
gttgggctgt gggggacaca cctgctctgg gggatgtggg aggggggcgc ttccgggcct 3780
gcagctgttg gccccgagga cagacgcagc ctcctcagca acagctgggc cggaggctcg 3840
ggagccaaga gagaccagag cccaggccag cccggccccc accagggatc agttacctcg 3900
gctacagatg ccagcagggg ggactttcag ggggtgggca ccaggctggg gtgggcagct 3960
ggggtgggcc ggggctgcag tgctctccca cccagccttg ggcacaggac gagccagccc 4020
ccacctctgt ccactcagca ctccgcaggg gctgctgtct tgctgagtgc aggacgctga 4080
ccccaggatg tcaccatctg atgctgccca ggctctcccc tcagaatacc tgcttagtga 4140
ccacagccac cccagcccct cccactgacc ccaggcagag caaggggccc cacctgcacc 4200
cagcaggtgc agactgggca gggcagggtc ccaggagggt gcatgcttgg ggagcccccg 4260
cacgcagaca gtgggggggcc agggaggagc cacgtgctgg gcacaaggca gtttccgcag 4320
gccccttgct gggtctgtct cagcacggag ggaccggcag cacacagtag gtgctcagca 4380
aatgccgggg ggcggggata agcgagcacc cacgggggga agcactggct gcggtttcgc 4440
cacctgcctt ggcagcaccc ccacctaggc cctggatggg cggcgaccac cgcctctctg 4500
ctcctggggc tctgactccc agcatccggc agccggggac ccggcctccc ccaaggcctg 4560
ggagcagcca agtggccagg tatcaagtgg caaactcagg ccagggttgg gtgacggctt 4620
ggagggcccc tgtggcagct gagccccgct cctcctggtc ctgcccaccc tcctggtgct 4680
gcccaccctc ctggcctgca caccctcctg gcctgcacac cctcctggcc tgcacaccct 4740
```

```
cctggcctac ccaggctgcc actggcccga gggtcccagt gaggatgagc cggagatgga    4800
cactgagggg gttgacgggc ctggggcagg ctggccgggg caggggggct gggcctgtcc    4860
agcggccgcc tgccctcaga agaatgacac cctgctttct cccctgcccg aggaggtggg    4920
gtgagggtca gaccccagca cccatgtctc tgtttttctt tctcctgaaa agcctacaga    4980
ctctcatctg aattgctcca gcagagccca ggcggggtac cacccccacc cacacacaca    5040
aagggctgag acagtgcagc ctggcgtgtg agcagccctg gtgtctccag gctggggtgc    5100
tgtccgcctg cccacctggc cacggtcccc aacagccggt tcagcaaagg acaggcagac    5160
cctacgggcc aaggatgcgc tggcctggcc ggtgcggggc caggcgggca gcttgccagg    5220
atttggggat tttgagccag ctgctgcagc aggtgtggcc ggcccagcca ggcaagtcct    5280
gctgccggga gggtcgccca ccagctgctg gagggtgtgg ggcggccggg ctggccactc    5340
gccggcagca aagccagcag agagtgggtg gggctggggg aggcgggagg gtcccagtcc    5400
cacccccagg gtccagcttc cctgggccct gcagccaggc caggaaggtc agctgggctg    5460
gttctgggcc ctaggtctta gcctggaaac caggggtttc aatcttggca tgagaaagcc    5520
ctcgccacag ggcaggagga gccccacata gaacccccaa cccctgtcct cgggcctgag    5580
cctggcagga ctggacgccg gacaccggcg ctgctgggag ccttgagaac tgcacgtgtg    5640
ccccccagtg gccagcgccg gccgtgctcc gcctgattct tcaggccctg atgcggggtc    5700
ctggggctca gggagccgag gcccggggtg ggggctgcac ggtggtgtgg tcctctcaga    5760
ttgcaggatc agagggcagt tctgaaagtc tccttggtca gggagctggt ttgcagagct    5820
gaaggggagg agggggccag gaggttgctt gcggccgtgg tcctgaaaat caggccctgc    5880
caagggtctc tgagggccag ctggggaggg aagggcacac agtgtgttca gagcaggacc    5940
ccagggcgct cgggctcagc gcttctgggg acaaagggag atggagcaga ggggcctcag    6000
ggaggcccgc cccgtggtgg tataagagcc actgcgaaca ccctgctttg gtgaggctgc    6060
gccccaggtg ttaacagcca caccccagca caccaggtgc aagccactct ccataggccc    6120
cagcctgttg caggaaccct aagctccagg ccgggaagga atgggcaggg ctgccgtagg    6180
gactgtggtc ttgcaggacg agggtttgtc aggggtgctc aggggacagc gggccgggac    6240
gtgctggggc tgctgtaggg actgtggcct tgtggggtga gggttggttg gggtgctggg    6300
gctgcttttg ggtggccagg ccctacctga gagcccggcc atgggaccca aggtcagagg    6360
gtggccgagc tgagggttgc ctgctttggg ggggtccctg gccccaaacc catctacctg    6420
gggggcggca ctgaggcagg tccccaaggg cagaaagggg agtgacctgg taaaggcagc    6480
cctgtgttgc ccctcctctc ccgtccatgc cacgcacacc ccaatgaaac aagaaatttt    6540
ccttgacccc tttgtgggcc tggtgacagc ggtatcttgc ttagcccaga gctctcaacc    6600
cctcgaggca gcaggagcac gcaggtgggc gggtgcagga gctggagtga acacttctgg    6660
gcacagcagg agcagaactc cgtgcagtcc tgtggcagtg tctagggcag tgcctgtgac    6720
ccgtgaagcc tcagagggtg tgtgttacag tgcttttagc tttgctgtcc aaggatgtct    6780
ggagtgttta acagctcagt ggaccctctg ccttttctcg agggcagagg gtcagtgtga    6840
cagctttctg tatcccaagc tcttgtccag catccaggaa atatcaggtt gcatgaagaa    6900
attgaaggat agtaaatgca agggatttta tggccgatgg agggaggggg acctggaaag    6960
gggggtggag ggggaaggtg atggaagagg ctctcagagg gagggagagc tggagagggg    7020
gtggaggggg aaggtgatgg aagaggctct cagagggagg gggagctgga gagggggtgg    7080
agggggaagg tgatggaaga ggctgtcaga gggaggggag ctggagaggg ggtggagggg    7140
gaaggtgatg gacgaggctc tcacagggag cgggagctgg agaggggtg gaggggggaag    7200
gtggtggaag aggctctcag tgggaggggg agctggagag gggtggaggg gggaaggtga    7260
tagtcccctg aagtcctgcc gtctctacca gactcttctc caaagtcctg ctgtccagct    7320
gtccctctga ggtcacgctg ctcctctctg atgtcaaact gccatctccg atggccagct    7380
gcttctcctc tccttgcctg ctctgttctc tgccaataag gtcaggagtt tttatgggta    7440
caggatgggg gtggggcgag gcagtggtgg ttttggaaaa ggcagcattg gagcagggaa    7500
actggaatgc gtgttctcac tcagggctat gcttccaggc ttgagggtgg ggttcctcag    7560
gaaccccatc tttttctacc tagaatttct gtctcctgtc cctatcactg agggcccgtg    7620
aggctgacag tacccatccc caaagaagga ttggcctggg cagggctgtc aggagcacca    7680
gggcagggcc tgccaatcca gaggtccccg aggaacagat tcaggcctgg gaacgggccc    7740
acggcagggc tttcctggaa atgcctggca gagtgggcag gggacgtcat tgtgaccccg    7800
tattgccggg gacaccaggc tcagaggggc tgactgacca gccaaggccg cacaacagag    7860
ctgctgggag cgggtccctg ccaggccgtg ggacacaggg accccggctc tcgctgcctc    7920
tgccccagcc ccagttcagg acccactgtg gctgtcactg agctccagcc tcagagccgc    7980
ccaccaagcc cgacctcacg cagggcccac tgtgcccacc agagggcgcc agaagccggc    8040
ggtcccgaca ccgcggttca aacgcaggga ccgtccggtg ggggctgctg caaccccccc    8100
cccggcgtgt ccaggcccct cctcgagggt ccccagggtc ggggcctggt cggggagaga    8160
accaggtgtg gtggagctgg ggagtccggc gctgggcagt gggcggcctg tgcctgggga    8220
ccccggtgtc acccgccagg tggcagggag gctggtcccc tccaggagcc cccactttct    8280
gaggtggccc taagcccctg ctgctgtccc ttctgcccca cgccccatcc ccctgtttaa    8340
ttcccccgac agggtccggg cagggacgaa gcaggtcccc cccaccctgg agctgggagg    8400
```

```
agggggggtgg gggcgtcacc aagcatgggg tccaggcaga ttcagttcat gggggacatg    8460
ggggtcccag ggtgaggacc cctggggggc tggtcggggg gcgctcagag cccacggaga    8520
gcggcctggt gggggaggcc ttgccagcag accctccctt cccccacccg gagtcccggc    8580
tcccatcagc cccacttcc tgctcagtgt ccggcctctg cctgcccctg gtcaatgctg    8640
ccatttgtct cttcagccaa agatcccatg tccacgtgac atgatcatga caggagctgt    8700
ggggggtccc agggaggccg gcgctgggct tgaacttgcc ccgtgaaggc cccgagcccc    8760
cggctgccgc atcctgactg cttcgtgtga agggaaggga gtcacagcca cgccacaaga    8820
gaaggggctg ctgcctcccc aacccccgcc ctcccgtctg ccccaacctg cctgggaccc    8880
cccagagtcg gctgggggcc tcagcccagt cacccaagct gcatcctgcc ccaggaaacc    8940
cccacgggcc ctgaccagtg cccaccaggg tccccaggac attctggggg cgggaagtgg    9000
gggcaggctt tatgcctgca tctcatctgc gagcttccgg agcctctttg cacacttcac    9060
agggctggag gggttttgtg tttcagacgg agtctcgctc tgttgcccgg gctggagtgc    9120
agtggtgtga tctcggctca ctgcaagctc cgcctcccgg gttcaaggga ctctcctgcc    9180
tcagcctccc aagtagctgg gactacaggc acgtgccacc acgcctggct aatttttgtg    9240
ttttcagtag agatggggtt tcaccatgtt ggtcaggctg gtcttgaact cctgacccaa    9300
gcaatctgcc tatctcagcc tcccagagtg ctgggattac aggcgtgagc caccacacca    9360
ggcatgtttt tgagacaagg cctcgctctg tcccccacac tggagtgcag tggtgccatc    9420
acgcctcaag gcaacctcaa actcctgggc tcaagtgatc ctcccacctc agcctcccca    9480
gtagctggga ctgcaggtgt gtgccaccat gccccacaag ttttttaatt ttttgtagag    9540
attggggtct cgctatgttg tccaggctgg tctcttgaac tcctgacctc aagtgatcct    9600
cccaccttgg cctcacagaa agtgctggga ttacaggcgt gagccactgc gctcagccta    9660
gggttagagg tttttttttg tttttttttt aatgtgtagg tgtttcctga tgtctcccac    9720
cctctttgtt gggtctgggt ctcgattcat ttttccgatg ttctgagcag ggattggaga    9780
gtgtcttcaa tgtgaaaaca ctggcctttc tgcagcttag tttcagtctc ttacctgcta    9840
ctatatgggg aggcatcatc tgggtgactt cctcagggct gtcttccaac tcccatattc    9900
tcttttggcc acatctaatc tgctattgaa gccatcagct gaggttttta taccaacagc    9960
catattcctc gtgttaaagg tcctgtgtgg ttgtttttct aatctgctgg gtccttttca    10020
tgctattata ttgtttttca tttctacttt tatctcttta gtcattttaa actcatgtat    10080
aatctttttt tttttttttt gacagattct cactctgtgg cccaggctgg agtgcagtgg    10140
cgtgatcttg gctcactgca acctccacct cccaggttca agccattctc ctgcttcagc    10200
ttcccaagta gctgggatta caggcgccca ccaccatgtc ccgctaattt tttgtatttt    10260
tagtagacat ggggtttcac cttgttggcc aggctggtct caaactcctg acctcaagtg    10320
atctgccctc ctcagcctcc caaagtgcta ggattacagg cgtgcgccac cacgcctggc    10380
ctataatctc ttaattgttc tattattatt tctggttctt aagatactat tgactgggtg    10440
cagtggctca cacccgtaat cttagaactc tgggagaccg aggcaggtgg actgcttgag    10500
cccaggagct ggagacagcc taggcaagct ggtgagactc tttctacaaa aaaacagaaa    10560
acgtttattg gacttggtgg cactgcctat agtcccagct acttgggagg ctgaggcagg    10620
aggataactt gagcctaggg gtccaaggct gcagtgagct atggttgtgc cactgtattc    10680
cagcctgggt gacagagcaa gacttcatct ctttaaaaaa aaaatggaac attaaaaatt    10740
agaagatggt gccgtgtctg ccagtccctc ggggtgtgag ctcaccattg cgggccattt    10800
cttctatgga gcccaagccc caggggggaca tatccccacg gagggtgcat gtttgccttt    10860
gctggagcct gggtggcctc taggtcctgg aaccagactc catatccccc accccgccca    10920
ctggctgcca gtggtgcccc atccgtgtcc tggtgacaga ggcagcgccc cgggggtaca    10980
ggcaccggcg tcagctttga gtctgaaccc cagcccggcc ccagtcagct gcatggctga    11040
gggtgggtgg gggatgcccc tgccttgatc ccccacatcc tcacctatag tgcagcctca    11100
cctccctcac aggagtcgga gggccacatg ggaaaacagg catggtagac gtaaaaggcc    11160
cagtggaaat gccggttctc cttgctgcca ttactgcatt tatctggcaa ccccgcacca    11220
caacccgaga cctagaagac cctatgcccc aggcggggct gaacaggaca gacctcgaag    11280
agccggtccc cagatcggac cgccaacaac caggccggat tccgcgcctc cagccggtca    11340
gtgccttggc aaggcaggag cgggtgaaac tcccagccct cccagggagc agtcagatga    11400
tgacacatcc acacccgtcc acagggaggg agtgcgacaa ggcctgtggc tttctgcgcg    11460
agaatcggcc agtgtccttt ggggcccggg atggggtttc agcacccact ctcactaccg    11520
gggctcggga gggagggctg gagagcctcc atctggtgcc gtcggtggcc actcctcaga    11580
cagcgcgggc gggttcgcag gccctgggca aatgtggtgt tgccacagcc ccgccctaca    11640
gggacagcgg tcagcagctg cccctggccc cgccgtccct aatgaagtta tagatggcag    11700
ctcttaaatg tctccagaaa tggcagagag gctcccagag ctgacgtgca aataaattgc    11760
ttctcaggct cggaccacac aattctgctg ggaccaagca caggcctggg ccctccggct    11820
gcccctcagg gcctggctct gctgagagga ggaagcctgg ctcttgggag ggggtctcag    11880
gtgccccctc catggagccc agcaacctgg caggtggggg tgggcaccgc ccctccccccg    11940
ggactccatc tccccggcaa tcagctgcct ctcccttctc tggggcccca ggtggtcctc    12000
atggaggggt gggtgggact gaggcctgag ccccggggag gagctggctg gtgagccatg    12060
```

```
tatcccagtg gtcactgcgg ccagccgggg aggttgaatc ccaggcccca gcactcagaa   12120
gctggtggag tgggttgaat tgagcccctc caaaaacgta tgtcgaagcc ctgaccccgg   12180
gtacctgcga gcactggaaa tggggtcttt gtgaatgatc gagttaagat gaggtcggac   12240
ggggagggtc ccaaaagcaa cgaccggcac cttgtaagga ggctgctgac agcagggaga   12300
agtcgggcgc gtgacgtcag aggcagagac tggagagagg cagcccagga acaccacggg   12360
tggctggcag tgcggagcgg tagggccctc ccctggaggc ttcggaggga gtgtggccct   12420
gcggacacct cgatctcagg cttgcggccc cagggctgga ggggtcgcgt ttccgtggct   12480
ttagctccca gttcgtggct ctttgttatg gcagccccgg gagactcaca caggcggtga   12540
ctgtggacag gacccctgac ctccgggcca cagtttcccc accggcccct gccacggggt   12600
aaataggtct ggggtggtgc ttaggccggg gaactgcccc agctgcctgc cgtccttgct   12660
gtggctgacg gggtcgtccc ggctgcagct ccggccctgg ctccttgagg cctcgcaccc   12720
ttcctggcac ccactggctg ggctctcggg gcctctggtc accccgtggg tgctgattga   12780
atcgggacaa acgctgtgtg cagctaaggg gtgactcaag gccggccagg cccagagctg   12840
atcccaggcc gggaacagca gcagtgatgg tgtcggcatc caccgtgtcc ctcccagggc   12900
ccggccgtgg gagccgaggg gacgtgtctg tggcctctgt ggtctgagtc tggaggtcag   12960
cgcccagcgg tgtggcacct gggcacctct gtcacctgcc tgcagccagt gtgtcctcgt   13020
ggggggcagc gaggtgaccg ggtctctgtt gggtgcccct ggaaggctca gaggcaagga   13080
tcttcaggca gagagtttgg gggtggcccc aggaaacacc cgtcattgag gggtgctgt    13140
gaagggaagg ggtgggtgcc aatgggcagt agtgagcagg tcaccgctgt gggcagaccc   13200
caggaggcag caccccggag ctgtccctac catagggaag tggccaggct gtagtggctg   13260
taccccaggg catggactca ccacactcct ggccgttccc acgcccaggc caggcctcct   13320
gtggtcagag acggccagag tcacaggggt tgcaggaagt ggccgcaggc atggtcaggg   13380
gcaggtggca tctgcttcga gggcctgcac gtggcttccc tgccccgcca gcctctgcca   13440
tgtgggtccc gggaaattgg cgcccaattc catgagcagc cctgggctct ggggcctcag   13500
atcccccaac gctgccgtgt ccgtaggagg tgctccgggg cctcagatct cccaatgctg   13560
tcgtgtccgt gggaggtgct gtgggggcct cggacccccc aacgctgccg tgtccgtggg   13620
aggtgctgtg gggcctcag atcccccaac gctgctgtgg ccatgggagg tgctgtgggc    13680
agggctgacc ccggggccca gctggttcct ccccgcctaa tgctagagaa gggaagtgct   13740
gagtgtttga gggggatcag caggggaagg gggtacagtc aggctcatgg ggtgaccagg   13800
agcagagacc ccaccccacc aaagcacaca cactggctgc gtctgagggg aaaggagccc   13860
agggaggtgg atgaggtggg caggggggtgc ccgtcgctgg cctggacgcc tagggggggca 13920
tcttactgtc tttggggctg gcaggctcca gaccccctaa gctcacggtg ccaaacgtga   13980
ccccccatac tgggcggcag cggctacaac tgtgaggggc aggcagtggc tgcctggacg   14040
gggacgtgct tgtcctttga cgaggagttt gcgcctcatt caagtcccct acctgcatcc   14100
ctggcggggc atctgcggtc cagcctggca gggaccctga gtttgggtct gtgggggtgt   14160
ggcctctggg ggagggcaaa ccccggtggg cactggtcac cggtgagccg gcgtcgtggc   14220
cacagccttc acactcccag ccgctgcagg tccccgtttc cgtacgtcac ccagcgccac   14280
gcttctgcac ccacgcagag ccacgcccca cctgtgcgtt catgctgact tattaccgac   14340
tcttctcgag tgaggagttc tcgccaaacg ccattaggct ttcagcttcc agatgaaatg   14400
aaatggctga tgaatcacta tatttcaaca tattaggaaa gatggaccta tttgcatccg   14460
atgagcaaaa tgcacttttt tattgattga aaagttgatg aggcaaacaa atcagggaaa   14520
atggctccct tttcatttcc ctctgagcag caaattaagc gacttaaaag agctttgaat   14580
gagtcaacaa caaaatgccc ctaaatgtgg aaatgtgaag tgcaaacatt tcagcctcac   14640
tgtcagtgcc caccgaggac acagcctgcc tcccgccctg ggaagctggg ggctgtggag   14700
gaccccggcc acttggacat ccagccacat cccaaagtct ccaaggggag atggggtccc   14760
cagcaagcct gtccggatta gccaagatga ggggcttcag gagtcaacac agatggagct   14820
gccaggcgtg gtggctcaag cctgccatcc taacactttg ggaggccaag gcgagaggat   14880
cacttgagcc caagagttca aggccagcct gggccacata gggagacctc cccatctct    14940
aaaaagattt ttaaaaatta gccaggtctg atggcacctg tagtcccagc tactcgggag   15000
gctgaggtgg gaggatcgct tgagcccagg cattagaggc tccagtgagc tatgatcaca   15060
ccactgcact ccagcgacag gtgagacccc gtctaaaaaa tatctcacaa caagagagag   15120
agagggaggg aggagcctgc accggctgaa ctatttcagc gtcctggggc tgctgtagtg   15180
caggcttaca acatcagaaa tttattctct cccagttctg gatgccacga gtccaaaatc   15240
acggtgtggg cagcacgcag cacactcctt ccggaggccc tggggaggat ccctccctct   15300
tccagctctg gggctccac gcccctgctg cggccgacgc tccagtccct gcctctgtcc    15360
tcacagggcc tgctctgtgc atctgtctct attctgctct gctcttcatg gaggaacacc   15420
agtgactgac ttgggactca gcctcaatcc agggtgacct cgtccccaga tccttaatta   15480
catctgcaaa gccctactac cagggaaggt tgcattctga ggttctgggc ggacatgaat   15540
tttgggggca ggacactgtc cagctcagtg ccgggcgccg ctgtgggcca ggccctttga   15600
ctcttgcaag cccacgatgg ctacagagct ccaaggagga ggcttcctgg aggaggcggc   15660
ccagctcatc agagaaccag catgtcttcc ccttgggttc tgggaccagc acttttaacg   15720
```

ataagtcctt ttggaccacg ctgatccggg ctgagacctg tcgcctgccg cgcgccttgg 15780
gcggaggtta gacaggccgc gcacctccaa gctttgacac gtccttcctc caaagggtgg 15840
aggccccaca gggtgagtgc aggttgggga tgggccgtgg ggttgccggg aggggaccag 15900
tgggggcttg cagtgttctc gggagggccc ggggctgggg gcaaccgaga cctgcttcgg 15960
tgggtgagtg gagaagcatc tggggggttt ccttcagcgc tggagagaaa tgggtgatga 16020
agcctgggaa aggcgtgggg ggcccgaagc gtgcaccacg gtgagaaggc tgcgcgtggt 16080
cggattccag ctcccccgcg tctgcgaaag gctgagctgc gccggccggg ggagggtcgg 16140
aggctgccgg gggttggggg atgaagagcc gggggcagtt tagggcagcg catcctccgc 16200
ggaggcagga acggcaggcg gtgcgattcc caccaggacc tctgccatcg gaaccgcggg 16260
tttctgccgg gccggaggcg cctgagagat ctccgcggaa cggcgcgtga acctgcggcc 16320
gcggcggcgg gtccggatgg agatggacgc tggatgggct gtttcctgag tttggttccc 16380
gtggcggggt cggcccctcg cccctgcatt tggcagacct gatgcggagc ccgagggtgg 16440
ctgttggcct ggggcgggga aggggctctc tagatcgggg gtcccacctg ctgggcttgg 16500
ggagcccatt tggccttctc tgattggtct ggagttgcag gtgaggccac ctcaggaagc 16560
tgctgtatct gagggagccc gggccgccct gggggacgct ggggtttgac ttctggggct 16620
gggggctgca cgggtgggtc ggggtctgtc tgggttgggg tccgcggccg cgtggggtct 16680
ggattggcgt ctgcctggct tgggggtcct cagccgcatg cagtttgggt cgaggtcggg 16740
ggtccgcggc cgcgtggggt ctgggtcggg gtctgcctgg cttgggggtc ctcagccgca 16800
tgcagttcgg gtcgaggtct gcagctgcgt ggggtccggc tctcatggcc cctgtgcctg 16860
gacttcgctc tccaaccatt tcctctgcac gtttccctgg aaaaggacat ggggttcagg 16920
gtggtggatg tggacacccc ctgaaaccca ccagccctgg tctgtgatgc ctggatggag 16980
gggccacagc cacgccggct cccagcctct tcccagacac cctagcccct ggaggcaggg 17040
tccccccgtg aggcgtatca tccctgagcg ccccacacgc ctgctcctgg gcatcctcct 17100
gggtggtggt gtggtgcggc tctgcgtccc cacccaaacc tcacatggaa atggagtctg 17160
cagtgtgacg gtggggcctg gtggggagtg actgggtagc aggggcggat ttctcgtgaa 17220
tggcttggcc ccgtccctcc tggtgctgtc ctcgagattg tgagtggcca tttaactgtg 17280
tcccgcacct gctctgtctt tctcgttccc gctcacgcca tgtgagacac ctgctcccgc 17340
ttcgccttcc accacggttg gaagcttcct ggggcctctc cagaagcaga aacacctgtg 17400
ctttccatat agcctacaaa gcgtgagcca atcaaactct tttctctata aattacccag 17460
tctcagggtt ttttttgttt gtttgtttgt ttgttttggt ggaggcgggg gtggacagcg 17520
tctctctctg tcgcccagga gggagtgcag tggggcgatc tcagctcaat gcaccctctg 17580
ctcctgggtt gaagtgattc tcctgcctca gcctcctgag tagctgggat tacagttatg 17640
tgccaccatg cctggctaat ttttatactt ttagtagaga cggggtttca ccatgttggc 17700
caggttggtc tcgagctctt aacctcatga tccccctacc cccaacgccc cgccttggcc 17760
tcccacagtg ttgggattac aggcgtgagc caccacgccc ggtcgtgggt attttctagc 17820
agtgcaagga cagaccaccg cacctggtga acgcccctga cggcagtaat gaggcacacc 17880
tgggggtgcc ccgtggcaga cacacctgaa ggtggttggg gttgccagcg agggaatctg 17940
ggaggagcca accggagacc caagtcctct ctatgagaaa catctgagcc ctggcctgtc 18000
ctgtggaacc cgggccatgc tggggattga ggtcctctgt tttgggttca acggaggctg 18060
cgggtggagg ttgttggggg agggtgctaa gtgaaaatgc tgtagggagg gcgtgcttct 18120
ggcgggggtt gtggttttgt ggtggtcgtg gtttttgttg tggttgcgat ttttgcggtg 18180
gttgtgactg tgggttttgc ggtggttgtg gtttttgaga tggttgtggt tttgcagtgg 18240
gtgtagttct cccaccccac ctgctgccgc tggaccctct cccctgtgtg tagcccccgc 18300
taggacccca tgtctggatc ctggctctgg gtctcttctc tggcctcgtg agactggcgc 18360
catctccact ggagccaata ggcgttcggc acagctaccc ccagctccct ctgtctctgg 18420
acgagttggt ggccgctccc acgcaggaat gccgacagcc tttggcatca cacagggtca 18480
ggatcctggc tgtgacccct cccccagtga cttcctgcgg gactgatggc cagagagggg 18540
gcctgtcggc cctgcagtgc ccctcccacc atcagccaca ctgagttctg ggcaggagtc 18600
ccctggcctt ggtcccaccc accccaggag gatccagtga gtccagtctc cacctgggac 18660
accgcagagg tcaagcccac cactgtgagg ctcagtgggt gccgggagcc atggcaactc 18720
cgcagggatg gtcctcggtc ccctctccag ggaccccacc ctagctgtga cctgtcccac 18780
ggcacctgta ccgtgacacc ctccttgctg tccatgtggc tatgagggct gccctttgca 18840
cgtccattcc cgagccccag ccctaaagca gtgctgggct caccaggccc ctctgtggac 18900
gccgcctctg tctgtgcggt gggatgttaa tagtctccgc gtcatgtggc tgttaatttt 18960
ggaagctcgc ccccttcctc ctcccccgc tatggtcctg ccttcacacc ccgctgcctg 19020
agtcagcgcc cacggctgca ccagatgcta ggtttggccc cagctgaggg gctggtgtcg 19080
cgcacgcagg caggggggcct ggtaatgggg ctataccatc tgcatgaacg gctttgctac 19140
ttccccctgc cctgcccagc acaggctgag tccacagccc agacagcagt gccccgagag 19200
ctgcctggca caccccggga agaggatgaa tcttgccgta tggggaggct ttggccccga 19260
gagctgcctg gcacatcccg ggaagagggt gaatcttgcc gtatggggag cctttggcag 19320

```
gagtggggct gcctggtgac cgcgtgaagc acctcccggg atgaaccagg gccctgccaa   19380
agctgaggct gggccgggac tcctggggcc tcattgtctg ggatgaggcg ggtcccagct   19440
gggctgcggc aggggccagg aggtacctgc atctgccctg atctccctcc tcacacctgc   19500
agagcccctc cactcactga aaagcagggt gtcctgggtg ggccccggcc tgagcatcca   19560
gagactcagc tccaagccaa gccctttacc ctctctgagc ctcagtttcc ccatcttcaa   19620
atgaagatgc ccaaggagag caggccccgc cccccagctg gacctatcag agtgcacaga   19680
ggtgcccagg agagcaggcc ccgcccccca gctggaccta tcagagtgca cagaggtgcc   19740
taggcgagca ggccccgccc cccagctggg cctatcagag tgctgctgca ggcctgaggc   19800
ctcacctggg tggtacaacc tgctggcgga ggaaagagct gccgtagccc aggtgggact   19860
gggggggtccc catgatcccc caggagggtg ggggtcttgt gtgggagtct ctcaccagct   19920
ccagacgcgg tttccagggc atattcactc aaattcctca tcctcacctt cccccaagtg   19980
caggcccggc agtggcttct acctggggct gggagggggc aggtctttgg tgctcatggg   20040
acgtcactcc acactgggca ctggtccagg tgccagggga cagagtgggc accccagcgg   20100
gttgtgtggg tgagggcctc agcactgccc ccgcctccca tcctgaaggc tggagtagcc   20160
tgggaagctg cattttaatg agcaccccat tttaacagag tgcccactga agctcgagac   20220
cacggccagg agggtcagcc agggccccag gggcgggact caggcccctg cagaaggccc   20280
catccttgaa accctttgag ggtctccctg tttctgtctg gaccccgggc cacgctctgc   20340
ccttggcagc tgcagcctgc atctcactcc agacgccttg gtgggaagcc cctgctgccg   20400
ggtccccagg ctggaatccg caggatgtca gggcaccctg tgctcttggg gacgctctgc   20460
ggtagctccc tgaggctctc agacaccagg acaccctccc agcccagtcc cagcaacaca   20520
tgccggcagc gcctgcgcca gtgttttctt ctcctgtcgg gaggctggtg acccgctgtg   20580
ctgccccggt ggggggctga tctctgacct cctgtctggc gggctgtgct gcaccagcca   20640
gggccgggag gtgcacgtgg acccgtgttt attggaatcc tgctgtgatc agacccgggg   20700
gcgacgtcga gacccaccag gccccgtggc ctcctgtgaa catctgcagg ctgttggggg   20760
cagcggtcga cggaacaagt gtggggcggc ccgggcttcc cggaggaagc gttgcttgag   20820
ctgccagggg agttacctgc agaaagggag gtggggtgca ccaggcaggg gggcagccag   20880
ggcaaacgcc tgatgctcag agggtgacag tgggcagcgg ggccctttgg aggagcggag   20940
ggagggccct gttggctgca gggcagagtg ccccacactc ctcctggccg ccacccccag   21000
ggcccagccc agtctgggga ctaagacgag aggcctctgg ggtgttccag gtgagctgga   21060
gtcagcccct gttgcttttg aataattaca ggggagtcag cccagtgacc aagcctctgc   21120
ctggtggggg aggggcacgg gctttgtgtc cgggagctgc tgtccctcagc ctcaggagag   21180
ggggtgtcac gggccgtggg acaggtcaca gttagggtgg ggtccctgga gaggggtctg   21240
aggaccgtcc ctgcggagtc catccagcca ccatcggttg ccatggctac tggcagcccc   21300
taatcctcac ggtgctgggt ttgacctctg cagcgtccca ggcgggcatg acctcatttg   21360
atcctcctgg ggtgggtggg actgaggcca ggggactcct gcccagaact cagggtggcc   21420
gatgtgggga ggggcactgt ggggccgaca ggtcccctct ctggccatca ggcccccagg   21480
agggcccagg tgcttggagg tggggggtgt cccagcttga tgttggggat gtgagtagat   21540
ccagcgccca cccacccgca gggctgagct cccactgggc gctgggccgt gactcgcaca   21600
ccggaccctg cacccctggg gcacccagtg attccagaac agtgggaccc cagaggcttc   21660
tcagggagcc ccagaacatg acagatgggg gcccaggtgc gaggcagtgg gagctggagg   21720
aacacagcca ggggctgagg cgagaaccat gaggatggtg ggcagaggct gaggggaggg   21780
gcagcagatg gagatggggg aggcgttagg ggtcacaaag ggggttctca gaggatctgc   21840
cttgggaagc cactgctgtg ggagggtggg agggatacag cccaggtacc tccagagagg   21900
atggaaaggc ttatggggcg gattctgggc aggggacctg ggaccagcct gcagcctccc   21960
ctgagggcct ggccatggcc gcaggctggg gaggaggggga ggggaccttg gggccaggtg   22020
ccatggtcct ctaagcactc ttgctcgatg gtaaacataa cacgcgctgc ttgcagaaca   22080
tgtggaaaca ctgaaacgct taaaggagag aacatggtcc cggtacccgg ccttcaaggg   22140
agctgctggc gcctgttcaa caggagccga ccccacctgt gaacagacgg caggcggctg   22200
ctcccatgtc ccctcagaga ggggccctcc gagttctgct gtcacctggg ggcgcctcac   22260
ttcattcagc cagtggcggg gctccctggg tcggcacaga gtcaggggag ggggctgggg   22320
gacctcctgg tgggaaaatg tccggtgatc cccagcctcc gcgctcagcg ggaggaggcg   22380
ctcggtcccg cttcttacaa ccagcggcgc tcacggcggg cccggggatc agcatcccgg   22440
gagcttctca ggaatgcaga ttcccaggcc ctcactgcct ggggagtcg              22489
```

<210> 35
<211> 4419
<212> DNA
<213> Homo Sapiens

<400> 35

```
tgtgccgtgc gggtagggag cagccccttc cggtcgtcga ggcgctctta atttggttgg      60
agagacaagt gcactacaag aaacagaggc agccaagcgc ctgggccacc atcaagtaag     120
gttgaaaagg ggagggcagg tagatcggga cccgtgggcg ccaatcaatg ctatggtggc     180
ggagagtaaa ggggacgaac acagcctggg gccatccccg gagtccccca gcgtccgcct     240
gtggccgtgc ctggttggcc gcggatcccg gcgggcgccg caaagcggcg ggattgccag     300
cgcagagctc cggctctctg ccttgtccct gggtccgagc accggagcct ctggtgtctg     360
cggggagaag tctcggattg agaaatacgg gagggtctcg tcagtggctg caggtgcggc     420
agccactctg gggacccagt gagaatgggg tcgcctggct ctgcgcgaac ccctcacgtg     480
ggtgcagctc ctgagccgcc gagggaggcg gtggtaatgt cgcccagtgc cagcagaggg     540
cagcctcgag gccgtgagcc cgaacggcga cctcgccaaa ccgcgggctc ccttccagct     600
cccggattct gcggggtaga ggcggccttg gagcccagag accagcgacc tcagtctgca     660
ggagcccggc gcagaggccc aagggcaccc ccgggatgtg gtcaagctac agcccttagg     720
cagcctcact ctgcgacggc aagggcttag agggtggagg gggaccagat gccttaggag     780
gggttagaaa gccaacgcac acagggaatt tgttttcaag caacagattc caagcatgtg     840
gaaacctttt aaatgatgct ggtgagagct atgatagttt tcgcattctt ggcgagggaa     900
gtcggaggct agtagcggga tggctcctgg gcgcgcggga gacagaggga ccagcgatcc     960
ttgctggggg cagagggact gtggaccaag gatccagaca cacttaattg gggattccag    1020
tcccgactgt ggccactcaa ctggcgttga acttgagcaa ctcactatac cgctctagcc    1080
tccgactaat cacttctgta aaatgggcat tgtgggctcc gagtctcgta agggcgctag    1140
ggattcgaga cagcaggaat ttcctcactg ctccagcaac cctgggttcc tgggtttgta    1200
ggtgggctga aaggatcctt tcatcgcacg tttgcctggc gtggctgctt tagagaccga    1260
ggcccgccag gctttgcaac ccagttgcgc gtggtcagcc atggccttgg aaccgccggg    1320
atcgcctcag cggcactccg gccagctccc gacttcccgc cctgggcact agggtcaccc    1380
agctccagaa gatagtgctt atcagcacag gaattgagac cccacacccg gcgcagtggg    1440
tctcccagga agcctggcga agagccaagg cccgccggac ctgaggtcgc ctggggcgct    1500
aaacagaccc atctatgagc acattcggcc aacacaccca agttcaacag tgggcgggat    1560
ctttggctgg cctcgatctc tctcacagtg tgtgcaaacg ggtacccatt agattctttt    1620
tgcttgtttc ctcatctgta aactttgaaa ggagagtatt tcccggggtg attctgaaaa    1680
tcatgccaga actgaaaagg cctgtgtaaa tgcgaggtgt aatgatgact tattagaagc    1740
agccagccct gtacgtgcct gtaggataga tgctagaacg ttctacaaat gtgtgcacat    1800
acacatcacg tatgtgtgaa ggtgcacacc tgtccctaga cacttgacaa atgcacacac    1860
gtatgcgtgt acctctggct gagagcaaag ggctaatac aggctcata caggacatca     1920
tcctgcagtc ttgtgcgtgc acagtacaca cgcgagcacg actgtctgtg cctggagaca    1980
ccgtatggca ccggaggagc tgctcagccg tcggtgccac ctgggaagta gggtttcggg    2040
tgctgctctg gagcgcggga aagtccggat ccgggtctgc tggtcagcgc ttcggcgccg    2100
ccgggctcct ttatctctca tccgccggcc ggtgagcggc tccagtttca agaccccga     2160
tcctgccggt gaggggaggg agcgagaagg agtgcgctcc gggcccaaga ggctgcaggg    2220
ccccactccc caggtctgtc gtccttcctt tatctcctca ggtcacaccc ccacttaaaa    2280
taaagaaggg tgcctgactc attcaggtca gggaagtcgg cccccgggag cctcctgccc    2340
tcacaagcca tggtgaaggg aggcgaagcc agggtttgcc acggcctggg agaaaatgcg    2400
gctgcagggt ctctttgggc tgcagcgctg gcccggggcc ccaagactgt taaggtgtgt    2460
gtgggaggcg cgcagtgtgg ctaccgaaga gccctccatc accgcaccgg caggccgccg    2520
ggctcgtcct cctctcatgc ttccaagggt tctgagctgc gcagcactcg catcacccag    2580
aagtgtctgt ggagaaaacg acctcaggtg taagcccaag gctgctgcct gcaaaggcaa    2640
tgccgtggag actgggttcc acagcgacct gggtttttaa gtcaccgaaa gctacagggc    2700
agggtcacaa acactctccc gccttctctg cagaaccgtg cggctgacag gagagcgttg    2760
cgcagaaaat tcgaggccgg gcgctggagg agtctccgcg gcccggagaa ggcacagagg    2820
cgcccctgag aggcgcagct ggaacaggcg atgcacgggt tcggatctgg ccggccaacc    2880
gagcccagcg gtcgcgaaaa ccagagtcgc cacagaggtt gagcacgatt ccatttctgg    2940
ggatcgggtc cggtggggag ccactgtccc taacgcctcc agagacttta agtaggacaa    3000
tataatgctg gtaggagcaa ggtgcaagga atttagcggc agaagccttt cggggggtg     3060
gggaaaggca gatccgtgcc tcgtctgagc ctgggggtgg ggacagcccc tggccttgta    3120
gcccctgttc cggggatcag ctgggcccac ctaacccccc tacatggggt tgaaaggggc    3180
caatgggacg gccccgccgc ccctccctcg ggattccaca gggccctgac ccggccttta    3240
tctctgcacg gtcagcagtc gcgggggctt caggaaggag gacaaaggcc cggtgtcacc    3300
gcgggcgggg gctcggtttc ctggctctct gctcacactc acagccttta gcggttgttg    3360
ggggaagatt tttaaaaata tgtgtcgaat ttcctttttt tctcttctag aaacaaacaa    3420
acaaaaaagg caaaaggcga attccccttc actcctcgtc catagagatt aaagtttcct    3480
gggatcctgc cctttttttt tctttgactg cctagaaata cttgttctcc cttgtacata    3540
gaggaaaatg cggggaaagg tttttaaaa ctcggtttca tactattatt attactaagg    3600
acaaccgggc aggctgaggt cccaacgtgg atgatccgag ttggcctcgc gccggggctc    3660
```

tgcagccact gccctgtgcg ctcagcacct ctgggggcga tcagggcccc tgcgcttccg 3720
cccgccgccc ggcagtcgag agcaccctgt gcccagactg gccgactcat tctcccccga 3780
attttgttta gagctggcaa gggggactta gctcgcgccc caagacctgg gcttgcagcg 3840
ccgccaacag gcccggggac acgaggcgct ccaggccggg gtcttcccgg ctgctggccc 3900
ctctcgctcc ccacccgctg gcggcgcctc ggtcgcccgc aattgaccca acccgcttcc 3960
tgcgtttgcc cctcaggttt cccgtttctc cacaaaggcc taggggagcc tcgcccacag 4020
gctgaccctg caacctctgg cccggtggct accttctgct tttctgaaaa agaaaaggaa 4080
aaaaaaaaaa aaaaagaaaa aatcaaccag tcgagggagg cgcgtgagga ctggaggcgc 4140
cagccggaca gcctatgagt aggtccctgg gtcggtgccg cttcgcgggt cagcacggcc 4200
tttctcagag aaaacctcct aaacgtgtga agaccgcttt gggggaagcg agagggaggt 4260
tggaggagcc ccgggcgggg tctcagcgcc caccagctgt gccttcaggg cttgggtgtt 4320
cgctgcaacg gcaaccgcgt gagcctcact cccacggcca aggggctagg gcagggtgga 4380
tgcaatcgcg tgcgcctggc cccggaaggt gctcgcagg 4419

<210> 36
<211> 1918
<212> DNA
<213> Homo Sapiens

<400> 36

catcggacat gattaccgta cacaatacag aagatactaa gggctaagca atgatgcata 60
aacggggtcc actgcccaaa cttacagaaa gcaaaacgtg gaaaattagt aactttaact 120
gatagtgtac acctaaagaa cctacaccgg ccgggcgcgg tggctcacgc ctgtaatccc 180
agcactttgg gaggccgagg cgggaggatc atttgaggtc aagagatcga gaccagcctg 240
accaacatgg tgaaaccccg tccctactga aaacacaaaa aaattagcca ggcgtggtgg 300
cacatgcctg tagtttcagc tactcgggag ggtgaggcag gagaactgct tgaacccagg 360
agacagaggt tgcagtgagc cgagatcgcg ccactgcact cgtctgggag acagagcgag 420
actccgtctc aaaacaaaaa acaaacaaaa agaacccacc caacagaatt aagtaccaac 480
ataataaata cgaagaattt tagattcttg gtttttaaaa aacatacaaa gatgatattc 540
cttcaaaata tctttacaaa acatattgag actgtgatgc tttatattga ttgtatgaaa 600
acaatgaaaa agaaccagca ctgtttcact ataaaagctt tactaatgta aatttataaa 660
tcctttctta aatattttga gttaattcta attttatgat agaaattcat tattttcagc 720
aaaaacagct ggcatttggg aaaccaaagg ctcaaaaact aagaatagta accaaagaaa 780
cttgacaaaa cagtcctttt aaaactctca tctacactat aaggggaaac tttgatcacg 840
tcccttctcc ttcatcaatc gtagaactca acattaagga ctacacaatc ccacatccct 900
ctccgagaaa aagcaaaggc tttgtgttgt agcaacaacg caagacatgg agggaagctc 960
cactcaagac ttccctgcct gctccttccc caaagccact ccagaatacc agggaggggtt 1020
gagaggtaag gcatgaaggg cgcaatatcc aatatgagca acgcgtgtga tgcatctggt 1080
caaaatgcat acagaggact tgtctctgtc cctagataga agtcctccgt cctgcagtca 1140
tgagggtcaa ttgctgaggc ttcacagttc ccttctctct tacactcgga ccgtcacgct 1200
cctcacctac taccccgatg cagaggtaga ctcaggatcc ctgcacttgt caaggattcc 1260
tcggcaagct cacggggcgg gagtggccac aagacggagc tcgcctggtc ctggccttcc 1320
cggcctatac aagcctgccc ccttcccaat tcccaatctc cacagccttc catcctccca 1380
ctttcgattc accttgcgcc accgacgccc ctggccttcg tttgcagcaa gtttaccccc 1440
accattacct ctcgcataaa agcctgcatt taccaggtca aagaggggaa ccaacgcctg 1500
caggaatcgc ttcaccgaat cgcctggccg cgtcctctgc tagacttcac ctgccgctgc 1560
ggaccgtaca caaccactcc cggcatgccc cgcgcacgca ctacctctcc caccccgccc 1620
ctctcccgcc caaacacgtg acctcctttc gtctccgtcc acgcccactt ccgttcctcc 1680
actttccctt aggaaggagg agggagctgg gggtgttaaa agcgtagcga cttcctcctc 1740
ctccccgccc ccgctcctgt acctcccgct acaatgtctt ccgggtcgct agcgcctcga 1800
cgccttctgg gaaaatagct catttcctcc cctccccctc ctcctgcctt caaccaacca 1860
gccacccgtc agagagggac atgcgcagtg agtgcctccc gtctcttcta cccgaacc 1918

<210> 37
<211> 13670
<212> DNA
<213> Homo Sapiens

<400> 37

```
agtgctggga ttacacgagt gagccactac gcccggcctg ggtttttgtt tgtttgtttt 60
taagacacgg tcttgctctg tctccaggct ggagtgcagt ggcatgatca cagctcattg 120
tagtcttgac ctcacaagct caagcgatcc tcccatctca gcctcctgag gagctaggac 180
tacaggcaca cgcctccatg cccagcagtt aaattttttt tttttttttt aagagacaag 240
gggtcttgca atgtttccaa ggctggtctc aaaactcttg gtctgccgtg atcctccttc 300
ctcggcctcc caaagtgctg ggattgaagg catgaaccac cacgcttgtc tctcagcagc 360
ttgtgacgcc aggaactaca tccctattcc ttgacgctcc tccctcctcc attgctgggg 420
catcactttt gcctggttca cctcctacct tttttttttt tttttttttt gagatggagt 480
ctagctctgt catccaggct gtagtgcagt ggtgtgatct tggctcacta taacctccac 540
ctcccgggtt caagcgattc tcctgcctca gcctcccgag tagctgggat tacaggcgcc 600
caccaccacg cctggctaat atttgtattt ttagtagaga cggggtttcg ctgtgctgga 660
caggcttgtc tcaaactcct gacttcgtgt tcctcccgcc tcggcctccc aaagtgctgg 720
gattacaggt gtgagccact gtgcctagac tttttttttg agatggagtc tccctctctc 780
gcccaggctg gagtgcagtg gcatgatctt ggctcactgc aacctctgcc tcccgggttc 840
aagtgattct cctgcctcag cctcccgagt agctgggact acaggcatgt gccaccatgc 900
ccggctaatt ttttgtaatt ttggtagagg tggggtttca ccatgttagc caggacggtc 960
tcgatctcct gaccttgtga tctgcccgcc tcggcctccc aaagtgctgg gattacaggc 1020
gtgagccacc gtgcctggcc tcacctccta cctttgtggc ctctctcctc tgcctgtctc 1080
tgaggtgttg ggggtcctca gggttcagtc tccagtcttg ttctccattc tgctctaggg 1140
agacttcacc ctcgctccca gcttaagagc ctctctgccc cacaatacac cgatgcttgc 1200
tgggctattt ttgtctttag cccataacat gcttctaagt ccctaccccg ggatccagcg 1260
ccgtcagact gaatatatcc caaacctgcc tctgtacctt ctcagcgtat ctgctcctct 1320
tccagtgcct ggacttgagt ctcgttctcc actgcctgga ccacgcccat cctcctctga 1380
ggccttcccg tcctctggtc cggccttctc accatccccc aagtaacatt ccagacacac 1440
ggatctgtcc gagaccctcc cctgctggca tgtgaagcct tttatagtct cacgtcttcc 1500
tagcccaaac tttcctccca gtctcacgga aatgctaaat catcccggcg tgggaggctt 1560
ctgtgggtcg gggcgcagct ttgcaaggtt cagctccaag actgcttcct ccagggagcc 1620
ttccctccct tcccccaggt aagtgctcct ccccagttgc tcattccacc agggcctgat 1680
cacaccctgc tggcagtgtc agagcccggc tttgtcttcc accacccctc ctagaactgg 1740
tggcagggac agatgaatgg atgaatgaat gaatgggaag tgagaattct actactggac 1800
catcaatgca gccctcggaa tgagtgagtg agtgagtgag tgagtgagtg agtgatggaa 1860
agtggtctgg aggcagggag agggacaggc cgatcatgcg aggaatccca gcccaagccc 1920
ccgaggtccc cgctcccgcc ctaaaagatg cacgcgccaa gcccttgggc cagaaaaaaa 1980
aaaaaaaaac gggccgggat tggctcaggc tgggggaggc gggtctacga cgagacacga 2040
ttttctattg gcttactcgc caggactctg cccaatcgac gcgatcgtct tccccagaag 2100
acccccgcct ccctcccgcc ggcggccgcg gaggcggaag aaggcgcctt tccgtcgccc 2160
tagcaaccgt cagcggatct ccgtcgcccg ggcaactgtc ccaggcctcc cgtcagcctt 2220
tgcctgggcc cctccggtca ctcggacaac cgctacccgt tccggtcaac catcaacccg 2280
tcccccgtca ccccggcaac catcgccgga cccacctacc cctggacgcc gtcctggtcc 2340
tgtccgcagg cctcctcgcc ccaaacctcc gccgcgggct ccgacgtccg cgaccccacg 2400
tctcaggggc tccctcctcc tcctgcccgt cttctcgcct ccccggggcg gggggcattg 2460
ctcctcccct ggcagggggg ccccgggttg gacccggctc tgggggtccc tgctcggtcg 2520
ggacagtggg accgccctgt gctgtggccg cgcgcagccc cccgcaggct tcggcgctcg 2580
cactcttgcg tgggactgtc caaccgccac agggagctct tcaaaatgca agccatgtcc 2640
cttccctgct gaaaaccctc ccctggctcc ccttgagaat cgaatccaag ctcctcgcag 2700
cccctggcga tctggctgtg tggcctcttc tctgaagcct cctcgctcac tctgccttag 2760
caaggccctg gcctgcgtgt tgtaactcca atgacccaag cttcttcccg cctgggacct 2820
tcctagtcac tacttcccac tccaccaaaa agtgcatcct agacgggcgc ggtggctcat 2880
gcctggaatc ccagcacttt gggaggccga ggtgggagga tcacttgagc cgaggagttt 2940
gagaccactc tgggcagcat agtgagacgt ctgtctctat aaaaaatttt tgggaggccg 3000
aggcaggcgg actgcttgag gtcaggcgtt ggagatcagc ctggccaaca tggtgaaacc 3060
ccgtctctgc taaaattaca aaaattagcc aggtgtggtg gcgtgtgtct gtaatcctag 3120
ctactgggga ggctgaggca ggagaatcac ttgaacccag gaggtggaag ttgcagtgag 3180
ctgagatggc gccactgcac tcccgcctgg gcaatagagc aagcctccat tctcaaaaaa 3240
aaaattaaaa attagcctgg catggtggtg tgcccctata gtcccagcta ctcaggaagc 3300
tgaagcagga ggattgattg agcccaggag gtagaggctg ctgtgagctt acgtcacacc 3360
actgcactcc agcctgggca acagagtgag accctgtctc aaaacaacaa caaataagtg 3420
catcctctag ctagggtccc acctgtgagt cttcagtgaa gccttccctg agccctgtca 3480
gctctctcct ccttaaactc tttctggctc acactctgtc actaggacct agaacactgc 3540
ctggtatcta tgttgagtaa aaagatgttg tgccaggcgc agtggctcac gcctgtagtc 3600
ccagcacttt gggaggccga ggtgggcgga taatgaggtc aggaattcga gaccagcctg 3660
```

```
gccaacatgg tgaaacccca tctctactaa aaatacaaaa atgggccagg tgcggtggct    3720
cacgcctgta gtcccagcac tttgggaggc cgagacgggt gaatcatgag gtcaggagat    3780
cgagaccatc ctggctaaca cggtgaaacc ctgtctctac taaaaataca aaaaattagc    3840
cgggcgtggt ggtgggcgcc tgtagtccca gctactcggg aggctgaggc aggaaaatgg    3900
tgtgaacccg ggaggcggag cttgcagtga gccgagatcg caccactgca ctccatcctg    3960
ggacagagcg agactccgtc tcaaaaaaaa aaaaaaaaaa aaaaaataca aaaatgagct    4020
gggtgtggtg gcggacacct gtaatcccag ctgttcagga ggcagagaca ggagaatcgc    4080
ttgagcctgg gagggggctg cagtgagccg agatcgcgca attgcactcc agcctgggcg    4140
acagagcaag actccatctc acaaacaaac aaacaaaaaa aggatattgt gctggggata    4200
agggaggaca gagggggatg accagcaggc aggcctatct cagagacagg agaatgtatt    4260
tctcagggcc tctcagtcct catggtacag gtgatgatgg caacatcccc aaaaagccag    4320
gcacaagcca gggagataca agctgatgct acggtcagcc tgagactggg tggcgcaccc    4380
tccacatata accgccaccc cccacccatc cccaccccag agggctccag agtcagcgga    4440
aactgtggct aatttggagc agaagtttcc aggagctgga gaggcaggcc tcagatccca    4500
gatggctttt ctgggagctg gtcccttcct gcccggaatc cagaatctca cttgtcctaa    4560
cagagacgag agctgttttc ccagggatct gagacaggcc agcccaggct ggggcaagag    4620
aacttgacct gaccaggcct ggggtcatga cggggggtga gggaaggggc ttgtgggagc    4680
tctcctggga ctggctggag aggaatgagg aatctgcaca cccacctgcc tgctcatgag    4740
ggtctgccct tggggaggtt gccaggggcc aggcagggag gccccctgct cttttttttt    4800
ttttttgaga tggagtctcg ctctgttgcc aggctagagt gcagtggcgc gatctcagct    4860
caccgcaagc tccacttccc gggttcatgc cattctcctg cctcagcctc ctgagtagct    4920
aggactacag gcgcccacca ccacgcccgg ctaatttttt gtatttttag tagagacagc    4980
atttcaccag gttagccagg atggtctcga tctcctgacc tcgtgatccg cccacctcag    5040
cctcccaaag tgctgggatt acaggtttga gccactgtgg tcggccctga cccctactct    5100
taagctagga ttaccctcat cagcgctgcc acacacaact ttcttcactg atgcggcaat    5160
tgagcacttg aaatacagct ggtatggcca ggcacagtgg ctcacgcctg taatcccagc    5220
actgtgggag gccaaggtgg gcagatcact tgaggtcagc agttcgagac cagcccggcc    5280
aacatggtga aaccccatct ctactgaaaa tactaaaatt agtcaggcct ggtgacgctc    5340
ctgtagtccc agctactcgg gaagctgagg cacaagaatc gcttgaaccc ggcaggtgga    5400
ggttgcagtg agcctagatt gcgtccactg cactccatcc agcctgggcg acacagcaag    5460
actctgtctc aaggggaaaa aaatgaaata cagctggtgg gactgagcac tgcattttca    5520
ttgtatttca tttcaattaa catacattta agctgaagta gccaaatgtg gtcagcagta    5580
ttgtattcag agtagcactg agctgaagac ccaggcagga ggtcgaaggc agggacagga    5640
aggtcagggt ttaaggtctg tgcagctatg gcagagaagc gactttggca aagcgcagag    5700
tgaggcccca ggaagggagg aggtgggaca ctcggtctcg gcatctggct ccactggaag    5760
agaggtatac ccagttccca gggtcaaagt gtttgttaca cacacatgca tccatgcctg    5820
tgtgtgagta taggggttgg tgtgtcccga ggcacccata ggtgcgcttg tgtgggatcc    5880
aggagtccca tgcctatgag cgattcaaga gttcacccat gtatggacat tgtatggact    5940
cccaggcaca cacagataat gggtgtgtgt tccttcctga gtatccatgc ctcctgggca    6000
ggccagcaag gaccactagt ccagcaggtc ttgcaagaag gcccacatgt actggtgcat    6060
ctcctggggg ttgctctgtg ggatccaatg ccctatgcct ggcaggatgt gggcctccaa    6120
gcggcccggc acaaagcggc tgccgatggc ttccaccagc cccagctcca agtaagtgtc    6180
cttctccccc cacagcagca atgtgggtgt ggtcagctcc tggggttcca gggggaagtt    6240
cctgtggcca ggacagacag acaggcagac agacaggcag aaggatggtt gcctcccacc    6300
cccctgcccc tgcaaatctc ctgagcttat gcttggcaac tggtctcacc tgaagaggtt    6360
tcggtagtag ttgaggggcc cagtgaggcc accaggctgt gagaagttat aaaggaaggc    6420
ctcgagctcg ctgggggtca agcatgggat gcctgtcttg cggtgggtga gggtggtctt    6480
cagaatctag gtacacagca ggactctggc ttcagtgccc agcctgccca gccccgacct    6540
gcttccctcc tcccttcaac ctgcaccctc tgtacctgaa agtcagacat agacagcagc    6600
ttctcgggca gccagggcag ctggaacagg aacatgtagt gggaacggaa gaactggctg    6660
atgtggtgca gggaatagtc tggggtggga gggttggggg agagatataa ggcctgctcc    6720
tggggtggcc ccatacacct gcacccctac acatacccag gtcagggaca caagcctcag    6780
gaatgccact gctgcctgcc tgggtctgtg aaggtactag atacccctcc tggcctgggc    6840
accagctaca ttccctcctg ccatgtttca ctatccacag gatgggggca tctcctggga    6900
acccccatat ccaaaggtgt gcttcctctg cccaggccag ggtgcgtttc ctctgcttta    6960
ttcagctaac atttattgag caactactgc atgccaggcc ttgttctaga cacagcagtg    7020
aatgagacag tcaaaactcc ctgttcatcc taggcaactt aaggaaaccc ctgtatctat    7080
aatttttttt tttttttttt tttttttttt tttttgagac agagtctcgc tctgtcgccc    7140
aggctggagt gcagtggtgt gatcttggct cactgcgagc tccgcctccc gggttcacgc    7200
cattctcctg cctcagcctc ccggtagctg ggactacagg caccccgccac cacgtccggc    7260
taattttttt tgtattttta gtagagacag ggtttcacag tgttagccag gatggtctcg    7320
```

```
atctccggac ctcatgatcc acccgcctca gcctcccaaa gttctgggat tacagacgtg    7380
aaccaccgca cccggcctcc aaaaaataat tttttaaaaa ttagcagggt gtggtggtgt    7440
gtgcctatag tcccagctac ttgagaagca gaagtgggag gatcgcttga gcccaggagt    7500
ctgaggctgc agtgagctgc aattacagca ctgcactcca gcctggacca cagaacaaga    7560
ctgtctcaaa ggaaaaaaaa caaaacacaa aaaaagacac ccccaaaaga caaaaaaactc   7620
cctgccctca gggagctgac agtctagatg gggagacaga caagtaaaca ataataggct    7680
gggtgcggtg gctcacgcct gtaatcccag cactttggga agccaaggcg ggtggatcac    7740
ttgagatcag gagttagaca ctaacctggg caacatagtg aaaacccgtc tccactaaaa    7800
atacaaaaat tagctgggcg tgttggggca tatctgcagt ccccgctact ctggaggctg    7860
aggaaggaga gtcacttgaa cccaggaggt agaggttgcc aggcagggtg gctcactcct    7920
gtaatcccag cactttggga ggctgaggcc ggcagatcgc ttgagcccag gagttcaaga    7980
ccagcctggc caacatgctg aaaccccatt tctactaaaa atacaaaata attagccggt    8040
catggtggca cacatctgta gtctcagcta ctgaggaggc tgaggcaaga gaatcacttg    8100
aacctgagag gcggaggttg ccgtgagccg acattgcgcc actgcactgc acagagtgag    8160
actccatctc aagaaaaaaa aaaaaaaaag gggccaggcg tggtggctca cgcctatact    8220
cccagcactt tgagaggcca aggtgggcag atcacaaggt caggagatca agaccatctt    8280
ggccaacatg gtgaaacccc gtctctacta aaatacaaaa aattagcccg gcatggtggc    8340
ctgtaatccc agctacttgg gaggctgagg caggggaatc gcttgaacct gggaggcgga    8400
ggttgcagtg aactgagatc acgccactgc actccagcct ggcgacagag caagactctg    8460
tctcaaaaaa aaaaaaaaaa aaaaaaaaaa gaaaagaaaa gaaaaagaaa aaaaacaaca    8520
aaagagagag agagagcaca gttatgacca cagtgctgca tattaacatc aggtgatatg    8580
atagacattg ttaacctccc cagcctcagt ttcctccagc tggaacatgg ggataataca    8640
accttcataa gcccaggagc cttaagtagg ttactgtgtg catattcctt gttactgtaa    8700
gtgctataaa aaaatgtgga ttactcctaa gtgtattgtg atttatttta ttttatttta    8760
tttttgagac agagtctcac tctgtcatcc aggctggagt gcagtggtgc gatcttggct    8820
tactgcaacc tccacttctc aggttcaagt gattctcttg cctcagcctc ccgagtagct    8880
gagactacaa gcgtgcacta ccatgcctgg ctgatttttt tgtattttta gtagagacgg    8940
ggtttcacca tgttggccag gctggtcttg aacccctggg ctcaagcaat ccaccggctt    9000
tggcctccca aagtgcaggg attacaggca ggagccacca cgcctggcca tactttattt    9060
tatttattta ttttttgaga tggagttttg ctcttgttgc ctaggctgga gtgcaatggc    9120
atgatctcag ctcactgcaa cctccacctc ccaggttcaa gagattctcc tgcatcagcc    9180
tcccgagtag ctgggattac aggcatgcac caccaatgcc tggctaatta catttttttt    9240
tttttttgt agagacagtt ggggtcttgc tatgttgacc agactggtct tgaactcctg    9300
gcctcaagtg atcctcccac ctcagcctcc cggagggctg ggattacagg tgtgagccac    9360
tgggtctggc gtgtcattaa tttccgaatg aggagttcca caaccgaagg gacctgggtt    9420
gattgctgca ccctcagccc ctgatgtgtt gacaggtata cagagaattg aaaacacaga    9480
cacatgtccc tgccccccttg cacataagta catcccagtg tcccggactc ccacacacac  9540
cttggtacac cgacatgggg gcaccactga ccacaaccat ccgctcgacc agggatgggt    9600
agtagatgga gaaatgccag gcaaggaggg caccccagtc atgggccaca aggatgcact    9660
tcgagtaacc tgtgggaatt caaggagctc gcataagtga ggtgcccagt aggactgaag    9720
cagggatggg ggaagtaggt tccagcaagg agaaagcggg ggtatgcgtg tgtgcaggat    9780
gagggaggcc acgcctagga tgggggtatg tctgcaccca ggcctaggat gacatctttg    9840
atgtccacca gcagcaggtc gattgtgtag cagtccacat cccgaggtgc atccgagggg    9900
ccatagcctc gcaagtccac agccacaaca tggaagcggc tctggaactc ccggagctgg    9960
taacgccagg agaacctgcc aggcgggcga gggagggagg ctgagtcagg gccctcaggt   10020
tgcacctgca ccctacgcac atcagcaacc attggcaaac cctccttcct gctctaaact   10080
tcccctaaaa ggaggatggg gaatcccagg cagaggcaga ggctgaggct gaagccagct   10140
acaccctgaa tccagggaac ttcaatcaac ctcaataccc ctttgtcctg tcccttcagg   10200
gcctagggat cctgggtatg ctggctcagt accccctatcc ccatctatgt tcagcttgtc  10260
ccgatctata cagccctcct ccctgcctct tgggagccca agctccacct gcccccgagt   10320
cccgtgggcc tcaggcccct ggccccagac gtaccagttc tcagggaagc cgtgcagaaa   10380
cagcatgagg ggtccgttac ctcgtccagc cgagacatag tgcagacgca ggcccgagct   10440
ctagggggag ggcacagcct gaagcctggg gaaccctctc tcccgaggct tgaaccgtct   10500
cgaccccacg cgaccccgca gccccgatag cgcccccgtg cgatcacctt gaggttcagg   10560
aaaccgtgct cacccagcga ggggtcgctc aggcaggcgg gggacgcgct ccgacggcgc   10620
ccgcagcagc cgcgccgggg ccggcacagc acgtgcgtga gcgctatgca gccgtagacc   10680
gccgcggcca ccagcgccac cgagaacacc aggctccaca tgaaggcgcg cagcagcttc   10740
agcgacaggc gcgacggcgc cagcagcgcg gtcaccacca gctccggcat gtcgccgcgc   10800
tccgggacca cggcggcgct gccggccagg ggcacctgca gcagcggcga agcggtgtca   10860

gggctcaggg gcccatcgcc cttggcctgg ggcccctccg tgccgtcggg atttgtggga   10920
```

```
cgcgctgaag gaagaggcgg gcggctccga cagaaaacag ccagagcgca ccactcacct   10980
gagtgccagg taaacacctg ggcgcgacag ggacaggaaa caagggtagg gtgcggaggc   11040
tggggaggaa gaggttggaa agggggaaa taaatgggcg gggcctagca ggtcctgtgc   11100
ggggcttagg gccggggcgg ggcccaggaa gactcagcag cgggtgggtg agggtctaaa   11160
ggcggcaatt ccgggccggg tgcggtggct cacgcctgta atcccagcac tttggtaggc   11220
cgaggcgggc ggatcacctg agatcaagag ctcgagacca gcctgggcaa cgtggtgaaa   11280
ccccgtctct actaaaaata caaaaattag ctgggcgtgg tggcgggcgc ctgtagtccc   11340
agctacttgg gaggctgagg caggagaatc gcttgaaccc gggacgtgga ggttgcagtg   11400
agctgagatc gcgccactgt actccagcct gggtcacaac agggaaactc cgtctcaaaa   11460
aagaaaaaaa aaaaaggcaa ttccgagccc agacaaacct taaggagggg atcctggatc   11520
ttcagttaag tgggcgacac ctggagtgag gggcggggca tatgcagagt aggtgcggcc   11580
tacaagccaa aaaggagaaa gagttggaat ggtgggcctg gcttatgcgg gtgggcgggg   11640
agagggtgga tcctagagga ggtgaggcct aacattgggc gaagaaggcg ggagcctggg   11700
ccaatgagct gacggtaggc cggggagggg gcggtggggt ggggtgggca atgggcaatg   11760
agacggaggg cggggccggg acctaatatg gcgggtcagg agggtctgga agacgaagaa   11820
gagggacagg caatgccagg tctaggacta ggagggaggc gcgggcggta ttagcggctg   11880
gaggaggctt cgggaggccc ggccgacggc cgccgcctgg tgctacccac ccaggggcgc   11940
gcgaccctcc cttcggtctg gctccaaaga cctagcagca ctgacttcac ccagctgtgg   12000
ttccaacggc gggtccagcg gcctcggccc ggcgccgtcc tcctgctggc ccaacaggcc   12060
cgccagcccg cccctgtacg tctgtgattg gacggcggcg gccactgatg ttcaagcgac   12120
aggtcctggc ccgggagcca atctgcaggt gttgaggccc aggctccgag agcgggccga   12180
ggaggcgtgg ataccctgat tcctaggggg caggcctggt tcccccgagg aggacccggc   12240
ctatgaatga ctggagttct ggggttctgg ccgaaagagg aagtgggaca gggccgggtg   12300
tgatggggcc tagagtcaca gagccttgcg gccctgctgt ccctgcaaga agccagcttc   12360
tggccaggcg cggtggctca cgcctgtaat cccagcactt tgggaggccg aggcgggcgg   12420
atcacgaggt caggagatcg agaccatcct aacatggtga aaccctgtct ctactaaaaa   12480
tacaaaaaat tagccaggcg tggtggcggg cgcctgtagt cccagctact agggaggctg   12540
aggcaggaga acggcgtgaa cccaggaggc ggaggttgca gtgagctgag attgcgccac   12600
tgcactccag ccagggcgac agagcgagac tccgtctcaa aaaaaaaaaa aaaagcagcc   12660
agcttcttcc tcctattttg caaccttctc ccgatatcct tgaacatttt agggacagcc   12720
atcacttaac catagagcaa ccctattaag tctaagtagc ataatcacat tcctgtagta   12780
tagatcatga acctgaaatt cgaggatgaa gtcatttgcc tgaagacata catcttgtaa   12840
aatagccatc cgcaaagatg tagggaaaaa ggcagcgatc tgtggctaca cctccccttc   12900
ctcccggaag cagccactgg aacgttttta gctttttctt tttttttcag atggagtttc   12960
gatcatagct cactgcagcc tcgaactcct gggctcaagc actacaacct gtagttggga   13020
ctacaggcct gtgccaccac actcagctaa tttttcaaaa acttttcaga gagacgactt   13080
gctatattgt ccaggctggt ctcaaactcc tggcctcaag caatcctcaa gcctccacct   13140
cccaaagtgg gattacagat gcaagccacc gtgcctgacc tttatttctt gacttaacca   13200
atgtatgaca tcagccactg acttgctcac atcactaccc cggacgtgcc gttttttgtt   13260
tccctgttag tttcctttaa aaacactcca cttgtcacct cttgcccctt tccacctccc   13320
acttttatcc atataccttt gtactgtcaa ggctgctaaa cattatactg tcctgggacc   13380
ataactaagt ccatgaagtt gccacttaaa ggtgaatagt ggtttatgtt ctgcctcagt   13440
gaacaatgca tctcaagagc cgaatagacc actatgatta cattgcccat agtgtacaaa   13500
cttttgttct ccctggacta cttaaccact acctttcttt tgttgtgtga ttagcctaca   13560
tcaaagcttt gggggttttt ttgtttttgt ttttttttgag atgggagtct caccatatca   13620
cccaagctgg aatgcagtgg tgtgatctcg gctcaccgca acctccgcct              13670
```

<210> 38
<211> 3479
<212> DNA
<213> Homo Sapiens

<400> 38

```
gctggagcga agcttgcggg acgtcgtaga gaaaaccatc caaaagtacg gcaccaaccc      60
cgaggagacc gcggccgagg agagctggga ctatgtgcag ttccaggtaa gcccccctcc     120
tccagttccc tcccggactg acccgcctca gccctgtgct tggaggagac tccaccccaa     180
cgtgggcccg acccccagct ctacgatcct aacacacccc aatccctccc aggcccgacg     240
ctccccactc cccagatgac acaactgtcc ccggcgtcgc ctggtctccc agtacccaga     300
ccctggcgtg gcttcgccat ctacctcgag agactccgcc cccgcccccg ccccgaccag     360
aggttgtcag gcccctagtc ccaagaccct agcgagacac ggcttcctac cccgtagtga     420
```

```
ctctggcttc caccggaccc cgcccccgat gagactcaca tggtctcgca ctcctattca     480
cctgtcgggt atcagaggct caattgcacc acccacccaa tctctagtcc acagcgcgac     540
cctgcagctc cttccccagc cccaggtcac cccgtgactc gtctttccca gccccatgtt     600
cccgtaatgt cccctggctc cggccccatc gtgaccccag cccactgtcc cccactccac     660
acccaccact tagtccccctg ctccccgacc tgacctcata cccatcacct tgtcccctga     720
tccccaacat catatgtctc cagtcccggt ccctctgact cgtatccctc tcccagctgc     780
gctgctgcgg ctggcactac ccgcaggact ggttccaagt cctcatcctg agaggtaacg     840
ggtcggaggc gcaccgcgtg ccctgctcct gctacaactt gtcggcgacc aacgactcca     900
caatcctaga taaggtgatc ttgccccagc tcagcaggct tggacacctg gcgcggtcca     960
gacacagtgc agacatctgc gctgtccctg cagagagcca catctaccgc gaggtgggca    1020
ggggttcgga gcataaacct gtcgaatggg gcggggcctg cgggaggggg agggctgtca    1080
gtgagtagcg gcctgagaaa gggcggggtc tacgagaaaa ggaaaggtac ggcaggaggg    1140
gcggggccct ctgaaggggg cggggtctgc aggaagggca gggcctaaag aaaaggctgg    1200
gctggctctg gaatacagat gtctagggag gggccaggct tggaaaaggt gaagcgaggg    1260
tgcactagta aggagactag agtgccctgg tgactagggg agcgggtaga tgcctgaaga    1320
cggtgagggt tggcctgaaa agaacgctgg gcctgggctt gagagtccca gaaagaatcc    1380
ctttaacttt tccctacacc ccccagggct gcgcgcaggg cctccagaag tggctgcaca    1440
acaaccttat ttccatagtg ggcatttgcc tgggcgtcgg cctactcgag gtgatctggc    1500
cccgccccca cccgcgatcg gccctaaatc cctagatggc cctgcccttc atttcgcgtc    1560
cttcggttgc ctgggaagga cgagctcagg gcggagcgca gcccaccccg gccctcccgc    1620
cgctccaccc agcaccggag ggtgggggcg gcccagcttc agggagccct gattgggtgt    1680
acgcagggaa agcctcctgc tattggctgc gatctccctc ccctttctcc gcagatgact    1740
gtcatggtgc tgagcgtaca gctacagcgc agggcactcc gccggaaatg cgagccgcac    1800
gtgccgggcg ctggggattc gagccccggg cccagcctga tcgctgacgg cggcggcggg    1860
cacagcggca gtctgtgggg tggctggggc atggcgggtg cctgccccaa ctggggagac    1920
aaggcaccgc agggcaagct gcccatggcc ctggggctct ggccgctgtg ggttcaagac    1980
gaggaccagc ctgacactgg aagtgcgggc gcagaattag aggaggcaca attagaggct    2040

gaggcagagg gggaagacag atgagcctcc aaaataaagg accctgggct tgcttccgac    2100
cttactcctt ctcagcctct acccccactt gtagcagcta ttcccgcctc atcagccagc    2160
cctgcggcag ttcccgtcga gccccgccct tttctaccta tccccttctc cagccccttc    2220
ccgcccaatt cacggcccca cccctgacct ttctcgcccg ggtgggcatc acccccgtct    2280
cgccagcacc ccttcgactt ctctgacctc atctcctttc tctatagctc gggttcatga    2340
cgctctcgat attcctgtgc agaaacctgg accacgtcta caaccggctc gctcgatacc    2400
gttaggcccc gccctcccca aagtcccgcc ccgcccccgt cacgtgcgct gggcacttcc    2460
ctgctgcctg taaatatttg tttaatcccc agttcgcctg gagccctccg ccttcacatt    2520
cccctgggga cccacgtggc tgcgtgcccc tgctgctgtc acctctccca cgggacctgg    2580
ggctttcgtc cacagcttcc tgtccccatc tgtcggccta ccaccaccca caagattatt    2640
tttcacccaa acctcaaata aatcccctgc gtttttggta aaatagcttt atcctctgtc    2700
agaacacaaa caaacaaact ttgagagggg aggaaggaaa ccgtctagct cagggctcac    2760
ttaggagagg gatgagatta gaaagttcaa cacactgctt gtgcagcgga gataaagtca    2820
agaccctagc acccacttat aaatatctcg ttatattaaa aaaaaaaaaa atgtccaggg    2880
cccacctggc tctgctcctg cacagaaagg gttcatcttc actttgtgat ctcacaggtc    2940
atggagtgag ggtggtagag aggggcagaa atttcagggg gaggggtggc tgggaaaaag    3000
taaaggggac aagccaatgt gtaactagcg ctctccaaga catgcagagg agtgggggtg    3060
gcctgtcagg ggctgaaaag aaaagccagt gctgtacctg gggggttgtc tcactcctgt    3120
ccccacaatc cctgatactc cggagtgatc tgtcctttca gacacccact gtgaggtccc    3180
aatatcgggg tttatccttt cctcagtccc agcctgttca gctctccaac caagttttgg    3240
gggcccctct aatggggggg atggccccag ttgcttaggc ctctgaggtc aacccccttta    3300
catcacagcc ctctccccaa ataagaagca tgaggtgagc tggaggaccc tccctgggag    3360
gagggtgttc tgggggggtga gccagttttg gggtcccccct tcagtccctg accaggcggt    3420
aaatgtgatg ctgggcccca cgctcgctgg tggagacctc gaagacatag gcggtgcag     3479
```

<210> 39
<211> 10857
<212> DNA
<213> Homo Sapiens

<400> 39

```
tcccagcgcc ttggggggct gcggtaggag aatcgctttg ggagcaggag ttgcaggccg      60
```

```
cagtgagcta tgatcagctt gggcgactga gcgagaccct gtctctaaaa caaacacaca     120
agtccgggcg cggtggctca tgcctgtaat cttagcactt tgggaggccg aggtgggcgg     180
atcacgaggt caagaaatcg agaccatcct ggccaacatg gtgaaacccc gtctctacta     240
aaaatacaaa aattagctgg gcgtggtggt gcgcgcctgt agtcccagct actcgggagg     300
ctgaggcagg agaatcgctt gaacccggga ggcagaggtt gcagtgagcc gagatcgtgc     360
cactgcactc cagcctggcg acagagtgag actccgtctc agaacaaaca aacaaaagga     420
tagaaaggcg agcacaaata ttcccaattc ataacactcc ctcgcactgt caatgcccca     480
gacacgcgct atcatctcta gcaaactccc ccaggcgcct gcaggatggg ttaaggaagg     540
cgacgagcac cagctgccct gctgaggctg tcccgacgtc acatgattct ccaatcacat     600
gatccctaga aatggggtgt ggggcgagag gaagcaggga ggagagtgat ttgagtagaa     660
aagaaacaca gcattccagg ctggccccac ctctatattg ataagtagcc aatgggagcg     720
ggtagccctg atccctggcc aatggaaact gaggtaggcg ggtcatcgcg ctggggtctg     780
tagtctgagc gctacccggt tgctgctgcc caaggaccgc ggagtcggac gcaggtagga     840
gagcggccgc gcagacctct cgcctgctcc tgcccagggg cccgccaggg ccatgtgagc     900
ttgaggttcc cctggagtct cagccggaga caacagaaga accgcttact gaaactcctt     960
gggggttctg atacactagg gggagtttta tgggaaagag gaagcagtaa ttgcagtgac    1020
gccccgttag aaggggcttt ctacctcccc agcattcccc caaagcaggg accacaccat    1080
tcttgaccca gctccacccc tgtcggtagg tgctggcttc ttcccctctc ctggtggtgg    1140
tgggtggttc ccgcggcggc ctggagccgg aggggcgcgc gaccctgggc tgggagctcc    1200
gagggcctgg gaacgagacc tgagaccttg gcttctcgaa ggtagtaggg acttgggagt    1260
ggtgactgaa cctggtctgg ctcctcctta cttcctcttg ttgcgggtgg gacgagctag    1320
cttccgcctc tcccagccac tttttcctgc tcatttgcag ctaggttggc tcccctttg    1380
ggaatttcct ctccccttgg cactcggagt tggggggtgc cacctagtgg aagataacgg    1440
agctagggtc ttgaagaggc tgctgtcccc tctggctgtt ttggcggtgt agggtggcat    1500
gagagactgc gactcgcctc ctcatccctg tttctgtatg cgagtgcttg tattcagtag    1560
aagcatacac tatactccct caatttaggg taaacaggag gggccacatg cacaggtaat    1620
tcaccaggga gccgaacact cctgtgcaga cagactcccc ttcccagcaa gccatggcag    1680
cggacagcct gctgagaaca cccaggaagc aggcggtgcc agctgcaggt gctttgcctg    1740
ggagctgtgg ggctgaggag agggtccact gtccaggacc agtgaacttc atccttatct    1800
gtccaggagg tggcctcttg gggatgctga gttaggggag gggcacttga ggaaagccag    1860
gtggagcaga gaggatgtga gtgactgggt gggtgagatt tcctgcccct ccccccgcag    1920
tggtatccac acctagactc gtggggtaac tgaggcacag acagagagca acttctcagg    1980
ccctcacagt tggcaattct aggattagga cccaagtgcg attttcaggc agtccctgta    2040
ccctgtttct gttgtacctg ttgcaccatt cccaggcact gcccatcgtg ccactagtga    2100
tatgaaccca ggtccaatac gctctggggc catcaaagcc tgacgtcacc atgacctgat    2160
gtgtgacgtg ttataggtgt cccttggtat cttcacggaa ctggttccag gaccccaaaa    2220
tctgtgggtg ctcaagcccc tgagataaaa tggtgtaata tttgcatata acctatacat    2280
actttaaatc atttctagat tacttatacc taatacaatg gaaatgacat gtcggctggg    2340
cgtggtggct catgcctgta atcccaccac tttgggaggc cgtggcaggt ggatcacctg    2400
aggtctggag tttgagacca gcctgaccaa catggtgaaa cccccatctc tactaaaaat    2460
acaaaaatta gccaggtgtg gtagcgcaca cctataatcc cacctacttg ggaggctgag    2520
gcaggagaat tgcttgaacc tgggaggcgg agttcgcagt aagctgagat cgcgccactg    2580
tactacagcc tgggtgacag agcaggactc catctcaaaa aaaaaagaga aaaagaaaaa    2640
gaaatgccat gtaaatagtt gtgatcctga attgtttagg gaataataag aaagaactat    2700
ctgtagatgt tcagtataga tgcacccatc gtaagcctaa ctacattgta taactcagca    2760
acgatgtaac attttcaggg gtttttttgt tttgtttttt gagacagaat ctcagtctca    2820
ctctgtcacc caggctggag tatgttggcg tgatctctgc tcactgcaac ctccacctcc    2880
tgggctcaag cgattctcct gcctcagcct cttgagtagc tgggattgca ggtgtgcgct    2940
accacgcatg gctaattttt gtatttttaa tagagatggg gttttaccac gttggtcagg    3000
ctggtcttga actcctgacc ttgggatccg cccacctggg cctcccaaag tgctgggatt    3060
acaggcgtta gccaccgcgc ccaatatatt ttgatccctg gttggatatg gaggctgac     3120
tgtacttaac atctctaagc ttcagtttcc tcctttaaaa taaaggtgtg gctgggtgtg    3180
gtggttcaag cctgtaatcc cagcacttag ggaggctgag gtgggtggat cagctgaggt    3240
caggagttca agaccagcct gaccaatatg gtgaaacccc ctctctgcta aaaatacaaa    3300
aattagccag gcgtggtggc gagcgcctgt agtcccagct acttgcttga acttgggagg    3360
cagaggttgc agtgagctga gatcgtgcca ctgaactcga gcatgggcaa cagagcaaga    3420
ctgtctcaaa aaaaaaaaaa aaaagggggt gagcagacgt ggtggcacgc tcccacagtc    3480
ccagctactt agtaggaggc caaggttgga ggattgcttg atcccaggag tctgagtcca    3540
gcctgggcaa catggcaata cctcatctct aaaaataaaa taaaagtaaa ggtattaatt    3600
actactttgg atggttgttg caaagaaata tatataaaat aatggagagt cttgtaactg    3660
gctcccaaga ggctcaacag acattactgt ttttgcttct tcattatgag ttacctctct    3720
```

ggccacccca ctgaactagc tgggctagct gagcctggga gaagagttgt ttaggaagtg 3780
agaggctgct ctccacagag actcaaggct cagttcctcc tggtgactca gatgggcagc 3840
ccagtgggca cacgtggtct ctctccacat gtggctgagt ttcacttcca gaatagatgg 3900
agaggcaagg gcagggttta gcatgcttga ggaatctcag agggccctgg tggtgtgggg 3960
gaccctcaga acacaggtgt ctcaagggct gacccagctt ctgtgtcctt ttctctgggt 4020
gaggagggga cattcatggg cagatggtga cctctgggga aggcagccca gactccactg 4080
gccaccatat ttcctttttc acaactttct cacccctgtg gtttcccatg tcatcatgtg 4140
gccgcttccc gcaaggcctt agcggggtgc aggtatgaac atagtgtcag gcaaggaggc 4200
atctggaggg gaaccctggc ttttcctggg gggactccct ccctgcaccc tagccctgtc 4260
ctctcccatg gctactgatg ccttcccctc accccagagg tggcccacat ctgcacagat 4320
cagacccaca aaaatcacgt cttcctgact ctcataagcc tgcccagtga ggcccaggca 4380
ttaggccatg tgctggggac tcagacccac acatatacgc atgtcagcat tcatgcttac 4440
aggtccgcac atgctggggc aagtgtcaca cacggggcgc tgtaggaagc tgactctcag 4500
cccctgcaga tttctgcctg cctggacagg gaggtgttga gaaggctcag gcagtcctgg 4560
gccaggacct tggcctgggg ctagggtact gagtgaccct agaatcaagg gtggcgtggg 4620
cttaagcagt tgccagacgt tccttggtac tttgcaggca gaccatgtgg accctggtga 4680
gctgggtggc cttaacagca gggctggtgg ctggaacgcg gtgcccagat ggtcagttct 4740
gccctgtggc ctgctgcctg gaccccggag gagccagcta cagctgctgc cgtccccttc 4800
tggtgagtgc ccctcagcct aggcaagagc tggcagcctg ggttttccca aagggtcatc 4860
ttggattggc cagaggagga cgccaggcac aagtctgtgg tttatcattt tccctgtctt 4920
tctaggacaa atggcccaca acactgagca ggcatctggg tggcccctgc caggttgatg 4980
cccactgctc tgccggccac tcctgcatct ttaccgtctc agggacttcc agttgctgcc 5040
ccttcccaga ggtgagcgtg ccatcagccc agtggagggg cttaggtctg catttatgct 5100
tttcctgcac tctaccacct gcagataaaa gggccctgcc aatgcaggtt tctctgtgtt 5160
ccacaggccg tggcatgcgg ggatggccat cactgctgcc cacggggctt ccactgcagt 5220
gcagacgggc gatcctgctt ccaaagatca ggtgcagctg gggtgtgggt gcagggcagg 5280
cagacgggca gcatgtggag tctggaaccc aggagcccag ctggcggggg cagccctgat 5340
tcctgccctt gtgccctcat tcatgtggca tctgtactaa gcaacagccc tgctgtggac 5400
agaggggcag cactggggat aggagggtgc gggagaaagt gcaagactcc aggtccaggc 5460
gttgtggggg tggggagagg tcgagctggg ccggtctaat accaacccat ggtcagtggg 5520
tgccccttcc ccatgccatc ttgctgaggg agggactgga ttgtgaggag ggtgagttag 5580
gcctgcctag gagatcactg agccttagtg tcaccctcaa accccagtag ctgggcttgc 5640
aggccctggt gccaccagct ccttgtgtga tgggggagtc accttccctg agtgggctgg 5700
tagtatcctg ggtcatcttg tccacaggta acaactccgt gggtgccatc cagtgccctg 5760
atagtcagtt cgaatgcccg gacttctcca cgtgctgtgt tatggtcgat ggctcctggg 5820
ggtgctgccc catgccccag gtacaaatct gggggagatg gggtatgtg gagggaagtg 5880
ggggcagagt tgggggccag gggcaggggg tgaagacgga gtcaggacca ttttttctca 5940
ggcttcctgc tgtgaagaca gggtgcactg ctgtccgcac ggtgccttct gcgacctggt 6000
tcacacccgc tgcatcacac ccacgggcac ccacccccctg gcaaagaagc tccctgccca 6060
gaggactaac agggcaggtg aggaggtggg agagcatcag gccaggggct ggggcggggc 6120
ctcattgact ccaagtgtag gaaaaagttt cctccatcct ggctgcccct cacgtttgct 6180
cctcttccag tggccttgtc cagctcggtc atgtgtccgg acgcacggtc ccggtgccct 6240
gatggttcta cctgctgtga gctgcccagt gggaagtatg gctgctgccc aatgcccaac 6300
gtgagtgagg ggctggagcc agcttggctg tgtgccccca gccacctggc cctgacacgc 6360
accttacagg ggctctgtgg catggggctg gctggctgct tgctgggagc ctggctgatg 6420
cagggttcat gctacccccct agtgggggat tggggcagtg ccagccatca gcctggctgc 6480
tccctgtgtg ctactgagcc tggaagtgac aaagacccac ccctgtcccc actcaggcca 6540
cctgctgctc cgatcacctg cactgctgcc cccaagacac tgtgtgtgac ctgatccaga 6600
gtaagtgcct ctccaaggag aacgctacca cggacctcct cactaagctg cctgcgcaca 6660
caggtaccag aggcagggtg cagatacagg ggtggggccc cctttcctcc cttttaggcc 6720
tggccttagg atcactgcaa ggtggtgtaa gcggtaccct ccatcttcaa cacctggttc 6780
cagctgtgga gccggcaaag ggttgatacc cctgagggtc cccagtgcca cttctgacct 6840
gtcctctctg cttccctcac agtgggggat gtgaaatgtg acatggaggt gagctgccca 6900
gatggctata cctgctgccg tctacagtcg gggggcctggg gctgctgccc ttttacccag 6960
gtacccaggg gtggcgggtg ggtgggctga gcacagtgtg gcaggcagcc gggcccccagt 7020
gcccacctgc ccttcttcat ctgccctagg ctgtgtgctg tgaggaccac atacactgct 7080
gtcccgcggg gtttacgtgt gacacgcaga agggtacctg tgaacagggg ccccaccagg 7140
tgccctggat ggagaaggcc ccagctcacc tcagcctgcc agacccacaa gccttgaaga 7200
gagatgtccc ctgtgataat gtcagcagct gtccctcctc cgatacctgc tgccaactca 7260
cgtctgggga gtggggctgc tgtccaatcc cagaggtata tgggaggggga cagcatcttg 7320
gcctgggcag gtgggtggcc aagctcctat tgctttctgc cctccgcata gcccataggt 7380

gatacccagc tctgacagat tcgtccccag ctggaggtgc tgtaagcagg agaggcgggc 7440
tggagtaggt aggggctcgg cactgcgccc cacatagtgg ctacctacaa cgccctttcc 7500
tgcccacccc ccaggctgtc tgctgctcgg accaccagca ctgctgcccc cagggctaca 7560
cgtgtgtagc tgaggggcag tgtcagcgag gaagcgagat cgtggctgga ctggagaaga 7620
tgcctgcccg ccgggcttcc ttatcccacc ccagagacat cggctgtgac cagcacacca 7680
gctgcccggt ggggcagacc tgctgcccga gcctgggtgg gagctgggcc tgctgccagt 7740
tgccccatgt gagtgcctcc ctgcctgccc ctggataggg gagctaagcc cagtgagggg 7800
acaggaacat aatgccattc tgtgctccct tccccgccag gctgtgtgct gcgaggatcg 7860
ccagcactgc tgcccggctg gctacacctg caacgtgaag gctcgatcct gcgagaagga 7920
agtggtctct gcccagcctg ccaccttcct ggcccgtagc cctcacgtgg gtgtgaagga 7980
cgtggagtgt ggggaaggac acttctgcca tgataaccag acctgctgcc gagacaaccg 8040
acagggctgg gcctgctgtc cctaccgcca ggtcagtgcc aacccccatc ctggggctgg 8100
gtatggccag ggaccaggtc ccacctcgtc caaccctctc gcccccctct gaccatccag 8160
ggcgtctgtt gtgctgatcg gcgccactgc tgtcctgctg gcttccgctg cgcagccagg 8220
ggtaccaagt gtttgcgcag ggaggccccg cgctgggacg cccctttgag ggacccagcc 8280
ttgagacagc tgctgtgagg gacagtactg aagactctgc agccctcggg accccactcg 8340
gagggtgccc tctgctcagg cctccctagc acctcccccct aaccaaattc tccctggacc 8400
ccattctgag ctccccatca ccatgggagg tggggcctca atctaaggcc ttccctgtca 8460
gaagggggtt gtggcaaaag ccacattaca agctgccatc ccctccccgt ttcagtggac 8520
cctgtggcca ggtgcttttc cctatccaca ggggtgtttg tgtgtgtgcg cgtgtgcgtt 8580
tcaataaagt ttgtacactt tcttaacagt gtctgatttg ccgccctgcc tgccctcccc 8640
agggccccag aacaggggtt cacgtccact gccaacactc ccctccccta ccccacagaa 8700
agacatacac agccttaacc tcaccagttt tatatttgct gctgcccaca tcggctgtat 8760
cacattaccc cctagtcccc agagctgtcc cagccaccag ccctgtgaca tcgtagccca 8820
gagatgggct agtcagcaag cagcaccccc tcccctccca ggggtccaca aagaacgccc 8880
cctcccttcc cagccctcac actagcagct gaggctgggt cacccctcct gctttcccac 8940
aatagagctt tctatgtaca gccacgtcta cacaggcact gcttcccccc agccctcctc 9000
cccggcacct ccccgtgggg gtctggaccc cccctcctcc ccggcttgga ggcagacaca 9060
gggtcccttg caagacacga cccagcacca accacggaac agctccaagg cccctgggcc 9120
cctctccggc ctggggctgg gagctacgcg cgagggcccc cgcgggcccc cggggcgcgc 9180
accctgggtg cgggcccgcg cgggaggggc ggtgccaggc cctgcgcggg cgctacttga 9240
cgttgaacac catcagcggc cgctcctccc gccgctgcca cggctcttc ttgtctcccg 9300
cctcgtcgcc cacctccgcc cccagctcgt cctccgggct ggtgagcgag tcgcgccgca 9360
gctcgctggc gctgctgcgg gagctgtccc cgcggctgcg gccccgcccc cggggtaccg 9420
tggccgcccg gccctcgggc gccgccacct cgtccagcag cggcgtcagc gagttgtcct 9480
ccggagagca gaggcccgtg cggctctcgc tgctgctggg ctccgtgtcc tcgctgagcg 9540
ccaggcgcgg gggcgcgggc ggcggcggcg gcgcggcagg cggggcgcgc tcccgctcct 9600
ccctcgcggg ccggcggcgc gcgggccgcg cctcgtggac atctacgccc gagtccaggc 9660
tggcgtcggt gctgcgtgcc ccgccgcccg cctgcaggtc gatgtaagag tggcgcactt 9720
gcgagttgga gcgcccgtcg agggacacga accaggcgcg cgggtgcggc ttcacgccca 9780
gttccagcag cttcttctcg gtcagggcct gcagctcccc gttgagctgc gccatggtgg 9840
actcgttgaa tagcacaggg atggtgactg agccactgac gggcgccgcg cgcccggccc 9900
cccagccctc gccgccgccc ccgccgccct cgccccccgg gcccgagtgg cccggcatct 9960
gcgggcgctg ggggtcgggc tggggaaaag cgcgcgccgg gccgggtgcc gtgccctccg 10020
gcggggccgg ctcgtcgccc acgccggcgg cgcccgcctc gccgccgagg cgcacgtagt 10080
gcgcggggat caccagggtg ggcatgacgt tgcggtagac gttgtccttg aggtggtcga 10140
tggagccgca gaagatgagc tgccccgcct ggcccagcga cggcggccgc gccagctggt 10200
ccaccgactg cgacagcagg aagtcggggg tcttgccctc ggccgccccc ttgtggccca 10260
ggtagtggtc gaagggcggc ggcggcgagg gcggctcgtg caggaaggcc gcagccccg 10320
agggcccccg ccggtgctcc tctaggccgg gctccagccc gccggggccc tcggccgagc 10380
gagcgccctt gagcccggcg ctctcgcccc cgcgggcacc cgaaggctcg gcggccgggc 10440
ggctggcaga gcgcggcttg gtgcggaaga agtcatcccg ggaggaggcc aagtcccggg 10500
agctggagaa ggccgagtgg agggggcctg gaggcggagc ctcggggtcc cccgacgggg 10560
cgggttccag gggtccccca cagatgaggt ggagctggga catcgaggtg gcctggtctc 10620
gtttgttacc gtcagagggc cccgagagct gcagcttgcg gtgctgttgc ctcggcttca 10680
ggcagcgcct cctggaaggg agggagcaga aggggccgct caggaaaggg tgagggccaa 10740
agtaggcccc gaaccccctt agatgcaaga caagagacga atatagtgga agaggtgggg 10800
agaatgcctg ggcgctcag gagagatggc ggggagccgc cgtgtccgtt tggcgat 10857

<210> 40
<211> 5465

<212> DNA
<213> Homo Sapiens

<400> 40

```
tcccggactt ggcaaggtgc ggtggcttat gcgtataatc ccagcactct gggaagccca      60
ggtgggtgga tcacatgagg ccaggagttc cagaccagcc tggccaacat ggcaaaactc     120
catctctact aaaaatacaa aaattagccg ggcatggtgg tgcacatctg tagtcctagc     180
tacttgggag gctgaggcag gggaatagct tgaacccggg acgtggaggt tgcagtgcgc     240
caagatcgtg ccactccgct ccaacctgcg caacagagtg agattgtcta aagaaaaaaa     300
aaaaatccca gactcaatgc cagctgtttg tgttagcctg gggatccttc acttataggg     360
tgcagtctgg ccaagaggaa ggagctatat aacatggttg atggaatttc taacatttaa     420
gctcaggggt acatgtgcag gatgtgcagg tttgttacat aggtaaatgt gtaccatggt     480
ggtccactgc acagatcatc ccatcaccta ggtattaagc ccagcatccg ttagctattg     540
ttcctgatgc tctccctcct cccacccctc accctctgac acgccccagt ctgtgctgtt     600
cctcccatgt gtccatgtct tcttaggttg atgtggtctt gagtgacccc tccttgcctt     660
tcctctttaa aaacaagatc taaaaggcta tgttctctat gggttggagg tgttgcctcg     720
atctgtcaag cacagtccag gactcagagg ctgttgtggt gaactactgt aggcacctgg     780
aagccaccaa ggtgcccttt tcccgcactc tagtcccaag tgagggaaat aagcctatgg     840
ttaagggaga actatctacc aatttgaact cattaaaata cataaatgtg tccactagaa     900
atggctggct ggagaagccc agtaccaaat caccccgttc ctgaaaccca ccagccccat     960
gaaatagttc gctagagaga gatttgcaat ctttgaattc gttgactatc aacctgtata    1020
ggtgctccgt gccccgagat ctgagaaaat gacaaaatac gtctacttcc cggccgggca    1080
cagtgactca cgcctgtaat cccaatactt tgagaggcca agttgggcag atcacttgag    1140
gtcaggagtt tgagaccagc ctggccaaca tggtaaaact cccgtctcta ctaaaaatac    1200
aaaaattagc tgagggtggt ggtgggcgcc tttaattcca gttactccgg aggctgagcc    1260
acaagaatcg cgggaagcgg aggttgcagt gagtcgagat ggggccacag cactccagcc    1320
tgggcgacag agtgggactc agtctcaaaa aaaaaaaaaa agtatttccc aattcttcgt    1380
cagtttttgc cctctcccca cttcgccttc tccaggctcc tgaaaagtat ctcccccaac    1440
cctttctgtg tacacgggga aaacacgtgt tcatttttat tatggcgctg aaacctaccc    1500
ttctcttcaa atcgcttctg tagatcgttc tagtataaaa tgtggcaaga ggctaaaggg    1560
agagatatag accagttctt tggccctcgc tttccaactt caagttacac ctgtgaaact    1620
catgggtcct tccacagcct tcaaaaacta agggcgtccc ctgtcctctc cccagatgtc    1680
ccttccccat cgccggtagc gagtgggaga cagctcagcg cggggcaggg gagcactggg    1740
cccggagatg gaaggcagcg tcaaaagcgc cgctggaaaa tccctgagcg ctaaccgttg    1800
cctgtgtgag cccttaaatc tacaaatttc caacacctgt agcctttggg tttcccagga    1860
cttccatcga ccctggcggc agagagggca ggcctgagat gcagtgactt gagggcacat    1920
ggccaactct tgtcactcca agatcacact ggggaaccag actgacttct ccaattctga    1980
actcgccccg gcctcgggcg gctcaaaggg cctcctctgc cgcatccccg ccaaaaccaa    2040
accgcctggc acaagccggt aagcaaccac cctgctggga gagggaagga agagtaggcg    2100
cagccctaga tcaatttcct tgcactgctt ctcccagacg gtcaagtcag ctgcgtccca    2160
ccgaaaaggg cgcatcgccc acgcccgaaa cgcagccgct gggggccgag aaattatccc    2220
cacctggccc gagggccagg gacgcaggag cgcagcagcg tggaggggct ccgcgctggc    2280
ccggcgctgc ccgcggtcct gccctcgttc caagggcacg gcgccggtac gaggacaccg    2340
acgctgtggc gcaactgccg tcccccgcag caatcccgga gcccggctcc cggccgcccc    2400
tcggccctgc gcaggctgcc tctccccgac gcggagtccc accccgctac ccgccgccca    2460
gacgacctca taaacaagtc ctcgaagtgc ggaggcagga ggcggggcgc agcgcggggg    2520
caggaggcgg gccagggtca gggcagaggc tgcggccgcg cgtccccatt ggccgggacg    2580
cagtgagccg cccggagctc ggcgcgggcg gggcctgccg gcgcgtgccc gcccacacac    2640
ccgcgccggt gcccgccccc cgccctccgc gcccgccccg tgcccgcccc aagccggccg    2700
acggagtttt taaagtgggc tgccggccgc gggagcttta cactcgcgag cggaccgcca    2760
cacgggtccg gtgcccgctg cgcttccgcc ccagcgctcc tgaggcggcc gtacaatcct    2820
cggcagtgtc ctgagactgt atggtcagct cagcccggcc tccgactcct tccgactccc    2880
agcattcgag ccactttttt ttttctttga aaactcagaa aagtgactcc ttttccaggg    2940
aaaaaggaac ttgggttccc ttctctccgt cctcttttcg ggtctgacag cctccaccca    3000
ctccttcccc ggaccccgcc tccgcgcgca ggttcctccc agtcaccttt ctccaccccc    3060
gcccccgcac ctagcccgcc gcgcgccacc ttccacctga ctgcgcgggg cgctcgggac    3120
ctgcgcgcac ctcggacctt caccacccgc ccgggccgcg gggagcggac gagggccaca    3180
gccccccacc cgccagggag cccaggtgct cggcgtctga acgtctcaaa gggccacagc    3240
gacaatgaca gctgacaagg agaagaaaag gtaagcgggc gtccgggccg atcaggggc     3300
cggtccgagg ccagggccgg gctgcgcggg gcaggcgcga ccgagagtgg ttgggagaag    3360
```

agtgctgaga ggtcttcgga gctcgaggct cggtttggag ggctgtgagg gggagagcat 3420
gtgcccggtt tgggggcgcg gatcagcagc tttgcagtgg agacttctgc ggctcggagg 3480
agtcggggat ttgcgcgcac ggcgaggcca ggaggccgag ggagatggcc tggaggtcgg 3540
aggtgcttcc gcggtgcctt tgaaaatctc ccagccgccc cgcagcagat taccgtggcc 3600
accggcgctt ggaaatgttt gtgggtgcat gtcctcgact ttctctggtc tctcattttg 3660
gggcagcatc cacgtctcta tttttctctg gattcgcgga gtccttccaa atgcgctcct 3720
gtgcccgcgc cgcggcccaa gtgggcaaag gggggcggga ggcggagagc ggctcaggga 3780
cacgatccta ccgaggagcc aggacccttc agagcgcccc gctgccgggc tccagacccc 3840
accgaaggtt ggagaaccca cttcctcgtg ccgcaccttg actctgggga gagtaggtag 3900
tgagtgacct ggattgccct gcgggggcag atggttcggt gtgtaggaag cggacggcaa 3960
cactggatgt ccctggcagg tccgggtgtc tgtctccgaa gaggacagag aagggcagcc 4020
acgatctgcc cgcctgccct cgcgagcctc tcggcactgg gtgagaggca actctggcca 4080
tttcttgctg ccctctcgcc ctctcccggc cgctcccagt ccagccgggc ccggcgctac 4140
ccgagcgagg gttcgagccc tctgcgcggc cgcgcagaag caggcagggc ctcaacttct 4200
gcaaatgtgt gcggctcgcc gcctgtcccc tttctcctcc tgtctccacc cgtgcggccc 4260
cagtggcctg gagcttccag ccccgcgctt ggccgcggct tggcgaggct atgctgcggg 4320
aagctggaat ccaacgcgcg gccggctgaa ccgcctgagc cgcgggaaac cagcggcgga 4380
gcggcggtat agaggtgcga ggatgaggag gaccgactcg ctaagggagg gaggtgactg 4440
gccgggaggg ctcactgccg aggttgattt gctcttgttc caacttccac tacgggactg 4500
gagccagaaa ccgtgtatcc tccggtcgaa agcagcggtt cccacctcgg ggcaccgata 4560
aggatttgat aaacgggagc gaatcgcgcc tgtcctggct cggcgcccgg gcccttcact 4620
gcgggatcgc taggggaggg gattgggccc tgctcccctt tcctggccgt agtccccccct 4680
ttctcgtcac cgtcgcgccc tcagacccaa gacaagtgag ggagccctga gactgcagcc 4740
ggcacattcc cagggccctc ggagcagtcc tggggttcca tgtcttttga aggcagcaga 4800
gcaaggaaaa gactcttaag attccagtgc gttttgtaaa aaccaatcat aatgctaata 4860
atgactgaac catcgaaaag caaagttata gacagcgcct gcagacgctt tcgggtccgg 4920
agccggggag ggaacttgtg tattttacaa ctgcgatgcc tatcgggaat gtggcaatcg 4980
ccgaggtggg gcctgacagt aatcttgaca aatgtgtgaa gtgtcagctc ctcctgcgct 5040
ccttagcgcc gggttttgtg cagttttgta ccgtatatac cgagaaactt gggagcaggg 5100
gaaaaatgat ttcgacacac ccagctacag tacctggttc gttagcggac tgttggaaag 5160
agagaagaag cgggaatggg agcgggaaga acgttgcgta attagactcc caattattgg 5220
cgagagcggc cgctttaaga accactgtgg ggactgcctg cgaaacgcct tggaggccgc 5280
tgtgtgagct gcggctccgg gctggtggcc acgcgagggt gtccgccctc ttcgacctgc 5340
ctggattttt gtgctgcaga atggcagggc aaacagcgtg tccttcacca tctagagccc 5400
cttaaggggt tgtccaattc ctattgggct aatcagtttg agactcattt acttttcgtg 5460
cctgg 5465

<210> 41
<211> 22169
<212> DNA
<213> Homo Sapiens

<400> 41

ggacgcgcca gtttcctcct ttggacttga tgaggcacga acgcatctct aataaagcca 60
ggtctccccg ccgtggctcc ctgggcgggt gcctgtggct cgggccatga gtcacgctgg 120
gtaaccccac tacggggaag agggcaggaa gctgggagcc accgcctctg tgcccggttg 180
tcatctcggc acgagggcga ccgtcggctt cgtcctgccc tcatggctga gggcttttgg 240

gatgtggcgg gagacggggg agtccacttc tcaaacccgg tgcatcctgc agggccgctg 300
cactcacaaa aaggctgact ccacacagga cctgcctccc tgggccttgg ctcaggctgg 360
ggcgagactg gcctcctgtg ctacctgtgg aagagtttgt tcagcaaatt gacagtgtca 420
acagaatgtc aagggggcat ttacccagct ttaaacaacc aattcaagcc cttcagagat 480
gcacaatgga ctccgaggcc aggggactgc gtagacaggg tcgacatctg atgacaaatt 540
ctcatctgcc aattagagca aggtcggggc gtgtcaccaa cctcagtgcc aggcggagca 600
gaaggaacaa cacgactatt tcttggggaa aaatctcaac tagcaaccaa ggagctggca 660
ggcctcccac tgggcgagaa cagcgcccac ctggtgccca ccagactgaa cagccccgtg 720
caagtgaggc cgggtgagtg ttctgtggcc attggtccct actgtggcgg caggcgctgt 780
gggggttggg tccgggcagc atctgggcct ggcctgatcc ggagacgccc ttggtagtgg 840
ccactcacag actgtgattg cgaggacccc gatggggccg ctgtgtgtgc ctgggaactg 900
caggtgtgga tgtcagaggc aaagcctccc ctgtgcacgc ccagcacatc agagcccccc 960

agttccctct cacagagccc ccagtccctc cccacagagc ccccagtccc tccccacaga 1020
gccccccagt ccctcctcac agagcccccg agtccctccc cacagagccc ccagtccctc 1080
cccacagagc cccccagtcc ctccccacag agcccccaag tccgtcccca cagagccccc 1140
agtccctccc cacagagccc ccgagtccgt ccccacaggc cccactggac accaggcccc 1200
agacctcctt ccaggaaatt gcaggaacca cagcagggca gccccacaga cctgagggaa 1260
ccgctgcccc acccccgccc cacagagccc tggtgtctgg gctgcagctg aggctgaggg 1320
gctgaggact cggaggatgg gatctcccgc ccagcgggtg cagccaggtg ggagggggcca 1380
ggcccccagg gttcacaagg ccccttgggg acccacccga agccaccgct ttgcattagg 1440
cagggcgtgc ggagagggcc ctgtttaacc gtgcgggctc aatctggagt gtagacggca 1500
ggacgccccc agccctgggc ccctgagcct gtgagtgggg ggttcagagg tgaggcctct 1560
acagaagtct ccctcccagg ggcaatgggt gcagggtagg cggggagcac tcaggatccc 1620
tcccaggggc aatgggtgca gggtgggagt gggtggaggg gggagcactc aggatccctc 1680
ccggggcaat gggtgcatgg tgggtggagg ggggagcact caggatccct cccaggggca 1740
atgggtgcag ggtgggtgga gaggggagca ctcaggatcc ctcctggggc aatgggtgca 1800
gggtgggtcg gggggagcac tcaggatccc tcccaggggc aatgggtgca gggtgggagt 1860
gggtggaggg gggagcactc aggatccctc ccggggcaat gagtgcaggg tgggtggaga 1920
ggggagcact caggatccct cccaggggca atgggtgcag ggtgggtgga ggggggatca 1980
ctcaggatcc ctcccagggg caatgggtgc agggtgggag tgggtggagg ggggagcact 2040
caggatccct cccggggcaa tgagtgcagg gtgggtggag aggggagcac tcaggatccc 2100
tcctggggca atgggtgcat ggtgggtggg ggggagcact caggatccct cccaggagca 2160
acgggtacag ggtagggggg cgcactgagg acactgctgg ggagtttctc ctcccagaat 2220
tccacaggtc tcggagatac actgaaagca accccaaaat gctaggaccg cctataaccg 2280
ccctgcacca cccctggggc aaccaggcac cccgatgcta acacgcacgc atcctcccga 2340
cagggagaag tcccccagct gcctgcagac aggcctcgc tgtcaagggc cactggctgg 2400
cctggtgccc tgcaggggac atggcgcttc cccgtcccaa gggcaacttg gagaggacac 2460
catctgtgtc tcagagtgag gggccaggtt cctagctctc tcctgtcaca cgggcctgga 2520
tctaggccag ggcatagcca gtcactctaa gctgaccctg agggtccagg gggagaagca 2580
gcctggagcc aatcacacag agctctgtcc agggaaggga aggccccgga gcaggcagac 2640
tgggcgggtg ggggctgaga tgcctgcccg ccccatggat gtgtccaaag cctcactgcc 2700
ccccgctgcc ccagccaccg ccgctccttt ggtctggagc tccctgccgc tgaccctgtc 2760
ccagccagcg tggaagctgc tccgtggtgg gcctcacttg aaagccacag caggtgcacc 2820
cagatggggt cccacagcac tgactttgca cacaggaggc catgaaggct gcagtgacta 2880
cccctagctt gtggccaagc ccgtccatcc tgtggcctgg ttcttaaggg gcatgctcct 2940
cccctgccca ggggggcctg catctgccgt gcccacccat accccagggt tcagcatgta 3000
gtagatgctc aataaagacc ctctactttg cattcttaca gcaccaggcg tgtggacgct 3060
cagtgaccgc ggccccactg tgtgcctcct ggggggctaag ggatgctgaa cagcttggcg 3120
gctggcatga gtgaggccca gggacaggtt gtgagcctgt ctcggagtcc tgagcaggac 3180
aggggccgcg ggcacgccag tccatcctac aggttcacct gggaatatgc caggactccc 3240
tctagggggc tctgtggctg tcgggtggct gcaccggccc agagtgagcg ccatctcttc 3300
caggaagcca ccccggacgc cctcccaaca cctgcctcct ctggactgtc tgggagctgg 3360
gatgctgggg tccacctctg gccagtccct gatgcttctg gaggctaaaa tgtagtcctc 3420
agaagaatgc agttctagtc cacgcgtgac atggatgaac cttaaacaca tcacgctcag 3480
tgaaagaagc caggggcaaa agaccaccta tggcacaatt ccattcatag gcaacgtcca 3540
gaagaggcga attcagaggc agaaagcggg ttagtggttg ccaggggctg gggcagtcag 3600
ggcagagtga cagtatttgg gtacagtttc tgttttgtgc agtgagaaag tcccggaact 3660
agatagtggt tgaatgaact tgtgaatgga ctagatgtca agcaacagca tgctttagtt 3720
caaaagtcac attttcagcc tggacaacat agcaagaccc cgcttctgca aaaaaattta 3780
aaattagcta ggcatggtgg tgcacacctg tggtcccagc tactctggag gcggaggcag 3840
gaggatcgct tgagcccagg agtttgaggc tgcagtcagc cgtgatccca ccacttcact 3900
ccagcctgtg tgacagggtg agaccccatc tcaaaaaaca aaaaagtggc catgcacagt 3960
ggctcacgcc tgtaatccca gcactttgag aggccaaggc aggtggatca cctgaggtta 4020
ggagtttgag accagcctga ccaatatggt gaaaccctgc ccctactaag aatacaaaaa 4080
ttagctggga gtggtggtat gtgcctgtag tcccagctac tcaggagcat cgcgtgaatc 4140
tgggaggcag aggttacagt gagccaagac tgcgtcactc taatccagtc tgggtgacaa 4200
agcaagactc catctcaaaa aacaaacaaa aaaaggcaaa ttttgtaata tgaataagtc 4260
atcacccaga atccctccat atacatatcc atccccaaac cagtccttag ctccctttgc 4320
tcccagggag cagggtgggg tggggcaggg ccacaaggag aacccaacca ggcaactttg 4380
gaaaagggct tccctccacc ctcccacccc tgcagccact gccccttccc cagctggggc 4440
gaccaccgag cccagcgcag gcctcgccgc aggcccacca gccaccacca cgccttcagc 4500
ccgccccagg gtcccgctgg ctgtagttgg aaggggaatc acatacagat ggggaaactg 4560
aggcccaggg aggggacagt gtggggagca cagcaaatca gagccagccc tgcttcacag 4620

cccctcggag ctccgggccc cacaggagaa agcgccagcg ctgcctggga aactcgaggc 4680
cgtcaaacgc agcccggccc acaggtgtga tgccccaaag ccagccgctc gttaaagctg 4740
ctggtatttc ctcctcagcc cggggaaaga tccggaaagt tctggatatg cctgcacatg 4800
aaggtgaaag ctgacgccag cctgcctcag atccccacgc cctcgcagcc acagctttcc 4860
cacctgtgcg ctggggatga aaactgcact ggcacacctg gagggtgggc ggccgtctgc 4920
cagggctttt acgaggctgg gtaacagcgg cgtcctgagt gtggagctgt catcgctgca 4980
ttattgagga aggcaagcta gacgcccaat cgattctgca aagccacatc ctttcactat 5040
ttatagacga gacatgaaac aggaaaggtc cctccccac agcaaaaggg tgcagtccct 5100
cggcccggcc tggaactgcc cagtgggcac cggtggccgc tgctgcccat cacaggtgca 5160
gggaatgagg aatgaagcca gtcccggctc ctgtggggtc tccagtttgc cgcccgcatc 5220
tgggggtccc cgcacccagc tctgccctgt ggaagcactt cccctggtgc actgtctgcc 5280
ctgccctacc ctcttcctgc cagggttgct gttttctttc tccctctctc tttttctatt 5340
attattcttt tcaatggaaa acatgttggc tgctgttcag aggcagggcc tgccccaagg 5400
agccagcttc cttcagagca ctccaggctc ccaccccagt ctccgcccca ggccttcctg 5460
ttcctgatgt ctccccagtc tgagtaggtg agactttcta tttgacactg tgtggggttt 5520
tttttgttgt tttttgtttt tttgagacag agtctcactt tgtcacccag gctggagtgc 5580
agtggcacga tctcagctca ctgcagcctc cgtctcctgg gttcaaagga tcctcctgcc 5640
tcagcttccc aagtagctgg gattacaggc acacgccact gcgcctgggt aatttttgta 5700
tttttagtag agacgggggt ttcgccatgt tggccaggct ggtcttgaac tcctgacctc 5760
aggtgatctg cccacctcgg atgctgcatg tttttaaaca tgtttttcta accctggcgc 5820
ccttgaagac caggttctgc ataaacagga ggtcctgtca caggcctttc cgctcagctc 5880
agcatgagga ggagaatcaa cctgttcctc ccctggccac gcccaagacg aggcaggtga 5940
ctcaatggaa agagccagac cgggtgacag gagctacccc gtccctcccc agcctcccag 6000
gccggctgca gggccaagtc acttcccgcc tgagcctcct tcctcttctt ttaaggggaa 6060
gccacgcccc cagtccgctt gtagtcggga aatgacgggg cagctcaggc tggctggggc 6120
tgtgtttagg gacattcgct tgggagaagg agaggggcag ggctagtagg aattctgcca 6180
ctgctgtgtg accctggggc tgatggcgac ttctctgggt ctcttcttct cacttatcgg 6240
tgcctgatcc agacagcttt acggggctgc cgggagctat gccaggccac caccgggccg 6300
gcacaccccc aacatgtccc acatgtgagc taggtgcctc tctcacgcct tgctgggaag 6360
tgaactccac aggagcaggg gccatgtctg ccctgtcccc accgggggcc tggcctggag 6420
cagaacgcag cacacagcag gcgctcaaac gtgtctgtga actggagcca ccctgggaga 6480
tggacttccc cggagctgga gccatcctgg gagatggact tccccagagc cggcaagtcc 6540
ctccccaggc agtgccaggc ccagcccacc aggcagcacc tttaggattc tctgaattca 6600
ctcgtcaatc aattaaacat tcaggaagga ggctctggaa accagcactg tcagccaacg 6660
gggagaaggc ggggaagaaa ggagcgctga ccaggtgccc tctccacacg gttctcatgc 6720
aatcctcaca caccaccaag agatccagcg tgtatcagcc cggctgacat gctggggaaa 6780
ctgaggcttg catgacctga tgacccaagg tcacagagct agtggccagg ccaggcctca 6840
gacacgaggt tgtccaaagc taaagcgcac cacccagcag gcagctggac atcctcctgg 6900
aggtgctggg tgtgacagcc tacccccatt tctcccagct cctggaagac gccccgtgcc 6960
attctgactc tgccgtcagc agccaacatc ttagctgccc cctgcctcaa cgcccagccc 7020
agacgcaggc agccctggga gagcgtttca gaccaaggac gtagtctcac ccagacagat 7080
agggtccctg cagctcttcc cagctctcct caaaacaaag agaaaaaagg tggagccctc 7140
ttcagtaccc cttgctcaca ctgacggagc tgatgagggg gctagagaag agccaagggc 7200
cacagtgacc ccctgggcct gctcagaagg ccaccatctc cgaccacagc caccacggcc 7260
acacctcgca cagcagctgc ccagagacca accatcctcc gcccaccagc atgtgctgcc 7320
aggtgctttc tacctggcca ctggctgacc aggccacggc gacactctga cctcctggga 7380
aggcattttg caccaacaaa accacttttt ccaatcccgg gccctgcaac cacctgattg 7440
gcagcttccg gtgaaggttc cttctctttg ggatggaaag aaatgattca cccagtacta 7500
gtgagccagg cactgtgacg cccatcatga ccatgcccct tctacagcgg aggcagcaaa 7560
tggcaccaaa agccaaagct ctttccgggg tcacagctgg ataccacggg gccaggattc 7620
ccactcaggc tggcagggct ccaagagcct gcaccccaga ctctttcgag aaacagtgtg 7680
ggtttctccc cttgctgggg gagccaggcc tcctgcaggt gccctggcta actcctggac 7740
agacctcctg ggccttctga gaaggatggg caggaacggg aaggcagagg cacctgaggg 7800
gtgtatgttg ggtggggctc aagcccaggg agggagcccg gggccttctc cgaagcggct 7860
gcaatgtccc ccagactgct gtgtgagccc tgacctgtgc ctccacaagc cgggctcggc 7920
tgtggccctg gcctccaact ctgcggccca gaagcaggag cctgaggacg cgcttccgtc 7980
ccatcttctc gttggacttg tggggtgtgc acaatggggt ctctgtgggc tgagctcggc 8040
tgcctgtcag gccaaactga agcaggcttc ccgccaagaa ggagggggcg ccaggcccca 8100
gcaaagggga caaggccagg caccaaagcc atggtcggcg gcctgccttg ggctggaaac 8160
agctcaccgg gatcccaggt ttggggaatg ggaaagtggg cccatgacac cagtgttttg 8220
cggccctgtg gcagggtttt aacccagaca ccagggagct gcagcaatct gtaccccaaa 8280

gcacagcgct ctggcccagg gagacctcag tcccagaccc ctgtgggcca ggctgtgcct 8340
actccctggt gccactgaga agaagcaaca ggcccccaat tttcccagca cggtgttcct 8400
cccaccctcc caaagtcctc tgcaggagca agaagcttct cccagagccc cgaatgggtg 8460
agaggcaaag tccgagccgg gagcgcagtg gcggggaagg gggttataca ttctgggaag 8520
ccacgcataa ttaatcacac ggcattaatc cgcctccccc aacaatagct gctgcacttc 8580
ccctggatcc cagctgtcgg tctctgaatg aaaggaaaca agatttaggg catcaagcgt 8640
ccgtgcggct tctgcagagg gagaaggggg ccccaaaaag caaaaaaaaa aaaaaaaaaa 8700
aaagtagtgc gcattcatta gtgtctgaca aagacacaat cggctttgtc cattaaactg 8760
ctcacagacc tgcttaattg gcttcagttt ggggagggtg ggggccgggg agggaggggg 8820
cctgctgccc acaggctggg cagtcggcac aggcaggagc cgggctgggg ttgtcaaggg 8880
aatgggacct tctgcagttg gaatgagggc gcgcagactg gctggggagc catttcttca 8940
tctgtagctc ctgggggggcg gagggaactc tttttccaga caccaaacac ctcctcatta 9000
ttctcatcag ggatggactc aagttgaagg gcatcggcca cagcagaaca gttccccacg 9060
gctgatgggc actcacaggc aggcaggagg gagcggttcc gcacgacttc ccggcccccct 9120
cagtgcccat tttacatctg aggctggcag accagaaggg ccacgggatt ttgaggtcag 9180
agtttgcaga tgccaggaca gaaaccagac tcaataatta ccccacaccc cacgccaaaa 9240
catgagttcc accttctctt ccccagcccc agcctgaggc cgggaagagg gagctggggc 9300
aaacagagag cccaaaggcg ggtccgattt tgaagagtca atttcctttg aagaggcatc 9360
acccacccgg aggtggcatc acctgcccag gaagtatcac ctgtgcctag aggaggcatc 9420
acccgcctag aggaggtatc accacccaga ggaggcatca cccacctaga ggaggtatca 9480
ccacccagag gaggcatcac ccacccagga ggtggcatca cccgtgccca gaggaggcat 9540
cacgcaccca gaggaggcat cacccgccca ggaggtggca tcacctgtgc ccagaggagg 9600
cattgccgcc agcccaggag gcatcacgca cccagaggag gcatcacccg cccaggaggt 9660
ggcatcacct gtgccagagg aggcatcact accagcccaa gaggcatcac cacccaccta 9720
ggaggcatca cccccgagag gaggcatcac ctgtgcccag gaggcatcac ccgcccagag 9780
gaggcgcccc aagttggcag ggtggtgggg gctgggcctc tcagtaaatg gtccagagct 9840
tcccctggca gatcaactgt ttcttgggga cttaaagaag aattggggta ctgggactcc 9900
cacttagtga ccccggagcc tgaggtggcc cacggaggag tgcccaagcc ctcagcagcc 9960
agaccagctc cacacgcagc ataattgctg gtgattaagt aattgccaga ctttgtccaa 10020
taaaagtcag cacgtgaccg tgcccagact ctgggccatc tccagaagac aatggcctag 10080
tgttctgtcc cctgcgcggc tgcagaaggc aggggctctg ccctcgacaa agcaacaggt 10140
cccgcagggg tggtactcac acgcaggcct ccgtgccatt ggccgcttca cacttcccgc 10200
cattcaggca ggtctcaccg ggctgggagc atcgcgggcc taggcagggg caggagaaga 10260
gaggtcagtc tcacccgcac caccaccacc gaaggccctg gttaccctgg gggcggggac 10320
ctgcacccag ccaaggggca tcgtccgccc cctaccccgg acgcactctg ctcagtatca 10380
tgggttgctg gggcacgggt ccgcaggaag cccctccatg aaaccaaagg ggcgtcaaca 10440
ttctgcccag agttctgtag tcagaaatgg ggggaagggg agcagcaggg aacacgtcct 10500
gcttgaagca gggtggggtg gcctgatgtg ccgcctggac gcagtggggg aaaagcagag 10560
acaaagtggg ctactctcca aagcgttgcc ccccacccctg gaggctcact gaggagtctg 10620
gcagggcagg gccccccatc cagccgccga gattgcctct gttcaaaccc gttctgcaag 10680
ggggcagagc tggacggggg gagggggggt ggccacactc agctcccgcc cgtgggaaaa 10740
tgcgacagct gctcgcccgc taccccgccc gccaacaaag cgccggcctc cgcctcagaa 10800
tcagagcggc ccattgtgga gtcggggccg cctccagggg caccgggaca gcaccctggg 10860
gacgcggccc cgcctccgcc gacacccaat acctgcctcc gcgggcgcgc atctccaggg 10920
cagcccccat acacccacag gagggggatg aaggtccccc attcggaagg aattcgaccc 10980
catctcgaga aggatcaat tactcaagcc cctccctccc catcttaccg gggctgagaa 11040
acgggggagg ggggtgtgac acagagccgc actttctgga agaggacaga taattctcgc 11100
ctccaactct cggagaaaga gtggattaat cactcgattc cccagagacc ccgctcgctg 11160
tgcggcgggg agaaatgtaa gagcccccca cccgcgtccc gctctccgcc cccaagcttt 11220
ccaaacttca actccgcaaa gcaaaaggaa agttcagtcc ccccgggcgc ggcggcttct 11280
tacgcaaccc ctcccccaaa ctgagagccg ggctgggggg caccggcggg cggggcgacc 11340
gggagcccgg ggacccagcc cggccgcgcg ggtcagtgaa ggcgaacgcg gcgccgcaga 11400
gcccacactc cgcgccccaa catccgcccc ggcgtgggcc agaggcgaag aagaaagaag 11460
ataaatggcc cggagaagca acaggaaacc aaaaccgact ctattcaaat cgaaaaatag 11520
taggcagccc gcccgcccgg ccgaccggcg gcaagcccca cgcgcggcgg gtgaagggcg 11580
ggcccggagt agggcctccg gggccccagc accccacacc ggcctcctaa ctcggctcca 11640
ggcacgggcg gcgcgagtcc gcctccacgg ggggatcgag ggggaaggtc ggtcctccct 11700
gatcccggga ctccagaacc ccacgccccg ggccgcccgc ttttccctct ccatgctggc 11760
ctccccgccg cccgctccca gccgtggggc gcgcgcgccg ggcgccgcca aagtttccaa 11820
agggcgcgga aagtgggggc tcgcgggtgg gtgggcgcct acctcgtgcg gcgagcgcgg 11880
gcagcagcgc caggcagagc aggggcgcca ggagcggcgg catgcctccc caccggctgc 11940

cctctgcgcc cgggcggcgg cctcctgcgc tggccggcgg ggctgggacg cacacgcgcg 12000
gcgtacggtc ccggggcggc ggcggacggt cccgccctct cttccccggc tggctggcgg 12060
cgctgtgctc tcgcaggccc ccgggcccgg ctccgcgccc ggctcgttcc ttcgctgcgc 12120
tcgcgcccgc gcccgcgccc cgcgccccgc gcccttgcgc tccctcccgc ggccgaggca 12180
ctagtgaggc tcagagtcga ggtgcgctcc gcccccgccg gccccgcccc gcccggcccc 12240
acccccgccg gtccccccgc cccctccgcg gccacctcct cccgcacgcc ccgtccgcct 12300
ttgcgcctcc ccgagctgag ccgcgcgtcc cgagcccagc ctctccgggg aggagggacc 12360
cggcggcgcg gcgtgggtcg ggagcgggcg cctgggacta cttctcgttt gaaagttttc 12420
agaggccaaa agtttgagcc ggggctgcgg aaggatccgg ctctggcggt tgccgcgctc 12480
ggcgacaact ggcgactgaa atttgcatgt gaataagcgg aggagcccgg ggcggaatgg 12540
ggagggcgag gccgcggaca gccgtgtgca ccgtgcgctg ggccgggcgg ggagcagctg 12600
aggccacgtt gggggacccc gggcccgggg cagggctctg ggagagtggc ctagccgtgt 12660
gtgcgcccct agcccagcgg cttcactccc tcccgagggc cccgtggccg cggctttagg 12720
cgtccagagg atcgatcgcc gccagacatc cctggccgtg attgcccgag cacttgaccg 12780
cgagggatgg gggcgccgcg gcctctgggc aggggactcc gggcgccccg agggagcagc 12840
cgggaccagt gcctcgcgga cggattgtgc cccgctcccc caagggggac ccgcgtcgac 12900
acgctcctcg gtcaccgtcc tcctcctgcc cgggcgccgc ctgcctaagg tggccccaga 12960
aagcacaaac gggtcgggcg gatctgccgc tggcgccggc gcagcccttc ccacgtgctc 13020
cttccggctg atttatttct ccaccacgat gccaggcacg ggcaccctgt gccaagcctg 13080
gttaagcgcc aggagtccaa aatgcctgcc atagtccctg cgcaaagttc acggcctcgt 13140
gccagggaca gacagcgccc cctccccacc ccaacacagg gtggtgagtg cgatggcaga 13200
aggcgcgtgt cccggcgccc ctcctggagt aggagggcta agcctgccct gcggggagac 13260
cgaaaggctg ggagggacct aggactgtgg gggcctgggg ggagggctgc agaaacccca 13320
gagcatggtg gcagaggcag gcacagaaag caggacaaat ctcacacctg accccagcaa 13380
gcctttcctg gcacacctct tgccaaatgc tgagcactct ggaaacagcc agtgcaggcg 13440
gcctgtcccg cccctgtagc tgctgctggg agcctccctc gcctgcgtcc tcccagaatc 13500
tggcctcact tctgcaggcc tggggtaaca tcttggggtc agcgccccag gagaggaggc 13560
gcctggaggc agcagagatg tttatgtaac agcagccagg cagcctggag gagcagattt 13620
tccatagaag gaagcgcggg gctgtgcagc tcccaggctg aggactctgc ccaccacgac 13680
gtcctctgct cctggtgggt gggggtgggg tggggcatag ggaggcccga aggcctcagg 13740
cctggtgctg atcgggtgcg ggtgcgactg cctgtgtgcc tccattggca tctctgcctg 13800
tgggccccag gccagaagta atggcccatg tagcccaggc ccgggggcca gtcctcagca 13860
ttttgcggag gggcggcctg ggaggggagg aggggatccc tggatagggg gtctcccctg 13920
gtctgcccca gctgtgccga cagcacctcc accttgggcc tccatcctct accagcagag 13980
aagagagtgg cccattcggt gtccacccgt caggccaccc agcagccagg cacgctcata 14040
agcccgcgtt acggatggtg cggccgggtg cacaggcccg agggaagcac aggcctcggc 14100
ctcccagctc ccaacccctg caccacccac agccgggagt accccatgga cagctgtggc 14160
cgaggagaag ccagggatga gggggatggg cctcacctcc ccaccagcct gggaaggggg 14220
taggcccctg gcaagtaggt gagaagcttt ttcattcaca agttgtccgg gctccccatt 14280
cctctttgtc aggccagcgg gtgtgggcag tcgggggagg ctggggtcct gacacaacag 14340
gagtacatct gctggcctag ggcctggaga ccttctccta caggtcggga ggcagccctg 14400
ggcagcgggc attgcgggga ctaaagagaa acaagagcag gttgtgtgca gggcacccca 14460
gccactccag aaacccaatg ggagggtggg ggctaggacc ccaagaagct gaattggggt 14520
gcagtgccgc caggcctccc ctgtaggagt cactttgggc ttcctgtccc aacttgtggt 14580
gggagctggg gcaaggttcc aggggtaccc cgaatgaatg gggctgttcc caagctggcc 14640
agggagcctg gaagcagatg agatggggcc gcagagcctg gtttgcggag gggagaggca 14700
gacgtgtcct gcagccctct cttcttcaga ggccccctgc gggtgctgag gggagagtgg 14760
cttaggcgtt gcaccccagg agactgaact gggtcagttc cccccttgttt gctgccagag 14820
aaatgggggt accattgtca gggtgagcta aggaggccct gctgacattg gcccaaccag 14880
gcccctcctt tgcggctgct gtggggactt tcgagagcag agcacacccc taagtgcgtg 14940
gagaggtaag cacagctggt gagcacacgg ggacagcgac ggagctcatg gagacccagg 15000
tgaccttaaa acaccccagg gccggctccg gccccagaga gatgtggtcc gagtagaggg 15060
gggtcagcag tgacctggtg ggtgggtgta tttgaggggt gcctgtgttg aaagctctgc 15120
agtagattct aaggagcagc cagggctgag actcccggtg ctgagcccaa ggcccctttc 15180
cttcctgcag gagttcatct tggctcttgc agccccccatg accctgtggc gctgggggc 15240
ggctagcatg atgctcccca tttttcagga gggaagctga ggctcagaga catcatgagg 15300
atgccagctc aaggcccagc caggccttgg tcctcagctg ctgtcatcca gtagggtaag 15360
tgggaccccc aggaggtgtt gggggacact cctcccctcc cccacactca cagtctgttc 15420
accgcccaag gcagcaggct ggactcggtg cagggaccat ggcttggcgt ggccaggttg 15480
gagtggcccc tgggactgaa agcagggggc gtgggggctc tgaggcgggg ccgagtgcgg 15540
ggacgtcctg tggcgggtac cctgctgggt cctgggtggg ggccaccacc aggcagctgt 15600

gggaacattc cagaccacct aggagtaggg ccccttcacc gtttgaagct gactgtaaaa 15660
aacatctgac ctgtgtgcaa agctcaagcc cagaggcctc cagggtgtcc agcctccagg 15720
gtgcctccta cgcagcctcc acgtccccat ccactgcagc cctgaccat cgggcacacc 15780
gtggggcctt cgctggccgc cgggcctcag gttctgctgc accaagtgat ttttaaagcc 15840
ccatctgcct ttctccactt ataaagaact tgggacattt cctcgaggtt gggcattttt 15900
ccagatgtgg tttcagtgtc tcgcttgcta cctggtcccc tgtgctgggc ggtccgctgt 15960
gacgcacctg gcagtccttg ctctaggatg gattcgggcc ggcccttgtt ctgagaacag 16020
agaacggccc ttgttctgag gaggctgggg tctggaatct gcttcctggt cccctgctgt 16080
gtgcagacag ccaccgtcac ccaggcccag gggtgcctgc aggtgctggg catctcaccc 16140
tggcccccac aggggctccc catgccctgc accccctcggt ctgtgccggg gacttccctg 16200
ctctgctgtt gcaggcctcg tcctttgccg agggggtctc tttcccacgt gtccctgaga 16260
ctcctcagcc atcccagagg ctctcggccg tctgagcacc cctgaccatg aagccttcct 16320
ggcccactct ctgtgtgtga ggcagagccc atccctacca ttccgaccca caccagaggc 16380
tccggaaggt ttgctgactg acctgtgact gcaagaagct gaaggaatgt gggcgcagac 16440
ccggcagggg cttttcttgg tgcaaagtca actggctgtc catgcaagcc tgctgctgtg 16500
gccagccctc ccgccaggcg cctggtaccc tccctggacc cagttgccag gacagtgatc 16560
cccagctgtg gccaggactc tgcccggggc cctgtctact cagtgtctcc agtggtcccc 16620
atgcagaaac tgcccccctt gctgcccatg aggcatggga tagggtcagc atccgagagg 16680
caggccctgc tgttcccctc tgcacagagg ctgggggagt gagttgcccg ggcaagtccc 16740
cgggcaggca ctgggcctcc tgagcaggtg agctgagaag gccccagctg gtgtaggcct 16800
cgagagctgc aggactggcc atggggcaga cctggcattg tcattctaga gctgggcctg 16860
gggggtggct ggtgcagtgc agatctcagg tggaggccac ctgcccgtcc tttccacgtg 16920
gctcagggct cagacccacc tgcctgtccc tggaagcccc tagactgctc agagcacaga 16980
cctcagtggg aaagacagta gataggcctg ggtatggtct cccagccctg agaagtgggg 17040
agcccactca gacctttcca ggaccccagg tcccctcagc agcccacaga gcgtgggtgg 17100
agcctgggct ggggttgggt cacttgggag ccaaaggccc atttctgctg cttccaggga 17160
gtcccaggga gacggcagag gcagtgcctg gctggggccc tggttggcgt gccgtttgag 17220
gggtgcatgc actgggaagt tggccaggcc tgcccaaatc agggggcttc caggaggaga 17280
gacatcagga ggatgggcac agaggcggct tagcaccagg gctggcacct ccaggtaggg 17340
gctgtggggg gctggaccag acagggaggc tgtcacgatg cctggctgtt ggatgggggc 17400
acagaggctc tgagtggggt ggcagagctt ggccaccagg tccatctgca ccccccatgc 17460
cccctcacta cccccagacc cccatgatcc caaactactc agtcccaggg acccctgcag 17520
agcctgtggt gggggtgatc ttccaccctg cctggcccct gcccacctca cctcagaact 17580
ttctccaaaa agtaagagcc ccgggggacg aaacttcgag gctccacccc ttgggctctg 17640
gtacaaaccc cgagcctcct ccgagggctg agtcactggg aaagccggga tgggctggcc 17700
cctccccctc cctctccttc cttcctcctt cccccttct cggtctccct ctttttctct 17760
ttgctttact tctctttctt gcctccctct ctccctctct tctttcgttc ctttcatctt 17820
cttcccttcc tccgtctttg cctttcttct tttctgaggg taaaaatagc tggaaaaccc 17880
ccttaaaaaa gtttatttta atcttagagg gacaatgttg gtgtcccaaa gcacagtcct 17940
ttgccccgga ggagccaccg gcagagccag gagcccctga aggactttga ccacaccccc 18000
caccccaccc cgacctgtgc ctggtgcccc aagtggagcc tgggccatcc ttgacctcac 18060
cgctggggtg atggagaagc agccgcccga ggcgagagtt tctcaggtgg ccggggtttc 18120
cccccatttg ctccagacac atttaaacaa gctgagaact tccgtccccc catactcccc 18180
cccgcggacc cccacggacc ccctctgcaa atgaacatgg gcctggaaat cggggtgtgg 18240
gttacagaga gcacggggaa ttctgctcgt gaagtaacac ggagcacgaa cataagcagg 18300
taccgcacat ctgcgagcta ttcgggtctc cgggggaagg caaaaagccc caacaagcct 18360
taagtaagac caggacgctt ctatagtttt gctttgacag ctaaggaaaa aatctgccaa 18420
aaaccagagg cgctgctgag tgttggcgtg ggaatgctag tttagtcaga gccttgctct 18480
gggccattta aataaaaaca gacatgttgg tggcaaatgc cagttgaaca cgcaggagag 18540
cggtgcttct cgggcccgga gaagggtttg cagctgcact ctctcttccc ttttctcttg 18600
gcctccccag ccttcttatc atcacgatca gcatttcact ggtaaacatt tggtgcaata 18660
attttcaaaa atcagcaaga aaagtctgag cgtttcatgt ggctgtgact gcgtggcccc 18720
agctggaagc atggattccc agcgcctccc agagcctgtc tccgagggat actctaatca 18780
cttgagggcc tttgcacgac ataaatcaga ccgactatct ctagagtgga gagggggata 18840
aactcacttg tagcaagtga aggccagagg ctgccctggg atggcattgc ctgttcccag 18900
gggcttgtgg tggtgcagca gacatgggga cccctgggcc ccttcccctc cttgctgggg 18960
atgccacaca gacccacgtt cagggacctg aattcagagc ctgccccagg ggtggcgaat 19020
gtatctggga cactgaggac ccccctaccg ggtgcatctc atggcctttt ggtctttttt 19080
tttgagacgg agtctcactt tgttgcccag gctggagtgt catggggcga tctccgctca 19140
ctaccacctc cgcctcccgg gttcaagcaa ttctcctgtc tcagcctccg agtagctggg 19200

attacaggta tgtgccacca cgccagctaa tttttctatt tttaatagag atagggtttc 19260
accatgttgg tcaggctggt cttgaactcc taacctcggg tgatctgcct gccttggcct 19320
cccgaagtgt tgggattaca ggcgtgagcc actgcacctg gccagccttt tggtcttgaa 19380
gggtctctgg gagggcctca gagaatgtct tggtttcctc actcttgggt tcccatcgac 19440
tgaggtcagg gctgggaagg ggtgatgagc ccaggactct tctggcgaga gcgagggtct 19500
atgttggggg gctcccttct gccacctctc cttggtgaca tggggctggc tggagagtcc 19560
ctccgtgcct gggagttcct cagtgtaggg ggggaagggg gccactgcaa tgaatctgac 19620
accagccccc tctctctccg ccctgctggc agccctcact catatggggg gcgggtctgt 19680
cctcctttcc ccaaagctgg gggtcctgag tgtggttggg gtaacgtctg gtcctcctta 19740
gaacagtggg gcttggaatt cattcaaggg aagaagagtg aaggaacgca gatgccgact 19800
gcatggagcc cccccgggct gcagccccta gagcctgggc gctctgtgag ccagaaagag 19860
aacggctggg gagagggctg tgtgaggccc tgttcttcca agaccgctgc ggaggaccga 19920
cggggcccag ggaccctgac tgaggtgtgc cgagcacagc agctctggac aggccgcacc 19980
ggcctctctg tctgtcatct gtccacctgt tggtgggcag gaacagacct gatgctgtaa 20040
gaagaggccc ccaggaccct gcctctccca ttggctgagc ctggactgtg gagccagccg 20100
ccctcacact gggcacagat gaccgactta ggttttgaga agatccaaac agcagcagga 20160
cgtgcacctg gtaggggtag gagggcctcc cctggactca aaagtgtatt cgcggagccc 20220
cttccctgca gcgtaggctc tgggggggaca gtggccagga cggaggccct cgtggaaagg 20280
gggagatgtg aacagtgagc ctcctgcatg caggcatctg ggaaagaaca ttccagccaa 20340
gtggctctga gcacaccccg accctggcca ccctggccct gtggaccaac cctggggccc 20400
aggattaatg acagccagtg ctcgctggtg cctccagcct gggccaggcc tgtgggctgg 20460
tcccttcaat gatggggcaa gctcagcccc gtccacaccc ttctcctcct cctctgtgct 20520
tccctccacc cccaggagtg ggctccaggt ctgtcactcg tcactcatgg ggggctgtgg 20580
gttgactcgt attccccccaa aagactcgct gtataccccc agagcttgtg agcaggacct 20640
gttttggaga tgggtctttg cagatgtcgt cgagttaaga caaggccatg ctggagtggg 20700
gcctgcccta atccaacgac tggggccctt ggcaggagag ggaggtttgg ggagaaggcg 20760
ctgggaagac aggcagaggc tggagacagt gcagccccaa gctgggagca caggggttgc 20820
cggcacccaa cagaagctgg aggaggtggg aaggatcctc cctagagcct tgggaggggg 20880
cccggtcctg tgacacccag attttggatt tctggccttc ggaactgaga gagcacacat 20940
ttctgcggtt tgaagccacc aaatttttgg taatttgttc tggccaggaa attaatacca 21000
gggataaagg tgttttaatg cagacaaaga cagtttttga attaaccaag ctttgcgcaa 21060
ggctgccggg attgactggg ggaagtgagg gactcagcag cttccccgac gctcgccagg 21120
caccggcctc catacacgct tgctggcggt ggctacggca ggtcgctctt caggtgaaac 21180
cacataatga gcagccttaa gcctgtcgat ttacacagca gaggctgcct ggcctggcgc 21240
tcttcctggc tggaggactc tgccttcccc ctctgcctgc ctgtggccac tccggctgca 21300
gaggagactg gcagacgacg ttctcatccc tggtcggggc aaaagttctg ctcaaatatg 21360
acaacggtga aactcggaat agcgacagag ccaacccatg attgaggcag ggctggaggc 21420
tttctggaaa acgcacggag aaacacgagg gcccagctgt gccctgggcc gccccatgag 21480
tccagccttc ttcgtcctag tcccttctcc aaagaaagga caaaaccact gaaagggttc 21540
gtctgggtca caaacgtccc aaattggctt ccgcgtggaa actaaacaag ggtaggtccc 21600
agcccctggg attcaggtga aacccgaggc ctgtctgagg tcacagcgtc ttcccgagga 21660
cgtcagccct gttggctgtg tctggggcca ggctgaggca tagcgggcca ggaggccgag 21720
cttcccaaag gagctgcact gtcttttcct tagaatatga aatcaatgtc tgccaccttg 21780
aatcctcatg gacaagactc cttggaagcc agcctgtgtc agggccagcg tcccccaggg 21840
ctgacaccta caccctcgca ttctcagcgt gactcgggac agtggcagaa cccgctgggt 21900
tctctctacg ggagtgcttt ttttttttct tttcttttt tttttttttt tttttttga 21960
gtcggagttt cactcttgtt gcccaggctg gagtacaatg gtgtgatctc ggctcactgc 22020
aacctccacc tcctgggttc aagcagttct cctgtctcag cctcccgagt agctgggatt 22080
acaggtgcac accaccatgc ctggctaatt tttgtatttt tagtagagat gaggtttcac 22140
ggtgttggcc aagctggtct cgaactcct 22169

<210> 42
<211> 3286
<212> DNA
<213> Homo Sapiens

<400> 42

agaacccgcg gctaacccca gaggactggt gagggctggc ttgggatgaa taaacactcg 60
acacctgacg gtcctttctc tgcggtgact ttcaccaagc cgaagtggcg cgcgcgcggg 120
caggcgggag ccgggacctg gcccgcgcgg cgccgcgcac ccggatcccg aggctacgcc 180

```
ggcgagcgcg gcgcgggggg cggggagcgg gacagcggga ccgcccgggc caagcgattg      240
tttcaaagac attttttatc aagacccttt tgtattcgag ccctacctct ttttggccta      300
ggaaagcttt cgtgcaagtg gaagacgact ttctccacaa agtgctgtat gttactgtgc      360
tccggaccgc gtacgaacac catccagtcg tgggtgaagc cctccacggt gggttttttc      420
ctcacctggg cgcggtgccc cagctccagc ttcacctgca cggcacactg cgggcagggg      480
gaggagagac agccgtgaat aacaggaagg cgaggtttcg gcagtgaacg ttgcgcctga      540
catttttttc ctccttcttg aaacgcacat aaaaggaaac ggcggtgccc tctgcatcca      600
cgtttcacgc gcgtgggcgc gcacactcca cacccccaaa tatacccgcc cgcccggccc      660
ggcttggccc caggcgcccc gggccccgca tctacatcgg acaggattgt aacggaatgt      720
tatccccagt cgggaagggg gtcggggaaa agagggagaa cacactcagc cacgacaagc      780
acgacaacaa atgcatcgga aacaaatcag aaggcgatgc cggggcggtt tcccggcgtg      840
ggaggggagg tggctgggac ccagggggac cgaagggctc ctggcgagcc ccctccccga      900
aagtaccgcg gcgacaggca cacgccgagg aagtctttgt gtacgtgtgt gtgcgtgcgt      960
gtggagcgct gtgccaggcg aaatggaaac tacaggcagc ctctgctgat gggcacaaac     1020
tcccgtgctc ttgccacggg gttcgtgcac aacaaaaatg agacgtcgtc atacacactg     1080
aaacacacac acacgcccgc aggaaaacac gccgaggcat ccataactta gagcaccccg     1140
caccccccag cgaaaagccc cacgcgatcg gcgaggccag tcgaaccgag cccccgcaac     1200
acactttcaa gagcccaagt ggctccaaag tatctcccac ctccccccaa aaaatgaaat     1260
tcagaaaggc agggcggcgg gcggacagcc gccgagcctc ggctcgcgct cagcacctcc     1320
cggcgctggg gcaaagttgc gtgcggcccc gccgctgtca gccccgcaca cttcggctca     1380
cacacgcgcg ccgcggagaa cgcaccatcg tagcggccgc ggcgctttgc ggaggtgcgg     1440
ccgccgaggc tgctcgccgc gtccccggac tgtgcccgca gctcccggcg gcggcggctg     1500
aaatatggct gagttattat tcgcctcctt ccaccgtgtg tgtgtgtgtg tgtgagtgcg     1560
cgcgtgtgag cgagagggag tgtgtgagtg cgcttcttgt gactgcaaag aggcgaggag     1620
ggagggaggc gcggggggtg gaggggcgag tgtgagcgtg tgtgagtgtg tgtgtgtctg     1680
tgtgtctgtg tgcgcgccgg ggggggtgg gggaggaggg gagcggaggc tgagggagag     1740
gcggcagctc cctgcaagcc gtaccaacct ttctctaatt ggaaccgcgg agcgctggcg     1800
ctgtctgggc tgcggcggcg cagggcgagg aaggaaagtg ggggagggct tttattatta     1860
tttttgcgcg tacttaccga gctagccatg cctgggggcc cggaggtttg ctggggtgtt     1920
gtgtggtacc ccccctcct ccgccccccc tcagctgtaa ttcatgaaga ggctgctatg     1980
aatgagagcg cgcccaggag cggagggtag atggcggaca ttctctgcct ttttcccccc     2040
gcgctcgctt gctcgctcgc tcgcttatta aactcagccc caaaagcaaa agcagcagca     2100
gcagcagcag ctccagggta aagaagatga ttgcggagca tgcgccgtgg cgcctgacac     2160
ccgggctccg agcctcacag ggcggaggga gggcttgcgg gcggggcgga gcgcgcgggg     2220
aggcagagcg agcgagccag gggagctcg aaagagccaa tcacctgcca cctcccgaaa     2280
ccccggcggc ccgcagcgcc acactggaaa gcctcgctta aagtaacagg ttcctgctgg     2340
gggggtggca gggaggggac gcaggagtgg ggagagcggc cagctgggga gccatcccag     2400
cccctgtgta aactctgctc gccctacaat aaaaaacgga ttaaaactgc tggaatgtag     2460
ccactgctta gagccctcag cagccggaga gggggtgtta aatcaagtcc ttagctagta     2520
cgcacgggga ggagggagtg ggctttcttt tactttccac cacgtatttc tgtggttttg     2580
tgctgaaaag acaagaactc acacccaaac aaacttactt aaagagagcg agagaaatgc     2640
catcctgcaa aaaacgagca tgaaaatagc aatgactgag gctgttctaa cccgcaacga     2700
tcccctggcc ccctgcccca tccactcaca ggcacccgcc cgccaccctc actccccgcc     2760
cttctgccac cgccaccgaa aatttgaaaa tgaaatgaac aagggagaaa caaggatgct     2820
atttccttgc aagggaacgc cttccgcttt ggtaatcccc ggctgggaag cttcataaag     2880
cggtgcccag gtgaaagcaa gacacctggg agtgccgggt acagaccctc ggggaccagg     2940
cttggagttg ttttcttcgg ggggcttccc ctcctgacag cctactgagt tgcatcagaa     3000
cgtggaggat tgtcctctga ggacgcgcgg ggcaaccggg agtcttaaaa tagcagataa     3060
agttaatact gactgtaatg tgcgtaaatt ggattataat ctttaggcgt attggtgaca     3120
accagcgtaa tccatctctg agtattaatc cggttagact cccgggtgac agccagtcgc     3180
aggagtgttc ccagagtcgc gcctctgcaa atgttctcac tcgcgttgta tttgatctca     3240
gtggccggag atagaacagc gcggccacgc gtggggcgcg gagagg                   3286
```

<210> 43
<211> 23717
<212> DNA
<213> Homo Sapiens

<400> 43

```
tccctgctgc ggcatctcag catgacggag gcccatcctt ggctttcctg gagcatgaat       60
```

```
atcctctgag gagcactggc cctggccttg taggtcttgc tgagactggc ttagattttc      120
tggcccagga gcaatcgtca cccaacctcc cacttgtaaa tctcgccaca gaatcggccc      180
acaaccgtct ctccggaatc tcggcggacc agccctgctc ctgtgtctcc ctctcccgca      240
gagccactgc cttctcaggc tcctcaagtg cacctaagag cgggagtctg gctgccctca      300
gcactcgcgt cccgccctgc agcctgataa gcttgacgcc aaggcattct cccgagagcc      360
ggcccagcag ctgctggcag gagagtggcc tcagggcagc gggaagggga cctccgggga      420
gagaatgagg cctgcgcaag ggtcctggaa ggacccagga gtcgccccgg cttccctccc      480
ccaggccctg gttgggcgcc gtgggccgcg tcccagttga gcccggcgtg gccgtttaat      540
ggcgtccctc cgcgaccgag gacgaagggg cctagagagg ctgtgcccaa gcgacgctgc      600
gaggccaggc cgcgtctcgg gccagggcgg tccccttgcc accgccgctc ttggccgcca      660
ggaccaagca cagcacgcag cgtcgcgaaa ggggcggtcc tggaaggcgc gaaggggtct      720
ccgggtctcg cgaacacggc ggggtcgggg cggagtcgtg ggtcctcggt cgagcggccg      780
cccagagccc cgcgggacac ggacggcccc agcccggctc cccgcgagcc ccacgcgtct      840
gtgacgccgc tgatgcgcag acgccgcggc tgcaggcccc gcccctccgt gcccctcgca      900
aggccctctg ggagttgtag ttccagctcc tcctcgggcc gccccactcc ctgataatcc      960
tggagcgcgg cgcctcccgg ggaccgaggc ctcgggcccg gccgtctcca gcagcgaggg     1020
caggagaagt ggcccgggcg gtcggcggac tgggagggcg ccacccgagg actacaactc     1080
ccggcctgcc tcgcgcaggc accgccccga gcttccgccc cgtcggcgcc gcgcagccct     1140
gtgggagccg tagtccgggc gcgcgattca cagcgccgcg tgcgccccgg gcgcgccgta     1200
gccgcggggc ccgggccggg cgctacgcta cgaaggcagc gtaaggcggg cgccggacgc     1260
gggcaggggc cggggccgga gccgggtcgg ggcgccccgc ggctgaggag cgggaggccg     1320
tccgggcagc gcggccggcg gcgagagggg ccccgccgct ctccggaggc agaagttgtg     1380
gatcggccgg cgggggcgag cgggcccggg ggccggggcc gcgctgcccg ggccgcgagg     1440
acgaggcggc gccgccgggc tgtggaggtg agtcccgtcg gccgggcccg gccgggcgcc     1500
cagccgggga ccccgggcgg ggagcgcggg cccggagggc ggcggggagc gcgggctcgg     1560
cggggcgcag gaggcctcgg gggccgggcg cgccgggctc cgagccaggg cagggacggc     1620
agggcccccg gagtggggtc agcccgccgc ccgctggcgc ctccaggctg gtgggggcca     1680
gggctgggcg atgcttcgca gaggaaccca gaggaaggcg ggaagtcggc agggacctgg     1740
gccatgtggg tcacttgagg ccagccccgg gggccgcgca caccggaggc ggcctcggcc     1800
ctgcctgccc ttgagtgccc ttatatccgg gcccatcgtg cggtcctggc cgccttaggg     1860
atgttgtgct tggtgcgggc atcactcccc tcacttgggg aacggcctgg gggcggccac     1920
ctggcgtgga ggggctggcg ggagccccgt ctgtgccagc catgccagtg ttctcttccg     1980
tgttcctctg ggctgctgc ggcaccagct gggaagttgc aaatctgctg tcctcccgct     2040
ggctgcagaa ggggcccctc tagtcctgac cgggggaact cttgtgtggt cagctggccc     2100
tggccgggcg agctctcggg gccccttac tgctctggag tttgttaccg gactgcaggt     2160
aacaagaccc ggaaagcaat ggtgaggccc agaacctgag gccaggcctg ggtgtgtggg     2220
gccagtgggg aagactggga ctccgttggg tgctgaagag ttgtgtgctt ctccttcggg     2280
aaacccggat ctcttcctct gcccaggtct gaggggtgcg gaaacgtgag tcacagttca     2340
gtgtgggagt gccccgtaat tgcctccgcg tttccagagt ctggggctgg gcaaggatat     2400
aggggtgatt ctcccttccg gttctgagtg acttctcaac tgtgaagtgg ggcaagagaa     2460
attgtttttc tggcctgtct gggggaggca ggtcctgcag gaaaccagct tggcctggcc     2520
ttgggttggg tgtgccttgc ctcctccgag gtccttctgc ttgcgggagg agcacccctc     2580
tttttcctgc ttctgtctgt gtctgaggct ctccagagct ccttgattgg cagaggagcc     2640
agtgacctct gagtccccag gagagcccga ggctcagtaa ctgcataact gcgcagggcc     2700
tcttgtctgg gcctccacag accgtggtct ctgagagcag gaagtggctg gtcccctgta     2760
atgctgcatc tgtgtgacag cctcaagccc tgctggctct ctctccgggc acccctcact     2820
tgcttcttgc cctggccttg ggcagcactc cggtaacccc agtgccacag gtgaggaacc     2880
gaagctggag acagctgttc atggcagggg aggggccgag ggaggctgag cgcctggtct     2940
ctccctcctg ctggttccac cagttcacac gaggcaattg gagggaagga ttctgctgcc     3000
cggaaaatcc tccggaacca caggtgttgt tcatgggcct gagccgctca gtgaagaact     3060
cggccgttgg tggccgagtt gcccctggtc cagccctctg gcccgcggtg tgatgcctct     3120
actcatggca acaggcgtgg acaggtgctg agaaggagga cgacatcatt aaacaaaaca     3180
gcttggggtc tttgaaaaac agtgacagaa acccaggaag gctggtgact tgtcaggtct     3240
gtgtctgtcc tgggtggcca ctcggccctg gaccgtgagg ctgaaatgac aggagacacg     3300
gagggtgggc ttgggtggtt tcctcataag acacagaaga aggaggcccc caacccgggg     3360
gctgcctcgt gcgctttgct ctcctgtttt cctgctctgt ccctctggtc ctgccctctg     3420
acccctggca cagcaggagt gcccagaagc ccagtgggac ttgttctctg cctaaagccc     3480
ttccgcatcc ctcgtaccca ctcggtgtgg atgtgggttt tctcaccacc aggctgtgcc     3540
tcagctgtgt ggagccactg gcctctcatg ccccacgctg gagagaacgc tgaggtctgt     3600
gcggttctca tgtgaccctg tagctggtcc ctggctcccc actgtgggat ctgcctgcag     3660
cacttacact tgggaccgcc ctgtctccct gctggatgtg gcgccgaggg cagggtccgt     3720
```

ttctgtttct tggtaggcct agtgtctagc gctatgcagc cgtgcgcaga gcaggcggtc 3780
agtgagcacc tggggttggc cctcaccaca ggcagttctg aatacctggg ttctggcagc 3840
ccagggggac tcctgcgtgc tccaggaaag tagcacagca gcccctgtgg cgtggacacg 3900
gccacacctg agctcggcct gtttctgagt ctgagagaac aggcttgctg gcctgtgatc 3960
tttgcatgtg tgtctcgctg cagggaggct gtgggttggg gctggaccta gacccgttca 4020
gccacaccct gtcactatgt gggtcacaca cagctgggtg gagacctccc ggtgcgtggc 4080
ccagggaggt gtctgggcgt ggagtctgtg gacagccaca ttgtaccact aggacagggg 4140
ctgctgctgg gcccacaggg tgcacggaat ggggtctgtg ggaggatggg ggcatcctag 4200
cccagggcag ggaccccgtg ggcccccaa gatgagctgc gtcaggctgt tgctttgcac 4260
actctgaggg gcctgcctga gtgctgcatg ggaagccagc agtgtggccg ccctccccag 4320
aactcatcca gggcaccagg gtggacccat gagtcccgtc gggtcatggg catcagcctc 4380
gccatcctgg cctccagata caacgatttg tcagattccc tgaggtcaaa gggaagcttc 4440
aagggttgcc atccccgcct ccctggcggt cctagacggg atgtcaggag gacgcagggc 4500
catcgctgct ttttaaatgt cacagtgact gcagcgtctg ctcctctgtg gaaggagctt 4560
gctccgctaa tgagggagtt cctcctcatg gccttcccct cacttctctt caaaaagttg 4620
ccctttattt taacattttt taagttttat ttttttgagc cagggtcgtg ctctgttgcc 4680
cacgctggag tgcagttgca atcatagctc gctgcagcct caaactcctg ggctcaagtg 4740
atcttcctgc ctcagcctcc caaatactgg gactacaggt gggcgccacc acacccagcc 4800
tctgcccttt gtaagtgaag tcccccaggc aggttgaacc tcttacacct tctgcccctg 4860
cctgatcccc actccctacc ccccacctcc ctgtcccctg taatgagaaa ggcaataagt 4920
tttggagctt tctggagttc ctgcccctcc tgaaaaggtg gctgcagaga ggctggagct 4980
gatggggaaa gaggtgccac gaggcctcct ctgggttagg gccacacggc tcccaaggcc 5040
cccgtctcct tctctggggt gacaggtgcc cccagggcca cacggctccc aaggcctccg 5100
tctccctctc tggggtgaca ggtgccccca gggccacacg gctcccaagg cctccgtctc 5160
cttctctggg atgacaggtg cccagggcca cacggctccc aaggcccccg tccccttctc 5220
tggggtgaca ggtgccccct gtgtcccaga ggcttctgct tggccaccag tgaggtcacc 5280
gggctgccca gaccagggct gcttctgcca ccacatggca agcagagaag tagagaagtg 5340
ccaaaaacca cgagtgcttg tctggtcctt ttttcctgtt aaaaatagaa gtcggggctg 5400
ggtgcggtgg ctgacacctg taatcccagc cctttcagag gccgaggcag gtgggttgcc 5460
tgaggtcagg agttcgagac cagcctgacc aaaatggcga aaccccatct ctactaaaaa 5520
taaaaaaatt agctgggtgt ggtggtgtgt gcctgtaatc ccagatactc aggaggctga 5580
gactgtagaa ttgcttgaac ctgggaggcg gaggttgcag ttagccggga tggcaccact 5640
gcactccagc ctaggcgaca gagctatact ccgtctcaaa aaaaaaaaaa agaaaaaaaa 5700
gtagaattcg gttgggctca gtggctcatg cgtgtaatcc tagcactttg gaaggccaag 5760
gtgggtgaat cacatgaggt caggaggtag agaccagcat ggccaacgtg acgaaacccc 5820
catctctact aaaaatacaa aaattagctg ggcgtggtgg tgtgtgcctg taatcccaga 5880
tgtttgggag gctgagacac tagaattgct tgaacccggg aggcagaggt tgcagtgagt 5940
cgagatggca ccactgcact ccagcctagg tgacagagtg ataccctgtc tcaaaaaaaa 6000
aaaaaaagcc agccgctgtg gctcacgcct gtaatcccag cactttggga ggtcaaggcg 6060
ggtggatcac ctgaggtcag gagattgaga ccatcctggc taacacggtg aaactccatc 6120
tctgctaaaa atacaaaaaa attagccggg cctggtggca ggcgcctgta gtcccagcta 6180
ctcgggaggc tgaggcagga gaatggcgtg aacccaggag gtggagcttg cagtgagccg 6240
agatcacacc actgcactcc agcctgggca acagagcgag actctgtcag agaaaaaaaa 6300
aaaaaaaaca ggtgatgcct gtgccactgg acacacctgc acaccatcct catgctgatc 6360
acaaaagagg gtgaggggcc gggcatggtg gctcatgcct ggaatcccag cactggggga 6420
ggccgaggcg ggtggatcac ctgaggtctg gagttcgaga ccagcctgac caacatggtg 6480
aaacctcgtc tttactaaaa atacaaaaat tagctgggca cggtggcggg cgcctgtaat 6540
ccgagctact tgggaggctg aggcaggaga atagcttgaa cctgggaggc ggaggctgca 6600
gtgagctgag attgcaccac tgcaccccag cctgggtgac agagcgaggc tctatctcaa 6660
aaaataaaat aaaaaattta aaaccaacaa aaaagagggt gatggctgag tgaggagtcc 6720
atggtgggcc ctacacaggg cccaggaagg acagggcggg aagcgtgagc atcctgccca 6780
ccgcagtggg tccgccatgg agagcatgac cttagagaag caaagccgcc tggctcgaag 6840
cagtggccgc cctgctgaca gctgtagtgg ccgggtggca ctcgggctgg ggtccggggg 6900
agtctaggtg gccacctggc tgtgtgcctg tgcctgccca agctgggcac ctttccctgg 6960
cagtgcctgg gaagctcggc cgccctccct ggctggtgct gctctgcagc cccgctcatg 7020
gactcttgcc cacggcctgc tccttgtagc agggtcgggc cgtgggaggt ccggacagga 7080
tcttgaatgg ccctgtctgc ggtcctggct ggccggaggt tgaccgcact ggacaggtca 7140
agggccttaa accagggtag tgaggagcct gctgagcccc tgctgctccc cgggctgcct 7200
ttggcgctcg gacagacacc caaggacaga agctcggaca gcttctaagg cgaagggtct 7260
cccggggctg atttcccggg actctggctc ctgcagagag ggctggaggc ggggctctgt 7320
gttccagggt cagagtcctc ctggtggaag ctgaggtgcc tgattaaatg cctccctttc 7380

taggcagcag cccagacgcg gcacagagag ggcctgggag cttgctgcag cttcaggtga 7440
gtttgttccc aagccctgcg gctcgtttat gggtttgctt gcgctgcaga actgtttgtg 7500
gagcagcagg gttggagtgc ccacggctgg ggggcctctc ggagcccttg attcacagaa 7560
cccctggagg ccggcagccc tgcctgccct gaagagcctc gttctcccag ccgtttgggc 7620
cgtggtcctg tcgtgggagg cgacggtgag ggcggcccgg ggtcccgaca ggggtgtttg 7680
tccctttggg gacctcccct gtgctgtggc cccggccccc agatggagct ccccatcccc 7740
acggcagcgc tggggctggg gctggtgagg agcctgtgca cggcccccac cgctatcctc 7800
agccaggagg gtgcctggtc cctggggagc ggcctgcacc tgtttctgga gaatgctgtc 7860
ctgccagttg tcatttgggg cgggcggtcg gagtccacgt cctcccccag ggcggggccc 7920
tggccttgtt tcttgtaggg acgatgggaa gccctgtcct cagccctccc acccgactca 7980
tgcaggagct tcctgtcctt ggggtcatct cctccacagg ggccttagcg tcagtgggga 8040
caaaggcaga gttgcctgtc ttcaggttgt gggcaccgac tgagcttcgt cccggtagtg 8100
gctggctctt gtttcctgcc tggaccagca gcctctggtc tggtgggtgc agttttaggg 8160
cgcagtgggg ttgccgtggg gcaaggccag gccaccagct gcctggacac aggggccccc 8220
gctttccatc tgctgccccc cacaccccca gctcgggcag gggtgacacc ggggcatcct 8280
tgttggggtc gctgtggttc ccatggtggc aagatggctc tgtgaaagat tgacggagat 8340
gtagggccca ggagcaggtg ctgtgtgagt gggacacgcc tgtgtcacct gcggggcagg 8400
ggagggctcg ggggtacagc cgtgtacatg attagttagc gagacagtga ggagtgtatg 8460
cggagtggac acagctgtgg gtgccgcccg cgcaaggctg gggtcgctg tgccagccct 8520
gctcccatgt gggctcccag ctccccaggt agggacgctg accccgggag gtgcctttgc 8580
ctgcatccac tggagcctca ggagcatgcg ttccgtcccc acgggtcacc cccactgagc 8640
ccaccaccta ccgagaggca cctcgagacc ctgtcctgcc acgggtgggc tgtcaaggcc 8700
agcaccagag acccccagca gacctcagtg gccgcagatg gagcggggcg gcaatggtca 8760
cctccgggac tcagccctgt gctgagcccc gggcagtgtg atcatcctgg cccttctcgt 8820
gcacgtcccc tggctggatg ctccttgctg ccctcacggg gtgtgtgtgt ggcatacagg 8880
acagggaccg gccagttggc cctgctcatt aaccacttgt ccccacaggg cagtggcggc 8940
ctcacctctg caattctctg aggctggatc taggccaccg ccccgtttaa aactagggca 9000
tcggctccca gggagggcgg ggagctgcac agttggactt gtgggggtca ggcatggatc 9060
cacacagccc ggggccctcc gcaccctgcc cctccaggga gcccagaggc gggcgtggct 9120
gcagcctggc tctgtggcag tcatgagtca cggccactct tgaggcgccg ccctgagagt 9180
cagccctgtg gtcacggctc ctgctggttc ccattcgcga gcacctgaaa cagcctggcc 9240

cacagcagag gcccgggggg cacatgcagc tcacgggagc agcacagatg cttggccggc 9300
ggcaggccct acaggagcag cagcggccca gggcatgtcc ctggggtcag gggtcagagt 9360
tcggcatgct ctgcgggggc ctcaccctgc tgtccctcgg cctggcctgg gcacactcct 9420
tggtgacagt gcccctgcac gccccccccc ccccccggtt attcagacag ggagccagga 9480
tgggaaccac ggactgacct gacctgcgtc ctcggggcca ctgcactggg tgattcacgt 9540
gtgcccaggc cctgaggtgg gccggacctg ggagtggcag gtgagcgttc ctcccaagag 9600
cctctggctg gccgcagcag gccctgtccc tgctgtcctc agtggccctg gacatgataa 9660
ccccccctgcc gaggtctggc cggagccagc acccacggcc gagatgcagt cagccctggc 9720
tgggcgctgt ccctgggggt ggctgctggt gctgccctcc ctccttccca ccacttcttg 9780
ctcctctttc ctggggccct cccatcggag tcttcctcct aaatgcagtt ccctccctct 9840
aggggaacgc gggctgggag cctatttttt tttttttga gacggagtct cgctctgtcg 9900
ccccggctgg agtgcagtgg cgcggtctcg gctcactgca acctccgcct cccggattca 9960
ggccattctc ctgcctcagc ctcccaagta gctgggatta caggcgcccg ccaccacgcc 10020
cagctaattt tttgtgtgtt tttagtagaa acagggtttc tccatcttgc gcaggctagt 10080
cttgaactcc tgacctcgtg atccacccac ctcggcctcc cacagtgctg ggattacagg 10140
cgtgagccac tgcacctggc ctgggagcct tttccgtagc tgctcatcct gtactagcat 10200
ctgtccatca tgagtggacg gaacacaggg ggacccagga cccacagctg cactcgggaa 10260
ggccagcagg gtggatttcg ggaagagcat cggtgccagt ttcccacagt caccccttgcg 10320
atgggaccct caggttggca cgtggctggc agctgtgtcc cgggagctct tgctccttag 10380
tgggcctcgc cagccagggc agggcccccg gcacccctca cggggctctt gactcctctg 10440
gcagcggatc ggggcggttc agcgatacca tctctggaga tcccctggcg tgtgctggaa 10500
aaaccccgga gagggcttaa cagagccagc tgtccagcct ctgtgacagg gacgtttgct 10560
tttctctgca gggtcctctc ttctcttccg agcacccacg ggaggggact gtgctgtccc 10620
tgctctccta ggacaggcct ccctcctgtc cagatggggc ccacagggtc agttgcctga 10680
gactggctcc agctcaagaa aggttttctg agtgagcgca ggggagcagg aggctggtga 10740
gcagctcgct tcctgccctg ggtgcagctg ggcaccactt ctgccttggg cctaaaggca 10800
tcagtgagga gcaggcgggg cctggtagga gcctgtgttt ctagcggcac gcgtgacccc 10860
aggtgccacg tgggtgagtg cgtgggtggg tgagtggcga aggagctgag gttggccctg 10920
gcaggtgtgc agtcctcaca cagaacgggc agggctcctg tcagagccaa accagctcag 10980

ggcccagtgc aagcagcttc tgtcccgtgg cacctccgct agtcgccgct cccacccaag 11040
ccctgccccg ccctgacccg ctcagggttt ggctacagaa ggggagtgtc ggaaatgccc 11100
aacccgtctc ccgtgaggga ctcctgcgtg gcctgggctg gggaacgcag gggccgtttg 11160
ggtggttccg gcgtctggcg tagccacgcg agaggccctg ggttgatgcg tgggctccct 11220
gctagcgagc ctgctccgtg cgtgctgggc tcccggcctt gcttccgtgt ggcattcgag 11280
accccgtgga cctcgcctgc gtcctgcatg tttgagcgct gccccttgtg gaggccgcca 11340
gcactggtgg ctgcagggcc tgtagtgtgg cacgtccaga gacccggcct ggagaacggg 11400
tcctggatgt ctccgtcatg ttttacgttc gcttcacacg gacgcagcga tgtctcagat 11460
acctcggatc acacagagca cgtcgccaag gcttgttttg tctgttcctt tccacctgtc 11520
ttacgtggat accaggagat ccgccacgac tcccgtggcc ccgtccacac tgcgcggcgc 11580
tctccggcgt ggccgccagg tgccccttcg agggcaggtg tgagtacagg gtggtccctg 11640
tggagaagcg cctgcaggcg agtgagccag gctgtgccct gctgaccccg aggcctgaat 11700
ttcatggaac tcccacctct cacagagtat tgtttttttt tttgattttt ttccaaccat 11760
ttaaaaatgt aaaagccatt gttgacttgc gggctggacc aggcttaggg ctgggctctg 11820
gtccgcaccc cacactggcc ccgtgtgtcc tgtgaccgtt ctgcctacgg ggcaggcctg 11880
agccaccaca ggatggcagg gtctccaggg tgcagggccc gcaatgcttc aaccctccca 11940
cgaccccagc accttcagag aaaacggacc ctcgtgccga gtgaagtggt tcaggcgaac 12000
gttatcacca cacgggggac tcggcggctt ctgccccagg cctcaaagga gaggggatcc 12060
agaattctct gtctcacccc ctcagcccct gcccttgaag cttctgtccc caaggctgtg 12120
gggaggaacc agggaagggc tggcgcgtct gctgtgcgat cccaggtgct cgtgcctcgc 12180
tgagctcagc ccctgtggcc tcagagctgc aggcccctcc cgggtgtcag agcactgact 12240
ggaaaggccc cccacatccc tgccctctgc tgctccccat cgtcgcgggg gagaagctgg 12300
aactgcaccc ccaccccccc cagcccgctc acctagggct gcccccaggc ctccctgagc 12360
tccaaaggat atgttctttg gtgctttttt ttttttggc tctgtctccc aggctggaat 12420
gcagctgtgg tgctcacgcc atcctcccac gtcagcctcc tgagtagcta ggtctacagg 12480
tgcacaccac cacacccgga tagtgtttag ttttctataa agacagggtc tcactgtgtt 12540
gcccaggctg gtctcaaact cttgggctca ggtgatcccc cacgcacggc agctttgaca 12600
cttacttgac cctttcctcc taagctctac cctgacgctg gctgtgtgtc ccagagactt 12660
aatcagaagc tggaagaagg gctgtgacgt gtgtgacagc aggaactgct ggtcaaccca 12720
cagccggcca gcctgccggg acgccatctt ggccccaatc ttggcaggga gaggggagtg 12780
tatcggactc tggggagagc agctgcctgc acgggcaggg tcctcccgtc tcaggattaa 12840
cgttctcaac tgtatttgat ttctcctcag ccagaggggg taaggtggcc ttcccagaag 12900
cacaggtggg ccgcgtgtca gaggccctgg acagcctgtg cctctcccag gcctcccagc 12960
aggggtggct ctcgcccctc tggaggggag accccaggaa ccggcccccg ccctcagcca 13020
tagcctcaca gggtccttgt ttctcggggc ctggctctgt gggtgcacaa ggcatggtcg 13080
ggctcccgtc cttctggaag tctccagagt ggcatgtggg cagctgtgtg aagcggcccc 13140
ccagatcttc atccagcctg gcctgcagca gctccctacg ccctctgcta cctcccctgc 13200
ctacctttgg gtagacgttc tcttgaccct tccttctgtg ataaagcaaa agcgtgtgat 13260
ctcttttctc accaaggaat aaaacgtctc tgaataccac agacgtgatg ccgggctttt 13320
gggggccggg gggtgtccta gggccccgag ggaggagccc tgtgggtgcc gaagtcgggc 13380
cgcaggccct gcactgagat gtcccctcag cctaccccag gcggctgggc atgctgggaa 13440
gtcctcccgg gctgtgtgtg tgaatcatgc tgtttcttct ctattttctc tctctgtctg 13500
tttattttg ttgtgtgtca ctctgcccag ccggccgtcc tgtctcttcc ctccatgctc 13560
cgcttcgccc gctgctggcc agaggtgacc aggtccggct ctcagcacgg ccgccggccg 13620
gggggccatg gggagctccg cctcctccct ggccgcagag accgagtcgg accacagctt 13680
ccccggggga ccgccctacc cgggtccccc ggcagcggct ggctggtgta ggagcggccc 13740
agggggggccc gtctggggag gtgccctggt cagccggcag caccagcccc cacggccccg 13800
ggcccgaagg taagaggtgg ggaggggtgg ccccggaggg ctcccggggg cagagggcct 13860
cgttctcagg gtgccccctc cttggcagag gtgcttgcag agaagtgggt ccctgctcct 13920
tccagttgtg gcagctctct tccccagggt gcaggagctg agcaggtctg agaccccggc 13980
ctcggcctgg ctggttggtg gtggggatgg gggctagtgc gcccaatccc ggcggccctg 14040
tgtttctcac aggctgtgct gctccacgca gagaagctgt tcctgaagct tctttttggg 14100
ctggggtcgc tggcggctgc aggctctggg catattgcct gctgcccacg gtttcaaagc 14160
tgccagcact cgctgggctc cgcctgctgc ccccccgggg gtcacaggcc acatggcttg 14220
gtggccgaac tccacgtgct ggctcctcac agctgtcacc atggccttgg ctacccgctg 14280
cgtccctcag gagctgccat caggttctga ggtgcctggt ctggaggcag tccaggtggt 14340
gaggtctgga ctggctggac cccaccgatg ctcctgccgt caccccgtcc tggccctcac 14400
tggaggcagg gacactcagg ggcccggggc ctcggggcca gtgctgcagt ggccgccgtt 14460
gctctcacag cgggtccagg cctggcttct gaaggcaatg tgcctgcgtc tcacactcaa 14520
aagggcctgc caggctgcac ctggcggcag ctcccacggg ggacgatgtc ctgctgtctg 14580
ttggccccca ggtgggaggg acgggcgtgg agctgcaggc agtgggcaag gccatggcag 14640

gtggccaggc ctgggaacca gcctccaggt ggcatccacg ctgaggcctg agctccggcc 14700
gacaaggact ggggatgcag ggattagcc aggggctgtg tgggggagcc ctggagccct 14760
gtctgcgagt taccccgatg aacagggaga cgcggcagag gcagggccgg ggaggggccg 14820
tgtgggggag ccctggagcc ctgtctgcga gttaccctga tggacagaga gacgcggcaa 14880
aggcagggcc ggggaggggc cgtgtggggc ctggtggtcg gcagaggaag ggtctgtgct 14940
cctcctgcgt gtggtggcac cggagcgcct cccagttcct ggtgttaccg caccagccgc 15000
ctcccagtga gcatctccac gtgtgccgac aacagctttg ttgaggtaga acttaccaga 15060
ctcacctgtt aaaagagtgc agtttggtca ctcttagtgg tttgtagagt tgtgcagccg 15120
tcattgcaat ccagcgttgc agcattccac agcccacagg gacccccagc cccacagtcc 15180
agtgttacag catcccgcag cccagaggga ccctcagccc cagacggcca ctgaccggct 15240
tcctgtcttg gtttgccttg tgcagaaggt gcgtggatgt ggggccacag gacgtggctg 15300
gctgggtttg ccttgtgcag aaggtgcgtg gatgtggggc cacaggacgt ggctggctgg 15360
gtttgccttg tgcagaaggt gcgtggatgt ggggccacag gacgtggctg gctgggtttg 15420
ccttgtgcag aaggtgcgtg gatgtggggc cacaggacgt ggctggctgg gtttgccttg 15480
tgcagaaggt gcgtggacgt ggggccacag gacgtggggc cccgtgtctg gcctcttcac 15540
tcggcaccgt gccctccaga ttcatgcacc tgcagcccga ggcagtttca ttcactgctc 15600
ctttgcggga tgaaccgcat tttatctatt catggacgct aggactgctt ccacgtggct 15660
tctgtggaca atgctgtgaa cattgtttac gaggttttgc gtggacctgt tttcatttat 15720
cttgcataga tacctaggag tgggattgct ggggcgtgtg gtgaactttc ataatgtctt 15780
gaggaactgc caagccatct tccgcagcgg ctgcattacc tagttcccac cggcaacgct 15840
ggggagggtc ctggcgtctc cactccctcg acagctcctg ccgttgtctg tccttgtgat 15900
tctggccgtc ccagggggtg ggagctggtg tccccttggc tttgatctgc gtttccctgg 15960
tggccagcca tgtggggcct ctctccctgt ctcatccacc cgtgcctgtc ttttttttcat 16020
tcattcactt atttatttat ttagagacag ggtcttactc tgtcacccag gctggagtac 16080
agtggtgtga tcacagctca ctgcagcttc tacttcctgg gttcaagaga acctcccatc 16140
tcagcctccc aagtagctgg aactacaggc gtgcaccacc atagctggtt aattagaaaa 16200
aaaaaatttg gccaggcgca gtagctcacg cctgtaatcc cagcactttg ggaggccgag 16260
gcgggtagat cacctgaggt caggagttca agaccagcct ggccaacatg gtgaaacccc 16320
atctctacta aaacaaaaat tagctgggcg tggtgacggg cacctataat cctagctact 16380
agggaggctg aagcaggaga atcgcttgaa cttgggaggc ggaggttgca gtgagccaag 16440
attgcaccac tgcactccag tgtgggcgac agagtgagac tctgtctcaa aataaaaata 16500
caaaccctga gcttgggagg gtccaggctg caatgagctg cgatcgtgcc accgcactcc 16560
agcttgggcg acacagtgag acgctagacg gtgtctcaaa aaacttaaac agaaaaatgt 16620
agaaaggatt gtcgttccct ttctcgtttt ccagagggca gaggactctc ggcccctgct 16680
ttttgagcac caggccgagt ttcagacctc gggatggcct ctccagtctg cagctcccgg 16740
cagcctcggg ccacactccc gggatcccca gggactggcc tgggactacc gggggtggcg 16800
gccgtggctc tggctatggg gagggaggca gagccgcggg gcaggcgtgg ggtctgctgt 16860
gccgggtatg tggggcgtgt gttagacttt ggacctggcc tgcggggtca ggccgtggtg 16920
tatgatgacg ccacgccgct ctttctcttt caccctggcc tcctgcctgg ccctcccctg 16980
ggtcggcatc cctggggcca ctcctgcccg tgcccgctgc tgtggggcct gggctccagg 17040
cagctcctgt ctgctgctgc aaacaggccc ccaaggtgca tggtgaggag acccccaggc 17100
tagagatggg cgagagcggt gggcgagcag actgcgggaa ggcagctggg cctccacacg 17160
cggagccctc cccaccacgt gcatgcacag gcgacccctg ccgtgctccg acggagggcg 17220
ccgggactgg ggagggtcag cgcgacagcc ctggcgttta ctgaagagct gcctgcctgg 17280
ctcgctgccc tggtggccgc tgaattctcc caacgcccca ggacagcagg tgctgtggcc 17340
ccatgaccgc tcccccacat ggatgaaggg gtggtgcaca ggctggtgga gcctgggaga 17400
gccagggcag cctgcggcca tgcggccatg cggccggcgt ggggctgagg tccctgtaca 17460
gctgacagag tgcctggcgc cctcaatcag taagtcatct acgggccggg caccggcggc 17520
tcagcacaga gaggcaacaa gggtagaccc cccctggcaa ggctgaccgg cgccgggctg 17580
tggccagacc aggagactgc agcccagctc gagggaggca ccacgcctgc ccctcagagc 17640
actgagcaga gccccccatg gtatccccg ccctgggcac agcaggagga gctgccgacc 17700
aagtctggca ccccggtgtg ccccgagagc tgtctgggca caggttgcgg ggctgccccg 17760
gccggacggg gcatctgtgt ttctcggtgt ggcctcaggg ctctggtggc tttgaccgag 17820
gccccggggg acagaatgca gcttcctgct cctcactttt cctaagaaag gggccgcctc 17880
ccaggactgg ggctgcgcca gtcacaggcc cgcggcctcc acgctccggg tgctgctgtc 17940
ccggctgctt cagggcgggc tctgtcttgc acgcagcgag cggcttcatc acctcccttt 18000
tgaattggag ctgagtgtcg gcaacgccca gataactggg gcgcgcgggt cttttgtgct 18060
ggatttttct gtacagttgc ctcgatcttc catccagctt ggcctctgtg cagtaaggag 18120
cggcccgtcg cctcccccac gtcgcctccc ccacgtcgcc tcccccacgt cgcctccccc 18180
acgtcgtcgc ctcccccacg tcgcctcccc cacgtcgtct cccccacgtc gtcgtctccc 18240
ccacgtcgcc tcccccacgt cgcctccccc acgtcgcctc cccccacgtcg cctcccccac 18300

gtcgcctccc ctcacgtcgt cgtctccccc cacgtcgtcg tctcccctca cgtcgtctgc 18360
cctgagcccg ctcccttcga aggtaaccat cctgcagtag tgagggctcc ttgctgagca 18420
cgtgtggctg cgacttcagg agcgttctgg gttgagaatt ccaaaagggc ttcgctcagg 18480
cctggctttg ggtctttggg ctcctgacgc gagcttttac ttccaggctt tggcggtctg 18540
gcctcaggag gtcaaggctg cagtgagccg tgcgcgagcc actgcaccat cctgggcaat 18600
cagaccccat ctctaaagtt caaaacaaca tctttgtaag ggtttcgggt ctctgagccc 18660
atgaacctgc ccaggccctg cagaggtgag ggtggcccta gagtggctgg ggggttgtgg 18720
agaccagaac cagggcagaa gggcagggag ggtgggggcc ctgccgaggt ctcccaccgc 18780
tgacccctga cccctggccc ccttccacct cggcccaccg agagaggccg gcacagcgca 18840
gactgcgggg tcagtgacac tcccctcagc gtctccagcc acagaagggc cgtggcctgc 18900
cttcgccctc ctcacccgtg tcccggggcc acgtttgaac tttaggcctt gttctgactt 18960
cattgaaacc ctcctgaagt gcagacgtct tcctgaccca cctgggtttt gccaggatga 19020
ctccgaaagg tgtctcccag cagacacacc ccgctggcga ttgtttggtc ctggggaccc 19080
gggttctcat tctcctctct ggagctggtg aggctgtcct tgctagtcgg tgacaactac 19140
agggccaaag ggagggtgtg tcccaggagg cccagcccac ctctgcctgc acctgcggtc 19200
agcggggtgc agagtctgtg aaggcacctc ccggagtggg ggctgcaaag atcatggcca 19260
ctgtgccacc cacaggggcc caggccggac gcctgggctg tgccgcctcg gcagccaggc 19320
cgaggaacgt gcaggtgcgg ccgggagccc gccggccaca gctgcatcca tgcccttctc 19380
cgagtggtga tggcggggag aggcctggct gtctacgggc agattgcttg ggctttcaca 19440
gaagcccgct gttagagcag attcccggcc ttgggtctct ctgctccagc tggacgccct 19500
gagcctcgcc gggcccagtc cacccccgtc cactctgcca ggggcagctc agagccctcg 19560
ccacgcgggg ctgagcgcag ggaaggcagc atcacggagt tcgctcggct ccacagaaca 19620
cccgtgttgc tcgcggagac tttttgtttt tgtgcttctt atgaagaggc aggttttctt 19680
tcttcctgaa ccaaaagggt ttagatggag cacaagattc aggccatatt tacaagaaca 19740
attgattttt ctgccaggca gagcagccag tctgtctcgg aggctgtgct gtggtgtggg 19800
gaggcccctt gctcagatca gcctgtgcct tccccaggcc ccggcccatg cacctgccct 19860
tccgtaggtc tgagcacctg ttcttccacg cccaggacga cctggtcctg agtttggccg 19920
ggacccccctg ctatctgccg tccacaggcc tggtgacaga gaaagcagtg ccctggaaca 19980
gcagcccggc tgtggggtgc tcagccctct ctgtaggatc ctgtgtggct caaggggtct 20040
gctgagcctc caacgggcaa gaggggccct gagccatgga tccattctgg gtggaggcag 20100
cccccaccgc cgcctgcctc aaatgctcag ccctaggccc tcggccccgt agctctgcag 20160
ccacagacgg gtgccttccc caggccgaga ggagggtggg gtccagccac tcaggacctc 20220
acagggtggc cctgatgagc accttccctt gcagccacac ccactcgccc gggtctatgg 20280
ccacggtcgg agagtggtcc tgtgtgcgct gcaccttcct gaacccggcc ggccagcgcc 20340
agtgctccat ctgcgaggct ccccggcaca agcccgacct caaccacatc ctgcggctca 20400
gcgtggagga gcagaaatgg ccctgcgccc gctgcacctt ccgcaacttc ctgggcaagg 20460
aggcctgcga ggtgtgcggc ttcacccccgg agcctgcgcc tggggctgcc ttcctgccag 20520
tcctcaacgg ggtcctcccc aagccacccg ccatcctggg ggagcccaag ggcagctgcc 20580
aggaggaagc aggtccagtg aggactgcgg ggctggtggc cacggagccc gccagggggc 20640
agtgcgagga caaggacgag gaggagaagg aggagcagga ggaggaggag ggagcggcgg 20700
agcccagagg gggctgggcg tgtccgcgtt gcacgctgca caacacgccc gtggccagct 20760
cctgctccgt ctgcgggggc ccacgcaggc tctcgctgcc acggatccct cctgaggccc 20820
tggtggtccc ggaagtggtg gccccggccg gcttccacgt cgtgcctgcc gcgcctccac 20880
ctggcctccc cggggaaggt gccgaggcca accccccagc caccagccag ggcccagctg 20940
ccgaaccaga gccgcccagg gtcccgccct tcagcccctt ctcgtccacc ctgcagaaca 21000
accccgtgcc gcgcagccga cgcgaggttc ccccccagct gcagccaccg gtgcctgagg 21060
ctgcccagcc gtcaccctct gccggctgca ggggagcccc ccagggctcg ggctgggctg 21120
gggcctcccg cctagcagag ttgctgtctg gcaagcggct gagtgtgctg gaggaagagg 21180
ccacggaggg tggcaccagc cgcgtagagg ccggcagctc cacctcgggc agtgacatca 21240
ttgacctggc cggagacacc gtgcgttaca cgcccgccag cccctccagc cccgacttca 21300
ccacctggtc atgtgccaag tgcacgctca gaaaccccac agtggccccc aggtgctcgg 21360
cctgcggctg ctccaaactg cacggcttcc aggagcatgg cgagcccccc acccactgcc 21420
ccgactgtgg ggccgacaag cccagcccct gcggcagaag ctgcggacgg gtgtcctcgg 21480
cccagaaggc cgcccgcgtc ctgcccgagc gcccgggcca gtgggcctgc cctgcctgta 21540
ccctgctcaa cgcactgcgg gccaagcact gcgccgcctg ccacacgcct cagctcctgg 21600
tggcccagcg gcggggggcc gcgcccctga ggcgcaggga gagcatgcac gtggagcagc 21660
ggcggcagac agacgagggc gaggccaagg cactctggga gaacatcgtg gccttctgcc 21720
gggaggtgag gcgcccctc gccctcttcc tgctcctgag cctcctgctc ccgccctccc 21780
cttcctaggc tttgggctct ggcgatgggc tggggaggag cccacaaggc ctaagacgtg 21840
atggggaggg cagcaccctc cgcccgagg ctccaagctc ccaatggccc tggggtccag 21900
cttggcctcc tgacccttgg tccttgctgg tgaccgagat gcacgacctt caccccctc 21960

```
aaccctccac cgtggccagg agggccccga gccccgtctg atgtgccgca ggaggcgagg   22020
gcttcccagg ggcagcgtgg agaccccgct gaagtgcgag gtcgggatgt acccgtgctc   22080
cgccctctag agtccaggat ggcatcaccc acgggtcaac tggggcgcag cctgcaccct   22140
ccccagcagg gccagggcca gagtcgtgct gccacccact gggggctgcg ctgtccccct   22200
gggcccacac gtcccctgct cctcacctgt cgccagccgg tggcctctcc actagggctg   22260
agggcttcct cgacacaggg ccggcgccag gtctgtggct gtccacgctc cacccgtccc   22320
tttggggctc aggcagtgct tttgggcgct ctcctgggtg ggtcttgtc cctgccaggt    22380
ggagcgaggc caccctgccc agcctgagca catggccgtg gtctctgcag aacaatgtga   22440
gcttcgtgga tgacagcttc cctcccgggc ccgagtctgt cggcttcccc gcgggtgaca   22500
gcgtgcagca gcgtgtgagg cagtggctgc gaccccagga gatcaactgc tccgtcttca   22560
gggaccacag ggccacgtgg tctgtgttcc acacactgcg gccctcagac atcctgcagg   22620
ggctgctggg gaactgctgg tgaggccttc tccaaggccg gggtggggcg ggtgggcggg   22680
cgaccggccg cggtccccgc gaggtcaccc tgaggctctg cgcaggttcc tgagcgccct   22740
ggcggtgctg gcggagcggc cggacctggt ggagcgggtg atggtcacgc gcagcctgtg   22800
tgcagagggc gcctaccagg tgcggctgtg caaggacggc acgtggacca cggtgctggt   22860
ggacgacatg ctgccctgtg atgaggccgg ctgcctcctc ttctcacagg tggggcggcc   22920
tgcagggtgg gcacgggcgg caggggcagc ctctgacccc agccccgaaa acaaggccgg   22980
ctgcttcctc ttctcccagg gcgggtagtg tggggggcgg gcgggggtgg cctctgaccc   23040
ggccctctgc aggcgcagcg gaagcagctg tgggtggccc tcatcgagaa ggcgctggcc   23100
aagctgcacg gctcctactt tgcgctccag gcgggccgcg ccatcgaagg cctggccacg   23160
ctcaccggcg ccccctgtga gagcctggcg ctgcagctca gctccactaa cccccgcgag   23220
gagcccgttg acactgacct catctgggcc aaaatgctga gttctaagga ggctgggtaa   23280
gaggaggggc actgcgtggt cagccgcgtt gggaggagag gcgaggcgga gcggtgggag   23340
atgtggggct ggtggcccct gacccagttc tgcttccacg aggtgcccct ggcgtgggct   23400
gaccccgcgt ggccaggcca cagccccaat cacctccccc tccctgggcg gggctggagc   23460
tggccttctc atgccaggaa ggccacatcc tggccaggcc gtggggcgag accgatgccg   23520
tcaccccgga gcaggtgtga gcggcgtgtc gagtttgacc tgggccgtgg ggcgagactg   23580
atgccgtcat ccgcctcggg gcaggcgtga gcggcgtgtc gagtttggcc acactctggt   23640
cagcgggtct gtgcctcgac cccaggggc ctcctcggag tgccccggtg cgagcacctt    23700
gaagctcccg gagccct                                                  23717
```

<210> 44
<211> 17265
<212> DNA
<213> Homo Sapiens

<400> 44

```
gcaggcggat cacgaggtta ggagttcgag accagcctca ccaacatgct gaaaccccgt      60
ctctaccaaa aatacaaaaa ttagccaggc atggtggcac acgcctgtaa tcccagctac     120
tcaggaggct gaggcaggac aatcccttga acccgggagg cggaggttgc agtgagccga     180
gatcgtacca ttgcactcca gcctgggtga cagaatggaa tgagactctg tctcaaaaaa     240
aaaaaaaaaa aaaaagaagc cctagatttc ggttgtgttg gttgtaaaag gagagaccca     300
gtaagtgggg gttgtgccgc agattgctac ccacaatgga cgggtcactg agcaggtccg     360
gccaactggg cgttccctcg ctggagggcc agcacaccag actgcaggtg gcgcgggtca     420
gcaaggtacc aggggatgtg tcacacacac agcccacccc cgtccagtca cgcacggaca     480
ccctgggctt ccgagcaaac ctgctcccac gtggtgtgac cacatggagc cacagacacc     540
cagcaaggac acgcagcccg cacacccccg gtactccaga cacagtgacc tgcaccaggg     600
ctcgaggttt ctctagggaa cccacctctt agaatcatcc agaaacaagt cactcttcac     660
ctgtccagca aaggcctgct gagaggtgca cagtgtctgg agtccaagct gcgccaaggc     720
ggcaggaccc ccagcccagc ccaggacccc cagtagagcc ctcacctcag cgtggaggcc     780
tgagaacgtg aggaaggagc tgtccagcac ggacgagtcc aggcagctgt cgatgtccag     840
cacctactgc ccggcaggtg tggggctcag gctcccagcc acctgcagga cgacggcagt     900
ggtcagcggg cggcagctca gacctgctca ggacagggat gagaagccac ctcctcagca     960
gacaggacag agcccggtgc catctaacag aatgtcctag aatgctggat atatggaca     1020
tctgcaccgt ccgtgatggc agcccctcgc gacatgtgcc actgaacact tgacagcaga    1080
ctggtgcagc taaggaacag agttttgaat ttcatttttt tttttttttt aagatggagt    1140
ctcgctctgt cacccaggca ggagtgcact ggcgcaatct cagctcactg caacctccac    1200
ctcccgcgtt caggcgattg tcctggctca gcctcctgag tagctgggat tacaggtgcc    1260
tgccacgatg cccagctaat tttttgtatt tttagtagag acggggtttc actgtgttgg    1320
ccaggctggt cttggaactc ctgacctcaa gtgatctgcc cgcctccgcc tcccaaagtg    1380
```

```
ctgggattac aggtgtgagc caccacgccc ggccatcgtt ccattttaat taactttaat    1440
acgagcagcc acatgtggcc tctggttcct gccacggact cgggagcaac ccctcctggt    1500
cgcggcttat gcgccttctc tgtgtgctgc tggggttagt ttgcatgtaa cctcttgagg    1560
accccacgtg tgcattccta aggggtgcgg cctcccgttt ccgtatgaat gggaagagtt    1620
cccacctgct gtattcttgg aaagagtctg tgaaggattg gtgttaattc ttccttaact    1680
gcttagaaaa attctatcgt gaaggctctg agcccaagct ttctttgtg ggattttttt     1740
tttctttttt ttggagatgg agtcttgctc cattgcccag gatggagtgc agtggcgcaa    1800
tctcggctca ctgcaagctc cgcctcctgg gttcatgcca ttctcctgcc tcagcctctc    1860
gagtagctgg gactacaggc gcccgccacc atgcccagct aagtttttgt atttgtagta    1920
gagacagggt ttcattgtgt tggccaggct ggtctcgaac tcctgacctc aactgatctg    1980
cccgcctcgg cctcccaaag tgttgggatt acaggcgtga gccaccgtgc ctggcccttt    2040
ttaatgtttt atatagatgg ggtcttgcta tgttgcccag gctggtctca aactcctgga    2100
ctcagatccg cccacctcgg cctcctgaag tgttgggatt acaggcgtga gccaccacac    2160
ccggcccggc cactgggagg tttctaaggg actaactcgg cctcttcact tgctatagat    2220
gtactgagat ttcttctgg agtgcatttc ggaagcgtgc acagccgcgt gcttgcttct     2280
tctgagttat ctggcgtgct gctgtgcagt tgtccgtggc gtctgcttag cgaagtgccc    2340
tcgttctttc acgattctgg cttctgagtc ttctctcttt ctccctggtc agtctagcta    2400
aggctgctca agtgtgttga cccttcccga gcagcctttg gtggacgcct ttccctctgg    2460
ctgcagcact ggaaagtggc ggccctgggc atggtgccga ggcccaggct ccattcccag    2520
tactcccggg tccccagccc cagcccacct tgctccggga catccggaag agaaagagga    2580
tggccaggta gacgggatag acaaccacgc tggacaccag gccaacagcg actgtgtcga    2640
cgctcagcgg gctcagcctg gacacatgcc ccgtgctgtg tggaggagag gaggccacac    2700
aggtgaggct gaggggcagg aagggctggg caggaagagg ctgtcccgac ccctacggca    2760
cccacctgta ggcagagtca ccaacagccc cgtaccacac ggcgttggcg cccaggaaga    2820
ggcagatgag gagaacgcag caggtggccc tctggatgcg agtgaaacag ctacgaggtg    2880
gccggtccca tatggagagc cagatgtgct tgtcaaagaa gccacgctgc agctcagcca    2940
ccagcaggcg ccggaagcgc aacagggctg cgtgacctag aaggcaggga gggccgcact    3000
gcaggaggcc acggggcagg accaccctgc ccaacctccc acggagtggg aacatggaac    3060
gaggccttac tcgcagccag cacctccttc tccaccaggc cccgttggc ctccgtctcc     3120
accgaaagcc agtcattgac caggaagaag gtgctgcgtg ccgtctgcag gtccctgacg    3180
atgatgtgct gcaggaacca ggcagggctg agccctgcag aggcgcggga gggaggtcag    3240
gctcgcaggg cgccccaatg cgggggcaga ggggcagagc ttggcagggt ccgcacagac    3300
ctttgttgtc gtgccacact cggatcttcc acacgctacc caggctgtgc ggggtggcga    3360
tccggaagat gtccagactg ttgcggtgga aggctctgtc gccgtccagg tgccggtggc    3420
cgctccggct gtccacccca tacagcatga tgcccacgtg ggccgtggta cctggagggc    3480
aagagggagg ggtgggaggc tcggtctgct gcccaacacg tgtggcatcc caggcaagtc    3540
atctcagctt tggcctccgc gcactcaagg agccacacag gcagtcccgg ctttgcacgg    3600
ctctgccata cacgaggagc tgcggttact gcaatttgtc caataaacag caggacctca    3660
aggacatgat taagttacac ggaaagaact gtaacttgtg acatgcaaac atggctgcac    3720
acgcctcagt ccacaccaca accagtgacc cgcgctgcac acctgtccac gcctcagtca    3780
cgccacaacc agtgacccgc accacacacc cgtccctcag ttcatgcaca gactgcgaag    3840
cgtgaagctg tgtcacctcc tctcccagtg acagacccag gtgacagtat tttttttctt    3900
ttttttttg agatggagtc ttgctgtgtc acccaggctg gagtgcagtg gcgcaatctc     3960
agctcactgc aagctccgcc tcccgggctc acgccattct cctgcctcag tctcccgagg    4020
agctgggact acaggtgcct gccaccacgc cgggctaatt tttttgtatt ttttagtaga    4080
gacagggttt caccgttagc caggatggtc tcggtctcct gaccccgtga tttgcctgcc    4140
tcggcctccc aaagtgctcg gattacaggt gtgagccacc acgcccggcc gacagttttt    4200
aaaagtaggt aatcaaaaga actgggaaat gaagatgaaa gcaacacgga aataaaaaat    4260
gggaacacag ccaggtgtgg tggctcacac ctgtcatccc agcactctgg caggccgagg    4320
caggcggatc acctgaggtc aggagttcgc ctggctgaca tggtgaaaaa ttaactgggt    4380
gtggtggcgt gcacctgtac tcccagctac tcaggagaat cgcttaaggg gaatcgctta    4440
aacccaggag ctggaagttg ctgtgagcca agatcacgcc attgcactcc agcctgggca    4500
acagagtgag actccgtctc caaaaaaaga aaaacgaaaa caaaaaggga atgccagaag    4560
ggcaattcca ataaaggaaa atggaggtat tgaagaaaca gccacgggga gggtgctggc    4620
acctccgtct gagagacgag ctatgcagtc aggatcgcgg gtggatgcat ggtctcccac    4680
agtggtagcg atgctcatgt cacttgtggg gccacgctac tgtgcagaac gtgggctgcc    4740
caccctgact gactggcacc tacttccagc taggagctgt cctagtcctc agggacagtg    4800
agtgctcacg aggtcattcc caggatgaac acacgagccc ttcacacagc actgcaaaaa    4860
ctgccttgtt ctgacgcctg cgacgagact cactcccaga gggtgcaacc agcacagcca    4920
gtgagagcag gggaggccct gccaccccgc cgcgcccctc acctgagccc cggccccagc    4980
ctgtcttgac gaggatctcg tacttgaagc ggccccgctg cccacagaag gggatggcgc    5040
```

agccccggct ggcatccaac tggtccagct tgtgcaggat ggcggccatg accatgtagg 5100
tcaccaggca cacagcacat gtcagcatga cgatgtagtt tacatccgct gtcggctcct 5160
gtgaagacac agccgccggg cccaggaggt cacgtgcaag ctgtgccttc tcaggataga 5220
gccgagccca cccaggccct cctcgactct gcagaggctc ccaggagcac agggtcactc 5280
acaggaaaca caaagcgtac atggcttggg ggcatgaaga ggctggcgcc gaaggcggtg 5340
aggtggcggg tgaggcagac ggcctggcgg ggcgaggtct cctccagggg cagcagcccc 5400
tctgtccgcc acaccacatc ctcctcgctg aagtactggc acagggacgt gtacaagccc 5460
acggacacct ccagcgccga ccagcggaag tggctggaga ggttcagacg gtaactcccc 5520
actgggtctc tggtcctggg aagggaaggg gcagtggacg tgagcccagg ctccgccagg 5580
ttggatgtcg gagtcccaga gcccataccc ggtccagtcc cctcgctgcc tgccgtcccc 5640
acggggcccg taacccgggc aatgctgacc catgatgccc tgccctgccc tgccaggccg 5700
gcccgcagag ctcaccccgg ggaaatgaag aaggtgtagg gccggtggtc ggcaccctgg 5760
agggactctg ggcggatcct cctgctagcc gagcagttgc gctcattggg ccggggctcc 5820
gagtgcaggt agactgccag gtagggctcg ggttcctcag acaggtagcg gcctggggca 5880
gaacgcgcag gtcacacgcc tgccgggaag ctcaaccacc cgggggacac ccacgatggc 5940
cctcctgagc ccaccctctg ccacgggcct gaaaggccat aggagcctct gcaccagagc 6000
tggcacctgc ttctccgtgg cccccagctc ctctccggcc aggcccccaa cagcccatga 6060
aacagcaaat ttcaccagag acacccatgg aagccctacg agaaacgcct tccccccaag 6120
aacaaggcca gggggccgcg tgtgccccac ccgctgcaca caccgtccag cagcgtatag 6180
ttgagctgca gatgcagcac ggccacaggg ctgctgctgt ccagggtgac cacagcaccg 6240
acggaggcct ggggctggac cacaacggag ttggcggagc tgcggtggcc ccgggcagcc 6300
cagtccgagt tgttgggcac cttcacggtg atggcgcgct ctgaggccag ccgctcgatg 6360
gggatctggg cgccggcctg tgtctggaac gccatcgagg ccaccttggt ggagacggtg 6420
tagttgctga tatagccaaa gagaaaggga ttggagtcca ccagaaagac gagctgcacc 6480
acgtcactga ggttggcggg ggccctgctg aaagcctagg ggatggagag gtggcagcca 6540
ggccctgggg cgccgccata gcacagcagg ctccgcgggt ccgagcgctt gccctgggcc 6600
atgatctcct cgcccgccag cgtcacgggc tcctcgttga gcacgcggga gcgcgtgagg 6660
atgcgcatga gggcagaggt caggttgtag gcctgggacg ccaccatccg caatggtgac 6720
tcggctccca gctctgagcg ctgcggtgcc cgcacgtctg agctggccag gtggatgagg 6780
tctcctgcag acaggcgtga ggtcagtgca gagacaggga ggcagaggga gggtggggc 6840
aggcaaaaag ggggagccgg agggtggggg ctgggagaaa gggggaacct gagggggcag 6900
agagcgaggt gcaggcagaa ggaagaggga agctggagag agagtggtgg aggggggaggg 6960
ggaaggggat ggggatgagg acgaagatga gggggatgat ggggagaggg aggaaaaagg 7020
aaggaaaagg gtagagaaaa gagaaagggg agaagaagag gagcagggtg aaagggaggg 7080
gaaggggata aggggataa gggaggggaa gggggataag ggagggggaag gaggataagg 7140
gggataagaa agatgagggg aatggacaaa aggacgggga ggatcggggg ggaaatggag 7200
aaaaggggag agagatggag aaaagggatg gtaataggga agggggaggg ggaggagaat 7260
gggaattggg ggagggggat aaggatggga attgggggag ggggatgagg atgggaattg 7320
ggggagcggg atgaggatgg gaattggggg agcgggatga ggatgggaat tgggggaggg 7380
ggatgaggat gggaattggg gggaggggag ggggacgaag atgggatggg gcaaaggcga 7440
ggcggttgtg gggaggaggg aggcagagga aagggcggca tggggcggac gggccacgtg 7500
gggcgggcgg gtggcgtggg gcacgggccg cggcacctgt gatgttgagg atgctgtctc 7560
cgatggcggt gggcgtcacg gtgcccgcgg tggtctctgc ctgcaggatg cgcatcatgg 7620
cctccagctt gtgcagcgtc tgcttcaggc acgagcggca tacgagctcc ctgctgggcc 7680
cctgtgtgga gccagcagtg tccagccccg ctcctggccc cactccttgc acacgccctc 7740
ctctctacac gggtcctcac ctggctccca cccccagccc tgcagctgga gagcccactt 7800
gactggaccc ccacagcctc ctcactaagc attttttgtg gctctgcatg acccagggcc 7860
tccacctggg gaacacgtga tgcagcccac cgaccacaca aggcacctct tcacatgaga 7920
gaaggaggag ggcagaaggg agagaggaga gggaagtgga gaaaaggggg gagaggagag 7980
ggaaggagag agaaggggga gaggagaggg gaggggagag aaggggggag aggagggagg 8040
gggaggaggg gaggaaggag gaggggaggg gtgaggggat ggaggggctg gggggaggagt 8100
ggagggggctc agcgggatga ggtgagggga aggtctaggg gaggggagga ggggaagggc 8160
taggggaggg gaggggctgg gggaggaggc aggggctagg ggaggggggga gggactaggg 8220
gagggaaggg ggaggggagg ggttagggga gggaagggga ggggaggggc taggggaggg 8280
aagggggagg ggaggggaga gtggagggca cagagcagca tcttcttagt ccctccccac 8340
atctgggccc ctctttacac cctgggtccc ccgagaggca ccctgcgttc acacaggaca 8400
gcagaatggc tgaggctact gaagcaggtc agagaccgag gaacgccatg gcaggaagga 8460
gcccaggctg gaggctcagc tcctcggcca agctgcccgt ctgccctggg gggctgaacc 8520
cagtaccctg gcaggcatgc ggggcgggga gagcatgtgg ggccatccta ccatgcactg 8580
ggccagcgca gcagcgatct gctggatgtc atccacagtg tggaccctca gggacaccag 8640
agtctccgtg atgttcttgc gtatctgggc tcggcgctgc cgctcgtgct tgggctctgc 8700

cgccacgtcc agggcccgct cgtactgggg caggcagggg gcacagcaag ctgtcagcag 8760
ggcaggaggc cggcaggagg ccagcagatg cccacgactc ccggggtgca gttacgtgct 8820
agatgctgtg tgatgtgggc actgacccgc aacactgagc tgtttcttca tgggcaaaac 8880
agggtaagca catgggccct cctgggcggg ggctgcattg tggaaagcag acgccggaga 8940
gggcccggtg ggtgtggctg ctgggagcgg aacgtcgggg tgctgcttca gggtcactgg 9000
gatttatctc tggggcccgg gatgagccct ccgcaaagct ccaggcaggg gaacaggtct 9060
tggtccccag cacgcatgca gcagatgtga ggtcccctcc caggctgcac tcacctcgtt 9120
cagcacagtg accagggcca gcgagtactc gatgacgtgc tggggatcgg cctgccgcag 9180
cagccccggg agcacactag cggtgagccc gtgcagccag actgtgagcc ccattgcgct 9240
gccgttgggc tctgggaggg tgatggccag agacctacga gcagaggggg gtggtgagta 9300
ggtggcagtc tcgggggcgc cctcccacgg cctggctcac ctgttgaggg cgaccacagc 9360
ggctcccagc tggtcctgca ccaccacggc caggcccacc tcgaagtgtg gcctgaaacc 9420
cgggggcagc acggctccgt agccggagag gctgcccttg tagacacaga actcctcgca 9480
gtggccctgg cgacagcgct gcagcagcag ggcgtacacc agcggggcgc cagcatcctc 9540
cgcgtcatgc cagcctgagg gacggtcccc acggcatcac gggagggctc cgtgacctca 9600
cagagtcggg ggatcccgct gctcccccta cgcaggcctg cactcaccca tgcattcgaa 9660
gtgcaccttg gtggtgagag cgtgcacagc gcccagtggg aagaggcggc aagagccccc 9720
cagcggcggg cggttggggg acagggggat ggaggcgcag ccctcctcct tgccagagcg 9780
gcccagcacc gtcagcgtga aggtgtatcc ctcgccgtcc cgcagcacgc cccgccgcag 9840
caccagtcac atgcctgcgc tgcccgtgga tgtggtggtc tcatccagca ccagcgtctt 9900
gttgctgaac gtacgtgcag cccaccgctg caggcagaag ggatggtgag ggggcgcaac 9960
cctctgccct gtcagcccca cttctgcctg caggccccgt cccctcggcc atgggaccca 10020
tccccaaccc gcccacaccc cgctcaacac tcacccctcg cttggagccg ctgctgcaat 10080
tgaggcagcg gccctccagg tacacgtagg agctgcggct cacttcgtac acggcctgtg 10140
ccttgcagga cacacactcc aaggacacaa tgggcacccg gccactgcgg atcagcacct 10200
ggcgtgggag tggggttacc tccaacacag gtctatttgg cctgctggaa ggtctggggg 10260
acccgtggag gatgctgctc ccaaactcca ggtttcccag gggcctggcc actgccggtg 10320
agctcacccc ctcccaggat actcatccgg tttgccacct tccaacctgg gcggcggaag 10380
ggcatacaca gggcagagga cactggggtg tgcgttctgg tgtactggag ccagctggac 10440
cctggcagga ggcaggcaat gctcactgag ggcccctggg gggatgcgtg tgggaacaga 10500
cgtatgtgtg ggtgtgagga ccgcagttgc cacgtaggcc tgactcacag actcctgcag 10560
cccttagcca gggcctgggt caggaggctg agccgggatg gaacctgctc ccacaccctc 10620
ccctcagacg acccctctgg gcagaccccc aatcaggcca gctgaggaaa gcagggactg 10680
gggaacagac acccactctg gggtaccagc aggccccagt cagggaggcg cacacgctca 10740
cagagggcag ggaggcgcac acgctcacag gcacctgctg cgtccggttc tcgaaggcat 10800
tagatgccag caaggtcagg acgtactcac ctgtggggac aggcccaagt ggggcagccg 10860
cggcaccccc acctgctccc cacccgctcg gcagaagccc cccgcctgag gagcccgggg 10920
tgaacggctg cacctgcggc ccagccttaa gggtcccagg ctcccaagcc acgtgcggga 10980
cggagcacag gtgcagcagc actgagggct gcctggtgag gacggcaccg cctccaagtg 11040
cagctgcact cggggcagca gagcagcaag agccaggccg cggtgggggg cagttcaggg 11100
ggcccagctt ccctgtccac tcctcccacg cctggcccct ccctcacccc agtaggggcc 11160
taagccatca gcccaggtga ggtcacagtg aggctgttg gggaggaagc ggggcagctt 11220
gactggggga ctgggggggc cccgtgctca gagcctgaaa ggcagtggcc ccctcacccc 11280
ctcatccctc acctggggca gcgtaggtgt gggtgacatt gtgctccacc agcacctggg 11340
ccaccgaggg gtctggaacc gggaaggact cgttgtacgg aggctggaac tggtggaggg 11400
cctgctcccc atccccaaag gtccacctgc cggggcggtg ggaggcagtg agtgaaccgg 11460
gacaggggtg cgcagtggcg gggcacaggt gcgcggtggc ggggcagggg gtgcttggga 11520
cccagccgag gctccactct gcagtcacgc cccgggcctc cattcagggc ccacccggct 11580
gtgctgaggc ctctcccggc tcccgtgcag cctcagggct cctgtgcacc cagtacctcc 11640
caacagacag ggaaaccgag gctcagaaaa gcaacccccct gatgtggggt ccctcggctg 11700
aggctggagc cgggacaaga gcctggtgcc cggacaagag cctggtgccc accccaaacc 11760
ggcccctgag tcactcacag gaaggccacc tccacggccg agtccaccag cacgcccgcc 11820
gtcagtgcca gcgtggcatt gggggacagc acggctggca ctgtagagac ccgcaggccc 11880
tgcatcctgt tcatccgctc cacggtgatg ttgtagttca cggtgacgtt gctcacgtgg 11940
ttggaggccg tcagctgcag ggacaggcat cagtgggccc aggtggcagg tgagaggcct 12000
gccctgcttg gcgtccctcc ctccactcac cacagccatg gcagcgtcct cgggcagcat 12060
gaagcagagc agaaggcaga ggtgaaggtg gagcccgccc ccgccccgcc ccatcccctc 12120
ccctccccac tcccgcccac ctactgagag cttgaagacc gccgcgctct gataaatgac 12180
attgaagacc acgttctgga aggtcaggga ctgcttgtcg ttgatggtcc accggaagac 12240
catgtccgag ccggcctcca ccacggggct gtacctctgc ggggggactg gtgtcagcct 12300
gggctctgtg gaggactctg cccttagcct gtcgcctcct ggacacacct cccgtcgggc 12360

```
tggagagtcc cacgcggggc acagaggaga ggaggtgccc ggggctctgc atgccatgag   12420
agccaagccc gggctgggac actgactgtc cggctctcca gccagccatg tagtactact   12480
aatgcctcaa cctctctgtg cctcagtttc cccatctgta aagcaaacct agtaccagct   12540
acaaagagtc cacctctctc tgagtcttct cagaccctcc cggggctcct gccccagctc   12600
ctcagccaga gagctcggag cagtgagggg aggcacacgg gcctcacagg gacagcacct   12660
acactggctt acagaaccca ggacaggctg cacaggtcac gccatttctg atggcccctc   12720
ccaaggcccc tggtgaaggg gcaggtaccc gcaagatgga acagccctgt cccccatgta   12780
cccagcatgg tggcactgcg ggcagcccgc agtttcccat caggggttcg gactccacct   12840
caaaagccac ttgctttagc caggcgagaa cacagcagag ggcgtgagag actcacgggg   12900
actcgtgtga ggtcagggag cggagtttta aattcatttc gtgaaatgag acggtggaat   12960
gagttagcgg agccgctgtc agagccgtga ctttccagga atttaaagcc caccaggtag   13020
cctgaggagc cagccagcag gacctgcccg gggccgacgt ccccagtaac tgggctgctg   13080
ccctcactgg gaagccaggc ctcaagccct gtgtgagcac cctgtctgca ggcacctgcc   13140
tgggggctgg tggtggagcc tcggccatac tcaccactgg gactccctgc agtacacggg   13200
cctcggggct gggcgtggcg cggaggccac agatgggctc ctccgccgtc acccgcaggc   13260
tgaggttggc ccggctggcg ctgttttcca ccacaacgtc catcacgtgc tccccctcac   13320
cgagccacgg cagtgctacc actgagaaca gggtatcatt ggtctcccag gggcagccgg   13380
gcacgaaggt ggccaccagg gcagggcagg cattctcaaa gcgggcgctg acactgcccc   13440
caggccagcg agccgtggcc gtggcgctgg caccagagtc cacctggagc accgaggctg   13500
agccgttggt gggcacgtag aggcggccgt cgcggggggc agggtagatg acccgcagcc   13560
cagccactgg ggagaccacg tcaaagctgc aggacaggtt gtgcctggac acgccattgc   13620
ccacctctgc ccggacctca tagcgcccag gcagccgcag cccagggttg ggcctcaggc   13680
ccagcagcac ggtgagctgt tccgtggctg caagcagccg cagggcacag gcagggcagg   13740
cccaagtgcc ctccagctgg gctggcaagt ggggcagcca tgacgaggcg ttggcggaga   13800
ggtacggggc ctggggacca gggtggccgg gagccggcga gcagtgcagg agggcgccag   13860
ggccagcgtc gtgctgcaag ccaacgaggt caccagggag catgaggaca tcctggctgt   13920
ggagggtgac ctgtggagag ggaggcaggg ctgcatcacg tcctcacggt catggcccgt   13980
ggacccctgc acgacggatg agggtggaca cgcagggctc cccgcttcgt cagccacacc   14040
tcagggagcc tccccacagt gctcgtgaca aggacaggca ggacagttgc agacaggggg   14100
acacacgggg agaggacaca ggccaagacc tggcagacag gaaggagcgg ctgtgctggg   14160
agagaggaag aggaggcaca gctcgtgcca agggcccagg cgagagcttc tcccactggg   14220
agaggggcaa gggcactgca gaggtcggag gttggaggtc ggaggtcgga ggtcagaggt   14280
ggcaaggacg tgggaggggc ctgcaggctg ggtgtgcctg ctgcgcagac ccagaccctg   14340
ggcagcagac aggaaggtgg cctgaggaga tgcagggaac agacccaggt cagggccaca   14400
caccgagtac tgcgcggggg gccccgcggg aacggagaag aggaactctc tccatagcgc   14460
atagggggcc ccgagtagcc ctggccctga cgtgcagcca ttggcgcagg cctgggggtg   14520
gcaggaggcg tccagcggca agcagatgtt ggctccaggg caccagcgtc cccctggcat   14580
gcacgcgggg accagctggg tcctgttgtc cggggacctg ctctcaggct cgctgccgtt   14640
ctccggggtc cctgcgagga ggggagggtg ttggggccct cattcgccca cgggccaccg   14700
tcagagatgc ccaactgcct gcaccagcga gcctggcctt gctgtgagga caggtctccc   14760
cgcccgggca gcactcccag cccagtgctg cgtccctgtc tccggccagc tgactgaccc   14820
aggccggtcc ccaggcaggc cccacccgat ccaccccccag gacacctgga atgagctggt   14880
gtctctggaa cccctgctct gtccacctaa gactgggaac cactctgatg gccacaggac   14940
cagcagacgt gagagctcag agaggccacc ccgagtcctg cggcgcccac caccccagag   15000
tcccacctgc tgtgctgagg agccggtaca cctgcagccg cagctgggcg ggccgccgga   15060
gctcctgggt cccaaattcg gccgtggtga ggaaggcttc acggctcaga cgcaggcccg   15120

ggaataccat gacctggtgg gcaggggggcc gcctcagctc cacagacccc atcccagcct   15180
gaagcccaga ctccccccac ccgaacttcg caggaagagg ggagggaagg agagcgagcc   15240
atcggacccc cacaggcctg gctcctgtcg ctcgagagga agactccgat ggaaactgtc   15300
catgggggc aggacccctg acctgccttt caggaataac tcacccacac tcagagaaaa   15360
ggcctggggg taatgtgagt aaacgctttc ctctctgcac tctggatttt cccaaccatc   15420
ttcactgggc acaagcaaca ttaaggcccc caagtttttt ggcgagaccc acagtgggca   15480
gggcaggcga ggcctccagg ggcaggcagg agggcaggtt atagaacgtg gggggccgac   15540
tacctccacg ggctcgtgcg gggctgagag gccgtcctgc cgtgccagag gcatcagggg   15600
tccctacagg tccccactgg gcgctcccac gaggaggttc tcggcatcct gcactgggcc   15660
tggggtggca agtgcacagt gaggcgccgg gccagggccc aggacaccag gacgaacaga   15720
ctggggaccg agccgcccga gaaccccccc accagcccct cctcctcagc ccaggctcca   15780
ccgcgggcgc tcggcaggcc cctaaccaca gccagcgtct caggcccctg cctggcccct   15840
cgcacacctc caggccgcag ctcgcagacg tagctgtgcg gcgctgagca caggtcggtg   15900
ttacaccacc cggtgggccc gagccggacg cagtgctcgg ctgtggctgg gtgtggctcc   15960
```

```
ccgggcagcc agttctggca gctctccagg ctgaaggcct cgccctgcgg cgctgggccc   16020
acctccaccc cctgcacagt cgagaagccg atccacatgt ctaggctcct gggggcgggt   16080
gtgggatggc agggggctca gggcactcct ccatcctccc accctcacag cagcccgctg   16140
ggagccccgt cactgtcccc ctttccagat ggggaaactg aggctcagag cccggagagc   16200
agggcccacc agcccaggct cacagcagca cccacccacg gggcctgtgg gcaccggcag   16260
ggatccccgt gcaggccacc tcccgtatgg cgtgcccagg agtgtccgga ggctgccccc   16320
agctcgcgtc cacctctgca tctgcagagc tgacaggaac ggccccaccg gccggcgcca   16380
cctgctcacc agggccggcc cagctcccac ctccctcctc ctgagactcc ccagccgcag   16440
gctctgcccc actgcttcag agatctccca acctatggcc cctcgggggg tggggcaggc   16500
acctggtgac ccgggagacc aggaagcgct gcacggcggg actgtccacc attgccaggg   16560
tggccccggc ccaggcccga cactgctcct gcgcctgcag ccaggccgcc ttctccacca   16620
ccaggcggta gcagtgccca ttgccagaga agatctccgt gtccgagggg cagagcgggt   16680
gcaccgctgg agaccagtgg gaacgagggt gtcaacggtc agtgtgggcc caagacgggg   16740
gtaccaggct ctgccccatc tggatggccc tggggaggaa ggggagtggg cagcagacac   16800
tcacctcggg ccggctcctc gcccagggcc acgatgctgt aggcggcctc caggcctgaa   16860
ccaccgcggt tctggatgct gaggtcgagg ctctcgtcac tctgcaccga ggacgggcac   16920
acgagctcca gggcggcagg tgccgcttcc acctgcacgt ctgtccccag cagggctgag   16980
ccggtcccca gggccagcac ggccgtcacg tgatagcgcc caggcagcac atagcgatgc   17040
gaggcagccg gcccagcggc atccacctcg ggggagccgt ctccgaagtc ccagcgtgtg   17100
gcagtgacag ggagcggggc agcgatgtgg aaggctgcta gctggccgga ggccagggt    17160
ccgtggggcc ccaccagggt ggcccctggg gaggcaggga agacgtgctg gaggagggtg   17220
gggcccctac aggtgggggc aggaggcggc ggggggccgg agcag                  17265
```

<210> 45
<211> 2204
<212> DNA
<213> Homo Sapiens

<400> 45

```
agggacaaac tcacttaaat ttttttttaa ttcgctatcc tcgggaccct aattagaatt     60
gggctgcccc cttgcggtct ctccgacgtc ctatcttcaa ccagctcaca gtccttgggc    120
cctgctctcc tctccattgt actctcaaaa tgcaaggcgc aacgacaata gtaacgtccc    180
agttacttct gagactctct aaactctgga cttgcctccc tcccctcctt caaagtcttt    240
cgaatcttct ccagcggaca aatgaaggga ctgaagctgg acaaaaccat aacctaggag    300
atcagggacc taccccattc tgttccccgt gcttggaatc agataattta ggacgccggg    360
cccgagaagc ctggataaaa aagtcaggaa aaactgagtt tcgttttgac ctctagcgtc    420
gtggcgggct ggcatctgag ctggtaacgt gcgtggtagg ttatgtaaga aaagggatgg    480
aggagccgcc ccagcgggag ccagaaagga cgcggtgttc tcggttgcaa tccccaccct    540
cctcacccag cagggcagga ggcacccaac ttggaggaga aaggggtggg ggaggtgaaa    600
cagagaccgg agagtcacga gggctgggcc gccgagagca ggagaatata ccgtgtcaca    660
cacctccatt ctctcacaca cgttgcagac acaaatcact gacggtttcc acgtgctgcg    720
ctcgtgagcg gaggtgttca aagaggggc agatgagtta cttcccgaga cggaaccggg     780
ggtcccacgt ccgccgcctt cagtagcaca accaatctct gaacactcaa accgcgcatc    840
tctggcgcat caccatccta tttaaggcca cgggctccgc ccttttcctc ccctcccttc    900
ttttccactc tttttccatc ctcctccttt tctcatcacc gggtcctctc cagcagccgc    960
atgtagggga ggagcacgga atgcttccct tacagccaat catcacctgg ctgcctctct   1020
actggctgag cccctttgta ctgtagcaaa tgggctatgg acttcagtaa ctagagtcac   1080
gtgttaccgg cgacacaacc aatgagggcg cgagatctgt gtaagcaggg ggaggcacgt   1140
gccgggccgc acgtgtctgg gagggggga aggggcgggg cccactgaga gaggcggagg     1200
tgggtagata gacccggaga gacggcgaag gagctggaaa ccgagccagg gctgagccgg   1260
ctgacaacag gtaaggagat tcggaggaca aagcgagctg ttagggagta tttgggagtg   1320
gatttggggg ctgaatgcag atcttggtat tgggagggtt tgcatcacgt ggcaggtgcg   1380
agcccagaga gaccggcgca gggatcctga tcttgaaaga ttgggagagg gcaggaggcc   1440
agtctctatg gggcggttc ccgtaggatc accaggtggg ttcgcgtgcc cagattgact    1500
gcttgtgggt ggcatggcct caggtggaga ggcttgctgc ggactcaggc tggcgaatag   1560
ccctggcgaa caacgggcgg aggggaaggt gtctttcaag tcggtattta ctctgaactg   1620
agggaagaaa agaacggagg ccgcgtcaga ccgagagctg ctctgcggcg gccagagagg   1680
gaatcccaag ctcttaatgg gcgggggtgg gggtagaaat gtctttttct attgtgtttg   1740
agaccggtaa tattggggag ggggagaaca agtattatcc cacgcagtac accccaatct   1800
cccagttcat ttcctaatcc ttaaactctg gtttcaaact ccctcgcttt gctcttcagt   1860
```

tgcaggttcc gatctttggg tactccagga gctgttctat agcccctgct tctggaccta 1920
tggattctcc atctagcgtt tcttcctatt cctcctactc tctctcttcg tcttttccca 1980
cctccccagt gaacagtgac tttggcttcc cctctgatag tgagagggag gacaaggggg 2040
cccatgggcc caggccagac actgttgggc agaggggagg ttcacggccc agcccgggtc 2100
ctatccgctg caggcatcga tcgaaggttt ccggtaacca gcatacacca tctcatccga 2160
aacagcgggg ttcggcttct cctatggcag gatctggggc gaaa 2204

<210> 46
<211> 13135
<212> DNA
<213> Homo Sapiens

<400> 46

ttcgtgcctg tttccgattt tatcctccaa accagacgcc cagcctggtg caaatgcagg 60
agtggagttt ccagggggat gtggactcct tccctccacc cccacctcgg tccctgtctc 120
cttccctccg cccccacctc ggtccctgtc tccttccctc cgcccccacc tcggtccctg 180
tctccttccc tccgccccca cctcggtccc tgtctccttc cctccgcccc cacctcggtc 240
cctgtctcct tccctccgcc cccacctcgg tccctgtctc cttccctccg cccccacctc 300
ggtccctgtc tccttccctc cgcccccacc tcggtccctg tctccttccc tccgccccca 360
cctcggtccc tgtctccttc cctccgcccc cacctcggtc cctgtctcct tccctccgcc 420
cccacctcgg tccctgtctc cttccctctg ctcccatctc ggtccctgtc tccttcccca 480
caggaggagt ctgggggctc ccgcgtcaca ggggcgtggc ccagggcatc aggtggggcg 540
gtgggtactg ggctcagagt ggccttgggc tgtccagtcc ctccctttct ccccaggacc 600
agtgaccctc ccagccccag gacacttctt gggccagggc ccagggcaga gccaatgctc 660
ccccagatac tccctggctg caaacctcag gctggggtt tttggggaca gggatgcagg 720
tgtctaagga cacgaccctc caggcagtgg acgtttctgc ctgggtggag ggcacggtta 780
cgagagcagg gcccgcgctc tggcatcctg gtgcccgggc cctctgccct caaggcctgt 840
cctgccccag ctcagccctc tctggccagg cccatcactg tcagcaaaca cccccacact 900
gctgcccccc cagatggcca tggcagccct cccggggccc gtgtctgcag cccccacgca 960
gcccggccgg ccgatggaaa cgcacaccca cactctgtcc cacctcactc cgagctgggg 1020
gttcggggac cacaggtccc acctgcacag gttgcatctg gggaatgggc ctgtggcaga 1080
aaatgtgcgg cgcggggcgg ggggtcccaa ccaggcagcc ccaacctgga ctctcctcca 1140
cccccgtccc acctccttgc ccccagcaca cggtggcgga gtgcgtgggg ccagcctggc 1200
gggggctttg ctcccccgacc tgcacttaga gaacagagca tcctacggga gagagcagct 1260
cggaacgcag cttgagtaat gccgacttta tatcagcaca cccagtgccc ccacgttccc 1320
gctggcccag gtcccggaga ccatgatggc acccacagtg gacttcgcaa aggagcgtgg 1380
ggaccccgaa ggccaggcca cccctcagag gggggtccca tgctaaagca gacggtgccg 1440
gtgccgcagg gcgtctgaga cccacggtgg agcccgggcc tggcgtgcgg gaggcggcca 1500
cgacggcgcc tttctcccag gaactccggg agggacccca ggactcagcg ccagggcagc 1560
cttggcaggt gcagtgaggc agtgacttgt gggggttaga tgtgggcctg ccccacgtgg 1620
gcagggatca gccaggcatg gggtccagc ggatccgagc ggggccacag agctctggag 1680
gacttttctg cacaatgaat ttctgcttaa aaacagagat caagaggaag ctctatcctg 1740
cagctccgga aatgggtggc tgcgtgcttg gggtctccgc cacgcgggag gacagcccag 1800
aggcaccctg gtctcaggca gctggttcct aggctgtgtc ctccccactg gccgggcacc 1860
ggctggaacg gcacgaggac cagcctcact gttctcagag gggtctcggg ccactgggtg 1920
tggtggtgct ggaggagtcc tgcccgctgt gggctctgga gggcacggca gcagagcccc 1980
tggcttttct aggcagagcg gcacctgccc catagatcgt gccagctttg gctgcaggcg 2040
ctggggcctc accgtgtttc ctggggggc ccggaccccc caaggcctcg atctgaagct 2100
ctgaggtctc atggatgcct gctcctggtc cgataaacga gatggctgtg gtggggagga 2160
gcctcaggcg gcccgggacc ctttggcgtc cgtcgctggg aggcggaggg actcagagcc 2220
gaggctgtgc ccaggactgt cccgggcgcc gggcgatgcc aacactttgt gctggttccc 2280
gccaaggggt cggcctccgg gtccctgccg ccaagtgcgt ggcgtggtct cggcctccag 2340
gtcagacatt gctgatacgg gcctcccccg ggaaggcagc gggatccgtg gccctgtcca 2400
ggggccgctc ccgcctgacg tagcgcggct tccgtactga aacgggtcag agacaggcga 2460
ggtcagagtg ggacctggac agcagggaca gtgcaggcct cagccctcac cggaggctct 2520
cgggagtggg catatggcac aggcgcccgg ggctgtttac tctgaccagg tgggaaccca 2580
gatgcccccct gcccctaagg cgccctcaga gctccagctc gggaaaggga cggcttcctc 2640
ctgcccggct gtgtcctctc atttacaccc tgggccccgg ggcctggaca agggccaggc 2700
gatccccgaa gacagagatg tgggtcccag agtatcatgc acctcagggg gcgacaagca 2760
ggccgtagtc ccaagacctc gaatgtgacc ttatttagaa gaaggggctt tgcagggggt 2820

tacgtcaggg tctcaggtgt gatcgcccag ggttccccgg gcaggccctg aaacaacggc 2880
cagggcccac ctgagagaga ggcagagaga gaagacgcag aggtggggga gacgccgtgg 2940
gaccagggga cagagacggg caacgcagcc acagatgcct gagccggcag gggctgggaa 3000
ggcagacagc gtcctccccg gagcctcggg aagcgtgtgg ccttgtttgc cccttggttt 3060
tggacttctg aactctagga tagtcagatt aaaggtctgc tgttgaagct ggcagccccg 3120
gggcactcgg gcaccgtggg gtctgctggg cacttgcaga cagggctgct ttagggacgg 3180
ggcacgtcca gccttgatgg agaacggtct gtcccctaaa ggcacggtca cctgccccca 3240
tgacagactt ctgcacaggg ggacggaggc cgggtgagcc ctgctcatgg ccttggagct 3300
gtgtggctgt gcgccggtca ctcagcctgt gtctgtggcc ccgcacataa aatgggtcct 3360
aaccgggctg actcttgggt ccgcctgacc cagcaccact gaagcgatgg ctgctcagga 3420
gggcctggcg gaggccggct gcgtctgtcc tcgggtggag ctggacttca aggaggagca 3480
ggtattgggg gtctgccctg tcgtggtggg tcctcctttc ctgcagaccc cacgtctcgg 3540
gaacaggaaa gagcatggaa gtcacgggga ttaaaggggg gcgggcaggg ggcgccggcc 3600
aaatccagct gagatccaga gcagggggca ccagccaaat ccggccctgg ccctgccttg 3660
tgggtgggtc tcctggggac ccgggcaggg gtggccgaga tccagagcca ggcaaacccg 3720
tttggggaca aacccacaga gcaccctggg actggcccag agatgggaaa ccccacaggg 3780
ggatgcgaca gctgctgtta cctgaggact gtggcggggg gatcattgca gccttgaaaa 3840
ggagagaaag gcagagtgag gactcgggat ggcctggcac aggcacacgc ggccagggac 3900
gctcagcccg gatgatgtgg gttacttacg gcttcgccag gctgcagggc cggagctgtg 3960
ggggagaaag cggagagtca ccctggagcc caggagctgc ctcaacctca gcccagtgca 4020
gcgtccggga cagcagagcg aggagctgcc tcagcctcag cccagtgcgg cgtcctcgga 4080
gcagcagagg ccccggggct gcagggtctc cccaacctc tccctgcggt gcatcagcct 4140
ggccctcggt cctgccacat tcctccgggg cactcacagg tgcgggggcc tccctagcac 4200
cccaggtgag tccccgctcc ctggacccac cttcccgcct cagcagaggg gacaagcccc 4260
cctcatgaga agcgtctccc caggctccag ggtcccaggg gaagggctgc cctcccaccc 4320
tcccgggagg agcctcaggc acgaggtggc tttgagcccc acggccgtgg gcttctgcct 4380
ccatggtgcc cccgcgatgg gggtgccgtg gcatcgtggc caacctggct tctcgtcccg 4440
tggccaccag ggctgaccaa gccctgagcc ctgctctgct ccaagggtgc ccataggcag 4500
agatgctgag caggccgggt ctctcggtgc ctggctgtgc aggagaccct ggcagcgggc 4560
tcccgactgg aggctgggcc ctgagacccc gagagaggtt gagggggatg gacaggggtc 4620
tcacagcagc caggtggggc tgcccagaga cctgggagga ggccccagct cggctatcac 4680
agtctggcca agtgctcacc ttggggtccg aactgctggc tggaccatct gcctggcagg 4740
gaccctgcgg gggccggccc tggacaacga gggcaggcac cacggaccct gctcagaacc 4800
cgcccccgcc tgaaggcacc acagtcacaa ggccgtctct ccagtgggaa ggggtcagtg 4860
cccaggcccg gggtgaccgt gtgtacggcg tggcgtaggc tggggtgtgg gccctgcacc 4920
agcctggcca gccctgaccc caggagaggg gagggggggac catggctgct ggaggagccc 4980
aggcaagtcc aactgccgag agccacccag ccccgctcca ctcgcccctc gtgatgccct 5040
gagggccccg gggagacgcc tctgccggaa gagggctcca gcagaggttc tgggccccga 5100
ggaaggcccc ttggtgcttc gacccttcaa atccacacac ggctgtttcc gggaagtgac 5160
gggccttctc acaccgggac ccaaaagcca caggacaggc cctgctccct cccggcctct 5220
gccagcctgc tctggcctgg gacactccag tctcaggccc atctccacgg ctggatgccg 5280
cctgccctgt gccctcccgt ggggcaggtg ctggtccggg agataccttt gtttcttctg 5340
tagcaggccc tggggagttt actggagcgc ttgaggtaga agaacccagc tacggagagg 5400
atgaggccgg agctaatgaa gaacgtgccc aggaagaggg aggcggccac gcgcggagcc 5460
cctgcaaaca gacaccgctg agcagacggg caggacggtg gccctggctc ctcgcctggc 5520
ccccggtgcc ccctgcacat ggcagatgcg ctgctctctg aacatgcctg agcctcacgg 5580
gaccggctgt gggtgccgag ggctctgcac atgggcctgg gtcatccctg caggctcagc 5640
catgcctggg catctggccg cggggtctgg gaaagttacc acctgccccg gcctcagctt 5700
ctctcaggag ctcggatgtt gggagcgtta caggggctga ccctggccgg gctctgcgga 5760
aacacctctc cgattccagt tgcccctgaa agccaggggg ttctcagcct tgttccttcc 5820
tccgggctca gcacaggccc tggggccccc acacagggca gagcctcaag ctggcagcag 5880
gtgctcctgc accccactca cctccacctg cctgggccag gacaggccag ggctgctggc 5940
ctggggctcg ggacagttgg caatggctgc tgggtgtgct ctgagatgct ggggaggaga 6000
ctccccaacc ctgagacaca ggcgtgtttg ccaggcttgg gggcctcgct ccttcctgcc 6060
ccagcacaga gccacaggag gccaggagcc catccaggcc aaccccaccc taaaacactg 6120
ccactcaggt cagcaatgga cccaggccag ctcagggccg tgagtgtgaa cctgagagcg 6180
tgcagtgtgg ccgtgagcgt gaacctgaga gtgtgtggtg tcagcgtgag ccgtgagtgt 6240
gagccttaag tgtgaaccta agagcgtgcg gtgtggccgt gagccatgag tgtgaacctg 6300
agagcgtgtg gctgtgagct gtgtgaccct gagagagtgc ggtgtggcca tcagggcctc 6360
ccctaactac ggccctttag agatgtggca gagcccccca tgacgcccca catcccccac 6420
ccacaccagc tgaccccaag ctgcaggggc tgccacgctc ccctctggct gttttgcaga 6480

```
accctgagcc tgctgtggct gcgtcaaggg tgagggtctg cactggctcc gacggccccc    6540
agccccccagg acaccggcag aggtggctgt ggcctgctga gacttaccag gatgtggccg    6600
cccgggggtc cctgagcttc tgttcatggg gaatggcgca cccgggccag caactgtggg    6660
atagcagaac tgtgggaagc ctgtgtgcac ggagcccacg gggacaccca cgtgcaccct    6720
gaagggatct caggacggaa atatgaaagc gaggggctga gggggcggag cagccctcac    6780
cacagagctg tcccgaatgg cccagcagac tcgggaagag ggggctcccg gggctgttcc    6840
ccagtggaca gtggctctgg ggcaaggtgg ggacacagac agcaactccc acagtgtctc    6900
ccacagctgg agctgtgaga agggagccgg ggggagccgg gcagacctac agcttctaca    6960
ctcggagccg ttgtaggctg ggagggttcc gttcccacag cggacgcagc tggcgctccc    7020
gtccgcgttc cagcgcctgt aacagcctgc gtggggcaga ggacgggcat gtcagccaaa    7080
gcaaaatcca caccagctgg gtctcctggg aatgattctc tatttgagtt ggtgagataa    7140
ctttcattcc agatactaac atgctctgga gggtaggtgg gcggcagaca aggacacccc    7200
cagggagcat ggcggggccg tgagcacccc acgctgagta ctccacactg cgcaccctga    7260
ccctgagtac tccgacccca agtactccac gctgcactct ccacgccgag caccccggcc    7320
ccgagtactc cacgctgcac tctccacgcc gagcacccca gccccgagta ctccacgctg    7380
cactccacgc cgagcacccc ggccctgagc accccgaccc tgagtactcc acactgcgta    7440
cttcagagag tggtaggcgg cagcatccag aatgaacagg cctgttggag ggactcgtgc    7500
tctgcaggac tgcaggctgg gcccggcttc caccacccag ccttcacccc acagactccc    7560
caagaacccc cactgcaacg cctgccccag gcctccccca agaccccccc cacgcctctc    7620
ctaggatccc ccaacggcct cccccaggac cccccccca tggcctccca caggaccccc    7680
gcatgcctcc cccagggtcc cccacgcctc cccgggacct ccccacgcct ccctgggacc    7740
ctcccacgcc tccccaggac ccccccacgg cctcccccag gaccccccac gcctccccca    7800
agacccccat ctgcagtgcc tggagcggct gcctcgcacc ccgtgttccc tcatgccgcc    7860
caagcaggat aggcccaccc gccagcagca cagcctcctg ccccaagctg atcccgaccc    7920
ttggtccatc tgtcacctcc acaccctggc accctccccc gggtctctct cgggggatcc    7980
tgcagaacca agcctcatcc ccccgccagg ccgacgctgc gcctgaagcc tcatttccgc    8040
ccaccggcca gggcgccttt aaggacagga attgactttt actccttgac tttctaaata    8100
tctccaacac tcagcccaga gctgagtaga cacactcttg atgccgactg cggtgcggct    8160
gctggtgcgg gccacgggtg gaccacacgc ctcgaccctt cctcacagac tgatctgaat    8220
acaaacgggc cgcggcgaca cggggacccc gcggtggaga gtgggcctga gaccccggca    8280
cggttgtgac aggggtcact ctgcagacac acctcttgga agaatggaag tccacccgct    8340
cagtcctacc agggtggagc tgcgctctcc cagcactggg ggcagcctct gtctcccggc    8400
atctcctggc acagggctcg ggcgacttca gcgaggctct gtctgagagt ctctgccccg    8460
gaggccgaag aacggagtcc acggcaggga gccaaacacc agcgcagcac tgtccccaga    8520
agtgcccgct gcctccccga gcggagacgt gacctggctt ggggccgcac gcagggggaa    8580
gcgtatccaa caccgcaggc ccggcccctc cccacgcgcg ccagacggtc cccaccctgc    8640
gcctgtgcat ggggccacct cggcccttgg acttcacgac ccgctggcct ggcccctcca    8700
tgggcaccgt gactgctgca cagctgcctg cacccacaga gtgcctgggg tacacaatcc    8760
ctcacaggag acgccacggc cccagcactc ttgctgccat ggggtgccag gggagtggtt    8820
ctcacccagc caccagggct tcagcatcag acgatgagga ccccaaggtg ctcagcttcc    8880
tcccagcccc tctgttctcc ctcagagcgg tgccctcctc ctcctaccct gcgtgtgcct    8940
cagatgtcat cactgccttc ctgtgggctc cccaaccccc acgcccagcc tcggaccccc    9000
gcgcccagcc tcactggctg ccacagccgc cgtcttgacc acgccctcct cacgaggccc    9060
ctgagtcccc aggagcagcc cttctgggtt cttccatcat gggagggctg gacccccagg    9120
atgggagtgc caaggccctt ggaggtacag ctggggcatc tcagccccaa gttcaccaag    9180
ggccgccaag gagtggccct tgcaccttct cactctgtcc tcccctcccc tggctgctcc    9240
cactggctgc cctcaccagc ctcaacctcc tgacacccag ctgcccccag acagtccctt    9300
ccgcagcctc ccagaacctc tgatgaacag ggcctgcccg tggcacgctg acacaggccg    9360
gcatcctcgt ggcgtcctgt tgattggcat tccactccac gcaccagggg ccagcagcac    9420
ctctgcccga gcacggacag gcaaaaatgt cttgggcttt gccagaggcc cctggagcca    9480
ggacagcccc gtgagaatgg ctgagctcat gggccactcc cccagaccct tctccaggca    9540
cctttctgat ctcagctcct gcctctgctc ccagccctct gctgggccag tcttctcatg    9600
aggctgccgc tcaaggccgc ccgccagaaa ggaggctcag atgtccttcc ccaagctcca    9660
cagccctcac tgccccatcc actccccagg tcccgcagca cagatgagca ctgtccttgt    9720
caccgagggg agggccctgg gcacacacac accagtggct caggggccgg tctggaatct    9780
gcttgggaca aagcggctta cctggaccac acagacttgc gcctgggcag ctggcgttga    9840
cgcccaccac atccacacag cactcgggca gctgggcctg gaggggacac ggcagtgaaa    9900
ctccaggacg cgcctgccag gcaggctcac gcccagagca gcagcggctg aggggtgccg    9960
tgggagcaca tctcaatgtc cgcagctccg gcctcttctg gagtcagcat cgggtggccc   10020
tctgtcccct gtgggcctgg gagcaaggag ggcctcaca gccacagtgc acatccctgt   10080
gggaccctgg gtgggcggtc tccaaggaaa agctgggcct ggaagagtaa accacccccca   10140
```

```
ggaggcggca cccgcaggcc atggcagcgc caccgacatt ctctgcaagt ctccggaggc   10200
tctccatcag gggtttccgc ttgaccccac agaggaagtg cccgggcctg gctggcattt   10260
caggggacga attcacgccc aggtgcccag gtactgcaca ggtgcgctgg gtgctggcac   10320
ttacggtgtg tgtgagggcc cattccctga ccacaggcag actggacagg cctggggggc   10380
tcagggtacc ctcagcttgt gtgttgatca cagctgtgct gtttctcgtg acctgaatgc   10440
agtgaacggt gagggcgtcc aggggtgact gcggagtgcg ggatagaatc cacaccgcca   10500
gggaggggcc caggtccggt ccctcaaacg ccccagccag gacagtgagg ggtccccacc   10560
ctccacccgc tccgcctggg agtctggagc cgccgcagct acactccccg atagcgatgc   10620
gcggcacgct gtgctgtcac cgctggcaga ggtgctccca ttgcgggcca ggatgcctca   10680
ccgtgggggc cccgcctgac ccagccattc ctgagcaggc gctcacccag caggcagcgg   10740
agggctcccc tctgcgaggc acaggcccct ctgaccccag cagtggacct ggtcagcacc   10800
tctggggctg ggacatcagt gtcctaaagg tggaagggtt agaccctcta ggggaagggc   10860
ccaccgccct ccacagaggc tctggcttag gccgcgtgga cacgtgaggg gggcacctac   10920
cgtgttctcc atggacttgc tggcgactcc cacgagaagg ccagccagga gggcgaggtg   10980
ccgcagcgcc atgccaggag cagatgcgca gagcctgcca cagggaggag catgcggagc   11040
caaagagatg gagtggggct gaggcagggt gggtggggcc aaatgaaagt ggggtcaaga   11100
gatgttgggg gggtgcgggc caaggcaagg agggcagagc caaatggaga tgggtggggc   11160
tgtggtggag ggtggggcca aatggaagtg ggcggggctg tggtggaggg tggggccaaa   11220
tggaagtggg cggggctgtg gcagggaggg tggggccaaa tggagatggg gtggggcggg   11280
gccgcggcag atgactgagt taaatggaga tggggcaggg ccgtggcagg ggtgcagggc   11340
cacgacaggg aaggtggagc caaatggagg tggggtgaaa gagtgaaggc ttggggcctt   11400
tggaggcacg ggtggggcga ggtgtaggca gggccttacc tgcccctcca ggatggggac   11460
taccgacatc agccctttgc ccgcctgggt gttcaggggt tagctctggg agctcatggg   11520
ctcagctgag ccctgcagac cccggcccag tcctgcagat gaagacagca ggtgaggccg   11580
tggtcacgcg agggcaaccc aggtgggccg tggcctacgg tgcggggtct gggtccggtc   11640
cgggccctct gtccacaccc ttgccggccc ctggctgtcg agcagggcgt cctggagggg   11700
ctgtctccac gagtgtttcc cccgagctgg cctcctggcc tcctgcgggt gagagtgcct   11760
gggatacagg ccctcgctgg gatacgggcc ctcggcccgg cctttgctca gggaccaggt   11820
acggagctca gtggcccagg ctgcacttgg tggacaccgg tcctcccgca caaacccccct   11880
ctctcccccc tgcacttcca gccacaggac ctgttaccat ggccctgcct tcctaaggga   11940
ggaaacgcct gccaggcgca caaatcctca agtggtgtca ctgcactggc tcaggggcct   12000
ccctggcaag ttaccccgag gggggctgag gcccagccag gcctgtgggg ctcctcttga   12060
tgggcctagg ccggggtcct gggacaaggg ctgtgagcag cagggagacg gaggccctgg   12120
aggacacagc tccagtctct gcacagggac ggtccactct ccgcacaggg acggtccact   12180
ctgtcaccca ttccatgctg gattcaggtc atggcctcac aaactgacca ggctgcccag   12240
gtgtgagcct gccggaggac tctggaaggt gggggtggtc ggaatgcttg agctcaggag   12300
ttggagaccc acctgggcaa cacagcccgt ctttacaaaa agtttaaaat tagtcgagcc   12360
tggtggtggg agcctgaggt cccagctact caggaggctg aggtgggatg atcacctgag   12420
cccagaaggt ggaggctgca gtgaacggag actgcactcc agcctgtgca aggggaactc   12480
cgtctcaaaa aaaaaaagca tgctctcctc tgattcagct cctcctctct gatgtgaaat   12540
cctttcagat ggaacgtgtt gaagtcacag acatgctgct cgccccaccc acagagtgca   12600
atcaagtctt agtttgtcct tttgtccctt taacatttgc ccagcagaga ccgtcttccc   12660
ctgctcagtg gaaaactcca gacatcacag acccttctgc tccctctctg gttaaagggc   12720
atcctgaggg ccacattaag tcacaaaaca tcattttgat tcaggaacca gaagtccaag   12780
atttcaatca acactttcat ctgctattta gtcaacttca tggagatcta ctttacatac   12840
aataaaccac atccgtgtaa agtacacaag cgggtgagtg tgaccacccc cttgaagctg   12900
ccaccacagc caggacggtg cccggtccca cacagctgcc agcactcgct gcggccccca   12960
caagcccggg ctcccggcag ccaggagctg accagactgc agctgtatct tctagggtct   13020
tacacaaagg ggttgcacac actgaccttt agatccttcc atgttgtttt atccgcactt   13080
tcttcctttc cacagtcgga ggattttcca ctgtgttggc caaacgtcac cgcct        13135
```

<210> 47
<211> 4852
<212> DNA
<213> Homo Sapiens

<400> 47

```
ctcggctgac tgcaagctct gcctcccggg ttcaccccat tctcctgcct cagcctcccg      60
agtagctggg actacaggcc cctgccacca ggctcggcta aatttttttt ttttttttgag     120
acggactctc actctgtcgc caggctggag tgcagtggcg cgatctcggc tcactgcaag     180
```

ctctgcctcc tgggttcacg ccattctcct gcctcagcct cccgagtagc tgggactaca 240
agcgcccgcc accatgcttg gctaattttt tgtattttta gtagagacgg ggtttcgcca 300
tgttagccag gatggtctcg atctcctgac cttgtgatcc gcccacctcg gcctcccaga 360
gtgctggggt aacaggcacg agccacgacg tccagccttg gacatacatt ttgaaaggca 420
atggggtgac aaagagagga agactgggct gggcgcggtg gcttacgcct gtaatcccag 480
ctttgggtgg ctgaggtgag cggatcatga ggtcaggagt ttgagaccag cctgaccaac 540
atggtgaaac cccgtctcta ctaaaaatac aaaaattagc tgggcatggt ggtgcatgcc 600
tgtaatccca gctactctgg aggctgaggc aggagaatcg cttgaacccg ggaggtggag 660
aggttgcagt gggctgagat tgtgccactg cactccagcc tgggcaacag agcaagactc 720
cgtctcaaaa aaaaaaaaaa aaaagagtaa gactgtagaa gtggtatttg ggtgtttggg 780
tgggaggaat tcgggagctt gatttttaga tgtaagtttg aaatacctag gccgggcgcg 840
gtggttcaca cctgtaatcc cagcactctg ggaggccgag gcgggcagat cacgaggtca 900
ggagatgaga ccatcctgac taacacagtg aaaccacgtc tctactaaaa aatacaaaaa 960
attagccagg cgtagtggca ggcgcctgta gtcccagcta ctggggaggc tgaggcagaa 1020
tggcgtgaac ccgggaggag ctcgcagtga gccgagatcg tgccactgca ctccagcact 1080
ccagcctggg caacagagca agactccacc tcaaaaaaaa gaaataccta ttagacatcc 1140
aactgggtat gtatactaga cagttggata tatgattctg gagttagggg atgagtccag 1200
aaagaaatat aaattacgga gctgtcagcg tagagatggt gcagaaagtc ttgacgctga 1260
aggctgggca cagtggctca ctcctataat cccagcactt tgggacgccc aggtgggagg 1320
atcgcttgag cgagttcaat accagcctgg gcaacaaagg ctggtattac aaaggcccgt 1380
ctctacaaaa aaataaaaag ttagccaggt gtggcagccc aagcctgttg tcccagctac 1440
ttaggagact gaggtgggag gatcacttga gccctggagg ttgaggctgc agtgaaccat 1500
aattgtgcca ctgtactcta gccttgggga cacagcaaga ccccagtgcc acaaacacac 1560
aagccttgac atggaaagag atcaccacat tctatgtgcc ctctcatccc tgctcactcc 1620
attacttggt gtccctaagc tcaatacttc ccttgctgat ctgaggccat acagtctggt 1680
gaaagagggc cggactcttg gcttcttaac acattcacat acgtttgttt aaaatttcca 1740
gtgtttcatg ctttcagccc acaaagcaca gacacagttt taatgagagg ttttcacatc 1800
aggccagaga atgtcagtct tctccatctc catagagatt ttaaaaagca cacctcagac 1860
aaaaaagaaa aacaagcaat taagactatt tcagggctgg gcacggtggc tcacacctgt 1920
aatcccacca ctttgggagg ccgaggcggg cggatcacct gaagtcggga gttccagacc 1980
agcctgacca aggagaaatc ctgtctctac taaaaataca aaattagctg ggcgtggtgg 2040
cgcgtgactg taatcccagc tactcgggag gctgaggcag gagaatcgct tgaaccccgg 2100
aggcggaggt tgcagtgagc tgaatttcac cattgcactc cagcctgggc gacagagtga 2160
gaatccgtct caaaaaaaaa aaacgttttt ttcacaagga attctgagga cttgttggct 2220
caattgcttg gacataaaga cacacataca tgaagctgga gagataggaa gaagcgctca 2280
acacagaaca ccctaagtat ccctactccc tttcaaggac taaatccctt cagagatttc 2340
ctttgcttca gtccttcagg agcctcccga agatgctagg agtcgtttaa gaaggctgga 2400
agatggccgt tgggaaggaa ctgttggtag ctggcaatac ttcatcccaa ggaggcggtg 2460
gcaatagctc cctgggcagc agacatcatg gattttcctc cctctgggaa ccagaggtcc 2520
ctccgtttct ggtttgaatc ccttagagac aaagaacttc attctctccc atacccgcgt 2580
gggactgctg gggaggagag ggggtggagg ggttagactt ctacccccat gaaacacccg 2640
cgggctcaaa gaggagcctg gatcctgact ggtccaggag gcgtgcgggg ccgcggggcc 2700
cctccggctt gggccaggcg cgttgccaag atgcccgggg aaggggccgc ccggcgcagg 2760
ccggggacgt ggagcgctcg gggcgctccc acgtaggcca cacattggcc gcctgtatcc 2820
tccttctttc ctctcaactc cattctctcg cgcttttttc ccggggagcc tccgctttcc 2880
ccttcttccc gcggccgccc tcgcccttct tccccctcca gaccctcccc cggctcgccc 2940
ctccctcggc agcccacctt caccccgctc ctccgcgcgc tcctcggtcc tcgcccctca 3000
gccccggcct cccctcgccg gcccccggcc cccgcggtcc cctccctgcc cccctcgagg 3060
cggcggcggc ccctttcaca atagcgcggc ccgcctccct cctctcggct cccggtgcac 3120
tcccgcccgc tcccctcccc gcgcatgcgt cctccggttg gcggcggctg cggcggtggc 3180
ggctacgagc ggctccgttt ttttaaaggg aaacgctgag gcgccggggg tgactgtggg 3240
ggaggggggac cccgagccgc ggagaccccc gggagaggcg acagaccccc tctcccggag 3300
taggagggct ttgggcggac ccagccctcc accccctcct gaggaggagg gggggaaat 3360
ggaggctcgg ggcggttgag gcgagctccg gggcgggggg ccggggcggg gaagggggggg 3420
tgcggggtgg ggagacgagg cgggcccggc cgggccaggg ggggccgaag cgagctccgg 3480
ggatgagctc gggggcggct gggtgcgagc tcctgcctct gaggcgaagg cggcggcggc 3540
ggcagcagag gcggcggcga ggcccccatg ggccggcggc gggcctcagc cgcggcctcc 3600
acgtctccgc acgccggcgg gatggcgccc gggccccgga ggagccgcgc tagcggaggc 3660
ctgctgccgc gctgctgagg cgagcccgcc aaactcccct ccccccccctc agtcctcgac 3720
cccccgcacc tcgccccttc cccacccccct cctccgcctc ggtgcccggc gctgctccgg 3780
accactatga ccatgagatc cgcggtgttc aaggcggccg cggcccctgc cggcggcaat 3840

cctgagcagc gactggacta cgagcgggct gcggcgctgg gcgggcccga ggacgagcct 3900
ggggcggccg aagcccactt cctcccccgg caccgtaagc tcaaggagcc ggggcccccg 3960
ctggcctcct cccagggcgg gagccccgcg ccttccccgg ccggctgcgg cggcaagggc 4020
cggggcttgt tactcccggc cggggcggcc cccgggcagc aggaagagag ctggggcggt 4080
tcggtgccct tgccctgtcc gcccccggcc accaagcaag ccggcattgg gggggagcct 4140
gccgcagccg gagccggctg cagcccccgg cccaagtatc aggcggtgct gcccattcag 4200
acgggctctc tcgtggcggc ggccaaagag cctacgccct gggctgggga caagggtggg 4260
gcggcctccc ccgctgccac cgcctcggac ccggcgggac ccccaccact acctctgccc 4320
gggccgccac ccctcgcgcc caccgccacc gccgggaccc tggcggccag cgagggcaga 4380
tggaagagta tgaggaagag ccctctcggg ggtggtggcg gctcgggagc ctccagtcag 4440
gccgcctgcc tcaaacagat ccttctgctg caattggacc tcatcgaaca gcagcagcag 4500
cagctgcagg ccaaggaaaa ggagatcgag gagctgaagt cagagagaga cacggtacgg 4560
gaggggttaa tctgcattcg ggccgaggag cgagttgtgc gcatgctcgg gggaaggggg 4620
ctgtaggagg ttaaggcacc cccgggttag ggtaaagga ggttggccac cggcaaagga 4680
gtatcttagg cgctgggtct ctgggagcca ggctcacaga cacgttgggt tggagggaag 4740
ggttaaaggg caacctctca gggggagggg gaggggtttc aggtgccaaa gggttaattt 4800
aaaatgacag ccccagacgt gtgggaaaga cgtttagtgg aaaaagacgt ga 4852

<210> 48
<211> 2519
<212> DNA
<213> Homo Sapiens

<400> 48

tatgcaacgc tggattctaa atcggtctta atttctaaaa agcctacagg cacggatata 60
aattctcagc tctcgtggct gtggccgccc tatgcactct tcccagtggt ggacacaaac 120
gttaatgtac ttgttattat ttaaggacac aaaaatatag aaggtggcat ttttcgcttt 180
ccccttcgaa gactctcccc agagctgcca gatgaacgcc cacgccacct tctcccacca 240
aaggtggatc cagctaacag gagactggat cgcgaccata gcgcgtttgt gcccagttct 300
ggggaaagcc tcatgttagc gagtggccct cggctcgtct cgcagatcgg ccaagtacgc 360
ggaggccgcg cggtttcacc attaatactc ctgaaggaca atagctcatc aagccccacc 420
gaaagccccg gacccgtcgc ggagcctggg ctggcggggg atgaggctga gcaactcctc 480
ctagaggtgg ttatgtggag aaggagcaat cccttctccc tcctcctcct ccccccgcta 540
ctatccgcgg cccagagaac tgccgcttgc cgccattgac acgcacagat agaacccaaa 600
gaaaggcaaa gagtcctgcc cggcaccggc gccgcgtggg ccaaacctgc gcccgtggag 660
gggcgcgcag agggcaccgg gcgccgggag caggcggcgc agcaccaggt gagcccgcca 720
tggcgatggc attattgttt agggtgctcc ggggtctgga aacaaaacgc gccgccgggg 780
cagtgcaggg gccggagaga accacctctg cctctccccc gagcccctgc gggcgcaccc 840
tcgatagccg acccaccctc tgcaaaaaca aattcttaaa tccagctgcc caacttgctt 900
ccttgggcga taaagggggg gctgctaaag aagtggtcaa attcctgcgg ccagcgaggg 960
gccgggggcg tcgagggtac tgagagggga aggaggaggg atgcgaggtt cagcagcggg 1020
ccccaggcga ggctggaccc ggaggaagcg gaggtctcct gggaatgcgg agtttcgggc 1080
gcaggagcgg gaggacggag gcgcccggcc gccgatgggc acggggcgat gggtgggggg 1140
ctgcgcaggg cccgagggag cccggggagg gccgctcggg ggcctctgaa ggtgcccggg 1200
cagggcgccg gcggctccag gctgacaatg gagggggcgc ggagcagccg ggcggaggtt 1260
agggacccga gtccgggggc gggccgcgcc ggggaaggtc aggtcgggga cgccgggtgg 1320
cggggcgagg gctgggggtt gctgcgcgga cacccgggcg ccgcgggcgg gcgtgcgccc 1380
cggggccggc gcggaagagg gcgcgaggcc gggcactccc cttcggttcc ctttccgggg 1440
cgcccggtgc cgacgcgcgc tgcgctttca ccgggaaatg gcggcccggc cgtggctcgc 1500
ctgggcagtc tggctcccgc tttgcgcggg aaaaaataat aataataaaa atatcgaaaa 1560
agggaataaa agtaaactgg ctgcggcgca ggaaagggct ctgccgggcg ccggacgccg 1620
tgcgtatcgc tcgcggggcg cggcggttgg ggaccagccc tgcctccccc ggctcccagc 1680
ctccagcccc ggcgcacctc agccccagcc cggccagccc cccggatcgc ccgtgggtgt 1740
cctcccccgc ctttccttgg gggggcgccc tcagttcttg ccctctttgt tccttacaac 1800
aatggccaaa ggggcattgg ttccccaagg gccaaacgag cttcctccag gagtttcccg 1860
tcgcgtgcag tggaccaccg tgggcctgac tgaggcctgg tccttgccag ggttttttgg 1920
caacggccct ggatgagtga agggatgaaa ggcgcgagaa gaaatgggct gagaaaccaa 1980
agtgcggggc cctgagtgtt tgaggggacg cgatgggaac cgtggctccg ctgccctgat 2040
tttaagggca gttaccctgt cacagccacg ctggaactga ggtgtccttc cagcttctgt 2100
ccacctcaga ggaattcaaa acaagggccc agttctaagc cccacagacc ccaggatccc 2160

ctaggggcca ttagtccacc gcggcaaatg gtttcctcac tttgctcatt tgtgtttgtt 2220
ttgaaatcca attgtcacct cactgggctg cacagttgct actgaaacgg ttcaataggt 2280
gagagaaaaa gaaaacatta ttaagagcga gaacgtgtct tcgtgtgtgt tgttttgaac 2340
cacaaactaa gctacacata attctcaatc cgttttaaca cactttttat tttcaaacta 2400
aatgttaaga gctccaagat ttggaaacat actggtgatt tttagaattg ttttagccta 2460
aaattacata taaataaata gaggcactgc ccggcggaaa agtttatctt gagtcgcat 2519

<210> 49
<211> 9783
<212> DNA
<213> Homo Sapiens

<400> 49

ggctacatca gacctctctc caatcgctct cttgaattcc cagatgatcc agagcggccg 60
gttgactttg ccggcccacc ctacacctag ggaaggaatc cgttctggag ataccaagag 120
atactgcatt ctcaacttta aaaccagagt gagggcggga ctcggcctcg ggatcccagt 180
cccaaacccc aggatactct ttcttggttc gagtatgaag aaggaggatt ctcaatctca 240
ttttctctct ctctctctct ctctacacac acacacacac acacacacac acacacacca 300
cacacacaca cacacacaca cacacacaca cacacacaca cacacacaca cacacacaca 360
cacacacaca cagagttgga tccttcccca tctcccttat cctcaacaca agcgggctcc 420
aggaaccgtc cagggcggcg tccagaccct gccgagcagt ggcaggcccc aatggtcaca 480
tcccgcccct attcccgcca tacctccctc ctggcctgga ggaattccca ccccatccca 540
ctctaggtcc cctgcggccg cccaccctgg gggctggggc gtctgagttg aagttccctc 600
gcccctcgcc cggcggggca ggttccggga agctgggagg agaagggaag gggctccgac 660
tcggacctca ggaggccaga gcccctgccg gcaggtccgg gccccctcgc tgcagcccgg 720
ggaggggccg acgcggtccg accaccgccc agcccggatg ccgactccgc ttggagggaa 780
cggggtccac gcccaggccg aagcccctcc tcgcacgggc cccgatctcc gggccagcga 840
ggaagggaaa gtgctctcct ggggacgagt tcccaagacg ccccaccgcc cgcagccccg 900
gcccagggta ccttccgctc cgccgcctcc agctgcgcgg ccggtgccgg aggctcagac 960
tgaccccagg gccccgcccc gccctcctcc agcgccgcgg cggggagggc gggcccggca 1020
gggggagggt cggccggggg cggcccttgg ctcggcccgc cccggccccg cgcagggccg 1080
cagcgcggcg gccccctccg cagaaaaaaa actgggggcc aggggcgtcc acccctccag 1140
gtcggggaga gccacgggga gattcctgga ctgcggagga gccgggcgag cggggggagc 1200
acgacccggg tggactcaga ctctgcccag ccttcaatcc cctaggggag gaccggcgtc 1260
gggaaagtta agaggtgctc ggggtggggt caggaagggc cgctgctgcc ctcctgtggc 1320
caagcggcca cccgctcctg ctgcacctgg gacctcgcgg gaagcgggac tgggaaaggg 1380
tatccgccgg gaggtcccag ggcccggcgc gggtgtgttg gttgggggga gatgtccacc 1440
ctcttgttcc taggatcgta cagcccaacg tcatccccca aacttgaaga cctctcagag 1500
ccgttcagac ccagaccacc gttcctcctc acaattcgct ttcttccctg ggcctggaat 1560
ttccaggagt ccaattccta cctactcctc aaagggtgcg cctagaaatg cttctggatc 1620
ccagctgtga attctgttcc cactcctagg tctgacttgg cgcataggat atttacctac 1680
acgcgtatct cctttccctg taagggagct gaggaggtgg gagtagaatg aggacgtcca 1740
ccaacatttc attggcttgt gcttgggaca ctacacagct taaccctttc aatattcatc 1800
acagatctct ggggaaaata tacccatccc cttttgccca tagagaagct gaggttcaga 1860
gaaatcaagt ggcttacaca actgtttttg acttgtgaca gcaagaattc acacagccgg 1920
gcctgtctgg ctccaaactg gtggtctggc cctgctacct cacgggctcc tccagcctgt 1980
cccttccccc gttgtctagc agagtgagtg gcacataata ggttgtcaac aaatatttga 2040
tgaatgaatg aatgttagga gtcatacaga ctttaaatat ctgaagtgca atccagacat 2100
ttgacaaatg tgaaaacagg tccgcagcag tttggcccca ggcctcttaa ctgactcaat 2160
gaacatttct cgagggtgaa ctgagagcta tgttcggaag atccatttct gccttcaaac 2220
aacttagagt ctaaatgagg aatgcaattt caagatagct aacatttatt gaatccatat 2280
tacatggacc tgggtccaca tacattaact acaagaatcc catgaggtag ctatctccat 2340
tttactgaga agcaaattga ggttcagaga ggttacagag ctagtgctgg gtctgacact 2400
agaacccaaa cagctgaact ctagagcccc tgttcttaat tcctactccc tactgactcc 2460
taatgtagac agttacaata ccggaggtca ttgctgtagt gtgggcatgt gtgggctgtg 2520
cgatacccag tcctgaacaa caggtgaagt tctcaagata aggtgatgtg tagatttttt 2580
taattttaat tttttttga gacaaggtct ctgttgccta agtgtgcagt ggcaagatct 2640
cagctcactg cagcctcaac tttccaggct ccaatgattt tcccacttca gcttccccag 2700
cagctgagac tacaggcgtg taccatcaca cctaattttt gtattttttg tacagatgag 2760
gtcttgctat attgcccagg ctggtgaggg gccttttcta attattttgc tcatctttat 2820

```
ttttgcttcc cttaacttac tttgggttta atttgctgtt ctttttctag cctcttaagg    2880
tggaaacaga tcactgattt gagatcttta ttcttttcta ctataagcaa tactcttaat    2940
ttccttggat gcttaactgc ttcccacagg cactcaccac tacattcagc taattttta     3000
tttttttgta gagatggggt ctcactattg cccagggtgg tcttaaactc ctgggcttag    3060
cagtcctctc acctctgcct cccaaagtgt tgggattaca ggcgtgaacc accgtgtcca    3120
gcagtagatt ttgaataagt tggagctact aaaagcccac agttatgcaa accccaggaa    3180
gttggtggtg gtggtaaaaa atgaagctgg agaaggaagc agtggctgga tcatgaaggt    3240
ccttatatgc catgcacagg agttgagtgt tgattttgtt ctaggcgcaa taaggagcca    3300
ctaaatttag gaggccaata tagtaaaagg tggtggtgac tgaaggcggg gagagatgga    3360
gataagtaga gaaatacttt taaaggtcta tagtcaacaa tacttagtga tcaattacct    3420
tgatgagaaa gaaatgaaag ccaagggtga ccaactccaa gatttctaac tggagcagca    3480
gagctggtgc ccattgaggg gatataccag gagaaggaac atgctatgga gggcagtcgt    3540
gaatttagtt tgaggtattt gaattgaagt gccagcaggc tatccaggag acattcggga    3600
gatgactggt atgtgtgggt ctaaagctca ggaagggtct gggcagatgg gtgaggccac    3660
agaagtggat gatgacactc acagcaaatg cgtggagtgg aaagagtggg ggtggaataa    3720
agaacctggg ggacaccaca tttctagaac tgttctggtg ctctttccac caccccacag    3780
agctttcttc ttatctttca gtttctagtc aaagcaaata cagacacaca cctttatcca    3840
tctcgtttat ttgccaatag ctgtaatttt ggatatagcg gacatccctt cttcagttaa    3900
gttgccacta aagaagggtc agggagacaa cttcagaatt tgggctgtga gaggagtaag    3960
tatggagagg ctagtgggag ggatggtgtt cagcccctca ggagtaactg cctggccctc    4020
ccaggggat gacgaagaca gagatgtcat ccccggaacc cagcttgttg ttggggagac     4080
gccagccacg gtctcggggg gtaccccggg cccccaggac cagagcttgg gccagagctg    4140
tatacctgcc aggagcacac atgcacattc atgtaccaac atgtatgtgg gaagacagat    4200
acacaggccc acatgtgggg tcccaagcat tgcttccctt tgacccttct ttcaccaatg    4260
ccatcccctt accaccatgc catgcagccc ctacctgctg tggtcattag gctcataggc    4320
cgacagcacc ctgtccacag tggcagctac ctcacagtca gtagtgacat cccacaggcc    4380
atctgttccc aggactagca catcatctgg gcagtgctca tattgtgtca ggtcatacac    4440
tcgtacctgg tgggagaaaa gggcagagag gattgaggtg tggggtttgc tggacaggga    4500
gcttggagat aaagggactg cagatgaaag ctggcctgag actcccagag aaggcaaggt    4560
gtggtttaag gggctcacct cagggaagca ggagagaaag ggcttgatgg gcagggtgga    4620
actgcagacc ttaaggctgt ggtctcccaa gcctcgggtc accccaatgg tggccatcac    4680
ccgagcctga aagaaatgag gagggagtgc cactgtagtc ctctgctaga ttctctgaaa    4740
ccaaagatcc cttccacccc taccctcagg tagaacctcc acctccctag acaagttgtg    4800
ataggtcctc ctcccctctc ccacctctac catggagttt accttttttgc cctccccaca   4860
gaccagagga aacctgagat cctccagctc gatcttttttg taggccctgg ggagggggga   4920
gtagaggagg catcacccag gcatcaaagc ccctctcccc taggagctgc ataccctcac    4980
acaaacccct tagaagggcc acatcctccc aactcccagc ctccagcttc caccttgctg    5040
cgggcgaggg actggttgtg ttgccatggg aacgggcccc agctctgagg gaagtgttac    5100
cagccggtca tgttctggtc ccggtacaac atcctctgcc ccagctcctt gggcagaact    5160
ctgcggggga actcaaggtg ggtgaattca ctgcctagca gctctggttt caggaagccc    5220
tggagttggg gatgatcaga acagagagcc agggggcatt atgctcacag cacacggtgg    5280
cacacagatg acgttgacta cagcagaccc ttaaacagag tttaccatgt gccacacgtt    5340
attctaatat tagcaaatat taactcattt actcctcaca acagccctta aggcaggaac    5400
tattgatgca cagagaggtt aagggaggga actgctccgg tcacacaatt agcaaagcac    5460
tcagaccagg atgcatcccg ggcttccagc tccacagcca catgcgttta ggcactaagc    5520
cggactgcct ttcaggggcc tttgctgttt ccttccccca cctcagtctc tacttcctgc    5580
ctcagctggg gccacagtgg gagcactgga ccagcccagt gctgcagcct ccctgctgca    5640
acctcccacc ggctcagccc cgcactccat agtccacaca gcggccttcc cagtaaagtg    5700
ctttctgtgc ctgtggtggg ggtgtgagag ttctcagggc catggggtgc agggaggcac    5760
aagacccgac aaagaatggg ggtagggaat ttggttccta cttacaagca gctgaagacg    5820
ctggcgctca gtctccgggg taaactcccg ggacattgga atgatttcac cattccggac    5880
aatgatggcc ctgcccaaag aaagaaaagg aattgggacc tggttctctt cagacccact    5940
catgaaaaat tccagaaaga ccaggccctc tccataatga cctttcagat ctcttaccca    6000
gacccccata tctgcccctg accccaccca atggatattt caggccactc cttatgtctg    6060
tcacccctct tgactccctc caatcttgat ttttaacccc tgccacctta ctctcctgta    6120
ttcccccaac tcagagagtg tggccctctg tcgccacctt ggagccccct cccccatacc    6180
tgctatcgcc tgcattggcc acgtacacct tgcctagcag gtagatcaca accagtgcac    6240
agcagccccc ctccacttgg tggccacgcc gctcccgggc catctgctca tcctgccaca    6300
taaggaaggg tcagaggtgg gtagaaggag aggttccatc acacagtcac cctcccccta    6360
ccccctccac ccgtagaaat caactggccc agccaggcta cactggaaga caggagggaa    6420
gactggggtg taactgcccc ttctccctcc cctctgaacc aagctctgct caggctctca    6480
```

```
aaggtgttgt gggttctcag ggtaaccccc tctggcttcc acacagagtt ggccagagtg    6540
gttgcctagc aggctcttgg cccaggtcac ccactcacca tgagctggaa ggcattctca    6600
acggccccca ctaccaggct ctcgtggctc acttccttct gtgaagacca gcaggactga    6660
gggccaagca agtgagaggg atcggaggaa tctggggtcc ccggagtggt tgggaggcag    6720
aggggtggtg gcgaagggtc ctgaagtatc tctaccaggt cctttagctg ctctcggata    6780
tggcgatgca ggagccgtga ggccatttca gcagctccgc cccctgcatg cccatcaaat    6840
aggccccagt agtagaagca gagtccctgg tggaggaaaa gttggaggtg agttttgttt    6900
gtgtgtgtgt gtgtgtgtgt gtgtgtgtgt gttatgcaag gtgaataaca agactctggg    6960
catccataca aacatctgag aggtcagtgt ttgctactga aggcaaatta ggtttattca    7020
cataaggctt cgtatgtgtc aagagaaagg aaaggctcaa aaacccaaac tcagaggata    7080
acagatccaa aactcagcac tggcaattaa atcatccata aatatgtgag cctctacatt    7140
ttaggtggga aagtaacatt catgcttaca tggtagaatc taactgatga ataccaggta    7200
aaggttctgt gccagaaggt agatagccag tgagttcatc aggtcttccg catggctcag    7260
atttaaaacc agaggcctga gtccctgggg agaggtgtca ctgtgtctct gggaaagggg    7320
atgggtctta cctggcctcg gctaggctcc ctaggtactc ctgtaacact cctccgacct    7380
tccacataca ccacttcaca gcaagcctgg tcctcattgt gccgactctt gccagcattg    7440
atgaccctgc caggccagaa tgaccacatc aggttggaca ccagaccagg tcctggaata    7500
acccccacct tagacacaca ccgtctcctg cgcccgctgg ccaagctgag aaaggtcaca    7560
gccacctggg atccaggcca gagggaggag gagaagggag ggctggcaag gtgtaatgag    7620
tactgtttgg gtggccagcc ctaccacttc acagaaaggc caccaggacc ttctggccaa    7680
ccgctatgtc ccctgcctgc ctcacttccc ccatccctgg caaaaaaaaa agaaaaaaga    7740
aaaaggcatt cctagtggtg cttctggggg aggggctagt tggtagcaga agttttactt    7800
ccctgctagc tagaccctac atcccaccct caaccactat caactctccc agtgccttgt    7860
ccccagcacc tcatcctcat gggcacaccc acccttatcc taaaaccatc cagcgaaagt    7920
ttctgctgcc ttaacaatga ggaagctctc cagcccagat cgggaaagag gaactgaaat    7980
agagtagaag tgaaagccca cctggactgg gcaggagcca catacccata aactcctccc    8040
acacaggagg ctaagggagg tggtggtggg tccatgggcc accagccacc ttcctcagcg    8100
aactgagcgc ccccatctcc ctcggtgctc tgcgttcccg gcagatgcat ttaaaataca    8160
cacagcccgg gggccagccg gccgctccgg gcagggggtc cgcttctttg ccaggccagc    8220
acgagttggc aggctgaagg gcatctaaat gggaccctcc tctaccccgg gcgtccccct    8280
ccccgctcct ggggaagctg gcggcctcct ggtgtcgcgg gctatttctg ggtgtggagg    8340
gggaggggaa ctacggtaag agggacaggg aactggggcg ctggagccgg gaagcgggtg    8400
agcgagtctt cccaaccgct atgtccccta ttaaaaagaa ccccgctcct tcgtgaaacc    8460
ccttttccct tagcctttaa gctgccgggc cgcgacgggg aactggccaa ctcatccttc    8520
gccgggctct ccgccctccg ggctctgggg cagaacaggg tcctgaggcg gcgtgggcga    8580
gggcagtcaa tgagcgggaa ggacgtgaag aaggtgacac aggctgcccc ccactggaaa    8640
atgggttggg agggggtgcg gcccgaagac cagaggatag caaaggtcca cggaaaaggg    8700
agctcctggc ccggacgcgg cgtggggaac gcgtcctagc gccggggatg cggagctggg    8760
gagaggggtg ggagccccat cctggtttgg gtgccccagg gggcgctcac tcggcgtagc    8820

ctgtgctcca gggcaggcgg cggcccgtgt ccgggggggct ttgcacagcc cggcccgcgt    8880
ggtcatcggc gcgtcgcagc cccccggggc tcagctgcaa aaaggtcggt ctggagaagc    8940
tcgctcgagc ctcaacagcc ttcgcgggcg tcgcgctccc gctcccaacc ttcgcgggag    9000
ggctcctggg cgcttctgga gcggcggcgg gcggcgccga ggcggcgttg ggcaggtccg    9060
gggatttggg gcgcggaggc ggagcgcccc cggagctcac caggtgcgcc acggccgagc    9120
gcacccggtt tagcatgctg cctccctgcc ccgccctcgg ccgcggcccc gcccccgccc    9180
aggccccgcc cccatggctc cgcgtgggcg gtgctcgcgc cagccggccc ttccctgcta    9240
ctggctcccg gccagctggg cgggtcccgg gcgaaacttg cccttgctct gggccacccc    9300
aggaaccggc ccacgggtcc ccggcctctg ccccgcccca ttccggcttg gccccgcctc    9360
cgtttcttag ggtaggttc tgttgctgtg agaaggggggc ggagagaagg aaactttatt    9420
tcagccgtgc gctagattag acacagctag aatgtctcca ttgtgacggg ggctgaggga    9480
caagggggtg cggtctcctc gctctcggtt cggtgtggcc agccagccca gagagccatg    9540
gagctaatga cctcagagca aaacaggcgc ttgggagccg ctgggtatga ggcgccaggt    9600
gtgacaccac ctctggatgg ggctgctgga gcgagagcgg gcaagctggt accgggtggg    9660
gttgcgggat taatgacgca ggctgcagcc gaccaccccg gaatcaggag ccggggtaga    9720
ttcctgggct cctgatgcgc tgttccccgt gaagaccacc tctagatctg ggagagcggt    9780
tgc                                                                   9783
```

<210> 50
<211> 13537
<212> DNA

<213> Homo Sapiens

<400> 50

```
tgtacaacca ccgccaatcc ggtttccagc agaggcttgt gggctaggga gtgtatcgat      60
ttgtattgtt aaaggaattt cctgctcctc tcagtgtcag ctgaagagcc ctctgcagtc     120
attctcctat tttgagagaa gagaaacaag aggagacgaa ctcctggttc cttcctacgg     180
ggtgtataga ccagtgacaa tgttagtcct acaatttgga tttatttgtt tgttttctaa     240
tcaccgccac ttctccaagt acaaagtctg tgtctataat tagatatctt ctacatgtta     300
caccggcaag tccatctcat cattttacag agggatggtg acttccttta gcgccccata     360
ctgaccgcgg tccctcacct gctgactgat tgacttataa atctgccccc cacccacttg     420
tctgtccatt gaaggcaaat tcgccatcgg gaacacagta gtttctcaat aactatctaa     480
tgcatggcca cccttcccag gatttagaat atcaaaggga cgtgttcttt cctcccaaat     540
cacctgcctg cgcttccccc agccgggtgc cccagccttc acctcggtcc ggccaccacc     600
tcatcccgct tcttcgcggc ttccaggcct cgggctggga tctcagcccc tgcctgtagg     660
tgcggcccag agggtgcgac acgcgtcgga gcggggcagc ggaggagggg acagggacgc     720
ggcagccaca cccgcatctc tttcccctct cgggcatccc cgagggccct tctgcctcca     780
gttccttgag cccccgaagc cccccatgcg gcaagagcga gtcgcaggaa caagtgggga     840
atcttggccg cggcccccctc ccccgcctta taaaaaagaa attgactttt gtttggagtt     900
tgtgaaaagt ggggctcggg gcccagtcaa tggggcgccc cgcggcgcgg gctgagtgga     960
gctagcgcga accgctcagc cgcggcccca attaatccgc cctttgtgcg gcccgcccgg    1020
ccgcccccgc cgcagccgca ccagcggccc attgttcggc ctcgccgggc cgcgggattt    1080
acccttttca aacagccggt tttgtccagg gcagttcgag cggaagtttc tcactgacaa    1140
tttgccccaa atagatagat ttgtcagaag cgaccttcgg gaaggaagca aaaagccacc    1200
ggcccgaagg ttgggcccaa aacagggact ctgcgtccca cccgcggccg cgccgccccc    1260
ccgcgccccc ggccctgcag tcccgaagcc ccgcgggggt ccagaccact gcacctgctg    1320
agccagacac tccgcggacc tgacctccgt tttacccatc tgtaaaatgg agcagctggg    1380
cgctggcaag ccgtaagtcc aagagctttg gctccaccag cgtgtacgac gctcatttcc    1440
tttctctctc cccacaccct ttccaaaaac tgtaagccag aaaacctggc caggcctccg    1500
cgacggcgag aaacggtctt gaaggtcggt cggtcgccgt aatgcggtcc ggatcgtgca    1560
cgtgctgttt cgccactgcc ctccggctcc tccttaacaa agatgtcaac cgtgctaaat    1620
cacaggtggt gcctttgtaa gaccagctat tgtcccctca taagtcaaga tataaaacgc    1680
tttgtactga atggcccatg atctggagcg ctcaacttgg gagttgagct gttagatttt    1740
cttttcccca cccaccagag ccactcttct cccaaatgac ctcacttaat aagcaaaatt    1800
taaatggggt ggggggggac tcaacatgct gtggatgaaa agaatagtta ctgatttctt    1860
cacacttaat ggcatgtagg agtcttttaa aatacagtct cataccaaac agatctgtga    1920
attttaagca gaactgaatt gtattaatca gttgatgggt ttgttcatct tctatcacta    1980
gatgagaaat acaaaggttg ggaatccaca gcgtctaatt ctactgtact taagcaatct    2040
ctccctttag tccagaaaat ttcctactag ttgcacatga gagaaaaatt gaagcaaaaa    2100
aaaaaaaatc tgtttttttgt ttatccatca ggtgaataca cacttcagca tcatacagag    2160
gaaaccctag gtggtcttta gcaagatttc agtggttaag agctacaatt tagaaaagat    2220
gacaatacat acacagtgtc aatagtattt aaacaaacat cccagacaac agaataatgg    2280
ttattaacta tgatgctttg ggcttctgac taatttccca tgtctgtctg cagccaaaca    2340
gctgcaggat ttttccccct ttaaaaactg tttgtgcatc attttaaaaa ttgcatttaa    2400
cagccatatt ctccagagca gtccctctgt gtggaactga agtgaatggt ttagcaatag    2460
cgatgaagcc agtaagtagt aagaatgtct ttcatgccaa aagaactcta accactctgc    2520
tagaaatggg ctacattttt actctctgaa atgtagaagc tgtctgctac tgtatgacct    2580
tcagtatggt ttacatgtct aaataaatgg aattatttaa ccctgctcac acttgcacta    2640
gtgttttcaa aattgattaa gttgtgcaac tgtactaaag tgtacctttc ttggggtttg    2700
aaaatattaa agaaatcaag agagggtaag attattttgc aaaattgctt tctcactctt    2760
ccagataaat attacatttt aaaaattact tggtgatcca aagattaaaa tgaatgcatt    2820
tctgtacact ccaaatatgc aattccaatg attatttagc tactgcagct ggaaatacat    2880
atatttaggg atacagatat gtacaattat gaatacttgg gtaattacac attgtaacta    2940
actattaatg catggactaa aatcaaatta cctccactgt tttcattgtt atttagtact    3000
taacaagtat ccacttggca ctaaaagatg gtcaaaatat atcaaacaat gtcagccttc    3060
caaaaaggta tcaaaatact tatatcctag atagatacac atatgttgta tgtagtgcat    3120
gttggtgttt caaatatctc attatttaat gtcctgatct gatagaaatg ttagatttca    3180
aatcactgtt tcacctatcc caacagttac acctgaaatt ttagaggcta aggagaaatg    3240
tctgcaacct acctcctcag ttgtgaactc ctgtacctga aaggccaaat cctcctcctc    3300
ccagcaggca atcatcttat gccctgaagc atgagatctg attacccccta tcatcatctt    3360
agcttgcata gatacagatg ttattaacgg ctgtagttta tccaaccctt ttttaaaacg    3420
```

cagctgcagc atttgacttg gggacctcct gtgaatccca ctggttgact ccgtcttgca 3480
caaagcacgt ggggtttggg ggagggagaa gagagaggga ggtttgtttt gttttggagg 3540
gttttttttt ttttttttg aatcagagtt ccctaaatag ccactcggca tctttgtaag 3600
tgatgtgaat tctgaaatta cttactagaa taggacaaca gagtactgtg tgaactagat 3660
ctgttggaaa caaagcatag tagaatgtat gatgcttttc acttcattgg cagagatcaa 3720
atatgtggtc ctgatatact catccacata ataaagaatt gtagcttatt ctgaaatata 3780
ttggccacat tcaaactgga gtacctggtt ttacataaca attgtttcta caagtgttga 3840
aacagtaatc tggataaagt aggtcctaaa gctgctgtga agggaagaca gggacgtttt 3900
taaaatctga aaggcctaga ttggcaatcc aggcagatca aattcagcga ttaattggtc 3960
aatctaatca ttgccttttt ctcagcatct atgatttgtg tctctaagta ttgctcctgt 4020
aggtcatatc atcccttcta taaaacagag aggacacctc acaggtttcc tataccttgg 4080
aacacagcaa attttaagta ctggcagaaa gggaatttag aaaaaaaccc tgtgaagtaa 4140
aggaggctga cgttgcacaa atactgaact atagtttcaa gatgcacaaa acactgttct 4200
taattctaat tacttcacta gtaagtaagt gaaattgctt taattggagc ttctagaagc 4260
ccttctggga aatgagaggt agctggatgc agtagaatgc agtggaatga gtatttcaaa 4320
gagcactgga ctcagcatca ggagatatgg gttctgggga tgatctctgt ctctcagtga 4380
gtaagcaagg acaagttgtc tctaaggttc tcactttcct catctataaa atgactacat 4440
tagaggagtg ccttgtaact tatgtgctct gtgattccac gctaaatgga agccaagtta 4500
gactgtgtaa ttcacaggaa attaggaggc tgaagaaagc ttggcagcca gtatctccct 4560
ggatgtagtc tgtgttctgc tttcttgttt ctctagtcat tcttttgttt tcagaaagcc 4620
aaaaatgacc tgcactgctt cccaaggttt ggagattcag aggacctgtc cctgttccct 4680
tcctacccca ttgctggggc tgagcaaaaa tgagcccttc acctcaagct tcagtagctt 4740
tcattgagta tcatcaggca ggttcactca tacattccac atttacaaag caccactatg 4800
tgccagggtg caagggacta gaaagatgtt tctacttgat tattcttgga gccagagaat 4860
gtcaagatgc agcgggactc tagaagcctc ggagtctcag cctgacttta gaactgaatt 4920
tttgtccaac caagggtgtg aataaatgca aagacatgac ttgttatttg ttaaggttgt 4980
ggggtgaggg gggtggtcac acagagcatt gtctccttgt tgttagcagc gttcatcttc 5040
ctacttgtgg ggagtaacag actacttttg gcaatacagt gctcaaaagt gtcaagaaaa 5100
accctgcaac tcaggaccca ctggattcta gagaacaagc gtctcttttc tggattgttc 5160
ttgtttattt ggggggtaaa tttggataag aatcaaacaa cttgtagaga gatccacagc 5220
agaatcaaga gcaagcctgc aaacactgtt tctttcattt ggcacttagc aagcacctac 5280
tatgtgccag acactgctcc atgacccacc tcctctctca ccatccactg ggcaaacaac 5340
agccataggt tgaattatga tgtggaagca aatgcaaatc aacttcctcc ttcatataaa 5400
caaacaaaca aacaaacaaa caaaaacttg tgtcagaatg tttttggata ctaaacatca 5460
aaactcagcc aagctcagct ggtaggtttt cccagcgatt tccttggtct gagcatctct 5520
taactctgca tatcggtaag gggaatggta ttttaaagga gtgactctct agaggttgaa 5580
tggcctctgt gaggggtcga tcacagggga ttggggaagg ggtctcttta acaccaattc 5640
catcagctct gctagctgat gggaaagctt gtcctttcac cttcggtatt ttaatttaga 5700
agaatgtgtg tgtgtggagg gggtgttaat ggaaaaaatg tcaccgtttt tagagaagct 5760
agtgaatggc agggctagca gtcaacctca gatagtctga aactttgagg ggtgctctgt 5820
acccctgcct ctctcataca acctagttaa taggattctc tttaacaggg cactagcaga 5880
ggaatgagct ataatacaat acagtcctca gaggactgta gaatcagtag attctcctaa 5940
cacaaggcca gctttccagg gcaattctgg acagcctatg tcaccgcaca ctggtttagg 6000
gctccttgta tatatctcca gtgtcctgtg tttcatccca atctaaaaca tttatcatct 6060
tttatgtgtc tgctccctgc tacatggaga aaggagtcat gtcattaatc tgctatatcc 6120
ccagggtctg gcatatagtg ggtgctcagt atttgatgag tgggtagatt gatggatggg 6180
cagaaatacc ccttgatcca tggtcctctc atgatcttat ttcctatctc ttggaccaaa 6240
atgtaaacat ccttcgataa aagaaccttc cctgcgtttt aggctttggt tttggttgag 6300
agctccatac tcacaagcgc ctacagagga aattaggggc tatgtctgaa cctgtttaga 6360
agggtttcag aacctctgag ggtgtggcta tccccaccac cttccaaagc aataaactta 6420
tccttgtaac ttaaggtctt gtctctgtta aaagataatt ctgagccaga tggcatttgc 6480
cccttatcaa tcatttcctt taatggactc acaggaaaaa atctttgaaa gttaagaccg 6540
aacagggtga gaatcctttt acaaataaca atctggaacc cagagaggtc tagccactcg 6600
cctaaggtca cacagcttgt tggcacagcc ctgaatccct atctcgtggt ttccagcgtt 6660
tagcctggct gtgcctccag tggcccctca gggcatctgc aggtgacttc ccttcatttg 6720
cccaacattt atgcagcacc tactatgaat caggcagcat gctggggcct gtggatagaa 6780
agatgaagag ccatagtctt tgtcctgcaa aggctttctt ctctgagctg tacccatggt 6840
agccccaagt ttctacgcct taaatccgag gtcccccgtg gcctgcagca gctttgcctg 6900
agtagaaggc agcccgcact aaggctccag gcttatttgg gacctggtgg gcgacggggc 6960
ctcagcccct cccgcctacc ctggagcttg ttgccgccgt taccactgaa ccagtcatgc 7020
cagaaagtca tcacgatgat ttcagaactg tattgtgatt tatgcaaagc gcgccgataa 7080

```
ttacagccgg gcgcacagaa cacaggatgc atatgctgcg ggtgtgtgcg cgcagtgttg     7140
tgcctttcgc cagggctgaa tggggtgtgt gtgtattccg taggcatctt cttgacacca     7200
tgcttgatcg gggactggtg acaggaaaaa gagggaagat acccattttg attttgagac     7260
aatttgatga gccttgggag tattttccag aagttttggt gcctacaaat gagccagcct     7320
ggcaggagcg ttcgagagta ggggaagagg tggtttggag cccaggaaag ccatcgtcca     7380
gcccccgcac aggcaaagca attagtagtt ctccttctcg aaacagaccc ctaaatgctt     7440
cccctgcaga caggcctggg tctagtcatc accccccaca ctgccctcat cttcgcccca     7500
gataaattag ttccaccctt gaagttcttt gtcctgaacc aaagaacccc ataatcgcgt     7560
ccgcgttatg gggtggtgg tggtaggtgg ctgaagcaag tggatttctg gcgtgaacca     7620
gaacctgagg attccaaacg gagcctggca tcttattgtg tttctcaaat cctgccgaa     7680
attcctcccc tctctgggcg ccgagttcga gaaagcgcta cgccgccggt cgggctagct     7740
ccacaagcgg ctgtacaagt tggctgtcaa aaaacgctga tttctcctcc tgtcacctaa     7800
taaaccccta cgcgcttatg gcctcgtccc acaatccccc aatctcgtcc caattcgaaa     7860
aaccgaggag gagggaataa actgagagat aaagatcccc ccatcttgct ctttccccgg     7920
gaccccagcc ttggtcgcgg cgccccacta aggaggacac aggctctggt gtgtgtggtg     7980
tgcgagaccc cgagctcgag gccgagccaa ggctgggcag aaagttgcaa tcacgtgctg     8040
tcggagccca ctggagcgca cagcccgctc cccctgggac gcccaggcgg aggacctgct     8100
gcgccctccc agggctcggg ggactccagc attcacttgc acgcacaggc gaactctgat     8160
tgaaagcccg ggatgacacc gagtctggag aaagagggac cggggggtgg gctggcggaa     8220
ttgcagagcg ccggccacag ctcccctccc cgcgaacgtc gagcggaggg cgggaggtgt     8280
aacctctgac ctctggccgg gtccacgccc tgaggaggga ctggcaagct cttgttcgac     8340
aagttcaagc tgccgagaga gcttaaatag aattaatctc ttagagatcg ggatcatcg     8400
ctccctcggc atgcgctctc ccagcgccgc gcacagagca aggcgcgaga gagctcagga     8460
atcgcgggaa ggcaagcgga atggggaggg ggtaggggat gagggcctct cttcactatt     8520
cctccgcccg gagagcggga gcccgcaacg cccgccgagg acgagcggcg ggagggaacg     8580
ctctgccctc cagccgcccc ggtgcagata atggaggcga caagagattc gctcagcgtc     8640
ggatgggcca gctctgcttg ggaagctgg cggcatcctc ccctcggctg gtgcccaaac     8700
ccactgcgcg aaggccgaag gaacgcggaa cctccagaag accccatcct cagccctgac     8760
tttccgtaga tatgtgcaaa atgagtaaat tactcacctc gggccagatc caagttttac     8820
ccaacagaag ggcaccgga ccaagaatga accaactcac atggccatgt ccggcgcgca     8880
caatcacacg ccagcacaca gccacccaat ttcttccgcg aatctatctg gcactctgga     8940
gagaggggga aaagcgtttt gagaaagccc cgtcacccct ccccttcctt cttgccgtga     9000
aatatacgaa ttcattttta ttacgagccg caccgtcctc accatcacgc acgcacagag     9060
ccacactccc atattcacac tttctaactc gtaagctccg acagcgcctg cattttcttt     9120
gggagccgct tggaggttca ttaatatcat tagcatttaa ccccctccct cttcccatcc     9180
cctccccgca catggctgac gtcagacccc gccaggagtt gggggaaaag ctaagtgggc     9240
cagggacgcc ctattcccct ccccgcggct gcctgtcaga gcgcttctgg agatattaca     9300
ggggacccag cccgcagcga caggcacaaa gtcacggggt aatgaacttc ggggaccctt     9360
cgccgctgcg tgcgcggctc tccccggaaa cccggacctg gccgcctctt ccctcggaag     9420
atttcccagc aatctagttt tcccactctg cgcttgggtt ccggcagcgc ggagcccgtc     9480
tgcctctgag actgcggtag tgttttcctt ctttccttgg gagaccagcg gtcggcagag     9540
attgcccaca ctctgcatgc ctatgtagag ggagagatcg aagactgagt gacaggaatg     9600
gggaaaaaga gggatttcgc tccgtaggaa ggccattttc gtgtctccat ctctgtcttt     9660
caacatccct ctcttgctgt tcttccttct tcctcagtct tcctgtccat ctctccatct     9720
gtctgtccat gtgtgtgtcc atatcaagca gcattcccag cagctgcggt tttgcaagag     9780
ccgggaagaa acttaaggat gcttaaattt ccactgttgg acgaattctg agcgcccagg     9840
gagcagcgca gcgcgcgact gacacccacc tgtcccgccc aggagccttg caggctggag     9900
ggcagctgga gagcggcggc gcccggcggc gaggcgggcg ctgccggccg ggactcgggc     9960
agcgcccacc aaccgctccg ccccgggaca gccagcatga gcaagccagc cggatcaaca    10020
agtgggtacc tctcgggccg ccgtggggcc taggcgcgca gcctggggcg agcgagcggg    10080
gaggctgggg gaggtcctgc ctggagcgct gcgaatctga gcccctgaga gggattccag    10140
cgggcgtgtg cgttcggccc agacctgtag accgtgagtt ggagcatttc gtggagaggg    10200
gagagccgtt tcgttgcctc tggattgctt gatcccccct gtctggtgcg gtgagaaggt    10260
tacgacccgc gcagcccacc agtcggatga gttgtctcca tttagccgcc aggtgctgga    10320
tggggggcc atgggggcgg gaactgggcc gcagctccag gcggtagcac aataacacac    10380
tcgctcaaaa ctccgagctc cagcgcgcaa aagcaactct gtgcaaagcg gattttgaat    10440
ggaatgcttt gcaccccgtt tctagctatt tcaaataatc ctgcaaactg ggaagcagaa    10500
acaatttaaa agtcacattt tccttaatcc taaatccgcg taggtcataa ctggggaatt    10560
taaagtatgg cgaaccactc tagcaaagag aggaccaaat ccctaatccc aaggactttt    10620
cgagccggag cccagcagag gcaggagtgc gcggcctgct ccctccgtgc gcttctctcc    10680
ttcctcgaac ttccttagct gccggctctc cgaacgccag gccgcagctg acctctcacc    10740
```

```
accccgagac tcacgagcgc agggctaagt gtgtgtgcga gggcatttgc ttgcaccctg   10800
cctgcggaac ccaagaatgt gcaggcccga gccagcgttg agcaggcgcg gtcacggtgc   10860
tcagatctcc cgggggcatt tcagttcccg ccatccagtg gcccacggct gcgggctcca   10920
gggtctgagg ctggggacta ccgttgccgc cgcagtcccc atatcccgaa gttgccttgc   10980
tgcttgtgtt gttttcgcag atagcatttt tggcgctctg tgcgttcctt ccctccccct   11040
ccccctttca ctcgccctca ttgtcctgag tctttgaaag ttgggagaat cggagatact   11100
tctgaggact ggtaatgaag tctcacttaa gtgggatgca attcccgccc tcctaccccc   11160
ctccaagaag gaggttgtgt tttcattttg ttttgctttg ggtgctgacc tttaaaaaat   11220
tagagcaaaa tgaacgtgaa caaaaagaaa aggagaaatg tttcgagctg gggcagaggg   11280
agcagagaag gagccctcac cgcggccgga atgcagagcg gaccctggcc caggactggg   11340
tttcccttta ggctcgggcc taccctggcc ctcgctgttg gaatctccag gaggtaaagc   11400
gacctcgatt tttgttgccc gcattcccgg gcgtgagtgt ccttcccagg aggctcagga   11460
ggccgtttct gttgcattct gagcctccgt tgcaaaaact gaagcccgtg ggtctcggca   11520
ggcctcctag ctcgctcgcc ccgggacagg ccctcgccta cacccctgga agtaaggagc   11580
cccgggctct ttcgtccttt tcggggtgtg gagcccctgg ggcccttgaa aggtgaggcc   11640
tcagaggcga gggaggggtg agcggggagc tctgcccgcc tgcggctgcg cccccgctgt   11700
ggactaggag gcaggccaac cctccggact ttgggggaaa aaccacagcg ggctccttgc   11760
ggaaactttg gccgttctaa cttgccaaga gcctgagtga ggccttggaa gcctccagcc   11820
ccggctcagg tcgggacgcg gctgctgagc tttctcaggc ccgcaggaca gcggcccccg   11880
ccggtggcgc cgctgcattt aggccctttc cagaccggtg gcggcagcca acccgagact   11940
tgcgtccctc gggcccgggg cagctaggag gtcggcgcgc agcgggccgg gtcaggactg   12000
ggtcgagcag acagagctgc agcccccgcc ttgcccggct tctcgcggct ggagagcaga   12060
gcgatgtcac ccggagcccc gcctgggtgg taacgagacc ctggccagtc acccctgcag   12120
cccagactaa cttctttcaa cagcctctga tggtaattac agtaatcgaa gctgccatat   12180
atctttaggc aattatgaca cacaaaaagc cccgagggga ccccctggcg agggaagtta   12240
agaacggttt tccagcttca ggaaactccg gctcgcctca cgtcggagct cgctcggctt   12300
gctaaatgag aggagctttg caacggggtc aaccagcttg tctcgtgacc ccaagtcacc   12360
ttaacgtggc tgggtggcgg agtctgaggc acaggcccgc tatgccccgg aattttcgcg   12420
tccctccctc ctgggccccg ccccagcccg gttgcctgtt tctaatctgc cccgggagcc   12480
gcggctcaga ggtctgctca gaggcaggac tcgcactggt ggtggcctag agggcaacag   12540
tccggaagct cgggcggggg aatccgagga ggggccccca ccctgcactg gtcttccctc   12600
caccctcatc gggttctcca gagatgttgt catgggcctc ctgtccggat gggaagtggg   12660
gaaaaagaga ccccaccact gctcctccca ctcactcatc ttggcatctt caacagtgcc   12720
acagggaaga ggacagctgg ggtgacagta ctgctgggcc ccaaacgtgg accctggagc   12780
ggtcagaggg cgcgtgttaa gcagaggatg gttgtaagat atggaaacca agtttaccgc   12840
ttccagagtg agtgcagtgc agcggagctc cagacccggt tcctgagcga ccaggaactc   12900
ccttgggcgc agtgacaggc ccgaaggagt ggaggacaag acccgctgtg cccttgcctg   12960
gccctctcca gcccctccct cccacagcac aatctcccct cccgccctgt gggagggggg   13020
cgccgagccg gtggccgccg cgccgcgcgc ctcccctgcc cgcctttgtc gccgtccgaa   13080
ttcccatgct actctttttg acgggtcact ggtggctcta taggcaggtg ctgccgcggg   13140
gttgtaatta cccggccgag cctggtcgtt accgaacacc ccctcacccc caagcacggt   13200
cccccgcgct ctccttcccc caccccctcc acctgagacg agtctgggac ccacggtgca   13260

agctcgcccg gacgcctcgg gttccaggag caagtttaca cctgcggtgc ctcgaggccc   13320
tccccgcgcc acgccctcac ctcccctact cccctcgccc agacgttcca ctgccgcccc   13380
ttcccacctc aatcccatcg tagtccccaa cccaagcgct ttcggcagca gctgtcgaat   13440
ttcagggagg cctgaggccg ctggggccca aactggaggc cttcggatag gtagaagaag   13500
agaaaagcaa gtgttaataa gtcaattgtt atcaact                            13537
```

<210> 51
<211> 2567
<212> DNA
<213> Homo Sapiens

<400> 51

```
tcactgaggc gctacacgca cccaaaaatc atcatcagaa agagaacttg aacttcctga      60
ctcagagtct ggctcactga atccacctaa gtcccattca gtgctagact cacttggtat     120
ttcttcttct tcagtgagga aactctgacc aggttcaagg caggaaggat aaacacgagc     180
agagaggcaa aaaaagttag aatcaaactg caagaggcct aatgtggtac ctatattaat     240
ccattggtct cccctaatat catattcata cacagtcacc cggttctttt tccactgtgg     300
```

ggtgcgagag gtgatgagca acaattttg gccatgcttg ataattctgt agttgttggt 360
ctctgagacc aagggaatat tattcatttg cctccattct gccctaactg ggttgtagac 420
cttcatgact gggatatcac agatacaata gatctcctca ttgaagacgc aggcttcctg 480
aaagtcattg cgcttcagag aaacgcactt cagccacata ttgtggctag gatcataaca 540
gaacatgcgc ttactgttga gagcatagag atagtctcga attaccatta ggtcaaagga 600
taaaaaagaa tggggcagtg gagccaccaa tgcccactgg tttctcttaa cattgtagca 660
ttccacttcc ttcaacttaa ctccagtaat agggtctcgc ccccccaaaa tgtagatata 720
gccattgaga tatgccacat ccatgccctc acgacacagc aagcgatctg caagttgctg 780
ccaactattc tgagctggtt tatacaccca gaggtctgtc ctgggctgag cagctagata 840
gatgtcatgg tcaggagaga tacagacagc taaagtggtg acagtcctag tgtgagccag 900
acaggtcaaa ggtgacggca ctttgtaaag gtcccccgag tatggatcac agcagagaaa 960
gggatctctg gggtgtccaa agaagatcac catctccttg gcacacatac ccagtctctg 1020
gggtggattt tctgctgcag atacaagaga gttgctgctg ctactgctgc tgctgctgtt 1080
tggcactggc accagagact tgtacaacag gtcaccatag cgcatctgca gggccccttc 1140
aataacgtcc aggcagtact tcttcacgat gggcttggtc agcagccctt ctaagtagtc 1200
ctgatcttct tcagtgaagt gcatccagcg cacgcacttg aagacttctg cagcactggg 1260
accccgctct ttgggagcag cctccagcca ctgcactgcc acatggcaca ctgtctgctc 1320
actctccacg tccagactat ccaagcgcag gacagccagc agctgggcca gggtcagatc 1380
tgctagagtc tcctcccgaa ttgaacccat gtggctgagt tgcttgaagt tctgagctat 1440
ataggactgg gcctgggatc gcagcttgcg atggccaaag gcatctgcaa acttgaggat 1500
ggcggtgcag ttggtcaggt caagacgtcg ggctaagaag gaggcacagg cttcccgcac 1560
atattccagc tgtagcatgt cggaggccgc gtacaggcgc tccacgttgg cctcactgag 1620
agacacacga cccgtgtagc agtagtcgac caacacctcg aaggactcgg cgtccacatc 1680
gtgcatggtc acgctggcct gctggctctc gtacatgcca cctgtgaaca tgctcttgaa 1740
gtagggacat gccgcggcca gcacattgcg gttgcagggg aacaggcgac ccgtgccagg 1800
cccgctgcca ggcgtcacca cctcgatggt cacatcacac agcagccgcg catcgtagaa 1860
ggacttgagc tgtgccagca gggctgcaga atgggccgtg tcctttaatt cctctggacc 1920
cgtgaaaaag gccgaaactg tgggtttgtg aatcttcttg ggccgcttcc caccacgggg 1980
actggctagg cggcgagagc gcggggcgtc ttcccgggac tgcatggtgg agatggcgac 2040
gggcgctgat ggcgagaaac gctgcaggac ccggctctgg ctcctcctca accttccctc 2100
gctgacgcta agatagcagc gtctccgaag agacagcagc acgagcccat ttactgaatt 2160
caaccctacc ccgcagaacc gcctcccgtt atcgtttaga cagtggctga ctcaccctct 2220
ctcactcccg cgtcctttct ccgcgtctgc tcgccttcac aggacccgct ggcttggagc 2280
cgcagaagcc gctactgcag cgtgggcgac aatgctaggc gcccaggaga actaccgctc 2340
ccaggttgcc tttcgcgccc cgagggctgc tgggactctc ggactcccgg gccgagaccg 2400
agggaaagaa tccgctttgc atgtcgggag tagtagtttg tgtaatatcg cgatccttct 2460
ctggtgttcc ttgttttagc tgacgacact cgagagcgcc aattttccta gattcgattt 2520
tgtggtttgg aactgtggtt ttggaatcca gagtagtcgc ctcgttc 2567

<210> 52
<211> 10664
<212> DNA
<213> Homo Sapiens

<400> 52

gcgtgggggg cgtggcccga agagaccaaa tggaggtgga ggtatgggct aaactgaact 60
ctccaggcca cgggaggggc acggctgagg gtcccttccg tccccagacg tgatggtgtg 120
gtccaatgag cgggtcatgg gttgggtgtc cgggctgggc ctgaaggaat ttgccacgaa 180
cctcacggag agcggggtac acggggcact gctcgccctg gacgagacct tcgactactc 240
cgacctggcc ttgctcctgc agatccccac gcagaatgca caggtgagct gccgctgggc 300
ccggagcatg ctgggcgtcc ccacctcgca gactgcacgc tccaaccgcc ccctccacct 360
cctctttcca ggcccggcag cttctggaga aggaattcag caaccttatc tccttaggca 420
cagacaggcg gctggacgag gtgggcgcgg caacagctca gagggctctg ctcccagcgg 480
ctcctcgaga ggctgagctg agggggcggg gcctgactat taatggtcac ggttggaggc 540
ggggccagga gaggggcggg gttaaaggag aggtgagacc cggagagggg tggagtcaaa 600
ggaaggggcg gagtcagaca aggcaggagt ccctcaccgg ctgtccggct cctataccta 660
ggacagcgcc aagtctttca gccgctcccc atcctggcgg aagatgttcc gggagaagga 720
cctccgaggc gtaactcccg actcagctga gatgttgccc cccaactttc gttcggctgc 780
agcgggagcc ctgggctctc cggggctccc tctccgcaag ctgcagccag aaggtggggg 840
gctcccaaat gcgacagccc ttcctcgctt ccctagggtg gggcatggac cacctcagta 900

gtccgggttc tggaatggcc tagtcctttc tcgcccaccc ctgaggaatg gactgctcca 960
acgttccgga ctccccgtc aggatgaaat ggataaatcc cactcccct tcccagggcg 1020
ggagtgaact cgcccaatgc gagtttgagt ccttggctgc ggggaaggga gggaaaccca 1080
tgtggagccc ggcgatcgtt gtgacatcgg gaagggaagt ccaaagggag gaatcctgga 1140
gaggaacagg gaggagggtg ctccaggctg aaccgctgct cgctctccct ccaggccaga 1200
cttctgggag ttcccgggca gacggcgttt cggtccggac ctattcctgc tagtgcaggc 1260
ctccaggtga ggaccgtgct gggcgacctt gggggtgcgg ggcggcacgg tggatcttca 1320
ctgctccacg cgctgcccgg cgtcatgcag gtgacctcac tcggacggaa gaatcttccc 1380
gaggctgggc tgttccctct cctgcccgga ctgtggcctc gccgggggaga gcgggcgggg 1440
gagctcgcgc cgaggactgg accatctgta cagaccagcg ggagtgcgcg cgcccgcctc 1500
gcacagggcc ggggcctgga ccaaaccaca tgaactggac tgagaggggg aagaagcggg 1560
gaggaagaaa tcccgcccca aacgtccgct ttccttttct ctactttgta atttattgat 1620
cagtttctgt tgggagacgg gtgtccttta cccgcgggaa gggggcgggg cttccctccc 1680
gggccgcatg cggggagagg ctgctccctc cccttttcc tgcccagtcg cggggcccaa 1740
gtcttccttc ttcgtccgaa aggaggggag gggggactcg ctgctacaag cctcgccccc 1800
tgtgccactc agctccgccc cgccgcgtcc ggtcgccggt cccccgggtc atctgcgggc 1860
gggttcccct ctccctcccc cgtgtctcgt gtccccgggg cctcaccgcc ccccgtgctg 1920
tggccgtgtc cgtgccccgg gggtaggggg cgcagaatgg cgcttcccct tctcctctgg 1980
ctccggggtt tgcatgggag aatcctcttt ccacgatgcc gctgggcgac gtggcgtggg 2040
ggcaggggga cggtggggga gccctcgccc ccgactctcg gtcggcctcc ccgccccagg 2100
cgtcactcag tgatcacggg taaagagaac tgtttcaaaa agcttccttg ttgactgatt 2160
ttttttttgc gggggggggg ggggctgttc aaccaaccac ctcgagccag gagtccaggc 2220
ccctcatccc tcaagtctag gtccaggtct ctagacccta cccgcattcc ccgatgccct 2280
gagattccca gaccccacgt ctgacaatga ggtttcgggg agcacgttta ttcggagaaa 2340
taaatatagc tcgtggaaag gggttggaag gcagggggag gtacacctgc gtcgcagtcg 2400
agcgactggg tcagggttcc ggcgtttcca gcatgtctgc cagggccctg gagacgagaa 2460
cgccagaagt tagacagctg gtgttcaaag cccaccccag gacgcttaga gatcattaac 2520
ctgcccattt catggacggg gaaatggagt cccagagtca tctgataact catagcgaga 2580
caagcacttc tctaaacggg gcttttacac gctccccttt tacacactta ctgataaagg 2640
gacgaggaga aatagtcaac gtagcttcct gtgggacagc agagaaaaga ggcttgaggg 2700
gtactgcacg agcaaaatta ggcagccact cagtatagcg tgcgagatta gtgctagctc 2760
cacctccttt tccctaattc cttcttccaa ctgaatccct tctaccggac cccttttcg 2820
ttctgggtga ttccgttctc cagtagcccc gccccttcga ttcctacttg agttggcctc 2880
gcccattagg ctctaagccc gcatctcaac cctccactca tttctgtccc ttggctccag 2940
caactccacc tggtgttcct cgagccaggc cccggcccac gtgctcagag ccttggccac 3000
gcctcccctc ttagccccgc cccaccagcc taccgggcca ggctccgccc ccaccaggat 3060
tcaccacgct gggcccggca ccttcctcag gtttttccag ggccccgccc atgctcacct 3120
ggagcgcaga ccgtttcgca gaccagcggg cagagatagc agaactgaca gtgctggagg 3180
cggggacggg gagagagagt gagcgtgggg cgtggccggg ggcggagtta ggatggggcc 3240
gggaccagac ccggaccaga ggctaggtct aggttaaggt tggagtcagg atcgcttgtg 3300
gagtcaaggt tgcatcaggg gtaaggggtt gggtcagagg ccaggattta gggctggggg 3360
tcctcagagg gtcccggtgc atcctgaggc ccagtggagc cttgagtgtg aggtgaggtc 3420
tcacctggca ctggtcgcag tgctcaccca tgttctcctc atcacagtga cagctctcac 3480
attcagtgca tcgctgggga ccgggggagc ctggttagat ggaaactcta acgccacttg 3540
ggaggtaccc gcccctggcc cacccctgca ccctggacgc ttccccagac tctaccctct 3600
ttatctctaa cactgagctc tgtatctgcc ccccaaacca gcctcccctc ccctcccctg 3660
cccctggttt ccttcctttc ccttttttc cttttttttc ttgaggcgga gtctctctct 3720
gtcgcccagg ctggagtgca gtggcgcaac ctcagctcac tgcaacctcc gtctcccggg 3780
ttcaagcgat tctcctgcct cggcctcctg agtagctggg attacaggtg cgtgccacca 3840
cgcccggcta atttttttgt attttttagt agaaatgggg tttcaccgtg ttagccagga 3900
tggtctcgat ctcctgacct cgtgttccgc ccaccctggc ctcttaaagt gctgagatta 3960
caggcgtgag ccaccacacc cggccctgcc cctggttttc tatctcaggg cgaccccatg 4020
tctccctgtc agggtgggct caggtgctct ctccacccgc tctctgccta ttctccccct 4080
agcctatcag cccctgtgcc atccctcctt cccccgactc tctttgccac agacccctgt 4140
ctctgtcccc acaccccaag atgcagcttg ggtcttgtcc tctccttcct ggaccactgc 4200
cccagcctcc cagccttcag tctgtcctca cctcacatgc ctgcagaagg atctcttttc 4260
tttcagaact gaccctggcc tgcccttgct ctgagccctc ccacagcacc accccagggc 4320
ccctgggaca gattctgggg acaccttggt gggtggatct gggctgggga catacattgc 4380
agaaggagca gtagccacac agggaccctg gctggtagtt cccatactcc tgagcatcct 4440
gtggacctgc ggggacagga gggcagcagt gacccaggct gactcggttc cttcccaccc 4500

acaccagccc aggccactta cctgtgtcct caccgctttc ctcctcgctg gaggcacctc 4560
cagcctcctc tctcctgtcc agtgggttgg ggatgatggt gaagcggggc tcatcttcct 4620
ccagcccctc ctcctcctcc tcttcctcct cgctgtggtc tgggctcagt ggggccccaa 4680
cctcagccct ctcttccctc ctctcctcgt cttcttcctc ctcctcctcc tccttgtctt 4740
cctcttcttc ctcctcctgg ttcaggctga ggccatgacc ttcctcctca tcctcctcat 4800
cttcctggtc ccggctgcca tgatgggtgc ctttctctcc ctgctctgaa ctttcatcgt 4860
cttcctcatg ggagccgggg tcctcttcct tctcctttc ctcctcagaa tcatccttcc 4920
tcaaatggct tctatccttg accactgtgt gtcctggggg gtgatggctc atctctttga 4980
tggaccctct ttgaccatgg ccagtttctt catcttggtg gctttgcctg tggctggggg 5040
cctggtggcc aagctcagca gagacctcct catcttcctc cctggggact ctgtggtggt 5100
gatggtgagg gacttctgtt ttatactcat cttggaagtc ctcttcatca ctcttgtggc 5160
ctcgaggttg gtggcttgca acatagtggc cgaactggac tgtgatctcc tcttcttcct 5220
cttcctcatc tacaaggcca tggtggacat gttggggacc ctggtgccaa cgttcagtgg 5280
acacatcctc atcttcttcc tcgtctctgt ggccttggtg cctgtggtca gggacatgat 5340
ggtggtgttc acctgagaca gcctcaacct cttcctttct gtggtcttgg tgcctgtggg 5400
cctggtgtcc atactcagtg gagacatcat catcatcatt gtcatcttct tcatggcttc 5460
ggtgcctgtg gctggggtcg cgatgatggt gtccatctga gacatcttca tcctcttcaa 5520
tcccgtggcc ttggtgcctg tgagcctggt gtctatattc aatggagaca tcatcatcat 5580
catcatcatc atcatcatca tcatcatcat cgtcatcttc ttcatggcct tggtgcctgt 5640
ggctggggcc atgatgatgg tgtccatctg agacatcctc atcctcttca ctcccatggc 5700
ctcggtgcct gtgggcctgg tgtccatact cagtggaggc ctcctcttcc tcctcctcct 5760
cctcctcctc ctcttctcct tcatcatctt ccccatcatg gcctcggtgt ccatgcctga 5820
ggatatgatg gtgatgctca ctggacacaa cttcatcctc atcctcgtct tgatggctgt 5880
ggctcctgtg gctggggagg tggtgcctgt gctcagctga gtcttccgtg tcttcactcc 5940
cgtggcctct gtgcccacgg gcctgcccac catgctctgc aaagacctcc tcacctgaga 6000
caccctcatc tccgactttg tggtcttggg acctgtggcc tgggagtagg tgcccatatt 6060
ccttggagac atcttcatcc tccttttcac ggtctgggtg gctccagaaa tgatgcccat 6120
tctctgtgga aacatccttg ttctcatctg gatggtctct agggctgtgg aggtggtggc 6180
gaagctctgc tgatgcctcc tcggagagcc cggcgactcc agtgctgttg ttccggtttc 6240
tgaagcctag cccatcccct ctgagctgct gggtcatggc cgggggggagg agcaggctgg 6300
ccaccccagc ccagaggaca gaagcgtgca gccatggcct atggtggccc atggggacgg 6360
acaggcagca ctggctccag ctgcctctgc ggcaatgtgg acaaacgttg ggtctttgt 6420
ccctttgggg ttggtctctt tttttctctg tgtctctcct ttgtcctttc ggtctctgtc 6480
caccttcctc tggtccttgc tgcctggctc tggacacccc tctgaggctg tctccggagc 6540
cccctgaccc ccctctgggg ccctccctcc ccattcccca gccaataggg tccttcccct 6600
cccctctctc cagctaaatt tactctcagc cctgagttat tctgggtcag tccccgcctg 6660
cctgcctcct gctcctcctc ctcccagctg gggaggggac cagtgagggg tctctccctg 6720
gccaggagac ggtggccaag ggacttgact ttgaactacc aacaagctca cgtttggcag 6780
ctgcaaagac aaaggctaga cttttagcag gtttttgggg gagcctgggg cacctggggg 6840
aggcagaaga gacttatcag aggggagaga ctcctgggac ggaaggactg gggttcgat 6900
tgcggggtgt ttccagctgg aatgatacgt gctggtgaga gagtgatgtc agtattgagg 6960
ccctagaatg gggggaaagg aacatggccc ccaacacacg tgcccatgac ctcctgtccc 7020
tggaactcag atctgggggc agggactggg ctaggccagg gctataaata cagctgggag 7080
gggtaggggg actcaggtta cggaggccac agctgtcccc atcacagagg gctggcagga 7140
gacaagtggc cttgcccgtc tctgtgtgtc agtatttcct actcctcacc cttcatgact 7200
gcccccacta gggtctcctt tcctgttcac gggtcctcct ctctcttcaa ttctgtcatc 7260
tgctctctca gggtccctgt ccctcctcca tgggattgcc tctccctctc actctgggct 7320
tctgtcccac tcttatctta gtgtcagtcc tcccccaagt ctgtgtccct ctctctcccc 7380
taaatctctg gcccctcctt tctgagttcc tgcccttccc ccaattcttt ggtttttgca 7440
tccccctctg ccccttgcct cagtcaaggt gtctcctccc catctctggc atccacctct 7500
ctgggtctct gtccccactc tctctcagag tctctgtccc cctctgtctc agagtctctg 7560
tccacctctc cctgggtctc tgtccccctc tctctgggtc tctgtccccc tctccctggg 7620
tctctgtccc cctctctctg tggatctctg tccccctctc tctgggtctc tgttcccctc 7680
tctctgtggg tctctgtccc cctctctctg tggatctctg tccccctctc tctgggtctc 7740
tgttcccctc tctctgtggg tctctgtccc cctctctctc tgggtctctg ttcccctctc 7800
tctgggtctc tgtccccctc tctcagggtc tctgtccccc tctgtctcag agtctctgtc 7860
cccctctctc tgggtctctg tcccctctcc ctgggtctct gtacccctct ccgtgggtct 7920
ctgtcccctc tccctgggtc tctgtccccc catccctggg tctctgtccc cccctctctg 7980
ggtctctgtc cccctctctc tgggtctctg tcctcctctc tctctgggtc tctgttcccc 8040
tctctctggg tctctgtccc cctctctctg ggtctctgtc cccgtctctc tgggtctctg 8100
tccccctctc tctgggtctc tgtcctcctc tctctctggg tctctgttcc cctctctctg 8160

```
ggtctctgtc cccctctctc tgggtctctg tccccgtctc tctgggtctc tgtccccctc    8220
tctctgggtc tctgtccccc tctctctggg tctctgtccc ctctccctgg gtctctgtcc    8280
ccctctccgt gggtctctgt cccctctccc tgggtctctg tcccccccctc cctgggtctc    8340
tgtccccccc tctctgggtc tctgtccccc cctctctggg tctctgtcct cctctccctg    8400
ggtctctgtc cccctctctc tgtgggtctc tgtcccactc tctctgggtc tctgtcccac    8460
tctctctggg tctctgtccc ctctccctgg gtctctgtcc ccctctctct gtgggtctct    8520
gtccccctct ctctctgtct atccctgggt ccctgctgcc ccaccttctg attctctgtc    8580
ccctaagtct ttgtctcccc ctctttgggt taaattgtcc cctccctgtc tggcatcctc    8640
ctttctgagt ctgttccctc tccgccactg gcccccaact ccttctgttc ccatctcgcg    8700
cttgcccttg gagtctcccc tgtgtgtctc tctcccccccg gcccggacct ctgcaccccc    8760
caggtcgctg tccctctgtc cccttatcgc ggcctgggac ccgccctctc cccgcctccc    8820
gctttggcgt ctccaagact ccccgccccc cagacctcgc cccgccccag gctaggctgg    8880
aaagtggagg atccggtttg ctctgggcgg gtctggaagc agagccggcg gagggagcgc    8940
cggggccctg ggctgcagga ggttgcggcg gccgcggcag catggtggtg ccggagaagg    9000
agcaggtgag cgccggacca gggtctgcgg gagcgcggag ctggggacct cgcctccagg    9060
ctcccagaga ggagggcgct ggacacccag attcctgggt cagacggagg agggggatgg    9120
gagggtccag gttccagggt ccagggcatg gggtcgaga ctcctgagtc tggagcggga    9180
gggcgctggg ggcccggact cctgtgtccg tggggagcgc acggggtggg tggctctgtg    9240
tcccatagga cagaactggg tgccctctca ccccacttcc acccctacat ttgttcctgt    9300
ccccagagct ggatccccaa gatcttcaag aagaagacct gcacgacgtt catagttgac    9360
tccacagatc cggggtgagg agttcgcccc tggactgacc ccagagggtc cgcggcccgc    9420
tgaccccgcc cgcggatggt cacgccccag ccctgctcct tccccattcc tggactcggg    9480
gaccttccca agggcgttcc ttgccgacgc tctccctcta ggagcgtttg ggtctgtgta    9540
gacacccctc attccccatt cgccattctc ccgctcaggg tcagtgtaga cgctccaggg    9600
ctcggttctg cttctttcct ccccgatgtc tcttctatgt gcttctgtct cttcgtgtca    9660
ctttctgtgt ctctgtcttt cccttttggg tatttctgtt tcattcattc attcattcat    9720
tcattcattc attcatccat ccatgcaaga aaccctcact cagcgctccc aaggtccctt    9780
tagcctaatt caacggccct gccagggttc caatttcccc tgggacacgt gctgtgtggc    9840
ttcgggcaaa tcatttcacc tctctgggtc ccgtttctca tctgggcatc aaaagaggct    9900
tgatctcctg ggttattat gagaattaaa caagttaatg cttgtgatat tcctggcaca    9960
gcgcccagca caggacgctg ggaaatatct gcagctccca ctgggaggtg gcaattatta   10020
ccgcgggcgg gccctgtctt ggtggccggg gacaccaatt gagctagata gggtgtgtgc   10080
gtccgcgagg agctcccgcc cttcagtgac ggcctctcaa gcggatccag gctttctagt   10140
tttttttttt tttttttttt ttttgagacg gtgtctcgct ctgtccccca ggctggagtg   10200
cagtggcgcg atctcggctc actgcaagct ccgcctcccg ggttcacgcc gttttcctgc   10260
ctcagcctcc cgagtagctg ggactacagg cgcccgccac cacgcccggc taattttttg   10320
tattttagta gagacggggt ttcaccgtgt tggccaggat gatctcgatc tcctgacctt   10380
gtgatccgcc cgcctcagcc cctcaaagta ctgggattac aggcgtgagc catcgcgctg   10440
ggccaatttt ttgtattttt tttttagtag agacggggtt gcaccgtgtt agccagatgg   10500
tctcgatctc ctgacctcat gatccgccgg cctcggcctc ccaaagcgct aggattacag   10560
gtgtgagcca ccgcacctgg cccaggcttt ctagtttttt gttttttgtt tttttgagac   10620
agagtctggc tctgtcgccc aggctggagt gcagtgttgc gata                    10664
```

<210> 53
<211> 22069
<212> DNA
<213> Homo Sapiens

<400> 53

```
caccggtgct gcgagagaaa ccctgagcgc tggccggtga gccaggccca gatggggcca      60
gatggggcgg ccgcaccagg ccaggtcctc cccaatcctg aacccgacgc tgggcagaga     120
acagcctcac catctcgcag tccagggcgt agatccccgg gtgggtgtct cctgagagct     180
ctttctcaaa ggtcttcacg aagccctcaa ggcgctcctt ccggccatcc tgcacgtgtt     240
gctgggggtg gagaaggcag gtgagggcag cttcggggtg cagtgggggc gggggtggca     300
gcagcagcac atctctgagc ccttcgggac catcctatcc atccatcacg cccattcact     360
ggttgggacc ctggtgagca ggtggggaag ggggcttgcc aagaagacac agggaggacg     420
gcttcccagg atgggaagtg gccagggaca gggcctgggg cctacgggtg ctacgttctc     480
tgggtaacac aagaaaggct gcccgggggc ccagaccctg agcccaggac tgtgctgtgt     540
cccgagaccc cacaccaacg atggggaggc agaagtctgt tgagaacagg cagaggggac     600
tgggacgcca gggctcacct ttgcgacttg gcagccgaca gagccggcgg cagccgagca     660
```

gcacatgtac tgggtctccc agcctccggc cactgcaggg gacacagaca cacagtcagg 720
gcccggccag gcccactcca cagcccccgg ggcactgacc acctgcatct accgagccct 780
actagggagt gaggactgag gacaggaggc ctcagcctct tggtggagct ggaagcagac 840
caggccccgg cagtcgtgtg gcagccagga cgcccaggcc tggatccagg gagaccagtg 900
ccctctgcag aggcctcttg gcagcgctgc cccgggcagc accctccagg tatccctgag 960
gcgatggctt taggggccgc cacagggctg tgcaaggctg gccatgtgcc ctgagcgtgg 1020
ctgcgtggtt gcctggggga ttgaatagaa ccaccaggcc aagggtggga caagcccctc 1080
cgctcctggg gccagcaacc tccacatttc catggaccaa gggcacagga aggcagggac 1140
acagggcctt tcaggtggtg gcatttgaac cttgtcatgg gaaagctctt gtcttacttt 1200
tgcataaaga acaggtggag agaaaatgag gccaagggct agccccggct ctgccaaaga 1260
cggtgcagcc cagggaccct gagtgggatg gagggcctgg gtaaagcctt accccagttc 1320
tgggggcctc aagggagggc ccaggttccg ggggccatgg gtgggaaggg gaaggacccg 1380
ggtaaggcct taccccggtt ccggcgcagc cgtccccagt ggtaataaca ctcctcgtcc 1440
cggatgcagc ggcctgaaga ggacacgagg tactcggtgc cacagcggca gcaggtcctg 1500
caggaagctg tgggtgggga cccaggtgga agctgaggct cacgctgggg cccgcatgcc 1560
cggccttgcc cacatcccgc acctgcccac ctaggccgtc gcaggccagc gactcacagt 1620
ccttgggcct cttctcctca gctgtgaaga tgattgcgcc cccgggccgc tctgggtgcg 1680
ggaaggggta gccgttctcc ttgagctggt cctgggtgag caggtactcc ctgaggcggc 1740
tgtacagggc agcccctgtg gacaggcaca gtggtcagcc cctgcctggg atggcccacg 1800
gggcggtaga caccaccggg aaaggcggct gggccggcag gggcccacga agcaccgtgt 1860
ggcacagcag gggctgggcc ggcagaggcc cacgaagcac cgtgtggcag agcaggggca 1920
gggccttacc tttcaggtcc tccacccggg ggctgcttgg acggctgagc gagaagctgg 1980
tcttggcggc caacctgccc cccaacacca cctcgtggga cacaaccctg cggccactgg 2040
tttctggaag gaagggaggg aggggaggag ggtgtgaagt agctggctca gacccctgcc 2100
ccgcctgctc tcccacccac cctgccctcc tcagacctct gcctcaactc cccctttctg 2160
ggacagcacc ccactgaccc cgggttccag cctgacccga acaggagagg cagacatgcg 2220
gtgagccagg gacgcagggc agccgccagc tctgcaggcc ccagagctgc acggaggccc 2280
cacacccaaa gtcttcgtcc cctcccctca agcgaagggg gaagggcggg gagaacagca 2340
cgggggatct aatggctttc gagggctttg agccttcatg ggccacccag cctgcgacgc 2400
caacccaggg cccctccgag tgctaatgca ggcggggacg ggggcagggg ccggtgatag 2460
cttaatgcag cctggagagg ctgcaatgaa acgcccaggt ctccgctttc ttcctcccca 2520
gagcttaatg catggagctc gcccagccct gggcctcctg ctttgaagaa gctgccgcag 2580
ctccattagc aggcggcccc tcggctcggc ttggagaccc cactcagcgg aggaaggcgg 2640
ctgacagcga ggacaccagg gccaggtccg tcctacacag aagcctggga accaggcagc 2700
aggcagcccc cccacagcac cgcgctttcc ttttgtctga gactgtggct gagctcaggg 2760
ctgtgaaggt cccagcgagg gtcccagccc agctgccacc ccaagagcaa ccctgagcct 2820
cccccgccca cccgccagga ctcactgctg aggccgggca cagcgctggg ggccaggccc 2880
ctgagcttct tgagggtgtt cacggccaca ttcaggtaga tgttcttgct ggggctgcgg 2940
tcataggcca ccttctcctc gttcagtgcc tggggggacag gcggtgccca gctgaggcca 3000
tgcctgcctg gtgccgggga gccccagcgg tggggtcgcc atggggtcgg ggacttgcat 3060
ctctcccagg cagctccggg tcacagctgc ggctgagagg ccgggggatg tctggggacc 3120
accctggacc cctagtcccc acccgaccgg ccagatagga actccaggac ctcaccttct 3180
ctatggcctc ctggttggag gtacagaact tgagacactc ctcgatgaac aggttgagat 3240
agcgctggcg gatgacggtg gggactttgc ccccaaactc tttggggata atgggtttct 3300
ttaaactcta gagggaaggc aaaagctgcc atgggtgagg gcctccacaa ggcctccagc 3360
ggggaacgca gacgcgatga ggccgtgccc atggctggag gcaagagtga ggtgcgctgg 3420
gacagatcag cctggctgca gtagggacag acacgccaag gcaagacagg agcagcaggg 3480
gaaactgagg gcagcggaac taaggtcatg ggaaactgag gccatggggg cacaagggaa 3540
cttgctgtac tttctgctca attttcctag aaacctaaag ttgctcaaaa aacataaagt 3600
ctattaatta aaaacaaaac aaggccaagc gtggtggctc acgcctataa tcccagcact 3660
ttgggaggct gaggcaggag gatcacttga ggacaagagt tcaagatcag cctggacaac 3720
gtggcaaaac cctgtctcta caaaataaaa aacaaaatta ttagctgggg tgtgatgaca 3780
ccggcttgtg gtcccagcta ctcaggaggc tgaggtggaa ggatcacttg agcccaggag 3840
gtcgaggttg cagtgagcca tgattgcgcc cctgcactct agcctgggca acagagaccc 3900
tgtctctaaa caaaaaaaaa aaacaccaac caaccaaaaa aacaaaacaa gacaaaacaa 3960
aaaaacaccg ccagggcggt cgggcatggt ggctcacgcc tgtaatccca gcactttggg 4020
aggccgaggc gggtggatca cgaggtcaga agatcgagac catcctggct aacatggtga 4080
aaccccgttt ctactaaaaa tacaaaaaca gccaggcgtg gtggcgggcg cctgtagtcc 4140
cagctactca ggaggctgag gccagagaat ggcgtgaacc caggaagcag agcttgcagt 4200
gagccaagat cgtgccactg cactccagcc tgggcgacag agcgagactc catctcaaaa 4260
aaaaaaaaaa aacaccaagg catggggagg gaaggtgaag gctgtactgc ggtgtggagc 4320

acgaaggccc gcagggcagg ggcgaggctg gccctccgca ggtcccatgt ggccgggcct 4380
cagggcgtgg tcttgggggt ggtggtcctg gccgagatgg gaggggctac ctgtaaggat 4440
ggactgtggg cgattcgctt agggatgatg gtggtggtag tcttggacgc catccccgac 4500

aatgtgcgtg ttttcagctg ctggccacct ggctcagggc cgttggagga ctggctgccg 4560
ctggccgcaa gggtcctctt ggcacctgtg gggctggcg gggcacaggg ggtgtgggca 4620
cagggcgagt ggctgcccat caccacgtgg cggagcacca ggcagccgcg gccgctcagc 4680
gccagcagca cgtgcatctc cgcgtgcagg gggccgggcc agggtgaggg gctggggctg 4740
cgcaatggca tcaggctcta gctgccttca ggcaagtcca tcgaggcaga ggacacaggt 4800
caggctgcgg aggccgctga ggacacgtca ccggtggggt ggtccaggcc tctgccacgt 4860
gttctgaccc caagcccaag gcgtcgatgt ctgagggaaa ggccctggct cccggacaga 4920
ggaccgaagg gtctcgggtg cccccacctg ctcattggct ttgccctgcc ctgcggcctc 4980
tgtgttctgc cttgcccttt gatgaagtca caaaccagag ggaggaggcc aggcctgcag 5040
gggctgcttc ggagggctgg ccacgcgggc agctgcaacc tgggcatgta cgtctgtgtg 5100
gcagggggc ttctggactg gggctcggc accgacccag gaaggggagc tgtgagcagg 5160
gacatctggc cctagtctca gagcaacatc cctcgaaatg ccatctggcc ctggaaggtg 5220
caagggaggc aggatgagtc tgctcatgct accgcgggcc gcccagcaag gaagcaggct 5280
gcccgccagg ctggcacgcg cctcttgcag tggagggttt gctcttcagg aacggacaga 5340
gaacctccag actccctcgg ctgcacgctg ggggcgagcc caggcagcca caggagtcct 5400
ccaagccaga tgagcccgcc ctgcggcact gccagcactt gggacgccag actcccttca 5460
ggcggcgggc cccaagggca ctgcgacagc tcagcaccca ccacagatca gcaacaggac 5520
aacccgagcg cggagacaca gacgggaagc gtgtggggtc ctgggatagg cccaactcaa 5580
tgatttcccc tccctggggc taaggtctca gccgtgaggg ggctctgggg aggggaggtc 5640
agagtagcct ggagagctct ccctaaggag ggccgtggga tccatgggat ctgcagggga 5700
atcgccgggg ctggccctaa ggctctccag ccagcgccag ggaggcaggg gctccaaacc 5760
agcaggctgc tcagggtggt cctcggacag cagccatgcc ctcccaggga gcttgccaga 5820
cacacagacc tttcccagcc tccagaccag aacctgcatt ttttaggagc tttctggggg 5880
accctcatct gtgacctgcc tccagggata ctttctcgct ctacagacac cactgatgtg 5940
aagacgcagg agacaggaca accccccgtg aagggtcctg tccacccacc actgaggcct 6000
ggcccgactt tctacaagac cctgctgggg gggaagtgcc cctcggagta aaggaaatac 6060
agccccactc ctgggaagac agcactcatt tccatcagag gccacgcccc ccactcacac 6120
gccaggagaa agccacacct gcagaagcct gctccccacc caatgccagg taccccgaca 6180
tggctcccca ttccctggaa ccacaggctc aggccctggt tcagcccagt ccccagcagg 6240
ctgttggact cctagtaacc ccggagctgg gtcagggaag gaaggtgctg ggccaggcct 6300
cctgcacccc tcaacagtcc ccatttcaca gacaggcagg ctgagggccc cagggagggg 6360
gcccaaagtc atcccatgag caagcaccat gtggttcagg tgagctcaga gacctcaggg 6420
tgccacctct cctgcccaca tgcccacggc cccccggcac aggccccctg ggccaggact 6480
cacctgcagc caggcggggg ttggggatgt gggcgatcct cctcttctcg ccaggggcgg 6540
aaatgtggac ggagggcgac ttctctgcca gcctggcggg ggcctgcagc aagctcgccg 6600
atgccctctg cgcctgctgg gcccgcaggt agcacacctc ctgcgccgtc gggggccggg 6660
ccgggggcac cgcgggaccc ctcctcgggg gctccacctg cgtcaccaca aatgctaagg 6720
cctggcagca cagcgggggc acctggagca ggcgaccccg ggcaggcttg ggagagggtg 6780
gctggagctc gggggccgga ggcaccacct ccctggctgc cttctgagct gctgggcaca 6840
ctggggggaa gcagcagggg gcagtggtcc tcaatgccat gtggctccag gctcccccac 6900
tcccagtgtc tcccggtgcc caggctatgt cctcagccct gataaggcac gggtgggcgg 6960
gactaccccg attacactgg cactccaatc caatcccact acgcctaatc ttcctcaaag 7020
cccctcctct ggggaggctc tgggactgga gcaactggga ctctcctggc tgctgacccc 7080
ggagccaggc tctctgcttg tcctgcacta cctcgccacg tctgcacagg ggcctgacaa 7140
gcgctactgt ctccgggcta cagaggacac tggagctcag agctggacaa ccggcccagg 7200
cccaggccgc acacggcgca gcaggccgtc tgccgcactc tgggggaggt caccctgggg 7260
ctgctgacct gctctgtccc tcgccccagc accgtggcaa tctaacagga aggggcaggg 7320
ccagctccct ctggaactcg ggcagcgtca aagataaggt gtcttcaaaa agctcatgga 7380
aaacgtgcgt tgtgacgaaa cttgcatggc tttcaagttt ttttgcccca aaataaactg 7440
atactaactt gtcataacat gtctgaacca gacggagttt gaggcactaa aaaaaagtaa 7500
gacatcagtt agaacagagc cccagtcaga gcaacaggag ttctgctaaa attgaagcaa 7560
atgttaagca tcaaatttat ggtaaaactt gggtggaaga acgaatgggg aaatcactga 7620
tgctttacaa aaagcgtcac agatttacag gaacaatgtc ccaaagaaat cagaagttta 7680
tgaatggata actcatttta tgtatttatc tttttgagac agagtcttgc cttgtcgccc 7740
aggctggagc gcaatggcac gatcttggct cattgaatcc tccacctccc gggttcaagc 7800
aattctcctg cctcagcctc ccgagtagct gggattacag gtgcgtgcca ccgcgcccag 7860
ctaatatttt gtatctttag tagagacggg gtttcaccat gttggccagg ctggtctcga 7920

actcctgacc tcgtgatctg cccacctctg ggcgcagtgc ctcatgcttg taatcccagc 7980
attttgggag gccgaggtag gcagatcacg agaccagtct ggccaacatg gtgaaacccc 8040
gtctctacta aagatacaaa aattagcctg gtgtggtggt gggcacctgt aatcccaggt 8100
actcgggagg ctgaagcagg aaaatcactt aagcccggga ggcggaagtt gcagtgagca 8160
gagatcgtgc cactgcactc cagcctgggt aacagagcga gactccgtct caaaaaaaaa 8220
aaaaaaaaaa aaaacaacca aaaaacaaac aaacagaaaa cagagggatg aagctgggag 8280
cagtggctca cgcctgtaat cccaacgctg ggaagccgag gcaggaggat cacttgaggt 8340
caggagttgg aagaccagcc tgggtgacaa agcaagaccc cctttttta taattttttt 8400
tttttgagac agagtcttgc tctatcaccc aggctggagt gcagtggtgc aatcttggct 8460
cactgtaacc tctgcctcct gagttcaagc gattctcctg cctcagcctc tctagtagct 8520
ggtattacag gcacccatca ccatgcccag ctaatttttg tatttttagt acagatgggg 8580
ttttgccatg ctggccaggc tggtctcgaa ctcctggcct caggtgatcc acccgccttg 8640
gcctcccaaa gtgctgggat tacaggcgtg agccaccaaa ccaggccaac ctcgtcttta 8700
aaaaaaaaaa aaaaaaaggc tcctgctggc ctggcctctg cggaccttac ctcctggcct 8760
tgcttggaaa ggtgggagat cctcctcttc tgcccgggga acagagtggt cagacccgaa 8820
agccccttct cctcactctt ctcttccttg gggggctaag acacatgtcc gtccgtcagc 8880
acaggtctgc cctcgctgcc aacaccaaca caccccaacc tcaaactgcc cccggcaagt 8940
ggggaatgga acagtccagc ccccaggcac agcagctgag ggctcagggc caacagtccc 9000
tgggctttgt gtgggtgcag tcggaggggt gggcaggtgt cacacgttct gcagatgccc 9060
aaggctacct ctcctgggca cccagggccc cagcttggag accccagggc tgcagaatga 9120
aagcacactg ggaaagcgag gccaccttgg gccgaaccag ctgctgcggg ctgagcactt 9180
gggctcagtc tgtgtggagt gagctccccc tggtggccaa tttggggaca cggggccagg 9240
agacccaggg gtccagggct ggcccaggag agcaagagct cgccacgctg ggagtaggga 9300
ggagggaggg gaggagaaag gagccggagg ggatgaggag gaggcaagga gaggagacag 9360
aggagctgga agagaagaga cagagatggg ggaagggagg aggggagaag agagggggaa 9420
gggaggaaag gggaggcgag gggagagggg ctagaagggt gtgccggagg tggattcccc 9480
tgaggtgggt gggtgggcca atatccccct gccagtcagt gggaacagac cgtgtgcacg 9540
tggccacaga agcctggctg gtggccacgg gccagtgacc tctgggtggg tgaggggcaa 9600
caggagcaac agggctgccc gggtgctcct gagctcctga gatttcaacg ggctcaggga 9660
ccagcactga ggagctgcct ccaccccgtg cctccgagcc aactggaaac cactccagat 9720
agaaggtctc tcaccaggcc ctcggctctg cctctgcccg agcccagccc cagcacccgc 9780
gcgcctcacc tgccgggcca gccggcctct gtcctccgtc ttgacgctgg tggactcgtt 9840
gaagatccgc aggcactcct ccatggggtc ggagtcaaag tccacctcct tctccagggc 9900
cgagtagtcc acatccgccc ccgcgctgga ggtggaggag gaggaggagg aggaggagga 9960
tggggcgggg gagggggcg gggaggcctt gagccgcttg ggcggcccct gcgcctccgg 10020
gaagcccagg ctggagtctg agtcggagtc tgagtccgag ctgaggctgg ggagggcaga 10080
gggccacacg ctcggcaccc ctggccctgc ggcctcgtcc tcactctcgt ccccaaagag 10140
gtcggcgtgg ctcagggccc gcttcttcag cttgggggcg tcctgggagg cgccctcgtc 10200
tagtgagcgg gctttccgct ccactagctt ccccgacggg gccttggtgc tcttcctgtc 10260
gggcagctgg agggggcggg gtgggcctct gccggccgcc ggtcgggagt ccccgcttgt 10320
ggggctcggc cgccgcgctg gccggtcagg cctccctttc cctgaggtgg ccacaggagt 10380
ggccgaagat ggcttcttct tggtcccttc cggccgctct gccttgcgcc gggggctgct 10440
ggcctgcggg cccttcttgt ccgcacgggg cttctccaca ggccgccctc ggcccttgtc 10500
cttggtcttg tccttcccct gggccccgtc tttgcagctg ggggcaggtg gggccccggt 10560
ttttttcttc ttgggttttc cctccttggg gcagccccca tcctgtgagg actggacctg 10620
ggctggggag gcgggcttgg ctgggggcgg ctggaggtcc cccacgtcgc actgcacggc 10680
cgtctccttg gtctcccgca ggccgccccc ctcggcctcc aggccctctt tggagggtgg 10740
ctgcccggtg gccttgatct cagggtcggc cctggctttg ggggtgctgg ccgtggtggg 10800
ctcgttacct gggaccgtgg cggcctcatc ttctgagtct gagaaccttg catcgcaact 10860
gccaaagggg tcacagagct tcttgggagc aggtgtgtag ggctcactgc cgcgggagcc 10920
ccggggccgc ttggcggccc gctcatcccg ggagctggcc ctgctgaggt gccgggccga 10980
gtagttggag agagggtcat actccaggtc tgtgggtggc ctggagttgt ccaccacgta 11040
cttgccactg ggaacggggc ggctgtgccg ccggggctgg ctcacagcct tggggacgta 11100
ttccagggca ccgccacccc ctccacctct gccctgaccc ctgtccaggg acgccagcga 11160
gtacttgctg cccggctcgg caggggcggc cagtggggtg ggctggtagc cggcatcagg 11220
gcttaatagg ccgtggctgc cggggctgta gtcgaaggcc agtgggaagg catcctcgtc 11280
cgggcccaca gtggggctgg cgttggggcc gcggggcgcc agggcggggg cctcggcgga 11340
gcggtgctca cgggtcgtct ccagcagctc ccggtagcgc cgctgctcca gctccacctc 11400
actgcgcacg gcctcgatgg cctggttgac cagctccaac tccagcacat ccgggcgcgg 11460
ctcctccccc aggcccaggg tgccattctc cctctgcgcg gggggcttgg gcagctcagg 11520
gttgtagggg tcgtaaccca gccctgaagg gaacagagag cacagctgtg accagcctgc 11580

cggagaggag cccgcagcat gggggcggcc ccggtctcaa actgcaggga agggaagaga 11640
cctgcctccg aaacccacca cgcactggcg cccgccaagg ctctggggtg cccagctggg 11700
tacaggccat ggcgccatct caggccctgt gccgggcccc gccttgccct gaccaatggg 11760
cgctggatgg gcctgggctg cgtgacacac agctccctgg gtactggagg ctccctcaca 11820
cggctcatga aatgcgcaca atgaccatgg tggggtggac actcgacagg ctggaactcc 11880
ccggggcagg cgggtgtgga gctcccagca ttcagggtgc cgggctgagg caggactggg 11940
ctgggccgac aaccacagcc tgacggtgga cacaggagag cccttcctca gccaacacgg 12000
ccccacccag ccagcagtgt gcccgcggtg tggcggaggt gtgccagcag tgtgccagcg 12060
gtggtgtggt ggcggtgtgc ctgcggtgat gtggctgagc agggccgggg gatggggaca 12120
tgcccagttg atgggccacc ctgcaaaatc cacaggaaga tttttttttt ttgagacagt 12180
ctctctgctg cccaagctgg agtatagtgg tgcgatcttg gctcaccgca gcctctatct 12240
cccgagttca agcaaacctc ctgcctcagc cccccaagta gctgggatta caggcgtgcg 12300
ccatcacacc cagctaattt ttgtattttt agtagagatg gggtttcacc atgttggcca 12360
gtctcgaact cctgacctca agtgatccac tgcacctggc ctactttaaa ttaatttttt 12420
aatttcaaaa ctttttgtag ctgggcgcgg tggcgcacgc ctgtaatccc aacattttgg 12480
gaggccaagg cgggcggatc acctgaggtc gggagttcaa gaccagccag accgacatgg 12540
agaaaccccg tctctactaa aaatacaaaa ttagccgggc gtggtggcgc atgcctgtaa 12600
tcccagctac tcaggaggct gaggcaggag aatcgcttga acctgggagg cagaggtttc 12660
ggtgagccga gatcacacca ttgcactcca gcctgcgcga caagagtgaa accccgtctc 12720
aacaaaaaaa aatttgtaga ggtggagtct caccatattg gccaggctgg ctttgaactc 12780
gtagactcaa gcaatcctcc cacctcagcc tccaagatag ctgggactat agcccaagct 12840
gctgtgcctg gcttaaagca cacggctgaa atgtcttccc gggggcagc tgggaacatg 12900
agactcgcat gttatttacg tggttccaga acatagtgta gcagaccctc acaacctcaa 12960
tgccaccaac gtcagcacag aagtcagaac cacagaagcc tcccgtgggg acaggccgaa 13020
ggagtctaaa gaaaacatga gcaagtggag cccagtattc cagcgaagca ataaaaacca 13080
agttcaaata tttactccag aaacttggaa gttgaccatt agaaattcta ctgagcacgg 13140
ccaggcacag tggctcacgc cgatcatctc agcactttgg gaggctgagg cgggaggata 13200
ctgcttgagc tcaggagttt aagaccagcc tggatgacac agatcccatc tcttaaaaaa 13260
atacttaaaa ataagctgag catggtgatg cacacctgtg ctcctagcta tgtgggaggc 13320
taaagtggga gaatcacttg agcccagggg cttgaggctg cagcgagcca ggattgtgcc 13380
tctgcactct ggcctggaca acaaaacaag accccagctc ttaaaaaaat aaaataaagt 13440
tccactgaat gtaattccgt aacatctcta aaggtctcta aaggtcaaca ggtctgatcc 13500
tctggagttg gcaggaaatg gcgccaaaga cgtctgcgtc ctaattcctg gaacctgtag 13560
atggtatatc cccttagatg gcaaatgggg ctctagatgg ggtgaaatct ccccttgcccg 13620
tcactgaggg ggccatggac aggccgagat cttgcagagc agtttcgcaa ccatcccatg 13680
cagcttcaac accaccacct tcagactcgg aatcctcaga acccgtgcca cagacagacc 13740
gcacagggca ggtacagatg cgtctggtgc agccttcact gcaaaaacac aacaataaat 13800
aacgcaggag atgacctaga agccgaaacg caggtcacct gtggttaagg gggaggcgtg 13860
cgccctggag ggacctccaa gtcacactgc tatggggaag aagaggggca aggaatgttg 13920
cacactctca cctttgaaaa gcagctccac aaacccagtg agatcagatt ccaacaaagg 13980
cgcacgggcc cacaccaagc ccgcagtccc atggggtaca gcaaatgcaa tctggccttc 14040
atttatgctg cacccaacac caactgccca agagggacgt gaatgctgac acacatcccc 14100
gacaggctga taaggagctg tgacaccatg tagcaaaagg caacagcccc atgggaacct 14160
gcccgcacca agacgccact tggctctgac ttgattctga ctgaacgtgc agcttgtctg 14220
aaggtcgggc ctcaggtgtg tttcttccac tcagaaaacc tgcctgccct tcggcccacc 14280
tggagatcga cccccacagc gtgctaagac ttccgtgcag agaaatggct ggacgcagcc 14340
aaggggaggc aggcacaaga cctcccactg tgggcgacgc agagaacagc aacagaactg 14400
gaggcttccc ctatggcccc cacccacacc ccctcaagcc ccagcaggca taagaaagaa 14460
atggctggtt tcggccggtt gtggtggctc acacctgtaa tcccagcact ttgggaggct 14520
gaggcgggcg gatcacctga ggtcaggagt tcgagaccag cctgaccaac aaggaaaaac 14580
cccatctcta ctaaaaatac aaaaaaatta cccagccgtg gtgacccatg cctgtaatcc 14640
cagctactcg ggaggctgag gcaagagaat cgcttgaacc tgggaggcgg aagttgcagt 14700
gagccaagat tgagccattg cactctatcc tgggcaacaa gagcaaaact ccatctcaaa 14760
aaaaaaaaaa aaaaaaaaag aaagaaaaag aaaaagaaaa aaagaaatgg ctggtttccc 14820
aagctgctcc cccgagacac acccaccagc agaacaaaag cctccccagg taagtcccca 14880
tcctaatccc tgggacctgt ggccaggcca cctcactcag ccaatagact gcaggtgcga 14940
cttgaacaaa ggaccctgag ggtgggtgaa catcttgcat tatccgggaa cccagtgtcc 15000
tcacagggtc ctttaaagag ggaggcagga gggtgagagt cagagagact ggaaaaggct 15060
gtgctgtggg tttgaagggg aggaaggcgc cctgagccga gggatgcagg tgcctccaga 15120
tgctgggaaa ggcgggcact gactctcccc ggaagccttc gggaggccca gtcctgccca 15180
cagcttgact tgagcccagc gaggctgact ttggacttcc cagcccagcg ctgagagaga 15240

```
gtgtgctgct ttaaggccca cggggacgaa tgcagcctca ccgggtgatg aaagtgcagt   15300
ggaaaacagc tgcccgctgc ctgtacctgc gaaggcggaa ggcccctcct gttcccggga   15360
accctgcagg gacagcggga ggccgggacc cggaggctgc agaggaggtg ggaggaggcc   15420
tggtcacggg cagctgccgc ctttcccttg aaatcacagc agtgttgtgc ccatctttaa   15480
aattacattt agggaccagg cacggtggct cacgcctgtc atcccagcac ggtggctcac   15540
gcctgtcatc ccaggcacgg tggctcacgc ctgtcatccc agcactttgg gagaccaagg   15600
cgggcagatc accagctcag gagttcaaga ccatcctggc caacatggtg aaaccccatc   15660
tctactaaaa atacaaaatt agccaggcat gggggcgtgc acctgtaatc ccagctactg   15720
aggaggctga ggcaggagaa tcgcttgaac ccaggaggca gaggttgcag tgagccaaga   15780
tcacaccact acactccagc ctggcgacag agcaagactc tgtctcaaaa aaaaaaaaaa   15840
aaaaaaaaat tacacttagt gccaggctca gtggctcacg cctgtaatcc cagcactttg   15900
gaaggccaag atgagcagac tgcttgagtt caagaccagc ctaggcaaca cggcgagacc   15960
ccgtctctac aaaaaaatac aaaaaatgag ctgggtgtga tggttgcatg cctgtggtcc   16020
cagctacttg gaaggctgag gtgggaggat ctcttgagcc taagaggtca aggcttcaat   16080
gagctgtgat cacaccaccg cattccagcc tgggcaacag agcaagatcc tgtctctaaa   16140
acaaataaaa ttaagtagca tttagacaag aggaaggatc cgtgtgtctg ttttggacca   16200
ctcggaactc cgcgggactg tgtggagggc cacgaggtac gggcaggcag gggaacatca   16260
cacagaagcc acaggttctt ctttgcctgt gccaggctac agccaaggca tttccaggaa   16320
acgtcacccc atgtgcccag caggcgcaga ggggcccatt ctggcaccat ctcacagcca   16380
aggaccctgg gagtcagcga gggcacccaa ctaggatcag caactgtggc tgccagttcc   16440
tctgtgcggg gagggacagc tgccaggcca ggaggatgct cccggcaggg aaacggccgg   16500
ctggggctgg gtgtcagccc ccacccaggg ggagggctgc ggaggccagc agcagctcaa   16560
gggcgccctc ccggcccact tcactggggg aggctccggc tcctccctga cccagccggc   16620
tgcagagcac gccaccctgc cacccgcctg gtgaacaggg caactagtgc cagccctccc   16680
ccagccccgg aagcccggct gcccagtgac gtggaggggc caggccaagg ccactgtggc   16740
gtgagctgca gagattcatg aaggggaggt gacagagcac aattactgag gaggagcaag   16800
gccaggtcag ggagacactg gtctcccctc ccaaggctcc agctaacggg gatgcagaga   16860
ggaggaagag cccgcctaac gggaaggctc tcctccccag gcaggggtgg gcagcagtga   16920
gactgggccc caggagggca ggcgggggtg gaggggactg aggcatctct aaggggctaa   16980
ggataaggga ctccactcag gggcttccac ctccttgtcc tggaccccag ggctctaaga   17040
cacagcctgc aagatcaacc cagcccaggg gagccaagag tcacaggcaa tacacacacc   17100
agccagcaga cacccgcgag cccttcccaa aggaaggaaa ttcagacctc tggacattgt   17160
ggactctgaa aggagagacc atttccccaa aaggtgcttt tggaaggtct tggaaggaat   17220
agcacctgcc tcccaggagt ggagccaggc cccc aagcag agcgcaggac aggtgataag   17280
ggtccaatct gggggacgtg ggcttgtctt tctaactgga agcctgtgtg agtacttgtt   17340
ttgttgtttt gtggagacag gcattgccca ggctggagtg cagtggtacc atgatggctc   17400
gctgaagctt tgcctccccg gctccagcga tcctcccgcc tcagcctcgc aagtagctgg   17460
gaccacctgc gtgcaccacc acatccagct aattcttttc tttttttggt agagacgggg   17520
gtctcgctgt aattgcccag gctggtctca aacttctaag ctcaagcggt cttctcgcct   17580
cggcctccca aagcactgct tgtcttttaa gggctgtgaa acctgcccca gcctttagcc   17640
aacagatggg aaagagcagg ggcacctgga ggccggaggg cacactctgc agagggggtt   17700
tcagcatttt cactcccggg tggaggctgc taccacctcc acaaagactg caccaaaaag   17760
aaagcaggca tgctaggcgg gtccgagaag actgagatca ggatctgcac cacggcctct   17820
cctccctgtg gtcactgggc tcccccaccc tgcagcctgt gacccactgg tgctggaagg   17880
cagcatcctg gcctgggcgc tccagccagg tctggaagtt tcacacagga agttgcctgg   17940
ggctcggcca tggctgcctg cagctcagaa caggggggtac ggcgtgctga gcccctgaaa   18000
ggtcacctga agctggtaca caccactgcc tccctggacc tccctgtccc accatgcctc   18060
agagggttgg atctgggtac ctgtcaggac tggaaaggcc ccagggtggc atgtgcatgc   18120
tgctaggtgc cctgggagcc cacccagggg gtcacatcta aaggccgagt ttgggccagg   18180
cgtggtggct cacacttgta atgctagcac tttgggaggc ggaggcggga ggatcaccga   18240
ggtcaggagt tcgaaaccag cctggccaac atggtgaaac tccacctcta ctaaaaaata   18300
caaaaaaaaa ttagccaggc ctggtggcag gcgcctgtaa tcccagctag tcgggaagct   18360
gaggcaggag aatcacctaa acctgggaag tggaggttgc agtgagccga gaacgcacca   18420
ctgcactcca acctgggcga cagagctata catgcataca cacatacata catacataca   18480
cacacacata cataaatgaa tgccgactct ggaagtagga ggggcccgtc caggccccgt   18540
cccacggctg ggaattactt tggataggag caggtgggct ccttcctgga tgtggcccag   18600
gtcagtctgt ttctgggaaa catggtgaat tggctgacag gaaaagccta cctccagagg   18660
cctgggagct gccccttcct ccctgcttca ccccaaggcc aggactggga ggtaccaccc   18720
agccccagtg gtcctgagag gccacaacgc ctggctggct atcttcccca gcctctccag   18780
ccagggctca gcccagggca acgcagccca gccttgtccc ggcctaggag agagcccact   18840
ccctacacca cccatcctga ccgcatctcc tgagaccagt gccctgcagc cccgggacgg   18900
```

```
gctggatgac aaagatgata tttcttccca gggttctagg tgtggggcct gggaccagca   18960
ctatctgccc aaggccatct cctggccccc aaaccttccc ttggcccaga aacccctcac   19020
cctgggcatc cactctcctc tctgctccca ctgcccccgc cccaccacac tcaatccacc   19080
cacacagctg tcctgtcccc agcaatgggg agaacctggc atgagctcca accccacacg   19140
gagactgaaa ggcagcaaaa atgacaagat catggagcac gaagatccca acctgaactt   19200
ccagaacgtt ctctagtgag acccgtccaa actcctgtct tctctcagtc agcccatttc   19260
aaagatgagg acacccagtg ccagcgagag ccggcagctc ctagccccac aaggagcccc   19320
aggcccctcc cacccagacg gggagactca gtggctctaa agaacccggc aggctggctg   19380
gacacgatgg ctcatgcctg taatcccagc actgtaggag gccgagacgg gtggatcacc   19440
tgaggtcagg agttcgagac cagcctggac aataaggtaa aaccccgtct ctactaagaa   19500
aacacaaatt agccgggcgt ggcggcaggc gcctatagtc ccagctactt gggaggctga   19560
gacaggagaa tcacttgaac ccgggaggcg gagactgcgg tgagccaaga ttacgccatc   19620
gcactgcagc ctgggcaaca gagcaagact gtctcaaaaa aaaaaaaaaa aaaaaaaaag   19680
aacccggcag gcccagcccc tgctccggac accgttcggt gggcccgcag ggctctcaac   19740
gcaaccaggc atcagcagga ctggcccctc cagcccaggc agtgggcccc gcatgctggc   19800
ccagaacagg ccaggcagag accccagatg gaaacagaca gtccctcccg cctggggctt   19860

agctggacaa aaaagataag cctgacactg cggcatgaga taagcaagac tacccccaac   19920
ctagcccctg ggggagccat ctgggcggcc tgaggctttg aaggtggaga cagaagagga   19980
cattcccaaa acagccacag cggcagagaa ggcacggggc tgaccctcgc gaagcgcaag   20040
gcaggggctg gttgtgtcca cgggagcgcc catcccataa cagaccctct gggaagaggg   20100
gtctggagga cagcacccct gaagtccacc tcctgcggac ggcagagtaa ccataaggtc   20160
ccccacaaatg gccatacgtc cactctgggg gtccctgctc ctggggcctc ccctccaacc  20220
ctcacagtgg gacgtcctcc agccccatcc ttggacatgc gggcgcccgg ggccctccat   20280
gcacagccca ctggacagat aggaacccgc tgctcagagg caaagggacc agcactctgc   20340
acccagagga ggtcccacag ctcctagacc acacggggca gaaacagggt cacctggaga   20400
gggcttcgag gaaaagtcca gccagtgccg ggggcctgga cctccaccac ccagcagctt   20460
ccaataccct ctctgaaatc caaggtggcc cagctctgcc ccacccccca caccagactc   20520
cacccaggtc tcagagcttg ctgtggaggc tgggctgtgc ctgcagcagc ggcacctggg   20580
gtctacccca ctgcccccag ggttctcctc gacccccagg gctgcgatcc gagcctcccc   20640
tctgctccaa ggcaccgccc tccccacctc gacagcgcag tgtgcagtgc caggaccgcc   20700
cagcaggtgg agccacacgg cagggtccag gggagaaaaa gaaaaaggtc ccggcctgac   20760
cggggaccac cagctctgct ctctggggcg tcctgggcat ttattcacct catggggcgg   20820
ccgtccccac atgccatgac tggacgcgct gctgtcccaa ggcttacagc tcgaggctag   20880
gggacaggga cggtaaccag gacagaggtc actgcctttc aaaacaatgt agaaacaggc   20940
tgggcgcagt agctcacggc tctaatcccc gcgctctggg aggctgagcc caaggccgag   21000
gcaggtggat gatttgaggt gaggagtttg agaccagcct ggccaacatg gggaaacccc   21060
gtccctacta aaactacaaa aaattagccg ggtgtggggg tgcctataac cccagctact   21120
caggaggctg aggcaggaga attgcttgaa cccggaaggt ggagtctgca gtgagctgag   21180
atggcgccac cgcactccag cctgggcaac aagaacgaaa ctccatctca aaaaaaaaaa   21240
aaaaaaccag ctgggcgtgg tggctcacac ctgtaatccc agcactttgg aaagtcgaag   21300
cgggcggatc acctgaggtt gggagttcga gaccagcctg agcaacatgc agaaatcctg   21360
tctctactta aaaaaataca aaattagcca gctgtggtgg cacatgcctg taatcccagg   21420
taatcccatg cctgggcgca gtagctcacg cctgtaatcc cagctactcg ggaggctgag   21480
gcaggagaat cgtttgaact cgggaggcgg acgttgcggt gagccgagat cgtgacattg   21540
ctctccagcc tgggtgacag ggcgagactc cttttcaaaa aaaaaaaaaa actgaaacag   21600
aagggtacaa atggcaaagg acaagaaccg gcagggagga gccagcggga ccttctcccc   21660
ctatgcctct ccatccctgc caagagccca ccttggctgt gagccacttt catggtccac   21720
acagagtaag ttaagagtcc agctctcagc cgggcacggt agctcacgcc tgtaacccca   21780
gcacttggga ggacaaggct ggtggatctc ttgagcccag gagttcgaga ccagcctggg   21840
caacatggtg aaaccctgtg tctactaaaa atacaaaaat cagcagctgg gcacggtggc   21900
tcacgcctat aatcccagca cttcggaaag ctgaggcggg cggactgcct tgagctcagg   21960
agtttgagac cagcctgggc aacacggtga aactctgtct ctactaaaat acaaaaaatt   21980
agctgggcgt ggcggcgtgc tcctgtaatc ccagctactc gggaggctg              22069
```

<210> 54
<211> 9724
<212> DNA
<213> Homo Sapiens

<400> 54

```
cccctcggcg cccaaggcag ggtcagcccc actctcagga gacggggggt tccttcacta      60
ccggggcctt tgtgctccca ggcgcatgga ggagggcagc cccacggtgc tggagtctcc     120
ccaacatggc ctgcccaggc agcaccccgg catggtcccc tggggctggg gctccgggtc     180
aggcctgggt cccacggtac ttgaggaagg tgctctccag gaaggcggcc agcttcatcc     240
ggtcgtcctg cggaaggagg gaggcatggg ggacggaggg gcgcggcctg ccccgtgcgc     300
cccctccgcc aggccgcgcc gcaccgccca gcgcacctcg tggaggaagc catagtgcac     360
gagctccgag gcgaggtcct gggcgctgtc cgctgaggca atggcgtaag gcgaggcatg     420
aggccgctgc ccacccgccg cctggggtca ccgcccgccg cccaagtccc ctgcccagcc     480
tacttgggag caggtcgtag gtcagctgcc ggtgcagccg gtcttccagc accagaagca     540
gagtgagctg gggaggcggc ggggcgtggt cggctggggg ttcagggctc cgccacgctc     600
ccgccttccc cacggcagcg ccgccctccc aggccccgct cacatgccag cgcgccttgt     660
cctcgcttct ctccaggttg cactgcatct ggatgacctg cagcgggggga aggctgggac     720
tcacaaaccc agcagcaaga ccacatgccg cggccccaaa ggggccggga agggacagca     780
aagactggaa agggggggaat ctctaagagg agcccatctc actgctagtg gcttggcaag     840
ctaaggaaag cttcgagaag agtcgacatt aggggctggg tactgaaggc tgaataggag     900
ctttcaccgc ggagagaaca acttgaacaa agagggaact gtgggcgtgg ggaaagggag     960
gttgggcggg acaggaccgc gccgaagtgc acggtgcctg cgggatcagg cctcccagag    1020
cctgggcgtc agctgaggac tcccatgatc tctgctggca gccaggagca ggcagtttgc    1080
ctgttaggac atgaattctg gctgcactgc accgagagag gcccctggcc aggagggggt    1140
gctgcaggaa aagtctgggg agacaccagg acatagatgt ccgggaatca gcaccaggtg    1200
ggggatggga ggggtggggg gggggtggga ggggtgcggg ggtgggggaa gattgctggc    1260
agggtggaag agcccgggtg agttctggag gaaggatgct tggagtaggt tcagggcagg    1320
cagcaggatg caggggaagg atctgagggg acagggagcc tcccggaaaa ggagagtgcc    1380
cagcctgtcc tataaccccc atgaccccca caaggggggtc agcctggatc tggtctctgg    1440
cagccaggag gagagctggg ctgtggggat gcaggcccta agagaaggag gggcacctct    1500
tatctggctg ttagaaggtc tgagggatgg cactgagcag caggagaggg gactcacctt    1560
tctggtctca gagtcaaagg gctctggcgt cggggtcttg gccttttgga cctcctccgg    1620
gggtggggcc agcacacggg gcagccccag gggtcgagtg gctgcaaagt tcatcagtgg    1680
gtagattcca ttcctgggga cacaacaggg tggctggggg ttcaggcctg acagctgcct    1740
ggcccccaac cttacatcag ttctcacctg acatcctcca ggaatttgtc cagctccatg    1800
aaggagactt ccgagtacct ggcatggaag tgggaggcac atgaggggct ggccggggcg    1860
aggggagggg aggtgcgggg aggggatgtg tggggagggg aggcttctgg gtgctgactg    1920
ctcaccgcca ctgcagcggg ggcctgcggg gccggggaag ctccgccaag accgcgtgca    1980
ggtccatggc cttggtcttc tcctccacca cattctcagg catgaggtct gcggccacaa    2040
gagcatcaag gcggtgtgct caggccgtgg gtcctgcagg gccccttcca cgtcccaacc    2100
tccacctccc cagccacccc ccgggccggc ctgctgctca cactggtgct ggatgaagca    2160
gtgggctgcc aggagcttca gcgagtgcac ctcgaagagc acgcggtgga agaggaggct    2220
gtgggcagag ggccggcggg cagggtcccg ggccaggcag caaaggatga actcctgggg    2280
cacagggagg agaggctggt ggtgtgcaag ggctctctgc tctggccccg cagttcgagg    2340
agaggtggcc cctgagttcc ccatgtccct cctcagggac acacaggaca tgcagggatg    2400
cccatagggga ggagtcccag cagcagcagc caggccagga cctggtcctc tgagcttgag    2460
gacagctggg ttctgggatt ggagcaccat gctctgcttc cctcgacccc caaaaaatcg    2520
tggggagaag ccatgatcct aaagtcacac ctgcaagcct cctacccatg gccttttgat    2580
cggctcagct cgggcccaac actggagacg accttgatgg gtccccactc tgtccacatc    2640

cgtgcccagc ccggctccag ctcgccacca gccccgacca ggcaccccca tctcctgagg    2700
ccatggagtt aagtctagac tccgagcaga gggggctgtg gatgcagctt ccatcccata    2760
cctctttttg cctgcttggc acccaccatg tccccccaca agctggagcc tcatcacctc    2820
ctggaagctt aaagtcaagg acttggagtg aagcagaccc aggtgcagat gttggctcca    2880
ccactcactc taaagccttg ggcaaaggac tcatctcctc tgaaccttgg tttctttata    2940
aaacgggcat ggtgatggca gccacagagg gagggtctct cttgccttct tgtccttaga    3000
atgtgctgac cccttagggc cttcaggcct ggaaccagta tgtgctccca ctgaggtcct    3060
ctttgggagg ggtgcagtca gctggtatac ccctcaactg cacaaactgt gatgttctcc    3120
caggtctgcc tggagcacct tgctagccct agctgagcac ggtgagccag ctggggaggg    3180
gcagggcaag ctgcttaccc gcatgttggg gtcactcagc gagtgcctgg cgcgagcaat    3240
ggcctcctct gtgacccggg tgtccccatt ggtctggatt tccagtacag ccatctgggg    3300
cacagagcca ggtcaggcat ggggaaggga ccacacaggt gagccaggca gggcacagtt    3360
aggaggaggg cagagcacaa ggtggagggc gggggcagta cctccagcgc acacatccca    3420
aaggagaaga tgtccacagc ggtcccatcg gccacctctg aacagaagag agcacaggac    3480
acgtaggaga gaagcgcagg gtaggcacgg ggcacccgga cccagcacca ctcacctcca    3540
```

tactctgggg ggaagaagtg caggttccga agttcctctc gctcagcgcg gatggggctt 3600
cggagatcat ctggaagtgc tgtgggaggg cgcagagctg agcgggcggg gacctctcca 3660
ggaccccgtc cccccaaagt ccgcacttac cattggagaa gattcggtgc cacactgcca 3720
aggggcggga gaaagagtgg agttagctgc tggggcatca gaactcctct gcccttggct 3780
ccaggcacct tcccctgccc cgttccccca cccagccctg ccccgccagc accggagccg 3840
atcttgatga ggccgttgtg ctgaatgaag atggtgtcgc tggtcaggtt cccgtggatg 3900
attggggggc tgcaggcgtg caggaagctg cagacgttgg ggaggggaga gtaggaggag 3960
ccggtcagga ggctctggag agatgggggc tcggtggcgc cgcgcccagg ccagcccagg 4020
ccctcacctg agcgcagaca ggatctgcgt gcaccagcgc ttccaggcct ggcggcggac 4080
gcacgactcc gtcggtcggg tgggcgcagg agaggcggct gggcctgcgg agcccgcccc 4140
gcatcctcgc ccagcccctg tccgaggccg ccgggcaccc cctcaccatc ccagtccccg 4200
aggctgcccc aaccccgtcc tgtccccgtg gctgccccag cccgctcccc atacccgggc 4260
gttcatggcc ttgtggttct tcttggtctt tttgaggaat tgcttgaggc tgcctgatga 4320
cacgtactct gtgatgaaga tgacctgcac ggtgcgagct caggatttcc accagctgcg 4380
ggttcgtccc catgcccgcc ccacctagct gtggtctctg cctgcccggg gccttgcccg 4440
tgctcaccct cgcgcaggcc tcagaggtat ccagccagta cttgtgcaac ttcacgatgt 4500
tcgggtggtc caccagcacc agctgctcga acacggtctg gatcttctcc tggggaggga 4560
gggtggtcgc tgggtggtca gcaggtggtc acccaagcag gatgaggagg gggcagcggt 4620
ctcacctcgt gcgccgcgaa ggccttcctg tctccgaagt ggagctcgtt ccacaccacc 4680
tctacccccct cctccgtgtc catggctagg aaggtgctct gaagccctgg catgttccct 4740
tggtttacct gggggtgaat aaagggttat gtgtgccctg gtgtgtgtca gggttgtggg 4800
tgaggatttg gtccctgtcc acacctttcc agtgggccca agcgtgggct gcaggccctg 4860
agccactctg cgggaaggtg gggcttggag ggtagctgcc cccaggagcc aagggctcct 4920
atccaagggc tgggctaagg ggatttggag caggtttcag ggggtcgagg gggatcccca 4980
ggaagctagc ggctgagtcc agaggcatgg ggaggtggtc ctgggaggag actggccctc 5040
agggagtccc agggcgagcg ccaggccaaa ggggtccagg ggtggctgat tcgcgggccg 5100
cgaggggccg cggagaggtt tccagccgcc gcgctctccc agcgccccca cgccccgcgc 5160
agcctccagg cccctcccgc tctgggaggg cggtgtcacc ccgtgcccca acccccgcgc 5220
ccacctgctc ccgtcgcttt tgccagcgac cacacgggct ttcctccagg atgtcgctct 5280
cgtcctcgct ctcgtcctcc cgctcccgct cccgttcccg ggcccgcctc ggcgccggct 5340
ccgggggccgc catggttcgg ccagcccagg ccaccgccct ctgcgcgatc cgccgccggc 5400
gcagcctctc ccggcccgcc ctggcctcgc gcccagcagc ccagcctaga gccgccgcgg 5460
cagcctagag ccccagcccc ggctctggga attgggaggc gcgggcgcgc ggcggacggg 5520
cggggcccgg gggcggagcc ggcgcgcacc tcccgagccg cctgtgcctg ccggagccag 5580
agcagagctt ccccgagagc tgccgagccc gggccgcgcc caggccccgc ccccccggag 5640
gccccgcccc gcgaagcccc gccccagtcc ctgtttcctc ctcgtctccc aggcctgctc 5700
cgcgaagccc ctccccaggc cctgtcccct cctcgtcctc caagcctcct tcgcgaagcc 5760
ccgccccagg acccgcccca cccccgcgaa tccccgcccc gttttagtcc ccgcccccgc 5820
ccgttcccag ggtcctccgc gaagccccac cccaggcccc gcctcacctc ctcgaagccc 5880
cgcccctatg aggccccgcc ccgcgcagag gaaccgcccc caaatccccg ccccctcgga 5940
ggccccgccc tgcggacacc tggagcccgg agcgcccact tctctcgcgg ttgctcccct 6000
aggatctggg ggaagacccg gggcatctgg gggcccccaa gcccacggca ccctccagct 6060
gcagaagacc ccgcgggctc gaaagcggga cccgctctcc tggccacgca ctcattcccc 6120
ggccgacccc caagggacag gtcgaagggg caccgagggc gggggaaagg cccaccagca 6180
ggctgaccca ggctgacttt gatcggtgac cacgaatctc tccctggccc gcccaccagt 6240
gcctcctccc gcccacaagc ccatcttctc actttccagg atggcgattt tctcttttct 6300
cctcaaacgc gcatttcctt tccgcggctg gctcccatgg gacagtggaa ccatgagggg 6360
ggagcccacc cggctcacac gacctctccc tccggaggcc gctctaagca caccctccag 6420
ccacgccgac caccggggct gtccccgatt ctcacctaaa accccttca gcccacagcc 6480
cccacggcct tcctgtggtc tcctccctcc ttcacggcgg gtcctcatcc tttaccttct 6540
ctctctggag cccaaattgg aactccgctc ctcccctccg cgcagctcca tgcactggcc 6600
ctttgggacg cctctttctt tctttggcag aggtgcatct ggccctgctg cgtcccctgt 6660
ggtctctctt ttctgaccag ccttggtcac caggcacctt gtgtgtggtt tccaccaatg 6720
ccaggtgatc ctgctgaggc cctctgcagt aaaggtttgg ggtgttcaaa tcctccaccc 6780
tgaaactggc cctgaccagc tcctgggaga caccctctaa gcccttggaa tgttctgcct 6840
gatttgaatg tctgtgttta cctcggccac accagattct atgctaagct tgtccaaccc 6900
gcagcccaga gggtgcattc ggccaaggac agctttgaat atggcccaac acaaattcat 6960
aaactttctt attgagattt tttttaaagc tcatcagctg ttgttagtgt taagtgtatt 7020
ttatttaagg gtattgccca ggacaattct tccaatgtgt gccaggaaag cccacagatt 7080
ggacacccct ggtctgtgct aacaatatgg tctacggggg gaccttgggc catgtggcat 7140
cagcctgacc tctgcagggg ctggaggcca aagtcagcca cctgggaggt cagccatgcc 7200

```
tgtgtgacca acccaataaa aaccctggac actgaggtac ggagggcttc cctggctgct    7260
gacatgctgt gctcattgtc acacctcagt gctgggaggt tgagcactgt ccccatgcct    7320
gcagtgggtg gggacacctg cagacccgtg ctggtctgtc ctgggttctg ttctgtgagt    7380
ccttgccact gctgatttta acgcatccat tgctgtaata aatagtaccc tttccgagtt    7440
ctgtgagtcc ttctagtgaa ccattgaact tgagggtggt tttggggagc ccccccagtg    7500
gtcccttctc acttggtatg agggcctaaa catgaaggcc cagtaattgg tcccattacc    7560
ctacctcacg tcagctgcct tcttcagctg agcccacccc aagtacacgg ccctgtgcca    7620
tcggacatga caggcacttc ccctcagggt ctctgccctg gttatacctt tgcccggaac    7680
gcccttccca aacatcctct gaggtcactt cctgagaggc ctgtcctgac cacgaatcct    7740
gacatcacag tccccacacc ctttcgcctg cttgacttga cttttcccgg agtattcacc    7800
ccacctgact tgcatctggg tttttctcat ctctcttctt cactgtctgt gtccctgttg    7860
cgattcgagc tcccgcaggg caggagtgtc tgctcgttgt ccctgctctt tctctggtgc    7920
gaggacggta cccggcacac agcaggccct cagcaaacac ttgttgaatg gatctgtggg    7980
tgggcagggg tgcgttttgg cgggtgtggt ccagagaagc ccctaaggag gtaagccttg    8040
agcagaagaa tctcaatgaa tttcctcccc tttctccgcc cccataacca gacacacatg    8100
ttctgtggta ggtcacatag tgtcccccaa aagtctacat cctgatcccc agtacctagg    8160
agcacagcct tatttgaaaa aaaaaaaatg gtctttgcag atgtaaataa gtttaggatc    8220
ttgagatgct ggggtgggcc ctaaatccaa tgtttgatgt ccttatcaga gaaagaaaag    8280
ggatacttgg ggtgcagaga aataaggtag acagtcacat gaacatggag gccagtgttg    8340
gagggatgta cccacaggtc aaggcgtgcc tgggactcga ggacacgagc tgttccagta    8400
taataaaata tataaaataa gaatagttgt actgatatag atcttagata tgattatata    8460
tgaatatcat taatcattag tttgtagcaa ttactcttta ttccaatatt ataataatcc    8520
tcactctata atcatagcct aggaaaaacc aggccataca gagataggag ctaaggggac    8580
atagtgagga gtaaccagaa gacaagagtg cgagccttct gttatgccca gacagggcca    8640
ccagagggct ccttggtcta gcggtaacgc cagcgtctgg gaagatgcct gttgccaagt    8700
ggaccttggt ctagcggtag gcgcagtgtc aaggaaaaac acctgctact tagcagaccg    8760
ggaaagggag tctcccttgc cccggtggag tttaaagaag actctactcc tccacctctt    8820
gtggagggcc tgacattagt caggctcgcc tgcagttatc cggaggccta accgtctccc    8880
tgtgatgctg tgcttcagcg gtcacactcc tagtccgcct tcatgttcca tcctgtacac    8940
ctggctctgc cttctagata gcagtagcaa attagtgaaa gtactaaaag tctctgatat    9000
gcagaaataa tggcgtaggc tgtctttctc tctgtctcct ctctctctct gcctcggctg    9060
ccaggcaggg aagggccccc tgtccagtgg acacatgacc cacgtggcct tacctatcat    9120
tggagatggc tcactctcct tatcctgccc ctttgtcttg tatccaataa atatcagtgc    9180
agcctggcat tcggggccac taccggtctc cgcgacttgg tggtagtggt cccccgggcc    9240
cagctgcctt ttcttttatc tctgtcttgt gtctttattt ctacactctc tcgtctctgc    9300
acacggggag agacccaccg tccctgtggg gctggaccct acttgggact gccagggcca    9360
tgagtggtga ggagagagtc ctgggatgga ttctccctcg gagcctccgg aagcatcagc    9420
cctaccgata ccttgatttt gcacttccag cctccagcac agtgagcgaa tctgtttgtg    9480
ctgttttacg ctactcctcc atctctggtg ctgctccagc atgacccccg tggtaagaac    9540
agtaacgtga caacagctat gtgttcgcgt gcagggatgc gtcccagcat ttattcccat    9600
gcacagagtg ggtcgtccta ctgcctgccc acgcacagaa gtggtggccg ctgcggcaca    9660
ccctccctgg cctcactcag gctgtgcccc agggcggggg cgtaccctcc cttctgctcg    9720
gcct                                                                 9724
```

<210> 55
<211> 1301
<212> DNA

<213> Homo Sapiens

<400> 55

```
aagtagccgg tgtcgcctct gaacacaccc gggcagggcg agcaggatgt ccttgtccag      60
ggagacccct tctcctggct ttggggaagg tgctccctat ccccgcagga ggctgggtct     120
gcaggacgca gcactgggcg ggtgagagaa agcctgggca gaagagcacg gagccggcga     180
ctggacgcga gcaaggggcg aatggagagc gtctcccctg cgccaatgct gttgttccgg     240
gtgctgaaaa gccgggaact tcccggaaag cagcggcact tttctccctg gaaggtgggt     300
gtgggggttc caggcttcgc acctgctaaa cctgcctcgt cacctgggag gggtccgcca     360
gatgtgcccc tcctcagccc cattttgggg agaacgatgg gggcgggggc gtgggggtga     420
gcacagccgg gcacccccgc acacccgcag gcgggacccc ggggtgccct tgccctcctc     480
tcctgggcag ccgcacgccg ggatccccac tccacctgcg cgcgaacttt cgccatctgg     540
```

gactcaagcg cccgcggctt gagcgctgcg gtaaacagaa ggcacccgcg tgggttccca 600
agggtgtttg tgtgttgggt ctgggggagc atgatcgggg cattcgcagt ggggaggaag 660
aggtgctgct ggccggggcg agctgcctgt gctaaggccg ctccctgcag ggcgagttcg 720
tgcacgacga cgtgtgggcg accgtgaaca accccaacgt gcggcccggc gggcgccccg 780
ctccgctgag gcatcttcac caacgacttc ttgggccagg gcatggccga gaacaccagc 840
cacaagtcct agcggccgct cagcgtcccc accttcaagt gcactcctgt ctagggcttc 900
cctttgagcc agggccgggt gtagttgcgg gactggcttg aagggaattt ctatttctca 960
tcacgccttg gcctctctgc ctccctgcct ctcccactcc agctgttcac gccaccaggc 1020
tcccaggtcc tctccaggct aatctgggtt cctgggacca ggcgggaggc gcgcgctggg 1080
gagttccctt cgagtttcca gagtttcccg aggtgtctgc gttctaggct gtttgttttc 1140
tctggacaga cctgggaatg ggcggctgga gcggccacct cacttccctt ccgccagccc 1200
ctctgggtga gcaagaaaac cagtcctaca cagttggccc cggaggtgtg gtgtgtgtgg 1260
aaggggcggg ggaaatgctg ttttcagggc cgtaatttct c 1301

<210> 56
<211> 22355
<212> DNA
<213> Homo Sapiens

<400> 56

aggtgaacac atagcacccc ccgggttcca taccagccct gggcgccagg gacacagcag 60
tgaatagaac agatggagcc cctctcctct gtagcggggg gctgcaatag gggggcccac 120
tggacaagga gcacattccc accagagaga acattctagc tgggtgagcg gccctgcagg 180
gggccagcct gggggaacct ctggagacgg tgggaattga acaaagcctc tggggaggcc 240
aggcacggtg gctcacacct gtgatcccaa cactcgggga ggttgaggct acagggagcc 300
atgatcacac cactgcaccc cagcctgggt gacagcgaga tcctgtctca aaaatttaaa 360
aactgggcca ggcgcagtgg ctcatgcctg taatttcagt actttgggag gccaaggagg 420
gtggatcact tgaggtcagg agttcgagac cagcctggcc aacatggtga aaccctgtct 480
gcattaaaaa tactagagat taggccaggc acagtgactc acacctgtaa ttccagcact 540
ttgggagacc gaggcgggtg gctcacctga ggtcgggagt tcgagaccac cctgaccaac 600
atagagaaac cccgtctcca ctaaaaatac aaaattagcc gggcatggtg gcacatacct 660
gtaatcccag ctactcggga ggctgtaatc ctagctactc gggaggctga ggcaggagaa 720
ccgcttgaac ccgggaggcg gagattgcag tgagctgaga tcgcaccatt gcacttcagc 780
ctgggcaaca agagcaaaac tccatctcaa aaaaaaaaaa aacataaaag attaggtgga 840
ggttgcagtg agctgagatc gcgccactgc attccagcct gggcaacaga gcaagactcc 900
gtctcaaaaa aaaaaaaaat ttaaaaagcg tcttgaagga gttggggggg gagagaagac 960
agggccgcca gggctctgag cgcagcgcca gcctgtgtgg ggcatgcagg ctgtgcccca 1020
cgcctgtgtt gggagagaga gtagatgggg tgcctggccc tgtgagtggt tgtgagtatg 1080
tgtgtccgag ggcaaggggg actctgtccc ggctgcatgg gtgttaggtc cacggcatgc 1140
cccgggtgtg tgttgtgcgt gcgtgcactt gcacggcatg tgcgtgcatg ttgacatggc 1200
agccggtgca cctgtggctc ctgtcggcag acacgtgccc tccagcctct cgctctctct 1260
catccgttct ctttctcatc ctcaccctct tgctctcact ccaccctcct gagctgccat 1320
gtggccatca ccacctctgt ggcctctgtt ctcagggcct ttgtgtacct ccttccccca 1380
gtgcctacct gaaccctccc ttccatcccc ccatgctcca gtctgtggtc cggcagcctc 1440
ctccccaggc tccggggcca ccctcggaag gcctggtggg ctctgtcagg gaaggggccc 1500
acaggactcc tcgagggctg tggtcgtcaa ccgctgagtg cagggtgtga gcgggactcg 1560
gatactctct gagggcggga actaggcagt gtctcaagag atgccaggtg cacaggggtt 1620
gggggctggg ttgggggggag cacaggccct cgggagccag aaggggactg gggctgtggc 1680
tcaggccatg gacagagcag aggcgcaggg acctgaagaa gcagagggag aagcctggcc 1740
aacatagcga gaccccatct ctactcaaaa gacaaaaatt agctgggtgt ggtggcgtgt 1800
acctataatc ccagctactc aggaggctga ggcaggagaa tcacttgaac ctgggagggg 1860
gatgttgcag tgagttgaga tcacgccact gcactccagc ctgggtgaca gagcgagaac 1920
ctatctcaaa aaaaaaaaaa aacagtggga ggtgacgtgg tgctgcagag gcggcagtgg 1980
ccaggacagc gggaggggca cgggaggtga ggcctctagg ccagtcatga acgctgggcc 2040
tggctgtgtc ccaaggtccc cagagccctt cccctgctcc tgcctggtcc tccaggaggg 2100
gagctagggg gcacagctcc tgcaggggct ggcaggacca gataggggca atgctgggcc 2160
tcctgcattg ggactgaggc gtcttcctgc cctcctact ggctgagatc agggtcctgg 2220
gcacacggga gggacggggt ttagctgcca gcatggagcg ggtttgtctg ttcatattag 2280
tccctgggca ccagtcacca ggagcctgca gaactgtggg gagaaaaggt ggacgaggca 2340
ggaggaaaag aggggagagg ggcttgttgg ctgagggctt tggagagcgg caggccccag 2400

```
ctcctgtggg aggggcacct tggtaggtgt gtgtagccgt tgtcagggcc tctaggatgt    2460
gacgtgtcag gattggctcc tctggaggcc acgttagcat cccggcctct gttcctgcac    2520
cctgttgcca gcccagccag ggagacccta gctcctccgg gaagctgccc tcctgtccca    2580
ggcccctctc tggcctgtct ctgcagcgcc tcctctgggc acctgggccc tagtcttggt    2640
tcctgtctgt ctgtctgtct cctgccctcc cacccctaat gccgcctgcc caccccaggg    2700
tgcctgctgc ctgcccgccg cctgcccacc cgctgtagca tgcccgagct gcccgcatgc    2760
aggctgctgg atgagccaac ccccgcctgg cccagcattg gccccacctg gtcccgccag    2820
cccctgcagg agctgtgccc ccgagctctc ctcccagagg atgcaggccc cctgaggtcc    2880
tgtcctgccc ccatgggccc cagctctgca ggccaggcca ggaggccctc atgtcagccc    2940
actggcccga ggggggccct ccctgacccc ggaacagaag atagggcaag gcagcctgaa    3000
gcctgggtct cgtgcccgct gcctccccag cgtggccctg ctcgctcttg tctgggagcc    3060
aacttcagtg ctaccagcag agccacagcc tgggccagcc ctggcaggca ctccgccctg    3120
gcctatccat cctgtgggtg ctgtggccct gggcctgctt cctgggcaga agtgggagga    3180
agtgcacaca cctgtcaggg aggcccccctg gccgagggac acctcaggtc cccatctcgt    3240
ttctccgtca gcgtagactt ggggctccct ctcccctgct agcctcctcc atctctttcc    3300
aggtcagggg atgagacaag ggaccctgag aacagtctgg ggtgggaggg agaccccagc    3360
tcctccagcc cagcagggca gggggctgtg ccagcctagc tcgggaaacc caggagggga    3420
aggtgagggt cccgacccct ccctggagtg ccagatgtgg agctgacacc agctgggcgc    3480
tgacccccct ccctgccact ggttctcacc cccctagccc tgcagctccc gctggccaac    3540
gatgggggca gccgctcgcc atcctcagag agctccccgc agcaccccac gcccccccgcc    3600
cggccccgcc acatgctggg gctcccgtca accctgttca caccccgcag catggagagg    3660
tgagccaggg gcccagcagg gttgggtggg aagcagcatt gaggggccac cgtgccagcc    3720
ctgggaggag ggtggttaaa ccggtgggaa ccccggtatg gggggactggg gtggccaacc    3780
taaggtcagg acccacctcc accggcacct gctccatgtc cccagcattg agattgacca    3840
gaagctgcag gagatcatga agcagacggg ctacctgacc atcgggggcc aggtaccacc    3900
ttcactgtgg cggggagagg gaggaggccc agccaggctg gacccatcct gggagcgcca    3960
gtggggggca gggggcggtg gcagaggccc cagggaccct ccaaccctcc ctctcctccc    4020
agcgctacca ggcagaaatc aacgacctgg agaacttggg cgagatgggc agcggcacct    4080
gcggccaggt gtggaagatg cgcttccgga agaccggcca cgtcattgcc gttaaggtga    4140
gccttggcgg ctaccccggc tgcgccccac accccaggcc ggtgctgagg ctccctcctg    4200
tccctgcctg tgcagcaaat gcggcgctcc gggaacaagg aggagaacaa gcgcatcctc    4260
atggacctgg atgtggtgct gaagagccac gactgcccct acatcgtgca gtgctttggg    4320
acgttcatca ccaacgtgag tacctggccg cgccctgcag cgtctcctcc tccctcaccc    4380
ctgccccttc ctagggagca gagcctctgg ggggtgggcc ggaagacaca gctcccccgg    4440
gtgcccctct ccctgcagac ggacgtcttc atcgccatgg agctcatggg cacctgcgct    4500
gagaagctca agaagcggat gcagggcccc atccccgagc gcattctggg caagatgaca    4560
gtggcggtga gtgaccaggc ggggcttgca ctgggcagga tgacagaggc ggtgagtgac    4620
caggcggggc gtgcactggg caagatgaca gtggcggtga gtggccaggt ggggcgtgca    4680
ccgggcaggt ggccttgggt ctgtgccccc tgccacatgc accccccctct gcgtgcctgc    4740
agattgtgaa ggcgctgtac tacctgaagg agaagcacgg tgtcatccac cgcgacgtca    4800
agccctccaa catcctgctg gacgagcggg gccagatcaa gctctgcgac ttcggcatca    4860
gcggccgcct ggtggactcc aaagccaaga cgcggagcgc cggctgtgcc gcctacatgg    4920
cagtgagtgg gggcccccca gcgggggagg gggtgggggc tgggaggccg gccccagcct    4980
tggagatacg tcttctcctc ccccccctgc agcccgagcg cattgacccc ccagacccca    5040
ccaagccgga ctatgacatc cgggccgacg tatggagcct gggcatctcg ttggtgagtt    5100
ggggccctcc cctgttctcc agccaggagt gagggcttct gggggactcg gagggaggag    5160
aacataaacc tgtccagccc tgcccgtctc cctcccaggt ggagctggca acaggacagt    5220
ttccctacaa gaactgcaag acggactttg aggtcctcac caaagtccta caggaagagc    5280
cccgcttct gcccggacac atgggcttct cgggggactt ccagtccttc gtcaaagact    5340
ggtgagaacc tccctccact tgggaggtca gggacagccc gctgctctgg gcagctgggg    5400
aggcaatggc aggaggggaa tggaggccgc accctgggat gggcggatgc gactgtgggc    5460
gggctcctgg ctggcggctg ccacaggagc tggagctggt gcccagggag ccatgagctg    5520
ggtttggacc tagcctgagg ggacagccag ccctgtggca tttggcgggg tggctgttgg    5580
agattgtgag cacgcttctg caacaagcac agctacaagt tcaggagggc catgttcgat    5640
tctctcaggg gagccccacc cacctttctg gggacctca gccagctctg gccccccaag    5700
ccaggccctt gccacctggc cctaggccag gaggctccgg gactcacagt tgctccccgc    5760
tgtcagccgg ggtcctgagg acatccacag ctccttcccc cacacacatc ttggggaccc    5820
ctggcccctt ggggagggcc tgagcacagg ggtggggccg gcatcccctc ctccctcgtt    5880
ctcacatctg tcttcccttc tcttgctcta gccttactaa agatcacagg aagagaccaa    5940
agtataataa gctacttgtg agtacctgag ccctcccagt ccccgtcctg tccctgcgga    6000
ggcgcgaggc caggagggaa gaccgtctcc tcctaagccc caccccctcg ggcccacagg    6060
```

```
aacacagctt catcaagcgc tacgagacgc tggaggtgga cgtggcgtcc tggttcaagg    6120
atgtcatggc gaagactgag tcaccgcgga ctagcggcgt cctgagccag ccccacctgc    6180
ccttcttcag gtagctgctt ggcggcggcc agccccacag gggccaggg gcatggccac    6240
aggcccccct ccccacttgg ccacccagct gcctgccagg ggagacctgg gacctggacg    6300
gccacctagg actgaggaca gagagtgggg ggtgcccacc cacccccccc gccccgggcc    6360
taccaagccc ccgcccttcc caccccgggg tcagccggcc gtgtgcgtcc cccgacagac    6420
actgtgaacg gaagacagca ggccgcgatc agagtcgctg ttcattcagc cgcagcctct    6480
gggccggggc ggcccccagg ggccaggaga gagccctgga gtcccgcagc caccatgcac    6540
gctcccagcg tgctgtgtcc ttcgccactc ccacgcgccc gttcctcttc cgtcgccctc    6600
tgtcccctgc tctacctctc tgtccttgtc tggctctccc gtcaccctcc ctgcctctgt    6660
ctctcttctg gcctgagcct gggcccagcc acctcctgac gggtcccctg ggtctgcata    6720
ggtctcccat ggcgcaatga gtcagtggcc cccagccagg cagtgtgggc attgccactg    6780
cggctggacg gggctgcgcg ctcgcgctct ctctctctct ctctctctct ctttgatctc    6840
agggggtcct ttttggagtt tattgtattt tattgtactt ggtggggtgt ttggggtggg    6900
ggcggaggag agcttgttct cgtggggttg tcggtacctt cagaaacttt taccaaagtc    6960
acgattagct gcttgtggtg gggcccccaac cgccctcggg cactggggag ctgggctggg    7020
gctgctgctc tggggtctcc gggggccaca gcttggggtg agttgaagac ctcaggggat    7080
gtggaggggt ctgcggggcc ctggccgcac aggatggcct tcagggaagg tggtcttggg    7140
gcatggtgca gagcaggtga ccggagggaa tcggtgacgg agcggggcca agggaggggt    7200
ccggagggag tcagggatgg agggcagagg gagtggatgt gggggtttga ggacgtgtga    7260
caagctccag caggggtggg ggccgggctg agggtggggg tgcgaggtgg tcactcccat    7320
cgtgcccctg gccgtccctc cactcaccca cacctggccc agtccacgtt gaggtccagg    7380
actgggaagg accgggtgag tgcaccgggg acccaggcca ggtgcccccc ggagcctgct    7440
ggggtggcca gagcaggagg gggtgtgttt cctttttgtg ggtgttgcat gcaaatcaag    7500
tggacaagaa aaaataacaa aacaaaaaac aagaaaaaaa aaacacaaaa ccccgtaaaa    7560
tcacaaagaa aatccaacac caaaggcgca gaagccggct ggccgtggtg ggggcagcgt    7620
aggcgtagca tccctctcct ctcacttagc ctgttgactc ttgttattat catgatattc    7680
acaaaacgcc gcatgtttaa aaagtcatag atgtcatctt ctctctgccc ccagggagga    7740
aagccacctt ctcttgcccc ttggcccctt tgtcaggggc caggggtctg ccgggtgggg    7800
gtgccaacag gcctggccct ttcctcccct gcatccagcc atgggggcct ctgcgattgc    7860
cggaaggttg catggctggt cccagggcca gcacaggccc gaggccgggc tgcctggttt    7920
tattttttatt taactttatt ttctgtttta tgagtgtgtg tccgcccacc cccacccccct    7980
tcagtgttaa gtggggagcc ctgggggagt ctctcctgcc tcccagcctc tcccaagacc    8040
tcccccctcg tcaccagcca tccctctgga ccaggcagag ggcggaccgg gtgggcaggg    8100
gcctgagggt ggctcgggcc agcccaccag ccaatggacc cctcctcagg ccgccagtgt    8160
cgccctgccc ctttttaaaa caaaatgccc tcgtttgtaa acccttagac gcttgagaat    8220
aaacccccttc cttttcttcc accgagtctg tggtgtgtca tgggcctggc agggcgaggt    8280
gggaaggggg tggttgcggg gggaggtttc tgcattgagg ggagactgag gcaggtttgg    8340
gagctagacg ggaggtgttt gtagtgactt gccgatgggt atcaagcagc tggtgtaagg    8400
gaccccctgc ccccagtggc cacaggtcgg gcagcagagg atcccttaat cccctcaggc    8460
cttgggcatt ggctctggtg tcagcccctc agtctgccac ccccgctccc tgcctccctc    8520
cttgccaagg acagggccgc ctgcccacgt gcgggacctg ggtgcagccc agctggtgtc    8580
tgctctggcc ccaaacactc cgacctcagc tggtccagca tgctgggcac cgtgctgctg    8640
ctggccctgc tcccagggat caccacctta cccagcgggc cacctggtat gtatgggcag    8700
ggaggtgacg ggtctgtcat gcttctatcc ctgtttcacc ctctttattt tttttgagat    8760
ggagtcttgc tctgccacgc aggctggagt gcagtggcgc aatctccact caccgcaacc    8820
tccgcctccc cggttcaagt gattctcctg cctcagcctc ctgagtagct gggactacag    8880
gcacgcacca ccacgcctgg ctaattgttg tatttttagt agagacagcg tttccccatg    8940
ttggccaggc tggtctcgaa ctcctgacct caggcgatcc gcccgcctcg aacttttcaa    9000
agtgctggga ttacaggtgt gagccaccgc gccccaccga tgtcactctc tttcaactag    9060
acctttggtg gtggtcctca tgggggtggg gacagggcag gggagtgggg tggggtctct    9120
gtcccccatc ccaagggtgg ctcacatccc cacttcggca aagctccctg cagccacgga    9180
gagggccaga gttgtgggag ggggccagtc tacccaggtg ctcttcagaa gtgggtgtgg    9240
tgtgcggatg gacaaggggg ctcatgcctg taatcccagc acttaagaga ggctgaggtg    9300
agaggatcgc ttgaagccag gagttccaga ccagcctggg caacatagac cccgtatggc    9360
cgtggtagcg agtggctgtg gtcatagcta ctcgagaggc tgaagcagga ggatggctgg    9420
agcccaggag gtggaggctg cagtgagctg tattcgcacc actgcactcc agcctgggcg    9480
acagagggag accctgtctc agaaaacaag aaaaatggat gtgcaggcaa tctttcgtat    9540
ttggagatta gccgttttac caggaccccg ctcagcacct gaaggcagac atcagcgtgt    9600
gtttaggggc acgaagaggg gaggggtccc atggctgcct cagcctggac aagtggaagc    9660
gtctcagatc ttggtggcct ggctgggctt tggctgtgcc caggagagcc tcttgggagg    9720
```

```
gtatcctctt cacttccctt cctcgttggt gacagcacct atctcccctt agaaagggga    9780
gccgcctgtc ctgctgcgtc cccaagagca gccctgggga aggtggggga gctctgactt    9840
cacccagccg gaccccacca tcgggtgctc ctcaccgctt ctcttccgcc ccagggaggg    9900
cttcgccagc ttcggaggct tctctagggg cgcatggctc tgcaaccggc tcagttgctg    9960
ggctgtgcga gtcccagggg tcgccagggg gcacatcacc gtcagggggg aaagtggcgc   10020
ggagcccatc atgggtgaat cggccgccgc aaccgcatcc cttttccaaa ggcggcggcg   10080
ggggcgaggt ggtcgggtca cttttcccgg aggcctaaag ggcagcgcgc gttttctctc   10140
ctttgggccg cccttccccg ccccgccagc tcccccgttc cccgcggcgc ccggcccctg   10200
gctgcgcaga cccctcttca gcctgaagct gtccgacaca gaggacgtct ttcctcgccg   10260
cgcggggccg ctcgaggtcc cggccgacag ccgcgtgttc gtgcaggtgg ggaccccggg   10320
gacaccaggg gcggatgggg gccggtgggg acgggcgatc cctgatggcg cctccgtccc   10380
ccaggcggcc ttggcccgtc cctccccgcg ctggggcctg gccctgcacc gctgctcagt   10440
gacgccgtcc tcacgcccgg ccccggggcc cgccctggct ctgctgcgtg agggctgccc   10500
cgccgacacc tctgtcgcct tcccgccacc gccgccgccg agcccgggtg ccgcccgccc   10560
cgcgcgtttc agcttccgcc tgcgcccggt cttcaacgcc tcggtgcagt tcctgcactg   10620
ccagctgagc cgctgccgcc gcctccgggg agtccgccgg gcgcctgcgc ctctgacgcc   10680

gccgccgccg ccgccgccat cgcgggtgcg cgggcgcaga gcctggaatc cgggcgctgg   10740
ggcccttcgg gggctgggca cgggcgactc tggcagtgga aagaggatac tctcggggtc   10800
tgaggatctg ggggttgggg gaggtggggc gcaagggtcc atctctgccg acagagtttc   10860
tccggggaca gtgtggacac tgggttcccc catggacagc tctggaatcc ttcagggctg   10920
ggggttggat gcagaaggcc actaagggga cacatttctg ggaacctggt gctgggcctc   10980
acatgctagg tgctgggaga cgcggaccat gcgatcttgt ggggctggga tttggggtcc   11040
ttaggggctg ggcggctagc cacttgtatg cgtaggggct gggctcaggg caccctaagg   11100
ggccggattt ccaggggtcc catcttggct gtggtggaga gctggactca ggtctctctt   11160
gggggccctg gggaccagag ccacgatccc ggtgccctga tgtccccgtc tcttccccac   11220
cttcctctcc cccagtgtct gcctcaggac gaggcgtgcg ccgacactgg cagtggcagc   11280
gccgagggcc tggctgctga cggcccccac ctgcacacgc tgacgcagcc tatcgtggtc   11340
accgtgccgc ggccgccccc cagtgagcac gcagtcctcc tccgcatggg gccgtggggc   11400
ggcagagcgg gtgggaagga cgcctgggag ctggacccag tctcagcgtg gcacttccca   11460
cagggccgcc caagagtgtc cccggccgtg cagtgcgccc tgagcctccc gcgccggccc   11520
ccgcggccct ggaacccgcg ccggtggtgg cgctggtgtt ggcagccttc gtgctgggcg   11580
ccgcgctggc cgccgggctg ggtctcgtct gtgcgcactc aggtaccgac gacctccgcc   11640
aagccgggcc cccagtctaa tcccgcgcgt cgggacccgg ggcggccgcg atcccgcctg   11700
cggggcctga tcaaccctag ctaggcctgc ctccgcgatg cccgcagcag cccccagggg   11760
ccgcccttag cctggcgtgg cgcgcagcag cccttctgca gctcgcctct cccttccagc   11820
gccccacgcc cctggcccgc ccgcgagagc ctcgcccagc ggtccccagc ccaggaggtc   11880
ccagtgagga aggtaggtat ggaggtggag ggagctgggt gaggtaggag atctgacagt   11940
gacgcttcct agcgcttagt tcgtgccagg cactgtggtt tttttgtttg tttgtttgtt   12000
tgtttgtttg ttttttgaga cggagtttcg ctcttgttgc ccaggcggga gtgcaatggc   12060
gcgatctcgg ctcaccgcaa cctctgcctc ccgggttcaa gcgattctcc tacctcagcc   12120
tcctgagtag ctgggattac aggcatgcgc caccacaccc ggctaatttt gtatttttag   12180
tagagacggg gtttctccat gttggtcagg ctggtctcgc acacccgacc tcaggtgatc   12240
ctaccgcttc ggtctcccaa agtgctgaga ttacaggcgt gagccactgc gcccagcctc   12300
caggcactgt tgtaaatgtt ttacgcctat gaactaactt aatcccctta acctctaggc   12360
ggacactatt ataatcccca ttttagagac gagaatgatg aggcattctc tgaattaagt   12420
aacttgtcaa aagtcacaat actgggtgga gcttggattc gaactcaggc agtgggtcac   12480
ccgcggcagg cgcgggcttt gggtcgagcc aggggcatta ggtgaggaga ggcactaggc   12540
gaggagaact cagcattcgg acccggctcc cactctcacc ccggcatgca gaccgatgga   12600
agaggctagt ggtctgctgg gaagggaggt ggcccgcgta gcgtcccctc cctcgctgag   12660
cctcagccac ccctcccagg gatggtgcgc ccccaacatg gtccggagat acacccagct   12720
accaattcgg gaccaggacc aacaggaccg gacccgcctc cctggacctc ggacctgatg   12780
aggccacgac ccctgcgctt ctctcctccc cctgtccctc ccacctgtgc tcaaaataaa   12840
cctctggact gaccggctaa gttctggtgc agtcagtgag cgcgcaacgt gcggggtggg   12900
ggataggtgg ggaggcttgg ggagaaagct gggagcagga cttggggggt attcctgaga   12960
aatcgtgact tacaggggtg gagcctgggg acggggccca tatgcccata tacccgtgtg   13020
cactcccagg ccatgggaga ctgtagggcg ttttgtctca aacagaaagc gacacccggc   13080
cgggcgcggt ggctcccgcc tgtaatccca gcacattggg aggccgaggt gggtggatca   13140
cttgaggtca ggagttccag accagcgtgg ccgacatggt gaaaccccgt ccctactaaa   13200
aaaagtaaca aaaaaagaat tagcagggcg tagtggcgcg tgcttataat cccagctgct   13260
ggggaggctg aggcaggaga atcgcttgaa cccaggaggc agaggttgca gtgagccgag   13320
```

atcgcgccat tgcactgcag cctgggcaac aagagcaaaa atctgtctaa aaaaaaaaaa 13380
aaagcggcac ccaaccccac aagcgtaagt ttaaaataaa ccttccccct ccctcctctc 13440
cttacccgga cacctgccca tctgcctcct gcgatctttc tcaagccagg ccttctccgc 13500
tctccattac ccccgcccgg accctgccct agctccgctt cgaccttctg cctctgctcc 13560
agttccctcc tggtctggac ttcgccctcc tcttagattg tcagtctagt ctagagctcc 13620
ttaaatccct cctgcccgag ctccactcta ccttacacag acaagagtgg gactatcagc 13680
cccagtctac agaggaggaa actgaggctc ggcaaggcta aatcacttgc ccaaagtccc 13740
caggcctgtc tgaccccaga gcccaggctc ttgaccgact ggatcttcgg acttgacctt 13800
tcggccagaa cccacccctc tgcgagaagc aaagcacaca gtagtagctt catgagttag 13860
tccagcaaca gagggagggc atgggatcca aggtgtcgtc tccacttcat gaatgagata 13920
aagtgcaggc tgagggtctc cttcgccccg ccccagttcc ggtgccgatc tccccttcac 13980
ttccattggg gcggttcccc gggcctccaa acgcgaagcc acgcccccac gcgcgtccgc 14040
ctccgcgcgg ttctttctga cctcggcggc ccccgtagct ccgcccatcg gagaagcgac 14100
cttacagcgc ctgcctcttt ctgagcggca tgaagccacc tcccaggcgg cgagcggccc 14160
cggcgcgcta tctgggcgag gtgaccggtc ccgcgacctg gagcgctcgc gagaagcggc 14220
agctagtgcg actcctgcag gcgcggcagg gccagccgga gccggacgcc accgagctgg 14280
cccgggagct gcggggccgg agcgaggctg aggtgagatg cggttctcgg gaccggagcc 14340
aggctggagg cggggctggg gtggggcgga gagtgggcgg ggctgcggca ggagccagcg 14400
gggcggggcc aggaatgtaa gcgagcgggg gcgtggcgtg gggctgagtg gcggggcgtt 14460
agtgggtgag cctgggcggg gtgaggggcg gggcgtggac gggaagagaa gacctagggg 14520
cggggcgagc ttgggagcta gacgtgggcg gggcataagt gaagcggagg ggcggggcga 14580
tgccgggggc ggggcgtgcg tgccgctgag gtttggagag tgggtgggtg agtttgaggg 14640
gcaggacgag cctgggggcg gggcgtgggc gggttaatag caagacttaa gggcggggcc 14700
gttttgggag taaggcgtgg gaggggcgtg ggcgggatga gggcaagctt taaaggcggg 14760
gcgagcctcg gggtaatgcg tgggtgaggc gagggctaaa cgggcggaat gaactgggag 14820
taagatgctg ccgagtccgg gcgaaaagca acgcatgctg ttggacagga tacgagcttg 14880
aagtgagagg gaatagagaa gaggggacga agaagggagt tgaacggggt agtagtcagc 14940
atagggttgg gccctggctg cgtgaaggag ccggaactgg cctgggcctt acaacttcct 15000
taggtagctg gtcacgctct gggtggggta ggggccttgg cagtcactgg gactcctaac 15060
accgggccag caggagacta aggcgcagta gcggggccca gagcatacaa gggattgggc 15120
tttggcttct ctgctgcagc cctgagctca tagaagcctc attccgccgc ctctttcctt 15180
tctagatccg ggtcttcctc cagcagctca agggccgcgt agcccgggag gccattcaga 15240
aagtgcatcc gggtggcctt cagggaccaa ggcgccggga ggcacagccc ccagccccca 15300
tagaggtgag gcagatgaac agggtgaggc tgtccagggc aggtccctgg tgggtaggtg 15360
ggagttgcgg gggataacct gattagggga tccccaggag gagcagggct tggggggcca 15420
ttgcaggctg atccctgggc tgaccctgta cctctggctt cactcaggtc tggacggatc 15480
tggctgagaa gataacaggg ccactggaag aagccctggc agtggctttc tcgcaggtac 15540
cgccattccc ccaggcaggt cagggtgtcc cagaggacag ggtcacatgg gccaaatcat 15600
gtgaacctgg gtcctgggcc cttttgccag ctctgtggac tccgtcagtc actggacacc 15660
tcgagcctca gtgtccctgg ctctgaggcc atctcttgtc tgaggagcat cttagggtgg 15720
cagggaggat gggggaatgt gtagacggtg agaagacttc ccagctcctc ttcctccctg 15780
attgttctgc ctgccaggtg ctcaccatcg cggccacgga accggtcacc ctcctgcact 15840
ccaagccccc caagcccacg caggcccgtg gaaagccttt gctcctgagc gcccctggag 15900
gacaggaaga ccccgcccct gaaataccta gctctgcccc tgctgcacct agctccgcac 15960
ccaggactcc tgaccctgcc cctgagaaac cttctgagtc gtcggctggt ccctccactg 16020
aagaagactt tgctgtggac tttgagaaga tctacaagta cttgtcctct gtctcccgaa 16080
gtggccgcag ccccgagctc tcagcagctg gtgagaaggg tgagggaggg ggcaggagca 16140
gagggatcaa gggtccctgg cagcaggcag gggtgtcccc ttacccctct cctccatcca 16200
tcactagagt ccgctgtggt cctcgacctg ctcatgtcac ttccagagga gctgccactc 16260
ctgccctgca cagccctggt tgagcatatg acggagacgt acctacgcct gacagccccc 16320
cagcccattc ccgctggagg gagcctgggg cctgcagcag aaggggatgg ggctggctcc 16380
aaggcaccag aggagacccc cccagccacc gagaaggccg agcacagcga actgaaatcg 16440
ccttggcaag cagctgggat ctgtcccctg aacccgttcc tggtgcccct ggagcttctg 16500
ggtcgggcag ccacccctgc caggtgaggg gcatggcggg caggaggcca caccaggccc 16560
cccgccctgc ccctcggttc tgctcggctg gccctggctc tttctgagga tcccgtcatg 16620
ggggaaggtc cttgagatga tgctcagctg tggggcgggc ctctaagatg ccccatactt 16680
tgggggtctc agaaatggaa cccccgttgt acaggggttg ggtgggggtt gcaggactcc 16740
actcacaagc ctcctgatgt caaggacagg cggacagggc tggcctcccc cagtccccaa 16800
gccccactgt gccttgttgt ctgctggggg gccatagctg gcactgccca ccgtaaaggc 16860
cctcgcacat tttccccctt cctgtacacc tcggggccag catcctcacc ttcttcaact 16920
gaccagtcgt ggttactccc tgctgccagg tccttcccct tcccgggggt attctgtgac 16980

```
catgaataaa gttatcattc tctttctctt tcacctgtga cttaaagatg gaagtggggg   17040
ccctgaagtg tcagagcttg ggggtcacag ggctgggggt gtgtgccagg gaggagagct   17100
gccacctgac tggtgtgatg aggcctgggg tgaggagccc cttggtgact gtgaagctga   17160
caagtgtgga tggggagagc cggcagggca ccttggggac aggcgtcagg gatgggtttc   17220
agagtagggc aaaatcacat cgcacaggtg gaggggagaa ggcagcagag agtgggggtg   17280
acccggtgga aggagttgag ccccaggctg ggcggttaga ggaagataga taggggtggg   17340
ctgccccgag tggcaggagg ctagtgggag tggagtgtcc cctggggtgg ctttgggacc   17400
tcaaggatcc ctagaaggcg gggagggagg ggtactggag tcagggctga ccccaaaccc   17460
ctgggggtgc tgggaccccg cttgaggccc cctccatcag cttcacctgc cctgtgggtg   17520
tggaccgagg gtcgggtcct agagagggaa gcagatgtcc tcccacggca ccgcgtccca   17580
cgcccagcta gtaatccaag tcggtaaatt ctccgttctc ggagcctgtg cattcaccca   17640
gcctgccttt tatagaaacg ctcattcctc cccccacaca ctgcacacgg ctcccctggg   17700
gtgcacatct cttgggaggg ccttggagca cagccagccc actccgtccg tggtcaccgc   17760
cccaccgcac cagacccaca aacgctgcca atggtggtgg gcagggggag gaggcttagg   17820
ctgcaggtac ggtcggtcgt cggagcctcc ctccagggct ggcagagggt gcgcctccca   17880
gttccccaca gggctgagtc tctctcacca cccccggcgc ctccccagat caacagaatt   17940
gtgtgtggag gggaggcagc tgggggttac acatcagccc ccccccccaa agccggtgaa   18000
aggtgcttgt gatcttggtg tctttgcagc cctgggaagc tttaaaaaca tgcaaaagcc   18060
agacctgggt tccacttcca gccctaccag actctagcca tgtgactgtg ggcaagtcag   18120
tcactggagc tgcctggccc tcggtctcct ggcctgtaga atagggatgc taacagggcc   18180
tggctcattc tcacgatccc gaagaggaga caggctataa acaggaggtg ccaactcagc   18240
acgctacttg gcatgcagta agcgctcaat aatagctttt attatcatgt tcggctcaga   18300
ggcagggagg agtggaacgg catgggaggg ctgcgggagg cacggcaggg gggtcagggg   18360
caagtggcag gagggcggat ggggggcagc ggtgggcacc ggggcagggc gcgctgacct   18420
gtcctggggc ccgggttggg ggcagaaatg agcctgccca cgctgtcccg ccacggcagg   18480
cgccacgcat cctcgacaca gccgccatgg caggccttcg ggctccgtct ccgggacagg   18540
cggtgcaggg caaattggta tgcagcgtcc gccccgtggg cccgggagag cctgccccgc   18600
agggaccaga gcccaaggac gggctcaaca ctcagtcaag gtggggttga cgacggccag   18660
acaacagggg agggaggagg gacaaggggg tccccacttc cagggacgca caatagcaga   18720
gccacttaca cgctggggag ggggcggtgc gggggtcctc ctcccgcccg ggattcagag   18780
tcgggggtgg gggccagccg ggcgggtgag atgcgcagag aggaagggac agggcggata   18840
aaggcacgag gtggggctcc ggccaggcca ggaagggaca tgggaggggt ctcgaggggg   18900
aggggctggg cttctcccaa ctccctcccc ctctcccccg ggctggggc tccggggcgg    18960
gatcctgagc gcagtcctgg ccccgcggcg ccccccgccg ggccggccct ctgagacccc   19020
ggcgcagggc cggctagggg gcgccgcgcc cctcacacca acgcgtagtc gaactgcccg   19080
cgctcgagcg cctccttgtg gtcggtccag gacgaggcgg gcatgcggct gtaggggtcg   19140
agcaggatgc gcacgcagcg caccatcaac agcaccagca ccacgagcag gcagagcacg   19200
aaggcgaaca ccgtgcgctg ctccgcgtcc aggcccgcgc ccaccggggg ccccgtcgac   19260
ggcggcaggg gctccggcga cagcgtccac gtcgcgctca tggctcacat cgccgcgcgc   19320
cctgcggagg aggggacccg ccccgggcgc cggggatgag gctgcgccct ccctcccgcg   19380
cctcctccgc ttcgccccct cctgccgccg ccgccgccgc ctcccttaac gtgcccgacc   19440
ccatcccccg cccgcgcctc ggtccccggg ctacgcccat gcccggcccg ccggatcgcc   19500
gccaccacca cgcggggacc caactctggc cgtgcgcgca ggtgggggcg ctgagagccg   19560
ggaaacggag ccgcgcccgg cctgggccgc acacaacagg tgcgaaagcg cggccgcggc   19620
ccgggcgcgc ggcgtgggga cggtccccgc ggcccctgcc cccgctccct ttgttctcgg   19680
cggcctggga ccccctcccc caccgccccc gccccggcgc gcccctcacc ctggcttcga   19740
cccggacggg gaccgaccgc ccggcggccg cgctctctca gggacccgcg ccggctgcgc   19800
gttcggcctc tccccggcgg cggcggcggc ggcccgggct ccggctcagc ccaccccacc   19860
cgcccctgga gcggcggaga ccgaggcagg caagcagcgc gcgagccggg ccgccgcggc   19920
tgttcctatg gcgacggggg cggggccgcc gcggggggcg gggctgcagg gggcggaggc   19980
tgcgcaggga gcggcgcgcg agccagcgac cccgcccggg cctgcgggag gacggaaccc   20040
cagccccgag gacccgcgag cacccacgac ctcgccgggg agtgggcagc tgccactccc   20100
acctcccctg gcacttcggg ccccccagcc tgccctgtgc ctgcattctc ctccgcgata   20160
gaccccactc gagactccct gtttgtgggg ggacctcagt ggccctaaaa cactcatatg   20220
ggcactcaag ctcccccggc cctggggacc tcaattctca gagagctcag gcaggtcact   20280
gggatgagta agtcccccta gttttgtctg tcactggcct cgtcttccaa ctcagggtcc   20340
agccctgggc aaggcgtcca cccactgcgg ctgcagttcc tcactcgacc ttgaccgcct   20400
cccacagcag ccgcctgctc ccaccccac catcgcctcg gccactcccg cgccagagaa    20460
gacacttcag actccgtagc tgctcttctg caattcggtg ttttattctt tccaaatctc   20520
aggcttatgc acaagatgga agagcactgt taaaattaaa aaatggacct aaagtggctt   20580
ggctgacgtg gctagcgggc cactgagccg cgggtcccgg gtcccaccct gctgtggggg   20640
```

gagtccctgg gccctggggc ctcttggcac tgtgtgacct gtgtgcaccc caggtgacca 20700
ggcgccggga cccctgcagg gcagagcaac agggcagggg ttggccctgc gggggagtgt 20760
ctccagctgc cgcgcacccg caacagcccg ttgtcccctc ccgggccagc tggtcttgca 20820
gccgtcctgg cagagctggg ggcagagccc gcagtcttgt tcccagaggt ctggagttgc 20880
cgcaggtggt gttgcggtgc ctctgtgcct gatgacccag gccggggcta cctggctccg 20940
gcaccacact cagagaatct gctcggtgct ggaggccgag atgtccagga gccgccaggc 21000
cgcgtagggg ttgagctcgt cctggtctcg gcagagcgcc cacacgtaca gcatccgcag 21060
caccttgtcc tgcagggtgg ggtgggaagg gcactgttga gagggttgag gtgacccagg 21120
cagctctggg ggggggccag gctctgtggg gcaggagcca ccggcaactt ccccaggggc 21180
tgggagcaga ggccagcaca attgcctaga ctcaggaacc actcagcctt ggtaaaaaca 21240
gacaggtctg ctggtctccg acctccctca accccaaact ctggcccaga ggtttctcca 21300
catccccacc cagtcccaga gcttcagggc tctgagctgg ggtctctgcc aggtggccac 21360
acacagatct tggggacagg cggtgtcatg tgcctgcccc atcccactcc ctcactcacc 21420
gggtcaccct ccaccacctc gcctttgggg ttcctgacca ccatcaccag ctgtgcctgg 21480
aaggtgatga tcagcaccgg cccctgctcc atcatcttgc ccatggccag ctgcagaggg 21540
gccgagaggg gggatgtgcc tcagaggaca gcccggctgc tcccctggag gtgaaacccc 21600
tgcgcctagg ccctgctagg agaaggctcc agcaccaggc ccagcaccgg tccctccccg 21660
tgggccttgc ctctcaggcc aggaccaggc ctccctcgag accctcccct tggacaagcc 21720
caagacctct tggatcagcc catggccccc agcccctggc aaggccacct cctgggcctt 21780
gtctgggcca tagttcccac cccgatggt ggcccgggcc cccactgcga ggtgcttacg 21840
tcgacgttgt caatgtctag gatgcgagaa tggaactgga gacccagtgc cttggcctgc 21900
tggatggggt gggccagctg gctgtaagtc tgcaatgaga ggccgacacg cctgcgtgat 21960
gccacccagg gtgggcacca cgccacacag agctgctgcc cacacaccct ggcgcccagg 22020
tgccctgccg ccagccagca atggcagagg ccaagaaccc aggtccaagt ggggaggcag 22080
gtgccaccca cttactggcc cagatcacac gcttcccact cctgggaaca acactctatc 22140
cagatgaccc aatgaaaaca ggattaatca gcaaagtggg tgattctctt ggaacaaaat 22200
cccaaatcta ctcccagggc taagctggcg gcccccttggc tgatctttaa tacttccata 22260
ttcgccgcac tctgaatctt ggcctgagat caaacgcctc ttctaggatc tgtcctcagc 22320
ctgaggctgc ctctgtcact gaccagcccc cagtg 22355

<210> 57
<211> 11000
<212> DNA
<213> Homo Sapiens

<400> 57

gaaaggcgag cgctggtcga atcccacctg actccagtca ctctccagga gtggcctggt 60
gcagaccaca acacttgcca cctatgcacc tacgtatttt tccttgaaag gagctgcaga 120
gcctttggaa cttcacttct cagcgaggaa aacccttcag attctcactg cttcttggag 180
ggcgggggta gggctggtga aaatatgaga aagccctttc ggtgcatttc acttgtctcc 240
acgtgcaaat agttgcacgg ctctcccctc attaacttca ttagaaggta tattagcaac 300
aattaaggac gccacaggaa aatgggaagg ccgccccgca gaccgcgcgc cctgcacaat 360
gactcgggga gtgaatgggg gagcacgccc ggccttggtc ctggctgcta caagatgaga 420
acaattagca aaccccttttc caccacctaa tttcgcgtgg taacaaaatg gatttttccc 480
catgaatgcc tggccggcct attcattatc tctcttctat tagtaatttt ctgctccgta 540
ttcagcctcc caactcttca tttcctcccg tctcccagcg tacacagtgc ctcttatgtc 600
aggccgggcc cgtttttatc ttgtaatcat aaaggccacc aaagcaatta tcgccggtct 660
tgactggaaa agctggtcat taattagcct ccttttgcct tcggtgcagc ggattagctg 720
gtgccgggac tgagttaatg gaaacgcggg ttaaatgaga aaagacgccc ggattttcct 780
gcctggatgc gcggctcgga ggaggaccgc gaggttctcc agcggcggga ggcggtggag 840
ccgccgcgga gagggaggga ggctttggag agggagggaa ggaaaggggg agggagaggg 900
aaagggggga gagggagagg aggcgcgggg tgggggaggg gagtgagcag ggagccggga 960
gagggaggag gggcgggaac caggggagcg gcccgaaccc cgtttggtcc ggaccccgca 1020
gccaccgctg ggtctcgccg ccgggtcgcc cttcgcgtgg agatccggtc cgcgccccct 1080
ccccggtctc ctcccctccc ctcccctccg cccccctccc tccggcccac acagcctctt 1140
ccagaaagaa gtcactctag agccgcgcga cccagcccca gagtccgccg gggtccgccc 1200
accgggtctc ctgcgcgccc ctccccgccc ctccccgggc acagcccgtt cacgaaacct 1260
aaggcgccgg ccacgcgcca cctcccccgg gccggggtct cctcggtccc cgcgcgggcg 1320
ctggttctcc cgggtgggcg gcagccccgc cctgtgccct cctgggccgg cgcgagggac 1380
cctagagcgc gcggggggcc ccaggcgggg aggagcggcc gcgaccccgg atgcgagagg 1440

```
cgtgtgggtc caagcggcgc cgggacaggg gccgggtcct ctgcccaccc cagggtccat    1500
gttcgcctgc ggggccgcgt cccccggagc catacctggg cgggagggct tttcccgggg    1560
gcccggcctg cgcccctcgc cgcccctcgc ccctcgcctc tcgcctcccg cgggtcccgc    1620
ctggccttgg ccgacggaac aagaacaaca aaggaagcgg cgcgaccggc ggaggggggcg    1680
cccgccgccc tcccacccct catcccggcc gggccgatgg cccccgcgcg cctccatccg    1740
ccccagcgcg gaggcgaccc cggccccgct gccccggcgt cgggaggaca ccgcgcgctc    1800
acgtggatga ctgcgcccgg gaacagggac ttcccgaacc ttcctgcccc gcgccgtgag    1860
gcccacacgc gcgcggtgac caccctggtt ctgaggggggc ggcggggcca cggcgtgtgg    1920
cggacccggc agtggcaccc aggctgtgcg cgcgggtgtg tggggttccg aggcgcaggg    1980
cgaccgccgg ctccggctcc ccgaggaagg ggcacaggcg ctgcccgcgg tcactcgggc    2040
caggtgaggg caggggacgc gcgggggccg gaggaggagg cctggtctgc gggcggcagg    2100
agggacccgg ggccagccga ggctgttccc agggaggcag acacctgctg tcgccgggac    2160
cctcgacacg ctccgcacgc gcgggagcgg aaccgggcct gctttggagg cctcccttgg    2220
cgcgcttgga tttactcaaa ggtcaaagaa aaatgtcaag gagagcgatt gcctggagag    2280
ctcctggctc tcctcccggg tccccgctgg gtcccaggag tccccggagg ggaagcaagg    2340
agccccagtg ccctggagtc ctgtctccac caacctcgcc cggctccctg ctgcctaggc    2400
ccgcgctgtc ccagcagagg aggggtggga gccgccccag gcccacctcc ccgcgccggt    2460
ccacaaggag gcccctcctg caacctggga gaggccgggg gaggcccgga gggcgaccgc    2520
gcggggccgc gggacctgga cagccgggga gtcgtgaccg cgggcagcgc cggggctgag    2580

ggcgaggagg gtcttactgc agggaagggc gggacgctgg acagagggac cgactggggt    2640
ccaccggcct gacacagaag tcgctggatc ccagggcccg gctgggaaac ccgcttccta    2700
cccgcccgcg ggcgccgcag aagaaaccga agaaagaggt cttccctcca cgcccaggac    2760
ggtttcaagt gtcccaccca gcgctgagca ggagggtgat gcggagggaa aaactgttgg    2820
ctgcaagtca ggtcttagga atcccggaag aaacgaagat ttaaagaatt tttttctaag    2880
tgattgagtt ccaataactt tacaatgact tgactccctt tttgtggagt taaaaaaaat    2940
cttcaaactc ctgctagcca tggcctttaa gagaaagaaa tgagtttaaa agtgagtttg    3000
aggcacagtc ttgacccagg tgtggctttg gtggtcctca gcgccatctc cccatgggc    3060
accgaccccc caaggctgca ccacggcggg tggaccccaa agagagccca gactaattag    3120
aggcatctta tcagtcttga cttgaaattg aaagaaaatt atgtttaaat atttatgact    3180
ttgacattta cagtcaggcg tttttgaaaa gcggaacacc cccttctgta cagcggcagc    3240
tcaatgactg aaagtttgga acagagaaga agtgaggagg aggaggaggg agaaggaggc    3300
ctggtaacac agcggcaggg gtgcgtcccg ggtgggggag atgcacagat aggcacactt    3360
cagtgctgag ttctcagagg caggaatagt ataatttaaa aaaataatca ggaatgatta    3420
agttaatttt atcccaggtt agatctatct tgagaataag atgaagtaca atatcgcata    3480
tttcttcctc tcaaccgtct gcacctgcaa ctcctcaaat ttagaataga cagtattgca    3540
aaagttgtga ttcaaaataa gtgtatgacc acagagacaa tttgtggagc tgtcttagaa    3600
cttagcatct gcagacactg tcctgaccaa tttaaattat tttgaccgtg cccctgagtg    3660
gaggtgcatg aggcttccac cccatgccct tgtgagccat ttacgggaaa atgccttcag    3720
aactttttta gctgtccatg tggtaagctt aaaagaaaag aaaaaaggac atgttatgcc    3780
caaactcaat tgtgagagct gatacagcca cacaaataca aaactttatg gtaaatatgt    3840
gttgacgggt agccagattt agtccttttc caaggttgtg tgatataacc agttaacagg    3900
agcagttcat tctctgagtc acgatgtgca gtattaaaat atgcttgcat cataaagacg    3960
tagggataca ggtgctcggt gccagttaat gttaaacttc taacattgtt gatgctttca    4020
tttgtatcat ttctaaggaa tcagatttca gtcccagcct catcctatct ttgtgtgcat    4080
ttaaattact aaattataaa taccttttttc tcttaaaact tagaagtcag ttttagagac    4140
cattaatgag ttgctgtaac aagtttatct ttggtttgct ggatgaatcc tcttagaatt    4200
attaggaaat ttcattctaa atttactaaa aggcattact aaaatataat actcacttta    4260
gggctgatga acattttaag ttttcaggcc atagcaagtg atccaacttg aaaacacggg    4320
ggaaggggtg tcttaaaata ttttaaaagt attttttaaa gctgttaacg gaactacttt    4380
taatttggaa tttgccacag aattctaata aatgagagaa gtcaggcgga aataatcatg    4440
tattaattct ctgcattaca aaatcttttc agaagctatt atcagagcaa gaggcactgc    4500
ggtctgaatt cagggtggga gaagcccttt gtgcaactga aagctattgt tcctccgtgc    4560
agatgaaatt gtacttgcaa atctgccatt caagtggcca aaaaacccaa aaaagcaaaa    4620
gaaaagaatc ttcaaggaaa aattattctt aaatgtaaac tttaatgaaa ataagttggg    4680
atatactgtt ttaaaatggg ctatttgttg agtttttcca ggcatacact taaaagtgaa    4740
tatacaagaa aggcaataaa ttgtctattt gccatcttta agcactccta aaacatcagt    4800
tttaaagaaa tactgtgttt taagacatca gggaagattt atgatactga tgtccaaagg    4860
gggaagcacc cttgtgcaaa ctaattctaa ccagcagatt gcctccggtg ggagagaggg    4920
acccagaggc tgagcagggg aaggatgccc ctgagaaagc cgcccccagc ctctttcaga    4980
ctctccgctg gcgttttcat gacacccaaa ctcagactca gcggatcaca cttaaggcac    5040
```

```
cggctcctga cccctatttt tggcccgccc gcctggtccc caggcctgcc tggacgcacg 5100
cgcgccttcc acaccaggtg cttctcggga cggggcctgg cctccccacc ccgcaagaac 5160
cagaccctct tcaggactgg gggggttacc gcttcgcctc ctctacccgg actcagaagc 5220
cccaacccaa gacccgccta gcgtcgcttg tcagaacccc tctgtggcac gactttgagc 5280
agagggcgag ggaaaggagg gtcagcgttc ggccccgtcc cctgcctggg ctttctcccc 5340
cgcttccccg tccctccccc ctcgccggtt caccgcatcc ccacaggagt cataaagatc 5400
aggccccagc gccggacaca aaggccaagg gttcccggga gccgcgggta ttcagagcca 5460
ggagaggggt cccggcgcgg agggtgctgc ggcccagcct cctggccaca gagcgcgccg 5520
gccgcgaggg aggaccaagg tccccaacca cgcccgcgtc cacccgtgaa tcccggcgct 5580
cgcagccccc gggcggcgca gccgccaccg ccgccgtctt ctctggagcg cagaggggtg 5640
tgcttgggag ggggcgggag ggcactttcc tctgtgtcgg tgaaaggaag agcttccagc 5700
tccctcctcc gcacaaacct tcacttcctc tcttgttcga tcgcgtccta aaatctggga 5760
tcggcgacgc aaggacagcc tggtttggcg aagacccctc ggacaccctg cgctccggac 5820
tccaggcggg tggctccacg gtgccggccg ggacgcgcag ctcgggggggc gggaccacgt 5880
ccgggacccc ggggccgctg tccgaggacg gaggtcggtg acttgaaccg cgtcgtcctc 5940
agggctgtgg cggggcccct ctcccagccg tccagcccgc agcgcccagc tcagcgcact 6000
gaggaccaaa aagggcggga gactctctcc ggccctaaaa ggcaattgtg ctgagatttt 6060
aactctaact tgggggttcg cccgattccc tggagcaacg cttctctaaa accacggagc 6120
aggccagtgt tcggaactgc acaggtagtt acgaaggaag gaaatgaagt ccctcgttcc 6180
aacagcttcg ggtccagtca caagtttctc tctggcgagc acgcggccgc ctcacctggg 6240
gtgggggagg cgctgagaaa gcgcagttct ctttggggga ggagagatgg cctctcagag 6300
gcgtccccac ctgcctctgg aacgcctctc cctgcaggcc ccctcggttc cctgcaattc 6360
gccgcccgct gggatactcc catggctcgg aggcgcgctc ccggaccctg gtgaggaggg 6420
tgaagagcgc cccaaaatgg gctgagagga aaatgcgccc acactggcgc ctctccatcc 6480
tggctgctcg ccccttccct cctcctttcc cacacgcctc ccatctccgt gcctgcaccc 6540
ccccaccccc agcaccctgc aatggtcgga actgcaggga gtggggagca acgcgaaagg 6600
ccgagagaag agcgctgggg agaagtcccg gaggtcctag actccttggg gcggtctctg 6660
cggggcgctg ggccaggaca gctgccaacc ctccaaatct gtgcgctcag aaggagctgc 6720
cctttgcccg ctcctcctca caggcccacc ctagacccac ttggggatga tcccaggcgc 6780
agctctcccc tgccggccca agcctttgac aggagtcctg cggcgaggca gagacccccct 6840
gaactgagct ctccgacgcc ctagttgccc gttagagccc ccatgcgcgc cctccagcct 6900
tcgaaagctc tcttcctcgc cccaccacct acgcgcctgg tgatgctctc gtgggccctt 6960
cctgctccct ccgcccccat cccagcctct ctcgcctctt ccagggccca cccaaccgca 7020
ctacgagcgc ccgcgccccg cccggcaccc ctggttacct gcagctgggc cggcgcccag 7080
cgcccgcgcg ggaggaggtg ggggcgtgcg ccgggccgag cggcctcggg gcgcagcagc 7140
tgcgggtagc ggcagcgcca tcatcacagc cagctgcttc cctgcgctgg agccacagac 7200
tagcggggtc atccgcttct gaccacaaac ttctagggcg gccggaggag acaaaggtgc 7260
cgcgcgtcgc tgctccagca gcagcggctg cgcggcggca cgggctcgga gcccgcaggc 7320
gccggagctc tgcgcggccg ctcggcccag ctccgcgtgc tccgccccgg cccggccccc 7380
gggctaggca cgctgggccg gggcgcggcg cgggcgggct cagggcgggc tccggacggt 7440
cccggtaggg ccgccggccg cgcgcgtagc accaaccagc gcggccgccg cgccccgcct 7500
tatatccggc ccggcgcgca cccggccggc ggcggcgcgg ctcgcggccc agccaccggt 7560
tgctatagcg atgtccccccc cccccccac atgttgctga cagcaattgg ctaaggggcc 7620
gcggctctcc aaacaccctc tcccgcccga gactatgaat ggacatcagc tttgtcagtc 7680
attggtacaa cgccgaagcc agggccttcg ccttttcttt cttttcagtg tttctccctt 7740
tcatcgttca tactgggcaa gttcagggta gcaccgaggg gagagagacc tcggccagaa 7800
aagtcaaggc gcgcggctct gctgtgacct ttgtcctggg aggggtgggt ggggtgcgtc 7860
gccactcttg gcaagagaaa attgcacggc atatcgttct tcccagttac cttttccagt 7920
gttgccgttc gggccttttt gctccctttc caaatggaag caggccaatg attcagggcg 7980
agtagacgag agcccctctg ctcactttac cagggtcctc ccgagtgttt aggtcccgat 8040
gcgcgtagaa agtgtcttgc gagacgcgga actggtgaga ctttgccacc atttggtcca 8100
ggccaatgtc ggccgccagc ccaaaacgcc gaagggagac ggcggtgaaa acccaggcgt 8160
gcgagctgcg tccttgccct tccccctacc caggtcaatg gtctggccta gaccaccgct 8220
ctgcgggcga aaggagtgga gcagaatcac aggccacgct cccagtcgtt cgagcctgcc 8280
cacataaccc ccggcccctg gcgcagtcag gcccgcagcg ggcgagaaga gctagggtct 8340
cggggcgact aggaccactg agcacctccc ccccgcgcct cccgcccatg ctctctctcc 8400
ccaggccctg gaccccgggc cacagccaca ggttggggca ccccggtgcg cggcgccccc 8460
gcggagctgg cgagagccgg agagctgaaa cccaacatct gtgtgtacaa tgcgctctcc 8520
gaggcccaca gctgcaggaa caaggagaag aaaggaagag gggggcgcag atcttgtcac 8580
acgcagatga cagtctgcgg agggctcttt agtttctcct tttctggaaa ccccagcggc 8640
cccctcctag gcccccctct cccaccgcct ggccattgtt ccctgcgccc gggacagctg 8700
```

gagacggcgg cccgcgccac tcgacgcccc tcccgccgca cacccggctg cccgcggcgc 8760
ccgctcccgg cccctccggg gccaactcct ccacaccccc cacctcccca ggccaggccc 8820
aggcggcgct tccacagtct cccctcccct cccttgcact ctggcgctgc ccctagacct 8880
gctcggtgca gagggccttg cctggcgctt ttctctcaca aacctagttc tgtaaaacct 8940
gggggcgagc cccagggggc agggctcaga ggcgggggtt gtccacagat atgagtacgg 9000
tgccgtccgc tgcaggcccc tgtctaaaag tggccgaagg accccgtttc ctctgactgc 9060
aggcccaggc tgagttccgc atttattttt gtatttatag ggcaaggcca gagcgcttca 9120
taaagctcca gggcaaagaa gtggcctcgc atcgggccat ggttcccgca ggcctgagcg 9180
ggctgcagga ggcccggtgc ggctcggcgg gggcgcgaga ggcgagaaac tgcggcgtca 9240
ctggcgtcct ctgcccatga gtgaacccct ccagtaggcg gggacggggt acatcacctt 9300
ggccctctcg tcccccctgc ccagggcaag gcccgcaaaa acccccgcac cctggctcgg 9360
gggcggcgcg gcggcgggca ggtcagagag aagcggtact cgggttcccc cagcgcccgg 9420
gatgccctcc cagggacctt ctcgggcaaa gagtcgagtg gtagcggttc ctgtccctcc 9480
ccgccaccct cctccggtgc ctcggcgtcc ccagccaggc tccagggcga ccccaggccc 9540
ggccctgggc agagacagag gaggggaggg gaggtggcgc tgggggggcgg ggggccggag 9600
acggcgggag gaggactcgg gagccggggc gggaagaggg gaagacagcg gagagaaggg 9660
acggggaggg gagggggctc ggaggagggg acctgctgcc ctcagcctgg ctggtaaccg 9720
gcctctccat agcaacggcc agcgcgcgcg tctgtgtgtg cgcgcgtgtc tgatgtgtgt 9780
gtgcccgtgg tgttcccggg actccccgcg gggctggga ggggatcgca gaaccctagg 9840
gtggcgacag agcaaacccc tccaggactg gaggcccgac ctccgcggtg ctgggagcgc 9900
cctagggtct ggctgcgccg cgctggcgac ggggtaggct cggggaacgc gcgcaggccc 9960
cgggatcggc cgctggggca ttggggcatc ctgccctcgc tgccccgacc ctcccgctgc 10020
ggtcgcaggg ccttggggca ccggggagtg ggcgggtctt cccagcgacc cggagcacag 10080
gggcgggcga cggcgccccc accatggccc tgcccggccc gagcgcccgg cgcacctgag 10140
gtctgggccg gggagcagct ggggaaccgc gcgtcgcctc ggggagctcc cggccaggcc 10200
ggctccgggc agaggccaga ggggccctgg cgcaccctcc ccgcccgcgg cgacctcgca 10260
ccaccttccc cgccagcccg cggctctcct cgccctggat cgccggagcc tcatccaggc 10320
tggggtctcc agcaggcggc cgggagctct ccgaggtggc caggagtggc cggtagccgt 10380
ctccctcggc caggccgtgt ccagcccctc agtctccacg gcggcgcaga ccgcacgcgg 10440
ccaccctggg cctcggacag ggctaggggg acgcagaccc tgccgtctgg gggtcctggg 10500
gcggccacgg ggccggcgac cccagagcaa ggcggagcct agtacaggcc agagagctgg 10560
ttttgctttc ttcacagaca tgacctgaca gtgaatacac aaataaatac aattcttttc 10620
gttagtccct tcttccgttg tctcagtgtt tcacttccaa catcaaagca ccgcattcct 10680
gatttgatct taatttaatg acctgtcttt ctagtgcttt gaggaaagat gggtgggagg 10740
tggcaccaat tcagttcaat ttgggtcact ttaaaaatcg tgagagggcc tgaaaggttc 10800
catgcgggtt caccagcaga acagcacact tcctcgtcca cgccttggtg cacctgcggc 10860
aatatattct gggtggcaga cggttattta tgtctgagag cctccaactc actccccttc 10920
ctcccaccac ccctcctcta aggtgcggcc tgacctcagt ggggctgcag aactggcctc 10980
gccaaagctg aaggcggacc 11000

<210> 58
<211> 1743
<212> DNA
<213> Homo Sapiens

<400> 58

ctgcttctta aatcccgccg taactgtgaa tgtttgcgat gccaccacat ccccagtaca 60
agagaactgg agcatctctg gctccttcca taatcgaagg gcttccagcg tagggcgtag 120
ggggagaggc ccgaagccct ggagacagcg cccgagggat ggaggccgcg ctcggctgcg 180
ctcaggtccg ggtggctccg ggagctgaga ccccgcagga tgcagcgggg gggggccagg 240
gcctgatccc ctacccctgc gagccagggc cgcctacgcc cccactgctt cccacaggta 300
acgtccagcc ctctcctccc ctatcccccg aaatatcccg gttccgctcc gctccgctcg 360
ccgcccgtga ccccgacccc gacccgtccg cgacggcgcc ctccccagga ggcagcggag 420
gactagggcc aggccgccca ccctggcggc ggcggcggct cccggccaag gcggaccctg 480
gggaggacag cagctgggcc gcggcctgtc cctaggggga gcgggcgcag ggccgcgagt 540
gggcagcgcc tccgccccgc acgaacaaag cggccaggcg cgccgggccg aggcagcacc 600
gcctctccgg gagcgcgggg ggcggggagg atggcggggg tactcacctg agggccggcg 660
gagggcgcgg gcgggacagg cccaggagac gggaccgcgg cgggccgggg ctccgcgccg 720
agggcgcacg ggctacgggg aaggggcgac cggcgcgacg tccgcggcgg ggcccgggag 780
ccccgcgccg cgggccgggg gccggggccc gagctcgatt ctgcggccgc gaggtatggg 840

aaccggtccg ccgggccagg cagcggtgct aggcagcagg ggcagcgacg ccgccgcctt 900
tcccttctcc tgcccccgtc cctgctccgc cgcgtcctcg tcctcgctgg gtccccgccg 960
ccgccgcctc agcctcggcg ctcctcgggt ttcttctctc catcaaggcc gggccgaccc 1020
gcagggacca tcccggaaag tgaggggttg ttgccgtttc ccgcagctgt tggtggccat 1080
ctttaatcct cctcctcctc ctgctttctc cacctcccgc tggctgtctg actgactgac 1140
tggatcctcc tctttcccct cctgctcctc ctactgagcc ggccgcagaa attgcagccg 1200
ctcagcttct accccctcct gcctttcctt cctctttcct tacttccttc ccttccctcg 1260
gcttcccgct cttgcctcac tctcagcggc tgccttcgcc cctgtctgca gacagcgccg 1320
ctggatgctc ccagctggac ttcaacccca ctcctctcag tccctctccc cactgccttc 1380
cagacgcgcc tcttccccgc cccgcgcccc tctctcctct cccacccctg cccctctccg 1440
cggcgctcac cctcctcagt cccagtttct gaaaggactc agctgagaaa ggacaactgg 1500
gttccgcttt ccttaaccct acacccttta gctggatgct gtcagaggcg atggagaaac 1560
gcaaaggtgc gttaatttta ctctagccag agtataaaag aactgcccac gccagctatt 1620
gtactacgca gcttcgaaac tcctgcacgc tctcctgtgt ggctcccgtc cgtgatggga 1680
ctgtagttga ggagctcggt ctcaaatggt gccttccgtc agtgaccttg agtcggttgc 1740
cta 1743

<210> 59
<211> 10196
<212> DNA
<213> Homo Sapiens

<400> 59

aagtgcacgc ttaccgagga ggcagtcaca gtcacaacca cattgtcaca gtcggcgcac 60
acctgtaccg ggtcacagtc acgatcacac attaaccaca gccacacaca cagatagaaa 120
catgcagtta ctcgcaggta tccccacctg aactctgccg cacacagtca cacagcatat 180
agtcgcacac cactacgggg acaaacgcag taacactcaa ccaggcacaa gctccccaca 240
gccgcacaca caatgccgca cgtggtcctg ctccctctca cacacacacc agcagccaca 300
gtctccgagg ccgtgctgca cacggtgtga gcgttggaaa cacactcatc acaagggcag 360
cctgtcacaa tcgcgcgatc attcaccaca cacctccaca cagacgcaag cgcacgcttt 420
caatctcacc tccaccgccg cacaaagccg aggcttggcc cccggccccg ccctctcccg 480
ggtttctcac ctcaggcctt accagctttc gaagcagcag aaccctcccc ggcactgggg 540
tagttgctgg taaagccctg agggtcccgc cagcgcgtga gttggaagct gaattaccgc 600
gagaagtaca gtcttgccga ggagacctct gggaagcgta gtcagagacg tatcaaggcc 660
ggacaagatg gttcccgatc gtcagaattc ctgtccgcgc cttgcgtcag ccccgccgaa 720
aggatcgccc cccctagtca aaccgctcgc gccccagagg cctcatggag tcccgcctca 780
ggctcgggcc ttggccccca aagaaagcca ctccgacgtg tttgagatgg gcttagttcc 840
cggacccgtc ctactgcccc caggcgctgg acagcctcag aaagaggaca agtctgcatg 900
gaaagataat cgcacaactg agccttctgg gagatgtagt tcggagctgg gaaggcggtt 960
ggaccctccg aaagtcatct taattgctct cacagataag ccggtgctgg gctgccggcc 1020
tgagtactgt gcgcccagag tgcgcaggcg caaacatcct gggttgctgt tttggcctct 1080
ttgttcgggc gtgaattttg tttattgctc cttagtgcag aggggttatg ggaccagagg 1140
ttgtgagact cagaagtggt tggggacgca gaaggggtgt agaccaggaa cctgcgacct 1200
gatgggggga ggtcgaaggg aaaggaatcg ccaaacgtgg gacaggtttg gagaggcaga 1260
aagagaggag tctgaaattg caaagggcag gtgttattct gacttctgtg tgctttgtga 1320
taaatgagtg gggcaggaag taagaggagt ttcactttct ccttctgcaa ttatttgtgt 1380
acttggacta agtgtatctc atggccagta tttgactgct tttgacctat aaaggtattg 1440
tatacacagt catgcatagt ttaataataa ggatatatgc tctccccacg gtctccctct 1500
ccctctcttt ccacggtctc cctctgatgc cgagccaaag ctggactgta ctgctgccct 1560
ctcggctcac tgcaacctcc ctgcctgatt ctcctgcctc agcctgccga gtgcctgcga 1620
ttgcaggcgc gcgccgccac gcctgactgg ttttcgtatt tttttggtct ccaccaaaca 1680
cagggtttcg ctgtgttggc cgggctggtc tccagctcct aaccgcgagt ggtccgccag 1740
cctcggcctc ccgaggtgcc gggattgcag acggagtctt gttcactcag tgctcaatgg 1800
tgcccaggct ggagtgcagt ggcgtgatct cggctcgcta caacctcctc ccagccgcct 1860
gctttggccc cccaaagagc cgagattgca gcctctgccc cgccgccacc ccatctggga 1920
agtgaggagc ttctctgcct ggccgcccat cgtctgggat gtgaggagcc cctctgcctg 1980
gctgcccagt ctggaaagtg aggagcgtct ctgccaggcc gcccatcgtc tgggatgtgg 2040
ggagcgcctc tgccccgccg ccccgtctgg gatgtgagga acgcctctgc ctggccgcga 2100
ccccgtctgg gaggtgagga gcgtctctgc ccggccgccc cgtctgagaa gtgagaccct 2160
ctgcctggca accgccccgt ctgagaagtg aggagcccct ccgcccggca gccgccccgt 2220

```
ctgagaagtg aggagcccct ccgtccggca gccaccccgt ctgggaagtg aggagcgtct    2280
ccgcccggca gccaccccat ccgggaggga ggtgggggtc acccccgcca ggccagccgc    2340
cccgtccggg agggaggtgg gggggtcagc cccccgcccg gccagccgcc ccgttcggga    2400
gggaggtggg ggggtcagtc ccccgcccgg ccagccgccc cgtccgggag gcgaggggcg    2460
cctctgcctg gccaccccta ctgggaattg aggagcccct ctgcccggcc agccgccccg    2520
tccgggaggg aggtgggggg gggtcagccc cccgcccggc cagcctctcc gtccgggagg    2580
tgaggggcgc ctctgcccgg ccgcccctac tgggaagtga ggagcccctc tgcctggcca    2640
cgaccccatc tgggaggtgt acccaacagc tcattgagaa cgggccatga tgacaatggc    2700
ggtgttgtgg aatagaaagg cgggaaaggt ggggaaaaga ttgagaaatc ggatggttgc    2760
cgtgtctgtg tagaaagacg tagatatggg agtcttttca ttttgttctg tactaagaaa    2820
aattcttctg ccttgggatc ctgttgatct gtgaccttac cccaaccct gtgctctctg    2880
aaacatgtgc tgtgtccact cagggttaaa tggattaagg gcggtgcaag atgtgctttg    2940
ttaaacagat gcttgaaggc agcatgctcg ttaagagtca tcaccactcc ctaatcttaa    3000
gtacccaggg acacaaacac tgccgaaggc cgcagggtcc tctgcctagg aaaaccagag    3060
acctttgttc acttgtttat ctgctgacat tccctccact atcgtcctat gaccctgcca    3120
aatccccctc tgcaagaaac acccaagaat gatcaattaa aaaaaaataa ataaataaat    3180
aataaggata tattctggcc gggtgcggtg gttcacgcct gaaatcccag cactttggga    3240
ggctgagggg tttgagacca gcctgggcaa catggtgtaa accccgtcta tactaaaaat    3300
gcaaaaatta gccgggcatg gtggtgcgcg cctgtagtcc cagctactcg ggaggctgag    3360
gcaggagaat cgcttgaacc caggaggcag aggttgcagt gagccaagat tgcaccactg    3420
cactccagcc tgggcgacag agtgagactc ggtctcaaaa aaaaaaagga tacattctga    3480
gaaatgcatt gttgggcaat ttcatcatta tggagtgtac tgacacaaac ctagatggta    3540
tagctgactg aacacttagg ctacatggta taacctgttg ctcctaaact acaaagctat    3600
acagtatgtt actgtactga atacagttgg aacactgtag ggaccagccc tacaaggtct    3660
gtgggttttt ctccctgtgt gcagagacta gaggtggtag aaataaagac acaagacaaa    3720
gagataaaag acacctgggc ccgggtgagc cactaccacc aagacacaga gactgatagt    3780
ggccccgaat gccaggctgc actgttattt attggataca agacaagggg ggcagggtaa    3840
ggagtgtgag ccatctccaa tgataggtaa ggtcacgtgg gtcacgtgtc cactggacag    3900
ggggcccttc cctgtttggc agctgaagcg gagagagaga gaagacagct tacgcattat    3960
ttctgcatat cagagacttt tagtactttc actaatttg ttactgctat ctagaaggca    4020
gggccaggtg tacaggatgg aacatgaaag cggaccagta gtgtgaccac tgaagcacag    4080
catcacagga agacggttag acctccagat aactgcgggc gggcctgact catgtcaggc    4140
cctccacaag acatggtgga gtagagtctt ctctaaactc ccccggggaa agggagactc    4200
cctttgctgg tctgctaagt agtgggtgct tttccttggc actgacgcta ctgctagacc    4260
acaatccgct tggtaacagg cgtcttccca gacgctaacg ttccgctaga ccaaggagcc    4320
ctctggtggc cctgtccggg cataacagag gctcacacac ttgtcttctg gtcacttctc    4380
accatgtccc ttcagctcat acctctgtat ggcctggttt ttcctaggtt atgattgtag    4440
agtgaagatt attataatat ttgaataaag agtaattact acaaactaat gattagtgat    4500
acttgtgtat aatcatatct gtgatctgta tctagtgtaa ttcttgttat tttatatatt    4560
ttattatact gaaacagccc atgccctcgg tctcttgcct cggcacctgg gtggcttgct    4620
gcccacagaa cacaatggta agtatttatg tatctaaaca tatctaaaca cacaaaaggt    4680
acagtaaaaa aaacagcatg aaaagtaaaa aatggtacac ttgtataggg tacttaccaa    4740
gaatggaggc tgcaggactg gaagttgctc tggagagtcg atgagaggtg agtgaatgtg    4800
aaggctagga cattacagta caacactcta gactttataa acattgtaca cttaagcaac    4860
actacatgta tttaaagttt ttcttccata ataaattaac ccttgcttgc tgtaactttt    4920
ctactttata aatgttcaaa tttttaaaag ctttttgact tttctgtaat atcgcttagc    4980
ttaaaacaca aatacattgc acagcccatg tgggatccgt atctcttggt aaggaaatgt    5040
tgcaggtcag atgtcattct tatgtggcca catagaggta gaaagactgc atatatgata    5100
ctgcctttcc caaccacggg ctcgtattct aacataccgc aacaatgccc aatatttatg    5160
gggtcataga aaataggatt cactatggtt ggagaatttc cagagccaat ttaagttcag    5220
gaatgctggc aatgagaatg atcagtagga atcagtgtaa tgatttacat agttgaaact    5280
atcaactggg ccaggctggt ggctcacgcc tataatccca gcactttgtg aggctgaggt    5340
gggctgatca cctgaggtca ggagtttgag actagcctgg gcaacatggt gaaaccccgt    5400
ctctactaaa aatacaaaaa ttagctaggc atggtggcac acacctgtga taccagctac    5460
tcgggaggcc aaggcaggag aactgcttga acttgggagg cagaggttgc agccagccaa    5520
gatcatgcca ctgcattcaa gcttgggtga cagagcaaag actccatctc aaaaaaaaag    5580
ttaaaataga ataaaacata tagccattat ttttctgttt ggggcaattt taaagttttt    5640
cttttgtcac aaaaacagga atgtactatg caaaggcttg aaataggcca tctttttttt    5700
tttttttttt gagttggagt ttcgctcttg ttgcccaggc aggctggagt gcaatggcat    5760
gatctcggct caccacaacc tccatctcct gggttcaagc aattctcctg cctcagcctc    5820
ccaagtagct gggattacag gcatgcacta ccacgcgtgg ctaattttgg gtttctagta    5880
```

```
gagacgggat ttctccatgt tggtcaggct ggtctcgaac tcccgatctc aggtgatctg    5940
cctgcctcgg tctgtcaaag tgctgggatt acaggcatga gccactgtgc ctggaaaata    6000
ggccatcttt ttaaacaaaa aggcaatgat tcacaaaagt ctatgaatag aacatgtaac    6060
tagttgatac aaatctaata ggatttgttt aaaatcagtc aaatctaata catctgaagt    6120
gttcttgtat aaagtatcac atgaagaaaa gaagaattta ttaatgtctt aaaaaaatgg    6180
gtttcttcat agacaatatg acaacttacc attaaaagtg tttcgggacg gccgggtgca    6240
gtggcttacg cctgtaatcc cagcactttg ggaggccgag gcgggtggat cacgaggtca    6300
ggagatcgag accatcctgg ctaacacagt gaaaccccat ctctactaaa aatacaaaaa    6360
attagccggg catggtggcg ggtgcctgta gtcccagcta ctcgggaggc tgaggcagga    6420
gaatggcgtg aacctgggag gcagggcctg cagtgagccg agatcgtgcc actgcactcc    6480
agcccgggcg acagagcaag actccatctc aaaaaaaaaa aaaaaaaaaa agtgtttcag    6540
gagacataag gaaatgcaac attattcttc ctgaacactt ttagttcaag actttccact    6600
caataaaata ggaaaggatc tgaaactgag aaaatgtatt tgagtacaaa cagcttgtga    6660
aacataatac tttttttttgc atcatcagag ggttttactg aacttataac caacttgccc    6720
actcagtatg caattcagat gtgagtgagg ggccgggcgc agtggctcac tcctgtaatc    6780
acagcacttt ggaaggcaga ggcgggcgag tcacctgagg tcaggagttc gagaccagtc    6840
tgaccaacat ggtggaacct agtctctacc aaaaatacaa aaattagctg ggcgtggtgg    6900
tgtacacttg taatcctagg tactccggag gctgaggcag gagaatcgct tgaacctggg    6960
aggcagagat tgcagtgagt ccagatctcg ccattgcact ccagcctggg tgacagagca    7020
agactccatc tcaaaacaaa acaaaacaaa aaaaacagat gtgagaggac agaggtgtct    7080
cacaggagcc tgtactgtct tcaatcctat gtgtgcaggt atctaccaca ggcaaacagt    7140
ttctccacat tttgtagtta tatgatcttg ctactagcaa aaagaggtaa acagcccctg    7200
tagaatgaag ggactacagt cacgggacta gagtcacaga atgaagcttt cctcttaata    7260
tttttttaatt tgatgtttga actctttttc tttttccttt gagacagtct tgctctttcg    7320
cccaggctag agtgcagtgg cacgatcttg gcttactgcc acctcctgtg ttcaaacaat    7380
tctcctgcct cagcctccgg agtagctggg attacaggcg ctcgccacca cacccagcta    7440
atttttgtat ttttagtaga gacggggttt caccatgttg gccaggctgg tctcaaactc    7500
cttacctcag gtgatccacc cgcctcgggc tcccaagtgc tgggattaca ggcatgagcc    7560
actgcgcatg gcccgatgtt tgaactctca atgcagcatc ctagagaagg gtgttcggga    7620
cctcctgtgc ccaagggacc gataaagaaa aaagctccat ttattatttg ggatttgatg    7680
cacagatgaa aaacttaaca cacaataacg gaagtcggtt gttagtaaat cacatcctag    7740
tctttcagca cttccataag agacgacatc ttcagttgtc tagttcttgt agttttagca    7800
cggcaacatc aatgatgcct atgtccagaa tcagttaaaa agaccataat ttgtttctct    7860
tagttcatct atttttcact ggctcatggt cccaagtgta tctgaatgat tacctttggg    7920
cattctctgc tattgcttat tagggtgctg tcgattgtcc ccatgttttg tgggctggtt    7980
gggaagggga gcttgggaag gatgtgtcac tctcaggagg ttgtaagtca ttgggacgcc    8040
tccagggatg attccttcca tggctgcagg aagtcctcct ggagccacgc ccacgatgcc    8100
tggcggatat ctgtatgttg caccgttgag ctcgggatga attgcttgct ggtctattgc    8160
tgaccaaggc gctgatgtga caaataactc cttgttcaca cagtttctta agctttctgg    8220
gacgcgacct gtgatgactc ggcggatctc ggtggcagct gcctccttca tctccagtga    8280
cgcctgcatg ctgtcctagg cagtgtgagg agtgaagatg agatttggcg catctttcaa    8340
cggagtctga gcaaagctaa agggctccga ttcgtgcaag ccaagggctg cccctcctat    8400
cctgtcctcc ttgaggacct gtgctaaggc tttctcatcc accaggccac catgggctgc    8460
gttcacaagg aatgctccct gtctcatctg ctttatagta aagtcattga cgaggtggtg    8520
gttatgttca ttgagattgc agtgcaacga gacacagtca ctctgataca gcaaaccctg    8580
cagggtgtat cagggtcccc tctgcatgcc ctgggacctc tctatcttgt cctacaagta    8640
ggggtcataa aatacgacgc tgaatccaaa ggccttggct caaactgcaa ccgcctgcct    8700
catgcaaccg aagcccatga ggcctagcgt cttccacgaa tgagggccac tcccatggcc    8760
acctcgagaa tctgctccac gctctgaacc cgcgcgcctt cccacagtgt ctggtacagc    8820
cacatattcc tccggcagag attgaggatg tggcagatag tggagttggc tgcctcttcc    8880

acggctgtgg aggggatgtt gcacatagta atccggagct cactggcagc cttgatgtcc    8940
acgttgtcgt agccacttgc ctatccacac gatcactctc agggccttga acttctccag    9000
gtcctccctt gtttagaatc ttctcgtgga tctcctgcgt ggactgtgcg tcacagaagg    9060
ccacagtggc caggtccttc aggatgggca tctccacggt gcaggtcaca gccatccagc    9120
ggcgccagca gcgggagggg gtgcaggggg ctgttcgtga tctgagggtg gatagcttca    9180
caagttctgt ccaatcgctg tctcgtgatt ttgtgcttat ccaccagggc cattctttat    9240
ggaactttgc aactctcaga tcaaaaggca aagcaggcct ggcatggtgc ctcatgcctg    9300
taatcccagc acttgggagg ctgaggcagg cagatcactt gaggtcagga gtttgagacc    9360
agcctggcca acatggggaa acaccatctt tactaaaaat ataaaaatta gctgggcatg    9420
gtggcgcgct cctgtaatcc cagctactcg ggaggctgag gcaggagaat cacttgaacc    9480
```

cgggagtcag aggttgctgt gagctgagat catgccactg cactccagcc tgagtggcag 9540
agtgagactc tgtctccaaa aaccaaacca aaacaaacaa caacaacaaa acaaaaggca 9600
aagcagtcct ctaaaaactt acggaaactc gcaggagtgt gtgtgcatga cgccactatg 9660
aacccaatac atatctgtcc acaaactcta tagttcacag gatgggctgt ccatcttttc 9720
tttctttctt tctttctttt tgtcgccagg ctggagtgca gtggcgcgat ctcagctcac 9780
tgcaatctcc acctcccggg ttcaaacgat tgtcctgcct cagcctcctg agtagctggg 9840
attacaggtg cacaccacca cacctggcta ttttttgtat tttagtagag acggggtttc 9900
accatgttgg acaggatggt ctcgatcttc tgaactcgtg atccacctgc cttggcctcc 9960
caaagcgctg ggattacagg tgtgagccac tagctgtcca tctttttaag ggaatgcagc 10020
ttcgtcagtt caaaaacatt taaggtgatg aaaccctcgt cctgggcagt cagcatccac 10080
agcggaaagg ctcccgcccc tcccacccca cctggaggct gtgctgcctc agttcagtgc 10140
agcatggctc ctggttcagt ccttcgctcc tcgggccgct cccaagccac cttaaa 10196

<210> 60
<211> 1231
<212> DNA
<213> Homo Sapiens

<400> 60

ccacccaacc ggtgacacag cccggggccc cgccgccccc tggcggccat tacggatttc 60
cgcctccctc acagaaggca gtcactgcaa cgtgcgtggc ctcagttgcg tcatatccgg 120
cccttgcgat cagggcttga ggaacccgcg ccatgaagtg cgtgtttgtt accgtaggga 180
ccaccagctt tgacgacctc attgcgtgtg tgtcggcgcc cgacagtctg caagtgagtg 240
agggaggcga gcaggcggcg gcttggctcg ccacccgcca tctccggcca tactgccagc 300
cgcgttagcc ttgcctgacc gtcgcgctcg gaaagaaacc cccgcaactc taccacaggt 360
gccgctgccc cttagctggc ttctgcccac gcccggcggg gcccttctcc ggctacccgg 420
ccactcgggg ttgcctgttt cctcttccca ttattccggc cgctcctctc cctcatttct 480
tcagctcctt ttccggtctg cccccgcgct ctattctccg cctccgcgtc ctccgcccct 540
ccttccaacg gctccgcccc tccgagctcg ggttttccac cgggctcggc agtttttcct 600
caggcccccc tgtggaccgc acgtcggcgc cggcgtctga gccagccgag ctcgcctcag 660
agcccggctc cttcccctca gcacttggcc ggagcccgcg cggttcctct cctcagcgcg 720
cgtcgtcccc tcagggctcc cggttctcgc agttgatcaa gtgaggcaga acgggtagtg 780
cctgggttcc ccccgagttt gcgactcctt ttccaaagtc ttttcctgct gtggctttcg 840
cgggactctg cgagctgggt cgcttagatt catgagtgct tgggaggttc ctcgtcgggc 900
cccggaccag aaaatagttt tgaaaactaa gtgaaatcgg ccacagatag gctttttggc 960
ggccgtgctc cgatgttaac gtcaaacttt agtagtggtg gtggttcctg ctctgtttta 1020
catagccagt ttaaaaggcc ctcacattct gatttcctct ttcagaaaat cgagagcctt 1080
ggttacaacc gacttatcct gcaaattggt agaggaacgg tggtacctga acccttcagt 1140
actgagtcgt ttactctgga tgtttacagg tacaaggatt ccttgaaaga agacattcag 1200
aaagcagatc ttgttattag tcacgcaggt a 1231

<210> 61
<211> 16425
<212> DNA
<213> Homo Sapiens

<400> 61

ggtctctgtg ccttggcctc tcctggtgcc tcttcctcgt ttgtctcctt tcagcatcac 60
gacccggtgc cctccaccca gcccagtgct gcctgccaga gccccgggaa ccctccaagt 120
tctgcaaaag ccttccctgg ccaggttgca gccaatgccc gcagccagag ggtgggtggg 180
cagggctgat ggtggttcct cagccgccag gcaagagacc cccagccgga gggtgggtga 240
gcagggccga tggtgcttcc tcagccgctg ggtgagagac tcccaggtgc ccaggcctcc 300
tgggagggtg tggctggggc cctctgggcc tggcgcccgg ggagctggcc agtggccatc 360
cactcctgag tccccccagac ctgtgtctac ccggcacacg tgctgtgtcc cagggagagc 420
cgctgcgtcc tggggctctg gtgtcccggc cttgacctgg cttggtctca gccctgggcc 480
tggagcttcc atctctctgg taccagttcc tgcagcgctt tgccccctgg cactccccga 540
gagatgcact ttaaaaacag gatcgattta ttgttggtga tgaggagagg gcggcctgag 600
cgtgaggggg tgggcgtcag ctgccggaga agcttctgcc aggcaggagg gacgagcggg 660
cccccagctc tgacctctcg gcttctgttc atgtttgaaa atgaaatcgg tggtttgggt 720

```
ggtcgtcagt ttcctgtggc cgacagaaga aagcaccaca gctgggcctt caaacaatga     780
gaattcaccc actgagtcct gggagttaga gctctgcagt cgaggggccc gtagggccac     840
gctccctcca ggcttcccag aggggctcct tcctgcctct cccagctcct gttagacaca     900
ggtgtccctt ggctgtggcc tcagcgctcc agtccctgcc tccgtcccca ttcaggtgac     960
cttccctgtg tctgcgcctc ctgttctccc ccttacaagg acacttgctg ctgcgttcag    1020
acaatccagg atcctgaact cgattacata tgcagaaacc ctgttcccaa ataaggacat    1080
acaggttccg ggtcgggag tggacacgtc ttgctggggg ccacgctcag cccctgcctg    1140
taagtgctcc tggattttca ggcgttccca aggtgcagga tgtgagtctg catttgtaga    1200

cagagtccag aaaagtcaga caggccgagg ggtggcgtcc gtgaatcaga aagaggtctg    1260
agggtcgagc gtgggttcag catgcgcccc accaccagcc ttgcaggggt agaggtctag    1320
ggtggggccg gctggcttgt ggaaggtgtg agaagccgtc gtggcaaatt caactcttgg    1380
ccaggagtcg gctcactgtg gtctctgggc caaatcctgc cggaacctgt ttttgtaaat    1440
aaagttttat tggctcgcag ccattcccac acgtgtcatc tgtggctgct ttccagctaa    1500
gtatggtaaa ggagacttta agctcaatca agccaaaagg gtgacgtcgg gctctttaca    1560
ggcaagctcc tgaccccagt cctgactgta ttattgggaa tgtaacttgt tgaacaaagg    1620
aggtgctggt tgggggcaat ggaaggaact ggttgttatc aggtgccgga gctgtctgcc    1680
agggtgtggc caaggtgcca ccaccacaga gcagagccac aggggaggaa aggtccctgc    1740
ctgacttctg ggtgatcagc ccaccaaggc tccgaggaag ctgaggccct cgaggtggaa    1800
ggtggtggat ggttggccag tgtgtgcccc ttgctgagca ctgagggtga gccatgggtc    1860
acctggtccc tctgtgcatg ggcagccaga ttgcagggaa gacgctctgg ccttatagct    1920
ggtaacgcag acagatgtgt acctgaatat gcaagcatgt attgagtgct tactgcacac    1980
agggcctcca caccttcctg cagatggtcc ttacggcatt gctgcaggtg ggggtgccac    2040
tgtccccagg tcacagatga ggaaactgag gccaagatcc caaggccata cagctgggag    2100
gtcagagcag ggattcccag ggccttgccc ccttagggga agggagtgaa ggggtggtct    2160
gtatggtgcg tgattatcct cagtggaagt ttcaacatgg gtgtggctca gctttttagg    2220
gtcccccaag gcagaggtgg cccgggcccc tgcagcgatc tcacaacaca cttcaactgc    2280
agacggtgga gactgcgggc tgtcctccgg ctcaagcggc cctggggccc ccgtcctgcg    2340
cggagccacc cgtgttcatg ctgttcccac acaccgtcat ttcttgggtg ctctggtgac    2400
gcatttgagg gacattgctg ttacggcgaa ttccgattac gccgctgtgt ttgatttctc    2460
tcttggggcc gggcgtcctg cttcctgcct tggagtttcc agaggggggcc aggagattgt    2520
ctggaaagcc tcagctgggc aggcggagtt ctgtaggtgc gggaatgagg gggcttcttt    2580
ctcaaattgg gaatgcctag ctttttttcta gctgtaaacg tttcagacgc tgcggacaga    2640
tcctccaatg ggaagtgaac gcactgtcgc cgcgcccaca ccacgccttg cttttgtgaa    2700
tcaagcttcc tggcatgcag ctgcgcccaa gggtctacac agggtctgag gctgctttca    2760
cgcagcaagg gtggcggtgc tgagtagtgg ccgcagagcc tgtgtggccc tgaaagctgc    2820
atgcttgccc tctggcccct tgcagaggaa gcttgctgac cgctggcccc tgtggtcaca    2880
cagacccccct ggctctgcgg gctgtggcca tgtggcccgg ggaggtgggc acggctgggg    2940
agggaagctg agcaccatgt ctgctctgga ctccattgca aaaatgagtg tctgcgtcaa    3000
attcatttgg gattttggtt ctcacgtggc tttgcagggc ctgtgtgaag agtgagcacc    3060
tgcttttcag agcttcagat gacacgtcat tctgcaatcc tcttcgggga cattctcccc    3120
aatcccctcc tgcatgctca cagggatttg gggtgtttgt atgaggtccc aaaacctaca    3180
ccctcgccga agccgggtat cctggagccc agctgccggg gcctaggtgg gaggaggcct    3240
ggtcatcaag cctggagcca tgccgggagg gccgggagtc ttcctgcgcc gtcctctctg    3300
ctctctcagg cagacctgct gcccccgcca ttcacaggag tgtccccggg ccaggtggtt    3360
tgcccaaggc cacacaggga ctgtgctgtg ccaggtttga gctgcacctg gtggagtaga    3420
cagcaccagc cggttgcctg tggtgacagt gacgttggaa aatgggaagg tgggggtgga    3480
gacggggacc cgagttgccc tggagggtcc gggaggcaga acgcaggcag gtccccagca    3540
gccgaggaag agactagggc ctgctgtgct gggcagtggc cgggtggatg gggatgggac    3600
ccaaggccca tcgacctccc ctaccccctg gggctgaagc tcagccacaa cctgggaact    3660
aaaggaacca ggagggggcgg tgggtctcga ggcagcaggg agtgggcaca ggtgtggctg    3720
gcagggcggc aggataggag cggactggca gaaccagggc aggaggccac agccaccggg    3780
caggccccac gctgggcccc caagcccgtg accacagatc ctgcagccct cagtgtggcc    3840
cagggggctc cagggcctgg gacagggcaa atccgccagt gaaggtccag aacccagtca    3900
tgggagccag cgtcgggggc tgggcacaga catgaatatt ctgagttcat ttccgctctt    3960
gcagcctgtg ggtcttgcaa ggggaggggga tcgccccatt ttgtatgccg ggagtcgaag    4020
ccttggcatg gctgagtggc tggcttgagg tcatgacccc agcccgggtc tctgcactgt    4080
cacggcctcc ccggggacgc caagggtgcg ggcaggcggt gttggctggc gtccctgacg    4140
gccacgctcc ttgcagactg cctgctgatg ctggtctaca aggacaagtc ggagcgtatc    4200
aagggcctgc gggagcgcag cagcctgacg ctagaggaca tctgcgggct ggagcccggc    4260
ctgccctacg agggcttggt ccacacgctg gccattgtct gcctgtccca ggccatcatg    4320
```

```
ctgggctttg acagccacga ggccatgtgt gcgtgggatg cccggatccg ctatgcgctc    4380
ggcgagggtg agtgacgggg gccggggccg ggcgggggct ccccgttcag gtgtgccggg    4440
gcccctcacc aacgtgggct gcagaggtgg gagcggctcc agggaaccgt gcaggaagat    4500
gggacattca tgggtgcctt agggtggact gagctccagc ctgggggtgc ccacgagggt    4560
ttgggagggg gagtctgctg ggctatggtg tctttagagc tccctagcct gaccgttagt    4620
tacaactgga gcctttaacc caagctggtg gcctttggag ggacgtggct tcccaaagcc    4680
ctgccctttg cttcctacac tggacagtgg agcccagagc ctggctagga aggagagccc    4740
caggctgggc ggtcccaccc cagcttcccc ccaaaacagg atgggaggga gggagggaat    4800
agagggaggg gagagggagg ggaagggagt gctcccctgt ggaactggcc ctggaagagg    4860
tggaacgtca gtcccagccc tgcaggggct tcgaatgggt gcctgggggc ctctgggggc    4920
tgagctgccc tggggctgtg agcagaggtg ggtggtgatg gggccctggg cactcagcgg    4980
cagagcaagc ataatcctgc atctcctaag aaaccaaaat cagtgccaaa accccataat    5040
gagcaaaaca gtagaatgtc cactgagttg agtcctggtc ctaccctggc acccacccaa    5100
taatcccagc agccacagct gccctccctt taaaaacttg ctattttagg ctgggcacgg    5160
tggctcatac ctgtaattcc agtacttcgg gaggctgagg tgggtggatc acctgagccc    5220
aggagataga gaccagacca ccctgggcaa cgtggcgaaa cctcgtctct actaaaaata    5280
caaaaatgat ggccaggtgc agtggctcat gcctgtaaac cttgtgcttt gggaggccaa    5340
ggcgggcaga tcacctgagg tcaggacttc gagaccagcc tggccaacat agcgaagccc    5400
catctctact aaaaatggaa aaattagcct ggtgtggtgg tgggcacatg taatcccagc    5460
tactcaggag gctgagacag gagaatcgct tgaacctggg aggcagaggt tgcaatgagc    5520
tgagatcgcg ccattgcact ccagcctggg caacagagta ggactccatc tcaaaaaaaa    5580
aagagctggg aggtggaggt tgcggtgacc tgaggttgtg ccactgccct ctagcctggg    5640
cgacagagtg agaccctgtc tcaaaaaaac aaacacaaaa aaacttgcta ttttgatcag    5700
catggactct gcataacttt actttttaaa atattgatta aaatatcctg gcccttgatg    5760
gctgagttct ttggccttgg aggtgagtgt cccgctcccc cagtcttgtc cccaaggcag    5820
cagcccctcc tgctccattg agtggggaga gagaagctgg gggcttagtg ggaggaggtg    5880
cggttggctc tgcaggaggg cgcaggggaa ggagacatta ggagggaaga actgcatctt    5940
tccagaaagt tctcccgttg gggttccct ccagcctggc tcagcggcat gggtctgctt    6000
ccttcatccg ctggcgcagg gccatggcac agaggccgct gggcaccgcc caggcagctt    6060
catcttgata gaacgtggcc tgacaaggat gcgcctgagg ccacagcttg gagcaggtag    6120
aggaggcaga gaagggggag gtgggatggg aacaggggtc cctttggctc tgggggttgg    6180
agatggagca ggtggggccc ctggtgggga ggctgctgtc tgcctggtgt gctgacctgg    6240
gaagacaggg gaggtgggga cagcattcag gaccactgtc ctctctgagg ctttcagggg    6300
caggggcctg ggcagagcca gtgcacagga ccatgggggt gctcagggtt tgggtgaatg    6360
gccacctgga tggagagagg gccggtggtg ccctgaggtt cccgtggcca ggctggggcc    6420
tcgatacaga ctccagactc tccacacccg aactggaggc tgggggatcc ctgagcctcg    6480
gtttcccctt ctgtgatgga gccacacagc tgtggagaga gagccttcct ggagaggtgc    6540
cgggggtggg agcccagcca gggcactcag ctgccctgtc ctccctagca cctccgtggt    6600
gggaggggag ggaggtggag gtccgaggtc aggcccctct gggacttcct tgcccctgaa    6660
caaagatagt tcctgggagg atggcaagga gagaagtgca gcccccaggc cccctcgtgg    6720
ctgtgaggag tgccatttgc tcatcctgcc gtcgctgcct ccccggctga cacctgggcg    6780
gctgccccca cctgccccag cctggcccct gcccctgctg cctcctcccg agatgccctc    6840
tcaccccacc tgtccacagt ggcccctgtt gcctcctctc atgatgccct ctcgccccgc    6900
ctgaccgtga tgccctctcg ccccgcccac cctcgatgcc ctctcacctc acccgcccat    6960
gatgccctct cgccccgcct gcccgtgatg ccctctcacc ccacccgccc gtgatgccct    7020
ctcaccccac ccgcccgtga tgtcctctca ccctgcccgc ccgtgatgcc ctcttgcccc    7080
gcctgcccgc agtgcatagg ttccatgtga cagtggctcc aggcaccaag ttggagagcg    7140
gcccggctac cctgcacctc tgcaatgatg tcctcgtctt ggccagggac atccccccgg    7200
ctgtcacggg gcagtggaag ctgtctgacc tccggcgcta cggggccgtg ccaagcggat    7260
tcatctttga aggcgggacc aggtgtgggt actgtaagta cggatgtgtg gggtcactgg    7320
gcagcagcag caccccccac ttcccctgag aactgctggc ttcgggccgg ccgaccccac    7380
ttgcaggctg gcctgctggc atttccagaa tgcgccggca ggtggacgga gtctccccac    7440
gctcgatgtc caaggtcctc tgcccgcagg aacctcagtg tggagtgtcg aggaggggcc    7500
tcggctacct ctgctcttcc tcactggagg gagagagttc agggatgcga ggaggggcct    7560
gaggccacag agcctcagag ccgctcatat ccagatttca tggaaaggaa aaaacaaaga    7620
acaaacgcat ttagcagtgg ggagtgtgag ctcccgcata agcaccgcag aaacatattc    7680
ttggaccaag aattcctggt ggacatgcca gccaccccgc ctgtgggccg cccagagctg    7740
ccctaatgtc cccagtgagg tggccaggcc gtgggtgggg atggcctgag tcctctgggc    7800
ctgtgaaggc ctgagagctg tcctcactca gccaggggag tgagggaaat cggagcagga    7860
aggcacccag ccctgcccag agagtctgag gtgcccgccg ggctgtgcca ggagcaggtt    7920
gttccagcaa ccccctccac tttctcccat gttgggtgca gatgcgccag gttgccctgt    7980
```

ggacctgcag tccccgcgga gggtgtgacg cctcctctct tctctctgga ggggcagctg 8040
ctcattggcc gggctgaagg cgggcagagg gacctcctgt gggcctggtt tggggctgag 8100
aggactgagg gactgcactt ggctcacact ggtgggggct ctggtggagc ctggatgtgt 8160
ctgggtggag gaaacgcagg caaggacacg ggccacactg gccgcctggg acctcatcct 8220
gcaaaggcca gctggggcac gggcgcaagc ccatctgggt gccacgttct gccaccttct 8280
gcgctgggcg ctgggagcaa acccagtagg tcttgtggca ggagccagcc ctgttggggc 8340
ctttgaggag acgacatctg acccttgaaa ggggaacagg gcagtgaggc tcaggctcca 8400
cggactgggg gcgtacacag ccagctgcag gaggcacgtg cggcagagct ggcccggagt 8460
ctcggcccct ggggctggca ggggccatag ccagagtcca aagggccttg gcccctctgg 8520
gattggagag ccagtatgga tgggcagggc cgctgcctat gccagctgtg agctggtgcg 8580
gggggtgggg tggggggtgg ttcccgggag agctaggaca cctgtcgggg tcccatctgc 8640
ccccaggccc tccctgcggc aggccagagg ttcagtactg ggcttcggg agccactggg 8700
gagtgggttg gcggtggctg ggcacagtgg gtgggcccag cgtccctgtc ctttgtcgtg 8760
tgggaagcca gagctgcagg ctgtagttgt cctgacacag gcagcctgtc ctgtctgttg 8820
tgtgctaggt ttgggggtgc ctggaggagg gggacccata ccttcctttc tgtctgtgga 8880
attccttggc acgggcaggc agccggccgg ggtggcgccg cgtgctcacc ctctaagccg 8940
gggcacagca ggaggacccg tttccttgga gacctgctta ttttgtctgg gccataatgg 9000
aatggggaca ctggatgttt ttcctctgag gccgtggagg ttcacatccc tgccgaggtg 9060
tgggtggccc cttttccgct ggtaaacaat cccacacctg ggctgtgct tctccccagg 9120
gcgaggctac tgtgccgttt tcctgggctg ctcaaaagct gggggcactc ttgtggggcc 9180
gtctgtcttg ctgctgcctc tccggggcaa gctgttagag cagccccacc cgcaaacctg 9240
caaaccaggc tggccccaca gaacctgaaa acctgcaaac cgggctggcc ccacagaacc 9300
cacaaacctg cagactgggc tggccccaca gaacccgcaa acgggggctg gccccacaga 9360
acccgccttt gcagctcagt gctgaatgtt ctcgtccccg gtggtaccct ggcctgagag 9420
aggaagggct ctgggaattc ggtcaccgac ggctgcacac cccgtagggt aggtgggttc 9480
tggaggccag gctggaaggc ttggccgagc cacccatccc tgtccccagc aggccctgct 9540
gggctgtatc ctcctggaga caggcgtggc caccaccctg ggtggtttag gctgggtctt 9600
agactcggcc aagcggtggt gttctcaacg cagcactgag tgcaggccgt ctggctggag 9660
ggacggggag tctgcggcac gtgtgggcag cctggggcct cctgcgggag ggagccggca 9720
actcgggtgg tccaggggca gcggcatcag cttcctgggg cttctgtagc agatgacccc 9780
caagagaggg ccaagtgtcc agaacgggtt ccttctgcag cctggaggcc agaaacccca 9840
aatcaagggg tcctcagggt tggctttttc caaaagctct tggggaggac ccatgggaag 9900
gtccttcctg ccccttccag ccgctggtgg ccccggcggc ctcggctcgt ggctgtgcca 9960
ctccagtccc tgcgcctgct ggctcctgcg tgagcctcct cttcagtctc ttgcagtctg 10020
tggacttagg gcccacctgg atgacccagg gtgaccccat cccgagacca tcaattccat 10080
ccgcagaggc tcctgttcac aggccctggt atgtgaacac attttgtggg ccaccatttg 10140
gccgacaact ggggccactg ggacggagaa atatccagaa tctgaatccc aataagtctg 10200
tgggtgtcgt cacattcagt ctggaggtca cagctgggct ggagtcgtgg gaaggccctg 10260
gaagttcttg ggtgtaggaa atccacattc cctccttccc cagggacagg tcctagcctg 10320
gcccccgatc cccagaaccg cctcacttgg ccccgccaca gccagctctg cagagagcag 10380
agagggtgac cctggcccaa ggcgatggct gcgcctcact catcctgaga cactgggcgt 10440
ggctgtgcat cacgggggcc ctggtggggg tcgggtggag acaggcctgg ctggtcttgg 10500
tgcctggggg gcagaggtct ctctgtggac agctgcctgt tcagaatggg acaggaggtg 10560
gcttggtgac agcaaccccc gggtccccag tgtggcccaa ttccccgtct ctgttggtca 10620
cgggcctgta gtgtggactc ctggagcttg gcccgagcgc acttggcaca tggcaggcga 10680
cggtctgtgg acagctcagg tctgcaggct ccagcctcac acacgggcaa gtgactgggc 10740
cccgccttcg gggatggact gtgcgtggtg gagagtgatg cagagagggc cactcgccct 10800
gggacaggct cagcttgcag cacagacggt ggtgggcccc agagagggtg agcccaccca 10860
gagcctagga agggagggct ttggaggcgc agggggacgt ttgagctggg ccttgagggt 10920
gaatggaagc ttttggaggt gcccccttttt tccccccctta cctgctctct ccagctgggt 10980
ccagtccaag gaccatatct gcctctttgc ccagcctctg gggccgcccc ggagcatttg 11040
aaactcattt tgacttttaa aaatgattaa gaatgtggcc aggcacagtg gctcatgtct 11100
gtaatcccag cactttggga ggcggaggtg ggaggatcac ctgaggtcag gagttcgaga 11160
tcagcctggt caacatggtg aaaccccatc tctactaaaa atacaaaaat taggcaggtg 11220
tggtagcagg tgcctgtaat cccagctact ccagaggctg aggtgggaaa atcacttgaa 11280
cctgggagtt ggaggttgca gtgagctgag atcccgccat tgcagtccag ccaaggcaac 11340
agagccaggc tctgtctcaa aaaaagaaga gtaaagaatg aaaacctgaa ctaggaggct 11400
cttgctacat gaacgcggca tctgcccagc tctggagagc cccctgccgg ccacctttgg 11460
ccactgcagc ctgcgggggc agcctggaca gagctggctg gaaccctggc ctctgtcagg 11520
cagcagggag cgccttcagc agtttctcat tgccgggcgc tgggcctagg gatcagacgg 11580
tccggcctca gaggctgcgt gagactgagt gaggcatttt cagtcctcag agggtgcgag 11640

gggagccagg aggggcacag accccaggct gggggcactg cggggaagct ctctggaggg 11700
gcgagcctca ggggacttct gcagggtgaa taggagttag ctcagctgtc agagggtaag 11760
gggtggaggg gggtgaaaag ggcacttcag acatctttga gcacgaatgg gtgtcccgaa 11820
gggctgtgta ggagccctgt tgggggggat aggaggcgtg agggtggagg aggaggagga 11880
gacagggctg ggttgtagag agctggctaa ttggaccagt gtttgtttgc accattgtct 11940
cggagcagca gggagccatt gaaggtgctt gagcatgggg agtgttttat atgctaatgt 12000
tgaccccggg gtgcagatga gcttggagga agaggctggc aggggccaga gaaggttctg 12060
attgaggtcc agaagatgga gggcgaggct gccccaggca gcagctgcct ggttggggga 12120
tgggaaccct gtggattgca ggaatgttaa caaggtggtg atggacaagg ggaggagggc 12180
cggcccggga aggggggggcc ttctagagtg gtgcccctgg actggggctg cttcaggagg 12240
gcagaggagg ggtgggagcc aacacccact gccaggctcc aggccttcct ccacccgaga 12300
cccactaggt ggggccactc ccaagtgtac aagaagctgc caccttgggg gatggagagg 12360
gctgagggtc atgttatcaa gacttctcag tcctgcccat gcctacctgg catgagccat 12420
ccaggacacc ctggtcaccc cgatgtttgt tctctgagga gctgggatta tgagcactta 12480
agattctgac atcaaggggc agccacgagg gcactcaaga atattcagat ggaaaccatc 12540
ttagatctaa ttggctcatt aaatgtctgc gtggaggagc attttctttc tcaagccggt 12600
gagtcctgga gttagtgcaa cagctttttc actggagacg tgtttctcag gaagcaggtg 12660
cgtctgtaac gtgggtgagg tttgcctgcc gctcagcaac cccagcctct gtccagagtt 12720
ggcgccgccc tgagccccgc agctcctggc cttctgcttg tgttctgctg actctgtcct 12780
gcttctctgc gtcttccatg ctctttcctc ttaccctcac gcctcctatc ccccacaaca 12840
gggctcctat acagcccttt gggacacccg tttgtgctca aagatgtctg acgtgccctt 12900
ttcacccccc tccaccgctt accctctaac agctgagcta actcctattt accctgccac 12960
acctagcggc tggcggctgt tccctggctg gcaattctgt gggccaggga tctggacggg 13020
gctcagtgag gacagctcat ctctgcccca tgaggtgctg cctggggtcc ctcgggcagt 13080
ggcatttggc tatggtgggg cccaaaggcc caggaaagca ctgtgccggc tgttggctgg 13140
gttgcctcag tttccctcca ggaacctcac cctccgatag gacaccttgg gcttctcaat 13200
aggttccaag agagcagaag ctgccaggcc tctgacggtc ttggggcagg gctgggagag 13260
catccctcat gccactttat gccagagcca tcactggctg gcttcgattc cagggaggga 13320
gtggaccagc ctctggagaa ggagcggcct ctgagggcag ggataggggga aacagagtgg 13380
ccggctttgc tgcagtcaca cctgtgtgat atagcaaaat ggggcaggcc ctgttctccc 13440
ccctttcccc aaacacacag gcaaacagga aacatgcgca ccctgtcttg gggaagggggc 13500
agggggggcgg tcagcagcct ctcacctatg tctttccacc caaggccgag cactggggggt 13560
ctcctggtta tgggcgcttg ggtggacact gagaccacag acggcttgtg ctccctgccg 13620
gcaaggtgct cagcctggca gccgggacgg cagcaggtgg ggctgaaccc cagaggaggc 13680
gtccgtgaga gcaccacaga ggggctgtcg gggtcagggg actgaagggg gccagaggcc 13740
aagaaaagca tcatgaaagg tgtcgttatg tggcctgagt ctggaaaggt ctggggtgtt 13800
ctaggtggcc aggggtgtgg agggtggggg tggcagcctc ctgagtggag atgtaggggt 13860
gtggagggtg gggacggcag cctcctgagt ggagatgtag ggggtgtggag ggtggggacg 13920
gcagcctcct gagtggagat gtaggggtgt ggagggtggg gacggcagcc tcctgagtgg 13980
agatgtaggg gtgtggaggg tggggacggc agcctcctga gtggagatgg caggcagacc 14040
tggtgtgcag agggagcttt gagcattccc aagaccgggg ggggctgggt gggagcgggg 14100
gtggagcatg aacccaggga ggcccagcga gagcctagtc gtgcggtgtc ctgggaggcc 14160
atggcccaag accttggcag accagcattg gagatgggca tcttcctcca gggccactga 14220
tgtcagctct gctggcatgg atgatggggg tggctggcga gggtgtcgcg tgggcacgtg 14280
ggagcccgag gctggcggtg ggctgtgctc caggccttgc ccggttgacc gtgtcttcgg 14340
ggtgagtgac gctccgcaga cgccgcatgg aatgcggggc cggctccact ccttccgtaa 14400
tgtggttttt tatagtcggg ggactcattg tcccgtggcc actgccagct gtctgtaagc 14460
tcaggattag agagcctggc ctttcgtgag agctggggct gtgccggctg agcccttcac 14520
agggaggggt ggaccacggg gacagggagc gactgcctgg cactgagtga gccagccagc 14580
agacagcatg acggccttct ccttgcctgc ccccagggcc tggtgcaggc tctcttctgc 14640
ccaccctacc cagcttgggg gccccacccc atcacggccc tcatgagcct ctcggggcat 14700
cctcctggat ggcatcgcag cgggcattgg cctcccctcc atgcccacct ccaattctga 14760
tcaggagcac ccctcaccca cagtaggact ggaagctcta tcgggccaga aggtgcacag 14820
ggggccaggg gacttggact tgagtccacc tcgtggctgt gtgatggtgg acaggtggcg 14880
tgccttctct gggcctcagt gtcctttttg ctaaagtagg aatggcgctg tcccatgacc 14940
cggtttgggg acactacgtg gaaggaagca tccacagcac aaggtgccgc tcgccctcgt 15000
gtgcccgggg tggctgccaa ggggccggca gcgtcgccac gtgtcggggc tggcctgagt 15060
tccgtgtccc tggaatctac agcctgtgtt ccgtgaaggc agagcatgac tgggcagcag 15120
gcagcggggg ttctaatgcc gagatttccc ttccccccaa ctcctgccca ccccggcgcc 15180
ttccctcctg cgtacctttc tttgggcaat gggcagaggc cccggagcgg gcgggattcc 15240
tgtgaggtga cgccagccgg agtgtccagc cgggtgcagc cagcccagtg gcccattcac 15300

```
gcggccgcca gggcttcatt cacccctggc cgggagggca gcgcccccgt gactcgcagc   15360
tggggcttct ccacgggtgg cggctgcatc gctctgccag tgacgctggg ctggtccctg   15420
ggggccctcc tgctgctgtg ggggtgcagg ggtggatgct ggccccagga tgctgcacac   15480
ggcctcaggg cagcacccac acactggcag ctccagcttc cctgaaaga tgaccgagga   15540
ggtggggagg acgcggtgct ggccagcagt gacggctgca gcatgtgtgg gcaggtgccg   15600
tgccacacct tttacaaaca tctgaatgca gccctcccaa cagcccgggg gaaggaagtg   15660
tggtcacatg gcagccttcc tgggatgtga ccccagctcc aggtggcaca gctcccttct   15720
cctgtggcct tggagccctg aggggaccct acttgatgtc tctggggtct cctggctgct   15780
tcatttgccc tcaagacggc tgtgggggca gggtcgctgg gaccttgggg cagaggaggg   15840
actgatcaag tgcctgctgg gtggcagagc agccacatcc ccccatcccc ccaagcaagc   15900
ccggaaggca ggtatgtggg cgggggcacc catctgtcca gggcagggca ccccctccct   15960
cctccccatg ccctgtcggc tgcaggacct aggatgggcg gggcagcact caccccagcg   16020
cccaagtttg aagggtgcgg cggggtccct ggggagtcca gaggctctgg ggacccagct   16080
tcacagagcc aggtggggtg tcggctctgg gcacccggat tcacggggcc aggcggggtg   16140
tcggctctgg gcatctgact tcgtggggcc aggcagggtg tcattgtcgg ctcttggtgg   16200
agtttgctgc ggtttcctgt gttcctcctc ttcagggtcc ccagcccgcc ctcgggccag   16260
actgacctgt ctctgtcctt cctctgcagg ggctggcgtc ttcttcctgt cctcggccga   16320
gggggagcag atcagcttcc tgttcgactg catcgtccga ggcatctccc ccaccaaggg   16380
ccccttttggg ctgcggccgg ttctaccagg tgcgtgtggg agcct                 16425
```

<210> 62
<211> 2031
<212> DNA
<213> Homo Sapiens

<400> 62

```
tcccatcgga cgccactgca tatgcgttct gcctgcagtt gatgatgcag ccacagggca      60
agtgggtcca gcgtttggac aagacaaaca ctggggttac agtggtggcc agcagggtaa     120
gtagaaagct gaatgtctcc aagggaaggc tctgaaaccc cacgacgttc tggaccacca     180
tgtgcatctg caagggcaag agaacttcag acctaaccta aaattatact ttgtacttct     240
caagcctttt ctccacgcac gctgcctcag ttccctttcc agttccttct cctgatggac     300
agcggggacc tctgctacgg agctggctcc ctgtccttgg tgatgggctc ctgggaacgg     360
cggggcacac ccccagggtt gggaggggtt tcagcttttg agtgccggat ctgcgcccgc     420
tgcctattca agactccacc agaaaggcac agtctacaac gtgggggacg ctagctagga     480
aacaggcgtc cgtgaaaccc gcatctgatt ctccgaatgg cctttgtcgc ctccctggac     540
cctcacctcc gcccatgcgc gctcgtcaca gtggggcgcc actctgccac ctcttaatcg     600
gcttcctcct ggaaggagga acaaaagcct gttttgttcc ctgtcttctc cctccacaac     660
tgatctctcc tcccctctat cccgccctgc tggagatggg cgtcgtcctc tctaggcggg     720
aatggaacgg cgagaaaggg atgacagaag gttgtgggga aaaaaccttt taattccctc     780
tttccttttc tgaatactct ctgattttgc tgtgtgagac aatatccttg gccaccaccg     840
atatggaaaa ctaagtcgct cgcgacggaa agcgatcccc atgcccctga cttttgaaag     900
gagaagcatt tagtagtaaa ggtttcgcag ccaactagcc ctgtcccgaa ctgccttctt     960
ctaagtgcat gtctaaaacc cagcgagata cacttggaag cggacttcac tgtgtgtctc    1020
ttggtgactc cccaaaccgg cgacgccggt cccaacactc atttacactg tctggcacac    1080
acactcgcgc tttgtgagca actgcacttt taccatcatg ggcagccaaa ggacgacttt    1140
tgtaagcgct aggatcaaag cgaagcgctt ctgcgctacg ctcacggtga agctcctggt    1200
gccatagaga gtcccccggt tctcacgcct gcaggcttca gccccagcgg cagcgggcgc    1260
acccggtccg aacacggtgt cggagctcgg agagcatccc ccagagcgcc gcagactcgg    1320
gcctggagca tcctctgggg acagggaaac cactctcccc gcagtaggag gggtcccagg    1380
aattgaagct ccacgggaaa tttcctggta gttggagtgg agtctcggcg gctcctccga    1440
acacagcaat cggtgagtga gtgggactga gaggcccacg aggagtccga aggccaaagc    1500
gtacacgata gagaggaata gtacgtagga gctggagcag tccaccaggc agccccaggg    1560
cgtgcgcacg aaggcgcccc agccgcacag cgggagcgcc gagagcagca gactggctgc    1620
ccacacggtc agcaccacgc cgagcacctg gcccgatctt ctggaggctg tctggctccc    1680
cacacctctg tgcatcgtat aaaagttgta agagactagg agagtcgcct tcaagttgct    1740
agagaggccc tggcataaat acattaaggc agaggtggtg cacagaaatt ggaagtaacc    1800
ggggacctcg tttggccact gcaaaaacat gaagatggtc accgacagga cgctcatgag    1860
atcatccaca gaccaggaag ccacaagcat ggacacaaca gttctgttct gcattttcag    1920
cagggaaatt agtgaataaa tgctgcccac caaggctgca aaagtcatga gacatgtcaa    1980
gcaaaaaaga tagatattca gggttcctgg cggatttaaa aggtccgtag a             2031
```

<210> 63
<211> 2237
<212> DNA
<213> Homo Sapiens

<400> 63

gtttgtgaca gacgcctgta ggtatatggt aaggaaaaca gattaaaaca ctaagaaata 60
gtaagaggtc ggaccgaggg tttctcatct gtatgggaaa tctcatctcg ttcattactt 120
tcagaaacaa aaaggaaaac gactttaaag atacaggtta aaggccgaag gggtgtagaa 180
ccaccccccg cctccttccc tccatccctc ccaaatatcc cagaacgagt caaacctttc 240
gaatctcgta gcacagataa caagcaactc agtaagactg gacggggatg actgagggcc 300
aactcattac aggactacag gaaactcctt agtcctaaac ccgaatgcat ccgtgaccat 360
ggtaacactc cctacctgac caaaagctat caaacgctct gacaagtgtt tacagctgct 420
ttctcgatta ctgaagtggc tctgaaaaga gcctttgggg ttaggtggtt gatctattgc 480
gtcccttgca cactcttact tcgagctggt gtacttggtg accgccttgg tgccctcgga 540
cacggcgtgc ttggccagct cgccgggcag cagcaggcgc acggccgtct ggatctcgcg 600
ggatgtgatg gtggagcgct tgttgtagtg cgccaggcgg gacgcctctc ccgcgatgcg 660
ctcgaagatg tcgttgacga aggagttcat gatgcccatg gccttggacg agatgccggt 720
gtcggggtgg acctgcttca gcaccttgta cacgtaaacg gagtagctct ccttgcggct 780
gcgcttgcgc ttcttgccgt ccttcttctg cactttcgta acagcctttt tggagccctt 840
cttgggagca ggagcggatt tcgctggatc cggcattttt gcgcgaaaaa agagaaaaga 900
gacttaaaga agtaatccga actaccgcaa aacgggctgg ttatgcgcta cttatagagc 960
ccgtatgcaa atgaagactc gcaaagtgct gcgctttcat tggttaactt acaacggtgt 1020
cgtcagaggg ggtggagtct atgtaaataa tagattctag tcttgcttcc ttaatggtca 1080
ctgtaacaaa agtcttgtta tcctatcaga atggtccatt tcacactaac cagttttaag 1140
ctaggtgaag cgatagtttc tacggcttgg ggtttttttct tggttttcac tttccattag 1200
acttgcatac ggaagaaatc ttaacgccag ctagacaagt taactctctt gcctcgtttt 1260
tgcattttta ttctatactt tgtggaaatt tttttctctt ccttgaaata ctacctcacc 1320
catttttaaa aatcgccaga tatttactcc cgcccgctta aattactatt ctggcttgga 1380
caccgcctgt agtatgcaat gaggaagatg ctgagcttta attaagctta tagttcctaa 1440
ataaaccagg aaacgggttt ataattttta agggactcgc cccaccctta cactcaggat 1500
cttccacatc tagaaactcc ccatagtctc ttgcccccat gcaaaacttt acccacagaa 1560
ttgggagccg gacctctagc cttaaaccag cgtgaaaacg ctcggccgga accacaagga 1620
ccatgcctct gcggctttct ctactgccgg ccaactcacg gctgggcttt gggttctgta 1680
acgtcaccca ccacgagtgc ccctggcccc tgaccgctgc gcaaggcaga cgcacatcag 1740
atggagagcc ggtacagctg ctcttgatga aaacggcagt ggctctgaaa agagccttta 1800
gatcgaccac ttaaaaatat gccttaggcc cgctccccgc ggatgcggcg ggccaactgg 1860
atgtccttgg gcatgatggt cacgcgcttg gcatggatgg cgcacaggtt cgtgtcttcg 1920
aacagcccca ccaggtaggc ctcgctggcc tcctgcagcg ccatcacggc cgagctctgg 1980
aagcgcaggt ccgtcttaaa gtcctgcgcg atctcgcgta ccagccgctg gaagggcagc 2040
ttgcggatca gcagctccgt agacttctgg tagcgccgga tctcccgcag agccacggtg 2100
ccgggccggt agcggtgcgg cttcttcacc ccgcccgtgg ccggcgcgct cttgcgggcc 2160
gctttggtag ccagctgctt cctcggggcc ttgccgccgg tcgacttgcg ggcagtctgc 2220
ttagtacggg ccatgct 2237

<210> 64
<211> 4182
<212> DNA
<213> Homo Sapiens

<400> 64

agactctctg aacgtttgat gaaatggact cttgtgaaaa ttaacagtga atattcactg 60
ttgcactgta cgaagtctct gaaatgtaat taaaagtttt tattgagccc ccgagctttg 120
gcttgcgcgt atttttccgg tcgcggacat cccaccgcgc agagcctcgc ctccccgctg 180
ccctcagcct ccgatgactt ccccgccccc gccctgctcg gtgacagacg ttctactgct 240
tccaatcgga ggcacccttc gcgggagcgg ccaatcggga gctccggcag gcggggaggc 300
cgggccagtt agatttggag gttcaacttc aacatggccg aagcaagtag cgccaatcta 360
ggcagcggct gtgaggaaaa aaggcatgag ggtcgtcttt cggaatctgt gccacccggc 420

```
actaccattt cgagggtgaa gctcctcgac accatggtgg acacttttct tcagaagctg     480
gtcgccgccg gcaggtaaag tggacgcagc cgcggtggga gtgtttgttg gcaccgaagc     540
tcaaatcccg cgaggtcagg acggccgcag gctggcgcgc ggtgacgtgg gtccgcgttg     600
ggggcggggc agtcggacga ggcgacccag tcaaatcctg agccttagga gtcagggtat     660
tcacgcactg ataacctgta gcggaccggg atagctagct actccttcct acaggaagcc     720
ccgttttcac taaaatttca ggtggttggg aggaaagata gagcctttgc aaattagagc     780
agggtttttt attttttat tatctttgag atagggcctt gcgttgtcgc ccaggctgga     840
gtgcagtggc gtgatcactg cagactcgac ctcctgggct caagcgatct tcctgccgca     900
gcttcccaag tagccgggac tacaggctcg tgctcccgcg cccggctaca gatcagggtt     960
taagtcgcgg cccaccacta tgtgagcaag acgtaagccc cctctccctc gtcggcttct    1020
tgtcagtcaa gtgacagcaa tagtaccttt ctcatagggt cgttttgaga attaaatgag    1080
taaatacacc taaggcgcct aggacaggac ctgacacata gaaagatttg ttagctatta    1140
tgagaatatc ttcttcactg ggagattgcc aggactaaat gtgttaccat gaaaagaaac    1200
atttttcctg tctcgacttt tattttattt atttttattt ttattttttg agacggagtc    1260
tcgtcctgtc gcccaggctg gagcagtggc gcgatcttgg ctcattgcaa cctccgtctc    1320
ccgggttcaa aggattcccc tgcctcagcc tcccaagtag ctgggattac aggtgcacac    1380
taccacaccc ggctaatttt ttgtatctgt actagtagag acgaggtttc accatgttgg    1440
ccaggctggt ctggaactcc tgacctcgtg atccacccac ctcggcctcc caaagtacca    1500
ggattacagg cgtgagccac cgcgcccggc cttcactttt atataattta tatgactcga    1560
gagttttgcc catgatccca ccaccccagc acatcagtgt tgaaacgctt ccaggagttc    1620
ggtgccctga ccacagcctc tccttctcta gccctctcta tccccacttc cctgttcttt    1680
ggcttcatca ggactctcat ctgttcccag tccattcctc ctgggttcag cactttacct    1740
ttcctacgca taattaatga tttgtcttca ctcatgactc atcaaatatt tgagtgtcag    1800
ctggggatac catgaggcag acaaggcctc ttgtcttttt ggagcttgca ttcaatgcgt    1860
agagacgcca acaacatcat cacataggct ggtaagatag taaggccatt attatgtgtc    1920
aacagtgact catgcctgta atccccgtgc tttgggaggc tgaggcagga ggatcatttg    1980
agcctggaag tttgaggctt cggtgagtta taacagcccc attgcactcc agcctaggtg    2040
acagagcaag accctgcctc taaaaaagaa aaaaaaaaaa gataatgaac aggtaatgat    2100
accactagat aggatgcttg gggcgcccat tctgagatgg catttcagct ggaacttgaa    2160
ggatgagaag gaaccagatt attattatta ttattattat tattttttt ttttaggcag    2220
tgtctagctc tgttgcccag gctggagtgc agtggctcga tctcggctca ctgtagtctc    2280
cacctgccgg gttcaagtga ttctcctccc tcagcctccc gagtagttga gattacaggt    2340
gcctgctacc acggccagct aatttttgta ttttttagta gagacagggt ttcaccatgt    2400
tggccaggct ggtctcgaac tgagctcaag cgatccactc acctctgcct cccaaagtgc    2460
tgggattaca ggcatgagcc actgcaccca ggcggaacca cctttttgaa gaattacaga    2520
acgaatgtat aggaaaaggg gacagcctgt gcaaagtccc tgaggtgatc agatattcca    2580
ggaactggca gagaccttct tggctacagc atagtgatca gagtaaaatg agatgaggct    2640
ggagaggcag gtaacagcct tatcatgcag ggcctagttt gccataataa tgaatttgat    2700
aaaataattt gtaatcactt ttatgaacca atcatggcac gtcatcatca tatgtcctgg    2760
cacggtccct cccctgttgt atttattttt ccctactcat tcttgccctt gtctcccctc    2820
ccatagtccc cactttctct tagttatatg tccccccatt tatttattta tttatttatt    2880
tttatttctt ttagagatga ggtcttgctg tgttgcccaa gctgatctca agctctgagg    2940
ctcaagtgat cctcccacct cagcctccca gagtgctagg attacaggca tgagccacag    3000
tgcccgtccc atttatttat ttatgagaca gggtcttgct ctgttgctca ggttggagtg    3060
taatggcaca atctaggctc actgcaaccc ctgcctcttg ggttcaagcg attctcctgt    3120
ctcagcctcc cgagtggctg ggattacagg catgcaccac catgcccagc taattttcgt    3180
atttttagta gagatggggt tttgccatgt tggccaggct ggtgttgaac tcctgacctc    3240
aggtgatccg cctgcctcgg ccacttattt ataacacaaa tatttactga ccattgacag    3300
tgtcaataaa tttgttctag ttctagtagg gttctagttg ctggagataa ggcagtgatc    3360
aagatggaca aggttccaat cttcttggag tagacattct ggtaccatta aaaaaaaata    3420
aaattgagcc tggcgcagtg gctcacacct gtaatcccag cactttgaga ggcagaggcg    3480
ggtggatcac ttgaggtcag aagttcgaga ccagcctggc caatgtagcg aaaccccatc    3540
tctactacaa atataaaaat tagccgggcc tagtggcgca tgcctgtaat cccagctact    3600
cgggaggctg aagcaggaga atcgcttgaa cctgggaagc ggaggtttca gtgagccaag    3660
atcccaccat tgcattctag cctgggcaac agagtgagac tctatctcaa aaaaataaat    3720
aaaataaaat aaaatcagtg taaaggaata gagagcagct gacacgctgt cctctggcga    3780
cctgtcgctg gagaggttgg gactctggat gcgtgcgggg ctctggccta ccggtgaccc    3840
ggctagccgg ccgtgctcct gcttgagccg cctgctgggg cccgcgggcc tgctgatctc    3900
tcgcgcgtcc gagcgtcccg actcccggtg ccggcccggg tccgggtctc tgacccaccc    3960
gggggcggcg gggaaggcgg cgagggctac cctgcccccg tgcgctctcc gctgcgggcg    4020
cccggggcgg ccgcgacaac cccaccccac tggctccgtg ccgtgcgtgt caggcgttct    4080
```

cgtctccgct gggttgtccg ccgccccttc cccggagtgg ggagttggcc agggccgatc 4140
gactcgctgg ccggccggcc cgcctccgct cccggggggc tc 4182

<210> 65
<211> 7572
<212> DNA
<213> Artificial Sequence

<220>
<223> chemically treated genomic DNA (Homo sapiens)

<400> 65

tgtatgagta cggatgttac gagggtattt agcgagtggg aagggagtcg ttttatatgt 60
ttttatatcg atttgtaaaa ttgttttgaa agggtatttt tagatttatt tgttttaaat 120
gtagtaaaat ttattcgata tcgtttataa tgtatgtacg ggttatttta tatgtaatag 180
ttatcgtatt aattaattta gttgatattg agatggtgaa tggggcgggg gagtagggag 240
gaatatgtcg cgtatgaaaa atttcgggtg taaaagttaa tgtagaattt atttttcgag 300
gaatcgaaaa agatatttac gatttttaag tggagagagg atagaaagtt aagtggcggg 360
ggtcgcgatt agtgatcggg attttagttt gattttagta tttagcggaa taggattata 420
atttaaagaa atgagttttt ttcggagtaa agagcggttt cgattttaaa gtagaaaaaa 480
taaattgttt gggggtggaa gatggcgagg ggcgggggc ggggagggag gcgggtcgcg 540
gcgttggttt cggggttcgc ggtacggttt tttttgtcgc gttgtttagg gttcggattt 600
tgttcggcgt tgcgtttatc gcgagggtgt tagattcgtc gcgtcgtcgt cgcgtttttt 660
tagtcgcggt tttttttttc gtcgtttttt tttgtttttt tagttttttt ttttacgtgt 720
ggatgacgtt aaaatttcgc gaaaaagtcg cgtggtggtt tttcgagcgg aggcgcgatt 780
tcgtcgttta gcggtttttt ttcgggggcg tttggagggg gagaagggcg gaggcggtcg 840
gttttttttt ttttcgggag gtaggatttt tcgacgtcga tcgtcggacg tttttcgttt 900
taaagtttat tggaaaattt atttttgtaa agtaaatgta tttttagagt tattttcggt 960
cgcgtgaggc gtgttttaag ttgaattaga taggaagagg gggaagttcg ggttttttaa 1020
tttttttttt tttcgaaggg aggggagtga gatgttaggt gggtgataga cggtaggcgt 1080
tcgttttttt aatttacgtt tgtcgtattt gtcgttttag taatttagtt tcgtttaagt 1140
cgaaattcgt cgaagggagt gcggatgtat aggtttggcg gattttgttt ttttttagaa 1200
cgtagcggtt tagcgtttcg gcgggcgcgg ttgcgattag agggtttcgg aagcgagtga 1260
atatttgtaa tcgtattgtt cgtttttatt tattttgttt tttcgtgttt ttcgtttttc 1320
gacgtttttt tttttttttt gttcgtattt ttttattttg tttgtatttt tttttttttt 1380
ttgatgtgtt ttttatgtgt tttttcggat ttatgtgttt tttcgtattt tgttagtcgg 1440
gttttcggtt ttgattgatc gtttattttt ttacggagaa atataaatat agttttattt 1500
tttgggggag tcgaggggaa ggtagtggtt tttattttttg agtttgaatt tagttattat 1560
tcgtttttta tttggtttag gcgtttttgc gcggagaagg cgggattcga attcgcgttg 1620
ttttcgggtt ttgagtcgat cgcggaatta tttggttggg aggtattttt taggtaagtt 1680
tgggagtatg tgtgtttagt gttgtaggtt tttgtagaaa agtgtttata aatatttaat 1740
attttgttgt atttttttatt tttaggtttt gtatattttt taatcgaaat tttaaaacgt 1800
tagcgtagag ggtggggagt cggtcggaaa aagaataaaa gtttaataaa attggcgttc 1860
gttaaagttt attattagtt ttatatggga tatatatata tgtatttttt ttttcgtgaa 1920
gggtgaaaag gagataagga agaattaata atttattttt tttcgtcgtt ttttattttc 1980
gcgttacggt gattagtttg gattttaaag ttagagtata ggtttttgcg tttttttttg 2040
aaatcgaagt ttatattgaa agaaagtgtg tatttttgtt tagaaggtaa tatgcgtttt 2100
ttcgcgtgtt aggtggagtg tattttaata agatattagg gttttaatat atggttggag 2160
tggcgattaa aagggaaaat ttagttttta gtttaagttt tttagagata tttttgttaa 2220
ttttcgtatt tttttacgtt ttattttacg tgtgagagtt tgtaaaatta tcggggattg 2280
gattcgatgg cgagttttac gttcgggaat agttagtaat cggaagggga agtggatagg 2340
ggaattttaa gaggcgagtt tgttacgcgg gaagcgttcg aatttgcggg tttttatgaa 2400
tgtagagggc gtcgggaagg gggggtattc ggtcgcgatt tttttttgttt tttttattcg 2460
ttgttttttt tttcgttgga atttttttgt aaagtaagac ggagtagggg gagggggaga 2520
gggaaggggc gagagggttt tcggtttatt ttcgagacgt gaggattcgt tatttaggta 2580
ttttttttcgg ggtgggttgg gtttcgggac gattattcgt tttttttttcg ttttcgttgt 2640
ttttatttcg ggagatttag agttttggat gtttttttttc ggagaagggg gggtgtgcgg 2700
agtcggggtg gaagagattt tgttcgtaga gttatattaa gtgatgttta gaggttggga 2760
gtttcggcgg tttttgtttt ttgtttgtcg ggttagattt atattttaaa aatatttttc 2820
gttttttttt tttttttcgt tttttttcgt tgtaattttt tttgattcgt ttaaaggtgg 2880

```
tttaggtgaa attggagtaa tttttttatg ggggttttaa aatgtaagtg aatgttttta     2940
ttcggggtga tttaaaagtt taagtcggga agtttaacgt gattaaaatt aataggtgat     3000
tgttcggggt cgattgtgtg cgggtgtata cggtattcgg ttcgggtgtt attcgcggcg     3060
ttggattgtt ttattttgag tttgtaattt gggtttttta gcgagttttt tttttttttg     3120
aaagttaatg gtttttatag taattagata tttttttcgt tcgttttttt tttttttttt     3180
ttttatatat aggagatggg atatttattt tcgttgttat tgataaggtc ggaggcggtc     3240
gggttttttt ttagttttcg ttcgttttag cgttcgtttt tttttcgttt tttttttggtt     3300
tttttttgat gtagtgggga acgcgtttta ttaaaaaaaa aaaaaaaaaa aaaaaaaaaa     3360
agtaatttgt tcggtaataa ttagcgcgta gtagtaggag ttttagagtt attggttatg     3420
taaatagagg gaggggagac ggcgttttaa attttttttt atttttttaa agcgatattt     3480
tttttttttt tttttattat ttttttcgtt ttattttcgt ttaaagaggt tggtttcggg     3540
gtttgagtta atcgtttgta tttttagttt attcgttttt tttttttcgtt ttgtagggaa     3600
tttagtgtac ggtttattta gttcgcgttt tatttcgttt tgttggtttt tcgcgttttt     3660
gttcgggttt ttttttttcgg tgagggaggt atttagtcgg tttcggtgtg tttagagagt     3720
tcgagttacg ttatgttcgt tgtacgtgtt agtttggtta gtatattagg gcgttggttt     3780
tttttttttt ttttggagtg aaatatatta aagggcgcgg tgggggtggg gggtgacggg     3840
aggaaggagg tgaagaaacg ttattagatc gtattttttg taaagatagt tttgatttaa     3900
gtatgcgtta gagtacgtgt tagggtcgat cgtgttggcg gcgattttat cgtagtcggt     3960
ttttagggag aaagtttggc gagtgaggcg cgaaatcgga ggggtcggcg aggatgcggg     4020
cgaaggatcg agcgtggagg ttttatgttt tcggggaaag gaaggggtgg tggtgtttgc     4080
gtagggggag cgagggggag tcggatttaa tttttttattc gttttttttt tttttcgggt     4140
tattttttag aaagttgtat cggtgtggtt acgtttagcg tagatatttc gggcggtttg     4200
ttagtagatg taggggcgag gaagcgggtt ttttttgcgt ggtcgttggt cgcgggggaa     4260
tcgttgggag ttttgttttc ggtttgcggc ggttttagac gttgtatcgc gtcgttttac     4320
ggcgttcgaa gagtttttag aaatacgatg gtttttgttc gaggattata ttttattttt     4380
ttagagaagt attttttttt tttttttaata tttatttttt tttttttttt tttttttttgt     4440
atatattttg taggggggggg tagaagggac gttgttttgg tttttttaat cggggttttc     4500
gaaatagttt cgaagttatt aggaatatag attttaggga tatgattttt atttttgggt     4560
atgcgaggtt gttatttttt aaaattattt ttttttttat tttttattta aggatttat     4620
ttttaaattg tttgaggtta ttttattttt agataattta ttttatattt ttggatttta     4680
aatataaggg taggaggatt aggattcgtt ttgaagaagt taaagttgga gggtcgtatt     4740
ttggcgtgtt atatttatag aatgagtgaa attagagggt agaaatagga gtcggtagtt     4800
ttttgtgggt tgttttgttc ggggtttttg gtatgtaggg ttggatggag ggagaggggt     4860
ggggggtggc gggggatcgc gtttgaagtt gggtcgggtt agttgttgtt ttttttaata     4920
acgagagggg aaaaggaggg agggagggag agattgaaag gaggagggga ggatcgggag     4980
gggaggaaag gggaggagga attagagcgg ggagcgcggg gagagggagg agagttaatt     5040
gtttagttag tttgcgttat cgttttagag cggagaagag cgagtagggg agagcgagat     5100
tagttttaag ggaggatcg gtgcgagtga ggtagtttcg aggttttgtt cgtttattat     5160
ttaattttcg tttttttttt gttttatttt ttttttttgt ttttattttt ttttcgaaaa     5220
tttttttattt agttaaagga aggaggttag gggaacgttt tttttttttt ttttaaaaaa     5280
taaaaataga aaaatttttt tttaggtcgg ggaaagtagg agggagaggg gtcgtcgggt     5340
tggttatgga gttgttgtgt tacgaggtgg attcggttcg tagggtcgtg cgggatcgta     5400
atttgtttcg agacgatcgc gttttgtaga atttgtttat tatcgaggag cgttattttt     5460
cgtagtgttt ttattttaag tgcgtgtaga aggatattta attttatatg cgtagaatgg     5520
tggttatttg gatgttggag gtaggtcggg gggtggcgtt cgttaggagt taggattttt     5580
tcggatgttc gggttttcgg tcggagtttt aaatttggga gagggtaatt ttcgcgtcgg     5640
tttttcggtt tttgtgcggg agtttatcgc gcgttttttg gcgagacgcg tggttttatt     5700
tttgtttttt tttagataaa ttggggaggt agagggggga ggaaaatttg ggagaagcga     5760
ggttgttttg ggcgggggta ggggagtatt tcgcgcgcgt gtttttgtat gtggttgttt     5820
ttttttttta tttttttcgc gatttgtttt ttgcgaagtc gtcgcggttg tttgcgttgc     5880
ggttaggaag agagtcgggg tttgaaatcg ggatttcgag tagaaaggta attttttttta     5940
aaaggttaga gtaaattcgt tttggtttta ggttttcgtt tgtggtcgcg atttcgcgtt     6000
ggtattttat cggggaggtg gagggaggag ggagaaggag agaaacgggg aattcgggag     6060
tttcggaagt tttattgaag aaacgcgtgt tttaggggaa tttaaaagaa tcgttttttg     6120
tttttttattt taagttttg tttagcgagt cgtgtgttta cgtatgtata gttttggatt     6180
tgtcgtggtt tcgttatgtt gttttgtaaa ttttttttgg aaaggttggg aaacgtcgtt     6240
cgtttatttt cgtattttat tatttcggtt attttatttt taatcgtgtt ttttttttta     6300
tttttttattt tacgaagatt atttatagtt tggtttttta gttttttcgg tgcggagatt     6360
tgggtggggt aggaaggggg ggagggggtg tgtgtagggg ggatatcggg ttatttttga     6420
aggagaggtg ggagttttc gtgttttttt ttagggtttt ttgttttcgt tttttttttc     6480
gttcgttatc gcgtttttta gtttttgttt ttgtgggtcg tagtcggata ttttcgtcgc     6540
```

```
ggcgtttatt tttttaagtt ttttttcggg atttaaagtt ttaggggtcg gaatatagat    6600
ttttttggta ttcgaaattt ttgtttcggg aagtttggtt tttttttggg gtttttacgt    6660
taaaagggtt tttttaggat attttttcga tttttaatta ttttttgaaa ttattttttt    6720
ttgtttcgat tttagttgtc gaggttgcgg cgtttttttt tttttttttt tttttttttt    6780
tttttttatt tttttttatt cgttttttta aaagcggttt ttgggtcgta gggatttttt    6840
tagatatttt ttttaattgt cgggaatgat agaggaggat ttggggattc ggatgagatt    6900
aagaagtgga ttttcggagt ttttagaata tttttattga tttttttgaaa agatgacgaa    6960
atttaaaaaa gagtgagtga gttagagtgc gcgaaggagt agttaaaggg agcgtcgcgg    7020
agttgtcgcg gttgttgcgt atttttttcga tttttcgttt tggagttgta gtttattttt    7080
ttttatagga tttttgggaa tattaaagta ttgatgggtt attttgattt attttagttt    7140
ttttattttt gtttttttatt tttattacgt attttggttt tttttttttt ttataaaaaa    7200
aaattaattt tttttgtttc gatagattac gttttttttat tttttttttt ttttgttgat    7260
gttatgtttt ttattttcgt tttttaattt tttttttattt ttattatagg tttgtgagga    7320
atagaagtgc gaagaagagg tttttttttt ggttatgaat tatttggatc gtttttttggt    7380
tggggtttcg atttcgaagt tttatttgta atttttgggt gttgtttgta tgtttttggt    7440
ttttaaattt aaagagatta gttcgttgat cgcggagaag ttgtgtattt atatcgataa    7500
ttttattaag ttttaggagt tgttggtaat gatcggtttt tttttttttt tttttttgcg    7560
attttcgttt tt                                                        7572
```

<210> 66
<211> 7572
<212> DNA
<213> Artificial Sequence

<220>
<223> chemically treated genomic DNA (Homo sapiens)

<400> 66

```
gaaagcggga atcgtagaaa ggaagggagg aaggggtcgg ttattattag tagtttttga      60
ggtttgatgg agttgtcggt gtaaatgtat agttttttcg cggttagcgg gttggttttt     120
ttgagtttgg aggttaggaa tatgtagata gtatttagga gttgtagatg ggatttcgga     180
gtcgggattt tagttaagaa acggtttagg taatttatgg ttagagggaa gatttttttt     240
tcgtattttt gtttttttata gatttataat gggagtggga aagggttggg gggcgggggt     300
ggagagtata gtattagtaa aagagaaaaa gaataaaaaa gcgtaattta tcgaaataaa     360
aaaaattaat ttttttttgt gggaggggga ggggattaga atgcgtgata agaataaaga     420
ataaagatga ggaaattgga gtgaattaga atagtttatt agtgttttgg tatttttagg     480
gattttataa aagggggtga attatagttt taaaacgggg agtcggagaa gtgcgtagta     540
gtcgcggtag tttcgcgacg ttttttttgg ttatttttttc gcgtatttta atttatttat     600
ttttttttgg atttcgttat ttttttaaaa aattaataaa aatattttgg aggtttcggg     660
ggtttatttt ttggttttat tcggattttt agattttttt ttattatttt cgataattgg     720
aaaaaatgtt tgggggggtt tttgcggttt aggggtcgtt tttggggggg cgggtgagga     780
gaggtgggga agagaggggg agggaggaga ggaggaagag cgtcgtagtt tcggtagttg     840
gagtcggagt agggggggat ggttttaaaa aataattaaa aatcgaggaa atattttaga     900
aaagtttttt tggcgtgaaa attttaaaga agagttaggt ttttcgaaat agaggtttcg     960
ggtgttagaa aggtttgtat ttcgattttt ggggttttgg atttcgggag gaaatttgaa    1020
ggggtgagcg tcgcggcggg agtgttcggt tgcggtttat aaggatagaa attagggaac    1080
gcggtgacga gcgaggggaa agacgggagt agagagtttt ggaaagaggt acgggggggtt    1140
tttattttttt ttttaggggt gattcggtat ttttttttata tatatttttt tttttttttt    1200
ttattttatt taaattttcg tatcgaggaa gttggaaaat taaattgtaa gtaattttcg    1260
tgaggtgggg ggtgggggag agggtacgat tagaagtggg gtggtcggga taatggggtg    1320
cgagggtaag cgggcgacgt tttttagttt ttttaaaggg agtttgtaaa gtaatatggc    1380
gaaattacgg taggtttaga gttgtgtata cgtgggtata cggttcgttg ggtaaagatt    1440
tggagtgagg ggtaagaagc ggttttttttg ggtttttttg aaatacgcgt tttttttaatg    1500
ggattttcgg ggttttcgaa tttttcgttt tttttttttt tttttttttt tttatttttt    1560
cggtgaagtg ttagcgcgga gtcgcgatta taggcgggga tttgaggtta agacgaattt    1620
gttttggttt tttggggggg gttgtttttt tattcggggt ttcgatttta ggtttcgatt    1680
ttttttttgg tcgtagcgta agtagtcgcg gcggtttcgt aaaagatagg tcgcgagggg    1740
ggtgggaaga agaggtagtt atatgtagga atacgcgcgc gggatgtttt tttattttcg    1800
tttaggatag tttcgttttt tttagatttt tttttttttt ttgttttttt agtttatttg    1860
gagaggaata gaaataaagt tacgcgtttc gttagaaggc gcgcggtaaa ttttcgtata    1920
```

ggagtcggga ggtcggcgcg gggattgttt ttttttaggt ttagggtttc ggtcggggat 1980
tcgagtattc ggaggggttt tggtttttgg cgagcgttat ttttcgattt atttttagta 2040
tttaggtggt tattattttg cgtatgtagg gttggatgtt tttttgtacg tatttgaagt 2100
aggagtattg cggaaggtag cgtttttcga tggtgagtag gttttgtagg acgcggtcgt 2160
ttcggagtag gttgcggttt cgtacggttt tgcggatcgg gtttatttcg tggtatagta 2220
gttttatggt tagttcggcg gttttttttt tttttgtttt tttcggtttg gaaaaaggtt 2280
tttttgtttt tgttttttgg aaggggaggg gagagcgttt ttttgatttt tttttttttgg 2340
ttaaataggg ggttttcggg ggagaggtga gggtagagag agaaggtgga gtagaaggga 2400
ggcgaggatt gggtggtggg cgagtagagt ttcggggttg ttttattcgt atcggttttt 2460
tttttaaaat tggtttcgtt tttttttgtt cgtttttttt cgttttgaag cggtgacgta 2520
agttggttgg gtagttagtt tttttttttt ttttcgcgtt tttcgttttg gttttttttt 2580
tttttttttt ttttttcggt tttttttttt tttttttaat tttttttttt tttttttttt 2640
tttttttttc gttattaagg agaatagtag ttggttcgat ttaattttaa acgcggtttt 2700
tcgttatttt ttattttttt ttttttattt agttttgtat gttaggggtt tcggataggg 2760
taatttataa aaaattatcg atttttattt ttgtttttta attttattta ttttgtaggt 2820
gtagtacgtt aaaatacgat tttttaattt tggttttttt aaaacggatt ttaatttttt 2880
tgtttttgta tttaagattt aggaatgtag ggtagattat ttgaagatgg ggtgatttta 2940
gataatttag aaataggttt tttaagtgaa aaataaggga gggggtgatt ttagaaaata 3000
gtaatttcgt atatttagag ataaaggtta tgtttttgaa gtttgtgttt ttgataattt 3060
cgaagttgtt tcgaaagttt cgattaaagg gattagaata acgttttttt tgtttttttt 3120
tgtagagtgt gtgtagagga agaagaggga gggagaggtg ggtattagga aggaaggggg 3180
gtgttttttt ggagggatag aatgtgattt tcgagtagaa attatcgtgt ttttgggggt 3240
ttttcgggcg tcgtggggcg acgcggtgta gcgtttaggg tcgtcgtagg tcgggggtag 3300
ggtttttagc ggtttttttcg cggttagcgg ttacgtagga aaaattcgtt ttttcgtttt 3360
tgtatttgtt gataagtcgt tcgaggtgtt tgcgttgagc gtggttatat cgatgtagtt 3420
ttttaggaaa tggttcggga gggggagggg gcgagtgagg gattaggttc ggtttttttt 3480
cgtttttttt gcgtaaatat tattattttt tttttttttc ggaggtatga ggtttttacg 3540
ttcggttttt cgttcgtatt ttcgtcgatt ttttcggttt cgcgttttat tcgttaggtt 3600
ttttttttgg gagtcgattg cggtgaagtc gtcgttagta cggtcggttt tgatacgtgt 3660
tttaacgtat gtttgagtta aggttgtttt tataggagat acgatttggt ggcgtttttt 3720
tatttttttt ttttcgttat tttttatttt tatcgcgttt tttggtgtat tttattttag 3780
gaggaagggg agagattagc gttttgatgt gttggttaag ttggtacgtg tagcgggtat 3840
ggcgtggttc gagtttttttg ggtatatcga ggtcgattga gtgttttttt tatcgagaga 3900
gggggttcgg gtaggggcgc ggagagttag taaggcgggg tggggcgcgg gttgggtgag 3960
tcgtatatta ggttttttgt aaggcggagg aggggagcga ataaattaga ggtgtaagcg 4020
attaatttag gtttcggagt tagttttttt aaacgagggt ggggcggaag gggtggtggg 4080
ggggaaagga ggggatatcg ttttaaaagg gtgaggaaga gtttggggcg tcgttttttt 4140
tttttttatt tgtatagtta atagttttgg ggtttttgtt attgcgcgtt gattgttatc 4200
gggtagatta tttttttttt tttttttttt tttttttttt agtaggacgc gtttttttatt 4260
atattaaaag gaagttaagg gagggcggag ggagagcggg cgttggggcg ggcgaaagtt 4320
ggggagaggt tcggtcgttt tcgattttat taatagtagc gggaatgagt attttatttt 4380
ttatatgtaa agaaagggga gggaagggggc gggcgaggaa aatgtttaat tgttgtggaa 4440
gttattagtt tttaggaaga aaaaaagttc gttgaaaaat ttaagttgta aatttaaaat 4500
gaaatagttt agcgtcgcgg atgatattcg ggtcgaatat cgtgtatatt cgtatatagt 4560
cggtttcgaa taattattta ttggttttag ttacgttggg tttttcgatt taagtttttg 4620
agttatttcg gataaggata tttatttata ttttaagatt tttataaggg aattatttta 4680
attttattta agttattttt gagcggatta aagaaagttg tagcggggag aggcgagaga 4740
aggagggagg gcgggaggta tttttaaagt ataaatttaa ttcgataggt aagggataga 4800
ggtcgtcggg gtttttagtt tttggatatt atttgatata gttttgcgag taaggttttt 4860
tttatttcga tttcgtatat tttttttttt tcggggaaag gtatttagag ttttgggttt 4920
ttcgaagtgg gggtagcggg ggcgaggaag aagcgggtgg tcgtttcggg gtttagttta 4980
tttcgaggag aatgtttggg tggcgagttt ttacgtttcg ggagtgagtc gagggttttt 5040
tcgttttttt tttttttttt tttttttatt tcgttttgtt ttatagagaa attttagcgg 5100
ggaggggggt agcgaatggg aggggtagag agggtcgcgg tcggatgttt tttttttttcg 5160
gcgtttttttg tatttatgga gattcgtaag ttcgggcgtt tttcgcgtgg taggttcgtt 5220
ttttgaagtt tttttgttta tttttttttt cgattattga ttgttttcga gcgtgaagtt 5280
cgttatcgaa tttaattttc ggtggttttg tagattttta tacgtggggt ggggcgtgag 5340
agggtgcgga ggttggtagg aatgttttta ggaggtttgg attggaaatt gagttttttt 5400
ttttggtcgt tattttagtt atgtgttaaa attttaatgt tttgttaaaa tgtattttat 5460
ttagtacgcg ggaaaacgta tgttgttttt taggtaaaag tgtatatttt tttttagtgt 5520
agatttcggt tttaagaaaa agcgtaagag tttgtgtttt ggttttgaag tttaaattga 5580

ttatcgtggc gcgggggtgg ggggcggcgg gagggggtag attgttggtt ttttttttatt 5640
ttttttttat tttttacgga aaaaaaaata tatatatata tattttatgt aggattggtg 5700
ataaatttta gcgaacgtta gttttattgg atttttattt ttttttcggt cggtttttta 5760
ttttttacgt tagcgttttg gagtttcggt tggaaagtgt ataaagtttg gaggtggagg 5820
gtgtagtagg gtgttgggtg tttataggta tttttttgta ggagtttgta gtattaagta 5880
tatatatttt taagtttatt tggaggatgt tttttagtta ggtgatttcg cggtcggttt 5940
aaggttcggg agtagcgcga gttcgagttt cgttttttttc gcgtaggggc gtttaggtta 6000
ggtggggagc gggtagtgat tgagtttagg tttagaaatg ggagttattg tttttttttc 6060
ggttttttta aagagtggag ttgtgtttgt gtttttcgt ggagggatgg acggttaatt 6120

aaaatcgaaa attcgattgg taaaatgcga ggggatatat aaattcgagg ggatatatag 6180
gaggtatatt aagaggaagg agggagtgta ggtaaagtag aaaaatgcgg ataaggagaa 6240
gaggaaaacg tcgggaagcg gagagtacgg gggagtaggg tgggtgggga cgggtagtgc 6300
gattgtaggt gtttattcgt tttcgggatt ttttggtcgt agtcgcgttc gtcggggcgt 6360
tgggtcgttg cgttttggag gggggtagag ttcgttaggt ttgtgtattc gtattttttt 6420
cggcgaattt cggtttggac ggggttggat tattgaggcg gtagatgcga taggcgtgag 6480
ttaagaaggc gagcgtttgt cgtttgttat ttatttagta ttttattttt ttttttcgg 6540
aaaaaaaaaa aattaaaaga ttcgaatttt tttttttttt tgtttgattt agtttaggat 6600
acgttttacg cggtcgaaaa tagttttgga aatatatttg ttttataaaa gtgaattttt 6660
taataagttt tggggcgggg ggcgttcggc ggtcggcgtc ggggggtttt gtttttcggg 6720
aggagaagga atcggtcgtt ttcgtttttt ttttttttta ggcgttttcg ggagggggtc 6780
gttgggcggc ggaatcgcgt tttcgttcgg ggagttatta cgcggttttt tcgcggaatt 6840
ttgacgttat ttatacgtgg gggaaggggt tgggagagta agaggaagcg gcgggggagg 6900
gggtcgcggt tgggagagcg cggcggcggc gcggcgggtt tggtatttc gcggtgggcg 6960
tagcgtcggg tagggttcgg gttttgggta gcgcggtaga ggaggtcgtg tcgcggattt 7020
cggagttagc gtcgcgattc gttttttttt tcgtttttcg tttttcgtta tttttttattt 7080
ttaagtagtt tattttttttt attttgaaat cggggtcgtt ttttgtttcg gaaaaagttt 7140
atttttttag gttatggttt tgtttcgttg ggtattgaga ttagattgaa atttcgatta 7200
ttggtcgcga ttttcgttat ttgatttttt attttttttt tatttgagag tcgtgaatgt 7260
ttttttcggt ttttcgggga gtgagttttg tattgatttt tatattcgga gtttttttatg 7320
cgcgatatat tttttttttgt tttttcgttt tatttattat tttagtatta gttgggttgg 7380
ttaatacgat ggttattata tgtaagataa ttcgtgtatg tattgtgagc gatatcggat 7440
gaattttgtt gtatttggaa taaatggatt tagaaatatt tttttaaaat agttttataa 7500
atcgatgtga aaatatgtag ggcgattttt tttttattcg ttggatattt tcgtggtatt 7560
cgtgtttatg ta 7572

<210> 67
<211> 3491
<212> DNA
<213> Artificial Sequence

<220>
<223> chemically treated genomic DNA (Homo sapiens)

<400> 67

gttggtttcg aattttcgat tttaggtgat ttttttcgtt ttgatttttt aaagtgaagg 60
gattataagg cgtgaggtat cgcgttcggt cgttttttgaa taatttcgat taaaatttat 120
attcgatatt tattttaata tatattatag atttttattg ataatttttt ttagtaagaa 180
agataagttt tatttaggta tttgtgaatt ggaggttaag tagttttagt atattttata 240
ttttttttaag attttttttt tattttaaac gttcgtaaat tttgtatttg ataaagagta 300
tatttttatt taatataaat atgtttttttt ttttagattt ttttagtatt cgagagattt 360
gtacgcgcgt ggtttttttat tttttttttt tggtttttta agtttttagg gcgtcgttag 420
gaggaggttt gtgattataa attttttttg aaaatttttt aggaagtttt tttttttttc 480
ggagaatcga agcgttattt gattttaatt tttttgtaaa tttcgttttt tagagtcgtt 540
cgttattttt tgttttcgtt gtagattttt tatttatttg gatcggtttt cgatcgtaat 600
tattcggtgc gttgggtagc gttttcgttt ttagtagcgt tcgtattttt tttattcgat 660
ttcgggtcgc ggtcgtggtt agttagttag tcgaaggttt tatgttgttt ttcgtcgtcg 720
gttttatgtt gtttttcgtc gttcgttgtt tgtttttttt tttttcgtag tcgtcgagcg 780
tacgcggttc gttttatttt ttggtgatta gttagttttt tttttttttt ttttcggtgt 840
tggcggaaga gttttttcg attttgtttt ttaaattttt tggagggatc gcggtatttt 900

```
tttaggtaag gggacgtcgt gagcgagtgt tcggaggagg tgttattaat ttcgagtatt     960
tagcgaatgt ggtatttttg aagtcgtttt aggttgggtt tttttcgggg gtattagtcg    1020
gaagtagttt tcgttagagt tagcgttggt aaggaaggag gattgggttt tttttattt    1080
gtttttata tcgtttttcg gtttttttgt ttttagtcgc gtttttcgt ttgttagtaa    1140
aggcgtgttt gagtgcgttt attttgttaa aaagaaattc gttttcgttt cgttttttt    1200
tttcgcgata taattttttt aattgttaaa ttgaatcggg gtgtttggtg ttatagggaa    1260
agtatggttt tttttttaa ttataagaaa aagtaaaatt atttttttt agttgtgaga    1320
gttttatcga gaatcgaaat tatttgtacg attagaaagt gtttttttat tttttaatt    1380
tttgattttt aggagcgcgg ggtttattaa gttagaaatt ttagtttaaa ggattttttt    1440
tggagagtcg gattgttttt tttttttttt tttttttttt ttttgcgtgt aaaacggttg    1500
tttggggtaa gggtttttta gacgtgtata ttgtttggta taagagtaga ttttgaaaag    1560
atgaggttta tttaatacgg acgggggaga attttgtttg taggtagata ggaaaatggg    1620
gagggagtta ttggaaggac ggattttatt tttaaagtta taatttttag attagaaaaa    1680
gtgtttagtg ttttagaagt agagttgtat agtgatttaa agattagttt taaatattgt    1740
tttgtttttt ttatattttt tatatttttt ttttttattg aaaatatttt gtatttttcg    1800
taattataaa gggggaaggg aatatgagtg ttttttgttt tataggggtt gttgtgagtt    1860
taaatgatgt attaatatat ataagtttta agaatagtgt tatatatttt aagttaatat    1920
ttgttagttt ttgaattatt cgttttgagg attggtttgt aattttgttt tgaggtatag    1980
aaagaaaatg ttttggagta ggacgcggtg gtttatattt gtaattttag tattttggga    2040
agtcgaggcg ggtagattat ttgaggttag gagttcgagg ttagtttggt taaaatggtg    2100
atatttcgtt tttattaaaa atataaaaat tagttggtta tggtggcgta cgtgtgtaat    2160
tttagttatt taggaggttg aggtaggaga atcgtttgaa ttcgggaggt agaggttgta    2220
gtaagtcgag atcgcgttat tattttttag tttgggtgat agaatgagat ttcgatttaa    2280

aaaaaaaaaa aaaaatgttt tggatagaat tattattatt atataaaagg aaagttcgga    2340
tgcggtggtt tacgtttata attttagtat tttgggaggt cgagataggc ggattatttg    2400
aggttaggag ttcgagataa gtttgattaa tatggcgaaa ttttgttttt attaaaaaat    2460
ataaaattag cggggtttgg tggcgtatgt ttgtaatttt agttattcgg aggttgatgt    2520
aggagaatcg tttgaattta ggagaaggcg gaggttgtag tgagtcgaga tcgcgttatt    2580
gtattttagt ttgggagata agagcgaaat ttggttttaa gaaaaaaaga aagaaagaaa    2640
gaaagaaaga ttaagaagaa tttatttttt gaaaagatta tgggtatttt ttattatttt    2700
tatttataaa gaaaagttaa atagtattaa agagtataat aagcgtaagg aggtaaaagt    2760
tttaattttt tttgtgatta ttattttta agtttattaa aaatatgtat tacgttttaa    2820
aaaatggatt gtttagattt tgttgatgtt ttaagtatat gtttaatttg tttattggat    2880
aatttagttt tgtttaaaag ttatatttta atttttggtt gatttaaatt ttgtagattt    2940
agtatatttt gtatttttag tgtttgtttg attttaaaat atgtagtttt taaaatgaag    3000
ttaatgaaaa taatttggga tgttaagtat gttattaaaa tttataatgt attattgtat    3060
tatttatatt tttttcgggg tattttttaa ttagttgtgt agtaatgata gggaaaattt    3120
aaattatcga taaataaaat tattttagtt tagtttaaga tattttatga tggaggagga    3180
agaaagtggt tgttaggatg ggagggaggg aatatatttt tattattaat ataatggttt    3240
tttttttttg tttgtttgtt ttgtgtgttt ttttgagatg gagtttcgtt ttgttgttta    3300
ggttggagtg aaatggtatc gtacgatttt tttcggttta ttgtaatttt cgtttttcgg    3360
gttcgagtaa ttttttttgtt ttagtttttc gagtagttgg gattataggt atatgttgtt    3420
atatttagtt aatttttgta ttattagtag agacggggtt ttattatgtt ggttaggttt    3480
gtttcgaatt t                                                        3491
```

<210> 68
<211> 3491
<212> DNA
<213> Artificial Sequence

<220>
<223> chemically treated genomic DNA (Homo sapiens)

<400> 68

```
gagttcgaga taagtttagt taatatagtg aaatttcgtt tttattaata atataaaaat      60
tagttgggta tggtagtatg tgtttgtaat tttagttatt cgggaggttg aggtaggaga     120
attgttcgaa ttcgggaggc ggaggttgta gtgaatcgag agagatcgtg cggtgttatt     180
ttattttagt ttgggtaata gagcgaaatt ttattttaaa aaaatatata aaataaataa     240
ataaaaagaa agaattattg tattagtgat ggaaatgtgt tttttttttt ttattttggt     300
```

```
aattattttt ttttttttt attataaaat attttaaatt aaattaaaat aattttattt     360
atcgatagtt tgaatttttt ttattattgt tatatagtta attgagaggt atttcgagga    420
aaatataaat ggtatagtaa tgtattgtag attttaataa tatatttgat attttaaatt    480
gtttttattg gttttatttt aaaaattata tgttttaaaa ttaagtagat attaaaagta    540
taagatatat tgggtttata aggtttaagt taattaggga ttgaaatata atttttaaat    600
agagttggat tatttagtag gtagattaag tatgtgttta aggtattagt aaagtttgag    660
taatttattt tttaaaacgt agtatatgtt tttgataagt ttaaaaagta gtagttatag    720
gaaaaattag aatttttatt tttttgcgtt tgttatattt tttagtgttg tttaattttt    780
ttttgtaagt gagggtggtg gagggtgttt ataatttttt tagggagtaa gttttttttg    840
gttttttttt ttttttttt ttttttttt tttgagatta agtttcgttt ttgtttttta      900
ggttggagtg taatggcgcg atttcggttt attgtaattt tcgtttttttt ttgggtttaa   960
gcgatttttt tatattagtt ttcgagtagt tgggattata ggtatgcgtt attaagtttc   1020
gttaattttg tattttttag tagagatagg gtttcgttat gttggttagg tttgtttcga   1080
atttttggtt ttaggtgatt cgtttgtttc ggttttttag aatgttggga ttatagacgt   1140
gagttatcgt attcggattt tttttttatg taatagtgat aattttattt aaagtatttt   1200
tttttttttt tttgagtcgg agttttattt tgttatttag gttggagggt ggtggcgcga   1260
tttcggttta ttgtaatttt tgtttttcgg gtttaagcga ttttttttgtt ttagtttttt   1320
gagtagttgg aattatatac gtgcgttatt atggttagtt aatttttgta tttttagtag   1380
agacggggtg ttattatttt ggttaagttg gtttcgaatt tttgatttta ggtgatttgt   1440
tcgtttcggt tttttaaagt gttgggatta taggtgtgag ttatcgcgtt ttgttttaaa   1500
gtattttttt tttatgtttt aaaataagat tgtaagttag tttttaaagc ggataattta   1560
agagttaata ggtattagtt taggatgtgt ggtattgttt ttaaggttta tatgtattaa   1620
tatattattt aaatttataa taatttttat aaagtagggg gtatttatat tttttttttt   1680
ttttataatt acgaaaaatg taaggtattt ttagtaggaa agagaaatgt gagaagtgtg   1740
aaggagatag gatagtattt gaagttggtt tttggattat tgtgtaattt tgtttttaga   1800
atattgagta ttttttttgg tttaggaatt atgattttga gaatggagtt cgttttttta   1860
atgatttttt ttttattttt ttatttgttt ataggtagaa ttttttttcg ttcgtattaa   1920
ataaatttta ttttttttaga gtttgttttt atattaggta atgtatacgt ttgagaaatt   1980
tttgttttag atagtcgttt tatacgtagg aggggaaggg gaggggaagg agagagtagt   2040
tcgatttttt aaaaggaatt ttttgaatta gggtttttga tttagtgaat ttcgcgtttt   2100
tgaaaattaa gggttgaggg ggtaggggga tatttttttag tcgtataggt gatttcgatt   2160
ttcggtgggg tttttataat taggaaagaa tagttttgtt ttttttttatg attaaaagaa   2220
gaagttatat tttttttatg atattaaata tttcgattta atttggtagt taggaaggtt   2280
gtatcgcgga ggaaggaaac ggggcggggg cggatttttt tttaatagag tgaacgtatt   2340
taaatacgtt tttgttggta ggcgggggag cgcggttggg agtagggagg tcggagggcg   2400
gtgtgggggg taggtgggga ggagtttagt tttttttttt tgttaacgtt ggttttggcg   2460
agggttgttt tcggttggtg ttttcggggg agatttaatt tggggcgatt ttaggggtgt   2520

tatattcgtt aagtgttcgg agttaatagt atttttttcg agtattcgtt tacggcgttt   2580
ttttgtttgg aaagatatcg cggtttttttt agaggatttg agggataggg tcggaggggg   2640
ttttttcgtt agtatcggag gaagaaagag gaggggttgg ttggttatta gagggtgggg   2700
cggatcgcgt gcgttcggcg gttgcggaga gggggagagt aggtagcggg cggcggggag   2760
tagtatggag tcggcggcgg ggagtagtat ggagttttcg gttgattggt tggttacggt   2820
cgcggttcgg ggtcgggtag aggaggtgcg ggcgttgttg gaggcggggg cgttgtttaa   2880
cgtatcgaat agttacggtc ggaggtcgat ttaggtgggt agagggtttg tagcgggagt   2940
aggggatggc gggcgatttt ggaggacgaa gtttgtaggg gaattggaat taggtagcgt   3000
ttcgattttt cggaaaaagg ggaggttttt tggggagttt ttagaagggg tttgtaatta   3060
tagatttttt tttggcgacg ttttgggggt ttgggaagtt aaggaagagg aatgaggagt   3120
tacgcgcgta tagatttttc gaatgttgag aagatttgaa ggggggaata tatttgtatt   3180
agatggaagt atgtttttta ttagatataa aatttacgaa cgtttgggat aaaaagggag   3240
ttttaaagaa atgtaagatg tgttgggatt atttagtttt taatttatag atatttggat   3300
ggagtttatt tttttattta ggagggatta ttagtggaaa tttgtggtgt atgttggaat   3360
aaatatcgaa tataaatttt gatcgaaatt atttagaagc ggtcgggcgc ggtgttttac   3420
gttttgtaat ttttttattt tgggagatta aggcgggggg aattatttga ggtcgggagt   3480
tcgagattag t                                                        3491
```

<210> 69
<211> 1145
<212> DNA
<213> Artificial Sequence

<220>
<223> chemically treated genomic DNA (Homo sapiens)

<400> 69

```
gggtacggag gtattgcgtt atttagggta agatttcgtt tttttttag ttttttttt      60
aggatattta atattttgtg aaatttagag attttgtttt agtcggattt agagaaattt    120
agcgggaaag gagaggttaa aggttgaatt taatggtgta aggttttacg gttcggttat    180
tttttgtttt gacgtcgcgg ggttagcggg agaagaaagt tagtgcgttt ttgggcgtag    240
gggttagtgg ggttcggagg tataggtatt tcgcgatatt ttaggttttt cgatttacgt    300
ttttggtagt ttcgattatt tatagtttta gtagagtacg gggcgggggt agaggggttc    360
gttcgggagg gttgttattt tttaaaattt ttgcgggttg tttagttata gttttttttg    420
tttgggtgtg tttttcgttc gttttttttt ttcgttttag gttattgttt ttaatttcga    480
ataaaaattg tagttaattt tcgaggtagt tttattgttt agcggatttt agtttttgtt    540
aggttcggtt cgttattttc gtttcgtttt tcgtcggttt ttgtttcgcg tttagggatt    600
ttttagtttt tttcgttcgc gtttttcgtt cgtttcggat attatggata agttttggtg    660
gtacgtagtt tggggatttt gtttcgtgtc gttgagtttg gcgtagatcg gtgagtgttc    720
gtcgtagttt gggtagtaag atgggtgcgg ggtgtttagc gcggattcgg cggtagtttt    780
ttcggttgag tcggttttgg gggattggag ttaagtgagt tgtttgcgaa gtgtattggg    840
tttcggaaag tagggttggg atttgcgtta aatcgttgga gaatgtgttt gtggaagtat    900
tatttggttg aaagaaaaag agaaagagaa gaaagtttgt tgggtaggtt gtcggcgcgt    960
agttttgggc gaggtcgtta gagttgtagt atatggtaga aagtaatcgt tttttcggat   1020
gcgtatagtc gttgtttgga ttaataggtt tttgtgttta agggttcgtt aagttttatc   1080
gggttgtgtt taggtagggt agagttgggc ggggtagaga ttggggttgg aatagggcga   1140
gtggt                                                               1145
```

<210> 70
<211> 1145
<212> DNA
<213> Artificial Sequence

<220>
<223> chemically treated genomic DNA (Homo sapiens)

<400> 70

```
gttattcgtt ttgttttagt tttagttttt gtttcgttta gttttgtttt gtttagatat     60
agttcggtgg ggtttggcga gtttttgggt ataggagttt gttagtttag ataacgattg    120
tgcgtattcg ggagaacggt tattttttgt tatgtgttgt agttttagcg atttcgttta    180
aaattgcgcg tcggtagttt gtttaataaa tttttttttt tttttttttt ttttttaatt    240
aaatggtgtt tttatagata tattttttaa cggtttagcg taaattttag ttttgttttt    300
cggagtttaa tgtatttcgt agatagttta tttgatttta gtttttttagg gtcgatttag    360
tcggaggggt tgtcgtcggg ttcgcgttga gtatttcgta tttattttgt tgtttaggtt    420
gcggcgggta tttatcgatt tgcgttaggt ttagcggtac gaggtagagt ttttaggttg    480
cgtgttatta aaatttgttt atggtgttcg gagcgaacgg agggcgcggg cgaaaggagt    540
tggaggattt ttgggcgcgg ggtaggggtc ggcggaggac gggacgagga tggcggatcg    600
aatttggtag aggttggggt tcgttgggta atgaggttgt ttcggaagtt ggttgtagtt    660
tttattcgag gttgaaaata gtgatttaag acggagggag ggagcgagcg aaggatatat    720
ttaagtaagg ggggttgtga ttaagtagtt cgtagaggtt ttaagaagta gtagtttttt    780
cgggcgggtt tttttgtttt cgtttcgtgt tttgttgagg ttgtaaataa tcggggttgt    840
tagggacgtg ggtcggggaa tttggagtgt cgcggggtgt ttgtgttttc gagttttatt    900
ggtttttgcg tttagagacg tattggtttt tttttttcgt tggtttcgcg gcgttaggat    960
agaggatgat cgaatcgtaa aattttgtat tattgggttt agtttttggt ttttttttt    1020
tcgttaaatt tttttgaatt cggttggagt aagattttttg ggttttatag gatgttggat   1080
attttgggag aggagttgga gagagggcga ggttttgttt tgggtggcgt agtgttttcg   1140
tgttt                                                               1145
```

<210> 71
<211> 2221
<212> DNA
<213> Artificial Sequence

<220>
<223> chemically treated genomic DNA (Homo sapiens)

<400> 71

```
taatgacgat gtgtagtagg ttttttagag ttaagttggc gattaagata ttgggatcgt      60
ttcgtatgta tttgtttttg tagataattt ttagaagtgt ggagttttcg atgattttta     120
gtacgaatat aaggtaggat ataatcgtgt tgatgtattt gaaagttttt ttgatttcga     180
tgggtttttg gtacggggga ggggagatgg tgcgtggcgg agattttgtc gttttgtttt     240
ttttaggtat tttcgtaggt gttaacgatc gcgttagatt ggcgttggaa tttttgggtt     300
ataaggtttt agtgggtggc gttattattt ttgcggtttg taaaagcgga gtggttttgt     360
taggcgggaa gttttttttt ttttttaga ttcgcgatag gtcgtaggta agaattagcg     420
taattagggc gcgttcgtat agatttggag gcggttgtat gttgttattt gttttagaag     480
gcgttcggtg gtcgtttcgt agttttagag tttagagata agtagaggaa ggaagatagg     540
atatttgggt tagttgttcg agttaagtcg ttgtaaacgt taatatcgtt cgtagttttt     600
tcgttagtat ttacgtttaa aagtaattta agtttgcgcg ttagtgggaa atttggcgtt     660
tatgattcgt tagcgcgggt cgaaattttt ttttgatgtt tttttagttg tttttttttt     720
ttcgcgtggt taggaggggt aaataatatg tagtggggcg gggcgttttg gaaggggtgt     780
gttagggagg gaatgatggg ggttttaggt aaagtttcga attttttac gtaacgggag     840
gaatatagat acgttttagt taagcgtgcg tgggaatcgc ggaattaagg tatttagagg     900
ttaagggttt gtgttaagtt ttgtattttg tttcgtaggg gaatttttat atttcgcgat     960
tgaattcgaa agattttttt cggcggatga aagtcgttat tttttggttg gttgagttat    1020
agttttcgta gcgcgtttag gagtgcgtcg gagattcgga aattcgtaga gattttttaa    1080
gttagtagga atttggaaat cgttgttttt tttaaacggt tttaaatatt cgcgtttttt    1140
tttttattta atttaatttt aatttgtttt ttttataggt ttaattttta ttttttttaaa   1200
attttaaatt agtaagttaa atcgaagttt tagaataagg tttaattaat gattttaatt    1260
taatattaag ttcgaatgtt tatttttcgg atttgataaa ttagatgtag agcgacgaat    1320
ggagattatt ttttcgggag gcggaattta gttgggtttt agtttgtttt gggagaggag    1380
tacgtttttt ttgagttgta ggcgggttcg gttttgacga aacgttgcga gaatgttaga    1440
tagtgtagtt tttatcgttt gttcggggtt tttgttgtta ttagataagt atttttattt    1500
atttattttt ttttattaat tatcgttaga tttttttcgc gtttttcgat ttcgttgtaa    1560
tttttttttt tgggcgatgt ttggatagta ttagtagtag ttgttcgagg gagggggggta   1620
gttttcgttc ggggataggt tttaaaaggt tgtttatttg ttagagtttt acgtttttta    1680
aatgaattta aattaagggt aagttattac gtattatgtg atattcggta gttttagatt    1740
agaataaggt tttgtattga acgtgataat tgaattttta atgttaagtg ttattttttt    1800
tttaggtaaa tgtttcgtcg ttgtgatatt taaggttggt ttgtgggcgt tgttttttgt    1860
tttgttttat tttgttttga agttttattt taagttaata ttaattttaa tacggaatag    1920
ttcgattttt tttttttagg ttttttatt gtttcgagag aggattataa gtatttttta     1980
ttttttttag tatagttttt tttttttttt tttgttatag tatcggtgtt aggtaatttt    2040
aggtattttt atattgtatt tatgatagat ttgatgttta tcggagatat gaaaataatt    2100
agaaagttgt tatgtaaatt taatatgata tttatttatt aggtagagta atataataag    2160
gtttttttat ttatttttga gttttatgtt tatcgtagag aggaaaataa tagcgattgg    2220
t                                                                   2221
```

<210> 72
<211> 2221
<212> DNA
<213> Artificial Sequence

<220>
<223> chemically treated genomic DNA (Homo sapiens)

<400> 72

```
attagtcgtt gttatttttt tttttgcggt aaatatggag tttaagaatg gatagaaaga      60
ttttattgta ttgttttatt tggtaaataa atattatgtt gaatttgtat ggtagttttt     120
tgattatttt tatgttttcg ataagtatta agtttgttat aggtatagta taaaaatgtt     180
tgagattgtt taatatcgat attgtggtag gaagagagga gaagagttgt attggggaaa     240
atgaggagta tttgtaattt tttttcgggg tagtgagagg gtttgggaag aaaagatcga     300
gttgtttcgt gttggggtta atgttggttt gaaatgaaat tttagggtaa aatgaagtag     360
```

```
agtaaagagt agcgtttata aattaatttt aaatattata gcggcggaat atttatttga     420
aagaaaagtg atatttggta ttaaaagttt aattattacg tttagtgtag agttttgttt     480
tagtttggag ttgtcgggtg ttatatggtg cgtgataatt tgtttttgat ttgggtttat     540
ttgaagagcg tagaatttta ataaataaat agttttttgg gatttgtttt cggacgagga     600
ttgttttttt ttttcgggta attattattg atgttgttta ggtatcgttt aaggggaaag     660
gttgtagcgg ggtcggaagg cgcgggagga gtttggcggt gattgatggg aagggatgaa     720
tgaataaaag tatttgtttg atggtagtag agatttcgag taaacggtgg aggttatatt     780
gtttggtatt ttcgtagcgt ttcgttagag tcggattcgt ttgtagttta agggaggcgt     840
gttttttttt tagagtaggt tggaatttag ttgggtttcg tttttcggga aggtggtttt     900
tattcgtcgt tttgtatttg gtttgttaga ttcgagaggt aaatattcgg gtttggtgtt     960
gaattaaaat tattgattga attttatttt ggggtttcgg tttggtttat tagtttggga    1020
ttttaaaaaa ataaaaatta agtttataga gagggtaaat taaaattagg ttgggtaaag    1080
gaaggagcgc gagtgtttga agtcgtttgg agggaatagc ggtttttaag tttttgttga    1140
tttgagaagt ttttgcgggt tttcgaattt tcggcgtatt tttgggcgcg ttgcgggagt    1200
tgtagtttag ttagttaggg agtagcggtt tttattcgtc gggaggagtt tttcgagttt    1260
aatcgcgggg tatagaggtt tttttgcggg gtaaaatgta gagtttgata taagtttttg    1320
gtttttaggt gttttaattt cgcggttttt acgtacgttt aattaagacg tgtttgtatt    1380
tttttcgtta cgtgaaagag ttcggagttt tgtttgggat ttttattatt ttttttttgg    1440
tatatttttt ttagaacgtt tcgttttatt gtatattatt tatttttttt ggttacgcgg    1500
gggaagaaaa atagttgaga gggtattagg aaggagtttc gattcgcgtt ggcgagttat    1560
gagcgttaag tttttttattg gcgcgtaaat ttgagttatt tttgagcgtg gatattggcg    1620
aagaggttgc gggcggtatt agcgtttgta gcgatttggt tcgggtagtt gatttaagtg    1680
ttttgttttt ttttttttgt ttgtttttag gttttgaaat tgcggagcgg ttatcggacg    1740
tttttggag taggtagtag tatgtagtcg tttttaagtt tgtgcggacg cgttttggtt    1800
gcgttggttt ttgtttgcgg tttgtcgcgg atttggggag aggagagagg tttttcgttt    1860
gatagggtta tttcgttttt gtaaatcgta gagataatga cgttatttat taagatttta    1920
tggtttaagg gttttaacgt tagtttggcg cggtcgttgg tatttgcgga ggtgtttaaa    1980
ggagatagga cggtaggatt ttcgttacgt attatttttt tttttcgtg ttaaggattt    2040
atcgagatta aggagatttt taaatatatt aatacggttg tgttttgttt tgtgttcgtg    2100
ttggggatta tcgggaattt tatattttg agaattattt ataagaataa gtgtatgcga     2160
aacggtttta atattttgat cgttagtttg gttttgggag atttgttgta tatcgttatt    2220
g                                                                     2221
```

<210> 73
<211> 2172
<212> DNA
<213> Artificial Sequence

<220>
<223> chemically treated genomic DNA (Homo sapiens)

<400> 73

```
atacggtagg tgtttaataa acgttagttt ttattggtat tattattatt attggcgtta      60
atgtatttat ttattgtagt gaggtagaac gtttttgtgg ttacgagtgt gattaggttg     120
atttattatt aagaggtgga ggatagggaa ggtcgtatag tgttttatta taaggaaaag     180
aggattagta cgtttttttt tgggaaggga aaaaaaaaga gaaggaaatt gaaagtatac     240
ggtcgatttt tttttaatta taaagtattg gatttacggg agggtatgcg gggggattgtg    300
taagtatata ggggtgtggg ggaggaaggt taatgaatag atttattgta aagggagggg     360
tgatgaggtt tatcgtttga ggggaagggg agtagaaaat tttatagatt tttgtattat     420
agtgagagga gagaaaagtt attatataga ttgatttaaa gtagagaggg aaaaggttat     480
ggattgagtt attgaaaata gggagatgta aagtattaaa ttaatttatt attaaaaagg     540
agagaatttt aagcgttaga gcgatagttt acgaaaaaga ggagataggg taatggattg     600
atagttaaag ggacgtgaat ttaaatatga agattgattt attataaaat ggtggtggtg     660
gtggtggggg ggggggtgg ttttgaaaaa agttatggat tgatgtattg ggggattgga      720
ggaggtagga atataaattt aggtagattt attgttaaag agggatgcgg aggagtgcgg     780
cgttacggat tgattcgtta cgatgtatag gaagggtgga ataaataagg ttacggacgg     840
gtttttgaa agttgaggtt aggagaaatt taaatattta gatatattta ttattaaggg      900
ggttgaatat tgttataggt tagtttttta taagtggggg tgggtggggg gcgggtgggg     960
gtaggggtag ggggaaagtt tatggacgag tttattttaa gttggcgtgg ggaaaaatat    1020
aagtacgtag atcgatttat tatataacgg gagggggagg ttaggttacg gttagattta    1080
```

ttataaggga cgaagggttt gagtaatgtt atacgtaggt ttattagtcg tggtggtggc 1140
ggcggggagt cgaatattag taggttatcg gttaagttat attttttgcg cgggggcggg 1200
aaggatacgt ggggttacgg attgggtttt ttattttta ggtttagagt ttatttgtgt 1260
gtagcgtggc ggtggcgttg gtaatgttgg tagtttcggg gggcgggggt tttatacgcg 1320
gttttatcgt tttttaggtt tgggtttcga ggcggcggtg gaggtggtgg tggcggtggc 1380
ggcggtagta tttagaagtc gtttttgcgt ttttttatag tttacgtggg ttcgaggagg 1440
aggagtttaa tgtttcgtcg ttcgttgatt ggttaaagcg ttattaatcg gtcgtaaggt 1500
ttcgaggggt tgggggacgg gtttttttt atagagttgt gtcgtgattg gcgtaagggg 1560
aaatgatgga acgtttacgg tttttggagt ttcgaggagg cgcgttcggt ttttacggtg 1620
ttttacgtcg attggtttaa ggattgacgg attgtgagta attgaaaagg ttcggggttt 1680
gtgtcggggg ggcgggggtt tgttttatat tgattggttt atatgggatt gatggaagat 1740
agtttttaag gacgggggtg ggtggttttg tttttttttg attggcggac gagggatttt 1800
tagattttttg ttaaaatatt aagggggcgg tgtcgtacgt tatgttgttt agcggaagta 1860
gggtttgtat agaaatgggc gtagtagtcg gcgggagtgg gtcggattgg gtttcgcgta 1920
ggcgggtagg gtagttttac ggttagataa gattatagag ttgggtaaag acgaatttag 1980
aatatagcgg aggtaggagg gtaggatggt tgttaggtat acgaaagagt attgagtggt 2040
agaaacgaaa tgtttttaga gggtagggtt gaattgcggt tagagttaaa aaggggagga 2100
atttggtttt gtattgattg gcggtggttg gatttaattt agaatagggg ttcgattagg 2160
gagagcggga tt 2172

<210> 74
<211> 2172
<212> DNA
<213> Artificial Sequence

<220>
<223> chemically treated genomic DNA (Homo sapiens)

<400> 74

gatttcgttt tttttagtcg ggtttttatt ttaggttgag tttagttatc gttaattaat 60
gtagagttag gttttttttt tttttaattt tggtcgtagt ttaattttgt tttttgaaag 120
tatttcgttt ttgttattta atgtttttc gtgtgtttga tagttatttt gttttttttat 180
tttcgttgtg ttttaaattc gtttttattt agttttatgg ttttgtttaa tcgtagagtt 240
gttttgttcg tttacgcgga gtttagttcg atttattttc gtcggttgtt acgtttattt 300
ttatataagt tttgttttcg ttgagtagta tggcgtgcga tatcgttttt ttggtgtttt 360
ggtaggggtt tagaagtttt tcgttcgtta attagagaaa aataggggta tttattttcg 420
tttttggggg ttgtttttta ttaattttat gtaagttaat tagtgtgagg tagattttcg 480
tttttcgat ataggtttcg agttttttta gttgtttata gttcgttagt ttttgagtta 540
atcggcgtgg agtatcgtga aggtcgaacg cgttttttcg ggattttagg ggtcgtgagc 600
gttttattat ttttttttac gttaattacg gtatagtttt gtagggaagg gttcgttttt 660
taatttttcg aggttttgcg gtcgattaat agcgttttgg ttaattagcg agcggcggga 720
tattgggttt tttttttcg ggtttacgtg agttgtaggg aaacgtaggg gcggttttta 780
ggtgttgtcg tcgttatcgt tattattatt tttatcgtcg tttcggaatt taggtttggg 840
gggcggtggg gtcgcgtatg gagttttcgt ttttcggagt tgttaatatt gttaacgtta 900
tcgttacgtt atatataggt gagttttggg tttggagggt ggagggttta gttcgtgatt 960
ttacgtattt ttttcgtttt cgcgtagagg atgtggtttg gtcggtggtt tgttggtgtt 1020
cgatttttcg tcgttattat tacggttggt ggatttgcgt gtggtattgt ttaagttttt 1080
cgttttttat agtggatttg atcgtggttt aattttttttt tttcgttgta taatggatcg 1140
gtttgcgtgt ttatgttttt ttttacgtta atttagggtg gattcgttta taggtttttt 1200
ttttattttt atttttattc gttttttatt tatttttatt tatagggagt tagtttgtga 1260
tagtgtttag tttttttaat agtaggtgta tttgagtgtt tggatttttt ttagttttaa 1320
tttttaggag attcgttcgt ggttttattt attttatttt ttttgtatat cgtagcgaat 1380
taattcgtgg cgtcgtattt tttcgtattt ttttttaata gtgagtttat ttgagtttgt 1440
atttttgttt tttttagttt tttaatgtat tagtttatgg ttttttttaa aattattttt 1500
ttttttttatt attattatta ttattttata gtggattagt ttttatgttt ggatttacgt 1560
tttttttagtt attagtttat tgttttgttt tttttttttc gtagattgtc gttttagcgt 1620
ttggggtttt tttttttta atagtgggtt agtttagtgt tttatatttt tttattttta 1680
gtagtttagt ttatagtttt tttttttttt gttttgaatt agtttgtgtg atggtttttt 1740
tttttttta ttgtggtgta ggagtttgta aggtttttttg tttttttttt ttttagacga 1800
tgagttttat tatttttttt tttgtagtgg atttgtttat tggttttttt tttttatatt 1860

tttgtatgtt tgtatagttt ttcgtatatt ttttcgtgag tttagtgttt tgtaattggg 1920
ggaagatcgg tcgtgtattt ttaatttttt tttttttttt tttttttttt tagaagagaa 1980
cgtgttgatt tttttttttt tgtgatggag tattgtacgg ttttttttgt tttttatttt 2040
ttaatagtgg gttagtttgg ttatattcgt aattataaag acgttttgtt ttattataat 2100
aagtgaatat attagcgtta gtgatgatag taatgttaat aggagttagc gtttattgag 2160
tatttgtcgt at 2172

<210> 75
<211> 3289
<212> DNA
<213> Artificial Sequence

<220>
<223> chemically treated genomic DNA (Homo sapiens)

<400> 75

tttgtgttta cgtattatgt gtgttttttc gttttttttt tttttatga gtgagaaaaa 60
aaagcgttta aatttttatt aatataaatt aatgatatat aatgatgaaa ttttgttttt 120
atttttgttt gtgataggga atgtaaaaat agtaagtggt ttagttttac gaattttcgt 180
tttttgtttt tttcgttttt gtcgggttgg atttttaaga atggaggtta gcgtatagtt 240
tcgcgcgggt cgtttagttt tcggattcgg cggatgatgt taggcgacgg gagcggtcgc 300
ggtcgggtcg gggaggtcgc ggtttagggg agttgggagg gagggtggtt tcgttaggtc 360
gacggcgcgt tcggtcgcgc ggcgttgttt ggagacggtt ttggcggcgt tgtgttgttg 420
taaatagtcg ttttttgtt attatttata gtaggatttt ttggttttcg ggcgcggcgg 480
ttggaggtag gtttgcggtt cggtttttcg cgcgtttcga attattcgtt cgtcggtttt 540
atttgtttc gttttttttt aggtgtttat cgcgggtttc gattttcggg ttcgaagagt 600
ggagaaggga agatcggggt tgtgcgggga tatgcgtttt cgcgttttgg aggtggttag 660
cgcgttgggg ttgagtttcg gtagcgtgat ttcggttgtt ttacgtagta gggtaggaga 720
ttggggggcg tggtatattt tggagtattt tgttttttta aagtttcgtg ttttaggacg 780
tggagtcgtt tttggggttt tagtagtcga ggtatttcgt ttaggcgtag ttggatattg 840
ttttttttagt tttcgttttt tattttttaa gttcgcgttg gaaaattatt cgttgcgggt 900
tttcgtaagt atagtttttt ggcgggatcg aattagtttt tagcgtagat ttgagttttt 960
cgtaggaagt atatttcgtt ttgttatttc gaattgatta ttttgtttat ataattatat 1020
ttcgtatttt ttatttttgg ggtttagttt agaatcgggt agatattttt tttaaatgtt 1080
ttcgtacgta ggttttgtat agtgtttatt tgttggtgtt ttagggattt gatagttttt 1140
ttaatatttt tatatatggt cgagaaaaat aaataaataa atgcgttgtt tttttaaaa 1200
aaataaataa ataaagtatt tagtatcgta aagtaggtta tcgtattttt ttattttgga 1260
ttttttattt tttgttttta aacgtaggaa tagtgttagt attgttcgag ttcgagggtt 1320
ggaggttagg ggatgaaggt ttgtttttac gttttgtatt gaattagggt tagaattggg 1380
gatgggggta ggggcgtatt ttttcgggag tcgaggttta agttttcggg gttttgtatt 1440
cgatgtcgtt ttttttattt ttgagtttta gaattgtttt tagttttcgt ataagggtaa 1500
aaaggcgttt tttgttttat ttttttcgat ttcgggaata agggttcgta ttgaattagg 1560
tgcgaatgtt tttttttatt ttgcgtcgtt ttcgtttttt tttttttagt cgcggttttc 1620
gttttttttc gtattgtatt ttcggtgttg gttgtagttc gcgagtagtt ttcgttaatt 1680
tttttttttt tatataggat gtttatatta ggatatttgc gttagtaggt ttttacggtt 1740
ttttttgta gttttggggg gagttatttt cgaaattttt tattttgggg ggtttacgag 1800
atttttgaga taggaattgc gaaatgttta cgagattagg atacgcgtta aggcgggggt 1860
agggagttgc gagcgttggg gacgtagtcg ggcggtcgta gaagcgttta ggttcgcgcg 1920
ttattttttt ggcgttatcg tggttgagtt cgtgacgttt atatttattt ataaaacgtt 1980
tgttataaaa gtagtggttg cggcgtttcg tattttaatc gtatttgtag cgagtatttg 2040
agaagttaag attgagtcgg cggtcgcggc gtagcgaacg agtagtgatc gtgtttttat 2100
ttagttttgt tttatagcgt ttatttgttt tcgtttttcg gtttttcgtt cggttttgtt 2160
taatcgttac gatgatgttt tcgggtttta acgtagatta cgaggcgtta ttttttcgtt 2220
gtagtagcgc gttttcggtc ggggatagtt ttttttatta ttatttattc gtagattttt 2280
tttttagtat gggttcgttt gttaacgcgt aggtaaggtt ggtttttcgt cgtcgcgggg 2340
tcgggggttt ggggtcgcgg aggaggagat atcgggcggg acgttttagt agatgagtag 2400
ggggtttttt tgtgtttgga gggaggttgt cgtggtcgga gcggtgtcgg ttcgggggtt 2460
cgggatttgt tttgagcgta cgtacgtttg ttatagtaag aattggtttt tttttcggga 2520
ggtaggttcg ttttgagtaa tttttggttt gtattttagg acggattttt gatattagtt 2580
ggagtagacg tgttttaagt ataaattcgt taattagagt ttggtttttt cggggaggtg 2640

```
gtagaaagcg gtaatttttt ttttttcggt agtttggagt acggaggagg gatgagggag    2700
gagggtgtag cgggcgggtg tgtaaggtag ttttattgat aaaaagcgag tttattttgg    2760
agatttcgga gcggcgtttg cgttagcgta gacgttaggg atatttataa taaatttttt    2820
tttaagtaag tgatgttgaa gggataacgg gaacgtagcg gtaggatgga agagataggt    2880
attgcgttgc ggaatgtttg ggaggaaaag gggagattt tttatttagg atgagggata    2940
tttaagatga aatgttcgtg gtaggatcgt tttttttat tgttgtatgc ggtattggga    3000
attcgtttta tttgtgttcg gaatttgttc gtttacgtcg gtttttttt tttgttttgt    3060
tttaggattt ttgtacggat ttggtcgttt ttagtgttaa ttttattttt acggttattg    3120
ttatttcgat tagttcggat ttgtagtggt tggtgtagtt cgttttcgtt tttttcgtgg    3180
ttttatcgta gattagagtt ttttattttt tcggagtttt cgtttttttc gttggggttt    3240
attttagggt tggcgttgtg aagattatga taggaggtcg agcgtagag               3289
```

<210> 76
<211> 3289
<212> DNA
<213> Artificial Sequence

<220>
<223> chemically treated genomic DNA (Homo sapiens)

<400> 76

```
ttttgcgttc ggttttttgt tatggttttt ataacgttag ttttggagta agttttagcg      60
gagggggcgg ggatttcgaa agggtgaggg gttttggttt gcgatggggt tacggaggag     120
acgagggcgg gttgtattag ttattgtagg ttcggattgg tcgagatggt agtgatcgtg     180
ggaatgaagt tggtattgga gacggttagg ttcgtgtaga agttttagaa taaaatagaa     240
ggggaaagtc gacgtgagcg agtaggtttc ggatataggt ggggcgagtt tttagtgtcg     300
tatgtagtag tgaagagaaa cgattttgtt acggatattt tattttaaat gtttttttatt    360
ttggatgaaa ggtttttttt tttttttttt aggtatttcg tagcgtagtg tttgtttttt     420
ttattttgtc gttgcgtttt cgttattttt ttagtattat ttgtttgaaa gggggtttgt     480
tataaatatt tttgacgttt gcgttgacgt aggcgtcgtt tcggagtttt tagaatgaat     540
tcgtttttta ttaatgaaat tgttttatat attcgttcgt tgtatttttt tttttttattt    600
tttttcgtg ttttaggttg tcgggggagg ggggattgtc gtttttttgtt atttttttcgg    660
agaagttagg ttttagttag cgagtttgtg tttgggatac gtttgtttta gttaatgtta     720
gagattcgtt ttggagtgta gattagaggt tgtttagaac gaatttgttt ttcgaagggg     780
gaattaattt ttattatggt aagcgtgcgt gcgtttagag taagtttcga gttttcgagt     840
cggtatcgtt tcggttacgg tagttttttt ttaggtataa gggagttttt tatttattta     900
ttggagcgtt tcgttcggtg tttttttttt cgcgatttta agttttcggt ttcgcggcga     960
cgggaagtta gttttatttg cgcgttgata ggcgagttta tgttggagaa ggagtttgcg    1020
ggtgagtggt agtaagagag gttattttcg gtcggggacg cgttgttgta gcgggaggat    1080
gacgtttcgt agtttgcgtt gaagttcgag aatattatcg tggcggttag gtaaagtcgg    1140
gcgagggggtc gaggggcgga gataggtggg cgttgtggag tagagttggg taggagtacg   1200
gttattgttc gttcgttgcg tcgcggtcgt cggtttagtt ttggtttttt agatgttcgt    1260
tgtagatgcg gttggagtac gaggcgtcgt agttattgtt tttataataa gcgttttatg    1320
aatgagtgta aacgttacgg gtttaattac ggtggcgtta gaggggtggc gcgcgggttt    1380
gggcgttttt gcggtcgttc ggttgcgttt ttagcgttcg tagtttttttg ttttcgtttt   1440
ggcgcgtgtt ttaatttcgt gagtatttcg tagtttttgt tttagaggtt tcgtgggttt    1500
tttaagatga ggggtttcgg ggatggtttt ttttagggtt atagggaaag gtcgtggaaa    1560
tttgttgacg tagatgtttt aatatggata ttttgtgtaa gggggggaggg attgacggga   1620
attgttcgcg ggttgtagtt aatatcgagg gtgtagtgcg gggggaggcg ggggtcgcgg    1680
ttgggggagg ggaggcggga acggcgtaga atgagagaga atattcgtat ttggtttaat    1740
gcggattttt gttttcgagg tcgggggggga tggggtagag agcgtttttt tatttttgta   1800
cggaaattga agatagtttt gaggtttaga gataggagaa acggtatcga gtataggatt    1860
tcgaggattt aagtttcggt tttcgaagga atgcgttttt atttttattt ttaattttag    1920
ttttaattta gtgtaaagcg tggaagtaga tttttatttt ttaattttta gttttcgggt    1980
tcgagtaata ttagtattgt ttttgcgttt ggaagtagaa agtggaggat ttaaaataag    2040
agaatacgat aatttatttt acgatattgg gtattttatt tatttatttt tttaaagaag    2100
atagcgtatt tatttattta tttttttcgg ttatgtgtgg gaatattaag gaaattgtta    2160
aatttttgag atattagtag ataaatattg tgtaaaattt acgtgcgaag atatttgaag    2220
ggggtgtttg ttcggttttg agttgggttt taggggtagg gagtgcgagg tgtggttatg    2280
tgggtagggt ggttagttcg ggatgataag gcggggtgtg ttttttgcgg ggaatttaaa    2340
```

tttgcgttga gggttggttc ggtttcgtta ggaagttgtg tttacgggag ttcgtagcgg 2400
gtgatttttt agcgcggatt tggagggtgg aggacggggg ttggaaggat agtgtttagt 2460
tgcgtttggg cggaatattt cgattgttgg gattttagga gcggttttac gttttggaat 2520
acggggtttt ggggaggtaa ggtgttttag agtgtgttac gtttttttaat tttttgtttt 2580
gttgcgtagg atagtcgggg ttacgttgtc ggggtttagt tttagcgcgt tggttatttt 2640
tagggcgcga agacgtatgt tttcgtatag tttcggtttt ttttttttta tttttcgggt 2700
tcgggggtcg gggttcgcgg tgggtatttg gagaggggcg aggtaggatg aggtcggcgg 2760
gcggatagtt cggggcgcgc ggggagtcgg atcgtagatt tgtttttagt cgtcgcgttc 2820
gggagttagg agattttgtt atagatagta atagggaagc ggttgtttat agtaatatag 2880
cgtcgttagg gtcgttttta ggtaacgtcg cgcggtcggg cgcgtcgtcg gtttggcgag 2940
gttatttttt tttttagttt ttttgggtcg cggtttttttc ggttcggtcg cggtcgtttt 3000
cgtcgtttga tattattcgt cgggttcggg ggttgagcgg ttcgcgcgag gttgtgcgtt 3060
aatttttatt tttagagatt taattcggta ggagcgggga gggtaggagg cggggattcg 3120
tggaattggg ttatttgtta tttttgtatt ttttgttata ggtagaggtg aaaatagaat 3180
tttattattg tatgttattg gtttatgttg gtgggaattt aggcgttttt ttttttttatt 3240
tataaaaagg gagggaaacg gaaaaatata tataatacgt aaatataga 3289

<210> 77
<211> 3812
<212> DNA
<213> Artificial Sequence

<220>
<223> chemically treated genomic DNA (Homo sapiens)

<400> 77

agcgagatcg tatggtttta tttataagtg ggagttgaat aatgagaata tatggttata 60
tggcggcgat taatatatat tggtgtttgt tgagcggggt gttggggagg gagagtatta 120
ggaagaatag ttaagggata ttgggtttaa tatttgggtg atgggatgat ttgtatagta 180
aattattatg gcgtatatat ttatgtaata aatttgtata ttttttatat gtattttaga 240
attttaaata aaagttggac ggttaggcgt ggtggtttac gtttgtaatt ttagtatttt 300
gggaagtcga ggcgtgtaga ttatttaagg ttaggagttc gagattagtt cggttaatat 360
ggtgaaattt cgtttttatt aaaaatataa aaattagtta gatgtggtac gtatttataa 420
ttttatttat tcgggaggtt gaagtagaat tgtttgaatt cgagaggcgg aggttgtagt 480
gagtcgtcga gatcgcgtta ttgtatttta gtttgggtta tagcgtgaga ttacgttata 540
aaataaaata aaataatata aaataaaata aaataaaata aaataaaata aaataaaata 600
aaataaaata aaataaaaaa ataaaataaa ataaaataaa ataaagtaat tttttttttt 660
ttaagcggtt tttatttttt tttttttgttt tgtgaagcgg gtgtgtaagt ttcgggatcg 720
tagcggtttt agggaatttt ttttcgcgat gtttcggcgc gttagttcgt tgcgtatatt 780
tcgttgcggt tttttttttg ttgtttgttt attttttagg tttcgttggg gatttgggaa 840
agagggaaag gtttttttcgg ttagttgcgc ggcgatttcg gggattttag ggcgtttttt 900
tgcggtcgac gttcggggtg tagcggtcgt cggggttggg gtcggcggga gttcgcggga 960
ttttttagaa gagcggtcgg cgtcgtgatt tagtattggg gcggagcggg gcgggattat 1020
ttttataagg ttcggaggtc gcgaggtttt cgttggagtt tcgtcgtcgt agttttcgtt 1080
attagtgagt acgcgcggtt cgcgttttcg gggatggggt ttagagtttt tagtatgggg 1140
ttaattcgta gtattaggtt cgggttttcg gtagggtttt tcgtttattt cgagattcgg 1200
gacgggggtt taggggattt aggacgtttt tagtgtcgtt agcggttttt agggggttcg 1260
gagcgtttcg gggagggatg ggatttcggg ggcggggagg gggggtagat tgcgtttatc 1320
gcgttttggt atttttttttc gggttttagt aaattttttt ttgttcgttg tagtgtcgtt 1380
ttatatcgtg gtttattttt tagttcgagg taggagtatg tgtttggtag ggaagggagg 1440
taggggttgg ggttgtagtt tatagttttt cgtttattcg gagagattcg aatttttttta 1500
tttttttcgtc gtgtggtttt tatttcgggt ttttttttttg tttttcgttt ttttcgttat 1560
gtttgttttt cgttttagtg ttgtgtgaaa ttttcggagg aatttgtttt tttgtttttt 1620
ttttgtattt ttgatttttt ttcgggttgt tgcgaggcgg agtcggttcg gtttttatat 1680
ttcgtatttt tttttttttcg taggtcgttg cgcggttttg cgtatgttgt tggtagatta 1740
gggttagagt tggaaggagg aggtggtgat cgtggagacg tggtaggagg gtttatttaa 1800
agttttttgc gtaagtgatt atgttcgggt aaggggaggg ggtgttgggt tttagggggt 1860
tgtgattagg atcgggggac gtttaagttt agtgtttttt tttgagttat gtttttttta 1920
atagttatac gggtagtttt ttaagtttta ggacggagat tttattttgt attagtttaa 1980
tattattttg cgttatttgg gtcgtatttt tggtgagttt tgaattttta agtttagggt 2040

```
aggtatgggt aagtttttgt tttcggagtt tttttgttta aattagttgt ttcgtagttt    2100
tttggagtgg aggaaattga gatttattga ggttacgtag tttgtttaag gttaagtttg    2160
ggtgtttgta atttttgttt tgtgttaggt tgttttttag gtgttaggtg agttttgagt    2220
atttgttgtg tggtagtttt ttattttttt acgtatattt tttttttttt tttttaggtt    2280
ggggtttata gatagttttt tggttggttt atttttagtg attgtgtgtt gattaggcgt    2340
ttagttacgc ggtttgtttt tttttattta attttagggt tttatgggaa ggattagtag    2400
gaggtagttt tggtggatat ggtgaatgac ggcgtggagg attttcgttg taaatatatt    2460
tttttatttt atattaatta tgtgagtatt tgtattaggg ttgggtattg ggggttgaat    2520
aaagaaaggg gtttttttgtg tttttatttt ttttattttt taggtggttt gggttgattt    2580
ttttttgggt tagggtgtag gggttgggtt agttttgggt taggggttta ggggtttggg    2640
ataagatata atttgtattt ttattgtttg ggatattaat tagttaagta acgggttatg    2700
ggggcgagtg taaggataga gatttttagt aattggtggt ttttgatttt ttggggtggc    2760
gagggttttt tggagtagtt agaggtggag gaggatttgt cgttagtttt tggatggagg    2820
tgttggtatt tttagttgag gaaaatatgt agatatagag tatatttggg gatttgggat    2880
tagtttagta gaggtagcgt gtgtgcgcgt gcgtgtgtat gtgtgtgcgt gtgtgtgtgt    2940
acgtttgtat ttgtgtcggg tgggtaagga gatagagatg ggcgggtagt aggtttaggt    3000
ttcgaaggtt ttgaatttat tggtttggag ttttttaagg gtaatggggg ttattgagaa    3060
gtttgaatag ggttgtgttt gaatgtgagg tttagaagga ttttttagag aagttagttt    3120
taaagttttt gtaattattt ggtgagagaa tttagtaagg atggataggt agaatggaat    3180
agagatgagt tggtagttga agtggatagg atttggtatt agtttggttg tggggagtaa    3240
gtagaggaga atttgggatt ttggtgtttg gtttggggta gacgggggtg ttttaggggt    3300
tgggagggat gagagtagga tgatatatgg tggtgtttgg taggaggcgg gtaaggatga    3360
ttatgtgaag gtattgttcg ggtaattgaa gtttttttgag attttgttgt tttagaatta    3420
gggaggtaag atttttattg tgggagatta ggtgagtatt tggttttatg ttgttttttt    3480
ttcgttattt tttgttttta gatggatata ggtgtgagtt atttgtttag taaagtagag    3540
tagatttagg ggatgggttt aggtttttttg tttttaattt ttttagtttg ttttcgttgg    3600
ttgagttttt ggtttttttg ttttgtagat ttttttcgtt gattataatt tgttggattt    3660
gttgttgatt tatgaggttt tagtttttgg ttgtttggat gcgttttttt tgtttttagt    3720
atatgtgggg cgttttagtg ttcggtttaa gtttaaggtt tttttggttt tttttgagta    3780
cgtgaatttt tttattaatg gtaacgggaa at                                  3812
```

<210> 78
<211> 3812
<212> DNA
<213> Artificial Sequence

<220>
<223> chemically treated genomic DNA (Homo sapiens)

<400> 78

```
gtttttcgtt gttattgatg gggaggttta cgtatttagg ggaggttagg aaggttttga      60
gtttgggtcg ggtattgagg cgttttatat atgttgagag tagggggaac gtatttaggt     120
agttaggggt taggatttta tggattagta gtaagtttag taggttgtag ttagcgaagg     180
agatttgtag ggtagggggg ttagggattt agttagcggg agtaggttgg aggaattggg     240
ggtagagggt ttaagtttat tttttaggtt tgttttgttt tgttaaataa atggtttata     300
tttgtgttta tttggaagta gagggtggcg aggaaggaat agtatggggt tagatgttta     360
tttggttttt tataatgaag gttttgtttt tttggttttg ggatagtagg gttttaaaag     420
gttttagttg ttcgggtagt gtttttatat agttattttt gttcgttttt tgttagatat     480
tattatgtat tattttattt ttattttttt tagtttttga gatattttcg tttgttttag     540
gttagatatt agagttttag attttttttt gtttgttttt tataattagg ttagtattaa     600
atttgtttta ttttagttgt taatttattt ttattttatt ttgtttgttt atttttgttg     660
ggttttttta ttagatgatt gtaaaagttt tagagttggt ttttttggag gattttttta     720
gattttatat ttagatatag ttttgtttag atttttttaat ggtttttattt gtttttagga     780
gattttaaat tagtgggttt aaggttttcg ggatttgggt ttattgttcg tttattttta     840
tttttttatt tattcgatat aaatgtaagc gtatatatat atacgtatat atatgtatac     900
gtacgcgtat atacgttgtt tttgttgaat tggttttagg tttttaaatg tgttttgtgt     960
ttgtatattt tttttagtta aaagtgttag tatttttatt tagaaattgg cgataaattt    1020
tttttttattt ttggttattt taggaagttt tcgttatttt aggagattag aaattattag    1080
ttgttggagg tttttgtttt tgtattcgtt tttatgattc gttatttggt tggttgatgt    1140
tttaggtaat aagggtgtag gttgtgtttt gttttaggtt tttgggtttt tggtttagag    1200
```

ttgatttagt ttttgtattt tgatttaaga aggggttagt ttaagttatt tgaggggtaa 1260
ggggggtgag ggtataagaa gttttttttt ttgtttagtt tttagtgttt aattttggtg 1320
tagatgttta tatagttggt gtagatgagg gagatgtatt tgtagcggag gtttttttacg 1380
tcgttattta ttatgtttat tagggttgtt ttttgttggt tttttttata gagttttggg 1440
gttgggtgga ggggagtagg tcgcgtgatt gggcgtttga ttaatatata gttattgggg 1500
atgggttaat tagggggttg tttgtgagtt ttagtttggg aggaggggaa gaggatgtgc 1560
gtggaaggat gagagattgt tatatagtag gtgtttagag tttatttgat atttgggagg 1620
tagtttggta tagggtaagg attgtaggta tttaggtttg attttgggta aattacgtaa 1680
ttttagtggg ttttagtttt ttttatttta gagggttgcg gggtagttga tttaaataaa 1740
agggtttcgg gggtagaggt ttgtttatgt ttgttttgga tttggaggtt taagatttat 1800
taagggtgcg gtttaggtga cgtaggatgg tattggattg gtatagggtg aggttttcgt 1860
tttggaattt ggggagttgt tcgtatagtt gttgggggag gtatggttta gggaggggta 1920
ttgagtttgg gcgtttttcg attttagtta tagtttttta aggtttagta tttttttttt 1980
ttgttcgggt atggttattt acgtaggagg ttttgagtga gttttttttgt tacgttttta 2040
cggttattat tttttttttt tagttttggt tttgatttgt tagtagtatg cgtagggtcg 2100
cgtagcggtt tgcggggagg gagaagtacg agatgtgggg atcgggtcga tttcgtttcg 2160
tagtaattcg gggaggggtt aggagtgtag ggagggaata gggaaatagg ttttttcgaa 2220
gattttatat aatattgggg cggggagtag gtatggcggg agaggcgggg aataggaagg 2280
aggttcgggg taaaagttat acgacggagg gataaggggg ttcggatttt ttcgggtggg 2340
cgaggggttg tgggttgtag ttttagtttt tgtttttttt ttttgttaga tatatgtttt 2400
tatttcgaat tgggaaatag attacggtgt agggcggtat tgtagcgaat aaagaaaagt 2460
ttgttggagt tcgggggagg atgttaaggc gcggtgagcg tagtttgttt tttttttttcg 2520
ttttcggggt tttatttttt ttcgaggcgt ttcgggtttt ttgaaagtcg ttaacggtat 2580
tggggacgtt ttgggttttt taggttttcg tttcgggttt cgaggtgggc gaggagtttt 2640
gtcgggagtt cgggtttgat gttgcgggtt ggttttatgt tgggagtttt gagttttatt 2700
ttcggggacg cgggtcgcgc gtatttattg gtggcgaaga ttgcggcggc gaaattttag 2760
cgaaggtttc gcggttttcg agttttataa gggtggtttc gtttcgtttc gttttagtgt 2820
tgagttacgg cgtcggtcgt ttttttggag ggtttcgcgg attttcgtcg gttttagttt 2880
cggcggtcgt tgtatttcgg gcgtcggtcg tagaggggcg ttttggagtt ttcggagtcg 2940
tcgcgtagtt ggtcggggaa gttttttttt tttttttagg tttttagcgg ggtttaggga 3000
gtaaatagat agtaggaaga ggatcgtagc gaagtgtgcg tagcgaattg gcgcgtcggg 3060
atatcgcggg gggaaatttt ttaagatcgt tgcgatttcg gagtttgtat attcgtttta 3120
tagggtaggg gagaggggtg gaggtcgttt agaggaaagg aaattgtttt attttatttt 3180
attttatttt attttttttat tttattttat tttattttat tttattttat tttattttat 3240
tttattttat tttgtgttat tttattttat tttatgacgt agttttacgt tgtggtttag 3300
gttggagtgt agtggcgcga tttcggcggt ttattgtaat tttcgttttt cgggtttaag 3360
taattttgtt ttagtttttc gagtaggtgg aattataggt gcgtgttata tttggttgat 3420
ttttgtattt ttagtagaga cggggtttta ttatgttggt cgggttggtt tcgaattttt 3480
gattttaggt gatttgtacg tttcggtttt ttaaagtgtt gggattatag gcgtgagtta 3540
ttacgtttgg tcgtttaatt tttatttgaa gttttggggt atatgtagag gatgtgtagg 3600
tttgttatat aggtgtgtgc gttatgatgg tttgttgtat agattatttt attatttagg 3660
tattaagttt agtatttttt agttattttt tttggtattt ttttttttta gtatttcgtt 3720
taataggtat tagtgtgtgt tgatcgtcgt tatgtgatta tgtgttttta ttgtttagtt 3780
tttatttata agtgagatta tgcggtttcg tt 3812

<210> 79
<211> 1267
<212> DNA
<213> Artificial Sequence

<220>
<223> chemically treated genomic DNA (Homo sapiens)

<400> 79

atgaatttcg aaagttggat tgtagagtaa acgagtgtag ttaatttagt taggggtttg 60
taagagggag aaagagaaaa agattgtgga atggaaagtt ttttaattta agtttttttt 120
aaggggtagt tattttttgt tttatagttt agggtttgta tgtgtttttt ttggagtgga 180
aaaatatata agttataagg aatttaatag atagaaaggc gtatagagga atttaaagtg 240
tgggttgggg ggcgaggcgg tgggcgggag gcgagcgggc gtaggcggaa tatcgttttt 300
taagttaagt cgtcgtaaat aaaaaggcgt aaagggagag aagttggtgt ttaacgtgag 360

ttaggagtag cgtttcggtt ttttttttgt ttattttaaa agtatttttt gtattgtttt 420
taaggtgaga aataggaaag aaaacgtcgg tttgtgcgtt cgttgtttgt ttttttggtt 480
gtttgttttt gtagggttgt tgggagtttt taagttttgt gagaattttg ggagttggtg 540
atgttagatt agttgggtta tttgaaggtt agtagttcgg gtagggttta tcgaaagttt 600
attcgtatat attaggtaat ttaatttttt attttgtgtg atagaagtag taggaagtga 660
gttgtttaga ggtaggaggg tttatttttt gttaaagggg ggattagaat ttttttatgc 720
gagttgtttg aggattggga tgtcgagaac gcgagcgatt cgagtagggt ttgtttgggt 780
atcgtcgggg taggattcgg aacgtattcg gaaggttttt tgtaagtatt tatttggaag 840
gagaatttgg gatttttttg ggaatttttc gtttcggttg gattggtcga gtaagtttgg 900
aaaatggtaa atgattattt ggattaatta taggttttta gttggtttgt ttgttataat 960
ttatgattcg gggttgggaa aaagattaat agtttacgtg ttaaaaaagg ggtagagttt 1020
gatggagttg ggtggatttt tttatgttat ttgtttttat atttagagga taagtatttt 1080
tgtagatatt tagtgtaagg gagattatgt ttgattgtat ggatgttttg ttagtgagtt 1140
ttgggtaaat tttggatttt tatattgcga gttcgttttt ttgtatgttt taggagaaag 1200
tttttaaagt atgttttagt ggattgattt aaatcgaatg gtagtatcgg tatattgttt 1260
aatgtag 1267

<210> 80
<211> 1267
<212> DNA
<213> Artificial Sequence

<220>
<223> chemically treated genomic DNA (Homo sapiens)

<400> 80

ttatattgag tagtgtgtcg atgttgttat tcggtttggg ttaatttatt gaagtatgtt 60
ttgagagttt ttttttggag tatgtaggaa gacggattcg tagtgtagaa atttaggatt 120
tgtttaggat ttattgatag aatatttata tagttaaata tgattttttt tgtattgaat 180
gtttgtaaaa gtgtttattt tttaggtgtg gaggtaaatg gtatagaaaa gtttatttaa 240
ttttattaaa ttttgttttt tttttggtac gtaggttgtt ggtttttttt ttagtttcga 300
attatgaatt atgatagata agttagttaa aagtttgtaa ttgatttaaa tgattattta 360
ttatttttta ggtttgttcg gttaatttag tcggggcggg gggtttttag aaagatttta 420
agtttttttt ttaagtaaat gtttgtaaaa agtttttcga atgcgtttcg gattttattt 480
cgacggtgtt tagataaatt ttgttcggat cgttcgcgtt ttcggtattt tagtttttaa 540
atagttcgta tgggggaatt ttggtttttt ttttggtaaa gaatagattt ttttgttttt 600
gaatagttta ttttttatta tttttgttat atagaatgaa agattgaatt gtttaatata 660
tgcgagtgaa ttttcggtga attttattcg ggttgttaat ttttaaatga tttaattagt 720
ttgatattat taatttttag gatttttata gagtttaaaa atttttagta gttttgtaaa 780
agtagatagt tagagaggta ggtagcgagc gtataagtcg gcgttttttt ttttattttt 840
tattttaaaa ataatataag aagtgttttt aaaatgagta ggggaggagt cgggacgttg 900
tttttggttt acgttgagta ttaatttttt tttttttacg ttttttttatt tgcggcggtt 960
tagtttggaa aacggtgttt cgtttgcgtt cgttcgtttt tcgtttatcg tttcgttttt 1020
tagtttatat tttaaatttt tttgtgcgtt tttttgtttg ttaaattttt tataatttat 1080
gtattttttt attttagaaa aagtatatgt aagttttaaa ttgtagaata gagaatggtt 1140
atttttttggg aaaggtttgg gttgggaaat tttttatttt atagtttttt tttttttttt 1200
ttttttgtaa gtttttggtt gaattgattg tattcgtttg ttttgtaatt tagttttcga 1260
gatttat 1267

<210> 81
<211> 1266
<212> DNA
<213> Artificial Sequence

<220>
<223> chemically treated genomic DNA (Homo sapiens)

<400> 81

agttacgtag ttttttttatt cggagagaag tcggagtatt gggatagatt taggaggtta 60

gagcggaaat tttgttcggg tgcgtggaat cggagttttc ggtgcgcggc gttaggtatt 120
tatttgtatg gttgagcgtt aggatcgcgt atagtagtag ggcgcgggcg agtatcgtag 180
cggcgggtag ggcgcggcgc gggggtaggt tttgttgttt gagggcgttt ggttgtggag 240
ttgaaggagg cgttgttgag gagtttttgg acgtgttttt gacgtttatt gtaagtcgta 300
tgataattgg tcgttaatcg agagaatttt tttttttata agattgaaaa ttaagtttat 360
gtgacgaaat gattgttttt tttcgttttt tcgtattttt tataaatttt tttttttttt 420
tttttaaaa aaattgcgta agttcggtgg gggtagggtt ttttatttac ggaaatgaga 480
aaatcggaaa tttaggaagt tgttttaatt tgggagtaga gggggtagtt tttatttttt 540
tgtttgattt tttttttttt ttttcgagtt ttatcgtttt aggcgtatag gttttcgtta 600
gatgtttttt tttttttcgta gtttttgttc gagcgttttc gagagttagt tttggattga 660
tcgttttgga tgggatatcg ggggagggta gaaggatatt tggttttttt tttaggaatt 720
tgagcggttt tgaggttcgg gggcgtaggg aatttttttt ttcgtcgtcg tcgtcgtgtt 780
tggtttgtac gtttttagag ggtcgaggaa gttacgtcgg gatagattgg ggcgagtaag 840
gttaagaaag gttgatatgt tttatgtttt agtgacgacg tttaataggt tgtatatttg 900
ttttatatgt agtatatata tatatagttt ttttaaaaaat ttttattttg tggaatgaaa 960
tagttatttt tagtgtatat agttgtaatt tatttttgta gttaagttgt ttttaataga 1020
agaaatattg tttttcgtat ttttattttt tttttgtttt ttggaaagag aggcgggaaa 1080
ggtaaatttt ttttataata ttggttttaa gtagttattt tgtaaatagt taatgtgagt 1140
tttacgggtt attaatataa agttttttgt tttttgatgg ataaaggaag cggcgatggt 1200
tagaatttgt agggacgtta aatgtttaaa acgtatgttt taaggtattt tttttttttga 1260
tgcgtg 1266

<210> 82
<211> 1266
<212> DNA
<213> Artificial Sequence

<220>
<223> chemically treated genomic DNA (Homo sapiens)

<400> 82

tacgtattag ggagagaaat gttttaaggt atacgttttg gatatttagc gtttttgtaa 60
attttggtta tcgtcgtttt ttttgtttat tagaaggtag gaaattttat attggtgatt 120
cgtggagttt atattaatta tttataggt aattgtttag gattagtatt atgaggagaa 180
tttattttt tcgtttttt ttttaagaaa taaggagggg gtgaaggtac ggagaatagt 240
atttttttg ttgaaagtaa tttagttata aagataaatt atagttatgt atattgaagg 300
tagttatttt attttataaa ataagagttt tttaaaaagt tatgtatgta tgtgttgtat 360
atagagtaga tatatagttt attaagcgtc gttattaaaa tataaaatat gttagttttt 420
tttaatttta ttcgttttag tttgtttcga cgtgattttt tcgattttt aaagacgtat 480
agattagata cggcggcggc ggcgggagag gggatttttt gcgttttcgg attttagggt 540
cgtttagatt tttggagagg aagttaagtg ttttttgtt ttttcggt attttattta 600
aggcgattag tttagaattg gttttcggaa gcgttcgggt aaagattgcg aagaagaaaa 660
gatatttggc ggaaatttgt gcgtttgggg cggtggaatt cggggaggag agggagggat 720
tagataggag agtggggatt attttttttg tttttaaatt ggggtagttt tttgggtttt 780
cgattttttt attttcgtgg gtaaaaaatt ttgtttttat cgggtttacg taattttttt 840
aaggggagag gagggaaaaa tttgtggggg gtacgaaaag gcggaaagaa atagttattt 900
cgttatatgg gtttggtttt tagttttata aaaaggaagg ttttttcggt tagcgattaa 960
ttgttatacg atttgtagtg agcgttagga gtacgtttag gaatttttta gtagcgtttt 1020
ttttagtttt atagttagac gtttttagat agtaaagttt attttcgcgt cgcgttttgt 1080
tcgtcgttgc gatgttcgtt cgcgttttgt tgttgtgcgc ggttttggcg tttagttata 1140
taggtgagta tttggcgtcg cgtatcgggg atttcggttt tacgtattcg ggtagagttt 1200
tcgttttgat tttttgggtt tattttagta tttcgatttt ttttcgaata gagaagttac 1260
gtgatt 1266

<210> 83
<211> 4511
<212> DNA
<213> Artificial Sequence

<220>

<223> chemically treated genomic DNA (Homo sapiens)

<400> 83

```
ggagtattcg aacgcggaaa tcgaggtgtt agtttaaatt gttcggtcgg ttttagttat      60
agttggataa tgttcggttt aggtttatta taagttatat agttgttttt ttcgtgttta     120
atttgtttgt gatagaaatt aaggggggttt cggtatttag tatttaggcg gtggaatcgg    180
ggttttacgt acggtttcgc gggtaggttt tcggttagga ttcgcgggga gttacgtagt     240
taggagggtg gggttgttta tcgatttagg acgcggtaac ggatcgggga gggcggagtt     300
ttagcgatcg tttttttttt cgttcgtcgg tatttttggg tttttatttg gtttcggcgc     360
ggtttgcgag ttagcgaggt tcgcgcggtg aagtattgtt cgagtttcga gttcgagttt     420
ttttggttgt agtagttatt gttcgtcgtg ttgttttagg ttatttcgaa agaaggcgtt     480
ttcgtttcgt ttatagtcgt attcgttcgt tttttagttt tgcgcgttcg tagtcgttaa     540
ttatcgtttc ggtcgcgtgc gtgcgtgtac gcgtgttagt gtgcgcgtgc gttcgggtta     600
gagtcgcgtc gtaatcgtta agattgaaac gtagatcgtc gggatttagt ttttgtttta     660
ttggggtagg aacgtcgggg cggggatacg tacgtttcgt ttttaggaat gattttatcg     720
tttcggagtt ttatttatag attttattta ttatagggaa cgggggcggg tgttagcgtt     780
cgggtaagcg tataagagtg gtttttggtc ggaggcgagg gcgggaaggt gcgggaagtg     840
cgcgtgcgcg gagtttgggt tagtttgggt tcgggttcgt ttgtagcggg tggagtattt     900
gcggagtcgg taatttaggt ttttttttta gttttcgcgt agaattagtt tttttgtgtc     960
gtcgggaaat cggtaattag aacgtttttt gcgcgcggta tttaggtagt tttcgagaat    1020
gtttgtattg tggtttgttt attttcgttt tttttatacg ttttcggttt cgcgtttatt    1080
acgttcgtgt ttcgttttat cgcgggtttt tagtttaggt ttcggggttc gtaatagttt    1140
aggtagacga gcgcgcggta gcggtagtgg taggtgaatt tgtaatttgt agagaggttt    1200
ggcggtgagg cggaggagtt ttaggtcggg gaaatgtttc ggagattgaa gggaagtttt    1260
agggagaggg tcgttgttcg ttaggtttcg taggttcgat ttattttagt gggttatttt    1320
atattgttac gcggatttta atgttggtta tttgggcgtt tggaaatcgg tcggaaggtt    1380
ataggtagag aggtttgttt aatagttgga tttttatcgt ttagtataga attttttttt    1440
tttttattgg taattaaaaa aataataata aaaaattgcg ttttgttttt gttatttagg    1500
ttggagtgta atggcgcgat ttcggtttat cgtaatttttc gtttttttggg tttaagcgat  1560
tttttttgttt tagtttttttg agtatttagg attataggcg ttcgttatta tgtttagtta  1620
atttttgtat ttttagtaga gacggggttt tattatgtta gttaggttgg ttttaaattt    1680
ttgattttag gtgatttatt cgtttcggtt ttttaaagtg ttgggattat aggtttgagt    1740
tatcgtattc ggttttttat tgggaacgta tatggaatat atttgtttat ttatttgaag    1800
gaaaaattaa atatttttaa tttacgtttg ttttgtggtt gttatttgtt tttatttttt    1860
ttagattaag atattggttt ttatatattt taatttttcg tttttatttt tttttttatt    1920
tattttagtt aggtttgttt tttttttttag gaaattgttc gggatagggt ttttagcgat   1980
ttgtgtatta ttaaatggaa tttagtgttt tatttttttat ttttattttt ttagtattat   2040
ttgaagtttg ttttttttttga tttttagggg ttatattttt ttagtttttt ttttattttt  2100
tgtagttttt gtttagtttt tttgtagatt ttgatttaat ttttatattt tacgatgaag    2160
tttgggttta gttttgatta ttggtttggt ttgtttatat ttatttgttt tagatttacg    2220
gttgaaatat tgatttaaat ttttagatta gatttttcgt gttagtattt ttattaggat    2280
gtttaaaaga cgttttaagt gaatatggtt aaaatttaat tttttttttt ttagttttat    2340
tgttatattt gtttagtttt ttttttgtag taaaaatggt tattaggttt ttagttattg    2400
gagataaaag tttaaattta tttttgattt ttttttgtt tttatttttg ataaatatgt     2460
ttaaattatt ttgtttttta tttttatggt tattttattt ttttttgaga acgttgtaat    2520
gttttagttt tgtttttttt tttttttttt tttttttgga gatagagttt tattttgtcg    2580
ttaaggttgg agggtagtgg tacgatttcg gtttattgta attttcgttt tttgtgttta    2640
agtaattttt ttattttagt ttttcgagta gttgggatta taggtattcg ttattacgtt    2700
tggtttattt tttttattt tttagtagat atgaggtttt attatgttgg ttaggttggt     2760
tttgaatttt tgattttagg tgatttattc gttttcgttt ttcgaagtgt tgggattata    2820
ggtatgagtt atcgcgttcg gttttttgtt tattttttgt attttgttat aattttgtgt    2880
ttttttttagt tgaatttgtg atgtttttttt gtatcggatg agagggtttt tatgtatata  2940
tagatttggg atattattta tttataagtt tttaaatagg ttagagtagt gatgtttaat    3000
ttagatttta tgttataata atttggggag tttttaaaat ttattgatgt ttagggttta    3060
tttttagtag ttgatttaat aggtttgcgg tgggatttag gttagcgggg aggattgtaa    3120
aagtattttt ggtgatttta gttggtgttt atttagggga gagtaatttt tgtttgttgg    3180
cgatttttag ggtgtagaa ggattgttgg gtgtgtggtt gcgtgtatat tttagtattt     3240
gatttattgg gttagaaaag ggtgtttgtt aaataaagat ttaataaaat ttttgtttgt    3300
agggggttta ttaaaggttt taaatttttt taggtttttt tttataggtg gtaatttttt    3360
tttattttaa aggttttgga gggggttatg agtgtttgag aagaggtaag tttgggaaga    3420
```

tggatttcga ggatagtagg tataaatttt tttttaagaa gggttaaggt attttaaaga 3480
taagaaattt aaaattagcg tatttttata tataagtagt tatttttgtt tatttgtggt 3540
ttagatacga gtggagtgcg ataagggata aattattttc gcgtattttt tagcgatggg 3600
gcgaaagtaa cggatttagt tttcgggagt tgttttcgtc gatttttttt gtcgcgattt 3660
gattcgcggc gattgcgttg ttttttggtt gtttttttcg ttttcgtagg cgcgcggggt 3720
tattatttac gcgcgtattg taggtttttg cgtacgacgt tttagatgaa gtcgttatag 3780
aggtcgtatt acgtgtgcgt ggcgggtttc gcgggttgga agcggtggtt acggttaggg 3840
attagttgtc gtgtggggtt gtacgcggtg tttcgcgcga tgcgtagcgc gttggtacgt 3900
tttagtcggg tgcggttttt tttagcgcgt ttagcgggtg ttagttttcg tagtttaatg 3960
agtttaggtt ttttcgatat ggttcggttg ggttcgtgtt tcgttggttt tgggcgttag 4020
taagcgcggg tcgggcgggg ttatagggcg ggtttcgatt ttagcgtttt ttttaggatt 4080
tagattgggc ggcgggaagg agttgaggag agtcgcgtaa tggaaatttg ggtgtaggga 4140
ttgtggggtt cgaaggcggg gttgggcgcg ttttcgtaga gttttttttcg ttttgttttt 4200
tttttttttt ttcgtttttt ttttatattt tatttcggac ggttataacg acggcgatcg 4260
taaagtatta cgcggagata ttcgtgtttt tggaggttag ttttattgtg ttagaggaag 4320
agggttttta tattcggttt tggttttttt ggttcggttt gttgaagtaa tatatttggt 4380
ttatttattg ggtggggtag gaagtttcga gtttttattt ggggtgagga ggagggagat 4440
cggttagtag ttttatcgtt cgttttgttt tttattgcgg agattggggt ttcggtagag 4500
gttggatcgt g 4511

<210> 84
<211> 4511
<212> DNA
<213> Artificial Sequence

<220>
<223> chemically treated genomic DNA (Homo sapiens)

<400> 84

tacggtttag tttttgtcgg agttttagtt ttcgtagtgg agagtagagc gggcggtaaa 60
gttgttgatc gatttttttt ttttttattt taagtgaagg ttcgagattt tttgttttat 120
ttagtgggta ggttaagtgt gttgttttag taaatcggat taggagggtt agggtcggat 180
gtggggattt tttttttttta gtatagtaaa gttggttttt agaaatacgg gtattttcgc 240
gtggtgtttt gcggtcgtcg tcgttgtggt cgttcggggt ggggtgtgag gaggggacga 300
aggagggaag gaagggtaag gcgggggggg ttttgcgaga gcgcgtttag tttcgttttc 360
gggttttata gtttttgtat ttaggttttt attgcgcggt tttttttagt ttttttcgt 420
cgtttagttt ggattttggg ggaggcgttg aagtcggggt tcgttttgtg gtttcgttcg 480
gttcgcgttt gttagcgttt aaagttagcg aagtacgggt ttaatcgggt tatgtcgggg 540
gagtttgagt ttattgagtt gcgggagttg gtattcgttg ggcgcgttgg gaagggtcgt 600
attcggttgg agcgtgttaa cgcgttgcgt atcgcgcggg gtatcgcgtg taattttata 660
cggtagttgg tttttggtcg tggttatcgt ttttagttcg cggggttcgt tacgtatacg 720
tggtgcgatt tttgtggcga ttttatttgg ggcgtcgtgc gtaaaggttt gtagtgcgcg 780
cgtgagtagt ggtttcgcgc gtttacgaga gcggaagggg tagttaaggg gtagcgtagt 840
cgtcgcgggt taagtcgcgg tagagggggt cggcggggat agttttcgag gattaggttc 900
gttattttcg ttttatcgtt gaagagtgcg cgaaaatggt ttattttttg tcgtatttta 960
ttcgtatttg ggttatagat gagtagaggt ggttgtttat atgtaaaaat acgttgattt 1020
taagtttttt atttttaaaa tgttttggtt tttttgaga aagggtttgt gtttattgtt 1080
ttcggagttt atttttttag gtttgttttt ttttaaatat ttatgatttt ttttagaatt 1140
tttagggtga agggaaatta ttatttatgg gagggagttt ggaaaaattt agaattttttg 1200
gtgggttttt tgtaagtagg agttttgttg agtttttatt tagtaaatat tttttttttga 1260
tttagtgaat tagatgttaa aatatgtacg tagttatata tttagtagtt tttttgtatt 1320
tttgggaatc gttagtaagt aaaggttgtt ttttttttggg tagatattag ttggaattat 1380
taggggtgtt tttatagttt ttttcgttag tttggatttt atcgtagatt tgttgaatta 1440
attgttggga gtggatttta ggtattagta aattttaaaa attttttaaa ttattgtaat 1500
atggagtttg ggttgagtat tattgttttg gtttatttag gaatttgtgg atggatagtg 1560
ttttaggttt gtgtgtgtat ggagattttt ttattcggta taagaggata ttataaattt 1620
agttgggggg agtataaagt tgtgatagaa tgtaaagaat gaataagggg tcgagcgcgg 1680
tggtttatgt ttgtaatttt agtatttcgg aaggcggagg cgggtggatt atttgaggtt 1740
aggagtttaa gattagtttg gttaatatgg tgaaatttta tgtttattaa aaaataaaaa 1800
aaaatgagtt aggcgtagtg gcgggtgttt gtaattttag ttattcggga ggttgaggtg 1860

ggagaattgt ttgaatatag gaggcggagg ttgtagtgag tcgagatcgt gttattgttt 1920
tttagttttg gcgatagagt gagattttgt tttaaaaaaa aaaaaaaaaa aaaaaagaat 1980
aaggttggga tattgtagcg tttttaaaga gaaataaagt agttatggag ataagaagta 2040
ggatgatttg ggtatgttta ttagaggtag agataaggga gaaattaaag ataagtttgg 2100
gtttttgttt ttagtaattg ggagtttagt ggttattttt gttgtaaaga ggaagttggg 2160
taagtgtagt agtgaggttg aagaaaaggg aattaaattt tggttatgtt tatttgaaac 2220
gtttttaga tattttagtg aaggtattgg tacggaggat ttagtttgag ggtttaggtt 2280
agtgttttag tcgtggattt ggggtagatg aatgtagata gattaggtta gtgattagga 2340
ttgagtttag attttatcgt gagatatgga agttgagtta gaatttgtaa aggagttgag 2400
taggagttgt agggggtagg aggaaaattg ggagagtgta gtttttggga gttaaaggga 2460
gtaagtttta aatgatgttg agggggtgag aatggagaat ggaatattgg attttatttg 2520
gtagtatata gatcgttgag gattttgttt cgggtagttt tttggaggaa gaggtaagtt 2580
tggttggagt gggtagaggg gagagtgaag gcgaaggatt agagtgtata gagattagtg 2640
ttttggtttg aggggagtag agataggtga taattatagg gtagacgtag gttaaaggtg 2700
tttagttttt tttttaagta aatgggtaga tgtattttat atacgttttt agtgaagggt 2760
cgggtgcggt ggtttaagtt tgtagtttta gtattttgga aggtcgaggc gggtggatta 2820
tttgagatta ggagtttgag attagtttgg ttaatatggt gaaatttcgt ttttattaaa 2880
aatataaaaa ttagttgggt atggtggcgg gcgtttgtaa ttttaggtat ttaggaggtt 2940
gaggtagaag aatcgtttga atttaggagg cggaggttgc ggtgagtcga aatcgcgtta 3000
ttgtatttta gtttgggtga taaaagtaag acgtagtttt ttgttgttgt ttttttaatt 3060
gttaatgagg aaaggggaag ttttgtgtta ggcgatagag atttaattgt tgagtaggtt 3120
tttttgtttg tggtttttcg gtcggttttt agacgtttag gtggttaata ttagagttcg 3180
cgtagtagtg tgaggtaatt tattgagata ggtcgggttt gcggagtttg gcgagtagcg 3240
gttttttttt tggggttttt ttttaatttt cgggatattt tttcgatttg gagtttttttc 3300
gttttatcgt taggtttttt tgtagattgt aagtttattt gttattatcg ttgtcgcgcg 3360
ttcgtttgtt tggattgttg cgggtttcgg gatttgggtt gggaattcgc ggtggagcgg 3420
gatacgaacg tggtgagcgc ggggtcgagg gcgtatggga agggcgagga tgggtaggtt 3480
atagtgtagg tattttcgag ggttgtttgg gtgtcgcgcg taaggagcgt tttaattgtc 3540
gatttttcgg cggtatagag aggttaattt tgcgcggggg ttgggagggg agtttggatt 3600
gtcggtttcg taagtatttt attcgttgta agcggattcg ggtttaggtt gatttaggtt 3660
tcgcgtacgc gtatttttcg tattttttcg ttttcgtttt cggttagagg ttatttttgt 3720
gcgtttgttc ggacgttggt attcgttttc gtttttttgtg gtaggtgggg tttgtgagtg 3780
gagtttcgga gcgatgaggt tatttttggg ggcgaagcgt gcgtgtttc gtttcggcgt 3840
ttttgtttta atgagataag agttagattt cggcgattta cgttttagtt ttaacggttg 3900
cggcgcggtt ttggttcggg cgtacgcgta tattgatacg cgtatacgta cgtacgcgat 3960
cggggcggtg gttggcggtt acggacgcgt aggattgggg gacgggcggg tacggttatg 4020
ggcgaggcgg aggcgttttt tttcgaaatg atttggagta gtacgacgag tagtggttat 4080
tgtagttaag aggattcgga ttcggagttc gagtagtatt ttatcgcgcg aatttcgtta 4140
gttcgtaggt cgcgtcggga ttaggtggga gttagggggt gtcggcgggc gggaggggaa 4200
gcggtcgttg gagtttcgtt tttttcggtt cgttgtcgcg ttttgggtcg gtgggtagtt 4260
ttattttttt ggttacgtgg tttttcgcgg gttttggtcg gggatttgtt cgcggaatcg 4320
tgcgtaagat ttcgatttta tcgtttagat gttgggtgtc ggggtttttt tggtttttgt 4380
tatagatagg ttgaatacgg aaaaagtagt tgtatggttt gtggtagatt tgagtcgggt 4440
attatttagt tatgattaaa gtcgatcgag tagtttggat tagtatttcg attttcgcgt 4500
tcgaatgttt t 4511

<210> 85
<211> 1775
<212> DNA
<213> Artificial Sequence

<220>
<223> chemically treated genomic DNA (Homo sapiens)

<400> 85

atatcgagtt acgaaattta gagtaaaagt aaaatttttt aatggaatgt ttatgtaaat 60
attagaaata taataaaata aggtaaagtt tgtttttttaa aatgttgttt attatattaa 120
aatataattt tgttttttaa atagcgagta aaattaaatt aattttattt ttttaaatag 180
aagtttattt aaagtttttt tataaaaatg atatttattt gatttaatat tgttttttta 240
ataagaataa tacgtagggt gttagagggt agttgttgga ttttttttta gtaaataaag 300

gagattagtt agagtttagt ttggtttgtt taggaaagga ggaacgtagt ttgttgatat 360
ggttaaggaa tgttaattaa tatattttaa aatttttatt attttgtttg agacgtgtat 420
atttttttag gtttttaaag tttaattaga aagtgtattt aattttattg ttattttatt 480
tatagtgggg aagtttatcg aggaaaatta tagggaaata aaatgttttt tagttattta 540
tattagtata attaatttag agtttacggt ataaattaaa taagttaatt tgttagtgtt 600
ttgaatgaag atatttaatt aaagtatgta ttttttaatt ttttagtagt ttttaggata 660
gttggttttg gtgatcgttt tttggttttt tattgttttt aatacgattg gtttttttatc 720
gtaggatttt aaataaaatg agataattaa attatatttc gagcgaaggg gcgtttattt 780
tgtagataaa tatatcggtt ttgttttttt taaaatgcgg atacgtgttt ttttcgtatt 840
aggggggggtt tttcggcgcg cgtttcgtcg ttatttgttg aggaaagcga gcgtattttt 900
tgtagtttag gtttcgggcg ttagttttgt ttcgtagttt tagagttcgt cgtagttcgg 960
gtggtttttt ttcggtttag cgttcgtcgt ttgtttttcg ttttgtaagt tttaagaggt 1020
agttattttt cgtagttttc gcgtttgtaa ttgtttttttg gcgggggagt gggtgtttaa 1080
aaagttagta gttggagaaa ttgaaaagat tataagcgat ttaacgataa gttttttttt 1140
ttttttaaag atcgagagga gggtagaggg gagtagtgtt tgagtttacg tgatcgagtt 1200
agggagttcg acggttttag gaacgttcga cgtcgcgcgt gatttttaag tgggagtatt 1260
ttcgaatcga tttttggttt atttataagg atagtggcgt atagatggcg tttttcgtag 1320
ttttagtttt agatttaaga ggtttggagt agggtttgag aatatgtatt tttaattagg 1380
ttttgggggga tgtcgatatt gatatagtta gtttggggat tatatttcga ggattacgtt 1440
tttagttttt gatttatata agtgttattt agaatagatg tttgatttta aggagttagt 1500
ggtgaaatta gagaggtttt gggtttgtta aatttttagt agtaaacgta atttcgggtt 1560
ttggagtggt aaagtcgtgg attagaggtg gagggagtgg gtttttagtt tttaagaggt 1620
tattgggaag gtttttcgcg ttagattaaa gattttaggt attttttcga atttatttga 1680
agtggtatcg ggagatttg tgttttcggt tacggcgttt tttttcgttg gaggtatttg 1740
tttattttttt ttttcgggcg aaggtttttc gcgtt 1775

<210> 86
<211> 1775
<212> DNA
<213> Artificial Sequence

<220>
<223> chemically treated genomic DNA (Homo sapiens)

<400> 86

ggcgcgaggg gttttcgttc gagaagaggg gtgggtaggt gtttttagcg gagaagggcg 60
tcgtggtcgg aggtataggt tttttcggtg ttattttaag tgagttcgag gaagtatttg 120
ggatttttga tttaacgcga aaggtttttt tagtgatttt ttgagagttg agaatttatt 180
ttttttattt ttagtttacg gttttgttat tttagggttc gaggttacgt ttgttgttgg 240
ggatttgata aatttaaagt ttttttggtt ttattattgg tttttttagaa ttagatattt 300
gttttgaatg atatttatgt gagttagggg ttgaggacgt gattttcgaa gtgtggtttt 360
tagattggtt gtattagtgt cggtattttt taggatttgg ttggaaatgt atatttttag 420
gtttatttt agatttttta aatttgagat tggggttgcg gggagcgtta tttgtgcgtt 480
attattttg tgggtggatt aggagtcggt tcgagggtgt ttttatttag aggttacgcg 540
cggcgtcggg cgtttttgag atcgtcgggt tttttggttc ggttacgtgg gtttaggtat 600
tatttttttt tattttttttt tcggttttta aaaggaagaa ggggtttatc gttaagtcgt 660
ttgtgatttt tttagttttt ttagttgttg gttttttgga tatttatttt ttcgttagga 720
ggtagttgta agcgcggagg ttgcgagaaa taattgtttt ttgaaatttg tagggcgaag 780
agtaggcggc gagcgttggg tcggggaggg attattcgag ttgcgacggg ttttggggtt 840
gcggggtagg gttggcgttc ggagtttgag ttgtaggagg tgcgttcgtt ttttttaata 900
ggtggcggcg gggcgcgcgt cgggagattt tttttaatgc gggaaaagta cgtgttcgta 960
ttttagagaa ggtaaggtcg gtgtgtttat ttgtaaggta agcgtttttt cgttcgaggt 1020
gtggtttaat tgttttattt tgtttgaaat tttgcggtga gaaattagtc gtgttgagaa 1080
taataaaaga ttaaaaaacg attattaaaa ttaattgttt tgaaagttat tggaaagttg 1140
gaaaatgtat gttttgatta aatgttttta tttaagatat tggtaagtta atttatttag 1200
tttgtgtcgt gagttttggg ttgattgtgt taatatgaat aattgaaaaa tattttattt 1260
ttttatggtt tttttcgatg gattttttta ttatgggtga aatgataatg gagttgaata 1320
tattttttga ttgaattttg agggtttggg aagatgtata cgttttaggt aagatgatag 1380
gggttttaaa atgtattaat tggtattttt tagttatgtt agtaagttgc gttttttttt 1440
ttttgggtag attaagttaa gttttaattg gttttttttta tttgttgaag aggagtttaa 1500

taattgtttt ttaatatttt gcgtgttatt tttattggaa ggataatatt aagttaagtg 1560
aatgttattt ttgtgaaaaa attttgagtg gatttttatt taggaagata aggttgattt 1620
aattttattc gttgtttaaa aagtaggatt gtgttttggt gtggtaggta atattttgga 1680
ggatagattt tgttttattt tgttatattt ttagtattta tatgggtatt ttattagaaa 1740
gttttatttt tgttttaagt ttcgtaattc ggtgt 1775

<210> 87
<211> 1223
<212> DNA
<213> Artificial Sequence

<220>
<223> chemically treated genomic DNA (Homo sapiens)

<400> 87

tttatgtgtt ttattcgggg ttcggagtaa ataaaatcga tttaaaaata attttgaatg 60
atagaaaggg ggtatttttt ttatttaagg gggtgaggta ggttgaagat gggtttttat 120
tttttttaag attatggagg gcgtcggggt gttattttag ttgtgaaaaa gggatttagg 180
attcgtagat gttggcgagg aataggggtg cggagtatga gtcggttttg tattacgttt 240
tggggttttt agtattgaag cgtttagtat tatgtagagt agttgggagc gggtttcgtt 300
gggcggggtc gagttgcgcg cgattttcgg cgcgttcggg gaggtttaga taggggtggt 360
ttttttggtt ttcgttttcg cgggttttat gatatcgtat tggttcgcgg gacggggcgg 420
ggtcgggtga gggggaggag atagttttac gttttgcgat tcgcggtgat ttttacgcgg 480
aattttttcg cggtaattcg aagtatcgcg tttgcgtgtt gtagtagcgt ttggtggcgg 540
tggtagtttg ttcgcgggtg tgtgaaggga gatagtgtgg aggttatagg gtattcgtta 600
cgatgagtag tattttagtt aagatcgcgg agatagaagt agaggtaatg gacgtagttg 660
gcggtttatt tttagtttga gtattttttt tttggtggcg tcgttttttt ttgttttttt 720
ttgagtcgcg ttaggatcgg gtttagattt cgcgattttt tttagcgagg ttcgtgtttc 780
gattgttttt tgttacgatt aggagttttc gatttttttt agttagggtt tttttttttt 840
tcgtttcgcg tttacgtttt cggatttttt tttgttcggg ttcgcgtttc gtatttttgt 900
attttaggtt tttagttcgt tttttttttt agtgtttggt ttttgttttg atgttgtttt 960
ttttcgtttt gttttgagaa atattgaatt ttgtttttta ttttattttt gatttggtta 1020
gatagtttag gatttttttt tattattttt cgttgcgttt tttttatttt tttttttttt 1080
agtgtttatt ttaatgattt tttgatttag agtatttgtt ttcggtaatc ggatttggaa 1140
gtaaaatgat atttgtgtgg gagtagttta tttcgtttgg atagtgtgtg attttattgc 1200
gtgcggtttt gagagcgttt tat 1223

<210> 88
<211> 1223
<212> DNA
<213> Artificial Sequence

<220>
<223> chemically treated genomic DNA (Homo sapiens)

<400> 88

gtaaagcgtt tttagggtcg tacgtaatgg aattatatat tatttaggcg gaataaattg 60
tttttatata gatgttattt tatttttaga ttcgattatc gggagtaaat attttgaatt 120
aggggggttat tagggtagat attgggaaaa gggggaataa gggggacgta gcgaaggatg 180
atgaggaaag attttgaatt atttgattaa gttagagatg gaataaggga taggatttag 240
tgttttttag agtaaaacgg aaagaaataa tattagaata ggagttaaat attgggaggg 300
ggaacggatt aagggtttaa ggtgtagggg tgcggaacgc gggttcgggt aaaggggagt 360
tcgagagcgt agacgcggga cgagggggaaa gggggttttg attgaggaga gtcggggatt 420
tttaatcgtg gtagagggta gtcggaatac gggtttcgtt gagagaagtc gcggaattta 480
ggttcggttt tgacgcggtt taaaggagaa taggaaggag cgacgttatt aaagaaggaa 540
tgtttagatt aagagtgaat cgttagttgc gtttattatt tttgttttta ttttcgcgat 600
tttagttaag gtgttgttta tcgtggcgag tattttgtgg tttttatatt gttttttttt 660
atatattcgc gggtaaattg ttatcgttat taggcgttat tgtagtacgt aggcgcggta 720
tttcgaatta tcgcgagaga gtttcgcgta ggggttatcg cgagtcgtaa agcgtggggt 780

tgttttttt tttttattcg atttcgtttc gtttcgcgag ttagtgcggt gttatagagt 840
tcgcgggagc ggaggttaga gaggttattt ttgtttgggt tttttcgagc gcgtcgaggg 900
tcgcgcgtag ttcggtttcg tttagcggga ttcgttttta gttgttttgt atggtattga 960
acgttttagt gttggagatt ttagggcgtg gtgtaaagtc gatttatgtt tcgtattttt 1020
gtttttcgtt agtatttgcg ggttttgggt tttttttta tagttggggt gatatttcga 1080
cgtttttat ggttttggag ggagtagaga tttattttta gtttgtttta ttttttgga 1140
taaagaaaat gtttttttt tattatttaa aattattttt aggtcggttt tgtttatttc 1200
gaatttcgag tagagtatat ggg 1223

<210> 89
<211> 2183
<212> DNA
<213> Artificial Sequence

<220>
<223> chemically treated genomic DNA (Homo sapiens)

<400> 89

aagggagaag gacgggggcg ttgtttggtg attgttcgcg tgattttttt aaggcggcga 60
ttattttttg ggattcgttt tttagtcgcg tcgttcgtat tcgggtttcg cgattttttt 120
tcgtttttt ttcgttttag cgatgttaag acgttgaatc gtagggacga ggttttgggg 180
ataaagttat ttcgcggcgt tttttttgt taagtcgcgg gtttttggcg gttagaaagc 240
gttttaggtt tatttttgcg ggttcggttt tttttttta tttaaggttt cggggttacg 300
agttttttat tttgcgtttt tttcgcgttt tttttatttt aaattttagt agcgtcgttg 360
tttttttcgg aaataagttc gttgagtaaa ggcggaggaa aattttgggt ttttcgtttt 420
gattggtttt ttttcgtcga gcggggcgag gtagcgattg gttgtcgggc gttgttagtc 480
gtcgaggtta gagttatcgt cggggaagcg tagtcgggtt cgggtgtttc ggttgtcgtc 540
gcggttgttc ggttagtgta gttgggcgag ttcgcgcggg cgtcgtcgtc gtttttgttg 600
ttgtttattt cgtcgcgtcg tcggcgttgt cggttttgta ggcggtcgtc ggggaggggc 660
ggtcgagaga gcggagtacg aggaggcggg ggcggtagag aagtgatgtt ggcgtcgggg 720
atcggggtag cggtagcgga gtagtagtat tttcgggatt ttggttgtag cgtttttgtg 780
gttcgcgcgt tagtttagcg gtcggagatt acgcgtttcg cgggtaaggt tttgttcggt 840
agcgttcgcg acgttttcgg gacggttggc gtggtttttg gggttacga ggcgcgtggt 900
ggtcggggta tttcgacgaa ggtgttggga ttcggattgc gtgtttcgac gttgggttgt 960
gttattgttc gcgttcgtgc ggttttagtt ggtgcgtttt ttttttcgcg tttatagatt 1020
ttgatagcgc ggggatggta ttgtgtgagt gtggatgtgg atgcgtgtgt gcgcgcgcgc 1080
ggtttggggt attttgtagc gagaatagtg ggtttttttt gcgtggttcg acgtttttta 1140
aggatttaag ttgggttgga gtgagtagga tgttgttggt tcggcgggag gtagaacgta 1200
cggagcgtac gggaaagttt atcgtggttc gtaaagagtc gagtaaattg aattttattg 1260
cggtgattaa aggagttgt ggatttggcg cgttagggga gaggcgcgtt ttacgaggtt 1320
ttttaaagtt ttattttgtt tcggggattt cggaggggaa gggaagagag ggaaaagtaa 1380
ttgttggttt atattgttga agaaaaggag gaaagagaag ttgggggtgg gggtgggggt 1440

ggaggttatt tttgggggag tggcgatttt taggtgtttg aagtttcgcg tgggaggttt 1500
gttatatttt tttggagcgt agatttgagg atggagagtt ttattttgag gtattggtag 1560
cggtttttt tgtttggtgg cggcgtaggg cgtagcggga tagtttggtg gaggtagagc 1620
ggggtttcga cgttcgacgg ttcggcgttt aggtggggtt attgtttttt taggtcgttt 1680
tggttcgttg attttttagg ttgagattcg gtttggtgcg tttttggtag ttgttaaatg 1740
tttggcggtt tcgtttttcg ataaagttta ggttcgttgg ggtttggttt attagattta 1800
ggatacgtta attacgtaga gtaattaggt tagggtattt agttgttttt agagcgtttt 1860
tgttttatat tagattattt tatttgataa agttgttgag agttttattc gtgttgtaaa 1920
gtttttcgta gaaggcgaat tttttgcgtt gttgtaaatg gatggtgtta ttgggttaga 1980
ttcgaatttt cgtagtttag tgtagtgggt tttttatttt tgtttaggac gtataaattt 2040
atttttggcg attgtttttt atgtggtttt ttgttttcgt gggagtattt gtttaggaaa 2100
gtttttgaaa tattttaata ttaaggagtt ttttttattg ttttagatgg ggcgaggttt 2160
cggtatttga ttggcgttgg ggt 2183

<210> 90
<211> 2183
<212> DNA

<213> Artificial Sequence

<220>
<223> chemically treated genomic DNA (Homo sapiens)

<400> 90

```
attttaacgt tagttaagta tcggagtttc gttttatttg agatagtggg gagagttttt      60
tagtattgag gtattttaag aattttttta agtagatatt tttacggaga taaggagtta     120
tatagaaaat aatcgttaag agtaagtttg tgcgttttga gtagggatgg agaatttatt     180
gtattgaatt acggagattc ggatttggtt tagtagtatt atttatttgt agtagcgtaa     240
agaattcgtt ttttgcggaa ggttttgtag tacgggtgga atttttaata gttttattaa     300
ataagataat ttaatatgaa ataaaaacgt tttggaggta gttgggtatt ttaatttggt     360
tattttgcgt ggttaacgtg ttttaggttt gatggattag attttagcgg gtttgaattt     420
tgtcggaaag cggagtcgtt aggtatttgg tagttgttag aggcgtatta ggtcgggttt     480
taatttggga ggttagcggg ttaaagcggt ttggggaggt agtggtttta tttggacgtc     540
gggtcgtcgg gcgtcggagt ttcgttttgt ttttattaag ttgtttcgtt gcgttttacg     600
tcgttattag gtaggaggag tcgttattag tattttagag tgggattttt tatttttaag     660
tttgcgtttt agaaaagtgt agtaagtttt ttacgcgggg ttttagatat ttaaaagtcg     720
ttattttttt aagagtaatt tttattttta tttttatttt taattttttt tttttttttt     780
tttttagtaa tgtggattaa tagttatttt tttttttttt tttttttttt tcgaagtttt     840
cggggtaaag tgggattttg gggagtttcg tggaacgcgt tttttttttg gcgcgttagg     900
tttatagttt tttttaatta tcgtaataaa gtttaatttg ttcggttttt tacgagttac     960
ggtggatttt ttcgtgcgtt tcgtgcgttt tatttttcgt cgagttaata gtattttatt    1020
tattttagtt tagtttgggt ttttgaagga cgtcgggtta cgtagagggg atttattgtt    1080
ttcgttgtaa agtgttttaa gtcgcgcgcg cgtatatacg tatttatatt tatatttata    1140
taatgttatt ttcgcgttgt taaagtttgt gagcgcggaa gggagaacgt attagttgga    1200
gtcgtacggg cgcgggtagt agtatagttt agcgtcggga tacgtagttc gggttttaat    1260
atttcgtcg ggatgtttcg gttattacgc gtttcgtaat ttttaaagat tacgttagtc     1320
gtttcggggg cgtcgcgggc gttgtcggat agaattttat tcgcggggcg cgtggtttc     1380
ggtcgttggg ttggcgcgcg ggttataggg acgttgtagt tagggtttcg aagatgttgt    1440
tgtttcgttg tcgttgtttc gattttcggc gttagtatta ttttttgtc gttttcgttt     1500
tttcgtgttt cgttttttcg gtcgtttttt ttcggcggtc gtttgtaaga tcggtagcgt    1560
cggcggcgcg gcggggtggg tagtagtaga ggcggcggcg gcgttcgcgc gagttcgttt    1620
agttgtatta gtcgagtagt cgcggcggta gtcgaagtat tcgagttcgg ttgcgttttt    1680
tcggcgatgg ttttggtttc ggcgattgat aacgttcggt agttaatcgt tgtttcgttt    1740
cgttcggcgg ggggagatta attagagcgg aaggtttaag gtttttttc gtttttgttt     1800
agcgggtttg ttttcgggag aggtagcgac gttgttgggg tttgggatgg gggaggcgcg    1860
ggaggggcgt aggatggggg attcgtggtt tcggagtttt aggtgaggag ggaaagtcgg    1920
gttcgtaggg gtgggtttgg ggcgtttttt gatcgttagg agttcgcggt ttggtaggaa    1980
agggcgtcgc ggggtgattt tgtttttaga gtttcgtttt tgcggtttag cgttttggta    2040
tcgttggggc gaggggggag cggggagggg tcgcgggatt cgagtacgaa cggcgcggtt    2100
ggagagcgaa ttttaggagg tggtcgtcgt tttgaaggaa ttacgcgaat agttattaga    2160
tagcgttttc gttttttttt ttt                                            2183
```

<210> 91
<211> 8030
<212> DNA
<213> Artificial Sequence

<220>
<223> chemically treated genomic DNA (Homo sapiens)

<400> 91

```
tttgcgtaat tttagttagt ttgaattttc ggaggtaaat agagcgtaat ttgttttgga      60
agaattttgt tacgtttaag gttttatgtc gggtgttggt ttttttgttt ttaattattt     120
ttttatttcg attcggattg tagaaatttt taattttttg ttttttagtt ttatttttat     180
ttttagattt ggggttgtta aataacggaa attttaggaa gacgtaggtg cggtttttaa     240
agttttggtt tgtgagcgtt tttaggttgg tcggggcgtt ggttttttta gaaataaggt     300
ttgaatatgt aggtgttagt taggattttt tgtcgtttgt ttatcgtcgt tttttttcg      360
```

ggatttgaga gaagcgggag tgtgtgtgtg tgtgtgtgtg tgtgtgtgtg cgcgtgtgtg 420
tgtttcgtgt taggagaaag gttcgtggtt gtcgttttag ttggtttgtt tattttttgt 480
ttttaggaga agtttgttta tttgggatat tagcgatttt ttttattttt attttatcgg 540
ttttatattt tttttatttt tttttaagtc gttgatgtag aaagtatttg gttattttga 600
aacggtagtt ttcggaattt tagttttgtt ttgtaattta gtagggtttt tcggaagttt 660
tataattgag atttatgggt ttatcgggta attaaatgat gttattgtgt taattttgta 720
tgtattattg ttgttcgcgt cgttcgtggg tcgtcggtcg gtcgtagttc gttggcgacg 780
agggtattat agttgttttg atcgcgtaga ttatgtatgt ttcggtttcg ggaatttatt 840
atgtattaga atatattagc gtttgtattt taaaaggtta aattattggt ttttagttag 900
ggattttcgg taagtggttt gttagtgacg agtgtttgtt tatattggta tatagcggag 960
tttttttgttt tcggtttatt cgtttttagg aaagttttgg ggtgaggcga aggcgattga 1020
agtaatgttt tttttttttag atcgtagttg tttagggggg atatagtacg gtatttttta 1080
tcgaattttt ttcgttcgtt cgtattttag tttttttttt tttagcgatt tttttatttt 1140
tttttttttt tttttttttg acgtttgatt ttagtagtaa aggaggtaaa aaaggtatcg 1200
agtcgttagt taaatttgaa aagtgcggtt tcgttttttt tatagttatt ggtagttttt 1260
cgtggaaggt tcgtttttcg gggtagttgc ggtttcggag tggttgcgtt tggcgttcgt 1320
cgggcgtggt ttcgttttag gttcgggagg gtaggttggt tgtttcggcg agcggtagag 1380
ttttttttgga tagttttcgt ttatttaaat agaagacgtc ggcgtcggag cgggttcgga 1440
tatggcgagg ttgcgagtcg gttcgagcgg cggggttcgg tgattttttt tttttttttc 1500
gttttttttt ttttttcgta cgtacgtttc gttcgttttt atttcgtttt tatttcgggc 1560
gagttcgttc gtagttcggg gcgtatattc gtacgcgtat ttttttttat ttattttcgc 1620
gttcgttttt attttcgtag tcgagtttcg ttacgcgcgt tttgttcgtt cgtcggtcgt 1680
tttcgtcgtt ttcgtcgttt tcgggttttg atggattgaa tgaaggttgt ttatatcgtt 1740
tatcgatgtt ttattaaaga tttagaaggt tgcgttatga attcggagtt gataatggaa 1800
agtttgggta ttttgtacgg gtcggtcggc ggcggtagtg gcgggggcgg cggcgggggc 1860
ggcgggggcg gcggcggggg ttcgggttat gagtaggagt tgttggttag ttttagtttt 1920
tattacgcgg gtcgcggcgt cgttggttcg ttgcggggtt tttcgtcgtt tttaatcgcg 1980
tattaggagt tgggtacggc ggtagcggcg gtagcggcgg cgtcgcgttc ggttatggtt 2040
attagtatgg tttcgatttt ggacggcggc gattatcggt tcgagttttt tatttcgttg 2100
tattacgtta tgagtatgtt ttgcgattcg ttttcgtttg gtatgggtat gagtaatatt 2160
tatattacgt tgatatcgtt ttagtcgttg ttatttattt ttatcgtgtt tgataagttt 2220
tattattttt attcgtatta ttattcgtat tattattatt attattatta ttagcgtttg 2280
ttcggtaacg ttagcggtag ttttattttt atgcgcgacg agcgcgggtt ttcggttatg 2340
aataattttt atagttttta taaggagatg ttcggtatga gttagagttt gttttcgttg 2400
gtcgttacgt cgttgggtaa cgggttaggc ggtttttata acgcgtagta gagtttgttt 2460
aattacggtt cgtcgggtta cgataaaatg tttagtttta atttcgacgc gtattatatt 2520
gttatgttga ttcgcggtga gtaatatttg tttcgcggtt tgggtatttt atttgcggtt 2580
atgatgtcgt atttgaacgg tttgtattat tcgggttata tttagtttta cgggtcggtg 2640
ttggtattta gtcgcgagcg gttattttcg tttttatcgg gttcgtaggt ggttacgtcg 2700
ggttagttgg aagaaattaa tattaaagag gtggtttagc gtattatagc ggagttgaag 2760
cgttatagta ttttttaggc gatttttgcg tagagggtgt tgtgtcggtt ttaggggatt 2820
tttttcgatt tgtttcggaa tttaaaatcg tggagtaaat ttaaatttgg tagggagatt 2880
tttcgtagga tgtggaagtg gttttaggag ttcgagtttt agcgtatgtt cgttttacgt 2940
ttggtaggta aggtcggggt tagttagggg ttaggttgtt gggaagaggg tttcgggttc 3000
ggtgtttgtg gtttaagttt gcgcgtcgag ttattttttt tgattttttt tttttttttt 3060
ttatatacgt tttttttttt ttcgttttta tttttttttt tatttttttt ttttttttcg 3120
tttttttttt attttttttt tttttttttt tttttttttt ttattttttt tttgttttttg 3180
agttttttatt gatcgatttt tttttatttt attcgttttt tttttaatgt gttaattttt 3240
gttttatttt cgattttttt aggtattggg aggcgggatg ggggtgtgcg ttttttttta 3300
ggagttttgt tttttttaaga tttatagaaa ttaggatttg tttttattta aaattttatg 3360
tattttaagt tttttttaga taatatattt taatttttcg ggttgattag ttttttttgtg 3420
tagaggtagt tgagaggttt tgttttgtag agggaaaaga gtttttttatt tttttattta 3480
ttatataggt aaatttattt ggttattggt tgaaggtata gttttgtttt cgcggggaat 3540
cggcggttag gatataatag cgtttttgga gtttattttt ggttttggcg ttggcgtagg 3600
gattttttga tcgggtttga ggggttcggg ttagttttaa tgttattatt tatagcgagg 3660
gtagggtgta aggttgagaa ggttatattt atcgttttgg gaggacgtgg gagaagagat 3720
tgaggtggaa agcgttttgt tttgtttatc ggtcgttttt gtttcggttt tagcgtttgt 3780
tgggatttgt taggatttgt cggggtttcg ggagattttg agtattcgta ggaagaggtg 3840
ttgagaaatt aaaaatttag gttagttaat gtatttttgt cgtcggttgt aggtttcgtt 3900
tttgtattaa gcgggcgttg attgtgcgcg tttggcgatc gcggggagga ttggcggttc 3960
gcgggagggg acgggtagag gcgcgggtta tattgttttg gagtcggttc ggttttttgt 4020

gtttttttta gcggttaagt tgcgaggtat agtttttat tgttttagga gtatagaaat 4080
tttttgtgtg ggcggcgggt gcgcgagtta gagggaaaga tgtagtagtt attgcgattg 4140
gtacgtagtt gcgcgttttt gtgcgtacgg atttcgcgcg gtgtgcgtgg cgattgcgtt 4200
gtttttagga gtaagttacg ggtttagagg ggtaaaatgt ttaggttttt cgttgggaag 4260
gatatattat attttatggt aagttagggt gggcgatttt ttatggatcg ggtggagggg 4320
ggtatttttt aggatcggcg ggcggtttag gggaataatt cgtggtggcg atgatttgta 4380
tagcgcgggt tttgggatgc gcgcggtttc gagttagttt cgtatagttc gttttcggag 4440
ttgcgagttt aggtttttat tttcgatttt tcgggttttt ttcgtatcgt tgagtttagt 4500
ttgtggggtg tattcgatta acgttcgata gggttgggga atgtgatagg tagtaggttt 4560

attcgggttt ggggaggggg agttttcgtt ttgatagtat tttttttgt cgtttgttgg 4620
tggattttta tttttagtcg gtaatcgttt cgtagtgttg atttaagaag gtaaagaaaa 4680
ttaggttttt ttgtaaagag ttttttttaa atcggcggat ttcggatatt ttgagtggat 4740
ttagaaattt atgtaatttt tttttttag tttattttt attttttttt atagtttttt 4800
ttgatttgtt gttggttcgg ggtaagataa agtagttagt agagagcgat aataatagcg 4860
gcgggaaatg aattggagat tggttgatag tttttaatat tttgttatag atttttttcga 4920
atgttttagg ttgtttttgg tgggttttag tattcgtcgg tttttgggt atcggggatt 4980
agaaggaatt tggtagttgg ttttaggggt atagttaaag gtaggatgat agttattttt 5040
ttgtttattt tagagcgttg tcgtttttt atgtcggtcg cgtaaagaat atagttttta 5100
aaaaatacgt gtttttttgtt tatataggtt tgaaagtgat gaggaaagta atgtttcgtt 5160
tattagcgag ttttagtttt taaaatgatt ttaagcgttg ttgagatgag aaagcgtggt 5220
atttcggggg tttttagttt tattcgcgtt tatggtgtaa gtttgtaggg ataggttcgg 5280
gatagtattg tttacgttgt tagattttc gtagaggatc gttgaagttg ttttcgtggg 5340
agatagaatg tttttttttag cgagtggaaa aggtttgttg aggatttcgt tttgttcgag 5400
tatttaaatg tgtgtttgtt ttattatttt gggttgaaaa gggataagag ttttagtttt 5460
tttatttggt tattttatta gtaattataa gtgtgttgag tggttattat tatataggag 5520
gtttttagt ttggggttag tagattagtt tttttagata ttgatgtaga agttgggatt 5580
ggtaagtagg tattatgtgt tcggagcgtt aggggatagg agtaaatgga gaagaaaagc 5640
ggaggttttt ttcgttcgga gtatcgatcg gaattttcgt cggtacgtcg tagagggttt 5700
tcgtcgttgg gtttcggggg tttaataagt ttagtcgttt cgtaggcggt tcggtcggat 5760
ttttagatcg gtgtttggaa gatatcgttt ttgttttttt ttcgttaaat ttgttttttt 5820
tttttttttt ataggttata ggtttttttt tttttttatt ttggtttcgt tttcgggttt 5880
tgttaaatag ttaagtaggt cggggtttag gggtttaga atgaagaggt ttgatttggt 5940
tagcgtcggt aaagtttatt tttaggcgag gttataatag aggtaggttt tttttgttta 6000
gtttgtcggt gtagttatag ttaagggtgg tatttgaaag gaaaagggag aaaatttcgg 6060
agaaatttag attgttttaa cgttagattt tagagaaatt gattttaaat gtacggattc 6120
gttcggaaag ggcggttaag tggtaggtgg ttgtaatttc gttcggtcgg gttttcgtag 6180
aggtttttta agattagttt ttgtagggcg gtttttagta atttgataag aggcggttaa 6240
gataaatttt tgcgggttcg agtatatatt ttcgggcgtt gggttttaga gatttttaaa 6300
ttaagtataa ataagaaggg agtgagagaa tttaggttag aatttgtacg ggtattttat 6360
tgaggaaaag cgaggtttcg gtggtaggta tgtttttttt cgacgttcga aaatcgagtc 6420
gagcgttcga ttatatttat tgtagaggtt ttcgttttta gtgagttcgg atttttagc 6480
ggtttgttcg gagttggttt ttagttttcg tcgtagttcg acgtacggtt ttttttggt 6540
agtaagtttt tagcggttag tttgaagtta attttgttta ggcggtcgag ggtttttagt 6600
taatttatta tgatgtcgtt tgggttattt gatgttcgta gcggcgggat acggttcggg 6660
tagtgcgtag tggtttttgt taggggtatc gcgtgcgtgt ttgtttttcg ttgcgtcggg 6720
gacgtttttg ggtgatacgg gtcgttgggt attttttaag tcgaggaaac ggattttttt 6780
cgtagagttt cgcgtttatt ttttaatttt ttatttcgtt tttcgttgtt agggttttcg 6840
atttagttta tttttttttgg cggtttagtt agggattaga gttggagagg ttgaacgtaa 6900
ttcgtgttag tacggaatag acgatatgtt tgtttgttag ttgtttggat gaataattga 6960
aaagttcgtt gtagtttgtg tttcgttaag tttcgggtgt cgggagaata ttttttttaat 7020
acgtattagg gtgggcggga gcgggtagag gaggcgggat tcgagggagg agagtgaatt 7080
cgagtaggag aagtagttta ggtagttagg cgttttcgat gcgagaggtt gggtatttat 7140
ttttatttta ggttttttat tgtgtggtta tgttattttt ttaaataaat gtgtatatgg 7200
agggagatcg atgttgataa tgtttagaag attaaaagag tattaatgtt ggtaataata 7260
acgtaaacgt gtggatttag attttattga tttggaattt gattcggcgc gtttttagta 7320
agttcgacgg cgcgtttttt ttagtagagc gtttattagc gttacggttt cgcggttttt 7380
tagcggtgtc gtttcgttag ttttgcgcgg gtttttttcgt ttgatcgtag tttttttttc 7440
gcgaggtttt agttcgtttt atttttcga ggtttttttt tttttcgcg gggtttttg 7500
tttttgtat tttttttttc gattttgta ttattcgttt ttgtgcgtat atatcgttat 7560
ttgcgttttc ggcgattcgt ttgggcggtt gggttcgcga agttaatgcg ttgaacggtg 7620

ttcgagtttt tttaattatt ttgtgtttgg tcgttgttat tgggttttgg tgattaagtt 7680
taagtttgaa aatgacgtgg ttaaagttgt ttgttaacgg tagagtttaa ttagtcgtag 7740
attttttttt atttttatcg gttttcgtat taaaaaggtt tacgcgtaga ttttttacgt 7800
aggtgtattc gttggataat taaacgtggt tttagtaata aaagtttgag ttttattttt 7860
tcgagtagta ggttttgcgt tggattggtc gggtttaata gggagaattt tgtgttttat 7920
tttggttggg ataggaagta agagaagtaa attggggaat ttttgtttta ttttttttta 7980
ttttttggac gttttgggtc gaggttcggg ttattggttt atcgcgggta 8030

<210> 92
<211> 8030
<212> DNA
<213> Artificial Sequence

<220>
<223> chemically treated genomic DNA (Homo sapiens)

<400> 92

tattcgcgat gggttagtga ttcgggtttc ggtttaggac gtttaggggg tgggaagggg 60
tggggtaggg gtttttttagt ttgttttttt tattttttgt tttagttaag gtaggatata 120
agattttttt tgttagattc gattagttta gcgtagagtt tgttattcgg agggatgaag 180
tttaggtttt tgttattagg gttacgttta attgtttaac gaatgtattt gcgtaaaaag 240
tttgcgcgtg agttttttta gtacgagaat cgatgaggat aggaggggat ttgcggttga 300

ttaagttttg tcgttagtag gtagttttag ttacgttatt tttaaatttg ggtttagtta 360
ttagggttta gtggtaacgg ttaagtatag gatagttagg aagattcggg tatcgtttag 420
cgtattggtt tcgcggattt agtcgtttag gcggatcgtc ggaagcgtaa gtagcggtgt 480
gtgcgtatag ggcgggtgg tgtagaggtc gggggagggg gtgtagaggg tagagagttt 540
cgcgagagga ggagaaaatt tcggggaagt aaggcgggtt ggggtttcgc gggggaggag 600
ttgcggttag acgggagaat tcgcgtagag ttggcgaagc ggtatcgttg gaaaatcgcg 660
gggtcgtggc gttggtgagc gttttgttgg gaagagcgcg tcgtcgggtt tattggaaac 720
gcgtcggatt aagttttaga ttaatgaaat ttgggtttat acgtttacgt tattgttgtt 780
agtattaatg ttttttttaat tttttaaata ttattagtat cgattttttt ttatatatat 840
atttgtttga gaaagtgata taattatata gtggaaagtt tggaataaaa ataaatgttt 900
agtttttcgt atcgagggcg tttggttgtt tgggttgttt tttttgttcg ggtttatttt 960
ttttttttcgg gtttcgtttt ttttgttcgt tttcgtttat tttgatgcgt gttgggaagg 1020
tgttttttcg gtattcggga tttgacgaag tatagattgt agcgaatttt ttaattattt 1080
atttaagtag ttagtaggta aatatatcgt ttgtttcgta ttggtacggg ttgcgtttag 1140
tttttttagt tttgattttt aattaaatcg ttaggagagg tgggttgagt cgggagtttt 1200
agtagcgaga aacgaggtgg gaggttgggg ggtgggcgcg agattttgcg aagggggttc 1260
gttttttcgg tttgggaggt gtttagcggt tcgtgttatt taaggacgtt ttcggcgtag 1320
cgggagataa gtacgtacgc ggtgttttta gtaggagtta ttgcgtattg ttcgggtcgt 1380
gtttcgtcgt tgcgggtatt aggtggttta ggcgatatta tggtgggttg gttaagggtt 1440
ttcggtcgtt tgaatagaat tggttttaga ttggtcgttg ggagtttgtt gttaggagag 1500
ggtcgtgcgt cggattacgg cggggattgg agattagttt cgggtaggtc gttgggggat 1560
tcgggtttat tggaggcgga aatttttgta gtaaatgtag tcgggcgttc ggttcgattt 1620
tcgggcgtcg gaagaaaata tgtttgttat cgaggtttcg ttttttttta gtgggatgtt 1680
cgtgtaagtt ttagtttggg ttttttttatt ttttttttgt ttgtgtttgg tttagaggtt 1740
tttggggttt aacgttcgag agtgtgtgtt cgaattcgta gaaatttgtt ttggtcgttt 1800
tttgttaggt tgttgaaaat cgttttgtaa gggttggttt tggggaattt ttgcgaaggt 1860
tcgatcgggc ggggttgtaa ttatttgtta tttagtcgtt tttttcgaac gaattcgtgt 1920
atttggagtt aattttttttg aaatttaacg ttggggtaat ttaaattttt tcgaagtttt 1980
tttttttttt tttttaagtg ttatttttgg ttgtgattat atcggtaggt tgggtaggaa 2040
ggatttgttt ttgttgtgat ttcgtttaag ggtgagtttt gtcggcgttg gttaaattag 2100
atttttttat tttgagtttt ttaaatttcg gtttgtttgg ttgtttggta ggattcgggg 2160
gcggggttaa aatgagagag aaagggaatt tataatttat gagagaaaga gaagaggtag 2220
gtttggcggg agggggtag ggacggtgtt ttttaggtat cggtttgaga gttcggtcga 2280
gtcgtttgcg gagcggttgg gtttgttaaa ttttcggggt ttaacggcga gggttttttg 2340
cggcgtatcg gcggggattt cgatcgatat ttcgggcgga gaaagttttc gttttttttt 2400
tttatttgtt tttgtttttt agcgtttcga gtatataata tttatttatt agttttagtt 2460

tttgtattag tgtttgaaga gattgattta ttgattttaa attgaaaagt tttttatgta 2520
ataataatta tttaatatat ttgtagttgt tgataaaatg gttagataaa aaggttaaag 2580
tttttgtttt tttttaattt agggtaataa aatagatata tatttgaatg ttcgagtaaa 2640
gcggggtttt tagtaggttt tttttattcg ttggaggagg tattttgttt tttacgaagg 2700
tagttttagc gattttttgc ggaaaattta gtagcgtggg tagtgttgtt tcgggtttgt 2760
ttttgtagat ttgtattatg ggcgcgggtg gggttgagga ttttcgggat gttacgtttt 2820
tttattttag taacgtttgg gattatttta aaagttgaaa ttcgttaata ggcgaagtat 2880
tatttttttt attattttta gatttatatg ggtagaaggt acgtgttttt taaaagttgt 2940
gtttttgcg cgatcggtat gagggggcgg tagcgttttg agatgagtag gagaatagtt 3000
gttattttgt ttttaattgt attttaaga ttagttgtta agtttttttt ggttttcggt 3060
gtttaagaag tcggcgggta ttaaaattta ttagagatag tttgggatat tcggggggat 3120
ttatgataaa atgttaagaa ttgttagtta gtttttagtt tatttttcgt cgttattatt 3180
atcgtttttt attggttgtt ttattttgtt tcgaattaat agtaaattag agaaaattgt 3240
aggagaggat gaaaaataaa ttaaaggaga aagattatat aaatttttaa atttatttaa 3300
agtattcgga gttcgtcgat ttgggggagg tttttgtag ggaaatttag ttttttttat 3360
ttttttagat taatattgcg gggcgattat cgattgggaa taggaattta ttagtagacg 3420
gtaaaggaaa atgttgttaa agcggaaatt ttttttttt aagttcgggt gaatttgttg 3480
tttgttatat tttttagttt tgtcgggcgt tggtcgagtg tattttataa gttgggttta 3540
gcggtgcgag gaaagttcgg gggatcgggg gtggaaattt gagttcgtag tttcggaagc 3600
gagttgtgcg aggttggttc ggaatcgcgc gtattttaag attcgcgtta tgtaaattat 3660
cgttattacg aattgttttt ttagatcgtt cgtcgatttt gaaagatatt tttttttatt 3720
cgatttatgg gaagtcgttt attttggttt gttatagggt atagtgtgtt ttttttagcg 3780
ggggatttgg atattttgtt tttttgggtt cgtggtttgt ttttaggggt agcgtagtcg 3840
ttacgtatat cgcgcggggt tcgtgcgtat aaaagcgcgt aattgcgtgt tagtcgtagt 3900
aattattgta tttttttttt tagttcgcgt attcgtcgtt tatataggag gtttttgtgt 3960
ttttagaata atagagggtt gtatttcgta gtttggtcgt tagaggaggt ataaagagtc 4020
gagtcggttt tagaataatg taattcgcgt ttttattcgt tttttttcgc gggtcgttag 4080
ttttttttcgc ggtcgttagg cgcgtataat tagcgttcgt ttaatgtaaa ggcggagttt 4140
gtagtcggcg gtagggatgt attaattaat ttgaattttt aattttttag tatttttttt 4200
tgcgagtgtt tagggttttt cggagtttcg gtaaattttg gtaaatttta gtaaacgttg 4260
ggatcggggt aaggacggtc ggtgagtaag gtaaagcgtt ttttatttta gttttttttt 4320
ttacgttttt ttaaagcggt gaatgtgatt tttttaattt tatattttgt tttcgttgta 4380
ggtagtgata ttggagttgg ttcgagtttt ttaagttcgg ttagaaagtt tttgcgttaa 4440
cgttaaggtt agagatgggt tttaggagcg ttgttgtatt ttggtcgtcg gtttttcgcg 4500
ggggtaaggt tgtgttttta gttaatgatt aaataagttt gtttatatgg tgggtgggag 4560
agtagagagt tttttttttt ttgtagagta aagtttttta attgtttttg tatagggaga 4620
ttagttagtt cggaaaattg aaatgtgttg tttaaaagag atttgaagtg tatggggttt 4680
tgaataaggg taggttttgg tttttgtggg ttttggaaag atagggtttt tagaggaaaa 4740
cgtatatttt tatttcgttt tttagtattt gggaagatcg gaaatagggt aaaggttggt 4800
atattgaggg gagggcgaat gaaatggggg ggggtcggtt aatgaaagtt tagggataag 4860
gagagagtaa gaaagaaaaa gaaaagggag aagggaaagt aggggaagag cggaagagaa 4920
agagaaaatg gaagaagaaa taaaaacgag aagaaagagg acgtgtatag gaaagagaag 4980
gaaagaatta agagaagtga ttcggcgcgt agatttgggt tataagtatc ggattcggag 5040
tttttttttt agtagtttgg tttttggtta gtttcggttt tatttgttag gcgtaaggcg 5100
gatatgcgtt ggaattcggg tttttgaagt tatttttata ttttgcggaa ggttttttttg 5160
ttagatttga gtttatttta cggttttgga tttcggagta ggtcggagag agttttttga 5220
gatcggtata gtatttttttg cgtaaagatc gtttgggggga tattgtagcg ttttagtttc 5280
gttgtgatgc gttgggttat ttttttggtg ttgatttttt ttagttggtt cgacgtggtt 5340
atttgcgagt tcgatgagga cgagggtggt cgttcgcgat tgggtgttag tatcggttcg 5400
tgagattgag tgtggttcgg gtggtgtagg tcgtttaggt gcgatattat ggtcgtaggt 5460
ggggtgttta ggtcgcggga taggtgttgt ttatcgcggg ttagtatggt agtgtggtgc 5520
gcgtcgaagt tggggttgag tattttgtcg tggttcggcg gatcgtagtt gggtagattt 5580
tgttgcgcgt tgtggaggtc gtttagttcg ttgtttagcg gcgtggcggt tagcggggat 5640
aggttttggt ttatgtcggg tattttttttg tagggattgt agaggttgtt tatggtcggg 5700
agttcgcgtt cgtcgcgtat gagggtgaag ttgtcgttga cgttgtcgga taggcgttgg 5760
tggtggtggt ggtggtggtg gtgcggatgg tggtgcgggt gagggtggtg gaatttgtta 5820
gatacggtgg agatgggtgg tagcggttgg agcggtgtta gcgtggtgta ggtgttgttt 5880
atgtttatgt taggcggaga cgagtcgtag gatatgttta tggcgtggtg tagcgggatg 5940
gagagttcgg gtcggtagtc gtcgtcgttt aggatcgagg ttatgttggt gattatggtc 6000
gagcgcgacg tcgtcgttgt cgtcgttgtc gtcgtgttta gtttttggtg cgcggttgga 6060
ggcggcggag ggtttcgtag cgagttagcg gcgtcgcggt tcgcgtggtg ggggttgggg 6120

```
ttggttagta gtttttgttt atggttcggg ttttcgtcgt cgttttcgtc gttttcgtcg    6180
tcgttttcgt tattgtcgtc gtcggtcggt tcgtgtaaag tgtttagatt ttttattgtt    6240
agtttcgggt ttatggcgta gtttttttagg tttttggtga ggtatcgata ggcggtgtag    6300
gtagttttta tttagtttat tagggttcgg gggcggcggg ggcggcgggg gcggtcggcg    6360
ggcgggtaag gcgcgcgtgg cggggttcgg ttgcgggagt gggggcgggc gcgggagtga    6420
gtggagagga gtgcgcgtgc gggtgtgcgt ttcgggttgc gggcgggttc gttcggggtg    6480
ggggcggggt gggggcggac ggggcgtgcg tgcgggagag gggaggggac ggggagggag    6540
ggaggggatta tcgggtttcg tcgttcgggt cggttcgtag tttcgttatg ttcgagttcg    6600
tttcggcgtc gacgtttttt gtttgggtga gcgggagttg tttagaaggg ttttgtcgtt    6660
cgtcggggta gttaatttat tttttcggat ttggggcggg gttacgttcg acgggcgtta    6720
agcgtaatta tttcgaggtc gtagttgttt cgggaggcgg gttttttacg ggaagttatt    6780
agtggttgtg gaggggggcgg ggtcgtattt tttaggtttg gttgacggtt cggtgttttt    6840
tttatttttt ttgttattgg aattaaacgt taaggagagg gagggaggag aaggtggggg    6900
ggtcgttggg gagagggagg ttggagtgcg agcgagcgag agagattcgg tgaaagatgt    6960
cgtgttgtgt ttttttgag tagttgcgat ttgggggaag ggtattgtt ttaatcgttt    7020
tcgttttatt ttaaagtttt tttggaggcg agtaagtcgg gagtaaaaga tttcgttatg    7080
tgttagtata gataaatatt cgttattaat aagttattta tcgagagttt ttagttggga    7140
gttaatagtt tagtttttta aaatgtaggc gttaatgtat tttaatgtat ggtaaatttt    7200
cgaggtcggg atatgtataa tttacgcggt tagagtaatt gtagtgtttt cgtcgttagc    7260
gagttgcggt cggtcggcgg tttacgggcg gcgcgggtag tagtaatgta tgtaaagtta    7320
atataataat attatttaat tattcggtga gtttataaat tttagttatg aggttttcga    7380
agagttttgt tagattgtaa aataaaaatta gaatttcggg agttgtcgtt ttaaggtgat    7440
taagtgtttt ttgtattagc gatttggaaa gaagtgggga gagtgtagaa tcggtgaggt    7500
ggaggtgagg agggtcgttg gtgttttaag tgagtaaatt ttttttgggg gtagagagta    7560
agtaaattag ttgagacggt aattacggat ttttttttta atacgagata tatatacgcg    7620
tatatatata tatatatata tatatatata ttttcgtttt ttttaaattt cgggagagaa    7680
acggcggtgg gtagacgata aggaattttg gttgatattt gtatatttaa attttgtttt    7740
tgaaaagatt agcgtttcgg ttagtttgag aacgtttata agttaaggtt ttaaaaatcg    7800
tatttacgtt tttttagaat tttcgttgtt tggtaatttt aggtttggag gtgggagtgg    7860
aattgggggga tagagagttg gagatttttg tagttcgagt cggggtggag gggtagttga    7920
agatagagag gttaatattc ggtataaagt tttaaacgta ataagatttt tttaaagtag    7980
gttgcgtttt gtttgttttc gaagatttaa gttagttgag gttgcgtaga                8030
```

<210> 93
<211> 2225
<212> DNA
<213> Artificial Sequence

<220>
<223> chemically treated genomic DNA (Homo sapiens)

<400> 93

```
tatttgggtg taggcgggtt gagttcgaaa agagagttag cgaagggaga taggggtggg      60
gtcgttttat aggatttggg aaggtaatgg aaaattatag ttaaaggggg ttgtttttttg    120
gtgggtaggg gtgaatttta taaagtatat ttttaagggt ggggagaatt ataaataatt    180
tttttaaggg tggggaagat tataaagtat attgattagt tagggtgggg taggaataaa    240
ttataatggt ggaatgttat tagttaaggt tgtttttatt ttttttgtgg atttttagtt    300
attttaggtt atttggatgt atatttgtaa gttatagggg atgcgatggt ttggtttggg    360
atgcgatggt ttggtttgat aattattatt tatgttatgt ttattatttt aagttttatt    420
attattattt tatttatttt attttatttt ttttttatat attcgttttt attttggaga    480
ggttagatga gttagatttt agggaggttt agaagtgggt aaggggaaac gggaaaggag    540
gaagatggta tgggtgtgtt tggttagggg tgggagtgtt ggacggagtt cgggataaga    600
ggggttttgt agttattggt atataatgtt tgggagtttt tgttggtgtt gggattattt    660
tagtgagttt tgggagggaa ttgaagattt ttaattatta atgtatttgt ttttaaaatc    720
gacgggggga aggatatgtt taggtttaag gatacgtgta ggtttggatg atttcgggtt    780
attagggagt tttcggagta ttttgatttt tagacgggtt tgatgaaacg agtatttgat    840
ttagtaggtt tgggttcggg ttcgagaatt tgcgtttttc gcgagttttc gcgaggtaag    900
tgttgtaggt gcggggttag gagttaggtt tcgtttttgc gttcggagtc gtttttagta    960
tagggtttgt gagttttatt ttttcgcggc gcggggcggg gttgggcgcg gggtgaaaga   1020
ggcgaagcga gagcggaggt cgtattttag tattgcgtag ggatcggtga gtgtcgtttt   1080
```

tgggggtagc gtttagtaat cgcgttagga gcgcggagaa gggtattggg agagcggcgt 1140
tcgtggcgga gattagcgtt tcggagtacg ggtacgacgg gggtattttt tcggttgtta 1200
gtaattaata ataataataa ttataattat agtaagggcg ttgatgggcg ggttcggagt 1260
acgtttgatt ttggttttta ttaggttgtt taggtttttg atgacgtatt agaaatattt 1320
ttttaattcg cggttttttt gtaggagagg ttgggaaggg gtgggggacg gggttcgggg 1380
gaggttttcg aggggattta gtaagcgggg aagggcgtcg ggaaagtttt agatttacgg 1440
ttgcgcgggt tacgagttta ttcgaacgtc gattattgtt tttcgtcgat ttttattttt 1500
tgggaacgcg cgaaagtaaa tttaagttag attgcggagg tcgttgggga gggaaggttt 1560
aaggagtttt cgtcgatttt gttgaataaa gggggtttcg agttgggtcg agatggggta 1620
tgcgcgggaa gatttttgtt cgttgttttt ttttatcgtt ttagtggatg ttatgtttgg 1680
ggttttttcg gcgcgtgggg ttgacgtatt ttcggggttt atcgtagttg gtcgggatcg 1740
tggagtgggt gcggtggacg aagggaggta ggatagtttc gggggtggta gaaggagttc 1800
gggtatagtt gagatttgcg tttttatttt attaatattt atagtaggtg ttgtcgagtt 1860
gggtaattgg gatggtttaa gttatttggt taaattttaa attacgtttg ttattgggaa 1920
gtagagttta gtgatgttaa tcgcgttttt atttttatta tcggtgttag ttttaaaggg 1980
tttttaaaat ggttgtgtta ttttttagtt ttggatcgta gttgtcggtt aggaatttta 2040
gtgttacggt ggatacgttt ttttcgcgtt tttgtcgttt atttgtttat ttagtttagg 2100
ggttttggta ggtagtagtg tatttggttt aaagggtaag atgttttttt ttttatttat 2160
aataaattta aatattagta gggtttgggg ggaaaaacgt ttttagaaga aaaggtgaat 2220
gttag 2225

<210> 94
<211> 2225
<212> DNA
<213> Artificial Sequence

<220>
<223> chemically treated genomic DNA (Homo sapiens)

<400> 94

ttgatattta tttttttttt tgaaagcgtt ttttttttta aattttgttg gtatttaaat 60
ttgttatgaa taaaagagag aatattttgt tttttagatt aaatgtattg ttatttatta 120
aagtttttga gttgggtgag taggtgggcg gtaagggcgc gagggaggcg tgtttatcgt 180
gatattggga tttttggtcg gtaattgcgg tttaaggttg aaagatgata tagttatttt 240
aggagttttt tgggattgat atcggtggtg gaggtagagg cgcggttagt attattggat 300
tttatttttt agtaataaac gtgatttaaa atttaattaa ataatttggg ttattttaat 360
tgtttagttc ggtagtattt gttgtgagtg ttggtgggat gggagcgtag gttttagttg 420
tgttcgggtt tttttgttta ttttcgggat tgttttgttt tttttcgttt atcgtattta 480
ttttacgatt tcggttaatt acgatggatt tcgagggtgc gttagtttta cgcgtcgggg 540
aggttttagg tatggtattt attggggcgg tggggggggaa tagcgggtag gggttttttc 600
gcgtatgttt tatttcggtt tagttcggaa ttttttttat ttagtaggat cggcgagaat 660
tttttgaatt tttttttttt agcgatttc gtagtttgat ttgggtttgt tttcgcgcgt 720
ttttaggaaa tagaagtcga cggaaagtag tggtcggcgt tcgggtgggt tcgtggttcg 780
cgtagtcgtg gatttgaaat tttttcggcg ttttttttcg tttattagag tttttcggag 840
atttttttcg agtttcgttt tttattttt tttaattttt tttgtaggaa ggtcgcgggt 900
tagggggatg tttttgatgc gttattagga gtttgggtag tttggtggga attagaatta 960
ggcgtgtttc gagttcgttt attagcgttt ttgttatgat tatgattatt attattgtta 1020
gttattagta gtcgagaagg tgttttcgtc gtgttcgtgt ttcggagcgt tagttttcgt 1080
tacgaacgtc gttttttaa tgttttttt cgcgttttta gcgcggttat tgggcgttgt 1140
ttttagaagc gatatttatc ggtttttgcg tagtgttgga gtgcggtttt cgttttcgtt 1200
tcgtttttt tatttcgcgt ttagtttcgt ttcgcgtcgc gaagaaatga aatttataga 1260
ttttgtgttg agggcggttt cgggcgtaga aacgaaattt agtttttggt ttcgtatttg 1320
tagtatttgt ttcgcgggaa ttcgcgggag acgtaggttt tcgggttcga atttaggttt 1380
gttgaattag atgttcgttt tattaggttc gtttgaggat taaggtgttt cggaggtttt 1440
ttaatggttc ggagttattt aagtttgtac gtatttttga atttaggtat gttttttttt 1500
tcgtcggttt tgaaaataga tgtattggta attgggggtt tttagttttt ttttagggtt 1560
tattgggatg attttagtat tagtagggat ttttaggtat tgtgtgttaa tggttgtaga 1620
gttttttttg tttcgaattt cgtttagtat ttttattttt aattaggtat atttatatta 1680
tttttttttt ttttcgtttt tttttgttta tttttaggtt tttttggagt ttggtttatt 1740
tggttttttt agggtggaaa cgggtgtgtg gaaggaaaat aaaataaaat aaataaaata 1800

```
gtaataataa agtttaaaat aataaatata atataggtaa tagttgttag gttaggttat    1860
cgtattttag gttaggttat cgtatttttt gtgatttgta ggtatatatt tagatggttt    1920
aaagtaattg aagatttata aaagaagtaa aaatagtttt aattgatgat attttattat    1980
tgtgatttgt ttttgtttta ttttaattga ttaatgtatt ttgtaatttt ttttattttt    2040
aagaaggtta tttgtaattt tttttatttt tgagaatgta ttttgtgaga tttattttttg   2100
tttattagag aataattttt tttgattgta attttttatt atttttttaa attttataaa    2160
acggttttat ttttattttt tttcgttgat tttttttttcg gatttagttc gtttgtattt   2220
aggtg                                                                2225
```

<210> 95
<211> 3377
<212> DNA
<213> Artificial Sequence

<220>
<223> chemically treated genomic DNA (Homo sapiens)

<400> 95

```
tattcgcggt tttatgagtt tgtattgaag tcggatattt ataatatttt ttttaatatt      60
ttattatttt ttttttttga taattatgtt ttgagaattt gaagtaatag atatgaagag     120
ttttaaggga ttgtattaat gtacgttaag attagttttt attatatagt ttaatgatac     180
ggttttaggt ttagaaggga tgttttatat ttatagaatt ttatcgagtt taggtgttag     240
tttagagaat ttattatttt tttgtttttt tttaagagtt aattgatggg atagtatgta     300
aagtatatgc gtttttgttg ttaaaatagt agttaaaatt gtttgtttag atgtgtaaga     360
ttttatgaaa tgttttggta aaagttagtt taggagtgtt ggttaaggtg ttgaagcgaa     420
gtaaggggag ttaggattat ttttgttatt gaattttttt tttttattt tttttttaaag    480
atagcgagtt tatttatttt gataagatat tttaggattt taaatttgat ataattttgg     540
agaaagtagt gcgtgtgtgt gtgtgtgtgt gtgtgtgtgt gtgtggtgta gaagggtatt     600
tgggatatat aatttttata gaaattttag tgttataaaa attttaggag gatttatatt     660
taaaaaaata tataatagtt taagttgttt attttaattg aacgttgtgt gtttttatta     720
gatttttagt gagatttagt ttaatgagtt aattgtaaaa taaagttttt gttaagggaa     780
tggtagaaaa tgataaaatt ataattttga atgattattt aaaagtaaaa agtatttttt     840
attattgtgt ggagattttt ttatttttat ttttattcga attgaatttt ttttttttaat    900
gtttttttga atagtttgtt tttggtgttt tgaaaatgtg tacgttgata gtagtattat     960
atgttgtttt tggtatagag tgagttaata gttgaattat ttttgggtaa ttgcggattg    1020
tagttagtgt aagtgtaaga gaaggtattt tggttattaa ttcgttttat attttaatta    1080
ttttcgttcg ttttatattt tttgggtatt cgatgttttt ttttttttgtt aggtaatttg   1140
ttttcgtttt gtttaatttt tcgattttttg gttttagaga atagttttgg ttgtgggaat   1200
tggttttggg aagtttttgg cgtttttttt attggtgatt ggtaagagta atatttttat    1260
atttttaaaa agagtcgttt agtttttaat gatatttttt ggtaaaaaag aaaatgaaat    1320
atataattcg gatttcggtt tattaaacgt aggttaagag cgcggttgtt gagtttcggt    1380
tttgttgtga ggttgttgtt tttattcgtt tttcgttttt attagagttg atttgttttt    1440
tgttattttt tttcggtagt gtggatttat tgtaacgtag gaatatttgg gttggtatgg    1500
ataggatttt agtattagat gttggttgtt gttttaggga tttttcgcgg aaattattta    1560
gtatttagaa gtcgttttaa taaggaagta attagtaaag tattgttggt gggagaattt    1620
cgcgaaatat tttagaaaaa tattagaaat cgttggagat ttaatgattt acgttttttt    1680
tagtttgtgt tttttagttt tttttggagg agtattaagt ggtttgtggg tttttatttt    1740
gcggtgggag ggttgtttta agtatattta tttttgcgat agttaacgtc gggggggtttt   1800
agttttaatt agggcgagga ttacggattg tatttttttt ttttagcgta ttggtttgtt    1860
tattggaggg tgagagggac gcgtagattt tcggatttag ggttttgtt ttgtagattt     1920
ttttatttta ttcggggttt ttggtattcg tattatttag tattagtgtg attggcggtg    1980
tatttcggtt ttttcgcgtt ttattaaagt ttttgtatat ttttttttac gttgcgtttt    2040
tgttatttac gtagttgttt ttttgagcgt ttcgcgtgta taaacgcgtt ttcggttttt    2100
acgcgtcggt ggcgttgttt cgttgaggtg gcgtcggggg agttcgtaga ggcggcgggg    2160
gagggagtgc ggaggagagg attcgtgagt tgcggggatt tgggttcgtc gagtttttgg    2220
ggaagttggg tttcgttttt ttcgggcgtt tggggcgcgc gggaaggcgc ggaggacgcg    2280
tcgtatttat tcgttgttgt tgtcggttgt tgattgttat cgcggcggcg gacgcggcgt    2340
tgtgtcggag ttcggtcgtt tgtttaggtt ttaataggtt tcgggtgaat ttgcgggttc    2400
gtttatcggt tatcgtcggt tacgttaatc gcgtcgcggg ttcggggcgc gtttttcgtc    2460
ggttttttttc gcgttaattt tttttattta ttcgtcgttt aggaagcgga attcggagtt   2520
```

```
tttttttttt ttttttcgt ttaattcgta agaggcgggt tttttttttt tgtattttta    2580
ttttttttat tttttttttt tttttgaagt ttttcgaaga ttttttaaat tttgcgtttt    2640
ttcgggcgcg cgcggttaat tcggttggcg gtcgggtttt gtagcggcgt atttggagcg    2700
ggcgtcgcgg gacgcgtgtg gggcggtttt tgttcgcgtc gtttatacgg gtttagttgg    2760
tcggtcgggg ttgtttacgt cgttcggggg aattgtaggt tgcgggggag ggcggggggc    2820
ggtcgtcgag ggtttcgggg cggggtttgt ttaaggtcgt cgtttgttta aggtcgttag    2880
ttttatttgg aggttgttgg gagagttgga gttttggatg tatttgtcgt ttagattttt    2940
attttttggg ttcggttagt gggtgggggt taagttcggt tcgttttttt attttcggga    3000
aggggttaga ggtttattaa agatttggtg gttgggattt tagtagtatc gtttaagtta    3060
gcgtttggaa ggttttttttg ttagaagttt atgtttaggg ttttggaata agttttttta    3120
tttatttatt tttttaatga gtatttatta agcgtttatt atatgttagt ttatattgtt    3180
ttggaaattg aggatacgga gaggaattta gtaattatat tttaaggttg tgtagagttg    3240
ataggttttt tcggggtagg ggtgggagat agtaagtaat taagcgaata aataaataaa    3300
tatagagaaa ttaaggagat agtttaagtt attaatacga atttaaaatt aataaattag    3360
cgttttgggg gtaagag                                                    3377
```

<210> 96
<211> 3377
<212> DNA
<213> Artificial Sequence

<220>
<223> chemically treated genomic DNA (Homo sapiens)

<400> 96

```
tttttatttt tagaacgttg atttattggt tttgagttcg tgttagtagt ttaaattgtt      60
tttttgattt ttttgtattt gtttatttgt tcgtttggtt atttgttgtt ttttattttt     120
atttcgaaaa aatttattag ttttatatag ttttaaagtg tagttgttaa gtttttttttc    180
gtatttttag tttttagaat agtgtgagtt ggtatatagt aggcgtttaa taaatattta     240
ttgaaggagt gaatgaatgg ggaagtttat tttaaggttt tgaatatgga tttttggtaa     300
gggagttttt taaacgttgg tttgggcgat gttgttggga ttttagttat tagattttta     360
ataagttttt gatttttttt cgagggtgga agggcgaatc gagtttggtt tttatttatt     420
agtcggattt agaaaataga agtttaaacg gtaaatgtat ttagggtttt aatttttttta    480
gtaattttta aatgaggttg acggttttgg ataggcgacg gttttggata ggtttcgttc     540
ggagtttttc ggcggtcgtt tttcgttttt tttcgtagtt tgtagttttt tcgggcggcg     600
tggatagttt cggtcggtta gttgagttcg tgtgggcggc gcgggtagga gtcgttttat     660
acgcgtttcg cgacgttcgt tttagatgcg tcgttgtagg gttcggtcgt tagtcgagtt     720
ggtcgcgcgc gttcggggga gcgtagagtt tggaaagttt tcgagaagtt ttagaggagg     780
gaagggggtg ggaagagtgg gggtgtaaga gagaaaaatt cgttttttgc gagttgagcg     840
aggaagaggg gggggaaggt ttcgagtttc gttttttgaa cggcgggtgg atagagaagg     900
ttggcgcggg gaagatcggc gggaggcgcg tttcgggttc gcggcgcgat tggcgtgatc     960
ggcgatggtc ggtgagcgga ttcgtaggtt tattcggagt ttgttgagat ttgggtaggc    1020
ggtcgagttt cggtatagcg tcgcgttcgt cgtcgcggtg gtagttagta gtcggtagta    1080
gtagcgggtg agtgcggcgc gtttttcgcg ttttttcgcg cgttttaggc gttcgggggg    1140
aacggagttt agtttttta aaagttcggc gggtttaggt tttcgtagtt tacgggtttt     1200
tttttcgta tttttttttt cgtcgttttt gcgggttttt tcggcgttat tttagcgagg     1260
tagcgttatc gacgcgtaag gatcgggggc gcgtttgtgt acgcgggacg tttagaggag    1320
tagttgcgtg ggtagtagaa gcgtagcgtg ggagaggatg tgtaggggtt ttaatggggc    1380
gcgagggggt cggaatgtat cgttagttat attggtgtta agtgatacga gtgttagaga    1440
tttcgggtgg ggtgggggga tttatagaat agaatttttg agttcggagg tttgcgcgtt    1500
ttttttattt tttagtaaat agattagtgc gttgagagag agggatgtag ttcgtggttt    1560
tcgttttggt tagagttggg gtttttcgac gttaattatc gtagaaatag atgtatttgg    1620
aatagttttt ttatcgtagg gtaaaaattt ataaattatt taatattttt ttagaaaggg    1680
ttgaggaata taggttggag gagacgtggg ttattaggtt tttagcggtt tttgatattt    1740
ttttaagatg tttcgcgggg ttttttttatt agtaatgttt tattggttat ttttttattg   1800
aaacggtttt taagtgttgg gtgattttcg cgggaagttt ttggggtagt agttagtatt    1860
tgatgttagg attttattta tattaattta gatgtttttg cgttgtagta ggtttatatt    1920
gtcggggaga ggtggtaaga gataaattaa ttttagtgga aacgggaagc gagtgggaat    1980
aatagtttta tagtaagatc ggggtttagt agtcgcgttt ttggtttgcg tttggtggat    2040
cggaattcgg gttgtgtgtt ttattttttt ttttgttaga aaatattatt aggaattaaa    2100
```

```
cggttttttt taagagtgtg aaagtgttat ttttgttagt tattaatgag gagggcgtta    2160
aaagtttttt agagttagtt tttatagtta ggattatttt ttaaagttag aggtcggaaa    2220
gttgaataga acggaagtaa attgtttggt agagggaaga ggtatcgggt gtttaggggg    2280
tgtagagcgg gcgggggtga ttggagtgtg aaacgaattg gtggttaggg tgtttttttt    2340
tgtatttata ttagttgtag ttcgtagtta tttagaggtg gtttaattgt tagtttattt    2400
tgtgttaggg atagtatgtg gtattgttat tagcgtgtat atttttagga tattaaagat    2460
aaattgttta aaagaatatt agaagaaaag atttaattcg agtaaaagta gagataagaa    2520
aatttttata tagtagtaga aagtattttt tatttttgaa tgattattta gaattgtggt    2580
tttgttattt tttgttattt ttttgataaa aattttattt tgtaattgat ttattggatt    2640
gaattttatt gaggatttga tggaaatata tagcgtttaa ttggaataaa tagtttaaat    2700
tgttgtgtgt ttttttaaat gtagattttt ttagaatttt tgtggtatta gaatttttat    2760
aaagattatg tattttagat atttttttat attatatata tatatatata tatatatata    2820
tatacgtatt gttttttta aaattatgtt agatttgaaa ttttagggta ttttgttaaa    2880
atgaataagt tcgttgtttt taaaaagagg tgggggaggg gagatttagt ggtaaaggtg    2940
gttttgattt tttttgtttc gttttagtat tttggttaat atttttaaat tagtttttgt    3000
taagatattt tatgggattt tatatattta ggtaggtagt tttggttgtt attttggtag    3060
taagaacgta tgtgttttgt atattatttt attagttaat ttttgaaggg aaataggaag    3120
gtgatgagtt ttttgagttg gtatttgaat tcggtgggat tttgtgggtg tagagtattt    3180
tttttgaatt taaaatcgtg ttattggatt atgtggtaga gattaatttt agcgtatatt    3240
gatgtaattt ttttaagattt tttatatttg ttgttttaag tttttaaaat atggttgtta    3300
agggagaaaa atgataggat gttggggaaa atattatgga tattcgattt tagtatagat    3360
ttatagaatc gcgggtg                                                   3377
```

<210> 97
<211> 3043
<212> DNA
<213> Artificial Sequence

<220>
<223> chemically treated genomic DNA (Homo sapiens)

<400> 97

```
aaaatttaaa tttataattt gaggtatttg ggaagtcgtt tttttagggc ggaatggtta      60
cggaaattgg gtaggttggg ggttttttttg gggaatgagg gagtggagga gtttttttgtt   120
tttttgttta gattagaaag agaaacgttt taagaatttg ggtttttatt ttttaggcgt     180
tttttttgga ggttttttag ggatcgttta tagcgttttt ttttagtttt tttttttttt     240
ttattttttt ttttattcgt ttcgtttcgc gttcgtttcg tttttcggtt cgcggggttt     300
taaggacggt tggtcgtttt cgttatagtt attcggtcgt ttagagcggc ggggcgtacg     360
gggtcgagag agttagtttt agggtttggg gttgggaatt gaagtttggc gtgaaggaag     420
tgggagttcg ggtcgaggag gcgaagggga aaggaaaagc gaggggaatt tgagcgggag     480
ggttttgaga ggagcgggag gttgcgggaa ggggaggttt ggtatttttg ggggttatgg     540
gggtcggggc gcggttttcg gtttgggagg gcgcgggttt ttttcggtag cgtcgttcgt     600
tggtttagtt acgcgtgttg tgcgttgcga ggtcggcggg ggtttcgttg ggttcggggg     660
tgttttcggg ggtcgtttgc gtttagttat ggtagggcgt ttttcggtga aatggggttc     720
gaggcgggtt tcgatttcgc gtcggcgttg cggatcgttc gggagttgta gtcgcgggtg     780
ttcggtgttc ggtttgtagg tagtgttatt agttgattgt attgtggtgg ttataattat     840
taattttatt gttattaaaa taaggtatag tattatcgtc gttcggtcgg gaagaagacg     900
tcggttcggg tagttcgtag ttttcgagag aagatgtttg agaagcgcgg cgtcggcgtg     960
ggttttgcgt agtttgtttc gcgagcgttc gttgtaagtg cgaggaaatt cgcggttttt    1020
ttagatatag ttaaattgtt agtttttttt aggagttata gaagatgaaa tagttttatg    1080
ttaggaaagt aaaatttttg gaggtgaagt tttttttattt atgtaacggt taatattgta  1140
tgttgtgatt tattgtgttt atttgttatc gtttagtaga gtatttatta agttagtaat    1200
ttagttgagt tttaatttaa ttaatgaatt gtttgtttgt tataacgtgt tggggtatta    1260
atttgagatt gtaatttttt ttatgagttt agttattagg tagtgtagtt agttgattgt    1320
taatagtatt aattattaat tatacggtta ggtaaaaaga tttgggaatt cgtttaaatg    1380
agttgtttgt gtattattaa tgtgcgtggg gaagaggggt gttggaaaat gttgatttta    1440
tttattgttt attaattgtt tagttaaaag aaaaaaatta atattttagt tattaagttt    1500
ataatgtatg taatgtgtat gtatgtgggg ttttgttttg tttttttttt aatatttttt    1560
taggttttta attaaaattt tagatataag gggaatatg attttttttt tagttgattg     1620
atgtatgtta ttatatgaat atgtgattat taatttttttg agattatatt gttagtaata   1680
```

ttttgaaagt aatattgtta gtaatatttc gaaagaataa agtgttataa agatataaat 1740
tgtggttatt gttatgtgtt tatttttttt gttaaattga ttttatgatg tttattttag 1800
tttttgttat agtaatttta atttttttat taatgaagtg gttaagttgt ttttgttatg 1860
ataaaaattt ttcgggaaag ttatttatat atatagattt gttaatattt ttataagaaa 1920
tatattaata aaatttttta tatttttttt ttttatgaag agattttgtt tatttattaa 1980
gatgtatttt ttttttaag taattattta taattgttgg agtttatgta atttttagtg 2040
ggagtgatta gtaaatgtat ttatatttta aatttagttt gtttttttaag atttaaagga 2100
tttagaattt taaaattata gaaattgtat atatgggtac gtattcgttt agtttaggtt 2160
tttagttttt aagggttagg tttttaggcg tgaatttttt agcgtaacgt ttttagaatt 2220
tttatttagt aggtgttgtt tttggtaatt tggtttggaa gtggggattg gggtaatggg 2280
gttgaggaat ttaaagttta tttagttatt tttttggggg gaggggagag gatgtggtag 2340
tttattgagg atataagaag ttaaattatt tttttattga tttcgatagg atataattta 2400
aagttgacgt tttgattttt ttatatttaa gatttggatt ggttttaaaa tttgattatc 2460
gtaggggtgg gttttcgacg gtttttacgc gcgtggcgga ttttaatatt tatttaggaa 2520
atcgagggta gtcggtaggg gttggtatta atttggcggt tttcggaggg gcggggtagg 2580
ggcggggcgg ggggaggggga gaggacgttt gtagaagcgt ttttagttgt tatggaaacg 2640
ggatttagcg aagaatttag cggtcgaaaa gagttttagg aaatgtgttt ttcgggatta 2700
ttggtatttg ttggtttttc ggaataagat gttaatttgg ttaagtagtt ttggatttcg 2760
gcgtcgatgt attttttttag cgaattttag ttggtgttgc gtagagatag tagttagcgt 2820
ttgtcggtgg cgcggtttag gaggagtaga gggtttggtg agtagaatag cggtttttttg 2880
ttagttatat ttgttttatc gtaattggtt tgtaggtatt agagtttttt ttaggagaaa 2940
ttgtaatggt atatataaat agttataagt atatataaat gtatgtataa atattttttg 3000
aattttttta gtaatttttt tattcgaaaa ggattagtgg tat 3043

<210> 98
<211> 3043
<212> DNA
<213> Artificial Sequence

<220>
<223> chemically treated genomic DNA (Homo sapiens)

<400> 98

atattattga tttttttcgg gtggaagggt tgttggaagg gtttaggaaa tatttatata 60
tgtatttgta tgtatttata attatttgta tatattattg tagtttttttt tggaaagggt 120
tttggtattt gtaggttagt tacggtgagg tagatatgat tgataggagg tcgttatttt 180
atttattaga ttttttgttt tttttgggtc gcgttatcgg tagacgttga ttgttgtttt 240
tgcgtagtat tagttgagat tcgttagggg gatatatcga cgtcgagatt tagaattgtt 300
tagttaagtt ggtattttgt ttcgaggagt tagtaaatat tagtaatttc ggggggtata 360
ttttttggga tttttttcgg tcgttaggtt tttcgttggg tttcgttttt atggtaattg 420
aggacgtttt tgtaagcgtt tttttttttt ttttcgtttc gtttttgttt cgttttttcg 480

agaatcgtta agttggtatt agtttttgtc ggttgttttc ggtttttttag gtaggtgttg 540
ggattcgtta cgcgcgtggg ggtcgtcgga agtttatttt tgcggtgatt aggttttagg 600
attagtttaa attttggata tgggagagtt aaaacgttaa ttttgggttg tgttttgtcg 660
gagttagtgg gggagtagtt tagtttttta tatttttagt gggttgttat attttttttt 720
ttttttttag aaagatagtt aaataaattt taaatttttt agttttattg ttttaatttt 780
tatttttaaa ttagattgtt agggatagta tttgttaggt ggaagttttg agaacgttgc 840
gttggagaat ttacgtttga aagtttagtt tttgggagtt agaggtttga attgggcgga 900
tacgtgttta tgtgtatagt ttttgtgatt ttaaaatttt gaatttttta aattttagga 960
aataagttgg atttaaagtg taagtgtatt tattaattat ttttattaaa agttgtatag 1020
attttaatag ttgtaaatga ttatttaaaa gaaggatat attttaatga gtgaatagag 1080
tttttttatg gaaaaaagaa gtatgaaaaa ttttgttaat gtattttttta taaaagtatt 1140
aataagtttg tgtgtatgag tagtttttttc gggagatttt tgttatggta ggaataattt 1200
aattatttta ttgatagaaa agttaagatt attgtggtag gaattgagat ggatattata 1260
aagttaattt agtaaaggaa atggatatat agtaataatt ataatttatg tttttatggt 1320
attttatttt ttcgaaatat tattaataat attattttta aaatattatt aataatatag 1380
ttttaagaag ttaataatta tatgtttata taataatata tattagttag ttaagaaaaa 1440
aattatattt ttttttatat ttaaaatttt ggttaagagt ttgaaggaat attgaaaaaa 1500
aaataaaata aaattttata tatatatata ttatatatat tgtaaattta gtagttgaaa 1560

```
tgttgatttt ttttttttgg ttgagtaatt aataggtaat ggatgaaatt agtatttttt    1620
aatatttttt tttttttacgt atattgatga tgtataggta gtttatttgg acgaattttt    1680
aaattttttt atttggtcgt gtggttagtg attggtatta ttagtaatta gttaattata    1740
ttgtttagta attagattta tagaaaaaat tgtagtttta agttggtatt ttaatacgtt    1800
gtgataggta ggtaatttat tggttgagtt ggagtttaat tgagttgtta gtttggtgaa    1860
tattttatta gacgatggta aatagatata gtgaattata gtatatagta ttaatcgtta    1920
tatggatgga ggggttttat ttttagggat tttgtttttt tggtatgaga ttgttttatt    1980
ttttgtgatt tttagagaga gttaatagtt taattgtatt tggagaaatc gcgggttttt    2040
tcgtatttgt agcgggcgtt cgcggggtag gttgcgtagg atttacgtcg acgtcgcgtt    2100
ttttaggtat tttttttcga aggttgcggg ttgttcgagt cggcgttttt ttttcggtcg    2160
ggcggcggtg atgttgtgtt ttgttttgat ggtagtggag ttagtgattg taattattat    2220
agtataatta gttaatgata ttgtttataa atcgagtatc gggtattcgc ggttgtagtt    2280
ttcggacggt tcgtagcgtc gacgcggggt cggggttcgt ttcggatttt attttatcgg    2340
gggacgtttt attatggttg ggcgtaagcg gttttcgagg atattttcgg gtttagcggg    2400
attttcgtcg gtttcgtagc gtataatacg cgtagttggg ttagcgggcg gcgttgtcgg    2460
ggaggattcg cgtttttttta ggtcgggagt cgcgtttcga tttttatgat ttttaggagt    2520
gttaggtttt tttttttcgt agtttttcgt tttttttagg gtttttttcgt ttaggttttt    2580
ttcgtttttt tttttttttt cgtttttttcg gttcgggttt ttattttttt tacgttaggt    2640
tttagttttt agttttaaat tttagagttg gtttttttcga tttcgtgcgt ttcgtcgttt    2700
tgagcggtcg agtgattgtg gcgggggcga ttaatcgttt ttggaatttc gcgggtcgag    2760
gggcggggcg ggcgcgagac ggggcgggtg ggggaggggga tgggggggggg gaggaggttg    2820
agagaaagcg ttgtgggcgg tttttgaagg gtttttaagg gagacgttta gaagatggag    2880
gtttagattt ttgagacgtt tttttttttg atttagtagg agggataaag agttttttta    2940
tttttttatt ttttaagaag gtttttagtt tatttagttt tcgtgattat ttcgttttgg    3000
gaaagcggtt ttttaggtat tttaaattat aggtttgaat ttt                      3043
```

<210> 99
<211> 4908
<212> DNA
<213> Artificial Sequence

<220>
<223> chemically treated genomic DNA (Homo sapiens)

<400> 99

```
ttagaggtta cgtcggttat ttttttttgt ttgtaatgtt ttagatatat atgttttgta      60
atttagaaat ttgtggttaa gaaaggtgtt aagattcgta gaaaagttgg gatataggaa     120
agagaaacga aggttgataa tagattttac gggggtgagg gagattaggt taattagaga     180
agatagtata cgttagtttt agatgtagaa taattggatt tggatttaat aagttgagta     240
taagtggaga cgtaattaga aaagattgaa gtgaaatttt taagtaaaaa aaaaaaaaaa     300
aaaaaaaaaa ggatggagta gtagttagag gggatgttat ttttataaaa gataaataaa     360
ttgatggaat ggaattttgt agggagagag aggattagta gagagaggga ggcgtaggaa     420
aatgaggagt ggggtataaa ggtggggttt ttagggagaa gaggagtcga ttttagaggg     480
ggagaagaaa aggatataga gttggttgta tagagaaaag gggttaaggt agagaagatg     540
gcgggatggt gtgagacgga gtttatagaa atatttagag aaggcgacga ttatatagag     600
gagtttttag aggggcgaat tttgaggaa gatgaacgcg gagggtggag ttagttagaa     660
atttagaaaa attagaggcg ttttcggtag cgaaggaagt cgggaagggg ttaggttatt     720
agggtatgaa gtacgggaga aaatgggttc gaagagggag agcgttcgag agaaaaggaa     780
aattagagat taggaaaatt ataatcgata taaagataag aagagatata aggaaagggg     840
tgtaatttga ggaaggaggg ttcggtaagt aattcggtaa ttatagcgtt gggagagaac     900
gtataaaaga agggtagttt aggagttgtg gggtatcgga agagatataa tagaaaacgt     960
aggtttttat ttagtaaaaa taagttacgc gcgaagtcga ggtataaggt aagggaaggt    1020
tagaaacgag gtaattcgtg tatagatcgg gtttggttaa cgttttaggg gcggggttag    1080
gcgggtcgtt tttggggggc ggggttagtt tcggtttaat aaggggtatt cgacgtttta    1140
ttggttattt gtttcgaaag ggggaagata gtttgggcgg gtaggacgtg cggagggaga    1200

ggtagcggtg gcgcgaggtt taattcgaag cgttattggt gggattgata gttttgtgcg    1260
ttaagaagtt ggtagaaggg aaggggcggg atattagttt aggattttaa atcgtattgg    1320
tcgtttgttt cggttgagag aggcgatgtt tgaagtttat tggtttttgt gagatagggt    1380
aagaaaagta gcgcgagttt ggcggttttt ttggttattt ttttatagtg tttttgggcg    1440
```

```
tttttttgat tgaatttgat tttattggag gttgtggtaa tcgtgaatta gttgtacgaa     1500
tacggttttt tttgtttttt cgtttttttt ttttttagtc ggatttttta atttaggtat     1560
tttttttttt ttttttcgcg tttttttcgt tttcgcgttt tttttttttt tttttgtttc     1620
gcgggttttt tggcgcgttt tcggtttttt attcgttttt tggggtacgg gaagggggga     1680
gggggatatg tatgaaatta attgttggta gtggttatag gagtaggaag cgttggatat     1740
agtgatttta tatgtttata taaaaatttg aataaggtgt gagcgtaaga gagatattag     1800
tgttttaagg agaaggtttt tggtttagat tagggtttag tatatgatag gggtttagta     1860
attatttgtg gaatgaatga aggagtgaag gaaaataatt tgaaagtggg gtaggtggcg     1920
ggtgcggtgg tttatgtttg taattttaat attttgggag gtcgaagtag gtaggtcgtt     1980
tgaggtaggt taggagttta agattagttt ggttaatatg gtgaaatttt atttttatta     2040
aaaatataaa aattagacgg gcgtggtggt ataggtttgt aattttaggt attcgggagg     2100
ttgaggtagg agaattgttt gaatttagga ggcggaggtt gtagtgagtc gggattgtat     2160
tattgtatta tagtttgggt tatagagtga gattttattt taaaataaaa aaaaagaaag     2220
aaagaaagtg ggatagggga gttaagtata gtggttttag tttgtagttt tagttattgg     2280
ggaggttgcg gtaggaggat tgtttgagtt taggaggtgg aggttgtagt gagttatgat     2340
tacgttattg cgttttagtt tggcgatag agcgagattt tgttaataaa aaaaaaaaag     2400
aaagtggagt agggaattta tgagaataag gtaattattg gggtgtaag agagggtttt     2460
gatttaaagg ggtataagaa taaaataaga ttaaaggaat gtaaggaggt agttgttttg     2520
tagggtatag atgagattaa gaattgaaat tgaattaaga attgaaaggg gtataaaatt     2580
attggtaggt attggatgaa tgtatgagag tggggtgagt attgttgaag aggatgttag     2640
gaaatattgt tgggacggaa tagggtgttt gtgaaaaggg gatgtaggat tcgttgaagg     2700
ggtatgtgag gatatgagta ggttggaatt taggatgtgt gtgtgggtgg gaaagtgttt     2760
ggattgaaag aaaagaatgt ttgaattgaa agaatataga atgtcgggcg tagtagttta     2820
tgtttttgat tttagtattg tgggaggtta aggtaggagg atcgtttgag tttaggagtt     2880
cgagattaga ttagtttggg aaatataatg agattttgtt tttataaaaa taaaaataaa     2940
taattaaaaa aaaatttttt ttttgagata gagtttttgtt tcgttattta ggttaaagtg    3000
tagtggcgcg attatggttt attgttagtt ttgtttttcg ggtttacgtt attttttttgt    3060
tttagttttt ggagtagttg ggattatagg cgttcgttat tacgtttagt taatttttttg    3120
tatttttagt agagataggg ttttattgtg ttagttagga tggtttcgat tttttgattt     3180
tgtgatttat tcgtttttggt tttttaaagt gttgggatta taggtttgag ttatcgtatt    3240
cggtttaaaa atttttaaat ttaaaaaaag gggggacggg tgtagtggtt tatggttgta     3300
atttagtat tttgggaggt cgagataggt agattatttg aggttaggag ttcgagatta      3360
gtttggttaa tatggtgaaa tattattttt attaaatata taaaaataaa aaaaaatagt     3420
cgggtatggt ggtttttgtt tgtaatttta gttatttagg aggttgaggt aggagaatcg     3480
tttgaattcg ggaggtggag gttgtagtaa gttaagatcg tgttattgta ttttagtttg     3540
ggagatagag tgagatttta ttttaaaaaa aaaaaaaaaa aaaaagaaag aatatagaag     3600
aggaagagga gaggggtttt tgaagaagga agatatttga gaaatgttat ggggtaaaga     3660
ggggagaaag ttgggaaaga agttagaatt agggcggggt ttaaagaggg gtaggatgga     3720
gatttgtagg tgtggttttt agggtaggag ggttgttagg ggtaggttta tttggttatg     3780
tttattttttt atttttgttt ttagatttat ggaatttagt gatatgtttg ttggttatta    3840
ttgtttttta gattttgttt tttgggatga gattagagat ttttagagta tagagttgat     3900
ttttaggaga gttattagtc gtttttttaat ttgcgttcgt tgtcgtaatt atggtgttat    3960
cgtttatttt aagggttata agcgtttttg tttttttttag gtttgcgagt gttataaatg    4020
tgttttttatt ttgtgagtat gaggtttggg aggggaggag gatgagtttt ttttaaaggg    4080
tggttgattt ttgatttgta atttttttatt tttagggagc gtcgtagggt tatggttgtt    4140
taggtggttt tgcgtaggta gtaggaggcg tagttaaaga agtatttgat gaggagaggg     4200
gaagtttttt ttaaagtttt taattatttt agaaagggaa ttatttagtt ataggttttt     4260
tgtgagtgtt tttgattagc gatggggtag gagtttgggt ataggaggta ggtatgggaa     4320
aaagaggttt agttttgttg ttgaattggt gtacgatttt gaattaaggg tttttatttt     4380
ttcggtttta gttgttttag cgttgagaga atgtagcgag ttttgtgggt taaaaatggg     4440
ttagaggtcg ggtatagtgg tttacgtttg taattcgagt attttgggag gttgaggtag     4500
gtagattacg aggttaagag atcgagatta ttttggttaa tatggtgaaa tttcgttttt     4560
attaaaaata taaaaattag ttgggtttgg tggcgtatgt ttgtagtttt agttattcgg     4620
gaagttgagg taggagaatt atttgaattt aggaggcgga ggttgtagtg agttaagatt     4680
acgttattgt attttagttt gggcgatata gtaagatttt attttaaaaa aaaaaaatga     4740
gttagatggg ggagatgaga attgtttggg gttggggtag gataggggtt aggagaaagg     4800
gtttattaga gttttttttg gttttttata gttggaaagg agaatatagt attttagttt     4860
tagatttttt atggggtagt tttgttggta tcgatatttt tcgggaag                 4908
```

<210> 100
<211> 4908

<212> DNA
<213> Artificial Sequence

<220>
<223> chemically treated genomic DNA (Homo sapiens)

<400> 100

```
tttttcgggg ggtgtcggtg ttagtaggat tgttttatgg ggggtttgag gttggggtgt      60
tatgtttttt tttttagttg tgaaggatta gggagagttt taatgagttt ttttttttga     120
tttttatttt attttaattt tagatagttt ttattttttt tatttagttt attttttttt     180
tttgagatgg agttttgttg tgtcgtttag gttggagtgt agtggcgtga ttttggttta     240
ttataatttt cgtttttttgg gtttaagtga ttttttttgtt ttagtttttc gagtagttgg     300
gattataggt atgcgttatt aagtttagtt aatttttgta tttttagtag agacggggtt     360
ttattatgtt ggttaggatg gtttcgattt tttgatttcg tggtttgttt gttttagttt     420
tttaaagtgt tcggattata ggcgtgagtt attgtgttcg gtttttagtt tatttttgat     480
ttatagaatt cgttgtattt ttttaacgtt gggatagttg aggtcgaggg agtagaaatt     540
tttagtttaa ggtcgtatat taatttagta gtaggattgg gttttttttt tttatatttg     600
ttttttgtat ttaaattttt gttttatcgt tggttagaga tatttatagg ggatttgtgg     660
ttgagtggtt ttttttttga agtggttggg agttttggga gaggtttttt ttttttttat     720
taggtgtttt tttagttgcg ttttttgttg tttacgtaag gttatttggg tagttatgat     780
tttgcggcgt tttttaaaag tggggagttg taagttaaag gttagttatt ttttggagga     840
ggtttatttt tttttttttt tagattttat atttatagga tgaggatata tttgtgatat     900
tcgtaagttt ggaagaggta gaggcgtttg tggtttttga gatgggcggt gatattatgg     960
ttgcggtagc gggcgtaggt tggagagcgg ttgatggttt ttttggggat tagttttgtg    1020
ttttgggggt ttttggtttt attttagggg gtagagttta aggggtagtg gtagttagta    1080
ggtatgttat tgggttttat ggatttaggg gtaggagtgg aaggtaagta tagttaggtg    1140
ggtttgtttt taataatttt tttgttttgg agattatatt tgtaggtttt tattttgttt    1200
ttttttagat ttcgttttga ttttggtttt ttttttagtt tttttttttt tttattttat    1260
gatatttttt aggtattttt ttttttttaga aatttttttt tttttttttt tttgtgtttt    1320
tttttttttt tttttttttt ttttgagatg gagttttatt ttgtttttta ggttggagtg    1380
tagtggtacg attttggttt gttgtaattt ttatttttcg ggtttaagcg atttttttgt    1440
tttagttttt tgagtagttg ggattatagg taagagttat tatgttcggt tatttttttt    1500
tatttttgta tgtttagtag agatggtgtt ttattatgtt ggttaggttg gtttcgaatt    1560
tttggtttta agtgatttgt ttgtttcggt tttttaaagt gttggaatta tagttatgag    1620
ttattgtatt cgtttttttt ttttttaaat ttaaaaattt ttaggtcggg tgcggtggtt    1680
taagtttata attttagtat tttgggaggt taaggcgggt ggattataag gttaggagat    1740
cgagattatt ttggttaata tagtgaaatt ttgtttttat taaaaatata aaaaattagt    1800
tgggcgtggt ggcgggcgtt tgtagtttta gttattttag aggttgaggt aggagaatgg    1860
cgtgaattcg ggaggtagag ttggtagtga gttatgatcg cgttattgta ttttagtttg    1920
ggtgacggag taagattttg ttttaaaaaa aaaatttttt tttaattgtt tatttttatt    1980
tttatagaga taaggtttta ttatgttttt taggttggtt tggtttcgaa tttttgggtt    2040
taagcgattt ttttgttttg gtttttttata gtgttaggat tagaggtatg agttgttgcg    2100
ttcgatattt tgtatttttt taatttaaat attttttttt tttaatttaa atattttttt    2160
atttatatat atattttagg ttttaattta tttatgtttt tatatatttt tttagcgagt    2220
tttatatttt ttttttataa gtattttgtt tcgttttaat agtgtttttt ggtatttttt    2280
ttaataatgt ttattttatt tttatgtatt tatttaatgt ttattagtag ttttatgttt    2340
tttttaattt ttagtttaat tttaattttt agttttattt atgttttgta aagtaattgt    2400
ttttttgtat ttttttagtt ttattttgtt tttgtatttt tttaaattag aatttttttt    2460
tgtattttta ataattattt tatttttatg gatttttttgt tttatttttt tttttttttt    2520
tattgatagg gtttcgtttt gtcgtttagg ttggagcgta gtggcgtgat tatggtttat    2580
tgtagttttt atttttttgag tttaagtaat ttttttgtcg tagttttttt agtagttggg    2640
attataggtt ggagttatta tgtttggttt ttttgtttta tttttttttt tttttttttt    2700
ttgttttgag atggagtttt attttgtagt ttaggttgta gtgtagtagt gtaatttcgg    2760
tttattgtaa ttttcgtttt ttgggtttaa gtaatttttt tgttttagtt tttcgagtat    2820
ttgggattat aggtttgtgt tattacgttc gtttaatttt tgtattttta gtagagatgg    2880
ggttttatta tgttggttag gttggttttg aatttttgat ttgttttaag cgatttgttt    2940
gtttcggttt ttttaaagtgt tgggattata ggtatgagtt atcgtattcg ttatttgttt    3000
tatttttaag ttattttttt ttattttttt atttatttta taaatggtta ttgagttttt    3060
gttatgtgtt aggttttgat ttgggttaaa aatttttttt ttggggtatt aatgtttttt    3120
ttgcgtttat attttattta ggtttttgtg taaatatgtg aagttattgt gtttagcgtt    3180
```

```
ttttgttttt gtggttatta ttaataatta attttatgta tgtttttttt ttttttttc    3240
gtgttttagg gagcgagtgg ggaatcggga acgcgttaag gggttcgcga ggtagggaag    3300
gggagagggg gcgcgagggc gggaggggcg cgagggggag gagaggggat gtttgggtta    3360
gggagttcgg ttaagaggga ggaggacgga aaggtaggag ggatcgtgtt cgtgtagtta    3420
gtttacggtt attatagttt ttaatggagt tagatttagt taagaagacg tttaaagata    3480
ttgtgagaaa gtggttagag gaatcgttaa gttcgcgttg tttttttat tttgttttat    3540

aaagattaat gaattttaag tatcgttttt tttagtcgag gtagacgatt aatgcggttt    3600
gagattttga attgatgttt cgtttttttt tttttgttag ttttttggcg tataagatta    3660
ttagttttat taatagcgtt tcgagttgag tttcgcgtta tcgttgtttt ttttttcgta    3720
cgttttgttc gtttagattg tttttttttt ttcgaggtag gtggttaatg gggcgtcgag    3780
tattttttat tggatcgagg ttggtttcgt tttttaggaa cggttcgttt ggtttcgttt    3840
ttggggcgtt gattaagttc ggtttgtgta cgggttgttt cgtttttggt ttttttttgt    3900
tttgtatttc gatttcgcgc gtggtttgtt tttgttgagt ggaggtttgc gtttttttgtt    3960
atgttttttt cggtgtttta taatttttga gttatttttt ttttatgcgt ttttttttag    4020
cgttgtgatt atcgggttgt ttatcgggtt tttttttttt agattgtatt tttttttttg    4080
tgtttttttt tgtttttgtg tcggttgtga tttttttaat ttttgatttt tttttttttt    4140
cggacgtttt tttttttcgg atttattttt tttcgtgttt tatgttttga tagtttggtt    4200
ttttttcggt ttttttcgtt atcggggacg tttttagttt ttttgaattt ttggttggtt    4260
ttatttttcg cgtttatttt ttttaagagt tcgttttttt ggggggtttt ttgtgtaatc    4320
gtcgtttttt ttgggtattt ttgtgaattt cgttttatat tatttcgtta ttttttttgt    4380
tttggttttt tttttttgta tagttagttt tgtgtttttt tttttttttt ttttaaaatc    4440
gatttttttt ttttttgaga gttttatttt tgtgttttat tttttatttt tttacgtttt    4500
tttttttttg ttggtttttt ttttttttgt aaggttttat tttattaatt tgtttgtttt    4560
ttgtaggggt ggtatttttt ttgattattg ttttattttt tttttttttt tttttttttt    4620
tttgtttgag gattttattt taattttttt tggttgcgtt tttatttgta tttagtttgt    4680
taggtttagg tttagttgtt ttgtatttga ggttggcgtg tgttgttttt tttgattggt    4740
ttaatttttt ttattttcgt gagatttgtt gttagttttc gttttttttt tttgtgtttt    4800
agtttttttg cgggttttgg tatttttttt ggttatagat ttttgggtta tagagtatgt    4860
gtgtttgagg tattgtaggt agaaaagggt ggtcgacgtg atttttag                4908
```

<210> 101
<211> 7563
<212> DNA
<213> Artificial Sequence

<220>
<223> chemically treated genomic DNA (Homo sapiens)

<400> 101

```
gttacggcgt tcggttaata ttaaatttta agttttaaaa ataatttttt ttggttttat      60
gttttatttt tagatatatt gatgtggtga atttgttttt atagttttgg gtggttttgt     120
ttttagggta gtttgatatt tggtttgtat tttggttttt tggggttaga gttatatatt     180
ggttgtttta ttgttttggg ttaagggagt tgatttattt tagttttgtt gagcgttggg     240
ttttataggg attttttgtg gtgttttata ttttcggagg tttttgtatt ttgcgttttt     300
ataagggtat taattttttt tatgaaggtt ttgtttttag aatttagtta ttttttaaag     360
gtttatattt aaatattatt aattgaagat tagtttttaa tatgtaattt tttggggaga     420
taaaaatatt tagattataa taattttaat attatttata attatattta atatattatg     480
aatttaatat tttaaagata ttgttagatt ggattaaaat aaatgtttta atgtttatat     540
gggatttata ttaaatataa agatatagaa agatttaaag taaaaagatt ttttaaaaaa     600
agttgtttta tgataatatt aatggaaaaa tagttaatgt agttgtaatg atattaaata     660
aaagtggtta tatatagtaa gtaggaaatg gttgggcgcg gtggtttatg tcggtaattt     720
tagtattttg ggaggtcgag gagggcggat tacgaggtta ggagatcgag attattttgg     780
ttaatatggt gaaattttat ttttaataaa aaatagaaaa aattagttag gcgtggtggt     840
aggcgtttgt agttttagtt atttaggagg ttgaggtagg agaatggcgt gaattcggga     900
ggtggggttt gtagtgagtt aagatcgttt tattgtattt tagtttgggc gatagagcga     960
gattttgttt taaaaaaaaa aaaaataaaa aaagagaaaa aagaaataga aatttagtta    1020
aagatgttat aatgataaaa ggtaatatat atagataata ttataattat atgtgatata    1080
ttttatatta taatgttaaa tgtatgatta tttgataaaa ttaaataata tgatgataaa    1140
attattataa ttaggttatt aagatgaaag ggtaaatttt ataggaaaat atagcgattt    1200
```

taaattcgtg tatttttaat aaaatagtat taaattatgt agtgaaaatt ggttagttat 1260
ggtggtttgt gtttgtaatt ttagtatttt gggaggttaa ggtaggcgga tcgtttgagg 1320
ttagaagttc gagattagtt tggttaatat ggtgaaattt tatttttata aaaaatataa 1380
aaaaaaagaa aattagttgg gcgtggtggt gtgtttttgt agttttagtt attcgggagg 1440
ttgaggtagg agaattattt gaatttggga ggtataggtt tttattatta tattttagtt 1500
tgggtaatag agtgagtgag attttatttt aaaaaaaaaa aaaagtaaaa ttacgtagta 1560
taagttgata gtatataatg aaagcgtaag tagagaaatt tatatttatt aattgaaatt 1620
tttttttttc gttttttttag taattaatga gatagttaga taaaaatatt agtagaagtt 1680
aggtacggtg gtttatatat gtaattttag tattttagga ggttgaggtt ggtggttcgt 1740
ttgagtttag gaatttaaga ttagtttggg taatatggtg agattttgtt tttacgaaaa 1800
atataaaaaa ttagtttggt gtggtgatgc gtatttgtaa ttttagttat taggggtgtt 1860
gagaggggat gattatttgt gtttaggagg ttgaggttgt agtgagttgt aattgtatta 1920
ttgtatttta gtttgggtga taaagtaaga tttagtttta aaaaaaaaga aaaaaaatta 1980
atagagaaat ttgaataata tagtaagttt gattagttga tatatgtaga gttttgtatt 2040
taataagtgt aaaataattt tttaagtata tatgaaataa ttagaaaata attatatatt 2100
ggggagtaag ttttaaaaaa tatttatgaa ttgaagttat attgagtatg tttttagttt 2160
atagtgtaat ttaattggaa gttaataata aaaaggttag aaaattttta aacggttgaa 2220
gatgattaat tatatatata tatgtgtata tatatatata aatatatatg gatatacgtg 2280
tatatatacg tgtatatata cgtatatata tatgtgtata tgtgtatata aatatttata 2340
cgtgtatata tacgtgtata aattatatat atgtgtatat atatatatat atatatatgt 2400
gtgtatatat gtgtgtatat atatacgtgt gtatgtgtat atatacgtgt gtatatgtgt 2460
gtgtatatat atataagtgt gtatatatgt gtgtatatat attgtgtatg tgtgtgtata 2520
tatacgtgta tatgtgtttg tatatatacg tgtgtatatg tgcgtgtata tatgcgtgtg 2580
tatatatgtg tgtgtatata tacgtgtgtg tatatatgtg tatgtatata cgtatgtgta 2640
tatagttgat tatttttaat cgtttggaga ttttttatta tatatatttt tttttttaata 2700
ttatatggtg ttttattata tagtgttttt tacggatata gtttaagaat tatttgttat 2760
tttgttttat agaaagtgtg aaattgtagg ggagtcgatt tgtatagatt ttggtaaaaa 2820
gataattgag taggtaaatt ttagggagtt tttggatagt ggattttttt gttagtggtt 2880
ttggagtttt tagttttagt tattcgtggt taattgtggt ttaaaaatat taaatagaaa 2940
atttaggaa taaatagttt ttaggttttt aattgtgttg atttttgatt ggcgtgatga 3000
gatttagtta ttttattttt ttttatagaa agtgagtttt ttttttgttt agcgtttttt 3060
tgttgtttat aatatttatt tgttagttat tttataattt gggttattag atgattgtgg 3120
taatgattag tgtggtgttt ttaagttgtt ttgattttat tgatttttttg tttttatttt 3180
tttttggttt taaagtgtaa gagaagtgat gttggtaatt tagatatgtt aaagagaagt 3240
tgtaaatgtt ttttttaagt taaaaggtaa aaattttgga tttaataagg aaagaaaaaa 3300
aattatatgt agaggttgtt aagatttatt aaaaataaat ttatttacga aaatgtgaag 3360
aagaaaaaag aaatgtatgt tagttttgtt gttgtattat ttttggatgg aagaatagaa 3420
atatgttttt attgatcgta atgggtatta tttatggttt taggtattta ttggggtttt 3480
tggaatttat tttttatagg tatgggggggg ttattgtatt tttggtttta tagtttttta 3540
ttgtcgattt taggtttttt tggattttag gtttttttttt ttaagatgta ttttagagga 3600
ttaaaaatat attttatttg ggtttcgttt gtttttgtgg aagggtagtt tattagagga 3660
tataatttcg tgttttaatt tgtttttttt tttaaaggaa atgtggagaa aaaaaaaaag 3720
tagaaattgg aaataattaa tatttagttt attttattcg atttttaggg gaattggtga 3780
ggagtttaag atgatttttt ttttttagag aaagaattta aagtttaggg aaatagcgat 3840
aggggagttt aagattgttt ttgttagttt ttttttggtt atttttcgtt gcgatcgtag 3900
gatagttttt attagtagga gaatcgggta agtgtgtgga taagtagaga gtgtgttgaa 3960
taatttgtaa cgttttatga aatacgtatt gttatggttt tttaaaaggt tttgcggaag 4020
tcgtttgttt ttattaatta agtttttatt tatataaaag tagaagtaga agtagtttta 4080
gaaaatatat taataatttt ttattttttt gaagattaga gtagtagaaa ataggtgatt 4140
tgtattataa aattgtattt atttttttttt tttttagat tttatattat attagtttat 4200
ttgtgttacg gtgtataaaa aatggaatag gcgtttttat tatattgttt tttttttaaaa 4260
atagattatt tatattttaa ttttgttttt tttaaattcg atttttaata ggagagtttt 4320
ttattatttt agatggagtg aggttgtacg attgggatgg aagaaaggaa tttttttaaat 4380
ttgggggaat ttttgttttt tgttttaaga ttattttatt tggggtgtgg gggtgggcgc 4440
ggcggttagg gtagtggaac gtagtcgcgg ttgcgttatt tttgtatttt taggcgcgcg 4500
ggagggatcg gcggggacgc gagttgcgga ttttggcgaa ttcgggggag gtagataggg 4560
ggaggcggat atttagtcgg taggcgtttt agttttttcg tagtcggcgg gttttttttt 4620
tgatagtttt aggaaaggta gatttttttt ttagttagtt aggtaaggta aagattgttg 4680
ttgagtttgt tgttattgag ggcgtataga ttttggggag atcgaagttt gttattgcgg 4740
gattttgtgg ggtaatttgg gtttacggaa gtttttgaa agaggggaga agggtttgta 4800
ttttttttat ggaggatttt tttttttta gtatttcgtt tgatgtattt aattggtaga 4860

```
agtgagattt taataggtag tagagagcgt ttacgtggag gaggtttggg gcgtcgcggc    4920
gttattttta ttttttttcg ggatcgcgtt tatttttaaa gttatacgtc gacgaattaa    4980
tttatgtttt aaattttttt ttttagtttc gtgagttcgc ggcgatattg ggtcgtgggg    5040
tggttgggaa cggttttttt tttcggaaaa attagagaac ggtttggaga gttgaaacga    5100
gcgttcgcga gtaggttcgt gtagaatcgg gttttaggat cgttgagttt cgtagggcgt    5160
ttttgggggga cgttaggtcg tcggtttttt tgttttcgtt gagatggata acgtttcgtt    5220
ttcggagttt tggttcgtta acgtatcggg ttcggattcg gcgttgagtt gttttaacgc    5280
gtcgattttg gcgtcgttgt cggcgtcgtt ggcggtggtt gtattagttg tttacgcggt    5340
gatttgcgtc gtgggtttgg cgggtaattt cgtcgtgttg tacgtgttgt tgcgggcgtt    5400
tcgtatgaag atcgttatta atttgtttat ttttaatttg gttatcgtcg acgagttttt    5460
tacgttggtg ttgtttatta atatcgtcga ttttttgttg cggtagtggt ttttcgggga    5520
gtttatgtgt aagtttatcg tggttatcga ttagtataat atttttttta gtttttattt    5580
ttttatcgtt atgagcgtcg atcgttattt ggtggtgttg gttattgcgg agtcgcgtcg    5640
ggtggtcggt cgtatttata gcgtcgcgcg cgcggtgagt ttggtcgtgt gggggatcgt    5700
tatattcgtc gtgttgtttt tcgtagtttt cgttcggtta gacgacgagt agggtcggcg    5760
ttagtgcgtg ttagtttttt cgtagttcga ggttttttgg tggcgcgcga gtcgttttta    5820
tacgttcgtg ttgggtttcg ttattttcgt gtttattatt tgtgtttttt atattatttt    5880

gttgtgtcgg ttgtatgtta tgcggttgga tagttacgtt aaggttttgg agcgcgttaa    5940
gaagcgggtg attttttttgg tggtggtaat tttggcggtg tgtttttttt gttggacgtt    6000
ttattatttg agtatcgtgg tggcgtttat tatcgatttt tcgtagacgt cgttggttat    6060
cgttattttt tattttatta ttagtttgag ttacgttaat agttgtttta attttttttt    6120
ttacgttttt ttggacgtta gtttcgtag gaattttcgt tagttgataa tttgtcgcgc    6180
ggtagtttga ttttttttagc gttcggtttc gtaattgttc gttatttttg gttagcgagg    6240
gaggagtcgg cgttagagtg cgggattaga taggtcgttt aggtttttttg gggaaatcga    6300
ttcgcgtttt atattcgatt tagtagatcg gaagcgttgc gattgtgttc gtaggttgat    6360
tttgttaagt tttttaggtg atgcgcggtt atgtcgggtg aggagaattg aggttgagat    6420
cgttatattg agggtttttt aaagtcgagg tggaggaaga ggagggtaga ggaggagggc    6480
ggtattgttg ggaatcgttt tttttttgtt ttgtttttttg ttgttttatt cgagttttgg    6540
tagtttggaa tttggttgga gtcggaattt ttcgcgtttt cgaaggagg ttcgaggacg    6600
ttgtcggcgt ttttaggcgt cggtttcgcg cggtttttgt tgtagttggc gtatagtttc    6660
ggcgggtttt tttgtttttc gcgcgttttg ggaagacggt taggcgcgcg cgtttggcgt    6720
ttatttttgt gagattcgtt tggtttggcg tcgcgggcgg ggatcgttgt cgaggtcgtt    6780
agcgtttttt taattgggga aaatataata attcgggatg ttttatcgga gtgtgtatat    6840
atttgtgtgt gtgtgtgtgt ggtgtgcgcg cgtttgcgga ggtatgaaat gcggggaaag    6900
aggttggcgt ttggtagtga ttgtttagaa tatgatacga atagtgattt ttttttttgt    6960
ttgtttttat tttttttttt tgtttttttt tttttttatt cgtgattttt tttgaaaatg    7020
ttagattttc ggagtaagga attcggagta gttcgggcgc gcggtttcgg cgtttttttgg    7080
aggagttagc ggtcgagtgt ttagcgtcgg aggatcgcgg ggttttgtgg tggagtattt    7140
cggttgagcg cgttttatag cgttgtggta tattggttat cgttgtttat agaggatgtt    7200
aagttttatt tttatttttt taattggttt cgttttttgt tttatttaga ttagtttgtt    7260
gtaattgttt atgtgtgtat tgttttttag gattgaaaat tttataaata atgtatatgt    7320
tttaatttta cgagttgtta tgaagattaa ataagataaa aagaaatgcg atttattttt    7380
ttgtgttttt tttttttttt gttttttttgg tttcgtttta gggagagtag gaagtaggga    7440
tgatcgacgg ttttaggttt tttttgttgt atttttttata gggagttgtg gattatttgg    7500
gttttatttt ttatttgtag acggtattcg gtaggtagtt tcggtaaatc gggtaaatat    7560
tgg                                                                  7563
```

<210> 102
<211> 7563
<212> DNA
<213> Artificial Sequence

<220>
<223> chemically treated genomic DNA (Homo sapiens)

<400> 102

```
ttagtattta ttcggtttat cgggattatt tatcggatgt cgtttataag taaggaataa      60
agtttaggta atttatagtt ttttgtgaaa agtatagtag agaaaatttg aagtcgtcgg     120
ttatttttgt tttttgtttt ttttaaggcg gagttaagga agtagaggag aaagggaata     180
```

```
taagagaata gatcgtattt ttttttattt tatttgattt ttatggtaat tcgtgagatt     240
aggatatgta tattatttat aaagttttta gttttgaaaa atagtatata tatgaatagt     300
tataataaat tgatttaagt aagataagag acgaggttaa ttgggggaat gggggtgggg     360
tttggtattt tttataagta acgatgatta gtgtgttata gcgttgtgaa gcgcgtttag     420
tcggagtgtt ttattataaa gtttcgcgat ttttcgacgt taagtattcg gtcgttggtt     480
tttttagggg gcgtcggaat cgcgcgttcg ggttatttcg ggtttttttgt ttcgggagtt     540
tgatattttt aggggagatt acggatagga aagggaaaag gtaaagaaaa ggagtagggg     600
tagatagaaa gaaggattat tattcgtatt atattttagg tagttattgt taagcgttag     660
tttttttttt cgtattttat gttttcgtaa acgcgcgtat attatatata tatatatata     720
ggtgtatata tatttcgatg gggtatttcg agttgttgtg ttttttttag ttgaagaggc     780
gttggcggtt tcggtagcgg ttttcgttcg cggcgttagg ttaagcgggt tttatagggg     840
tgggcgttaa acgcgcgcgt ttgatcgttt ttttagagcg cgcggaggat aaggggattc     900
gtcggggttg tgcgttagtt atagtagggg tcgcgcggag tcgacgtttg gggggcgtcgg     960
tagcgttttc gggttttttt tcgaaggcgc ggagagtttc gattttagtt aggttttaga    1020
ttgttagggt tcgggtgggg tagtagggag tagggtaggg agggggcggt ttttagtaat    1080
atcgtttttt tttttttattt tttttttttt ttatttcggt tttagggagt ttttagtgtg    1140
gcgattttag ttttagtttt ttttattcgg tatggtcgcg tattatttgg agggtttggt    1200
aaggttaatt tgcgggtata gtcgtagcgt tttcgatttg ttaggtcggg tatggggcgc    1260
gagtcggttt ttttaggagg tttaggcggt ttgtttggtt tcgtattttg gcgtcggttt    1320
tttttcgtt ggttaggagt ggcgggtagt tgcggagtcg gacgttgggg gagttaggtt    1380
gtcgcgcggt aagttattag ttggcggagg tttttgcgga agttggcgtt taggaaggcg    1440
tagaggaagg ggttgaggta gttgttggcg tagtttaggt tggtgatgaa gtaggagata    1500
gcgatgatta gcggcgtttg cgggaggtcg gtggtgagcg ttattacggt gtttaggtgg    1560
tagggcgttt agtagaggag gtatatcgtt aggattgtta ttattaggaa ggttattcgt    1620
tttttggcgc gttttagggt tttggcgtgg ttgtttagtc gtatggtatg tagtcggtat    1680
agtagggtgg tatagaggat atagatggtg gatacgggga tggcgaagtt tagtacgagc    1740
gtgtagaggc ggttcgcgcg ttattagaag gtttcgggtt gcggaaagat tagtacgtat    1800
tggcgtcggt tttgttcgtc gtttagtcgg gcgaagattg cgaagggtag tacgacgagt    1860
gtgacgattt tttatacggt taggtttatc gcgcgcgcgg cgttgtaggt gcggtcggtt    1920
attcggcgcg atttcgtagt ggttaatatt attaggtagc ggtcggcgtt tatgacggtg    1980
aggaagtaga ggttggagaa ggtgttgtat tggtcgatag ttacgatgag tttgtatatg    2040
agtttttcga agggttattg tcgtagtagg aagtcggcga tgttgatggg tagtattagc    2100
gtgaagagtt cgtcggcgat ggttaggttg aggatgaata ggttggtgac ggtttttatg    2160
cggggcgttc gtagtaatac gtatagtacg gcggagttgt tcgttagatt tacggcgtag    2220
attatcgcgt agataattgg tatagttatc gttagcggcg tcggtagcgg cgttagagtc    2280
gacgcgttgg agtagtttag cgtcgggttc gggttcgatg cgttggcggg ttagggtttc    2340
gagaacgagg cgttgtttat tttaacgagg gtagaggagt cggcgatttg gcgtttttta    2400
aggacgtttt acggagttta gcggttttga agttcggttt tgtacggatt tgttcgcgga    2460
cgttcgtttt agttttttaa gtcgtttttt ggttttttcg gaggagggga tcgtttttag    2520
ttattttacg gtttaatgtc gtcgcggatt tacggggttg gaaagaggaa tttaaagtat    2580
aggttagttc gtcgacgtgt aattttagaa ataggcgcgg tttcgaggag gggtgggggt    2640
ggcgtcgcgg cgttttaaat tttttttacg tgagcgtttt ttgttatttg ttgaaatttt    2700
atttttatta gttgaatata ttaaacgaaa tgttagagag agaagaattt tttataggaa    2760
aaatgtaaat tttttttttt tttttttagga aattttcgta gatttaggtt attttataga    2820
atttcgtagt ggtaagtttc ggttttttta gggtttgtgc gtttttagta atagtaagtt    2880
taatagtagt ttttatttta tttggttggt tggggaaggg gtttgttttt tttggagttg    2940
ttaggagaaa agttcgtcgg ttgcggggag gttgagacgt ttgtcggttg ggtgttcgtt    3000
ttttttttgtt tgttttttttc gagttcgtta gagttcgtag ttcgcgtttt cgtcggtttt    3060
tttcgcgcgt ttggaagtgt agggatggcg tagtcgcgat tgcgttttat tgttttgatc    3120
gtcgcgttta tttttatatt ttaagtaaaa tggttttaga atagagaata gaaatttttt    3180
taaatttaag ggattttttt tttttatttt agtcgtgtaa ttttatttta tttgaaataa    3240
tagaaagttt ttttgttaag aatcgaattt aaggaaaata aagttagggt gtaagtgatt    3300
tatttttaaa ggagaataat gtagtggagg cgtttgtttt atttttttata tatcgtaata    3360
taaataggtt aatgtaatgt gaaatttgaa aggaggaaaa agtgagtgta attttataat    3420
gtaaattatt tgtttttttgt tattttggtt tttaagggga tagaagattg ttagtatgtt    3480
ttttaaaatt atttttattt ttattttttgt gtaagtaaag atttgattag taaagataaa    3540
cggttttcgt aaagtttttt agggaattat gataatgcgt attttataaa acgttataag    3600
ttgtttaata tattttttat ttatttatat atttgttcga tttttttgtt aatgagagtt    3660
attttgcgat cgtagcggag agtagttaag gaaggattag taggggtagt tttgaatttt    3720
tttgtcgtta ttttttttgga ttttggattt ttttttttagg aagggaaaat tattttaggt    3780
tttttattag tttttttaag aatcgaatga aataaattaa atattggtta tttttaattt    3840
```

```
ttgttttttt ttttttttta tattttttttt aggagagaga gtaaattaaa atacgagatt    3900
atatttttta gtaaattatt tttttataaa agtaggcgaa gtttaaataa agtgtatttt    3960
tggtttttta ggatgtattt tagagaaggg ggtttgagat ttagagagat ttaaagtcgg    4020
tagtagaaga ttgtggggtt agaaatatag tagttttttt atatttatgg gggataggtt    4080
ttaagagttt tagtggatgt ttgaaattat ggatagtatt tattgcggtt aatgggaata    4140
tgtttttgtt tttttattta aaaatgatgt aatagtaaaa ttagtatgta ttttttttttt    4200
tttttttata ttttcgtgga tagatttgtt tttagtagat tttggtaatt tttgtatatg    4260
attttttttt ttttttatt aagtttagaa tttttatttt ttaatttaaa ggaggtattt    4320
gtagtttttt tttggtatat ttgaattgtt agtattattt tttttgtatt ttggagttag    4380
agagaaataa gggtaaaaag ttagtaaaat taaggtaatt tgaagatatt atattggtta    4440
ttgttatagt tatttaataa tttagattgt gaagtgatta ataggtggat attgtagata    4500
gtagggaaac gttgggtaaa gggaggattt attttttgtg ggaaggagta agatggttgg    4560
attttattac gttaattaga agttaatata attgaaaatt taggaattgt ttattttttgg    4620
aatttttgt ttaatatttt tagattatag ttgattacga gtaattgaaa ttagggattt    4680
taaaattatt gatagggggga tttattgttt aaaagttttt tggaatttat ttatttaatt    4740
atttttttat taggatttat ataaatcggt ttttttgtag ttttatattt tttgtgaaat    4800
aaaataatag atgatttttta agttatgttc gtaagaaata ttatatgata aaatattata    4860
taatgttaaa gaaaaaatat atataataga aaatttttaa acggttgaag atgattaatt    4920
atatatatat acgtgtatat atatatatat atatatatac gtgtatatat atatatatat    4980
atatatacgt atatatatac gtatatatat atacgtgtat atatagatat atatgtacgt    5040
gtatatatat atatatatat agtgtatata tatatatata tgtatatttg tgtgtatata    5100
tatatatata tgtatacgtg tatatatata tatatacgtg tgtatatata tatatatata    5160
tatatatgtg tgtatgtgtg tatatatgta tatgtatata gtttatatac gtgtgtatat    5220
acgtgtgaat gtttgtatat atatatgtat atgtgtatat acgtgtatat atacgtatgt    5280
atatacgtgt gtttatatat gtttatgtgt atatatatat atgtatgtat atagttgatt    5340
atttttaatc gtttggagat tttttagttt ttttattgtt gatttttagt tgaattatat    5400
tgtaaattga gaatatattt aatatgattt taatttatag atatttttttg aaatttattt    5460
tttagtatat agttattttt taattatttt atgtgtattt gaaaaattgt tttgtatttg    5520

ttgggtgtaa aattttatat atgttaatta gttaaattta ttatgttgtt taaatttttt    5580
tattgatttt tttttttttt ttttgagatt gggttttgtt ttgttattta ggttgaagta    5640
tagtggtgta attgtagttt attgtagttt taattttttg ggtataagta attatttttt    5700
tttagtattt ttagtagttg ggattatagg tgcgtattat tatattaggt taattttttg    5760
tatttttcgt agaggtagga ttttattatg ttgtttaggt tggttttgag tttttgagtt    5820
taagcgaatt attaatttta gtttttttaaa gtgttgggat tatatgtgtg agttatcgtg    5880
tttggttttt gttgatattt ttatttaatt gttttattag ttattgaaag gacgggggggg    5940
gggaatttta attaatgagt gtgaattttt ttatttacgt ttttattata tgttgttaat    6000
ttgtattacg taattttatt tttttttttt tttgagatag agttttattt attttgttgt    6060
ttaggttgaa gtgtggtggt gggagtttgt gtttttttagg tttaagtgat tttttttgttt    6120
tagtttttcg agtagttggg attatagggg tatattatta cgtttagtta attttttttt    6180
tttttgtatt ttttgtggag atggagtttt attatgttgg ttagattggt ttcgaatttt    6240
tgattttaag cgattcgttt gttttggttt tttaaagtgt tgggattata ggtataagtt    6300
attatgattg gttaattttt attatataat ttgatgttgt tttattaagg gtatacgagt    6360
ttaagatcgt tatatttttt tgtggaattt atttttttat tttgatagtt taattataat    6420
gattttatta ttatgttatt tgattttatt aaatgattat atatttaata ttataatata    6480
gaatatatta tatataatta taatattatt tgtatatatt atttttttatt attataatat    6540
ttttaattag gtttttattt tttttttttt tttttttgtt tttttttttt ttgagataga    6600
gtttcgtttt gtcgtttaag ttggagtgta atggaacgat tttggtttat tgtaagtttt    6660
atttttcggg tttacgttat ttttttattt tagttttttg agtagttggg attataggcg    6720
tttgttatta cgtttggtta atttttttta tttttttatta gagatggggt tttattatgt    6780
tagttaggat ggtttcgatt ttttgatttc gtgattcgtt tttttcggtt ttttaaagtg    6840
ttgggattat cggtatgagt tatcgcgttt agttattttt tatttgttgt atgtggttat    6900
ttttgtttga tattattata gttatattaa ttgttttttt attaatatta ttatggaata    6960
gttttttttta aaaaattttt ttattttaaa ttttttttatg tttttgtatt taatgtgggt    7020
tttatgtaag tattagaata tttgttttaa tttaatttga taatgttttt gaagtattga    7080
gtttatagta tattaaatgt agttatagat agtattaggg ttgttatggt ttgaatgttt    7140
ttgttttttt aaaaaattat atattgaaag ttaattttta attgatagta tttggatatg    7200
ggtttttggg aggtgattag gttttgagaa tagagttttt atgaagggaa ttagtgtttt    7260
tataaaggcg tagagtgtaa aagttttcga gggtgtgagg tattatagag agtttttgtg    7320
aagtttaacg tttagtagag ttggggtagg ttagtttttt tgatttagag tagtaaagta    7380
gttagtgtgt gattttagtt ttaggaagtt aggatgtaag ttaggtatta agttgttttg    7440
```

```
ggagtagggt tatttaaagt tgtggagata ggtttattat attagtatgt ttggaagtga    7500
gatatggaat taaagaggat tatttttaag atttaagatt taatgttggt cgagcgtcgt    7560
ggt                                                                  7563
```

<210> 103
<211> 5849
<212> DNA
<213> Artificial Sequence

<220>
<223> chemically treated genomic DNA (Homo sapiens)

<400> 103

```
attgtaatgg tacgatttcg gtttattata attttcgttt ttcgggttga agcgattttt      60
ttgttttagt tttttaagta gttgggatta taggtatgta ttattatatt tcgttaattt     120
tgtattttta gtagagacgg ggttttttta tgttggttag gttggtttcg gattttcgat     180
tttcggtgat tcgttcgttt cgattttttta tagggttggg tttataggcg tgaggtatta    240
cgttcggtta taatttttaa atattttttt gttggtattt gttcggatta tggatttaaa     300
tgatttatta atatgatgag aatttaacgg ggatatttta aaagagataa atgaatatat     360
aggtggttcg aaatgatttt ataaagtttt ttaataatta tattttttttt tagagtgtta    420
aagaatttgg tttatttaag gtgtttattt tttaaaaaat ggttatataa atgtaaattt     480
tatttaaatt aaattttatt ttgagtttgt taaatgttat gtttagtatt atttaaggtt     540
tatattaatt gttgttttaa aaaattatat agtgttttaa gtatttttat attttaaata     600
taaaggaata attttaattg tggaattaga gttgtttttt taagataaaa agataaagat     660
aaatattata attaattatt aattggagaa atattttttt aaacgttttt gtaataattg     720
gatgtgttgg atagtataat taaatttatt aagaatagtg tgaaaaatat tattattatt     780
tagtttttat atatagatat tttttaaat tatcgaaatt tgtatttgaa tatacgaagt      840
attaagaatt tgtttttgaa aaggaaatga atatttgaag gttatttttt ttttaatttt     900
tttttatatt aataattatt gttaatattt atttttaaaa aatcgttttt attaggtatt     960
cgggaattgt ttaataaaag ttatttaatg aaattatagt ttagataaaa aaaaaaattt    1020
aattgtatag aaaatattta tgagtttatt tttttaattt atataattgc gtttttgttt    1080
ttaaagtttt gtaatttata ttagcgggaa taaaaaaatt taatatatag tagaaagttg    1140
gtttttttaaa agaaagtatt tttttttaaa ggtaatgaag gattaaaaat tttttttttt   1200
aaaaagattt tagaagtttt tttttagag aagatagaaa atattgataa atcgtatatt     1260
gtgtagatgt ttagtttcga aatgtagttt cgtagttttt tatttcgtag tttatcggtt    1320
gtatttgtat tttcgggtag cgtttttgta gggcgtaaat atttagagat ttagggattc    1380
ggtagtttcg cgtaggtttt tcgcgtgtat cgtttagcga gcgggttttt cgtagacggc    1440
gggatgtttt aggagatcgc gaggtcggcg tagttcggcg agttttcgga gggaaggcgg    1500
cgtgttagaa aggatcggat tttagtaatg gtttcggatc ggtttcgcgc ggtcgttttt    1560
tttttcgtt tcgtcgtcgg gatttttttta gttagtgttg aatttttttaa ttgggaagga   1620
tcgagttatt ttatttttcg ggaagtcgcg cgtttattcg ttttcggtag cgtcggagtt    1680
cgagtcgggg atcgggcgga tatttataga gttcgtatcg tagcgcgttg tacgttttcg    1740
ttttcgttcg tcggttcgcg ttttagcgaa ttaatcgtaa agttggttcg aagtggcgac    1800
ggcgttttcg tggagtttat tcgtcgtttt agcgcgtttt cgagtaggga tcgggtttgt    1860
cggggtttc gtcgggttcg cgcgttcgta tggtatttgt cgtacgagtc gttttcgcgt     1920
tttttttcg cgttcggtcg tttagttcgg cgtcggcgtt ttacgttttt cgagcgtagg     1980
agattttttc gtttcggttt cgttcgtcgg cgtcggcgtt tatttttcgg gcgcggaaat    2040
tcgagtttat tttttcggt tgtcggtttt gggatcgcgg gttcggttac gtagtttcgt     2100
tcggatttaa agagttttag tttttgcgaa tattaggttc gtgttgggag acgagaaggc    2160
gtttagcggg gtttagcgcg gtttcgaggg gtagggcgag gtttgtgatg tcgaggttag    2220
ttacggttag ttataggcgt tgtgtaagga attggggaaa gtttttttgtt aagaaaggga   2280
aacgttagtt tagttaaaaa aaaaaaaaaa aaaaaaaggt tggggtttaa tgagaaagtt    2340
ttttagaggt atttttttag tttttttata gtttttttggg agtttttgaa tttgtatttg   2400
aatggcgcgg ttttgggttc gcgaaggttt ggtgggttga agtcgttttt taggttttta    2460
aaggaatgta gttaggtcgg aatttatttt tttttttaat ttttagaaaa tggaaacgtt    2520
tttttcgcga ttttttttg tcgagttaga ttttaggttt ttttcgtcgc gggcgtttgg     2580
ttttttcgt tgatatttcg gacgtttagg tttgtgggat aaaaggtagt cgcggcgttt     2640
cgcgggtttt ttagttcggg agtcggtttt aggggtttta aagcgtgatt ttaggtcgat    2700
aattgtaagt ttttaggtag aggggaagtt ttagcgtcga gaagttcgtt tcgagaattt    2760
ttcgcgtcgg gcgtttagtt tgagcgtttc gggcgcggag tttattttcg gtgcgggtag    2820
```

```
ggttttagtc gtaggttcgg cgtagggcgg attcggtcga agttcggagg gtcggaggcg    2880
ttgggacgta gttcggataa gagggcggat ttgatttcgg aaagtttgtg ggatttaggt    2940
attttaatat tgggaagata aagagattcg attttttttgg gattggtgag gggagtttgg   3000
cggggagggg aacggtaggg tagttaagtg tgaaagagaa ttttttgggt ttaaagttaa    3060
cgtagttatt ttataagtta ttttagaggt gatcggaaaa aataataata ataaaggtgt    3120
ttagtaggtt gtggttggga aggaaaaata atgtttgtaa gatgagtgtg tcgattcgta    3180
gttttatatt ttttgtttat tagttggaat atatatagat ttatatattt agtaaatgta    3240
aattttatta tttaaagacg tgtttgtttc ggttataatt gttttaagtg tttataggtt    3300
aattttatat atggaatatt tcgaaagtgg gtgatttgat ttttaagttt aatcggtttg    3360
tgtcgtttgt cgaattgtaa attttagtaa aatttaaata tatcggaatt ttagtcgttt    3420
tttgggagcg aggaagcggg agtaatgtta aaaggggaaa agaagaatag taaaaaaagt    3480
ataaagatat tattttttga cgtaaaatta cgggattttt tttaaatggt ataagagaat    3540
ttttggagaa gattattatt aattaataag taataacgtt ttttagggtt tattagatgt    3600
tttatatttg tttgggaatt tcgatttttt aggtttttttg agaaatttat ttgtttggaa   3660
gaagttgtat ttcgtcgtta taggagtcgt cgagagttag ggatggagat tttagattta    3720
ggattttgtt acgattttat tgatttttaa gcgttaatag aacgaaatgg atatgtttat    3780
tagttattat aaatcgaatt attttatatg agagagagaa atgtggttta agttttatat    3840
ttgtttttta aataatcgat ttttttttaat tttgttttta aaggttcgtg gaaattaggg   3900
agggggttgg ggagacggtt ttagaaataa aacgtttttt tttagtacgg tttttatatt    3960
tagtttattt aagttgattt ttgtaattta tataaagaat gggttcgggt gggtggttag    4020
tgaagattgg ggtgagtaag cgtttacgtc gtcgagcgtc ggggaggttt aggttcgttt    4080
tcgtatagaa ttttgtaagg agcgcgtttc gttaggtgtt cggattagag ggcgttgcgt    4140
ttttgtaaat ttcgcggttt cgatgcgttt tttttaggta ttattgttat aataagttat    4200
atttaaaata atgtataaat agtatcgttt tttaagtttt ttttaatagt gttttatatg    4260
tattttatga atttttaaag ttatgtatac gaatttttta tcgtcgattc gtataaagtt    4320
agagggatgc gtagtttttg gagtcgagaa ttcgtagggc gtatacgttt tttcgtattc    4380
gtttagagtt atatatttta cgtaatttta ttaacgttta aatttaaatg ttaaaaggta    4440
tgtacgattt gaataaagaa tttattagtt tagtcgagtt ttcggttttg ggttttaaag    4500
ttttcggtag tttcgtgatt ttttcgcggt gtagatatgt gttaagagtc gggtttgaag    4560
tattaatttt gtttttttcgg gatttttagt atgtttttaa ttagagtgtt aacgttcgtg   4620
tcgtcgggat ttaggcggtc gtattttcgg aggttttcga ttagggtaaa tttaagtatt    4680
aaacgtaggc gaaaagagag tgcgcggggt cggaacgagg gttcgggagt agttaagttt    4740
gttaggacgt gtttaggtag ttagtcgtcg agttgaggag ttgcggtcgt cggttgcggc    4800
gattcgttgt tttttttttt attttttttt ttttggaggc ggtgggggag gggaggtgac    4860
gttagcgttt cgtttcgttt ttcgtttcgg gcgcggacgg gatttttcgg ttgggcgcgt    4920
tatttcgata gtggtagtag cggtcgtgga ggttgcgggg gtttcggttt ttgcggtttt    4980
tttggtcggt cgtagttgtg gcgcgggtga aacgttcgtt tttttttttc ggttgcgttt    5040
ttcgggcgtc ggcgagcgcg ggcgtttttc gtatttagcg tttggagtcg tttggagttt    5100
cgttgcgcgt atttttttcgg ttcgcggagt tttttcggcg gcgtttttta cgtcgcggat   5160

tgtaggtgat gttttttttt ttttgtagtc gaaattcgtc gcgatgtgtc gtttgttaga    5220
tttttgttgg gcggaagtat gaatagtttt tgtagattgg tttggacgat ttgtattttg    5280
tggtttttta atttttggat gtgaattttt ttttacgatt tattattatg tttttcgtgg    5340
tttttgtgtt gaaaattacg gagttgtatt tttattttta aattttttttt taatgttttt   5400
tttgttttga tcgtattttg aagtttataa ggttttgaga ggagggggcg aagggatagg    5460
ggaggagtta gttttaatg tgttttataa gttattttta gtgataggggg ttaatacgta    5520
aattattgtt tttttttcga aataatattt atttagataa atgtaaatat tgttgaggtt    5580
taatttataa agaatgcgtc gtttttatgt aagtgtttag tttttaggaa aatatttgtt    5640
gtggaatata tttttaatgt tattaaagta ttaaaatatt ttttcgaata gcgtgaaatt    5700
tcgagggagt tatttttgtg agttgatatt gtttttagaa gattgttatt aatatttaag    5760
atttttgtaa aagggggttg ggggcggttt tattatattt gtattcgttg gtataaatat    5820
gttgattagt ttagttggtc gtttttta                                       5849
```

<210> 104
<211> 5849
<212> DNA
<213> Artificial Sequence

<220>
<223> chemically treated genomic DNA (Homo sapiens)

<400> 104

```
taaaagaacg attagttaaa ttgattaata tatttatatt aacggatgta aatatgatag      60
aatcgttttt agtttttttt tgtaaggatt ttgaatgtta atgataattt tttggggata     120
atgttaattt atagaaatgg ttttttcgga attttacgtt gttcggggag gtattttggt     180
attttagtgg tattaaaaat atattttata gtagatattt ttttggaggt tggatattta     240
tataaagacg acgtattttt tataaattaa attttaatag tgtttgtatt tatttaaatg     300
gatattattt cgaggaggag atagtgattt gcgtattagt ttttattatt agggatgatt     360
tgtaagatat attaggaatt ggtttttttt ttattttttc gttttttttt tttagaattt     420
tataagtttt agagtacggt taagataaga ggagtattgg gaaagaattt gaaaataaaa     480
atatagtttc gtgattttta atataaagat tacgaagagt ataataataa gtcgtgaaaa     540
aaaatttata tttagaagtt ggagggttat aaagtataag tcgtttaggt tagtttgtag     600
agattgttta tgttttcgtt tagtaaaaat ttggtaggcg atatatcgcg acgaatttcg     660
gttgtagaag aggaagagta ttatttgtag ttcgcggcgt gggaaacgtc gtcggagggg     720
tttcgcgagt cggggaagtg cgcgtagcgg ggttttaggc ggttttaggc gttgagtgcg     780
aggaacgttc gcgttcgtcg gcgttcggga gacgtagtcg aaggaggagg gcgggcgttt     840
tattcgcgtt atagttgcgg tcggttagag agatcgtaga ggtcgggatt ttcgtagttt     900
ttacggtcgt tgttgttatt gtcggggtgg cgcgtttagt cggagaattt cgttcgcgtt     960
cggggcgggg ggcgaggcgg ggcgttgacg ttattttttt ttttttatcg tttttaaggg    1020
ggagggagtg gggaaagagg tagcgagtcg tcgtagtcgg cggtcgtaat tttttaattc    1080
ggcggttggt tgtttgggta cgttttgata aatttggttg ttttcgggtt ttcgtttcgg    1140
tttcgcgtat ttttttttcg tttgcgtttg gtgtttaagt ttgttttggt cggaagtttt    1200
cggagatgcg gtcgtttggg tttcggcggt acggacgttg gtattttggt tagggatatg    1260
ttgaagattt cggggaggta aagttggtat tttaaattcg gtttttaata tatatttata    1320
tcgcgagggg attacggagt tgtcgaggat tttagagttt agggtcgagg attcggttag    1380
attggtgagt tttttgttta aatcgtgtat gtttttttggt atttaagttt aaacgttaat    1440
gaaattacgt ggggtgtgtg gttttaagcg agtgcgggga agcgtgtgcg ttttacgggt    1500
tttcggtttt aggagttgcg tatttttttg gttttgtgcg gatcggcgat gaaagattcg    1560
tatgtatagt tttgggagtt tataaaatat atatagagta ttgttaaaaa gaatttgaga    1620
ggcgatgttg tttgtgtatt attttgaatg taatttatta tagtaatggt gtttagggag    1680
ggcgtatcgg ggtcgcgaga tttgtagggg cgtagcgttt tttagttcgg gtatttggcg    1740
aggcgcgttt tttgtagggt tttgtgcgga ggcgaatttg gatttttttcg gcgttcggcg    1800
acgtgggcgt ttgtttattt tagtttttat tgattattta ttcgggttta tttttttatat    1860
gagttataaa aattagtttg ggtggattgg atgtagaggt cgtgttggag gaaagcgttt    1920
tgtttttgga atcgtttttt taattttttt tttaattttt acgagttttt ggggataggg    1980
ttagggaggg tcgattattt aaaggataaa tgtggaattt gaattatatt tttttttttt    2040
atataaaata attcgatttg tggtagttaa taggtatgtt tatttcgttt tgttggcgtt    2100
taaggattag tagaatcgtg gtagagtttt aagtttgaaa tttttatttt tggttttcgg    2160
cggtttttgt ggcgacgaag tgtaattttt tttaggtaga tagatttttt agggagtttg    2220
gaaaatcggg atttttagat agatgtaaaa tatttagtaa gttttaggaa gcgttattat    2280
ttattaatta atggtaattt tttttaggaa tttttttgtg ttatttaaaa aaaatttcgt    2340
gattttacgt tagggaatgg tatttttgta ttttttttat tgtttttttt tttttttttt    2400
agtattattt tcgtttttttc gtttttagga aacggttgga gtttcggtgt gtttgggttt    2460
tgttgagatt tataattcgg taagcggtat aagtcggttg ggtttggaga ttaggttatt    2520
tattttcgag gtgttttatg tatgaaattg atttgtaagt atttggagta attatggtcg    2580
ggataaatac gtttttggat gatggagttt gtatttgttg gatgtgtgaa tttatgtgta    2640
ttttaattgg tgggtaggga atatagggtt acgaatcggt atatttattt tgtaggtatt    2700
attttttttt tttagttata atttgttaga tatttttatt attattattt ttttcggtta    2760
tttttaagat ggtttataag gtgattgcgt taattttgaa tttaggaaat ttttttttat    2820
atttgattgt tttgtcgttt tttttttcgt taagtttttt ttattagttt tagggaaatc    2880
ggattttttt gtttttttag tgttaaaatg tttgagtttt ataaattttt cgagattaag    2940
ttcgtttttt tgttcgggtt gcgttttagc gttttcggtt tttcgggttt cggtcgagtt    3000
cgttttgcgt cgagtttgcg gttggagttt tgttcgtatc ggggatgggt ttcgcgttcg    3060
ggacgtttag gttgggcgtt cggcgcgggg agttttcgga gcgggttttt cggcgttggg    3120
gttttttttt tgtttaggaa tttgtagttg tcggtttggg gttacgtttt ggggttttttg    3180
gggtcgattt tcgggttagg aagttcgcgg gacgtcgcga ttgtttttttg ttttataggt    3240
ttgggcgttc ggggtgttag cgggggaggt taggcgttcg cggcggggag ggtttggggt    3300
ttaattcggt agaaaaaggt cgcgggggaa gcgtttttat tttttgggga ttgaaggagg    3360
aagtagattt cgatttagtt gtattttttt ggagatttga aggacgattt tagtttatta    3420
aattttcgcg gatttagagt cgcgttattt aaatgtagat ttaggggttt ttagaaggtt    3480
atggaaaagt taaagggatg tttttgggaa gtttttttat taggttttag tttttttttt    3540
```

```
tttttttttt ttttagttaa gttaacgttt tttttttttg gtagggagtt ttttttaatt    3600
ttttgtatag cgtttgtggt tggtcgtaat taatttcggt attataagtt tcgttttgtt    3660
tttcggagtc gcgttagatt tcgttgggcg ttttttcgtt ttttagtacg gatttggtgt    3720
tcgtagggat tggggttttt tgggttcggg cgggattgcg tggtcgagtt cgcggtttta    3780
gagtcggtag tcggggaaag tgggttcgag ttttcgcgtt cgagaaatga gcgtcggcgt    3840
cggcgggcgg ggtcggggcg gagggatttt ttgcgttcgg ggagcgtgag gcgtcggcgt    3900
cgagttgggc ggtcgggcgc ggggagaggg cgcgggagcg gttcgtgcgg taggtattat    3960
gcggacgcgc gagttcggcg agggtttcgg taggttcggt ttttgttcgg gggcgcgttg    4020
agacggcggg tgagttttac gagagcgtcg tcgttatttc gggttaattt tgcggttagt    4080
tcgttggggc gcgggtcggc ggcgcggggc gagagcgtgt agcgcgttgc ggtgcggatt    4140
ttgtgggtgt tcgttcggtt ttcggttcgg gtttcggcgt tgtcgaaaac ggatgagcgc    4200
gcgatttttc ggagggtgga gtgattcggt ttttttagt tgggaaattt agtattggtt    4260
agggggggttt cggcggcgga gcgggaaaag gaggcgatcg cgcgggatcg attcggggtt    4320
attgttgggg ttcgattttt tttgatacgt cgtttttttt tcgggagttc gtcgggttgc    4380
gtcggtttcg cggtttttttg gggtatttcg tcgtttgcga aagattcgtt cgttgagcgg    4440
tgtacgcggg aggtttgcgc ggggttgtcg ggtttttggg tttttgggtg tttgcgtttt    4500
gtaggagcgt tgttcggagg tgtagatgta atcggtggat tgcggagtga agggttgcga    4560
ggttgtattt cggggttgaa tatttgtata atgtgcggtt tgttagtgtt ttttgttttt    4620
tttggaggag gaatttttaa gatttttttta aaagaaagag tttttggttt tttattgttt    4680
ttaaaaaaaa atgttttttt ttagagagtt agtttttttgt tgtgtgttag atttttttgt    4740
tttcgttgat gtgaattgta aggttttgga aataaggacg taattgtatg agttaggaaa    4800
gtaaatttat aaatattttt tatgtagtta agtttttttt tttgtttgaa ttgtggtttt    4860
attaagtggt ttttgttaag taattttcgg gtgtttgata gaggcgattt tttaaaagtg    4920
gatattaata gtgattatta gtatgagaaa agattgaaaa gaaaatgatt tttagatatt    4980
tatttttttt ttaaaagtag atttttaatg tttcgtatat ttagatatag gtttcgatag    5040
tttaagaagg tatttgtgtg tgagaattag gtaatggtga tgttttttat attattttta    5100
atgagtttgg ttatgttgtt taatatattt agttgttata gggacgtttg aaaaggtgtt    5160
tttttagtta atagttaatt gtgatatttg tttttgtttt tttgttttag gaagatagtt    5220
ttgattttat agttagaatt atttttttgt atttaagatg taaaaatatt taaagtatta    5280
tgtaattttt taaaataata attggtataa gttttgagtg atgttaaata tggtatttaa    5340
taagtttaag gtaaggtttg atttgaatga agtttgtatt tgtataatta ttttttgaaa    5400
aatgaatatt ttaaatgagt taaatttttt ggtattttgg aaagaagtgt ggttgttaga    5460
ggattttata aaattatttc gggttatttg tatatttatt tgtttttttt gaagtatttt    5520
cgttgggttt ttattatgtt agtagattat ttaaatttat ggttcggata ggtgttaata    5580
gaaaaatgtt taaaaattat ggtcgggcgt agtgttttac gtttgtaaat ttagttttat    5640
gggaggtcga ggcgggcgga ttatcggagg tcgggggttc gagattaatt tgattaatat    5700
ggagaaattt cgtttttatt aaaaatataa aattagcgga gtgtggtggt gtatgtttgt    5760
aattttagtt atttgggagg ttgaggtagg agaatcgttt taattcggga ggcggaggtt    5820
gtggtaagtc gagatcgtgt tattgtaat                                     5849
```

<210> 105
<211> 12201
<212> DNA
<213> Artificial Sequence

<220>
<223> chemically treated genomic DNA (Homo sapiens)

<400> 105

```
taataattgt gtagttatcg ttttcgtacg ttgttaataa ggtagataga tgtagaggaa      60
acgtttttat aatttttttgg aatgtagata cgaagcgtaa attgaatttt tggtagatgg     120
tagaaaaggt gatttgataa tgagtttgaa ataattaaag tgatattttt atttatttat     180
ttttataatt ttatttattt cgaggaaagt ttagaggatt ggaatatgga tataaaggag     240
gataatttaa gattgtaagt tttatttttc gaaaggttgt atttataagt aggttgttag     300
aggttgcgtt ttttttttta tttttgtat ttaggagttt ggaggatttt gttgtttatt     360
tttcgtttta cgttaatttt atgagatagg gatatcgtcg ttttttgttt agtaattttc     420
gggttgtttg attattattt tttaggttag cgattggatt tattttttttt ttcgcgtatt     480
tacgggtaat cgaggttatt cgaggttttg gtttcgaacg aatttgagta gcgagagatt     540
tataataaag aattaagtgt ggttttttag gggttgcgtt cgttatttag ggattgaaag     600
agatttgcgg gatgattttt tgttttttttt tagtaggaat tagaataagg ggcgtagatt     660
```

```
cggtttaatt tttttgtttc ggttaatggg aagatgaaat agaaaaattt ttttttattc     720
gtaatacgga gatttagggg gttgtttagt tagaaggttg gtaaattgta aaacggcggg     780
gtagggggtt aaggggacgg ttggggtttt tttgtttagt tttttttttta ttttaggatt     840
taggttaggt taaattttta gcgtcgtgtg tgtttgaaag tagataggtg ggatttttgg     900
aaagaatata tataatatag tttttgagtt ttagaaggat tttaagatag gagtttttga     960
gttttgaata agaggttatt tagggtaaag cgattgattt tcgcgcggtg cggttgagaa    1020
gagaaggcgt tgttgatttt tcggggagta agttaataat ttagtcgagt tgatgaagtg    1080
acgttttttt tgaaaggggt cgtttttttgg gttttttttt ttagtcggta tttagtaggt    1140
agggaggtag ggtaaatttt tttttttacg tgaagttata aagaattgat gagttttaat    1200
agtatggatt tgaagtcgta gataatttta aatatattcg cgttagagtt tatatttttg    1260
tttatttttg cggagttgga gtagatagcg atttttaaaa ttgtttttaa aattttttttc    1320
gaattgatcg ggtatttatt gggatttgag gggttttttt ttagttttta aattattgtt    1380
atatgtagaa tatattggta gatgttatta atgattgttt atttttttttt aaaagttgtt    1440
tcgtttacgt attatttagt ttatattaaa ttgtttcgat atgttttagt cgttagttta    1500
atttataaag tagttgtaat ttttgatatt attttcgtag tttattcgag gatttttttt    1560
taggatattt tatgaggttg tttttcgagg gttgaatttt gagatttaaa ggaaggttat    1620
ttgaggtatt ttggtatatt ttcgggtttt taattttttt ttttgtttgg ttggtaattt    1680
tttgttgttt tttttttttt tttatttgat ttttttcgag gtattttcgt tttttttttt    1740
tcgttcgttt ttagtaaatt tttagcgggt gggggaaatt aattttttgg cgttaaagtt    1800
aaatttagag ttgttcgttg gataggattg tagtggggta aatggtgttc gtttttttag    1860
aaagaatgat taattatatg gaggtaattt gttttgggtt ttttgagatt tcgggcggat    1920
tcgttcgtgt tttgttagat ttagtgtaaa tgtagatttt agttgaatcg gttaggattt    1980

gagagatagt tgttttattt ttcggattgt aattattttc gcgagaattt gatatattta    2040
atgaaagttt tgttaaatgt acgggttagc gcgtttttgg gttattatta tttgggttag    2100
tatattgtat ttattagcgt taatttgggt tttacgtagt agaattcggt taaaaatcgt    2160
ttgtttttgg ttatttagga gttttagggg gtagggggaa gtaagaaaaa ggttttattt    2220
gatattgtta gtcgtttttg ataagttata gtaaatattt ttagaagtat aattttaaag    2280
ggagaatata aattttcgtt ttcgtttggt cgaacgtttt tttgtaatta attgcggatt    2340
ttcgtatgtt taggattgaa ttgaggtttg tggtttcgta gttgtttcga tgtcgggagt    2400
atgaaatttt atttcggttg agaatcgagt aatagatcgt ataagaacgg tgttttttgg    2460
tttgtcgttt gttaagaaat attaggaaag ttggtgttag aaataattat ttgaatttag    2520
aaagtaaggt taggaagttt tgcgtgtaga gaaaattttt gttaatttat ttagattgtt    2580
ttatttggtt aataaagtaa ttgatattta ataaaattat aatcgtagta ggagttttag    2640
gtattagaat aagaattata attaaaggtt tgttgttgtt aataattgtt tttattatta    2700
ttgtttagta tttttttaag taaaaggaaa gaaaaagaga attattttat atttattggt    2760
attttttttt tttttttgaa attagagtat agattttttt gattgttttt taataagtta    2820
ttattattaa cgggaagaga cgtaaattgg ttttaaatat ttatatttcg ttcgcggtgg    2880
aatataaatg gttaatcgta atttttttta aacgtatata attttatttg atttttggat    2940
atgattaaag atattgagga tttatatagt tttattttat attattagag attatataga    3000
aaagataaaa acgggaaagg ggatatatac gtatttattt aatatttatt tttagaggta    3060
aataaaattt ttttgtttta ttttggtttt taaattatat gatgtggata ttagaaaata    3120
taaatttttt agttatatat atttttttat ttgaatattt tatttagtaa aattggtagt    3180
gtaatttatt ttattttttt aaatttgatt ttgatgtgag ttaaatatat agtattattt    3240
ttttgggaat aaatttttaa atttatattt ttattggaaa gttgattaaa gttttttttt    3300
ttttttttagt tttggttaga tgagaaggaa taagttgttt tattgtttgt gatttttttaa    3360
agaaataatt taaaatttat ttatatttgt tagaggttat tgggagataa ataaatgtta    3420
aatgtgtaat tttattttttt aattgtaatt aaaatttatt tagatttttg aaatgtagtt    3480
tgagagtatg atgtttgtgt aggaatttgt atttgatatt aagtttgatg tggaatgttt    3540
aaatggtaat taaagtagag aaagtaatag tatataaaaa tataagttta agagtttatt    3600
aggaggttgg gtgtaatagt tatattttgg aaggtgtata taagggtata tgggtagatg    3660
ttatagattt tagaggaatt taaattttag tatttttttt atattgaaag atattgttag    3720
atatattgag gtagtttgga ggtgatgggg attatattta ataattattt atatttttta    3780
ttttttttagt aaaggaatta aattagagat ttatattatt gtcgttttta aggatatatt    3840
tttgatttaa taatatataa aggttaaaac gagttttttt ttgagaggag agggtttaat    3900
tttcgttttg aatataagag gtcgtttttg tgattaagta ttacggataa attgtttatt    3960
agttttatgt atataaaatt ttatagatat atttgatgta ttaaatttat atatagtaat    4020
tagttgagga aattataata atttatgatt aatttagaat tttaaggtaa agtaaaatta    4080
taaagtttgt tattttaatt tggattttaa tgtaaaatgt gttatgatat atgtattgaa    4140
ttttagagta tgtttattat ggtggggttt tttaaacgtt tttttttaat gttggtattt    4200
ttattatgcg ttttgttttt attagtttag gaatttatta agaaaatatt tgtttataat    4260
```

```
aatggtgtag aatgaaaaga ggaaaatgtt gtttttatta ttaaggtgaa attgtttatt    4320
attaaaggtt ttttttaagt cgtatgtaag ttttttttatt tggaagtgtt attatttgta    4380
ataatttttt gtaatagttt attggtttag ggagttaaaa tattatattg tgggtgggag    4440
gtgggggttg gtttgaagga ggatggatag tgagggtgtt ttgtggattt taaatgtaag    4500
ttattaggtt tttggtggtt tttggtgttt aattttattt tataaaagtg gtatgatgga    4560
atatgattag gtaaaatttt attttttggt attgtggtgg tatttttttt tttttttttt    4620
tttattaggg ttaatatgtt tatttttatg ggaaatatat ataaatatta ggaagatttg    4680
tttcgtattg agattaatag tattagtttt gtaaaagtag tttgataaaa ggttttatat    4740
atttaatatt agagattaga aatagattaa aaaaaaaaaa aaaaaaaaat atatattata    4800
taattggtaa atttattaat ttaggtttat ttaattttcg tttttatata cgcgttttaa    4860
aataatttat aataaaaatg gaagggaaga tagatttaat ttgaaatttt ttttgagaaa    4920
aaataaatta aaattagttt ttggggaaga aagttttagt taggttagga ggaaatttac    4980
gtatttttga tttttttttc gttttggaga ggtattattc gtttaagttt agttaatgcg    5040
gtcggttagg atttatagtt tttattaagg gttagatttt gcgaaagata taaagaaagg    5100
agttttttaa tatatcgggt ttttttatag taataagata tcgaaattaa gtttttttatg    5160
gtttgtgttt gtaggatttt ttagataaaa ttggtagttt tattagataa gaaatggttt    5220
tttagaagtt ttgggggaag cgcgggggtt tttcgtggtt tttttgtgat tgtttttggg    5280
agatcgtaat agatgatggg aatatgtaag aatgattgaa gaaggttatc ggcgagttgt    5340
atttaatagt tttttttttt taagtagttt ttcgagagaa aattatatat taacgtttgg    5400
ggacgtgaaa taggtttttt attgttttgt aataaatttt tttttattcg cgtttttaga    5460
ttaatgtatt tttaaaaaat ttttataaag ttatggagat ttgtttttat aattattaaa    5520
atattatagt tagttgtata agtttgttta aaaataaagg gtaggagatt agtagtgaga    5580
agaattgaat agtatataaa aattttataa attttagatt attgaaagat tatttcgttt    5640
tttggtttcg ggttagtgag ttttaatttt agagttaaat tgagaagtag atttcgcggg    5700
tttgaaagat aaaaagttag ttcgcggagt taatttcggt ttatcggtgc gttttaattt    5760
gattgtttta atagtaagaa gcgttttttt ttttttattt aacgttttta gatatttgag    5820
gttgggggag acggagtaat ggtgtagtag ttaggaaata gttttttttgg gtttaattat    5880
tttattttcg attttttttt ttttagcgcg tcgagcgtag ttagttttgg aggaattagt    5940
ttttcggagt agttaaggta ggttaggttt cggggatgtg tattacggcg tggggattaa    6000
attaagttga acggtttgtt ttaagttttt tttttttttt agagttcggg agggatttcg    6060
ttaataggta gatttattta ttagttattc gttatcggcg gtagaaagtg agtttcgcgc    6120
gtaacgcggt tagtcggatt gcggggattt taggagcgta gggcggagga gtagcgttat    6180
aggaggcgag ggcgtaggcg gcgcggtcgg gaaggaacgc gggaggggat agaaggaaga    6240
ggaagaggag gagagggagg ttagagttag aatagttcgg tagttcgagt ttcgggggag    6300
aacggtttga gtttcgagta agttgtttcg ggagttttaa ttttttttcg ttggttcgtc    6360
gagcggttag tggcgtttag cggcggcgag gttgaaatat gataattaga atagttgcgt    6420
cgcgcgtttt gtagttaatg ggcgcggcgt tcgtttgacg ttttcgcgcg ttgcgttaga    6480
ttaatggcga tggagttgag ttggagtaga gaagtttgag taagagataa ggaagagagg    6540
tgttcgagtc gcgtcgagtt tgtcgtcgtc gtagcgtttt cgtttcgtta atttcgtcgg    6600
tttaaattgg atttttagat tcgcgagggc gcggcgtagt cgagtagcgg tttttttagt    6660
attggtaatt ttaggggtta atattttttta tttagttata gttttagtat ttttttttgtg    6720
ggttgtttat taattgtata attattattt tattgtggat attatttttt tttatagata    6780
tgggagatat gggagattta ttaaaaagta agaggttatt ttattttgtg gggttcggtg    6840
tgttgttttt gtgcggggtt tttttttagg tataggttga ggtgttaagg gtttttttgga    6900
gttggagtta ttgtttggag aaagagaaaa ggtggttttt ttttgttgtc gttacgtttg    6960
tatgtttatt gtcggttttt attttcggga aattgattgt attttgtgtg tgaattcgtt    7020
tgtgtgtttt ttaaagtttt agttttttgg tgttaagcgg tgattttttt ttggggaatt    7080
ttgagttttt cgagaaggtt attatgttgt aaaggtttgt ttgtatagtt aatgtttaga    7140
gatgtgaatt agtattagat ttgtaaaaga gaacgagtga taattgtatt tatgtttgtt    7200
tttgttaata atatttagtt ttgtgtgtta tttaagagcg cgttttacgg aaaatataga    7260
tatttttgcg tttatttaac gtttttagtt aaaatttttt ttttgatttg atttatttat    7320
aatttttttt aatgattata gtaaagagga agggggggggg gagaaatata aaatgagcgg    7380
gtttgattgc gtgttaggcg tataaatgta gattatttta atttgtattt tatatatatt    7440
ttattttttt ttaaaaatga gttaaggttt tgatggtata ttttaattat tattttaaag    7500
tgtaatgttt taaaaaaaaa aaaaaagaaa gaaagaaaga aagaaaaaaa ttttttagag    7560
tacgttttat aagagaacga tattaaaagt gtgtttattt ttgtaggaag taaacggtta    7620
gttaattatg tatttatttt tattttagaa aaacgtttga tttttttatg tgttggttgc    7680
ggtaattaga tttacgatta gtatattttg agggtttttt cggatttgga atggtatgcg    7740
gtatgtttga aatgtgcgga gtgtaattag tatttggacg agagttgtat atgttttgtt    7800
agggatggga aaatttattg taaaagagat tatattaggt atggtattta tatttttttt    7860
ttaattttgt gggatttttt tgaatttttt tattttttat gtattatttg gtgtggtttt    7920
```

gttttttttgt gaagtttgtt ttagtgtagt tatataagtt aaagttattt tgtatatgtg 7980
ttaaaaaaat taagttatgt tgtttatttt attttttagt tgagaaaaat aaaaattttt 8040
aatagtggta tttataattt cggggtattg aggtttgttt aattattttt ggagtttatg 8100
atgtataaat tatttttttt ttttattttt ttttttataa aataaaattt taaaaagatg 8160
gagaagtttg gatttttagt tttaaaatag ggttgatttt tgttgtatag tgtagtgttt 8220
tgtttgtttt agtttttttt aaaattagta gtttataaat tttttggtgg tattaatgta 8280
ataggtgaaa taaaagtttg atcgaagtat gtttagagat gtattttgaa agagcgagta 8340
taggtattgt ttttttttatt tttggggtaa gatttttttt tgagaaaaat ttaaaattaa 8400
tttaaatatt ttttggaaaa aatatcggaa atttaatttt tttaaatatt aatttttttgg 8460
tgatatttaa ttgttttttt ttttttattt tatttgagtt gatgaattag agtagattaa 8520
attgtttatt atttgtttac gaataattgg tatatttaga taattgaata gttttttttt 8580
ttatattaaa atttggtaat tgataaaatg agcgaattgt ttaaattgat aagattgaaa 8640
taatatagga attttttgag tttggtttttg ttgttttgga gagtttttgt ttttttttt 8700
ttttaattta ttttgtaata cgttttgtta attttaagtt ttttttgatt tgtgtgtatg 8760
tattagaaat tttgtttttt gttttagaaa gttagtagtt tttaaagaaa attgtattta 8820
ttttattaat aaatagaaga gatattagta ttattattat gttaaataat agtaaaatat 8880
tattttttaa atgttcggtg gttattaata agtaattaat tagtttttgt taggtaaatg 8940
gtttttggag tttgagaatt tttattaaag tttagttaag atttaatata tagttatagt 9000
ttgtttttgt ttatttagta tttagtattt tttttttttt ttttaaaaat tatgatatag 9060
agagtataat tttggtagat aaaattaatt tggttggggg aggttaatat ttcggagagg 9120
gagtgaaagg aagtaaggga agtcggggta taggaagggg gagggatttt ttaaattgtt 9180
tggttatcgt taaagttaag tttttatttt atgaaatgga agattttata ttgagtaggc 9240
ggagggagga aaaatttttg agtttatttt ttaatttttg ataaatgagc gtttttattg 9300
tttattagat tggtgtgtaa acgtaagatt ttagagaagg agagtttatt ttaggagtat 9360
ttttattgtt atggaaatag tattttgttt aattgtatat aggtttgtat gtgtttaatt 9420
ttggatatat atatgtgtag aaggaattaa ttatttttat ttttttttta ttttttttta 9480
attttgtgtg tgtgtgtgtg tgtgtgtgtg tgtgtgtgtg taattttgta gttgtaaaag 9540
tagaatagat tggatagtta gatttttata tttttttttg gagaagtagg atgttttttt 9600
ttgttatgtg gatttttttt tttttttttt atttttttttg ttcgtaggaa tgttttagtt 9660
tttgttattt ttgaaagatg gagaaggggt tagggaagtg tagtttagat ggaatttata 9720
aagattgttt tttggtaagg aaaggttagg agtgagaaag attttagaag cgggtttttg 9780
tatttttttt attttggtta tggtttttaa gaaaattgaa atgaggtaga tgatttagta 9840
atttgaaaaa gattgaggga atagacgtag atttttttaa aaaaataata atataaggaa 9900
gaatggagag gaaatttttt gttaatattg ttgtttgaag aaaattttta gttggagaaa 9960
gattggaaag tatttgtgta aaaggagatg tggaaatttt tagaggtttt attttgttat 10020
tttgtttgtt tatttgtgag tgtttgtaaa tcgagtgggg tgataatttt ttttttttat 10080
tttttttttt tttggaagga ggattttttg ttgtagtttt agatattttt agtagtagaa 10140
attgtgggat agggaagtga aagtgttggt gtcggtggtt attagagttt ttttggattt 10200
tttttgtta agatttgtaa gattaatatt gggattgatt gttagagtag tagttcgagt 10260
ttggaattta ttaatatatt ttttgtggta taagttaggt gttttgagtt aagagttatt 10320
aattaagata gaggtttatc ggaaaggaaa cgggagtaaa agaaagggag gagggaggga 10380
ggggaaaaga gagatggggg aaggaagaga gatagggaag gagagagtag ggttttattt 10440
ttgttttttt gtttttaatt tttgtttgga aatgttgttt atttggggcg ttttgttcgg 10500
gattttgggt tagggaagtg tcggtttgaa gtgatttttt ttttttgtat tttttttttc 10560
gttttgggtc gttttcgttt tttttttttt cgtataggtt gtacgggatt aaatgcgtta 10620
agtgtagtat cggttttagt aagaacgatt tcgtgatgcg tgttcgtttt aaggtgtatt 10680
atatcgagtg ttttcgttgt gtggtttgta gtcgttagtt tatttttggg gacgaatttg 10740
cgtttcggga ggacggtttt ttttgtcgag tagattacga tgtggtggag agggttagtt 10800
taggcgttgg cgattcgttt agttttttgt atttagcgcg gttattgtaa atggtaggta 10860
tttttttgtt cggttcgggt aggtaggcgt taggttaagt tagtttgtgt gttagcggtt 10920
ataataatta tggtagttat aggggtggtc gtagtgtttg tttgtagtta aatgaagtgt 10980
tttgtatgta atttgcgttg tgttttgttt ttttgtagta aggtttaatg tatttattgt 11040
ttttttttgat ttttcgagta tatttatatc gtttgtgtgt ttttatatgg ttatatataa 11100
atgtatatta tttgtgtata cgtgtatata tacgtttaaa tattattttt agttcgtttt 11160
ggtttttaaa ttttggtttg ttgaaaacgg gttttagttt ttagttaggt attttttttgt 11220
tgtttaatta aaggggcgga gtttcgggtt tttggagttt tatttttttaa tttaatgaag 11280
gaagtttagg tggtttgaag ttatttagtt ttttaaattt tttttttttg tgagttgttt 11340
tatattcgaa attttttttt taatttttttt atttttttgt gagagaatag gattgaaaag 11400
atatagtttt aaaaattgta ggttattgta tagagttgta atataaaatt gttaataagt 11460
ttatttgtag taattgtttt ttaaagggag tttgtttttt taaattattt attttatatg 11520
atttggtgag aattttattt ttaggtttat ggttgtagtt ttaaattgat tttataggtg 11580

```
ttggtttgat tttagggggt tgtagaaggt gtaggatttg tattatgtag ataagaggat   11640
ttatttttaa ggaagaggag tggaaatata gtaaggttgg tcgggattaa agtagtgggt   11700
tagaaggtgg atagtgtttt taaatttgat tttttgttat gaatagattt atttttttgt   11760
agttttaaag tattaatttt tattaaatat tgaaggtgag gaaattatag ttttttttta   11820
ttttttgtt ttttggtagt tttaagaatt ttgtttaggg ggatttgtga ttagtttgta   11880
tcggggtacg gttggggtgg tgtttttgtt tagtggagtt tgtattttgt ttgtggggaa   11940
gtatagagga agttaaaata tcgagaggga ggcgggggat atttttagt tatcgtttat   12000
ttagagttta ggtagtttaa tagagttttc gtttttttatt gtttgggatt agtttatttt   12060
aggaatattt atgtattatt ttagatttaa tattaagagt agggattgga gatatggtag   12120
aaatagcgaa tttttttagt ttttttatat gattgttttt tcggattgaa gtttaaggcg   12180
ttttggtaga gttttcgatt t                                              12201
```

<210> 106
<211> 12201
<212> DNA
<213> Artificial Sequence

<220>
<223> chemically treated genomic DNA (Homo sapiens)

<400> 106

```
aggtcgagaa ttttgttaga acgttttgaa ttttagttcg agaggatagt tatgtgaagg      60
ggttgaagag attcgttatt tttgttatat ttttaatttt tgtttttagt attaaattta     120
aggtaatata tggatgtttt tgagatgggt tgattttaag tagtggaaaa cggaaatttt     180
gttgagttgt ttaggtttta gataaacggt ggttgggaga tgttttcgt tttttttttcg     240
gtattttagt tttttttgtg ttttttttata agtagagtgt aggttttatt gaataggagt     300
attattttag tcgtgtttcg gtgtaaatta gttataaatt tttttgaata gaatttttag     360
agttgttaga aggtagaagg gtaagggagg gttatagttt ttttattttt agtgtttagt     420
gggaattgat attttaaaat tgtaaagggg tagatttatt tatggtagga agttaggttt     480
ggaaatattg tttatttttt aatttattgt tttggtttcg gttaattttg ttgtgttttt     540
attttttttt tttgggaatg agttttttta tttatatgat ataaattttg tattttttat     600
aatttttttag ggttaggtta atatttatgg ggttaattta gagttataat tatgggtttg     660
aagatgaaat ttttattaaa ttatatagaa tgaatgattt aaagaagtag gtttttttta     720
aaaggtaatt attgtagata agtttgttga tagttttata ttataatttt gtgtaatggt     780
ttgtagtttt taaaattgta tttttttagt tttgtttttt tatagaaaga taaaaaaatt     840
aaaaaaaaaa tttcgagtgt gaagtaattt ataaggaaaa aggatttggg agattaaatg     900
attttaggtt atttaagttt tttttattgg gttaaaggat gaagttttag ggattcgggg     960
tttcgttttt ttaattaggt agtaggagaa tatttggttg ggagttgaag ttcgttttta    1020
gtaagttaag gtttggaggt tagagcgaat tggaagtaat gtttgggcgt gtgtatatac    1080
gtgtatataa gtggtgtata tttatgtgta attatataga gatatataga cggtgtaggt    1140
gtgttcgggg aattaaggga gatagtgagt gtattgaatt ttgttgtaaa ggagtagagt    1200
atagcgtaaa ttgtatatag aatattttat ttaattgtag gtaaatatta cgattatttt    1260
tgtagttatt atagttgttg tggtcgttgg tatataggtt ggtttaattt ggcgtttgtt    1320
tattcgagtc gggtagagga gtatttgtta tttgtagtgg tcgcgttgga tgtaggggat    1380
tgagcgggtc gttagcgttt agattggttt tttttattat atcgtggttt gttcggtaga    1440
agagatcgtt ttttcgaagc gtaaattcgt ttttagggat gagttggcgg ttgtaggtta    1500
tatagcggaa atattcgatg tgatatattt tggagcgggt acgtattacg aagtcgtttt    1560
tgttgaagtc gatgttgtat ttggcgtatt tgatttcgta taatttgtgc gggggagggg    1620
ggagcggagg cggtttagag cggggagaga agtataggaa gagggggtta ttttaggtcg    1680
gtattttttt ggtttaagat ttcgggtaag acgttttaag taaatagtat ttttaaatag    1740
aagttggaaa tagaaggata gaaatgaaat tttgtttttt tttttttttgt tttttttttt    1800
tttttttattt tttttttttt tttttttttt tttttttttt ttttattttc gttttttttt    1860
cgatgaattt ttgttttagt tggtaatttt tagtttaaaa tatttagttt gtattataga    1920
aaatgtattg atgggtttta aattcgggtt gttgttttag taattaattt tagtgttgat    1980
tttgtagatt ttggtaggaa ggaatttaga aagattttgg tggttatcga tattaatatt    2040
tttatttttt tattttataa tttttgttgt tagaaatgtt taaaattgta ataaaaagtt    2100
tttttttttaa gaaaaaagga ggtaggagaa ggggattgtt attttattcg gtttgtaaat    2160
atttataaat aaataagtag aataataaaa tgaaattttt gagagttttt atattttttt    2220
ttgtataagt attttttagt ttttttttaa ttaaagattt tttttaggta gtaatgttaa    2280
tagaaaattt ttttttttatt tttttttgta ttattatttt tttaaaaaaa tttgcgtttg    2340
```

tttttttaat tttttttaag ttgttgaatt atttatttta ttttaatttt tttgaaaatt 2400
atgattagaa tgaaaaaaat gtaaagattc gtttttaagg ttttttttat ttttggtttt 2460
tttttattaa gggataattt ttataggttt tatttaggtt gtattttttt ggtttttttt 2520
ttattttta ggaataatag aagttggggt atttttacga atagaaagaa tggaagagag 2580
aggaaaagat ttatataata ggaggaggta ttttgttttt ttaaggagag atgtggaaat 2640
ttaattgttt agtttgtttt gtttttataa ttataagatt atatatatat atatatatat 2700
atatatatat atatatagag ttgagagaga gtgaagagaa ggtaaaaatg attagttttt 2760
tttatatatg tgtgtattta gaattaggta tatgtaagtt tgtgtgtaat tgagtaaaat 2820
attattttta tagtagtaaa gatattttg aagtgggttt ttttttttta gaattttgcg 2880
tttatatatt aatttagtaa ataatgaaaa cgtttatttg ttagaggtta gaaggtggat 2940
ttaaaagttt tttttttttt cgtttattta atgtgagatt ttttatttta tagggtgaag 3000
atttgatttt ggcggtgatt aaataattta gaaaattttt tttttttttt gtatttcgat 3060
ttttttattt tttttttatt ttttttcga agtattaatt ttttttaatt aggttaattt 3120
tatttattaa gattatattt tttgtgttat gatttttaaa aaagaagaaa aaaatgttgg 3180
atattaagtg agtaggagta aattgtgatt gtatattaaa ttttaattaa attttaataa 3240
aagtttttaa gttttagaaa ttatttgttt aataaaagtt aattaattat ttgttaatag 3300
ttatcggata tttaaaaagt gatattttgt tattatttaa tataataatg atgttgatgt 3360
tttttttgtt tgttaataaa atgaatataa ttttttttag agattattgg tttttttaagg 3420
tagaaaataa agtttttgat atatatatat aagttagagg aaatttgaga ttagtaaaac 3480
gtgttgtaga ataaattgga ggaaaaaaaa aataaaaaatt ttttaaaata ataaaattaa 3540
atttagaaag tttttatgtt gttttagttt tgttaatttg gataattcgt ttattttatt 3600
agttattaga ttttaatgtg agaaaggaag ttgtttaatt atttaaatat attaattgtt 3660
cgtagataga tgatgggtaa tttgatttgt tttaatttat tagtttagat aagataagaa 3720
agaagagata gttagatgtt attaaagggt taatatttaa aaagattaag ttttcggtgt 3780
tttttttaag gaatatttag gttggtttta aattttttttt agaaggaggt tttattttaa 3840
aaataaaagg agtaatattt gtattcgttt ttttaaagta tatttttaaa tatgtttcgg 3900
ttaaattttt attttattta ttgtattgat gttattaaag aatttgtaag ttattaattt 3960
taaaaaggat taaaataaat agaatattgt attatataat aaaaattaat tttattttaa 4020
agttaaaaat ttaaattttt ttattttttt aaaattttgt tttgtaaggg ggagggtgaa 4080
agaggaaaat aatttgtgta ttataaattt taagagtaat taaataagtt ttaatatttc 4140
ggaattatga atattattgt taagggtttt tgtttttttt aattaaagaa tgaaatgaat 4200
agtatagttt gattttttta atatatgtat agggtaattt tggtttgtat gattatattg 4260
aggtaaattt tataaaaaga taaagttata ttaaataata tataaagagt ggggagattt 4320
agggaaattt tataaaatta agaaagaagt gtaaatgtta tatttgatat aatttttttt 4380
atagtaggtt tttttatttt taataaagta tgtatagttt tcgtttaaat attgattata 4440
tttcgtatat tttaaatatg tcgtatgtta ttttaaattc ggagaaattt ttagaatata 4500
ttgatcgtga atttgattgt cgtaattaat atatagggaa attagacgtt tttttgaaat 4560
gaaaataaat atatgattga ttaatcgttt atttttttata gagataaaata tatttttagt 4620
gtcgtttttt tatagggcgt attttgggag gttttttttt tttttttttt tttttttttt 4680
tttttttttt agagtattgt attttgggat ggtaattgaa atgtgttatt aaaattttga 4740
tttattttta aaaggaggtg gaatatatgt aaaatgtaga ttggaatagt ttatatttgt 4800
acgtttagta cgtaattaaa ttcgtttatt ttgtattttt tttttttttt ttttttttg 4860
ttataattat tgggaaagat tatggataag ttaagttaaa gaaaaggttt tgattagaag 4920
cgttaagtga acgtagggat gtttatattt ttcgtgaagc gcgtttttaa atagtatata 4980
ggattggata ttgttagtaa gagtaggtat aaatatagtt gttattcgtt tttttttgta 5040
agtttaatgt taatttatat ttttgggtat tgattgtgta ggtagatttt tgtaatataa 5100
taattttttc ggagagttta ggattttttta ggaggaaatt atcgtttagt attagaaagt 5160
tagagttttg gagggtatat aggcgaattt atatataagg tataattagt ttttcgaaga 5220
taagaatcga tagtaagtat gtaggcgtgg cggtaataag aaaaagttat tttttttttt 5280
tttttaggta atgattttaa ttttaaagag tttttggtat tttagtttgt gtttgagaga 5340
gaatttcgta taagaatagt atatcgagtt ttataaggta aaatagtttt ttattttttg 5400
gtggattttt tatgtttttt atatttgtaa gagggagtaa tgtttatagt gaaatggtgg 5460
ttgtatagtt ggtgaatagt ttatagagag gatgttggag ttgtggttaa gtgggaaata 5520
ttggtttttg gggttgttaa tgttgaaaga gtcgttgttc ggttgcgtcg cgttttcgcg 5580
gatttaggag tttaatttaa gtcggcggag ttggcggagc ggaggcgttg cggcggcggt 5640
agattcggcg cggttcgggt attttttttt tttatttttt atttaaattt ttttgtttta 5700
atttagtttt atcgttattg gtttgacgta gcgcgcgggg acgttaggcg agcgtcgcgt 5760
ttattggttg tagggcgcgc ggcgtagttg ttttgattat tatattttag tttcgtcgtc 5820
gttgagcgtt attgatcgtt cggcgagtta gcgggagagg attagggttt tcgaggtaat 5880
ttgttcgggg tttaggtcgt tttttttcga agttcgggtt gtcgggttgt tttggttttg 5940
gttttttttt tttttttttt tttttttttt tgtttttttt cgcgtttttt ttcggtcgcg 6000

```
tcgtttgcgt tttcgttttt tgtggcgttg ttttttcgtt ttgcgttttt ggggttttcg    6060
tagttcggtt ggtcgcgttg cgcgcggggt ttattttttg tcgtcggtgg cgagtggttg    6120
gtgggtaggt ttatttgttg acgaggtttt tttcgggttt taggaaaagg ggggaatttg    6180
gagtaggtcg tttagtttgg tttggttttt acgtcgtggt gtatattttc ggggtttggt    6240
ttgttttggt tgtttcggag ggttagtttt tttagaattg gttgcgttcg gcgcgttagg    6300
ggaagaagaa tcgggaatgg gatgattaaa tttagaggaa ttgtttttttg attgttatat    6360
tattatttcg ttttttttaa ttttagatat ttaaaaacgt tgggtgggag gaaaggggcg    6420

tttttgttg ttgggataat taggttggaa cgtatcggta aatcgaagtt gatttcgcgg    6480
attgattttt tgttttttag attcgcgagg tttgtttttt agtttaattt tagaattgaa    6540
gtttattaat tcgaagttaa agagcgaaat gattttttag tagtttaaag tttgtaagat    6600
ttttatatat tgtttaattt tttttattgt tggtttttg tttttgttt ttaagtaaat    6660
ttgtgtaatt agttgtggta ttttggtaat tataaggata agtttttatg attttgtgga    6720
gatttttaa aaatgtatta gtttaagggc gcgagtaggg agggatttat tatagaatag    6780
tgaaagattt attttacgtt tttaggcgtt aatatataat ttttttcga gaaattattt    6840
ggagagagaa gattgttagg tgtaattcgt cggtaatttt ttttaattat ttttatatgt    6900
ttttattatt tgttacggtt ttttaaagat agttatagga aggttacgaa gaattttcgc    6960
gtttttttta aagtttttaa aaagttattt tttatttaat ggggttgtta gttttattta    7020
aaaagtttta tagatataaa ttatagaagg tttaatttcg gtgttttgtt gttgtgagaa    7080
gattcggtgt gttaggaaat tttttttttt atatttttcg taaaatttaa tttttgataa    7140
gagttgtaaa ttttggtcgg tcgtattggt tgggtttgaa cgggtaatgt tttttaaag    7200
cggaaaggga attaaagatg cgtaagtttt tttttgattt agttgaggtt ttttttttta    7260
ggaattagtt ttgatttgtt ttttttaag gaggatttta aattggattt gtttttttt    7320
ttatttttat tataagttgt tttagagcgc gtgtatgaaa gcgaaagttg gatagattta    7380
ggttgataag tttattagtt gtgtggtgtg tgttttttt ttttttttt tttggtttgt    7440
ttttagtttt tggtattaga tgtatggagt tttttgttag gttgtttttg taaagttgat    7500
gttgttaatt ttaatacgaa gtaaattttt ttagtatttg tgtgtgtttt ttatagagat    7560
aaatatgttg attttagtaa aaaaaaaaaa aaaaaaagat attattataa tgttagaaaa    7620
tagagtttta tttagttata ttttattatg ttatttttat aagatggaat taaatattag    7680
aagttattag aagtttgatg atttatattt gagatttata aagtattttt attatttatt    7740
ttttttaaa ttagtttta ttttttattt atagtgtggt attttaattt tttaaattaa    7800
taagttatta taaagaatta ttgtagatga tagtattttt aagtagaaaa gtttgtatgc    7860
ggtttggggg aaatttttag taatagataa ttttatttta atggtgaaga taatattttt    7920
tttttttat tttgtattat tgttgtaaat aagtattttt ttaataagtt tttaaattga    7980
tagaagtaag acgtatggtg aagatattag tattaaaagg agacgtttga gggattttat    8040
tatgatggat atgttttgaa gtttagtgta tgtattatga tatattttgt attaaaattt    8100
aagttagaat agtaggtttt gtagttttat tttattttga agttttaaat tgattatgga    8160
ttattatgat ttttttagtt gattgttgta tgtggattta gtgtattaaa tatatttgta    8220
aaattttgta tatataaaat tgatgagtaa tttattcgta atatttgatt ataggagcga    8280
tttttgtgt ttaaagcggg gattagattt ttttttttta aaaaaggatt cgttttgatt    8340
tttgtatatt gttaggttaa aaatatattt ttgaaaacgg taatagtgtg aatttttggt    8400
ttgatttttt tattgagaaa atgaggagta tgagtagtta ttaagtgtag tttttattat    8460
ttttaggttg ttttaatata tttgatagtg ttttttaata taaaagggat attgaaattt    8520
aagttttttt ggagtttgtg gtatttgttt atgtgttttt gtatatattt tttaaaatgt    8580
ggttgttata tttaattttt tgatgggttt ttagatttgt gtttttgtgt attgttgttt    8640
tttttgtttt agttattatt taaatatttt atattaaatt taatgttaga tatagatttt    8700
tgtataaata ttatgttttt aagttatatt ttaaaaattt aaataggttt taattgtaat    8760
tgaaaggtaa aattatatat ttagtattta tttatttttt aataattttt ggtagatata    8820
aatgaatttt aagttgtttt tttgggaaat tataaatagt aagatagttt attttttttt    8880
atttaattaa gattgggaag ggaagagggg ttttgattag ttttttaatg gaggtataaa    8940
tttgagaatt tatttttagg gagatgatgt tatatgttta gtttatatta gaattaggtt    9000
tgaaagagta gaataagtta tattgttagt tttattgagt aaggtattta gatgaaaaaa    9060
tatgtataat tgaaggattt atattttttg atgtttatat tatataattt gggggttaaa    9120
atgaaatagg aagattttat ttattttaa aaatggatat tgagtagatg cgtatgtatt    9180
ttttttttcg tttttatttt ttttgtgtag tttttaatga tgtagaataa ggttatataa    9240
atttttagta tttttaatta tgtttaaggg ttagataaaa ttatgtgcgt ttaaagggag    9300
ttacgattaa ttatttatat tttatcgcga acggggtgtg ggtgtttaga attaatttgc    9360
gtttttttc gttggtggtg gtggtttatt gaggaataat taggagaatt tgtgttttaa    9420
ttttagagaa aaggaagaga tattagtggg tatgaaatga tttttttttt ttttttttt    9480
atttaaaaaa atattaagta atgataataa aggtagttgt taataataat aaatttttag    9540
ttgtgatttt tattttgatg tttaggattt ttgttgcgat tgtggttttg ttgagtgtta    9600
```

```
attgttttat taattagata aaatagtttg ggtgaattag taagaatttt ttttgtacgt    9660
agagtttttt agttttattt tttgagttta ggtagttgtt tttgatatta gttttttttgg   9720
tattttttgg tagacgatag gttagaaggt atcgtttttg tgcggtttgt tgttcggttt    9780
ttagtcgaga tagaatttta tgttttcggt atcggggtag ttgcgaaatt ataaatttta    9840
gtttagtttt gagtatgcga aggttcgtag ttgattgtag ggaggcgttc ggttaagcga    9900
ggacggaaat ttgtattttt tttttaaaat tgtgtttttg aaagtattta ttatgattta    9960
ttaaaagcgg ttggtagtgt taagtgaaat tttttttttg tttttttttg tttttttagaa  10020
tttttaagtg attaggaata gacgattttt aatcgagttt tgttgcgtgg agtttaggtt   10080
ggcgttagtg agtgtagtgt gttgatttaa gtggtggtgg tttaaggacg cgttgattcg   10140
tatatttggt aaagttttta ttaaatgtat tagattttcg cggaagtggt tgtagttcgg   10200
agagtgaaat agttattttt taagttttga tcggtttagt tgaaatttat atttgtattg   10260
aatttggtaa aatacggacg ggttcgttcg agattttagg aaatttagag taagttattt   10320
ttatgtagtt gattattttt tttgaaagga cggatattat ttattttatt gtaattttgt   10380
ttaacgggta gttttgagtt tagttttagc gttaaaggat tgattttttt tattcgttgg   10440
agatttattg gggacgagcg gggggaggga ggcgaaggta tttcggagga ggttaagtgg   10500
gggaggagga ggagtagtag aaaattatta gttaagtagg ggagggagtt ggaagttcgg   10560
aaatatatta ggatgtttta agtaattttt ttttaagttt tagaatttag ttttcggaaa   10620
atagttttat aaggtgtttt gggaaggagt tttcgggtgg gttgcggagg tgatgttaag   10680
agttatagtt attttatgaa ttaaattgac ggttaaggta tgtcgggata gtttagtgta   10740
agttgaatgg tgcgtggacg gggtaatttt taaagagagg tgggtaatta ttggtggtat   10800
ttattagtat attttgtatg taatagtagt ttggaagttg aagaaaaatt ttttaaattt   10860
tagtaaatgt tcggttagtt cgagagagat tttagggata attttgaagg tcgttgtttg   10920
ttttaatttc gtagagatga ataagagtgt gaattttggc gcgggtgtgt ttagaattat   10980
ttacgatttt agatttatgt tgttgaaatt tattaatttt ttgtgatttt acgtggagag   11040
aagaatttgt tttgtttttt tatttgttaa atgtcgattg agaaaagggg tttaggagac   11100
gatttttttt agaaagaacg ttattttatt aattcggttg agttattaat ttgtttttcg   11160
aaaggttaat aacgtttttt ttttttagtc gtatcgcgcg gagattaatc gttttatttt   11220
aggtagtttt ttgtttaggg tttaggaatt tttgttttaa ggttttttttg gggtttagaa  11280
gttgtgttgt gtatgttttt tttaagaatt ttatttgttt gtttttaagt atatacggcg   11340
ttagaaattt agtttagttt gagttttggg atgagagaag agttaaataa agagatttta   11400
atcgtttttt tggtttttttg tttcgtcgtt ttgtagtttg ttaatttttt agttagatag  11460
tttttttaagt tttcgtgttg cgagtgaaag agaatttttt tattttattt ttttattgat  11520
cgaagtagaa aaattgaatc gaatttacgt tttttgtttt gatttttgtt agaggaaaat   11580
agaaaattat ttcgtaggtt ttttttagtt tttggatggc gagcgtagtt tttgggaggt   11640
tatatttagt tttttattgt gaatttttcg ttatttaagt tcgttcggga ttagggtttc   11700
ggatggtttc ggttgttcgt aagtacgcga aagaagaggt gaatttaatc gttggtttag   11760
aggatagtga ttagataatt cgaggattat taaataaggg gcggcggtgt ttttgtttta   11820
tggggttggc gtggggcggg gggtaggtag taagattttt taggtttttg gatgtaaaga   11880
gtgagaaaga aagcgtagtt tttggtagtt tgtttataaa tgtagttttt cggaagatga   11940
aatttgtagt tttaggttgt ttttttttat atttatgttt taattttttg ggtttttttc   12000
gaaatgaata aaattgtgga aatgaatgaa tgagaatatt attttaattg ttttaaattt   12060
attgttaagt tatttttttt gttatttgtt agaggtttaa tttgcgtttc gtgtttatat   12120
tttagaaaat tgtaaggacg tttttttttgt atttgtttgt tttgttggta gcgtgcggaa  12180
acgataattg tatagttatt g                                             12201
```

<210> 107
<211> 22692
<212> DNA
<213> Artificial Sequence

<220>
<223> chemically treated genomic DNA (Homo sapiens)

<400> 107

```
taggtaatcg gtgattaaaa atatttttag gtagtatcga gtgttaggaa aagggggttt      60
cggggaggtg agtgaatatt tacgttttta attgaaagtt tttatattta agttattttt     120
tttgtttttt ttaaattaat ttaaagggtt ggtttacggg tttttcgggg gtattagtgg     180
tttaatgtgg gtttcggggg tagtgagggt gggagagagt tagtggtttt ggggttatta     240
ggtgtgttta tggtggaaag aggttgtgga gttgggtagg tgagatagac gtgttttttt     300
gttagtttta ggtaggattt agtttgtaga ggataaatgg gtagttttgg tcgtgtgtgg     360
```

ggaggagatg tgagggcgtg aggaggttgg gtaattttgg ttatttagtg tttggatgac 420
gttgttatgt aagattcgag atttcgttgt atattattcg attttttag tatgtgaggt 480
aagttttgtt atttttattt tagagatgag gaaagttgag gtttagaatg gttaggtgat 540
ttagttaagg gcgcgtagat aatgtgaggt agagttaaga tttaagttta aatgttttgg 600
ttttcgttta tttggattat gaagttagtg tttttgagga ttttttatta ttgtttttta 660
aaataaattt ttttatcggt agtcggtagg agaaaatttt gttgttttgg gaatggaacg 720
gtatttgggg ttttagttag ttatttaagt tttggtgtga gtggtttatt ttgttttttt 780
tagggttggg aatttttgaa ggagaggagg agggagggta agggtttagg atagttgtgg 840
gttttaggtt aggtattatt gggttttggg agtttggagt atttatgaag ttaggtaggg 900
tcggttttga ttttatttat tttttatttg ggaggtttgt ggttttcgtt ttggaggagt 960
tcggggtagg tttttacggt gtttttgtta ttttggtttt ttttttgtat tgtaggtttg 1020
tgtttgttag gttcgggtta cgggtaaaat gtatgtttgt aagcgtttgg agaagaagag 1080
gattaaaaag aggaaagggg agtttatggt ttttaatgag aagtagattt tcgagaaggt 1140
taatagttag tttgtggtga gtgagtattt gggtttagtg atcggttcgt ttttttgtgg 1200
attggggttt tttttttttc ggaagggcgt ggttttttaa tgcggtcggt ttttattttt 1260
gggaagggga atgttagtgg tagcgttgag ttatagaaag gtcgtaagat atttttttat 1320
tatacggttt attgtggtcg attgtggttg gttttgtttt atttttagag tcggttagtg 1380
tttggttttt tgttgtgtat gagatcgacg tttttgtttt tttggattat tttgtaaatt 1440
ttttagtgga taaagaaagt ttatttgggt ttaggttggg agttgtgggt tttatagatt 1500
ttttaggttg gtgttatcgt ggtggggatt ttagtgttag tattttggaa tggtattatt 1560
aagtttgtgt gtattgtgtt agttagttta ggttgggttt tgttgtagta atatatttgt 1620
tttaaatttt aagtagttaa atatagtaga ggtttatttg ttatttattt gaagtttaat 1680
tagattgggt tatttttttt tatttaatag ttgttttatt tggagttttg aaagtgagtg 1740
ttaggggttt tatatagaga tttttttattg tttttattgg atagatgttt tttagtttta 1800
tttatagttt gttagttaga attagttaat atagagataa gttagtttta ggggaggttg 1860
ggaaatgtga agtgtatgga tatttggtta gttttaatat tttttgttat atattgttat 1920
aaaaaataaa gttgggtgta gtggtatatg tttgtagttt tagttattta gaaggttgag 1980
gtaggaggat tgtttgaatt taggatttcg aggttgtagt gaattatgat cgtgttattg 2040
tattttagtt tgggtgatat agtaagattt tatttttaaa attaaatata tgtatatatt 2100
aaaaataata tatatataag gtaaaaattt aaatagtata gaaggatata taataaaacg 2160
aaaagttttt ttttttgagt aattatttta ggtatgtttt atttatgtat aggttttatt 2220
atttttagtt aatgggattt ttttgtattt tttatggagg aatgtttttt ttttatattg 2280
tagatttttt tttttattta gatttttagt tatatttttt tttaaagttg ggtattattt 2340
ttttgaagag ataaagtata atttatatag ttagttttta tggatggaaa ttgtttttta 2400
tttttaaatt attatagggt cgggtacggt ggtttacgtt tgtaatttta gtattttggg 2460
aggtcgaggt agatagatta tttgagatta ggagtttaag attaatttgg ttaatatggt 2520
aaaattttat ttttattaaa aatataaaaa ttatttgggt atggtggtgg gcgtttgtaa 2580
ttttagttat ttgggaggtt gaggtatgag aattatttga atttgggagg tagagattgt 2640
agtgagttga gattatgtta ttgtatttta gtttgggtga tagagtgaga ttttgttaaa 2700
aataaaataa aataaataaa taaaattatt atagattagt ggtattttta gaggatggaa 2760
gtttgtattt tgggatttta ttttaggtta tttttagttg ttagttttgt cgggggtgta 2820
ggtgtgggta gtgttatggt atgtggagat gaggtagatg aggttaattt tagttattat 2880
tttagttgcg ggtttttttg agttcgaaat tttttttttt attgttagtc gttttagtag 2940
ggatttttgg atgttgttta tggagttttg tttttgaagg gatagtgttg gcgggttttg 3000
tttagggaag aggattatgt gatttataaa gttgggtggt acgtttttaa gggtttttag 3060
ttacgatggt tgtatttttt ttaattgtgt tttatttacg gtttcggtag ggagttttta 3120
cgatgttatt ttttggattt ttttttcggt gattatttgg gtagatgtgg gagaaagggg 3180
atagttgtag ttattgtagt agaagtatag tttttatgag tttgtttatt tttggttgtt 3240
attttatttt ttttttttaa aaatagttga tgttgatgta ttagttttac ggtatagtta 3300
gtttttagtt tggtttacga tggtcgggtt ttatgtgtgt tattttttta tgtggagaag 3360
gattattttt tggtgtaatt tgtttttttt gttttattat ggttttgttt tatagagttt 3420
tgggtgtttg gattttatta gtagaggatt agattttttt ttttggtggt tttttgttaa 3480
gtggattagt gtattagttt atttttatat tgttgattaa gatataattg agattgagta 3540
atttataaaa gaaagaggtt tgatggattt atggttttat gtggttgggg aggttttata 3600
attatggtgg aatgtgaaag gtatttttta tatggcggtg gtaagagaag aatgagaatt 3660
aagtgaaagg ggtttttttt tataaaatta ttagatttta tgagatttat ttattattat 3720
gagaatagta taggagaaat tgtttataat ttaattattt tttatcgggt ttttttttata 3780
atatatagga attatgggag tataatttaa gatgagattt gggtggggat atagtttaat 3840
tatattattt tgtttttggt ttttttttaaa ttttatgatt ttatatttta aaattaatta 3900
tgttttttta atagtttttt aaaattttaa tttattttag tattaattta aaagtttata 3960
tatagtttaa agttttattt gagataaagt aagttttttt cgtttacgag ttcgtaaaat 4020

taaaagtaag ttagttattt tttagatata atgtgggtat aggtattggg taaatatagt 4080
tgttttaaat gggtgaaatt ggttaaaata aaggggttat aggttttatg taagttagaa 4140
atttagtagg gtagttaaat tttaaagttt taaaatgatt tttttgattt tatgttttat 4200
atttagatta tgtcgatgtt aagacgtggg tttttacggt tttgggtagt tttatttttg 4260
tggttttgta gggtatcgtt ttttttttgg ttgtttttat gggttggtat tgagtgtttg 4320
taggtttttt aggtaaatag tgtaagttgt tagtggattt attattttgg ggtttggagg 4380
atagtggttt ttttttgata gttttaatag gtaatgtttt agtagggatt ttgtgtgggg 4440
tttttgattt tatatttttt ttttgtattg ttttagtaga gggtttttat gagggtttcg 4500
tttttgtagt aaatttttat ttgagtattt aggtgttttt atatattttt tgaaatttag 4560
gcggaggttt ttaaatttta atttttgatt tttgtgtatt cgtaggttta atattatatg 4620
gaagttgtta aggtttgggg tttttattat ggttcgagta ttgtattggt tttttttagt 4680
tacggatgga gtagttggga tttagggtat tgggtattaa gtttttttggt tgtatatagt 4740
acggggattt tggatttagt tcgtgaaatt attttttttt tttaggtttt tgggtttgtg 4800
atgggagggg ttgttgtgaa gatttttgat atgttttgta gatatttttt ttatggtttt 4860
ggggattaat atttagtttt ttgttattta tgtaaattta ggtagttagt ttgaattttt 4920
ttttagaaaa tgggattttt tttttttattg tattgttagg ttgtaaattt tttaaatttt 4980
tatgtttttt tttttttata aaattgaatg tttttaatag tatttatgtt attttttgag 5040
tgttttgttg tttagaaatt ttttttgtga gatattttaa attatttttt ttaagtttaa 5100
agttttaaaa atttttaggg taggggtaaa atgttagtag tttttttgtt aaaagataat 5160
aagagttatt tttgttttag tttttaataa gtttttcgtt tttatttgag attattttag 5220
tttggatttt attatttata ttatttttag tattttgggt aaagttattt aataagtttt 5280
taggaagttt taaatttttt tatatttttt tatttttttga gtttttttaaa tttttttaat 5340
ttttatttgt tatcggtttt aaagttatgt ttattttttt gggtattttt tttagtagtg 5400
ttttattttt ttggtattaa tttattttat tagttcgttt ttatattgtt gataaagata 5460
tatttgagat tgggtaattt ataaaagaaa gaggtttaat ggatttatag ttttacgtgg 5520
ttggggaggt tttataatta tggtagaagg tgaaaggtat tttttatatg gcggtagtaa 5580
gagaagaatg agaattaagt gaaatgggtt ttttttttata aaattattag gtttgtgaga 5640
tatatttatt attataagag tagtatggga gaaattgttt ttatgagtta attgtttttt 5700
attggttttt tttataatat atgggaatta tgggagtata atttaagatg agatttgggt 5760
ggggatatag agttaaatta tattaattag gtatattttg tatgtaagag aaagttttta 5820
ttaggttatg ttttattttt taaagtttta ttgttttttt agtttatttt tatttggttt 5880
tgtgagaatg gtttttatat ttaaaagggt aagtgagttt tgtttttttg attggttaag 5940
aaggtatttt ttagtttttt ttagttgatg tatatatgtg tttttgtgga ggaagtatag 6000
acgttatttt gttggattta ttgggaggtc ggggtgttag tttgttttaa ttcgttttgg 6060
ttttttttatt ttgatttatt gaaagggagg agtaggtgtg agttttttat tagttaagga 6120
ggtcgtaggt agggttatcg tagaggttat ttgggtgtag gggagtaggg tgaggttttt 6180
ttgttttttt atattttagt ttttaggatt ttgaggaggg aagtggaagt gagtggttat 6240
tgttataatt tttttatggt attgtttttg tgtttttagg ttaatttggt ttatgtttac 6300
gagattaagg atgtattgtg tttggttttg attattatga atggggggtga tttgaagttt 6360
tatatttata atatgggtaa ttttggtttc gaggaggagc gggttttgtt ttatgcggta 6420
gagatttttt gcggtttaga agatttttat cgtgagaata tcgtttatcg gtgagtggaa 6480
ggtattaagg atttttaagt ttaagttcga gggggtgggg ttagtttttt tatattttcg 6540
ggtaggtggt tgttatcgtt ttgggaatta gttttggtta gtattagggg aggatgtaga 6600
gttagataga tttttttattt taggaagttg gggttttcgg tattgaggaa gcgaggtggg 6660
ttattttttg gttagtgttt tttagttttt aaagtttagg gaagttaagt aagtgaaatg 6720
atgtttttga aattgattgt tttttttaagt taaggaggac gggtcgtgat tattgttttt 6780
tttagttgtt tttattttag tagttaggga agttgggagg gggtttggtg gtcgttattt 6840
gggattagtt ttgggaagat ttgggtcggg ttattgttta taagtagaat cggagtggtt 6900
tatggtttta ttttatgttg tttttagttt gttttggttt ttattttttt tttttattta 6960
ttttaatttt atagggattt ggatattgta gggttagggg tttagatttt atttataatg 7020
agtagtgttt tttagatttt ttttttagtt tttttgaaag aattttgaaa aatttttgtg 7080
atgttgtatt ttaagtagat atttgaaata ttttattata ttgaaatagt ggtaattttt 7140
gatatattga aattgaatag ttatattata tttttttaggg gatttagtgg aatttttagt 7200
tgttgtagtt aattgatttt tatttgtatt tatttaaaaa atgtatagaa agttttttag 7260
tgatgaggaa tttttttatta ttttttttat ttttttttatt tttttagaat gttaataata 7320
tattttttttg tttgaaagtt ttttattgat tagcgtatat ttttgaaata aatatataga 7380
tatatataag agtttttgaa gttttttttg atcgtaagtt tttaagtgtt aaaataattt 7440
ttttggtcgg ttgtggtggt ttatatttgt aattttagta ttttgggagg ttggggtagg 7500
cggattattt gaggttagtg gtttgagatt agtttggtta acgtggtgaa attttgtttt 7560
tattaaaaat ataaaaagta gtcggatgcg gtggtacgcg tttgtagttt tagatatttg 7620
ggaggttgag gttaaagaat cgttttaatt tggaggttgt agtgagttaa gattattatt 7680

```
gtattttagt ttgggtaata gagtgagatt tcgttttaaa ataaaataaa ataatttttt    7740
ttgtcgagtt gttattgtaa ttagtagtag attttatttt taattagttt tttggaagta    7800
ggggttttga tatttggagt gggtcgttat agttatattc gggaggggag gaggttgttt    7860
ttttttgta atttgggaga agagttattg agaaagggta gatgggaggg agattttaaa    7920
agatgttagg atagagtatt ttgatgtgaa gaaatttaaa ttttttaaat tgcggatagg    7980
agggaatttt ttgttcggtt ttggtagatt tcgatacgta gtttagtttg aagatttttg    8040
tagtggagtt tattttggga ttaagaaggt tttgaaaata agaaagaatg gttttgatga    8100
tatgtttttt tttttatatt attttttat ttatgttttt gttttgtttt tatttaaatg    8160
aaaagagtta aaattttttt taagggttga ataaagaatt ttttacgtga ttgtttttta    8220
tttaaaaaga aaaaagaaag ttttttaagt tttagttcgt tttttttcgg gggaaagaga    8280
attatagtgt tttttttttg gttgtgatta tttattacgg tttttttttg ggtttcgttt    8340
tggttagtgt ttattagtga gttgttatgt tagtttggtg gtagtcggtt tgttgagatg    8400
atggttgttg tttttatttg gaattgttaa tatattaaaa gaatgttatt taaataatag    8460
taataaaagt attttatgat aaataaggat atataaatat taattattat ttgatgaggt    8520
ttattggttg gatttgggta tattaattat ttcgtatgag gatagttgtt attagttaaa    8580
taaataaagt tgggtggttg agaattggag tttttattta gattttgtgt attgtttgtt    8640
ttatggttta attgagttgt atatattttt tttttggttg ttttttttatt tatagagaag    8700
attttttttt ataaaagtag tttttttgtt ggtttttttt ttattttggg aatgtagagg    8760
ttttttttatt tgtgagtaag attttgttat taagagagga gatattaggg ataggataag    8820
gtagttatat ttatgttatg tttcgtagtt gttttttttt ttaggttgta gcgtagttgg    8880
tgttgggggga tatgttagtt ttttgtaggt tttattttaa agggggtttt tttttttgtgt    8940
ttttattttt tttttttga ttttttttt ttaattgtat agttttttttt attttaggtt    9000
ttgttttgtt tttagggttt tttgagattt gtagggtttt tttagttttg gagtattttt    9060
agatttgtag ttattgatgt gtgaataggg gttatgattg gtttttttttt aatttttttta    9120
gttatttttt aggattggga aatgtttgtg gtaaacggta gtgttgtttt aaattattgg    9180
aaggagaaaa attagttgaa gattattttg atttaaatta gatagtttga gttaggaggt    9240
gggaaggaaa tatattgtgg ttatttaggt ttgattagtt cgaggggtcg atttagatgt    9300
tagtgtattt tatttgttta gaatgtttat attttgatgt ttgttgtttt ttttttttaga    9360
gatttgaaat ttgaaaatat tttgttagat gattatggta agttttttttt tattcggtag    9420
ttgaggtgag tattgtaatt ttaagaaagt aaagggtttt ttagggattt ttagagaggg    9480
ttggggaagt tgattacggg ttttattttt attgtttagt gttgggtatt tggggaaggg    9540
ggaaagttgt gagtgtgaat agagtttcgt ttggtgaata ggtagtgttt agagagattt    9600
tgaaatttag agtttttgaa atttttttttg attataaggt tttaagtgtt aaaataattt    9660
ttggtcgggc gtggttttta tagtgtattg aataaggagt ttagaatttt agagatgttg    9720
ggtaaatatt taatttttat taagtttttt atttttttatt ttgtttgtaa aatggaaata    9780
aagcgttttg ttttgttttg tttatttaga aggaagaggg tggtggagag gtttttatat    9840
gatagtttga gagggaattg tttgttagtt tagttagttg taaatgtttt aggtattacg    9900
tttgtgtatt attattatta tttttttttg gttgtggata ttgaataata agaataatta    9960
tagtattttt tatagttgtt tagttttata tttggaaaat agtttttata tttttttttt   10020
tttttttga gatagagttt tatttgttta ggttagagtg taatggcgtg atttcggttt   10080
attgtaattt ttgtttttta ggtttaagtg atttttttgt tttagttttt taagtagttg   10140
ggattatagg tgtacgttat tatatttggt tagtttttgt attttttttt ttttttgaga   10200
tgtagtttta tttttgttgt ttaggttgga gtgtagtggt gtaattttaa tttagtgtaa   10260
tttttatttt ttaagtttaa gtgattttgt tgttttagtt ttttaagtag ttgagattat   10320
aggtacgtgt tattacgttc ggttaatttt tgtatttttg gtagaaatgg ggttttgtta   10380
tgttgattag gttggtttta gatttttgat tttaagtgat ttgttttttt tggttttttt   10440
atgtgttggg attataggta tgagttatcg gtttttatat ttttgatttg attagatttt   10500
tttatttgta tagagaaggg taaggtagta tttttgtgtt tatttaatag atgataaaat   10560
tgagttttag gggagttatt ttgttaaagt tatttagtag tattagtggg gagttgaggg   10620
tagaaattgg gttttttttt attaggtttt agaatttggt agtttttagt agagggaggg   10680
gagaaatcgg gtcgttgatg atttgaagag aggtttgtat gggatattgt tttgtggagg   10740
ggcggtcgga gggtttaggt ttgtcgtttt tttttggatg tgttcggtgt tttttttggt   10800
ttaagaatta agtttacgtt gttttttttgt tattttgatt gttagttttt atcgagttag   10860

ggagtttata cggttttagt ttggcggggt ttcgatattg gtttttgttg atatttaggt   10920
tggagggatg ggggtggggt tttggtgttt ataggtaggg aagggaaggg gacgtgtttt   10980
tttttgtggg gtaagttgtg gtcgcgattt tgtttaggtg ggttgtggga gggataaaga   11040
ttaaagtgga gttttagttt agcgtttttc gttttggggg tttagttttg ggttgtgagt   11100
tgggatttat ttttaggttt tgttaggtta gattagatgt tttttttttt cgttttttta   11160
aagtgaaaaa taattaattt ttagagatta ataaggtagg tcggggagtt gtttttttaaa   11220
tcgttttata attttttttt tttaagtaaa ttgttgtata atgttagaag aattttatat   11280
```

```
tttttttatg aagtaatttt agaattaagt taagattttt tgattttagc gttttaggaa   11340
gtttatttag tttttgatta ggtaggtcgg ttaacgttta gtcgggttta ggtattttat   11400
tgtattttta tgttttattt ttgaagagtg ggtatttttt tgtgtttttt tatatttgta   11460
tttttttaag gtttttgtgg ttattttgag atggtagatt ggggtttttg ttgtttttgg   11520
atagatgaga atgtcgagag ttcgtatgtt ttgtttaagg gtatatagta aggtcgggtg   11580
tttatgcggt tgttttaggg atttattgat tttgttgttt tttttaggtt atattaggat   11640
tttagatttg ggtttggttg tgaagatttt cgagggagat ttgattcgcg gtcgggtggg   11700
tattgttggt tatatgggtg agtgttgggt tgtttgtgtt aatgtatttt gagatttatt   11760
atttgtttat cgtcggtcga gtttttagag tttgttcgta gagtttttcg tttttttatg   11820
tattgttgat tatatataga gttatagttt ttgtagggtt gttgggggc ggttttagtt   11880
agggggaggg ggagtatagg ggttttagat gtgatgattg ttttgagttt tttgtatttt   11940
taggttttta gttttatttg tgtttaaagt taagttattt ttaggggtcg ggtttggta   12000
tatagcgttg ttaggggagg ttatattgaa gtaggggttt gggaggagag gtttttgaga   12060
ggaaggaggt aatttataag agttcgtaaa tgagaggcgc gtatagttgt tgtgttatta   12120
agtatgattt tagtagagag tagttttggg tttttgttat ttttttttag tttgttttga   12180
gattagcgtt ttggggggttg tagggaagag atttagagtt aagttttgtg ttttagaatt   12240
tttcgtttaa ttggggggtac gttttgggtt tttttttgtg tattttatat atttattttt   12300
ttttttttga gaggcgcgtg ttcggggagt aggtacgttg tcgtttttta gttaggttgg   12360
tattgatagt agttgtatag gaggtgtgtt ttgtttttgg tgttagaggg ttttttttag   12420
attgtatgat atttggtaat ttgtggagtg tgtgtttgtg tgtatgtgag tgtgtgtgtg   12480
tttatatgaa tgttgatcga tgattgatag gtaggcgggt gggcgggtgg atggatggtt   12540
ggatggatga tggatgatgg atgggtggat gatagatttg tttatttgtg tgtgtatttg   12600
tttgggtttt tttttgtaga gtgttttatg tatttgtttg tttcggtttt tgtgtgtaaa   12660
ttagtatatg tgtggatgta ttttttttta gatatacgtg ttgatgttgt agaattattt   12720
tttttgtatt tttatggtaa atttgtaatt cggtttatta aagtggtata gaagatatag   12780
taagagattg tttgaattat agataaattt aagattattt ttaatttggg tagatatatt   12840
tttatttatt gaaataaata tattgatagt atttgttttg tgttaggtgt tatattgtta   12900
cggtagtgaa taaagtagtt tatgttttag gcgaggggat attaagagtg taggatattt   12960
ttatttacgg tggttagagt tttaaagtta tagttaaagt ttttcggggg tatagaggag   13020
agttattagg gtaagttttt tggagggggt ggtatttata gtagttggtg gggtaagagt   13080
tggtggcggt tatgaaattt taggtttttt gtgagtagcg ttattagttg tttttttttt   13140
ttttattttt tttttttttt ttttgttttt agttttagag gttttgaata attagaggta   13200
cggtttgagt ttcgattatt ggggttttgg ttgttttatt tatgagatga tcgagggtta   13260
gtcgtcgttt cgcggtcgta aggagaaggt gaagcgggag gaggtggatc gtcgggtttt   13320
ggagacggag gaggtgtatt tttataagtt tttcgaggag gttaagttta tttgtaagat   13380
ggtgagtttt tggtggttag atgttatttt taagttggtg gttttttttt ttgggtttgg   13440
ttttagtttg tttttagaat agtaaatagg ttgaagggat aggggtttga gtatgggggt   13500
gggggttgta gtttattaag ttagagttga gggtattttt tgttgtggat tggggtggtt   13560
agcggagatt tttaggagaa agtttgggtg gggtagggat atttaggaat taagggaaga   13620
ggggtgaggg gaatagtgtg gaatattttt atatttagtt tgtggggggg tttatatagg   13680
gcggagaaga gatggtttga gttggtgttt tagatataaa gtaattttgg gttaagtttc   13740
ggtttttgtt cgtgggtttt ttgttttttt tatttttgga ttttaaggtg ttggtttaag   13800
acgaagggtt ataggggggtg ggggcgacga ttttagtttt tgaatatttt ataataaagt   13860
tttttattat tagatggatt gttggttacg ttagtttggt ttttgtatga tagggtttag   13920
ggatagtgag gttagattta tttaaaagtt gggtgggtgg ggttagatgg ggggattgga   13980
agttagataa ggagtgatag gagttattgg aagtgggggga tatgggagtg gagttcgttc   14040
gtgtattttt gttattgggt cgttttatgt cggtttttag gttttttttt gggaattagg   14100
attagtggtt ggttttgtgg ggagatggtt atttttttgtt gttgtttggt ttttagtttt   14160
taagtttagt ttatgttttt ttggggtttt tttttttatg gtttttagta tagttgtgtt   14220
ggtattttta ttgtaagtag ttgtttttta atgttagttt tatagggata ggaggggtcg   14280
gagtatttag ttttataatt gttagtttat agattttttt tttttttttt ggagttatag   14340
atgtatttcg tattttatgt attcggagtt atagatgggt aaatcgagtt tgggaggggg   14400
gagttatatt ttttattttt ttatatttgt aggtattttt ggggtagatt tagcgtgtgt   14460
attgataatt taggtaattt tcggagttta ggaagtagta gttttgggag ttgattaaaa   14520
gtcgtaatgt ttttgtttag gaagatttga gatttgcgtc gtaagcggtt ttaggagggt   14580
ttggttcggt tttttatttt agaggttttt tttttgtagt tagatatttt tgtttggttt   14640
ttttttgtta ttaattttat ttttatatta tagagttgtg ggaagaagtt ttgtgtgtat   14700
agtagattta tattttaatt ttatagttgt tggttcgtta tgtagttggg gttttagaga   14760
aagttatatg gttatcgtag ttagggttag ggtgggggga tcggaggagt agtgtagtat   14820
tggtcgggta aatggaaatg tgggtgtgag ttatatttgg ttgttttatt gggagatatt   14880
tgttaggtgc gataaaacga gataagggaa agaagaaggg gttagatagt cgtgggtaga   14940
```

gtattattaa atgttttagg ttagggttag gtaattagag aagtaggttt tgagagattt 15000
tagtagggat atgggacgta ttaagaggat gacgaggcgg agagggtttt agttttattt 15060
ttagatttta gatatttatt ttggtttttg gttttggagt tgggattttt tgttgtagtt 15120
tggattaggg tgttgttgtt tttttgttgt tttttggtgg atattgagag aaagtttgtt 15180
gttttcggag gatttgtttc gtgggtataa aattattgtt ttttggaagt ttttggtttt 15240
gttgcggttt tttttataaa tgatttttgg ttttgggagt ttagttttag gtttttttta 15300
cgtttttgat tgtagtattt tcggtattta ggtttatttt gtttttttta gttggtggta 15360
gtttaggata ttgtagagtt tgatttgtta ggagatttcg taggttttgg gtttataggg 15420
ttatttttag gttttggtgg ttttagtttt gtttgtttaa gagggttttt ttataattag 15480
tgaggaagtt agtttttggt ttttaggttt gtttttttatt ttattttagt agtcgtagaa 15540
tttatttttt ttttttggga gtttagaaat ttatatattt tattattttg ttttttgagt 15600
ttagtttatg ggtttagatt ttagttttta ttttttttaa atttttgttt agttttagta 15660
ttgggtttta gttttttttt agtttttttt tgtttttttag tcgttttggt tttatttgtt 15720
tttttagttt atttgtattt ttttttagg aagttcgttt tgtttgtttt atttatttta 15780
gtttatattt tttttttttt ttattttttt tggtgtttat ttgatagtat gattgtaaat 15840
tttttatttg acgatgtggt tgtaaattgt tgattggatg ttagggtttt aaatggtagt 15900
atataggcgg gtgtttaatt tgtatacggt tttttaaaag aagtaaatgg taggtttatt 15960
gttaagattt agaaatgagg agattttatg taataatttg gattttttgg ttttttttga 16020
aaagtagaag ttttaggttt tggttgtcgt tttatttggt aggagttgat atatagttcg 16080
gttcggttta tttagtttat ttttcgtttt ttttattttc gtttgtgtta tttttttggt 16140
tagttgttgt agtttttgta gaaggagatt tgtttttgag ggattgttta gaatagtgta 16200
gtatttgggt ttagtttaat ttagtgtttt gagtttttgt tgaatagtga agtttttaga 16260
gagagaagta gaggtatgag tgttagattt tgagtcgtta gtttggtggt tcgtgggggc 16320
gttaaggggt aatattgagc gattgagtgt tttgttttgt ttttttaagg gtggaatttg 16380
gtgttggttt tgatgttatt ttcgattttt ttgtttgggg ggtagttttt tttagatttt 16440
gagttttaag aggttagttt ggttgtttat ttttttcggc gtttttagtc ggtgtagtgt 16500
ttgttttata aggattagta ggaagtgtat atgggatgaa tatgagtaaa gcgtataggg 16560
tattgggaaa agataaggtt gggtttttat tttaagtagt ttgttttttt ggatttttagt 16620
tttaaagtag atatttagta gatattaatt gcgtatagtt gtagcgttta gtaaggtaga 16680
ggagaagaga gaattacgag gttggggtat tgggagtgta ggttaggggt cggggttttg 16740
tggtcgtgtg gttggttatg gttagttaag gtttttgcg taggtttagt tttttggttt 16800
tatttatagt tttatttcgt tttatttggg tgtttgtttt taaagatttt ttttttttta 16860
gtaatttttt tgtttcgtgg ttttgatgaa gatatttttt tattttaatt atttttggtt 16920
ttttttattt ttagatgaaa atttcgttat ttgatttggt ttttaggatt ttttattatt 16980
ttttttgat tagttttttt tttttgattt atttttgtat ttttttttcg ttttttaagt 17040
taaacgtgtt ttgggtgttt tttagattta ttttgtacgt tttgttattt tgttttgttg 17100
gtgtagtttt ttttatttgt atattttttt ttttatttat attttgaaag tttatttgtt 17160
ttgtaagatt taatttaaat attttttttt cgtgtagttt tttgattttt agttggtagt 17220
tagttatgtt ttttgttttc ggggtgttcg atttgtatta ttttgatgcg ttttttgagt 17280
atgattagtt ttttatgagt ttgagtattt ttagaggtta agtgtttcgg tggtgttttt 17340
tttgttagga aggggtttgt atttaggagg tataattttg taaattagtt gagtatgtgt 17400
acgagataga gttattgatg tagtttttgt tttttgttgt ttattttata tagaagaggt 17460
gatagtgatt cgatatttat taatgagtgt ttattataat ttaggaacga ggtttgtaat 17520
tttgagagaa ataagatttt tgtttttttg ttttattagg tatttagtta ggttgtgagt 17580
gtgtgagtat agatgtacgt taagatttag aggtagtttt gaagtaagat ttttgtaggg 17640
tggggtagcg tgttttgggt ttttaggtat ggtgtagata ttgtggaggt aggaggttcg 17700
gataggagga gaggtttagg ggacgtggtt ttatggggtt ttttgtttta gttgtttacg 17760
aaagatgcga agtagaggtt gggttgttag gaggaggggg ttgtagaggt taagagatat 17820
tttttttttta ggaatatgaa ttttaagcgt ttagaagtcg ggatgttgga tttttttttc 17880
gttttagacg tgagtagttt tttttagttt ttagtagttt tttttatagg gtatttaggg 17940
ttgttttcga gtgttgtttt atagtgagtt ggtgtagagt gtggggtata tcgtgttttt 18000
gtggggatta gggttttttt tcggttattt tttatttaag ttgtagatga attttacgtt 18060
aggtagtgta gaagaggcgt gggttaggga gtttatattg gttgtttaga gatttgggta 18120
tttttttagg tttgggggtt ttggatagat tttttttaag cgttagtttt ttttttgttt 18180
agtgaaatta gtaatattta tgttttttat ttgtttggga gatttgaggg tgaaatgaga 18240
attgaatggg gaatgttttt aagagtattg acggttatta tatttagttt attttgatta 18300
gtatgtatgt atcgagtatt attgttgacg ttaggttttg ggttaggtag aaatataaga 18360
ggtttttttt ttagtagttt atattgtagg gagttgtatt tttttaggag gtttttaggt 18420
tttttagtcg gtgtgtgagg taagatttta gttgaggtag aattgttttt gaaagttttt 18480
tatatggttt ataaattatg agtttttagt tggattagag gtttaagaga tgtagattag 18540
gtagaggttg ggtgatttgt gcgggatacg tgttgtgatt tcggggtttg ttttgtttaa 18600

agttagggag ttagaagtta ggaaggtttg ttgtgtttcg gtttttgtta cgttttcgcg 18660
gatgattttt tgttggtagg gggattgggt cggggattat ggaggtcgtg ggagtagtag 18720
tgtgttgtag gcgaatgatt gtatttttag agagtttatg tgtatttttt tttttaattt 18780
cgtgtttaat ggttttgcgt cggtagttta tagtagattt ggtggtagta tttgtatagt 18840
agtaatttgt aaatgttata aattagggtt tttgcgtttt ttttttttag aattagtggt 18900
tattatttat tagtatcgtt ttgagtggag gtaggtgggg atcgtttttg gaattttggt 18960
atttttata tatggggatt ttttggtttt ttgggataga aggtggtttg ggaagttgtt 19020
aggtgcggat tagtgtttat ggtaattata ttatagattt cgtttggtta gggatggtat 19080
tagtgtgatg ggtaattagg gaatgttttt tggagaaata cgggggttat tgagaatata 19140
aagagtaggt cgggtacggt ggtttatgtt tataatttta gtatttggga aggttgaggt 19200
agtcgggtta ggagttcgag attagtttga ttaacgtggt gaaattttat ttttattaaa 19260
atataaaatt aattaggttt gggggtatat atttataatt ttagttattt gggaggttga 19320
ggtaggagaa ttatttgaat ttaggaggta gaggttgtag tgagttgaga ttgtattatt 19380
gtattttagt ttgggcgacg gagcgagatt gtattttaaa aaataaaaag tgaaagttat 19440
tgaatgttgt aaatttaatt tgtagatgag tttgagtagg taggggggagg atggtaatta 19500
tagtattgtt ttcgattttg gttgtttggc gaatcgtttg gggagtatga ataatattcg 19560
ttttggatgt agtttttagg gagtttgggg taagattcgg gttttaggag ttttgaaatt 19620
tttttagttg attttagcgt agagttaggg ttgagagtta ttattatggg aagtgttata 19680
tgtaaggagt gtgttgtttg ttgtttgttt ttagtgttaa gggttttggg agtggttatt 19740
ttttggtgtt ttaggaaatt atatagtgtt gagtgtatga atattacggt tgtgttattg 19800
ggttgatgtt cgaaattgga gaggatgtgg gtatttagat tgggggtgtg gtaaaggttt 19860
ggaattcggg tggttatttt tgtggttttt gggttattta ttgttatggt gacgggttga 19920
gattagttat cgtattggag ttattttttt atttttagt atattttagg tttcgtgtta 19980
ggttttaagg gagattttat ggtatataaa atagttggtt gtttttagtt tgaagaggag 20040
taggggtgtt agtaaaattt tttaaataga agtagaaata cgagttataa gagaagagaa 20100
taggtagttt tcgggaagat gggagagtag cggggttttc gtgcgtgtat ttaggtatgg 20160
ttggagaaaa gcgttttagg ttttggaagt agtatgtgga aggaggcggt tttgagggtt 20220
agagcgatta attgttttgt tttgtttggg attgttttgt ttttagtatt gaaagttttg 20280
tgttttgggg agatagggat agttggttat tttgggagtg attttggtta tttcgaatta 20340
gggtattagg aggatgaggt agtagagttt cgtaaagcgt atagtttgat aaggtgtata 20400
gagtagttta tcggtgtttt tttgttttag tcgtgtattt tgtggtttag ggtttttgtt 20460
tttggtattc gaggttttag gttagtaaag gttgtaggtt ttggagtgcg aagtcgttgt 20520
gagttgggga ttttgttttt tttttattgg tttttttttag gttgtacgtt aatttttagt 20580
tatttttgtt agtttttttat agttatttcg tttttatag tttttttttgt tagtttttta 20640
tagtttttttt tgttagtttt tttagtcgtt tttgttagtt ttttatagtt atttttgtta 20700
gtttttttatt atcgttttttg ttagtttttt atcgtcgttt ttgttagttt tttatagtta 20760
tttatgttag tttttttatag ttattttatt tttatattat tgtcggggtt gttgttatat 20820
ttttaaatta cgagtattgt gagtgatatt atttttttta tttttattga ttttttttt 20880
aaaataaaaa ttaatgtatt ttaaaaggaa atttttttaa ggaaataata gttatgaaag 20940
aggggtttgg gtgtgttagg taattttttg ttttattata gatttttaaa atacgtttat 21000
tttttaatat ttttttgtaa gttatttagg gttagtttcg tgagtttgat ttggtatttt 21060
ttagggtatt ttgggttttt atatttggtt tagtgttttt tgttgttatt ttaaaatttt 21120
aaatgtggaa tatggatttt gcgtttttat tttgcgttga gtatgtgaat gatgtggttt 21180
tgtttttagt taaaagttcg ttgagtttta cggtggcgtg tatttcgggt tttgggaatt 21240
agagaagggt cgtagttttg taattggata tttttgttcg tatttttgtt ttcgtttacg 21300
tgtatttggg tttatggttt gttttgttgg ggatagtatg gttttgagtt ttgagttgga 21360
gattagagta gattggaggg gagagtttta gaaagtgtcg agttttgaga ttattgttag 21420
aattttttttg gtatggcgga gtttatgttg agtcggtgta ttgatttata attttcgtat 21480
tagttaaatg ttaggagatt ataagtagga ggtagttttt attttttagt gatagatgtg 21540
ttattaggtt tggggatttt tttatatttt atttggggtt gtttttataa aggttttatt 21600
tttagaggtt ttgggaagtt tttttatatt aattttgaat ttttagaggg tagggtttat 21660
ttaggtttat ttttgttttg gtatagagta ggtttttagt gtatagttga gggacgagta 21720
aggtaaaaga tatgtttatt ataataatat tagtttttag ttacgggcgt taggttcggt 21780
ggttagtatt gatagtagtt ttatcgttta ggtgtttcgt tgtttttatt ttgtcggtaa 21840
ggaaatagga tataggtttt aggggatatg agattttttt aaagttttta agttttatag 21900
ttgggatttt agtttacgtt tgtttagtat gggtaaggga gtgatggtag gaatgagttt 21960
tgggttgggt atttaggtgt ttcgagtttt tgtgttattt tagttagggt tttcgttatg 22020
gatttgagag agggttgtat tgttatttgg aggggtcgta taaaaaaatt ataggttatt 22080
atttgaggtg gataggaagt ttagttaagt tattggggtc gatttttttt ttggatttat 22140
ttgtatttga gttatatatt ttttagttat tatttatagt agttttgggg gtgtgttttg 22200
ggaagatttt tttttgtttt ttaaaatttt aaggtttggt tcggggttat tggagtcgta 22260

ggcgggatat atgtgtgatc ggttttttgt ttttggtagt ttcgcgttgt gtattgtaag 22320
gacgtgttgg atatcgagta gttttttatt gtgaagggcg ttaatttgga ttatatagac 22380
gacgattttt attttaagtt ttttacgggt tttgtgttta ttttatggta aaacgaggtg 22440
agtagggtag attatttgtt ttggtttggg tgggagggag ggattgacgg gtggaaggag 22500
gcgtcgggaa tatgagtttg gcggtaggag gttgagcgta tggtttttgt tttttttatg 22560
aaggtagtat ataaaagttg ttagtggtta agtagggagt tgtagttatt acgggttagt 22620
tattgacggg gagtttcgtt tagtttttga gatttggagg gcgtgtggtt taggggtagg 22680
tgaggttagg gg 22692

<210> 108
<211> 22692
<212> DNA
<213> Artificial Sequence

<220>
<223> chemically treated genomic DNA (Homo sapiens)

<400> 108

tttttggttt tatttgtttt tgagttatac gtttttttagg ttttaggggt tgggcgaggt 60
ttttcgttaa tgattggttc gtggtggtta tagtttttta tttggttatt gatagttttt 120
gtgtgttgtt tttatggaga aaatagaaat tatgcgttta gtttttttgtc gttaaattta 180
tattttcgac gttttttttt attcgttagt tttttttttt tatttagatt aaagtaagtg 240
gtttgttttg tttatttcgt tttgttatgg gatggatata gagttcgtgg agaatttgga 300
gtagaagtcg tcgtttgtgt ggtttagatt gacgtttttt atagtggaga attgttcgat 360
gtttagtacg tttttatagt atatagcgcg gggttgttag gggtagaggg tcggttatat 420
atatgtttcg tttgcggttt tagtggtttc gagttaggtt ttggggtttt ggggagtaga 480
agggagtttt tttaggatat atttttagaa ttgttatggg tagtggttgg agggtgtgtg 540
gtttagatgt aggtgagttt aggggagggg tcggttttag tggtttggtt gggtttttttg 600
tttattttag atgatggttt gtggtttttt tgtgcgattt ttttagatga tagtgtagtt 660
tttttttaga tttatgacgg ggattttggt tggggtgata taggagttcg gggtatttgg 720
gtgtttagtt tagggtttat ttttgttatt atttttttat ttatattgga tagacgtggg 780
ttagagtttt agttgtgggg tttaggaatt ttggggaagt tttatatttt ttgaggttta 840
tgttttgttt ttttatcggt aaaatgggga taacgaggta tttgggcgat ggggttgttg 900
ttagtgttgg ttatcgagtt tggcgttcgt aattgggagt tggtattatt atggtaggta 960
tattttttgt tttattcgtt ttttagttgt gtattggggg tttgttttat gttagggtag 1020
agatgaattt gggtggattt tgttttttaa gagtttaagg ttggtgtggg gaggtttttt 1080
agagttttttg gagatgaggt ttttatagag atagttttag gtggggtgtg aagggatttt 1140
tagatttggt gatatatttg ttattggagg gtagaggtta ttttttgttt atagtttttt 1200
gatatttggt taatacgaag gttgtgaatt agtgtatcgg tttagtatgg gtttcgttat 1260
gttaggaagg ttttagtaat ggttttaggg ttcggtattt tttgggattt tttttttag 1320
tttgttttgg tttttagttt aaggtttaag gttatgttgt ttttagtaaa gtaaattatg 1380
ggtttaagtg tacgtggacg gaagtagggg tgcgggtagg ggtgtttagt tgtagagttg 1440
cgattttttt ttagttttta aagttcgggg tgtacgttat cgtggaattt agcggatttt 1500
tagttggaag taagattata ttatttatat gtttagcgta gaatgaaaac gtagggttta 1560
tgttttatat ttagaatttt aagatggtaa tagagggtat tgagttaggt gtggggattt 1620
agggtgtttt agggggtgtt aggttaagtt tacgaagttg gttttgggtg gtttgtagag 1680
gaatgttaag aaatgagcgt attttaaaaa tttgtaatgg aatagaaagt tatttggtat 1740
atttaaattt tttttttatg gttattattt ttttggaaag gttttttttt aaaatatatt 1800
gatttttatt ttaaaaaaag agttaatgga agtgaaaaag atgatgttat ttatagtgtt 1860
cgtggtttag gaatgtggta gtagtttcga tagtggtgtg gggatgggat gattgtgggg 1920
ggttggtatg gatgattgtg gggggttggt agggacgacg gtggggggtt ggtaggacg 1980
atggtggggg gttggtaggg atgattgtgg ggggttggta gggacgattg ggggggttgg 2040
tagggaagat tgtggggggt tggtagggaa gattgtgggg ggcgggatga ttgtgggggg 2100
ttggtaggga tgattggggg ttggcgtgta gtttggggag ggttagtggg gaaaaggtaa 2160
agtttttagt ttatagcggt ttcgtatttt agggtttgta atttttgttg atttgaggtt 2220
tcggatgtta ggaatagagg ttttgggtta tagggtatac ggttgaggta ggagggtatc 2280
ggtgggttgt tttgtgtatt ttgttaggtt gtgcgttttg cggggttttg ttattttatt 2340
tttttaatgt tttgattcga agtggttagg gttattttta gggtgattaa ttgtttttgt 2400
ttttttagga tataggattt ttagtgttaa aagtaggata gttttaggta aagtaggata 2460
gttggtcgtt ttgattttta gagtcgtttt tttttatatg ttgtttttag ggtttggagc 2520

gtttttttt agttatgttt gggtgtacgt acggaggttt cgttattttt ttattttttc 2580
ggaggttgtt tgtttttttt ttttgtgatt cgtattttta tttttattta aggggttttg 2640
ttgatatttt tgtttttttt tagattggaa ataattaatt gttttgtgtg ttatgggatt 2700
tttttttgggg tttggtacgg gatttggagt gtgttggaag gtagaggaat agttttaatg 2760
cgatgattaa ttttagttcg ttattatagt aataggtagt ttagaggtta taaggatggt 2820
tattcgggtt ttaaattttt gttatatttt tagtttggat gtttatattt tttttaattt 2880
cggatattag tttaatggta taatcgtgat gtttatgtat ttaatattat gtggtttttt 2940
ggaatattaa ggagtggtta tttttaaagt ttttggtatt aaaaataaat aataaataat 3000
atattttttg tatgtggtat tttttataat agtgattttt aattttggtt ttacgttaga 3060
attagttggg gaggttttaa aatttttgaa gttcgagttt tattttagat tttttgggga 3120
ttgtatttaa ggcgagtgtt gtttatgttt tttaggcggt tcgttaggta gttagggtcg 3180
agagtaatgt tgtgattatt attttttttt tgtttgttta agtttatttg tagattgagt 3240
ttgtagtatt tagtgatttt tatttttttat tttttgagat gtagtttcgt ttcgtcgttt 3300
aggttggagt gtagtggtat aattttagtt tattgtaatt tttgtttttt gggtttaagt 3360
gatttttttg ttttagtttt ttaggtagtt gggattatag gtgtgtgttt ttaagtttgg 3420
ttaattttgt attttagtag agatggggtt ttattacgtt ggttaggttg gtttcgaatt 3480
tttaattcga ttgttttagt ttttttaagt gttgggatta taggtatgag ttatcgtgtt 3540
cggtttattt tttatatttt tagtggtttt cgtgtttttt taaaaagtat tttttgatta 3600
tttattatat tgatgttatt tttggttaga cggggtttgt ggtgtggttg ttatggatat 3660
tgattcgtat ttagtagttt tttaagttat tttttgtttt agaggattag ggaattttta 3720
tgtatagggg gtgttagagt tttaaggacg gtttttattt gtttttattt agggcggtat 3780
tggtaagtga taattattgg ttttgggaga gggggacgta gaggttttga tttgtagtat 3840
ttgtaaattg ttgttgtgta gatgttatta ttagatttat tgtaagttat cgacgtaaag 3900
ttattggata cggaattggg aagagaggtg tatatgagtt ttttggagat gtagttattc 3960
gtttgtagta tattgttgtt tttacggttt ttatagtttt cggtttagtt tttttgttag 4020
taaggggtta ttcgcgggag cgtggtagga gtcgaagtat agtaggtttt tttggttttt 4080
gatttttttga ttttaaatag agtaggtttc ggaattatag tacgtatttc gtatagatta 4140
tttagttttt gtttggtttg tattttttgg gtttttgatt tagttgagag tttatgattt 4200
atagattatg taggggattt ttaaggataa ttttatttta gttggaattt tgttttatat 4260
atcgattggg ggatttgggg atttttttggg aagatgtaat tttttgtaat gtgagttgtt 4320
gggggagggg ttttttatgt ttttgtttgg tttagaattt ggcgttaata gtagtgttcg 4380
atgtatgtat gttggttaag atgaattgaa tgtagtggtc gttaatgttt ttgaaagtat 4440
tttttattta gtttttattt tatttttaaa ttttttaggt aggtgggagg tatgagtgtt 4500
attggtttta ttggataggg aggaaattga cgtttagaag gaatttgttt aggattttta 4560
gatttgggaa agtgtttaag tttttggata gttaatgtgg gttttttggt ttacgttttt 4620
tttgtattgt ttggcgtgaa gtttatttgt agtttaggtg aggagtggtc gggaggggat 4680
tttggttttt ataaagatac gatgtgtttt atattttgta ttagtttatt gtgaggtagt 4740
attcgggggt agttttgggt attttgtgaa gggagttgtt gggggttggg ggaggttatt 4800

tacgtttgga acgaagggag ggtttaatat ttcggttttt aagcgtttga agtttatgtt 4860
tttgaagaag ggtgtttttt tgatttttgt agtttttttt ttttggtagt ttagtttttg 4920
tttcgtattt ttcgtgagta gttgaaatag agagttttat gaagttacgt tttttgggtt 4980
tttttttttg ttcggatttt ttgtttttat agtgtttgta ttatgtttag gaatttaaga 5040
tacgttgttt tattttgtaa aggttttgtt ttagggttgt ttttgagttt tggcgtgtat 5100
ttgtgtttat atatttatag tttggttaga tgtttgatgg gataggagag taggaatttt 5160
gtttttttta ggattataga tttcgttttt gggttatggt gggtatttat taatgggtgt 5220
cgagttattg ttattttttt tgtgtagggt aagtaataaa aggtaaggat tgtattaata 5280
gttttgtttc gtgtatatat ttagttaatt tgtagagttg tattttttgg gtgtaagttt 5340
ttttttgata gagaaggtat tatcgaggta tttggttttt aggagtgttt agatttatgg 5400
ggggttaatt atgtttagag gacgtattaa ggtgatatag atcgggtatt tcggggggtag 5460
ggggtatgat tggttgttag ttggaggtta gggagttgta cggggaagag gtatttaagt 5520
tgagttttgt aggataaata ggtttttaag gtgtgggtag ggggaggggt atataggtag 5580
agggaattgt attagtaaag tagagtggta ggacgtgtag ggtgagtttg gggaatattt 5640
aaaatacgtt tgatttagaa agcgggagga gggtgtagag atgggttaag gggagggggt 5700
tggttaggga gagatgatag aggattttga aggttaggtt aggtggcggg atttttattt 5760
ggaggtagaa ggagttaggg atggttgagg taggagagtg tttttattag aattacgagg 5820
taggaaggtt attaggagag gaagagtttt tgggagtaga tatttaggtg agacgggatg 5880
aggttatgaa tgaggttaga gggttagatt tgcgtagggg gttttgatta gtttatggtta 5940
gttatacgat tatagggttt cggtttttga tttgtatttt tagtgtttta gtttcgtgat 6000
tttttttttt tttttgtttt attgagcgtt gtagttgtac gtagttggtg tttattaagt 6060
gtttgttttg aggttaaaat ttagaggagt agattgtttg gggtaggggt ttagttttgt 6120

ttttttttaa tgttttgtgc gttttgttta tatttatttt atgtatattt tttgttggtt 6180
tttgtggggt aggtattgta tcggttgggg acgtcgggga gagtgagtag ttagattggt 6240
tttttggggt ttagggtttg gagggggttg ttttttaaat aaaggaatcg aaggtggtat 6300
tagggttagt attaggtttt atttttgaaa ggataaagta gagtatttag tcgtttagtg 6360
ttgtttttta acgtttttac ggattattag gttggcggtt tagggtttgg tatttatgtt 6420
tttgtttttt tttttggggg ttttattgtt taataaaggt ttaaggtatt gggttgggtt 6480
gggtttaggt gttgtattgt tttaagtagt tttttagagg taagtttttt tttgtagggg 6540
ttgtagtagt tggttaggaa ggtggtataa acgaggatga gaaaggcgag gggtgggttg 6600
ggtgggtcgg gtcgggttat gtgttagttt ttgttaggtg ggacggtagt tagggtttgg 6660
ggtttttgtt ttttaagaga agttagaaaa tttaggttat tatatgaaat ttttttattt 6720
ttaaattttg gtaatgaatt tattatttat tttttttgaa aaatcgtgtg taggttgaat 6780
attcgtttat atgttgttat ttgaaatttt ggtatttagt taatagtttg tagttatatc 6840
gttaggtaaa gagtttgtaa ttatgttatt aggtggatat taggagaagt gagaggaaga 6900
gaaggtgtga gttgggatgg gtggggtagg tagagcgggt tttttggagg aggggtatag 6960
atgggttgga aggataagtg agattaagac ggttgaaagg taagagggag ttggaggggg 7020
gttaaggttt agtgttggga ttgagtaggg gtttggggaa gatggaggtt gaggtttggg 7080
tttatggatt gggtttagga gataaagtgg tgagatgtgt gaatttttgg gttttagagg 7140
agggggagtg ggttttgcgg ttgttaaggt agaatggggg atagatttgg gggttagaga 7200
ttggtttttt tattgattgt ggagggattt ttttaggtag gtagggttgg agttattaga 7260
gtttagaaat agttttgtga atttaggatt tgcgaggttt tttggtaagt taggttttgt 7320
agtgttttga gttgttatta gttggagggg ataaggtagg tttagatgtc ggagatgttg 7380
tagttaaggg cgtgaaggag gtttaagatt agatttttaa gattaaagat tatttgtaag 7440
ggaagtcgta gtaaggttag aggtttttag aggatagtgg ttttgtattt acgggatagg 7500
tttttcggga gtagtaggtt ttttttagt gtttattagg gggtagtaga gggatagtag 7560
tattttggtt taggttgtag taggagattt tagttttaaa gttaggggtt aaggtgggtg 7620
tttggggttt gggggtggga ttggggtttt tttcgtttcg ttattttttt agtacgtttt 7680
atatttttgt tgagattttt taagatttgt ttttttgatt gtttggtttt gatttgggat 7740
atttggtgat gttttgttta cggttgtttg attttttttt ttttttttttg tttcgttttg 7800
tcgtatttga tagatatttt ttaatgagat agttaagtat ggtttatatt tatattttta 7860
tttattcgat tagtgttgta ttgtttttttc ggttttttta ttttagtttt ggttgcgatg 7920
attatgtggt ttttttgag gttttagttg tatggcgggt taatagttgt ggggttagag 7980
tgtgggtttg ttgtatatat aggatttttt tttatagttt tgtgatgtga ggatgaggtt 8040
ggtgatagga aggggttaag tagaggtgtt tggttgtagg agaggggttt ttggggtgag 8100
gagtcgagtt agattttttt ggggtcgttt gcggcgtaga ttttaggttt ttttgagtag 8160
aagtattgcg atttttaatt aatttttagg attgttgttt tttaaatttc gggagttgtt 8220
tgggttgtta atgtatacgt tgaatttgtt ttaaaagtgt ttgtagatgt agaggggtgg 8280
gaagtgtggt tttttttttt taggttcggt ttgtttattt gtggtttcgg gtatatggga 8340
tacggggtat atttgtggtt ttagggagag gagaggaagt ttgtgggttg gtaattgtgg 8400
agttgggtgt ttcggttttt tttgttttta tgaggttggt attagagggt agttgtttgt 8460
agtgggggtg ttagtatagt tgtgttgggg attataagga ggagggtttt aaggggggtat 8520
gggttggatt tgggggttga gagttaggta gtagtaggaa gtggttattt ttttataaag 8580
ttagttatta gttttggttt ttagggaagg gtttgagggt cggtatgggg cggtttagtg 8640
gtaggggtgt acgggcggat tttattttta tattttttat ttttaatggt ttttgttatt 8700
ttttatttgg tttttagttt ttttatttgg ttttatttat ttagtttttg ggtgggtttg 8760
attttattat ttttagattt tgttatgtag gagttaggtt ggcgtgatta gtagtttatt 8820
tagtggtgga aaattttgtt ataagatgtt taaagattga aatcgtcgtt tttatttttt 8880
gtagtttttc gttttaaatt aatattttag gatttagagg tggagggagt aggaggttta 8940
cgggtagggg tcggggtttg gtttaaggtt gttttgtgtt tggggtatta gtttagatta 9000
tttttttttc gttttgtgtg gattttttta taggttggat gtgagggtgt tttatattgt 9060
ttttttttatt tttttttttt tggtttttgg gtgtttttgt tttatttagg tttttttttg 9120
gaagttttcg ttgattattt tagtttatag taaggagtgt ttttaatttt agtttggtgg 9180
gttgtaattt ttatttttat gtttagattt ttgttttttt agtttgtttg ttgttttggg 9240
gatagattgg ggttaggttt agggagggga gttattagtt tgagggtggt atttggttat 9300
taggagttta ttattttgta gatggatttg gttttttcgg agaatttgtg ggagtatatt 9360
tttttcgttt ttaggattcg gcggtttatt ttttttcgtt ttattttttt tttgcggtcg 9420
cggaacggcg attggtttc gattatttta tagatgaggt agttaaggtt ttagtagtcg 9480
gggtttaggt cgtattttttg gttgtttagg atttttggag ttgggggtag aggagaggag 9540
agaggagtga ggaggaggga gatagttggt ggcgttgttt atagaaggtt tggagtttta 9600
tggtcgttat taatttttat tttattaatt gttgtgaata ttattttttt tagggagttt 9660
gttttgatga ttttttttttg tgttttcgaa ggattttgat tgtagtttta gaattttgat 9720
tatcgtggat ggaggtgttt tgtatttttg atgtttttttc gtttagaata tgagttattt 9780

```
tatttattat cgtaataata tgatatttgg tatagaatag gtgttattag tatatttgtt    9840
ttaatgaata aaggtgtatt tatttaaatt aaagatgatt ttaaatttat ttgtaattta    9900
aataattttt tgttatgttt tttgtattat tttgataaat cgaattatag atttattata    9960
gaaatataga aaagatagtt ttgtagtatt agtacgtatg tttggaggga aatgtattta   10020
tatatgtgtt ggtttgtata taagagtcgg ggtagataga tgtatggaat attttgtaaa   10080
aggagattta ggtaaatgta tatataggta ggtagattta ttatttattt atttattatt   10140
tattatttat ttaattattt atttattcgt ttattcgttt atttattaat tatcgattag   10200
tatttatgtg ggtatatata tatttatata tatatagata tatattttat aggttgttaa   10260
atgttatgta atttgaaaag gattttttag tattagagat aaggtatatt ttttgtgtag   10320
ttgttgttag tattagtttg attagagggc ggtagcgtgt ttgtttttcg ggtacgcgtt   10380
ttttagggga aggagggtag gtgtgtgaaa tgtataggag gaagtttagg gcgtgttttt   10440
aattggacgg gggattttag ggtataggat ttaattttgg gttttttttt tgtaattttt   10500
aggacgttgg ttttagagta gattgaggaa aggtggtagg agtttaaggt tgttttttat   10560
tggaattatg tttggtgata tagtagttgt gcgcgttttt tatttgcggg tttttgtgaa   10620
ttattttttt ttttttaaag gttttttttt ttagattttt gttttaatgt ggtttttttt   10680
agtagcgttg tatgttaggt ttcgattttt aagagtggtt tggttttgga tataggtggg   10740
gttggggggtt tgggagtgta gagagtttag agtagttatt atatttgggg tttttgtgtt   10800
tttttttttt ttggttaagg tcgtttttta gtagttttgt agagattgtg attttgtgtg   10860
tgattagtag tgtatgaaga ggcgagaggt tttgcgggta ggttttgggg gttcggtcgg   10920
cggtgagtag gtggtgggtt ttaaggtgta ttgatatagg tagtttagta tttatttatg   10980
tagttaatag tgtttattcg gtcgcggatt aggttttttt cggggatttt tatagttaag   11040
tttaggtttg agattttaat gtggtttgag ggggatagta gggttagtgg gtttttggga   11100
tagtcgtatg ggtattcggt tttgttgtgt gtttttgggt aaggtatacg agttttcggt   11160
atttttattt gtttaaggat aatagggatt ttaatttgtt attttagagt gattataaga   11220
gttttgggag agtgtaggtg tgggaggata taggaaaatg tttatttttt aggagtggaa   11280
tataaagatg taataggatg tttggattcg gttggacgtt gatcgatttg tttagttaaa   11340
ggttgaatgg gtttttttgga acgttgaggt tagagaattt tggtttggtt ttgggattat   11400
tttatgggaa aaatgtgagg ttttttttggt attatgtaat aatttgttta aggggaaaaa   11460
attataaaac gatttgggaa gtagtttttc ggtttgtttt gttggttttt ggagattggt   11520
tgtttttttat tttagagaaa cgagaaggga aaatatttgg tttgatttgg tagagtttgg   11580
agatgaattt tagtttatag tttaggattg agtttttaga gcgggagacg ttgagttgag   11640
gttttatttt ggtttttgtt tttttatag tttatttggg tagggtcgcg gttataattt   11700
attttatagg aaggaatacg tttttttttt ttttttgttt atggatatta gggttttatt   11760
tttatttttt tagtttagat gttagtaagg gttagtgtcg agatttcgtt aggttggagt   11820
cgtgtggatt ttttggttcg gtgggggttg atagttagga tagtagagga gtagcgtggg   11880
tttggttttt agattaaggg aggtatcggg tatatttaga gaggggcggt aggtttgggt   11940
ttttcgatcg ttttttttata gggtagtatt ttatgtagat ttttttttag gttattagcg   12000
attcggtttt tttttttttt ttattggggg ttgttaggtt ttggggtttg gtgggaggag   12060
atttagtttt tgtttttagt tttttattga tgttgttggg tgattttggt aggatggttt   12120
ttttgaggtt tagttttgtt atttgttaaa tgggtataga gatgttgttt tgtttttttt   12180
tgtgtaggtg ggaagattta gttagattaa aggtgtggaa atcggtggtt tatgtttgta   12240
attttagtat atagggaggt taaggagggt agattatttg aggttaggag tttgagatta   12300
gtttggttaa tatggtaaaa ttttattttt attaaagata taaaaattag tcgggcgtgg   12360
tggtacgtgt ttgtaatttt agttatttgg gaggttgagg tagtagaatt atttgaattt   12420
gggaggtaga ggttgtattg agttgagatt gtattattgt attttagttt gggtaataag   12480
agtaaaatta tattttaaaa aaaaaaaaaa atataaaaat tagttaggtg tggtggcgtg   12540
tatttgtaat tttagttatt tgggaggttg aggtaggaga attatttgaa tttgggaggt   12600
agaggttgta gtaggtcgag attacgttat tgtattttag tttgagtaag tgagattttg   12660
ttttaaaaaa aaaaaaagaa ggtataggaa ttattttttta ggtgtgaggt taaatagttg   12720
tgaggggtat tgtggttgtt tttattattt agtgtttata gttaagagaa aataatgatg   12780
gtaatgtata aacgtaatgt ttgagatatt tatagttggt tagattgata agtaattttt   12840
ttttaagttg ttatgtagaa gtttttttat tatttttttt tttttgggtg ggtagggtag   12900
ggtaggacgt tttgttttta ttttataaat aagatgagag gtggagaatt tgatggaagt   12960
taagtatttg tttagtattt ttgaaatttt ggattttttg tttagtgtat tgtagagatt   13020
acgttcggtt agaagttatt ttaatattta gagttttatg gttaaaggga attttaaaaa   13080
ttttgaattt taaagttttt ttgaatattg tttgtttatt aagcggagtt ttgtttatat   13140
ttatagtttt tttttttttt taggtattta gtattgagta gtgggagtgg ggttcgtggt   13200
tagttttttt agtttttttt gagaattttt gggaggtttt ttgttttttt ggggttgtaa   13260
tgtttatttt agttatcgag taggaaaaga tttattataa ttatttaata ggatgttttt   13320
aggttttaga tttttgaaag gaaaaataat aagtattaga gtgtgggtat tttgggtagg   13380
taggatatat tggtatttgg gtcgattttt cgggttaatt aggtttgagt ggttatagtg   13440
```

tgtttttttt ttattttttg gtttaggttg tttggtttga gttaaaatga tttttagtta 13500
attttttttt ttttagtaat ttaaagtaat attgtcgttt gttataggta ttttttaatt 13560
ttggagggtg attgggaaag ttaggaaaag gttagttatg gtttttgttt atatattaat 13620
gattgtaggt ttgaggatat tttaaggttg agaagatttt gtaggtttta ggaagttttg 13680
aaggtagaat agggtttgga atgaagggag ttgtgtaatt gggaagagag gattaaaggg 13740
gagaaggtgg ggatatagga aaaagatttt ttttgggatg gggtttgtag gagattggta 13800
tattttttaa tattagttac gttatagttt gggagaaaga atagttacgg ggtatggtat 13860
aggtgtggtt gttttatttt gtttttaatg tttttttttt tgatgatagg attttgttta 13920
taagtaaggg agtttttgta tttttaggat gaggggagag ttagtagagg agttgttttt 13980
ataggaaaag gtttttttta tgggtaaaag aatagttaag aggagagtgt gtgtaattta 14040
gttaagttat ggggtaaata gtatatagga tttggatgga ggttttagtt tttaattatt 14100
tagttttgtt tatttaattg atggtagttg tttttatgcg gagtggttaa tgtatttaaa 14160
tttagttagt gagttttatt aaatgataat tggtgtttgt gtatttttat ttattataaa 14220
atatttttgt tgttgttatt taaatggtat ttttttgatg tgttaataat tttaaataag 14280
ggtagtagtt attattttaa taagtcggtt gttattaggt tagtatggta gtttattagt 14340
gggtattggt tagaacggag tttaaagaga ggtcgtggtg agtggttata gttagggagg 14400
aggtattgtg gttttttttt tttcggaaga agacgagttg gaatttggag ggtttttttt 14460
ttttttttta aataaaaggt aattacgtag agaatttttt gtttaatttt tagggaaaat 14520
tttgattttt tttatttgaa tgaaaataaa atagaaatat aaatgggggg atggtatggg 14580
gggaagggta tgttattaag attatttttt tttgttttta aaattttttt ggttttaaga 14640
tgagttttat tgtaaaggtt tttaaattgg gttgcgtgtc ggggtttgtt aggatcgagt 14700
aggggggttt tttttgttcg tagtttgaag ggtttggatt tttttatatt aggatgtttt 14760
gttttggtat tttttgggat tttttttttta tttgtttttt tttaataatt tttttttttaa 14820
gttatagaag aaaagtagtt tttttttttt tcgagtatga ttgtagcgat ttattttaga 14880
tgttaaaatt tttgttttta aaggattgat tagaaatgag atttattatt aattataatg 14940
gtaattcgat agaagaagtt attttatttt attttgagac ggagttttat tttgttgttt 15000
aggttggagt gtagtggtga ttttgattta ttgtaatttt taggttaaag cgattttttta 15060
gttttagttt tttaagtatt tgggattata ggcgcgtgtt atcgtattcg gttatttttt 15120
gtatttttag tagagatagg gttttattac gttggttagg ttgattttaa attattgatt 15180
ttaggtgatt cgtttgtttt agttttttaa agtgttggga ttataggtgt gaattattat 15240
aatcggttag agaagttatt ttaatattta gagatttacg gttaaggaga attttaaaaa 15300
tttttgtatg tatttatata tttgttttag agatgtacgt taattagtga aagattttta 15360
ggtaaaaaaa tatattatta atattttggg gggatggaga gagtgaagga aatgatagaa 15420
aattttttat tattaaagag ttttttgtgt atttttggga taaatgtagg tggaaattaa 15480
ttggttatag taattagaga ttttattgga ttttttgagg agtgtaatgt aattgtttaa 15540
ttttaatata ttaaaaattg ttattatttt aatatgatgg aatattttaa atgtttattt 15600
aaaatgtagt attatagaga ttttttaaaa tttttttaga aggattggga gaaaaatttg 15660
aagggtattg tttattgtga gtggaatttg gatttttggt tttataatgt ttaggttttt 15720
gtgggattga ggtgggtggg gagagggggt gaagattaga gtaagttgag gatagtatgg 15780
agtgggatta tgggttattt cgattttgtt tgtgggtagt gattcgattt aagttttttt 15840
aggattgatt ttagatagcg gttattaggt tttttttttag tttttttggt tgttgggatg 15900
gaggtaattg gagggagtag tggttacggt tcgttttttt tgatttgaag gagtagttag 15960
ttttaggaat attattttat ttgtttggtt tttttaaatt ttggaggttg gagaatattg 16020
gttagagggt ggtttatttc gttttttttag tgtcggaggt tttagttttt tggagtggaa 16080
agtttgtttg attttgtatt ttttttttggt gttgattagg gttgattttt agagcgatgg 16140
taattatttg ttcgagggtg tggaggggtt ggttttattt tttcggattt agatttggaa 16200
gtttttggtg tttttttattt atcggtagac ggtgttttta cggtggaggt tttttaagtc 16260
gtagaggatt tttgtcgtat aaaataaggt tcgttttttt tcgaagttag ggttgtttat 16320
gttgtagatg tggaatttta ggttattttt atttatgatg gttaggatta agtatagtgt 16380
atttttggtt tcgtaggtat aggttaggtt gatttgggag tataagaata gtgttatggg 16440
gaggttgtga taatggttat ttatttttat tttttttttt aggattttgg gagttggggt 16500
gtgagggaat agaggagttt tatttttgttt ttttgtattt agatggtttt tgcggtgatt 16560
ttgtttgcgg tttttttggt tgataagggg tttatatttg tttttttttt ttagtggatt 16620
aaggtgagaa ggttaaggcg ggttagggta ggttagtatt tcgatttttt agtgaattta 16680
atagaatggc gtttgtgttt tttttataga agtatatgtg tgtattagtt ggggaagatt 16740
ggagggtgtt tttttggtta gttagaaaag tagggtttat ttgttttttt gggtatggga 16800
gttattttta tagggttaag taagggtgag ttggaggggt agtggggttt tgggaagtgg 16860
aatatggttt ggtaaaggtt tttttttgta tgtaggatgt gtttagttga tatggtttgg 16920
ttttgtgttt ttatttaaat tttattttga attgtatttt tataattttt atgtgttgtg 16980
ggagggatta gtgggagata attgatttat gggggtagtt tttttatatat tgtttttgtg 17040
gtagtgaata tgttttatag atttgatggt tttataaggg gaagtttatt ttatttggtt 17100

```
tttatttttt ttttgttatc gttatgtaag aagtgttttt tattttttgt tatgattgtg   17160
gggttttttt agttacgtgg aattgtaagt ttattaaatt tttttttttt gtaaattgtt   17220
tagttttagg tatgttttta ttagtagtgt gaaaacggat taatagagta aattggtatt   17280
agaagagtga ggtattgttg aaaaaaatat ttaaaaaggt agatatgatt ttggaatcgg   17340
taataggtag aggttggaag agtttgaagg gtttagaaga taggaaaatg tgagaaagtt   17400
tggaattttt tagagatttg ttgaatggtt ttgtttaaaa tgttgaaagt gatatggata   17460
ataaagttta ggttgaggtg gttttagatg gaaacgagga atttgttggg aattggagta   17520
aaggtgattt ttgttatttt ttagtaaaga aattgttggt attttgtttt tgttttagag   17580
atttttgaaa ttttgaattt gagagagatg atttagggta ttttatagaa gaaattttta   17640
agtaataaag tatttaaggg gtgatatagg tgttgttaaa ggtatttagt tttgtaaggg   17700
aaggagagta taaaggtttg gaaagtttgt agtttgataa tgtaatagaa aagaaaattt   17760
tatttttga ggagaaattt aggttggttg tttaaatttg tataagtaat aaggagttga   17820
atgttaattt ttaagattat ggggaaaatg tttatagggt atgttagagg tttttatagt   17880
agtttttttt attataggtt taggggttta ggaggaaaaa gtggttttac gggttgagtt   17940
tagggttttc gtgttgtgtg tagttaaggg atttggtgtt tagtgttttg agttttagtt   18000
gttttattcg tggttgaaag gggttaatgt agtgttcgag ttatggtgga agttttaagt   18060
tttggtagtt tttatgtggt gttgagtttg cgagtgtata gaagttaaga attgaggttt   18120
gggaattttc gtttagattt tagaagatgt gtgaaaatat ttgaatgttt aggtagaagt   18180
ttgttgtagg ggcggggttt ttatggagat tttttgttag ggtagtgtag aagggaaatg   18240
tggggttaga gattttatat agagttttta ttggggtatt gtttattgga gttgttagaa   18300
gagggttatt gtttttttaga ttttagaatg gtagatttat tgatagtttg tattgtttgt   18360
ttggaaaatt tgtagatatt taatgttagt ttatgaaagt agttaggagg gagacggtat   18420
tttgtaaagt tatagggatg gagttgttta agatcgtggg aatttacgtt ttggtatcgg   18480
tatgatttgg atgtgagata tgaggttaaa gagattattt tagagtttta agatttgatt   18540
gttttgttgg atttttgatt tgtatggggt ttgtagtttt tttgttttgg ttaattttat   18600
ttatttggaa tagttgtatt tatttaatgt ttgtatttat attgtattta ggaagtaatt   18660
aatttgtttt tgattttacg ggttcgtagg cggaaaggat ttgttttgtt ttagatggga   18720
ttttggatta tgtatggatt tttgagttaa tgttgaaatg agttaagatt ttgggggatt   18780
gttgggaagg tatgattggt tttgaaatgt gaggttatga gatttgggag gggttagggg   18840
tagaatgata tggttgggtt gtgtttttat ttaaatttta ttttgaattg tatttttata   18900
atttttatgt gttgtgggag ggattcggtg ggagataatt gaattgtggg tagtttttt   18960
tatattgttt ttatagtagt gaataagttt tatgagattt gatggtttta taaggggaaa   19020
ttttttttat ttggttttta tttttttttt gttatcgtta tgtaagaagt gtttttata   19080
ttttattatg attgtgaggt ttttttagtt atatggaatt ataagtttat taaattttt   19140
tttttgtaa attgtttagt tttagttata ttttaattag tagtgtgaaa atggattaat   19200
atattggttt atttggtaaa gggttattaa gagagggggt ttagtttttt gttggtggag   19260
tttagatatt taaggttttg tgaagtaagg ttatggtggg gtaggagggg taaattatat   19320
tagaaggtaa ttttttttta tatagggagg tggtatatat gaaattcggt tatcgtgggt   19380
taggttggga attggttgtg tcgtaaaatt ggtgtattaa tattagttat ttttgaggaa   19440
aggagataaa atggtagtta aggatgagta agtttatggg gattgtgttt ttgttgtaat   19500
ggttgtagtt gttttttttt ttttatattt gtttaggtga ttatcgagaa agggatttag   19560
gaggtgatat cgtgggaatt ttttgtcggg gtcgtggatg gggtatagtt aaagaaggtg   19620
tagttatcgt aattgaaggt ttttaaagac gtgttattta gttttgtgaa ttatatgatt   19680
ttttttttg agtagaattc gttagtattg ttttttaga gataaggttt tataggtagt   19740
atttaggggt ttttgttgaa gcggttggta gtggggagga aggtttcgga tttaagaagg   19800
ttcgtagttg aaatgatggt tgaagttagt tttatttgtt ttatttttat atattatggt   19860
attgtttata tttgtatttt cgataaggtt ggtagttggg ggtggtttgg gatgagattt   19920
taagatgtaa atttttattt tttgggagtg ttattggttt gtagtggttt tgtttgtttg   19980
ttttgttttg tttttaataa agttttattt tgttatttag gttggagtgt agtggtatga   20040
ttttagttta ttgtaatttt tgtttttttag gtttaagtga tttttatgtt ttagtttttt   20100
aagtagttgg aattataggc gtttattatt atgtttaggt aatttttgta tttttagtag   20160

aaatggggtt ttgttatgtt ggttaggttg attttgaatt tttgatttta agtgatttgt   20220
ttgtttcgat tttttaaagt gttgagatta taggcgtgag ttatcgtgtt cggttttgta   20280
gtggtttaaa gatggaaaat aatttttatt tatggggatt gattatatgg attatgtttt   20340
atttttttag gagaatgatg tttaatttta aaaaggggtg tgattagagg tttggatgga   20400
gaaaaagatt tataatgtaa gggaaaaata ttttttttata agaggtatag gaagatttta   20460
ttggttaaga ataataaagt ttgtgtatga ataaaatatg tttggagtga ttatttaaag   20520
agggaagttt ttcgttttat tgtgtatttt tttgtgttgt ttaaattttt attttgtgta   20580
tatattattt ttaatgtatg tatgtattta gttttagaga tgaggttttg ttgtgttatt   20640
taggttgggg tgtagtggta cgattatagt ttattgtagt ttcgaaattt tgggtttaag   20700
```

```
taattttttt gttttagttt tttgagtagt tgggattata agtatgtatt attgtattta   20760
gttttatttt ttataatagt gtgtagtaga agatattaga gttgattaaa tatttatgta   20820
ttttatattt tttagttttt tttgaaatta atttgttttt gtgttgattg gttttggttg   20880
gtagattgtg ggtgaaattg aggaatattt gtttagtaga ggtagtggaa agtttttgtg   20940
tggaattttt agtatttatt tttaaggttt tagatggggt agttgttaga tggaggaggg   21000
tggtttaatt tgattggatt ttaggtgagt gatagatgaa tttttgttgt atttagttat   21060
ttagggtttg gggtaaatgt gttattgtag tagggtttag tttagattga ttaatataat   21120
gtatatagat ttgatagtgt tattttagga tgttggtatt aaggttttta ttacgatggt   21180
attagtttag agggtttgtg gaatttatag tttttagttt gggtttaggt gaattttttt   21240
tgtttattga ggagtttata aagtggttta ggagaataga ggcgtcggtt ttatgtatag   21300
tagggagtta ggtattggtc ggttttgggg atgggataaa gttagttata gtcgattata   21360
atgggtcgtg tgatggagga atgttttgcg gtttttttgt agtttagcgt tgttattggt   21420
attttttttt ttagggggtgg ggatcggtcg tattagagga ttacgttttt tcggagggag   21480
ggaagtttta gtttatagaa gggcgagtcg gttattgggt ttagatgttt atttattata   21540
aattgattgt tgattttttc gaggatttgt tttttattga gggttatgga tttttttttt   21600
ttttttttga tttttttttt ttttaagcgt ttgtaggtat atattttatt cgtggttcga   21660
atttggtagg tatagatttg tagtgtaaga aagaaattag ggtggtagga gtatcgtgga   21720
ggtttgtttc gagttttttt agggcgggga ttataggttt tttaggtaga gaatggatgg   21780
ggttagagtc ggttttgttt ggttttatga gtgttttagg tttttaggat ttagtgatgt   21840
ttggtttggg gtttatagtt gttttgggtt tttgtttttt tttttttttt tttttaagga   21900
tttttagttt tggaaggggt agaatgagtt atttatatta aggtttagat aattgattgg   21960
ggttttaagt atcgttttat ttttagggta ataggatttt tttttatcgg ttgtcggtag   22020
agaagtttgt tttgggaggt agtgatagga gatttttaag aatattgatt ttataattta   22080
gataagcggg aattaaagta tttgggttta agttttagtt ttgttttata ttgtttgcgc   22140
gtttttggtt gggttattta attattttgg attttagttt tttttatttt taaaatggga   22200
ataataaggt ttgttttata tgttgggaag atcgggtaat gtataacgaa atttcgaatt   22260
ttgtatgata gcgttattta ggtattgagt ggttaaggtt gtttagtttt tttacgtttt   22320
tatatttttt ttttatatac gattagggtt gtttatttgt tttttgtagg ttggattttg   22380
tttgaggttg gtaggagagt acgtttgttt tatttgttta gttttataat ttttttttat   22440
tatagatata tttgatagtt ttaaggttat tgattttttt ttattttttat tgttttcggg   22500
gtttatattg agttattggt gttttcgggg gattcgtggg ttaatttttt aggttgattt   22560
agaagggata ggaaaggtga tttgaatgta agaattttta attaaaaacg tgaatgttta   22620
tttatttttt cgaagttttt tttttttagt attcgatatt gtttgaaagt gtttttggtt   22680
atcggttgtt tg                                                        22692
```

<210> 109
<211> 5096
<212> DNA
<213> Artificial Sequence

<220>
<223> chemically treated genomic DNA (Homo sapiens)

<400> 109

```
gaggtcgagg cgggcggatt atttgaggtt gggagttcga gaatagtttg attaatatgg     60
agaagtttta tttttataaa aaatataaaa ttagttaggt gtggtggtgt atgtttgtaa    120
ttttagttat ttggtaggtt gaggtagaag aagagtttga attcgggagg cggaggttgc    180
ggtgagtcga gatcgcgtta ttgaatttta gtttgggtaa taatagtgaa attttgttta    240
aaaaaaaaaa atgtttataa ttgttatttt ataagaaagg aaaaagtttt taaagagata    300
tataaaaagg atatttatag ataaaagaaa ttttatattt aggaggttat tagattgatt    360
ataaaatata ttcggatttt aaaagtatta gaatattagg agagaaaaaa aaaaaattag    420
gttaggtacg gtggtttacg tttataattt tagtattttt ggaggttgag gtagatggat    480
tatttgaggt taggagttta agattagttt ggttaatatg gtgaaatttt gtttgtatta    540
aaaattagtc gggtatggtg gtaggcgttt gtaattttag ttatttagga ggttgaggta    600
ggagaattat ttgaattcgg gaggtagagg ttgtagtgag ttatgatcgt gtcgttgtat    660
tttagtttgg gtaataagag taaaattttg ttttaaaaaa aaaaaaaaaa aagaagaaga    720
agaaaagaaa agaaaattat tgttaatttt tattatattt tgttaaatat atattttgtt    780
atattatttg ttttagtttt aaggttattg gtattttttg aagtaattat taaatgtttt    840
tattatttaa gaatagagag ttaatggtga taattaattt atatagagta ttgaaaggag    900
ttaggttttg tttgtttttt tttgtatagt aattttgtaa tagtattata aggtaggtat    960
```

tgttattatt tttattttat aaataagaaa attggagtat aggttgggcg cggtggttta 1020
cgtttgtaat tttattattt tgggaggcgg aggtgggtgt attatttgag gttaggagtt 1080
taagattagt ttggttaata tggggaaatt ttatttttat taaaagtatg aaaaaattag 1140
tttggcgtgg tggtatatat ttgtaatttt agttatttcg gaggttgagg taggataatc 1200
gtttgaattc gaaaggttga ggttgtagtt agttgagata gagtttttgt atttaggttt 1260
gggtgataga atgagatttt gttaggaagg aaggtgatag aatgagattt cgttaggagg 1320
gagggaggga gggagagaaa agagggaggg agattggagt atagagaagt taattgattt 1380
gtttaagaag ggagggaggg agggagagag ggagatcgga gtatagagaa gtgatttgtt 1440
taggaaggaa ggaaggaagg taggaaggaa ggaagggagg gagggaagga aggaagggag 1500
ggagggaggg agggaaaaga gggaaggaga ttggagtata gagaagttaa gtgatttgtt 1560
taagaaggaa gggagggagg gagattgaag tatagagaag tgatttgttt aggaaggaag 1620
gaaggtagga aggaagggag gggcggaggg agggaattgg agtatagaga attgatttgt 1680
ttaaggttat ttagtagaag gaagggagga aggaagggag gaaggaaggg agggagggag 1740
attggagtat agagaagtta agtgatttgt ttaagacgga cggatacgga cggaaggaag 1800
gaataaagaa ggagggaggg agggagggag ggaattggag tatagataat tgatttgtgg 1860
aaggaaggac ggaaggaagg agagagggaa cgaaggaagg agagagggaa ggaaggaagg 1920
aaggagagag agggaaggaa ggaaggagag agggagagag ggagggaagg gagggaattg 1980
gagtatagag aattgatttg tttaaggtta tttagttagg atttgagttt agaatgtgag 2040
tgtttttagt tattatattg aattgttttt tagttgagaa tttttaataa atgtattagg 2100
atatatataa ttggatgtta taaggtggtt ttcggttaat tttttttttt aaatatatgt 2160
attaatttta aattatttat atgataaaaa atgtttatat ttttatcgat ttcgttttta 2220
aaatggtatg gtttttttagg attatttttt ataatcgtta aagggaaatt cgttttgtta 2280
atatgaaaaa aaaattaaat atttgtttta tgagttgtta gtggttaacg ttgtttagat 2340
tacggtttga acgtattatt ttttaaagat tattttgtgg ttttgggtaa attgtttagt 2400
ttttttttgtt tttttagcgg taaagtggat ataataatag tattattttt taaagtggtg 2460
aggattagtt gatattattt atataaattg ttatgtatag tatttgatat agagcgagtg 2520
tttaataaat gtttttttatt atttaggttt tgggtttttt tatttttaga gtgtaagttt 2580
tttagtaatt atagaaacgt tttttagagt cgttgtttat tttttaaaaa tatttgtttt 2640
tatttcgtat agagtatagt gatattataa ataggttttt ttggatttta gttttggggg 2700
tggttatatt atttcgttta ggtttaggaa gagagagtcg gttttggttt tttattcggt 2760
agtagtataa aatttaatta ggattttttta tttcggttaa tttttgaatt atcgcgttta 2820
gtttggttgg ttcggtagta gatggaatga gggcgtcggc gttaggaagt ggcgtttatt 2880
tatttgttta gttcgaggtg ggtatttttt ttttatagta ttttcgggga tttcgttttt 2940
ttatcgcgga acgttttaga ggatcgattt aatagtgtta tagggtgggt tttttttttaa 3000
atttttttaaa atatatcgtt acgatttcga aacgggagtc gagggagacg tattaagtgt 3060
ttttcgacgt taggtgaagc gtattatatt tggtgtgcgt gttgagtttc gaatcgaagt 3120
tcgtgcggtc gttatttttg tttttttgtt gggggtttta gagagtgtgt tttttgttta 3180
cgtgcgcgta gggttcgtag tcgtttttac gttttagcgg agttgtagta cgtttttttt 3240
atttattagg tgaagagttt gttttttattt ttgcgtagta ggttggagaa ttcggtcgtc 3300
gttttttttcg cgaggggttt tggggcgtcg gggttcgttg gcgagtttgt ttttatcggc 3360
gtttttgcgc gttttcggcg tttaaaattt tcgaagtgtc gggtttcgga agacgggagg 3420
cggtgtttgt gcggtcggtt ggggtttcgt tttggttttg cgtttttttt ttcgtttttt 3480
tgttcgcgtt tttttcgttt tttcgttcgg tttttttagc gattaattat taattgttaa 3540
gttttaaggg ttagataata tatttgtaga ttataatttg gtatttttag ggtggtagga 3600
ggagaaaggg taattttttt ttcgttttgg tagtcgagtt ttattgttga gcggtattta 3660
gtgtgttttt aggtttttag ggttttttgt ttgaaattat aagatatagt gatacgaggt 3720
tattgtaggt gatttttaag ttaagaagcg gtgagtgagt agtgagttag aagtagtgag 3780
tgagtcggga tttttttaat tggaaggatt ggttgatttt tttttttttt ttttattttt 3840
tttttttttg gttgatttgt ataaaatcgt tttattaaaa taattgaagg ttggtagggc 3900
gcgttggttt atatttgtaa ttttaacgtt ttgggaggtc gagacgggtg gattatttga 3960
ggttaggagt tcgagattag attgggaaat atggtgaaat ttcgttttta ttaaaaatat 4020
aaaaattagt cgggcgtggt gacgggtgtt tgtagtttta gttattcgag aggttgaggt 4080
aggagaatcg tttgaattta ggaggcggcg gttgtgagtt aagatcgcgt ttttgtattt 4140
tagtttgggt aatagaggga gacgtcgttt taaaaaaaaa aaaaaaaaaa aaaaaaattt 4200
gaaggcgttt taggtttgtg tcgttttttt ttgtaaaata tgggtaagat tttaaaaaga 4260
ttttggaaac gtgtttttgt ggcgtggatg gaggcgtggt ttatttttcg cggtgtgttg 4320
gttttgtttt ttagtattta gttgttttcg tcgtgttagg gagagagacg gtgttaattt 4380
tcggcgttcg gtttggatgt tttgttttgg ttacgttttt cgaggttttt acgggttaaa 4440
gagggattaa gagatattaa gttggggtcg aaaagttttt gttattgatt ttttgttttc 4500
ggaaaggagg agatttagaa aataaaagaa aaggtgttgg gattttttat tttttttttgg 4560
ttatttgttt tgggggttgg ggtgggttaa aataaacgta ggtttaaata gataggttgt 4620

gtagacgagt tggtaatttt gtaagaggag tttaagaaaa aaaatagttt tcgaaaatga 4680

aatattattt taggaatttt aagtttagta gttgtaggaa ttttaaaaaa atttttgaaa 4740
ttaagaacgt ggttattcgt agagattaat taagtattat aatataagta tattatgaaa 4800
taagaggata ttagaagtat atttaagatg ttattttgt aaagtaaata atttatttaa 4860
ttttgtatat tgataacgtt gaattttatt tgagttttgt gtttttggaa agtaatgata 4920
attaagaaat ttttttaggt cgggcgcggt ggttgacgtt tgtaatttta gtgttttggg 4980
agatcgaagc gggcggatta tttttgaggt taggagttcg agattagttt gattaatatg 5040
gcgaaatttc gtttttatta aaattataaa aattagttcg gcgttttggt gtgtgt 5096

<210> 110
<211> 5096
<212> DNA
<213> Artificial Sequence

<220>
<223> chemically treated genomic DNA (Homo sapiens)

<400> 110

gtatatatta aaacgtcggg ttaatttttg tagttttagt agagacggga tttcgttatg 60
ttggttaggt tggtttcgaa tttttgattt taggagtaat tcgttcgttt cggtttttta 120
aagtattggg attataggcg ttagttatcg cgttcggttt aggaaggttt tttaattgtt 180
attatttttt aggagtatag ggtttaggta gaatttaacg ttgttaatat gtagggttaa 240
atgagttgtt tattttataa aagtgatatt ttaaatgtgt ttttaatgtt tttttgtttt 300
ataatatgtt tgtattataa tatttaatta gtttttacgg atgattacgt ttttagtttt 360
aaaggttttt ttgaggtttt tgtagttgtt aaatttgggg tttttgaagt aatattttat 420
tttcgaaagt tgtttttttt tttagatttt ttttatagaa ttgttagttc gtttgtatag 480
tttgtttatt tgaatttacg tttattttga tttattttaa tttttaaagt aggtggttag 540
gaaaaaataa aaggttttaa tatttttttt tttgtttttt ggattttttt tttttcggaa 600
gtaggggtt agtgatagga atttttcgat tttagtttag tgtttttttaa ttttttttta 660
gttcgtggaa gtttcggaag gcgtggttag ggtagggtat ttaagtcgag cgtcgggggt 720
tggtatcgtt ttttttttg gtacggcgga gataattgaa tattgaaaga taaaattaat 780
atatcgcgag gaatgagtta cgtttttatt tacgttataa aggtacgttt ttaagatttt 840
tttaggattt tatttatgtt ttgtagggaa aagcgatata gatttgaagc gtttttaggt 900
tttttttttt tttttttttt tttttaagac gacgtttttt tttgttgttt aggttggagt 960
gtaaaggcgc gattttggtt tataatcgtc gtttttgggg tttaagcgat ttttttgttt 1020
tagtttttcg agtagttggg attataggta ttcgttatta cgttcggtta atttttgtat 1080
ttttagtaga gacggggttt tattatattt tttagtttgg tttcgaattt ttgattttag 1140
gtgatttatt cgtttcggtt ttttaaagcg ttgggattat aggtgtgagt taacgcgttt 1200
tgttagtttt tagttatttt aatgaagcga ttttgtataa attagttaga gggagggaga 1260
gtgggaggga agaggagggg ttagttagtt tttttagtta ggagagtttc ggtttattta 1320
ttgtttttaa tttattgttt atttatcgtt ttttagttta ggaattattt ataataattt 1380
cgtgttattg tattttgtga ttttaaataa ggaattttaa aaatttgaag gtatattggg 1440
tgtcgtttaa taataaggtt cggttgttaa gacgaaaaaa aaattgtttt tttttttttt 1500
gttattttgg aggtgttagg ttgtaatttg taaatgtatt gtttggtttt tagggtttga 1560
tagttaatga ttggtcgttg aaggagtcga gcggaggagc gagagggacg cgagtaagag 1620
agcgagggag ggagcgtagg gttagagcgg agttttagtc ggtcgtatag gtatcgtttt 1680
tcgtttttcg ggattcggta tttcgggggt tttgggcgtc ggagacgcgt aagggcgtcg 1740
atggaggtag attcgttagc gggtttcggc gttttagagt ttttcgcgga gggagcggcg 1800
gtcgagtttt ttagtttgtt gcgtaggata aaaggtaaat tttttatttg gtgagtggaa 1860
ggggcgtgtt gtagtttcgt tggggcgtgg gggcggttgc gggttttgcg cgtacgtgga 1920
taaggagtat attttttgag gtttttagta gggaggtaga ggtggcggtc gtacggattt 1980
cggttcgggg tttaatacgt atattaggta tggtgcgttt tatttggcgt cgggaagtat 2040
ttggtgcgtt tttttcggtt ttcgtttcga aatcgtggcg atgtattttg gaggatttgg 2100
ggggaaattt attttgtggt attattaagt cggtttttttg gggcgtttcg cggtggaggg 2160
acgaggtttt cggggatgtt gtgaaggagg agtgtttatt tcgggttggg taggtgggtg 2220
agcgttattt tttggcgtcg gcgtttttat tttatttgtt gtcgagttag ttagattggg 2280
cgcggtgatt taagagttga tcgaagtgag aaattttggt taagttttgt gttgttgtcg 2340
ggtgggagat tagagtcggt tttttttttt tgggtttgag cgaggtgata tagttatttt 2400
tagggttggg gtttagggaa gtttgtttgt ggtattatta tgttttgtgc gaagtaggga 2460

```
taaatgtttt taaggagtgg gtagcgattt tagaaagcgt ttttgtggtt gttggaggat    2520
ttgtattttg gaaatgagag aatttaggat ttaaatagtg aaagatattt attgggtatt    2580
cgttttgtgt taggtattgt gtataataat ttatatggat aatattagtt aatttttatt    2640
attttaggag atggtattat tgttatattt attttgtcgt tggagaaata aagaaggtta    2700
agtaatttgt ttaaggttat aagatgattt ttaaggggtg atgcgtttaa gtcgtagttt    2760
aaatagcgtt ggttattgat aatttatgaa atagatattt gattttttt ttatattaat    2820
aaagcgaatt ttttttaac gattgtgaaa gatagttttg aaaagttata ttattttaaa    2880
aacgagatcg ataaaggtgt aagtattttt tattatataa atagtttgaa attgatgtat    2940
gtgtttaaag gggaaagttg gtcgaaaatt attttgtagt atttagttgt gtatgttttg    3000
atatatttgt taaaagtttt tagttgggag gtaatttagt gtaatggtta agggtattta    3060
tattttggat ttaaattttg gttgggtgat tttggataag ttagtttttt gtgttttagt    3120
tttttttttt tttttttttt tttttttttt tttttttttt tttttttttt tttttttttt    3180
tttttttttt tttttttttt ttttcgtttt tttttttttt tttttcgttt ttttttttat    3240
aagttagtta tttgtatttt agttttttttt tttttttttt tttttttttt ttgtttttt    3300
ttttcgttcg tgttcgttcg ttttggataa gttatttaat ttttttgtgt tttagttttt    3360
tttttttttt tttttttttt tttttttttt tttttttttt tgttgggtga ttttggataa    3420
attagttttt tgtgttttag tttttttttt tcgtttttt tttttttttt gttttttttt    3480
tttttggat aagttatttt tttgtgtttt agtttttttt tttttttttt tttttggata    3540
agttatttaa tttttttgtg ttttagtttt tttttttttt tttttttttt tttttttttt    3600
tttttttttt tttttttttt tttttttttt tttttgtttt tttttttttt tttttggata    3660
agttattttt ttgtgtttcg gttttttttt tttttttttt tttttttttt ttggataagt    3720
taattaattt ttttgtgttt tagttttttt tttttttttt tttttttttt tttttttttt    3780
ttgacggagt tttattttgt tattttttt tttgatagag ttttattttg ttatttaggt    3840
ttgagtgtag aggttttatt ttagttaatt gtaattttaa tttttcgggt ttaagcgatt    3900
attttgtttt agttttcgaa gtagttggga ttatagatgt atattattac gttaggttaa    3960
tttttttatg tttttagtag agatggggtt tttttatgtt gattaggttg gttttgaatt    4020
tttgatttta agtgatgtat ttattttcgt tttttaaagt agtgggatta taggcgtgag    4080
ttatcgcgtt tagtttgtgt tttagttttt ttatttgtaa aatggagata ataaatagtat    4140
ttattttgta gtgttgttat aaggttatta tgtaggaaag ggtagataga gtttggtttt    4200
ttttagtgtt ttatatgggt tagttgttat tattagtttt ttattttttgg gtgatgggaa    4260
tatttggtga ttgttttagg agatgttagt ggttttgagg ttggggtaga tgatatggta    4320
agatgtatgt ttaataagat gtgataagaa ttaataatga tttttttttt tttttttttt    4380
tttttttttt tttttttttt ttgagataga gttttgtttt tgttgtttag gttggagtgt    4440
agcggtacga ttatggttta ttgtaatttt tgtttttcgg gtttaagtga tttttttgtt    4500
ttagtttttt gagtagttag gattataggc gtttgttatt atgttcggtt aatttttaat    4560
atagatagga ttttattatg ttggttaggt tggttttgag tttttgattt taggtgattt    4620
atttatttta gtttttaaaa gtgttgggat tataagcgta agttatcgtg tttggtttga    4680
tttttttttt ttttttttta atgttttgat atttttggaa ttcgggtatg ttttataatt    4740
agtttagtag tttttttaaat atgagatttt ttttgtttat gaatgttttt tttatgtatt    4800
tttttgaggg tttttttttt ttttgtggaa tagtagttat gggtattttt tttttttaga    4860
tagagtttta ttgttgttgt ttaggttgga gtttaatggc gcgatttcgg tttatcgtaa    4920
ttttcgtttt tcgggtttaa gttttttttt tattttagtt tgttaagtag ttgggattat    4980
aggtatgtat tattatattt ggttaatttt gtattttttg tagagatggg gttttttttat    5040
gttggttagg ttgttttcga atttttaatt ttaggtgatt cgttcgtttc ggtttt        5096
```

<210> 111
<211> 3072
<212> DNA
<213> Artificial Sequence

<220>
<223> chemically treated genomic DNA (Homo sapiens)

<400> 111

```
ttttaattta gcgggacggt ttgtattttt atttgttaaa tttggtaatt tttggggttg      60
gggtttgggg ttcggtaatt tttggggtgg gaggtttttg tgtagagttt aatttgttcg     120
ttaaggttta tttatcggag aagaattggt tgatggaggt gggtaggagc gtgaggaagg     180
ttaaggggtg ggcgaaggag atggaaagga tagcggagat gtaggttacg tcggttatta     240
tggagtagag gtaggttagg gaggtgtaga ggtagaatag gacgaagttg cgtatgtttt     300
tgttgtcgat gtagttgtcg gtgaagaaat agtgatggtc gtgttttagg gtgattttgg     360
```

```
cgtatattcg gtagaagtgg gtgttaggtg aggggtatgg agttttttttg gtcgaggttt      420
tttggtaggt ttttaggtcg tttggggagt tttggatgat aaggacgtaa ttgtttaggg      480
cgttggtcga gaggaatagg aagagcgttt cgtggagtag ggcgggcgag aagagtcggg      540
cggtcgcggg gttttcgcgt atgttgggta ggaagaggaa gagttgtagt acgaaggtta      600
ttagggagat gtataagaag taggcggggg ttattacgtt gagtagtcgt agggttagta      660
ttttcgatta tatttttgcg gggttcgggg tgagaagagg attgattgat tacggcgttt      720
agggggcgtt ttcgttcgtt ttttcgtgtc gcgcgtttgg ggtattgtac gcgattttac      780
gtttttaatt ttttgtttcg cggatttttt tttttagtt ttttaatttc ggattaggta      840
ggttagagat tcgagttcgt agcggtttcg ggtttaggtt ttatttgggg gtgagtgttg      900
tttggggttt tgagcgagtt cggcgttttg gcgcggtaga gtttggtata ggagggaggg      960
attcgtttgt agtttttagg agttgtgagg gtggggggta tcgggggata ggattttttt     1020
tttttgtgga tgtggaaata agtttaggtt tgggattttt ggtttagatt tttttaggag     1080
gagaaaggga aggatttggg gtcgggggta ggagaattag cggtatttga tttgttggaa     1140
attttgtttt atattttttt tttttcgttt ttgcgttttt atttatatat atattttggg     1200
tagtatttag ggattttatt aggttattta aggtttttttg gaggtatgtg tgcgtgaggg     1260
attacgatgt gtgtgtgtgt gtttttgttt gtgattttgt acgtgtaatt tgtgtgcgtt     1320
tgagtatttg tatttgtgta tgtatatatt tgcgtgtata gatacgtagt ttttgttatt     1380
tagttttaaa aaaattaatt gaaataatcg atatatttat attcgttttc gggtatcggg     1440
tgaggtgggg gcgtaattag aatatttgtg ttagcgggtt ttggttagta tatttttttta     1500
acgttggttg ttcgggttat tttttttgcga agagcgtttt tatttgaggc gaaattaaaa     1560
tttttttttc gcgttcgttt ttcgtttttt tgttgagaga aatttaaata atttttatgg     1620
cgtcgaaatt ttttttatgt cgataagttc gttttgggcg tacgagtgga tgtttggtta     1680
gtttttttttg ggatcgattt cgtcgtcgtg tttagttttt attacgtttt tattttattt     1740
tacgttttat agtcggggtt tttggttagt ttcggaagtt atcgagaaat aggatttcgt     1800
gcgttcgaga gaattttttt aggggttaag gaatcggtta gtcggaggcg cgagaaaagt     1860
tttgggaagg cggttgtatt taggatgggt ggatgttacg gggttttcgt ttaggttgtt     1920
tcgttcgtac gggtcgattg gttattttgg aattttttttt gtaggtttta gcgtttttcg     1980
ggaattcgta gttttcgcgg agagcgtggg ttttttttta tcgttggggc gtagcgtagt     2040
gtacgtttga gggtggtcgt cgggggttgg gtacgttttt agttttgcgt cgtcgggggt     2100
tgcggcggtg ttgtttattt tagagagttt tcggtttggg gtttcggcga agtaagtgtt     2160
ttttcggcgt cggtcgttag gggggcgcgg gagtagttag atgcgtcgta gcgttgggaa     2220
ggcggcgaag gataggggtt aggggagtga ggggcgttcg gtaggtagtt ttagttttgg     2280
ttttgcgcgg gagaagggat agtagagatc gttcgtgggg tttcggggtg tagggagttg     2340
ttcgtttagt tttggcgttc gtttcgttcg cggtagaggg cggtatagtc ggagttttgg     2400
aaagatcggt agcgtcggta gtcgcgggtt tttcggttat tgtttttcgg acgtacggga     2460
agtcgttttt cgcgtcgtcg tcgtcgtatt gtcgtcgtcg tagaggggtg aggaaattaa     2520
tttatcgagt tttggtcgtc gataagagga gtttcggacg tcggttttcg ttttgttcga     2580
ggttgtaaag ttgtggattc ggttcggttg gttcgtagtt tgcgtttcgt ttcgggaatt     2640
gggtaagtag cggggatgtg gggaggaggg agcgggtagt tgttggtttt ttatttgggc     2700
gttagcgagt aagggttagg aagcggcggg gatggtagtt gggtattttt tagtttcgtt     2760
atttttttttt tcgtttttgg gggcggtgtt ttggtcgtta gttttagtat cgtggatttg     2820
tttttttttt ttttttcgat ttttttttgag cgggttgggg cgttttgttt ggttttagcg     2880
ttcgttttag ttgggagatt gggatttagt tttgatgtgg attttttagt tattattttg     2940
tttttttttt tcggttaggt tttacgattt tttttgtttt ttattcggat tgaaggaatt     3000
ttggggggttt gagaggttag tttgttaggg taatatttat gaagattagc gttatttttt     3060
cggaagggtt gt                                                         3072
```

<210> 112
<211> 3072
<212> DNA
<213> Artificial Sequence

<220>
<223> chemically treated genomic DNA (Homo sapiens)

<400> 112

```
atagtttttt cgaggagatg acgttgattt ttatgggtgt tgttttggta agttgatttt       60
ttaagttttt agggtttttt tagttcgagt ggagagtagg gaaggtcgtg ggatttagtc      120
gagagggaag ggtaggatga tggttggaga atttatatta gggttgaatt ttaatttttt      180
agttggaacg ggcgttgaaa ttaggtaggg cgttttagtt cgtttaaggg gaatcgagag      240
```

```
gggaggaggg ggtaagttta cgatgttgag attggcggtt agagtatcgt ttttaggagc     300
ggagggaggg gtggcggggt tggggggtgt ttagttgtta ttttcgtcgt tttttggttt     360
ttattcgttg gcgtttaagt gggaagttag tagttgttcg tttttttttt tttatatttt     420
cgttatttgt ttagttttcg aagcgaagcg taggttgcga gttagtcggg tcgagtttat     480
aattttgtag tttcgggtag ggcgagagtc ggcgttcggg gttttttttg tcggcgatta     540
gagttcggtg agttgatttt tttatttttt tgcgacggcg gtagtgcggc ggcggcggcg     600
cggggggcgg tttttcgtgc gttcgggagg tagtggtcga gaagttcgcg gttgtcggcg     660
ttgtcggttt ttttaaggtt tcggttgtgt cgttttttgt cgcgggcgag gcgggcgtta     720
gggttgaacg gatagttttt tatatttcgg ggttttacgg gcggtttttg ttgttttttt     780
tttcgcgtag ggttagggtt gaggttgttt gtcgagcgtt ttttattttt ttagtttttg     840
tttttcgtcg tttttttagc gttgcggcgt atttggttgt tttcgcgttt ttttggcgat     900
cggcgtcggg aaggtatttg tttcgtcgga gttttaggtc gagggttttt tggggtgggt     960
agtatcgtcg tagttttcgg cggcgtagga ttgggggcgt gtttagtttt cggcgattat    1020
ttttaggcgt gtattgcgtt gcgttttagc ggtagggaga ggtttacgtt tttcgcggag    1080
gttgcgggtt ttcggggggc gttgggattt gtaaagggga ttttaaggtg attagtcgat    1140
tcgtgcggac gggatagttt ggacggaggt ttcgtggtat ttatttattt taggtgtaat    1200
cgttttttta gaattttttt cgcgttttcg gttggtcgat tttttagttt ttggaaaagt    1260
tttttcgggc gtacggaatt ttatttttcg gtggttttcg aagttggtta ggaatttcgg    1320
ttgtgaggcg tggggtggga tggggacgtg gtgagggttg agtacggcgg cggggtcgat    1380
tttaggaaag gttggttaag tatttattcg tgcgtttagg gcgggtttgt cggtatggaa    1440
aggatttcgg cgttatgagg gttgtttggg tttttttttag taggagggcg ggaggcgggc    1500
gcgaaggggg gattttaatt tcgttttaaa tgaaaacgtt tttcgtaaag aaatagttcg    1560
ggtagttagc gttggagaga tgtgttggtt aggattcgtt gatataggta ttttggttgc    1620
gtttttattt tattcggtgt tcgagagcga gtgtgagtgt atcgattgtt ttaattgatt    1680
tttttaaagt tagatagtag aggttgcgtg tttgtatacg tagatatata tatatataaa    1740
tatagatatt taagcgtata tagattatac gtatagagtt atagataagg atatatatat    1800
atatatcgtg gttttttacg tatatatatt tttaaagagt tttgggtaat ttggtgaggt    1860
ttttgagtgt tgtttaagat gtgtgtgtgg gtggggacgt agggacggag aaaggggagt    1920
gtgaagtagg atttttagta ggttaagtgt cgttgatttt tttgttttcg attttaaatt    1980
tttttttttt tttttttagg gaggtttgaa ttagaaattt taaatttggg tttgttttta    2040
tatttataga ggggaggagt tttatttttc ggtgtttttt atttttataa tttttaaaga    2100
ttgtaaacga gttttttttt tttgtgttag attttgtcgc gttaaggcgt cgagttcgtt    2160
taaaatttta ggtaatattt atttttaggt ggggtttaga ttcgaggtcg ttgcgggttc    2220
gggtttttgg tttgtttgat tcggggttag ggaattgggg aggggaaatt cgcgggatag    2280
ggggttgggg gcgtggagtc gcgtgtagtg ttttagacgc gcggtacgga ggagcgggcg    2340
agggcgtttt ttggacgtcg tagttagtta gttttttttt tatttcgggt ttcgtaggaa    2400
tgtaatcgag gatgttggtt ttgcggttgt ttaacgtggt ggttttcgtt tattttttgt    2460
gtattttttt ggtgattttc gtgttgtagt tttttttttt tttgtttagt atgcgcgagg    2520
atttcgcggt cgttcggttt ttttcgttcg ttttgtttta cggggcgttt tttttatttt    2580
tttcggttaa cgttttgggt aattacgttt ttgttattta gaatttttta gacgatttgg    2640
gggtttgtta gggggtttcg gttaggaaga ttttatgttt tttatttagt atttattttt    2700
gtcgagtgtg cgttagagtt attttgaggt acgattatta ttgttttttt atcggtaatt    2760
gtatcggtag taggaatatg cgtaatttcg ttttgttttg tttttatatt tttttggttt    2820
gtttttattt tatggtggtc ggcgtggttt atattttcgt tgtttttttt attttttttcg    2880
tttatttttt ggtttttttt acgtttttgt ttatttttat tagttagttt tttttcggtg    2940
agtgggtttt ggcgggtagg ttgggtttta tataaagatt ttttatttta aaggttgtcg    3000
ggttttaaat tttaatttta aaagttgtta gatttagtaa ataaaaatat agatcgtttc    3060
gttaaattaa aa                                                        3072
```

<210> 113
<211> 3845
<212> DNA
<213> Artificial Sequence

<220>
<223> chemically treated genomic DNA (Homo sapiens)

<400> 113

```
tcgtttttta cgtttaagta atttttttgt tttagttttt cgagtagtta agattatagg      60
tatgtgttat tacgtttagt taattttttt gtttttttgta gagatgggtt ttattatgtt    120
```

ggttaggatg gtttcgattt tttgatttcg tgatttgttt atttcggttt tttaaagtgt 180
tgggaatata ggcgtaagtt attgtgttta gtttatattt tatttttcga ttgattttcg 240
tgattagagt taattttttta tgttatttta tgtgaatatg tattaatgaa ttattagaaa 300
ttgtaatgtt tttgtaggtt aaaataattt aatattgata attttatatg atttagttta 360
atatattatt ttttttatta gttatattgt atgttattat ttatatatac gtttatttag 420
ttagtttatt tgttttttgt gaggaatatt atatagtttt tgagattttg ttacgataga 480
agttagtatt gataagtttt tgcgtatttg ttttaatgta ggattattgg gaattgagta 540
gtagtttata gggtttggga ggggatttaa ttatttagtt aatttttatt tatattattt 600
tttttttttc gataatgggt aattgagaaa gggtaaatgt gtgtaaaaat tatgtagatg 660
tagttttagg attatttttt tttttttttgt agtgtttttt tttaagttat attagcgttt 720
aaattttgga gttattgatg ttttcgttat ttttgtagat agaatattat gtcgtaaata 780
atgtttattt gtttattagt attatataaa agtgtttgtt ttttttttatt ttagttatta 840
ttgattattg gtaaaaattt taatgtattt aattttaata ggtaaagaaa ggtttttttta 900
tttagatttt aggtgggggt ttaagtttat atttttgttt tataaaaatt gaatagttag 960
tgtttttgag ttgaatgatt tatttatttc gtataggaga gtttaattat attgtgattt 1020
tgggggagat aatttttata atttattttt ttaaaatgta gaaagtagaa aaagggtaga 1080
aaaatcgtat taaatttgaa taatgtagta tgtttttaga gttagggata tttttttttg 1140
gaaggatttt aagtttggcg tatttgtatt gattgtggtt atggcgtatt taattttttt 1200
aaattagttt ttttgtatcg tatataggaa gatttgaggg tgagtgtaaa ttaggattgt 1260
ttggttagtg gtgtgtgtaa aaatttattg tagaattaag aattattttt ttagtatttt 1320
atatatttta aaaaataggt agatatgttt attgatttta ggttgtttat ggtttttaga 1380
ggtttttttt tattgtaata gtgttgtggg gttttagggg tttagtagtt tttttttttt 1440
aggttatttc ggtgagggag ggagttggga tttttgtatt gcggtaaata agtacgtttg 1500
cgcggtcgta gaggtaggtt ggcgcgtatg tttaggcggg gatgtgtgcg aagtttgtcg 1560
ttgttgtagc gagtttggcg tagagtggag cggtcgtcgg agatgtttga cgtatttgtt 1620
tgaggagcgg ttagtgacgc gatggagcgg gtaaggttag ttgtgtcggt ggtttttttt 1680
aagagatagt ttggggagcg gttatttttta tttattagat attttaagtt tttaggattt 1740
ggagtattga ataaacggaa tttgggtttt aaagtttttt gttttggaga attagattcg 1800
gaatgtttag aggtttgttg ttgtgtcgtt ttgtttcgat ggtattttgt tcgttcgtat 1860
tggggcgcgt tttttattcg tttttaattg tggtgtcgcg ataggtttta ttgcgggtgt 1920

ttgcggtggg aagggcggtg gtgattggga gtatgcgggg taatcgtagt gggtaaggga 1980
tatgggtgga ggtgggtata ttagggtgat tgtagtttcg tgtggcgagg gtacgtgggg 2040
ggattagtgt atagggattt taggcggagg taggtatatt ggagtgattg tggcggggcg 2100
aaggtacgtg aagtgatcgg tatttagggg gtgttgagcg taggtaggtg tataatagtg 2160
attattgcgt ggtggagtag ggtacgtggg gcgattgggg ggggaattcg tcgtagtgat 2220
cgaggcgagg aggttatggt agtggattag ggggatgagt cggtgtgata gtggtggggg 2280
ttgtgttggt ggtcgcggcg cggggagagt tcgtaggaag tggttgggag gtagggggaa 2340
ggcggcgata gtgggtattg gcggcggtgg gtattggtag cgggtaggta tggcggtggt 2400
ggattttggc ggcggtaggt atggcggcgg tgggtatggt atggaggcgg tgggtatggc 2460
ggtcgcgggg tttgtcgttg ttcggagtga ttaagggacg ttgaatgatt gttgtgtaga 2520
ggagggggta ttagaagggg tagtagtata gttcgtagtt tttaattttt ttgtgatttg 2580
cgatggttat ttggtttgtt tattgtggtg gtggtgtttt tttattaaaa ttgcgtaatg 2640
tttatattgt cgtaggggtt gtagaaatgc gtggaatttt tgcgtatttt ttaggaattt 2700
tcgttttaga ggttatggta taagaagatt ggaatgatgt gtagaatttg tatttataga 2760
ttttttgatt gataggttag gtgatgtttg ggggttattt ttttttttatt gtttttttttt 2820
gttgggaatt aggttttgga aggttatgtc gaaataattt gggggtttgt gttgcgttat 2880
tgttattgtg tagttttttt aagatggtcg tcgttgtagc ggtttagatt tgcgtaagcg 2940
ttttagcggg ggggtggtcg tttttttttt gtagagtcgt tagtggggag ggggtaggtg 3000
gtgtgtgttt agtttttgtt gtttcggagt tttagtcgta ttttttttag ggtgtagtgg 3060
taaggagagg aagtcggtta aggtgggcgg ttgttttttt tttaaagtgt tgtttgggaa 3120
aggatgtagg ttgcgtagac gtagtagagg tgatagtcgt tttcgtttgt agtggggcga 3180
gacgtggggt aggggagcga gggtttacgt agtttttgtt tggaaggaat ggtagttttt 3240
tttttttattt ggttgatata gtagttgttt tttttcggtt gtggcgtagt agaggggga 3300
ggagggagat gggttggcgt aggtttcgtt tagtaagttt ggtagttatt ttttatttac 3360
ggtgtagtag tagaggattg gaggggaatg tggttagagg ttaggtattt gtattatttt 3420
cgtttgtgtg ggatggtagt tgtattgggt tattgttttt tagttttgta gtaaattaga 3480
gtggtggcgg tttggaggtt attaaaggtg tagttatttt aagatttgtt gtgttgtttt 3540
agtaagtttt tattaatgat agagatttta aattggtttt ttgatttttt ggaaaatcgt 3600
atttttttat ttttgtagtt tttttttttgg ttgtattgtg cgttaatttt ttatttttaa 3660
attggatttg ttaatttttt ttttttaatt ttttgtagtt tttgattttt attcgatata 3720

```
tttaataaat gttattattt aagatttagt tattttttt aatttttgga tagtttattt    3780
tgatgtattg tatattttt ttaaatgggt tgggggagag gatatcgtga tggtttttt     3840
cgatt                                                               3845
```

<210> 114
<211> 3845
<212> DNA
<213> Artificial Sequence

<220>
<223> chemically treated genomic DNA (Homo sapiens)

<400> 114

```
agtcgggaag agttattacg atgttttttt ttttaattta tttaaggaaa gtatgtagtg      60
tattagaatg gattatttag agattaagaa ggataattga attttaaatg gtgatattta     120
ttaaatgtgt cgaatggaaa ttaaagatta tagggagtta aaaaaaaggg gttaataagt     180
ttagtttgaa aatagagaat tagcgtataa tataattaag agaggaatta taaaaatgaa     240
aaggtacgat tttttaggga gttagaaaat taatttaaaa tttttattat taatggaggt     300
ttgttgaaat agtatagtag gttttggggt ggttgtattt ttgatggttt ttaggtcgtt     360
attattttag tttattgtag agttgaaaag tagtagttta gtgtagttgt tattttatat     420
aggcggaggt ggtataaatg tttggttttt agttatattt ttttttagtt ttttattgtt     480
gtatcgtggg taaggggtgg ttgttaagtt tgttaggcgg agtttgcgtt aatttatttt     540
ttttttttt tttttattgc gttataatcg gaaaggaata gttgttgtat tagttaggtg      600
aaggggaggg ttgttatttt ttttaggtaa aagttgcgta agttttcgtt tttttgtttt     660
acgtttcgtt ttattgtaag cggaggcgat tgttattttt gttgcgtttg cgtaatttgt     720
attttttttt aagtagtatt ttggggaggg ggtagtcgtt tattttggtc ggtttttttt     780
ttttattatt gtattttaaa gagggtacgg ttgaaatttc gaaatagtag aagttgaata     840
tatattattt gttttttttt tattggcggt tttataggga ggaagcggtt attttttcgt     900
tgaagcgttt gcgtagattt aagtcgttgt agcggcggtt attttgaagg agttgtataa     960
tggtagtgac gtaatataga tttttaggtt atttcgatat agttttttaa gatttggttt    1020
ttaatagaga gaaataatga aaaaaaagta atttttaggt attatttggt ttattagtta    1080
aaagatttgt aaatgtaagt tttgtatatt attttagttt ttttatgtta tagtttttaa    1140
agcgagggtt tttaaagggt acgtaaggat tttacgtatt tttgtagttt ttgcggtagt    1200
gtaggtattg cgtagtttta ataaaaaagt attattatta tagtaggtaa attagatgat    1260
tatcgtaggt tataggaaaa ttaaaggttg cggattgtgt tattgttttt tttgatgttt    1320
ttttttttat atagtaatta tttagcgttt tttagttatt tcggatagcg ataggtttcg    1380
cggtcgttat gtttatcgtt tttatgttat gtttatcgtc gttatgttta tcgtcgttaa    1440
agtttattat cgttatgttt attcgttgtt aatgtttatc gtcgttaata tttattgtcg    1500
tcgttttttt tttatttttt agttattttt tacggatttt tttcgcgtcg cgattattaa    1560
tataattttt attattgtta tatcgattta tttttttggt ttattgttat agtttttttcg    1620
tttcggttat tgcgacgaat ttttttttta gtcgttttac gtattttgtt ttattacgta    1680
gtggttatta ttatatattt atttgcgttt aatatttttt aaatatcgat tattttacgt    1740
attttcgttt cgttataatt attttaatat atttattttc gtttaaaatt tttatgtatt    1800
ggttttttta cgtattttcg ttatacggaa ttgtaattat tttgatgtat ttattttttat   1860
ttatgttttt tgtttattgc ggttatttcg tatgttttta gttattatcg ttttttttat    1920
cgtagatatt cgtaatagga tttgtcgcga tattatagtt gggggcggat gggggacgcg    1980
ttttaatgcg agcggatagg atattatcgg ggtagaacgg tataatagta agtttttgaa    2040
tatttcggat ttggtttttt agaataaagg attttagggt ttaaatttcg tttatttagt    2100
attttaagtt ttaaaaattt ggaatatttg atgaataaaa gtggtcgttt tttaggttgt    2160
tttttgagag aagttatcgg tatagttgat tttgttcgtt ttatcgcgtt attgatcgtt    2220
ttttagatag atgcgttagg tattttcggc ggtcgtttta ttttgcgtta gattcgttgt    2280
agtagcggta ggtttcgtat atattttcgt ttgagtatgc gcgttagttt gtttttgcgg    2340
tcgcgtaggc gtgtttgttt gtcgtagtgt aggggtttta gtttttttttt ttatcggaat   2400
gatttggggg gagggggtta ttggattttt agggttttat agtattgttg taatgagagg    2460
gggtttttag aaattataag taatttggga ttaatggata tgtttatttg ttttttaaaa    2520
tgtgtaaaat attaaagaaa taatttttgg ttttataata aatttttgta tatattattg    2580
gttaaataat tttaatttat atttattttt aggttttttt atgtgcggta taaaaaagtt    2640
ggtttggaaa agttaaatgc gttataatta taattaatgt aaatacgtta gatttgaaat    2700
tttttttaaaa gaaaatattt ttaattttaa aagtatgtta tattgtttaa atttggtgcg   2760
atttttttat ttttttttta tttttatat tttgaggggg taaattgtga aaattatttt     2820
```

```
ttttaaaatt ataatataat taaatttttt tatacggaat ggatagatta tttaatttaa    2880
aaatattggt tatttaattt ttgtaaaata aaaatatgaa tttagatttt tatttaagat    2940
ttaagtagga gagttttttt ttgtttatta gaattggata tattaaaatt tttattaata    3000
attaatgata gttggagtgg ggagaaatag gtatttttat ataatattaa tggataagta    3060
aatattattt acgatataat attttatttg tagaaataac ggaagtatta gtaattttaa    3120
aatttagacg ttgatgtagt ttgggaaaag atattgtaga aaagaagaaa ataattttga    3180
aattgtattt gtatagtttt tatatatatt tgtttttttt taattgttta ttatcgaaga    3240
agggagggtg atgtgagtgg aggttgatta aatggttaaa ttttttttta agttttgtaa    3300
gttattgttt agtttttaat gattttatat taaaataggt gcgtagaggt ttattaatgt    3360
tgatttttat cgtaataaaa ttttagaaat tgtgtaatgt tttttataga gaatagatag    3420
gttaattaaa taaacgtata tataaataat aatatgtaat atggttgatg gaaaaaatga    3480
tgtattaggt tgaattatat aaaattgtta atattgaatt gttttgattt ataaaaatat    3540
tgtaattttt aatagtttat tgatatatat ttatataaaa taatatggga agttgatttt    3600
gattacggga gttaatcgaa aaataaaatg taggttgggt atagtggttt acgtttgtat    3660
ttttagtatt ttgggaggtc gaggtgggta gattacgagg ttaggagatc gagattattt    3720
tggttaatat ggtgaaattt atttttataa aaaatagaaa aattagttgg gcgtgatggt    3780
atatgtttgt aattttagtt attcgggagg ttgaggtagg agaattgttt gaacgtggga    3840
ggcgg                                                                 3845
```

<210> 115
<211> 18463
<212> DNA
<213> Artificial Sequence

<220>
<223> chemically treated genomic DNA (Homo sapiens)

<400> 115

```
tttgagtagg cgtgtatttc gtagataggt ttagcgtttt ttgtttgtta ggcgtcgagt      60
tggatggtag gaatatattt tatcgggtaa gatttggtgt ttagtttttt aaggtattag     120
ttattgggtt gaaagagaga aataagttaa aaaacggaaa aattagttag aagtttagta     180
tatgttttaa ggaagttagt aggttgtagt gtgagagaat tagtaaaagc gtatttgatt     240
tagatagggg tgttggaagt agttttttaa aagtggcgtt gtttttgaaa cgtagtttag     300
gatttgtatt tatgtgtagg aagttttata gtacgagagg tttatatagg gataaggcgt     360
taagggggat tgaggggtac gaggggattg agattatttt tggtttgtat cgttgttgtg     420
gttcgggttt tggtgattta ggagtaggga gaatagagag tttcgtttat atttgagata     480
ttagagggag agatgttgta ttagttcgtg tttatgttgt tgataaagat atatttgaga     540
ttgggtaatt tattaaagaa agaggtttaa tggatttaaa tagttttatt tgtgggggaa     600
ggttttataa ttatggtaga aggtaggagg agtaagttaa gttttatatg gatggtagta     660
ggtaaagaga gagggagttt gtgtagggaa attttttttt ataaaattgt tagattttgt     720
gagatttatt tattattacg agaacggtat ggaaaagatt tgtttttatg atttaattat     780
tttttattag gtttttttta tagtacgtgg gaatttaaga tgagatgtgg gtggggacgt     840
agttaaatcg tattagattt ttaaaggtta gatttagtag gtgttgttat agtaaagaga     900
attgtcgggt atagtggttt gatatgggcg ggagttttgg tttgagtttt ggatacggaa     960
gggatttaga ggaggtttcg gttgttcgtt ggtgttgaag gttgagattt ggaaaggtga    1020
tggggggtgt aggtaaggta gaaaggaaat ttttaggttt aggtagaagt attatgttgt    1080
ttatattttt ttgggaagtg gtattcgcgg gggtggttag taagattttt tggttatggg    1140
atttttaaga tgtatttttt tatgttgttt ttttagatat tttggttatt ttgttgatcg    1200
acgtattttt gttgttataa gaaaaagatt agaaatacgt ttttgttttt gtggtatgta    1260
ttttgttttt ttagacgaag ggtcggttgg gtgttgtggt ttacgtttat aattttagta    1320
ttttgggagg tcgaggtggg tagattattt gaggttaaga gttgaagttt agtttggtta    1380
atatggtgaa attttatttt tattaaaaat ataaaaatta gtcgggtatg gtggtttatg    1440
tttgtagttt tagttattta ggaggttgag gtaggataat tatttgaatt taggaggtga    1500
aggttgtagt gagttgagat tgtgttattg ttttttagta agattgcgtt attgtttttt    1560
cgtttgggta atagagcgag attttatttt aaaataaaaa aataaaataa ataaataaaa    1620
aatagacgaa gggttttgtt tgttgtgatt ttgttgggag gttggttgaa attgtttatg    1680
gaaggggtat ttgtgagttt tttaggttta tatagttggg attataggcg ttcgttatta    1740
cgtttggtta atttttttgta tttttagtag agacggggtt ttattatgtt ggttaggttg    1800
gtttttattt tttaatttta agtgattttt tcgttttggt tttttaaagt gatgggatta    1860
tagtcgtgcg ttatcgtgtt tagttgttgg tgtgttttta attagtgtat ttgtttgttg    1920
```

aggttgttgt aaataaagta ttatggattg ggtggtttag attatataaa tttattgttt 1980
tatgtttttg taggttgaaa gtttaagatg aaggtattag tagggttggt ttttttttgag 2040
gtttttttggt ttgtaggtag ttatgtgttt atttgttttt tgtgttttcg agtggttatt 2100
tttttgtgtg tgtttttgtg ttaatttttt tttgttagaa ggatgttagt tagattggat 2160
ttgggttcgt ttatatgatt ttattttgtt ataattattt tttttttttt tttttttttt 2220
tttttttttt gagatagttt tattttgtcg tttaggttgg agtgtgtaat gatataattt 2280
cgatttattg taatttttat ttttcgagtt taagtgattt ttttgtttta gtttttttgag 2340
tagttgggat tataggtatt tgtcgttata ttcggttatt ttttgtattt ttagtagaga 2400
tggggtttta ttatgttggt taggttggtt ttgaattttt gattttaagt gatttgtttg 2460
ttttagtttt ttaaagtgtt gggattatag gagtgagtta ttatattcgt atattaatta 2520
tttttttaaa ggttttattt ttaaagatag ttatattttg aggtgttagg gtttaggatt 2580
ttatatgaat ttgtggggaa tatagtgtag tttattttag ttagttaaaa tgtgtatttt 2640
gttggtttgt ggaagtattg tttttttgaag gatgcgtgat agattagaaa tatcggtggt 2700

tttgggtggt ttgaggtata agatagagat gggtttattt gttgttatat attatatatc 2760
gttgtatttt ttgagttttt tcgtgtgtat gtgtattgtc gattatttaa aaaaaaattt 2820
ttaagttgat gaaattgata tattatcggg atattaaggg gtaagatggt ttatttttgt 2880
tattttagta ttttgggagg tcgagatagg aggattattt gagtttagga ttttaagatt 2940
agtttgggta atatagtgag attttatttt tataaaaaat ataaaaatta gttgggtttg 3000
gcggtacgtg tttataattt tagttattta ggaggttgag gtgggaggat tatttgagtt 3060
taggaggtta aggttgtagg gaataatgat tgtattattg tattatagtt tgggtgatag 3120
agtaagattt ggtttttaag atagtttaaa aaaaaaaaaa aaaaaaaaaa ggtttttagg 3180
atttattgt aatagtattt tttttttttt agatggtaaa atattttcgt tttgaattgt 3240
ttgaaaagaa atgaggagcg tgatttttat ttttagtttt ttttattagg tttttgattt 3300
ttttgttgtt tgggtgttgt ggggtaaagg gtgatttttt taatgttttt tatttatgta 3360
ggatttaaag ttattcgtta tttcgttata aaagtttatc gtgagggaag tggttaacga 3420
ggagaaagcg atgtttttga ttagcgtttt tttgtaaggg tcggagatgt atgaaattta 3480
tacgagtttt aaagaggata ggaacgtttg gatggtttat atttaaaggg ttgtggagag 3540
gtgaggaggg tttgggggtt tttttattta ttgtgttttt ttcgttgatt ggtttatttt 3600
tggtttcgat cgttggttat tagttgtgat tttgaatttt tttattttag tttgggattt 3660
atgacgttat tttagttttt tttgatgatt taatttgttg gacgttgagg ttaacgggtt 3720
tagaaaggta gttgtaattt gggttttttt gtatgcgtgg gttattgtat ttgttatggc 3780
gggtttttttg tattttaggg ttagatttgt tggagtaggg tatggtattt tgtttttttt 3840
ttttgggtcg agtatttttt agggtttaga attagttggt tttttttttt tttttttatt 3900
ttttattttt ttgagataga gttttgtttt gttatttagg ttggagtgta gtgatgtaat 3960
ttcggtttat tgtagttttc gtttttttagg tttaaacgat tttgttgttt tagttttttc 4020
gagtagttgg gattataggc gtgagttatt gcgtttagtt tattttgttt tgttttgttt 4080
tgtttttttag agtttggttt ttgttttatt atttaggttg gagtgcgttg gtataattat 4140
agtttattgt aatttaaatt tttgggttta agttatttta ttttttcggt tttttaagta 4200
gtttgaatta taggtatata ttattatatt tagttagatt ttttattttt gtagagatgg 4260
ggttttattg tgttgtttag gttggttttg aatttttggt tttaagttat ttttttgttt 4320
tggtttttta aagtgttggg attataggcg tgagttatta tgtttgttta tttttttttt 4380
tgattttcgg aggtttttttg gtgttttaat ttggtttaga ttttttttatt tatagtagtt 4440
gtagttttac ggggtttgta gttatttatt tgtatttggt aggttttttg taagttcgtt 4500
gtttttttgga tttttttttt aagttatagg taggtatgta aagttttttt ttggttatgt 4560
tttggcgttt tggggcggga tgaatgttag tggttttttt tttttttttt ttcgtttttt 4620
tgtattacga ataagttaaa ttatttttaa agttagagac ggtcgtggcg gttggttttg 4680
tagaattagc gtttgtgtcg aggttggttt gtttgggaag tatagttatt tattcgattt 4740
tttttttttgt agttgttttg acgaggagga ggggtttttt agtttgttcg aagaggaaag 4800
gaaggtggtc gaggttcgcg ttacgagatt tcgggatttt taaggtgagc gggagatagc 4860
gtttgggtat attttttgtg tggtgagttt tgggtttttt attaggattg gttatagagg 4920
gtgaattgtt ttttaggtga ttcggtgttt tttcgtgggt tgtgttatgt tttgtagttt 4980
tgtgaagtat tagaacgggt tgtgttatga tatagcgatt tttagaggtt atttagtttt 5040
ttgggggtat gtgggagagg gagggaggag agtttagagg atttgagttt tttttagagt 5100
cgattatata gttatatacg ttattattat tttattttcg tgtagttagg agtttaatag 5160
ggtattttag ttttatacga aggaatagta tagggttttt atagtttttt tttagagttt 5220
tttttttttt gtttaattat acgattgaga tgtaatttat atattatagt atttattttt 5280
ttaagcgtgg ggtttagggg tattttagtg tatttttaat tatgtagtta gaattatgtt 5340
aattttagaa tattttatta ttttagaaag aagtttcgtt ttttttaata gttatttttt 5400
attttatgtt ttaattttgg taataggaat ttattttttg tttttgtaga ttggtttgtt 5460
ttggatattt tatataaatg gagttatatc gcgcggtttt ttgggtttgg tttttttcga 5520

```
ggtgtggtat taaggttcgt tcgcgtttag tgagtatttt gttaagaata atttttggtg    5580
ttttgtagtt tgcggtaagt tttttttttt ttttttattg tttgtatttt tttttatttt    5640
attttttttt ttttttttaa atagagtttc gttttgttat taggttggaa tgtagtggcg    5700
tgatttcggt ttattgtaag ttttgtttat taggtttaag agattttttt tttttagttt    5760
ttcgagtagt tgggattata ggtattcgtt attacgtttt attaattttt gtgtttttag    5820
tagagatggg gttttattat gttggttagg atggttttaa tttttcgatt tcgtgatttg    5880
tttgtttcgg tttttaaag tgttgggatt ataggcgtga gttatcgttt tcggttttgt    5940
attatttatt tttaaattgg ataatattta aataataagt ggtttggtat ggtggtttat    6000
gtttgtaatt tttgtatttt gggaggttga ggtagatgga ttattggaag ttaggagttt    6060
gagattagtt tggataatat ggtaaaattt tgtttttata aaaaatttaa aaattaggtt    6120
gggtgcggtg gtttacgttt gtaattttag tattttggga ggttaaagta ggtggattat    6180
ttgagattag gagtttgaga ttagtttggt taagatggta aaatttcgtt tttattaaaa    6240
atataaaaaa ttagttaggt atggtggtgc gtgtttatag ttttagttat tcgggaggtt    6300
gaggtaggag aatcgtttga attcgggagg tggaggttgt agtgagtcga gattgtgtta    6360
ttgtatttta gtttgggcga tagagcgaga ttttttttta aaagaagaag gaaggaaaga    6420
aagaaaggga ggatggaaaa tttagttttt taaggtttta agttggtttt agattaataa    6480
atgggtttga tataattttt atttatattt tattgtgttt gattttattt tatttttttt    6540
agagatagga ttttgttttg ttggttaggt tggagtatag tattgtagtt atagtttatt    6600
gtagttttga atttttgggt ttaagcgatt tttttgtttt attttttagg tatgtattat    6660
tataattagt taatttttta tttgtagaga tagagtttta ttatgttgtt taggttggtt    6720
tcgaatttta ggtcgtaaga gtttttttat tttggttttt ttaagtattg ggatcgtagg    6780
tttgagttat tatagttaat ttgattttta taataagggg gaaaataatt agaggataag    6840
gttatgtttt tttttatttt ttaggaatgt aggatataag gggtagttt attttagggt    6900
agggttagcg gggtttttta tttttaggtt agttttcggg gtttagatga ttttagggaa    6960
ggtcgattcg gttgattgtt atttttatta ttatttttgta gagcggttga gtatgaaaga    7020
ttagttgatc gtatagagtt ttttagagaa atagtagatt tatttggaga tggtcgagat    7080
gggcggtttc gaagatttgt tttagtttcg aggtttattt cgtggagggg atttattcga    7140
gattttgtag ggggagttaa tttttaagtc ggttatgagc gagagtaagt tggttgttta    7200
tattttaagg gtgtagtttt gtcggggtgg gttttttagg gaattttagg ttaggtttac    7260
ggtttattgt ggtcgatacg gtattttgtt taggataggt ttttatgtgg gggggggttta    7320
ggagtagtat tgatcgtttt tcggtgtttg tgagagttag aagggtttta gatataattt    7380
gggttaagtc gtttttttgta gatataaagt attttttttt cgtttaataa tcgttttgat    7440
gtttttttgta tgtagaggtt ttaagtcggg tcgtggggag gatttgaagt tgattaagat    7500
tggtttttta tttattagag gttttttttt tttttgggag gtgggttttg gtgatatttt    7560
tagaaaaata tacgtatatt tttttagtgg gtttttacgt atagttttaa tatagtcgtt    7620
aggtttttgg gttggtcgtt tgagtaggta gttagcgttt agtttgtttt tttttttttt    7680
tttttttttt tttttttttt ttttgagatg gagtttcgtt ttgttattta ggttggagtg    7740
tagtggcgtg atttcggttt attgtaattt ttattttttg ggtttaagta attttgtttt    7800
agtttttttgg gtagttggga ttataggtat ttattattac gtttagttaa tttttgtatt    7860
tttcgtagag atggggtttt attatgttgg ttaggttggt ttcgaatttt tgattttaga    7920
tgatttattt ttttttgttt tttaaggtgt tgggattata ggcgtgagtt atcgcgtttt    7980
gttttaattt gtttttttaa tagtgaggga agtcgattta gtttttgttt tttagagttt    8040
tttcgtagaa taagacggta gagacggggg ttagtttttt tttttttagg tttattgata    8100
ttttttttatt cggttttagt ggttattgtt tttggttttt tggggatatt tttatatttc    8160
gtattttggt ttggaatgtt tttgttttta tttttgtgtt tggttatagt ttgttggttt    8220
tagagggttt tttgtagata gtttttttttt ataaagttat ttttttatat ggttattgtt    8280
atataattag tgtatggtta ttgttatata atatattaag tgttttttgt tggttttggg    8340
tagggttata tttagtattc gttagttttt ggtttagggc ggtttggtgg aaggagttgt    8400
aggagtttgt attcgttttt tttatggttg ttttatagtt ttgttttttt atttagacgt    8460
agattggttt gttttggtgg ttatatttga agtaagggtg tgcgtaggga tagtggggtt    8520
gaaattttac ggtatataga agggggtagg cgattattat tttagtgagt tttttcgttt    8580
attcgggttt cgttgtttta gcgtttcgta tttttttagt atattttcgt agggcgattc    8640
gtgtttttttg tttttttttt tttatagtcg agggtattta gagtttgatt tgtaggtagt    8700
tgggtagcgt taacggttag gcggaagacg gaggtagttt tataggttcg tttaggaggg    8760
ttgagatttt cgcgggttac gattgtataa atagttttat taagagtaag agcggggtcg    8820
tttttttttt tgtttttagg gtcgtttttt aggatgtata ttaatttgta tggggttttt    8880
tagaattttt tttttgttg ttgttgagat agagttttgt tttgttattt aggttggagt    8940
gtagtggcgt aattttagtt ttttgtaatt tttgtttttt gggtttaagt gatttttttcg    9000
ttttagttta ttaagtagtt gggattataa gtatacgtta ttatgttcgg ttattttttg    9060
tatttttagt agaaatgggg ttttattatg ttggttaggt tggtttcgaa tttttgattt    9120
taggtgattc gttcgtttta gttttttaaa gtgttgagat tgtaggtgtg agttatcgta    9180
```

```
tttagtcgat tttttggaat tttttgaaat tttttagata tcgttggttg tgttgtgata    9240
gtagtattta gattagtttg ttttttttta gtaggcgttt gtgttgtaga cgttattatt    9300
cgggtttagt ttgggttttt tatttgggta ggggttgggg gtggattggg aagttttttt    9360
tttttacgg gatggtagta ttttatgtt tttggtttgg ggtttttaga tggtagtttt    9420
aagaagaaag ttagtagtat tgattttagg tttcgagatt ggcgaggttt tttaaatagt    9480
tcggatttga agtttagtga tagtgatatt tttgggagtt ttgaggaatc gtcgtaggtg    9540
gtacgtggat atttatttgt tcggtatagt ttgagtcgtt ataaggattt atgtgtgtga    9600
gtagtttttg ttggggttgt ttagttttag ttttattagt atcgataagt attgtttgtt    9660
ttgtggtgaa taatagtatt ttagtttaga ttaatgtatt ggtaggtgat tttttttgt    9720
agtttttgg gaaaagtaaa gaggttttta agaggaaggt tggggagggg ataggtttag    9780
ggtatttatt atggttttat taataggcga ggaatttttt atagggatta ttagttttta    9840
gtcggggtag gtgtggtggt gtttattaga tgttagatgt tttcggttat tttttgttt    9900
ttgaattaga aggttaggtg tttattcggt tttgtttagg ttaggggggtt agaggttaag    9960
gttagttgtt tttgattagt tttgaggttt ggatttgttt ttgtggttag agttggtacg   10020
taggttttat agttaagttt attttatttt ttaggaggtt ttagtttttc gtttttaaa   10080
tatttagatt tgtataagtt agttgattag tagggttgt gtttttagtt atgattcggt   10140
tttgggtttt ttttgttatt tatattaagg gatgtatttt taataataag taggggtcgg   10200
gtgtagtgtt ttatatttat aattttagtg ttttgggaga tcgaggtggg aggattattt   10260
tttttttttt tttttggaga taaggtttta ttttgttatt taggtttgag ggtagtggcg   10320
tgattatagt ttatatagtt tttaattttt aggtttaagt aattttttta ttttagtttt   10380
ttaagtagtt gaaattatag gtgcgcgttt ttatatttga ttaatttttt aaatgtttgg   10440
tagatatagg gttttattat gttgtttagg ttggttttta atttttgggt ttaagtaatt   10500
tttttgtttg ggttttttaa agtgttggga ttataggtaa ggtcgttata tttggttttg   10560
ggaggattat ttgaggttat gagtttgaga ttagtttggg taatatagta agattatatg   10620
tcgataaaat atttttaaaa tagttgattt tttggttggg tgcggtggtt tatgtttgta   10680
attttagtat tttaggaggt cgattcgggt gattatttga gtttagggtt tgagattagt   10740
ttggttaata tggtgaaatt ttgtttttat taaaaatata aaaattagtt ggacgtggtg   10800
gtggatattt gtaattttag ttatttagga ggttaaggtg ggagaatcgt ttgaatttag   10860
gaggtggagg ttgtagtgag tcgagattgt attattgtat tttagtttgg gcgattaagt   10920
aagattttgt tttaaaaaag ttaaaaatag ttgttgggtg tggtggttta cgtttgtaat   10980
tttagtattt tgggaagtta aggtaggtgg attatttgag gttaggaatt cgagattagt   11040
ttgattaata tggtgaaatt tcgtttttat taaatatata aaattagtta ggcgtggtgg   11100
tatattcgag taattttagt tatttgggag gttgaggtag gagaattatt tgaattagga   11160
aggtggtggt tttagtgagt tgagatcgtg ttattgtatt ttagtttggg taacgagtaa   11220
aattttattt taaaaaaaaa aaaaaaaaaa attaaaaata gttgatttg gtggtgtata   11280
tttgtagttt tagttattta ggaggtcgag gtgggaggat taggttatta tattttagag   11340
taaggttttg ttttaaaaaa aaaaaaaaga agttaggtat ggtggtttat gtttgtaatt   11400
ttagtatttt gggaggtcga ggcgggcgga ttatttgagg ttaggagttt gtaattagtt   11460
tggttaatat ggtgaaattt cgtttttatt aaaaatatat aaaattagtt aggcgtggag   11520
gtttgagttt ataattttaa ttattcggga ggttgaggta ggagaattgt ttgaattcgg   11580
gagttggagg gtgtagtgag ttaagatcgc gttattgtat tttagtttgg gtaataagag   11640
tagaatttcg ttttaaaaaa aaaaaaaaaa aaaaaagtta aaaaaaaaga agaagaagaa   11700
gaaggtttga gtttagtcgg atgggttttg agttattttt tttggtttga cggtgttttt   11760
tttttaggtg gaggcgttag gtacggaatt cgattttcgt ttgtttatcg ttttggagtc   11820
ggaggtaggc gttcgcgggt ttttattttt ttagggtttt gtgtacgcgc gtgtggtttt   11880
agttcgataa ttagtattaa tttttttttt tttttcgtag tttgtttagc ggatttagat   11940
attgttttag ttgtttttga atttttaggt ataggggcgg ggtggggtcg gttacgcgtg   12000
tttttttttg gttggttggg gcgtaggtgc ggtttttatt cgtttggttt tggtttttcg   12060
taggcggtaa tcgtttatta ggatagttat gtggagacgt agcgggttgt tatttaggag   12120
cgggagaagt agtttcggtt gtagtcgacg cgtgggaatt tgttgttgga gtaggagcgg   12180
taacgtaatt tcgagaagta gcgggaggag cgcgcggttt tggagaagtt gtagagttag   12240
ttgcggtacg agtagtagcg ttgggagcgc gagcgttagt ggtagtatta ggagttggag   12300
cgtgcgggcg cgcggttgta ggagcgcgag ggcgaggcgc ggtagttacg cgagcggttg   12360
gagtaggagc gggtcgagtt ggagcgttag cgttaggttt attagtacga tttggagcgg   12420
ttgcgcgagg tttagcgtgt cgtggagcgc gagcgggagc gtttggagtt gttgcgtcgt   12480
tttaagaagt agaatatcgc gttaggcgcg ttgtcgttcg atatattggt cgaggtgagc   12540
gcgtagtagt tagtgtgcgt aggttggggg tgatcggttt gtacgtgtat ttgtacgagt   12600
gtatgtaggt gatagggttc gcgggtgtat gcgtgtagga gtgtaagagt aaacggtata   12660
agatattttt gtattaatag tttatgtcgt agtatagatt tttttcggag ttggttaaat   12720
gtttaataga acgttgttgt ttaaaatatg ttataaatgt tagaaatttt ttaaaaaaaa   12780
aatgtatttt tgtaatttta gtattttggg tggtggaggt aggcggattg tttgagggta   12840
```

```
ggagttttag gttagtttgg gtaatatggc gaaattttat ttttataaaa aaatataaaa   12900
atgagttagg tatggtggta tgtatttgtg gttttcgtta tttggaatgt tgaggtggga   12960
ggattatttg agttcgggag aatgaggttg ttatgagtcg tgattacgtt attgtatttt   13020
agtttgggta atagagtgag atatgagtcg tgattatgtt attgtatttt agtttgggta   13080
atagagtgag atttcgtttt aaaaaaaaaa aaaaaatgaa gttgggtgtt atggtgtgta   13140
ttttagttat tcgggaggtt gaggtcggag gattgtttga ggttaggtat ttgagattag   13200
tttgggttac gtagtgagat tttattttta aaaaaaaaat gtaaaaatta gtagggcgtg   13260
gtggtatata tttgtaattt aaattatttg ggaggttgag gtagggggat tatttgagtt   13320
taagaggttt aagagttatg attgtgttat tgtattttag tttgggtgat agagtaagat   13380
cgtgtttttg aaaataaatt tataaattat gggttttgtg aatagatgtg gatacggatg   13440
tgtgagatgt ttgtatatga gtgtgtgata gtatgggtgt tattgagtgt tattataagt   13500
ttgtatattt agtaggggtt ggggacgggt aatgattagt agttttttta ggttgtattt   13560
ggttttgttt agaataggag aggtcgaggg tttttttgtgt acgattttcg ggggtttttt   13620
agaggtcgta tagtaggaat tttatattgg atgtatttgt tgttgttttt ttaggtttag   13680
tttttaagtt attttttttag ttttaacggg gaagggttgg agggttttcg tgtgagtatg   13740
ttgttattcg gcgtggggtt agagtacgta gagcgtttcg aggtggttcg tcgggatagc   13800
gtttttatcg agaatcggtt ggttaagagc gatgtgttta tttagttgtt tagcgttatt   13860
aattagtttt agaggtaggc ggtcgtgtag tagtagattt ttattaagtt ggcggttttt   13920
attaagggtg gtaaggataa gggcggtaag agtaggggtt tttagcgttg ggagagttta   13980
ggtgagtcgg ttttattttt tcgtttgggt ttggaaggtg taattggtta ggttttagcg   14040
gttcgttggg agttaggaaa ttcgggatat ttgtgttatt ttttggagag gaaaattaga   14100
aggtatagtt ggtatcgtgg ttgaggttat tttatatagg gtcgtgtttg gtttttaggt   14160
ttaaaattga gatgatttat tttgtatttt ttttatttta atttttaaaa tagtgggatg   14220
gggattagga gttttagaaa gggatacggg taagaaggta gagatttttg gttacgtggg   14280
attagtatag tcgggatagg gataggtgta ggcgtttttg tttattgttt tagttttata   14340
gagtcgtttt atagagtggg atacggagtt tagagtagtt ttagtgtttt tacggaggat   14400
gtttaggagt tttttttgggg gatatttttt tgggggataa ttagggatgt tttgggatgt   14460
tttttgaaga ttaagggtgt gtttcgtggt tattagggtt tgtgttattt ttataggagg   14520
tttagttttt tttttagatg tcgggtgatt ttgtaggttt tttcgtaggg aggttggggt   14580
cgtttttttt gaatttgggg tttagtattt gagtagttta ggttacggga tttaggtcgt   14640
tcgtgggaag tgttaggtgg gggtggttag gttttttttgt tttcggaggt tgttttggcg   14700
ggtggggata gttggttagt ttggagttgg tttaaatatt tttttttttt tagcgttttt   14760
cgatttgaag tagtagttgt tgtttaataa gtttatgggg aaagatgaga gtattttacg   14820
gaatcgtcgt tcgttgagtt ttattttgtt cggtagatat agttttgcgt ttttattagg   14880
tgagttttta tttttttgata tgagtttagt tttagggtag cggggttgcg gtattattcg   14940
gcgattgttt agtttgaatt ttttttttgt tttagatttt ggttttttcg tttcgagttt   15000
atcgttagtt gatagttttt tcgagggttt tttttttaag gtcgggggta tagtttttttt   15060
gttcgggttt ttagtttttt cgttattgtc ggttatatta tttagcgtta aggaggacgt   15120
tagtaaagaa gacgttattt ttttttaaaa gggtcgtgat ttaaggtgta aggttttttt   15180
ttgttttgtt tatttttttt gtttttttggg gatttttatg gggttattat gtttatttag   15240
ttgtttttttt ttttttttta gtaaattatt gatgatcgtt tggtaggggt tagtttgtcg   15300
gtgttttggg ttttgtagtt gtttttgtag ggttagcggt ggtgtcgggg ttattttttg   15360
aattttttttt tttttttttt aaaaaggaaa gtttttaatg gaaagttgag ttagaattaa   15420
attagggagt tgtttgaaat tatagtattt tattcgggtg gcggggagat taatttcgag   15480
ttgtttttttc gaggtagtga ggaacggtgt cggttttgta cggagttgag gataggatag   15540
attttgtttt gagaaggagt tgtcggtcgg ggttacgttt tatagtcgtc gcgcgatagt   15600
ggagttaagg gttagggtat taggaggggt taggtggcgt cggtagtatt tgtttttaga   15660
attaggtaga atttattttt taaatagagt tttacgtagg tttattatga attttagggt   15720
tagggaatga gttaggtacg ggggtatggg tagagagggt tacggggtag ggtttattga   15780
gggaatatta gtggtttttt agttaggttt tgtgggtttg gaagtttatc gtgaaagggg   15840
ttgattttttg ttttttttta tttggtattg gttttgaaat tagttgtttt ttaaattttg   15900
tttttttaagg gtaatcgttg ttgtttatac gtttagtttg tttgggggag cgggttttta   15960
gttttagtta gggcgggtat atattttggg tatagggttt ttagttttcg ggaaatgagt   16020
ttttagggtt ggcgttttat tttttaggtg ggggttggta tattatagat tgtcgagagc   16080
gttatgtttt agggtatgta gaggatgtat ttagagacgt tgtagtaagt ggataagtgg   16140
tcgttgtgcg ggtttttcgt ttgtagtgag ttgttgtagt ttacggttcg ttttttggag   16200
gggtggagga aggaggtgtt gggtagtatt aaaggtgttg ggatatttta gggtggtgag   16260
atttatttac gatttagttt cggtggagaa agggtttatg ttaagttttg ttttgtattt   16320
taagtattgg tggtaggatt gggttttggt tgatcgtttt tgtttttagt ggggtatatg   16380
tgagtttttg ttagggttaa gtttttttttt cgaatttagg gttttgggaa ttgtagattt   16440
cggggggatt tagttttttt tttattgtgt tggtagaggt atttttgtga cgttgaatat   16500
```

agtgaatagg gatattttcg ttattcgggg atagatgggt ataagggagg ggaaatttta 16560
ttaggaagtg ttttttggg tagaggcgtt tattgggtgt tgtgggttta ggagggggcg 16620
gggtaggagt tggtgttaat cgggaattag agttttatag ttatatagtt tattggtgat 16680
aaggttttga gaatatagtg gttaggtgtt tttaggtttt tggtttttc gacgatttta 16740
attttgttta gttcggtttt tggttattag cgacgttgtt cgtttttcgt tagattttat 16800
gtgtgttatg tttattatta gtgttttgta tttttgtatt gagtatcgga gtattttata 16860
gaagttgatt gtatattttt gttttgttgt gaagagaata tttttagatt ttggtatttt 16920
tttgagtcgg cgtgtgtcgg tttagttttt cgagatgtta taattttttg gatgggggag 16980
aagtttaagg aatttttgtt cggttacgcg gtgggaattt cgcgttttcg ttatgtggta 17040
gaggggtttt agtcgtgtta ggtatcgggc ggtagcgtcg atagtttttt tttcgttagt 17100
gtttttcggt tatttttggg ttggagttcg attttatttg taaagttgat agtcgagtaa 17160
atgtttttat tttcgtggga tttgtatacg tttttgttag ttgtggttat gattttagtt 17220
atttgttaat tatttttata atgattataa tattttttta ttgcgggata tttttgattc 17280
gttttagaat ttaagatttg attttagtaa taaacgtgtt cgagatgagc ggtgattggc 17340
gtgtacgttt ttggaggttt tatttgagga ttttttttttg tttaaacgtt gttgtaggag 17400
tagtaggttt tgttgggggt tttttatggt ttttgttttt tagttcgttt atttgttttt 17460
cgtgttttgt ttaacgtttt gaagattttt ttaggtttag tttttaggag ttgttggggg 17520
ttttgttttt gtttttgttt ttgttttttt ggtttgtttg tttttttgag atagagtttt 17580
gttttgtcgt ttaggttgga gattagtggt aggtttttag tttattgtaa tttttgtttt 17640
tcgggtttaa gtattttttt tgttttagtt ttttgagtag ttgggattat aggtgtgtat 17700
tattacgttt agttaatttt tgtatttttt tagtagatat ggggtttcgt tatgttggcg 17760
aggttggttt cgaattttttg attttaagtg atttgttcgt tttagttttt taaagtgttg 17820
ggattacggg tatagggtag gagtggggcg tggaggtgtt tgtggagggt tttgtttatc 17880
gggtatattt taggttttgg tggcgttttt acgaaaattt ttttgttttt aatttataat 17940
aatcgtaaag gttggaattt ttattttttt tatttggggt gagaggtagt ttggttatga 18000
gtatgaaatt attattttta tttggtttga gtttcggttt agtgaggcga ggattttgtt 18060

gttttaatag agggtttttt attataaggg gattaggtgt aaagtcgatt attagtttat 18120
taggaagcgt aaaaagggaa tttttaggta gtattgttta ttaggaagat gatgtaggtt 18180
acgtgtggaa ttttttattt ttagtagtta tattaaaaaa gtaaaaggag ggtcgggtat 18240
agtggtttat ttttgtaatt ttagtatttt ggggaggcgg gggttgaggt aggaggatag 18300
tttgagttta ggagtttaag attagtttgg gtaatatagt gaaatttttt tatagaaaaa 18360
ttaaaaaatt agttaggttg gtcgggcgta gtggtttacg cgtgtaattt tagtattttg 18420
ggaagttaag gtgggtagat tacgaggtta ggagatcgag att 18463

<210> 116
<211> 18463
<212> DNA
<213> Artificial Sequence

<220>
<223> chemically treated genomic DNA (Homo sapiens)

<400> 116

ggtttcgatt ttttgatttc gtaatttgtt tattttggtt ttttaaagtg ttgggattat 60
acgcgtgagt tattgcgttc ggttaatttg gttaattttt taattttttt gtgaaggggt 120
tttattatgt tgtttaggtt ggttttgaat ttttgggttt aagttatttt tttgttttag 180
ttttcgtttt tttaaaatgt tgggattata gggatgagtt attgtgttcg gttttttttt 240
tatttttttta atatggttat taaaaatgaa aaattttata cgtggtttgt attatttttt 300
tggtggatag tgttgtttga gagttttttt tttgcgtttt ttagtgagtt agtgatcgat 360
tttatattta gttttttttgt gatgggaaat tttttgttga aatagtaggg ttttcgtttt 420
attggatcgg gatttagatt aggtggaaat gatgatttta tatttatggt taagttgttt 480
tttattttag atggaggaag tgaaagtttt agtttttgcg gttattgtgg attaaaagta 540
aaaagatttt cgtggaggcg ttattaaagt ttgaagtgta ttcggtgggt aggatttttt 600
ataggtattt ttacgtttta tttttgtttt gtgttcgtaa ttttagtatt ttgggaggtt 660
gaggcgggta gattatttga ggttaggagt tcgagattag tttcgttaat atggcgaaat 720
tttatgttta ttaaaaaaat ataaaaatta gttgggcgtg gtggtgtata tttgtaattt 780
tagttattta ggaggttgag gtaggagaag tgtttgaatt cgggaggtag aggttgtagt 840
gagttgagaa tttgttattg gtttttagtt tgggcgatag agtaagattt tgttttaaaa 900

```
aaataaataa attaaaaaaa tagaaataaa aataaaaata aaatttttaa taatttttgg     960
ggattggatt tgaaggggtt tttagagcgt taagtaggat acggagggta ggtgagcgag    1020
ttgaggggta ggagttatgg ggagttttta atagaatttg ttatttttgt agtaacgttt    1080
gggtagaaga aaatttttaa ataaaatttt taagaacgtg tacgttagtt atcgtttatt    1140
tcggatacgt ttattgttag aattaggttt taagttttaa agcgggttag aaatatttcg    1200
tagtgaggaa atgttgtaat tattgtaaaa ataattagta ggtgattaag attatgatta    1260
taattgataa agacgtgtgt agattttacg aaaataggaa tatttgttcg attgttaatt    1320
ttataaataa aatcgggttt taatttagga gtggtcgagg ggtattggcg agggaagggt    1380
tgtcggcgtt gtcgttcgat gtttagtacg attgagattt ttttgttata tggcggggac    1440
gcggggtttt tatcgcgtgg tcgagtagaa attttttgaa tttttttttt atttaaggaa    1500
ttgtggtatt tcggagggtt ggatcggtat acgtcggttt aggagggtgt tagggtttgg    1560
gaatgttttt tttataatag aataggaatg tatagttagt ttttgtaaag tgtttcgata    1620
tttagtataa aaatataaaa tattgatgat aaatatggta tatatgggat ttgacggggg    1680
gcggatagcg tcgttgatgg ttagggatcg ggttgggtag agttgaggtc gtcggagggg    1740
ttaggagttt ggggatattt ggttattgtg tttttaggat tttgttatta atgggttgta    1800
tggttgtggg gttttggttt tcggttggta ttagtttttg tttcgttttt ttttgagttt    1860
atagtattta gtgggcgttt ttgtttaggg gagtattttt tgatggagtt tttttttttt    1920
tgtgtttatt tgttttcgag tggcgggaat gtttttgttt attgtattta gcgttatagg    1980
agtgtttttg ttagtatagt gggagaaggg ttgaattttt tcgggatttg tagtttttag    2040
gattttgggt tcgggagaag gatttggttt tggtaggggt ttatatgtat tttattggga    2100
ataaggacga ttagttagga tttagtttta ttattaatgt ttggggtgta gaataaggtt    2160
tgatatgggt ttttttttta tcggagttgg atcgtggatg gattttatta ttttgggatg    2220
ttttagtatt tttgatgttg tttaatattt ttttttttta tttttttagg gaacggatcg    2280
taagttgtaa tagtttatta taagcgaggg gttcgtatag cggttatttg tttatttgtt    2340
gtaacgtttt taggtgtatt ttttgtatgt tttgggatat ggcgttttcg atagtttgtg    2400
atgtgttagt ttttatttgg aaggtgggac gttagttttg ggagtttatt tttcgggggt    2460
tgaggatttt gtgtttaggg tgtgtattcg ttttggttga agttagaggt tcgttttttt    2520
agataggttg agcgtgtgaa tagtaacggt tgtttttggg aagtagagtt tgggaaatag    2580
ttggttttaa aattaatgtt aagtaaaaag gggtaaaggt tagttttttt tacgatgggt    2640
ttttaaattt atagaatttg attggagggt tattgatgtt tttttagtgg gttttgtttc    2700
gtggtttttt ttgtttatgt tttcgtgttt ggtttatttt ttgattttga ggtttatggt    2760
gaatttgcgt ggggttttgt ttgggaagtg gattttgttt gattttgggg atagatgttg    2820
tcgacgttat ttggtttttt ttggtgtttt aatttttggt tttattgtcg cgcggcggtt    2880
gtggagcgtg gtttcggtcg gtagtttttt tttaaagtaa ggtttgtttt gtttttagtt    2940
tcgtgtagag tcggtatcgt tttttattgt ttcggaaaaa tagttcggag ttgatttttt    3000
cgttattcgg atggggtgtt atgattttag atagtttttt ggtttagttt tggtttaatt    3060
ttttattaaa aatttttttt tttgaaaaaa aaaaaaagag atttagaaag tgatttcggt    3120
attatcgtta attttataga aatagttgta aggtttagag tatcgatagg ttggtttttg    3180
ttaggcggtt attagtggtt tgttagggaa gaggagggga tagttgggtg ggtatggtga    3240
ttttatgggg gtttttagag agtaggaagg gtgggtaggg tagggagggg ttttgtattt    3300
tgagttacgg tttttttaga agaagatgac gttttttttg ttggcgtttt ttttggcgtt    3360
gagtggtgtg gtcggtagtg gcgagggagt tggggggttcg ggtaggaggg ttgtgtttc    3420
ggttttgaga gagaagtttt cggaggggtt gttagttggc ggtgggttcg gggcggggaa    3480
gttagggttt ggaatagaga gagggtttag attgagtagt cgtcgggtgg tgtcgtagtt    3540
tcgttgtttt gggattgggt ttatgttagg gggtgggggt ttatttggtg ggggcgtagg    3600
attgtgtttg tcgggtagga tagggtttag cgagcggcgg tttcgtgagg tgtttttatt    3660
ttttttatg agtttgttga gtagtagttg ttgttttagg tcgaaggacg ttggaagaga    3720
gaaggtattt gggttagttt taggttggtt agttgttttt attcgttagg gtagttttcg    3780
agagtagagg ggtttggtta tttttatttg atattttta cggcggttt ggatttcgtg    3840
atttgagttg tttagatgtt aagttttaga tttagggaga acggttttaa tttttttgcg    3900
gaaggatttg tagagttatt cggtatttgg agaggaggtt gagtttttttg tggaaatagt    3960
ataggtttta gtgattacgg gatatatttt taatttttag agaatatttt agaatatttt    4020
tggttgtttt ttagggaggt gtttttttaga ggagtttttg gatatttttc gtggaaatat    4080
tggggttgtt ttgggtttcg tgttttattt tgtggaacgg ttttgtggaa ttgggataat    4140
gagtagaggc gtttgtattt gtttttgttt cggttgtgtt gattttacgt ggttagggat    4200
ttttgttttt ttgttcgtgt ttttttttaa ggtttttggt ttttatttta ttgttttgag    4260
ggttgaggtg gagaagatgt agggtggatt attttagttt tgggtttgga gattaaatac    4320
ggttttgtgt ggggtggttt tagttacgat gttagttgtg ttttttggtt tttttttta    4380
gaggatggta taggtgtttc gaatttttttg gtttttagcg agtcgttaga gtttgattag    4440
ttgtattttt taggtttagg cgaaggggtg gggtcggttt atttgagttt ttttagcgtt    4500
gagagttttt gtttttgtcg tttttgtttt tgttattttt ggtggaggtc gttagtttgg    4560
```

```
tggggatttg ttgttgtacg gtcgtttgtt tttggaattg gttggtggcg ttgagtagtt    4620
ggatgggtat atcgtttttg gttagtcggt tttcggtggg ggcgttgttt cggcgagtta    4680
tttcggggcg ttttgcgtat tttggtttta cgtcggatgg tagtatgttt atacgagggt    4740
tttttagttt tttttcgttg aagttgggag ggtggtttgg gggttgggtt tgaggggata    4800
atagtagata tatttaatgt gaggtttttg ttgtgcggtt tttggagagt tttcgagggt    4860
cgtgtatagg agattttcga tttttttttgt tttggataga gttaggtgta gtttgaagga    4920
gttgttggtt attgttcgtt tttaattttt gttaaatgtg tagatttgtg gtgatattta    4980
gtggtattta tattgttata tatttatata taaatatttt atatattcgt gtttatattt    5040
gtttatagaa tttatggttt atagatttgt ttttagagat acggttttgt tttgttattt    5100
aggttagaat gtagtggtat aattatagtt tttgagtttt ttgggtttaa atgatttttt    5160
tgttttagtt ttttaagtag tttggattat aggtatgtgt tattacgttt tgttaatttt    5220
tgtatttttt ttttagagat gaggttttat tacgtggttt aggttggttt taaatattta    5280
gttttaagta atttttcggt tttagttttt cgagtagtta gggtgtatat tatggtattt    5340
agttttattt tttttttttt tttgagacgg ggttttattt tgttgtttag gttggagtgt    5400
agtggtatga ttacggttta tgttttattt tgttgtttag gttggagtgt agtggcgtga    5460
ttacggttta tggtagtttt attttttcgg gtttaagtga ttttttttatt ttagtatttt    5520
aagtagcggg gattataggt gtatgttatt atgtttagtt tatttttgta ttttttttgta    5580
gagatggagt ttcgttatgt tatttaggtt ggtttggaat ttttgttttt aagtaattcg    5640
tttgttttta ttatttaaag tgttgggatt ataggagtgt attttttttt taagaagttt    5700
ttaatattta tggtatgttt tgagtaatag cgttttgtta agtatttagt tagtttcgag    5760
aaaggtttgt attgcggtat gagttgttaa tgtaagggta ttttgtgtcg tttgttttta    5820
tatttttgta cgtatgtatt cgcgaatttt gttatttgta tgtattcgtg taaatgtacg    5880
tgtaaatcgg ttatttttaa tttgcgtata ttggttgttg cgcgtttatt tcggttagtg    5940
tgtcgggcgg tagcgcgttt ggcgcggtgt tttgtttttt gaggcggcgt agtagtttta    6000
ggcgttttcg ttcgcgtttt acggtacgtt gggtttcgcg tagtcgtttt aggtcgtgtt    6060
ggtaggtttg gcgttggcgt tttagttcgg ttcgtttttg ttttagtcgt tcgcgtagtt    6120
gtcgcgtttc gttttcgcgt ttttgtagtc gcgcgttcgt acgttttagt ttttggtgtt    6180
gttattggcg ttcgcgtttt tagcgttgtt gttcgtgtcg tagttggttt tgtagttttt    6240
ttagggtcgc gcgttttttt cgttgttttt cgaagttgcg ttgtcgtttt tgttttagta    6300
gtaggttttt acgcgtcgat tgtagtcgga attgtttttt tcgtttttgg atggtagttc    6360
gttgcgtttt tatatagttg ttttggtggg cgattatcgt ttgcggaggg ttagggttag    6420
gcgagtgaga gtcgtatttg cgttttagtt aattaggaaa gggtacgcgt ggtcggtttt    6480
atttcgtttt tgtatttgaa ggtttaggag tagttgggat agtgtttgga ttcgttggat    6540
aagttgcgga gaggagaggg agattgatgt tagttatcgg gttgaggtta tacgcgcgtg    6600
tataaggttt tggggagatg gagattcgcg ggcgtttatt ttcgatttta ggacggtggg    6660
tagacgggga tcggatttcg tgtttggcgt ttttatttgg gaagaggata tcgttaaatt    6720
agagagagtg gtttaagatt tattcgatta gatttaagtt tttttttttt tttttttttt    6780
ttttaatttt tttttttttt tttttttttg agacggaatt ttgtttttgt tgtttaagtt    6840
ggagtgtagt ggcgcgattt tggtttattg tatttttttag ttttcgggtt taagtaattt    6900
ttttgtttta atttttcgag taattgggat tataggttta agtttttacg tttggttaat    6960
tttgtgtatt tttagtagaa acggagtttt attatgttgg ttaggttggt tgtaaatttt    7020
tgattttagg tgattcgttc gtttcggttt tttaaagtgt tgggattata ggtatgagtt    7080
attatgtttg gttttttttt tttttttttg agatagggtt ttgttttaga gtgtagtggt    7140
ttgatttttt tatttcggtt ttttgagtag ttgggattat aggtgtgtat tattaggatt    7200
agttattttt aatttttttt tttttttttt ttgagatgga gttttgttcg ttgtttaggt    7260
tggagtgtaa tggtacgatt ttagtttatt gaaattatta tttttttggt ttaagtgatt    7320
tttttgtttt agttttttaa atagttggga ttattcgaat gtgttattac gtttggttaa    7380
ttttgtatat ttggtagagg cggggtttta ttatgttggt taggttggtt tcgaattttt    7440
gattttaggt gatttatttg ttttggtttt ttaaagtgtt gggattatag gcgtgagtta    7500
ttatatttag tagttatttt taattttttt gaaataaagt tttgtttggt cgtttaggtt    7560
agagtgtagt ggtgtaattt cggtttattg taatttttat tttttaggtt taagcgattt    7620
ttttatttta gtttttgag tagttgggat tataggtatt tattattacg tttagttaat    7680
ttttatattt ttagtagaga tagggtttta ttatgttggt taggttggtt ttaaattttg    7740
gatttaagtg attattcgag tcggtttttt gaagtgttgg gattataggt atgagttatc    7800
gtatttagtt aaaggattag ttattttaaa aatattttgt cgatatgtgg ttttgttatg    7860
ttgtttaggt tggttttaaa tttatggttt taagtgattt ttttaaggtt aggtatggcg    7920
gttttgttta taattttaat attttgggag gtttaggtag gaggattgtt tgagtttagg    7980
agttggaaat tagtttgggt aatatagtga gattttgtat ttattaaata tttaaaaaat    8040
tagttaggtg tgggggcgcg tatttgtagt tttagttatt tgggaggttg aggtgggagg    8100
attgtttgag tttaggagtt agaggttgtg tgagttgtga ttacgttatt gtttttaagt    8160
ttgggtgata gagtgagatt ttgtttttaa aaaaaaaaaa aaaaagtgat tttttttattt    8220
```

```
cggtttttta aaatattggg attatgggtg tgaggtattg tattcggttt ttgtttatta    8280
ttgaaaatat attttttagt gtggataata ggaaggattt aggatcgggt tatgattggg    8340
aatataggtt ttgttggtta gttgatttgt ataaatttgg gtgtttggaa aacgaggggt    8400
tggggttttt tgggaggtag agtgggtttg gttatgaggt ttgcgtgtta gttttggtta    8460
taggggtaag tttaagtttt agaattgatt agaggtaatt ggttttggtt tttgattttt    8520
tgatttgggt agggtcgagt aggtatttgg ttttttgatt taaaggtaaa ggaatagtcg    8580
gggatatttg gtatttggta ggtattatta tatttgtttc ggttgaaagt tggtggtttt    8640
tgtagggggt ttttcgtttg ttgatgaagt tatggtgagt gttttgagtt tgtttttttt    8700
ttagtttttt ttttggggat ttttttgttt tttttaggag gttgtaggaa gaaattattt    8760
gttagtgtat taatttgggt tgagatattg ttatttatta taaaataaat aatgtttgtc    8820
gatgttaata agattaaaat tggataattt tagtagaagt tgtttatata tatgaatttt    8880
tatgacgatt tagattgtat cgagtaaatg gatatttacg tattatttgc ggcgattttt    8940
tagagttttt aggaatgtta ttgttattga gttttaagtt cgggttgttt ggggggtttc    9000
gttagtttcg gggtttgggg ttagtgttgt tgattttttt tttgaaattg ttatttgaga    9060
attttaagtt aggggtatga ggatgttgtt atttcgtggg gagggggaag ttttttagtt    9120
tatttttagt ttttgtttag gtaggaaatt taggttaggt tcgaatggtg gcgtttgtag    9180
tatagacgtt tgttggagag gggtaggttg gtttgggtgt tgttgttata atataattaa    9240
cgatgtttgg agggttttag aaggttttag aaaatcggtt gggtgcggtg gtttatattt    9300
gtaattttag tattttggga ggttgaggcg ggcggattat ttgaagttag gagttcgaga    9360
ttagtttggt taatatggtg aaatttttatt tttattaaaa atataaaaag tagtcgggta    9420
tggtggcgtg tgtttatagt tttagttatt tggtaggttg aggcgggaga attatttgaa    9480
tttaggaggt agaggttgta gagagttgag attgcgttat tgtattttag tttgggtgat    9540
agagtaagat tttgttttaa taataataaa aaagaaagtt ttaggaaatt ttatataagt    9600
taatgtgtat tttgaggggc ggttttggag gtaggagggg agacggtttc gtttttattt    9660
ttggtggggt tgtttgtgta gtcgtagttc gcgaaggttt tagttttttt gggcgggttt    9720
gtggagttgt tttcgttttt cgtttggtcg ttggcgttgt ttagttgttt gtagattagg    9780
ttttggatgt tttcgattgt ggaaggaagg ggataggagg tacgggtcgt tttgcggaag    9840
tgtgttgggg gaatacggag cgttggggta gcgagattcg ggtggacgga gggatttatt    9900
ggggtggtga tcgtttgttt ttttttgtgt gtcgtgggat tttagtttta ttgtttttgc    9960
gtatattttt attttaggta tggttattag gataggttaa tttgcgttta agtgagagga   10020
tagggttgtg gggtagttat ggggagggcg ggtataggtt tttgtagttt tttttattag   10080
gtcgttttgg gttaggggtt ggcgaatgtt ggatgtggtt ttgtttaggg ttagtaggaa   10140
atatttggtg tgttatatga taatgattat atattgattg tgtgataatg gttatatggg   10200
agagtgattt tgtggggaag ggttgtttat agaaagtttt ttaggattaa taggttgtgg   10260
ttaggtatag aggtggggat aagaatattt taggttaagg tgcgggatat gagagtgttt   10320
ttagggggtt aagaataatg gttattggag tcgggtggaa ggatgttagt gggtttggag   10380
ggagaaagat tgattttcgt ttttgtcgtt ttattttgcg gaaaggtttt ggaaggtagg   10440
ggttgagtcg gttttttttta ttgttgaaag agtagattga ggtagggcgc ggtggtttac   10500
gtttataatt ttagtatttt gggaggtaga ggagggtgga ttatttgagg ttaggagttc   10560
gagattagtt tggttaatat ggtgaaattt tatttttacg aaaaatataa aaattagttg   10620
ggcgtggtgg tgggtgtttg taattttagt tatttaggag gttgaggtag aattatttaa   10680
atttaggagg tggaggttgt agtgagtcga gattacgtta ttgtatttta gtttgggtga   10740
taaagcgaga ttttatttta aaaaaaaaaa aaaaaaagaa aagaaaaaaa gaaagagtag   10800
attgggcgtt gattgtttgt ttaagcggtt agtttagggg tttggcggtt gtgttagagt   10860
tgtgcgtggg gatttattga gggagtgtgc gtgtgttttt ttggggatat tattaggatt   10920
tattttttag gaagaagagg aatttttggt aggtagggaa ttagttttag ttaattttag   10980
attttttttta cggttcggtt tagagttttt gtatgtaggg agtattagag cggttgttga   11040
acggggagaa agtgttttat gtttgtaagg agcggtttgg tttagattat gtttaaggtt   11100
tttttggttt ttataggtat cggggggcgg ttagtgttgt ttttgggttt tttttatata   11160
gaagtttgtt ttggataggg tgtcgtgtcg gttataatga gtcgtgagtt tggtttgggg   11220
ttttttgagg agtttatttc ggtaagattg tatttttgag gtgtgggtag ttaatttatt   11280
ttcgtttatg gtcgatttga gaattagttt tttttgtagg gtttcggatg ggtttttttt   11340
acggaatagg tttcggggtt ggggtaggtt ttcgaggtcg tttatttcgg ttatttttag   11400
gtagatttgt tgtttttttta ggaggttttg tgcgattagt tggtttttta tgtttaatcg   11460
ttttgtagag tgatggtgga ggtggtagtt agtcgggtcg gtttttttttg gaattatttg   11520
agtttcgggg attggtttaa ggatgaggaa tttcgttggt tttgttttga ggtaggttgt   11580
ttttttgtgt tttgtatttt tgggaggtag gggagggtat ggttttgttt tttgattatt   11640
tttttttttg ttataaaaat taggttagtt gtggtggttt aggtttgcga ttttagtatt   11700
tagggaagtt aaggtaggag gatttttgcg gtttggagtt cgagattagt ttggataata   11760
taatgagatt ttgtttttat aaataaaaaa ttagttggtt gtggtggtgt atgtttggga   11820
aatgaggtag gaggatcgtt tgagtttagg agtttaaggt tgtagtgagt tatgattgta   11880
```

gtattgtatt ttagtttggt taataaagta agattttgtt tttaaaagaa ataaaataaa 11940
attaaatata gtagggtgtg agtgaggatt gtattaggtt tatttattag tttgggatta 12000
atttagagtt ttagaaaatt gaatttttta tttttttttt tttttttttt tttttttttt 12060
ttttgagaag gagtttcgtt ttgtcgttta ggttggagtg tagtggtata atttcggttt 12120
attgtaattt ttatttttcg ggtttaagcg atttttttgt tttagttttt cgagtagttg 12180
ggattatagg tacgtattat tatgtttggt taatttttttg tatttttagt agaaacgggg 12240
ttttgttatt ttggttaggt tggttttaaa tttttgattt taaatgattt atttgttttg 12300
gttttttaaa gtgttgggat tataggcgtg agttatcgta tttagtttaa tttttaaatt 12360
ttttgtagag atagggtttt gttatgttgt ttaggttggt tttaaatttt tggtttttag 12420
tgatttattt gttttagttt tttaaagtgt agggattata ggtatgagtt attatattag 12480
gttatttatt gtttgagtat tatttaattt agaaatgaat aatatagggt cgggagcggt 12540
ggtttacgtt tgtaatttta gtattttggg aggtcgaggt aggtagatta cgaggtcgag 12600
agattgagat tattttggtt aatatggtga aattttattt ttattaaaaa tataaaaatt 12660
agtggggcgt ggtggcgggt gtttatagtt ttagttattc gggaggttga gggaggagaa 12720
ttttttggat ttgataggta gagtttgtag taagtcgaga ttacgttatt gtattttagt 12780
ttggtgatag agcgaggttt tgtttaaaaa aaaaaaagaa gataaaataa aaaaaaatat 12840
agatagtgaa agggaagaag ggagtttgtc gtaggttata gggtattagg aattgttttt 12900
gataaagtgt ttattgagcg cgggcgaatt ttaatattat atttcgagag gagttagatt 12960
taaaaggtcg cgcggtgtga ttttatttat gtggaatgtt tagggtaggt taatttatag 13020
ggataggaag tagattttg ttgttagggt tggggtatgg ggtggggagt gattgttaag 13080
ggggacgggg ttttttttg gggtggtggg atgttttaaa gttaatatga ttttggttgt 13140
ataattggaa atgtattgaa atgtttttga attttacgtt tgggagggtg agtgttgtgg 13200
tgtgtgaatt atattttaat cgtgtaatta aatagggagg aggaagtttt ggaaggaggt 13260
tgtggggatt ttgtgttatt ttttcgtatg ggattgggat gttttgttga gtttttggtt 13320
gtacgaggat ggggtggtgg tggcgtgtgt ggttgtgtgg tcggttttgg aaggggttta 13380
ggttttttgg gttttttttt tttttttttt tatatatttt taaagggtta agtgattttt 13440
ggagatcgtt atgttatggt atagttcgtt ttaatgtttt atagagttgt aggatatggt 13500
atagtttacg ggaaaatatc gggttatttg gggggtagtt tattttttgt ggttagtttt 13560
gatggagggt ttagggttta ttatataagg ggtgtgttta gacgttgttt ttcgtttatt 13620
ttgaaagttt cggagtttcg tggcgcgggt ttcgattatt tttttttttt tttcgggtag 13680
gttgaagggt tttttttttt cgttagggta gttgtaggga gaggagtcgg gtgggtaatt 13740
gtgtttttta ggtaggttag tttcggtata gacgttggtt ttgtagaatt agtcgttacg 13800
atcgtttttg attttgggag tgatttagtt tgttcgtggt gtaggaggac gaggaggggga 13860
agagagagag ttattgatat ttatttcgtt ttaaagcgtt agggtataat taggaagagg 13920
ttttatatgt ttgtttgtga tttagaggag gagtttaggg gatagcgggt ttgtagagga 13980
tttgttaggt gtaggtgggt ggttgtaagt ttcgtagagt tatagttatt gtggatgggg 14040
gagtttgaat tagattaagg tattaggaag ttttcgggaa ttaagagagg gagtggatag 14100
gtatggtggt ttacgtttgt aattttaata ttttgggagg ttaagatagg aggatggttt 14160
gaggttagga gtttaagatt agtttgggta atatagtgag gttttatttt tataaaaata 14220
aaaaatttag ttgggtgtgg tggtgtatgt ttgtagttta agttatttgg gaggtcgagg 14280
gggtaggatg gtttgagttt aggagtttgg gttgtaataa gttatgattg tgttagcgta 14340
ttttagtttg ggtgatagag taagagttag attttaaaaa ataaaataaa ataaaataaa 14400
ataggttggg cgtagtggtt tacgtttgta attttagtta ttcggggagg ttgaggtagt 14460
agaatcgttt gaatttggga ggcggaggtt gtagtgagtc gagattgtat tattgtattt 14520
tagtttgggt gatagagtaa gattttgttt taaaaaaata aaaaataaag agagggaggg 14580
gaagttagtt ggttttgggt tttgaggggt gttcggttta aggaggaagg ataagatgtt 14640
atgttttgtt ttaataaatt tggttttaga atgtagagag ttcgttatgg taggtgtagt 14700
ggtttacgta tgtagaaagg tttaggttat agttgttttt ttgggttcgt tggttttaac 14760
gtttaatagg ttgagttatt agaaggagtt ggaatggcgt tatgggtttt aagttgggat 14820
gagggagttt aaggttatag ttgatggtta acggtcgaag ttaagggtgg attaattagc 14880
ggaaggaata tagtgggtgg ggagattttt aggttttttt tattttttta tagtttttttg 14940
gatgtgggtt atttaggcgt ttttgttttt tttggagttc gtgtagattt tatatatttt 15000
cggtttttgt aaggaggcgt tgattagaaa tatcgttttt ttttcgttgg ttattttttt 15060
tacgatgagt ttttgtaacg agatgacggg tggttttgag ttttgtatga gtagagggta 15120
ttggggaggt tattttttat tttatagtat ttaaatagta gggaggttaa gggtttggtg 15180
aggggaatta agagtggggg ttacgttttt tatttttttt taaataattt aaagcggaaa 15240
tattttgtta tttgggggaa aaaagatatt attgtagtga aattttagaa attttttttt 15300
tttttttttt ttttttaaat tattttggag gttaggtttt gttttgttat ttaggttgta 15360
gtgtagtggt gtaattattg tttttgtag ttttgatttt ttgggtttaa gtgatttttt 15420
tattttagtt ttttgagtag ttgggattat aggtacgtgt cgttaggttt agttaatttt 15480
tgtatttttt gtagagatgg gattttatta tgttgtttag gttggttttg aagttttagg 15540

```
tttaagtgat ttttttattt cggtttttta aagtgttgag atgataggag tgagttattt   15600
tgttttttag tatttcgata atgtgttaat tttattagtt taaaaatttt tttttaaata   15660
gtcggtaatg tatatatata cgagaaagtt taaaagatat aacggtatgt agtatataat   15720
aatagataaa tttatttttg ttttgtgttt taggttattt agagttatcg gtatttttag   15780
tttgttacgt atttttaag agataatgtt tttatagatt agtaaaatgt atattttaat   15840
tggttagagt gggttatatt gtgtttttta taaatttata tgaagttttg agttttagta   15900
ttttagaatg tggttgtttt tggagatggg gtttttaaag aggtgattaa tgtgcgggtg   15960
tggtggttta tttttgtaat tttagtattt tgggaggttg aggtaggtag attatttaag   16020
gttaggagtt taagattagt ttggttaata tggtgaaatt ttatttttat taaaaatata   16080
aaaagtagtc gggtgtggcg gtaggtgttt gtaattttag ttatttagga ggttgaggta   16140
ggagaattat ttgaattcgg gaggtggagg ttgtagtgag tcgagattgt gttattgtat   16200
attttagttt gggcgataga gtgagattgt tttaaaaaaa aaaaaaaaaa aaaaaaaaaa   16260

gagaggtgat tatggtaaaa tgagattata tgggcgggtt taaatttaat ttgattggta   16320
ttttttaat aagaggagat tagtatagag gtatatatag agggatggtt attcgaggat   16380
atagagaata gatgagtata tggttgttta taagttaagg ggttttagaa gaaattaatt   16440
ttgttgatat ttttattttg gatttttagt ttgtagaaat atgagataat aaatttatgt   16500
ggtttaaatt atttagttta tggtattttg tttatagtag ttttagtaaa taaatatatt   16560
ggttaagaat atattagtag ttgggtacgg tggcgtacga ttgtaatttt attattttgg   16620
gaggttaagg cgggaggatt atttgaggtt agaagatgga gattagtttg gttaatatgg   16680
tgaaatttcg tttttattaa aaatataaaa aattagttag gcgtggtggc gggcgtttgt   16740
aattttagtt atgtgggttt ggaaaattta taggtgtttt ttttatgggt agttttagtt   16800
aattttttaa taaggttata gtaagtaggg tttttcgttt gttttttgtt tgtttgtttt   16860
gttttttgt tttgagatag agtttcgttt tgttgtttag gcgggagagt agtggcgtag   16920
ttttgttgga gagtagtggt ataattttag tttattgtaa tttttatttt ttgggtttag   16980
gtgattattt tgttttagtt ttttgagtag ttgggattat agatatgggt tattatgttc   17040
ggttaatttt tgtattttta gtagaggtga ggttttatta tgttggttag gttggatttt   17100
aatttttgat tttaagtgat ttgtttattt cggtttttta aagtgttggg attatagacg   17160
tgagttatag tatttagtcg atttttcgtt tgaagagata ggatatatat tatagaagta   17220
aagacgtatt tttgattttt tttttgtagt agtaaaagta cgtcggttag taggatagtt   17280
aggatatttg ggaaaatagt atggaaggat gtattttaaa agttttatag ttaggaggtt   17340
ttgttggtta ttttcgcgga tgttattttt taggaaggtg taagtagtat gatgtttttg   17400
tttgggttta gaagtttttt ttttgttttg tttgtatttt ttattatttt tttaggtttt   17460
aatttttagt attaacgagt agtcggagtt ttttttaagt ttttttcgtg tttagagttt   17520
aaattagggt tttcgtttat gttaggttat tgtgttcggt aatttttttt gttgtaataa   17580
tatttgttgg gtttgatttt tgagaatttg atacggtttg gttgcgtttt tatttatatt   17640
ttattttgaa tttttacgtg ttgtaagagg gatttggtgg gaggtaattg aattatgggg   17700
gtagattttt tttatgtcgt tttcgtgata gtgaatgagt tttataagat ttgatagttt   17760
tataaggggg agttttttg tataagtttt tttttttttt gtttgttgtt atttatgtaa   17820
gatttgattt gttttttttg ttttttgtta tgattgtgag gttttttttt ataggtggaa   17880
ttgtttaagt ttattaaatt tttttttttg gtaagttgtt tagttttagg tatgtttttta   17940
ttagtagtat gaatacggat taatataata tttttttttt tgatgtttta gatgtgaacg   18000
gagtttttttg tttttttttgt ttttgggtta ttaggattcg agttatagta gcgatgtagg   18060
ttaggggtgg ttttagtttt ttcgtgtttt ttagtttttt ttggcgtttt gtttttgtat   18120
gaatttttcg tgttgtaagg tttttttgtat atagatgtaa attttgagtt gcgttttaag   18180
ggtaacgtta tttttgggga gttgttttta atatttttgt ttaagttaga tacgtttttg   18240
ttaatttttt tatattgtag tttgttggtt tttttaaagt atgtgttgga tttttaatta   18300
gtttttcgt tttttggttt gttttttttt tttagtttag tgattgatgt tttaaaaggt   18360
taggtattag gttttgttcg gtggggtgta tttttattat ttaattcggc gtttggtagg   18420
tagaaggcgt taaatttgtt tgcgaaatat acgtttattt aaa                     18463
```

<210> 117
<211> 15597
<212> DNA
<213> Artificial Sequence

<220>
<223> chemically treated genomic DNA (Homo sapiens)

<400> 117

```
ttttgtcgtt ggtaattttt tttcgaaagc gtaaacgttt ttagggagtt attaggaata      60
gatgtggatg ggtagttttt atttagggta tagttgttat ttattcggtt tagaattggg     120
aagtcggtcg ggtatagtgg tttacgtttg taattttagt attttgggag gtcgaggcgg     180
gtaggttatt taaggttagg agtttaagat tagtttgacg aatatggtga aattttattt     240
ttattaaaaa tataaaaaat tagttaggta tggtggtgga tatttgtaat tttagttatt     300
taggaggtta aggtaggaga attatttgaa tttaggaggt agaggttgta gcgagtcgag     360
attgcgtttt tgcgttttag tttggtaata gagggagatt ttttttaaa ataataaaat     420
aaaaacgaat tgtgacgttt atttaagtag gaggtatttt ttatgtaaag gtatagcgtg     480
tttttggaa taggcgtttg ggagaatcga tttatttatt tgttgtgtag ttttgtcggc     540
gagtagcgcg aattttatag ataggttttt tagagtttgt tttagggatt tttacgtgtt     600
gttatttaga gcggtttagg agggtagcgg ggtcgtgtta gattttattg tattgaggga     660
gtaagtatat aggagaggag atatttaagg tttggagttt atttggtagt cgagtttggt     720
atttttttcg atagatgttt tttacggtaa tttgcgtgat ttcgttttag gattttgttg     780
tttaaagagt ggtttgtggg gattttggga gttcgggaga aagggtggtt ttggggttgt     840
attttaggat ttgttgcgtt tttttttttt aattagtttt taggtgatat gtgtgtatag     900
gtgacgggta ttgttggggt aagtagagtt tcgggtgggg tttttttttt ttttaggtaa     960
aggaagcgtt gttagtttat ggtttgtttt tttttaagtt aaagggattt tttttttttt    1020
tgagacggag tttcgttgta ttatttaggt tggagtgtag tggcgcgatt tcggtttatt    1080
gtaattttcg tttttttaggt ttaagtaatt ttttgtttta gtttttcgag tagttgagat    1140
gataggtatt cgttattacg tttagagaat ttttttttgt atttttagta gagacgggtt    1200
ttattatttt ggttaggttg attttgaatt tttgatttcg tgatttattt atttcggttt    1260
tttaaagtgt tgggattata ggtgtgagtt attacgtttg gtttgggatt ttttttttaaa    1320
gttttatttt gatagtgaaa aaattattag attagaaatt ggtgagtatt tattcgtgtt    1380
tagaaattcg tgttttaggg tcgaaatttt ttcgtgtttt ttttaaatat gattttttat    1440
gtgtttgagt tattttttgt tttttttgag atgaaatagg ttaggatgtt ggttattacg    1500
gtaaacgtat ttttcgttta agttttttat tagtatggat tttcgtttta tcgcgcgtgt    1560
ttttgtagaa gttgaaaggt tatttatttt agtttttttt cgaatatgag aagatttgtc    1620
gtattgtcga tggttcgcga tgcgattcgt tcggttttgt cgcgaagttt gtgaaagtta    1680
ttgtagatgt ttcggattag ttttcggttg atggtaaatt gagtttggat gttagttggg    1740
aggaagtttt gatattatga tggggggaga gatattgtgt tagtcgttgg ggagtatttg    1800
ttagacggta gtagtcggtt ttttagaagt tgagttacgg gagatatagt aggtttatga    1860
gttatttcgg gagttttatt tttttaggtt tgtagggatt attttgtgtg tttaaaaaag    1920
aaataaaaat tattattatt tgtatgttat aaatataaag ataaatgatt ttatgggatg    1980
aggattatag agtttattga aattttatta tattggagga tttatttaaa aatatttaaa    2040
taaggttagg ggtagttatt tatatttgta attttagtat tttggaggtt gaggtaggtg    2100
gattatttta ggttaggagt ttaagattag tttgattaat atggtgaaat tttattttta    2160
ttaaaaatat aaaaattagt tgggtatggc ggtatgtatt tgtaatttta gttattttag    2220
aggttgagat aggagaattg tttgaattcg ggaggtagag gttgtagtga gttgagatcg    2280
cgtaattata ttttagtttg ggcgatagaa cgagattttg ttttaaaata aataaattaa    2340
taaaatattc gaataataat tttgtcgggc gtagtggttt acgtttgtaa ttttagtatt    2400
ttgggaggtc gagtagggcg aattacgagg taaggagatc gagattatgg tgaaatttta    2460
tttttattaa aaatataaaa aattagtcga gtttggtggt gggtatttag ttttagttat    2520
ttgggaggtt gaggtaggag aatggtatga atttcggagg cggagtttgt agtgagttgc    2580
gattatatta ttgtatttta gtttgggtaa tagagcgaga tttcgtttta aaaattaata    2640
atattaattt ttagttggat atagtgattt atatttgtat tttagcgttt tgggagattg    2700
aggtaagagg attgttagag tttgggagtt taagaatagt ttgggtaata tagtgaaatt    2760
ttattttttt taaaaaatta aaaaattagt taggcgtggt ggagcgtttg tggttttagg    2820
tgtttataga gggttgaggt aggaggatta tttgagttta ggagttttat gttgtaagga    2880
gttatgatta taatattgta ttttagtttg gggattttgt ttttagaaaa taataaaaat    2940
aaaaataaat aatttttaag ttaaatataa tgaatataaa tttttgaaag atttttttata    3000
tggggggaag gttttttttta tgtcgcgttt atttttaaag atttttttata agatttgata    3060
ttaattatta gagtttagaa aatagaaagt ttagagagta aaatgaaggt aaaagatttg    3120
gttaggtatg gtggtttatg tttttaattt tagtattttg gggggttatg ttaggaggat    3180
tgtttgaggt taagagttta agattagttt gggtaatata gtgagatttt atttatatta    3240
aaaataaata aataaattag ttaagtattg tggtgtatgt ttgtggtttt agttattttg    3300
ggaggttgaa gtgggagaat tgtttgaatt taggaggtcg aggttgtaat gatttacgat    3360
tgtattattg tattatagtt tagaggatag agtaagattt tgttttaaaa aataaaataa    3420
aatagatttt taatttttgt atgaagaagt aggttttttt ttggtttttt tgttgttgtt    3480
gttgtcgttt ttgatataaa gttttatttt gttatttagg ttggagtgta atggtacgat    3540
ttgggtttat tgtagttttt gttttttggg tttaagtagt ttttttgttt tagtttttta    3600
aatagttggg attataggtg tttgttatta agtttggtta attttttttt tttttttttt    3660
```

```
gatggtgttt ttgtcgttta ggttggagtg tagtggcgta attttggttt attttaattt     3720
ttttttttta ggtttaagta attttttgt tttagttttt tgagtagttg gaattatagg      3780
tgtacgttat cgtatttagt taatttttgt atttttagta gaggcggggt tttattatgt     3840
tagttaggtt agttttgaat ttttgatttc gtgatttatt cgtttcggtt ttttaaagtg     3900
ttaggattat aggcgtaagt tattacgttc ggtcggtttt ttggggtttg ttttgttttt     3960
ttttgttttt ttttttgtt ttgtattttt attagagatg ggttttgtt atgttggtta       4020
ggttggtttt aaatttttga ttttaagtga ttcgatagtt ttagtttttt aaagtgttgg     4080
gattatagga aggagttatc gcgttagttt gaagaagagg tttaaaaaga aaaaattaaa     4140
aataaaaaaa ttattaagta gttattttag ttttttttgg aaaaagatta aattttaaat     4200
atttaataat aaataaatgt aataatgaat ttttagaaat ttgaaaaggt gttgatataa     4260
gtattcgttt gtttgagtta tagaaaaggg tagagggtac gaggtgtttg gagggtttga     4320
gggtagagtt tagtcgtaat tcggttttgt tgtttagagg ttcggggttt tggtaaatga     4380
tagttttttt gagtttgttt ttttattcgt aaaataggtt ttttagtttt gtgtcggagt     4440
aattgaaacg agggttaaat gaggaaatta tgcgatagat gttagtataa tttttgttac     4500
gtagaggagg taaaattatt ttgaagggtt aaaatttgtt tttttggtta aaaagggatg     4560
tattttttaa agttagaaaa atattttggt tttttttttt tttttttttt tttttttttt     4620
tttttttttt ttttttattt tatagttttt tttttatgcg gagtcgaagt tggattgtat     4680
tgttgttatt tcggtttatt gtaatttttt tgtttgattt ttttgtttta gtttgtcgaa     4740
tgtttgcgat tgtaggtacg cgtcgttacg tttgattggt tttggtggag acggggtttc     4800
gttgtgttgg tcgggtcggt ttttagtttt taatcgcgag tgattcgtta atttcggttt     4860
ttcgaggtgt cgggattgta gacggagttt cgtttattta gtgtttaatg gtgtttaggt     4920
tggagtgtag tgacgtgatt tcggtttatt ataatttata ttttttagtc gtttgttttg     4980
gtttttttaaa gtgtcgagat tgtagttttt gttcggtcgt tatttcgttt gggaagtgag    5040
gagtgttttt gtttggtcgt ttatcgtttg ggatgtgagg agttttttttg tttggttgtt    5100
tagtttggaa agtgaggagc gttttcgttc ggtcgttatt ttatttagga agtgaggagc     5160
gttttttttt agtcgttatt atatttagga agtgaggagc gtttttgttc ggtcgtttat     5220
agtttgagat gtggggagcg tttttgtttc gtcgttttat ttgggatgtg aggagcgttt     5280
ttgttcggtc gagatttcgt ttgggaggtg aggagcgttt ttgttcggtt agttatttcg     5340
ttcgggaggg agatgggggg gttagtttta ttatttggtt agtcgtttcg ttcgggaggg     5400
aggtgggggg gttagttttt cgtttggtta gtcgttttat tcgggaggga ggtgggggc      5460
gttagttttt cgttcggtta gtcgttttat ttgggatgtg aggagcgttt ttgttcggcg     5520
agatttcgtt tgggaggtga ggagcgtttt tgttcggtcg tttcgtttga gaagtgagga     5580
gatttttttgt ttggtaatta tttcgtttga gaagtgagga gttttttcgt tcggtagttg    5640
ttttgtttga gaagtgagga gtttttttcgt tcggtagtta ttttatttgg gaagtgagga    5700
gcgttttcgt tcggtagtta ttttattcgg gagggaggtg ggggggggtta gtttttcgtt    5760
cggttagtcg ttttattcgg gagggaggtg gggggttagt tttttcgttc ggttagtcgt     5820
gttattcggg agggaggtgg gggggttagt ttttcgtttg gttagtcgtt ttgttcggga     5880
gggaggtggg ggggttagtt tttcgtttag ttagtcgttt cgtttgggag gtgaggggcg     5940
tttttgttcg gtcgttttta ttgggaagtg aggagttttt ttgtttagtt agtcgtttcg     6000
ttcgggaggg aggtgggggg ggttagtttt ttagttcggt tagtcgtttc gttcgggagg     6060
tgaggggcgt ttttgttcgg tcgtttttat tgggaagtga ggaggttttt tgttcggtta     6120
gtcgtttcgt tcgggaggga ggtggggggg ttagtttttc gttcggttag tcgtttcgtt     6180
cgggagggag gtgggggggg gttagttttt ttgtttggtt agtcgtttcg ttcgggaggt     6240
gaggggcgtt tttgttcggt tatttttatt gggaagtgag gagttttttt gttcggttat     6300
tatttcgttt gggaggtgtg tttaatagtt tattgagaac gggttaggat gataatggcg     6360
gttttgtgga atagaaaggc gggaaaggtg gggaaaagat tgagaaatcg gatggttgtc     6420
gtgtttgtgt agaaagaagt agatatggga gatttttttat tttgttttgt attaagaaaa    6480
atttttttgt tttgggattt tgttgatttg tgattttatt tttaattttg tgttgtgttt     6540
atttagggtt aaatggatta agggcggtgt aggatgtgtt ttgttaaata gatgtttgaa     6600
ggtagtatgt tcgttaagag ttattattat tttttaattt taagtaatta gggatataaa     6660
tattgcggaa ggtcgtaggg tttttttgttt aggaaaatta gagatttttg tttatttgtt    6720
tatttgttga tttttttttt attattgttt tatgattttg ttaaattttt ttttgcgaga     6780
aatatttaag aatgattaat aaaaaaataa attaaaaaaa aaaaaaaaga aaaaaaaaag     6840
aaaaatattt tggttttggt agttagttta tagtttagga attcgttttc gacgtattgt     6900
gttgattttt ttaatttgtt ttgtatattt tgtaaaagga aaatatatag ttttattttt     6960
ttttattagt agtaagaaag ttgttgtggg gtatgggtta ttgtggagcg ggaggtcgtt     7020
ttttttttagg agaaatatat ttttatatgt tggttttgat attagtattt tgtaattttt    7080
tgattttttt ttagatgttt tttttattaa ataggatagt ttggttttat agtaggagta     7140
ttataaattt gtagtttttt tttagaggta tagttatttt attcgtagac ggggggtagt     7200
tttttagaga aatagttatt ttatttatag attaggggcg gttttgtaga gaaacggttt     7260
ttttatttat agattcgggg cggcgttagt tttagagata tttgagattt tggtagtttt     7320
```

```
taaaaacgta atagaatagg gtaaaagtgt tttttttttt gggaatgtat ttatggcgtt    7380
agggatatta gagtttaggg agttgttggg tagagggata ggtttagttt ttggtatcgg    7440
agttttttta tttgaaagag gttttttggg taaagtcgtg ttttaaagtt aggagttatt    7500
taggagatat aaggggaaat tttaatatgt agttgtttgc gtgtttagga atttttaggt    7560
tttagagatg ttttaaagat tatttgattt aatttttatt ttataaaaaa aaaaaaaaaa    7620
aaaaaaaaaa agatttgagg tttagtatgg tgcgtgtttg tagttttagt tatttagagt    7680
ttgaggcggg aggatcgttg gagtttaggg gtttagggtt agtttgggta atatagtgag    7740
attttatttt taaaaataga aagtaagtaa gaaaattgag gtcgggaagt gatagagaat    7800
gatatttgtg ggaaggttat agggaacgtt ttgtttttag aatagggata ttgtttattt    7860
gtgatttatt gtatttttgt ttatcgtttt ttttaggttt tgttatattt ttttgttggt    7920
aatgtagatc gtttttaaag gaaggttttt aattttaggg tttaaaagat tttattgttt    7980
gttttagata ttttcggggg tattttaggg atattttagg tataggggga aggttttttt    8040
gttttcgaga ggaacgaaga taaaaggaat taaggtttgt gtgaagttag tttgggagag    8100
ggggtgtttt tttgtagtag ggataaggat agaggtcgtt aggtttttat ttaagacggg    8160
gtttttttga aatagaggag agtttggaaa tttttatttt agtggagttt tcgttttttt    8220
tcgtataggg agagggtttt gtaggtatag gtggaggaga agtataggat tcgttttttt    8280
tggaggggtt ggtagttgtt ttttttttta ggaaatagga gttgggttgt agggttgttt    8340
tttgtttttt tgagataggg ttttatttttg tcgtttaggt tggagtgtag tggtataatt    8400
atagtttatt gtaagtttta tttttgggt ttaagcgatt tttatgtttt agtttttttaa    8460
gtgattggga ttataggtgt gtattattat gtttggtttt agttttgttt tggtagagac    8520
gaagtttcgt tatgttgttt aggttggttt taaacgtttg ggtttaaggg attatttagt    8580
ttcggttttt tcgagcgttg ggattagagg cgtgggttat tgtatttgcg aattgtagtt    8640
ttttgatata taataaggtt gtgtcgtagt agttgggagg agggaagttt tatttagatt    8700
tttgacggag tgtatgtata ataagttaat tttcggatgt aggtgtgggt tgtggttttg    8760
ataaggaggt ttagtttgtg ttttagtggt ttttagttag atagggtttt gttttaagtt    8820
aatatttttt tttatttgga gtttttggta tttttttaaa ttttttaaat tatttagttt    8880
aaatttttaa agtgattata gagatttatg atttttacgc ggatttataa aattttgtaa    8940
agttaatatt agggatattt ttgaaaatta tatttagatt gttatttgga aaggaaagaa    9000
gttttgttta tgttattttt gttgggcgtt taaataaggg attaggagga taagaaatta    9060
tatttgagag aggaaaaggc gggttcggag gttcggggat ttttcggggt cgggatatgg    9120
gtagggtaga tatatttatc gagtcgttga aggataggaa gagtttgagg attatatttt    9180
cggagaatag ggggtgtcgc gttattaggt tgacgaagcg ttttagggtt ttttttttgg    9240
tttcgatgaa tttttttgtta gttggaggag tatacggggt tattggatag agtttagggc    9300
gtcggttttt attttttgtt ttgggcgagt ttttaaaatt ttttttagag gtggtagagg    9360
gtttagcggt tagtagaggg tattggttgg gtttttagtg ttgtttagat aatgggtttg    9420
aaaaggtcgt ttagagtaat aattttattt ttagaggttt tttttagtta tatatatttg    9480
ggtttaggag ttgttaagga aaattataga tttgtttgtt tttatcggga gttttgggaa    9540
ttacgtgagt ggttatttta attattttcg ttaaagatat tttttataat tttttttttgg    9600
tattttttat tgtaaataaa tgggttttgt tacgttttaa tattaacgta atttaggttt    9660
tttttttttt tttttttttt tgagatagag tttcgttttt gttgtttagg ttggagtgta    9720
gtggcgtaat ttcggtttat tgttattttc gttttttagg tttaagcgat ttttttgttt    9780
tagtttttgg aattagttgg gattataggg gttcgttaat atgtttggtt aatttttgta    9840
tttttagtag agatggggtt ttattatgtt ggttacgttg gtttttaatt tttgatttta    9900
ggtgatttat ttgtttcggt tttttaaagt gttgatatta taggcgtaag ttatcgcgtt    9960
cggtcgagtt tttttattta ttgtgtttat aatttatttt gtaaaggatg taattattta   10020
ttgtttttat tcgggttaaa tatttttatt ttaaggggaa aaaaagtaaa agtatatatt   10080
gatgtattta ggatatttta atttgaatat aaataaaaat ggtatttaaa tatttttttt   10140
gagacggagt tttattttttg tcgtttaggt tggagtgtag tggtgttatt tcggtttatt   10200
gtaattttta atttttaggt ttaagcgatt tttttgtttt agtttttttaa gtagttggga   10260
ttataggtat tcgttattat atttggttaa tgtttatatt tttattagag aggaggtttt   10320
attatgttgg ttatgttggt ttcgaatttt tgattttagg tgattcgttt attttagttt   10380
tttaaagtgt tgggattata ggtatgagtt attagcgttt agtttttaaa tattattttt   10440
aatgtattaa gaaatatagt taggagtgtg gtttatattt gtaattttag ttttttggga   10500
ggttgaggag ggtagattat ttgaggttaa ggagttttat attagtttgg ttagcgtagc   10560
gaaattttgt ttttgaaaaa tattataaaa gaggttaggc gtagtggttt atatttgtaa   10620
ttttagtatt ttgggaggtt gaggtaggtg gattatgagg ttaggagatt gagattattt   10680
taatacggtg aaattttatt tttattaaaa atataaaaaa aaataattag ttaggcgtgg   10740
tggcgggcgt ttgtagtttt agttatttgg gagatttagg taggagaatg gcgtgaattc   10800
ggtaggcgga gtttgtagtg agtcgagatc gtattattgt attttagttt gggtgatacg   10860
gcgagttttc gttttaaaaa taaaaataaa aataaaaaat attataaaag aggttaggtg   10920
cggtggttta tatttgtaat tttagtattt tgggaggttg aggtgcgcgg attacgaggt   10980
```

```
taggagattg agattatttt ggttaatacg gtgaaatttc gtttttatta aaaatataaa   11040
aaaattagtt aggcgtggtg gcgggcgttt gtagttttag ttacgcggga ggttgaggta   11100
ggagaatggc gtgaattcgg gaggcggagt ttgttatgag tcgagattgt attattgtat   11160
tttagtttgg gcgatagagc gagattttat gtaaaaaaaa aaaaaaaata ttataaaaga   11220
aagaaaataa ataatatagg taggtggttg gggttttttt ttggtttagt attggtaggt   11280
ttttttcgat gaggttgtat ttttttaaat gcgtaagcgg tttagcgcgc gtgtgtagtt   11340
tttaggcgtt gaacgttagt tagcgtttat taattcggat gtttaggttt gagttagtat   11400
tatatatttt tgtcgcgttt tttgtttttt gttgttttga gcgttcgtgt gttttttagc   11460
gtgagttgaa tgatttgttt tgtggttttt atatgcggcg gggtgaaaat tatattatta   11520
gattttgggt tgtttgttta gtgatcgatg agaggtagaa gtatttatgt attatcgtac   11580
gattttagat gcggggaatt ggcgattgtg ttagttttta tgttgcgttt tcgtttagga   11640
tacggtgatt gtggaagggt tgggaacggt agaaagttat gattagtttt aagtatttat   11700
tttttttagta tgttttattt agaatttgag gaacgggttg agggagagat tattagtgag   11760
attagagttg gtagtcgtta ttttaagttt tgtagtttag ggggatatag gtttggggag   11820
tagttttgag gtcgtttgcg atttttttatt tggttcgagg ttatttttag gtagagaggt   11880
gaaggtgagg tagtcgtgtg ttgttttttt ttttttgtaa gatgtagttg atattttttta   11940
tttgtataat ttagtttttt tttatgaggt tatcgagcgt ttttatggtg tttatggttt   12000
aggttgttat ggttttgtag atgtaggtag ggtaatgtta gttaggtgag tatatagtgt   12060
ttttggtagt tttttcggga gttttcggga gttttggtta ttgtatttttg gagaggagaa   12120
ttagggtttt tttttatatt tgtatagtaa gggttttttt atttgttagt tatgtagttg   12180
aaggttttgg tgggttaagg ttgtcgggtg ttagtttttt tgtttgtatt aaggttgggt   12240
ttaagttata gttttgtttt agatgttaaa aggttggagt ttttttcgta gttttgtatt   12300
tgtttcggtt tatagtgggg tttattttttg agttaggatt atagggttta ggagatgggt   12360
tcgttttttt cgttttcggt gggtagtacg tttggtttta tttttttagta tttttttggg   12420
tggtagggta ggtattatac ggtaggggaa tttgtgtagg agtatttttt ggaagattac   12480
gaagttattg tatcgtttgt atatcgagga tttgaagcgt tgtaagagaa gggtcggtgt   12540
ttagattcga cgttggaaat tatgttgtcg cgttattttt ttttaataga gttcgggaaa   12600
acgatagtat taagtgtatt ttttttttatt aggtttttat taaatggaga aattttgtga   12660
cggtttcgta tatggaaata taatttttaa attttgtata gtttttaggt cgggtgcggt   12720
ggttttcgtt tgtaatttta gtattttggg aggttgaggc gggtgaatta tttgaggtta   12780
ggagttaaag agattagttt ggttaatatg gtaaaatttt atttttatta aaaatataaa   12840
aattagttgg gtatggtggt gggcggttgt agttttagtt attttggagt ttgaggtagg   12900
agaatttttt gaattttgga gatagaggtt gtagtgattt aagatcgtgt tattgtattt   12960
tagtttggac gataaagcga gattttgttt taaaaaaatt aaaaaaggtc gggcgcggtg   13020
gtttacgttt gtatttttag tattttggga ggtcgagata ggcggattat gaggttagga   13080
gatcgagatt attttggtta atacggtgaa atttcgtttt tattaaaaat ataaaaaaatt   13140
agtcgggcgt ggtggtgggt atttgtagtt ttagttattt aggaagttga ggtaggagaa   13200
tggcgtgaat tcgggaggta gagtttgcgg tgagttgaga ttacgttatt gtattttagt   13260
ttgggcggta gagcgagatt ttattttaaa aaaaaaaaaa aaattaaata aatggtatga   13320
agtatagtgg tttttttattt ggttagtgat tggttcgtta tatatatatt agggtgatta   13380
aattgttttt ttgataaatg ttttttatgt ggtaatgaat ttatttaagg gacgggtagt   13440
ttagtaagat tggataaaag gttagtcgtt ggttttttgg taggtaacgg tacgttttat   13500
acgtttagag ttgttttatt tttaggttgt taggttttag aaggaagggg tggttcgttt   13560
tgttttttcg tggtatttta ttagttggtt tatattttcg attgcggttt agagggtcgg   13620
gaattacggt aaagttagat agtaaggcgg ttttgatacg gttttttaag ggagtttttt   13680
tttttgattc gttttgtttt ttggtagtag tattgataat tattcggatc gtgtggttat   13740
aggttttgga atatagtgaa gtatagtaga gtagacggta gggaggggag atcggttcgt   13800
ttgtttttaa ggttaggtgt agtggtttat gtttgtgagt ttagtatttt ggcgggttaa   13860
agtgagagga ttgtttgagt ttaggagttc gagattagtt gagataatat agtaagtttt   13920
tatttgtatt ttaaaaaaaa aaaaaaaaaa aagattgggt atggtggttt acgtttgtaa   13980
ttttagtatt ttgggaaatt aaggtaggag gattatttga gtttaggagt ttaagattag   14040
tttggataat atagtgagat tttattttta taaataattt aaaaaattgt ttggtgtggt   14100
ggtattcgtt tgtggtttta gttatttagg agattgaggt gggagggttg tttgagtttg   14160
ggaggttgag gttgtagtga gttatgatcg tgttattgta ttttagtttg gttgatagag   14220
taatattttg tttttttaaa aataagtaaa taaaaagata taatttttat ttaggattat   14280
ttgtttatga cgttttttaa ataagttttt ggtagtgtcg ttcggtcggg gttgggttag   14340
ggtcgttttt ttttatatag gagtgggttt tgttttgttt gtgcgaattt tataagtttt   14400
gttgatttta tattttttaa ggatagtgtt agtttattaa ttatttaatg tttaggatga   14460

ataatgaaaa tttttattag aatttgtcgt aatggaattg agataacgaa gtaaggata    14520
gagagagtta cggtgttgtt ttagacgggt attaggtatt cggtgtttgt gttttatttg   14580
```

```
tttaatatcg ggatgtttag taataggagg gtcgtgttat taaaggggat tagagtgata   14640
tatattgatt ttgttatgta aattttattt attgtttggg gttcggagtc gatggggttg   14700
atattttaat tttcgggggt tttttattat tataaagcga ggttggtgtg gatttcgttt   14760
tgtgttttgt ggatcgttcg cgggagattt ttaagaaata ggtttttagg tcgggcgcgg   14820
tggtttacgt tagtaatttt agtattttgg gaggtcgagg cgggtggatt ataaggttag   14880
gagattaaga ttattttggt taatatggtg aaattttgtt tttattaaaa aaaaaaatat   14940
aaaaaaattt gttaggcgtg gtggcgggta tttgttattt tagttatttg ggaggttgag   15000
gtaggagaat cgtttgaatt cgggaggtag agtttgtagt gagtcgagat tatgttattg   15060
tattttagtt tgggcgatag agtaagattt tatatcgggg aaaaaaaaaa aagaaatagg   15120
attttagagt atagtggtgg ggaggataaa ttggattagg ggtgattagg tggggacgtg   15180
gttgggttag agtgaggtgc ggggagtaga ggtgtggaaa agttattttc gtagtttgat   15240
tttgttgcga gttttggatt ttgtttgtaa gttagttata tagatttatt ttttagtttt   15300
tttttgttat attattttttg ttttgagatg ttgagacgtg attttttttt tttttttttg   15360
tttcgatata tatataattt gttagttatt tggttggaaa ttttatattt tatatgtttt   15420
aggaagaggt tttttttttt cggaatgagt tttatttgta cggtgttttt ggttagtagt   15480
ttttgtaggg tgtgggatag tagtagcggg ttttttttgcg gtatttgtat tcgattgggg   15540
gttgggattt gttgtacgat ggtttggggt tcgatggttt ggggtgtcgg tagattt      15597
```

<210> 118
<211> 15597
<212> DNA
<213> Artificial Sequence

<220>
<223> chemically treated genomic DNA (Homo sapiens)

<400> 118

```
agatttgtcg atattttagg ttatcgagtt ttaggttatc gtgtagtagg ttttagtttt      60
tagtcgaatg tagatgtcgt aggggaattc gttgttgttg ttttatattt tgtaggagtt     120
gttggttagg gatatcgtgt aggtggagtt tatttcggag aagaagggtt tttttttgaa     180

gtatgtggag tatgaggttt ttagttaggt aattggtaaa ttgtgtgtgt gtcggggtag     240
gagggggaag aaagggttac gttttaatat tttagggtag gagtggtgtg ataggagaag     300
gttgaagggt gagtttgtgt ggttggttta taggtaagat ttagaattcg tagtagagtt     360
aggttgcgga ggtgattttt ttatattttt gtttttcgta ttttattttg gtttagttac     420
gtttttattt ggttattttt ggtttagttt gtttttttta ttattgtgtt ttggagtttt     480
gttttttttt tttttttttt cgatgtggag ttttgttttg tcgtttaggt tggagtgtag     540
tggtatgatt tcggtttatt gtaaattttg tttttcgggt ttaagcgatt tttttgtttt     600
agttttttaa gtagttggga tgataggtgt tcgttattac gtttggtaaa tttttttgta     660
tttttttttt tagtagagat aggggtttat tatgttagtt agggtggttt tgattttttg     720
attttatgat ttattcgttt cggtttttta aagtgttggg attattggcg tgagttatcg     780
cgttcggttt ggaggtttgt tttttaggaa tttttcgcgg acggtttata gagtataaag     840
cgggatttat attagtttcg ttttgtaata gtgggaaatt ttcgaggatt agaatgttag     900
ttttatcgat ttcggatttt aaatagtgaa tgaaatttgt atggtaggat taatatgtgt     960
tattttaatt tttttttaata atacggtttt tttgttgttg ggtatttcga tattgggtag    1020
gtgagatata gatatcgagt gtttgatgtt cgtttggagt aatatcgtgg ttttttttgt    1080
tttttgtttc gttgttttag ttttattacg atagattttg atgggagttt ttattgttta    1140
ttttgggtat tgagtaattg atgagttaat attgtttttg ggaggtgtgg agttagtagg    1200
gtttgtggga ttcgtataga tagagtaggg tttatttttg tgtgggagag ggcggttttg    1260
gtttagtttc ggtcggacgg tattgttagg ggtttgtttg aaaagcgtta tgagtagatg    1320
gttttgggta aaagttgtgt ttttttgttt gtttgttttt aaagagatag ggtattgttt    1380
tgttaattag gttggagtgt agtggtacga ttatagttta ttatagtttt aattttttag    1440
gtttaagtaa tttttttatt ttagtttttt gagtagttgg aattataggc ggatgttatt    1500
atattaggta gttttttaaa ttatttgtag agatgggatt ttattatatt gtttaggttg    1560
gttttgagtt tttgggttta agtggttttt ttgttttggt tttttaaagt gttgggatta    1620
taggcgtgag ttattatgtt tagttttttt tttttttttt tttttaaagt ataaatggag    1680
atttgttatg ttgttttagt tggtttcgaa tttttgggtt taagtagttt ttttattttg    1740
gttcgttaaa gtgttaggtt tataggtatg agttattgta tttggtttta aggataagcg    1800
agtcggtttt tttttttttgt cgtttgtttt gttgtgtttt attgtgtttt agagtttgtg    1860
gttatacggt tcgaatggtt gttagtattg ttattaaagg ataaagcggg ttagagggga    1920
```

```
ggattttttt ggaggatcgt gttagagtcg ttttgttgtt tggttttgtc gtggttttcg    1980
gtttttttggg tcgtagtcgg ggatgtggat tagttggtgg gatgttacgg gggagtagag   2040
cgagttattt ttttttttg gggtttggta gtttgggggt gaggtaattt tgagcgtgtg     2100
agacgtgtcg ttgtttgtta gggagttagc ggttggtttt ttgtttagtt ttattaagtt    2160
gttcgttttt taagtaaatt tattgttata tagggaatat ttgttaaggg ggtagtttgg    2220
ttattttgat gtgtgtgtaa cgggttagtt attgattagg tgggaagtta ttatgtttta    2280
tattatttgt ttagtttttt tttttttttt gaaatggagt ttcgttttgt cgtttaggtt    2340
ggagtgtagt ggcgtgattt tagtttatcg taagttttgt ttttcgggtt tacgttattt    2400
ttttgtttta gttttttgag tagttgggat tataggtgtt tattattacg ttcggttaat    2460
tttttgtgtt tttagtagag acgaggtttt atcgtgttgg ttaggatggt ttcgattttt    2520
tgattttatg attcgtttgt ttcggttttt taaagtgttg ggaatgtagg cgtgagttat    2580
cgcgttcggt tttttttaat ttttttaaga taaggtttcg ttttgtcgtt taggttagag    2640
tgtagtggta cgattttgga ttattgtaat ttttgttttt agggtttaag ggattttttt    2700
gttttaggtt ttaaagtagt tgggattata gtcgtttatt attatgttta gttaattttt    2760
gtatttttag tagagatggg attttgttat gttggttagg ttggtttttt taatttttga    2820
ttttaggtga tttattcgtt ttaatttttt agaatgttga gattataggc gggagttatc    2880
gtattcggtt taagagttat ataaagttta aaagttgtat ttttatatac gaagtcgtta    2940
tagggttttt ttatttagta aaggtttggt gagagaagat gtatttggtg ttgtcgtttt    3000
ttcgggtttt gttgaggggga agtgacgcga tagtatagtt tttaacgtcg gatttaagta   3060
tcgatttttt ttttgtagcg ttttaagttt tcggtatata gacggtataa tgatttcgtg    3120
gtttttttagg agatgttttt gtataagttt ttttatcgta tggtgtttgt tttgttattt   3180
aagagaatgt tgggaggtaa agttaggcgt gttgtttatc gggggcggag ggagcgagtt    3240
tattttttgg atttgtggt tttggtttag ggatggattt tattgtagat cggggtaggt     3300
gtagggttgc gggggaaatt ttagtttttt ggtatttggg atagagttgt ggtttgagtt    3360
tagttttggt gtaggtaggg agattggtat tcggtagttt tggtttatta gagtttttag    3420
ttgtatgatt gataggtggg gaagtttttg ttgtgtaggt gtgaaagaga attttagttt    3480
tttttttttag agtgtagtgg ttagggtttt cgagagtttt cgagagggtt gttaggagta   3540
ttgtgtgttt atttggttgg tattgttttg tttgtatttg taaagttatg gtagtttgag    3600
ttatgggtat tatgggagcg ttcggtggtt ttatggaaga gaattgagtt gtgtaagtag    3660
gaaatgttag ttgtattttg taggagagaa aaagtagtat acggttgttt tatttttatt    3720
tttttgtttg aagatggttt cgggttaggt gggagatcgt agacgatttt agagttgttt    3780
tttaggtttg tgttttttttg ggttgtagag tttgggatgg cggttgttag ttttgatttt   3840
attggtggtt ttttttttag ttcgtttttt aaattttgaa taaaatatgt tggggaaatg    3900
agtgtttaga gttgattatg gttttttgtc gtttttagtt tttttatagt tatcgtgttt    3960
tggacgggga cgtagtatgg gagttaatat agtcgttagt ttttcgtatt tgaaatcgta    4020
cgatgatgta taaatgtttt tgttttttat cggttattga gtaagtagtt tagggtttga    4080
tgatgtgatt tttatttcgt cgtatgtgaa ggttatagag tagattattt agtttacgtt    4140
gaagggtata cgggcgttta gggtagtaga gagtagagga cgcgataggg atgtgtgatg    4200
ttggtttagg tttgggtatt cgagttgatg agcgttggtt ggcgtttagc gtttggaaat    4260
tgtatacgcg cgttgggtcg tttgcgtatt taaggaagtg tagttttatc ggaagaggtt    4320
tgttagtgtt gagttaggag ggagttttag ttatttattt gtattgtttg tttttttttt    4380
tttatggtgt tttttttttt tttttatatg gagtttcgtt ttgtcgttta ggttggagtg    4440
tagtggtgta atttcggttt atggtaagtt tcgtttttcg ggtttacgtt attttttttgt   4500
tttagttttt cgcgtagttg ggattatagg cgttcgttat tacgtttggt taattttttt    4560
gtatttttag tagagacggg gttttatcgt gttagttagg atggttttaa tttttttgatt   4620
tcgtgattcg cgtattttag ttttttaaag tgttgggatt ataggtgtga gttatcgtat    4680
ttggtttttt ttatggtgtt ttttgttttt gtttttgttt ttgagacgaa gattcgtcgt    4740
gttatttagg ttggagtgta gtggtgcggt ttcggtttat tgtaagtttc gtttgtcggg    4800
tttacgttat tttttttgttt gagtttttta agtagttggg attataggcg ttcgttatta   4860
cgtttggtta attatttttt tttgtatttt tagtggagat ggggttttat cgtgttagga    4920
tggttttaat tttttgattt tatgatttat ttgttttagt tttttaaagt gttgggatta    4980
taggtgtgag ttattgcgtt tggttttttt tatggtattt tttagagata gggtttcgtt    5040
acgttggtta ggttgatgta gaattttttg gttttaagtg atttgttttt tttagttttt    5100
taagaggttg ggattatagg tgtgagttat atttttggtt gtattttttta atatattaaa   5160
aatagtattt aaaggttggg cgttggtggt ttatgtttgt aattttagta ttttgggagg    5220
ttgaggtggg cggattattt gaggttagga gttcgagatt agtatgatta atatggtgaa    5280
attttttttt taataaaagt ataaatatta gttaggtgtg gtggcgggtg tttgtaattt    5340
tagttatttg ggaggttgag gtaggagaat cgtttgaatt tgggagttag aggttgtagt    5400
gagtcgagat gatattattg tattttagtt tgggcgataa gagtgaaatt tcgttttaaa    5460
aaaagtattt aaatattatt tttgtttgtg tttagattga aatgttttga atgtattagt    5520
atatgttttt attttttttt tttttggagt gaaaatattt aattcgaatg aaagtaatga    5580
```

gtaattgtat tttttgtaga gtaagttata agtatagtga atagaaaaat tcggtcgggc 5640
gcggtggttt acgtttgtaa tattagtatt ttgggaggtc gaggtaggtg gattatttga 5700
ggttaggagt tggagattag cgtgattaat atggtgaaat tttattttta ttaaaaatat 5760
aaaaattagt taggtatgtt ggcgggtttt tgtaatttta gttaatttta gaggttgaga 5820
taggagaatc gtttgaattt ggaaggcgga ggtagtagtg agtcgagatt gcgttattgt 5880
attttagttt gggtaatagg agcgaaattt tgttttaaaa aaaaaaaaaa gaaaagaaga 5940
tttgggttgc gttagtgtta gagcgtagta aaatttattt gtttgtagtg agaagtgtta 6000
ggaggggatt atggaagatg tttttaacgg aggtgattaa agtgattatt tacgtggttt 6060
ttaggatttt cgatggaaat aagtagattt gtggtttttt ttggtagttt ttggatttaa 6120
gtgtgtgtgg ttggaggggg tttttggggg tgaggttatt gttttgggcg gttttttttag 6180
atttattgtt tgagtagtat tgggggttta gttagtgttt tttgttagtc gttgggtttt 6240
ttgttatttt tagggagagt tttgagaatt cgtttagagt agaagatggg ggtcggcgtt 6300
ttggattttg tttagtgatt tcgtgtgttt ttttagttga tagggagttt atcgaggtta 6360
ggaggagagt tttgaagcgt ttcgttaatt tggtggcgcg atattttttg tttttcgagg 6420
atgtggtttt taagtttttt ttgtttttta gcggttcggt gagtgtgttt gttttgttta 6480
tgtttcggtt tcggggagtt ttcgagtttt cggattcgtt tttttttttt ttaggtgtgg 6540
tttttattt ttttggtttt ttgtttggac gtttagtagg aatgatatgg ataagatttt 6600
tttttttttt agataatagt ttgaatgtga tttttagagg tgtttttggt gttgattttg 6660
taggattttg taaattcgcg tggagattat ggatttttgt ggttattttg aggatttagg 6720
ttggatggtt tggagggttt agggaggtgt tagagatttt agatgggaag aagtgttgat 6780
ttaaaataag gttttgtttg attaaggatt attggaatat aggttgagtt tttttgttag 6840
agttatagtt tatatttgta ttcgggaatt aatttgttgt atatgtattt cgttagaggt 6900
ttgagtgggg tttttttttt tttagttgtt gcggtatagt tttattgtgt gttaagaagt 6960
tatagttcgt aggtatagtg gtttacgttt ttaattttag cgttcggaga ggtcgaggtt 7020
ggatggtttt ttaagtttag gcgtttgaga ttagtttgga taatatagcg agatttcgtt 7080
tttattaaaa taaaattgag gttaggtatg gtggtgtata tttgtaattt tagttatttg 7140
ggaggttgag gtatgagaat cgtttgaatt taggaggtgg agtttgtagt gagttgtgat 7200
tgtgttattg tattttagtt tgggcgatag agtgagattt tgttttaaaa aaataaaaaa 7260
taattttgta gtttagtttt tgttttttgg aggaggaagt agttgttagt ttttttaggg 7320
aaggcgggtt ttgtgttttt tttttatttg tgtttgtagg attttttttt tgtgcgggga 7380
ggggcggggg ttttattgga atagaggttt ttaggttttt ttttatttta gggaaatttc 7440
gttttgaatg gggatttgac ggtttttgtt tttgtttttg ttgtaggaag atattttttt 7500
ttttaggttg attttatata agttttggtt ttttttgttt tcgttttttt cggaggtaga 7560
ggggtttttt ttttgtgttt gagatgtttt tgggatgttt tcggggatgt ttggagtagg 7620
tagtgggatt ttttgagttt tggggttgga aatttttttt tggaggcgat ttgtattgtt 7680
agtagaggaa tgtggtagga tttgggaggg gcggtgggta ggggtgtagt gggttatagg 7740
tgggtaatat ttttgttttg ggggtaggac gttttttgtg gttttttttat aggtattatt 7800
ttttgttatt tttcggtttt agttttttttg tttgtttttt gtttttagag atggggtttt 7860
attatgttat ttaggttggt tttgaatttt tgggttttag cgatttttttc gttttaggtt 7920
ttgagtagtt gggattatag gtacgtatta tgttgggttt taggtttttt tttttttttt 7980
tttttttttt tttgtaaaat gagagttgag ttagatgatt tttaaggtat ttttgaaatt 8040
tgagaatttt tggatacgta aatagttgta tattagggtt ttttttttgta ttttttgggt 8100
ggtttttgat tttaaggtac ggttttgttt aggaggtttt ttttagatga gagggtttcg 8160
gtgttagggg ttgggtttgt ttttttgttt agtagttttt tgggttttgg tgtttttggc 8220
gttatggatg tatttttagg aaggaagata tttttgtttt gttttattgc gtttttggaa 8280
gttattaggg ttttaggtat ttttggggtt gacgtcgttt cgggtttgtg gatggagggg 8340
tcgttttttt gtagagtcgt ttttggtttg tggatggagt ggttgttttt ttggagagtt 8400
gtttttcgtt tgcggatgga gtggttgtgt ttttggagag aggttgtagg tttgtggtgt 8460
ttttgttgtg aagttaggtt gttttgtttg gtgagaaaaa tatttaaggg agagttaaga 8520
gattgtagaa tattaatgtt aaagttaata tatggaaatg tgtttttttttt gaggaaagac 8580
gatttttcgt tttatagtga tttatatttt atagtagttt ttttgttgtt agtagaagag 8640
ggtgaagttg tgtgtttttt ttttgtagga tgtgtagaat aagttaaagg agttagtata 8700
gtgcgtcggg gacgaatttt tgaattgtaa gttggttatt agggttaagg tatttttttt 8760
tttttttttt tttttttttt ttttaattta ttttttttatt gattattttt gggtgttttt 8820
cgtagagggg gatttggtag ggttatggga taatagtgga gggaaggtta gtagataaat 8880
aagtgaataa aggtttttgg ttttttttagg tagaggattt tgcggttttt cgtagtgttt 8940
gtgtttttga ttatttgaga ttagggagtg gtgatgattt ttaacgagta tgttgttttt 9000
aagtatttgt ttaataaagt atattttgta tcgtttttaa tttatttaat tttgagtgga 9060
tatagtatag ggttgggggt aaggttatag attaatagga ttttaaggta gaggaatttt 9120
ttttagtata gaataaaatg aaaagttttt tatgtttatt tttttttata tagatacggt 9180
aattattcga ttttttaatt ttttttttat ttttttcgtt tttttatttt ataaagtcgt 9240

```
tattgttatt ttggttcgtt tttaatgagt tgttgggtat attttttaga cggggtggtg     9300
gtcgggtaga ggggtttttt atttttagt aggggtggtc gggtagaggc gttttttatt      9360
tttcggacgg ggcggttggt taggtagggg ggttgatttt tttttatttt tttttcggac     9420
ggggcggttg gtcgggcggg gggttgattt ttttattttt ttttcggacg gggcggttgg     9480
tcgggtagag ggtttttttta ttttttagta ggggcggtcg ggtagaggcg ttttttattt    9540
ttcggacggg gcggttggtc gggttggggg gttgattttt tttatttttt tttcggacgg     9600
ggcggttggt tgggtagagg ggtttttttat tttttagtag gggcggtcgg gtagaggcgt    9660
tttttatttt ttagacgggg cggttggttg ggcggagggt tgattttttt attttttttt     9720
cggatagggc ggttggttag gcgggggggtt gatttttttta tttttttttc ggatggtacg   9780
gttggtcggg cgggggggtt gatttttttat ttttttttcg gatggggcgg ttggtcgggc    9840
ggggggttga tttttttttat ttttttttcg gatggggtgg ttgtcgggcg gagacgtttt    9900
ttattttttta gatggggtgg ttgtcgggcg gagaggtttt ttattttttta gatagggtag   9960
ttgtcgggcg gaggggtttt ttattttttta gacggggtgg ttgttaggta gagggttttt   10020
ttattttttta gacggggcgg tcgggtagag acgtttttta tttttttagac ggggtttcgt  10080
cgggtagagg cgtttttttat attttagatg gggcggttgg tcgggcggag ggttgacgtt   10140
ttttattttt ttttcggatg gggcggttgg ttaggcgggg ggttgatttt tttattttttt   10200
tttcggacgg ggcggttggt taggtggtgg ggttgatttt tttattttttt tttcggacgg   10260
ggtggttggt cgggtagaga cgttttttat tttttagacg gggtttcggt cgggtagagg    10320
cgtttttttat attttagatg gggcggcggg gtagaggcgt tttttatatt ttagattatg   10380
ggcggtcggg tagagacgtt ttttattttt tagatgtgat ggcggttggg aagaggcgtt    10440
ttttattttt tagatgggat ggcggtcggg cggagacgtt ttttattttt tagattgggt    10500
agttaggtag aggggttttt tatattttag acgatgggcg gttaggtaga gatatttttt    10560
atttttttaga cggggtggcg gtcgggtaga ggttgtagtt tcggtatttt gggaggttaa  10620
ggtaggcggt tgggaggtgt aggttgtagt gagtcgagat tacgttattg tattttagtt    10680
tgggtattat tgagtattga gtgaacgaga tttcgtttgt aatttcggta tttcgggagg    10740
tcgaggttgg cggattattc gcggttaggg gttggagatc ggttcggtta atatagcgaa    10800
atttcgtttt tattaaaatt agttaggcgt ggcggcgcgt gtttgtaatc gtaggtattc    10860
ggtagattga ggtaggagaa ttaggtaggg aggttgtagt gagtcgagat ggtagtagta    10920
tagtttagtt tcggtttcgt atgagaggga gattgtgggg tgagggagag ggagaggggg    10980
agggggaggg ggagggggag ggagagggtt aaggtatttt tttaattttg aagagtatat    11040
ttttttttaa ttagagaaat aagttttaat tttttaaagt gattttgttt tttttgcgtg    11100
gtagggatta tgttgatatt tgtcgtatgg ttttttttatt taattttcgt tttagttgtt   11160
tcgatatagg gttgggaggt ttgttttacg gatggagaaa taggtttagg gaggttgtta    11220
tttgttaagg tttcgagttt ttaagtagta gggtcgggtt gcggttgagt tttgttttta    11280
ggtttttttag gtatttcgtg tttttttgttt tttttttgtga tttagataga cggatgtttg 11340
tgttagtatt tttttagatt tttggggatt tattgttata tttgtttatt attaaatgtt    11400
taaagtttgg tttttttttta aaaaagatta aggtagttat ttggtagttt ttttgttttt   11460
ggttttttttt ttttaaattt tttttttagg ttggcgcggt ggtttttttt tgtaatttta   11520
gtattttggg aggttgaggt tgtcggatta tttgaggtta ggagtttgag attagtttgg    11580
ttaatatagt aaaattttat ttttaataaa aatataaaat aaaaaaggaa aataaaaaaa    11640
agtaaaataa attttaaaaa atcggtcggg cgtggtggtt tacgtttgta attttagtat    11700
tttgggaggt cgaggcgggt ggattacgag gttaggagtt taagattagt ttgattaata    11760
tggtgaaatt tcgtttttat taaaaatata aaaattagtt gggtgcggtg gcgtgtattt    11820
gtaattttag ttatttagga ggttgaggta ggagaattgt ttgaatttag gaggaagagg    11880
ttggagtgag ttaagattgc gttattgtat tttagtttgg acgatagaga tattattaaa    11940
aaaaagaaaa aaaaaattag ttaggtttgg tggtaggtat ttgtaatttt agttatttgg    12000
gaggttgagg taagaggatt gtttgaattt aggaggtaga ggttgtagtg agtttaggtc    12060
gtgttattgt attttagttt gggtaataga gtgagatttt gtgttaaaga cgataataat    12120
aataataaaa aaattagaaa aaaatttgtt tttttatata aaaattaaaa atttatttta    12180
ttttattttt tgagataagg ttttgttttg ttttttaggt tatagtgtag tggtgtaatc    12240
gtaggttatt gtagtttcga tttttttgagt ttaagtaatt tttttatttt agttttttag   12300
aatagttggg attataggta tgtattatag tatttggttg atttgtttgt ttgtttttgg    12360
tatagatgaa gttttattat gttgtttagg ttggttttga atttttggtt ttaagtaatt    12420
tttttggtat ggtttttttaa agtattggga ttaaaggtat gagttattat gtttggttaa   12480
atttttttatt tttattttat tttttgagtt ttttgttttt tgaattttga tagttagtat   12540
taaattttat aaaaagtttt tgggaataaa cgcggtatag aagaggtttt ttttttatgt    12600
ggagagtttt ttaggaattt atgtttattg tgtttaattt gaagattgtt tgtttttatt    12660
tttgttgttt tttggggata gggtttttag gttggagtgt agtgttgtga ttatggtttt    12720
ttgtagtatg gaatttttgg gtttaagtga ttttttttgtt ttagtttttt gtaagtattt   12780
gggattatag gcgttttatt acgtttggtt aattttttttga tttttttggga gagatggggt 12840
tttattatgt tgtttaggtt gtttttaaat ttttaggttt tagtaatttt tttgttttag    12900
```

ttttttagag cgttggggta taggtgtgag ttattgtatt tagttaaaga ttagtattgt 12960
tagtttttga gacggagttt cgttttgttg tttaggttgg ggtgtagtgg tgtgatcgta 13020
gtttattgta agtttcgttt tcggggttta tgttattttt ttgttttagt tttttaagta 13080
gttgggatta ggtgtttatt attaggttcg gttaattttt tgtattttta gtagagatgg 13140
ggttttatta tagtttcgat ttttttattt cgtgattcgt tttattcggt tttttaaagt 13200
gttgggatta taggcgtgag ttattgcgtt cggtaagatt attattcgag tgttttgttg 13260
gtttgtttgt tttgagatag agtttcgttt tgtcgtttaa attggagtgt ggttgcgcga 13320
ttttagttta ttgtaatttt tgtttttcgg atttaagtaa tttttttgtt ttagtttttg 13380
gagtagttgg gattataggt gtatgtcgtt atgtttagtt aatttttgta tttttagtgg 13440
agatggggtt ttattatatt ggttaggttg gttttgaatt tttgatttga ggtgatttat 13500
ttgttttagt ttttaaagtg ttgggattat aggtgtgagt gattgttttt ggttttattt 13560
gagtgttttt ggataggttt tttagtgtga tgagatttta gtggattttg tggtttttat 13620
tttatgggat tatttgtttt tgtgtttgtg gtatgtaggt gatgatggtt tttgtttttt 13680
ttttggatat atagagtgat ttttgtaggt ttggaggagt ggggttttcg gggtggttta 13740
tggatttgtt gtgtttttcg tggtttagtt tttgggggat cggttgttgt cgtttgatag 13800
gtgtttttta gcgattaata tagtgttttt tttttattta tgatgttagg attttttttt 13860
agttgatatt taggtttagt ttgttattag tcgggagttg attcggaata tttataatag 13920
tttttataag tttcgcgata gggtcgagcg gatcgtatcg cgggttatcg ataatgcggt 13980
agattttttt atattcggga aggagttaag gtaggtgatt ttttagtttt tgtaggggta 14040
cgcgcggtgg gacgggaatt tatgttggtg aagggtttgg gcggaaagtg cgtttatcgt 14100
aataattagt attttgattt gttttatttt agaaggggta gaaagtggtt taggtatatg 14160
gggggttata tttggaagaa atacgggaga atttcggttt tgaaatacgg atttttgagt 14220
acgagtaaat gtttattagt ttttagtttg gtagtttttt tattgttagg atgagatttt 14280
aaaaaaagat tttaggttag gcgtggtggt ttatatttgt aattttagta ttttgggagg 14340
tcgaggtggg tggattacga ggttaggagt ttaagattag tttggttaag atggtgaaat 14400
tcgtttttat taaaaatata aaaaaaagtt ttttgggcgt ggtggcgggt gtttgttatt 14460
ttagttattc gggaggttga ggtagagaat tgtttgaatt taggaggcgg aggttgtagt 14520
gagtcgagat cgcgttattg tattttagtt tgggtgatat agcgagattt cgttttaaaa 14580
aaaaaaaaga ttttttttgat ttggggaaga atagattatg ggttggtagc gttttttttg 14640
tttgagggga ggggagattt tattcgggat tttgtttgtt ttagtagtgt tcgttatttg 14700
tgtatatatg ttatttgggg gttggttggg aagaagagac gtagtaggtt ttgaggtgta 14760
gttttagggt tatttttttt ttcgggtttt tagggttttt ataggttatt ttttgagtag 14820
tagggtttta ggacggggtt acgtaggttg tcgtggagga tatttgtcgg gggggatgtt 14880
agattcgatt gttaggtgag ttttaggttt tgagtgtttt tttttttgtg tgtttgtttt 14940
tttagtgtaa tagggtttga tacgatttcg ttgttttttt gggtcgtttt gaatagtagt 15000
acgtgggggt ttttgaagta ggttttgaaa ggtttgtttg tggaattcgc gttgttcgtc 15060
gataaggttg tataataggt gagtggatcg gtttttttag gcgtttgttt tagagaatac 15120
gttatgtttt tgtatgggga gtgtttttttg tttagatggg cgttatagtt cgtttttgtt 15180
ttgttgtttt gagaaggagt ttttttttgt tgttaggttg gagcgtaaag gcgtaatttc 15240
ggttcgttgt aatttttgtt ttttgggttt aagtgatttt tttgttttag ttttttgagt 15300
agttgggatt ataggtgttt attattatgt ttggttaatt ttttgtattt ttagtagaga 15360
tgaggtttta ttatgttcgt taggttggtt ttgaattttt gattttaggt gatttgttcg 15420
tttcggtttt ttaaagtgtt gggattatag gcgtgagtta ttgtgttcgg tcggtttttt 15480
agttttgagt cgagtgaatg atagttgtgt tttgggtggg agttgtttat ttatatttgt 15540

ttttggtggt tttttgggag cgtttacgtt ttcgagaggg ggttgttagc gataaaa 15597

<210> 119
<211> 3143
<212> DNA
<213> Artificial Sequence

<220>
<223> chemically treated genomic DNA (Homo sapiens)

<400> 119

ttatttaaat ggggtcgcgt ttcgattatt agtgtaatgt gattatttga ttttaaacgt 60
aattatgcgt ttaaatggtt gagtgtaaga ggtcggtcgg agatagggtc gagtgcgttg 120
ttttaagttg cgatttttgt taagtattta cggataatgt attggttttt tttagggagc 180
gtttttttatt tagggtagta gtaggagtgg aatttggagt cgtatacgtt gggtttgtga 240

```
tggataagcg cggttacgta atagataggt agagggatgt agtaatatat aaacggtttt     300
cgtaagtaat gtttcgagcg agtagatttt aggataataa ataaattaaa attatatttt     360
aaattattgg agatcgggag gtagacgtgg aagtaaaaga gttagatgtt ggaatgtagg     420
agggttagaa atgtttaaat attgtttttt atttgtcgtt ttttgtttgt ttttttaaag     480
ggcgaaaaga ggtatggaaa tatataattt gttattattt tagtttttag atgtagagaa     540
taaagggatt atttcgtttt aaagaatttt tgtttttttt tttaaattta ttttttttttg    600
gttaattgtt attttggttt cgacgtgtta aggaaagcgc gtcgtaaggt ggagcgttcg     660
ggttgacgat gagaaggtga gttgtgaatt ttgtatacgt ttttaaataa aaggtttacg     720
agcggcggtt tcgacgtggg gagtaagtag gtttttttgg tttttgtagt aggcggattt     780
aggttcgttt gtcgtcggat atttagaatt tgttttcgta gcgcgagttt tgtatacgtt     840
ttgcgatcgg tatttttgga ttttttaatt attcgtcgtt taattttcgg agagagggtt     900
tttcgttcgt tgagttgcgg ggttgggaag gaaaaggggg acgcgttttt gggggttttt     960
ggatattttg gtaaaggggg ttgtagtttt aggttttagt taaagggttt gggacggagt    1020
gggttagaaa gtcgttaaat tgaggatttt tttttttttt aagtcggagt tttaggtgcg    1080
gagtcgtttg tttatttatt ttcggagtac gttttgttgt tttcgtcggc ggacgcggtt    1140
ggttattgcg gtattttttt cgcgatattc gggcgttaga aattcgtttt tagagtttgt    1200
tgtagcggat cgaatttgtt gcggcggcgg ttggggtcgg ggcggtttgg gcggaggagg    1260
ttgaggttgg tcggtcgtgt tttgcgttta gagtattata tagtttagtt ataggaagta    1320
gagttggaga tatagttttc gttttttttt tatcgtcggc gtcgaaagcg cgcggtcgta    1380
gattcgcgga gagggagtga ttaaagataa aatttgcgcg ttttagaaaa gagttagggt    1440
ttaataagtt tttcgtcggt ttcggggatt ttttcgggtt cgggtttttt gttttagttg    1500
gagatttcga tagagcgtcg gtttttttgat tgttcgcgaa gcgagacgcg gggcgtcggg   1560
tttagcgtag tgagcggcga ggcgcggcgg aggtgtagtc ggtagtttcg agcggtatac    1620
gttgtataat cggtttaaag tttcgagcga ttttaggatc gttagcgggc gggggaggaa    1680
attataaaag ttttggcgta aaattttaaa aaatgtgaat tttaaaagaa aaaagggttt    1740
gtacgtagag ttcggcggat tttcgtgcgt ttcgaagacg gatattggaa agttgtaaaa    1800
gttattagaa agaaataggt aagtaaataa aagtcgtttt tagaagaaga ggagggtttt    1860
tttcgggttc gttcggagtt ttatagggta acgaagcgcg ggtagcggtt gcggagtcgg    1920
gcggaggtgc gcggggtcgg ggcgtgcggt tagtaagaga gagggcggga ggttgggcgt    1980
tggagaagat gcggcggtgg agggcgcggc gcgggggttt tagaggttcg gcggggcgcg    2040
gggcgggtcg gcgggcggcg gttgttagtg ggggcgcggt cgtttatacg tggggtttaa    2100
ttcggattga gaggtcggcg tagagtgggc ggggatagag taagggagag atcgggattg    2160
aggaacgagg ggaattaggg ttgggtgagg ggaagaggaa ggaagggggg gagggcgaag    2220
ggagggaaga ggacggagat tggggagaag gggggcgaat tcgagaggaa ggtcgggttg    2280
gaagagggat ttggaaagag ggattggcga gttcgggtgc gagaggaaat gagcgggggga   2340
tgggggcgaa gttagggtaa agagagaagt gggaaaggag agggagggtt aaggttcggg    2400
cgtcgagagg aggggttcgg ggggtcggtt ggagggggcg aggcgttagg cggttttgtt    2460
tttcgttttc gttaattggt cgttagtttt gttttgattg tttgtataaa gcgagtagac    2520
ggcgggggat ggggcgatta gggatcggat ttgggaaagg aggtagtagt tttttttttt    2580
tttttttcgt tttcgttcgg tcgcggatta gggattaggg tgtttaagcg cgttaggttt    2640
ggtcggattc gtagttgaat tgattcgtag ttgaattttt tcgtcggata gaggtcgtaa    2700
ggttgaggat gagaacgggt ttttttgtag ggtttcggtt tttgatttgg gatgggggga    2760
ttcgtagtta gttaggaata gaggtttacg gggttgttgg ttgtagtttt aatttaagaa    2820
ggaggagatt tttttttttgt tttttttattt ttatcgtttt atcgggagcg gcgttagttt  2880
gcgggattaa attaggggtt gggagttttt agattgaaat gcgtttttta ttattaagag    2940
ggagtttacg cggtcgcggg gggtcgaggt agttagtcgg cgtcgcggtt ttggggagtt    3000
cgtgtagggg tgtgttttgg tgtgaaattt ggtaggattt tgtttgggtt tgtatgtagg    3060
gaggggttag agattttgaa atgtattagc gggtagggtg gtgataaggc gggtaaatga    3120
ggttcggggt atgtttttgt ttt                                             3143
```

<210> 120
<211> 3143
<212> DNA
<213> Artificial Sequence

<220>
<223> chemically treated genomic DNA (Homo sapiens)

<400> 120

```
gaagtaaggg tatgtttcga gttttattta ttcgttttgt tattattttg ttcgttagta      60
```

```
tattttagag tttttaattt ttttttgtat gtaagtttag gtagagtttt gttaggtttt     120
atattagagt atatttttgt acggattttt taggatcgcg gcgtcggttg gttgtttcgg     180
tttttcgcgg tcgcgtgagt ttttttttgg tggtggaggg cgtattttaa tttggaaatt     240
tttagttttt aatttggttt cgtagattga cgtcgttttc ggtggggcgg tgggggtagg     300
agggtaaagg ggaggttttt ttttttttgg attgaggttg taattaatag tttcgtgagt     360
ttttattttt ggttgattgc gaattttttt attttaggtt aggggtcggg attttataga     420
aagattcgtt tttattttta attttgcggt ttttgttcgg cggggaagtt tagttgcggg     480
ttagtttagt tgcgggttcg gttaagtttg gcgcgtttag gtattttagt ttttggttcg     540
cggtcgggcg aggacggagg gggaggggaa ggggttgttg tttttttttt taaattcggt     600
ttttaatcgt tttattttc gtcgtttatt cgttttgtat aggtagttaa aatagagtta     660
gcggttaatt gacggggacg ggaagtaaag tcgtttagcg tttcgttttt tttagtcggt     720
ttttcgggtt tttttttcg gcgttcggat tttggttttt tttttttttt tttatttttt     780
tttttgtttt aatttcgttt ttatttttcg tttatttttt ttcgtattcg ggttcgttaa     840
tttttttttt taagtttttt ttttagttcg gttttttttt cgggttcgtt tttttttttt     900
ttaattttcg tttttttttt ttttttcgtt tttttttttt tttttttttt ttttttttatt     960
taattttggt ttttttcgtt ttttagtttc gatttttttt ttattttgtt ttcgtttatt    1020
ttgcgtcggt tttttagttc gggttgagtt ttacgtgtgg acggtcgcgt ttttattgat    1080
agtcgtcgtt cgtcggttcg tttcgcgttt cgtcgggttt ttaaaatttt cgcgtcgcgt    1140
tttttatcgt cgtatttttt ttagcgttta gtttttcgtt tttttttttg ttggtcgtac    1200
gtttcggttt cgcgtatttt cgttcggttt cgtagtcgtt attcgcgttt cgttgttttg    1260
tgggatttcg agcgagttcg gagggaattt tttttttttt ttgggggcga tttttgtttg    1320
tttgtttgtt ttttttggt gatttttgta gtttttttaat attcgtttc ggagcgtacg    1380
ggaattcgtc gagttttgcg tgtaggtttt tttttttttt gaggtttata tttttttgaaa   1440
ttttacgtta gggtttttgt aatttttttt ttcgttcgtt gacggttttg gagtcgttcg    1500
gggttttagg tcggttatgt aacgtgtatc gttcggggtt gtcggttgta ttttcgtcgc    1560
gtttcgtcgt ttattgcgtt agattcggcg tttcgcgttt cgtttcgcgg gtagttaggg    1620
ggtcggcgtt ttgtcgaggt ttttagttag agtagggagt tcgagttcga gggagttttc    1680
ggagtcgacg aagggtttat tagattttga tttttttttg aggcgcgtag attttgtttt    1740
tgattatttt tttttcgcgg gtttacggtc gcgcgttttc ggcgtcggcg atggggagaa    1800
gacggaggtt gtgtttttag ttttattttt tgtggttggg ttgtgtggtg ttttgggcgt    1860
agggtacggt cggttagttt tagttttttt cgtttaagtc gtttcggttt tagtcgtcgt    1920
cgtaataggt tcggttcgtt atagtaggtt ttgaaggcgg gtttttagcg ttcgagtatc    1980
gcgaggaggg tgtcgtagtg gttagtcgcg ttcgtcggcg aggatagtag gacgtgtttc    2040
gagggtaagt gggtaagcgg tttcgtattt agggtttcgg tttgggggag gggggaattt    2100
ttagtttggc ggttttttgg tttatttcgt tttagatttt ttagttggag tttagagttg    2160
tagtttttttt tgttagaata tttaaagatt tttaggagcg cgtttttttt ttttttttta   2220
atttcgtagt ttagcgggcg gaaagttttt ttttcggggg ttgggcggcg ggtggttagg    2280
gggtttaggg gtgtcgatcg tagagcgtgt gtagagttcg cgttgcggga ataggttttg    2340
aatgttcggc ggtaggcggg tttgggttcg tttgttgtag ggttagagaa agtttgtttg    2400
ttttttacgt cggggtcgtc gttcgtgagt tttttgtttg aggacgtgtg tagggtttat    2460
agtttatttt tttatcgtta attcgagcgt tttattttgc gacgcgtttt ttttgatacg    2520
tcggggttaa agtaatagtt gattaaggag gaatggattt gggaaggagg gtaaggattt    2580
tttggaacgg aatggttttt ttgtttttttg tatttggaag ttagaatagt agtaaattat   2640
atgtttttat gtttttttttc gtttttttaaa aaggtaggta agggacgata gatgaaaggt  2700
agtgtttaga tatttttgat ttttttgtat tttagtattt agtttttttg tttttacgtt    2760
tgtttttcga tttttaataa tttgaagtgt aattttgatt tgtttgttgt tttgaaattt    2820
attcgttcgg ggtattgttt acgaagatcg tttatatgtt gttgtatttt tttatttatt    2880
tgttacgtga tcgcgtttgt ttattatagg tttaacgtgt gcggttttag attttatttt    2940
tattgttgtt ttggatggaa gacgtttttt ggaggaaatt agtgtattgt tcgtgagtgt    3000
ttggtaggga tcgtagtttg gggtagcgta ttcggtttta ttttcggtcg attttttgta    3060
tttagttatt tggacgtata gttgcgttta aaattaagtg attatattgt attgataatc    3120
gggacgcggt tttatttgaa tag                                             3143
```

<210> 121
<211> 6676
<212> DNA
<213> Artificial Sequence

<220>
<223> chemically treated genomic DNA (Homo sapiens)

<400> 121

```
gattaaacga aatatcgtaa tataaggcga ggtaggtttg tgggaatcgg tttttagcgg      60
gcggcggtag agcggggagg attggagggg ttagggttgg gggtggtacg ggtttttggg     120
tcgttgggtt tttttgggcg ggtttagggt tggaaggcgt tgttagttgt agttaggttt     180
atatttttta atttgttgtt ttttttttat tttatgcgtc ggttttggaa ttagattttg     240
atttggcgtt cggataggta gagtgcgtgg gcgatttcga tgcgtcgtcg tcgggttagg     300
tagcggttga agtggaattt ttttttttagt tttagggttt ggtagcgggt atacgcggtt    360
cgggtttttt tttcgttcgg ttcggttata tttggagtag atagaggaaa gtcgtaaggt     420
aatattattt cggttaaggg agggggtatt gggtgggagg gggcggggga gtaggattta     480
ggcgtttcgg tatttagttt attatagttt taattttttt tattttagat gtttatattt     540
aagttcgatt tttgtatttt tttttattgt atttttttatt tttgtaggta gttagtgcgt    600
tcggttttgt tttttgttcg tttttagtgt tttatttttg gttgggttta ggcggtcgtt     660
cgtttcggga gggaggagag cggttgtatt tttgtttcgg aaagtttttt ttattgttta     720
tttttttttt gttagcgata gttttaaaat tttaggggta agtaggattt ttttttatat     780
tatatgaatt ttattttttta ttttaaagtt ggtttagttg cgttaataaa aatttaagtc    840
gaagttggaa ataaattatt aaaaatattt tttttttaat agagagattt tttttggttt     900
tgtttacgaa gttatgaggt ttttttttaga tattttttttt ttttagaaaa atgaatttat   960
atttagggta aagttaagtt tttttttgttt tttcgatttt attttaaaac gtagagaagg   1020
aaagtcgaga agataaaaaa gagagagaga attattatgg ttgatgtgaa gttttttttat  1080
ttaggggaat atttgcggag tttgtttttc gggcgcggtt gtggaggtgg ttatgggttt    1140
tggttgcgtt cgagttaggt tggggttgtt tagttggttt gtcgtttttt taggtttcga    1200
ggacgcgttg gtttcgttta tttcggtgaa attggcgttg gagttggttg aggtcgtttg    1260
gtcggagggg gacgaagtag agggtttggc gtcgtggttg tcgtcgttgg tgtagggtag    1320
ggattcgggc gaggataggg agtagttcga agtcgtttgt ttgaagcggg gtttttgggc    1380
gggcgcgggg aaggcgcgcg agttttcgtt tatcgtttta aagtggttgg aggaggtcga    1440
ggagcggttg acgttgaggt ttattttatt gtaattgtag tcgtaagagt cggtgtgtat    1500
ggtagcggga tttttgtaag agtcgtttag agagttgtta ttgttataat ttagtaattg    1560
atagttcggg ttatttggat aacgtttcga gaaggagttt ataaagtacg agtttatttg    1620
ggtgattttg gagggtttga tttgtggatc gtggtcgtta taattttgtg ttttacgatt    1680
tatgatgtat taatgaatta tagcgatgta ttgtatttcg ttttgttatg tatgcggtta    1740
aatatggggg gggggttgga agggggggtgg ggggcgttag ggaattacgt gtttttgttg   1800
attagtcgta aattttcgtt gatgatttta agaggtaatt tatgttttat ggttataaat    1860
aatgacgatt cgagaatcgt tagggtcgat ttaatgcgag ttttcgagag agggggaaaa    1920
aaggagaagg gggagaaata gagagaaggt tggttttggt ttttgagtag agcgcgttat    1980
ttttcggcga tcgacgggag cgcgggcgtg agatcgttat ggcggcgggc ggttgttttt    2040
ttgttggttt tttgtaggtt ttttgtagtc gtcgggagag aaagaaattc ggttaaggtt    2100
tagttatagg gttgagtttt ttagagtagg ggttgggttt tttatttatt tttttgtggg    2160
tggggttagt tttttttattt ggtttttttt ttttatatta ttattgtcgt tattatttta   2220
attttggatg agaagattaa agatagagtt gttttttttt tttttgggtt ttgtaagttt    2280
tcgaattgga ggcggaaatg ggggaagggt tggaagttgt gtgtgaaaaa taattattgt    2340
aacgggtttt ttttaggagt ttagttcggt ttgtattttg gtggaattgt tgttatcggt    2400
ttatagcgtt taggtcgagt cggtagggtt gggcgggagg tttgaaaaag aaggagaagc    2460
gtttattttt tttgggttat ttggaagttc gaaatttttt ttcggatttt tttggtggtt    2520
tttagtttag gcgtggggag tgatagagta ggatagggat tttagaaatc gagtatagtt    2580
ggggttaagc ggtttagttg tcgaaaagat tgtagcggtt atcgcgtttg ttgtttagtt    2640
tgagtagtaa tatgtgtttg agagtttgta aagcgggatt tttttttttt tttttcggtt    2700
attgtattcg gaggttttat tataggtttt ttattcgtag ttaggtagtt tgagaatttg    2760
tttggtggta gtcgtttttt cggaaaaggg gttttaagga ttcgtatgtt ttgttgagta    2820
cgttgggcgg ttagagatcg tcgtagttgg tttttttatta ggaggggatt atttttttta   2880
aaaataagaa aggaagcgag agggcggagg aataaaggaa aatagtgatc gtagtttgtt    2940
ttttgggtgt cggtgtaagt agagagagaa tgagttattt tgggttgaga ggatgatatt    3000
aaaagtttta ttatttggtt ttttgttttg gggtttttaa ggagtaaggg aaagttgtgg    3060
ttttcggttt gtgtttggtt ggtggtttta tatttttttta tttgaggttg atttttttatt   3120
tgaggttgat ttgttatttg aggtgggaag gggtatttgg agtttttagt tgtagggtga    3180
gagatattta gtggttttat tttttagttt ttagagttat aggttaggag cggagggtta    3240
aggttggcga aaaaatttgg ttatgtggac gggttaaaga ttaggggtcg ttgcgaaggt    3300
gaggatagaa aagcgttgta gaggttttta gattatggtt tcgttttcgt tagagagttt    3360
taggggtttt agtggttttt ttagtgttgt gtatatacga ggaaaggtta gagaatgaga    3420
gggatatagt attttttttat tttttaagtt tgtacgggga gaagcggttg tgagttttag  3480
```

```
tattagggaa acgtagcgtt ggggtttggg taggattgat cgttttagtt tttattttag    3540
ttgtaatttt ttagtcgaat agggttcgtt tcggcgggtt agggttttcg tttttgttga    3600
agaattttgg tttaggaaag atggagaggt tgggggttga ggagagagga aaaaatggta    3660
ggggaggatt ggaggtgatc gagcgtcgag tttttatatt tttattattg ttgttgttgt    3720
tgttattatt attattatta ttattattat tattattatt attatcgaag tattttacgt    3780
ttagagttaa gataagatta taaatataat atatagaaaa ttaataaaat agaattttgt    3840
tttttttga ggttttttttt tttattttta ggtagtatgt gttttttttta gtggttttgg    3900
ggaggggggtg ggagagacga taggtttggg attagggagt ttttaaggtt ttcgttgagg    3960
ggatagcgaa tttattattg aatcgtgtac gggggatatg gataaaatga gacgcgatag    4020
ggataagagt attttgtttt gtttttagga aatattaagt gagttcgttt tttagttttt    4080
attaggaagt ttgattaggt tgaggcgagt ttttcgggaa ggaagggcgc gcgggatgtt    4140
gggtgggttc gagtagtgag gttttagagt attcgtcggg agttgtgcgg gcgggggcgt    4200
ttaggagagc gttgggtttc gtttgtttgc gtcggagagt aggtttttttg gtttttttat    4260
tcgagagttt tttttttcgg gttttttttga cgttttcggt tttttatagg tttttttttt    4320
cgcgtttttt tttttttttt agtattttta ttcggtttgt tttttttttt ttttttcggt    4380
attgagttga gacgcgttga gtagtttgtt tttttttttt tattttatgc gtcggttttg    4440
gaattatatt ttgatttgtt ttttcgttag gtatagggcg tgcgcgattt cgatgcgtcg    4500
tcgtcgcgtt aggtagcgat tgtagtgaaa tttttttttt agttttagcg tttggtaacg    4560
tgtgtatgtt tggcggtttc gtcggtcgtt gggtttaaag gaggaatttg ttacgtagag    4620
tggagatgtt gaggtttgcg gttatcgggt ttaggatttt ttttttttagt cgattttcga    4680
atatagattt tagttagtat cgggatgttt tttattttgt cggttttttt ttttcgtttc    4740
gtattttttt agcgtttttt ttagattttt ttttagtttt ttaggtttgt gtcgtttgtt    4800
tagattttag atgggggagg ggaggagtag tttgaatttt tatttgagtt tgggggggagg    4860
ggttggttag gtgtgttttt ttttagttgt atttgtttt tttttttttt tttatttatt    4920
ttgtttttat ttttcgttat tttttttaatt taatgataaa tttaggtcgt taattcgtaa    4980
tgacgtagat cgatttatag tttatattaa cggtttttta ttttcgagtt cggttaatgg    5040
atattagttg ggatttaagg ttaataaata atttaatttg agatttcgcg tttgtttttt    5100
ttttttcgt ttcgtttgtt tttttttttt tttttttttt tatttttttt tttttttttg    5160
attattattt tttttttggt gttttatttt gttttagttt tttatttata gggaaatata    5220
gttttagata gatttaattt tttttttttta gcgttatttt ttatttttttt gtatgacgta    5280
tttcgtttt aatggagtcg tttttggttg gggaatttta ttagggttgg gagagggggg    5340
ttgggggttt aaagtaaagt taagttagtg attgtaggtt tttgtttttt tttttgtttt    5400
ggaatttcgg ttttggttag gcgttttagg agagtatatt tgtttttat ggtttttagt    5460
gcggttggga ttttgtaaat tttgtaattt ttgtttaata ggggatatag cgagagattt    5520
tcggtcggcg tttaattttt ttttattttt taattgagat gtttatttag tattttgtgg    5580
gagattgggg tagggggcgt ttattagttt tgtgtttcgg ggtgggcgtt taggggagtc    5640
ggggcgacgg cgacgggaag ttttatttat tgttgtacga atttattcgt tgtatttacg    5700
ggtagatcgg agtggagtat ttttgttttt ttgtttcgtc gaatacgttt ttgttttgcg    5760
cgtagttcga ttttcgcggt tcggggtgga acgggggttg ttcgtcggcg tcggagagtg    5820
cgtaggtcga ttttttttttcg cggtagaagg tcggcgtcgg ttcgtagtcg tagggcgtcg    5880
ttcggtcgta gttatcgttc gtcggcgggt aataggagga agtggtaaag tttttgtttt    5940
ggttcggttt tggttcgtag ggcgcggggt aatgttttag cgggttcgta tagttcgacg    6000
aatagagcgg tagttggttt aggaaggatt tttgttcgtt ggttagagtg acggggaagg    6060
tggagtttac gaaataggaa tttattggga ggggaggcgc gcgcggtcgt tgttgtttcg    6120
tcgggtttat gatttgttgt gttttatagt tcgagcgtcg cgtttattag tagtatattc    6180
gagattgggt tttagttgag gggggatggc gaggtgttag tgagggttag aaggaaatat    6240
aattatttga tttaatattg attaatggta gaggtaggaa ttgttaaata gtatttagga    6300
ggagcgtagt tcgtacgagg gattcgcgta taaatttatt aattttttttt tttttttttt    6360
ttttttttcg tttttttttt tttttttttt aataatataa cgcgttttgt tttatttacg    6420
taggatttgt gaaataggtt tagtgttttt gagggaggag ggaacgttta gattgagatt    6480
attttatttt cgggtgatta aataaatatg tttttttttt attttacgta ggattggaaa    6540
aagttaattg tgtttttaga aaagttaatt tttgcgttgt ttcgattttt ttttttcggg    6600
gagggttggg taaatgtttt cgaatcgttt tgagagagaa ttaaagaagg tttgttttta    6660
gggttttgtg tcggta                                                    6676
```

<210> 122
<211> 6676
<212> DNA
<213> Artificial Sequence

<220>

<223> chemically treated genomic DNA (Homo sapiens)

<400> 122

```
tatcgatata aaattttaga aataaatttt ttttagtttt tttttaaggc gattcggaga      60
tatttattta atttttttcg gagagaaggg gtcgaagtaa cgtaggggtt aattttttttg    120
aaaatataat taatttttt taattttgcg tggggtgggg gagagatata tttatttaat      180
tattcgggag tggggtggtt ttaatttaaa cgtttttttt tttttaaag gtattgagtt      240
tgttttatag attttacgta ggtggaatag gacgcgttgt gttgttgggg ggagagagga     300
gaaaggcgaa ggagaaggag gaaaaaaaaa aaggttgata ggtttgtgcg cgggtttttc     360
gtgcgggttg cgtttttttt gggtgttatt tgataatttt tgtttttgtt attggttagt     420
gttggattag atggttgtat ttttttttgg tttttattgg tatttcgtta ttttttttta     480
gttaaaattt aatttcggat atattattaa taggcgcggc gttcggatta taaaatataa     540

taaattataa attcggcgga gtagtagcgg tcgcgcgcgt tttttttttt aatgagtttt     600
tatttcgtga atttatttt tttcgttatt ttggttagcg ggtaggagtt ttttttgggt      660
tagttatcgt tttattcgtc gggttatgcg gattcgttga gatattattt cgcgttttac     720
gggttagggt cgggttagga taagggtttt gttatttttt tttattattc gtcggcgggc     780
ggtggttacg gtcgagcggc gttttgcgat tacgggtcgg cgtcggtttt ttatcgcgag     840
aaagagtcgg tttgcgtatt tttcggcgtc gacgagtagt tttcgtttta tttcgagtcg     900
cggaagtcgg attgcgcgta ggataagagc gtgttcggcg agatagaaga gtagaagtgt     960
tttatttcgg tttattcgtg gatgtagcgg atgaattcgt gtaatagtga gtgagatttt    1020
tcgtcgtcgt cgtttcggtt tttttgggcg tttatttcgg gatatagaat tagtgagcgt    1080
tttttgtttt aattttttat agggtgttgg gtgggtattt tagttaggag atagaagaag    1140
attgggcgtc ggtcgggggt ttttcgttgt gtttttttatt aggtaggagt tataaagttt   1200
gtaaagtttt agtcgtattg ggagttatgg ggagtaagtg tgttttttttg gggcgtttgg   1260
ttagagtcgg ggttttagga tagggaaggg agtaggagtt tgtagttatt ggtttggttt    1320
tattttgagt ttttaatttt ttttttttag ttttggtagg gtttttttaat taaagacggt   1380
tttattaaaa acggagtgcg ttatgtaagg gggtaggggg tgacgttgaa gagaaagagt    1440
tgagtttgtt tgggattgtg tttttttgtg ggtggggagt tggggtaaga tgaggtatta    1500
ggaaaggggt ggtagttaga ggagggagga ggaataaggg gaaggaagag agagggagta    1560
ggcggagcga gagagggaga aataggcgcg gggttttagg ttggattatt tgttggtttt    1620
aagttttaat tgatgtttat tagtcggatt cgaaagtgag gagtcgttaa tgtggattat    1680
ggatcgattt acgttattac ggattaacgg tttggattta ttattgggtt ggggggatga    1740
cggggggtgg gaataagatg gatggaaagg gagggaaaga taagatgtaa ttaggaagag    1800
atatatttga ttagtttttt tttttaggtt taggtgggag tttaaattgt tttttttttt    1860
ttttatttag agtttagata gacggtatag gtttaggaga ttaggaggga atttggaggg    1920
ggcgttggag gagtgcgaaa cgggggaagg gagtcggtag aatagagggt atttcggtat    1980
tggttggagt ttgtgttcga gggtcgatta ggggaggggg ttttgggttc ggtgatcgta    2040
ggttttagta tttttatttt gcgtaatagg tttttttttt gggtttagcg gtcggcgagg    2100
tcgttagata tatatacgtt attagacgtt ggagttggag aaggagtttt attataatcg    2160
ttatttgacg cggcggcggc gtatcgagat cgcgtacgtt ttgtgtttga cggagaggta    2220
gattaagata tggttttaga atcgacgtat gaagtggaaa aaggagagta aattgtttag    2280
cgcgttttag tttagtgtcg aggaggagga agaaaaatag gtcgagtgaa ggtgttggaa    2340
agggagggag gacgcgaggg gaaaggtttg tggggagtcg agggcgttag agagattcgg    2400
gaaggaaggt tttcgggtgg gggagttagg agatttgttt ttcggcgtag ataggcgggg    2460
tttagcgttt ttttggacgt tttcgttcgt atagttttcg gcgggtgttt tgaggtttta    2520
ttattcgagt ttatttagta tttcgcgcgt tttttttttt cgaggaattc gttttagttt    2580
gattaggttt tttggtgaga attgaggagc ggatttattt gatgtttttt ggaagtagag    2640
taaaatgttt ttgtttttgt cgcgttttat tttgtttatg tttttcgtgt acggtttaat    2700
ggtagattcg ttgttttttt agcgggggtt ttgaagattt tttgatttta gatttgtcgt    2760
tttttttatt ttttttttaa agttattgga aggagtatat attatttaga agtaagaaga    2820
ggagttttag aagaaaataa agttttattt tattaatttt ttatgtgttg tgtttgtagt    2880
tttgttttag ttttggacgt gaaatatttc gatgatgatg atgatgatga tgatgataat    2940
aataataata ataataataa taataataat aataaagatg tgaaaattcg acgttcggtt    3000
atttttaatt tttttttgtt attttttttt ttttttttaa tttttagttt ttttattttt    3060
tttgagttag aatttttttag taaaggcgag agttttggtt cgtcgaagcg agttttgttc   3120
gattgggaag ttatagttga gataaaggtt ggagcgatta gttttgttta ggttttagcg    3180
ttgcgttttt ttggtgttga ggtttatagt cgtttttttt cgtgtagatt tgggggatgg    3240
agaggtgttg tgtttttttt attttttagt ttttttcgt gtatatatag tattaaggga     3300
gttattgagg tttttaaagt tttttggcgg agacggagtt atagtttggg ggtttttgta    3360
```

```
gcgttttttt gtttttattt tcgtagcgat ttttggtttt tggttcgttt atatggttag    3420
gttttttcgt tagttttggt ttttcgtttt tggtttgtgg ttttggagat tgaaaaatgg    3480
ggttattggg tattttttat tttgtagttg aaggttttag gtgttttttt ttattttaag    3540
tagtagatta gttttaagta ggagattagt tttaagtagg ggaatgtaaa gttattagtt    3600
aggtataggt cgaaggttat aatttttttt tgttttttgg agattttagg gtaggaggtt    3660
aggtgatggg gtttttagtg ttattttttt aatttagaat ggtttatttt tttttgtttt    3720
gtatcggtat ttagaaggta agttgcggtt attgtttttt tttatttttt cgtttttttcg    3780
tttttttttt tatttttaaa gaaaatagtt ttttttaat aggagattag ttgcggcggt    3840
ttttggtcgt ttagcgtgtt tagtaaagta tgcgggtttt tggaattttt ttttcgggaa    3900
ggcggttgtt attaggtaaa tttttaaatt gtttagttgc gagtgaggga tttgtagtgg    3960
ggttttcgaa tgtaatagtc gaggaggagg ggaagggatt tcgttttata aatttttaga    4020
tatatgttat tatttaaatt aagtagtaga cgcggtggtc gttgtagttt tttcggtaat    4080
taggtcgttt agttttagtt gtgttcggtt tttggggttt ttgttttgtt ttgttatttt    4140
ttacgtttgg gttaaggatt attaaggagg ttcggaaggg gatttcgaat ttttaagtgg    4200
tttaggagaa gtgaacgttt tttttttttt tttaagtttt tcgtttagtt ttgtcggttc    4260
ggtttgggcg ttgtggatcg gtaatagtag ttttattagg atgtaggtcg gattaagttt    4320
ttggggaaaa ttcgttgtaa taattgtttt ttatatataa tttttaattt ttttttttatt    4380
ttcgttttta attcggagat ttgtaaagtt taagaaaggg aaaagtaatt ttatttttga    4440
ttttttttatt taaagttaaa ataataacga taatgatgat gtagggagaa agaattaaat    4500
agagaggtta attttattta taagaaagtg ggtaaaaagt ttaattttttg ttttaaaagg    4560
tttaattttg tggttgagtt ttggtcgggt tttttttttt ttcggcgatt gtagggagtt    4620
tgtagggagt tagtagggag gtagtcgttc gtcgttatgg cggttttacg ttcgcgtttt    4680
cgtcggtcgt cgggaggtgg cgcgttttgt ttagaggtta aagttaattt tttttttgtt    4740
tttttttttt tttttttttt tttttttttt cggaggttcg tattgaatcg gttttaacga    4800
ttttcggatc gttattattt gtaattatag agtatgaatt atttttttgag gttattagcg    4860
agaatttacg attggttaat aaaagtacgt gattttttaa cgtttttttat tttttttttta    4920
attttttttt tatatttggt cgtatatata gtaaaacgaa gtatagtgta tcgttataat    4980
ttattaatat attataaatc gtgaagtata gggttataac gattacgatt tataaattaa    5040
gttttttaaa attatttaaa tgagttcgta ttttgtaaat ttttttttcgg ggcgttattt    5100
aaatggttcg gattattagt tgttaaatta tggtagtggt agttttttga gcggttttta    5160
tagggatttc gttgttatgt atatcggttt ttacggttat aattataatg ggatggattt    5220
tagcgttaat cgtttttcgg tttttttttag ttattttggg gcggtgggcg agagttcgcg    5280
cgttttttttc gcgttcgttt aggagtttcg ttttaggtaa gcggtttcga gttgtttttt    5340
gttttcgttc gagtttttgt tttgtattaa cggcgatagt tacggcgtta agtttttttgt    5400
ttcgtttttt ttcgattagg cgattttagt tagttttagc gttaatttta tcgaaataga    5460
cgaggttagc gcgttttcgg agtttgagga agcggtaagt tagttaagta gttttagttt    5520
agttcgggcg tagttagagt ttatggttat ttttatagtc gcgttcgagg ggtagatttc    5580
gtaaatattt ttttggatga ggaagtttta tattagttat ggtaattttt tttttttttt    5640
ttgtttttttc ggtttttttt ttttgcgttt tggggtggga tcggggggat aaggagagtt    5700
tggttttatt ttaaatatag atttattttt ttaaggggga gaagtattta agaggggttt    5760
tatagtttcg tagatagagt taaaaagggt ttttttgtta agaggggggt atttttaata    5820
atttgttttt agtttcggtt taggttttta ttagcgtagt tgaattagtt ttaggataaa    5880
ggatggggtt tatataatgt ggggaagggt tttgtttatt tttgaaattt tgagattgtc    5940
gttagtaaag aaggggtgaa tagtaaaagg agtttttcgg ggtaaaagtg taatcgtttt    6000
tttttttttc ggggcgagcg gtcgtttgag tttagttaag ggtgaggtat taggagcgag    6060
taagagataa agtcgggcgt attggttgtt tgtagaagtg ggggatgtag tagaaagggg    6120
tgtagaaatc gagtttgaat gtgggtattt ggagtagaga ggattggagt tgtggtgagt    6180
tgggtgtcgg gacgtttggg ttttgttttt tcgttttttt ttatttagtg tttttttttt    6240
taatcggaat aatgttgttt tgcggttttt ttttatttgt tttagatatg atcgggtcgg    6300
acgggaaaag ggttcggatc gcgtatattc gttattagat tttggagttg gaaaaggagt    6360
tttattttaa tcgttatttg attcggcgac ggcgtatcga gatcgtttac gtattttgtt    6420
tgttcgagcg ttagattaag atttggtttt agaatcggcg tatgaagtgg aagaaggata    6480
ataaattgaa aagtatgagt ttggttatag ttggtagcgt tttttagttt tgagttcgtt    6540
tagaggagtt tagcggttta agagttcgtg ttatttttag ttttggtttt tttaattttt    6600
ttcgttttgt cgtcgttcgt tggggatcgg tttttataag tttgtttcgt tttgtgttac    6660
gatatttcgt ttggtt                                                    6676
```

<210> 123
<211> 11656
<212> DNA
<213> Artificial Sequence

<220>
<223> chemically treated genomic DNA (Homo sapiens)

<400> 123

```
gttatcgttt ggtaggggtt cgtttttttt tttggtgttg tttattcgcg aggtttgaga      60
cgcggatcgt tattttttc gggtttagtt tggttttaat gtaggtttta gagagaggga      120
taaaattcgg ttcgtttttt tttttttttt aaggcgatta ttagtgtttt gggggtttat     180
agggtttttt tagttttgat tcgagttttc ggttgtagtt ggtttgggtt tgtgggggtt     240
tcgcgtaggg aagcggaggt ttttttttta ggtttttata tttttttggt ttgttttagt     300
agagttagcg gtttttcgag atagttgggt ttcgtagttt ttagtcgagg tttgtcgttg     360
ggagaaagaa gaatgaatga agggcggaag gggcggggag gggcggggcg cgtcgcgtta     420
gttcgcggcg tttttaggtc gggtcgtagc ggggcgtggt ttttagaggc gtggttttgg     480
gcgtgggggc ggggtttttt ggtgggggcg gggtcgcgta gggtcgtttt cgtcgtcggt     540
cgttcgcgtt tcgttttcgt gtcgtagttt ataggtttcg tttcggttcg ttttttcgtt     600
cgttttttaa gtcgtcgcgg cgtcgagtcg gtttttttcgt cggtgttcga gaggcgtttt    660
cggttcggtt cggttcggtt cgcgttttttc gttttcgttt tttcgggtcg gcggcggtgg    720
gcgagttcgc gggttcggtc gtttttagtt ttagtttcgt cgggtttcgt ttttcgtcga     780
gtgtatgagg ttgacgttat tttgttgtat ttggagggaa gaacgtatgg gagaggaagg     840
tgcgcgggtc gcggggtgtg gggcgagggt ttggaggggg tgttcggggt agcgtggggt     900
acgggagggg gtcgggtttg ttaggaggtc gcgtttttgt ttttttcggg atgagttcgt     960
ttttacgaag ttcgtaggtt tttttttgtt tttttttcgt tcgggatttt ttttttcggt    1020
tttcggcgag ttgttttttt gcgggtttgg gggtttttgg attttttttt ttttttttac    1080
gtttttttcgc gaaggatttt tagatattgt ttatttcgcg tcggttttta ttcgcgtgtt   1140
ttgtttatta ttcgggtttc gttttggggt tattttttat ttagtttcgg aagaaagagc    1200
ggttggggaa gtcgtagttt cgggttttag tggtcgtcgg gcgtttgttc ggttttgtga    1260
ttttgagtta ggcgttgatt ttttggtttt tagtttttttt ttttgtatat ttggaaattg   1320
ggtagttgtt ttttcggtgt cggtgattgg gggttagatg ggtagagcgg agataggcgt    1380
ttaggaagtc ggaggtcgtg tattgcggga gttttattta ttttttttgt tcgtgtttta    1440
aattcggtgt ttgtttttag ttttggttgg gagtatgatt tattttaatt tttttttttag   1500
tttttttttt tttattgggg tttaaggcgt agtattgtat tgtagttagg gtttaaggat    1560
ttttttagtt taggataggg tttgggggtt tttttttgga ttttttttcgt tgatttgtta   1620
tttagattta tttggtttta aggtagggtt tgattttata tttttttttg ttattaattt    1680
tttttaggtt tatgaaaatt tgattggggt aggggtttat ttttttgtag tttttgttta    1740
tttaaggtat ttgcgttttt atagagggtt aggttgttgt ggttttggga tttagttatg    1800
tgattgggta agttatgtta tttttggggt ttagtttttt tttttgtata gtggagaggg    1860
gtaggtttgg ggtatttttt agggtttatt agttttaagg gtgttaggtt ttaaggatga    1920
ttaagtatgt ttgtggttgg ttagaggagg tgttaggttt ttttgggata ggtgtttggg    1980
agtatttagg tttgtagttt tttttttttg ttaagttagg gtatttattg ttaaggattt    2040
gttagggtcg gtatttttag gttttttgtt tttgattttt tagttggttt tagtttagga    2100
tgtattaatt tagttttgtt tagtttttgt ttttgaaggg tttttttagt attttttttt    2160
gggtaggaga gggaagaaag gaggttgtga taggaatgtt atttattgtt ttatttttaa    2220
agttattgtt tttttttttt ttatttattt tgttagattt aatgttatag cgggaggatg    2280
gatttgattt ttttttttaag ttgatatata gggaaatagg gttagagaag tttggtaatt   2340
tagttagtat tttagtaaga tttaggttag cgtttttttt ttttttattt ggtatagcga    2400
ttgttttgtt tgggcgttgt atatgtgtag tgtgcgagga ttggtgtagg tgtaggtata    2460
tgtggggtgg gtagggtaag ttgggtttgt attagattat atttttttgag aatgtttttt   2520
aatttttttt ttattttgta ggaagcgagt tgttcgtgtg tgtaagttgc ggttagagga    2580
tttatgatgg ttagtatttt taggttttga acgcggattg gtacgtagat tgttttaggt    2640
agggtggggt gtttagggtt tgtgttgttt taaataaggt ttgttagaga ggataggttg    2700
gttaaggaat gggggaggtc gggatatgtt ttttggtgtc gttttttatt gtgatttcgt    2760
ggttttaatt tattatttat gatatgaggt gttttgatta gaaaattttt ataggttttt    2820
ttgttgttat tttatttatt tattttttttt ttttttttgag acggagtttc gttttgttat  2880
ttaggttgga gtatagtggt gcgattttgg tttattgtaa tttttttttt ttgggtttac    2940
gtagtttttt tgtgttagtt tttggagtag ttgggattat aggcgtgtat tattacgttt    3000
agttaatttt tgtattttta gtagagacgg gttttgttat gttagttagg ttggtttgaa    3060
atttttgatt ttaagtgatt tttttatttt agttttttaa agtgttggga tgatagatat    3120
aaattatcgt ttttggtttt attttatttt tttttatgta tttttttttt ttcgaaatgg    3180
ttttgttttg ttgtttaggt tggagtgtag tggtgttatt tcggtttatt gtaattttta    3240
tttttcgggt taaagtgatt tttttatttt agtttttcga gtagttggga ttataggtat    3300
```

```
atattataat gtttagttaa ttttttaaat tttgtgtaaa gatagggttt tattattgag     3360
atttaggttg gttttgaatt tgtgatttta agtaattttt ttgttttggt tttcgaaagt     3420
gttaggttta taggcgtgag ttaacgtttt ggtttttgtt gttattttag atttttgaga     3480
tttaaatttt agagaggatg ggagagattt ttaattgagt tagtgtttgt aatttagttt     3540
tttgttggta tttagatagg gggaagagtt ggaaggaatg ttttttttgt tttttgggtg     3600
tttatgtttt tgtagggagg gaagataaat taggttttaa gggaaattag gttattttta     3660
gtgtttttta ggttgtttgc gaatatgtat aatttagtta tattagtttt agtgtttaga     3720
tatatattta taggtaggaa gaaagtaggg ttagggttgt ggcggaggat aaagagtata     3780
tgaggatttg aaggttattt agtaggatta ttttgagaag ttaggagtag ggtttattt      3840
gttttgagtt agagtagggt tagagtaggg gttttaaagg atgtgagatt ttttgggtag     3900
aaaagtagag tggaggtggg gcgtggtggt ttatatttat aattttatta ttttttgggg     3960
ttgaggtggg tagattattt gagtttagga gttcgagata agtttgggta atatggtaat     4020
atttgtttt tattgaaaat ataaaaaatt agtcgggcgt ggtggcgtat gtttgtagtt      4080
ttagttattt aagaggttga ggtggtaggg ttatttgagt ttgggaggtg gaggttgtag     4140
tgagttatga tcgtattatt gtattttagt ttgggtaata gagcgagatt tagttttaat     4200
ttttaaaaaa gaaagaaaga aaaataaatg gtgtgggaga gaattatagg tatagttatt     4260
aaatagtaag gtttagggga gaaaatttta taaaagggta gaaggtgaag tttttgggat     4320
gtttagtagg ggttaagata tttattatta ggattttatt ttaggttttt gttttggttt     4380
attttttggt ttttggtttt tttgagatag ttttgttgtg tcgtttaggt tggggagtag     4440
tggcgcgatt tttttttatt gtaattttcg ttttttaggt ttaagcgatt tttttgtttt     4500
agtttttttaa gtagttggga ttataggtgt gtattattac gttcggttaa gttttgtatt    4560
tttagtagag atagggtttt gttatgttgg ttaggttggt ttcgaatttt tgattttaag     4620
tgatttgttc gttttagttt tttaaaatgt tggaattata ggtatgagtt attgtatttg     4680
gtttcggttt gttttttttgt tttttttttt tttttttttt atatagggtt ttgttgtgtt    4740
atttaggttg gcgtgtagtg gtgagattat agtttattgt agtttttagt tttaattggt     4800
ttaagcgatt tttttgattt agtttttttaa agtgttggga ttataagtat aagttattat    4860
gtttagtttg tttttttttt tttaggaata acgtttaacg ttttttaata tttagtaagg     4920
gataattttt gttttaagta ttttgtatag ttagatatta gtgttgtttt tgttttgtta     4980
gagagaaaat tgggatatag agaggttaat ttttttgatg aaagttatat agttagtgag     5040
tgaaatgaat atatttagtg tggttgaaag gagatagata gtatgttttg ggattttgta     5100
ttaggtgttt agaaagaggt tttcgggggg taagagggtt tttaataggt agaggaaatt     5160
atttgtatag cggtgggatg gtgcggattg ttgagggaat aggaatagtt tttttggaag     5220
gaatagaata agttgaggga tttaataaga aataaagttg agatcgattc gtgaagggtt     5280
ttgaatgtta agataaggag ttttagaagt taggatgggg gtggtggttt attttcgtaa     5340
ttttagtatt ttgggaggtc gaggtaggta gattatttga ttttaggagt ttgagattag     5400
tttggttaac gtggtgaaat tttcgttttt attaaatatt taaaaattag ttaggtatgg     5460
tggtatatga ttgtaattta agttattcgg gaggttaagg aaggagaatt atttgaattt     5520
gggaggcgga ggttgtagtg agttgagatt acgttattgt attttagttt gggagataga     5580
gcgagatttt attttaaaaa aaaaaaaaaa aaaaaataga agggagtcgg tagaaagtta     5640
gggagggggtt ggggtgatat gttgttgaag aatgttattt tagtgggtcg gtggtggtat    5700
ttaggtaggg aagggattgt tttagtaatt taagggtttg agtttatagg atttgatttg     5760
ggatagtgat tgaggatgga gagaatttta ggtagaaggg atagtttttg gtgagtattt     5820
gttatattgg ttattatgta ttgagtattt tttatgttaa tttgttaaat ttttattata     5880
attttatgag ggattgtatg tgtttagttt gtatgggaga aatagagatt ttatgtaatt     5940
aagtgtttcg ttgaaggttg taatatttag agttgggatt aaaatttttt tattttatat     6000
cgttatagag taggaaaggt aggggttggc ggtggtatat gtttataatt ttagtttttt     6060
gggaggcgga ggtaggtgga ttttttgagt ttaggagttt gagattagtg tgggtaatat     6120
agtgaaattt tgtttttata aaaaaattag ttgagtatgg tggtggtgtt tgtagtttta     6180
gttatttaag ggcgttgata tgggagggtt gtttgagttt gggaggtgga ggttgtagtg     6240
agttatgatt atattattgt aagttagttt gggtgataga gtgaaatttt attttaaaaa     6300
aaagaaagaa aggaagaaag aaaaagatag gggtttcggg gagggtatgg gtattggcga     6360
atggtagggt ggaatttgaa gttatttggt tttttaattt gggtattggg gagttggtgg     6420
tttgttgatt ttgatggaat tgggggttat gttggggagg agatatgttt atttgtgttg     6480
agttggaggg gatatgggtt atttatggtg gttgtgtttt gtttagagtt agttatggga     6540
gtttgagttt agttggaggt aggaaagtta gaaaaaacgg tcgtttcgga gttggttttg     6600
agatggtgag tgggatttgt gatagggtta agacgaatga agggaagaat tttggggatt     6660
tttgtgtttg cggtgagggg ggagatggag ttttgggtga tggagagagg gttaggagta     6720
gagttataga agttatagga gggaagtcgt gttataggat gggtgtattt ggttttggag     6780
tggtttgttt taaattattt attaggagag gggtgagttt ttaggttagg gtagtaaaga     6840
ggaggtatgt ttgtgttgtt tttggtgtag tgaaatttaa gaaggaagtt aggtgtagtg     6900
gtttacgttt gtaattttag tattttggga ggttaaggta ggtagattat ttgaggtcgg     6960
```

```
gagtttgaga ttagtttggt taatatggtg aaattttatt tttattaaaa atataaaaat    7020
tagtcgggtt tgttggtggg cgtttgtaat tttagttatt taggaggttg aggtagaaga    7080
atcgtttgaa ttcgagaggt agtgattgta gtgagttaag atcgcgttat tgtattttag    7140
tttgggtgat agagtaagat tttatttcga aaaaaaaaag ttttaatatg ggaaagatt     7200
tattagaagt aagagttatg gtttttattt cgtggtttgt gggtgtgata tttttaatag    7260
tttttaaagt tggtggtttt tatcgcgtga tagtgagtag agtagtttag agggggttat    7320
tgtttatttg ggtgtatggt tgattatagt taggttggtt tttagtggga tgtttaaggt    7380
gttagatttt gtttagtttt tttcgggttt tgggtttgag gtattgggtt cggtttagat    7440
tttattttat gtattgagat ttttgtttta gggtttttta tttttttgaa ggtgttcggg    7500
taggggtaat gtgataaggt tatgaggggt ttgtagtttt tagttttatt ggggaggtgg    7560
ttagtgattt ttattttttt ggttttttttg tatgtttttt ttagtggaat tttttagggt   7620
ttttgagtta gttatttgta aataattatg gcgtgtttat tttgtattag gtttttttta    7680
taataattta gtaagggggt gttatttatt tattaaagcg attttttttt tgagtttcgt    7740
tttgtcgttt aggttggagt gcggtggcgt aatttcggtt tattgtaagt tttgtttttc    7800
gggtttatat tatttttttg ttttagtttt ttaagtagtt gggattatag gcgtttatta    7860
ttatattcgg ttaatttttt tttgtttgtt tgtattttta gtatagacga ggtttcgttg    7920
tgtgagttag gatggtttcg attttttgat ttcgtgattc gtttatttta gtttttttaaa   7980
gtgttgggat tataggcgtg agttatcgtg ttcggtaata ttaaagcgat tttaaggtta    8040
aggttggtaa tttacgtttg taattttagt attttgggag gttgaggtag gaggattgtt    8100
tgaggttagg agtttgagat taatttaggt aatatagtga gattttattt ttataaaaaa    8160
attagttagg cgtggtggtg cgtatttgta gttttagtta tttaggaggt tgagatggga    8220
ggattatttg aatttaggat gtcgaagttg tagtgagttg tgattacgtt attgtatttt    8280
ggtttgggta atagagcgag atattgtttt aaatttttaa aaagcgattt tataaatgag    8340
gtgtagagtt tagttatttg ttaaaagttt tatagcgcgt gaggagtaga attaggattc    8400
gaatcgaggt agtttggttt tagagtttat agtgtaatta tagtttagtt ttatattttt    8460
tagattaata gggtttttgt tttttagtgg gtaagatatt tagtgaataa atgtagtgtt    8520
aggtattggg ggatagtatt tttaggaagt gatgtttaag ggatagaatt gaagggagta    8580
gtgtttagag gatgtcgggg gtagggtcgg tgtatgtgta aaggttttgg ggtgggaatg    8640
tgtttggtat aattgaggat tataaagtta gcgtgcggga gtgtagttag tggttagggg    8700
tgtatagagt tttgtgggtt tcgtggaagg tgtcgttggt tgtatagttt tttttttttt    8760
tttttttttt tttttttgag atagagtttc gtttttgttg tttaggttgg agtgtagtgg    8820
cgtgatttta gtttattgta attttcgttt ttcgggttta agcgattttt ttgttttagt    8880
tttttggtag ttgggattat aggcgtttat tattatattt ggttaatttt tgtgttttta    8940
atagagacgg ggttttatta tgttagttag gttggtttta aatttttgat tttaagcgat    9000
ttgtttattt tagtttttta aagtgttggg gttataggta tgagttattg cgtataggta    9060
gttgtgtatt tttgaatgtt ataatttgag tatttgagag ttgtttttgt tttttggttt    9120
ttgtttttga ggaagtttta cgttgatagg atagataggg ttataagtgt tgtgatgggg    9180
gtttgtaggt tgttggaggg tttagtcggg attagatgtt gttttttttt gtagagtggg    9240
ataattgttg taggtttatg ggatttttgg tattagtttt gagggttgtt atttcggggt    9300
ttgttttttt ttgtgttttg attttttagt tttttgtagg ttttgttttt cggaaggtta    9360
tgattaggtt tggattggtt taggtttttt tttggtttat atattgtttt tgcgaggttt    9420
tttttaggaa gtttttttgtg tataattttt agggttgtcg tatttttggt agtatttttt   9480
tggtagttgg gtgggttggt tttgggtaag gagggttgag tatgttgttg gtttgtgggg    9540
ttggagtagc ggcgggatgt aatttttttt tttttagggg attttttttgg cgaagataaa   9600
ttgtttatag gaagtcgatt tttgtttttt tgggggtagt agtggaagag gtagttgttt    9660
ttgagtttgt ttttgttttt agagaagttt gaggttttta gtgtcgttgt tagggtcgag    9720
gttgaggagt ttatagcgtg tgtttaggat tgagggttag ggacgggtta taggtttttt    9780
gtttggggtt taagtttaga tcgttcgttt tttattcgta ttaaagttta ggtaaagggt    9840
gttttagtta tttttttgttg taggtttaga ttaagttttt tggtatttac gcggttgtag   9900
tttttttttg tgcgttgtag ttacgttggt tttatttttt gtagttttta attttgagtt    9960
tttgagggag gatggggaag tagttggttt ggttattttt gttttttttt agattttttag   10020
agtttttagt gtagttatag aggatgttgt tggttttagt tttaagaaga cgtcgttttt    10080
tttagagggt taagtaagtg ggaatttttt tttttatttg ttttgggttt taggtagggt    10140
ttttggtgta aggtttgggg ttggaagtcg atttatttag gtttaggttt tggggtagaa    10200
ttgaaatttt ttggttattg tcggttgtag tttgggagta ggttattgtt aaagttgtgg    10260
gttttttttag gatagttttt ttatgaggtc ggttttttat ttgttgtttt tttatatttg   10320
gtggttaggg atgtggtttt gggtagaacg atgatttttt atttttgtta ttatggaagt    10380
tatcgttgtt ttttagttta gttagttatt tgggttgtag agtatttttt ttatgttttt    10440
cgggtgtttt tttttttttt tgttttagtt tggttttgtt ttattttgtt tttagggagg    10500

ggtattttgg agtggggtta gggtatggtt ttttttcgag ggagtttttt tttggttgtt    10560
```

```
tttagggtag ttttgtatag ttttagtatt tggcgtattt tttttgatat tttttttagg   10620
gatagttagg tattttgtgt ggggtattta agagagttag gttcgttagt ttttagtttt   10680
tgttagaatg taggtttgag ggtgagggg cggggtaggg gtagggatag gaatttcggc   10740
gtgtttttta ttcgtaaagg tttattgagg tttcgagttt tagttattga gttattaagt   10800
tagtttgggt taggtttggg tgttttgttt gtaatggagg tagagacggg gtttcggggt   10860
agttttgagg atgttgggtg tatagcgggg gtttcgtcgg taggaattat ttatgttttt   10920
ttttgggtta agttttgtgg atgtttagtt tggggtcgcg gggagttggt aggttagtgg   10980
tagatattgg tgggtagatt tagtgtttgg tagaataggt attaaggaag tggtgatcgg   11040
agggaagtta agtgtattta aattttcggg tgagttatta tcgtcgggtt ttttatagtt   11100
gttgaaagtg agtaatagtg atgaaggttt gtgagttttt gcgtgagcga gtgaatggat   11160
tagtagtagt ttttaggttg tggaagagcg ttttttttttc gggatgggga tatttggtta   11220
tagtaatttt taatttttta tttatttatc gtttattgta gaggtatgcg gggg tttgt   11280
tttttgtagg taggagtgag ggtatttttt gtgatgtggt atttttgtga tcgaggttat   11340
gtgtgatcgg tgtaagggta ggaagcgagt tattggtttg tattaggcgt gggggttttt   11400
gcgagggtag gatttaaagt cggtttggtt tttcggttgt agtatttttt tttttttcga   11460
attaggttag agttttggga cgggaggtgt tttgtagatt atttttttta ttaattttcg   11520
tttttcgttt tattttcgcg gtgattcggt gaattgtcgg ttttttgttg tgtatcgagt   11580
ggggtagtga ttttgacgtg gcgtttttttg tcgtttttgt tatcgttatt attttcggtg   11640
gtttagtttt cgtatt                                                 11656
```

<210> 124
<211> 11656
<212> DNA
<213> Artificial Sequence

<220>
<223> chemically treated genomic DNA (Homo sapiens)

<400> 124

```
aatgcggagg ttgggttatc ggaggtggtg gcggtggtag gggcggtagg agacgttacg      60
ttagggttat tgttttattc ggtgtatagt agggggtcgg tagtttatcg gattatcgcg     120

ggggtggggc ggaggacgga agttggtgag gggggtggtt tatagggtat ttttcgtttt     180
agggttttaa tttaattcga aagggaaagg agtgttgtag tcgggaggtt aggtcgattt     240
tgggttttgt tttcgtagaa gtttttacgt ttggtgtaga ttaatgattc gtttttttgtt     300
tttatatcga ttatatatga tttcggttat aggggtgtta tattatagga gtgtttttta     360
tttttgtttg taggaagtag ggttttcgta tatttttgta gtgggcggtg ggtgggtggg     420
ggattaggaa ttgttgtaat taagtgtttt tatttcgggg agggaacgtt tttttataat     480
ttggaaattg ttattggttt atttattcgt ttacgtagaa atttataaat ttttattatt     540
gttgtttatt tttagtagtt gtgaaagatt cggcggtgat gatttattcg agggtttgag     600
tgtatttggt ttttttttcgg ttattatttt tttgatgttt gttttattag atattaggtt     660
tgtttattag tgtttgttat tgatttgtta gtttttcgcg gttttaggtt gggtatttat     720
aaagtttggt ttaggagaga gtataagtga tttttgtcgg cgaggttttc gttgtgtatt     780
tagtattttt agaattgttt cgagatttcg tttttgtttt tattgtagat agggtattta     840
ggtttggttt aggttgattt ggtggtttag tggttggggt tcggggtttt agtgaatttt     900
tgcggatgga gagtacgtcg gagtttttgt ttttgttttt gtttcgtttt ttattttttta     960
ggtttgtatt ttggtaggag ttagaggttg acgggtttgg ttttttttgag tgttttatat    1020
agagtgtttg attgttttta agaaggatgt taagggaggt gcgttaggta ttgaggttgt    1080
gtagagttgt tttggggata gttagagagg aattttttcg ggggagagtt atgttttggt    1140
tttattttag ggtatttttt tttgagagta gggtaggata aagttaggtt ggggtaggag    1200
aaggggggagg tattcggagg gtatgaaagg ggtgttttgt agtttaggtg gttggttggg    1260
ttgggagata gcggtggttt ttataatgat aggagtggag aattatcgtt ttatttaggg    1320
ttatattttt ggttattagg tgtgaagaaa tagtaggtgg aggatcggtt ttatggggag    1380
attgttttgg aaggatttat agttttggta gtggtttgtt tttaggttgt agtcgatagt    1440
aattaaggag ttttagtttt gttttagagt ttggatttag gtgggtcggt ttttagtttt    1500
aggttttata ttaggggttt tgtttggagt ttaggataag tagggagggg gatttttatt    1560
tatttagttt tttggaggaa gcggcgtttt tttggggttg aagttaatag tatttttttgt    1620
ggttatattg gggg ttttgg aggtttaagg gagggtaggg gtggttagat tagttgtttt    1680
tttatttttt tttaggggtt taggattgga ggttgtagag ggtggggtta gcgtggttgt    1740
agcgtatagg aggaggttgt agtcgcgtgg gtgttagggg atttggtttg agtttgtaat    1800
```

aggaagtggt tgaagtattt tttgtttggg ttttggtgcg ggtggggagc gagcgattta 1860
ggtttggatt ttaggtaggg agtttgtggt tcgtttttgg tttttagttt tgaatatacg 1920
ttgtaggttt tttagtttcg gttttggtaa cggtattgaa ggttttaggt ttttttgggg 1980
atagggataa gtttaaaagt agttgttttt tttattgttg tttttaaggg aatagaggtc 2040
gatttttat ggatagtttg ttttcgttaa aaaggttttt tgaagaaagg gaggttgtat 2100
ttcgtcgttg ttttaatttt ataggttagt agtatgttta gttttttttg tttagggtta 2160
gtttatttag ttgttaagga gatgttatta gggatgcggt agttttgggg gttgtgtata 2220
ggaggttttt tggaggggat ttcgtagagg tagtatgtga gttaaaggga agtttggatt 2280
agtttaagtt tggttataat ttttcgggag gtagggtttg taagggggttg ggaggttaga 2340
atatagaaag ggtaggtttt cggagtgata atttttaggg ttaatattag aggttttatg 2400
ggtttgtagt aattgtttta ttttataaag aggggtagta tttggtttcg gttgagtttt 2460
ttagtagttt gtaggttttt attatagtat ttatgatttt gtttgtttta ttagcgtggg 2520
attttttaa gagtaggggt taggggatag gagtagtttt tagatgttta ggttatgata 2580
tttaaagatg tatagttgtt tgtgcgtagt ggtttatgtt tgtaatttta gtattttggg 2640
aggttgaggt agatagatcg tttgaggtta ggagtttgag gttagtttgg ttaatatggt 2700
gaaatttcgt ttttattaaa aatataaaaa ttagttaggt gtggtggtgg gcgtttgtag 2760
ttttagttat taggaagttg aggtaggaga atcgtttgaa ttcgggaggc ggaggttgta 2820
gtgagttgag attacgttat tgtattttag tttgggtaat aaaagcgaga ttttgtttta 2880
aaaaaaaaaa aaaaaaaaaa aaaaaaaagt tgtatagtta acggtatttt ttacggggtt 2940
tataaggttt tatgtatttt tggttattga ttgtattttc gtacgttggt tttgtggttt 3000
ttagttgtgt taagtatatt tttattttaa ggtttttgta tatgtatcgg ttttattttc 3060
gatattttt aaatattgtt ttttttaatt ttgtttttta aatattattt tttgagagtg 3120
ttgtttttta atatttgata ttatatttgt ttattgagtg ttttgtttat tagaaggtag 3180
ggattttgtt ggtttgggag gtgtgggatt aagttgtggt tatattgtag gttttgaagt 3240
taggttgttt cggttcgagt tttgatttta ttttttacgc gttgtgagat ttttggtaag 3300
tgattgaatt ttgtatttta tttgtaaaat cgttttttaa aaatttgaga tagtgtttcg 3360
ttttgttgtt taggttagag tgtagtggcg tgattatagt ttattgtagt ttcgatattt 3420
tgggtttaaa tgattttttt attttagttt tttgagtagt tgggattata ggtacgtatt 3480
attacgtttg gttaattttt ttgtagagat ggagttttat tatgttgttt aggttgattt 3540
taaattttg gttttaagta gttttttttgt tttagttttt taaagtgttg ggattatagg 3600
cgtgagttat tagttttggt tttaaaatcg ttttaatatt gtcgggtacg gtggtttacg 3660
tttgtaattt tagtattttg ggaggttgag gtgggcggat tacgaggtta ggagatcgag 3720
attattttgg tttatatagc gaaatttcgt ttgtattaaa aatataaata aataaaaaaa 3780
aattagtcgg gtgtggtggt gggcgtttgt agttttagtt atttgggagg ttgaggtagg 3840
agaatggtgt gaattcggga ggtagagttt gtagtaagtc gagattgcgt tatcgtattt 3900
tagtttgggc gatagagcga gatttaaaaa aaaaatcgtt ttaataaata aatagtattt 3960
ttttattggg ttgttgtgag aggggtttaa tgtagagtgg gtacgttata attatttgta 4020
agtgattgat ttagggattt taggaagttt tattgggagg ggtatgtaga ggggttagga 4080
aagtggaaat tattggttat ttttttagtg gggttggagg ttgtagattt tttatggttt 4140
tattatattg tttttgttcg aatattttta gaggggtgag gggttttgga ataaaggttt 4200
tagtgtatga aatgaggttt gggtcgggtt taatgtttta agtttagggt tcgagggaga 4260
ttaagtagag tttagtattt tgggtatttt attgagagtt agtttggttg tggttagtta 4320
tgtatttagg tgagtagtga tttttttttga gttgttttgt ttattgttac gcggtgagga 4380
ttattagttt tggggattgt tgggagtatt atatttataa gttacgaggt agaagttatg 4440
gtttttattt ttagtggatt tttttttata ttgagatttt tttttttcga gatggagttt 4500
tgttttgtta tttaggttag agtgtagtgg cgcgatttttg atttattgta attattgttt 4560
ttcgggttta agcgattttt ttgttttagt tttttgagta gttgggatta taggcgttta 4620
ttaataggtt cggttaattt ttgtattttt agtagagatg gggttttatt atgttggtta 4680
gattggtttt aaatttttcga ttttaggtga tttgtttgtt ttggtttttt aaagtgttgg 4740
gattataggc gtgagttatt gtatttggtt tttttttttga gttttattat attagggata 4800
gtataaatat gttttttttt tattgttttg atttggggat ttattttttt tttggtgagt 4860
gatttgggat aggttatttt aaagttaggt atatttattt tgtaatacgg ttttttttt 4920
gtggtttttg tggttttatt tttgattttt tttttattat ttaaggtttt atttttttt 4980
ttatcgtaga tataggggtt tttaaagttt ttttttttat tcgttttggt tttattataa 5040
attttattta ttattttagg gttagtttcg aggcggtcgt ttttttttgat ttttttattt 5100
ttaattggat ttaggttttt atggttagtt ttgggtagga tatagttatt atggatagtt 5160
tatgtttttt ttagtttaat atagatgagt atgttttttt tttaatatga tttttaattt 5220
tattagagtt aataaattat taatttttta gtgtttaggt tagaaaatta gatggtttta 5280
ggttttattt tgttattcgt tagtgtttat gttttttttcg aagtttttgt ttttttttt 5340
ttttttttt ttttttttt tgagatggga ttttattttg ttatttaggt tggtttgtag 5400
tggtgtgatt atagtttatt gtaattttta ttttttaggt ttaagtaatt tttttatgtt 5460

```
agcgtttttg agtagttggg attataggta ttattattat gtttagttaa tttttttgta    5520
gagataaggt tttattatgt tgtttatatt ggttttaaat ttttgggttt aagagattta    5580
tttgttttcg ttttttaaag ggttgggatt ataggtatgt gttatcgtta gtttttgttt    5640
tttttatttt gtggcgatgt ggagtgagaa ggttttagtt ttagttttag atgttataat    5700
ttttagcgag gtatttgatt gtatggaatt tttgtttttt ttatgtaaat tgggtatata    5760
tagtttttta tagagttatg atgaagattt aataaattag tatgaagagt gtttaatgta    5820
tggtagttaa tatgataaat atttattaaa aattgttttt tttgtttgaa attttttttta    5880
tttttagtta ttgttttagg ttaggtttta tgagtttaga tttttgagtt attgggatag    5940
tttttttttt gtttagatat tattatcggt ttattgggat ggtatttttt aatagtatgt    6000
tattttagtt tttttttggt tttttgtcga tttttttttta tttttttttt tttttttttt    6060
tgagatggag tttcgttttg ttttttaggt tggagtgtag tgacgtgatt ttagtttatt    6120
gtagttttcg ttttttaggt ttaagtgatt tttttttttt agtttttcga gtagtttgga    6180
ttatagttat gtgttattat gtttggttaa tttttgaata tttagtagag acggggggttt    6240
tattacgttg gttaggttgg ttttaagttt ttggggttaa gtgatttgtt tgtttcggtt    6300
ttttaaagtg ttgggattac ggggatgagt tattattttt attttgattt ttgaaatttt    6360
ttattttggt atttaaggtt ttttacgaat cggttttaat tttgtttttt gttggatttt    6420
ttagtttatt ttgttttttt taagggaatt gtttttgttt ttttagtagt tcgtattatt    6480
ttatcgttgt gtagatggtt ttttttgttt gttgagagtt tttttgtttt tcgaaggttt    6540
tttttttgagt atttgatgta gaattttagg gtatgttgtt tgtttttttt tagttatatt    6600
gagtgtgttt attttattta ttggttgtgt gatttttatt aagagaatta attttttttgt    6660
gttttaattt tttttttggt aaaataaaga tagtattaat atttaattat gtaaagtatt    6720
tagaataggg gttgtttttt attgaatgtt agaaaacgtt agacgttatt tttaaaaaag    6780
aaaaaatagg ttgggtatgg tggtttatgt ttgtaatttt agtattttgg gaggttgagt    6840
tagaaggatc gtttgaatta gttggagttg gaggttatag tgggttatga ttttattatt    6900
gtacgttagt ttgggtgata tagtaagatt ttgtgtaaaa aaaaagaaaa gaagaaataa    6960
aaaaataaat cgaggttagg tgtagtggtt tatgtttgta atttagtat tttgggaggt    7020
tgaggcgggt agattatttg aggttaggag ttcgagatta gtttggttaa tatggtaaaa    7080
ttttattttt attgaaaata taaaatttag tcgggcgtgg tggtgtatat ttgtaatttt    7140
agttatttgg gaggttgaag taggagaatc gtttgaattt gggaggcgga ggttgtagtg    7200
aggagggatc gcgttattgt tttttagttt gggcgatata ataagattgt tttaaaaaaa    7260
ttaaaaatta aaaaataaat taaggtagaa gtttaaaata aagttttagt ggtggatgtt    7320
ttgattttttg ttgggtattt tagaagtttt atttttttatt tttttatgga gttttttttt    7380
ttgaattttg ttgtttggtg attatgtttg taattttttt ttatattatt tgtttttttt    7440
tttttttttt ttaaaaattg agattgggtt tcgttttatt gtttaggtta gagtgtagtg    7500
gtgcgattat agtttattgt agtttttatt ttttaggttt aagtaatttt gttattttag    7560
tttttttgagt agttgggatt ataggtatgc gttattacgt tcggttaatt ttttgtattt    7620
ttagtggaga tagggtgttg ttatattgtt taggtttgtt tcgaattttt gaatttaagt    7680
gatttgttta ttttagtttt aaaaagtgat gggattatag gtgtgagtta ttacgtttta    7740
tttttatttt attttttttat ttaggaaatt ttatattttt tgagattttt gttttggttt    7800
tgttttggtt taaggtaggt agatttttgt ttttggtttt ttaggatggt tttattgggt    7860
gatttttagg tttttatgta tttttttattt ttcgttataa ttttgatttt attttttttt    7920
tatttgtggg tgtgtgtttg gatattgggg ttggtgtgat tgggttatgt atgttcgtaa    7980
gtagtttgga agatattgag ggtggtttgg tttttttttaa ggtttggttt gttttttttt    8040
tttgtaagag tatgaatatt tagaaggtag gagggatatt ttttttaatt tttttttttg    8100
tttgggtgtt agtagggggt tgaattgtag gtattggttt aattggaggt tttttttatt    8160
tttttttaaga tttagatttt agagatttag gatgataata gaggttaagg cgttagttta    8220
cgtttgtaag tttagtattt tcggaggtta aggtaggagg attgtttgag gttataagtt    8280
taagattagt ttgggtttta atagtgagat tttgttttta tataaaattt aaaaaattag    8340
ttgagtattg tggtgtatat ttataatttt agttattcgg gaggttgaag taggaggatt    8400
attttagttc gggagatgga ggttgtaatg agtcgagatg gtattattgt attttagttt    8460
gggtaataga gtaagattat ttcgaaaaaa gaaaaatata taaaagaaaa taaaatgagg    8520
ttaggagcgg tggtttatgt ttgttatttt agtattttgg gaggttgagg tgggaagatt    8580
atttgaggtt agaagtttta gattagtttg gttaatatgg taaaattcgt ttttattaaa    8640
aatataaaaa ttagttgggc gtggtggtgt acgtttgtaa ttttagttat tttagaggtt    8700
gatataggag aattgcgtga gtttaggagg aagaggttgt aatgagttaa gatcgtatta    8760
ttgtatttta gtttgggtga tagagcgaga tttcgtttta aaaagaaaaa aaaatagata    8820
aataaaatga taataggaag gtttgtaggg atttttttagt taaaatattt tatgttataa    8880
atggtaaatt aaggttacga agttataata ggggacggta ttaggaggta tatttcggtt    8940
tttttttattt tttgattagt ttgttttttt tggtaggttt tgtttagggt aatataggtt    9000
ttgggtattt tattttattt gaagtagttt gcgtgttagt tcgcgtttag ggtttggagg    9060
tattggttat tatagatttt ttggtcgtag tttgtatata cgggtaattc gttttttgta    9120
```

gggtgggaag ggagttggga agtattttta ggaagtgtga tttggtgtag gtttagtttg 9180
ttttgtttat tttatatata tttatatttg tattaatttt cgtatattgt atatgtgtag 9240
cgtttaggta gggtagtcgt tgtgttaaat aggagaagaa agggcgttgg tttgagtttt 9300
gttgagatat tgattaggtt gttaagtttt tttggttttg ttttttttatg tattagttta 9360
ggaggaggat taggtttatt ttttcgttat agtattggat ttggtaaggt aaatgaggag 9420
aaaggaagta gtggttttag ggataaggta gtgggtgata tttttattat agtttttttt 9480
tttttttttt ttgtttagga agaagtatta agagggtttt ttaaaggtag ggattggata 9540
gggttggatt agtgtatttt gggttgaagt tagttgggag gttaagggta ggaagtttgg 9600
aggtgtcggt tttaatagat ttttggtaat gaatgttttg gtttggtaag gggagggggt 9660
tgtagatttg ggtattttta gatatttgtt ttagggaggt ttggtatttt ttttagttag 9720
ttataggtat gtttagttat ttttggggtt tggtattttt ggagttggtg ggttttggaa 9780
aatgttttag atttgttttt ttttattgta tagaagggga gattgagttt tagagatggt 9840
atggtttgtt tagttatata gttaggtttt aaggttataa tagtttgatt ttttgtgaag 9900
acgtaggtat tttaggtagg taggggttat aggaaggtgg gtttttattt taattaggtt 9960
tttatgggtt tgggaagagt tggtggtagg gggaggtgtg gagttaggtt ttgttttggg 10020
gttaggtgga tttaagtgat aggttagcga aggagattta aggaggggtt tttagatttt 10080
gttttaggtt gggggagttt ttgaatttta attgtagtgt agtattgcgt tttgggtttt 10140
agtgagggag aaggggttaa agaggaggtt aggatgagtt atgtttttag ttaagattga 10200
gggtaggtat cgggtttggg gtacggatag ggagagtgga tgaggtttc gtagtatacg 10260
gttttcggtt ttttggacgt ttattttcgt tttatttatt tggtttttaa ttatcgatat 10320
cggggggggta attatttagt ttttaaatgt atagaagggg aaattgagga ttaggaagtt 10380
agcgtttggt ttaaggttat agagtcgggt aggcgttcgg cggttattgg gattcggggt 10440
tgcggttttt ttagtcgttt tttttttcga gattggataa agggtggttt tagggcgagg 10500
ttcgggtggt ggatagagta cgcggatgga ggtcgacgcg gggtgggtag tgtttgggag 10560
tttttcgcgg ggggacgtgg gaggaggaaa aagggtttag gggtttttag attcgtagga 10620
gggtagttcg tcgggggtcg gggaggggga tttcgggcgg gagggggata gggaggggtt 10680
tgcgggtttc gtaaggacga gtttatttcg gaggaggtag gggcgcggtt ttttggtaga 10740
ttcggttttt tttcgtgttt tacgttgttt cgggtatttt ttttaggttt tcgttttata 10800
tttcgcggtt cgcgtatttt ttttttttat acgttttttt ttttaggtgt aataaagtag 10860
cgttaatttt atgtattcga cggggggcgg ggttcggcgg ggttggggtt gggggcggtc 10920
gggttcgcga gttcgtttat cgtcgtcggt tcggggaggc gggggcggag ggcgcgggtc 10980
gggtcgggtc gggtcggggg cgtttttcgg atatcggcgg ggaaatcggt tcggcgtcgc 11040
ggcggtttgg ggagcgagcg gaggggcgga tcgaggcggg gtttgtgagt tgcggtacgg 11100
gggcggggcg cgggcggtcg gcggcgggaa cggttttgcg cggtttcgtt tttattagaa 11160
aatttcgttt ttacgtttaa ggttacgttt ttagaggtta cgtttcgttg cggttcggtt 11220
tgggggcgtc gcgagttggc gcggcgcgtt tcgttttttt tcgttttttt cgttttttat 11280
ttattttttt ttttttagc gataggtttc ggttgggagt tgcgaggttt agttgtttcg 11340
gagggtcgtt agttttgttg gggtaggtta ggaggatgta ggagtttaga gaaggggttt 11400
tcgttttttt gcgcgaggtt tttatagatt taaattagtt gtagtcggga attcggatta 11460
gagttggaga ggttttgtgg atttttagga tattggtggt cgttttgggg aggggaggga 11520
ggcgggtcgg gttttgtttt tttttttgag gtttgtattg aggttaggtt gggttcgggg 11580
aaggtgacgg ttcgcgtttt aggtttcgcg aatgagtagt attagagaaa ggagcgagtt 11640
tttgttaggc ggtgat 11656

<210> 125
<211> 13705
<212> DNA
<213> Artificial Sequence

<220>
<223> chemically treated genomic DNA (Homo sapiens)

<400> 125

gacgggaaag ggagggcgag ggttgggggc ggggatcgta tgaaatggag gcgttcgatc 60
gcgaaggggg tttttttcgc gcggttacgg ggaggagttt ggggaggtta gcgtgggtgg 120
tagttttagg gaggttgaga gtatcgttag gtttggggtt gtgacgttgg gcggattcgg 180
tagggtttgg aatttttttt tggttggttt gggttggggt ggtgggaggg gagaggtcgg 240
atcgtttttt tgtagttttt ttttattgag ttaagagggg tttcgaggga ggacggaggt 300
agggaaggaa tagttttggt tttatttagt ttattcgtag atattttatt tatatataga 360
tttgaagtta cgtaatatat ttcgattttg atatatattt taataaatta gacgtattag 420

```
tttacggtta ttttaattcg tagatattat ttatttagat ggatatttta aaatttagaa      480
tttttagttt ataaagatat attttttatt tagaggtata tggtatattt tagatacggt      540
taattcgaag atgtaaattt tattatatat aaatatttag tttatatttt aatttaaata      600
tatattttga atcgtaagtt ttaaagtata aatatattaa gttatataaa tttttgattt      660
acgacgttcg ggtatataga tgtaatttaa tttaagtaat attttagttt attatggacg      720
ttttttttta gatatatttt aaatttagaa gtttagtatt ttaagtttat agaaacgttt      780
taggataatt ttgtgttgtt atatgtatgt agtggagtag agatattttt gttttagttt      840
gggggttttt ttaagtgagt ttaggttttt ttagataaat tattattaga ttagggagag      900
gagtgaattt attggatgag ttattttttt tttttatcgg tattagtttt tacgtatttg      960
agttttcggt tgcgggttat ggagttggcg tttgggaacg ggatttgttt atagggagtt     1020
agaaagtagt ttttagtttt agttttgcgt taattcgttg ttagattttg gaaaagttta     1080
gttttttta ggttttttt ttttcgggtt tttgtagcgt agtagtatcg ggaaggtttt     1140
tttggttgtt tacgcggcgg gggagtagtg tggagtgtat ttttatttaa tttttgtttc     1200
gtgtagggat tttcgtaatt ttcgtttttt attttagcgt atattggagg aggggaattg     1260
aaggtaaacg taggggtcgg ggtcgtttgt tcgcgttttt tagagtcggg tttttttttt     1320
cgtcgtgcgt agtcgcgatc gtacgtatgt gcgtatgttt cgggagagtt tgcgcgtggg     1380
gtttggggag ggggcggttt ttaggttgag ttacgagagt tcgggttttc gtggtaggag     1440
tcgggggtcg gtaagtttta gttttgtttt tttttcgatt ttggatttcg tcgtcgattg     1500
ggttttttat tttttttttt tagcgatttt tattttgttt cgttttttat tttgagtagt     1560
tttttaggga agtcgtcgtt attattgagg tttttttaggt tttcgatgaa gttttcgtag     1620
gttatgcgtt tttcgatgtt ttaaggaagg gaacggaggt ttgtgagttt ggagggtttt     1680
tggtttttta ggttttgtat gtttggttag gggtttgttt ttcggttttt gttcgttcgt     1740
ttttatttat atggtcgtgg agattcgtgt tgagtagtat gagcgtatag gttagcgtgt     1800
gggcgtcgtt taggggagag ttgattttta gttttggttg tttttttaggg atttttttta     1860
tagttatttt ttttaatttt ttattttatt tttatttttt ttggtcggta atttttttatt     1920
ttttgaattt cgttagtaaa ttttttttat aaagagtttt tttttttagta gattcgttag     1980
taaggattcg tttttggttt ttttaagtat tgagggttag ttgttttaac gtattagttg     2040
ttttaacgtt tttttgtttt taagtttgga ttacgagaaa gtgaatcgta gtatagtttt     2100
gtagattagt gtagttaaat agaatttttt ttttagttaa gtattggttt gtataggtat     2160
gtaaattttt gttttttttat aatatattta attttagtaa tatgtttgtt tatatgtata     2220
gttttatata tatgtagtta tgatagcgtt gtttgtatat atgtaagttt atatgtttat     2280
acgtatgtat atttattttt tgttgtatat atatttatgt ttttgtatat atatataggg     2340
tttagtttat tttgttattg tggttagtta aggatatttt tgttttcgga attttttatt     2400
ttttgaggat agggttttag gattgtattg gaagtatcgt tgggagaagt gggttagtac     2460
gcgttttcgt ttttgggttt tatttattaa ggttagtttt tttagaaata ttttggagaa     2520
ggggtaagag agggttgttt agtaattagg agtttttagt ttttgttttt ggggtgatag     2580
gaggttttga gagtatagta gttggggtgg gaaggggagg agagaaaggg gatgagggta     2640
tgtattttga tttttttatt attattttta tttaaggttt ttttattagg tagttttggt     2700
ttttggttat gtttagttat tttgggaagg gtgatgttag gacggtttgt gggggataga     2760
ggggtttaaa ggaggtattt tggggtttta atacgaattt cggagagtgg gggttgtttt     2820
ttaaggggtt aatatttatt tgagagtttg gtttagagtt atgttcgtga agataaagaa     2880
tttgaggtat tttttagtta ttagtttgtt gaagttattg ttgtgggtag ggtagagaga     2940
taggtagggt tttaagggga agtgtagggg gttaggtggg gattatttaa tgattagggg     3000
atattagatg gagagttgtt tttaggatgt attttttttg tttttatttt tagttttagt     3060
ttagtggttt tattttttgt ttaggtgtcg ggttatatcg gttttttttga agttatttag     3120
tcggtatagt tttttggtta ggcgttgcgt agtttttagg ttcgtttttt gtttattgga     3180
taaggtgttt gtgtttttta gggttagtcg ttttgtgttg tttagttttg agtttgagtc     3240
ggatattagt tggtttaggg gtggttcgtt gtcggggtag aatattagtt taggttgggg     3300
tagggttttt aggagttatt tagatttttt ttttgttttt aggtttagaa gatggagaag     3360
gggagggagt ggagggatat ggggaaagta tttttttttt ttttagggaa ggagattaga     3420
tttttttat tagtttattt tattattatt atttttttgt ggaagttttg ttttaaattt     3480
aatttatatt cgttttgttg ttgtgggagt tttggttgtt tttttaagtt tttaggggtt     3540
tatatttgtt attagatata gattatttgt ttttattttg gttttatata gaatatagtt     3600
ttttgttttt tagattatgg ataaagttgg aatagatggg ttattacgga ggaattgtgg     3660
agggttaggg aaagatttga gggtagtagg gatattggtt cgaaggtttt tttttttttg     3720
tttttttaggg agaggaattt tttttttggtg tttttttttt gtttatatag aagtaggttt     3780
tggagttagg gggttaaggg tcggggtgta tatttttatt tttttttttt tttttttttt     3840
ttattttagt tgttttattc gtaaagttat ttgttagttt agagatagag gtcggaaatt     3900
tttggttgtt aggtaattcg gttttttttgg ttattttttt ttgttgttat ggtttttatg     3960
attgagaggg tagttggtag aggggagagt agaaaggtta gattgtgaaa ggattttttg     4020
gagttggacg agagatgttt gattaggtag gggtgatatg ggagggaggt agtgggagag     4080
```

```
gttggtggtt gtatttttgg tgaagttttt aggttgggtg agggtaaggt tataagaatt    4140
ttgagtaggt taaagatttt gttaagggaa gaaagggttt tttaggagag taagaagggt    4200
atttttttgg gatttttttaa tgttgaatta tgttttttta agttattgga tttgagatta    4260
agaggggtgt tgtgagggat ttttttttat agttaatggt agtagttttt taaataggaa    4320
aataggattt gtattttttta attattttta tttaggggtt aggaatataa attaattttt    4380
atatttagga tttgtttttt atagtagttg tttttggagt tttgggggtt ttaagtttga    4440
gggataaaag gaattattta tttgttaagg ggtgttgttt ttagtttaag gttgtttttt    4500
gagtggtagg gattaggggg tttttttttt ggggttagtt tggttttggt ttttgttttt    4560
tttttttttt ttttttgtgt ttaaggatta ttagtagtag agttggtatt agaattcggt    4620
ttaagagggg taagtggggc gggagttggt gggtcgggtc ggggtgtagg gggttgggga    4680
ggtagtttaa aggtgaagaa ggggttttgg gttgggttag gtgaggttaa ggtttttagc    4740
gaggcgaggt tggtatagga ggtgtttttg gttcggtgtg attttaggat ggtttcgaat    4800
atataattga aagagttagg tttattagtc gaggggttcg ttttaggtag aaagggtata    4860
ggtgatttga gaggtattcg gtttttttggt ttgtaggggt agggagaatt tttgattagg    4920
ggtttagatt atagggttgg ggtaggattt tattgaattt ttatattgat tttgttaagg    4980
ggattattat tttttttttta ttggttttag tttattaata aaaaagagtt tttagggtag    5040
ggcgttaagt tatttagtgt tttttggggt atattagttt tattagtttt tagtatttta    5100

tttttttttt ttttgttaat tttttttttt tttttttttt cgtgattcgt ttttttttttg    5160
ttattttatt tttattttta gtaatatttt aggagaagat gaggttttaa gcgatattgg    5220
ggtgtgagac ggaaaggaat gtttttatat tttttatttt tttattagga tttaggattt    5280
agatttatgt ttttgtgttt ttgggtttga ttgtggtttt tggtttttta ttttgttttt    5340
tgttttgtta gaatttttgg tttttttgtt ttttatattt ttagtgtttt gttaagtttt    5400
aggtttttgt ttagaatttt ttgttatata tattgtttcg ttagttttat atttattttt    5460
attttaggtt ttttgagttc ggttttgttt gtgggttagt gcgaaggagt aagtcgtttt    5520
ttttttttat tttttttttt tattttttta tttatcgggt ttttttagag gttttaaata    5580
tttcgtcgtt tatatttttt tttttatttg ttttatcgtc gtttttattt ttattgttat    5640
tgtcgaggtt gggatgaggg ttagggagtg ggtcgggtcg tggggtaggt gggtaggtag    5700
tgtttggtgg gtttttagag ttgggttgat tttcgatggt cgagtcggtt ttttttcgtt    5760
tagttagtat tttatcgatg ttagtttcgc ggtagtattt taggggtatc gtgtttaggt    5820
ttgtttcgga gtatttggtt gttcgtttta tagttatttt agagttttgt tttgagttta    5880
ttttaggtgg agatgggggg gtttgttttg ggggtttagt atttgtagtg tgtataggta    5940
tttaggttag gaggttattt acgttagttt tttgttttaa ttttgtttta ggatttttgt    6000
ttattgtttt atattgatag gaagtttttt tattgtttag ttattatttt ttttgttata    6060
tatttagttt gtttttggtg ttagtggagt ttattttaaa ttaagttttt gttaaatggt    6120
tttgaggagt tgggggaaga ttagtttttt tttgtttttt ttatttattt atttatttat    6180
tttgagatag ggttttattg tgttattgag gttgaagtgt agtggtgtaa ttataattta    6240
ttgtagtttt gattgtttgg atttaaggga tttttttatt taattttttt agtagttagg    6300
attataggtg tatattatta tatttagtta attaaatttt tggtagagat ttgtttgttg    6360
tttaggttgg ttttggattt ttggttttga gtagtttttt tgttttggtt ttttaaaata    6420
ttggggttat tgagttttgt tgagtttttt ttttatttta tggaatgttt tttaatattt    6480
ttttgttttt ttttttttatt atttagtttt ttttaggtgt tttgtttagt tttatttttt    6540
agtttttttat tatagcgtag atggaaagat tgaggtttgg ggagatattg gttgatttaa    6600
gagttaattt taaggattgg gggaagtttg aaggagggtt ttttattgag ttttagttgt    6660
tgtggggaag gttttgtttt gtttttttttg ttttagtttt aggttttatt tgagttgggg    6720
gggtttaagg agttatagaa gaatttttat ttggttttag cgatttttttg ggtatgagag    6780
gagattttttc gggggggagga ttaggtattt ttttgagtta acgaggggaga tataggtatt    6840
tagagataaa gttagatata taaggatata agaatatggg atagaaatag aaattaaaat    6900
atgtatcgat agagatggag gtttagagat atagagataa atatagaggg taagagagag    6960
gtagatttag aatagattaa aggatttaga gtgaatagta gataagttta tgtttgatag    7020
gaggttgagt tttgtttttt tattgggggt ttagggagtt taggggaagt tgtatatttg    7080
gtttaggaat ttagtgttag taggtttttc gaagagtgtg gttaggcgtt tagggggggtt    7140
ttttagttta ttggggagag aggagtaaag gttatttgtt ttaatttggg gtggggttgg    7200
cggtttatgt ataaggtttc ggttgttagg tagggtcgag tttttttttgg cggggagtgg    7260
gtttgatgtg gatgttttta gtcgtaattt tcggttggag ttaggtttaa gggttggggt    7320
gggtttggga ggagaaattt tatttagatt ttagtttcga tttaagtttt ttggagtagg    7380
tagtttattt agtggtttta tattggtttt ttttataaag cggaagatga ttatagagtt    7440
ttgggtttcg ggtgggggtt gtttagtggg tgaagagggt gtttagggtg agggagtaat    7500
acggggtgag ggggggttac gtagaggtgt atagggtgtt gttatacgtt ttagttttcg    7560
gtttcgagga tttggatttg ttattcgtcg tagtaaggag tttgtgttgt tatatatgtt    7620
ggttgggggg ttttggggta tcgggttttc ggggagttag cggcgtgggt atcgtggtgg    7680
```

ggagatggta tagtcgtttt tcgagtagaa gcgtatggta ttttgggtta tgttggggtc 7740
gggggttagg ttgggggggt agggatggcg aggttaggcg gggagtaagg gggttgtatg 7800
ttttttagat aggtttgtaa gagaggaagg ggagtatggg gtgaattgtt aaggagttag 7860
atggatagga tagtagagat agggaaggga aagttggtta gggtttttga gtattaggga 7920
tttagtatat tttggtaata tgggattatt gattttacgt ttggggatta tttttataga 7980
ggtttgacga gaaaagattt atatatttag tgggtttttg gttttttttt ttttgtttga 8040
ggttttgagt attattttgg gaggagagag gaagtaattt taaagttgag tgagttcgga 8100
gtttttatgg tattttggag ttatttttag aataggggaa gttggtattt tattttttgg 8160
aaaaggggtg ttagttagtt gttattatta tatagttttg tgatttttat agtttttttt 8220
atggaggttt tttatttagg gaagggaaat ttaggttttt tggaaggaga gggaagagga 8280
ataggtgtta tatagggatg ttgaagggtt gagaggtata gtatggaagt ggggagaaaa 8340
gtttgagtag aggtttttat ttttaatata tatatattga gtgggaaagg gatatttttt 8400
tttttttttt ttttttttga gatggagttt cgtttttgtt gtttaggttg gagtggaatg 8460
gcgttatttt agtttattgt aattttcgtt ttttaagttt aagtgatttt tttgttttag 8520
atttttgaat agtttggatt ataggcgttt agtattatgt ttggttaatt tttgtatttt 8580
tagtagagat ggagttttat tatgttgttt aggttagttt taaatttttg attttagggg 8640
atttatttat tttggttttt taaagtgttg ggattatagg cgtgagttat cgtattcggt 8700
tagggatatt ttttttttta tataggtata gataggtatt tatatatata taaatatata 8760
tatatatata tatatatata tacgtatata tatatataag gataaatatg ttagtatata 8820
gttaaatata tttataggta atattttttg gtatatatat agatatatat atatatatat 8880
tgtttaaaga tatattttta tgtattttta tagttataat aagtatgtgt gtaatgaaaa 8940
ggtatatgtt tgttttatat atatattatt attaaatagg aatatataaa gaggtattta 9000
aatatatata taagttgtta ttttttagtg tatatagagg tttaggtgta ttagatattt 9060
atagagattt attgtttata gttatatata tatatagatg tatatatata gatgtatata 9120
tttaagtagg gatatatatc gttttttttt ttttatttat tggggttttt tgaggggaat 9180
ttgtatttat attttttttt tttttttttt tttttagggg taagatagat ttcgtttagg 9240
agagattata attaggtaaa ggggtttttg gtggtaatgt aggggagggg tagaggttag 9300
gagtagtaat tttgttaggt aggtttcgga agtttaacgg ggttttagcg tatattcggt 9360
gtagttttaa tttaatgtta ttttagtatt tatcgttgtt cgtttagagt tgagatcgag 9420
gagagataga ggagaggttg ggtttagttg agttgtgaag gtagcgcggt tttcgtttgt 9480
tttaggttcg ttcgtttttt agtttttttt tttatttttt acgcgtcgtt taggtaacgt 9540
taattttttt tatttttcgt tttttttttag ggtttagagt ttcggaggtt gttagttttt 9600
tttgtcgtta ggttttcgtt gggtttttttc gttaatttaa gattcgtttt agataaggag 9660
gagggcgtat ggaattttttg gttttatttt ttagattttt acgtgtttta ttttatttgt 9720
gtttacgttt ttttcgttta tcgtttttttg agttatacgt aaggttcggt tgtatacgcg 9780
tgtttcgttt tagattttac gcgtgttttt aggaggttgt tatatgattt gttatatgtt 9840
atacgcgttt tcgtttttac gagtacgcgg tcgcgttaag tatacggtat gggtgtagat 9900
tggattcgtt ttttaaattt aggagattat taattatgga aaggagattt agggaatatc 9960
gttttgaatt cgttaggggt tttgagtttc ggatttagga gatttaattt tgattatttt 10020
tttaggatgt agtaggggga tgttaaatta gattttagga agaatttttt tgttgtttga 10080
gttaggtgtc gttacgtttg tttaggggta agaggttaga cgggatgaat tagggatcgg 10140
aagacgtggg ggatcggtaa tttttgtgtt tttggttagg gtagaagggg gttttgttat 10200
tttaagaaag agtcgttgga gttattatgg taattatttt ttttgggagg gttagggcgg 10260
gtagatggtg atttcggaag gaagaagggg tttgttcggg tagttagggg gcgcgaggac 10320
gtgcgaggcg ggggcgcgcg gggtttatcg cggggagggg cggcgcggat ttcgcgtggt 10380
gttgaagggg atagcgttag atttgtcgta ttgcgtaggc gtagaggcgc gaataaagag 10440
gggaggggga cgaggcgcgc ggtcggaggt ggtgcgcgtt tacgtggttt tatttttgta 10500
ggggtttgtt tttttagtta tttttttttt aagtaggttg aggaagaaga gatagtaagt 10560
ttttatttaa tttgattttt tttttgtttt ttttcgttgt tagttttttt tttttcgttg 10620
ttttgcgggg tggggaaacg tggaaggaat tttgggaaaa ttagatgttt gggtttttta 10680
ttttcgtttg gtttgatttt ggataagggg tttttttttt agggtttcgg tttttttatt 10740
tttaaaatgg attgatagta tagatttgtt tattttttag aggtgttcga agttttgacg 10800
agataataga tgagaagtgg ttttcgttgt gatgagttag ttgaagcggt tgtatcggag 10860
cgttagatcg ttagcggtgt agtggttttc gcgtttttta tattttttta tatttatttc 10920
gtggatgatg ggggaaggta ttatttttgg ggtttttcga agcggatttt gggattggga 10980
tttggggacg cggtgagagg tagttttgaa gttgagttta gaggtttggg gcgttattgt 11040
tttagggcgg atttagtttt agattttata attaaattta aggttagtcg agaagtatta 11100
ttattggcgg gttgagttga gtttttattt tttttaggat tttggatttt tggaggaaat 11160
gaaaggggatt tagttggaaa tatagttttt tttcgcgttt ttttatattt ttttttttcgg 11220
cggaaacgcg gtttaaattg ttacgaagtg gaagaggttg gatagttttt tataattaat 11280
taggaatggg aggtttgagt aggtttattt gatgagggaa atattatagt ttcgttaatt 11340

```
tatttattat tttaataatt atttattgaa tatttgttac gtggtagata gtaagagttt   11400
ggatagtttt ttttcgtttt ttaaagtcgt taaacgcgat tagagggtag aggagaggtc   11460
ggtaatttaa gagttttggt ttcgtttttt tcgcgtttgg ttttgtttgt ttttttttatt   11520
attatttcgt ttagaggcgg ttattggttt aagaggacga tagaagttag tttttgatgt   11580
ttatcgtatt ttttagtttt aggttttaag ttttgtagtc gagaaggcga ggtttaatgt   11640
tatattatcg gtttgtttcg ttttcgtttc ggggtttatt cgaaaagttt ttagatcgga   11700
ttttcggtga gacggtattc gattaaatag gtttgttttt ataggttaag gaggcgggtt   11760
tggtgcggtt attttaaggt taaagcgaga aaattttatt gcgtacgcgt aatagatagt   11820
cgatggagtt atcgatggag tcgttcggag gggagtaaga gttcggagtc gttaggtatc   11880
gagtatcgga ggggtcgaga gggaagagga atttgaggga ggggagtcga gtcggggttg   11940
ggtttggggg tcgtttttgg gagttggggc gtaggaatat aacggaggcg gattcgtagg   12000
taatagtggt ttcgggacgt tagtgttaac gttttttat tcgtaggttt ttggatttgt   12060
tttgggaaga cgtgttgttt ttatacgttt tgaatcgggt ttcgttgcgt tagttgtttc   12120
ggttgtagcg cgttagtcgg gtttttcggt cgttggtgta gttttatttg gtcgggttgc   12180
gtcgtttcga tgtcgcgtag gtgagtcggg ggttgaagtt tcgtttttgt ttgggtatcg   12240
tttcgttttt agtttagttt ttagtttcgt agtatgtttt tttggatttt tttgggtcgt   12300
cggggttgtt cggaagggat ttacgtatta aagggtaaat attcggtagc gatttagagg   12360
gaaagtgggg ttttttttgg gagagtagga ggttgttaga aaagaattta ggttaggggt   12420
gtataggcgg ttgaggagtg cgggacgggt tgagagttgg ggtgtttttt cgttcgtagg   12480
tgggttcgta gatttcgcgg gtcgtattgg ttcggttgtt gcgggatgtc gaggggttgt   12540
aggagttggt attggcgtcg tgttacgaat ggttgttaga cgaggatttg gtgtcggtgt   12600
tggcgcggaa ttcgtagttg cggagtgtgg cgttgggcgg ttgcgggtaa ttgagtcgtc   12660
gggcgtttgg ggttttggtc gagggttgtt tacgtttgta gcgtttgtcg ttcgcgtatt   12720
gtgattgggt ggacgggttg gcgttgcgcg gtttcgttga tcgttgttcg gttttggagg   12780
agttggattt tatcgtttgt cgttagttta aggacgaggt tatcgtgtat ttggcgtaga   12840
ggcgcggcgt tggttttcgt agttttttttt tggtcgttaa cgttaacgtg ggggacgtcg   12900
cggtttaaga gttggttcgg aattgtttag aattttatta ttttgatttt atcggttgtt   12960
ttcgcgtcgg aagcgacggt gttaggtgtt tgggtttgat agggcgggag gagggtagtt   13020
atttagtttt tgttggtatg gggcgggttg tagttgttaa aaggtcggat tgagggtttt   13080
gaattttttt gtaaattata gtatttttat tttgaataga aagggttttt aggattgttt   13140
aggttagaga gtttatagtt taaaaatttt ttttaattaa tgtttatata ttgggagatt   13200
ttatatagaa agtcgatttt tggttttttt ttaataaggg ggagattttg taattttagg   13260
cggagttgag ggttaggttt tttttttaaa tgagatagga agtttttagt ttattggaag   13320
ttaatttagg cgtttttttt gtgtagggac ggaaaaagta tgtttttgtt tcgagttgga   13380
gggagatcgg gattttgtcg gagttagttc gaggttgagt gagttcgggt ttttatttta   13440
ttttattaaa ggtgtggtgg ttttatgttt ttagttttat tttgtttttt ttttaggata   13500
ttggtcgagt attgtttcgt gttgcgttcg ttgcgggtgc ggtattgtta ttatgtggcg   13560
gagtttagtt tgagtcgttt gcggaagcgc ggcgtggata tcgacgtgga gtcgtcgttg   13620
tattaggttt tggtgttgtt gtaggatatg gcgggtttcg tattttttgt taatttgtag   13680
gtttgattcg tttgttaatc ggagt                                          13705
```

<210> 126
<211> 13705
<212> DNA
<213> Artificial Sequence

<220>
<223> chemically treated genomic DNA (Homo sapiens)

<400> 126

```
gtttcgattg gtaggcgggt tagatttgta ggttgataaa aggtgcgaag ttcgttatat      60
tttgtagtag tattagggtt tggtgtagcg gcggttttac gtcgatgttt acgtcgcgtt     120
ttcgtaagcg gtttaggttg gatttcgtta tatggtggta gtgtcgtatt cgtagcgaac     180
gtagtacggg gtagtattcg gttaatgttt tgagggagga gtagggtgag gttagggata     240
tgaggttatt atatttttgg tggggtaagg tggaggttcg ggtttattta gtttcggatt     300
ggtttcggta aaatttcggt tttttttttag ttcggggtag gggtatattt ttttcgtttt     360
tatataaggg aggcgtttgg attggttttt agtgagttgg agattttttg ttttatttaa     420
ggggggaatt tggtttttag tttcgtttag gattgtaaga tttttttttt gttgaagaaa     480
agttagaagt cgattttttta tgtgaaattt tttaatgtgt aaatattggt tgaaaaaagt     540
ttttaaatta tgggtttttt ggtttaggta attttagagg tttttttttgt ttagaatggg     600
```

```
ggtgttgtgg tttgtaggaa gatttagaat ttttagttcg gttttttaat aattatagtt      660
cgttttatat tagtagaggt taagtgattg tttttttttc gttttattag gtttaggtat      720
ttgatatcgt cgttttcgac gcggaggtag tcggtgaggt taaggtggtg gagttttggg      780
tagtttcgag ttaatttttg aatcgcggcg ttttttacgt tggcgttgac ggttagagag      840
aggttgcgga gattagcgtc gcgtttttgc gttaggtata cgatggtttc gtttttgagt      900
tggcggtagg cggtgagatt tagttttttt agggtcgggt agcgattagc gaggtcgcgt      960
agcgttagtt cgtttattta gttatagtgc gcgagcgata ggcgttgtag gcgtgggtag     1020
ttttcggtta aagttttaag cgttcggcga tttagttgtt cgtagtcgtt taacgttata     1080
tttcgtagtt gcggatttcg cgttagtatc ggtattaggt tttcgtttga tagttattcg     1140
tgatacggcg ttagtgttag tttttgtagt ttttcggtat ttcgtagtag tcgggttaat     1200
gcggttcgcg ggatttgcgg atttatttgc gggcggggag gtattttaat ttttagttcg     1260
tttcgtattt tttagtcgtt tatgtatttt tgatttgagt ttttttttgg tagtttttttg    1320
ttttttttagg agagatttta tttttttttt gagtcgttat cgagtatttg tttttttggtg   1380
cgtggatttt tttcgggtag tttcggcggt ttaagggggt ttagaagggt atattgcggg     1440
gttgggggtt gggttagagg cggggcggta tttaggtagg ggcggggttt tagttttcgg     1500
tttatttgcg cggtatcgaa gcgacgtagt tcggttaggt gaagttgtat tagcgatcgg     1560
aaggttcggt taacgcgttg tagtcggagt agttggcgta gcgggattcg gtttaggacg     1620
tgtgggagta gtacgttttt ttagggtagg tttaggaatt tgcgagtaaa ggggcgttgg     1680
tattggcgtt tcggggttat tattatttgc gagttcgttt tcgttatgtt tttgcgtttt     1740
agtttttaag agcggttttt agatttagtt tcggttcggt tttttttttt taggtttttt     1800
ttttttttcg gtttttttcgg tgttcggtat ttgacggttt cgggttttttg ttttttttcg   1860
gacggtttta tcggtggttt tatcggttgt ttattgcgcg tgcgtagtaa gatttttttcg    1920
ttttggtttt ggagtggtcg tattaaattc gttttttttgg tttgtagaga tagatttatt    1980
taatcgagtg tcgttttatc gaggattcga tttgaaagtt tttcgggtgg atttcgaaac     2040
ggaggcggaa tagatcggtg gtgtaatatt aggtttcgtt ttttcggtta tagggtttga     2100
ggtttgaagt tggagaatgc ggtgggtatt agagattggt ttttgtcgtt tttttgagtt     2160
agtagtcgtt tttgagcgaa gtggtggtgg gaggagtagg taaggttaaa cgcggagggg     2220
gcggagttag gatttttagg ttatcggttt tttttttgtt ttttggtcgc gtttagcgat     2280
tttgaaaaac ggggagaaat tatttaggtt tttattgttt gttacgtagt aggtgtttaa     2340
taagtaattg ttgaaataat gaatgaattg gcgaggttat aatgtttttt ttattaagta     2400
aatttgttta aatttttttat ttttgattaa ttatgaagag ttatttagtt ttttttattt    2460
cgtaatagtt tgggtcgcgt tttcgtcggg ggagagagat gtagggggcg cgaagaaaga     2520
ttatattttt aattgggttt tttttatttt ttttaaaggt ttagagtttt aagaagaatg     2580
agagtttagt ttagttcgtt agtggtagtg tttttcggtt ggttttgggt ttagttgtag     2640
ggtttggggt tgggttcgtt ttgggataat gacgttttag atttttggat ttagttttag     2700
ggttgttttt tatcgcgttt ttaggtttta gttttaggat tcgtttcggg gaattttagg     2760
agtagtgttt tttttttatta tttacgggat gggtgtgggg ggatgtgaag gacgcgagag    2820
ttattgtatc gttgacggtt tggcgtttcg gtgtaatcgt tttagttggt ttattataac     2880
gaaaattatt ttttatttgt tatttcgtta aggtttcggg tatttttgaa aggtgggtag     2940
gtttatgtta ttagtttatt ttaaagatgg ggaaatcgag gttttggaga ggaagttttt     3000
tgtttagaat taagttagac gggaatagag aatttagata tttggttttt ttagaatttt     3060
ttttacgttt ttttatttcg taaggtagcg agaagggaga agttagtagc ggggagagat     3120
aagaaagggg ttaagttagg tgagggtttg ttattttttt tttttttagtt tgtttaggga    3180
gggggtagtt gggaggatag atttttgtaa agatagagtt acgtgagcgc gtattatttt     3240
cggtcgcgcg tttcgttttt tttttttttt tgttcgcgtt tttgcgtttg cgtagtgcga     3300
taaatttggc gttgtttttt ttagtattac gcggagttcg cgtcgttttt tttcgcggtg     3360
agtttcgcgc gttttcgttt cgtacgtttt cgcgtttttt gattgttcga atagattttt     3420
ttttttttttc gaggttatta tttgttcgtt ttggtttttt taaagagagt agttgttata    3480
gtaattttag cggttttttt ttaaaatagt agggtttttt tttattttgg ttaagggtat     3540
aagggttgtc ggttttttac gtttttcggt ttttagttta tttcgtttaa tttttttattt    3600
ttgggtaggc gtgacgatat ttaatttagg tagtaaggaa gtttttttttg aagtttgatt    3660
taatattttt ttgttgtatt ttgggagggt ggttagggtt ggattttttg ggttcgagat     3720
ttaaaatttt tggcgggttt aagacggtgt tttttagatt ttttttttat agttgatggt     3780
tttttggatt taggggggcgg gtttagttta tatttatatc gtgtgtttgg cgcggtcgcg    3840
tgttcgtggg ggcgggagcg cgtgtggtat gtaataggtt atgtggtagt tttttaggga     3900
tacgcgtggg gtttggagcg ggatacgcgt gtgtagtcgg gttttacgtg tggtttaagg     3960
ggcggtgggc gagagagacg tggatataag tggggtgggg tacgtgaggg tttggggaat     4020
agaattaggg gttttatgcg tttttttttt tgtttggggc gggttttagg ttggcgggag     4080
agtttagcgg ggatttagcg ataagggggg ttggtagttt tcggggtttt gggttttggg     4140
agagggcggg gggtgggggg gattagcgtt gtttgagcgg cgcgtagggg gtagggggag     4200
ggattgagag gcgggcgggt ttggagtaaa cgggagtcgc gttgttttta tagtttagtt     4260
```

```
gggtttagtt ttttttttgt tttttttcgg ttttagtttt gggcgagtag cggtgagtat 4320
tgaggtggta ttgagttggg gttgtatcgg gtatgcgttg ggatttcgtt gagttttcgg 4380
aatttgtttg gtagggttgt tatttttgat ttttgttttt tttttgtatt gttattagag 4440
gtttttttat ttggttatgg ttttttttgg gcgggatttg ttttattttt ggggagagag 4500
ggagaggaga ggaatatggg tgtagatttt ttttaagaga ttttagtagg tggggggggag 4560
gggacggtgt gtgtttttgt ttgagtgtgt atatttgtat gtgtatattt gtatgtgtat 4620
gtgattgtga gtagtaggtt tttgtgagtg tttgatatat ttggattttt gtgtgtattg 4680
gagaatgata gtttgtgtat atatttgggt attttttttgt gtgtttttgt ttgatgatgg 4740
tgtgtgtgta aaataagtat gtgttttttt attatatata tatttgttat gattgtgaga 4800
gtgtatgagg gtgtgttttt gagtagtgtg tgtgtgtgtg tgtttgtgtg tgtgttagag 4860
ggtgttgttt atgaatgtgt ttaattgtgt attgatatat ttgtttttat gtatgtgtgt 4920
gcgtgtgtgt gtgtgtgtgt gtgtgtgtgt gtttgtgtgt gtgtaggtgt ttgtttgtat 4980
ttgtatgaag aaaagaatgt ttttggtcgg gtgcggtggt ttacgtttgt aattttaata 5040
ttttgggagg ttaaagtggg tggatttttt gaggttagga gtttgaaatt agtttggata 5100
atatggtgaa attttatttt tattaaaaat ataaaaatta gttaggtata gtgttgagcg 5160
tttgtaattt aagttattta ggagtttgag gtaggagaat tatttgaatt tgggaggcgg 5220
aggttgtagt gagttgagat agcgttattt tattttagtt tgggtagtag ggcgaaatt 5280
ttattttaaa aaaaaaaaaa aaaaagaaga atgttttttt tttatttagt atgtatatat 5340
tgggggtagg agtttttgtt taggtttttt tttttatttt tatgttgtgt tttttagttt 5400
tttagtattt ttatataata tttgtttttt tttttttttt ttttaggagg tttgagtttt 5460
tttttttga gtgaggggtt tttataagag agattatagg ggttataggg ttgtatgatg 5520
gtgatagttg gttgatattt ttttttttaga gggtggggta ttaatttttt ttattttaga 5580
agtagtttta aagtgttata agaatttcgg gtttatttaa ttttgaagtt gttttttttt 5640
tttttttagg gtgatgttta gggttttagg tagaaggaaa ggagttaggg gtttattggg 5700
tgtataagtt ttttttcgtt aggtttttat ggaagtagtt tttaggcgtg ggattaatga 5760
ttttatatta ttagggtgtg ttaggttttt gatatttaga ggttttagtt aatttttttt 5820
tttttatttt tgttgtttta tttatttaat tttttggtag tttattttat gttttttttt 5880
tttttttata ggtttgtttg aagagtatgt agttttttta tttttcgttt ggtttcgtta 5940
tttttgtttt tttagtttga ttttcggttt tagtatggtt tagggtgtta tgcgtttttg 6000
ttcggaaggc gattgtgtta ttttttttatt acgatgttta cgtcgttggt ttttcgaagg 6060
ttcggtgttt tagagttttt tagttagtat gtatggtagt ataggttttt tgttacggcg 6120
agtggtaggt ttaggttttc gaggtcggga attgggacgt gtgatagtat tttgtatatt 6180
tttgcgtggt tttttttttat ttcgtgttgt tttttttattt tgggtatttt ttttatttat 6240
tgggtagttt ttattcgggg tttagagttt tgtggttatt tttcgttttg tggagaaggt 6300
tagtgtgagg ttattgaatg ggttatttgt tttagggggt ttgagtcgga gttgggattt 6360
gggtggggtt tttttttttta ggtttatttt agtttttggg tttggtttta atcggaagtt 6420
acggttggaa gtatttatat tagatttatt tttcgttaga ggaggttcgg ttttatttgg 6480
tagtcggaat tttgtatatg ggtcgttagt tttattttag gttggagtag atggttttta 6540
tttttttttt tttaatgggt tggggggttt ttttgagcgt ttggttatat ttttcggagg 6600
atttgttgat attggatttt tgaattaggt atgtaatttt ttttgggttt tttgggtttt 6660
tagtgaaggg gtaaggttta gtttttttgtt agatatgggt ttgtttgttg tttattttga 6720
attttttaat ttgttttaaa tttgtttttt ttttgttttt tgtgtttgtt tttgtgtttt 6780
tgaattttta tttttgtcga tgtatgtttt aatttttgtt tttgttttat atttttgtgt 6840
ttttgtatgt ttggttttat ttttgggtgt ttgtgttttt ttcgttggtt tagggggata 6900
tttggttttt ttttcgggaa gttttttttt atgtttagag aatcgttaga gttaaatggg 6960
aattttttta tggtttttttg gatttttttta gtttaggtaa ggtttgggat tgaggtaggg 7020
agaatagggt agggtttttt ttatagtagt tgaggtttag tagggagttt ttttttaaat 7080
tttttttaat ttttggaatt ggtttttgag ttagttaatg ttttttttagg ttttaatttt 7140
tttatttgcg ttatgatggg aggttggagg ataggattgg gtagggtatt tggaggaagt 7200
tgagtaatgg agggagggga tagaaggata ttgaggggta ttttatggag tagaaaagag 7260
gtttagtagg gtttagtggt tttagtattt tgggaggtta aggtaggagg attgtttaaa 7320
gttaggagtt taagattagt ttggataata agtaagtttt tattaaaaat ttaattagtt 7380
ggatgtagtg gtgtgtattt atagttttag ttattgggga ggttgggtgg gaggattttt 7440
tgagtttagg tagttaaggt tgtagtgagt tatgattgta ttattgtatt ttagttttag 7500
tgatatagtg agattttgtt ttaaaataaa taaataaata aataaaaaag atagaaaaag 7560
gttggttttt ttttagtttt ttaaagttat ttgataagaa tttgatttaa aatgagtttt 7620
attggtatta ggaataagtt aagtatgtag taggaggaat ggtggttaga tagtgaaaga 7680
atttttttgtt aatatgaggt agtgggtaga gattttagag tagaattagg atagggagtt 7740
gacgtggata atttttttgat ttggatgttt atgtatatta taggtgttaa gtttttagag 7800
taggtttttt tatttttatt tggggtgggt ttaaggtagg gttttggggt ggttgtgggg 7860
cgagtagtta agtatttcga gatagatttg gatacggtgt ttttgaggtg ttatcgcgag 7920
```

attgatatcg atgaggtgtt ggttgagcgg gaggaggtcg attcggttat cgaaagttag 7980
tttagttttg agggtttatt aggtattgtt tatttatttg ttttacggtt cggtttattt 8040

tttggttttt attttagttt cggtagtggt aatgaggatg aggacgacga tgaggtaggt 8100
ggggaagaag atgtggacga cgaggtgttt gaggttttttg aaggggttcg gtgagtgggg 8160
aagtgggagg gggaagtggg agagggaagc ggtttgtttt ttcgtattgg tttataggta 8220
gagtcgggtt taaagggttt gggatagaga tgggtgtggg gttggcgggg tagtgtgtat 8280
agtaggagat tttagataga ggtttgaggt ttggtagaat attggggatg tggggagtaa 8340
agaggttagg ggttttagta gaatagggggg tagggtgaga ggttagaggt tatagttaag 8400
tttaggaata taggagtatg agtttggatt ttggattttg gtgggagagt ggggagtgtg 8460
gggatatttt ttttcgtttt atattttagt gtcgtttgga gttttatttt tttttaagat 8520
gttgttagaa atagaaatgg gatggtagaa gggaagcgga ttacggaaaa ggaggagaaa 8580
gggagattag tagggaggga ggggatgggg tgttggggat tggtagggtt ggtgtgtttt 8640
agggggtatt gaatagtttg gcgttttatt ttagaggttt ttttttgtta gtgggttgga 8700
gttagtgaaa aggggatgat aattttttta gtagagttag tgtgagagtt tagtgaaatt 8760
ttgttttagt tttgtgattt gggtttttaa ttagagattt tttttgtttt tgtaggttag 8820
ggagtcggat gtttttttaag ttatttgtgt ttttttttatt tgggacgagt ttttcggttg 8880
atgggtttga tttttttagt tgtgtgttcg aagttatttt ggagttatat cgggttaaag 8940
gtattttttta tattagtttc gtttcgttgg aggttttggt tttatttggt ttaatttaga 9000
gttttttttt tatttttgag ttgtttttttt aatttttttgt atttcggttc gatttattag 9060
ttttcgtttt atttgttttt tttgaatcgg attttggtat tagttttgtt gttgatggtt 9120
tttggatata gagaggggag gaggaggagg tagaggttag agttaagttg gttttaggga 9180
gggagttttt tagtttttgt tatttagagg atagttttgg gttgggggta gtattttttg 9240
gtaggtaagt aatttttttt gtttttttaga tttgaggttt ttagggtttt agaaatagtt 9300
gttgtaaggg atagattttg agtgtgagga ttggtttata tttttgattt ttgagtaggg 9360
gtggttaagg gatgtagatt ttgttttttt atttggagga ttattgttat tagttgtgag 9420
ggagagtttt ttatagtatt tttttggtt ttaagtttag tggtttgaga aagtatgatt 9480
tagtattgaa ggattttagg agaatatttt ttttgttttt ttggagggtt tttttttttt 9540
ttggtagagt ttttagtttg tttagaattt ttgtgatttt atttttattt agtttgaagg 9600
ttttattaaa gatgtagtta ttagtttttt ttattgtttt tttttatgt tatttttgtt 9660
taattaggta tttttcgttt agttttagga agttttttta tagtttaatt tttttgtttt 9720
tttttttgtt agttattttt ttagttatgg aagttatggt aataggaggg ggtggttagg 9780
gagatcgggt tgtttagtaa ttaggagttt tcggtttttg tttttgggtt gataggtggt 9840
tttgcgagta gagtagttgg ggtgggaagg ggaggaaaga aagggaatga gggtatgtat 9900
ttcgattttt aatttttttaa ttttagagtt tgtttttgtg tgggtagagg gggagtatta 9960
aggagaggtt tttttttttta aaaagtagga gggggaaagt tttcggatta gtatttttgt 10020
tgtttttaaa tttttttttg gtttttttata gtttttttcgt ggtagtttat ttattttagt 10080
tttgtttata gtttaagaga taaaggatta tgttttgtgt agggttaggg tgggaataga 10140
taatttatgt ttaatagtaa atgtaggttt ttgggagttt gaggaagtag ttagaatttt 10200
tatagtagta agacggatgt gggttagatt tgaggtagga tttttataga gaggtggtaa 10260
tgatggagta ggttgatgga gagaatttaa tttttttttt tggaaaaaag aagaatgttt 10320
tttttatgtt tttttatttt tttttttttt ttattttttg ggtttggagg tagaaggagg 10380
gtttggatgg tttttaagga ttttgtttta gtttgagttg gtgttttatt tcggtagcga 10440
attattttg agttagttgg tgttcgattt agatttagag ttggatagta tagagcggtt 10500
ggttttggga agtatagata ttttgtttaa tgggtagaaa gcggatttgg aggttgcgta 10560
gcgtttggtt aagaggttgt atcgattaga tggttttagg aaggtcgatg tggttcggta 10620
tttgggtaag aagtaaagtt attaggttgg gattgaggat gggggtagag ggaatgtatt 10680
ttgaaggtag tttttttattt aatgtttttt ggttattggg taatttttat ttgatttttt 10740
gtattttttt ttgagatttt atttgttttt ttgttttgtt tatagtaatg attttagtaa 10800
attggtggtt ggggagtatt ttaagttttt tgtttttacg ggtatgattt tggattaagt 10860
ttttaggtgg gtgttgattt tttggaggat agtttttatt tttcggggtt cgtgttaggg 10920
ttttaggatg tttttttttga gtttttttgt tttttataag tcgttttgat attattttttt 10980
ttaagatggt tgggtatggt taagggttag agttgtttgg taaagggatt ttgggtaggg 11040
gtgatgggtg gagggttagg gtgtatattt ttattttttt tttttttttt tttttttattt 11100
ttagttgttg tatttttaaa gtttttttgtt attttagaga tagaggttgg aaatttttgg 11160
ttgttaggta atttttttttt gtttttttttt tagggtgttt ttgaaggagt tggttttaat 11220
gggtgagatt taggaacgag agcgcgtgtt ggtttatttt ttttagcgat atttttagtg 11280
taattttgaa gttttgtttt tagagggtga ggagtttcga gagtaggggt gtttttagtt 11340
ggttatagtg gtaaaatggg ttgggttttg tgtgtgtgta taggagtatg agtgtgtgta 11400
tagtagggaa tggatgtata tgcgtgtagg tatgtgaatt tgtatgtgtg tagatagcgt 11460
tgttatggtt gtatgtatgt ggaattatat atgtgggtag gtatattgtt agggttgagt 11520

gtgttgtgaa gaggtaggag tttgtatgtt tgtgtaggtt agtgtttggt tggagagggg 11580
attttgtttg gttgtattgg tttataggat tgtgttgcgg tttatttttt cgtggtttag 11640
gtttagaggt aagggaacgt tggggtaatt agtacgttgg ggtaattagt ttttagtatt 11700
tgagggagtt agggacgggt ttttgttgac ggatttgttg gaagaggagt tttttgtggg 11760
gagggtttat tagcggggtt taggaggtag gaagttgtcg gttaaagggg gtggggatga 11820
aatgggaggt tggggaagat ggttgtggga agggtttttg ggaggtagtt aaggttgaaa 11880
gttagttttt ttttagacgg cgtttatacg ttgatttgtg cgtttatgtt gtttaatacg 11940
gatttttacg gttatgtgag tggaggcggg cgggtaggag tcggaaaata aatttttggt 12000
tagatatgta ggatttggga ggttaagggt tttttaaatt tataggtttt cgtttttttt 12060
tttagaatat cgggaagcgt atgatttgcg gggattttat cgggaatttg gagggtttta 12120
atgatggcgg cgattttttt agggagttgt ttaaggtggg gggcggggta gggtagaagt 12180
cgttggaggg gaaggatgga ggatttagtc ggcgacggag tttagagtcg gagggagggt 12240
agggttgggg tttgtcgatt ttcggttttt gttacggaag ttcgggtttt cgtgatttag 12300
tttggggatc gttttttttt taggttttac gcgtaggttt tttcggaata tgcgtatatg 12360
cgtgcggtcg cgattgcgta cggcggggga ggggattcgg ttttggaaaa cgcgagtagg 12420
cggtttcggt ttttgcgttt gtttttagtt tttttttttt agtgtgcgtt ggggtggaag 12480
gcgggagttg cgggggtttt tgtacgggat agggattgga tgggggtata ttttatattg 12540
ttttttcgtc gcgtggatag ttaaaaagat tttttcggtg ttgttgcgtt gtaggaattc 12600
ggagggagag gggtttgaga gggattaagt ttttttaagg tttggtagcg agttagcgta 12660
aagttggagt tagaaattgt tttttggttt tttgtgagta ggtttcgttt ttaggcgtta 12720
gttttatggt tcgtaatcga gggtttagat acgtaggggt tggtgtcggt aggagaaggg 12780
agtggtttat ttagtgggtt tatttttttt tttgatttgg tggtagttta tttagagagg 12840
tttggattta tttgaagaaa tttttaggtt aggataaagg tgtttttgtt ttattatata 12900
tatgtaatag tataggattg ttttagggcg tttttgtgag tttggggtgt tgggttttttg 12960
agtttggggt atatttgaga gggaacgttt atggtgagtt ggggtgttgt ttgagttgag 13020
ttgtatttgt gtgttcgggc gtcgtgaatt aagggtttgt atggtttggt gtgtttgtgt 13080
tttagggttt acgatttaga gtgtgtgttt gagttggggt gtaggttgga tgtttatgtg 13140
tggtggggtt tatattttcg aattggtcgt gtttgaggta tgttatatgt ttttgagtag 13200
ggggtgtatt tttgtgagtt aaagatttta agttttgaag tgtttatttg aatgggtagt 13260
gtttgcgagt tggggtgatc gtgaattggt gcgtttggtt tgttggggtg tatgttagaa 13320
tcgaggtgtg ttacgtgatt ttagatttat atatgggtgg ggtatttgcg ggtgagttgg 13380
atgaggttag ggttgttttt tttttgtttt cgtttttttt cgaggttttt tttggtttag 13440
tggaaaaggg ttgtagggaa gcggttcgat tttttttttt ttattatttt aatttagatt 13500
agttagaagg gagttttaga ttttgtcggg ttcgtttagc gttatagttt taggtttagc 13560
ggtgttttta gttttttggg agttgttatt tacgttggtt tttttaggtt ttttttcgtg 13620
gtcgcgcggg gagggttttt ttcgcgatcg ggcgttttta ttttatgcgg ttttcgtttt 13680
tagttttcgt tttttttttt tcgtt 13705

<210> 127
<211> 3574
<212> DNA
<213> Artificial Sequence

<220>
<223> chemically treated genomic DNA (Homo sapiens)

<400> 127

tatatcgttt ttttcggtga gttattttat tttaagtatt tttttttatt atatgatttt 60
gttttatttt tttattgtat taagaatatt tgaaatgtat atgtttatat ttatatagtt 120
tatttttttt tgagaacgga agttttatag aaggtgaaat aaattttgtt agttgttgtt 180
agtcgtatag taagtttttt gtagaaaatg tttattggtt atattttttt aaaaagcgaa 240
taaattttaa gattaagatt ttaaattaaa ttattttagt ttttaattgt ttatagcgat 300
atattgagaa atcgagtgat ttttaagaga aaggttattt tgaggttggg gtcgaattaa 360
aatttagggg gtttatatta ggttagtatt tcggaattgg ttgttttgta agatttcggg 420
agtattaaat tggtatgaga tatttgataa cgaggttagt tatgtatcga gtttaggttt 480
tattttcgtt gtttttttga cgttagaaaa agtacgagtt gttgtatagt tttttttatt 540
aaggtgataa attatatttt cgttgggttt tgtattttgt aaattataaa tttatttttt 600
tttttttttt tatattgaaa aatgtaaaaa tttattgtag agaggttaaa attttttttta 660
aacgtaaata aatatcgttt tttagtagag gcgttttttt ttatttttgt tatatatatt 720
atcgcggatt cgttcgagaa agtacgtgat tttggcggta gttaggtttt taatagagaa 780

```
cgagggtttg tataaatttc gggttcgcgt cgtagagtaa gataaaggat tttgaattgt     840
agtattattt tttttagttt aaggtagttt gtgaaaggta ttgggagcgg cgtgacgcgg     900
gtatcgcgaa attttttttg ttgttatatg tatttgtgtt ttttttaatt atagtttagt     960
ataaatttta ggcgggcgat ttggtaggta aacggtgttt aggtagtcgg gtaatttatt    1020
gggttttagt gggtgttggg gagttttatg cgttaggagg tggataattt agggggtaag    1080
gggttcgtat aagggcgaag atgcgttttt ttttcggtat cgatttacgc gtttaaaggt    1140
ttttgcggta acgtttaggg acgcgaatta ggtgtcgggg gttggggagg aggtgggatt    1200
ggaagcgaga gtagtttgcg tttaagatcg cgtaagaggt ggttttttgt tcgttggttt    1260
tagtatattt cggttttcga aaaaatatta tttttgtttt ttgttatttt ttgtatttta    1320
gaaaattttt tttagggtag gaaaggatag tgagttattg taagtatttt ttagtaattt    1380
tttttcgcgt tttttttattt tttcgcgtta tagaggggta gagtattagg aaggacggta    1440
aatatgtttc ggtttagtgg tggttcgcgg tcgttattta gtcggtcgta cgtagtattt    1500
ttcggatatt atacgatttt ttttgggttt tgcggatata ggaaattttt attaaaggtc    1560

ggggatttaa atatttttag aatacggtcg cgttttcggg ttagttgcgt ttttttttta    1620
gacgtgcgtg gtttttggtt ttcgtaaaaa tggaattaga tttggtatta tattttgtta    1680
tagttatagg aaaatatatt aaagttatag gaaaataaat taaaaagtaa gttatagttt    1740
acgtaaaggt gaattcgggg gttttggcgt ttttttttcgt tgtcgcgtgc gttcgggaag    1800
tataggagat ttggattttt tttttttttaa aagtaatatt cgaaagtatc gggtatagtc    1860
gagttttttt tgtttttacg ttcgtagaga tgatttttac gttatttttt ttttcgatat    1920
ttaaattttt ttttgtcgtt cgtttagttt ttttcgggaa cggataatta ttttattttc    1980
ggaaataaag ggaaaggtgg taaaaggcga tgtggaggtg ttcgtttttt cggggttttt    2040
attttcggta gtttgatgtt ttttgtgtta aggtttggtt gcggaggtcg ggaaaatgtg    2100
gttttcgtta gtaagggttg ggtagggagt ttggcgtggt ttggcggatt tcggaggaag    2160
gtgaagggcg gaggtttttg gcgatttttt ggcggagtgg cgtggtcgtt ttagttcgcg    2220
ttcgtttggt tttttttttt ttcgggtcga ttgtattaga attttttttt attattaatt    2280
tttatttata ttttgttcgt cggtttcgta tttatatcga tatgtatcgg cgttgagggt    2340
tacgaatcgg agttattttt ttattggggg acgcgtggtt ttttcgtaat tatggtttgt    2400
gatggtttaa cgcggatagg ttattttttt tcgtttcgcg cgcggttttt tttttttttg    2460
tgcgttcgtt ttttaattaa agttgcgtgt tagtgagtcg tgtcggttat atggtgttat    2520
tgttggcggt cgtattttgt tgtttttttt ttgtagtttt ttgacgatgt agcgatcgcg    2580
gttggcggag attgagtaac ggggaaaggg tgagggaggg gttgcggggc gcgcgtgtgt    2640
tcggcgtatt gaagttggta tcgggagtag aggttgcgcg tcgttttggt atttgttttt    2700
ttttttttcg gggttcggcg gcgtgttcgg aatttagttg cggttgtagt tggagaatga    2760
gagtttttag ggatgggggt ggggcggtta aaggggtatc ggcggaggat aggagggttg    2820
tgcgtttcgt tggggtttgt attttgggta cgttagagtt tcgtcggggt cgaaggagcg    2880
agggagttgt attgtgggaa gggcgtagta tgaggatggt cgcgggagtg gtaggttcga    2940
gcgtagttcg gagagtcgcg ggtagcgttt gggattacgg gtagcgttgg agcggagggt    3000
tggttttggg tatagtttgt agatggttcg ggttggcggg aaggggatcg aagtggtagg    3060
ggcggcgggg agttcgggta tttttaaaaa tttttttgttt ttaatttgaa gcgggaggta    3120
tttttttttt ttcggattta gtattcgtag atttggttag ttttttttga gatttgtttt    3180
gtggagtttt gttttgggga ttgggtgtga ggtgaattag aaggatttgt tgagtatcga    3240
gaaagtatcg gtttaggcgt aaagagtcgg gggttagttt ttggagggtt tggagatttt    3300
acgtggtttc gatcgaagga tttggtgcgt ttaggttaac ggagttgggg cgttttaagt    3360
tcgggggacg agggagcggt atggagggga gtaggggtaa ttgggtttgg tttttttttt    3420
tttcgtttat tttttgtttt agatatagtt ttttttttatt ggttgggtat ttgtttgtag    3480
aggagttgtg ttttaaaatg gcgataggaa attttatttg cgttttagtt aattagattg    3540
ttatagcgcg aatttgtagg gtggaaacgc gagt                                3574
```

<210> 128
<211> 3574
<212> DNA
<213> Artificial Sequence

<220>
<223> chemically treated genomic DNA (Homo sapiens)

<400> 128

```
gttcgcgttt ttattttgta aattcgcgtt atgatagttt aattggttga ggcgtaggtg      60
ggattttttg tcgttatttt aagatataat ttttttgtaa gtaagtgttt agttagtgaa     120
```

```
aggggggttgt gtttgaaata aagagtaagc gggaaaaaag ggaattaggt ttagttgttt     180
ttgttttttt ttatgtcgtt ttttcgtttt tcggatttgg agcgttttag tttcgttaat     240
ttggacgtat taggtttttc ggtcgggatt acgtggggtt tttaggtttt ttaagagttg     300
gttttcggtt ttttgcgttt aggtcggtgt tttttcggtg tttagtaaat ttttttggtt     360
tattttatat ttagttttta aagtagagtt ttatagggta gattttagga agggttgatt     420
aggtttgcgg gtgttggatt cggaagaggg gaggtgtttt tcgttttagg ttagggataa     480
agggttttta aaggtgttcg agtttttcgt cgtttttgtt atttcggttt ttttttcgtt     540
aattcgggtt atttgtaggt tgtgtttagg gttagttttt cgttttaacg ttgttcgtag     600
ttttaggcgt tgttcgcggt ttttcgggtt gcgttcggat ttgttatttt cgcggttatt     660
tttatgttgc gtttttttta taatgtaatt ttttcgtttt ttcggtttcg gcggggtttt     720
ggcgtattta aaatgtaggt tttagcggaa cgtatagttt ttttgttttt cgtcggtgtt     780
tttttagtcg ttttattttt atttttaagg gtttttattt tttagttgta gtcgtagttg     840
ggtttcgaat acgtcgtcgg gtttcgggag ggagaggggt aggtgttagg gcggcgcgta     900
gtttttgttt tcggtgttag ttttagtgcg tcgggtatac gcgcgtttcg taattttttt     960
tttatttttt tttcgttgtt taattttcgt tagtcgcggt cgttgtatcg ttagagagtt    1020
gtaagaggga ggtagtaagg tgcggtcgtt agtagtgata ttatgtgatc ggtacggttt    1080
attgatacgt agttttggtt aaagagcggg cgtataggag gggaggagat cgcgcgcggg    1140
acggggagga atggtttgtt cgcgttaaat tattataagt tatggttgcg gaagggttac    1200
gcgtttttta gtaggagaat gatttcgatt cgtgattttt agcgtcggtg tatgtcgatg    1260
taagtgcgag gtcggcgagt agagtgtgag tgggggttgg tggtgagggg agattttgat    1320
gtaatcggtt cggaaaggag gagagttagg cgagcgcgga ttggggcggt tacgttattt    1380
cgttagaagg tcgttaggag ttttcgtttt ttattttttt tcgaaattcg ttaggttacg    1440
ttaagttttt tgtttaattt ttattgacgg gggttatatt ttttcggttt tcgtagttag    1500
attttgatat aaaggatatt aaattgtcga gggtaaaaat ttcggaaggg cggatatttt    1560
tatatcgttt tttgttattt ttttttttat tttcggaggt aaggtgatta ttcgttttcg    1620
gggagggttg ggcgagcggt aaggggagat ttagatgtcg gaggaggagg taacgtagaa    1680
attatttttg cgggcgtgaa agtagagaga gttcggttgt gttcgatgtt ttcggatgtt    1740
gtttttagga gaggaaaagt ttaggttttt tgtgtttttc gaacgtacgc ggtagcggga    1800
ggaagcgtta gggttttcga gtttattttt gcgtgggttg tggtttattt tttggtttgt    1860
tttttttgtga ttttggtgtg tttttttgtg gttgtggtag agtgtggtat tagatttggt    1920
tttattttta cgagagttag gaattacgta cgtttggagg gagagcgtag ttgattcgag    1980
ggcgcgatcg tgttttggga gtgtttgaat tttcggtttt tggtgaaaat tttttgtgtt    2040
cgtaaggttt agaggagatc gtgtgatgtt cgggggggtgt tgcgtgcggt cggttgggta    2100
gcggtcgcgg attattatta aatcggggta tgtttgtcgt ttttttttggt gttttgtttt    2160
tttgtaacgc ggggaagtag gagggcgcgg ggagaaatta ttaaaggatg tttgtagtga    2220
tttattgttt ttttttgttt tgaagaggat tttttaaaat gtagagggtg gtaggggata    2280
gaggtagtgt tttttcgaga gtcgggatgt gttggagtta gcgaataaaa ggttattttt    2340
tgcgcggttt tgggcgtaga ttgttttcgt ttttagtttt attttttttt taattttcgg    2400
tatttagttc gcgtttttaa acgttgtcgt agaggttttt aaacgcgtgg gtcggtgtcg    2460
ggaggagagc gtattttcgt ttttgtgcga gttttttgtt ttttaaatta tttatttttt    2520
ggcgtatggg gtttttttagt atttattggg atttagtagg ttattcggtt gtttgggtat    2580
cgtttgtttg ttaaatcgtt cgtttgaggt ttgtgttgaa ttgtagttag agaaggtata    2640
agtgtatatg gtagtaaaag gaatttcgcg gtattcgcgt tacgtcgttt ttagtgtttt    2700
ttataggttg ttttgggttg gggaaggtgg tgttataatt tagagttttt tgttttgttt    2760
tgcggcgcgg attcggggtt tgtataaatt ttcgtttttt gttagaaatt taattgtcgt    2820
taggattacg tattttttcg gacgaattcg cggtgatgtg tgtagtaaaa gtgaaaaaag    2880
acgtttttgt tagaaaacgg tatttatttg cgtttggaag gggttttgat ttttttgtag    2940
tgggttttta tattttttaa tataaggggg aggaggaaaa taaatttatg gtttgtagaa    3000
tgtagggttt agcgagggtg taatttgtta ttttgataaa agaaattgtg tagtaattcg    3060
tgtttttttt gacgttagaa ggataacgga aataaaattt gagttcggtg tatggttggt    3120
ttcgttgtta aatgttttat gttaatttgg tattttcgaa attttgtaaa atagttaatt    3180
tcggggtatt gatttggtgt ggattttttg gattttggtt cgattttagt tttaaaatgg    3240
tttttttttt gagagttatt cgattttttta gtatgtcgtt gtaagtaatt ggaaattgga    3300
atagtttggt ttgaggtttt aattttgagg tttattcgtt ttttaaaaaa atgtggttaa    3360
taaatatttt ttatagaggg tttattgtgc gattggtagt aattaataag atttgtttta    3420
tttttttatga agttttcgtt tttagggggga aatagattat ataaatgtag gtatatatat    3480
tttaggtatt tttagtgtaa tgaagaaata aaataggatt atgtgatggg ggaggatgtt    3540
tgaaatagag tgatttatcg ggaggggcgg tgtg                                3574
```

<210> 129
<211> 3506

<212> DNA
<213> Artificial Sequence

<220>
<223> chemically treated genomic DNA (Homo sapiens)

<400> 129

```
tagcggtaaa gttttgttta cgttaagtaa taaaggataa gttagttttt gttgtgatta      60
ttttgttgta ttgataagtt acgtattttt atttaaggat ttaaattttt atttttttta     120
agaattgggt taaaatcgat aaattaaatt tatttacggt ttattgatta aaggttgttg     180
tataataagt ttttgttatg tttagtagtt ggatttatag cgttagaaat ttataattgt     240
ttgatttttt tttttattat attgcgaaaa ttgtttttta aatgtaatta attttaaaat     300
tttaatagta tcgtggttag gcgtggtggt ttattattgt aatattaata ttaggtatag     360
gcgaggggat tgaggttagg atatcgaaat tagtttggga aatatacgga gattcggttt     420
ttggaaaaat aattagtttt gcgtggtggt gggcgcgagg tttcggttaa tcgggaggtt     480
atagtgagtt atgatgatat tgtattatag tttgcgcgac ggtttatgtt agtaagtttt     540
ggagtatttg aaataagttg tgttgggtat tttatttatt ggagagcgat tagtgattga     600
tgtttattta tagcgattag agacgtatgt ttcgatagta gtataaattt agtaggcgcg     660
aataaatggt aaagagaaat tgggtaaata agtattacgg tttttttagtt gagaaagtgg    720
gggttttaaa aagggttttt tgttgataga aagggacgtt taattatcga aatcgtagag     780
ggtgcggttt tggcgtttga gcgcgtagat tatatttatg gcggtgatcg ttttgcgttt     840
ggcgtgtttt gtataggtta cggcgtttcg gattacgttt tttaggaata tttttagtat     900
ttcgcgagtt ttttcgtaga tgaggtcgga gatgcgtttt acgtcgtcgc ggcgagtaag     960
gcgtcggatg gtcggtttgg tgatgttttg gatattgtcg cgtagtattt tacggtggcg    1020
tttagcgtcg tttttgttaa gattttttttc gtttttgtcg cggttagata tgacgagtaa   1080
gaggagtttt atttaacgtt ttgtgaggat tttggtttga ggtagcgttt ttatacgata    1140
gttggcggat cgaattgaga atttgaaaga agtcggcggg aagtttcgtt tcggtggggg    1200
aggggaaatt taaagggtta aatcgaaata gggggaaaaa aaaagcgagt tttttgtttt    1260
cgtgttttga attttgtaac gtgtatagta ttttgttatt acgttatgag gttttaaaaa    1320
attgtttttg aacgtagaag atatatatta atattgtggg aaatataaga aaggataaga    1380
aattaagaaa ttataatgtt attttattat ataggttagt taattatgta ttttgtagag    1440
tagttgtata tattttttta agaaaatgta tatagtgttg tatatggagt tttgtaattt    1500
ttttatattg attataattt aattaatttt tattaaagag ataaaagtga tgttttggtg    1560
tttatgtttt ttaggaatta ttaatagtta taattagttt tttagtaatt ttttaatcgg    1620
ttgtatttta aaaataatgt tttttatatt taatataaat gtattttttt tttatatttg    1680
ggattaatat tgaaatttat gattttatta tattaaaatt taaattttat tatattaata    1740
tttaaaattg tattagaggt tttatgattt ggtattacgg gttttcgtat tatttttttt    1800
ttaaattttt taatttgttt tattaaggtt tttggataat tttagagatt ttttgtgaag    1860
tttgaataaa attttttcga gattttgata attgtattag ttttaggatt taattggaat    1920
agaattaaaa tttttaaaat aagtttttat aattagaaaa ttggtgtttg taggttttgt    1980
gtgtggggtt tttttttttt tttggaagga gtttcgtttt gtcgttaggt cggagggtag    2040
tggtgtaatt tcggtttatt gtaattttta tttttcgggt ttaagcgatt tttatgtttt    2100
aattttcgga ttagcgcgga ttataggtat gcgttattac gtttagttaa ttttttgtat    2160
ttttagtaaa gaataagttt tattatgttg gataggatgt ttttgatttt ttgatatcgt    2220
gattcgttcg ttttagtttt ttaaagtgtt gggattatag gcgtgagtcg tttcgtttgg    2280
acgtttgtag gtttttaaaa agatattttt atatgattta attaaagatt tgttattaat    2340
tattatggag taattttgat ttcgtatatt tgattttttt ttttattaat aaatataggg    2400
ttatattttg aatttttttt tttttttttt tttttgtgga gacggagttt cgttttttcg    2460
tttaggttgg agtgtaatgg tttaattttg gtttattgta atttttattt ttcgggttta    2520
agcgattttt ttattttagt ttttttttgag tagttgggat tataggtatt tattattacg   2580
ttcggttaat ttttttgtat ttttagtaga gacggggttt tattatgttg gttaggttag    2640
tttttaattt tcgatttcgt gatttatatg tttcggtttt ttaatgtgtt gggattatag    2700
gtttgagtta ttgcgttcgg ttattttgaa attattttaa tattaatgaa aattataaaa    2760
tttttataat tattttaata taatttttta gtatttaatt ttttgtttag aaagtagtta    2820
agagttgaaa ttttggaaat agaaaatttt ggttttagat taaatgttta ttttttagat    2880
tcgagttgat ttgttatcga ttagatacga ggtgatttgt ttattttttt taaattttttg   2940
tttatttata aagttagaat aggatagtga cggtattgtt ggggtgtaga atgatttttt    3000
aaaatgtggt ttttgggtat gttgagcgtt tttgaatatt gaaaggtttt agaaataagt    3060
tttagaatta aagttttttt aatttcgttt ttttttttat atatgcgaag ggattttgat    3120
attttttaat cggattaaga aaattttta ttagaagtaa taattgtttt ttattttttt     3180
```

tttgttattt tattattgta gaaaagaata ttgaatatgg attttttaag ataatgtttg 3240
tttttcggtt ttatttaaat tattaagata tattaggtgt tgtggttttt tttattaatt 3300
ttagtattgt gggaggtcga ggtaggtgga ttttttgagt ttaggagttc gagattagtt 3360
tggttaatat ggcgaatttt tgtttttata aaatatataa aaaattagtt aggtggtgtt 3420
atatgtttgt aattttagtt atttgggagg ttgaggtagg agaattattt gaatttggga 3480
ggcggaggtt gtagtgagtc gagatt 3506

<210> 130
<211> 3506
<212> DNA
<213> Artificial Sequence

<220>
<223> chemically treated genomic DNA (Homo sapiens)

<400> 130

aatttcggtt tattgtaatt ttcgtttttt aggtttaagt gatttttttg ttttagtttt 60
ttaagtagtt gggattatag gtatgtgata ttatttggtt aatttttttgt atattttgta 120
gagataggga ttcgttatgt tggttaggtt ggtttcgaat ttttgagttt aagggattta 180
tttgtttcga ttttttatag tgttgggatt agtgggagga gttatagtat ttagtatgtt 240
ttggtaattt aaatgagatc gagaggtaga tattattttg aaaaatttat atttagtatt 300
ttttttgta ataatgaaat aatagggagg ggatagaagg taattgttat ttttggtaag 360
aaattttttt ggttcgatta ggaaatgtta gagtttttttc gtatgtatgg gaaagaagac 420
gaagttagag gggttttgat tttgaagttt atttttgagg ttttttaatg tttaaaagcg 480
tttagtatgt ttaagaatta tattttgggg aattattttg tattttaata gtatcgttat 540
tattttattt taattttgta gatgagtaaa gatttaaaaa gaataaataa gttatttcgt 600
gtttgatcgg taataagtta gttcgagttt gaaaggtaaa tatttaattt aaaattaaag 660
tttttttattt ttagagtttt aatttttaat tattttttaa ataaagaatt gaatattaag 720
agattatatt agaatagtta taggagtttt ataattttta ttgatgttaa aataatttta 780
gagtggtcga gcgtagtggt ttaagtttgt aattttagta tattgggagg tcgaggtata 840
tggattacga ggtcgggagt tggagattag tttggttaat atggtgaaat ttcgttttta 900
ttaaagatat aaaaaaatta gtcgggcgtg gtggtgagtg tttgtaattt tagttattta 960
ggggaggttg aggtgggaga atcgtttgaa ttcgggaggt ggaggttgta gtgagttaag 1020
attgggttat tgtattttag tttgagcgaa agggcgagat ttcgttttta taaaaaaaaa 1080
aaaaaaaaaa aaaatttaga gtataatttt gtatttatta ataagaaaga aaattaaata 1140
tacggaatta aagttgtttt ataatggttg ataataaatt tttagttggg ttatataaaa 1200
gtgttttttt aaaaatttat aagcgtttaa gcgaagcggt ttacgtttgt aattttagta 1260
ttttgggagg ttgaggcggg cggattacga tgttaagaga ttaagagtat tttgtttaat 1320
atggtgaaat ttgtttttta ttaaaaatat aaaaaattag ttgggcgtgg tggcgtatgt 1380
ttgtagttcg cgttaattcg gaggttgagg tatgagaatc gtttgaattc gggaggtgga 1440
ggttgtagtg agtcgagatt gtattattgt ttttcggttt gacgatagag cgagattttt 1500
tttaaaaaaa aaaaaaaatt ttatatataa aatttataaa tattaatttt ttagttataa 1560
gagtttgttt taaggatttt aattttattt taattaagtt ttaaagttaa tgtaattatt 1620
aaaatttcga agagatttta tttaaatttt ataaaaggtt tttaaagttg tttagaaatt 1680
ttggtgaaat agattaggaa atttggaaag gaaataatgc ggagattcgt agtattaaat 1740
tatgagattt ttaatataat tttaaatatt aatgtaataa aatttaaatt ttggtgtaat 1800
aaaattataa attttaatat tggttttaag tatagagaaa aagtatattt atgttgaatg 1860
tggaaaatat tatttttaaa atatagtcga ttaaaaaatt gttggggaat tgattataat 1920
tattgataat ttttaagaaa tatagatatt aaaatattat ttttattttt ttaatagaaa 1980
ttggttaaat tataattaat ataaggaggt tataaaattt tatatataat attgtatata 2040
tttttttgga aaaatatgtg taattgtttt gtaaaatata tgattaatta gtttgtgtga 2100
tgggataata ttgtagtttt ttaattttttt gttttttttt gtatttttta tagtattgat 2160
gtatattttt tgcgtttaaa agtaattttt taaagtttta taacgtggta ataaaatatt 2220
atgtacgtta taaaatttag aatacggaaa taagaagttc gttttttttt ttttttttatt 2280
tcggtttggt tttttagatt tttttttttt tatcggggcg ggatttttcg tcgatttttt 2340
ttaggttttt agttcggttc gttaattgtc gtataaaggc gttgttttag gttagagttt 2400
ttataaagcg ttgggtgaga ttttttttgt tcgttatgtt tggtcgcggt aaaggcggga 2460
agggttttgg taaaggcggc gttaagcgtt atcgtaaagt attgcgcgat aatatttagg 2520
gtattattaa gtcggttatt cggcgttttg ttcgtcgcgg cggcgtgaag cgtattttcg 2580
gttttattta cgaggagatt cgcggggtgt tgaaggtgtt tttggagaac gtgattcggg 2640

acgtcgtgat ttatatagag tacgttaagc gtaagacggt tatcgttatg gatgtggttt 2700
acgcgtttaa gcgttagggt cgtatttttt acggtttcgg tggttgagcg ttttttttta 2760
ttaataaaag gtttttttta gggtttttat tttttagtt gaggagtcgt gatgtttgtt 2820
tgtttagttt tttttatta tttgttcgcg tttgttgagt ttgtgttgtt atcggagtat 2880
gcgtttttag tcgttgtaag taggtattag ttattaatcg tttttagta aataaaatat 2940
ttaatataat ttgttttagg tgttttagag tttattgata tgggtcgtcg cgtagattgt 3000
agtgtagtgt tattatggtt tattgtagtt tttcgattag tcggaatttc gcgtttatta 3060
ttacgtaagg ttaattattt ttttaaagat cgggttttcg tgtgtttttt aggttagttt 3120
cgatattttg gttttaattt tttcgtttat gtttaatgtt ggtattatag tagtgagtta 3180
ttacgtttgg ttacgatatt gttgaggttt tagggttagt tatatttaag gggtaatttt 3240
cgtagtgtag tggggaggaa agttaagtag ttataggttt ttggcgttgt gaatttaatt 3300
gttgaatata gtaagaattt attatgtaat aatttttaat tagtggatcg taaataagtt 3360
tagtttatcg gttttggttt aatttttgag aaaggtgaga atttgaattt ttgagtagaa 3420
atacgtagtt tattagtata ataaagtgat tataataaag attaatttat tttttgttat 3480
ttaacgtggg tagagtttta tcgttg 3506

<210> 131
<211> 22489
<212> DNA
<213> Artificial Sequence

<220>
<223> chemically treated genomic DNA (Homo sapiens)

<400> 131

ggtcgttggg agatattgtg gttagtgggg cggggggttc gggtatttgt ttttagattt 60
tcggttattt taagtgatag gaagggggt tagtgaggat atgaatagtg gtcgttttta 120
tttttaaggg ttggttttat tgtttttttta tttttgtttg gtttagtagt tttgggagta 180
gttattttgt agtttatgcg tgtggggatt ttgtcgtgtt ttgagttgtc gaagtagttt 240
tttattgggt gttgtgttgg gtttttgttt ttattttgta gggaggaagt atagttgggg 300
gagtggtttt tcgagggttt ttgggagttt agattttatt tttggaggta gttttgggga 360
ggttcggtgg agatttgtt tttttgttga agttgttgtt gaagagtgat aggtttgttt 420
agcgtcgggg gagattcggg atttttagag ggagtgtagg atagggtagg gtagggtagg 480
gttatgattt tcggtgacgg ttaggttgtt atgggtcgaa ttgttatttt tgggtttgcg 540
ggtaggcgtt tagtttttta ggttttattt tttttattta aggaatggga tacgtatatt 600
ttcgtttttta gggtggtgta gagaatagga cggtgttttc gagtagtgtt tttaacgtcg 660
tttggggttt gggaatgtat taggatttga taaataaata ttagtttttta atatttcgtt 720
ttttgtaagg agaaggttag gaacgtttcg tttagagaga aggcgagttt acgagtagta 780
gatagtcggt gtttaatagg tgttcgtttt tttttagagt tttagggtgt tagttggtta 840
gagtgagttt cgttgattag ggagttgtgg tagtcggtcg ggggtcggtt tttggggtag 900
gtgggatgtt tgtggggtcg gcgtaggttt aggttttgtt tgggagtgtt ttgtttcgag 960
tattggtttg gatatttgag ttgagtttta gataggcgtt tcggggtcga ttcggtagga 1020
gtagtcgtta ttttcgggta gttgagttta gtagcgaggg ttggtagaac gatttagacg 1080
aaatattgaa tttaatagta aaattatttt tttcgaggga aaatacgatt cgtagttagt 1140
cggcgcgtat atatggggtt tggaaaagaa gagaatttcg gagatataaa cggttttacg 1200
cgtcgtcgtt tcggttcggt tgttaagtta ttaaattttt gtatagggaa aagcgataaa 1260
gtaaatattt atttttttcg tcgtttttag aagtgaaagg tttttgagat gggaaatgta 1320
gacggtttgt agagtgtttt atttaaataa aattattaga tgtttgaaga ttttgggaga 1380
aatgggttta gatgaattat ttgcgtaaag ttatttagta tttcgtaggg gtttcgaagt 1440
tcggtgtcgg taaggtgttc gtcgttggag taggggtggt tgatggattt ggtgcggggt 1500
gggcgggagg agtcgtagat ttagatgtag gaggtttggt ggggttattg atttacgttt 1560
ggttcggggt tttgtttttg gggtcgggag gggattaggt tggggaagtt ggggaggggg 1620
tttttttttt tttttttta gttgttaggt cgttagtggg agatataggt tttggggatt 1680
aggttttgta attttttttg atttcgacgt ttgagattgg tttggtgttg gttttgaggt 1740
ttttgtgtaa ttttagttcg ggttggttgc ggaggatttt tgagggtttt tcggtaaggt 1800
tttttttttt ttagttggtt atggtatata ggattttatt ttttcgtatg tttagggtag 1860
gtatcgttaa tcgagttttg tattttttttt aattttttgt attagggttt attaggttta 1920
tttatttttg tgtttagatt atttggtcga tgggcggttt tgttttagga ggaatagggg 1980
tttttttgtt tttttagagt tttcgttttg ggttttgtat ttacgtttcg gatgtgtcgt 2040
tttttttata tttgggagtt acgtcgattt tttttgcgtt tttttgggga gatatttttt 2100

tgtattagag atcggttttt cggggtttgg gcgtgggggc ggggatttgg atgttggggg 2160
tgtagaggtt atggtttcgt tatttttttt gattttgtta tgagagtatg attagggttt 2220
gttggttttt tgggttttga cgttttcggg ttcggtattt ttttggaagt tttatttatt 2280
tttttttttt ttttttttta taaggatttt tttatcgtaa ttttgtatga ttttatttta 2340
gttgattaaa tttgtaaaaa tggttaggtt cggtggttta tgtttgtaat tttagtattt 2400
tgggaggttg aggtaggtag attatttgag gttaggagtt taagattagt tcggttaata 2460
tggcgaaatt ttgtttttat taaaaatata aaaaatagtc gggcgtggtg gtaggcgttt 2520
ataattttag ttatttagga ggttgaggta ggagaattag ttgaatttgg gaggcggagg 2580
ttgtaatgag ttgagataac gttattgtat tttagttagg gtaatagagt aagattttat 2640
tttaataaat aaataaataa atttgtaaag atgttatttt cgtgtaaggt tatacgttga 2700
ggttttgggt ggatatgaat ttgggggata tcgtttagtt taggatagga attaattgta 2760
gtagggtagt tgtttcgggt atatagtttg attggtattt atttttggta ggtttttcgg 2820
agtttggttt ttttattatg gggcgggggt tttagttatt tttttggggg agtttttaat 2880
ttgaggtttt ttgtgtcggg gtttagttag ggtgggggtt gtattttatt tttggagtag 2940
gtatgttggg ttttgatagt aagttgggga gggttttagg gttagaggag gatttagggg 3000
ttgaaggtag tgatgggttt agtaggtagt agggagggta gggtagggga ggggtgttaa 3060
ggtttataag cgagaggagg taggagggag gatatggtat ttatagttgt ggtggaggag 3120
ggagggtgtt tataaggtgg gtttttagtt tggagtttta ggtatttttc gttgtggtta 3180
tcgattgttg gggaattgat tagttttata gtatggtatg gagttgggtt aggtagggtt 3240
ggatgttcgt taggggtatt gtaggtgggt tattaggggt tggtttgggg tttagttgtt 3300
atttagttga tacgggatta ttaatggttg ttatggttta ggtatttttt ttttgttacg 3360
ttggggatat tgtgatatag gtttttaggt tagtgaggat agtgtgggtg tcggagtttg 3420
gagggagggg agtggtatcg gggaggtttt cgtcgagttt ggatagtggg gtaggtggaa 3480
gggtgtgtgt tcggtcgtgt attttgggtt tggtgagcgt ggttgagggg tatagttgtt 3540
tttggtcggg gttgtagtga gtgtttagtt tatttggtta taggttggtt attagggatt 3600
tggcgtttgt tatttatagg attgaggatt ttaatagtag ttttagttta ggtttcgaaa 3660
agttgagtag agaagggtag gaggttttcg tatttttggg atttattttt ggtaggttgg 3720
gttgggttgt gggggatata tttgttttgg gggatgtggg aggggggcgt tttcgggttt 3780
gtagttgttg gtttcgagga tagacgtagt ttttttagta atagttgggt cggaggttcg 3840
ggagttaaga gagattagag tttaggttag ttcggttttt attagggatt agttatttcg 3900
gttatagatg ttagtagggg ggatttttag gggtgggta ttaggttggg gtgggtagtt 3960
ggggtgggtc ggggttgtag tgttttttta tttagttttg ggtataggac gagttagttt 4020
ttatttttgt ttatttagta tttcgtaggg gttgttgttt tgttgagtgt aggacgttga 4080
ttttaggatg ttattatttg atgttgttta ggtttttttt ttagaatatt tgtttagtga 4140
ttatagttat tttagttttt tttattgatt ttaggtagag taaggggttt tatttgtatt 4200
tagtaggtgt agattgggta gggtagggtt ttaggagggt gtatgtttgg ggagttttcg 4260
tacgtagata gtggggggtt agggaggagt tacgtgttgg gtataaggta gttttcgtag 4320
gtttttttgtt gggtttgttt tagtacggag ggatcggtag tatatagtag gtgtttagta 4380
aatgtcgggg ggcggggata agcgagtatt tacgggggga agtattggtt gcggtttcgt 4440
tatttgtttt ggtagtattt ttatttaggt tttggatggg cggcgattat cgtttttttg 4500
tttttggggt tttgattttt agtattcggt agtcggggat tcggtttttt ttaaggtttg 4560
ggagtagtta agtggttagg tattaagtgg taaatttagg ttagggttgg gtgacggttt 4620
ggagggtttt tgtggtagtt gagtttcgtt ttttttggtt ttgtttattt ttttggtgtt 4680
gtttattttt ttggtttgta tatttttttg gtttgtatat ttttttggtt tgtatatttt 4740
tttggtttat ttaggttgtt attggttcga gggttttagt gaggatgagt cggagatgga 4800
tattgagggg gttgacgggt ttggggtagg ttggtcgggg tagggggggtt gggtttgttt 4860
agcggtcgtt tgtttttaga agaatgatat tttgtttttt tttttgttcg aggaggtggg 4920
gtgagggtta gattttagta tttatgtttt tgtttttttt ttttttgaaa agtttataga 4980
tttttatttg aattgtttta gtagagttta ggcggggtat tatttttatt tatatatata 5040
aagggttgag atagtgtagt ttggcgtgtg agtagttttg gtgtttttag gttggggtgt 5100
tgttcgtttg tttatttggt tacggttttt aatagtcggt ttagtaaagg ataggtagat 5160
tttacgggtt aaggatgcgt tggtttggtc ggtgcggggt taggcgggta gtttgttagg 5220
atttggggat tttgagttag ttgttgtagt aggtgtggtc ggtttagtta ggtaagtttt 5280
gttgtcggga gggtcgttta ttagttgttg gagggtgtgg ggcggtcggg ttggttattc 5340
gtcggtagta aagttagtag agagtgggtg gggttggggg aggcgggagg gttttagttt 5400
tatttttagg gtttagtttt tttgggtttt gtagttaggt taggaaggtt agttgggttg 5460
gttttgggtt ttaggtttta gtttggaaat taggggtttt aattttggta tgagaaagtt 5520
ttcgttatag ggtaggagga gttttatata gaatttttaa tttttgtttt cgggtttgag 5580
tttggtagga ttggacgtcg gatatcggcg ttgttgggag ttttgagaat tgtacgtgtg 5640
ttttttagtg gttagcgtcg gtcgtgtttc gtttgatttt ttaggttttg atgcggggtt 5700
ttggggttta gggagtcgag gttcggggtg ggggttgtac ggtggtgtgg tttttttaga 5760

```
ttgtaggatt agagggtagt tttgaaagtt tttttggtta gggagttggt ttgtagagtt    5820
gaaggggagg agggggttag gaggttgttt gcggtcgtgg ttttgaaaat taggttttgt    5880
taagggtttt tgagggttag ttggggaggg aagggtatat agtgtgttta gagtaggatt    5940
ttagggcgtt cgggtttagc gtttttgggg ataaagggag atggagtaga ggggttttag    6000
ggaggttcgt ttcgtggtgg tataagagtt attgcgaata ttttgttttg gtgaggttgc    6060
gttttaggtg ttaatagtta tattttagta tattaggtgt aagttatttt ttataggttt    6120
tagtttgttg taggaatttt aagttttagg tcgggaagga atgggtaggg ttgtcgtagg    6180
gattgtggtt ttgtaggacg agggtttgtt aggggtgttt aggggatagc gggtcgggac    6240
gtgttggggt tgttgtaggg attgtggttt tgtggggtga gggttggttg gggtgttggg    6300
gttgtttttg ggtggttagg ttttatttga gagttcggtt atgggattta aggttagagg    6360
gtggtcgagt tgagggttgt ttgttttggg ggggtttttg gttttaaatt tatttatttg    6420
gggggcggta ttgaggtagg tttttaaggg tagaaagggg agtgatttgg taaaggtagt    6480
tttgtgttgt tttttttttt tcgtttatgt tacgtatatt ttaatgaaat aagaaatttt    6540
ttttgatttt tttgtgggtt tggtgatagc ggtattttgt ttagtttaga gtttttaatt    6600
tttcgaggta gtaggagtac gtaggtgggc gggtgtagga gttggagtga atatttttgg    6660
gtatagtagg agtagaattt cgtgtagttt tgtggtagtg tttagggtag tgtttgtgat    6720
tcgtgaagtt ttagagggtg tgtgttatag tgtttttagt tttgttgttt aaggatgttt    6780
ggagtgttta atagtttagt ggatttttttg tttttttttcg agggtagagg gttagtgtga    6840
tagtttttttg tattttaagt ttttgtttag tatttaggaa atattaggtt gtatgaagaa    6900
attgaaggat agtaaatgta agggatttta tggtcgatgg agggaggggg atttggaaag    6960
gggggtggag ggggaaggtg atggaagagg tttttagagg gagggagagt tggagagggg    7020
gtggaggggg aaggtgatgg aagaggtttt tagagggagg gggagttgga gagggggtgg    7080
agggggaagg tgatggaaga ggttgttaga gggaggggag ttggagaggg ggtggagggg    7140
gaaggtgatg gacgaggttt ttatagggag cgggagttgg agagggggtg gagggggaag    7200
gtggtggaag aggtttttag tgggaggggg agttggagag ggggtggagg gggaaggtga    7260
tagtttttttg aagttttgtc gtttttatta gatttttttt taaagttttg ttgtttagtt    7320
gtttttttga ggttacgttg ttttttttttg atgttaaatt gttattttcg atggttagtt    7380
gttttttttt tttttgtttg ttttgttttt tgttaataag gttaggagtt tttatgggta    7440
taggatgggg gtggggcgag gtagtggtgg ttttggaaaa ggtagtattg gagtagggaa    7500
attggaatgc gtgtttttat ttagggttat gtttttaggt ttgagggtgg ggtttttttag    7560
gaattttatt ttttttttatt tagaattttt gtttttttgtt tttattattg agggttcgtg    7620
aggttgatag tatttatttt taaagaagga ttggtttggg tagggttgtt aggagtatta    7680
gggtagggtt tgttaattta gaggttttcg aggaatagat ttaggtttgg gaacgggttt    7740
acggtagggt ttttttggaa atgtttggta gagtgggtag gggacgttat tgtgatttcg    7800
tattgtcggg gatattaggt ttagaggggt tgattgatta gttaaggtcg tataatagag    7860
ttgttgggag cgggtttttg ttaggtcgtg ggatataggg atttcggttt tcgttgtttt    7920
tgttttagtt ttagtttagg atttattgtg gttgttattg agttttagtt ttagagtcgt    7980
ttattaagtt cgattttacg tagggtttat tgtgtttatt agagggcgtt agaagtcggc    8040
ggtttcgata tcgcggttta aacgtaggga tcgttcggtg ggggttgttg taattttttt    8100
ttcggcgtgt ttaggttttt tttcgagggt ttttagggtc ggggtttggt cggggagaga    8160
attaggtgtg gtggagttgg ggagttcggc gttgggtagt gggcggtttg tgtttgggga    8220
tttcggtgtt attcgttagg tggtagggag gttggttttt tttaggagtt tttatttttt    8280
gaggtggttt taagtttttg ttgttgtttt ttttgtttta cgttttattt ttttgtttaa    8340
ttttttcgat agggttcggg tagggacgaa gtagggtttt tttattttgg agttgggagg    8400
aggggggtgg gggcgttatt aagtatgggg tttaggtaga tttagtttat gggggatatg    8460
ggggttttag ggtgaggatt tttggggggt tggtcggggg gcgtttagag tttacggaga    8520
gcggtttggt gggggaggtt ttgttagtag attttttttt tttttattcg gagtttcggt    8580
ttttattagt ttttattttt tgtttagtgt tcggtttttg tttgtttttg gttaatgttg    8640
ttatttgttt ttttagttaa agattttatg tttacgtgat atgattatga taggagttgt    8700
gggggggtttt agggaggtcg gcgttgggtt tgaatttgtt tcgtgaaggt ttcgagtttt    8760
cggttgtcgt attttgattg tttcgtgtga agggaaggga gttatagtta cgttataaga    8820
gaaggggttg ttgttttttt aattttcgtt ttttcgtttg ttttaatttg tttgggattt    8880
tttagagtcg gttgggggtt ttagtttagt tatttaagtt gtattttgtt ttaggaaatt    8940
tttacgggtt ttgattagtg tttattaggg tttttaggat attttggggg cgggaagtgg    9000
gggtaggttt tatgtttgta ttttatttgc gagttttcgg agtttttttg tatattttat    9060
agggttggag gggttttgtg ttttagacgg agtttcgttt tgttgttcgg gttggagtgt    9120
agtggtgtga tttcggttta ttgtaagttt cgtttttcgg gtttaaggga tttttttgtt    9180
ttagtttttt aagtagttgg gattataggt acgtgttatt acgtttggtt aatttttgtg    9240
tttttagtag agatggggtt ttattatgtt ggttaggttg gttttgaatt tttgatttaa    9300
gtaatttgtt tattttagtt ttttagagtg ttgggattat aggcgtgagt tattatatta    9360
ggtatgtttt tgagataagg tttcgttttg tttttttatat tggagtgtag tggtgttatt    9420
```

acgttttaag gtaattttaa atttttgggt ttaagtgatt tttttatttt agttttttta 9480
gtagttggga ttgtaggtgt gtgttattat gttttataag ttttttaatt ttttgtagag 9540
attggggttt cgttatgttg tttaggttgg ttttttgaat ttttgatttt aagtgatttt 9600
tttattttgg ttttatagaa agtgttggga ttataggcgt gagttattgc gtttagttta 9660
gggttagagg tttttttttg tttttttttt aatgtgtagg tgtttttga tgtttttttat 9720
ttttttgtt gggtttgggt ttcgatttat tttttcgatg ttttgagtag ggattggaga 9780
gtgtttttaa tgtgaaaata ttggtttttt tgtagtttag ttttagtttt ttatttgtta 9840
ttatatgggg aggtattatt tgggtgattt ttttagggtt gtttttttaat ttttatattt 9900
tttttggtt atatttaatt tgttattgaa gttattagtt gaggttttta tattaatagt 9960
tatatttttc gtgttaaagg ttttgtgtgg ttgtttttttt aatttgttgg gtttttttta 10020
tgttattata ttgtttttta tttttatttt tattttttta gttattttaa atttatgtat 10080
aattttttt tttttttttt gatagatttt tattttgtgg tttaggttgg agtgtagtgg 10140
cgtgattttg gtttattgta atttttattt tttaggttta agttattttt ttgttttagt 10200
tttttaagta gttgggatta taggcgttta ttattatgtt tcgttaattt tttgtatttt 10260
tagtagatat ggggttttat tttgttggtt aggttggttt taaatttttg attttaagtg 10320
atttgttttt tttagttttt taaagtgtta ggattatagg cgtgcgttat tacgtttggt 10380

ttataatttt ttaattgttt tattattatt tttggttttt aagatattat tgattgggtg 10440
tagtggttta tattcgtaat tttagaattt tgggagatcg aggtaggtgg attgtttgag 10500
tttaggagtt ggagatagtt taggtaagtt ggtgagattt tttttataaa aaaatagaaa 10560
acgtttattg gatttggtgg tattgtttat agttttagtt atttgggagg ttgaggtagg 10620
aggataattt gagtttaggg gtttaaggtt gtagtgagtt atggttgtgt tattgtattt 10680
tagtttgggt gatagagtaa gattttattt ttttaaaaaa aaaatggaat attaaaaatt 10740
agaagatggt gtcgtgtttg ttagtttttc ggggtgtgag tttattattg cgggttattt 10800
tttttatgga gtttaagttt taggggggata tatttttacg gagggtgtat gtttgttttt 10860
gttggagttt gggtggtttt taggttttgg aattagattt tatatttttt atttcgttta 10920
ttggttgtta gtggtgtttt attcgtgttt tggtgataga ggtagcgttt cgggggtata 10980
ggtatcggcg ttagttttga gtttgaattt tagttcggtt ttagttagtt gtatggttga 11040
gggtgggtgg gggatgtttt tgttttgatt ttttatattt ttatttatag tgtagtttta 11100
ttttttttat aggagtcgga gggttatatg ggaaaatagg tatggtagac gtaaaaggtt 11160
tagtggaaat gtcggttttt tttgttgtta ttattgtatt tatttggtaa tttcgtatta 11220
taattcgaga tttagaagat tttatgtttt aggcggggtt gaataggata gatttcgaag 11280
agtcggtttt tagatcggat cgttaataat taggtcggat ttcgcgtttt tagtcggtta 11340
gtgttttggt aaggtaggag cgggtgaaat ttttagtttt tttagggagt agttagatga 11400
tgatatattt atattcgttt atagggaggg agtgcgataa ggtttgtggt tttttgcgcg 11460
agaatcggtt agtgtttttt ggggttcggg atggggtttt agtatttatt tttattatcg 11520
gggttcggga gggagggttg gagagttttt atttggtgtc gtcggtggtt attttttaga 11580
tagcgcgggc gggttcgtag gttttgggta aatgtggtgt tgttatagtt tcgttttata 11640
gggatagcgg ttagtagttg tttttggttt cgtcgttttt aatgaagtta tagatggtag 11700
tttttaaatg tttttagaaa tggtagagag gtttttagag ttgacgtgta aataaattgt 11760
tttttaggtt cggattatat aattttgttg ggattaagta taggtttggg tttttcggtt 11820
gtttttttagg gtttggtttt gttgagagga ggaagtttgg tttttgggag gggggttttag 11880
gtgttttttt tatggagttt agtaatttgg taggtggggg tgggtatcgt tttttttttcg 11940
ggattttatt ttttcggtaa ttagttgttt tttttttttt tggggtttta ggtggttttt 12000
atggaggggt gggtgggatt gaggtttgag tttcggggag gagttggttg gtgagttatg 12060
tattttagtg gttattgcgg ttagtcgggg aggttgaatt ttaggtttta gtatttagaa 12120
gttggtggag tgggttgaat tgagtttttt taaaaacgta tgtcgaagtt ttgatttcgg 12180
gtatttgcga gtattggaaa tggggttttt gtgaatgatc gagttaagat gaggtcggac 12240
ggggagggtt ttaaaagtaa cgatcggtat tttgtaagga ggttgttgat agtagggaga 12300
agtcgggcgc gtgacgttag aggtagagat tggagagagg tagtttagga atattacggg 12360
tggttggtag tgcggagcgg tagggttttt ttttggaggt ttcggaggga gtgtggtttt 12420
gcggatattt cgattttagg tttgcggttt tagggttgga ggggtcgcgt tttcgtggtt 12480
ttagttttta gttcgtggtt ttttgttatg gtagtttcgg gagatttata taggcggtga 12540
ttgtggatag gatttttgat tttcgggtta tagttttttt atcggttttt gttacggggt 12600
aaataggttt ggggtggtgt ttaggtcggg gaattgtttt agttgtttgt cgtttttgtt 12660
gtggttgacg gggtcgtttc ggttgtagtt tcggttttgg ttttttgagg tttcgtattt 12720
tttttggtat ttattggttg ggttttcggg gtttttggtt atttcgtggg tgttgattga 12780
atcgggataa acgttgtgtg tagttaaggg gtgatttaag gtcggttagg tttagagttg 12840
attttaggtc gggaatagta gtagtgatgg tgtcggtatt tatcgtgttt tttttagggt 12900
tcggtcgtgg gagtcgaggg gacgtgtttg tggtttttgt ggtttgagtt tggaggttag 12960
cgtttagcgg tgtggtattt gggtattttt gttatttgtt tgtagttagt gtgttttcgt 13020

gggggtagc gaggtgatcg ggtttttgtt gggtgttttt ggaaggttta gaggtaagga 13080
tttttaggta gagagtttgg gggtggtttt aggaaatatt cgttattgag ggggtgttgt 13140
gaagggaagg ggtgggtgtt aatgggtagt agtgagtagg ttatcgttgt gggtagattt 13200
taggaggtag tatttcggag ttgtttttat tatagggaag tggttaggtt gtagtggttg 13260
tattttaggg tatggattta ttatattttt ggtcgttttt acgtttaggt taggtttttt 13320
gtggttagag acggttagag ttatagggt tgtaggaagt ggtcgtaggt atggttaggg 13380
gtaggtggta tttgtttcga gggtttgtac gtggtttttt tgtttcgtta gtttttgtta 13440
tgtgggtttc gggaaattgg cgtttaattt tatgagtagt tttgggtttt ggggttttag 13500
attttttaac gttgtcgtgt tcgtaggagg tgtttcgggg ttttagattt tttaatgttg 13560
tcgtgttcgt gggaggtgtt gtgggggttt cggatttttt aacgttgtcg tgttcgtggg 13620
aggtgttgtg gggttttag atttttaac gttgttgtgg ttatgggagg tgttgtgggt 13680
agggttgatt tcggggttta gttggttttt tttcgtttaa tgttagagaa gggaagtgtt 13740
gagtgtttga gggggattag taggggaagg gggtatagtt aggtttatgg ggtgattagg 13800
agtagagatt ttattttatt aaagtatata tattggttgc gtttgagggg aaaggagttt 13860
agggaggtgg atgaggtggg tagggggtgt tcgtcgttgg tttggacgtt tagggggta 13920
ttttattgtt tttggggttg gtaggtttta gattttttaa gtttacggtg ttaaacgtga 13980
ttttttatat tgggcggtag cggttataat tgtgaggggt aggtagtggt tgtttggacg 14040
gggacgtgtt tgttttttga cgaggagttt gcgttttatt taagtttttt atttgtattt 14100
ttggcggggt atttgcggtt tagtttggta gggattttga gtttgggttt gtgggggtgt 14160
ggtttttggg ggagggtaaa tttcggtggg tattggttat cggtgagtcg gcgtcgtggt 14220
tatagttttt atattttag tcgttgtagg ttttcgtttt cgtacgttat ttagcgttac 14280
gtttttgtat ttacgtagag ttacgtttta tttgtgcgtt tatgttgatt tattatcgat 14340
ttttttcgag tgaggagttt tcgttaaacg ttattaggtt tttagttttt agatgaaatg 14400
aaatggttga tgaattatta tattttaata tattaggaaa gatggattta tttgtattcg 14460
atgagtaaaa tgtatttttt tattgattga aaagttgatg aggtaaataa attagggaaa 14520
atggtttttt ttttattttt ttttgagtag taaattaagc gatttaaaag agttttgaat 14580
gagttaataa taaaatgttt ttaaatgtgg aaatgtgaag tgtaaatatt ttagttttat 14640
tgttagtgtt tatcgaggat atagtttgtt tttcgttttg ggaagttggg ggttgtggag 14700
gatttcggtt atttggatat ttagttatat tttaaagttt ttaaggggag atggggtttt 14760
tagtaagttt gttcggatta gttaagatga ggggttttag gagttaatat agatggagtt 14820
gttaggcgtg gtggtttaag tttgttattt taatatttg ggaggttaag gcgagaggat 14880
tatttgagtt taagagttta aggttagttt gggttatata gggagatttt ttttattttt 14940
aaaaagattt ttaaaaatta gttaggtttg atggtatttg tagttttagt tattcgggag 15000
gttgaggtgg gaggatcgtt tgagtttagg tattagaggt tttagtgagt tatgattata 15060
ttattgtatt ttagcgatag gtgagatttc gtttaaaaaa tattttataa taagagagag 15120
agagggaggg aggagtttgt atcggttgaa ttattttagc gttttggggt tgttgtagtg 15180
taggtttata atattagaaa tttattttttt tttagttttg gatgttacga gtttaaaatt 15240
acggtgtggg tagtacgtag tatatttttt tcggaggttt tggggaggat tttttttttt 15300
tttagttttg ggggttttac gtttttgttg cggtcgacgt tttagttttt gtttttgttt 15360
ttatagggtt tgttttgtgt atttgttttt attttgtttt gtttttttatg gaggaatatt 15420
agtgattgat ttgggattta gttttaattt agggtgattt cgtttttaga tttttaatta 15480
tatttgtaaa gttttattat tagggaaggt tgtattttga ggttttgggc ggatatgaat 15540
tttgggggta ggatattgtt tagtttagtg tcgggcgtcg ttgtgggtta ggtttttttga 15600
tttttgtaag tttacgatgg ttatagagtt ttaaggagga ggttttttgg aggaggcggt 15660
ttagtttatt agagaattag tatgtttttt ttttgggttt tgggattagt atttttaacg 15720
ataagttttt ttggattacg ttgattcggg ttgagatttg tcgtttgtcg cgcgttttgg 15780
gcggaggtta gataggtcgc gtatttttaa gttttgatac gtttttttttt taaagggtgg 15840
aggttttata gggtgagtgt aggttgggga tgggtcgtgg ggttgtcggg aggggattag 15900
tgggggtttg tagtgttttc gggagggttc ggggttgggg gtaatcgaga tttgtttcgg 15960
tgggtgagtg gagaagtatt tggggggttt tttttagcgt tggagagaaa tgggtgatga 16020
agtttgggaa aggcgtgggg ggttcgaagc gtgtattacg gtgagaaggt tgcgcgtggt 16080
cggattttag ttttttcgcg tttgcgaaag gttgagttgc gtcggtcggg ggagggtcgg 16140
aggttgtcgg gggttggggg atgaagagtc gggggtagtt tagggtagcg tatttttcgc 16200
ggaggtagga acggtaggcg gtgcgatttt tattaggatt tttgttatcg gaatcgcggg 16260
tttttgtcgg gtcggaggcg tttgagagat tttcgcggaa cggcgcgtga atttgcggtc 16320
gcggcggcgg gttcggatgg agatggacgt tggatgggtt gttttttgag tttggttttc 16380
gtggcggggt cggtttttcg tttttgtatt tggtagattt gatgcggagt tcgagggtgg 16440
ttgttggttt ggggcgggga aggggttttt tagatcgggg gttttatttg ttgggtttgg 16500
ggagtttatt tggtttttttt tgattggttt ggagttgtag gtgaggttat tttaggaagt 16560
tgttgtattt gagggagttc gggtcgtttt gggggacgtt ggggtttgat ttttggggtt 16620
gggggttgta cgggtgggtc ggggtttgtt tgggttgggg ttcgcggtcg cgtggggttt 16680

ggattggcgt ttgtttggtt tggggggtttt tagtcgtatg tagtttgggt cgaggtcggg 16740
ggttcgcggt cgcgtggggt ttgggtcggg gtttgtttgg tttgggggtt tttagtcgta 16800
tgtagttcgg gtcgaggttt gtagttgcgt ggggttcggt ttttatggtt tttgtgtttg 16860
gatttcgttt tttaattatt ttttttgtac gtttttttgg aaaaggatat ggggtttagg 16920
gtggtggatg tggatatttt ttgaaattta ttagttttgg tttgtgatgt ttggatggag 16980
gggttatagt tacgtcggtt tttagttttt ttttagatat tttagttttt ggaggtaggg 17040
tttttcgtg aggcgtatta tttttgagcg ttttatacgt ttgtttttgg gtattttttt 17100
gggtggtggt gtggtgcggt tttgcgtttt tatttaaatt ttatatggaa atggagtttg 17160
tagtgtgacg gtggggtttg gtggggagtg attgggtagt aggggcggat ttttcgtgaa 17220
tggtttggtt tcgttttttt tggtgttgtt ttcgagattg tgagtggtta tttaattgtg 17280
tttcgtattt gttttgtttt tttcgttttc gtttacgtta tgtgagatat ttgttttcgt 17340
ttcgtttttt attacggttg gaagtttttt ggggtttttt tagaagtaga aatatttgtg 17400
tttttatat agtttataaa gcgtgagtta attaaatttt tttttttata aattatttag 17460
ttttagggtt ttttttgttt gtttgtttgt ttgttttggt ggaggcgggg gtggatagcg 17520
tttttttttg tcgtttagga gggagtgtag tggggcgatt ttagtttaat gtatttttttg 17580
tttttgggtt gaagtgattt ttttgtttta gtttttttgag tagttgggat tatagttatg 17640
tgttattatg tttggttaat ttttatattt ttagtagaga cggggtttta ttatgttggt 17700
taggttggtt tcgagttttt aattttatga ttttttttatt tttaacgttt cgttttggtt 17760
ttttatagtg ttgggattat aggcgtgagt tattacgttc ggtcgtgggt attttttagt 17820
agtgtaagga tagattatcg tatttggtga acgtttttga cggtagtaat gaggtatatt 17880
tgggggtgtt tcgtggtaga tatatttgaa ggtggttggg gttgttagcg agggaatttg 17940
ggaggagtta atcggagatt taagtttttt ttatgagaaa tatttgagtt ttggtttgtt 18000
ttgtggaatt cgggttatgt tggggattga ggttttttgt tttgggttta acggaggttg 18060
cgggtggagg ttgttggggg agggtgttaa gtgaaaatgt tgtagggagg gcgtgttttt 18120
ggcgggggtt gtggttttgt ggtggtcgtg gtttttgttg tggttgcgat ttttgcggtg 18180
gttgtgattg tgggttttgc ggtggttgtg gtttttgaga tggttgtggt tttgtagtgg 18240
gtgtagtttt tttattttat ttgttgtcgt tggatttttt tttttgtgtg tagttttcgt 18300
taggatttta tgtttggatt ttggttttgg gttttttttt tggtttcgtg agattggcgt 18360
tatttttatt ggagttaata ggcgttcggt atagttattt ttagtttttt ttgtttttgg 18420
acgagttggt ggtcgttttt acgtaggaat gtcgatagtt tttggtatta tatagggtta 18480
ggattttggt tgtgattttt tttttagtga tttttttgcgg gattgatggt tagagagggg 18540
gtttgtcggt tttgtagtgt ttttttttatt attagttata ttgagttttg ggtaggagtt 18600
ttttggtttt ggttttattt attttaggag gatttagtga gtttagtttt tatttgggat 18660
atcgtagagg ttaagtttat tattgtgagg tttagtgggt gtcgggagtt atggtaattt 18720
cgtagggatg gttttcggtt tttttttttag ggattttatt ttagttgtga tttgttttac 18780
ggtatttgta tcgtgatatt tttttttgttg tttatgtggt tatgagggtt gtttttttgta 18840
cgtttatttt cgagttttag ttttaaagta gtgttgggtt tattaggttt ttttgtggac 18900
gtcgtttttg tttgtgcggt gggatgttaa tagttttcgc gttatgtggt tgttaatttt 18960
ggaagttcgt tttttttttt ttttttttcgt tatggttttg tttttatatt tcgttgtttg 19020
agttagcgtt tacggttgta ttagatgtta ggtttggttt tagttgaggg gttggtgtcg 19080
cgtacgtagg tagggggttt ggtaatgggg ttatattatt tgtatgaacg gttttgttat 19140
tttttttttgt tttgtttagt ataggttgag tttatagttt agatagtagt gtttcgagag 19200
ttgtttggta tatttcggga agaggatgaa ttttgtcgta tggggaggtt ttggtttcga 19260
gagttgtttg gtatatttcg ggaagagggt gaattttgtc gtatggggag tttttggtag 19320
gagtggggtt gtttggtgat cgcgtgaagt atttttcggg atgaattagg gttttgttaa 19380
agttgaggtt gggtcgggat ttttggggtt ttattgtttg ggatgaggcg ggttttagtt 19440
gggttgcggt aggggttagg aggtatttgt atttgttttg atttttttttt ttatatttgt 19500
agagtttttt tatttattga aaagtagggt gttttgggtg ggtttcggtt tgagtattta 19560
gagatttagt tttaagttaa gtttttttatt tttttttgagt tttagttttt ttatttttaa 19620
atgaagatgt ttaaggagag taggtttcgt tttttagttg gatttattag agtgtataga 19680
ggtgtttagg agagtaggtt tcgtttttta gttggattta ttagagtgta tagaggtgtt 19740
taggcgagta ggtttcgttt tttagttggg tttattagag tgttgttgta ggtttgaggt 19800
tttatttggg tggtataatt tgttggcgga ggaaagagtt gtcgtagttt aggtgggatt 19860
ggggggtttt tatgattttt taggagggtg ggggttttgt gtgggagttt tttattagtt 19920
ttagacgcgg tttttagggt atatttattt aaattttttta tttttatttt tttttaagtg 19980
taggttcggt agtggttttt atttggggtt gggagggggt aggtttttgg tgtttatggg 20040
acgttatttt atattgggta ttggtttagg tgttaggggga tagagtgggt attttagcgg 20100
gttgtgtggg tgagggtttt agtattgttt tcgtttttta ttttgaaggt tggagtagtt 20160
tgggaagttg tattttaatg agtattttat tttaatagag tgtttattga agttcgagat 20220
tacggttagg agggttagtt aggggttttag gggcgggatt taggtttttg tagaaggttt 20280
tatttttgaa attttttgag ggttttttttg tttttgtttg gatttcgggt tacgttttgt 20340

ttttggtagt tgtagtttgt attttatttt agacgttttg gtgggaagtt tttgttgtcg 20400
ggtttttagg ttggaattcg taggatgtta gggtattttg tgtttttggg gacgttttgc 20460
ggtagttttt tgaggttttt agatattagg atattttttt agtttagttt tagtaatata 20520
tgtcggtagc gtttgcgtta gtgttttttt tttttgtcgg gaggttggtg attcgttgtg 20580
ttgtttcggt ggggggttga tttttgattt tttgtttggc gggttgtgtt gtattagtta 20640
gggtcgggag gtgtacgtgg attcgtgttt attggaattt tgttgtgatt agattcgggg 20700
gcgacgtcga gatttattag gtttcgtggt tttttgtgaa tatttgtagg ttgttggggg 20760
tagcggtcga cggaataagt gtggggcggt tcgggttttt cggaggaagc gttgtttgag 20820
ttgttagggg agttatttgt agaaagggag gtggggtgta ttaggtaggg gggtagttag 20880
ggtaaacgtt tgatgtttag agggtgatag tgggtagcgg ggttttttgg aggagcggag 20940
ggagggtttt gttggttgta gggtagagtg ttttatattt tttttggtcg ttatttttag 21000
ggtttagttt agtttgggga ttaagacgag aggtttttgg ggtgttttag gtgagttgga 21060
gttagttttt gttgtttttg aataattata ggggagttag tttagtgatt aagtttttgt 21120
ttggtggggg aggggtacgg gttttgtgtt cgggagttgt tgtttttagt tttaggagag 21180
ggggtgttac gggtcgtggg ataggttata gttagggtgg ggtttttgga gaggggtttg 21240
aggatcgttt ttgcggagtt tatttagtta ttatcggttg ttatggttat tggtagtttt 21300
taatttttac ggtgttgggt ttgatttttg tagcgtttta ggcgggtatg attttatttg 21360
atttttttgg ggtgggtggg attgaggtta ggggattttt gtttagaatt tagggtggtc 21420
gatgtgggga ggggtattgt ggggtcgata ggtttttttt ttggttatta ggtttttagg 21480
agggtttagg tgtttggagg tggggggtgt tttagtttga tgttggggat gtgagtagat 21540
ttagcgttta tttattcgta gggttgagtt tttattgggc gttgggtcgt gattcgtata 21600
tcggattttg tatttttggg gtatttagtg attttagaat agtgggattt tagaggtttt 21660
ttagggagtt ttagaatatg atagatgggg gtttaggtgc gaggtagtgg gagttggagg 21720
aatatagtta ggggttgagg cgagaattat gaggatggtg ggtagaggtt gaggggaggg 21780
gtagtagatg gagatggggg aggcgttagg ggttataaag gggtttttta gaggatttgt 21840
tttgggaagt tattgttgtg ggagggtggg agggatatag tttaggtatt tttagagagg 21900
atggaaaggt ttatggggcg gattttgggt aggggatttg ggattagttt gtagtttttt 21960
ttgagggttt ggttatggtc gtaggttggg gaggagggga ggggattttg gggttaggtg 22020
ttatggtttt ttaagtattt ttgttcgatg gtaaatataa tacgcgttgt ttgtagaata 22080
tgtggaaata ttgaaacgtt taaaggagag aatatggttt cggtattcgg tttttaaggg 22140
agttgttggc gtttgtttaa taggagtcga ttttatttgt gaatagacgg taggcggttg 22200
tttttatgtt tttttagaga ggggtttttc gagttttgtt gttatttggg ggcgttttat 22260
tttatttagt tagtggcggg gtttttttggg tcggtataga gttaggggag ggggttgggg 22320
gatttttttgg tgggaaaatg ttcggtgatt tttagttttc gcgtttagcg ggaggaggcg 22380
ttcggtttcg tttttttataa ttagcggcgt ttacggcggg ttcggggatt agtatttcgg 22440
gagtttttta ggaatgtaga tttttaggtt tttattgttt ggggagtcg 22489

<210> 132
<211> 22489
<212> DNA
<213> Artificial Sequence

<220>
<223> chemically treated genomic DNA (Homo sapiens)

<400> 132

cgatttttta ggtagtgagg gtttgggaat ttgtattttt gagaagtttt cgggatgttg 60
attttcgggt tcgtcgtgag cgtcgttggt tgtaagaagc gggatcgagc gttttttttc 120
gttgagcgcg gaggttgggg attatcggat atttttttat taggaggttt tttagttttt 180
ttttttgatt ttgtgtcgat ttagggagtt tcgttattgg ttgaatgaag tgaggcgttt 240
ttaggtgata gtagaattcg gagggttttt ttttgagggg atatgggagt agtcgtttgt 300
cgtttgttta taggtggggt cggtttttgt tgaataggcg ttagtagttt ttttgaaggt 360
cgggtatcgg gattatgttt tttttttaa gcgttttagt gtttttatat gttttgtaag 420
tagcgcgtgt tatgtttatt atcgagtaag agtgtttaga ggattatggt atttggtttt 480
aaggtttttt tttttttttt ttagtttgcg gttatggtta ggtttttagg ggaggttgta 540
ggttggtttt aggtttttttg tttagaattc gttttataag tttttttatt ttttttggag 600
gtatttgggt tgtatttttt ttattttttt atagtagtgg ttttttaagg tagattttttt 660
gagaattttt tttgtgattt ttaacgtttt ttttattttt atttgttgtt ttttttttta 720
gtttttgttt attattttta tggttttcgt tttagttttt ggttgtgttt ttttagtttt 780
tattgtttcg tatttgggtt tttatttgtt atgttttggg gtttttttgag aagtttttgg 840

ggttttattg ttttggaatt attgggtgtt ttaggggtgt agggttcggt gtgcgagtta 900
cggtttagcg tttagtggga gtttagtttt gcgggtgggt gggcgttgga tttatttata 960
tttttaatat taagttggga tattttttat ttttaagtat ttgggttttt ttgggggttt 1020
gatggttaga gaggggattt gtcggtttta tagtgttttt ttttatatcg gttattttga 1080
gttttgggta ggagtttttt ggttttagtt ttatttattt taggaggatt aaatgaggtt 1140
atgttcgttt gggacgttgt agaggttaaa tttagtatcg tgaggattag gggttgttag 1200
tagttatggt aatcgatggt ggttggatgg atttcgtagg gacggttttt agattttttt 1260
ttagggattt tattttaatt gtgatttgtt ttacggttcg tgatattttt ttttttgagg 1320
ttgaggatag tagttttcgg atataaagtt cgtgtttttt ttttattagg tagaggtttg 1380
gttattgggt tgattttttt gtaattattt aaaagtaata ggggttgatt ttagtttatt 1440
tggaatattt tagaggtttt tcgttttagt ttttagattg ggttgggttt tgggggtggc 1500
ggttaggagg agtgtggggt attttgtttt gtagttaata gggttttttt ttcgtttttt 1560
taaagggttt cgttgtttat tgttattttt tgagtattag gcgtttgttt tggttgtttt 1620
tttgtttggt gtattttatt tttttttttg taggtaattt ttttggtagt ttaagtaacg 1680
tttttttcgg gaagttcggg tcgttttata tttgtttcgt cgatcgttgt ttttaatagt 1740
ttgtagatgt ttataggagg ttacggggtt tggtgggttt cgacgtcgtt ttcgggtttg 1800
attatagtag gattttaata aatacgggtt tacgtgtatt tttcggtttt ggttggtgta 1860
gtatagttcg ttagatagga ggttagagat tagtttttta tcggggtagt atagcgggtt 1920
attagttttt cgataggaga agaaaatatt ggcgtaggcg ttgtcggtat gtgttgttgg 1980
gattgggttg ggagggtgtt ttggtgtttg agagttttag ggagttatcg tagagcgttt 2040
ttaagagtat agggtgtttt gatattttgc ggattttagt ttggggattc ggtagtaggg 2100
gtttttttatt aaggcgtttg gagtgagatg taggttgtag ttgttaaggg tagagcgtgg 2160
ttcggggttt agatagaaat agggagattt ttaaagggtt ttaaggatgg ggttttttgt 2220
aggggtttga gtttcgtttt tggggttttg gttgattttt ttggtcgtgg tttcgagttt 2280
tagtgggtat tttgttaaaa tggggtgttt attaaaatgt agtttttttag gttattttag 2340
tttttaggat gggaggcggg ggtagtgttg aggtttttat ttatataatt cgttggggtg 2400
tttattttgt tttttggtat ttggattagt gtttagtgtg gagtgacgtt ttatgagtat 2460
taaagatttg tttttttttta gttttaggta gaagttattg tcgggtttgt atttgggggga 2520
aggtgaggat gaggaatttg agtgaatatg ttttggaaat cgcgtttgga gttggtgaga 2580
gatttttata taagattttt attttttttgg gggattatgg ggattttttta gttttatttg 2640
ggttacggta gtttttttttt tcgttagtag gttgtattat ttaggtgagg ttttaggttt 2700
gtagtagtat tttgataggt ttagttgggg ggcggggttt gttcgtttag gtatttttgt 2760
gtattttgat aggtttagtt ggggggcggg gtttgttttt ttgggtattt ttgtgtattt 2820
tgataggttt agttgggggg cggggtttgt tttttttggg tatttttatt tgaagatggg 2880
gaaattgagg tttagagagg gtaaagggtt tggtttggag ttgagttttt ggatgtttag 2940
gtcggggttt atttaggata ttttgttttt tagtgagtgg aggggtttttg taggtgtgag 3000
gagggagatt agggtagatg taggtatttt ttggtttttg tcgtagttta gttgggattc 3060
gttttatttt agataatgag gttttaggag tttcggttta gttttagttt tggtagggtt 3120
ttggtttatt tcgggaggtg ttttacgcgg ttattaggta gttttatttt tgttaaaggt 3180
tttttatacg gtaagattta tttttttttc gggatgtgtt aggtagtttt cggggttaaa 3240
gtttttttat acggtaagat ttattttttt ttcggggtgt gttaggtagt tttcggggta 3300
ttgttgtttg ggttgtggat ttagtttgtg ttgggtaggg tagggggaag tagtaaagtc 3360
gtttatgtag atggtatagt tttattatta ggttttttgt ttgcgtgcgc gatattagtt 3420
ttttagttgg ggttaaattt agtatttggt gtagtcgtgg gcgttgattt aggtagcggg 3480
gtgtgaaggt aggattatag cggggggagg aggaaggggg cgagtttttta aaattaatag 3540
ttatatgacg cggagattat taatatttta tcgtatagat agaggcggcg tttatagagg 3600
ggtttggtga gtttagtatt gttttagggt tggggttcgg gaatggacgt gtaaagggta 3660
gtttttatag ttatatggat agtaaggagg gtgttacggt ataggtgtcg tgggataggt 3720
tatagttagg gtggggtttt tggagagggg atcgaggatt atttttgcgg agttgttatg 3780
gttttcggta tttattgagt tttatagtgg tgggtttgat ttttgcggtg ttttaggtgg 3840
agattggatt tattggattt ttttggggtg ggtgggatta aggttagggg atttttgttt 3900
agaatttagt gtggttgatg gtgggagggg tattgtaggg tcgataggtt ttttttttgg 3960
ttattagttt cgtaggaagt tattggggga ggggttatag ttaggatttt gattttgtgt 4020
gatgttaaag gttgtcggta tttttgcgtg ggagcggtta ttaattcgtt tagagataga 4080
gggagttggg ggtagttgtg tcgaacgttt attggtttta gtggagatgg cgttagtttt 4140
acgaggttag agaagagatt tagagttagg atttagatat ggggttttag cgggggttat 4200
atatagggga gagggtttag cggtagtagg tggggtggga gaattatatt tattgtaaaa 4260
ttataattat tttaaaaatt ataattatcg taaaatttat agttataatt atcgtaaaaa 4320
tcgtaattat aataaaaatt acgattatta taaaattata attttcgtta gaagtacgtt 4380
ttttttatag tatttttatt tagtattttt ttttaataat ttttattcgt agttttcgtt 4440
gaatttaaaa tagaggattt taatttttag tatggttcgg gtttttatagg ataggttagg 4500

```
gtttagatgt tttttataga gaggatttgg gttttcggtt ggtttttttt agatttttttc    4560

gttggtaatt ttaattattt ttaggtgtgt ttgttacggg gtatttttag gtgtgtttta    4620
ttattgtcgt taggggcgtt tattaggtgc ggtggtttgt ttttgtattg ttagaaaata    4680
tttacgatcg ggcgtggtgg tttacgtttg taattttaat attgtgggag gttaaggcgg    4740
ggcgttgggg gtagggggat tatgaggtta agagttcgag attaatttgg ttaatatggt    4800
gaaatttcgt ttttattaaa agtataaaaa ttagttaggt atggtggtat ataattgtaa    4860
ttttagttat ttaggaggtt gaggtaggag aattatttta atttaggagt agagggtgta    4920
ttgagttgag atcgttttat tgtatttttt tttgggcgat agagagagac gttgtttatt    4980
ttcgttttta ttaaaataaa taaataaata aataaaaaaa attttgagat tgggtaattt    5040
atagagaaaa gagtttgatt ggtttacgtt ttgtaggtta tatggaaagt ataggtgttt    5100
ttgtttttgg agaggtttta ggaagttttt aatcgtggtg gaaggcgaag cgggagtagg    5160
tgttttatat ggcgtgagcg ggaacgagaa agatagagta ggtgcgggat atagttaaat    5220
ggttatttat aatttcgagg atagtattag gagggacggg gttaagttat ttacgagaaa    5280
ttcgtttttg ttatttagtt atttttttatt aggttttatc gttatattgt agattttatt    5340
tttatgtgag gtttgggtgg ggacgtagag tcgtattata ttattattta ggaggatgtt    5400
taggagtagg cgtgtggggc gtttagggat gatacgtttt acggggggat tttgttttta    5460
ggggttaggg tgtttgggaa gaggttggga gtcggcgtgg ttgtggtttt tttatttagg    5520
tattatagat tagggttggt gggttttagg gggtgtttat atttattatt ttgaatttta    5580
tgtttttttt tagggaaacg tgtagaggaa atggttggag agcgaagttt aggtataggg    5640
gttatgagag tcggatttta cgtagttgta gatttcgatt cgaattgtat gcggttgagg    5700
atttttaagt taggtagatt tcgatttaga ttttacgcgg tcgcggattt tcgatttcga    5760
tttaaattgt atgcggttga ggatttttaa gttaggtaga cgttaattta gattttacgc    5820
ggtcgcggat tttaatttag atagatttcg atttattcgt gtagttttta gttttagaag    5880
ttaaatttta gcgtttttta gggcggttcg ggttttttta gatatagtag ttttttgagg    5940
tggttttatt tgtaatttta gattaattag agaaggttaa atgggttttt taagtttagt    6000
aggtgggatt ttcgatttag agagtttttt tttcgtttta ggttaatagt tattttcggg    6060
tttcgtatta ggtttgttaa atgtaggggc gaggggtcga tttcgttacg ggaattaaat    6120
ttaggaaata gtttatttag cgtttatttt tattcggatt cgtcgtcgcg gtcgtaggtt    6180
tacgcgtcgt ttcgcggaga ttttttaggc gttttcggtt cggtagaaat tcgcggtttc    6240
gatggtagag gttttggtgg gaatcgtatc gtttgtcgtt tttgttttcg cggaggatgc    6300
gttgttttaa attgttttcg gtttttttatt ttttaatttt cggtagtttt cgattttttt    6360
tcggtcggcg tagtttagtt tttcgtagac gcgggggagt tggaattcga ttacgcgtag    6420
tttttttatc gtggtgtacg tttcgggttt tttacgtttt ttttaggttt tattatttat    6480
tttttttttag cgttgaagga aattttttag atgttttttt atttatttat cgaagtaggt    6540
ttcggttgtt tttagtttcg ggttttttcg agaatattgt aagtttttat tggttttttt    6600
tcggtaattt tacggtttat ttttaatttg tatttatttt gtggggtttt tattttttgg    6660
aggaaggacg tgttaaagtt tggaggtgcg cggtttgttt aattttcgtt taaggcgcgc    6720
ggtaggcgat aggttttagt tcggattagc gtggtttaaa aggatttatc gttaaaagtg    6780
ttggttttag aatttaaggg gaagatatgt tggtttttttg atgagttggg tcgttttttt    6840
taggaagttt tttttttgga gttttgtagt tatcgtgggt ttgtaagagt taaagggttt    6900
ggtttatagc ggcgttcggt attgagttgg atagtgtttt gtttttaaaa tttatgttcg    6960
tttagaattt tagaatgtaa tttttttttgg tagtagggtt ttgtagatgt aattaaggat    7020
ttggggacga ggttattttg gattgaggtt gagttttaag ttagttattg gtgttttttt    7080
atgaagagta gagtagaata gagatagatg tatagagtag gttttgtgag gatagaggta    7140
gggattggag cgtcggtcgt agtaggggcg tggagttttt agagttggaa gagggaggga    7200
tttttttttag ggttttcgga aggagtgtgt tgcgtgttgt ttatatcgtg attttggatt    7260
cgtggtattt agaattggga gagaataaat ttttgatgtt gtaagtttgt attatagtag    7320
ttttaggacg ttgaaatagt ttagtcggtg taggtttttt tttttttttt ttttttttgt    7380
tgtgagatat tttttagacg gggttttatt tgtcgttgga gtgtagtggt gtgattatag    7440
tttattggag tttttaatgt ttgggtttaa gcgatttttt tattttagtt tttcgagtag    7500
ttgggattat aggtgttatt agatttggtt aatttttaaa aattttttta gagatggggg    7560
aggttttttt atgtggttta ggttggtttt gaattttttgg gtttaagtga ttttttcgtt    7620
ttggtttttt aaagtgttag gatggtaggt ttgagttatt acgtttggta gttttatttg    7680
tgttgatttt tgaagttttt tattttggtt aattcggata ggtttgttgg ggattttatt    7740
tttttttgga gattttggga tgtggttgga tgtttaagtg gtcggggttt tttatagttt    7800
ttagtttttt agggcgggag gtaggttgtg ttttcggtgg gtattgatag tgaggttgaa    7860
atgtttgtat tttatatttt tatatttagg ggtattttgt tgttgattta tttaaagttt    7920
ttttaagtcg tttaatttgt tgtttagagg gaaatgaaaa gggagttatt ttttttgatt    7980
tgtttgtttt attaattttt taattaataa aaaagtgtat tttgtttatc ggatgtaaat    8040
aggtttattt tttttaatat gttgaaatat agtgatttat tagttatttt attttatttg    8100
```

gaagttgaaa gtttaatggc gtttggcgag aattttttat tcgagaagag tcggtaataa 8160
gttagtatga acgtataggt ggggcgtggt tttgcgtggg tgtagaagcg tggcgttggg 8220
tgacgtacgg aaacggggat ttgtagcggt tgggagtgtg aaggttgtgg ttacgacgtc 8280
ggtttatcgg tgattagtgt ttatcggggt ttgttttttt ttagaggtta tatttttata 8340
gatttaaatt tagggttttt gttaggttgg atcgtagatg tttcgttagg gatgtaggta 8400
ggggatttga atgaggcgta aatttttcgt taaaggataa gtacgttttc gtttaggtag 8460
ttattgtttg ttttttatag ttgtagtcgt tgtcgtttag tatggggggt tacgtttggt 8520
atcgtgagtt taggggggttt ggagtttgtt agttttaaag atagtaagat gttttttttag 8580
gcgtttaggt tagcgacggg tattttttgt ttattttatt tattttttttg ggtttttttt 8640
tttttagacg tagttagtgt gtgtgttttg gtggggtggg gtttttgttt ttggttattt 8700
tatgagtttg attgtatttt tttttttttgt tgattttttt taaatattta gtattttttt 8760
tttttagtat taggcgggga ggaattagtt gggtttcggg gttagttttg tttatagtat 8820
tttttatggt tatagtagcg ttgggggatt tgaggttttt atagtatttt ttacggatac 8880
ggtagcgttg gggggttcga ggtttttata gtatttttta cggatacgat agtattggga 8940
gatttgaggt ttcggagtat tttttacgga tacggtagcg ttgggggatt tgaggtttta 9000
gagtttaggg ttgtttatgg aattgggcgt taatttttcg ggatttatat ggtagaggtt 9060
ggcggggtag ggaagttacg tgtaggtttt cgaagtagat gttatttgtt tttgattatg 9120
tttgcggtta ttttttgtaa tttttgtgat tttggtcgtt tttgattata ggaggtttgg 9180
tttgggcgtg ggaacggtta ggagtgtggt gagtttatgt tttggggtat agttattata 9240
gtttggttat ttttttatgg tagggatagt ttcggggtgt tgttttttgg ggtttgttta 9300
tagcggtgat ttgtttatta ttgtttattg gtatttattt tttttttttta tagtattttt 9360
ttaatgacgg gtgtttttttg gggttatttt taaatttttt gtttgaagat ttttgttttt 9420
gagtttttta ggggtattta atagagattc ggttatttcg ttgtttttta cgaggatata 9480
ttggttgtag gtaggtgata gaggtgttta ggtgttatat cgttgggcgt tgatttttag 9540
atttagatta tagaggttat agatacgttt tttcggtttt tacggtcggg ttttgggagg 9600
gatacggtgg atgtcgatat tattattgtt gttgttttcg gtttgggatt agttttgggt 9660
ttggtcggtt ttgagttatt ttttagttgt atatagcgtt tgtttcgatt taattagtat 9720
ttacggggtg attagaggtt tcgagagttt agttagtggg tgttaggaag ggtgcgaggt 9780
tttaaggagt tagggtcgga gttgtagtcg ggacgatttc gttagttata gtaaggacgg 9840
taggtagttg gggtagtttt tcggtttaag tattatttta gatttattta tttcgtggta 9900
ggggtcggtg gggaaattgt ggttcggagg ttaggggttt tgtttatagt tatcgtttgt 9960
gtgagttttt cggggttgtt ataataaaga gttacgaatt gggagttaaa gttacggaaa 10020
cgcgattttt ttagttttgg ggtcgtaagt ttgagatcga ggtgttcgta gggttatatt 10080
tttttcgaag tttttagggg agggttttat cgtttcgtat tgttagttat tcgtggtgtt 10140
tttgggttgt tttttttttag tttttgtttt tgacgttacg cgttcgattt ttttttgttg 10200
ttagtagttt ttttataagg tgtcggtcgt tgtttttggg atttttttcg ttcgatttta 10260
ttttaattcg attatttata aagattttat ttttagtgtt cgtaggtatt cggggttagg 10320
gtttcgatat acgtttttgg aggggtttaa tttaatttat tttattagtt tttgagtgtt 10380
ggggtttggg atttaatttt ttcggttggt cgtagtgatt attgggatat atggtttatt 10440
agttagtttt ttttcggggt ttaggtttta gttttattta ttttttttatg aggattattt 10500
ggggtttttag agaaggaga ggtagttgat tgtcggggag atggagtttc gggggagggg 10560
cggtgtttat ttttatttgt taggttgttg ggttttatgg agggggtatt tgagattttt 10620
ttttaagagt taggtttttt ttttttagta gagttaggtt ttgaggggta gtcggagggt 10680
ttaggtttgt gtttggtttt agtagaattg tgtggttcga gtttgagaag taatttattt 10740
gtacgttagt tttgggagtt tttttgttat ttttggagat atttaagagt tgttatttat 10800
aattttatta gggacggcgg ggttaggggt agttgttgat cgttgttttt gtagggcggg 10860
gttgtggtaa tattatattt gtttagggtt tgcgaattcg ttcgcgttgt ttgaggagtg 10920
gttatcgacg gtattagatg gaggtttttt agttttttttt ttcgagtttc ggtagtgaga 10980
gtgggtgttg aaatttttatt tcgggtttta aaggatattg gtcgattttc gcgtagaaag 11040
ttataggttt tgtcgtattt tttttttgtg gacgggtgtg gatgtgttat tatttgattg 11100
ttttttggga gggttgggag ttttattcgt ttttgttttg ttaaggtatt gatcggttgg 11160
aggcgcggaa ttcggtttgg ttgttggcgg ttcgatttgg ggatcggttt ttcgaggttt 11220
gttttgttta gtttcgtttg gggtataggg ttttttaggt ttcgggttgt ggtgcggggt 11280
tgttagataa atgtagtaat ggtagtaagg agaatcggta tttttattgg gttttttacg 11340
tttattatgt ttgttttttt atgtggtttt tcgatttttg tgagggaggt gaggttgtat 11400
tataggtgag gatgtggggg attaaggtag gggtattttt tatttatttt tagttatgta 11460
gttgattggg gtcgggttgg ggtttagatt taaagttgac gtcggtgttt gtattttcgg 11520
ggcgttgttt ttgttattag gatacggatg gggtattatt ggtagttagt gggcggggtg 11580
ggggatatgg agtttggttt taggatttag aggttattta ggttttagta aaggtaaata 11640
tgtatttttc gtggggatat gtttttttgg ggtttgggtt ttatagaaga aatggttcgt 11700
aatggtgagt ttatatttcg agggattggt agatacggta ttattttttta atttttaatg 11760

```
ttttattttt tttttaaaga gatgaagttt tgttttgtta tttaggttgg aatatagtgg   11820
tataattata gtttattgta gttttggatt tttaggttta agttattttt ttgttttagt   11880
tttttaagta gttgggatta taggtagtgt tattaagttt aataaacgtt ttttgttttt   11940
ttgtagaaag agttttatta gtttgtttag gttgttttta gtttttgggt ttaagtagtt   12000
tatttgtttc ggttttttag agttttaaga ttacgggtgt gagttattgt atttagttaa   12060
tagtatttta agaattagaa ataataatag aataattaag agattatagg ttaggcgtgg   12120
tggcgtacgt ttgtaatttt agtattttgg gaggttgagg agggtagatt atttgaggtt   12180
aggagtttga gattagtttg gttaataagg tgaaatttta tgtttattaa aaatataaaa   12240
aattagcggg atatggtggt gggcgtttgt aattttagtt atttgggaag ttgaagtagg   12300
agaatggttt gaatttggga ggtggaggtt gtagtgagtt aagattacgt tattgtattt   12360
tagtttgggt tatagagtga gaatttgtta aaaaaaaaaa aaaaagatta tatatgagtt   12420
taaaatgatt aaagagataa aagtagaaat gaaaaataat ataatagtat gaaaaggatt   12480
tagtagatta gaaaaataat tatataggat ttttaatacg aggaatatgg ttgttggtat   12540
aaaaatttta gttgatggtt ttaatagtag attagatgtg gttaaaagag aatatgggag   12600
ttggaagata gttttgagga agttatttag atgatgtttt tttatatagt agtaggtaag   12660
agattgaaat taagttgtag aaaggttagt gtttttatat tgaagatatt ttttaatttt   12720
tgtttagaat atcggaaaaa tgaatcgaga tttagattta ataaagaggg tgggagatat   12780
taggaaatat ttatatatta aaaaaaaaat aaaaaaaaat ttttaatttt aggttgagcg   12840
tagtggttta cgtttgtaat tttagtattt tttgtgaggt taaggtggga ggattatttg   12900
aggttaggag tttaagagat tagtttggat aatatagcga gattttaatt tttataaaaa   12960
attaaaaaat ttgtggggta tggtggtata tatttgtagt tttagttatt ggggaggttg   13020
aggtgggagg attatttgag tttaggagtt tgaggttgtt ttgaggcgtg atggtattat   13080
tgtattttag tgtgggggat agagcgaggt tttgttttaa aaatatgttt ggtgtggtgg   13140
tttacgtttg taattttagt attttgggag gttgagatag gtagattgtt tgggttagga   13200
gtttaagatt agtttgatta atatggtgaa attttatttt tattgaaaat ataaaaatta   13260
gttaggcgtg gtggtacgtg tttgtagttt tagttatttg ggaggttgag gtaggagagt   13320
tttttgaatt cgggaggcgg agtttgtagt gagtcgagat tatattattg tattttagtt   13380
cgggtaatag agcgagattt cgtttgaaat ataaaatttt tttagttttg tgaagtgtgt   13440
aaagaggttt cggaagttcg tagatgagat gtaggtataa agtttgtttt tatttttcgt   13500
ttttagaatg ttttggggat tttggtgggt attggttagg gttcgtgggg gtttttttggg   13560
gtaggatgta gtttgggtga ttgggttgag gtttttagtc gattttgggg ggttttaggt   13620
aggttggggt agacgggagg gcgggggttg gggaggtagt agttttttttt tttgtggcgt   13680
ggttgtgatt tttttttttt tatacgaagt agttaggatg cggtagtcgg gggttcgggg   13740
tttttacggg gtaagtttaa gtttagcgtc ggtttttttg ggatttttta tagtttttgt   13800
tatgattatg ttacgtggat atgggatttt tggttgaaga gataaatggt agtattgatt   13860
aggggtaggt agaggtcgga tattgagtag gaagtggggg ttgatgggag tcgggatttc   13920
gggtggggga agggagggtt tgttggtaag gttttttttta ttaggtcgtt tttcgtgggt   13980
tttgagcgtt tttcgattag ttttttaggg gtttttattt tgggattttt atgtttttta   14040
tgaattgaat ttgtttggat tttatgtttg gtgacgtttt tattttttttt tttttagttt   14100
tagggtgggg ggattttgtt tcgtttttgt tcggattttg tcgggggaat taaatagggg   14160
gatggggcgt ggggtagaag ggatagtagt aggggtttag ggttatttta gaaagtgggg   14220
gtttttggag gggattagtt tttttgttat ttggcgggtg atatcggggt ttttaggtat   14280
aggtcgttta ttgtttagcg tcggattttt tagttttatt atatttggtt tttttttcga   14340
ttaggtttcg attttgggga ttttcgagga ggggtttgga tacgtcgggg gggggggttgt   14400
agtagttttt atcggacggt ttttgcgttt gaatcgcggt gtcgggatcg tcggtttttg   14460
gcgttttttg gtgggtatag tgggttttgc gtgaggtcgg gtttggtggg cggttttgag   14520
gttggagttt agtgatagtt atagtgggtt ttgaattggg gttggggtag aggtagcgag   14580
agtcggggtt tttgtgtttt acggtttggt agggattcgt ttttagtagt tttgttgtgc   14640
ggttttggtt ggttagttag ttttttttgag tttggtgttt tcggtaatac ggggttataa   14700
tgacgttttt tgtttatttt gttaggtatt tttaggaaag ttttgtcgtg ggttcgtttt   14760
taggtttgaa tttgttttttc ggggattttt ggattggtag gttttgtttt ggtgtttttg   14820
atagttttgt ttaggttaat ttttttttgg ggatgggtat tgttagtttt acgggttttt   14880
agtgataggg ataggagata gaaattttag gtagaaaaag atggggtttt tgaggaattt   14940
tatttttaag tttggaagta tagttttgag tgagaatacg tattttagtt tttttgtttt   15000
aatgttgttt tttttaaaat tattattgtt tcgttttatt tttattttgt atttataaaa   15060
atttttgatt ttattggtag agaatagagt aggtaaggag aggagaagta gttggttatc   15120
ggagatggta gtttgatatt agagaggagt agcgtgattt tagagggata gttggatagt   15180
aggattttgg agaagagttt ggtagagacg gtaggatttt aggggattat tattttttttt   15240
ttttattttt tttttagttt ttttttttat tgagagtttt ttttattatt tttttttttt   15300
atttttttttt tagttttcgt tttttgtgag agtttcgttt attatttttt tttttttattt   15360
tttttttagt tttttttttt ttgatagttt tttttattat tttttttttt tattttttttt   15420
```

ttagtttttt ttttttttga gagttttttt tattattttt tttttttatt ttttttttag 15480
ttttttttttt ttttgagagt tttttttatt attttttttt tttattttttt tttttaggtt 15540
ttttttttttt tatcggttat aaaatttttt gtatttatta tttttttaatt tttttatgta 15600
atttgatatt ttttggatgt tggataagag tttgggatat agaaagttgt tatattgatt 15660
ttttgttttc gagaaaaggt agagggttta ttgagttgtt aaatatttta gatatttttg 15720
gatagtaaag ttaaaagtat tgtaatatat attttttgag gttttacggg ttataggtat 15780
tgttttagat attgttatag gattgtacgg agttttgttt ttgttgtgtt tagaagtgtt 15840
tattttagtt tttgtattcg tttatttgcg tgtttttgtt gtttcgaggg gttgagagtt 15900
ttgggttaag taagatatcg ttgttattag gtttataaag gggttaagga aaattttttg 15960
ttttattggg gtgtgcgtgg tatggacggg agaggagggg taatataggg ttgtttttat 16020
taggttattt ttttttttgt ttttggggat ttgttttagt gtcgtttttt aggtagatgg 16080
gtttggggtt agggattttt ttaaagtagg taatttttag ttcggttatt ttttgatttt 16140
gggttttatg gtcgggtttt taggtagggt ttggttattt aaaagtagtt ttagtatttt 16200
aattaatttt tattttataa ggttatagtt tttatagtag ttttagtacg tttcggttcg 16260
ttgtttttttg agtattttttg ataaattttc gttttgtaag attatagttt ttacggtagt 16320
tttgtttatt tttttcggt ttggagttta gggtttttgt aataggttgg ggtttatgga 16380
gagtggtttg tatttggtgt gttggggtgt ggttgttaat atttggggcg tagttttatt 16440
aaagtagggt gttcgtagtg gtttttatat tattacgggg cgggtttttt tgaggttttt 16500
ttgttttatt tttttttgtt tttagaagcg ttgagttcga gcgttttggg gttttgtttt 16560
gaatatattg tgtgtttttt ttttttttagt tggtttttag agatttttgg tagggtttga 16620
tttttaggat tacggtcgta agtaattttt tggttttttt ttttttttta gttttgtaaa 16680
ttagtttttt gattaaggag atttttagaa ttgtttttttg attttgtaat ttgagaggat 16740
tatattatcg tgtagttttt atttcgggtt tcggtttttt gagttttagg atttcgtatt 16800
agggtttgaa gaattaggcg gagtacggtc ggcgttggtt attgggggt atacgtgtag 16860
tttttaaggt ttttagtagc gtcggtgttc ggcgtttagt tttgttaggt ttaggttcga 16920
ggatagggt tgggggtttt atgtggggtt ttttttgttt tgtggcgagg gtttttttat 16980
gttaagattg aaatttttgg tttttaggtt aagatttagg gtttagaatt agtttagttg 17040
attttttttgg tttggttgta gggtttaggg aagttggatt ttgggggtgg gattgggatt 17100
ttttcgtttt ttttagtttt atttattttt tgttggtttt gttgtcggcg agtggttagt 17160
tcggtcgttt tatattttttt agtagttggt gggcgatttt ttcggtagta ggatttgttt 17220
ggttgggtcg gttatatttg ttgtagtagt tggtttaaaa tttttaaatt ttggtaagtt 17280
gttcgtttgg tttcgtatcg gttaggttag cgtatttttg gttcgtaggg tttgtttgtt 17340
ttttgttgaa tcggttgttg gggatcgtgg ttaggtgggt aggcggatag tattttagtt 17400
tggagatatt agggttgttt atacgttagg ttgtattgtt ttagtttttt gtgtgtgtgg 17460
gtgggggtgg tatttcgttt gggttttgtt ggagtaattt agatgagagt ttgtaggttt 17520
tttaggagaa agaaaaatag agatatgggt gttggggttt gatttttatt ttatttttttc 17580
gggtagggga gaaagtaggg tgttattttt ttgagggtag gcggtcgttg gataggttta 17640
gttttttttgt ttcggttagt ttgttttagg ttcgttaatt tttttagtgt ttattttcgg 17700
tttattttta ttgggatttt cgggttagtg gtagtttggg taggttagga gggtgtgtag 17760
gttaggaggg tgtgtaggtt aggagggtgt gtaggttagg agggtgggta gtattaggag 17820
ggtgggtagg attaggagga gcggggttta gttgttatag gggtttttta agtcgttatt 17880
taattttggt ttgagtttgt tatttgatat ttggttattt ggttgttttt aggttttggg 17940
ggaggtcggg ttttcggttg tcggatgttg ggagttagag ttttaggagt agagaggcgg 18000
tggtcgtcgt ttatttaggg tttaggtggg ggtgttgtta aggtaggtgg cgaaatcgta 18060
gttagtgttt tttttcgtgg gtgttcgttt attttcgttt ttcggtattt gttgagtatt 18120
tattgtgtgt tgtcggtttt ttcgtgttga gatagattta gtaaggggtt tgcggaaatt 18180
gttttgtgtt tagtacgtgg ttttttttttg gtttttttatt gtttgcgtgc gggggttttt 18240
taagtatgta tttttttggg attttgtttt gtttagtttg tatttgttgg gtgtaggtgg 18300
ggtttttttgt tttgtttggg gttagtggga ggggttgggg tggttgtggt tattaagtag 18360
gtattttgag gggagagttt gggtagtatt agatggtgat attttggggt tagcgttttg 18420
tatttagtaa gatagtagtt tttgcggagt gttgagtgga tagaggtggg ggttggttcg 18480
ttttgtgttt aaggttgggt gggagagtat tgtagtttcg gtttatttta gttgtttatt 18540
ttagtttggt gtttattttt tgaaagtttt ttttgttggt atttgtagtc gaggtaattg 18600
atttttggtg ggggtcgggt tggtttgggt tttggttttt tttggtttttc gagttttcgg 18660
tttagttgtt gttgaggagg ttgcgtttgt tttcggggtt aatagttgta ggttcggaag 18720
cgtttttttt ttatattttt tagagtaggt gtgtttttta tagtttaatt tagtttgtta 18780
aggatgggtt ttagggatac gggggttttt tgtttttttt tgtttaattt ttcggggttt 18840
gggttgggat tgttgttgga gtttttagtt ttgtgggtag tagacgttaa gtttttggtg 18900
gttagtttgt ggttaagtgg gttgagtatt tattgtagtt tcgattaaga gtagttgtgt 18960
tttttagtta cgtttattag atttaaagtg tacggtcggg tatatatttt tttatttgtt 19020
ttattgttta ggttcggcgg gggtttttttc gatgttattt ttttttttttt aggtttcgat 19080

```
atttatattg tttttattga tttgagagtt tgtgttatag tgtttttagc gtggtaggag   19140
agggatgttt gagttatggt agttattggt gatttcgtgt tagttgggtg atagttgaat   19200
tttaggttag tttttggtag tttatttgta gtgtttttgg cgggtattta gttttgtttg   19260
gtttaatttt atgttatgtt atgaggttga ttaatttttt agtagtcggt ggttatagcg   19320
gagggtgttt gagattttaa attggggatt tattttgtgg atatttttttt tttttatta   19380
tagttgtggg tattatgttt ttttttttgt tttttttcgt ttatgggttt tgatattttt   19440
tttttgtttt gtttttttttg ttgtttgttg gatttattat tgtttttagt ttttgagttt   19500
ttttttggtt ttggaatttt ttttagtttg ttattagggt ttagtatatt tgttttaggg   19560
atggggtgta gtttttattt tgattagatt tcggtatagg agattttagg ttagggattt   19620
ttttagggaa gtaattggag ttttcgtttt atggtggagg ggttaggttt cgggggattt   19680
gttaggggta agtgttagtt agattgtgtg ttcgggatag ttgttttgtt atagttggtt   19740
tttgttttgg gttgaacggt gtttttttaga tttatattta tttagaattt tagcgtgtga   19800
ttttatacgg agatggtatt tttgtaaatt tatttattta tttattgaga tggagtttta   19860
ttttgttgtt ttggttggag tgtaatggcg ttattttagt ttattgtaat tttcgttttt   19920

taggtttaat tgattttttt gttttagttt tttgagtagt tgggattata ggcgtttgtt   19980
attacgttcg gttatttttt gtatttttag tagagatagg gtttcgttat gttggtcggg   20040
ttggttttga atttttgatt ttaggtgatt tgtttgtttt agttttttaa agtgttggga   20100
ttataggtat gagttatcga gtttggttat ttttgtaaat ttaattagtt aagatgaggt   20160
tatgtaggat tacggtgaaa gggtttttgt gagaaaagga aaagaggaga atgggtaggg   20220
tttttaggga ggtgtcgggt tcggggacgt tagggtttag ggggttaata ggttttggtt   20280
atgtttttat ggtagagtta gagaagatga cgaggttatg gtttttgtat ttttaatatt   20340
taaattttcg tttttacgtt tagatttcgg ggggtcgatt tttggtgtag agaggtgttt   20400
ttttaggaaa gcgtagaggg ggtcggcgtg gtttttaggt gtggggaggg cggtatattc   20460
gagacgtgga tgtaggattt agagcgggag ttttggggaa gtaggaagat ttttgttttt   20520
tttggggtag ggtcgtttat cggttaggtg gtttgggtat agaagtgggt gggtttggtg   20580
ggttttgatg taaagggttg gagggagtgt agggttcgat tagcgatgtt tgttttgggt   20640
atacggagga gtggggtttt atgtgttatg gttagttgag agggagaagg ttttgtcggg   20700
aaatttttaa aggtttttcg tagttagttc gagttgaggt tgtatagggg ttttagagtt   20760
agtattaggt tagttttaga cgtcgggatt agaaggggtt gtagggtttg gtttttaaga   20820
tttgtgtttt ttattgacgg tttggtagtt gggaaagaga agggagaaat ttttttttta   20880
atttttttag tttggttttt tttcgatttt agggatagag tttcgggtta gacgtgaatt   20940
agtggtttta ttaaattttt tatatttgag tttgcgattt ttttcgttta tttcgtatta   21000
gatttattag ttatttttgt tttagcgacg gatattttgt cgatatcggg tttcggggtt   21060
tttgcgaagt gttaagtggt tttgcgtagg tgatttattt gggtttattt tttttagggt   21120
ttttaagtat ttgatgattt tatttggatg gagtattttg taggtcgttt gtattttttta   21180
ttttaggaat tttttatttt tgagagcgac gggagaggtg gatgtttatt ttatcgtttt   21240
tttttgtgta ggagtttaat aatttagtag tcgggtcgag gcggcgacgc gtagggtcgt   21300
ttgtgttttc ggaatttttt tttttttttag gttttatgtg tacgcgtcgg ttggttgcgg   21360
atcgtgtttt ttttcgaaag aagtggtttt gttattaagt ttagtgtttc gtttagatcg   21420
ttttattagt tttcgttgtt gggtttagtt gttcggggat agcggttgtt tttgtcgggt   21480
cggtttcgaa acgtttgttt gaggtttagt ttaggtgttt aggttagtgt tcggggtagg   21540
gtatttttag ataaagtttg agtttacgtc ggttttatag gtattttatt tgttttaggg   21600
gtcgattttc ggtcggttgt tatagttttt tggttagcga ggtttatttt ggttagttgg   21660
tattttaagg ttttgaagaa gagcgagtat ttgttaagta tcgattgttt gttgttcgtg   21720
gattcgtttt ttttttaaac gggacgtttt tggttttttt tttgtagagg gcgaggtgtt   21780
gagagttggt gtttatttat taagttttgg tgtattttta ggttttagac ggcgttgaag   21840
gtattgttcg gaggtatcgt tttgtttttt gtattatttt gggggcgaag gtgtgcgtgt   21900
tttattttttt aggtgaggaa aatgaggttt ggggagttga gcgtttgttc gtaggtttag   21960
agatggtagt tcggtttatg gtagtttggt cgttatcggg aattatggtt ttgttttgtt   22020
ttgttttgtt ttgtattttt tttgagagtt tcgggttttt ttcggcgttg ggtaggtttg   22080
ttattttta gtagtagttt tagtagaaga gtagagtttt tatcgggttt ttttagggtt   22140
gtttttaggg gtaggatttg agtttttagg ggttttcggg gggttatttt tttagttgtg   22200
tttttttttt gtaggatggg gataggggtt tagtatagta tttagtggga ggttgtttcg   22260
atagtttagg gtacggtagg gtttttatac gtatgggttg tagggtggtt gtttttagag   22320
ttgttgggtt aggtagagat gaaggggtag tgaggttagt ttttgagggt gggggcggtt   22380
attgtttatg tttttattgg tttttttttt tgttatttga ggtggtcgga gatttgggga   22440
taagtgttcg agtttttcgt tttattgatt atagtgtttt ttagcggtt               22489
```

<210> 133
<211> 4419

<212> DNA
<213> Artificial Sequence

<220>
<223> chemically treated genomic DNA (Homo sapiens)

<400> 133

```
tgtgtcgtgc gggtagggag tagtttttt cggtcgtcga ggcgttttta atttggttgg      60
agagataagt gtattataag aaatagaggt agttaagcgt ttgggttatt attaagtaag     120
gttgaaaagg ggagggtagg tagatcggga ttcgtgggcg ttaattaatg ttatggtggc     180
ggagagtaaa ggggacgaat atagtttggg gttattttcg gagtttttta gcgttcgttt     240
gtggtcgtgt ttggttggtc gcggatttcg gcgggcgtcg taaagcggcg ggattgttag     300
cgtagagttt cggttttttg ttttgttttt gggttcgagt atcggagttt ttggtgtttg     360
cggggagaag tttcggattg agaaatacgg gagggtttcg ttagtggttg taggtgcggt     420
agttattttg gggatttagt gagaatgggg tcgtttggtt ttgcgcgaat tttttacgtg     480
ggtgtagttt ttgagtcgtc gagggaggcg gtggtaatgt cgtttagtgt tagtagaggg     540
tagtttcgag gtcgtgagtt cgaacggcga tttcgttaaa tcgcgggttt ttttttagtt     600
ttcggatttt gcggggtaga ggcggttttg gagtttagag attagcgatt ttagtttgta     660
ggagttcggc gtagaggttt aagggtattt tcgggatgtg gttaagttat agtttttagg     720
tagttttatt ttgcgacggt aagggtttag agggtggagg gggattagat gttttaggag     780
gggttagaaa gttaacgtat atagggaatt tgtttttaag taatagattt taagtatgtg     840
gaaatttttt aaatgatgtt ggtgagagtt atgatagttt tcgtattttt ggcgagggaa     900
gtcggaggtt agtagcggga tggtttttgg gcgcgcggga gatagaggga ttagcgattt     960
ttgttggggg tagagggatt gtggattaag gatttagata tatttaattg gggattttag    1020
tttcgattgt ggttatttaa ttggcgttga atttgagtaa tttattatat cgttttagtt    1080
ttcgattaat tatttttgta aaatgggtat tgtgggtttc gagtttcgta agggcgttag    1140
ggattcgaga tagtaggaat ttttttattg ttttagtaat tttgggtttt tgggtttgta    1200
ggtgggttga aaggattttt ttatcgtacg tttgtttggc gtggttgttt tagagatcga    1260
ggttcgttag gttttgtaat ttagttgcgc gtggttagtt atggttttgg aatcgtcggg    1320
atcgttttag cggtatttcg gttagtttc gatttttcgt tttgggtatt agggttattt    1380
agttttagaa gatagtgttt attagtatag gaattgagat tttatattcg gcgtagtggg    1440
tttttttagga agtttggcga agagttaagg ttcgtcggat ttgaggtcgt ttggggcgtt    1500
aaatagattt atttatgagt atattcggtt aatatattta agtttaatag tgggcgggat    1560
ttttggttgg tttcgatttt ttttatagtg tgtgtaaacg ggtatttatt agattttttt    1620
tgtttgtttt tttatttgta aattttgaaa ggagagtatt tttcggggtg attttgaaaa    1680
ttatgttaga attgaaaagg tttgtgtaaa tgcgaggtgt aatgatgatt tattagaagt    1740
agttagtttt gtacgtgttt gtaggataga tgttagaacg ttttataaat gtgtgtatat    1800
atatattacg tatgtgtgaa ggtgtatatt tgtttttaga tatttgataa atgtatatac    1860
gtatgcgtgt atttttggtt gagagtaaag gggttaatat agggtttata taggatatta    1920
ttttgtagtt ttgtgcgtgt atagtatata cgcgagtacg attgtttgtg tttggagata    1980
tcgtatggta tcggaggagt tgtttagtcg tcggtgttat ttgggaagta gggtttcggg    2040
tgttgttttg gagcgcggga aagttcggat tcgggtttgt tggttagcgt ttcggcgtcg    2100
tcgggttttt ttattttta ttcgtcggtc ggtgagcggt tttagtttta agattttcga    2160
ttttgtcggt gaggggaggg agcgagaagg agtgcgtttc gggtttaaga ggttgtaggg    2220
ttttattttt taggtttgtc gttttttttt tattttttta ggttatattt ttatttaaaa    2280
taaagaaggg tgtttgattt atttaggtta gggaagtcgg ttttcgggag tttttttgttt    2340
ttataagtta tggtgaaggg aggcgaagtt agggtttgtt acggtttggg agaaaatgcg    2400
gttgtagggt ttttttgggt tgtagcgttg gttcggggtt ttaagattgt taaggtgtgt    2460
gtgggaggcg cgtagtgtgg ttatcgaaga gtttttttatt atcgtatcgg taggtcgtcg    2520
ggttcgtttt tttttatgt ttttaagggt tttgagttgc gtagtattcg tattatttag    2580
aagtgtttgt ggagaaaacg attttaggtg taagtttaag gttgttgttt gtaaaggtaa    2640
tgtcgtggag attgggtttt atagcgattt gggtttttaa gttatcgaaa gttatagggt    2700
agggttataa atatttttc gttttttttg tagaatcgtg cggttgatag gagagcgttg    2760
cgtagaaaat tcgaggtcgg gcgttggagg agttttcgcg gttcggagaa ggtatagagg    2820
cgtttttgag aggcgtagtt ggaataggcg atgtacgggt tcggatttgg tcggttaatc    2880
gagtttagcg gtcgcgaaaa ttagagtcgt tatagaggtt gagtacgatt ttattttgg     2940
ggatcgggtt cggtggggag ttattgtttt taacgttttt agagatttta agtaggataa    3000
tataatgttg gtaggagtaa ggtgtaagga atttagcggt agaagttttt cgggggggtg    3060
gggaaaggta gattcgtgtt tcgtttgagt ttgggggtgg ggatagtttt tggttttgta    3120
gtttttgttt cggggattag ttgggtttat ttaatttttt tatatggggt tgaaaggggt    3180
```

```
taatgggacg gtttcgtcgt tttttttcg ggattttata gggttttgat tcggttttta    3240
tttttgtacg gttagtagtc gcgggggttt taggaaggag gataaaggtt cggtgttatc    3300
gcgggcgggg gttcggtttt ttggtttttt gtttatattt atagttttta gcggttgttg    3360
ggggaagatt tttaaaaata tgtgtcgaat tttttttttt tttttttag aaataaataa     3420
ataaaaaagg taaaaggcga attttttttt atttttcgtt tatagagatt aaagtttttt    3480
gggattttgt tttttttttt tttttgattg tttagaaata tttgtttttt tttgtatata    3540
gaggaaaatg cggggaaagg ttttttaaaa ttcggtttta tattattatt attattaagg    3600
ataatcgggt aggttgaggt tttaacgtgg atgattcgag ttggtttcgc gtcggggttt    3660
tgtagttatt gttttgtgcg tttagtattt ttgggggcga ttagggtttt tgcgttttcg    3720
ttcgtcgttc ggtagtcgag agtattttgt gtttagattg gtcgatttat tttttttcga    3780
attttgttta gagttggtaa gggggattta gttcgcgttt taagatttgg gtttgtagcg    3840
tcgttaatag gttcggggat acgaggcgtt ttaggtcggg gtttttcgg ttgttggttt     3900
ttttcgtttt ttattcgttg gcggcgtttc ggtcgttcgt aattgattta attcgttttt    3960
tgcgtttgtt ttttaggttt ttcgtttttt tataaaggtt taggggagtt tcgtttatag    4020
gttgattttg taatttttgg ttcggtggtt atttttttgtt tttttgaaaa agaaaaggaa  4080
aaaaaaaaaa aaaaagaaaa aattaattag tcgagggagg cgcgtgagga ttggaggcgt    4140
tagtcggata gtttatgagt aggtttttgg gtcggtgtcg tttcgcgggt tagtacggtt    4200
ttttttagag aaaatttttt aaacgtgtga agatcgtttt gggggaagcg agagggaggt    4260
tggaggagtt tcgggcgggg ttttagcgtt tattagttgt gtttttaggg tttgggtgtt    4320
cgttgtaacg gtaatcgcgt gagttttatt tttacggtta aggggttagg gtagggtgga    4380
tgtaatcgcg tgcgtttggt ttcggaaggt gttcgtagg                          4419
```

<210> 134
<211> 4419
<212> DNA
<213> Artificial Sequence

<220>
<223> chemically treated genomic DNA (Homo sapiens)

<400> 134

```
tttgcgagta tttttcgggg ttaggcgtac gcgattgtat ttattttgtt ttagtttttt      60
ggtcgtggga gtgaggttta cgcggttgtc gttgtagcga atatttaagt tttgaaggta     120
tagttggtgg gcgttgagat ttcgttcggg gttttttttaa ttttttttttc gttttttttta 180
aagcggtttt tatacgttta ggaggttttt tttgagaaag gtcgtgttga ttcgcgaagc     240
ggtatcgatt tagggattta tttataggtt gttcggttgg cgtttttagt ttttacgcgt     300
ttttttcgat tggttgattt tttttttttt tttttttttt tttttttttt ttttagaaaa     360
gtagaaggta gttatcgggt tagaggttgt agggttagtt tgtgggcgag gttttttttag   420
gtttttgtgg agaaacggga aatttgaggg gtaaacgtag gaagcgggtt gggttaattg     480
cgggcgatcg aggcgtcgtt agcgggtggg gagcgagagg ggttagtagt cgggaagatt     540
tcggtttgga gcgtttcgtg ttttcgggtt tgttggcggc gttgtaagtt taggttttgg     600
ggcgcgagtt aagtttttttt tgttagtttt aaataaaatt cgggggagaa tgagtcggtt   660
agtttgggta tagggtgttt tcgattgtcg ggcggcgggc ggaagcgtag gggttttgat     720
cgtttttaga ggtgttgagc gtatagggta gtggttgtag agtttcggcg cgaggttaat     780
tcggattatt tacgttggga ttttagtttg ttcggttgtt tttagtaata ataatagtat     840
gaaatcgagt tttaaaaaat tttttttcgt attttttttt atgtataagg gagaataagt     900
atttttaggt agttaaagaa aaaaaaaggg taggatttta ggaaatttta atttttatgg     960
acgaggagtg aaggggaatt cgtttttttgt tttttttgtt tgtttgtttt tagaagagaa  1020
aaaaaggaaa ttcgatatat atttttaaaa attttttttt aataatcgtt aaaggttgtg    1080
agtgtgagta gagagttagg aaatcgagtt ttcgttcgcg gtgatatcgg gtttttgttt    1140
ttttttttga agttttcgcg attgttgatc gtgtagagat aaaggtcggg ttagggtttt    1200
gtggaatttc gagggagggg cggcggggtc gttttattgg ttttttttaa ttttatgtag    1260
gggggttagg tgggtttagt tgattttcgg aataggggtt ataaggttag gggttgtttt    1320
tatttttagg tttagacgag gtacggattt gttttttttt attttttcga aaggtttttg    1380
tcgttaaatt ttttgtattt tgtttttatt agtattatat tgttttattt aaagtttttg    1440
gaggcgttag ggatagtggt tttttatcgg attcgatttt tagaaatgga atcgtgttta    1500
atttttgtgg cgattttggt tttcgcgatc gttgggttcg gttggtcggt tagattcgaa    1560
ttcgtgtatc gtttgtttta gttgcgtttt ttagggggcgt ttttgtgttt ttttcgggtc  1620
gcggagattt ttttagcgtt cggtttcgaa ttttttgcgt aacgtttttt tgttagtcgt    1680
acggttttgt agagaaggcg ggagagtgtt tgtgattttg ttttgtagtt ttcggtgatt    1740
```

taaaaattta ggtcgttgtg gaatttagtt tttacggtat tgtttttgta ggtagtagtt 1800
ttgggtttat atttgaggtc gtttttttta tagatatttt tgggtgatgc gagtgttgcg 1860
tagtttagaa tttttggaag tatgagagga ggacgagttc ggcggtttgt cggtgcggtg 1920
atggagggtt tttcggtagt tatattgcgc gtttttttata tatattttaa tagttttggg 1980
gtttcgggtt agcgttgtag tttaaagaga ttttgtagtc gtattttttt ttaggtcgtg 2040
gtaaattttg gtttcgtttt tttttattat ggtttgtgag ggtaggaggt tttcgggggt 2100
cgattttttt gatttgaatg agttaggtat ttttttttat tttaagtggg ggtgtgattt 2160
gaggagataa aggaaggacg atagatttgg ggagtggggt tttgtagttt tttgggttcg 2220
gagcgtattt tttttcgttt ttttttttta tcggtaggat cgggggtttt gaaattggag 2280
tcgtttatcg gtcggcggat gagagataaa ggagttcggc ggcgtcgaag cgttgattag 2340
tagattcgga ttcggatttt ttcgcgtttt agagtagtat tcgaaatttt atttttagg 2400
tggtatcgac ggttgagtag ttttttcggt gttatacggt gtttttaggt atagatagtc 2460
gtgttcgcgt gtgtattgtg tacgtataag attgtaggat gatgttttgt atgagttttg 2520
tattagtttt tttgttttta gttagaggta tacgtatacg tgtgtgtatt tgttaagtgt 2580
ttagggatag gtgtgtattt ttatatatac gtgatgtgta tgtgtatata tttgtagaac 2640
gttttagtat ttattttata ggtacgtata gggttggttg tttttaataa gttattatta 2700
tatttcgtat ttatataggt ttttttagtt ttggtatgat ttttagaatt atttcgggaa 2760
atattttttt tttaaagttt atagatgagg aaataagtaa aaagaattta atgggtattc 2820
gtttgtatat attgtgagag agatcgaggt tagttaaaga tttcgtttat tgttgaattt 2880
gggtgtgttg gtcgaatgtg tttatagatg ggtttgttta gcgttttagg cgattttagg 2940
ttcggcgggt tttggttttt cgttaggttt tttgggagat ttattgcgtc gggtgtgggg 3000
ttttaatttt tgtgttgata agtattattt tttggagttg ggtgatttta gtgtttaggg 3060
cgggaagtcg ggagttggtc ggagtgtcgt tgaggcgatt tcggcggttt taaggttatg 3120
gttgattacg cgtaattggg ttgtaaagtt tggcgggttt cggtttttaa agtagttacg 3180
ttaggtaaac gtgcgatgaa aggatttttt tagtttattt ataaatttag gaatttaggg 3240
ttgttggagt agtgaggaaa tttttgttgt ttcgaatttt tagcgttttt acgagattcg 3300
gagtttataa tgtttatttt atagaagtga ttagtcggag gttagagcgg tatagtgagt 3360
tgtttaagtt taacgttagt tgagtggtta tagtcgggat tggaattttt aattaagtgt 3420
gtttggattt ttggtttata gttttttgt ttttagtaag gatcgttggt ttttttgttt 3480
ttcgcgcgtt taggagttat ttcgttatta gttttcgatt tttttcgtta agaatgcgaa 3540
aattattata gtttttatta gtattattta aaaggttttt atatgtttgg aatttgttgt 3600
ttgaaaataa attttttgtg tgcgttggtt ttttaatttt ttttaaggta tttggttttt 3660
ttttattttt taagtttttg tcgtcgtaga gtgaggttgt ttaagggttg tagtttgatt 3720
atatttcggg ggtgtttttg ggtttttgcg tcgggttttt gtagattgag gtcgttggtt 3780
tttgggtttt aaggtcgttt ttatttcgta gaattcggga gttggaaggg agttcgcggt 3840
ttggcgaggt cgtcgttcgg gtttacggtt tcgaggttgt tttttgttgg tattgggcga 3900
tattattatc gtttttttcg gcggtttagg agttgtattt acgtgagggg ttcgcgtaga 3960
gttaggcgat tttatttttta ttgggttttt agagtggttg tcgtatttgt agttattgac 4020
gagatttttt cgtatttttt aattcgagat ttttttcgt agatattaga ggtttcggtg 4080
ttcggattta gggataaggt agagagtcgg agttttgcgt tggtaatttc gtcgttttgc 4140
ggcgttcgtc gggattcgcg gttaattagg tacggttata ggcggacgtt gggggatttc 4200
ggggatggtt ttaggttgtg ttcgtttttt ttatttttcg ttattatagt attgattggc 4260
gtttacgggt ttcgatttat ttgttttttt ttttttaatt ttatttgatg gtggtttagg 4320
cgtttggttg tttttgtttt ttgtagtgta tttgtttttt taattaaatt aagagcgttt 4380
cgacgatcgg aaggggttgt tttttattcg tacggtata 4419

<210> 135
<211> 1918
<212> DNA
<213> Artificial Sequence

<220>
<223> chemically treated genomic DNA (Homo sapiens)

<400> 135

tatcggatat gattatcgta tataatatag aagatattaa gggttaagta atgatgtata 60
aacggggttt attgtttaaa tttatagaaa gtaaaacgtg gaaaattagt aattttaatt 120
gatagtgtat atttaaagaa tttatatcgg tcgggcgcgg tggtttacgt ttgtaatttt 180
agtattttgg gaggtcgagg cgggaggatt atttgaggtt aagagatcga gattagtttg 240
attaatatgg tgaaatttcg tttttattga aaatataaaa aaattagtta ggcgtggtgg 300

```
tatatgtttg tagttttagt tattcgggag ggtgaggtag gagaattgtt tgaatttagg     360
agatagaggt tgtagtgagt cgagatcgcg ttattgtatt cgtttgggag atagagcgag     420
atttcgtttt aaaataaaaa ataaataaaa agaatttatt taatagaatt aagtattaat     480
ataataaata cgaagaattt tagatttttg gtttttaaaa aatatataaa gatgatattt     540
ttttaaaata tttttataaa atatattgag attgtgatgt tttatattga ttgtatgaaa     600
ataatgaaaa agaattagta ttgttttatt ataaaagttt tattaatgta aatttataaa     660
ttttttttta aatattttga gttaatttta attttatgat agaaatttat tatttttagt     720
aaaaatagtt ggtatttggg aaattaaagg tttaaaaatt aagaatagta attaaagaaa     780
tttgataaaa tagtttttt aaaattttta tttatattat aaggggaaat tttgattacg     840
tttttttttt tttattaatc gtagaattta atattaagga ttatataatt ttatattttt     900
tttcgagaaa aagtaaaggt tttgtgttgt agtaataacg taagatatgg agggaagttt     960
tatttaagat ttttttgttt gttttttttt taaagttatt ttagaatatt agggagggtt    1020
gagaggtaag gtatgaaggg cgtaatattt aatatgagta acgcgtgtga tgtatttggt    1080
taaaatgtat atagaggatt tgtttttgtt tttagataga agtttttcgt tttgtagtta    1140
tgagggttaa ttgttgaggt tttatagttt tttttttttt tatattcgga tcgttacgtt    1200
ttttatttat tatttcgatg tagaggtaga tttaggattt ttgtatttgt taaggatttt    1260
tcggtaagtt tacggggcgg gagtggttat aagacggagt tcgtttggtt ttggtttttt    1320
cggtttatat aagtttgttt tttttttaat ttttaatttt tatagttttt tattttttta    1380
ttttcgattt attttgcgtt atcgacgttt ttggttttcg tttgtagtaa gtttattttt    1440
attattattt ttcgtataaa agtttgtatt tattaggtta aagaggggaa ttaacgtttg    1500
taggaatcgt tttatcgaat cgtttggtcg cgttttttgt tagattttat ttgtcgttgc    1560
ggatcgtata taattatttt cggtatgttt cgcgtacgta ttattttttt tatttcgttt    1620
ttttttcgtt taaatacgtg atttttttc gttttcgttt acgtttattt tcgttttttt    1680
attttttttt aggaaggagg agggagttgg gggtgttaaa agcgtagcga tttttttttt    1740
tttttcgttt tcgtttttgt atttttcgtt ataatgtttt tcgggtcgtt agcgtttcga    1800
cgttttttgg gaaaatagtt tattttttttt tttttttttt tttttgtttt taattaatta    1860
gttattcgtt agagagggat atgcgtagtg agtgttttttc gtttttttta ttcgaatt     1918
```

<210> 136
<211> 1918
<212> DNA
<213> Artificial Sequence

<220>
<223> chemically treated genomic DNA (Homo sapiens)

<400> 136

```
ggttcgggta gaagagacgg gaggtattta ttgcgtatgt tttttttttga cgggtggttg      60
gttggttgaa ggtaggagga gggggagggg aggaaatgag ttattttttt agaaggcgtc     120
gaggcgttag cgattcggaa gatattgtag cgggaggtat aggagcgggg gcggggagga     180
ggaggaagtc gttacgtttt taatattttt agttttttttt tttttttttaa gggaaagtgg     240
aggaacggaa gtgggcgtgg acggagacga aaggaggtta cgtgtttggg cgggagaggg     300

gcggggtggg agaggtagtg cgtgcgcggg gtatgtcggg agtggttgtg tacggttcgt     360
agcggtaggt gaagtttagt agaggacgcg gttaggcgat tcggtgaagc gatttttgta     420
ggcgttggtt tttttttttg atttggtaaa tgtaggtttt tatgcgagag gtaatggtgg     480
gggtaaattt gttgtaaacg aaggttaggg gcgtcggtgg cgtaaggtga atcgaaagtg     540
ggaggatgga aggttgtgga gattgggaat tgggaagggg gtaggtttgt ataggtcggg     600
aaggttagga ttaggcgagt ttcgttttgt ggttatttttc gtttcgtgag tttgtcgagg     660
aatttttgat aagtgtaggg attttgagtt tatttttgta tcggggtagt aggtgaggag     720
cgtgacggtt cgagtgtaag agagaaggga attgtgaagt tttagtaatt gatttttatg     780
attgtaggac ggaggatttt tatttaggga tagagataag ttttttgtat gtattttgat     840
tagatgtatt atacgcgttg tttatattgg atattgcgtt ttttatgttt tattttttaa     900
ttttttttgg tattttggag tggttttggg gaaggagtag gtagggaagt tttgagtgga     960
gttttttttt atgttttgcg ttgttgttat aatataaagt ttttgttttt tttcggagag    1020
ggatgtggga ttgtgtagtt tttaatgttg agttttacga ttgatgaagg agaagggacg    1080
tgattaaagt tttttttttat agtgtagatg agagttttaa aaggattgtt ttgttaagtt    1140
tttttggtta ttattttttag tttttgagtt tttggttttt taaatgttag ttgtttttgt    1200
tgaaaataat gaatttttat tataaaatta gaattaattt aaaatattta agaaaggatt    1260
tataaattta tattagtaaa gtttttatag tgaaatagtg ttggtttttt tttattgttt    1320
```

ttatataatt aatataaagt attatagttt taatatgttt tgtaaagata ttttgaagga 1380
atattatttt tgtatgtttt ttaaaaatta agaatttaaa atttttcgta tttattatgt 1440
tggtatttaa ttttgttggg tgggtttttt ttgtttgttt tttgttttga gacggagttt 1500
cgttttgttt tttagacgag tgtagtggcg cgatttcggt ttattgtaat ttttgttttt 1560
tgggtttaag tagttttttt gttttatttt ttcgagtagt tgaaattata ggtatgtgtt 1620
attacgtttg gttaattttt ttgtgttttt agtagggacg gggttttatt atgttggtta 1680
ggttggtttc gattttttga ttttaaatga ttttttcgtt tcggtttttt aaagtgttgg 1740
gattataggc gtgagttatc gcgttcggtc ggtgtaggtt ttttaggtgt atattattag 1800
ttaaagttat taattttta cgttttgttt tttgtaagtt tgggtagtgg atttcgttta 1860
tgtattattg tttagttttt agtatttttt gtattgtgta cggtaattat gttcgatg 1918

<210> 137
<211> 13670
<212> DNA
<213> Artificial Sequence

<220>
<223> chemically treated genomic DNA (Homo sapiens)

<400> 137

agtgttggga ttatacgagt gagttattac gttcggtttg ggtttttgtt tgtttgtttt 60
taagatacgg ttttgttttg tttttaggtt ggagtgtagt ggtatgatta tagtttattg 120
tagttttgat tttataagtt taagcgattt ttttattttta gtttttgag gagttaggat 180
tataggtata cgtttttatg tttagtagtt aaattttttt tttttttttt aagagataag 240
gggttttgta atgtttttaa ggttggtttt aaaattttttg gtttgtcgtg attttttttt 300
ttcggttttt taaagtgttg ggattgaagg tatgaattat tacgtttgtt ttttagtagt 360
ttgtgacgtt aggaattata tttttatttt ttgacgtttt tttttttttt attgttgggg 420
tattattttt gtttggttta ttttttattt tttttttttt tttttttttt gagatggagt 480
ttagttttgt tatttaggtt gtagtgtagt ggtgtgattt tggtttatta taatttttat 540
ttttcgggtt taagcgattt ttttgtttta gtttttcgag tagttgggat tataggcgtt 600
tattattacg tttggttaat atttgtattt ttagtagaga cggggtttcg ttgtgttgga 660
taggtttgtt ttaaattttt gatttcgtgt ttttttcgtt tcggtttttt aaagtgttgg 720
gattataggt gtgagttatt gtgtttagat tttttttttg agatggagtt ttttttttttc 780
gtttaggttg gagtgtagtg gtatgatttt ggtttattgt aatttttgtt tttcgggttt 840
aagtgatttt tttgttttag tttttcgagt agttgggatt ataggtatgt gttattatgt 900
tcggttaatt ttttgtaatt ttggtagagg tggggtttta ttatgttagt taggacggtt 960
tcgatttttt gattttgtga tttgttcgtt tcggtttttt aaagtgttgg gattataggc 1020
gtgagttatc gtgtttggtt ttattttttta tttttgtggt tttttttttt tgtttgtttt 1080
tgaggtgttg gggggtttta gggtttagtt tttagttttg tttttttattt tgttttaggg 1140
agattttatt ttcgttttta gtttaagagt ttttttgttt tataatatat cgatgtttgt 1200
tgggttattt ttgtttttag tttataatat gtttttaagt ttttatttcg ggatttagcg 1260
tcgttagatt gaatatattt taaatttgtt tttgtatttt tttagcgtat ttgttttttt 1320
tttagtgttt ggatttgagt ttcgtttttt attgtttgga ttacgtttat ttttttttga 1380
ggtttttttcg tttttttggtt cggttttttt attatttttt aagtaatatt ttagatatac 1440
ggatttgttc gagatttttt tttgttggta tgtgaagttt tttatagttt tacgtttttt 1500
tagtttaaat ttttttttta gttttacgga aatgttaaat tatttcggcg tgggaggttt 1560
ttgtgggtcg gggcgtagtt ttgtaaggtt tagttttaag attgtttttt ttagggagtt 1620
tttttttttt tttttaggt aagtgttttt ttttagttgt ttattttatt agggtttgat 1680
tatattttgt tggtagtgtt agagttcggt tttgtttttt attatttttt ttagaattgg 1740
tggtagggat agatgaatgg atgaatgaat gaatgggaag tgagaatttt attattggat 1800
tattaatgta gttttcggaa tgagtgagtg agtgagtgag tgagtgagtg agtgatggaa 1860
agtggtttgg aggtagggag agggataggt cgattatgcg aggaatttta gtttaagttt 1920
tcgaggtttt cgttttcgtt ttaaaagatg tacgcgttaa gtttttgggt tagaaaaaaa 1980
aaaaaaaaac gggtcgggat tggtttaggt tgggggaggc gggtttacga cgagatacga 2040
tttttttattg gtttattcgt taggatttttg tttaatcgac gcgatcgttt tttttagaag 2100
attttcgttt tttttcgtc ggcggtcgcg gaggcggaag aaggcgtttt ttcgtcgttt 2160
tagtaatcgt tagcggattt tcgtcgttcg ggtaattgtt ttaggttttt cgttagtttt 2220
tgtttgggtt ttttcggtta ttcggataat cgttattcgt ttcggttaat tattaattcg 2280
tttttcgtta tttcggtaat tatcgtcgga tttatttatt tttggacgtc gttttggttt 2340
tgttcgtagg ttttttcgtt ttaaatttc gtcgcgggtt tcgacgttcg cgattttacg 2400

```
ttttaggggt tttttttttt ttttgttcgt tttttcgttt tttcggggcg gggggtattg    2460
tttttttttt ggtagggggg tttcgggttg gattcggttt tgggggtttt tgttcggtcg    2520
ggatagtggg atcgttttgt gttgtggtcg cgcgtagttt ttcgtaggtt tcggcgttcg    2580
tatttttgcg tgggattgtt taatcgttat agggagtttt ttaaaatgta agttatgttt    2640
tttttttgtt gaaaattttt ttttggtttt ttttgagaat cgaatttaag tttttcgtag    2700
tttttggcga tttggttgtg tggttttttt tttgaagttt tttcgtttat tttgttttag    2760
taaggttttg gtttgcgtgt tgtaatttta atgatttaag ttttttttcg tttgggattt    2820
ttttagttat tattttttat tttattaaaa agtgtatttt agacgggcgc ggtggtttat    2880
gtttggaatt ttagtatttt gggaggtcga ggtgggagga ttatttgagt cgaggagttt    2940
gagattattt tgggtagtat agtgagacgt ttgtttttat aaaaaatttt tgggaggtcg    3000
aggtaggcgg attgtttgag gttaggcgtt ggagattagt ttggttaata tggtgaaatt    3060
tcgtttttgt taaaattata aaaattagtt aggtgtggtg gcgtgtgttt gtaattttag    3120
ttattgggga ggttgaggta ggagaattat ttgaatttag gaggtggaag ttgtagtgag    3180
ttgagatggc gttattgtat tttcgtttgg gtaatagagt aagtttttat ttttaaaaaa    3240
aaaattaaaa attagtttgg tatggtggtg tgtttttata gttttagtta tttaggaagt    3300
tgaagtagga ggattgattg agtttaggag gtagaggttg ttgtgagttt acgttatatt    3360
attgtatttt agtttgggta atagagtgag attttgtttt aaaataataa taaataagtg    3420
tatttttag ttagggtttt atttgtgagt ttttagtgaa gtttttttttg agttttgtta    3480
gttttttttt ttttaaattt tttttggttt atattttgtt attaggattt agaatattgt    3540
ttggtattta tgttgagtaa aaagatgttg tgttaggcgt agtggtttac gtttgtagtt    3600
ttagtatttt gggaggtcga ggtgggcgga taatgaggtt aggaattcga gattagtttg    3660
gttaatatgg tgaaatttta tttttattaa aaatataaaa atgggttagg tgcggtggtt    3720
tacgtttgta gttttagtat tttgggaggt cgagacgggt gaattatgag gttaggagat    3780
cgagattatt ttggttaata cggtgaaatt ttgtttttat taaaaatata aaaaattagt    3840
cgggcgtggt ggtgggcgtt tgtagtttta gttattcggg aggttgaggt aggaaaatgg    3900
tgtgaattcg ggaggcggag tttgtagtga gtcgagatcg tattattgta ttttattttg    3960
ggatagagcg agatttcgtt ttaaaaaaaa aaaaaaaaaa aaaaaatata aaaatgagtt    4020
gggtgtggtg gcggatattt gtaattttag ttgtttagga ggtagagata ggagaatcgt    4080
ttgagtttgg gagggggttg tagtgagtcg agatcgcgta attgtatttt agtttgggcg    4140
atagagtaag attttatttt ataaataaat aaataaaaaa aggatattgt gttggggata    4200
agggaggata gagggggatg attagtaggt aggtttattt tagagatagg agaatgtatt    4260
ttttagggtt ttttagtttt tatggtatag gtgatgatgg taatattttt aaaaagttag    4320
gtataagtta gggagatata agttgatgtt acggttagtt tgagattggg tggcgtattt    4380
tttatatata atcgttattt tttatttatt tttattttag agggttttag agttagcgga    4440
aattgtggtt aatttggagt agaagttttt aggagttgga gaggtaggtt ttagatttta    4500
gatggttttt ttgggagttg gttttttttt gttcggaatt tagaatttta tttgttttaa    4560
tagagacgag agttgttttt ttagggattt gagataggtt agtttaggtt ggggtaagag    4620
aatttgattt gattaggttt ggggttatga cggggggtga gggaaggggt ttgtgggagt    4680
tttttttggga ttggttggag aggaatgagg aatttgtata tttatttgtt tgtttatgag    4740
ggtttgtttt tggggaggtt gttaggggtt aggtagggag gttttttgtt tttttttttt    4800
ttttttgaga tggagtttcg ttttgttgtt aggttagagt gtagtggcgc gattttagtt    4860
tatcgtaagt tttatttttc gggtttatgt tattttttttg ttttagtttt ttgagtagtt    4920
aggattatag gcgtttatta ttacgttcgg ttaatttttt gtatttttag tagagatagt    4980
attttattag gttagttagg atggtttcga ttttttgatt tcgtgattcg tttattttag    5040
ttttttaaag tgttgggatt ataggtttga gttattgtgg tcggttttga tttttatttt    5100
taagttagga ttatttttat tagcgttgtt atatataatt ttttttattg atgcggtaat    5160
tgagtatttg aaatatagtt ggtatggtta ggtatagtgg tttacgtttg taattttagt    5220
attgtgggag gttaaggtgg gtagattatt tgaggttagt agttcgagat tagttcggtt    5280
aatatggtga aattttattt ttattgaaaa tattaaaatt agttaggttt ggtgacgttt    5340
ttgtagtttt agttattcgg gaagttgagg tataagaatc gtttgaattc ggtaggtgga    5400
ggttgtagtg agtttagatt gcgtttattg tattttattt agtttgggcg atatagtaag    5460
attttgtttt aaggggaaaa aaatgaaata tagttggtgg gattgagtat tgtattttta    5520
ttgtatttta ttttaattaa tatatattta agttgaagta gttaaatgtg gttagtagta    5580
ttgtatttag agtagtattg agttgaagat ttaggtagga ggtcgaaggt agggatagga    5640
aggttagggt ttaaggtttg tgtagttatg gtagagaagc gattttggta aagcgtagag    5700
tgaggtttta ggaagggagg aggtgggata ttcggtttcg gtatttggtt ttattggaag    5760
agaggtatat ttagttttta gggttaaagt gtttgttata tatatatgta tttatgtttg    5820
tgtgtgagta taggggttgg tgtgtttcga ggtatttata ggtgcgtttg tgtgggattt    5880
aggagtttta tgtttatgag cgatttaaga gtttatttat gtatggatat tgtatggatt    5940
tttaggtata tatagataat gggtgtgtgt ttttttttga gtatttatgt tttttgggta    6000
ggttagtaag gattattagt ttagtaggtt ttgtaagaag gtttatatgt attggtgtat    6060
```

```
tttttggggg ttgttttgtg ggatttaatg ttttatgttt ggtaggatgt gggtttttaa    6120
gcggttcggt ataaagcggt tgtcgatggt ttttattagt tttagtttta agtaagtgtt    6180
tttttttttt tatagtagta atgtgggtgt ggttagtttt tggggtttta gggggaagtt    6240
tttgtggtta ggatagatag ataggtagat agataggtag aaggatggtt gtttttttatt    6300
tttttgtttt tgtaaatttt ttgagtttat gtttggtaat tggttttatt tgaagaggtt    6360
tcggtagtag ttgaggggtt tagtgaggtt attaggttgt gagaagttat aaaggaaggt    6420
ttcgagttcg ttggggggtta agtatgggat gtttgttttg cggtgggtga gggtggtttt    6480
tagaatttag gtatatagta ggattttggt tttagtgttt agtttgttta gtttcgattt    6540
gttttttttt ttttttaat ttgtattttt tgtatttgaa agttagatat agatagtagt    6600
ttttcgggta gttagggtag ttggaatagg aatatgtagt gggaacggaa gaattggttg    6660
atgtggtgta gggaatagtt tggggtggga gggttggggg agagatataa ggtttgtttt    6720
tggggtggtt ttatatattt gtatttttat atatatttag gttagggata taagttttag    6780
gaatgttatt gttgtttgtt tgggtttgtg aaggtattag atattttttt tggtttgggt    6840
attagttata tttttttttg ttatgtttta ttatttatag gatgggggta tttttttggga    6900
atttttatat ttaaaggtgt gtttttttttg tttaggttag ggtgcgtttt ttttgtttta    6960
tttagttaat atttattgag taattattgt atgttaggtt ttgttttaga tatagtagtg    7020
aatgagatag ttaaaatttt ttgtttattt taggtaattt aaggaaattt ttgtatttat    7080
aattttttttt tttttttttt tttttttttt tttttgagat agagtttcgt tttgtcgttt    7140
aggttggagt gtagtggtgt gattttggtt tattgcgagt ttcgtttttc gggtttacgt    7200
tattttttttg ttttagtttt tcggtagttg ggattatagg tattcgttat tacgttcggt    7260
taattttttt tgtattttta gtagagatag ggttttatag tgttagttag gatggtttcg    7320
attttcggat tttatgattt attcgtttta gttttttaaa gttttgggat tatagacgtg    7380
aattatcgta ttcggttttt aaaaaataat tttttaaaaa ttagtagggt gtggtggtgt    7440
gtgtttatag ttttagttat ttgagaagta gaagtgggag gatcgtttga gtttaggagt    7500
ttgaggttgt agtgagttgt aattatagta ttgtatttta gtttggatta tagaataaga    7560
ttgttttaaa ggaaaaaaaa taaaatataa aaaaagatat ttttaaaaga taaaaaattt    7620
tttgttttta gggagttgat agtttagatg gggagataga taagtaaata ataataggtt    7680
gggtgcggtg gtttacgttt gtaattttag tattttggga agttaaggcg ggtggattat    7740
ttgagattag gagttagata ttaatttggg taatatagtg aaaattcgtt tttattaaaa    7800
atataaaaat tagttgggcg tgttggggta tatttgtagt tttcgttatt ttggaggttg    7860
aggaaggaga gttatttgaa tttaggaggt agaggttgtt aggtagggtg gtttattttt    7920
gtaattttag tattttggga ggttgaggtc ggtagatcgt ttgagtttag gagtttaaga    7980
ttagtttggt taatatgttg aaattttatt tttattaaaa atataaaata attagtcggt    8040
tatggtggta tatatttgta gttttagtta ttgaggaggt tgaggtaaga gaattatttg    8100
aatttgagag gcggaggttg tcgtgagtcg atattgcgtt attgtattgt atagagtgag    8160
attttatttt aagaaaaaaa aaaaaaaaag gggttaggcg tggtggttta cgtttatatt    8220
tttagtattt tgagaggtta aggtgggtag attataaggt taggagatta agattatttt    8280
ggttaatatg gtgaaatttc gtttttatta aaatataaaa aattagttcg gtatggtggt    8340
ttgtaatttt agttatttgg gaggttgagg taggggaatc gtttgaattt gggaggcgga    8400
ggttgtagtg aattgagatt acgttattgt attttagttt ggcgatagag taagattttg    8460
ttttaaaaaa aaaaaaaaaa aaaaaaaaaa gaaaagaaaa gaaaaagaaa aaaaataata    8520
aaagagagag agagagtata gttatgatta tagtgttgta tattaatatt aggtgatatg    8580
atagatattg ttaatttttt tagttttagt tttttttagt tggaatatgg ggataatata    8640
atttttataa gtttaggagt tttaagtagg ttattgtgtg tatatttttt gttattgtaa    8700
gtgttataaa aaaatgtgga ttatttttaa gtgtattgtg atttatttta ttttatttta    8760
tttttgagat agagttttat tttgttattt aggttggagt gtagtggtgc gattttggtt    8820
tattgtaatt tttatttttt aggtttaagt gattttttttg ttttagtttt tcgagtagtt    8880
gagattataa gcgtgtatta ttatgtttgg ttgatttttt tgtattttta gtagagacgg    8940
ggttttatta tgttggttag gttggtttttg aatttttggg tttaagtaat ttatcggttt    9000
tggtttttta aagtgtaggg attataggta ggagttatta cgtttggtta tattttattt    9060
tatttattta ttttttgaga tggagttttg tttttgttgt ttaggttgga gtgtaatggt    9120
atgattttag tttattgtaa tttttatttt ttaggtttaa gagatttttt tgtattagtt    9180
tttcgagtag ttgggattat aggtatgtat tattaatgtt tggttaatta tatttttttt    9240
ttttttttgt agagatagtt ggggttttgt tatgttgatt agattggttt tgaattttttg    9300
gttttaagtg atttttttat tttagttttt cggagggttg ggattatagg tgtgagttat    9360
tgggtttggc gtgttattaa ttttcgaatg aggagtttta taatcgaagg gatttgggtt    9420
gattgttgta tttttagttt ttgatgtgtt gataggtata tagagaattg aaaatataga    9480
tatatgtttt tgtttttttg tatataagta tattttagtg tttcggattt ttatatatat    9540
tttggtatat cgatatgggg gtattattga ttataattat tcgttcgatt agggatgggt    9600
agtagatgga gaaatgttag gtaaggaggg tattttagtt atgggttata aggatgtatt    9660
tcgagtaatt tgtgggaatt taaggagttc gtataagtga ggtgtttagt aggattgaag    9720
```

```
tagggatggg ggaagtaggt tttagtaagg agaaagcggg ggtatgcgtg tgtgtaggat    9780
gagggaggtt acgtttagga tgggggtatg tttgtattta ggtttaggat gatatttttg    9840
atgtttatta gtagtaggtc gattgtgtag tagtttatat ttcgaggtgt attcgagggg    9900
ttatagtttc gtaagtttat agttataata tggaagcggt tttggaattt tcggagttgg    9960
taacgttagg agaatttgtt aggcgggcga gggagggagg ttgagttagg gtttttaggt   10020
tgtatttgta ttttacgtat attagtaatt attggtaaat tttttttttt gttttaaatt   10080
tttttaaaa ggaggatggg gaattttagg tagaggtaga ggttgaggtt gaagttagtt   10140
atattttgaa tttagggaat tttaattaat tttaatattt ttttgttttg tttttttagg   10200
gtttagggat tttgggtatg ttggtttagt atttttattt ttatttatgt ttagtttgtt   10260
tcgatttata tagttttttt ttttgttttt tgggagttta agttttattt gttttcgagt   10320
ttcgtgggtt ttaggttttt ggttttagac gtattagttt ttagggaagt cgtgtagaaa   10380
tagtatgagg ggttcgttat ttcgtttagt cgagatatag tgtagacgta ggttcgagtt   10440
ttagggggag ggtatagttt gaagtttggg gaattttttt tttcgaggtt tgaatcgttt   10500
cgattttacg cgatttcgta gtttcgatag cgttttcgtg cgattatttt gaggtttagg   10560
aaatcgtgtt tatttagcga ggggtcgttt aggtaggcgg gggacgcgtt tcgacggcgt   10620
tcgtagtagt cgcgtcgggg tcggtatagt acgtgcgtga gcgttatgta gtcgtagatc   10680
gtcgcggtta ttagcgttat cgagaatatt aggttttata tgaaggcgcg tagtagtttt   10740
agcgataggc gcgacggcgt tagtagcgcg gttattatta gtttcggtat gtcgtcgcgt   10800
ttcgggatta cggcggcgtt gtcggttagg ggtatttgta gtagcggcga agcggtgtta   10860
gggtttaggg gtttatcgtt tttggtttgg ggttttttcg tgtcgtcggg atttgtggga   10920
cgcgttgaag gaagaggcgg gcggtttcga tagaaaatag ttagagcgta ttatttattt   10980
gagtgttagg taaatatttg ggcgcgatag ggataggaaa taagggtagg gtgcggaggt   11040
tggggaggaa gaggttggaa aggggggaaa taaatgggcg gggtttagta ggttttgtgc   11100
ggggtttagg gtcggggcgg ggtttaggaa gatttagtag cgggtgggtg agggtttaaa   11160
ggcggtaatt tcgggtcggg tgcggtggtt tacgtttgta attttagtat tttggtaggt   11220
cgaggcgggc ggattatttg agattaagag ttcgagatta gtttgggtaa cgtggtgaaa   11280
tttcgttttt attaaaaata taaaaattag ttgggcgtgg tggcgggcgt ttgtagtttt   11340
agttatttgg gaggttgagg taggagaatc gtttgaattc gggacgtgga ggttgtagtg   11400
agttgagatc gcgttattgt attttagttt gggttataat agggaaattt cgttttaaaa   11460
aagaaaaaaa aaaaaggtaa tttcgagttt agataaattt taaggagggg attttggatt   11520
tttagttaag tgggcgatat ttggagtgag ggcggggta tatgtagagt aggtgcggtt   11580
tataagttaa aaaggagaaa gagttggaat ggtgggtttg gtttatgcgg gtgggcgggg   11640
agagggtgga ttttagagga ggtgaggttt aatattgggc gaagaaggcg ggagtttggg   11700
ttaatgagtt gacggtaggt cggggagggg gcggtggggt ggggtgggta atgggtaatg   11760
agacggaggg cggggtcggg atttaatatg gcgggttagg agggtttgga agacgaagaa   11820
gagggatagg taatgttagg tttaggatta ggagggaggc gcgggcggta ttagcggttg   11880
gaggaggttt cgggaggttc ggtcgacggt cgtcgtttgg tgttatttat ttaggggcgc   11940
gcgatttttt tttcggtttg gttttaaaga tttagtagta ttgattttat ttagttgtgg   12000
ttttaacggc gggtttagcg gtttcggttc ggcgtcgttt ttttgttggt ttaataggtt   12060
cgttagttcg tttttgtacg tttgtgattg gacggcggcg gttattgatg tttaagcgat   12120
aggttttggt tcgggagtta atttgtaggt gttgaggttt aggtttcgag agcgggtcga   12180
ggaggcgtgg atattttgat ttttaggggg taggtttggt tttttcgagg aggattcggt   12240
ttatgaatga ttggagtttt ggggttttgg tcgaaagagg aagtgggata gggtcgggtg   12300
tgatggggtt tagagttata gagttttgcg gttttgttgt ttttgtaaga agttagtttt   12360
tggttaggcg cggtggttta cgtttgtaat tttagtattt tgggaggtcg aggcgggcgg   12420
attacgaggt taggagatcg agattatttt aatatggtga aattttgttt ttattaaaaa   12480
tataaaaaat tagttaggcg tggtggcggg cgtttgtagt tttagttatt agggaggttg   12540
aggtaggaga acggcgtgaa tttaggaggc ggaggttgta gtgagttgag attgcgttat   12600
tgtattttag ttagggcgat agagcgagat ttcgttttaa aaaaaaaaaa aaaagtagtt   12660
agtttttttt ttttattttg taattttttt tcgatatttt tgaatatttt agggatagtt   12720
attatttaat tatagagtaa ttttattaag tttaagtagt ataattatat ttttgtagta   12780
tagattatga atttgaaatt cgaggatgaa gttatttgtt tgaagatata tattttgtaa   12840
aatagttatt cgtaaagatg tagggaaaaa ggtagcgatt tgtggttata tttttttttt   12900
ttttcggaag tagttattgg aacgttttta gttttttttt tttttttag atggagtttc   12960
gattatagtt tattgtagtt tcgaattttt gggtttaagt attataattt gtagttggga   13020
ttataggttt gtgttattat atttagttaa tttttaaaa attttttaga gagacgattt   13080
gttatattgt ttaggttggt tttaaatttt tggttttaag taatttttaa gtttttattt   13140
tttaaagtgg gattatagat gtaagttatc gtgtttgatt tttattttt gatttaatta   13200
atgtatgata ttagttattg atttgtttat attattattt cggacgtgtc gttttttgtt   13260
tttttgttag tttttttaa aaatatttta tttgttattt tttgtttttt tttattttt   13320
atttttattt atatattttt gtattgttaa ggttgttaaa tattatattg ttttgggatt   13380
```

```
ataattaagt ttatgaagtt gttatttaaa ggtgaatagt ggtttatgtt ttgttttagt   13440
gaataatgta ttttaagagt cgaatagatt attatgatta tattgtttat agtgtataaa   13500
tttttgtttt ttttggatta tttaattatt attttttttt tgttgtgtga ttagtttata   13560
ttaaagtttt gggggttttt ttgtttttgt ttttttgag atgggagttt tattatatta   13620
tttaagttgg aatgtagtgg tgtgatttcg gtttatcgta attttcgttt              13670
```

<210> 138
<211> 13670
<212> DNA
<213> Artificial Sequence

<220>
<223> chemically treated genomic DNA (Homo sapiens)

<400> 138

```
aggcggaggt tgcggtgagt cgagattata ttattgtatt ttagtttggg tgatatggtg      60
agatttttat tttaaaaaaa ataaaaataa aaaaattttt aaagttttga tgtaggttaa     120
ttatataata aaagaaaggt agtggttaag tagtttaggg agaataaaag tttgtatatt     180
atgggtaatg taattatagt ggtttattcg gtttttgaga tgtattgttt attgaggtag     240
aatataaatt attatttatt tttaagtggt aattttatgg atttagttat ggttttagga     300
tagtataatg tttagtagtt ttgatagtat aaaggtatat ggataaaagt gggaggtgga     360
aaggggtaag aggtgataag tggagtgttt ttaaaggaaa ttaataggga aataaaaaac     420
ggtacgttcg ggtagtgat gtgagtaagt tagtggttga tgttatatat tggttaagtt      480
aagaaataaa ggttaggtac ggtggtttgt atttgtaatt ttattttggg aggtggaggt     540
ttgaggattg tttgaggtta ggagtttgag attagtttgg ataatatagt aagtcgtttt     600
tttgaaaagt ttttgaaaaa ttagttgagt gtggtggtat aggtttgtag ttttaattat     660
aggttgtagt gtttgagttt aggagttcga ggttgtagtg agttatgatc gaaattttat     720
ttgaaaaaaa aagaaaaagt taaaaacgtt ttagtggttg ttttcgggag gaaggggagg     780
tgtagttata gatcgttgtt ttttttttta tatttttgcg gatggttatt ttataagatg     840
tatgttttta ggtaaatgat tttattttcg aattttaggt ttatgattta tattatagga     900
atgtgattat gttatttaga tttaataggg ttgttttatg gttaagtgat ggttgttttt     960
aaaatgttta aggatatcgg gagaaggttg taaaatagga ggaagaagtt ggttgttttt    1020
tttttttttt ttgagacgga gtttcgtttt gtcgttttgg ttggagtgta gtggcgtaat    1080
tttagtttat tgtaattttc gtttttttggg tttacgtcgt tttttttgttt tagttttttt    1140
agtagttggg attataggcg ttcgttatta cgtttggtta attttttgta tttttagtag    1200
agatagggtt ttattatgtt aggatggttt cgattttttg atttcgtgat tcgttcgttt    1260
cggtttttta aagtgttggg attataggcg tgagttatcg cgtttggtta gaagttggtt    1320
ttttgtaggg atagtagggt cgtaaggttt tgtgatttta ggttttatta tattcggttt    1380
tgttttattt tttttttcgg ttagaatttt agaattttag ttatttatag gtcgggtttt    1440
tttcgggggga attaggtttg tttttttagga attagggtat ttacgttttt tcggttcgtt  1500
ttcggagttt gggttttaat atttgtagat tggttttcgg gttaggattt gtcgtttgaa    1560
tattagtggt cgtcgtcgtt taattataga cgtatagggg cgggttggcg ggtttgttgg    1620
gttagtagga ggacggcgtc gggtcgaggt cgttggattc gtcgttggaa ttatagttgg    1680
gtgaagttag tgttgttagg tttttggagt tagatcgaag ggagggtcgc gcgtttttgg    1740
gtgggtagta ttaggcggcg gtcgtcggtc gggtttttcg aagttttttt tagtcgttaa    1800
tatcgttcgc gttttttttt tagttttaga tttggtattg tttgtttttt tttttcgttt    1860
tttagatttt tttgattcgt tatattaggt ttcggtttcg tttttcgttt tattgtttat    1920
tgtttatttt attttatcgt tttttttttcg gtttatcgtt agtttattgg tttaggtttt  1980
cgtttttttc gtttaatgtt aggttttatt tttttttagga tttatttttt tttcgtttat   2040
tcgtataagt taggtttatt attttaattt tttttttttt ttggtttgta ggtcgtattt    2100
atttgtata tgtttcgttt tttattttag gtgtcgttta tttaattgaa gatttaggat     2160
ttttttttta aggtttgttt gggttcggaa ttgttttttt tttttttttt ttttgagacg    2220
gagttttttt gttgtgattt aggttggagt atagtggcgc gattttagtt tattgtaatt    2280
tttacgtttc gggtttaagc gatttttttg ttttagtttt ttaagtagtt gggattatag    2340
gcgttcgtta ttacgtttag ttaatttttg tatttttagt agagacgggg ttttattacg    2400
ttgtttaggt tggtttcgag tttttgattt taggtgattc gttcgtttcg gtttattaaa    2460
gtgttgggat tataggcgtg agttatcgta ttcggttcgg aattgtcgtt tttagatttt    2520
tatttattcg ttgttgagtt tttttgggtt tcgtttcggt tttaagtttc gtataggatt    2580
tgttaggttt cgtttattta tttttttttt ttttaatttt ttttttttta gttttcgtat    2640
tttatttttg ttttttgttt ttgtcgcgtt taggtgttta tttggtattt aggtgagtgg    2700
```

tgcgttttgg ttgtttttg tcggagtcgt tcgtttttt ttttagcgcg ttttataaat 2760
ttcgacggta cggaggggtt ttaggttaag ggcgatgggt ttttgagttt tgatatcgtt 2820
tcgtcgttgt tgtaggtgtt tttggtcggt agcgtcgtcg tggtttcgga gcgcggcgat 2880
atgtcggagt tggtggtgat cgcgttgttg gcgtcgtcgc gtttgtcgtt gaagttgttg 2940
cgcgttttta tgtggagttt ggtgttttcg gtggcgttgg tggtcgcggc ggtttacggt 3000
tgtatagcgt ttacgtacgt gttgtgtcgg tttcggcgcg gttgttgcgg gcgtcgtcgg 3060
agcgcgtttt tcgtttgttt gagcgatttt tcgttgggtg agtacggttt tttgaatttt 3120
aaggtgatcg tacgggggcg ttatcggggt tgcggggtcg cgtggggtcg agacggttta 3180
agtttcggga gagagggttt tttaggtttt aggttgtgtt tttttttag agttcgggtt 3240
tgcgtttgta ttatgtttcg gttggacgag gtaacggatt ttttatgttg tttttgtacg 3300
gttttttga gaattggtac gtttggggtt aggggtttga ggtttacggg attcgggggt 3360
aggtggagtt tgggttttta agaggtaggg aggagggttg tatagatcgg gataagttga 3420
atatagatgg ggataggggt attgagttag tatatttagg atttttaggt tttgaaggga 3480
taggataaag gggtattgag gttgattgaa gtttttgga tttagggtgt agttggtttt 3540
agttttagtt tttgttttg tttgggattt tttattttt ttttagggga agtttagagt 3600
aggaaggagg gtttgttaat ggttgttgat gtgcgtaggg tgtaggtgta atttgagggt 3660
tttgatttag tttttttttt tcgttcgttt ggtaggtttt tttggcgtta ttagtttcgg 3720
gagttttaga gtcgtttta tgttgtggtt gtggatttgc gaggttatgg tttttcggat 3780
gtatttcggg atgtggattg ttatataatc gatttgttgt tggtggatat taaagatgtt 3840
attttaggtt tgggtgtaga tatattttta ttttaggcgt ggtttttttt attttgtata 3900
tacgtatatt ttcgtttttt ttttgttgga atttattttt tttattttg ttttagtttt 3960
attgggtatt ttatttatgc gagtttttg aatttttata ggttattcga agtgtatttt 4020
tgtggtttat gattggggtg ttttttttgt ttggtatttt tttatttatt atttattttt 4080
ggtcgagcgg atggttgtgg ttagtggtgt ttttatgtcg gtgtattaag gtgtgtgtgg 4140
gagttcggga tattgggatg tatttatgtg taagggggta gggatatgtg tttgtgtttt 4200
taattttttg tatatttgtt aatatattag gggttgaggg tgtagtaatt aatttaggtt 4260
ttttcggttg tggaattttt tattcggaaa ttaatgatac gttagattta gtggtttata 4320
tttgtaattt tagtttttcg ggaggttgag gtgggaggat tatttgaggt taggagttta 4380
agattagttt ggttaatata gtaagatttt aattgttttt ataaaaaaaa aaaaaaaatg 4440
taattagtta ggtattggtg gtgtatgttt gtaattttag ttattcggga ggttgatgta 4500
ggagaatttt ttgaatttgg gaggtggagg ttgtagtgag ttgagattat gttattgtat 4560
tttagtttag gtaataagag taaaatttta ttttaaaaaa taaataaata aaataaagta 4620
tggttaggcg tggtggtttt tgtttgtaat ttttgtattt tgggaggtta aagtcggtgg 4680
attgtttgag tttaggggtt taagattagt ttggttaata tggtgaaatt tcgtttttat 4740
taaaaatata aaaaaattag ttaggtatgg tagtgtacgt ttgtagtttt agttattcgg 4800
gaggttgagg taagagaatt atttgaattt gagaagtgga ggttgtagta agttaagatc 4860
gtattattgt attttagttt ggatgataga gtgagatttt gttttaaaaa taaaataaaa 4920
taaaataaat tataatatat ttaggagtaa tttatatttt tttatagtat ttatagtaat 4980
aaggaatatg tatatagtaa tttatttaag gtttttgggt ttatgaaggt tgtattattt 5040
ttatgtttta gttggaggaa attgaggttg gggaggttaa taatgtttat tatattattt 5100
gatgttaata tgtagtattg tggttataat tgtgtttttt tttttttttt tgttgttttt 5160
tttttttttt tttttttttt tttttttttt tttttttttt ttttttgaga tagagttttg 5220
ttttgtcgtt aggttggagt gtagtggcgt gattttagtt tattgtaatt ttcgtttttt 5280
aggtttaagc gatttttttg ttttagtttt ttaagtagtt gggattatag gttattatgt 5340
cgggttaatt ttttgtattt tagtagagac ggggttttat tatgttggtt aagatggttt 5400
tgattttttg attttgtgat ttgtttattt tggtttttta aagtgttggg agtataggcg 5460
tgagttatta cgtttggttt tttttttttt tttttttttt gagatggagt tttattttgt 5520
gtagtgtagt ggcgtaatgt cggtttacgg taattttcgt tttttaggtt taagtgattt 5580
ttttgtttta gttttttttag tagttgagat tatagatgtg tgttattatg atcggttaat 5640
tattttgtat ttttagtaga aatggggttt tagtatgttg gttaggttgg ttttgaattt 5700
ttgggtttaa gcgatttgtc ggttttagtt ttttaaagtg ttgggattat aggagtgagt 5760
tattttgttt ggtaattttt atttttttggg tttaagtgat tttttttttt tagtttttag 5820
agtagcgggg attgtagata tgttttaata cgtttagtta atttttgtat ttttagtgga 5880
gacgggtttt tattatgttg tttaggttag tgtttaattt ttgattttaa gtgatttatt 5940
cgttttggtt ttttaaagtg ttgggattat aggcgtgagt tatcgtattt agtttattat 6000
tgtttatttg tttgtttttt tatttagatt gttagttttt tgagggtagg gagttttttg 6060
ttttttgggg gtgttttttt ttgtgttttg tttttttttt tttgagatag ttttgttttg 6120
tggtttaggt tggagtgtag tgttgtaatt gtagtttatt gtagttttag atttttgggt 6180
ttaagcgatt tttttatttt tgttttttaa gtagttggga ttataggtat atattattat 6240
attttgttaa tttttaaaaa attattttt ggaggtcggg tgcggtggtt tacgtttgta 6300

```
attttagaat tttgggaggt tgaggcgggt ggattatgag gttcggagat cgagattatt    6360
ttggttaata ttgtgaaatt ttgtttttat taaaaatata aaaaaaatta gtcggacgtg    6420
gtggcgggtg tttgtagttt tagttatcgg gaggttgagg taggagaatg gcgtgaattc    6480
gggaggcgga gttcgtagtg agttaagatt atattattgt attttagttt gggcgataga    6540
gcgagatttt gttttaaaaa aaaaaaaaaa aaaaaaaaaa aaaaaaaatt atagatatag    6600
gggttttttt aagttgttta ggatgaatag ggagttttga ttgttttatt tattgttgtg    6660
tttagaataa ggtttggtat gtagtagttg tttaataaat gttagttgaa taaagtagag    6720
gaaacgtatt ttggtttggg tagaggaagt atatttttgg atatgggggt ttttaggaga    6780
tgtttttatt ttgtggatag tgaaatatgg taggagggaa tgtagttggt gtttaggtta    6840
ggaggggtat ttagtatttt tatagattta ggtaggtagt agtggtattt ttgaggtttg    6900
tgtttttgat ttgggtatgt gtaggggtgt aggtgtatgg ggttatttta ggagtaggtt    6960
ttatattttt tttttaattt ttttatttta gattattttt tgtattatat tagttagttt    7020
tttcgttttt attatatgtt tttgttttag ttgttttggt tgttcgagaa gttgttgttt    7080
atgtttgatt tttaggtata gagggtgtag gttgaaggga ggagggaagt aggtcggggt    7140
tgggtaggtt gggtattgaa gttagagttt tgttgtgtat ttagattttg aagattattt    7200
ttatttatcg taagataggt attttatgtt tgatttttag cgagttcgag gttttttttt    7260
ataatttttt atagtttggt ggttttattg ggttttttaa ttattatcga aattttttta    7320
ggtgagatta gttgttaagt ataagtttag gagatttgta ggggtagggg ggtgggaggt    7380
aattattttt ttgtttgttt gtttgtttgt ttgtttgttt tggttatagg aatttttttt    7440
tggaatttta ggagttgatt atatttatat tgttgttgtg gggggagaag gatatttatt    7500
tggagttggg gttggtggaa gttatcggta gtcgttttgt gtcgggtcgt ttggaggttt    7560
atattttgtt aggtataggg tattggattt tatagagtaa tttttaggag atgtattagt    7620
atatgtgggt ttttttgtaa gatttgttgg attagtggtt tttgttggtt tgtttaggag    7680
gtatggatat ttaggaagga atatatattt attatttgtg tgtgtttggg agtttatata    7740
atgtttatat atgggtgaat ttttgaatcg tttataggta tgggattttt ggattttata    7800
taagcgtatt tatgggtgtt tcgggatata ttaattttta tatttatata taggtatgga    7860
tgtatgtgtg tgtaataaat attttgattt tgggaattgg gtatattttt tttttagtgg    7920
agttagatgt cgagatcgag tgttttattt tttttttttt tggggtttta ttttgcgttt    7980
tgttaaagtc gtttttttgt tatagttgta tagattttaa attttgattt ttttgttttt    8040
gttttcgatt ttttgtttgg gtttttagtt tagtgttatt ttgaatataa tattgttgat    8100
tatatttggt tattttagtt taaatgtatg ttaattgaaa tgaaatataa tgaaaatgta    8160
gtgtttagtt ttattagttg tattttattt tttttttttt gagatagagt tttgttgtgt    8220
cgtttaggtt ggatggagtg tagtggacgt aatttaggtt tattgtaatt tttatttgtc    8280
gggtttaagc gatttttgtg ttttagtttt tcgagtagtt gggattatag gagcgttatt    8340
aggtttgatt aattttagta tttttagtag agatggggtt ttattatgtt ggtcgggttg    8400
gtttcgaatt gttgatttta agtgatttgt ttattttggt tttttatagt gttgggatta    8460
taggcgtgag ttattgtgtt tggttatatt agttgtattt taagtgttta attgtcgtat    8520
tagtgaagaa agttgtgtgt ggtagcgttg atgagggtaa ttttagttta agagtagggg    8580
ttagggtcga ttatagtggt ttaaatttgt aattttagta ttttgggagg ttgaggtggg    8640
cggattacga ggttaggaga tcgagattat tttggttaat ttggtgaaat gttgttttta    8700
ttaaaaatat aaaaaattag tcgggcgtgg tggtgggcgt ttgtagtttt agttatttag    8760
gaggttgagg taggagaatg gtatgaattc gggaagtgga gtttgcggtg agttgagatc    8820
gcgttattgt attttagttt ggtaatagag cgagatttta ttttaaaaaa aaaaaaaaag    8880
agtagggggt ttttttgttt ggtttttggt aatttttttta agggtagatt tttatgagta    8940
ggtaggtggg tgtgtagatt ttttattttt ttttagttag ttttaggaga gtttttataa    9000
gttttttttt ttatttttcg ttatgatttt aggtttggtt aggttaagtt tttttgtttt    9060
agtttgggtt ggtttgtttt agatttttgg gaaaatagtt ttcgtttttg ttaggataag    9120
tgagatttttg gatttcgggt aggaagggat tagtttttag aaaagttatt tgggatttga    9180
ggtttgtttt tttagttttt ggaaattttt gttttaaatt agttatagtt ttcgttgatt    9240
ttggagtttt ttggggtggg gatgggtggg gggtggcggt tatatgtgga gggtgcgtta    9300
tttagtttta ggttgatcgt agtattagtt tgtatttttt tggtttgtgt ttggtttttt    9360
ggggatgttg ttattattat ttgtattatg aggattgaga ggttttgaga aatatatttt    9420
tttgttttttg agataggttt gtttgttggt tatttttttt tgtttttttt tatttttagt    9480
ataatatttt tttttttgttt gtttgtttgt gagatggagt tttgttttgt cgtttaggtt    9540
ggagtgtaat tgcgcgattt cggtttattg tagtttttttt ttaggtttaa gcgatttttt    9600
tgtttttgtt ttttgaatag ttgggattat aggtgttcgt tattatattt agtttatttt    9660
tgtatttttt tttttttttt tttttttgga gacggagttt cgttttgttt taggatggag    9720
tgtagtggtg cgatttcggt ttattgtaag tttcgttttt cgggtttata ttattttttt    9780
gttttagttt ttcgagtagt tgggattata ggcgtttatt attacgttcg gttaattttt    9840
tgtattttta gtagagatag ggttttatcg tgttagttag gatggtttcg atttttttgat    9900
tttatgattt attcgtttcg gtttttttaaa gtgttgggat tataggcgtg agttatcgta    9960
```

```
tttggtttat ttttgtattt ttagtagaga tggggtttta ttatgttggt taggttggtt   10020
tcgaattttt gattttatta ttcgtttatt tcggtttttt aaagtgttgg gattataggc   10080
gtgagttatt gcgtttggta taatattttt ttatttaata tagatattag gtagtgtttt   10140
aggttttagt gatagagtgt gagttagaaa gagtttaagg aggagagagt tgatagggtt   10200
tagggaaggt tttattgaag atttataggt gggattttag ttagaggatg tatttatttg   10260
ttgttgtttt gagatagggt tttattttgt tgtttaggtt ggagtgtagt ggtgtgacgt   10320
aagtttatag tagtttttat tttttgggtt taattaattt ttttgtttta gtttttttgag   10380
tagttgggat tatagggtta tattattatg ttaggttaat ttttaatttt tttttgaga   10440
atggaggttt gttttattgt ttaggcggga gtgtagtggc gttattttag tttattgtaa   10500
tttttatttt ttgggtttaa gtgatttttt tgttttagtt tttttagtag ttaggattat   10560
agatatacgt tattatattt ggttaatttt tgtaatttta gtagagacgg ggttttatta   10620
tgttggttag gttgattttt aacgtttgat tttaagtagt tcgtttgttt cggtttttta   10680
aaaatttttt atagagatag acgttttatt atgttgttta gagtggtttt aaatttttcg   10740
gtttaagtga ttttttttatt tcggtttttt aaagtgttgg gattttaggt atgagttatc   10800
gcgttcgttt aggatgtatt ttttggtgga gtgggaagta gtgattagga aggttttagg   10860
cgggaagaag tttgggttat tggagttata atacgtaggt tagggttttg ttaaggtaga   10920
gtgagcgagg aggttttaga gaagaggtta tatagttaga tcgttagggg ttgcgaggag   10980
tttggattcg atttttaagg ggagttaggg gagggttttt agtagggaag ggatatggtt   11040
tgtattttga agagtttttt gtggcggttg gatagtttta cgtaagagtg cgagcgtcga   11100
agtttgcggg gggttgcgcg cggttatagt atagggcggt tttattgttt cgatcgagta   11160
gggattttta gagtcgggtt taattcgggg tttttttgtt aggggaggag taatgttttt   11220
cgtttcgggg aggcgagaag acgggtagga ggaggaggga gtttttgaga cgtggggtcg   11280
cggacgtcgg agttcgcggc ggaggtttgg ggcgaggagg tttgcggata ggattaggac   11340
ggcgtttagg ggtaggtggg ttcggcgatg gttgtcgggg tgacggggga cgggttgatg   11400
gttgatcgga acgggtagcg gttgttcgag tgatcggagg ggtttaggta aaggttgacg   11460
ggaggtttgg gatagttgtt cgggcgacgg agattcgttg acggttgtta gggcgacgga   11520
aaggcgtttt ttttcgtttt cgcggtcgtc ggcgggaggg aggcgggggt tttttgggga   11580
agacgatcgc gtcgattggg tagagttttg gcgagtaagt taatagaaaa tcgtgtttcg   11640
tcgtagattc gttttttttta gtttgagtta atttcggttc gttttttttt tttttttttg   11700
gtttaagggt ttggcgcgtg tattttttag ggcgggagcg gggatttcgg gggtttgggt   11760
tgggattttt cgtatgatcg gtttgttttt tttttttgttt ttagattatt ttttattatt   11820
tatttattta tttatttatt tatttattta tttcgagggt tgtattgatg gtttagtagt   11880
agaattttta tttttttattt atttatttat ttatttattt gtttttgtta ttagttttag   11940
gaggggtggt ggaagataaa gtcgggtttt gatattgtta gtagggtgtg attaggtttt   12000
ggtggaatga gtaattgggg aggagtattt atttgggggga agggagggaa ggtttttttgg   12060
aggaagtagt tttggagttg aattttgtaa agttgcgttt cgatttatag aagtttttta   12120
cgtcgggatg atttagtatt ttcgtgagat tgggaggaaa gtttgggtta ggaagacgtg   12180
agattataaa aggttttata tgttagtagg ggagggtttc ggatagattc gtgtgtttgg   12240
aatgttattt gggggatggt gagaaggtcg gattagagga cgggaaggtt ttagaggagg   12300
atgggcgtgg tttaggtagt ggagaacgag atttaagttt aggtattgga agaggagtag   12360
atacgttgag aaggtataga ggtaggtttg ggatatattt agtttgacgg cgttggattt   12420
cggggtaggg atttagaagt atgttatggg ttaaagataa aaatagttta gtaagtatcg   12480
gtgtattgtg gggtagagag gtttttaagt tgggagcgag ggtgaagttt ttttagagta   12540
gaatggagaa taagattgga gattgaattt tgaggatttt taatatttta gagataggta   12600
gaggagagag gttataaagg taggaggtga ggttaggtac ggtggtttac gtttgtaatt   12660
ttagtatttt gggaggtcga ggcgggtaga ttataaggtt aggagatcga gatcgttttg   12720
gttaatatgg tgaaatttta tttttattaa aattataaaa aattagtcgg gtatggtggt   12780
atatgtttgt agttttagtt attcgggagg ttgaggtagg agaattattt gaattcggga   12840
ggtagaggtt gtagtgagtt aagattatgt tattgtattt tagtttgggc gagagaggga   12900
gattttattt taaaaaaaaa gtttaggtat agtggtttat atttgtaatt ttagtatttt   12960
gggaggtcga ggcgggagga atacgaagtt aggagtttga gataagtttg tttagtatag   13020
cgaaatttcg tttttattaa aaatataaat attagttagg cgtggtggtg ggcgtttgta   13080
attttagtta ttcgggaggt tgaggtagga gaatcgtttg aattcgggag gtggaggtta   13140
tagtgagtta agattatatt attgtattat agtttggatg atagagttag attttatttt   13200
aaaaaaaaaa aaaaaaaaaa aggtaggagg tgaattaggt aaaagtgatg ttttagtaat   13260
ggaggaggga ggagcgttaa ggaataggga tgtagttttt ggcgttataa gttgttgaga   13320
gataagcgtg gtggtttatg tttttaattt tagtattttg ggaggtcgag gaaggaggat   13380
tacggtagat taagagtttt gagattagtt ttggaaatat tgtaagattt tttgtttttt   13440
aaaaaaaaaa aaaaaaattt aattgttggg tatggaggcg tgtgtttgta gttttagttt   13500
tttaggaggt tgagatggga ggatcgtttg agtttgtgag gttaagatta taatgagttg   13560
tgattatgtt attgtatttt agtttggaga tagagtaaga tcgtgtttta aaaataaata   13620
```

aataaaaatt taggtcgggc gtagtggttt attcgtgtaa ttttagtatt 13670

<210> 139
<211> 3479
<212> DNA
<213> Artificial Sequence

<220>
<223> chemically treated genomic DNA (Homo sapiens)

<400> 139

gttggagcga agtttgcggg acgtcgtaga gaaaattatt taaaagtacg gtattaattt 60
cgaggagatc gcggtcgagg agagttggga ttatgtgtag ttttaggtaa gttttttttt 120
tttagttttt tttcggattg attcgtttta gttttgtgtt tggaggagat tttattttaa 180
cgtgggttcg atttttagtt ttacgatttt aatatatttt aatttttttt aggttcgacg 240
tttttttattt tttagatgat ataattgttt tcggcgtcgt ttggtttttt agtatttaga 300
ttttggcgtg gtttcgttat ttatttcgag agatttcgtt ttcgttttcg tttcgattag 360
aggttgttag gtttttagtt ttaagatttt agcgagatac ggttttttat ttcgtagtga 420
ttttggtttt tatcggattt cgttttcgat gagatttata tggtttcgta tttttattta 480
tttgtcgggt attagaggtt taattgtatt atttatttaa tttttagttt atagcgcgat 540
tttgtagttt ttttttagt tttaggttat ttcgtgattc gtttttttta gttttatgtt 600
ttcgtaatgt tttttggttt cggttttatc gtgattttag tttattgttt tttattttat 660
atttattatt tagtttttttg tttttcgatt tgattttata tttattattt tgtttttttga 720
tttttaatat tatatgtttt tagtttcggt ttttttgatt cgtatttttt ttttagttgc 780
gttgttgcgg ttggtattat tcgtaggatt ggttttaagt ttttattttg agaggtaacg 840
ggtcggaggc gtatcgcgtg ttttgttttt gttataattt gtcggcgatt aacgatttta 900
taattttaga taaggtgatt ttgttttagt ttagtaggtt tggatatttg gcgcggttta 960
gatatagtgt agatatttgc gttgtttttg tagagagtta tatttatcgc gaggtgggta 1020
ggggttcgga gtataaattt gtcgaatggg gcggggtttg cgggaggggg agggttgtta 1080
gtgagtagcg gtttgagaaa gggcggggtt tacgagaaaa ggaaaggtac ggtaggaggg 1140
gcggggtttt ttgaaggggg cggggtttgt aggaagggta gggtttaaag aaaaggttgg 1200
gttggttttg gaatatagat gtttagggag gggttaggtt tggaaaaggt gaagcgaggg 1260
tgtattagta aggagattag agtgttttgg tgattagggg agcgggtaga tgtttgaaga 1320
cggtgagggt tggtttgaaa agaacgttgg gtttgggttt gagagtttta gaaagaattt 1380
ttttaatttt tttttatatt ttttagggtt gcgcgtaggg tttttagaag tggttgtata 1440
ataattttat ttttatagtg ggtatttgtt tgggcgtcgg tttattcgag gtgatttggt 1500
ttcgttttta ttcgcgatcg gttttaaatt tttagatggt tttgtttttt atttcgcgtt 1560
tttcggttgt ttgggaagga cgagtttagg gcggagcgta gtttatttcg gtttttttcgt 1620
cgttttattt agtatcggag ggtgggggcg gtttagtttt agggagtttt gattgggtgt 1680
acgtagggaa agtttttttgt tattggttgc gatttttttt ttttttttttc gtagatgatt 1740
gttatggtgt tgagcgtata gttatagcgt agggtatttc gtcggaaatg cgagtcgtac 1800
gtgtcgggcg ttggggattc gagtttcggg tttagtttga tcgttgacgg cggcggcggg 1860
tatagcggta gtttgtgggg tggttggggt atggcgggtg tttgttttaa ttggggagat 1920
aaggtatcgt agggtaagtt gtttatggtt ttggggtttt ggtcgttgtg ggtttaagac 1980
gaggattagt ttgatattgg aagtgcgggc gtagaattag aggaggtata attagaggtt 2040
gaggtagagg gggaagatag atgagttttt aaaataaagg attttgggtt tgttttcgat 2100
tttatttttt tttagttttt atttttattt gtagtagtta ttttcgtttt attagttagt 2160
tttgcggtag ttttcgtcga gtttcgtttt tttttattta ttttttttttt tagttttttt 2220
tcgtttaatt tacggtttta tttttgattt ttttcgttcg ggtgggtatt attttcgttt 2280
cgttagtatt ttttcgattt ttttgatttt attttttttt tttatagttc gggtttatga 2340
cgttttcgat atttttgtgt agaaatttgg attacgttta taatcggttc gttcgatatc 2400
gttaggtttc gttttttttta aagtttcgtt tcgttttcgt tacgtgcgtt gggtattttt 2460
ttgttgtttg taaatatttg tttaattttt agttcgtttg gagtttttcg tttttatatt 2520
tttttgggga tttacgtggt tgcgtgtttt tgttgttgtt atttttttta cgggatttgg 2580
ggttttcgtt tatagttttt tgtttttattt tgtcggttta ttattattta taagattatt 2640
ttttatttaa attttaaata aattttttgc gtttttggta aaatagtttt attttttgtt 2700
agaatataaa taaataaatt ttgagagggg aggaaggaaa tcgtttagtt tagggtttat 2760
ttaggagagg gatgagatta gaaagtttaa tatattgttt gtgtagcgga gataaagtta 2820
agattttagt atttatttat aaatatttcg ttatattaaa aaaaaaaaaa atgtttaggg 2880
tttatttggt tttgtttttg tatagaaagg gtttattttt attttgtgat tttataggtt 2940

atggagtgag ggtggtagag aggggtagaa attttagggg gaggggtggt tgggaaaaag 3000
taaaggggat aagttaatgt gtaattagcg ttttttaaga tatgtagagg agtgggggtg 3060
gtttgttagg ggttgaaaag aaaagttagt gttgtatttg gggggttgtt ttatttttgt 3120
ttttataatt tttgatattt cggagtgatt tgttttttta gatatttatt gtgaggtttt 3180
aatatcgggg tttatttttt ttttagtttt agtttgttta gtttttttaat taagttttgg 3240
gggttttttt aatggggggg atggttttag ttgtttaggt ttttgaggtt aattttttta 3300
tattatagtt ttttttttaa ataagaagta tgaggtgagt tggaggattt tttttgggag 3360
gagggtgttt tggggggtga gttagttttg gggttttttt ttagtttttg attaggcggt 3420
aaatgtgatg ttgggtttta cgttcgttgg tggagatttc gaagatatag gcggtgtag 3479

<210> 140
<211> 3479
<212> DNA
<213> Artificial Sequence

<220>
<223> chemically treated genomic DNA (Homo sapiens)

<400> 140

ttgtatcgtt tatgttttcg aggtttttat tagcgagcgt ggggtttagt attatattta 60

tcgtttggtt agggattgaa gggggatttt aaaattggtt tattttttag aatatttttt 120
ttttagggag ggttttttag tttattttat gtttttttatt tggggagagg gttgtgatgt 180
aaaggggttg attttagagg tttaagtaat tggggttatt tttttttatta gaggggtttt 240
taaaatttgg ttggagagtt gaataggttg ggattgagga aaggataaat ttcgatattg 300
ggattttata gtgggtgttt gaaaggatag attatttcgg agtattaggg attgtgggga 360
taggagtgag ataatttttt aggtatagta ttggtttttt tttttagttt ttgataggtt 420
atttttattt ttttgtatgt tttggagagc gttagttata tattggtttg ttttttttat 480
ttttttttag ttattttttt ttttgaaatt tttgtttttt tttattattt ttattttatg 540
atttgtgaga ttataaagtg aagatgaatt tttttgtgt aggagtagag ttaggtgggt 600
tttggatatt tttttttttt ttaatataac gagatattta taagtgggtg ttagggtttt 660
gattttattt tcgttgtata agtagtgtgt tgaatttttt aattttattt tttttttaag 720
tgagttttga gttagacggt tttttttttt ttttttaaa gtttgtttgt ttgtgttttg 780
atagaggata aagttatttt attaaaaacg taggggattt atttgaggtt tgggtgaaaa 840
ataattttgt gggtggtggt aggtcgatag atggggatag gaagttgtgg acgaaagttt 900
taggtttcgt gggagaggtg atagtagtag gggtacgtag ttacgtgggt ttttagggga 960
atgtgaaggc ggagggtttt aggcgaattg gggattaaat aaatatttat aggtagtagg 1020
gaagtgttta gcgtacgtga cgggggcggg gcgggatttt ggggagggcg gggtttaacg 1080
gtatcgagcg agtcggttgt agacgtggtt taggttttttg tataggaata tcgagagcgt 1140
tatgaattcg agttatagag aaaggagatg aggttagaga agtcgaaggg gtgttggcga 1200
gacgggggtg atgtttattc gggcgagaaa ggttaggggt ggggtcgtga attgggcggg 1260
aaggggttgg agaaggggat aggtagaaaa gggcggggtt cgacgggaat tgtcgtaggg 1320
ttggttgatg aggcgggaat agttgttata agtgggggta gaggttgaga aggagtaagg 1380
tcggaagtaa gtttagggtt ttttattttg gaggtttatt tgtttttttt ttttgtttta 1440
gtttttaatt gtgttttttt taattttgcg ttcgtatttt tagtgttagg ttggttttcg 1500
ttttgaattt atagcggtta gagttttagg gttatgggta gtttgttttg cggtgttttg 1560
tttttttagt tggggtaggt attcgttatg ttttagttat tttatagatt gtcgttgtgt 1620
tcgtcgtcgt cgttagcgat taggttgggt tcggggttcg aatttttagc gttcggtacg 1680
tgcggttcgt attttcggcg gagtgttttg cgttgtagtt gtacgtttag tattatgata 1740
gttatttgcg gagaaagggg agggagatcg tagttaatag taggaggttt tttttgcgta 1800
tatttaatta gggttttttg aagttgggtc gtttttattt ttcggtgttg ggtggagcgg 1860
cgggagggtc ggggtgggtt gcgtttcgtt ttgagttcgt tttttttagg taatcgaagg 1920
acgcgaaatg aagggtaggg ttatttaggg atttagggtc gatcgcgggt gggggcgggg 1980
ttagattatt tcgagtaggt cgacgtttag gtaaatgttt attatggaaa taaggttgtt 2040
gtgtagttat ttttggaggt tttgcgcgta gttttggggg gtgtagggaa aagttaaagg 2100
gatttttttt gggattttta agtttaggtt tagcgttttt tttaggttaa tttttatcgt 2160
ttttaggtat ttattcgttt ttttagttat tagggtattt tagttttttt attagtgtat 2220
tttcgtttta ttttttttaa gtttggtttt tttttagata tttgtatttt agagttagtt 2280
tagttttttt tttaggtttt gtttttttttg tagatttcgt ttttttttaga gggtttcgtt 2340
ttttttgtcg tattttttttt tttttcgtag atttcgtttt tttttaggtc gttatttatt 2400

gatagttttt tttttttcgt aggtttcgtt ttattcgata ggtttatgtt tcgaattttt 2460
gtttatttcg cggtagatgt ggttttttgt agggatagcg tagatgtttg tattgtgttt 2520
ggatcgcgtt aggtgtttaa gtttgttgag ttggggtaag attattttat ttaggattgt 2580
ggagtcgttg gtcgtcgata agttgtagta ggagtagggt acgcggtgcg ttttcgattc 2640
gttatttttt aggatgagga tttggaatta gttttgcggg tagtgttagt cgtagtagcg 2700
tagttgggag agggatacga gttagaggga tcgggattgg agatatatga tgttggggat 2760
taggggataa ggtgatgggt atgaggttag gtcggggagt aggggattaa gtggtgggtg 2820
tggagtgggg gatagtgggt tggggttacg atggggtcgg agttagggga tattacggga 2880
atatggggtt gggaaagacg agttacgggg tgatttgggg ttggggaagg agttgtaggg 2940
tcgcgttgtg gattagagat tgggtgggtg gtgtaattga gtttttgata ttcgataggt 3000
gaataggagt gcgagattat gtgagtttta tcgggggcgg ggttcggtgg aagttagagt 3060
tattacgggg taggaagtcg tgtttcgtta gggttttggg attaggggtt tgataatttt 3120
tggtcggggc gggggcgggg gcggagtttt tcgaggtaga tggcgaagtt acgttagggt 3180
ttgggtattg ggagattagg cgacgtcggg gatagttgtg ttatttgggg agtggggagc 3240
gtcgggtttg ggagggattg gggtgtgtta ggatcgtaga gttgggggtc gggtttacgt 3300
tggggtggag tttttttaa gtatagggtt gaggcgggtt agttcgggag ggaattggag 3360
gagggggtt tatttggaat tgtatatagt tttagttttt ttcggtcgcg gtttttttcgg 3420
ggttggtgtc gtatttttgg atggtttttt ttacgacgtt tcgtaagttt cgttttagt 3479

<210> 141
<211> 10857
<212> DNA
<213> Artificial Sequence

<220>
<223> chemically treated genomic DNA (Homo sapiens)

<400> 141

ttttagcgtt ttgggggggtt gcggtaggag aatcgttttg ggagtaggag ttgtaggtcg 60
tagtgagtta tgattagttt gggcgattga gcgagatttt gtttttaaaa taaatatata 120
agttcgggcg cggtggttta tgtttgtaat tttagtattt tgggaggtcg aggtgggcgg 180
attacgaggt taagaaatcg agattatttt ggttaatatg gtgaaatttc gtttttatta 240
aaaatataaa aattagttgg gcgtggtggt gcgcgtttgt agttttagtt attcgggagg 300
ttgaggtagg agaatcgttt gaattcggga ggtagaggtt gtagtgagtc gagatcgtgt 360
tattgtattt tagtttggcg atagagtgag atttcgtttt agaataaata aataaaagga 420
tagaaaggcg agtataaata tttttaattt ataatatttt ttcgtattgt taatgtttta 480
gatacgcgtt attattttta gtaaattttt ttaggcgttt gtaggatggg ttaaggaagg 540
cgacgagtat tagttgtttt gttgaggttg tttcgacgtt atatgatttt ttaattatat 600
gatttttaga aatggggtgt ggggcgagag gaagtaggga ggagagtgat ttgagtagaa 660
aagaaatata gtattttagg ttggttttat ttttatattg ataagtagtt aatgggagcg 720
ggtagttttg atttttggtt aatggaaatt gaggtaggcg ggttatcgcg ttggggtttg 780
tagtttgagc gttattcggt tgttgttgtt taaggatcgc ggagtcggac gtaggtagga 840
gagcggtcgc gtagattttt cgtttgtttt tgtttagggg ttcgttaggg ttatgtgagt 900
ttgaggtttt tttggagttt tagtcggaga taatagaaga atcgtttatt gaaatttttt 960
gggggttttg atatattagg gggagtttta tgggaaagag gaagtagtaa ttgtagtgac 1020
gtttcgttag aaggggtttt ttattttttt agtatttttt taaagtaggg attatattat 1080
ttttgattta gttttatttt tgtcggtagg tgttggtttt tttttttttt ttggtggtgg 1140
tgggtggttt tcgcggcggt ttggagtcgg aggggcgcgc gattttgggt tgggagtttc 1200
gagggtttgg gaacgagatt tgagattttg gtttttcgaa ggtagtaggg atttgggagt 1260
ggtgattgaa tttggtttgg ttttttttta tttttttttg ttgcgggtgg gacgagttag 1320
ttttcgtttt ttttagttat tttttttttgt ttatttgtag ttaggttggt tttttttttg 1380
ggaatttttt tttttttttgg tattcggagt tggggggtgt tatttagtgg aagataacgg 1440
agttagggtt ttgaagaggt tgttgttttt tttggttgtt ttggcggtgt agggtggtat 1500
gagagattgc gattcgtttt tttatttttg tttttgtatg cgagtgtttg tatttagtag 1560
aagtatatat tatatttttt taatttaggg taaataggag gggttatatg tataggtaat 1620
ttattaggga gtcgaatatt tttgtgtaga tagatttttt tttttagtaa gttatggtag 1680
cggatagttt gttgagaata tttaggaagt aggcggtgtt agttgtaggt gttttgtttg 1740
ggagttgtgg ggttgaggag agggtttatt gtttaggatt agtgaatttt atttttattt 1800
gtttaggagg tggtttttttg gggatgttga gttaggggag gggtatttga ggaaagttag 1860
gtggagtaga gaggatgtga gtgattgggt gggtgagatt ttttgttttt tttttcgtag 1920

```
tggtatttat atttagattc gtggggtaat tgaggtatag atagagagta attttttagg    1980
tttttatagt tggtaatttt aggattagga tttaagtgcg atttttaggt agtttttgta    2040
ttttgttttt gttgtatttg ttgtattatt tttaggtatt gtttatcgtg ttattagtga    2100
tatgaattta ggtttaatac gttttggggt tattaaagtt tgacgttatt atgatttgat    2160
gtgtgacgtg ttataggtgt tttttggtat ttttacggaa ttggttttag gattttaaaa    2220
tttgtgggtg tttaagtttt tgagataaaa tggtgtaata tttgtatata atttatatat    2280
atttaaatt atttttagat tatttatatt taatataatg gaaatgatat gtcggttggg    2340
cgtggtggtt tatgtttgta attttattat tttgggaggt cgtggtaggt ggattatttg    2400
aggtttggag tttgagatta gtttgattaa tatggtgaaa tttttatttt tattaaaaat    2460
ataaaaatta gttaggtgtg gtagcgtata tttataattt tatttatttg ggaggttgag    2520
gtaggagaat tgtttgaatt tgggaggcgg agttcgtagt aagttgagat cgcgttattg    2580
tattatagtt tgggtgatag agtaggattt tattttaaaa aaaaaagaga aaaagaaaaa    2640
gaaatgttat gtaaatagtt gtgattttga attgtttagg gaataataag aaagaattat    2700
ttgtagatgt ttagtataga tgtatttatc gtaagtttaa ttatattgta taatttagta    2760
acgatgtaat atttttaggg gttttttgt tttgtttttt gagatagaat tttagtttta    2820
ttttgttatt taggttggag tatgttggcg tgatttttgt ttattgtaat ttttattttt    2880
tgggtttaag cgattttttt gttttagttt tttgagtagt tgggattgta ggtgtgcgtt    2940
attacgtatg gttaattttt gtatttttaa tagagatggg gttttattac gttggttagg    3000
ttggttttga atttttgatt ttgggattcg tttatttggg ttttttaaag tgttgggatt    3060
ataggcgtta gttatcgcgt ttaatatatt ttgatttttg gttggatatg gagggttgat    3120
tgtatttaat atttttaagt tttagttttt tttttaaaa taaaggtgtg gttgggtgtg    3180
gtggtttaag tttgtaattt tagtatttag ggaggttgag gtgggtggat tagttgaggt    3240
taggagttta agattagttt gattaatatg gtgaaatttt tttttgtta aaaatataaa    3300
aattagttag gcgtggtggc gagcgtttgt agttttagtt atttgtttga atttgggagg    3360
tagaggttgt agtgagttga gatcgtgtta ttgaattcga gtatgggtaa tagagtaaga    3420
ttgttttaaa aaaaaaaaaa aaaagggggt gagtagacgt ggtggtacgt ttttatagtt    3480
ttagttattt agtaggaggt taaggttgga ggattgtttg attttaggag tttgagttta    3540
gtttgggtaa tatggtaata ttttattttt aaaaataaaa taaaagtaaa ggtattaatt    3600
attattttgg atggttgttg taaagaaata tatataaaat aatggagagt tttgtaattg    3660
gtttttaaga ggtttaatag atattattgt ttttgttttt ttattatgag ttattttttt    3720
ggttatttta ttgaattagt tgggttagtt gagtttggga gaagagttgt ttaggaagtg    3780
agaggttgtt ttttatagag atttaaggtt tagttttttt tggtgattta gatgggtagt    3840
ttagtgggta tacgtggttt ttttttatat gtggttgagt tttattttta gaatagatgg    3900
agaggtaagg gtagggttta gtatgtttga ggaattttag agggttttgg tggtgtgggg    3960
gattttttaga atataggtgt tttaagggtt gatttagttt ttgtgttttt ttttttgggt    4020
gaggagggga tatttatggg tagatggtga tttttgggga aggtagttta gattttattg    4080
gttattatat tttttttttt ataatttttt tatttttgtg gttttttatg ttattatgtg    4140
gtcgtttttc gtaaggtttt agcggggtgt aggtatgaat atagtgttag gtaaggaggt    4200
atttggaggg gaattttggt ttttttggg gggatttttt ttttgtattt tagttttgtt    4260
ttttttttatg gttattgatg tttttttttt attttagagg tggtttatat ttgtatagat    4320
tagatttata aaaattacgt ttttttgatt tttataagtt tgtttagtga ggtttaggta    4380
ttaggttatg tgttggggat ttagatttat atatatacgt atgttagtat ttatgtttat    4440
aggttcgtat atgttggggt aagtgttata tacggggcgt tgtaggaagt tgatttttag    4500
tttttgtaga tttttgtttg tttggatagg gaggtgttga gaaggtttag gtagttttgg    4560
gttaggattt tggtttgggg ttagggtatt gagtgatttt agaattaagg gtggcgtggg    4620
tttaagtagt tgttagacgt tttttggtat tttgtaggta gattatgtgg attttggtga    4680
gttgggtggt tttaatagta gggttggtgg ttggaacgcg gtgtttagat ggttagtttt    4740
gttttgtggt ttgttgtttg gatttcggag gagttagtta tagttgttgt cgtttttttt    4800
tggtgagtgt tttttagttt aggtaagagt tggtagtttg ggtttttttta aagggttatt    4860
ttggattggt tagaggagga cgttaggtat aagtttgtgg tttattattt tttttgtttt    4920
tttaggataa atggtttata atattgagta ggtatttggg tggtttttgt taggttgatg    4980
tttattgttt tgtcggttat ttttgtattt ttatcgtttt agggattttt agttgttgtt    5040
tttttttaga ggtgagcgtg ttattagttt agtggagggg tttaggtttg tatttatgtt    5100
ttttttgtat tttattattt gtagataaaa gggttttgtt aatgtaggtt tttttgtgtt    5160
ttataggtcg tggtatgcgg ggatggttat tattgttgtt tacggggttt ttattgtagt    5220
gtagacgggc gattttgttt ttaaagatta ggtgtagttg gggtgtgggt gtagggtagg    5280
tagacgggta gtatgtggag tttggaattt aggagtttag ttggcggggg tagttttgat    5340
ttttgttttt gtgtttttat ttatgtggta tttgtattaa gtaatagttt tgttgtggat    5400
agaggggtag tattggggat aggagggtgc gggagaaagt gtaagatttt aggtttaggc    5460
gttgtggggg tggggagagg tcgagttggg tcggtttaat attaatttat ggttagtggg    5520
tgtttttttt ttatgttatt ttgttgaggg agggattgga ttgtgaggag ggtgagttag    5580
```

```
gtttgtttag gagattattg agttttagtg ttatttttaa attttagtag ttgggtttgt    5640
aggttttggt gttattagtt ttttgtgtga tgggggagtt atttttttg agtgggttgg    5700
tagtattttg ggttattttg tttataggta ataatttcgt gggtgttatt tagtgttttg    5760
atagttagtt cgaatgttcg gattttttta cgtgttgtgt tatggtcgat ggtttttggg    5820
ggtgttgttt tatgttttag gtataaattt gggggagatg ggggtatgtg gagggaagtg    5880
ggggtagagt tgggggttag gggtaggggg tgaagacgga gttaggatta ttttttttta    5940
ggttttttgt tgtgaagata gggtgtattg ttgttcgtac ggtgtttttt gcgatttggt    6000
ttatattcgt tgtattatat ttacgggtat ttattttttg gtaaagaagt tttttgttta    6060
gaggattaat agggtaggtg aggaggtggg agagtattag gttaggggtt ggggcggggt    6120
tttattgatt ttaagtgtag gaaaaagttt tttttatttt ggttgttttt tacgtttgtt    6180
ttttttttag tggttttgtt tagttcggtt atgtgttcgg acgtacggtt tcggtgtttt    6240
gatggtttta tttgttgtga gttgtttagt gggaagtatg gttgttgttt aatgtttaac    6300
gtgagtgagg ggttggagtt agtttggttg tgtgttttta gttatttggt tttgatacgt    6360
attttatagg ggttttgtgg tatggggttg gttggttgtt tgttgggagt ttggttgatg    6420
tagggtttat gttatttttt agtgggggat tggggtagtg ttagttatta gtttggttgt    6480
tttttgtgtg ttattgagtt tggaagtgat aaagatttat ttttgttttt atttaggtta    6540
tttgttgttt cgattatttg tattgttgtt tttaagatat tgtgtgtgat ttgatttaga    6600
gtaagtgttt ttttaaggag aacgttatta cggatttttt tattaagttg tttgcgtata    6660
taggtattag aggtagggtg tagatatagg ggtggggttt tttttttttt tttttaggtt    6720
tggttttagg attattgtaa ggtggtgtaa gcggtatttt ttatttttaa tatttggttt    6780
tagttgtgga gtcggtaaag ggttgatatt tttgagggtt tttagtgtta tttttgattt    6840
gttttttttg ttttttttat agtgggggat gtgaaatgtg atatggaggt gagttgttta    6900
gatggttata tttgttgtcg tttatagtcg ggggtttggg gttgttgttt ttttatttag    6960
gtatttaggg gtggcgggtg ggtgggttga gtatagtgtg gtaggtagtc gggttttagt    7020
gtttatttgt ttttttttat ttgttttagg ttgtgtgttg tgaggattat atatattgtt    7080
gtttcgcggg gtttacgtgt gatacgtaga agggtatttg tgaatagggg ttttattagg    7140
tgttttggat ggagaaggtt ttagtttatt ttagtttgtt agatttataa gttttgaaga    7200
gagatgtttt ttgtgataat gttagtagtt gttttttttt cgatatttgt tgttaattta    7260
cgtttgggga gtggggttgt tgtttaattt tagaggtata tgggaggga tagtattttg    7320
gtttgggtag gtgggtggtt aagtttttat tgttttttgt ttttcgtata gtttataggt    7380
gatatttagt tttgatagat tcgtttttag ttggaggtgt tgtaagtagg agaggcgggt    7440
tggagtaggt aggggttcgg tattgcgttt tatatagtgg ttatttataa cgtttttttt    7500
tgtttatttt ttaggttgtt tgttgttcgg attattagta ttgttgtttt tagggttata    7560
cgtgtgtagt tgaggggtag tgttagcgag gaagcgagat cgtggttgga ttggagaaga    7620
tgtttgttcg tcgggttttt ttattttatt ttagagatat cggttgtgat tagtatatta    7680
gttgttcggt ggggtagatt tgttgttcga gtttgggtgg gagttgggtt tgttgttagt    7740
tgttttatgt gagtgttttt ttgtttgttt ttggataggg gagttaagtt tagtgagggg    7800
ataggaatat aatgttattt tgtgtttttt ttttcgttag gttgtgtgtt gcgaggatcg    7860
ttagtattgt tgttcggttg gttatatttg taacgtgaag gttcgatttt gcgagaagga    7920
agtggttttt gtttagtttg ttattttttt ggttcgtagt ttttacgtgg gtgtgaagga    7980
cgtggagtgt ggggaaggat atttttgtta tgataattag atttgttgtc gagataatcg    8040
atagggttgg gtttgttgtt tttatcgtta ggttagtgtt aatttttatt ttggggttgg    8100
gtatggttag ggattaggtt ttatttcgtt taattttttc gttttttttt gattatttag    8160
ggcgtttgtt gtgttgatcg gcgttattgt tgttttgttg gttttcgttg cgtagttagg    8220
ggtattaagt gtttgcgtag ggaggtttcg cgttgggacg ttttttttgag ggatttagtt    8280
ttgagatagt tgttgtgagg gatagtattg aagattttgt agttttcggg attttattcg    8340
gagggtgttt tttgtttagg ttttttttagt attttttttt aattaaattt tttttggatt    8400
ttattttgag tttttttatta ttatgggagg tggggtttta atttaaggtt ttttttgtta    8460
gaagggggtt gtggtaaaag ttatattata agttgttatt ttttttttcgt tttagtggat    8520
tttgtggtta ggtgtttttt tttatttata ggggtgtttg tgtgtgtgcg cgtgtgcgtt    8580
ttaataaagt ttgtatattt ttttaatagt gtttgatttg tcgttttgtt tgtttttttt    8640
agggttttag aataggggtt tacgtttatt gttaatattt ttttttttta ttttatagaa    8700
agatatatat agttttaatt ttattagttt tatatttgtt gttgtttata tcggttgtat    8760
tatattattt tttagttttt agagttgttt tagttattag ttttgtgata tcgtagttta    8820
gagatgggtt agttagtaag tagtattttt ttttttttta ggggtttata aagaacgttt    8880
tttttttttt tagtttttat attagtagtt gaggttgggt tatttttttt gttttttttat    8940
aatagagttt tttatgtata gttacgttta tataggtatt gttttttttt agtttttttt    9000
ttcggtattt tttcgtgggg gtttggattt tttttttttt tcggtttgga ggtagatata    9060
gggttttttg taagatacga tttagtatta attacggaat agttttaagg tttttgggtt    9120
tttttttcggt ttggggttgg gagttacgcg cgagggtttt cgcgggtttt cggggcgcgt    9180
attttgggtg cgggttcgcg cgggaggggc ggtgttaggt tttgcgcggg cgttatttga    9240
```

```
cgttgaatat tattagcggt cgttttttc gtcgttgtta cgggtttttt ttgtttttcg    9300
tttcgtcgtt tattttcgtt tttagttcgt ttttcgggtt ggtgagcgag tcgcgtcgta    9360
gttcgttggc gttgttgcgg gagttgtttt cgcggttgcg gtttcgtttt cggggtatcg    9420
tggtcgttcg gttttcgggc gtcgttattt cgtttagtag cggcgttagc gagttgtttt    9480
tcggagagta gaggttcgtg cggttttcgt tgttgttggg tttcgtgttt tcgttgagcg    9540
ttaggcgcgg gggcgcgggc ggcggcggcg gcgcggtagg cggggcgcgt tttcgttttt    9600
ttttcgcggg tcggcggcgc gcgggtcgcg tttcgtggat atttacgttc gagtttaggt    9660
tggcgtcggt gttgcgtgtt tcgtcgttcg tttgtaggtc gatgtaagag tggcgtattt    9720
gcgagttgga gcgttcgtcg agggatacga attaggcgcg cgggtgcggt tttacgttta    9780
gttttagtag tttttttttcg gttagggttt gtagtttttc gttgagttgc gttatggtgg    9840
attcgttgaa tagtataggg atggtgattg agttattgac gggcgtcgcg cgttcggttt    9900
tttagttttc gtcgtcgttt tcgtcgtttt cgtttttcgg gttcgagtgg ttcggtattt    9960
gcgggcgttg ggggtcgggt tggggaaaag cgcgcgtcgg gtcgggtgtc gtgtttttcg   10020
gcggggtcgg ttcgtcgttt acgtcggcgg cgttcgtttc gtcgtcgagg cgtacgtagt   10080
gcgcggggat tattagggtg ggtatgacgt tgcggtagac gttgtttttg aggtggtcga   10140
tggagtcgta gaagatgagt tgtttcgttt ggtttagcga cggcggtcgc gttagttggt   10200
ttatcgattg cgatagtagg aagtcggggg ttttgttttc ggtcgttttt ttgtggttta   10260
ggtagtggtc gaagggcggc ggcggcgagg gcggttcgtg taggaaggtc gtagttttcg   10320
agggttttcg tcggtgtttt tttaggtcgg gttttagttc gtcggggttt tcggtcgagc   10380
gagcgttttt gagttcggcg ttttcgtttt cgcgggtatt cgaaggttcg gcggtcgggc   10440
ggttggtaga gcgcggtttg gtgcggaaga agttatttcg ggaggaggtt aagtttcggg   10500
agttggagaa ggtcgagtgg agggggtttg gaggcggagt ttcggggttt ttcgacgggg   10560
cgggttttag gggtttttta tagatgaggt ggagttggga tatcgaggtg gtttggtttc   10620
gtttgttatc gttagagggt ttcgagagtt gtagtttgcg gtgttgttgt ttcggtttta   10680
ggtagcgttt tttggaaggg agggagtaga aggggtcgtt taggaaaggg tgagggttaa   10740
agtaggtttc gaattttttt agatgtaaga taagagacga atatagtgga agaggtgggg   10800
agaatgtttg gggcgtttag gagagatggc ggggagtcgt cgtgttcgtt tggcgat      10857
```

<210> 142
<211> 10857
<212> DNA
<213> Artificial Sequence

<220>
<223> chemically treated genomic DNA (Homo sapiens)

<400> 142

```
atcgttaaac ggatacggcg gtttttcgtt attttttttg agcgttttag gtattttttt      60
tattttttttt attatattcg ttttttgttt tgtatttaag ggggttcggg gtttattttg     120
gtttttattt ttttttgagc ggtttttttt gttttttttt ttttaggagg cgttgtttga     180
agtcgaggta atagtatcgt aagttgtagt tttcggggtt ttttgacggt aataaacgag     240
attaggttat ttcgatgttt tagttttatt ttatttgtgg gggatttttg gaattcgttt     300
cgtcggggga tttcgaggtt tcgtttttag gtttttttta ttcggttttt tttagttttc     360
gggatttggt tttttttcgg gatgattttt ttcgtattaa gtcgcgtttt gttagtcgtt     420
cggtcgtcga gttttcgggt gttcgcgggg gcgagagcgt cgggtttaag ggcgttcgtt     480
cggtcgaggg tttcggcggg ttggagttcg gtttagagga gtatcggcgg gggttttcgg     540
gggttgcggt tttttgtac gagtcgtttt cgtcgtcgtc gtttttcgat tattatttgg     600
gttataaggg ggcggtcgag ggtaagattt tcgatttttt gttgtcgtag tcggtggatt     660
agttggcgcg gtcgtcgtcg ttgggttagg cggggtagtt tattttttgc ggttttatcg     720
attattttaa ggataacgtt tatcgtaacg ttatgtttat tttggtgatt ttcgcgtatt     780
acgtgcgttt cggcggcgag gcgggcgtcg tcggcgtggg cgacgagtcg gtttcgtcgg     840
agggtacggt attcggttcg gcgcgcgttt ttttttagtt cgatttttag cgttcgtaga     900
tgtcgggtta ttcgggttcg gggggcgagg gcggcggggg cggcggcgag ggttgggggg     960
tcgggcgcgc ggcgttcgtt agtggtttag ttattatttt tgtgttattt aacgagttta    1020
ttatggcgta gtttaacggg gagttgtagg ttttgatcga gaagaagttg ttggaattgg    1080
gcgtgaagtc gtattcgcgc gtttggttcg tgtttttcga cgggcgtttt aattcgtaag    1140
tgcgttattt ttatatcgat ttgtaggcgg gcggcggggt acgtagtatc gacgttagtt    1200
tggattcggg cgtagatgtt tacgaggcgc ggttcgcgcg tcgtcggttc gcgagggagg    1260
agcgggagcg cgtttcgttt gtcgcgtcgt cgtcgtcgtt cgcgttttcg cgtttggcgt    1320
ttagcgagga tacggagttt agtagtagcg agagtcgtac gggtttttgt ttttcggagg    1380
```

```
ataattcgtt gacgtcgttg ttggacgagg tggcggcgtt cgagggtcgg gcggttacgg    1440
tatttcgggg gcggggtcgt agtcgcgggg atagttttcg tagtagcgtt agcgagttgc    1500
ggcgcgattc gtttattagt tcggaggacg agttgggggc ggaggtgggc gacgaggcgg    1560
gagataagaa gagttcgtgg tagcggcggg aggagcggtc gttgatggtg tttaacgtta    1620
agtagcgttc gcgtagggtt tggtatcgtt tttttcgcgc gggttcgtat ttagggtgcg    1680
cgtttcgggg gttcgcgggg gttttcgcgc gtagttttta gttttaggtc ggagaggggt    1740
ttaggggttt tggagttgtt tcgtggttgg tgttgggtcg tgttttgtaa gggattttgt    1800
gtttgttttt aagtcgggga ggaggggggg tttagatttt tacggggagg tgtcggggag    1860
gagggttggg gggaagtagt gtttgtgtag acgtggttgt atatagaaag ttttattgtg    1920
ggaaagtagg aggggtgatt tagttttagt tgttagtgtg agggttggga agggaggggg    1980
cgttttttgt ggatttttgg gaggggaggg ggtgttgttt gttgattagt ttatttttgg    2040
gttacgatgt tatagggttg gtggttggga tagttttggg gattaggggg taatgtgata    2100
tagtcgatgt gggtagtagt aaatataaaa ttggtgaggt taaggttgtg tatgtttttt    2160
tgtggggtag gggaggggag tgttggtagt ggacgtgaat ttttgttttg gggttttggg    2220
gagggtaggt agggcggtaa attagatatt gttaagaaag tgtataaatt ttattgaaac    2280
gtatacgcgt atatatataa atatttttgt ggatagggaa aagtatttgg ttatagggtt    2340
tattgaaacg gggaggggat ggtagtttgt aatgtggttt ttgttataat ttttttttga    2400
tagggaaggt tttagattga ggttttattt tttatggtga tggggagttt agaatggggt    2460
ttagggagaa tttggttagg gggaggtgtt agggaggttt gagtagaggg tatttttcga    2520
gtggggtttc gagggttgta gagtttttag tattgttttt tatagtagtt gttttaaggt    2580
tgggtttttt aaaggggcgt tttagcgcgg ggttttttttg cgtaaatatt tggtattttt    2640
ggttgcgtag cggaagttag taggatagta gtggcgtcga ttagtataat agacgttttg    2700
gatggttaga ggggggcgag agggttggac gaggtgggat ttggtttttg gttatattta    2760
gttttaggat gggggttggt attgatttgg cggtagggat agtaggttta gttttgtcgg    2820
ttgtttcggt agtaggtttg gttattatgg tagaagtgtt tttttttata ttttacgttt    2880
tttatattta cgtgagggtt acgggttagg aaggtggtag gttgggtaga gattattttt    2940
ttttcgtagg atcgagtttt tacgttgtag gtgtagttag tcgggtagta gtgttggcga    3000
ttttcgtagt atatagtttg gcggggaagg gagtatagaa tggtattatg tttttgtttt    3060
tttattgggt ttagtttttt tatttagggg taggtaggga ggtatttata tggggtaatt    3120
ggtagtaggt ttagtttttta tttaggttcg ggtagtaggt ttgttttatc gggtagttgg    3180
tgtgttggtt atagtcgatg tttttggggt gggataagga agttcggcgg gtaggtattt    3240
tttttagttt agttacgatt tcgtttttttc gttgatattg tttttttagtt atatacgtgt    3300
agttttgggg gtagtagtgt tggtggttcg agtagtagat agtttggggg gtgggtagga    3360
aagggcgttg taggtagtta ttatgtgggg cgtagtgtcg agtttttatt tattttagtt    3420
cgtttttttt gtttatagta tttttagttg gggacgaatt tgttagagtt gggtattatt    3480
tatgggttat gcggagggta gaaagtaata ggagtttggt tatttatttg tttaggttaa    3540
gatgttgttt tttttttatat atttttggga ttggatagta gttttatttt ttagacgtga    3600
gttggtagta ggtatcggag gagggatagt tgttgatatt attatagggg atattttttt    3660
ttaaggtttg tgggtttggt aggttgaggt gagttggggt tttttttattt tagggtattt    3720

ggtggggttt ttgtttatag gtattttttt gcgtgttata cgtaaatttc gcgggatagt    3780
agtgtatgtg gtttttatag tatatagttt agggtagatg aagaagggta ggtgggtatt    3840
ggggttcggt tgtttgttat attgtgttta gtttatttat tcgttatttt tgggtatttg    3900
ggtaaaaggg tagtagtttt aggttttcga ttgtagacgg tagtaggtat agttatttgg    3960
gtagtttatt tttatgttat attttatatt ttttattgtg agggaagtag agaggatagg    4020
ttagaagtgg tattggggat ttttaggggt attaattttt tgtcggtttt atagttggaa    4080
ttaggtgttg aagatggagg gtatcgttta tattattttg tagtgatttt aaggttaggt    4140
ttaaaaggga ggaaaggggg ttttattttt gtatttgtat tttgtttttg gtatttgtgt    4200
gcgtaggtag tttagtgagg aggttcgtgg tagcgttttt tttggagagg tatttatttt    4260
ggattaggtt atatatagtg ttttgggggt agtagtgtag gtgatcggag tagtaggtgg    4320
tttgagtggg gatagggggtg ggtttttgtt atttttaggt ttagtagtat atagggagta    4380
gttaggttga tggttggtat tgttttaatt ttttattagg gggtagtatg aattttgtat    4440
tagttaggtt tttagtaagt agttagttag ttttatgtta tagagttttt gtaaggtgcg    4500
tgttaggggtt aggtggttgg gggtatatag ttaagttggt tttagttttt tatttacgtt    4560
gggtattggg tagtagttat attttttatt gggtagttta tagtaggtag aattattagg    4620
gtatcgggat cgtgcgttcg gatatatgat cgagttggat aaggttattg gaagaggagt    4680
aaacgtgagg ggtagttagg atggaggaaa ttttttttta tatttggagt taatgaggtt    4740
tcgttttagt ttttggtttg atgttttttt attttttttat ttgttttgtt agttttttgg    4800
gtagggagtt tttttgttag gggtgggtg ttcgtgggtg tgatgtagcg ggtgtgaatt    4860
aggtcgtaga aggtatcgtg cggatagtag tgtattttgt ttttatagta ggaagtttga    4920
gaaaaaatgg ttttgatttc gttttatttt tttgtttttg gtttttaatt ttgtttttat    4980
```

```
ttttttttat atatttttat ttttttaga tttgtatttg gggtatgggg tagtattttt 5040
aggagttatc gattataata tagtacgtgg agaagttcgg gtattcgaat tgattattag 5100
ggtattggat ggtatttacg gagttgttat ttgtggataa gatgatttag gatattatta 5160
gtttatttag ggaaggtgat tttttatta tataaggagt tggtggtatt agggtttgta 5220
agtttagtta ttggggtttg agggtgatat taaggtttag tgatttttta ggtaggttta 5280
atttatttt tttataattt agttttttt ttagtaagat ggtatgggga aggggtattt 5340
attgattatg ggttggtatt agatcggttt agttcgattt ttttttattt ttataacgtt 5400
tggatttgga gttttgtatt ttttttcgta ttttttattt tttagtgttg tttttttgtt 5460
tatagtaggg ttgttgttta gtatagatgt tatatgaatg agggtataag ggtaggaatt 5520
agggttgttt tcgttagttg ggtttttggg ttttagattt tatatgttgt tcgtttgttt 5580
gttttgtatt tatattttag ttgtatttga tttttggaag taggatcgtt cgtttgtatt 5640
gtagtggaag tttcgtgggt agtagtgatg gttattttcg tatgttacgg tttgtggaat 5700
atagagaaat ttgtattggt agggtttttt tatttgtagg tggtagagtg taggaaaagt 5760
ataaatgtag atttaagttt ttttattggg ttgatggtac gtttattttt gggaaggggt 5820
agtaattgga agtttttgag acggtaaaga tgtaggagtg gtcggtagag tagtgggtat 5880
taatttggta ggggttattt agatgtttgt ttagtgttgt gggttatttg ttttagaaag 5940
atagggaaaa tgataaatta tagatttgtg tttggcgttt ttttttggtt aatttaagat 6000
gatttttggg gaaaatttag gttgttagtt tttgtttagg ttgaggggta tttattagaa 6060
ggggacggta gtagttgtag ttggtttttt cggggtttag gtagtaggtt atagggtaga 6120
attgattatt tgggtatcgc gttttagtta ttagttttgt tgttaaggtt atttagttta 6180
ttagggttta tatggtttgt ttgtaaagta ttaaggaacg tttggtaatt gtttaagttt 6240
acgttatttt tgattttagg gttatttagt attttagttt taggttaagg ttttggttta 6300
ggattgtttg agttttttta atattttttt gtttaggtag gtagaaattt gtaggggttg 6360
agagttagtt ttttatagcg tttcgtgtgt gatatttgtt ttagtatgtg cggatttgta 6420
agtatgaatg ttgatatgcg tatatgtgtg ggtttgagtt tttagtatat ggtttaatgt 6480
ttgggtttta ttgggtaggt ttatgagagt taggaagacg tgatttttgt gggtttgatt 6540
tgtgtagatg tgggttattt ttggggtgag gggaaggtat tagtagttat gggagaggat 6600
agggttaggg tgtagggagg gagttttttt aggaaaagtt agggtttttt tttagatgtt 6660
tttttgtttg atattatgtt tatatttgta tttcgttaag gttttgcggg aagcggttat 6720
atgatgatat gggaaattat aggggtgaga aagttgtgaa aaaggaaata tggtggttag 6780
tggagtttgg gttgtttttt ttagaggtta ttatttgttt atgaatgttt ttttttttatt 6840
tagagaaaag gatatagaag ttgggttagt ttttgagata tttgtgtttt gagggttttt 6900
tatattatta gggttttttg agatttttta agtatgttaa attttgtttt tgttttttta 6960
tttattttgg aagtgaaatt tagttatatg tggagagaga ttacgtgtgt ttattgggtt 7020
gtttatttga gttattagga ggaattgagt tttgagtttt ttgtggagagt agtttttttat 7080
tttttaaata attttttttt taggtttagt tagtttagtt agtttagtgg ggtggttaga 7140
gaggtaattt ataatgaaga agtaaaaaata gtaatgtttg ttgagttttt tgggagttag 7200
ttataagatt ttttattatt ttatatatat ttttttgtaa taattattta aagtagtaat 7260
taatattttt atttttattt tatttttaga gatgaggtat tgttatgttg tttaggttgg 7320
atttagattt ttgggattaa gtaatttttt aattttggtt ttttattaag tagttgggat 7380
tgtgggagcg tgttattacg tttgtttatt tttttttttt tttttttttt gagatagttt 7440
tgttttgttg tttatgttcg agtttagtgg tacgatttta gtttattgta atttttgttt 7500
tttaagttta agtaagtagt tgggattata ggcgttcgtt attacgtttg gttaattttt 7560
gtatttttag tagagagggg gttttattat attggttagg ttggttttga atttttgatt 7620
ttagttgatt tatttatttt agttttttta agtgttggga ttataggttt gaattattat 7680
atttagttat atttttattt taaaggagga aattgaagtt tagagatgtt aagtatagtt 7740
agtttttat atttaattag ggattaaaat atattgggcg cggtggttaa cgtttgtaat 7800
tttagtattt tgggaggttt aggtgggcgg attttaaggt taggagttta agattagttt 7860
gattaacgtg gtaaaatttt attttttatta aaaatataaa aattagttat gcgtggtagc 7920
gtatatttgt aattttagtt atttaagagg ttgaggtagg agaatcgttt gagtttagga 7980
ggtggaggtt gtagtgagta gagattacgt taatatattt tagtttgggt gatagagtga 8040
gattgagatt ttgttttaaa aaataaaata aaaaaatttt tgaaaatgtt atatcgttgt 8100
tgagttatat aatgtagtta ggtttacgat gggtgtattt atattgaata tttatagata 8160
gtttttttttt attatttttt aaataattta ggattataat tatttatatg gtattttttt 8220
tttttttttt tttttttttt tgagatggag ttttgttttg ttatttaggt tgtagtatag 8280
tggcgcgatt ttagtttatt gcgaatttcg ttttttaggt ttaagtaatt tttttgtttt 8340
agtttttaa gtaggtggga ttataggtgt gcgttattat atttggttaa tttttgtatt 8400
tttagtagag atgggggttt tattatgttg gttaggttgg ttttaaattt tagattttag 8460
gtgatttatt tgttacggtt ttttaaagtg gtgggattat aggtatgagt tattacgttt 8520
agtcgatatg ttattttttat tgtattaggt ataagtaatt tagaaatgat ttaaagtatg 8580
tataggttat atgtaaatat tatattattt tattttaggg gtttgagtat ttatagattt 8640
```

```
tggggttttg gaattagttt cgtgaagata ttaagggata tttataatac gttatatatt    8700
aggttatggt gacgttaggt tttgatggtt ttagagcgta ttggatttgg gtttatatta    8760
ttagtggtac gatgggtagt gtttgggaat ggtgtaatag gtataataga aatagggtat    8820
agggattgtt tgaaaatcgt atttgggttt taattttaga attgttaatt gtgagggttt    8880
gagaagttgt tttttgtttg tgttttagtt attttacgag tttaggtgtg gatattattg    8940
cggggggagg ggtaggaaat tttatttatt tagttattta tatttttttt gttttatttg    9000
gtttttttta agtgtttttt ttttaattta gtatttttaa gaggttattt tttggataga    9060
taaggatgaa gtttattggt tttggatagt ggattttttt tttagtttta tagtttttag    9120
gtaaagtatt tgtagttggt atcgtttgtt ttttgggtgt ttttagtagg ttgttcgttg    9180
ttatggtttg ttgggaaggg gagtttgttt gtataggagt gttcggtttt ttggtgaatt    9240
atttgtgtat gtggtttttt ttgtttattt taaattgagg gagtatagtg tatgttttta    9300
ttgaatataa gtattcgtat atagaaatag ggatgaggag gcgagtcgta gtttttttatg    9360
ttattttata tcgttaaaat agttagaggg gatagtagtt tttttaagat tttagtttcg    9420
ttattttttta ttaggtggta ttttttaatt tcgagtgtta aggggagagg aaatttttaa    9480
aaggggagtt aatttagttg taaatgagta ggaaaaagtg gttgggagag gcggaagtta    9540
gttcgtttta ttcgtaataa gaggaagtaa ggaggagtta gattaggttt agttattatt    9600
tttaagtttt tattattttc gagaagttaa ggttttaggt ttcgttttta ggttttcgga    9660
gtttttagtt tagggtcgcg cgttttttcg gttttaggtc gtcgcgggaa ttatttatta    9720
ttattaggag aggggaagaa gttagtattt atcgataggg gtggagttgg gttaagaatg    9780
gtgtggtttt tgttttgggg gaatgttggg gaggtagaaa gtttttttta acggggcgtt    9840
attgtaatta ttgttttttt tttttataa aatttttttt agtgtattag aatttttaag    9900
gagttttagt aagcggtttt tttgttgttt tcggttgaga ttttagggga attttaagtt    9960
tatatggttt tggcgggttt ttgggtagga gtaggcgaga ggtttgcgcg gtcgtttttt   10020
tatttgcgtt cgatttcgcg gtttttgggt agtagtaatc gggtagcgtt tagattatag   10080
atttagcgc gatgattcgt ttattttagt ttttattggt tagggattag ggttattcgt   10140
ttttattggt tatttattaa tatagaggtg gggttagttt ggaatgttgt gttttttttt   10200
tatttaaatt atttttttttt ttgttttttt tcgttttata ttttattttt agggattatg   10260
tgattggaga attatgtgac gtcgggatag ttttagtagg gtagttggtg ttcgtcgttt   10320
tttttaattt attttgtagg cgtttggggg agtttgttag agatgatagc gcgtgtttgg   10380
ggtattgata gtgcgaggga gtgttatgaa ttgggaatat ttgtgttcgt tttttattt   10440
ttttgtttgt ttgttttgag acggagtttt attttgtcgt taggttggag tgtagtggta   10500
cgatttcggt ttattgtaat ttttgttttt cgggtttaag cgattttttt gttttagttt   10560
ttcgagtagt tgggattata ggcgcgtatt attacgttta gttaattttt gtatttttag   10620
tagagacggg gttttattat gttggttagg atggtttcga ttttttgatt tcgtgattcg   10680
tttatttcgg ttttttaaag tgttaagatt ataggtatga gttatcgcgt tcggatttgt   10740
gtgtttgttt tagagatagg gtttcgttta gtcgtttaag ttgattatag tttattgcgg   10800
tttgtaattt ttgtttttaa agcgattttt ttatcgtagt tttttaaggc gttggga      10857
```

<210> 143
<211> 5465
<212> DNA
<213> Artificial Sequence

<220>
<223> chemically treated genomic DNA (Homo sapiens)

<400> 143

```
tttcggattt ggtaaggtgc ggtggtttat gcgtataatt ttagtatttt gggaagttta      60
ggtgggtgga ttatatgagg ttaggagttt tagattagtt tggttaatat ggtaaaattt     120

tatttttatt aaaaatataa aaattagtcg ggtatggtgg tgtatatttg tagttttagt     180
tatttgggag gttgaggtag gggaatagtt tgaattcggg acgtggaggt tgtagtgcgt     240
taagatcgtg ttatttcgtt ttaatttgcg taatagagtg agattgttta aagaaaaaaa     300
aaaaatttta gatttaatgt tagttgtttg tgttagtttg gggatttttt atttataggg     360
tgtagtttgg ttaagaggaa ggagttatat aatatggttg atggaatttt taatatttaa     420
gtttaggggt atatgtgtag gatgtgtagg tttgttatat aggtaaatgt gtattatggt     480
ggtttattgt atagattatt ttattattta ggtattaagt ttagtattcg ttagttattg     540
tttttgatgt tttttttttt tttatttttt attttttgat acgttttagt ttgtgttgtt     600
ttttttatgt gtttatgttt ttttaggttg atgtggtttt gagtgatttt tttttgtttt     660
tttttttaa aaataagatt taaaaggtta tgttttttat gggttggagg tgttgtttcg     720
```

atttgttaag tatagtttag gatttagagg ttgttgtggt gaattattgt aggtatttgg 780
aagttattaa ggtgtttttt tttcgtattt tagttttaag tgagggaaat aagtttatgg 840
ttaagggaga attatttatt aatttgaatt tattaaaata tataaatgtg tttattagaa 900
atggttggtt ggagaagttt agtattaaat tatttcgttt ttgaaattta ttagttttat 960
gaaatagttc gttagagaga gatttgtaat ttttgaattc gttgattatt aatttgtata 1020
ggtgtttcgt gtttcgagat ttgagaaaat gataaaatac gtttattttt cggtcgggta 1080
tagtgattta cgtttgtaat tttaatattt tgagaggtta agttgggtag attatttgag 1140
gttaggagtt tgagattagt ttggttaata tggtaaaatt ttcgttttta ttaaaaatat 1200
aaaaattagt tgagggtggt ggtgggcgtt tttaatttta gttatttcgg aggttgagtt 1260
ataagaatcg cgggaagcgg aggttgtagt gagtcgagat ggggttatag tattttagtt 1320
tgggcgatag agtgggattt agttttaaaa aaaaaaaaaa agtatttttt aatttttcgt 1380
tagtttttgt ttttttttta tttcgttttt tttaggtttt tgaaaagtat ttttttttaat 1440
tttttttgtg tatacgggga aaatacgtgt ttatttttat tatggcgttg aaatttattt 1500
ttttttttaa atcgttttttg tagatcgttt tagtataaaa tgtggtaaga ggttaaaggg 1560
agagatatag attagttttt tggttttcgt tttttaattt taagttatat ttgtgaaatt 1620
tatgggtttt tttatagttt ttaaaaatta agggcgtttt ttgttttttt tttagatgtt 1680
ttttttttat cgtcggtagc gagtgggaga tagtttagcg cggggtaggg gagtattggg 1740
ttcggagatg gaaggtagcg ttaaaagcgt cgttggaaaa tttttgagcg ttaatcgttg 1800
tttgtgtgag tttttaaatt tataaatttt taatatttgt agtttttggg ttttttagga 1860
tttttatcga ttttggcggt agagagggta ggtttgagat gtagtgattt gagggtatat 1920
ggttaatttt tgttatttta agattatatt ggggaattag attgattttt ttaattttga 1980
attcgtttcg gtttcgggcg gtttaaaggg ttttttttgt cgtattttcg ttaaaattaa 2040
atcgtttggt ataagtcggt aagtaattat tttgttggga gagggaagga agagtaggcg 2100
tagttttaga ttaatttttt tgtattgttt tttttagacg gttaagttag ttgcgtttta 2160
tcgaaaaggg cgtatcgttt acgttcgaaa cgtagtcgtt gggggtcgag aaattatttt 2220
tatttggttc gagggttagg gacgtaggag cgtagtagcg tggaggggtt tcgcgttggt 2280
tcggcgttgt tcgcggtttt gttttcgttt taagggtacg gcgtcggtac gaggatatcg 2340
acgttgtggc gtaattgtcg tttttcgtag taatttcgga gttcggtttt cggtcgtttt 2400
tcggttttgc gtaggttgtt ttttttcgac gcggagtttt atttcgttat tcgtcgttta 2460
gacgatttta taaataagtt ttcgaagtgc ggaggtagga ggcggggcgt agcgcggggg 2520
taggaggcgg gttagggtta gggtagaggt tgcggtcgcg cgtttttatt ggtcgggacg 2580
tagtgagtcg ttcggagttc ggcgcgggcg gggtttgtcg gcgcgtgttc gtttatatat 2640
tcgcgtcggt gttcgttttt cgtttttcgc gttcgtttcg tgttcgtttt aagtcggtcg 2700
acggagtttt taaagtgggt tgtcggtcgc gggagtttta tattcgcgag cggatcgtta 2760
tacgggttcg gtgttcgttg cgttttcgtt ttagcgtttt tgaggcggtc gtataatttt 2820
cggtagtgtt ttgagattgt atggttagtt tagttcggtt ttcgattttt ttcgattttt 2880
agtattcgag ttattttttt tttttttga aaatttagaa aagtgatttt ttttttaggg 2940
aaaaaggaat ttgggttttt ttttttcgt ttttttttcg ggtttgatag tttttattta 3000
ttttttttc ggatttcgtt ttcgcgcgta ggtttttttt agttattttt ttttattttc 3060
gttttcgtat ttagttcgtc gcgcgttatt ttttatttga ttgcgcgggg cgttcgggat 3120
ttgcgcgtat ttcggatttt tattattcgt tcgggtcgcg gggagcggac gagggttata 3180
gtttttttatt cgttagggag tttaggtgtt cggcgtttga acgttttaaa gggttatagc 3240
gataatgata gttgataagg agaagaaaag gtaagcgggc gttcgggtcg attagggggt 3300
cggttcgagg ttagggtcgg gttgcgcggg gtaggcgcga tcgagagtgg ttgggagaag 3360
agtgttgaga ggttttcgga gttcgaggtt cggtttggag ggttgtgagg gggagagtat 3420
gtgttcggtt tgggggcgcg gattagtagt tttgtagtgg agatttttgc ggttcggagg 3480
agtcggggat ttgcgcgtac ggcgaggtta ggaggtcgag ggagatggtt tggaggtcgg 3540
aggtgttttc gcggtgtttt tgaaaatttt ttagtcgttt cgtagtagat tatcgtggtt 3600
atcggcgttt ggaaatgttt gtgggtgtat gttttcgatt ttttttggtt ttttattttg 3660
gggtagtatt tacgttttta tttttttttg gattcgcgga gtttttttaa atgcgttttt 3720
gtgttcgcgt cgcggtttaa gtgggtaaag gggggcggga ggcggagagc ggtttaggga 3780
tacgatttta tcgaggagtt aggatttttt agagcgtttc gttgtcgggt tttagatttt 3840
atcgaaggtt ggagaattta ttttttcgtg tcgtattttg attttgggga gagtaggtag 3900
tgagtgattt ggattgtttt gcgggggtag atggttcggt gtgtaggaag cggacggtaa 3960
tattggatgt ttttggtagg ttcgggtgtt tgttttcgaa gaggatagag aagggtagtt 4020
acgatttgtt cgtttgtttt cgcgagtttt tcggtattgg gtgagaggta attttggtta 4080
ttttttgttg ttttttcgtt ttttttcggt cgtttttagt ttagtcgggt tcggcgttat 4140
tcgagcgagg gttcgagttt tttgcgcggt cgcgtagaag taggtagggt tttaattttt 4200
gtaaatgtgt gcggttcgtc gtttgttttt tttttttttt tgtttttatt cgtgcggttt 4260
tagtggtttg gagtttttag tttcgcgttt ggtcgcggtt tggcgaggtt atgttgcggg 4320
aagttggaat ttaacgcgcg gtcggttgaa tcgtttgagt cgcgggaaat tagcggcgga 4380

```
gcggcggtat agaggtgcga ggatgaggag gatcgattcg ttaagggagg gaggtgattg    4440
gtcgggaggg tttattgtcg aggttgattt gtttttgttt taatttttat tacgggattg    4500
gagttagaaa tcgtgtattt ttcggtcgaa agtagcggtt tttatttcgg ggtatcgata    4560
aggatttgat aaacgggagc gaatcgcgtt tgttttggtt cggcgttcgg gtttttttatt    4620
gcgggatcgt taggggaggg gattgggttt tgttttttttt ttttggtcgt agtttttttt    4680
ttttcgttat cgtcgcgttt ttagatttaa gataagtgag ggagttttga gattgtagtc    4740
ggtatatttt tagggttttc ggagtagttt tggggtttta tgtttttttga aggtagtaga    4800
gtaaggaaaa gatttttaag attttagtgc gttttgtaaa aattaattat aatgttaata    4860
atgattgaat tatcgaaaag taaagttata gatagcgttt gtagacgttt tcgggttcgg    4920
agtcggggag ggaatttgtg tattttataa ttgcgatgtt tatcgggaat gtggtaatcg    4980
tcgaggtggg gtttgatagt aattttgata aatgtgtgaa gtgttagttt tttttgcgtt    5040
ttttagcgtc gggttttgtg tagttttgta tcgtatatat cgagaaattt gggagtaggg    5100
gaaaaatgat ttcgatatat ttagttatag tatttggttc gttagcggat tgttggaaag    5160
agagaagaag cgggaatggg agcgggaaga acgttgcgta attagatttt taattattgg    5220
cgagagcggt cgttttaaga attattgtgg ggattgtttg cgaaacgttt tggaggtcgt    5280
tgtgtgagtt gcggtttcgg gttggtggtt acgcgagggt gttcgttttt ttcgatttgt    5340
ttggattttt gtgttgtaga atggtagggt aaatagcgtg ttttttatta tttagagttt    5400
tttaaggggt tgtttaattt ttattgggtt aattagtttg agatttattt atttttcgtg    5460
tttgg                                                                5465
```

<210> 144
<211> 5465
<212> DNA
<213> Artificial Sequence

<220>
<223> chemically treated genomic DNA (Homo sapiens)

<400> 144

```
ttaggtacga aaagtaaatg agttttaaat tgattagttt aataggaatt ggataatttt      60
ttaaggggtt ttagatggtg aaggatacgt tgtttgtttt gttattttgt agtataaaaa     120
tttaggtagg tcgaagaggg cggatatttt cgcgtggtta ttagttcgga gtcgtagttt     180
atatagcggt ttttaaggcg tttcgtaggt agtttttata gtggttttta aagcggtcgt     240
tttcgttaat aattgggagt ttaattacgt aacgtttttt tcgtttttat tttcgttttt     300
tttttttttt taatagttcg ttaacgaatt aggtattgta gttgggtgtg tcgaaattat     360
ttttttttg tttttaagtt tttcggtata tacggtataa aattgtataa aattcggcgt     420
taaggagcgt aggaggagtt gatattttat atatttgtta agattattgt taggttttat     480
ttcggcgatt gttatatttt cgataggtat cgtagttgta aaatatataa gttttttttt     540
cggtttcgga ttcgaaagcg tttgtaggcg ttgtttataa ttttgttttt cgatggttta     600
gttattatta gtattatgat tggtttttat aaaacgtatt ggaattttaa gagttttttt     660
tttgttttgt tgtttttaaa agatatggaa ttttaggatt gtttcgaggg ttttgggaat     720
gtgtcggttg tagttttagg gttttttttat ttgttttggg tttgagggcg cgacggtgac     780
gagaaagggg ggattacggt taggaaaggg gagtagggtt taatttttttt ttttagcgat     840
ttcgtagtga agggttcggg cgtcgagtta ggataggcgc gattcgtttt cgtttattaa     900
atttttatcg gtgtttcgag gtgggaatcg ttgttttcga tcggaggata tacggttttt     960
ggttttagtt tcgtagtgga agttggaata agagtaaatt aatttcggta gtgagttttt    1020
tcggttagtt atttttttttt tttagcgagt cggttttttt tattttcgta tttttatatc    1080
gtcgtttcgt cgttggtttt tcgcggttta ggcggtttag tcggtcgcgc gttggatttt    1140
agtttttcgt agtatagttt cgttaagtcg cggttaagcg cggggttgga agttttaggt    1200
tattggggtc gtacgggtgg agataggagg agaaagggga taggcggcga gtcgtatata    1260
tttgtagaag ttgaggtttt gtttgttttt gcgcggtcgc gtagagggtt cgaattttcg    1320
ttcgggtagc gtcgggttcg gttggattgg gagcggtcgg gagagggcga gagggtagta    1380
agaaatggtt agagttgttt tttatttagt gtcgagaggt tcgcgagggt aggcgggtag    1440
atcgtggttg tttttttttg ttttttttcgg agatagatat tcggatttgt tagggatatt    1500
tagtgttgtc gttcgttttt tatatatcga attatttgtt ttcgtagggt aatttaggtt    1560
atttattatt tatttttttt agagttaagg tgcggtacga ggaagtgggt tttttaattt    1620
tcggtggggt ttggagttcg gtagcggggc gttttgaagg gttttggttt ttcggtagga    1680
tcgtgttttt gagtcgtttt tcgtttttcg ttttttttttg tttatttggg tcgcggcgcg    1740
ggtataggag cgtatttgga aggatttcgc gaatttagag aaaaatagag acgtggatgt    1800
tgttttaaaa tgagagatta gagaaagtcg aggatatgta tttataaata tttttaagcg    1860
```

tcggtggtta cggtaatttg ttgcggggcg gttgggagat ttttaaaggt atcgcggaag 1920
tattttcgat ttttaggtta tttttttcgg ttttttggtt tcgtcgtgcg cgtaaatttt 1980
cgattttttc gagtcgtaga agtttttatt gtaaagttgt tgattcgcgt ttttaaatcg 2040
ggtatatgtt ttttttttta tagtttttta aatcgagttt cgagtttcga agatttttta 2100
gtattttttt tttaattatt ttcggtcgcg tttgtttcgc gtagttcggt tttggtttcg 2160
gatcggtttt ttgatcggtt cggacgttcg tttatttttt tttttttttg ttagttgtta 2220
ttgtcgttgt ggttttttga gacgtttaga cgtcgagtat ttgggttttt tggcgggtgg 2280
ggggttgtgg ttttcgttcg tttttcgcgg ttcgggcggg tggtgaaggt tcgaggtgcg 2340
cgtaggtttc gagcgtttcg cgtagttagg tggaaggtgg cgcgcggcgg gttaggtgcg 2400
ggggcggggg tggagaaagg tgattgggag gaatttgcgc gcggaggcgg ggttcgggga 2460
aggagtgggt ggaggttgtt agattcgaaa agaggacgga gagaagggaa tttaagtttt 2520
tttttttttg gaaaaggagt tatttttttg agtttttaaa gaaaaaaaaa agtggttcga 2580
atgttgggag tcggaaggag tcggaggtcg ggttgagttg attatatagt tttaggatat 2640
tgtcgaggat tgtacggtcg ttttaggagc gttggggcgg aagcgtagcg ggtatcggat 2700
tcgtgtggcg gttcgttcgc gagtgtaaag ttttcgcggt cggtagttta ttttaaaaat 2760
ttcgtcggtc ggtttggggc gggtacgggg cgggcgcgga gggcgggggg cgggtatcgg 2820
cgcgggtgtg tgggcgggta cgcgtcggta ggtttcgttc gcgtcgagtt tcgggcggtt 2880
tattgcgttt cggttaatgg ggacgcgcgg tcgtagtttt tgttttgatt ttggttcgtt 2940
ttttgttttc gcgttgcgtt tcgttttttg ttttcgtatt tcgaggattt gtttatgagg 3000
tcgtttgggc ggcgggtagc ggggtgggat ttcgcgtcgg ggagaggtag tttgcgtagg 3060
gtcgaggggc ggtcgggagt cgggtttcgg gattgttgcg ggggacggta gttgcgttat 3120
agcgtcggtg ttttcgtatc ggcgtcgtgt ttttggaacg agggtaggat cgcgggtagc 3180
gtcgggttag cgcggagttt ttttacgttg ttgcgttttt gcgtttttgg ttttcgggtt 3240
aggtggggat aatttttcgg tttttagcgg ttgcgtttcg ggcgtgggcg atgcgttttt 3300
ttcggtggga cgtagttgat ttgatcgttt gggagaagta gtgtaaggaa attgatttag 3360
ggttgcgttt attttttttt ttttttttta gtagggtggt tgtttatcgg tttgtgttag 3420
gcggtttggt tttggcgggg atgcggtaga ggaggttttt tgagtcgttc gaggtcgggg 3480
cgagtttaga attggagaag ttagtttggt tttttagtgt gattttggag tgataagagt 3540
tggttatgtg tttttaagtt attgtatttt aggtttgttt tttttgtcgt tagggtcgat 3600
ggaagttttg ggaaatttaa aggttatagg tgttggaaat ttgtagattt aagggtttat 3660
ataggtaacg gttagcgttt agggattttt tagcggcgtt tttgacgttg tttttttatt 3720
tcgggtttag tgtttttttg tttcgcgttg agttgttttt tattcgttat cggcgatggg 3780
gaagggatat ttggggagag gataggggac gtttttagtt tttgaaggtt gtggaaggat 3840
ttatgagttt tataggtgta atttgaagtt ggaaagcgag ggttaaagaa ttggtttata 3900
tttttttttt tagtttttttg ttatatttta tattagaacg atttatagaa gcgatttgaa 3960
gagaagggta ggttttagcg ttataataaa aatgaatacg tgttttttttc gtgtatatag 4020
aaagggttgg gggagatatt ttttaggagt ttggagaagg cgaagtgggg agagggtaaa 4080
aattgacgaa gaattgggaa atattttttt tttttttttg agattgagtt ttattttgtc 4140
gtttaggttg gagtgttgtg gttttatttc gatttattgt aattttcgtt tttcgcgatt 4200
tttgtggttt agttttcgga gtaattggaa ttaaaggcgt ttattattat ttttagttaa 4260
tttttgtatt tttagtagag acgggagttt tattatgttg gttaggttgg ttttaaattt 4320
ttgattttaa gtgatttgtt taatttggtt ttttaaagta ttgggattat aggcgtgagt 4380
tattgtgttc ggtcgggaag tagacgtatt ttgttatttt tttagatttc ggggtacgga 4440
gtatttatat aggttgatag ttaacgaatt taaagattgt aaattttttt ttagcgaatt 4500
attttatggg gttggtgggt tttaggaacg gggtgatttg gtattgggtt tttttagtta 4560
gttattttta gtggatatat ttatgtattt taatgagttt aaattggtag atagtttttt 4620
tttaattata ggtttatttt ttttatttgg gattagagtg cgggaaaagg gtattttggt 4680
ggtttttagg tgtttatagt agtttattat aatagttttt gagttttgga ttgtgtttga 4740
tagatcgagg taatattttt aatttataga gaatatagtt ttttagattt tgtttttaaa 4800
gaggaaaggt aaggaggggt tatttaagat tatattaatt taagaagata tggatatatg 4860
ggaggaatag tatagattgg ggcgtgttag agggtgaggg gtgggaggag ggagagtatt 4920
aggaataata gttaacggat gttgggttta atatttaggt gatgggatga tttgtgtagt 4980
ggattattat ggtatatatt tatttatgta ataaatttgt atattttgta tatgtatttt 5040
tgagtttaaa tgttagaaat tttattaatt atgttatata gttttttttt tttggttaga 5100
ttgtatttta taagtgaagg atttttaggt taatataaat agttggtatt gagtttggga 5160
tttttttttt ttttttagat aattttattt tgttgcgtag gttggagcgg agtggtacga 5220
ttttggcgta ttgtaatttt tacgtttcgg gtttaagtta ttttttttgtt ttagtttttt 5280
aagtagttag gattatagat gtgtattatt atgttcggtt aatttttgta tttttagtag 5340
agatggagtt ttgttatgtt ggttaggttg gtttggaatt tttggtttta tgtgatttat 5400
ttatttgggt tttttagagt gttgggatta tacgtataag ttatcgtatt ttgttaagtt 5460

cggga 5465

<210> 145
<211> 22169
<212> DNA
<213> Artificial Sequence

<220>
<223> chemically treated genomic DNA (Homo sapiens)

<400> 145

ggacgcgtta gttttttttt ttggatttga tgaggtacga acgtattttt aataaagtta 60
ggtttttcg tcgtggtttt ttgggcgggt gtttgtggtt cgggttatga gttacgttgg 120
gtaattttat tacggggaag agggtaggaa gttgggagtt atcgtttttg tgttcggttg 180
ttatttcggt acgagggcga tcgtcggttt cgttttgttt ttatggttga gggtttttgg 240
gatgtggcgg gagacggggg agtttatttt ttaaattcgg tgtattttgt agggtcgttg 300
tatttataaa aaggttgatt ttatatagga tttgtttttt tgggttttgg tttaggttgg 360
ggcgagattg gttttttgtg ttatttgtgg aagagtttgt ttagtaaatt gatagtgtta 420
atagaatgtt aagggggtat ttatttagtt ttaaataatt aatttaagtt ttttagagat 480
gtataatgga tttcgaggtt aggggattgc gtagataggg tcgatatttg atgataaatt 540
tttatttgtt aattagagta aggtcggggc gtgttattaa ttttagtgtt aggcggagta 600
gaaggaataa tacgattatt ttttggggaa aaattttaat tagtaattaa ggagttggta 660
ggtttttat tgggcgagaa tagcgtttat ttggtgttta ttagattgaa tagtttcgtg 720
taagtgaggt cgggtgagtg ttttgtggtt attggttttt attgtggcgg taggcgttgt 780
ggggggttggg ttcgggtagt atttgggttt ggtttgattc ggagacgttt ttggtagtgg 840
ttatttatag attgtgattg cgaggatttc gatggggtcg ttgtgtgtgt ttgggaattg 900
taggtgtgga tgttagaggt aaagtttttt ttgtgtacgt ttagtatatt agagtttttt 960
agtttttttt tatagagttt ttagtttttt tttatagagt ttttagtttt tttttataga 1020
gtttttagt ttttttttat agagttttcg agtttttttt tatagagttt ttagtttttt 1080
tttatagagt tttttagttt ttttttatag agtttttaag ttcgttttta tagagttttt 1140
agtttttttt tatagagttt tcgagttcgt ttttataggt tttattggat attaggtttt 1200
agattttttt ttaggaaatt gtaggaatta tagtagggta gttttataga tttgagggaa 1260
tcgttgtttt attttcgttt tatagagttt tggtgtttgg gttgtagttg aggttgaggg 1320
gttgaggatt cggaggatgg gatttttcgt ttagcgggtg tagttaggtg ggaggggtta 1380
ggtttttagg gtttataagg ttttttgggg atttattcga agttatcgtt ttgtattagg 1440
tagggcgtgc ggagagggtt ttgtttaatc gtgcgggttt aatttggagt gtagacggta 1500
ggacgttttt agttttgggt ttttgagttt gtgagtgggg ggtttagagg tgaggttttt 1560
atagaagttt ttttttagg ggtaatgggt gtagggtagg cggggagtat ttaggatttt 1620
ttttagggggt aatgggtgta gggtgggagt gggtggaggg gggagtattt aggatttttt 1680
tcggggtaat gggtgtatgg tgggtggagg ggggagtatt taggattttt tttaggggta 1740
atgggtgtag ggtgggtgga gaggggagta tttaggattt tttttggggt aatgggtgta 1800
gggtgggtcg gggggagtat ttaggatttt ttttaggggt aatgggtgta gggtgggagt 1860
gggtggaggg gggagtattt aggatttttt tcggggtaat gagtgtaggg tgggtggaga 1920
ggggagtatt taggattttt tttaggggta atgggtgtag ggtgggtgga ggggggatta 1980
tttaggattt tttttagggg taatgggtgt agggtgggag tgggtggagg ggggagtatt 2040
taggattttt ttcggggtaa tgagtgtagg gtgggtggag aggggagtat ttaggatttt 2100
ttttggggta atgggtgtat ggtgggtggg ggggagtatt taggattttt tttaggagta 2160
acgggtatag ggtagggggg cgtattgagg atattgttgg ggagtttttt tttttagaat 2220
tttataggtt tcggagatat attgaaagta attttaaaat gttaggatcg tttataatcg 2280
ttttgtatta tttttggggt aattaggtat ttcgatgtta atacgtacgt attttttcga 2340
tagggagaag ttttttagtt gtttgtagat agggtttcgt tgttaagggt tattggttgg 2400
tttggtgttt tgtaggggat atggcgtttt ttcgttttaa gggtaatttg gagaggatat 2460
tatttgtgtt ttagagtgag gggttaggtt tttagttttt ttttgttata cgggtttgga 2520
tttaggttag ggtatagtta gttattttaa gttgattttg agggtttagg gggagaagta 2580
gtttggagtt aattatatag agttttgttt agggaaggga aggtttcgga gtaggtagat 2640
tgggcgggtg ggggttgaga tgtttgttcg ttttatggat gtgtttaaag ttttattgtt 2700
tttcgttgtt ttagttatcg tcgttttttt ggtttggagt tttttgtcgt tgattttgtt 2760
ttagttagcg tggaagttgt ttcgtggtgg gttttatttg aaagttatag taggtgtatt 2820
tagatggggt tttatagtat tgattttgta tataggaggt tatgaaggtt gtagtgatta 2880
tttttagttt gtggttaagt tcgtttattt tgtggtttgg tttttaaggg gtatgttttt 2940

tttttgttta gggggggtttg tatttgtcgt gtttatttat attttagggt ttagtatgta 3000
gtagatgttt aataaagatt ttttattttg tatttttata gtattaggcg tgtggacgtt 3060
tagtgatcgc ggttttattg tgtgtttttt gggggttaag ggatgttgaa tagtttggcg 3120
gttggtatga gtgaggttta gggataggtt gtgagtttgt ttcggagttt tgagtaggat 3180
aggggtcgcg ggtacgttag tttattttat aggtttattt gggaatatgt taggattttt 3240
tttagggggt tttgtggttg tcgggtggtt gtatcggttt agagtgagcg ttattttttt 3300
taggaagtta tttcggacgt ttttttaata tttgtttttt ttggattgtt tgggagttgg 3360
gatgttgggg tttattttttg gttagttttt gatgtttttg gaggttaaaa tgtagttttt 3420
agaagaatgt agttttagtt tacgcgtgat atggatgaat tttaaatata ttacgtttag 3480
tgaaagaagt taggggtaaa agattattta tggtataatt ttatttatag gtaacgttta 3540
gaagaggcga atttagaggt agaaagcggg ttagtggttg ttaggggttg gggtagttag 3600
ggtagagtga tagtatttgg gtatagtttt tgttttgtgt agtgagaaag tttcggaatt 3660
agatagtggt tgaatgaatt tgtgaatgga ttagatgtta agtaatagta tgttttagtt 3720
taaaagttat atttttagtt tggataatat agtaagattt cgtttttgta aaaaaattta 3780
aaattagtta ggtatggtgg tgtatatttg tggttttagt tattttggag gcggaggtag 3840
gaggatcgtt tgagtttagg agtttgaggt tgtagttagt cgtgatttta ttattttatt 3900
ttagtttgtg tgatagggtg agattttatt ttaaaaaata aaaaagtggt tatgtatagt 3960
ggtttacgtt tgtaatttta gtattttgag aggttaaggt aggtggatta tttgaggtta 4020
ggagtttgag attagtttga ttaatatggt gaaattttgt ttttattaag aatataaaaa 4080
ttagttggga gtggtggtat gtgtttgtag ttttagttat ttaggagtat cgcgtgaatt 4140
tgggaggtag aggttatagt gagttaagat tgcgttattt taatttagtt tgggtgataa 4200
agtaagattt tattttaaaa aataaataaa aaaaggtaaa ttttgtaata tgaataagtt 4260
attatttaga atttttttat atatatattt atttttaaat tagtttttag ttttttttgt 4320
ttttagggag tagggtgggg tggggtaggg ttataaggag aatttaatta ggtaattttg 4380
gaaaagggtt ttttttttatt tttttatttt tgtagttatt gttttttttt tagttggggc 4440
gattatcgag tttagcgtag gtttcgtcgt agggtttata gttattatta cgtttttagt 4500
tcgttttagg gtttcgttgg ttgtagttgg aaggggaatt atatatagat ggggaaattg 4560
aggtttaggg aggggatagt gtggggagta tagtaaatta gagttagttt tgttttatag 4620
tttttcggag tttcgggttt tataggagaa agcgttagcg ttgtttggga aattcgaggt 4680
cgttaaacgt agttcggttt ataggtgtga tgttttaaag ttagtcgttc gttaaagttg 4740
ttggtatttt ttttttagtt cggggaaaga ttcggaaagt tttggatatg tttgtatatg 4800
aaggtgaaag ttgacgttag tttgttttag atttttacgt tttcgtagtt atagtttttt 4860
tatttgtgcg ttggggatga aaattgtatt ggtatatttg gagggtgggc ggtcgtttgt 4920
tagggttttt acgaggttgg gtaatagcgg cgttttgagt gtggagttgt tatcgttgta 4980
ttattgagga aggtaagtta gacgtttaat cgattttgta aagttatatt tttttattat 5040
ttatagacga gatatgaaat aggaaaggtt ttttttttat agtaaaaggg tgtagttttt 5100
cggttcggtt tggaattgtt tagtgggtat cggtggtcgt tgttgtttat tataggtgta 5160
gggaatgagg aatgaagtta gtttcggttt ttgtggggtt tttagtttgt cgttcgtatt 5220
tgggggtttt cgtatttagt tttgttttgt ggaagtattt tttttggtgt attgtttgtt 5280
ttgttttatt ttttttttgt tagggttgtt gttttttttt tttttttttt ttttttttatt 5340
attatttttt ttaatggaaa atatgttggt tgttgtttag aggtagggtt tgttttaagg 5400
agttagtttt ttttagagta ttttaggttt ttattttagt tttcgtttta ggtttttttg 5460
tttttgatgt ttttttagtt tgagtaggtg agatttttta tttgatattg tgtggggttt 5520
tttttgttgt tttttgtttt tttgagatag agttttattt tgttatttag gttggagtgt 5580
agtggtacga ttttagttta ttgtagtttt cgtttttttgg gtttaaagga ttttttttgtt 5640
ttagtttttt aagtagttgg gattataggt atacgttatt gcgtttgggt aatttttgta 5700
tttttagtag agacgggggt ttcgttatgt tggttaggtt ggttttgaat ttttgatttt 5760
aggtgatttg tttatttcgg atgttgtatg tttttaaata tgttttttta attttggcgt 5820
ttttgaagat taggttttgt ataaatagga ggttttgtta taggtttttt cgtttagttt 5880
agtatgagga ggagaattaa tttgtttttt ttttggttac gtttaagacg aggtaggtga 5940
tttaatggaa agagttagat cgggtgatag gagttatttc gttttttttt agtttttttag 6000
gtcggttgta gggttaagtt atttttcgtt tgagtttttt tttttttttt ttaaggggaa 6060
gttacgtttt tagttcgttt gtagtcggga aatgacgggg tagtttaggt tggttggggt 6120
tgtgtttagg gatattcgtt tgggagaagg agaggggtag ggttagtagg aattttgtta 6180
ttgttgtgtg attttggggt tgatggcgat ttttttgggt tttttttttt tatttatcgg 6240
tgtttgattt agatagtttt acggggttgt cgggagttat gttaggttat tatcgggtcg 6300
gtatattttt aatatgtttt atatgtgagt taggtgtttt ttttacgttt tgttgggaag 6360
tgaattttat aggagtaggg gttatgtttg ttttgttttt atcgggggtt tggtttggag 6420
tagaacgtag tatatagtag gcgtttaaac gtgtttgtga attggagtta ttttgggaga 6480
tggatttttt cggagttgga gttattttgg gagatggatt tttttagagt cggtaagttt 6540
ttttttaggt agtgttaggt ttagtttatt aggtagtatt tttaggattt tttgaattta 6600

ttcgttaatt aattaaatat ttaggaagga ggttttggaa attagtattg ttagttaacg 6660
gggagaaggc ggggaagaaa ggagcgttga ttaggtgttt tttttatacg gtttttatgt 6720
aatttttata tattattaag agatttagcg tgtattagtt cggttgatat gttggggaaa 6780
ttgaggtttg tatgatttga tgatttaagg ttatagagtt agtggttagg ttaggtttta 6840
gatacgaggt tgtttaaagt taaagcgtat tatttagtag gtagttggat atttttttgg 6900
aggtgttggg tgtgatagtt tatttttatt ttttttagtt tttggaagac gtttcgtgtt 6960
attttgattt tgtcgttagt agttaatatt ttagttgttt tttgttttaa cgtttagttt 7020
agacgtaggt agttttggga gagcgtttta gattaaggac gtagttttat ttagatagat 7080
agggttttttg tagttttttt tagttttttt taaaataaag agaaaaaagg tggagttttt 7140
tttagtattt tttgtttata ttgacggagt tgatgagggg gttagagaag agttaagggt 7200
tatagtgatt ttttgggttt gtttagaagg ttattatttt cgattatagt tattacggtt 7260
atatttcgta tagtagttgt ttagagatta attatttttc gtttattagt atgtgttgtt 7320
aggtgttttt tatttggtta ttggttgatt aggttacggc gatattttga ttttttggga 7380
aggtattttg tattaataaa attatttttt ttaatttcgg gttttgtaat tatttgattg 7440
gtagttttcg gtgaaggttt tttttttttg ggatggaaag aaatgattta tttagtatta 7500
gtgagttagg tattgtgacg tttattatga ttatgttttt tttatagcgg aggtagtaaa 7560
tggtattaaa agttaaagtt tttttcgggg ttatagttgg atattacggg gttaggattt 7620
ttatttaggt tggtagggtt ttaagagttt gtattttaga ttttttcgag aaatagtgtg 7680
ggtttttttt tttgttgggg gagttaggtt ttttgtaggt gttttggtta atttttggat 7740
agatttttttg ggtttttttga gaaggatggg taggaacggg aaggtagagg tatttgaggg 7800
gtgtatgttg ggtggggttt aagtttaggg agggagttcg gggttttttt cgaagcggtt 7860
gtaatgtttt ttagattgtt gtgtgagttt tgatttgtgt ttttataagt cgggttcggt 7920
tgtggttttg gtttttaatt ttgcggttta gaagtaggag tttgaggacg cgttttcgtt 7980
ttattttttc gttggatttg tggggtgtgt ataatggggt ttttgtgggt tgagttcggt 8040
tgtttgttag gttaaattga agtaggtttt tcgttaagaa ggaggggggcg ttaggtttta 8100
gtaaagggga taaggttagg tattaaagtt atggtcggcg gtttgttttg ggttggaaat 8160
agtttatcgg gattttaggt ttggggaatg ggaaagtggg tttatgatat tagtgttttg 8220
cggttttgtg gtagggtttt aatttagata ttagggagtt gtagtaattt gtattttaaa 8280
gtatagcgtt ttggtttagg gagattttag ttttagattt ttgtgggtta ggttgtgttt 8340
attttttggt gttattgaga agaagtaata ggtttttaat ttttttagta cggtgttttt 8400
tttatttttt taaagttttt tgtaggagta agaagttttt tttagagttt cgaatgggtg 8460
agaggtaaag ttcgagtcgg gagcgtagtg gcggggaagg gggttatata ttttgggaag 8520
ttacgtataa ttaattatac ggtattaatt cgtttttttt aataatagtt gttgtatttt 8580
ttttggattt tagttgtcgg tttttgaatg aaaggaaata agatttaggg tattaagcgt 8640
tcgtgcggtt tttgtagagg gagaaggggg ttttaaaaag taaaaaaaaa aaaaaaaaaa 8700
aaagtagtgc gtatttatta gtgtttgata aagatataat cggttttgtt tattaaattg 8760
tttatagatt tgtttaattg gttttagttt ggggagggtg ggggtcgggg agggaggggg 8820
tttgttgttt ataggttggg tagtcggtat aggtaggagt cgggttgggg ttgttaaggg 8880
aatgggattt tttgtagttg gaatgagggc gcgtagattg gttggggagt tattttttta 8940
tttgtagttt ttgggggggcg gagggaattt tttttttaga tattaaatat tttttttatta 9000
tttttattag ggatggattt aagttgaagg gtatcggtta tagtagaata gtttttttacg 9060
gttgatgggt atttataggt aggtaggagg gagcggtttc gtacgatttt tcggtttttt 9120
tagtgtttat tttatatttg aggttggtag attagaaggg ttacgggatt ttgaggttag 9180
agtttgtaga tgttaggata gaaattagat ttaataatta ttttatattt tacgttaaaa 9240
tatgagtttt attttttttt ttttagtttt agtttgaggt cgggaagagg gagttggggt 9300
aaatagagag tttaaaggcg ggttcgattt tgaagagtta atttttttttg aagaggtatt 9360
atttattcgg aggtggtatt atttgtttag gaagtattat ttgtgtttag aggaggtatt 9420
attcgtttag aggaggtatt attatttaga ggaggtatta tttatttaga ggaggtatta 9480
ttatttagag gaggtattat ttatttagga ggtggtatta ttcgtgttta gaggaggtat 9540
tacgtattta gaggaggtat tattcgttta ggaggtggta ttatttgtgt ttagaggagg 9600
tattgtcgtt agtttaggag gtattacgta tttagaggag gtattattcg tttaggaggt 9660
ggtattattt gtgttagagg aggtattatt attagtttaa gaggtattat tatttattta 9720
ggaggtatta ttttcgagag gaggtattat ttgtgtttag gaggtattat tcgtttagag 9780
gaggcgtttt aagttggtag ggtggtgggg gttgggtttt ttagtaaatg gtttagagtt 9840
ttttttggta gattaattgt tttttggggga tttaaagaag aattggggta ttgggatttt 9900
tatttagtga tttcggagtt tgaggtggtt tacggaggag tgtttaagtt tttagtagtt 9960
agattagttt tatacgtagt ataattgttg gtgattaagt aattgttaga ttttgtttaa 10020
taaaagttag tacgtgatcg tgtttagatt ttgggttatt tttagaagat aatggtttag 10080
tgttttgttt tttgcgcggt tgtagaaggt aggggttttg ttttcgataa agtaataggt 10140
ttcgtagggg tggtatttat acgtaggttt tcgtgttatt ggtcgtttta tatttttcgt 10200
tatttaggta ggttttatcg ggttgggagt atcgcgggtt taggtagggg taggagaaga 10260

gaggttagtt ttattcgtat tattattatc gaaggttttg gttattttgg gggcggggat 10320
ttgtatttag ttaaggggta tcgttcgttt tttatttcgg acgtattttg tttagtatta 10380
tgggttgttg gggtacgggt tcgtaggaag tttttttatg aaattaaagg ggcgttaata 10440
ttttgtttag agttttgtag ttagaaatgg ggggaagggg agtagtaggg aatacgtttt 10500
gtttgaagta gggtggggtg gtttgatgtg tcgtttggac gtagtggggg aaaagtagag 10560
ataaagtggg ttattttta aagcgttgtt ttttattttg gaggtttatt gaggagtttg 10620
gtagggtagg gtttttttatt tagtcgtcga gattgttttt gtttaaattc gttttgtaag 10680
ggggtagagt tggacggggg gaggggggt ggttatattt agttttcgtt cgtgggaaaa 10740
tgcgatagtt gttcgttcgt tatttcgttc gttaataaag cgtcggtttt cgttttagaa 10800
ttagagcggt ttattgtgga gtcggggtcg tttttagggg tatcgggata gtattttggg 10860
gacgcggttt cgttttcgtc gatatttaat atttgttttc gcgggcgcgt atttttaggg 10920
tagtttttat atatttatag gagggggatg aaggtttttt attcggaagg aattcgattt 10980
tatttcgaga agggattaat tatttaagtt tttttttttt tattttatcg gggttgagaa 11040
acgggggagg ggggtgtgat atagagtcgt attttttgga agaggataga taattttcgt 11100
ttttaatttt cggagaaaga gtggattaat tattcgattt tttagagatt tcgttcgttg 11160
tgcggcgggg agaaatgtaa gagtttttta ttcgcgtttc gtttttcgtt tttaagtttt 11220
ttaaatttta atttcgtaaa gtaaaaggaa agtttagttt tttcgggcgc ggcggttttt 11280
tacgtaattt tttttttaaa ttgagagtcg ggttgggggg tatcggcggg cggggcgatc 11340
gggagttcgg ggatttagtt cggtcgcgcg ggttagtgaa ggcgaacgcg gcgtcgtaga 11400
gtttatattt cgcgttttaa tattcgtttc ggcgtgggtt agaggcgaag aagaaagaag 11460
ataaatggtt cggagaagta ataggaaatt aaaatcgatt ttatttaaat cgaaaaatag 11520
taggtagttc gttcgttcgg tcgatcggcg gtaagtttta cgcgcggcgg gtgaagggcg 11580
ggttcggagt agggttttcg gggttttagt attttatatc ggtttttttaa ttcggtttta 11640
ggtacgggcg gcgcgagttc gtttttacgg ggggatcgag ggggaaggtc ggtttttttt 11700
gatttcggga ttttagaatt ttacgtttcg ggtcgttcgt tttttttttt ttatgttggt 11760
tttttcgtcg ttcgttttta gtcgtggggc gcgcgcgtcg ggcgtcgtta aagtttttaa 11820
agggcgcgga aagtgggggt tcgcgggtgg gtgggcgttt atttcgtgcg gcgagcgcgg 11880
gtagtagcgt taggtagagt aggggcgtta ggagcggcgg tatgtttttt tatcggttgt 11940
tttttgcgtt cgggcggcgg ttttttgcgt tggtcggcgg ggttgggacg tatacgcgcg 12000
gcgtacggtt tcggggcggc ggcggacggt ttcgtttttt tttttcggt tggttggcgg 12060
cgttgtgttt tcgtaggttt tcgggttcgg tttcgcgttc ggttcgtttt ttcgttgcgt 12120
tcgcgttcgc gttcgcgttt cgcgtttcgc gtttttgcgt tttttttcgc ggtcgaggta 12180
ttagtgaggt ttagagtcga ggtgcgtttc gttttcgtcg gtttcgtttc gttcggtttt 12240
atttcgtcg gtttttttcgt ttttttcgcg gttatttttt ttcgtacgtt tcgttcgttt 12300
ttgcgttttt tcgagttgag tcgcgcgttt cgagtttagt tttttcgggg aggagggatt 12360
cggcggcgcg gcgtgggtcg ggagcgggcg tttgggatta tttttcgttt gaaagttttt 12420
agaggttaaa agtttgagtc ggggttgcgg aaggattcgg ttttggcggt tgtcgcgttc 12480
ggcgataatt ggcgattgaa atttgtatgt gaataagcgg aggagttcgg ggcggaatgg 12540
ggagggcgag gtcgcggata gtcgtgtgta tcgtgcgttg ggtcgggcgg ggagtagttg 12600
aggttacgtt gggggatttc gggttcgggg tagggttttg ggagagtggt ttagtcgtgt 12660
gtgcgttttt agtttagcgg ttttattttt tttcgagggt ttcgtggtcg cggttttagg 12720
cgtttagagg atcgatcgtc gttagatatt tttggtcgtg attgttcgag tatttgatcg 12780
cgagggatgg gggcgtcgcg gtttttgggt aggggatttc gggcgtttcg agggagtagt 12840
cgggattagt gtttcgcgga cggattgtgt ttcgtttttt taagggggat tcgcgtcgat 12900
acgtttttcg gttatcgttt tttttttgtt cgggcgtcgt ttgtttaagg tggttttaga 12960
aagtataaac gggtcgggcg gatttgtcgt tggcgtcggc gtagtttttt ttacgtgttt 13020

ttttcggttg atttattttt ttattacgat gttaggtacg ggtattttgt gttaagtttg 13080
gttaagcgtt aggagtttaa aatgtttgtt atagtttttg cgtaaagttt acggtttcgt 13140
gttagggata gatagcgttt tttttttatt ttaatatagg gtggtgagtg cgatggtaga 13200
aggcgcgtgt ttcggcgttt tttttggagt aggagggtta agtttgtttt gcggggagat 13260
cgaaaggttg ggagggattt aggattgtgg gggtttgggg ggagggttgt agaaatttta 13320
gagtatggtg gtagaggtag gtatagaaag taggataaat tttatatttg attttagtaa 13380
gtttttttttg gtatattttt tgttaaatgt tgagtatttt ggaaatagtt agtgtaggcg 13440
gtttgtttcg tttttgtagt tgttgttggg agttttttttc gtttgcgttt ttttagaatt 13500
tggttttatt tttgtaggtt tggggtaata ttttggggtt agcgttttag gagaggaggc 13560
gtttggaggt agtagagatg tttatgtaat agtagttagg tagtttggag gagtagattt 13620
tttatagaag gaagcgcggg gttgtgtagt ttttaggttg aggattttgt ttattacgac 13680
gttttttgtt tttggtgggt gggggtgggg tggggtatag ggaggttcga aggttttagg 13740
tttggtgttg atcgggtgcg ggtgcgattg tttgtgtgtt tttattggta tttttgtttg 13800
tgggttttag gttagaagta atggtttatg tagtttaggt tcgggggtta gtttttagta 13860

```
ttttgcggag gggcggtttg ggaggggagg aggggatttt tggatagggg gttttttttg   13920
gtttgtttta gttgtgtcga tagtattttt attttgggtt tttatttttt attagtagag   13980
aagagagtgg tttattcggt gtttattcgt taggttattt agtagttagg tacgtttata   14040
agttcgcgtt acggatggtg cggtcgggtg tataggttcg agggaagtat aggtttcggt   14100
tttttagttt ttaattttttg tattatttat agtcgggagt attttatgga tagttgtggt   14160
cgaggagaag ttagggatga gggggatggg ttttattttt ttattagttt gggaaggggg   14220
taggtttttg gtaagtaggt gagaagtttt tttatttata agttgttcgg gttttttatt   14280
ttttttttgtt aggttagcgg gtgtgggtag tcgggggagg ttggggtttt gatataatag   14340
gagtatattt gttggtttag ggtttggaga ttttttttta taggtcggga ggtagttttg   14400
ggtagcgggt attgcgggga ttaaagagaa ataagagtag gttgtgtgta gggtatttta   14460
gttattttag aaatttaatg ggagggtggg ggttaggatt ttaagaagtt gaattggggt   14520
gtagtgtcgt taggtttttt ttgtaggagt tattttgggt tttttgtttt aatttgtggt   14580
gggagttggg gtaaggtttt aggggtattt cgaatgaatg gggttgtttt taagttggtt   14640
agggagtttg gaagtagatg agatggggtc gtagagtttg gtttgcggag gggagaggta   14700
gacgtgtttt gtagtttttt ttttttaga ggttttttgc gggtgttgag gggagagtgg   14760
tttaggcgtt gtattttagg agattgaatt gggttagttt ttttttgttt gttgttagag   14820
aaatgggggt attattgtta gggtgagtta aggaggtttt gttgatattg gtttaattag   14880
gttttttttt tgcggttgtt gtggggattt tcgagagtag agtatatttt taagtgcgtg   14940
gagaggtaag tatagttggt gagtatacgg ggatagcgac ggagtttatg gagatttagg   15000
tgattttaaa atattttagg gtcggtttcg gttttagaga gatgtggttc gagtagaggg   15060
gggttagtag tgatttggtg ggtgggtgta tttgaggggt gtttgtgttg aaagttttgt   15120
agtagatttt aaggagtagt tagggttgag atttcggtg ttgagtttaa ggtttttttt   15180
ttttttgtag gagtttattt tggtttttgt agtttttatg attttgtggc gttgggggc   15240
ggttagtatg atgtttttta tttttttagga gggaagttga ggtttagaga tattatgagg   15300
atgttagttt aaggtttagt taggttttgg tttttagttg ttgttattta gtagggtaag   15360
tgggattttt aggaggtgtt gggggatatt tttttttttt tttatattta tagtttgttt   15420
atcgtttaag gtagtaggtt ggattcggtg tagggattat ggtttggcgt ggttaggttg   15480
gagtggtttt tgggattgaa agtagggggc gtggggttt tgaggcgggg tcgagtgcgg   15540
ggacgttttg tggcgggtat tttgttgggt tttgggtggg ggttattatt aggtagttgt   15600
gggaatattt tagattattt aggagtaggg tttttttatc gtttgaagtt gattgtaaaa   15660
aatatttgat ttgtgtgtaa agtttaagtt tagaggtttt tagggtgttt agtttttagg   15720
gtgtttttta cgtagttttt acgtttttat ttattgtagt ttttgattat cgggtatatc   15780
gtggggtttt cgttggtcgt cgggttttag gttttgttgt attaagtgat ttttaaagtt   15840
ttatttgttt tttttattt ataaagaatt tgggatattt tttcgaggtt gggtattttt   15900
ttagatgtgg ttttagtgtt tcgtttgtta tttggttttt tgtgttgggc ggttcgttgt   15960
gacgtatttg gtagttttttg ttttaggatg gattcgggtc ggtttttgtt ttgagaatag   16020
agaacggttt ttgttttgag gaggttgggg tttggaattt gtttttttggt tttttgttgt   16080
gtgtagatag ttatcgttat ttaggtttag gggtgtttgt aggtgttggg tattttattt   16140
tggtttttat aggggttttt tatgttttgt atttttcggt ttgtgtcggg gattttttttg   16200
ttttgttgtt gtaggtttcg ttttttgtcg agggggtttt tttttttacgt gtttttgaga   16260
ttttttagtt attttagagg ttttcggtcg tttgagtatt tttgattatg aagttttttt   16320
ggtttatttt ttgtgtgtga ggtagagttt atttttatta tttcgattta tattagaggt   16380
ttcggaaggt ttgttgattg atttgtgatt gtaagaagtt gaaggaatgt gggcgtagat   16440
tcggtagggg tttttttttgg tgtaaagtta attggttgtt tatgtaagtt tgttgttgtg   16500
gttagttttt tcgttaggcg tttggtattt tttttggatt tagttgttag gatagtgatt   16560
tttagttgtg gttaggattt tgttcggggt tttgtttatt tagtgttttt agtggttttt   16620
atgtagaaat tgtttttttt gttgtttatg aggtatggga tagggttagt attcgagagg   16680
taggttttgt tgtttttttt tgtatagagg ttgggggagt gagttgttcg ggtaagtttt   16740
cgggtaggta ttgggttttt tgagtaggtg agttgagaag gttttagttg gtgtaggttt   16800
cgagagttgt aggattggtt atggggtaga tttggtattg ttattttaga gttgggtttg   16860
gggggtggtt ggtgtagtgt agattttagg tggaggttat ttgttcgttt tttttacgtg   16920
gtttagggtt tagatttatt tgtttgtttt tggaagtttt tagattgttt agagtataga   16980
ttttagtggg aaagatagta gataggtttg ggtatggttt tttagttttg agaagtgggg   17040
agtttattta gattttttta ggattttagg tttttttagt agtttataga gcgtgggtgg   17100
agtttgggtt ggggttgggt tatttgggag ttaaaggttt atttttgttg tttttaggga   17160
gttttaggga gacggtagag gtagtgtttg gttggggttt tggttggcgt gtcgtttgag   17220
gggtgtatgt attgggaagt tggttaggtt tgtttaaatt agggggtttt taggaggaga   17280
gatattagga ggatgggtat agaggcggtt tagtattagg gttggtattt ttaggtaggg   17340
gttgtggggg gttggattag atagggaggt tgttacgatg tttggttgtt ggatgggggt   17400
atagaggttt tgagtggggt ggtagagttt ggttattagg tttatttgta ttttttatgt   17460
ttttttatta tttttagatt tttatgattt taaattattt agttttaggg atttttgtag   17520
```

agtttgtggt gggggtgatt ttttattttg tttggttttt gtttatttta ttttagaatt 17580
ttttttaaaa agtaagagtt tcgggggacg aaatttcgag gttttatttt ttgggttttg 17640
gtataaattt cgagtttttt tcgagggttg agttattggg aaagtcggga tgggttggtt 17700
tttttttttt tttttttttt tttttttttt tttttttttt cggttttttt tttttttttt 17760
ttgttttatt tttttttttt gttttttttt tttttttttt tttttcgttt tttttatttt 17820
tttttttttt ttcgttttttg tttttttttt tttttgaggg taaaaatagt tggaaaattt 17880
ttttaaaaaa gtttatttta attttagagg gataatgttg gtgttttaaa gtatagtttt 17940
ttgtttcgga ggagttatcg gtagagttag gagtttttga aggattttga ttatattttt 18000
tattttattt cgatttgtgt ttggtgtttt aagtggagtt tgggttattt ttgattttat 18060
cgttggggtg atggagaagt agtcgttcga ggcgagagtt ttttaggtgg tcggggtttt 18120
tttttatttg ttttagatat atttaaataa gttgagaatt ttcgtttttt tatatttttt 18180
ttcgcggatt tttacggatt ttttttgtaa atgaatatgg gtttggaaat cggggtgtgg 18240
gttatagaga gtacggggaa ttttgttcgt gaagtaatac ggagtacgaa tataagtagg 18300
tatcgtatat ttgcgagtta ttcgggtttt cgggggaagg taaaaagttt taataagttt 18360
taagtaagat taggacgttt ttatagtttt gttttgatag ttaaggaaaa aatttgttaa 18420
aaattagagg cgttgttgag tgttggcgtg ggaatgttag tttagttaga gttttgtttt 18480
gggttattta aataaaaata gatatgttgg tggtaaatgt tagttgaata cgtaggagag 18540
cggtgttttt cgggttcgga gaagggtttg tagttgtatt tttttttttt tttttttttg 18600
gttttttttag ttttttttatt attacgatta gtattttatt ggtaaatatt tggtgtaata 18660
atttttaaaa attagtaaga aaagtttgag cgttttatgt ggttgtgatt gcgtggtttt 18720
agttggaagt atggattttt agcgtttttt agagtttgtt ttcgagggat attttaatta 18780
tttgagggtt tttgtacgat ataaattaga tcgattattt ttagagtgga gagggggata 18840
aatttatttg tagtaagtga aggttagagg ttgttttggg atggtattgt ttgtttttag 18900
gggtttgtgg tggtgtagta gatatgggga tttttgggtt tttttttttt tttgttgggg 18960
atgttatata gatttacgtt tagggatttg aatttagagt ttgttttagg ggtggcgaat 19020
gtatttggga tattgaggat ttttttatcg ggtgtatttt atggtttttt ggtttttttt 19080
tttgagacgg agttttattt tgttgtttag gttggagtgt tatggggcga ttttcgttta 19140
ttattatttt cgtttttcgg gtttaagtaa ttttttttgtt ttagttttcg agtagttggg 19200
attataggta tgtgttatta cgttagttaa tttttttatt tttaatagag atagggtttt 19260
attatgttgg ttaggttggt tttgaatttt taatttcggg tgatttgttt gttttggttt 19320
ttcgaagtgt tgggattata ggcgtgagtt attgtatttg gttagttttt tggttttgaa 19380
gggtttttgg gagggtttta gagaatgttt tggttttttt atttttgggt ttttatcgat 19440
tgaggttagg gttgggaagg ggtgatgagt ttaggatttt tttggcgaga gcgagggttt 19500
atgttggggg gttttttttt gttatttttt tttggtgata tggggttggt tggagagttt 19560
tttcgtgttt gggagttttt tagtgtaggg ggggaagggg gttattgtaa tgaatttgat 19620
attagttttt ttttttttcg ttttgttggt agtttttatt tatatggggg gcgggtttgt 19680
tttttttttt ttaaagttgg gggttttgag tgtggttggg gtaacgtttg gttttttttta 19740
gaatagtggg gtttggaatt tatttaaggg aagaagagtg aaggaacgta gatgtcgatt 19800
gtatggagtt ttttcgggtt gtagttttta gagtttgggc gttttgtgag ttagaaagag 19860
aacggttggg gagagggttg tgtgaggttt tgttttttta agatcgttgc ggaggatcga 19920
cggggtttag ggattttgat tgaggtgtgt cgagtatagt agttttggat aggtcgtatc 19980
ggttttttttg tttgttattt gtttatttgt tggtgggtag gaatagattt gatgttgtaa 20040
gaagaggttt ttaggatttt gttttttttta ttggttgagt ttggattgtg gagttagtcg 20100
tttttatatt gggtatagat gatcgattta ggttttgaga agatttaaat agtagtagga 20160
cgtgtatttg gtaggggtag gagggttttt tttggattta aaagtgtatt cgcggagttt 20220
tttttttgta gcgtaggttt tggggggata gtggttagga cggaggtttt cgtggaaagg 20280
gggagatgtg aatagtgagt tttttgtatg taggtatttg ggaaagaata ttttagttaa 20340
gtggttttga gtatatttcg attttggtta ttttggtttt gtggattaat tttggggttt 20400
aggattaatg atagttagtg ttcgttggtg tttttagttt gggttaggtt tgtgggttgg 20460
tttttttaat gatggggtaa gtttagtttc gtttatattt tttttttttt tttttgtgtt 20520
tttttttatt tttaggagtg ggttttaggt ttgttattcg ttatttatgg ggggttgtgg 20580
gttgattcgt atttttttaa aagattcgtt gtatattttt agagtttgtg agtaggattt 20640
gttttggaga tgggtttttg tagatgtcgt cgagttaaga taaggttatg ttggagtggg 20700
gtttgtttta atttaacgat tggggttttt ggtaggagag ggaggtttgg ggagaaggcg 20760
ttgggaagat aggtagaggt tggagatagt gtagttttaa gttgggagta taggggttgt 20820
cggtatttaa tagaagttgg aggaggtggg aaggattttt tttagagttt tgggaggggg 20880
ttcggttttg tgatatttag attttggatt tttggttttc ggaattgaga gagtatatat 20940
ttttgcggtt tgaagttatt aaatttttgg taatttgttt tggttaggaa attaatatta 21000
gggataaagg tgttttaatg tagataaaga tagtttttga attaattaag ttttgcgtaa 21060
ggttgtcggg attgattggg ggaagtgagg gatttagtag ttttttcgac gttcgttagg 21120
tatcggtttt tatatacgtt tgttggcggt ggttacggta ggtcgttttt taggtgaaat 21180

tatataatga gtagttttaa gtttgtcgat ttatatagta gaggttgttt ggtttggcgt 21240
ttttttttggt tggaggattt tgttttttttt ttttgtttgt ttgtggttat ttcggttgta 21300
gaggagattg gtagacgacg tttttatttt tggtcggggt aaaagttttg tttaaatatg 21360
ataacggtga aattcggaat agcgatagag ttaatttatg attgaggtag ggttggaggt 21420
tttttggaaa acgtacggag aaatacgagg gtttagttgt gttttgggtc gttttatgag 21480
tttagttttt ttcgttttag tttttttttt aaagaaagga taaaattatt gaaagggttc 21540
gtttgggtta taaacgtttt aaattggttt tcgcgtggaa attaaataag ggtaggtttt 21600
agtttttggg atttaggtga aattcgaggt ttgtttgagg ttatagcgtt ttttcgagga 21660
cgttagtttt gttggttgtg tttggggtta ggttgaggta tagcgggtta ggaggtcgag 21720
ttttttaaag gagttgtatt gttttttttt tagaatatga aattaatgtt tgttattttg 21780
aatttttatg gataagattt tttggaagtt agtttgtgtt agggttagcg tttttttaggg 21840
ttgatattta tattttcgta tttttagcgt gattcgggat agtggtagaa ttcgttgggt 21900
tttttttacg ggagtgtttt tttttttttt tttttttttt tttttttttt ttttttttga 21960
gtcggagttt tatttttgtt gtttaggttg gagtataatg gtgtgatttc ggtttattgt 22020
aatttttatt ttttgggttt aagtagtttt tttgttttag tttttcgagt agttgggatt 22080
ataggtgtat attattatgt ttggttaatt tttgtatttt tagtagagat gaggttttac 22140
ggtgttggtt aagttggttt cgaatttttt 22169

<210> 146
<211> 22169
<212> DNA
<213> Artificial Sequence

<220>
<223> chemically treated genomic DNA (Homo sapiens)

<400> 146

aggagttcga gattagtttg gttaatatcg tgaaatttta tttttattaa aaatataaaa 60
attagttagg tatggtggtg tgtatttgta attttagtta ttcgggaggt tgagatagga 120
gaattgtttg aatttaggag gtggaggttg tagtgagtcg agattatatt attgtatttt 180
agtttgggta ataagagtga aatttcgatt taaaaaaaaa aaaaaaaaaa aaaaagaaaa 240
gaaaaaaaaa aagtattttc gtagagagaa tttagcgggt tttgttattg tttcgagtta 300
cgttgagaat gcgagggtgt aggtgttagt tttgggggac gttggttttg atataggttg 360
gtttttaagg agttttgttt atgaggattt aaggtggtag atattgattt tatattttaa 420
ggaaaagata gtgtagtttt tttgggaagt tcggtttttt ggttcgttat gttttagttt 480
ggttttagat atagttaata gggttgacgt tttcgggaag acgttgtgat tttagatagg 540
tttcgggttt tatttgaatt ttaggggttg ggatttattt ttgtttagtt tttacgcgga 600
agttaatttg ggacgtttgt gatttagacg aattttttta gtggttttgt tttttttttg 660
gagaagggat taggacgaag aaggttggat ttatggggcg gtttagggta tagttgggtt 720
ttcgtgtttt ttcgtgcgtt ttttagaaag tttttagttt tgttttaatt atgggttggt 780
tttgtcgtta tttcgagttt tatcgttgtt atatttgagt agaatttttg tttcgattag 840
ggatgagaac gtcgtttgtt agttttttttt gtagtcggag tggttatagg taggtagagg 900
gggaaggtag agtttttttag ttaggaagag cgttaggtta ggtagttttt gttgtgtaaa 960
tcgataggtt taaggttgtt tattatgtgg ttttatttga agagcgattt gtcgtagtta 1020
tcgttagtaa gcgtgtatgg aggtcggtgt ttggcgagcg tcggggaagt tgttgagttt 1080
tttatttttt ttagttaatt tcggtagttt tgcgtaaagt ttggttaatt taaaaattgt 1140
ttttgtttgt attaaaatat ttttattttt ggtattaatt ttttggttag aataaattat 1200
taaaaatttg gtggttttaa atcgtagaaa tgtgtgtttt tttagtttcg aaggttagaa 1260
atttaaaatt tgggtgttat aggatcgggt ttttttttaa ggttttaggg aggatttttt 1320
ttattttttt tagtttttgt tgggtgtcgg taatttttgt gtttttagtt tggggttgta 1380
ttgtttttag tttttgtttg tttttttagc gttttttttt taaatttttt ttttttgtta 1440
agggttttag tcgttggatt agggtaggtt ttattttagt atggtttttgt tttaattcga 1500
cgatatttgt aaagatttat ttttaaaata ggttttgttt ataagttttg ggggtatata 1560
gcgagttttt tgggggaata cgagttaatt tatagttttt tatgagtgac gagtgataga 1620
tttggagttt atttttgggg gtggagggaa gtatagagga ggaggagaag ggtgtggacg 1680
gggttgagtt tgttttatta ttgaagggat tagtttatag gtttggttta ggttggaggt 1740
attagcgagt attggttgtt attaatttttg ggttttaggg ttggtttata gggttagggt 1800
ggttagggtc ggggtgtgtt tagagttatt tggttggaat gttttttttt agatgtttgt 1860
atgtaggagg tttattgttt atattttttt ttttttacga gggttttcgt tttggttatt 1920
gttttttttag agtttacgtt gtagggaagg ggtttcgcga atatattttt gagtttaggg 1980

```
gaggtttttt tatttttatt aggtgtacgt tttgttgttg tttggatttt tttaaaattt    2040
aagtcggtta tttgtgttta gtgtgagggc ggttggtttt atagtttagg tttagttaat    2100
gggagaggta gggttttggg ggtttttttt tatagtatta ggtttgtttt tgtttattaa    2160
taggtggata gatgatagat agagaggtcg gtgcggtttg tttagagttg ttgtgttcgg    2220
tatattttag ttagggtttt tgggtttcgt cggtttttcg tagcggtttt ggaagaatag    2280
ggttttatat agtttttttt ttagtcgttt tttttttggt ttatagagcg tttaggtttt    2340
aggggttgta gttcgggggg gttttatgta gtcggtattt gcgttttttt attttttttt    2400
ttttgaatga attttaagtt ttattgtttt aaggaggatt agacgttatt ttaattatat    2460
ttaggatttt tagttttggg gaaaggagga tagattcgtt ttttatatga gtgagggttg    2520
ttagtagggc ggagagagag ggggttggtg ttagatttat tgtagtggtt tttttttttt    2580
tttatattga ggaattttta ggtacggagg gattttttag ttagttttat gttattaagg    2640
agaggtggta gaagggagtt ttttaatata gattttcgtt ttcgttagaa gagttttggg    2700
tttattattt ttttttagtt ttgattttag tcgatgggaa tttaagagtg aggaaattaa    2760
gatatttttt gaggtttttt tagagatttt ttaagattaa aaggttggtt aggtgtagtg    2820
gtttacgttt gtaattttaa tatttcggga ggttaaggta ggtagattat tcgaggttag    2880
gagtttaaga ttagtttgat taatatggtg aaattttatt tttattaaaa atagaaaaat    2940
tagttggcgt ggtggtatat atttgtaatt ttagttattc ggaggttgag ataggagaat    3000
tgtttgaatt cgggaggcgg aggtggtagt gagcggagat cgttttatga tattttagtt    3060
tgggtaataa agtgagattt cgttttaaaa aaaaagatta aaaggttatg agatgtattc    3120
ggtagggggg tttttagtgt tttagatata ttcgttattt ttggggtagg ttttgaattt    3180
aggtttttga acgtgggttt gtgtggtatt tttagtaagg aggggaaggg gtttaggggt    3240
ttttatgttt gttgtattat tataagtttt tgggaatagg taatgttatt ttagggtagt    3300
ttttggtttt tatttgttat aagtgagttt attttttttt ttattttaga gatagtcggt    3360
ttgatttatg tcgtgtaaag gtttttaagt gattagagta tttttcggag ataggttttg    3420
ggaggcgttg ggaatttatg tttttagttg gggttacgta gttatagtta tatgaaacgt    3480
ttagattttt tttgttgatt tttgaaaatt attgtattaa atgtttatta gtgaaatgtt    3540
gatcgtgatg ataagaaggt tggggaggtt aagagaaaag ggaagagaga gtgtagttgt    3600
aaattttttt tcgggttcga gaagtatcgt ttttttgcgt gtttaattgg tatttgttat    3660
taatatgttt gtttttattt aaatggttta gagtaaggtt ttgattaaat tagtattttt    3720
acgttaatat ttagtagcgt ttttggtttt tggtagattt ttttttagt tgttaaagta    3780
aaattataga agcgttttgg ttttatttaa ggtttgttgg ggttttttgt tttttttcgg    3840
agattcgaat agttcgtaga tgtgcggtat ttgtttatgt tcgtgtttcg tgttatttta    3900
cgagtagaat ttttcgtgtt ttttgtaatt tatatttcga tttttaggtt tatgtttatt    3960
tgtagagggg gttcgtgggg gttcgcgggg gggagtatgg ggggacggaa gtttttagtt    4020
tgtttaaatg tgtttggagt aaatgggggg aaatttcggt tatttgagaa atttttcgttt    4080
cgggcggttg ttttttttatt attttagcgg tgaggttaag gatggtttag gttttatttg    4140
gggtattagg tataggtcgg ggtggggtgg ggggtgtggt taaagttttt taggggtttt    4200
tggttttgtc ggtggttttt tcggggtaaa ggattgtgtt ttgggatatt aatattgttt    4260
ttttaagatt aaaataaatt tttttaaggg ggttttttag ttatttttat ttttagaaaa    4320
gaagaaaggt aaagacggag gaagggaaga agatgaaagg aacgaaagaa gagagggaga    4380
gagggaggta agaaagagaa gtaaagtaaa gagaaaaaga gggagatcga gaaaggggga    4440
aggaggaagg aaggagaggg agggggaggg gttagtttat ttcggttttt ttagtgattt    4500
agttttcgga ggaggttcgg ggtttgtatt agagtttaag gggtggagtt tcgaagtttc    4560
gtttttcggg gtttttattt tttggagaaa gttttgaggt gaggtgggta ggggttaggt    4620
agggtggaag attattttta ttataggttt tgtaggggtt tttgggattg agtagtttgg    4680
gattatgggg gtttgggggt agtgaggggg tatggggggt gtagatggat ttggtggtta    4740
agttttgtta ttttatttag agtttttgtg tttttattta atagttaggt atcgtgatag    4800
ttttttttgtt tggtttagtt ttttatagtt tttatttgga ggtgttagtt ttggtgttaa    4860
gtcgtttttg tgtttatttt tttgatgttt tttttttttgg aagtttttttg atttgggtag    4920
gtttggttaa ttttttagtg tatgtatttt ttaaacggta cgttaattag ggttttagtt    4980
aggtattgtt tttgtcgttt ttttgggatt ttttggaagt agtagaaatg ggtttttggt    5040
ttttaagtga tttaatttta gtttaggttt tatttacgtt ttgtgggttg ttgaggggat    5100
ttggggtttt ggaaaggttt gagtgggttt tttatttttt agggttggga gattatattt    5160
aggtttattt attgtttttt ttattgaggt ttgtgttttg agtagtttag gggtttttag    5220
ggataggtag gtgggtttga gttttgagtt acgtggaaag gacgggtagg tggtttttat    5280
ttgagatttg tattgtatta gttatttttt aggtttagtt ttagaatgat aatgttaggt    5340
ttgttttatg gttagttttg tagttttcga ggtttatatt agttggggtt tttttagttt    5400
atttgtttag gaggtttagt gtttgttcgg ggatttgttc gggtaattta ttttttttagt    5460
ttttgtgtag aggggaatag tagggtttgt ttttcggatg ttgattttat tttatgtttt    5520
atgggtagta agggggggtag tttttgtatg gggattattg gagatattga gtagataggg    5580
tttcgggtag agttttggtt atagttgggg attattgttt tggtaattgg gtttagggag    5640
```

ggtattaggc gtttggcggg agggttggtt atagtagtag gtttgtatgg atagttagtt 5700
gattttgtat taagaaaagt ttttgtcggg tttgcgttta tatttttta gtttttttgta 5760
gttataggtt agttagtaaa tttttcggag tttttggtgt gggtcggaat ggtagggatg 5820
ggttttgttt tatatataga gagtgggtta ggaaggtttt atggttaggg gtgtttagac 5880
ggtcgagagt ttttgggatg gttgaggagt tttagggata cgtgggaaag agattttttc 5940
ggtaaaggac gaggtttgta atagtagagt agggaagttt tcggtataga tcgaggggtg 6000
tagggtatgg ggagttttttg tgggggttag ggtgagatgt ttagtatttg taggtatttt 6060
tgggtttggg tgacggtggt tgtttgtata tagtagggga ttaggaagta gattttagat 6120
tttagttttt ttagaataag ggtcgttttt tgtttttaga ataagggtcg gttcgaattt 6180
atttagagt aaggattgtt aggtgcgtta tagcggatcg tttagtatag gggattaggt 6240
agtaagcgag atattgaaat tatatttgga aaaatgttta atttcgagga aatgttttaa 6300
gtttttttata agtggagaaa ggtagatggg gttttaaaaa ttatttggtg tagtagaatt 6360
tgaggttcgg cggttagcga aggttttacg gtgtgttcga tggttagggg ttgtagtgga 6420
tggggacgtg gaggttgcgt aggaggtatt ttggaggttg gatattttgg aggtttttgg 6480
gtttgagttt tgtatatagg ttagatgttt tttatagtta gttttaaacg gtgaaggggt 6540
tttattttta ggtggtttgg aatgttttta tagttgtttg gtggtggttt ttatttagga 6600
tttagtaggg tattcgttat aggacgtttt cgtattcggt ttcgttttag agtttttacg 6660
tttttttgttt ttagttttag gggttatttt aatttggtta cgttaagtta tggtttttgt 6720
atcgagttta gtttgttgtt ttgggcggtg aatagattgt gagtgtgggg gaggggagga 6780
gtgtttttta atattttttg ggggttttat ttattttatt ggatgatagt agttgaggat 6840
taaggtttgg ttgggttttg agttggtatt tttatgatgt ttttgagttt tagtttttttt 6900
tttgaaaaat ggggagtatt atgttagtcg ttttttagcg ttatagggtt atgggggttg 6960
taagagttaa gatgaatttt tgtaggaagg aaagggggttt tgggtttagt atcgggagtt 7020
ttagttttgg ttgtttttta gaatttattg tagagttttt aatataggta tttttttaaat 7080
atatttattt attaggttat tgttgatttt tttttattcg gattatattt ttttggggtc 7140
ggagtcggtt ttggggtgtt ttaaggttat ttgggttttt atgagtttcg tcgttgtttt 7200
cgtgtgttta ttagttgtgt ttattttttt acgtatttag ggtgtgtttt tgttttcgaa 7260
agtttttata gtagtcgtaa aggagggggtt tggttgggtt aatgttagta gggttttttt 7320
agtttatttt gataatggta tttttatttt tttggtagta aataaggggg aattgattta 7380
gtttagtttt ttggggtgta acgtttaagt tattttttttt ttagtattcg tagggggttt 7440
ttgaagaaga gagggttgta ggatacgttt gttttttttt ttcgtaaatt aggttttgcg 7500

gttttatttt atttgttttt aggtttttttg gttagtttgg gaatagtttt atttattcgg 7560
ggtatttttg gaattttgtt ttagttttta ttataagttg ggataggaag tttaaagtga 7620
tttttatagg ggaggtttgg cggtattgta ttttaattta gtttttttggg gttttagttt 7680
ttattttttt attgggtttt tggagtggtt ggggtgtttt gtatataatt tgtttttttgtt 7740
tttttttagt tttcgtaatg ttcgttgttt agggttgttt ttcgatttgt aggagaaggt 7800
ttttaggttt taggttagta gatgtatttt tgttgtgtta ggattttagt ttttttcgat 7860
tgtttatatt cgttggtttg ataaagagga atggggagtt cggataattt gtgaatgaaa 7920
aagtttttta tttatttgtt aggggtttat tttttttttta ggttggtggg gaggtgaggt 7980
ttattttttt tatttttggt ttttttttcgg ttatagttgt ttatggggta ttttcggttg 8040
tgggtggtgt aggggttggg agttgggagg tcgaggtttg tgtttttttc gggtttgtgt 8100
attcggtcgt attattcgta acgcgggttt atgagcgtgt ttggttgttg ggtggtttga 8160
cgggtggata tcgaatgggt tatttttttt tttgttggta gaggatggag gtttaaggtg 8220
gaggtgttgt cggtatagtt ggggtagatt aggggagatt ttttatttag ggattttttt 8280
tttttttttt aggtcgtttt ttcgtaaaat gttgaggatt ggttttcggg tttgggttat 8340
atgggttatt atttttggtt tggggtttat aggtagagat gttaatggag gtatataggt 8400
agtcgtattc gtattcgatt agtattaggt ttgaggtttt cgggtttttt tatgttttat 8460
tttattttta tttattagga gtagaggacg tcgtggtggg tagagttttt agtttgggag 8520
ttgtatagtt tcgcgttttt ttttatggaa aatttgtttt tttaggttgt ttggttgttg 8580
ttatataaat atttttgttg tttttaggcg tttttttttt tggggcgttg attttaagat 8640
gttattttag gtttgtagaa gtgaggttag attttgggag gacgtaggcg agggaggttt 8700
ttagtagtag ttataggggc gggataggtc gtttgtattg gttgttttta gagtgtttag 8760
tatttggtaa gaggtgtgtt aggaaaggtt tgttggggtt aggtgtgaga tttgttttgt 8820
tttttgtgtt tgtttttgtt attatgtttt ggggtttttg tagttttttt tttaggtttt 8880
tatagtttta ggtttttttt agtttttcgg tttttttcgta gggtaggttt agttttttta 8940
ttttaggagg ggcgtcggga tacgcgtttt ttgttatcgt atttattatt ttgtgttggg 9000
gtggggaggg ggcgttgttt gtttttggta cgaggtcgtg aattttgcgt agggattatg 9060
gtaggtattt tggattttttg gcgtttaatt aggtttggta tagggtgttc gtgtttggta 9120
tcgtggtgga gaaataaatt agtcggaagg agtacgtggg aagggttgcg tcggcgttag 9180
cggtagattc gttcgattcg tttgtgtttt ttggggttat tttaggtagg cggcgttcgg 9240

```
gtaggaggag gacggtgatc gaggagcgtg tcgacgcggg ttttttttgg gggagcgggg    9300
tataattcgt tcgcgaggta ttggtttcgg ttgttttttc ggggcgttcg gagtttttttg   9360
tttagaggtc gcggcgtttt tatttttcgc ggttaagtgt tcgggtaatt acggttaggg    9420
atgtttggcg gcgatcgatt ttttggacgt ttaaagtcgc ggttacgggg ttttcgggag    9480
ggagtgaagt cgttgggtta ggggcgtata tacggttagg ttattttttt agagttttgt    9540
ttcgggttcg gggtttttta acgtggtttt agttgttttt cgttcggttt agcgtacggt    9600
gtatacggtt gttcgcggtt tcgttttttt tatttcgttt cgggtttttt cgtttattta    9660
tatgtaaatt ttagtcgtta gttgtcgtcg agcgcggtaa tcgttagagt cggatttttt    9720
cgtagtttcg gtttaaattt ttggttttttg aaaattttta aacgagaagt agttttaggc   9780
gttcgttttc gatttacgtc gcgtcgtcgg gttttttttt ttcggagagg ttgggttcgg    9840
gacgcgcggt ttagttcggg gaggcgtaaa ggcggacggg gcgtgcggga ggaggtggtc    9900
gcggaggggg cgggggggatc ggcgggggtg gggtcgggcg gggcggggtc ggcgggggcg   9960
gagcgtattt cgattttgag ttttattagt gtttcggtcg cgggagggag cgtaagggcg    10020
cggggcgcgg ggcgcgggcg cgggcgcgag cgtagcgaag gaacgagtcg ggcgcggagt    10080
cgggttcggg ggtttgcgag agtatagcgt cgttagttag tcggggaaga gagggcggga    10140
tcgttcgtcg tcgtttcggg atcgtacgtc gcgcgtgtgc gttttagttt cgtcggttag    10200
cgtaggaggt cgtcgttcgg gcgtagaggg tagtcggtgg ggaggtatgt cgtcgttttt    10260
ggcgtttttg ttttgtttgg cgttgttgtt cgcgttcgtc gtacgaggta ggcgtttatt    10320
tattcgcgag tttttatttt tcgcgttttt tggaaatttt ggcggcgttc ggcgcgcgcg    10380
ttttacggtt gggagcgggc ggcggggagg ttagtatgga gagggaaaag cgggcggttc    10440
ggggcgtggg gttttggagt ttcgggatta gggaggatcg attttttttt tcgatttttt    10500
cgtggaggcg gattcgcgtc gttcgtgttt ggagtcgagt taggaggtcg gtgtggggtg    10560
ttggggtttc ggaggtttta tttcgggttc gttttttatt cgtcgcgcgt ggggtttgtc    10620
gtcggtcggt cgggcgggcg ggttgtttat tatttttcga tttgaataga gtcggttttg    10680
gtttttttgtt gttttttcgg gttatttatt tttttttttt ttcgtttttg gtttacgtcg   10740
gggcggatgt tggggcgcgg agtgtgggtt ttgcggcgtc gcgttcgttt ttattgattc    10800
gcgcggtcgg gttgggtttt cgggttttcg gtcgtttcgt tcgtcggtgt tttttagttc    10860
ggtttttagt ttgggggagg ggttgcgtaa gaagtcgtcg cgttcggggg gattgaattt    10920
ttttttttgtt ttgcggagtt gaagtttgga aagtttgggg gcggagagcg ggacgcgggt   10980
ggggggtttt tatatttttt ttcgtcgtat agcgagcggg gtttttgggg aatcgagtga    11040
ttaatttatt tttttttcga gagttggagg cgagaattat ttgttttttt ttagaaagtg    11100
cggttttgtg ttatattttt ttttttcgtt ttttagtttc ggtaagatgg ggagggaggg    11160
gtttgagtaa ttgatttttt ttcgagatgg ggtcgaattt ttttcgaatg ggggattttt    11220
attttttttt tgtgggtgta tgggggttgt tttggagatg cgcgttcgcg gaggtaggta    11280
ttgggtgtcg gcggaggcgg ggtcgcgttt ttagggtgtt gtttcggtgt ttttggaggc    11340
ggtttcgatt ttataatggg tcgttttgat tttgaggcgg aggtcggcgt tttgttggcg    11400
ggcggggtag cgggcgagta gttgtcgtat ttttttacgg gcgggagttg agtgtggtta    11460
tttttttttt tttcgtttag ttttgttttt ttgtagaacg ggtttgaata gaggtaattt    11520
cggcggttgg atggggggtt ttgttttgtt agatttttta gtgagttttt agggtggggg    11580
gtaacgtttt ggagagtagt ttattttgtt tttgtttttt ttttattgcg tttaggcggt    11640
atattaggtt attttatttt gttttaagta ggacgtgttt tttgttgttt tttttttttt    11700
tatttttgat tatagaattt tgggtagaat gttgacgttt ttttggtttt atggaggggt    11760
tttttgcgga ttcgtgtttt agtaatttat gatattgagt agagtgcgtt cggggtaggg    11820
ggcggacgat gttttttggt tgggtgtagg ttttcgtttt tagggtaatt agggttttcg    11880
gtggtggtgg tgcgggtgag attgattttt ttttttttgt ttttgtttag gttcgcgatg    11940
tttttagttc ggtgagattt gtttgaatgg cgggaagtgt gaagcggtta atggtacgga    12000
ggtttgcgtg tgagtattat ttttgcggga tttgttgttt tgtcgagggt agagtttttg    12060
ttttttgtag tcgcgtaggg gatagaatat taggttattg ttttttggag atggtttaga    12120
gtttgggtac ggttacgtgt tgatttttat tggataaagt ttggtaatta tttaattatt    12180
agtaattatg ttgcgtgtgg agttggtttg gttgttgagg gtttgggtat tttttcgtgg    12240
gttattttag gtttcggggt tattaagtgg gagttttagt attttaattt ttttttaagt   12300
ttttaagaaa tagttgattt gttaggggaa gttttggatt atttattgag aggtttagtt    12360
tttattattt tgttaatttg gggcgttttt tttgggcggg tgatgttttt tgggtatagg    12420
tgatgttttt tttcgggggt gatgtttttt aggtgggtgg tgatgttttt tgggttggta    12480
gtgatgtttt ttttggtata ggtgatgtta tttttttgggc gggtgatgtt tttttttgggt  12540
gcgtgatgtt ttttgggttg gcggtaatgt ttttttttggg tataggtgat gttatttttt   12600
gggcgggtga tgtttttttt gggtgcgtga tgtttttttt gggtacgggt gatgttattt    12660
tttgggtggg tgatgttttt tttgggtggt gatatttttt ttaggtgggt gatgtttttt    12720
ttgggtggtg atattttttt taggcgggtg atgttttttt taggtatagg tgatattttt    12780
tgggtaggtg atgttatttt cgggtgggtg atgttttttt aaaggaaatt gattttttaa    12840
aatcggattc gtttttgggt tttttgtttg ttttagtttt tttttttcgg ttttaggttg    12900
```

gggttgggga agagaaggtg gaatttatgt tttggcgtgg ggtgtggggt aattattgag 12960
tttggttttt gttttggtat ttgtaaattt tgattttaaa atttcgtggt ttttttggtt 13020
tgttagtttt agatgtaaaa tgggtattga gggggtcggg aagtcgtgcg gaatcgtttt 13080
tttttgtttg tttgtgagtg tttattagtc gtggggaatt gttttgttgt ggtcgatgtt 13140
ttttaatttg agtttatttt tgatgagaat aatgaggagg tgtttggtgt ttggaaaaag 13200
agttttttc gtttttttagg agttatagat gaagaaatgg ttttttagtt agtttgcgcg 13260
tttttatttt aattgtagaa ggttttattt ttttgataat tttagttcgg tttttgtttg 13320
tgtcgattgt ttagtttgtg ggtagtaggt tttttttttt ttcggttttt atttttttta 13380
aattgaagtt aattaagtag gtttgtgagt agtttaatgg ataaagtcga ttgtgttttt 13440
gttagatatt aatgaatgcg tattattttt tttttttttt ttttttttgt tttttggggt 13500
tttttttttt ttttgtagaa gtcgtacgga cgtttgatgt tttaaatttt gttttttttt 13560
atttagagat cgatagttgg gatttagggg aagtgtagta gttattgttg ggggaggcgg 13620
attaatgtcg tgtgattaat tatgcgtggt tttttagaat gtataatttt tttttcgtt 13680
attgcgtttt cggttcggat tttgtttttt atttattcgg ggttttggga gaagtttttt 13740
gtttttgtag aggattttgg gagggtggga ggaatatcgt gttgggaaaa ttgggggttt 13800
gttgtttttt tttagtggta ttagggagta ggtatagttt ggtttatagg ggtttgggat 13860
tgaggttttt ttgggttaga gcgttgtgtt ttggggtata gattgttgta gttttttggt 13920
gtttgggtta aaattttgtt atagggtcgt aaaatattgg tgttatgggt ttattttttt 13980
attttttaaa tttgggattt cggtgagttg tttttagttt aaggtaggtc gtcgattatg 14040
gttttggtgt ttggttttgt tttttttgtt ggggtttggc gttttttttt ttttggcggg 14100
aagtttgttt tagtttggtt tgataggtag tcgagtttag tttatagaga ttttattgtg 14160
tatattttat aagtttaacg agaagatggg acggaagcgc gtttttaggt ttttgttttt 14220
gggtcgtaga gttggaggtt agggttatag tcgagttcgg tttgtggagg tataggttag 14280
ggtttatata gtagtttggg ggatattgta gtcgtttcgg agaaggtttc gggttttttt 14340
tttgggtttg agttttattt aatatatatt ttttaggtgt ttttgttttt tcgtttttgt 14400
ttattttttt tagaaggttt aggaggtttg tttaggagtt agttagggta tttgtaggag 14460
gtttggtttt tttagtaagg ggagaaattt atattgtttt tcgaaagagt ttggggtgta 14520
ggtttttgga gttttgttag tttgagtggg aattttggtt tcgtggtatt tagttgtgat 14580
ttcggaaaga gttttggttt ttggtgttat ttgttgtttt cgttgtagaa ggggtatggt 14640
tatgatgggc gttatagtgt ttggtttatt agtattgggt gaattatttt tttttatttt 14700
aaagagaagg aatttttatc ggaagttgtt aattaggtgg ttgtagggtt cgggattgga 14760
aaaagtggtt ttgttggtgt aaaatgtttt tttaggaggt tagagtgtcg tcgtggtttg 14820
gttagttagt ggttaggtag aaagtatttg gtagtatatg ttggtggcg gaggatggtt 14880
ggtttttggg tagttgttgt gcgaggtgtg gtcgtggtgg ttgtggtcgg agatggtggt 14940
tttttgagta ggtttagggg gttattgtgg tttttggttt ttttttagtt tttttattag 15000
tttcgttagt gtgagtaagg ggtattgaag agggttttat tttttttttt tttgttttga 15060
ggagagttgg gaagagttgt agggatttta tttgtttggg tgagattacg tttttggttt 15120
gaaacgtttt tttagggttg tttgcgtttg ggttgggcgt tgaggtaggg ggtagttaag 15180
atgttggttg ttgacggtag agttagaatg gtacggggcg ttttttagga gttgggagaa 15240
atgggggtag gttgttatat ttagtatttt taggaggatg tttagttgtt tgttgggtgg 15300
tgcgttttag ttttggataa tttcgtgttt gaggtttggt ttggttatta gttttgtgat 15360
tttgggttat taggttatgt aagttttagt ttttttagta tgttagtcgg gttgatatac 15420
gttggatttt ttggtggtgt gtgaggattg tatgagaatc gtgtggagag ggtatttggt 15480
tagcgttttt tttttttttcg tttttttttc gttggttgat agtgttggtt tttagagttt 15540
tttttttgaa tgtttaattg attgacgagt gaatttagag aattttaaag gtgttgtttg 15600
gtgggttggg tttggtattg tttggggagg gatttgtcgg ttttggggaa gtttattttt 15660
taggatggtt ttagtttcgg ggaagtttat tttttagggt ggttttagtt tatagatacg 15720
tttgagcgtt tgttgtgtgt tgcgttttgt tttaggttag gttttcggtg gggatagggt 15780
agatatggtt tttgtttttg tggagtttat tttttagtaa ggcgtgagag aggtatttag 15840
tttatatgtg ggatatgttg ggggtgtgtc ggttcggtgg tggtttggta tagttttcgg 15900
tagtttcgta aagttgtttg gattaggtat cgataagtga gaagaagaga tttagagaag 15960
tcgttattag ttttagggtt atatagtagt ggtagaattt ttattagttt tgtttttttt 16020
tttttttttaa gcgaatgttt ttaaatatag ttttagttag tttgagttgt ttcgttattt 16080
ttcgattata agcggattgg gggcgtggtt ttttttttaaa agaagaggaa ggaggtttag 16140
gcgggaagtg atttggtttt gtagtcggtt tgggaggttg gggagggacg gggtagtttt 16200
tgttattcgg tttggttttt tttattgagt tatttgtttc gttttgggcg tggttagggg 16260
aggaataggt tgattttttt ttttatgttg agttgagcgg aaaggtttgt gataggattt 16320
tttgtttatg tagaatttgg tttttaaggg cgttagggtt agaaaaatat gtttaaaaat 16380
atgtagtatt cgaggtgggt agattatttg aggttaggag tttaagatta gtttggttaa 16440
tatggcgaaa ttttcgtttt tattaaaaat ataaaaatta tttaggcgta gtggcgtgtg 16500
tttgtaattt tagttatttg ggaagttgag gtaggaggat tttttgaatt taggagacgg 16560

aggttgtagt gagttgagat cgtgttattg tattttagtt tgggtgataa agtgagattt 16620
tgttttaaaa aaataaaaaa taataaaaaa aattttatat agtgttaaat agaaagtttt 16680
atttatttag attggggaga tattaggaat aggaaggttt ggggcggaga ttggggtggg 16740
agtttggagt gttttgaagg aagttggttt tttggggtag gttttgtttt tgaatagtag 16800
ttaatatgtt ttttattgaa aagaataata atagaaaaag agagagggag aaagaaaata 16860
gtaattttgg taggaagagg gtagggtagg gtagatagtg tattagggga agtgttttta 16920
tagggtagag ttgggtgcgg ggattttttag atgcgggcgg taaattggag attttatagg 16980
agtcgggatt ggttttattt tttatttttt gtatttgtga tgggtagtag cggttatcgg 17040
tgtttattgg gtagttttag gtcgggtcga gggattgtat ttttttgttg tggggagggg 17100
atttttttttg ttttatgttt cgtttataaa tagtgaaagg atgtggtttt gtagaatcga 17160
ttgggcgttt agtttgtttt ttttaataat gtagcgatga tagttttata tttaggacgt 17220
cgttgttatt tagtttcgta aaagttttgg tagacggtcg tttatttttt aggtgtgtta 17280
gtgtagtttt tatttttagc gtataggtgg gaaagttgtg gttgcgaggg cgtggggatt 17340
tgaggtaggt tggcgttagt ttttattttt atgtgtaggt atatttagaa tttttcggat 17400
ttttttttcgg gttgaggagg aaatattagt agttttaacg agcggttggt tttggggtat 17460
tatatttgtg ggtcgggttg cgtttgacgg tttcgagttt tttaggtagc gttggcgttt 17520
tttttttgtgg ggttcggagt ttcgaggggt tgtgaagtag ggttggtttt gatttgttgt 17580
gtttttttata ttgttttttt tttgggtttt agttttttta tttgtatgtg attttttttt 17640
taattatagt tagcgggatt ttggggcggg ttgaaggcgt ggtggtggtt gtgggttttg 17700
cggcgaggtt tgcgttgggt tcggtggtcg ttttagttgg ggaaggggta gtggttgtag 17760
gggtgggagg gtggagggaa gttttttttt aaagttgttt ggttgggttt tttttgtggt 17820
tttgttttat tttattttgt tttttgggag taaagggagt taaggattgg tttggggatg 17880
gatatgtata tggagggatt ttgggtgatg atttatttat attataaaat ttgttttttt 17940
ttgtttgttt tttgagatgg agttttgttt tgttatttag attggattag agtgacgtag 18000
ttttggttta ttgtaatttt tgttttttag atttacgcga tgtttttgag tagttgggat 18060
tataggtata tattattatt tttagttaat ttttgtattt ttagtagggg tagggtttta 18120
ttatattggt taggttggtt ttaaattttt aattttaggt gatttatttg ttttggtttt 18180
ttaaagtgtt gggattatag gcgtgagtta ttgtgtatgg ttattttttt gtttttttgag 18240
atggggtttt attttgttat ataggttgga gtgaagtggt gggattacgg ttgattgtag 18300
ttttaaattt ttgggtttaa gcgatttttt tgttttcgtt tttagagtag ttgggattat 18360
aggtgtgtat tattatgttt agttaatttt aaattttttt gtagaagcgg ggttttgtta 18420
tgttgtttag gttgaaaatg tgattttttga attaaagtat gttgttgttt gatatttagt 18480
ttatttataa gtttatttaa ttattattta gtttcgggat ttttttattg tataaaatag 18540
aaattgtatt taaatattgt tattttgttt tgattgtttt agtttttggt aattattaat 18600
tcgttttttg tttttgaatt cgtttttttt ggacgttgtt tatgaatgga attgtgttat 18660
aggtggtttt ttgtttttgg ttttttttat tgagcgtgat gtgtttaagg tttatttatg 18720
ttacgcgtgg attagaattg tattttttttg aggattatat tttagttttt agaagtatta 18780
gggattggtt agaggtggat tttagtattt tagtttttag atagtttaga ggaggtaggt 18840
gttgggaggg cgttcggggt ggttttttgg aagagatggc gtttattttg ggtcggtgta 18900
gttattcgat agttatagag ttttttagag ggagttttgg tatattttta ggtgaatttg 18960
taggatggat tggcgtgttc gcggttttttg ttttgtttag gatttcgaga taggtttata 19020
atttgttttt gggttttatt tatgttagtc gttaagttgt ttagtatttt ttagttttta 19080
ggaggtatat agtggggtcg cggttattga gcgtttatac gtttggtgtt gtaagaatgt 19140
aaagtagagg gtttttattg agtatttatt atatgttgaa ttttggggta tgggtgggta 19200
cggtagatgt aggttttttt gggtagggga ggagtatgtt ttttaagaat taggttatag 19260
gatggacggg tttggttata agttaggggt agttattgta gtttttatgg tttttttgtgt 19320
gtaaagttag tgttgtggga ttttatttgg gtgtatttgt tgtggttttt aagtgaggtt 19380
tattacggag tagtttttac gttggttggg atagggttag cggtagggag ttttagatta 19440
aaggagcggc ggtggttggg gtagcggggg gtagtgaggt tttggatata tttatggggc 19500
gggtaggtat tttagttttt attcgtttag tttgtttgtt tcggggtttt ttttttttttg 19560
gatagagttt tgtgtgattg gttttaggtt gttttttttt ttggattttt agggttagtt 19620
tagagtgatt ggttatgttt tggtttagat ttaggttcgt gtgataggag agagttagga 19680
atttggtttt ttattttgag atatagatgg tgtttttttt aagttgtttt tgggacgggg 19740
aagcgttatg ttttttgtag ggtattaggt tagttagtgg tttttgatag cgaggttttg 19800
tttgtaggta gttgggggat tttttttttgt cgggaggatg cgtgcgtgtt agtatcgggg 19860
tgtttggttg ttttaggggt ggtgtagggc ggttataggc ggttttagta ttttggggtt 19920
gtttttagtg tattttcgag atttgtggaa ttttgggagg agaaattttt tagtagtgtt 19980
tttagtgcgt tttttttattt tgtattcgtt gtttttggga gggattttga gtgttttttt 20040
ttatttatta tgtatttatt gttttaggag ggattttgag tgtttttttttt tttatttatt 20100
ttgtatttat tgtttcggga gggattttga gtgttttttt ttttatttat ttttattttg 20160
tatttattgt ttttgggagg gattttgagt gatttttttt ttatttattt tgtatttatt 20220

gtttttggga gggattttga gtgttttttt ttttatttat tttgtattta ttgtttcggg 20280
agggattttg agtgtttttt tttttattta tttttatttt gtatttattg tttttgggag 20340
ggattttgag tgtttttttc gatttatttt gtatttattg ttttaggagg gattttgagt 20400
gttttttttt ttatttattt tgtatttatt gtttttggga gggattttga gtgttttttt 20460
ttttatttat tatgtattta ttgtttcggg agggattttg agtgtttttt tttttattta 20520
tttttatttt gtatttattg tttttgggag ggattttgag tgtttttcgt ttattttgta 20580
tttattgttt ttgggaggga gatttttgta gaggttttat ttttgaattt tttatttata 20640
ggtttagggg tttagggttg ggggcgtttt gtcgtttata ttttagattg agttcgtacg 20700
gttaaatagg gtttttttcg tacgttttgt ttaatgtaaa gcggtggttt cgggtgggtt 20760
tttaaggggt tttgtgaatt ttggggggttt ggtttttttt atttggttgt attcgttggg 20820
cgggagattt tatttttcga gtttttagtt ttttagtttt agttgtagtt tagatattag 20880
ggttttgtgg ggcgggggtg gggtagcggt ttttttaggt ttgtggggtt gttttgttgt 20940
ggtttttgta atttttttgga aggaggtttg gggtttggtg tttagtgggg tttgtgggga 21000
cggattcggg ggttttgtgg ggagggattg ggggttttgt ggggacggat ttgggggttt 21060
tgtggggagg gattgggggg ttttgtgggg agggattggg ggttttgtgg ggagggattc 21120
gggggttttg tgaggaggga ttggggggtt ttgtggggag ggattggggg ttttgtgggg 21180
agggattggg ggttttgtga gagggaattg gggggttttg atgtgttggg cgtgtatagg 21240
ggaggttttg tttttgatat ttatatttgt agtttttagg tatatatagc ggttttatcg 21300
gggttttcgt aattatagtt tgtgagtggt tattattaag ggcgttttcg gattaggtta 21360
ggtttagatg ttgttcggat ttaattttta tagcgtttgt cgttatagta gggattaatg 21420
gttatagaat atttattcgg ttttatttgt acggggttgt ttagtttggt gggtattagg 21480
tgggcgttgt tttcgtttag tgggaggttt gttagttttt tggttgttag ttgagatttt 21540
tttttaagaa atagtcgtgt tgtttttttt gtttcgtttg gtattgaggt tggtgatacg 21600
tttcgatttt gttttaattg gtagatgaga atttgttatt agatgtcgat tttgtttacg 21660
tagttttttg gtttcggagt ttattgtgta tttttgaagg gtttgaattg gttgtttaaa 21720
gttgggtaaa tgtttttttg atattttgtt gatattgtta atttgttgaa taaatttttt 21780
tataggtagt ataggaggtt agtttcgttt tagtttgagt taaggtttag ggaggtaggt 21840
tttgtgtgga gttagttttt ttgtgagtgt agcggttttg taggatgtat cgggtttgag 21900
aagtggattt tttcgttttt cgttatattt taaaagtttt tagttatgag ggtaggacga 21960
agtcgacggt cgttttcgtg tcgagatgat aatcgggtat agaggcggtg gtttttagtt 22020
ttttgttttt tttttcgtag tggggttatt tagcgtgatt tatggttcga gttataggta 22080
ttcgtttagg gagttacggc ggggagattt ggttttatta gagatgcgtt cgtgttttat 22140
taagtttaaa ggaggaaatt ggcgcgttt 22169

<210> 147
<211> 3286
<212> DNA
<213> Artificial Sequence

<220>
<223> chemically treated genomic DNA (Homo sapiens)

<400> 147

agaattcgcg gttaatttta gaggattggt gagggttggt ttgggatgaa taaatattcg 60
atatttgacg gttttttttt tgcggtgatt tttattaagt cgaagtggcg cgcgcgcggg 120
taggcgggag tcgggatttg gttcgcgcgg cgtcgcgtat tcggatttcg aggttacgtc 180
ggcgagcgcg gcgcgggggg cggggagcgg gatagcggga tcgttcgggt taagcgattg 240
ttttaaagat attttttatt aagatttttt tgtattcgag ttttattttt ttttggttta 300
ggaaagtttt cgtgtaagtg gaagacgatt ttttttataa agtgttgtat gttattgtgt 360
ttcggatcgc gtacgaatat tatttagtcg tgggtgaagt tttttacggt gggttttttt 420
tttatttggg cgcggtgttt tagttttagt tttatttgta cggtatattg cgggtagggg 480
gaggagagat agtcgtgaat aataggaagg cgaggtttcg gtagtgaacg ttgcgtttga 540
tatttttttt tttttttttg aaacgtatat aaaaggaaac ggcggtgttt tttgtattta 600
cgttttacgc gcgtgggcgc gtatatttta tatttttaaa tatattcgtt cgttcggttc 660
ggtttggttt taggcgtttc gggtttcgta tttatatcgg ataggattgt aacggaatgt 720
tatttttagt cgggaagggg gtcggggaaa agagggagaa tatatttagt tacgataagt 780
acgataataa atgtatcgga aataaattag aaggcgatgt cggggcggtt tttcggcgtg 840
ggaggggagg tggttgggat ttaggggggat cgaagggttt ttggcgagtt tttttttcga 900
aagtatcgcg gcgataggta tacgtcgagg aagtttttgt gtacgtgtgt gtgcgtgcgt 960
gtggagcgtt gtgttaggcg aaatggaaat tataggtagt ttttgttgat gggtataaat 1020

tttcgtgttt ttgttacggg gttcgtgtat aataaaaatg agacgtcgtt atatatattg 1080
aaatatatat atacgttcgt aggaaaatac gtcgaggtat ttataattta gagtatttcg 1140
tattttttag cgaaaagttt tacgcgatcg gcgaggttag tcgaatcgag ttttcgtaat 1200
atatttttaa gagtttaagt ggttttaaag tatttttat ttttttttaa aaaatgaaat 1260
ttagaaaggt agggcggcgg gcggatagtc gtcgagtttc ggttcgcgtt tagtattttt 1320
cggcgttggg gtaaagttgc gtgcggtttc gtcgttgtta gtttcgtata tttcggttta 1380
tatacgcgcg tcgcggagaa cgtattatcg tagcggtcgc ggcgttttgc ggaggtgcgg 1440
tcgtcgaggt tgttcgtcgc gttttcggat tgtgttcgta gttttcggcg gcggcggttg 1500
aaatatggtt gagttattat tcgttttttt ttatcgtgtg tgtgtgtgtg tgtgagtgcg 1560
cgcgtgtgag cgagagggag tgtgtgagtg cgttttttgt gattgtaaag aggcgaggag 1620
ggagggaggc gcggggggtg gaggggcgag tgtgagcgtg tgtgagtgtg tgtgtgtttg 1680
tgtgtttgtg tgcgcgtcgg ggggggtgg gggaggaggg gagcggaggt tgagggagag 1740
gcggtagttt tttgtaagtc gtattaattt ttttttaatt ggaatcgcgg agcgttggcg 1800
ttgtttgggt tgcggcggcg tagggcgagg aaggaaagtg ggggagggtt tttattatta 1860
tttttgcgcg tatttatcga gttagttatg tttgggggtt cggaggtttg ttggggtgtt 1920
gtgtggtatt tttttttttt tcgttttttt ttagttgtaa tttatgaaga ggttgttatg 1980
aatgagagcg cgtttaggag cggagggtag atggcggata ttttttgttt tttttttttc 2040
gcgttcgttt gttcgttcgt tcgtttatta aatttagttt taaaagtaaa agtagtagta 2100
gtagtagtag ttttagggta aagaagatga ttgcggagta tgcgtcgtgg cgtttgatat 2160
tcgggtttcg agttttatag ggcggaggga gggtttgcgg gcggggcgga gcgcgcgggg 2220
aggtagagcg agcgagttag ggggagttcg aaagagttaa ttatttgtta tttttcgaaa 2280
tttcggcggt tcgtagcgtt atattggaaa gtttcgttta aagtaatagg tttttgttgg 2340
gggggtggta gggaggggac gtaggagtgg ggagagcggt tagttgggga gttattttag 2400
tttttgtgta aattttgttc gttttataat aaaaaacgga ttaaaattgt tggaatgtag 2460
ttattgttta gagtttttag tagtcggaga gggggtgtta aattaagttt ttagttagta 2520
cgtacgggga ggagggagtg ggttttttt tattttttat tacgtatttt tgtggttttg 2580
tgttgaaaag ataagaattt atatttaaat aaatttattt aaagagagcg agagaaatgt 2640
tattttgtaa aaaacgagta tgaaaatagt aatgattgag gttgttttaa ttcgtaacga 2700
ttttttggtt ttttgtttta tttatttata ggtattcgtt cgttattttt atttttcgtt 2760
tttttgttat cgttatcgaa aatttgaaaa tgaaatgaat aagggagaaa taaggatgtt 2820
atttttttgt aagggaacgt ttttcgtttt ggtaattttc ggttgggaag ttttataaag 2880
cggtgtttag gtgaaagtaa gatatttggg agtgtcgggt atagattttc ggggattagg 2940
tttggagttg ttttttcgg ggggtttttt tttttgatag tttattgagt tgtattagaa 3000
cgtggaggat tgttttttga ggacgcgcgg ggtaatcggg agttttaaaa tagtagataa 3060
agttaatatt gattgtaatg tgcgtaaatt ggattataat ttttaggcgt attggtgata 3120
attagcgtaa tttatttttg agtattaatt cggttagatt ttcgggtgat agttagtcgt 3180
aggagtgttt ttagagtcgc gtttttgtaa atgtttttat tcgcgttgta tttgatttta 3240
gtggtcggag atagaatagc gcggttacgc gtggggcgcg gagagg 3286

<210> 148
<211> 3286
<212> DNA
<213> Artificial Sequence

<220>
<223> chemically treated genomic DNA (Homo sapiens)

<400> 148

ttttttcgcg ttttacgcgt ggtcgcgttg ttttattttc ggttattgag attaaatata 60
acgcgagtga gaatatttgt agaggcgcga ttttgggaat atttttgcga ttggttgtta 120
ttcgggagtt taatcggatt aatatttaga gatggattac gttggttgtt attaatacgt 180
ttaaagatta taatttaatt tacgtatatt atagttagta ttaattttat ttgttatttt 240
aagattttcg gttgtttcgc gcgtttttag aggataattt tttacgtttt gatgtaattt 300
agtaggttgt taggagggga agtttttcga agaaaataat tttaagtttg gttttcgagg 360
gtttgtattc ggtattttta ggtgttttgt ttttatttgg gtatcgtttt atgaagtttt 420
ttagtcgggg attattaaag cggaaggcgt ttttttgtaa ggaaatagta tttttgtttt 480
ttttttgttt attttatttt taaattttcg gtggcggtgg tagaagggcg gggagtgagg 540
gtggcgggcg ggtgtttgtg agtggatggg gtagggggtt aggggatcgt tgcgggttag 600
aatagtttta gttattgtta tttttatgtt cgttttttgt aggatggtat ttttttcgtt 660
ttttttaagt aagtttgttt gggtgtgagt ttttgttttt ttagtataaa attatagaaa 720

```
tacgtggtgg aaagtaaaag aaagtttatt tttttttttt cgtgcgtatt agttaaggat     780
ttgatttaat attttttttt cggttgttga gggttttaag tagtggttat attttagtag     840
ttttaattcg ttttttattg tagggcgagt agagtttata taggggttgg gatggttttt     900
tagttggtcg ttttttttat ttttgcgttt tttttttgtt atttttttag taggaatttg     960
ttattttaag cgaggttttt tagtgtggcg ttgcgggtcg tcggggtttc gggaggtggt    1020
aggtgattgg ttttttcgag ttttttttgg ttcgttcgtt ttgtttttttc gcgcgtttcg    1080
tttcgttcgt aagttttttt ttcgttttgt gaggttcgga gttcgggtgt taggcgttac    1140
ggcgtatgtt tcgtaattat tttttttatt ttggagttgt tgttgttgtt gttgtttttg    1200
tttttggggt tgagtttaat aagcgagcga gcgagtaagc gagcgcgggg ggaaaaaggt    1260
agagaatgtt cgttatttat ttttcgtttt tgggcgcgtt tttatttata gtagtttttt    1320
tatgaattat agttgagggg gggcggagga gggggggggta ttatataata ttttagtaaa    1380
ttttcgggtt tttaggtatg gttagttcgg taagtacgcg taaaaataat aataaaagtt    1440
ttttttttatt ttttttttttc gttttgcgtc gtcgtagttt agatagcgtt agcgtttcgc    1500
ggttttaatt agagaaaggt tggtacggtt tgtagggagt tgtcgttttt tttttagttt    1560
tcgttttttt ttttttttat ttttttttcgg cgcgtatata gatatataga tatatatata    1620
tttatatacg tttatattcg ttttttttatt tttcgcgttt tttttttttt tcgttttttt    1680
gtagttataa gaagcgtatt tatatatttt ttttcgttta tacgcgcgta tttatatata    1740
tatatatata cggtggaagg aggcgaataa taatttagtt atattttagt cgtcgtcgtc    1800
gggagttgcg ggtatagttc ggggacgcgg cgagtagttt cggcggtcgt attttcgtaa    1860
agcgtcgcgg tcgttacgat ggtgcgtttt tcgcggcgcg cgtgtgtgag tcgaagtgtg    1920
cggggttgat agcggcgggg tcgtacgtaa ttttgtttta gcgtcgggag gtgttgagcg    1980
cgagtcgagg ttcggcggtt gttcgttcgt cgttttgttt ttttgaattt tattttttgg    2040
ggggaggtgg gagatatttt ggagttattt gggtttttga aagtgtgttg cgggggttcg    2100
gttcgattgg tttcgtcgat cgcgtggggt ttttcgttgg ggggtgcggg gtgttttaag    2160
ttatggatgt ttcggcgtgt ttttttgcgg gcgtgtgtgt gtgttttagt gtgtatgacg    2220
acgttttatt tttgttgtgt acgaatttcg tggtaagagt acgggagttt gtgtttatta    2280
gtagaggttg tttgtagttt ttatttcgtt tggtatagcg ttttatacgt acgtatatat    2340
acgtatataa agattttttc ggcgtgtgtt tgtcgtcgcg gtattttcgg ggaggggggtt    2400
cgttaggagt ttttcggttt ttttgggttt tagttatttt ttttttttacg tcgggaaatc    2460
gtttcggtat cgttttttga tttgttttcg atgtatttgt tgtcgtgttt gtcgtggttg    2520
agtgtgtttt tttttttttt ttcgattttt ttttcgattg gggataatat ttcgttataa    2580
ttttgttcga tgtagatgcg gggttcgggg cgtttggggt taagtcgggt cgggcgggcg    2640
ggtatatttg ggggtgtgga gtgtgcgcgt ttacgcgcgt gaaacgtgga tgtagagggt    2700
atcgtcgttt ttttttatgt gcgttttaag aaggaggaaa aaaatgttag gcgtaacgtt    2760
tattgtcgaa atttcgtttt tttgttattt acggttgttt tttttttttt tgttcgtagt    2820
gtgtcgtgta ggtgaagttg gagttggggt atcgcgttta ggtgaggaaa aaatttatcg    2880
tggagggttt tatttacgat tggatggtgt tcgtacgcgg ttcggagtat agtaatatat    2940
agtattttgt ggagaaagtc gttttttatt tgtacgaaag ttttttttagg ttaaaaagag    3000
gtagggttcg aatataaaag ggttttgata aaaaatgttt ttgaaataat cgtttggttc    3060
gggcggtttc gttgtttcgt ttttcgtttt tcgcgtcgcg ttcgtcggcg tagtttcggg    3120
attcgggtgc gcggcgtcgc gcgggttagg tttcggtttt cgtttgttcg cgcgcgcgtt    3180
atttcggttt ggtgaaagtt atcgtagaga aaggatcgtt aggtgtcgag tgtttattta    3240
ttttaagtta gtttttatta gtttttttggg gttagtcgcg ggtttt                  3286
```

<210> 149
<211> 23717
<212> DNA
<213> Artificial Sequence

<220>
<223> chemically treated genomic DNA (Homo sapiens)

<400> 149

```
tttttgttgc ggtattttag tatgacggag gtttattttt ggtttttttg gagtatgaat      60
attttttgag gagtattggt tttggttttg taggttttgt tgagattggt ttagattttt     120
tggtttagga gtaatcgtta tttaattttt tatttgtaaa tttcgttata gaatcggttt     180
ataatcgttt tttcggaatt tcggcggatt agttttgttt ttgtgttttt ttttttcgta     240
gagttattgt ttttttaggt tttttaagtg tatttaagag cgggagtttg gttgttttta     300
gtattcgcgt ttcgttttgt agtttgataa gtttgacgtt aaggtatttt ttcgagagtc     360
ggtttagtag ttgttggtag gagagtggtt ttagggtagc gggaaggggga ttttcgggga     420
```

```
gagaatgagg tttgcgtaag ggttttggaa ggatttagga gtcgtttcgg tttttttttt     480
ttaggttttg gttgggcgtc gtgggtcgcg ttttagttga gttcggcgtg gtcgtttaat     540
ggcgtttttt cgcgatcgag gacgaagggg tttagagagg ttgtgtttaa gcgacgttgc     600
gaggttaggt cgcgtttcgg gttagggcgg ttttttttgtt atcgtcgttt ttggtcgtta    660
ggattaagta tagtacgtag cgtcgcgaaa ggggcggttt tggaaggcgc gaaggggttt     720
tcgggtttcg cgaatacggc ggggtcgggg cggagtcgtg ggttttcggt cgagcggtcg     780
tttagagttt cgcgggatac ggacggtttt agttcggttt ttcgcgagtt ttacgcgttt     840
gtgacgtcgt tgatgcgtag acgtcgcggt tgtaggtttc gttttttcgt gtttttcgta     900
aggtttttttg ggagttgtag ttttagtttt ttttcgggtc gttttatttt ttgataattt    960
tggagcgcgg cgtttttcgg ggatcgaggt ttcgggttcg gtcgtttttta gtagcgaggg    1020
taggagaagt ggttcgggcg gtcggcggat tgggagggcg ttattcgagg attataattt    1080
tcggtttgtt tcgcgtaggt atcgtttcga gttttcgttt cgtcggcgtc gcgtagtttt    1140
gtgggagtcg tagttcgggc gcgcgattta tagcgtcgcg tgcgtttcgg gcgcgtcgta    1200
gtcgcggggt tcgggtcggg cgttacgtta cgaaggtagc gtaaggcggg cgtcggacgc    1260
gggtaggggt cggggtcgga gtcgggtcgg ggcgtttcgc ggttgaggag cgggaggtcg    1320
ttcgggtagc gcggtcggcg gcgagagggg tttcgtcgtt tttcggaggt agaagttgtg    1380
gatcggtcgg cgggggcgag cgggttcggg ggtcggggtc gcgttgttcg ggtcgcgagg    1440
acgaggcggc gtcgtcgggt tgtggaggtg agtttcgtcg gtcgggttcg gtcgggcgtt    1500
tagtcgggga tttcgggcgg ggagcgcggg ttcggagggc ggcggggagc gcgggttcgg    1560
cggggcgtag gaggtttcgg gggtcgggcg cgtcgggttt cgagttaggg tagggacggt    1620
agggttttcg gagtggggtt agttcgtcgt tcgttggcgt ttttaggttg gtgggggtta    1680
gggttgggcg atgtttcgta gaggaattta gaggaaggcg ggaagtcggt agggatttgg    1740
gttatgtggg ttatttgagg ttagtttcgg gggtcgcgta tatcggaggc ggtttcggtt    1800
ttgtttgttt ttgagtgttt ttatattcgg gtttatcgtg cggttttggt cgttttaggg    1860
atgttgtgtt tggtgcgggt attattttt ttatttgggg aacggtttgg gggcggttat     1920
ttggcgtgga ggggttggcg ggagtttcgt ttgtgttagt tatgttagtg ttttttttcg    1980
tgttttttttg gggttgttgc ggtattagtt gggaagttgt aaatttgttg ttttttcgtt   2040
ggttgtagaa ggggtttttt tagttttgat cgggggaatt tttgtgtggt tagttggttt    2100
tggtcgggcg agttttcggg gtttttttat tgttttggag tttgttatcg gattgtaggt    2160
aataagattc ggaaagtaat ggtgaggttt agaatttgag gttaggtttg ggtgtgtggg    2220
gttagtgggg aagattggga tttcgttggg tgttgaagag ttgtgtgttt ttttttcggg    2280
aaattcggat tttttttttt gtttaggttt gaggggtgcg gaaacgtgag ttatagttta    2340
gtgtgggagt gtttcgtaat tgttttcgcg tttttagagt ttggggttgg gtaaggatat    2400
aggggtgatt ttttttttcg gttttgagtg attttttaat tgtgaagtgg ggtaagagaa    2460
attgtttttt tggtttgttt gggggaggta ggttttgtag gaaattagtt tggtttggtt    2520
ttgggttggg tgtgttttgt ttttttcgag gtttttttgt ttgcgggagg agtatttttt    2580
ttttttttgt ttttgtttgt gtttgaggtt ttttagagtt ttttgattgg tagaggagtt    2640
agtgattttt gagtttttag gagagttcga ggtttagtaa ttgtataatt gcgtagggtt    2700
ttttgtttgg gtttttatag atcgtggttt ttgagagtag gaagtggttg gttttttgta    2760
atgttgtatt tgtgtgatag ttttaagttt tgttggtttt tttttcgggt atttttttatt   2820
tgtttttttgt tttggttttg ggtagtattt cggtaatttt agtgttatag gtgaggaatc   2880
gaagttggag atagttgttt atggtagggg aggggtcgag ggaggttgag cgtttggttt    2940
tttttttttg ttggttttat tagtttatac gaggtaattg gagggaagga ttttgttgtt    3000
cggaaaattt ttcggaatta taggtgttgt ttatgggttt gagtcgttta gtgaagaatt    3060
cggtcgttgg tggtcgagtt gtttttggtt tagttttttg gttcgcggtg tgatgttttt    3120
atttatggta ataggcgtgg ataggtgttg agaaggagga cgatattatt aaataaaata    3180
gtttggggtt tttgaaaaat agtgatagaa atttaggaag gttggtgatt tgttaggttt    3240
gtgtttgttt tgggtggtta ttcggttttg gatcgtgagg ttgaaatgat aggagatacg    3300
gagggtgggt ttgggtggtt tttttataag atatagaaga aggaggtttt taattcgggg    3360
gttgtttcgt gcgttttgtt tttttgtttt tttgttttgt ttttttggtt ttgttttttg    3420
atttttggta tagtaggagt gtttagaagt ttagtgggat ttgttttttg tttaaagttt    3480
tttcgtattt ttcgtattta ttcggtgtgg atgtgggttt ttttattatt aggttgtgtt    3540
ttagttgtgt ggagttattg gttttttatg ttttacgttg gagagaacgt tgaggtttgt    3600
gcggttttta tgtgattttg tagttggttt ttggtttttt attgtgggat ttgtttgtag    3660
tatttatatt tgggatcgtt ttgttttttt gttggatgtg gcgtcgaggg tagggttcgt    3720
ttttgttttt tggtaggttt agtgtttagc gttatgtagt cgtgcgtaga gtaggcggtt    3780
agtgagtatt tggggttggt ttttattata ggtagttttg aatatttggg ttttggtagt    3840
ttagggggat ttttgcgtgt tttaggaaag tagtatagta gtttttgtgg cgtggatacg    3900
gttatatttg agttcggttt gtttttgagt ttgagagaat aggtttgttg gtttgtgatt    3960
tttgtatgtg tgtttcgttg tagggaggtt gtgggttggg gttggattta gattcgttta    4020
gttatatttt gttattatgt gggttatata tagttgggtg gagatttttc ggtgcgtggt    4080
```

ttagggaggt gtttgggcgt ggagtttgtg gatagttata ttgtattatt aggatagggg 4140
ttgttgttgg gtttataggg tgtacggaat ggggtttgtg ggaggatggg ggtattttag 4200
tttagggtag ggatttcgtg ggttttttaa gatgagttgc gttaggttgt tgttttgtat 4260
attttgaggg gtttgtttga gtgttgtatg ggaagttagt agtgtggtcg tttttttag 4320
aatttattta gggtattagg gtggatttat gagtttcgtc gggttatggg tattagtttc 4380
gttattttgg tttttagata taacgatttg ttagattttt tgaggttaaa gggaagtttt 4440
aagggttgtt attttcgttt ttttggcggt tttagacggg atgttaggag gacgtagggt 4500
tatcgttgtt ttttaaatgt tatagtgatt gtagcgtttg ttttttttgtg gaaggagttt 4560
gtttcgttaa tgagggagtt tttttttatg gttttttttt tatttttttt taaaaagttg 4620
tttttattt taatattttt taagttttat ttttttgagt tagggtcgtg ttttgttgtt 4680
tacgttggag tgtagttgta attatagttc gttgtagttt taaattttttg ggtttaagtg 4740
atttttttgt tttagttttt taaatattgg gattataggt gggcgttatt atatttagtt 4800
tttgtttttt gtaagtgaag tttttttaggt aggttgaatt ttttatattt tttgtttttg 4860
tttgattttt atttttttatt ttttattttt ttgtttttttg taatgagaaa ggtaataagt 4920
tttggagttt tttggagttt ttgttttttt tgaaaaggtg gttgtagaga ggttggagtt 4980
gatggggaaa gaggtgttac gaggtttttt ttgggttagg gttatacggt ttttaaggtt 5040
ttcgtttttt tttttggggt gataggtgtt tttagggtta tacggttttt aaggttttcg 5100
tttttttttt tggggtgata ggtgttttta gggttatacg gtttttaagg ttttcgtttt 5160
ttttttttggg atgataggtg tttagggtta tacggttttt aaggttttcg tttttttttt 5220
tggggtgata ggtgtttttt gtgttttaga ggtttttgtt tggttattag tgaggttatc 5280
gggttgttta gattagggtt gtttttgtta ttatatggta agtagagaag tagagaagtg 5340
ttaaaaatta cgagtgtttg tttggttttt tttttttgtt aaaaatagaa gtcggggttg 5400
ggtgcggtgg ttgatatttg taattttagt ttttttagag gtcgaggtag gtgggttgtt 5460
tgaggttagg agttcgagat tagtttgatt aaaatggcga aattttattt ttattaaaaa 5520
taaaaaaatt agttgggtgt ggtggtgtgt gtttgtaatt ttagatattt aggaggttga 5580
gattgtagaa ttgtttgaat ttgggaggcg gaggttgtag ttagtcggga tggtattatt 5640
gtattttagt ttaggcgata gagttatatt tcgttttaaa aaaaaaaaaa agaaaaaaaa 5700
gtagaattcg gttgggttta gtggtttatg cgtgtaattt tagtattttg gaaggttaag 5760
gtgggtgaat tatatgaggt taggaggtag agattagtat ggttaacgtg acgaaatttt 5820
tatttttatt aaaaatataa aaattagttg ggcgtggtgg tgtgtgtttg taattttaga 5880
tgtttgggag gttgagatat tagaattgtt tgaattcggg aggtagaggt tgtagtgagt 5940
cgagatggta ttattgtatt ttagtttagg tgatagagtg atattttgtt ttaaaaaaaa 6000
aaaaaaagtt agtcgttgtg gtttacgttt gtaattttag tattttggga ggttaaggcg 6060
ggtggattat ttgaggttag gagattgaga ttattttggt taatacggtg aaattttatt 6120
tttgttaaaa atataaaaaa attagtcggg tttggtggta ggcgtttgta gttttagtta 6180
ttcgggaggt tgaggtagga gaatggcgtg aatttaggag gtggagtttg tagtgagtcg 6240
agattatatt attgtatttt agtttgggta atagagcgag attttgttag agaaaaaaaa 6300
aaaaaaaata ggtgatgttt gtgttattgg atatatttgt atattatttt tatgttgatt 6360
ataaaagagg gtgaggggtc gggtatggtg gtttatgttt ggaattttag tattggggga 6420
ggtcgaggcg ggtggattat ttgaggtttg gagttcgaga ttagtttgat taatatggtg 6480
aaatttcgtt tttattaaaa atataaaaat tagttgggta cggtggcggg cgtttgtaat 6540
tcgagttatt tgggaggttg aggtaggaga atagtttgaa tttgggaggc ggaggttgta 6600
gtgagttgag attgtattat tgtattttag tttgggtgat agagcgaggt tttattttaa 6660
aaaataaaat aaaaaattta aaattaataa aaaagagggt gatggttgag tgaggagttt 6720
atggtgggtt ttatataggg tttaggaagg atagggcggg aagcgtgagt attttgttta 6780
tcgtagtggg ttcgttatgg agagtatgat tttagagaag taaagtcgtt tggttcgaag 6840
tagtggtcgt tttgttgata gttgtagtgg tcgggtggta ttcgggttgg ggttcggggg 6900
agtttaggtg gttatttggt tgtgtgtttg tgtttgttta agttgggtat tttttttttgg 6960
tagtgtttgg gaagttcggt cgtttttttt ggttggtgtt gttttgtagt ttcgtttatg 7020
gatttttgtt tacggtttgt tttttgtagt agggtcgggt cgtgggaggt tcggatagga 7080
ttttgaatgg ttttgtttgc ggttttggtt ggtcggaggt tgatcgtatt ggataggtta 7140
agggttttaa attagggtag tgaggagttt gttgagtttt tgttgttttt cgggttgttt 7200
ttggcgttcg gatagatatt taaggataga agttcggata gtttttaagg cgaagggttt 7260
ttcggggttg atttttcggg attttggttt ttgtagagag ggttggaggc ggggttttgt 7320
gttttagggt tagagttttt ttggtggaag ttgaggtgtt tgattaaatg tttttttttt 7380
taggtagtag tttagacgcg gtatagagag ggtttgggag tttgttgtag ttttaggtga 7440
gtttgttttt aagttttgcg gttcgtttat gggtttgttt gcgttgtaga attgtttgtg 7500
gagtagtagg gttggagtgt ttacggttgg ggggtttttc ggagtttttg atttatagaa 7560
tttttggagg tcggtagttt tgtttgtttt gaagagtttc gtttttttag tcgtttgggt 7620
cgtggttttg tcgtgggagg cgacggtgag ggcggttcgg ggtttcgata ggggtgtttg 7680
ttttttttggg gatttttttt gtgttgtggt ttcggttttt agatggagtt ttttattttt 7740

acggtagcgt tggggttggg gttggtgagg agtttgtgta cggtttttat cgttattttt 7800
agttaggagg gtgtttggtt tttggggagc ggtttgtatt tgtttttgga gaatgttgtt 7860
ttgttagttg ttatttgggg cgggcggtcg gagtttacgt ttttttttag ggcggggttt 7920
tggttttgtt ttttgtaggg acgatgggaa gttttgtttt tagttttttt attcgattta 7980
tgtaggagtt ttttgttttt ggggttattt tttttatagg ggttttagcg ttagtgggga 8040
taaaggtaga gttgtttgtt tttaggttgt gggtatcgat tgagtttcgt ttcggtagtg 8100
gttggttttt gtttttttgtt tggattagta gtttttggtt tggtgggtgt agttttaggg 8160
cgtagtgggg ttgtcgtggg gtaaggttag gttattagtt gtttggatat aggggttttc 8220
gtttttttatt tgttgttttt tatattttta gttcgggtag gggtgatatc ggggtatttt 8280
tgttggggtc gttgtggttt ttatggtggt aagatggttt tgtgaaagat tgacggagat 8340
gtagggttta ggagtaggtg ttgtgtgagt gggatacgtt tgtgttattt gcggggtagg 8400
ggagggttcg ggggtatagt cgtgtatatg attagttagc gagatagtga ggagtgtatg 8460
cggagtggat atagttgtgg gtgtcgttcg cgtaaggttg ggggtcgttg tgttagtttt 8520
gtttttatgt gggtttttag ttttttaggt agggacgttg atttcgggag gtgtttttgt 8580
ttgtatttat tggagtttta ggagtatgcg tttcgttttt acgggttatt tttattgagt 8640
ttattattta tcgagaggta tttcgagatt ttgttttgtt acgggtgggt tgttaaggtt 8700
agtattagag atttttagta gattttagtg gtcgtagatg gagcggggcg gtaatggtta 8760
ttttcgggat ttagttttgt gttgagtttc gggtagtgtg attatttttgg tttttttcgt 8820
gtacgttttt tggttggatg ttttttgttg tttttacggg gtgtgtgtgt ggtatatagg 8880
atagggatcg gttagttggt tttgtttatt aattatttgt ttttataggg tagtggcggt 8940
tttatttttg taattttttg aggttggatt taggttatcg tttcgtttaa aattagggta 9000
tcggttttta gggagggcgg ggagttgtat agttggattt gtgggggtta ggtatggatt 9060
tatatagttc ggggttttttc gtattttgtt tttttaggga gtttagaggc gggcgtggtt 9120
gtagtttggt tttgtggtag ttatgagtta cggttatttt tgaggcgtcg ttttgagagt 9180
tagttttgtg gttacggttt ttgttggttt ttattcgcga gtatttgaaa tagtttggtt 9240
tatagtagag gttcgggggg tatatgtagt ttacgggagt agtatagatg tttggtcggc 9300
ggtaggtttt ataggagtag tagcggttta gggtatgttt ttggggttag gggttagagt 9360
tcggtatgtt ttgcgggggt tttattttgt tgtttttcgg tttggtttgg gtatattttt 9420
tggtgatagt gtttttgtac gttttttttt tttttcggtt atttagatag ggagttagga 9480
tgggaattac ggattgattt gatttgcgtt ttcggggtta ttgtattggg tgatttacgt 9540
gtgtttaggt tttgaggtgg gtcggatttg ggagtggtag gtgagcgttt tttttaagag 9600
tttttggttg gtcgtagtag gttttgtttt tgttgttttt agtggttttg gatatgataa 9660
tttttttgtc gaggtttggt cggagttagt atttacggtc gagatgtagt tagttttggt 9720
tgggcgttgt ttttgggggt ggttgttggt gttgtttttt tttttttta ttattttttg 9780
tttttttttt ttggggtttt tttatcggag tttttttttt aaatgtagtt tttttttttt 9840
aggggaacgc gggttgggag tttatttttt ttttttttga gacggagttt cgttttgtcg 9900

tttcggttgg agtgtagtgg cgcggtttcg gtttattgta attttcgttt ttcggattta 9960
ggttattttt ttgttttagt tttttaagta gttgggatta taggcgttcg ttattacgtt 10020
tagttaattt tttgtgtgtt tttagtagaa atagggtttt tttattttgc gtaggttagt 10080
tttgaatttt tgatttcgtg atttatttat ttcggttttt tatagtgttg ggattatagg 10140
cgtgagttat tgtatttggt ttgggagttt ttttcgtagt tgtttatttt gtattagtat 10200
ttgtttatta tgagtggacg gaatataggg ggatttagga tttatagttg tattcgggaa 10260
ggttagtagg gtggatttcg ggaagagtat cggtgttagt tttttatagt tatttttgcg 10320
atgggatttt taggttggta cgtggttggt agttgtgttt cgggagtttt tgttttttag 10380
tgggtttcgt tagttagggt agggttttcg gtattttta cggggttttt gatttttttg 10440
gtagcggatc ggggcggttt agcgatatta tttttggaga ttttttggcg tgtgttggaa 10500
aaatttcgga gagggtttaa tagagttagt tgtttagttt ttgtgatagg gacgtttgtt 10560
ttttttgta gggttttttt ttttttttcg agtatttacg ggaggggatt gtgttgtttt 10620
tgttttttta ggataggttt ttttttgtt tagatggggt ttatagggtt agttgtttga 10680
gattggtttt agtttaagaa aggtttttttg agtgagcgta ggggagtagg aggttggtga 10740
gtagttcgtt ttttgttttg ggtgtagttg ggtattattt ttgttttggg tttaaaggta 10800
ttagtgagga gtaggcgggg tttggtagga gtttgtgttt ttagcggtac gcgtgatttt 10860
aggtgttacg tgggtgagtg cgtgggtggg tgagtggcga aggagttgag gttggttttg 10920
gtaggtgtgt agtttttata tagaacgggt agggtttttg ttagagttaa attagtttag 10980
ggtttagtgt aagtagtttt tgtttcgtgg tattttcgtt agtcgtcgtt tttatttaag 11040
ttttgtttcg ttttgattcg tttagggttt ggttatagaa ggggagtgtc ggaaatgttt 11100
aattcgtttt tcgtgaggga tttttgcgtg gtttgggttg gggaacgtag gggtcgtttg 11160
ggtggtttcg gcgtttggcg tagttacgcg agaggttttg ggttgatgcg tgggtttttt 11220
gttagcgagt ttgtttcgtg cgtgttgggt tttcggtttt gttttcgtgt ggtattcgag 11280
atttcgtgga tttcgtttgc gttttgtatg tttgagcgtt gttttttgtg gaggtcgtta 11340

gtattggtgg ttgtagggtt tgtagtgtgg tacgtttaga gattcggttt ggagaacggg 11400
ttttggatgt tttcgttatg ttttacgttc gttttatacg gacgtagcga tgttttagat 11460
atttcggatt atatagagta cgtcgttaag gtttgttttg tttgtttttt tttatttgtt 11520
ttacgtggat attaggagat tcgttacgat tttcgtggtt tcgtttatat tgcgcggcgt 11580
ttttcggcgt ggtcgttagg tgtttttcg agggtaggtg tgagtatagg gtggtttttg 11640
tggagaagcg tttgtaggcg agtgagttag gttgtgtttt gttgatttcg aggtttgaat 11700
tttatggaat ttttattttt tatagagtat tgtttttttt tttgattttt ttttaattat 11760
ttaaaaatgt aaaagttatt gttgatttgc gggttggatt aggtttaggg ttgggttttg 11820
gttcgtattt tatattggtt tcgtgtgttt tgtgatcgtt ttgtttacgg ggtaggtttg 11880
agttattata ggatggtagg gtttttaggg tgtagggttc gtaatgtttt aattttttta 11940
cgattttagt atttttagag aaaacggatt ttcgtgtcga gtgaagtggt ttaggcgaac 12000
gttattatta tacgggggat tcggcggttt ttgttttagg ttttaaagga gaggggattt 12060
agaatttttt gttttatttt tttagttttt gtttttgaag tttttgtttt taaggttgtg 12120
gggaggaatt agggaagggt tggcgcgttt gttgtgcgat tttaggtgtt cgtgtttcgt 12180
tgagtttagt ttttgtggtt ttagagttgt aggttttttt cgggtgttag agtattgatt 12240
ggaaaggttt tttatatttt tgttttttgt tgttttttat cgtcgcgggg gagaagttgg 12300
aattgtattt ttattttttt tagttcgttt atttagggtt gtttttaggt ttttttgagt 12360
tttaaaggat atgtttttttg gtgttttttt tttttttggt tttgtttttt aggttggaat 12420
gtagttgtgg tgtttacgtt attttttttac gttagttttt tgagtagtta ggtttatagg 12480
tgtatattat tatattcgga tagtgtttag ttttttataa agatagggtt ttattgtgtt 12540
gtttaggttg gttttaaatt tttgggttta ggtgattttt tacgtacggt agttttgata 12600
tttatttgat tttttttttt taagttttat tttgacgttg gttgtgtgtt ttagagattt 12660
aattagaagt tggaagaagg gttgtgacgt gtgtgatagt aggaattgtt ggttaattta 12720
tagtcggtta gtttgtcggg acgttatttt ggttttaatt ttggtaggga gaggggagtg 12780
tatcggattt tggggagagt agttgtttgt acgggtaggg tttttttcgtt ttaggattaa 12840
cgtttttaat tgtatttgat tttttttttag ttagaggggg taaggtggtt tttttagaag 12900
tataggtggg tcgcgtgtta gaggttttgg atagtttgtg tttttttttag gtttttttagt 12960
aggggtggtt ttcgtttttt tggaggggag attttaggaa tcggttttcg tttttagtta 13020
tagttttata gggtttttgt ttttcggggt ttggttttgt gggtgtataa ggtatggtcg 13080
ggttttcgtt tttttggaag tttttagagt ggtatgtggg tagttgtgtg aagcggtttt 13140
ttagattttt atttagtttg gtttgtagta gttttttacg tttttttgtta tttttttttgt 13200
ttatttttgg gtagacgttt ttttgatttt tttttttgtg ataaagtaaa agcgtgtgat 13260
tttttttttt attaaggaat aaaacgtttt tgaatattat agacgtgatg tcgggttttt 13320
gggggtcggg gggtgtttta gggtttcgag ggaggagttt tgtgggtgtc gaagtcgggt 13380
cgtaggtttt gtattgagat gttttttttag tttattttag gcggttgggt atgttgggaa 13440
gtttttttcgg gttgtgtgtg tgaattatgt tgtttttttt ttattttttt tttttgtttg 13500
tttattttttg ttgtgtgtta ttttgtttag tcggtcgttt tgtttttttt ttttatgttt 13560
cgtttcgttc gttgttggtt agaggtgatt aggttcggtt tttagtacgg tcgtcggtcg 13620
gggggttatg gggagtttcg tttttttttt ggtcgtagag atcgagtcgg attatagttt 13680
tttcgggggga tcgttttatt cgggtttttc ggtagcggtt ggttggtgta ggagcggttt 13740
aggggggttc gtttggggag gtgttttggt tagtcggtag tattagtttt tacggtttcg 13800
ggttcgaagg taagaggtgg ggaggggtgg tttcggaggg ttttcggggg tagagggttt 13860
cgtttttagg gtgttttttt tttggtagag gtgtttgtag agaagtgggt ttttgttttt 13920
tttagttgtg gtagtttttt tttttagggt gtaggagttg agtaggtttg agatttcggt 13980
ttcggtttgg ttggttggtg gtggggatgg gggttagtgc gtttaatttc ggcggttttg 14040
tgttttttat aggttgtgtt gttttacgta gagaagttgt ttttgaagtt tttttttggg 14100
ttggggtcgt tggcggttgt aggttttggg tatattgttt gttgtttacg gttttaaagt 14160
tgttagtatt cgttgggttt cgtttgttgt tttttcgggg gttataggtt atatggtttg 14220
gtggtcgaat tttacgtgtt ggttttttat agttgttatt atggttttgg ttattcgttg 14280
cgtttttttag gagttgttat taggttttga ggtgtttggt ttggaggtag tttaggtggt 14340
gaggtttgga ttggttggat tttatcgatg tttttgtcgt tatttcgttt tggtttttat 14400
tggaggtagg gatatttagg ggttcggggt ttcggggtta gtgttgtagt ggtcgtcgtt 14460
gtttttatag cgggtttagg tttggttttt gaaggtaatg tgtttgcgtt ttatatttaa 14520
aagggtttgt taggttgtat ttggcggtag tttttacggg ggacgatgtt ttgttgtttg 14580
ttggttttta ggtgggaggg acgggcgtgg agttgtaggt agtgggtaag gttatggtag 14640
gtggttaggt ttgggaatta gtttttaggt ggtatttacg ttgaggtttg agtttcggtc 14700
gataaggatt ggggatgtag gggattagtt aggggttgtg tgggggagtt ttggagtttt 14760
gtttgcgagt tatttcgatg aatagggaga cgcggtagag gtagggtcgg ggaggggtcg 14820
tgtgggggag ttttggagtt ttgtttgcga gttattttga tggatagaga gacgcggtaa 14880
aggtagggtc ggggaggggt cgtgtggggt ttggtggtcg gtagaggaag ggtttgtgtt 14940
ttttttgcgt gtggtggtat cggagcgttt tttagttttt ggtgttatcg tattagtcgt 15000

tttttagtga gtatttttac gtgtgtcgat aatagttttg ttgaggtaga atttattaga 15060
tttatttgtt aaaagagtgt agtttggtta tttttagtgg tttgtagagt tgtgtagtcg 15120
ttattgtaat ttagcgttgt agtattttat agtttatagg gatttttagt tttatagttt 15180
agtgttatag tatttcgtag tttagaggga tttttagttt tagacggtta ttgatcggtt 15240
ttttgttttg gtttgttttg tgtagaaggt gcgtggatgt ggggttatag gacgtggttg 15300
gttgggtttg ttttgtgtag aaggtgcgtg gatgtggggt tataggacgt ggttggttgg 15360
gtttgttttg tgtagaaggt gcgtggatgt ggggttatag gacgtggttg gttgggtttg 15420
ttttgtgtag aaggtgcgtg gatgtggggt tataggacgt ggttggttgg gtttgttttg 15480
tgtagaaggt gcgtggacgt ggggttatag gacgtggggt ttcgtgtttg gtttttttat 15540
tcggtatcgt gtttttttaga tttatgtatt tgtagttcga ggtagtttta tttattgttt 15600
ttttgcggga tgaatcgtat tttatttatt tatggacgtt aggattgttt ttacgtggtt 15660
tttgtggata atgttgtgaa tattgtttac gaggttttgc gtggatttgt ttttatttat 15720
tttgtataga tatttaggag tgggattgtt ggggcgtgtg gtgaattttt ataatgtttt 15780
gaggaattgt taagttattt ttcgtagcgg ttgtattatt tagtttttat cggtaacgtt 15840
ggggagggtt ttggcgtttt tattttttcg atagttttttg tcgttgtttg tttttgtgat 15900
tttggtcgtt ttagggggtg ggagttggtg tttttttggt tttgatttgc gtttttttgg 15960
tggttagtta tgtggggttt ttttttttgt tttatttatt cgtgtttgtt ttttttttat 16020
ttatttattt atttatttat ttagagatag ggttttattt tgttatttag gttggagtat 16080
agtggtgtga ttatagttta ttgtagtttt tattttttgg gtttaagaga atttttttatt 16140
ttagtttttt aagtagttgg aattataggc gtgtattatt atagttggtt aattagaaaa 16200
aaaaaatttg gttaggcgta gtagtttacg tttgtaattt tagtattttg ggaggtcgag 16260
gcgggtagat tatttgaggt taggagttta agattagttt ggttaatatg gtgaaatttt 16320
atttttatta aaataaaaat tagttgggcg tggtgacggg tatttataat tttagttatt 16380
agggaggttg aagtaggaga atcgtttgaa tttgggaggc ggaggttgta gtgagttaag 16440
attgtattat tgtattttag tgtgggcgat agagtgagat tttgttttaa aataaaaata 16500
taaattttga gtttgggagg gtttaggttg taatgagttg cgatcgtgtt atcgtatttt 16560
agtttgggcg atatagtgag acgttagacg gtgttttaaa aaatttaaat agaaaaatgt 16620
agaaaggatt gtcgtttttt ttttcgtttt ttagagggta gaggatttc ggtttttgtt 16680
ttttgagtat taggtcgagt tttagatttc gggatggttt ttttagtttg tagttttcgg 16740
tagtttcggg ttatattttc gggattttta gggattggtt tgggattatc gggggtggcg 16800
gtcgtggttt tggttatggg gagggaggta gagtcgcggg gtaggcgtgg ggtttgttgt 16860
gtcgggtatg tggggcgtgt gttagatttt ggatttggtt tgcggggtta ggtcgtggtg 16920
tatgatgacg ttacgtcgtt tttttttttt tattttggtt ttttgtttgg tttttttttg 16980
ggtcggtatt tttggggtta tttttgttcg tgttcgttgt tgtggggttt gggttttagg 17040
tagtttttgt ttgttgttgt aaataggttt ttaaggtgta tggtgaggag atttttaggt 17100
tagagatggg cgagagcggt gggcgagtag attgcgggaa ggtagttggg tttttatacg 17160
cggagttttt tttattacgt gtatgtatag gcgattttttg tcgtgtttcg acggagggcg 17220
tcgggattgg ggagggttag cgcgatagtt ttggcgttta ttgaagagtt gtttgtttgg 17280
ttcgttgttt tggtggtcgt tgaatttttt taacgtttta ggatagtagg tgttgtggtt 17340
ttatgatcgt tttttatat ggatgaaggg gtggtgtata ggttggtgga gtttgggaga 17400
gttagggtag tttgcggtta tgcggttatg cggtcggcgt ggggttgagg tttttgtata 17460
gttgatagag tgtttggcgt ttttaattag taagttattt acgggtcggg tatcggcggt 17520
ttagtataga gaggtaataa gggtagattt tttttggtaa ggttgatcgg cgtcgggttg 17580
tggttagatt aggagattgt agtttagttc gagggaggta ttacgtttgt tttttagagt 17640
attgagtaga gtttttttatg gtatttttcg ttttgggtat agtaggagga gttgtcgatt 17700
aagtttggta tttcggtgtg tttcgagagt tgtttgggta taggttgcgg ggttgtttcg 17760
gtcggacggg gtatttgtgt ttttcggtgt ggttttaggg ttttggtggt tttgatcgag 17820
gtttcggggg atagaatgta gttttttgtt ttttattttt tttaagaaag gggtcgtttt 17880
ttaggattgg ggttgcgtta gttataggtt cgcggttttt acgtttcggg tgttgttgtt 17940
tcggttgttt tagggcgggt tttgttttgt acgtagcgag cggttttatt attttttttt 18000
tgaattggag ttgagtgtcg gtaacgttta gataattggg gcgcgcgggt tttttgtgtt 18060
ggattttttt gtatagttgt ttcgattttt tatttagttt ggtttttgtg tagtaaggag 18120
cggttcgtcg ttttttttac gtcgtttttt ttacgtcgtt ttttttacgt cgtttttttt 18180
acgtcgtcgt ttttttttacg tcgtttttttt tacgtcgttt tttttacgtc gtcgtttttt 18240
ttacgtcgtt ttttttacgt cgtttttttt acgtcgtttt ttttacgtcg ttttttttac 18300
gtcgtttttt tttacgtcgt cgtttttttt tacgtcgtcg ttttttttta cgtcgtttgt 18360
tttgagttcg ttttttttcga aggtaattat tttgtagtag tgagggtttt ttgttgagta 18420
cgtgtggttg cgattttagg agcgttttgg gttgagaatt ttaaaagggt ttcgtttagg 18480
tttggttttg ggtttttggg tttttgacgc gagtttttat ttttaggttt tggcggtttg 18540
gttttaggag gttaaggttg tagtgagtcg tgcgcgagtt attgtattat tttgggtaat 18600
tagattttat ttttaaagtt taaaataata tttttgtaag ggtttcgggt ttttgagttt 18660

atgaatttgt ttaggttttg tagaggtgag ggtggtttta gagtggttgg ggggttgtgg 18720
agattagaat tagggtagaa gggtagggag ggtgggggtt ttgtcgaggt tttttatcgt 18780
tgatttttga tttttggttt ttttttattt cggtttatcg agagaggtcg gtatagcgta 18840
gattgcgggg ttagtgatat tttttttagc gtttttagtt atagaagggt cgtggtttgt 18900
tttcgttttt tttattcgtg tttcggggtt acgtttgaat tttaggtttt gttttgattt 18960
tattgaaatt tttttgaagt gtagacgttt ttttgattta tttgggtttt gttaggatga 19020
tttcgaaagg tgttttttag tagatatatt tcgttggcga ttgtttggtt ttggggattc 19080
gggtttttat tttttttttt ggagttggtg aggttgtttt tgttagtcgg tgataattat 19140
agggttaaag ggagggtgtg ttttaggagg tttagtttat ttttgtttgt atttgcggtt 19200
agcggggtgt agagtttgtg aaggtatttt tcggagtggg ggttgtaaag attatggtta 19260
ttgtgttatt tatagggggtt taggtcggac gtttgggttg tgtcgtttcg gtagttaggt 19320
cgaggaacgt gtaggtgcgg tcgggagttc gtcggttata gttgtattta tgtttttttt 19380
cgagtggtga tggcggggag aggtttggtt gtttacgggt agattgtttg ggtttttata 19440
gaagttcgtt gttagagtag attttcggtt ttgggttttt ttgttttagt tggacgtttt 19500
gagtttcgtc gggtttagtt tattttcgtt tattttgtta ggggtagttt agagttttcg 19560
ttacgcgggg ttgagcgtag ggaaggtagt attacggagt tcgttcggtt ttatagaata 19620
ttcgtgttgt tcgcggagat tttttgtttt tgtgtttttt atgaagaggt aggttttttt 19680
ttttttgaa ttaaaagggt ttagatggag tataagattt aggttatatt tataagaata 19740
attgattttt ttgttaggta gagtagttag tttgtttcgg aggttgtgtt gtggtgtggg 19800
gaggtttttt gtttagatta gtttgtgttt tttttaggtt tcggtttatg tatttgtttt 19860
ttcgtaggtt tgagtatttg tttttttacg tttaggacga tttggttttg agtttggtcg 19920
ggattttttg ttatttgtcg tttataggtt tggtgataga gaaagtagtg ttttggaata 19980
gtagttcggt tgtggggtgt ttagtttttt ttgtaggatt ttgtgtggtt taaggggttt 20040
gttgagtttt taacgggtaa gaggggtttt gagttatgga tttattttgg gtggaggtag 20100
tttttatcgt cgtttgtttt aaatgtttag ttttaggttt tcggtttcgt agttttgtag 20160
ttatagacgg gtgttttttt taggtcgaga ggagggtggg gtttagttat ttaggatttt 20220
atagggtggt tttgatgagt attttttttt gtagttatat ttattcgttc gggtttatgg 20280
ttacggtcgg agagtggttt tgtgtgcgtt gtattttttt gaattcggtc ggttagcgtt 20340
agtgttttat ttgcgaggtt tttcggtata agttcgattt taattatatt ttgcggttta 20400
gcgtggagga gtagaaatgg ttttgcgttc gttgtatttt tcgtaatttt ttgggtaagg 20460
aggtttgcga ggtgtgcggt tttatttcgg agtttgcgtt tggggttgtt tttttgttag 20520
tttttaacgg ggtttttttt aagttattcg ttattttggg ggagtttaag ggtagttgtt 20580
aggaggaagt aggtttagtg aggattgcgg ggttggtggt tacggagttc gttagggggt 20640
agtgcgagga taaggacgag gaggagaagg aggagtagga ggaggaggag ggagcggcgg 20700
agtttagagg gggttgggcg tgttcgcgtt gtacgttgta taatacgttc gtggttagtt 20760
tttgtttcgt ttgcgggggt ttacgtaggt tttcgttgtt acggattttt tttgaggttt 20820
tggtggtttc ggaagtggtg gtttcggtcg gtttttacgt cgtgtttgtc gcgtttttat 20880
ttggtttttt cggggaaggt gtcgaggtta attttttagt tattagttag ggtttagttg 20940
tcgaattaga gtcgtttagg gtttcgtttt ttagtttttt ttcgtttatt ttgtagaata 21000
atttcgtgtc gcgtagtcga cgcgaggttt ttttttagtt gtagttatcg gtgtttgagg 21060
ttgtttagtc gttatttttt gtcggttgta ggggagtttt ttagggttcg ggttgggttg 21120
gggtttttcg tttagtagag ttgttgtttg gtaagcggtt gagtgtgttg gaggaagagg 21180
ttacggaggg tggtattagt cgcgtagagg tcggtagttt tatttcgggt agtgatatta 21240
ttgatttggt cggagatatc gtgcgttata cgttcgttag tttttttagt ttcgatttta 21300
ttatttggtt atgtgttaag tgtacgttta gaaattttat agtggttttt aggtgttcgg 21360
tttgcggttg ttttaaattg tacggttttt aggagtatgg cgagtttttt atttattgtt 21420
tcgattgtgg ggtcgataag tttagttttt gcggtagaag ttgcggacgg gtgttttcgg 21480
tttagaaggt cgttcgcgtt ttgttcgagc gttcgggtta gtgggtttgt tttgtttgta 21540
ttttgtttaa cgtattgcgg gttaagtatt gcgtcgtttg ttatacgttt tagtttttgg 21600
tggtttagcg gcggggggtc gcgtttttga ggcgtaggga gagtatgtac gtggagtagc 21660
ggcggtagat agacgagggc gaggttaagg tattttggga gaatatcgtg gttttttgtc 21720
gggaggtgag gcgtttttttc gttttttttt tgtttttgag ttttttgttt tcgttttttt 21780
ttttttaggt tttgggtttt ggcgatgggt tggggaggag tttataaggt ttaagacgtg 21840
atggggaggg tagtattttt cgtttcgagg ttttaagttt ttaatggttt tggggtttag 21900
tttggttttt tgatttttgg tttttgttgg tgatcgagat gtacgatttt tatttttttt 21960
aatttttat cgtggttagg agggtttcga gtttcgtttg atgtgtcgta ggaggcgagg 22020
gtttttttagg ggtagcgtgg agatttcgtt gaagtgcgag gtcgggatgt attcgtgttt 22080
cgttttttag agtttaggat ggtattattt acgggttaat tggggcgtag tttgtatttt 22140
ttttagtagg gttagggtta gagtcgtgtt gttatttatt gggggttgcg ttgttttttt 22200
gggtttatac gtttttttgtt ttttatttgt cgttagtcgg tggttttttt attagggttg 22260
agggtttttt cgatataggg tcggcgttag gtttgtggtt gtttacgttt tattcgtttt 22320

```
tttggggttt aggtagtgtt tttgggcgtt tttttgggtg gggttttgtt tttgttaggt   22380
ggagcgaggt tattttgttt agtttgagta tatggtcgtg gtttttgtag aataatgtga   22440
gtttcgtgga tgatagtttt tttttcgggt tcgagtttgt cggtttttttc gcgggtgata   22500
gcgtgtagta gcgtgtgagg tagtggttgc gattttagga gattaattgt ttcgttttta   22560
gggattatag ggttacgtgg tttgtgtttt atatattgcg gtttttagat attttgtagg   22620
ggttgttggg gaattgttgg tgaggttttt tttaaggtcg gggtggggcg ggtgggcggg   22680
cgatcggtcg cggttttcgc gaggttattt tgaggttttg cgtaggtttt tgagcgtttt   22740
ggcggtgttg gcggagcggt cggatttggt ggagcgggtg atggttacgc gtagtttgtg   22800
tgtagagggc gtttattagg tgcggttgtg taaggacggt acgtggatta cggtgttggt   22860
ggacgatatg ttgtttttgtg atgaggtcgg ttgttttttt tttttatagg tggggcggtt   22920
tgtagggtgg gtacgggcgg taggggtagt ttttgatttt agtttcgaaa ataaggtcgg   22980
ttgttttttt ttttttttagg gcgggtagtg tggggggcgg gcgggggtgg tttttgattc   23040
ggttttttgt aggcgtagcg gaagtagttg tgggtggttt ttatcgagaa ggcgttggtt   23100
aagttgtacg gtttttattt tgcgttttag gcgggtcgcg ttatcgaagg tttggttacg   23160
tttatcggcg ttttttgtga gagtttggcg ttgtagttta gttttattaa ttttcgcgag   23220
gagttcgttg atattgattt tatttgggtt aaaatgttga gttttaagga ggttgggtaa   23280
gaggaggggt attgcgtggt tagtcgcgtt gggaggagag gcgaggcgga gcggtgggag   23340
atgtggggtt ggtggttttt gatttagttt tgtttttacg aggtgttttt ggcgtgggtt   23400
gatttcgcgt ggttaggtta tagttttaat tattttttttt tttttgggcg gggttggagt   23460
tggtttttttt atgttaggaa ggttatattt tggttaggtc gtggggcgag atcgatgtcg   23520
ttatttcgga gtaggtgtga gcggcgtgtc gagtttgatt tgggtcgtgg ggcgagattg   23580
atgtcgttat tcgtttcggg gtaggcgtga gcggcgtgtc gagtttggtt atattttggt   23640
tagcgggttt gtgtttcgat ttttaggggt tttttcggag tgtttcggtg cgagtatttt   23700
gaagttttcg gagtttt                                                  23717
```

<210> 150
<211> 23717
<212> DNA
<213> Artificial Sequence

<220>
<223> chemically treated genomic DNA (Homo sapiens)

<400> 150

```
agggtttcgg gagttttaag gtgttcgtat cggggtattt cgaggaggtt tttgggggtc      60
gaggtataga ttcgttgatt agagtgtggt taaattcgat acgtcgttta cgtttgtttc     120
gaggcggatg acggtattag tttcgtttta cggtttaggt taaattcgat acgtcgttta     180
tatttgtttc ggggtgacgg tatcggtttc gttttacggt ttggttagga tgtggttttt     240
ttggtatgag aaggttagtt ttagtttcgt ttagggaggg ggaggtgatt ggggttgtgg     300
tttggttacg cggggttagt ttacgttagg ggtatttcgt ggaagtagaa ttgggttagg     360
ggttattagt tttatatttt ttatcgtttc gtttcgtttt ttttttttaac gcggttgatt     420
acgtagtgtt ttttttttta tttagttttt ttagaattta gtattttggt ttagatgagg     480
ttagtgttaa cgggtttttc gcgggggtta gtggagttga gttgtagcgt taggttttta     540
taggggggcgt cggtgagcgt ggttaggttt tcgatggcgc ggttcgtttg gagcgtaaag     600
taggagtcgt gtagtttggt tagcgttttt tcgatgaggg ttatttatag ttgttttcgt     660
tgcgtttgta gagggtcggg ttagaggtta ttttcgttcg tttttttatat tattcgtttt     720
gggagaagag gaagtagtcg gttttgtttt cggggttggg gttagaggtt gtttttgtcg     780
ttcgtgttta ttttgtaggt cgttttattt gtgagaagag gaggtagtcg gttttattat     840
agggtagtat gtcgtttatt agtatcgtgg tttacgtgtc gtttttgtat agtcgtattt     900
ggtaggcgtt ttttgtatat aggttgcgcg tgattattat tcgttttatt aggttcggtc     960
gtttcgttag tatcgttagg gcgtttagga atttgcgtag agttttaggg tgatttcgcg    1020
gggatcgcgg tcggtcgttc gtttattcgt tttatttcgg ttttggagaa ggttttatta    1080
gtagtttttt agtagttttt gtaggatgtt tgagggtcgt agtgtgtgga atatagatta    1140
cgtggttttg tggtttttga agacggagta gttgattttt tggggtcgta gttattgttt    1200
tatacgttgt tgtacgttgt tattcgcggg gaagtcgata gattcgggtt cgggagggaa    1260
gttgttattt acgaagttta tattgttttg tagagattac ggttatgtgt ttaggttggg    1320
tagggtggtt tcgttttatt tggtagggat aagattttat ttaggagagc gtttaaaagt    1380
attgtttgag ttttaaaggg acgggtggag cgtggatagt tatagatttg gcgtcggttt    1440
tgtgtcgagg aagtttttag ttttagtgga gaggttatcg gttggcgata ggtgaggagt    1500
aggggacgtg tgggtttagg gggatagcgt agtttttagt gggtggtagt acgattttgg    1560
```

```
ttttggtttt gttggggagg gtgtaggttg cgttttagtt gattcgtggg tgatgttatt    1620
ttggatttta gagggcggag tacgggtata tttcgatttc gtattttagc ggggttttta    1680
cgttgttttt gggaagtttt cgttttttgc ggtatattag acggggttcg ggtttttttt    1740
ggttacggtg gagggttgag gggggtgaag gtcgtgtatt tcggttatta gtaaggatta    1800
agggttagga ggttaagttg gattttaggg ttattgggag tttggagttt cggggcggag    1860
ggtgttgttt tttttattac gttttaggtt ttgtgggttt ttttttagtt tatcgttaga    1920
gtttaaagtt taggaagggg agggcgggag taggaggttt aggagtagga agagggcgag    1980
ggggcgtttt atttttcggt agaaggttac gatgtttttt tagagtgttt tggtttcgtt    2040
ttcgtttgtt tgtcgtcgtt gttttacgtg tatgtttttt ttgcgtttta ggggcgcggt    2100
ttttcgtcgt tgggttatta ggagttgagg cgtgtggtag gcggcgtagt gtttggttcg    2160
tagtgcgttg agtagggtat aggtagggta ggtttattgg ttcgggcgtt cgggtaggac    2220
gcgggcggtt ttttgggtcg aggatattcg ttcgtagttt ttgtcgtagg ggttgggttt    2280
gtcggtttta tagtcggggt agtgggtggg gggttcgtta tgtttttgga agtcgtgtag    2340
tttggagtag tcgtaggtcg agtatttggg ggttattgtg gggtttttga gcgtgtattt    2400
ggtatatgat taggtggtga agtcggggtt ggaggggttg gcgggcgtgt aacgtacggt    2460
gttttcggtt aggttaatga tgttattgtt cgaggtggag ttgtcggttt ttacgcggtt    2520
ggtgttattt ttcgtggttt tttttttag tatatttagt cgtttgttag atagtaattt    2580
tgttaggcgg gaggttttag tttagttcga gttttggggg gttttttttgt agtcggtaga    2640
gggtgacggt tgggtagttt taggtatcgg tggttgtagt tggggggggaa tttcgcgtcg    2700
gttgcgcggt acggggttgt tttgtagggt ggacgagaag gggttgaagg gcgggatttt    2760
gggcggtttt ggttcggtag ttgggttttg gttggtggtt gggggggttgg tttcggtatt    2820

tttttcgggg aggttaggtg gaggcgcggt aggtacgacg tggaagtcgg tcggggttat    2880
tattttcggg attattaggg ttttaggagg gattcgtggt agcgagagtt tgcgtgggtt    2940
ttcgtagacg gagtaggagt tggttacggg cgtgttgtgt agcgtgtaac gcggatacgt    3000
ttagtttttt ttgggtttcg tcgttttttt tttttttttt tgtttttttt tttttttttc    3060
gtttttgttt tcgtattgtt ttttggcggg tttcgtggtt attagtttcg tagtttttat    3120
tggatttgtt tttttttggt agttgttttt gggttttttt aggatggcgg gtggtttggg    3180
gaggatttcg ttgaggattg gtaggaaggt agttttaggc gtaggtttcg gggtgaagtc    3240
gtatatttcg taggtttttt tgtttaggaa gttgcggaag gtgtagcggg cgtagggtta    3300
tttttgtttt tttacgttga gtcgtaggat gtggttgagg tcgggtttgt gtcggggagt    3360
ttcgtagatg gagtattggc gttggtcggt cgggtttagg aaggtgtagc gtatatagga    3420
ttatttttcg atcgtggtta tagattcggg cgagtgggtg tggttgtaag ggaaggtgtt    3480
tattagggtt atttgtgag gttttgagtg gttggatttt attttttttt cggtttgggg    3540
aaggtattcg tttgtggttg tagagttacg gggtcgaggg tttagggttg agtatttgag    3600
gtaggcggcg gtggggggttg tttttattta gaatggattt atggtttagg gttttttttg    3660
ttcgttggag gtttagtaga ttttttgagt tatataggat tttatagaga gggttgagta    3720
ttttatagtc gggttgttgt tttagggtat tgtttttttt gttattaggt ttgtggacgg    3780
tagatagtag ggggtttcgg ttaaatttag gattaggtcg ttttgggcgt ggaagaatag    3840
gtgtttagat ttacggaagg gtaggtgtat gggtcggggt ttggggaagg tataggttga    3900
tttgagtaag ggtttttttt atattatagt atagttttcg agatagattg gttgttttgt    3960
ttggtagaaa aattaattgt ttttgtaaat atggtttgaa ttttgtgttt tatttaaatt    4020
tttttggttt aggaagaaag aaaatttgtt tttttataag aagtataaaa ataaaaagtt    4080
ttcgcgagta atacgggtgt tttgtggagt cgagcgaatt tcgtgatgtt gttttttttg    4140
cgtttagttt cgcgtggcga gggttttgag ttgtttttgg tagagtggac gggggtggat    4200
tgggttcggc gaggtttagg gcgtttagtt ggagtagaga gatttaaggt cgggaatttg    4260
ttttaatagc gggtttttgt gaaagtttaa gtaatttgtt cgtagatagt taggtttttt    4320
ttcgttatta ttattcggag aagggtatgg atgtagttgt ggtcggcggg ttttcggtcg    4380
tatttgtacg tttttcggtt tggttgtcga ggcggtatag tttaggcgtt cggtttgggt    4440
ttttgtgggt ggtatagtgg ttatgatttt tgtagttttt atttcgggag gtgtttttat    4500
agattttgta tttcgttgat cgtaggtgta ggtagaggtg ggttgggttt tttgggatat    4560
attttttttt tggttttgta gttgttatcg attagtaagg atagttttat tagttttaga    4620
gaggagaatg agaattcggg tttttaggat taaataatcg ttagcggggt gtgtttgttg    4680
ggagatattt ttcggagtta ttttggtaaa atttaggtgg gttaggaaga cgtttgtatt    4740
ttaggagggt tttaatgaag ttagaataag gtttaaagtt taaacgtggt ttcgggatac    4800
gggtgaggag ggcgaaggta ggttacggtt tttttgtggt tggagacgtt gaggggagtg    4860
ttattgattt cgtagtttgc gttgtgtcgg tttttttcgg tgggtcgagg tggaaggggg    4920
ttaggggtta ggggttagcg gtgggagatt tcggtagggt ttttattttt tttgtttttt    4980
tgttttggtt ttggttttta taatttttta gttattttag ggttattttt atttttgtag    5040
ggtttgggta ggtttatggg tttagagatt cgaaattttt ataaagatgt tgttttgaat    5100
tttagagatg gggtttgatt gtttaggatg gtgtagtggt tcgcgtacgg tttattgtag    5160
```

```
ttttgatttt ttgaggttag atcgttaaag tttggaagta aaagttcgcg ttaggagttt    5220
aaagatttaa agttaggttt gagcgaagtt tttttggaat ttttaattta gaacgttttt    5280
gaagtcgtag ttatacgtgt ttagtaagga gtttttatta ttgtaggatg gttattttcg    5340
aagggagcgg gtttagggta gacgacgtga ggggagacga cgacgtgggg ggagacgacg    5400
acgtgagggg aggcgacgtg ggggaggcga cgtgggggag gcgacgtggg ggaggcgacg    5460
tgggggaggc gacgtggggg agacgacgac gtgggggaga cgacgtgggg gaggcgacgt    5520
gggggaggcg acgacgtggg ggaggcgacg tgggggaggc gacgtggggg aggcgacgtg    5580
ggggaggcga cgggtcgttt tttattgtat agaggttaag ttggatggaa gatcgaggta    5640
attgtataga aaaatttagt ataaaagatt cgcgcgtttt agttatttgg gcgttgtcga    5700
tatttagttt taatttaaaa gggaggtgat gaagtcgttc gttgcgtgta agatagagtt    5760
cgttttgaag tagtcgggat agtagtattc ggagcgtgga ggtcgcgggt ttgtgattgg    5820
cgtagtttta gttttgggag gcggtttttt ttttaggaaa agtgaggagt aggaagttgt    5880
attttgtttt tcggggtttc ggttaaagtt attagagttt tgaggttata tcgagaaata    5940
tagatgtttc gttcggtcgg ggtagtttcg taatttgtgt ttagatagtt ttcggggtat    6000
atcggggtgt tagatttggt cggtagtttt ttttgttgtg tttagggcgg gggatattat    6060
ggggggtttt gtttagtgtt ttgaggggta ggcgtggtgt ttttttcgag ttgggttgta    6120
gtttttttggt ttggttatag ttcggcgtcg gttagttttg ttaggggggg tttatttttg    6180
ttgttttttt gtgttgagtc gtcggtgttc ggttcgtaga tgatttattg attgagggcg    6240
ttaggtattt tgttagttgt atagggattt tagttttacg tcggtcgtat ggtcgtatgg    6300
tcgtaggttg ttttggtttt tttaggtttt attagtttgt gtattatttt tttatttatg    6360
tgggggagcg gttatggggt tatagtattt gttgttttgg ggcgttggga gaatttagcg    6420
gttattaggg tagcgagtta ggtaggtagt tttttagtaa acgttagggt tgtcgcgttg    6480
attttttta gtttcggcgt ttttcgtcgg agtacggtag gggtcgtttg tgtatgtacg    6540
tggtggggag ggtttcgcgt gtggaggttt agttgttttt tcgtagtttg ttcgtttatc    6600
gttttcgttt atttttagtt tgggggtttt tttattatgt attttggggg tttgtttgta    6660
gtagtagata ggagttgttt ggagtttagg ttttatagta gcgggtacgg gtaggagtgg    6720
ttttagggat gtcgatttag gggagggtta ggtaggaggt tagggtgaaa gagaaagagc    6780
ggcgtggcgt tattatatat tacggtttga tttcgtaggt taggtttaaa gtttaatata    6840
cgttttatat attcggtata gtagatttta cgtttgtttc gcggttttgt tttttttttt    6900
atagttagag ttacggtcgt tattttcggt agttttaggt tagtttttgg ggatttcggg    6960
agtgtggttc gaggttgtcg ggagttgtag attggagagg ttatttcgag gtttgaaatt    7020
cggtttggtg tttaaaaagt aggggtcgag agttttttgt tttttggaaa acgagaaagg    7080
gaacgataat tttttttata ttttttttgtt taagtttttt gagatatcgt ttagcgtttt    7140
attgtgtcgt ttaagttgga gtgcggtggt acgatcgtag tttattgtag tttggatttt    7200
tttaagttta gggtttgtat ttttattttg agatagagtt ttattttgtc gtttatattg    7260
gagtgtagtg gtgtaatttt ggtttattgt aattttcgtt ttttaagttt aagcgatttt    7320
tttgttttag ttttttttagt agttaggatt ataggtgttc gttattacgt ttagttaatt    7380
tttgttttag tagagatggg gttttattat gttggttagg ttggttttga atttttgatt    7440
ttaggtgatt tattcgtttc ggttttttaa agtgttggga ttataggcgt gagttattgc    7500
gtttggttaa attttttttt tttaattaat tagttatggt ggtgtacgtt tgtagtttta    7560
gttatttggg aggttgagat gggaggtttt tttgaattta ggaagtagaa gttgtagtga    7620
gttgtgatta tattattgta ttttagtttg ggtgatagag taagattttg tttttaaata    7680
aataaataag tgaatgaatg aaaaaaagat aggtacgggt ggatgagata gggagagagg    7740
ttttatatgg ttggttatta gggaaacgta gattaaagtt aaggggatat tagtttttat    7800
tttttgggac ggttagaatt ataaggatag ataacggtag gagttgtcga gggagtggag    7860
acgttaggat ttttttttagc gttgtcggtg ggaattaggt aatgtagtcg ttgcggaaga    7920
tggtttggta gtttttttaag atattatgaa agtttattat acgttttagt aattttattt    7980
ttaggtattt atgtaagata aatgaaaata ggtttacgta aaatttcgta aataatgttt    8040
atagtattgt ttatagaagt tacgtggaag tagttttagc gtttatgaat agataaaatg    8100
cggtttattt cgtaaaggag tagtgaatga aattgtttcg ggttgtaggt gtatgaattt    8160
ggagggtacg gtgtcgagtg aagaggttag atacggggtt ttacgttttg tggttttacg    8220
tttacgtatt ttttgtataa ggtaaattta gttagttacg ttttgtggtt ttatatttac    8280
gtattttttg tataaggtaa atttagttag ttacgttttg tggttttata tttacgtatt    8340
ttttgtataa ggtaaattta gttagttacg ttttgtggtt ttatatttac gtattttttg    8400
tataaggtaa atttagttag ttacgttttg tggttttata tttacgtatt ttttgtataa    8460
ggtaaattaa gataggaagt cggttagtgg tcgtttgggg ttgagggttt ttttgggttg    8520
cgggatgttg taatattgga ttgtggggtt gggggttttt gtgggttgtg gaatgttgta    8580
acgttggatt gtaatgacgg ttgtataatt ttataaatta ttaagagtga ttaaattgta    8640
tttttttaat aggtgagttt ggtaagtttt attttaataa agttgttgtc ggtatacgtg    8700
gagatgttta ttgggaggcg gttggtgcgg taatattagg aattgggagg cgtttcggtg    8760
ttattatacg taggaggagt atagattttt tttttgtcga ttattaggtt ttatacggtt    8820
```

```
tttttcggt tttgtttttg tcgcgttttt ttgtttatta gggtaattcg tagatagggt    8880
tttagggttt ttttatacgg tttttttttcg gttttgtttt tgtcgcgttt ttttgtttat    8940
cggggtaatt cgtagatagg gttttagggt tttttttatat agtttttggt taattttttg    9000
tatttttagt ttttgtcggt cggagtttag gttttagcgt ggatgttatt tggaggttgg    9060
tttttaggtt tggttatttg ttatggtttt gtttattgtt tgtagtttta cgttcgtttt    9120
ttttatttgg gggttaatag atagtaggat atcgtttttc gtgggagttg tcgttaggtg    9180
tagtttggta ggtttttttg agtgtgagac gtaggtatat tgtttttaga agttaggttt    9240
ggattcgttg tgagagtaac ggcggttatt gtagtattgg tttcgaggtt tcgggttttt    9300
gagtgttttt gtttttagtg agggttagga cggggtgacg gtaggagtat cggtggggtt    9360
tagttagttt agattttatt atttggattg tttttagatt aggtatttta gaatttgatg    9420
gtagtttttg agggacgtag cgggtagtta aggttatggt gatagttgtg aggagttagt    9480
acgtggagtt cggttattaa gttatgtggt ttgtgatttt cggggggggta gtaggcggag    9540
tttagcgagt gttggtagtt ttgaaatcgt gggtagtagg taatatgttt agagtttgta    9600
gtcgttagcg attttagttt aaaaagaagt tttaggaata gtttttttgc gtggagtagt    9660
atagtttgtg agaaatatag ggtcgtcggg attgggcgta ttagttttta tttttattat    9720
taattagtta ggtcgaggtc ggggttttag atttgtttag tttttgtatt ttggggaaga    9780
gagttgttat aattggaagg agtagggatt tattttttttg taagtatttt tgttaaggag    9840
ggggtatttt gagaacgagg tttttttgttt tcgggagttt ttcggggtta ttttttttta    9900
tttttattt tcgggttcgg ggtcgtgggg gttggtgttg tcggttgatt agggtatttt    9960
tttagacggg ttttttttggg tcgtttttat attagttagt cgttgtcggg ggattcgggt   10020
agggcggttt ttcggggaag ttgtggttcg attcggtttt tgcggttagg gaggaggcgg   10080
agtttttat ggtttttcgg tcggcggtcg tgttgagagt cggatttggt tatttttggt   10140
tagtagcggg cgaagcggag tatggaggga agagatagga cggtcggttg ggtagagtga   10200
tatataataa aaataaatag atagagagag aaaatagaga agaaatagta tgatttatat   10260
atatagttcg ggaggatttt ttagtatgtt tagtcgtttg gggtaggttg aggggatatt   10320
ttagtgtagg gtttgcggtt cgatttcggt atttataggg tttttttttc ggggttttag   10380
gatattttc ggtttttaaa agttcggtat tacgtttgtg gtatttagag acgttttatt   10440
ttttggtgag aaaagagatt atacgttttt gttttattat agaaggaagg gttaagagaa   10500
cgtttattta aaggtaggta ggggaggtag tagagggcgt agggagttgt tgtaggttag   10560
gttggatgaa gatttggggg gtcgttttat atagttgttt atatgttatt ttggagattt   10620
ttagaaggac gggagttcga ttatgttttg tgtatttata gagttaggtt tcgagaaata   10680
aggattttgt gaggttatgg ttgagggcgg gggtcggttt ttggggtttt ttttttagag   10740
gggcgagagt tatttttgtt gggaggtttg ggagaggtat aggttgttta gggtttttga   10800
tacgcggttt atttgtgttt ttgggaaggt tattttattt tttttggttg aggagaaatt   10860
aaatatagtt gagaacgtta attttgagac gggaggattt tgttcgtgta ggtagttgtt   10920
tttttagag ttcgatatat tttttttttt ttgttaagat tggggttaag atggcgtttc   10980
ggtaggttgg tcggttgtgg gttgattagt agtttttgtt gttatatacg ttatagtttt   11040
tttttagtt tttgattaag tttttgggat atatagttag cgttagggta gagtttagga   11100
ggaaagggtt aagtaagtgt taaagttgtc gtgcgtgggg gattatttga gtttaagagt   11160
ttgagattag tttgggtaat atagtgagat tttgttttta tagaaaatta aatattattc   11220
gggtgtggtg gtgtgtattt gtagatttag ttatttagga ggttgacgtg ggaggatggc   11280
gtgagtatta tagttgtatt ttagtttggg agatagagtt aaaaaaaaaa aaagtattaa   11340
agaatatatt ttttggagtt tagggaggtt tgggggtagt tttaggtgag cgggttgggg   11400
ggggtggggg tgtagtttta gtttttttttt cgcgacgatg gggagtagta gagggtaggg   11460
atgtgggggg ttttttttagt tagtgttttg atattcggga ggggtttgta gttttgaggt   11520
tataggggtt gagtttagcg aggtacgagt atttgggatc gtatagtaga cgcgttagtt   11580
ttttttggt tttttttat agttttgggg atagaagttt taagggtagg ggttgagggg   11640
gtgagataga gaattttgga tttttttttt tttgaggttt ggggtagaag tcgtcgagtt   11700
tttcgtgtgg tgataacgtt cgtttgaatt attttattcg gtacgagggt tcgttttttt   11760
tgaaggtgtt ggggtcgtgg gagggttgaa gtattgcggg ttttgtattt tggagatttt   11820
gttattttgt ggtggtttag gtttgtttcg taggtagaac ggttatagga tatacggggt   11880
tagtgtgggg tgcggattag agtttagttt taagtttggt ttagttcgta agttaataat   11940
ggtttttata tttttaaatg gttggaaaaa aattaaaaaa aaaaataata ttttgtgaga   12000
ggtgggagtt ttatgaaatt taggtttcgg ggttagtagg gtatagtttg gtttattcgt   12060
ttgtaggcgt ttttttatag ggattatttt gtatttatat ttgttttcga aggggtattt   12120
ggcggttacg tcggagagcg tcgcgtagtg tggacggggt tacgggagtc gtggcggatt   12180
ttttggtatt tacgtaagat aggtggaaag gaatagataa aataagtttt ggcgacgtgt   12240
tttgtgtgat tcgaggtatt tgagatatcg ttgcgttcgt gtgaagcgaa cgtaaaatat   12300
gacggagata tttaggattc gttttttagg tcgggttttt ggacgtgtta tattataggt   12360
tttgtagtta ttagtgttgg cggtttttat aaggggtagc gtttaaatat gtaggacgta   12420
ggcgaggttt acggggtttc gaatgttata cggaagtaag gtcgggagtt tagtacgtac   12480
```

```
ggagtaggtt cgttagtagg gagtttacgt attaatttag ggtttttcgc gtggttacgt   12540
tagacgtcgg aattatttaa acggtttttg cgtttttttag tttaggttac gtaggagttt   12600
tttacgggag acgggttggg tattttcgat attttttttt tgtagttaaa ttttgagcgg   12660
gttagggcgg ggtagggttt gggtgggagc ggcgattagc ggaggtgtta cgggatagaa   12720
gttgtttgta ttgggttttg agttggtttg gttttgatag gagttttgtt cgttttgtgt   12780
gaggattgta tatttgttag ggttaatttt agttttttcg ttatttattt atttacgtat   12840
ttatttacgt ggtatttggg gttacgcgtg tcgttagaaa tataggtttt tattaggttt   12900
cgtttgtttt ttattgatgt ttttaggttt aaggtagaag tggtgtttag ttgtatttag   12960
ggtaggaagc gagttgttta ttagtttttt gttttttgc gtttatttag aaaatttttt   13020
ttgagttgga gttagtttta ggtaattgat tttgtgggtt ttatttggat aggagggagg   13080
tttgttttag gagagtaggg atagtatagt ttttttcgt gggtgttcgg aagagaagag   13140
aggattttgt agagaaaagt aaacgttttt gttatagagg ttggatagtt ggttttgtta   13200
agtttttttc ggggtttttt tagtatacgt taggggattt ttagagatgg tatcgttgaa   13260
tcgtttcgat tcgttgttag aggagttaag agtttcgtga ggggtgtcgg gggttttgtt   13320
ttggttggcg aggtttatta aggagtaaga gttttcggga tatagttgtt agttacgtgt   13380
taatttgagg gttttatcgt aagggtgatt gtgggaaatt ggtatcgatg ttttttcga   13440
aatttatttt gttggttttt tcgagtgtag ttgtgggttt tgggtttttt tgtgtttcgt   13500
ttatttatga tggatagatg ttagtatagg atgagtagtt acggaaaagg tttttaggtt   13560
aggtgtagtg gtttacgttt gtaattttag tattgtggga ggtcgaggtg ggtggattac   13620
gaggttagga gtttaagatt agtttgcgta agatggagaa attttgtttt tattaaaaat   13680
atataaaaaa ttagttgggc gtggtggcgg gcgtttgtaa ttttagttat ttgggaggtt   13740
gaggtaggag aatggtttga attcgggagg cggaggttgt agtgagtcga gatcgcgtta   13800
ttgtatttta gtcggggcga tagagcgaga tttcgtttta aaaaaaaaaa aaataggttt   13860
ttagttcgcg tttttttaga gggagggaat tgtatttagg aggaagattt cgatgggagg   13920
gttttaggaa agaggagtaa gaagtggtgg gaaggaggga gggtagtatt agtagttatt   13980
tttagggata gcgtttagtt agggttgatt gtatttcggt cgtgggtgtt ggtttcggtt   14040
agatttcggt aggggggtta ttatgtttag ggttattgag gatagtaggg atagggtttg   14100
ttgcggttag ttagaggttt ttgggaggaa cgtttatttg ttatttttag gttcggttta   14160
ttttagggtt tgggtatacg tgaattattt agtgtagtgg tttcgaggac gtaggttagg   14220
ttagttcgtg gtttttattt tggtttttg tttgaataat cggggggggg ggggggcgtg   14280
taggggtatt gttattaagg agtgtgttta ggttaggtcg agggatagta gggtgaggtt   14340
ttcgtagagt atgtcgaatt ttgatttttg attttaggga tatgttttgg gtcgttgttg   14400
tttttgtagg gtttgtcgtc ggttaagtat ttgtgttgtt ttcgtgagtt gtatgtgttt   14460
ttcgggtttt tgttgtgggt taggttgttt taggtgttcg cgaatgggaa ttagtaggag   14520
tcgtgattat agggttgatt tttagggcgg cgttttaaga gtggtcgtga tttatgattg   14580
ttatagagtt aggttgtagt tacgttcgtt tttgggtttt ttggaggggt agggtgcgga   14640
gggtttcggg ttgtgtggat ttatgtttga tttttataag tttaattgtg tagtttttcg   14700
ttttttttgg gagtcgatgt tttagtttta aacggggcgg tggtttagat ttagttttag   14760
agaattgtag aggtgaggtc gttattgttt tgtggggata agtggttaat gagtagggtt   14820
aattggtcgg tttttgtttt gtatgttata tatatatttc gtgagggtag taaggagtat   14880
ttagttaggg gacgtgtacg agaagggtta ggatgattat attgttcggg gtttagtata   14940
gggttgagtt tcggaggtga ttattgtcgt ttcgttttat ttgcggttat tgaggtttgt   15000
tgggggtttt tggtgttggt tttgatagtt tattcgtggt aggatagggt ttcgaggtgt   15060
ttttcggtag gtggtgggtt tagtgggggt gattcgtggg gacggaacgt atgtttttga   15120
ggttttagtg gatgtaggta aaggtatttt tcggggttag cgtttttatt tggggagttg   15180
ggagtttata tgggagtagg gttggtatag cgatttttag ttttgcgcgg gcggtattta   15240
tagttgtgtt tatttcgtat atatttttta ttgtttcgtt aattaattat gtatacggtt   15300
gtattttcga gttttttttt gtttcgtagg tgatataggc gtgttttatt tatatagtat   15360
ttgtttttgg gttttatatt ttcgttaatt ttttatagag ttattttgtt attatgggaa   15420
ttatagcgat tttaataagg atgtttcggt gttatttttg ttcgagttgg gggtgtgggg   15480
ggtagtagat ggaaagcggg ggtttttgtg tttaggtagt tggtggtttg gttttgtttt   15540
acggtaattt tattgcgttt taaaattgta tttattagat tagaggttgt tggtttaggt   15600
aggaaataag agttagttat tatcgggacg aagtttagtc ggtgtttata atttgaagat   15660
aggtaatttt gtttttgttt ttattgacgt taaggttttt gtggaggaga tgattttaag   15720
gataggaagt ttttgtatga gtcgggtggg agggttgagg atagggtttt ttatcgtttt   15780
tataagaaat aaggttaggg tttcgttttg ggggaggacg tggatttcga tcgttcgttt   15840
taaatgataa ttggtaggat agtatttttt agaaataggt gtaggtcgtt ttttagggat   15900
taggtatttt tttggttgag gatagcggtg ggggtcgtgt ataggttttt tattagtttt   15960
agttttagcg ttgtcgtggg gatggggagt tttatttggg ggtcggggtt atagtatagg   16020
ggaggttttt aaagggataa atatttttgt cgggatttcg ggtcgttttt atcgtcgttt   16080
tttacgatag gattacggtt taaacggttg ggagaacgag gtttttttagg gtaggtaggg   16140
```

ttgtcggttt ttaggggttt tgtgaattaa gggtttcgag aggtttttta gtcgtgggta 16200
ttttaatttt gttgttttat aaatagtttt gtagcgtaag taaatttata aacgagtcgt 16260
agggtttggg aataaattta tttgaagttg tagtaagttt ttaggttttt tttgtgtcgc 16320
gtttgggttg ttgtttagaa agggaggtat ttaattaggt attttagttt ttattaggag 16380
gattttgatt ttggaatata gagtttcgtt tttagttttt tttgtaggag ttagagtttc 16440
gggaaattag tttcgggaga tttttcgttt tagaagttgt tcgagttttt gtttttgggt 16500
gtttgttcga gcgttaaagg tagttcgggg agtagtaggg gtttagtagg tttttttatta 16560
ttttggttta aggtttttga tttgtttagt gcggttaatt ttcggttagt taggatcgta 16620
gatagggtta tttaagattt tgttcggatt ttttacggtt cgattttgtt ataaggagta 16680
ggtcgtgggt aagagtttat gagcggggtt gtagagtagt attagttagg gagggcggtc 16740
gagtttttta ggtattgtta gggaaaggtg tttagtttgg gtaggtatag gtatatagtt 16800
aggtggttat ttagattttt tcggatttta gttcgagtgt tattcggtta ttatagttgt 16860
tagtagggcg gttattgttt cgagttaggc ggttttgttt ttttaaggtt atgtttttta 16920
tggcggattt attgcggtgg gtaggatgtt tacgtttttc gttttgtttt ttttgggttt 16980
tgtgtagggt ttattatgga ttttttattt agttattatt tttttttttg ttggttttaa 17040
atttttattt ttattttttg agatagagtt tcgttttgtt atttaggttg gggtgtagtg 17100
gtgtaatttt agtttattgt agttttcgtt ttttaggttt aagttatttt tttgttttag 17160
ttttttaagt agttcggatt ataggcgttc gttatcgtgt ttagttaatt tttgtatttt 17220
tagtaaagac gaggttttat tatgttggtt aggttggttt cgaattttag attttaggtg 17280
atttattcgt ttcggttttt tttagtgttg ggattttagg tatgagttat tatgttcggt 17340
tttttatttt tttttgtgat tagtatgagg atggtgtgta ggtgtgttta gtggtatagg 17400
tattatttgt tttttttttt tttttttttg atagagtttc gttttgttgt ttaggttgga 17460
gtgtagtggt gtgatttcgg tttattgtaa gttttatttt ttgggtttac gttatttttt 17520
tgttttagtt tttcgagtag ttgggattat aggcgtttgt tattaggttc ggttaatttt 17580
tttgtatttt tagtagagat ggagttttat cgtgttagtt aggatggttt taatttttttg 17640
attttaggtg atttattcgt tttgattttt taaagtgttg ggattatagg cgtgagttat 17700
agcggttggt tttttttttt tttttgagat agggtattat tttgttattt aggttggagt 17760
gtagtggtgt tatttcgatt tattgtaatt tttgtttttc gggtttaagt aattttagtg 17820
ttttagtttt ttaaatattt gggattatag gtatatatta ttacgtttag ttaatttttg 17880
tatttttagt agagatgggg gtttcgttac gttggttatg ttggttttta tttttttgatt 17940
ttatgtgatt tatttatttt ggttttttaa agtgttagga ttatacgtat gagttattga 18000
gtttaatcga atttttatttt tttttttttt tttttttttt gagacggagt atagttttgt 18060
cgtttaggtt ggagtgtagt ggtgttattt cggttaattg taattttcgt tttttaggtt 18120
taagtaattt tatagtttta gtttttttgag tatttgggat tataggtata tattattata 18180

tttagttaat tttttttattt ttagtagaga tggggtttcg ttattttggt taggttggtt 18240
tcgaattttt gattttaggt aatttatttg tttcggtttt tgaaagggtt gggattatag 18300
gtgttagtta tcgtatttag tttcgatttt tatttttaat aggaaaaaag gattagataa 18360
gtattcgtgg tttttggtat ttttttattt ttttgtttgt tatgtggtgg tagaagtagt 18420
tttggtttgg gtagttcggt gattttattg gtggttaagt agaagttttt gggatatagg 18480
gggtatttgt tattttagag aaggggacgg gggttttggg agtcgtgtgg ttttgggtat 18540
ttgttatttt agagaaggag acggaggttt tgggagtcgt gtggttttgg gggtatttgt 18600
tattttagag agggagacgg aggttttggg agtcgtgtgg ttttgggggt atttgttatt 18660
ttagagaagg agacgggggt tttgggagtc gtgtggtttt aatttagagg aggtttcgtg 18720
gtattttttt ttttattagt tttagttttt ttgtagttat ttttttagga ggggtaggaa 18780
ttttagaaag ttttaaaatt tattgttttt tttattatag gggataggga ggtggggggt 18840
agggagtggg gattaggtag gggtagaagg tgtaagaggt ttaatttgtt tgggggattt 18900
tatttataaa gggtagaggt tgggtgtggt ggcgtttatt tgtagtttta gtatttggga 18960
ggttgaggta ggaagattat ttgagtttag gagtttgagg ttgtagcgag ttatgattgt 19020
aattgtattt tagcgtgggt aatagagtac gattttggtt taaaaaaata aaatttaaaa 19080
aatgttaaaa taaagggtaa ttttttgaag agaagtgagg ggaaggttat gaggaggaat 19140
tttttttatta gcggagtaag ttttttttat agaggagtag acgttgtagt tattgtgata 19200
tttaaaaagt agcgatggtt ttgcgttttt ttgatatttc gtttaggatc gttagggagg 19260
cggggatggt aatttttgaa gttttttttt gattttaggg aatttgataa atcgttgtat 19320
ttggaggtta ggatggcgag gttgatgttt atgattcgac gggatttatg ggtttatttt 19380
ggtgttttgg atgagttttg gggagggcgg ttatattgtt ggttttttat gtagtattta 19440
ggtaggtttt ttagagtgtg taaagtaata gtttgacgta gtttattttg gggggtttac 19500
ggggtttttg ttttgggtta ggatgttttt atttttttat agattttatt tcgtgtattt 19560
tgtgggttta gtagtagttt ttgttttagt ggtataatgt ggttgtttat agattttacg 19620
tttagatatt tttttgggtt acgtatcggg aggtttttat ttagttgtgt gtgatttata 19680
tagtgatagg gtgtggttga acgggtttag gtttagtttt aatttatagt ttttttgtag 19740

cgagatatat atgtaaagat tataggttag taagtttgtt tttttagatt tagaaatagg 19800
tcgagtttag gtgtggtcgt gtttacgtta taggggttgt tgtgttattt ttttggagta 19860
cgtaggagtt tttttgggtt gttagaattt aggtatttag aattgtttgt ggtgagggtt 19920
aattttaggt gtttattgat cgtttgtttt gcgtacggtt gtatagcgtt agatattagg 19980
tttattaaga aatagaaacg gattttgttt tcggcgttat atttagtagg gagatagggc 20040
ggttttaagt gtaagtgttg taggtagatt ttatagtggg gagttaggga ttagttatag 20100
ggttatatga gaatcgtata gattttagcg tttttttttag cgtggggtat gagaggttag 20160
tggttttata tagttgaggt atagtttggt ggtgagaaaa tttatattta tatcgagtgg 20220
gtacgaggga tgcggaaggg ttttaggtag agaataagtt ttattgggtt tttgggtatt 20280
tttgttgtgt taggggttag agggtaggat tagagggata gagtaggaaa ataggagagt 20340
aaagcgtacg aggtagtttt cgggttgggg gttttttttt tttgtgtttt atgaggaaat 20400
tatttaagtt tatttttcgt gtttttttgtt attttagttt tacggtttag ggtcgagtgg 20460
ttatttagga tagatataga tttgataagt tattagtttt tttgggtttt tgttattgtt 20520
ttttaaagat tttaagttgt tttgtttaat gatgtcgttt ttttttttag tatttgttta 20580
cgtttgttgt tatgagtaga ggtattatat cgcgggttag agggttggat taggggtaat 20640
tcggttatta acggtcgagt tttttattga gcggtttagg tttatgaata atatttgtgg 20700
tttcggagga tttttcgggt agtagaattt ttttttttaa ttgtttcgtg tgaattggtg 20760
gaattagtag gagggagaga ttaggcgttt agtttttttc ggtttttttt ttgttatgaa 20820
tagttgtttt tagtttcggt tttttatttg tggtattggg gttatcggag tgttgtttaa 20880
ggttagggta agaagtaagt gaggggtgtt cggagagaga gttagtaggg tttgaggttg 20940
ttatatagat gtagtattat aggggattag ttattttttg tttttagaga ttacggtttg 21000
tggaggttta gataagaggt tttgcgtagt tatgtagtta ttgagtttcg ggttttttttg 21060
gggatttaga ggttattggt ttttttgtta attaaggagt tttggagagt tttagatata 21120
gatagaagta ggaaaaaagag gggtgttttt ttcgtaagta gaaggatttc ggaggaggta 21180
aggtatattt aatttaaggt taggttaagt tggttttttg taggatttgt tttttttaga 21240
taggttagaa aaataatttt ttttgtttta ttttatagtt gagaagttat ttagaatcgg 21300
aagggagaat tatttttata tttttgttta gttttagatt ttggaaacgc ggaggtaatt 21360
acggggtatt tttatattga attgtgattt acgttttcgt attttttaga tttgggtaga 21420
ggaagagatt cgggtttttc gaaggagaag tatataattt tttagtattt aacggagttt 21480
tagttttttt tattggtttt atatatttag gtttggtttt aggttttggg ttttattatt 21540
gtttttcggg ttttgttatt tgtagttcgg taataaattt tagagtagta agggggtttc 21600
gagagttcgt tcggttaggg ttagttgatt atataagagt tttttcggtt aggattagag 21660
gggttttttt tgtagttagc gggaggatag tagatttgta attttttagt tggtgtcgta 21720
gtagttttag aggaatacgg aagagaatat tggtatggtt ggtatagacg gggttttcgt 21780
tagttttttt acgttaggtg gtcgttttta ggtcgttttt taagtgaggg gagtgatgtt 21840
cgtattaagt ataatatttt taaggcggtt aggatcgtac gatgggttcg gatataaggg 21900
tatttaaggg taggtagggt cgaggtcgtt ttcggtgtgc gcggttttcg gggttggttt 21960
taagtgattt atatggttta ggtttttgtc gatttttcgt ttttttttgg gtttttttgc 22020
gaagtatcgt ttagttttgg tttttattag tttggaggcg ttagcgggcg gcgggttgat 22080
tttatttcgg gggttttgtc gtttttgttt tggttcggag ttcggcgcgt tcggttttcg 22140
aggtttttttg cgtttcgtcg agttcgcgtt tttcgtcgtt tttcgggttc gcgtttttcg 22200
ttcggggttt tcggttgggc gttcggtcgg gttcggtcga cgggatttat ttttatagtt 22260
cggcggcgtc gtttcgtttt cgcggttcgg gtagcgcggt ttcggttttc gggttcgttc 22320
gttttcgtcg gtcgatttat aatttttgtt ttcggagagc ggcggggttt ttttcgtcgt 22380
cggtcgcgtt gttcggacgg tttttcgttt tttagtcgcg gggcgtttcg attcggtttc 22440
ggtttcggtt tttgttcgcg ttcggcgttc gttttacgtt gttttcgtag cgtagcgttc 22500
ggttcgggtt tcgcggttac ggcgcgttcg gggcgtacgc ggcgttgtga atcgcgcgtt 22560
cggattacgg tttttatagg gttgcgcggc gtcgacgggg cggaagttcg gggcggtgtt 22620
tgcgcgaggt aggtcgggag ttgtagtttt cgggtggcgt ttttttagtt cgtcgatcgt 22680
tcgggttatt tttttttgttt tcgttgttgg agacggtcgg gttcgaggtt tcggttttcg 22740
ggaggcgtcg cgttttagga ttattaggga gtggggcggt tcgaggagga gttggaatta 22800
taatttttag agggttttgc gaggggtacg gaggggcggg gtttgtagtc gcggcgtttg 22860
cgtattagcg gcgttataga cgcgtggggt tcgcggggag tcgggttggg gtcgttcgtg 22920
tttcgcgggg ttttgggcgg tcgttcgatc gaggatttac gatttcgttt cgatttcgtc 22980
gtgttcgcga gattcggaga ttttttcgcg tttttttagga tcgttttttt cgcgacgttg 23040
cgtgttgtgt ttggttttgg cggttaagag cggcggtggt aaggggatcg ttttggttcg 23100
agacgcggtt tggtttcgta gcgtcgtttg ggtatagttt ttttaggttt tttcgttttc 23160
ggtcgcggag ggacgttatt aaacggttac gtcgggttta attgggacgc ggtttacggc 23220
gtttaattag ggtttggggg agggaagtcg gggcgatttt tgggtttttt taggattttt 23280
gcgtaggttt tattttttttt tcggaggttt tttttcgtt gttttgaggt tatttttttg 23340
ttagtagttg ttgggtcggt tttcgggaga atgttttggc gttaagttta ttaggttgta 23400

gggcgggacg cgagtgttga gggtagttag attttcgttt ttaggtgtat ttgaggagtt 23460
tgagaaggta gtggttttgc gggagaggga gatataggag tagggttggt tcgtcgagat 23520
ttcggagaga cggttgtggg tcgattttgt ggcgagattt ataagtggga ggttgggtga 23580
cgattgtttt tgggttagaa aatttaagtt agttttagta agatttataa ggttagggtt 23640
agtgtttttt agaggatatt tatgttttag gaaagttaag gatgggtttt cgttatgttg 23700
agatgtcgta gtaggga 23717

<210> 151
<211> 17265
<212> DNA
<213> Artificial Sequence

<220>
<223> chemically treated genomic DNA (Homo sapiens)

<400> 151

gtaggcggat tacgaggtta ggagttcgag attagtttta ttaatatgtt gaaatttcgt 60
ttttattaaa aatataaaaa ttagttaggt atggtggtat acgtttgtaa ttttagttat 120
ttaggaggtt gaggtaggat aattttttga attcgggagg cggaggttgt agtgagtcga 180
gatcgtatta ttgtatttta gtttgggtga tagaatggaa tgagattttg ttttaaaaaa 240
aaaaaaaaaa aaaaagaagt tttagatttc ggttgtgttg gttgtaaaag gagagattta 300
gtaagtgggg gttgtgtcgt agattgttat ttataatgga cgggttattg agtaggttcg 360
gttaattggg cgtttttttcg ttggagggtt agtatattag attgtaggtg gcgcgggtta 420
gtaaggtatt aggggatgtg ttatatatat agtttatttt cgtttagtta cgtacggata 480
ttttgggttt tcgagtaaat ttgtttttac gtggtgtgat tatatggagt tatagatatt 540
tagtaaggat acgtagttcg tatattttcg gtattttaga tatagtgatt tgtattaggg 600
ttcgaggttt ttttagggaa tttatttttt agaattattt agaaataagt tatttttttat 660
ttgtttagta aaggtttgtt gagaggtgta tagtgtttgg agtttaagtt gcgttaaggc 720
ggtaggattt ttagtttagt ttaggatttt tagtagagtt tttattttag cgtggaggtt 780
tgagaacgtg aggaaggagt tgtttagtac ggacgagttt aggtagttgt cgatgtttag 840
tatttattgt tcggtaggtg tggggtttag gtttttagtt atttgtagga cgacggtagt 900
ggttagcggg cggtagttta gatttgttta ggatagggat gagaagttat ttttttagta 960
gataggatag agttcggtgt tatttaatag aatgttttag aatgttggat atatgggata 1020
tttgtatcgt tcgtgatggt agtttttcgc gatatgtgtt attgaatatt tgatagtaga 1080
ttggtgtagt taaggaatag agttttgaat tttatttttt tttttttttt aagatggagt 1140
ttcgttttgt tatttaggta ggagtgtatt ggcgtaattt tagtttattg taatttttat 1200
ttttcgcgtt taggcgattg ttttggttta gtttttttgag tagttgggat tataggtgtt 1260
tgttacgatg tttagttaat tttttgtatt tttagtagag acggggtttt attgtgttgg 1320
ttaggttggt tttggaattt ttgattttaa gtgatttgtt cgttttcgtt ttttaaagtg 1380
ttgggattat aggtgtgagt tattacgttc ggttatcgtt ttattttaat taattttaat 1440
acgagtagtt atatgtggtt tttggttttt gttacggatt cgggagtaat tttttttggt 1500
cgcggtttat gcgtttttttt tgtgtgttgt tggggttagt ttgtatgtaa tttttttgagg 1560
attttacgtg tgtattttta aggggtgcgg tttttcgttt tcgtatgaat gggaagagtt 1620
tttatttgtt gtatttttgg aaagagtttg tgaaggattg gtgttaattt tttttttaatt 1680
gtttagaaaa attttatcgt gaaggttttg agtttaagtt tttttttgtg ggattttttt 1740
tttttttttt ttggagatgg agttttgttt tattgtttag gatggagtgt agtggcgtaa 1800
tttcggttta ttgtaagttt cgttttttgg gtttatgtta tttttttgtt ttagtttttc 1860
gagtagttgg gattataggc gttcgttatt atgtttagtt aagtttttgt atttgtagta 1920
gagatagggt tttattgtgt tggttaggtt ggtttcgaat ttttgatttt aattgatttg 1980
ttcgtttcgg ttttttaaag tgttgggatt ataggcgtga gttatcgtgt ttggtttttt 2040
ttaatgtttt atatagatgg ggttttgtta tgttgtttag gttggtttta aatttttgga 2100
tttagattcg tttatttcgg ttttttgaag tgttgggatt ataggcgtga gttattatat 2160
tcggttcggt tattgggagg tttttaaggg attaattcgg tttttttatt tgttatagat 2220
gtattgagat tttttttttgg agtgtatttc ggaagcgtgt atagtcgcgt gtttgttttt 2280
tttgagttat ttggcgtgtt gttgtgtagt tgttcgtggc gtttgtttag cgaagtgttt 2340
tcgttttttt acgattttgg tttttgagtt tttttttttt ttttttggtt agtttagtta 2400
aggttgttta agtgtgttga ttttttttcga gtagtttttg gtggacgttt tttttttttgg 2460
ttgtagtatt ggaaagtggc ggttttgggt atggtgtcga ggtttaggtt ttatttttag 2520
tattttcggg tttttagttt tagtttattt tgtttcggga tattcggaag agaaagagga 2580
tggttaggta gacgggatag ataattacgt tggatattag gttaatagcg attgtgtcga 2640

```
cgtttagcgg gtttagtttg gatatatgtt tcgtgttgtg tggaggagag gaggttatat    2700
aggtgaggtt gaggggtagg aagggttggg taggaagagg ttgtttcgat ttttacggta    2760
tttatttgta ggtagagtta ttaatagttt cgtattatac ggcgttggcg tttaggaaga    2820
ggtagatgag gagaacgtag taggtggttt tttggatgcg agtgaaatag ttacgaggtg    2880
gtcggtttta tatggagagt tagatgtgtt tgttaaagaa gttacgttgt agtttagtta    2940
ttagtaggcg tcggaagcgt aatagggttg cgtgatttag aaggtaggga gggtcgtatt    3000
gtaggaggtt acggggtagg attattttgt ttaatttttt acggagtggg aatatggaac    3060
gaggttttat tcgtagttag tatttttttt tttattaggt tttcgttggt tttcgttttt    3120
atcgaaagtt agttattgat taggaagaag gtgttgcgtg tcgtttgtag gtttttgacg    3180
atgatgtgtt gtaggaatta ggtagggttg agttttgtag aggcgcggga gggaggttag    3240
gttcgtaggg cgttttaatg cgggggtaga ggggtagagt ttggtagggt tcgtatagat    3300
ttttgttgtc gtgttatatt cggatttttt atacgttatt taggttgtgc ggggtggcga    3360
ttcggaagat gtttagattg ttgcggtgga aggttttgtc gtcgtttagg tgtcggtggt    3420
cgtttcggtt gtttatttta tatagtatga tgtttacgtg ggtcgtggta tttggagggt    3480
aagagggagg ggtgggaggt tcggtttgtt gtttaatacg tgtggtattt taggtaagtt    3540
attttagttt tggttttcgc gtatttaagg agttatatag gtagtttcgg ttttgtacgg    3600
ttttgttata tacgaggagt tgcggttatt gtaatttgtt taataaatag taggatttta    3660
aggatatgat taagttatac ggaaagaatt gtaatttgtg atatgtaaat atggttgtat    3720
acgttttagt ttatattata attagtgatt cgcgttgtat atttgtttac gttttagtta    3780
cgttataatt agtgattcgt attatatatt cgttttttag tttatgtata gattgcgaag    3840
cgtgaagttg tgttattttt tttttagtg atagatttag gtgatagtat tttttttttt    3900
ttttttttg agatggagtt ttgttgtgtt atttaggttg gagtgtagtg gcgtaatttt    3960
agtttattgt aagtttcgtt tttcgggttt acgttatttt tttgttttag tttttcgagg    4020
agttgggatt ataggtgttt gttattacgt cgggttaatt tttttgtatt ttttagtaga    4080
gatagggttt tatcgttagt taggatggtt tcggtttttt gatttcgtga tttgtttgtt    4140
tcggtttttt aaagtgttcg gattataggt gtgagttatt acgttcggtc gatagttttt    4200
aaaagtaggt aattaaaaga attgggaaat gaagatgaaa gtaatacgga aataaaaaat    4260
gggaatatag ttaggtgtgg tggtttatat ttgttatttt agtattttgg taggtcgagg    4320
taggcggatt atttgaggtt aggagttcgt ttggttgata tggtgaaaaa ttaattgggt    4380
gtggtggcgt gtatttgtat ttttagttat ttaggagaat cgtttaaggg gaatcgttta    4440
aatttaggag ttggaagttg ttgtgagtta agattacgtt attgtatttt agtttgggta    4500
atagagtgag atttcgtttt taaaaaaaga aaaacgaaaa taaaaaggga atgttagaag    4560
ggtaatttta ataaaggaaa atggaggtat tgaagaaata gttacgggga gggtgttggt    4620
attttcgttt gagagacgag ttatgtagtt aggatcgcgg gtggatgtat ggttttttat    4680
agtggtagcg atgtttatgt tatttgtggg gttacgttat tgtgtagaac gtgggttgtt    4740
tattttgatt gattggtatt tatttttagt taggagttgt tttagttttt agggatagtg    4800
agtgtttacg aggttatttt taggatgaat atacgagttt tttatatagt attgtaaaaa    4860
ttgttttgtt ttgacgtttg cgacgagatt tatttttaga gggtgtaatt agtatagtta    4920
gtgagagtag gggaggtttt gttatttcgt cgcgtttttt atttgagttt cggttttagt    4980
ttgttttgac gaggatttcg tatttgaagc ggtttcgttg tttatagaag gggatggcgt    5040
agtttcggtt ggtatttaat tggtttagtt tgtgtaggat ggcggttatg attatgtagg    5100
ttattaggta tatagtatat gttagtatga cgatgtagtt tatattcgtt gtcggttttt    5160
gtgaagatat agtcgtcggg tttaggaggt tacgtgtaag ttgtgttttt ttaggataga    5220
gtcgagttta tttaggtttt tttcgatttt gtagaggttt ttaggagtat agggttattt    5280
ataggaaata taaagcgtat atggtttggg ggtatgaaga ggttggcgtc gaaggcggtg    5340
aggtggcggg tgaggtagac ggtttggcgg ggcgaggttt tttttagggg tagtagtttt    5400
tttgttcgtt atattatatt tttttcgttg aagtattggt atagggacgt gtataagttt    5460
acggatattt ttagcgtcga ttagcggaag tggttggaga ggtttagacg gtaatttttt    5520
attgggtttt tggttttggg aagggaaggg gtagtggacg tgagtttagg tttcgttagg    5580
ttggatgtcg gagttttaga gtttatattc ggtttagttt tttcgttgtt tgtcgttttt    5640
acggggttcg taattcgggt aatgttgatt tatgatgttt tgttttgttt tgttaggtcg    5700
gttcgtagag tttatttcgg ggaaatgaag aaggtgtagg gtcggtggtc ggtattttgg    5760
agggattttg ggcggatttt tttgttagtc gagtagttgc gtttattggg tcggggtttc    5820
gagtgtaggt agattgttag gtagggttcg ggttttttag ataggtagcg gtttggggta    5880
gaacgcgtag gttatacgtt tgtcgggaag tttaattatt cgggggatat ttacgatggt    5940
tttttgagt ttattttttg ttacgggttt gaaaggttat aggagttttt gtattagagt    6000
tggtatttgt tttttcgtgg tttttagttt tttttcggtt aggtttttaa tagtttatga    6060
aatagtaaat tttattagag atatttatgg aagttttacg agaaacgttt ttttttaag     6120
aataaggtta gggggtcgcg tgtgttttat tcgttgtata tatcgtttag tagcgtatag    6180
ttgagttgta gatgtagtac ggttataggg ttgttgttgt ttagggtgat tatagtatcg    6240
acggaggttt ggggttggat tataacggag ttggcggagt tgcggtggtt tcgggtagtt    6300
```

tagttcgagt tgttgggtat ttttacggtg atggcgcgtt ttgaggttag tcgttcgatg 6360
gggatttggg cgtcggtttg tgtttggaac gttatcgagg ttattttggt ggagacggtg 6420
tagttgttga tatagttaaa gagaaaggga ttggagttta ttagaaagac gagttgtatt 6480
acgttattga ggttggcggg ggttttgttg aaagtttagg ggatggagag gtggtagtta 6540
ggttttgggg cgtcgttata gtatagtagg tttcgcgggt tcgagcgttt gttttgggtt 6600
atgatttttt cgttcgttag cgttacgggt ttttcgttga gtacgcggga gcgcgtgagg 6660
atgcgtatga gggtagaggt taggttgtag gtttgggacg ttattattcg taatggtgat 6720
tcggttttta gttttgagcg ttgcggtgtt cgtacgtttg agttggttag gtggatgagg 6780
ttttttgtag ataggcgtga ggttagtgta gagataggga ggtagaggga gggtgggggt 6840
aggtaaaaag ggggagtcgg agggtggggg ttgggagaaa gggggaattt gagggggtag 6900
agagcgaggt gtaggtagaa ggaagaggga agttggagag agagtggtgg agggggaggg 6960
ggaaggggat ggggatgagg acgaagatga gggggatgat ggggagaggg aggaaaaagg 7020
aaggaaaagg gtagagaaaa gagaaagggg agaagaagag gagtagggtg aaagggaggg 7080
gaaggggata aggggggataa gggaggggaa gggggataag ggaggggaag gaggataagg 7140
gggataagaa agatgagggg aatggataaa aggacgggga ggatcggggg ggaaatggag 7200
aaaaggggag agagatggag aaaagggatg gtaataggga agggggaggg ggaggagaat 7260
gggaattggg ggagggggat aaggatggga attgggggag ggggatgagg atgggaattg 7320
ggggagcggg atgaggatgg gaattggggg agcgggatga ggatgggaat tgggggaggg 7380
ggatgaggat gggaattggg gggaggggag ggggacgaag atgggatggg gtaaaggcga 7440
ggcggttgtg gggaggaggg aggtagagga aagggcggta tggggcggac gggttacgtg 7500
gggcgggcgg gtggcgtggg gtacgggtcg cggtatttgt gatgttgagg atgttgtttt 7560
cgatggcggt gggcgttacg gtgttcgcgg tggtttttgt ttgtaggatg cgtattatgg 7620
tttttagttt gtgtagcgtt tgttttaggt acgagcggta tacgagtttt ttgttgggtt 7680
tttgtgtgga gttagtagtg tttagtttcg tttttggttt tatttttttgt atacgttttt 7740
ttttttatac gggtttttat ttggttttta tttttagttt tgtagttgga gagtttattt 7800
gattggattt ttatagtttt tttattaagt atttttttgtg gttttgtatg atttagggtt 7860
tttatttggg gaatacgtga tgtagtttat cgattatata aggtattttt ttatatgaga 7920
gaaggaggag ggtagaaggg agagaggaga gggaagtgga gaaaaggggg gagaggagag 7980
ggaaggagag agaaggggga gaggagaggg gaggggagag aaggggggag aggagggagg 8040
gggaggaggg gaggaaggag gaggggaggg gtgaggggat ggaggggttg ggggaggagt 8100
ggaggggttt agcgggatga ggtgagggga aggtttaggg gaggggagga ggggaagggt 8160
taggggaggg gaggggttgg gggaggaggt aggggttagg ggaggggggga gggattaggg 8220
gagggaaggg ggagggggagg ggttagggga gggaagggga ggggaggggt taggggaggg 8280
aaggggggagg ggagggggaga gtggagggta tagagtagta tttttttagt ttttttttat 8340
atttgggttt ttttttatat tttgggtttt tcgagaggta ttttgcgttt atataggata 8400
gtagaatggt tgaggttatt gaagtaggtt agagatcgag gaacgttatg gtaggaagga 8460
gtttaggttg gaggtttagt ttttcggtta agttgttcgt ttgttttggg gggttgaatt 8520
tagtattttg gtaggtatgc ggggcgggga gagtatgtgg ggttatttta ttatgtattg 8580
ggttagcgta gtagcgattt gttggatgtt atttatagtg tggattttta gggatattag 8640
agttttcgtg atgtttttgc gtatttgggt tcggcgttgt cgttcgtgtt tgggttttgt 8700
cgttacgttt agggttcgtt cgtattgggg taggtagggg gtatagtaag ttgttagtag 8760
ggtaggaggt cggtaggagg ttagtagatg tttacgattt tcggggtgta gttacgtgtt 8820
agatgttgtg tgatgtgggt attgattcgt aatattgagt tgttttttta tgggtaaaat 8880
agggtaagta tatgggtttt tttgggcggg ggttgtattg tggaaagtag acgtcggaga 8940
gggttcggtg ggtgtggttg ttgggagcgg aacgtcgggg tgttgtttta gggttattgg 9000
gatttatttt tggggttcgg gatgagtttt tcgtaaagtt ttaggtaggg gaataggttt 9060
tggtttttag tacgtatgta gtagatgtga ggtttttttt taggttgtat ttatttcgtt 9120
tagtatagtg attagggtta gcgagtattc gatgacgtgt tggggatcgg tttgtcgtag 9180
tagtttcggg agtatattag cggtgagttc gtgtagttag attgtgagtt ttattgcgtt 9240
gtcgttgggt tttgggaggg tgatggttag agatttacga gtagaggggg gtggtgagta 9300
ggtggtagtt tcgggggcgt ttttttacgg tttggtttat ttgttgaggg cgattatagc 9360
ggtttttagt tggttttgta ttattacggt taggtttatt tcgaagtgtg gtttgaaatt 9420
cgggggtagt acggtttcgt agtcggagag gttgtttttg tagatataga atttttcgta 9480
gtggttttgg cgatagcgtt gtagtagtag ggcgtatatt agcggggcgt tagtattttt 9540
cgcgttatgt tagtttgagg gacggttttt acggtattac gggagggttt cgtgatttta 9600
tagagtcggg ggatttcgtt gttttttttta cgtaggtttg tatttattta tgtattcgaa 9660
gtgtattttg gtggtgagag cgtgtatagc gtttagtggg aagaggcggt aagagttttt 9720
tagcggcggg cggttggggg ataggggggat ggaggcgtag tttttttttt tgttagagcg 9780
gtttagtatc gttagcgtga aggtgtattt ttcgtcgttt cgtagtacgt ttcgtcgtag 9840
tattagttat atgtttgcgt tgttcgtgga tgtggtggtt ttatttagta ttagcgtttt 9900
gttgttgaac gtacgtgtag tttatcgttg taggtagaag ggatggtgag gggcgtaat 9960

```
tttttgtttt gttagtttta tttttgtttg taggtttcgt tttttcggtt atgggattta   10020
tttttaattc gtttatattt cgtttaatat ttatttttcg tttggagtcg ttgttgtaat   10080
tgaggtagcg gtttttttagg tatacgtagg agttgcggtt tatttcgtat acggtttgtg   10140
ttttgtagga tatatatttt aaggatataa tgggtattcg gttattgcgg attagtattt   10200
ggcgtgggag tggggttatt tttaatatag gtttatttgg tttgttggaa ggtttggggg   10260
attcgtggag gatgttgttt ttaaatttta ggttttttag gggtttggtt attgtcggtg   10320
agtttatttt tttttaggat atttattcgg tttgttattt tttaatttgg gcggcggaag   10380
ggtatatata gggtagagga tattggggtg tgcgttttgg tgtattggag ttagttggat   10440
tttggtagga ggtaggtaat gtttattgag ggtttttggg gggatgcgtg tgggaataga   10500
cgtatgtgtg ggtgtgagga tcgtagttgt tacgtaggtt tgatttatag atttttgtag   10560
tttttagtta gggtttgggt taggaggttg agtcgggatg gaatttgttt ttatattttt   10620
tttttagacg atttttttgg gtagattttt aattaggtta gttgaggaaa gtagggattg   10680
gggaatagat atttattttg gggtattagt aggttttagt tagggaggcg tatacgttta   10740
tagagggtag ggaggcgtat acgtttatag gtatttgttg cgttcggttt tcgaaggtat   10800
tagatgttag taaggttagg acgtatttat ttgtggggat aggtttaagt ggggtagtcg   10860
cggtattttt atttgttttt tattcgttcg gtagaagttt ttcgtttgag gagttcgggg   10920
tgaacggttg tatttgcggt ttagttttaa gggttttagg tttttaagtt acgtgcggga   10980
cggagtatag gtgtagtagt attgagggtt gtttggtgag gacggtatcg tttttaagtg   11040
tagttgtatt cggggtagta gagtagtaag agttaggtcg cggtgggggg tagtttaggg   11100

ggtttagttt ttttgtttat ttttttttacg tttggttttt tttttatttt agtaggggtt   11160
taagttatta gtttaggtga ggttatagtg agggttgttg gggaggaagc ggggtagttt   11220
gattggggga ttggggggggt ttcgtgttta gagtttgaaa ggtagtggtt tttttatttt   11280
tttatttttt atttggggta gcgtaggtgt gggtgatatt gtgttttatt agtatttggg   11340
ttatcgaggg gtttggaatc gggaaggatt cgttgtacgg aggttggaat tggtggaggg   11400
tttgtttttt atttttaaag gtttatttgt cggggcggtg ggaggtagtg agtgaatcgg   11460
gataggggtg cgtagtggcg gggtataggt gcgcggtggc ggggtagggg gtgtttggga   11520
tttagtcgag gttttatttt gtagttacgt ttcgggtttt tatttagggt ttattcggtt   11580
gtgttgaggt ttttttcggt tttcgtgtag ttttagggtt tttgtgtatt tagtattttt   11640
taatagatag ggaaatcgag gtttagaaaa gtaatttttt gatgtggggt ttttcggttg   11700
aggttggagt cgggataaga gtttggtgtt cggataagag tttggtgttt attttaaatc   11760
ggtttttgag ttatttatag gaaggttatt tttacggtcg agtttattag tacgttcgtc   11820
gttagtgtta gcgtggtatt gggggatagt acggttggta ttgtagagat tcgtaggttt   11880
tgtattttgt ttattcgttt tacggtgatg ttgtagttta cggtgacgtt gtttacgtgg   11940
ttggaggtcg ttagttgtag ggataggtat tagtgggttt aggtggtagg tgagaggttt   12000
gttttgtttg gcgttttttt ttttatttat tatagttatg gtagcgtttt cgggtagtat   12060
gaagtagagt agaaggtaga ggtgaaggtg gagttcgttt tcgtttcgtt ttattttttt   12120
ttttttttat tttcgtttat ttattgagag tttgaagatc gtcgcgtttt gataaatgat   12180
attgaagatt acgttttgga aggttaggga ttgtttgtcg ttgatggttt atcggaagat   12240
tatgttcgag tcggttttta ttacggggtt gtatttttgc ggggggattg gtgttagttt   12300
gggttttgtg gaggattttg tttttagttt gtcgtttttt ggatatattt ttcgtcgggt   12360
tggagagttt tacgcggggt atagaggaga ggaggtgttc ggggttttgt atgttatgag   12420
agttaagttc gggttgggat attgattgtt cggtttttta gttagttatg tagtattatt   12480
aatgttttaa tttttttgtg ttttagtttt tttatttgta aagtaaattt agtattagtt   12540
ataaagagtt tatttttttt tgagtttttt tagattttttt cggggttttt gttttagttt   12600
tttagttaga gagttcggag tagtgagggg aggtatacgg gttttatagg gatagtattt   12660
atattggttt atagaattta ggataggttg tataggttac gttatttttg atggtttttt   12720
ttaaggtttt tggtgaaggg gtaggtattc gtaagatgga atagttttgt tttttatgta   12780
tttagtatgg tggtattgcg ggtagttcgt agttttttat taggggttcg gattttattt   12840
taaaagttat ttgttttagt taggcgagaa tatagtagag ggcgtgagag atttacgggg   12900
attcgtgtga ggttagggag cggagtttta aatttatttc gtgaaatgag acggtggaat   12960
gagttagcgg agtcgttgtt agagtcgtga ttttttagga atttaaagtt tattaggtag   13020
tttgaggagt tagttagtag gatttgttcg gggtcgacgt ttttagtaat tgggttgttg   13080
tttttattgg gaagttaggt tttaagtttt gtgtgagtat tttgtttgta ggtatttgtt   13140
tgggggttgg tggtggagtt tcggttatat ttattattgg gatttttgt agtatacggg   13200
tttcggggtt gggcgtggcg cggaggttat agatgggttt tttcgtcgtt attcgtaggt   13260
tgaggttggt tcggttggcg ttgtttttta ttataacgtt tattacgtgt ttttttttat   13320
cgagttacgg tagtgttatt attgagaata gggtattatt ggttttttag gggtagtcgg   13380
gtacgaaggt ggttattagg gtagggtagg tatttttaaa gcgggcgttg atattgtttt   13440
taggttagcg agtcgtggtc gtggcgttgg tattagagtt tatttggagt atcgaggttg   13500
agtcgttggt gggtacgtag aggcggtcgt cgcggggggt agggtagatg attcgtagtt   13560
```

tagttattgg ggagattacg ttaaagttgt aggataggtt gtgtttggat acgttattgt 13620
ttatttttgt tcggatttta tagcgtttag gtagtcgtag tttagggttg ggttttaggt 13680
ttagtagtac ggtgagttgt ttcgtggttg taagtagtcg tagggtatag gtagggtagg 13740
tttaagtgtt ttttagttgg gttggtaagt ggggtagtta tgacgaggcg ttggcggaga 13800
ggtacggggt ttggggatta gggtggtcgg gagtcggcga gtagtgtagg agggcgttag 13860
ggttagcgtc gtgttgtaag ttaacgaggt tattagggag tatgaggata ttttggttgt 13920
ggagggtgat ttgtggagag ggaggtaggg ttgtattacg tttttacggt tatggttcgt 13980
ggatttttgt acgacggatg agggtggata cgtagggttt ttcgtttcgt tagttatatt 14040
ttagggagtt tttttatagt gttcgtgata aggataggta ggatagttgt agataggggg 14100
atatacgggg agaggatata ggttaagatt tggtagatag gaaggagcgg ttgtgttggg 14160
agagaggaag aggaggtata gttcgtgtta agggtttagg cgagagtttt ttttattggg 14220
agaggggtaa gggtattgta gaggtcggag gttggaggtc ggaggtcgga ggttagaggt 14280
ggtaaggacg tgggaggggt ttgtaggttg ggtgtgtttg ttgcgtagat ttagattttg 14340
ggtagtagat aggaaggtgg tttgaggaga tgtagggaat agatttaggt tagggttata 14400
tatcgagtat tgcgcggggg gtttcgcggg aacggagaag aggaattttt tttatagcgt 14460
ataggggggtt tcgagtagtt ttggttttga cgtgtagtta ttggcgtagg tttgggggtg 14520
gtaggaggcg tttagcggta agtagatgtt ggttttaggg tattagcgtt tttttggtat 14580
gtacgcgggg attagttggg ttttgttgtt cggggatttg tttttaggtt cgttgtcgtt 14640
tttcggggtt tttgcgagga ggggagggtg ttggggtttt tattcgttta cgggttatcg 14700
ttagagatgt ttaattgttt gtattagcga gtttggtttt gttgtgagga taggtttttt 14760
cgttcgggta gtatttttag tttagtgttg cgtttttgtt ttcggttagt tgattgattt 14820
aggtcggttt ttaggtaggt tttattcgat ttattttttag gatatttgga atgagttggt 14880
gtttttggaa tttttgtttt gtttatttaa gattgggaat tattttgatg gttataggat 14940
tagtagacgt gagagtttag agaggttatt tcgagttttg cggcgtttat tattttagag 15000
ttttatttgt tgtgttgagg agtcggtata tttgtagtcg tagttgggcg ggtcgtcgga 15060
gtttttgggt tttaaattcg gtcgtggtga ggaaggtttt acggtttaga cgtaggttcg 15120
ggaatattat gatttggtgg gtagggggtc gttttagttt tatagatttt attttagttt 15180
gaagtttaga tttttttttat tcgaatttcg taggaagagg ggagggaagg agagcgagtt 15240
atcggatttt tataggtttg gtttttgtcg ttcgagagga agatttcgat ggaaattgtt 15300
tatggggggt aggattttttg atttgttttt taggaataat ttatttatat ttagagaaaa 15360
ggtttggggg taatgtgagt aaacgttttt ttttttgtat tttggatttt tttaattatt 15420
tttattgggt ataagtaata ttaaggtttt taagtttttt ggcgagattt atagtgggta 15480
gggtaggcga ggtttttagg ggtaggtagg agggtaggtt atagaacgtg gggggtcgat 15540
tatttttacg ggttcgtgcg gggttgagag gtcgttttgt cgtgttagag gtattagggg 15600
tttttatagg tttttattgg gcgtttttac gaggaggttt tcggtatttt gtattgggtt 15660
tggggtggta agtgtatagt gaggcgtcgg gttagggttt aggatattag gacgaataga 15720
ttggggatcg agtcgttcga gaattttttt attagttttt tttttttagt ttaggtttta 15780
tcgcgggcgt tcggtaggtt tttaattata gttagcgttt taggtttttg tttggttttt 15840
cgtatatttt taggtcgtag ttcgtagacg tagttgtgcg gcgttgagta taggtcggtg 15900
ttatattatt cggtgggttc gagtcggacg tagtgttcgg ttgtggttgg gtgtggtttt 15960
tcgggtagtt agttttggta gtttttttagg ttgaaggttt cgttttgcgg cgttgggttt 16020
atttttattt tttgtatagt cgagaagtcg atttatatgt ttaggttttt gggggcgggt 16080
gtgggatggt agggggttta gggtattttt ttattttttt atttttatag tagttcgttg 16140
ggagtttcgt tattgttttt tttttagat ggggaaattg aggtttagag ttcggagagt 16200
agggtttatt agtttaggtt tatagtagta tttatttacg gggtttgtgg gtatcggtag 16260
ggattttcgt gtaggttatt tttcgtatgg cgtgtttagg agtgttcgga ggttgttttt 16320
agttcgcgtt tatttttgta tttgtagagt tgataggaac ggttttatcg gtcggcgtta 16380
tttgtttatt agggtcggtt tagtttttat tttttttttt ttgagatttt ttagtcgtag 16440
gttttgtttt attgttttag agattttttta atttatggtt tttcgggggg tggggtaggt 16500
atttggtgat tcgggagatt aggaagcgtt gtacggcggg attgtttatt attgttaggg 16560
tggtttcggt ttaggttcga tattgttttt gcgtttgtag ttaggtcgtt tttttttatta 16620
ttaggcggta gtagtgttta ttgttagaga agattttcgt gttcgagggg tagagcgggt 16680
gtatcgttgg agattagtgg gaacgagggt gttaacggtt agtgtgggtt taagacgggg 16740
gtattaggtt ttgttttatt tggatggttt tggggaggaa ggggagtggg tagtagatat 16800
ttatttcggg tcggtttttc gtttagggtt acgatgttgt aggcggtttt taggtttgaa 16860
ttatcgcggt tttggatgtt gaggtcgagg ttttcgttat tttgtatcga ggacgggtat 16920
acgagtttta gggcggtagg tgtcgttttt atttgtacgt ttgtttttag tagggttgag 16980
tcggttttta gggttagtac ggtcgttacg tgatagcgtt taggtagtat atagcgatgc 17040
gaggtagtcg gtttagcggt atttatttcg ggggagtcgt tttcgaagtt ttagcgtgtg 17100
gtagtgatag ggagcggggt agcgatgtgg aaggttgtta gttggtcgga ggttaggggt 17160
tcgtggggtt ttattagggt ggtttttggg gaggtaggga agacgtgttg gaggagggtg 17220

gggtttttat aggtgggggt aggaggcggc ggggggtcgg agtag 17265

<210> 152
<211> 17265
<212> DNA
<213> Artificial Sequence

<220>
<223> chemically treated genomic DNA (Homo sapiens)

<400> 152

```
ttgtttcggt ttttcgtcgt tttttgtttt tatttgtagg ggttttattt ttttttagta      60
cgtttttttt gttttttttag gggttatttt ggtggggttt tacggatttt tggttttcgg    120
ttagttagta gtttttttata tcgttgtttc gtttttttgtt attgttatac gttgggattt    180
cggagacggt tttttcgagg tggatgtcgt tgggtcggtt gtttcgtatc gttatgtgtt    240
gtttgggcgt tattacgtga cggtcgtgtt ggttttgggg atcggtttag ttttgttggg    300
gatagacgtg taggtggaag cggtatttgt cgttttggag ttcgtgtgtt cgttttcggt    360
gtagagtgac gagagtttcg attttagtat ttagaatcgc ggtggtttag gtttggaggt    420
cgtttatagt atcgtggttt tgggcgagga gtcggttcga ggtgagtgtt tgttgtttat    480
tttttttttt ttttagggtt atttagatgg ggtagagttt ggtattttcg ttttgggttt    540
atattgatcg ttgatatttt cgtttttatt ggtttttagc ggtgtattcg ttttgttttt    600
cggatacgga gatttttttt ggtaatgggt attgttatcg tttggtggtg gagaaggcgg    660
tttggttgta ggcgtaggag tagtgtcggg tttgggtcgg ggttattttg gtaatggtgg    720
atagtttcgt cgtgtagcgt tttttggttt ttcgggttat taggtgtttg ttttattttt    780
cgaggggtta taggttggga gatttttgaa gtagtggggt agagtttgcg gttggggagt    840
tttaggagga gggaggtggg agttgggtcg gttttggtga gtaggtggcg tcggtcggtg    900
gggtcgtttt tgttagtttt gtagatgtag aggtggacgc gagttggggg tagttttcgg    960
atatttttgg gtacgttata cgggaggtgg tttgtacggg gatttttgtc ggtgtttata   1020
ggtttcgtgg gtgggtgttg ttgtgagttt gggttggtgg gttttgtttt tcgggttttg   1080
agttttagtt tttttatttg gaaaggggga tagtgacggg gtttttagcg ggttgttgtg   1140
agggtgggag gatggaggag tgttttgagt tttttgttat tttatattcg tttttaggag   1200
tttagatatg tggatcggtt tttcgattgt gtagggggtg gaggtgggtt tagcgtcgta   1260
gggcgaggtt tttagtttgg agagttgtta gaattggttg ttcggggagt tatatttagt   1320
tatagtcgag tattgcgttc ggttcgggtt tatcgggtgg tgtaatatcg atttgtgttt   1380
agcgtcgtat agttacgttt gcgagttgcg gtttggaggt gtgcgagggg ttaggtaggg   1440
gtttgagacg ttggttgtgg ttaggggttt gtcgagcgtt cgcggtggag tttgggttga   1500
ggaggagggg ttggtggggg ggttttcggg cggttcggtt tttagtttgt tcgttttggt   1560
gttttgggtt ttggttcggc gttttattgt gtatttgtta ttttaggttt agtgtaggat   1620
gtcgagaatt ttttcgtggg agcgtttagt ggggatttgt agggattttt gatgtttttg   1680
gtacggtagg acggtttttt agtttcgtac gagttcgtgg aggtagtcgg ttttttacgt   1740
tttataattt gttttttttgt ttgtttttgg aggtttcgtt tgttttgttt attgtgggtt   1800
tcgttaaaaa atttgggggt tttaatgttg tttgtgttta gtgaagatgg ttgggaaaat   1860
ttagagtgta gagaggaaag cgtttattta tattattttt aggttttttt tttgagtgtg   1920
ggtgagttat ttttgaaagg taggttaggg gttttgtttt ttatggatag tttttatcgg   1980
agtttttttt tcgagcgata ggagttaggt ttgtgggggt tcgatggttc gttttttttt   2040
tttttttttt ttttgcgaag ttcgggtggg gggagtttgg gttttaggtt gggatggggt   2100
ttgtggagtt gaggcggttt tttgtttatt aggttatggt attttcgggt ttgcgtttga   2160
gtcgtgaagt ttttttttatt acggtcgaat ttgggattta ggagtttcgg cggttcgttt   2220
agttgcggtt gtaggtgtat cggtttttta gtatagtagg tgggattttg ggtggtgggg   2280
cgtcgtagga ttcggggtgg tttttttgag tttttacgtt tgttggtttt gtggttatta   2340
gagtggtttt tagttttagg tggatagagt aggggtttta gagatattag tttattttag   2400
gtgttttggg ggtggatcgg gtggggtttg tttggggatc ggtttgggtt agttagttgg   2460
tcggagatag ggacgtagta ttgggttggg agtgttgttc gggcggggag atttgttttt   2520
atagtaaggt taggttcgtt ggtgtaggta gttgggtatt tttgacggtg gttcgtgggc   2580
gaatgagggt tttaatattt tttttttttc gtagggattt cggagaacgg tagcgagttt   2640
gagagtaggt tttcggataa taggatttag ttggttttcg cgtgtatgtt agggggacgt   2700
tggtgttttg gagttaatat ttgtttgtcg ttggacgttt tttgttattt ttaggtttgc   2760
gttaatggtt gtacgttagg gttagggtta ttcggggttt tttatgcgtt atggagagag   2820
tttttttttt tcgttttcgc ggggtttttc gcgtagtatt cggtgtgtgg ttttgatttg   2880
ggtttgtttt ttgtattttt ttaggttatt tttttgtttg ttgtttaggg tttgggtttg   2940
```

```
cgtagtaggt atatttagtt tgtaggtttt ttttacgttt ttgttatttt tgattttcga     3000
ttttcgattt ttaattttcg atttttgtag tgtttttgtt tttttttag tgggagaagt     3060
tttcgtttgg gtttttggta cgagttgtgt ttttttttt tttttttag tatagtcgtt     3120
tttttttgtt tgttaggttt tggtttgtgt ttttttttcg tgtgtttttt tgtttgtaat     3180
tgttttgttt gtttttgtta cgagtattgt ggggaggttt tttgaggtgt ggttgacgaa     3240
gcggggagtt ttgcgtgttt atttttattc gtcgtgtagg ggtttacggg ttatgatcgt     3300
gaggacgtga tgtagttttg ttttttttt tataggttat tttttatagt taggatgttt     3360
ttatgttttt tggtgatttc gttggtttgt agtacgacgt tggttttggc gttttttttgt     3420
attgttcgtc ggttttcggt tattttggtt tttaggtttc gtattttttc gttaacgttt     3480
cgttatggtt gttttatttg ttagtttagt tggagggtat ttgggtttgt tttgtttgtg     3540
ttttgcggtt gtttgtagtt acggaatagt ttatcgtgtt gttgggtttg aggtttaatt     3600
ttgggttgcg gttgtttggg cgttatgagg ttcgggtaga ggtgggtaat ggcgtgttta     3660
ggtataattt gttttgtagt tttgacgtgg tttttttagt ggttgggttg cgggttattt     3720
atttgttttt tcgcgacggt cgtttttacg tgtttattaa cggtttagtt tcggtgtttt     3780
aggtggattt tggtgttagc gttacggtta cggttcgttg gtttgggggt agtgttagcg     3840
ttcgttttga gaatgtttgt tttgttttgg tggttatttt cgtgttcggt tgtttttggg     3900
agattaatga tattttgttt ttagtggtag tattgtcgtg gttcggtgag ggggagtacg     3960
tgatggacgt tgtggtggaa aatagcgtta gtcgggttaa ttttagtttg cgggtgacgg     4020
cggaggagtt tatttgtggt tttcgcgtta cgtttagttt cgaggttcgt gtattgtagg     4080
gagttttagt ggtgagtatg gtcgaggttt tattattagt ttttaggtag gtgtttgtag     4140
atagggtgtt tatatagggt ttgaggtttg gtttttttagt gagggtagta gtttagttat     4200
tggggacgtc ggtttcgggt aggttttgtt ggttggtttt ttaggttatt tggtgggttt     4260
taaattttttg gaaagttacg gttttgatag cggtttcgtt aatttatttt atcgttttat     4320
tttacgaaat gaatttaaaa tttcgttttt tgattttata cgagttttcg tgagtttttt     4380
acgtttttttg ttgtgttttc gtttggttaa agtaagtggt ttttgaggtg gagttcgaat     4440
ttttgatggg aaattgcggg ttgttcgtag tgttattatg ttgggtatat gggggatagg     4500
gttgttttat tttgcgggta tttgtttttt tattaggggt tttgggaggg gttattagaa     4560
atggcgtgat ttgtgtagtt tgttttgggt tttgtaagtt agtgtaggtg ttgtttttgt     4620
gaggttcgtg tgttttttttt tattgtttcg agtttttttgg ttgaggagtt ggggtaggag     4680
tttcgggagg gtttgagaag atttagagag aggtggattt tttgtagttg gtattaggtt     4740
tgttttatag atggggaaat tgaggtatag agaggttgag gtattagtag tattatatgg     4800
ttggttggag agtcggatag ttagtgtttt agttcgggtt tggtttttat ggtatgtaga     4860
gtttcgggta ttttttttt tttgtgtttc gcgtgggatt ttttagttcg acgggaggtg     4920
tgtttaggag gcgataggtt aagggtagag tttttttatag agtttaggtt gatattagtt     4980
ttttcgtaga ggtatagttt cgtggtggag gtcggttcgg atatggtttt tcggtggatt     5040
attaacgata agtagttttt gatttttttag aacgtggttt ttaatgttat ttattagagc     5100
gcggcggttt ttaagttttt agtaggtggg cgggagtggg gaggggaggg gatggggcgg     5160
ggcgggggcg ggttttattt ttatttttgt tttttgtttt gttttatgtt gttcgaggac     5220
gttgttatgg ttgtggtgag tggagggagg gacgttaagt agggtaggtt ttttatttgt     5280
tatttgggtt tattgatgtt tgtttttgta gttgacggtt tttaattacg tgagtaacgt     5340
tatcgtgaat tataatatta tcgtggagcg gatgaatagg atgtagggtt tgcgggtttt     5400
tatagtgtta gtcgtgttgt tttttaatgt tacgttggta ttgacggcgg gcgtgttggt     5460
ggattcggtc gtggaggtgg ttttttttgtg agtgatttag gggtcggttt ggggtgggta     5520
ttaggttttt gttcgggtat taggtttttg tttcggtttt agttttagtc gagggatttt     5580
atattagggg gttgtttttt tgagtttcgg ttttttttgtt tgttgggagg tattgggtgt     5640
ataggagttt tgaggttgta cgggagtcgg gagaggtttt agtatagtcg ggtgggtttt     5700
gaatggaggt tcggggcgtg attgtagagt ggagtttcgg ttgggtttta agtatttttt     5760
gtttcgttat cgcgtatttg tgtttcgtta ttgcgtattt ttgtttcggt ttatttattg     5820
ttttttatcg tttcggtagg tggatttttg gggatgggga gtaggttttt tattagtttt     5880
agttttcgta taacgagttt ttttcggttt tagatttttc ggtggtttag gtgttggtgg     5940
agtataatgt tatttatatt tacgttgttt taggtgaggg atgagggggt gagggggtta     6000
ttgtttttta ggttttgagt acggggtttt tttagttttt tagttaagtt gtttcgtttt     6060
tttttaata gtttttattg tgattttatt tgggttgatg gtttaggttt ttattggggt     6120
gagggagggg ttaggcgtgg gaggagtgga tagggaagtt gggtttttttg aattgttttt     6180
tatcgcggtt tggtttttgt tgttttgttg tttcgagtgt agttgtattt ggaggcggtg     6240
tcgtttttat taggtagttt ttagtgttgt tgtatttgtg tttcgtttcg tacgtggttt     6300
gggagtttgg gatttttaag gttgggtcgt aggtgtagtc gtttatttcg ggttttttag     6360
gcgggggggtt tttgtcgagc gggtggggag taggtggggg tgtcgcggtt gttttatttg     6420
ggtttgtttt tataggtgag tacgttttga ttttgttggt atttaatgtt ttcgagaatc     6480
ggacgtagta ggtgtttgtg agcgtgtgcg tttttttgtt ttttgtgagc gtgtgcgttt     6540
ttttgattgg ggtttgttgg tattttagag tgggtgtttg tttttttagtt tttgtttttt     6600
```

```
ttagttggtt tgattggggg tttgtttaga ggggtcgttt gaggggaggg tgtgggagta    6660
ggttttattt cggtttagtt ttttgattta ggttttggtt aagggttgta ggagtttgtg    6720
agttaggttt acgtggtaat tgcggttttt atatttatat atacgtttgt ttttatacgt    6780
attttttttag gggtttttag tgagtattgt ttgtttttttg ttagggttta gttggtttta    6840
gtatattaga acgtatattt tagtgttttt tgttttgtgt atgtttttttc gtcgtttagg    6900
ttggaaggtg gtaaatcgga tgagtatttt gggagggggt gagtttatcg gtagtggtta    6960
ggtttttggg aaatttggag tttgggagta gtatttttta cgggtttttt agatttttta    7020
gtaggttaaa tagatttgtg ttggaggtaa ttttattttt acgttaggtg ttgattcgta    7080
gtggtcgggt gtttattgtg tttttggagt gtgtgttttg taaggtatag gtcgtgtacg    7140
aagtgagtcg tagtttttac gtgtatttgg agggtcgttg ttttaattgt agtagcggtt    7200
ttaagcgagg ggtgagtgtt gagcggggtg tgggcgggtt ggggatgggt tttatggtcg    7260
aggggacggg gtttgtaggt agaagtgggg ttgatagggt agagggttgc gtttttttat    7320
tatttttttt gtttgtagcg gtgggttgta cgtacgttta gtaataagac gttggtgttg    7380
gatgagatta ttatatttac gggtagcgta ggtatgtgat tggtgttgcg gcggggcgtg    7440
ttgcgggacg gcgagggata tatttttacg ttgacggtgt tgggtcgttt tggtaaggag    7500
gagggttgcg tttttatttt tttgtttttt aatcgttcgt cgttgggggg tttttgtcgt    7560
ttttttttat tgggcgttgt gtacgttttt attattaagg tgtatttcga atgtatgggt    7620
gagtgtaggt ttgcgtaggg ggagtagcgg gatttttcga ttttgtgagg ttacggagtt    7680
ttttcgtgat gtcgtgggga tcgtttttta ggttggtatg acgcggagga tgttggcgtt    7740
tcgttggtgt acgttttgtt gttgtagcgt tgtcgttagg gttattgcga ggagttttgt    7800
gtttataagg gtagtttttt cggttacgga gtcgtgttgt tttcgggttt taggttatat    7860
ttcgaggtgg gtttggtcgt ggtggtgtag gattagttgg gagtcgttgt ggtcgttttt    7920
aataggtgag ttaggtcgtg ggagggcgtt ttcgagattg ttatttattt attatttttt    7980
tttgttcgta ggtttttggt tattattttt ttagagttta acggtagcgt aatggggttt    8040
atagtttggt tgtacgggtt tatcgttagt gtgttttcgg ggttgttgcg gtaggtcgat    8100
ttttagtacg ttatcgagta ttcgttggtt ttggttattg tgttgaacga ggtgagtgta    8160
gtttgggagg ggattttata tttgttgtat gcgtgttggg gattaagatt tgtttttttg    8220
tttggagttt tgcggagggt ttatttcggg ttttagagat aaattttagt gattttgaag    8280
tagtatttcg acgtttcgtt tttagtagtt atatttatcg ggtttttttc ggcgtttgtt    8340
ttttataatg tagttttcgt ttaggagggt ttatgtgttt attttgtttt gtttatgaag    8400
aaatagttta gtgttgcggg ttagtgttta tattatatag tatttagtac gtaattgtat    8460
ttcgggagtc gtgggtattt gttggttttt tgtcggtttt ttgttttgtt gatagtttgt    8520
tgtgtttttt gtttgtttta gtacgagcgg gttttggacg tggcggtaga gtttaagtac    8580
gagcggtagc gtcgagttta gatacgtaag aatattacgg agattttggt gtttttgagg    8640
gtttatattg tggatgatat ttagtagatc gttgttgcgt tggtttagtg tatggtagga    8700
tggttttata tgtttttttc gtttcgtatg tttgttaggg tattgggttt agttttttag    8760
ggtagacggg tagtttggtc gaggagttga gtttttagtt tgggtttttt tttgttatgg    8820
cgtttttcgg tttttgattt gttttagtag ttttagttat tttgttgttt tgtgtgaacg    8880
tagggtgttt ttcgggggat ttagggtgta aagaggggtt tagatgtggg gagggattaa    8940
gaagatgttg ttttgtgttt tttatttttt tttttttttt tttttttttt ttttagtttt    9000
tttttttttt tttttttttt taattttttt tttttttttt ttttttttta gttttttttt    9060
ttttttttag ttttgttttt ttttttagt tttttttttt ttttagtttt tttttttttt    9120
ttttttttta gatttttttt ttattttatt tcgttgagtt tttttatttt ttttttagtt    9180
tttttatttt tttatttttt tttttttttt tttttttttt tttttttttt tttttttttt    9240
tttttttttt tttttttttt tttttttttt tttttttttt tttttttttt tttttttttt    9300
ttttttttat tttttttttt tttttttttt ttgttttttt ttttttttta tgtgaagagg    9360
tgttttgtgt ggtcggtggg ttgtattacg tgttttttag gtggaggttt tgggttatgt    9420
agagttataa aaaatgttta gtgaggaggt tgtgggggtt tagttaagtg ggttttttag    9480
ttgtagggtt gggggtggga gttaggtgag gattcgtgta gagaggaggg cgtgtgtaag    9540
gagtggggtt aggagcgggg ttggatattg ttggttttat atagggggttt agtagggagt    9600
tcgtatgtcg ttcgtgtttg aagtagacgt tgtataagtt ggaggttatg atgcgtattt    9660
tgtaggtaga gattatcgcg ggtatcgtga cgtttatcgt tatcggagat agtattttta    9720
atattatagg tgtcgcggtt cgtgttttac gttattcgtt cgttttacgt ggttcgttcg    9780
ttttatgtcg tttttttttt tgtttttttt tttttataa tcgtttcgtt tttgttttat    9840
tttattttcg tttttttttt ttttttttaa tttttatttt tatttttttt ttttaatttt    9900
tatttttatt tcgttttttt aatttttatt tttatttcgt ttttttaatt tttattttta    9960
tttttttttt ttaattttta tttttatttt ttttttttaa tttttatttt tttttttttt   10020
tttttttttt attattattt ttttttttta tttttttttt ttttttttta tttttttttc   10080
gattttttttc gtttttttgt ttattttttt tatttttttt attttttttta tttttttttt   10140
ttttttttat tttttttttt tttttttatt tttttttatt tttttttttt tttttttatt   10200
tgtttttttt tttttttttt tttttttttt tttattttttt tttttttttt tttttttttt   10260
```

```
tttttattat ttttttttatt ttcgttttta tttttatttt tttttttttt ttttttttatt 10320
atttttttttt tagttttttt ttttttttttg tttgtatttc gtttttttgtt tttttaggtt 10380
tttttttttt tttagttttt atttttcggt tttttttttt tgtttgtttt tattttttttt 10440
ttgtttttttt gtttttgtat tgattttacg tttgtttgta ggagatttta tttatttggt 10500

tagtttagac gtgcgggtat cgtagcgttt agagttggga gtcgagttat tattgcggat 10560
ggtggcgttt taggtttata atttgatttt tgtttttatg cgtattttta cgcgttttcg 10620
cgtgtttaac gaggagttcg tgacgttggc gggcgaggag attatggttt agggtaagcg 10680
ttcggattcg cggagtttgt tgtgttatgg cggcgtttta gggtttggtt gttatttttt 10740
tattttttag gtttttagta gggttttcgt taattttagt gacgtggtgt agttcgtttt 10800
tttggtggat tttaattttt tttttttttgg ttatattagt aattatatcg tttttattaa 10860
ggtggtttcg atggcgtttt agatataggt cggcgtttag atttttatcg agcggttggt 10920
tttagagcgc gttattatcg tgaaggtgtt taataattcg gattgggttg ttcggggtta 10980
tcgtagtttc gttaatttcg ttgtggttta gttttaggtt ttcgtcggtg ttgtggttat 11040
tttggatagt agtagttttg tggtcgtgtt gtatttgtag tttaattata cgttgttgga 11100
cggtgtgtgt agcgggtggg gtatacgcgg tttttttggtt ttgtttttgg ggggaaggcg 11160
tttttcgtag ggtttttatg ggtgtttttg gtgaaatttg ttgttttatg ggttgttggg 11220
ggtttggtcg gagaggagtt gggggttacg gagaagtagg tgttagtttt ggtgtagagg 11280
tttttatggt tttttaggtt cgtggtagag ggtgggttta ggagggttat cgtgggtgtt 11340
tttcgggtgg ttgagttttt cggtaggcgt gtgatttgcg cgttttgttt taggtcgtta 11400
tttgtttgag gaattcgagt tttatttggt agtttatttg tattcggagt ttcggtttaa 11460
tgagcgtaat tgttcggtta gtaggaggat tcgtttagag ttttttttagg gtgtcgatta 11520
tcggttttat attttttttta ttttttcggg gtgagttttg cgggtcggtt tggtagggta 11580
gggtagggta ttatgggtta gtattgttcg ggttacgggt ttcgtgggga cggtaggtag 11640
cgaggggatt ggatcgggta tgggttttgg gatttcgata tttaatttgg cggagtttgg 11700
gtttacgttt attgtttttt ttttttttag gattagagat ttagtgggga gttatcgttt 11760
gaatttttttt agttattttc gttggtcggc gttggaggtg ttcgtgggtt tgtatacgtt 11820
tttgtgttag tattttagcg aggaggatgt ggtgtggcgg atagaggggt tgttgttttt 11880
ggaggagatt tcgtttcgtt aggtcgtttg ttttattcgt tattttatcg ttttcggcgt 11940
tagttttttt atgtttttaa gttatgtacg ttttgtgttt tttgtgagtg attttgtgtt 12000
tttgggagtt tttgtagagt cgaggagggt ttgggtgggt tcggttttat tttgagaagg 12060
tatagtttgt acgtgatttt ttgggttcgg cggttgtgtt tttataggag tcgatagcgg 12120
atgtaaatta tatcgttatg ttgatatgtg ttgtgtgttt ggtgatttat atggttatgg 12180
tcgttatttt gtataagttg gattagttgg atgttagtcg gggttgcgtt atttttttttt 12240
gtgggtagcg gggtcgtttt aagtacgaga ttttcgttaa gataggttgg ggtcggggtt 12300
taggtgaggg gcgcggcggg gtggtagggt ttttttttgtt tttattggtt gtgttggttg 12360
tattttttgg gagtgagttt cgtcgtaggc gttagaataa ggtagttttt gtagtgttgt 12420
gtgaagggtt cgtgtgttta ttttgggaat gatttcgtga gtatttattg tttttgagga 12480
ttaggatagt ttttagttgg aagtaggtgt tagttagtta gggtgggtag tttacgtttt 12540
gtatagtagc gtggttttat aagtgatatg agtatcgtta ttattgtggg agattatgta 12600
tttattcgcg attttgattg tatagttcgt tttttagacg gaggtgttag tatttttttc 12660
gtggttgttt ttttaatatt tttattttttt tttattggaa ttgttttttt ggtatttttt 12720
ttttgttttc gttttttttt ttttggagac ggagttttat tttgttgttt aggttggagt 12780
gtaatggcgt gattttggtt tatagtaatt tttagttttt gggtttaagc gattttttttt 12840
aagcgatttt tttgagtagt tgggagtata ggtgtacgtt attatattta gttaattttt 12900
tattatgtta gttaggcgaa tttttgattt taggtgattc gtttgtttcg gtttgttaga 12960
gtgttgggat gataggtgtg agttattata tttggttgtg tttttatttt ttattttcgt 13020
gttgttttta tttttatttt ttagtttttt tgattattta tttttaaaaa ttgtcggtcg 13080
ggcgtggtgg tttatatttg taattcgagt attttgggag gtcgaggtag gtaaattacg 13140
gggttaggag atcgagatta ttttggttaa cggtgaaatt ttgtttttat taaaaaatat 13200
aaaaaaatta gttcggcgtg gtggtaggta tttgtagttt tagtttttcg ggagattgag 13260
gtaggagaat ggcgtgagtt cgggaggcgg agtttgtagt gagttgagat tgcgttattg 13320
tattttagtt tgggtgatat agtaagattt tattttaaaa aaaaaaagaa aaaaaatatt 13380
gttatttggg tttgttattg ggagaggagg tgatatagtt ttacgtttcg tagtttgtgt 13440
atgaattgag ggacgggtgt gtggtgcggg ttattggttg tggcgtgatt gaggcgtgga 13500
taggtgtgta gcgcgggtta ttggttgtgg tgtggattga ggcgtgtgta gttatgtttg 13560
tatgttataa gttatagttt ttttcgtgta atttaattat gtttttgagg ttttgttgtt 13620
tattggataa attgtagtaa tcgtagtttt tcgtgtatgg tagagtcgtg taaagtcggg 13680
attgtttgtg tggtttttttg agtgcgcgga ggttaaagtt gagatgattt gtttgggatg 13740
ttatacgtgt tgggtagtag atcgagtttt ttattttttt tttttgtttt ttaggtatta 13800
cggtttacgt gggtattatg ttgtatgggg tggatagtcg gagcggttat cggtatttgg 13860
```

acggcgatag agttttttat cgtaatagtt tggatatttt tcggatcgtt atttcgtata 13920
gtttgggtag cgtgtggaag attcgagtgt ggtacgataa taaaggtttg tgcggatttt 13980
gttaagtttt gtttttttgt tttcgtattg gggcgttttg cgagtttgat ttttttttcg 14040
cgtttttgta gggtttagtt ttgtttggtt tttgtagtat attatcgtta gggatttgta 14100
gacggtacgt agtatttttt ttttggttaa tgattggttt tcggtggaga cggaggttaa 14160
cgggggtttg gtggagaagg aggtgttggt tgcgagtaag gtttcgtttt atgtttttat 14220
ttcgtgggag gttgggtagg gtggttttgt ttcgtggttt tttgtagtgc ggtttttttt 14280
gtttttttagg ttacgtagtt ttgttgcgtt ttcggcgttt gttggtggtt gagttgtagc 14340
gtggtttttt tgataagtat atttggtttt ttatatggga tcggttattt cgtagttgtt 14400
ttattcgtat ttagagggtt atttgttgcg ttttttttat ttgttttttt ttgggcgtta 14460
acgtcgtgtg gtacggggtt gttggtgatt ttgtttatag gtgggtgtcg taggggtcgg 14520
gatagttttt ttttgtttag ttttttttgt tttttagttt tatttgtgtg gttttttttt 14580
ttttatatag tacggggtat gtgtttaggt tgagttcgtt gagcgtcgat atagtcgttg 14640
ttggtttggt gtttagcgtg gttgtttatt tcgtttattt ggttattttt tttttttttc 14700
ggatgtttcg gagtaaggtg ggttggggtt ggggattcgg gagtattggg aatggagttt 14760
gggtttcggt attatgttta gggtcgttat tttttagtgt tgtagttaga gggaaaggcg 14820
tttattaaag gttgttcggg aagggttaat atatttgagt agttttagtt agattgatta 14880
gggagaaaga gagaagattt agaagttaga atcgtgaaag aacgagggta tttcgttaag 14940
tagacgttac ggataattgt atagtagtac gttagataat ttagaagaag taagtacgcg 15000
gttgtgtacg ttttcgaaat gtattttaga agaaaatttt agtatattta tagtaagtga 15060
agaggtcgag ttagtttttt agaaattttt tagtggtcgg gtcgggtgtg gtggtttacg 15120
tttgtaattt taatatttta ggaggtcgag gtgggcggat ttgagtttag gagtttgaga 15180
ttagtttggg taatatagta agattttatt tatataaaat attaaaaagg gttaggtacg 15240
gtggtttacg tttgtaattt taatattttg ggaggtcgag gcgggtagat tagttgaggt 15300
taggagttcg agattagttt ggttaatata atgaaatttt gtttttatta taaatataaa 15360
aatttagttg ggtatggtgg cgggcgtttg tagttttagt tattcgagag gttgaggtag 15420
gagaatggta tgaatttagg aggcggagtt tgtagtgagt cgagattgcg ttattgtatt 15480
ttattttggg taatggagta agattttatt tttaaaaaaa agaaaaaaaa aattttataa 15540
agaaaagttt gggtttagag tttttacgat agaatttttt taagtagtta aggaagaatt 15600
aatattaatt ttttatagat tttttttaag aatatagtag gtgggaattt tttttattta 15660
tacggaaacg ggaggtcgta ttttttagga atgtatacgt ggggttttta agaggttata 15720
tgtaaattaa ttttagtagt atatagagaa ggcgtataag tcgcgattag gaggggttgt 15780
tttcgagttc gtggtaggaa ttagaggtta tatgtggttg ttcgtattaa agttaattaa 15840
aatggaacga tggtcgggcg tggtggttta tatttgtaat tttagtattt tgggaggcgg 15900
aggcgggtag attatttgag gttaggagtt ttaagattag tttggttaat atagtgaaat 15960
ttcgttttta ttaaaaatat aaaaaattag ttgggtatcg tggtaggtat ttgtaatttt 16020
agttatttag gaggttgagt taggataatc gtttgaacgc gggaggtgga ggttgtagtg 16080
agttgagatt gcgttagtgt atttttgttt gggtgataga gcgagatttt attttaaaaa 16140
aaaaaaaaaa atgaaattta aaattttgtt ttttagttgt attagtttgt tgttaagtgt 16200
ttagtggtat atgtcgcgag gggttgttat tacggacggt gtagatgttt tatatattta 16260
gtattttagg atattttgtt agatggtatc gggttttgtt ttgtttgttg aggaggtggt 16320
tttttatttt tgttttgagt aggtttgagt tgtcgttcgt tgattattgt cgtcgttttg 16380
taggtggttg ggagtttgag ttttatattt gtcgggtagt aggtgttgga tatcgatagt 16440
tgtttggatt cgttcgtgtt ggatagtttt ttttttacgt ttttaggttt ttacgttgag 16500
gtgagggttt tattgggggt tttgggttgg gttgggggtt ttgtcgtttt ggcgtagttt 16560
ggattttaga tattgtgtat tttttagtag gtttttgttg gataggtgaa gagtgatttg 16620
tttttggatg attttaagag gtgggttttt tagagaaatt tcgagttttg gtgtaggtta 16680
ttgtgtttgg agtatcgggg gtgtgcgggt tgcgtgtttt tgttgggtgt ttgtggtttt 16740
atgtggttat attacgtggg agtaggtttg ttcggaagtt tagggtgttc gtgcgtgatt 16800
ggacgggggt gggttgtgtg tgtgatatat tttttggtat tttgttgatt cgcgttattt 16860
gtagtttggt gtgttggttt tttagcgagg gaacgtttag ttggtcggat ttgtttagtg 16920
attcgtttat tgtgggtagt aatttgcggt ataattttta tttattgggt tttttttttt 16980
ataattaata taatcgaaat ttagggtttt tttttttttt tttttttttt tgagatagag 17040
ttttatttta ttttgttatt taggttggag tgtaatggta cgatttcggt ttattgtaat 17100
tttcgttttt cgggtttaag ggattgtttt gttttagttt tttgagtagt tgggattata 17160
ggcgtgtgtt attatgtttg gttaattttt gtatttttgg tagagacggg gttttagtat 17220
gttggtgagg ttggtttcga atttttaatt tcgtgattcg tttgt 17265

<210> 153
<211> 2204
<212> DNA

<213> Artificial Sequence

<220>
<223> chemically treated genomic DNA (Homo sapiens)

<400> 153

```
agggataaat ttatttaaat tttttttaa ttcgttattt tcgggatttt aattagaatt      60
gggttgtttt tttgcggttt tttcgacgtt ttatttttaa ttagtttata gtttttgggt     120
tttgtttttt tttttattgt atttttaaaa tgtaaggcgt aacgataata gtaacgtttt     180
agttattttt gagatttttt aaattttgga tttgtttttt tttttttttt taaagttttt     240
cgaatttttt ttagcggata aatgaaggga ttgaagttgg ataaaattat aatttaggag     300
attagggatt tattttattt tgtttttcgt gtttggaatt agataattta ggacgtcggg     360
ttcgagaagt ttggataaaa aagttaggaa aaattgagtt tcgttttgat ttttagcgtc     420
gtggcgggtt ggtatttgag ttggtaacgt gcgtggtagg ttatgtaaga aaagggatgg     480
aggagtcgtt ttagcgggag ttagaaagga cgcggtgttt tcggttgtaa tttttatttt     540
ttttatttag tagggtagga ggtatttaat ttggaggaga aaggggtggg ggaggtgaaa     600
tagagatcgg agagttacga gggttgggtc gtcgagagta ggagaatata tcgtgttata     660
tatttttatt tttttatata cgttgtagat ataaattatt gacggttttt acgtgttgcg     720
ttcgtgagcg gaggtgttta aagagggggt agatgagtta tttttcgaga cggaatcggg     780
ggttttacgt tcgtcgtttt tagtagtata attaattttt gaatatttaa atcgcgtatt     840
tttggcgtat tattatttta tttaaggtta cgggtttcgt tttttttttt tttttttttt     900
tttttttattt tttttttatt tttttttttt ttttattatc gggttttttt tagtagtcgt     960
atgtagggga ggagtacgga atgtttttttt tatagttaat tattatttgg ttgttttttt    1020
attggttgag tttttttgta ttgtagtaaa tgggttatgg attttagtaa ttagagttac    1080
gtgttatcgg cgatataatt aatgagggcg cgagatttgt gtaagtaggg ggaggtacgt    1140
gtcgggtcgt acgtgtttgg gagggggggga aggggcgggg tttattgaga gaggcggagg    1200
tgggtagata gattcggaga gacggcgaag gagttggaaa tcgagttagg gttgagtcgg    1260
ttgataatag gtaaggagat tcggaggata aagcgagttg ttagggagta tttgggagtg    1320
gatttggggg ttgaatgtag attttggtat tgggagggtt tgtattacgt ggtaggtgcg    1380
agtttagaga gatcggcgta gggattttga ttttgaaaga ttgggagagg gtaggaggtt    1440
agtttttatg ggggcggttt tcgtaggatt attaggtggg ttcgcgtgtt tagattgatt    1500
gtttgtgggt ggtatggttt taggtggaga ggtttgttgc ggatttaggt tggcgaatag    1560
ttttggcgaa taacgggcgg aggggaaggt gtttttttaag tcggtattta ttttgaattg    1620
agggaagaaa agaacggagg tcgcgttaga tcgagagttg ttttgcggcg gttagagagg    1680
gaattttaag tttttaatgg gcgggggtgg gggtagaaat gtttttttttt attgtgtttg    1740
agatcggtaa tattggggag ggggagaata agtattattt tacgtagtat attttaattt    1800
tttagtttat tttttaattt ttaaattttg gttttaaatt ttttcgtttt gtttttttagt    1860
tgtaggtttc gatttttggg tattttagga gttgttttat agtttttgtt tttggattta    1920
tggatttttt atttagcgtt ttttttttatt tttttttattt ttttttttcg ttttttttta    1980
tttttttagt gaatagtgat tttggttttt tttttgatag tgagagggag gataaggggg    2040
tttatgggtt taggttagat attgttgggt agagggggagg tttacggttt agttcgggtt    2100
ttattcgttg taggtatcga tcgaaggttt tcggtaatta gtatatatta ttttattcga    2160
aatagcgggg ttcggttttt tttatggtag gatttggggc gaaa                     2204
```

<210> 154
<211> 2204
<212> DNA
<213> Artificial Sequence

<220>
<223> chemically treated genomic DNA (Homo sapiens)

<400> 154

```
tttcgtttta gattttgtta taggagaagt cgaatttcgt tgtttcggat gagatggtgt      60
atgttggtta tcggaaattt tcgatcgatg tttgtagcgg ataggattcg ggttgggtcg     120
tgaatttttt ttttgtttaa tagtgtttgg tttgggttta tgggtttttt tgttttttttt    180
tttattatta gaggggaagt taaagttatt gtttattggg gaggtgggaa aagacgaaga     240
gagagagtag gaggaatagg aagaaacgtt agatggagaa tttataggtt tagaagtagg     300
```

ggttatagaa tagtttttgg agtatttaaa gatcggaatt tgtaattgaa gagtaaagcg 360
agggagtttg aaattagagt ttaaggatta ggaaatgaat tgggagattg gggtgtattg 420
cgtgggataa tatttgtttt tttttttttt aatattatcg gttttaaata taatagaaaa 480
agatattttt atttttattt tcgtttatta agagtttggg attttttttt tggtcgtcgt 540
agagtagttt tcggtttgac gcggttttcg tttttttttt tttttagttt agagtaaata 600
tcgatttgaa agatattttt tttttcgttc gttgttcgtt agggttattc gttagtttga 660
gttcgtagta agttttttta tttgaggtta tgttatttat aagtagttaa tttgggtacg 720
cgaatttatt tggtgatttt acgggaatcg tttttataga gattggtttt ttgttttttt 780
ttaattttt aagattagga tttttgcgtc ggttttttg ggttcgtatt tgttacgtga 840
tgtaaatttt tttaatatta agatttgtat ttagttttta aatttatttt taaatatttt 900
ttaatagttc gttttgtttt tcgaattttt ttatttgttg ttagtcggtt tagttttggt 960
tcggttttta gtttttcgt cgtttttcg ggtttattta tttattttcg tttttttag 1020
tgggtttcgt tttttttttt ttttttagat acgtgcggtt cggtacgtgt tttttttgt 1080
ttatatagat ttcgcgtttt tattggttgt gtcgtcggta atacgtgatt ttagttattg 1140
aagtttatag tttatttgtt atagtataaa ggggtttagt tagtagagag gtagttaggt 1200
gatgattggt tgtaagggaa gtatttcgtg tttttttttt atatgcggtt gttggagagg 1260
attcggtgat gagaaaagga ggaggatgga aaaagagtgg aaaagaaggg aggggaggaa 1320
aagggcggag ttcgtggttt taaataggat ggtgatgcgt tagagatgcg cggtttgagt 1380
gtttagagat tggttgtgtt attgaaggcg gcggacgtgg gattttcggt ttcgtttcgg 1440
gaagtaattt atttgttttt tttttgaata ttttcgttta cgagcgtagt acgtggaaat 1500
cgttagtgat ttgtgtttgt aacgtgtgtg agagaatgga ggtgtgtgat acggtatatt 1560
tttttgtttt cggcggttta gttttcgtga tttttcggtt tttgttttat ttttttttatt 1620
tttttttttt ttaagttggg tgtttttgt tttgttgggt gaggagggtg gggattgtaa 1680
tcgagaatat cgcgtttttt ttggttttcg ttggggcggt tttttttattt ttttttttat 1740
ataatttatt acgtacgtta ttagtttaga tgttagttcg ttacgacgtt agaggttaaa 1800
acgaaattta gtttttttg atttttttat ttaggttttt cgggttcggc gttttaaatt 1860
atttgatttt aagtacgggg aatagaatgg ggtaggtttt tgatttttta ggttatggtt 1920
ttgtttagtt ttagtttttt tatttgttcg ttggagaaga ttcgaaagat tttgaaggag 1980
gggagggagg taagtttaga gtttagagag ttttagaagt aattgggacg ttattattgt 2040
cgttgcgttt tgtattttga gagtataatg gagaggagag tagggtttaa ggattgtgag 2100
ttggttgaag ataggacgtc ggagagatcg taagggggta gtttaatttt aattagggtt 2160
tcgaggatag cgaattaaaa aaaaatttaa gtgagtttgt tttt 2204

<210> 155
<211> 13135
<212> DNA
<213> Artificial Sequence

<220>
<223> chemically treated genomic DNA (Homo sapiens)

<400> 155

ttcgtgtttg ttttcgattt tattttttaa attagacgtt tagtttggtg taaatgtagg 60
agtggagttt ttagggggat gtggattttt tttttttatt tttatttcgg tttttgtttt 120
ttttttttcg tttttatttc ggtttttgtt tttttttttt cgtttttatt tcggtttttg 180
tttttttttt ttcgttttta tttcggtttt tgtttttttt ttttcgtttt tatttcggtt 240
tttgtttttt ttttttcgtt tttatttcgg tttttgtttt ttttttttcg tttttatttc 300
ggtttttgtt tttttttttt cgtttttatt tcggtttttg tttttttttt ttcgttttta 360
tttcggtttt tgtttttttt ttttcgtttt tatttcggtt tttgtttttt ttttttcgtt 420
tttatttcgg tttttgtttt tttttttttg tttttatttc ggtttttgtt tttttttttta 480
taggaggagt ttgggggttt tcgcgttata ggggcgtggt ttagggtatt aggtggggcg 540
gtgggtattg ggtttagagt ggttttgggt tgtttagttt tttttttttt ttttaggatt 600
agtgattttt ttagttttag gatatttttt gggttagggt ttagggtaga gttaatgttt 660
ttttagatat tttttggttg taaattttag gttgggggtt tttggggata gggatgtagg 720
tgtttaagga tacgattttt taggtagtgg acgtttttgt ttgggtggag ggtacggtta 780
cgagagtagg gttcgcgttt tggtattttg gtgttcgggt tttttgtttt taaggtttgt 840
tttgttttag tttagttttt tttggttagg tttattattg ttagtaaata tttttatatt 900
gttgtttttt tagatggtta tggtagtttt ttcggggttc gtgtttgtag tttttacgta 960
gttcggtcgg tcgatggaaa cgtatattta tattttgttt tattttattt cgagttgggg 1020
gttcggggat tataggtttt atttgtatag gttgtatttg ggaatgggt ttgtggtaga 1080

```
aaatgtgcgg cgcggggcgg ggggttttaa ttaggtagtt ttaatttgga ttttttttta    1140
ttttcgtttt attttttttgt ttttagtata cggtggcgga gtgcgtgggg ttagtttggc    1200
gggggttttg tttttcgatt tgtatttaga gaatagagta ttttacggga gagagtagtt    1260
cggaacgtag tttgagtaat gtcgatttta tattagtata tttagtgttt ttacgttttc    1320
gttggtttag gtttcggaga ttatgatggt atttatagtg gatttcgtaa aggagcgtgg    1380
ggatttcgaa ggttaggtta ttttttagag gggggtttta tgttaaagta gacggtgtcg    1440
gtgtcgtagg gcgtttgaga tttacggtgg agttcgggtt tggcgtgcgg gaggcggtta    1500
cgacggcgtt ttttttttag gaatttcggg agggatttta ggatttagcg ttagggtagt    1560
tttggtaggt gtagtgaggt agtgatttgt gggggttaga tgtgggtttg ttttacgtgg    1620
gtagggatta gttaggtatg gggttttagc ggattcgagc ggggttatag agttttggag    1680
gatttttttg tataatgaat ttttgtttaa aaatagagat taagaggaag ttttattttg    1740
tagtttcgga aatgggtggt tgcgtgtttg gggttttcgt tacgcgggag gatagtttag    1800
aggtattttg gttttaggta gttggttttt aggttgtgtt ttttttattg gtcgggtatc    1860
ggttggaacg gtacgaggat tagttttatt gtttttagag gggtttcggg ttattgggtg    1920
tggtggtgtt ggaggagttt tgttcgttgt gggttttgga gggtacggta gtagagtttt    1980
tggttttttt aggtagagcg gtatttgttt tatagatcgt gttagttttg gttgtaggcg    2040
ttggggtttt atcgtgtttt ttgggggggt tcggattttt taaggtttcg atttgaagtt    2100
ttgaggtttt atggatgttt gtttttggtt cgataaacga gatggttgtg gtggggagga    2160
gttttaggcg gttcgggatt ttttggcgtt cgtcgttggg aggcggaggg atttagagtc    2220
gaggttgtgt ttaggattgt ttcgggcgtc gggcgatgtt aatattttgt gttggtttc    2280
gttaaggggt cggttttcgg gtttttgtcg ttaagtgcgt ggcgtggttt cggtttttag    2340
gttagatatt gttgatacgg gtttttttcg ggaaggtagc gggattcgtg gttttgttta    2400
ggggtcgttt tcgtttgacg tagcgcggtt ttcgtattga aacgggttag agataggcga    2460
ggttagagtg ggatttggat agtagggata gtgtaggttt tagtttttat cggaggtttt    2520
cgggagtggg tatatggtat aggcgttcgg ggttgtttat tttgattagg tgggaattta    2580
gatgtttttt gtttttaagg cgtttttaga gttttagttc gggaaaggga cggttttttt    2640
ttgttcggtt gtgttttttt atttatattt tgggtttcgg ggtttggata agggttaggc    2700
gattttcgaa gatagagatg tgggttttag agtattatgt attttagggg gcgataagta    2760
ggtcgtagtt ttaagatttc gaatgtgatt ttatttagaa gaaggggttt tgtagggggt    2820
tacgttaggg ttttaggtgt gatcgtttag ggtttttcgg gtaggttttg aaataacggt    2880
tagggtttat ttgagagaga ggtagagaga gaagacgtag aggtggggga gacgtcgtgg    2940
gattagggga tagagacggg taacgtagtt atagatgttt gagtcggtag ggttgggaa    3000
ggtagatagc gttttttcg gagtttcggg aagcgtgtgg ttttgtttgt tttttggttt    3060
tggattttg aattttagga tagttagatt aaaggtttgt tgttgaagtt ggtagtttcg    3120
gggtattcgg gtatcgtggg gtttgttggg tatttgtaga tagggttgtt ttagggacgg    3180
ggtacgttta gttttgatgg agaacggttt gttttttaaa ggtacggtta tttgttttta    3240
tgatagattt ttgtataggg ggacggaggt cgggtgagtt ttgtttatgg ttttggagtt    3300
gtgtggttgt gcgtcggtta tttagtttgt gtttgtggtt tcgtatataa aatgggtttt    3360
aatcgggttg atttttgggt tcgtttgatt tagtattatt gaagcgatgg ttgtttagga    3420
gggtttggcg gaggtcggtt gcgtttgttt tcgggtggag ttggatttta aggaggagta    3480
ggtattgggg gtttgttttg tcgtggtggg tttttttttt ttgtagattt tacgtttcgg    3540
gaataggaaa gagtatggaa gttacgggga ttaaaggggg gcgggtaggg ggcgtcggtt    3600
aaatttagtt gagatttaga gtagggggta ttagttaaat tcggttttgg ttttgttttg    3660
tgggtgggtt ttttggggat tcgggtaggg gtggtcgaga tttagagtta ggtaaattcg    3720
tttggggata aatttataga gtattttggg attggtttag agatgggaaa ttttataggg    3780
ggatgcgata gttgttgtta tttgaggatt gtggcggggg gattattgta gttttgaaaa    3840
ggagagaaag gtagagtgag gattcgggat ggtttggtat aggtatacgc ggttagggac    3900
gtttagttcg gatgatgtgg gttatttacg gtttcgttag gttgtagggt cggagttgtg    3960
ggggagaaag cggagagtta ttttggagtt taggagttgt tttaatttta gtttagtgta    4020
gcgttcggga tagtagagcg aggagttgtt ttagttttag tttagtgcgg cgttttcgga    4080
gtagtagagg tttcggggtt gtagggtttt ttttaatttt tttttgcggt gtattagttt    4140
ggttttcggt tttgttatat tttttcgggg tatttatagg tgcgggggtt tttttagtat    4200
tttaggtgag ttttcgtttt ttggatttat tttttcgttt tagtagaggg gataagtttt    4260
ttttatgaga agcgtttttt taggttttag ggttttaggg gaagggttgt ttttttattt    4320
tttcgggagg agttttaggt acgaggtggt tttgagtttt acggtcgtgg gtttttgttt    4380
ttatggtgtt ttcgcgatgg gggtgtcgtg gtatcgtggt taatttggtt tttcgtttcg    4440
tggttattag ggttgattaa gttttgagtt ttgttttgtt ttaagggtgt ttataggtag    4500
agatgttgag taggtcgggt ttttcggtgt ttggttgtgt aggagatttt ggtagcgggt    4560
tttcgattgg aggttgggtt ttgagatttc gagagaggtt gagggggatg gataggggtt    4620

ttatagtagt taggtggggt tgtttagaga tttgggagga ggttttagtt cggttattat    4680
```

agtttggtta agtgtttatt ttggggttcg aattgttggt tggattattt gtttggtagg 4740
gattttgcgg gggtcggttt tggataacga gggtaggtat tacggatttt gtttagaatt 4800
cgttttcgtt tgaaggtatt atagttataa ggtcgttttt ttagtgggaa ggggttagtg 4860
tttaggttcg gggtgatcgt gtgtacggcg tggcgtaggt tggggtgtgg gttttgtatt 4920
agtttggtta gttttgattt taggagaggg gagggggggat tatggttgtt ggaggagttt 4980
aggtaagttt aattgtcgag agttatttag tttcgtttta ttcgtttttc gtgatgtttt 5040
gagggtttcg ggagacgttt tttgtcggaa gagggtttta gtagaggttt tgggtttcga 5100
ggaaggtttt ttggtgtttc gattttttaa atttatatac ggttgttttc gggaagtgac 5160
gggttttttt atatcgggat ttaaaagtta taggataggt tttgtttttt ttcggttttt 5220
gttagtttgt tttggtttgg gatattttag ttttaggttt atttttacgg ttggatgtcg 5280
tttgttttgt gttttttcgt ggggtaggtg ttggttcggg agatattttt gttttttttg 5340
tagtaggttt tggggagttt attggagcgt ttgaggtaga agaatttagt tacggagagg 5400
atgaggtcgg agttaatgaa gaacgtgttt aggaagaggg aggcggttac gcgcggagtt 5460
tttgtaaata gatatcgttg agtagacggg taggacggtg gttttggttt ttcgtttggt 5520
tttcggtgtt ttttgtatat ggtagatgcg ttgtttttttg aatatgtttg agttttacgg 5580
gatcggttgt gggtgtcgag ggttttgtat atgggtttgg gttattttttg taggtttagt 5640
tatgtttggg tatttggtcg cggggtttgg gaaagttatt atttgtttcg gttttagttt 5700
tttttaggag ttcggatgtt gggagcgtta taggggttga ttttggtcgg gttttgcgga 5760
aatatttttt cgattttagt tgtttttgaa agttaggggg tttttagttt tgtttttttt 5820
ttcgggttta gtataggttt tggggttttt atatagggta gagttttaag ttggtagtag 5880
gtgtttttgt atttattta ttttatttg tttgggttag gataggttag ggttgttggt 5940
ttggggttcg ggatagttgg taatggttgt tgggtgtgtt ttgagatgtt ggggaggaga 6000
tttttttaatt ttgagatata ggcgtgtttg ttaggtttgg gggtttcgtt tttttttgtt 6060
ttagtataga gttataggag gttaggagtt tatttaggtt aattttattt taaaatattg 6120
ttatttaggt tagtaatgga tttaggttag tttagggtcg tgagtgtgaa tttgagagcg 6180
tgtagtgtgg tcgtgagcgt gaatttgaga gtgtgtggtg ttagcgtgag tcgtgagtgt 6240
gagttttaag tgtgaattta agagcgtgcg gtgtggtcgt gagttatgag tgtgaatttg 6300
agagcgtgtg gttgtgagtt gtgtgatttt gagagagtgc ggtgtggtta ttagggtttt 6360
ttttaattac ggttttttag agatgtggta gagtttttta tgacgtttta tatttttttat 6420
ttatattagt tgattttaag ttgtaggggt tgttacgttt ttttttggtt gttttgtaga 6480
attttgagtt tgttgtggtt gcgttaaggg tgagggtttg tattggtttc gacggttttt 6540
agtttttagg atatcggtag aggtggttgt ggtttgttga gatttattag gatgtggtcg 6600
ttcgggggtt tttgagtttt tgtttatggg gaatggcgta ttcgggttag taattgtggg 6660
atagtagaat tgtgggaagt ttgtgtgtac ggagtttacg gggatattta cgtgtatttt 6720
gaagggattt taggacggaa atatgaaagc gaggggttga gggggcggag tagtttttat 6780
tatagagttg tttcgaatgg tttagtagat tcgggaagag ggggttttcg gggttgtttt 6840
ttagtggata gtggttttgg ggtaaggtgg ggatatagat agtaattttt atagtgtttt 6900
ttatagttgg agttgtgaga agggagtcgg ggggagtcgg gtagatttat agtttttata 6960
ttcggagtcg ttgtaggttg ggagggtttc gtttttatag cggacgtagt tggcgttttc 7020
gttcgcgttt tagcgtttgt aatagtttgc gtggggtaga ggacgggtat gttagttaaa 7080
gtaaaattta tattagttgg gtttttgggg aatgattttt tatttgagtt ggtgagataa 7140
tttttatttt agatattaat atgttttgga gggtaggtgg gcggtagata aggatatttt 7200
tagggagtat ggcggggtcg tgagtatttt acgttgagta ttttatattg cgtattttga 7260
ttttgagtat ttcgatttta agtattttac gttgtatttt ttacgtcgag tatttcggtt 7320
tcgagtattt tacgttgtat tttttacgtc gagtatttta gtttcgagta ttttacgttg 7380
tattttacgt cgagtatttc ggttttgagt atttcgattt tgagtatttt atattgcgta 7440
ttttagagag tggtaggcgg tagtatttag aatgaatagg tttgttggag ggattcgtgt 7500
tttgtaggat tgtaggttgg gttcggtttt tattatttag tttttatttt atagattttt 7560
taagaatttt tattgtaacg tttgttttag gtttttttta agattttttt tacgtttttt 7620
ttaggatttt ttaacggttt tttttaggat ttttttttta tggtttttta taggattttc 7680
gtatgttttt tttagggttt tttacgtttt ttcgggattt ttttacgttt ttttgggatt 7740
tttttacgtt tttttaggat tttttttacgg ttttttttag gatttttttac gttttttttta 7800
agatttttat ttgtagtgtt tggagcggtt gtttcgtatt tcgtgttttt ttatgtcgtt 7860
taagtaggat aggtttattc gttagtagta tagtttttttg ttttaagttg atttcgattt 7920
ttggtttatt tgttattttt atattttggt atttttttttc gggttttttt cgggggattt 7980
tgtagaatta agttttattt tttcgttagg tcgacgttgc gtttgaagtt ttattttcgt 8040
ttatcggtta gggcgttttt aaggatagga attgattttt attttttgat tttttaaata 8100
tttttaatat ttagtttaga gttgagtaga tatatttttg atgtcgattg cggtgcggtt 8160
gttggtgcgg gttacgggtg gattatacgt ttcgattttt ttttatagat tgatttgaat 8220
ataaacgggt cgcggcgata cggggatttc gcggtggaga gtgggtttga gatttcggta 8280
cggttgtgat aggggttatt ttgtagatat attttttgga agaatggaag tttattcgtt 8340

```
tagttttatt agggtggagt tgcgtttttt tagtattggg ggtagttttt gtttttcggt    8400
atttttggt atagggttcg ggcgatttta gcgaggtttt gtttgagagt ttttgtttcg    8460
gaggtcgaag aacggagttt acggtaggga gttaaatatt agcgtagtat tgtttttaga    8520
agtgttcgtt gttttttcga gcggagacgt gatttggttt ggggtcgtac gtagggggaa    8580
gcgtatttaa tatcgtaggt tcggtttttt tttacgcgcg ttagacggtt tttattttgc    8640
gtttgtgtat ggggttattt cggtttttgg attttacgat tcgttggttt ggttttttta    8700
tgggtatcgt gattgttgta tagttgtttg tatttataga gtgtttgggg tatataattt    8760
tttataggag acgttacggt tttagtattt ttgttgttat ggggtgttag gggagtggtt    8820
tttatttagt tattagggtt ttagtattag acgatgagga ttttaaggtg tttagttttt    8880
ttttagtttt tttgtttttt tttagagcgg tgtttttttt tttttatttt gcgtgtgttt    8940
tagatgttat tattgttttt ttgtgggttt tttaattttt acgtttagtt tcggattttc    9000
gcgtttagtt ttattggttg ttatagtcgt cgttttgatt acgttttttt tacgaggttt    9060
ttgagttttt aggagtagtt tttttgggtt tttttattat gggagggttg gatttttagg    9120
atgggagtgt taaggttttt ggaggtatag ttggggtatt ttagttttaa gtttattaag    9180
ggtcgttaag gagtggtttt tgtatttttt tattttgttt tttttttttt tggttgtttt    9240
tattggttgt ttttattagt tttaattttt tgatatttag ttgtttttag atagtttttt    9300
tcgtagtttt ttagaatttt tgatgaatag ggtttgttcg tggtacgttg atataggtcg    9360
gtattttcgt ggcgttttgt tgattggtat tttattttac gtattagggg ttagtagtat    9420
ttttgttcga gtacggatag gtaaaaatgt tttgggtttt gttagaggtt tttggagtta    9480
ggatagtttc gtgagaatgg ttgagtttat gggttatttt tttagatttt tttttaggta    9540
ttttttttgat tttagttttt gtttttgttt ttagtttttt gttgggttag tttttttatg    9600
aggttgtcgt ttaaggtcgt tcgttagaaa ggaggtttag atgttttttt ttaagtttta    9660
tagtttttat tgttttattt atttttagg tttcgtagta tagatgagta ttgtttttgt    9720
tatcgagggg agggttttgg gtatatatat attagtggtt taggggtcgg tttggaattt    9780
gtttgggata aagcggttta tttggattat atagatttgc gtttgggtag ttggcgttga    9840
cgtttattat atttatatag tattcgggta gttgggtttg gaggggatac ggtagtgaaa    9900
ttttaggacg cgtttgttag gtaggtttac gtttagagta gtagcggttg aggggtgtcg    9960
tgggagtata ttttaatgtt cgtagtttcg gttttttttg gagttagtat cgggtggttt   10020
tttgtttttt gtgggtttgg gagtaaggag gggttttata gttatagtgt atatttttgt   10080
gggattttgg gtgggcggtt tttaaggaaa agttgggttt ggaagagtaa attattttta   10140
ggaggcggta ttcgtaggtt atggtagcgt tatcgatatt ttttgtaagt tttcggaggt   10200
tttttattag ggttttcgt ttgattttat agaggaagtg ttcgggtttg gttggtattt   10260
taggggacga atttacgttt aggtgtttag gtattgtata ggtgcgttgg gtgttggtat   10320
ttacggtgtg tgtgagggtt tattttttga ttataggtag attggatagg tttggggggt   10380
ttagggtatt tttagtttgt gtgttgatta tagttgtgtt gtttttcgtg atttgaatgt   10440
agtgaacggt gagggcgttt aggggtgatt gcggagtgcg ggatagaatt tatatcgtta   10500
gggaggggtt taggttcggt tttttaaacg ttttagttag gatagtgagg ggtttttatt   10560
ttttattcgt ttcgtttggg agtttggagt cgtcgtagtt atatttttcg atagcgatgc   10620
gcggtacgtt gtgttgttat cgttggtaga ggtgttttta ttgcgggtta ggatgtttta   10680
tcgtgggggt ttcgtttgat ttagttattt ttgagtaggc gtttatttag taggtagcgg   10740
agggtttttt tttgcgaggt ataggttttt ttgattttag tagtggattt ggttagtatt   10800
tttggggttg ggatattagt gttttaaagg tggaagggtt agatttttta ggggaagggt   10860
ttatcgtttt ttatagaggt tttggtttag gtcgcgtgga tacgtgaggg gggtatttat   10920
cgtgtttttt atggatttgt tggcgatttt tacgagaagg ttagttagga gggcgaggtg   10980
tcgtagcgtt atgttaggag tagatgcgta gagtttgtta tagggaggag tatgcggagt   11040
taaagagatg gagtggggtt gaggtagggt gggtggggtt aaatgaaagt ggggttaaga   11100
gatgttgggg gggtgcgggt taaggtaagg agggtagagt taaatggaga tgggtggggt   11160
tgtggtggag ggtggggtta aatggaagtg ggcggggttg tggtggaggg tggggttaaa   11220
tggaagtggg cggggttgtg gtagggaggg tggggttaaa tggagatggg gtggggcggg   11280
gtcgcggtag atgattgagt taaatggaga tggggtaggg tcgtggtagg ggtgtagggt   11340
tacgataggg aaggtggagt taaatggagg tggggtgaaa gagtgaaggt ttggggtttt   11400
tggaggtacg ggtggggcga ggtgtaggta gggttttatt tgttttttta ggatggggat   11460
tatcgatatt agttttttgt tcgtttgggt gtttaggggt tagttttggg agtttatggg   11520
tttagttgag ttttgtagat ttcggtttag ttttgtagat gaagatagta ggtgaggtcg   11580
tggttacgcg agggtaattt aggtgggtcg tggtttacgg tgcggggttt gggttcggtt   11640
cgggtttttt gtttatattt ttgtcggttt ttggttgtcg agtagggcgt tttggagggg   11700
ttgtttttac gagtgttttt ttcgagttgg ttttttggtt ttttgcgggt gagagtgttt   11760
gggatatagg ttttcgttgg gatacgggtt ttcggttcgg tttttgttta gggattaggt   11820
acggagttta gtggtttagg ttgtatttgg tggatatcgg ttttttcgta taaatttttt   11880
tttttttttt tgtattttta gttataggat ttgttattat ggttttgttt ttttaaggga   11940
ggaaacgttt gttaggcgta taaattttta agtggtgtta ttgtattggt ttaggggttt   12000
```

```
ttttggtaag ttatttcgag gggggttgag gtttagttag gtttgtgggg tttttttttga   12060
tgggtttagg tcggggtttt gggataaggg ttgtgagtag tagggagacg gaggttttgg   12120
aggatatagt tttagttttt gtatagggac ggtttatttt tcgtataggg acggtttatt   12180
ttgttattta ttttatgttg gatttaggtt atggttttat aaattgatta ggttgtttag   12240
gtgtgagttt gtcggaggat tttggaaggt gggggtggtc ggaatgtttg agtttaggag   12300
ttggagattt atttgggtaa tatagttcgt ttttataaaa agtttaaaat tagtcgagtt   12360
tggtggtggg agtttgaggt tttagttatt taggaggttg aggtgggatg attatttgag   12420
tttagaaggt ggaggttgta gtgaacggag attgtatttt agtttgtgta aggggaattt   12480
cgttttaaaa aaaaaaagta tgtttttttt tgatttagtt tttttttttt gatgtgaaat   12540
ttttttagat ggaacgtgtt gaagttatag atatgttgtt cgttttattt atagagtgta   12600
attaagtttt agtttgtttt tttgtttttt taatatttgt ttagtagaga tcgttttttt   12660
ttgtttagtg gaaaatttta gatattatag atttttttgt tttttttttg gttaaagggt   12720
attttgaggg ttatattaag ttataaaata ttattttgat ttaggaatta gaagtttaag   12780
attttaatta atatttttat ttgttattta gttaatttta tggagattta ttttatatat   12840
aataaattat attcgtgtaa agtatataag cgggtgagtg tgattatttt tttgaagttg   12900
ttattatagt taggacggtg ttcggtttta tatagttgtt agtattcgtt gcggttttta   12960
taagttcggg ttttcggtag ttaggagttg attagattgt agttgtattt tttagggttt   13020
tatataaagg ggttgtatat attgattttt agattttttt atgttgtttt attcgtattt   13080
tttttttttt tatagtcgga ggatttttta ttgtgttggt taaacgttat cgttt         13135
```

<210> 156
<211> 13135
<212> DNA
<213> Artificial Sequence

<220>
<223> chemically treated genomic DNA (Homo sapiens)

<400> 156

```
aggcggtgac gtttggttaa tatagtggaa aatttttcga ttgtggaaag gaagaaagtg     60
cggataaaat aatatggaag gatttaaagg ttagtgtgtg taattttttt gtgtaagatt    120
ttagaagata tagttgtagt ttggttagtt tttggttgtc gggagttcgg gtttgtgggg    180
gtcgtagcga gtgttggtag ttgtgtggga tcgggtatcg ttttggttgt ggtggtagtt    240
ttaagggggt ggttatattt attcgtttgt gtattttata cggatgtggt ttattgtatg    300
taaagtagat ttttatgaag ttgattaaat agtagatgaa agtgttgatt gaaattttgg    360
atttttggtt tttgaattaa aatgatgttt tgtgatttaa tgtggttttt aggatgtttt    420
ttaattagag agggagtaga agggtttgtg atgtttggag tttttttattg agtaggggaa    480
gacggttttt gttgggtaaa tgttaaaggg ataaaaggat aaattaagat ttgattgtat    540
tttgtgggtg gggcgagtag tatgtttgtg attttaatac gttttatttg aaaggatttt    600
atattagaga ggaggagttg aattagagga gagtatgttt tttttttttg agacggagtt    660
ttttttgtat aggttggagt gtagttttcg tttattgtag tttttatttt ttgggtttag    720
gtgattattt tattttagtt ttttgagtag ttgggatttt aggtttttat tattaggttc    780
gattaatttt aaattttttg taaagacggg ttgtgttgtt taggtgggtt tttaattttt    840
gagtttaagt atttcgatta tttttatttt ttagagtttt tcggtaggtt tatatttggg    900
tagtttggtt agtttgtgag gttatgattt gaatttagta tggaatgggt gatagagtgg    960
atcgtttttg tgcggagagt ggatcgtttt tgtgtagaga ttggagttgt gtttttttagg   1020
gttttcgttt ttttgttgtt tatagttttt gttttaggat ttcggtttag gtttattaag   1080
aggagtttta taggtttggt tgggtttttag ttttttttcgg ggtaatttgt tagggaggtt   1140
tttgagttag tgtagtgata ttatttgagg atttgtgcgt ttggtaggcg tttttttttt   1200
taggaaggta gggttatggt aataggtttt gtggttggaa gtgtaggggg gagagagggg   1260
gtttgtgcgg gaggatcggt gtttattaag tgtagtttgg gttattgagt ttcgtatttg   1320
gtttttgagt aaaggtcggg tcgagggttc gtattttagc gagggtttgt attttaggta   1380
tttttattcg taggaggtta ggaggttagt tcgggggaaa tattcgtgga gatagttttt   1440
ttaggacgtt ttgttcgata gttaggggtc ggtaagggtg tggatagagg gttcggatcg   1500
gatttagatt tcgtatcgta ggttacggtt tatttgggtt gttttcgcgt gattacggtt   1560
ttatttgttg tttttatttg taggattggg tcggggtttg tagggtttag ttgagtttat   1620
gagtttttag agttaatttt tgaatattta ggcgggtaaa gggttgatgt cggtagtttt   1680
tattttggag gggtaggtaa ggttttgttt atatttcgtt ttattcgtgt ttttaaaggt   1740
tttaagtttt tatttttttta ttttattttt atttggtttt atttttttttg tcgtggtttt   1800
gtatttttgt tacggttttg ttttattttt atttaattta gttatttgtc gcggtttcgt   1860
```

```
tttattttat ttttatttgg ttttattttt tttgttatag tttcgtttat ttttatttgg    1920
ttttattttt tattatagtt tcgtttattt ttatttggtt ttattttttta ttatagtttt    1980
atttattttt atttggtttt gttttttttg ttttggttcg tattttttta atatttttttg    2040
attttatttt tatttggttt tatttatttt gttttagttt tattttattt ttttggtttc    2100
gtatgttttt ttttgtggta ggttttgcgt atttgttttt ggtatggcgt tgcggtattt    2160
cgttttttttg gttggttttt tcgtgggagt cgttagtaag tttatggaga atacggtagg    2220
tgtttttttt acgtgtttac gcggtttaag ttagagtttt tgtggagggc ggtgggtttt    2280
tttttagag ggtttaattt ttttattttt aggatattga tgttttagtt ttagaggtgt    2340
tgattaggtt tattgttggg gttagagggg tttgtgtttc gtagagggga gtttttcgtt    2400
gtttgttggg tgagcgtttg tttaggaatg gttgggttag gcggggtttt tacggtgagg    2460
tattttggtt cgtaatggga gtatttttgt tagcggtgat agtatagcgt gtcgcgtatc    2520
gttatcgggg agtgtagttg cggcggtttt agatttttag gcggagcggg tggagggtgg    2580
ggatttttta ttgttttggt tggggcgttt gagggatcgg atttgggttt ttttttggcg    2640
gtgtggattt tatttcgtat ttcgtagtta tttttggacg tttttatcgt ttattgtatt    2700
taggttacga gaaatagtat agttgtgatt aatatataag ttgagggtat tttgagtttt    2760
ttaggtttgt ttagtttgtt tgtggttagg gaatgggttt ttatatatat cgtaagtgtt    2820
agtatttagc gtatttgtgt agtatttggg tatttgggcg tgaattcgtt ttttgaaatg    2880
ttagttaggt tcgggtattt tttttgtggg gttaagcgga aatttttgat ggagagtttt    2940
cggagatttg tagagaatgt cggtggcgtt gttatggttt gcgggtgtcg tttttttgggg    3000
gtggtttatt tttttaggtt tagttttttt ttggagatcg tttatttagg gttttatagg    3060
gatgtgtatt gtggttgtga ggtttttttt tgtttttagg tttatagggg atagagggtt    3120
attcgatgtt gattttagaa gaggtcggag ttgcggatat tgagatgtgt ttttacggta    3180
tttttttagtc gttgttgttt tgggcgtgag tttgtttggt aggcgcgttt tggagtttta    3240
ttgtcgtgtt tttttaggt ttagttgttc gagtgttgtg tggatgtggt gggcgttaac    3300
gttagttgtt taggcgtaag tttgtgtggt ttaggtaagt cgttttgttt taagtagatt    3360
ttagatcggt ttttgagtta ttggtgtgtg tgtgtttagg gtttttttttt cggtgataag    3420
gatagtgttt atttgtgttg cgggatttgg ggagtggatg gggtagtgag ggttgtggag    3480
tttggggaag gatatttgag tttttttttt ggcgggcggt tttgagcggt agttttatga    3540
gaagattggt ttagtagagg gttgggagta gaggtaggag ttgagattag aaaggtgttt    3600
ggagaagggt ttgggggagt ggtttatgag tttagttatt tttacggggt tgttttggtt    3660
ttaggggttt ttggtaaagt ttaagatatt tttgtttgtt cgtgttcggg tagaggtgtt    3720
gttggttttt ggtgcgtgga gtggaatgtt aattaatagg acgttacgag gatgtcggtt    3780
tgtgttagcg tgttacgggt aggttttgtt tattagaggt tttgggaggt tgcggaaggg    3840
attgtttggg ggtagttggg tgttaggagg ttgaggttgg tgagggtagt tagtgggagt    3900
agttagggga ggggaggata gagtgagaag gtgtaagggt tattttttgg cggtttttgg    3960
tgaatttggg gttgagatgt tttagttgta tttttaaggg ttttggtatt tttattttgg    4020
gggtttagtt tttttatgat ggaagaattt agaagggttg tttttgggga tttaggggtt    4080
tcgtgaggag ggcgtggtta agacggcggt tgtggtagtt agtgaggttg ggcgcggggg    4140
ttcgaggttg ggcgtggggg ttggggagtt tataggaagg tagtgatgat atttgaggta    4200
tacgtagggt aggaggagga gggtatcgtt ttgagggaga atagaggggt tgggaggaag    4260
ttgagtattt tggggttttt atcgtttgat gttgaagttt tggtggttgg gtgagaatta    4320
ttttttttggt attttatggt agtaagagtg ttggggtcgt ggcgtttttt gtgagggatt    4380
gtgtatttta ggtattttgt gggtgtaggt agttgtgtag tagttacggt gtttatggag    4440
gggttaggtt agcgggtcgt gaagtttaag ggtcgaggtg gttttatgta taggcgtagg    4500
gtggggatcg tttggcgcgc gtggggaggg gtcgggtttg cggtgttgga tacgtttttt    4560
tttgcgtgcg gttttaagtt aggttacgtt ttcgttcggg gaggtagcgg gtatttttgg    4620
ggatagtgtt gcgttggtgt ttggtttttt gtcgtggatt tcgtttttcg gttttcgggg    4680
tagagatttt tagatagagt ttcgttgaag tcgttcgagt tttgtgttag gagatgtcgg    4740
gagatagagg ttgtttttag tgttgggaga gcgtagtttt attttggtag gattgagcgg    4800
gtggattttt atttttttaa gaggtgtgtt tgtagagtga tttttgttat aatcgtgtcg    4860
gggttttagg tttatttttt atcgcggggt tttcgtgtcg tcgcggttcg tttgtattta    4920
gattagtttg tgaggaaggg tcgaggcgtg tggtttattc gtggttcgta ttagtagtcg    4980
tatcgtagtc ggtattaaga gtgtgtttat ttagttttgg gttgagtgtt ggagatattt    5040
agaaagttaa ggagtaaaag ttaatttttg tttttaaagg cgttttggtc ggtgggcgga    5100
aatgaggttt taggcgtagc gtcggtttgg cgggggggatg aggtttggtt ttgtaggatt    5160
tttcgagaga gattcggggg agggtgttag ggtgtggagg tgatagatgg attaaggtc     5220
gggattagtt tggggtagga ggttgtgttg ttggcgggtg ggtttatttt gtttgggcgg    5280
tatgagggaa tacggggtgc gaggtagtcg ttttaggtat tgtagatggg ggttttgggg    5340
gaggcgtggg gggttttggg ggaggtcgtg ggggggtttt ggggaggcgt gggagggttt    5400
tagggaggcg tggggaggtt tcggggaggc gtgggggatt ttgggggagg tatgcggggg    5460
ttttgtggga ggttatgggg ggggggtttt gggggaggtc gttgggggat tttaggagag    5520
```

gcgtgggggg ggttttgggg gaggtttggg gtaggcgttg tagtgggggt ttttggggag 5580
tttgtggggt gaaggttggg tggtggaagt cgggtttagt ttgtagtttt gtagagtacg 5640
agttttttta ataggtttgt ttattttgga tgttgtcgtt tattattttt tgaagtacgt 5700
agtgtggagt atttagggtc ggggtgttta gggtcggggt gttcggcgtg gagtgtagcg 5760
tggagtattc ggggttgggg tgttcggcgt ggagagtgta gcgtggagta ttcggggtcg 5820
gggtgttcgg cgtggagagt gtagcgtgga gtatttgggg tcggagtatt tagggttagg 5880
gtgcgtagtg tggagtattt agcgtggggt gtttacggtt tcgttatgtt ttttgggggt 5940
gtttttgttt gtcgtttatt tattttttag agtatgttag tatttggaat gaaagttatt 6000
ttattaattt aaatagagaa ttatttttag gagatttagt tggtgtggat tttgttttgg 6060
ttgatatgtt cgtttttttgt tttacgtagg ttgttatagg cgttggaacg cggacgggag 6120
cgttagttgc gttcgttgtg ggaacggaat ttttttagtt tataacggtt tcgagtgtag 6180
aagttgtagg tttgttcggt ttttttcggt ttttttttta tagttttagt tgtgggagat 6240
attgtgggag ttgttgtttg tgtttttatt ttgttttaga gttattgttt attggggaat 6300
agtttcggga gttttttttt ttcgagtttg ttgggttatt cgggatagtt ttgtggtgag 6360
ggttgtttcg tttttttagt ttttcgtttt tatattttcg ttttgagatt tttttagggt 6420
gtacgtgggt gttttcgtgg gtttcgtgta tataggtttt ttatagtttt gttattttat 6480
agttgttggt tcgggtgcgt tattttttat gaatagaagt ttagggattt tcgggcggtt 6540
atattttggt aagttttagt aggttatagt tatttttgtc ggtgttttgg gggttggggg 6600
tcgtcggagt tagtgtagat ttttattttt gacgtagtta tagtaggttt agggttttgt 6660
aaaatagtta gaggggagcg tggtagtttt tgtagtttgg ggttagttgg tgtgggtggg 6720
ggatgtgggg cgttatgggg ggttttgtta tatttttaaa gggtcgtagt taggggaggt 6780
tttgatggtt atatcgtatt tttttagggt tatatagttt atagttatac gtttttaggt 6840
ttatatttat ggtttacggt tatatcgtac gtttttaggt ttatatttaa ggtttatatt 6900
tacggtttac gttgatatta tatattttta ggtttacgtt tacggttata ttgtacgttt 6960
ttaggtttat atttacggtt ttgagttggt ttgggtttat tgttgatttg agtggtagtg 7020
ttttagggtg gggttggttt ggatgggttt ttggtttttt gtggttttgt gttggggtag 7080
gaaggagcga ggtttttaag tttggtaaat acgtttgtgt tttagggttg gggagttttt 7140
tttttagtat tttagagtat atttagtagt tattgttaat tgtttcgagt tttaggttag 7200
tagttttggt ttgttttggt ttaggtaggt ggaggtgagt ggggtgtagg agtatttgtt 7260
gttagtttga ggttttgttt tgtgtggggg ttttagggtt tgtgttgagt tcggaggaag 7320
gaataaggtt gagaattttt tggtttttag gggtaattgg aatcggagag gtgttttcgt 7380
agagttcggt tagggttagt ttttgtaacg tttttaatat tcgagttttt gagagaagtt 7440
gaggtcgggg taggtggtaa tttttttaga tttcgcggtt agatgtttag gtatggttga 7500
gtttgtaggg atgatttagg tttatgtgta gagttttcgg tatttatagt cggtttcgtg 7560
aggtttaggt atgtttagag agtagcgtat ttgttatgtg tagggggtat cgggggttag 7620
gcgaggagtt agggttatcg ttttgttcgt ttgtttagcg gtgtttgttt gtaggggttt 7680
cgcgcgtggt cgtttttttt tttttgggta cgttttttat tagtttcggt tttatttttt 7740
tcgtagttgg gttttttttat tttaagcgtt ttagtaaatt ttttagggtt tgttatagaa 7800
gaaataaagg tatttttcgg attagtattt gttttacggg agggtatagg gtaggcggta 7860
tttagtcgtg gagatgggtt tgagattgga gtgttttagg ttagagtagg ttggtagagg 7920
tcgggaggga gtagggtttg ttttgtggtt tttgggtttc ggtgtgagaa ggttcgttat 7980
ttttcggaaa tagtcgtgtg tggatttgaa gggtcgaagt attaaggggt ttttttcggg 8040
gtttagaatt tttgttggag ttttttttcg gtagaggcgt tttttcgggg tttttagggt 8100
attacgaggg gcgagtggag cggggttggg tggttttcgg tagttggatt tgtttgggtt 8160

tttttagtag ttatggtttt tttttttttt ttttggggtt agggttggtt aggttggtgt 8220
agggtttata ttttagttta cgttacgtcg tatatacggt tatttcgggt ttgggtattg 8280
attttttttt attggagaga cggttttgtg attgtggtgt ttttaggcgg gggcgggttt 8340
tgagtagggt tcgtggtgtt tgttttcgtt gtttagggtc ggttttcgta gggtttttgt 8400
taggtagatg gtttagttag tagttcggat tttaaggtga gtatttggtt agattgtgat 8460
agtcgagttg gggttttttt ttaggttttt gggtagtttt atttggttgt tgtgagattt 8520
ttgtttattt tttttaattt ttttcggggt tttagggttt agtttttagt cgggagttcg 8580
ttgttagggt tttttgtata gttaggtatc gagagattcg gtttgtttag tatttttgtt 8640
tatgggtatt tttggagtag agtagggttt agggtttggt tagttttggt ggttacggga 8700
cgagaagtta ggttggttac gatgttacgg tatttttatc gcgggggtat tatggaggta 8760
gaagtttacg gtcgtggggt ttaaagttat ttcgtgtttg aggttttttt cgggagggtg 8820
ggagggtagt tttttttttg ggattttgga gtttggggag acgtttttta tgaggggggt 8880
ttgttttttt tgttgaggcg ggaaggtggg tttagggagc ggggatttat ttggggtgtt 8940
agggaggttt tcgtatttgt gagtgtttcg gaggaatgtg gtaggatcga gggttaggtt 9000
gatgtatcgt agggagaggt tgggggagat tttgtagttt cggggttttt gttgtttcga 9060
ggacgtcgta ttgggttgag gttgaggtag tttttcgttt tgttgtttcg gacgttgtat 9120

```
tgggttgagg ttgaggtagt ttttgggttt tagggtgatt tttcgttttt ttttttatag    9180
tttcggtttt gtagtttggc gaagtcgtaa gtaatttata ttattcgggt tgagcgtttt    9240
tggtcgcgtg tgtttgtgtt aggttatttc gagtttttat tttgtttttt tttttttttt    9300
aaggttgtaa tgatttttttc gttatagttt ttaggtaata gtagttgtcg tatttttttg    9360
tggggttttt tatttttggg ttagttttag ggtgttttgt gggtttgttt ttaaacgggt    9420
ttgtttggtt ttggatttcg gttatttttg ttcgggtttt taggagattt atttataagg    9480
tagggttagg gtcggatttg gttggtgttt tttgttttgg attttagttg gatttggtcg    9540
gcgttttttg ttcgtttttt tttaattttc gtgattttta tgtttttttt tgttttcgag    9600
acgtggggtt tgtaggaaag gaggatttat tacgataggg tagattttta atatttgttt    9660
tttttgaag tttagtttta ttcgaggata gacgtagtcg gttttcgtta ggttttttttg    9720
agtagttatc gttttagtgg tgttgggtta ggcggattta agagttagtt cggttaggat    9780
ttattttatg tgcggggtta tagatatagg ttgagtgatc ggcgtatagt tatatagttt    9840
taaggttatg agtagggttt attcggtttt cgtttttttg tgtagaagtt tgttatgggg    9900
gtaggtgatc gtgtttttag gggatagatc gttttttatt aaggttggac gtgtttcgtt    9960
tttaaagtag ttttgtttgt aagtgtttag tagattttac ggtgttcgag tgtttcgggg   10020
ttgttagttt taatagtaga tttttaattt gattatttta gagtttagaa gtttaaaatt   10080
aaggggtaaa taaggttata cgtttttcga ggtttcgggg aggacgttgt ttgttttttt   10140
agtttttgtc ggtttaggta tttgtggttg cgttgttcgt ttttgttttt tggttttacg   10200
gcgttttttt tatttttgcg tttttttttt ttgttttttt tttaggtggg ttttggtcgt   10260
tgttttaggg tttgttcggg gaattttggg cgattatatt tgagattttg acgtaatttt   10320
ttgtaaagtt tttttttttta aataaggtta tattcgaggt tttgggatta cggtttgttt   10380
gtcgtttttt gaggtgtatg atattttggg atttatattt ttgttttcgg ggatcgtttg   10440
gtttttgttt aggtttcggg gtttagggtg taaatgagag gatatagtcg ggtaggagga   10500
agtcgttttt ttttcgagtt ggagttttga gggcgtttta ggggtagggg gtatttgggt   10560
ttttatttgg ttagagtaaa tagtttcggg cgtttgtgtt atatgtttat tttcgagagt   10620
tttcggtgag ggttgaggtt tgtattgttt ttgttgttta ggttttattt tgatttcgtt   10680
tgtttttgat tcgttttagt acggaagtcg cgttacgtta ggcgggagcg gtttttggat   10740
agggttacgg atttcgttgt tttttcgggg gaggttcgta ttagtaatgt ttgatttgga   10800
ggtcgagatt acgttacgta tttggcggta gggattcgga ggtcgatttt ttggcgggaa   10860
ttagtataaa gtgttggtat cgttcggcgt tcgggatagt tttgggtata gtttcggttt   10920
tgagtttttt cgttttttag cgacggacgt taaagggttt cgggtcgttt gaggtttttt   10980
tttattatag ttatttcgtt tatcggatta ggagtaggta tttatgagat tttagagttt   11040
tagatcgagg ttttgggggg ttcgggtttt tttaggaaat acggtgaggt tttagcgttt   11100
gtagttaaag ttggtacgat ttatggggta ggtgtcgttt tgtttagaaa agttaggggt   11160
tttgttgtcg tgtttttttag agtttatagc gggtaggatt tttttagtat tattatattt   11220
agtggttcga gattttttttg agaatagtga ggttggtttt cgtgtcgttt tagtcggtgt   11280
tcggttagtg gggaggatat agtttaggaa ttagttgttt gagattaggg tgtttttggg   11340
ttgttttttc gcgtggcgga gattttaagt acgtagttat ttattttcgg agttgtagga   11400
tagagttttt ttttgatttt tgtttttaag tagaaattta ttgtgtagaa aagtttttta   11460
gagttttgtg gtttcgttcg gattcgttgg atttttatgt ttggttgatt tttgtttacg   11520
tggggtaggt ttatatttaa tttttataag ttattgtttt attgtatttg ttaaggttgt   11580
tttggcgttg agttttgggg tttttttcgg agtttttggg agaaaggcgt cgtcgtggtc   11640
gtttttcgta cgttaggttc gggttttatc gtgggtttta gacgttttgc ggtatcggta   11700
tcgtttgttt tagtatggga tttttttttg aggggtggtt tggttttcgg ggtttttacg   11760
ttttttttgcg aagtttattg tgggtgttat tatggttttc gggatttggg ttagcgggaa   11820
cgtgggggta ttgggtgtgt tgatataaag tcggtattat ttaagttgcg tttcgagttg   11880
tttttttttcg taggatgttt tgttttttaa gtgtaggtcg gggagtaaag ttttcgttag   11940
gttggtttta cgtatttcgt tatcgtgtgt tgggggtaag gaggtgggac gggggtggag   12000
gagagtttag gttggggttg tttggttggg atttttcgtt tcgcgtcgta tattttttgt   12060
tataggttta tttttagat gtaatttgtg taggtgggat ttgtggtttt cgaattttta   12120
gttcggagtg aggtgggata gagtgtgggt gtgcgttttt atcggtcggt cgggttgcgt   12180
gggggttgta gatacgggtt tcgggagggt tgttatggtt atttgggggg gtagtagtgt   12240
gggggtgttt gttgatagtg atgggtttgg ttagagaggg ttgagttggg gtaggatagg   12300
ttttgagggt agagggttcg ggtattagga tgttagagcg cgggttttgt tttcgtaatc   12360
gtgtttttta tttaggtaga aacgtttatt gtttggaggg tcgtgttttt agatatttgt   12420
atttttgttt ttaaaaattt ttagtttgag gtttgtagtt agggagtatt tgggggagta   12480
ttggttttgt tttgggtttt ggtttaagaa gtgttttggg gttgggaggg ttattggttt   12540
tggggagaaa gggagggatt ggatagttta aggttatttt gagtttagta tttatcgttt   12600
tatttgatgt tttgggttac gtttttgtga cgcgggagtt tttagatttt ttttgtgggg   12660
aaggagatag ggatcgagat gggagtagag ggaaggagat agggatcgag gtgggggcgg   12720
agggaaggag atagggatcg aggtgggggc ggagggaagg agatagggat cgaggtgggg   12780
```

```
gcggagggaa ggagataggg atcgaggtgg gggcggaggg aaggagatag ggatcgaggt   12840
gggggcggag ggaaggagat agggatcgag gtgggggcgg agggaaggag atagggatcg   12900
aggtgggggc ggagggaagg agatagggat cgaggtgggg gcggagggaa ggagataggg   12960
atcgaggtgg gggcggaggg aaggagatag ggatcgaggt gggggcggag ggaaggagat   13020
agggatcgag gtgggggtgg agggaaggag tttatatttt tttggaaatt ttatttttgt   13080
atttgtatta ggttgggcgt ttggtttgga ggataaaatc ggaaataggt acgaa        13135
```

<210> 157
<211> 4852
<212> DNA
<213> Artificial Sequence

<220>
<223> chemically treated genomic DNA (Homo sapiens)

<400> 157

```
ttcggttgat tgtaagtttt gtttttcggg tttattttat ttttttgttt tagtttttcg      60
agtagttggg attataggtt tttgttatta ggttcggtta aatttttttt tttttttgag     120
acggattttt attttgtcgt taggttggag tgtagtggcg cgatttcggt ttattgtaag     180
ttttgttttt tgggtttacg ttattttttt gttttagttt ttcgagtagt tgggattata     240
agcgttcgtt attatgtttg gttaattttt tgtattttta gtagagacgg ggtttcgtta     300
tgttagttag gatggtttcg attttttgat tttgtgattc gtttatttcg gtttttttaga    360
gtgttggggt aataggtacg agttacgacg tttagttttg gatatatatt ttgaaaggta     420
atggggtgat aaagagagga agattgggtt gggcgcggtg gtttacgttt gtaattttag     480
ttttgggtgg ttgaggtgag cggattatga ggttaggagt ttgagattag tttgattaat     540
atggtgaaat ttcgttttta ttaaaaatat aaaaattagt tgggtatggt ggtgtatgtt     600
tgtaatttta gttattttgg aggttgaggt aggagaatcg tttgaattcg ggaggtggag     660
aggttgtagt gggttgagat tgtgttattg tattttagtt tgggtaatag agtaagattt     720
cgttttaaaa aaaaaaaaaa aaaagagtaa gattgtagaa gtggtatttg ggtgtttggg     780
tgggaggaat tcgggagttt gatttttaga tgtaagtttg aaatatttag gtcgggcgcg     840
gtggtttata tttgtaattt tagtattttg ggaggtcgag gcgggtagat tacgaggtta     900
ggagatgaga ttattttgat taatatagtg aaattacgtt tttattaaaa aatataaaaa     960
attagttagg cgtagtggta ggcgtttgta gttttagtta ttggggaggt tgaggtagaa    1020
tggcgtgaat tcgggaggag ttcgtagtga gtcgagatcg tgttattgta ttttagtatt    1080
ttagtttggg taatagagta agattttatt ttaaaaaaaa gaaatattta ttagatattt    1140
aattgggtat gtatattaga tagttggata tatgattttg gagttagggg atgagtttag    1200
aaagaaatat aaattacgga gttgttagcg tagagatggt gtagaaagtt ttgacgttga    1260
aggttgggta tagtggttta tttttataat tttagtattt tgggacgttt aggtgggagg    1320
atcgtttgag cgagtttaat attagtttgg gtaataaagg ttggtattat aaaggttcgt    1380
ttttataaaa aaataaaaag ttagttaggt gtggtagttt aagtttgttg ttttagttat    1440
ttaggagatt gaggtgggag gattatttga gttttggagg ttgaggttgt agtgaattat    1500
aattgtgtta ttgtatttta gttttgggga tatagtaaga ttttagtgtt ataaatatat    1560
aagttttgat atggaaagag attattatat tttatgtgtt tttttatttt tgtttatttt    1620
attatttggt gtttttaagt ttaatatttt ttttgttgat ttgaggttat atagtttggt    1680
gaaagagggt cggatttttg gtttttttaat atatttatat acgtttgttt aaaattttta   1740
gtgttttatg tttttagttt ataaagtata gatatagttt taatgagagg tttttatatt    1800
aggttagaga atgttagttt tttttatttt tatagagatt ttaaaaagta tattttagat    1860
aaaaaagaaa aataagtaat taagattatt ttagggttgg gtacggtggt ttatatttgt    1920
aattttatta ttttgggagg tcgaggcggg cggattattt gaagtcggga gttttagatt    1980
agtttgatta aggagaaatt ttgtttttat taaaaatata aaattagttg ggcgtggtgg    2040
cgcgtgattg taattttagt tattcgggag gttgaggtag gagaatcgtt tgaatttcgg    2100
aggcggaggt tgtagtgagt tgaattttat tattgtattt tagtttgggc gatagagtga    2160
gaattcgttt taaaaaaaaa aaacgttttt tttataagga attttgagga tttgttggtt    2220
taattgtttg gatataaaga tatatatata tgaagttgga gagataggaa gaagcgttta    2280

atatagaata ttttaagtat ttttattttt ttttaaggat taaatttttt tagagatttt    2340
ttttgtttta gtttttttagg agtttttcga agatgttagg agtcgtttaa gaaggttgga   2400
agatggtcgt tgggaaggaa ttgttggtag ttggtaatat tttattttaa ggaggcggtg    2460
gtaatagttt tttgggtagt agatattatg gatttttttt tttttgggaa ttagaggttt    2520
tttcgttttt ggtttgaatt ttttagagat aaagaatttt attttttttt atattcgcgt    2580
```

```
gggattgttg gggaggagag ggggtggagg ggttagattt ttatttttat gaaatattcg    2640
cgggtttaaa gaggagtttg gattttgatt ggtttaggag gcgtgcgggg tcgcggggtt    2700
ttttcggttt gggttaggcg cgttgttaag atgttcgggg aaggggtcgt tcggcgtagg    2760
tcggggacgt ggagcgttcg gggcgttttt acgtaggtta tatattggtc gtttgtattt    2820
tttttttttt tttttaattt tattttttcg cgtttttttt tcggggagtt ttcgtttttt    2880
tttttttttc gcggtcgttt tcgttttttt ttttttttta gatttttttt cggttcgttt    2940
ttttttcggt agtttatttt tatttcgttt tttcgcgcgt ttttcggttt tcgtttttta    3000
gtttcggttt tttttcgtcg gttttcggtt ttcgcggttt ttttttttgtt tttttcgagg    3060
cggcggcggt ttttttttata atagcgcggt tcgttttttt tttttcggtt ttcggtgtat    3120
tttcgttcgt tttttttttc gcgtatgcgt ttttcggttg gcggcggttg cggcggtggc    3180
ggttacgagc ggtttcgttt ttttaaaggg aaacgttgag gcgtcggggg tgattgtggg    3240
ggagggggat ttcgagtcgc ggagatttc gggagaggcg atagattttt tttttcggag    3300
taggagggtt ttgggcggat ttagtttttt attttttttt gaggaggagg ggggggaaat    3360
ggaggttcgg ggcggttgag gcgagtttcg gggcgggggg tcggggcggg gaagggggggg    3420
tgcggggtgg ggagacgagg cgggttcggt cgggttaggg ggggtcgaag cgagtttcgg    3480
ggatgagttc gggggcggtt gggtgcgagt ttttgttttt gaggcgaagg cggcggcggc    3540
ggtagtagag gcggcggcga ggttttttatg ggtcggcggc gggttttagt cgcggttttt    3600
acgttttcgt acgtcggcgg gatggcgttc gggtttcgga ggagtcgcgt tagcggaggt    3660
ttgttgtcgc gttgttgagg cgagttcgtt aaattttttt tttttttttt agttttcgat    3720
ttttcgtatt tcgttttttt tttatttttt ttttcgtttc ggtgttcggc gttgtttcgg    3780
attattatga ttatgagatt cgcggtgttt aaggcggtcg cggtttttgt cggcggtaat    3840
tttgagtagc gattggatta cgagcgggtt gcggcgttgg gcgggttcga ggacgagttt    3900
ggggcggtcg aagtttattt ttttttttcgg tatcgtaagt ttaaggagtc ggggttttcg    3960
ttggtttttt tttagggcgg gagtttcgcg tttttttcgg tcggttgcgg cggtaagggt    4020
cggggtttgt tattttcggt cggggcggtt ttcgggtagt aggaagagag ttggggcggt    4080
tcggtgtttt tgttttgttc gttttcggtt attaagtaag tcggtattgg gggggagttt    4140
gtcgtagtcg gagtcggttg tagttttcgg tttaagtatt aggcggtgtt gtttatttag    4200
acgggttttt tcgtggcggc ggttaaagag tttacgtttt gggttgggga taagggtggg    4260
gcggtttttt tcgttgttat cgtttcggat tcggcgggat ttttattatt atttttgttc    4320
gggtcgttat ttttcgcgtt tatcgttatc gtcgggattt tggcggttag cgagggtaga    4380
tggaagagta tgaggaagag ttttttcggg ggtggtggcg gttcgggagt ttttagttag    4440
gtcgtttgtt ttaaatagat ttttttgttg taattggatt ttatcgaata gtagtagtag    4500
tagttgtagg ttaaggaaaa ggagatcgag gagttgaagt tagagagaga tacggtacgg    4560
gaggggttaa tttgtattcg ggtcgaggag cgagttgtgc gtatgttcgg gggaaggggg    4620
ttgtaggagg ttaaggtatt ttcgggttag gggtaaagga ggttggttat cggtaaagga    4680
gtattttagg cgttgggttt ttgggagtta ggtttataga tacgttgggt tggagggaag    4740
ggttaaaggg taattttttta gggggagggg gaggggtttt aggtgttaaa gggttaattt    4800
aaaatgatag ttttagacgt gtgggaaaga cgtttagtgg aaaaagacgt ga            4852
```

<210> 158
<211> 4852
<212> DNA
<213> Artificial Sequence

<220>
<223> chemically treated genomic DNA (Homo sapiens)

<400> 158

```
ttacgttttt ttttattaaa cgtttttttt atacgtttgg ggttgttatt ttaaattaat      60
tttttggtat ttgaaatttt tttttttttt tttgagaggt tgttttttaa tttttttttt     120
taatttaacg tgtttgtgag tttggttttt agagatttag cgtttaagat atttttttgt     180
cggtggttaa ttttttttat ttttaattcg gggtgtttt aatttttttat agtttttttt     240
tttcgagtat gcgtataatt cgtttttcgg ttcgaatgta gattaatttt tttcgtatcg     300
tgtttttttt tgattttagt ttttcgattt tttttttttt ggtttgtagt tgttgttgtt     360
gttgttcgat gaggtttaat tgtagtagaa ggatttgttt gaggtaggcg gtttgattgg     420
aggttttcga gtcgttatta ttttcgagag ggtttttttt tatattttt tatttgtttt     480
cgttggtcgt tagggtttcg gcggtggcgg tgggcgcgag gggtggcggt tcgggtagag     540
gtagtggtgg gggtttcgtc gggttcgagg cggtggtagc gggggaggtc gttttatttt     600
tgtttttagt ttagggcgta ggttttttgg tcgtcgttac gagagagttc gtttgaatgg     660
gtagtatcgt ttgatatttg ggtcgggggt tgtagtcggt ttcggttgcg gtaggttttt     720
```

tttttaatgtc ggtttgtttg gtggtcgggg gcggataggg taagggtatc gaatcgtttt 780
agttttttttt ttgttgttcg ggggtcgttt cggtcgggag taataagttt cggtttttgt 840
cgtcgtagtc ggtcggggaa ggcgcggggt tttcgttttg ggaggaggtt agcgggggtt 900
tcggtttttt gagtttacgg tgtcgggggga ggaagtgggt ttcggtcgtt ttaggttcgt 960
tttcgggttc gtttagcgtc gtagttcgtt cgtagtttag tcgttgttta ggattgtcgt 1020
cggtaggggt cgcggtcgtt ttgaatatcg cggattttat ggttatagtg gttcggagta 1080
gcgtcgggta tcgaggcgga ggagggggtg gggaaggggc gaggtgcggg gggtcgagga 1140
ttgagggggg ggaggggagt ttggcgggtt cgttttagta gcgcggtagt aggttttcgt 1200
tagcgcggtt ttttcggggt tcgggcgtta tttcgtcggc gtgcggagac gtggaggtcg 1260
cggttgaggt tcgtcgtcgg tttatggggg tttcgtcgtc gtttttgttg tcgtcgtcgt 1320
cgttttcgtt ttagaggtag gagttcgtat ttagtcgttt tcgagtttat tttcggagtt 1380
cgtttcggtt ttttttggtt cggtcgggtt cgtttcgttt ttttatttcg tatttttttt 1440
ttttcgtttc ggtttttcgt ttcggagttc gttttaatcg tttcgagttt ttattttttt 1500
tttttttttt ttaggagggg gtggagggtt gggttcgttt aaagtttttt tatttcggga 1560
gagggggttt gtcgtttttt tcgggggttt tcgcggttcg gggttttttt tttttatagt 1620
tattttcggc gttttagcgt ttttttttaa aaaaacggag tcgttcgtag tcgttatcgt 1680
cgtagtcgtc gttaatcgga ggacgtatgc gcggggaggg gagcgggcgg gagtgtatcg 1740
ggagtcgaga ggagggaggc gggtcgcgtt attgtgaaag gggtcgtcgt cgtttcgagg 1800
ggggtaggga ggggatcgcg ggggtcgggg gtcggcgagg ggaggtcggg gttgaggggc 1860
gaggatcgag gagcgcgcgg aggagcgggg tgaaggtggg ttgtcgaggg aggggcgagt 1920
cgggggaggg tttggagggg gaagaagggc gagggcggtc gcgggaagaa ggggaaagcg 1980
gaggtttttc gggaaaaaag cgcgagagaa tggagttgag aggaaagaag gaggatatag 2040
gcggttaatg tgtggtttac gtgggagcgt ttcgagcgtt ttacgttttc ggtttgcgtc 2100
gggcggtttt tttttcgggt attttggtaa cgcgtttggt ttaagtcgga ggggtttcgc 2160
ggtttcgtac gttttttgga ttagttagga tttaggtttt tttttgagtt cgcgggtgtt 2220
ttatgggggt agaagtttaa tttttttatt tttttttttt tttagtagtt ttacgcgggt 2280
atgggagaga atgaagtttt ttgtttttaa gggatttaaa ttagaaacgg agggattttt 2340
ggtttttaga gggaggaaaa tttatgatgt ttgttgttta gggagttatt gttatcgttt 2400
ttttgggatg aagtattgtt agttattaat agtttttttt taacggttat tttttagttt 2460
ttttaaacga tttttagtat tttcgggagg tttttgaagg attgaagtaa aggaaatttt 2520
tgaagggatt tagtttttga aagggagtag ggatatttag ggtgttttgt gttgagcgtt 2580
ttttttttatt tttttagttt tatgtatgtg tgtttttatg tttaagtaat tgagttaata 2640
agtttttaga attttttgtg aaaaaaaacgt tttttttttt tgagacggat ttttattttg 2700
tcgtttaggt tggagtgtaa tggtgaaatt tagtttattg taattttcgt tttcggggtt 2760
taagcgattt ttttgtttta gtttttcgag tagttgggat tatagttacg cgttattacg 2820
tttagttaat tttgtatttt tagtagagat aggatttttt tttggttagg ttggtttgga 2880
attttcgatt ttaggtgatt cgttcgtttc ggttttttaa agtggtggga ttataggtgt 2940
gagttatcgt gtttagtttt gaaatagttt taattgtttg tttttttttt ttgtttgagg 3000
tgtgtttttt aaaattttta tggagatgga gaagattgat atttttttggt ttgatgtgaa 3060
aatttttttat taaaattgtg tttgtgtttt gtgggttgaa agtatgaaat attggaaatt 3120
tttaaataaac gtatgtgaat gtgttaagaa gttaagagtt cggttttttt ttattagatt 3180
gtatggtttt agattagtaa gggaagtatt gagtttaggg atattaagta atggagtgag 3240
tagggatgag agggtatata gaatgtggtg attttttttt atgttaaggt ttgtgtgttt 3300
gtggtattgg ggttttgttg tgtttttaag gttagagtat agtggtataa ttatggttta 3360
ttgtagtttt aatttttagg gtttaagtga tttttttatt ttagtttttt aagtagttgg 3420
gataataggt ttgggttgtt atatttggtt aattttttat ttttttgtag agacgggttt 3480
ttgtaatatt agtttttgtt gtttaggttg gtattgaatt cgtttaagcg attttttttat 3540
ttgggcgttt taaagtgttg ggattatagg agtgagttat tgtgtttagt ttttagcgtt 3600
aagatttttt gtattatttt tacgttgata gtttcgtaat ttatattttt ttttggattt 3660
atttttttaat tttagaatta tatatttaat tgtttagtat atatatttag ttggatgttt 3720
aataggtatt tttttttttt gaggtggagt tttgttttgt tgtttaggtt ggagtgttgg 3780
agtgtagtgg tacgatttcg gtttattgcg agtttttttc gggtttacgt tattttgttt 3840
tagttttttt agtagttggg attataggcg tttgttatta cgtttggtta atttttttgta 3900
tttttttagta gagacgtggt tttattgtgt tagttaggat ggttttattt tttgatttcg 3960
tgatttgttc gtttcggttt tttagagtgt tgggattata ggtgtgaatt atcgcgttcg 4020
gtttaggtat tttaaattta tatttaaaaa ttaagttttc gaattttttt tatttaaata 4080
tttaaatatt atttttatag ttttattttt tttttttttt ttttttgaga cggagttttg 4140
ttttgttgtt taggttggag tgtagtggta taattttagt ttattgtaat ttttttattt 4200
ttcgggttta agcgattttt ttgttttagt ttttagagta gttgggatta taggtatgta 4260
ttattatgtt tagttaattt ttgtattttt agtagagacg gggttttatt atgttggtta 4320
ggttggtttt aaatttttga ttttatgatt cgtttatttt agttatttaa agttgggatt 4380

ataggcgtaa gttatcgcgt ttagtttagt ttttttttt ttgttatttt attgtttttt 4440
aaaatgtatg tttaaggttg gacgtcgtgg ttcgtgtttg ttattttagt attttgggag 4500
gtcgaggtgg gcggattata aggttaggag atcgagatta ttttggttaa tatggcgaaa 4560
tttcgttttt attaaaaata taaaaaatta gttaagtatg gtggcgggcg tttgtagttt 4620
tagttattcg ggaggttgag gtaggagaat ggcgtgaatt taggaggtag agtttgtagt 4680

gagtcgagat cgcgttattg tattttagtt tggcgataga gtgagagttc gttttaaaaa 4740
aaaaaaaaaa tttagtcgag tttggtggta ggggtttgta gttttagtta ttcgggaggt 4800
tgaggtagga gaatggggtg aattcgggag gtagagtttg tagttagtcg ag 4852

<210> 159
<211> 2519
<212> DNA
<213> Artificial Sequence

<220>
<223> chemically treated genomic DNA (Homo sapiens)

<400> 159

tatgtaacgt tggattttaa atcggtttta atttttaaaa agtttatagg tacggatata 60
aatttttagt tttcgtggtt gtggtcgttt tatgtatttt ttttagtggt ggatataaac 120
gttaatgtat ttgttattat ttaaggatat aaaaatatag aaggtggtat ttttcgtttt 180
tttttcgaa gattttttttt agagttgtta gatgaacgtt tacgttattt ttttttatta 240
aaggtggatt tagttaatag gagattggat cgcgattata gcgcgtttgt gtttagtttt 300
ggggaaagtt ttatgttagc gagtggtttt cggttcgttt cgtagatcgg ttaagtacgc 360
ggaggtcgcg cggttttatt attaatattt ttgaaggata atagtttatt aagttttatc 420
gaaagtttcg gattcgtcgc ggagtttggg ttggcggggg atgaggttga gtaatttttt 480
ttagaggtgg ttatgtggag aaggagtaat ttttttttt ttttttttt tttttcgtta 540
ttattcgcgg tttagagaat tgtcgtttgt cgttattgat acgtatagat agaatttaaa 600
gaaaggtaaa gagttttgtt cggtatcggc gtcgcgtggg ttaaatttgc gttcgtggag 660
gggcgcgtag agggtatcgg gcgtcgggag taggcggcgt agtattaggt gagttcgtta 720
tggcgatggt attattgttt agggtgtttc ggggtttgga aataaaacgc gtcgtcgggg 780
tagtgtaggg gtcggagaga attattttg ttttttttc gagtttttgc gggcgtattt 840
tcgatagtcg atttattttt tgtaaaaata aatttttaaa tttagttgtt taatttgttt 900
ttttgggcga taaagggggg gttgttaaag aagtggttaa atttttgcgg ttagcgaggg 960
gtcgggggcg tcgagggtat tgagagggga aggaggaggg atgcgaggtt tagtagcggg 1020
ttttaggcga ggttggattc ggaggaagcg gaggtttttt gggaatgcgg agtttcgggc 1080
gtaggagcgg gaggacggag gcgttcggtc gtcgatgggt acggggcgat gggtgggggg 1140
ttgcgtaggg ttcgagggag ttcggggagg gtcgttcggg ggtttttgaa ggtgttcggg 1200
tagggcgtcg gcggttttag gttgataatg gagggggcgc ggagtagtcg ggcggaggtt 1260
agggattcga gttcgggggc gggtcgcgtc ggggaaggtt aggtcgggga cgtcgggtgg 1320
cggggcgagg gttgggggtt gttgcgcgga tattcgggcg tcgcgggcgg gcgtgcgttt 1380
cggggtcggc gcggaagagg gcgcgaggtc gggtattttt tttcggtttt tttttcgggg 1440
cgttcggtgt cgacgcgcgt tgcgttttta tcgggaaatg gcggttcggt cgtggttcgt 1500
ttgggtagtt tggttttcgt tttgcgcggg aaaaaataat aataataaaa atatcgaaaa 1560
agggaataaa agtaaattgg ttgcggcgta ggaaagggtt ttgtcgggcg tcggacgtcg 1620
tgcgtatcgt tcgcggggcg cggcggttgg ggattagttt tgtttttttc ggtttttagt 1680
ttttagtttc ggcgtatttt agttttagtt cggttagttt ttcggatcgt tcgtgggtgt 1740
ttttttttcgt ttttttttgg gggggcgttt ttagtttttg ttttttttgt tttttataat 1800
aatggttaaa ggggtattgg tttttaagg gttaaacgag ttttttttag gagtttttcg 1860
tcgcgtgtag tggattatcg tgggtttgat tgaggtttgg tttttgttag ggttttttgg 1920
taacggtttt ggatgagtga aggatgaaa ggcgcgagaa gaaatgggtt gagaaattaa 1980
agtgcggggt tttgagtgtt tgaggggacg cgatgggaat cgtggtttcg ttgttttgat 2040
tttaagggta gttattttgt tatagttacg ttggaattga ggtgtttttt tagtttttgt 2100
ttattttaga ggaatttaaa ataagggttt agttttaagt tttatagatt ttaggatttt 2160
ttaggggtta ttagtttatc gcggtaaatg gttttttat tttgtttatt tgtgtttgtt 2220
ttgaaattta attgttattt tattgggttg tatagttgtt attgaaacgg tttaataggt 2280
gagagaaaaa gaaaatatta ttaagagcga gaacgtgttt tcgtgtgtgt tgttttgaat 2340
tataaattaa gttatatata atttttaatt cgttttaata tattttttat ttttaaatta 2400
aatgttaaga gttttaagat ttggaaatat attggtgatt tttagaattg ttttagttta 2460

aaattatata taaataaata gaggtattgt tcggcggaaa agtttatttt gagtcgtat 2519

<210> 160
<211> 2519
<212> DNA
<213> Artificial Sequence

<220>
<223> chemically treated genomic DNA (Homo sapiens)

<400> 160

atgcgattta agataaattt tttcgtcggg tagtgttttt atttatttat atgtaatttt 60
aggttaaaat aattttaaaa attattagta tgtttttaaa ttttggagtt tttaatattt 120
agtttgaaaa taaaaagtgt gttaaaacgg attgagaatt atgtgtagtt tagtttgtgg 180
tttaaaataa tatatacgaa gatacgtttt cgtttttaat aatgtttttt tttttttta 240
tttattgaat cgttttagta gtaattgtgt agtttagtga ggtgataatt ggattttaaa 300
ataaatataa atgagtaaag tgaggaaatt atttgtcgcg gtggattaat ggtttttagg 360
ggattttggg gtttgtgggg tttagaattg ggtttttgtt ttgaattttt ttgaggtgga 420
tagaagttgg aaggatattt tagttttagc gtggttgtga tagggtaatt gtttttaaaa 480
ttagggtagc ggagttacgg tttttatcgc gttttttttaa atatttaggg tttcgtattt 540
tggtttttta gtttattttt tttcgcgttt tttatttttt tatttattta gggtcgttgt 600
taaaaaattt tggtaaggat taggttttag ttaggtttac ggtggtttat tgtacgcgac 660
gggaaatttt tggaggaagt tcgtttggtt tttggggaat taatgttttt ttggttattg 720
ttgtaaggaa taaagagggt aagaattgag ggcgtttttt taaggaaagg cgggggagga 780
tatttacggg cgattcgggg ggttggtcgg gttggggttg aggtgcgtcg gggttggagg 840
ttgggagtcg ggggaggtag ggttggtttt taatcgtcgc gtttcgcgag cgatacgtac 900
ggcgttcggc gttcggtaga gttttttttt gcgtcgtagt tagtttattt ttattttttt 960
tttcgatatt tttattatta ttattttttt tcgcgtaaag cgggagttag attgtttagg 1020
cgagttacgg tcgggtcgtt atttttcggt gaaagcgtag cgcgcgtcgg tatcgggcgt 1080
ttcggaaagg gaatcgaagg ggagtgttcg gtttcgcgtt ttttttcgcg tcggtttcgg 1140
ggcgtacgtt cgttcgcggc gttcgggtgt tcgcgtagta atttttagtt ttcgtttcgt 1200
tattcggcgt tttcgatttg attttttttcg gcgcggttcg ttttcggatt cgggttttta 1260
attttcgttc ggttgtttcg cgtttttttt attgttagtt tggagtcgtc ggcgttttgt 1320
tcgggtattt ttagaggttt tcgagcggtt tttttcgggt tttttcgggt tttgcgtagt 1380
tttttattta tcgtttcgtg tttatcggcg gtcgggcgtt ttcgtttttt cgtttttgcg 1440
ttcgaaattt cgtattttta ggagattttc gtttttttcg ggtttagttt cgtttggggt 1500
tcgttgttga atttcgtatt tttttttttt ttttttttag tattttcgac gttttcggtt 1560
tttcgttggt cgtaggaatt tgattatttt tttagtagtt ttttttttat cgtttaagga 1620
agtaagttgg gtagttggat ttaagaattt gtttttgtag agggtgggtc ggttatcgag 1680
ggtgcgttcg taggggttcg ggggagaggt agaggtggtt tttttcggtt tttgtattgt 1740
ttcggcggcg cgttttgttt tttagatttcg gagtatttta aataataatg ttatcgttat 1800
ggcgggttta tttggtgttg cgtcgtttgt tttcggcgtt cggtgttttt tgcgcgtttt 1860
tttacgggcg taggtttggt ttacgcggcg tcggtgtcgg gtaggatttt ttgttttttt 1920
ttgggtttta tttgtgcgtg ttaatggcgg taagcggtag ttttttgggt cgcggatagt 1980
agcgggggga ggaggaggag ggagaaggga ttgttttttt tttatataat tatttttagg 2040
aggagttgtt tagttttatt tttcgttagt ttaggtttcg cgacgggttc ggggtttcg 2100
gtggggtttg atgagttatt gtttttttagg agtattaatg gtgaaatcgc gcggttttcg 2160
cgtatttggt cgatttgcga gacgagtcga gggttattcg ttaatatgag gtttttttta 2220
gaattgggta taaacgcgtt atggtcgcga tttagttttt tgttagttgg atttattttt 2280
ggtgggagaa ggtggcgtgg gcgtttattt ggtagttttg gggagagttt tcgaagggga 2340
aagcgaaaaa tgttattttt tatatttttg tgtttttaaa taataataag tatattaacg 2400
tttgtgttta ttattgggaa gagtgtatag ggcggttata gttacgagag ttgagaattt 2460
atattcgtgt ttgtaggttt tttagaaatt aagatcgatt tagaatttag cgttgtata 2519

<210> 161
<211> 9783
<212> DNA
<213> Artificial Sequence

<220>

<223> chemically treated genomic DNA (Homo sapiens)

<400> 161

```
ggttatatta gattttttt taatcgtttt tttgaatttt tagatgattt agagcggtcg      60
gttgattttg tcggtttatt ttatatttag ggaaggaatt cgttttggag atattaagag     120
atattgtatt tttaatttta aaattagagt gagggcggga ttcggtttcg ggattttagt     180
tttaaatttt aggatatttt tttttggttc gagtatgaag aaggaggatt tttaatttta     240
tttttttttt tttttttttt ttttatatat atatatatat atatatatat atatatatta     300
tatatatata tatatatata tatatatata tatatatata tatatatata tatatatata     360
tatatatata tagagttgga ttttttttta ttttttttat ttttaatata agcgggtttt     420
aggaatcgtt tagggcggcg tttagatttt gtcgagtagt ggtaggtttt aatggttata     480
tttcgttttt attttcgtta tatttttttt ttggtttgga ggaattttta ttttatttta     540
ttttaggttt tttgcggtcg tttattttgg gggttggggc gtttgagttg aagtttttttc    600
gtttttcgtt cggcggggta ggtttcggga agttgggagg agaagggaag gggtttcgat     660
tcggatttta ggaggttaga gtttttgtcg gtaggttcgg gtttttttcgt tgtagttcgg    720
ggaggggtcg acgcggttcg attatcgttt agttcggatg tcgatttcgt ttggagggaa     780
cggggtttac gtttaggtcg aagttttttt tcgtacgggt ttcgattttc gggttagcga     840
ggaagggaaa gtgttttttt ggggacgagt ttttaagacg ttttatcgtt cgtagtttcg     900
gtttagggta tttttcgttt cgtcgttttt agttgcgcgg tcggtgtcgg aggtttagat     960
tgattttagg gtttcgtttc gtttttttttt agcgtcgcgg cggggagggc gggttcggta   1020
gggggagggt cggtcggggg cggtttttgg ttcggttcgt ttcggtttcg cgtagggtcg    1080
tagcgcggcg gtttttttcg tagaaaaaaa attggggggtt aggggcgttt atttttttag   1140
gtcggggaga gttacgggga gatttttgga ttgcggagga gtcgggcgag cggggggagt    1200

acgattcggg tggatttaga ttttgtttag tttttaattt tttaggggag gatcggcgtc    1260
gggaaagtta agaggtgttc ggggtggggt taggaagggt cgttgttgtt tttttgtggt    1320
taagcggtta ttcgtttttg ttgtatttgg gatttcgcgg gaagcgggat tgggaaaggg    1380
tattcgtcgg gaggttttag ggttcggcgc gggtgtgttg gttgggggga gatgtttatt    1440
tttttgtttt taggatcgta tagtttaacg ttattttttta aatttgaaga tttttttagag  1500
tcgtttagat ttagattatc gtttttttttt ataattcgtt tttttttttg ggtttggaat   1560
ttttaggagt ttaattttta tttatttttt aaagggtgcg tttagaaatg tttttggatt    1620
ttagttgtga attttgtttt tatttttagg tttgatttgg cgtataggat atttatttat    1680
acgcgtattt tttttttttg taagggagtt gaggaggtgg gagtagaatg aggacgttta    1740
ttaatatttt attggtttgt gtttgggata ttatatagtt taattttttt aatatttatt    1800
atagattttt ggggaaaata tatttatttt tttttgttta tagagaagtt gaggtttaga    1860
gaaattaagt ggtttatata attgttttttg atttgtgata gtaagaattt atatagtcgg   1920
gtttgtttgg ttttaaattg gtggtttggt tttgttattt tacgggtttt tttagtttgt    1980
tttttttttc gttgtttagt agagtgagtg gtatataata ggttgttaat aaatatttga    2040
tgaatgaatg aatgttagga gttatataga ttttaaatat ttgaagtgta atttagatat    2100
ttgataaatg tgaaaatagg ttcgtagtag tttggtttta ggttttttaa ttgatttaat    2160
gaatattttt cgagggtgaa ttgagagtta tgttcggaag atttattttt gtttttaaat    2220
aatttagagt ttaaatgagg aatgtaattt taagatagtt aatatttatt gaatttatat    2280
tatatggatt tgggtttata tatattaatt ataagaattt tatgaggtag ttatttttat    2340
tttattgaga agtaaattga ggtttagaga ggttatagag ttagtgttgg gtttgatatt    2400
agaatttaaa tagttgaatt ttagagtttt tgtttttaat ttttattttt tattgatttt    2460
taatgtagat agttataata tcggaggtta ttgttgtagt gtgggtatgt gtgggttgtg    2520
cgatatttag ttttgaataa taggtgaagt ttttaagata aggtgatgtg tagattttttt   2580
taattttaat tttttttttga gataaggttt ttgttgttta agtgtgtagt ggtaagattt   2640
tagtttattg tagttttaat tttttaggtt ttaatgattt ttttatttta gttttttttag   2700
tagttgagat tataggcgtg tattattata tttaattttt gtatttttttg tatagatgag   2760
gttttgttat attgtttagg ttggtgaggg gttttttttta attattttgt ttatttttat   2820
ttttgttttt tttaatttat tttgggttta atttgttgtt tttttttttag ttttttaagg   2880
tggaaataga ttattgattt gagattttta tttttttttta ttataagtaa tatttttaat   2940
ttttttggat gtttaattgt tttttatagg tatttattat tatatttagt taattttttta   3000
ttttttttgta gagatggggt tttattattg tttagggtgg ttttaaattt ttgggtttag   3060
tagttttttt atttttgttt tttaaagtgt tgggattata ggcgtgaatt atcgtgttta    3120
gtagtagatt ttgaataagt tggagttatt aaaagtttat agttatgtaa attttaggaa    3180
gttggtggtg gtggtaaaaa atgaagttgg agaaggaagt agtggttgga ttatgaaggt    3240
ttttatatgt tatgtatagg agttgagtgt tgattttgtt ttaggcgtaa taaggagtta    3300
ttaaatttag gaggttaata tagtaaaagg tggtggtgat tgaaggcggg gagagatgga    3360
```

gataagtaga gaaatatttt taaaggttta tagttaataa tatttagtga ttaattattt 3420
tgatgagaaa gaaatgaaag ttaagggtga ttaattttaa gatttttaat tggagtagta 3480
gagttggtgt ttattgaggg gatatattag gagaaggaat atgttatgga gggtagtcgt 3540
gaatttagtt tgaggtattt gaattgaagt gttagtaggt tatttaggag atattcggga 3600
gatgattggt atgtgtgggt ttaaagttta ggaagggttt gggtagatgg gtgaggttat 3660
agaagtggat gatgatattt atagtaaatg cgtggagtgg aaagagtggg ggtggaataa 3720
agaatttggg ggatattata tttttagaat tgttttggtg ttttttttat tattttatag 3780
agttttttttt ttattttttta gtttttagtt aaagtaaata tagatatata tttttattta 3840
tttcgtttat ttgttaatag ttgtaatttt ggatatagcg gatatttttt ttttagttaa 3900
gttgttatta aagaagggtt agggagataa ttttagaatt tgggttgtga gaggagtaag 3960
tatggagagg ttagtgggag ggatggtgtt tagtttttta ggagtaattg tttggttttt 4020
ttagggggat gacgaagata gagatgttat tttcggaatt tagtttgttg ttggggagac 4080
gttagttacg gtttcggggg gtatttcggg tttttaggat tagagtttgg gttagagttg 4140
tatatttgtt aggagtatat atgtatattt atgtattaat atgtatgtgg gaagatagat 4200
atataggttt atatgtgggg ttttaagtat tgtttttttt tgattttttt tttattaatg 4260
ttattttttt attattatgt tatgtagttt ttatttgttg tggttattag gtttataggt 4320
cgatagtatt ttgtttatag tggtagttat tttatagtta gtagtgatat tttataggtt 4380
atttgttttt aggattagta tattatttgg gtagtgttta tattgtgtta ggttatatat 4440
tcgtatttgg tgggagaaaa gggtagagag gattgaggtg tggggtttgt tggataggga 4500
gtttggagat aaagggattg tagatgaaag ttggtttgag atttttagag aaggtaaggt 4560
gtggtttaag ggggtttattt tagggaagta ggagagaaag ggtttgatgg gtagggtgga 4620
attgtagatt ttaaggttgt ggttttttaa gtttcgggtt attttaatgg tggttattat 4680
tcgagtttga aagaaatgag gagggagtgt tattgtagtt ttttgttaga ttttttgaaa 4740
ttaaagattt tttttatttt tatttttagg tagaattttt atttttttag ataagttgtg 4800
ataggttttt tttttttttt ttatttttat tatggagttt atttttttgt ttttttttata 4860
gattagagga aatttgagat tttttagttc gatttttttg taggttttgg ggagggggga 4920
gtagaggagg tattatttag gtattaaagt tttttttttt taggagttgt atatttttat 4980
ataaattttt tagaagggtt atattttttt aatttttagt ttttagtttt tattttgttg 5040
cgggcgaggg attggttgtg ttgttatggg aacgggtttt agttttgagg gaagtgttat 5100
tagtcggtta tgttttggtt tcggtataat attttttgtt ttagtttttt gggtagaatt 5160
ttgcgggggga atttaaggtg ggtgaattta ttgtttagta gttttggttt taggaagttt 5220
tggagttggg gatgattaga atagagagtt agggggtatt atgtttatag tatacggtgg 5280
tatatagatg acgttgatta tagtagattt ttaaatagag tttattatgt gttatacgtt 5340
attttaatat tagtaaatat taatttattt atttttttata atagttttta aggtaggaat 5400
tattgatgta tagagaggtt aagggaggga attgtttcgg ttatataatt agtaaagtat 5460
ttagattagg atgtatttcg ggtttttagt tttatagtta tatgcgttta ggtattaagt 5520
cggattgttt tttaggggtt tttgttgttt tttttttttta ttttagtttt tatttttttgt 5580
tttagttggg gttatagtgg gagtattgga ttagtttagt gttgtagttt ttttgttgta 5640
atttttttatc ggtttagttt cgtattttat agtttatata gcggtttttt tagtaaagtg 5700
ttttttgtgt ttgtggtggg ggtgtgagag tttttagggt tatggggtgt agggaggtat 5760
aagattcgat aaagaatggg ggtagggaat ttggttttta tttataagta gttgaagacg 5820
ttggcgttta gttttcgggg taaattttcg ggatattgga atgattttat tatttcggat 5880
aatgatggtt ttgtttaaag aaagaaaagg aattgggatt tggttttttt tagatttatt 5940
tatgaaaaat tttagaaaga ttaggttttt tttataatga ttttttagat tttttattta 6000
gatttttata tttgtttttg attttattta atggatattt taggttattt tttatgtttg 6060
ttattttttt tgattttttt taattttgat ttttaatttt tgttatttta tttttttgta 6120
tttttttaat ttagagagtg tggtttttttg tcgttatttt ggagtttttt tttttatatt 6180
tgttatcgtt tgtattggtt acgtatattt tgtttagtag gtagattata attagtgtat 6240
agtagttttt ttttatttgg tggttacgtc gttttcgggt tatttgttta ttttgttata 6300
taaggaaggg ttagaggtgg gtagaaggag aggtttttatt atatagttat ttttttttta 6360
ttttttttat tcgtagaaat taattggttt agttaggtta tattggaaga taggagggaa 6420
gattggggtg taattgtttt tttttttttt tttttgaatt aagttttgtt taggttttta 6480
aaggtgttgt gggtttttag ggtaattttt tttggttttt atatagagtt ggttagagtg 6540
gttgtttagt aggtttttgg tttaggttat ttatttatta tgagttggaa ggtattttta 6600
acggttttta ttattaggtt ttcgtggttt attttttttt gtgaagatta gtaggattga 6660
gggttaagta agtgagaggg atcggaggaa tttggggttt tcggagtggt tgggaggtag 6720
aggggtggtg gcgaagggtt ttgaagtatt tttattaggt tttttagttg ttttcggata 6780
tggcgatgta ggagtcgtga ggttatttta gtagtttcgt tttttgtatg tttattaaat 6840
aggttttagt agtagaagta gagtttttgg tggaggaaaa gttggaggtg agttttgttt 6900
gtgtgtgtgt gtgtgtgtgt gtgtgtgtgt gttatgtaag gtgaataata agattttggg 6960
tatttatata aatatttgag aggttagtgt ttgttattga aggtaaatta ggtttattta 7020

tataaggttt cgtatgtgtt aagagaaagg aaaggtttaa aaatttaaat ttagaggata 7080
atagatttaa aatttagtat tggtaattaa attatttata aatatgtgag tttttatatt 7140
ttaggtggga aagtaatatt tatgtttata tggtagaatt taattgatga atattaggta 7200
aaggttttgt gttagaaggt agatagttag tgagtttatt aggtttttcg tatggtttag 7260
atttaaaatt agaggtttga gtttttgggg agaggtgtta ttgtgttttt gggaaagggg 7320
atgggtttta tttggtttcg gttaggtttt ttaggtattt ttgtaatatt ttttcgattt 7380
tttatatata ttattttata gtaagtttgg tttttattgt gtcgattttt gttagtattg 7440
atgattttgt taggttagaa tgattatatt aggttggata ttagattagg ttttggaata 7500
atttttattt tagatatata tcgttttttg cgttcgttgg ttaagttgag aaaggttata 7560
gttatttggg atttaggtta gagggaggag gagaagggag ggttggtaag gtgtaatgag 7620
tattgtttgg gtggttagtt ttattatttt atagaaaggt tattaggatt ttttggttaa 7680
tcgttatgtt ttttgtttgt tttatttttt ttatttttgg taaaaaaaaa agaaaaaaga 7740
aaaaggtatt tttagtggtg tttttggggg aggggttagt tggtagtaga agttttattt 7800
ttttgttagt tagattttat attttatttt taattattat taattttttt agtgttttgt 7860
ttttagtatt ttatttttat gggtatattt atttttattt taaaattatt tagcgaaagt 7920
ttttgttgtt ttaataatga ggaagttttt tagtttagat cgggaaagag gaattgaaat 7980
agagtagaag tgaaagttta tttggattgg gtaggagtta tatatttata aatttttttt 8040
atataggagg ttaagggagg tggtggtggg tttatgggtt attagttatt ttttttagcg 8100
aattgagcgt ttttattttt ttcggtgttt tgcgttttcg gtagatgtat ttaaaatata 8160
tatagttcgg gggttagtcg gtcgtttcgg gtaggggggtt cgttttttttg ttaggttagt 8220
acgagttggt aggttgaagg gtatttaaat gggatttttt tttatttcgg gcgttttttt 8280
tttcgttttt ggggaagttg gcggtttttt ggtgtcgcgg gttatttttg ggtgtggagg 8340
gggaggggaa ttacggtaag agggataggg aattggggcg ttggagtcgg gaagcgggtg 8400
agcgagtttt tttaatcgtt atgtttttta ttaaaaagaa tttcgttttt tcgtgaaatt 8460
tttttttttt tagtttttaa gttgtcgggt cgcgacgggg aattggttaa tttatttttc 8520
gtcgggtttt tcgtttttcg ggttttgggg tagaataggg ttttgaggcg gcgtgggcga 8580
gggtagttaa tgagcgggaa ggacgtgaag aaggtgatat aggttgtttt ttattggaaa 8640
atgggttggg agggggtgcg gttcgaagat tagaggatag taaaggttta cggaaaaggg 8700
agtttttggt tcggacgcgg cgtggggaac gcgttttagc gtcggggatg cggagttggg 8760
gagaggggtg ggagttttat tttggtttgg gtgttttagg gggcgtttat tcggcgtagt 8820
ttgtgtttta gggtaggcgg cggttcgtgt tcggggggtt ttgtatagtt cggttcgcgt 8880
ggttatcggc gcgtcgtagt ttttcggggt ttagttgtaa aaaggtcggt ttggagaagt 8940
tcgttcgagt tttaatagtt ttcgcgggcg tcgcgttttc gtttttaatt ttcgcgggag 9000
ggtttttggg cgtttttgga gcggcggcgg gcggcgtcga ggcggcgttg ggtaggttcg 9060
gggatttggg gcgcggaggc ggagcgtttt cggagtttat taggtgcgtt acggtcgagc 9120
gtattcggtt tagtatgttg tttttttgtt tcgttttcgg tcgcggtttc gttttcgttt 9180
aggtttcgtt tttatggttt cgcgtgggcg gtgttcgcgt tagtcggttt tttttttgtta 9240
ttggttttcg gttagttggg cgggtttcgg gcgaaatttg tttttgtttt gggttatttt 9300
aggaatcggt ttacgggttt tcggtttttg tttcgtttta tttcggtttg gtttcgtttt 9360
cgttttttag ggtaggtttt tgttgttgtg agaagggggc ggagagaagg aaattttatt 9420
ttagtcgtgc gttagattag atatagttag aatgttttta ttgtgacggg ggttgaggga 9480
taagggggtg cggtttttttc gttttcggtt cggtgtggtt agttagttta gagagttatg 9540
gagttaatga ttttagagta aaataggcgt ttgggagtcg ttgggtatga ggcgttaggt 9600
gtgatattat ttttggatgg ggttgttgga gcgagagcgg gtaagttggt atcgggtggg 9660
gttgcgggat taatgacgta ggttgtagtc gattatttcg gaattaggag tcggggtaga 9720
tttttgggtt tttgatgcgt tgtttttcgt gaagattatt tttagatttg ggagagcggt 9780
tgt 9783

<210> 162
<211> 9783
<212> DNA
<213> Artificial Sequence

<220>
<223> chemically treated genomic DNA (Homo sapiens)

<400> 162

gtaatcgttt ttttagattt agaggtggtt tttacgggga atagcgtatt aggagtttag 60
gaatttattt cggtttttga tttcggggtg gtcggttgta gtttgcgtta ttaatttcgt 120
aattttattc ggtattagtt tgttcgtttt cgttttagta gttttattta gaggtggtgt 180

```
tatatttggc gttttatatt tagcggtttt taagcgtttg ttttgttttg aggttattag     240
ttttatggtt ttttgggttg gttggttata tcgaatcgag agcgaggaga tcgtattttt     300
ttgtttttta gttttcgtta taatggagat attttagttg tgtttaattt agcgtacggt     360
tgaaataaag tttttttttt ttcgtttttt ttttatagta atagaattta tttttaagaa     420
acggaggcgg ggttaagtcg gaatggggcg gggtagaggt cggggattcg tgggtcggtt     480
tttggggtgg tttagagtaa gggtaagttt cgttcgggat tcgtttagtt ggtcgggagt     540
tagtagtagg gaagggtcgg ttggcgcgag tatcgtttac gcggagttat gggggcgggg     600
tttgggcggg ggcggggtcg cggtcgaggg cggggtaggg aggtagtatg ttaaatcggg     660
tgcgttcggt cgtggcgtat ttggtgagtt tcgggggcgt ttcgttttcg cgttttaaat     720
tttcggattt gtttaacgtc gtttcggcgt cgttcgtcgt cgttttagaa gcgtttagga     780
gttttttcgc gaaggttggg agcgggagcg cgacgttcgc gaaggttgtt gaggttcgag     840
cgagtttttt tagatcgatt tttttgtagt tgagtttcgg ggggttgcga cgcgtcgatg     900
attacgcggg tcgggttgtg taaagttttt cggatacggg tcgtcgtttg ttttggagta     960
taggttacgt cgagtgagcg ttttttgggg tatttaaatt aggatggggt ttttattttt    1020
tttttttagtt tcgtattttc ggcgttagga cgcgtttttt acgtcgcgtt cgggttagga    1080
gttttttttt tcgtggattt ttgttatttt ttggttttcg ggtcgtattt ttttttaatt    1140
tattttttag tgggggggtag tttgtgttat tttttttacg ttttttttcgt ttattgattg    1200
ttttcgttta cgtcgtttta ggattttgtt ttgttttaga gttcggaggg cggagagttc    1260
ggcgaaggat gagttggtta gtttttcgtc gcggttcggt agtttaaagg ttaaggggaaa    1320
aggggtttta cgaaggagcg gggttttttt taataggggga tatagcggtt gggaagattc    1380
gtttattcgt ttttcggttt tagcgtttta gtttttttgtt ttttttatcg tagttttttt    1440
tttttttttat atttagaaat agttcgcgat attaggaggt cgttagtttt tttaggagcg    1500
gggagggggga cgttcggggt agaggagggt tttatttaga tgttttttag tttgttaatt    1560
cgtgttggtt tggtaaagaa gcggattttt tgttcggagc ggtcggttgg ttttcgggtt    1620
gtgtgtattt taaatgtatt tgtcgggaac gtagagtatc gagggagatg ggggcgttta    1680
gttcgttgag gaaggtggtt ggtggtttat ggatttatta ttatttttttt tagtttttttg    1740
tgtgggagga gtttatgggt atgtggtttt tgtttagttt aggtgggttt ttatttttat    1800
tttattttag ttttttttttt tcgatttggg ttggagagtt tttttattgt taaggtagta    1860
gaaattttcg ttggatggtt ttaggataag ggtgggtgtg tttatgagga tgaggtgttg    1920
gggataaggt attgggagag ttgatagtgg ttgagggtgg gatgtagggt ttagttagta    1980
gggaagtaaa atttttgtta ttaattagtt tttttttttag aagtattatt aggaatgttt    2040
tttttttttt tttttttttt ttgttaggga tgggggaagt gaggtaggta ggggatatag    2100
cggttggtta gaaggttttg gtggtttttt tgtgaagtgg tagggttggt tatttaaata    2160
gtatttatta tattttgtta gttttttttt tttttttttt ttttggtttg gattttaggt    2220
ggttgtgatt ttttttagtt tggttagcgg gcgtaggaga cggtgtgtgt ttaaggtggg    2280
ggttatttta ggatttggtt tggtgtttaa tttgatgtgg ttattttggt ttggtagggt    2340
tattaatgtt ggtaagagtc ggtataatga ggattaggtt tgttgtgaag tggtgtatgt    2400
ggaaggtcgg aggagtgtta taggagtatt tagggagttt agtcgaggtt aggtaagatt    2460
tatttttttt tttagagata tagtgatatt tttttttagg gatttaggtt tttggtttta    2520
aatttgagtt atgcggaaga tttgatgaat ttattggtta tttatttttt ggtatagaat    2580
ttttatttgg tatttattag ttagatttta ttatgtaagt atgaatgtta ttttttttatt    2640
taaaatgtag aggtttatat atttatggat gatttaattg ttagtgttga gttttggatt    2700
tgttattttt tgagtttggg tttttgagtt tttttttttt tttgatatat acgaagtttt    2760
atgtgaataa atttaatttg tttttagtag taaatattga ttttttagat gtttgtatgg    2820
atgtttagag ttttgttatt tattttgtat aatatatata tatatatata tatatatata    2880
tataaataaa atttattttt aatttttttt ttattaggga ttttgttttt attattgggg    2940
tttatttgat gggtatgtag ggggcggagt tgttgaaatg gttttacggt ttttgtatcg    3000
ttatattcga gagtagttaa aggatttggt agagatattt taggattttt cgttattatt    3060
tttttgtttt ttaattattt cggggatttt agatttttttc gattttttttt atttgtttgg    3120
tttttagttt tgttggtttt tatagaagga agtgagttac gagagtttgg tagtgggggt    3180
cgttgagaat gtttttttagt ttatggtgag tgggtgattt gggttaagag tttgttaggt    3240
aattattttg gttaatttttg tgtggaagtt agagggggtt attttgagaa tttataatat    3300
ttttgagagt ttgagtagag tttggtttag aggggaggga gaagggggtag ttatatttta    3360
gtttttttttt ttgtttttta gtgtagtttg gttgggttag ttgattttta cgggtggagg    3420
gggtaggggg agggtgattg tgtgatggaa tttttttttt tatttatttt tgattttttt    3480
ttatgtggta ggatgagtag atggttcggg agcggcgtgg ttattaagtg gagggggggtt    3540
gttgtgtatt ggttgtgatt tatttgttag gtaaggtgta cgtggttaat gtaggcgata    3600
gtaggtatgg gggagggggt tttaaggtgg cgatagaggg ttatattttt tgagttgggg    3660
gaatatagga gagtaaggtg gtaggggtta aaaattaaga ttggagggag ttaagagggg    3720
tgatagatat aaggagtggt ttgaaatatt tattgggtgg ggttaggggt agatatgggg    3780
gtttgggtaa gagatttgaa aggttattat ggagagggtt tggttttttt ggaatttttt    3840
```

atgagtgggt ttgaagagaa ttaggtttta attttttttt tttttttggg tagggttatt 3900
attgttcgga atggtgaaat tattttaatg tttcgggagt ttatttcgga gattgagcgt 3960
tagcgttttt agttgtttgt aagtaggaat taaatttttt atttttattt tttgtcgggt 4020
tttgtgtttt tttgtatttt atggttttga gaatttttat atttttatta taggtataga 4080
aagtatttta ttgggaaggt cgttgtgtgg attatggagt gcggggttga gtcggtggga 4140
ggttgtagta gggaggttgt agtattgggt tggtttagtg tttttattgt ggttttagtt 4200
gaggtaggaa gtagagattg aggtggggga aggaaatagt aaaggttttt gaaaggtagt 4260
tcggtttagt gtttaaacgt atgtggttgt ggagttggaa gttcgggatg tattttggtt 4320
tgagtgtttt gttaattgtg tgatcggagt agtttttttt tttaattttt ttgtgtatta 4380
atagtttttg ttttaagggt tgttgtgagg agtaaatgag ttaatatttg ttaatattag 4440
aataacgtgt ggtatatggt aaattttgtt taagggtttg ttgtagttaa cgttatttgt 4500
gtgttatcgt gtgttgtgag tataatgttt tttggttttt tgttttgatt atttttaatt 4560
ttagggtttt ttgaaattag agttgttagg tagtgaattt atttattttg agttttttcg 4620
tagagttttg tttaaggagt tggggtagag gatgttgtat cgggattaga atatgatcgg 4680
ttggtaatat tttttttaga gttggggttc gtttttatgg taatataatt agtttttcgt 4740
tcgtagtaag gtggaagttg gaggttggga gttgggagga tgtggttttt ttaaggggtt 4800
tgtgtgaggg tatgtagttt ttaggggaga ggggttttga tgtttgggtg atgttttttt 4860
tatttttttt tttttagggt ttataaaaag atcgagttgg aggattttag gttttttttg 4920
gtttgtgggg agggtaaaaa ggtaaatttt atggtagagg tgggagaggg gaggaggatt 4980
tattataatt tgtttaggga ggtggaggtt ttatttgagg gtaggggtgg aagggatttt 5040
tggttttaga gaatttagta gaggattata gtggtatttt tttttatttt tttttaggtt 5100
cgggtgatgg ttattattgg ggtgattcga ggtttgggag attatagttt taaggtttgt 5160
agttttattt tgtttattaa gttttttttt ttttgttttt ttgaggtgag tttttaaat 5220
tatattttgt tttttttggg agttttaggt tagtttttat ttgtagtttt tttattttta 5280
agtttttgt ttagtaaatt ttatatttta attttttttg tttttttttt ttattaggta 5340
cgagtgtatg atttgatata atatgagtat tgtttagatg atgtgttagt tttgggaata 5400
gatggtttgt gggatgttat tattgattgt gaggtagttg ttattgtgga tagggtgttg 5460
tcggtttatg agtttaatga ttatagtagg taggggttgt atggtatggt ggtaagggga 5520
tggtattggt gaaagaaggg ttaaagggaa gtaatgtttg ggatttttata tgtgggtttg 5580
tgtatttgtt tttttatata tatgttggta tatgaatgtg tatgtgtgtt tttggtaggt 5640
atatagtttt ggtttaagtt ttggttttgg gggttcgggg tatttttcga gatcgtggtt 5700
ggcgtttttt taataataag ttgggtttcg gggatgatat ttttgtttc gttatttttt 5760
tgggagggtt aggtagttat ttttgagggg ttgaatatta ttttttttat tagtttttt 5820
atatttattt tttttatagt ttaaattttg aagttgtttt tttgattttt ttttagtggt 5880
aatttaattg aagaagggat gttcgttata tttaaaatta tagttattgg taaataaacg 5940
agatggataa aggtgtgtgt ttgtatttgt tttgattaga aattgaaaga taagaagaaa 6000
gttttgtggg gtggtggaaa gagtattaga atagttttag aaatgtggtg ttttttaggt 6060
tttttatttt atttttattt tttttatttt acgtatttgt tgtgagtgtt attatttatt 6120
tttgtggttt tatttatttg tttagatttt ttttgagttt tagatttata tatattagtt 6180
atttttcgaa tgttttttgg atagtttgtt ggtattttaa tttaaatatt ttaaattaaa 6240
tttacgattg tttttttatag tatgtttttt tttttggtat attttttttaa tgggtattag 6300
ttttgttgtt ttagttagaa attttggagt tggttatttt tggtttttat ttttttttta 6360
ttaaggtaat tgattattaa gtattgttga ttatagattt ttaaaagtat tttttttattt 6420
atttttattt tttttcgttt ttagttatta ttattttttta ttatattggt tttttaaatt 6480
tagtggtttt ttattgcgtt tagaataaaa ttaatattta atttttgtgt atggtatata 6540
aggattttta tgatttagtt attgtttttt tttttagttt tatttttttat tattattatt 6600
aatttttttgg ggtttgtata attgtgggtt tttagtagtt ttaatttatt taaaatttat 6660
tgttggatac ggtggtttac gtttgtaatt ttaatatttt gggaggtaga ggtgagagga 6720
ttgttaagtt taggagttta agattatttt gggtaatagt gagattttat ttttataaaa 6780
aaataaaaaa ttagttgaat gtagtggtga gtgtttgtgg gaagtagtta agtatttaag 6840
gaaattaaga gtattgttta tagtagaaaa gaataaagat tttaaattag tgatttgttt 6900
ttattttaag aggttagaaa aagaatagta aattaaattt aaagtaagtt aagggaagta 6960
aaaataaaga tgagtaaaat aattagaaaa ggttttttat tagtttgggt aatatagtaa 7020
gattttattt gtataaaaaa tataaaaatt aggtgtgatg gtatacgttt gtagttttag 7080
ttgttgggga agttgaagtg ggaaaattat tggagtttgg aaagttgagg ttgtagtgag 7140
ttgagatttt gttattgtat atttaggtaa tagagatttt gttttaaaaa aaaattaaaa 7200
ttaaaaaaat ttatatatta ttttattttg agaattttat ttgttgttta ggattgggta 7260
tcgtatagtt tatatatgtt tatattatag taatgatttt cggtattgta attgtttata 7320
ttaggagtta gtagggagta ggaattaaga atagggggttt tagagtttag ttgtttgggt 7380
tttagtgtta gatttagtat tagttttgta atttttttga attttaattt gttttttagt 7440
aaaatggaga tagttatttt atgggatttt tgtagttaat gtatgtggat ttaggtttat 7500

```
gtaatatgga tttaataaat gttagttatt ttgaaattgt atttttttatt tagattttaa    7560
gttgtttgaa ggtagaaatg gatttttcga atatagtttt tagtttattt tcgagaaatg    7620
tttattgagt tagttaagag gtttggggtt aaattgttgc ggatttgttt ttatatttgt    7680
taaatgtttg gattgtattt tagatattta aagtttgtat gatttttaat atttatttat    7740
ttattaaata tttgttgata atttattatg tgttatttat tttgttagat aacgggggaa    7800
gggataggtt ggaggagttc gtgaggtagt agggttagat tattagtttg gagttagata    7860
ggttcggttg tgtgaatttt tgttgttata agttaaaaat agttgtgtaa gttatttgat    7920
tttttttgaat tttagttttt ttatgggtaa aaggggatgg gtatattttt tttagagatt    7980
tgtgatgaat attgaaaggg ttaagttgtg tagtgtttta agtataagtt aatgaaatgt    8040
tggtggacgt ttttatttta tttttatttt tttagttttt ttatagggaa aggagatacg    8100
cgtgtaggta aatattttat gcgttaagtt agatttagga gtgggaatag aatttatagt    8160
tgggatttag aagtattttt aggcgtattt tttgaggagt aggtaggaat tggatttttg    8220
gaaattttag gtttagggaa gaaagcgaat tgtgaggagg aacggtggtt tgggtttgaa    8280
cggttttgag aggtttttaa gtttggggga tgacgttggg ttgtacgatt ttaggaataa    8340
gagggtggat attttttttt aattaatata ttcgcgtcgg gttttgggat ttttcggcgg    8400
atattttttt ttagtttcgt ttttcgcgag gttttaggtg tagtaggagc gggtggtcgt    8460
ttggttatag gagggtagta gcggtttttt ttgattttat ttcgagtatt ttttaatttt    8520
ttcgacgtcg gttttttttt aggggattga aggttgggta gagtttgagt ttattcgggt    8580
cgtgtttttt tcgttcgttc ggttttttcg tagtttagga attttttcgt ggtttttttc    8640
gatttggagg ggtggacgtt tttggttttt agtttttttt ttgcggaggg ggtcgtcgcg    8700
ttgcggtttt gcgcggggtc ggggcgggtc gagttaaggg tcgttttcgg tcgatttttt    8760
ttttgtcggg ttcgtttttt tcgtcgcggc gttggaggag ggcggggcgg ggttttgggg    8820
ttagtttgag ttttcggtat cggtcgcgta gttggaggcg gcggagcgga aggtattttg    8880
ggtcggggtt gcgggcggtg gggcgttttg ggaattcgtt tttaggagag tatttttttt    8940
ttttcgttgg ttcggagatc ggggttcgtg cgaggagggg tttcggtttg ggcgtggatt    9000
tcgttttttt taagcggagt cggtattcgg gttgggcggt ggtcggatcg cgtcggtttt    9060
ttttcgggtt gtagcgaggg ggttcggatt tgtcggtagg ggttttggtt ttttgaggtt    9120
cgagtcggag tttttttttt ttttttttta gtttttcgga atttgtttcg tcgggcgagg    9180
ggcgagggaa ttttaattta gacgttttag tttttagggt gggcggtcgt aggggattta    9240
gagtgggatg gggtgggaat ttttttaggt taggagggag gtatggcggg aatagggggcg    9300
ggatgtgatt attggggttt gttattgttc ggtagggttt ggacgtcgtt ttggacggtt    9360
tttggagttc gtttgtgttg aggataaggg agatggggaa ggatttaatt ttgtgtgtgt    9420
gtgtgtgtgt gtgtgtgtgt gtgtgtgtgt gtgtgtgtgt gtgtgtgtgt gtgtgtgtgt    9480
gtgtggtgtg tgtgtgtgtg tgtgtgtgtg tgtgtgtgta gagagagaga gagagagaga    9540
aaatgagatt gagaattttt tttttttata ttcgaattaa gaaagagtat tttggggttt    9600
gggattggga tttcgaggtc gagtttcgtt tttattttgg ttttaaagtt gagaatgtag    9660
tatttttttgg tatttttaga acggattttt tttttaggtg tagggtgggt cggtaaagtt    9720
aatcggtcgt tttggattat ttgggaattt aagagagcga ttggagagag gtttgatgta    9780
gtt                                                                  9783
```

<210> 163
<211> 13537
<212> DNA
<213> Artificial Sequence

<220>
<223> chemically treated genomic DNA (Homo sapiens)

<400> 163

```
tgtataatta tcgttaattc ggtttttagt agaggtttgt gggttaggga gtgtatcgat      60
ttgtattgtt aaaggaattt tttgtttttt ttagtgttag ttgaagagtt ttttgtagtt     120
atttttttat tttgagagaa gagaaataag aggagacgaa tttttggttt ttttttacgg     180
ggtgtataga ttagtgataa tgttagtttt ataatttgga tttatttgtt tgttttttaa     240
ttatcgttat tttttaagt ataaagtttg tgtttataat tagatatttt ttatatgtta      300
tatcggtaag tttattttat tattttatag agggatggtg atttttttta gcgttttata     360
ttgatcgcgg tttttattt gttgattgat tgatttataa atttgttttt tatttatttg      420
tttgtttatt gaaggtaaat tcgttatcgg gaatatagta gtttttaat aattatttaa      480
tgtatggtta ttttttttag gatttagaat attaaaggga cgtgtttttt tttttaaat      540
tatttgtttg cgtttttttt agtcgggtgt tttagttttt atttcggttc ggttattatt     600
ttatttcgtt ttttcgcggt ttttaggttt cgggttggga ttttagtttt tgtttgtagg     660
```

```
tgcggtttag agggtgcgat acgcgtcgga gcggggtagc ggaggagggg atagggacgc     720
ggtagttata ttcgtatttt tttttttttt cgggtatttt cgagggtttt tttgttttta     780
gtttttgag ttttcgaagt tttttatgcg gtaagagcga gtcgtaggaa taagtgggga     840
attttggtcg cggttttttt tttcgtttta taaaaaagaa attgattttt gtttggagtt     900
tgtgaaaagt ggggttcggg gtttagttaa tggggcgttt cgcggcgcgg gttgagtgga     960
gttagcgcga atcgtttagt cgcggtttta attaattcgt tttttgtgcg gttcgttcgg    1020
tcgttttcgt cgtagtcgta ttagcggttt attgttcggt ttcgtcgggt cgcgggattt    1080
attttttta aatagtcggt tttgtttagg gtagttcgag cggaagtttt ttattgataa    1140
tttgttttaa atagatagat ttgttagaag cgattttcgg gaaggaagta aaaagttatc    1200
ggttcgaagg ttgggtttaa aatagggatt ttgcgtttta ttcgcggtcg cgtcgttttt    1260
tcgcgttttc ggttttgtag tttcgaagtt tcgcgggggt ttagattatt gtatttgttg    1320
agttagatat ttcgcggatt tgattttcgt tttatttatt tgtaaaatgg agtagttggg    1380
cgttggtaag tcgtaagttt aagagttttg gttttattag cgtgtacgac gtttattttt    1440
tttttttttt tttatatttt ttttaaaaat tgtaagttag aaaatttggt taggttttcg    1500
cgacggcgag aaacggtttt gaaggtcggt cggtcgtcgt aatgcggttc ggatcgtgta    1560
cgtgttgttt cgttattgtt tttcggtttt tttttaataa agatgttaat cgtgttaaat    1620
tataggtggt gtttttgtaa gattagttat tgttttttta taagttaaga tataaaacgt    1680
tttgtattga atggtttatg atttggagcg tttaatttgg gagttgagtt gttagatttt    1740
ttttttttta tttattagag ttattttttt tttaaatgat tttatttaat aagtaaaatt    1800
taaatggggt ggggggggat ttaatatgtt gtggatgaaa agaatagtta ttgatttttt    1860
tatatttaat ggtatgtagg agtttttaa aatatagttt tatattaaat agatttgtga    1920
attttaagta gaattgaatt gtattaatta gttgatgggt ttgtttattt tttattatta    1980
gatgagaaat ataaaggttg ggaatttata gcgtttaatt ttattgtatt taagtaattt    2040
tttttttag tttagaaaat tttttattag ttgtatatga gagaaaaatt gaagtaaaaa    2100
aaaaaaaatt tgttttttgt ttatttatta ggtgaatata tattttagta ttatatagag    2160
gaaattttag gtggttttta gtaagatttt agtggttaag agttataatt tagaaaagat    2220
gataatatat atatagtgtt aatagtattt aaataaatat tttagataat agaataatgg    2280
ttattaatta tgatgttttg ggtttttgat taattttta tgtttgtttg tagttaaata    2340
gttgtaggat tttttttttt ttaaaaattg tttgtgtatt attttaaaaa ttgtatttaa    2400
tagttatatt ttttagagta gtttttttgt gtggaattga agtgaatggt ttagtaatag    2460
cgatgaagtt agtaagtagt aagaatgttt tttatgttaa aagaatttta attattttgt    2520
tagaaatggg ttatattttt attttttgaa atgtagaagt tgtttgttat tgtatgattt    2580
ttagtatggt ttatatgttt aaataaatgg aattatttaa ttttgtttat atttgtatta    2640
gtgtttttaa aattgattaa gttgtgtaat tgtattaaag tgtatttttt ttggggtttg    2700
aaaatattaa agaaattaag agagggtaag attattttgt aaaattgttt ttttattttt    2760
ttagataaat attatatttt aaaaattatt tggtgattta aagattaaaa tgaatgtatt    2820
tttgtatatt ttaaatatgt aattttaatg attatttagt tattgtagtt ggaaatatat    2880
atatttaggg atatagatat gtataattat gaatatttgg gtaattatat attgtaatta    2940
attattaatg tatggattaa aattaaatta tttttattgt ttttattgtt atttagtatt    3000
taataagtat ttatttggta ttaaaagatg gttaaaatat attaaataat gttagttttt    3060
taaaaaggta ttaaaatatt tatattttag atagatatat atatgttgta tgtagtgtat    3120
gttggtgttt taaatatttt attatttaat gttttgattt gatagaaatg ttagatttta    3180
aattattgtt ttatttattt taatagttat atttgaaatt ttagaggtta aggagaaatg    3240
tttgtaattt atttttttag ttgtgaattt ttgtatttga aaggttaaat tttttttttt    3300
ttagtaggta attattttat gttttgaagt atgagatttg attattttta ttattatttt    3360
agtttgtata gatatagatg ttattaacgg ttgtagttta tttaattttt ttttaaaacg    3420
tagttgtagt atttgatttg gggatttttt gtgaatttta ttggttgatt tcgttttgta    3480
taaagtacgt ggggtttggg ggagggagaa gagagaggga ggtttgtttt gttttggagg    3540
gttttttttt tttttttttg aattagagtt ttttaaatag ttattcggta tttttgtaag    3600
tgatgtgaat tttgaaatta tttattagaa taggataata gagtattgtg tgaattagat    3660
ttgttggaaa taaagtatag tagaatgtat gatgtttttt attttattgg tagagattaa    3720
atatgtggtt ttgatatatt tatttatata ataaagaatt gtagtttatt ttgaaatata    3780
ttggttatat ttaaattgga gtatttggtt ttatataata attgttttta taagtgttga    3840
aatagtaatt tggataaagt aggttttaaa gttgttgtga agggaagata gggacgtttt    3900
taaaatttga aaggtttaga ttggtaattt aggtagatta aatttagcga ttaattggtt    3960
aatttaatta ttgttttttt tttagtattt atgatttgtg tttttaagta ttgtttttgt    4020
aggttatatt attttttta taaaatagag aggatatttt ataggttttt tatattttgg    4080
aatatagtaa attttaagta ttggtagaaa gggaatttag aaaaaaattt tgtgaagtaa    4140
aggaggttga cgttgtataa atattgaatt atagttttaa gatgtataaa atattgtttt    4200
taattttaat tattttatta gtaagtaagt gaaattgttt taattggagt ttttagaagt    4260
ttttttggga aatgagaggt agttggatgt agtagaatgt agtggaatga gtattttaaa    4320
```

```
gagtattgga tttagtatta ggagatatgg gttttgggga tgatttttgt tttttagtga    4380
gtaagtaagg ataagttgtt tttaaggttt ttattttttt tatttataaa atgattatat    4440
tagaggagtg ttttgtaatt tatgtgtttt gtgattttac gttaaatgga agttaagtta    4500
gattgtgtaa tttataggaa attaggaggt tgaagaaagt ttggtagtta gtattttttt    4560
ggatgtagtt tgtgttttgt ttttttgttt ttttagttat ttttttgttt ttagaaagtt    4620
aaaaatgatt tgtattgttt tttaaggttt ggagatttag aggatttgtt tttgtttttt    4680
ttttatttta ttgttggggt tgagtaaaaa tgagtttttt attttaagtt ttagtagttt    4740
ttattgagta ttattaggta ggtttattta tatattttat atttataaag tattattatg    4800
tgttagggtg taagggatta gaaagatgtt tttatttgat tatttttgga gttagagaat    4860
gttaagatgt agcgggattt tagaagtttc ggagttttag tttgatttta gaattgaatt    4920
tttgtttaat taagggtgtg aataaatgta aagatatgat ttgttatttg ttaaggttgt    4980
ggggtgaggg gggtggttat atagagtatt gtttttttgt tgttagtagc gtttattttt    5040
ttatttgtgg ggagtaatag attattttttg gtaatatagt gtttaaaagt gttaagaaaa    5100
atttttgtaat ttaggattta ttggatttta gagaataagc gttttttttt tggattgttt    5160
ttgtttattt ggggggtaaa tttggataag aattaaataa tttgtagaga gatttatagt    5220
agaattaaga gtaagtttgt aaatattgtt ttttttattt ggtatttagt aagtatttat    5280
tatgtgttag atattgtttt atgatttatt ttttttttta ttatttattg ggtaaataat    5340
agttataggt tgaattatga tgtggaagta aatgtaaatt aatttttttt tttatataaa    5400
taaataaata aataaataaa taaaaatttg tgttagaatg tttttggata ttaaatatta    5460

aaatttagtt aagtttagtt ggtaggtttt tttagcgatt tttttggttt gagtattttt    5520
taattttgta tatcggtaag gggaatggta ttttaaagga gtgatttttt agaggttgaa    5580
tggtttttgt gaggggtcga ttatagggga ttggggaagg ggttttttta atattaattt    5640
tattagtttt gttagttgat gggaaagttt gttttttttat tttcggtatt ttaatttaga    5700
agaatgtgtg tgtgtggagg gggtgttaat ggaaaaaatg ttatcgtttt tagagaagtt    5760
agtgaatggt agggttagta gttaatttta gatagtttga aattttgagg ggtgttttgt    5820
atttttgttt tttttatata atttagttaa taggattttt tttaataggg tattagtaga    5880
ggaatgagtt ataatataat atagttttta gaggattgta gaattagtag atttttttaa    5940
tataaggtta gttttttagg gtaattttgg atagtttatg ttatcgtata ttggtttagg    6000
gtttttttgta tatattttta gtgttttgtg ttttatttta atttaaaata tttattattt    6060
tttatgtgtt tgttttttgt tatatggaga aaggagttat gttattaatt tgttatattt    6120
ttagggtttg gtatatagtg ggtgtttagt atttgatgag tgggtagatt gatggatggg    6180
tagaaatatt ttttgattta tggttttttt atgattttat tttttatttt ttggattaaa    6240
atgtaaatat ttttcgataa aagaattttt tttgcgtttt aggttttggt tttggttgag    6300
agttttatat ttataagcgt ttatagagga aattaggggt tatgtttgaa tttgtttaga    6360
agggttttag aatttttgag ggtgtggtta tttttattat tttttaaagt aataaattta    6420
tttttgtaat ttaaggtttt gtttttgtta aaagataatt ttgagttaga tggtatttgt    6480
tttttattaa ttattttttt taatggattt ataggaaaaa atttttgaaa gttaagatcg    6540
aatagggtga gaattttttt ataaataata atttggaatt tagagaggtt tagttattcg    6600
tttaaggtta tatagtttgt tggtatagtt ttgaattttt atttcgtggt ttttagcgtt    6660
tagtttggtt gtgtttttag tggtttttta gggtatttgt aggtgatttt tttttatttg    6720
tttaatattt atgtagtatt tattatgaat taggtagtat gttggggttt gtggatagaa    6780
agatgaagag ttatagtttt tgttttgtaa aggttttttt ttttgagttg tatttatggt    6840
agttttaagt ttttacgttt taaattcgag gtttttcgtg gtttgtagta gttttgtttg    6900
agtagaaggt agttcgtatt aaggttttag gtttatttgg gatttggtgg gcgacggggt    6960
tttagttttt ttcgtttatt ttggagtttg ttgtcgtcgt tattattgaa ttagttatgt    7020
tagaaagtta ttacgatgat tttagaattg tattgtgatt tatgtaaagc gcgtcgataa    7080
ttatagtcgg gcgtatagaa tataggatgt atatgttgcg ggtgtgtgcg cgtagtgttg    7140
tgtttttcgt tagggttgaa tggggtgtgt gtgtatttcg taggtatttt tttgatatta    7200
tgtttgatcg gggattggtg ataggaaaaa gagggaagat atttattttg attttgagat    7260
aatttgatga gttttgggag tattttttag aagttttggt gtttataaat gagttagttt    7320
ggtaggagcg ttcgagagta ggggaagagg tggtttggag tttaggaaag ttatcgttta    7380
gttttcgtat aggtaaagta attagtagtt tttttttcg aaatagattt ttaaatgttt    7440
tttttgtaga taggtttggg tttagttatt atttttttata ttgtttttat tttcgtttta    7500
gataaattag ttttattttt gaagtttttt gttttgaatt aaagaatttt ataatcgcgt    7560
tcgcgttatg ggggtggtgg tggtaggtgg ttgaagtaag tggattttttg gcgtgaatta    7620
gaatttgagg attttaaacg gagtttggta ttttattgtg ttttttaaat tttgttcgaa    7680
atttttttt tttttgggcg tcgagttcga gaaagcgtta cgtcgtcggt cgggttagtt    7740
ttataagcgg ttgtataagt tggttgttaa aaaacgttga tttttttttt tgttatttaa    7800
taaattttta cgcgtttatg gtttcgtttt ataatttttt aatttcgttt taattcgaaa    7860
aatcgaggag gagggaataa attgagagat aaagattttt ttattttgtt ttttttcgg    7920
```

gattttagtt ttggtcgcgg cgttttatta aggaggatat aggttttggt gtgtgtggtg 7980
tgcgagattt cgagttcgag gtcgagttaa ggttgggtag aaagttgtaa ttacgtgttg 8040
tcggagttta ttggagcgta tagttcgttt tttttgggac gtttaggcgg aggatttgtt 8100
gcgttttttt agggttcggg ggattttagt atttatttgt acgtataggc gaattttgat 8160
tgaaagttcg ggatgatatc gagtttggag aaagagggat cggggggtgg gttggcggaa 8220
ttgtagagcg tcggttatag tttttttttt cgcgaacgtc gagcggaggg cgggaggtgt 8280
aatttttgat ttttggtcgg gtttacgttt tgaggaggga ttggtaagtt tttgttcgat 8340
aagtttaagt tgtcgagaga gtttaaatag aattaatttt ttagagatcg gggattatcg 8400
ttttttcggt atgcgttttt ttagcgtcgc gtatagagta aggcgcgaga gagtttagga 8460
atcgcgggaa ggtaagcgga atggggaggg ggtaggggat gagggttttt ttttattatt 8520
ttttcgttcg gagagcggga gttcgtaacg ttcgtcgagg acgagcggcg ggagggaacg 8580
ttttgttttt tagtcgtttc ggtgtagata atggaggcga taagagattc gtttagcgtc 8640
ggatgggtta gttttgtttg gggaagttgg cggtattttt ttttcggttg gtgtttaaat 8700
ttattgcgcg aaggtcgaag gaacgcggaa tttttagaag attttatttt tagttttgat 8760
ttttcgtaga tatgtgtaaa atgagtaaat tatttatttc gggttagatt taagttttat 8820
ttaatagaag gggtatcgga ttaagaatga attaatttat atggttatgt tcggcgcgta 8880
taattatacg ttagtatata gttatttaat ttttttcgcg aatttatttg gtattttgga 8940
gagaggggga aaagcgtttt gagaaagttt cgttattttt tttttttttt tttgtcgtga 9000
aatatacgaa tttattttta ttacgagtcg tatcgttttt attattacgt acgtatagag 9060
ttatattttt atatttatat tttttaattc gtaagtttcg atagcgtttg tatttttttt 9120
gggagtcgtt tggaggttta ttaatattat tagtatttaa tttttttttt ttttttattt 9180
ttttttcgta tatggttgac gttagatttc gttaggagtt gggggaaaag ttaagtgggt 9240
tagggacgtt ttattttttt tttcgcggtt gtttgttaga gcgtttttgg agatattata 9300
ggggatttag ttcgtagcga taggtataaa gttacggggt aatgaatttc ggggattttt 9360
cgtcgttgcg tgcgcggttt ttttcggaaa ttcggatttg gtcgtttttt ttttcggaag 9420
atttttagt aatttagttt ttttattttg cgtttgggtt tcggtagcgc ggagttcgtt 9480
tgtttttgag attgcggtag tgtttttttt tttttttggg gagattagcg gtcggtagag 9540
attgtttata ttttgtatgt ttatgtagag ggagagatcg aagattgagt gataggaatg 9600
gggaaaaaga gggatttcgt ttcgtaggaa ggttattttc gtgtttttat ttttgttttt 9660
taatattttt tttttgttgt tttttttttt tttttagttt ttttgtttat tttttttattt 9720
gtttgtttat gtgtgtgttt atattaagta gtatttttag tagttgcggt tttgtaagag 9780
tcgggaagaa atttaaggat gtttaaattt ttattgttgg acgaattttg agcgtttagg 9840
gagtagcgta gcgcgcgatt gatatttatt tgtttcgttt aggagttttg taggttggag 9900
ggtagttgga gagcggcggc gttcggcggc gaggcgggcg ttgtcggtcg ggattcgggt 9960
agcgtttatt aatcgtttcg tttcgggata gttagtatga gtaagttagt cggattaata 10020
agtgggtatt tttcgggtcg tcgtggggtt taggcgcgta gtttggggcg agcgagcggg 10080
gaggttgggg gaggttttgt ttggagcgtt gcgaatttga gtttttgaga gggattttag 10140
cgggcgtgtg cgttcggttt agatttgtag atcgtgagtt ggagtatttc gtggagaggg 10200
gagagtcgtt tcgttgtttt tggattgttt gatttttttt gtttggtgcg gtgagaaggt 10260
tacgattcgc gtagtttatt agtcggatga gttgttttta tttagtcgtt aggtgttgga 10320
tggggggggtt atgggggcgg gaattgggtc gtagttttag gcggtagtat aataatatat 10380
tcgtttaaaa tttcgagttt tagcgcgtaa aagtaatttt gtgtaaagcg gattttgaat 10440
ggaatgtttt gtatttcgtt tttagttatt ttaaataatt ttgtaaattg ggaagtagaa 10500
ataatttaaa agttatattt tttttaattt taaattcgcg taggttataa ttggggaatt 10560
taaagtatgg cgaattattt tagtaaagag aggattaaat ttttaatttt aaggattttt 10620
cgagtcggag tttagtagag gtaggagtgc gcggtttgtt tttttcgtgc gttttttttt 10680
tttttcgaat ttttttagtt gtcggttttt cgaacgttag gtcgtagttg atttttttatt 10740
atttcgagat ttacgagcgt agggttaagt gtgtgtgcga gggtatttgt ttgtattttg 10800
tttgcggaat ttaagaatgt gtaggttcga gttagcgttg agtaggcgcg gttacggtgt 10860
ttagattttt cgggggtatt ttagttttcg ttatttagtg gtttacggtt gcgggtttta 10920
gggtttgagg ttggggatta tcgttgtcgt cgtagttttt atatttcgaa gttgttttgt 10980
tgtttgtgtt gttttcgtag atagtatttt tggcgttttg tgcgtttttt tttttttttt 11040
ttttttttta ttcgttttta ttgttttgag tttttgaaag ttgggagaat cggagatatt 11100
tttgaggatt ggtaatgaag ttttatttaa gtgggatgta attttcgttt ttttattttt 11160
ttttaagaag gaggttgtgt ttttattttg ttttgttttg ggtgttgatt tttaaaaaat 11220
tagagtaaaa tgaacgtgaa taaaaagaaa aggagaaatg tttcgagttg ggtagaggg 11280
agtagagaag gagtttttat cgcggtcgga atgtagagcg gattttggtt taggattggg 11340
ttttttttta ggttcgggtt tattttggtt ttcgttgttg gaatttttag gaggtaaagc 11400
gatttcgatt tttgttgttc gtattttcgg gcgtgagtgt ttttttttagg aggtttagga 11460
ggtcgttttt gttgtatttt gagttttcgt tgtaaaaatt gaagttcgtg ggtttcggta 11520
ggtttttttag ttcgttcgtt tcgggatagg ttttcgttta tatttttgga agtaaggagt 11580

```
ttcgggtttt ttcgtttttt tcggggtgtg gagtttttgg ggtttttgaa aggtgaggtt   11640
ttagaggcga gggaggggtg agcggggagt tttgttcgtt tgcggttgcg ttttcgttgt   11700
ggattaggag gtaggttaat ttttcggatt ttgggggaaa aattatagcg ggtttttttgc   11760
ggaaattttg gtcgttttaa tttgttaaga gtttgagtga ggttttggaa gtttttagtt   11820
tcggtttagg tcgggacgcg gttgttgagt tttttaggt tcgtaggata gcggttttcg   11880
tcggtggcgt cgttgtattt aggttttttt tagatcggtg gcggtagtta attcgagatt   11940
tgcgtttttc gggttcgggg tagttaggag gtcggcgcgt agcgggtcgg gttaggattg   12000
ggtcgagtag atagagttgt agttttcgtt ttgttcggtt tttcgcggtt ggagagtaga   12060
gcgatgttat tcggagtttc gtttgggtgg taacgagatt ttggttagtt atttttgtag   12120
tttagattaa tttttttttaa tagtttttga tggtaattat agtaatcgaa gttgttatat   12180
atttttaggt aattatgata tataaaaagt ttcgagggga ttttttggcg agggaagtta   12240
agaacggttt tttagtttta ggaaatttcg gttcgtttta cgtcggagtt cgttcggttt   12300
gttaaatgag aggagttttg taacggggtt aattagtttg tttcgtgatt ttaagttatt   12360
ttaacgtggt tgggtggcgg agtttgaggt ataggttcgt tatgtttcgg aattttcgcg   12420
tttttttttt ttgggtttcg ttttagttcg gttgtttgtt tttaatttgt ttcgggagtc   12480
gcggtttaga ggtttgttta gaggtaggat tcgtattggt ggtggtttag agggtaatag   12540
ttcggaagtt cgggcggggg aattcgagga ggggttttta ttttgtattg gttttttttt   12600
tatttttatc gggtttttta gagatgttgt tatgggtttt ttgttcggat gggaagtggg   12660
gaaaaagaga ttttattatt gtttttttta tttatttatt ttggtatttt taatagtgtt   12720
atagggaaga ggatagttgg ggtgatagta ttgttgggtt ttaaacgtgg atttggagc   12780
ggttagaggg cgcgtgttaa gtagaggatg gttgtaagat atggaaatta agtttatcgt   12840
ttttagagtg agtgtagtgt agcggagttt tagattcggt ttttgagcga ttaggaattt   12900
ttttgggcgt agtgataggt tcgaaggagt ggaggataag attcgttgtg tttttgtttg   12960
gtttttttta gtttttttttt tttatagtat aattttttttt ttcgttttgt gggaggggggg   13020
cgtcgagtcg gtggtcgtcg cgtcgcgcgt tttttttgtt cgtttttgtc gtcgttcgaa   13080
tttttatgtt atttttttttg acgggttatt ggtggtttta taggtaggtg ttgtcgcggg   13140
gttgtaatta ttcggtcgag tttggtcgtt atcgaatatt tttttatttt taagtacggt   13200
ttttcgcgtt tttttttttt tattttttttt atttgagacg agtttgggat ttacggtgta   13260
agttcgttcg gacgtttcgg gttttaggag taagtttata tttgcggtgt ttcgaggttt   13320
ttttcgcgtt acgtttttat ttttttttatt tttttcgttt agacgtttta ttgtcgtttt   13380
tttttatttt aattttatcg tagtttttaa tttaagcgtt ttcggtagta gttgtcgaat   13440
tttagggagg tttgaggtcg ttggggttta aattggaggt tttcggatag gtagaagaag   13500
agaaaagtaa gtgttaataa gttaattgtt attaatt                           13537
```

<210> 164
<211> 13537
<212> DNA
<213> Artificial Sequence

<220>
<223> chemically treated genomic DNA (Homo sapiens)

<400> 164

```
agttgataat aattgattta ttaatatttg tttttttttt tttttattta ttcgaaggtt      60
tttagtttgg gttttagcgg ttttaggttt ttttgaaatt cgatagttgt tgtcgaaagc     120
gtttgggttg gggattacga tgggattgag gtgggaaggg gcggtagtgg aacgtttggg     180
cgaggggagt aggggaggtg agggcgtggc gcggggaggg tttcgaggta tcgtaggtgt     240
aaatttgttt ttggaattcg aggcgttcgg gcgagtttgt atcgtgggtt ttagattcgt     300
tttaggtgga gggggtgggg gaaggagagc gcgggggatc gtgtttgggg gtgagggggt     360
gttcggtaac gattaggttc ggtcgggtaa ttataatttc gcggtagtat ttgtttatag     420
agttattagt gattcgttaa aaagagtagt atgggaattc ggacggcgat aaaggcgggt     480
aggggaggcg cgcggcgcgg cggttatcgg ttcggcgttt tttttttata gggcgggagg     540
ggagattgtg ttgtgggagg gaggggttgg agagggttag gtaagggtat agcgggtttt     600
gtttttttatt ttttcgggtt tgttattgcg tttaagggag tttttggtcg tttaggaatc     660
gggtttggag tttcgttgta ttgtatttat tttggaagcg gtaaatttgg tttttatatt     720
ttataattat tttttgttta atacgcgttt tttgatcgtt ttagggttta cgtttggggt     780
ttagtagtat tgttatttta gttgtttttt tttttgtggt attgttgaag atgttaagat     840
gagtgagtgg gaggagtagt ggtggggttt tttttttttt attttttatt cggataggag     900
gtttatgata atatttttgg agaattcgat gagggtggag ggaagattag tgtagggtgg     960
gggttttttt tcggattttt tcgttcgagt tttcggattg ttgtttttta ggttattatt    1020
```

agtgcgagtt ttgtttttga gtagattttt gagtcgcggt tttcggggta gattagaaat 1080
aggtaatcgg gttggggcgg ggtttaggag ggagggacgc gaaaatttcg gggtatagcg 1140
ggtttgtgtt ttagatttcg ttatttagtt acgttaaggt gatttggggt tacgagataa 1200
gttggttgat ttcgttgtaa agtttttttt atttagtaag tcgagcgagt ttcgacgtga 1260
ggcgagtcgg agtttttga agttggaaaa tcgtttttaa ttttttcgt tagggggttt 1320
tttcggggtt ttttgtgtgt tataattgtt taaagatata tggtagtttc gattattgta 1380
attattatta gaggttgttg aaagaagtta gtttgggttg taggggtgat tggttagggt 1440
ttcgttatta tttaggcggg gtttcgggtg atatcgtttt gttttttagt cgcgagaagt 1500
cgggtaaggc gggggttgta gttttgtttg ttcgatttag ttttgattcg gttcgttgcg 1560
cgtcgatttt ttagttgttt cgggttcgag ggacgtaagt ttcgggttgg ttgtcgttat 1620
cggtttggaa aggggtttaaa tgtagcggcg ttatcggcgg gggtcgttgt tttgcgggtt 1680
tgagaaagtt tagtagtcgc gtttcgattt gagtcggggt tggaggtttt taaggtttta 1740
tttaggtttt tggtaagtta gaacggttaa agttttcgta aggagttcgt tgtggttttt 1800
tttttaaagt tcggagggtt ggtttgtttt ttagtttata gcgggggcgt agtcgtaggc 1860
gggtagagtt tttcgtttat ttttttttcg tttttgaggt tttatttttt aagggtttta 1920
ggggttttat atttcgaaaa ggacgaaaga gttcggggtt ttttattttt aggggtgtag 1980
gcgagggttt gtttcggggc gagcgagtta ggaggtttgt cgagatttac gggttttagt 2040
ttttgtaacg gaggtttaga atgtaataga aacggttttt tgagtttttt gggaaggata 2100
tttacgttcg ggaatgcggg taataaaaat cgaggtcgtt ttattttttg gagattttaa 2160
tagcgagggt tagggtaggt tcgagtttaa agggaaattt agttttgggt tagggttcgt 2220
tttgtatttc ggtcgcggtg agggtttttt ttttgttttt tttgttttag ttcgaaatat 2280
tttttttttt ttttttgttt acgtttattt tgttttaatt ttttaaaggt tagtatttaa 2340
agtaaaataa aatgaaaata taattttttt tttggagggg ggtaggaggg cgggaattgt 2400
attttattta agtgagattt tattattagt ttttagaagt attttcgatt tttttaattt 2460
ttaaagattt aggataatga gggcgagtga aagggggagg gggagggaag gaacgtatag 2520
agcgttaaaa atgttatttg cgaaaataat ataagtagta aggtaatttc gggatatggg 2580
gattgcggcg gtaacggtag tttttagttt tagattttgg agttcgtagt cgtgggttat 2640
tggatggcgg gaattgaaat gttttcggga gatttgagta tcgtgatcgc gtttgtttaa 2700
cgttggttcg ggtttgtata tttttgggtt tcgtaggtag ggtgtaagta aatgttttcg 2760
tatatatatt tagttttgcg ttcgtgagtt tcggggtggt gagaggttag ttgcggtttg 2820
gcgttcggag agtcggtagt taaggaagtt cgaggaagga gagaagcgta cggagggagt 2880
aggtcgcgta tttttgtttt tgttgggttt cggttcgaaa agtttttggg attagggatt 2940
tggttttttt tttgttagag tggttcgtta tattttaaat tttttagtta tgatttacgc 3000
ggatttagga ttaaggaaaa tgtgattttt aaattgtttt tgttttttag tttgtaggat 3060
tatttgaaat agttagaaac ggggtgtaaa gtattttatt taaaattcgt tttgtataga 3120
gttgtttttg cgcgttggag ttcggagttt tgagcgagtg tgttattgtg ttatcgtttg 3180
gagttgcggt ttagttttcg tttttatggt ttttttattt agtatttggc ggttaaatgg 3240
agataattta ttcgattggt gggttgcgcg ggtcgtaatt tttttatcgt attagatagg 3300
ggggattaag taatttagag gtaacgaaac ggtttttttt tttttacgaa atgttttaat 3360
ttacggttta taggtttggg tcgaacgtat acgttcgttg gaattttttt taggggttta 3420
gattcgtagc gttttaggta ggattttttt tagtttttc gttcgttcgt tttaggttgc 3480
gcgtttaggt tttacggcgg ttcgagaggt atttatttgt tgattcggtt ggtttgttta 3540
tgttggttgt ttcggggcgg agcggttggt gggcgttgtt cgagtttcgg tcggtagcgt 3600
tcgtttcgtc gtcgggcgtc gtcgtttttt agttgttttt tagtttgtaa ggtttttggg 3660
cgggataggt gggtgttagt cgcgcgttgc gttgtttttt gggcgtttag aattcgttta 3720
atagtggaaa tttaagtatt tttaagtttt ttttcggttt ttgtaaaatc gtagttgttg 3780
ggaatgttgt ttgatatgga tatatatatg gatagataga tggagagatg gataggaaga 3840
ttgaggaaga aggaagaata gtaagagagg gatgttgaaa gatagagatg gagatacgaa 3900
aatggttttt ttacggagcg aaattttttt ttttttttat ttttgttatt tagttttcga 3960
tttttttttt tatataggta tgtagagtgt gggtaatttt tgtcgatcgt tggtttttta 4020
aggaaagaag gaaaatatta tcgtagtttt agaggtagac gggtttcgcg ttgtcggaat 4080
ttaagcgtag agtgggaaaa ttagattgtt gggaaatttt tcgagggaag aggcggttag 4140
gttcgggttt tcggggagag tcgcgtacgt agcggcgaag ggttttcgaa gtttattatt 4200
tcgtgatttt gtgtttgtcg ttgcgggttg ggtttttgt aatattttta gaagcgtttt 4260
gataggtagt cgcggggagg ggaatagggc gtttttggtt tatttagttt tttttttaat 4320
ttttggcggg gtttgacgtt agttatgtgc ggggaggggga tgggaagagg gagggggtta 4380
aatgttaatg atattaatga atttttaagc ggtttttaaa gaaaatgtag gcgttgtcgg 4440
agtttacgag ttagaaagtg tgaatatggg agtgtggttt tgtgcgtgcg tgatggtgag 4500
gacggtgcgg ttcgtaataa aaatgaattc gtatatttta cggtaagaag gaaggggagg 4560
ggtgacgggg tttttttaaa acgttttttt tttttttttt agagtgttag atagattcgc 4620
ggaagaaatt gggtggttgt gtgttggcgt gtgattgtgc gcgtcggata tggttatgtg 4680

```
agttggttta tttttggttc ggtgtttttt ttgttgggta aaatttggat ttggttcgag    4740
gtgagtaatt tatttatttt gtatatattt acggaaagtt agggttgagg atggggtttt    4800
ttggaggttt cgcgtttttt cggttttcgc gtagtgggtt tgggtattag tcgaggggag    4860
gatgtcgtta gttttttaa gtagagttgg tttattcgac gttgagcgaa ttttttgtcg    4920
tttttattat ttgtatcggg gcggttggag ggtagagcgt tttttttcgt cgttcgtttt    4980
cggcgggcgt tgcgggtttt cgtttttcgg gcggaggaat agtgaagaga ggtttttatt    5040
ttttattttt tttttatttc gtttgttttt tcgcgatttt tgagtttttt cgcgttttgt    5100
tttgtgcgcg gcgttgggag agcgtatgtc gagggagcga tgattttcga tttttaagag    5160
attaatttta tttaagtttt ttcggtagtt tgaatttgtc gaataagagt ttgttagttt    5220
tttttaggg cgtggattcg gttagaggtt agaggttata tttttcgttt ttcgttcgac    5280
gttcgcgggg aggggagttg tggtcggcgt tttgtaattt cgttagttta tttttcggtt    5340
tttttttttt tagattcggt gttatttcgg gtttttaatt agagttcgtt tgtgcgtgta    5400
agtgaatgtt ggagttttc gagttttggg agggcgtagt aggtttttcg tttgggcgtt    5460
ttagggggag cgggttgtgc gttttagtgg gtttcgatag tacgtgattg taattttttg    5520
tttagttttg gttcggtttc gagttcgggg tttcgtatat tatatatatt agagtttgtg    5580
ttttttttag tggggcgtcg cgattaaggt tggggtttcg gggaaagagt aagatggggg    5640
gatttttatt ttttagttta tttttttttt ttcggttttt cgaattggga cgagattggg    5700
ggattgtggg acgaggttat aagcgcgtag ggtttatta ggtgatagga ggagaaatta    5760
gcgtttttttg atagttaatt tgtatagtcg tttgtggagt tagttcgatc ggcggcgtag    5820
cgttttttcg aattcggcgt ttagagaggg gaggaatttc gggtaggatt tgagaaatat    5880
aataagatgt taggtttcgt ttggaatttt taggttttgg tttacgttag aaatttattt    5940
gtttagttta tttattatta ttatttttat aacgcggacg cgattatggg gtttttttggt    6000
ttaggataaa gaattttaag ggtggaatta atttatttgg ggcgaagatg agggtagtgt    6060
gggggggtgat gattagattt aggtttgttt gtaggggaag tatttagggg tttgtttcga    6120
gaaggagaat tattaattgt tttgtttgtg cggggggttgg acgatggttt ttttgggttt    6180
taaattattt ttttttttat tttcgaacgt ttttgttagg ttggtttatt tgtaggtatt    6240
aaaatttttg gaaaatattt ttaaggttta ttaaattgtt ttaaaattaa aatgggtatt    6300
tttttttttt tttttgttat tagttttcga ttaagtatgg tgttaagaag atgtttacgg    6360
aatatatata tattttattt agttttggcg aaaggtataa tattgcgcgt atatattcgt    6420
agtatatgta ttttgtgttt tgtgcgttcg gttgtaatta tcggcgcgtt ttgtataaat    6480
tataatatag ttttgaaatt atcgtgatga ttttttggta tgattggttt agtggtaacg    6540
gcggtaataa gtttagggt aggcgggagg ggttgaggtt tcgtcgttta ttaggtttta    6600
aataagtttg gagttttagt gcgggttgtt ttttatttag gtaaagttgt tgtaggttac    6660
gggggatttc ggatttaagg cgtagaaatt tggggttatt atgggtatag tttagagaag    6720
aaagtttttg taggataaag attatggttt tttatttttt tatttatagg ttttagtatg    6780
ttgtttgatt tatagtaggt gttgtataaa tgttgggtaa atgaagggaa gttatttgta    6840
gatgttttga ggggttattg gaggtatagt taggttaaac gttggaaatt acgagatagg    6900
gatttagggt tgtgttaata agttgtgtga ttttaggcga gtggttagat ttttttgggt    6960
tttagattgt tatttgtaaa aggattttta ttttgttcgg ttttaatttt taaagatttt    7020
tttttgtgag tttattaaag gaaatgattg ataaggggta aatgttattt ggtttagaat    7080
tattttttaa tagagataag attttaagtt ataaggataa gtttattgtt ttggaaggtg    7140
gtggggatag ttatattttt agaggttttg aaattttttt aaataggttt agatatagtt    7200
tttaattttt tttgtaggcg tttgtgagta tggagttttt aattaaaatt aaagtttaaa    7260
acgtagggaa ggtttttttta tcgaaggatg tttatatttt ggtttaagag ataggaaata    7320
agattatgag aggattatgg attaaggggt atttttgttt atttattaat ttatttattt    7380
attaaatatt gagtatttat tatatgttag attttgggga tatagtagat taatgatatg    7440
attttttttt ttatgtagta gggagtagat atataaaaga tgataaatgt tttagattgg    7500
gatgaaatat aggatattgg agatatatat aaggagtttt aaattagtgt gcggtgatat    7560
aggttgttta gaattgtttt ggaaagttgg ttttgtgtta ggagaattta ttgattttat    7620
agttttttga ggattgtatt gtattatagt ttattttttt gttagtgttt tgttaaagag    7680
aattttatta attaggttgt atgagagagg taggggtata gagtattttt taaagtttta    7740
gattatttga ggttgattgt tagttttgtt atttattagt ttttttaaaa acggtgatat    7800
ttttttttatt aatatttttt ttatatatat atattttttt aaattaaaat atcgaaggtg    7860
aaaggataag ttttttttatt agttagtaga gttgatggaa ttggtgttaa agagattttt    7920
tttttaattt tttgtgatcg atttttttata gaggttattt aatttttaga gagttatttt    7980
tttaaaatat tatttttttt atcgatatgt agagttaaga gatgtttaga ttaaggaaat    8040
cgttgggaaa atttattagt tgagtttggt tgagttttga tgtttagtat ttaaaaatat    8100
tttgatataa gtttttgttt gtttgtttgt ttgtttgttt atatgaagga ggaagttgat    8160
ttgtatttgt ttttatatta taatttaatt tatggttgtt gtttgtttag tggatggtga    8220
gagaggaggt gggttatgga gtagtgtttg gtatatagta ggtgtttgtt aagtgttaaa    8280
tgaaagaaat agtgtttgta ggtttgtttt tgattttgtt gtggattttt ttataagttg    8340
```

```
tttgattttt atttaaattt attttttaaa taaataagaa taatttagaa aagagacgtt    8400
tgttttttag aatttagtgg gttttgagtt gtagggtttt ttttgatatt tttgagtatt    8460
gtattgttaa aagtagtttg ttattttttta taagtaggaa gatgaacgtt gttaataata    8520
aggagataat gttttgtgtg attatttttt ttattttata attttaataa ataataagtt    8580

atgtttttgt atttatttat atttttggtt ggataaaaat ttagttttaa agttaggttg    8640
agatttcgag gtttttagag tttcgttgta ttttgatatt ttttggtttt aagaataatt    8700
aagtagaaat attttttttag ttttttgtat tttggtatat agtggtgttt tgtaaatgtg    8760
gaatgtatga gtgaatttgt ttgatgatat ttaatgaaag ttattgaagt ttgaggtgaa    8820
gggtttattt ttgtttagtt ttagtaatgg ggtaggaagg gaatagggat aggtttttttg    8880
aatttttaaa ttttgggaag tagtgtaggt tatttttggt tttttgaaaa taaaagaatg    8940
attagagaaa taagaaagta gaatatagat tatatttagg gagatattgg ttgttaagtt    9000
tttttagtt ttttaatttt ttgtgaatta tatagtttaa tttggttttt atttagcgtg    9060
gaattataga gtatataagt tataaggtat ttttttaatg tagttatttt atagatgagg    9120
aaagtgagaa ttttagagat aatttgtttt tgtttattta ttgagagata gagattattt    9180
ttagaattta tattttttga tgttgagttt agtgtttttt gaaatattta ttttattgta    9240
ttttattgta tttagttatt ttttattttt tagaagggtt tttagaagtt ttaattaaag    9300
taattttatt tatttattag tgaagtaatt agaattaaga atagtgtttt gtgtattttg    9360
aaattatagt ttagtatttg tgtaacgtta gtttttttta ttttataggg ttttttttta    9420
aattttttttt ttgttagtat ttaaaatttg ttgtgtttta aggtatagga aatttgtgag    9480
gtgtttttttt tgttttatag aagggatgat atgatttata ggagtaatat ttagagatat    9540
aaattataga tgttgagaaa aaggtaatga ttagattgat taattaatcg ttgaatttga    9600
tttgtttgga ttgttaattt aggtttttta gattttaaaa acgtttttgt ttttttttta    9660
tagtagtttt aggatttatt ttatttagat tattgtttta atatttgtag aaataattgt    9720
tatgtaaaat taggtatttt agtttgaatg tggttaatat attttagaat aagttataat    9780
tttttattat gtggatgagt atattaggat tatatatttg atttttgtta atgaagtgaa    9840
aagtattata tattttatta tgttttgttt ttaatagatt tagtttatat agtattttgt    9900
tgttttattt tagtaagtaa ttttagaatt tatattattt ataaagatgt cgagtggtta    9960
tttagggaat tttgatttaa aaaaaaaaaa aaaaaatttt ttaaaataaa ataaattttt   10020
tttttttttt tttttttttt aaattttacg tgttttgtgt aagacggagt taattagtgg   10080
gatttatagg aggtttttaa gttaaatgtt gtagttgcgt tttaaaaaag ggttggataa   10140
attatagtcg ttaataatat ttgtatttat gtaagttaag atgatgatag gggtaattag   10200
attttatgtt ttagggtata agatgattgt ttgttgggag gaggaggatt tggtttttta   10260
ggtataggag tttataattg aggaggtagg ttgtagatat tttttttttag tttttaaaat   10320
tttaggtgta attgttggga taggtgaaat agtgatttga aatttaatat ttttattaga   10380
ttaggatatt aaataatgag atatttgaaa tattaatatg tattatatat aatatatgtg   10440
tatttattta ggatataagt attttgatat ttttttggaa ggttgatatt gtttgatata   10500
ttttgattat tttttagtgt taagtggata tttgttaagt attaaataat aatgaaaata   10560
gtggaggtaa tttgatttta gtttatgtat taatagttag ttataatgtg taattattta   10620
agtatttata attgtatata tttgtatttt taaatatatg tatttttagt tgtagtagtt   10680
aaataattat tggaattgta tatttggagt gtatagaaat gtatttattt taattttttgg   10740
attattaagt aatttttaaa atgtaatatt tatttggaag agtgagaaag taattttgta   10800
aaataatttt atttttttttt gatttttttta atattttttaa attttaagaa aggtatattt   10860
tagtatagtt gtataattta attaattttg aaaatattag tgtaagtgtg agtagggtta   10920
aataatttta tttatttaga tatgtaaatt atattgaagg ttatatagta gtagatagtt   10980
tttatatttt agagagtaaa aatgtagttt atttttagta gagtggttag agttttttttg   11040
gtatgaaaga tatttttatt atttattggt tttatcgtta ttgttaaatt atttatttta   11100
gttttatata gagggattgt tttggagaat atggttgtta aatgtaattt ttaaaatgat   11160
gtataaatag tttttaaagg gggaaaaatt ttgtagttgt ttggttgtag atagatatgg   11220
gaaattagtt agaagtttaa agtattatag ttaataatta ttattttgtt gtttgggatg   11280
tttgtttaaa tattattgat attgtgtatg tattgttatt ttttttaaat tgtagttttt   11340
aattattgaa attttgttaa agattattta gggttttttt tgtatgatgt tgaagtgtgt   11400
atttatttga tggataaata aaaaatagat tttttttttt ttgttttaat ttttttttta   11460
tgtgtaatta gtaggaaatt ttttggatta aagggagaga ttgtttaagt atagtagaat   11520
tagacgttgt ggatttttaa tttttgtatt ttttatttag tgatagaaga tgaataaatt   11580
tattaattga ttaatataat ttagttttgt ttaaaattta tagatttgtt tggtatgaga   11640
ttgtatttta aaagattttt atatgttatt aagtgtgaag aaattagtaa ttattttttt   11700
tatttatagt atgttgagtt tttttttatt ttatttaaat tttgtttatt aagtgaggtt   11760
atttgggaga agagtggttt tggtgggtgg ggaaaagaaa atttaatagt ttaattttta   11820
agttgagcgt tttagattat gggttattta gtataaagcg ttttatattt tgatttatga   11880
ggggataata gttggtttta taaaggtatt atttgtgatt tagtacggtt gatatttttg   11940
```

ttaaggagga gtcggagggt agtggcgaaa tagtacgtgt acgattcgga tcgtattacg 12000
gcgatcgatc gatttttaag atcgtttttc gtcgtcgcgg aggtttggtt aggtttttttg 12060
gtttatagtt tttggaaagg gtgtggggag agagaaagga aatgagcgtc gtatacgttg 12120
gtggagttaa agtttttgga tttacggttt gttagcgttt agttgtttta ttttatagat 12180
gggtaaaacg gaggttaggt tcgcggagtg tttggtttag taggtgtagt ggtttggatt 12240
ttcgcggggt ttcgggattg tagggtcggg ggcgcggggg ggcggcgcgg tcgcgggtgg 12300
gacgtagagt ttttgttttg ggtttaattt tcgggtcggt ggttttttgt tttttttttcg 12360
aaggtcgttt ttgataaatt tatttatttg gggtaaattg ttagtgagaa attttcgttc 12420
gaattgtttt ggataaaatc ggttgtttga aaagggtaaa tttcgcggtt cggcgaggtc 12480
gaataatggg tcgttggtgc ggttgcggcg gggcggtcg ggcgggtcgt ataaagggcg 12540
gattaattgg ggtcgcggtt gagcggttcg cgttagtttt atttagttcg cgtcgcgggg 12600
cgttttattg attgggtttc gagttttatt ttttataaat tttaaataaa agttaatttt 12660
ttttttataa ggcgggggag ggggtcgcgg ttaagatttt ttatttgttt ttgcgattcg 12720
tttttgtcgt atggggggtt tcgggggttt aaggaattgg aggtagaagg gttttcgggg 12780
atgttcgaga ggggaaagag atgcgggtgt ggttgtcgcg tttttgtttt ttttttcgtt 12840
gtttcgtttc gacgcgtgtc gtattttttg ggtcgtattt ataggtaggg gttgagattt 12900
tagttcgagg tttggaagtc gcgaagaagc gggatgaggt ggtggtcgga tcgaggtgaa 12960
ggttggggta ttcggttggg ggaagcgtag gtaggtgatt tgggaggaaa gaatacgttt 13020
ttttgatatt ttaaattttg ggaagggtgg ttatgtatta gatagttatt gagaaattat 13080
tgtgttttcg atggcgaatt tgtttttaat ggatagataa gtgggtgggg ggtagattta 13140
taagttaatt agttagtagg tgagggatcg cggttagtat ggggcgttaa aggaagttat 13200
tattttttttg taaaatgatg agatggattt gtcggtgtaa tatgtagaag atatttaatt 13260
atagatatag attttgtatt tggagaagtg gcggtgatta gaaaataaat aaataaattt 13320
aaattgtagg attaatattg ttattggttt atatatttcg taggaaggaa ttaggagttc 13380
gtttttttttt gtttttttttt ttttaaaata ggagaatgat tgtagagggt tttttagttg 13440
atattgagag gagtaggaaa tttttttaat aatataaatc gatatatttt ttagtttata 13500
agtttttgtt ggaaatcgga ttggcggtgg ttgtata 13537

<210> 165
<211> 2567
<212> DNA
<213> Artificial Sequence

<220>
<223> chemically treated genomic DNA (Homo sapiens)

<400> 165

ttattgaggc gttatacgta tttaaaaatt attattagaa agagaatttg aatttttttga 60
tttagagttt ggtttattga atttatttaa gttttattta gtgttagatt tatttggtat 120
tttttttttt ttagtgagga aattttgatt aggtttaagg taggaaggat aaatacgagt 180
agagaggtaa aaaaagttag aattaaattg taagaggttt aatgtggtat ttatattaat 240
ttattggttt tttttaatat tatatttata tatagttatt cggttttttt tttattgtgg 300
ggtgcgagag gtgatgagta ataatttttg gttatgtttg ataattttgt agttgttggt 360
ttttgagatt aagggaatat tatttatttg tttttatttt gttttaattg ggttgtagat 420
ttttatgatt gggatattat agatataata gattttttta ttgaagacgt aggtttttttg 480
aaagttattg cgttttagag aaacgtattt tagttatata ttgtggttag gattataata 540
gaatatgcgt ttattgttga gagtatagag atagtttcga attattatta ggttaaagga 600
taaaaaagaa tggggtagtg gagttattaa tgtttattgg tttttttttaa tattgtagta 660
ttttattttt tttaatttaa ttttagtaat agggtttcgt tttttttaaaa tgtagatata 720
gttattgaga tatgttatat ttatgttttt acgatatagt aagcgatttg taagttgttg 780
ttaattattt tgagttggtt tatatattta gaggtttgtt ttgggttgag tagttagata 840
gatgttatgg ttaggagaga tatagatagt taaagtggtg atagttttag tgtgagttag 900
ataggttaaa ggtgacggta ttttgtaaag gtttttcgag tatggattat agtagagaaa 960
gggatttttg gggtgtttaa agaagattat tatttttttg gtatatatat ttagtttttg 1020
gggtggattt tttgttgtag atataagaga gttgttgttg ttattgttgt tgttgttgtt 1080
tggtattggt attagagatt tgtataatag gttattatag cgtatttgta gggttttttt 1140
aataacgttt aggtagtatt tttttacgat gggtttggtt agtagttttt ttaagtagtt 1200
ttgatttttt ttagtgaagt gtatttagcg tacgtatttg aagatttttg tagtattggg 1260
atttcgtttt ttgggagtag tttttagtta ttgtattgtt atatggtata ttgtttgttt 1320
attttttacg tttagattat ttaagcgtag gatagttagt agttgggtta gggttagatt 1380

```
tgttagagtt ttttttcgaa ttgaatttat gtggttgagt tgtttgaagt tttgagttat    1440
ataggattgg gtttgggatc gtagtttgcg atggttaaag gtatttgtaa atttgaggat    1500
ggcggtgtag ttggttaggt taagacgtcg ggttaagaag gaggtatagg tttttcgtat    1560
atattttagt tgtagtatgt cggaggtcgc gtataggcgt tttacgttgg ttttattgag    1620
agatatacga ttcgtgtagt agtagtcgat taatatttcg aaggattcgg cgtttatatc    1680
gtgtatggtt acgttggttt gttggttttc gtatatgtta tttgtgaata tgtttttgaa    1740
gtagggatat gtcgcggtta gtatattgcg gttgtagggg aataggcgat tcgtgttagg    1800
ttcgttgtta ggcgttatta tttcgatggt tatattatat agtagtcgcg tatcgtagaa    1860
ggatttgagt tgtgttagta gggttgtaga atgggtcgtg ttttttaatt tttttggatt    1920
cgtgaaaaag gtcgaaattg tgggtttgtg aatttttttg ggtcgttttt tattacgggg    1980
attggttagg cggcgagagc gcggggcgtt ttttcgggat tgtatggtgg agatggcgac    2040
gggcgttgat ggcgagaaac gttgtaggat tcggttttgg ttttttttta attttttttc    2100
gttgacgtta agatagtagc gttttcgaag agatagtagt acgagtttat ttattgaatt    2160
taattttatt tcgtagaatc gtttttcgtt atcgtttaga tagtggttga tttatttttt    2220
tttattttcg cgtttttttt tcgcgtttgt tcgtttttat aggattcgtt ggtttggagt    2280

cgtagaagtc gttattgtag cgtgggcgat aatgttaggc gtttaggaga attatcgttt    2340
ttaggttgtt tttcgcgttt cgagggttgt tgggattttc ggattttcgg gtcgagatcg    2400
agggaaagaa ttcgttttgt atgtcgggag tagtagtttg tgtaatatcg cgattttttt    2460
ttggtgtttt ttgttttagt tgacgatatt cgagagcgtt aatttttttta gattcgattt    2520
tgtggtttgg aattgtggtt ttggaattta gagtagtcgt ttcgttt                   2567
```

<210> 166
<211> 2567
<212> DNA
<213> Artificial Sequence

<220>
<223> chemically treated genomic DNA (Homo sapiens)

<400> 166

```
gaacgaggcg attattttgg attttaaaat tatagtttta aattataaaa tcgaatttag      60
gaaaattggc gttttcgagt gtcgttagtt aaaataagga atattagaga aggatcgcga     120
tattatataa attattattt tcgatatgta aagcggattt tttttttcgg tttcggttcg     180
ggagttcgag agttttagta gttttcgggg cgcgaaaggt aatttgggag cggtagtttt     240
tttgggcgtt tagtattgtc gtttacgttg tagtagcggt ttttgcggtt ttaagttagc     300
gggttttgtg aaggcgagta gacgcggaga aaggacgcgg gagtgagaga gggtgagtta     360
gttattgttt aaacgataac gggaggcggt tttgcggggt agggttgaat ttagtaaatg     420
ggttcgtgtt gttgtttttt cggagacgtt gttattttag cgttagcgag ggaaggttga     480
ggaggagtta gagtcgggtt ttgtagcgtt tttcgttatt agcgttcgtc gttattttta     540
ttatgtagtt tcgggaagac gtttcgcgtt ttcgtcgttt agttagtttt cgtggtggga     600
agcggtttaa gaagatttat aaatttatag tttcggtttt ttttacgggt ttagaggaat     660
taaaggatac ggtttatttt gtagttttgt tggtatagtt taagtttttt tacgatgcgc     720
ggttgttgtg tgatgtgatt atcgaggtgg tgacgtttgg tagcgggttt ggtacgggtc     780
gtttgttttt ttgtaatcgt aatgtgttgg tcgcggtatg tttttatttt aagagtatgt     840
ttataggtgg tatgtacgag agttagtagg ttagcgtgat tatgtacgat gtggacgtcg     900
agtttttcga ggtgttggtc gattattgtt atacgggtcg tgtgtttttt agtgaggtta     960
acgtggagcg tttgtacgcg gttttcgata tgttatagtt ggaatatgtg cgggaagttt    1020
gtgtttttttt tttagttcga cgttttgatt tgattaattg tatcgttatt tttaagtttg    1080
tagatgtttt tggttatcgt aagttgcgat tttaggttta gttttatata gtttagaatt    1140
ttaagtaatt tagttatatg ggtttaattc gggaggagat tttagtagat ttgattttgg    1200
tttagttgtt ggttgttttg cgtttggata gtttggacgt ggagagtgag tagatagtgt    1260
gttatgtggt agtgtagtgg ttggaggttg tttttaaaga gcggggtttt agtgttgtag    1320
aagtttttaa gtgcgtgcgt tggatgtatt ttattgaaga agattaggat tatttagaag    1380
ggttgttgat taagtttatc gtgaagaagt attgtttgga cgttattgaa ggggttttgt    1440
agatgcgtta tggtgatttg ttgtataagt ttttggtgtt agtgttaaat agtagtagta    1500
gtagtagtag tagtaatttt tttgtatttg tagtagaaaa tttattttag agattgggta    1560
tgtgtgttaa ggagatggtg attttttttg gatattttag agattttttt ttttgttgtg    1620
atttatattc ggggggatttt tataaagtgt cgttattttt gatttgtttg gtttatatta    1680
ggattgttat tattttagtt gtttgtattt tttttgatta tgatatttat ttagttgttt    1740
```

agtttaggat agatttttgg gtgtataaat tagtttagaa tagttggtag taatttgtag 1800
atcgtttgtt gtgtcgtgag ggtatggatg tggtatattt taatggttat atttatattt 1860
tgggggggcg agattttatt attggagtta agttgaagga agtggaatgt tataatgtta 1920
agagaaatta gtgggtattg gtggttttat tgttttattt ttttttattt tttgatttaa 1980
tggtaattcg agattatttt tatgttttta atagtaagcg tatgttttgt tatgatttta 2040
gttataatat gtggttgaag tgcgtttttt tgaagcgtaa tgatttttag gaagtttgcg 2100
tttttaatga ggagatttat tgtatttgtg atattttagt tatgaaggtt tataatttag 2160
ttagggtaga atggaggtaa atgaataata tttttttggt tttagagatt aataattata 2220
gaattattaa gtatggttaa aaattgttgt ttattatttt tcgtatttta tagtggaaaa 2280
agaatcgggt gattgtgtat gaatatgata ttaggggaga ttaatggatt aatataggta 2340
ttatattagg tttttttgtag tttgatttta attttttttg ttttttttgtt cgtgtttatt 2400
ttttttgttt tgaatttggt tagagttttt ttattgaaga agaagaaata ttaagtgagt 2460
ttagtattga atgggattta ggtggattta gtgagttaga ttttgagtta ggaagtttaa 2520
gttttttttt tgatgatgat ttttgggtgc gtgtagcgtt ttagtga 2567

<210> 167
<211> 10664
<212> DNA
<213> Artificial Sequence

<220>
<223> chemically treated genomic DNA (Homo sapiens)

<400> 167

gcgtgggggg cgtggttcga agagattaaa tggaggtgga ggtatgggtt aaattgaatt 60
ttttaggtta cgggaggggt acggttgagg gtttttttcg tttttagacg tgatggtgtg 120
gtttaatgag cgggttatgg gttgggtgtt cgggttgggt ttgaaggaat ttgttacgaa 180
ttttacggag agcggggtat acggggtatt gttcgttttg gacgagattt tcgattattt 240
cgatttggtt ttgtttttgt agatttttac gtagaatgta taggtgagtt gtcgttgggt 300
tcggagtatg ttgggcgttt ttatttcgta gattgtacgt tttaatcgtt tttttattt 360
ttttttttta ggttcggtag tttttggaga aggaatttag taattttatt tttttaggta 420
tagataggcg gttggacgag gtgggcgcgg taatagttta gagggttttg tttttagcgg 480
tttttcgaga ggttgagttg agggggcggg gtttgattat taatggttac ggttggaggc 540
ggggttagga gaggggcggg gttaaaggag aggtgagatt cggagagggg tggagttaaa 600
ggaaggggcg gagttagata aggtaggagt tttttatcgg ttgttcggtt tttatattta 660
ggatagcgtt aagtttttta gtcgtttttt attttggcgg aagatgtttc gggagaagga 720
ttttcgaggc gtaattttcg atttagttga gatgttgttt tttaattttc gttcggttgt 780
agcgggagtt ttgggttttt cggggttttt ttttcgtaag ttgtagttag aaggtggggg 840
gtttttaaat gcgatagttt ttttttcgttt ttttagggtg gggtatggat tattttagta 900
gttcgggttt tggaatggtt tagttttttt tcgtttattt ttgaggaatg gattgtttta 960
acgtttcgga ttttttcgtt aggatgaaat ggataaattt tatttttttt ttttagggcg 1020
ggagtgaatt cgtttaatgc gagtttgagt ttttggttgc ggggaaggga gggaaattta 1080
tgtggagttc ggcgatcgtt gtgatatcgg gaagggaagt ttaaagggag gaattttgga 1140
gaggaatagg gaggagggtg ttttaggttg aatcgttgtt cgtttttttt ttaggttaga 1200
tttttgggag ttttcgggta gacggcgttt cggttcggat ttatttttgt tagtgtaggt 1260
ttttaggtga ggatcgtgtt gggcgatttt gggggtgcgg ggcggtacgg tggattttta 1320
ttgttttacg cgttgttcgg cgttatgtag gtgattttat tcggacggaa gaattttttc 1380
gaggttgggt tgtttttttt tttgttcgga ttgtggtttc gtcggggaga gcgggcgggg 1440
gagttcgcgt cgaggattgg attatttgta tagattagcg ggagtgcgcg cgttcgtttc 1500
gtatagggtc ggggtttgga ttaaattata tgaattggat tgagaggggg aagaagcggg 1560
gaggaagaaa tttcgtttta aacgttcgtt tttttttttt ttatttttgta atttattgat 1620
tagtttttgt tgggagacgg gtgtttttta ttcgcgggaa gggggcgggg tttttttttc 1680
gggtcgtatg cggggagagg ttgttttttt tttttttttt tgtttagtcg cggggtttaa 1740
gttttttttt ttcgttcgaa aggaggggag gggggattcg ttgttataag tttcgttttt 1800
tgtgttattt agtttcgttt cgtcgcgttc ggtcgtcggt ttttcgggtt atttgcgggc 1860
gggttttttt tttttttttt cgtgtttcgt gttttcgggg ttttatcgtt tttcgtgttg 1920
tggtcgtgtt cgtgtttcgg gggtaggggg cgtagaatgg cgtttttttt ttttttttgg 1980
tttcggggtt tgtatgggag aatttttttt ttacgatgtc gttgggcgac gtggcgtggg 2040
ggtaggggga cggtggggga gttttcgttt tcgattttcg gtcggttttt tcgttttagg 2100
cgttatttag tgattacggg taaagagaat tgttttaaaa agtttttttg ttgattgatt 2160

```
tttttttttgc gggggggggg ggggttgttt aattaattat ttcgagttag gagtttaggt    2220
tttttatttt ttaagtttag gtttaggttt ttagatttta ttcgtatttt tcgatgtttt    2280
gagattttta gattttacgt ttgataatga ggtttcgggg agtacgttta ttcggagaaa    2340
taaatatagt tcgtggaaag gggttggaag gtagggggag gtatatttgc gtcgtagtcg    2400
agcgattggg ttagggtttc ggcgttttta gtatgtttgt tagggttttg gagacgagaa    2460
cgttagaagt tagatagttg gtgtttaaag tttattttag gacgtttaga gattattaat    2520
ttgtttattt tatggacggg gaaatggagt tttagagtta tttgataatt tatagcgaga    2580
taagtatttt tttaaacggg gtttttatac gttttttttt tatatattta ttgataaagg    2640
gacgaggaga aatagttaac gtagtttttt gtgggatagt agagaaaaga ggtttgaggg    2700
gtattgtacg agtaaaatta ggtagttatt tagtatagcg tgcgagatta gtgttagttt    2760
tatttttttt tttttaattt ttttttttaa ttgaattttt tttatcggat ttttttttcg    2820
ttttgggtga tttcgttttt tagtagtttc gtttttttcga tttttatttg agttggtttc    2880
gtttattagg ttttaagttc gtattttaat tttttattta tttttgtttt ttggttttag    2940
taattttatt tggtgttttt cgagttaggt ttcggtttac gtgtttagag ttttggttac    3000
gttttttttt ttagtttcgt tttattagtt tatcgggtta ggtttcgttt ttattaggat    3060
ttattacgtt gggttcggta tttttttttag gtttttttag ggtttcgttt atgtttattt    3120
ggagcgtaga tcgtttcgta gattagcggg tagagatagt agaattgata gtgttggagg    3180
cggggacggg gagagagagt gagcgtgggg cgtggtcggg ggcggagtta ggatggggtc    3240
gggattagat tcggattaga ggttaggttt aggttaaggt tggagttagg atcgtttgtg    3300
gagttaaggt tgtattaggg gtaaggggtt gggttagagg ttaggattta gggttggggg    3360
tttttagagg gtttcggtgt attttgaggt ttagtggagt tttgagtgtg aggtgaggtt    3420
ttatttggta ttggtcgtag tgtttattta tgtttttttt attatagtga tagtttttat    3480
atttagtgta tcgttgggga tcggggggagt ttggttagat ggaaatttta acgttatttg    3540
ggaggtattc gtttttggtt tatttttgta ttttggacgt ttttttagat tttatttttt    3600
ttatttttaa tattgagttt tgtatttgtt ttttaaatta gttttttttt tttttttttg    3660
tttttggttt tttttttttt tttttttttt tttttttttt ttgaggcgga gttttttttt    3720
gtcgtttagg ttggagtgta gtggcgtaat tttagtttat tgtaatttc gtttttcggg    3780
tttaagcgat ttttttgttt cggtttttttg agtagttggg attataggtg cgtgttatta    3840
cgttcggtta atttttttgt atttttttagt agaaatgggg ttttatcgtg ttagttagga    3900
tggtttcgat tttttgattt cgtgtttcgt ttattttggt tttttaaagt gttgagatta    3960
taggcgtgag ttattatatt cggttttgtt tttggttttt tattttaggg cgattttatg    4020
ttttttttgtt agggtgggtt taggtgtttt ttttattcgt tttttgttta tttttttttt    4080
agtttattag tttttgtgtt attttttttt ttttcgattt tttttgttat agattttttgt    4140
ttttgttttt atattttaag atgtagtttg ggttttgttt tttttttttt ggattattgt    4200
tttagttttt tagtttttag tttgttttta ttttatatgt ttgtagaagg attttttttt    4260
ttttagaatt gattttggtt tgtttttgtt ttgagttttt ttatagtatt attttagggt    4320
ttttgggata gattttgggg atattttggt gggtggattt gggttgggga tatatattgt    4380
agaaggagta gtagttatat agggattttg gttggtagtt tttatatttt tgagtatttt    4440
gtggatttgc ggggatagga gggtagtagt gatttaggtt gattcggttt tttttttattt    4500
atattagttt aggttattta tttgtgtttt tatcgttttt tttttcgttg gaggtatttt    4560
tagtttttttt tttttttgttt agtgggttgg ggatgatggt gaagcggggt ttattttttt    4620
ttagtttttt tttttttttt tttttttttt cgttgtggtt tgggtttagt ggggttttaa    4680
ttttagtttt tttttttttt tttttttcgt tttttttttt tttttttttt tttttgtttt    4740
tttttttttt tttttttttgg tttaggttga ggttatgatt ttttttttta ttttttttat    4800
tttttttggtt tcggttgtta tgatgggtgt tttttttttt ttgttttgaa tttttatcgt    4860
tttttttatg ggagtcgggg tttttttttt tttttttttt ttttttagaa ttattttttt    4920
ttaaatggtt tttatttttg attattgtgt gttttggggg gtgatggttt atttttttga    4980
tggatttttt ttgattatgg ttagtttttt tattttggtg gttttgtttg tggttggggg    5040
tttggtggtt aagttttagta gagatttttt tatttttttt tttggggatt ttgtggtggt    5100
gatggtgagg gatttttgtt ttatatttat tttggaagtt tttttttatta tttttgtggt    5160
ttcgaggttg gtggtttgta atatagtggt cgaattggat tgtgattttt tttttttttt    5220
tttttttatt tataaggtta tggtggatat gttggggatt ttggtgttaa cgtttagtgg    5280
atatattttt attttttttt tcgtttttgt ggttttggtg tttgtggtta gggatatgat    5340
ggtggtgttt atttgagata gtttaattt tttttttttt gtggttttgg tgtttgtggg    5400
tttggtgttt atatttagtg gagatattat tattattatt gttatttttt ttatggtttc    5460
ggtgtttgtg gttggggtcg cgatgatggt gtttatttga gatattttta tttttttttaa    5520
tttcgtggtt ttggtgtttg tgagtttggt gtttatattt aatggagata ttattattat    5580
tattattatt attattatta ttattattat cgttattttt tttatggttt tggtgtttgt    5640
ggttggggtt atgatgatgg tgtttatttg agatattttt atttttttta tttttatggt    5700

ttcggtgttt gtgggtttgg tgtttatatt tagtggaggt tttttttttt tttttttttt    5760
```

tttttttttt tttttttttt ttattatttt ttttattatg gtttcggtgt ttatgtttga 5820
ggatatgatg gtgatgttta ttggatataa ttttattttt attttcgttt tgatggttgt 5880
ggtttttgtg gttggggagg tggtgtttgt gtttagttga gtttttcgtg tttttatttt 5940
cgtggttttt gtgtttacgg gtttgtttat tatgttttgt aaagattttt ttatttgaga 6000
tatttttatt ttcgattttg tggttttggg atttgtggtt tgggagtagg tgtttatatt 6060
ttttggagat atttttattt tttttttac ggtttgggtg gtttagaaa tgatgtttat 6120
tttttgtgga aatatttttg tttttatttg gatggttttt agggttgtgg aggtggtggc 6180
gaagttttgt tgatgttttt tcggagagtt cggcgatttt agtgttgttg tttcggtttt 6240
tgaagtttag tttattttttt ttgagttgtt gggttatggt cggggggagg agtaggttgg 6300
ttattttagt ttagaggata gaagcgtgta gttatggttt atggtggttt atggggacgg 6360
ataggtagta ttggttttag ttgtttttgc ggtaatgtgg ataaacgttg gggtttttgt 6420
tttttttgggg ttggtttttt tttttttttg tgttttttttt ttgtttttttc ggtttttgtt 6480
tattttttttt tggttttttgt tgtttggttt tggatatttt tttgaggttg ttttcggagt 6540
tttttgattt ttttttgggg tttttttttt ttattttttta gttaataggg tttttttttt 6600
tttttttttt tagttaaatt tatttttagt tttgagttat tttgggttag ttttcgtttg 6660
tttgtttttt gttttttttt tttttagttg gggaggggat tagtgagggg tttttttttg 6720
gttaggagac ggtggttaag ggatttgatt ttgaattatt aataagttta cgtttggtag 6780
ttgtaaagat aaaggttaga tttttagtag gtttttgggg gagtttgggg tatttggggg 6840
aggtagaaga gatttattag aggggagaga tttttgggac ggaaggattg ggggttcgat 6900
tgcggggtgt ttttagttgg aatgatacgt gttggtgaga gagtgatgtt agtattgagg 6960
ttttagaatg gggggaaagg aatatggttt ttaatatacg tgtttatgat tttttgtttt 7020
tggaatttag atttgggggt agggattggg ttaggttagg gttataaata tagttgggag 7080
gggtaggggg atttaggtta cggaggttat agttgttttt attatagagg gttggtagga 7140
gataagtggt tttgttcgtt tttgtgtgtt agtatttttt attttttatt ttttatgatt 7200
gtttttatta gggttttttt ttttgtttac gggtttttttt tttttttttaa ttttgttatt 7260
tgtttttttta gggtttttgt tttttttttta tgggattgtt tttttttttt attttgggtt 7320
tttgttttat ttttatttta gtgttagttt tttttttaagt ttgtgttttt tttttttttt 7380
taaattttttg gttttttttt tttgagtttt tgttttttttt ttaatttttt ggtttttgta 7440
tttttttttg ttttttgttt tagttaaggt gttttttttt tatttttggt atttattttt 7500
ttgggttttt gtttttattt ttttttagag tttttgtttt tttttgtttt agagtttttg 7560
tttatttttt tttgggtttt tgtttttttt tttttgggtt tttgtttttt tttttttggg 7620
tttttgtttt tttttttttg tggattttttg tttttttttt tttgggtttt tgtttttttt 7680
tttttgtggg tttttgtttt tttttttttg tggattttttg tttttttttt tttgggtttt 7740
tgtttttttt tttttgtggg tttttgtttt tttttttttt tgggtttttg tttttttttt 7800
tttgggtttt tgtttttttt ttttagggtt tttgtttttt tttgttttag agtttttgtt 7860
tttttttttt tgggtttttg tttttttttt ttgggttttt gtattttttt tcgtgggttt 7920
ttgtttttttt tttttgggtt tttgtttttt tatttttggg tttttgtttt tttttttttg 7980
ggtttttgtt tttttttttt tgggtttttg tttttttttt tttttgggtt tttgtttttt 8040
tttttttggg tttttgtttt tttttttttg ggtttttgtt ttcgtttttt tgggtttttg 8100
tttttttttt tttgggtttt tgtttttttt tttttttggg tttttgtttt tttttttttg 8160
ggtttttgtt tttttttttt tgggtttttg ttttcgtttt tttgggtttt tgtttttttt 8220
tttttgggtt tttgtttttt tttttttggg tttttgtttt tttttttttgg gtttttgttt 8280
tttttttcgt gggtttttgt tttttttttt tgggtttttg tttttttttt tttgggtttt 8340
tgtttttttt tttttgggtt tttgtttttt tttttttggg tttttgtttt tttttttttg 8400
ggtttttgtt tttttttttt tgtgggtttt tgttttattt tttttgggtt tttgttttat 8460
tttttttggg tttttgtttt ttttttttgg gtttttgttt tttttttttt gtgggttttt 8520
gttttttttt ttttttgttt atttttgggt ttttgttgtt ttattttttg atttttttgtt 8580
ttttaagttt ttgttttttt ttttttgggt taaattgttt ttttttttgtt tggtattttt 8640
tttttttgagt ttgttttttt ttcgttattg gtttttaatt ttttttgttt ttatttcgcg 8700
tttgttttttg gagttttttt tgtgtgtttt ttttttttcg gttcggattt ttgtattttt 8760
taggtcgttg tttttttgtt tttttatcgc ggtttgggat tcgttttttt ttcgtttttc 8820
gttttggcgt ttttaagatt tttcgttttt tagatttcgt ttcgttttag gttaggttgg 8880
aaagtggagg attcggtttg ttttgggcgg gtttggaagt agagtcggcg gagggagcgt 8940
cggggttttg ggttgtagga ggttgcggcg gtcgcggtag tatggtggtg tcggagaagg 9000
agtaggtgag cgtcggatta gggtttgcgg gagcgcggag ttggggattt cgtttttagg 9060
tttttagaga ggagggcgtt ggatatttag atttttgggt tagacggagg agggggatgg 9120
gagggtttag gttttagggt ttagggtatg ggggtcgaga tttttgagtt tggagcggga 9180
gggcgttggg ggttcggatt tttgtgttcg tggggagcgt acggggtggg tggttttgtg 9240
ttttatagga tagaattggg tgttttttta ttttattttt atttttatat ttgtttttgt 9300
ttttagagtt ggatttttaa gatttttaag aagaagattt gtacgacgtt tatagttgat 9360
tttatagatt cggggtgagg agttcgtttt tggattgatt ttagagggtt cgcggttcgt 9420

tgatttcgtt cgcggatggt tacgttttag ttttgttttt tttttatttt tggattcggg 9480
gattttttta agggcgtttt ttgtcgacgt ttttttttta ggagcgtttg ggtttgtgta 9540
gatattttttt atttttttatt cgttattttt tcgtttaggg ttagtgtaga cgttttaggg 9600
ttcggttttg tttttttttt tttcgatgtt ttttttatgt gtttttgttt tttcgtgtta 9660
ttttttgtgt ttttgttttt tttttttggg tatttttgtt ttatttattt atttatttat 9720
ttatttattt atttatttat ttatgtaaga aatttttatt tagcgttttt aaggtttttt 9780
tagtttaatt taacggtttt gttagggttt taattttttt tgggatacgt gttgtgtggt 9840
ttcgggtaaa ttattttatt tttttgggtt tcgtttttta tttgggtatt aaaagaggtt 9900
tgattttttg gggttattat gagaattaaa taagttaatg tttgtgatat ttttggtata 9960
gcgtttagta taggacgttg ggaaatattt gtagttttta ttgggaggtg gtaattatta 10020
tcgcgggcgg gttttgtttt ggtggtcggg gatattaatt gagttagata gggtgtgtgc 10080
gttcgcgagg agttttcgtt ttttagtgac ggttttttaa gcggatttag gtttttttagt 10140
tttttttttt tttttttttt ttttgagacg gtgtttcgtt ttgtttttta ggttggagtg 10200
tagtggcgcg atttcggttt attgtaagtt tcgtttttcg ggtttacgtc gtttttttgt 10260
tttagttttt cgagtagttg ggattatagg cgttcgttat tacgttcggt taattttttg 10320
tattttagta gagacggggt tttatcgtgt tggttaggat gatttcgatt ttttgatttt 10380
gtgattcgtt cgttttagtt ttttaaagta ttgggattat aggcgtgagt tatcgcgttg 10440
ggttaatttt ttgtattttt tttttagtag agacggggtt gtatcgtgtt agttagatgg 10500
tttcgatttt ttgattttat gattcgtcgg tttcggtttt ttaaagcgtt aggattatag 10560
gtgtgagtta tcgtatttgg tttaggtttt ttagtttttt gtttttttgtt tttttgagat 10620
agagtttggt tttgtcgttt aggttggagt gtagtgttgc gata 10664

<210> 168
<211> 10664
<212> DNA
<213> Artificial Sequence

<220>
<223> chemically treated genomic DNA (Homo sapiens)

<400> 168

tatcgtaata ttgtatttta gtttgggcga tagagttaga ttttgtttta aaaaaataaa 60
aaataaaaaa ttagaaagtt tgggttaggt gcggtggttt atatttgtaa ttttagcgtt 120
ttgggaggtc gaggtcggcg gattatgagg ttaggagatc gagattattt ggttaatacg 180
gtgtaatttc gtttttatta aaaaaaaaat ataaaaaatt ggtttagcgc gatggtttac 240
gtttgtaatt ttagtatttt gaggggttga ggcgggcgga ttataaggtt aggagatcga 300
gattattttg gttaatacgg tgaaatttcg tttttattaa aatataaaaa attagtcggg 360
cgtggtggcg ggcgtttgta gttttagtta ttcgggaggt tgaggtagga aaacggcgtg 420
aattcgggag gcggagtttg tagtgagtcg agatcgcgtt attgtatttt agtttggggg 480
atagagcgag atatcgtttt aaaaaaaaaa aaaaaaaaaa aaaaattaga aagtttggat 540
tcgtttgaga ggtcgttatt gaagggcggg agtttttcgc ggacgtatat attttattta 600
gtttaattgg tgttttcggt tattaagata gggttcgttc gcggtaataa ttgttatttt 660
ttagtgggag ttgtagatat tttttagcgt tttgtgttgg gcgttgtgtt aggaatatta 720
taagtattaa tttgtttaat ttttataata attttaggag attaagtttt ttttgatgtt 780
tagatgagaa acgggattta gagaggtgaa atgatttgtt cgaagttata tagtacgtgt 840
tttaggggaa attggaattt tggtagggtc gttgaattag gttaaaggga ttttgggagc 900
gttgagtgag ggttttttgt atggatggat gaatgaatga atgaatgaat gaatgaatga 960
atgaaataga aatatttaaa agggaaagat agagatatag aaagtgatac gaagagatag 1020
aagtatatag aagagatatc ggggaggaaa gaagtagaat cgagttttgg agcgtttata 1080
ttgattttga gcgggagaat ggcgaatggg gaatgagggg tgtttatata gatttaaacg 1140
tttttagagg gagagcgtcg gtaaggaacg tttttgggaa ggttttcgag tttaggaatg 1200
gggaaggagt agggttgggg cgtgattatt cgcgggcggg gttagcgggt cgcggatttt 1260
ttggggttag tttaggggcg aattttttat ttcggatttg tggagttaat tatgaacgtc 1320
gtgtaggttt tttttttgaa gattttgggg atttagtttt ggggatagga ataaatgtag 1380
gggtggaagt ggggtgagag ggtatttagt tttgttttat gggatataga gttatttatt 1440
tcgtgcgttt tttacggata taggagttcg ggtttttagc gtttttttcgt tttagattta 1500
ggagtttcga tttttatgtt ttggatttttg gaatttggat tttttttattt ttttttttcg 1560
tttgatttag gaatttgggt gtttagcgtt ttttttttttg ggagtttgga ggcgaggttt 1620
ttagtttcgc gttttcgtag atttggttc ggcgtttatt tgtttttttt tcggtattat 1680
tatgttgtcg cggtcgtcgt aatttttttgt agtttagggt ttcggcgttt ttttcgtcgg 1740

```
ttttgttttt agattcgttt agagtaaatc ggatttttta ttttttagtt tagtttgggg    1800
cggggcgagg tttggggggc ggggagtttt ggagacgtta aagcgggagg cggggagagg    1860
gcgggtttta ggtcgcgata aggggataga gggatagcga tttggggggt gtagaggttc    1920
gggtcggggg gagagagata tataggggag attttaaggg taagcgcgag atgggaatag    1980
aaggagttgg gggttagtgg cggagaggga atagatttag aaaggaggat gttagatagg    2040
gaggggataa tttaatttaa agagggggag ataaagattt aggggataga gaattagaag    2100
gtggggtagt agggatttag ggatagatag agagagaggg ggatagagat ttatagagag    2160
agggggatag agatttaggg agaggggata gagatttaga gagagtggga tagagattta    2220
gagagagtgg gatagagatt tatagagaga gggggataga gatttaggga gaggaggata    2280
gagatttaga gaggggggga tagagattta gagagggggg gatagagatt tagggagggg    2340
gggatagaga tttagggaga ggggatagag atttacggag agggggatag agatttaggg    2400
agaggggata gagatttaga gagaggggga tagagattta gagagagggg gatagagatt    2460
tagagagacg ggatagagag tttagagaga gggggataga gatttagaga gaggggaata    2520
gagatttaga gagagaggag gatagagatt tagagagagg gggatagaga tttagagaga    2580
cggggataga gatttagaga gaggggggata gagatttaga gagaggggaa tagagattta    2640
gagagagagg aggatagaga tttagagaga gggggataga gatttagaga gggggggata    2700
gagatttagg gatgggggga tagagattta gggagagggg atagagattt acggagaggg    2760
gtatagagat ttagggagag gggatagaga tttagagaga gggggataga gattttgaga    2820
tagaggggga tagagatttt gagagagggg gatagagatt tagagagagg ggaatagaga    2880
tttagagaga gagggggata gagatttata gagagagggg aatagagatt tagagagagg    2940
gggatagaga tttatagaga gagggggata gagatttata gagagagggg aatagagatt    3000
tagagagagg gggatagaga tttatagaga gagggggata gagatttagg gagaggggga    3060
tagagattta gagagagggg gatagagatt tagggagagg tggatagaga ttttgagata    3120
gagggggata gagattttga gagagagtgg ggatagagat ttagagaggt ggatgttaga    3180
gatggggagg agatattttg attgaggtaa ggggtagagg gggatgtaaa aattaaagaa    3240
ttgggggaag ggtaggaatt tagaaaggag gggttagaga tttaggggag agagagggat    3300
atagatttgg gggaggattg atattaagat aagagtggga tagaagttta gagtgagagg    3360
gagaggtaat tttatggagg agggataggg attttgagag agtagatgat agaattgaag    3420
agagaggagg attcgtgaat aggaaaggag attttagtgg gggtagttat gaagggtgag    3480
gagtaggaaa tattgatata tagagacggg taaggttatt tgtttttttgt tagttttttg    3540
tgatggggat agttgtggtt ttcgtaattt gagttttttt atttttttta gttgtattta    3600
tagttttggt ttagtttagt ttttgttttt agatttgagt tttagggata ggaggttatg    3660
ggtacgtgtg ttgggggtta tgtttttttt tttttatttt agggttttaa tattgatatt    3720
atttttttat tagtacgtat tattttagtt ggaaatattt cgtaatcgaa tttttagttt    3780
tttcgtttta ggagtttttt ttttttgata agtttttttt gttttttttta ggtgttttag    3840
gtttttttaa aaatttgtta aaagtttagt ttttgttttt gtagttgtta aacgtgagtt    3900
tgttggtagt ttaaagttaa gtttttttggt tatcgttttt tggttaggga gagatttttt    3960
attggttttt tttttagttg ggaggaggag gagtaggagg taggtaggcg gggattgatt    4020
tagaataatt tagggttgag agtaaattta gttggagaga ggggagggga aggattttat    4080
tggttgggga atggggaggg agggttttag aggggggtta gggggtttcg gagatagttt    4140
tagaggggtg tttagagtta ggtagtaagg attagaggaa ggtggataga gatcgaaagg    4200
ataaaggaga gatatagaga aaaaaagaga ttaattttaa agggataaag attttaacgt    4260
ttgtttatat tgtcgtagag gtagttggag ttagtgttgt ttgttcgttt ttatgggtta    4320
ttataggtta tggttgtacg tttttgtttt ttgggttggg gtggttagtt tgtttttttt    4380
ttcggttatg atttagtagt ttagagggga tgggttaggt tttagaaatc ggaataatag    4440
tattggagtc gtcgggtttt tcgaggaggt attagtagag tttcgttatt atttttatag    4500
ttttagagat tatttagatg agaataagga tgtttttata gagaatgggt attatttttg    4560
gagttattta gatcgtgaaa aggaggatga agatgttttt aaggaatatg ggtatttatt    4620
tttaggttat aggttttaag attataaagt cggagatgag ggtgttttag gtgaggaggt    4680
ttttgtagag tatggtgggt aggttcgtgg gtatagaggt tacgggagtg aagatacgga    4740
agatttagtt gagtataggt attatttttt tagttatagg agttatagtt attaagacga    4800
ggatgaggat gaagttgtgt ttagtgagta ttattattat atttttaggt atggatatcg    4860
aggttatgat ggggaagatg atgaaggaga agaggaggag gaggaggagg aggaggaaga    4920
ggaggttttt attgagtatg gatattaggt ttataggtat cgaggttatg ggagtgaaga    4980
ggatgaggat gttttagatg gatattatta ttatggtttt agttataggt attaaggtta    5040
tgaagaagat gacgatgatg atgatgatga tgatgatgat gatgatgatg atgatgtttt    5100
tattgaatat agatattagg tttataggta ttaaggttac gggattgaag aggatgaaga    5160
tgttttagat ggatattatt atcgcgattt tagttatagg tatcgaagtt atgaagaaga    5220
tgataatgat gatgatgatg tttttattga gtatggatat taggtttata ggtattaaga    5280
ttatagaaag gaagaggttg aggttgtttt aggtgaatat tattattatg tttttgatta    5340
taggtattaa ggttatagag acgaggaaga agatgaggat gtgtttattg aacgttggta    5400
```

ttagggtttt taatatgttt attatggttt tgtagatgag gaagaggaag aagaggagat 5460
tatagtttag ttcggttatt atgttgtaag ttattaattt cgaggttata agagtgatga 5520
agaggatttt taagatgagt ataaaataga agttttttat tattattatt atagagtttt 5580
tagggaggaa gatgaggagg tttttgttga gtttggttat taggttttta gttataggta 5640
aagttattaa gatgaagaaa ttggttatgg ttaaagaggg tttattaaag agatgagtta 5700
ttatttttta ggatatatag tggttaagga tagaagttat ttgaggaagg atgattttga 5760
ggaggaaaag gagaaggaag aggatttcgg tttttatgag gaagacgatg aaagtttaga 5820
gtagggagag aaaggtattt attatggtag tcgggattag gaagatgagg aggatgagga 5880
ggaaggttat ggttttagtt tgaattagga ggaggaagaa gaggaagata aggaggagga 5940
ggaggaggaa gaagacgagg agaggaggga agagagggtt gaggttgggg ttttattgag 6000
tttagattat agcgaggagg aagaggagga ggaggagggg ttggaggaag atgagtttcg 6060
ttttattatt atttttaatt tattggatag gagagaggag gttggaggtg tttttagcga 6120
ggaggaaagc ggtgaggata taggtaagtg gtttgggttg gtgtgggtgg gaaggaatcg 6180
agttagtttg ggttattgtt gtttttttgt tttcgtaggt ttataggatg tttaggagta 6240
tgggaattat tagttagggt ttttgtgtgg ttattgtttt ttttgtaatg tatgttttta 6300
gtttagattt atttattaag gtgtttttag aatttgtttt aggggttttg ggtggtgtt 6360
gtgggagggt ttagagtaag ggtaggttag ggttagtttt gaaagaaaag agattttttt 6420
gtaggtatgt gaggtgagga tagattgaag gttgggaggt tggggtagtg gtttaggaag 6480
gagaggataa gatttaagtt gtattttggg gtgtggggat agagataggg gtttgtggta 6540
aagagagtcg ggggaaggag ggatggtata ggggttgata ggttaggggg agaataggta 6600
gagagcgggt ggagagagta tttgagttta ttttgatagg gagatatggg gtcgttttga 6660
gatagaaaat taggggtagg gtcgggtgtg gtggtttacg tttgtaattt tagtatttta 6720
agaggttagg gtgggcggaa tacgaggtta ggagatcgag attattttgg ttaatacggt 6780
gaaattttat ttttattaaa aaatataaaa aaattagtcg ggcgtggtgg tacgtatttg 6840
taattttagt tatttaggag gtcgaggtag gagaatcgtt tgaattcggg agacggaggt 6900
tgtagtgagt tgaggttgcg ttattgtatt ttagtttggg cgatagagag agatttcgtt 6960
ttaagaaaaa aaaggaaaaa aagggaaagg aaggaaatta ggggtagggg aggggagggg 7020
aggttggttt ggggggtaga tatagagttt agtgttagag ataaagaggg tagagtttgg 7080
ggaagcgttt agggtgtagg ggtgggttag gggcgggtat tttttaagtg gcgttagagt 7140
ttttatttaa ttaggttttt tcggttttta gcgatgtatt gaatgtgaga gttgttattg 7200
tgatgaggag aatatgggtg agtattgcga ttagtgttag gtgagatttt attttatatt 7260
taaggtttta ttgggtttta ggatgtatcg ggattttttg aggattttta gttttaaatt 7320
ttggtttttg atttaatttt ttatttttga tgtaattttg attttataag cgattttgat 7380
tttaatttta atttagattt agtttttggt tcgggtttgg tttcggtttt attttaattt 7440
cgttttcggt tacgttttac gtttattttt ttttttcgtt ttcgttttta gtattgttag 7500
ttttgttatt tttgttcgtt ggtttgcgaa acggtttgcg ttttaggtga gtatgggcgg 7560
ggttttggaa aaatttgagg aaggtgtcgg gtttagcgtg gtgaattttg gtgggggcgg 7620
agtttggttc ggtaggttgg tggggcgggg ttaagagggg aggcgtggtt aaggttttga 7680
gtacgtgggt cggggtttgg ttcgaggaat attaggtgga gttgttggag ttaaggata 7740
gaaatgagtg gagggttgag atgcgggttt agagtttaat gggcgaggtt aatttaagta 7800
ggaatcgaag gggcggggtt attggagaac ggaattattt agaacgaaaa aggggttcgg 7860
tagaagggat ttagttggaa gaaggaatta gggaaaagga ggtggagtta gtattaattt 7920
cgtacgttat attgagtggt tgtttaattt tgttcgtgta gtattttta agtttttttt 7980
ttttgttgtt ttataggaag ttacgttgat tatttttttt cgttttttta ttagtaagtg 8040
tgtaaaaggg gagcgtgtaa aagtttcgtt tagagaagtg tttgtttcgt tatgagttat 8100
tagatgattt tgggatttta ttttttcgtt tatgaaatgg gtaggttaat gatttttaag 8160
cgttttgggg tgggttttga atattagttg tttaattttt ggcgttttcg tttttagggt 8220
tttggtagat atgttggaaa cgtcggaatt ttgatttagt cgttcgattg cgacgtaggt 8280
gtattttttt ttgtttttta attttttttt acgagttata tttatttttt cgaataaacg 8340
tgtttttcga aattttattg ttagacgtgg ggtttgggaa ttttagggta tcggggaatg 8400
cgggtagggt ttagagattt ggatttagat ttgagggatg aggggtttgg atttttggtt 8460
cgaggtggtt ggttgaatag tttttttttt tttcgtaaaa aaaaaattag ttaataagga 8520
agtttttttga aatagttttt tttattcgtg attattgagt gacgtttggg gcggggaggt 8580
cgatcgagag tcgggggcga gggttttttt atcgtttttt tgtttttacg ttacgtcgtt 8640
tagcggtatc gtggaaagag gattttttta tgtaaatttc ggagttagag gagaagggga 8700
agcgttattt tgcgtttttt attttcgggg tacggatacg gttatagtac gggggcggt 8760
gaggtttcgg ggatacgaga tacgggggag ggagagggga attcgttcgt agatgattcg 8820
ggggatcggc gatcggacgc ggcggggcgg agttgagtgg tatagggggc gaggtttgta 8880
gtagcgagtt tttttttttt ttttttcgga cgaagaagga agatttgggt ttcgcgattg 8940
ggtaggaaaa aggggaggga gtagtttttt ttcgtatgcg gttcgggagg gaagtttcgt 9000
ttttttttcg cgggtaaagg atattcgttt tttaatagaa attgattaat aaattataaa 9060

```
gtagagaaaa ggaaagcgga cgtttggggc gggatttttt ttttttcgtt tttttttttt     9120
tttagtttag tttatgtggt ttggtttagg tttcggtttt gtgcgaggcg ggcgcgcgta     9180
ttttcgttgg tttgtataga tggtttagtt ttcggcgcga gttttttcgt tcgttttttt     9240
cggcgaggtt atagttcggg taggagaggg aatagtttag tttcgggaag atttttttcgt    9300
tcgagtgagg ttatttgtat gacgtcgggt agcgcgtgga gtagtgaaga tttatcgtgt     9360
cgtttcgtat ttttaaggtc gtttagtacg gtttttattt ggaggtttgt attagtagga     9420
ataggttcgg atcgaaacgt cgtttgttcg ggaattttta gaagtttggt ttggagggag     9480
agcgagtagc ggtttagttt ggagtatttt tttttttgtt tttttttagg attttttttt     9540
ttggattttt tttttcgatg ttataacgat cgtcgggttt tatatgggtt tttttttttt     9600
tttcgtagtt aaggatttaa attcgtattg ggcgagttta ttttcgtttt gggaaggggg     9660
agtgggattt atttatttta ttttgacggg ggagttcgga acgttggagt agtttatttt     9720
ttaggggtgg gcgagaaagg attaggttat tttagaattc ggattattga ggtggtttat     9780
gttttatttt agggaagcga ggaagggttg tcgtatttgg gagtttttta tttttttggtt    9840
gtagtttgcg gagagggagt ttcggagagt ttagggtttt cgttgtagtc gaacgaaagt     9900
tggggggtaa tattttagtt gagtcgggag ttacgtttcg gaggtttttt tttcggaata     9960
tttttcgtta ggatggggag cggttgaaag atttggcgtt gttttaggta taggagtcgg    10020
atagtcggtg agggattttt gttttgtttg atttcgtttt tttttttgat tttatttttt    10080
ttcgggtttt attttttttt taatttcgtt ttttttttgg tttcgttttt aatcgtgatt    10140
attaatagtt aggtttcgtt tttttagttt agtttttcga ggagtcgttg ggagtagagt    10200
tttttgagtt gttgtcgcgt ttatttcgtt tagtcgtttg tttgtgttta aggagataag    10260
gttgttgaat ttttttttta gaagttgtcg ggtttggaaa gaggaggtgg agggggcggt    10320
tggagcgtgt agtttgcgag gtggggacgt ttagtatgtt tcgggtttag cggtagttta    10380
tttgtgtatt ttgcgtgggg atttgtagga gtaaggttag gtcggagtag tcgaaggttt    10440
cgtttagggc gagtagtgtt tcgtgtattt cgtttttcgt gaggttcgtg gtaaattttt    10500
ttaggtttag ttcggatatt taatttatga ttcgtttatt ggattatatt attacgtttg    10560
gggacggaag ggatttttag tcgtgttttt ttcgtggttt ggagagttta gtttagttta    10620
tatttttatt tttatttggt tttttcgggt tacgtttttt acgt                      10664
```

<210> 169
<211> 22069
<212> DNA
<213> Artificial Sequence

<220>
<223> chemically treated genomic DNA (Homo sapiens)

<400> 169

```
tatcggtgtt gcgagagaaa ttttgagcgt tggtcggtga gttaggttta gatggggtta       60
gatggggcgg tcgtattagg ttaggttttt tttaattttg aattcgacgt tgggtagaga      120
atagttttat tatttcgtag tttagggcgt agattttcgg gtgggtgttt tttgagagtt      180
ttttttaaa ggtttttacg aagtttttaa ggcgtttttt tcggttattt tgtacgtgtt       240
gttgggggtg gagaaggtag gtgagggtag tttcggggtg tagtgggggc gggggtggta      300
gtagtagtat atttttgagt ttttcgggat tattttattt atttattacg tttatttatt      360
ggttgggatt ttggtgagta ggtggggaag ggggtttgtt aagaagatat agggaggacg      420
gtttttttagg atgggaagtg gttagggata gggtttgggg tttacgggtg ttacgttttt     480
tgggtaatat aagaaaggtt gttcgggggt ttagattttg agtttaggat tgtgttgtgt      540
ttcgagattt tatattaacg atggggaggt agaagtttgt tgagaatagg tagaggggat      600
tgggacgtta gggtttattt ttgcgatttg gtagtcgata gagtcggcgg tagtcgagta      660
gtatatgtat tgggtttttt agttttcggt tattgtaggg gatatagata tatagttagg      720
gttcggttag gtttatttta tagttttcgg ggtattgatt atttgtattt atcgagtttt      780
attagggagt gaggattgag gataggaggt tttagttttt tggtggagtt ggaagtagat      840
taggtttcgg tagtcgtgtg gtagttagga cgtttaggtt tggatttagg gagattagtg      900
tttttttgtag aggttttttg gtagcgttgt ttcgggtagt attttttagg tatttttgag     960
gcgatggttt taggggtcgt tatagggttg tgtaaggttg gttatgtgtt ttgagcgtgg     1020
ttgcgtggtt gtttgggggga ttgaatagaa ttattaggtt aagggtggga taagtttttt    1080
cgtttttggg gttagtaatt tttatatttt tatggattaa gggtatagga aggtagggat     1140
atagggtttt ttaggtggtg gtatttgaat tttgttatgg gaaagttttt gttttatttt     1200
tgtataaaga ataggtggag agaaaatgag gttaagggtt agtttcggtt ttgttaaaga     1260
cggtgtagtt tagggatttt gagtgggatg gagggtttgg gtaaagtttt attttagttt     1320
tgggggtttt aagggagggt ttaggtttcg ggggttatgg gtgggaaggg gaaggattcg     1380
```

```
ggtaaggttt tatttcggtt tcggcgtagt cgtttttagt ggtaataata tttttcgttt    1440
cggatgtagc ggtttgaaga ggatacgagg tattcggtgt tatagcggta gtaggttttg    1500
taggaagttg tgggtgggga tttaggtgga agttgaggtt tacgttgggg ttcgtatgtt    1560
cggttttgtt tatatttcgt atttgtttat ttaggtcgtc gtaggttagc gatttatagt    1620
ttttgggttt ttttttttta gttgtgaaga tgattgcgtt ttcgggtcgt tttgggtgcg    1680
ggaaggggta gtcgtttttt ttgagttggt tttgggtgag taggtatttt ttgaggcggt    1740
tgtatagggt agtttttgtg gataggtata gtggttagtt tttgtttggg atggtttacg    1800
gggcggtaga tattatcggg aaaggcggtt gggtcggtag gggtttacga agtatcgtgt    1860
ggtatagtag ggttgggtc ggtagaggtt tacgaagtat cgtgtggtag agtaggggta    1920
gggttttatt ttttaggttt tttattcggg ggttgtttgg acggttgagc gagaagttgg    1980
ttttggcggt taatttgttt tttaatatta tttcgtggga tataattttg cggttattgg    2040
tttttggaag gaagggaggg aggggaggag ggtgtgaagt agttggttta gatttttgtt    2100
tcgtttgttt ttttatttat tttgtttttt ttagattttt gttttaattt tttttttttg    2160
ggatagtatt ttattgattt cgggttttag tttgattcga ataggagagg tagatatgcg    2220
gtgagttagg gacgtagggt agtcgttagt tttgtaggtt ttagagttgt acggaggttt    2280
tatatttaaa gttttcgttt ttttttttta agcgaagggg gaagggcggg gagaatagta    2340

cgggggattt aatggttttc gagggttttg agtttttatg ggttatttag tttgcgacgt    2400
taatttaggg ttttttcgag tgttaatgta ggcggggacg ggggtagggg tcggtgatag    2460
tttaatgtag tttggagagg ttgtaatgaa acgtttaggt tttcgttttt ttttttttta    2520
gagtttaatg tatggagttc gtttagtttt gggtttttttg ttttgaagaa gttgtcgtag    2580
ttttattagt aggcggtttt tcggttcggt ttggagattt tatttagcgg aggaaggcgg    2640
ttgatagcga ggatattagg gttaggttcg ttttatatag aagtttggga attaggtagt    2700
aggtagtttt tttatagtat cgcgtttttt ttttgtttga gattgtggtt gagtttaggg    2760
ttgtgaaggt tttagcgagg gttttagttt agttgttatt ttaagagtaa ttttgagttt    2820
ttttcgttta ttcgttagga tttattgttg aggtcgggta tagcgttggg ggttaggttt    2880
ttgagttttt tgagggtgtt tacggttata tttaggtaga tgtttttgtt ggggttgcgg    2940
ttataggtta ttttttttttc gtttagtgtt tgggggatag gcggtgttta gttgaggtta    3000
tgtttgtttg gtgtcgggga gttttagcgg tggggtcgtt atggggtcgg ggatttgtat    3060
ttttttttagg tagtttcggg ttatagttgc ggttgagagg tcgggggatg tttggggatt    3120
attttggatt tttagttttt attcgatcgg ttagatagga attttaggat tttatttttt    3180
ttatggtttt ttggttggag gtatagaatt tgagatattt ttcgatgaat aggttgagat    3240
agcgttggcg gatgacggtg gggattttgt ttttaaattt tttggggata atgggttttt    3300
ttaaatttta gagggaaggt aaaagttgtt atgggtgagg gtttttataa ggtttttagc    3360
ggggaacgta gacgcgatga ggtcgtgttt atggttggag gtaagagtga ggtgcgttgg    3420
gatagattag tttggttgta gtagggatag atacgttaag gtaagatagg agtagtaggg    3480
gaaattgagg gtagcggaat taaggttatg ggaaattgag gttatggggg tataagggaa    3540
tttgttgtat tttttgttta atttttttag aaatttaaag ttgtttaaaa aatataaagt    3600
ttattaatta aaaataaaat aaggttaagc gtggtggttt acgtttataa ttttagtatt    3660
ttgggaggtt gaggtaggag gattatttga ggataagagt ttaagattag tttggataac    3720
gtggtaaaat tttgttttta taaaataaaa aataaaatta ttagttgggg tgtgatgata    3780
tcggtttgtg gttttagtta tttaggaggt tgaggtggaa ggattatttg agtttaggag    3840
gtcgaggttg tagtgagtta tgattgcgtt tttgtatttt agtttgggta atagagattt    3900
tgtttttaaa taaaaaaaaa aaatattaat taattaaaaa aataaaataa gataaaataa    3960
aaaaatatcg ttagggcggt cgggtatggt ggtttacgtt tgtaatttta gtattttggg    4020
aggtcgaggc gggtggatta cgaggttaga agatcgagat tattttggtt aatatggtga    4080
aatttcgttt ttattaaaaa tataaaaata gttaggcgtg gtggcgggcg tttgtagttt    4140
tagttattta ggaggttgag gttagagaat ggcgtgaatt taggaagtag agtttgtagt    4200
gagttaagat cgtgttattg tattttagtt tgggcgatag agcgagattt tattttaaaa    4260
aaaaaaaaaa aatattaagg tatggggagg gaaggtgaag gttgtattgc ggtgtggagt    4320
acgaaggttc gtagggtagg ggcgaggttg gtttttcgta ggttttatgt ggtcgggttt    4380
tagggcgtgg ttttgggggt ggtggttttg gtcgagatgg gaggggttat ttgtaaggat    4440
ggattgtggg cgattcgttt agggatgatg gtggtggtag ttttggacgt tattttcgat    4500
aatgtgcgtg tttttagttg ttggttattt ggtttagggt cgttggagga ttggttgtcg    4560
ttggtcgtaa gggttttttt ggtatttgtg ggggttggcg gggtataggg ggtgtgggta    4620
tagggcgagt ggttgtttat tattacgtgg cggagtatta ggtagtcgcg gtcgtttagc    4680
gttagtagta cgtgtatttt cgcgtgtagg gggtcgggtt agggtgaggg gttggggttg    4740
cgtaatggta ttaggtttta gttgttttta ggtaagttta tcgaggtaga ggatataggt    4800
taggttgcgg aggtcgttga ggatacgtta tcggtggggt ggtttaggtt tttgttacgt    4860
gttttgattt taagtttaag gcgtcgatgt ttgagggaaa ggttttggtt ttcggataga    4920
ggatcgaagg gtttcgggtg tttttatttg tttattggtt ttgttttgtt ttgcggtttt    4980
```

```
tgtgttttgt tttgtttttt gatgaagtta taaattagag ggaggaggtt aggtttgtag    5040
gggttgtttc ggagggttgg ttacgcgggt agttgtaatt tgggtatgta cgtttgtgtg    5100
gtaggggggt ttttggattg ggggttcggt atcgatttag gaaggggagt tgtgagtagg    5160
gatatttggt tttagtttta gagtaatatt tttcgaaatg ttatttggtt ttggaaggtg    5220
taagggaggt aggatgagtt tgtttatgtt atcgcgggtc gtttagtaag gaagtaggtt    5280
gttcgttagg ttggtacgcg ttttttgtag tggagggttt gttttttagg aacggataga    5340
gaatttttag attttttcgg ttgtacgttg ggggcgagtt taggtagtta taggagtttt    5400
ttaagttaga tgagttcgtt ttgcggtatt gttagtattt gggacgttag atttttttta    5460
ggcggcgggt tttaagggta ttgcgatagt ttagtattta ttatagatta gtaataggat    5520
aattcgagcg cggagatata gacgggaagc gtgtggggtt ttgggatagg tttaatttaa    5580
tgattttttt tttttggggt taaggtttta gtcgtgaggg ggttttgggg aggggaggtt    5640
agagtagttt ggagagtttt ttttaaggag ggtcgtggga tttatgggat ttgtagggga    5700
atcgtcgggg ttggttttaa ggttttttag ttagcgttag ggaggtaggg gttttaaatt    5760
agtaggttgt ttagggtggt tttcggatag tagttatgtt tttttaggga gtttgttaga    5820
tatatagatt ttttttagtt tttagattag aatttgtatt ttttaggagt tttttggggg    5880
atttttattt gtgatttgtt tttagggata ttttttcgtt ttatagatat tattgatgtg    5940
aagacgtagg agataggata atttttcgtg aagggttttg tttatttatt attgaggttt    6000
ggttcgattt ttttataagat tttgttgggg gggaagtgtt tttcggagta aaggaaatat   6060
agttttattt ttgggaagat agtatttatt tttattagag gttacgtttt ttatttatac    6120
gttaggagaa agttatattt gtagaagttt gttttttatt taatgttagg tatttcgata    6180
tggtttttta ttttttggaa ttataggttt aggttttggt ttagtttagt ttttagtagg    6240
ttgttggatt tttagtaatt tcggagttgg gttagggaag gaaggtgttg ggttaggttt    6300
tttgtatttt ttaatagttt ttattttata gataggtagg ttgagggttt tagggagggg    6360
gtttaaagtt attttatgag taagtattat gtggtttagg tgagtttaga gattttaggg    6420
tgttattttt tttgtttata tgtttacggt ttttcggtat aggtttttttg ggttaggatt   6480
tatttgtagt taggcggggg ttggggatgt gggcgatttt ttttttttcg ttaggggcgg    6540
aaatgtggac ggagggcgat ttttttgtta gtttggcggg ggtttgtagt aagttcgtcg    6600
atgtttttttg cgtttgttgg gttcgtaggt agtatatttt ttgcgtcgtc gggggtcggg   6660
tcgggggtat cgcgggattt tttttcgggg gttttatttg cgttattata aatgttaagg    6720
tttggtagta tagcgggggt atttggagta ggcgatttcg ggtaggtttg ggagagggtg    6780
gttggagttc gggggtcgga ggtattattt ttttggttgt tttttgagtt gttgggtata    6840
ttggggggaa gtagtagggg gtagtggttt ttaatgttat gtggttttag gttttttttat   6900
ttttagtgtt tttcggtgtt taggttatgt ttttagtttt gataaggtac gggtgggcgg    6960
gattatttcg attatattgg tattttaatt taattttatt acgtttaatt ttttttaaag    7020
tttttttttt ggggaggttt tgggattgga gtaattggga ttttttttggt tgttgatttc   7080
ggagttaggt tttttgtttg ttttgtatta tttcgttacg tttgtatagg ggtttgataa    7140
gcgttattgt tttcgggtta tagaggatat tggagtttag agttggataa tcggtttagg    7200
tttaggtcgt atacggcgta gtaggtcgtt tgtcgtattt tgggggaggt tattttgggg    7260
ttgttgattt gttttgtttt tcgttttagt atcgtggtaa tttaatagga aggggtaggg    7320
ttagtttttt ttggaattcg ggtagcgtta aagataaggt gtttttaaaa agtttatgga    7380
aaacgtgcgt tgtgacgaaa tttgtatggt ttttaagttt ttttgtttta aaataaattg    7440
atattaattt gttataatat gtttgaatta gacggagttt gaggtattaa aaaaaagtaa    7500
gatattagtt agaatagagt tttagttaga gtaataggag ttttgttaaa attgaagtaa    7560
atgttaagta ttaaatttat ggtaaaattt gggtggaaga acgaatgggg aaattattga    7620
tgttttataa aaagcgttat agatttatag gaataatgtt ttaaagaaat tagaagttta    7680
tgaatggata atttatttta tgtatttatt tttttgagat agagttttgt tttgtcgttt    7740
aggttggagc gtaatggtac gattttggtt tattgaattt tttattttttc gggtttaagt   7800
aattttttttg ttttagtttt tcgagtagtt gggattatag gtgcgtgtta tcgcgtttag   7860
ttaatatttt gtatttttag tagagacggg gttttattat gttggttagg ttggtttcga    7920
atttttgatt tcgtgatttg tttatttttg ggcgtagtgt tttatgtttg taattttagt    7980
atttgggagg gtcgaggtag gtagattacg agattagttt ggttaatatg gtgaaatttc    8040
gtttttatta aagatataaa aattagtttg gtgtggtggt gggtatttgt aattttaggt    8100
attcgggagg ttgaagtagg aaaattattt aagttcggga ggcggaagtt gtagtgagta    8160
gagatcgtgt tattgtattt tagtttgggt aatagagcga gatttcgttt taaaaaaaaa    8220
aaaaaaaaaa aaaataatta aaaaataaat aaatagaaaa tagagggatg aagttgggag    8280
tagtggttta cgtttgtaat tttaacgttg ggaagtcgag gtaggaggat tatttgaggt    8340
taggagttgg aagattagtt tgggtgataa agtaagattt tttttttta taattttttt     8400
tttttgagat agagttttgt tttattattt aggttggagt gtagtggtgt aattttggtt    8460
tattgtaatt tttgtttttt gagtttaagc gatttttttg ttttagtttt tttagtagtt    8520
ggtattatag gtatttatta ttatgtttag ttaatttttg tatttttagt atagatgggg    8580
ttttgttatg ttggttaggt tggtttcgaa tttttggttt taggtgattt attcgttttg    8640
```

gttttttaaa gtgttgggat tataggcgtg agttattaaa ttaggttaat ttcgttttta 8700
aaaaaaaaaa aaaaaaaggt ttttgttggt ttggtttttg cggattttat tttttggttt 8760
tgtttggaaa ggtgggagat tttttttttt tgttcgggga atagagtggt tagattcgaa 8820
agtttttttt ttttattttt tttttttttg gggggttaag atatatgttc gttcgttagt 8880
ataggtttgt tttcgttgtt aatattaata tattttaatt ttaaattgtt ttcggtaagt 8940
ggggaatgga atagtttagt ttttaggtat agtagttgag ggtttagggt taatagtttt 9000
tgggttttgt gtgggtgtag tcggaggggt gggtaggtgt tatacgtttt gtagatgttt 9060
aaggttattt tttttgggta tttagggttt tagtttggag attttagggt tgtagaatga 9120
aagtatattg ggaaagcgag gttattttgg gtcgaattag ttgttgcggg ttgagtattt 9180
gggtttagtt tgtgtggagt gagttttttt tggtggttaa tttggggata cggggttagg 9240
agatttaggg gtttagggtt ggtttaggag agtaagagtt cgttacgttg ggagtaggga 9300
ggagggaggg gaggagaaag gagtcggagg ggatgaggag gaggtaagga gaggagatag 9360
aggagttgga agagaagaga tagagatggg ggaagggagg aggggagaag agaggggaa 9420
gggaggaaag gggaggcgag gggagagggg ttagaagggt gtgtcggagg tggatttttt 9480
tgaggtgggt gggtgggtta atattttttt gttagttagt gggaatagat cgtgtgtacg 9540
tggttataga agtttggttg gtggttacgg gttagtgatt tttgggtggg tgaggggtaa 9600
taggagtaat agggttgttc gggtgttttt gagtttttga gattttaacg ggtttaggga 9660
ttagtattga ggagttgttt ttatttcgtg ttttcgagtt aattggaaat tattttagat 9720
agaaggtttt ttattaggtt ttcggttttg tttttgttcg agtttagttt tagtattcgc 9780
gcgttttatt tgtcgggtta gtcggttttt gtttttcgtt ttgacgttgg tggattcgtt 9840
gaagattcgt aggtattttt ttatggggtc ggagttaaag tttatttttt tttttagggt 9900
cgagtagttt atattcgttt tcgcgttgga ggtggaggag gaggaggagg aggaggagga 9960
tggggcgggg gagggggcg gggaggtttt gagtcgtttg ggcggttttt gcgttttcgg 10020
gaagtttagg ttggagtttg agtcggagtt tgagttcgag ttgaggttgg ggagggtaga 10080
gggttatacg ttcggtattt ttggttttgc ggtttcgttt ttattttcgt ttttaaagag 10140
gtcggcgtgg tttagggttc gttttttttag tttgggggcg ttttgggagg cgttttcgtt 10200
tagtgagcgg gtttttcgtt ttattagttt tttcgacggg gttttggtgt tttttttgtc 10260
gggtagttgg agggggcggg gtgggttttt gtcggtcgtc ggtcgggagt tttcgtttgt 10320
ggggttcggt cgtcgcgttg gtcggttagg tttttttttt tttgaggtgg ttataggagt 10380
ggtcgaagat ggtttttttt tggttttttt cggtcgtttt gttttgcgtc ggggggttgtt 10440
ggtttgcggg ttttttttgt tcgtacgggg tttttttata ggtcgttttc ggtttttgtt 10500
tttggttttg tttttttttt gggtttcgtt tttgtagttg ggggtaggtg gggtttcggt 10560
tttttttttt ttgggttttt tttttttggg gtagttttta ttttgtgagg attggatttg 10620
ggttggggag gcgggtttgg ttgggggcgg ttggaggttt tttacgtcgt attgtacggt 10680
cgtttttttg gtttttcgta ggtcgttttt ttcggttttt aggttttttt tggagggtgg 10740
ttgttcggtg gttttgattt tagggtcggt tttggttttg ggggtgttgg tcgtggtggg 10800
ttcgttattt gggatcgtgg cggttttatt ttttgagttt gagaattttg tatcgtaatt 10860
gttaaagggg ttatagagtt ttttgggagt aggtgtgtag ggtttattgt cgcgggagtt 10920
tcggggtcgt ttggcggttc gtttatttcg ggagttggtt ttgttgaggt gtcgggtcga 10980
gtagttggag agagggttat attttaggtt tgtgggtggt ttggagttgt ttattacgta 11040
tttgttattg ggaacggggc ggttgtgtcg tcggggttgg tttatagttt tggggacgta 11100
ttttagggta tcgttatttt ttttattttt gttttgattt ttgtttaggg acgttagcga 11160
gtatttgttg ttcggttcgg taggggcggt tagtggggtg ggttggtagt cggtattagg 11220
gtttaatagg tcgtggttgt cggggttgta gtcgaaggtt agtgggaagg tattttcgtt 11280
cgggtttata gtggggttgg cgttggggtc gcggggcgtt agggcggggg tttcggcgga 11340
gcggtgttta cgggtcgttt ttagtagttt tcggtagcgt cgttgtttta gttttatttt 11400
attgcgtacg gtttcgatgg tttggttgat tagttttaat tttagtatat tcgggcgcgg 11460
tttttttttt aggtttaggg tgttattttt tttttgcgcg gggggtttgg gtagtttagg 11520
gttgtagggg tcgtaattta gttttgaagg gaatagagag tatagttgtg attagtttgt 11580
cggagaggag ttcgtagtat gggggcggtt tcggttttaa attgtaggga agggaagaga 11640
tttgttttcg aaatttatta cgtattggcg ttcgttaagg ttttggggtg tttagttggg 11700
tataggttat ggcgttattt taggttttgt gtcgggtttc gttttgtttt gattaatggg 11760
cgttggatgg gtttgggttg cgtgatatat agttttttgg gtattggagg ttttttttata 11820
cggtttatga aatgcgtata atgattatgg tggggtggat attcgatagg ttggaatttt 11880
tcggggtagg cgggtgtgga gtttttagta tttagggtgt cgggttgagg taggattggg 11940
ttgggtcgat aattatagtt tgacggtgga tataggagag ttttttttta gttaatacgg 12000
ttttatttag ttagtagtgt gttcgcggtg tggcggaggt gtgttagtag tgtgttagcg 12060
gtggtgtggt ggcggtgtgt ttgcggtgat gtggttgagt agggtcgggg gatggggata 12120
tgtttagttg atgggttatt ttgtaaaatt tataggaaga tttttttttt ttgagatagt 12180
ttttttgttg tttaagttgg agtatagtgg tgcgattttg gtttatcgta gtttttattt 12240
ttcgagttta agtaaatttt ttgttttagt tttttaagta gttgggatta taggcgtgcg 12300

```
ttattatatt tagttaattt ttgtattttt agtagagatg gggttttatt atgttggtta   12360
gtttcgaatt tttgatttta agtgatttat tgtatttggt ttattttaaa ttaatttttt   12420
aattttaaaa tttttgtag ttgggcgcgg tggcgtacgt ttgtaatttt aatattttgg   12480
gaggttaagg cgggcggatt atttgaggtc gggagtttaa gattagttag atcgatatgg   12540
agaaatttcg tttttattaa aaatataaaa ttagtcgggc gtggtggcgt atgtttgtaa   12600
ttttagttat ttaggaggtt gaggtaggag aatcgtttga atttgggagg tagaggtttc   12660
ggtgagtcga gattatatta ttgtatttta gtttgcgcga taagagtgaa atttcgtttt   12720
aataaaaaaa aatttgtaga ggtggagttt tattatattg gttaggttgg ttttgaattc   12780
gtagatttaa gtaatttttt tattttagtt tttaagatag ttgggattat agtttaagtt   12840
gttgtgtttg gtttaaagta tacggttgaa atgttttttc ggggggtagt tgggaatatg   12900
agattcgtat gttatttacg tggttttaga atatagtgta gtagattttt ataattttaa   12960
tgttattaac gttagtatag aagttagaat tatagaagtt tttcgtgggg ataggtcgaa   13020
ggagtttaaa gaaaatatga gtaagtggag tttagtattt tagcgaagta ataaaaatta   13080
agtttaaata tttattttag aaatttggaa gttgattatt agaaatttta ttgagtacgg   13140
ttaggtatag tggtttacgt cgattatttt agtattttgg gaggttgagg cgggaggata   13200
ttgtttgagt ttaggagttt aagattagtt tggatgatat agattttatt ttttaaaaaa   13260
atatttaaaa ataagttgag tatggtgatg tatatttgtg tttttagtta tgtgggaggt   13320
taaagtggga gaattatttg agtttagggg tttgaggttg tagcgagtta ggattgtgtt   13380
tttgtatttt ggtttggata ataaaataag attttagttt ttaaaaaaat aaaataaagt   13440
tttattgaat gtaatttcgt aatattttta aaggttttta aaggttaata ggtttgattt   13500
tttggagttg gtaggaaatg gcgttaaaga cgtttgcgtt ttaatttttg gaatttgtag   13560
atggtatatt tttttagatg gtaaatgggg ttttagatgg ggtgaaattt tttttgttcg   13620
ttattgaggg ggttatggat aggtcgagat tttgtagagt agtttcgtaa ttattttatg   13680
tagttttaat attattattt ttagattcgg aatttttaga attcgtgtta tagatagatc   13740
gtatagggta ggtatagatg cgtttggtgt agtttttatt gtaaaaatat aataataaat   13800
aacgtaggag atgatttaga agtcgaaacg taggttattt gtggttaagg gggaggcgtg   13860
cgttttggag ggatttttaa gttatattgt tatggggaag aagaggggta aggaatgttg   13920
tatattttta tttttgaaaa gtagttttat aaatttagtg agattagatt ttaataaagg   13980
cgtacgggtt tatattaagt tcgtagtttt atggggtata gtaaatgtaa tttggttttt   14040
atttatgttg tatttaatat taattgttta agagggacgt gaatgttgat atatattttc   14100
gataggttga taaggagttg tgatattatg tagtaaaagg taatagtttt atgggaattt   14160
gttcgtatta agacgttatt tggttttgat ttgattttga ttgaacgtgt agtttgtttg   14220
aaggtcgggt tttaggtgtg tttttttttat ttagaaaatt tgtttgtttt tcggtttatt   14280
tggagatcga tttttatagc gtgttaagat tttcgtgtag agaaatggtt ggacgtagtt   14340
aaggggaggt aggtataaga ttttttattg tgggcgacgt agagaatagt aatagaattg   14400
gaggtttttt ttatggtttt tatttatatt tttttaagtt ttagtaggta taagaaagaa   14460
atggttggtt tcggtcggtt gtggtggttt atatttgtaa ttttagtatt ttgggaggtt   14520
gaggcgggcg gattatttga ggttaggagt tcgagattag tttgattaat aaggaaaaat   14580
tttattttta ttaaaaatat aaaaaaatta tttagtcgtg gtgatttatg tttgtaattt   14640
tagttattcg ggaggttgag gtaagagaat cgtttgaatt tgggaggcgg aagttgtagt   14700
gagttaagat tgagttattg tattttattt tgggtaataa gagtaaaatt ttattttaaa   14760
aaaaaaaaaa aaaaaaaaag aaagaaaaag aaaaagaaaa aaagaaatgg ttggtttttt   14820
aagttgtttt ttcgagatat atttattagt agaataaaag ttttttttagg taagtttttta   14880
ttttaatttt tgggatttgt ggttaggtta ttttatttag ttaatagatt gtaggtgcga   14940
tttgaataaa ggattttgag ggtgggtgaa tattttgtat tattcgggaa tttagtgttt   15000
ttatagggtt ttttaaagag ggaggtagga gggtgagagt tagagagatt ggaaaaggtt   15060
gtgttgtggg tttgaagggg aggaaggcgt tttgagtcga gggatgtagg tgtttttaga   15120
tgttgggaaa ggcgggtatt gatttttttc ggaagttttc gggaggttta gttttgttta   15180
tagtttgatt tgagtttagc gaggttgatt ttggattttt tagtttagcg ttgagagaga   15240
gtgtgttgtt ttaaggttta cggggacgaa tgtagtttta tcgggtgatg aaagtgtagt   15300
ggaaaatagt tgttcgttgt ttgtatttgc gaaggcggaa ggtttttttt gttttcggga   15360
attttgtagg gatagcggga ggtcgggatt cggaggttgt agaggaggtg ggaggaggtt   15420
tggttacggg tagttgtcgt tttttttttg aaattatagt agtgttgtgt ttatttttaa   15480
aattatattt aggattagg tacggtggtt tacgtttgtt attttagtac ggtggtttac   15540
gtttgttatt ttaggtacgg tggtttacgt ttgttatttt agtattttgg gagattaagg   15600
cgggtagatt attagtttag gagtttaaga ttattttggt taatatggtg aaattttatt   15660
tttattaaaa atataaaatt agttaggtat gggggcgtgt atttgtaatt ttagttattg   15720
aggaggttga ggtaggagaa tcgtttgaat ttaggaggta gaggttgtag tgagttaaga   15780
ttatattatt atattttagt ttggcgatag agtaagattt tgttttaaaa aaaaaaaaaa   15840
aaaaaaaaat tatatttagt gttaggttta gtggtttacg tttgtaattt tagtattttg   15900
gaaggttaag atgagtagat tgtttgagtt taagattagt ttaggtaata cggcgagatt   15960
```

```
tcgtttttat aaaaaaatat aaaaaatgag ttgggtgtga tggttgtatg tttgtggttt   16020
tagttatttg gaaggttgag gtgggaggat tttttgagtt taagaggtta aggttttaat   16080
gagttgtgat tatattatcg tattttagtt tgggtaatag agtaagattt tgtttttaaa   16140
ataaataaaa ttaagtagta tttagataag aggaaggatt cgtgtgtttg ttttggatta   16200
ttcggaattt cgcgggattg tgtggagggt tacgaggtac gggtaggtag gggaatatta   16260
tatagaagtt ataggttttt ttttgtttgt gttaggttat agttaaggta tttttaggaa   16320
acgttatttt atgtgtttag taggcgtaga ggggtttatt ttggtattat tttatagtta   16380
aggattttgg gagttagcga gggtatttaa ttaggattag taattgtggt tgttagtttt   16440
tttgtgcggg gagggatagt tgttaggtta ggaggatgtt ttcggtaggg aaacggtcgg   16500
ttggggttgg gtgttagttt ttatttaggg ggagggttgc ggaggttagt agtagtttaa   16560
gggcgttttt tcggtttatt ttattggggg aggtttcggt tttttttga tttagtcggt   16620
tgtagagtac gttattttgt tattcgtttg gtgaataggg taattagtgt tagtttttt   16680
ttagtttcgg aagttcggtt gtttagtgac gtggaggggt taggttaagg ttattgtggc   16740
gtgagttgta gagatttatg aaggggaggt gatagagtat aattattgag gaggagtaag   16800
gttaggttag ggagatattg gtttttttt ttaaggtttt agttaacggg gatgtagaga   16860
ggaggaagag ttcgtttaac gggaaggttt tttttttag gtaggggtgg gtagtagtga   16920
gattgggttt taggagggta ggcgggggtg gaggggattg aggtattttt aaggggttaa   16980
ggataaggga ttttatttag gggtttttat ttttttgttt tggattttag ggttttaaga   17040
tatagtttgt aagattaatt tagtttaggg gagttaagag ttataggtaa tatatatatt   17100
agttagtaga tattcgcgag tttttttaa aggaaggaaa tttagatttt tggatattgt   17160
ggattttgaa aggagagatt atttttttaa aaggtgtttt tggaaggttt tggaaggaat   17220
agtatttgtt ttttaggagt ggagttaggt ttttaagtag agcgtaggat aggtgataag   17280
ggtttaattt gggggacgtg ggtttgtttt tttaattgga agtttgtgtg agtatttgtt   17340
ttgttgtttt gtggagatag gtattgttta ggttggagtg tagtggtatt atgatggttc   17400
gttgaagttt tgtttttcg gttttagcga tttttcgtt ttagtttcgt aagtagttgg   17460
gattatttgc gtgtattatt atatttagtt aattttttt ttttttggt agagacgggg   17520
gtttcgttgt aattgtttag gttggtttta aattttaag tttaagcggt tttttcgttt   17580
cggtttttta aagtattgtt tgttttttaa gggttgtgaa atttgtttta gtttttagtt   17640
aatagatggg aaagagtagg ggtatttgga ggtcggaggg tatattttgt agagggggtt   17700

ttagtatttt tattttcggg tggaggttgt tattattttt ataaagattg tattaaaaag   17760
aaagtaggta tgttaggcgg gttcgagaag attgagatta ggatttgtat tacggttttt   17820
tttttttgtg gttattgggt ttttttattt tgtagtttgt gatttattgg tgttggaagg   17880
tagtattttg gtttgggcgt tttagttagg tttggaagtt ttatatagga agttgtttgg   17940
ggttcggtta tggttgtttg tagtttagaa taggggtac ggcgtgttga gtttttgaaa   18000
ggttatttga agttggtata tattattgtt tttttggatt tttttgtttt attatgtttt   18060
agagggttgg atttgggtat ttgttaggat tggaaaggtt ttagggtggt atgtgtatgt   18120
tgttaggtgt tttgggagtt tatttagggg gttatattta aaggtcgagt ttgggttagg   18180
cgtggtggtt tatatttgta atgttagtat tttgggaggc ggaggcggga ggattatcga   18240
ggttaggagt tcgaaattag tttggttaat atggtgaaat tttattttta ttaaaaaata   18300
taaaaaaaaa ttagttaggt ttggtggtag gcgtttgtaa ttttagttag tcgggaagtt   18360
gaggtaggag aattatttaa atttgggaag tggaggttgt agtgagtcga gaacgtatta   18420
ttgtatttta atttgggcga tagagttata tatgtatata tatatatata tatatatata   18480
tatatatata tataaatgaa tgtcgatttt ggaagtagga ggggttcgtt taggtttcgt   18540
tttacggttg ggaattattt tggataggag taggtgggtt tttttttgga tgtggtttag   18600
gttagtttgt ttttgggaaa tatggtgaat tggttgatag gaaaagttta tttttagagg   18660
tttgggagtt gtttttttt ttttgtttta ttttaaggtt aggattggga ggtattattt   18720
agttttagtg gttttgagag gttataacgt ttggttggtt atttttttta gtttttttag   18780
ttagggttta gtttagggta acgtagttta gttttgtttc ggtttaggag agagtttatt   18840
ttttatatta tttattttga tcgtattttt tgagattagt gttttgtagt ttcgggacgg   18900
gttggatgat aaagatgata ttttttttta gggttttagg tgtggggttt gggattagta   18960
ttatttgttt aaggttattt tttggttttt aaattttttt ttggtttaga aatttttat   19020
tttgggtatt tatttttttt tttgttttta ttgttttcgt tttattatat ttaatttatt   19080
tatatagttg ttttgttttt agtaatgggg agaatttggt atgagtttta attttatacg   19140
gagattgaaa ggtagtaaaa atgataagat tatggagtac gaagatttta atttgaattt   19200
ttagaacgtt ttttagtgag attcgtttaa atttttgttt ttttttagtt agtttatttt   19260
aaagatgagg atatttagtg ttagcgagag tcggtagttt ttagttttat aaggagtttt   19320
aggtttttt tatttagacg gggagattta gtggttttaa agaattcggt aggttggttg   19380
gatacgatgg tttatgtttg taattttagt attgtaggag gtcgagacgg gtggattatt   19440
tgaggttagg agttcgagat tagtttggat aataaggtaa aatttcgttt ttattaagaa   19500
aatataaatt agtcgggcgt ggcggtaggc gtttatagtt ttagttattt gggaggttga   19560
```

```
gataggagaa ttatttgaat tcgggaggcg gagattgcgg tgagttaaga ttacgttatc   19620
gtattgtagt ttgggtaata gagtaagatt gttttaaaaa aaaaaaaaaa aaaaaaaaag   19680
aattcggtag gtttagtttt tgtttcggat atcgttcggt gggttcgtag ggtttttaac   19740
gtaattaggt attagtagga ttggtttttt tagtttaggt agtgggtttc gtatgttggt   19800
ttagaatagg ttaggtagag attttagatg gaaatagata gttttttcg tttggggttt   19860
agttggataa aaaagataag tttgatattg cggtatgaga taagtaagat tatttttaat   19920
ttagtttttg ggggagttat ttgggcggtt tgaggttttg aaggtggaga tagaagagga   19980
tatttttaaa atagttatag cggtagagaa ggtacggggt tgattttcgc gaagcgtaag   20040
gtaggggttg gttgtgttta cgggagcgtt tattttataa tagatttttt gggaagaggg   20100
gtttggagga tagtattttt gaagtttatt ttttgcggac ggtagagtaa ttataaggtt   20160
tttataaatg gttatacgtt tattttgggg gtttttgttt ttggggtttt ttttttaatt   20220
tttatagtgg gacgtttttt agttttattt ttggatatgc gggcgttcgg ggttttttat   20280
gtatagttta ttggatagat aggaattcgt tgtttagagg taaagggatt agtattttgt   20340
atttagagga ggttttatag tttttagatt atacggggta gaaatagggt tatttggaga   20400
gggtttcgag gaaaagttta gttagtgtcg ggggtttgga tttttattat ttagtagttt   20460
ttaatatttt ttttgaaatt taaggtggtt tagttttgtt ttattttttta tattagattt   20520
tatttaggtt ttagagtttg ttgtggaggt tgggttgtgt ttgtagtagc ggtatttggg   20580
gtttatttta ttgtttttag ggttttttttc gattttttagg gttgcgattc gagttttttt   20640
tttgttttaa ggtatcgttt tttttatttc gatagcgtag tgtgtagtgt taggatcgtt   20700
tagtaggtgg agttatacgg tagggtttag gggagaaaaa gaaaaaggtt tcggtttgat   20760
cggggattat tagttttgtt ttttggggcg ttttgggtat ttatttattt tatggggcgg   20820
tcgtttttat atgttatgat tggacgcgtt gttgttttaa ggtttatagt tcgaggttag   20880
gggataggga cggtaattag gatagaggtt attgtttttt aaaataatgt agaaataggt   20940
tgggcgtagt agtttacggt tttaattttc gcgttttggg aggttgagtt taaggtcgag   21000
gtaggtggat gatttgaggt gaggagtttg agattagttt ggttaatatg gggaaatttc   21060
gtttttatta aaattataaa aaattagtcg ggtgtggggg tgtttataat tttagttatt   21120
taggaggttg aggtaggaga attgtttgaa ttcggaaggt ggagtttgta gtgagttgag   21180
atggcgttat cgtattttag tttgggtaat aagaacgaaa ttttatttta aaaaaaaaaa   21240
aaaaaattag ttgggcgtgg tggtttatat ttgtaatttt agtattttgg aaagtcgaag   21300
cgggcggatt atttgaggtt gggagttcga gattagtttg agtaatatgt agaaattttg   21360
tttttattta aaaaaatata aaattagtta gttgtggtgg tatatgtttg taattttagg   21420
taattttatg tttgggcgta gtagtttacg tttgtaattt tagttattcg ggaggttgag   21480
gtaggagaat cgtttgaatt cgggaggcgg acgttgcggt gagtcgagat cgtgatattg   21540
tttttttagtt tgggtgatag ggcgagattt ttttttaaaa aaaaaaaaaa attgaaatag   21600
aagggtataa atggtaaagg ataagaatcg gtagggagga gttagcggga tttttttttt   21660
ttatgttttt ttatttttgt taagagttta ttttggttgt gagttatttt tatggtttat   21720
atagagtaag ttaagagttt agtttttagt cgggtacggt agtttacgtt tgtaatttta   21780
gtatttggga ggataaggtt ggtggatttt ttgagtttag gagttcgaga ttagtttggg   21840
taatatggtg aaattttgtg tttattaaaa atataaaaat tagtagttgg gtacggtggt   21900
ttacgtttat aattttagta tttcggaaag ttgaggcggg cggattgttt tgagtttagg   21960
agtttgagat tagtttgggt aatacggtga aattttgttt ttattaaaat ataaaaaatt   22020
agttgggcgt ggcggcgtgt ttttgtaatt ttagttattc gggaggttg              22069
```

<210> 170
<211> 22069
<212> DNA
<213> Artificial Sequence

<220>
<223> chemically treated genomic DNA (Homo sapiens)

<400> 170

```
tagtttttcg agtagttggg attataggag tacgtcgtta cgtttagtta attttttgta      60
ttttagtaga gatagagttt tatcgtgttg tttaggttgg ttttaaattt ttgagtttaa     120
ggtagttcgt tcgttttagt ttttcgaagt gttgggatta taggcgtgag ttatcgtgtt     180
tagttgttga tttttgtatt tttagtagat atagggtttt attatgttgt ttaggttggt     240
ttcgaatttt tgggtttaag agatttatta gttttgtttt tttaagtgtt ggggttatag     300
gcgtgagtta tcgtgttcgg ttgagagttg gatttttaat ttattttgtg tggattatga     360
aagtggttta tagttaaggt gggtttttgg tagggatgga gaggtatagg gggagaaggt     420
ttcgttggtt tttttttgtc ggtttttgtt ttttgttatt tgtatttttt tgttttagtt     480
```

```
tttttttttt tttgaaaagg agtttcgttt tgttatttag gttggagagt aatgttacga 540
tttcggttta tcgtaacgtt cgtttttcga gtttaaacga ttttttttgtt ttagtttttc 600
gagtagttgg gattataggc gtgagttatt gcgtttaggt atgggattat ttgggattat 660
aggtatgtgt tattatagtt ggttaatttt gtattttttt aagtagagat aggatttttg 720
tatgttgttt aggttggttt cgaattttta attttaggtg attcgttcgt ttcgattttt 780
taaagtgttg ggattatagg tgtgagttat tacgtttagt tggttttttt tttttttttt 840
gagatggagt ttcgtttttg ttgtttaggt tggagtgcgg tggcgttatt ttagtttatt 900
gtagatttta tttttcgggt ttaagtaatt tttttgtttt agttttttga gtagttgggg 960
ttataggtat ttttatattc ggttaatttt ttgtagtttt agtagggacg gggttttttt 1020
atgttggtta ggttggtttt aaattttttta ttttaaatta tttatttgtt tcggttttgg 1080
gtttagtttt ttagagcgcg gggattagag tcgtgagtta ttgcgtttag tttgttttta 1140
tattgttttg aaaggtagtg atttttgttt tggttatcgt ttttgttttt tagtttcgag 1200
ttgtaagttt tgggatagta gcgcgtttag ttatggtatg tggggacggt cgttttatga 1260
ggtgaataaa tgtttaggac gttttagaga gtagagttgg tggttttcgg ttaggtcggg 1320
attttttttt tttttttttt tggattttgt cgtgtggttt tatttgttgg gcggttttgg 1380
tattgtatat tgcgttgtcg aggtggggag ggcggtgttt tggagtagag gggaggttcg 1440
gatcgtagtt ttgggggtcg aggagaattt tgggggtagt ggggtagatt ttaggtgtcg 1500
ttgttgtagg tatagtttag tttttatagt aagttttgag atttgggtgg agtttggtgt 1560
ggggggtggg gtagagttgg gttattttgg attttagaga gggtattgga agttgttggg 1620
tggtggaggt ttaggttttc ggtattggtt ggattttttt tcgaagtttt ttttaggtga 1680
ttttgttttt gtttcgtgtg gtttaggagt tgtgggattt tttttgggtg tagagtgttg 1740
gttttttttgt ttttgagtag cgggttttta tttgtttagt gggttgtgta tggagggttt 1800
cgggcgttcg tatgtttaag gatggggttg gaggacgttt tattgtgagg gttggagggg 1860
aggttttagg agtagggatt tttagagtgg acgtatggtt atttgtgggg attttatggt 1920
tattttgtcg ttcgtaggag gtggatttta ggggtgttgt tttttagatt ttttttttta 1980
gagggtttgt tatgggatgg gcgttttcgt ggatataatt agtttttgtt ttgcgtttcg 2040
cgagggttag tttcgtgttt tttttgtcgt tgtggttgtt ttgggaatgt ttttttttgt 2100
ttttattttt aaagttttag gtcgtttaga tggttttttt aggggttagg ttgggggtag 2160
ttttgtttat tttatgtcgt agtgttaggt ttattttttt tgtttagtta agttttaggc 2220
gggagggatt gtttgttttt atttggggtt tttgtttggt ttgttttggg ttagtatgcg 2280
gggtttattg tttgggttgg aggggttagt tttgttgatg tttggttgcg ttgagagttt 2340
tgcgggttta tcgaacggtg ttcggagtag gggttgggtt tgtcgggttt tttttttttt 2400
tttttttttt ttttgagata gttttgtttt gttgtttagg ttgtagtgcg atggcgtaat 2460
tttggtttat cgtagttttc gtttttcggg tttaagtgat ttttttgttt tagtttttta 2520
agtagttggg attataggcg tttgtcgtta cgttcggtta atttgtgttt ttttagtaga 2580
gacggggttt tattttattg tttaggttgg tttcgaattt ttgattttag gtgatttatt 2640
cgtttcggtt ttttatagtg ttgggattat aggtatgagt tatcgtgttt agttagtttg 2700
tcgggttttt tagagttatt gagtttttttc gtttgggtgg gaggggtttg gggtttttttg 2760
tggggttagg agttgtcggt tttcgttggt attgggtgtt tttatttttg aaatgggttg 2820
attgagagaa gataggagtt tggacgggtt ttattagaga acgttttgga agtttaggtt 2880
gggattttcg tgttttatga ttttgttatt tttgttgttt tttagttttc gtgtggggtt 2940
ggagtttatg ttaggttttt tttattgttg gggataggat agttgtgtgg gtggattgag 3000
tgtggtgggg cgggggtagt gggagtagag aggagagtgg atgtttaggg tgaggggttt 3060
ttgggttaag ggaaggtttg ggggttagga gatggttttg ggtagatagt gttggtttta 3120
ggttttatat ttagaatttt gggaagaaat attatttttg ttatttagtt cgtttcgggg 3180
ttgtagggta ttggttttag gagatgcggt taggatgggt ggtgtaggga gtgggttttt 3240
ttttaggtcg ggataaggtt gggttgcgtt gttttgggtt gagttttggt tggagaggtt 3300
ggggaagata gttagttagg cgttgtggtt ttttaggatt attggggttg ggtggtattt 3360
tttagttttg gttttggggt gaagtaggga ggaaggggta gtttttaggt ttttggaggt 3420
aggttttttt tgttagttaa tttattatgt tttttagaaa tagattgatt tgggttatat 3480
ttaggaagga gtttatttgt ttttatttaa agtaattttt agtcgtggga cggggtttgg 3540
acgggttttt tttattttta gagtcggtat ttatttatgt atgtgtgtgt gtatgtatgt 3600
atgtatgtgt gtatgtatgt atagttttgt cgtttaggtt ggagtgtagt ggtgcgtttt 3660
cggtttattg taatttttat tttttaggtt taggtgattt ttttgtttta gtttttcgat 3720
tagttgggat tataggcgtt tgttattagg tttggttaat tttttttttgt atttttttagt 3780
agaggtggag ttttattatg ttggttaggt tggtttcgaa tttttgattt cggtgatttt 3840
ttcgttttcg ttttttaaag tgttagtatt ataagtgtga gttattacgt ttggtttaaa 3900
ttcggttttt agatgtgatt ttttgggtgg gtttttaggg tatttagtag tatgtatatg 3960
ttattttggg gttttttttag ttttgatagg tatttagatt taattttttg aggtatggtg 4020
ggatagggag gtttagggag gtagtggtgt gtattagttt taggtgattt tttaggggtt 4080
tagtacgtcg tatttttttgt tttgagttgt aggtagttat ggtcgagttt taggtaattt 4140
```

tttgtgtgaa atttttagat ttggttggag cgtttaggtt aggatgttgt tttttagtat 4200
tagtgggtta taggttgtag ggtgggggag tttagtgatt atagggagga gaggtcgtgg 4260
tgtagatttt gattttagtt ttttcggatt cgtttagtat gtttgttttt tttttggtgt 4320
agtttttgtg gaggtggtag tagtttttat tcgggagtga aaatgttgaa atttttttttg 4380
tagagtgtgt ttttcggttt ttaggtgttt ttgttttttt ttatttgttg gttaaaggtt 4440
ggggtaggtt ttatagtttt taaaagataa gtagtgtttt gggaggtcga ggcgagaaga 4500
tcgtttgagt ttagaagttt gagattagtt tgggtaatta tagcgagatt ttcgttttta 4560
ttaaaaaaag aaaagaatta gttggatgtg gtggtgtacg taggtggttt tagttatttg 4620
cgaggttgag gcgggaggat cgttggagtc ggggaggtaa agttttagcg agttattatg 4680
gtattattgt attttagttt gggtaatgtt tgtttttata aaataataaa ataagtattt 4740
atataggttt ttagttagaa agataagttt acgtttttta gattggattt ttattatttg 4800
ttttgcgttt tgtttggggg tttggtttta tttttgggag gtaggtgtta tttttttttaa 4860
gatttttttaa aagtattttt tggggaaatg gttttttttt ttagagttta taatgtttag 4920
aggtttgaat tttttttttt tgggaagggt tcgcgggtgt ttgttggttg gtgtgtgtat 4980
tgtttgtgat ttttggtttt tttgggttgg gttgattttg taggttgtgt tttagagttt 5040
tggggtttag gataaggagg tggaagtttt tgagtggagt tttttatttt tagtttttta 5100
gagatgtttt agtttttttt attttcgttt gttttttttgg ggtttagttt tattgttgtt 5160
tatttttgtt tggggaggag agtttttttcg ttaggcgggt tttttttttt ttttgtattt 5220
tcgttagttg gagttttggg aggggagatt agtgtttttt tgatttggtt ttgttttttt 5280
ttagtaattg tgttttgtta tttttttttt atgaattttt gtagtttacg ttatagtggt 5340
tttggtttgg ttttttttacg ttattgggta gtcgggtttt cggggttggg ggagggttgg 5400
tattagttgt tttgtttatt aggcgggtgg tagggtggcg tgttttgtag tcggttgggt 5460
tagggaggag tcggagtttt ttttagtgaa gtgggtcggg agggcgtttt tgagttgttg 5520
ttggttttcg tagttttttt tttgggtggg ggttgatatt tagttttagt cggtcgtttt 5580
tttgtcggga gtattttttt ggtttggtag ttgttttttt tcgtatagag gaattggtag 5640
ttatagttgt tgattttagt tgggtgtttt cgttgatttt tagggttttt ggttgtgaga 5700
tggtgttaga atgggttttt ttgcgtttgt tgggtatatg gggtgacgtt ttttggaaat 5760
gttttggttg tagtttggta taggtaaaga agaatttgtg gtttttgtgt gatgtttttt 5820
tgtttgttcg tatttcgtgg ttttttatat agtttcgcgg agtttcgagt ggtttaaaat 5880
agatatacgg attttttttt ttgtttaaat gttatttaat tttatttgtt ttagagatag 5940
gattttgttt tgttgtttag gttggaatgc ggtggtgtga ttatagttta ttgaagtttt 6000
gattttttag gtttaagaga ttttttttatt ttagtttttt aagtagttgg gattataggt 6060
atgtaattat tatatttagt ttatttttttg tattttttttg tagagacggg gtttcgtcgt 6120
gttgtttagg ttggttttga atttaagtag tttgtttatt ttggtttttt aaagtgttgg 6180
gattataggc gtgagttatt gagtttggta ttaagtgtaa tttttttttt tttttttttt 6240
tttgagatag agtttttgttt tgtcgttagg ttggagtgta gtggtgtgat tttggtttat 6300
tgtaattttt gttttttggg tttaagcgat tttttttgttt tagtttttttt agtagttggg 6360
attataggtg tacgtttttta tgtttggtta attttgtatt tttagtagag atggggtttt 6420
attatgttgg ttaggatggt tttgaatttt tgagttggtg atttgttcgt tttggttttt 6480
taaagtgttg ggatgatagg cgtgagttat cgtgtttggg atgataggcg tgagttatcg 6540
tgttgggatg ataggcgtga gttatcgtgt ttggttttta aatgtaattt taaagatggg 6600
tataatattg ttgtgatttt aagggaaagg cggtagttgt tcgtgattag gttttttttt 6660
attttttttg tagttttcgg gtttcggttt ttcgttgttt ttgtagggtt ttcgggaata 6720
ggaggggttt ttcgttttcg taggtatagg tagcgggtag ttgtttttta ttgtattttt 6780
attattcggt gaggttgtat tcgttttcgt gggttttaaa gtagtatatt tttttttagc 6840
gttgggttgg gaagtttaaa gttagtttcg ttgggtttaa gttaagttgt gggtaggatt 6900
gggtttttcg aaggttttcg gggagagtta gtgttcgttt tttttagtat ttggaggtat 6960
ttgtattttt cggtttaggg cgtttttttt tttttaaat ttatagtata gtttttttta 7020
gttttttttga tttttatttt tttgtttttt tttttaaagg attttgtgag gatattgggt 7080
tttcggataa tgtaagatgt ttatttattt ttagggtttt ttgtttaagt cgtatttgta 7140
gtttattggt tgagtgaggt ggtttggtta taggttttag ggattaggat ggggatttat 7200
ttggggaggt ttttgttttg ttggtgggtg tgtttcgggg gagtagtttg ggaaattagt 7260
tatttttttt tttttttttt tttttttttt tttttttttt tttttttttt ttgagatgga 7320
gttttgtttt tgttgtttag gatagagtgt aatggtttaa ttttggttta ttgtaatttt 7380
cgttttttag gtttaagcga ttttttttgtt ttagtttttc gagtagttgg gattataggt 7440
atgggttatt acggttgggt aatttttttg tatttttagt agagatgggg tttttttttg 7500
ttggttaggt tggtttcgaa tttttgattt taggtgattc gttcgtttta gtttttttaaa 7560
gtgttgggat tataggtgtg agttattata atcggtcgaa attagttatt ttttttttat 7620
gtttgttggg gtttgagggg gtgtgggtgg gggttatagg ggaagttttt agttttgttg 7680
ttgtttttttg cgtcgtttat agtgggaggt tttgtgtttg tttttttttg gttgcgttta 7740
gttatttttt tgtacggaag ttttagtacg ttgtgggggt cgatttttag gtgggtcgaa 7800

gggtaggtag gttttttgag tggaagaaat atatttgagg ttcgattttt agataagttg 7860
tacgtttagt tagaattaag ttagagttaa gtggcgtttt ggtgcgggta ggtttttatg 7920
gggttgttgt tttttgttat atggtgttat agttttttat tagtttgtcg gggatgtgtg 7980
ttagtattta cgtttttttt gggtagttgg tgttgggtgt agtataaatg aaggttagat 8040
tgtatttgtt gtattttatg ggattgcggg tttggtgtgg gttcgtgcgt ttttgttgga 8100
atttgatttt attgggtttg tggagttgtt ttttaaaggt gagagtgtgt aatatttttt 8160
gttttttttt ttttttatag tagtgtgatt tggaggtttt tttagggcgt acgttttttt 8220
tttaattata ggtgatttgc gtttcggttt ttaggttatt ttttgcgtta tttattgttg 8280
tgtttttgta gtgaaggttg tattagacgt atttgtattt gttttgtgcg gtttgtttgt 8340
ggtacgggtt ttgaggattt cgagtttgaa ggtggtggtg ttgaagttgt atgggatggt 8400
tgcgaaattg ttttgtaaga tttcggtttg tttatggttt ttttagtgac gggtaaggga 8460
gattttattt tatttagagt tttatttgtt atttaagggg atatattatt tataggtttt 8520
aggaattagg acgtagacgt ttttggcgtt attttttgtt aattttagag gattagattt 8580
gttgattttt agagattttt agagatgtta cggaattata tttagtggaa ttttatttta 8640
ttttttttaag agttggggtt ttgttttgtt gtttaggtta gagtgtagag gtataatttt 8700
ggttcgttgt agttttaagt ttttgggttt aagtgatttt tttattttag ttttttatat 8760
agttaggagt ataggtgtgt attattatgt ttagtttatt tttaagtatt tttttaagag 8820
atgggatttg tgttatttag gttggtttta aatttttgag tttaagtagt attttttcgt 8880
tttagttttt taaagtgttg agatgatcgg cgtgagttat tgtgtttggt cgtgtttagt 8940
agaattttta atggttaatt tttaagtttt tggagtaaat atttgaattt ggtttttatt 9000
gtttcgttgg aatattgggt tttatttgtt tatgtttttt ttagattttt tcggtttgtt 9060
tttacgggag gtttttgtgg ttttgatttt tgtgttgacg ttggtggtat tgaggttgtg 9120
agggtttgtt atattatgtt ttggaattac gtaaataata tgcgagtttt atgtttttag 9180
ttgtttttcg ggaagatatt ttagtcgtgt gttttaagtt aggtatagta gtttgggtta 9240
tagttttagt tattttggag gttgaggtgg gaggattgtt tgagtttacg agtttaaagt 9300
tagtttggtt aatatggtga gattttattt ttataaattt ttttttgttg agacggggtt 9360
ttatttttgt cgcgtaggtt ggagtgtaat ggtgtgattt cggtttatcg aaatttttgt 9420
tttttaggtt taagcgattt ttttgtttta gttttttgag tagttgggat tataggtatg 9480
cgttattacg ttcggttaat tttgtatttt tagtagagac ggggtttttt tatgtcggtt 9540
tggttggttt tgaattttcg attttaggtg attcgttcgt tttggttttt taaaatgttg 9600
ggattatagg cgtgcgttat cgcgtttagt tataaaaagt tttgaaatta aaaaattaat 9660
ttaaagtagg ttaggtgtag tggattattt gaggttagga gttcgagatt ggttaatatg 9720
gtgaaatttt attttattta aaaatataaa aattagttgg gtgtgatggc gtacgtttgt 9780
aattttagtt atttgggggg ttgaggtagg aggtttgttt gaattcggga gatagaggtt 9840
gcggtgagtt aagatcgtat tattatattt tagtttgggt agtagagaga ttgttttaaa 9900
aaaaaaaaat ttttttgtgg atttgtagg gtggtttatt aattgggtat gtttttattt 9960
ttcggttttg tttagttata ttatcgtagg tatatcgtta ttatattatc gttggtatat 10020
tgttggtata ttttcgttat atcgcgggta tattgttggt tgggtggggt cgtgttggtt 10080
gaggaagggt ttttttgtgt ttatcgttag gttgtggttg tcggtttagt ttagttttgt 10140
tttagttcgg tattttgaat gttgggagtt ttatattcgt ttgtttcggg gagttttagt 10200
ttgtcgagtg tttattttat tatggttatt gtgcgtattt tatgagtcgt gtgagggagt 10260
ttttagtatt tagggagttg tgtgttacgt agtttaggtt tatttagcgt ttattggtta 10320
gggtaaggcg gggttcggta tagggtttga gatggcgtta tggtttgtat ttagttgggt 10380
attttagagt tttggcgggc gttagtgcgt ggtgggtttc ggaggtaggt tttttttttt 10440
ttttgtagtt tgagatcggg gtcgttttta tgttgcgggt tttttttcgg taggttggtt 10500
atagttgtgt tttttgtttt ttttagggtt gggttacgat ttttataatt ttgagttgtt 10560
taagtttttc gcgtagaggg agaatggtat tttgggtttg ggggaggagt cgcgttcgga 10620
tgtgttggag ttggagttgg ttaattaggt tatcgaggtc gtgcgtagtg aggtggagtt 10680
ggagtagcgg cgttatcggg agttgttgga gacgattcgt gagtatcgtt tcgtcgaggt 10740
tttcgttttg gcgtttcgcg gttttaacgt tagttttatt gtgggttcgg acgaggatgt 10800
ttttttattg gttttcgatt atagtttcgg tagttacggt ttattaagtt ttgatgtcgg 10860
ttattagttt attttattgg tcgtttttgt cgagtcgggt agtaagtatt cgttggcgtt 10920
tttggatagg ggttagggta gaggtggagg gggtggcggt gttttggaat acgtttttaa 10980
ggttgtgagt tagtttcggc ggtatagtcg tttcgttttt agtggtaagt acgtggtgga 11040
taattttagg ttatttatag atttggagta tgattttttt tttaattatt cggttcggta 11100
ttttagtagg gttagttttc gggatgagcg ggtcgttaag cggtttcggg gttttcgcgg 11160
tagtgagttt tatatatttg tttttaagaa gttttgtgat ttttttggta gttgcgatgt 11220
aaggttttta gatttagaag atgaggtcgt tacggtttta ggtaacgagt ttattacggt 11280
tagtattttt aaagttaggg tcgattttga gattaaggtt atcgggtagt tattttttaa 11340
agagggtttg gaggtcgagg ggggcggttt gcgggagatt aaggagacgg tcgtgtagtg 11400
cgacgtgggg gatttttagt cgtttttagt taagttcgtt tttttagttt aggtttagtt 11460

```
tttataggat gggggttgtt ttaaggaggg aaaatttaag aagaaaaaaa tcggggtttt   11520
atttgttttt agttgtaaag acggggttta ggggaaggat aagattaagg ataagggtcg   11580
agggcggttt gtggagaagt ttcgtgcgga taagaagggt tcgtaggtta gtagttttcg   11640
gcgtaaggta gagcggtcgg aagggattaa gaagaagtta ttttcggtta tttttgtggt   11700
tattttaggg aaagggaggt ttgatcggtt agcgcggcgg tcgagtttta taagcgggga   11760
ttttcgatcg gcggtcggta gaggtttatt tcgttttttt tagttgttcg ataggaagag   11820
tattaaggtt tcgtcgggga agttagtgga gcggaaagtt cgtttattag acgagggcgt   11880
tttttaggac gtttttaagt tgaagaagcg ggttttgagt tacgtcgatt tttttgggga   11940
cgagagtgag gacgaggtcg tagggttagg ggtgtcgagc gtgtggtttt ttgttttttt   12000
tagttttagt tcggatttag atttcgattt agattttagt ttgggttttt cggaggcgta   12060
ggggtcgttt aagcggttta aggttttttc gttttttttt ttcgttttat tttttttttt   12120
tttttttttt ttttttattt ttagcgcggg ggcggatgtg gattattcgg ttttggagaa   12180
ggaggtggat tttgatttcg attttatgga ggagtgtttg cggattttta acgagtttat   12240
tagcgttaag acggaggata gaggtcggtt ggttcggtag gtgaggcgcg cgggtgttgg   12300

ggttgggttc gggtagaggt agagtcgagg gtttggtgag agatttttta tttggagtgg   12360
tttttagttg gttcggaggt acggggtgga ggtagttttt tagtgttggt ttttgagttc   12420
gttgaaattt taggagttta ggagtattcg ggtagttttg ttgtttttgt tgtttttttat   12480
ttatttagag gttattggtt cgtggttatt agttaggttt ttgtggttac gtgtatacgg   12540
tttgttttta ttgattggta gggggatatt ggtttattta tttattttag gggaatttat   12600
tttcggtata tttttttagt tttttttttt tcgttttttt tttttttttt tttttttttt   12660
tttttttttt tttttttttt ttatttttgt tttttttttt ttagtttttt tgtttttttt   12720
ttttgttttt tttttatttt tttcggtttt tttttttttt tttttttttt ttttattttt   12780
agcgtggcga gtttttgttt ttttgggtta gttttggatt tttgggtttt ttggtttcgt   12840
gtttttaaat tggttattag ggggagttta ttttatatag attgagttta agtgtttagt   12900
tcgtagtagt tggttcggtt taaggtggtt tcgttttttt agtgtgtttt tattttgtag   12960
ttttggggtt tttaagttgg ggttttgggt gtttaggaga ggtagttttg ggtatttgta   13020
gaacgtgtga tatttgttta ttttttcgat tgtatttata taaagtttag ggattgttgg   13080
ttttgagttt ttagttgttg tgtttggggg ttggattgtt ttattttttta tttgtcgggg   13140
gtagtttgag gttggggtgt gttggtgttg gtagcgaggg tagatttgtg ttgacggacg   13200
gatatgtgtt ttagtttttt aaggaagaga agagtgagga gaaggggttt tcgggtttga   13260
ttattttgtt tttcgggtag aagaggagga tttttttattt ttttaagtaa ggttaggagg   13320
taaggttcgt agaggttagg ttagtaggag tttttttttt tttttttttt aaagacgagg   13380
ttggtttggt ttggtggttt acgtttgtaa ttttagtatt ttgggaggtt aaggcgggtg   13440
gattatttga ggttaggagt tcgagattag tttggttagt atggtaaaat tttatttgta   13500
ttaaaaatat aaaaattagt tgggtatggt gatgggtgtt tgtaatatta gttattagag   13560
aggttgaggt aggagaatcg tttgaattta ggaggtagag gttatagtga gttaagattg   13620
tattattgta ttttagtttg ggtgatagag taagattttg ttttaaaaaa aaaaaattat   13680
aaaaaaaggg ggttttgttt tgttatttag gttggttttt taatttttga ttttaagtga   13740
tttttttgtt tcggtttttt agcgttggga ttataggcgt gagttattgt ttttagtttt   13800
atttttttgt tttttgtttg tttgtttttt ggttgttttt tttttttttt ttttttttga   13860
gacggagttt cgttttgtta tttaggttgg agtgtagtgg tacgattttt gtttattgta   13920
attttcgttt ttcgggttta agtgattttt ttgttttagt ttttcgagta tttgggatta   13980
taggtgttta ttattatatt aggttaattt ttgtattttt agtagagacg gggttttatt   14040
atgttggtta gattggtttc gtgatttgtt tatttcggtt ttttaaaatg ttgggattat   14100
aagtatgagg tattgcgttt agaggtgggt agattacgag gttaggagtt cgagattagt   14160
ttggttaata tggtgaaatt tcgtttttat taaagatata aaatattagt tgggcgcggt   14220
ggtacgtatt tgtaatttta gttattcggg aggttgaggt aggagaattg tttgaattcg   14280
ggaggtggag gatttaatga gttaagatcg tgttattgcg ttttagtttg ggcgataagg   14340
taagattttg ttttaaaaag ataaatatat aaaatgagtt atttatttat aaatttttga   14400
tttttttggg atattgtttt tgtaaatttg tgacgttttt tgtaaagtat tagtgatttt   14460
tttattcgtt tttttattta agttttatta taaatttgat gtttaatatt tgttttaatt   14520
ttagtagaat ttttgttgtt ttgattgggg ttttgtttta attgatgttt tatttttttt   14580
tagtgtttta aatttcgttt ggtttagata tgttatgata agttagtatt agtttatttt   14640
ggggtaaaaa aatttgaaag ttatgtaagt ttcgttataa cgtacgtttt ttatgagttt   14700
tttgaagata ttttattttt gacgttgttc gagttttaga gggagttggt tttgtttttt   14760
tttgttagat tgttacggtg ttggggcgag ggatagagta ggttagtagt tttagggtga   14820
ttttttttag agtgcggtag acggtttgtt gcgtcgtgtg cggtttgggt ttgggtcggt   14880
tgtttagttt tgagttttag tgttttttgt agttcggaga tagtagcgtt tgttaggttt   14940
ttgtgtagac gtggcgaggt agtgtaggat aagtagagag tttggtttcg gggttagtag   15000
ttaggagagt tttagttgtt ttagttttag agttttttta gaggaggggt tttgaggaag   15060
```

attaggcgta gtgggattgg attggagtgt tagtgtaatc ggggtagttt cgtttattcg 15120
tgttttatta gggttgagga tatagtttgg gtatcgggag atattgggag tgggggagtt 15180
tggagttata tggtattgag gattattgtt ttttgttgtt ttttttagt gtgtttagta 15240
gtttagaagg tagttaggga ggtggtgttt tcggttttcg agttttagtt attttttttt 15300
aagtttgttc ggggtcgttt gttttaggtg ttttcgttgt gttgttaggt tttagtattt 15360
gtggtgacgt aggtggagtt ttcgaggagg ggtttcgcgg tgttttcggt tcggttttcg 15420
acggcgtagg aggtgtgtta tttgcgggtt tagtaggcgt agagggtatc ggcgagtttg 15480
ttgtaggttt tcgttaggtt ggtagagaag tcgtttttcg tttatatttt cgtttttggc 15540
gagaagagga ggatcgttta tatttttaat tttcgtttgg ttgtaggtga gttttggttt 15600
agggggtttg tgtcgggggg tcgtgggtat gtgggtagga gaggtggtat tttgaggttt 15660
ttgagtttat ttgaattata tggtgtttgt ttatgggatg attttgggtt tttttttgg 15720
ggtttttagt ttgtttgttt gtgaaatggg gattgttgag ggtgtagga ggtttggttt 15780
agtatttttt ttttttgatt tagtttcggg gttattagga gtttaatagt ttgttgggga 15840
ttgggttgaa ttagggtttg agtttgtggt tttagggaat ggggagttat gtcggggtat 15900
ttggtattgg gtggggagta ggtttttgta ggtgtggttt ttttttggcg tgtgagtggg 15960
gggcgtggtt tttgatggaa atgagtgttg tttttttagg agtggggttg tattttttttt 16020
atttcgaggg gtattttttt tttagtaggg ttttgtagaa agtcgggtta ggttttagtg 16080
gtgggtggat aggatttttt acggggggtt gttttgtttt ttgcgttttt atattagtgg 16140
tgtttgtaga gcgagaaagt atttttggag gtaggttata gatgagggtt ttttagaaag 16200
tttttaaaaa atgtaggttt tggtttggag gttgggaaag gtttgtgtgt ttggtaagtt 16260
ttttgggagg gtatggttgt tgttcgagga ttattttgag tagtttgttg gtttggagtt 16320
tttgtttttt tggcgttggt tggagagttt tagggttagt ttcggcgatt tttttgtaga 16380
ttttatggat tttacggttt tttttaggga gagtttttta ggttatttttg atttttttttt 16440
tttagagttt ttttacggtt gagattttag ttttagggag gggaaattat tgagttgggt 16500
ttattttagg attttatacg tttttcgttt gtgttttcgc gttcgggttg ttttgttgtt 16560
gatttgtggt gggtgttgag ttgtcgtagt gtttttgggg ttcgtcgttt gaagggagtt 16620
tggcgtttta agtgttggta gtgtcgtagg gcgggtttat ttggtttgga ggattttttgt 16680
ggttgtttgg gttcgttttt agcgtgtagt cgagggagtt tggaggtttt ttgttcgttt 16740
ttgaagagta aatttttttat tgtaagaggc gcgtgttagt ttggcgggta gtttgttttt 16800
ttgttgggcg gttcgcggta gtatgagtag atttattttg ttttttttgt atttttttagg 16860
gttagatggt atttcgaggg atgttgtttt gagattaggg ttagatgttt ttgtttatag 16920
tttttttttt tgggtcggtg tcgagttttt agtttagaag tttttttgtt atatagacgt 16980
atatgtttag gttgtagttg ttcgcgtggt tagtttttcg aagtagtttt tgtaggtttg 17040
gttttttttt tttggtttgt gattttatta aagggtaagg tagaatatag aggtcgtagg 17100
gtagggtaaa gttaatgagt aggtgggggt attcgagatt tttcggtttt ttgttcggga 17160
gttagggttt tttttttaga tatcgacgtt ttgggtttgg ggttagaata cgtggtagag 17220
gtttggatta ttttatcggt gacgtgtttt tagcggtttt cgtagtttga tttgtgtttt 17280
ttgtttcgat ggatttgttt gaaggtagtt agagtttgat gttattgcgt agttttagtt 17340
ttttattttg gttcggtttt ttgtacgcgg agatgtacgt gttgttggcg ttgagcggtc 17400
gcggttgttt ggtgtttcgt tacgtggtga tgggtagtta ttcgttttgt gtttatattt 17460
tttgtgtttc gttagttttt ataggtgtta agaggatttt tgcggttagc ggtagttagt 17520
tttttaacgg ttttgagtta ggtggttagt agttgaaaat acgtatattg tcggggatgg 17580
cgtttaagat tattattatt attattttta agcgaatcgt ttatagttta tttttatagg 17640
tagttttttt tatttcggtt aggattatta tttttaagat tacgttttga ggttcggtta 17700
tatgggattt gcggagggtt agtttcgttt ttgttttgcg ggttttcgtg ttttatatcg 17760
tagtatagtt tttatttttt ttttttatgt tttggtgttt tttttttttt tttgagatgg 17820
agtttcgttt tgtcgtttag gttggagtgt agtggtacga ttttggttta ttgtaagttt 17880
tgttttttgg gtttacgtta ttttttggtt ttagtttttt gagtagttgg gattataggc 17940
gttcgttatt acgtttggtt gtttttgtat ttttagtaga aacggggttt tattatgtta 18000
gttaggatgg tttcgatttt ttgatttcgt gatttattcg tttcggtttt ttaaagtgtt 18060
gggattatag gcgtgagtta ttatgttcga tcgttttggc ggtgtttttt tgttttgttt 18120
tgttttgttt ttttggttgg ttggtgtttt tttttttgt ttagagatag ggtttttgtt 18180
gtttaggtta gagtgtaggg gcgtaattat ggtttattgt aatttcgatt ttttgggttt 18240
aagtgatttt tttattttag ttttttgagt agttgggatt ataagtcggt gttattatat 18300
tttagttaat aattttgttt tttattttgt agagataggg ttttgttacg ttgtttaggt 18360
tgattttgaa tttttgtttt taagtgattt ttttgtttta gttttttaaa gtgttgggat 18420
tataggcgtg agttattacg tttggtttttg ttttgttttt aattaataga ttttatgttt 18480
tttgagtaat tttaggtttt taggaaaatt gagtagaaag tatagtaagt ttttttgtgt 18540
ttttatggtt ttagtttttt atgattttag tttcgttgtt tttagttttt tttgttgttt 18600
ttgttttgtt ttggcgtgtt tgtttttatt gtagttaggt tgatttgttt tagcgtattt 18660
tatttttgtt tttagttatg ggtacggttt tatcgcgttt gcgtttttcg ttggaggttt 18720

```
tgtggaggtt tttatttatg gtagtttttg tttttttttt agagtttaaa gaaatttatt   18780
atttttaaag agtttggggg taaagttttt atcgttattc gttagcgtta ttttaatttg   18840
tttatcgagg agtgttttaa gttttgtatt tttaattagg aggttataga gaaggtgagg   18900
ttttggagtt tttatttggt cggtcgggtg ggattagggg gtttagggtg gtttttagat   18960
atttttcggt tttttagtcg tagttgtgat tcggagttgt ttgggagaga tgtaagtttt   19020
cgattttatg gcgattttat cgttggggtt tttcggtatt aggtaggtat ggttttagtt   19080
gggtatcgtt tgttttttag gtattgaacg aggagaaggt ggtttatgat cgtagtttta   19140
gtaagaatat ttatttgaat gtggtcgtga atatttttaa gaagtttagg ggtttggttt   19200
ttagcgttgt gttcggtttt agtagtgagt tttggcgggt gggcggggga ggtttagggt   19260
tgtttttggg gtggtagttg ggttgggatt ttcgttggga tttttatagt tttgagttta   19320
gttatagttt tagataaaag gaaagcgcgg tgttgtgggg gggttgtttg ttgtttggtt   19380
tttaggtttt tgtgtaggac ggatttggtt ttggtgtttt cgttgttagt cgtttttttt   19440
cgttgagtgg ggtttttaag tcgagtcgag gggtcgtttg ttaatggagt tgcggtagtt   19500
tttttaaagt aggaggttta gggttgggcg agttttatgt attaagtttt ggggaggaag   19560
aaagcggaga tttgggcgtt ttattgtagt ttttttaggt tgtattaagt tattatcggt   19620
ttttgttttc gttttcgttt gtattagtat tcggaggggt tttgggttgg cgtcgtaggt   19680
tgggtggttt atgaaggttt aaagttttcg aaagttatta gatttttcgt gttgtttttt   19740
tcgttttttt tttttcgttt gaggggaggg gacgaagatt ttgggtgtgg ggttttcgtg   19800
tagttttggg gtttgtagag ttggcggttg ttttgcgttt ttggtttatc gtatgtttgt   19860
ttttttttgtt cgggttaggt tggaattcgg ggttagtggg gtgttgtttt agaaaggggg   19920
agttgaggta gaggtttgag gagggtaggg tgggtgggag agtaggcggg gtaggggttt   19980
gagttagtta ttttatattt tttttttttt tttttttttt ttttagaaat tagtggtcgt   20040
agggttgtgt tttacgaggt ggtgttgggg ggtaggttgg tcgttaagat tagtttttcg   20100
tttagtcgtt taagtagttt tcgggtggag gatttgaaag gtaaggtttt gtttttgttt   20160
tgttatacgg tgtttcgtgg gtttttgtcg gtttagtttt tgttgtgtta tacggtgttt   20220
cgtgggtttt tgtcggttta gtcgtttttt tcggtggtgt ttatcgtttc gtgggttatt   20280
ttaggtaggg gttgattatt gtgtttgttt ataggggttg ttttgtatag tcgttttagg   20340
gagtatttgt ttatttagga ttagtttaag gagaacggtt attttttttc gtatttagag   20400
cggttcgggg gcgtaattat ttttatagtt gaggagaaga ggtttaagga ttgtgagtcg   20460
ttggtttgcg acggtttagg tgggtaggtg cgggatgtgg gtaaggtcgg gtatgcgggt   20520
tttagcgtga gttttagttt ttatttgggt ttttatttat agttttttgt aggatttgtt   20580
gtcgttgtgg tatcgagtat ttcgtgtttt ttttaggtcg ttgtattcgg gacgaggagt   20640
gttattatta ttggggacgg ttgcgtcgga atcggggtaa ggttttattc gggttttttt   20700
tttttttatt tatggtttc ggaatttggg ttttttttttg aggtttttag aattggggta   20760
aggttttatt taggtttttt attttattta gggtttttgg gttgtatcgt ttttggtaga   20820
gtcggggtta gtttttggtt ttattttttt tttatttgtt ttttatgtaa aagtaagata   20880
agagtttttt tatgataagg tttaaatgtt attatttgaa aggttttgtg tttttgtttt   20940
tttgtgtttt tggtttatgg aaatgtggag gttgttggtt ttaggagcgg aggggtttgt   21000
tttatttttg gtttggtggt tttatttaat tttttaggta attacgtagt tacgtttagg   21060
gtatatggtt agttttgtat agttttgtgg cggtttttaa agttatcgtt ttagggatat   21120
ttggagggtg ttgttcgggg tagcgttgtt aagaggtttt tgtagagggt attggttttt   21180
ttggatttag gtttgggcgt tttggttgtt atacgattgt cggggtttgg tttgttttta   21240
gttttattaa gaggttgagg ttttttgttt ttagttttta ttttttagta gggttcggta   21300
gatgtaggtg gttagtgttt cgggggttgt ggagtgggtt tggtcgggtt ttgattgtgt   21360
gtttgtgttt tttgtagtgg tcggaggttg ggagatttag tatatgtgtt gttcggttgt   21420
cgtcggtttt gtcggttgtt aagtcgtaaa ggtgagtttt ggcgttttag ttttttttgt   21480
ttgtttttaa tagatttttg tttttttatc gttggtgtgg ggtttcggga tatagtatag   21540
ttttgggttt agggtttggg ttttcgggta gtttttttttg tgttatttag agaacgtagt   21600
attcgtaggt tttaggtttt gtttttggtt atttttttatt ttgggaagtc gttttttttg   21660
tgtttttttg gtaagttttt tttttttattt gtttattagg gttttaatta gtgaatgggc   21720
gtgatggatg gataggatgg tttcgaaggg tttagagatg tgttgttgtt gttattttcg   21780
tttttattgt atttcgaagt tgtttttatt tgtttttttt atttttagta atacgtgtag   21840
gatggtcgga aggagcgttt tgagggtttc gtgaagattt ttgagaaaga gtttttagga   21900
gatatttatt cggggattta cgttttggat tgcgagatgg tgaggttgtt ttttgtttag   21960
cgtcgggttt aggattgggg aggatttggt ttggtgcggt cgttttattt ggttttattt   22020
gggtttggtt tatcggttag cgtttagggt tttttcgta gtatcggtg                22069
```

<210> 171
<211> 9724
<212> DNA
<213> Artificial Sequence

<220>
<223> chemically treated genomic DNA (Homo sapiens)

<400> 171

```
tttttcggcg tttaaggtag ggttagtttt atttttagga gacggggggt tttttttatta      60
tcggggtttt tgtgttttta ggcgtatgga ggagggtagt tttacggtgt tggagttttt     120
ttaatatggt ttgtttaggt agtatttcgg tatggttttt tggggttggg gtttcgggtt     180
aggtttgggt tttacggtat ttgaggaagg tgttttttag gaaggcggtt agttttattc     240
ggtcgttttg cggaaggagg gaggtatggg ggacggaggg gcgcggtttg tttcgtgcgt     300
ttttttcgtt aggtcgcgtc gtatcgttta gcgtatttcg tggaggaagt tatagtgtac     360
gagtttcgag gcgaggtttt gggcgttgtt cgttgaggta atggcgtaag gcgaggtatg     420
aggtcgttgt ttattcgtcg tttggggtta tcgttcgtcg tttaagtttt ttgtttagtt     480
tatttgggag taggtcgtag gttagttgtc ggtgtagtcg gttttttagt attagaagta     540
gagtgagttg gggaggcggc ggggcgtggt cggttggggg tttagggttt cgttacgttt     600
tcgttttttt tacggtagcg tcgttttttt aggtttcgtt tatatgttag cgcgttttgt     660
tttcgttttt ttttaggttg tattgtattt ggatgatttg tagcgggggga aggttgggat    720
ttataaattt agtagtaaga ttatatgtcg cggttttaaa ggggtcggga agggatagta     780
aagattggaa agggggggaat ttttaagagg agtttatttt attgttagtg gtttggtaag   840
ttaaggaaag tttcgagaag agtcgatatt aggggttggg tattgaaggt tgaataggag     900
tttttatcgc ggagagaata atttgaataa agagggaatt gtgggcgtgg ggaaagggag     960
gttgggcggg ataggatcgc gtcgaagtgt acggtgtttg cgggattagg tttttttagag   1020
tttgggcgtt agttgaggat ttttatgatt tttgttggta gttaggagta ggtagtttgt    1080
ttgttaggat atgaattttg gttgtattgt atcgagagag gtttttggtt aggaggggggt   1140
gttgtaggaa aagtttgggg agatattagg atatagatgt tcgggaatta gtattaggtg    1200
ggggatggga ggggtggggg gggggtggga ggggtgcggg ggtgggggaa gattgttggt    1260
agggtggaag agttcgggtg agttttggag gaaggatgtt tggagtaggt ttagggtagg    1320
tagtaggatg taggggaagg atttgagggg atagggagtt tttcggaaaa ggagagtgtt    1380
tagtttgttt tataattttt atgattttta taagggggtt agtttggatt tggtttttgg    1440
tagttaggag gagagttggg ttgtggggat gtaggtttta agagaaggag gggtattttt    1500
tatttggttg ttagaaggtt tgagggatgg tattgagtag taggagaggg gatttatttt    1560
tttggtttta gagttaaagg gttttggcgt cggggttttg gtttttttgga tttttttcgg   1620
gggtggggtt agtatacggg gtagttttag gggtcgagtg gttgtaaagt ttattagtgg    1680
gtagatttta tttttgggga tataataggg tggttggggg tttaggtttg atagttgttt    1740
ggtttttaat tttatattag tttttatttg atattttttta ggaatttgtt tagttttatg   1800
aaggagattt tcgagtattt ggtatggaag tgggaggtat atgaggggtt ggtcggggcg    1860
aggggagggg aggtgcgggg aggggatgtg tggggagggg aggtttttgg gtgttgattg    1920
tttatcgtta ttgtagcggg ggtttgcggg gtcggggaag tttcgttaag atcgcgtgta    1980
ggtttatggt tttggttttt tttttatta tattttttagg tatgaggttt gcggttataa    2040
gagtattaag gcggtgtgtt taggtcgtgg gttttgtagg gttttttttta cgttttaatt   2100
tttatttttt tagttatttt tcgggtcggt ttgttgttta tattggtgtt ggatgaagta    2160
gtgggttgtt aggagtttta gcgagtgtat ttcgaagagt acgcggtgga agaggaggtt    2220
gtgggtagag ggtcggcggg tagggtttcg ggttaggtag taaaggatga atttttgggg    2280
tatagggagg agaggttggt ggtgtgtaag ggttttttgt tttggtttcg tagttcgagg    2340
agaggtggtt tttgagtttt ttatgttttt ttttagggat atataggata tgtagggatg    2400
tttataggga ggagttttag tagtagtagt taggttagga tttggttttt tgagtttgag    2460
gatagttggg ttttgggatt ggagtattat gttttgtttt tttcgatttt taaaaaatcg    2520
tggggagaag ttatgatttt aaagttatat ttgtaagttt tttatttatg gtttttttgat   2580
cggtttagtt cgggtttaat attggagacg attttgatgg gtttttattt tgtttatatt    2640
cgtgtttagt tcggttttag ttcgttatta gtttcgatta ggtattttta tttttttgagg   2700
ttatggagtt aagtttagat ttcgagtaga gggggttgtg gatgtagttt ttattttata    2760
tttttttttg tttgtttggt atttattatg tttttttata agttggagtt ttattatttt    2820
ttggaagttt aaagttaagg atttggagtg aagtagattt aggtgtagat gttggtttta    2880
ttatttattt taaagttttg ggtaaaggat ttattttttt tgaattttgg tttttttata    2940
aaacgggtat ggtgatggta gttatagagg gagggttttt tttgtttttt tgtttttaga    3000
atgtgttgat tttttagggt ttttaggttt ggaattagta tgtgttttta ttgaggtttt    3060
ttttgggagg ggtgtagtta gttggtatat tttttaattg tataaattgt gatgtttttt    3120
taggtttgtt tggagtattt tgttagtttt agttgagtac ggtgagttag ttggggaggg    3180
gtagggtaag ttgtttattc gtatgttggg gttatttagc gagtgtttgg cgcgagtaat    3240
```

```
ggttttttt  gtgattcggg tgtttttatt ggtttggatt tttagtatag ttatttgggg    3300
tatagagtta ggttaggtat ggggaaggga ttatataggt gagttaggta gggtatagtt    3360
aggaggaggg tagagtataa ggtggagggc gggggtagta tttttagcgt atatatttta    3420
aaggagaaga tgtttatagc ggttttatcg gttatttttg aatagaagag agtataggat    3480
acgtaggaga gaagcgtagg gtaggtacgg ggtattcgga tttagtatta tttattttta    3540
tattttgggg ggaagaagtg taggtttcga agttttttc gtttagcgcg gatggggttt     3600
cggagattat ttggaagtgt tgtgggaggg cgtagagttg agcgggcggg gattttttta    3660
ggatttcgtt tttttaaagt tcgtatttat tattggagaa gattcggtgt tatattgtta    3720
aggggcggga gaaagagtgg agttagttgt tggggtatta gaattttttt gtttttggtt    3780
ttaggtattt ttttttgttt cgttttttta tttagttttg tttcgttagt atcggagtcg    3840
attttgatga ggtcgttgtg ttgaatgaag atggtgtcgt tggttaggtt ttcgtggatg    3900
attgggggt  tgtaggcgtg taggaagttg tagacgttgg ggaggggaga gtaggaggag    3960
tcggttagga ggttttggag agatgggggt tcggtggcgt cgcgtttagg ttagtttagg    4020
tttttatttg agcgtagata ggatttgcgt gtattagcgt ttttaggttt ggcggcggac    4080
gtacgatttc gtcggtcggg tgggcgtagg agaggcggtt gggtttgcgg agttcgtttc    4140
gtattttcgt ttagtttttg ttcgaggtcg tcgggtattt ttttattatt ttagttttcg    4200
aggttgtttt aatttcgttt tgttttcgtg gttgttttag ttcgtttttt atattcgggc    4260
gtttatggtt ttgtggtttt ttttggtttt tttgaggaat tgtttgaggt tgtttgatga    4320
tacgtatttt gtgatgaaga tgatttgtac ggtgcgagtt taggattttt attagttgcg    4380
ggttcgtttt tatgttcgtt ttatttagtt gtggtttttg tttgttcggg gttttgttcg    4440
tgtttatttt cgcgtaggtt ttagaggtat ttagttagta tttgtgtaat tttacgatgt    4500
tcgggtggtt tattagtatt agttgttcga atacggtttg gatttttttt tggggaggga    4560
gggtggtcgt tgggtggtta gtaggtggtt atttaagtag gatgaggagg gggtagcggt    4620
tttatttcgt gcgtcgcgaa ggttttttg  ttttcgaagt ggagttcgtt ttatattatt    4680
tttatttttt ttttcgtgtt tatggttagg aaggtgtttt gaagttttgg tatgtttttt    4740
tggtttattt gggggtgaat aaagggttat gtgtgttttg gtgtgtgtta gggttgtggg    4800
tgaggatttg gtttttgttt atattttttt agtgggttta agcgtgggtt gtaggttttg    4860
agttattttg cgggaaggtg gggtttggag ggtagttgtt tttaggagtt aagggttttt    4920
atttaagggt tgggttaagg ggatttggag taggttttag gggtcgaggg gggatttttta   4980
ggaagttagc ggttgagttt agaggtatgg ggaggtggtt ttgggaggag attggttttt    5040
agggagtttt agggcgagcg ttaggttaaa ggggtttagg ggtggttgat tcgcgggtcg    5100
cgaggggtcg cggagaggtt tttagtcgtc gcgttttttt agcgttttta cgtttcgcgt    5160
agtttttagg tttttttcgt tttgggaggg cggtgttatt tcgtgtttta attttcgcgt    5220
ttatttgttt tcgtcgtttt tgttagcgat tatacgggtt tttttttagg atgtcgtttt    5280
cgttttcgtt ttcgtttttt cgttttcgtt ttcgttttcg ggttcgtttc ggcgtcggtt    5340
tcggggtcgt tatggttcgg ttagtttagg ttatcgtttt ttgcgcgatt cgtcgtcggc    5400
gtagtttttt tcggttcgtt ttggtttcgc gtttagtagt ttagtttaga gtcgtcgcgg    5460
tagtttagag ttttagtttc ggttttggga attgggaggc gcgggcgcgc ggcggacggg    5520
cggggttcgg gggcggagtc ggcgcgtatt tttcgagtcg tttgtgtttg tcggagttag    5580
agtagagttt tttcgagagt tgtcgagttc gggtcgcgtt taggtttcgt tttttcggag    5640
gtttcgtttc gcgaagtttc gttttagttt ttgttttttt ttcgtttttt aggtttgttt    5700
cgcgaagttt ttttttaggt tttgtttttt tttcgttttt taagtttttt tcgcgaagtt    5760
tcgttttagg attcgtttta ttttcgcgaa ttttcgtttc gttttagttt tcgttttcgt    5820
tcgtttttag ggtttttcgc gaagttttat tttaggtttc gttttatttt ttcgaagttt    5880
cgtttttatg aggtttcgtt tcgcgtagag gaatcgtttt taaattttcg tttttttcgga   5940
ggtttcgttt tgcggatatt tggagttcgg agcgtttatt tttttcgcgg ttgttttttt    6000
aggatttggg ggaagattcg gggtatttgg gggtttttaa gtttacggta tttttttagtt   6060
gtagaagatt tcgcgggttc gaaagcggga ttcgtttttt tggttacgta tttatttttc    6120
ggtcgatttt taagggatag gtcgaagggg tatcgagggc gggggaaagg tttattagta    6180
ggttgattta ggttgatttt gatcggtgat tacgaatttt tttttggttc gtttattagt    6240
gttttttttc gtttataagt ttattttttt attttttagg atggcgattt tttttttttt    6300
ttttaaacgc gtattttttt ttcgcggttg gtttttatgg gatagtggaa ttatgagggg    6360
ggagtttatt cggtttatac gattttttt  ttcggaggtc gttttaagta tattttttag    6420
ttacgtcgat tatcggggtt gttttcgatt tttatttaaa attttttttta gtttatagtt   6480
tttacggttt ttttgtggtt tttttttttt tttacggcgg gtttttattt tttatttttt    6540
ttttttggag tttaaattgg aatttcgttt tttttttttcg cgtagtttta tgtattggtt   6600
ttttgggacg tttttttttt tttttggtag aggtgtattt ggttttgttg cgttttttgt    6660
ggtttttttt ttttgattag ttttggttat taggtatttt gtgtgtggtt tttattaatg    6720
ttaggtgatt ttgttgaggt tttttgtagt aaaggtttgg ggtgtttaaa tttttttattt   6780
tgaaattggt tttgattagt ttttgggaga tattttttaa gtttttggaa tgttttgttt    6840
gatttgaatg tttgtgttta tttcggttat attagatttt atgttaagtt tgtttaattc    6900
```

```
gtagtttaga gggtgtattc ggttaaggat agttttgaat atggtttaat ataaatttat    6960
aaattttttt attgagattt tttttaaagt ttattagttg ttgttagtgt taagtgtatt    7020
ttatttaagg gtattgttta ggataatttt tttaatgtgt gttaggaaag tttatagatt    7080
ggatattttt ggtttgtgtt aataatatgg tttacggggg gattttgggt tatgtggtat    7140
tagtttgatt tttgtagggg ttggaggtta aagttagtta tttgggaggt tagttatgtt    7200
tgtgtgatta atttaataaa aattttggat attgaggtac ggagggtttt tttggttgtt    7260
gatatgttgt gtttattgtt atattttagt gttgggaggt tgagtattgt ttttatgttt    7320
gtagtgggtg gggatatttg tagattcgtg ttggtttgtt ttgggttttg ttttgtgagt    7380
ttttgttatt gttgatttta acgtatttat tgttgtaata aatagtattt ttttcgagtt    7440
ttgtgagttt ttttagtgaa ttattgaatt tgagggtggt tttggggagt ttttttagtg    7500
gttttttttt atttggtatg agggtttaaa tatgaaggtt tagtaattgg ttttattatt    7560
ttattttacg ttagttgttt tttttagttg agtttatttt aagtatacgg ttttgtgtta    7620
tcggatatga taggtatttt tttttagggt ttttgttttg gttatatttt tgttcggaac    7680
gttttttttta aatatttttt gaggttattt tttgagaggt ttgttttgat tacgaatttt    7740
gatattatag tttttatatt ttttcgtttg tttgatttga ttttttttcgg agtatttatt    7800
ttatttgatt tgtatttggg ttttttttat tttttttttt tattgtttgt gtttttgttg    7860
cgattcgagt tttcgtaggg taggagtgtt tgttcgttgt ttttgttttt tttttggtgc    7920
gaggacggta ttcggtatat agtaggtttt tagtaaatat ttgttgaatg gatttgtggg    7980
tgggtagggg tgcgttttgg cgggtgtggt ttagagaagt ttttaaggag gtaagttttg    8040
agtagaagaa ttttaatgaa tttttttttt ttttttcgtt tttataatta gatatatatg    8100
ttttgtggta ggttatatag tgttttttaa aagtttatat tttgattttt agtatttagg    8160
agtatagttt tatttgaaaa aaaaaaaatg gtttttgtag atgtaaataa gtttaggatt    8220
ttgagatgtt ggggtgggtt ttaaatttaa tgtttgatgt ttttattaga gaaagaaaag    8280
ggatatttgg ggtgtagaga aataaggtag atagttatat gaatatggag gttagtgttg    8340
gagggatgta tttataggtt aaggcgtgtt tgggattcga ggatacgagt tgttttagta    8400
taataaaata tataaaataa gaatagttgt attgatatag attttagata tgattatata    8460
tgaatattat taattattag tttgtagtaa ttatttttta ttttaatatt ataataattt    8520
ttattttata attatagttt aggaaaaatt aggttatata gagataggag ttaaggggat    8580
atagtgagga gtaattagaa gataagagtg cgagtttttt gttatgttta gatagggtta    8640
ttagagggtt ttttggttta gcggtaacgt tagcgtttgg gaagatgttt gttgttaagt    8700
ggattttggt ttagcggtag gcgtagtgtt aaggaaaaat atttgttatt tagtagatcg    8760
ggaaagggag tttttttttgt ttcggtggag tttaaagaag attttatttt tttatttttt    8820
gtggagggtt tgatattagt taggttcgtt tgtagttatt cggaggttta atcgtttttt    8880
tgtgatgttg tgttttagcg gttatatttt tagttcgttt ttatgtttta ttttgtatat    8940
ttggttttgt tttttagata gtagtagtaa attagtgaaa gtattaaaag tttttgatat    9000
gtagaaataa tggcgtaggt tgtttttttt tttgtttttt tttttttttt gtttcggttg    9060
ttaggtaggg aagggttttt tgtttagtgg atatatgatt tacgtggttt tatttattat    9120
tggagatggt ttattttttt tattttgttt ttttgttttg tatttaataa atattagtgt    9180
agtttggtat tcggggttat tatcggtttt cgcgatttgg tggtagtggt ttttcgggtt    9240
tagttgtttt ttttttttatt tttgttttgt gtttttattt ttatattttt tcgtttttgt    9300
atacggggag agatttatcg tttttgtggg gttggatttt atttgggatt gttagggtta    9360
tgagtggtga ggagagagtt ttgggatgga tttttttttcg gagttttcgg aagtattagt    9420
tttatcgata ttttgatttt gtatttttag tttttagtat agtgagcgaa tttgtttgtg    9480
ttgttttacg ttattttttt atttttggtg ttgttttagt atgattttcg tggtaagaat    9540
agtaacgtga taatagttat gtgttcgcgt gtagggatgc gttttagtat ttatttttat    9600
gtatagagtg ggtcgtttta ttgtttgttt acgtatagaa gtggtggtcg ttgcggtata    9660
tttttttttgg ttttatttag gttgtgtttt agggcggggg cgtatttttt tttttgttcg    9720
gttt                                                                 9724
```

<210> 172
<211> 9724
<212> DNA
<213> Artificial Sequence

<220>
<223> chemically treated genomic DNA (Homo sapiens)

<400> 172

```
aggtcgagta gaagggaggg tacgttttcg ttttggggta tagtttgagt gaggttaggg      60
agggtgtgtc gtagcggtta ttatttttgt gcgtgggtag gtagtaggac gatttatttt     120
```

```
gtgtatggga ataaatgttg ggacgtattt ttgtacgcga atatatagtt gttgttacgt     180
tattgttttt attacggggg ttatgttgga gtagtattag agatggagga gtagcgtaaa     240
atagtataaa tagattcgtt tattgtgttg gaggttggaa gtgtaaaatt aaggtatcgg     300
tagggttgat gttttcggag gtttcgaggg agaatttatt ttaggatttt tttttttatta     360
tttatggttt tggtagtttt aagtagggtt tagttttata gggacggtgg gttttttttc     420
gtgtgtagag acgagagagt gtagaaataa agatataaga tagagataaa agaaaaggta     480
gttgggttcg gggattattt attattaagt cgcggagatc ggtagtggtt tcgaatgtta     540
ggttgtattg atatttattg gatataagat aaaggggtag gataaggaga gtgagttatt     600
tttaatgata ggtaaggtta cgtgggttat gtgtttattg gatagggggt ttttttttgt     660
ttggtagtcg aggtagagag agagaggaga tagagagaaa gatagtttac gttattattt     720
ttgtatatta gagattttta gtatttttat taatttgtta ttgttattta gaaggtagag     780
ttaggtgtat aggatggaat atgaaggcgg attaggagtg tgatcgttga agtatagtat     840
tatagggaga cggttaggtt ttcggataat tgtaggcgag tttgattaat gttaggtttt     900
ttataagagg tggaggagta gagtttttttt taaattttat cggggtaagg gagatttttt     960
ttttcggttt gttaagtagt aggtgttttt ttttgatatt gcgtttatcg ttagattaag    1020
gtttatttgg taataggtat ttttttagac gttggcgtta tcgttagatt aaggagtttt    1080
ttggtggttt tgtttgggta taatagaagg ttcgtatttt tgttttttgg ttattttta    1140
ttatgttttt ttagttttta tttttgtatg gtttggtttt ttttaggtta tgattataga    1200
gtgaggatta ttataatatt ggaataaaga gtaattgtta taaattaatg attaatgata    1260
tttatatata attatattta agatttatat tagtataatt atttttattt tatatatttt    1320
attatattgg aatagttcgt gttttcgagt tttaggtacg ttttgatttg tgggtatatt    1380
tttttaatat tggtttttat gtttatgtga ttgtttattt tattttttttg tattttaagt    1440
attttttttt tttttttgat aaggatatta aatattggat ttagggttta ttttagtatt    1500
ttaagatttt aaatttattt atatttgtaa agattatttt ttttttttta aataaggttg    1560
tgtttttagg tattggggat taggatgtag atttttgggg gatattatgt gatttattat    1620
agaatatgtg tgtttggtta tgggggcgga gaaaggggag gaaatttatt gagatttttt    1680
tgtttaaggt ttattttttt aggggttttt ttggattata ttcgttaaaa cgtatttttg    1740
tttatttata gatttattta ataagtgttt gttgagggtt tgttgtgtgt cgggtatcgt    1800
tttcgtatta gagaaagagt agggataacg agtagatatt tttgttttgc gggagttcga    1860
atcgtaatag ggatatagat agtgaagaag agagatgaga aaaatttaga tgtaagttag    1920
gtggggtgaa tatttcggga aaagttaagt taagtaggcg aaagggtgtg gggattgtga    1980
tgttaggatt cgtggttagg ataggttttt taggaagtga ttttagagga tgtttgggaa    2040
gggcgtttcg ggtaaaggta taattagggt agagattttg aggggaagtg tttgttatgt    2100
tcgatggtat agggtcgtgt atttggggtg ggtttagttg aagaaggtag ttgacgtgag    2160
gtagggtaat gggattaatt attgggtttt tatgtttagg tttttatatt aagtgagaag    2220
ggattattgg gggggttttt taaaattatt tttaagttta atggtttatt agaaggattt    2280
atagaattcg gaaagggtat tatttattat agtaatggat gcgttaaaat tagtagtggt    2340
aaggatttat agaatagaat ttaggataga ttagtacggg tttgtaggtg tttttattta    2400
ttgtaggtat ggggatagtg tttaattttt tagtattgag gtgtgataat gagtatagta    2460
tgttagtagt tagggaagtt tttcgtattt tagtgtttag ggtttttatt gggttggtta    2520
tataggtatg gttgattttt taggtggttg attttggttt ttagtttttg tagaggttag    2580
gttgatgtta tatggtttaa ggtttttttcg tagattatat tgttagtata gattaggggt    2640
gtttaatttg tgggtttttt tggtatatat tggaagaatt gttttgggta atatttttaa    2700
ataaaatata tttaatatta ataatagttg atgagtttta aaaaaaattt taataagaaa    2760
gtttatgaat ttgtgttggg ttatatttaa agttgttttt ggtcgaatgt attttttggg    2820
ttgcgggttg gataagttta gtatagaatt tggtgtggtc gaggtaaata tagatattta    2880
aattaggtag aatattttaa gggtttagag ggtgtttttt aggagttggt tagggttagt    2940
tttagggtgg aggatttgaa tattttaaat ttttattgta gagggtttta gtaggattat    3000
ttggtattgg tggaaattat atataaggtg tttggtgatt aaggttggtt agaaaagaga    3060
gattataggg gacgtagtag ggttagatgt atttttgtta aagaaagaaa gaggcgtttt    3120
aaagggttag tgtatggagt tgcgcggagg ggaggagcgg agttttaatt tgggttttag    3180
agagagaagg taaaggatga ggattcgtcg tgaaggaggg aggagattat aggaaggtcg    3240
tgggggttgt gggttgaagg gggttttagg tgagaatcgg ggatagtttc ggtggtcggc    3300
gtggttggag ggtgtgttta gagcggtttt cggagggaga ggtcgtgtga gtcgggtggg    3360
tttttttttt atggttttat tgttttatgg gagttagtcg cggaaaggaa atgcgcgttt    3420
gaggagaaaa gagaaaatcg ttattttgga aagtgagaag atgggtttgt gggcgggagg    3480
aggtattggt gggcgggtta gggagagatt cgtggttatc gattaaagtt agtttgggtt    3540
agtttgttgg tgggtttttt tttcgttttc ggtgtttttt cgatttgttt tttgggggtc    3600
ggtcggggaa tgagtgcgtg gttaggagag cgggtttcgt tttcgagttc gcggggtttt    3660
ttgtagttgg agggtgtcgt gggtttgggg gtttttagat gtttcgggtt ttttttttaga    3720
ttttagggga gtaatcgcga gagaagtggg cgtttcgggt tttaggtgtt cgtagggcgg    3780
```

```
ggttttcgag ggggcgggga tttgggggcg gttttttttgc gcggggcggg gttttatagg    3840
ggcggggttt cgaggaggtg aggcggggtt tggggtgggg tttcgcggag gattttggga    3900
acgggcgggg gcggggatta aaacggggcg gggattcgcg ggggtggggc gggttttggg    3960
gcggggtttc gcgaaggagg tttggaggac gaggaggggga tagggtttgg ggaggggttt    4020
cgcggagtag gtttgggaga cgaggaggaa atagggattg gggcggggtt tcgcggggcg    4080
gggttttcgg gggggcgggg tttgggcgcg gttcgggttc ggtagttttc ggggaagttt    4140
tgttttggtt tcggtaggta taggcggttc gggaggtgcg cgtcggtttc gttttcgggt    4200
ttcgttcgtt cgtcgcgcgt tcgcgttttt taatttttag agtcggggtt ggggttttag    4260
gttgtcgcgg cggttttagg ttgggttgtt gggcgcgagg ttagggcggg tcgggagagg    4320
ttgcgtcggc ggcggatcgc gtagagggcg gtggtttggg ttggtcgaat tatggcggtt    4380
tcggagtcgg cgtcgaggcg ggttcgggaa cgggagcggg agcgggagga cgagagcgag    4440
gacgagagcg atattttgga ggaaagttcg tgtggtcgtt ggtaaaagcg acgggagtag    4500
gtgggcgcgg gggttggggt acggggtgat atcgtttttt tagagcggga ggggtttgga    4560
ggttgcgcgg ggcgtggggg cgttgggaga gcgcggcggt tggaaatttt ttcgcggttt    4620
ttcgcggttc gcgaattagt tatttttgga tttttttggt ttggcgttcg ttttgggatt    4680
ttttgagggt tagttttttt ttaggattat ttttttatgt ttttggattt agtcgttagt    4740
tttttgggga tttttttcga ttttttgaaa tttgttttaa atttttttag tttagttttt    4800
ggataggagt ttttggtttt tgggggtagt tattttttaa gttttatttt ttcgtagagt    4860
ggtttagggt ttgtagttta cgtttgggtt tattggaaag gtgtggatag ggattaaatt    4920
tttatttata attttgatat atattagggt atatataatt ttttatttat ttttaggtaa    4980
attaagggaa tatgttaggg ttttagagta tttttttagt tatggatacg gaggaggggg    5040
tagaggtggt gtggaacgag ttttatttcg gagataggaa ggttttcgcg gcgtacgagg    5100
tgagatcgtt gttttttttt tattttgttt gggtgattat ttgttgatta tttagcgatt    5160
attttttttt tttaggagaa gatttagatc gtgttcgagt agttggtgtt ggtggattat    5220
tcgaatatcg tgaagttgta taagtattgg ttggatattt ttgaggtttg cgcgagggtg    5280
agtacgggta aggtttcggg taggtagaga ttatagttag gtggggcggg tatggggacg    5340
aattcgtagt tggtggaaat tttgagttcg tatcgtgtag gttattttta ttatagagta    5400
cgtgttatta ggtagtttta agtaattttt taaaaagatt aagaagaatt ataaggttat    5460
gaacgttcgg gtatggggag cgggttgggg tagttacggg gataggacgg ggttggggta    5520
gtttcgggga ttgggatggt gagggggtgt tcggcggttt cggatagggg ttgggcgagg    5580
atgcggggcg ggtttcgtag gtttagtcgt tttttttgcg tttattcgat cgacggagtc    5640
gtgcgttcgt cgttaggttt ggaagcgttg gtgtacgtag attttgtttg cgtttaggtg    5700
agggtttggg ttggtttggg cgcggcgtta tcgagttttt atttttttag agtttttga    5760
tcggtttttt ttattttttt ttttttaacg tttgtagttt tttgtacgtt tgtagttttt    5820
taattattta cgggaatttg attagcgata ttatttttat ttagtataac ggttttatta    5880
agatcggttt cggtgttggc ggggtagggt tgggtggggg aacggggtag gggaaggtgt    5940
ttggagttaa gggtagagga gttttgatgt tttagtagtt aattttattt tttttttcgt    6000
tttttggtag tgtggtatcg aattttttttt aatggtaagt gcggattttg gggggacggg    6060
gttttggaga ggttttcgtt cgtttagttt tgcgtttttt tatagtattt ttagatgatt    6120
ttcgaagttt tattcgcgtt gagcgagagg aatttcggaa tttgtatttt ttttttttag    6180
agtatggagg tgagtggtgt tgggttcggg tgtttcgtgt ttattttgcg tttttttttt    6240
acgtgttttg tgtttttttt tgtttagagg tggtcgatgg gatcgttgtg gatatttttt    6300
tttttgggat gtgtgcgttg gaggtattgt tttcgttttt tattttgtgt tttgtttttt    6360
ttttaattgt gttttgtttg gtttatttgt gtggtttttt ttttatgttt gatttggttt    6420
tgtgttttag atggttgtat tggaaattta gattaatggg gatattcggg ttatagagga    6480
ggttattgtt cgcgttaggt attcgttgag tgattttaat atgcgggtaa gtagtttgtt    6540
ttgttttttt ttagttggtt tatcgtgttt agttagggtt agtaaggtgt tttaggtaga    6600
tttgggagaa tattatagtt tgtgtagttg aggggtatat tagttgattg tatttttttt    6660
aaagaggatt ttagtgggag tatatattgg ttttaggttt gaaggtttta aggggttagt    6720
atattttaag gataagaagg taagagagat tttttttttg tggttgttat tattatgttc    6780
gttttataaa gaaattaagg tttagaggag atgagttttt tgtttaaggt tttagagtga    6840
gtggtggagt taatatttgt atttgggttt gttttatttt aagtttttga ttttaagttt    6900
ttaggaggtg atgaggtttt agtttgtggg gggatatggt gggtgttaag taggtaaaaa    6960
gaggtatggg atggaagttg tatttatagt ttttttttgtt cggagtttag atttaatttt    7020
atggttttag gagatggggg tgtttggtcg gggttggtgg cgagttggag tcgggttggg    7080
tacggatgtg gatagagtgg ggatttatta aggtcgtttt tagtgttggg ttcgagttga    7140
gtcgattaaa aggttatggg taggaggttt gtaggtgtga ttttaggatt atggtttttt    7200
tttacgattt tttggggtc gagggaagta gagtatggtg ttttaatttt agaatttagt    7260
tgtttttaag tttagaggat taggttttgg tttggttgtt gttgttggga ttttttttta    7320
tgggtatttt tgtatgtttt gtgtgttttt gaggagggat atggggaatt taggggttat    7380
tttttttcga attgcggggt tagagtagag agtttttgta tattattagt tttttttttt    7440
```

```
tgtgttttag gagtttattt tttgttgttt ggttcgggat tttgttcgtc ggttttttgt    7500
ttatagtttt tttttttatc gcgtgttttt cgaggtgtat tcgttgaagt ttttggtagt    7560
ttattgtttt atttagtatt agtgtgagta gtaggtcggt tcggggggtg gttggggagg    7620
tggaggttgg gacgtggaag gggttttgta ggatttacgg tttgagtata tcgttttgat    7680
gtttttgtgg tcgtagattt tatgtttgag aatgtggtgg aggagaagat taaggttatg    7740
gatttgtacg cggttttggc ggagtttttt cggtttcgta ggttttcgtt gtagtggcgg    7800
tgagtagtta gtatttagaa gttttttttt tttatatatt ttttttcgt atttttttt     7860
ttttcgtttc ggttagtttt ttatgtgttt tttattttta tgttaggtat tcggaagttt    7920
tttttatgga gttggataaa tttttggagg atgttaggtg agaattgatg taaggttggg    7980
ggttaggtag ttgttaggtt tgaattttta gttattttgt tgtgttttta ggaatggaat    8040
ttatttattg atgaattttg tagttattcg atttttgggg ttgtttcgtg tgttggtttt    8100
attttcggag gaggtttaaa aggttaagat ttcgacgtta gagtttttttg attttgagat   8160
tagaaaggtg agtttttttt tttgttgttt agtgttattt tttagatttt ttaatagtta    8220
gataagaggt gttttttttt tttttagggt ttgtattttt atagtttagt tttttttttg    8280
gttgttagag attagattta ggttgatttt tttgtggggg ttatggggggt tataggatag    8340
gttgggtatt tttttttttc gggaggtttt ttgttttttt agattttttt tttgtatttt    8400
gttgtttgtt ttgaatttat tttaagtatt tttttttttag aatttattcg ggttttttta   8460
ttttgttagt aatttttttt tattttcgta ttttttttat ttttttttta ttttttttat    8520
tttttatttg gtgttgattt tcggatattt atgttttggt gtttttttag attttttttg    8580
tagtattttt ttttggttag gggttttttt cggtgtagtg tagttagaat ttatgtttta    8640
ataggtaaat tgtttgtttt tggttgttag tagagattat gggagttttt agttgacgtt    8700
taggttttgg gaggtttgat ttcgtaggta tcgtgtattt cggcgcggtt ttgtttcgtt    8760
taattttttt tttttacgt ttatagtttt tttttttgttt aagttgtttt tttcgcggtg    8820
aaagttttta tttagttttt agtatttagt ttttaatgtc gatttttttc gaagtttttt    8880
ttagtttgtt aagttattag tagtgagatg ggtttttttt agagattttt tttttttttag   8940
tttttgttgt ttttttttcgg ttttttttggg gtcgcggtat gtggttttgt tgttgggttt  9000
gtgagtttta gttttttttc gttgtaggtt atttagatgt agtgtaattt ggagagaagc    9060
gaggataagg cgcgttggta tgtgagcggg gtttgggagg gcggcgttgt cgtggggaag    9120
gcgggagcgt ggcggagttt tgaattttta gtcgattacg tttcgtcgtt tttttagttt    9180
atttgtttt tggtgttgga agatcggttg tatcggtagt tgatttacga tttgttttta     9240
agtaggttgg gtaggggatt tgggcggcgg gcggtgattt taggcggcgg gtgggtagcg    9300
gttttatgtt tcgttttacg ttattgtttt agcggatagc gtttaggatt tcgtttcgga    9360
gttcgtgtat tatggttttt tttacgaggt gcgttgggcg gtgcggcgcg gtttggcgga    9420
gggggcgtac ggggtaggtc gcgttttttc gtttttatg tttttttttt ttcgtaggac     9480
gatcggatga agttggtcgt ttttttggag agtatttttt ttaagtatcg tgggatttag    9540
gtttgattcg gagttttagt tttaggggat tatgtcgggg tgttgtttgg gtaggttatg    9600
ttggggagat tttagtatcg tggggttgtt tttttttatg cgtttgggag tataaaggtt    9660
tcggtagtga aggaattttt cgtttttttga gagtggggtt gattttgttt tgggcgtcga   9720
gggg                                                                 9724
```

<210> 173
<211> 1301
<212> DNA
<213> Artificial Sequence

<220>
<223> chemically treated genomic DNA (Homo sapiens)

<400> 173

```
aagtagtcgg tgtcgttttt gaatatattc gggtagggcg agtaggatgt ttttgtttag      60
ggagattttt tttttggtt ttggggaagg tgttttttat tttcgtagga ggttgggttt      120
gtaggacgta gtattgggcg ggtgagagaa agtttgggta gaagagtacg gagtcggcga     180
ttggacgcga gtaaggggcg aatggagagc gttttttttg cgttaatgtt gttgtttcgg     240
gtgttgaaaa gtcgggaatt tttcggaaag tagcggtatt tttttttttg gaaggtgggt     300
gtgggggttt taggtttcgt atttgttaaa tttgtttcgt tatttgggag gggttcgtta     360
gatgtgtttt tttttagttt tattttgggg agaacgatgg gggcgggggc gtgggggtga     420
gtatagtcgg gtattttcgt atattcgtag gcgggatttc ggggtgtttt tgtttttttt     480
ttttgggtag tcgtacgtcg ggatttttat tttatttgcg cgcgaatttt cgttatttgg     540
gatttaagcg ttcgcggttt gagcgttgcg gtaaatagaa ggtattcgcg tgggttttta     600
agggtgtttg tgtgttgggt ttgggggagt atgatcgggg tattcgtagt ggggaggaag     660
```

aggtgttgtt ggtcggggcg agttgtttgt gttaaggtcg ttttttgtag ggcgagttcg 720
tgtacgacga cgtgtgggcg atcgtgaata attttaacgt gcggttcggc gggcgtttcg 780
tttcgttgag gtatttttat taacgatttt ttgggttagg gtatggtcga gaatattagt 840
tataagtttt agcggtcgtt tagcgttttt atttttaagt gtatttttgt ttagggtttt 900
tttttgagtt agggtcgggt gtagttgcgg gattggtttg aagggaattt ttattttttta 960
ttacgttttg gtttttttgt ttttttgttt tttttatttt agttgtttac gttattaggt 1020
ttttaggttt tttttaggtt aatttgggtt tttgggatta ggcgggaggc gcgcgttggg 1080
gagttttttt cgagttttta gagtttttcg aggtgtttgc gttttaggtt gtttgttttt 1140
tttggataga tttgggaatg ggcggttgga gcggttattt tatttttttt tcgttagttt 1200
ttttgggtga gtaagaaaat tagttttata tagttggttt cggaggtgtg gtgtgtgtgg 1260
aaggggcggg ggaaatgttg tttttagggt cgtaattttt t 1301

<210> 174
<211> 1301
<212> DNA
<213> Artificial Sequence

<220>
<223> chemically treated genomic DNA (Homo sapiens)

<400> 174

gagaaattac ggttttgaaa atagtatttt tttcgttttt tttatatata ttatattttc 60
ggggttaatt gtgtaggatt ggttttttttg tttatttaga ggggttggcg gaagggaagt 120
gaggtggtcg ttttagtcgt ttatttttag gtttgtttag agaaaataaa tagtttagaa 180
cgtagatatt tcgggaaatt ttggaaattc gaagggaatt ttttagcgcg cgtttttcgt 240
ttggttttag gaatttagat tagtttggag aggatttggg agtttggtgg cgtgaatagt 300
tggagtggga gaggtaggga ggtagagagg ttaaggcgtg atgagaaata gaaatttttt 360
ttaagttagt ttcgtaatta tattcggttt tggtttaaag ggaagtttta gataggagtg 420
tatttgaagg tggggacgtt gagcggtcgt taggatttgt ggttggtgtt ttcggttatg 480
ttttggttta agaagtcgtt ggtgaagatg ttttagcgga gcggggcgtt cgtcgggtcg 540
tacgttgggg ttgtttacgg tcgtttatac gtcgtcgtgt acgaattcgt tttgtaggga 600
gcggttttag tataggtagt tcgtttcggt tagtagtatt tttttttttt tattgcgaat 660
gtttcgatta tgttttttta gatttaatat ataaatattt ttgggaattt acgcgggtgt 720
tttttgttta tcgtagcgtt taagtcgcgg gcgtttgagt tttagatggc gaaagttcgc 780
gcgtaggtgg agtggggatt tcggcgtgcg gttgtttagg agaggagggt aagggtattt 840
cggggtttcg tttgcgggtg tgcgggggtg ttcggttgtg tttattttta cgttttcgtt 900
tttatcgttt tttttaaaat ggggttgagg aggggtatat ttggcggatt ttttttaggt 960
gacgaggtag gtttagtagg tgcgaagttt ggaattttta tatttatttt ttagggagaa 1020
aagtgtcgtt gtttttcggg aagttttcgg ttttttagta ttcggaataa tagtattggc 1080
gtaggggaga cgttttttat tcgtttttttg ttcgcgttta gtcgtcggtt tcgtgttttt 1140
ttgtttaggt tttttttttat tcgtttagtg ttgcgttttg tagatttagt tttttgcggg 1200
gatagggagt attttttttta aagttaggag aaggggtttt tttggataag gatattttgt 1260
tcgttttgtt cgggtgtgtt tagaggcgat atcggttatt t 1301

<210> 175
<211> 22355
<212> DNA
<213> Artificial Sequence

<220>
<223> chemically treated genomic DNA (Homo sapiens)

<400> 175

aggtgaatat atagtatttt tcgggtttta tattagtttt gggcgttagg gatatagtag 60
tgaatagaat agatggagtt tttttttttt gtagcggggg gttgtaatag gggggtttat 120
tggataagga gtatattttt attagagaga atattttagt tgggtgagcg gttttgtagg 180
gggttagttt gggggaattt ttggagacgg tgggaattga ataaagtttt tggggaggtt 240
aggtacggtg gtttatattt gtgattttaa tattcgggga ggttgaggtt atagggagtt 300
atgattatat tattgtattt tagtttgggt gatagcgaga ttttgtttta aaaatttaaa 360

```
aattgggtta ggcgtagtgg tttatgtttg taattttagt attttgggag gttaaggagg     420
gtggattatt tgaggttagg agttcgagat tagtttggtt aatatggtga aattttgttt     480
gtattaaaaa tattagagat taggttaggt atagtgattt atatttgtaa ttttagtatt     540
ttgggagatc gaggcgggtg gtttatttga ggtcgggagt tcgagattat tttgattaat     600
atagagaaat ttcgttttta ttaaaaatat aaaattagtc gggtatggtg gtatatattt     660
gtaattttag ttattcggga ggttgtaatt ttagttattc gggaggttga ggtaggagaa     720
tcgtttgaat tcgggaggcg gagattgtag tgagttgaga tcgtattatt gtattttagt     780
ttgggtaata agagtaaaat tttattttaa aaaaaaaaaa aatataaaag attaggtgga     840
ggttgtagtg agttgagatc gcgttattgt attttagttt gggtaataga gtaagatttc     900
gttttaaaaa aaaaaaaaat ttaaaaagcg ttttgaagga gttggggggg gagagaagat     960
agggtcgtta gggttttgag cgtagcgtta gtttgtgtgg ggtatgtagg ttgtgtttta    1020
cgtttgtgtt gggagagaga gtagatgggg tgtttggttt tgtgagtggt tgtgagtatg    1080
tgtgttcgag ggtaaggggg attttgtttc ggttgtatgg gtgttaggtt tacggtatgt    1140
ttcgggtgtg tgttgtgcgt gcgtgtattt gtacggtatg tgcgtgtatg ttgatatggt    1200
agtcggtgta tttgtggttt ttgtcggtag atacgtgttt tttagttttt cgtttttttt    1260
tattcgtttt ttttttattt tttatttttt tgtttttatt ttattttttt gagttgttat    1320
gtggttatta ttatttttgt ggtttttgtt tttagggttt ttgtgtattt ttttttttta    1380
gtgtttattt gaattttttt ttttattttt ttatgtttta gtttgtggtt cggtagtttt    1440
tttttaggt ttcggggtta ttttcggaag gtttggtggg ttttgttagg gaaggggttt    1500
ataggatttt tcgagggttg tggtcgttaa tcgttgagtg tagggtgtga gcgggattcg    1560
gatatttttt gagggcggga attaggtagt gttttaagag atgttaggtg tatagggggtt    1620
ggggggttggg ttgggggggag tataggtttt cgggagttag aaggggattg gggttgtggt    1680
ttaggttatg gatagagtag aggcgtaggg atttgaagaa gtagagggag aagtttggtt    1740
aatatagcga gattttattt ttatttaaaa gataaaaatt agttgggtgt ggtggcgtgt    1800
atttataatt ttagttattt aggaggttga ggtaggagaa ttatttgaat ttgggagggg    1860
gatgttgtag tgagttgaga ttacgttatt gtattttagt ttgggtgata gagcgagaat    1920
ttattttaaa aaaaaaaaaa aatagtggga ggtgacgtgg tgttgtagag gcggtagtgg    1980
ttaggatagc gggaggggta cgggaggtga ggtttttagg ttagttatga acgttgggtt    2040
tggttgtgtt ttaaggtttt tagagttttt tttttgtttt tgtttggttt tttaggaggg    2100
gagttagggg gtatagtttt tgtaggggtt ggtaggatta gatagggggta atgttgggtt    2160
ttttgtattg ggattgaggc gtttttttgt ttttttttatt ggttgagatt agggttttgg    2220
gtatacggga gggacggggt ttagttgtta gtatggagcg ggtttgtttg tttatattag    2280
tttttgggta ttagttatta ggagtttgta gaattgtggg gagaaaaggt ggacgaggta    2340
ggaggaaaag aggggagagg ggtttgttgg ttgagggttt tggagagcgg taggttttag    2400
tttttgtggg aggggtattt tggtaggtgt gtgtagtcgt tgttagggtt tttaggatgt    2460
gacgtgttag gattggtttt tttggaggtt acgttagtat ttcggttttt gtttttgtat    2520
tttgttgtta gtttagttag ggagatttta gttttttcgg gaagttgttt ttttgtttta    2580
ggtttttttt tggtttgttt ttgtagcgtt ttttttgggt atttgggttt tagttttggt    2640
ttttgtttgt ttgtttgttt ttttgttttttt tatttttaat gtcgtttgtt tattttaggg    2700
tgtttgttgt ttgttcgtcg tttgtttatt cgttgtagta tgttcgagtt gttcgtatgt    2760
aggttgttgg atgagttaat tttcgtttgg tttagtattg gttttatttg gtttcgttag    2820
tttttgtagg agttgtgttt tcgagttttt tttttagagg atgtaggttt tttgaggttt    2880
tgttttgttt ttatgggttt tagttttgta ggttaggtta ggaggttttt atgttagttt    2940
attggttcga ggggggtttt ttttgatttc ggaatagaag atagggtaag gtagtttgaa    3000
gtttgggttt cgtgttcgtt gtttttttag cgtggttttg ttcgtttttg tttgggagtt    3060
aattttagtg ttattagtag agttatagtt tgggttagtt ttggtaggta tttcgttttg    3120
gtttatttat tttgtgggtg ttgtggtttt gggtttgttt ttttgggtaga agtgggagga    3180
agtgtatata tttgttaggg aggttttttg gtcgagggat attttaggtt tttatttcgt    3240
tttttcgtta gcgtagattt ggggtttttt ttttttttgtt agtttttttt attttttttt    3300
aggttagggg atgagataag ggattttgag aatagtttgg ggtgggaggg agatttttagt    3360
tttttttagtt tagtagggta gggggttgtg ttagtttagt tcgggaaatt taggagggga    3420
aggtgagggt ttcgattttt ttttggagtg ttagatgtgg agttgatatt agttgggcgt    3480
tgattttttt ttttgttatt ggtttttatt tttttagttt tgtagttttc gttggttaac    3540
gatgggggta gtcgttcgtt atttttagag agtttttcgt agtattttac gtttttcgtt    3600
cggtttcgtt atatgttggg gttttcgtta attttgttta tatttcgtag tatggagagg    3660
tgagttaggg gtttagtagg gttgggtggg aagtagtatt gaggggttat cgtgttagtt    3720
ttgggaggag ggtggttaaa tcggtgggaa tttcggtatg ggggattggg gtggttaatt    3780
taaggttagg atttattttt atcggtattt gttttatgtt tttagtattg agattgatta    3840
gaagttgtag gagattatga agtagacggg ttatttgatt atcgggggtt aggtattatt    3900
tttattgtgg cggggagagg gaggaggttt agttaggttg gatttatttt gggagcgtta    3960
gtgggggggta gggggcggtg gtagaggttt tagggatttt ttaatttttt tttttttttt    4020
```

agcgttatta ggtagaaatt aacgatttgg agaatttggg cgagatgggt agcggtattt 4080
gcggttaggt gtggaagatg cgttttcgga agatcggtta cgttattgtc gttaaggtga 4140
gttttggcgg ttatttcggt tgcgttttat attttaggtc ggtgttgagg tttttttttg 4200
tttttgtttg tgtagtaaat gcggcgtttc gggaataagg aggagaataa gcgtattttt 4260
atggatttgg atgtggtgtt gaagagttac gattgttttt atatcgtgta gtgttttggg 4320
acgtttatta ttaacgtgag tatttggtcg cgttttgtag cgtttttttt tttttatttt 4380
ttgttttttt ttagggagta gagtttttgg ggggtgggtc ggaagatata gtttttttcgg 4440
gtgttttttt ttttgtagac ggacgttttt atcgttatgg agtttatggg tatttgcgtt 4500
gagaagttta agaagcggat gtagggtttt attttcgagc gtattttggg taagatgata 4560
gtggcggtga gtgattaggc ggggtttgta ttgggtagga tgatagaggc ggtgagtgat 4620
taggcggggc gtgtattggg taagatgata gtggcggtga gtggttaggt ggggcgtgta 4680
tcgggtaggt ggttttgggt ttgtgttttt tgttatatgt attttttttt gcgtgtttgt 4740
agattgtgaa ggcgttgtat tatttgaagg agaagtacgg tgttatttat cgcgacgtta 4800
agttttttaa tattttgttg gacgagcggg gttagattaa gttttgcgat ttcggtatta 4860
gcggtcgttt ggtggatttt aaagttaaga cgcggagcgt cggttgtgtc gtttatatgg 4920
tagtgagtgg gggtttttta gcgggggagg gggtgggggt tgggaggtcg gttttagttt 4980
tggagatacg tttttttttt ttttttttgt agttcgagcg tattgatttt ttagatttta 5040
ttaagtcgga ttatgatatt cgggtcgacg tatggagttt gggtatttcg ttggtgagtt 5100
ggggtttttt tttgtttttt agttaggagt gagggttttt gggggattcg gagggaggag 5160
aatataaatt tgtttagttt tgttcgtttt ttttttaggt ggagttggta ataggatagt 5220
tttttttataa gaattgtaag acggatttg aggtttttat taaagtttta taggaagagt 5280
tttcgttttt gttcggatat atgggttttt cgggggattt ttagtttttc gttaaagatt 5340
ggtgagaatt tttttttatt tgggaggtta gggatagttc gttgttttgg gtagttgggg 5400
aggtaatggt aggaggggaa tggaggtcgt attttgggat gggcggatgc gattgtgggc 5460
gggtttttgg ttggcggttg ttataggagt tggagttggt gtttagggag ttatgagttg 5520
ggtttggatt tagtttgagg ggatagttag ttttgtggta tttggcgggg tggttgttgg 5580
agattgtgag tacgtttttg taataagtat agttataagt ttaggagggt tatgttcgat 5640
tttttttaggg gagttttatt tatttttttg ggggatttta gttagttttg gtttttttaag 5700
ttaggttttt gttatttggt tttaggttag gaggtttcgg gatttatagt tgtttttcgt 5760
tgttagtcgg ggttttgagg atatttatag tttttttttt tatatatatt ttggggattt 5820
ttggtttttt ggggagggtt tgagtatagg ggtggggtcg gtattttttt ttttttcgtt 5880
tttatatttg tttttttttt ttttgtttta gttttattaa agattatagg aagagattaa 5940
agtataataa gttatttgtg agtatttgag ttttttttagt tttcgttttg tttttgcgga 6000
ggcgcgaggt taggagggaa gatcgttttt ttttaagttt tattttttcg ggtttatagg 6060
aatatagttt tattaagcgt tacgagacgt tggaggtgga cgtggcgttt tggtttaagg 6120
atgttatggc gaagattgag ttatcgcgga ttagcggcgt tttgagttag ttttatttgt 6180
ttttttttag gtagttgttt ggcggcggtt agttttatag gggttaggg gtatggttat 6240
aggtttttttt ttttatttgg ttatttagtt gtttgttagg ggagatttgg gatttggacg 6300
gttatttagg attgaggata gagagtgggg ggtgtttatt tattttttttc gtttcgggtt 6360
tattaagttt tcgttttttt tatttcgggg ttagtcggtc gtgtgcgttt ttcgatagat 6420
attgtgaacg gaagatagta ggtcgcgatt agagtcgttg tttatttagt cgtagttttt 6480
gggtcggggc ggtttttagg ggttaggaga gagttttgga gtttcgtagt tattatgtac 6540
gtttttagcg tgttgtgttt ttcgttattt ttacgcgttc gttttttttt cgtcgttttt 6600
tgttttttgt tttatttttt tgtttttgtt tggtttttttc gttattttttt ttgtttttgt 6660
tttttttttg gtttgagttt gggtttagtt atttttttgac gggttttttg ggtttgtata 6720
ggttttttat ggcgtaatga gttagtggtt tttagttagg tagtgtgggt attgttattg 6780
cggttggacg gggttgcgcg ttcgcgtttt tttttttttt tttttttttt ttttgatttt 6840
aggggggtttt ttttggagtt tattgtattt tattgtattt ggtggggtgt ttggggtggg 6900
ggcggaggag agtttgtttt cgtggggttg tcggtatttt tagaaatttt tattaaagtt 6960
acgattagtt gtttgtggtg gggttttaat cgttttcggg tattggggag ttgggttggg 7020
gttgttgttt tggggttttc gggggttata gtttggggtg agttgaagat tttaggggat 7080
gtggaggggt ttgcggggtt ttggtcgtat aggatggttt ttagggaagg tggttttggg 7140
gtatggtgta gagtaggtga tcggagggaa tcggtgacgg agcggggtta agggaggggt 7200
tcggagggag ttagggatgg agggtagagg gagtggatgt gggggtttga ggacgtgtga 7260
taagttttag taggggtggg ggtcgggttg agggtggggg tgcgaggtgg ttatttttat 7320
cgtgtttttg gtcgtttttt tatttattta tatttggttt agtttacgtt gaggtttagg 7380
attgggaagg atcgggtgag tgtatcgggg atttaggtta ggtgttttttc ggagtttgtt 7440
ggggtggtta gagtaggagg gggtgtgttt ttttttttgtg ggtgttgtat gtaaattaag 7500
tggataagaa aaaataataa aataaaaaat aagaaaaaaa aaatataaaa tttcgtaaaa 7560
ttataaagaa aatttaatat taaaggcgta gaagtcggtt ggtcgtggtg ggggtagcgt 7620
aggcgtagta tttttttttt tttatttagt ttgttgattt ttgttattat tatgatattt 7680

ataaaacgtc gtatgtttaa aaagttatag atgttatttt ttttttgttt ttagggagga 7740
aagttatttt tttttgtttt ttggtttttt tgttaggggt taggggtttg tcgggtgggg 7800
gtgttaatag gtttggtttt tttttttttt gtatttagtt atgggggttt ttgcgattgt 7860
cggaaggttg tatggttggt tttagggtta gtataggttc gaggtcgggt tgtttggttt 7920
tatttttatt taattttatt ttttgtttta tgagtgtgtg ttcgtttatt tttattttt 7980
ttagtgttaa gtggggagtt ttgggggagt tttttttgtt ttttagtttt ttttaagatt 8040
tttttttttcg ttattagtta tttttttgga ttaggtagag ggcggatcgg gtgggtaggg 8100
gtttgagggt ggttcgggtt agtttattag ttaatggatt ttttttagg tcgttagtgt 8160
cgttttgttt ttttttaaaa taaaatgttt tcgtttgtaa atttttagac gtttgagaat 8220
aaattttttt tttttttttt atcgagtttg tggtgtgtta tgggtttggt agggcgaggt 8280
gggaaggggg tggttgcggg gggaggtttt tgtattgagg ggagattgag gtaggtttgg 8340
gagttagacg ggaggtgttt gtagtgattt gtcgatgggt attaagtagt tggtgtaagg 8400
gattttttgt ttttagtggt tataggtcgg gtagtagagg atttttttaat ttttttaggt 8460
tttgggtatt ggttttggtg ttagtttttt agtttgttat tttcgttttt tgtttttttt 8520
tttgttaagg atagggtcgt ttgtttacgt gcgggatttg ggtgtagttt agttggtgtt 8580
tgttttggtt ttaaatattt cgattttagt tggtttagta tgttgggtat cgtgttgttg 8640
ttggttttgt ttttagggat tattatttta tttagcgggt tatttggtat gtatgggtag 8700
ggaggtgacg ggtttgttat gtttttattt ttgttttatt tttttatttt tttttgagat 8760
ggagttttgt tttgttacgt aggttggagt gtagtggcgt aatttttatt tatcgtaatt 8820
ttcgtttttt cggtttaagt gatttttttg ttttagtttt ttgagtagtt gggattatag 8880
gtacgtatta ttacgtttgg ttaattgttg tatttttagt agagatagcg ttttttttatg 8940
ttggttaggt tggtttcgaa tttttgattt taggcgattc gttcgtttcg aatttttttaa 9000
agtgttggga ttataggtgt gagttatcgc gttttatcga tgttattttt ttttaattag 9060
atttttggtg gtggttttta tgggggtggg gatagggtag gggagtgggg tggggttttt 9120
gtttttttatt ttaagggtgg tttatatttt tatttcggta aagtttttttg tagttacgga 9180
gagggttaga gttgtgggag ggggttagtt tatttaggtg ttttttagaa gtgggtgtgg 9240
tgtgcggatg gataaggggg tttatgtttg taattttagt atttaagaga ggttgaggtg 9300
agaggatcgt ttgaagttag gagttttaga ttagtttggg taatatagat ttcgtatggt 9360
cgtggtagcg agtggttgtg gttatagtta ttcgagaggt tgaagtagga ggatggttgg 9420
agtttaggag gtggaggttg tagtgagttg tattcgtatt attgtatttt agtttgggcg 9480
atagagggag attttgtttt agaaaataag aaaaatggat gtgtaggtaa tttttcgtat 9540
ttggagatta gtcgttttat taggatttcg tttagtattt gaaggtagat attagcgtgt 9600
gtttaggggt acgaagaggg gagggggtttt atggttgttt tagtttggat aagtggaagc 9660
gttttagatt ttggtggttt ggttgggttt tggttgtgtt taggagagtt ttttgggagg 9720
gtattttttt tattttttttt tttcgttggt gatagtattt atttttttttt agaaagggga 9780
gtcgtttgtt ttgttgcgtt tttaagagta gttttgggga aggtgggggga gttttgattt 9840
tatttagtcg gattttatta tcgggtgttt tttatcgttt tttttttcgtt ttagggaggg 9900
tttcgttagt ttcggaggtt tttttagggg cgtatggttt tgtaatcggt ttagttgttg 9960
ggttgtgcga gttttagggg tcgttagggg gtatattatc gttagggggg aaagtggcgc 10020
ggagtttatt atgggtgaat cggtcgtcgt aatcgtattt tttttttaaa ggcggcggcg 10080
ggggcgaggt ggtcgggtta tttttttcgg aggtttaaag ggtagcgcgc gtttttttttt 10140
ttttgggtcg ttttttttcg tttcgttagt tttttcgttt ttcgcggcgt tcggttttttg 10200
gttgcgtaga ttttttttta gtttgaagtt gttcgatata gaggacgttt tttttcgtcg 10260
cgcggggtcg ttcgaggttt cggtcgatag tcgcgtgttc gtgtaggtgg ggatttcggg 10320
gatattaggg gcggatgggg gtcggtgggg acgggcgatt tttgatggcg ttttcgtttt 10380
ttaggcggtt ttggttcgtt tttttttcgcg ttggggtttg gttttgtatc gttgtttagt 10440
gacgtcgttt ttacgttcgg tttcggggtt cgttttggtt ttgttgcgtg agggttgttt 10500
cgtcgatatt tttgtcgttt tttcgttatc gtcgtcgtcg agttcgggtg tcgttcgttt 10560
cgcgcgtttt agtttttcgtt tgcgttcggt ttttaacgtt tcggtgtagt ttttgtattg 10620
ttagttgagt cgttgtcgtc gttttcgggg agttcgtcgg gcgtttgcgt ttttgacgtc 10680
gtcgtcgtcg tcgtcgttat cgcgggtgcg cgggcgtaga gtttggaatt cgggcgttgg 10740
ggtttttcgg gggttgggta cgggcgattt tggtagtgga aagaggatat tttcggggtt 10800
tgaggatttg ggggttgggg gaggtggggc gtaagggttt atttttgtcg atagagtttt 10860
ttcggggata gtgtggatat tgggtttttt tatggatagt tttggaattt tttagggttg 10920
ggggttggat gtagaaggtt attaagggga tatatttttg ggaatttggt gttgggtttt 10980
atatgttagg tgttgggaga cgcggattat gcgattttgt ggggttggga tttggggttt 11040
ttaggggttg ggcggttagt tatttgtatg cgtaggggtt gggtttaggg tattttaagg 11100
ggtcggattt ttaggggttt tattttggtt gtggtggaga gttggattta ggtttttttt 11160
gggggttttg gggattagag ttacgatttc ggtgttttga tgttttcgtt tttttttttat 11220
tttttttttt tttagtgttt gttttaggac gaggcgtgcg tcgatattgg tagtggtagc 11280
gtcgagggtt tggttgttga cggtttttat ttgtatacgt tgacgtagtt tatcgtggtt 11340

atcgtgtcgc ggtcgttttt tagtgagtac gtagtttttt ttcgtatggg gtcgtggggc 11400
ggtagagcgg gtgggaagga cgtttgggag ttggatttag ttttagcgtg gtattttta 11460
tagggtcgtt taagagtgtt ttcggtcgtg tagtgcgttt tgagttttc gcgtcggttt 11520
tcgcggtttt ggaattcgcg tcggtggtgg cgttggtgtt ggtagttttc gtgttgggcg 11580
tcgcgttggt cgtcgggttg ggtttcgttt gtgcgtattt aggtatcgac gattttcgtt 11640
aagtcgggtt tttagtttaa tttcgcgcgt cgggattcgg ggcggtcgcg atttcgtttg 11700
cggggtttga ttaattttag ttaggtttgt tttcgcgatg ttcgtagtag tttttagggg 11760
tcgtttttag tttggcgtgg cgcgtagtag ttttttgta gttcgttttt ttttttagc 11820
gttttacgtt tttggttcgt tcgcgagagt ttcgtttagc ggtttttagt ttaggaggtt 11880
ttagtgagga aggtaggtat ggaggtggag ggagttgggt gaggtaggag atttgatagt 11940
gacgtttttt agcgtttagt tcgtgttagg tattgtggtt tttttgtttg tttgtttgtt 12000
tgtttgtttg ttttttgaga cggagtttcg tttttgttgt ttaggcggga gtgtaatggc 12060
gcgatttcgg tttatcgtaa tttttgtttt tcgggtttaa gcgatttttt tattttagtt 12120
ttttgagtag ttgggattat aggtatgcgt tattatattc ggttaatttt gtatttttag 12180
tagagacggg gtttttttat gttggttagg ttggtttcgt atattcgatt ttaggtgatt 12240
ttatcgtttc ggttttttaa agtgttgaga ttataggcgt gagttattgc gtttagtttt 12300
taggtattgt tgtaaatgtt ttacgtttat gaattaattt aattttttta atttttaggc 12360
ggatattatt ataattttta ttttagagac gagaatgatg aggtattttt tgaattaagt 12420
aatttgttaa aagttataat attgggtgga gtttggattc gaatttaggt agtgggttat 12480
tcgcggtagg cgcgggtttt gggtcgagtt aggggtatta ggtgaggaga ggtattaggc 12540
gaggagaatt tagtattcgg attcggtttt tatttttatt tcggtatgta gatcgatgga 12600
agaggttagt ggtttgttgg gaagggaggt ggttcgcgta gcgttttttt tttcgttgag 12660
ttttagttat ttttttttagg gatggtgcgt ttttaatatg gttcggagat atatttagtt 12720
attaattcgg gattaggatt aataggatcg gattcgtttt tttggatttc ggatttgatg 12780
aggttacgat ttttgcgttt tttttttttt tttgtttttt ttatttgtgt ttaaaataaa 12840
tttttggatt gatcggttaa gttttggtgt agttagtgag cgcgtaacgt gcggggtggg 12900
ggataggtgg ggaggtttgg ggagaaagtt gggagtagga tttgggggt atttttgaga 12960
aatcgtgatt tatagggggtg gagtttgggg acggggttta tatgtttata tattcgtgtg 13020
tatttttagg ttatgggaga ttgtagggcg ttttgtttta aatagaaagc gatattcggt 13080
cgggcgcggt ggttttcgtt tgtaatttta gtatattggg aggtcgaggt gggtggatta 13140
tttgaggtta ggagttttag attagcgtgg tcgatatggt gaaatttcgt ttttattaaa 13200
aaaagtaata aaaaaagaat tagtagggcg tagtggcgcg tgtttataat tttagttgtt 13260
ggggaggttg aggtaggaga atcgtttgaa tttaggaggt agaggttgta gtgagtcgag 13320
atcgcgttat tgtattgtag tttgggtaat aagagtaaaa atttgtttaa aaaaaaaaaa 13380
aaagcggtat ttaattttat aagcgtaagt ttaaaataaa tttttttttt tttttttttt 13440
tttattcgga tatttgttta tttgtttttt gcgatttttt ttaagttagg ttttttttcgt 13500
tttttattat tttcgttcgg attttgtttt agtttcgttt cgattttttg tttttgtttt 13560
agtttttttt tggtttggat ttcgtttttt ttttagattg ttagtttagt ttagagtttt 13620
ttaaattttt tttgttcgag ttttatttta ttttatatag ataagagtgg gattattagt 13680
tttagtttat agaggaggaa attgaggttc ggtaaggtta aattatttgt ttaaagtttt 13740

taggtttgtt tgattttaga gtttaggttt ttgatcgatt ggattttcgg atttgatttt 13800
tcggttagaa tttatttttt tgcgagaagt aaagtatata gtagtagttt tatgagttag 13860
tttagtaata gagggagggt atgggattta aggtgtcgtt tttattttat gaatgagata 13920
aagtgtaggt tgagggtttt tttcgtttcg ttttagtttc ggtgtcgatt ttttttttat 13980
ttttattggg gcggttttttc gggtttttaa acgcgaagtt acgtttttac gcgcgttcgt 14040
tttcgcgcgg ttttttttga tttcggcggt tttcgtagtt tcgtttatcg gagaagcgat 14100
tttatagcgt ttgttttttt ttgagcggta tgaagttatt ttttaggcgg cgagcggttt 14160
cggcgcgtta tttgggcgag gtgatcggtt tcgcgatttg gagcgttcgc gagaagcggt 14220
agttagtgcg atttttgtag gcgcggtagg gttagtcgga gtcggacgtt atcgagttgg 14280
ttcgggagtt gcggggtcgg agcgaggttg aggtgagatg cggttttcgg gatcggagtt 14340
aggttggagg cggggttggg gtggggcgga gagtgggcgg ggttgcggta ggagttagcg 14400
gggcggggtt aggaatgtaa gcgagcgggg gcgtggcgtg gggttgagtg gcggggcgtt 14460
agtgggtgag tttgggcggg gtgaggggcg gggcgtggac gggaagagaa gatttagggg 14520
cggggcgagt ttgggagtta gacgtgggcg gggtataagt gaagcggagg ggcggggcga 14580
tgtcgggggc ggggcgtgcg tgtcgttgag gtttggagag tgggtgggtg agtttgaggg 14640
gtaggacgag tttgggggcg gggcgtgggc gggttaatag taagatttaa gggcggggtc 14700
gttttgggag taaggcgtgg gaggggcgtg ggcgggatga gggtaagttt taaaggcggg 14760
gcgagtttcg gggtaatgcg tgggtgaggc gagggttaaa cgggcggaat gaattgggag 14820
taagatgttg tcgagttcgg gcgaaaagta acgtatgttg ttggatagga tacgagtttg 14880
aagtgagagg gaatagagaa gaggggacga agaagggagt tgaacggggt agtagttagt 14940

```
atagggttgg gttttggttg cgtgaaggag tcggaattgg tttgggtttt ataatttttt   15000
taggtagttg gttacgtttt gggtggggta ggggttttgg tagttattgg gatttttaat   15060
atcgggttag taggagatta aggcgtagta gcggggttta gagtatataa gggattgggt   15120
tttggttttt ttgttgtagt tttgagttta tagaagtttt atttcgtcgt tttttttttt   15180
tttagattcg ggtttttttt tagtagttta agggtcgcgt agttcgggag gttatttaga   15240
aagtgtattc gggtggtttt tagggattaa ggcgtcggga ggtatagttt ttagttttta   15300
tagaggtgag gtagatgaat agggtgaggt tgtttagggt aggtttttgg tgggtaggtg   15360
ggagttgcgg gggataattt gattagggga tttttaggag gagtagggtt tgggggggtta   15420
ttgtaggttg atttttgggt tgattttgta tttttggttt tatttaggtt tggacggatt   15480
tggttgagaa gataataggg ttattggaag aagttttggt agtggttttt tcgtaggtat   15540
cgttattttt ttaggtaggt tagggtgttt tagaggatag ggttatatgg gttaaattat   15600
gtgaatttgg gttttgggtt tttttgttag ttttgtggat ttcgttagtt attggatatt   15660
tcgagtttta gtgtttttgg ttttgaggtt attttttgtt tgaggagtat tttagggtgg   15720
tagggaggat gggggaatgt gtagacggtg agaagatttt ttagtttttt tttttttttg   15780
attgttttgt ttgttaggtg tttattatcg cggttacgga atcggttatt tttttgtatt   15840
ttaagttttt taagtttacg taggttcgtg gaaagttttt gtttttgagc gtttttggag   15900
gataggaaga tttcgttttt gaaatattta gttttgtttt tgttgtattt agtttcgtat   15960
ttaggatttt tgattttgtt tttgagaaat tttttgagtc gtcggttggt ttttttattg   16020
aagaagattt tgttgtggat tttgagaaga tttataagta tttgtttttt gtttttcgaa   16080
gtggtcgtag tttcgagttt ttagtagttg gtgagaaggg tgagggaggg ggtaggagta   16140
gagggattaa gggtttttgg tagtaggtag ggtgtttttt ttattttttt tttttattta   16200
ttattagagt tcgttgtggt tttcgatttg tttatgttat ttttagagga gttgttattt   16260
ttgttttgta tagttttggt tgagtatatg acggagacgt atttacgttt gatagttttt   16320
tagtttattt tcgttggagg gagtttgggg tttgtagtag aaggggatgg ggttggtttt   16380
aaggtattag aggagatttt tttagttatc gagaaggtcg agtatagcga attgaaatcg   16440
ttttggtaag tagttgggat ttgtttttttg aattcgtttt tggtgttttt ggagtttttg   16500
ggtcgggtag ttatttttgt taggtgaggg gtatggcggg taggaggtta tattaggttt   16560
ttcgttttgt ttttcggttt tgttcggttg gttttggttt tttttgagga tttcgttatg   16620
ggggaaggtt tttgagatga tgtttagttg tggggcgggt ttttaagatg ttttatattt   16680
tgggggtttt agaaatggaa ttttcgttgt ataggggttg ggtgggggtt gtaggatttt   16740
atttataagt tttttgatgt taaggatagg cggatagggt tggttttttt tagtttttaa   16800
gttttattgt gttttgttgt ttgttggggg gttatagttg gtattgttta tcgtaaaggt   16860
tttcgtatat tttttttttt tttgtatatt tcggggttag tatttttatt ttttttaatt   16920
gattagtcgt ggttattttt tgttgttagg tttttttttt tttcgggggt attttgtgat   16980
tatgaataaa gttattattt tttttttttt ttatttgtga tttaaagatg gaagtggggg   17040
ttttgaagtg ttagagtttg ggggttatag ggttgggggt gtgtgttagg gaggagagtt   17100
gttatttgat tggtgtgatg aggtttgggg tgaggagttt tttggtgatt gtgaagttga   17160
taagtgtgga tggggagagt cggtagggta ttttggggat aggcgttagg gatgggtttt   17220
agagtagggt aaaattatat cgtataggtg gaggggagaa ggtagtagag agtgggggtg   17280
attcggtgga aggagttgag ttttaggttg ggcggttaga ggaagataga taggggtggg   17340
ttgtttcgag tggtaggagg ttagtgggag tggagtgttt tttggggtgg ttttgggatt   17400
ttaaggattt ttagaaggcg gggagggagg ggtattggag ttagggttga ttttaaattt   17460
ttgggggtgt tgggatttcg tttgaggttt tttttattag ttttatttgt tttgtgggtg   17520
tggatcgagg gtcgggtttt agagagggaa gtagatgttt ttttacggta tcgcgtttta   17580
cgtttagtta gtaatttaag tcggtaaatt tttcgttttc ggagtttgtg tatttattta   17640
gtttgttttt tatagaaacg tttatttttt tttttatata ttgtatacgg tttttttggg   17700
gtgtatattt tttgggaggg ttttggagta tagttagttt atttcgttcg tggttatcgt   17760
tttatcgtat tagatttata aacgttgtta atggtggtgg gtagggggag gaggtttagg   17820
ttgtaggtac ggtcggtcgt cggagttttt ttttagggtt ggtagagggt gcgtttttta   17880
gtttttttata gggttgagtt tttttattta ttttcggcgt ttttttagat taatagaatt   17940
gtgtgtggag gggaggtagt tggggggttat atattagttt tttttttttaa agtcggtgaa   18000
aggtgtttgt gattttggtg tttttgtagt tttgggaagt tttaaaaata tgtaaaagtt   18060
agatttgggt tttattttta gttttattag attttagtta tgtgattgtg ggtaagttag   18120
ttattggagt tgtttggttt tcggtttttt ggtttgtaga atagggatgt taatagggtt   18180
tggtttattt ttacgatttc gaagaggaga taggttataa ataggaggtg ttaatttagt   18240
acgttatttg gtatgtagta agcgtttaat aatagttttt attattatgt tcggtttaga   18300
ggtagggagg agtggaacgg tatgggaggg ttgcgggagg tacggtaggg gggttagggg   18360
taagtggtag gagggcggat ggggggtagc ggtgggtatc ggggtagggc gcgttgattt   18420
gttttggggt tcgggttggg ggtagaaatg agtttgttta cgttgtttcg ttacggtagg   18480
cgttacgtat tttcgatata gtcgttatgg taggttttcg ggtttcgttt tcgggatagg   18540
cggtgtaggg taaattggta tgtagcgttc gtttcgtggg ttcgggagag tttgtttcgt   18600
```

agggattaga gtttaaggac gggtttaata tttagttaag gtggggttga cgacggttag 18660
ataatagggg agggaggagg gataaggggg tttttatttt tagggacgta taatagtaga 18720
gttatttata cgttggggag ggggcggtgc gggggttttt ttttcgttcg ggatttagag 18780
tcgggggtgg gggttagtcg ggcgggtgag atgcgtagag aggaagggat agggcggata 18840
aaggtacgag gtggggtttc ggttaggtta ggaagggata tgggaggggt ttcgaggggg 18900
aggggttggg ttttttttaa tttttttttt tttttttcg ggttgggggt ttcggggcgg 18960
gattttgagc gtagttttgg tttcgcggcg tttttcgtcg ggtcggtttt ttgagatttc 19020
ggcgtagggt cggttagggg gcgtcgcgtt ttttatatta acgcgtagtc gaattgttcg 19080
cgttcgagcg tttttttgtg gtcggtttag gacgaggcgg gtatgcggtt gtaggggtcg 19140
agtaggatgc gtacgtagcg tattattaat agtattagta ttacgagtag gtagagtacg 19200
aaggcgaata tcgtgcgttg tttcgcgttt aggttcgcgt ttatcggggg tttcgtcgac 19260
ggcggtaggg gtttcggcga tagcgtttac gtcgcgttta tggtttatat cgtcgcgcgt 19320
tttgcggagg aggggattcg tttcgggcgt cggggatgag gttgcgtttt ttttttcgcg 19380
ttttttcgt ttcgtttttt tttgtcgtcg tcgtcgtcgt ttttttaac gtgttcgatt 19440
ttattttcg ttcgcgtttc ggttttcggg ttacgtttat gttcggttcg tcggatcgtc 19500
gttattatta cgcggggatt taattttggt cgtgcgcgta ggtgggggcg ttgagagtcg 19560
ggaaacggag tcgcgttcgg tttgggtcgt atataatagg tgcgaaagcg cggtcgcggt 19620
tcgggcgcgc ggcgtgggga cggttttcgc ggtttttgtt ttcgtttttt ttgttttcgg 19680
cggtttggga tttttttttt tatcgttttc gtttcggcgc gtttttttatt ttggtttcga 19740
ttcggacggg gatcgatcgt tcggcggtcg cgtttttta gggattcgcg tcggttgcgc 19800
gttcggtttt ttttcggcgg cggcggcggc ggttcgggtt tcggtttagt ttattttatt 19860
cgtttttgga gcggcggaga tcgaggtagg taagtagcgc gcgagtcggg tcgtcgcggt 19920
tgtttttatg gcgacggggg cggggtcgtc gcggggggcg gggttgtagg gggcggaggt 19980
tgcgtaggga gcggcgcgcg agttagcgat ttcgttcggg tttgcgggag gacggaattt 20040
tagtttcgag gattcgcgag tatttacgat ttcgtcgggg agtgggtagt tgttattttt 20100
atttttttg gtatttcggg ttttttagtt tgttttgtgt ttgtattttt tttcgcgata 20160
gattttattc gagatttttt gtttgtgggg ggattttagt ggttttaaaa tatttatatg 20220
ggtatttaag tttttcggt tttggggatt ttaattttta gagagtttag gtaggttatt 20280
gggatgagta agtttttta gttttgtttg ttattggttt cgttttttaa tttagggttt 20340
agttttgggt aaggcgttta tttattgcgg ttgtagtttt ttattcgatt ttgatcgttt 20400
tttatagtag tcgtttgttt ttatttttat tatcgtttcg gttattttcg cgttagagaa 20460
gatattttag atttcgtagt tgttttttg taattcggtg ttttattttt tttaaatttt 20520
aggtttatgt ataagatgga agagtattgt taaaattaaa aaatggattt aaagtggttt 20580
ggttgacgtg gttagcgggt tattgagtcg cgggtttcgg gttttatttt gttgtggggg 20640
gagtttttgg gttttggggt tttttggtat tgtgtgattt gtgtgtattt taggtgatta 20700
ggcgtcggga tttttgtagg gtagagtaat agggtagggg ttggttttgc gggggagtgt 20760
ttttagttgt cgcgtattcg taatagttcg ttgttttttt tcgggttagt tggttttgta 20820
gtcgttttgg tagagttggg ggtagagttc gtagttttgt ttttagaggt ttggagttgt 20880
cgtaggtggt gttgcggtgt ttttgtgttt gatgatttag gtcggggtta tttggtttcg 20940
gtattatatt tagagaattt gttcggtgtt ggaggtcgag atgtttagga gtcgttaggt 21000
cgcgtagggg ttgagttcgt tttggtttcg gtagagcgtt tatacgtata gtattcgtag 21060
tattttgttt tgtagggtgg ggtgggaagg gtattgttga gagggttgag gtgatttagg 21120
tagttttggg ggggggttag gttttgtggg gtaggagtta tcggtaattt ttttaggggt 21180
tgggagtaga ggttagtata attgtttaga tttaggaatt atttagtttt ggtaaaaata 21240
gataggtttg ttggttttcg attttttta atttaaatt ttggtttaga ggttttttta 21300
tatttttatt tagttttaga gttttagggt tttgagttgg ggtttttgtt aggtggttat 21360
atatagattt tggggatagg cggtgttatg tgtttgtttt attttatttt tttatttatc 21420
gggttatttt ttattatttc gtttttgggg tttttgatta ttattattag ttgtgtttgg 21480
aaggtgatga ttagtatcgg tttttgtttt attattttgt ttatggttag ttgtagaggg 21540
gtcgagaggg gggatgtgtt ttagaggata gttcggttgt ttttttggag gtgaaatttt 21600
tgcgtttagg ttttgttagg agaaggtttt agtattaggt ttagtatcgg ttttttttcg 21660
tgggttttgt tttttaggtt aggattaggt ttttttcgag atttttttt tggataagtt 21720
taagattttt tggattagtt tatggttttt agtttttggt aaggttattt tttgggtttt 21780
gtttgggtta tagtttttat tttcgatggt ggttcgggtt tttattgcga ggtgtttacg 21840
tcgacgttgt taatgtttag gatgcgagaa tggaattgga gatttagtgt tttggtttgt 21900
tggatggggt gggttagttg gttgtaagtt tgtaatgaga ggtcgatacg tttgcgtgat 21960
gttatttagg gtgggtatta cgttatatag agttgttgtt tatatatttt ggcgtttagg 22020
tgttttgtcg ttagttagta atggtagagg ttaagaattt aggtttaagt ggggaggtag 22080
gtgttattta tttattggtt tagattatac gttttttatt tttgggaata atattttatt 22140
tagatgattt aatgaaaata ggattaatta gtaaagtggg tgattttttt ggaataaaat 22200
tttaaattta tttttagggt taagttggcg gtttttttggt tgatttttaa tatttttata 22260

ttcgtcgtat tttgaatttt ggtttgagat taaacgtttt ttttaggatt tgtttttagt 22320
ttgaggttgt ttttgttatt gattagtttt tagtg 22355

<210> 176
<211> 22355
<212> DNA
<213> Artificial Sequence

<220>
<223> chemically treated genomic DNA (Homo sapiens)

<400> 176

tattgggggt tggttagtga tagaggtagt tttaggttga ggatagattt tagaagaggc 60
gtttgatttt aggttaagat ttagagtgcg gcgaatatgg aagtattaaa gattagttaa 120
ggggtcgtta gtttagtttt gggagtagat ttgggatttt gttttaagag aattatttat 180
tttgttgatt aattttgttt ttattgggtt atttggatag agtgttgttt ttaggagtgg 240
gaagcgtgtg atttgggtta gtaagtgggt ggtatttgtt tttttatttg gatttgggtt 300
tttggttttt gttattgttg gttggcggta gggtatttgg gcgttagggt gtgtgggtag 360
tagttttgtg tggcgtggtg tttattttgg gtggtattac gtaggcgtgt cggtttttta 420
ttgtagattt atagttagtt ggtttatttt atttagtagg ttaaggtatt gggtttttag 480
ttttattttc gtattttaga tattgataac gtcgacgtaa gtatttcgta gtgggggttc 540
gggttattat cgggggtggg aattatggtt tagataaggt ttaggaggtg gttttgttag 600
gggttggggg ttatgggttg atttaagagg ttttgggttt gtttaagggg agggtttcga 660
gggaggtttg gttttggttt gagaggtaag gtttacgggg agggatcggt gttgggtttg 720
gtgttggagt tttttttag tagggtttag gcgtaggggt tttattttta ggggagtagt 780
cgggttgttt tttgaggtat atttttttt tcggtttttt tgtagttggt tatgggtaag 840
atgatggagt aggggtcggt gttgattatt attttttagg tatagttggt gatggtggtt 900
aggaatttta aaggcgaggt ggtggagggt gattcggtga gtgagggagt gggatggggt 960
aggtatatga tatcgtttgt ttttaagatt tgtgtgtggt tatttggtag agattttagt 1020
ttagagtttt gaagttttgg gattgggtgg ggatgtggag aaatttttgg gttagagttt 1080
ggggttgagg gaggtcggag attagtagat ttgtttgttt ttattaaggt tgagtggttt 1140
ttgagtttag gtaattgtgt tggtttttgt ttttagtttt tggggaagtt gtcggtggtt 1200
tttgttttat agagtttggt ttttttttag agttgtttgg gttattttaa tttttttaat 1260
agtgtttttt ttattttatt ttgtaggata aggtgttgcg gatgttgtac gtgtgggcgt 1320
tttgtcgaga ttaggacgag tttaattttt acgcggtttg gcggtttttg gatatttcgg 1380
tttttagtat cgagtagatt ttttgagtgt ggtgtcggag ttaggtagtt tcggtttggg 1440
ttattaggta tagaggtatc gtaatattat ttgcggtaat tttagatttt tgggaataag 1500
attgcgggtt ttgtttttag ttttgttagg acggttgtaa gattagttgg ttcgggaggg 1560
gataacgggt tgttgcgggt gcgcggtagt tggagatatt ttttcgtagg gttaattttt 1620
gttttgttgt tttgttttgt aggggtttcg gcgtttggtt atttggggtg tatataggtt 1680
atatagtgtt aagaggtttt agggtttagg gatttttttt atagtagggt gggattcggg 1740
attcgcggtt tagtggttcg ttagttacgt tagttaagtt attttaggtt tattttttaa 1800
ttttaatagt gttttttat tttgtgtata agtttgagat ttggaaagaa taaaatatcg 1860
aattgtagaa gagtagttac ggagtttgaa gtgttttttt tggcgcggga gtggtcgagg 1920
cgatggtggg ggtgggagta ggcggttgtt gtgggaggcg gttaaggtcg agtgaggaat 1980
tgtagtcgta gtgggtggac gttttgttta gggttggatt ttgagttgga agacgaggtt 2040
agtgatagat aaaattaggg ggatttattt attttagtga tttgtttgag ttttttgaga 2100
attgaggttt ttagggtcgg gggagtttga gtgtttatat gagtgtttta gggttattga 2160
ggttttttta taaataggga gtttcgagtg gggtttatcg cggaggagaa tgtaggtata 2220
gggtaggttg gggggttcga agtgttaggg gaggtgggag tggtagttgt ttatttttcg 2280
gcgaggtcgt gggtgttcgc gggttttcgg ggttggggtt tcgtttttc gtaggttcgg 2340
gcggggtcgt tggttcgcgc gtcgtttttt gcgtagtttt cgttttttgt agtttcgttt 2400
ttcgcggcgg tttcgttttc gtcgttatag gaatagtcgc ggcggttcgg ttcgcgcgtt 2460
gtttgtttgt ttcggttttc gtcgttttag ggcgggtgg ggtgggttga gtcggagttc 2520
gggtcgtcgt cgtcgtcgtc ggggagaggt cgaacgcgta gtcggcgcgg gtttttgaga 2580
gagcgcggtc gtcgggcggt cggttttcgt tcgggtcgaa gttagggtga ggggcgcgtc 2640
ggggcggggg cggtggggga gggggtttta ggtcgtcgag aataaaggga gcgggggtag 2700
gggtcgcggg gatcgttttt acgtcgcgcg ttcgggtcgc ggtcgcgttt tcgtatttgt 2760
tgtgtgcggt ttaggtcggg cgcggtttcg tttttcggtt tttagcgttt ttatttgcgc 2820
gtacggttag agttgggttt tcgcgtggtg gtggcggcga ttcggcgggt cgggtatggg 2880

```
cgtagttcgg ggatcgaggc gcgggcgggg gatggggtcg ggtacgttaa gggaggcggc    2940
ggcggcggcg gtaggagggg gcgaagcgga ggaggcgcgg gagggagggc gtagttttat    3000
tttcggcgtt cggggcgggt tttttttttc gtagggcgcg cggcgatgtg agttatgagc    3060
gcgacgtgga cgttgtcgtc ggagtttttg tcgtcgtcga cggggttttc ggtgggcgcg    3120
ggtttggacg cggagtagcg tacggtgttc gttttcgtgt tttgtttgtt cgtggtgttg    3180
gtgttgttga tggtgcgttg cgtgcgtatt ttgttcgatt tttatagtcg tatgttcgtt    3240
tcgttttgga tcgattataa ggaggcgttc gagcgcgggt agttcgatta cgcgttggtg    3300
tgaggggcgc ggcgtttttt agtcggtttt gcgtcggggt tttagagggt cggttcggcg    3360
gggggcgtcg cggggttagg attgcgttta ggatttcgtt tcggagtttt tagttcgggg    3420
gagaggggga gggagttggg agaagtttag tttttttttt tcgagatttt ttttatgttt    3480
ttttttggtt tggtcggagt tttatttcgt gtttttattc gttttgtttt tttttttttg    3540
cgtattttat tcgttcggtt ggtttttatt ttcgattttg aatttcgggc gggaggagga    3600
ttttcgtatc gttttttttt tagcgtgtaa gtggttttgt tattgtgcgt ttttggaagt    3660
ggggattttt ttgttttttt tttttttttt gttgtttggt cgtcgttaat tttattttga    3720
ttgagtgttg agttcgtttt tgggttttgg tttttgcggg gtaggttttt tcgggtttac    3780
ggggcggacg ttgtatatta atttgttttg tatcgtttgt ttcggagacg gagttcgaag    3840
gtttgttatg gcggttgtgt cgaggatgcg tggcgtttgt cgtggcggga tagcgtgggt    3900
aggtttattt ttgtttttaa ttcgggtttt aggataggtt agcgcgtttt gtttcggtgt    3960
ttatcgttgt tttttattcg tttttttgtt atttgttttt gattttttttg tcgtgttttt    4020
cgtagttttt ttatgtcgtt ttattttttt ttgtttttga gtcgaatatg ataataaaag    4080
ttattattga gcgtttattg tatgttaagt agcgtgttga gttggtattt tttgtttata    4140
gtttgttttt ttttcgggat cgtgagaatg agttaggttt tgttagtatt tttattttat    4200
aggttaggag atcgagggtt aggtagtttt agtgattgat ttgtttatag ttatatggtt    4260
agagtttggt agggttggaa gtggaattta ggtttggttt ttgtatgttt ttaaagtttt    4320
ttagggttgt aaagatatta agattataag tatttttttat cggttttggg gggggggggtt    4380
gatgtgtaat ttttagttgt ttttttttta tatataattt tgttgatttg gggaggcgtc    4440
gggggtggtg agagagattt agttttgtgg ggaattggga ggcgtatttt ttgttagttt    4500
tggagggagg tttcgacgat cgatcgtatt tgtagtttaa gttttttttt tttgtttatt    4560
attattggta gcgtttgtgg gtttggtgcg gtggggcggt gattacggac ggagtgggtt    4620
ggttgtgttt taaggttttt ttaagagatg tgtattttag gggagtcgtg tgtagtgtgt    4680
ggggggagga atgagcgttt ttataaaagg taggttgggt gaatgtatag gtttcgagaa    4740
cggagaattt atcgatttgg attattagtt gggcgtggga cgcggtgtcg tgggaggata    4800
tttgtttttt tttttaggat tcgattttcg gtttatattt atagggtagg tgaagttgat    4860
ggaggggggtt ttaagcgggg ttttagtatt tttaggggtt tggggttagt tttgatttta    4920
gtattttttt tttttcgttt tttagggatt tttgaggttt taaagttatt ttaggggata    4980
ttttattttt attagttttt tgttattcgg ggtagtttat ttttatttat ttttttttaa    5040
tcgtttagtt tggggtttaa ttttttttat cgggttattt ttattttttg ttgttttttt    5100
tttttttattt gtgcgatgtg attttgtttt attttgaaat ttatttttga cgtttgtttt    5160
taaggtgttt tgtcggtttt ttttatttat atttgttagt tttatagtta ttaaggggtt    5220
ttttatttta ggttttatta tattagttag gtggtagttt tttttttttgg tatatatttt    5280
tagttttgtg atttttaagt tttgatattt tagggttttt atttttattt ttaagttata    5340
ggtgaaagag aaagagaatg ataattttat ttatggttat agaatatttt cgggaagggg    5400
aaggatttgg tagtagggag taattacgat tggttagttg aagaaggtga ggatgttggt    5460
ttcgaggtgt ataggaaggg ggaaaatgtg cgagggtttt tacggtgggt agtgttagtt    5520
atggtttttt agtagataat aaggtatagt ggggtttggg gattgggggga ggttagtttt    5580
gttcgtttgt ttttgatatt aggaggtttg tgagtggagt tttgtaattt ttatttaatt    5640
tttgtataac gggggtttta tttttgagat ttttaaagta tggggtattt tagaggttcg    5700
ttttatagtt gagtattatt ttaaggattt ttttttatga cgggattttt agaaagagtt    5760
agggttagtc gagtagaatc gaggggtagg gcgggggggtt tggtgtggtt ttttgttcgt    5820
tatgtttttt atttggtagg ggtggttgtt cgatttagaa gttttagggg tattaggaac    5880
gggtttaggg gatagatttt agttgtttgt taaggcgatt ttagttcgtt gtgttcggtt    5940
ttttcggtgg ttggggggggt tttttttggt gttttggagt tagttttatt ttttttttgtt    6000
gtaggtttta ggtttttttt agcgggaatg ggttgggggg ttgttaggcg taggtacgtt    6060
ttcgttatat gtttaattag ggttgtgtag ggtaggagtg gtagtttttt tggaagtgat    6120
atgagtaggt cgaggattat agcggatttt agtgatggat ggaggagagg ggtaagggga    6180
tatttttgtt tgttgttagg gatttttgat tttttttgttt ttgttttttt ttttattttt    6240
tttattagtt gttgagagtt cggggttgcg gttatttcgg gagatagagg ataagtattt    6300
gtagattttt ttaaagttta tagtaaagtt ttttttagtg gagggattag tcgacgattt    6360
agaaggtttt ttaggggtag ggttaggagt tttgggtgcg gagttaggtg tagtaggggt    6420
agagttaggt attttagggg cggggttttt ttgtttttta ggggcgttta ggagtaaagg    6480
tttttttacgg gtttgcgtgg gtttgggggg tttggagtgt aggagggtga tcggtttcgt    6540
```

ggtcgcgatg gtgagtattt ggtaggtaga ataattaggg aggaagagga gttgggaagt 6600
ttttttatcg tttatatatt tttttatttt ttttgttatt ttaagatgtt ttttagataa 6660
gagatggttt tagagttagg gatattgagg ttcgaggtgt ttagtgattg acggagttta 6720
tagagttggt aaaagggttt aggatttagg tttatatgat ttggtttatg tgattttgtt 6780
ttttgggata ttttgatttg tttgggggaa tggcggtatt tgcgagaaag ttattgttag 6840
ggttttttttt agtggttttg ttattttttt agttagattc gtttagattt gagtgaagtt 6900
agaggtatag ggttagttta gggattagtt tgtaatggtt ttttaagttt tgtttttttt 6960
ggggattttt taattaggtt atttttcgta atttttattt atttattagg gatttgtttt 7020
ggatagtttt attttgttta tttgttttat ttttatgggg gttgggggtt gtgtttttcg 7080
gcgttttggt ttttgaaggt tattcggatg tattttttga atggtttttc gggttacgcg 7140
gtttttgagt tgttggagga agattcggat ttagaaagga aagaggcggc ggaatgaggt 7200
ttttatgagt ttagggttgt agtagagaag ttaaagttta attttttgta tgttttgggt 7260
ttcgttattg cgttttagtt ttttgttggt tcggtgttag gagttttagt gattgttaag 7320
gtttttattt tatttagagc gtgattagtt atttaaggaa gttgtaaggt ttaggttagt 7380
ttcggttttt ttacgtagtt agggtttaat tttatgttga ttattatttc gtttaatttt 7440
ttttttcgtt tttttttttt tatttttttt tattttaagt tcgtattttg tttaatagta 7500
tgcgttgttt ttcgttcgga ttcggtagta ttttattttt agtttatttc gttcgtttag 7560
ttttcgtttt atttacgtat tatttcgagg ttcgtttcgt ttttaaagtt tgtttttatt 7620
tcgtttacgt tttttttacg ttttattttt aaaacggttt cgtttttaag ttttgttatt 7680
aattcgttta cgtttcgttt ttaggttcgt tttgtttttt aaatttattt atttattttt 7740
taaattttag cggtacgtac gtttcgtttt cggtatcgtt tcgtttttttc gttttattta 7800
tgtttcgttt acgtttagtt tttaagttcg tttcgttttt aggttttttt ttttcgttta 7860
cgtttcgttt tttatttcgt ttaggtttat ttattaacgt ttcgttattt agttttacgt 7920
tacgttttcg ttcgtttata tttttggttt cgtttcgttg gtttttgtcg tagtttcgtt 7980
tatttttcgt tttattttag tttcgttttt agtttggttt cggtttcgag aatcgtattt 8040

tattttagtt tcgtttcggt ttcgtagttt tcgggttagt tcggtggcgt tcggtttcgg 8100
ttggttttgt cgcgtttgta ggagtcgtat tagttgtcgt ttttcgcgag cgttttaggt 8160
cgcgggatcg gttatttcgt ttagatagcg cgtcggggtc gttcgtcgtt tgggaggtgg 8220
ttttatgtcg tttagaaaga ggtaggcgtt gtaaggtcgt tttttcgatg ggcggagtta 8280
cgggggtcgt cgaggttaga aagaatcgcg cggaggcgga cgcgcgtggg ggcgtggttt 8340
cgcgtttgga ggttcgggga atcgttttaa tggaagtgaa ggggagatcg gtatcggaat 8400
tggggcgggg cgaaggagat ttttagtttg tatttttattt tatttatgaa gtggagacga 8460
tattttggat tttatgtttt ttttttgttg ttggattaat ttatgaagtt attattgtgt 8520
gttttgtttt tcgtagaggg gtgggttttg gtcgaaaggt taagttcgaa gatttagtcg 8580
gttaagagtt tgggttttgg ggttagatag gtttggggat tttgggtaag tgatttagtt 8640
ttgtcgagtt ttagtttttt tttttgtaga ttggggttga tagttttatt tttgtttgtg 8700
taaggtagag tggagttcgg gtaggaggga tttaaggagt tttagattag attgataatt 8760
taagaggagg gcgaagttta gattaggagg gaattggagt agaggtagaa ggtcgaagcg 8820
gagttagggt agggttcggg cgggggtaat ggagagcgga gaaggtttgg tttgagaaag 8880
atcgtaggag gtagatgggt aggtgttcgg gtaaggagag gagggagggg gaaggtttat 8940
tttaaattta cgtttgtggg gttgggtgtc gttttttttt tttttttaga tagatttttg 9000
tttttgttgt ttaggttgta gtgtaatggc gcgatttcgg tttattgtaa tttttgtttt 9060
ttgggtttaa gcgatttttt tgttttagtt tttttagtag ttgggattat aagtacgcgt 9120
tattacgttt tgttaatttt ttttttgtta ttttttttag tagggacggg gttttattat 9180
gtcggttacg ttggtttgga atttttgatt ttaagtgatt tatttatttc ggttttttaa 9240
tgtgttggga ttataggcgg gagttatcgc gttcggtcgg gtgtcgtttt ttgtttgaga 9300
taaaacgttt tatagttttt tatggtttgg gagtgtatac gggtatatgg gtatatgggt 9360
ttcgttttta ggttttattt ttgtaagtta cgatttttta ggaatatttt ttaagttttg 9420
tttttagttt tttttttaag tttttttatt tatttttttat ttcgtacgtt gcgcgtttat 9480
tgattgtatt agaatttagt cggttagttt agaggtttat tttgagtata ggtgggaggg 9540
atagggggag gagagaagcg taggggtcgt ggttttatta ggttcgaggt ttagggaggc 9600
gggttcggtt ttgttggttt tggtttcgaa ttggtagttg ggtgtatttt cggattatgt 9660
tgggggcgta ttatttttgg gaggggtggt tgaggtttag cgagggaggg gacgttacgc 9720
gggttatttt tttttttagt agattattag tttttttttat cggtttgtat gtcggggtga 9780
gagtgggagt cgggttcgaa tgttgagttt ttttcgttta gtgttttttt ttatttaatg 9840
tttttggttc gatttaaagt tcgcgtttgt cgcgggtgat ttattgtttg agttcgaatt 9900
taagttttat ttagtattgt gatttttgat aagttattta atttagagaa tgttttatta 9960
ttttcgtttt taaaatgggg attataatag tgttcgttta gaggttaagg ggattaagtt 10020
agtttatagg cgtaaaatat ttataatagt gtttggaggt tgggcgtagt ggtttacgtt 10080
tgtaatttta gtattttggg agatcgaagc ggtaggatta tttgaggtcg ggtgtgcgag 10140

attagtttga ttaatatgga gaaatttcgt ttttattaaa aatataaaat tagtcgggtg 10200
tggtggcgta tgtttgtaat tttagttatt taggaggttg aggtaggaga atcgtttgaa 10260
ttcgggaggt agaggttgcg gtgagtcgag atcgcgttat tgtattttcg tttgggtaat 10320
aagagcgaaa tttcgtttta aaaaataaat aaataaataa ataaataaat aaaaaaatta 10380
tagtgtttgg tacgaattaa gcgttaggaa gcgttattgt tagattttt attttattta 10440
gttttttta tttttatatt tattttttt attgggattt tttgggttgg ggatcgttgg 10500
gcgaggtttt cgcgggcggg ttaggggcgt ggggcgttgg aagggagagg cgagttgtag 10560
aagggttgtt gcgcgttacg ttaggttaag ggcggttttt gggggttgtt gcgggtatcg 10620
cggaggtagg tttagttagg gttgattagg tttcgtaggc gggatcgcgg tcgtttcggg 10680
tttcgacgcg cgggattaga ttgggggttc ggtttggcgg aggtcgtcgg tatttgagtg 10740
cgtatagacg agatttagtt cggcggttag cgcggcgttt agtacgaagg ttgttaatat 10800
tagcgttatt atcggcgcgg gttttagggt cgcgggggtc ggcgcgggag gtttagggcg 10860
tattgtacgg tcggggatat ttttgggcgg ttttgtggga agtgttacgt tgagattggg 10920
tttagttttt aggcgttttt tttattcgtt ttgtcgtttt acggttttat gcggaggagg 10980
attgcgtgtt tattgggggg cggtcgcggt acggtgatta cgataggttg cgttagcgtg 11040
tgtaggtggg ggtcgttagt agttaggttt tcggcgttgt tattgttagt gtcggcgtac 11100
gtttcgtttt gaggtagata ttgggggaga ggaaggtggg gaagagacgg ggatattagg 11160
gtatcgggat cgtggttttg gtttttaggg tttttaagag agatttgagt ttagtttttt 11220
attatagtta agatgggatt tttggaaatt cggtttttta gggtgttttg agtttagttt 11280
ttacgtatat aagtggttag tcgtttagtt tttaaggatt ttaaatttta gttttataag 11340
atcgtatggt tcgcgttttt tagtatttag tatgtgaggt ttagtattag gtttttagaa 11400
atgtgttttt ttagtggttt tttgtattta atttttagtt ttgaaggatt ttagagttgt 11460
ttatgggggga atttagtgtt tatattgttt tcggagaaat tttgtcggta gagatggatt 11520
tttgcgtttt attttttta atttttagat ttttagattt cgagagtatt ttttttttat 11580
tgttagagtc gttcgtgttt agttttcgaa gggttttagc gttcggattt taggttttgc 11640
gttcgcgtat tcgcgatggc ggcggcggcg gcggcggcgt tagaggcgta ggcgttcggc 11700
ggatttttcg gaggcggcgg tagcggttta gttggtagtg taggaattgt atcgaggcgt 11760
tgaagatcgg gcgtaggcgg aagttgaaac gcgcggggcg ggcggtattc gggttcggcg 11820
gcggcggtgg cgggaaggcg atagaggtgt cggcggggta gtttttacgt agtagagtta 11880
gggcgggttt cggggtcggg cgtgaggacg gcgttattga gtagcggtgt agggttaggt 11940
tttagcgcgg ggagggacgg gttaaggtcg tttgggggac ggaggcgtta ttagggatcg 12000
ttcgttttta tcggttttta ttcgttttg gtgttttcgg ggtttttatt tgtacgaata 12060
cgcggttgtc ggtcgggatt tcgagcggtt tcgcgcggcg aggaaagacg tttttgtgt 12120
cggatagttt taggttgaag aggggtttgc gtagttaggg gtcgggcgtc gcggggaacg 12180
ggggagttgg cggggcgggg aagggcggtt taaaggagag aaaacgcgcg ttgtttttta 12240
ggttttcggg aaaagtgatt cgattatttc gttttcgtcg tcgtttttgg aaaagggatg 12300
cggttgcggc ggtcgattta tttatgatgg gtttcgcgtt attttttttt ttgacggtga 12360
tgtgtttttt ggcgattttt gggattcgta tagtttagta attgagtcgg ttgtagagtt 12420
atgcgttttt agagaagttt tcgaagttgg cgaagttttt tttggggcgg aagagaagcg 12480
gtgaggagta ttcgatggtg gggttcggtt gggtgaagtt agagtttttt tattttttttt 12540
agggttgttt ttggggacgt agtaggatag gcggtttttt tttttaaggg gagataggtg 12600
ttgttattaa cgaggaaggg aagtgaagag gatatttttt taagaggttt ttttgggtat 12660
agttaaagtt tagttaggtt attaagattt gagacgtttt tatttgttta ggttgaggta 12720
gttatgggat tttttttttt ttcgtgtttt taaatatacg ttgatgtttg tttttaggtg 12780
ttgagcgggg ttttggtaaa acggttaatt tttaaatacg aaagattgtt tgtatattta 12840
ttttttttgt tttttgagat agggtttttt tttgtcgttt aggttggagt gtagtggtgc 12900
gaatatagtt tattgtagtt tttatttttt gggttttagt tattttttttg ttttagtttt 12960
tcgagtagtt atgattatag ttattcgtta ttacggttat acggggttta tgttgtttag 13020
gttggtttgg aatttttggt tttaagcgat tttttattt tagtttttt taagtgttgg 13080
gattataggt atgagttttt ttgtttattc gtatattata tttatttttg aagagtattt 13140
gggtagattg gttttttttt ataattttgg tttttttcgt ggttgtaggg agttttgtcg 13200
aagtggggat gtgagttatt tttgggatgg gggatagaga ttttatttta tttttttgtt 13260
ttgtttttat ttttatgagg attattatta aaggtttagt tgaaagagag tgatatcggt 13320
ggggcgcggt ggtttatatt tgtaatttta gtattttgaa aagttcgagg cgggcggatc 13380
gtttgaggtt aggagttcga gattagtttg gttaatatgg ggaaacgttg tttttattaa 13440
aaatataata attagttagg cgtggtggtg cgtgtttgta gttttagtta tttaggaggt 13500
tgaggtagga gaattatttg aatcggggag gcggaggttg cggtgagtgg agattgcgtt 13560
attgtatttt agtttgcgtg gtagagtaag attttatttt aaaaaaaaata aagagggtga 13620
aatagggata gaagtatgat agattcgtta tttttttgtt tatatatatt aggtggttcg 13680
ttgggtaagg tggtgatttt tgggagtagg gttagtagta gtacggtgtt tagtatgttg 13740
gattagttga ggtcggagtg tttggggtta gagtagatat tagttgggtt gtatttaggt 13800

ttcgtacgtg ggtaggcggt tttgtttttg gtaaggaggg aggtagggag cgggggtggt 13860
agattgaggg gttgatatta gagttaatgt ttaaggtttg aggggattaa gggatttttt 13920
gttgttcgat ttgtggttat tgggggtagg gggtttttta tattagttgt ttgatattta 13980
tcggtaagtt attataaata tttttcgttt agtttttaaa tttgttttag ttttttttta 14040
atgtagaaat tttttttcgt aattattttt tttttatttc gttttgttag gtttatgata 14100
tattatagat tcggtggaag aaaaggaagg ggtttatttt taagcgttta agggtttata 14160
aacgagggta ttttgtttta aaaaggggta gggcgatatt ggcggtttga ggaggggttt 14220
attggttggt gggttggttc gagttatttt taggtttttg tttattcggt tcgtttttttg 14280
tttggtttag agggatggtt ggtgacgagg ggggaggttt tgggagaggt tgggaggtag 14340
gagagatttt tttagggttt tttatttaat attgaagggg gtgggggtgg gcggatatat 14400
atttataaaa tagaaaataa agttaaataa aaataaaatt aggtagttcg gtttcgggtt 14460
tgtgttggtt ttgggattag ttatgtaatt tttcggtaat cgtagaggtt tttatggttg 14520
gatgtagggg aggaaagggt taggtttgtt ggtattttta ttcggtagat ttttggtttt 14580
tgataaaggg gttaaggggt aagagaaggt ggtttttttt tttgggggta gagagaagat 14640
gatatttatg attttttaaa tatgcggcgt tttgtgaata ttatgataat aataagagtt 14700
aataggttaa gtgagaggag agggatgtta cgtttacgtt gtttttatta cggttagtcg 14760
gtttttgcgt ttttggtgtt ggattttttt tgtgatttta cggggttttg tgtttttttt 14820
tttttgtttt ttgttttgtt attttttttt gtttatttga tttgtatgta atatttataa 14880
aaaggaaata tattttttttt tgttttggtt attttagtag gtttcggggg gtatttggtt 14940
tgggttttcg gtgtatttat tcggtttttt ttagttttgg attttaacgt ggattgggtt 15000
aggtgtgggt gagtggaggg acggttaggg gtacgatggg agtgattatt tcgtattttt 15060
atttttagtt cggtttttat ttttgttgga gtttgttata cgtttttaaa tttttatatt 15120
tatttttttt gttttttatt tttgattttt ttcggatttt tttttttggtt tcgtttcgtt 15180
atcgattttt ttcggttatt tgttttgtat tatgttttaa gattattttt tttgaaggtt 15240
attttgtgcg gttagggttt cgtagatttt tttatatttt ttgaggtttt taatttattt 15300
taagttgtgg ttttcggaga ttttagagta gtagttttag tttagttttt tagtgttcga 15360
gggcggttgg ggttttatta taagtagtta atcgtgattt tggtaaaagt ttttgaaggt 15420
atcgataatt ttacgagaat aagttttttt tcgtttttat tttaaatatt ttattaagta 15480
taataaaata taataaattt taaaaaggat tttttgagat taaagagaga gagagagaga 15540
gagagagagc gcgagcgcgt agtttcgttt agtcgtagtg gtaatgttta tattgtttgg 15600
ttgggggtta ttgatttatt gcgttatggg agatttatgt agatttaggg gattcgttag 15660
gaggtggttg ggtttaggtt taggttagaa gagagataga ggtagggagg gtgacgggag 15720
agttagataa ggatagagag gtagagtagg ggatagaggg cgacggaaga ggaacgggcg 15780
cgtgggagtg gcgaaggata tagtacgttg ggagcgtgta tggtggttgc gggattttag 15840
ggtttttttt tggtttttgg gggtcgtttc ggtttagagg ttgcggttga atgaatagcg 15900
attttgatcg cggtttgttg tttttcgttt atagtgtttg tcgggggacg tatacggtcg 15960
gttgatttcg gggtgggaag ggcgggggtt tggtaggttc ggggcggggg gggtgggtgg 16020
gtatttttta tttttttgttt ttagttttag gtggtcgttt aggttttagg ttttttttgg 16080
taggtagttg ggtggttaag tggggagggg ggtttgtggt tatgtttttg gtttttttgtg 16140
gggttggtcg tcgttaagta gttatttgaa gaagggtagg tggggttggt ttaggacgtc 16200
gttagttcgc ggtgatttag ttttcgttat gatatttttg aattaggacg ttacgtttat 16260
ttttagcgtt tcgtagcgtt tgatgaagtt gtgtttttgt gggttcgagg gggtggggtt 16320
taggaggaga cggttttttt ttttggtttc gcgttttcgt agggatagga cggggattgg 16380
gagggtttag gtatttataa gtagtttatt atattttggt ttttttttgt gatttttagt 16440
aaggttagag taagagaagg gaagatagat gtgagaacga gggaggaggg gatgtcggtt 16500
ttatttttgt gtttaggttt tttttaaggg gttaggggtt tttaagatgt gtgtggggga 16560
aggagttgtg gatgttttta ggatttcggt tgatagcggg gagtaattgt gagtttcgga 16620
gtttttttggt ttagggttag gtggtaaggg tttggtttgg ggggttagag ttggttgagg 16680
ttttttagaa aggtgggtgg ggtttttttg agagaatcga atatggtttt tttgaatttg 16740
tagttgtgtt tgttgtagaa gcgtgtttat aatttttaat agttatttcg ttaaatgtta 16800
tagggttggt tgttttttta ggttaggttt aaatttagtt tatggttttt tgggtattag 16860
ttttagtttt tgtggtagtc gttagttagg agttcgttta tagtcgtatt cgtttatttt 16920
agggtgcggt ttttattttt tttttgttat tgttttttta gttgtttaga gtagcgggtt 16980
gtttttgatt ttttaagtgg agggaggttt ttattagttt ttgacgaagg attggaagtt 17040
tttcgagaag tttatgtgtt cgggtagaag cgggggtttt ttttgtagga ttttggtgag 17100
gattttaaag ttcgttttgt agtttttgta gggaaattgt tttgttgtta gttttatttg 17160
ggagggagac gggtagggtt ggataggttt atgttttttt tttttcgagt tttttagaag 17220
tttttatttt tggttggaga atagggggagg gttttaattt attaacgaga tgtttaggtt 17280
ttatacgtcg gttcggatgt tatagttcgg tttggtgggg tttgggggggt taatgcgttc 17340
gggttgtagg gggggggagga gaagacgtat ttttaaggtt ggggtcggtt ttttagtttt 17400
tatttttttt ttcgttgggg ggtttttatt tattgttatg taggcggtat agtcggcgtt 17460

```
tcgcgttttg gttttggagt ttattaggcg gtcgttgatg tcgaagtcgt agagtttgat   17520
ttggtttcgt tcgtttagta ggatgttgga gggtttgacg tcgcggtgga tgatatcgtg   17580
tttttttttt aggtagtata gcgtttttat aatttgtagg tacgtagagg ggggtgtatg   17640
tggtaggggg tatagattta aggttatttg ttcggtgtac gttttatttg gttatttatc   17700
gttattgtta ttttgtttag tgtacgtttc gtttggttat ttatcgtttt tgttattttg   17760
tttagtgtaa gtttcgtttg gttatttatc gttattgtta ttttgtttag aatgcgttcg   17820
gggatggggt tttgtattcg tttttgagt tttttagcgt aggtgtttat gagttttatg   17880
gcgatgaaga cgttcgtttg tagggagagg ggtattcggg ggagttgtgt ttttcggttt   17940
atttttttaga ggttttgttt tttaggaagg ggtaggggtg agggaggagg agacgttgta   18000
gggcgcggtt aggtatttac gttggtgatg aacgttttaa agtattgtac gatgtagggg   18060
tagtcgtggt tttttagtat tatatttagg tttatgagga tgcgtttgtt tttttttttg   18120
ttttcggagc gtcgtatttg ttgtataggt agggatagga gggagtttta gtatcggttt   18180
ggggtgtggg gcgtagtcgg ggtagtcgtt aaggtttatt ttaacggtaa tgacgtggtc   18240
ggtttttcgg aagcgtattt tttatatttg gtcgtaggtg tcgttgttta tttcgtttaa   18300
gtttttttagg tcgttgattt ttgtttggta gcgttgggag gagagggagg gttggagggt   18360
ttttggggtt tttgttatcg tttttttgttt tttattggcg tttttaggat gggtttagtt   18420
tggttgggtt tttttttttt tttcgttata gtgaaggtgg tatttggttt tcgatggtta   18480
ggtagttcgt ttgttttatg attttttgta gtttttggtt aattttaatg ttggggatat   18540
ggagtaggtg tcggtggagg tgggttttga ttttaggttg gttattttag tttttttatat   18600
cggggttttt atcggtttaa ttattttttt tttagggttg gtacggtggt tttttaatgt   18660
tgttttttat ttaattttgt tgggtttttg gtttattttt ttatgttgcg gggtgtgaat   18720
agggttgacg ggagttttag tatgtggcgg ggtcgggcgg ggggcgtggg gtgttgcggg   18780
gagtttttttg aggatggcga gcggttgttt ttatcgttgg ttagcgggag ttgtagggtt   18840
agggggggtga gaattagtgg tagggagggg ggttagcgtt tagttggtgt tagttttata   18900
tttggtattt tagggagggg tcgggatttt tattttttttt ttttgggttt ttcgagttag   18960
gttggtatag ttttttgttt tgttgggttg gaggagttgg ggtttttttt ttattttaga   19020
ttgtttttag ggttttttgt tttatttttt gatttggaaa gagatggagg aggttagtag   19080
gggagaggga gttttaagtt tacgttgacg gagaaacgag atggggattt gaggtgtttt   19140
tcggttaggg ggtttttttg ataggtgtgt gtattttttt ttattttttgt ttaggaagta   19200
ggtttagggt tatagtattt ataggatgga taggttaggg cggagtgttt gttagggttg   19260
gtttaggttg tggttttgtt ggtagtattg aagttggttt ttagataaga gcgagtaggg   19320
ttacgttggg gaggtagcgg gtacgagatt taggttttag gttgttttgt tttatttttt   19380
gtttcggggt tagggagggt tttttttcggg ttagtgggtt gatatgaggg ttttttggtt   19440
tggtttgtag agttggggtt tatgggggta ggataggatt ttagggggtt tgtatttttt   19500
gggaggagag ttcgggggta tagtttttgt aggggttggc gggattaggt ggggttaatg   19560
ttgggttagg cgggggttgg tttatttagt agtttgtatg cgggtagttc gggtatgtta   19620
tagcgggtgg gtaggcggcg ggtaggtagt aggtattttg gggtgggtag gcggtattag   19680
gggtgggagg gtaggagata gatagataga taggaattaa gattagggtt taggtgttta   19740
gaggaggcgt tgtagagata ggttagagag gggtttggga taggagggta gtttttcgga   19800
ggagttaggg tttttttggt tgggttggta atagggtgta ggaatagagg tcgggatgtt   19860
aacgtggttt ttagaggagt taattttgat acgttatatt ttagaggttt tgataacggt   19920
tatatatatt tattaaggtg tttttttttat aggagttggg gtttgtcgtt ttttaaagtt   19980
tttagttaat aagttttttt tttttttttt ttttttgttt cgtttatttt tttttttttat   20040
agttttgtag gtttttggtg attggtgttt agggattaat atgaatagat aaattcgttt   20100
tatgttggta gttaaatttc gtttttttcg tgtgtttagg atttgatttt tagttagtag   20160
gaggggtagg aagacgtttt agttttaatg taggaggttt agtattgttt ttatttggtt   20220
ttgttagttt ttgtaggagt tgtgtttttt agtttttttt ttggaggatt aggtaggagt   20280
aggggaaggg ttttggggat tttgggatat agttaggttt agcgtttatg attggtttag   20340
aggttttatt tttcgtgttt ttttcgttgt tttggttatt gtcgtttttg tagtattacg   20400
ttatttttta ttgttttttt ttttttttga gataggtttt cgttttgtta tttaggttgg   20460
agtgtagtgg cgtgatttta atttattgta atattttttt tttaggttta agtgattttt   20520
ttgttttagt tttttgagta gttgggatta taggtatacg ttattatatt tagttaattt   20580
ttgtttttttg agtagagatg gggtttcgtt atgttggtta ggtttttttt tttgtttttt   20640
taggttttttg cgtttttgtt ttgtttatgg tttgagttat agttttagtt tttttttggt   20700
tttcgagggt ttgtgttttt tttaatttag tttttaattt ttgtgtattt ggtatttttt   20760
gagatattgt ttagttttcg tttttagaga gtattcgagt ttcgtttata ttttgtattt   20820
agcggttgac gattatagtt ttcgaggagt tttgtgggtt ttttttttga tagagtttat   20880
taggtttttc gagggtggtt tcggagtttg gggaggaggt tgtcggatta tagattggag   20940
tatgggggga tggaagggag ggtttaggta ggtattgggg gaaggaggta tataaaggtt   21000
ttgagaatag aggttataga ggtggtgatg gttatatggt agtttaggag ggtggagtga   21060
gagtaagagg gtgaggatga gaaagagaac ggatgagaga gagcgagagg ttggagggta   21120
```

```
cgtgtttgtc gataggagtt ataggtgtat cggttgttat gttaatatgt acgtatatgt   21180
cgtgtaagtg tacgtacgta taatatatat tcggggtatg tcgtggattt aatatttatg   21240
tagtcgggat agagtttttt ttgttttcgg atatatatat ttataattat ttatagggtt   21300
aggtatttta tttatttttt tttttaatat aggcgtgggg tatagtttgt atgttttata   21360
taggttggcg ttgcgtttag agttttggcg gttttgtttt tttttttttt ttaatttttt   21420
taagacgttt tttaaatttt tttttttttt gagacggagt tttgttttgt tgtttaggtt   21480
ggaatgtagt ggcgcgattt tagtttattg taatttttat ttaatttttt atgttttttt   21540
tttttttgag atggagtttt gtttttgttg tttaggttga agtgtaatgg tgcgatttta   21600
gtttattgta attttcgttt ttcgggttta agcggttttt ttgttttagt ttttcgagta   21660
gttaggatta tagtttttcg agtagttggg attataggta tgtgttatta tgttcggtta   21720
attttgtatt tttagtggag acggggtttt tttatgttgg ttagggtggt ttcgaatttt   21780
cgattttagg tgagttattc gtttcggttt tttaaagtgt tggaattata ggtgtgagtt   21840
attgtgtttg gtttaatttt tagtattttt aatgtagata gggttttatt atgttggtta   21900
ggttggtttc gaatttttga ttttaagtga tttatttttt ttggtttttt aaagtattga   21960
aattataggt atgagttatt gcgtttggtt tagtttttaa atttttgaga taggatttcg   22020
ttgttattta ggttggggtg tagtggtgtg attatggttt tttgtagttt taattttttc   22080
gagtgttggg attataggtg tgagttatcg tgtttggttt ttttagaggt tttgtttaat   22140
ttttatcgtt tttagaggtt tttttaggtt ggttttttgt agggtcgttt atttagttag   22200
aatgtttttt ttggtgggaa tgtgtttttt gtttagtggg ttttttttatt gtagtttttc   22260
gttatagagg agaggggttt tatttgtttt atttattgtt gtgtttttgg cgtttagggt   22320
tggtatggaa ttcgggggt gttatgtgtt tattt                               22355
```

<210> 177
<211> 11000
<212> DNA
<213> Artificial Sequence

<220>
<223> chemically treated genomic DNA (Homo sapiens)

<400> 177

```
gaaaggcgag cgttggtcga attttatttg attttagtta ttttttagga gtggtttggt     60
gtagattata atatttgtta tttatgtatt tacgtatttt tttttgaaag gagttgtaga    120
gtttttggaa ttttattttt tagcgaggaa aattttttag atttttattg ttttttggag    180
ggcgggggta gggttggtga aaatatgaga aagtttttttc ggtgtatttt atttgttttt    240
acgtgtaaat agttgtacgg tttttttttt attaatttta ttagaaggta tattagtaat    300
aattaaggac gttataggaa aatgggaagg tcgtttcgta gatcgcgcgt tttgtataat    360
gattcgggga gtgaatgggg gagtacgttc ggttttggtt ttggttgtta taagatgaga    420
ataattagta aatttttttt tattatttaa tttcgcgtgg taataaaatg gatttttttt    480
tatgaatgtt tggtcggttt atttattatt ttttttttat tagtaatttt ttgtttcgta    540
tttagttttt taatttttta tttttttttcg tttttttagcg tatatagtgt tttttatgtt    600
aggtcgggtt cgtttttttatt ttgtaattat aaaggttatt aaagtaatta tcgtcggttt    660
tgattggaaa agttggttat taattagttt ttttttgttt tcggtgtagc ggattagttg    720
gtgtcgggat tgagttaatg gaaacgcggg ttaaatgaga aaagacgttc ggattttttt    780
gtttggatgc gcggttcgga ggaggatcgc gaggtttttt agcggcggga ggcggtggag    840
tcgtcgcgga gagggaggga ggttttggag agggagggaa ggaaaggggg agggagaggg    900
aaagggggga gagggagagg aggcgcgggg tgggggaggg gagtgagtag ggagtcggga    960
gagggaggag gggcgggaat taggggagcg gttcgaattt cgtttggttc ggatttcgta   1020
gttatcgttg ggtttcgtcg tcgggtcgtt tttcgcgtgg agattcggtt cgcgtttttt   1080
tttcggtttt tttttttttt tttttttttcg tttttttttt ttcggtttat atagtttttt   1140
ttagaaagaa gttattttag agtcgcgcga tttagtttta gagttcgtcg ggttcgttt   1200
atcgggtttt ttgcgcgttt tttttcgttt ttttttcgggt atagttcgtt tacgaaattt   1260
aaggcgtcgg ttacgcgtta tttttttcgg gtcggggttt tttcggtttt cgcgcgggcg   1320
ttggtttttt cgggtgggcg gtagtttcgt tttgtgtttt tttgggtcgg cgcgagggat   1380
tttagagcgc gcgggggggtt ttaggcgggg aggagcggtc gcgatttcgg atgcgagagg   1440
cgtgtgggtt taagcggcgt cgggataggg gtcgggtttt ttgtttattt tagggtttat   1500
gttcgtttgc ggggtcgcgt ttttcggagt tatatttggg cgggagggtt tttttcgggg   1560
gttcggtttg cgtttttcgt cgtttttcgt ttttcgtttt tcgtttttcg cgggtttcgt   1620
ttggttttgg tcgacggaat aagaataata aaggaagcgg cgcgatcggc ggagggggcg   1680
ttcgtcgttt ttttattttt tatttcggtc gggtcgatgg ttttcgcgcg tttttattcg   1740
```

```
ttttagcgcg gaggcgattt cggtttcgtt gtttcggcgt cgggaggata tcgcgcgttt    1800
acgtggatga ttgcgttcgg gaatagggat ttttcgaatt tttttgtttc gcgtcgtgag    1860
gtttatacgc gcgcggtgat tattttggtt ttgagggggc ggcggggtta cggcgtgtgg    1920
cggattcggt agtggtattt aggttgtgcg cgcgggtgtg tggggtttcg aggcgtaggg    1980
cgatcgtcgg tttcggtttt tcgaggaagg ggtataggcg ttgttcgcgg ttattcgggt    2040
taggtgaggg taggggacgc gcgggggtcg gaggaggagg tttggtttgc gggcggtagg    2100
agggattcgg ggttagtcga ggttgttttt agggaggtag atatttgttg tcgtcgggat    2160
tttcgatacg tttcgtacgc gcgggagcgg aatcgggttt gttttggagg tttttttttgg    2220
cgcgtttgga tttatttaaa ggttaaagaa aaatgttaag gagagcgatt gtttggagag    2280
tttttggttt ttttttcggg ttttcgttgg gttttaggag ttttcggagg ggaagtaagg    2340

agttttagtg ttttggagtt ttgtttttat taatttcgtt cggtttttttg ttgtttaggt    2400
tcgcgttgtt ttagtagagg aggggtggga gtcgttttag gtttattttt tcgcgtcggt    2460
ttataaggag gtttttttttg taatttggga gaggtcgggg gaggttcgga gggcgatcgc    2520
gcggggtcgc gggatttgga tagtcgggga gtcgtgatcg cgggtagcgt cggggttgag    2580
ggcgaggagg gttttattgt agggaagggc gggacgttgg atagagggat cgattggggt    2640
ttatcggttt gatatagaag tcgttggatt ttagggttcg gttgggaaat tcgtttttta    2700
ttcgttcgcg ggcgtcgtag aagaaatcga agaaagaggt ttttttttta cgtttaggac    2760
ggttttaagt gttttattta gcgttgagta ggagggtgat gcggagggaa aaattgttgg    2820
ttgtaagtta ggttttagga atttcggaag aaacgaagat ttaaagaatt ttttttttaag    2880
tgattgagtt ttaataattt tataatgatt tgattttttt tttgtggagt taaaaaaaat    2940
ttttaaattt ttgttagtta tggtttttaa gagaaagaaa tgagtttaaa agtgagtttg    3000
aggtatagtt ttgatttagg tgtggttttg gtggttttta gcgttatttt ttttatgggt    3060
atcgattttt taaggttgta ttacggcggg tggattttaa agagagttta gattaattag    3120
aggtatttta ttagttttga tttgaaattg aaagaaaatt atgtttaaat atttatgatt    3180
ttgatattta tagttaggcg tttttgaaaa gcggaatatt ttttttttgta tagcggtagt    3240
ttaatgattg aaagtttgga atagagaaga agtgaggagg aggaggaggg agaaggaggt    3300
ttggtaatat agcggtaggg gtgcgtttcg ggtgggggag atgtatagat aggtatattt    3360
tagtgttgag tttttagagg taggaatagt ataatttaaa aaaataatta ggaatgatta    3420
agttaatttt attttaggtt agatttattt tgagaataag atgaagtata atatcgtata    3480
tttttttttt ttaatcgttt gtatttgtaa ttttttaaat ttagaataga tagtattgta    3540
aaagttgtga tttaaaataa gtgtatgatt atagagataa tttgtggagt tgttttagaa    3600
tttagtattt gtagatattg ttttgattaa tttaaattat tttgatcgtg tttttgagtg    3660
gaggtgtatg aggtttttat tttatgtttt tgtgagttat ttacgggaaa atgtttttag    3720
aattttttta gttgtttatg tggtaagttt aaaagaaaag aaaaaaggat atgttatgtt    3780
taaatttaat tgtgagagtt gatatagtta tataaatata aaattttatg gtaaatatgt    3840
gttgacgggt agttagattt agtttttttt taaggttgtg tgatataatt agttaatagg    3900
agtagtttat tttttgagtt acgatgtgta gtattaaaat atgtttgtat tataaagacg    3960
tagggatata ggtgttcggt gttagttaat gttaaatttt taatattgtt gatgttttta    4020
tttgtattat ttttaaggaa ttagatttta gttttagttt tattttattt ttgtgtgtat    4080
ttaaattatt aaattataaa tatttttttt ttttaaaatt tagaagttag ttttagagat    4140
tattaatgag ttgttgtaat aagtttattt ttggtttgtt ggatgaattt ttttagaatt    4200
attaggaaat tttattttaa atttattaaa aggtattatt aaaatataat atttatttta    4260
gggttgatga atattttaag tttttaggtt atagtaagtg atttaatttg aaaatacggg    4320
ggaaggggtg ttttaaaata ttttaaaagt atttttttaaa gttgttaacg gaattatttt    4380
taatttggaa tttgttatag aattttaata aatgagagaa gttaggcgga aataattatg    4440
tattaatttt ttgtattata aaattttttt agaagttatt attagagtaa gaggtattgc    4500
ggtttgaatt tagggtggga gaagtttttt gtgtaattga aagttattgt tttttcgtgt    4560
agatgaaatt gtatttgtaa atttgttatt taagtggtta aaaaatttaa aaaagtaaaa    4620
gaaaagaatt tttaaggaaa aattattttt aaatgtaaat tttaatgaaa ataagttggg    4680
atatattgtt ttaaaatggg ttatttgttg agttttttta ggtatatatt taaaagtgaa    4740
tatataagaa aggtaataaa ttgtttattt gttattttta agtattttta aaatattagt    4800
tttaaagaaa tattgtgttt taagatatta gggaagattt atgatattga tgtttaaagg    4860
gggaagtatt tttgtgtaaa ttaattttaa ttagtagatt gttttcggtg ggagagaggg    4920
atttagaggt tgagtagggg aaggatgttt ttgagaaagt cgtttttagt ttttttttaga    4980
tttttcgttg gcgtttttat gatatttaaa tttagattta gcggattata tttaaggtat    5040
cggtttttga tttttatttt tggttcgttc gtttggtttt taggtttgtt tggacgtacg    5100
cgcgtttttt atattaggtg tttttcggga cggggtttgg tttttttatt tcgtaagaat    5160
tagatttttt ttaggattgg gggggttatc gtttcgtttt ttttattcgg atttagaagt    5220
tttaatttaa gattcgttta gcgtcgtttg ttagaatttt tttgtggtac gattttgagt    5280
agagggcgag ggaaaggagg gttagcgttc ggtttcgttt tttgtttggg tttttttttt    5340
```

cgttttttcg ttttttttt ttcgtcggtt tatcgtattt ttataggagt tataaagatt 5400
aggttttagc gtcggatata aaggttaagg gttttcggga gtcgcgggta tttagagtta 5460
ggagaggggt ttcggcgcgg agggtgttgc ggtttagttt tttggttata gagcgcgtcg 5520
gtcgcgaggg aggattaagg tttttaatta cgttcgcgtt tattcgtgaa tttcggcgtt 5580
cgtagttttc gggcggcgta gtcgttatcg tcgtcgtttt ttttggagcg tagaggggtg 5640
tgtttgggag ggggcgggag ggtatttttt tttgtgtcgg tgaaaggaag agtttttagt 5700
ttttttttc gtataaattt ttattttttt ttttgttcga tcgcgtttta aaatttggga 5760
tcggcgacgt aaggatagtt tggtttggcg aagatttttc ggatattttg cgtttcggat 5820
tttaggcggg tggttttacg gtgtcggtcg ggacgcgtag ttcgggggc gggattacgt 5880
tcgggatttc ggggtcgttg ttcgaggacg gaggtcggtg atttgaatcg cgtcgttttt 5940
agggttgtgg cggggttttt tttttagtcg tttagttcgt agcgtttagt ttagcgtatt 6000
gaggattaaa aagggcggga gattttttc ggttttaaaa ggtaattgtg ttgagatttt 6060
aattttaatt tgggggttcg ttcgattttt tggagtaacg tttttttaaa attacggagt 6120
aggttagtgt tcggaattgt ataggtagtt acgaaggaag gaaatgaagt ttttcgtttt 6180
aatagtttcg ggtttagtta taagtttttt tttggcgagt acgcggtcgt tttatttggg 6240
gtgggggagg cgttgagaaa gcgtagtttt ttttggggga ggagagatgg ttttttagag 6300
gcgtttttat ttgtttttgg aacgtttttt tttgtaggtt ttttcggttt tttgtaattc 6360
gtcgttcgtt gggatatttt tatggttcgg aggcgcgttt tcggattttg gtgaggaggg 6420
tgaagagcgt tttaaaatgg gttgagagga aaatgcgttt atattggcgt ttttttattt 6480
tggttgttcg ttttttttt ttttttttt tatacgtttt ttattttcgt gtttgtattt 6540
ttttattttt agtattttgt aatggtcgga attgtaggga gtggggagta acgcgaaagg 6600
tcgagagaag agcgttgggg agaagtttcg gaggttttag attttttggg gcggtttttg 6660
cggggcgttg ggttaggata gttgttaatt ttttaaattt gtgcgtttag aaggagttgt 6720
tttttgttcg ttttttttta taggtttatt ttagatttat ttggggatga ttttaggcgt 6780
agtttttttt tgtcggttta agtttttgat aggagttttg cggcgaggta gagatttttt 6840
gaattgagtt tttcgacgtt ttagttgttc gttagagttt ttatgcgcgt tttttagttt 6900
tcgaaagttt tttttttcgt tttattattt acgcgtttgg tgatgttttc gtgggttttt 6960
tttgtttttt tcgtttttat tttagttttt ttcgtttttt ttagggttta tttaatcgta 7020
ttacgagcgt tcgcgtttcg ttcggtattt ttggttattt gtagttgggt cggcgtttag 7080
cgttcgcgcg ggaggaggtg gggcgtgcg tcgggtcgag cggtttcggg gcgtagtagt 7140
tgcgggtagc ggtagcgtta ttattatagt tagttgtttt tttgcgttgg agttatagat 7200
tagcggggtt attcgttttt gattataaat ttttagggcg gtcggaggag ataaaggtgt 7260
cgcgcgtcgt tgttttagta gtagcggttg cgcggcggta cgggttcgga gttcgtaggc 7320
gtcggagttt tgcgcggtcg ttcggtttag tttcgcgtgt ttcgtttcgg ttcggttttc 7380
gggttaggta cgttgggtcg gggcgcggcg cgggcgggtt tagggcgggt ttcggacggt 7440
ttcggtaggg tcgtcggtcg cgcgcgtagt attaattagc gcggtcgtcg cgtttcgttt 7500
tatattcggt tcggcgcgta ttcggtcggc ggcggcgcgg ttcgcggttt agttatcggt 7560
tgttatagcg atgttttttt tttttttat atgttgttga tagtaattgg ttaaggggtc 7620
gcggtttttt aaatattttt tttcgttcga gattatgaat ggatattagt tttgttagtt 7680
attggtataa cgtcgaagtt agggttttcg ttttttttt ttttttagtg tttttttttt 7740
ttatcgttta tattgggtaa gtttagggta gtatcgaggg gagagagatt tcggttagaa 7800
aagttaaggc gcgcggtttt gttgtgattt ttgttttggg aggggtgggt ggggtgcgtc 7860
gttattttg gtaagagaaa attgtacggt atatcgtttt ttttagttat tttttttagt 7920
gttgtcgttc gggttttttt gttttttttt taaatggaag taggttaatg atttagggcg 7980
agtagacgag agtttttttg tttattttat tagggttttt tcgagtgttt aggtttcgat 8040
gcgcgtagaa agtgttttgc gagacgcgga attggtgaga ttttgttatt atttggttta 8100
ggttaatgtc ggtcgttagt ttaaaacgtc gaagggagac ggcggtgaaa atttaggcgt 8160
gcgagttgcg tttttgtttt tttttttatt taggttaatg gtttggttta gattatcgtt 8220
ttgcgggcga aaggagtgga gtagaattat aggttacgtt tttagtcgtt cgagtttgtt 8280
tatataattt tcggtttttg gcgtagttag gttcgtagcg ggcgagaaga gttagggttt 8340
cggggcgatt aggattattg agtatttttt tttcgcgttt ttcgtttatg tttttttttt 8400
ttaggttttg gatttcgggt tatagttata ggttggggta tttcggtgcg cggcgttttc 8460
gcggagttgg cgagagtcgg agagttgaaa tttaatattt gtgtgtataa tgcgtttttc 8520
gaggtttata gttgtaggaa taaggagaag aaaggaagag gggggcgtag attttgttat 8580
acgtagatga tagtttgcgg agggtttttt agtttttttt tttttggaaa ttttagcggt 8640
ttttttttag gttttttttt tttatcgttt ggttattgtt ttttgcgttc gggatagttg 8700
gagacggcgg ttcgcgttat tcgacgtttt tttcgtcgta tattcggttg ttcgcggcgt 8760
tcgttttcgg ttttttcggg gttaattttt ttatattttt tatttttta ggttaggttt 8820
aggcggcgtt tttatagttt ttttttttt tttttgtatt ttggcgttgt ttttagattt 8880
gttcggtgta gagggttttg tttggcgttt ttttttata aatttagttt tgtaaaattt 8940
gggggcgagt tttagggggt agggtttaga ggcgggggtt gtttatagat atgagtacgg 9000

```
tgtcgttcgt tgtaggtttt tgtttaaaag tggtcgaagg atttcgtttt ttttgattgt    9060
aggtttaggt tgagtttcgt atttattttt gtatttatag ggtaaggtta gagcgtttta    9120
taaagtttta gggtaaagaa gtggtttcgt atcgggttat ggttttcgta ggtttgagcg    9180
ggttgtagga ggttcggtgc ggttcggcgg gggcgcgaga ggcgagaaat tgcggcgtta    9240
ttggcgtttt ttgtttatga gtgaattttt ttagtaggcg gggacggggt atattatttt    9300
ggttttttcg ttttttttgt ttagggtaag gttcgtaaaa attttcgtat tttggttcgg    9360
gggcggcgcg gcggcgggta ggttagagag aagcggtatt cgggtttttt tagcgttcgg    9420
gatgtttttt tagggatttt ttcgggtaaa gagtcgagtg gtagcggttt ttgttttttt    9480
tcgttatttt ttttcggtgt ttcggcgttt ttagttaggt tttagggcga ttttaggttc    9540
ggttttgggt agagatagag gaggggaggg gaggtggcgt tggggggcgg ggggtcggag    9600
acggcgggag gaggattcgg gagtcggggc gggaagaggg gaagatagcg gagagaaggg    9660
acggggaggg gagggggttc ggaggagggg atttgttgtt tttagtttgg ttggtaatcg    9720
gtttttttat agtaacggtt agcgcgcgcg tttgtgtgtg cgcgcgtgtt tgatgtgtgt    9780
gtgttcgtgg tgttttcggg atttttcgcg gggttggga ggggatcgta gaattttagg    9840
gtggcgatag agtaaatttt tttaggattg gaggttcgat tttcgcggtg ttgggagcgt    9900
tttagggttt ggttgcgtcg cgttggcgac ggggtaggtt cggggaacgc gcgtaggttt    9960
cgggatcggt cgttggggta ttggggtatt ttgttttcgt tgtttcgatt ttttcgttgc   10020
ggtcgtaggg ttttggggta tcggggagtg ggcgggtttt tttagcgatt cggagtatag   10080
gggcgggcga cggcgttttt attatggttt tgttcggttc gagcgttcgg cgtatttgag   10140
gtttgggtcg gggagtagtt ggggaatcgc gcgtcgtttc ggggagtttt cggttaggtc   10200
ggtttcgggt agaggttaga ggggttttgg cgtatttttt tcgttcgcgg cgatttcgta   10260
ttattttttt cgttagttcg cggttttttt cgttttggat cgtcggagtt ttatttaggt   10320
tggggttttt agtaggcggt cgggagtttt tcgaggtggt taggagtggt cggtagtcgt   10380
tttttttcggt taggtcgtgt ttagtttttt agtttttacg gcggcgtaga tcgtacgcgg   10440
ttattttggg tttcggatag ggttaggggg acgtagattt tgtcgtttgg gggttttggg   10500
gcggttacgg ggtcggcgat tttagagtaa ggcggagttt agtataggtt agagagttgg   10560
ttttgttttt tttatagata tgatttgata gtgaatatat aaataaatat aattttttttc   10620
gttagttttt tttttcgttg ttttagtgtt ttatttttaa tattaaagta tcgtattttt   10680
gatttgattt taatttaatg atttgttttt ttagtgtttt gaggaaagat gggtgggagg   10740
tggtattaat ttagtttaat ttgggttatt ttaaaaatcg tgagagggtt tgaaaggttt   10800
tatgcgggtt tattagtaga atagtatatt ttttcgttta cgttttggtg tatttgcggt   10860
aatatatttt gggtggtaga cggttattta tgtttgagag tttttaattt attttttttt   10920
tttttattat ttttttttta aggtgcggtt tgattttagt ggggttgtag aattggtttc   10980
gttaaagttg aaggcggatt                                                11000
```

<210> 178
<211> 11000
<212> DNA
<213> Artificial Sequence

<220>
<223> chemically treated genomic DNA (Homo sapiens)

<400> 178

```
ggttcgtttt tagttttggc gaggttagtt ttgtagtttt attgaggtta ggtcgtattt      60
tagaggaggg gtggtgggag gaaggggagt gagttggagg tttttagata taaataatcg     120
tttgttattt agaatatatt gtcgtaggtg tattaaggcg tggacgagga agtgtgttgt     180
tttgttggtg aattcgtatg gaatttttta ggttttttta cgatttttaa agtgatttaa     240
attgaattga attggtgtta tttttatttt attttttttt aaagtattag aaagataggt     300
tattaaatta agattaaatt aggaatgcgg tgttttgatg ttggaagtga aatattgaga     360
taacggaaga aggattaac gaaaagaatt gtatttattt gtgtatttat tgttaggtta     420
tgtttgtgaa gaaagtaaaa ttagtttttt ggtttgtatt aggtttcgtt ttgttttggg     480
gtcgtcggtt tcgtggtcgt tttaggattt ttagacggta gggtttgcgt ttttttagtt     540
ttgttcgagg tttagggtgg tcgcgtgcgg tttgcgtcgt cgtggagatt gaggggttgg     600
atacggtttg gtcgagggag acggttatcg gttatttttg gttatttcgg agagttttcg     660
gtcgtttgtt ggagatttta gtttggatga ggtttcggcg atttagggcg aggagagtcg     720
cgggttggcg gggaaggtgg tgcgaggtcg tcgcgggcgg ggagggtgcg ttagggtttt     780
tttggttttt gttcggagtc ggtttggtcg ggagtttttc gaggcgacgc gcggtttttt     840
agttgttttt cggtttagat tttaggtgcg tcgggcgttc gggtcgggta gggttatggt     900
gggggcgtcg tcgttcgttt ttgtgtttcg ggtcgttggg aagattcgtt tatttttcgg     960
```

tgttttaagg ttttgcgatc gtagcgggag ggtcggggta gcgagggtag gatgttttaa 1020
tgttttagcg gtcgatttcg gggtttgcgc gcgtttttcg agtttatttc gtcgttagcg 1080
cggcgtagtt agattttagg gcgtttttag tatcgcggag gtcgggtttt tagttttgga 1140
ggggtttgtt ttgtcgttat tttagggttt tgcgattttt ttttagtttt cgcggggagt 1200
ttcgggaata ttacgggtat atatatatta gatacgcgcg tatatataga cgcgcgcgtt 1260
ggtcgttgtt atggagaggt cggttattag ttaggttgag ggtagtaggt tttttttttc 1320
gagtttttt ttttttttcgt ttttttttt cgttgttttt ttttttttc gtttcggttt 1380
tcgagttttt ttttcgtcgt tttcggtttt tcgtttttta gcgttatttt ttttttttt 1440
ttttgttttt gtttagggtc gggtttgggg tcgttttgga gtttggttgg ggacgtcgag 1500
gtatcggagg agggtggcgg ggagggatag gaatcgttat tattcgattt tttgttcgag 1560
aaggttttg ggagggtatt tcgggcgttg ggggaattcg agtatcgttt tttttgatt 1620
tgttcgtcgt cgcgtcgttt tcgagttagg gtgcgggggt ttttgcgggt tttgttttgg 1680
gtagggggga cgagagggtt aaggtgatgt atttcgtttt cgtttattgg aggggtttat 1740
ttatgggtag aggacgttag tgacgtcgta gtttttcgtt tttcgcgttt tcgtcgagtc 1800
gtatcgggtt ttttgtagtt cgtttaggtt tgcgggaatt atggttcgat gcgaggttat 1860
ttttttgttt tggagtttta tgaagcgttt tggttttgtt ttataaatat aaaaataaat 1920
gcggaattta gtttgggttt gtagttagag gaaacggggt ttttcggtta tttttagata 1980
ggggtttgta gcggacggta tcgtatttat atttgtggat aattttcgtt tttgagtttt 2040
gtttttggg gttcgttttt aggttttata gaattaggtt tgtgagagaa aagcgttagg 2100
taaggttttt tgtatcgagt aggtttaggg gtagcgttag agtgtaaggg aggggagggg 2160
agattgtgga agcgtcgttt gggtttggtt tggggaggtg gggggtgtgg aggagttggt 2220
ttcggagggg tcgggagcgg gcgtcgcggg tagtcgggtg tgcggcggga ggggcgtcga 2280
gtggcgcggg tcgtcgtttt tagttgtttc gggcgtaggg aataatggtt aggcggtggg 2340
agaggggggt ttaggagggg gtcgttgggg tttttagaaa aggagaaatt aaagagtttt 2400
tcgtagattg ttatttgcgt gtgataagat ttgcgttttt ttttttttt ttttttttg 2460
tttttgtagt tgtgggtttc ggagagcgta ttgtatatat agatgttggg ttttagtttt 2520
tcggttttcg ttagtttcgc gggggcgtcg cgtatcgggg tgttttaatt tgtggttgtg 2580
gttcggggtt tagggtttgg ggagagagag tatgggcggg aggcgcgggg gggaggtgtt 2640
tagtggtttt agtcgtttcg agattttagt ttttttcgtt cgttgcgggt ttgattgcgt 2700
taggggtcgg gggttatgtg ggtaggttcg aacgattggg agcgtggttt gtgattttgt 2760
tttattttt tcgttcgtag agcggtggtt taggttagat tattgatttg ggtaggggga 2820
agggtaagga cgtagttcgt acgtttgggt ttttatcgtc gttttttttc ggcgttttgg 2880
gttggcggtc gatattggtt tggattaaat ggtggtaaag ttttattagt ttcgcgtttc 2940
gtaagatatt ttttacgcgt atcgggattt aaatattcgg gaggattttg gtaaagtgag 3000
tagaggggtt ttcgtttatt cgttttgaat tattggtttg tttttatttg gaaagggagt 3060
aaaaaggttc gaacggtaat attggaaaag gtaattggga agaacgatat gtcgtgtaat 3120
tttttttgt taagagtggc gacgtatttt atttattttt tttaggataa aggttatagt 3180
agagtcgcgc gttttgattt ttttggtcga ggttttttt ttttcggtgt tattttgaat 3240
ttgtttagta tgaacgatga aagggagaaa tattgaaaag aaagaaaagg cgaaggtttt 3300
ggtttcggcg ttgtattaat gattgataaa gttgatgttt atttatagtt tcgggcggga 3360
gagggtgttt ggagagtcgc ggttttttag ttaattgttg ttagtaatat gtgggggggg 3420
gggggatat cgttatagta atcggtggtt gggtcgcgag tcgcgtcgtc gtcggtcggg 3480
tgcgcgtcgg gtcggatata aggcggggcg cggcggtcgc gttggttggt gttacgcgcg 3540
cggtcggcgg ttttatcggg atcgttcgga gttcgttttg agttcgttcg cgtcgcgttt 3600
cggtttagcg tgtttagttc gggggtcggg tcggggcgga gtacgcggag ttgggtcgag 3660
cggtcgcgta gagtttcggc gtttgcgggt ttcgagttcg tgtcgtcgcg tagtcgttgt 3720
tgttggagta gcgacgcgcg gtatttttgt ttttttcggt cgttttagaa gtttgtggtt 3780
agaagcggat gatttcgtta gtttgtggtt ttagcgtagg gaagtagttg gttgtgatga 3840
tggcgttgtc gttattcgta gttgttgcgt ttcgaggtcg ttcggttcgg cgtacgtttt 3900
tattttttt cgcgcgggcg ttgggcgtcg gtttagttgt aggtaattag gggtgtcggg 3960
cggggcgcgg gcgttcgtag tgcggttggg tgggttttgg aagaggcgag agaggttggg 4020
atgggggcgg agggagtagg aagggtttac gagagtatta ttaggcgcgt aggtggtggg 4080
gcgaggaaga gagttttcga aggttggagg gcgcgtatgg gggttttaac gggtaattag 4140
ggcgtcggag agtttagttt aggggtttt tgtttcgtcg taggattttt gttaaaggtt 4200
tgggtcggta ggggagagtt gcgtttggga ttatttttaa gtgggtttag ggtgggtttg 4260
tgaggaggag cgggtaaagg gtagtttttt ttgagcgtat agatttggag ggttggtagt 4320
tgttttggtt tagcgtttcg tagagatcgt tttaaggagt ttaggatttt cgggattttt 4380
ttttagcgtt ttttttttcgg tttttcgcgt tgttttttat tttttgtagt ttcgattatt 4440
gtagggtgtt gggggtgggg gggtgtaggt acggagatgg gaggcgtgtg ggaaaggagg 4500
agggaagggg cgagtagtta ggatggagag gcgttagtgt gggcgtattt ttttttagt 4560
ttattttggg gcgtttttta ttttttttat tagggttcgg gagcgcgttt tcgagttatg 4620

```
ggagtatttt agcgggcggc gaattgtagg gaatcgaggg ggtttgtagg gagaggcgtt    4680
ttagaggtag gtggggacgt ttttgagagg ttattttttt ttttttaaag agaattgcgt    4740
tttttttagcg ttttttttat tttaggtgag gcggtcgcgt gttcgttaga gagaaatttg    4800
tgattggatt cgaagttgtt ggaacgaggg attttatttt tttttttcgt aattatttgt    4860
gtagtttcga atattggttt gtttcgtggt tttagagaag cgttgtttta gggaatcggg    4920
cgaattttta agttagagtt aaaattttag tataattgtt ttttagggtc ggagagagtt    4980
tttcgttttt tttggttttt agtgcgttga gttgggcgtt gcgggttgga cggttgggag    5040
aggggtttcg ttatagtttt gaggacgacg cggtttaagt tatcgatttt cgttttcgga    5100
tagcggtttc ggggtttcgg acgtggtttc gtttttcgag ttgcgcgttt cggtcggtat    5160
cgtggagtta ttcgtttgga gttcggagcg tagggtgttc gaggggtttt cgttaaatta    5220
ggttgttttt gcgtcgtcga ttttagattt taggacgcga tcgaataaga gaggaagtga    5280
aggtttgtgc ggaggaggga gttggaagtt ttttttttta tcgatataga ggaaagtgtt    5340
ttttcgtttt tttttaagta tattttttttg cgttttagag aagacggcgg cggtggcggt    5400
tgcgtcgttc gggggttgcg agcgtcggga tttacgggtg gacgcgggcg tggttgggga    5460
ttttggtttt ttttcgcggt cggcgcgttt tgtggttagg aggttgggtc gtagtatttt    5520
tcgcgtcggg attttttttt tggttttgaa tattcgcggt tttcgggaat ttttggtttt    5580
tgtgttcggc gttggggttt gatttttatg atttttgtgg ggatgcggtg aatcggcgag    5640
gggggaggga cggggaagcg ggggagaaag tttaggtagg ggacggggtc gaacgttgat    5700
tttttttttt ttcgtttttt gtttaaagtc gtgttataga ggggttttga taagcgacgt    5760
taggcgggtt ttgggttggg gtttttgagt tcgggtagag gaggcgaagc ggtaattttt    5820
ttagttttga agagggtttg gtttttgcgg ggtggggagg ttaggtttcg tttcgagaag    5880
tatttggtgt ggaaggcgcg cgtgcgttta ggtaggtttg ggattaggc gggcgggtta    5940
aaaatagggg ttaggagtcg gtgttttaag tgtgattcgt tgagtttgag tttgggtgtt    6000
atgaaaacgt tagcggagag tttgaaagag gttgggggcg gttttttttag gggtattttt    6060
tttttgttta gtttttgggt tttttttttt tatcggaggt aatttgttgg ttagaattag    6120
tttgtataag ggtgtttttt tttttggata ttagtattat aaattttttt tgatgtttta    6180
aaatatagta tttttttaaa attgatgttt taggagtgtt taaagatggt aaatagataa    6240
tttattgttt tttttgtata tttattttta agtgtatgtt tggaaaaatt taataaatag    6300
tttattttaa aatagtatat tttaatttat ttttattaaa gtttatattt aagaataatt    6360
tttttttgaa gatttttttt ttttgttttt ttgggttttt tggttatttg aatggtagat    6420
ttgtaagtat aattttattt gtacggagga ataatagttt ttagttgtat aaagggtttt    6480
ttttattttg aatttagatc gtagtgtttt ttgttttgat aatagttttt gaaaagattt    6540
tgtaatgtag agaattaata tatgattatt ttcgtttgat tttttttatt tattagaatt    6600
ttgtggtaaa ttttaaatta aaagtagttt cgttaatagt tttaaaaaat atttttaaaa    6660
tattttaaga tatttttttt ttcgtgtttt taagttggat tatttgttat ggtttgaaaa    6720
tttaaaatgt ttattagttt taaagtgagt attatatttt agtaatgttt tttagtaaat    6780
ttagaatgaa attttttaat aattttaaga ggatttattt agtaaattaa agataaattt    6840
gttatagtaa tttattaatg gtttttaaaa ttgattttta agttttaaga gaaaaaggta    6900
tttataattt agtaatttaa atgtatataa agataggatg aggttgggat tgaaatttga    6960
ttttttagaa atgatataaa tgaaagtatt aataatgtta gaagtttaat attaattggt    7020
atcgagtatt tgtattttta cgtttttatg atgtaagtat attttaatat tgtatatcgt    7080
gatttagaga atgaattgtt tttgttaatt ggttatatta tataatttttg gaaaaggatt    7140
aaatttggtt attcgttaat atatatttat tataaagttt tgtatttgtg tggttgtatt    7200
agtttttata attgagtttg ggtataatat gttttttttt tttttttttt aagtttatta    7260
tatggatagt taaaaaagtt ttgaaggtat tttttcgtaa atggtttata agggtatggg    7320
gtggaagttt tatgtatttt tatttagggg tacggttaaa ataatttaaa ttggttagga    7380
tagtgtttgt agatgttaag ttttaagata gttttataaa ttgtttttgt ggttatatat    7440
ttattttgaa ttataatttt tgtaatattg tttattttaa atttgaggag ttgtaggtgt    7500
agacggttga gaggaagaaa tatgcgatat tgtattttat tttattttta agatagattt    7560
aatttgggat aaaattaatt taattatttt tgattatttt tttaaattat attatttttg    7620
tttttgagaa tttagtattg aagtgtgttt atttgtgtat ttttttttatt cgggacgtat    7680
ttttgtcgtt gtgttattag gttttttttt tttttttttt tttttttatt ttttttttgt    7740
tttaaatttt tagttattga gttgtcgttg tatagaaggg ggtgtttcgt tttttaaaaa    7800
cgtttgattg taaatgttaa agttataaat atttaaatat aatttttttt taattttaag    7860
ttaagattga taagatgttt ttaattagtt tgggtttttt ttggggttta ttcgtcgtgg    7920
tgtagttttg gggggtcggt gtttatgggg gagatggcgt tgaggattat taaagttata    7980

tttgggttaa gattgtgttt taaatttatt tttaaattta tttttttttt ttaaaggtta    8040
tggttagtag gagtttgaag attttttttta attttataaa aagggagtta agttattgta    8100
aagttattgg aatttaatta tttagaaaaa aattttttaa attttcgttt ttttcggat    8160
ttttaagatt tgatttgtag ttaatagttt ttttttcgt attatttttt tgtttagcgt    8220
```

```
tgggtgggat atttgaaatc gttttgggcg tggagggaag attttttttt tcggtttttt    8280
ttgcggcgtt cgcgggcggg taggaagcgg gtttttttagt cgggttttgg gatttagcga    8340
tttttgtgtt aggtcggtgg attttagtcg gtttttttgt ttagcgtttc gttttttttt    8400
gtagtaagat ttttttcgtt tttagtttcg gcgttgttcg cggttacgat ttttcggttg    8460
tttaggtttc gcggtttcgc gcggtcgttt ttcgggtttt tttcggtttt ttttaggttg    8520
taggaggggt ttttttgtgg atcggcgcgg ggaggtgggt ttggggcggt ttttattttt    8580
tttttgttgg gatagcgcgg gtttaggtag tagggagtcg ggcgaggttg gtggagatag    8640
gattttaggg tattggggtt ttttgttttt ttttcgggga tttttgggat ttagcgggga    8700
ttcgggagga gagttaggag ttttttaggt aatcgttttt tttgatattt ttttttgatt    8760
tttgagtaaa tttaagcgcg ttaagggagg tttttaaagt aggttcggtt tcgttttcgc    8820
gcgtgcggag cgtgtcgagg gtttcggcga tagtaggtgt ttgttttttt gggaatagtt    8880
tcggttggtt tcgggttttt tttgtcgttc gtagattagg tttttttttt cggttttcgc    8940
gcgtttttttg tttttatttg gttcgagtga tcgcgggtag cgtttgtgtt tttttttcgg    9000
ggagtcggag tcggcggtcg ttttgcgttt cggaatttta tatattcgcg cgtatagttt    9060
gggtgttatt gtcgggttcg ttatacgtcg tggtttcgtc gttttttttag aattagggtg    9120
gttatcgcgc gcgtgtgggt tttacggcgc ggggtaggaa ggttcgggaa gtttttgttt    9180
tcgggcgtag ttatttacgt gagcgcgcgg tgttttttcg acgtcggggt agcggggtcg    9240
gggtcgtttt cgcgttgggg cggatggagg cgcgcggggg ttatcggttc ggtcgggatg    9300
aggggtggga gggcggcggg cgtttttttc gtcggtcgcg tcgttttttt tgttgttttt    9360
gtttcgtcgg ttaaggttag gcgggattcg cgggaggcga gaggcgaggg gcgaggggcg    9420
gcgaggggcg taggtcgggt tttcgggaaa agtttttttcg tttaggtatg gtttcggggg    9480
acgcggtttc gtaggcgaat atggattttg gggtgggtag aggattcggt ttttgtttcg    9540
gcgtcgtttg gatttatacg tttttcgtat tcggggtcgc ggtcgttttt tttcgtttgg    9600
ggtttttcgc gcgttttagg gtttttcgcg tcggtttagg agggtatagg gcggggttgt    9660
cgtttattcg ggagaattag cgttcgcgcg gggatcgagg agatttcggt tcgggggagg    9720
tggcgcgtgg tcggcgtttt aggtttcgtg aacgggttgt gttcggggag gggcggggag    9780
gggcgcgtag gagattcggt gggcggattt cggcggattt tggggttggg tcgcgcggtt    9840
ttagagtgat ttttttttgg aagaggttgt gtgggtcgga gggagggggg cggaggggag    9900
gggaggggag gagatcgggg agggggcgcg gatcggattt ttacgcgaag ggcgattcgg    9960
cggcgagatt tagcggtggt tgcggggttc ggattaaacg gggttcgggt cgtttttttg   10020
gttttcgttt tttttttttt tttcggtttt ttgtttattt ttttttttta tttcgcgttt   10080
tttttttttt tttttttttt tttttttttt tttttttttt tttttttttt ttttaaagtt   10140
tttttttttt ttcgcggcgg ttttatcgtt tttcgtcgtt ggagaatttc gcggtttttt   10200
ttcgagtcgc gtatttaggt aggaaaattc gggcgttttt ttttatttaa ttcgcgtttt   10260
tattaattta gtttcggtat tagttaattc gttgtatcga aggtaaaagg aggttaatta   10320
atgattagtt tttttagtta agatcggcga taattgtttt ggtggttttt atgattataa   10380
gataaaaacg ggttcggttt gatataagag gtattgtgta cgttgggaga cgggaggaaa   10440
tgaagagttg ggaggttgaa tacggagtag aaaattatta atagaagaga gataatgaat   10500
aggtcggtta ggtatttatg gggaaaaatt tattttgtta ttacgcgaaa ttaggtggtg   10560
gaaaggggtt tgttaattgt ttttattttg tagtagttag gattaaggtc gggcgtgttt   10620
ttttatttat ttttcgagtt attgtgtagg gcgcgcggtt tgcggggcgg ttttttttatt   10680
tttttgtggc gtttttaatt gttgttaata tattttttaa tgaagttaat gaggggagag   10740
tcgtgtaatt atttgtacgt ggagataagt gaaatgtatc gaaagggttt ttttatattt   10800
ttattagttt tattttcgtt ttttaagaag tagtgagaat ttgaagggtt tttttcgttg   10860
agaagtgaag ttttaaaggt tttgtagttt tttttaagga aaaatacgta ggtgtatagg   10920
tggtaagtgt tgtggtttgt attaggttat ttttggagag tgattggagt taggtgggat   10980
tcgattagcg ttcgtttttt                                                11000
```

<210> 179
<211> 1743
<212> DNA
<213> Artificial Sequence

<220>
<223> chemically treated genomic DNA (Homo sapiens)

<400> 179

```
ttgtttttta aatttcgtcg taattgtgaa tgtttgcgat gttattatat ttttagtata      60
agagaattgg agtattttttg gtttttttta taatcgaagg gtttttagcg tagggcgtag     120
ggggagaggt tcgaagtttt ggagatagcg ttcgagggat ggaggtcgcg ttcggttgcg     180
```

```
tttaggttcg ggtggtttcg ggagttgaga tttcgtagga tgtagcgggg gggggttagg     240
gtttgatttt ttatttttgc gagttagggt cgtttacgtt tttattgttt tttataggta     300
acgtttagtt ttttttttt ttatttttcg aaatatttcg gtttcgtttc gtttcgttcg      360
tcgttcgtga tttcgatttc gattcgttcg cgacggcgtt ttttttagga ggtagcggag     420
gattagggtt aggtcgttta ttttggcggc ggcggcggtt ttcggttaag gcggattttg     480
gggaggatag tagttgggtc gcggtttgtt tttaggggga gcgggcgtag ggtcgcgagt     540
gggtagcgtt ttcgtttcgt acgaataaag cggttaggcg cgtcgggtcg aggtagtatc     600
gttttttcgg gagcgcgggg ggcggggagg atggcggggg tatttatttg agggtcggcg     660
gagggcgcgg gcgggatagg tttaggagac gggatcgcgg cgggtcgggg tttcgcgtcg     720
agggcgtacg ggttacgggg aaggggcgat cggcgcgacg ttcgcggcgg ggttcgggag     780
tttcgcgtcg cgggtcgggg gtcggggttc gagttcgatt ttgcggtcgc gaggtatggg     840
aatcggttcg tcgggttagg tagcggtgtt aggtagtagg ggtagcgacg tcgtcgtttt     900
ttttttttt tgttttcgtt tttgtttcgt cgcgttttcg ttttcgttgg gttttcgtcg      960
tcgtcgtttt agtttcggcg tttttcgggt ttttttttt tattaaggtc gggtcgattc     1020
gtagggatta tttcggaaag tgaggggttg ttgtcgtttt tcgtagttgt tggtggttat    1080
ttttaatttt ttttttttt ttgtttttt tatttttcgt tggttgtttg attgattgat     1140
tggatttttt ttttttttt tttgtttttt ttattgagtc ggtcgtagaa attgtagtcg    1200
tttagttttt atttttttt gtttttttt ttttttttt tattttttt ttttttttcg     1260
gtttttcgtt tttgttttat ttttagcggt tgttttcgtt tttgtttgta gatagcgtcg    1320
ttggatgttt ttagttggat tttaatttta tttttttag ttttttttt tattgttttt     1380
tagacgcgtt tttttttcgt ttcgcgtttt ttttttttt tttatttttg tttttttcg     1440
cggcgtttat tttttttagt tttagttttt gaaaggattt agttgagaaa ggataattgg    1500
gtttcgtttt ttttaatttt atatttttta gttggatgtt gttagaggcg atggagaaac    1560
gtaaaggtgc gttaatttta ttttagttag agtataaaag aattgtttac gttagttatt    1620
gtattacgta gtttcgaaat ttttgtacgt ttttttgtgt ggttttcgtt cgtgatggga    1680
ttgtagttga ggagttcggt tttaaatggt gtttttcgtt agtgattttg agtcggttgt    1740
tta                                                                  1743
```

<210> 180
<211> 1743
<212> DNA
<213> Artificial Sequence

<220>
<223> chemically treated genomic DNA (Homo sapiens)

<400> 180

```
taggtaatcg atttaaggtt attgacggaa ggtattattt gagatcgagt tttttaatta      60
tagttttatt acggacggga gttatatagg agagcgtgta ggagtttcga agttgcgtag     120
tataatagtt ggcgtgggta gttttttat attttggtta gagtaaaatt aacgtatttt      180
tgcgtttttt tatcgttttt gatagtattt agttaaaggg tgtagggtta aggaaagcgg     240
aatttagttg ttttttttta gttgagtttt tttagaaatt gggattgagg agggtgagcg     300
tcgcggagag gggtaggggt gggagaggag agaggggcgc ggggcgggga agaggcgcgt     360
ttggaaggta gtggggagag ggattgagag gagtggggtt gaagtttagt tgggagtatt     420
tagcggcgtt gtttgtagat aggggcgaag gtagtcgttg agagtgaggt aagagcggga     480
agtcgaggga agggaaggaa gtaaggaaag aggaaggaaa ggtaggaggg ggtagaagtt     540
gagcggttgt aatttttgcg gtcggtttag taggaggagt aggaggggaa agaggaggat     600
ttagttagtt agttagatag ttagcgggag gtggagaaag taggaggagg aggaggatta     660
aagatggtta ttaatagttg cgggaaacgg taataatttt ttatttttcg ggatggtttt     720
tgcgggtcgg ttcggttttg atggagagaa gaaattcgag gagcgtcgag gttgaggcgg     780
cggcggcggg gatttagcga ggacgaggac gcggcggagt agggacgggg gtaggagaag     840
ggaaaggcgg cggcgtcgtt gtttttgttg tttagtatcg ttgtttggtt cggcggatcg     900
gtttttatat ttcgcggtcg tagaatcgag ttcgggtttc ggttttcggt tcgcggcgcg     960
gggttttcgg gtttcgtcgc ggacgtcgcg tcggtcgttt ttttttcgta gttcgtgcgt    1020
tttcggcgcg gagtttcggt tcgtcgcggt ttcgtttttt gggtttgttt cgttcgcgtt    1080
tttcgtcggt ttttaggtga gtattttcgt tattttttttc gtttttcgcg ttttcggaga   1140
ggcggtgttg tttcggttcg gcgcgtttgg tcgttttgtt cgtgcggggc ggaggcgttg    1200
tttattcgcg gttttgcgtt cgttttttttt agggataggt cgcggtttag ttgttgtttt   1260
ttttagggtt cgttttggtc gggagtcgtc gtcgtcgtta gggtggcggt tttggtttta    1320
gtttttcgtt gtttttttggg gagggcgtcg tcgcggacgg gtcggggtcg gggttacggg   1380
```

```
cggcgagcgg agcggagcgg aatcgggata tttcggggga taggggagga gagggttgga    1440
cgttatttgt gggaagtagt gggggcgtag gcggttttgg ttcgtagggg taggggatta    1500
ggttttggtt ttttttcgtt gtattttgcg gggttttagt tttcggagtt attcggattt    1560
gagcgtagtc gagcgcggtt tttattttc gggcgttgtt tttagggttt cgggtttttt    1620
tttttacgtt ttacgttgga agtttttcga ttatggaagg agttagagat gttttagttt    1680
ttttgtattg gggatgtggt ggtatcgtaa atatttatag ttacggcggg atttaagaag    1740
tag                                                                  1743
```

<210> 181
<211> 10196
<212> DNA
<213> Artificial Sequence

<220>
<223> chemically treated genomic DNA (Homo sapiens)

<400> 181

```
aagtgtacgt ttatcgagga ggtagttata gttataatta tattgttata gtcggcgtat      60
atttgtatcg ggttatagtt acgattatat attaattata gttatatata tagatagaaa     120
tatgtagtta ttcgtaggta tttttatttg aattttgtcg tatatagtta tatagtatat     180
agtcgtatat tattacgggg ataaacgtag taatatttaa ttaggtataa gtttttttata    240
gtcgtatata taatgtcgta cgtggttttg ttttttttta tatatatatt agtagttata    300
gttttcgagg tcgtgttgta tacggtgtga gcgttggaaa tatatttatt ataagggtag    360
tttgttataa tcgcgcgatt atttattata tatttttata tagacgtaag cgtacgtttt    420
taattttatt tttatcgtcg tataaagtcg aggtttggtt ttcggtttcg ttttttttcg    480
ggttttttat tttaggtttt attagtttc gaagtagtag aatttttttc ggtattgggg    540
tagttgttgg taaagttttg agggtttcgt tagcgcgtga gttggaagtt gaattatcgc    600
gagaagtata gttttgtcga ggagattttt gggaagcgta gttagagacg tattaaggtc    660
ggataagatg gttttcgatc gttagaattt ttgttcgcgt tttgcgttag tttcgtcgaa    720
aggatcgttt tttttagtta aatcgttcgc gttttagagg ttttatggag tttcgtttta    780
ggttcgggtt ttggttttta aagaaagtta tttcgacgtg tttgagatgg gtttagtttt    840
cggattcgtt ttattgtttt taggcgttgg atagttttag aaagaggata agtttgtatg    900
gaaagataat cgtataattg agtttttgg gagatgtagt tcggagttgg gaaggcggtt    960
ggattttcg aaagttattt taattgtttt tatagataag tcggtgttgg gttgtcggtt    1020
tgagtattgt gcgtttagag tgcgtaggcg taaatatttt gggttgttgt tttggttttt    1080
ttgttcgggc gtgaattttg tttattgttt tttagtgtag aggggttatg ggattagagg    1140
ttgtgagatt tagaagtggt tggggacgta gaaggggtgt agattaggaa tttgcgattt    1200
gatgggggga ggtcgaaggg aaaggaatcg ttaaacgtgg gataggtttg gagaggtaga    1260
aagagaggag tttgaaattg taaagggtag gtgttatttt gatttttgtg tgttttgtga    1320
taaatgagtg gggtaggaag taagaggagt tttatttttt ttttttgtaa ttatttgtgt    1380
atttggatta agtgtatttt atggttagta tttgattgtt tttgatttat aaaggtattg    1440
tatatatagt tatgtatagt ttaataataa ggatatatgt ttttttttacg gtttttttttt   1500
tttttttttt ttacggtttt tttttgatgt cgagttaaag ttggattgta ttgttgtttt    1560
ttcggtttat tgtaattttt ttgtttgatt tttttgtttt agtttgtcga gtgtttgcga    1620
ttgtaggcgc gcgtcgttac gtttgattgg ttttcgtatt tttttggttt ttattaaata    1680
tagggtttcg ttgtgttggt cgggttggtt tttagttttt aatcgcgagt ggttcgttag    1740
tttcggtttt tcgaggtgtc gggattgtag acggagtttt gtttatttag tgtttaatgg    1800
tgtttaggtt ggagtgtagt ggcgtgattt cggttcgtta taattttttt ttagtcgttt    1860
gttttggttt tttaaagagt cgagattgta gtttttgttt cgtcgttatt ttatttggga    1920
agtgaggagt ttttttgttt ggtcgtttat cgtttgggat gtgaggagtt tttttgtttg    1980
gttgtttagt ttggaaagtg aggagcgttt ttgttaggtc gtttatcgtt tgggatgtgg    2040
ggagcgtttt tgtttcgtcg tttcgtttgg gatgtgagga acgtttttgt ttggtcgcga    2100
tttcgtttgg gaggtgagga gcgtttttgt tcggtcgttt cgtttgagaa gtgagatttt    2160
ttgtttggta atcgtttcgt ttgagaagtg aggagttttt tcgttcggta gtcgtttcgt    2220
ttgagaagtg aggagttttt tcgttcggta gttatttcgt ttgggaagtg aggagcgttt    2280
tcgttcggta gttattttat tcgggaggga ggtgggggtt attttcgtta ggttagtcgt    2340
ttcgttcggg agggaggtgg gggggttagt ttttcgttcg gttagtcgtt tcgttcggga    2400
gggaggtggg ggggttagtt tttcgttcgg ttagtcgttt cgttcgggag gcgaggggcg    2460
tttttgtttg gttattttta ttgggaattg aggagttttt ttgttcggtt agtcgtttcg    2520
ttcgggaggg aggtgggggg gggttagttt ttcgttcggt tagttttttc gttcgggagg    2580
```

```
tgaggggcgt ttttgttcgg tcgtttttat tgggaagtga ggagtttttt tgtttggtta    2640
cgattttatt tgggaggtgt atttaatagt ttattgagaa cgggttatga tgataatggc    2700
ggtgttgtgg aatagaaagg cgggaaaggt ggggaaaaga ttgagaaatc ggatggttgt    2760
cgtgtttgtg tagaaagacg tagatatggg agtttttta ttttgttttg tattaagaaa    2820
aatttttttg ttttgggatt ttgttgattt gtgattttat ttttaatttt gtgtttttg    2880
aaatatgtgt tgtgtttatt tagggttaaa tggattaagg gcggtgtaag atgtgttttg    2940
ttaaatagat gtttgaaggt agtatgttcg ttaagagtta ttattatttt ttaattttaa    3000
gtatttaggg atataaatat tgtcgaaggt cgtagggttt tttgtttagg aaaattagag    3060
atttttgttt atttgtttat ttgttgatat tttttttatt atcgttttat gattttgtta    3120
aatttttttt tgtaagaaat atttaagaat gattaattaa aaaaaaataa ataaataaat    3180
aataaggata tattttggtc gggtgcggtg gtttacgttt gaaattttag tattttggga    3240
ggttgagggg tttgagatta gtttgggtaa tatggtgtaa atttcgttta tattaaaaat    3300
gtaaaaatta gtcgggtatg gtggtgcgcg tttgtagttt tagttattcg ggaggttgag    3360
gtaggagaat cgtttgaatt taggaggtag aggttgtagt gagttaagat tgtattattg    3420
tattttagtt tgggcgatag agtgagattc ggttttaaaa aaaaaaagga tatattttga    3480
gaaatgtatt gttgggtaat tttattatta tggagtgtat tgatataaat ttagatggta    3540
tagttgattg aatatttagg ttatatggta taatttgttg tttttaaatt ataaagttat    3600
atagtatgtt attgtattga atatagttgg aatattgtag ggattagttt tataaggttt    3660
gtgggttttt tttttgtgt gtagagatta gaggtggtag aaataaagat ataagataaa    3720
gagataaaag atatttgggt tcgggtgagt tattattatt aagatataga gattgatagt    3780
ggtttcgaat gttaggttgt attgttattt attggatata agataagggg ggtagggtaa    3840
ggagtgtgag ttatttttaa tgataggtaa ggttacgtgg gttacgtgtt tattggatag    3900
ggggtttttt tttgtttggt agttgaagcg gagagagaga gaagatagtt tacgtattat    3960
ttttgtatat tagagatttt tagtattttt attaattttg ttattgttat ttagaaggta    4020
gggttaggtg tataggatgg aatatgaaag cggattagta gtgtgattat tgaagtatag    4080
tattatagga agacggttag atttttagat aattgcgggc gggtttgatt tatgttaggt    4140
tttttataag atatggtgga gtagagtttt ttttaaattt tttcggggaa agggagattt    4200
tttttgttgg tttgttaagt agtgggtgtt ttttttggt attgacgtta ttgttagatt    4260
ataattcgtt tggtaatagg cgttttttta gacgttaacg tttcgttaga ttaaggagtt    4320
ttttggtggt tttgttcggg tataatagag gtttatatat ttgtttttg gttatttttt    4380
attatgtttt tttagtttat attttgtat ggtttggttt tttttaggtt atgattgtag    4440
agtgaagatt attataatat ttgaataaag agtaattatt ataaattaat gattagtgat    4500
atttgtgtat aattatattt gtgatttgta tttagtgtaa tttttgttat tttatatatt    4560
ttattatatt gaaatagttt atgttttcgg ttttttgttt cggtatttgg gtggtttgtt    4620
gtttatagaa tataatggta agtatttatg tatttaaata tatttaaata tataaaaggt    4680
atagtaaaaa aaatagtatg aaaagtaaaa aatggtatat ttgtataggg tatttattaa    4740
gaatggaggt tgtaggattg gaagttgttt tggagagtcg atgagaggtg agtgaatgtg    4800
aaggttagga tattatagta taatatttta gattttataa atattgtata tttaagtaat    4860
attatatgta tttaaagttt tttttttata ataaattaat ttttgtttgt tgtaattttt    4920
ttattttata aatgtttaaa tttttaaaag ttttttgatt tttttgtaat atcgtttagt    4980
ttaaaatata aatatattgt atagtttatg tgggattcgt attttttggt aaggaaatgt    5040
tgtaggttag atgttatttt tatgtggtta tatagaggta gaaagattgt atatatgata    5100
ttgtttttttt taattacggg ttcgtatttt aatatatcgt aataatgttt aatatttatg    5160
gggttataga aaataggatt tattatggtt ggagaatttt tagagttaat ttaagtttag    5220
gaatgttggt aatgagaatg attagtagga attagtgtaa tgatttatat agttgaaatt    5280
attaattggg ttaggttggt ggtttacgtt tataatttta gtattttgtg aggttgaggt    5340
gggttgatta tttgaggtta ggagtttgag attagtttgg gtaatatggt gaaatttcgt    5400
ttttattaaa aatataaaaa ttagttaggt atggtggtat atatttgtga tattagttat    5460
tcgggaggtt aaggtaggag aattgtttga atttgggagg tagaggttgt agttagttaa    5520
gattatgtta ttgtatttaa gtttgggtga tagagtaaag attttatttt aaaaaaaaag    5580
ttaaaataga ataaaatata tagttattat ttttttgttt ggggtaattt taaagttttt    5640
tttttgttat aaaaatagga atgtattatg taaaggtttg aaataggtta tttttttttt    5700
tttttttttt gagttggagt ttcgttttttg ttgtttaggt aggttggagt gtaatggtat    5760
gatttcggtt tattataatt tttatttttt gggtttaagt aattttttttg ttttagtttt    5820
ttaagtagtt gggattatag gtatgtatta ttacgcgtgg ttaattttgg gtttttagta    5880
gagacgggat ttttttatgt tggttaggtt ggtttcgaat tttcgatttt aggtgatttg    5940
tttgtttcgg tttgttaaag tgttgggatt ataggtatga gttattgtgt ttggaaaata    6000
ggttattttt ttaaataaaa aggtaatgat ttataaaagt ttatgaatag aatatgtaat    6060
tagttgatat aaatttaata ggatttgttt aaaattagtt aaatttaata tatttgaagt    6120
gtttttgtat aaagtattat atgaagaaaa gaagaattta ttaatgtttt aaaaaaatgg    6180
gttttttttat agataatatg ataatttatt attaaaagtg tttcgggacg gtcgggtgta    6240
```

gtggtttacg tttgtaattt tagtattttg ggaggtcgag gcgggtggat tacgaggtta 6300
ggagatcgag attattttgg ttaatatagt gaaattttat ttttattaaa aatataaaaa 6360
attagtcggg tatggtggcg ggtgtttgta gttttagtta ttcgggaggt tgaggtagga 6420
gaatggcgtg aatttgggag gtagggtttg tagtgagtcg agatcgtgtt attgtatttt 6480
agttcgggcg atagagtaag attttatttt aaaaaaaaaa aaaaaaaaaa agtgttttag 6540
gagatataag gaaatgtaat attatttttt ttgaatattt ttagtttaag atttttttatt 6600
taataaaata ggaaaggatt tgaaattgag aaaatgtatt tgagtataaa tagtttgtga 6660
aatataatat ttttttttgt attattagag ggttttattg aatttataat taatttgttt 6720
atttagtatg taatttagat gtgagtgagg ggtcgggcgt agtggtttat ttttgtaatt 6780
atagtatttt ggaaggtaga ggcgggcgag ttatttgagg ttaggagttc gagattagtt 6840
tgattaatat ggtggaattt agtttttatt aaaaatataa aaattagttg ggcgtggtgg 6900
tgtatatttg taattttagg tatttcggag gttgaggtag gagaatcgtt tgaatttggg 6960
aggtagagat tgtagtgagt ttagatttcg ttattgtatt ttagtttggg tgatagagta 7020
agattttatt ttaaaataaa ataaaataaa aaaaatagat gtgagaggat agaggtgttt 7080
tataggagtt tgtattgttt ttaattttat gtgtgtaggt atttattata ggtaaatagt 7140
tttttttatat tttgtagtta tatgatttttg ttattagtaa aaagaggtaa atagttttttg 7200
tagaatgaag ggattatagt tacgggatta gagttataga atgaagtttt tttttaata 7260
ttttttaatt tgatgtttga attttttttt ttttttttt gagatagttt tgtttttttcg 7320
tttaggttag agtgtagtgg tacgattttg gtttattgtt atttttgtg tttaaataat 7380
ttttttgttt tagttttcgg agtagttggg attataggcg ttcgttatta tatttagtta 7440
atttttgtat ttttagtaga gacggggttt tattatgttg gttaggttgg ttttaaattt 7500
tttattttag gtgatttatt cgtttcgggt ttttaagtgt tgggattata ggtatgagtt 7560
attgcgtatg gttcgatgtt tgaattttta atgtagtatt ttagagaagg gtgttcggga 7620
ttttttgtgt ttaagggatc gataaagaaa aaagttttat ttattatttg ggatttgatg 7680
tatagatgaa aaatttaata tataataacg gaagtcggtt gttagtaaat tatattttag 7740
ttttttagta tttttataag agacgatatt tttagttgtt tagtttttgt agttttagta 7800
cggtaatatt aatgatgttt atgtttagaa ttagttaaaa agattataat ttgtttttttt 7860
tagtttattt atttttatt ggtttatggt tttaagtgta tttgaatgat tatttttggg 7920
tattttttgt tattgtttat tagggtgttg tcgattgttt ttatgttttg tgggttggtt 7980
gggaagggga gtttgggaag gatgtgttat ttttaggagg ttgtaagtta ttgggacgtt 8040
tttagggatg atttttttta tggttgtagg aagttttttt ggagttacgt ttacgatgtt 8100
tggcggatat ttgtatgttg tatcgttgag ttcgggatga attgtttgtt ggtttattgt 8160
tgattaaggc gttgatgtga taaataattt tttgtttata tagtttttta agtttttttgg 8220
gacgcgattt gtgatgattc ggcggatttc ggtggtagtt gtttttttta tttttagtga 8280
cgtttgtatg ttgttttagg tagtgtgagg agtgaagatg agatttggcg tattttttaa 8340
cggagtttga gtaaagttaa agggtttcga ttcgtgtaag ttaagggttg ttttttttat 8400
tttgtttttt ttgaggattt gtgttaaggt tttttattt attaggttat tatgggttgc 8460
gtttataagg aatgtttttt gttttatttg ttttatagta aagttattga cgaggtggtg 8520
gttatgttta ttgagattgt agtgtaacga gatatagtta ttttgatata gtaaattttg 8580
tagggtgtat tagggttttt tttgtatgtt ttgggatttt tttattttgt tttataagta 8640
ggggttataa aatacgacgt tgaatttaaa ggttttggtt taaattgtaa tcgtttgttt 8700
tatgtaatcg aagtttatga ggtttagcgt tttttacgaa tgagggttat ttttatggtt 8760
atttcgagaa tttgttttac gttttgaatt cgcgcgtttt tttatagtgt ttggtatagt 8820
tatatatttt ttcggtagag attgaggatg tggtagatag tggagttggt tgtttttttt 8880
acggttgtgg aggggatgtt gtatatagta attcggagtt tattggtagt tttgatgttt 8940
acgttgtcgt agttatttgt ttatttatac gattattttt agggttttga atttttttag 9000
gttttttttt gtttagaatt ttttcgtgga ttttttgcgt ggattgtgcg ttatagaagg 9060
ttatagtggt taggtttttt aggatgggta tttttacggt gtaggttata gttatttagc 9120
ggcgttagta gcgggagggg gtgtaggggg ttgttcgtga tttgagggtg gatagtttta 9180

taagttttgt ttaatcgttg tttcgtgatt ttgtgtttat ttattagggt tatttttttat 9240
ggaattttgt aatttttaga ttaaaaggta aagtaggttt ggtatggtgt tttatgtttg 9300
taattttagt atttgggagg ttgaggtagg tagattattt gaggttagga gtttgagatt 9360
agtttggtta atatggggaa atattatttt tattaaaaat ataaaaaatta gttgggtatg 9420
gtggcgcgtt tttgtaattt tagttattcg ggaggttgag gtaggagaat tatttgaatt 9480
cgggagttag aggttgttgt gagttgagat tatgttattg tattttagtt tgagtggtag 9540
agtgagattt tgtttttaaa aattaaatta aaataaataa taataataaa ataaaaggta 9600
aagtagtttt ttaaaaattt acggaaattc gtaggagtgt gtgtgtatga cgttattatg 9660
aatttaatat atatttgttt ataaatttta tagtttatag gatgggttgt ttattttttt 9720
tttttttttt ttttttttt tgtcgttagg ttggagtgta gtggcgcgat tttagtttat 9780
tgtaattttt atttttcggg tttaaacgat tgttttgttt tagtttttttg agtagttggg 9840

attataggtg tatattatta tatttggtta ttttttgtat tttagtagag acggggtttt 9900
attatgttgg ataggatggt ttcgattttt tgaattcgtg atttatttgt tttggttttt 9960
taaagcgttg ggattatagg tgtgagttat tagttgttta ttttttttaag ggaatgtagt 10020
ttcgttagtt taaaaatatt taaggtgatg aaattttcgt tttgggtagt tagtatttat 10080
agcggaaagg ttttcgtttt ttttatttta tttggaggtt gtgttgtttt agtttagtgt 10140
agtatggttt ttggtttagt ttttcgtttt tcgggtcgtt tttaagttat tttaaa 10196

<210> 182
<211> 10196
<212> DNA
<213> Artificial Sequence

<220>
<223> chemically treated genomic DNA (Homo sapiens)

<400> 182

tttaaggtgg tttgggagcg gttcgaggag cgaaggattg aattaggagt tatgttgtat 60
tgaattgagg tagtatagtt tttaggtggg gtgggagggg cgggagtttt ttcgttgtgg 120
atgttgattg tttaggacga gggtttatt attttaaatg tttttgaatt gacgaagttg 180
tattttttta aaaagatgga tagttagtgg tttatatttg taattttagc gttttgggag 240
gttaaggtag gtggattacg agtttagaag atcgagatta ttttgtttaa tatggtgaaa 300

tttcgttttt attaaaatat aaaaaatagt taggtgtggt ggtgtgtatt tgtaatttta 360
gttatttagg aggttgaggt aggataatcg tttgaattcg ggaggtggag attgtagtga 420
gttgagatcg cgttattgta ttttagtttg gcgataaaaa gaaagaaaga aagaaagaaa 480
agatggatag tttattttgt gaattataga gtttgtggat agatatgtat tgggtttata 540
gtggcgttat gtatatatat ttttgcgagt tttcgtaagt ttttagagga ttgtttttgtt 600
ttttgttttg ttgttgttgt ttgttttggt ttggtttttg gagatagagt tttattttgt 660
tatttaggtt ggagtgtagt ggtatgattt tagtttatag taatttttga ttttcgggtt 720
taagtgattt ttttgtttta gtttttcgag tagttgggat tataggagcg cgttattatg 780
tttagttaat ttttatattt ttagtaaaga tggtgttttt ttatgttggt taggttggtt 840
ttaaattttt gattttaagt gatttgtttg ttttagtttt ttaagtgttg ggattatagg 900
tatgaggtat tatgttaggt ttgtttttgtt ttttgatttg agagttgtaa agttttataa 960
agaatggttt tggtggataa gtataaaatt acgagatagc gattggatag aatttgtgaa 1020
gttatttatt tttagattac gaatagtttt ttgtattttt tttcgttgtt ggcgtcgttg 1080
gatggttgtg atttgtatcg tggagatgtt tattttgaag gatttggtta ttgtggtttt 1140
ttgtgacgta tagtttacgt aggagattta cgagaagatt ttaaataagg gaggatttgg 1200
agaagtttaa ggttttgaga gtgatcgtgt ggataggtaa gtggttacga taacgtggat 1260
attaaggttg ttagtgagtt tcggattatt atgtgtaata ttttttttat agtcgtggaa 1320
gaggtagtta attttattat ttgttatatt tttaattttt gtcggaggaa tatgtggttg 1380
tattagatat tgtgggaagg cgcgcgggtt tagagcgtgg agtagatttt cgaggtggtt 1440
atgggagtgg tttttattcg tggaagacgt taggttttat gggtttcggt tgtatgaggt 1500
aggcggttgt agtttgagtt aaggtttttg gatttagcgt cgtattttat gatttttatt 1560
tgtaggataa gatagagagg ttttagggta tgtagagggg attttgatat attttgtagg 1620
gtttgttgta ttagagtgat tgtgtttcgt tgtattgtaa ttttaatgaa tataattatt 1680
atttcgttaa tgattttatt ataaagtaga tgagataggg agtatttttt gtgaacgtag 1740
tttatggtgg tttggtggat gagaaagttt tagtataggt ttttaaggag gataggatag 1800
gaggggtagt ttttggtttg tacgaatcgg agttttttag ttttgtttag atttcgttga 1860
aagatgcgtt aaattttatt tttatttttt atattgttta ggatagtatg taggcgttat 1920
tggagatgaa ggaggtagtt gttatcgaga ttcgtcgagt tattataggt cgcgttttag 1980
aaagtttaag aaattgtgtg aataaggagt tatttgttat attagcgttt tggttagtaa 2040
tagattagta agtaatttat ttcgagttta acggtgtaat atatagatat tcgttaggta 2100
tcgtgggcgt ggttttagga ggatttttttg tagttatgga aggaattatt tttggaggcg 2160
ttttaatgat ttataatttt ttgagagtga tatattttttt ttaagttttt tttttttaatt 2220
agtttataaa atatggggat aatcgatagt attttaataa gtaatagtag agaatgttta 2280
aaggtaatta tttagatata tttgggatta tgagttagtg aaaaatagat gaattaagag 2340
aaataaatta tggttttttt aattgatttt ggatataggt attattgatg ttgtcgtgtt 2400
aaaattataa gaattagata attgaagatg tcgtttttta tggaagtgtt gaaagattag 2460
gatgtgattt attaataatc gattttcgtt attgtgtgtt aagtttttta tttgtgtatt 2520
aaattttaaa taataaatgg agtttttttt tttatcggtt ttttgggtat aggaggtttc 2580

gaatattttt ttttaggatg ttgtattgag agtttaaata tcgggttatg cgtagtggtt 2640
tatgtttgta attttagtat ttgggagttc gaggcgggtg gattatttga ggtaaggagt 2700
ttgagattag tttggttaat atggtgaaat ttcgttttta ttaaaaatat aaaaattagt 2760
tgggtgtggt ggcgagcgtt tgtaatttta gttatttcgg aggttgaggt aggagaattg 2820
tttgaatata ggaggtggta gtaagttaag atcgtgttat tgtattttag tttgggcgaa 2880
agagtaagat tgttttaagg aaaaaagaaa aagagtttaa atattaaatt aaaaaatatt 2940
aagaggaaag ttttattttg tgattttagt ttcgtgattg tagttttttt attttatagg 3000
ggttgtttat ttttttttgt tagtagtaag attatataat tataaaatgt ggagaaattg 3060
tttgtttgtg gtagatattt gtatatatag gattgaagat agtataggtt tttgtgagat 3120
atttttgttt ttttatattt gttttttttg ttttgttttg ttttgagatg gagttttgtt 3180
ttgttattta ggttggagtg taatggcgag atttggattt attgtaattt ttgtttttta 3240
ggtttaagcg atttttttgt tttagttttc ggagtattta ggattataag tgtatattat 3300
tacgtttagt taatttttgt atttttggta gagattaggt tttattatgt tggttagatt 3360
ggtttcgaat ttttgatttt aggtgattcg ttcgtttttg ttttttaaag tgttgtgatt 3420
ataggagtga gttattgcgt tcggtttttt atttatattt gaattgtata ttgagtgggt 3480
aagttggtta taagtttagt aaaatttttt gatgatgtaa aaaaaagtat tatgttttat 3540
aagttgtttg tatttaaata tattttttta gtttagatt ttttttttatt ttattgagtg 3600
gaaagttttg aattaaaagt gtttaggaag aataatgttg tattttttta tgtttttttga 3660
aatatttttt tttttttttt ttttttgaga tggagttttg ttttgtcgtt cgggttggag 3720
tgtagtggta cgatttcggt ttattgtagg ttttgttttt taggtttacg ttattttttt 3780
gtttagtttt ttcgagtagt tgggattata ggtattcgtt attatgttcg gttaattttt 3840
tgtattttta gtagagatgg ggttttattg tgttagttag gatggtttcg atttttttgat 3900
ttcgtgattt attcgtttcg gttttttaaa gtgttgggat tataggcgta agttattgta 3960
ttcggtcgtt tcgaaatatt tttaatggta agttgttata ttgtttatga agaaatttat 4020
tttttttaaga tattaataaa tttttttttt ttttatgtga tattttatat aagaatattt 4080
tagatgtatt agatttgatt gatttaaat aaattttatt agatttgtat taattagtta 4140
tatgttttat ttatagattt ttgtgaatta ttgttttttt gtttaaaaag atggtttatt 4200
ttttaggtat agtggtttat gtttgtaatt ttagtatttt gatagatcga ggtaggtaga 4260
ttatttgaga tcgggagttc gagattagtt tgattaatat ggagaaattt cgtttttatt 4320
agaaatttaa aattagttac gcgtggtagt gtatgtttgt aattttagtt atttgggagg 4380
ttgaggtagg agaattgttt gaatttagga gatggaggtt gtggtgagtc gagattatgt 4440
tattgtattt tagtttgttt gggtaataag agcgaaattt taatttaaaa aaaaaaaaaa 4500
aaaagatggt ttattttaag tttttgtata gtatattttt gtttttgtga taaaagaaaa 4560
attttaaaat tgttttaaat agaaaaaataa tggttatatg ttttatttta ttttaatttt 4620
tttttttgaga tggagttttt gttttgttat ttaagtttga atgtagtggt atgattttgg 4680
ttggttgtaa tttttgtttt ttaagtttaa gtagtttttt tgttttggtt tttcgagtag 4740
ttggtattat aggtgtgtgt tattatgttt agttaatttt tgtattttta gtagagacgg 4800
ggttttatta tgttgtttag gttagtttta aatttttgat tttaggtgat tagtttattt 4860
tagttttata aagtgttggg attataggcg tgagttatta gtttggttta gttgatagtt 4920
ttaattatgt aaattattat attgattttt attgattatt tttattgtta gtatttttga 4980
atttaaattg gttttggaaa ttttttaatt atagtgaatt ttattttttta tgattttata 5040
aatattgggt attgttgcgg tatgttagaa tacgagttcg tggttgggaa aggtagtatt 5100
atatatgtag ttttttttatt tttatgtggt tatataagaa tgatatttga tttgtaatat 5160
tttttttatta agagatacgg attttatatg ggttgtgtaa tgtatttgtg ttttaagtta 5220
agcgatatta tagaaaagtt aaaaagtttt taaaaatttg aatatttata aagtagaaaa 5280
gttatagtaa gtaagggtta atttattatg gaagaaaaat tttaaatata tgtagtgttg 5340
tttaagtgta taatgtttat aaagtttaga gtgttgtatt gtaatgtttt agtttttata 5400
tttatttatt ttttatcgat tttttagagt aatttttagt tttgtagttt ttatttttgg 5460
taagtatttt atataagtgt attatttttt atttttttatg ttgttttttt tattgtattt 5520
tttgtgtgtt tagatatgtt tagatatata aatatttatt attgtgtttt gtgggtagta 5580
agttatttag gtgtcgaggt aagagatcga gggtatgggt tgttttagta taataaaata 5640
tataaaataa taagaattat attagatata gattatagat atgattatat ataagtatta 5700
ttaattatta gtttgtagta attatttttt atttaaatat tataataatt tttattttat 5760
aattataatt taggaaaaat taggttatat agaggtatga gttgaaggga tatggtgaga 5820
agtgattaga agataagtgt gtgagttttt gttatgttcg gatagggtta ttagagggtt 5880
ttttggttta gcggaacgtt agcgtttggg aagacgtttg ttattaagcg gattgtggtt 5940
tagtagtagc gttagtgtta aggaaaagta tttattattt agtagattag taaagggagt 6000
tttttttttt tcgggggagt ttagagaaga ttttatttta ttatgttttg tggagggttt 6060
gatatgagtt aggttcgttc gtagttattt ggaggtttaa tcgttttttt gtgatgttgt 6120
gttttagtgg ttatattatt ggttcgtttt tatgttttat tttgtatatt tggttttgtt 6180
ttttagatag tagtaataaa attagtgaaa gtattaaaag tttttgatat gtagaaataa 6240

```
tgcgtaagtt gttttttttt ttttttcgtt ttagttgtta aatagggaag ggttttttgt    6300
ttagtggata cgtgatttac gtgattttat ttattattgg agatggttta tattttttat    6360
tttgtttttt ttgttttgta tttaataaat aatagtgtag tttggtattc ggggttatta    6420
ttagtttttg tgttttggtg gtagtggttt attcgggttt aggtgttttt tattttttttg    6480
ttttgtgttt ttatttttat tatttttagt ttttgtatat agggagaaaa atttatagat    6540
tttgtagggt tggtttttat agtgttttaa ttgtatttag tatagtaata tattgtatag    6600
ttttgtagtt taggagtaat aggttatatt atgtagttta agtgtttagt tagttatatt    6660
atttaggttt gtgttagtat attttataat gatgaaattg tttaataatg tattttttag    6720
aatgtatttt tttttttttt gagatcgagt tttattttgt cgtttaggtt ggagtgtagt    6780
ggtgtaattt tggtttattg taatttttgt tttttgggtt taagcgattt ttttgtttta    6840
gtttttcgag tagttgggat tataggcgcg tattattatg ttcggttaat ttttgtattt    6900
ttagtataga cggggtttat attatgttgt ttaggttggt tttaaatttt ttagtttttt    6960
aaagtgttgg gattttaggc gtgaattatc gtattcggtt agaatatatt tttattattt    7020
atttatttat tttttttttaa ttgattattt ttgggtgttt tttgtagagg gggatttggt    7080
agggttatag gacgatagtg gagggaatgt tagtagataa ataagtgaat aaaggttttt    7140
ggttttttta ggtagaggat tttgcggttt tcggtagtgt ttgtgttttt gggtatttaa    7200
gattagggag tggtgatgat ttttaacgag tatgttgttt ttaagtattt gtttaataaa    7260
gtatattttg tatcgttttt aatttattta attttgagtg gatatagtat atgttttaga    7320
gagtataggg ttgggggtaa ggttatagat taataggatt ttaaggtaga agaatttttt    7380
ttagtataga ataaaatgaa aagattttta tatttacgtt tttttatata gatacggtaa    7440
ttattcgatt ttttaatttt ttttttattt ttttcgtttt tttattttat aatatcgtta    7500
ttgttattat ggttcgtttt taatgagttg ttgggtatat tttttagatg gggtcgtggt    7560
taggtagagg ggttttttat tttttagtag gggcggtcgg gtagaggcgt tttttatttt    7620
tcggacggag aggttggtcg ggcggggggt tgattttttt ttattttttt ttcggacggg    7680
gcggttggtc gggtagaggg gtttttttaat ttttagtagg ggtggttagg tagaggcgtt    7740
tttcgttttt cggacggggc ggttggtcgg gcgggggatt gattttttta tttttttttc    7800
gaacggggcg gttggtcggg cgggggggttg atttttttat tttttttttcg gacggggcgg    7860
ttggtttggc gggggtgatt tttatttttt tttcggatgg ggtggttgtc gggcggagac    7920
gtttttttatt ttttagacgg ggtggttgtc ggacggaggg gtttttttatt ttttagacgg    7980
ggcggttgtc gggcggaggg gtttttttatt ttttagacgg ggcggttgtt aggtagaggg    8040
ttttattttt tagacggggc ggtcgggtag agacgttttt tattttttag acggggtcgc    8100
ggttaggtag aggcgttttt tatattttag acggggcggc ggggtagagg cgttttttat    8160
atttagacg atgggcggtt tggtagagac gtttttttatt ttttagattg ggtagttagg    8220
tagaggggtt ttttatattt tagacgatgg gcggttaggt agagaagttt tttatttttt    8280
agatggggtg gcggcggggt agaggttgta atttcggttt tttggggggt taaagtaggc    8340
ggttgggagg aggttgtagc gagtcgagat tacgttattg tattttagtt tgggtattat    8400
tgagtattga gtgaataaga tttcgtttgt aatttcggta tttcgggagg tcgaggttgg    8460
cggattattc gcggttagga gttggagatt agttcggtta atatagcgaa attttgtgtt    8520
tggtggagat taaaaaaata cgaaaattag ttaggcgtgg cggcgcgcgt ttgtaatcgt    8580
aggtattcgg taggttgagg taggagaatt aggtagggag gttgtagtga gtcgagaggg    8640
tagtagtata gtttagtttt ggttcggtat tagagggaga tcgtggaaag agagggagag    8700
ggagatcgtg ggagagtat atatttttat tattaaatta tgtatgattg tgtatataat    8760
atttttatag gttaaaagta gttaaatatt ggttatgaga tatatttagt ttaagtatat    8820
aaataattgt agaaggagaa agtgaaattt tttttatttt ttgttttatt tatttattat    8880
aaagtatata gaagttagaa taatatttgt tttttgtaat tttagatttt tttttttttg    8940
ttttttttaaa tttgttttac gtttggcgat tttttttttt tcgatttttt tttattaggt    9000
cgtaggtttt tggtttatat tttttttgcg tttttaatta tttttgagtt tttataatttt    9060
tggttttata attttttttgt attaaggagt aataaataaa atttacgttc gaataaagag    9120
gttaaaatag taatttagga tgtttgcgtt tgcgtatttt gggcgtatag tatttaggtc    9180
ggtagtttag tatcggttta tttgtgagag taattaagat gattttcgga gggtttaatc    9240
gtttttttag tttcgaatta tatttttttag aaggtttagt tgtgcgatta ttttttttatg    9300
tagatttgtt ttttttttga ggttgtttag cgtttggggg tagtaggacg ggttcgggaa    9360
ttaagtttat tttaaatacg tcggagtggt tttttttggg ggttaaggtt cgagtttgag    9420
gcgggatttt atgaggtttt tggggcgcga gcggtttgat taggggggggc gatttttttcg    9480
gcggggttga cgtaaggcgc ggataggaat tttgacgatc gggaattatt ttgttcggtt    9540
ttgatacgtt tttgattacg ttttttagag gttttttcgg taagattgta tttttcgcgg    9600
taatttagtt tttaatttac gcgttggcgg gatttttagg gttttattag taattatttt    9660
agtgtcgggg agggttttgt tgtttcgaaa gttggtaagg tttgaggtga gaaattcggg    9720
agagggcggg gtcgggggtt aagtttcggt tttgtgcggc ggtggaggtg agattgaaag    9780
cgtgcgtttg cgtttgtgtg gaggtgtgtg gtgaatgatc gcgcgattgt gataggttgt    9840
ttttgtgatg agtgtgtttt taacgtttat atcgtgtgta gtacggtttc ggagattgtg    9900
```

```
gttgttggtg tgtgtgtgag agggagtagg attacgtgcg gtattgtgtg tgcggttgtg    9960
gggagtttgt gtttggttga gtgttattgc gtttgttttc gtagtggtgt gcgattatat   10020
gttgtgtgat tgtgtgcggt agagtttagg tggggatatt tgcgagtaat tgtatgtttt   10080
tatttgtgtg tgtggttgtg gttaatgtgt gatcgtgatt gtgattcggt ataggtgtgc   10140
gtcgattgtg ataatgtggt tgtgattgtg attgtttttt cggtaagcgt gtattt       10196
```

<210> 183
<211> 1231
<212> DNA
<213> Artificial Sequence

<220>
<223> chemically treated genomic DNA (Homo sapiens)

<400> 183

```
ttatttaatc ggtgatatag ttcggggttt cgtcgttttt tggcggttat tacggatttt      60
cgtttttttt atagaaggta gttattgtaa cgtgcgtggt tttagttgcg ttatattcgg     120
tttttgcgat tagggtttga ggaattcgcg ttatgaagtg cgtgtttgtt atcgtaggga     180
ttattagttt tgacgatttt attgcgtgtg tgtcggcgtt cgatagtttg taagtgagtg     240
agggaggcga gtaggcggcg gtttggttcg ttattcgtta ttttcggtta tattgttagt     300
cgcgttagtt ttgtttgatc gtcgcgttcg gaaagaaatt ttcgtaattt tattataggt     360
gtcgttgttt tttagttggt ttttgtttac gttcggcggg gttttttttc ggttattcgg     420
ttattcgggg ttgtttgttt ttttttttta ttatttcggt cgtttttttt ttttattttt     480
ttagtttttt tttcggtttg ttttcgcgtt ttatttttcg ttttcgcgtt tttcgttttt     540
ttttttaacg gtttcgtttt ttcgagttcg ggttttttat cgggttcggt agtttttttt     600
taggtttttt tgtggatcgt acgtcggcgt cggcgtttga gttagtcgag ttcgttttag     660
agttcggttt ttttttttta gtatttggtc ggagttcgcg cggttttttt ttttagcgcg     720
cgtcgttttt ttagggtttt cggttttcgt agttgattaa gtgaggtaga acgggtagtg     780
tttgggtttt tttcgagttt gcgatttttt ttttaaagtt tttttttgtt gtggttttcg     840
cgggattttg cgagttgggt cgtttagatt tatgagtgtt tgggaggttt ttcgtcgggt     900
ttcggattag aaaatagttt tgaaaattaa gtgaaatcgg ttatagatag gtttttggc      960
ggtcgtgttt cgatgttaac gttaaatttt agtagtggtg gtggttttg ttttgtttta     1020
tatagttagt ttaaaaggtt tttatatttt gatttttttt tttagaaaat cgagagtttt    1080
ggttataatc gatttatttt gtaaattggt agaggaacgg tggtatttga attttttagt    1140
attgagtcgt ttattttgga tgtttatagg tataaggatt ttttgaaaga agatatttag    1200
aaagtagatt ttgttattag ttacgtaggt a                                   1231
```

<210> 184
<211> 1231
<212> DNA
<213> Artificial Sequence

<220>
<223> chemically treated genomic DNA (Homo sapiens)

<400> 184

```
tatttgcgtg attaataata agatttgttt tttgaatgtt ttttttaag gaatttttgt       60
atttgtaaat atttagagta aacgatttag tattgaaggg tttaggtatt atcgtttttt     120
tattaatttg taggataagt cggttgtaat taaggttttc gattttttga aagaggaaat     180
tagaatgtga gggtttttta aattggttat gtaaaataga gtaggaatta ttattattat     240
taaagtttga cgttaatatc ggagtacggt cgttaaaaag tttatttgtg gtcgatttta     300
tttagttttt aaaattattt tttggttcgg ggttcgacga ggaatttttt aagtatttat     360
gaatttaagc gatttagttc gtagagtttc gcgaaagtta tagtaggaaa agattttgga     420
aaaggagtcg taaattcggg gggaatttag gtattattcg ttttgtttta tttgattaat     480
tgcgagaatc gggagttttg aggggacgac gcgcgttgag gagaggaatc gcgcgggttt     540
cggttaagtg ttgaggggaa ggagtcgggt tttgaggcga gttcggttgg tttagacgtc     600
ggcgtcgacg tgcggtttat agggggggttt gaggaaaaat tgtcgagttc ggtggaaaat    660
tcgagttcgg aggggcggag tcgttggaag gaggggcgga ggacgcggag gcggagaata     720
gagcgcgggg gtagatcgga aaaggagttg aagaaatgag ggagaggagc ggtcggaata     780
```

```
atgggaagag gaaataggta atttcgagtg gtcgggtagt cggagaaggg tttcgtcggg     840
cgtgggtaga agttagttaa ggggtagcgg tatttgtggt agagttgcgg gggttttttt     900
tcgagcgcga cggttaggta aggttaacgc ggttggtagt atggtcggag atggcgggtg     960
gcgagttaag tcgtcgtttg ttcgtttttt ttatttattt gtagattgtc gggcgtcgat    1020
atatacgtaa tgaggtcgtt aaagttggtg gtttttacgg taataaatac gtattttatg    1080
gcgcgggttt tttaagtttt gatcgtaagg gtcggatatg acgtaattga ggttacgtac    1140
gttgtagtga ttgttttttg tgagggaggc ggaaattcgt aatggtcgtt agggggcggc    1200
ggggtttcgg gttgtgttat cggttgggtg g                                   1231
```

<210> 185
<211> 16425
<212> DNA
<213> Artificial Sequence

<220>
<223> chemically treated genomic DNA (Homo sapiens)

<400> 185

```
ggtttttgtg ttttggtttt ttttggtgtt tttttttcgt ttgttttttt ttagtattac      60
gattcggtgt tttttattta gtttagtgtt gtttgttaga gtttcgggaa tttttttaagt    120
tttgtaaaag ttttttttgg ttaggttgta gttaatgttc gtagttagag ggtgggtggg     180
tagggttgat ggtggttttt tagtcgttag gtaagagatt tttagtcgga gggtgggtga     240
gtagggtcga tggtgttttt ttagtcgttg ggtgagagat ttttaggtgt ttaggttttt     300
tgggagggtg tggttggggt tttttgggtt tggcgttcgg ggagttggtt agtggttatt     360
tatttttgag ttttttagat ttgtgtttat tcggtatacg tgttgtgttt tagggagagt     420
cgttgcgttt tggggttttg gtgtttcggt tttgatttgg tttggtttta gttttgggtt     480
tggagttttt attttttttgg tattagtttt tgtagcgttt tgttttttgg tatttttcga    540
gagatgtatt ttaaaaatag gatcgattta ttgttggtga tgaggagagg gcggtttgag     600
cgtgaggggg tgggcgttag ttgtcggaga agtttttgtt aggtaggagg gacgagcggg     660
tttttagttt tgatttttcg gtttttgttt atgtttgaaa atgaaatcgg tggtttgggt     720
ggtcgttagt tttttgtggt cgatagaaga aagtattata gttgggtttt taaataatga     780
gaatttattt attgagtttt gggagttaga gttttgtagt cgaggggttc gtagggttac     840
gttttttttta ggtttttttag aggggttttt ttttgttttt tttagttttt gttagatata    900
ggtgtttttt ggttgtggtt ttagcgtttt agtttttgtt ttcgttttta tttaggtgat     960
ttttttttgtg tttgcgtttt ttgttttttt ttttataagg atatttgttg ttgcgtttag   1020
ataatttagg attttgaatt cgattatata tgtagaaatt ttgtttttaa ataaggatat    1080
ataggtttcg gggtcgggag tggatacgtt ttgttggggg ttacgtttag tttttgtttg    1140
taagtgtttt tggattttta ggcgttttta aggtgtagga tgtgagtttg tatttgtaga    1200
tagagtttag aaaagttaga taggtcgagg ggtggcgttc gtgaattaga aagaggtttg    1260
agggtcgagc gtgggtttag tatgcgtttt attattagtt ttgtaggggt agaggtttag    1320
ggtggggtcg gttggtttgt ggaaggtgtg agaagtcgtc gtggtaaatt taatttttgg    1380
ttaggagtcg gtttattgtg gtttttgggt taaattttgt cggaatttgt ttttgtaaat    1440
aaagttttat tggttcgtag ttatttttat acgtgttatt tgtggttgtt ttttagttaa    1500
gtatggtaaa ggagatttta agtttaatta agttaaaagg gtgacgtcgg gtttttttata   1560
ggtaagtttt tgattttagt tttgattgta ttattgggaa tgtaatttgt tgaataaagg    1620
aggtgttggt tgggggtaat ggaaggaatt ggttgttatt aggtgtcgga gttgtttgtt    1680
agggtgtggt taaggtgtta ttattataga gtagagttat aggggaggaa aggtttttgt    1740
ttgatttttg ggtgattagt ttattaaggt ttcgaggaag ttgaggtttt cgaggtggaa    1800
ggtggtggat ggttggttag tgtgtgtttt ttgttgagta ttgagggtga gttatgggtt    1860
atttggtttt tttgtgtatg ggtagttaga ttgtagggaa gacgttttgg ttttatagtt    1920
ggtaacgtag atagatgtgt atttgaatat gtaagtatgt attgagtgtt tattgtatat    1980
agggttttta tatttttttg tagatggttt ttacggtatt gttgtaggtg ggggtgttat    2040
tgtttttagg ttatagatga ggaaattgag gttaagattt taaggttata tagttgggag    2100
gttagagtag ggatttttag gggttttgtt ttttagggga agggagtgaa ggggtggttt    2160
gtatggtgcg tgattatttt tagtggaagt tttaatatgg gtgtggttta gtttttttagg   2220
gtttttttaag gtagaggtgg ttcgggtttt tgtagcgatt ttataatata ttttaattgt   2280
agacggtgga gattgcgggt tgtttttcgg tttaagcggt tttggggttt tcgttttgcg    2340
cggagttatt cgtgtttatg ttgtttttat atatcgttat tttttgggtg ttttggtgac    2400
gtatttgagg gatattgttg ttacggcgaa tttcgattac gtcgttgtgt ttgatttttt    2460
ttttggggtc gggcgttttg tttttttgttt tggagttttt agagggggtt aggagattgt   2520
```

ttggaaagtt ttagttgggt aggcggagtt ttgtaggtgc gggaatgagg gggtttttt 2580
tttaaattgg gaatgtttag ttttttttta gttgtaaacg ttttagacgt tgcggataga 2640
ttttttaatg ggaagtgaac gtattgtcgt cgcgtttata ttacgttttg tttttgtgaa 2700
ttaagttttt tggtatgtag ttgcgtttaa gggtttatat agggtttgag gttgttttta 2760
cgtagtaagg gtggcggtgt tgagtagtgg tcgtagagtt tgtgtggttt tgaaagttgt 2820
atgtttgttt tttggttttt tgtagaggaa gtttgttgat cgttggtttt tgtggttata 2880
tagatttttt ggttttgcgg gttgtggtta tgtggttcgg ggaggtgggt acggttgggg 2940
agggaagttg agtattatgt ttgttttgga ttttattgta aaaatgagtg tttgcgttaa 3000
atttatttgg gattttggtt tttacgtggt tttgtagggt ttgtgtgaag agtgagtatt 3060
tgttttttag agttttagat gatacgttat tttgtaattt ttttcgggga tattttttt 3120
aattttttttt tgtatgttta tagggatttg gggtgtttgt atgaggtttt aaaatttata 3180
ttttcgtcga agtcgggtat tttggagttt agttgtcggg gtttaggtgg gaggaggttt 3240
ggttattaag tttggagtta tgtcgggagg gtcgggagtt tttttgcgtc gtttttttttg 3300
tttttttagg tagatttgtt gttttcgtta tttataggag tgttttcggg ttaggtggtt 3360
tgtttaaggt tatataggga ttgtgttgtg ttaggtttga gttgtatttg gtggagtaga 3420

tagtattagt cggttgtttg tggtgatagt gacgttggaa aatgggaagg tgggggtgga 3480
gacggggatt cgagttgttt tggagggttc gggaggtaga acgtaggtag gtttttagta 3540
gtcgaggaag agattagggt ttgttgtgtt gggtagtggt cgggtggatg gggatgggat 3600
ttaaggttta tcgatttttt ttattttttg gggttgaagt ttagttataa tttgggaatt 3660
aaaggaatta ggaggggcgg tgggtttcga ggtagtaggg agtgggtata ggtgtggttg 3720
gtagggcggt aggataggag cggattggta gaattagggt aggaggttat agttatcggg 3780
taggttttac gttgggtttt taagttcgtg attatagatt ttgtagtttt tagtgtggtt 3840
taggggggttt tagggtttgg gatagggtaa attcgttagt gaaggtttag aatttagtta 3900
tgggagttag cgtcgggggt tgggtataga tatgaatatt ttgagtttat tttcgttttt 3960
gtagtttgtg ggttttgtaa ggggaggggga tcgttttattt ttgtatgtcg ggagtcgaag 4020
ttttggtatg gttgagtggt tggtttgagg ttatgatttt agttcgggtt tttgtattgt 4080
tacggttttt tcggggacgt taagggtgcg ggtaggcggt gttggttggc gtttttgacg 4140
gttacgtttt ttgtagattg tttgttgatg ttggtttata aggataagtc ggagcgtatt 4200
aagggtttgc gggagcgtag tagtttgacg ttagaggata tttgcgggtt ggagttcggt 4260
ttgttttacg agggtttggt ttatacgttg gttattgttt gtttgtttta ggttattatg 4320
ttgggttttg atagttacga ggttatgtgt gcgtgggatg ttcggattcg ttatgcgttc 4380
ggcgagggtg agtgacgggg gtcggggtcg ggcgggggtt tttcgtttag gtgtgtcggg 4440
gtttttttatt aacgtgggtt gtagaggtgg gagcggtttt agggaatcgt gtaggaagat 4500
gggatattta tgggtgtttt agggtggatt gagttttagt ttgggggtgt ttacgagggt 4560
ttgggagggg gagtttgttg ggttatggtg tttttagagt tttttagttt gatcgttagt 4620
tataattgga gtttttaatt taagttggtg gtttttggag ggacgtggtt ttttaaagtt 4680
ttgtttttttg tttttttatat tggatagtgg agtttagagt ttggttagga aggagagttt 4740
taggttgggc ggttttattt tagttttttt ttaaaatagg atgggaggga gggagggaat 4800
agagggaggg gagagggagg ggaagggagt gttttttttgt ggaattggtt ttggaagagg 4860
tggaacgtta gttttagttt tgtaggggtt tcgaatgggt gtttgggggt ttttgggggt 4920
tgagttgttt tggggttgtg agtagaggtg ggtggtgatg gggttttggg tatttagcgg 4980
tagagtaagt ataattttgt atttttaag aaattaaaat tagtgttaaa attttataat 5040
gagtaaaata gtagaatgtt tattgagttg agttttggtt ttattttggt atttatttaa 5100
taattttagt agttatagtt gttttttttt taaaaatttg ttattttagg ttgggtacgg 5160
tggtttatat ttgtaatttt agtatttcgg gaggttgagg tgggtggatt atttgagttt 5220
aggagataga gattagatta ttttgggtaa cgtggcgaaa tttcgttttt attaaaaata 5280
taaaaatgat ggttaggtgt agtggtttat gtttgtaaat tttgtgtttt gggaggttaa 5340
ggcgggtaga ttatttgagg ttaggatttc gagattagtt tggttaatat agcgaagttt 5400
tatttttatt aaaaatggaa aaattagttt ggtgtggtgg tgggtatatg taattttagt 5460
tatttaggag gttgagatag gagaatcgtt tgaatttggg aggtagaggt tgtaatgagt 5520
tgagatcgcg ttattgtatt ttagtttggg taatagagta ggattttatt ttaaaaaaaa 5580
aagagttggg aggtggaggt tgcggtgatt tgaggttgtg ttattgtttt ttagtttggg 5640
cgatagagtg agattttgtt ttaaaaaaat aaatataaaa aaatttgtta ttttgattag 5700
tatggatttt gtataatttt atttttttaaa atattgatta aaatattttg gtttttgatg 5760
gttgagtttt ttggttttgg aggtgagtgt ttcgtttttt tagttttgtt tttaaggtag 5820
tagtttttttt tgttttattg agtggggaga gagaagttgg gggtttagtg ggaggaggtg 5880
cggttggttt tgtaggaggg cgtaggggaa ggagatatta ggagggaaga attgtatttt 5940
tttagaaagt tttttcgttg ggggtttttt ttagtttggt ttagcggtat gggtttgttt 6000
tttttattcg ttggcgtagg gttatggtat agaggtcgtt gggtatcgtt taggtagttt 6060
tattttgata gaacgtggtt tgataaggat gcgtttgagg ttatagtttg gagtaggtag 6120

aggaggtaga gaagggggag gtgggatggg aataggggtt tttttggttt tgggggttgg 6180
agatggagta ggtggggttt ttggtgggga ggttgttgtt tgtttggtgt gttgatttgg 6240
gaagataggg gaggtgggga tagtatttag gattattgtt ttttttgagg tttttagggg 6300
taggggtttg ggtagagtta gtgtatagga ttatgggggt gtttagggtt tgggtgaatg 6360
gttatttgga tggagagagg gtcggtggtg ttttgaggtt ttcgtggtta ggttggggtt 6420
tcgatataga ttttagattt tttatattcg aattggaggt tgggggattt ttgagtttcg 6480
gttttttttt ttgtgatgga gttatatagt tgtggagaga gagtttttttt ggagaggtgt 6540
cgggggtggg agtttagtta gggtatttag ttgttttgtt ttttttagta ttttcgtggt 6600
gggaggggag ggaggtggag gttcgaggtt aggtttttttt gggatttttt tgtttttgaa 6660
taaagatagt ttttgggagg atggtaagga gagaagtgta gtttttaggt tttttcgtgg 6720
ttgtgaggag tgttatttgt ttattttgtc gtcgttgttt tttcggttga tatttgggcg 6780
gttgttttta tttgttttag tttggttttt gtttttgttg ttttttttcg agatgttttt 6840
ttattttatt tgtttatagt ggtttttgtt gtttttttttt atgatgtttt ttcgtttcgt 6900
ttgatcgtga tgtttttttcg tttcgtttat tttcgatgtt tttttatttt attcgtttat 6960
gatgtttttt cgtttcgttt gttcgtgatg ttttttttatt ttattcgttc gtgatgtttt 7020
tttattttat tcgttcgtga tgttttttta ttttgttcgt tcgtgatgtt tttttgtttc 7080
gtttgttcgt agtgtatagg ttttatgtga tagtggtttt aggtattaag ttggagagcg 7140
gttcggttat tttgtatttt tgtaatgatg ttttcgtttt ggttagggat attttttcgg 7200
ttgttacggg gtagtggaag ttgtttgatt ttcggcgtta cggggtcgtg ttaagcggat 7260
ttatttttga aggcgggatt aggtgtgggt attgtaagta cggatgtgtg gggttattgg 7320
gtagtagtag tattttttat ttttttgag aattgttggt ttcgggtcgg tcgattttat 7380
ttgtaggttg gtttgttggt atttttagaa tgcgtcggta ggtggacgga gtttttttac 7440
gttcgatgtt taaggttttt tgttcgtagg aattttagtg tggagtgtcg aggaggggtt 7500
tcggttattt ttgttttttt ttattggagg gagagagttt agggatgcga ggaggggttt 7560
gaggttatag agttttagag tcgtttatat ttagatttta tggaaaggaa aaaataaaga 7620
ataaacgtat ttagtagtgg ggagtgtgag ttttcgtata agtatcgtag aaatatattt 7680
ttggattaag aatttttggt ggatatgtta gttatttcgt ttgtgggtcg tttagagttg 7740
ttttaatgtt tttagtgagg tggttaggtc gtgggtgggg atggtttgag ttttttgggt 7800
ttgtgaaggt ttgagagttg tttttattta gttaggggag tgagggaaat cggagtagga 7860
aggtatttag ttttgtttag agagtttgag gtgttcgtcg gggttgtgta ggagtaggtt 7920
gttttagtaa ttttttttat ttttttttat gttgggtgta gatgcgttag gttgttttgt 7980
ggatttgtag ttttcgcgga gggtgtgacg tttttttttt ttttttgga ggggtagttg 8040
tttattggtc gggttgaagg cgggtagagg gatttttttgt gggtttggtt tggggttgag 8100
aggattgagg gattgtattt ggtttatatt ggtggggggtt ttggtggagt ttggatgtgt 8160
ttgggtggag gaaacgtagg taaggatacg ggttatattg gtcgtttggg attttatttt 8220
gtaaaggtta gttggggtac gggcgtaagt ttatttgggt gttacgtttt gttatttttt 8280
gcgttgggcg ttgggagtaa atttagtagg ttttgtggta ggagttagtt ttgttggggt 8340
ttttgaggag acgatatttg atttttgaaa ggggaatagg gtagtgaggt ttaggtttta 8400
cggattgggg gcgtatatag ttagttgtag gaggtacgtg cggtagagtt ggttcggagt 8460
ttcggttttt ggggttggta ggggttatag ttagagttta aagggttttg gtttttttgg 8520
gattggagag ttagtatgga tgggtagggt cgttgtttat gttagttgtg agttggtgcg 8580
gggggtgggg tggggggtgg ttttcgggag agttaggata tttgtcgggg ttttatttgt 8640
ttttaggttt tttttgcggt aggttagagg tttagtattg gggtttcggg agttattggg 8700
gagtgggttg gcggtggttg ggtatagtgg gtgggtttag cgtttttgtt ttttgtcgtg 8760
tgggaagtta gagttgtagg ttgtagttgt tttgatatag gtagtttgtt ttgtttgttg 8820
tgtgttaggt ttgggggtgt ttggaggagg gggatttata tttttttttt tgtttgtgga 8880
atttttttggt acgggtaggt agtcggtcgg ggtggcgtcg cgtgtttatt ttttaagtcg 8940
gggtatagta ggaggattcg tttttttgga gatttgttta ttttgtttgg gttataatgg 9000
aatggggata ttggatgttt ttttttttgag gtcgtggagg tttatatttt tgtcgaggtg 9060
tgggtggttt ttttttcgtt ggtaaataat tttatatttg gggttgtgtt ttttttttagg 9120
gcgaggttat tgtgtcgttt ttttgggttg tttaaaagtt gggggtattt ttgtggggtc 9180
gtttgttttg ttgttgtttt ttcggggtaa gttgttagag tagttttatt cgtaaatttg 9240
taaattaggt tggttttata gaatttgaaa atttgtaaat cgggttggtt ttatagaatt 9300
tataaatttg tagattgggt tggttttata gaattcgtaa acgggggttg gttttataga 9360
attcgttttt gtagtttagt gttgaatgtt ttcgttttcg gtggtatttt ggtttgagag 9420
aggaagggtt ttgggaattc ggttatcgac ggttgtatat ttcgtagggt aggtgggttt 9480
tggaggttag gttggaaggt ttggtcgagt tatttatttt tgtttttagt aggttttgtt 9540
gggttgtatt tttttggaga taggcgtggt tattattttg ggtggtttag gttgggtttt 9600
agattcggtt aagcggtggt gtttttaacg tagtattgag tgtaggtcgt ttggttggag 9660
ggacggggag tttgcggtac gtgtgggtag tttggggttt tttgcgggag ggagtcggta 9720
attcgggtgg tttaggggta gcggtattag tttttttgggg tttttgtagt agatgatttt 9780

```
taagagaggg ttaagtgttt agaacgggtt ttttttgtag tttggaggtt agaaatttta    9840
aattaagggg tttttagggt tggttttttt taaaagtttt tggggaggat ttatgggaag    9900
gttttttttg tttttttag tcgttggtgg tttcggcggt ttcggttcgt ggttgtgtta    9960
ttttagtttt tgcgtttgtt ggtttttgcg tgagtttttt ttttagtttt ttgtagtttg   10020
tggatttagg gtttatttgg atgatttagg gtgattttat ttcgagatta ttaattttat   10080
tcgtagaggt ttttgtttat aggttttggt atgtgaatat attttgtggg ttattatttg   10140
gtcgataatt ggggttattg ggacggagaa atatttagaa tttgaatttt aataagtttg   10200
tgggtgtcgt tatatttagt ttggaggtta tagttgggtt ggagtcgtgg gaaggttttg   10260
gaagtttttg ggtgtaggaa atttatattt tttttttttt tagggatagg ttttagtttg   10320
gttttcgatt tttagaatcg ttttatttgg tttcgttata gttagttttg tagagagtag   10380
agagggtgat tttggtttaa ggcgatggtt gcgttttatt tattttgaga tattgggcgt   10440
ggttgtgtat tacgggggtt ttggtggggg tcgggtggag ataggtttgg ttggttttgg   10500
tgtttggggg gtagaggttt ttttgtggat agttgtttgt ttagaatggg ataggaggtg   10560
gtttggtgat agtaattttc gggtttttag tgtggtttaa tttttcgttt ttgttggtta   10620
cgggtttgta gtgtggattt ttggagtttg gttcgagcgt atttggtata tggtaggcga   10680
cggtttgtgg atagtttagg tttgtaggtt ttagttttat atacgggtaa gtgattgggt   10740
ttcgttttcg gggatggatt gtgcgtggtg gagagtgatg tagagagggt tattcgtttt   10800
gggataggtt tagtttgtag tatagacggt ggtgggtttt agagagggtg agtttattta   10860
gagtttagga agggagggtt ttggaggcgt agggggacgt ttgagttggg ttttgagggt   10920
gaatggaagt ttttggaggt gttttttttt ttttttttta tttgtttttt ttagttgggt   10980
ttagtttaag gattatattt gtttttttgt ttagttttg ggggtcgttc ggagtatttg   11040
aaatttattt tgatttttaa aaatgattaa gaatgtggtt aggtatagtg gtttatgttt   11100
gtaattttag tattttggga ggcggaggtg ggaggattat ttgaggttag gagttcgaga   11160
ttagtttggt taatatggtg aaattttatt tttattaaaa atataaaaat taggtaggtg   11220
tggtagtagg tgtttgtaat tttagttatt ttagaggttg aggtgggaaa attatttgaa   11280
tttgggagtt ggaggttgta gtgagttgag atttcgttat tgtagtttag ttaaggtaat   11340
agagttaggt tttgttttaa aaaaagaaga gtaaagaatg aaaatttgaa ttaggaggtt   11400
tttgttatat gaacgcggta tttgtttagt tttggagagt tttttgtcgg ttatttttgg   11460
ttattgtagt ttgcgggggt agtttggata gagttggttg gaattttggt ttttgttagg   11520
tagtagggag cgtttttagt agttttttat tgtcgggcgt tgggtttagg gattagacgg   11580
ttcggtttta gaggttgcgt gagattgagt gaggtatttt tagtttttag agggtgcgag   11640
gggagttagg aggggtatag atttaggtt gggggtattg cggggaagtt ttttggaggg   11700
gcgagtttta ggggattttt gtagggtgaa taggagttag tttagttgtt agagggtaag   11760
gggtggaggg gggtgaaaag ggtattttag atatttttga gtacgaatgg gtgtttcgaa   11820
gggttgtgta ggagttttgt tggggggat aggaggcgtg agggtggagg aggaggagga   11880
gatagggttg ggttgtagag agttggttaa ttggattagt gtttgtttgt attattgttt   11940
cggagtagta gggagttatt gaaggtgttt gagtatgggg agtgttttat atgttaatgt   12000
tgatttcggg gtgtagatga gtttggagga agaggttggt aggggttaga gaaggttttg   12060
attgaggttt agaagatgga gggcgaggtt gttttaggta gtagttgttt ggttggggga   12120
tgggaatttt gtggattgta ggaatgttaa taaggtggtg atggataagg ggaggagggt   12180
cggttcggga agggggggtt ttttagagtg gtgtttttgg attggggttg ttttaggagg   12240
gtagaggagg ggtgggagtt aatatttatt gttaggtttt aggttttttt ttattcgaga   12300
tttattaggt ggggttattt ttaagtgtat aagaagttgt tattttgggg gatggagagg   12360
gttgagggtt atgttattaa gattttttag ttttgtttat gtttatttgg tatgagttat   12420
ttaggatatt ttggttattt cgatgtttgt tttttgagga gttgggatta tgagtattta   12480
agattttgat attaaggggt agttacgagg gtatttaaga atatttagat ggaaattatt   12540
ttagatttaa ttggtttatt aaatgtttgc gtggaggagt attttttttt ttaagtcggt   12600
gagttttgga gttagtgtaa tagttttttt attggagacg tgttttttag gaagtaggtg   12660
cgtttgtaac gtgggtgagg tttgtttgtc gtttagtaat tttagttttt gtttagagtt   12720
ggcgtcgttt tgagtttcgt agtttttggt tttttgtttg tgttttgttg attttgtttt   12780
gttttttgc gtttttatg tttttttttt ttatttttac gtttttttatt ttttataata   12840
gggtttttat atagtttttt gggatattcg tttgtgttta aagatgtttg acgtgttttt   12900
tttattttt tttatcgttt attttttaat agttgagtta atttttattt attttgttat   12960
atttagcggt tggcggttgt tttttggttg gtaattttgt gggttaggga tttggacggg   13020
gtttagtgag gatagtttat ttttgtttta tgaggtgttg tttggggttt ttcgggtagt   13080
ggtatttggt tatggtgggg tttaaaggtt taggaaagta ttgtgtcggt tgttggttgg   13140
gttgttttag tttttttttta ggaattttat ttttcgatag gatattttgg gttttttaat   13200
aggttttaag agagtagaag ttgttaggtt tttgacggtt ttggggtagg gttgggagag   13260
tattttttat gttattttat gttagagtta ttattggttg gtttcgattt tagggaggga   13320
gtggattagt ttttggagaa ggagcggttt ttgagggtag ggatagggga aatagagtgg   13380
tcggttttgt tgtagttata tttgtgtgat atagtaaaat ggggtaggtt ttgttttttt   13440
```

```
tttttttttt aaatatatag gtaaatagga aatatgcgta ttttgttttg gggaaggggt   13500
agggggggcgg ttagtagttt tttatttatg tttttttatt taaggtcgag tattggggt   13560
tttttggtta tgggcgtttg ggtggatatt gagattatag acggtttgtg ttttttgtcg   13620
gtaaggtgtt tagtttggta gtcgggacgg tagtaggtgg ggttgaattt tagaggaggc   13680
gttcgtgaga gtattataga ggggttgtcg gggttagggg attgaagggg gttagaggtt   13740
aagaaaagta ttatgaaagg tgtcgttatg tggtttgagt ttggaaaggt ttggggtgtt   13800
ttaggtggtt aggggtgtgg agggtggggg tggtagtttt ttgagtggag atgtaggggt   13860
gtggagggtg gggacggtag ttttttgagt ggagatgtag ggtgtggag ggtggggacg   13920
gtagtttttt gagtggagat gtaggggtgt ggagggtggg gacggtagtt ttttgagtgg   13980
agatgtaggg gtgtggaggg tggggacggt agtttttga gtggagatgg taggtagatt   14040
tggtgtgtag agggagtttt gagtattttt aagatcgggg ggggttgggt gggagcgggg   14100
gtggagtatg aatttaggga ggtttagcga gagtttagtc gtgcggtgtt ttgggaggtt   14160
atggtttaag attttggtag attagtattg gagatgggta tttttttta gggttattga   14220
tgttagtttt gttggtatgg atgatggggg tggttggcga gggtgtcgcg tgggtacgtg   14280
ggagttcgag gttggcggtg ggttgtgttt taggttttgt tcggttgatc gtgttttcgg   14340
ggtgagtgac gtttcgtaga cgtcgtatgg aatgcggggt cggttttatt tttttcgtaa   14400
tgtggttttt tatagtcggg ggatttattg tttcgtggtt attgttagtt gtttgtaagt   14460
ttaggattag agagtttggt ttttcgtgag agttggggtt gtgtcggttg agttttttat   14520
agggaggggt ggattacggg gatagggagc gattgtttgg tattgagtga gttagttagt   14580
agatagtatg acggtttttt ttttgtttgt ttttagggtt tggtgtaggt tttttttttgt   14640
ttattttatt tagtttgggg gttttatttt attacggttt ttatgagttt ttcggggtat   14700
tttttttggat ggtatcgtag cgggtattgg tttttttttt atgtttattt ttaattttga   14760
ttaggagtat tttttattta tagtaggatt ggaagtttta tcgggttaga aggtgtatag   14820
ggggttaggg gatttggatt tgagtttatt tcgtggttgt gtgatggtgg ataggtggcg   14880
tgttttttttt gggttttagt gtttttttttg ttaaagtagg aatggcgttg ttttatgatt   14940
cggtttgggg atattacgtg gaaggaagta tttatagtat aaggtgtcgt tcgttttcgt   15000
gtgttcgggg tggttgttaa ggggtcggta gcgtcgttac gtgtcggggt tggtttgagt   15060
ttcgtgtttt tggaatttat agtttgtgtt tcgtgaaggt agagtatgat tgggtagtag   15120
gtagcggggg ttttaatgtc gagatttttt tttttttttaa tttttgttta tttcggcgtt   15180
tttttttttg cgtatttttt tttgggtaat gggtagaggt ttcggagcgg gcgggatttt   15240
tgtgaggtga cgttagtcgg agtgtttagt cgggtgtagt tagtttagtg gtttatttac   15300
gcggtcgtta gggttttatt tatttttggt cgggagggta gcgttttcgt gattcgtagt   15360
tggggttttt ttacgggtgg cggttgtatc gttttgttag tgacgttggg ttggtttttg   15420
ggggtttttt tgttgttgtg ggggtgtagg ggtggatgtt ggttttagga tgttgtatac   15480
ggttttaggg tagtatttat atattggtag ttttagtttt ttttgaaaga tgatcgagga   15540
ggtggggagg acgcggtgtt ggttagtagt gacggttgta gtatgtgtgg gtaggtgtcg   15600
tgttatattt tttataaata tttgaatgta gttttttttaa tagttcgggg gaaggaagtg   15660
tggttatatg gtagtttttt tgggatgtga ttttagtttt aggtggtata gtttttttttt   15720
tttgtggttt tggagttttg aggggatttt atttgatgtt tttggggttt tttggttgtt   15780
ttatttgttt ttaagacggt tgtgggggta gggtcgttgg gattttgggg tagaggaggg   15840
attgattaag tgtttgttgg gtggtagagt agttatattt ttttattttt ttaagtaagt   15900
tcggaaggta ggtatgtggg cgggggtatt tatttgttta gggtagggta tttttttttt   15960
tttttttatg ttttgtcggt tgtaggattt aggatgggcg gggtagtatt tattttagcg   16020
tttaagtttg aagggtgcgg cggggttttt ggggagttta gaggttttgg ggatttagtt   16080
ttatagagtt aggtggggtg tcggttttgg gtattcggat ttacggggtt aggcggggtg   16140
tcggttttgg gtatttgatt tcgtggggtt aggtagggtg ttattgtcgg tttttggtgg   16200
agtttgttgc ggttttttgt gttttttttt tttagggttt ttagttcgtt ttcgggttag   16260
attgatttgt ttttgttttt tttttgtagg ggttggcgtt ttttttttgt tttcggtcga   16320
gggggagtag attagttttt tgttcgattg tatcgttcga ggtatttttt ttattaaggg   16380
ttttttttggg ttgcggtcgg ttttattagg tgcgtgtggg agttt                  16425
```

<210> 186
<211> 16425
<212> DNA
<213> Artificial Sequence

<220>
<223> chemically treated genomic DNA (Homo sapiens)

<400> 186

```
aggtttttat acgtatttgg tagaatcggt cgtagtttaa aggggttttt ggtgggggag      60
atgtttcgga cgatgtagtc gaataggaag ttgatttgtt ttttttcggt cgaggatagg     120
aagaagacgt tagtttttgt agaggaagga tagagatagg ttagtttggt tcgagggcgg     180
gttggggatt ttgaagagga ggaatatagg aaatcgtagt aaattttatt aagagtcgat     240
aatgatattt tgtttggttt tacgaagtta gatgtttaga gtcgatattt cgtttggttt     300
cgtgaattcg ggtgtttaga gtcgatattt tatttggttt tgtgaagttg ggtttttaga     360
gtttttggat tttttaggga tttcgtcgta ttttttaaat ttgggcgttg gggtgagtgt     420
tgtttcgttt attttaggtt ttgtagtcga tagggtatgg ggaggaggga gggggtgttt     480
tgttttggat agatgggtgt tttcgtttat atatttgttt ttcgggtttg tttgggggga     540
tggggggatg tggttgtttt gttatttagt aggtatttga ttagtttttt ttttgtttta     600
aggttttagc gatttttgttt ttatagtcgt tttgagggta aatgaagtag ttaggagatt     660
ttagagatat taagtagggt tttttttaggg ttttaaggtt ataggagaag ggagttgtgt     720
tatttggagt tggggttata ttttaggaag gttgttatgt gattatattt ttttttttcg     780
ggttgttggg agggttgtat ttagatgttt gtaaaaggtg tggtacggta tttgtttata     840
tatgttgtag tcgttattgt tggttagtat cgcgtttttt ttattttttc ggttattttt     900
taggggaagt tggagttgtt agtgtgtggg tgttgttttg aggtcgtgtg tagtattttg     960
gggttagtat ttatttttgt atttttatag tagtaggagg gtttttaggg attagtttag    1020
cgttattggt agagcgatgt agtcgttatt cgtggagaag ttttagttgc gagttacggg    1080
ggcgttgttt tttcggttag gggtgaatga agttttggcg gtcgcgtgaa tgggttattg    1140
ggttggttgt attcggttgg atatttcggt tggcgttatt ttataggaat ttcgttcgtt    1200
tcggggtttt tgtttattgt ttaaagaaag gtacgtagga gggaaggcgt cggggtgggt    1260
aggagttggg gggaagggaa atttcggtat tagaattttc gttgtttgtt gtttagttat    1320
gttttgtttt tacggaatat aggttgtaga ttttagggat acggaattta ggttagtttc    1380
gatacgtggc gacgttgtcg gttttttggt agttatttcg ggtatacgag ggcgagcggt    1440
attttgtgtt gtggatgttt tttttttacgt agtgttttta aatcgggtta tgggatagcg    1500
ttattttttat tttagtaaaa aggatattga ggtttagaga aggtacgtta tttgtttatt    1560
attatatagt tacgaggtgg atttaagttt aagtttttttg gtttttttgtg tattttttgg    1620
ttcgatagag tttttagttt tattgtgggt gaggggtgtt tttgattaga attggaggtg    1680
ggtatggagg ggaggttaat gttcgttgcg atgttattta ggaggatgtt tcgagaggtt    1740
tatgagggtc gtgatggggt ggggttttta agttgggtag ggtgggtaga agagagtttg    1800
tattaggttt tgggggtagg taaggagaag gtcgttatgt tgtttgttgg ttggtttatt    1860
tagtgttagg tagtcgtttt ttgttttcgt ggtttatttt tttttgtgaa gggtttagtc    1920
ggtatagttt tagtttttac gaaaggttag gttttttaat tttgagttta tagatagttg    1980
gtagtggtta cgggataatg agtttttcga ttataaaaaa ttatattacg gaaggagtgg    2040
agtcggtttc gtattttatg cggcgtttgc ggagcgttat ttatttcgaa gatacggtta    2100
atcgggtaag gtttggagta tagtttatcg ttagtttcgg gtttttacgt gtttacgcga    2160
tattttcgtt agttattttt attatttatg ttagtagagt tgatattagt ggttttggag    2220
gaagatgttt atttttaatg ttggtttgtt aaggttttgg gttatggttt tttaggatat    2280
cgtacgatta ggttttcgtt gggttttttt gggtttatgt tttattttcg tttttattta    2340
gttttttttcg gttttgggaa tgtttaaagt tttttttgta tattaggttt gtttgttatt    2400
tttatttagg aggttgtcgt ttttattttt tatattttta tatttttatt taggaggttg    2460
tcgtttttat tttttatatt tttatatttt tatttaggag gttgtcgttt ttattttttta    2520
tatttttata tttttattta ggaggttgtc gtttttattt tttatatttt tatattttta    2580
tttaggaggt tgttattttt atttttttata tttttggtta tttagaatat tttagatttt    2640
tttagattta ggttatataa cgatattttt tatgatgttt tttttggttt ttggtttttt    2700
ttagtttttt gatttcgata gttttttttgt ggtgttttta cggacgtttt ttttggggtt    2760
tagttttatt tgttgtcgtt tcggttgtta ggttgagtat tttgtcggta gggagtataa    2820
gtcgtttgtg gttttagtgt ttatttaagc gtttataatt aggagatttt tagtgttcgg    2880
ttttgggtgg aaagatatag gtgagaggtt gttgatcgtt tttttgtttt ttttttaaga    2940
tagggtgcgt atgtttttttg tttgtttgtg tgtttgggga aagggggggag aatagggttt    3000
gttttatttt gttatattat ataggtgtga ttgtagtaaa gtcggttatt ttgttttttt    3060
tatttttgtt tttagaggtc gttttttttt tagaggttgg tttatttttt ttttggaatc    3120
gaagttagtt agtgatggtt ttggtataaa gtggtatgag ggatgttttt ttagttttgt    3180
tttaagatcg ttagaggttt ggtagttttt gttttttttgg aatttattga gaagtttaag    3240
gtgttttatc ggagggtgag gtttttggag ggaaattgag gtaatttagt taatagtcgg    3300
tatagtgttt ttttgggttt ttgggtttta ttatagttaa atgttattgt tcgagggatt    3360
ttaggtagta ttttatgggg tagagatgag ttgtttttat tgagtttcgt ttagattttt    3420
ggtttataga attgttagtt agggaatagt cgttagtcgt taggtgtggt agggtaaata    3480
ggagttagtt tagttgttag agggtaagcg gtggaggggg gtgaaaaggg tacgttagat    3540
atttttgagt ataaacgggt gttttaaagg gttgtatagg agttttgttg tggggggatag    3600
gaggcgtgag ggtaagagga aagagtatgg aagacgtaga gaagtaggat agagttagta    3660
```

```
gaatataagt agaaggttag gagttgcggg gtttagggcg gcgttaattt tggatagagg    3720
ttggggttgt tgagcggtag gtaaatttta tttacgttat agacgtattt gttttttgag    3780
aaatacgttt ttagtgaaaa agttgttgta ttaattttag gatttatcgg tttgagaaag    3840
aaaatgtttt tttacgtaga tatttaatga gttaattaga tttaagatgg tttttatttg    3900
aatattttg agtgttttcg tggttgtttt ttgatgttag aattttaagt gtttataatt    3960
ttagtttttt agagaataaa tatcggggtg attagggtgt tttggatggt ttatgttagg    4020
taggtatggg taggattgag aagttttgat aatatgattt ttagtttttt ttatttttta    4080
aggtggtagt tttttgtata tttgggagtg gttttattta gtgggtttcg ggtggaggaa    4140
ggtttggagt ttggtagtgg gtgttggttt ttattttttt tttgtttttt tgaagtagtt    4200
ttagtttagg ggtattattt tagaaggttt tttttttcg ggtcggtttt ttttttttg    4260
tttattatta ttttgttaat atttttgtaa tttatagggt ttttattttt taattaggta    4320
gttgttgttt ggggtagttt cgtttttat tttttggatt ttaattagaa tttttttgg    4380
tttttgttag ttttttttt taagtttatt tgtatttcgg ggttaatatt agtatataaa    4440
atatttttta tgtttaagta tttttaatgg tttttgttg tttcgagata atggtgtaaa    4500
taaatattgg tttaattagt tagtttttta taatttagtt ttgttttttt ttttttttt    4560
atttttacgt tttttatttt ttttaatagg gtttttatat agtttttcgg gatatttatt    4620
cgtgtttaaa gatgtttgaa gtgttttttt tattttttttt tattttttat tttttgatag    4680
ttgagttaat ttttatttat tttgtagaag tttttgagg ttcgtttttt tagagagttt    4740
tttcgtagtg tttttagttt ggggtttgtg tttttttgg tttttttcgt atttttgag    4800
gattgaaaat gttttattta gttttacgta gtttttgagg tcggatcgtt tgatttttag    4860
gtttagcgtt cggtaatgag aaattgttga aggcgttttt tgttgtttga tagaggttag    4920
ggttttagtt agttttgttt aggttgtttt cgtaggttgt agtggttaaa ggtggtcggt    4980
agggggtttt ttagagttgg gtagatgtcg cgtttatgta gtaagagttt tttagtttag    5040
gtttttattt tttatttttt tttttttgag atagagtttg gttttgttgt tttggttgga    5100
ttgtaatggc gggattttag tttattgtaa ttttaattt ttaggtttaa gtgatttttt    5160
tattttagtt tttggagtag ttgggattat aggtatttgt tattatattt gtttaatttt    5220
tgtattttta gtagagatgg ggttttatta tgttgattag gttgatttcg aatttttgat    5280
tttaggtgat ttttttattt tcgtttttta aagtgttggg attatagata tgagttattg    5340
tgttttggtta tatttttaat tatttttaaa agttaaaatg agttttaaat gtttcgggcg    5400
gtttttagag gttgggtaaa gaggtagata tggtttttgg attggattta gttggagaga    5460
gtaggtaagg ggggaaaaag ggggtatttt taaaagtttt tatttatttt taaggtttag    5520
tttaaacgtt tttttgcgtt tttaaagttt tttttttta ggttttgggt gggtttattt    5580
tttttggggt ttattatcgt ttgtgttgta agttgagttt gttttagggc gagtggtttt    5640
ttttgtatta ttttttatta cgtatagttt attttcgaag gcggggttta gttatttgtt    5700
cgtgtgtgag gttggagttt gtagatttga gttgtttata gatcgtcgtt tgttatgtgt    5760
taagtgcgtt cgggttaagt tttaggagtt tatattatag gttcgtgatt aatagagacg    5820
gggaattggg ttatattggg gattcggggg ttgttgttat taagttattt tttgttttat    5880
tttgaatagg tagttgttta tagagagatt tttgtttttt aggtattaag attagttagg    5940
tttgttttta ttcgattttt attagggttt tcgtgatgta tagttacgtt tagtgtttta    6000
ggatgagtga ggcgtagtta tcgttttggg ttagggttat tttttttgtt ttttgtagag    6060
ttggttgtgg cggggttaag tgaggcggtt ttggggatcg ggggttaggt taggatttgt    6120
ttttggggaa ggagggaatg tggatttttt atatttaaga atttttaggg tttttttacg    6180
attttagttt agttgtgatt tttagattga atgtgacgat atttatagat ttattgggat    6240
ttagattttg gatatttttt cgttttagtg gttttagttg tcggttaaat ggtggtttat    6300
aaaatgtgtt tatatattag ggtttgtgaa taggagtttt tgcggatgga attgatggtt    6360
tcgggatggg gttattttgg gttatttagg tgggttttaa gtttatagat tgtaagagat    6420
tgaagaggag gtttacgtag gagttagtag gcgtagggat tggagtggta tagttacgag    6480
tcgaggtcgt cggggttatt agcggttgga aggggtagga aggatttttt tatgggtttt    6540
ttttaagagt ttttggaaaa agttaatttt gaggattttt tgatttgggg tttttggttt    6600
ttaggttgta gaaggaattc gttttggata tttggttttt ttttgggggt tatttgttat    6660
agaagtttta ggaagttgat gtcgttgttt ttggattatt cgagttgtcg gttttttttc    6720
gtaggaggtt ttaggttgtt tatacgtgtc gtagattttt cgtttttttta gttagacggt    6780
ttgtatttag tgttgcgttg agaatattat cgtttggtcg agtttaagat ttagtttaaa    6840
ttatttaggg tggtggttac gtttgttttt aggaggatat agtttagtag ggtttgttgg    6900
ggatagggat gggtggttcg gttaagtttt ttagtttggt ttttagaatt tatttatttt    6960
acggggtgtg tagtcgtcgg tgatcgaatt tttagagttt tttttttttt aggttagggt    7020
attatcgggg acgagaatat ttagtattga gttgtaaagg cgggttttgt ggggttagtt    7080
ttcgtttgcg ggttttgtgg ggttagttta gtttgtaggt ttgtgggttt tgtggggtta    7140
gttcggtttg taggttttta ggttttgtgg ggttagtttg gtttgtaggt ttgcgggtgg    7200
ggttgtttta atagtttgtt tcggagaggt agtagtaaga tagacggttt tataagagtg    7260
```

```
tttttagttt ttgagtagtt taggaaaacg gtatagtagt ttcgttttgg ggagaagtat    7320
agttttaggt gtgggattgt ttattagcgg aaaaggggtt atttatattt cggtagggat    7380
gtgaattttt acggttttag aggaaaaata tttagtgttt ttattttatt atggtttaga    7440
taaaataagt aggtttttaa ggaaacgggt ttttttgttg tgtttcggtt tagagggtga    7500
gtacgcggcg ttatttcggt cggttgtttg ttcgtgttaa ggaattttat agatagaaag    7560
gaaggtatgg gttttttttt tttaggtatt tttaaattta gtatataata gataggatag    7620
gttgtttgtg ttaggataat tatagtttgt agttttggtt ttttatacga taaaggatag    7680
ggacgttggg tttatttatt gtgtttagtt atcgttaatt tattttttag tggttttcga    7740
agttttagta ttgaattttt ggtttgtcgt agggagggtt tgggggtaga tgggatttcg    7800
ataggtgttt tagttttttc gggaattatt ttttatttta tttttcgtat tagtttatag    7860
ttggtatagg tagcggtttt gtttatttat attggttttt taattttaga ggggttaagg    7920
ttttttggat tttggttatg gtttttgtta gttttagggg tcgagatttc gggttagttt    7980
tgtcgtacgt gtttttttgta gttggttgtg tacgtttttta gttcgtggag tttgagtttt    8040
attgttttgt tttttttttta agggttagat gtcgtttttt taaaggtttt aatagggttg    8100
gtttttgtta taagatttat tgggtttgtt tttagcgttt agcgtagaag gtggtagaac    8160
gtggtattta gatgggtttg cgttcgtgtt ttagttggtt tttgtaggat gaggttttag    8220
gcggttagtg tggttcgtgt ttttgtttgc gtttttttta tttagatata tttaggtttt    8280
attagagttt ttattagtgt gagttaagtg tagttttttta gtttttttag ttttaaatta    8340
ggtttatagg aggtttttttt gttcgttttt agttcggtta atgagtagtt gtttttttag    8400
agagaagaga ggaggcgtta tatttttcgc ggggattgta ggtttatagg gtaatttggc    8460
gtatttgtat ttaatatggg agaaagtgga gggggttgtt ggaataattt gtttttgtat    8520
agtttcggcg ggtattttag atttttgggg tagggttggg tgtttttttg tttcgatttt    8580
ttttattttt ttggttgagt gaggatagtt tttaggtttt tataggttta gaggatttag    8640
gttattttta tttacggttt ggttatttta ttggggatat tagggtagtt ttgggcggtt    8700
tataggcggg gtggttggta tgtttattag gaatttttgg tttaagaata tgtttttgcg    8760
gtgtttatgc gggagtttat atttttttatt gttaaatgcg tttgtttttt gttttttttt    8820
ttttatgaaa tttggatatg agcggttttg aggttttgtg gttttaggtt ttttttcgta    8880
tttttgaatt tttttttttt agtgaggaag agtagaggta gtcgaggttt tttttcgata    8940
ttttatattg aggtttttgc gggtagagga ttttggatat cgagcgtggg gagatttcgt    9000
ttatttgtcg gcgtattttg gaaatgttag taggttagtt tgtaagtggg gtcggtcggt    9060
tcgaagttag tagtttttag gggaagtggg gggtgttgtt gttgtttagt gattttatat    9120
attcgtattt atagtattta tatttggttt cgtttttaaa gatgaattcg tttggtacgg    9180
tttcgtagcg tcggaggtta gatagttttt attgtttcgt gatagtcggg gggatgtttt    9240
tggttaagac gaggatatta ttgtagaggt gtagggtagt cgggtcgttt tttaatttgg    9300
tgtttggagt tattgttata tggaatttat gtattgcggg taggcggggt aagagggtat    9360
tacgggcggg tagggtgaga ggatattacg ggcgggtggg gtgagagggt attacgggcg    9420
ggtggggtga gagggtatta cgggtaggcg gggcgagagg gtattatggg cgggtgaggt    9480
gagagggtat cgagggtggg cggggcgaga gggtattacg gttaggcggg gcgagagggt    9540
attatgagag gaggtaatag gggttattgt ggataggtgg ggtgagaggg tatttcggga    9600
ggaggtagta ggggtagggg ttaggttggg gtaggtgggg gtagtcgttt aggtgttagt    9660
cggggaggta gcgacggtag gatgagtaaa tggtattttt tatagttacg agggggtttg    9720
ggggttgtat tttttttttt gttatttttt taggaattat ttttgtttag gggtaaggaa    9780
gttttagagg ggtttgattt cggattttta tttttttttt tttttattac ggaggtgtta    9840
gggaggatag ggtagttgag tgttttggtt gggtttttat tttcggtatt tttttaggaa    9900
ggtttttttt ttatagttgt gtggttttat tatagaaggg gaaatcgagg tttagggatt    9960
ttttagtttt tagttcgggt gtggagagtt tggagtttgt atcgaggttt tagtttggtt    10020
acgggaattt tagggtatta tcggtttttt ttttatttag gtggttattt atttaaattt    10080
tgagtatttt tatggttttg tgtattggtt ttgtttaggt ttttgttttt gaaagtttta    10140
gagaggatag tggttttgaa tgttgttttt atttttttttg ttttttttagg ttagtatatt    10200
aggtagatag tagtttttttt attaggggtt ttatttgttt tatttttaat ttttagagtt    10260
aaagggattt ttgtttttat tttatttttt tttttttttgt tttttttatt tgttttaagt    10320
tgtggtttta ggcgtatttt tgttaggtta cgttttatta agatgaagtt gtttgggcgg    10380
tgtttagcgg tttttgtgtt atggttttgc gttagcggat gaaggaagta gatttatgtc    10440
gttgagttag gttggaggga atttttaacg ggagaatttt ttggaaagat gtagtttttt    10500
ttttttaatg tttttttttt ttgcgttttt ttgtagagtt aatcgtattt ttttttatta    10560
agtttttagt tttttttttt ttatttaatg gagtaggagg ggttgttgtt ttggggataa    10620
gattggggga gcgggatatt tatttttaag gttaaagaat ttagttatta agggttagga    10680
tattttaatt aatattttaa aaagtaaagt tatgtagagt ttatgttgat taaaatagta    10740
agtttttttg tgtttgtttt tttgagatag ggttttattt tgtcgtttag gttagagggt    10800
agtggtataa ttttaggtta tcgtaatttt tattttttag tttttttttt tttgagatgg    10860
agttttattt tgttgtttag gttggagtgt aatggcgcga ttttagttta ttgtaatttt    10920
```

```
tgttttttag gtttaagcga tttttttgtt ttagtttttt gagtagttgg gattatatgt   10980
gtttattatt atattaggtt aattttttta tttttagtag agatggggtt tcgttatgtt   11040
ggttaggttg gtttcgaagt tttgatttta ggtgatttgt tcgttttggt tttttaaagt   11100
ataaggttta taggtatgag ttattgtatt tggttattat ttttgtattt ttagtagaga   11160
cgaggtttcg ttacgttgtt tagggtggtt tggtttttat tttttgggtt taggtgattt   11220
atttatttta gtttttcgaa gtattggaat tataggtatg agttatcgtg tttagtttaa   11280
aatagtaagt ttttaaaggg agggtagttg tggttgttgg gattattggg tgggtgttag   11340
ggtaggatta ggatttaatt tagtggatat tttattgttt tgtttattat ggggttttgg   11400
tattgatttt ggttttttag gagatgtagg attatgtttg ttttgtcgtt gagtgtttag   11460
ggttttatta ttatttattt ttgtttatag ttttagggta gtttagtttt tagaggtttt   11520
taggtattta ttcgaagttt ttgtagggtt gggattgacg ttttattttt tttagggtta   11580
gttttatagg ggagtatttt tttttttttt tttttttttt ttttattttt tttttttttt   11640
tttattttgt tttgggggga agttggggtg ggatcgttta gtttggggtt ttttttttta   11700
gttaggtttt gggttttatt gtttagtgta ggaagtaaag ggtagggttt tgggaagtta   11760
cgttttttta aaggttatta gtttgggtta aaggttttag ttgtaattaa cggttaggtt   11820
agggagtttt aaagatatta tagtttagta gatttttttt tttaaatttt cgtgggtatt   11880
tttaggttgg agtttagttt attttaaggt atttatgaat gttttatttt tttgtacggt   11940
tttttggagt cgtttttatt tttgtagttt acgttggtga ggggtttcgg tatatttgaa   12000
cggggagttt tcgttcggtt tcggttttcg ttatttattt tcgtcgagcg tatagcggat   12060
tcgggtattt tacgtatata tggtttcgtg gttgttaaag tttagtatga tggtttggga   12120
taggtagata atggttagcg tgtggattaa gttttcgtag ggtaggtcgg gttttagttc   12180
gtagatgttt tttagcgtta ggttgttgcg ttttcgtagg tttttgatac gtttcgattt   12240
gtttttgtag attagtatta gtaggtagtt tgtaaggagc gtggtcgtta gggacgttag   12300
ttaatatcgt ttgttcgtat ttttggcgtt ttcggggagg tcgtgatagt gtagagattc   12360
gggttggggt tatgatttta agttagttat ttagttatgt taaggtttcg attttcggta   12420
tataaaatgg ggcgattttt ttttttgta agatttatag gttgtaagag cggaaatgaa   12480
tttagaatat ttatgtttgt gtttagtttt cgacgttggt ttttatgatt gggttttgga   12540
tttttattgg cggatttgtt ttgttttagg ttttggagtt ttttgggtta tattgagggt   12600
tgtaggattt gtggttacgg gtttgggggt ttagcgtggg gtttgttcgg tggttgtggt   12660
tttttgtttt ggttttgtta gttcgttttt attttgtcgt tttgttagtt atatttgtgt   12720
ttattttttg ttgtttcgag atttatcgtt ttttttggtt tttttagttt ttaggttgtg   12780
gttgagtttt agttttaggg ggtaggggag gtcgatgggt tttgggtttt atttttattt   12840
attcggttat tgtttagtat agtaggtttt agttttttt tcggttgttg gggatttgtt   12900
tgcgttttgt ttttcggatt ttttagggta attcgggttt tcgtttttat ttttattttt   12960
ttattttttta acgttattgt tattataggt aatcggttgg tgttgtttat tttattaggt   13020
gtagtttaaa tttggtatag tatagttttt gtgtggtttt gggtaaatta tttggttcgg   13080
ggatattttt gtgaatggcg ggggtagtag gtttgtttga gagagtagag aggacggcgt   13140
aggaagattt tcggtttttt cggtatggtt ttaggtttga tgattaggtt ttttttttatt   13200
taggtttcgg tagttgggtt ttaggatatt cggtttcggc gagggtgtag gttttgggat   13260
tttatataaa tattttaaat ttttgtgagt atgtaggagg ggattgggga gaatgttttc   13320
gaagaggatt gtagaatgac gtgttatttg aagttttgaa aagtaggtgt ttattttttta   13380
tataggtttt gtaaagttac gtgagaatta aaattttaaa tgaatttgac gtagatattt   13440
atttttgtaa tggagtttag agtagatatg gtgtttagtt ttttttttta gtcgtgttta   13500
ttttttcggg ttatatggtt atagttcgta gagttagggg gtttgtgtga ttatagggg t   13560
tagcggttag taagtttttt ttgtaagggg ttagagggta agtatgtagt ttttagggtt   13620
atataggttt tgcggttatt atttagtatc gttatttttg ttgcgtgaaa gtagttttag   13680
attttgtgta gatttttggg cgtagttgta tgttaggaag tttgatttat aaaagtaagg   13740
cgtggtgtgg gcgcggcgat agtgcgttta tttttttattg gaggatttgt tcgtagcgtt   13800
tgaaacgttt atagttagaa aaaagttagg tatttttaat ttgagaaaga agttttttta   13860
ttttcgtatt tatagaattt cgtttgttta gttgaggttt tttagataat tttttggttt   13920
tttttggaaa ttttaaggta ggaagtagga cgttcggttt taagagagaa attaaatata   13980
gcggcgtaat cggaattcgt cgtaatagta atgtttttta aatgcgttat tagagtattt   14040
aagaaatgac ggtgtgtggg aatagtatga atacgggtgg tttcgcgtag gacgggggtt   14100
ttagggtcgt ttgagtcgga ggatagttcg tagtttttat cgtttgtagt tgaagtgtgt   14160
tgtgagatcg ttgtaggggt tcgggttatt tttgttttgg gggattttaa aaagttgagt   14220
tatatttatg ttgaaatttt tattgaggat aattacgtat tatatagatt atttttttat   14280
tttttttttt taaggggtaa ggtttttggg aatttttgtt ttgatttttt agttgtatgg   14340
ttttgggatt ttggttttag tttttttatt tgtgatttgg ggatagtggt atttttattt   14400
gtagtaatgt cgtaaggatt atttgtagga aggtgtggag gttttgtgtg tagtaagtat   14460
ttaatatatg tttgtatatt taggtatata tttgtttgcg ttattagtta taaggttaga   14520
gcgttttttt tgtaatttgg ttgtttatgt atagagggat taggtgattt atggtttatt   14580
```

tttagtgttt agtaaggggt atatattggt taattattta ttattttta tttcgagggt 14640
tttagttttt tcggagtttt ggtgggttga ttatttagaa gttaggtagg gatttttttt 14700
tttttgtggt tttgttttgt ggtggtggta ttttggttat attttggtag atagtttcgg 14760
tatttgataa taattagttt tttttattgt ttttaattag tatttttttt gtttaataag 14820
ttatattttt aataatatag ttaggattgg ggttaggagt ttgtttgtaa agagttcgac 14880
gttatttttt tggtttgatt gagtttaaag ttttttttat tatatttagt tggaaagtag 14940
ttatagatga tacgtgtggg aatggttgcg agttaataaa attttattta taaaaatagg 15000
tttcggtagg atttggttta gagattatag tgagtcgatt tttggttaag agttgaattt 15060
gttacgacgg ttttttatat tttttataag ttagtcggtt ttattttaga tttttatttt 15120
tgtaaggttg gtggtggggc gtatgttgaa tttacgttcg atttttagat ttttttttga 15180
tttacggacg ttatttttcg gtttgtttga tttttttgga ttttgtttat aaatgtagat 15240
ttatattttg tattttggga acgtttgaaa atttaggagt atttataggt aggggttgag 15300
cgtggttttt agtaagacgt gtttattttc gatttcggaa tttgtatgtt tttatttggg 15360
aatagggttt ttgtatatgt aatcgagttt aggattttgg attgtttgaa cgtagtagta 15420
agtgtttttg taagggggag aataggaggc gtagatatag ggaaggttat ttgaatgggg 15480
acggaggtag ggattggagc gttgaggtta tagttaaggg atatttgtgt ttaataggag 15540
ttgggagagg taggaaggag tttttttggg aagtttggag ggagcgtggt tttacgggtt 15600
tttcgattgt agagttttaa tttttaggat ttagtgggtg aatttttatt gtttgaaggt 15660
ttagttgtgg tgtttttttt tgtcggttat aggaaattga cgattattta aattatcgat 15720
tttattttta aatatgaata gaagtcgaga ggttagagtt gggggttcgt tcgttttttt 15780
tgtttggtag aagtttttc ggtagttgac gtttattttt ttacgtttag gtcgtttttt 15840
ttttattatt aataataaat cgattttgtt tttaaagtgt atttttcggg gagtgttagg 15900
gggtaaagcg ttgtaggaat tggtattaga gagatggaag ttttaggttt agggttgaga 15960
ttaagttagg ttaaggtcgg gatattagag ttttaggacg tagcggtttt ttttgggata 16020
tagtacgtgt gtcgggtaga tataggtttg ggggatttag gagtggatgg ttattggtta 16080
gtttttcggg cgttaggttt agagggtttt agttatattt ttttaggagg tttgggtatt 16140
tgggagtttt ttatttagcg gttgaggaag tattatcggt tttgtttatt tatttttcgg 16200
ttgggggttt tttgtttggc ggttgaggaa ttattattag ttttgtttat ttattttttg 16260
gttgcgggta ttggttgtaa tttggttagg gaaggttttt gtagaatttg gagggttttc 16320
ggggttttgg taggtagtat tgggttgggt ggagggtatc gggtcgtgat gttgaaagga 16380
gataaacgag gaagaggtat taggagaggt taaggtatag agatt 16425

<210> 187
<211> 2031
<212> DNA
<213> Artificial Sequence

<220>
<223> chemically treated genomic DNA (Homo sapiens)

<400> 187

ttttatcgga cgttattgta tatgcgtttt gtttgtagtt gatgatgtag ttatagggta 60
agtgggttta gcgtttggat aagataaata ttggggttat agtggtggtt agtagggtaa 120
gtagaaagtt gaatgttttt aagggaaggt tttgaaattt tacgacgttt tggattatta 180
tgtgtatttg taagggtaag agaattttag atttaattta aaattatatt ttgtattttt 240
taagtttttt ttttacgtac gttgttttag tttttttttt agtttttttt tttgatggat 300
agcggggatt tttgttacgg agttggtttt ttgtttttgg tgatgggttt ttgggaacgg 360
cggggtatat ttttagggtt gggaggggtt ttagtttttg agtgtcggat ttgcgttcgt 420
tgtttattta agattttatt agaaaggtat agtttataac gtgggggacg ttagttagga 480
aataggcgtt cgtgaaattc gtatttgatt tttcgaatgg tttttgtcgt ttttttggat 540
ttttattttc gtttatgcgc gttcgttata gtggggcgtt attttgttat tttttaatcg 600
gttttttttt ggaaggagga ataaaagttt gttttgtttt ttgttttttt tttttataat 660
tgattttttt ttttttttat ttcgttttgt tggagatggg cgtcgttttt tttaggcggg 720
aatggaacgg cgagaaaggg atgatagaag gttgtgggga aaaaattttt taattttttt 780
tttttttttt tgaatatttt ttgattttgt tgtgtgagat aatatttttg gttattatcg 840
atatggaaaa ttaagtcgtt cgcgacggaa agcgattttt atgtttttga tttttgaaag 900
gagaagtatt tagtagtaaa ggtttcgtag ttaattagtt ttgtttcgaa ttgttttttt 960
ttaagtgtat gtttaaaatt tagcgagata tatttggaag cggattttat tgtgtgtttt 1020
ttggtgattt tttaaatcgg cgacgtcggt tttaatattt atttatattg tttggtatat 1080
atattcgcgt tttgtgagta attgtatttt tattattatg ggtagttaaa ggacgatttt 1140

tgtaagcgtt aggattaaag cgaagcgttt ttgcgttacg tttacggtga agtttttggt 1200
gttatagaga gtttttcggt ttttacgttt gtaggtttta gttttagcgg tagcgggcgt 1260
attcggttcg aatacggtgt cggagttcgg agagtatttt ttagagcgtc gtagattcgg 1320
gtttggagta ttttttgggg atagggaaat tattttttttc gtagtaggag gggttttagg 1380
aattgaagtt ttacgggaaa ttttttggta gttggagtgg agtttcggcg gtttttttcga 1440
atatagtaat cggtgagtga gtgggattga gaggtttacg aggagttcga aggttaaagc 1500
gtatacgata gagaggaata gtacgtagga gttggagtag tttattaggt agttttaggg 1560
cgtgcgtacg aaggcgtttt agtcgtatag cgggagcgtc gagagtagta gattggttgt 1620
ttatacggtt agtattacgt cgagtatttg gttcgatttt ttggaggttg tttggttttt 1680
tatatttttg tgtatcgtat aaaagttgta agagattagg agagtcgttt ttaagttgtt 1740
agagaggttt tggtataaat atattaaggt agaggtggtg tatagaaatt ggaagtaatc 1800
ggggatttcg tttggttatt gtaaaaatat gaagatggtt atcgatagga cgtttatgag 1860
attatttata gattaggaag ttataagtat ggatataata gttttgtttt gtatttttag 1920
tagggaaatt agtgaataaa tgttgtttat taaggttgta aaagttatga gatatgttaa 1980
gtaaaaaaga tagatattta gggtttttgg cggatttaaa aggttcgtag a 2031

<210> 188
<211> 2031
<212> DNA
<213> Artificial Sequence

<220>
<223> chemically treated genomic DNA (Homo sapiens)

<400> 188

tttacggatt ttttaaattc gttaggaatt ttgaatattt atttttttttg tttgatatgt 60
tttatgattt ttgtagtttt ggtgggtagt atttatttat taatttttttt gttgaaaatg 120
tagaatagaa ttgttgtgtt tatgtttgtg gtttttttggt ttgtggatga ttttatgagc 180
gttttgtcgg tgattatttt tatgtttttg tagtggttaa acgaggtttt cggttatttt 240
taatttttgt gtattatttt tgttttaatg tatttatgtt agggtttttt tagtaatttg 300
aaggcgattt ttttagtttt ttataatttt tatacgatgt atagaggtgt ggggagttag 360
atagttttta gaagatcggg ttaggtgttc ggcgtggtgt tgatcgtgtg ggtagttagt 420
ttgttgtttt cggcgttttc gttgtgcggt tggggcgttt tcgtgcgtac gttttggggt 480
tgtttggtgg attgttttag tttttacgta ttattttttt ttatcgtgta cgttttggtt 540
ttcggatttt tcgtgggttt tttagtttta tttatttatc gattgttgtg ttcggaggag 600
tcgtcgagat tttattttaa ttattaggaa atttttcgtg gagttttaat ttttgggatt 660
ttttttattg cggggagagt ggttttttttg tttttagagg atgttttagg ttcgagtttg 720
cggcgttttg ggggatgttt ttcgagtttc gatatcgtgt tcggatcggg tgcgttcgtt 780
gtcgttgggg ttgaagtttg taggcgtgag aatcggggga ttttttatgg tattaggagt 840
tttatcgtga gcgtagcgta gaagcgtttc gttttgattt tagcgtttat aaaagtcgtt 900
ttttggttgt ttatgatggt aaaagtgtag ttgtttataa agcgcgagtg tgtgtgttag 960
atagtgtaaa tgagtgttgg gatcggcgtc gtcggtttgg ggagttatta agagatatat 1020
agtgaagttc gtttttaagt gtatttcgtt gggttttaga tatgtattta gaagaaggta 1080
gttcgggata gggttagttg gttgcgaaat ttttattatt aaatgttttt tttttttaaaa 1140
gttaggggta tggggatcgt ttttcgtcgc gagcgattta gttttttata tcggtggtgg 1200
ttaaggatat tgttttatat agtaaaatta gagagtattt agaaaaggaa agagggaatt 1260
aaaaggtttt tttttttataa tttttttgtta tttttttttc gtcgttttat tttcgtttag 1320
agaggacgac gtttattttt agtagggcgg gatagagggg aggagagatt agttgtggag 1380
ggagaagata gggaataaaa taggtttttg tttttttttt taggaggaag tcgattaaga 1440
ggtggtagag tggcgtttta ttgtgacgag cgcgtatggg cggaggtgag ggtttaggga 1500
ggcgataaag gttattcgga gaattagatg cgggttttac ggacgtttgt tttttagtta 1560
gcgtttttta cgttgtagat tgtgtttttt tggtggagtt ttgaataggt agcgggcgta 1620
gattcggtat ttaaaagttg aaattttttt taattttggg ggtgtgtttc gtcgttttta 1680
ggagtttatt attaaggata gggagttagt ttcgtagtag aggttttcgt tgtttattag 1740
gagaaggaat tggaaaggga attgaggtag cgtgcgtgga gaaaaggttt gagaagtata 1800
aagtataatt ttaggttagg tttgaagttt ttttgttttt gtagatgtat atggtggttt 1860
agaacgtcgt ggggttttag agtttttttt tggagatatt tagtttttta tttattttgt 1920
tggttattat tgtaatttta gtgtttgttt tgtttaaacg ttggatttat ttgttttgtg 1980
gttgtattat taattgtagg tagaacgtat atgtagtggc gttcgatggg a 2031

<210> 189
<211> 2237
<212> DNA
<213> Artificial Sequence

<220>
<223> chemically treated genomic DNA (Homo sapiens)

<400> 189

```
gtttgtgata gacgtttgta ggtatatggt aaggaaaata gattaaaata ttaagaaata      60
gtaagaggtc ggatcgaggg ttttttattt gtatgggaaa ttttatttcg tttattattt     120
ttagaaataa aaaggaaaac gattttaaag atataggtta aaggtcgaag gggtgtagaa     180
ttatttttcg tttttttttt tttatttttt ttaaatattt tagaacgagt taaatttttc     240
gaatttcgta gtatagataa taagtaattt agtaagattg gacggggatg attgagggtt     300
aatttattat aggattatag gaaatttttt agttttaaat tcgaatgtat tcgtgattat     360
ggtaatattt tttatttgat taaaagttat taaacgtttt gataagtgtt tatagttgtt     420
ttttcgatta ttgaagtggt tttgaaaaga gtttttgggg ttaggtggtt gatttattgc     480
gttttttgta tatttttatt tcgagttggt gtatttggtg atcgttttgg tgttttcgga     540
tacggcgtgt ttggttagtt cgtcgggtag tagtaggcgt acggtcgttt ggatttcgcg     600
ggatgtgatg gtggagcgtt tgttgtagtg cgttaggcgg gacgtttttt tcgcgatgcg     660
ttcgaagatg tcgttgacga aggagtttat gatgtttatg gttttggacg agatgtcggt     720
gtcggggtgg atttgtttta gtattttgta tacgtaaacg gagtagtttt ttttgcggtt     780
gcgtttgcgt tttttgtcgt tttttttttg tattttcgta atagtttttt tggagttttt     840
tttgggagta ggagcggatt tcgttggatt cggtattttt gcgcgaaaaa agagaaaaga     900
gatttaaaga agtaattcga attatcgtaa aacgggttgg ttatgcgtta tttatagagt     960
tcgtatgtaa atgaagattc gtaaagtgtt gcgtttttat tggttaattt ataacggtgt    1020
cgttagaggg ggtggagttt atgtaaataa tagattttag ttttgttttt ttaatggtta    1080
ttgtaataaa agttttgtta ttttattaga atggtttatt ttatattaat tagttttaag    1140
ttaggtgaag cgatagtttt tacggtttgg ggtttttttt tggtttttat tttttattag    1200
atttgtatac ggaagaaatt ttaacgttag ttagataagt taattttttt gtttcgtttt    1260
tgtattttta ttttatattt tgtggaaatt tttttttttt ttttgaaata ttattttatt    1320
tatttttaaa aatcgttaga tatttatttt cgttcgttta aattattatt ttggtttgga    1380
tatcgtttgt agtatgtaat gaggaagatg ttgagtttta attaagttta tagtttttaa    1440
ataaattagg aaacgggttt ataattttta agggattcgt tttattttta tatttaggat    1500
tttttatatt tagaaatttt ttatagtttt ttgtttttat gtaaaatttt atttatagaa    1560
ttgggagtcg gatttttagt tttaaattag cgtgaaaacg ttcggtcgga attataagga    1620
ttatgttttt gcggtttttt ttattgtcgg ttaatttacg gttgggtttt gggttttgta    1680
acgttattta ttacgagtgt ttttggtttt tgatcgttgc gtaaggtaga cgtatattag    1740
atggagagtc ggtatagttg tttttgatga aaacggtagt ggttttgaaa agagttttta    1800
gatcgattat ttaaaaatat gttttaggtt cgtttttcgc ggatgcggcg ggttaattgg    1860
atgtttttgg gtatgatggt tacgcgtttg gtatggatgg cgtataggtt cgtgttttcg    1920
aatagtttta ttaggtaggt ttcgttggtt ttttgtagcg ttattacggt cgagttttgg    1980
aagcgtaggt tcgttttaaa gttttgcgcg atttcgcgta ttagtcgttg gaagggtagt    2040
ttgcggatta gtagtttcgt agatttttgg tagcgtcgga tttttcgtag agttacggtg    2100
tcgggtcggt agcggtgcgg ttttttttatt tcgttcgtgg tcggcgcgtt tttgcgggtc    2160
gttttggtag ttagttgttt tttcggggtt ttgtcgtcgg tcgatttgcg ggtagtttgt    2220
ttagtacggg ttatgtt                                                   2237
```

<210> 190
<211> 2237
<212> DNA
<213> Artificial Sequence

<220>
<223> chemically treated genomic DNA (Homo sapiens)

<400> 190

```
agtatggttc gtattaagta gattgttcgt aagtcgatcg gcggtaaggt ttcgaggaag      60
tagttggtta ttaaagcggt tcgtaagagc gcgtcggtta cgggcggggt gaagaagtcg     120
```

```
tatcgttatc ggttcggtat cgtggttttg cgggagattc ggcgttatta gaagtttacg     180
gagttgttga ttcgtaagtt gtttttttag cggttggtac gcgagatcgc gtaggatttt     240
aagacggatt tgcgttttta gagttcggtc gtgatggcgt tgtaggaggt tagcgaggtt     300
tatttggtgg ggttgttcga agatacgaat ttgtgcgtta tttatgttaa gcgcgtgatt     360
attatgttta aggatattta gttggttcgt cgtattcgcg gggagcgggt ttaaggtata     420
tttttaagtg gtcgatttaa aggttttttt tagagttatt gtcgttttta ttaagagtag     480
ttgtatcggt tttttatttg atgtgcgttt gttttgcgta gcggttaggg gttaggggta     540
ttcgtggtgg gtgacgttat agaatttaaa gtttagtcgt gagttggtcg gtagtagaga     600
aagtcgtaga ggtatggttt ttgtggtttc ggtcgagcgt ttttacgttg gtttaaggtt     660
agaggttcgg tttttaattt tgtgggtaaa gttttgtatg ggggtaagag attatgggga     720
gtttttagat gtggaagatt ttgagtgtaa gggtggggcg agttttttaa aaattataaa     780
ttcgtttttt ggtttattta ggaattataa gtttaattaa agtttagtat ttttttttatt    840
gtatattata ggcggtgttt aagttagaat agtaatttaa gcgggcggga gtaaatattt     900
ggcgattttt aaaaatgggt gaggtagtat tttaaggaag agaaaaaaat ttttataaag     960
tatagaataa aaatgtaaaa acgaggtaag agagttaatt tgtttagttg gcgttaagat    1020
tttttttcgta tgtaagttta atggaaagtg aaaattaaga aaaaatttta agtcgtagaa   1080
attatcgttt tatttagttt aaaattggtt agtgtgaaat ggattatttt gataggataa    1140
taagattttt gttatagtga ttattaagga agtaagatta gaatttatta tttatataga    1200
ttttattttt tttgacgata tcgttgtaag ttaattaatg aaagcgtagt attttgcgag    1260
tttttatttg tatacgggtt ttataagtag cgtataatta gttcgttttg cggtagttcg    1320
gattattttt ttaagttttt tttttttttt ttcgcgtaaa aatgtcggat ttagcgaaat    1380
tcgtttttgt ttttaagaag ggttttaaaa aggttgttac gaaagtgtag aagaaggacg    1440
gtaagaagcg taagcgtagt cgtaaggaga gttatttcgt ttacgtgtat aaggtgttga    1500
agtaggttta tttcgatatc ggtatttcgt ttaaggttat gggtattatg aattttttcg    1560
ttaacgatat tttcgagcgt atcgcgggag aggcgtttcg tttggcgtat tataataagc    1620
gttttattat tatatttcgc gagatttaga cggtcgtgcg tttgttgttg ttcggcgagt    1680
tggttaagta cgtcgtgttc gagggtatta aggcggttat taagtatatt agttcgaagt    1740
aagagtgtgt aagggacgta atagattaat tatttaattt taaaggtttt ttttagagtt    1800
attttagtaa tcgagaaagt agttgtaaat atttgttaga gcgtttgata gtttttggtt    1860
aggtagggag tgttattatg gttacggatg tattcgggtt taggattaag gagttttttg    1920
tagttttgta atgagttggt ttttagttat tttcgtttag ttttattgag ttgtttgtta    1980
tttgtgttac gagattcgaa aggtttgatt cgttttggga tatttgggag ggatggaggg    2040
aaggaggcgg ggggtggttt tatatttttt cggtttttaa tttgtatttt taaagtcgtt    2100
tttttttttg tttttgaaag taatgaacga gatgagattt tttatataga tgagaaattt    2160
tcggttcgat tttttattat tttttagtgt tttaatttgt ttttttttatt atatatttat   2220
aggcgtttgt tataaat                                                    2237
```

<210> 191
<211> 4182
<212> DNA
<213> Artificial Sequence

<220>
<223> chemically treated genomic DNA (Homo sapiens)

<400> 191

```
agatttttttg aacgtttgat gaaatggatt tttgtgaaaa ttaatagtga atatttattg    60
ttgtattgta cgaagttttt gaaatgtaat taaaagtttt tattgagttt tcgagttttg    120
gtttgcgcgt atttttttcgg tcgcggatat tttatcgcgt agagtttcgt tttttcgttg   180
tttttagttt tcgatgattt tttcgttttc gttttgttcg gtgatagacg ttttattgtt    240
tttaatcgga ggtatttttc gcgggagcgg ttaatcggga gtttcggtag gcggggaggt    300
cgggttagtt agatttggag gtttaatttt aatatggtcg aagtaagtag cgttaattta    360
ggtagcggtt gtgaggaaaa aaggtatgag gggtcgtttt cggaatttgt gttattcggt    420
attattattt cgagggtgaa gtttttcgat attatggtgg atattttttt ttagaagttg    480
gtcgtcgtcg gtaggtaaag tggacgtagt cgcggtggga gtgtttgttg gtatcgaagt    540
ttaaatttcg cgaggttagg acggtcgtag gttggcgcgc ggtgacgtgg gttcgcgttg    600
ggggcggggt agtcggacga ggcgatttag ttaaattttg agttttagga gttagggtat    660
ttacgtattg ataatttgta gcggatcggg atagttagtt atttttttttt ataggaagtt   720
tcgtttttat taaaatttta ggtggttggg aggaaagata gagtttttgt aaattagagt    780
agggtttttt attttttttat tatttttgag atagggtttt gcgttgtcgt ttaggttgga   840
```

```
gtgtagtggc gtgattattg tagattcgat tttttgggtt taagcgattt ttttgtcgta     900
gtttttttaag tagtcgggat tataggttcg tgttttcgcg ttcggttata gattagggtt    960
taagtcgcgg tttattatta tgtgagtaag acgtaagttt tttttttttc gtcggttttt    1020
tgttagttaa gtgatagtaa tagtattttt tttatagggt cgttttgaga attaaatgag    1080
taaatatatt taaggcgttt aggataggat ttgatatata gaaagatttg ttagttatta    1140
tgagaatatt tttttattg ggagattgtt aggattaaat gtgttattat gaaaagaaat     1200
atttttttttg tttcgatttt tattttattt atttttattt ttattttttg agacggagtt    1260
tcgttttgtc gtttaggttg gagtagtggc gcgattttgg tttattgtaa ttttcgtttt    1320
tcgggtttaa aggatttttt tgttttagtt ttttaagtag ttgggattat aggtgtatat    1380
tattatattc ggttaatttt ttgtatttgt attagtagag acgaggtttt attatgttgg    1440
ttaggttggt ttggaatttt tgatttcgtg atttatttat ttcggttttt taaagtatta    1500
ggattatagg cgtgagttat cgcgttcggt ttttattttt atataattta tatgattcga    1560
gagttttgtt tatgatttta ttattttagt atattagtgt tgaaacgttt ttaggagttc    1620
ggtgttttga ttatagtttt ttttttttta gttttttttta tttttatttt tttgtttttt    1680
ggttttatta ggatttttat ttgtttttag tttatttttt ttgggtttag tattttattt    1740
tttttacgta taattaatga tttgttttta tttatgattt attaaatatt tgagtgttag    1800
ttggggatat tatgaggtag ataaggtttt ttgttttttt ggagtttgta tttaatgcgt    1860
agagacgtta ataatattat tatataggtt ggtaagatag taaggttatt attatgtgtt    1920
aatagtgatt tatgtttgta attttcgtgt tttgggaggt tgaggtagga ggattatttg    1980
agtttggaag tttgaggttt cggtgagtta taatagtttt attgtatttt agtttaggtg    2040
atagagtaag attttgtttt taaaaaagaa aaaaaaaaaa gataatgaat aggtaatgat    2100
attattagat aggatgtttg gggcgtttat tttgagatgg tattttagtt ggaatttgaa    2160
ggatgagaag gaattagatt attattatta ttattattat tatttttttt ttttaggtag    2220
tgtttagttt tgttgtttag gttggagtgt agtggttcga tttcggttta ttgtagtttt    2280
tatttgtcgg gtttaagtga tttttttttt ttagttttttc gagtagttga gattataggt   2340
gtttgttatt acggttagtt aatttttgta tttttagta gagatagggt tttattatgt     2400
tggttaggtt ggtttcgaat tgagtttaag cgatttattt atttttgttt tttaaagtgt    2460
tgggattata ggtatgagtt attgtattta ggcggaatta ttttttttgaa gaattataga   2520
acgaatgtat aggaaaaggg gatagtttgt gtaaagtttt tgaggtgatt agatatttta    2580
ggaattggta gagatttttt tggttatagt atagtgatta gagtaaaatg agatgaggtt    2640
ggagaggtag gtaatagttt tattatgtag ggtttagttt gttataataa tgaatttgat    2700
aaaataattt gtaattattt ttatgaatta attatggtac gttattatta tatgttttgg    2760
tacggttttt tttttgttgt atttattttt ttttatttat ttttgttttt gttttttttt    2820
ttatagtttt tatttttttt tagttatatg ttttttttatt tatttattta tttatttatt   2880
tttatttttt ttagagatga ggttttgttg tgttgtttaa gttgatttta agttttgagg    2940
tttaagtgat ttttttattt tagtttttta gagtgttagg attataggta tgagttatag    3000
tgttcgtttt atttatttat ttatgagata gggttttgtt ttgttgttta ggttggagtg    3060
taatggtata atttaggttt attgtaattt ttgtttttttg ggtttaagcg atttttttgt   3120
tttagttttt cgagtggttg ggattatagg tatgtattat tatgtttagt taattttcgt    3180
atttttagta gagatggggt tttgttatgt tggttaggtt ggtgttgaat ttttgatttt    3240
aggtgattcg tttgtttcgg ttatttattt ataatataaa tatttattga ttattgatag    3300
tgttaataaa tttgttttag ttttagtagg gttttagttg ttggagataa ggtagtgatt    3360
aagatggata aggttttaat ttttttggag tagatatttt ggtattatta aaaaaaaata    3420
aaattgagtt tggcgtagtg gtttatattt gtaattttag tattttgaga ggtagaggcg    3480
ggtggattat ttgaggttag aagttcgaga ttagtttggt taatgtagcg aaattttatt    3540
tttattataa atataaaaat tagtcgggtt tagtggcgta tgtttgtaat tttagttatt    3600
cgggaggttg aagtaggaga atcgtttgaa tttgggaagc ggaggtttta gtgagttaag    3660
attttattat tgtattttag tttgggtaat agagtgagat tttattttaa aaaaataaat    3720
aaaataaaat aaaattagtg taaaggaata gagagtagtt gatacgttgt tttttggcga    3780
tttgtcgttg gagaggttgg gattttggat gcgtgcgggg ttttggttta tcggtgattc    3840
ggttagtcgg tcgtgttttt gtttgagtcg tttgttgggg ttcgcgggtt tgttgatttt    3900
tcgcgcgttc gagcgtttcg attttcggtg tcggttcggg ttcgggtttt tgatttattc    3960
gggggcggcg gggaaggcgg cgagggttat tttgttttcg tgcgtttttc gttgcgggcg    4020
ttcggggcgg tcgcgataat tttattttat tggtttcgtg tcgtgcgtgt taggcgtttt    4080
cgttttcgtt gggttgttcg tcgttttttt ttcggagtgg ggagttggtt agggtcgatc    4140
gattcgttgg tcggtcggtt cgttttcgtt ttcggggggt tt                       4182
```

<210> 192
<211> 4182
<212> DNA
<213> Artificial Sequence

<220>
<223> chemically treated genomic DNA (Homo sapiens)

<400> 192

```
gagtttttcg ggagcggagg cgggtcggtc ggttagcgag tcgatcggtt ttggttaatt      60
ttttatttcg gggaaggggc ggcggataat ttagcggaga cgagaacgtt tgatacgtac     120
ggtacggagt tagtggggtg gggttgtcgc ggtcgtttcg ggcgttcgta gcggagagcg     180
tacgggggta gggtagtttt cgtcgttttt ttcgtcgttt tcgggtgggt tagagattcg     240
gattcgggtc ggtatcggga gtcgggacgt tcggacgcgc gagagattag taggttcgcg     300
ggttttagta ggcggtttaa gtaggagtac ggtcggttag tcgggttatc ggtaggttag     360
agtttcgtac gtatttagag ttttaatttt tttagcgata ggtcgttaga ggatagcgtg     420
ttagttgttt tttattttt tatattgatt ttattttatt ttatttattt ttttgagata     480
gagttttatt ttgttgttta ggttagaatg taatggtggg attttggttt attgaaattt     540
tcgtttttta ggtttaagcg attttttgt tttagttttt cgagtagttg ggattatagg     600
tatgcgttat taggttcggt taatttttat atttgtagta gagatggggt ttcgttatat     660
tggttaggtt ggtttcgaat ttttgatttt aagtgattta ttcgtttttg tttttttaaag    720
tgttgggatt ataggtgtga gttattgcgt taggtttaat tttatttttt tttaatggta     780
ttagaatgtt tattttaaga agattggaat tttgtttatt ttgattattg ttttattttt     840
agtaattaga attttattag aattagaata aatttattga tattgttaat ggttagtaaa     900
tatttgtgtt ataaataagt ggtcgaggta ggcggattat ttgaggttag gagtttaata     960
ttagtttggt taatatggta aaattttatt tttattaaaa atacgaaaat tagttgggta    1020
tggtggtgta tgtttgtaat tttagttatt cgggaggttg agataggaga atcgtttgaa    1080
tttaagaggt aggggttgta gtgagtttag attgtgttat tatattttaa tttgagtaat    1140
agagtaagat tttgttttat aaataaataa atgggacggg tattgtggtt tatgtttgta    1200
attttagtat tttgggaggt tgaggtggga ggattatttg agttttagag tttgagatta    1260
gtttgggtaa tatagtaaga ttttattttt aaaagaaata aaaataaata aataaataaa    1320
taaatggggg gatatataat taagagaaag tggggattat gggaggggag ataagggtaa    1380
gaatgagtag ggaaaaataa atataatagg ggagggatcg tgttaggata tatgatgatg    1440
acgtgttatg attggtttat aaaagtgatt ataaattatt ttattaaatt tattattatg    1500
gtaaattagg ttttgtatga taaggttgtt atttgttttt ttagttttat tttattttat    1560
tttgattatt atgttgtagt taagaaggtt tttgttagtt tttggaatat ttgattattt    1620
tagggatttt gtataggttg ttttttttt ttatatattc gttttgtaat tttttaaaaa     1680
ggtggtttcg tttgggtgta gtggtttatg tttgtaattt tagtattttg ggaggtagag    1740
gtgagtggat cgtttgagtt tagttcgaga ttagtttggt taatatggtg aaattttgtt    1800
tttattaaaa aatataaaaa ttagttggtc gtggtagtag gtatttgtaa ttttaattat    1860
tcgggaggtt gagggaggag aattatttga attcggtagg tggagattat agtgagtcga    1920
gatcgagtta ttgtatttta gtttgggtaa tagagttaga tattgtttaa aaaaaaaaaa    1980
taataataat aataataata ataatttggt ttttttttat tttttaagtt ttagttgaaa    2040
tgttatttta gaatgggcgt tttaagtatt ttatttagtg gtattattat ttgtttatta    2100
tttttttttt tttttttttt tagaggtagg gttttgtttt gttatttagg ttggagtgta    2160
atggggttgt tataatttat cgaagtttta aatttttagg tttaaatgat ttttttgttt    2220
tagtttttta aagtacgggg attataggta tgagttattg ttgatatata ataatggttt    2280
tattatttta ttagtttatg tgatgatgtt gttggcgttt ttacgtattg aatgtaagtt    2340
ttaaaaagat aagaggtttt gtttgtttta tggtattttt agttgatatt taaatatttg    2400
atgagttatg agtgaagata aattattaat tatgcgtagg aaaggtaaag tgttgaattt    2460
aggaggaatg gattgggaat agatgagagt tttgatgaag ttaaagaata gggaagtggg    2520
gatagagagg gttagagaag gagaggttgt ggttagggta tcgaattttt ggaagcgttt    2580

taatattgat gtgttggggt ggtgggatta tgggtaaaat tttcgagtta tataaattat    2640
ataaaagtga aggtcgggcg cggtggttta cgtttgtaat tttggtattt tgggaggtcg    2700
aggtgggtgg attacgaggt taggagtttt agattagttt ggttaatatg gtgaaatttc    2760
gtttttatta gtatagatat aaaaaattag tcgggtgtgg tagtgtgtat ttgtaatttt    2820
agttatttgg gaggttgagg taggggaatt ttttgaattc gggagacgga ggttgtaatg    2880
agttaagatc gcgttattgt tttagtttgg gcgataggac gagatttcgt tttaaaaaat    2940
aaaaataaaa ataaataaaa taaaagtcga gataggaaaa atgttttttt ttatggtaat    3000
atatttagtt ttggtaattt tttagtgaag aagatatttt tataatagtt aataaatttt    3060
tttatgtgtt aggttttgtt ttaggcgttt taggtgtatt tatttattta atttttaaaa    3120
cgattttatg agaaaggtat tattgttgtt atttgattga taagaagtcg acgagggaga    3180
gggggtttac gttttgttta tatagtggtg ggtcgcgatt taaattttga tttgtagtcg    3240
```

ggcgcgggag tacgagtttg tagtttcggt tatttgggaa gttgcggtag gaagatcgtt 3300
tgagtttagg aggtcgagtt tgtagtgatt acgttattgt attttagttt gggcgataac 3360
gtaaggtttt attttaaaga taataaaaaa ataaaaaatt ttgttttaat ttgtaaaggt 3420
tttatttttt tttttaatta tttgaaattt tagtgaaaac ggggtttttt gtaggaagga 3480
gtagttagtt atttcggttc gttataggtt attagtgcgt gaatattttg atttttaagg 3540
tttaggattt gattgggtcg tttcgttcga ttgtttcgtt tttaacgcgg atttacgtta 3600
tcgcgcgtta gtttgcggtc gttttgattt cgcgggattt gagtttcggt gttaataaat 3660
atttttatcg cggttgcgtt tattttattt gtcggcggcg attagttttt gaagaaaagt 3720
gtttattatg gtgtcgagga gttttatttt cgaaatggta gtgtcgggtg gtatagattt 3780
cgaagacgat tttttatgtt tttttttttt atagtcgttg tttagattgg cgttatttgt 3840
ttcggttatg ttgaagttga atttttaaat ttaattggtt cggttttttc gtttgtcgga 3900
gttttcgatt ggtcgttttc gcgaagggtg ttttcgattg gaagtagtag aacgtttgtt 3960
atcgagtagg gcgggggcgg ggaagttatc ggaggttgag ggtagcgggg aggcgaggtt 4020
ttgcgcggtg ggatgttcgc gatcggaaaa atacgcgtaa gttaaagttc ggggggttaa 4080
taaaaatttt taattatatt ttagagattt cgtatagtgt aatagtgaat atttattgtt 4140
aatttttata agagtttatt ttattaaacg tttagagagt tt 4182

<210> 193
<211> 7572
<212> DNA
<213> Artificial Sequence

<220>
<223> chemically treated genomic DNA (Homo sapiens)

<400> 193

tgtatgagta tggatgttat gagggtattt agtgagtggg aagggagttg ttttatatgt 60
ttttatattg atttgtaaaa ttgttttgaa agggtatttt tagatttatt tgttttaaat 120
gtagtaaaat ttatttgata ttgtttataa tgtatgtatg ggttatttta tatgtaatag 180
ttattgtatt aattaattta gttgatattg agatggtgaa tggggtgggg gagtagggag 240
gaatatgttg tgtatgaaaa attttgggtg taaaagttaa tgtagaattt attttttgag 300
gaattgaaaa agatatttat gatttttaag tggagagagg atagaaagtt aagtggtggg 360
ggttgtgatt agtgattggg attttagttt gattttagta tttagtggaa taggattata 420
atttaaagaa atgagttttt tttggagtaa agagtggttt tgattttaaa gtagaaaaaa 480
taaattgttt gggggtggaa gatggtgagg ggtgggggt ggggagggag gtgggttgtg 540
gtgttggttt tggggtttgt ggtatggttt tttttgttgt gttgtttagg gtttggattt 600
tgtttggtgt tgtgtttatt gtgagggtgt tagatttgtt gtgttgttgt tgtgtttttt 660
tagttgtggt tttttttttt gttgtttttt tttgtttttt tagttttttt ttttatgtgt 720
ggatgatgtt aaaattttgt gaaaaagttg tgtggtggtt ttttgagtgg aggtgtgatt 780
ttgttgttta gtggtttttt tttgggggtg tttggagggg gagaagggtg gaggtggttg 840
gttttttttt tttttgggag gtaggatttt ttgatgttga ttgttggatg ttttttgttt 900
taaagtttat tggaaaattt atttttgtaa agtaaatgta tttttagagt tatttttggt 960
tgtgtgaggt gtgttttaag ttgaattaga taggaagagg gggaagtttg ggttttttaa 1020
tttttttttt ttttgaaggg aggggagtga gatgttaggt gggtgataga tggtaggtgt 1080
ttgttttttt aatttatgtt tgttgtattt gttgttttag taatttagtt ttgtttaagt 1140
tgaaatttgt tgaagggagt gtggatgtat aggtttggtg gattttgttt ttttttagaa 1200
tgtagtggtt tagtgttttg gtgggtgtgg ttgtgattag agggttttgg aagtgagtga 1260
atatttgtaa ttgtattgtt tgtttttatt tattttgttt ttttgtgttt tttgtttttt 1320
gatgtttttt tttttttttt gtttgtattt ttttattttg tttgtatttt tttttttttt 1380
ttgatgtgtt ttttatgtgt ttttttggat ttatgtgttt ttttgtattt tgttagttgg 1440
gtttttggtt ttgattgatt gtttattttt ttatggagaa atataaatat agttttattt 1500
tttgggggag ttgaggggaa ggtagtggtt tttattttttg agtttgaatt tagttattat 1560
ttgtttttta tttggtttag gtgtttttgt gtggagaagg tgggatttga atttgtgttg 1620
tttttgggtt ttgagttgat tgtggaatta tttggttggg aggtattttt taggtaagtt 1680
tgggagtatg tgtgtttagt gttgtaggtt tttgtagaaa agtgtttata aatatttaat 1740
atttgttgt atttttttatt tttaggtttt gtatattttt taattgaaat tttaaaatgt 1800
tagtgtagag ggtggggagt tggttggaaa aagaataaaa gtttaataaa attggtgttt 1860
gttaaagttt attattagtt ttatatggga tatatatata tgtatttttt tttttgtgaa 1920
gggtgaaaag gagataagga agaattaata atttattttt ttttgttgtt ttttattttt 1980
gtgttatggt gattagtttg gattttaaag ttagagtata ggtttttgtg tttttttttg 2040

```
aaattgaagt ttatattgaa agaaagtgtg tatttttgtt tagaaggtaa tatgtgtttt     2100
tttgtgtgtt aggtggagtg tattttaata agatattagg gttttaatat atggttggag     2160
tggtgattaa aagggaaaat ttagttttta gtttaagttt tttagagata tttttgttaa     2220
tttttgtatt tttttatgtt ttattttatg tgtgagagtt tgtaaaatta ttggggattg     2280
gatttgatgg tgagttttat gtttgggaat agttagtaat tggaagggga agtggatagg     2340
ggaattttaa gaggtgagtt tgttatgtgg gaagtgtttg aatttgtggg tttttatgaa     2400
tgtagaggt gttgggaagg gggggtattt ggttgtgatt ttttttgttt tttttatttg     2460
ttgttttttt ttttgttgga atttttttgt aaagtaagat ggagtagggg gagggggaga     2520
gggaaggggt gagagggttt ttggtttatt tttgagatgt gaggatttgt tatttaggta     2580
ttttttttgg ggtgggttgg gttttgggat gattatttgt tttttttttg tttttgttgt     2640
ttttattttg ggagatttag agttttggat gttttttttt ggagaagggg gggtgtgtgg     2700
agttggggtg gaagagattt tgtttgtaga gttatattaa gtgatgttta gaggttggga     2760
gttttggtgg tttttgtttt ttgtttgttg ggttagattt atattttaaa aatatttttt     2820
gttttttttt tttttttgt ttttttttgt tgtaattttt tttgatttgt ttaaaggtgg     2880
tttaggtgaa attggagtaa tttttttatg gggttttaa aatgtaagtg aatgttttta     2940
tttggggtga tttaaaagtt taagttggga agtttaatgt gattaaaatt aataggtgat     3000
tgtttggggt tgattgtgtg tgggtgtata tggtatttgg tttgggtgtt atttgtggtg     3060
ttggattgtt ttattttgag tttgtaattt gggtttttta gtgagttttt tttttttttg     3120
aaagttaatg gtttttatag taattagata tttttttgt ttgtttttt ttttttttt     3180
ttttatatat aggagatggg atatttattt ttgttgttat tgataaggtt ggaggtggtt     3240
gggttttttt ttagtttttg tttgttttag tgtttgtttt tttttgtttt tttttggtt     3300
tttttttgat gtagtgggga atgtgtttta ttaaaaaaaa aaaaaaaaaa aaaaaaaaaa     3360
agtaatttgt ttggtaataa ttagtgtgta gtagtaggag ttttagagtt attggttatg     3420
taaatagagg gaggggagat ggtgttttaa attttttttt atttttttaa agtgatattt     3480
tttttttttt tttttattat tttttttgtt ttattttgt ttaaagaggt tggttttggg     3540
gtttgagtta attgtttgta tttttagttt atttgttttt tttttttgtt ttgtagggaa     3600
tttagtgtat ggtttattta gtttgtgttt tattttgttt tgttggtttt ttgtgttttt     3660
gtttgggttt ttttttttgg tgagggaggt atttagttgg ttttggtgtg tttagagagt     3720
ttgagttatg ttatgtttgt tgtatgtgtt agtttggtta gtatattagg gtgttggttt     3780
tttttttttt ttttggagtg aaatatatta aagggtgtgg tgggggtggg gggtgatggg     3840
aggaaggagg tgaagaaatg ttattagatt gtattttttg taaagatagt tttgatttaa     3900
gtatgtgtta gagtatgtgt tagggttgat tgtgttggtg gtgattttat tgtagttggt     3960
ttttagggag aaagtttggt gagtgaggtg tgaaattgga ggggttggtg aggatgtggg     4020
tgaaggattg agtgtggagg ttttatgttt ttggggaaag gaaggggtgg tggtgtttgt     4080
gtagggggag tgagggggag ttggatttaa ttttttattt gttttttttt ttttttgggt     4140
tattttttag aaagttgtat tggtgtggtt atgtttagtg tagatatttt gggtggtttg     4200
ttagtagatg taggggtgag gaagtgggtt ttttttgtgt ggttgttggt tgtgggggaa     4260
ttgttgggag ttttgttttt ggtttgtggt ggttttagat gttgtattgt gttgttttat     4320
ggtgtttgaa gagtttttag aaatatgatg gtttttgttt gaggattata ttttattttt     4380
ttagagaagt attttttttt ttttttaata tttatttttt tttttttttt ttttttttgt     4440
atatattttg taggggggggg tagaagggat gttgttttgg ttttttttaat tggggttttt     4500
gaaatagttt tgaagttatt aggaatatag attttaggga tatgattttt atttttgggt     4560
atgtgaggtt gttatttttt aaaattattt ttttttttat tttttattta agggatttat     4620
ttttaaattg tttgaggtta ttttattttt agataattta ttttatattt ttggatttta     4680
aatataaggg taggaggatt aggatttgtt ttgaagaagt taaagttgga gggttgtatt     4740
ttggtgtgtt atatttatag aatgagtgaa attagagggt agaaatagga gttggtagtt     4800
ttttgtgggt tgttttgttt ggggtttttg gtatgtaggg ttggatggag ggagaggggt     4860
ggggggtggt gggggattgt gtttgaagtt gggttgggtt agttgttgtt ttttttaata     4920
atgagagggg aaaaggaggg agggagggag agattgaaag gaggagggga ggattgggag     4980
gggaggaaag gggaggagga attagagtgg ggagtgtggg gagagggagg agagttaatt     5040
gtttagttag tttgtgttat tgttttagag tggagaagag tgagtagggg agagtgagat     5100
tagttttaag gggaggattg gtgtgagtga ggtagttttg aggttttgtt tgtttattat     5160
ttaatttttg tttttttttt gttttatttt tttttttgt ttttattttt tttttgaaaa     5220
ttttttattt agttaaagga aggaggttag gggaatgttt tttttttttt ttttaaaaaa     5280
taaaaataga aaaatttttt tttaggttgg ggaaagtagg agggagaggg gttgttgggt     5340
tggttatgga gttgttgtgt tatgaggtgg atttggtttg tagggttgtg tgggattgta     5400
atttgttttg agatgattgt gttttgtaga atttgtttat tattgaggag tgttattttt     5460
tgtagtgttt ttattttaag tgtgtgtaga aggatattta attttatatg tgtagaatgg     5520
tggttatttg gatgttggag gtaggttggg gggtggtgtt tgttaggagt taggattttt     5580
ttggatgttt gggtttttgg ttggagtttt aaatttggga gagggtaatt tttgtgttgg     5640
```

ttttttggtt tttgtgtggg agtttattgt gtgttttttg gtgagatgtg tggttttatt 5700
tttgtttttt tttagataaa ttggggaggt agagggggga ggaaaatttg ggagaagtga 5760
ggttgttttg ggtgggggta ggggagtatt ttgtgtgtgt gtttttgtat gtggttgttt 5820
ttttttttta ttttttttgt gatttgtttt ttgtgaagtt gttgtggttg tttgtgttgt 5880
ggttaggaag agagttgggg tttgaaattg ggattttgag tagaaaggta attttttttta 5940
aaaggttaga gtaaatttgt tttggtttta ggtttttgtt tgtggttgtg attttgtgtt 6000
ggtattttat tggggaggtg gagggaggag ggagaaggag agaaatgggg aatttgggag 6060
ttttggaagt tttattgaag aaatgtgtgt tttaggggaa tttaaaagaa ttgtttttttg 6120
tttttttattt taagtttttg tttagtgagt tgtgtgttta tgtatgtata gttttggatt 6180
tgttgtggtt ttgttatgtt gttttgtaaa ttttttttgg aaaggttggg aaatgttgtt 6240
tgtttatttt tgtattttat tattttggtt attttatttt taattgtgtt ttttttttta 6300
ttttttattt tatgaagatt atttatagtt tggtttttta gtttttttgg tgtggagatt 6360
tgggtggggt aggaaggggg ggagggggtg tgtgtagggg ggatattggg ttatttttga 6420
aggagaggtg ggagtttttt gtgtttttttt ttagggtttt ttgtttttgt tttttttttt 6480
gtttgttatt gtgtttttta gtttttgttt ttgtgggttg tagttggata tttttgttgt 6540
ggtgtttatt tttttaagtt tttttttggg atttaaagtt ttaggggttg gaatatagat 6600
ttttttggta tttgaaattt ttgttttggg aagtttggtt tttttttggg gtttttatgt 6660
taaaagggtt tttttaggat atttttttga tttttaatta ttttttgaaa ttattttttt 6720
ttgttttgat tttagttgtt gaggttgtgg tgtttttttt tttttttttt tttttttttt 6780
ttttttttatt tttttttatt tgtttttttta aaagtggttt ttgggttgta gggatttttt 6840
tagatatttt ttttaattgt tgggaatgat agaggaggat ttggggattt ggatgagatt 6900
aagaagtgga tttttggagt ttttagaata tttttattga ttttttgaaa agatgatgaa 6960
atttaaaaaa gagtgagtga gttagagtgt gtgaaggagt agttaaaggg agtgttgtgg 7020
agttgttgtg gttgttgtgt atttttttga ttttttgttt tggagttgta gtttattttt 7080
ttttatagga tttttgggaa tattaaagta ttgatgggtt attttgattt attttagttt 7140
ttttattttt gtttttttatt tttattatgt attttggttt tttttttttt ttataaaaaa 7200
aaattaattt tttttgtttt gatagattat gttttttttat tttttttttt ttttgttgat 7260
gttatgtttt ttatttttgt tttttaattt tttttttattt ttattatagg tttgtgagga 7320
atagaagtgt gaagaagagg tttttttttt ggttatgaat tatttggatt gtttttttggt 7380
tggggttttg attttgaagt tttatttgta atttttgggt gttgtttgta tgtttttttggt 7440
ttttaaattt aaagagatta gtttgttgat tgtggagaag ttgtgtattt atattgataa 7500
ttttattaag ttttaggagt tgttggtaat gattggtttt tttttttttt tttttttgtg 7560
atttttgttt tt 7572

<210> 194
<211> 7572
<212> DNA
<213> Artificial Sequence

<220>
<223> chemically treated genomic DNA (Homo sapiens)

<400> 194

gaaagtggga attgtagaaa ggaagggagg aaggggttgg ttattattag tagtttttga 60
ggtttgatgg agttgttggt gtaaatgtat agttttttttg tggttagtgg gttggttttt 120
ttgagtttgg aggttaggaa tatgtagata gtatttagga gttgtagatg ggattttgga 180
gttgggattt tagttaagaa atggtttagg taatttatgg ttagagggaa gatttttttt 240
ttgtattttt gtttttttata gatttataat gggagtggga aagggttggg gggtgggggt 300
ggagagtata gtattagtaa aagagaaaaa gaataaaaaa gtgtaattta ttgaaataaa 360
aaaaattaat ttttttttgt gggaggggga ggggattaga atgtgtgata agaataaaga 420
ataaagatga ggaaattgga gtgaattaga atagtttatt agtgttttgg tatttttagg 480
gattttataa aaggggggtga attatagttt taaaatgggg agttggagaa gtgtgtagta 540
gttgtggtag ttttgtgatg ttttttttgg ttattttttt gtgtatttta atttatttat 600
ttttttttgg attttgttat ttttttaaaa aattaataaa aatattttgg aggttttggg 660
ggtttatttt ttggttttat ttggattttt agattttttt ttattatttt tgataattgg 720
aaaaaatgtt tggggggggtt tttgtggttt aggggttgtt tttggggggg tgggtgagga 780
gaggtgggga agagaggggg agggaggaga ggaggaagag tgttgtagtt ttggtagttg 840
gagttggagt agggggggat ggttttaaaa aataattaaa aattgaggaa atattttaga 900
aaagtttttt tggtgtgaaa attttaaaga agagttaggt tttttgaaat agaggttttg 960
ggtgttagaa aggtttgtat tttgattttt ggggttttgg attttgggag gaaatttgaa 1020

```
gggtgagtg ttgtggtggg agtgtttggt tgtggtttat aaggatagaa attagggaat    1080
gtggtgatga gtgaggggaa agatgggagt agagagtttt ggaaagaggt atggggggtt    1140
tttattttt ttttaggggt gatttggtat ttttttata tatatttttt tttttttttt     1200
ttattttatt taaatttttg tattgaggaa gttggaaaat taaattgtaa gtaattttg    1260
tgaggtgggg ggtgggggag agggtatgat tagaagtggg gtggttggga taatggggtg   1320
tgagggtaag tgggtgatgt tttttagttt ttttaaaggg agtttgtaaa gtaatatggt   1380
gaaattatgg taggtttaga gttgtgtata tgtgggtata tggtttgttg ggtaaagatt   1440
tggagtgagg ggtaagaagt ggtttttttg ggtttttttg aaatatgtgt tttttttaatg  1500
ggatttttgg ggtttttgaa tttttttgttt tttttttttt tttttttttt tttatttttt  1560
tggtgaagtg ttagtgtgga gttgtgatta taggtgggga tttgaggtta agatgaattt   1620
gttttggttt ttttgggggg gttgtttttt tatttggggt tttgatttta ggttttgatt   1680
tttttttttgg ttgtagtgta agtagttgtg gtggttttgt aaaagatagg ttgtgagggg  1740
ggtgggaaga agaggtagtt atatgtagga atatgtgtgt gggatgtttt tttattttttg  1800
tttaggatag ttttgttttt tttagatttt tttttttttt ttgttttttt agtttatttg   1860
gagaggaata gaaataaagt tatgtgtttt gttagaaggt gtgtggtaaa tttttgtata   1920
ggagttggga ggttggtgtg gggattgttt ttttttaggt ttagggtttt ggttggggat   1980
ttgagtattt ggaggggttt tggtttttgg tgagtgttat tttttgattt attttttagta  2040
tttaggtggt tattattttg tgtatgtagg gttggatgtt tttttgtatg tatttgaagt   2100
aggagtattg tggaaggtag tgttttttga tggtgagtag gttttgtagg atgtggttgt   2160
tttggagtag gttgtggttt tgtatggttt tgtggattgg gtttattttg tggtatagta   2220
gttttatggt tagtttggtg gttttttttt tttttgtttt ttttggtttg gaaaaaggtt   2280
tttttgtttt tgttttttgg aaggggaggg gagagtgttt ttttgatttt ttttttttgg   2340
ttaaataggg ggtttttggg ggagaggtga gggtagagag agaaggtgga gtagaaggga   2400
ggtgaggatt gggtggtggg tgagtagagt tttggggttg ttttatttgt attggttttt   2460
tttttaaaat tggttttgtt ttttttgttt tgttttttttt tgttttgaag tggtgatgta  2520
agttggttgg gtagttagtt tttttttttt tttttgtgtt ttttgttttg gttttttttt   2580
tttttttttt tttttttggt tttttttttt ttttttttaat tttttttttt tttttttttt  2640
tttttttttt gttattaagg agaatagtag ttggtttgat ttaattttaa atgtggtttt   2700
ttgttatttt ttatttttttt ttttttattt agttttgtat gttaggggtt ttggataggg  2760
taatttataa aaaattattg atttttattt ttgtttttta attttattta ttttgtaggt   2820
gtagtatgtt aaaatatgat tttttaattt tggttttttt aaaatggatt ttaatttttt   2880
tgtttttgta tttaagattt aggaatgtag ggtagattat ttgaagatgg ggtgatttta   2940
gataatttag aaataggttt tttaagtgaa aaataaggga gggggtgatt ttagaaaata   3000
gtaattttgt atatttagag ataaaggtta tgtttttgaa gtttgtgttt ttgataattt   3060
tgaagttgtt ttgaaagttt tgattaaagg gattagaata atgttttttt tgtttttttt   3120
tgtagagtgt gtgtagagga agaagaggga gggagaggtg ggtattagga aggaaggggg   3180
gtgttttttt ggagggatag aatgtgattt ttgagtagaa attattgtgt ttttgggggt   3240
tttttgggtg ttgtggggtg atgtggtgta gtgtttaggg ttgttgtagg ttgggggtag   3300
ggtttttagt ggtttttttg tggttagtgg ttatgtagga aaaatttgtt tttttgtttt   3360
tgtatttgtt gataagttgt ttgaggtgtt tgtgttgagt gtggttatat tgatgtagtt   3420
ttttaggaaa tggtttggga gggggagggg gtgagtgagg gattaggttt ggtttttttt   3480
tgtttttttt gtgtaaatat tattattttt tttttttttt ggaggtatga ggtttttatg   3540
tttggttttt tgtttgtatt tttgttgatt tttttggttt tgtgttttat ttgttaggtt   3600
tttttttttgg gagttgattg tggtgaagtt gttgttagta tggttggttt tgatatgtgt  3660
tttaatgtat gtttgagtta aggttgtttt tataggagat atgatttggt ggtgtttttt   3720
tatttttttt tttttgttat tttttatttt tattgtgttt tttggtgtat tttattttag   3780
gaggaagggg agagattagt gttttgatgt gttggttaag ttggtatgtg tagtgggtat   3840
ggtgtggttt gagtttttttg ggtatattga ggttgattga gtgttttttt tattgagaga  3900
gggggtttgg gtaggggtgt ggagagttag taaggtgggg tggggtgtgg gttgggtgag   3960
ttgtatatta ggttttttgt aaggtggagg aggggagtga ataaattaga ggtgtaagtg   4020
attaatttag gttttggagt tagttttttt aaatgagggt ggggtggaag gggtggtggg   4080
ggggaaagga ggggatattg ttttaaaagg gtgaggaaga gtttggggtg ttgttttttt   4140
tttttttatt tgtatagtta atagttttgg ggtttttgtt attgtgtgtt gattgttatt   4200
gggtagatta tttttttttt tttttttttt tttttttttt agtaggatgt gtttttttatt  4260
atattaaaag gaagttaagg gagggtggag ggagagtggg tgttggggtg ggtgaaagtt   4320
ggggagaggt ttggttgttt ttgattttat taatagtagt gggaatgagt attttatttt   4380
ttatatgtaa agaaaaggga gggaaggggt gggtgaggaa aatgtttaat tgttgtggaa   4440
gttattagtt tttaggaaga aaaaaagttt gttgaaaaat ttaagttgta aatttaaaat   4500
gaaatagttt agtgttgtgg atgatatttg ggttgaatat tgtgtatatt tgtatatagt   4560
tggttttgaa taattattta ttggttttag ttatgttggg tttttttgatt taagtttttg  4620
agttattttg gataaggata tttatttata ttttaagatt tttataaggg aattatttta   4680
```

atttattta agttatttt gagtggatta aagaaagttg tagtggggag aggtgagaga 4740
aggagggagg gtgggaggta tttttaaagt ataaatttaa tttgataggt aagggataga 4800
ggttgttggg gtttttagtt tttggatatt atttgatata gttttgtgag taaggttttt 4860
tttattttga ttttgtatat tttttttttt ttggggaaag gtatttagag ttttgggttt 4920
tttgaagtgg gggtagtggg ggtgaggaag aagtgggtgg ttgttttggg gtttagttta 4980
ttttgaggag aatgtttggg tggtgagttt ttatgttttg ggagtgagtt gagggttttt 5040
ttgttttttt tttttttttt ttttttattt ttgttttgtt ttatagagaa attttagtgg 5100
ggaggggggt agtgaatggg aggggtagag agggttgtgg ttggatgttt ttttttttttg 5160
gtgtttttttg tatttatgga gatttgtaag tttgggtgtt ttttgtgtgg taggtttgtt 5220
ttttgaagtt tttttgttta tttttttttt tgattattga ttgtttttga gtgtgaagtt 5280

tgttattgaa tttaattttt ggtggttttg tagattttta tatgtggggt ggggtgtgag 5340
agggtgtgga ggttggtagg aatgttttta ggaggtttgg attggaaatt gagttttttt 5400
ttttggttgt tattttagtt atgtgttaaa attttaatgt tttgttaaaa tgtattttat 5460
ttagtatgtg ggaaaatgta tgttgttttt taggtaaaag tgtatatttt tttttagtgt 5520
agattttggt tttaagaaaa agtgtaagag tttgtgtttt ggttttgaag tttaaattga 5580
ttattgtggt gtgggggtgg ggggtggtgg gagggggtag attgttggtt tttttttatt 5640
ttttttttat tttttatgga aaaaaaaata tatatatata tattttatgt aggattggtg 5700
ataaatttta gtgaatgtta gttttattgg atttttattt ttttttttggt tggtttttta 5760
ttttttatgt tagtgttttg gagttttggt tggaaagtgt ataaagtttg gaggtggagg 5820
gtgtagtagg gtgttgggtg tttataggta tttttttgta ggagtttgta gtattaagta 5880
tatatatttt taagtttatt tggaggatgt tttttagtta ggtgatttttg tggttggttt 5940
aaggtttggg agtagtgtga gtttgagttt tgtttttttt gtgtaggggt gtttaggtta 6000
ggtggggagt gggtagtgat tgagtttagg tttagaaatg ggagttattg tttttttttt 6060
ggttttttta aagagtggag ttgtgtttgt gttttttttgt ggagggatgg atggttaatt 6120
aaaattgaaa atttgattgg taaaatgtga ggggatatat aaatttgagg ggatatatag 6180
gaggtatatt aagaggaagg agggagtgta ggtaaagtag aaaaatgtgg ataaggagaa 6240
gaggaaaatg ttgggaagtg gagagtatgg gggagtaggg tgggtgggga tgggtagtgt 6300
gattgtaggt gtttatttgt ttttgggatt ttttggttgt agttgtgttt gttggggtgt 6360
tgggttgttg tgttttggag gggggtagag tttgttaggt ttgtgtattt gtattttttt 6420
tggtgaattt tggtttggat ggggttggat tattgaggtg gtagatgtga taggtgtgag 6480
ttaagaaggt gagtgtttgt tgtttgttat ttatttagta ttttattttt ttttttttgg 6540
aaaaaaaaaa aattaaaaga tttgaatttt tttttttttt tgtttgattt agtttaggat 6600
atgttttatg tggttgaaaa tagttttgga aatatatttg ttttataaaa gtgaattttt 6660
taataagttt tggggtgggg ggtgtttggt ggttggtgtt ggggggtttt gtttttttggg 6720
aggagaagga attggttgtt tttgtttttt ttttttttta ggtgtttttg ggagggggtt 6780
gttgggtggt ggaattgtgt ttttgtttgg ggagttatta tgtggttttt ttgtggaatt 6840
ttgatgttat ttatatgtgg gggaaggggt tgggagagta agaggaagtg gtgggggagg 6900
gggttgtggt tgggagagtg tggtggtggt gtggtgggtt tggtattttt gtggtgggtg 6960
tagtgttggg tagggtttgg gttttgggta gtgtggtaga ggaggttgtg ttgtggattt 7020
tggagttagt gttgtgattt gttttttttt ttgtttttttg tttttttgtta tttttttattt 7080
ttaagtagtt tatttttttt attttgaaat tggggttgtt ttttgtttttg gaaaaagttt 7140
atttttttag gttatggttt tgttttgttg ggtattgaga ttagattgaa attttgatta 7200
ttggttgtga tttttgttat ttgatttttt attttttttt tatttgagag ttgtgaatgt 7260
ttttttttggt tttttgggga gtgagttttg tattgatttt tatatttgga gttttttatg 7320
tgtgatatat ttttttttgt tttttttgttt tatttattat tttagtatta gttgggttgg 7380
ttaatatgat ggttattata tgtaagataa tttgtgtatg tattgtgagt gatattggat 7440
gaattttgtt gtatttggaa taaatggatt tagaaatatt tttttaaaat agttttataa 7500
attgatgtga aaatatgtag ggtgattttt tttttatttg ttggatattt ttgtggtatt 7560
tgtgtttatg ta 7572

<210> 195
<211> 3491
<212> DNA
<213> Artificial Sequence

<220>
<223> chemically treated genomic DNA (Homo sapiens)

<400> 195

```
gttggttttg aatttttgat tttaggtgat ttttttgtt ttgatttttt aaagtgaagg      60
gattataagg tgtgaggtat tgtgtttggt tgtttttgaa taattttgat taaaatttat     120
atttgatatt tattttaata tatattatag atttttattg ataatttttt ttagtaagaa     180
agataagttt tatttaggta tttgtgaatt ggaggttaag tagttttagt atattttata     240
tttttttaag attttttttt tattttaaat gtttgtaaat tttgtatttg ataaagagta     300
tatttttatt taatataaat atgttttttt ttttagattt ttttagtatt tgagagattt     360
gtatgtgtgt ggtttttttat tttttttttt tggtttttta agtttttagg gtgttgttag     420
gaggaggttt gtgattataa attttttttg aaaatttttt aggaagtttt tttttttttt     480
ggagaattga agtgttattt gattttaatt tttttgtaaa ttttgttttt tagagttgtt     540
tgttattttt tgtttttgtt gtagattttt tatttatttg gattggtttt tgattgtaat     600
tatttggtgt gttgggtagt gtttttgttt ttagtagtgt ttgtattttt tttatttgat     660
tttgggttgt ggttgtggtt agttagttag ttgaaggttt tatgttgttt tttgttgttg     720
gttttatgtt gtttttttgtt gtttgttgtt tgtttttttt ttttttgtag ttgttgagtg     780
tatgtggttt gttttatttt ttggtgatta gttagttttt tttttttttt tttttggtgt     840
tggtggaaga gttttttttg attttgtttt ttaaattttt tggagggatt gtggtatttt     900
tttaggtaag gggatgttgt gagtgagtgt ttggaggagg tgttattaat tttgagtatt     960
tagtgaatgt ggtatttttg aagttgtttt aggttgggtt tttttttgggg gtattagttg    1020
gaagtagttt ttgttagagt tagtgttggt aaggaaggag gattgggttt tttttttattt    1080
gtttttttata ttgtttttttg gttttttttgt ttttagttgt gtttttttgt ttgttagtaa    1140
aggtgtgttt gagtgtgttt attttgttaa aaagaaattt gtttttgttt tgtttttttt    1200
ttttgtgata taattttttt aattgttaaa ttgaattggg gtgtttggtg ttatagggaa    1260
agtatggttt ttttttttaa ttataagaaa aagtaaaatt attttttttt agttgtgaga    1320
gttttattga gaattgaaat tatttgtatg attagaaagt gttttttttat tttttttaatt    1380
tttgattttt aggagtgtgg ggtttattaa gttagaaatt ttagtttaaa ggattttttt    1440
tggagagttg gattgttttt tttttttttt tttttttttt ttttgtgtgt aaaatggttg    1500
tttggggtaa gggtttttta gatgtgtata ttgtttggta taagagtaga ttttgaaaag    1560
atgaggttta tttaatatgg atgggggaga attttgtttg taggtagata ggaaaatggg    1620
gagggagtta ttggaaggat ggattttatt tttaaagtta taatttttag attagaaaaa    1680
gtgtttagtg ttttagaagt agagttgtat agtgatttaa agattagttt taaatattgt    1740
tttgtttttt ttatattttt tatatttttt tttttattg aaaatatttt gtattttttg    1800
taattataaa gggggaaggg aatatgagtg tttttttgttt tatagggggtt gttgtgagtt    1860
taaatgatgt attaatatat ataagtttta agaatagtgt tatatatttt aagttaatat    1920
ttgttagttt ttgaattatt tgttttgagg attggtttgt aattttgttt tgaggtatag    1980
aaagaaaatg ttttggagta ggatgtggtg gtttatattt gtaattttag tattttggga    2040
agttgaggtg ggtagattat ttgaggttag gagtttgagg ttagtttggt taaaatggtg    2100
atattttgtt tttattaaaa atataaaaat tagttggtta tggtggtgta tgtgtgtaat    2160
tttagttatt taggaggttg aggtaggaga attgtttgaa tttgggaggt agaggttgta    2220
gtaagttgag attgtgttat tattttttag tttgggtgat agaatgagat tttgatttaa    2280
aaaaaaaaaa aaaaatgttt tggatagaat tattattatt atataaaagg aaagtttgga    2340
tgtggtggtt tatgtttata attttagtat tttgggaggt tgagataggt ggattatttg    2400
aggttaggag tttgagataa gtttgattaa tatggtgaaa ttttgttttt attaaaaaat    2460
ataaaattag tggggtttgg tggtgtatgt ttgtaatttt agttatttgg aggttgatgt    2520
aggagaattg tttgaattta ggagaaggtg gaggttgtag tgagttgaga ttgtgttatt    2580
gtattttagt ttgggagata agagtgaaat ttggttttaa gaaaaaaaga aagaaagaaa    2640
gaaagaaaga ttaagaagaa tttatttttt gaaaagatta tgggtatttt ttattatttt    2700
tatttataaa gaaaagttaa atagtattaa agagtataat aagtgtaagg aggtaaaagt    2760
tttaattttt tttgtgatta ttatttttta agtttattaa aaatatgtat tatgttttaa    2820
aaaatggatt gtttagattt tgttgatgtt ttaagtatat gtttaatttg tttattggat    2880
aatttagttt tgtttaaaag ttatatttta atttttggtt gatttaaatt ttgtagattt    2940
agtatatttt gtatttttag tgtttgtttg attttaaaat atgtagtttt taaaatgaag    3000
ttaatgaaaa taatttggga tgttaagtat gttattaaaa tttataatgt attattgtat    3060
tatttatatt tttttttgggg tattttttaa ttagttgtgt agtaatgata gggaaaattt    3120
aaattattga taaataaaat tattttagtt tagtttaaga tattttatga tggaggagga    3180
agaaagtggt tgttaggatg ggagggaggg aatatatttt tattattaat ataatggttt    3240
tttttttttg tttgtttgtt ttgtgtgttt ttttgagatg gagttttgtt ttgttgttta    3300
ggttggagtg aaatggtatt gtatgatttt ttttggttta ttgtaatttt tgttttttttgg    3360
gtttgagtaa tttttttgtt ttagtttttt gagtagttgg gattataggt atatgttgtt    3420
atatttagtt aatttttgta ttattagtag agatggggtt ttattatgtt ggttaggttt    3480
gttttgaatt t                                                        3491
```

<210> 196

<211> 3491
<212> DNA
<213> Artificial Sequence

<220>
<223> chemically treated genomic DNA (Homo sapiens)

<400> 196

```
gagtttgaga taagtttagt taatatagtg aaattttgtt tttattaata atataaaaat      60
tagttgggta tggtagtatg tgtttgtaat tttagttatt tgggaggttg aggtaggaga     120
attgtttgaa tttgggaggt ggaggttgta gtgaattgag agagattgtg tggtgttatt     180
ttattttagt ttgggtaata gagtgaaatt ttattttaaa aaaatatata aaataaataa     240
ataaaaagaa agaattattg tattagtgat ggaaatgtgt tttttttttt ttattttggt     300
aattattttt tttttttttt attataaaat attttaaatt aaattaaaat aattttattt     360
attgatagtt tgaatttttt ttattattgt tatatagtta attgagaggt attttgagga     420
aaatataaat ggtatagtaa tgtattgtag attttaataa tatatttgat attttaaatt     480
gtttttattg gttttatttt aaaaattata tgttttaaaa ttaagtagat attaaaagta     540
taagatatat tgggtttata aggtttaagt taattaggga ttgaaatata atttttaaat     600
agagttggat tatttagtag gtagattaag tatgtgttta aggtattagt aaagtttgag     660
taatttattt tttaaaatgt agtatatgtt tttgataagt ttaaaaagta gtagttatag     720
gaaaaattag aatttttatt tttttgtgtt tgttatattt tttagtgttg tttaattttt     780
ttttgtaagt gagggtggtg gagggtgttt ataatttttt tagggagtaa gttttttttg     840
gttttttttt tttttttttt tttttttttt tttgagatta agttttgttt ttgtttttta     900
ggttggagtg taatggtgtg attttggttt attgtaattt ttgttttttt ttgggtttaa     960
gtgatttttt tatattagtt tttgagtagt tgggattata ggtatgtgtt attaagtttt    1020
gttaatttttg tattttttag tagagatagg gttttgttat gttggttagg tttgttttga   1080
atttttggtt ttaggtgatt tgtttgtttt ggttttttag aatgttggga ttatagatgt    1140
gagttattgt atttggattt tttttttatg taatagtgat aattttattt aaagtatttt    1200
tttttttttt tttgagttgg agttttattt tgttatttag gttggagggt ggtggtgtga    1260
ttttggttta ttgtaatttt tgtttttttgg gtttaagtga tttttttgtt ttagtttttt   1320
gagtagttgg aattatatat gtgtgttatt atggttagtt aatttttgta tttttagtag    1380
agatggggtg ttattatttt ggttaagttg gttttgaatt tttgatttta ggtgatttgt    1440
ttgttttggt tttttaaagt gttgggatta taggtgtgag ttattgtgtt ttgttttaaa    1500
gtattttttt tttatgtttt aaaataagat tgtaagttag tttttaaagt ggataattta    1560
agagttaata ggtattagtt taggatgtgt ggtattgttt ttaaggttta tatgtattaa    1620
tatattattt aaatttataa taatttttat aaagtagggg gtatttatat tttttttttt    1680
ttttataatt atgaaaaatg taaggtattt ttagtaggaa agagaaatgt gagaagtgtg    1740
aaggagatag gatagtattt gaagttggtt tttggattat tgtgtaattt tgtttttaga    1800
atattgagta tttttttttgg tttaggaatt atgattttga gaatggagtt tgttttttta   1860
atgatttttt ttttattttt ttatttgttt ataggtagaa tttttttttg tttgtattaa    1920
ataaatttta ttttttttaga gtttgttttt atattaggta atgtatatgt ttgagaaatt   1980
tttgttttag atagttgttt tatatgtagg aggggaaggg gaggggaagg agagagtagt    2040
ttgatttttt aaaaggaatt ttttgaatta gggtttttga tttagtgaat tttgtgtttt    2100
tgaaaattaa gggttgaggg ggtagggggga tattttttag ttgtataggt gattttgatt   2160
tttggtgggg tttttataat taggaaagaa tagttttgtt ttttttttatg attaaaagaa   2220
gaagttatat ttttttttatg atattaaata ttttgattta atttggtagt taggaaggtt   2280
gtattgtgga ggaaggaaat ggggtggggg tggatttttt tttaatagag tgaatgtatt    2340
taaatatgtt tttgttggta ggtgggggag tgtggttggg agtagggagg ttggagggtg    2400
gtgtgggggg taggtgggga ggagtttagt tttttttttt tgttaatgtt ggttttggtg    2460
agggttgttt ttggttggtg tttttgggggg agatttaatt tggggtgatt ttaggggtgt   2520
tatatttgtt aagtgtttgg agttaatagt attttttttg agtatttgtt tatggtgttt    2580
ttttgtttgg aaagatattg tggttttttt agaggatttg agggataggg ttggaggggg    2640
tttttttgtt agtattggag gaagaaagag gaggggttgg ttggttatta gagggtgggg    2700
tggattgtgt gtgtttggtg gttgtggaga gggggagagt aggtagtggg tggtggggag    2760
tagtatggag ttggtggtgg ggagtagtat ggagtttttg gttgattggt tggttatggt    2820
tgtggtttgg ggttgggtag aggaggtgtg ggtgttgttg gaggtggggg tgttgtttaa    2880
tgtattgaat agttatggtt ggaggttgat ttaggtgggt agagggtttg tagtgggagt    2940
aggggatggt gggtgatttt ggaggatgaa gtttgtaggg gaattggaat taggtagtgt    3000
tttgattttt tggaaaaagg ggaggttttt tggggagttt ttagaagggg tttgtaatta    3060
tagatttttt tttggtgatg ttttgggggt ttgggaagtt aaggaagagg aatgaggagt    3120
```

tatgtgtgta tagatttttt gaatgttgag aagatttgaa ggggggaata tatttgtatt 3180
agatggaagt atgtttttta ttagatataa aatttatgaa tgtttgggat aaaaagggag 3240
ttttaaagaa atgtaagatg tgttgggatt atttagtttt taatttatag atatttggat 3300
ggagtttatt ttttttatta ggagggatta ttagtggaaa tttgtggtgt atgttggaat 3360
aaatattgaa tataaatttt gattgaaatt atttagaagt ggttgggtgt ggtgttttat 3420
gttttgtaat tttttattt tgggagatta aggtgggggg aattatttga ggttgggagt 3480
ttgagattag t 3491

<210> 197
<211> 1145
<212> DNA
<213> Artificial Sequence

<220>
<223> chemically treated genomic DNA (Homo sapiens)

<400> 197

gggtatggag gtattgtgtt atttagggta agattttgtt ttttttttag tttttttttt 60
aggatattta atattttgtg aaatttagag attttgtttt agttggattt agagaaattt 120
agtgggaaag gagaggttaa aggttgaatt taatggtgta aggttttatg gtttggttat 180
tttttgtttt gatgttgtgg ggttagtggg agaagaaagt tagtgtgttt ttgggtgtag 240
gggttagtgg ggtttggagg tataggtatt ttgtgatatt ttaggttttt tgatttatgt 300
ttttggtagt tttgattatt tatagtttta gtagagtatg gggtgggggt agaggggttt 360
gtttgggagg gttgttattt ttttaaaattt ttgtgggttg tttagttata gttttttttg 420
tttgggtgtg ttttttgttt gttttttttt tttgttttag gttattgttt ttaattttga 480
ataaaaattg tagttaattt ttgaggtagt tttattgttt agtggatttt agtttttgtt 540
aggtttggtt tgttattttt gttttgtttt ttgttggttt ttgttttgtg tttagggatt 600
ttttagtttt ttttgtttgt gttttttgtt tgttttggat attatggata agttttggtg 660
gtatgtagtt tggggatttt gttttgtgtt gttgagtttg gtgtagattg gtgagtgttt 720
gttgtagttt gggtagtaag atgggtgtgg ggtgtttagt gtggatttgg tggtagtttt 780
tttggttgag ttggttttgg gggattggag ttaagtgagt tgtttgtgaa gtgtattggg 840
ttttggaaag tagggttggg atttgtgtta aattgttgga gaatgtgttt gtggaagtat 900
tatttggttg aaagaaaaag agaaagagaa gaaagtttgt tgggtaggtt gttggtgtgt 960
agttttgggt gaggttgtta gagttgtagt atatggtaga aagtaattgt ttttttggat 1020
gtgtatagtt gttgtttgga ttaataggtt tttgtgttta agggtttgtt aagttttatt 1080
gggttgtgtt taggtagggt agagttgggt ggggtagaga ttggggttgg aatagggtga 1140
gtggt 1145

<210> 198
<211> 1145
<212> DNA
<213> Artificial Sequence

<220>
<223> chemically treated genomic DNA (Homo sapiens)

<400> 198

gttatttgtt ttgttttagt tttagttttt gttttgttta gttttgtttt gtttagatat 60
agtttggtgg ggtttggtga gtttttgggt ataggagttt gttagtttag ataatgattg 120
tgtgtatttg ggagaatggt tattttttgt tatgtgttgt agttttagtg attttgttta 180
aaattgtgtg ttggtagttt gtttaataaa tttttttttt tttttttttt ttttttaatt 240
aaatggtgtt tttatagata tattttttaa tggtttagtg taaattttag ttttgttttt 300
tggagtttaa tgtattttgt agatagttta tttgatttta gtttttttagg gttgatttag 360
ttggaggggt tgttgttggg tttgtgttga gtattttgta tttattttgt tgtttaggtt 420
gtggtgggta tttattgatt tgtgttaggt ttagtggtat gaggtagagt ttttaggttg 480
tgtgttatta aaatttgttt atggtgtttg gagtgaatgg agggtgtggg tgaaaggagt 540
tggaggattt ttgggtgtgg ggtaggggtt ggtggaggat gggatgagga tggtggattg 600
aatttggtag aggttggggt ttgttgggta atgaggttgt tttggaagtt ggttgtagtt 660

```
tttatttgag gttgaaaata gtgatttaag atggagggag ggagtgagtg aaggatatat      720
ttaagtaagg ggggttgtga ttaagtagtt tgtagaggtt ttaagaagta gtagtttttt      780
tgggtgggtt tttttgtttt tgttttgtgt tttgttgagg ttgtaaataa ttggggttgt      840
tagggatgtg ggttggggaa tttggagtgt tgtggggtgt ttgtgttttt gagttttatt      900
ggtttttgtg tttagagatg tattggtttt tttttttgt tggttttgtg gtgttaggat      960
agaggatgat tgaattgtaa aattttgtat tattgggttt agtttttggt tttttttttt     1020
ttgttaaatt tttttgaatt tggttggagt aagattttttg ggttttatag gatgttggat     1080
attttgggag aggagttgga gagagggtga ggttttgttt tgggtggtgt agtgtttttg     1140
tgttt                                                                  1145
```

<210> 199
<211> 2221
<212> DNA
<213> Artificial Sequence

<220>
<223> chemically treated genomic DNA (Homo sapiens)

<400> 199

```
taatgatgat gtgtagtagg ttttttagag ttaagttggt gattaagata ttgggattgt       60
tttgtatgta tttgtttttg tagataattt ttagaagtgt ggagtttttg atgattttta      120
gtatgaatat aaggtaggat ataattgtgt tgatgtattt gaaagttttt ttgattttga      180
tgggtttttg gtatggggga ggggagatgg tgtgtggtgg agattttgtt gttttgtttt      240
ttttaggtat ttttgtaggt gttaatgatt gtgttagatt ggtgttggaa tttttgggtt      300
ataaggtttt agtgggtggt gttattattt ttgtggtttg taaaagtgga gtggttttgt      360
taggtgggaa gttttttttt ttttttttaga tttgtgatag gttgtaggta agaattagtg      420
taattagggt gtgtttgtat agatttggag gtggttgtat gttgttattt gttttagaag      480
gtgtttggtg gttgttttgt agttttagag tttagagata agtagaggaa ggaagatagg      540
atatttgggt tagttgtttg agttaagttg ttgtaaatgt taatattgtt tgtagttttt      600
ttgttagtat ttatgtttaa aagtaattta agtttgtgtg ttagtgggaa atttggtgtt      660
tatgatttgt tagtgtgggt tgaaattttt ttttgatgtt tttttagttg tttttttttt      720
tttgtgtggt taggaggggt aaataatatg tagtggggtg gggtgttttg gaaggggtgt      780
gttagggagg gaatgatggg ggttttaggt aaagttttga attttttttat gtaatgggag      840
gaatatagat atgttttagt taagtgtgtg tgggaattgt ggaattaagg tatttagagg      900
ttaagggttt gtgttaagtt ttgtattttg ttttgtaggg gaatttttat attttgtgat      960
tgaatttgaa agattttttt tggtggatga aagttgttat tttttggttg gttgagttat     1020
agtttttgta gtgtgtttag gagtgtgttg gagatttgga aatttgtaga gatttttttaa     1080
gttagtagga atttggaaat tgttgttttt tttaaatggt tttaaatatt tgtgtttttt     1140
tttttattta atttaatttt aatttgtttt ttttataggt ttaatttttta tttttttaaa    1200
attttaaatt agtaagttaa attgaagttt tagaataagg tttaattaat gattttaatt     1260
taatattaag tttgaatgtt tattttttgg atttgataaa ttagatgtag agtgatgaat     1320
ggagattatt tttttgggag gtggaattta gttgggtttt agtttgtttt gggagaggag     1380
tatgtttttt ttgagttgta ggtgggtttg gttttgatga aatgttgtga gaatgttaga     1440
tagtgtagtt tttattgttt gtttggggtt tttgttgtta ttagataagt atttttattt     1500
atttattttt ttttattaat tattgttaga ttttttttgt gtttttttgat tttgttgtaa    1560
tttttttttt tgggtgatgt ttggatagta ttagtagtag ttgtttgagg gaggggggta     1620
gtttttgttt ggggataggt tttaaaaggt tgtttatttg ttagagtttt atgtttttta     1680
aatgaattta aattaagggt aagttattat gtattatgtg atatttggta gttttagatt     1740
agaataaggt tttgtattga atgtgataat tgaattttta atgttaagtg ttattttttt     1800
tttaggtaaa tgttttgttg ttgtgatatt taaggttggt ttgtgggtgt tgttttttgt     1860
tttgttttat tttgttttga agttttattt taagttaata ttaattttaa tatggaatag     1920
tttgattttt tttttttagg tttttttatt gttttgagag aggattataa gtatttttta     1980
tttttttttag tatagttttt tttttttttt tttgttatag tattggtgtt aggtaatttt    2040
aggtattttt atattgtatt tatgatagat ttgatgttta ttggagatat gaaaataatt     2100
agaaagttgt tatgtaaatt taatatgata tttatttatt aggtagagta atataataag     2160
gttttttttat ttattttttga gttttatgtt tattgtagag aggaaaataa tagtgattgg   2220
t                                                                     2221
```

<210> 200
<211> 2221

<212> DNA
<213> Artificial Sequence

<220>
<223> chemically treated genomic DNA (Homo sapiens)

<400> 200

```
attagttgtt gttatttttt tttttgtggt aaatatggag tttaagaatg gatagaaaga      60
ttttattgta ttgttttatt tggtaaataa atattatgtt gaatttgtat ggtagttttt     120
tgattatttt tatgtttttg ataagtatta agtttgttat aggtatagta taaaaatgtt     180
tgagattgtt taatattgat attgtggtag gaagagagga gaagagttgt attggggaaa     240
atgaggagta tttgtaattt tttttgggg tagtgagagg gtttgggaag aaaagattga     300
gttgttttgt gttggggtta atgttggttt gaaatgaaat tttagggtaa aatgaagtag     360
agtaaagagt agtgtttata aattaatttt aaatattata gtggtggaat atttatttga     420
aagaaaagtg atatttggta ttaaaagttt aattattatg tttagtgtag agttttgttt     480
tagtttggag ttgttgggtg ttatatggtg tgtgataatt tgtttttgat ttgggtttat     540
ttgaagagtg tagaatttta ataaataaat agtttttttgg gatttgtttt tggatgagga     600
ttgttttttt tttttgggta attattattg atgttgttta ggtattgttt aaggggaaag     660
gttgtagtgg ggttggaagg tgtgggagga gtttggtggt gattgatggg aagggatgaa     720
tgaataaaag tatttgtttg atggtagtag agattttgag taaatggtgg aggttatatt     780
gtttggtatt tttgtagtgt tttgttagag ttggatttgt ttgtagttta agggaggtgt     840
gttttttttt tagagtaggt tggaatttag ttgggttttg tttttggga aggtggtttt     900
tatttgttgt tttgtatttg gtttgttaga tttgagaggt aaatatttgg gtttggtgtt     960
gaattaaaat tattgattga attttatttt ggggttttgg tttggtttat tagtttggga    1020
ttttaaaaaa ataaaaatta agtttataga gagggtaaat taaaattagg ttgggtaaag    1080
gaaggagtgt gagtgtttga agttgtttgg agggaatagt ggtttttaag tttttgttga    1140
tttgagaagt ttttgtgggt ttttgaattt ttggtgtatt tttgggtgtg ttgtgggagt    1200
tgtagtttag ttagttaggg agtagtggtt tttatttgtt gggaggagtt ttttgagttt    1260
aattgtgggg tatagaggtt tttttgtggg gtaaaatgta gagtttgata taagtttttg    1320
gtttttaggt gttttaattt tgtggttttt atgtatgttt aattaagatg tgtttgtatt    1380
tttttgtta tgtgaaagag tttggagttt tgtttgggat ttttattatt tttttttttgg    1440
tatatttttt ttagaatgtt ttgttttatt gtatattatt tatttttttt ggttatgtgg    1500
gggaagaaaa atagttgaga gggtattagg aaggagtttt gatttgtgtt ggtgagttat    1560
gagtgttaag tttttattg gtgtgtaaat ttgagttatt tttgagtgtg gatattggtg    1620
aagaggttgt gggtggtatt agtgtttgta gtgatttggt ttgggtagtt gatttaagtg    1680
ttttgttttt ttttttttgt ttgtttttag gttttgaaat tgtggagtgg ttattggatg    1740
ttttttggag taggtagtag tatgtagttg tttttaagtt tgtgtggatg tgttttggtt    1800
gtgttggttt ttgtttgtgg tttgttgtgg atttggggag aggagagagg tttttttgttt    1860
gatagggtta ttttgttttt gtaaattgta gagataatga tgttatttat taagatttta    1920
tggtttaagg gttttaatgt tagtttggtg tggttgttgg tatttgtgga ggtgtttaaa    1980
ggagatagga tggtaggatt tttgttatgt attatttttt ttttttttgtg ttaaggattt    2040
attgagatta aggagatttt taaatatatt aatatggttg tgttttgttt tgtgtttgtg    2100
ttggggatta ttgggaattt tatatttttg agaattattt ataagaataa gtgtatgtga    2160
aatggtttta atattttgat tgttagtttg gttttgggag atttgttgta tattgttatt    2220
g                                                                    2221
```

<210> 201
<211> 2172
<212> DNA
<213> Artificial Sequence

<220>
<223> chemically treated genomic DNA (Homo sapiens)

<400> 201

```
atatggtagg tgtttaataa atgttagttt ttattggtat tattattatt attggtgtta      60
atgtatttat ttattgtagt gaggtagaat gtttttgtgg ttatgagtgt gattaggttg     120
atttattatt aagaggtgga ggatagggaa ggttgtatag tgttttatta taaggaaaag     180
aggattagta tgtttttttt tgggaaggga aaaaaaaaga gaaggaaatt gaaagtatat     240
```

```
ggttgatttt tttttaatta taaagtattg gatttatggg agggtatgtg ggggattgtg     300
taagtatata ggggtgtggg ggaggaaggt taatgaatag atttattgta aagggagggg     360
tgatgaggtt tattgtttga ggggaagggg agtagaaaat tttatagatt tttgtattat     420
agtgagagga gagaaaagtt attatataga ttgatttaaa gtagagaggg aaaaggttat     480
ggattgagtt attgaaaata gggagatgta aagtattaaa ttaatttatt attaaaaagg     540
agagaatttt aagtgttaga gtgatagttt atgaaaaaga ggagataggg taatggattg     600
atagttaaag ggatgtgaat ttaaatatga agattgattt attataaaat ggtggtggtg     660
gtggtggggg ggggggtgg ttttgaaaaa agttatggat tgatgtattg ggggattgga     720
ggaggtagga atataaattt aggtagattt attgttaaag agggatgtgg aggagtgtgg     780
tgttatggat tgatttgtta tgatgtatag gaagggtgga ataaataagg ttatggatgg     840
gttttttgaa agttgaggtt aggagaaatt taaatattta gatatattta ttattaaggg     900
ggttgaatat tgttataggt tagtttttta taagtggggg tgggtggggg gtgggtgggg     960
gtaggggtag ggggaaagtt tatggatgag tttattttaa gttggtgtgg ggaaaaatat    1020
aagtatgtag attgatttat tatataatgg gagggggagg ttaggttatg gttagattta    1080
ttataaggga tgaagggttt gagtaatgtt atatgtaggt ttattagttg tggtggtggt    1140
ggtggggagt tgaatattag taggttattg gttaagttat attttttgtg tgggggtggg    1200
aaggatatgt ggggttatgg attgggtttt ttattttttta ggtttagagt ttatttgtgt    1260
gtagtgtggt ggtggtgttg gtaatgttgg tagttttggg gggtgggggt tttatatgtg    1320
gttttattgt tttttaggtt tgggttttga ggtggtggtg gaggtggtgg tggtggtggt    1380
ggtggtagta tttagaagtt gtttttgtgt tttttatag tttatgtggg tttgaggagg    1440
aggagtttaa tgttttgttg tttgttgatt ggttaaagtg ttattaattg gttgtaaggt    1500
tttgaggggt tgggggatgg gttttttttt atagagttgt gttgtgattg gtgtaagggg    1560
aaatgatgga atgtttatgg tttttggagt tttgaggagg tgtgtttggt ttttatggtg    1620
ttttatgttg attggtttaa ggattgatgg attgtgagta attgaaaagg tttggggttt    1680
gtgttggggg ggtgggggtt tgttttatat tgattggttt atatgggatt gatggaagat    1740
agtttttaag gatgggggtg ggtggttttg tttttttttg attggtggat gagggatttt    1800
tagatttttg ttaaaatatt aagggggtgg tgttgtatgt tatgttgttt agtggaagta    1860
gggtttgtat agaaatgggt gtagtagttg gtgggagtgg gttggattgg gttttgtgta    1920
ggtgggtagg gtagttttat ggttagataa gattatagag ttgggtaaag atgaatttag    1980
aatatagtgg aggtaggagg gtaggatggt tgttaggtat atgaaagagt attgagtggt    2040
agaaatgaaa tgtttttaga gggtagggtt gaattgtggt tagagttaaa aaggggagga    2100
atttggtttt gtattgattg gtggtggttg gatttaattt agaatagggg tttgattagg    2160
gagagtggga tt                                                        2172
```

<210> 202
<211> 2172
<212> DNA
<213> Artificial Sequence

<220>
<223> chemically treated genomic DNA (Homo sapiens)

<400> 202

```
gattttgttt tttttagttg ggtttttatt ttaggttgag tttagttatt gttaattaat      60
gtagagttag gttttttttt tttttaattt tggttgtagt ttaattttgt tttttgaaag     120
tattttgttt ttgttattta atgttttttt gtgtgtttga tagttatttt gttttttttat    180
ttttgttgtg ttttaaattt gtttttattt agttttatgg ttttgtttaa ttgtagagtt     240
gttttgtttg tttatgtgga gtttagtttg atttattttt gttggttgtt atgtttattt     300
ttatataagt tttgtttttg ttgagtagta tggtgtgtga tattgttttt ttggtgtttt     360
ggtaggggtt tagaagtttt ttgtttgtta attagagaaa aatagggtta tttatttttg     420
tttttggggg ttgtttttta ttaattttat gtaagttaat tagtgtgagg tagatttttg     480
tttttttgat ataggttttg agttttttta gttgtttata gtttgttagt ttttgagtta     540
attggtgtgg agtattgtga aggttgaatg tgttttttttg ggattttagg ggttgtgagt    600
gttttattat ttttttttat gttaattatg gtatagtttt gtagggaagg gtttgttttt     660
taattttttg aggttttgtg gttgattaat agtgttttgg ttaattagtg agtggtggga     720
tattgggttt tttttttttg ggtttatgtg agttgtaggg aaatgtaggg gtggtttttta    780
ggtgttgttg ttgttattgt tattattatt tttattgttg ttttggaatt taggtttggg     840
gggtggtggg gttgtgtatg gagtttttgt tttttggagt tgttaatatt gttaatgtta     900
ttgttatgtt atatataggt gagttttggg tttggagggt ggagggttta gtttgtgatt     960
ttatgtattt tttttgtttt tgtgtagagg atgtggtttg gttggtggtt tgttggtgtt    1020
```

tgattttttg ttgttattat tatggttggt ggatttgtgt gtggtattgt ttaagttttt 1080
tgttttttat agtggatttg attgtggttt aatttttttt ttttgttgta taatggattg 1140
gtttgtgtgt ttatgttttt ttttatgtta atttagggtg gatttgttta taggtttttt 1200
ttttattttt atttttattt gtttttttatt tatttttatt tatagggagt tagtttgtga 1260
tagtgtttag tttttttaat agtaggtgta tttgagtgtt tggatttttt ttagttttaa 1320
tttttaggag atttgtttgt ggttttattt attttatttt ttttgtatat tgtagtgaat 1380
taatttgtgg tgttgtattt ttttgtattt tttttaata gtgagtttat ttgagtttgt 1440
atttttgttt tttttagttt tttaatgtat tagtttatgg ttttttttaa aattattttt 1500
ttttttattt attattatta ttattttata gtggattagt ttttatgttt ggatttatgt 1560
ttttttagtt attagtttat tgttttgttt tttttttttt gtagattgtt gttttagtgt 1620
ttggggtttt tttttttta atagtgggtt agtttagtgt tttatatttt tttattttta 1680
gtagtttagt ttatagtttt tttttttttt gttttgaatt agtttgtgtg atggtttttt 1740
ttttttttta ttgtggtgta ggagtttgta aggtttttttg tttttttttt ttttagatga 1800
tgagttttat tatttttttt tttgtagtgg atttgtttat tggtttttttt tttttatatt 1860
tttgtatgtt tgtatagttt tttgtatatt tttttgtgag tttagtgttt tgtaattggg 1920
ggaagattgg ttgtgtattt ttaatttttt tttttttttt tttttttttt tagaagagaa 1980
tgtgttgatt tttttttttt tgtgatggag tattgtatgg ttttttttgt tttttatttt 2040
ttaatagtgg gttagtttgg ttatatttgt aattataaag atgttttgtt ttattataat 2100
aagtgaatat attagtgtta gtgatgatag taatgttaat aggagttagt gtttattgag 2160
tatttgttgt at 2172

<210> 203
<211> 3289
<212> DNA
<213> Artificial Sequence

<220>
<223> chemically treated genomic DNA (Homo sapiens)

<400> 203

tttgtgttta tgtattatgt gtgttttttt gttttttttt ttttttatga gtgagaaaaa 60
aaagtgttta aatttttatt aatataaatt aatgatatat aatgatgaaa ttttgttttt 120
atttttgttt gtgataggga atgtaaaaat agtaagtggt ttagttttat gaatttttgt 180
tttttgtttt ttttgttttt gttgggttgg atttttaaga atggaggtta gtgtatagtt 240
ttgtgtgggt tgtttagttt ttggatttgg tggatgatgt taggtgatgg gagtggttgt 300
ggttgggttg gggaggttgt ggtttagggg agttgggagg gagggtggtt ttgttaggtt 360
gatggtgtgt ttggttgtgt ggtgttgttt ggagatggtt ttggtggtgt tgtgttgttg 420
taaatagttg tttttttgtt attatttata gtaggatttt ttggtttttg ggtgtggtgg 480
ttggaggtag gtttgtggtt tggtttttttg tgtgttttga attatttgtt tgttggtttt 540
atttgttttt gttttttttt aggtgtttat tgtgggtttt gatttttggg tttgaagagt 600
ggagaaggga agattggggt tgtgtgggga tatgtgtttt tgtgttttgg aggtggttag 660
tgtgttgggg ttgagttttg gtagtgtgat tttggttgtt ttatgtagta gggtaggaga 720
ttggggggtg tggtatattt tggagtattt tgtttttttta aagttttgtg ttttaggatg 780
tggagttgtt tttggggttt tagtagttga ggtattttgt ttaggtgtag ttggatattg 840
ttttttttagt ttttgttttt tattttttaa gtttgtgttg gaaaattatt tgttgtgggt 900
ttttgtaagt atagtttttt ggtgggattg aattagtttt tagtgtagat ttgagttttt 960
tgtaggaagt atattttgtt ttgttatttt gaattgatta ttttgtttat ataattatat 1020
tttgtatttt ttatttttgg ggtttagttt agaattgggt agatattttt tttaaatgtt 1080
tttgtatgta ggttttgtat agtgtttatt tgttggtgtt ttagggattt gatagttttt 1140
ttaatatttt tatatatggt tgagaaaaat aaataaataa atgtgttgtt tttttaaaa 1200
aaataaataa ataaagtatt tagtattgta aagtaggtta ttgtattttt ttattttgga 1260
tttttttattt tttgttttta aatgtaggaa tagtgttagt attgtttgag tttgagggtt 1320
ggaggttagg ggatgaaggt ttgtttttat gttttgtatt gaattagggt tagaattggg 1380
gatgggggta ggggtgtatt tttttgggag ttgaggttta agtttttggg gttttgtatt 1440
tgatgttgtt tttttttattt ttgagtttta gaattgtttt tagtttttgt ataagggtaa 1500
aaaggtgttt tttgttttat tttttttgat tttgggaata agggtttgta ttgaattagg 1560
tgtgaatgtt ttttttttatt ttgtgttgtt tttgtttttt ttttttttagt tgtggttttt 1620
gttttttttt gtattgtatt tttggtgttg gttgtagttt gtgagtagtt tttgttaatt 1680
tttttttttt tatataggat gtttatatta ggatatttgt gttagtaggt ttttatggtt 1740
ttttttttgta gttttggggg gagttatttt tgaaattttt tattttgggg ggtttatgag 1800

```
atttttgaga taggaattgt gaaatgttta tgagattagg atatgtgtta aggtgggggt    1860
agggagttgt gagtgttggg gatgtagttg ggtggttgta gaagtgttta ggtttgtgtg    1920
ttattttttt ggtgttattg tggttgagtt tgtgatgttt atatttattt ataaaatgtt    1980
tgttataaaa gtagtggttg tggtgttttg tattttaatt gtatttgtag tgagtatttg    2040
agaagttaag attgagttgg tggttgtggt gtagtgaatg agtagtgatt gtgtttttat    2100
ttagttttgt tttatagtgt ttatttgttt ttgtttttttg gtttttttgtt tggttttgtt    2160
taattgttat gatgatgttt ttgggtttta atgtagatta tgaggtgtta ttttttttgtt    2220
gtagtagtgt gtttttggtt ggggatagtt ttttttatta ttatttattt gtagattttt    2280
tttttagtat gggtttgttt gttaatgtgt aggtaaggtt ggtttttttgt tgttgtgggg    2340
ttgggggttt ggggttgtgg aggaggagat attgggtggg atgttttagt agatgagtag    2400
ggggtttttt tgtgtttgga gggaggtgt tgtggttgga gtggtgttgg tttgggggtt    2460
tgggatttgt tttgagtgta tgtatgtttg ttatagtaag aattggtttt ttttttggga    2520
ggtaggtttg ttttgagtaa tttttggttt gtattttagg atggattttt gatattagtt    2580
ggagtagatg tgttttaagt ataaatttgt taattagagt ttggtttttt tggggaggtg    2640
gtagaaagtg gtaatttttt tttttttggt agtttggagt atggaggagg gatgagggag    2700
gagggtgtag tgggtgggtg tgtaaggtag ttttattgat aaaaagtgag tttattttgg    2760
agattttgga gtggtgtttg tgttagtgta gatgttaggg atatttataa taaatttttt    2820
tttaagtaag tgatgttgaa gggataatgg gaatgtagtg gtaggatgga agagataggt    2880
attgtgttgt ggaatgtttg ggaggaaaag gggagatttt tttatttagg atgagggata    2940
tttaagatga aatgtttgtg gtaggattgt ttttttttat tgttgtatgt ggtattggga    3000
atttgtttta tttgtgtttg gaatttgttt gtttatgttg gttttttttt tttgttttgt    3060
tttaggattt ttgtatggat ttggttgttt ttagtgttaa ttttattttt atggttattg    3120
ttattttgat tagtttggat ttgtagtggt tggtgtagtt tgtttttgtt ttttttgtgg    3180
ttttattgta gattagagtt ttttattttt ttggagtttt tgtttttttt gttggggttt    3240
attttagggt tggtgttgtg aagattatga taggaggttg agtgtagag                3289
```

<210> 204
<211> 3289
<212> DNA
<213> Artificial Sequence

<220>
<223> chemically treated genomic DNA (Homo sapiens)

<400> 204

```
ttttgtgttt ggttttttgt tatggttttt ataatgttag ttttggagta agttttagtg      60
gagggggtgg ggattttgaa agggtgaggg gttttggttt gtgatggggt tatggaggag     120
atgagggtgg gttgtattag ttattgtagg tttggattgg ttgagatggt agtgattgtg     180
ggaatgaagt tggtattgga gatggttagg tttgtgtaga agttttagaa taaaatagaa     240
ggggaaagtt gatgtgagtg agtaggtttt ggatataggt ggggtgagtt tttagtgttg     300
tatgtagtag tgaagagaaa tgattttgtt atggatattt tattttaaat gtttttttatt    360
ttggatgaaa ggtttttttt tttttttttt aggtattttg tagtgtagtg tttgtttttt     420
ttattttgtt gttgtgtttt tgttattttt ttagtattat ttgtttgaaa gggggtttgt     480
tataaatatt tttgatgttt gtgttgatgt aggtgttgtt ttggagtttt tagaatgaat     540
ttgtttttta ttaatgaaat tgttttatat atttgtttgt tgtatttttt tttttttattt    600
tttttttgtg ttttaggttg ttgggggagg ggggattgtt gtttttttgtt atttttttgg    660
agaagttagg ttttagttag tgagtttgtg tttgggatat gtttgtttta gttaatgtta     720
gagatttgtt ttggagtgta gattagaggt tgtttagaat gaatttgttt tttgaagggg     780
gaattaattt ttattatggt aagtgtgtgt gtgtttagag taagttttga gtttttgagt     840
tggtattgtt ttggttatgg tagttttttt ttaggtataa gggagttttt tatttattta     900
ttggagtgtt ttgtttggtg tttttttttt tgtgatttta agtttttggt tttgtggtga     960
tgggaagtta gttttatttg tgtgttgata ggtgagttta tgttggagaa ggagtttgtg    1020
ggtgagtggt agtaagagag gttattttttg gttggggatg tgttgttgta gtgggaggat   1080
gatgttttgt agtttgtgtt gaagtttgag aatattattg tggtggttag gtaaagttgg    1140
gtgaggggtt gaggggtgga gataggtggg tgttgtggag tagagttggg taggagtatg    1200
gttattgttt gtttgttgtg ttgtggttgt tggtttagtt ttggtttttt agatgtttgt    1260
tgtagatgtg gttggagtat gaggtgttgt agttattgtt tttataataa gtgttttatg    1320
aatgagtgta aatgttatgg gtttaattat ggtggtgtta gaggggtggt gtgtgggttt    1380
gggtgttttt gtggttgttt ggttgtgttt ttagtgtttg tagtttttttg tttttgtttt   1440
ggtgtgtgtt ttaattttgt gagtattttg tagtttttgt tttagaggtt ttgtgggttt    1500
```

```
tttaagatga ggggttttgg ggatggtttt ttttagggtt atagggaaag gttgtggaaa    1560
tttgttgatg tagatgtttt aatatggata ttttgtgtaa ggggggaggg attgatggga    1620
attgtttgtg ggttgtagtt aatattgagg gtgtagtgtg gggggaggtg ggggttgtgg    1680
ttgggggagg ggaggtggga atggtgtaga atgagagaga atatttgtat ttggtttaat    1740
gtggattttt gtttttgagg ttggggggga tggggtagag agtgtttttt tatttttgta    1800
tggaaattga agatagtttt gaggtttaga gataggagaa atggtattga gtataggatt    1860
ttgaggattt aagttttggt ttttgaagga atgtgttttt atttttattt ttaattttag    1920
ttttaattta gtgtaaagtg tggaagtaga tttttatttt ttaattttta gtttttgggt    1980
ttgagtaata ttagtattgt ttttgtgttt ggaagtagaa agtggaggat ttaaaataag    2040
agaatatgat aatttatttt atgatattgg gtattttatt tatttatttt tttaaagaag    2100
atagtgtatt tatttattta ttttttttgg ttatgtgtgg gaatattaag gaaattgtta    2160
aatttttgag atattagtag ataaatattg tgtaaaattt atgtgtgaag atatttgaag    2220
ggggtgtttg tttggttttg agttgggttt taggggtagg gagtgtgagg tgtggttatg    2280
tgggtagggt ggttagtttg ggatgataag gtggggtgtg ttttttgtgg ggaatttaaa    2340
tttgtgttga gggttggttt ggttttgtta ggaagttgtg tttatgggag tttgtagtgg    2400

gtgatttttt agtgtggatt tggagggtgg aggatggggg ttggaaggat agtgtttagt    2460
tgtgtttggg tggaatattt tgattgttgg gattttagga gtggttttat gttttggaat    2520
atggggtttt ggggaggtaa ggtgttttag agtgtgttat gttttttaat tttttgtttt    2580
gttgtgtagg atagttgggg ttatgttgtt ggggtttagt tttagtgtgt tggttatttt    2640
tagggtgtga agatgtatgt ttttgtatag ttttggtttt ttttttttta ttttttgggt    2700
ttgggggttg gggtttgtgg tgggtatttg gagaggggtg aggtaggatg aggttggtgg    2760
gtggatagtt tggggtgtgt ggggagttgg attgtagatt tgtttttagt tgttgtgttt    2820
gggagttagg agattttgtt atagatagta atagggaagt ggttgtttat agtaatatag    2880
tgttgttagg gttgttttta ggtaatgttg tgtggttggg tgtgttgttg gtttggtgag    2940
gttatttttt tttttagttt ttttgggttg tggttttttt ggtttggttg tggttgtttt    3000
tgttgtttga tattatttgt tgggtttggg ggttgagtgg tttgtgtgag gttgtgtgtt    3060
aatttttatt tttagagatt taatttggta ggagtgggga gggtaggagg tggggatttg    3120
tggaattggg ttatttgtta tttttgtatt ttttgttata ggtagaggtg aaaatagaat    3180
tttattattg tatgttattg gtttatgttg gtgggaattt aggtgttttt ttttttttatt    3240
tataaaaagg gagggaaatg gaaaaatata tataatatgt aaatataga                3289
```

<210> 205
<211> 3812
<212> DNA
<213> Artificial Sequence

<220>
<223> chemically treated genomic DNA (Homo sapiens)

<400> 205

```
agtgagattg tatggtttta tttataagtg ggagttgaat aatgagaata tatggttata      60
tggtggtgat taatatatat tggtgtttgt tgagtggggt gttggggagg gagagtatta     120
ggaagaatag ttaagggata ttgggtttaa tatttgggtg atgggatgat ttgtatagta     180
aattattatg gtgtatatat ttatgtaata aatttgtata ttttttatat gtattttaga     240
attttaaata aaagttggat ggttaggtgt ggtggtttat gtttgtaatt ttagtatttt     300
gggaagttga ggtgtgtaga ttatttaagg ttaggagttt gagattagtt tggttaatat     360
ggtgaaattt tgtttttatt aaaaatataa aaattagtta gatgtggtat gtatttataa     420
ttttatttat ttgggaggtt gaagtagaat tgtttgaatt tgagaggtgg aggttgtagt     480
gagttgttga gattgtgtta ttgtatttta gtttgggtta tagtgtgaga ttatgttata     540
aaataaaata aaataatata aaataaaata aaataaaata aaataaaata aaataaaata     600
aaataaaata aaataaaaaa ataaaataaa ataaaataaa ataaagtaat tttttttttt     660
ttaagtggtt tttatttttt ttttttgttt tgtgaagtgg gtgtgtaagt tttgggattg     720
tagtggtttt agggaatttt tttttgtgat gttttggtgt gttagtttgt tgtgtatatt     780
ttgttgtggt tttttttttg ttgtttgttt attttttagg ttttgttggg gatttgggaa     840
agagggaaag gtttttttgg ttagttgtgt ggtgattttg gggattttag ggtgtttttt     900
tgtggttgat gtttggggtg tagtggttgt tggggttggg gttggtggga gtttgtggga     960
tttttttagaa gagtggttgg tgttgtgatt tagtattggg gtggagtggg gtgggattat    1020
ttttataagg tttggaggtt gtgaggtttt tgttggagtt ttgttgttgt agtttttgtt    1080
attagtgagt atgtgtggtt tgtgtttttg gggatggggt ttagagtttt tagtatgggg    1140
```

ttaatttgta gtattaggtt tgggtttttg gtagggtttt ttgtttattt tgagatttgg 1200
gatgggggtt taggggattt aggatgtttt tagtgttgtt agtggttttt agggggtttg 1260
gagtgttttg gggagggatg ggattttggg ggtggggagg gggggtagat tgtgtttatt 1320
gtgttttggt attttttttt gggttttagt aaattttttt ttgtttgttg tagtgttgtt 1380
ttatattgtg gtttattttt tagtttgagg taggagtatg tgtttggtag ggaagggagg 1440
taggggttgg ggttgtagtt tatagttttt tgtttatttg gagagatttg aattttttta 1500
tttttttgtt gtgtggtttt tattttgggt tttttttttg tttttttgttt tttttgttat 1560
gtttgttttt tgttttagtg ttgtgtgaaa tttttggagg aatttgtttt tttgtttttt 1620
ttttgtattt ttgatttttt tttgggttgt tgtgaggtgg agttggtttg gtttttatat 1680
tttgtatttt tttttttttg taggttgttg tgtggttttg tgtatgttgt tggtagatta 1740
gggttagagt tggaaggagg aggtggtgat tgtggagatg tggtaggagg gtttatttaa 1800
agttttttgt gtaagtgatt atgtttgggt aaggggaggg ggtgttgggt tttagggggt 1860
tgtgattagg attgggggat gtttaagttt agtgtttttt tttgagttat gttttttttta 1920
atagttatat gggtagtttt ttaagtttta ggatggagat tttattttgt attagtttaa 1980
tattattttg tgttatttgg gttgtatttt tggtgagttt tgaattttta agtttagggt 2040
aggtatgggt aagtttttgt ttttggagtt tttttgttta aattagttgt tttgtagttt 2100
tttggagtgg aggaaattga gatttattga ggttatgtag tttgtttaag gttaagtttg 2160
ggtgtttgta atttttgttt tgtgttaggt tgttttttag gtgttaggtg agttttgagt 2220
atttgttgtg tggtagtttt ttattttttt atgtatattt tttttttttt tttttaggtt 2280
ggggtttata gatagttttt tggttggttt atttttagtg attgtgtgtt gattaggtgt 2340
ttagttatgt ggtttgtttt tttttattta attttagggt tttatgggaa ggattagtag 2400
gaggtagttt tggtggatat ggtgaatgat ggtgtggagg atttttgttg taaatatatt 2460
ttttttattt atattaatta tgtgagtatt tgtattaggg ttgggtattg ggggttgaat 2520
aaagaaaggg gtttttttgtg tttttatttt ttttattttt taggtggttt gggttgattt 2580
tttttttgggt tagggtgtag gggttgggtt agttttgggt taggggttta ggggtttggg 2640
ataagatata atttgtattt ttattgtttg ggatattaat tagttaagta atgggttatg 2700
ggggtgagtg taaggataga gatttttagt aattggtggt ttttgatttt ttggggtggt 2760
gagggttttt tggagtagtt agaggtggag gaggatttgt tgttagtttt tggatggagg 2820
tgttggtatt tttagttgag gaaaatatgt agatatagag tatatttggg gatttgggat 2880
tagtttagta gaggtagtgt gtgtgtgtgt gtgtgtgtat gtgtgtgtgt gtgtgtgtgt 2940
atgtttgtat ttgtgttggg tgggtaagga gatagagatg ggtgggtagt aggtttaggt 3000
tttgaaggtt ttgaatttat tggtttggag ttttttaagg gtaatggggg ttattgagaa 3060
gtttgaatag ggttgtgttt gaatgtgagg tttagaagga ttttttagag aagttagttt 3120
taaagttttt gtaattattt ggtgagagaa tttagtaagg atggataggt agaatggaat 3180
agagatgagt tggtagttga agtggatagg atttggtatt agtttggttg tggggagtaa 3240
gtagaggaga atttgggatt ttggtgtttg gtttggggta gatgggggtg ttttaggggt 3300
tgggagggat gagagtagga tgatatatgg tggtgtttgg taggaggtgg gtaaggatga 3360
ttatgtgaag gtattgtttg ggtaattgaa gttttttgag attttgttgt tttagaatta 3420
gggaggtaag atttttattg tgggagatta ggtgagtatt tggttttatg ttgttttttt 3480
tttgttattt tttgttttta gatggatata ggtgtgagtt atttgtttag taaagtagag 3540
tagatttagg ggatgggttt aggttttttg tttttaattt ttttagtttg tttttgttgg 3600
ttgagttttt ggtttttttg ttttgtagat tttttttgtt gattataatt tgttggattt 3660
gttgttgatt tatgaggttt tagtttttgg ttgtttggat gtgttttttt tgtttttagt 3720
atatgtgggg tgttttagtg tttggtttaa gtttaaggtt tttttggttt tttttgagta 3780
tgtgaatttt tttattaatg gtaatgggaa at 3812

<210> 206
<211> 3812
<212> DNA
<213> Artificial Sequence

<220>
<223> chemically treated genomic DNA (Homo sapiens)

<400> 206

gttttttgtt gttattgatg gggaggttta tgtatttagg ggaggttagg aaggttttga 60
gtttgggttg ggtattgagg tgttttatat atgttgagag tagggggaat gtatttaggt 120
agttaggggt taggatttta tggattagta gtaagtttag taggttgtag ttagtgaagg 180
agatttgtag ggtagggggg ttagggattt agttagtggg agtaggttgg aggaattggg 240
ggtagagggt ttaagtttat tttttaggtt tgttttgttt tgttaaataa atggtttata 300

```
tttgtgttta tttggaagta gagggtggtg aggaaggaat agtatggggt tagatgttta     360
tttggttttt tataatgaag gttttgtttt tttggttttg ggatagtagg gttttaaaag     420
gttttagttg tttgggtagt gtttttatat agttattttt gtttgttttt tgttagatat     480
tattatgtat tattttattt ttattttttt tagtttttga gatatttttg tttgttttag     540
gttagatatt agagttttag attttttttt gtttgttttt tataattagg ttagtattaa     600
atttgtttta ttttagttgt taatttattt ttattttatt ttgtttgttt atttttgttg     660
ggttttttta ttagatgatt gtaaaagttt tagagttggt ttttttggag gatttttttta    720
gattttatat ttagatatag ttttgtttag atttttttaat ggttttttatt gtttttagga  780
gattttaaat tagtgggttt aaggttttttg ggatttgggt ttattgtttg tttattttta   840
ttttttttatt tatttgatat aaatgtaagt gtatatatat atatgtatat atatgtatat   900
gtatgtgtat atatgttgtt tttgttgaat tggttttagg tttttaaatg tgttttgtgt     960
ttgtatattt tttttagtta aaagtgttag tatttttatt tagaaattgg tgataaattt    1020
ttttttattt ttggttattt taggaagttt ttgttatttt aggagattag aaattattag    1080
ttgttggagg tttttgtttt tgtatttgtt tttatgattt gttatttggt tggttgatgt    1140
tttaggtaat aagggtgtag gttgtgtttt gttttaggtt tttgggtttt tggtttagag    1200
ttgatttagt ttttgtattt tgatttaaga aggggttagt ttaagttatt tgaggggtaa    1260
gggggggtgag ggtataagaa gttttttttt ttgtttagtt tttagtgttt aattttggtg    1320
tagatgttta tatagttggt gtagatgagg gagatgtatt tgtagtggag gtttttttatg    1380
ttgttattta ttatgtttat tagggttgtt ttttgttggt ttttttttata gagttttggg    1440
gttgggtgga ggggagtagg ttgtgtgatt gggtgtttga ttaatatata gttattgggg    1500
atgggttaat taggggggttg tttgtgagtt ttagtttggg aggaggggaa gaggatgtgt   1560
gtggaaggat gagagattgt tatatagtag gtgtttagag tttatttgat atttgggagg    1620
tagtttggta tagggtaagg attgtaggta tttaggtttg attttgggta aattatgtaa    1680
ttttagtggg ttttagtttt ttttatttta gagggttgtg gggtagttga tttaaataaa    1740
agggttttgg gggtagaggt ttgtttatgt ttgttttgga tttggaggtt taagatttat    1800
taagggtgtg gtttaggtga tgtaggatgg tattggattg gtatagggtg aggtttttgt    1860
tttggaattt ggggagttgt ttgtatagtt gttggggggag gtatggttta gggaggggta  1920
ttgagtttgg gtgtttttttg attttagtta tagtttttta aggtttagta tttttttttt   1980
ttgtttgggt atggttattt atgtaggagg ttttgagtga gtttttttgt tatgttttta    2040
tggttattat tttttttttt tagttttggt tttgatttgt tagtagtatg tgtagggttg    2100
tgtagtggtt tgtggggagg gagaagtatg agatgtgggg attgggttga ttttgttttg    2160
tagtaatttg gggaggggtt aggagtgtag ggagggaata gggaaatagg tttttttgaa    2220
gattttatat aatattgggg tggggagtag gtatggtggg agaggtgggg aataggaagg    2280
aggtttgggg taaaagttat atgatggagg gataaggggg tttggatttt tttgggtggg    2340
tgaggggttg tgggttgtag ttttagtttt tgtttttttt ttttgttaga tatatgtttt    2400
tattttgaat tgggaaatag attatggtgt agggtggtat tgtagtgaat aaagaaaagt    2460
ttgttggagt ttgggggagg atgttaaggt gtggtgagtg tagtttgttt tttttttttg    2520
tttttggggt tttatttttt tttgaggtgt tttgggtttt ttgaaagttg ttaatggtat    2580
tggggatgtt ttgggttttt taggtttttg ttttgggttt tgaggtgggt gaggagtttt    2640
gttgggagtt tgggtttgat gttgtgggtt ggttttatgt tgggagtttt gagttttatt    2700
tttggggatg tgggttgtgt gtatttattg gtggtgaaga ttgtggtggt gaaattttag    2760
tgaaggtttt gtggtttttg agttttataa gggtggtttt gttttgtttt gttttagtgt    2820
tgagttatgg tgttggttgt ttttttggag ggttttgtgg atttttgttg gttttagttt    2880
tggtggttgt tgtattttgg gtgttggttg tagaggggtg ttttggagtt tttggagttg    2940
ttgtgtagtt ggttggggaa gttttttttt ttttttttagg tttttagtgg ggtttaggga   3000
gtaaatagat agtaggaaga ggattgtagt gaagtgtgtg tagtgaattg gtgtgttggg    3060
atattgtggg gggaaatttt ttaagattgt tgtgattttg gagtttgtat atttgtttta    3120
tagggtaggg gagaggggtg gaggttgttt agaggaaagg aaattgtttt attttatttt    3180
attttatttt attttttat tttattttat tttattttat tttattttat tttattttat     3240
tttattttat tttgtgttat tttattttat tttatgatgt agttttatgt tgtggtttag    3300
gttggagtgt agtggtgtga ttttggtggt ttattgtaat ttttgttttt tgggtttaag    3360
taattttgtt ttagtttttt gagtaggtgg aattataggt gtgtgttata tttggttgat    3420
ttttgtattt ttagtagaga tggggtttta ttatgttggt tgggttggtt ttgaattttt    3480
gattttaggt gatttgtatg ttttggtttt ttaaagtgtt gggattatag gtgtgagtta    3540
ttatgtttgg ttgtttaatt tttatttgaa gttttggggt atatgtagag gatgtgtagg    3600
tttgttatat aggtgtgtgt gttatgatgg tttgttgtat agattatttt attatttagg    3660
tattaagttt agtatttttt agttattttt tttggtattt ttttttttta gtattttgtt    3720
taataggtat tagtgtgtgt tgattgttgt tatgtgatta tgtgttttta ttgtttagtt    3780
tttatttata agtgagatta tgtggttttg tt                                  3812
```

<210> 207

<211> 1267
<212> DNA
<213> Artificial Sequence

<220>
<223> chemically treated genomic DNA (Homo sapiens)

<400> 207

```
atgaattttg aaagttggat tgtagagtaa atgagtgtag ttaatttagt taggggtttg      60
taagagggag aaagagaaaa agattgtgga atggaaagtt ttttaattta agtttttttt     120
aaggggtagt tatttttttgt tttatagttt agggtttgta tgtgtttttt ttggagtgga     180
aaaatatata agttataagg aatttaatag atagaaaggt gtatagagga atttaaagtg     240
tgggttgggg ggtgaggtgg tgggtgggag gtgagtgggt gtaggtggaa tattgttttt     300
taagttaagt tgttgtaaat aaaaaggtgt aaagggagag aagttggtgt ttaatgtgag     360
ttaggagtag tgttttggtt ttttttttgt ttattttaaa agtatttttt gtattgtttt     420
taaggtgaga aataggaaag aaaatgttgg tttgtgtgtt tgttgtttgt tttttttggtt     480
gtttgttttt gtagggttgt tgggagtttt taagttttgt gagaattttg ggagttggtg     540
atgttagatt agttgggtta tttgaaggtt agtagtttgg gtagggttta ttgaaagttt     600
atttgtatat attaggtaat ttaatttttt attttgtgtg atagaagtag taggaagtga     660
gttgtttaga ggtaggaggg tttatttttt gttaaagggg ggattagaat tttttttatgt     720
gagttgtttg aggattggga tgttgagaat gtgagtgatt tgagtagggt ttgtttgggt     780
attgttgggg taggatttgg aatgtatttg gaaggttttt tgtaagtatt tatttggaag     840
gagaatttgg gatttttttg ggaatttttt gttttggttg gattggttga gtaagtttgg     900
aaaatggtaa atgattattt ggattaatta taggttttta gttggtttgt ttgttataat     960
ttatgatttg gggttgggaa aaagattaat agtttatgtg ttaaaaaagg ggtagagttt    1020
gatggagttg ggtggatttt tttatgttat ttgtttttat atttagagga taagtatttt    1080
tgtagatatt tagtgtaagg gagattatgt ttgattgtat ggatgttttg ttagtgagtt    1140
ttgggtaaat tttggatttt tatattgtga gtttgttttt ttgtatgttt taggagaaag    1200
tttttaaagt atgtttttagt ggattgattt aaattgaatg gtagtattgg tatattgttt    1260
aatgtag                                                              1267
```

<210> 208
<211> 1267
<212> DNA
<213> Artificial Sequence

<220>
<223> chemically treated genomic DNA (Homo sapiens)

<400> 208

```
ttatattgag tagtgtgttg atgttgttat ttggtttggg ttaatttatt gaagtatgtt      60
ttgagagttt ttttttggag tatgtaggaa gatggatttg tagtgtagaa atttaggatt     120
tgtttaggat ttattgatag aatatttata tagttaaata tgattttttt tgtattgaat     180
gtttgtaaaa gtgtttattt tttaggtgtg gaggtaaatg gtatagaaaa gtttatttaa     240
ttttattaaa ttttgttttt tttttggtat gtaggttgtt ggtttttttt ttagttttga     300
attatgaatt atgatagata agttagttaa aagtttgtaa ttgatttaaa tgattattta     360
ttattttttta ggtttgtttg gttaatttag ttggggtggg gggtttttag aaagatttta     420
agtttttttt ttaagtaaat gtttgtaaaa agttttttga atgtgttttg gattttattt     480
tgatggtgtt tagataaatt ttgtttggat tgtttgtgtt tttggtattt tagtttttaa     540
atagtttgta tgggggaatt ttggtttttt ttttggtaaa gaatagattt ttttgttttt     600
gaatagttta tttttttatta tttttgttat atagaatgaa agattgaatt gtttaatata     660
tgtgagtgaa tttttggtga attttatttg ggttgttaat ttttaaatga tttaattagt     720
ttgatattat taatttttag gatttttata gagtttaaaa atttttagta gttttgtaaa     780
agtagatagt tagagaggta ggtagtgagt gtataagttg gtgttttttt ttttattttt     840
tattttaaaa ataatataag aagtgttttt aaaatgagta ggggaggagt tgggatgttg     900
tttttggttt atgttgagta ttaatttttt tttttttatg ttttttttatt tgtggtggtt     960
tagtttggaa aatggtgttt tgtttgtgtt tgtttgtttt ttgtttattg ttttgttttt    1020
tagtttatat tttaaatttt tttgtgtgtt tttttgtttg ttaaattttt tataatttat    1080
```

gtattttttt attttagaaa aagtatatgt aagttttaaa ttgtagaata gagaatggtt 1140
atttttgggg aaaggtttgg gttgggaaat tttttatttt atagtttttt tttttttttt 1200
ttttttgtaa gtttttggtt gaattgattg tatttgtttg ttttgtaatt tagtttttga 1260
gatttat 1267

<210> 209
<211> 1266
<212> DNA
<213> Artificial Sequence

<220>
<223> chemically treated genomic DNA (Homo sapiens)

<400> 209

agttatgtag tttttttatt tggagagaag ttggagtatt gggatagatt taggaggtta 60
gagtggaaat tttgtttggg tgtgtggaat tggagttttt ggtgtgtggt gttaggtatt 120
tatttgtatg gttgagtgtt aggattgtgt atagtagtag ggtgtgggtg agtattgtag 180
tggtgggtag ggtgtggtgt gggggtaggt tttgttgttt gagggtgttt ggttgtggag 240
ttgaaggagg tgttgttgag gagtttttgg atgtgttttt gatgtttatt gtaagttgta 300
tgataattgg ttgttaattg agagaatttt tttttttata agattgaaaa ttaagtttat 360
gtgatgaaat gattgttttt ttttgttttt ttgtattttt tataaatttt tttttttttt 420
ttttttaaaa aaattgtgta agtttggtgg gggtagggtt ttttatttat ggaaatgaga 480
aaattggaaa tttaggaagt tgttttaatt tgggagtaga gggggtagtt tttatttttt 540
tgtttgattt tttttttttt tttttgagtt ttattgtttt aggtgtatag gtttttgtta 600
gatgtttttt ttttttttgta gtttttgttt gagtgttttt gagagttagt tttggattga 660
ttgttttgga tgggatattg ggggagggta gaaggatatt tggttttttt tttaggaatt 720
tgagtggttt tgaggtttgg gggtgtaggg aatttttttt tttgttgttg ttgttgtgtt 780
tggtttgtat gtttttagag ggttgaggaa gttatgttgg gatagattgg ggtgagtaag 840
gttaagaaag gttgatatgt tttatgtttt agtgatgatg tttaataggt tgtatatttg 900
ttttatatgt agtatatata tatatagttt tttaaaaaat ttttattttg tggaatgaaa 960
tagttatttt tagtgtatat agttgtaatt tatttttgta gttaagttgt ttttaataga 1020
agaaatattg tttttttgtat ttttattttt tttttgtttt ttggaaagag aggtgggaaa 1080
ggtaaatttt ttttataata ttggttttaa gtagttattt tgtaaatagt taatgtgagt 1140
tttatgggtt attaatataa agttttttgt tttttgatgg ataaaggaag tggtgatggt 1200
tagaatttgt agggatgtta aatgtttaaa atgtatgttt taaggtattt tttttttttga 1260
tgtgtg 1266

<210> 210
<211> 1266
<212> DNA
<213> Artificial Sequence

<220>
<223> chemically treated genomic DNA (Homo sapiens)

<400> 210

tatgtattag ggagagaaat gttttaaggt atatgttttg gatatttagt gtttttgtaa 60
attttggtta ttgttgtttt ttttgtttat tagaaggtag gaaattttat attggtgatt 120
tgtggagttt atattaatta ttttataggt aattgtttag gattagtatt atgaggagaa 180
tttatttttt ttgttttttt ttttaagaaa taaggagggg gtgaaggtat ggagaatagt 240
attttttttg ttgaaagtaa tttagttata aagataaatt atagttatgt atattgaagg 300

tagttatttt attttataaa ataagagttt tttaaaaagt tatgtatgta tgtgttgtat 360
atagagtaga tatatagttt attaagtgtt gttattaaaa tataaaatat gttagttttt 420
tttaatttta tttgttttag tttgttttga tgtgattttt ttgatttttt aaagatgtat 480
agattagata tggtggtggt ggtgggagag gggatttttt gtgtttttgg attttagggt 540
tgtttagatt tttggagagg aagttaagtg tttttttgtt tttttttggt attttattta 600
aggtgattag tttagaattg gtttttggaa gtgtttgggt aaagattgtg aagaagaaaa 660
gatatttggt ggaaatttgt gtgtttgggg tggtggaatt tggggaggag agggagggat 720

tagataggag agtggggatt atttttttg tttttaaatt ggggtagttt tttgggtttt 780
tgattttttt atttttgtgg gtaaaaaatt ttgtttttat tgggtttatg taattttttt 840
aaggggagag gagggaaaaa tttgtggggg gtatgaaaag gtggaaagaa atagttattt 900
tgttatatgg gtttggtttt tagttttata aaaaggaagg tttttttggt tagtgattaa 960
ttgttatatg atttgtagtg agtgttagga gtatgtttag gaatttttta gtagtgtttt 1020
ttttagtttt atagttagat gtttttagat agtaaagttt atttttgtgt tgtgttttgt 1080
ttgttgttgt gatgtttgtt tgtgttttgt tgttgtgtgt ggttttggtg tttagttata 1140
taggtgagta tttggtgttg tgtattgggg attttggttt tatgtatttg ggtagagttt 1200
ttgttttgat tttttgggtt tattttagta ttttgatttt tttttgaata gagaagttat 1260
gtgatt 1266

<210> 211
<211> 4511
<212> DNA
<213> Artificial Sequence

<220>
<223> chemically treated genomic DNA (Homo sapiens)

<400> 211

ggagtatttg aatgtggaaa ttgaggtgtt agtttaaatt gtttggttgg ttttagttat 60
agttggataa tgtttggttt aggtttatta taagttatat agttgttttt tttgtgttta 120
atttgtttgt gatagaaatt aagggggttt tggtatttag tatttaggtg gtggaattgg 180
ggttttatgt atggttttgt gggtaggttt ttggttagga tttgtgggga gttatgtagt 240
taggagggtg gggttgttta ttgatttagg atgtggtaat ggattgggga gggtggagtt 300
ttagtgattg tttttttttt tgtttgttgg tatttttttgg tttttatttg gttttggtgt 360
ggtttgtgag ttagtgaggt ttgtgtggtg aagtattgtt tgagttttga gtttgagttt 420
ttttggttgt agtagttatt gtttgttgtg ttgttttagg ttattttgaa agaaggtgtt 480
tttgttttgt ttatagttgt atttgtttgt tttttagttt tgtgtgtttg tagttgttaa 540
ttattgtttt ggttgtgtgt gtgtgtgtat gtgtgttagt gtgtgtgtgt gtttgggtta 600
gagttgtgtt gtaattgtta agattgaaat gtagattgtt gggatttagt ttttgtttta 660
ttggggtagg aatgttgggg tggggatatg tatgttttgt ttttaggaat gattttattg 720
ttttggagtt ttatttatag attttattta ttatagggaa tgggggtggg tgttagtgtt 780
tgggtaagtg tataagagtg gtttttggtt ggaggtgagg gtgggaaggt gtgggaagtg 840
tgtgtgtgtg gagtttgggt tagtttgggt ttgggtttgt ttgtagtggg tggagtattt 900
gtggagttgg taatttaggt ttttttttta gtttttgtgt agaattagtt tttttgtgtt 960
gttgggaaat tggtaattag aatgtttttt gtgtgtggta tttaggtagt ttttgagaat 1020
gtttgtattg tggtttgttt atttttgttt tttttatatg tttttggttt tgtgtttatt 1080
atgtttgtgt tttgttttat tgtgggtttt tagtttaggt tttggggttt gtaatagttt 1140
aggtagatga gtgtgtggta gtggtagtgg taggtgaatt tgtaatttgt agagaggttt 1200
ggtggtgagg tggaggagtt ttaggttggg gaaatgtttt ggagattgaa gggaagtttt 1260
agggagaggg ttgttgtttg ttaggttttg taggtttgat ttattttagt gggttatttt 1320
atattgttat gtggatttta atgttggtta tttgggtgtt tggaaattgg ttggaaggtt 1380
ataggtagag aggtttgttt aatagttgga tttttattgt ttagtataga attttttttt 1440
tttttattgg taattaaaaa aataataata aaaaattgtg ttttgttttt gttatttagg 1500
ttggagtgta atggtgtgat tttggtttat tgtaattttt gtttttttggg tttaagtgat 1560
tttttttgttt tagtttttttg agtatttagg attataggtg tttgttatta tgtttagtta 1620
atttttgtat ttttagtaga gatggggttt tattatgtta gttaggttgg ttttaaattt 1680
ttgattttag gtgatttatt tgttttggtt ttttaaagtg ttgggattat aggtttgagt 1740
tattgtattt ggttttttat tgggaatgta tatggaatat atttgtttat ttatttgaag 1800
gaaaaattaa atatttttaa tttatgtttg ttttgtggtt gttatttgtt tttatttttt 1860
ttagattaag atattggttt ttatatattt taattttttg tttttatttt tttttttatt 1920
tattttagtt aggtttgttt ttttttttag gaaattgttt gggatagggt ttttagtgat 1980
ttgtgtatta ttaaatggaa tttagtgttt tattttttat ttttattttt ttagtattat 2040
ttgaagtttg ttttttttga tttttagggg ttatattttt ttagtttttt ttttattttt 2100
tgtagttttt gtttagtttt tttgtagatt ttgatttaat ttttatattt tatgatgaag 2160
tttgggttta gttttgatta ttggtttggt ttgtttatat ttatttgttt tagatttatg 2220
gttgaaatat tgatttaaat ttttagatta gattttttgt gttagtattt ttattaggat 2280
gtttaaaaga tgttttaagt gaatatggtt aaaatttaat tttttttttt ttagttttat 2340
tgttatattt gtttagtttt ttttttgtag taaaaatggt tattaggttt ttagttattg 2400

```
gagataaaag tttaaattta tttttgattt tttttttgtt tttatttttg ataaatatgt    2460
ttaaattatt ttgtttttta tttttatggt tattttattt ttttttgaga atgttgtaat    2520
gttttagttt tgtttttttt tttttttttt ttttttttga gatagagttt tattttgttg    2580
ttaaggttgg agggtagtgg tatgattttg gtttattgta atttttgttt tttgtgttta    2640
agtaattttt ttattttagt tttttgagta gttgggatta taggtatttg ttattatgtt    2700
tggtttattt ttttttattt tttagtagat atgaggtttt attatgttgg ttaggttggt    2760
tttgaatttt tgattttagg tgatttattt gtttttgttt tttgaagtgt tgggattata    2820
ggtatgagtt attgtgtttg gtttttttgtt tattttttgt attttgttat aattttgtgt    2880
tttttttagt tgaatttgtg atgttttttt gtattggatg agagggtttt tatgtatata    2940
tagatttggg atattattta tttataagtt tttaaatagg ttagagtagt gatgtttaat    3000
ttagatttta tgttataata atttggggag tttttaaaat ttattgatgt ttagggttta    3060
tttttagtag ttgatttaat aggtttgtgg tgggatttag gttagtgggg aggattgtaa    3120
aagtattttt ggtgatttta gttggtgttt atttagggga gagtaatttt tgtttgttgg    3180
tgatttttag gggtgtagaa ggattgttgg gtgtgtggtt gtgtgtatat tttagtattt    3240
gatttattgg gttagaaaag ggtgtttgtt aaataaagat ttaataaaat ttttgtttgt    3300
agggggttta ttaaaggttt taaatttttt taggtttttt ttttataggtg gtaatttttt    3360
tttattttaa aggttttgga gggggttatg agtgtttgag aagaggtaag tttgggaaga    3420
tggattttga ggatagtagg tataaatttt tttttaagaa gggttaaggt attttaaaga    3480
taagaaattt aaaattagtg tatttttata tataagtagt tatttttgtt tatttgtggt    3540
ttagatatga gtggagtgtg ataagggata aattattttt gtgtattttt tagtgatggg    3600
gtgaaagtaa tggatttagt ttttgggagt tgtttttgtt gatttttttt gttgtgattt    3660
gatttgtggt gattgtgttg ttttttggtt gttttttttg tttttgtagg tgtgtggggt    3720
tattatttat gtgtgtattg taggtttttg tgtatgatgt tttagatgaa gttgttatag    3780
aggttgtatt atgtgtgtgt ggtgggtttt gtgggttgga agtggtggtt atggttaggg    3840
attagttgtt gtgtggggtt gtatgtggtg ttttgtgtga tgtgtagtgt gttggtatgt    3900
tttagttggg tgtggttttt tttagtgtgt ttagtgggtg ttagtttttg tagtttaatg    3960
agtttaggtt tttttgatat ggtttggttg ggtttgtgtt ttgttggttt tgggtgttag    4020
taagtgtggg ttgggtgggg ttatagggtg ggttttgatt ttagtgtttt ttttaggatt    4080
tagattgggt ggtgggaagg agttgaggag agttgtgtaa tggaaatttg ggtgtaggga    4140
ttgtggggtt tgaaggtggg gttgggtgtg tttttgtaga gttttttttg ttttgttttt    4200
tttttttttt tttgtttttt ttttatattt tattttggat ggttataatg atggtgattg    4260
taaagtatta tgtggagata tttgtgtttt tggaggttag ttttattgtg ttagaggaag    4320
agggttttta tatttggttt tggttttttt ggtttggttt gttgaagtaa tatatttggt    4380
ttatttattg ggtggggtag gaagttttga gtttttattt ggggtgagga ggagggagat    4440
tggttagtag ttttattgtt tgttttgttt tttattgtgg agattggggt tttggtagag    4500
gttggattgt g                                                          4511
```

<210> 212
<211> 4511
<212> DNA
<213> Artificial Sequence

<220>
<223> chemically treated genomic DNA (Homo sapiens)

<400> 212

```
tatggtttag tttttgttgg agttttagtt tttgtagtgg agagtagagt gggtggtaaa      60
gttgttgatt gatttttttt ttttttattt taagtgaagg tttgagattt tttgttttat     120
ttagtgggta ggttaagtgt gttgttttag taaattggat taggagggtt agggttggat     180
gtggggattt ttttttttta gtatagtaaa gttggttttt agaaatatgg gtatttttgt     240
gtggtgtttt gtggttgttg ttgttgtggt tgtttggggt ggggtgtgag gaggggatga     300
aggagggaag gaagggtaag gtgggggggg ttttgtgaga gtgtgtttag ttttgttttt     360
gggttttata gtttttgtat ttaggttttt attgtgtggt ttttttttagt ttttttttgt     420
tgtttagttt ggattttggg ggaggtgttg aagttggggt ttgttttgtg gttttgtttg     480
gtttgtgttt gttagtgttt aaagttagtg aagtatgggt ttaattgggt tatgttgggg     540
gagtttgagt ttattgagtt gtgggagttg gtatttgttg ggtgtgttgg gaagggttgt     600
atttggttgg agtgtgttaa tgtgttgtgt attgtgtggg gtattgtgtg taattttata     660
tggtagttgg tttttggttg tggttattgt ttttagtttg tggggtttgt tatgtatatg     720
tggtgtgatt tttgtggtga ttttatttgg ggtgttgtgt gtaaaggttt gtagtgtgtg     780
tgtgagtagt ggttttgtgt gtttatgaga gtggaagggg tagttaaggg gtagtgtagt     840
```

```
tgttgtgggt taagttgtgg tagagggggt tggtggggat agtttttgag gattaggttt      900
gttatttttg ttttattgtt gaagagtgtg tgaaaatggt ttattttttg ttgtatttta      960
tttgtatttg ggttatagat gagtagaggt ggttgtttat atgtaaaaat atgttgattt     1020
taagtttttt atttttaaaa tgttttggtt ttttttgaga aagggtttgt gtttattgtt     1080
tttggagttt atttttttag gtttgttttt ttttaaatat ttatgatttt ttttagaatt     1140
tttagggtga agggaaatta ttatttatgg gagggagttt ggaaaaattt agaatttttg     1200
gtgggttttt tgtaagtagg agttttgttg agtttttatt tagtaaatat ttttttttga     1260
tttagtgaat tagatgttaa aatatgtatg tagttatata tttagtagtt tttttgtatt     1320
tttgggaatt gttagtaagt aaaggttgtt tttttttggg tagatattag ttggaattat     1380
taggggtgtt tttatagttt tttttgttag tttggatttt attgtagatt tgttgaatta     1440
attgttggga gtggatttta ggtattagta aattttaaaa atttttttaaa ttattgtaat    1500
atggagtttg ggttgagtat tattgttttg gtttatttag gaatttgtgg atggatagtg     1560
ttttaggttt gtgtgtgtat ggagattttt ttatttggta taagaggata ttataaattt     1620
agttgggggg agtataaagt tgtgatagaa tgtaaagaat gaataagggg ttgagtgtgg     1680
tggtttatgt ttgtaatttt agtattttgg aaggtggagg tgggtggatt atttgaggtt     1740
aggagtttaa gattagtttg gttaatatgg tgaaatttta tgtttattaa aaaataaaaa     1800
aaaatgagtt aggtgtagtg gtgggtgttt gtaattttag ttatttggga ggttgaggtg     1860
ggagaattgt ttgaatatag gaggtggagg ttgtagtgag ttgagattgt gttattgttt     1920
tttagttttg gtgatagagt gagattttgt tttaaaaaaa aaaaaaaaaa aaaaaagaat     1980
aaggttggga tattgtagtg tttttaaaga gaaataaagt agttatggag ataagaagta     2040
ggatgatttg ggtatgttta ttagaggtag agataaggga gaaattaaag ataagtttgg     2100
gtttttgttt ttagtaattg ggagtttagt ggttattttt gttgtaaaga ggaagttggg     2160
taagtgtagt agtgaggttg aagaaaaggg aattaaattt tggttatgtt tatttgaaat     2220
gtttttttaga tattttagtg aaggtattgg tatggaggat ttagtttgag ggtttaggtt    2280
agtgttttag ttgtggattt ggggtagatg aatgtagata gattaggtta gtgattagga     2340
ttgagtttag attttattgt gagatatgga agttgagtta gaatttgtaa aggagttgag     2400
taggagttgt agggggtagg aggaaaattg ggagagtgta gtttttggga gttaaaggga     2460
gtaagtttta aatgatgttg agggggtgag aatggagaat ggaatattgg attttatttg     2520
gtagtatata gattgttgag gattttgttt tgggtagttt tttggaggaa gaggtaagtt     2580
tggttggagt gggtagaggg gagagtgaag gtgaaggatt agagtgtata gagattagtg     2640
ttttggtttg aggggagtag agataggtga taattatagg gtagatgtag gttaaaggtg     2700
tttagttttt tttttaagta aatgggtaga tgtattttat atatgttttt agtgaagggt     2760
tgggtgtggt ggtttaagtt tgtagtttta gtattttgga aggttgaggt gggtggatta     2820
tttgagatta ggagtttgag attagtttgg ttaatatggt gaaattttgt ttttattaaa     2880
aatataaaaa ttagttgggt atggtggtgg gtgtttgtaa ttttaggtat ttaggaggtt     2940
gaggtagaag aattgtttga atttaggagg tggaggttgt ggtgagttga aattgtgtta     3000
ttgtatttta gtttgggtga taaaagtaag atgtagtttt ttgttgttgt ttttttaatt     3060
gttaatgagg aaaggggaag ttttgtgtta ggtgatagag atttaattgt tgagtaggtt     3120
tttttgtttg tggtttttttg gttggttttt agatgtttag gtggttaata ttagagtttg    3180
tgtagtagtg tgaggtaatt tattgagata ggttgggttt gtggagtttg gtgagtagtg     3240
gttttttttt tggggttttt ttttaatttt tgggatattt ttttgatttg gagttttttt     3300
gttttattgt taggtttttt tgtagattgt aagtttattt gttattattg ttgttgtgtg     3360
tttgtttgtt tggattgttg tgggttttgg gatttgggtt gggaatttgt ggtggagtgg     3420
gatatgaatg tggtgagtgt ggggttgagg gtgtatggga agggtgagga tgggtaggtt     3480
atagtgtagg tatttttgag ggttgtttgg gtgttgtgtg taaggagtgt tttaattgtt     3540
gatttttttgg tggtatagag aggttaattt tgtgtggggg ttgggagggg agtttggatt    3600
gttggttttg taagtatttt atttgttgta agtggatttg ggtttaggtt gatttaggtt     3660
ttgtgtatgt gtattttttg tatttttttg tttttgtttt tggttagagg ttatttttgt     3720
gtgtttgttt ggatgttggt atttgttttt gtttttttgtg gtaggtgggg tttgtgagtg    3780
gagttttgga gtgatgaggt tatttttggg ggtgaagtgt gtgtgttttt gttttggtgt     3840
ttttgtttta atgagataag agttagattt tggtgattta tgttttagtt ttaatggttg     3900
tggtgtggtt ttggtttggg tgtatgtgta tattgatatg tgtatatgta tgtatgtgat     3960
tggggtggtg gttggtggtt atggatgtgt aggattgggg gatgggtggg tatggttatg     4020
ggtgaggtgg aggtgttttt ttttgaaatg atttggagta gtatgatgag tagtggttat     4080
tgtagttaag aggatttgga tttggagttt gagtagtatt ttattgtgtg aattttgtta     4140
gtttgtaggt tgtgttggga ttaggtggga gttagggggt gttggtgggt gggaggggaa     4200
gtggttgttg gagttttgtt ttttttggtt tgttgttgtg ttttgggttg gtgggtagtt     4260
ttattttttt ggttatgtgg ttttttgtgg gttttggttg gggatttgtt tgtggaattg     4320
tgtgtaagat tttgatttta ttgtttagat gttgggtgtt ggggtttttt tggtttttgt     4380
tatagatagg ttgaatatgg aaaaagtagt tgtatggttt gtggtagatt tgagttgggt     4440
attatttagt tatgattaaa gttgattgag tagtttggat tagtattttg atttttgtgt     4500
```

ttgaatgttt t 4511

<210> 213
<211> 1775
<212> DNA
<213> Artificial Sequence

<220>
<223> chemically treated genomic DNA (Homo sapiens)

<400> 213

```
atattgagtt atgaaattta gagtaaaagt aaaatttttt aatggaatgt ttatgtaaat      60
attagaaata taataaaata aggtaaagtt tgttttttaa aatgttgttt attatattaa     120
aatataattt tgttttttaa atagtgagta aaattaaatt aattttattt ttttaaatag     180
aagtttattt aaagtttttt tataaaaatg atatttattt gatttaatat tgttttttta     240
ataagaataa tatgtagggt gttagagggt agttgttgga ttttttttta gtaaataaag     300
gagattagtt agagtttagt ttggtttgtt taggaaagga ggaatgtagt ttgttgatat     360
ggttaaggaa tgttaattaa tatattttaa aatttttatt attttgtttg agatgtgtat     420
atttttttag gtttttaaag tttaattaga aagtgtattt aattttattg ttattttatt     480
tatagtgggg aagtttattg aggaaaatta tagggaaata aaatgttttt tagttattta     540
tattagtata attaatttag agtttatggt ataaattaaa taagttaatt tgttagtgtt     600
ttgaatgaag atatttaatt aaagtatgta ttttttaatt ttttagtagt ttttaggata     660
gttggttttg gtgattgttt tttggttttt tattgttttt aatatgattg gtttttttatt     720
gtaggatttt aaataaaatg agataattaa attatatttt gagtgaaggg gtgtttattt     780
tgtagataaa tatattggtt ttgttttttt taaaatgtgg atatgtgttt tttttgtatt     840
agggggggtt ttttggtgtg tgttttgttg ttatttgttg aggaaagtga gtgtattttt     900
tgtagtttag gttttgggtg ttagttttgt tttgtagttt tagagtttgt tgtagtttgg     960
gtggtttttt tttggtttag tgtttgttgt ttgttttttg ttttgtaagt tttaagaggt    1020
agttattttt tgtagttttt gtgtttgtaa ttgttttttg gtgggggagt gggtgtttaa    1080
aaagttagta gttggagaaa ttgaaaagat tataagtgat ttaatgataa gttttttttt    1140
tttttaaaag attgagagga gggtagaggg gagtagtgtt tgagtttatg tgattgagtt    1200
agggagtttg atggttttag gaatgtttga tgttgtgtgt gatttttaag tgggagtatt    1260
tttgaattga tttttggttt atttataagg atagtggtgt atagatggtg ttttttgtag    1320
ttttagtttt agatttaaga ggtttggagt agggtttgag aatatgtatt tttaattagg    1380
ttttggggga tgttgatatt gatatagtta gtttggggat tatattttga ggattatgtt    1440
tttagttttt gatttatata agtgttattt agaatagatg tttgatttta aggagttagt    1500
ggtgaaatta gagaggtttt gggtttgtta aatttttagt agtaaatgta attttgggtt    1560
ttggagtggt aaagttgtgg attagaggtg gagggagtgg gtttttagtt tttaagaggt    1620
tattgggaag gttttttgtg ttagattaaa gattttaggt atttttttga atttatttga    1680
agtggtattg gggagatttg tgtttttggt tatggtgttt ttttttgttg gaggtatttg    1740
tttatttttt tttttgggtg aaggtttttt gtgtt                               1775
```

<210> 214
<211> 1775
<212> DNA
<213> Artificial Sequence

<220>
<223> chemically treated genomic DNA (Homo sapiens)

<400> 214

```
ggtgtgaggg gtttttgttt gagaagaggg gtgggtaggt gtttttagtg gagaagggtg      60
ttgtggttgg aggtataggt ttttttggtg ttattttaag tgagtttgag gaagtatttg     120
ggatttttga tttaatgtga aaggtttttt tagtgatttt ttgagagttg agaatttatt     180
ttttttattt ttagtttatg gttttgttat tttagggttt gaggttatgt ttgttgttgg     240
ggatttgata aatttaaagt ttttttggtt ttattattgg ttttttagaa ttagatattt     300
gttttgaatg atatttatgt gagttagggg ttgaggatgt gatttttgaa gtgtggtttt     360
tagattggtt gtattagtgt tggtattttt taggatttgg ttggaaatgt atatttttag     420
gttttatttt agatttttta aatttgagat tggggttgtg gggagtgtta tttgtgtgtt     480
```

attatttttg tgggtggatt aggagttggt ttgagggtgt ttttatttag aggttatgtg 540
tggtgttggg tgtttttgag attgttgggt tttttggttt ggttatgtgg gtttaggtat 600
tattttttttt tatttttttt ttggttttta aaaggaagaa ggggtttatt gttaagttgt 660
ttgtgatttt tttagttttt ttagttgttg gtttttttgga tatttatttt tttgttagga 720
ggtagttgta agtgtggagg ttgtgagaaa taattgtttt ttgaaatttg tagggtgaag 780
agtaggtggt gagtgttggg ttggggaggg attatttgag ttgtgatggg ttttggggtt 840
gtggggtagg gttggtgttt ggagtttgag ttgtaggagg tgtgtttgtt ttttttaata 900
ggtggtggtg gggtgtgtgt tgggagattt tttttaatgt gggaaaagta tgtgtttgta 960
ttttagagaa ggtaaggttg gtgtgtttat ttgtaaggta agtgtttttt tgtttgaggt 1020
gtggtttaat tgttttattt tgtttgaaat tttgtggtga gaaattagtt gtgttgagaa 1080
taataaaaga ttaaaaaatg attattaaaa ttaattgttt tgaaagttat tggaaagttg 1140
gaaaatgtat gttttgatta aatgttttta tttaagatat tggtaagtta atttatttag 1200
tttgtgttgt gagttttggg ttgattgtgt taatatgaat aattgaaaaa tattttattt 1260
ttttatggtt ttttttgatg gatttttttta ttatgggtga aatgataatg gagttgaata 1320
tattttttga ttgaatttttg agggtttggg aagatgtata tgttttaggt aagatgatag 1380
gggttttaaa atgtattaat tggtattttt tagttatgtt agtaagttgt gttttttttt 1440
ttttgggtag attaagttaa gttttaattg gttttttttta tttgttgaag aggagtttaa 1500
taattgtttt ttaatatttt gtgtgttatt tttattggaa ggataatatt aagttaagtg 1560
aatgttattt ttgtgaaaaa attttgagtg gatttttatt taggaagata aggttgattt 1620
aattttattt gttgtttaaa aagtaggatt gtgttttggt gtggtaggta atattttgga 1680
ggatagattt tgttttattt tgttatattt ttagtattta tatgggtatt ttattagaaa 1740

gttttatttt tgttttaagt tttgtaattt ggtgt 1775

<210> 215
<211> 1223
<212> DNA
<213> Artificial Sequence

<220>
<223> chemically treated genomic DNA (Homo sapiens)

<400> 215

tttatgtgtt ttatttgggg tttggagtaa ataaaattga tttaaaaata attttgaatg 60
atagaaaggg ggtatttttt ttatttaagg gggtgaggta ggttgaagat gggtttttat 120
tttttttaag attatggagg gtgttggggt gttattttag ttgtgaaaaa gggatttagg 180
atttgtagat gttggtgagg aataggggtg tggagtatga gttggttttg tattatgttt 240
tggggttttt agtattgaag tgtttagtat tatgtagagt agttgggagt gggttttgtt 300
gggtggggtt gagttgtgtg tgatttttgg tgtgtttggg gaggtttaga taggggtggt 360
ttttttggtt tttgtttttg tgggttttat gatattgtat tggtttgtgg gatggggtgg 420
ggttgggtga gggggaggag atagttttat gttttgtgat ttgtggtgat ttttatgtgg 480
aattttttttg tggtaatttg aagtattgtg tttgtgtgtt gtagtagtgt ttggtggtgg 540
tggtagtttg tttgtgggtg tgtgaaggga gatagtgtgg aggttatagg gtatttgtta 600
tgatgagtag tattttagtt aagattgtgg agatagaagt agaggtaatg gatgtagttg 660
gtggtttatt tttagtttga gtattttttt tttggtggtg ttgttttttt ttgttttttt 720
ttgagttgtg ttaggattgg gtttagattt tgtgattttt tttagtgagg tttgtgtttt 780
gattgttttt tgttatgatt aggagttttt gatttttttt agttagggtt ttttttttt 840
ttgttttgtg tttatgtttt tggatttttt tttgtttggg tttgtgtttt gtatttttgt 900
atttaggttt tttagtttgt tttttttttt agtgtttggt ttttgttttg atgttgtttt 960
tttttgtttt gttttgagaa atattgaatt ttgtttttta ttttattttt gatttggtta 1020
gatagtttag gatttttttt tattattttt tgttgtgttt tttttatttt tttttttttt 1080
agtgtttatt ttaatgattt tttgatttag agtatttgtt tttggtaatt ggatttggaa 1140
gtaaaatgat atttgtgtgg gagtagttta ttttgtttgg atagtgtgtg attttattgt 1200
gtgtggtttt gagagtgttt tat 1223

<210> 216
<211> 1223
<212> DNA
<213> Artificial Sequence

<220>
<223> chemically treated genomic DNA (Homo sapiens)

<400> 216

```
gtaaagtgtt tttagggttg tatgtaatgg aattatatat tatttaggtg gaataaattg      60
tttttatata gatgttattt tatttttaga tttgattatt gggagtaaat attttgaatt     120
agggggttat tagggtagat attgggaaaa gggggaataa gggggatgta gtgaaggatg     180
atgaggaaag attttgaatt atttgattaa gttagagatg gaataaggga taggatttag     240
tgtttttttag agtaaaatgg aaagaaataa tattagaata ggagttaaat attgggaggg     300
ggaatggatt aagggtttaa ggtgtagggg tgtggaatgt gggtttgggt aaaggggagt     360
ttgagagtgt agatgtggga tgaggggaaa gggggttttg attgaggaga gttggggatt     420
tttaattgtg gtagagggta gttggaatat gggttttgtt gagagaagtt gtggaattta     480
ggtttggttt tgatgtggtt taaaggagaa taggaaggag tgatgttatt aaagaaggaa     540
tgtttagatt aagagtgaat tgttagttgt gtttattatt tttgttttta tttttgtgat     600
tttagttaag gtgttgttta ttgtggtgag tattttgtgg tttttatatt gttttttttt     660
atatatttgt gggtaaattg ttattgttat taggtgttat tgtagtatgt aggtgtggta     720
ttttgaatta ttgtgagaga gttttgtgta ggggttattg tgagttgtaa agtgtggggt     780
tgtttttttt tttttatttg attttgtttt gttttgtgag ttagtgtggt gttatagagt     840
ttgtgggagt ggaggttaga gaggttattt ttgtttgggt ttttttgagt gtgttgaggg     900
ttgtgtgtag tttggttttg tttagtggga tttgttttta gttgttttgt atggtattga     960
atgtttagt gttggagatt ttagggtgtg gtgtaaagtt gatttatgtt ttgtattttt    1020
gtttttgtt agtatttgtg ggttttgggt ttttttttta tagttggggt gatattttga    1080
tgttttttat ggttttggag ggagtagaga tttattttta gtttgtttta tttttttgga    1140
taaagaaaat gttttttttt tattatttaa aattattttt aggttggttt tgtttatttt    1200
gaattttgag tagagtatat ggg                                           1223
```

<210> 217
<211> 2183
<212> DNA
<213> Artificial Sequence

<220>
<223> chemically treated genomic DNA (Homo sapiens)

<400> 217

```
aagggagaag gatgggggtg ttgtttggtg attgtttgtg tgattttttt aaggtggtga      60
ttattttttg ggatttgttt tttagttgtg ttgtttgtat ttgggttttg tgattttttt     120
ttgttttttt tttgttttag tgatgttaag atgttgaatt gtagggatga ggttttgggg     180
ataaagttat tttgtggtgt ttttttttgt taagttgtgg gtttttggtg gttagaaagt     240
gttttaggtt tatttttgtg ggtttggttt ttttttttta tttaaggttt tggggttatg     300
agtttttat tttgtgtttt ttttgtgttt tttttatttt aaattttagt agtgttgttg     360
ttttttttgg aaataagttt gttgagtaaa ggtggaggaa aattttgggt tttttgtttt     420
gattggtttt tttttgttga gtggggtgag gtagtgattg gttgttgggt gttgttagtt     480
gttgaggtta gagttattgt tggggaagtg tagttgggtt tgggtgtttt ggttgttgtt     540
gtggttgttt ggttagtgta gttgggtgag tttgtgtggg tgttgttgtt gtttttgttg     600
ttgtttattt tgttgtgttg ttggtgttgt tggttttgta ggtggttgtt ggggaggggt     660
ggttgagaga gtggagtatg aggaggtggg ggtggtagag aagtgatgtt ggtgttgggg     720
attggggtag tggtagtgga gtagtagtat ttttgggatt ttggttgtag tgtttttgtg     780
gtttgtgtgt tagtttagtg gttggagatt atgtgttttg tgggtaaggt tttgtttggt     840
agtgtttgtg atgtttttgg gatggttggt gtggtttttg gggttatga ggtgtgtggt      900
ggttggggta ttttgatgaa ggtgttggga tttggattgt gtgttttgat gttgggttgt     960
gttattgttt gtgtttgtgt ggttttagtt ggtgtgtttt tttttttgtg tttatagatt    1020
ttgatagtgt ggggatggta ttgtgtgagt gtggatgtgg atgtgtgtgt gtgtgtgtgt    1080
ggtttggggt attttgtagt gagaatagtg ggtttttttt gtgtggtttg atgtttttta    1140
aggatttaag ttgggttgga gtgagtagga tgttgttggt ttggtgggag gtagaatgta    1200
tggagtgtat gggaaagttt attgtggttt gtaaagagtt gagtaaattg aattttattg    1260
tggtgattaa aaggagttgt ggatttggtg tgttagggga gaggtgtgtt ttatgaggtt    1320
ttttaaagtt ttattttgtt ttggggattt tggaggggaa gggaagagag ggaaaagtaa    1380
ttgttggttt atattgttga agaaaaggag gaaagagaag ttgggggtgg gggtgggggt    1440
```

```
ggaggttatt tttgggggag tggtgatttt taggtgtttg aagttttgtg tgggaggttt    1500
gttatatttt tttggagtgt agatttgagg atggagagtt ttattttgag gtattggtag    1560
tggttttttt tgtttggtgg tggtgtaggg tgtagtggga tagtttggtg gaggtagagt    1620
ggggttttga tgtttgatgg tttggtgttt aggtggggtt attgtttttt taggttgttt    1680
tggtttgttg attttttagg ttgagatttg gtttggtgtg tttttggtag ttgttaaatg    1740
tttggtggtt ttgtttttttg ataaagttta ggtttgttgg ggtttggttt attagattta    1800
ggatatgtta attatgtaga gtaattaggt tagggtattt agttgttttt agagtgtttt    1860
tgttttatat tagattattt tatttgataa agttgttgag agttttattt gtgttgtaaa    1920
gtttttgta gaaggtgaat tttttgtgtt gttgtaaatg gatggtgtta ttgggttaga    1980
tttgaatttt tgtagtttag tgtagtgggt tttttatttt tgtttaggat gtataaattt    2040
atttttggtg attgtttttt atgtggtttt ttgtttttgt gggagtattt gtttaggaaa    2100
gtttttgaaa tattttaata ttaaggagtt ttttttattg ttttagatgg ggtgaggttt    2160
tggtatttga ttggtgttgg ggt                                            2183
```

<210> 218
<211> 2183
<212> DNA
<213> Artificial Sequence

<220>
<223> chemically treated genomic DNA (Homo sapiens)

<400> 218

```
attttaatgt tagttaagta ttggagtttt gttttatttg agatagtggg gagagttttt      60
tagtattgag gtattttaag aatttttttta agtagatatt tttatggaga taaggagtta     120
tatagaaaat aattgttaag agtaagtttg tgtgttttga gtagggatgg agaatttatt     180
gtattgaatt atggagattt ggatttggtt tagtagtatt atttatttgt agtagtgtaa     240
agaatttgtt ttttgtggaa ggttttgtag tatgggtgga atttttaata gttttattaa     300
ataagataat ttaatatgaa ataaaaatgt tttggaggta gttgggtatt ttaatttggt     360
tattttgtgt ggttaatgtg ttttaggttt gatggattag attttagtgg gtttgaattt     420
tgttggaaag tggagttgtt aggtatttgg tagttgttag aggtgtatta ggttgggttt     480
taatttggga ggttagtggg ttaaagtggt ttggggaggt agtggtttta tttggatgtt     540
gggttgttgg gtgttggagt tttgttttgt ttttattaag ttgttttgtt gtgttttatg     600
ttgttattag gtaggaggag ttgttattag tattttagag tgggattttt tatttttaag     660
tttgtgtttt agaaaagtgt agtaagtttt ttatgtgggg ttttagatat ttaaaagttg     720
ttatttttttt aagagtaatt tttattttta tttttatttt taattttttt ttttttttttt   780
tttttagtaa tgtggattaa tagttatttt tttttttttt tttttttttt ttgaagtttt     840
tggggtaaag tgggattttg gggagttttg tggaatgtgt ttttttttttg gtgtgttagg    900
tttatagttt tttttaatta ttgtaataaa gtttaatttg tttggttttt tatgagttat     960
ggtggatttt tttgtgtgtt ttgtgtgttt tattttttgt tgagttaata gtattttatt    1020
tattttagtt tagtttgggt ttttgaagga tgttgggtta tgtagagggg atttattgtt    1080
tttgttgtaa agtgttttaa gttgtgtgtg tgtatatatg tatttatatt tatatttata    1140
taatgttatt tttgtgttgt taaagtttgt gagtgtggaa gggagaatgt attagttgga    1200
gttgtatggg tgtgggtagt agtatagttt agtgttggga tatgtagttt gggttttaat    1260
atttttgttg ggatgttttg gttattatgt gttttgtaat ttttaaagat tatgttagtt    1320
gttttggggg tgttgtgggt gttgttggat agaattttat ttgtggggtg tgtggttttt    1380
ggttgttggg ttggtgtgtg ggttataggg atgttgtagt tagggttttg aagatgttgt    1440
tgttttgttg ttgttgtttt gatttttggt gttagtatta tttttttgtt gtttttgttt    1500
ttttgtgttt tgttttttttg gttgtttttt tttggtggtt gtttgtaaga ttggtagtgt    1560
tggtggtgtg gtggggtggg tagtagtaga ggtggtggtg gtgtttgtgt gagtttgttt    1620
agttgtatta gttgagtagt tgtggtggta gttgaagtat ttgagtttgg ttgtgttttt    1680
ttggtgatgg ttttggtttt ggtgattgat aatgtttggt agttaattgt tgttttgttt    1740
tgtttggtgg ggggagatta attagagtgg aaggtttaag gttttttttt gtttttgttt    1800
agtgggtttg tttttgggag aggtagtgat gttgttgggg tttgggatgg gggaggtgtg    1860
ggaggggtgt aggatggggg atttgtggtt ttggagtttt aggtgaggag ggaaagttgg    1920
gtttgtaggg gtgggtttgg ggtgtttttt gattgttagg agtttgtggt ttggtaggaa    1980
agggtgttgt ggggtgattt tgtttttaga gttttgtttt tgtggtttag tgttttggta    2040
ttgttggggt gagggggggag tggggagggg ttgtgggatt tgagtatgaa tggtgtggtt   2100
ggagagtgaa ttttaggagg tggttgttgt tttgaaggaa ttatgtgaat agttattaga    2160
tagtgttttt gtttttttttt ttt                                            2183
```

<210> 219
<211> 8030
<212> DNA
<213> Artificial Sequence

<220>
<223> chemically treated genomic DNA (Homo sapiens)

<400> 219

```
tttgtgtaat tttagttagt ttgaattttt ggaggtaaat agagtgtaat ttgttttgga      60
agaattttgt tatgtttaag gttttatgtt gggtgttggt ttttttgttt ttaattattt     120
ttttattttg atttggattg tagaaatttt taattttttg ttttttagtt ttatttttat     180
ttttagattt ggggttgtta aataatggaa attttaggaa gatgtaggtg tggtttttaa     240
agttttggtt tgtgagtgtt tttaggttgg ttggggtgtt ggttttttta gaaataaggt     300
ttgaatatgt aggtgttagt taggattttt tgttgtttgt ttattgttgt ttttttttttg     360
ggatttgaga gaagtgggag tgtgtgtgtg tgtgtgtgtg tgtgtgtgtg tgtgtgtgtg     420
tgttttgtgt taggagaaag gtttgtggtt gttgttttag ttggtttgtt tattttttgt     480
ttttaggaga agtttgttta tttgggatat tagtgatttt ttttattttt atttattgg      540
ttttatattt tttttatttt tttttaagtt gttgatgtag aaagtatttg gttattttga     600
aatggtagtt tttggaattt tagttttgtt ttgtaattta gtagggtttt ttggaagttt     660
tataattgag atttatgggt ttattgggta attaaatgat gttattgtgt taattttgta     720
tgtattattg ttgtttgtgt tgtttgtggg ttgttggttg gttgtagttt gttggtgatg     780
agggtattat agttgttttg attgtgtaga ttatgtatgt tttggttttg ggaatttatt     840
atgtattaga atatattagt gtttgtattt taaaaggtta aattattggt ttttagttag     900
ggatttttgg taagtggttt gttagtgatg agtgtttgtt tatattggta tatagtggag     960
tttttttgttt ttggtttatt tgtttttagg aaagttttgg ggtgaggtga aggtgattga    1020
agtaatgttt ttttttttag attgtagttg tttagggggg atatagtatg gtatttttta    1080
ttgaattttt tttgtttgtt tgtattttag tttttttttt tttagtgatt tttttatttt    1140
tttttttttt ttttttttttg atgtttgatt ttagtagtaa aggaggtaaa aaaggtattg    1200
agttgttagt taaatttgaa aagtgtggtt ttgttttttt tatagttatt ggtagttttt    1260
tgtggaaggt ttgtttttttg gggtagttgt ggttttggag tggttgtgtt tggtgtttgt    1320
tgggtgtggt tttgttttag gtttgggagg gtaggttggt tgttttggtg agtggtagag    1380
ttttttttgga tagtttttgt ttatttaaat agaagatgtt ggtgttggag tgggtttgga    1440
tatggtgagg ttgtgagttg gtttgagtgg tggggtttgg tgattttttt tttttttttt    1500
gtttttttttt tttttttgta tgtatgtttt gtttgttttt attttgtttt tattttgggt    1560
gagtttgttt gtagtttggg gtgtatattt gtatgtgtat ttttttttat ttatttttgt    1620
gtttgttttt atttttgtag ttgagttttg ttatgtgtgt tttgtttgtt tgttggttgt    1680
ttttgttgtt tttgttgttt ttgggttttg atggattgaa tgaaggttgt ttatattgtt    1740
tattgatgtt ttattaaaga tttagaaggt tgtgttatga atttggagtt gataatggaa    1800
agtttgggta ttttgtatgg gttggttggt ggtggtagtg gtgggggtgg tggtgggggt    1860
ggtgggggtg gtggtggggg tttgggttat gagtaggagt tgttggttag ttttagtttt    1920
tattatgtgg gttgtggtgt tgttggtttg ttgtggggtt ttttgttgtt tttaattgtg    1980
tattaggagt tgggtatggt ggtagtggtg gtagtggtgg tgttgtgttt ggttatggtt    2040
attagtatgg ttttgatttt ggatggtggt gattattggt ttgagttttt tattttgttg    2100
tattatgtta tgagtatgtt ttgtgatttg tttttgtttg gtatgggtat gagtaatatt    2160
tatattatgt tgatattgtt ttagttgttg ttatttattt ttattgtgtt tgataagttt    2220
tattattttt atttgtatta ttatttgtat tattattatt attattatta ttagtgtttg    2280
tttggtaatg ttagtggtag ttttattttt atgtgtgatg agtgtgggtt tttggttatg    2340
aataattttt atagttttta taaggagatg tttggtatga gttagagttt gtttttgttg    2400
gttgttatgt tgttgggtaa tgggttaggt ggtttttata atgtgtagta gagtttgttt    2460
aattatggtt tgttgggtta tgataaaatg tttagtttta attttgatgt gtattatatt    2520
gttatgttga tttgtggtga gtaatatttg ttttgtggtt tgggtatttt atttgtggtt    2580
atgatgttgt atttgaatgg tttgtattat ttgggttata tttagtttta tgggttggtg    2640
ttggtattta gttgtgagtg gttatttttg tttttattgg gtttgtaggt ggttatgttg    2700
ggttagttgg aagaaattaa tattaaagag gtggtttagt gtattatagt ggagttgaag    2760
tgttatagta ttttttaggt gatttttgtg tagagggtgt tgtgttggtt ttaggggatt    2820
ttttttgatt tgttttggaa tttaaaattg tggagtaaat ttaaatttgg tagggagatt    2880
ttttgtagga tgtggaagtg gttttaggag tttgagtttt agtgtatgtt tgttttatgt    2940
ttggtaggta aggttggggt tagttagggg ttaggttgtt gggaagaggg ttttgggttt    3000
```

ggtgtttgtg gtttaagttt gtgtgttgag ttattttttt tgattttttt tttttttttt 3060
ttatatatgt tttttttttt tttgttttta tttttttttt tattttttttt ttttttttttg 3120
tttttttttt attttttttt tttttttttt tttttttttt ttattttttt tttgtttttg 3180
agtttttatt gattgatttt tttttatttt atttgttttt tttttaatgt gttaattttt 3240
gttttatttt tgattttttt aggtattggg aggtgggatg ggggtgtgtg ttttttttta 3300
ggagttttgt ttttttaaga tttatagaaa ttaggatttg tttttattta aaattttatg 3360
tattttaagt ttttttaga taatatattt taattttttg ggttgattag ttttttttgtg 3420
tagaggtagt tgagaggttt tgttttgtag agggaaaaga gtttttttatt tttttattta 3480
ttatataggt aaatttattt ggttattggt tgaaggtata gttttgtttt tgtggggaat 3540
tggtggttag gatataatag tgtttttgga gtttattttt ggttttggtg ttggtgtagg 3600
gatttttga ttgggtttga ggggtttggg ttagttttaa tgttattatt tatagtgagg 3660
gtagggtgta aggttgagaa ggttatattt attgttttgg gaggatgtgg gagaagagat 3720
tgaggtggaa agtgttttgt tttgtttatt ggttgttttt gttttggttt tagtgtttgt 3780
tgggatttgt taggatttgt tggggttttg ggagattttg agtatttgta ggaagaggtg 3840
ttgagaaatt aaaaatttag gttagttaat gtatttttgt tgttggttgt aggttttgtt 3900
tttgtattaa gtgggtgttg attgtgtgtg tttggtgatt gtggggagga ttggtggttt 3960
gtgggagggg atgggtagag gtgtgggtta tattgttttg gagttggttt ggtttttttgt 4020
gttttttta gtggttaagt tgtgaggtat agtttttttat tgttttagga gtatagaaat 4080
tttttgtgtg ggtggtgggt gtgtgagtta gagggaaaga tgtagtagtt attgtgattg 4140
gtatgtagtt gtgtgttttt gtgtgtatgg attttgtgtg gtgtgtgtgg tgattgtgtt 4200
gtttttagga gtaagttatg ggtttagagg ggtaaaatgt ttaggttttt tgttgggaag 4260
gatatattat attttatggt aagttagggt gggtgatttt ttatggattg ggtggagggg 4320
ggtatttttt aggattggtg ggtggtttag gggaataatt tgtggtggtg atgatttgta 4380
tagtgtgggt tttgggatgt gtgtggtttt gagttagttt tgtatagttt gtttttggag 4440
ttgtgagttt aggtttttat ttttgatttt ttgggttttt tttgtattgt tgagtttagt 4500
ttgtggggtg tatttgatta atgtttgata gggttgggga atgtgatagg tagtaggttt 4560
atttgggttt ggggaggggg agtttttgtt ttgatagtat ttttttttgt tgtttgttgg 4620
tggattttta tttttagttg gtaattgttt tgtagtgttg atttaagaag gtaaagaaaa 4680
ttaggttttt ttgtaaagag tttttttttaa attggtggat tttggatatt ttgagtggat 4740
ttagaaattt atgtaatttt tttttttag tttatttttt atttttttttt atagtttttt 4800
ttgatttgtt gttggtttgg ggtaagataa agtagttagt agagagtgat aataatagtg 4860
gtgggaaatg aattggagat tggttgatag tttttaatat tttgttatag attttttttga 4920
atgttttagg ttgtttttgg tgggttttag tatttgttgg ttttttgggt attggggatt 4980
agaaggaatt tggtagttgg ttttaggggt atagttaaag gtaggatgat agttattttt 5040
ttgtttattt tagagtgttg ttgttttttt atgttggttg tgtaaagaat atagttttta 5100
aaaaatatgt gtttttttgtt tatataggtt tgaaagtgat gaggaaagta atgttttgtt 5160
tattagtgag ttttagtttt taaaatgatt ttaagtgttg ttgagatgag aaagtgtggt 5220
atttgggggg tttttagttt tatttgtgtt tatggtgtaa gtttgtaggg ataggtttgg 5280
gatagtattg tttatgttgt tagatttttt gtagaggatt gttgaagttg tttttgtggg 5340
agatagaatg ttttttttag tgagtggaaa aggtttgttg aggattttgt tttgtttgag 5400
tatttaaatg tgtgtttgtt ttattatttt gggttgaaaa gggataagag ttttagtttt 5460
tttatttggt tattttatta gtaattataa gtgtgttgag tggttattat tatataggag 5520
gtttttttagt ttggggttag tagattagtt tttttagata ttgatgtaga agttgggatt 5580
ggtaagtagg tattatgtgt ttggagtgtt aggggatagg agtaaatgga gaagaaaagt 5640
ggaggttttt tttgtttgga gtattgattg gaatttttgt tggtatgttg tagagggttt 5700
ttgttgttgg gttttggggg tttaataagt ttagttgttt tgtaggtggt ttggttggat 5760
ttttagattg gtgtttggaa gatattgttt ttgttttttt tttgttaaat ttgttttttt 5820
tttttttttt ataggttata ggtttttttt tttttttatt ttggttttgt ttttgggttt 5880
tgttaaatag ttaagtaggt tggggtttag gggtttaga atgaagaggt ttgatttggt 5940
tagtgttggt aaagtttatt tttaggtgag gttataatag aggtaggttt tttttgttta 6000
gtttgttggt gtagttatag ttaagggtgg tatttgaaag gaaaagggag aaaattttgg 6060
agaaatttag attgttttaa tgttagattt tagagaaatt gattttaaat gtatggattt 6120
gtttggaaag ggtggttaag tggtaggtgg ttgtaatttt gtttggttgg gtttttgtag 6180
aggtttttta agattagttt ttgtagggtg gtttttagta atttgataag aggtggttaa 6240
gataaatttt tgtgggtttg agtatatatt tttgggtgtt gggttttaga gatttttaaa 6300
ttaagtataa ataagaaggg agtgagagaa tttaggttag aatttgtatg ggtattttat 6360
tgaggaaaag tgaggttttg gtggtaggta tgtttttttt tgatgtttga aaattgagtt 6420
gagtgtttga ttatatttat tgtagaggtt tttgttttta gtgagtttgg atttttttagt 6480
ggtttgtttg gagttggttt ttagtttttg ttgtagtttg atgtatggtt ttttttttggt 6540
agtaagtttt tagtggttag tttgaagtta attttgttta ggtggttgag ggtttttagt 6600
taatttatta tgatgttgtt tgggttattt gatgtttgta gtggtgggat atggtttggg 6660

```
tagtgtgtag tggtttttgt taggggtatt gtgtgtgtgt ttgtttttttg ttgtgttggg    6720
gatgtttttg ggtgatatgg gttgttgggt atttttttaag ttgaggaaat ggattttttt    6780
tgtagagttt tgtgtttatt ttttaatttt ttattttgtt ttttgttgtt agggttttttg    6840
atttagttta ttttttttgg tggtttagtt agggattaga gttggagagg ttgaatgtaa    6900
tttgtgttag tatggaatag atgatatgtt tgtttgttag ttgtttggat gaataattga    6960
aaagtttgtt gtagtttgtg ttttgttaag ttttgggtgt tgggagaata ttttttttaat    7020
atgtattagg gtgggtggga gtgggtagag gaggtgggat ttgagggagg agagtgaatt    7080
tgagtaggag aagtagttta ggtagttagg tgtttttgat gtgagaggtt gggtatttat    7140
ttttatttta ggtttttttat tgtgtggtta tgttattttt ttaaataaat gtgtatatgg    7200
agggagattg atgttgataa tgtttagaag attaaaagag tattaatgtt ggtaataata    7260
atgtaaatgt gtggatttag attttattga tttggaattt gatttggtgt gtttttagta    7320
agtttgatgg tgtgtttttt ttagtagagt gtttattagt gttatggttt tgtggttttt    7380
tagtggtgtt gtttttgttag ttttgtgtgg gtttttttgt ttgattgtag ttttttttttt    7440
gtgaggtttt agtttgtttt atttttttga ggtttttttt tttttttgtg gggtttttttg    7500
tttttttgtat ttttttttttt gatttttgta ttatttgttt ttgtgtgtat atattgttat    7560
ttgtgttttt ggtgatttgt ttgggtggtt gggtttgtga agttaatgtg ttgaatggtg    7620
tttgagtttt tttaattatt ttgtgtttgg ttgttgttat tgggttttgg tgattaagtt    7680
taagtttgaa aatgatgtgg ttaaagttgt ttgttaatgg tagagtttaa ttagttgtag    7740
atttttttttt atttttattg gtttttgtat taaaaaggtt tatgtgtaga tttttttatgt    7800
aggtgtattt gttggataat taaatgtggt tttagtaata aaagtttgag ttttattttt    7860
ttgagtagta ggttttgtgt tggattggtt gggtttaata gggagaattt tgtgttttat    7920
tttggttggg ataggaagta agagaagtaa attggggaat ttttgtttta ttttttttttta    7980
ttttttggat gttttgggtt gaggtttggg ttattggttt attgtgggta                8030
```

<210> 220
<211> 8030
<212> DNA
<213> Artificial Sequence

<220>
<223> chemically treated genomic DNA (Homo sapiens)

<400> 220

```
tatttgtgat gggttagtga tttgggtttt ggtttaggat gtttaggggg tgggaagggg      60
tggggtaggg gttttttagt ttgttttttt tatttttttgt tttagttaag gtaggatata     120
agattttttt tgttagattt gattagttta gtgtagagtt tgttatttgg agggatgaag     180
tttaggtttt tgttattagg gttatgttta attgtttaat gaatgtattt gtgtaaaaag     240
tttgtgtgtg agttttttta gtatgagaat tgatgaggat aggaggggat ttgtggttga     300
ttaagttttg ttgttagtag gtagttttag ttatgttatt tttaaatttg ggtttagtta     360
ttagggttta gtggtaatgg ttaagtatag gatagttagg aagatttggg tattgtttag     420
tgtattggtt ttgtggattt agttgtttag gtggattgtt ggaagtgtaa gtagtggtgt     480
gtgtgtatag ggtgggtggg tgtagaggtt gggggagggg gtgtagaggg tagagagttt     540
tgtgagagga ggagaaaatt ttggggaagt aaggtgggtt ggggttttgt gggggaggag     600
ttgtggttag atgggagaat ttgtgtagag ttggtgaagt ggtattgttg gaaaattgtg     660
gggttgtggt gttggtgagt gttttgttgg gaagagtgtg ttgttgggtt tattggaaat     720
gtgttggatt aagttttaga ttaatgaaat ttgggtttat atgtttatgt tattgttgtt     780
agtattaatg tttttttaat tttttaaata ttattagtat tgattttttt ttatatatat     840
atttgtttga gaaagtgata taattatata gtggaaagtt tggaataaaa ataaatgttt     900
agtttttttgt attgagggtg tttggttgtt tgggttgttt tttttgtttg ggtttatttt     960
ttttttttgg gttttgtttt ttttgtttgt ttttgtttat tttgatgtgt gttgggaagg    1020
tgtttttttg gtatttggga tttgatgaag tatagattgt agtgaatttt ttaattattt    1080
atttaagtag ttagtaggta aatatattgt ttgttttgta ttggtatggg ttgtgtttag    1140
tttttttagt tttgattttt aattaaattg ttaggagagg tgggttgagt tgggagtttt    1200
agtagtgaga aatgaggtgg gaggttgggg ggtgggtgtg agattttgtg aagggggttt    1260
gtttttttgg tttgggaggt gtttagtggt ttgtgttatt taaggatgtt tttggtgtag    1320
tgggagataa gtatgtatgt ggtgttttta gtaggagtta ttgtgtattg tttgggttgt    1380
gttttgttgt tgtgggtatt aggtggttta ggtgatatta tggtgggttg gttaagggtt    1440
tttggttgtt tgaatagaat tggttttaga ttggttgttg ggagtttgtt gttaggagag    1500
ggttgtgtgt tggattatgg tggggattgg agattagttt tgggtaggtt gttgggggat    1560
ttgggtttat tggaggtgga aatttttgta gtaaatgtag ttgggtgttt ggtttgattt    1620
```

ttgggtgttg gaagaaaata tgtttgttat tgaggttttg ttttttttta gtgggatgtt 1680
tgtgtaagtt ttagtttggg tttttttatt ttttttttgt ttgtgtttgg tttagaggtt 1740
tttggggttt aatgtttgag agtgtgtgtt tgaatttgta gaaatttgtt ttggttgttt 1800
tttgttaggt tgttgaaaat tgttttgtaa gggttggttt tggggaattt ttgtgaaggt 1860
ttgattgggt ggggttgtaa ttatttgtta tttagttgtt ttttttgaat gaatttgtgt 1920
atttggagtt aattttttttg aaatttaatg ttggggtaat ttaaattttt ttgaagtttt 1980
tttttttttt tttttaagtg ttatttttgg ttgtgattat attggtaggt tgggtaggaa 2040
ggatttgttt ttgttgtgat tttgtttaag ggtgagtttt gttggtgttg gttaaattag 2100
atttttttat tttgagtttt ttaaattttg gtttgtttgg ttgtttggta ggatttgggg 2160
gtggggttaa aatgagagag aaagggaatt tataatttat gagagaaaga gaagaggtag 2220
gtttggtggg agggggggtag ggatggtgtt ttttaggtat tggtttgaga gtttggttga 2280
gttgtttgtg gagtggttgg gtttgttaaa tttttggggt ttaatggtga gggtttttttg 2340
tggtgtattg gtggggattt tgattgatat tttgggtgga gaaagttttt gttttttttt 2400
tttatttgtt tttgtttttt agtgttttga gtatataata tttatttatt agttttagtt 2460
tttgtattag tgtttgaaga gattgattta ttgattttaa attgaaaagt tttttatgta 2520
ataataatta tttaatatat ttgtagttgt tgataaaatg gttagataaa aaggttaaag 2580
tttttgtttt tttttaattt agggtaataa aatagatata tatttgaatg tttgagtaaa 2640
gtggggtttt tagtaggttt tttttatttg ttggaggagg tattttgttt tttatgaagg 2700
tagttttagt gattttttgt ggaaaattta gtagtgtggg tagtgttgtt ttgggtttgt 2760
ttttgtagat ttgtattatg ggtgtgggtg gggttgagga tttttgggat gttatgtttt 2820
tttattttag taatgtttgg gattatttta aaagttgaaa tttgttaata ggtgaagtat 2880
tattttttttt attattttta gatttatatg ggtagaaggt atgtgttttt taaaagttgt 2940
gttttttgtg tgattggtat gagggggtgg tagtgttttg agatgagtag gagaatagtt 3000
gttattttgt ttttaattgt atttttaaga ttagttgtta agtttttttt ggtttttggt 3060
gtttaagaag ttggtgggta ttaaaattta ttagagatag tttgggatat ttggggggat 3120
ttatgataaa atgttaagaa ttgttagtta gtttttagtt tattttttgt tgttattatt 3180
attgtttttt attggttgtt ttattttgtt ttgaattaat agtaaattag agaaaattgt 3240
aggagaggat gaaaaataaa ttaaaggaga aagattatat aaatttttaa atttatttaa 3300
agtatttgga gtttgttgat ttgggggagg ttttttgtag ggaaatttag ttttttttat 3360
ttttttagat taatattgtg gggtgattat tgattgggaa taggaattta ttagtagatg 3420
gtaaaggaaa atgttgttaa agtggaaatt tttttttttt aagtttgggt gaatttgttg 3480
tttgttatat tttttagttt tgttgggtgt tggttgagtg tattttataa gttgggttta 3540
gtggtgtgag gaaagtttgg gggattgggg gtggaaattt gagtttgtag ttttggaagt 3600
gagttgtgtg aggttggttt ggaattgtgt gtattttaag atttgtgtta tgtaaattat 3660
tgttattatg aattgttttt ttagattgtt tgttgatttt gaaagatatt tttttttatt 3720
tgatttatgg gaagttgttt attttggttt gttatagggt atagtgtgtt ttttttagtg 3780
ggggatttgg atattttgtt tttttgggtt tgtggtttgt ttttaggggt agtgtagttg 3840
ttatgtatat tgtgtggggt ttgtgtgtat aaaagtgtgt aattgtgtgt tagttgtagt 3900
aattattgta tttttttttt tagtttgtgt atttgttgtt tatataggag gtttttgtgt 3960
ttttagaata atagagggtt gtattttgta gtttggttgt tagaggaggt ataaagagtt 4020
gagttggttt tagaataatg taatttgtgt ttttatttgt tttttttttgt gggttgttag 4080
tttttttttgt ggttgttagg tgtgtataat tagtgtttgt ttaatgtaaa ggtggagttt 4140
gtagttggtg gtagggatgt attaattaat ttgaattttt aattttttag tatttttttt 4200
tgtgagtgtt tagggttttt tggagttttg gtaaattttg gtaaatttta gtaaatgttg 4260
ggattggggt aaggatggtt ggtgagtaag gtaaagtgtt ttttatttta gttttttttt 4320
ttatgttttt ttaaagtggt gaatgtgatt tttttaattt tatattttgt ttttgttgta 4380
ggtagtgata ttggagttgg tttgagtttt ttaagtttgg ttagaaagtt tttgtgttaa 4440
tgttaaggtt agagatgggt tttaggagtg ttgttgtatt ttggttgttg gtttttttgtg 4500
ggggtaaggt tgtgttttta gttaatgatt aaataagttt gtttatatgg tgggtgggag 4560
agtagagagt tttttttttt ttgtagagta aagtttttta attgtttttg tatagggaga 4620
ttagttagtt tggaaaattg aaatgtgttg tttaaaagag atttgaagtg tatggggttt 4680
tgaataaggg taggttttgg tttttgtggg ttttggaaag atagggtttt tagaggaaaa 4740
tgtatatttt tattttgttt tttagtattt gggaagattg gaaatagggt aaaggttggt 4800
atattgaggg gagggtgaat gaaatggggg ggggttggtt aatgaaagtt tagggataag 4860
gagagagtaa gaaagaaaaa gaaaagggag aagggaaagt aggggaagag tggaagagaa 4920
agagaaaatg gaagaagaaa taaaaatgag aagaaagagg atgtgtatag gaaagagaag 4980
gaaagaatta agagaagtga tttggtgtgt agatttgggt tataagtatt ggatttggag 5040
tttttttttt agtagtttgg tttttggtta gttttggttt tatttgttag gtgtaaggtg 5100
gatatgtgtt ggaatttggg tttttgaagt tatttttata ttttgtggaa ggtttttttg 5160
ttagatttga gtttatttta tggttttgga ttttggagta ggttggagag agttttttga 5220
gattggtata gtatttttttg tgtaaagatt gtttggggga tattgtagtg ttttagtttt 5280

gttgtgatgt gttgggttat ttttttggtg ttgatttttt ttagttggtt tgatgtggtt 5340
atttgtgagt ttgatgagga tgagggtggt tgtttgtgat tgggtgttag tattggtttg 5400
tgagattgag tgtggtttgg gtggtgtagg ttgtttaggt gtgatattat ggttgtaggt 5460
ggggtgttta ggttgtggga taggtgttgt ttattgtggg ttagtatggt agtgtggtgt 5520
gtgttgaagt tggggttgag tattttgttg tggtttggtg gattgtagtt gggtagattt 5580
tgttgtgtgt tgtggaggtt gtttagtttg ttgtttagtg gtgtggtggt tagtggggat 5640
aggttttggt ttatgttggg tattttttttg tagggattgt agaggttgtt tatggttggg 5700
agtttgtgtt tgttgtgtat gagggtgaag ttgttgttga tgttgttgga taggtgttgg 5760
tggtggtggt ggtggtggtg gtgtggatgg tggtgtgggt gagggtggtg gaatttgtta 5820
gatatggtgg agatgggtgg tagtggttgg agtggtgtta gtgtggtgta ggtgttgttt 5880
atgtttatgt taggtggaga tgagttgtag gatatgttta tggtgtggtg tagtgggatg 5940
gagagtttgg gttggtagtt gttgttgttt aggattgagg ttatgttggt gattatggtt 6000
gagtgtgatg ttgttgttgt tgttgttgtt gttgtgttta gtttttggtg tgtggttgga 6060
ggtggtggag ggttttgtag tgagttagtg gtgttgtggt ttgtgtggtg ggggttgggg 6120
ttggttagta gtttttgttt atggtttggg tttttgttgt tgtttttgtt gtttttgttg 6180
ttgtttttgt tattgttgtt gttggttggt ttgtgtaaag tgtttagatt ttttattgtt 6240
agttttgggt ttatggtgta gtttttttagg tttttggtga ggtattgata ggtggtgtag 6300
gtagtttttta tttagtttat tagggtttgg gggtggtggg ggtggtgggg gtggttggtg 6360
ggtgggtaag gtgtgtgtgg tggggtttgg ttgtgggagt gggggtgggt gtgggagtga 6420
gtggagagga gtgtgtgtgt gggtgtgtgt tttgggttgt gggtgggttt gtttggggtg 6480
ggggtggggt gggggtggat ggggtgtgtg tgtgggagag gggaggggat ggggagggag 6540
ggagggatta ttgggttttg ttgtttgggt tggtttgtag ttttgttatg tttgagtttg 6600
ttttggtgtt gatgtttttt gtttgggtga gtgggagttg tttagaaggg ttttgttgtt 6660
tgttggggta gttaatttat ttttttggat ttggggtggg gttatgtttg atgggtgtta 6720
agtgtaatta ttttgaggtt gtagttgttt tgggaggtgg gttttttatg ggaagttatt 6780
agtggttgtg gagggggtgg ggttgtattt tttaggtttg gttgatggtt tggtgttttt 6840
tttatttttt ttgttattgg aattaaatgt taaggagagg gagggaggag aaggtggggg 6900
ggttgttggg gagagggagg ttggagtgtg agtgagtgag agagatttgg tgaaagatgt 6960
tgtgttgtgt tttttttgag tagttgtgat ttgggggaag gggtattgtt ttaattgttt 7020
ttgttttatt ttaaagtttt tttggaggtg agtaagttgg gagtaaaaga ttttgttatg 7080
tgttagtata gataaatatt tgttattaat aagttattta ttgagagttt ttagttggga 7140
gttaatagtt tagtttttta aaatgtaggt gttaatgtat tttaatgtat ggtaaatttt 7200
tgaggttggg atatgtataa tttatgtggt tagagtaatt gtagtgtttt tgttgttagt 7260
gagttgtggt tggttggtgg tttatgggtg gtgtgggtag tagtaatgta tgtaaagtta 7320
atataataat attatttaat tatttggtga gtttataaat tttagttatg aggtttttga 7380
agagttttgt tagattgtaa aataaaatta gaattttggg agttgttgtt ttaaggtgat 7440
taagtgtttt ttgtattagt gatttggaaa gaagtgggga gagtgtagaa ttggtgaggt 7500
ggaggtgagg agggttgttg gtgttttaag tgagtaaatt ttttttgggg gtagagagta 7560
agtaaattag ttgagatggt aattatggat tttttttttta atatgagata tatatatgtg 7620
tatatatata tatatatata tatatatata tttttgtttt ttttaaattt tgggagagaa 7680
atggtggtgg gtagatgata aggaattttg gttgatattt gtatatttaa attttgtttt 7740
tgaaaagatt agtgttttgg ttagtttgag aatgtttata agttaaggtt ttaaaaattg 7800
tatttatgtt tttttagaat ttttgttgtt tggtaatttt aggtttggag gtgggagtgg 7860
aattggggga tagagagttg gagatttttg tagtttgagt tggggtggag gggtagttga 7920
agatagagag gttaatattt ggtataaagt tttaaatgta ataagatttt tttaaagtag 7980
gttgtgtttt gtttgttttt gaagatttaa gttagttgag gttgtgtaga 8030

<210> 221
<211> 2225
<212> DNA
<213> Artificial Sequence

<220>
<223> chemically treated genomic DNA (Homo sapiens)

<400> 221

tatttgggtg taggtgggtt gagtttgaaa agagagttag tgaagggaga taggggtggg 60
gttgttttat aggatttggg aaggtaatgg aaaattatag ttaaaggggg ttgttttttg 120
gtgggtaggg gtgaatttta taaagtatat ttttaagggt ggggagaatt ataaataatt 180
tttttaaggg tggggaagat tataaagtat attgattagt tagggtgggg taggaataaa 240

```
ttataatggt ggaatgttat tagttaaggt tgtttttatt ttttttgtgg atttttagtt      300
attttaggtt atttggatgt atatttgtaa gttatagggg atgtgatggt ttggtttggg      360
atgtgatggt ttggtttgat aattattatt tatgttatgt ttattatttt aagttttatt      420
attattattt tatttatttt attttatttt ttttttatat atttgttttt attttggaga      480
ggttagatga gttagatttt agggaggttt agaagtgggt aaggggaaat gggaaaggag      540
gaagatggta tgggtgtgtt tggttagggg tgggagtgtt ggatggagtt tgggataaga      600
ggggttttgt agttattggt atataatgtt tgggagtttt tgttggtgtt gggattattt      660
tagtgagttt tgggagggaa ttgaagattt ttaattatta atgtatttgt ttttaaaatt      720
gatgggggga aggatatgtt taggtttaag gatatgtgta ggtttggatg attttgggtt      780
attagggagt ttttggagta ttttgatttt tagatgggtt tgatgaaatg agtatttgat      840
ttagtaggtt tgggtttggg tttgagaatt tgtgtttttt gtgagttttt gtgaggtaag      900
tgttgtaggt gtggggttag gagttaggtt ttgtttttgt gtttggagtt gtttttagta      960
tagggtttgt gagttttatt tttttgtggt gtggggtggg gttgggtgtg gggtgaaaga     1020
ggtgaagtga gagtggaggt tgtattttag tattgtgtag ggattggtga gtgttgtttt     1080
tgggggtagt gtttagtaat tgtgttagga gtgtggagaa gggtattggg agagtggtgt     1140
ttgtggtgga gattagtgtt ttggagtatg ggtatgatgg gggtattttt ttggttgtta     1200
gtaattaata ataataataa ttataattat agtaagggtg ttgatgggtg ggtttggagt     1260
atgtttgatt ttggttttta ttaggttgtt taggtttttg atgatgtatt agaaatattt     1320
ttttaatttg tggttttttt gtaggagagg ttgggaaggg gtgggggatg gggtttgggg     1380
gaggtttttg agggatttta gtaagtgggg aagggtgttg ggaaagtttt agatttatgg     1440
ttgtgtgggt tatgagttta tttgaatgtt gattattgtt ttttgttgat ttttattttt     1500
tgggaatgtg tgaaagtaaa tttaagttag attgtggagg ttgttgggga gggaaggttt     1560
aaggagtttt tgttgatttt gttgaataaa gggggttttg agttgggttg agatggggta     1620
tgtgtgggaa gatttttgtt tgttgttttt ttttattgtt ttagtggatg ttatgtttgg     1680
ggtttttttg gtgtgtgggg ttgatgtatt tttggggttt attgtagttg gttgggattg     1740
tggagtgggt gtggtggatg aagggaggta ggatagtttt gggggtggta gaaggagttt     1800
gggtatagtt gagatttgtg tttttatttt attaatattt atagtaggtg ttgttgagtt     1860
gggtaattgg gatggtttaa gttatttggt taaattttaa attatgtttg ttattgggaa     1920
gtagagttta gtgatgttaa ttgtgttttt atttttatta ttggtgttag ttttaaaggg     1980
tttttaaaat ggttgtgtta tttttttagtt ttggattgta gttgttggtt aggaatttta   2040
gtgttatggt ggatatgttt tttttgtgtt tttgttgttt atttgtttat ttagtttagg     2100
ggttttggta ggtagtagtg tatttggttt aaagggtaag atgttttttt ttttatttat     2160
aataaattta aatattagta gggtttgggg ggaaaaatgt ttttagaaga aaaggtgaat     2220
gttag                                                                 2225
```

<210> 222
<211> 2225
<212> DNA
<213> Artificial Sequence

<220>
<223> chemically treated genomic DNA (Homo sapiens)

<400> 222

```
ttgatattta tttttttttt tgaaagtgtt tttttttttta aattttgttg gtatttaaat      60
ttgttatgaa taaaagagag aatattttgt tttttagatt aaatgtattg ttatttatta     120
aagtttttga gttgggtgag taggtgggtg gtaagggtgt gagggaggtg tgtttattgt     180
gatattggga tttttggttg gtaattgtgg tttaaggttg aaagatgata tagttatttt     240
aggagttttt tgggattgat attggtggtg gaggtagagg tgtggttagt attattggat     300
tttatttttt agtaataaat gtgatttaaa atttaattaa ataatttggg ttattttaat     360
tgtttagttt ggtagtattt gttgtgagtg ttggtgggat gggagtgtag gttttagttg     420
tgtttgggtt ttttttgtta tttttgggat tgttttgttt ttttttgttt attgtattta     480
ttttatgatt ttggttaatt atgatggatt ttgagggtgt gttagtttta tgtgttgggg     540
aggttttagg tatggtattt attggggtgg tggggggggaa tagtgggtag gggttttttt   600
gtgtatgttt tattttggtt tagtttggaa ttttttttat ttagtaggat tggtgagaat     660
tttttgaatt tttttttttt agtgattttt gtagtttgat ttgggtttgt ttttgtgtgt     720
ttttaggaaa tagaagttga tggaaagtag tggttggtgt ttgggtgggt ttgtggtttg     780
tgtagttgtg gatttgaaat ttttttggtg tttttttttg tttattagag ttttttggag     840
atttttttttg agtttgtttt tttatttttt tttaattttt tttgtaggaa ggttgtgggt   900
taggggggatg tttttgatgt gttattagga gtttgggtag tttggtggga attagaatta   960
```

ggtgtgtttt gagtttgttt attagtgttt ttgttatgat tatgattatt attattgtta 1020
gttattagta gttgagaagg tgtttttgtt gtgtttgtgt tttggagtgt tagtttttgt 1080
tatgaatgtt gtttttttaa tgtttttttt tgtgttttta gtgtggttat tgggtgttgt 1140
ttttagaagt gatatttatt ggtttttgtg tagtgttgga gtgtggtttt tgtttttgtt 1200
ttgttttttt tattttgtgt ttagttttgt tttgtgttgt gaagaaatga aatttataga 1260
ttttgtgttg agggtggttt tgggtgtaga aatgaaattt agtttttggt tttgtatttg 1320
tagtatttgt tttgtgggaa tttgtgggag atgtaggttt ttgggtttga atttaggttt 1380
gttgaattag atgtttgttt tattaggttt gtttgaggat taaggtgttt tggaggtttt 1440
ttaatggttt ggagttattt aagtttgtat gtatttttga atttaggtat gttttttttt 1500
ttgttggttt tgaaaataga tgtattggta attgggggtt tttagttttt ttttagggtt 1560
tattgggatg attttagtat tagtagggat ttttaggtat tgtgtgttaa tggttgtaga 1620
gttttttttg ttttgaattt tgtttagtat ttttattttt aattaggtat atttatatta 1680
tttttttttt tttttgtttt tttttgttta tttttaggtt tttttggagt ttggtttatt 1740
tggttttttt agggtggaaa tgggtgtgtg gaaggaaaat aaaataaaat aaataaaata 1800
gtaataataa agtttaaaat aataaatata atataggtaa tagttgttag gttaggttat 1860
tgtattttag gttaggttat tgtatttttt gtgatttgta ggtatatatt tagatggttt 1920
aaagtaattg aagatttata aaagaagtaa aaatagtttt aattgatgat attttattat 1980

tgtgatttgt ttttgtttta ttttaattga ttaatgtatt ttgtaatttt ttttattttt 2040
aagaaggtta tttgtaattt tttttatttt tgagaatgta ttttgtgaga tttatttttg 2100
tttattagag aataattttt tttgattgta atttttttatt atttttttaa attttataaa 2160
atggttttat ttttattttt ttttgttgat tttttttttg gatttagttt gtttgtattt 2220
aggtg 2225

<210> 223
<211> 3377
<212> DNA
<213> Artificial Sequence

<220>
<223> chemically treated genomic DNA (Homo sapiens)

<400> 223

tatttgtggt tttatgagtt tgtattgaag ttggatattt ataatatttt ttttaatatt 60
ttattatttt ttttttttga taattatgtt ttgagaattt gaagtaatag atatgaagag 120
ttttaaggga ttgtattaat gtatgttaag attagttttt attatatagt ttaatgatat 180
ggttttaggt ttagaaggga tgttttatat ttatagaatt ttattgagtt taggtgttag 240
tttagagaat ttattatttt tttgtttttt tttaagagtt aattgatggg atagtatgta 300
aagtatatgt gtttttgttg ttaaaatagt agttaaaatt gtttgtttag atgtgtaaga 360
ttttatgaaa tgttttggta aaagttagtt taggagtgtt ggttaaggtg ttgaagtgaa 420
gtaaggggag ttaggattat ttttgttatt gaattttttt ttttttattt ttttttaaag 480
atagtgagtt tatttatttt gataagatat tttaggattt taaatttgat ataattttgg 540
agaaagtagt gtgtgtgtgt gtgtgtgtgt gtgtgtgtgt gtgtggtgta gaagggtatt 600
tgggatatat aatttttata gaaattttag tgttataaaa attttaggag gatttatatt 660
taaaaaaata tataatagtt taagttgttt attttaattg aatgttgtgt gtttttatta 720
gatttttagt gagatttagt ttaatgagtt aattgtaaaa taaagttttt gttaagggaa 780
tggtagaaaa tgataaaatt ataattttga atgattattt aaaagtaaaa agtatttttt 840
attattgtgt ggagattttt ttatttttat ttttatttga attgaatttt tttttttaat 900
gtttttttga atagtttgtt tttggtgttt tgaaaatgtg tatgttgata gtagtattat 960
atgttgtttt tggtatagag tgagttaata gttgaattat ttttgggtaa ttgtggattg 1020
tagttagtgt aagtgtaaga gaaggtattt tggttattaa tttgttttat attttaatta 1080
tttttgtttg ttttatattt tttgggtatt tgatgttttt ttttttttgtt aggtaatttg 1140
tttttgtttt gtttaatttt ttgattttttg gttttagaga atagttttgg ttgtgggaat 1200
tggttttggg aagtttttgg tgtttttttt attggtgatt ggtaagagta atatttttat 1260
atttttaaaa agagttgttt agtttttaat gatatttttt ggtaaaaaag aaaatgaaat 1320
atataatttg gattttggtt tattaaatgt aggttaagag tgtggttgtt gagttttggt 1380
tttgttgtga ggttgttgtt tttatttgtt ttttgttttt attagagttg atttgttttt 1440
tgttattttt ttttggtagt gtggatttat tgtaatgtag gaatatttgg gttggtatgg 1500
ataggatttt agtattagat gttggttgtt gttttaggga ttttttgtgg aaattattta 1560
gtatttagaa gttgttttaa taaggaagta attagtaaag tattgttggt gggagaattt 1620

tgtgaaatat tttagaaaaa tattagaaat tgttggagat ttaatgattt atgttttttt 1680
tagtttgtgt tttttagttt tttttggagg agtattaagt ggtttgtggg tttttatttt 1740
gtggtgggag ggttgtttta agtatattta tttttgtgat agttaatgtt gggggttttt 1800
agttttaatt agggtgagga ttatggattg tatttttttt ttttagtgta ttggtttgtt 1860
tattggaggg tgagagggat gtgtagattt ttggatttag gggttttgtt ttgtagattt 1920
ttttatttta tttggggttt ttggtatttg tattatttag tattagtgtg attggtggtg 1980
tattttggtt tttttgtgtt ttattaaagt ttttgtatat ttttttttat gttgtgtttt 2040
tgttatttat gtagttgttt ttttgagtgt tttgtgtgta taaatgtgtt tttggttttt 2100
atgtgttggt ggtgttgttt tgttgaggtg gtgttggggg agtttgtaga ggtggtgggg 2160
gagggagtgt ggaggagagg atttgtgagt tgtggggatt tgggtttgtt gagtttttgg 2220
ggaagttggg ttttgttttt tttgggtgtt tggggtgtgt gggaaggtgt ggaggatgtg 2280
ttgtatttat ttgttgttgt tgttggttgt tgattgttat tgtggtggtg gatgtggtgt 2340
tgtgttggag tttggttgtt tgtttaggtt ttaataggtt ttgggtgaat ttgtgggttt 2400
gtttattggt tattgttggt tatgttaatt gtgttgtggg tttggggtgt gttttttgtt 2460
ggtttttttt gtgttaattt tttttattta tttgttgttt aggaagtgga atttggagtt 2520
tttttttttt ttttttttgt ttaatttgta agaggtgggt tttttttttt tgtattttta 2580
ttttttttat tttttttttt tttttgaagt tttttgaaga ttttttaaat tttgtgtttt 2640
tttgggtgtg tgtggttaat ttggttggtg gttgggtttt gtagtggtgt atttggagtg 2700
ggtgttgtgg gatgtgtgtg gggtggtttt tgtttgtgtt gtttatatgg gtttagttgg 2760
ttggttgggg ttgtttatgt tgtttggggg aattgtaggt tgtgggggag ggtggggggt 2820
ggttgttgag gggttttggg tggggtttgt ttaaggttgt tgtttgttta aggttgttag 2880
ttttatttgg aggttgttgg gagagttgga gttttggatg tatttgttgt ttagattttt 2940
atttttttggg tttggttagt gggtgggggt taagtttggt ttgttttttt atttttggga 3000
aggggttaga ggtttattaa agatttggtg gttgggattt tagtagtatt gtttaagtta 3060
gtgtttggaa ggtttttttg ttagaagttt atgtttaggg ttttggaata agttttttta 3120
tttatttatt tttttaatga gtatttatta agtgtttatt atatgttagt ttatattgtt 3180
ttggaaattg aggatatgga gaggaattta gtaattatat tttaaggttg tgtagagttg 3240
ataggttttt ttggggtagg ggtgggagat agtaagtaat taagtgaata aataaataaa 3300
tatagagaaa ttaaggagat agtttaagtt attaatatga atttaaaatt aataaattag 3360
tgttttgggg gtaagag 3377

<210> 224
<211> 3377
<212> DNA
<213> Artificial Sequence

<220>

<223> chemically treated genomic DNA (Homo sapiens)

<400> 224

tttttatttt tagaatgttg atttattggt tttgagtttg tgttagtagt ttaaattgtt 60
tttttgattt ttttgtattt gtttatttgt ttgtttggtt atttgttgtt ttttattttt 120
attttgaaaa aatttattag ttttatatag ttttaaagtg tagttgttaa gttttttttt 180
gtatttttag tttttagaat agtgtgagtt ggtatatagt aggtgtttaa taaatattta 240
ttgaaggagt gaatgaatgg ggaagtttat tttaaggttt tgaatatgga tttttggtaa 300
gggagttttt taaatgttgg tttgggtgat gttgttggga ttttagttat tagattttta 360
ataagttttt gatttttttt tgagggtgga agggtgaatt gagtttggtt tttatttatt 420
agttggattt agaaaataga agtttaaatg gtaaatgtat ttagggtttt aattttttta 480
gtaattttta aatgaggttg atggttttgg ataggtgatg gttttggata ggttttgttt 540
ggagtttttt ggtggttgtt ttttgttttt ttttgtagtt tgtagttttt ttgggtggtg 600
tggatagttt tggttggtta gttgagtttg tgtgggtggt gtgggtagga gttgttttat 660
atgtgttttg tgatgtttgt tttagatgtg ttgttgtagg gtttggttgt tagttgagtt 720
ggttgtgtgt gtttggggga gtgtagagtt tggaaagttt ttgagaagtt ttagaggagg 780
gaagggggtg ggaagagtgg gggtgtaaga gagaaaaatt tgttttttgt gagttgagtg 840
aggaagaggg gggggaaggt tttgagtttt gttttttgaa tggtgggtgg atagagaagg 900
ttggtgtggg gaagattggt gggaggtgtg ttttgggttt gtggtgtgat tggtgtgatt 960
ggtgatggtt ggtgagtgga tttgtaggtt tatttggagt ttgttgagat ttgggtaggt 1020
ggttgagttt tggtatagtg ttgtgtttgt tgttgtggtg gtagttagta gttggtagta 1080
gtagtgggtg agtgtggtgt gttttttgtg ttttttttgtg tgttttaggt gtttgggggg 1140

aatggagttt agttttttta aaagtttggt gggtttaggt ttttgtagtt tatgggtttt 1200
tttttttgta ttttttttt tgttgttttt gtgggttttt ttggtgttat tttagtgagg 1260
tagtgttatt gatgtgtaag gattgggggt gtgtttgtgt atgtgggatg tttagaggag 1320
tagttgtgtg ggtagtagaa gtgtagtgtg ggagaggatg tgtaggggtt ttaatggggt 1380
gtgagggggt tggaatgtat tgttagttat attggtgtta agtgatatga gtgttagaga 1440
ttttgggtgg ggtgggggga tttatagaat agaattttg agtttggagg tttgtgtgtt 1500
ttttttattt tttagtaaat agattagtgt gttgagagag agggatgtag tttgtggttt 1560
ttgttttggt tagagttggg gttttttgat gttaattatt gtagaaatag atgtatttgg 1620
aatagttttt ttattgtagg gtaaaaattt ataaattatt taatattttt ttagaaaggg 1680
ttgaggaata taggttggag gagatgtggg ttattaggtt tttagtggtt tttgatattt 1740
ttttaagatg ttttgtgggg tttttttatt agtaatgttt tattggttat ttttttattg 1800
aaatggtttt taagtgttgg gtgattttttg tgggaagttt ttggggtagt agttagtatt 1860
tgatgttagg attttattta tattaattta gatgtttttg tgttgtagta ggtttatatt 1920
gttggggaga ggtggtaaga gataaattaa ttttagtgga aatgggaagt gagtgggaat 1980
aatagtttta tagtaagatt ggggtttagt agttgtgttt ttggtttgtg tttggtggat 2040
tggaatttgg gttgtgtgtt ttattttttt ttttgttaga aaatattatt aggaattaaa 2100
tggttttttt taagagtgtg aaagtgttat ttttgttagt tattaatgag gagggtgtta 2160
aaagtttttt agagttagtt tttatagtta ggattatttt ttaaagttag aggttggaaa 2220
gttgaataga atggaagtaa attgtttggt agagggaaga ggtattgggt gtttaggggg 2280
tgtagagtgg gtgggggtga ttggagtgtg aaatgaattg gtggttaggg tgtttttttt 2340
tgtatttata ttagttgtag tttgtagtta tttagaggtg gtttaattgt tagtttattt 2400
tgtgttaggg atagtatgtg gtattgttat tagtgtgtat atttttagga tattaaagat 2460
aaattgttta aaagaatatt agaagaaaag atttaatttg agtaaaagta gagataagaa 2520
aatttttata tagtagtaga aagtattttt tatttttgaa tgattattta gaattgtggt 2580
tttgttattt tttgttattt ttttgataaa aattttattt tgtaattgat ttattggatt 2640
gaattttatt gaggatttga tggaaatata tagtgtttaa ttggaataaa tagtttaaat 2700
tgttgtgtgt ttttttaaat gtagattttt ttagaatttt tgtggtatta gaatttttat 2760
aaagattatg tattttagat attttttat attatatata tatatatata tatatatata 2820
tatatgtatt gttttttta aaattatgtt agatttgaaa ttttagggta ttttgttaaa 2880
atgaataagt ttgttgtttt taaaaagagg tgggggaggg gagatttagt ggtaaaggtg 2940
gttttgattt tttttgtttt gttttagtat tttggttaat atttttaaat tagtttttgt 3000
taagatattt tatgggattt tatatattta ggtaggtagt tttggttgtt attttggtag 3060
taagaatgta tgtgttttgt atattatttt attagttaat ttttgaaggg aaataggaag 3120
gtgatgagtt ttttgagttg gtatttgaat ttggtgggat tttgtgggtg tagagtattt 3180
tttttgaatt taaaattgtg ttattggatt atgtggtaga gattaatttt agtgtatatt 3240
gatgtaattt tttaagattt tttatatttg ttgttttaag tttttaaaat atggttgtta 3300
agggagaaaa atgataggat gttggggaaa atattatgga tatttgattt tagtatagat 3360
ttatagaatt gtgggtg 3377

<210> 225
<211> 3043
<212> DNA
<213> Artificial Sequence

<220>
<223> chemically treated genomic DNA (Homo sapiens)

<400> 225

aaaatttaaa tttataattt gaggtatttg ggaagttgtt tttttagggt ggaatggtta 60
tggaaattgg gtaggttggg ggttttttttg gggaatgagg gagtggagga gttttttgtt 120
ttttttgtta gattagaaag agaaatgttt taagaatttg ggttttttatt ttttaggtgt 180
tttttttgga ggttttttag ggattgttta tagtgttttt ttttagtttt tttttttttt 240
ttattttttt ttttatttgt tttgttttgt gtttgttttg ttttttggtt tgtggggttt 300
taaggatggt tggttgtttt tgttatagtt atttggttgt ttagagtggt ggggtgtatg 360
gggttgagag agttagtttt agggtttggg gttgggaatt gaagtttggt gtgaaggaag 420
tgggagtttg ggttgaggag gtgaagggga aaggaaaagt gaggggaatt tgagtgggag 480
ggttttgaga ggagtgggag gttgtgggaa ggggaggttt ggtatttttg ggggttatgg 540
gggttggggt gtggtttttg gtttgggagg gtgtgggttt tttttggtag tgttgtttgt 600
tggtttagtt atgtgtgttg tgtgttgtga ggttggtggg ggttttgttg ggtttggggg 660
tgtttttggg ggttgtttgt gtttagttat ggtagggtgt tttttggtga aatggggttt 720

```
gaggtgggtt ttgattttgt gttggtgttg tggattgttt gggagttgta gttgtgggtg     780
tttggtgttt ggtttgtagg tagtgttatt agttgattgt attgtggtgg ttataattat     840
taattttatt gttattaaaa taaggtatag tattattgtt gtttggttgg gaagaagatg     900
ttggtttggg tagtttgtag tttttgagag aagatgtttg agaagtgtgg tgttggtgtg     960
ggttttgtgt agtttgtttt gtgagtgttt gttgtaagtg tgaggaaatt tgtggttttt    1020
ttagatatag ttaaattgtt agtttttttt aggagttata gaagatgaaa tagttttatg    1080
ttaggaaagt aaaattttttg gaggtgaagt tttttatttt atgtaatggt taatattgta    1140
tgttgtgatt tattgtgttt atttgttatt gtttagtaga gtatttatta agttagtaat    1200
ttagttgagt tttaatttaa ttaatgaatt gtttgtttgt tataatgtgt tggggtatta    1260
atttgagatt gtaatttttt ttatgagttt agttattagg tagtgtagtt agttgattgt    1320
taatagtatt aattattaat tatatggtta ggtaaaaaga tttgggaatt tgtttaaatg    1380
agttgtttgt gtattattaa tgtgtgtggg gaagaggggt gttggaaaat gttgatttta    1440
tttattgttt attaattgtt tagttaaaag aaaaaaatta atattttagt tattaagttt    1500
ataatgtatg taatgtgtat gtatgtgggg ttttgttttg tttttttttt aatatttttt    1560
taggttttta attaaaattt tagatataag ggggaatatg attttttttt tagttgattg    1620
atgtatgtta ttatatgaat atgtgattat taattttttg agattatatt gttagtaata    1680
ttttgaaagt aatattgtta gtaatatttt gaaagaataa agtgttataa agatataaat    1740
tgtggttatt gttatgtgtt tatttttttt gttaaattga ttttatgatg tttattttag    1800
tttttgttat agtaatttta atttttttat taatgaagtg gttaagttgt ttttgttatg    1860
ataaaaattt tttgggaaag ttatttatat atatagattt gttaatattt ttataagaaa    1920
tatattaata aaatttttta tatttttttt ttttatgaag agattttgtt tatttattaa    1980
gatgtatttt tttttttaag taattattta taattgttgg agtttatgta atttttagtg    2040
ggagtgatta gtaaatgtat ttatatttta aatttagttt gttttttaag atttaaagga    2100
tttagaattt taaaattata gaaattgtat atatgggtat gtatttgttt agtttaggtt    2160
tttagttttt aagggttagg tttttaggtg tgaatttttt agtgtaatgt ttttagaatt    2220
tttatttagt aggtgttgtt tttggtaatt tggtttggaa gtggggattg gggtaatggg    2280
gttgaggaat ttaaagttta tttagttatt tttttggggg gaggggagag gatgtggtag    2340
tttattgagg atataagaag ttaaattatt tttttattga ttttgatagg atataattta    2400
aagttgatgt tttgattttt ttatatttaa gatttggatt ggttttaaaa tttgattatt    2460
gtaggggtgg gtttttgatg gtttttatgt gtgtggtgga ttttaatatt tatttaggaa    2520
attgagggta gttggtaggg gttggtatta atttggtggt ttttggaggg gtggggtagg    2580
ggtggggtgg ggggagggga gaggatgttt gtagaagtgt ttttagttgt tatggaaatg    2640
ggatttagtg aagaatttag tggttgaaaa gagttttagg aaatgtgttt tttgggatta    2700
ttggtatttg ttggtttttt ggaataagat gttaatttgg ttaagtagtt ttggattttg    2760
gtgttgatgt atttttttag tgaattttag ttggtgttgt gtagagatag tagttagtgt    2820
ttgttggtgg tgtggtttag gaggagtaga gggtttggtg agtagaatag tggttttttg    2880
ttagttatat ttgttttatt gtaattggtt tgtaggtatt agagtttttt ttaggagaaa    2940
ttgtaatggt atatataaat agttataagt atatataaat gtatgtataa atatttttttg    3000
aatttttttta gtaatttttt tatttgaaaa ggattagtgg tat                     3043
```

<210> 226
<211> 3043
<212> DNA
<213> Artificial Sequence

<220>
<223> chemically treated genomic DNA (Homo sapiens)

<400> 226

```
atattattga ttttttttgg gtggaagggt tgttggaagg gtttaggaaa tatttatata      60
tgtatttgta tgtatttata attatttgta tatattattg tagttttttt tggaaagggt     120
tttggtattt gtaggttagt tatggtgagg tagatatgat tgataggagg ttgttatttt     180
atttattaga tttttttgttt tttttgggtt gtgttattgg tagatgttga ttgttgtttt     240
tgtgtagtat tagttgagat ttgttagggg gatatattga tgttgagatt tagaattgtt     300
tagttaagtt ggtattttgt tttgaggagt tagtaaatat tagtaatttt ggggggtata     360
ttttttggga ttttttttgg ttgttaggtt ttttgttggg ttttgttttt atggtaattg     420
aggatgtttt tgtaagtgtt tttttttttt tttttgtttt gtttttgttt tgttttttttg    480
agaattgtta agttggtatt agtttttgtt ggttgttttt ggttttttag gtaggtgttg     540
ggatttgtta tgtgtgtggg ggttgttgga agtttatttt tgtggtgatt aggttttagg     600
```

```
attagtttaa attttggata tgggagagtt aaaatgttaa ttttgggttg tgttttgttg     660
gagttagtgg gggagtagtt tagtttttta tatttttagt gggttgttat atttttttttt    720
tttttttag aaagatagtt aaataaattt taaattttttt agttttattg ttttaatttt     780
tatttttaaa ttagattgtt agggatagta tttgttaggt ggaagttttg agaatgttgt     840
gttggagaat ttatgtttga aagtttagtt tttgggagtt agaggtttga attgggtgga     900
tatgtgttta tgtgtatagt ttttgtgatt ttaaaatttt gaatttttta aattttagga     960
aataagttgg atttaaagtg taagtgtatt tattaattat ttttattaaa agttgtatag    1020
attttaatag ttgtaaatga ttatttaaaa gaagggatat attttaatga gtgaatagag    1080
tttttttatg gaaaaaagaa gtatgaaaaa ttttgttaat gtatttttta taaaagtatt    1140
aataagtttg tgtgtatgag tagttttttt gggagatttt tgttatggta ggaataattt    1200
aattatttta ttgatagaaa agttaagatt attgtggtag gaattgagat ggatattata    1260
aagttaattt agtaaaggaa atggatatat agtaataatt ataatttatg tttttatggt    1320
attttatttt tttgaaatat tattaataat attattttta aaatattatt aataatatag    1380
ttttaagaag ttaataatta tatgtttata taataatata tattagttag ttaagaaaaa    1440
aattatattt tttttatat ttaaaatttt ggttaagagt ttgaaggaat attgaaaaaa     1500
aaataaaata aaattttata tatatatata ttatatatat tgtaaattta gtagttgaaa    1560
tgttgatttt ttttttttgg ttgagtaatt aataggtaat ggatgaaatt agtatttttt    1620
aatatttttt tttttatgt atattgatga tgtataggta gtttatttgg atgaattttt     1680
aaattttttt atttggttgt gtggttagtg attggtatta ttagtaatta gttaattata    1740
ttgtttagta attagattta tagaaaaaat tgtagtttta agttggtatt ttaatatgtt    1800
gtgataggta ggtaatttat tggttgagtt ggagtttaat tgagttgtta gtttggtgaa    1860
tattttatta gatgatggta aatagatata gtgaattata gtatatagta ttaattgtta    1920
tatggatgga ggggttttat ttttagggat tttgtttttt tggtatgaga ttgttttatt    1980
ttttgtgatt tttagagaga gttaatagtt taattgtatt tggagaaatt gtgggttttt    2040
ttgtatttgt agtgggtgtt tgtggggtag gttgtgtagg atttatgttg atgttgtgtt    2100
ttttaggtat ttttttttga aggttgtggg ttgtttgagt tggtgttttt tttttggttg    2160
ggtggtggtg atgttgtgtt ttgttttgat ggtagtggag ttagtgattg taattattat    2220
agtataatta gttaatgata ttgtttataa attgagtatt gggtatttgt ggttgtagtt    2280
tttggatggt ttgtagtgtt gatgtggggt tggggtttgt tttggatttt attttattgg    2340
gggatgtttt attatggttg ggtgtaagtg gtttttgagg atatttttgg gtttagtggg    2400
atttttgttg gttttgtagt gtataatatg tgtagttggg ttagtgggtg gtgttgttgg    2460
ggaggatttg tgtttttttta ggttgggagt tgtgttttga tttttatgat ttttaggagt   2520
gttaggtttt tttttttgt agtttttttgt tttttttagg gttttttttgt ttaggttttt   2580
tttgtttttt tttttttttt tgtttttttg gtttgggttt ttattttttt tatgttaggt    2640
tttagttttt agttttaaat tttagagttg gttttttttga ttttgtgtgt tttgttgttt   2700
tgagtggttg agtgattgtg gtgggggtga ttaattgttt ttggaatttt gtgggttgag    2760
gggtggggtg ggtgtgagat ggggtgggtg ggggaggggga tgggggggggg gaggaggttg  2820
agagaaagtg ttgtgggtgg tttttgaagg gtttttaagg gagatgttta gaagatggag    2880
gtttagattt ttgagatgtt tttttttttg atttagtagg agggataaag agttttttta    2940
tttttttatt ttttaagaag gtttttagtt tatttagttt ttgtgattat tttgttttgg    3000
gaaagtggtt ttttaggtat tttaaattat aggtttgaat ttt                        3043
```

<210> 227

<211> 4908
<212> DNA
<213> Artificial Sequence

<220>
<223> chemically treated genomic DNA (Homo sapiens)

<400> 227

```
ttagaggtta tgttggttat tttttttgt ttgtaatgtt ttagatatat atgttttgta       60
atttagaaat ttgtggttaa gaaaggtgtt aagatttgta gaaaagttgg gatataggaa     120
agagaaatga aggttgataa tagattttat gggggtgagg gagattaggt taattagaga     180
agatagtata tgttagtttt agatgtagaa taattggatt tggatttaat aagttgagta     240
taagtggaga tgtaattaga aaagattgaa gtgaaatttt taagtaaaaa aaaaaaaaaa     300
aaaaaaaaaa ggatggagta gtagttagag gggatgttat ttttataaaa gataaataaa     360
ttgatggaat ggaattttgt agggagagag aggattagta gagagaggga ggtgtaggaa     420
aatgaggagt ggggtataaa ggtggggttt ttagggagaa gaggagttga ttttagaggg     480
```

```
ggagaagaaa aggatataga gttggttgta tagagaaaag gggttaaggt agagaagatg     540
gtgggatggt gtgagatgga gtttatagaa atatttagag aaggtgatga ttatatagag     600
gagtttttag aggggtgaat tttgaggaa gatgaatgtg gagggtggag ttagttagaa      660
atttagaaaa attagaggtg tttttggtag tgaaggaagt tgggaagggg ttaggttatt     720
agggtatgaa gtatgggaga aaatgggttt gaagagggag agtgtttgag agaaaaggaa     780
aattagagat taggaaaatt ataattgata taaagataag aagagatata aggaaagggg     840
tgtaatttga ggaaggaggg tttggtaagt aatttggtaa ttatagtgtt gggagagaat     900
gtataaaaga agggtagttt aggagttgtg gggtattgga agagatataa tagaaaatgt     960
aggtttttat ttagtaaaaa taagttatgt gtgaagttga ggtataaggt aagggaaggt    1020
tagaaatgag gtaatttgtg tatagattgg gtttggttaa tgttttaggg gtggggttag    1080
gtgggttgtt tttggggggt ggggttagtt ttggtttaat aaggggtatt tgatgtttta    1140
ttggttattt gttttgaaag gggaagata gtttgggtgg gtaggatgtg tggagggaga     1200
ggtagtggtg gtgtgaggtt taatttgaag tgttattggt gggattgata gttttgtgtg    1260
ttaagaagtt ggtagaaggg aaggggtggg atattagttt aggattttaa attgtattgg    1320
ttgtttgttt tggttgagag aggtgatgtt tgaagtttat tggtttttgt gagatagggt    1380
aagaaaagta gtgtgagttt ggtggttttt ttggttattt ttttatagtg tttttgggtg    1440
ttttttgat tgaatttgat tttattggag gttgtggtaa ttgtgaatta gttgtatgaa     1500
tatggttttt tttgtttttt tgttttttt ttttttagtt ggattttta atttaggtat      1560
ttttttttt ttttttgtg tttttttgt ttttgtgttt ttttttttt tttttgtttt         1620
gtgggttttt tggtgtgttt ttggtttttt atttgttttt tggggtatgg gaagggggga    1680
gggggatatg tatgaaatta attgttggta gtggttatag gagtaggaag tgttggatat    1740
agtgatttta tatgtttata taaaaatttg aataaggtgt gagtgtaaga gagatattag    1800
tgttttaagg agaaggtttt tggtttagat tagggtttag tatatgatag ggtttagta     1860
attatttgtg gaatgaatga aggagtgaag gaaaataatt tgaaagtggg gtaggtggtg    1920
ggtgtggtgg tttatgtttg taattttaat attttgggag gttgaagtag gtaggttgtt    1980
tgaggtaggt taggagttta agattagttt ggttaatatg gtgaaatttt atttttatta    2040
aaaatataaa aattagatgg gtgtggtggt ataggtttgt aattttaggt atttgggagg    2100
ttgaggtagg agaattgttt gaatttagga ggtggaggtt gtagtgagtt gggattgtat    2160
tattgtatta tagtttgggt tatagagtga gattttattt taaaataaaa aaaaagaaag    2220
aaagaaagtg ggatagggga gttaagtata gtggttttag tttgtagttt tagttattgg    2280
ggaggttgtg gtaggaggat tgtttgagtt taggaggtgg aggttgtagt gagttatgat    2340
tatgttattg tgttttagtt tgggtgatag agtgagattt tgttaataaa aaaaaaaaag    2400
aaagtggagt agggaattta tgagaataag gtaattattg ggggtgtaag agagggtttt    2460
gatttaaagg ggtataagaa taaaataaga ttaaaggaat gtaaggaggt agttgttttg    2520
tagggtatag atgagattaa gaattgaaat tgaattaaga attgaaaggg gtataaaatt    2580
attggtaggt attggatgaa tgtatgagag tggggtgagt attgttgaag aggatgttag    2640
gaaatattgt tgggatggaa tagggtgttt gtgaaaaggg gatgtaggat ttgttgaagg    2700
ggtatgtgag gatatgagta ggttggaatt taggatgtgt gtgtgggtgg gaaagtgttt    2760
ggattgaaag aaaagaatgt ttgaattgaa agaatataga atgttgggtg tagtagttta    2820
tgtttttgat tttagtattg tgggaggtta aggtaggagg attgtttgag tttaggagtt    2880
tgagattaga ttagtttggg aaatataatg agattttgtt tttataaaaa taaaaataaa    2940
taattaaaaa aaaatttttt ttttgagata gagttttgtt ttgttattta ggttaaagtg    3000
tagtggtgtg attatggttt attgttagtt ttgttttttg ggtttatgtt attttttttgt   3060
tttagttttt ggagtagttg ggattatagg tgtttgttat tatgtttagt taattttttg    3120
tatttttagt agagataggg ttttattgtg ttagttagga tggttttgat tttttgattt    3180
tgtgatttat ttgttttggt tttttaaagt gttgggatta taggtttgag ttattgtatt    3240
tggtttaaaa atttttaaat ttaaaaaaag gggggatggg tgtagtggtt tatggttgta    3300
attttagtat tttgggaggt tgagataggt agattatttg aggttaggag tttgagatta    3360
gtttggttaa tatggtgaaa tattattttt attaaatata taaaaataaa aaaaaatagt    3420
tgggtatggt ggtttttgtt tgtaatttta gttatttagg aggttgaggt aggagaattg    3480
tttgaatttg ggaggtggag gttgtagtaa gttaagattg tgttattgta ttttagtttg    3540
ggagatagag tgagatttta ttttaaaaaa aaaaaaaaaa aaaaagaaag aatatagaag    3600
aggaagagga gaggggtttt tgaagaagga agatatttga gaaatgttat ggggtaaaga    3660
ggggagaaag ttgggaaaga agttagaatt agggtggggt ttaaagaggg gtaggatgga    3720
gatttgtagg tgtggttttt agggtaggag ggttgttagg ggtaggttta tttggttatg    3780
tttattttt attttgttt ttagatttat ggaatttagt gatatgtttg ttggttatta       3840
ttgtttttta gattttgttt tttgggatga gattagagat ttttagagta tagagttgat    3900
ttttaggaga gttattagtt gttttttaat ttgtgtttgt tgttgtaatt atggtgttat    3960
tgtttatttt aagggttata agtgtttttg tttttttttag gtttgtgagt gttataaatg   4020
tgtttttatt ttgtgagtat gaggtttggg aggggaggag gatgagtttt ttttaaaggg    4080
tggttgattt ttgatttgta attttttatt tttagggagt gttgtagggt tatggttgtt    4140
```

```
taggtggttt tgtgtaggta gtaggaggtg tagttaaaga agtatttgat gaggagaggg    4200
gaagtttttt ttaaagtttt taattatttt agaaagggaa ttatttagtt ataggttttt    4260
tgtgagtgtt tttgattagt gatggggtag gagtttgggt ataggaggta ggtatgggaa    4320
aaagaggttt agttttgttg ttgaattggt gtatgatttt gaattaaggg tttttatttt    4380
tttggtttta gttgttttag tgttgagaga atgtagtgag ttttgtgggt taaaaatggg    4440
ttagaggttg ggtatagtgg tttatgtttg taatttgagt attttgggag gttgaggtag    4500
gtagattatg aggttaagag attgagatta ttttggttaa tatggtgaaa ttttgttttt    4560
attaaaaata taaaaattag ttgggtttgg tggtgtatgt ttgtagtttt agttatttgg    4620
gaagttgagg taggagaatt atttgaattt aggaggtgga ggttgtagtg agttaagatt    4680
atgttattgt attttagttt gggtgatata gtaagatttt attttaaaaa aaaaaaatga    4740
gttagatggg ggagatgaga attgtttggg gttggggtag gatagggttt aggagaaagg    4800
gtttattaga gttttttttg gtttttttata gttggaaagg agaatatagt attttagttt    4860
tagatttttt atggggtagt tttgttggta ttgatatttt ttgggaag                 4908
```

<210> 228
<211> 4908
<212> DNA
<213> Artificial Sequence

<220>
<223> chemically treated genomic DNA (Homo sapiens)

<400> 228

```
ttttttgggg ggtgttggtg ttagtaggat tgttttatgg ggggtttgag gttggggtgt      60
tatgtttttt tttttagttg tgaaggatta gggagagttt taatgagttt ttttttttga     120
tttttatttt attttaattt tagatagttt ttattttttt tatttagttt attttttttt     180
tttgagatgg agttttgttg tgttgtttag gttggagtgt agtggtgtga ttttggttta     240
ttataatttt tgttttttgg gtttaagtga tttttttgtt ttagtttttt gagtagttgg     300
gattataggt atgtgttatt aagtttagtt aatttttgta tttttagtag agatggggtt     360
ttattatgtt ggttaggatg gttttgattt tttgattttg tggtttgttt gttttagttt     420
tttaaagtgt ttggattata ggtgtgagtt attgtgtttg gtttttagtt tatttttgat     480
ttatagaatt tgttgtattt ttttaatgtt gggatagttg aggttgaggg agtagaaatt     540
tttagtttaa ggttgtatat taatttagta gtaggattgg gttttttttt tttatatttg     600
tttttttgtat ttaaattttt gttttattgt tggttagaga tatttatagg ggatttgtgg     660
ttgagtggtt tttttttttga agtggttggg agttttggga gaggtttttt ttttttttat     720
taggtgtttt tttagttgtg tttttttgttg tttatgtaag gttatttggg tagttatgat     780
tttgtggtgt tttttaaaag tggggagttg taagttaaag gttagttatt ttttggagga     840
ggtttatttt tttttttttt tagattttat atttatagga tgaggatata tttgtgatat     900
ttgtaagttt ggaagaggta gaggtgtttg tggtttttga gatgggtggt gatattatgg     960
ttgtggtagt gggtgtaggt tggagagtgg ttgatggttt ttttggggat tagttttgtg    1020
ttttgggggt ttttggtttt attttagggg gtagagttta aggggtagtg gtagttagta    1080
ggtatgttat tgggttttat ggatttaggg gtaggagtgg aaggtaagta tagttaggtg    1140
ggtttgtttt taataatttt tttgttttgg agattatatt tgtaggtttt tattttgttt    1200
tttttttagat tttgttttga ttttggtttt tttttttagtt tttttttttt tttattttat    1260
gatatttttt aggtattttt ttttttttaga aattttttttt tttttttttt tttgtgtttt    1320
tttttttttt tttttttttt ttttgagatg gagttttatt ttgtttttta ggttggagtg    1380
tagtggtatg attttggttt gttgtaattt ttattttttg ggtttaagtg atttttttgt    1440
tttagttttt tgagtagttg ggattatagg taagagttat tatgtttggt tatttttttt    1500
tatttttgta tgtttagtag agatggtgtt ttattatgtt ggttaggttg gttttgaatt    1560
tttggtttta agtgatttgt ttgttttggt tttttaaagt gttggaatta tagttatgag    1620
ttattgtatt tgtttttttt tttttttaaat ttaaaaattt ttaggttggg tgtggtggtt    1680
taagtttata attttagtat tttgggaggt taaggtgggt ggattataag gttaggagat    1740
tgagattatt ttggttaata tagtgaaatt ttgtttttat taaaaatata aaaaattagt    1800
tgggtgtggt ggtgggtgtt tgtagtttta gttattttag aggttgaggt aggagaatgg    1860
tgtgaatttg ggaggtagag ttggtagtga gttatgattg tgttattgta ttttagtttg    1920
ggtgatggag taagattttg ttttaaaaaa aaaatttttt tttaattgtt tatttttatt    1980
tttatagaga taaggtttta ttatgttttt taggttggtt tggttttgaa tttttgggtt    2040
taagtgattt ttttgttttg gttttttata gtgttaggat tagaggtatg agttgttgtg    2100
tttgatattt tgtatttttt taatttaaat attttttttt tttaatttaa atattttttt    2160
atttatatat atattttagg ttttaattta tttatgtttt tatatatttt tttagtgagt    2220
```

tttatatttt ttttttataa gtattttgtt ttgttttaat agtgtttttt ggtatttttt 2280
ttaataatgt ttattttatt tttatgtatt tatttaatgt ttattagtag ttttatgttt 2340
tttttaattt ttagtttaat tttaattttt agttttattt atgttttgta aagtaattgt 2400
ttttttgtat ttttttagtt ttattttgtt tttgtatttt tttaaattag aatttttttt 2460
tgtattttta ataattattt tatttttatg gatttttttgt tttatttttt tttttttttt 2520
tattgatagg gttttgtttt gttgtttagg ttggagtgta gtggtgtgat tatggtttat 2580
tgtagttttt attttttgag tttaagtaat ttttttgttg tagttttttt agtagttggg 2640
attataggtt ggagttatta tgtttggttt ttttgtttta tttttttttt tttttttttt 2700
ttgttttgag atggagtttt attttgtagt ttaggttgta gtgtagtagt gtaattttgg 2760
tttattgtaa tttttgtttt ttgggtttaa gtaatttttt tgttttagtt ttttgagtat 2820
ttgggattat aggtttgtgt tattatgttt gtttaatttt tgtattttta gtagagatgg 2880
ggttttatta tgttggttag gttggttttg aatttttgat ttgttttaag tgatttgttt 2940
gttttggttt tttaaagtgt tgggattata ggtatgagtt attgtatttg ttatttgttt 3000
tatttttaag ttattttttt ttattttttt atttatttta taaatggtta ttgagttttt 3060
gttatgtgtt aggttttgat ttgggttaaa aatttttttt ttggggtatt aatgtttttt 3120
ttgtgtttat attttattta ggtttttgtg taaatatgtg aagttattgt gtttagtgtt 3180
ttttgttttt gtggttatta ttaataatta attttatgta tgtttttttt tttttttttt 3240
gtgttttagg gagtgagtgg ggaattggga atgtgttaag gggtttgtga ggtagggaag 3300
gggagagggg gtgtgagggt gggaggggtg tgagggggag gagaggggat gtttgggtta 3360
gggagtttgg ttaagaggga ggaggatgga aaggtaggag ggattgtgtt tgtgtagtta 3420
gtttatggtt attatagttt ttaatggagt tagatttagt taagaagatg tttaaagata 3480
ttgtgagaaa gtggttagag gaattgttaa gtttgtgttg ttttttttat tttgttttat 3540
aaagattaat gaattttaag tattgttttt tttagttgag gtagatgatt aatgtggttt 3600
gagattttga attgatgttt tgtttttttt tttttgttag ttttttggtg tataagatta 3660
ttagttttat taatagtgtt ttgagttgag ttttgtgtta ttgttgtttt tttttttgta 3720
tgttttgttt gtttagattg tttttttttt tttgaggtag gtggttaatg gggtgttgag 3780
tatttttttat tggattgagg ttggttttgt tttttaggaa tggtttgttt ggttttgttt 3840
ttggggtgtt gattaagttt ggtttgtgta tgggttgttt tgtttttggt tttttttttgt 3900
tttgtatttt gattttgtgt gtggtttgtt tttgttgagt ggaggtttgt gttttttgtt 3960
atgttttttt tggtgtttta taatttttga gttatttttt ttttatgtgt ttttttttag 4020
tgttgtgatt attgggttgt ttattgggtt tttttttttt agattgtatt ttttttttttg 4080
tgtttttttt tgtttttgtg ttggttgtga ttttttttaat ttttgatttt tttttttttt 4140
tggatgtttt ttttttttgg atttattttt ttttgtgttt tatgttttga tagtttggtt 4200
ttttttttggt ttttttttgtt attggggatg tttttagttt ttttgaattt ttggttggtt 4260
ttatttttttg tgtttatttt ttttaagagt ttgttttttt gggggttttt ttgtgtaatt 4320
gttgtttttt ttgggtattt ttgtgaattt tgttttatat tattttgtta ttttttttgt 4380
tttggttttt tttttttgta tagttagttt tgtgtttttt tttttttttt ttttaaaatt 4440
gatttttttt tttttttgaga gttttatttt tgtgttttat tttttatttt tttatgtttt 4500
tttttttttg ttggtttttt tttttttttgt aaggttttat tttattaatt tgtttgtttt 4560
ttgtaggggt ggtatttttt ttgattattg ttttattttt tttttttttt tttttttttt 4620
tttgtttgag gattttattt taattttttt tggttgtgtt tttatttgta tttagtttgt 4680
taggtttagg tttagttgtt ttgtatttga ggttggtgtg tgttgttttt tttgattggt 4740
ttaatttttt ttattttttgt gagatttgtt gttagttttt gttttttttt tttgtgtttt 4800
agttttttttg tgggttttgg tatttttttt ggttatagat ttttgggtta tagagtatgt 4860
gtgtttgagg tattgtaggt agaaaagggt ggttgatgtg atttttag 4908

<210> 229
<211> 7563
<212> DNA
<213> Artificial Sequence

<220>
<223> chemically treated genomic DNA (Homo sapiens)

<400> 229

gttatggtgt ttggttaata ttaaatttta agttttaaaa ataatttttt ttggttttat 60
gttttatttt tagatatatt gatgtggtga atttgttttt atagttttgg gtggttttgt 120
ttttagggta gtttgatatt tggtttgtat tttggttttt tggggttaga gttatatatt 180
ggttgtttta ttgttttggg ttaagggagt tgatttattt tagttttgtt gagtgttggg 240
ttttataggg atttttttgtg gtgttttata tttttggagg tttttgtatt ttgtgttttt 300

```
ataagggtat taattttttt tatgaaggtt ttgtttttag aatttagtta ttttttaaag     360
gtttatattt aaatattatt aattgaagat tagtttttaa tatgtaattt tttggggaga     420
taaaaatatt tagattataa taattttaat attatttata attatattta atatattatg     480
aatttaatat tttaaagata ttgttagatt ggattaaaat aaatgtttta atgtttatat     540
gggatttata ttaaatataa agatatagaa agatttaaag taaaaagatt ttttaaaaaa     600
agttgtttta tgataatatt aatggaaaaa tagttaatgt agttgtaatg atattaaata     660
aaagtggtta tatatagtaa gtaggaaatg gttgggtgtg gtggtttatg ttggtaattt     720
tagtattttg ggaggttgag gagggtggat tatgaggtta ggagattgag attattttgg     780
ttaatatggt gaaattttat ttttaataaa aaatagaaaa aattagttag gtgtggtggt     840
aggtgtttgt agttttagtt atttaggagg ttgaggtagg agaatggtgt gaatttggga     900
ggtggggttt gtagtgagtt aagattgttt tattgtattt tagtttgggt gatagagtga     960
gattttgttt taaaaaaaaa aaaaataaaa aaagagaaaa aagaaataga aatttagtta    1020
aagatgttat aatgataaaa ggtaatatat atagataata ttataattat atgtgatata    1080
ttttatatta taatgttaaa tgtatgatta tttgataaaa ttaaataata tgatgataaa    1140
attattataa ttaggttatt aagatgaaag ggtaaatttt ataggaaaat atagtgattt    1200
taaatttgtg tatttttaat aaaatagtat taaattatgt agtgaaaatt ggttagttat    1260
ggtggtttgt gtttgtaatt ttagtatttt gggaggttaa ggtaggtgga ttgtttgagg    1320
ttagaagttt gagattagtt tggttaatat ggtgaaattt tatttttata aaaaatataa    1380
aaaaaaagaa aattagttgg gtgtggtggt gtgtttttgt agttttagtt atttgggagg    1440
ttgaggtagg agaattattt gaatttggga ggtataggtt tttattatta tattttagtt    1500
tgggtaatag agtgagtgag attttatttt aaaaaaaaaa aaaagtaaaa ttatgtagta    1560
taagttgata gtatataatg aaagtgtaag tagagaaatt tatatttatt aattgaaatt    1620
tttttttttt gttttttttag taattaatga gatagttaga taaaaatatt agtagaagtt    1680
aggtatggtg gtttatatat gtaattttag tattttagga ggttgaggtt ggtggtttgt    1740
ttgagtttag gaatttaaga ttagtttggg taatatggtg agattttgtt tttatgaaaa    1800
atataaaaaa ttagtttggt gtggtgatgt gtatttgtaa ttttagttat taggggtgtt    1860
gagaggggat gattatttgt gtttaggagg ttgaggttgt agtgagttgt aattgtatta    1920
ttgtatttta gtttgggtga taaagtaaga tttagtttta aaaaaaaaga aaaaaaatta    1980
atagagaaat ttgaataata tagtaagttt gattagttga tatatgtaga gttttgtatt    2040
taataagtgt aaaataattt tttaagtata tatgaaataa ttagaaaata attatatatt    2100
ggggagtaag ttttaaaaaa tatttatgaa ttgaagttat attgagtatg tttttagttt    2160
atagtgtaat ttaattggaa gttaataata aaaaggttag aaaattttta aatggttgaa    2220
gatgattaat tatatatata tatgtgtata tatatatata aatatatatg gatatatgtg    2280
tatatatatg tgtatatata tgtatatata tatgtgtata tgtgtatata aatatttata    2340
tgtgtatata tatgtgtata aattatatat atgtgtatat atatatatat atatatatgt    2400
gtgtatatat gtgtgtatat atatatgtgt gtatgtgtat atatatgtgt gtatatgtgt    2460
gtgtatatat atataagtgt gtatatatgt gtgtatatat attgtgtatg tgtgtgtata    2520
tatatgtgta tatgtgtttg tatatatatg tgtgtatatg tgtgtgtata tatgtgtgtg    2580
tatatatgtg tgtgtatata tatgtgtgtg tatatatgtg tatgtatata tgtatgtgta    2640
tatagttgat tatttttaat tgtttggaga tttttattta tatatatttt tttttttaata    2700
ttatatggtg ttttattata tagtgttttt tatggatata gtttaagaat tatttgttat    2760
tttgttttat agaaagtgtg aaattgtagg ggagttgatt tgtatagatt ttggtaaaaa    2820
gataattgag taggtaaatt ttagggagtt tttggatagt ggattttttt gttagtggtt    2880
ttggagtttt tagttttagt tatttgtggt taattgtggt ttaaaaatat taaatagaaa    2940
attttaggaa taaatagttt ttaggttttt aattgtgttg atttttgatt ggtgtgatga    3000
gatttagtta ttttattttt ttttatagaa agtgagtttt ttttttgttt agtgtttttt    3060
tgttgtttat aatatttatt tgttagttat tttataattt gggttattag atgattgtgg    3120
taatgattag tgtggtgttt ttaagttgtt ttgattttat tgattttttg tttttatttt    3180
tttttggttt taaagtgtaa gagaagtgat gttggtaatt tagatatgtt aaagagaagt    3240
tgtaaatgtt ttttttaagt taaaaggtaa aaattttgga tttaataagg aaagaaaaaa    3300
aattatatgt agaggttgtt aagatttatt aaaaataaat ttatttatga aaatgtgaag    3360
aagaaaaaag aaatgtatgt tagttttgtt gttgtattat ttttggatgg aagaatagaa    3420
atatgttttt attgattgta atgggtatta tttatggttt taggtattta ttggggtttt    3480
tggaatttat tttttatagg tatgggggggg ttattgtatt tttggtttta tagtttttta    3540
ttgttgattt taggtttttt tggattttag gtttttttttt ttaagatgta ttttagagga    3600
ttaaaaatat attttatttg ggttttgttt gtttttgtgg aagggtagtt tattagagga    3660
tataattttg tgttttaatt tgtttttttt tttaaaggaa atgtggagaa aaaaaaaaag    3720
tagaaattgg aaataattaa tatttagttt attttatttg atttttaggg gaattggtga    3780
ggagtttaag atgatttttt ttttttagag aaagaattta aagtttaggg aaatagtgat    3840
aggggagttt aagattgttt ttgttagttt ttttttggtt attttttgtt gtgattgtag    3900
gatagttttt attagtagga gaattgggta agtgtgtgga taagtagaga gtgtgttgaa    3960
```

taatttgtaa tgttttatga aatatgtatt gttatggttt tttaaaaggt tttgtggaag 4020
ttgtttgttt ttattaatta agtttttatt tatataaaag tagaagtaga agtagtttta 4080
gaaaatatat taataatttt ttattttttt gaagattaga gtagtagaaa ataggtgatt 4140
tgtattataa aattgtattt attttttttt tttttagat tttatattat attagtttat 4200
ttgtgttatg gtgtataaaa aatggaatag gtgtttttat tatattgttt ttttttaaaa 4260
atagattatt tatattttaa ttttgttttt tttaaatttg atttttaata ggagagtttt 4320
ttattatttt agatggagtg aggttgtatg attgggatgg aagaaaggaa tttttttaaat 4380
ttgggggaat ttttgttttt tgttttaaga ttattttatt tggggtgtgg gggtgggtgt 4440
ggtggttagg gtagtggaat gtagttgtgg ttgtgttatt tttgtatttt taggtgtgtg 4500
ggagggattg gtggggatgt gagttgtgga ttttggtgaa tttgggggag gtagataggg 4560
ggaggtggat atttagttgg taggtgtttt agttttttttg tagttggtgg gttttttttt 4620
tgatagtttt aggaaaggta gattttttttt ttagttagtt aggtaaggta aagattgttg 4680
ttgagtttgt tgttattgag ggtgtataga ttttggggag attgaagttt gttattgtgg 4740
gattttgtgg ggtaatttgg gtttatggaa gtttttttgaa agagggggaga agggtttgta 4800
ttttttttat ggaggatttt ttttttttta gtattttgtt tgatgtattt aattggtaga 4860
agtgagattt taataggtag tagagagtgt ttatgtggag gaggtttggg gtgttgtggt 4920
gttattttta tttttttttg ggattgtgtt tatttttaaa gttatatgtt gatgaattaa 4980
tttatgtttt aaattttttt ttttagtttt gtgagtttgt ggtgatattg ggttgtgggg 5040
tggttgggaa tggttttttt ttttggaaaa attagagaat ggtttggaga gttgaaatga 5100
gtgtttgtga gtaggtttgt gtagaattgg gttttaggat tgttgagttt tgtagggtgt 5160
ttttggggga tgttaggttg ttggtttttt tgtttttgtt gagatggata atgttttgtt 5220
tttggagttt tggtttgtta atgtattggg tttggatttg gtgttgagtt gttttaatgt 5280
gttgattttg gtgttgttgt tggtgttgtt ggtggtggtt gtattagttg tttatgtggt 5340
gatttgtgtt gtgggtttgg tgggtaattt tgttgtgttg tatgtgttgt tgtgggtgtt 5400
ttgtatgaag attgttatta atttgtttat ttttaatttg gttattgttg atgagttttt 5460
tatgttggtg ttgtttatta atattgttga ttttttgttg tggtagtggt tttttgggga 5520
gtttatgtgt aagtttattg tggttattga ttagtataat atttttttta gtttttattt 5580
ttttattgtt atgagtgttg attgttattt ggtggtgttg gttattgtgg agttgtgttg 5640
ggtggttggt tgtatttata gtgttgtgtg tgtggtgagt ttggttgtgt gggggattgt 5700
tatatttgtt gtgttgtttt ttgtagtttt tgtttggtta gatgatgagt agggttggtg 5760
ttagtgtgtg ttagtttttt tgtagtttga ggttttttgg tggtgtgtga gttgttttta 5820
tatgtttgtg ttgggttttg ttatttttgt gtttattatt tgtgtttttt atattatttt 5880
gttgtgttgg ttgtatgtta tgtggttgga tagttatgtt aaggttttgg agtgtgttaa 5940
gaagtgggtg attttttttgg tggtggtaat tttggtggtg tgtttttttt gttggatgtt 6000
ttattatttg agtattgtgg tggtgtttat tattgatttt ttgtagatgt tgttggttat 6060
tgttattttt tattttatta ttagtttgag ttatgttaat agttgtttta attttttttt 6120
ttatgttttt ttggatgtta gtttttgtag gaatttttgt tagttgataa tttgttgtgt 6180
ggtagtttga tttttttagt gtttggtttt gtaattgttt gttatttttg gttagtgagg 6240
gaggagttgg tgttagagtg tgggattaga taggttgttt aggtttttttg gggaaattga 6300
tttgtgtttt atatttgatt tagtagattg gaagtgttgt gattgtgttt gtaggttgat 6360
tttgttaagt tttttaggtg atgtgtggtt atgttgggtg aggagaattg aggttgagat 6420
tgttatattg agggtttttt aaagttgagg tggaggaaga ggagggtaga ggaggagggt 6480
ggtattgttg ggaattgttt tttttttgtt ttgttttttg ttgttttatt tgagttttgg 6540
tagtttggaa tttggttgga gttggaattt tttgtgtttt tgaagggagg tttgaggatg 6600
tgttggtgt ttttaggtgt tggttttgtg tggtttttgt tgtagttggt gtatagtttt 6660
ggtgggtttt tttgtttttt gtgtgttttg ggaagatggt taggtgtgtg tgtttggtgt 6720
ttatttttgt gagatttgtt tggtttggtg ttgtgggtgg ggattgttgt tgaggttgtt 6780
agtgtttttt taattgggga aaatataata atttgggatg ttttattgga gtgtgtatat 6840
atttgtgtgt gtgtgtgtgt ggtgtgtgtg tgtttgtgga ggtatgaaat gtggggaaag 6900
aggttggtgt ttggtagtga ttgtttagaa tatgatatga atagtgattt ttttttttgt 6960
ttgtttttat tttttttttt tgtttttttt tttttttatt tgtgattttt tttgaaaatg 7020
ttagattttt ggagtaagga atttggagta gtttgggtgt gtggttttgg tgtttttttgg 7080
aggagttagt ggttgagtgt ttagtgttgg aggattgtgg ggttttgtgg tggagtattt 7140
tggttgagtg tgttttatag tgttgtggta tattggttat tgttgtttat agaggatgtt 7200
aagtttttatt tttatttttt taattggttt tgttttttgt tttatttaga ttagtttgtt 7260
gtaattgttt atgtgtgtat tgttttttag gattgaaaat tttataaata atgtatatgt 7320
tttaatttta tgagttgtta tgaagattaa ataagataaa aagaaatgtg atttattttt 7380
ttgtgttttt tttttttttt gttttttttgg ttttgtttta gggagagtag gaagtaggga 7440
tgattgatgg ttttaggttt tttttgttgt attttttata gggagttgtg gattatttgg 7500
gttttatttt ttatttgtag atggtatttg gtaggtagtt ttggtaaatt gggtaaatat 7560
tgg 7563

<210> 230
<211> 7563
<212> DNA
<213> Artificial Sequence

<220>
<223> chemically treated genomic DNA (Homo sapiens)

<400> 230

```
ttagtattta tttggtttat tgggattatt tattggatgt tgtttataag taaggaataa      60
agtttaggta atttatagtt ttttgtgaaa agtatagtag agaaaatttg aagttgttgg     120
ttatttttgt tttttgtttt ttttaaggtg gagttaagga agtagaggag aaagggaata     180
taagagaata gattgtattt ttttttattt tatttgattt ttatggtaat ttgtgagatt     240
aggatatgta tattatttat aaagttttta gttttgaaaa atagtatata tatgaatagt     300
tataataaat tgatttaagt aagataagag atgaggttaa ttgggggaat gggggtgggg     360
tttggtattt tttataagta atgatgatta gtgtgttata gtgttgtgaa gtgtgtttag     420
ttggagtgtt ttattataaa gttttgtgat tttttgatgt taagtatttg gttgttggtt     480
tttttagggg gtgttggaat tgtgtgtttg ggttattttg ggttttttgt tttgggagtt     540
tgatattttt aggggagatt atggatagga aagggaaaag gtaaagaaaa ggagtagggg     600
tagatagaaa gaaggattat tatttgtatt atattttagg tagttattgt taagtgttag     660
tttttttttt tgtattttat gtttttgtaa atgtgtgtat attatatata tatatatata     720
ggtgtatata tattttgatg gggtattttg agttgttgtg ttttttttag ttgaagaggt     780
gttggtggtt ttggtagtgg tttttgtttg tggtgttagg ttaagtgggt tttatagggg     840
tgggtgttaa atgtgtgtgt ttgattgttt ttttagagtg tgtggaggat aaggggattt     900
gttggggttg tgtgttagtt atagtagggg ttgtgtggag ttgatgtttg gggtgttgg      960
tagtgttttt gggttttttt ttgaaggtgt ggagagtttt gattttagtt aggttttaga    1020
ttgttagggt ttgggtgggg tagtagggag tagggtaggg agggggtggt ttttagtaat    1080
attgtttttt ttttttattt tttttttttt ttattttggt tttagggagt ttttagtgtg    1140
gtgattttag ttttagtttt ttttatttgg tatggttgtg tattatttgg agggtttggt    1200
aaggttaatt tgtgggtata gttgtagtgt ttttgatttg ttaggttggg tatggggtgt    1260
gagttggttt ttttaggagg tttaggtggt ttgtttggtt ttgtattttg gtgttggttt    1320
tttttttgtt ggttaggagt ggtgggtagt tgtggagttg gatgttgggg gagttaggtt    1380
gttgtgtggt aagttattag ttggtggagg tttttgtgga agttggtgtt taggaaggtg    1440
tagaggaagg ggttgaggta gttgttggtg tagtttaggt tggtgatgaa gtaggagata    1500
gtgatgatta gtggtgtttg tgggaggttg gtggtgagtg ttattatggt gtttaggtgg    1560
tagggtgttt agtagaggag gtatattgtt aggattgtta ttattaggaa ggttatttgt    1620
tttttggtgt gttttagggt tttggtgtgg ttgtttagtt gtatggtatg tagttggtat    1680
agtagggtgg tatagaggat atagatggtg gatatgggga tggtgaagtt tagtatgagt    1740
gtgtagaggt ggtttgtgtg ttattagaag gttttgggtt gtggaaagat tagtatgtat    1800
tggtgttggt tttgtttgtt gtttagttgg gtgaagattg tgaagggtag tatgatgagt    1860
gtgatgattt tttatatggt taggtttatt gtgtgtgtgg tgttgtaggt gtggttggtt    1920
atttggtgtg attttgtagt ggttaatatt attaggtagt ggttggtgtt tatgatggtg    1980
aggaagtaga ggttggagaa ggtgttgtat tggttgatag ttatgatgag tttgtatatg    2040
agttttttga agggttattg ttgtagtagg aagttggtga tgttgatggg tagtattagt    2100
gtgaagagtt tgttggtgat ggttaggttg aggatgaata ggttggtgat ggtttttatg    2160
tggggtgttt gtagtaatat gtatagtatg gtggagttgt ttgttagatt tatggtgtag    2220
attattgtgt agataattgg tatagttatt gttagtggtg ttggtagtgg tgttagagtt    2280
gatgtgttgg agtagtttag tgttgggttt gggtttgatg tgttggtggg ttagggtttt    2340
gagaatgagg tgttgtttat tttaatgagg gtagaggagt tggtgatttg gtgtttttta    2400
aggatgtttt atggagttta gtggttttga agtttggttt tgtatggatt tgtttgtgga    2460
tgtttgtttt agttttttaa gttgtttttt ggttttttg gaggagggga ttgtttttag     2520
ttattttatg gtttaatgtt gttgtggatt tatggggttg gaaagaggaa tttaaagtat    2580
aggttagttt gttgatgtgt aattttagaa ataggtgtgg ttttgaggag gggtgggggt    2640
ggtgttgtgg tgttttaaat tttttttatg tgagtgtttt ttgttatttg ttgaaatttt    2700
atttttatta gttgaatata ttaaatgaaa tgttagagag agaagaattt tttataggaa    2760
aaatgtaaat tttttttttt ttttttagga aatttttgta gatttaggtt attttataga    2820
attttgtagt ggtaagtttt ggtttttttta gggtttgtgt gtttttagta atagtaagtt   2880
taatagtagt ttttatttta tttggttggt tggggaaggg gtttgttttt tttggagttg    2940
ttaggagaaa agtttgttgg ttgtggggag gttgagatgt ttgttggttg ggtgtttgtt    3000
```

```
ttttttgtt tgttttttt gagtttgtta gagtttgtag tttgtgtttt tgttggtttt    3060
ttttgtgtgt ttggaagtgt agggatggtg tagttgtgat tgtgttttat tgttttgatt    3120
gttgtgttta tttttatatt ttaagtaaaa tggttttaga atagagaata gaaatttttt    3180
taaatttaag ggattttttt tttttatttt agttgtgtaa ttttatttta tttgaaataa    3240
tagaaagttt ttttgttaag aattgaattt aaggaaaata aagttagggt gtaagtgatt    3300
tatttttaaa ggagaataat gtagtggagg tgtttgtttt attttttata tattgtaata    3360
taaataggtt aatgtaatgt gaaatttgaa aggaggaaaa agtgagtgta attttataat    3420
gtaaattatt tgtttttgt tattttggtt tttaagggga tagaagattg ttagtatgtt    3480
ttttaaaatt atttttattt ttatttttgt gtaagtaaag atttgattag taaagataaa    3540
tggtttttgt aaagtttttt agggaattat gataatgtgt attttataaa atgttataag    3600
ttgtttaata tattttttat ttatttatat atttgtttga tttttttgtt aatgagagtt    3660
atttgtgat tgtagtggag agtagttaag gaaggattag taggggtagt tttgaatttt    3720
tttgttgtta ttttttttgga ttttggattt tttttttagg aagggaaaat tattttaggt    3780
tttttattag ttttttaag aattgaatga aataaattaa atattggtta tttttaattt    3840
ttgtttttt ttttttttta tattttttt aggagagaga gtaaattaaa atatgagatt    3900
atatttttta gtaaattatt tttttataaa agtaggtgaa gtttaaataa agtgtatttt    3960
tggtttttta ggatgtattt tagagaaggg ggtttgagat ttagagagat ttaaagttgg    4020
tagtagaaga ttgtggggtt agaaatatag tagttttttt atatttatgg gggataggtt    4080
ttaagagttt tagtggatgt ttgaaattat ggatagtatt tattgtggtt aatgggaata    4140
tgtttttgtt tttttattta aaaatgatgt aatagtaaaa ttagtatgta tttttttttt    4200
tttttttata tttttgtgga tagatttgtt tttagtagat tttggtaatt tttgtatatg    4260
attttttttt tttttttatt aagtttagaa tttttatttt ttaatttaaa ggaggtattt    4320
gtagtttttt tttggtatat ttgaattgtt agtattattt tttttgtatt ttggagttag    4380
agagaaataa gggtaaaaag ttagtaaaat taaggtaatt tgaagatatt atattggtta    4440
ttgttatagt tatttaataa tttagattgt gaagtgatta ataggtggat attgtagata    4500
gtagggaaat gttgggtaaa gggaggattt attttttgtg ggaaggagta agatggttgg    4560
attttattat gttaattaga agttaatata attgaaaatt taggaattgt ttatttttgg    4620
aatttttgt ttaatatttt tagattatag ttgattatga gtaattgaaa ttagggattt    4680
taaaattatt gatagggggga tttattgttt aaaagttttt tggaatttat ttatttaatt    4740
atttttttat taggatttat ataaattggt ttttttgtag ttttatattt tttgtgaaat    4800
aaaataatag atgattttta agttatgttt gtaagaaata ttatatgata aaatattata    4860
taatgttaaa gaaaaaatat atataataga aaatttttaa atggttgaag atgattaatt    4920
atatatatat atgtgtatat atatatatat atatatatat gtgtatatat atatatatat    4980
atatatatgt atatatatat gtatatatat atatgtgtat atatagatat atatgtatgt    5040
gtatatatat atatatatat agtgtatata tatatatata tgtatatttg tgtgtatata    5100
tatatatata tgtatatgtg tatatatata tatatatgtg tgtatatata tatatatata    5160
tatatatgtg tgtatgtgtg tatatatgta tatgtatata gtttatatat gtgtgtatat    5220
atgtgtgaat gtttgtatat atatatgtat atgtgtatat atgtgtatat atatgtatgt    5280
atatatgtgt gtttatatat gtttatgtgt atatatatat atgtatgtat atagttgatt    5340
atttttaatt gtttggagat tttttagttt ttttattgtt gatttttagt tgaattatat    5400
tgtaaattga gaatatattt aatatgattt taatttatag atattttttg aaatttattt    5460
tttagtatat agttattttt taattatttt atgtgtattt gaaaaattgt tttgtatttg    5520
ttgggtgtaa aattttatat atgttaatta gttaaattta ttatgttgtt taaatttttt    5580
tattgatttt tttttttttt ttttgagatt gggttttgtt ttgttattta ggttgaagta    5640
tagtggtgta attgtagttt attgtagttt taattttttg ggtataagta attatttttt    5700
tttagtattt ttagtagttg ggattatagg tgtgtattat tatattaggt taattttttg    5760
tatttttgt agaggtagga ttttattatg ttgtttaggt tggttttgag tttttgagtt    5820
taagtgaatt attaatttta gtttttaaa gtgttgggat tatatgtgtg agttattgtg    5880
tttggttttt gttgatattt ttatttaatt gttttattag ttattgaaag gatggggggg    5940
gggaatttta attaatgagt gtgaattttt ttatttatgt ttttattata tgttgttaat    6000
ttgtattatg taattttatt tttttttttt tttgagatag agttttattt attttgttgt    6060
ttaggttgaa gtgtggtggt gggagtttgt gttttttagg tttaagtgat tttttgttt    6120
tagtttttttg agtagttggg attatagggg tatattatta tgtttagtta attttttttt    6180
tttttgtatt ttttgtggag atggagtttt attatgttgg ttagattggt tttgaatttt    6240
tgattttaag tgatttgttt gttttggttt tttaaagtgt tgggattata ggtataagtt    6300
attatgattg gttaattttt attatataat ttgatgttgt tttattaagg gtatatgagt    6360
ttaagattgt tatatttttt tgtggaattt attttttat tttgatagtt taattataat    6420
gattttatta ttatgttatt tgattttatt aaatgattat atatttaata ttataatata    6480
gaatatatta tatataatta taatattatt tgtatatatt attttttatt attataatat    6540
ttttaattag gtttttattt tttttttttt ttttttgtt tttttttttt ttgagataga    6600
gttttgtttt gttgtttaag ttggagtgta atggaatgat tttggtttat tgtaagtttt    6660
```

atttttttggg tttatgttat ttttttattt tagttttttg agtagttggg attataggtg 6720
tttgttatta tgtttggtta attttttttta tttttttatta gagatggggt tttattatgt 6780
tagttaggat ggttttgatt ttttgatttt gtgatttgtt ttttttggtt ttttaaagtg 6840
ttgggattat tggtatgagt tattgtgttt agttattttt tatttgttgt atgtggttat 6900
ttttgtttga tattattata gttatattaa ttgttttttt attaatatta ttatggaata 6960
gtttttttta aaaaattttt ttattttaaa tttttttatg tttttgtatt taatgtgggt 7020
tttatgtaag tattagaata tttgttttaa tttaatttga taatgttttt gaagtattga 7080
gtttatagta tattaaatgt agttatagat agtattaggg ttgttatggt ttgaatgttt 7140
ttgttttttt aaaaaattat atattgaaag ttaattttta attgatagta tttggatatg 7200
ggtttttggg aggtgattag gttttgagaa tagagttttt atgaagggaa ttagtgtttt 7260
tataaaggtg tagagtgtaa aagtttttga gggtgtgagg tattatagag agtttttgtg 7320
aagtttaatg tttagtagag ttggggtagg ttagtttttt tgatttagag tagtaaagta 7380
gttagtgtgt gattttagtt ttaggaagtt aggatgtaag ttaggtatta agttgttttg 7440
ggagtagggt tatttaaagt tgtggagata ggtttattat attagtatgt ttggaagtga 7500
gatatggaat taaagaggat tatttttaag atttaagatt taatgttggt tgagtgttgt 7560
ggt 7563

<210> 231
<211> 5849
<212> DNA
<213> Artificial Sequence

<220>
<223> chemically treated genomic DNA (Homo sapiens)

<400> 231

attgtaatgg tatgattttg gtttattata atttttgttt tttgggttga agtgattttt 60
ttgttttagt tttttaagta gttgggatta taggtatgta ttattatatt ttgttaattt 120
tgtattttta gtagagatgg ggttttttta tgttggttag gttggttttg gatttttgat 180
ttttggtgat ttgtttgttt tgattttttta tagggttggg tttataggtg tgaggtatta 240
tgtttggtta taatttttaa atattttttt gttggtattt gtttggatta tggatttaaa 300
tgatttatta atatgatgag aatttaatgg ggatatttta aaagagataa atgaatatat 360
aggtggtttg aaatgatttt ataaagtttt ttaataatta tatttttttt tagagtgtta 420
aagaatttgg tttatttaag gtgtttattt tttaaaaaat ggttatataa atgtaaattt 480
tatttaaatt aaattttatt ttgagtttgt taaatgttat gtttagtatt atttaaggtt 540
tatattaatt gttgttttaa aaaattatat agtgttttaa gtatttttat attttaaata 600
taaaggaata attttaattg tggaattaga gttgtttttt taagataaaa agataaagat 660
aaatattata attaattatt aattggagaa atattttttt aaatgttttt gtaataattg 720
gatgtgttgg atagtataat taaatttatt aagaatagtg tgaaaaatat tattattatt 780
tagtttttat atatagatat ttttttaaat tattgaaatt tgtatttgaa tatatgaagt 840
attaagaatt tgtttttgaa aaggaaatga atatttgaag gttatttttt ttttaatttt 900
tttttatatt aataattatt gttaatattt atttttaaaa aattgttttt attaggtatt 960
tgggaattgt ttaataaaag ttatttaatg aaattatagt ttagataaaa aaaaaaattt 1020
aattgtatag aaaatattta tgagtttatt tttttaattt atataattgt gtttttgttt 1080
ttaaagtttt gtaatttata ttagtgggaa taaaaaaatt taatatatag tagaaagttg 1140
gtttttttaaa agaaagtatt tttttttaaa ggtaatgaag gattaaaaat tttttttttt 1200
aaaaagattt tagaagtttt tttttagag aagatagaaa atattgataa attgtatatt 1260
gtgtagatgt ttagttttga aatgtagttt tgtagttttt tattttgtag tttattggtt 1320
gtatttgtat ttttgggtag tgtttttgta gggtgtaaat atttagagat ttagggattt 1380
ggtagttttg tgtaggtttt ttgtgtgtat tgtttagtga gtgggttttt tgtagatggt 1440
gggatgtttt aggagattgt gaggttggtg tagtttggtg agtttttgga gggaaggtgg 1500
tgtgttagaa aggattggat tttagtaatg gttttggatt ggttttgtgt ggttgttttt 1560
tttttttgtt ttgttgttgg gattttttta gttagtgttg aattttttaa ttgggaagga 1620
ttgagttatt ttattttttg ggaagttgtg tgtttatttg tttttggtag tgttggagtt 1680
tgagttgggg attgggtgga tatttataga gtttgtattg tagtgtgttg tatgtttttg 1740
tttttgtttg ttggtttgtg ttttagtgaa ttaattgtaa agttggtttg aagtggtgat 1800
ggtgtttttg tggagtttat ttgttgtttt agtgtgtttt tgagtaggga ttgggtttgt 1860
tggggttttt gttgggtttg tgtgtttgta tggtatttgt tgtatgagtt gtttttgtgt 1920
tttttttttg tgtttggttg tttagtttgg tgttggtgtt ttatgttttt tgagtgtagg 1980
agattttttt gttttggttt tgtttgttgg tgttggtgtt tattttttgg gtgtggaaat 2040

ttgagtttat ttttttggt tgttggtttt gggattgtgg gtttggttat gtagttttgt 2100
ttggatttaa agagttttag tttttgtgaa tattaggttt gtgttgggag atgagaaggt 2160
gtttagtggg gtttagtgtg gttttgaggg gtagggtgag gtttgtgatg ttgaggttag 2220
ttatggttag ttataggtgt tgtgtaagga attggggaaa gttttttgtt aagaaaggga 2280
aatgttagtt tagttaaaaa aaaaaaaaaa aaaaaaaggt tggggtttaa tgagaaagtt 2340
ttttagaggt attttttag ttttttata gttttttggg agtttttgaa tttgtatttg 2400
aatggtgtgg ttttgggttt gtgaaggttt ggtgggttga agttgttttt taggttttta 2460
aaggaatgta gttaggttgg aatttatttt ttttttaat ttttagaaaa tggaaatgtt 2520
ttttttgtga ttttttttg ttgagttaga ttttaggttt tttttgttgt gggtgtttgg 2580
ttttttttgt tgatattttg gatgtttagg tttgtgggat aaaaggtagt tgtggtgttt 2640
tgtgggtttt ttagtttggg agttggtttt aggggtttta aagtgtgatt ttaggttgat 2700
aattgtaagt ttttaggtag aggggaagtt ttagtgttga gaagtttgtt ttgagaattt 2760
tttgtgttgg gtgtttagtt tgagtgtttt gggtgtggag tttatttttg gtgtgggtag 2820
ggttttagtt gtaggtttgg tgtagggtgg atttggttga agtttggagg gttggaggtg 2880
ttgggatgta gtttggataa gagggtggat ttgattttgg aaagtttgtg ggatttaggt 2940
attttaatat tgggaagata aagagatttg atttttttgg gattggtgag gggagtttgg 3000
tggggagggg aatggtaggg tagttaagtg tgaaagagaa ttttttgggt ttaaagttaa 3060
tgtagttatt ttataagtta ttttagaggt gattggaaaa aataataata ataaaggtgt 3120
ttagtaggtt gtggttggga aggaaaaata atgtttgtaa gatgagtgtg ttgatttgta 3180
gttttatatt ttttgtttat tagttggaat atatatagat ttatatattt agtaaatgta 3240
aattttatta tttaaagatg tgtttgtttt ggttataatt gttttaagtg tttataggtt 3300
aattttatat atggaatatt ttgaaagtgg gtgatttgat ttttaagttt aattggtttg 3360
tgttgtttgt tgaattgtaa attttagtaa aatttaaata tattggaatt ttagttgttt 3420
tttgggagtg aggaagtggg agtaatgtta aaaggggaaa agaagaatag taaaaaaagt 3480
ataaagatat tattttttga tgtaaaatta tgggattttt tttaaatggt ataagagaat 3540
ttttggagaa gattattatt aattaataag taataatgtt ttttagggtt tattagatgt 3600
tttatatttg tttgggaatt ttgatttttt aggtttttg agaaatttat ttgtttggaa 3660
gaagttgtat tttgttgtta taggagttgt tgagagttag ggatggagat tttagattta 3720
ggattttgtt atgattttat tgatttttaa gtgttaatag aatgaaatgg atatgtttat 3780
tagttattat aaattgaatt attttatatg agagagagaa atgtggttta agttttatat 3840
ttgtttttta aataattgat ttttttaat tttgttttta aaggtttgtg gaaattaggg 3900
agggggttgg ggagatggtt ttagaaataa aatgtttttt tttagtatgg tttttatatt 3960
tagtttattt aagttgattt ttgtaattta tataaagaat gggtttgggt gggtggttag 4020
tgaagattgg ggtgagtaag tgtttatgtt gttgagtgtt ggggaggttt aggtttgttt 4080
ttgtatagaa ttttgtaagg agtgtgtttt gttaggtgtt tggattagag ggtgttgtgt 4140
ttttgtaaat tttgtggttt tgatgtgttt tttttaggta ttattgttat aataagttat 4200
atttaaaata atgtataaat agtattgttt tttaagtttt ttttaatagt gttttatatg 4260
tattttatga atttttaaag ttatgtatat gaattttttta ttgttgattt gtataaagtt 4320
agagggatgt gtagtttttg gagttgagaa tttgtagggt gtatatgttt ttttgtattt 4380
gtttagagtt atatatttta tgtaatttta ttaatgttta aatttaaatg ttaaaaggta 4440
tgtatgattt gaataaagaa tttattagtt tagttgagtt tttggttttg ggttttaaag 4500
tttttggtag ttttgtgatt tttttgtggt gtagatatgt gttaagagtt gggtttgaag 4560
tattaatttt gtttttttgg gatttttagt atgtttttaa ttagagtgtt aatgtttgtg 4620
ttgttgggat ttaggtggtt gtatttttgg aggtttttga ttagggtaaa tttaagtatt 4680
aaatgtaggt gaaaagagag tgtgtggggt tggaatgagg gtttgggagt agttaagttt 4740
gttaggatgt gtttaggtag ttagttgttg agttgaggag ttgtggttgt tggttgtggt 4800
gatttgttgt tttttttttt attttttttt ttttggaggt ggtgggggag gggaggtgat 4860
gttagtgttt tgttttgttt tttgttttgg gtgtggatgg gattttttgg ttgggtgtgt 4920
tattttgata gtggtagtag tggttgtgga ggttgtgggg gttttggttt ttgtggtttt 4980
tttggttggt tgtagttgtg gtgtgggtga aatgtttgtt tttttttttt ggttgtgttt 5040
tttgggtgtt ggtgagtgtg ggtgtttttt gtatttagtg tttggagttg tttggagttt 5100
tgttgtgtgt attttttttgg tttgtggagt tttttggtg gtgtttttta tgttgtggat 5160
tgtaggtgat gttttttttt ttttgtagtt gaaatttgtt gtgatgtgtt gtttgttaga 5220
tttttgttgg gtggaagtat gaatagtttt tgtagattgg tttggatgat ttgtatttg 5280
tggtttttta atttttggat gtgaattttt ttttatgatt tattattatg tttttttgtgg 5340
tttttgtgtt gaaaattatg gagttgtatt tttattttta aattttttttt taatgttttt 5400
tttgttttga ttgtattttg aagtttataa ggttttgaga ggagggggtg aagggatagg 5460
ggaggagtta gtttttaatg tgttttataa gttattttta gtgatagggg ttaatatgta 5520
aattattgtt ttttttttga aataatattt atttagataa atgtaaatat tgttgaggtt 5580
taatttataa agaatgtgtt gtttttatgt aagtgtttag tttttaggaa aatatttgtt 5640
gtggaatata tttttaatgt tattaaagta ttaaaatatt tttttgaata gtgtgaaatt 5700

ttgagggagt tatttttgtg agttgatatt gtttttagaa gattgttatt aatatttaag 5760
atttttgtaa aagggggttg ggggtggttt tattatattt gtatttgttg gtataaatat 5820
gttgattagt ttagttggtt gttttttta 5849

<210> 232
<211> 5849
<212> DNA
<213> Artificial Sequence

<220>
<223> chemically treated genomic DNA (Homo sapiens)

<400> 232

taaaagaatg attagttaaa ttgattaata tatttatatt aatggatgta aatatgatag 60
aattgttttt agttttttttt tgtaaggatt ttgaatgtta atgataattt tttggggata 120
atgttaattt atagaaatgg ttttttgga attttatgtt gtttggggag gtattttggt 180
attttagtgg tattaaaaat atattttata gtagatattt ttttggaggt tggatattta 240
tataaagatg atgtattttt tataaattaa attttaatag tgtttgtatt tatttaaatg 300
gatattattt tgaggaggag atagtgattt gtgtattagt ttttattatt agggatgatt 360
tgtaagatat attaggaatt ggttttttttt ttatttttttt gttttttttttt tttagaattt 420
tataagtttt agagtatggt taagataaga ggagtattgg gaaagaattt gaaaataaaa 480
atatagtttt gtgattttta atataaagat tatgaagagt ataataataa gttgtgaaaa 540
aaaatttata tttagaagtt ggagggttat aaagtataag ttgtttaggt tagtttgtag 600
agattgttta tgtttttgtt tagtaaaaat ttggtaggtg atatattgtg atgaattttg 660
gttgtagaag aggaagagta ttatttgtag tttgtggtgt gggaaatgtt gttggagggg 720
ttttgtgagt tggggaagtg tgtgtagtgg ggttttaggt ggttttaggt gttgagtgtg 780
aggaatgttt gtgtttgttg gtgtttggga gatgtagttg aaggaggagg gtgggtgttt 840
tatttgtgtt atagttgtgg ttggttagag agattgtaga ggttgggatt tttgtagttt 900
ttatggttgt tgttgttatt gttggggtgg tgtgtttagt tggagaattt tgtttgtgtt 960
tggggtgggg ggtgaggtgg ggtgttgatg ttattttttt tttttattg tttttaaggg 1020
ggagggagtg gggaaagagg tagtgagttg ttgtagttgg tggttgtaat tttttaattt 1080
ggtggttggt tgtttgggta tgttttgata aatttggttg tttttgggtt tttgttttgg 1140
ttttgtgtat ttttttttttg tttgtgtttg gtgtttaagt ttgttttggt tggaagtttt 1200
tggagatgtg gttgtttggg ttttggtggt atggatgttg gtattttggt tagggatatg 1260
ttgaagattt tggggaggta aagttggtat tttaaatttg gtttttaata tatatttata 1320
ttgtgagggg attatggagt tgttgaggat tttagagttt agggttgagg atttggttag 1380
attggtgagt tttttgttta aattgtgtat gttttttggt atttaagttt aaatgttaat 1440
gaaattatgt ggggtgtgtg gttttaagtg agtgtgggga agtgtgtgtg ttttatgggt 1500
ttttggtttt aggagttgtg tattttttttg gttttgtgtg gattggtgat gaaagatttg 1560
tatgtatagt tttgggagtt tataaaatat atatagagta ttgttaaaaa gaatttgaga 1620
ggtgatgttg tttgtgtatt attttgaatg taatttatta tagtaatggt gtttagggag 1680
ggtgtattgg ggttgtgaga tttgtagggg tgtagtgttt tttagtttgg gtatttggtg 1740
aggtgtgttt tttgtagggt tttgtgtgga ggtgaatttg gattttttttg gtgtttggtg 1800
atgtgggtgt ttgtttattt tagtttttat tgattattta tttgggttta tttttttatat 1860
gagttataaa aattagtttg ggtggattgg atgtagaggt tgtgttggag gaaagtgttt 1920
tgtttttgga attgtttttt taattttttt tttaattttt atgagttttt ggggataggg 1980
ttagggaggg ttgattattt aaaggataaa tgtggaattt gaattatatt ttttttttttt 2040
atataaaata atttgatttg tggtagttaa taggtatgtt tattttgttt tgttggtgtt 2100
taaggattag tagaattgtg gtagagtttt aagtttgaaa tttttatttt tggtttttgg 2160
tggtttttgt ggtgatgaag tgtaattttt tttaggtaga tagattttttt agggagtttg 2220
gaaaattggg atttttagat agatgtaaaa tatttagtaa gttttaggaa gtgttattat 2280
ttattaatta atggtaattt tttttaggaa ttttttttgtg ttatttaaaa aaaattttgt 2340
gattttatgt tagggaatgg tatttttgta tttttttttat tgttttttttt ttttttttttt 2400
agtattattt ttgttttttt gtttttagga aatggttgga gttttggtgt gtttgggttt 2460
tgttgagatt tataatttgg taagtggtat aagttggttg ggtttggaga ttaggttatt 2520
tatttttgag gtgttttatg tatgaaattg atttgtaagt atttggagta attatggttg 2580
ggataaatat gtttttggat gatggagttt gtatttgttg gatgtgtgaa tttatgtgta 2640
ttttaattgg tgggtaggga atatagggtt atgaattggt atatttattt tgtaggtatt 2700
atttttttttt tttagttata atttgttaga tatttttatt attattattt tttttggtta 2760
tttttaagat ggtttataag gtgattgtgt taattttgaa tttaggaaat ttttttttat 2820

```
atttgattgt tttgttgttt tttttttgt taagtttttt ttattagttt tagggaaatt    2880
ggattttttt gtttttttag tgttaaaatg tttgagtttt ataaattttt tgagattaag    2940
tttgtttttt tgtttgggtt gtgttttagt gtttttggtt ttttgggttt tggttgagtt    3000
tgttttgtgt tgagtttgtg gttggagttt tgtttgtatt ggggatgggt tttgtgtttg    3060
ggatgtttag gttgggtgtt tggtgtgggg agtttttgga gtgggttttt tggtgttggg    3120
gttttttttt tgtttaggaa tttgtagttg ttggtttggg gttatgtttt ggggtttttg    3180
gggttgattt ttgggttagg aagtttgtgg gatgttgtga ttgtttttg ttttataggt    3240
ttgggtgttt ggggtgttag tgggggaggt taggtgtttg tggtggggag ggtttggggt    3300
ttaatttggt agaaaaaggt tgtgggggaa gtgtttttat tttttgggga ttgaaggagg    3360
aagtagattt tgatttagtt gtattttttt ggagatttga aggatgattt tagtttatta    3420
aatttttgtg gatttagagt tgtgttattt aaatgtagat ttaggggttt ttagaaggtt    3480
atggaaaagt taaagggatg tttttgggaa gtttttttat taggttttag tttttttttt    3540
tttttttttt ttttagttaa gttaatgttt ttttttttttg gtagggagtt ttttttaatt    3600
ttttgtatag tgtttgtggt tggttgtaat taattttggt attataagtt ttgttttgtt    3660
ttttggagtt gtgttagatt ttgttgggtg tttttttgtt ttttagtatg gatttggtgt    3720
ttgtagggat tggggttttt tgggtttggg tgggattgtg tggttgagtt tgtggtttta    3780
gagttggtag ttggggaaag tgggtttgag tttttgtgtt tgagaaatga gtgttggtgt    3840
tggtgggtgg ggttggggtg gagggatttt ttgtgtttgg ggagtgtgag gtgttggtgt    3900
tgagttgggt ggttgggtgt ggggagaggg tgtgggagtg gtttgtgtgg taggtattat    3960
gtggatgtgt gagtttggtg agggttttgg taggtttggt ttttgtttgg gggtgtgttg    4020
agatggtggg tgagttttat gagagtgttg ttgttatttt gggttaattt tgtggttagt    4080
ttgttggggt gtgggttggt gggtgggggt gagagtgtgt agtgtgttgt ggtgtggatt    4140
ttgtgggtgt ttgtttggtt tttggtttgg gttttggtgt tgttgaaaat ggatgagtgt    4200
gtgatttttt ggagggtgga gtgatttggt tttttttagt tgggaaattt agtattggtt    4260
agggggggttt tggtggtgga gtgggaaaag gaggtgattg tgtgggattg atttggggtt    4320
attgttgggg tttgattttt tttgatatgt tgtttttttt ttgggagttt gttgggttgt    4380
gttggttttg tggtttttttg gggtattttg ttgtttgtga aagatttgtt tgttgagtgg    4440
tgtatgtggg aggtttgtgt ggggttgttg ggtttttggg tttttgggtg tttgtgtttt    4500
gtaggagtgt tgtttggagg tgtagatgta attggtggat tgtggagtga agggttgtga    4560
ggttgtattt tggggttgaa tatttgtata atgtgtggtt tgttagtgtt ttttgttttt    4620
tttggaggag gaatttttaa gattttttta aaagaaagag tttttggttt tttattgttt    4680
ttaaaaaaaa atgttttttt ttagagagtt agttttttgt tgtgtgttag atttttttgt    4740
ttttgttgat gtgaattgta aggttttgga aataaggatg taattgtatg agttaggaaa    4800
gtaaatttat aaatattttt tatgtagtta agtttttttt tttgtttgaa ttgtggtttt    4860
attaagtggt ttttgttaag taatttttgg gtgtttgata gaggtgattt tttaaaagtg    4920
gatattaata gtgattatta gtatgagaaa agattgaaaa gaaaatgatt tttagatatt    4980
tatttttttt ttaaaagtag atttttaatg ttttgtatat ttagatatag gttttgatag    5040
tttaagaagg tatttgtgtg tgagaattag gtaatggtga tgttttttat attattttta    5100
atgagtttgg ttatgttgtt taatatattt agttgttata gggatgtttg aaaaggtgtt    5160
tttttagtta atagttaatt gtgatatttg tttttgtttt tttgttttag gaagatagtt    5220
ttgattttat agttagaatt attttttttgt atttaagatg taaaaatatt taaagtatta    5280
tgtaattttt taaaataata attggtataa gttttgagtg atgttaaata tggtatttaa    5340
taagtttaag gtaaggtttg atttgaatga agtttgtatt tgtataatta ttttttgaaa    5400
aatgaatatt ttaaatgagt taaatttttt ggtattttgg aaagaagtgt ggttgttaga    5460
ggattttata aaattatttt gggttatttg tatatttatt tgtttttttt gaagtatttt    5520
tgttgggttt ttattatgtt agtagattat ttaaatttat ggtttggata ggtgttaata    5580
gaaaaatgtt taaaaattat ggttgggtgt agtgttttat gtttgtaaat ttagttttat    5640
gggaggttga ggtgggtgga ttattggagg ttgggggttt gagattaatt tgattaatat    5700
ggagaaattt tgtttttatt aaaaatataa aattagtgga gtgtggtggt gtatgtttgt    5760
aattttagtt atttgggagg ttgaggtagg agaattgttt taatttggga ggtggaggtt    5820
gtggtaagtt gagattgtgt tattgtaat                                     5849
```

<210> 233
<211> 12201
<212> DNA
<213> Artificial Sequence

<220>
<223> chemically treated genomic DNA (Homo sapiens)

<400> 233

```
taataattgt gtagttattg tttttgtatg ttgttaataa ggtagataga tgtagaggaa     60
atgtttttat aattttttgg aatgtagata tgaagtgtaa attgaatttt tggtagatgg    120
tagaaaaggt gatttgataa tgagtttgaa ataattaaag tgatattttt atttatttat    180
ttttataatt ttatttattt tgaggaaagt ttagaggatt ggaatatgga tataaaggag    240
gataatttaa gattgtaagt tttatttttt gaaaggttgt atttataagt aggttgttag    300
aggttgtgtt ttttttttta tttttgtat ttaggagttt ggaggatttt gttgtttatt    360
ttttgtttta tgttaatttt atgagatagg gatattgttg tttttttgttt agtaattttt    420
gggttgtttg attattattt tttaggttag tgattggatt tattttttttt tttgtgtatt    480
tatgggtaat tgaggttatt tgaggttttg gttttgaatg aatttgagta gtgagagatt    540
tataataaag aattaagtgt ggttttttag gggttgtgtt tgttatttag ggattgaaag    600
agatttgtgg gatgattttt tgtttttttt tagtaggaat tagaataagg ggtgtagatt    660
tggtttaatt tttttgtttt ggttaatggg aagatgaaat agaaaaattt tttttttattt    720
gtaatatgga gatttagggg gttgtttagt tagaaggttg gtaaattgta aaatggtggg    780
gtagggggtt aaggggatgg ttggggtttt tttgtttagt tttttttttta ttttaggatt    840
taggttaggt taaattttta gtgttgtgtg tgtttgaaag tagataggtg ggatttttgg    900
aaagaatata tataatatag tttttgagtt ttagaaggat tttaagatag gagtttttga    960
gttttgaata agaggttatt tagggtaaag tgattgattt ttgtgtggtg tggttgagaa   1020
gagaaggtgt tgttgatttt ttggggagta agttaataat ttagttgagt tgatgaagtg   1080
atgttttttt tgaaaggggt tgttttttgg gttttttttt ttagttggta tttagtaggt   1140
agggaggtag ggtaaatttt tttttttatg tgaagttata aagaattgat gagttttaat   1200
agtatggatt tgaagttgta gataatttta aatatatttg tgttagagtt tatatttttg   1260
tttatttttg tggagttgga gtagatagtg atttttaaaa ttgtttttaa aattttttttt   1320
gaattgattg ggtatttatt gggatttgag gggtttttttt ttagttttta aattattgtt   1380
atatgtagaa tatattggta gatgttatta atgattgttt attttttttt aaaagttgtt   1440
ttgtttatgt attatttagt ttatattaaa ttgttttgat atgttttagt tgttagttta   1500
atttataaag tagttgtaat ttttgatatt atttttgtag tttatttgag gatttttttt   1560
taggatattt tatgaggttg tttttgagg gttgaatttt gagatttaaa ggaaggttat   1620
ttgaggtatt ttggtatatt tttgggtttt taatttttttt ttttgtttgg ttggtaattt   1680
tttgttgttt tttttttttt tttatttgat ttttttgag gtatttttgt tttttttttt   1740
ttgtttgttt ttagtaaatt tttagtgggt gggggaaatt aattttttgg tgttaaagtt   1800
aaatttagag ttgtttgttg gataggattg tagtggggta aatggtgttt gtttttttag   1860
aaagaatgat taattatatg gaggtaattt gttttgggtt ttttgagatt ttgggtggat   1920
ttgtttgtgt tttgttagat ttagtgtaaa tgtagatttt agttgaattg gttaggattt   1980
gagagatagt tgttttattt tttggattgt aattattttt gtgagaattt gatatattta   2040
atgaaagttt tgttaaatgt atgggttagt gtgtttttgg gttattatta tttgggttag   2100
tatattgtat ttattagtgt taatttgggt tttatgtagt agaatttggt taaaaattgt   2160
ttgtttttgg ttatttagga gttttagggg gtagggggaa gtaagaaaaa ggttttattt   2220
gatattgtta gttgtttttg ataagttata gtaaatattt ttagaagtat aattttaaag   2280
ggagaatata aatttttgtt tttgtttggt tgaatgtttt tttgtaatta attgtggatt   2340
tttgtatgtt taggattgaa ttgaggtttg tggttttgta gttgttttga tgttgggagt   2400
atgaaatttt attttggttg agaattgagt aatagattgt ataagaatgg tgttttttgg   2460
tttgttgttt gttaagaaat attaggaaag ttggtgttag aaataattat ttgaatttag   2520
aaagtaaggt taggaagttt tgtgtgtaga gaaaattttt gttaatttat ttagattgtt   2580
ttatttggtt aataaagtaa ttgatattta ataaaattat aattgtagta ggagttttag   2640
gtattagaat aagaattata attaaaggtt tgttgttgtt aataattgtt tttattatta   2700
ttgtttagta tttttttaag taaaaggaaa gaaaaagaga attattttat atttattggt   2760
attttttttt tttttttgaa attagagtat agattttttt gattgttttt taataagtta   2820
ttattattaa tgggaagaga tgtaaattgg ttttaaatat ttatattttg tttgtggtgg   2880
aatataaatg gttaattgta atttttttta aatgtatata attttatttg atttttggat   2940
atgattaaag atattgagga tttatatagt tttattttat attattagag attatataga   3000
aaagataaaa atgggaaagg ggatatatat gtatttattt aatatttatt tttagaggta   3060
aataaaattt ttttgtttta ttttggtttt taaattatat gatgtggata ttagaaaata   3120
taaatttttt agttatatat atttttttat ttgaatattt tatttagtaa aattggtagt   3180
gtaatttatt ttattttttt aaatttgatt ttgatgtgag ttaaatatat agtattattt   3240
ttttgggaat aaatttttaa atttatattt ttattggaaa gttgattaaa gttttttttt   3300
tttttttagt tttggttaga tgagaaggaa taagttgttt tattgtttgt gattttttaa   3360
agaaataatt taaaatttat ttatatttgt tagaggttat tgggagataa ataaatgtta   3420
aatgtgtaat tttatttttt aattgtaatt aaaatttatt tagattttttg aaatgtagtt   3480
tgagagtatg atgtttgtgt aggaatttgt atttgatatt aagtttgatg tggaatgttt   3540
aaatggtaat taaagtagag aaagtaatag tatataaaaa tataagttta agagtttatt   3600
```

aggaggttgg gtgtaatagt tatattttgg aaggtgtata taagggtata tgggtagatg 3660
ttatagattt tagaggaatt taaattttag tatttttttt atattgaaag atattgttag 3720
atatattgag gtagtttgga ggtgatgggg attatattta ataattattt atattttta 3780
tttttttagt aaaggaatta aattagagat ttatattatt gttgttttta aggatatatt 3840
tttgatttaa taatatataa aggttaaaat gagtttttt ttgagaggag agggtttaat 3900
ttttgttttg aatataagag gttgtttttg tgattaagta ttatggataa attgtttatt 3960
agttttatgt atataaaatt ttatagatat atttgatgta ttaaatttat atatagtaat 4020
tagttgagga aattataata atttatgatt aatttagaat tttaaggtaa agtaaaatta 4080
taaagtttgt tattttaatt tggattttaa tgtaaaatgt gttatgatat atgtattgaa 4140
ttttagagta tgtttattat ggtggggttt tttaaatgtt tttttttaat gttggtattt 4200
ttattatgtg ttttgttttt attagtttag gaatttatta agaaaatatt tgtttataat 4260
aatggtgtag aatgaaaaga ggaaaatgtt gtttttatta ttaaggtgaa attgtttatt 4320
attaaaggtt ttttttaagt tgtatgtaag tttttttatt tggaagtgtt attatttgta 4380
ataattttt gtaatagttt attggtttag ggagttaaaa tattatattg tgggtgggag 4440
gtgggggttg gtttgaagga ggatggatag tgagggtgtt ttgtggattt taaatgtaag 4500
ttattaggtt tttggtggtt tttggtgttt aattttattt tataaaagtg gtatgatgga 4560
atatgattag gtaaaatttt atttttggt attgtggtgg tattttttttt tttttttttt 4620
tttattaggg ttaatatgtt tatttttatg ggaaatatat ataaatatta ggaagatttg 4680
ttttgtattg agattaatag tattagtttt gtaaaagtag tttgataaaa ggttttatat 4740
atttaatatt agagattaga aatagattaa aaaaaaaaaa aaaaaaaaat atatattata 4800
taattggtaa atttattaat ttaggtttat ttaattttg tttttatata tgtgttttaa 4860
aataatttat aataaaaatg gaagggaaga tagatttaat ttgaaatttt ttttgagaaa 4920
aaataaatta aaattagttt ttggggaaga aagttttagt taggttagga ggaaatttat 4980
gtatttttga tttttttttt gttttggaga ggtattattt gtttaagttt agttaatgtg 5040
gttggttagg atttatagtt tttattaagg gttagatttt gtgaaagata taaagaaagg 5100
agtttttttaa tatattgggt ttttttatag taataagata ttgaaattaa gtttttttatg 5160
gtttgtgttt gtaggatttt ttagataaaa ttggtagttt tattagataa gaaatggttt 5220
tttagaagtt ttgggggaag tgtgggggtt ttttgtggtt tttttgtgat tgtttttggg 5280
agattgtaat agatgatggg aatatgtaag aatgattgaa gaaggttatt ggtgagttgt 5340
atttaatagt tttttttttt taagtagttt tttgagagaa aattatatat taatgtttgg 5400
ggatgtgaaa taggtttttt attgttttgt aataaatttt tttttatttg tgtttttaga 5460
ttaatgtatt tttaaaaaat ttttataaag ttatggagat ttgtttttat aattattaaa 5520
atattatagt tagttgtata agtttgttta aaaataaagg gtaggagatt agtagtgaga 5580
agaattgaat agtatataaa aattttataa atttagatt attgaaagat tattttgttt 5640
tttggttttg ggttagtgag ttttaatttt agagttaaat tgagaagtag attttgtggg 5700
tttgaaagat aaaaagttag tttgtggagt taattttggt ttattggtgt gttttaattt 5760
gattgtttta atagtaagaa gtgttttttt ttttttattt aatgttttta gatatttgag 5820
gttgggggag atggagtaat ggtgtagtag ttaggaaata gtttttttgg gtttaattat 5880
tttatttttg attttttttt ttttagtgtg ttgagtgtag ttagttttgg aggaattagt 5940
tttttggagt agttaaggta ggttaggttt tggggatgtg tattatggtg tggggattaa 6000
attaagttga atggtttgtt ttaagttttt tttttttttt agagtttggg agggattttg 6060
ttaataggta gatttattta ttagttattt gttattggtg gtagaaagtg agttttgtgt 6120
gtaatgtggt tagttggatt gtggggattt taggagtgta gggtggagga gtagtgttat 6180
aggaggtgag ggtgtaggtg gtgtggttgg gaaggaatgt gggaggggat agaaggaaga 6240
ggaagaggag gagagggagg ttagagttag aatagtttgg tagtttgagt tttgggggag 6300
aatggtttga gttttgagta agttgttttg ggagttttaa tttttttttg ttggtttgtt 6360
gagtggttag tggtgtttag tggtggtgag gttgaaatat gataattaga atagttgtgt 6420
tgtgtgtttt gtagttaatg ggtgtggtgt ttgtttgatg tttttgtgtg ttgtgttaga 6480
ttaatggtga tggagttgag ttggagtaga gaagtttgag taagagataa ggaagagagg 6540
tgtttgagtt gtgttgagtt tgttgttgtt gtagtgtttt tgttttgtta attttgttgg 6600
tttaaattgg atttttagat ttgtgagggt gtggtgtagt tgagtagtgg ttttttttagt 6660
attggtaatt ttagggggtta atatttttta tttagttata gttttagtat tttttttgtg 6720
ggttgtttat taattgtata attattattt tattgtggat attatttttt tttatagata 6780
tgggagatat gggagattta ttaaaaagta agaggttatt ttattttgtg gggtttggtg 6840
tgttgttttt gtgtggggtt ttttttttagg tataggttga ggtgttaagg gtttttttgga 6900
gttggagtta ttgtttggag aaagagaaaa ggtggttttt ttttgttgtt gttatgtttg 6960
tatgtttatt gttggttttt atttttggga aattgattgt attttgtgtg tgaatttgtt 7020
tgtgtgtttt ttaaagtttt agttttttgg tgttaagtgg tgattttttt ttggggaatt 7080
ttgagttttt tgagaaggtt attatgttgt aaaggtttgt ttgtatagtt aatgtttaga 7140
gatgtgaatt agtattagat ttgtaaaaga gaatgagtga taattgtatt tatgtttgtt 7200
tttgttaata atatttagtt ttgtgtgtta tttaagagtg tgttttatgg aaaatataga 7260

```
tatttttgtg tttatttaat gtttttagtt aaaatttttt ttttgatttg atttatttat     7320
aatttttttt aatgattata gtaaagagga aggggggggg gagaaatata aaatgagtgg     7380
gtttgattgt gtgttaggtg tataaatgta gattatttta atttgtattt tatatatatt     7440
ttattttttt ttaaaaatga gttaaggttt tgatggtata ttttaattat tattttaaag     7500
tgtaatgttt taaaaaaaaa aaaaaagaaa gaaagaaaga aagaaaaaaa ttttttagag     7560
tatgttttat aagagaatga tattaaaagt gtgtttattt ttgtaggaag taaatggtta     7620
gttaattatg tatttatttt tattttagaa aaatgtttga tttttttatg tgttggttgt     7680
ggtaattaga tttatgatta gtatattttg agggtttttt tggatttgga atggtatgtg     7740
gtatgtttga aatgtgtgga gtgtaattag tatttggatg agagttgtat atgttttgtt     7800
agggatggga aaatttattg taaaagagat tatattaggt atggtattta tatttttttt     7860
ttaattttgt gggatttttt tgaatttttt tattttttat gtattatttg gtgtggtttt     7920
gtttttttgt gaagtttgtt ttagtgtagt tatataagtt aaagttattt tgtatatgtg     7980
ttaaaaaaat taagttatgt tgtttatttt atttttagt tgagaaaaat aaaaattttt     8040
aatagtggta tttataattt tggggtattg aggtttgttt aattattttt ggagtttatg     8100
atgtataaat tatttttttt ttttattttt tttttataa aataaaattt taaaaagatg     8160
gagaagtttg gatttttagt tttaaaatag ggttgatttt tgttgtatag tgtagtgttt     8220
tgtttgtttt agtttttttt aaaattagta gtttataaat tttttggtgg tattaatgta     8280
ataggtgaaa taaaagtttg attgaagtat gtttagagat gtattttgaa agagtgagta     8340
taggtattgt ttttttttatt tttggggtaa gatttttttt tgagaaaaat ttaaaattaa     8400
tttaaatatt ttttggaaaa aatattggaa atttaatttt tttaaatatt aattttttgg     8460

tgatatttaa ttgttttttt tttttattt tatttgagtt gatgaattag agtagattaa     8520
attgtttatt atttgtttat gaataattgg tatatttaga taattgaata gttttttttt     8580
ttatattaaa atttggtaat tgataaaatg agtgaattgt ttaaattgat aagattgaaa     8640
taatatagga attttttgag tttggttttg ttgttttgga gagtttttgt tttttttttt     8700
ttttaattta ttttgtaata tgttttgtta attttaagtt ttttttgatt tgtgtgtatg     8760
tattagaaat tttgtttttt gttttagaaa gttagtagtt tttaaagaaa attgtattta     8820
ttttattaat aaatagaaga gatattagta ttattattat gttaaataat agtaaaatat     8880
tattttttaa atgtttggtg gttattaata agtaattaat tagtttttgt taggtaaatg     8940
gtttttggag tttgagaatt tttattaaag tttagttaag atttaatata tagttatagt     9000
ttgtttttgt ttatttagta tttagtattt tttttttttt ttttaaaaat tatgatatag     9060
agagtataat tttggtagat aaaattaatt tggttggggg aggttaatat tttggagagg     9120
gagtgaaagg aagtaaggga agttggggta taggaagggg gagggatttt ttaaattgtt     9180
tggttattgt taaagttaag tttttatttt atgaaatgga agattttata ttgagtaggt     9240
ggagggagga aaaattttg agtttatttt ttaattttg ataaatgagt gtttttattg     9300
tttattagat tggtgtgtaa atgtaagatt ttagagaagg agagtttatt ttaggagtat     9360
ttttattgtt atggaaatag tattttgttt aattgtatat aggtttgtat gtgtttaatt     9420
ttggatatat atatgtgtag aaggaattaa ttatttttat ttttttttta ttttttttta     9480
atttgtgtg tgtgtgtgtg tgtgtgtgtg tgtgtgtgtg taattttgta gttgtaaaag     9540
tagaatagat tggatagtta gattttata ttttttttg gagaagtagg atgttttttt     9600
ttgttatgtg gatttttttt tttttttttt attttttttg tttgtaggaa tgttttagtt     9660
tttgttattt ttgaaagatg gagaaggggt tagggaagtg tagtttagat ggaatttata     9720
aagattgttt tttggtaagg aaaggttagg agtgagaaag atttagaag tgggtttttg     9780
tattttttt attttggtta tggttttaa gaaaattgaa atgaggtaga tgatttagta     9840
atttgaaaaa gattgaggga atagatgtag atttttttaa aaaaataata atataaggaa     9900
gaatggagag gaaatttttt gttaatattg ttgtttgaag aaaattttta gttggagaaa     9960
gattggaaag tatttgtgta aaaggagatg tggaaatttt tagaggtttt attttgttat    10020
tttgtttgtt tatttgtgag tgtttgtaaa ttgagtgggg tgataatttt ttttttttat    10080
tttttttttt tttggaagga ggattttttg ttgtagtttt agatattttt agtagtagaa    10140
attgtgggat agggaagtga aagtgttggt gttggtggtt attagagttt ttttggattt    10200
tttttgtta agatttgtaa gattaatatt gggattgatt gttagagtag tagtttgagt    10260
ttggaattta ttaatatatt ttttgtggta taagttaggt gttttgagtt aagagttatt    10320
aattaagata gaggtttatt ggaaaggaaa tgggagtaaa agaaagggag gagggaggga    10380
ggggaaaaga gagatggggg aaggaagaga gatagggaag gagagagtag ggttttattt    10440
ttgttttttt gtttttaatt tttgtttgga aatgttgttt atttggggtg ttttgtttgg    10500
gattttgggt tagggaagtg ttggtttgaa gtgatttttt tttttgtat tttttttttt    10560
gttttgggtt gtttttgttt tttttttttt tgtataggtt gtatgggatt aaatgtgtta    10620
agtgtagtat tggttttagt aagaatgatt ttgtgatgtg tgtttgtttt aaggtgtatt    10680
atattgagtg tttttgttgt gtggtttgta gttgttagtt tatttttggg gatgaatttg    10740
tgttttggga ggatggtttt ttttgttgag tagattatga tgtggtggag agggttagtt    10800
taggtgttgg tgatttgttt agtttttgt atttagtgtg gttattgtaa atggtaggta    10860
```

```
ttttttttgtt tggtttgggt aggtaggtgt taggttaagt tagtttgtgt gttagtggtt   10920
ataataatta tggtagttat aggggtggtt gtagtgtttg tttgtagtta aatgaagtgt   10980
tttgtatgta atttgtgttg tgttttgttt ttttgtagta aggtttaatg tatttattgt   11040
tttttttgat tttttgagta tatttatatt gtttgtgtgt ttttatatgg ttatatataa   11100
atgtatatta tttgtgtata tgtgtatata tatgtttaaa tattattttt agtttgtttt   11160
ggtttttaaa ttttggtttg ttgaaaatgg gttttagttt ttagttaggt attttttttgt   11220
tgtttaatta aaggggtgga gttttgggtt tttggagttt tattttttaa tttaatgaag   11280
gaagtttagg tggtttgaag ttatttagtt ttttaaattt ttttttttg tgagttgttt   11340
tatatttgaa attttttttt taattttttt atttttttgt gagagaatag gattgaaaag   11400
atatagtttt aaaaattgta ggttattgta tagagttgta atataaaatt gttaataagt   11460
ttatttgtag taattgtttt ttaaagggag tttgtttttt taaattattt attttatatg   11520
atttggtgag aattttattt ttaggtttat ggttgtagtt ttaaattgat tttataggtg   11580
ttggtttgat tttagggggt tgtagaaggt gtaggatttg tattatgtag ataagaggat   11640
ttatttttaa ggaagaggag tggaaatata gtaaggttgg ttgggattaa agtagtgggt   11700
tagaaggtgg atagtgtttt taaatttgat tttttgttat gaatagattt atttttttgt   11760
agttttaaag tattaatttt tattaaatat tgaaggtgag gaaattatag tttttttta   11820
ttttttttgtt ttttggtagt tttaagaatt ttgtttaggg ggatttgtga ttagtttgta   11880
ttggggtatg gttggggtgg tgtttttgtt tagtggagtt tgtattttgt ttgtggggaa   11940
gtatagagga agttaaaata ttgagaggga ggtgggggat atttttagt tattgtttat   12000
ttagagttta ggtagtttaa tagagttttt gtttttttatt gtttgggatt agtttatttt   12060
aggaatattt atgtattatt ttagatttaa tattaagagt agggattgga gatatggtag   12120
aaatagtgaa tttttttagt ttttttatat gattgttttt ttggattgaa gtttaaggtg   12180
ttttggtaga gtttttgatt t                                              12201
```

<210> 234
<211> 12201
<212> DNA
<213> Artificial Sequence

<220>
<223> chemically treated genomic DNA (Homo sapiens)

<400> 234

```
aggttgagaa ttttgttaga atgttttgaa ttttagtttg agaggatagt tatgtgaagg     60
ggttgaagag atttgttatt tttgttatat ttttaatttt tgtttttagt attaaattta    120
aggtaatata tggatgtttt tgagatgggt tgattttaag tagtggaaaa tggaaatttt    180
gttgagttgt ttaggtttta gataaatggt ggttgggaga tgtttttttgt ttttttttttg    240
gtattttagt ttttttgtg ttttttata agtagagtgt aggttttatt gaataggagt    300
attattttag ttgtgttttg gtgtaaatta gttataaatt tttttgaata gaatttttag    360
agttgttaga aggtagaagg gtaagggagg gttatagttt ttttattttt agtgtttagt    420
gggaattgat attttaaaat tgtaaagggg tagatttatt tatggtagga agttaggttt    480
ggaaatattg tttatttttt aatttattgt tttggttttg gttaattttg ttgtgttttt    540
attttttttt tttgggaatg agttttttta tttatatgat ataaattttg tatttttttat    600
aatttttttag ggttaggtta atatttatgg ggttaattta gagttataat tatgggtttg    660
aagatgaaat ttttattaaa ttatatagaa tgaatgattt aaagaagtag gttttttttta    720
aaaggtaatt attgtagata agtttgttga tagttttata ttataatttt gtgtaatggt    780
ttgtagtttt taaaattgta ttttttttagt tttgtttttt tatagaaaga taaaaaaaatt    840
aaaaaaaaaa ttttgagtgt gaagtaattt ataaggaaaa aggatttggg agattaaatg    900
attttaggtt atttaagttt tttttattgg gttaaaggat gaagttttag ggatttgggg    960
ttttgttttt ttaattaggt agtaggagaa tatttggttg ggagttgaag tttgttttta   1020
gtaagttaag gtttggaggt tagagtgaat tggaagtaat gtttgggtgt gtgtatatat   1080
gtgtatataa gtggtgtata tttatgtgta attatataga gatatataga tggtgtaggt   1140
gtgtttgggg aattaaggga gatagtgagt gtattgaatt ttgttgtaaa ggagtagagt   1200
atagtgtaaa ttgtatatag aatattttat ttaattgtag gtaaatatta tgattatttt   1260
tgtagttatt atagttgttg tggttgttgg tatataggtt ggtttaattt ggtgtttgtt   1320
tatttgagtt gggtagagga gtatttgtta tttgtagtgg ttgtgttgga tgtaggggat   1380
tgagtgggtt gttagtgttt agattggttt ttttattat attgtggttt gtttggtaga   1440
agagattgtt tttttgaagt gtaaatttgt ttttagggat gagttggtgg ttgtaggtta   1500
tatagtggaa atatttgatg tgatatattt tggagtgggt atgtattatg aagttgtttt   1560
tgttgaagtt gatgttgtat ttggtgtatt tgattttgta taatttgtgt gggggagggg   1620
```

```
ggagtggagg tggtttagag tggggagaga agtataggaa gagggggtta ttttaggttg    1680
gtattttttt ggtttaagat tttgggtaag atgttttaag taaatagtat ttttaaatag    1740
aagttggaaa tagaaggata gaaatgaaat tttgtttttt ttttttttgt tttttttttt    1800
ttttttattt tttttttttt tttttttttt tttttttttt ttttattttt gttttttttt    1860
tgatgaattt ttgttttagt tggtaatttt tagtttaaaa tatttagttt gtattataga    1920
aaatgtattg atgggtttta aatttgggtt gttgttttag taattaattt tagtgttgat    1980
tttgtagatt ttggtaggaa ggaatttaga aagattttgg tggttattga tattaatatt    2040
tttatttttt tattttataa tttttgttgt tagaaatgtt taaaattgta ataaaaagtt    2100
ttttttttaa gaaaaaagga ggtaggagaa ggggattgtt attttatttg gtttgtaaat    2160
atttataaat aaataagtag aataataaaa tgaaattttt gagagttttt atattttttt    2220
ttgtataagt atttttttagt ttttttttaa ttaaagattt tttttaggta gtaatgttaa    2280
tagaaaattt tttttttatt tttttttgta ttattatttt tttaaaaaaa tttgtgtttg    2340
tttttttaat ttttttttaag ttgttgaatt atttatttta ttttaatttt tttgaaaatt    2400
atgattagaa tgaaaaaaat gtaaagattt gtttttaagg ttttttttat ttttggtttt    2460
tttttattaa gggataattt ttataggttt tatttaggtt gtattttttt ggtttttttt    2520
ttattttttta ggaataatag aagttggggt atttttatga atagaaagaa tggaagagag    2580
aggaaaagat ttatataata ggaggaggta ttttgttttt ttaaggagag atgtggaaat    2640
ttaattgttt agtttgtttt gtttttataa ttataagatt atatatatat atatatatat    2700
atatatatat atatatagag ttgagagaga gtgaagagaa ggtaaaaatg attagttttt    2760
tttatatatg tgtgtattta gaattaggta tatgtaagtt tgtgtgtaat tgagtaaaat    2820
attattttta tagtagtaaa gatatttttg aagtgggttt ttttttttta gaattttgtg    2880
tttatatatt aatttagtaa ataatgaaaa tgtttatttg ttagaggtta gaaggtggat    2940
ttaaaagttt tttttttttt tgtttattta atgtgagatt ttttatttta tagggtgaag    3000
atttgatttt ggtggtgatt aaataattta gaaaattttt tttttttttt gtattttgat    3060
tttttttatt tttttttatt ttttttttga agtattaatt ttttttaatt aggttaattt    3120
tatttattaa gattatattt tttgtgttat gatttttaaa aaagaagaaa aaaatgttgg    3180
atattaagtg agtaggagta aattgtgatt gtatattaaa ttttaattaa attttaataa    3240
aagtttttaa gttttagaaa ttatttgttt aataaaagtt aattaattat ttgttaatag    3300
ttattggata tttaaaaagt gatattttgt tattatttaa tataataatg atgttgatgt    3360
ttttttttgtt tgttaataaa atgaatataa ttttttttag agattattgg ttttttaagg    3420
tagaaaataa agtttttgat atatatatat aagttagagg aaatttgaga ttagtaaaat    3480
gtgttgtaga ataaattgga ggaaaaaaaa aataaaaatt ttttaaaata ataaaattaa    3540
atttagaaag tttttatgtt gttttagttt tgttaatttg gataatttgt ttattttatt    3600
agttattaga ttttaatgtg agaaaggaag ttgtttaatt atttaaatat attaattgtt    3660
tgtagataga tgatgggtaa tttgatttgt tttaatttat tagtttagat aagataagaa    3720
agaagagata gttagatgtt attaaagggt taatatttaa aaagattaag tttttggtgt    3780
ttttttttaag gaatatttag gttggtttta aattttttttt agaaggaggt tttattttaa    3840
aaataaaagg agtaatattt gtatttgttt ttttaaagta tatttttaaa tatgttttgg    3900
ttaaattttt attttattta ttgtattgat gttattaaag aatttgtaag ttattaattt    3960
taaaaaggat taaaataaat agaatattgt attatataat aaaaattaat tttattttaa    4020
agttaaaaat ttaaattttt ttattttttt aaaattttgt tttgtaaggg ggagggtgaa    4080
agaggaaaat aatttgtgta ttataaattt taagagtaat taaataagtt ttaatatttt    4140
ggaattatga atattattgt taagggtttt tgtttttttt aattaaagaa tgaaatgaat    4200
agtatagttt gatttttttta atatatgtat agggtaattt tggtttgtat gattatattg    4260
aggtaaattt tataaaaaga taaagttata ttaaataata tataaagagt ggggagattt    4320
agggaaattt tataaaatta agaaagaagt gtaaatgtta tatttgatat aattttttttt    4380
atagtaggtt tttttatttt taataaagta tgtatagttt ttgtttaaat attgattata    4440
ttttgtatat tttaaatatg ttgtatgtta ttttaaattt ggagaaattt ttagaatata    4500
ttgattgtga atttgattgt tgtaattaat atatagggaa attagatgtt tttttgaaat    4560
gaaaataaat atatgattga ttaattgttt atttttttata gagataaata tatttttagt    4620
gttgtttttt tatagggtgt attttgggag gttttttttt tttttttttt tttttttttt    4680
tttttttttt agagtattgt attttgggat ggtaattgaa atgtgttatt aaaattttga    4740
tttattttta aaaggaggtg gaatatatgt aaaatgtaga ttggaatagt ttatatttgt    4800
atgtttagta tgtaattaaa tttgtttatt ttgtattttt tttttttttt tttttttttg    4860
ttataattat tgggaaagat tatggataag ttaagttaaa gaaaaggttt tgattagaag    4920
tgttaagtga atgtagggat gtttatattt tttgtgaagt gtgtttttaa atagtatata    4980
ggattggata ttgttagtaa gagtaggtat aaatatagtt gttatttgtt ttttttttgta    5040
agtttaatgt taatttatat ttttgggtat tgattgtgta ggtagatttt tgtaatataa    5100
taattttttt ggagagttta ggatttttta ggaggaaatt attgtttagt attagaaagt    5160
tagagttttg gagggtatat aggtgaattt atatataagg tataattagt tttttgaaga    5220
taagaattga tagtaagtat gtaggtgtgg tggtaataag aaaaagttat tttttttttt    5280
```

tttttaggta atgattttaa ttttaaagag tttttggtat tttagtttgt gtttgagaga 5340
gaattttgta taagaatagt atattgagtt ttataaggta aaatagtttt ttattttttg 5400
gtggattttt tatgtttttt atatttgtaa gagggagtaa tgtttatagt gaaatggtgg 5460
ttgtatagtt ggtgaatagt ttatagagag gatgttggag ttgtggttaa gtgggaaata 5520
ttggtttttg gggttgttaa tgttgaaaga gttgttgttt ggttgtgttg tgtttttgtg 5580
gatttaggag tttaatttaa gttggtggag ttggtggagt ggaggtgttg tggtggtggt 5640
agatttggtg tggtttgggt attttttttt tttatttttt atttaaattt ttttgtttta 5700
atttagtttt attgttattg gtttgatgta gtgtgtgggg atgttaggtg agtgttgtgt 5760
ttattggttg tagggtgtgt ggtgtagttg ttttgattat tatattttag ttttgttgtt 5820
gttgagtgtt attgattgtt tggtgagtta gtgggagagg attagggttt ttgaggtaat 5880
ttgtttgggg tttaggttgt ttttttttga agtttgggtt gttgggttgt tttggttttg 5940
gttttttttt tttttttttt tttttttttt tgtttttttt tgtgtttttt tttggttgtg 6000
ttgtttgtgt ttttgttttt tgtggtgttg tttttttgtt ttgtgttttt ggggtttttg 6060
tagtttggtt ggttgtgttg tgtgtggggt ttattttttg ttgttggtgg tgagtggttg 6120
gtgggtaggt ttatttgttg atgaggtttt ttttgggttt taggaaaagg ggggaatttg 6180
gagtaggttg tttagtttgg tttggttttt atgttgtggt gtatattttt ggggtttggt 6240
ttgttttggt tgttttggag ggttagtttt tttagaattg gttgtgtttg gtgtgttagg 6300
ggaagaagaa ttgggaatgg gatgattaaa tttagaggaa ttgtttttttg attgttatat 6360
tattattttg tttttttttaa ttttagatat ttaaaaatgt tgggtgggag gaaaggggtg 6420
tttttttgttg ttgggataat taggttggaa tgtattggta aattgaagtt gattttgtgg 6480
attgattttt tgttttttag atttgtgagg tttgtttttt agtttaattt tagaattgaa 6540
gtttattaat ttgaagttaa agagtgaaat gattttttag tagtttaaag tttgtaagat 6600
ttttatatat tgtttaattt tttttattgt tggtttttttg ttttttgttt ttaagtaaat 6660
ttgtgtaatt agttgtggta ttttggtaat tataaggata agtttttatg attttgtgga 6720
gatttttttaa aaatgtatta gtttaagggt gtgagtaggg agggatttat tatagaatag 6780
tgaaagattt attttatgtt tttaggtgtt aatatataat ttttttttga gaaattattt 6840
ggagagagaa gattgttagg tgtaatttgt tggtaatttt ttttaattat ttttatatgt 6900
ttttattatt tgttatggtt ttttaaagat agttatagga aggttatgaa gaatttttgt 6960
gtttttttta aagtttttaa aaagttattt tttatttaat ggggttgtta gttttattta 7020
aaaagtttta tagatataaa ttatagaagg tttaattttg gtgtttttgtt gttgtgagaa 7080
gatttggtgt gttaggaaat tttttttttt atattttttg taaaatttaa tttttgataa 7140
gagttgtaaa ttttggttgg ttgtattggt tgggtttgaa tgggtaatgt ttttttaaag 7200
tggaaaggga attaaagatg tgtaagtttt tttttgattt agttgaggtt ttttttttta 7260
ggaattagtt ttgatttgtt ttttttaag gaggatttta aattggattt gttttttttt 7320
ttattttttat tataagttgt tttagagtgt gtgtatgaaa gtgaaagttg gatagattta 7380
ggttgataag tttattagtt gtgtggtgtg tgttttttttt tttttttttt tttggtttgt 7440
ttttagtttt tggtattaga tgtatggagt tttttgttag gttgtttttg taaagttgat 7500
gttgttaatt ttaatatgaa gtaaattttt ttagtatttg tgtgtgtttt ttatagagat 7560
aaatatgttg attttagtaa aaaaaaaaaa aaaaaaagat attattataa tgttagaaaa 7620
tagagtttta tttagttata ttttattatg ttatttttat aagatggaat taaatattag 7680
aagttattag aagtttgatg atttatattt gagatttata aagtattttt attatttatt 7740
tttttttaaa ttagttttta tttttattt atagtgtggt attttaattt tttaaattaa 7800
taagttatta taaagaatta ttgtagatga tagtattttt aagtagaaaa gtttgtatgt 7860
ggtttggggg aaatttttag taatagataa ttttatttta atggtgaaga taatattttt 7920
tttttttttat tttgtattat tgttgtaaat aagtattttt ttaataagtt tttaaattga 7980
tagaagtaag atgtatggtg aagatattag tattaaaagg agatgtttga gggattttat 8040
tatgatggat atgttttgaa gtttagtgta tgtattatga tatattttgt attaaaattt 8100
aagttagaat agtaggtttt gtagttttat tttattttga agttttaaat tgattatgga 8160
ttattatgat ttttttagtt gattgttgta tgtggattta gtgtattaaa tatatttgta 8220
aaattttgta tatataaaat tgatgagtaa tttatttgta atatttgatt ataggagtga 8280
tttttttgtgt ttaaagtggg gattagattt tttttttttta aaaaaggatt tgttttgatt 8340
tttgtatatt gttaggttaa aaatatattt ttgaaaatgg taatagtgtg aatttttggt 8400
ttgatttttt tattgagaaa atgaggagta tgagtagtta ttaagtgtag tttttattat 8460
ttttaggttg ttttaatata tttgatagtg ttttttaata taaaagggat attgaaattt 8520
aagttttttt ggagtttgtg gtatttgttt atgtgttttt gtatatattt tttaaaatgt 8580
ggttgttata tttaattttt tgatgggttt ttagatttgt gtttttgtgt attgttgttt 8640
tttttgtttt agttattatt taaatatttt atattaaatt taatgttaga tatagatttt 8700
tgtataaata ttatgttttt aagttatatt ttaaaaattt aaataggttt taattgtaat 8760
tgaaaggtaa aattatatat ttagtattta tttatttttt aataattttt ggtagatata 8820
aatgaatttt aagttgtttt tttgggaaat tataaatagt aagatagttt attttttttt 8880
atttaattaa gattgggaag ggaagagggg ttttgattag ttttttaatg gaggtataaa 8940

```
tttgagaatt tatttttagg gagatgatgt tatatgttta gtttatatta gaattaggtt    9000
tgaaagagta gaataagtta tattgttagt tttattgagt aaggtattta gatgaaaaaa    9060
tatgtataat tgaaggattt atattttttg atgtttatat tatataattt gggggttaaa    9120
atgaaatagg aagattttat ttatttttaa aaatggatat tgagtagatg tgtatgtatt    9180
ttttttttg tttttatttt ttttgtgtag tttttaatga tgtagaataa ggttatataa    9240
atttttagta tttttaatta tgtttaaggg ttagataaaa ttatgtgtgt ttaaagggag    9300
ttatgattaa ttatttatat tttattgtga atggggtgtg ggtgtttaga attaatttgt    9360
gttttttttt gttggtggtg gtggtttatt gaggaataat taggagaatt tgtgttttaa    9420
ttttagagaa aaggaagaga tattagtggg tatgaaatga tttttttttt tttttttttt    9480
atttaaaaaa atattaagta atgataataa aggtagttgt taataataat aaatttttag    9540
ttgtgatttt tattttgatg tttaggattt ttgttgtgat tgtggttttg ttgagtgtta    9600
attgttttat taattagata aaatagtttg ggtgaattag taagaatttt ttttgtatgt    9660
agagtttttt agttttattt tttgagttta ggtagttgtt tttgatatta gtttttttgg    9720
tattttttgg tagatgatag gttagaaggt attgtttttg tgtggtttgt tgtttggttt    9780
ttagttgaga tagaatttta tgtttttggt attggggtag ttgtgaaatt ataaatttta    9840
gtttagtttt gagtatgtga aggtttgtag ttgattgtag ggaggtgttt ggttaagtga    9900
ggatggaaat ttgtattttt tttttaaaat tgtgtttttg aaagtattta ttatgattta    9960
ttaaaagtgg ttggtagtgt taagtgaaat tttttttttg tttttttttg ttttttagaa   10020
tttttaagtg attaggaata gatgattttt aattgagttt tgttgtgtgg agtttaggtt   10080
ggtgttagtg agtgtagtgt gttgatttaa gtggtggtgg tttaaggatg tgttgatttg   10140
tatatttggt aaagttttta ttaaatgtat tagatttttg tggaagtggt tgtagtttgg   10200
agagtgaaat agttattttt taagttttga ttggtttagt tgaaatttat atttgtattg   10260
aatttggtaa aatatggatg ggtttgtttg agatttttagg aaatttagag taagttattt   10320
ttatgtagtt gattattttt tttgaaagga tggatattat ttattttatt gtaattttgt   10380
ttaatgggta gttttgagtt tagttttagt gttaaaggat tgattttttt tatttgttgg   10440
agatttattg gggatgagtg ggggaggga ggtgaaggta ttttggagga ggttaagtgg   10500
gggaggagga ggagtagtag aaaattatta gttaagtagg ggagggagtt ggaagtttgg   10560
aaatatatta ggatgtttta agtaattttt ttttaagttt tagaatttag tttttggaaa   10620
atagttttat aaggtgtttt gggaaggagt ttttgggtgg gttgtggagg tgatgttaag   10680
agttatagtt attttatgaa ttaaattgat ggttaaggta tgttgggata gtttagtgta   10740
agttgaatgg tgtgtggatg gggtaatttt taaagagagg tgggtaatta ttggtggtat   10800
ttattagtat attttgtatg taatagtagt ttggaagttg aagaaaaatt ttttaaattt   10860
tagtaaatgt ttggttagtt tgagagagat tttagggata attttgaagg ttgttgtttg   10920
ttttaatttt gtagagatga ataagagtgt gaattttggt gtgggtgtgt ttagaattat   10980
ttatgatttt agatttatgt tgttgaaatt tattaatttt ttgtgatttt atgtggagag   11040
aagaatttgt tttgtttttt tatttgttaa atgttgattg agaaaagggg tttaggagat   11100
gatttttttt agaaagaatg ttattttatt aatttggttg agttattaat ttgtttttttg   11160
aaaggttaat aatgtttttt ttttttagtt gtattgtgtg gagattaatt gttttatttt   11220
aggtagtttt ttgtttaggg tttaggaatt tttgttttaa ggtttttttg gggtttagaa   11280
gttgtgttgt gtatgttttt tttaagaatt ttatttgttt gtttttaagt atatatggtg   11340
ttagaaattt agtttagttt gagttttggg atgagagaag agttaaataa agagatttta   11400
attgtttttt tggtttttttg ttttgttgtt ttgtagtttg ttaatttttt agttagatag   11460
ttttttaagt ttttgtgttg tgagtgaaag agaatttttt tattttattt ttttattgat   11520
tgaagtagaa aaattgaatt gaatttatgt tttttgtttt gatttttgtt agaggaaaat   11580
agaaaattat tttgtaggtt ttttttagtt tttggatggt gagtgtagtt tttgggaggt   11640
tatatttagt tttttattgt gaattttttg ttatttaagt ttgtttggga ttagggtttt   11700
ggatggtttt ggttgtttgt aagtatgtga aagaagaggt gaatttaatt gttggtttag   11760
aggatagtga ttagataatt tgaggattat taaataaggg gtggtggtgt ttttgtttta   11820
tggggttggt gtggggtggg gggtaggtag taagattttt taggtttttg gatgtaaaga   11880
gtgagaaaga aagtgtagtt tttggtagtt tgtttataaa tgtagttttt tggaagatga   11940
aatttgtagt tttaggttgt tttttttttat atttatgttt taattttttg ggtttttttt   12000
gaaatgaata aaattgtgga aatgaatgaa tgagaatatt attttaattg ttttaaattt   12060
attgttaagt tatttttttt gttatttgtt agaggtttaa tttgtgtttt gtgtttatat   12120
tttagaaaat tgtaaggatg ttttttttgt atttgtttgt tttgttggta gtgtgtggaa   12180
atgataattg tatagttatt g                                              12201
```

<210> 235
<211> 22692
<212> DNA
<213> Artificial Sequence

<220>
<223> chemically treated genomic DNA (Homo sapiens)

<400> 235

```
taggtaattg gtgattaaaa atatttttag gtagtattga gtgttaggaa aagggggttt      60
tggggaggtg agtgaatatt tatgttttta attgaaagtt tttatattta agttattttt     120
tttgtttttt ttaaattaat ttaaagggtt ggtttatggg ttttttgggg gtattagtgg     180
tttaatgtgg gttttggggg tagtgagggt gggagagagt tagtggtttt ggggttatta     240
ggtgtgttta tggtggaaag aggttgtgga gttgggtagg tgagatagat gtgttttttt     300
gttagtttta ggtaggattt agtttgtaga ggataaatgg gtagttttgg ttgtgtgtgg     360
ggaggagatg tgagggtgtg aggaggttgg gtaattttgg ttatttagtg tttggatgat     420
gttgttatgt aagatttgag attttgttgt atattatttg atttttttag tatgtgaggt     480
aagttttgtt atttttattt tagagatgag gaaagttgag gtttagaatg gttaggtgat     540
ttagttaagg gtgtgtagat aatgtgaggt agagttaaga tttaagttta aatgttttgg     600
tttttgttta tttggattat gaagttagtg tttttgagga ttttttatta ttgtttttta     660
aaataaattt ttttattggt agttggtagg agaaaatttt gttgttttgg gaatggaatg     720
gtatttgggg ttttagttag ttatttaagt tttggtgtga gtggtttatt ttgttttttt     780
tagggttggg aatttttgaa ggagaggagg agggagggta agggtttagg atagttgtgg     840
gttttaggtt aggtattatt gggttttggg agtttggagt atttatgaag ttaggtaggg     900
ttggttttga ttttatttat tttttatttg ggaggtttgt ggtttttgtt ttggaggagt     960
ttggggtagg tttttatggt gtttttgtta ttttggtttt ttttttgtat tgtaggtttg    1020
tgtttgttag gtttgggtta tgggtaaaat gtatgtttgt aagtgtttgg agaagaagag    1080
gattaaaaag aggaaagggg agtttatggt ttttaatgag aagtagattt ttgagaaggt    1140
taatagttag tttgtggtga gtgagtattt gggtttagtg attggtttgt tttttttgtgg    1200
attggggttt tttttttttt ggaagggtgt ggttttttaa tgtggttggt ttttattttt    1260
gggaagggga atgttagtgg tagtgttgag ttatagaaag gttgtaagat atttttttat    1320
tatatggttt attgtggttg attgtggttg gttttgtttt atttttagag ttggttagtg    1380
tttggttttt tgttgtgtat gagattgatg tttttgtttt tttggattat tttgtaaatt    1440
ttttagtgga taaagaaagt ttatttgggt ttaggttggg agttgtgggt tttatagatt    1500
ttttaggttg gtgttattgt ggtggggatt ttagtgttag tattttggaa tggtattatt    1560
aagtttgtgt gtattgtgtt agttagttta ggttgggttt tgttgtagta atatatttgt    1620
tttaaatttt aagtagttaa atatagtaga ggtttatttg ttatttattt gaagtttaat    1680
tagattgggt tatttttttt tatttaatag ttgttttatt tggagttttg aaagtgagtg    1740
ttaggggttt tatatagaga ttttttattg tttttattgg atagatgttt tttagtttta    1800
tttatagttt gttagttaga attagttaat atagagataa gttagtttta ggggaggttg    1860
ggaaatgtga agtgtatgga tatttggtta gttttaatat tttttgttat atattgttat    1920
aaaaaataaa gttgggtgta gtggtatatg tttgtagttt tagttattta gaaggttgag    1980

gtaggaggat tgtttgaatt taggattttg aggttgtagt gaattatgat tgtgttattg    2040
tattttagtt tgggtgatat agtaagattt tatttttaaa attaaatata tgtatatatt    2100
aaaaataata tatatataag gtaaaaattt aaatagtata gaaggatata taataaaatg    2160
aaaagttttt ttttttgagt aattatttta ggtatgtttt atttatgtat aggttttatt    2220
atttttagtt aatgggattt ttttgtattt tttatggagg aatgtttttt ttttatattg    2280
tagatttttt tttttattta gatttttagt tatatttttt tttaaagttg ggtattattt    2340
ttttgaagag ataaagtata atttatatag ttagttttta tggatggaaa ttgtttttta    2400
tttttaaatt attatagggt tgggtatggt ggtttatgtt tgtaatttta gtattttggg    2460
aggttgaggt agatagatta tttgagatta ggagtttaag attaatttgg ttaatatggt    2520
aaaattttat ttttattaaa aatataaaaa ttatttgggt atggtggtgg gtgtttgtaa    2580
ttttagttat ttgggaggtt gaggtatgag aattatttga atttgggagg tagagattgt    2640
agtgagttga gattatgtta ttgtatttta gtttgggtga tagagtgaga ttttgttaaa    2700
aataaaataa aataaataaa taaaattatt atagattagt ggtattttta gaggatggaa    2760
gtttgtattt tgggatttta ttttaggtta tttttagttg ttagttttgt tgggggtgta    2820
ggtgtgggta gtgttatggt atgtggagat gaggtagatg aggttaattt tagttattat    2880
tttagttgtg ggtttttttg agtttgaaat tttttttttt attgttagtt gttttagtag    2940
ggatttttgg atgttgttta tggagttttg tttttgaagg gatagtgttg gtgggttttg    3000
tttagggaag aggattatgt gatttataaa gttgggtggt atgtttttaa gggtttttag    3060
ttatgatggt tgtatttttt ttaattgtgt tttatttatg gttttggtag ggagttttta    3120
tgatgttatt ttttggattt tttttttggt gattatttgg gtagatgtgg gagaaagggg    3180
atagttgtag ttattgtagt agaagtatag tttttatgag tttgtttatt tttggttgtt    3240
attttatttt ttttttttaa aaatagttga tgttgatgta ttagttttat ggtatagtta    3300
```

gtttttagtt tggtttatga tggttgggtt ttatgtgtgt tattttttta tgtggagaag 3360
gattattttt tggtgtaatt tgtttttttt gttttattat ggttttgttt tatagagttt 3420
tgggtgtttg gattttatta gtagaggatt agattttttt ttttggtggt tttttgttaa 3480
gtggattagt gtattagttt atttttatat tgttgattaa gatataattg agattgagta 3540
atttataaaa gaaagaggtt tgatggattt atggttttat gtggttgggg aggttttata 3600
attatggtgg aatgtgaaag gtatttttta tatggtggtg gtaagagaag aatgagaatt 3660
aagtgaaagg ggtttttttt tataaaatta ttagatttta tgagatttat ttattattat 3720
gagaatagta taggagaaat tgtttataat ttaattattt tttattgggt ttttttttata 3780
atatatagga attatgggag tataatttaa gatgagattt gggtggggat atagtttaat 3840
tatattattt tgtttttggt tttttttaaa ttttatgatt ttatatttta aaattaatta 3900
tgttttttta atagtttttt aaaattttaa tttattttag tattaattta aaagtttata 3960
tatagtttaa agttttattt gagataaagt aagttttttt tgtttatgag tttgtaaaat 4020
taaaagtaag ttagttattt tttagatata atgtgggtat aggtattggg taaatatagt 4080
tgttttaaat gggtgaaatt ggttaaaata aaggggttat aggttttatg taagttagaa 4140
atttagtagg gtagttaaat tttaaagttt taaaatgatt tttttgattt tatgttttat 4200
atttagatta tgttgatgtt aagatgtggg tttttatggt tttgggtagt tttatttttg 4260
tggttttgta gggtattgtt tttttttttgg ttgtttttat gggttggtat tgagtgtttg 4320
taggtttttt aggtaaatag tgtaagttgt tagtggattt attattttgg ggtttggagg 4380
atagtggttt tttttttgata gttttaatag gtaatgtttt agtagggatt ttgtgtgggg 4440
tttttgattt tatatttttt ttttgtattg ttttagtaga gggtttttat gagggttttg 4500
tttttgtagt aaatttttat ttgagtattt aggtgttttt atatattttt tgaaatttag 4560
gtggaggttt ttaaatttta atttttgatt tttgtgtatt tgtaggttta atattatatg 4620
gaagttgtta aggtttgggg tttttattat ggtttgagta ttgtattggt ttttttttagt 4680
tatggatgga gtagttggga tttagggtat tgggtattaa gtttttttggt tgtatatagt 4740
atggggattt tggatttagt ttgtgaaatt attttttttt tttaggtttt tgggtttgtg 4800
atgggagggg ttgttgtgaa gatttttgat atgttttgta gatatttttt ttatggtttt 4860
ggggattaat atttagtttt ttgttattta tgtaaattta ggtagttagt ttgaattttt 4920
ttttagaaaa tgggattttt ttttttattg tattgttagg ttgtaaattt tttaaatttt 4980
tatgtttttt tttttttata aaattgaatg tttttaatag tatttatgtt attttttgag 5040
tgttttgttg tttagaaatt tttttttgtga gatattttaa attatttttt ttaagtttaa 5100
agttttaaaa atttttaggg taggggtaaa atgttagtag ttttttttgtt aaaagataat 5160
aagagttatt tttgttttag tttttaataa gttttttgtt tttatttgag attattttag 5220
tttggatttt attatttata ttattttttag tattttgggt aaagttattt aataagtttt 5280
taggaagttt taaatttttt tatatttttt tattttttga gtttttttaaa ttttttttaat 5340
ttttatttgt tattggtttt aaagttatgt ttattttttt gggtattttt tttagtagtg 5400
ttttattttt ttggtattaa tttattttat tagtttgttt ttatattgtt gataaagata 5460
tatttgagat tgggtaattt ataaaagaaa gaggtttaat ggatttatag ttttatgtgg 5520
ttggggaggt tttataatta tggtagaagg tgaaaggtat tttttatatg gtggtagtaa 5580
gagaagaatg agaattaagt gaaatgggtt ttttttttata aaattattag gtttgtgaga 5640
tatatttatt attataagag tagtatggga gaaattgttt ttatgagtta attgtttttt 5700
attggttttt tttataatat atgggaatta tgggagtata atttaagatg agatttgggt 5760
ggggatatag agttaaatta tattaattag gtatattttg tatgtaagag aaagttttta 5820
ttaggttatg ttttattttt taaagtttta ttgttttttt agtttatttt tatttggttt 5880
tgtgagaatg gtttttatat ttaaaagggt aagtgagttt tgttttttttg attggttaag 5940
aaggtatttt ttagtttttt ttagttgatg tatatatgtg tttttgtgga ggaagtatag 6000
atgttatttt gttggattta ttgggaggtt ggggtgttag tttgttttaa tttgttttgg 6060
tttttttatt ttgatttatt gaaagggagg agtaggtgtg agttttttat tagttaagga 6120
ggttgtaggt agggttattg tagaggttat ttgggtgtag gggagtaggg tgaggttttt 6180
ttgttttttt atattttagt ttttaggatt ttgaggaggg aagtggaagt gagtggttat 6240
tgttataatt tttttatggt attgtttttg tgtttttagg ttaatttggt ttatgtttat 6300
gagattaagg atgtattgtg tttggttttg attattatga atgggggtga tttgaagttt 6360
tatatttata atatgggtaa ttttggtttt gaggaggagt gggttttgtt ttatgtggta 6420
gagatttttt gtggtttaga agatttttat tgtgagaata ttgtttattg gtgagtggaa 6480
ggtattaagg atttttaagt ttaagtttga gggggtgggg ttagtttttt tatatttttg 6540
ggtaggtggt tgttattgtt ttgggaatta gttttggtta gtattagggg aggatgtaga 6600
gttagataga tttttttattt taggaagttg gggtttttgg tattgaggaa gtgaggtggg 6660
ttattttttg gttagtgttt tttagttttt aaagtttagg gaagttaagt aagtgaaatg 6720
atgtttttga aattgattgt tttttttaagt taaggaggat gggttgtgat tattgttttt 6780
tttagttgtt tttattttag tagttaggga agttgggagg gggtttggtg gttgttattt 6840
gggattagtt ttgggaagat ttgggttggg ttattgttta taagtagaat tggagtggtt 6900
tatggtttta ttttatgttg tttttagttt gttttggttt ttattttttt tttttattta 6960

ttttaatttt atagggattt ggatattgta gggttagggg tttagatttt atttataatg 7020
agtagtgttt tttagatttt ttttttagtt tttttgaaag aattttgaaa aatttttgtg 7080
atgttgtatt ttaagtagat atttgaaata ttttattata ttgaaatagt ggtaattttt 7140
gatatattga aattgaatag ttatattata ttttttaggg gatttagtgg aatttttagt 7200
tgttgtagtt aattgatttt tatttgtatt tatttaaaaa atgtatagaa agttttttag 7260
tgatgaggaa tttttttatta ttttttttat ttttttttatt tttttagaat gttaataata 7320
tattttttttg tttgaaagtt ttttattgat tagtgtatat ttttgaaata aatatataga 7380
tatatataag agtttttgaa gtttttttttg attgtaagtt tttaagtgtt aaaataattt 7440
ttttggttgg ttgtggtggt ttatatttgt aattttagta ttttgggagg ttggggtagg 7500
tggattattt gaggttagtg gtttgagatt agtttggtta atgtggtgaa attttgtttt 7560
tattaaaaaat ataaaaagta gttggatgtg gtggtatgtg tttgtagttt tagatatttg 7620
ggaggttgag gttaaagaat tgttttaatt tggaggttgt agtgagttaa gattattatt 7680
gtattttagt ttgggtaata gagtgagatt ttgttttaaa ataaaataaa ataatttttt 7740
ttgttgagtt gttattgtaa ttagtagtag attttatttt taattagttt tttggaagta 7800
ggggttttga tatttggagt gggttgttat agttatattt gggaggggag gaggttgttt 7860
ttttttttgta atttgggaga agagttattg agaaagggta gatgggaggg agattttaaa 7920
agatgttagg atagagtatt ttgatgtgaa gaaatttaaa ttttttaaat tgtggatagg 7980
agggaatttt ttgtttggtt ttggtagatt ttgatatgta gtttagtttg aagattttttg 8040
tagtggagtt tattttggga ttaagaaggt tttgaaaata agaaagaatg gttttgatga 8100
tatgtttttt tttttatatt atttttttat ttatgttttt gttttgtttt tatttaaatg 8160
aaaagagtta aaattttttt taagggttga ataaagaatt ttttatgtga ttgtttttta 8220
tttaaaaaga aaaaagaaag ttttttaagt tttagtttgt ttttttttgg gggaaagaga 8280
attatagtgt tttttttttg gttgtgatta tttattatgg ttttttttttg ggttttgttt 8340
tggttagtgt ttattagtga gttgttatgt tagtttggtg gtagttggtt tgttgagatg 8400
atggttgttg tttttatttg gaattgttaa tatattaaaa gaatgttatt taaataatag 8460
taataaaagt attttatgat aaataaggat atataaatat taattattat ttgatgaggt 8520
ttattggttg gatttgggta tattaattat tttgtatgag gatagttgtt attagttaaa 8580
taaataaagt tgggtggttg agaattggag tttttattta gattttgtgt attgtttgtt 8640
ttatggttta attgagttgt atatattttt tttttggttg ttttttttatt tatagagaag 8700
attttttttt ataaaagtag ttttttttgtt ggtttttttt ttattttggg aatgtagagg 8760
ttttttttatt tgtgagtaag atttgttat taagagagga gatattaggg ataggataag 8820
gtagttatat ttatgttatg ttttgtagtt gtttttttttt ttaggttgta gtgtagttgg 8880
tgttgggggga tatgttagtt ttttgtaggt tttattttaa agggggtttt ttttttgtgt 8940
ttttattttt ttttttttga tttttttttt ttaattgtat agttttttttt attttaggtt 9000
ttgttttgtt tttagggttt tttgagattt gtagggtttt tttagttttg gagtattttt 9060
agatttgtag ttattgatgt gtgaataggg gttatgattg gtttttttttt aattttttta 9120
gttatttttt aggattggga aatgtttgtg gtaaatggta gtgttgtttt aaattattgg 9180
aaggagaaaa attagttgaa gattattttg atttaaatta gatagtttga gttaggaggt 9240
gggaaggaaa tatattgtgg ttatttaggt ttgattagtt tgaggggttg atttagatgt 9300
tagtgtattt tatttgttta gaatgtttat attttgatgt ttgttgtttt ttttttttaga 9360
gatttgaaat ttgaaaatat tttgttagat gattatggta agttttttttt tatttggtag 9420
ttgaggtgag tattgtaatt ttaagaaagt aaagggtttt ttagggattt ttagagaggg 9480
ttggggaagt tgattatggg ttttattttt attgtttagt gttgggtatt tggggaaggg 9540
ggaaagttgt gagtgtgaat agagttttgt ttggtgaata ggtagtgttt agagagattt 9600
tgaaatttag agtttttgaa atttttttttg attataaggt tttaagtgtt aaaataattt 9660
ttggttgggt gtggttttta tagtgtattg aataaggagt ttagaatttt agagatgttg 9720
ggtaaatatt taattttttat taagtttttt atttttttatt ttgtttgtaa aatggaaata 9780
aagtgttttg ttttgttttg tttatttaga aggaagaggg tggtggagag gtttttatat 9840
gatagtttga gagggaattg tttgttagtt tagttagttg taaatgtttt aggtattatg 9900
tttgtgtatt attattatta tttttttttg gttgtggata ttgaataata agaataatta 9960
tagtattttt tatagttgtt tagttttata tttggaaaat agttttttata ttttttttttt 10020
tttttttttga gatagagttt tatttgttta ggttagagtg taatggtgtg attttggttt 10080
attgtaattt ttgtttttta ggtttaagtg atttttttgt tttagttttt taagtagttg 10140
ggattatagg tgtatgttat tatatttggt tagtttttgt attttttttt ttttttgaga 10200
tgtagtttta tttttgttgt ttaggttgga gtgtagtggt gtaattttaa tttagtgtaa 10260
tttttatttt ttaagtttaa gtgattttgt tgttttagtt ttttaagtag ttgagattat 10320
aggtatgtgt tattatgttt ggttaatttt tgtatttttg gtagaaatgg ggttttgtta 10380
tgttgattag gttggtttta gatttttgat tttaagtgat ttgttttttt tggttttttt 10440
atgtgttggg attataggta tgagttattg gtttttatat ttttgatttg attagatttt 10500
tttatttgta tagagaaggg taaggtagta tttttgtgtt tatttaatag atgataaaat 10560
tgagttttag gggagttatt ttgttaaagt tatttagtag tattagtggg gagttgaggg 10620

```
tagaaattgg gttttttttt attaggtttt agaatttggt agtttttagt agagggaggg   10680
gagaaattgg gttgttgatg atttgaagag aggtttgtat gggatattgt tttgtggagg   10740
ggtggttgga gggtttaggt ttgttgtttt tttttggatg tgtttggtgt tttttttggt   10800
ttaagaatta agtttatgtt gtttttttgt tattttgatt gttagttttt attgagttag   10860
ggagtttata tggttttagt ttggtggggt tttgatattg gtttttgttg atatttaggt   10920
tggagggatg ggggtggggt tttggtgttt ataggtaggg aagggaaggg gatgtgtttt   10980
tttttgtggg gtaagttgtg gttgtgattt tgtttaggtg ggttgtggga gggataaaga   11040
ttaaagtgga gttttagttt agtgtttttt gttttggggg tttagttttg ggttgtgagt   11100
tgggatttat ttttaggttt tgttaggtta gattagatgt tttttttttt tgtttttttta   11160
aagtgaaaaa taattaattt ttagagatta ataaggtagg ttggggagtt gtttttttaaa   11220
ttgttttata attttttttt tttaagtaaa ttgttgtata atgttagaag aattttatat   11280
tttttttatg aagtaatttt agaattaagt taagattttt tgattttagt gttttaggaa   11340
gtttatttag tttttgatta ggtaggttgg ttaatgttta gttgggttta ggtattttat   11400
tgtattttta tgttttattt ttgaagagtg ggtatttttt tgtgtttttt tatatttgta   11460
tttttttaag gtttttgtgg ttattttgag atggtagatt ggggttttttg ttgtttttgg   11520
atagatgaga atgttgagag tttgtatgtt ttgtttaagg gtatatagta aggttgggtg   11580
tttatgtggt tgttttaggg atttattgat tttgttgttt tttttaggtt atattaggat   11640
tttagatttg ggtttggttg tgaagatttt tgagggagat ttgatttgtg gttgggtggg   11700
tattgttggt tatatgggtg agtgttgggt tgtttgtgtt aatgtatttt gagatttatt   11760
atttgtttat tgttggttga gtttttagag tttgtttgta gagtttttttg tttttttatg   11820
tattgttgat tatatataga gttatagttt ttgtagggtt gttggggggt ggttttagtt   11880
agggggaggg ggagtatagg ggttttagat gtgatgattg ttttgagttt tttgtatttt   11940
taggttttta gttttatttg tgtttaaagt taagttattt ttaggggttg gggtttggta   12000
tatagtgttg ttaggggagg ttatattgaa gtaggggttt gggaggagag gtttttgaga   12060
ggaaggaggt aatttataag agtttgtaaa tgagaggtgt gtatagttgt tgtgttatta   12120
agtatgattt tagtagagag tagttttggg tttttgttat tttttttttag tttgttttga   12180
gattagtgtt ttgggggttg tagggaagag atttagagtt aagttttgtg ttttagaatt   12240
ttttgtttaa ttgggggtat gttttgggtt ttttttttgtg tattttatat atttattttt   12300
ttttttttga gaggtgtgtg tttggggagt aggtatgttg ttgttttttta gttaggttgg   12360
tattgatagt agttgtatag gaggtgtgtt ttgtttttgg tgttagaggg ttttttttttag   12420
attgtatgat atttggtaat ttgtggagtg tgtgtttgtg tgtatgtgag tgtgtgtgtg   12480
tttatatgaa tgttgattga tgattgatag gtaggtgggt gggtgggtgg atggatggtt   12540
ggatggatga tggatgatgg atgggtggat gatagatttg tttatttgtg tgtgtatttg   12600
tttgggtttt tttttgtaga gtgttttatg tatttgtttg ttttggtttt tgtgtgtaaa   12660
ttagtatatg tgtggatgta tttttttttta gatatatgtg ttgatgttgt agaattattt   12720
tttttgtatt tttatggtaa atttgtaatt tggtttatta aagtggtata gaagatatag   12780
taagagattg tttgaattat agataaattt aagattattt ttaatttggg tagatatatt   12840
tttatttatt gaaataaata tattgatagt atttgttttg tgttaggtgt tatattgtta   12900
tggtagtgaa taaagtagtt tatgttttag gtgaggggat attaagagtg taggatattt   12960
ttatttatgg tggttagagt tttaaagtta tagttaaagt tttttggggg tatagaggag   13020
agttattagg gtaagttttt tggagggggt ggtatttata gtagttggtg gggtaagagt   13080
tggtggtggt tatgaaattt taggtttttt gtgagtagtg ttattagttg tttttttttt   13140
ttttattttt tttttttttt ttttgttttt agttttagag gttttgaata attagaggta   13200
tggtttgagt tttgattatt ggggttttgg ttgttttatt tatgagatga ttgagggtta   13260
gttgttgttt tgtggttgta aggagaaggt gaagtgggag gaggtggatt gttgggtttt   13320
ggagatggag gaggtgtatt tttataagtt ttttgaggag gttaagttta tttgtaagat   13380
ggtgagtttt tggtggttag atgttatttt taagttggtg gtttttttttt ttgggtttgg   13440
ttttagtttg tttttagaat agtaaatagg ttgaagggat aggggtttga gtatgggggt   13500
gggggttgta gtttattaag ttagagttga gggtattttt tgttgtggat tggggtggtt   13560
agtggagatt tttaggagaa agtttgggtg gggtagggat atttaggaat taagggaaga   13620
ggggtgaggg gaatagtgtg gaatattttt atatttagtt tgtggggggg tttatatagg   13680
gtggagaaga gatggtttga gttggtgttt tagatataaa gtaattttgg gttaagtttt   13740
ggtttttgtt tgtgggtttt ttgttttttt tatttttgga ttttaaggtg ttggtttaag   13800
atgaagggtt ataggggggtg ggggtgatga ttttagtttt tgaatatttt ataataaagt   13860
tttttattat tagatggatt gttggttatg ttagtttggt ttttgtatga tagggtttag   13920
ggatagtgag gttagattta tttaaaagtt gggtgggtgg ggttagatgg ggggattgga   13980
agttagataa ggagtgatag gagttattgg aagtggggga tatgggagtg gagtttgttt   14040
gtgtattttt gttattgggt tgttttatgt tggtttttag gttttttttt gggaattagg   14100
attagtggtt ggttttgtgg ggagatggtt atttttttgtt gttgtttggt ttttagtttt   14160
taagtttagt ttatgttttt ttgggggtttt tttttttatg gtttttagta tagttgtgtt   14220
ggtattttta ttgtaagtag ttgtttttta atgttagttt tatagggata ggaggggttg   14280
```

```
gagtatttag ttttataatt gttagtttat agattttttt tttttttttt ggagttatag   14340
atgtattttg tattttatgt atttggagtt atagatgggt aaattgagtt tgggaggggg   14400
gagttatatt ttttattttt ttatatttgt aggtattttt ggggtagatt tagtgtgtgt   14460
attgataatt taggtaattt ttggagttta ggaagtagta gttttgggag ttgattaaaa   14520
gttgtaatgt ttttgtttag gaagatttga gatttgtgtt gtaagtggtt ttaggagggt   14580
ttggtttggt tttttatttt agaggttttt tttttgtagt tagatatttt tgtttggttt   14640
ttttttgtta ttaattttat ttttatatta tagagttgtg ggaagaagtt ttgtgtgtat   14700
agtagattta tattttaatt ttatagttgt tggtttgtta tgtagttggg gttttagaga   14760
aagttatatg gttattgtag ttagggttag ggtgggggga ttggaggagt agtgtagtat   14820
tggttgggta aatggaaatg tgggtgtgag ttatatttgg ttgttttatt gggagatatt   14880
tgttaggtgt gataaaatga gataagggaa agaagaaggg gttagatagt tgtgggtaga   14940
gtattattaa atgttttagg ttagggttag gtaattagag aagtaggttt tgagagattt   15000
tagtagggat atgggatgta ttaagaggat gatgaggtgg agagggtttt agttttattt   15060
ttagatttta gatatttatt ttggtttttg gttttggagt tgggattttt tgttgtagtt   15120
tggattaggg tgttgttgtt tttttgttgt tttttggtgg atattgagag aaagtttgtt   15180
gtttttggag gatttgtttt gtgggtataa aattattgtt ttttggaagt ttttggtttt   15240
gttgtggttt tttttataaa tgatttttgg ttttgggagt ttagttttag gtttttttta   15300
tgtttttgat tgtagtattt ttggtattta ggtttatttt gtttttttta gttggtggta   15360
gtttaggata ttgtagagtt tgatttgtta ggagattttg taggttttgg gtttataggg   15420
ttatttttag gttttggtgg ttttagtttt gtttgtttaa gagggttttt ttataattag   15480
tgaggaagtt agtttttggt ttttaggttt gtttttttatt ttattttagt agttgtagaa   15540
tttatttttt tttttttgga gtttagaaat ttatatattt tattattttg ttttttgagt   15600
ttagtttatg ggtttagatt ttagttttta ttttttttaa attttttgttt agttttagta   15660
ttgggtttta gttttttttt agtttttttt tgtttttttag ttgttttggt tttatttgtt   15720
tttttagttt atttgtattt tttttttagg aagtttgttt tgtttgtttt atttatttta   15780
gtttatattt tttttttttt ttattttttt tggtgtttat ttgatagtat gattgtaaat   15840
tttttatttg atgatgtggt tgtaaattgt tgattggatg ttagggtttt aaatggtagt   15900
atataggtgg gtgtttaatt tgtatatggt tttttaaaag aagtaaatgg taggtttatt   15960
gttaagattt agaaatgagg agattttatg taataatttg gatttttttgg tttttttttga   16020
aaagtagaag ttttaggttt tggttgttgt tttatttggt aggagttgat atatagtttg   16080
gtttggttta tttagtttat tttttgtttt ttttattttt gtttgtgtta ttttttttggt   16140
tagttgttgt agtttttgta gaaggagatt tgtttttgag ggattgttta gaatagtgta   16200
gtatttgggt ttagtttaat ttagtgtttt gagtttttgt tgaatagtga agtttttaga   16260
gagagaagta gaggtatgag tgttagattt tgagttgtta gtttggtggt ttgtggggggt   16320
gttaaggggt aatattgagt gattgagtgt tttgttttgt tttttttaagg gtggaatttg   16380
gtgttggttt tgatgttatt tttgattttt ttgtttgggg ggtagttttt tttagatttt   16440
gagttttaag aggttagttt ggttgtttat ttttttttggt gtttttagtt ggtgtagtgt   16500
ttgttttata aggattagta ggaagtgtat atgggatgaa tatgagtaaa gtgtataggg   16560
tattgggaaa agataaggtt gggtttttat tttaagtagt ttgttttttt ggattttagt   16620
tttaaagtag atatttagta gatattaatt gtgtatagtt gtagtgttta gtaaggtaga   16680
ggagaagaga gaattatgag gttggggtat tgggagtgta ggttaggggt tggggttttg   16740
tggttgtgtg gttggttatg gttagttaag gtttttttgtg taggtttagt tttttggttt   16800
tatttatagt tttattttgt tttatttggg tgtttgtttt taaagatttt tttttttttta   16860
gtaatttttt tgttttgtgg ttttgatgaa gatatttttt tattttaatt atttttggtt   16920
tttttattt ttagatgaaa atttgttat ttgatttggt ttttaggatt ttttattatt   16980
ttttttgat tagttttttt tttttgattt atttttgtat tttttttttg tttttaagt   17040
taaatgtgtt ttgggtgttt tttagattta ttttgtatgt tttgttattt tgttttgttg   17100
gtgtagtttt ttttatttgt atattttttt ttttatttat attttgaaag tttatttgtt   17160
ttgtaagatt taatttaaat attttttttt tgtgtagttt tttgattttt agttggtagt   17220
tagttatgtt ttttgttttt ggggtgtttg atttgtatta ttttgatgtg tttttttgagt   17280
atgattagtt ttttatgagt ttgagtattt ttagaggtta agtgttttgg tggtgttttt   17340

tttgttagga aggggtttgt atttaggagg tataattttg taaattagtt gagtatgtgt   17400
atgagataga gttattgatg tagtttttgt tttttgttgt ttattttata tagaagaggt   17460
gatagtgatt tgatatttat taatgagtgt ttattataat ttaggaatga ggtttgtaat   17520
tttgagagaa ataagatttt tgttttttttg ttttattagg tatttagtta ggttgtgagt   17580
gtgtgagtat agatgtatgt taagatttag aggtagtttt gaagtaagat ttttgtaggg   17640
tggggtagtg tgttttgggt ttttaggtat ggtgtagata ttgtggaggt aggaggtttg   17700
gataggagga gaggtttagg ggatgtggtt ttatggggtt ttttgtttta gttgtttatg   17760
aaagatgtga agtagaggtt gggttgttag gaggaggggg ttgtagaggt taagagatat   17820
ttttttttta ggaatatgaa ttttaagtgt ttagaagttg ggatgttgga tttttttttt   17880
```

gttttagatg tgagtagttt tttttagttt ttagtagttt tttttatagg gtatttaggg 17940
ttgtttttga gtgttgtttt atagtgagtt ggtgtagagt gtggggtata ttgtgttttt 18000
gtggggatta gggttttttt ttggttattt tttatttaag ttgtagatga attttatgtt 18060
aggtagtgta gaagaggtgt gggttaggga gtttatattg gttgtttaga gatttgggta 18120
tttttttagg tttggggttt ttggatagat ttttttaag tgttagtttt tttttgttt 18180
agtgaaatta gtaatattta tgttttttat ttgtttggga gatttgaggg tgaaatgaga 18240
attgaatggg gaatgttttt aagagtattg atggttatta tatttagttt attttgatta 18300
gtatgtatgt attgagtatt attgttgatg ttaggttttg ggttaggtag aaatataaga 18360
ggtttttttt ttagtagttt atattgtagg gagttgtatt tttttaggag gtttttaggt 18420
tttttagttg gtgtgtgagg taagatttta gttgaggtag aattgttttt gaaagttttt 18480
tatatggttt ataaattatg agtttttagt tggattagag gtttaagaga tgtagattag 18540
gtagaggttg ggtgatttgt gtgggatatg tgttgtgatt ttggggtttg ttttgtttaa 18600
agttagggag ttagaagtta ggaaggtttg ttgtgttttg gtttttgtta tgtttttgtg 18660
gatgattttt tgttggtagg gggattgggt tggggattat ggaggttgtg ggagtagtag 18720
tgtgttgtag gtgaatgatt gtatttttag agagtttatg tgtatttttt tttttaattt 18780
tgtgtttaat ggttttgtgt tggtagttta tagtagattt ggtggtagta tttgtatagt 18840
agtaatttgt aaatgttata aattagggtt tttgtgtttt ttttttttag aattagtggt 18900
tattatttat tagtattgtt ttgagtggag gtaggtgggg attgtttttg gaattttggt 18960
atttttttata tatggggatt ttttggtttt ttgggataga aggtggtttg ggaagttgtt 19020
aggtgtggat tagtgtttat ggtaattata ttatagattt tgtttggtta gggatggtat 19080
tagtgtgatg ggtaattagg gaatgttttt tggagaaata tgggggttat tgagaatata 19140
aagagtaggt tgggtatggt ggtttatgtt tataatttta gtatttggga aggttgaggt 19200
agttgggtta ggagtttgag attagtttga ttaatgtggt gaaattttat ttttattaaa 19260
atataaaatt aattaggttt gggggtatat atttataatt ttagttattt gggaggttga 19320
ggtaggagaa ttatttgaat ttaggaggta gaggttgtag tgagttgaga ttgtattatt 19380
gtattttagt ttgggtgatg gagtgagatt gtattttaaa aaataaaaag tgaaagttat 19440
tgaatgttgt aaatttaatt tgtagatgag tttgagtagg tagggggagg atggtaatta 19500
tagtattgtt tttgattttg gttgtttggt gaattgtttg gggagtatga ataatatttg 19560
ttttggatgt agtttttagg gagtttgggg taagatttgg gttttaggag ttttgaaatt 19620
tttttagttg attttagtgt agagttaggg ttgagagtta ttattatggg aagtgttata 19680
tgtaaggagt gtgttgtttg ttgtttgttt ttagtgttaa gggttttggg agtggttatt 19740
ttttggtgtt ttaggaaatt atatagtgtt gagtgtatga atattatggt tgtgttattg 19800
ggttgatgtt tgaaattgga gaggatgtgg gtatttagat tgggggtgtg gtaaaggttt 19860
ggaatttggg tggttatttt tgtggttttt gggttattta ttgttatggt gatgggttga 19920
gattagttat tgtattggag ttattttttt atttttttagt atattttagg ttttgtgtta 19980
ggttttaagg gagattttat ggtatataaa atagttggtt gtttttagtt tgaagaggag 20040
taggggtgtt agtaaaattt tttaaataga agtagaaata tgagttataa gagaagagaa 20100
taggtagttt ttgggaagat gggagagtag tggggttttt gtgtgtgtat ttaggtatgg 20160
ttggagaaaa gtgttttagg ttttggaagt agtatgtgga aggaggtggt tttgagggtt 20220
agagtgatta attgttttgt tttgtttggg attgttttgt ttttagtatt gaaagttttg 20280
tgttttgggg agatagggat agttggttat tttgggagtg attttggtta ttttgaatta 20340
gggtattagg aggatgaggt agtagagttt tgtaaagtgt atagtttgat aaggtgtata 20400
gagtagttta ttggtgtttt tttgttttag ttgtgtattt tgtggtttag ggtttttgtt 20460
tttggtattt gaggttttag gttagtaaag gttgtaggtt ttggagtgtg aagttgttgt 20520
gagttgggga ttttgttttt tttttattgg tttttttttag gttgtatgtt aatttttagt 20580
tatttttgtt agttttttat agttattttg ttttttatag ttttttttgt tagtttttta 20640
tagttttttt tgttagtttt tttagttgtt tttgttagtt ttttatagtt atttttgtta 20700
gtttttttatt attgtttttg ttagtttttt attgttgttt ttgttagttt tttatagtta 20760
tttatgttag ttttttatag ttattttatt tttatattat tgttggggtt gttgttatat 20820
ttttaaatta tgagtattgt gagtgatatt atttttttta tttttattga tttttttttt 20880
aaaataaaaa ttaatgtatt ttaaaaggaa atttttttaa ggaaataata gttatgaaag 20940
aggggtttgg gtgtgttagg taatttttttg ttttattata gatttttaaa atatgtttat 21000
tttttaatat ttttttgtaa gttatttagg gttagttttg tgagtttgat ttggtatttt 21060
ttagggtatt ttgggttttt atatttggtt tagtgttttt tgttgttatt ttaaaatttt 21120
aaatgtggaa tatggatttt gtgtttttat tttgtgttga gtatgtgaat gatgtggttt 21180
tgtttttagt taaaagtttg ttgagtttta tggtggtgtg tattttgggt tttgggaatt 21240
agagaagggt tgtagttttg taattggata tttttgtttg tatttttgtt tttgtttatg 21300
tgtatttggg tttatggttt gttttgttgg ggatagtatg gttttgagtt ttgagttgga 21360
gattagagta gattggaggg gagagtttta gaaagtgttg agttttgaga ttattgttag 21420
aatttttttg gtatggtgga gtttatgttg agttggtgta ttgatttata atttttgtat 21480
tagttaaatg ttaggagatt ataagtagga ggtagttttt atttttttagt gatagatgtg 21540

```
ttattaggtt tggggatttt tttatatttt atttggggtt gtttttataa aggttttatt   21600
tttagaggtt ttgggaagtt tttttatatt aattttgaat ttttagaggg tagggtttat   21660
ttaggtttat ttttgttttg gtatagagta ggtttttagt gtatagttga gggatgagta   21720
aggtaaaaga tatgtttatt ataataatat tagtttttag ttatgggtgt taggtttggt   21780
ggttagtatt gatagtagtt ttattgttta ggtgttttgt tgtttttatt ttgttggtaa   21840
ggaaatagga tataggtttt aggggatatg agattttttt aaagttttta agttttatag   21900
ttgggatttt agtttatgtt tgtttagtat gggtaaggga gtgatggtag gaatgagttt   21960
tgggttgggt atttaggtgt tttgagtttt tgtgttattt tagttagggt ttttgttatg   22020
gatttgagag agggttgtat tgttatttgg aggggttgta taaaaaaatt ataggttatt   22080
atttgaggtg gataggaagt ttagttaagt tattggggtt gatttttttt ttggatttat   22140
ttgtatttga gttatatatt ttttagttat tatttatagt agttttgggg gtgtgttttg   22200
ggaagatttt tttttgtttt ttaaaatttt aaggtttggt ttggggttat tggagttgta   22260
ggtgggatat atgtgtgatt ggtttttgt ttttggtagt tttgtgttgt gtattgtaag   22320
gatgtgttgg atattgagta gtttttattt gtgaagggtg ttaatttgga ttatatagat   22380
gatgattttt attttaagtt ttttatgggt tttgtgttta ttttatggta aaatgaggtg   22440
agtagggtag attatttgtt ttggtttggg tgggagggag ggattgatgg gtggaaggag   22500
gtgttgggaa tatgagtttg gtggtaggag gttgagtgta tggtttttgt tttttttatg   22560
aaggtagtat ataaaagttg ttagtggtta agtagggagt tgtagttatt atgggttagt   22620
tattgatggg gagttttgtt tagtttttga gatttggagg gtgtgtggtt taggggtagg   22680
tgaggttagg gg                                                       22692
```

<210> 236
<211> 22692
<212> DNA
<213> Artificial Sequence

<220>
<223> chemically treated genomic DNA (Homo sapiens)

<400> 236

```
tttttggttt tatttgtttt tgagttatat gtttttttagg ttttaggggt tgggtgaggt      60
tttttgttaa tgattggttt gtggtggtta tagtttttta tttggttatt gatagttttt     120
gtgtgttgtt tttatggaga aaatagaaat tatgtgttta gtttttttgtt gttaaattta     180
tatttttgat gttttttttt atttgttagt tttttttttt tatttagatt aaagtaagtg     240
gtttgttttg tttattttgt tttgttatgg gatggatata gagtttgtgg agaatttgga     300
gtagaagttg ttgtttgtgt ggtttagatt gatgtttttt atagtggaga attgtttgat     360
gtttagtatg tttttatagt atatagtgtg gggttgttag gggtagaggg ttggttatat     420
atatgttttg tttgtggttt tagtggtttt gagttaggtt ttggggtttt ggggagtaga     480
agggagtttt tttaggatat atttttagaa ttgttatggg tagtggttgg agggtgtgtg     540
gtttagatgt aggtgagttt aggggagggg ttggttttag tggtttggtt gggttttttg     600
tttattttag atgatggttt gtggtttttt tgtgtgattt ttttagatga tagtgtagtt     660
ttttttaga tttatgatgg ggattttggt tggggtgata taggagtttg gggtatttgg     720
gtgtttagtt tagggtttat ttttgttatt atttttttat ttatattgga tagatgtggg     780
ttagagtttt agttgtgggg tttaggaatt ttggggaagt tttatatttt ttgaggttta     840
tgttttgttt ttttattggt aaaatgggga taatgaggta tttgggtgat ggggttgttg     900
ttagtgttgg ttattgagtt tggtgtttgt aattgggagt tggtattatt atggtaggta     960
tattttttgt tttatttgtt ttttagttgt gtattggggg tttgttttat gttagggtag    1020
agatgaattt gggtggattt tgttttttaa gagtttaagg ttggtgtggg gaggtttttt    1080
agagttttttg gagatgaggt ttttatagag atagttttag gtggggtgtg aagggatttt    1140
tagatttggt gatatatttg ttattggagg gtagaggtta ttttttgttt atagtttttt    1200
gatatttggt taatatgaag gttgtgaatt agtgtattgg tttagtatgg gttttgttat    1260
gttaggaagg ttttagtaat ggttttaggg tttggtattt tttgggattt ttttttttag    1320
tttgttttgg tttttagttt aaggtttaag gttatgttgt ttttagtaaa gtaaattatg    1380
ggtttaagtg tatgtggatg gaagtagggg tgtgggtagg ggtgtttagt tgtagagttg    1440
tgattttttt ttagttttta aagtttgggg tgtatgttat tgtggaattt agtggatttt    1500
tagttggaag taagattata ttatttatat gtttagtgta gaatgaaaat gtagggttta    1560
tgttttatat ttagaatttt aagatggtaa tagagggtat tgagttaggt gtggggattt    1620
agggtgtttt agggggtgtt aggttaagtt tatgaagttg gttttgggtg gtttgtagag    1680
gaatgttaag aaatgagtgt attttaaaaa tttgtaatgg aatagaaagt tatttggtat    1740
atttaaattt tttttttatg gttattattt ttttggaaag gttttttttt aaaatatatt    1800
```

gatttttatt ttaaaaaaag agttaatgga agtgaaaaag atgatgttat ttatagtgtt 1860
tgtggtttag gaatgtggta gtagttttga tagtggtgtg gggatgggat gattgtgggg 1920
ggttggtatg gatgattgtg gggggttggt agggatgatg gtgggggggtt ggtagggatg 1980
atggtggggg gttggtaggg atgattgtgg ggggttggta gggatgattg ggggggttgg 2040
tagggaagat tgtggggggt tggtagggaa gattgtgggg ggtgggatga ttgtgggggg 2100
ttggtaggga tgattggggg ttggtgtgta gtttggggag ggttagtggg gaaaaggtaa 2160
agtttttagt ttatagtggt tttgtatttt agggtttgta atttttgttg atttgaggtt 2220
ttggatgtta ggaatagagg ttttgggtta tagggtatat ggttgaggta ggagggtatt 2280
ggtgggttgt tttgtgtatt ttgttaggtt gtgtgttttg tggggttttg ttattttatt 2340
tttttaatgt tttgatttga agtggttagg gttattttta gggtgattaa ttgtttttgt 2400
ttttttagga tataggattt ttagtgttaa aagtaggata gttttaggta aagtaggata 2460
gttggttgtt ttgattttta gagttgtttt tttttatatg ttgtttttag ggtttggagt 2520
gttttttttt agttatgttt gggtgtatgt atggaggttt tgttattttt ttattttttt 2580
ggaggttgtt tgtttttttt ttttgtgatt tgtattttta tttttattta aggggttttg 2640
ttgatatttt tgtttttttt tagattggaa ataattaatt gttttgtgtg ttatgggatt 2700
ttttttgggg tttggtatgg gatttggagt gtgttggaag gtagaggaat agttttaatg 2760
tgatgattaa ttttagtttg ttattatagt aataggtagt ttagaggtta taaggatggt 2820
tatttgggtt ttaaattttt gttatatttt tagtttggat gtttatattt tttttaattt 2880
tggatattag tttaatggta taattgtgat gtttatgtat ttaatattat gtggtttttt 2940
ggaatattaa ggagtggtta tttttaaagt ttttggtatt aaaaataaat aataaataat 3000
atattttttg tatgtggtat tttttataat agtgattttt aattttggtt ttatgttaga 3060
attagttggg gaggttttaa aatttttgaa gtttgagttt tattttagat tttttgggga 3120
ttgtatttaa ggtgagtgtt gtttatgttt tttaggtggt ttgttaggta gttagggttg 3180
agagtaatgt tgtgattatt attttttttt tgtttgttta agtttatttg tagattgagt 3240
ttgtagtatt tagtgatttt tatttttttat tttttgagat gtagttttgt tttgttgttt 3300
aggttggagt gtagtggtat aattttagtt tattgtaatt tttgtttttt gggtttaagt 3360
gatttttttg ttttagtttt ttaggtagtt gggattatag gtgtgtgttt ttaagtttgg 3420
ttaattttgt attttagtag agatggggtt ttattatgtt ggttaggttg gttttgaatt 3480
tttaatttga ttgttttagt ttttttaagt gttgggatta taggtatgag ttattgtgtt 3540
tggtttattt tttatatttt tagtggtttt tgtgtttttt taaaaagtat tttttgatta 3600
tttattatat tgatgttatt tttggttaga tggggtttgt ggtgtggttg ttatggatat 3660
tgatttgtat ttagtagttt tttaagttat tttttgtttt agaggattag ggaattttta 3720
tgtatagggg gtgttagagt tttaaggatg gtttttattt gtttttattt agggtggtat 3780
tggtaagtga taattattgg ttttgggaga gggggatgta gaggttttga tttgtagtat 3840
ttgtaaattg ttgttgtgta gatgttatta ttagatttat tgtaagttat tgatgtaaag 3900
ttattggata tggaattggg aagagaggtg tatatgagtt ttttggagat gtagttattt 3960
gtttgtagta tattgttgtt tttatggttt ttatagtttt tggtttagtt tttttgttag 4020
taaggggtta tttgtgggag tgtggtagga gttgaagtat agtaggtttt tttggttttt 4080
gattttttga ttttaaatag agtaggtttt ggaattatag tatgtatttt gtatagatta 4140
tttagttttt gtttggtttg tattttttgg gtttttgatt tagttgagag tttatgattt 4200
atagattatg taggggattt ttaaggataa ttttatttta gttggaattt tgttttatat 4260
attgattggg ggatttgggg attttttggg aagatgtaat tttttgtaat gtgagttgtt 4320
gggggagggg tttttatgt ttttgtttgg tttagaattt ggtgttaata gtagtgtttg 4380
atgtatgtat gttggttaag atgaattgaa tgtagtggtt gttaatgttt ttgaaagtat 4440
tttttattta gtttttattt tatttttaaa ttttttaggt aggtgggagg tatgagtgtt 4500
attggtttta ttggataggg aggaaattga tgtttagaag gaatttgttt aggattttta 4560
gatttgggaa agtgtttaag tttttggata gttaatgtgg gttttttggt ttatgttttt 4620
tttgtattgt ttggtgtgaa gtttatttgt agtttaggtg aggagtggtt gggaggggat 4680
tttggttttt ataaagatat gatgtgtttt atattttgta ttagtttatt gtgaggtagt 4740
atttgggggt agttttgggt attttgtgaa gggagttgtt gggggttggg ggaggttatt 4800
tatgtttgga atgaagggag ggtttaatat tttggttttt aagtgtttga agtttatgtt 4860
tttgaagaag gggtgttttt tgatttttgt agttttttt ttttggtagt ttagtttttg 4920
ttttgtattt tttgtgagta gttgaaatag agagttttat gaagttatgt tttttgggtt 4980
tttttttttg tttggatttt ttgtttttat agtgtttgta ttatgtttag gaatttaaga 5040
tatgttgttt tattttgtaa aggttttgtt ttagggttgt ttttgagttt tggtgtgtat 5100
ttgtgtttat atatttatag tttggttaga tgtttgatgg gataggagag taggaatttt 5160
gttttttttta ggattataga ttttgttttt gggttatggt gggtatttat taatgggtgt 5220
tgagttattg ttattttttt tgtgtagggt aagtaataaa aggtaaggat tgtattaata 5280
gttttgtttt gtgtatatat ttagttaatt tgtagagttg tattttttgg gtgtaagttt 5340
ttttttgata gagaaggtat tattgaggta tttggttttt aggagtgttt agatttatgg 5400
ggggttaatt atgtttagag gatgtattaa ggtgatatag attgggtatt ttgggggtag 5460

```
ggggtatgat tggttgttag ttggaggtta gggagttgta tggggaagag gtatttaagt    5520
tgagttttgt aggataaata ggtttttaag gtgtgggtag ggggaggggt atataggtag    5580
agggaattgt attagtaaag tagagtggta ggatgtgtag ggtgagtttg gggaatattt    5640
aaaatatgtt tgatttagaa agtgggagga gggtgtagag atgggttaag gggaggggt     5700
tggttaggga gagatgatag aggattttga aggttaggtt aggtggtggg atttttattt    5760
ggaggtagaa ggagttaggg atggttgagg taggagagtg tttttattag aattatgagg    5820
taggaaggtt attaggagag gaagagtttt tgggagtaga tatttaggtg agatgggatg    5880
aggttatgaa tgaggttaga gggttagatt tgtgtagggg gttttgatta gttatggtta    5940
gttatatgat tatagggttt tggtttttga tttgtatttt tagtgtttta gttttgtgat    6000
tttttttttt tttttgtttt attgagtgtt gtagttgtat gtagttggtg tttattaagt    6060
gtttgttttg aggttaaaat ttagaggagt agattgtttg gggtaggggt ttagttttgt    6120
tttttttttaa tgttttgtgt gttttgttta tatttatttt atgtatattt tttgttggtt   6180
tttgtggggt aggtattgta ttggttgggg atgttgggga gagtgagtag ttagattggt    6240
tttttggggt ttagggtttg gagggggttg ttttttaaat aaaggaattg aaggtggtat    6300
tagggttagt attaggtttt atttttgaaa ggataaagta gagtatttag ttgtttagtg    6360
ttgtttttta atgtttttat ggattattag gttggtggtt tagggtttgg tatttatgtt    6420
tttgtttttt tttttggggg ttttattgtt taataaaggt ttaaggtatt gggttgggtt    6480
gggtttaggt gttgtattgt tttaagtagt tttttagagg taagtttttt tttgtagggg    6540
ttgtagtagt tggttaggaa ggtggtataa atgaggatga gaaaggtgag gggtgggttg    6600
ggtgggttgg gttgggttat gtgttagttt ttgttaggtg ggatggtagt tagggtttgg    6660
ggtttttgtt ttttaagaga agttagaaaa tttaggttat tatatgaaat ttttttattt    6720
ttaaattttg gtaatgaatt tattatttat ttttttttgaa aaattgtgtg taggttgaat   6780
atttgtttat atgttgttat ttgaaatttt ggtatttagt taatagtttg tagttatatt    6840
gttaggtaaa gagtttgtaa ttatgttatt aggtggatat taggagaagt gagaggaaga    6900
gaaggtgtga gttgggatgg gtggggtagg tagagtgggt tttttggagg aggggtatag    6960
atgggttgga aggataagtg agattaagat ggttgaaagg taagagggag ttggaggggg    7020
gttaaggttt agtgttggga ttgagtaggg gtttggggaa gatggaggtt gaggtttggg    7080
tttatggatt gggtttagga gataaagtgg tgagatgtgt gaatttttgg gttttagagg    7140
agggggagtg ggtttttgtgg ttgttaaggt agaatggggg atagatttgg gggttagaga   7200
ttggtttttt tattgattgt ggagggattt ttttaggtag gtagggttgg agttattaga    7260
gtttagaaat agttttgtga atttaggatt tgtgaggttt tttggtaagt taggttttgt    7320
agtgttttga gttgttatta gttggagggg ataaggtagg tttagatgtt ggagatgttg    7380
tagttaaggg tgtgaaggag gtttaagatt agattttttaa gattaaagat tatttgtaag   7440
ggaagttgta gtaaggttag aggtttttag aggatagtgg ttttgtattt atgggatagg    7500
ttttttggga gtagtaggtt ttttttttagt gtttattagg gggtagtaga gggatagtag   7560
tattttggtt taggttgtag taggagattt tagttttaaa gttaggggtt aaggtgggtg    7620
tttggggttt gggggtggga ttggggtttt ttttgttttg ttattttttt agtatgtttt    7680
atatttttgt tgagattttt taagatttgt ttttttgatt gtttggtttt gatttgggat    7740
atttggtgat gttttgttta tggttgtttg attttttttt ttttttttttg ttttgttttg  7800
ttgtatttga tagatatttt ttaatgagat agttaagtat ggtttatatt tatattttta    7860
tttatttgat tagtgttgta ttgttttttt ggttttttta ttttagtttt ggttgtgatg    7920
attatgtggt ttttttttgag gttttagttg tatggtgggt taatagttgt ggggttagag   7980
tgtgggtttg ttgtatatat aggatttttt tttatagttt tgtgatgtga ggatgaggtt    8040
ggtgatagga aggggttaag tagaggtgtt tggttgtagg agaggggttt ttggggtgag    8100
gagttgagtt agattttttt ggggttgttt gtggtgtaga ttttaggttt ttttgagtag    8160
aagtattgtg atttttaatt aatttttagg attgttgttt tttaaatttt gggagttgtt    8220
tgggttgtta atgtatatgt tgaatttgtt ttaaaagtgt ttgtagatgt agaggggtgg    8280
gaagtgtggt tttttttttt taggtttggt ttgtttattt gtggttttgg gtatatggga    8340
tatggggtat atttgtggtt ttagggagag gagaggaagt ttgtgggttg gtaattgtgg    8400
agttgggtgt tttggttttt tttgttttta tgaggttggt attagagggt agttgtttgt    8460
agtgggggtg ttagtatagt tgtgttgggg attataagga ggagggtttt aagggggtat    8520
gggttggatt tgggggttga gagttaggta gtagtaggaa gtggttattt ttttataaag    8580
ttagttatta gttttggttt ttagggaagg gtttgagggt tggtatgggg tggtttagtg    8640
gtaggggtgt atgggtggat tttattttta tattttttat ttttaatggt ttttgttatt    8700
ttttatttgg tttttagttt ttttatttgg ttttatttat ttagtttttg ggtgggtttg    8760
attttattat ttttagattt tgttatgtag gagttaggtt ggtgtgatta gtagtttatt    8820
tagtggtgga aaattttgtt ataagatgtt taaagattga aattgttgtt tttattttttt   8880
gtagtttttt gttttaaatt aatattttag gatttagagg tggagggagt aggaggttta    8940
tgggtagggg ttggggtttg gtttaaggtt gttttgtgtt tggggtatta gtttagatta    9000
tttttttttt gttttgtgtg gatttttttta taggttggat gtgagggtgt tttatattgt   9060
tttttttatt tttttttttt tggtttttgg gtgtttttgt tttatttagg tttttttttg    9120
```

gaagtttttg ttgattattt tagtttatag taaggagtgt ttttaatttt agtttggtgg 9180
gttgtaattt ttatttttat gtttagattt ttgttttttt agtttgtttg ttgttttggg 9240
gatagattgg ggttaggttt agggagggga gttattagtt tgagggtggt atttggttat 9300
taggagttta ttattttgta gatggatttg gttttttttgg agaatttgtg ggagtatatt 9360
tttttttgttt ttaggatttg gtggtttatt ttttttgtt ttattttttt tttgtggttg 9420
tggaatggtg attggttttt gattatttta tagatgaggt agttaaggtt ttagtagttg 9480
gggtttaggt tgtatttttg gttgtttagg atttttggag ttgggggtag aggagaggag 9540
agaggagtga ggaggaggga gatagttggt ggtgttgttt atagaaggtt tggagtttta 9600
tggttgttat taatttttat tttattaatt gttgtgaata ttattttttt tagggagttt 9660
gttttgatga ttttttttttg tgtttttgaa ggattttgat tgtagtttta gaattttgat 9720
tattgtggat ggaggtgttt tgtattttttg atgtttttttt gtttagaata tgagttattt 9780
tatttattat tgtaataata tgatatttgg tatagaatag gtgttattag tatatttgtt 9840
ttaatgaata aaggtgtatt tatttaaatt aaagatgatt ttaaatttat ttgtaattta 9900
aataattttt tgttatgttt tttgtattat tttgataaat tgaattatag atttattata 9960
gaaatataga aaagatagtt ttgtagtatt agtatgtatg tttggaggga aatgtattta 10020
tatatgtgtt ggtttgtata taagagttgg ggtagataga tgtatggaat attttgtaaa 10080
aggagattta ggtaaatgta tatataggta ggtagattta ttatttattt atttattatt 10140
tattatttat ttaattattt atttatttgt ttatttgttt atttattaat tattgattag 10200
tatttatgtg ggtatatata tatttatata tatatagata tatattttat aggttgttaa 10260
atgttatgta atttgaaaag gattttttag tattagagat aaggtatatt ttttgtgtag 10320
ttgttgttag tattagtttg attagagggt ggtagtgtgt ttgtttttttg ggtatgtgtt 10380
ttttagggga aggagggtag gtgtgtgaaa tgtataggag gaagtttagg gtgtgttttt 10440
aattggatgg gggattttag ggtataggat ttaattttgg gttttttttt tgtaattttt 10500
aggatgttgg ttttagagta gattgaggaa aggtggtagg agtttaaggt tgttttttat 10560
tggaattatg tttggtgata tagtagttgt gtgtgttttt tatttgtggg tttttgtgaa 10620
ttattttttt ttttttaaag gttttttttt ttagattttt gttttaatgt ggtttttttt 10680
agtagtgttg tatgttaggt tttgattttt aagagtggtt tggttttgga tataggtggg 10740
gttgggggtt tgggagtgta gagagtttag agtagttatt atatttgggg tttttgtgtt 10800
tttttttttt ttggttaagg ttgtttttta gtagttttgt agagattgtg attttgtgtg 10860
tgattagtag tgtatgaaga ggtgagaggt tttgtgggta ggttttgggg gtttggttgg 10920
tggtgagtag gtggtgggtt ttaaggtgta ttgatatagg tagtttagta tttatttatg 10980
tagttaatag tgtttatttg gttgtggatt aggttttttt tggggatttt tatagttaag 11040
tttaggtttg agattttaat gtggtttgag ggggatagta gggttagtgg gtttttggga 11100
tagttgtatg ggtatttggt tttgttgtgt gtttttgggt aaggtatatg agtttttggt 11160
atttttattt gtttaaggat aatagggatt ttaatttgtt attttagagt gattataaga 11220
gttttgggag agtgtaggtg tgggaggata taggaaaatg tttatttttt aggagtggaa 11280

tataaagatg taataggatg tttggatttg gttggatgtt gattgatttg tttagttaaa 11340
ggttgaatgg gtttttttgga atgttgaggt tagagaattt tggtttggtt ttgggattat 11400
tttatgggaa aaatgtgagg tttttttggt attatgtaat aatttgttta aggggaaaaa 11460
attataaaat gatttgggaa gtagtttttt ggtttgtttt gttggttttt ggagattggt 11520
tgttttttat tttagagaaa tgagaaggga aaatatttgg tttgatttgg tagagtttgg 11580
agatgaattt tagtttatag tttaggattg agtttttaga gtgggagatg ttgagttgag 11640
gtttttatttt ggtttttgtt tttttatag tttatttggg tagggttgtg gttataattt 11700
attttatagg aaggaatatg tttttttttt ttttttgttt atggatatta gggttttatt 11760
tttatttttt tagtttagat gttagtaagg gttagtgttg agattttgtt aggttggagt 11820
tgtgtggatt ttttggtttg gtgggggttg atagttagga tagtagagga gtagtgtggg 11880
tttggttttt agattaaggg aggtattggg tatatttaga gaggggtggt aggtttgggt 11940
tttttgattg ttttttttata gggtagtatt ttatgtagat ttttttttag gttattagtg 12000
atttggtttt tttttttttt ttattggggg ttgttaggtt ttggggtttg gtgggaggag 12060
atttagtttt tgtttttagt tttttattga tgttgttggg tgattttggt aggatggttt 12120
ttttgaggtt tagttttgtt atttgttaaa tgggtataga gatgttgttt tgtttttttt 12180
tgtgtaggtg ggaagattta gttagattaa aggtgtggaa attggtggtt tatgtttgta 12240
attttagtat atagggaggt taaggagggt agattatttg aggttaggag tttgagatta 12300
gtttggttaa tatggtaaaa ttttattttt attaaagata taaaaattag ttgggtgtgg 12360
tggtatgtgt ttgtaatttt agttatttgg gaggttgagg tagtagaatt atttgaattt 12420
gggaggtaga ggttgtattg agttgagatt gtattattgt attttagttt gggtaataag 12480
agtaaaatta tattttaaaa aaaaaaaaaa atataaaaat tagttaggtg tggtggtgtg 12540
tatttgtaat tttagttatt tgggaggttg aggtaggaga attatttgaa tttgggaggt 12600
agaggttgta gtaggttgag attatgttat tgtattttag tttgagtaag tgagattttg 12660
ttttaaaaaa aaaaaaagaa ggtataggaa ttattttttta ggtgtgaggt taaatagttg 12720

tgaggggtat tgtggttgtt tttattattt agtgtttata gttaagagaa aataatgatg 12780
gtaatgtata aatgtaatgt ttgagatatt tatagttggt tagattgata agtaattttt 12840
ttttaagttg ttatgtagaa gttttttat tattttttt tttttgggtg ggtagggtag 12900
ggtaggatgt tttgttttta ttttataaat aagatgagag gtggagaatt tgatggaagt 12960
taagtatttg tttagtattt ttgaaatttt ggattttttg tttagtgtat tgtagagatt 13020
atgtttggtt agaagttatt ttaatattta gagttttatg gttaaaggga attttaaaaa 13080
ttttgaattt taaagttttt ttgaatattg tttgtttatt aagtggagtt ttgtttatat 13140
ttatagtttt tttttttttt taggtattta gtattgagta gtgggagtgg ggtttgtggt 13200
tagtttttt agtttttttt gagaattttt gggaggtttt ttgttttttt ggggttgtaa 13260
tgtttatttt agttattgag taggaaaaga tttattataa ttatttaata ggatgttttt 13320
aggttttaga tttttgaaag gaaaaataat aagtattaga gtgtgggtat tttgggtagg 13380
taggatatat tggtatttgg gttgattttt tgggttaatt aggtttgagt ggttatagtg 13440
tgtttttttt ttattttttg gtttaggttg tttggtttga gttaaaatga tttttagtta 13500
attttttttt ttttagtaat ttaaagtaat attgttgttt gttataggta ttttttaatt 13560
ttggagggtg attgggaaag ttaggaaaag gttagttatg gtttttgttt atatattaat 13620
gattgtaggt ttgaggatat tttaaggttg agaagatttt gtaggtttta ggaagttttg 13680
aaggtagaat agggtttgga atgaagggag ttgtgtaatt gggaagagag gattaaaggg 13740
gagaaggtgg ggatatagga aaaagatttt ttttgggatg gggtttgtag gagattggta 13800
tattttttaa tattagttat gttatagttt gggagaaaga atagttatgg ggtatggtat 13860
aggtgtggtt gttttatttt gtttttaatg tttttttttt tgatgatagg attttgttta 13920
taagtaaggg agtttttgta tttttaggat gaggggagag ttagtagagg agttgttttt 13980
ataggaaaag gtttttttta tgggtaaaag aatagttaag aggagagtgt gtgtaattta 14040
gttaagttat ggggtaaata gtatatagga tttggatgga ggttttagtt tttaattatt 14100
tagttttgtt tatttaattg atggtagttg tttttatgtg gagtggttaa tgtatttaaa 14160
tttagttagt gagttttatt aaatgataat tggtgtttgt gtatttttat ttattataaa 14220
atatttttgt tgttgttatt taaatggtat ttttttgatg tgttaataat tttaaataag 14280
ggtagtagtt attattttaa taagttggtt gttattaggt tagtatggta gtttattagt 14340
gggtattggt tagaatggag tttaaagaga ggttgtggtg agtggttata gttagggagg 14400
aggtattgtg gttttttttt ttttggaaga agatgagttg gaatttggag ggtttttttt 14460
ttttttttta aataaaaggt aattatgtag agaatttttt gtttaatttt tagggaaaat 14520
tttgattttt tttatttgaa tgaaaataaa atagaaatat aaatgggggg atggtatggg 14580
gggaagggta tgttattaag attatttttt tttgttttta aaattttttt ggttttaaga 14640
tgagttttat tgtaaaggtt tttaaattgg gttgtgtgtt ggggtttgtt aggattgagt 14700
aggggggttt tttttgtttg tagtttgaag ggtttggatt tttttatatt aggatgtttt 14760
gttttggtat tttttgggat ttttttttta tttgtttttt tttaataatt tttttttttaa 14820
gttatagaag aaaagtagtt tttttttttt ttgagtatga ttgtagtgat ttattttaga 14880
tgttaaaatt tttgttttta aaggattgat tagaaatgag atttattatt aattataatg 14940
gtaatttgat agaagaagtt attttatttt attttgagat ggagttttat tttgttgttt 15000
aggttggagt gtagtggtga ttttgattta ttgtaatttt taggttaaag tgatttttta 15060
gttttagttt tttaagtatt tgggattata ggtgtgtgtt attgtatttg gttatttttt 15120
gtatttttag tagagatagg gttttattat gttggttagg ttgattttaa attattgatt 15180
ttaggtgatt tgtttgtttt agttttttaa agtgttggga ttataggtgt gaattattat 15240
aattggttag agaagttatt ttaatattta gagatttatg gttaaggaga attttaaaaa 15300
tttttgtatg tatttatata tttgttttag agatgtatgt taattagtga aagattttta 15360
ggtaaaaaaa tatattatta atattttggg gggatggaga gagtgaagga aatgatagaa 15420
aattttttat tattaaagag ttttttgtgt attttttgga taaatgtagg tggaaattaa 15480
ttggttatag taattagaga ttttattgga ttttttgagg agtgtaatgt aattgtttaa 15540
ttttaatata ttaaaaattg ttattatttt aatatgatgg aatattttaa atgtttattt 15600
aaaatgtagt attatagaga ttttttaaaa ttttttttaga aggattggga gaaaaatttg 15660
aagggtattg tttattgtga gtggaatttg gatttttggt tttataatgt ttaggttttt 15720
gtgggattga ggtgggtggg gagagggggt gaagattaga gtaagttgag gatagtatgg 15780
agtgggatta tgggttattt tgattttgtt tgtgggtagt gatttgattt aagttttttt 15840
aggattgatt ttagatagtg gttattaggt ttttttttag ttttttttggt tgttgggatg 15900
gaggtaattg gagggagtag tggttatggt ttgttttttt tgatttgaag gagtagttag 15960
ttttaggaat attattttat ttgtttggtt tttttaaatt ttggaggttg gagaatattg 16020
gttagagggt ggtttatttt gtttttttag tgttggaggt tttagttttt tggagtggaa 16080
agtttgtttg attttgtatt tttttttggt gttgattagg gttgattttt agagtgatgg 16140
taattatttg tttgagggtg tggaggggtt ggttttattt ttttggattt agatttggaa 16200
gtttttggtg ttttttattt attggtagat ggtgttttta tggtggaggt tttttaagtt 16260
gtagaggatt tttgttgtat aaaataaggt ttgttttttt ttgaagttag ggttgtttat 16320
gttgtagatg tggaatttta ggttattttt atttatgatg gttaggatta agtatagtgt 16380

atttttggtt ttgtaggtat aggttaggtt gatttgggag tataagaata gtgttatggg 16440
gaggttgtga taatggttat ttatttttat tttttttttt aggattttgg gagttggggt 16500
gtgagggaat agaggagttt tattttgttt ttttgtattt agatggtttt tgtggtgatt 16560
ttgtttgtgg ttttttttggt tgataagggg tttatatttg tttttttttt ttagtggatt 16620
aaggtgagaa ggttaaggtg ggttagggta ggttagtatt ttgatttttt agtgaattta 16680
atagaatggt gtttgtgttt tttttataga agtatatgtg tgtattagtt ggggaagatt 16740
ggagggtgtt tttttggtta gttagaaaag tagggtttat ttgttttttt gggtatggga 16800
gttattttta tagggttaag taagggtgag ttggaggggt agtggggttt tgggaagtgg 16860
aatatggttt ggtaaaggtt tttttttgta tgtaggatgt gtttagttga tatggtttgg 16920
ttttgtgttt ttatttaaat tttattttga attgtatttt tataattttt atgtgttgtg 16980
ggagggatta gtgggagata attgatttat gggggtagtt ttttttatat tgtttttgtg 17040
gtagtgaata tgttttatag atttgatggt tttataaggg gaagtttatt ttatttggtt 17100
tttatttttt ttttgttatt gttatgtaag aagtgttttt tattttttgt tatgattgtg 17160
gggttttttt agttatgtgg aattgtaagt ttattaaatt tttttttttt gtaaattgtt 17220
tagttttagg tatgttttta ttagtagtgt gaaaatggat taatagagta aattggtatt 17280
agaagagtga ggtattgttg aaaaaaatat ttaaaaaggt agatatgatt ttggaattgg 17340
taataggtag aggttggaag agtttgaagg gtttagaaga taggaaaatg tgagaaagtt 17400
tggaattttt tagagatttg ttgaatggtt ttgtttaaaa tgttgaaagt gatatggata 17460
ataaagttta ggttgaggtg gttttagatg gaaatgagga atttgttggg aattggagta 17520
aaggtgattt ttgttatttt ttagtaaaga aattgttggt attttgtttt tgttttagag 17580
atttttgaaa ttttgaattt gagagagatg atttagggta ttttatagaa gaaattttta 17640
agtaataaag tatttaaggg gtgatatagg tgttgttaaa ggtatttagt tttgtaaggg 17700
aaggagagta taaaggtttg gaaagtttgt agtttgataa tgtaatagaa aagaaaattt 17760
tatttttttga ggagaaattt aggttggttg tttaaatttg tataagtaat aaggagttga 17820
atgttaattt ttaagattat ggggaaaatg tttatagggt atgttagagg tttttatagt 17880
agtttttttt attataggtt taggggttta ggaggaaaaa gtggttttat gggttgagtt 17940
tagggttttt gtgttgtgtg tagttaaggg atttggtgtt tagtgttttg agttttagtt 18000
gttttatttg tggttgaaag gggttaatgt agtgtttgag ttatggtgga agttttaagt 18060
tttggtagtt tttatgtggt gttgagtttg tgagtgtata gaagttaaga attgaggttt 18120
gggaattttt gtttagattt tagaagatgt gtgaaaatat ttgaatgttt aggtagaagt 18180
ttgttgtagg ggtggggttt ttatggagat tttttgttag ggtagtgtag aagggaaatg 18240
tggggttaga gattttatat agagttttta ttggggtatt gtttattgga gttgttagaa 18300
gagggttatt gtttttttaga ttttagaatg gtagatttat tgatagtttg tattgtttgt 18360
ttggaaaatt tgtagatatt taatgttagt ttatgaaagt agttaggagg gagatggtat 18420
tttgtaaagt tatagggatg gagttgttta agattgtggg aatttatgtt ttggtattgg 18480
tatgatttgg atgtgagata tgaggttaaa gagattattt tagagtttta agatttgatt 18540
gttttgttgg atttttgatt tgtatggggt ttgtagtttt tttgttttgg ttaattttat 18600
ttatttggaa tagttgtatt tatttaatgt ttgtatttat attgtattta ggaagtaatt 18660
aatttgtttt tgattttatg ggtttgtagg tggaaaggat ttgttttgtt ttagatggga 18720
ttttggatta tgtatggatt tttgagttaa tgttgaaatg agttaagatt ttgggggatt 18780
gttgggaagg tatgattggt tttgaaatgt gaggttatga gatttgggag gggttagggg 18840
tagaatgata tggttgggtt gtgtttttat ttaaatttta ttttgaattg tatttttata 18900
atttttatgt gttgtgggag ggatttggtg ggagataatt gaattgtggg tagttttttt 18960
tatattgttt ttatagtagt gaataagttt tatgagattt gatggtttta taaggggaaa 19020
ttttttttat ttggttttta tttttttttt gttattgtta tgtaagaagt gtttttttata 19080
ttttattatg attgtgaggt ttttttagtt atatggaatt ataagtttat taaatttttt 19140
ttttttgtaa attgtttagt tttagttata ttttaattag tagtgtgaaa atggattaat 19200
atattggttt atttggtaaa gggttattaa gagagggggt ttagtttttt gttggtggag 19260
tttagatatt taaggttttg tgaagtaagg ttatggtggg gtaggagggg taaattatat 19320
tagaaggtaa tttttttttta tatagggagg tggtatatat gaaatttggt tattgtgggt 19380
taggttggga attggttgtg ttgtaaaatt ggtgtattaa tattagttat ttttgaggaa 19440
aggagataaa atggtagtta aggatgagta agtttatggg gattgtgttt ttgttgtaat 19500
ggttgtagtt gttttttttt ttttatattt gtttaggtga ttattgagaa agggatttag 19560
gaggtgatat tgtgggaatt ttttgttggg gttgtggatg gggtatagtt aaagaaggtg 19620
tagttattgt aattgaaggt ttttaaagat gtgttattta gttttgtgaa ttatatgatt 19680
tttttttttg agtagaattt gttagtattg tttttttaga gataaggttt tataggtagt 19740
atttaggggt ttttgttgaa gtggttggta gtggggagga aggttttgga tttaagaagg 19800
tttgtagttg aaatgatggt tgaagttagt tttatttgtt ttatttttat atattatggt 19860
attgtttata tttgtatttt tgataaggtt ggtagttggg ggtggtttgg gatgagattt 19920
taagatgtaa atttttattt tttgggagtg ttattggttt gtagtggttt tgtttgtttg 19980
ttttgttttg tttttaataa agttttattt tgttatttag gttggagtgt agtggtatga 20040

```
ttttagttta ttgtaatttt tgttttttag gtttaagtga tttttatgtt ttagtttttt   20100
aagtagttgg aattataggt gtttattatt atgtttaggt aatttttgta tttttagtag   20160
aaatggggtt ttgttatgtt ggttaggttg attttgaatt tttgatttta agtgatttgt   20220
ttgttttgat tttttaaagt gttgagatta taggtgtgag ttattgtgtt tggttttgta   20280
gtggtttaaa gatggaaaat aatttttatt tatggggatt gattatatgg attatgtttt   20340
atttttttag gagaatgatg tttaatttta aaaaggggtg tgattagagg tttggatgga   20400
gaaaaagatt tataatgtaa gggaaaaata tttttttata agaggtatag gaagatttta   20460
ttggttaaga ataataaagt ttgtgtatga ataaaatatg tttggagtga ttatttaaag   20520
agggaagttt tttgttttat tgtgtatttt tttgtgttgt ttaaattttt attttgtgta   20580
tatattattt ttaatgtatg tatgtattta gttttagaga tgaggttttg ttgtgttatt   20640
taggttgggg tgtagtggta tgattatagt ttattgtagt tttgaaattt tgggtttaag   20700
taattttttt gttttagttt tttgagtagt tgggattata agtatgtatt attgtattta   20760
gttttatttt ttataatagt gtgtagtaga agatattaga gttgattaaa tatttatgta   20820
ttttatattt tttagttttt tttgaaatta atttgttttt gtgttgattg gttttggttg   20880
gtagattgtg ggtgaaattg aggaatattt gtttagtaga ggtagtggaa agtttttgtg   20940
tggaattttt agtatttatt tttaaggttt tagatggggt agttgttaga tggaggaggg   21000
tggtttaatt tgattggatt ttaggtgagt gatagatgaa tttttgttgt atttagttat   21060
ttagggtttg gggtaaatgt gttattgtag tagggtttag tttagattga ttaatataat   21120
gtatatagat ttgatagtgt tattttagga tgttggtatt aaggttttta ttatgatggt   21180
attagtttag agggtttgtg gaatttatag tttttagttt gggtttaggt gaattttttt   21240
tgtttattga ggagtttata aagtggttta ggagaataga ggtgttggtt ttatgtatag   21300
tagggagtta ggtattggtt ggttttgggg atgggataaa gttagttata gttgattata   21360
atgggttgtg tgatggagga atgttttgtg gttttttttgt agtttagtgt tgttattggt   21420
attttttttt ttaggggtgg ggattggttg tattagagga ttatgttttt ttggagggag   21480
ggaagtttta gtttatagaa gggtgagttg gttattgggt ttagatgttt atttattata   21540
aattgattgt tgattttttt gaggatttgt tttttattga gggttatgga tttttttttt   21600
ttttttttga tttttttttt ttttaagtgt ttgtaggtat atattttatt tgtggtttga   21660
atttggtagg tatagatttg tagtgtaaga aagaaattag ggtggtagga gtattgtgga   21720
ggtttgtttt gagttttttt agggtgggga ttataggttt tttaggtaga gaatggatgg   21780
ggttagagtt ggttttgttt ggttttatga gtgttttagg tttttaggat ttagtgatgt   21840
ttggtttggg gtttatagtt gttttgggtt tttgtttttt tttttttttt tttttaagga   21900
tttttagttt tggaaggggt agaatgagtt atttatatta aggtttagat aattgattgg   21960
ggttttaagt attgttttat ttttagggta ataggatttt tttttattgg ttgttggtag   22020
agaagtttgt tttgggaggt agtgatagga gatttttaag aatattgatt ttataattta   22080
gataagtggg aattaaagta tttgggttta agttttagtt ttgttttata ttgtttgtgt   22140
gtttttggtt gggttattta attattttgg attttagttt tttttatttt taaaatggga   22200
ataataaggt ttgttttata tgttgggaag attgggtaat gtataatgaa attttgaatt   22260
ttgtatgata gtgttattta ggtattgagt ggttaaggtt gtttagtttt tttatgtttt   22320
tatatttttt ttttatatat gattagggtt gtttatttgt tttttgtagg ttggattttg   22380
tttgaggttg gtaggagagt atgtttgttt tatttgttta gttttataat ttttttttat   22440
tatagatata tttgatagtt ttaaggttat tgattttttt ttattttttat tgtttttggg   22500
gtttatattg agttattggt gtttttgggg gatttgtggg ttaatttttt aggttgattt   22560
agaagggata ggaaaggtga tttgaatgta agaattttta attaaaaatg tgaatgttta   22620
tttatttttt tgaagttttt ttttttagt atttgatatt gtttgaaagt gtttttggtt   22680
attggttgtt tg                                                       22692
```

<210> 237
<211> 5096
<212> DNA
<213> Artificial Sequence

<220>
<223> chemically treated genomic DNA (Homo sapiens)

<400> 237

```
gaggttgagg tgggtggatt atttgaggtt gggagtttga gaatagtttg attaatatgg      60
agaagtttta tttttataaa aaatataaaa ttagttaggt gtggtggtgt atgtttgtaa     120
ttttagttat ttggtaggtt gaggtagaag aagagtttga atttgggagg tggaggttgt     180
ggtgagttga gattgtgtta ttgaatttta gtttgggtaa taatagtgaa attttgttta     240
aaaaaaaaaa atgtttataa ttgttatttt ataagaaagg aaaaagtttt taaagagata     300
```

```
tataaaaagg atatttatag ataaaagaaa ttttatattt aggaggttat tagattgatt      360
ataaaatata tttggatttt aaaagtatta gaatattagg agagaaaaaa aaaaaaattag     420
gttaggtatg gtggtttatg tttataattt tagtattttt ggaggttgag gtagatggat      480
tatttgaggt taggagttta agattagttt ggttaatatg gtgaaatttt gtttgtatta      540
aaaattagtt gggtatggtg gtaggtgttt gtaattttag ttatttagga ggttgaggta      600
ggagaattat ttgaatttgg gaggtagagg ttgtagtgag ttatgattgt gttgttgtat      660
tttagtttgg gtaataagag taaaattttg ttttaaaaaa aaaaaaaaaa aagaagaaga      720
agaaaagaaa agaaaattat tgttaatttt tattatattt tgttaaatat atattttgtt      780
atattatttg ttttagtttt aaggttattg gtattttttg aagtaattat taaatgtttt      840
tattatttaa gaatagagag ttaatggtga taattaattt atatagagta ttgaaaggag      900
ttaggttttg tttgtttttt tttgtatagt aattttgtaa tagtattata aggtaggtat      960
tgttattatt tttattttat aaataagaaa attggagtat aggttgggtg tggtggttta     1020
tgtttgtaat tttattattt tgggaggtgg aggtgggtgt attatttgag gttaggagtt     1080
taagattagt ttggttaata tggggaaatt ttatttttat taaaagtatg aaaaaattag     1140
tttggtgtgg tggtatatat ttgtaatttt agttattttg gaggttgagg taggataatt     1200
gtttgaattt gaaaggttga ggttgtagtt agttgagata gagtttttgt atttaggttt     1260
gggtgataga atgagatttt gttaggaagg aaggtgatag aatgagattt tgttaggagg     1320
gagggaggga gggagagaaa agagggaggg agattggagt atagagaagt taattgattt     1380
gtttaagaag ggagggaggg agggagagag ggagattgga gtatagagaa gtgatttgtt     1440
taggaaggaa ggaaggaagg taggaaggaa ggaagggagg gagggaagga aggaagggag     1500
ggagggaggg agggaaaaga gggaaggaga ttggagtata gagaagttaa gtgatttgtt     1560
taagaaggaa gggagggagg gagattgaag tatagagaag tgatttgttt aggaaggaag     1620
gaaggtagga aggaagggag gggtggaggg agggaattgg agtatagaga attgatttgt     1680
ttaaggttat ttagtagaag gaagggagga aggaagggag gaaggaaggg agggagggag     1740
attggagtat agagaagtta agtgatttgt ttaagatgga tggatatgga tggaaggaag     1800
gaataaagaa ggagggaggg agggagggag ggaattggag tatagataat tgatttgtgg     1860
aaggaaggat ggaaggaagg agagagggaa tgaaggaagg agagagggaa ggaaggaagg     1920
aaggagagag agggaaggaa ggaaggagag agggagagag ggagggaagg gagggaattg     1980
gagtatagag aattgatttg tttaaggtta tttagttagg atttgagttt agaatgtgag     2040
tgtttttagt tattatattg aattgttttt tagttgagaa tttttaataa atgtattagg     2100
atatatataa ttggatgtta taaggtggtt tttggttaat tttttttttt aaatatatgt     2160
attaatttta aattatttat atgataaaaa atgtttatat ttttattgat tttgttttta     2220
aaatggtatg gttttttagg attatttttt ataattgtta aagggaaatt tgttttgtta     2280
atatgaaaaa aaaattaaat atttgtttta tgagttgtta gtggttaatg ttgtttagat     2340
tatggtttga atgtattatt ttttaaagat tattttgtgg ttttgggtaa attgtttagt     2400
tttttttgtt tttttagtgg taaagtggat ataataatag tattattttt taaagtggtg     2460
aggattagtt gatattattt atataaattg ttatgtatag tatttgatat agagtgagtg     2520
tttaataaat gtttttttatt atttaggttt tgggtttttt tatttttaga gtgtaagttt     2580
tttagtaatt atagaaatgt tttttagagt tgttgtttat tttttaaaaa tatttgtttt     2640
tattttgtat agagtatagt gatattataa ataggttttt ttggatttta gttttggggg     2700
tggttatatt attttgttta ggtttaggaa gagagagttg gttttggttt tttatttggt     2760
agtagtataa aatttaatta ggattttttta ttttggttaa tttttgaatt attgtgttta     2820
gtttggttgg tttggtagta gatggaatga gggtgttggt gttaggaagt ggtgtttatt     2880
tatttgttta gtttgaggtg ggtatttttt ttttatagta tttttgggga ttttgttttt     2940
ttattgtgga atgttttaga ggattgattt aatagtgtta tagggtgggt ttttttttaa     3000
atttttttaaa atatattgtt atgattttga aatgggagtt gagggagatg tattaagtgt    3060
tttttgatgt taggtgaagt gtattatatt tggtgtgtgt gttgagtttt gaattgaagt     3120
ttgtgtggtt gttatttttg tttttttgtt gggggtttta gagagtgtgt tttttgttta     3180
tgtgtgtgta gggtttgtag ttgtttttat gttttagtgg agttgtagta tgtttttttt     3240
atttattagg tgaagagttt gtttttttatt ttgtgtagta ggttggagaa tttggttgtt    3300
gttttttttg tgagggggttt tggggtgttg gggtttgttg gtgagtttgt ttttattggt    3360
gtttttgtgt gtttttggtg tttaaaattt ttgaagtgtt gggttttgga agatgggagg     3420
tggtgtttgt gtggttggtt ggggttttgt tttggttttg tgtttttttt tttgtttttt     3480
tgtttgtgtt tttttttgttt ttttgtttgg tttttttagt gattaattat taattgttaa    3540
gttttaaggg ttagataata tatttgtaga ttataatttg gtatttttag ggtggtagga     3600
ggagaaaggg taattttttt tttgttttgg tagttgagtt ttattgttga gtggtattta     3660
gtgtgttttt aggtttttag ggtttttttgt ttgaaattat aagatatagt gatatgaggt    3720
tattgtaggt gatttttaag ttaagaagtg gtgagtgagt agtgagttag aagtagtgag     3780
tgagttggga tttttttaat tggaaggatt ggttgatttt tttttttttt ttttattttt     3840
ttttttttttg gttgatttgt ataaaattgt tttattaaaa taattgaagg ttggtagggt    3900
gtgttggttt atatttgtaa ttttaatgtt ttgggaggtt gagatgggtg gattatttga     3960
```

ggttaggagt ttgagattag attgggaaat atggtgaaat tttgttttta ttaaaaatat 4020
aaaaattagt tgggtgtggt gatgggtgtt tgtagtttta gttatttgag aggttgaggt 4080
aggagaattg tttgaattta ggaggtggtg gttgtgagtt aagattgtgt ttttgtattt 4140
tagtttgggt aatagaggga gatgttgttt taaaaaaaaa aaaaaaaaaa aaaaaaattt 4200
gaaggtgttt taggtttgtg ttgttttttt ttgtaaaata tgggtaagat tttaaaaaga 4260
ttttggaaat gtgtttttgt ggtgtggatg gaggtgtggt ttattttttg tggtgtgttg 4320
gttttgtttt ttagtattta gttgtttttg ttgtgttagg gagagagatg gtgttaattt 4380
ttggtgtttg gtttggatgt tttgttttgg ttatgttttt tgaggttttt atgggttaaa 4440
gagggattaa gagatattaa gttggggttg aaaagttttt gttattgatt ttttgttttt 4500
ggaaaggagg agatttagaa aataaaagaa aaggtgttgg gatttttttat ttttttttgg 4560
ttatttgttt tgggggttgg ggtgggttaa aataaatgta ggtttaaata gataggttgt 4620
gtagatgagt tggtaatttt gtaagaggag tttaagaaaa aaaatagttt ttgaaaatga 4680
aatattattt taggaatttt aagtttagta gttgtaggaa ttttaaaaaa atttttgaaa 4740
ttaagaatgt ggttatttgt agagattaat taagtattat aatataagta tattatgaaa 4800
taagaggata ttagaagtat atttaagatg ttatttttgt aaagtaaata atttatttaa 4860
ttttgtatat tgataatgtt gaattttatt tgagttttgt gtttttggaa agtaatgata 4920
attaagaaat ttttttaggt tgggtgtggt ggttgatgtt tgtaatttta gtgttttggg 4980
agattgaagt gggtggatta tttttgaggt taggagtttg agattagttt gattaatatg 5040
gtgaaatttt gtttttatta aaattataaa aattagtttg gtgttttggt gtgtgt 5096

<210> 238
<211> 5096
<212> DNA
<213> Artificial Sequence

<220>
<223> chemically treated genomic DNA (Homo sapiens)

<400> 238

gtatatatta aaatgttggg ttaatttttg tagttttagt agagatggga ttttgttatg 60
ttggttaggt tggttttgaa tttttgattt taggagtaat ttgtttgttt tggtttttta 120
aagtattggg attataggtg ttagttattg tgtttggttt aggaaggttt tttaattgtt 180
attatttttt aggagtatag ggtttaggta gaatttaatg ttgttaatat gtagggttaa 240
atgagttgtt tattttataa aagtgatatt ttaaatgtgt ttttaatgtt tttttgtttt 300
ataatatgtt tgtattataa tatttaatta gtttttatgg atgattatgt ttttagtttt 360
aaaggttttt ttgaggtttt tgtagttgtt aaatttgggg tttttgaagt aatattttat 420
ttttgaaagt tgtttttttt tttagatttt ttttatagaa ttgttagttt gtttgtatag 480
tttgtttatt tgaatttatg tttattttga tttattttaa tttttaaagt aggtggttag 540
gaaaaaaataa aaggttttaa tattttttttt tttgtttttt ggattttttt ttttttggaa 600
gtaggggggtt agtgatagga atttttttgat tttagtttag tgttttttaa ttttttttta 660
gtttgtggaa gttttggaag gtgtggttag ggtagggtat ttaagttgag tgttgggggt 720
tggtattgtt tttttttttg gtatggtgga gataattgaa tattgaaaga taaaattaat 780
atattgtgag gaatgagtta tgtttttatt tatgttataa aggtatgttt ttaagatttt 840
tttaggattt tatttatgtt ttgtagggaa aagtgatata gatttgaagt gtttttaggt 900
tttttttttt tttttttttt tttttaagat gatgtttttt tttgttgttt aggttggagt 960
gtaaaggtgt gattttggtt tataattgtt gtttttttggg tttaagtgat ttttttgttt 1020
tagtttttttg agtagttggg attataggta tttgttatta tgtttggtta atttttgtat 1080
ttttagtaga gatggggttt tattatattt tttagtttgg ttttgaattt ttgattttag 1140
gtgatttatt tgttttggtt ttttaaagtg ttgggattat aggtgtgagt taatgtgttt 1200
tgttagtttt tagttatttt aatgaagtga ttttgtataa attagttaga gggagggaga 1260
gtgggaggga agaggagggg ttagttagtt tttttagtta ggagagtttt ggtttattta 1320
ttgtttttaa tttattgttt atttattgtt ttttagttta ggaattattt ataataattt 1380
tgtgttattg tattttgtga ttttaaataa ggaattttaa aaatttgaag gtatattggg 1440
tgttgtttaa taataaggtt tggttgttaa gatgaaaaaa aaattgtttt tttttttttt 1500
gttattttgg aggtgttagg ttgtaatttg taaatgtatt gtttggtttt tagggtttga 1560
tagttaatga ttggttgttg aaggagttga gtggaggagt gagagggatg tgagtaagag 1620
agtgagggag ggagtgtagg gttagagtgg agttttagtt ggttgtatag gtattgtttt 1680
ttgttttttg ggatttggta ttttgggggt tttgggtgtt ggagatgtgt aagggtgttg 1740
atggaggtag atttgttagt gggttttggt gttttagagt tttttgtgga gggagtggtg 1800
gttgagtttt ttagtttgtt gtgtaggata aaaggtaaat tttttatttg gtgagtggaa 1860

```
ggggtgtgtt gtagttttgt tggggtgtgg gggtggttgt gggttttgtg tgtatgtgga    1920
taaggagtat atttttgag gtttttagta gggaggtaga ggtggtggtt gtatggattt    1980
tggtttgggg tttaatatgt atattaggta tggtgtgttt tatttggtgt tgggaagtat    2040
ttggtgtgtt ttttttggtt tttgttttga aattgtggtg atgtattttg gaggatttgg    2100
ggggaaattt attttgtggt attattaagt tggttttttg gggtgtttg tggtggaggg    2160
atgaggtttt tggggatgtt gtgaaggagg agtgtttatt ttgggttggg taggtgggtg    2220
agtgttattt tttggtgttg gtgtttttat tttatttgtt gttgagttag ttagattggg    2280
tgtggtgatt taagagttga ttgaagtgag aaattttggt taagttttgt gttgttgttg    2340
ggtgggagat tagagttggt tttttttttt tgggtttgag tgaggtgata tagttatttt    2400
tagggttggg gtttagggaa gtttgtttgt ggtattatta tgttttgtgt gaagtaggga    2460
taaatgtttt taaggagtgg gtagtgattt tagaaagtgt ttttgtggtt gttggaggat    2520
ttgtattttg gaaatgagag aatttaggat ttaaatagtg aaagatattt attgggtatt    2580
tgttttgtgt taggtattgt gtataataat ttatatggat aatattagtt aatttttatt    2640
attttaggag atggtattat tgttatattt attttgttgt tggagaaata aagaaggtta    2700
agtaatttgt ttaaggttat aagatgattt ttaaggggtg atgtgtttaa gttgtagttt    2760
aaatagtgtt ggttattgat aatttatgaa atagatattt gatttttttt ttatattaat    2820
aaagtgaatt ttttttaat gattgtgaaa gatagttttg aaaagttata ttattttaaa    2880
aatgagattg ataaaggtgt aagtattttt tattatataa atagtttgaa attgatgtat    2940
gtgtttaaag gggaaagttg gttgaaaatt attttgtagt atttagttgt gtatgttttg    3000
atatatttgt taaaagtttt tagttgggag gtaatttagt gtaatggtta agggtattta    3060
tattttggat ttaaatttg gttgggtgat tttggataag ttagtttttt gtgttttagt    3120
tttttttttt tttttttttt tttttttttt tttttttttt tttttttttt tttttttttt    3180
tttttttttt tttttttttt tttttgtttt tttttttttt tttttgtttt ttttttttat    3240
aagttagtta tttgtatttt agtttttttt tttttttttt tttttttttt ttgtttttt    3300
tttttgtttg tgtttgtttg ttttggataa gttatttaat ttttttgtgt tttagttttt    3360
tttttttttt tttttttttt tttttttttt tttttttttt tgttgggtga ttttggataa    3420
attagttttt tgtgttttag tttttttttt ttgtttttt tttttttttt gtttttttttt    3480
ttttttggat aagttatttt tttgtgtttt agtttttttt tttttttttt tttttggata    3540
agttatttaa ttttttgtg ttttagtttt tttttttttt tttttttttt tttttttttt    3600
tttttttttt tttttttttt tttttttttt tttttgtttt tttttttttt tttttggata    3660
agttattttt ttgtgtttg gtttttttt tttttttttt tttttttttt ttggataagt    3720
taattaattt ttttgtgttt tagttttttt tttttttttt tttttttttt tttttttttt    3780
ttgatggagt tttattttgt tatttttttt tttgatagag ttttatttg ttatttaggt    3840
ttgagtgtag aggttttatt ttagttaatt gtaattttaa tttttgggt ttaagtgatt    3900
attttgtttt agtttttgaa gtagttggga ttatagatgt atattattat gttaggttaa    3960
tttttttatg tttttagtag agatggggtt tttttatgtt gattaggttg gttttgaatt    4020
tttgatttta agtgatgtat ttattttgt tttttaaagt agtgggatta taggtgtgag    4080
ttattgtgtt tagtttgtgt tttagttttt ttatttgtaa aatggagata ataaatagtat    4140
ttattttgta gtgttgttat aaggttatta tgtaggaaag ggtagataga gtttggtttt    4200
ttttagtgtt ttatatgggt tagttgttat tattagtttt ttattttttgg gtgatgggaa    4260
tatttggtga ttgttttagg agatgttagt ggttttgagg ttggggtaga tgatatggta    4320
agatgtatgt ttaataagat gtgataagaa ttaataatga tttttttttt tttttttttt    4380
tttttttttt tttttttttt ttgagataga gttttgtttt tgttgtttag gttggagtgt    4440
agtggtatga ttatggttta ttgtaatttt tgttttttgg gtttaagtga ttttttgtt    4500
ttagtttttt gagtagttag gattataggt gtttgttatt atgtttggtt aatttttaat    4560
atagatagga ttttattatg ttggttaggt tggttttgag tttttgattt taggtgattt    4620
atttatttta gtttttaaaa gtgttgggat tataagtgta agttattgtg tttggtttga    4680
tttttttttt ttttttttta atgttttgat atttttggaa tttgggtatg ttttataatt    4740
agtttagtag ttttttaaat atgagatttt ttttgttat gaatgttttt tttatgtatt    4800
tttttgaggg tttttttttt ttttgtggaa tagtagttat gggtattttt tttttttaga    4860
tagagtttta ttgttgttgt ttaggttgga gtttaatggt gtgattttgg tttattgtaa    4920
tttttgtttt ttgggtttaa gtttttttttt tattttagtt tgttaagtag ttgggattat    4980
aggtatgtat tattatattt ggttaatttt gtattttttg tagagatggg gttttttat    5040
gttggttagg ttgtttttga atttttaatt ttaggtgatt tgtttgtttt ggtttt        5096
```

<210> 239
<211> 3072
<212> DNA
<213> Artificial Sequence

<220>

<223> chemically treated genomic DNA (Homo sapiens)

<400> 239

```
ttttaattta gtgggatggt ttgtattttt atttgttaaa tttggtaatt tttggggttg      60
gggtttgggg tttggtaatt tttggggtgg gaggtttttg tgtagagttt aatttgtttg     120

ttaaggttta tttattggag aagaattggt tgatggaggt gggtaggagt gtgaggaagg     180
ttaaggggtg ggtgaaggag atggaaagga tagtggagat gtaggttatg ttggttatta     240
tggagtagag gtaggttagg gaggtgtaga ggtagaatag gatgaagttg tgtatgtttt     300
tgttgttgat gtagttgttg gtgaagaaat agtgatggtt gtgttttagg gtgattttgg     360
tgtatatttg gtagaagtgg gtgttaggtg aggggtatgg agttttttttg gttgaggttt     420
tttggtaggt ttttaggttg tttggggagt tttggatgat aaggatgtaa ttgtttaggg     480
tgttggttga gaggaatagg aagagtgttt tgtggagtag ggtgggtgag aagagttggg     540
tggttgtggg gtttttgtgt atgttgggta ggaagaggaa gagttgtagt atgaaggtta     600
ttagggagat gtataagaag taggtggggg ttattatgtt gagtagttgt agggttagta     660
tttttgatta tatttttgtg gggtttgggg tgagaagagg attgattgat tatggtgttt     720
agggggtgtt tttgtttgtt tttttgtgtt gtgtgtttgg ggtattgtat gtgattttat     780
gtttttaatt ttttgttttg tggatttttt ttttttagtt ttttaatttt ggattaggta     840
ggttagagat ttgagtttgt agtggttttg ggtttaggtt ttatttgggg gtgagtgttg     900
tttggggttt tgagtgagtt tggtgttttg gtgtggtaga gtttggtata ggagggaggg     960
atttgtttgt agtttttagg agttgtgagg gtgggggggta ttgggggata ggattttttt    1020
tttttgtgga tgtggaaata agtttaggtt tgggattttt ggtttagatt tttttaggag    1080
gagaaaggga aggatttggg gttgggggta ggagaattag tggtatttga tttgttggaa    1140
attttgtttt atattttttt tttttttgttt ttgtgttttt atttatatat atattttggg    1200
tagtatttag ggattttatt aggttattta aggtttttttg gaggtatgtg tgtgtgaggg    1260
attatgatgt gtgtgtgtgt gtttttgttt gtgattttgt atgtgtaatt tgtgtgtgtt    1320
tgagtatttg tatttgtgta tgtatatatt tgtgtgtata gatatgtagt ttttgttatt    1380
tagttttaaa aaaattaatt gaaataattg atatatttat atttgttttt gggtattggg    1440
tgaggtgggg gtgtaattag aatatttgtg ttagtgggtt ttggttagta tatttttttta    1500
atgttggttg tttgggttat tttttttgtga agagtgtttt tatttgaggt gaaattaaaa    1560
tttttttttt gtgtttgttt tttgtttttt tgttgagaga aatttaaata atttttatgg    1620
tgttgaaatt tttttttatgt tgataagttt gttttgggtg tatgagtgga tgtttggtta    1680
gtttttttttg ggattgattt tgttgttgtg tttagttttt attatgtttt tattttattt    1740
tatgttttat agttggggtt tttggttagt tttggaagtt attgagaaat aggattttgt    1800
gtgtttgaga gaattttttt aggggttaag gaattggtta gttggaggtg tgagaaaagt    1860
tttgggaagg tggttgtatt taggatgggt ggatgttatg gggtttttgt ttaggttgtt    1920
ttgtttgtat gggttgattg gttattttgg aattttttttt gtaggtttta gtgtttttttg    1980
ggaatttgta gtttttgtgg agagtgtggg ttttttttta ttgttggggt gtagtgtagt    2040
gtatgtttga gggtggttgt tgggggttgg gtatgttttt agttttgtgt tgttgggggt    2100
tgtggtggtg ttgtttattt tagagagttt ttggtttggg gttttggtga agtaagtgtt    2160
tttttggtgt tggttgttag gggggtgtgg gagtagttag atgtgttgta gtgttgggaa    2220
ggtggtgaag gatagggggtt aggggagtga ggggtgtttg gtaggtagtt ttagttttgg    2280
ttttgtgtgg gagaagggat agtagagatt gtttgtgggg ttttggggtg tagggagttg    2340
tttgtttagt tttggtgttt gttttgtttg tggtagaggg tggtatagtt ggagttttgg    2400
aaagattggt agtgttggta gttgtgggtt ttttggttat tgttttttgg atgtatggga    2460
agttgttttt tgtgttgttg ttgttgtatt gttgttgttg tagaggggtg aggaaattaa    2520
tttattgagt tttggttgtt gataagagga gttttggatg ttggtttttg ttttgtttga    2580
ggttgtaaag ttgtggattt ggtttggttg gtttgtagtt tgtgttttgt tttgggaatt    2640
gggtaagtag tggggatgtg gggaggaggg agtgggtagt tgttggtttt ttatttgggt    2700
gttagtgagt aagggttagg aagtggtggg gatggtagtt gggtattttt tagttttgtt    2760
attttttttt ttgtttttgg gggtggtgtt ttggttgtta gttttagtat tgtggatttg    2820
tttttttttt tttttttgat ttttttttgag tgggttgggg tgttttgttt ggttttagtg    2880
tttgttttag ttgggagatt gggatttagt tttgatgtgg atttttttagt tattattttg    2940
tttttttttt ttggttaggt tttatgattt tttttgtttt ttatttggat tgaaggaatt    3000
ttgggggttt gagaggttag tttgttaggg taatatttat gaagattagt gttatttttt    3060
tggaagggtt gt                                                        3072
```

<210> 240
<211> 3072
<212> DNA

<213> Artificial Sequence

<220>
<223> chemically treated genomic DNA (Homo sapiens)

<400> 240

```
atagtttttt tgaggagatg atgttgattt ttatgggtgt tgttttggta agttgatttt      60
ttaagttttt agggtttttt tagtttgagt ggagagtagg gaaggttgtg ggatttagtt     120
gagagggaag ggtaggatga tggttggaga atttatatta gggttgaatt ttaatttttt     180
agttggaatg ggtgttgaaa ttaggtaggg tgttttagtt tgtttaaggg gaattgagag     240
gggaggaggg ggtaagttta tgatgttgag attggtggtt agagtattgt ttttaggagt     300
ggagggaggg gtggtggggt tggggggtgt ttagttgtta tttttgttgt tttttggttt     360
ttatttgttg gtgtttaagt gggaagttag tagttgtttg tttttttttt tttatatttt     420
tgttatttgt ttagtttttg aagtgaagtg taggttgtga gttagttggg ttgagtttat     480
aattttgtag ttttgggtag ggtgagagtt ggtgtttggg gttttttttg ttggtgatta     540
gagtttggtg agttgatttt tttatttttt tgtgatggtg gtagtgtggt ggtggtggtg     600
tggggggtgg tttttttgtgt gtttgggagg tagtggttga gaagtttgtg gttgttggtg     660
ttgttggttt ttttaaggtt ttggttgtgt tgttttttgt tgtgggtgag gtgggtgtta     720
gggttgaatg gatagttttt tatattttgg ggttttatgg gtggtttttg ttgttttttt     780
ttttgtgtag ggttagggtt gaggttgttt gttgagtgtt ttttattttt ttagtttttg     840
tttttttgttg ttttttttagt gttgtggtgt atttggttgt ttttgtgttt ttttggtgat     900
tggtgttggg aaggtatttg ttttgttgga gttttaggtt gagggttttt tggggtgggt     960
agtattgttg tagtttttgg tggtgtagga ttgggggtgt gtttagtttt tggtgattat    1020
ttttaggtgt gtattgtgtt gtgttttagt ggtagggaga ggtttatgtt ttttgtggag    1080
gttgtgggtt tttgggggggt gttgggattt gtaaagggga ttttaaggtg attagttgat    1140
ttgtgtggat gggatagttt ggatggaggt tttgtggtat ttatttattt taggtgtaat    1200
tgttttttta gaattttttt tgtgtttttg gttggttgat tttttagttt ttggaaaagt    1260
ttttttgggt gtatggaatt ttattttttg gtggtttttg aagttggtta ggaattttgg    1320
ttgtgaggtg tggggtggga tggggatgtg gtgagggttg agtatggtgg tggggttgat    1380
tttaggaaag gttggttaag tatttatttg tgtgtttagg gtgggtttgt tggtatggaa    1440
aggattttgg tgttatgagg gttgtttggg ttttttttag taggagggtg ggaggtgggt    1500
gtgaaggggg gattttaatt ttgttttaaa tgaaaatgtt ttttgtaaag aaatagtttg    1560
ggtagttagt gttggagaga tgtgttggtt aggatttgtt gatataggta ttttggttgt    1620
gtttttattt tatttggtgt ttgagagtga gtgtgagtgt attgattgtt ttaattgatt    1680
tttttaaagt tagatagtag aggttgtgtg tttgtatatg tagatatata tatatataaa    1740
tatagatatt taagtgtata tagattatat gtatagagtt atagataagg atatatatat    1800
atatattgtg gttttttatg tatatatatt tttaaagagt tttgggtaat ttggtgaggt    1860
ttttgagtgt tgtttaagat gtgtgtgtgg gtggggatgt agggatggag aaaggggagt    1920
gtgaagtagg atttttagta ggttaagtgt tgttgatttt tttgtttttg attttaaatt    1980
tttttttttt ttttttttagg gaggtttgaa ttagaaattt taaatttggg tttgttttta    2040
tatttataga ggggaggagt tttatttttt ggtgtttttt atttttataa tttttaaaga    2100
ttgtaaatga gttttttttt tttgtgttag attttgttgt gttaaggtgt tgagtttgtt    2160
taaaatttta ggtaatattt atttttaggt ggggtttaga tttgaggttg ttgtgggttt    2220
gggtttttgg tttgtttgat ttggggttag ggaattgggg aggggaaatt tgtgggatag    2280
ggggttgggg gtgtggagtt gtgtgtagtg ttttagatgt gtggtatgga ggagtgggtg    2340
agggtgtttt ttggatgttg tagttagtta gttttttttt tattttgggt tttgtaggaa    2400
tgtaattgag gatgttggtt ttgtggttgt ttaatgtggt ggtttttgtt tattttttgt    2460
gtattttttt ggtgattttt gtgttgtagt tttttttttt tttgtttagt atgtgtgagg    2520
attttgtggt tgtttggttt tttttgtttg ttttgtttta tggggtgttt tttttatttt    2580
ttttggttaa tgttttgggt aattatgttt ttgttattta gaatttttta gatgatttgg    2640
gggtttgtta gggggttttg gttaggaaga ttttatgttt tttatttagt atttattttt    2700
gttgagtgtg tgttagagtt attttgaggt atgattatta ttgttttttt attggtaatt    2760
gtattggtag taggaatatg tgtaattttg ttttgttttg tttttatatt tttttggttt    2820
gtttttattt tatggtggtt ggtgtggttt atattttttgt tgtttttttt attttttttg    2880
tttatttttt ggtttttttt atgtttttgt ttatttttat tagttagttt ttttttggtg    2940
agtgggtttt ggtgggtagg ttgggtttta tataaagatt ttttatttta aaggttgttg    3000
ggttttaaat tttaatttta aaagttgtta gatttagtaa ataaaaatat agattgtttt    3060
gttaaattaa aa                                                         3072
```

<210> 241

<211> 3845
<212> DNA
<213> Artificial Sequence

<220>
<223> chemically treated genomic DNA (Homo sapiens)

<400> 241

```
ttgtttttta tgtttaagta atttttttgt tttagttttt tgagtagtta agattatagg      60
tatgtgttat tatgtttagt taattttttt gtttttttgta gagatgggtt ttattatgtt     120
ggttaggatg gttttgattt tttgattttg tgatttgttt attttggttt tttaaagtgt     180
tgggaatata ggtgtaagtt attgtgttta gtttatattt tattttttga ttgattttttg     240
tgattagagt taatttttta tgttatttta tgtgaatatg tattaatgaa ttattagaaa     300
ttgtaatgtt tttgtaggtt aaaataattt aatattgata attttatatg atttagttta     360
atatattatt tttttttatta gttatattgt atgttattat ttatatatat gtttatttag     420
ttagtttatt tgttttttgt gaggaatatt atatagtttt tgagattttg ttatgataga     480
agttagtatt gataagtttt tgtgtatttg ttttaatgta ggattattgg gaattgagta     540
gtagtttata gggtttggga ggggatttaa ttatttagtt aatttttatt tatattattt     600
tttttttttt gataatgggt aattgagaaa gggtaaatgt gtgtaaaaat tatgtagatg     660
tagttttagg attatttttt ttttttttgt agtgtttttt tttaagttat attagtgttt     720
aaattttgga gttattgatg tttttgttat ttttgtagat agaatattat gttgtaaata     780
atgtttattt gtttattagt attatataaa agtgtttgtt tttttttatt ttagttatta     840
ttgattattg gtaaaaattt taatgtattt aattttaata ggtaaagaaa ggttttttta     900
tttagatttt aggtgggggt ttaagtttat atttttgttt tataaaaatt gaatagttag     960
tgtttttgag ttgaatgatt tatttatttt gtataggaga gtttaattat attgtgattt    1020
tgggggagat aatttttata atttattttt ttaaaatgta gaaagtagaa aaagggtaga    1080
aaaattgtat taaatttgaa taatgtagta tgtttttaga gttagggata tttttttttg    1140
gaaggatttt aagtttggtg tatttgtatt gattgtggtt atggtgtatt taattttttt    1200
aaattagttt ttttgtattg tatataggaa gatttgaggg tgagtgtaaa ttaggattgt    1260
ttggttagtg gtgtgtgtaa aaatttattg tagaattaag aattattttt ttagtatttt    1320
atatatttta aaaaataggt agatatgttt attgatttta ggttgtttat ggtttttaga    1380
ggtttttttt tattgtaata gtgttgtggg gttttagggg tttagtagtt tttttttttt    1440
aggttatttt ggtgagggag ggagttggga tttttgtatt gtggtaaata agtatgtttg    1500
tgtggttgta gaggtaggtt ggtgtgtatg tttaggtggg gatgtgtgtg aagtttgttg    1560
ttgttgtagt gagtttggtg tagagtggag tggttgttgg agatgtttga tgtatttgtt    1620
tgaggagtgg ttagtgatgt gatggagtgg gtaaggttag ttgtgttggt ggtttttttt    1680
aagagatagt ttggggagtg gttattttta tttattagat attttaagtt tttaggattt    1740
ggagtattga ataaatggaa tttgggtttt aaagtttttt gttttggaga attagatttg    1800
gaatgtttag aggtttgttg ttgtgttgtt ttgttttgat ggtattttgt ttgtttgtat    1860
tggggtgtgt tttttatttg tttttaattg tggtgttgtg ataggtttta ttgtgggtgt    1920
ttgtggtggg aagggtggtg gtgattggga gtatgtgggg taattgtagt gggtaaggga    1980
tatgggtgga ggtgggtata ttagggtgat tgtagttttg tgtggtgagg gtatgtgggg    2040
ggattagtgt atagggattt taggtggagg taggtatatt ggagtgattg tggtggggtg    2100
aaggtatgtg aagtgattgg tatttagggg gtgttgagtg taggtaggtg tataatagtg    2160
attattgtgt ggtggagtag ggtatgtggg gtgattgggg ggggaatttg ttgtagtgat    2220
tgaggtgagg aggttatggt agtggattag ggggatgagt tggtgtgata gtggtggggg    2280
ttgtgttggt ggttgtggtg tggggagagt ttgtaggaag tggttgggag gtagggggaa    2340
ggtggtgata gtgggtattg gtggtggtgg gtattggtag tgggtaggta tggtggtggt    2400
ggattttggt ggtggtaggt atggtggtgg tgggtatggt atggaggtgg tgggtatggt    2460
ggttgtgggg tttgttgttg tttggagtga ttaagggatg ttgaatgatt gttgtgtaga    2520
ggagggggta ttagaagggg tagtagtata gtttgtagtt tttaattttt ttgtgatttg    2580
tgatggttat ttggtttgtt tattgtggtg gtggtgtttt tttattaaaa ttgtgtaatg    2640
tttatattgt tgtagggggtt gtagaaatgt gtggaatttt tgtgtatttt ttaggaattt    2700
ttgttttaga ggttatggta taagaagatt ggaatgatgt gtagaatttg tatttataga    2760
ttttttgatt gataggttag gtgatgtttg ggggttattt ttttttttatt gttttttttt    2820
gttgggaatt aggttttgga aggttatgtt gaaataattt gggggtttgt gttgtgttat    2880
tgttattgtg tagttttttt aagatggttg ttgttgtagt ggtttagatt tgtgtaagtg    2940
ttttagtggg ggggtggttg tttttttttt gtagagttgt tagtggggag ggggtaggtg    3000
gtgtgtgttt agtttttgtt gttttggagt tttagttgta ttttttttag ggtgtagtgg    3060
taaggagagg aagttggtta aggtgggtgg ttgttttttt tttaaagtgt tgtttgggaa    3120
```

```
aggatgtagg ttgtgtagat gtagtagagg tgatagttgt ttttgtttgt agtggggtga    3180
gatgtggggt aggggagtga gggtttatgt agtttttgtt tggaaggaat ggtagttttt    3240
ttttttattt ggttgatata gtagttgttt ttttttggtt gtggtgtagt agaggggga    3300
ggagggagat gggttggtgt aggttttgtt tagtaagttt ggtagttatt ttttatttat    3360
ggtgtagtag tagaggattg gaggggaatg tggttagagg ttaggtattt gtattatttt    3420
tgtttgtgtg ggatggtagt tgtattgggt tattgttattt tagttttgta gtaaattaga    3480
gtggtggtgg tttggaggtt attaaaggtg tagttatttt aagatttgtt gtgttgtttt    3540
agtaagtttt tattaatgat agagatttta aattggtttt ttgatttttt ggaaaattgt    3600
atttttttat ttttgtagtt ttttttttgg ttgtattgtg tgttaatttt ttatttttaa    3660
attggatttg ttaatttttt ttttttaatt ttttgtagtt tttgattttt atttgatata    3720
tttaataaat gttattattt aagatttagt tattttttttt aatttttgga tagtttattt    3780
tgatgtattg tatattttttt ttaaatgggt tgggggagag gatattgtga tggttttttt    3840
tgatt                                                                3845
```

<210> 242
<211> 3845
<212> DNA
<213> Artificial Sequence

<220>
<223> chemically treated genomic DNA (Homo sapiens)

<400> 242

```
agttgggaag agttattatg atgttttttt ttttaattta tttaaggaaa gtatgtagtg      60
tattagaatg gattatttag agattaagaa ggataattga attttaaatg gtgatattta     120
ttaaatgtgt tgaatggaaa ttaaagatta tagggagtta aaaaaaaggg gttaataagt     180
ttagtttgaa aatagagaat tagtgtataa tataattaag agaggaatta taaaaatgaa     240
aaggtatgat tttttaggga gttagaaaat taatttaaaa tttttattat taatggaggt     300
ttgttgaaat agtatagtag gttttggggt ggttgtattt ttgatggttt ttaggttgtt     360
attattttag tttattgtag agttgaaaag tagtagttta gtgtagttgt tattttatat     420
aggtggaggt ggtataaatg tttggttttt agttatattt ttttttagtt ttttattgtt     480
gtattgtggg taaggggtgg ttgttaagtt tgttaggtgg agtttgtgtt aatttatttt     540
tttttttttt tttttattgt gttataattg gaaaggaata gttgttgtat tagttaggtg     600
aaggggaggg ttgttatttt ttttaggtaa aagttgtgta agtttttgtt tttttgtttt     660
atgttttgtt ttattgtaag tggaggtgat tgttattttt gttgtgtttg tgtaatttgt     720
attttttttt aagtagtatt ttggggaggg ggtagttgtt tattttggtt ggtttttttt     780
ttttattatt gtattttaaa gagggtatgg ttgaaatttt gaaatagtag aagttgaata     840
tatattattt gtttttttttt tattggtggt tttataggga ggaagtggtt attttttttgt   900
tgaagtgttt gtgtagattt aagttgttgt agtggtggtt attttgaagg agttgtataa     960
tggtagtgat gtaatataga tttttaggtt attttgatat agtttttttaa gatttggttt   1020
ttaatagaga gaaataatga aaaaaaagta atttttaggt attatttggt ttattagtta    1080
aaagatttgt aaatgtaagt tttgtatatt attttagttt ttttatgtta tagtttttaa    1140
agtgagggtt tttaaagggt atgtaaggat tttatgtatt tttgtagttt ttgtggtagt    1200
gtaggtattg tgtagtttta ataaaaaagt attattatta tagtaggtaa attagatgat    1260
tattgtaggt tataggaaaa ttaaaggttg tggattgtgt tattgttttt tttgatgttt    1320
ttttttttat atagtaatta tttagtgttt tttagttatt ttggatagtg ataggttttg    1380
tggttgttat gtttattgtt tttatgttat gtttattgtt gttatgttta ttgttgttaa    1440
agtttattat tgttatgttt atttgttgtt aatgtttatt gttgttaata tttattgttg    1500
ttgttttttt tttatttttt agttattttt tatggatttt ttttgtgttg tgattattaa    1560
tataattttt attattgtta tattgattta ttttttttggt ttattgttat agtttttttg   1620
ttttggttat tgtgatgaat ttttttttta gttgttttat gtattttgtt ttattatgta    1680
gtggttatta ttatatattt atttgtgttt aatatttttt aaatattgat tattttatgt    1740
atttttgttt tgttataatt attttaatat atttattttt gtttaaaatt tttatgtatt    1800
ggttttttta tgtattttttg ttatatggaa ttgtaattat tttgatgtat ttatttttat   1860
ttatgttttt tgtttattgt ggttattttg tatgttttta gttattattg ttttttttat    1920
tgtagatatt tgtaatagga tttgttgtga tattatagtt gggggtggat gggggatgtg    1980
ttttaatgtg agtggatagg atattattgg ggtagaatgg tataatagta agtttttgaa    2040
tattttggat ttggtttttt agaataaagg attttagggt ttaaattttg tttatttagt    2100
attttaagtt ttaaaaattt ggaatatttg atgaataaaa gtggttgttt tttaggttgt    2160
tttttgagag aagttattgg tatagttgat tttgtttgtt ttattgtgtt attgattgtt    2220
```

```
ttttagatag atgtgttagg tatttttggt ggttgtttta ttttgtgtta gatttgttgt    2280
agtagtggta ggttttgtat atatttttgt ttgagtatgt gtgttagttt gtttttgtgg    2340
ttgtgtaggt gtgtttgttt gttgtagtgt aggggtttta gttttttttt ttattggaat    2400
gatttggggg gagggggtta ttggattttt agggttttat agtattgttg taatgagagg    2460
gggtttttag aaattataag taatttggga ttaatggata tgtttatttg tttttttaaaa    2520
tgtgtaaaat attaaagaaa taatttttgg ttttataata aatttttgta tatattattg    2580
gttaaataat tttaatttat atttattttt aggttttttt atgtgtggta taaaaaagtt    2640
ggtttggaaa agttaaatgt gttataatta taattaatgt aaatatgtta gatttgaaat    2700
ttttttaaaa gaaaatattt ttaattttaa aagtatgtta tattgtttaa atttggtgtg    2760
atttttttat ttttttttta ttttttatat tttgaggggg taaattgtga aaattatttt    2820
ttttaaaatt ataatataat taaatttttt tatatggaat ggatagatta tttaatttaa    2880
aaatattggt tatttaattt ttgtaaaata aaaatatgaa tttagatttt tatttaagat    2940
ttaagtagga gagttttttt ttgtttatta gaattggata tattaaaatt tttattaata    3000
attaatgata gttggagtgg ggagaaatag gtatttttat ataatattaa tggataagta    3060
aatattattt atgatataat attttatttg tagaaataat ggaagtatta gtaattttaa    3120
aatttagatg ttgatgtagt ttgggaaaag atattgtaga aaagaagaaa ataattttga    3180
aattgtattt gtatagtttt tatatatatt tgtttttttt taattgttta ttattgaaga    3240
agggagggtg atgtgagtgg aggttgatta aatggttaaa ttttttttta agttttgtaa    3300
gttattgttt agtttttaat gattttatat taaaataggt gtgtagaggt ttattaatgt    3360
tgatttttat tgtaataaaa ttttagaaat tgtgtaatgt tttttataga gaatagatag    3420
gttaattaaa taaatgtata tataaataat aatatgtaat atggttgatg gaaaaaatga    3480
tgtattaggt tgaattatat aaaattgtta atattgaatt gttttgattt ataaaaatat    3540
tgtaattttt aatagtttat tgatatatat ttatataaaa taatatggga agttgatttt    3600
gattatggga gttaattgaa aaataaaatg taggttgggt atagtggttt atgtttgtat    3660
ttttagtatt ttgggaggtt gaggtgggta gattatgagg ttaggagatt gagattattt    3720
tggttaatat ggtgaaattt atttttataa aaaatagaaa aattagttgg gtgtgatggt    3780
atatgtttgt aattttagtt atttgggagg ttgaggtagg agaattgttt gaatgtggga    3840
ggtgg                                                                3845
```

<210> 243
<211> 18463
<212> DNA
<213> Artificial Sequence

<220>
<223> chemically treated genomic DNA (Homo sapiens)

<400> 243

```
tttgagtagg tgtgtatttt gtagataggt ttagtgtttt ttgtttgtta ggtgttgagt      60
tggatggtag gaatatattt tattgggtaa gatttggtgt ttagtttttt aaggtattag     120
ttattgggtt gaaagagaga aataagttaa aaaatggaaa aattagttag aagtttagta     180
tatgttttaa ggaagttagt aggttgtagt gtgagagaat tagtaaaagt gtatttgatt     240
tagatagggg tgttggaagt agttttttaa aagtggtgtt gtttttgaaa tgtagtttag     300
gatttgtatt tatgtgtagg aagttttata gtatgagagg tttatatagg gataaggtgt     360
taagggggat tgaggggtat gaggggattg agattatttt tggtttgtat tgttgttgtg     420
gtttgggttt tggtgattta ggagtaggga gaatagagag ttttgtttat atttgagata     480
ttagagggag agatgttgta ttagtttgtg tttatgttgt tgataaagat atatttgaga     540
ttgggtaatt tattaaagaa agaggtttaa tggatttaaa tagttttatt tgtgggggaa     600
ggttttataa ttatggtaga aggtaggagg agtaagttaa gtttttatatg gatggtagta     660
ggtaaagaga gagggagttt gtgtagggaa attttttttt ataaaattgt tagattttgt     720
gagatttatt tattattatg agaatggtat ggaaaagatt tgtttttatg atttaattat     780
tttttattag gtttttttta tagtatgtgg gaatttaaga tgagatgtgg gtggggatgt     840
agttaaattg tattagattt ttaaaggtta gatttagtag gtgttgttat agtaaagaga     900
attgttgggt atagtggttt gatatgggtg ggagttttgg tttgagtttt ggatatggaa     960
gggatttaga ggaggttttg gttgtttgtt ggtgttgaag gttgagattt ggaaaggtga    1020
tggggggtgt aggtaaggta gaaaggaaat ttttaggttt aggtagaagt attatgttgt    1080
ttatattttt ttgggaagtg gtatttgtgg gggtggttag taagattttt tggttatggg    1140
atttttaaga tgtatttttt tatgttgttt ttttagatat tttggttatt ttgttgattg    1200
atgtattttt gttgttataa gaaaaagatt agaaatatgt ttttgttttt gtggtatgta    1260
ttttgttttt ttagatgaag ggttggttgg gtgttgtggt ttatgtttat aattttagta    1320
```

```
ttttgggagg ttgaggtggg tagattattt gaggttaaga gttgaagttt agtttggtta    1380
atatggtgaa attttatttt tattaaaaat ataaaaatta gttgggtatg gtggtttatg    1440
tttgtagttt tagttattta ggaggttgag gtaggataat tatttgaatt taggaggtga    1500
aggttgtagt gagttgagat tgtgttattg ttttttagta agattgtgtt attgtttttt    1560
tgtttgggta atagagtgag attttatttt aaaataaaaa aataaaataa ataaataaaa    1620
aatagatgaa gggttttgtt tgttgtgatt ttgttgggag gttggttgaa attgtttatg    1680
gaaggggtat ttgtgagttt tttaggttta tatagttggg attataggtg tttgttatta    1740
tgtttggtta attttttgta tttttagtag agatggggtt ttattatgtt ggttaggttg    1800
gtttttattt tttaatttta agtgattttt ttgttttggt tttttaaagt gatgggatta    1860
tagttgtgtg ttattgtgtt tagttgttgg tgtgttttta attagtgtat ttgtttgttg    1920
aggttgttgt aaataaagta ttatggattg ggtggtttag attatataaa tttattgttt    1980
tatgtttttg taggttgaaa gtttaagatg aaggtattag tagggttggt tttttttgag    2040
gtttttttggt ttgtaggtag ttatgtgttt atttgttttt tgtgtttttg agtggttatt    2100
tttttgtgtg tgtttttgtg ttaatttttt tttgttagaa ggatgttagt tagattggat    2160
ttgggtttgt ttatatgatt ttattttgtt ataattattt tttttttttt tttttttttt    2220
tttttttttt gagatagttt tattttgttg tttaggttgg agtgtgtaat gatataattt    2280
tgatttattg taatttttat tttttgagtt taagtgattt ttttgtttta gtttttttgag    2340
tagttgggat tataggtatt tgttgttata tttggttatt ttttgtattt ttagtagaga    2400
tggggtttta ttatgttggt taggttggtt ttgaattttt gattttaagt gatttgtttg    2460
ttttagtttt ttaaagtgtt gggattatag gagtgagtta ttatatttgt atattaatta    2520
tttttttaaa ggttttattt ttaaagatag ttatattttg aggtgttagg gtttaggatt    2580
ttatatgaat ttgtggggaa tatagtgtag tttattttag ttagttaaaa tgtgtatttt    2640
gttggtttgt ggaagtattg tttttttgaag gatgtgtgat agattagaaa tattggtggt    2700
tttgggtggt ttgaggtata agatagagat gggtttattt gttgttatat attatatatt    2760
gttgtatttt ttgagttttt ttgtgtgtat gtgtattgtt gattatttaa aaaaaaattt    2820
ttaagttgat gaaattgata tattattggg atattaaggg gtaagatggt ttatttttgt    2880
tattttagta ttttgggagg ttgagatagg aggattattt gagtttagga ttttaagatt    2940
agtttgggta atatagtgag attttatttt tataaaaaat ataaaaatta gttgggtttg    3000
gtggtatgtg tttataattt tagttattta ggaggttgag gtgggaggat tatttgagtt    3060
taggaggtta aggttgtagg gaataatgat tgtattattg tattatagtt tgggtgatag    3120
agtaagattt ggtttttaag atagtttaaa aaaaaaaaaa aaaaaaaaaa ggtttttagg    3180
attttattgt aatagtattt tttttttttt agatggtaaa atatttttgt tttgaattgt    3240
ttgaaaagaa atgaggagtg tgattttttat ttttagtttt ttttattagg tttttgattt    3300
ttttgttgtt tgggtgttgt ggggtaaagg gtgatttttt taatgttttt tatttatgta    3360
ggatttaaag ttatttgtta ttttgttata aaagtttatt gtgagggaag tggttaatga    3420
ggagaaagtg atgtttttga ttagtgtttt tttgtaaggg ttggagatgt atgaaattta    3480
tatgagtttt aaagaggata ggaatgtttg gatggtttat atttaaaggg ttgtggagag    3540
gtgaggaggg tttgggggtt tttttattta ttgtgttttt tttgttgatt ggtttatttt    3600
tggttttgat tgttggttat tagttgtgat tttgaatttt tttattttag tttgggattt    3660
atgatgttat tttagttttt tttgatgatt taatttgttg gatgttgagg ttaatgggtt    3720
tagaaaggta gttgtaattt gggttttttt gtatgtgtgg gttattgtat ttgttatggt    3780
gggtttttttg tattttaggg ttagatttgt tggagtaggg tatggtattt tgtttttttt    3840
ttttgggttg agtatttttt agggtttaga attagttggt tttttttttt ttttttttatt    3900
ttttattttt ttgagataga gttttgtttt gttatttagg ttggagtgta gtgatgtaat    3960
tttggtttat tgtagttttt gtttttttagg tttaaatgat tttgttgttt tagttttttt    4020
gagtagttgg gattataggt gtgagttatt gtgtttagtt tattttgttt tgttttgttt    4080
tgtttttttag agtttggttt ttgttttatt atttaggttg gagtgtgttg gtataattat    4140
agtttattgt aatttaaatt tttgggttta agttatttta ttttttttggt tttttaagta    4200
gtttgaatta taggtatata ttattatatt tagttagatt ttttattttt gtagagatgg    4260
ggttttattg tgttgtttag gttggttttg aatttttggt tttaagttat ttttttgttt    4320
tggtttttta aagtgttggg attataggtg tgagttatta tgtttgttta tttttttttt    4380
tgatttttgg aggttttttg gtgttttaat ttggtttaga ttttttttatt tatagtagtt    4440
gtagttttat ggggtttgta gttatttatt tgtatttggt aggttttttg taagtttgtt    4500
gttttttgga tttttttttt aagttatagg taggtatgta aagttttttt ttggttatgt    4560
tttggtgttt tggggtggga tgaatgttag tggttttttt tttttttttt tttgtttttt    4620
tgtattatga ataagttaaa ttatttttaa agttagagat ggttgtggtg gttggttttg    4680
tagaattagt gtttgtgttg aggttggttt gtttgggaag tatagttatt tatttgattt    4740
tttttttgt agttgttttg atgaggagga ggggtttttt agtttgtttg aagaggaaag    4800
gaaggtggtt gaggtttgtg ttatgagatt ttgggatttt taaggtgagt gggagatagt    4860
gtttgggtat attttttgtg tggtgagttt tgggtttttt attaggattg gttatagagg    4920
gtgaattgtt ttttaggtga tttggtgttt ttttgtgggt tgtgttatgt tttgtagttt    4980
```

```
tgtgaagtat tagaatgggt tgtgttatga tatagtgatt tttagaggtt atttagtttt    5040
ttgggggtat gtgggagagg gagggaggag agtttagagg atttgagttt tttttagagt    5100
tgattatata gttatatatg ttattattat tttattttg tgtagttagg agtttaatag    5160
ggtattttag ttttatatga aggaatagta tagggttttt atagtttttt tttagagttt    5220
tttttttttt gtttaattat atgattgaga tgtaatttat atattatagt atttattttt    5280
ttaagtgtgg ggtttagggg tattttagtg tatttttaat tatgtagtta gaattatgtt    5340
aattttagaa tattttatta ttttagaaag aagttttgtt ttttttaata gttatttttt    5400
attttatgtt ttaattttgg taataggaat ttattttttg tttttgtaga ttggtttgtt    5460
ttggatattt tatataaatg gagttatatt gtgtggtttt ttgggtttgg ttttttttga    5520
ggtgtggtat taaggtttgt ttgtgtttag tgagtatttt gttaagaata atttttggtg    5580
ttttgtagtt tgtggtaagt tttttttttt tttttattg tttgtatttt tttttatttt    5640
attttttttt tttttttaa atagagtttt gttttgttat taggttggaa tgtagtggtg    5700
tgattttggt ttattgtaag ttttgtttat taggtttaag agattttttt tttttagttt    5760
tttgagtagt tgggattata ggtatttgtt attatgtttt attaattttt gtgtttttag    5820
tagagatggg gttttattat gttggttagg atggttttaa ttttttgatt ttgtgatttg    5880
tttgttttgg tttttaaag tgttgggatt ataggtgtga gttattgttt ttggttttgt    5940
attatttatt tttaaattgg ataatattta aataataagt ggtttggtat ggtggtttat    6000
gtttgtaatt tttgtatttt gggaggttga ggtagatgga ttattggaag ttaggagttt    6060
gagattagtt tggataatat ggtaaaattt tgtttttata aaaaatttaa aaattaggtt    6120
gggtgtggtg gtttatgttt gtaattttag tattttggga ggttaaagta ggtggattat    6180
ttgagattag gagtttgaga ttagtttggt taagatggta aaattttgtt tttattaaaa    6240
atataaaaaa ttagttaggt atggtggtgt gtgtttatag ttttagttat ttgggaggtt    6300
gaggtaggag aattgtttga atttgggagg tggaggttgt agtgagttga gattgtgtta    6360
ttgtatttta gtttgggtga tagagtgaga ttttttttta aaagaagaag gaaggaaaga    6420
aagaaaggga ggatggaaaa tttagttttt taaggtttta agttggtttt agattaataa    6480
atgggtttga tataattttt atttatattt tattgtgttt gattttattt tatttttttt    6540
agagatagga ttttgttttg ttggttaggt tggagtatag tattgtagtt atagtttatt    6600
gtagttttga atttttgggt ttaagtgatt tttttgtttt attttttagg tatgtattat    6660
tataattagt taattttta tttgtagaga tagagtttta ttatgttgtt taggttggtt    6720
ttgaatttta ggttgtaaga gtttttttat tttggttttt ttaagtattg ggattgtagg    6780
tttgagttat tatagttaat ttgattttta taataagggg gaaaataatt agaggataag    6840
gttatgtttt tttttatttt ttaggaatgt aggatataag gggggtagttt attttagggt    6900
agggttagtg gggtttttta tttttaggtt agtttttggg gtttagatga ttttagggaa    6960
ggttgatttg gttgattgtt atttttatta ttattttgta gagtggttga gtatgaaaga    7020
ttagttgatt gtatagagtt ttttagagaa atagtagatt tatttggaga tggttgagat    7080
gggtggtttt gaagatttgt tttagttttg aggtttattt tgtggagggg atttatttga    7140
gattttgtag ggggagttaa tttttaagtt ggttatgagt gagagtaagt tggttgttta    7200
tattttaagg gtgtagtttt gttggggtgg gttttttagg gaattttagg ttaggtttat    7260
ggtttattgt ggttgatatg gtattttgtt taggataggt ttttatgtgg gggggggttta    7320
ggagtagtat tgattgtttt ttggtgtttg tgagagttag aagggtttta gatataattt    7380
gggttaagtt gttttttgta gatataaagt attttttttt tgtttaataa ttgttttgat    7440
gtttttgta tgtagaggtt ttaagttggg ttgtggggag gatttgaagt tgattaagat    7500
tggtttttta tttattagag gttttttttt tttttgggag gtgggtttg gtgatatttt     7560
tagaaaaata tatgtatatt tttttagtgg gtttttatgt atagttttaa tatagttgtt    7620
aggtttttgg gttggttgtt tgagtaggta gttagtgttt agtttgtttt tttttttttt    7680
tttttttttt tttttttttt ttttgagatg gagttttgtt ttgttattta ggttggagtg    7740
tagtggtgtg attttggttt attgtaattt ttattttttg ggtttaagta attttgtttt    7800
agttttttgg gtagttggga ttataggtat ttattattat gtttagttaa tttttgtatt    7860
ttttgtagag atggggtttt attatgttgg ttaggttggt tttgaatttt tgattttaga    7920
tgatttattt ttttttgttt tttaaggtgt tgggattata ggtgtgagtt attgtgtttt    7980
gttttaattt gttttttttaa tagtgaggga agttgattta gtttttgttt tttagagttt    8040
ttttgtagaa taagatggta gagatggggg ttagtttttt tttttttagg tttattgata    8100
ttttttttatt tggttttagt ggttattgtt tttggttttt tggggatatt tttatatttt    8160
gtattttggt ttggaatgtt tttgttttta tttttgtgtt tggttatagt ttgttggttt    8220
tagagggttt tttgtagata gttttttttt ataaagttat ttttttatat ggttattgtt    8280
atataattag tgtatggtta ttgttatata atatattaag tgttttttgt tggttttggg    8340
tagggttata tttagtattt gttagttttt ggtttagggt ggtttggtgg aaggagttgt    8400
aggagtttgt atttgttttt tttatggttg ttttatagtt ttgttttttt atttagatgt    8460
agattggttt gttttggtgg ttatatttga agtaagggtg tgtgtaggga tagtggggtt    8520
gaaattttat ggtatataga agggggtagg tgattattat tttagtgagt ttttttgttt    8580
atttgggttt tgttgtttta gtgttttgta ttttttttagt atatttttgt agggtgattt    8640
```

```
gtgtttttg ttttttttt tttatagttg agggtattta gagtttgatt tgtaggtagt 8700
tgggtagtgt taatggttag gtggaagatg gaggtagttt tataggtttg tttaggaggg 8760
ttgagatttt tgtgggttat gattgtataa atagttttat taagagtaag agtggggttg 8820
tttttttttt tgtttttagg gttgtttttt aggatgtata ttaatttgta tggggttttt 8880
tagaattttt ttttttgttg ttgttgagat agagttttgt tttgttattt aggttggagt 8940
gtagtggtgt aattttagtt ttttgtaatt tttgtttttt gggtttaagt gatttttttg 9000
ttttagttta ttaagtagtt gggattataa gtatatgtta ttatgtttgg ttattttttg 9060
tatttttagt agaaatgggg ttttattatg ttggttaggt tggttttgaa tttttgattt 9120
taggtgattt gtttgtttta gtttttttaaa gtgttgagat tgtaggtgtg agttattgta 9180

tttagttgat tttttggaat tttttgaaat tttttagata ttgttggttg tgttgtgata 9240
gtagtattta gattagtttg ttttttttta gtaggtgttt gtgttgtaga tgttattatt 9300
tgggtttagt ttgggttttt tatttgggta ggggttgggg gtggattggg aagttttttt 9360
tttttttatgg gatggtagta tttttatgtt tttggtttgg ggtttttaga tggtagtttt 9420
aagaagaaag ttagtagtat tgattttagg ttttgagatt ggtgaggttt tttaaatagt 9480
ttggatttga agtttagtga tagtgatatt tttgggagtt ttgaggaatt gttgtaggtg 9540
gtatgtggat atttatttgt ttggtatagt ttgagttgtt ataaggattt atgtgtgtga 9600
gtagtttttg ttggggttgt ttagttttag ttttattagt attgataagt attgtttgtt 9660
ttgtggtgaa taatagtatt ttagtttaga ttaatgtatt ggtaggtgat tttttttttgt 9720
agttttttgg gaaaagtaaa gaggttttta agaggaaggt tggggagggg ataggtttag 9780
ggtatttatt atggttttat taataggtga ggaatttttt atagggatta ttagttttta 9840
gttggggtag gtgtggtggt gtttattaga tgttagatgt ttttggttat ttttttgttt 9900
ttgaattaga aggttaggtg tttatttggt tttgtttagg ttagggggtt agaggttaag 9960
gttagttgtt tttgattagt tttgaggttt ggatttgttt ttgtggttag agttggtatg 10020
taggttttat agttaagttt attttatttt ttaggaggtt ttagtttttt gtttttttaaa 10080
tatttagatt tgtataagtt agttgattag tagggtttgt gtttttagtt atgatttggt 10140
tttgggtttt ttttgttatt tatattaagg gatgtatttt taataataag taggggttgg 10200
gtgtagtgtt ttatatttat aattttagtg ttttgggaga ttgaggtggg aggattattt 10260
tttttttttt tttttggaga taaggtttta ttttgttatt taggtttgag ggtagtggtg 10320
tgattatagt ttatatagtt tttaattttt aggtttaagt aattttttta ttttagtttt 10380
ttaagtagtt gaaattatag gtgtgtgttt ttatatttga ttaatttttt aaatgtttgg 10440
tagatatagg gttttattat gttgtttagg ttggttttta atttttgggt ttaagtaatt 10500
tttttgtttg ggttttttaa agtgttggga ttataggtaa ggttgttata tttggttttg 10560
ggaggattat ttgaggttat gagtttgaga ttagtttggg taatatagta agattatatg 10620
ttgataaaat atttttaaaa tagttgattt tttggttggg tgtggtggtt tatgtttgta 10680
attttagtat tttaggaggt tgatttgggt gattatttga gtttagggtt tgagattagt 10740
ttggttaata tggtgaaatt ttgtttttat taaaaatata aaaattagtt ggatgtggtg 10800
gtggatattt gtaattttag ttatttagga ggttaaggtg ggagaattgt ttgaatttag 10860
gaggtggagg ttgtagtgag ttgagattgt attattgtat tttagtttgg gtgattaagt 10920
aagattttgt tttaaaaaag ttaaaaatag ttgttgggtg tggtggttta tgtttgtaat 10980
tttagtattt tgggaagtta aggtaggtgg attatttgag gttaggaatt tgagattagt 11040
ttgattaata tggtgaaatt ttgtttttat taaatatata aaattagtta ggtgtggtgg 11100
tatatttgag taattttagt tatttgggag gttgaggtag gagaattatt tgaattagga 11160
aggtggtggt tttagtgagt tgagattgtg ttattgtatt ttagtttggg taatgagtaa 11220
aattttattt taaaaaaaaa aaaaaaaaaa attaaaaata gttgattttg gtggtgtata 11280
tttgtagttt tagttattta ggaggttgag gtgggaggat taggttatta tattttagag 11340
taaggttttg ttttaaaaaa aaaaaaaaga agttaggtat ggtggtttat gtttgtaatt 11400
ttagtatttt gggaggttga ggtgggtgga ttatttgagg ttaggagttt gtaattagtt 11460
tggttaatat ggtgaaattt tgtttttatt aaaaatatat aaaattagtt aggtgtggag 11520
gtttgagttt ataattttaa ttatttggga ggttgaggta ggagaattgt ttgaatttgg 11580
gagttggagg gtgtagtgag ttaagattgt gttattgtat tttagtttgg gtaataagag 11640
tagaattttg ttttaaaaaa aaaaaaaaaa aaaaaagtta aaaaaaaaga agaagaagaa 11700
gaaggtttga gtttagttgg atgggttttg agttattttt tttggtttga tggtgttttt 11760
tttttaggtg gaggtgttag gtatggaatt tgatttttgt ttgtttattg ttttggagtt 11820
ggaggtaggt gtttgtgggt ttttattttt ttagggtttt gtgtatgtgt gtgtggtttt 11880
agtttgataa ttagtattaa tttttttttt ttttttgtag tttgtttagt ggatttagat 11940
attgttttag ttgtttttga atttttaggt ataggggtgg ggtggggttg gttatgtgtg 12000
tttttttttg gttggttggg gtgtaggtgt ggtttttatt tgtttggttt tggttttttg 12060
taggtggtaa ttgtttatta ggatagttat gtggagatgt agtgggttgt tatttaggag 12120
tgggagaagt agttttggtt gtagttgatg tgtgggaatt tgttgttgga gtaggagtgg 12180
taatgtaatt ttgagaagta gtgggaggag tgtgtggttt tggagaagtt gtagagttag 12240
```

ttgtggtatg agtagtagtg ttgggagtgt gagtgttagt ggtagtatta ggagttggag 12300
tgtgtgggtg tgtggttgta ggagtgtgag ggtgaggtgt ggtagttatg tgagtggttg 12360
gagtaggagt gggttgagtt ggagtgttag tgttaggttt attagtatga tttggagtgg 12420
ttgtgtgagg tttagtgtgt tgtggagtgt gagtgggagt gtttggagtt gttgtgttgt 12480
tttaagaagt agaatattgt gttaggtgtg ttgttgtttg atatattggt tgaggtgagt 12540
gtgtagtagt tagtgtgtgt aggttggggg tgattggttt gtatgtgtat ttgtatgagt 12600
gtatgtaggt gatagggttt gtgggtgtat gtgtgtagga gtgtaagagt aaatggtata 12660
agatattttt gtattaatag tttatgttgt agtatagatt ttttttggag ttggttaaat 12720
gtttaataga atgttgttgt ttaaaatatg ttataaatgt tagaaatttt ttaaaaaaaa 12780
aatgtatttt tgtaatttta gtattttggg tggtggaggt aggtggattg tttgagggta 12840
ggagttttag gttagtttgg gtaatatggt gaaattttat ttttataaaa aaatataaaa 12900
atgagttagg tatggtggta tgtatttgtg gtttttgtta tttggaatgt tgaggtggga 12960
ggattatttg agtttgggag aatgaggttg ttatgagttg tgattatgtt attgtatttt 13020
agtttgggta atagagtgag atatgagttg tgattatgtt attgtatttt agtttgggta 13080
atagagtgag attttgtttt aaaaaaaaaa aaaaaatgaa gttgggtgtt atggtgtgta 13140
ttttagttat ttgggaggtt gaggttggag gattgtttga ggttaggtat ttgagattag 13200
tttgggttat gtagtgagat tttattttta aaaaaaaaat gtaaaaatta gtagggtgtg 13260
gtggtatata tttgtaattt aaattatttg ggaggttgag gtagggggat tatttgagtt 13320
taagaggttt aagagttatg attgtgttat tgtattttag tttgggtgat agagtaagat 13380
tgtgtttttg aaaataaatt tataaattat gggttttgtg aatagatgtg gatatggatg 13440
tgtgagatgt ttgtatatga gtgtgtgata gtatgggtgt tattgagtgt tattataagt 13500
ttgtatattt agtaggggtt ggggatgggt aatgattagt agttttttta ggttgtattt 13560
ggttttgttt agaataggag aggttgaggg tttttttgtgt atgatttttg ggggtttttt 13620
agaggttgta tagtaggaat tttatattgg atgtatttgt tgttgttttt ttaggtttag 13680
tttttaagtt attttttttag ttttaatggg gaagggttgg agggtttttg tgtgagtatg 13740
ttgttatttg gtgtggggtt agagtatgta gagtgttttg aggtggtttg ttgggatagt 13800
gtttttattg agaattggtt ggttaagagt gatgtgttta tttagttgtt tagtgttatt 13860
aattagtttt agaggtaggt ggttgtgtag tagtagattt ttattaagtt ggtggttttt 13920
attaagggtg gtaaggataa gggtggtaag agtaggggtt tttagtgttg ggagagttta 13980
ggtgagttgg ttttattttt ttgtttgggt ttggaaggtg taattggtta ggttttagtg 14040
gtttgttggg agttaggaaa tttgggatat ttgtgttatt ttttggagag gaaaattaga 14100
aggtatagtt ggtattgtgg ttgaggttat tttatatagg gttgtgtttg gtttttaggt 14160
ttaaaattga gatgatttat tttgtatttt ttttatttta atttttaaaa tagtgggatg 14220
gggattagga gttttagaaa gggatatggg taagaaggta gagatttttg gttatgtggg 14280
attagtatag ttgggatagg gataggtgta ggtgtttttg tttattgttt tagttttata 14340
gagttgtttt atagagtggg atatggagtt tagagtagtt ttagtgtttt tatggaggat 14400
gtttaggagt ttttttgggg gatatttttt tgggggataa ttagggatgt tttgggatgt 14460
tttttgaaga ttaagggtgt gttttgtggt tattagggtt tgtgttattt ttataggagg 14520
tttagttttt tttttagatg ttgggtgatt ttgtaggttt ttttgtaggg aggttggggt 14580
tgtttttttt gaatttgggg tttagtattt gagtagttta ggttatggga tttaggttgt 14640
ttgtgggaag tgttaggtgg gggtggttag gtttttttgt ttttggaggt tgttttggtg 14700
ggtggggata gttggttagt ttggagttgg tttaaatatt tttttttttt tagtgttttt 14760
tgatttgaag tagtagttgt tgtttaataa gtttatgggg aaagatgaga gtattttatg 14820
gaattgttgt ttgttgagtt ttattttgtt tggtagatat agttttgtgt ttttattagg 14880
tgagttttta tttttgata tgagtttagt tttagggtag tggggttgtg gtattatttg 14940
gtgattgttt agtttgaatt tttttttgt tttagatttt ggtttttttg ttttgagttt 15000
attgttagtt gatagttttt ttgagggttt tttttttaag gttgggggta tagttttttt 15060
gtttgggttt ttagtttttt tgttattgtt ggttatatta tttagtgtta aggaggatgt 15120
tagtaaagaa gatgttattt ttttttaaaa gggttgtgat ttaaggtgta aggttttttt 15180
ttgttttgtt tatttttttt gtttttttggg gatttttatg gggttattat gtttatttag 15240
ttgttttttt tttttttta gtaaattatt gatgattgtt tggtaggggt tagtttgttg 15300
gtgttttggg ttttgtagtt gtttttgtag ggttagtggt ggtgttgggg ttatttttttg 15360
aatttttttt tttttttttt aaaaaggaaa gtttttaatg gaaagttgag ttagaattaa 15420
attagggagt tgtttgaaat tatagtattt tatttgggtg gtggggagat taattttgag 15480
ttgttttttt gaggtagtga ggaatggtgt tggttttgta tggagttgag gataggatag 15540
attttgtttt gagaaggagt tgttggttgg ggttatgttt tatagttgtt gtgtgatagt 15600
ggagttaagg gttagggtat taggaggggt taggtggtgt tggtagtatt tgtttttaga 15660
attaggtaga atttattttt taaatagagt tttatgtagg tttattatga attttagggt 15720
tagggaatga gttaggtatg ggggtatggg tagagagggt tatggggtag ggtttattga 15780
gggaatatta gtggtttttt agttaggttt tgtgggtttg gaagtttatt gtgaaagggg 15840
ttgatttttg tttttttta tttggtattg gttttgaaat tagttgtttt ttaaattttg 15900

```
tttttttaagg gtaattgttg ttgtttatat gtttagtttg tttgggggag tgggttttta   15960
gttttagtta gggtgggtat atattttggg tatagggttt ttagtttttg ggaaatgagt   16020
ttttagggtt ggtgttttat tttttaggtg ggggttggta tattatagat tgttgagagt   16080
gttatgtttt agggtatgta gaggatgtat ttagagatgt tgtagtaagt ggataagtgg   16140
ttgttgtgtg ggtttttttgt ttgtagtgag ttgttgtagt ttatggtttg ttttttggag   16200
gggtggagga aggaggtgtt gggtagtatt aaaggtgttg ggatatttta gggtggtgag   16260
atttatttat gatttagttt tggtggagaa agggtttatg ttaagttttg ttttgtattt   16320
taagtattgg tggtaggatt gggttttggt tgattgtttt tgtttttagt ggggtatatg   16380
tgagtttttg ttagggttaa gttttttttt tgaatttagg gttttgggaa ttgtagattt   16440
tggggggatt tagttttttt tttattgtgt tggtagaggt atttttgtga tgttgaatat   16500
agtgaatagg gatattttttg ttatttgggg atagatgggt ataagggagg ggaaatttta   16560
ttaggaagtg tttttttggg tagaggtgtt tattgggtgt tgtgggttta ggagggggtg   16620
gggtaggagt tggtgttaat tgggaattag agttttatag ttatatagtt tattggtgat   16680
aaggttttga gaatatagtg gttaggtgtt tttaggtttt tggttttttt gatgatttta   16740
atttgtttta gtttggtttt tggttattag tgatgttgtt tgtttttttgt tagattttat   16800
gtgtgttatg tttattatta gtgttttgta tttttgtatt gagtattgga gtattttata   16860
gaagttgatt gtatattttt gttttgttgt gaagagaata tttttagatt ttggtatttt   16920
tttgagttgg tgtgtgttgg tttagttttt tgagatgtta taattttttg gatgggggag   16980
aagtttaagg aatttttgtt tggttatgtg gtgggaattt tgtgtttttg ttatgtggta   17040
gaggggtttt agttgtgtta ggtattgggt ggtagtgttg atagtttttt ttttgttagt   17100
gtttttttggt tatttttggg ttggagtttg attttatttg taaagttgat agttgagtaa   17160
atgtttttat ttttgtggga tttgtatatg tttttgttag ttgtggttat gattttagtt   17220
atttgttaat tatttttata atgattataa tatttttttta ttgtgggata tttttgattt   17280
gttttagaat ttaagatttg atttagtaa taaatgtgtt tgagatgagt ggtgattggt   17340
gtgtatgttt ttggaggttt tatttgagga ttttttttttg tttaaatgtt gttgtaggag   17400
tagtaggttt tgttgggggt tttttatggt ttttgttttt tagtttgttt atttgttttt   17460
tgtgttttgt ttaatgtttt gaagattttt ttaggtttag tttttaggag ttgttggggg   17520
ttttgttttt gtttttgttt ttgtttttttt ggtttgtttg ttttttttgag atagagtttt   17580
gttttgttgt ttaggttgga gattagtggt aggtttttag tttattgtaa tttttgtttt   17640
ttgggtttaa gtattttttt tgttttagtt ttttgagtag ttgggattat aggtgtgtat   17700
tattatgttt agttaatttt tgtatttttt tagtagatat ggggtttttgt tatgttggtg   17760
aggttggttt tgaattttttg attttaagtg atttgtttgt tttagttttt taaagtgttg   17820
ggattatggg tatagggtag gagtggggtg tggaggtgtt tgtggagggt tttgtttatt   17880
gggtatattt taggttttgg tggtgttttt atgaaaattt ttttgttttt aatttataat   17940
aattgtaaag gttggaattt ttatttttttt tatttggggt gagaggtagt ttggttatga   18000
gtatgaaatt attattttta tttggtttga gttttggttt agtgaggtga ggattttgtt   18060
gttttaatag agggtttttt attataaggg gattaggtgt aaagttgatt attagtttat   18120
taggaagtgt aaaaagggaa tttttaggta gtattgttta ttaggaagat gatgtaggtt   18180
atgtgtggaa tttttttattt ttagtagtta tattaaaaaa gtaaaaggag ggttgggtat   18240
agtggtttat ttttgtaatt ttagtatttt ggggaggtgg gggttgaggt aggaggatag   18300
tttgagttta ggagtttaag attagtttgg gtaatatagt gaaatttttt tatagaaaaa   18360
ttaaaaaatt agttaggttg gttgggtgta gtggtttatg tgtgtaattt tagtattttg   18420
ggaagttaag gtgggtagat tatgaggtta ggagattgag att                       18463
```

<210> 244
<211> 18463
<212> DNA
<213> Artificial Sequence

<220>
<223> chemically treated genomic DNA (Homo sapiens)

<400> 244

```
ggttttgatt ttttgatttt gtaatttgtt tattttggtt ttttaaagtg ttgggattat      60
atgtgtgagt tattgtgttt ggttaatttg gttaattttt taattttttt gtgaaggggt     120
tttattatgt tgtttaggtt ggttttgaat ttttgggttt aagttatttt tttgttttag     180
tttttgtttt tttaaaatgt tgggattata gggatgagtt attgtgtttg gttttttttt     240
tatttttttta atatggttat taaaaatgaa aaattttata tgtggtttgt attattttttt     300
tggtggatag tgttgtttga gagttttttt tttgtgtttt ttagtgagtt agtgattgat     360
tttatattta gttttttttgt gatgggaaat tttttgttga aatagtaggg tttttgtttt     420
```

```
attggattgg gatttagatt aggtggaaat gatgatttta tatttatggt taagttgttt     480
tttattttag atggaggaag tgaaagtttt agtttttgtg gttattgtgg attaaaagta     540
aaaagatttt tgtggaggtg ttattaaagt ttgaagtgta tttggtgggt aggattttt      600
ataggtattt ttatgtttta tttttgtttt gtgtttgtaa ttttagtatt ttgggaggtt     660
gaggtgggta gattatttga ggttaggagt ttgagattag ttttgttaat atggtgaaat     720
tttatgttta ttaaaaaaat ataaaaatta gttgggtgtg gtggtgtata tttgtaattt     780
tagttattta ggaggttgag gtaggagaag tgtttgaatt tgggaggtag aggttgtagt     840
gagttgagaa tttgttattg gtttttagtt tgggtgatag agtaagattt tgttttaaaa     900
aaataaataa attaaaaaaa tagaaataaa aataaaaata aaatttttaa taatttttgg     960
ggattggatt tgaaggggtt tttagagtgt taagtaggat atggagggta ggtgagtgag    1020
ttgaggggta ggagttatgg ggagttttta atagaatttg ttatttttgt agtaatgttt    1080
gggtagaaga aaatttttaa ataaaatttt taagaatgtg tatgttagtt attgtttatt    1140
ttggatatgt ttattgttag aattaggttt taagttttaa agtgggttag aaatattttg    1200
tagtgaggaa atgttgtaat tattgtaaaa ataattagta ggtgattaag attatgatta    1260
taattgataa agatgtgtgt agattttatg aaaataggaa tatttgtttg attgttaatt    1320
ttataaataa aattgggttt taatttagga gtggttgagg ggtattggtg agggaagggt    1380
tgttggtgtt gttgtttgat gtttagtatg attgagattt ttttgttata tggtggggat    1440
gtggggtttt tattgtgtgg ttgagtagaa attttttgaa tttttttttt atttaaggaa    1500
ttgtggtatt ttggagggtt ggattggtat atgttggttt aggagggtgt tagggtttgg    1560
gaatgttttt tttataatag aataggaatg tatagttagt ttttgtaaag tgttttgata    1620
tttagtataa aaatataaaa tattgatgat aaatatggta tatatgggat ttgatggggg    1680
gtggatagtg ttgttgatgg ttagggattg ggttgggtag agttgaggtt gttggagggg    1740
ttaggagttt ggggatattt ggttattgtg tttttaggat tttgttatta atgggttgta    1800
tggttgtggg gttttggttt ttggttggta ttagtttttg ttttgttttt ttttgagttt    1860
atagtattta gtgggtgttt ttgtttaggg gagtattttt tgatggagtt tttttttttt    1920
tgtgtttatt tgtttttgag tggtgggaat gtttttgttt attgtattta gtgttatagg    1980
agtgtttttg ttagtatagt gggagaaggg ttgaattttt ttgggatttg tagtttttag    2040
gattttgggt ttgggagaag gatttggttt tggtaggggt ttatatgtat tttattggga    2100
ataaggatga ttagttagga tttagtttta ttattaatgt ttggggtgta gaataaggtt    2160
tgatatgggt ttttttttta ttggagttgg attgtggatg gattttatta ttttgggatg    2220
ttttagtatt tttgatgttg tttaatattt ttttttttta ttttttttagg gaatggattg   2280
taagttgtaa tagtttatta taagtgaggg gtttgtatag tggttatttg tttatttgtt    2340
gtaatgtttt taggtgtatt ttttgtatgt tttgggatat ggtgtttttg atagtttgtg    2400
atgtgttagt ttttatttgg aaggtgggat gttagttttg ggagtttatt ttttgggggt    2460
tgaggatttt gtgtttaggg tgtgtatttg ttttggttga agttagaggt ttgttttttt    2520
agataggttg agtgtgtgaa tagtaatggt tgtttttggg aagtagagtt tgggaaatag    2580
ttggttttaa aattaatgtt aagtaaaaag gggtaaaggt tagtttttttt tatgatgggt   2640
ttttaaattt atagaatttg attggagggt tattgatgtt tttttagtgg gttttgtttt    2700
gtggtttttt ttgtttatgt ttttgtgttt ggtttatttt ttgattttga ggtttatggt    2760
gaatttgtgt ggggttttgt ttgggaagtg gattttgttt gattttgggg atagatgttg    2820
ttgatgttat ttggtttttt ttggtgtttt aatttttggt tttattgttg tgtggtggtt    2880
gtggagtgtg gttttggttg gtagtttttt tttaaagtaa ggtttgtttt gtttttagtt    2940
ttgtgtagag ttggtattgt tttttattgt tttggaaaaa tagtttggag ttgatttttt    3000
tgttatttgg atggggtgtt atgattttag atagtttttt ggtttagttt tggtttaatt    3060
ttttattaaa aatttttttt tttgaaaaaa aaaaaaagag atttagaaag tgattttggt    3120
attattgtta attttataga aatagttgta aggtttagag tattgatagg ttggtttttg    3180
ttaggtggtt attagtggtt tgttagggaa gaggagggga tagttgggtg ggtatggtga    3240
ttttatgggg gtttttagag agtaggaagg gtgggtaggg tagggagggg ttttgtattt    3300
tgagttatgg tttttttaga agaagatgat gttttttttg ttggtgtttt ttttggtgtt    3360
gagtggtgtg gttggtagtg gtgagggagt tgggggtttg ggtaggaggg ttgtgttttt    3420
ggttttgaga gagaagtttt tggaggggtt gttagttggt ggtgggtttg gggtggggaa    3480
gttagggttt ggaatagaga gagggtttag attgagtagt tgttgggtgg tgttgtagtt    3540
ttgttgtttt gggattgggt ttatgttagg gggtgggggt ttatttggtg ggggtgtagg    3600
attgtgtttg ttgggtagga tagggtttag tgagtggtgg ttttgtgagg tgtttttatt    3660
tttttttatg agtttgttga gtagtagttg ttgttttagg ttgaaggatg ttggaagaga    3720
gaaggtattt gggttagttt taggttggtt agttgttttt atttgttagg gtagtttttg    3780
agagtagagg ggtttggtta tttttatttg atattttttta tgggtggttt ggattttgtg   3840
atttgagttg tttagatgtt aagttttaga tttagggaga atggttttaa ttttttttgtg   3900
gaaggatttg tagagttatt tggtatttgg agaggaggtt gagtttttttg tggaaatagt   3960
ataggtttta gtgattatgg gatatatttt taatttttag agaatatttt agaatatttt    4020
tggttgtttt ttagggaggt gttttttaga ggagtttttg gatatttttt gtggaaatat    4080
```

tggggttgtt ttgggttttg tgttttattt tgtggaatgg ttttgtggaa ttgggataat 4140
gagtagaggt gtttgtattt gtttttgttt tggttgtgtt gattttatgt ggttagggat 4200
ttttgttttt ttgtttgtgt tttttttaa ggtttttggt ttttatttta ttgttttgag 4260
ggttgaggtg gagaagatgt agggtggatt attttagttt tgggtttgga gattaaatat 4320
ggttttgtgt ggggtggttt tagttatgat gttagttgtg ttttttggtt ttttttttta 4380
gaggatggta taggtgtttt gaatttttg gtttttagtg agttgttaga gtttgattag 4440
ttgtattttt taggtttagg tgaaggggtg gggttggttt atttgagttt ttttagtgtt 4500
gagagttttt gtttttgttg tttttgtttt tgttattttt ggtggaggtt gttagtttgg 4560
tggggatttg ttgttgtatg gttgtttgtt tttggaattg gttggtggtg ttgagtagtt 4620
ggatgggtat attgtttttg gttagttggt ttttggtggg ggtgttgttt tggtgagtta 4680
ttttggggtg ttttgtgtat tttggtttta tgttggatgg tagtatgttt atatgagggt 4740
tttttagttt ttttttgttg aagttgggag ggtggtttgg gggttgggtt tgaggggata 4800
atagtagata tatttaatgt gaggttttg ttgtgtggtt tttggagagt ttttgagggt 4860
tgtgtatagg agatttttga tttttttgt tttggataga gttaggtgta gtttgaagga 4920
gttgttggtt attgtttgtt tttaattttt gttaaatgtg tagatttgtg gtgatattta 4980
gtggtattta tattgttata tatttatata taaatatttt atatatttgt gtttatattt 5040
gtttatagaa tttatggttt atagatttgt ttttagagat atggttttgt tttgttattt 5100
aggttagaat gtagtggtat aattatagtt tttgagtttt ttgggtttaa atgatttttt 5160
tgttttagtt ttttaagtag tttggattat aggtatgtgt tattatgttt tgttaatttt 5220
tgtatttttt ttttagagat gaggttttat tatgtggttt aggttggttt taaatattta 5280
gttttaagta atttttggt tttagttttt tgagtagtta gggtgtatat tatggtattt 5340
agttttattt tttttttttt tttgagatgg ggttttattt tgttgtttag gttggagtgt 5400
agtggtatga ttatggttta tgttttattt tgttgtttag gttggagtgt agtggtgtga 5460
ttatggttta tggtagtttt attttttgg gtttaagtga tttttttatt ttagtatttt 5520
aagtagtggg gattataggt gtatgttatt atgtttagtt tatttttgta ttttttgta 5580
gagatggagt tttgttatgt tatttaggtt ggtttggaat ttttgttttt aagtaatttg 5640
tttgttttta ttatttaaag tgttgggatt ataggagtgt atttttttt taagaagttt 5700
ttaatattta tggtatgttt tgagtaatag tgttttgtta agtatttagt tagttttgag 5760
aaaggtttgt attgtggtat gagttgttaa tgtaagggta ttttgtgttg tttgttttta 5820
tatttttgta tgtatgtatt tgtgaatttt gttatttgta tgtatttgtg taaatgtatg 5880
tgtaaattgg ttatttttaa tttgtgtata ttggttgttg tgtgtttatt ttggttagtg 5940
tgttgggtgg tagtgtgttt ggtgtggtgt tttgtttttt gaggtggtgt agtagtttta 6000
ggtgtttttg tttgtgtttt atggtatgtt gggttttgtg tagttgtttt aggttgtgtt 6060
ggtaggtttg gtgttggtgt tttagtttgg tttgtttttg ttttagttgt ttgtgtagtt 6120
gttgtgtttt gtttttgtgt ttttgtagtt gtgtgtttgt atgttttagt ttttggtgtt 6180
gttattggtg tttgtgtttt tagtgttgtt gtttgtgttg tagttggttt tgtagttttt 6240
ttagggttgt gtgttttttt tgttgttttt tgaagttgtg ttgttgtttt tgttttagta 6300
gtaggttttt atgtgttgat tgtagttgga attgtttttt ttgtttttgg atggtagttt 6360
gttgtgtttt tatatagttg ttttggtggg tgattattgt ttgtggaggg ttagggttag 6420
gtgagtgaga gttgtatttg tgttttagtt aattaggaaa gggtatgtgt ggttggtttt 6480
atttttgtttt tgtatttgaa ggtttaggag tagttgggat agtgtttgga tttgttggat 6540
aagttgtgga gaggagaggg agattgatgt tagttattgg gttgaggtta tatgtgtgtg 6600
tataaggttt tggggagatg gagatttgtg ggtgtttatt tttgatttta ggatggtggg 6660
tagatgggga ttggattttg tgtttggtgt ttttatttgg gaagaggata ttgttaaatt 6720
agagagagtg gtttaagatt tatttgatta gatttaagtt tttttttttt tttttttttt 6780
ttttaatttt tttttttttt ttttttttttg agatggaatt ttgtttttgt tgtttaagtt 6840
ggagtgtagt ggtgtgattt tggtttattg tattttttag tttttgggtt taagtaattt 6900
ttttgtttta atttttgag taattgggat tataggttta agtttttatg tttggttaat 6960
tttgtgtatt tttagtagaa atggagtttt attatgttgg ttaggttggt tgtaaatttt 7020
tgattttagg tgatttgttt gttttggttt tttaaagtgt tgggattata ggtatgagtt 7080
attatgtttg gttttttttt ttttttttttg agatagggtt ttgttttaga gtgtagtggt 7140
ttgatttttt tattttggtt ttttgagtag ttgggattat aggtgtgtat tattaggatt 7200
agttattttt aatttttttt tttttttttt ttgagatgga gttttgtttg ttgtttaggt 7260
tggagtgtaa tggtatgatt ttagtttatt gaaattatta tttttttggt ttaagtgatt 7320
tttttgtttt agtttttttaa atagttggga ttatttgaat gtgttattat gtttggttaa 7380

ttttgtatat ttggtagagg tggggtttta ttatgttggt taggttggtt ttgaattttt 7440
gattttaggt gatttatttg ttttggtttt ttaaagtgtt gggattatag gtgtgagtta 7500
ttatatttag tagttatttt taattttttt gaaataaagt tttgtttggt tgtttaggtt 7560
agagtgtagt ggtgtaattt tggtttattg taatttttat tttttaggtt taagtgattt 7620
ttttatttta gtttttttgag tagttgggat tataggtatt tattattatg tttagttaat 7680

```
ttttatattt ttagtagaga tagggtttta ttatgttggt taggttggtt ttaaattttg    7740
gatttaagtg attatttgag ttggtttttt gaagtgttgg gattataggt atgagttatt    7800
gtatttagtt aaaggattag ttattttaaa aatattttgt tgatatgtgg ttttgttatg    7860
ttgtttaggt tggttttaaa tttatggttt taagtgattt ttttaaggtt aggtatggtg    7920
gttttgttta taattttaat attttgggag gtttaggtag gaggattgtt tgagtttagg    7980
agttggaaat tagtttgggt aatatagtga gattttgtat ttattaaata tttaaaaaat    8040
tagttaggtg tgggggtgtg tatttgtagt tttagttatt tgggaggttg aggtgggagg    8100
attgtttgag tttaggagtt agaggttgtg tgagttgtga ttatgttatt gtttttaagt    8160
ttgggtgata gagtgagatt ttgtttttaa aaaaaaaaaa aaaaagtgat tttttttattt    8220
tggtttttta aaatattggg attatgggtg tgaggtattg tatttggttt ttgtttatta    8280
ttgaaaatat atttttttagt gtggataata ggaaggattt aggattgggt tatgattggg    8340
aatataggtt ttgttggtta gttgatttgt ataaatttgg gtgtttggaa aatgaggggt    8400
tggggttttt tgggaggtag agtgggtttg gttatgaggt ttgtgtgtta gttttggtta    8460
taggggtaag tttaagtttt agaattgatt agaggtaatt ggttttggtt tttgattttt    8520
tgatttgggt agggttgagt aggtatttgg ttttttgatt taaaggtaaa ggaatagttg    8580
gggatatttg gtatttggta ggtattatta tatttgtttt ggttgaaagt tggtggtttt    8640
tgtagggggt tttttgtttg ttgatgaagt tatggtgagt gttttgagtt tgtttttttt    8700
ttagtttttt ttttggggat ttttttgttt tttttaggag gttgtaggaa gaaattattt    8760
gttagtgtat taatttgggt tgagatattg ttatttatta taaaataaat aatgtttgtt    8820
gatgttaata agattaaaat tggataattt tagtagaagt tgtttatata tatgaatttt    8880
tatgatgatt tagattgtat tgagtaaatg gatatttatg tattatttgt ggtgattttt    8940
tagagttttt aggaatgtta ttgttattga gttttaagtt tgggttgttt gggggggtttt    9000
gttagttttg gggtttgggg ttagtgttgt tgattttttt tttgaaattg ttatttgaga    9060
attttaagtt aggggtatga ggatgttgtt attttgtggg gagggggaag ttttttagtt    9120
tatttttagt ttttgtttag gtaggaaatt taggttaggt ttgaatggtg gtgtttgtag    9180
tatagatgtt tgttggagag gggtaggttg gtttgggtgt tgttgttata atataattaa    9240
tgatgtttgg agggttttag aaggttttag aaaattggtt gggtgtggtg gtttatattt    9300
gtaattttag tattttggga ggttgaggtg ggtggattat ttgaagttag gagtttgaga    9360
ttagtttggt taatatggtg aaatttttatt tttattaaaa atataaaaag tagttgggta    9420
tggtggtgtg tgtttatagt tttagttatt tggtaggttg aggtgggaga attatttgaa    9480
tttaggaggt agaggttgta gagagttgag attgtgttat tgtattttag tttgggtgat    9540
agagtaagat tttgttttaa taataataaa aaagaaagtt ttaggaaatt ttatataagt    9600
taatgtgtat tttgaggggt ggttttggag gtaggagggg agatggtttt gtttttattt    9660
ttggtggggt tgtttgtgta gttgtagttt gtgaaggttt tagtttttttt gggtgggttt    9720
gtggagttgt ttttgttttt tgtttggttg ttggtgttgt ttagttgttt gtagattagg    9780
ttttggatgt ttttgattgt ggaaggaagg ggataggagg tatgggttgt tttgtggaag    9840
tgtgttgggg gaatatggag tgttggggta gtgagatttg ggtggatgga gggatttatt    9900
ggggtggtga ttgtttgttt tttttttgtgt gttgtgggat tttagtttta ttgtttttgt    9960
gtatattttt attttaggta tggttattag gataggttaa tttgtgttta agtgagagga   10020
tagggttgtg gggtagttat ggggagggtg ggtataggtt tttgtagttt tttttattag   10080
gttgttttgg gttaggggtt ggtgaatgtt ggatgtggtt ttgtttaggg ttagtaggaa   10140
atatttggtg tgttatatga taatgattat atattgattg tgtgataatg gttatatggg   10200
agagtgattt tgtggggaag ggttgtttat agaaagtttt ttaggattaa taggttgtgg   10260
ttaggtatag aggtggggat aagaatattt taggttaagg tgtgggatat gagagtgttt   10320
ttaggggggtt aagaataatg gttattggag ttgggtggaa ggatgttagt gggtttggag   10380
ggagaaagat tgattttttgt ttttgttgtt ttattttgtg gaaaggtttt ggaaggtagg   10440
ggttgagttg gtttttttta ttgttgaaag agtagattga ggtagggtgt ggtggtttat   10500
gtttataatt ttagtatttt gggaggtaga ggagggtgga ttatttgagg ttaggagttt   10560
gagattagtt tggttaatat ggtgaaattt tatttttatg aaaaatataa aaattagttg   10620
ggtgtggtgg tgggtgtttg taattttagt tatttaggag gttgaggtag aattatttaa   10680
atttaggagg tggaggttgt agtgagttga gattatgtta ttgtatttta gtttgggtga   10740
taaagtgaga ttttatttta aaaaaaaaaa aaaaaaagaa aagaaaaaaa gaaagagtag   10800
attgggtgtt gattgtttgt ttaagtggtt agtttagggg tttggtggtt gtgttagagt   10860
tgtgtgtggg gatttattga gggagtgtgt gtgtgttttt ttggggatat tattaggatt   10920
tattttttag gaagaagagg aatttttggt aggtagggaa ttagttttag ttaattttag   10980
atttttttta tggtttggtt tagagttttt gtatgtaggg agtattagag tggttgttga   11040
atggggagaa agtgttttat gtttgtaagg agtggtttgg tttagattat gtttaaggtt   11100
tttttggttt ttataggtat tggggggtgg ttagtgttgt ttttgggttt tttttatata   11160
gaagtttgtt ttggataggg tgttgtgttg gttataatga gttgtgagtt tggtttgggg   11220
ttttttgagg agtttatttt ggtaagattg tatttttgag gtgtgggtag ttaatttatt   11280
tttgtttatg gttgatttga gaattagttt tttttgtagg gttttggatg ggtttttttt   11340
```

```
atggaatagg ttttggggtt ggggtaggtt tttgaggttg tttattttgg ttatttttag   11400
gtagatttgt tgttttttta ggaggttttg tgtgattagt tggtttttta tgtttaattg   11460
ttttgtagag tgatggtgga ggtggtagtt agttgggttg gtttttttg gaattatttg   11520
agttttgggg attggtttaa ggatgaggaa ttttgttggt tttgttttga ggtaggttgt   11580
tttttgtgt tttgtatttt tgggaggtag gggagggtat ggttttgttt tttgattatt   11640
ttttttttg ttataaaaat taggttagtt gtggtggttt aggtttgtga ttttagtatt   11700
tagggaagtt aaggtaggag gatttttgtg gtttggagtt tgagattagt ttggataata   11760
taatgagatt ttgtttttat aaataaaaaa ttagttggtt gtggtggtgt atgtttggga   11820
aatgaggtag gaggattgtt tgagtttagg agtttaaggt tgtagtgagt tatgattgta   11880
gtattgtatt ttagtttggt taataaagta agattttgtt tttaaaagaa ataaaataaa   11940
attaaatata gtagggtgtg agtgaggatt gtattaggtt tatttattag tttgggatta   12000
atttagagtt ttagaaaatt gaatttttta ttttttttt ttttttttt ttttttttt   12060
ttttgagaag gagttttgtt ttgttgttta ggttggagtg tagtggtata attttggttt   12120
attgtaattt ttattttttg ggtttaagtg atttttttgt tttagttttt tgagtagttg   12180
ggattatagg tatgtattat tatgtttggt taatttttg tatttttagt agaaatgggg   12240
ttttgttatt ttggttaggt tggttttaaa tttttgattt taaatgattt atttgttttg   12300
gttttttaaa gtgttgggat tataggtgtg agttattgta tttagtttaa tttttaaatt   12360
ttttgtagag atagggtttt gttatgttgt ttaggttggt tttaaatttt tggtttttag   12420
tgatttattt gttttagttt tttaaagtgt agggattata ggtatgagtt attatattag   12480
gttatttatt gtttgagtat tatttaattt agaaatgaat aatatagggt tgggagtggt   12540
ggtttatgtt tgtaatttta gtattttggg aggttgaggt aggtagatta tgaggttgag   12600
agattgagat tattttggtt aatatggtga aattttattt ttattaaaaa tataaaaatt   12660
agtggggtgt ggtggtgggt gtttatagtt ttagttattt gggaggttga gggaggagaa   12720
tttttggat ttgataggta gagtttgtag taagttgaga ttatgttatt gtattttagt   12780
ttggtgatag agtgaggttt tgtttaaaaa aaaaaaagaa gataaaataa aaaaaaatat   12840
agatagtgaa agggaagaag ggagtttgtt gtaggttata gggtattagg aattgttttt   12900
gataaagtgt ttattgagtg tgggtgaatt ttaatattat attttgagag gagttagatt   12960
taaaaggttg tgtggtgtga ttttatttat gtggaatgtt tagggtaggt taatttatag   13020
ggataggaag tagatttttg ttgttagggt tggggtatgg ggtggggagt gattgttaag   13080
ggggatgggg ttttttttg gggtggtggg atgttttaaa gttaatatga ttttggttgt   13140
ataattggaa atgtattgaa atgtttttga attttatgtt tgggagggtg agtgttgtgg   13200
tgtgtgaatt atattttaat tgtgtaatta aatagggagg aggaagtttt ggaaggaggt   13260
tgtggggatt ttgtgttatt tttttgtatg ggattgggat gttttgttga gtttttggtt   13320
gtatgaggat ggggtggtgg tggtgtgtgt ggttgtgtgg ttggttttgg aaggggttta   13380
ggtttttttgg gttttttttt tttttttttt tatatatttt taaagggtta agtgattttt   13440
ggagattgtt atgttatggt atagtttgtt ttaatgtttt atagagttgt aggatatggt   13500
atagtttatg ggaaaatatt gggttatttg gggggtagtt tatttttttgt ggttagtttt   13560
gatggagggt ttagggttta ttatataagg ggtgtgttta gatgttgttt tttgtttatt   13620
ttgaaagttt tggagttttg tggtgtgggt tttgattatt tttttttttt ttttgggtag   13680
gttgaagggt tttttttttt tgttagggta gttgtaggga gaggagttgg gtgggtaatt   13740
gtgtttttta ggtaggttag ttttggtata gatgttggtt ttgtagaatt agttgttatg   13800
attgtttttg attttgggag tgatttagtt tgtttgtggt gtaggaggat gaggaggggga   13860
agagagagag ttattgatat ttattttgtt ttaaagtgtt agggtataat taggaagagg   13920
ttttatatgt ttgtttgtga tttagaggag gagtttaggg gatagtgggt ttgtagagga   13980
tttgttaggt gtaggtgggt ggttgtaagt tttgtagagt tatagttatt gtggatgggg   14040
gagtttgaat tagattaagg tattaggaag tttttgggaa ttaagagagg gagtggatag   14100
gtatggtggt ttatgtttgt aattttaata ttttgggagg ttaagatagg aggatggttt   14160
gaggttagga gtttaagatt agtttgggta atatagtgag gttttatttt tataaaaata   14220
aaaaatttag ttgggtgtgg tggtgtatgt ttgtagttta agttatttgg gaggttgagg   14280
gggtaggatg gtttgagttt aggagtttgg gttgtaataa gttatgattg tgttagtgta   14340
ttttagtttg ggtgatagag taagagttag attttaaaaa ataaaataaa ataaaataaa   14400
ataggttggg tgtagtggtt tatgtttgta attttagtta tttggggagg ttgaggtagt   14460
agaattgttt gaatttggga ggtggaggtt gtagtgagtt gagattgtat tattgtattt   14520
tagtttgggt gatagagtaa gattttgttt taaaaaaata aaaaataaag agagggaggg   14580
gaagttagtt ggttttgggt tttgaggggt gtttggttta aggaggaagg ataagatgtt   14640
atgttttgtt ttaataaatt tggttttaga atgtagagag tttgttatgg taggtgtagt   14700
ggtttatgta tgtagaaagg tttaggttat agttgttttt ttgggtttgt tggttttaat   14760
gtttaatagg ttgagttatt agaaggagtt ggaatggtgt tatgggtttt aagttgggat   14820
gagggagttt aaggttatag ttgatggtta atggttgaag ttaagggtgg attaattagt   14880
ggaaggaata tagtgggtgg ggagattttt aggttttttt tatttttttta tagttttttg   14940
gatgtgggtt atttaggtgt ttttgttttt tttggagttt gtgtagattt tatatatttt   15000
```

```
tggtttttgt aaggaggtgt tgattagaaa tattgttttt tttttgttgg ttattttttt   15060
tatgatgagt ttttgtaatg agatgatggg tggttttgag ttttgtatga gtagagggta   15120
ttggggaggt tattttttat tttatagtat ttaaatagta gggaggttaa gggtttggtg   15180
aggggaatta agagtggggg ttatgttttt tatttttttt taaataattt aaagtggaaa   15240
tattttgtta tttgggggaa aaaagatatt attgtagtga aattttagaa attttttttt   15300
tttttttttt ttttttaaat tattttggag gttaggtttt gttttgttat ttaggttgta   15360
gtgtagtggt gtaattattg ttttttgtag ttttgatttt ttgggtttaa gtgatttttt   15420
tattttagtt ttttgagtag ttgggattat aggtatgtgt tgttaggttt agttaatttt   15480
tgtatttttt gtagagatgg gattttatta tgttgtttag gttggttttg aagttttagg   15540
tttaagtgat ttttttattt tggtttttta aagtgttgag atgataggag tgagttattt   15600
tgtttttttag tattttgata atgtgttaat tttattagtt taaaaatttt tttttaaata   15660
gttggtaatg tatatatata tgagaaagtt taaaagatat aatggtatgt agtatataat   15720
aatagataaa tttatttttg ttttgtgttt taggttattt agagttattg gtatttttag   15780
tttgttatgt attttttaag agataatgtt tttatagatt agtaaaatgt atattttaat   15840
tggttagagt gggttatatt gtgtttttta taaatttata tgaagttttg agttttagta   15900
ttttagaatg tggttgtttt tggagatggg gtttttaaag aggtgattaa tgtgtgggtg   15960
tggtggttta tttttgtaat tttagtattt tgggaggttg aggtaggtag attatttaag   16020
gttaggagtt taagattagt ttggttaata tggtgaaatt ttattttttat taaaaatata   16080
aaaagtagtt gggtgtggtg gtaggtgttt gtaattttag ttatttagga ggttgaggta   16140
ggagaattat ttgaatttgg gaggtggagg ttgtagtgag ttgagattgt gttattgtat   16200
attttagttt gggtgataga gtgagattgt tttaaaaaaa aaaaaaaaaa aaaaaaaaaa   16260
gagaggtgat tatggtaaaa tgagattata tgggtgggtt taaatttaat ttgattggta   16320
tttttttaat aagaggagat tagtatagag gtatatatag agggatggtt atttgaggat   16380
atagagaata gatgagtata tggttgttta taagttaagg ggttttagaa gaaattaatt   16440
ttgttgatat ttttattttg gatttttagt ttgtagaaat atgagataat aaatttatgt   16500
ggtttaaatt atttagttta tggtattttg tttatagtag ttttagtaaa taaatatatt   16560
ggttaagaat atattagtag ttgggtatgg tggtgtatga ttgtaatttt attattttgg   16620
gaggttaagg tgggaggatt atttgaggtt agaagatgga gattagtttg gttaatatgg   16680
tgaaattttg tttttattaa aaatataaaa aattagttag gtgtggtggt gggtgtttgt   16740
aattttagtt atgtgggttt ggaaaattta taggtgtttt ttttatgggt agttttagtt   16800
aatttttttaa taaggttata gtaagtaggg ttttttgttt gttttttgtt tgtttgtttt   16860
gtttttttgt tttgagatag agttttgttt tgttgtttag gtgggagagt agtggtgtag   16920
ttttgttgga gagtagtggt ataattttag tttattgtaa tttttatttt ttgggtttag   16980
gtgattattt tgttttagtt ttttgagtag ttgggattat agatatgggt tattatgttt   17040
ggttaatttt tgtattttta gtagaggtga ggttttatta tgttggttag gttggatttt   17100
aatttttgat tttaagtgat ttgtttattt tggtttttta aagtgttggg attatagatg   17160
tgagttatag tatttagttg attttttgtt tgaagagata ggatatatat tatagaagta   17220
aagatgtatt tttgattttt tttttgtagt agtaaaagta tgttggttag taggatagtt   17280
aggatatttg ggaaaatagt atggaaggat gtattttaaa agttttatag ttaggaggtt   17340
ttgttggtta tttttgtgga tgttattttt taggaaggtg taagtagtat gatgtttttg   17400
tttgggttta gaagtttttt ttttgttttg tttgtatttt ttattatttt tttaggtttt   17460
aatttttagt attaatgagt agttggagtt ttttttaagt tttttttgtg tttagagttt   17520
aaattagggt ttttgtttat gttaggttat tgtgtttggt aatttttttt gttgtaataa   17580
tatttgttgg gtttgatttt tgagaatttg atatggtttg gttgtgtttt tatttatatt   17640
ttattttgaa tttttatgtg ttgtaagagg gatttggtgg gaggtaattg aattatgggg   17700
gtagattttt tttatgttgt ttttgtgata gtgaatgagt tttataagat ttgatagttt   17760
tataaggggg agttttttttg tataagtttt tttttttttt gtttgttgtt atttatgtaa   17820
gatttgattt gtttttttttg ttttttgtta tgattgtgag gttttttttt ataggtggaa   17880
ttgtttaagt ttattaaatt ttttttttttg gtaagttgtt tagttttagg tatgtttttta   17940
ttagtagtat gaatatggat taatataata tttttttttt tgatgtttta gatgtgaatg   18000
gagttttttg tttttttttgt ttttgggtta ttaggatttg agttatagta gtgatgtagg   18060
ttaggggtgg ttttagtttt tttgtgtttt ttagtttttt ttggtgtttt gtttttgtat   18120
gaattttttg tgttgtaagg tttttttgtat atagatgtaa attttgagtt gtgttttaag   18180
ggtaatgtta tttttgggga gttgtttttta atatttttgt ttaagttaga tatgtttttg   18240
ttaatttttt tatattgtag tttgttggtt tttttaaagt atgtgttgga tttttaatta   18300
gtttttttgt tttttggttt gttttttttt tttagtttag tgattgatgt tttaaaaggt   18360
taggtattag gttttgtttg gtggggtgta tttttattat ttaatttggt gtttggtagg   18420
tagaaggtgt taaatttgtt tgtgaaatat atgtttattt aaa                     18463
```

<210> 245
<211> 15597

<212> DNA
<213> Artificial Sequence

<220>
<223> chemically treated genomic DNA (Homo sapiens)

<400> 245

```
ttttgttgtt ggtaattttt ttttgaaagt gtaaatgttt ttagggagtt attaggaata      60
gatgtggatg ggtagttttt atttagggta tagttgttat ttatttggtt tagaattggg     120
aagttggttg ggtatagtgg tttatgtttg taattttagt attttgggag gttgaggtgg     180
gtaggttatt taaggttagg agtttaagat tagtttgatg aatatggtga aattttattt     240
ttattaaaaa tataaaaaat tagttaggta tggtggtgga tatttgtaat tttagttatt     300
taggaggtta aggtaggaga attatttgaa tttaggaggt agaggttgta gtgagttgag     360
attgtgtttt tgtgttttag tttggtaata gagggagatt tttttttaaa ataataaaat     420
aaaaatgaat tgtgatgttt atttaagtag gaggtatttt ttatgtaaag gtatagtgtg     480
tttttggaa taggtgtttg ggagaattga tttatttatt tgttgtgtag ttttgttggt     540
gagtagtgtg aattttatag ataggttttt tagagtttgt tttagggatt tttatgtgtt     600
gttatttaga gtggtttagg agggtagtgg ggttgtgtta gattttattg tattgaggga     660
gtaagtatat aggagaggag atatttaagg tttggagttt atttggtagt tgagtttggt     720
atttttttg atagatgttt tttatggtaa tttgtgtgat tttgttttag gattttgttg     780
tttaaagagt ggtttgtggg gattttggga gtttgggaga aagggtggtt ttggggttgt     840
attttaggat ttgttgtgtt tttttttttt aattagtttt taggtgatat gtgtgtatag     900
gtgatgggta ttgttggggt aagtagagtt ttgggtgggg tttttttttt ttttaggtaa     960
aggaagtgtt gttagtttat ggtttgtttt tttttaagtt aaagggattt tttttttttt    1020
tgagatggag ttttgttgta ttatttaggt tggagtgtag tggtgtgatt ttggtttatt    1080
gtaattttg tttttaggt ttaagtaatt ttttgtttta gtttttgag tagttgagat       1140
gataggtatt tgttattatg tttagagaat tttttttgt atttttagta gagatgggtt     1200
ttattatttt ggttaggttg attttgaatt tttgattttg tgatttattt attttggttt    1260
tttaaagtgt tggattata ggtgtgagtt attatgtttg gtttgggatt ttttttttaaa    1320
gttttatttt gatagtgaaa aaattattag attagaaatt ggtgagtatt tatttgtgtt    1380
tagaaatttg tgttttaggg ttgaaatttt tttgtgtttt ttttaaatat gatttttttat   1440
gtgtttgagt tattttttgt ttttttgag atgaaatagg ttaggatgtt ggttattatg     1500
gtaaatgtat tttttgttta agtttttat tagtatggat ttttgtttta ttgtgtgtgt     1560
ttttgtagaa gttgaaaggt tatttatttt agttttttt tgaatatgag aagatttgtt     1620
gtattgttga tggtttgtga tgtgatttgt ttggttttgt tgtgaagttt gtgaaagtta    1680
ttgtagatgt tttggattag tttttggttg atggtaaatt gagtttggat gttagttggg    1740
aggaagtttt gatattatga tggggggaga gatattgtgt tagttgttgg ggagtatttg    1800
ttagatggta gtagttggtt ttttagaagt tgagttatgg gagatatagt aggtttatga    1860
gttattttgg gagttttatt tttttaggtt tgtagggatt attttgtgtg tttaaaaaag    1920
aaataaaaat tattattatt tgtatgttat aaatataaag ataaatgatt ttatgggatg    1980
aggattatag agtttattga aattttatta tattggagga tttatttaaa aatatttaaa    2040
taaggttagg ggtagttatt tatatttgta attttagtat tttggaggtt gaggtaggtg    2100
gattatttta ggttaggagt ttaagattag tttgattaat atggtgaaat tttatttta     2160
ttaaaaatat aaaaattagt tgggtatggt ggtatgtatt tgtaatttta gttattttag    2220
aggttgagat aggagaattg tttgaatttg ggaggtagag gttgtagtga gttgagattg    2280
tgtaattata ttttagtttg ggtgatagaa tgagattttg ttttaaaata aataaattaa    2340
taaaatattt gaataataat tttgttgggt gtagtggttt atgtttgtaa ttttagtatt    2400
ttgggaggtt gagtagggtg aattatgagg taaggagatt gagattatgg tgaaatttta    2460
tttttattaa aaatataaaa aattagttga gtttggtggt gggtatttag ttttagttat    2520
ttgggaggtt gaggtaggag aatggtatga attttggagg tggagtttgt agtgagttgt    2580
gattatatta ttgtatttta gtttgggtaa tagagtgaga ttttgtttta aaaattaata    2640
atattaattt ttagttggat atagtgattt atatttgtat tttagtgttt tgggagattg    2700
aggtaagagg attgttagag tttgggagtt taagaatagt ttgggtaata tagtgaaatt    2760
ttattttttt taaaaaatta aaaaattagt taggtgtggt ggagtgtttg tggttttagg    2820
tgtttataga gggttgaggt aggaggatta tttgagttta ggagttttat gttgtaagga    2880
gttatgatta taatattgta ttttagtttg gggattttgt ttttagaaaa taataaaaat    2940
aaaaataaat aatttttaag ttaaatataa tgaatataaa tttttgaaag atttttttata   3000
tggggggaag gtttttttta tgttgtgttt atttttaaag atttttttata agatttgata   3060
ttaattatta gagtttagaa aatagaaagt ttagagagta aaatgaaggt aaaagatttg    3120
gttaggtatg gtggtttatg tttttaattt tagtattttg gggggttatg ttaggaggat    3180
```

```
tgtttgaggt taagagttta agattagttt gggtaatata gtgagatttt atttatatta    3240
aaaataaata aataaattag ttaagtattg tggtgtatgt ttgtggtttt agttattttg    3300
ggaggttgaa gtgggagaat tgtttgaatt taggaggttg aggttgtaat gatttatgat    3360
tgtattattg tattatagtt tagaggatag agtaagattt tgttttaaaa aataaaataa    3420
aatagatttt taatttttgt atgaagaagt aggttttttt ttggtttttt tgttgttgtt    3480
gttgttgttt ttgatataaa gttttatttt gttatttagg ttggagtgta atggtatgat    3540
ttgggtttat tgtagttttt gtttttgggg tttaagtagt tttttgtttt tagtttttta    3600
aatagttggg attataggtg tttgttatta agtttggtta attttttttt ttttttttt     3660
gatggtgttt ttgttgttta ggttggagtg tagtggtgta attttggttt attttaattt    3720
ttttttttta ggtttaagta atttttttgt tttagttttt tgagtagttg gaattatagg    3780
tgtatgttat tgtatttagt taatttttgt atttttagta gaggtggggt tttattatgt    3840
tagttaggtt agttttgaat ttttgatttt gtgatttatt tgttttggtt ttttaaagtg    3900
ttaggattat aggtgtaagt tattatgttt ggttggtttt ttggggtttg ttttgttttt    3960
ttttgttttt tttttttgtt ttgtattttt attagagatg gggttttgtt atgttggtta    4020
ggttggtttt aaatttttga ttttaagtga tttgatagtt ttagtttttt aaagtgttgg    4080
gattatagga aggagttatt gtgttagttt gaagaagagg tttaaaaaga aaaaattaaa    4140
aataaaaaaa ttattaagta gttattttag ttttttttgg aaaaagatta aattttaaat    4200
atttaataat aaataaatgt aataatgaat ttttagaaat ttgaaaaggt gttgatataa    4260
gtatttgttt gtttgagtta tagaaaaggg tagagggtat gaggtgtttg gagggtttga    4320
gggtagagtt tagttgtaat ttggttttgt tgtttagagg tttggggttt tggtaaatga    4380
tagttttttt gagtttgttt ttttatttgt aaaataggtt ttttagtttt gtgttggagt    4440
aattgaaatg agggttaaat gaggaaatta tgtgatagat gttagtataa tttttgttat    4500
gtagaggagg taaaattatt ttgaagggtt aaaatttgtt tttttggtta aaaagggatg    4560
tattttttaa agttagaaaa atattttggt tttttttttt tttttttttt tttttttttt    4620
tttttttttt tttttattt tatagttttt tttttatgtg gagttgaagt tggattgtat     4680
tgttgttatt ttggtttatt gtaatttttt tgtttgattt ttttgtttta gtttgttgaa    4740
tgtttgtgat tgtaggtatg tgttgttatg tttgattggt tttggtggag atggggtttt    4800
gttgtgttgg ttgggttggt ttttagtttt taattgtgag tgatttgtta attttggttt    4860
tttgaggtgt tgggattgta gatggagttt tgtttattta gtgtttaatg gtgtttaggt    4920
tggagtgtag tgatgtgatt ttggtttatt ataatttata tttttagtt gtttgttttg     4980
gtttttaaa gtgttgagat tgtagttttt gtttggttgt tattttgttt gggaagtgag     5040
gagtgttttt gtttggttgt ttattgtttg ggatgtgagg agttttttttg tttggttgtt   5100
tagtttggaa agtgaggagt gtttttgttt ggttgttatt ttatttagga agtgaggagt    5160
gttttttttt agttgttatt atatttagga agtgaggagt gtttttgttt ggttgtttat    5220
agtttgagat gtggggagtg tttttgtttt gttgttttat ttgggatgtg aggagtgttt    5280
ttgtttggtt gagattttgt ttgggaggtg aggagtgttt ttgtttggtt agttattttg    5340
tttgggaggg agatgggggg gttagtttta ttatttggtt agttgttttg tttgggaggg    5400
aggtgggggg gttagttttt tgtttggtta gttgttttat ttgggaggga ggtggggggt    5460
gttagttttt tgtttggtta gttgttttat ttgggatgtg aggagtgttt ttgtttggtg    5520
agattttgtt tgggaggtga ggagtgtttt tgtttggttg ttttgtttga gaagtgagga    5580

gattttttgt ttggtaatta ttttgtttga gaagtgagga gttttttttgt ttggtagttg   5640
ttttgtttga gaagtgagga gttttttttgt ttggtagtta ttttatttgg gaagtgagga   5700
gtgtttttgt ttggtagtta ttttatttgg gagggaggtg gggggggtta gtttttttgtt   5760
tggttagttg ttttatttgg gagggaggtg gggggttagt tttttttgttt ggttagttgt   5820
gttatttggg agggaggtgg gggggttagt ttttttgtttg gttagttgtt ttgtttggga   5880
gggaggtggg ggggttagtt ttttgtttag ttagttgttt tgtttgggag gtgaggggtg    5940
tttttgtttg gttgttttta ttgggaagtg aggagttttt ttgtttagtt agttgttttg    6000
tttgggaggg aggtgggggg ggttagtttt ttagtttggt tagttgtttt gtttgggagg    6060
tgaggggtgt ttttgtttgg ttgtttttat tgggaagtga ggaggttttt tgtttggtta    6120
gttgttttgt ttgggaggga ggtggggggg ttagtttttt gtttggttag ttgttttgtt    6180
tgggagggag gtgggggggg gttagttttt ttgtttggtt agttgttttg tttgggaggt    6240
gaggggtgtt tttgtttggt tatttttatt gggaagtgag gagttttttt gtttggttat    6300
tattttgttt gggaggtgtg tttaatagtt tattgagaat gggttaggat gataatggtg    6360
gttttgtgga atagaaaggt gggaaaggtg gggaaaagat tgagaaattg gatggttgtt    6420
gtgtttgtgt agaaagaagt agatatggga gattttttat tttgttttgt attaagaaaa    6480
atttttttgt tttgggattt tgttgatttg tgattttatt tttaattttg tgttgtgttt    6540
atttagggtt aaatggatta agggtggtgt aggatgtgtt ttgttaaata gatgtttgaa    6600
ggtagtatgt ttgttaagag ttattattat tttttaattt taagtaatta gggatataaa    6660
tattgtggaa ggttgtaggg ttttttgttt aggaaaatta gagattttttg tttatttgtt   6720
tatttgttga tttttttttt attattgttt tatgattttg ttaaattttt ttttgtgaga    6780
```

aatatttaag aatgattaat aaaaaaataa attaaaaaaa aaaaaaaaga aaaaaaaaag 6840
aaaaatattt tggttttggt agttagttta tagtttagga atttgttttt gatgtattgt 6900
gttgattttt ttaatttgtt ttgtatattt tgtaaaagga aaatatatag ttttattttt 6960
ttttattagt agtaagaaag ttgttgtggg gtatgggtta ttgtggagtg ggaggttgtt 7020
tttttttagg agaaatatat ttttatatgt tggttttgat attagtattt tgtaattttt 7080
tgattttttt ttagatgttt tttttattaa ataggatagt ttggttttat agtaggagta 7140
ttataaattt gtagtttttt tttagaggta tagttatttt atttgtagat ggggggtagt 7200
tttttagaga aatagttatt ttatttatag attaggggtg gttttgtaga gaaatggttt 7260
ttttatttat agatttgggg tggtgttagt tttagagata tttgagattt tggtagtttt 7320
taaaaatgta atagaatagg gtaaaagtgt tttttttttt gggaatgtat ttatggtgtt 7380
agggatatta gagtttaggg agttgttggg tagagggata ggtttagttt ttggtattgg 7440
agttttttta tttgaaagag gtttttttggg taaagttgtg ttttaaagtt aggagttatt 7500
taggagatat aaggggaaat tttaatatgt agttgtttgt gtgtttagga atttttaggt 7560
tttagagatg ttttaaagat tatttgattt aatttttatt ttataaaaaa aaaaaaaaaa 7620
aaaaaaaaaa agatttgagg tttagtatgg tgtgtgtttg tagttttagt tatttagagt 7680
ttgaggtggg aggattgttg gagtttaggg gtttagggtt agtttgggta atatagtgag 7740
attttatttt taaaaataga aagtaagtaa gaaaattgag gttgggaagt gatagagaat 7800
gatatttgtg ggaaggttat agggaatgtt ttgtttttag aatagggata ttgtttattt 7860
gtgatttatt gtatttttgt ttattgtttt ttttaggttt tgttatattt ttttgttggt 7920
aatgtagatt gtttttaaag gaaggttttt aattttaggg tttaaaagat tttattgttt 7980
gttttagata tttttggggg tattttaggg atattttagg tataggggga aggttttttt 8040
gtttttgaga ggaatgaaga taaaaggaat taaggtttgt gtgaagttag tttgggagag 8100
ggggtgtttt tttgtagtag ggataaggat agaggttgtt aggtttttat ttaagatggg 8160
gttttttttga aatagaggag agtttggaaa tttttatttt agtggagttt ttgttttttt 8220
ttgtataggg agagggtttt gtaggtatag gtggaggaga agtataggat ttgttttttt 8280
tggaggggtt ggtagttgtt tttttttttta ggaaatagga gttgggttgt agggttgttt 8340
tttgtttttt tgagataggg ttttattttg ttgtttaggt tggagtgtag tggtataatt 8400
atagtttatt gtaagtttta tttttttgggt ttaagtgatt tttatgtttt agttttttaa 8460
gtgattggga ttataggtgt gtattattat gtttggtttt agttttgttt tggtagagat 8520
gaagttttgt tatgttgttt aggttggttt taaatgtttg ggtttaaggg attatttagt 8580
tttggttttt ttgagtgttg ggattagagg tgtgggttat tgtatttgtg aattgtagtt 8640
ttttgatata taataaggtt gtgttgtagt agttgggagg agggaagttt tatttagatt 8700
tttgatggag tgtatgtata ataagttaat ttttggatgt aggtgtgggt tgtggttttg 8760
ataaggaggt ttagtttgtg ttttagtggt ttttagttag atagggtttt gttttaagtt 8820
aatatttttt tttatttgga gtttttggta tttttttaaa ttttttaaat tatttagttt 8880
aaatttttaa agtgattata gagatttatg atttttatgt ggatttataa aattttgtaa 8940
agttaatatt agggatattt ttgaaaatta tatttagatt gttatttgga aaggaaagaa 9000
gttttgttta tgttattttt gttgggtgtt taaataaggg attaggagga taagaaatta 9060
tatttgagag aggaaaaggt gggtttggag gtttggggat tttttggggt tgggatatgg 9120
gtagggtaga tatatttatt gagttgttga aggataggaa gagtttgagg attatatttt 9180
tggagaatag ggggtgttgt gttattaggt tgatgaagtg ttttagggtt tttttttttgg 9240
ttttgatgaa tttttttgtta gttggaggag tatatggggt tattggatag agtttagggt 9300
gttggttttt attttttgtt ttgggtgagt ttttaaaatt tttttttagag gtggtagagg 9360
gtttagtggt tagtagaggg tattggttgg gtttttagtg ttgtttagat aatgggtttg 9420
aaaaggttgt ttagagtaat aattttattt ttagaggttt tttttagtta tatatatttg 9480
ggtttaggag ttgttaagga aaattataga tttgtttgtt tttattggga gttttgggaa 9540
ttatgtgagt ggttatttta attatttttg ttaaagatat tttttataat tttttttttgg 9600
tattttttat tgtaaataaa tgggttttgt tatgttttaa tattaatgta atttaggttt 9660
tttttttttt tttttttttt tgagatagag ttttgttttt gttgtttagg ttggagtgta 9720
gtggtgtaat tttggtttat tgttattttt gtttttttagg tttaagtgat ttttttgttt 9780
tagtttttgg aattagttgg gattataggg gtttgttaat atgtttggtt aatttttgta 9840
tttttagtag agatggggtt ttattatgtt ggttatgttg gtttttaatt tttgatttta 9900
ggtgatttat ttgttttggt tttttaaagt gttgatatta taggtgtaag ttattgtgtt 9960
tggttgagtt tttttattta ttgtgtttat aatttatttt gtaaaggatg taattattta 10020
ttgtttttat ttgggttaaa tatttttatt ttaaggggaa aaaaagtaaa agtatatatt 10080
gatgtattta ggatatttta atttgaatat aaataaaaat ggtatttaaa tatttttttt 10140
gagatggagt tttattttttg ttgtttaggt tggagtgtag tggtgttatt ttggtttatt 10200
gtaattttta atttttaggt ttaagtgatt tttttgtttt agttttttaa gtagttggga 10260
ttataggtat ttgttattat atttggttaa tgtttatatt tttattagag aggaggtttt 10320
attatgttgg ttatgttggt tttgaatttt tgattttagg tgatttgttt attttagttt 10380
tttaaagtgt tgggattata ggtatgagtt attagtgttt agtttttaaa tattattttt 10440

aatgtattaa gaaatatagt taggagtgtg gtttatattt gtaattttag ttttttggga 10500
ggttgaggag ggtagattat ttgaggttaa ggagttttat attagtttgg ttagtgtagt 10560
gaaattttgt ttttgaaaaa tattataaaa gaggttaggt gtagtggttt atatttgtaa 10620
ttttagtatt ttgggaggtt gaggtaggtg gattatgagg ttaggagatt gagattattt 10680
taatatggtg aaattttatt tttattaaaa atataaaaaa aaataattag ttaggtgtgg 10740
tggtgggtgt ttgtagtttt agttatttgg gagatttagg taggagaatg gtgtgaattt 10800
ggtaggtgga gtttgtagtg agttgagatt gtattattgt attttagttt gggtgatatg 10860
gtgagttttt gttttaaaaa taaaaataaa aataaaaaat attataaaag aggttaggtg 10920
tggtggttta tatttgtaat tttagtattt tgggaggttg aggtgtgtgg attatgaggt 10980
taggagattg agattatttt ggttaatatg gtgaaatttt gtttttatta aaaatataaa 11040
aaaattagtt aggtgtggtg gtgggtgttt gtagttttag ttatgtggga ggttgaggta 11100
ggagaatggt gtgaatttgg gaggtggagt ttgttatgag ttgagattgt attattgtat 11160
tttagtttgg gtgatagagt gagattttat gtaaaaaaaa aaaaaaaata ttataaaaga 11220
aagaaaataa ataatatagg taggtggttg gggttttttt ttggtttagt attggtaggt 11280
tttttttgat gaggttgtat ttttttaaat gtgtaagtgg tttagtgtgt gtgtgtagtt 11340
tttaggtgtt gaatgttagt tagtgtttat taatttggat gtttaggttt gagttagtat 11400
tatatatttt tgttgtgttt tttgtttttt gttgttttga gtgtttgtgt gttttttagt 11460
gtgagttgaa tgatttgttt tgtggttttt atatgtggtg gggtgaaaat tatattatta 11520
gattttgggt tgtttgttta gtgattgatg agaggtagaa gtatttatgt attattgtat 11580
gattttagat gtggggaatt ggtgattgtg ttagttttta tgttgtgttt ttgtttagga 11640
tatggtgatt gtggaagggt tgggaatggt agaaagttat gattagtttt aagtatttat 11700
tttttagta tgttttattt agaatttgag gaatgggttg agggagagat tattagtgag 11760
attagagttg gtagttgtta ttttaagttt tgtagtttag gggatatag gtttggggag 11820
tagttttgag gttgtttgtg attttttatt tggtttgagg ttatttttag gtagagaggt 11880
gaaggtgagg tagttgtgtg ttgttttttt ttttttgtaa gatgtagttg atatttttta 11940
tttgtataat ttagtttttt tttatgaggt tattgagtgt ttttatggtg tttatggttt 12000
aggttgttat ggttttgtag atgtaggtag ggtaatgtta gttaggtgag tatatagtgt 12060
ttttggtagt ttttttggga gtttttggga gttttggtta ttgtattttg gagaggagaa 12120
ttagggtttt tttttatatt tgtatagtaa gggttttttt atttgttagt tatgtagttg 12180
aaggttttgg tgggttaagg ttgttgggtg ttagtttttt tgtttgtatt aaggttgggt 12240
ttaagttata gttttgtttt agatgttaaa aggttggagt ttttttgta gttttgtatt 12300
tgttttggtt tatagtgggg tttattttg agttaggatt atagggttta ggagatgggt 12360
ttgttttttt tgtttttggt gggtagtatg tttggtttta ttttttagta ttttttggg 12420
tggtagggta ggtattatat ggtaggggaa tttgtgtagg agtatttttt ggaagattat 12480
gaagttattg tattgtttgt atattgagga tttgaagtgt tgtaagagaa gggttggtgt 12540
ttagatttga tgttggaaat tatgttgttg tgttattttt ttttaataga gtttgggaaa 12600
atgatagtat taagtgtatt tttttttatt aggtttttat taaatggaga aattttgtga 12660
tggttttgta tatggaaata taatttttaa attttgtata gtttttaggt tgggtgtggt 12720
ggtttttgtt tgtaatttta gtattttggg aggttgaggt gggtgaatta tttgaggtta 12780
ggagttaaag agattagttt ggttaatatg gtaaaatttt atttttatta aaaatataaa 12840
aattagttgg gtatggtggt gggtggttgt agttttagtt attttggagt ttgaggtagg 12900
agaatttttt gaattttgga gatagaggtt gtagtgattt aagattgtgt tattgtattt 12960
tagtttggat gataaagtga gattttgttt taaaaaaatt aaaaaaggtt gggtgtggtg 13020
gtttatgttt gtatttttag tattttggga ggttgagata ggtggattat gaggttagga 13080
gattgagatt attttggtta atatggtgaa attttgtttt tattaaaaat ataaaaaatt 13140
agttgggtgt ggtggtgggt atttgtagtt ttagttattt aggaagttga ggtaggagaa 13200
tggtgtgaat ttgggaggta gagtttgtgg tgagttgaga ttatgttatt gtattttagt 13260
ttgggtggta gagtgagatt ttattttaaa aaaaaaaaaa aaattaaata aatggtatga 13320
agtatagtgg tttttttattt ggttagtgat tggtttgtta tatatatatt agggtgatta 13380
aattgttttt ttgataaatg ttttttatgt ggtaatgaat ttatttaagg gatgggtagt 13440
ttagtaagat tggataaaag gttagttgtt ggttttttgg taggtaatgg tatgttttat 13500
atgtttagag ttgttttatt tttaggttgt taggttttag aaggaagggg tggtttgttt 13560
tgttttttttg tggtatttta ttagttggtt tatattttg attgtggttt agagggttgg 13620
gaattatggt aaagttagat agtaaggtgg ttttgatatg gtttttttaag ggagtttttt 13680
tttttgattt gttttgtttt ttggtagtag tattgataat tatttggatt gtgtggttat 13740
aggttttgga atatagtgaa gtatagtaga gtagatggta gggaggggag attggtttgt 13800
ttgtttttaa ggttaggtgt agtggtttat gtttgtgagt ttagtatttt ggtgggttaa 13860
agtgagagga ttgtttgagt ttaggagttt gagattagtt gagataatat agtaagtttt 13920
tatttgtatt ttaaaaaaaa aaaaaaaaaa aagattgggt atggtggttt atgtttgtaa 13980
ttttagtatt ttgggaaatt aaggtaggag gattatttga gtttaggagt ttaagattag 14040
tttggataat atagtgagat tttattttta taaataattt aaaaaattgt ttggtgtggt 14100

```
ggtatttgtt tgtggtttta gttatttagg agattgaggt gggagggttg tttgagtttg   14160
ggaggttgag gttgtagtga gttatgattg tgttattgta ttttagtttg gttgatagag   14220
taatattttg ttttttttaaa aataagtaaa taaaaagata taatttttat ttaggattat   14280
ttgtttatga tgttttttaa ataagttttt ggtagtgttg tttggttggg gttgggttag   14340
ggttgttttt ttttatatag gagtgggttt tgttttgttt gtgtgaattt tataagtttt   14400
gttgatttta tattttttaa ggatagtgtt agtttattaa ttatttaatg tttaggatga   14460
ataatgaaaa tttttattag aatttgttgt aatggaattg agataatgaa gtaagggata   14520
gagagagtta tggtgttgtt ttagatgggt attaggtatt tggtgtttgt gttttatttg   14580
tttaatattg ggatgtttag taataggagg gttgtgttat taaaggggat tagagtgata   14640
tatattgatt ttgttatgta aattttattt attgtttggg gtttggagtt gatggggttg   14700
atattttaat ttttggggg tttttattat tataaagtga ggttggtgtg gattttgttt   14760
tgtgttttgt ggattgtttg tgggagattt ttaagaaata ggtttttagg ttgggtgtgg   14820
tggtttatgt tagtaatttt agtattttgg gaggttgagg tgggtggatt ataaggttag   14880
gagattaaga ttattttggt taatatggtg aaattttgtt tttattaaaa aaaaaaatat   14940
aaaaaaattt gttaggtgtg gtggtgggta tttgttattt tagttatttg ggaggttgag   15000
gtaggagaat tgtttgaatt tgggaggtag agtttgtagt gagttgagat tatgttattg   15060
tattttagtt tgggtgatag agtaagattt tatattgggg aaaaaaaaaa aagaaatagg   15120
attttagagt atagtggtgg ggaggataaa ttggattagg ggtgattagg tggggatgtg   15180
gttgggttag agtgaggtgt ggggagtaga ggtgtggaaa agttattttt gtagtttgat   15240
tttgttgtga gttttggatt ttgtttgtaa gttagttata tagatttatt ttttagtttt   15300
tttttgttat attattttttg ttttgagatg ttgagatgtg attttttttt ttttttttttg   15360
ttttgatata tatataattt gttagttatt tggttggaaa ttttatattt tatatgtttt   15420
aggaagaggt tttttttttt tggaatgagt tttatttgta tggtgttttt ggttagtagt   15480
ttttgtaggg tgtgggatag tagtagtggg ttttttgtg gtatttgtat ttgattgggg   15540
gttgggattt gttgtatgat ggtttggggt ttgatggttt ggggtgttgg tagattt      15597
```

<210> 246
<211> 15597
<212> DNA
<213> Artificial Sequence

<220>
<223> chemically treated genomic DNA (Homo sapiens)

<400> 246

```
agatttgttg atattttagg ttattgagtt ttaggttatt gtgtagtagg ttttagtttt      60
tagttgaatg tagatgttgt aggggaattt gttgttgttg ttttatattt tgtaggagtt     120
gttggttagg gatattgtgt aggtggagtt tattttggag aagaagggtt tttttttgaa     180
gtatgtggag tatgaggttt ttagttaggt aattggtaaa ttgtgtgtgt gttggggtag     240
gagggggaag aaagggttat gttttaatat tttagggtag gagtggtgtg ataggagaag     300
gttgaagggt gagtttgtgt ggttggttta taggtaagat ttagaatttg tagtagagtt     360
aggttgtgga ggtgattttt ttatattttt gtttttttgta ttttattttg gtttagttat     420
gtttttattt ggttattttt ggtttagttt gttttttttta ttattgtgtt ttggagtttt     480
gttttttttt tttttttttt tgatgtggag ttttgttttg ttgtttaggt tggagtgtag     540
tggtatgatt ttggtttatt gtaaattttg tttttttgggt ttaagtgatt tttttgtttt     600
agtttttttaa gtagttggga tgataggtgt ttgttattat gtttggtaaa ttttttttgta     660
tttttttttt tagtagagat agggttttat tatgttagtt agggtggttt tgatttttttg     720
attttatgat ttatttgttt tggtttttta aagtgttggg attattggtg tgagttattg     780
tgtttggttt ggaggtttgt tttttaggaa tttttttgtgg atggtttata gagtataaag     840
tgggatttat attagttttg ttttgtaata gtgggaaatt tttgaggatt agaatgttag     900
ttttattgat tttggatttt aaatagtgaa tgaaatttgt atggtaggat taatatgtgt     960
tattttaatt ttttttaata atatggtttt tttgttgttg ggtattttga tattgggtag    1020
gtgagatata gatattgagt gtttgatgtt tgtttggagt aatattgtgg tttttttttgt    1080
tttttgtttt gttgttttag ttttattatg atagattttg atgggagttt ttattgttta    1140
ttttgggtat tgagtaattg atgagttaat attgtttttg ggaggtgtgg agttagtagg    1200
gtttgtggga tttgtataga tagagtaggg tttatttttg tgtgggagag ggtggttttg    1260
gtttagtttt ggttggatgg tattgttagg ggtttgtttg aaaagtgtta tgagtagatg    1320
gttttgggta aaagttgtgt ttttttgttt gtttgttttt aaagagatag ggtattgttt    1380
tgttaattag gttggagtgt agtggtatga ttatagttta ttatagtttt aattttttag    1440
gtttaagtaa tttttttatt ttagtttttt gagtagttgg aattataggt ggatgttatt    1500
```

```
atattaggta gttttttaaa ttatttgtag agatgggatt ttattatatt gtttaggttg    1560
gttttgagtt tttgggttta agtggttttt ttgttttggt tttttaaagt gttgggatta    1620
taggtgtgag ttattatgtt tagttttttt tttttttttt tttttaaagt ataaatggag    1680
atttgttatg ttgttttagt tggttttgaa tttttgggtt taagtagttt ttttattttg    1740
gtttgttaaa gtgttaggtt tataggtatg agttattgta tttggtttta aggataagtg    1800
agttggtttt ttttttttgt tgtttgtttt gttgtgtttt attgtgtttt agagtttgtg    1860
gttatatggt ttgaatggtt gttagtattg ttattaaagg ataaagtggg ttagagggga    1920
ggattttttt ggaggattgt gttagagttg ttttgttgtt tggttttgtt gtggtttttg    1980
gtttttttggg ttgtagttgg ggatgtggat tagttggtgg gatgttatgg gggagtagag    2040
tgagttattt ttttttttttg gggtttggta gtttgggggt gaggtaattt tgagtgtgtg    2100
agatgtgttg ttgtttgtta gggagttagt ggttggtttt ttgtttagtt ttattaagtt    2160
gtttgttttt taagtaaatt tattgttata tagggaatat ttgttaaggg ggtagtttgg    2220
ttattttgat gtgtgtgtaa tgggttagtt attgattagg tgggaagtta ttatgtttta    2280
tattatttgt ttagtttttt tttttttttt gaaatggagt tttgttttgt tgtttaggtt    2340
ggagtgtagt ggtgtgattt tagtttattg taagttttgt tttttgggtt tatgttattt    2400
ttttgtttta gtttttttgag tagttgggat tataggtgtt tattattatg tttggttaat    2460
ttttgtgtt tttagtagag atgaggtttt attgtgttgg ttaggatggt tttgattttt    2520
tgattttatg atttgtttgt tttggttttt taaagtgttg ggaatgtagg tgtgagttat    2580
tgtgtttggt tttttttaat tttttttaaga taaggttttg ttttgttgtt taggttagag    2640
tgtagtggta tgattttgga ttattgtaat ttttgttttt agggtttaag ggattttttt    2700
gttttaggtt ttaaagtagt tgggattata gttgtttatt attatgttta gttaattttt    2760
gtattttttag tagagatggg attttgttat gttggttagg ttggtttttt taatttttga    2820
ttttaggtga tttatttgtt ttaatttttt agaatgttga gattataggt gggagttatt    2880
gtatttggtt taagagttat ataaagttta aaagttgtat ttttatatat gaagttgtta    2940
tagggttttt ttatttagta aaggtttggt gagagaagat gtatttggtg ttgttgtttt    3000
tttgggtttt gttgagggga agtgatgtga tagtatagtt tttaatgttg gatttaagta    3060
ttgatttttt ttttgtagtg ttttaagttt ttggtatata gatggtataa tgattttgtg    3120
gtttttttagg agatgttttt gtataagttt ttttattgta tggtgtttgt tttgttattt    3180
aagagaatgt tgggaggtaa agttaggtgt gttgtttatt gggggtggag ggagtgagtt    3240
tattttttgg atttgtggt tttggtttag ggatggattt tattgtagat tggggtaggt    3300
gtagggttgt gggggaaatt ttagtttttt ggtatttggg atagagttgt ggtttgagtt    3360
tagttttggt gtaggtaggg agattggtat ttggtagttt tggtttatta gagtttttag    3420
ttgtatgatt gataggtggg gaagtttttg ttgtgtaggt gtgaaagaga attttagttt    3480
tttttttttag agtgtagtgg ttagggtttt tgagagtttt tgagagggtt gttaggagta    3540
ttgtgtgttt atttggttgg tattgttttg tttgtatttg taaagttatg gtagtttgag    3600
ttatgggtat tatgggagtg tttggtggtt ttatggaaga gaattgagtt gtgtaagtag    3660
gaaatgttag ttgtattttg taggagagaa aaagtagtat atggttgttt tatttttatt    3720
tttttgtttg aagatggttt tgggttaggt gggagattgt agatgatttt agagttgttt    3780
tttaggtttg tgttttttttg ggttgtagag tttgggatgg tggttgttag ttttgatttt    3840
attggtggtt tttttttttag tttgtttttt aaattttgaa taaaatatgt tggggaaatg    3900
agtgtttaga gttgattatg gtttttttgtt gtttttagtt tttttatagt tattgtgttt    3960
tggatgggga tgtagtatgg gagttaatat agttgttagt tttttgtatt tgaaattgta    4020
tgatgatgta taaatgtttt tgtttttttat tggttattga gtaagtagtt tagggtttga    4080
tgatgtgatt tttattttgt tgtatgtgaa ggttatagag tagattattt agtttatgtt    4140
gaagggtata tgggtgttta gggtagtaga gagtagagga tgtgataggg atgtgtgatg    4200
ttggtttagg tttgggtatt tgagttgatg agtgttggtt ggtgtttagt gtttggaaat    4260
tgtatatgtg tgttgggttg tttgtgtatt taaggaagtg tagttttatt ggaagaggtt    4320
tgttagtgtt gagttaggag ggagttttag ttatttattt gtattgtttg tttttttttt    4380
tttatggtgt tttttttttt tttttatatg gagttttgtt ttgttgttta ggttggagtg    4440
tagtggtgta attttggttt atggtaagtt ttgttttttg ggtttatgtt attttttttgt    4500
tttagttttt tgtgtagttg ggattatagg tgtttgttat tatgtttggt taattttttt    4560
gtattttttag tagagatggg gttttattgt gttagttagg atggttttaa tttttttgatt    4620
ttgtgatttg tgtattttag tttttttaaag tgttgggatt ataggtgtga gttattgtat    4680
ttggtttttt ttatggtgtt ttttgttttt gtttttgttt ttgagatgaa gatttgttgt    4740
gttatttagg ttggagtgta gtggtgtggt tttggtttat tgtaagtttt gtttgttggg    4800
tttatgttat tttttttgttt gagtttttta agtagttggg attataggtg tttgttatta    4860
tgtttggtta attatttttt tttgtatttt tagtggagat ggggttttat tgtgttagga    4920
tggttttaat tttttgattt tatgatttat ttgttttagt tttttaaagt gttgggatta    4980
taggtgtgag ttattgtgtt tggtttttttt tatggtattt tttagagata gggttttgtt    5040
atgttggtta ggttgatgta gaatttttttg gttttaagtg atttgttttt tttagttttt    5100
taagaggttg ggattatagg tgtgagttat atttttggtt gtattttttta atatattaaa    5160
```

aatagtattt aaaggttggg tgttggtggt ttatgtttgt aattttagta ttttgggagg 5220
ttgaggtggg tggattattt gaggttagga gtttgagatt agtatgatta atatggtgaa 5280
attttttttt taataaaagt ataaatatta gttaggtgtg gtggtgggtg tttgtaattt 5340
tagttatttg ggaggttgag gtaggagaat tgtttgaatt tgggagttag aggttgtagt 5400
gagttgagat gatattattg tattttagtt tgggtgataa gagtgaaatt ttgttttaaa 5460
aaaagtattt aaatattatt tttgtttgtg tttagattga aatgttttga atgtattagt 5520
atatgttttt attttttttt tttttggagt gaaaatattt aatttgaatg aaagtaatga 5580
gtaattgtat tttttgtaga gtaagttata agtatagtga atagaaaaat ttggttgggt 5640
gtggtggttt atgtttgtaa tattagtatt ttgggaggtt gaggtaggtg gattatttga 5700
ggttaggagt tggagattag tgtgattaat atggtgaaat tttattttta ttaaaaatat 5760
aaaaattagt taggtatgtt ggtgggtttt tgtaatttta gttaatttta gaggttgaga 5820
taggagaatt gtttgaattt ggaaggtgga ggtagtagtg agttgagatt gtgttattgt 5880
attttagttt gggtaatagg agtgaaattt tgttttaaaa aaaaaaaaaa gaaaagaaga 5940
tttgggttgt gttagtgtta gagtgtagta aaatttattt gtttgtagtg agaagtgtta 6000
ggaggggatt atggaagatg tttttaatgg aggtgattaa agtgattatt tatgtggttt 6060
ttaggatttt tgatggaaat aagtagattt gtggtttttt ttggtagttt ttggatttaa 6120
gtgtgtgtgg ttggaggggg tttttggggg tgaggttatt gttttgggtg gtttttttag 6180
atttattgtt tgagtagtat tgggggttta gttagtgttt tttgttagtt gttgggtttt 6240
ttgttatttt tagggagagt tttgagaatt tgtttagagt agaagatggg ggttggtgtt 6300
ttggattttg tttagtgatt ttgtgtgttt ttttagttga tagggagttt attgaggtta 6360
ggaggagagt tttgaagtgt tttgttaatt tggtggtgtg atattttttg ttttttgagg 6420
atgtggtttt taagtttttt ttgtttttta gtggtttggt gagtgtgttt gttttgttta 6480
tgttttggtt ttggggagtt tttgagtttt tggatttgtt tttttttttt ttaggtgtgg 6540
tttttattt ttttggtttt ttgtttggat gtttagtagg aatgatatgg ataagatttt 6600
tttttttttt agataatagt ttgaatgtga tttttagagg tgtttttggt gttgatttg 6660

taggatttg taaatttgtg tggagattat ggatttttgt ggttattttg aggatttagg 6720
ttggatggtt tggagggttt agggaggtgt tagagatttt agatgggaag aagtgttgat 6780
ttaaaataag gttttgtttg attaaggatt attggaatat aggttgagtt tttttgttag 6840
agttatagtt tatatttgta tttgggaatt aatttgttgt atatgtattt tgttagaggt 6900
ttgagtgggg tttttttttt tttagttgtt gtggtatagt tttattgtgt gttaagaagt 6960
tatagtttgt aggtatagtg gtttatgttt ttaattttag tgtttggaga ggttgaggtt 7020
ggatggtttt ttaagtttag gtgtttgaga ttagtttgga taatatagtg agattttgtt 7080
tttattaaaa taaaattgag gttaggtatg gtggtgtata tttgtaattt tagttatttg 7140
ggaggttgag gtatgagaat tgtttgaatt taggaggtgg agtttgtagt gagttgtgat 7200
tgtgttattg tattttagtt tgggtgatag agtgagattt tgttttaaaa aaataaaaaa 7260
taattttgta gtttagtttt tgttttttgg aggaggaagt agttgttagt ttttttaggg 7320
aaggtgggtt ttgtgttttt tttttatttg tgtttgtagg attttttttt tgtgtgggga 7380
ggggtggggg ttttattgga atagaggttt ttaggttttt ttttatttta gggaaatttt 7440
gttttgaatg gggatttgat ggtttttgtt tttgtttttg ttgtaggaag atattttttt 7500
ttttaggttg attttatata agttttggtt tttttgttt ttgttttttt tggaggtaga 7560
ggggtttttt ttttgtgttt gagatgtttt tgggatgttt ttggggatgt ttggagtagg 7620
tagtgggatt ttttgagttt tggggttgga aatttttttt tggaggtgat ttgtattgtt 7680
agtagaggaa tgtggtagga tttgggaggg gtggtgggta ggggtgtagt gggttatagg 7740
tgggtaatat ttttgttttg ggggtaggat gttttttgtg gtttttttat aggtattatt 7800
ttttgttatt ttttggtttt agttttttttg tttgtttttt gtttttagag atggggtttt 7860
attatgttat ttaggttggt tttgaatttt tgggttttag tgattttttt gttttaggtt 7920
ttgagtagtt gggattatag gtatgtatta tgttgggttt taggtttttt tttttttttt 7980
tttttttttt tttgtaaaat gagagttgag ttagatgatt tttaaggtat ttttgaaatt 8040
tgagaatttt tggatatgta aatagttgta tattagggtt tttttttgta ttttttgggt 8100
ggtttttgat tttaaggtat ggttttgttt aggaggtttt ttttagatga gagggttttg 8160
gtgttagggg ttgggtttgt ttttttgttt agtagttttt tgggttttgg tgtttttggt 8220
gttatggatg tatttttagg aaggaagata tttttgtttt gttttattgt gtttttggaa 8280
gttattaggg ttttaggtat ttttggggtt gatgttgttt tgggtttgtg gatggagggg 8340
ttgtttttttt gtagagttgt ttttggtttg tggatggagt ggttgttttt ttggagagtt 8400
gtttttttgtt tgtggatgga gtggttgtgt ttttggagag aggttgtagg tttgtggtgt 8460
ttttgttgtg aagttaggtt gttttgtttg gtgagaaaaa tatttaaggg agagttaaga 8520
gattgtagaa tattaatgtt aaagttaata tatggaaatg tgtttttttt gaggaaagat 8580
gattttttgt tttatagtga tttatatttt atagtagttt ttttgttgtt agtagaagag 8640
ggtgaagttg tgtgtttttt ttttgtagga tgtgtagaat aagttaaagg agttagtata 8700
gtgtgttggg gatgaatttt tgaattgtaa gttggttatt agggttaagg tatttttttt 8760

```
tttttttttt tttttttttt ttttaattta tttttttatt gattattttt gggtgttttt    8820
tgtagagggg gatttggtag ggttatggga taatagtgga gggaaggtta gtagataaat    8880
aagtgaataa aggtttttgg tttttttagg tagaggattt tgtggttttt tgtagtgttt    8940
gtgtttttga ttatttgaga ttagggagtg gtgatgattt ttaatgagta tgttgttttt    9000
aagtatttgt ttaataaagt atattttgta ttgtttttaa tttatttaat tttgagtgga    9060
tatagtatag ggttgggggt aaggttatag attaatagga ttttaaggta gaggaatttt    9120
ttttagtata gaataaaatg aaaagttttt tatgtttatt ttttttttata tagatatggt    9180
aattatttga tttttaatt tttttttttat ttttttttgtt tttttatttt ataaagttgt    9240
tattgttatt ttggtttgtt tttaatgagt tgttgggtat attttttaga tggggtggtg    9300
gttgggtaga ggggtttttt attttttagt aggggtggtt gggtagaggt gtttttttatt    9360
ttttggatgg ggtggttggt taggtagggg ggttgatttt tttttatttt ttttttggat    9420
ggggtggttg gttgggtggg gggttgattt ttttattttt tttttggatg gggtggttgg    9480
ttgggtagag ggttttttta tttttttagta ggggtggttg ggtagaggtg tttttttattt    9540
tttggatggg gtggttggtt gggttggggg gttgattttt tttattttt ttttggatgg    9600
ggtggttggt tgggtagagg ggttttttat tttttagtag gggtggttgg gtagaggtgt    9660
tttttatttt ttagatgggg tggttggttg ggtggagggt tgattttttt attttttttt    9720
tggatagggt ggttggttag gtggggggtt gatttttttta tttttttttt ggatggtatg    9780
gttggttggg tgggggggtt gatttttttat tttttttttg gatggggtgg ttggttgggt    9840
gggggggttga tttttttttat tttttttttg gatggggtgg ttgttgggtg gagatgtttt    9900
ttatttttta gatggggtgg ttgttgggtg gagaggtttt ttattttttta gatagggtag    9960
ttgttgggtg gaggggtttt ttatttttta gatggggtgg ttgttaggta gagggttttt   10020
ttatttttta gatggggtgg ttgggtagag atgtttttta ttttttagat ggggttttgt   10080
tgggtagagg tgttttttat atttagatg gggtggttgg ttgggtggag ggttgatgtt   10140
ttttattttt tttttggatg gggtggttgg ttaggtgggg ggttgatttt tttatttttt   10200
ttttggatgg ggtggttggt taggtggtgg ggttgatttt tttatttttt ttttggatgg   10260
ggtggttggt tgggtagaga tgttttttat tttttagatg gggttttggt tgggtagagg   10320
tgttttttat atttagatg gggtggtggg gtagaggtgt tttttatatt ttagattatg   10380
ggtggttggg tagagatgtt ttttattttt tagatgtgat ggtggttggg aagaggtgtt   10440
ttttattttt tagatgggat ggtggttggg tggagatgtt ttttattttt tagattgggt   10500
agttaggtag aggggttttt tatattttag atgatgggtg gttaggtaga gatatttttt   10560
atttttttaga tggggtggtg gttgggtaga ggttgtagtt ttggtatttt gggaggttaa   10620
ggtaggtggt tgggaggtgt aggttgtagt gagttgagat tatgttattg tattttagtt   10680
tgggtattat tgagtattga gtgaatgaga ttttgtttgt aattttggta ttttgggagg   10740
ttgaggttgg tggattattt gtggttaggg gttggagatt ggtttggtta atatagtgaa   10800
attttgtttt tattaaaatt agttaggtgt ggtggtgtgt gtttgtaatt gtaggtattt   10860
ggtagattga ggtaggagaa ttaggtaggg aggttgtagt gagttgagat ggtagtagta   10920
tagtttagtt ttggttttgt atgagaggga gattgtgggg tgagggagag ggagaggggg   10980
agggggaggg ggagggggag ggagagggtt aaggtatttt tttaattttg aagagtatat   11040
ttttttttaa ttagagaaat aagttttaat tttttaaagt gattttgttt tttttgtgtg   11100
gtagggatta tgttgatatt tgttgtatgg tttttttatt taatttttgt tttagttgtt   11160
ttgatatagg gttgggaggt ttgttttatg gatggagaaa taggtttagg gaggttgtta   11220
tttgttaagg ttttgagttt ttaagtagta gggttgggtt gtggttgagt tttgttttta   11280
ggttttttag gtattttgtg tttttttgttt tttttttgtga tttagataga tggatgtttg   11340
tgttagtatt tttttagatt tttggggatt tattgttata tttgtttatt attaaatgtt   11400
taaagtttgg ttttttttta aaaaagatta aggtagttat ttggtagttt ttttgttttt   11460
ggttttttttt ttttaaattt tttttttagg ttggtgtggt ggtttttttt tgtaatttta   11520
gtattttggg aggttgaggt tgttggatta tttgaggtta ggagtttgag attagtttgg   11580
ttaatatagt aaaattttat ttttaataaa aatataaaat aaaaaaggaa aataaaaaaa   11640
agtaaaataa attttaaaaa attggttggg tgtggtggtt tatgtttgta attttagtat   11700
tttgggaggt tgaggtgggt ggattatgag gttaggagtt taagattagt ttgattaata   11760
tggtgaaatt ttgtttttat taaaaatata aaaattagtt gggtgtggtg gtgtgtattt   11820
gtaattttag ttatttagga ggttgaggta ggagaattgt ttgaatttag gaggaagagg   11880
ttggagtgag ttaagattgt gttattgtat tttagtttgg atgatagaga tattattaaa   11940
aaaaagaaaa aaaaaattag ttaggtttgg tggtaggtat ttgtaatttt agttatttgg   12000
gaggttgagg taagaggatt gtttgaattt aggaggtaga ggttgtagtg agtttaggtt   12060
gtgttattgt attttagttt gggtaataga gtgagatttt gtgttaaaga tgataataat   12120
aataataaaa aaattagaaa aaaatttgtt tttttatata aaaattaaaa atttatttta   12180
ttttattttt tgagataagg ttttgttttg ttttttaggt tatagtgtag tggtgtaatt   12240
gtaggttatt gtagttttga ttttttgagt ttaagtaatt tttttatttt agttttttag   12300
aatagttggg attataggta tgtattatag tatttggttg atttgtttgt ttgtttttgg   12360
tatagatgaa gttttattat gttgtttagg ttggttttga atttttggtt ttaagtaatt   12420
```

```
tttttggtat ggttttttaa agtattggga ttaaaggtat gagttattat gtttggttaa   12480
atttttttatt tttattttat tttttgagtt ttttgttttt tgaattttga tagttagtat   12540
taaattttat aaaaagtttt tgggaataaa tgtggtatag aagaggtttt ttttttatgt   12600
ggagagtttt ttaggaattt atgtttattg tgtttaattt gaagattgtt tgttttttatt   12660
tttgttgttt tttggggata gggtttttag gttggagtgt agtgttgtga ttatggtttt   12720
ttgtagtatg gaatttttgg gtttaagtga ttttttttgtt ttagtttttt gtaagtattt   12780
gggattatag gtgttttatt atgtttggtt aattttttga tttttttggga gagatggggt   12840
tttattatgt tgtttaggtt gtttttaaat ttttaggttt tagtaatttt tttgttttag   12900
ttttttagag tgttggggta taggtgtgag ttattgtatt tagttaaaga ttagtattgt   12960
tagtttttga gatggagttt tgttttgttg tttaggttgg ggtgtagtgg tgtgattgta   13020
gtttattgta agtttgttt ttggggttta tgttattttt ttgttttagt tttttaagta   13080
gttgggatta ggtgtttatt attaggtttg gttaattttt tgtattttta gtagagatgg   13140
ggttttatta tagttttgat ttttttattt tgtgatttgt tttatttggt tttttaaagt   13200
gttgggatta taggtgtgag ttattgtgtt tggtaagatt attatttgag tgttttgttg   13260
gtttgtttgt tttgagatag agttttgttt tgttgtttaa attggagtgt ggttgtgtga   13320
ttttagttta ttgtaatttt tgttttttgg atttaagtaa ttttttttgtt ttagtttttg   13380
gagtagttgg gattataggt gtatgttgtt atgtttagtt aatttttgta tttttagtgg   13440
agatggggtt ttattatatt ggttaggttg gttttgaatt tttgatttga ggtgatttat   13500
ttgttttagt ttttaaagtg ttgggattat aggtgtgagt gattgttttt ggttttattt   13560
gagtgttttt ggataggttt tttagtgtga tgagatttta gtggattttg tggtttttat   13620
tttatgggat tatttgtttt tgtgtttgtg gtatgtaggt gatgatggtt tttgtttttt   13680
ttttggatat atagagtgat ttttgtaggt ttggaggagt ggggtttttg gggtggttta   13740
tggatttgtt gtgtttttttg tggtttagtt tttgggggat tggttgttgt tgtttgatag   13800
gtgtttttta gtgattaata tagtgttttt tttttttatta tgatgttagg attttttttt   13860
agttgatatt taggtttagt ttgttattag ttgggagttg atttggaata tttataatag   13920
tttttataag ttttgtgata gggttgagtg gattgtattg tgggttattg ataatgtggt   13980
agattttttt atatttggga aggagttaag gtaggtgatt ttttagtttt tgtaggggta   14040
tgtgtggtgg gatgggaatt tatgttggtg aagggtttgg gtggaaagtg tgtttattgt   14100
aataattagt attttgattt gttttatttt agaaggggta gaaagtggtt taggtatatg   14160
gggggttata tttggaagaa atatgggaga attttggttt tgaaatatgg atttttgagt   14220
atgagtaaat gtttattagt ttttagtttg gtagtttttt tattgttagg atgagatttt   14280
aaaaaaagat tttaggttag gtgtggtggt ttatatttgt aattttagta ttttgggagg   14340
ttgaggtggg tggattatga ggttaggagt ttaagattag tttggttaag atggtgaaat   14400
ttgtttttat taaaaatata aaaaaaagtt ttttgggtgt ggtggtgggt gtttgttatt   14460
ttagttattt gggaggttga ggtagagaat tgtttgaatt taggaggtgg aggttgtagt   14520
gagttgagat tgtgttattg tattttagtt tgggtgatat agtgagattt tgttttaaaa   14580
aaaaaaaaga tttttttgat ttggggaaga atagattatg ggttggtagt gttttttttg   14640
tttgaggggga ggggagattt tatttgggat tttgtttgtt ttagtagtgt ttgttatttg   14700
tgtatatatg ttatttgggg gttggttggg aagaagagat gtagtaggtt ttgaggtgta   14760
gttttagggt tattttttttt tttgggtttt tagggttttt ataggttatt ttttgagtag   14820
tagggtttta ggatggggtt atgtaggttg ttgtggagga tatttgttgg gggggatgtt   14880
agatttgatt gttaggtgag ttttaggttt tgagtgtttt ttttttttgtg tgtttgtttt   14940
tttagtgtaa tagggtttga tatgatttg ttgtttttttt gggttgtttt gaatagtagt   15000
atgtgggggt ttttgaagta ggttttgaaa ggtttgtttg tggaatttgt gttgtttgtt   15060
gataaggttg tataataggt gagtggattg gttttttttag gtgtttgttt tagagaatat   15120
gttatgtttt tgtatgggga gtgtttttttg tttagatggg tgttatagtt tgtttttgtt   15180
ttgttgtttt gagaaggagt tttttttttgt tgttaggttg gagtgtaaag gtgtaatttt   15240
ggtttgttgt aatttttgtt ttttgggttt aagtgatttt tttgttttag ttttttgagt   15300
agttgggatt ataggtgttt attattatgt ttggttaatt ttttgtattt ttagtagaga   15360
tgaggtttta ttatgtttgt taggttggtt ttgaattttt gattttaggt gatttgtttg   15420
ttttggtttt ttaaagtgtt gggattatag gtgtgagtta ttgtgtttgg ttggtttttt   15480
agttttgagt tgagtgaatg atagttgtgt tttgggtggg agttgtttat ttatatttgt   15540
ttttggtggt tttttgggag tgtttatgtt tttgagaggg ggttgttagt gataaaa      15597
```

<210> 247
<211> 3143
<212> DNA
<213> Artificial Sequence

<220>
<223> chemically treated genomic DNA (Homo sapiens)

<400> 247

```
ttatttaaat ggggttgtgt tttgattatt agtgtaatgt gattatttga ttttaaatgt      60
aattatgtgt ttaaatggtt gagtgtaaga ggttggttgg agatagggtt gagtgtgttg     120
ttttaagttg tgatttttgt taagtattta tggataatgt attggttttt tttagggagt     180
gtttttttatt tagggtagta gtaggagtgg aatttggagt tgtatatgtt gggtttgtga     240
tggataagtg tggttatgta atagataggt agagggatgt agtaatatat aaatggtttt     300
tgtaagtaat gttttgagtg agtagatttt aggataataa ataaattaaa attatatttt     360
aaattattgg agattgggag gtagatgtgg aagtaaaaga gttagatgtt ggaatgtagg     420
agggttagaa atgtttaaat attgtttttt atttgttgtt ttttgtttgt tttttttaaag     480
ggtgaaaaga ggtatggaaa tatataattt gttattattt tagtttttag atgtagagaa     540
taaagggatt attttgtttt aaagaatttt tgtttttttt tttaaattta ttttttttttg     600
gttaattgtt attttggttt tgatgtgtta aggaaagtgt gttgtaaggt ggagtgtttg     660
ggttgatgat gagaaggtga gttgtgaatt ttgtatatgt ttttaaataa aaggtttatg     720
agtggtggtt ttgatgtggg gagtaagtag gtttttttgg tttttgtagt aggtggattt     780
aggtttgttt gttgttggat atttagaatt tgtttttgta gtgtgagttt tgtatatgtt     840
ttgtgattgg tatttttgga ttttttaatt atttgttgtt taatttttgg agagagggtt     900
ttttgtttgt tgagttgtgg ggttgggaag gaaaaggggg atgtgttttt gggggttttt     960
ggatattttg gtaaaggggg ttgtagtttt aggttttagt taaagggttt gggatggagt    1020
gggttagaaa gttgttaaat tgaggatttt tttttttttt aagttggagt tttaggtgtg    1080
gagttgtttg tttatttatt tttggagtat gttttgttgt ttttgttggt ggatgtggtt    1140
ggttattgtg gtattttttt tgtgatattt gggtgttaga aatttgtttt tagagtttgt    1200
tgtagtggat tgaatttgtt gtggtggtgg ttggggttgg ggtggtttgg gtggaggagg    1260
ttgaggttgg ttggttgtgt tttgtgttta gagtattata tagtttagtt ataggaagta    1320
gagttggaga tatagttttt gttttttttt tattgttggt gttgaaagtg tgtggttgta    1380
gatttgtgga gagggagtga ttaaagataa aatttgtgtg ttttagaaaa gagttagggt    1440
ttaataagtt ttttgttggt tttggggatt tttttgggtt tgggtttttt gttttagttg    1500
gagattttga tagagtgttg gttttttgat tgtttgtgaa gtgagatgtg gggtgttggg    1560
tttagtgtag tgagtggtga ggtgtggtgg aggtgtagtt ggtagttttg agtggtatat    1620
gttgtataat tggtttaaag ttttgagtga ttttaggatt gttagtgggt gggggaggaa    1680
attataaaag ttttggtgta aaattttaaa aaatgtgaat tttaaaagaa aaaagggttt    1740
gtatgtagag tttggtggat ttttgtgtgt tttgaagatg gatattggaa agttgtaaaa    1800
gttattagaa agaaataggt aagtaaataa aagttgtttt tagaagaaga ggagggtttt    1860
ttttgggttt gtttggagtt ttatagggta atgaagtgtg ggtagtggtt gtggagttgg    1920
gtggaggtgt gtggggttgg ggtgtgtggt tagtaagaga gagggtggga ggttgggtgt    1980
tggagaagat gtggtggtgg agggtgtggt gtgggggttt tagaggtttg gtggggtgtg    2040
gggtgggttg gtgggtggtg gttgttagtg ggggtgtggt tgtttatatg tggggtttaa    2100
tttggattga gaggttggtg tagagtgggt ggggatagag taagggagag attgggattg    2160
aggaatgagg ggaattaggg ttgggtgagg ggaagaggaa ggaagggggg gagggtgaag    2220
ggagggaaga ggatggagat tggggagaag gggggtgaat ttgagaggaa ggttgggttg    2280
gaagagggat ttggaaagag ggattggtga gtttgggtgt gagaggaaat gagtggggga    2340
tgggggtgaa gttagggtaa agagagaagt gggaaaggag agggagggtt aaggtttggg    2400
tgttgagagg aggggtttgg ggggttggtt ggagggggtg aggtgttagg tggttttgtt    2460
ttttgttttt gttaattggt tgttagtttt gttttgattg tttgtataaa gtgagtagat    2520
ggtgggggat ggggtgatta gggattggat ttgggaaagg aggtagtagt tttttttttt    2580
ttttttttgt ttttgtttgg ttgtggatta gggattaggg tgtttaagtg tgttaggttt    2640
ggttggattt gtagttgaat tgatttgtag ttgaattttt ttgttggata gaggttgtaa    2700
ggttgaggat gagaatgggt ttttttgtag ggttttggtt tttgatttgg gatgggggga    2760
tttgtagtta gttaggaata gaggtttatg gggttgttgg ttgtagtttt aatttaagaa    2820
ggaggagatt ttttttttgt ttttttattt ttattgtttt attgggagtg gtgttagttt    2880
gtgggattaa attaggggtt gggagttttt agattgaaat gtgtttttta ttattaagag    2940
ggagtttatg tggttgtggg gggttgaggt agttagttgg tgttgtggtt ttggggagtt    3000
tgtgtagggg tgtgttttgg tgtgaaattt ggtaggattt tgtttgggtt tgtatgtagg    3060
gaggggttag agattttgaa atgtattagt gggtagggtg gtgataaggt gggtaaatga    3120
ggtttggggt atgtttttgt ttt                                            3143
```

<210> 248
<211> 3143
<212> DNA
<213> Artificial Sequence

<220>
<223> chemically treated genomic DNA (Homo sapiens)

<400> 248

```
gaagtaaggg tatgttttga gttttattta tttgttttgt tattattttg tttgttagta      60
tattttagag tttttaattt ttttttgtat gtaagtttag gtagagtttt gttaggtttt     120
atattagagt atatttttgt atggattttt taggattgtg gtgttggttg gttgttttgg     180
ttttttgtgg ttgtgtgagt ttttttttgg tggtggaggg tgtattttaa tttggaaatt     240
tttagttttt aatttggttt tgtagattga tgttgttttt ggtggggtgg tgggggtagg     300
agggtaaagg ggaggttttt tttttttttgg attgaggttg taattaatag ttttgtgagt     360
ttttattttt ggttgattgt gaattttttt attttaggtt aggggttggg atttataga     420
aagatttgtt tttattttta attttgtggt ttttgtttgg tggggaagtt tagttgtggg     480
ttagtttagt tgtgggtttg gttaagtttg gtgtgtttag gtattttagt ttttggtttg     540
tggttgggtg aggatggagg gggaggggaa ggggttgttg tttttttttt taaatttggt     600
ttttaattgt tttatttttt gttgtttatt tgttttgtat aggtagttaa aatagagtta     660
gtggttaatt gatggggatg ggaagtaaag ttgtttagtg ttttgttttt tttagttggt     720
tttttgggtt tttttttttg gtgtttggat tttggttttt tttttttttt tttatttttt     780
tttttgtttt aatttgtttt ttattttttg tttatttttt tttgtatttg ggtttgttaa     840
tttttttttt taagtttttt ttttagtttg gttttttttt tgggtttgtt tttttttttt     900
ttaattttttg tttttttttt tttttttgtt tttttttttt tttttttttt tttttttatt     960
taattttggt tttttttgtt ttttagtttt gatttttttt ttattttgtt tttgtttatt    1020
ttgtgttggt tttttagttt gggttgagtt ttatgtgtgg atggttgtgt ttttattgat    1080
agttgttgtt tgttggtttg ttttgtgttt tgttgggttt ttaaaatttt tgtgttgtgt    1140
tttttattgt tgtatttttt ttagtgttta gtttttgtt ttttttttttg ttggttgtat    1200
gttttggttt tgtgtatttt tgtttggttt tgtagttgtt atttgtgttt tgttgttttg    1260
tgggattttg agtgagtttg gagggaattt tttttttttt ttgggggtga tttttgtttg    1320
tttgtttgtt tttttttggt gatttttgta gtttttttaat atttgttttt ggagtgtatg    1380
ggaatttgtt gagttttgtg tgtaggtttt tttttttttt gaggtttata ttttttgaaa    1440
ttttatgtta gggtttttgt aattttttttt tttgtttgtt gatggttttg gagttgtttg    1500
gggttttagg ttggttatgt aatgtgtatt gtttggggtt gttggttgta tttttgttgt    1560
gttttgttgt ttattgtgtt agatttggtg ttttgtgttt tgttttgtgg gtagttaggg    1620
ggttggtgtt ttgttgaggt ttttagttag agtagggagt ttgagtttga gggagttttt    1680
ggagttgatg aagggtttat tagattttga tttttttttg aggtgtgtag attttgtttt    1740
tgattatttt ttttttgtgg gtttatggtt gtgtgttttt ggtgttggtg atggggagaa    1800
gatggaggtt gtgtttttag ttttattttt tgtggttggg ttgtgtggtg ttttgggtgt    1860
agggtatggt tggttagttt tagttttttt tgtttaagtt gttttggttt tagttgttgt    1920
tgtaataggt ttggtttgtt atagtaggtt ttgaaggtgg gtttttagtg tttgagtatt    1980
gtgaggaggg tgttgtagtg gttagttgtg tttgttggtg aggatagtag gatgtgtttt    2040
gagggtaagt gggtaagtgg ttttgtattt agggttttgg tttgggggag gggggaattt    2100
ttagtttggt ggtttttttgg tttattttgt tttagatttt ttagttggag tttagagttg    2160
tagtttttttt tgttagaata tttaaagatt tttaggagtg tgtttttttt tttttttttta    2220
attttgtagt ttagtgggtg gaaagttttt tttttggggg ttgggtggtg ggtggttagg    2280
gggtttaggg gtgttgattg tagagtgtgt gtagagtttg tgttgtggga ataggttttg    2340
aatgtttggt ggtaggtggg tttgggtttg tttgttgtag gggttagaga agtttgtttg    2400

tttttttatgt tggggttgtt gtttgtgagt tttttgtttg aggatgtgtg tagggtttat    2460
agtttatttt tttattgtta atttgagtgt tttattttgt gatgtgtttt ttttgatatg    2520
ttggggttaa agtaatagtt gattaaggag gaatggattt gggaaggagg gtaaggattt    2580
tttggaatgg aatggttttt ttgtttttttg tatttggaag ttagaatagt agtaaattat    2640
atgtttttat gttttttttt gtttttttaaa aaggtaggta agggatgata gatgaaaggt    2700
agtgtttaga tatttttgat ttttttgtat tttagtattt agtttttttg tttttatgtt    2760
tgttttttttga tttttaataa tttgaagtgt aattttgatt tgtttgttgt tttgaaattt    2820
atttgtttgg ggtattgttt atgaagattg tttatatgtt gttgtatttt tttatttatt    2880
tgttatgtga ttgtgtttgt ttattatagg tttaatgtgt gtggttttag attttatttt    2940
tattgttgtt ttggatggaa gatgtttttt ggaggaaatt agtgtattgt ttgtgagtgt    3000
ttggtaggga ttgtagtttg gggtagtgta tttggtttta tttttggttg attttttgta    3060
tttagttatt tggatgtata gttgtgttta aaattaagtg attatattgt attgataatt    3120
gggatgtggt tttatttgaa tag                                          3143
```

<210> 249
<211> 6676
<212> DNA
<213> Artificial Sequence

<220>
<223> chemically treated genomic DNA (Homo sapiens)

<400> 249

```
gattaaatga aatattgtaa tataaggtga ggtaggtttg tgggaattgg tttttagtgg      60
gtggtggtag agtggggagg attggagggg ttagggttgg gggtggtatg ggtttttggg     120
ttgttgggtt tttttgggtg ggtttagggt tggaaggtgt tgttagttgt agttaggttt     180
atattttttta atttgttgtt ttttttttat tttatgtgtt ggttttggaa ttagattttg     240
atttggtgtt tggataggta gagtgtgtgg gtgattttga tgtgttgttg ttgggttagg     300
tagtggttga agtggaattt ttttttagt tttagggttt ggtagtgggt atatgtggtt     360
tgggtttttt ttttgtttgg tttggttata tttggagtag atagaggaaa gttgtaaggt     420
aatattattt tggttaaggg agggggtatt gggtgggagg gggtggggga gtaggattta     480
ggtgttttgg tatttagttt attatagttt taatttttttt tattttagat gtttatattt     540
aagtttgatt tttgtatttt tttttattgt atttttttatt tttgtaggta gttagtgtgt     600
ttggttttgt tttttgtttg tttttagtgt tttattttttg gttgggttta ggtggttgtt     660
tgttttggga gggaggagag tggttgtatt tttgttttgg aaagtttttt ttattgttta     720
tttttttttt gttagtgata gttttaaaat tttaggggta agtaggattt tttttttatat     780
tatatgaatt ttattttttta ttttaaagtt ggtttagttg tgttaataaa aatttaagtt     840
gaagttggaa ataaattatt aaaaatattt tttttttaat agagagattt tttttggttt     900
tgtttatgaa gttatgaggt tttttttaga tattttttttt ttttagaaaa atgaatttat     960
atttagggta aagttaagtt tttttttgttt ttttgatttt attttaaaat gtagagaagg    1020
aaagttgaga agataaaaaa gagagagaga attattatgg ttgatgtgaa gttttttttat    1080
ttaggggaat atttgtggag tttgtttttt gggtgtggtt gtggaggtgg ttatgggttt    1140
tggttgtgtt tgagttaggt tggggttgtt tagttggttt gttgtttttt taggttttga    1200
ggatgtgttg gttttgttta ttttggtgaa attggtgttg gagttggttg aggttgtttg    1260
gttggagggg gatgaagtag agggtttggt gttgtggttg ttgttgttgg tgtagggtag    1320
ggatttgggt gaggataggg agtagtttga agttgtttgt ttgaagtggg gtttttgggt    1380
gggtgtgggg aaggtgtgtg agtttttgtt tattgtttta aagtggttgg aggaggttga    1440
ggagtggttg atgttgaggt ttattttatt gtaattgtag ttgtaagagt tggtgtgtat    1500
ggtagtggga tttttgtaag agttgtttag agagttgtta ttgttataat ttagtaattg    1560
atagtttggg ttatttggat aatgttttga gaaggagttt ataaagtatg agtttatttg    1620
ggtgattttg gagggtttga tttgtggatt gtggttgtta taattttgtg ttttatgatt    1680
tatgatgtat taatgaatta tagtgatgta ttgtattttg ttttgttatg tatgtggtta    1740
aatatggggg gggggttgga agggggggtgg ggggtgttag ggaattatgt gtttttgttg    1800
attagttgta aatttttgtt gatgatttta agaggtaatt tatgttttat ggttataaat    1860
aatgatgatt tgagaattgt tagggttgat ttaatgtgag tttttgagag agggggaaaa    1920
aaggagaagg gggagaaata gagagaaggt tggttttggt ttttgagtag agtgtgttat    1980
tttttggtga ttgatgggag tgtgggtgtg agattgttat ggtggtgggt ggttgttttt    2040
ttgttggttt tttgtaggtt ttttgtagtt gttgggagag aaagaaattt ggttaaggtt    2100
tagttatagg gttgagtttt ttagagtagg ggttgggttt tttatttatt tttttgtggg    2160
tggggttagt tttttttattt ggtttttttt ttttatatta ttattgttgt tattatttta    2220
attttggatg agaagattaa agatagagtt gtttttttttt tttttgggtt ttgtaagttt    2280
ttgaattgga ggtggaaatg ggggaagggt tggaagttgt gtgtgaaaaa taattattgt    2340
aatgggtttt ttttaggagt ttagtttggt ttgtattttg gtggaattgt tgttattggt    2400
ttatagtgtt taggttgagt tggtagggtt gggtgggagg tttgaaaaag aaggagaagt    2460
gtttattttt tttgggttat ttggaagttt gaaatttttt tttggatttt tttggtggtt    2520
tttagtttag gtgtggggag tgatagagta ggatagggat tttagaaatt gagtatagtt    2580
ggggttaagt ggtttagttg ttgaaaagat tgtagtggtt attgtgtttg ttgtttagtt    2640
tgagtagtaa tatgtgtttg agagtttgta aagtgggatt tttttttttt ttttttggtt    2700
attgtatttg gaggttttat tataggtttt ttatttgtag ttaggtagtt tgagaatttg    2760
tttggtggta gttgtttttt tggaaaaggg gttttaagga tttgtatgtt ttgttgagta    2820
tgttgggtgg ttagagattg ttgtagttgg tttttttatta ggaggggatt atttttttta    2880
aaaataagaa aggaagtgag agggtggagg aataaaggaa aatagtgatt gtagtttgtt    2940
ttttgggtgt tggtgtaagt agagagagaa tgagttattt tgggttgaga ggatgatatt    3000
aaaagtttta ttatttggtt ttttgttttg gggtttttaa ggagtaaggg aaagttgtgg    3060
```

```
tttttggttt gtgtttggtt ggtggtttta tattttttta tttgaggttg attttttatt    3120
tgaggttgat ttgttatttg aggtgggaag gggtatttgg agtttttagt tgtagggtga    3180
gagatattta gtggttttat tttttagttt ttagagttat aggttaggag tggagggtta    3240
aggttggtga aaaaatttgg ttatgtggat gggttaaaga ttagggggttg ttgtgaaggt    3300
gaggatagaa aagtgttgta gaggttttta gattatggtt ttgtttttgt tagagagttt    3360
taggggtttt agtggttttt ttagtgttgt gtatatatga ggaaaggtta gagaatgaga    3420
gggatatagt atttttttat tttttaagtt tgtatgggga gaagtggttg tgagttttag    3480
tattagggaa atgtagtgtt ggggtttggg taggattgat tgttttagtt tttattttag    3540
ttgtaatttt ttagttgaat agggtttgtt ttggtgggtt agggtttttg tttttgttga    3600
agaattttgg tttaggaaag atggagaggt tgggggttga ggagagagga aaaaatggta    3660
ggggaggatt ggaggtgatt gagtgttgag tttttatatt tttattattg ttgttgttgt    3720
tgttattatt attattatta ttattattat tattattatt attattgaag tattttatgt    3780
ttagagttaa gataagatta taaatataat atatagaaaa ttaataaaat agaattttgt    3840
ttttttttga ggtttttttt tttattttta ggtagtatgt gtttttttta gtggttttgg    3900
ggaggggggtg ggagagatga taggtttggg attagggagt ttttaaggtt tttgttgagg    3960
ggatagtgaa tttattattg aattgtgtat gggggatatg gataaaatga gatgtgatag    4020
ggataagagt attttgtttt gtttttagga aatattaagt gagtttgttt tttagttttt    4080
attaggaagt ttgattaggt tgaggtgagt tttttgggaa ggaagggtgt gtgggatgtt    4140
gggtgggttt gagtagtgag gttttagagt atttgttggg agttgtgtgg gtgggggtgt    4200
ttaggagagt gttgggtttt gtttgtttgt gttggagagt aggtttttttg gtttttttat    4260
ttgagagttt tttttttttgg gttttttttga tgtttttggt tttttatagg tttttttttt    4320
tgtgtttttt tttttttttt agtattttta tttggtttgt tttttttttt tttttttggt    4380
attgagttga gatgtgttga gtagtttgtt tttttttttt tattttatgt gttggttttg    4440
gaattatatt ttgatttgtt tttttgttag gtatagggtg tgtgtgattt tgatgtgttg    4500
ttgttgtgtt aggtagtgat tgtagtgaaa tttttttttt agtttagtg tttggtaatg     4560
tgtgtatgtt tggtggtttt gttggttgtt gggtttaaag gaggaatttg ttatgtagag    4620
tggagatgtt gaggtttgtg gttattgggt ttaggatttt tttttttagt tgatttttga    4680
atatagattt tagttagtat tgggatgttt tttattttgt tggtttttttt tttttgtttt   4740
gtattttttt agtgtttttt ttagattttt ttttagtttt ttaggtttgt gttgtttgtt    4800
tagattttag atgggggagg ggaggagtag tttgaatttt tatttgagtt tggggggagg    4860
ggttggttag gtgtgttttt ttttagttgt attttgtttt tttttttttt tttatttatt    4920
ttgtttttat tttttgttat tttttttaatt taatgataaa tttaggttgt taatttgtaa   4980
tgatgtagat tgatttatag tttatattaa tggtttttta tttttgagtt tggttaatgg    5040
atattagttg ggatttaagg ttaataaata atttaatttg agattttgtg tttgtttttt    5100
tttttttttgt tttgtttgtt tttttttttt tttttttttt tatttttttt tttttttttg   5160
attattattt tttttttggt gttttatttt gttttagttt tttatttata gggaaatata    5220
gttttagata gatttaattt ttttttttta gtgttatttt ttattttttt gtatgatgta    5280
ttttgttttt aatggagttg tttttggttg gggaatttta ttagggttgg gagaggggggg   5340
ttgggggttt aaagtaaagt taagttagtg attgtaggtt tttgtttttt tttttgtttt    5400
ggaattttgg ttttggttag gtgttttagg agagtatatt tgttttttat ggtttttagt    5460
gtggttggga ttttgtaaat tttgtaattt ttgtttaata ggggatatag tgagagattt    5520
ttggttggtg tttaattttt ttttattttt taattgagat gtttatttag tattttgtgg    5580
gagattgggg tagggggtgt ttattagttt tgtgttttgg ggtgggtgtt taggggagtt    5640
ggggtgatgg tgatgggaag ttttatttat tgttgtatga atttatttgt tgtatttatg    5700
ggtagattgg agtggagtat ttttgttttt ttgttttgtt gaatatgttt ttgttttgtg    5760
tgtagtttga tttttgtggt ttggggtgga atgggggttg tttgttggtg ttggagagtg    5820
tgtaggttga tttttttttg tggtagaagg ttggtgttgg tttgtagttg tagggtgttg    5880
tttggttgta gttattgttt gttggtgggt aataggagga agtggtaaag tttttgtttt    5940
ggtttggttt tggtttgtag ggtgtggggt aatgttttag tgggtttgta tagtttgatg    6000
aatagagtgg tagttggttt aggaaggatt tttgtttgtt ggttagagtg atggggaagg    6060
tggagtttat gaaataggaa tttattggga ggggaggtgt gtgtggttgt tgttgttttg    6120
ttgggtttat gatttgttgt gttttatagt ttgagtgttg tgtttattag tagtatattt    6180
gagattgggt tttagttgag gggggatggt gaggtgttag tgagggttag aaggaaatat    6240
aattatttga tttaatattg attaatggta gaggtaggaa ttgttaaata gtatttagga    6300
ggagtgtagt ttgtatgagg gatttgtgta taaatttatt aatttttttt tttttttttt    6360
tttttttttg tttttttttt tttttttttt aataatataa tgtgttttgt tttatttatg    6420
taggatttgt gaaataggtt tagtgttttt gagggaggag ggaatgttta gattgagatt    6480

attttatttt tgggtgatta aataaatatg tttttttttt attttatgta ggattggaaa    6540
aagttaattg tgtttttaga aaagttaatt tttgtgttgt tttgattttt ttttttttggg   6600
gagggttggg taaatgtttt tgaattgttt tgagagagaa ttaaagaagg tttgttttta    6660
```

gggttttgtg ttggta 6676

<210> 250
<211> 6676
<212> DNA
<213> Artificial Sequence

<220>
<223> chemically treated genomic DNA (Homo sapiens)

<400> 250

tattgatata aaattttaga aataaatttt ttttagtttt tttttaaggt gatttggaga 60
tatttattta atttttttg gagagaaggg gttgaagtaa tgtaggggtt aattttttg 120
aaaatataat taatttttt taattttgtg tggggtgggg gagagatata tttatttaat 180
tatttgggag tggggtggtt ttaatttaaa tgtttttttt ttttttaaag gtattgagtt 240
tgttttatag attttatgta ggtggaatag gatgtgttgt gttgttgggg ggagagagga 300
gaaaggtgaa ggagaaggag gaaaaaaaaa aaggttgata ggtttgtgtg tgggtttttt 360
gtgtgggttg tgtttttttt gggtgttatt tgataatttt tgtttttgtt attggttagt 420
gttggattag atggttgtat ttttttttgg tttttattgg tattttgtta ttttttttta 480
gttaaaattt aattttggat atattattaa taggtgtggt gtttggatta taaaatataa 540
taaattataa atttggtgga gtagtagtgg ttgtgtgtgt tttttttttt aatgagtttt 600
tattttgtga atttatttt ttttgttatt ttggttagtg ggtaggagtt ttttttgggt 660
tagttattgt tttatttgtt gggttatgtg gatttgttga gatattattt tgtgttttat 720
gggttagggt tgggttagga taagggtttt gttatttttt tttattattt gttggtgggt 780
ggtggttatg gttgagtggt gttttgtgat tatgggttgg tgttggtttt ttattgtgag 840
aaagagttgg tttgtgtatt ttttggtgtt gatgagtagt ttttgtttta ttttgagttg 900
tggaagttgg attgtgtgta ggataagagt gtgtttggtg agatagaaga gtagaagtgt 960
tttattttgg tttatttgtg gatgtagtgg atgaatttgt gtaatagtga gtgagatttt 1020
ttgttgttgt tgttttggtt tttttgggtg tttattttgg gatatagaat tagtgagtgt 1080
tttttgtttt aatttttat agggtgttgg gtgggtattt tagttaggag atagaagaag 1140
attgggtgtt ggttgggggt tttttgttgt gttttttatt aggtaggagt tataaagttt 1200
gtaaagtttt agttgtattg ggagttatgg ggagtaagtg tgttttttg gggtgtttgg 1260
ttagagttgg ggttttagga tagggaaggg agtaggagtt tgtagttatt ggtttggttt 1320
tattttgagt ttttaatttt ttttttttag ttttggtagg gttttttaat taaagatggt 1380
tttattaaaa atggagtgtg ttatgtaagg gggtaggggg tgatgttgaa gagaaagagt 1440
tgagtttgtt tgggattgtg ttttttttgtg ggtggggagt tggggtaaga tgaggtatta 1500
ggaaaggggt ggtagttaga ggagggagga ggaataaggg gaaggaagag agagggagta 1560
ggtggagtga gagagggaga aataggtgtg gggttttagg ttggattatt tgttggtttt 1620
aagttttaat tgatgtttat tagttggatt tgaaagtgag gagttgttaa tgtggattat 1680
ggattgattt atgttattat ggattaatgg tttggattta ttattgggtt gggggatga 1740
tggggggtgg gaataagatg gatggaaagg gagggaaaga taagatgtaa ttaggaagag 1800
atatatttga ttagtttttt tttttaggtt taggtgggag tttaaattgt tttttttttt 1860
ttttatttag agtttagata gatggtatag gtttaggaga ttaggaggga atttggaggg 1920
ggtgttggag gagtgtgaaa tgggggaagg gagttggtag aatagagggt attttggtat 1980
tggttggagt ttgtgtttga gggttgatta ggggaggggg ttttgggttt ggtgattgta 2040
ggttttagta tttttatttt gtgtaatagg tttttttttt gggtttagtg gttggtgagg 2100
ttgttagata tatatatgtt attagatgtt ggagttggag aaggagtttt attataattg 2160
ttatttgatg tggtggtggt gtattgagat tgtgtatgtt ttgtgtttga tggagaggta 2220
gattaagata tggttttaga attgatgtat gaagtggaaa aaggagagta aattgtttag 2280
tgtgttttag tttagtgttg aggaggagga agaaaaatag gttgagtgaa ggtgttggaa 2340
agggagggag gatgtgaggg gaaaggtttg tggggagttg agggtgttag agagatttgg 2400
gaaggaaggt ttttgggtgg gggagttagg agatttgttt tttggtgtag ataggtgggg 2460
tttagtgttt ttttggatgt ttttgtttgt atagtttttg gtgggtgttt tgaggtttta 2520
ttatttgagt ttatttagta ttttgtgtgt tttttttttt tgaggaattt gttttagttt 2580
gattaggttt tttggtgaga attgaggagt ggatttattt gatgtttttt ggaagtagag 2640
taaaatgttt ttgtttttgt tgtgttttat tttgtttatg ttttttgtgt atggtttaat 2700
ggtagatttg ttgttttttt agtgggggtt ttgaagattt tttgatttta gatttgttgt 2760
tttttttatt tttttttaa agttattgga aggagtatat attatttaga agtaagaaga 2820
ggagttttag aagaaaataa agttttattt tattaatttt ttatgtgttg tgtttgtagt 2880
tttgttttag ttttggatgt gaaatatttt gatgatgatg atgatgatga tgatgataat 2940

```
aataataata ataataataa taataataat aataaagatg tgaaaatttg atgtttggtt    3000
atttttaatt ttttttgtt atttttttt ttttttttaa tttttagttt ttttattttt    3060
tttgagttag aatttttag taaaggtgag agttttggtt tgttgaagtg agttttgttt    3120
gattgggaag ttatagttga gataaaggtt ggagtgatta gttttgttta ggttttagtg    3180
ttgtgttttt ttggtgttga ggtttatagt tgttttttt tgtgtagatt tgggggatgg    3240
agaggtgttg tgttttttt attttttagt tttttttgt gtatatatag tattaaggga    3300
gttattgagg tttttaaagt tttttggtgg agatggagtt atagtttggg ggtttttgta    3360
gtgttttttt gtttttattt ttgtagtgat ttttggtttt tggtttgttt atatggttag    3420
gttttttgt tagttttggt tttttgtttt tggtttgtgg ttttggagat tgaaaaatgg    3480
ggttattggg tattttttat tttgtagttg aaggttttag gtgttttttt ttattttaag    3540
tagtagatta gttttaagta ggagattagt tttaagtagg ggaatgtaaa gttattagtt    3600
aggtataggt tgaaggttat aattttttt tgttttttgg agattttagg gtaggaggtt    3660
aggtgatggg gtttttagtg ttattttttt aatttagaat ggtttatttt tttttgttt    3720
gtattggtat ttagaaggta agttgtggtt attgtttttt tttatttttt tgtttttttg    3780
tttttttttt tatttttaaa gaaaatagtt ttttttaat aggagattag ttgtggtggt    3840
ttttggttgt ttagtgtgtt tagtaaagta tgtgggtttt tggaattttt tttttgggaa    3900
ggtggttgtt attaggtaaa tttttaaatt gtttagttgt gagtgaggga tttgtagtgg    3960
ggtttttgaa tgtaatagtt gaggaggagg ggaagggatt ttgttttata aatttttaga    4020
tatatgttat tatttaaatt aagtagtaga tgtggtggtt gttgtagttt ttttggtaat    4080
taggttgttt agttttagtt gtgtttggtt tttggggttt ttgttttgtt ttgttatttt    4140
ttatgtttgg gttaaggatt attaaggagg tttggaaggg gattttgaat ttttaagtgg    4200
tttaggagaa gtgaatgttt tttttttttt tttaagtttt ttgtttagtt ttgttggttt    4260
ggtttgggtg ttgtggattg gtaatagtag ttttattagg atgtaggttg gattaagttt    4320
ttggggaaaa tttgttgtaa taattgtttt ttatatataa tttttaattt ttttttatt    4380
tttgttttta atttggagat ttgtaaagtt taagaaaggg aaaagtaatt ttatttttga    4440
ttttttattt taaagttaaa ataataatga taatgatgat gtagggagaa agaattaaat    4500
agagaggtta attttattta taagaaagtg ggtaaaaagt ttaattttg ttttaaaagg    4560
tttaattttg tggttgagtt ttggttgggt tttttttttt tttggtgatt gtagggagtt    4620
tgtagggagt tagtagggag gtagttgttt gttgttatgg tggttttatg tttgtgtttt    4680
tgttggttgt tgggaggtgg tgtgttttgt ttagaggtta aagttaattt tttttttgtt    4740
tttttttttt tttttttttt tttttttttt tggaggtttg tattgaattg gttttaatga    4800
tttttggatt gttattattt gtaattatag agtatgaatt attttttgag gttattagtg    4860
agaatttatg attggttaat aaaagtatgt gattttttaa tgtttttat tttttttta    4920
atttttttt tatatttggt tgtatatata gtaaaatgaa gtatagtgta ttgttataat    4980
ttattaatat attataaatt gtgaagtata gggttataat gattatgatt tataaattaa    5040
gtttttaaa attatttaaa tgagtttgta ttttgtaaat ttttttttgg ggtgttattt    5100
aaatggtttg gattattagt tgttaaatta tggtagtggt agtttttga gtggttttta    5160
tagggatttt gttgttatgt atattggttt ttatggttat aattataatg ggatggattt    5220
tagtgttaat tgttttttgg ttttttttag ttattttggg gtggtgggtg agagtttgtg    5280
tgtttttttt gtgtttgttt aggagtttg ttttaggtaa gtggttttga gttgtttttt    5340
gtttttgttt gagtttttgt tttgtattaa tggtgatagt tatggtgtta agttttttgt    5400
tttgtttttt tttgattagg tgattttagt tagttttagt gttaatttta ttgaaataga    5460
tgaggttagt gtgtttttgg agtttgagga agtggtaagt tagttaagta gttttagttt    5520
agtttgggtg tagttagagt ttatggttat ttttatagtt gtgtttgagg ggtagatttt    5580
gtaaatattt ttttggatga ggaagtttta tattagttat ggtaattttt tttttttttt    5640
ttgtttttt ggttttttt ttttgtgttt tggggtggga ttggggggat aaggagagtt    5700
tggttttatt ttaaatatag atttattttt ttaaggggga gaagtattta agaggggttt    5760
tatagttttg tagatagagt taaaaagggt tttttgtta agagggggt atttttaata    5820
atttgttttt agttttggtt taggtttta ttagtgtagt tgaattagtt ttaggataaa    5880
ggatggggtt tatataatgt ggggaagggt tttgtttatt tttgaaattt tgagattgtt    5940
gttagtaaag aaggggtgaa tagtaaaagg agtttttgg ggtaaaagtg taattgtttt    6000
tttttttttt ggggtgagtg gttgtttgag tttagttaag ggtgaggtat taggagtgag    6060
taagagataa agttgggtgt attggttgtt tgtagaagtg ggggatgtag tagaaagggg    6120
tgtagaaatt gagtttgaat gtgggtattt ggagtagaga ggattggagt tgtggtgagt    6180
tgggtgttgg gatgtttggg ttttgttttt ttgtttttt ttatttagtg tttttttttt    6240
taattggaat aatgttgttt tgtggttttt ttttatttgt tttagatatg attgggttgg    6300
atgggaaaag ggtttggatt gtgtatattt gttattagat tttggagttg gaaaaggagt    6360
tttattttaa ttgttatttg atttggtgat ggtgtattga gattgtttat gtattttgtt    6420
tgtttgagtg ttagattaag atttggtttt agaattggtg tatgaagtgg aagaaggata    6480
ataaattgaa aagtatgagt ttggttatag ttggtagtgt tttttagttt tgagtttgtt    6540
tagaggagtt tagtggttta agagtttgtg ttatttttag ttttggtttt tttaattttt    6600
```

tttgttttgt tgttgtttgt tggggattgg tttttataag tttgttttgt tttgtgttat 6660
gatattttgt ttggtt 6676

<210> 251
<211> 11656
<212> DNA
<213> Artificial Sequence

<220>
<223> chemically treated genomic DNA (Homo sapiens)

<400> 251

gttattgttt ggtaggggtt tgtttttttt tttggtgttg tttatttgtg aggtttgaga 60
tgtggattgt tatttttttt gggtttagtt tggttttaat gtaggtttta gagagaggga 120
taaaatttgg tttgtttttt tttttttttt aaggtgatta ttagtgtttt gggggtttat 180
agggtttttt tagttttgat ttgagttttt ggttgtagtt ggtttgggtt tgtgggggtt 240
ttgtgtaggg aagtggaggt ttttttttta ggtttttata tttttttggt ttgttttagt 300
agagttagtg gtttttttgag atagttgggt tttgtagttt ttagttgagg tttgttgttg 360
ggagaaagaa gaatgaatga agggtggaag gggtggggag gggtggggtg tgttgtgtta 420
gtttgtggtg tttttaggtt gggttgtagt ggggtgtggt ttttagaggt gtggttttgg 480
gtgtgggggt ggggtttttt ggtgggggtg gggttgtgta gggttgtttt tgttgttggt 540
tgtttgtgtt ttgtttttgt gttgtagttt ataggtttttg ttttggtttg ttttttttgtt 600
tgttttttaa gttgttgtgg tgttgagttg gtttttttgt tggtgtttga gaggtgtttt 660
tggtttggtt tggtttggtt tgtgtttttt gtttttgttt ttttgggttg gtggtggtgg 720
gtgagtttgt gggtttggtt gtttttagtt ttagttttgt tgggttttgt tttttgttga 780
gtgtatgagg ttgatgttat tttgttgtat ttggagggaa gaatgtatgg gagaggaagg 840
tgtgtgggtt gtggggtgtg gggtgagggt ttggaggggg tgtttggggt agtgtggggt 900
atgggagggg gttgggtttg ttaggaggtt gtgtttttgt tttttttggg atgagtttgt 960
ttttatgaag tttgtaggtt ttttttttgtt ttttttttgt ttgggatttt ttttttttggt 1020
ttttggtgag ttgttttttt gtgggtttgg gggtttttgg attttttttt ttttttttat 1080
gtttttttgt gaaggatttt tagatattgt ttattttgtg ttggttttta tttgtgtgtt 1140
ttgtttatta tttgggtttt gttttggggt tattttttat ttagttttgg aagaaagagt 1200
ggttggggaa gttgtagttt tgggttttag tggttgttgg gtgtttgttt ggttttgtga 1260
ttttgagtta ggtgttgatt ttttggtttt tagtttttttt ttttgtatat ttggaaattg 1320
ggtagttgtt tttttggtgt tggtgattgg gggttagatg ggtagagtgg agataggtgt 1380
ttaggaagtt ggaggttgtg tattgtggga gttttattta tttttttttgt ttgtgtttta 1440
aatttggtgt ttgtttttag ttttggttgg gagtatgatt tattttaatt tttttttttag 1500
tttttttttt tttattgggg tttaaggtgt agtattgtat tgtagttagg gtttaaggat 1560
ttttttagtt taggataggg tttgggggtt tttttttgga ttttttttgt tgatttgtta 1620

tttagattta tttggtttta aggtagggtt tgattttata ttttttttttg ttattaattt 1680
tttttaggtt tatgaaaatt tgattggggt aggggtttat ttttttgtag tttttgttta 1740
tttaaggtat ttgtgttttt atagagggtt aggttgttgt ggttttggga tttagttatg 1800
tgattgggta agttatgtta tttttggggt ttagtttttt tttttgtata gtggagaggg 1860
gtaggtttgg ggtatttttt agggtttatt agttttaagg gtgttaggtt ttaaggatga 1920
ttaagtatgt ttgtggttgg ttagaggagg tgttaggttt ttttgggata ggtgtttggg 1980
agtatttagg tttgtagttt ttttttttttg ttaagttagg gtatttattg ttaaggattt 2040
gttagggttg gtatttttag gtttttttgtt tttgattttt tagttggttt tagtttagga 2100
tgtattaatt tagttttgtt tagtttttgt ttttgaaggg ttttttttagt attttttttt 2160
gggtaggaga gggaagaaag gaggttgtga taggaatgtt atttattgtt ttatttttaa 2220
agttattgtt tttttttttt ttatttattt tgttagattt aatgttatag tgggaggatg 2280
gatttgattt ttttttttaag ttgatatata gggaaatagg gttagagaag tttggtaatt 2340
tagttagtat tttagtaaga tttaggttag tgtttttttt tttttttattt ggtatagtga 2400
ttgttttgtt tgggtgttgt atatgtgtag tgtgtgagga ttggtgtagg tgtaggtata 2460
tgtggggtgg gtagggtaag ttgggtttgt attagattat atttttttgag aatgtttttt 2520
aatttttttt ttattttgta ggaagtgagt tgtttgtgtg tgtaagttgt ggttagagga 2580
tttatgatgg ttagtatttt taggttttga atgtggattg gtatgtagat tgttttaggt 2640
agggtggggt gtttagggtt tgtgttgttt taaataaggt ttgttagaga ggataggttg 2700
gttaaggaat gggggaggtt gggatatgtt ttttggtgtt gttttttatt gtgattttgt 2760
ggttttaatt tattatttat gatatgaggt gttttgatta gaaaattttt ataggttttt 2820

```
ttgttgttat tttatttatt tattttttttt tttttttgag atggagtttt gttttgttat    2880
ttaggttgga gtatagtggt gtgattttgg tttattgtaa ttttttttttt ttgggtttat    2940
gtagtttttt tgtgttagtt tttggagtag ttgggattat aggtgtgtat tattatgttt    3000
agttaatttt tgtattttta gtagagatgg gttttgttat gttagttagg ttggtttgaa    3060
atttttgatt ttaagtgatt tttttatttt agtttttttaa agtgttggga tgatagatat    3120
aaattattgt ttttggtttt attttatttt tttttatgta ttttttttttt tttgaaatgg    3180
ttttgttttg ttgtttaggt tggagtgtag tggtgttatt ttggtttatt gtaattttta    3240
tttttttgggt taaagtgatt tttttatttt agtttttttga gtagttggga ttataggtat    3300
atattataat gtttagttaa tttttttaaat tttgtgtaaa gatagggttt tattattgag    3360
atttaggttg gttttgaatt tgtgatttta agtaattttt ttgttttggt ttttgaaagt    3420
gttaggttta taggtgtgag ttaatgtttt ggtttttgtt gttattttag atttttgaga    3480
tttaaatttt agagaggatg ggagagattt ttaattgagt tagtgtttgt aatttagttt    3540
tttgttggta tttagatagg gggaagagtt ggaaggaatg tttttttttgt tttttgggtg    3600
tttatgtttt tgtagggagg gaagataaat taggttttaa gggaaattag gttattttta    3660
gtgtttttta ggttgtttgt gaatatgtat aatttagtta tattagtttt agtgtttaga    3720
tatatattta taggtaggaa gaaagtaggg ttagggttgt ggtggaggat aaagagtata    3780
tgaggatttg aaggttattt agtaggatta ttttgagaag ttaggagtag ggtttattt    3840
gttttgagtt agagtagggt tagagtaggg gttttaaagg atgtgagatt ttttgggtag    3900
aaaagtagag tggaggtggg gtgtggtggt ttatatttat aattttatta ttttttgggg    3960
ttgaggtggg tagattattt gagtttagga gtttgagata agtttgggta atatggtaat    4020
attttgtttt tattgaaaat ataaaaaatt agttgggtgt ggtggtgtat gtttgtagtt    4080
ttagttattt aagaggttga ggtggtaggg ttatttgagt ttgggaggtg gaggttgtag    4140
tgagttatga ttgtattatt gtattttagt ttgggtaata gagtgagatt tagttttaat    4200
ttttaaaaaa gaaagaaaga aaaataaatg gtgtgggaga gaattatagg tatagttatt    4260
aaatagtaag gtttagggga gaaaatttta taaaagggta gaaggtgaag tttttgggat    4320
gtttagtagg ggttaagata tttattatta ggattttatt ttaggttttt gttttggttt    4380
atttttttggt ttttggtttt tttgagatag ttttgttgtg ttgtttaggt tggggagtag    4440
tggtgtgatt tttttttatt gtaatttttg ttttttaggt ttaagtgatt tttttgtttt    4500
agttttttaa gtagttggga ttataggtgt gtattattat gtttggttaa gttttgtatt    4560
tttagtagag atagggtttt gttatgttgg ttaggttggt tttgaatttt tgattttaag    4620
tgatttgttt gttttagttt tttaaaatgt tggaattata ggtatgagtt attgtatttg    4680
gttttggttt gtttttttgt ttttttttttt ttttttttttt atataggggtt ttgttgtgtt    4740
atttaggttg gtgtgtagtg gtgagattat agtttattgt agtttttagt tttaattggt    4800
ttaagtgatt tttttgattt agtttttttaa agtgttggga ttataagtat aagttattat    4860
gtttagtttg ttttttttttt tttaggaata atgtttaatg ttttttaata tttagtaagg    4920
gataattttt gttttaagta ttttgtatag ttagatatta gtgttgtttt tgttttgtta    4980
gagagaaaat tgggatatag agaggttaat tttttttgatg aaagttatat agttagtgag    5040
tgaaatgaat atatttagtg tggttgaaag gagatagata gtatgttttg ggattttgta    5100
ttaggtgttt agaaagaggt ttttgggggg taagagggtt tttaataggt agaggaaatt    5160
atttgtatag tggtgggatg gtgtggattg ttgagggaat aggaatagtt tttttggaag    5220
gaatagaata agttgaggga tttaataaga aataaagttg agattgattt gtgaagggtt    5280
ttgaatgtta agataaggag ttttagaagt taggatgggg gtggtggttt atttttgtaa    5340
ttttagtatt ttgggaggtt gaggtaggta gattatttga ttttaggagt ttgagattag    5400
tttggttaat gtggtgaaat ttttgttttt attaaatatt taaaaattag ttaggtatgg    5460
tggtatatga ttgtaattta agttatttgg gaggttaagg aaggagaatt atttgaattt    5520
gggaggtgga ggttgtagtg agttgagatt atgttattgt attttagttt gggagataga    5580
gtgagatttt attttaaaaa aaaaaaaaaa aaaaaaataga agggagttgg tagaaagtta    5640
gggagggggtt ggggtgatat gttgttgaag aatgttattt tagtgggttg gtggtggtat    5700
ttaggtaggg aagggattgt tttagtaatt taagggtttg agtttatagg atttgatttg    5760
ggatagtgat tgaggatgga gagaatttta ggtagaaggg atagtttttg gtgagtattt    5820
gttatattgg ttattatgta ttgagtattt tttatgttaa tttgttaaat ttttattata    5880
attttatgag ggattgtatg tgtttagttt gtatgggaga aatagagatt ttatgtaatt    5940
aagtgttttg ttgaaggttg taatatttag agttgggatt aaaatttttt tattttatat    6000
tgttatagag taggaaaggt aggggttggt ggtggtatat gtttataatt ttagtttttt    6060
gggaggtgga ggtaggtgga ttttttgagt ttaggagttt gagattagtg tgggtaatat    6120
agtgaaattt tgtttttata aaaaaattag ttgagtatgg tggtggtgtt tgtagtttta    6180
gttatttaag ggtgttgata tgggagggtt gtttgagttt gggaggtgga ggttgtagtg    6240
agttatgatt atattattgt aagttagttt gggtgataga gtgaaatttt attttaaaaa    6300
aaagaaagaa aggaagaaag aaaaagatag gggttttggg gagggtatgg gtattggtga    6360
atggtagggt ggaatttgaa gttatttggt tttttaattt gggtattggg gagttggtgg    6420
tttgttgatt ttgatggaat tgggggttat gttggggagg agatatgttt atttgtgttg    6480
```

```
agttggaggg gatatgggtt atttatggtg gttgtgtttt gtttagagtt agttatggga    6540
gtttgagttt agttggaggt aggaaagtta gaaaaaatgg ttgttttgga gttggttttg    6600
agatggtgag tgggatttgt gatagggtta agatgaatga agggaagaat tttggggatt    6660
tttgtgtttg tggtgagggg ggagatggag ttttgggtga tggagagagg gttaggagta    6720
gagttataga agttatagga gggaagttgt gttataggat gggtgtattt ggttttggag    6780
tggtttgttt taaattattt attaggagag gggtgagttt ttaggttagg gtagtaaaga    6840
ggaggtatgt ttgtgttgtt tttggtgtag tgaaatttaa gaaggaagtt aggtgtagtg    6900
gtttatgttt gtaattttag tattttggga ggttaaggta ggtagattat ttgaggttgg    6960
gagtttgaga ttagtttggt taatatggtg aaattttatt tttattaaaa atataaaaat    7020
tagttgggtt tgttggtggg tgtttgtaat tttagttatt taggaggttg aggtagaaga    7080
attgtttgaa tttgagaggt agtgattgta gtgagttaag attgtgttat tgtattttag    7140
tttgggtgat agagtaagat tttattttga aaaaaaaaag ttttaatatg gggaaagatt    7200
tattagaagt aagagttatg gtttttattt tgtggtttgt gggtgtgata tttttaatag    7260
tttttaaagt tggtggtttt tattgtgtga tagtgagtag agtagtttag agggggttat    7320
tgtttatttg ggtgtatggt tgattatagt taggttggtt tttagtggga tgtttaaggt    7380
gttagatttt gtttagtttt ttttgggttt tgggtttgag gtattgggtt tggtttagat    7440
tttattttat gtattgagat ttttgtttta gggtttttta tttttttgaa ggtgtttggg    7500
taggggtaat gtgataaggt tatgaggggt ttgtagtttt tagttttatt ggggaggtgg    7560
ttagtgattt ttattttttt ggtttttttg tatgtttttt ttagtggaat tttttagggt    7620
ttttgagtta gttatttgta aataattatg gtgtgtttat tttgtattag gttttttttta    7680
taataattta gtaagggggt gttatttatt tattaaagtg attttttttt tgagttttgt    7740
tttgttgttt aggttggagt gtggtggtgt aattttggtt tattgtaagt tttgtttttt    7800
gggtttatat tattttttttg ttttagtttt ttaagtagtt gggattatag gtgtttatta    7860
ttatatttgg ttaatttttt tttgtttgtt tgtattttta gtatagatga ggttttgttg    7920
tgtgagttag gatggttttg atttttttgat tttgtgattt gtttatttta gtttttttaaa    7980
gtgttgggat tataggtgtg agttattgtg tttggtaata ttaaagtgat tttaaggtta    8040
aggttggtaa tttatgtttg taattttagt attttgggag gttgaggtag gaggattgtt    8100
tgaggttagg agtttgagat taatttaggt aatatagtga gattttattt ttataaaaaa    8160
attagttagg tgtggtggtg tgtatttgta gttttagtta tttaggaggt tgagatggga    8220
ggattatttg aatttaggat gttgaagttg tagtgagttg tgattatgtt attgtatttt    8280
ggtttgggta atagagtgag atattgtttt aaatttttaa aaagtgattt tataaatgag    8340
gtgtagagtt tagttatttg ttaaaagttt tatagtgtgt gaggagtaga attaggattt    8400
gaattgaggt agtttggttt tagagtttat agtgtaatta tagtttagtt ttatattttt    8460
tagattaata gggtttttgt tttttagtgg gtaagatatt tagtgaataa atgtagtgtt    8520
aggtattggg ggatagtatt tttaggaagt gatgtttaag ggatagaatt gaagggagta    8580
gtgtttagag gatgttgggg gtagggttgg tgtatgtgta aaggttttgg ggtgggaatg    8640
tgtttggtat aattgaggat tataaagtta gtgtgtggga gtgtagttag tggttagggg    8700
tgtatagagt tttgtgggtt ttgtggaagg tgttgttggt tgtatagttt tttttttttt    8760
tttttttttt ttttttttgag atagagtttt gtttttgttg tttaggttgg agtgtagtgg    8820
tgtgatttta gtttattgta atttttgttt tttgggttta agtgattttt ttgttttagt    8880
tttttggtag ttgggattat aggtgtttat tattatattt ggttaatttt tgtgttttta    8940
atagagatgg ggttttatta tgttagttag gttggtttta aatttttgat tttaagtgat    9000
ttgtttattt tagtttttta aagtgttggg gttataggta tgagttattg tgtataggta    9060
gttgtgtatt tttgaatgtt ataatttgag tatttgagag ttgtttttgt tttttggttt    9120
ttgtttttga ggaagtttta tgttgatagg atagataggg ttataagtgt tgtgatgggg    9180
gtttgtaggt tgttggaggg tttagttggg attagatgtt gttttttttt gtagagtggg    9240
ataattgttg taggtttatg ggattttttgg tattagtttt gagggttgtt attttggggt    9300
ttgttttttt ttgtgttttg atttttagt tttttgtagg ttttgttttt tggaaggtta    9360
tgattaggtt tggattggtt taggtttttt tttggtttat atattgtttt tgtgaggttt    9420
tttttaggaa gttttttgtg tataattttt agggttgttg tatttttggt agtatttttt    9480
tggtagttgg gtgggttggt tttgggtaag gagggttgag tatgttgttg gtttgtgggg    9540
ttggagtagt ggtgggatgt aattttttttt tttttagggg atttttttgg tgaagataaa    9600
ttgtttatag gaagttgatt tttgtttttt tgggggtagt agtggaagag gtagttgttt    9660
ttgagtttgt ttttgttttt agagaagttt gaggttttta gtgttgttgt tagggttgag    9720
gttgaggagt ttatagtgtg tgtttaggat tgagggttag ggatgggtta taggtttttt    9780
gtttggggtt taagtttaga ttgtttgttt tttatttgta ttaaagttta ggtaaagggt    9840
gttttagtta ttttttgttg taggtttaga ttaagttttt tggtatttat gtggttgtag    9900
tttttttttg tgtgttgtag ttatgttggt tttatttttt gtagttttta attttgagtt    9960
tttgagggag gatggggaag tagttggttt ggttattttt gttttttttt agatttttag   10020
agtttttagt gtagttatag aggatgttgt tggttttagt tttaagaaga tgttgttttt   10080
tttagagggt taagtaagtg ggaatttttt tttttatttg ttttgggttt taggtagggt   10140
```

```
ttttggtgta aggtttgggg ttggaagttg atttatttag gtttaggttt tggggtagaa   10200
ttgaaatttt ttggttattg ttggttgtag tttgggagta ggttattgtt aaagttgtgg   10260
gttttttag gatagttttt ttatgaggtt ggtttttat ttgttgtttt tttatatttg   10320
gtggttaggg atgtggtttt gggtagaatg atgatttttt atttttgtta ttatggaagt   10380
tattgttgtt ttttagttta gttagttatt tgggttgtag agtatttttt ttatgttttt   10440
tgggtgtttt tttttttttt tgttttagtt tggttttgtt ttattttgtt tttagggagg   10500
ggtattttgg agtggggtta gggtatggtt ttttttgag ggagtttttt tttggttgtt   10560
tttagggtag ttttgtatag ttttagtatt tggtgtattt tttttgatat ttttttagg   10620
gatagttagg tattttgtgt ggggtattta agagagttag gtttgttagt ttttagtttt   10680
tgttagaatg taggtttgag gggtgagggg tggggtaggg gtagggatag gaattttggt   10740
gtgtttttta tttgtaaagg tttattgagg ttttgagttt tagttattga gttattaagt   10800
tagtttgggt taggtttggg tgttttgttt gtaatggagg tagagatggg gttttggggt   10860
agttttgagg atgttgggtg tatagtgggg gttttgttgg taggaattat ttatgttttt   10920
ttttgggtta agttttgtgg atgtttagtt tggggttgtg gggagttggt aggttagtgg   10980
tagatattgg tgggtagatt tagtgtttgg tagaataggt attaaggaag tggtgattgg   11040
agggaagtta agtgtattta aatttttggg tgagttatta ttgttgggtt ttttatagtt   11100
gttgaaagtg agtaatagtg atgaaggttt gtgagttttt gtgtgagtga gtgaatggat   11160
tagtagtagt ttttaggttg tggaagagtg tttttttttt gggatgggga tatttggtta   11220
tagtaatttt taattttta tttatttatt gtttattgta gaggtatgtg ggggttttgt   11280
tttttgtagg taggagtgag ggtatttttt gtgatgtggt atttttgtga ttgaggttat   11340
gtgtgattgg tgtaagggta ggaagtgagt tattggtttg tattaggtgt gggggttttt   11400
gtgagggtag gatttaaagt tggtttggtt ttttggttgt agtatttttt tttttttga   11460
attaggttag agttttggga tgggaggtgt tttgtagatt atttttttta ttaattttg   11520
tttttgttt tatttttgtg gtgatttggt gaattgttgg tttttgttg tgtattgagt   11580
ggggtagtga ttttgatgtg gtgtttttg ttgtttttgt tattgttatt atttttggtg   11640
gtttagtttt tgtatt                                                  11656
```

<210> 252
<211> 11656
<212> DNA
<213> Artificial Sequence

<220>
<223> chemically treated genomic DNA (Homo sapiens)

<400> 252

```
aatgtggagg ttgggttatt ggaggtggtg gtggtggtag gggtggtagg agatgttatg      60
ttagggttat tgttttattt ggtgtatagt agggggttgg tagtttattg gattattgtg     120
ggggtggggt ggaggatgga agttggtgag gggggtggtt tatagggtat tttttgtttt     180
agggttttaa tttaatttga aagggaaagg agtgttgtag ttgggaggtt aggttgattt     240
tgggttttgt ttttgtagaa gtttttatgt ttggtgtaga ttaatgattt gttttttgtt     300
tttatattga ttatatatga ttttggttat aggggtgtta tattatagga gtgtttttta     360
tttttgtttg taggaagtag ggtttttgta tatttttgta gtgggtggtg ggtgggtggg     420
ggattaggaa ttgttgtaat taagtgtttt tattttgggg agggaatgtt tttttataat     480
ttggaaattg ttattggttt atttatttgt ttatgtagaa atttataaat ttttattatt     540
gttgtttatt tttagtagtt gtgaaagatt tggtggtgat gatttatttg agggtttgag     600
tgtatttggt ttttttttgg ttattatttt tttgatgttt gttttattag atattaggtt     660
tgtttattag tgtttgttat tgatttgtta gtttttttgtg gttttaggtt gggtatttat     720
aaagtttggt ttaggagaga gtataagtga tttttgttgg tgaggttttt gttgtgtatt     780
tagtattttt agaattgttt tgagattttg tttttgtttt tattgtagat agggtattta     840
ggtttggttt aggttgattt ggtggtttag tggttggggt ttggggtttt agtgaatttt     900
tgtggatgga gagtatgttg gagtttttgt ttttgttttt gttttgtttt ttattttttta     960
ggtttgtatt ttggtaggag ttagaggttg atgggtttgg tttttttgag tgttttatat    1020
agagtgtttg attgttttta agaaggatgt taagggaggt gtgttaggta ttgaggttgt    1080
gtagagttgt tttggggata gttagagagg aattttttttg ggggagagtt atgttttggt    1140
tttattttag ggtatttttt tttgagagta gggtaggata aagttaggtt ggggtaggag    1200
aagggggagg tatttggagg gtatgaaagg ggtgttttgt agtttaggtg gttggttggg    1260
ttgggagata gtggtggttt ttataatgat aggagtggag aattattgtt ttatttaggg    1320
ttatattttt ggttattagg tgtgaagaaa tagtaggtgg aggattggtt ttatggggag    1380
attgttttgg aaggatttat agttttggta gtggtttgtt tttaggttgt agttgatagt    1440
```

```
aattaaggag ttttagtttt gttttagagt ttggatttag gtgggttggt ttttagtttt     1500
aggttttata ttaggggttt tgtttggagt ttaggataag tagggagggg gatttttatt     1560
tatttagttt tttggaggaa gtggtgtttt tttggggttg aagttaatag tattttttgt     1620
ggttatattg gggttttgg aggtttaagg gagggtaggg gtggttagat tagttgtttt      1680
tttattttt tttaggggtt taggattgga ggttgtagag ggtggggtta gtgtggttgt      1740
agtgtatagg aggaggttgt agttgtgtgg gtgttagggg atttggtttg agtttgtaat     1800
aggaagtggt tgaagtattt tttgtttggg ttttggtgtg ggtggggagt gagtgattta     1860
ggtttggatt ttaggtaggg agtttgtggt ttgtttttgg tttttagttt tgaatatatg     1920
ttgtaggttt tttagttttg gttttggtaa tggtattgaa ggttttaggt ttttttgggg     1980
atagggataa gtttaaaagt agttgttttt tttattgttg tttttaaggg aatagaggtt     2040
gatttttat ggatagtttg tttttgttaa aaaggttttt tgaagaaagg gaggttgtat      2100
tttgttgttg ttttaatttt ataggttagt agtatgttta gtttttttttg tttagggtta    2160
gtttatttag ttgttaagga gatgttatta gggatgtggt agttttgggg gttgtgtata     2220
ggaggttttt tggaggggat tttgtagagg tagtatgtga gttaaaggga agtttggatt     2280
agtttaagtt tggttataat tttttgggag gtagggtttg taaggggttg ggaggttaga     2340
atatagaaag gggtaggttt tggagtgata atttttaggg ttaatattag aggttttatg     2400
ggtttgtagt aattgtttta ttttataaag aggggtagta tttggttttg gttgagtttt     2460
ttagtagttt gtaggttttt attatagtat ttatgatttt gtttgtttta ttagtgtggg     2520
attttttaa gagtaggggt taggggatag gagtagtttt tagatgttta ggttatgata      2580
tttaaagatg tatagttgtt tgtgtgtagt ggtttatgtt tgtaatttta gtattttggg     2640
aggttgaggt agatagattg tttgaggtta ggagtttgag gttagtttgg ttaatatggt     2700
gaaattttgt ttttattaaa aatataaaaa ttagttaggt gtggtggtgg gtgtttgtag     2760
ttttagttat taggaagttg aggtaggaga attgtttgaa tttgggaggt ggaggttgta     2820
gtgagttgag attatgttat tgtattttag tttgggtaat aaaagtgaga ttttgtttta     2880
aaaaaaaaaa aaaaaaaaaa aaaaaaaagt tgtatagtta atggtatttt ttatggggtt     2940
tataaggttt tatgtatttt tggttattga ttgtattttt gtatgttggt tttgtggttt     3000
ttagttgtgt taagtatatt tttattttaa ggtttttgta tatgtattgg ttttattttt     3060
gatatttttt aaatattgtt ttttttaatt ttgtttttta aatattattt tttgagagtg     3120
ttgtttttta atatttgata ttatatttgt ttattgagtg ttttgtttat tagaaggtag     3180
ggattttgtt ggtttgggag gtgtgggatt aagttgtggt tatattgtag gttttgaagt     3240
taggttgttt tggtttgagt tttgatttta ttttttatgt gttgtgagat ttttggtaag     3300
tgattgaatt ttgtatttta tttgtaaaat tgttttttaa aaatttgaga tagtgttttg     3360
ttttgttgtt taggttagag tgtagtggtg tgattatagt ttattgtagt tttgatattt     3420
tgggtttaaa tgattttttt attttagttt tttgagtagt tgggattata ggtatgtatt     3480
attatgtttg gttaattttt ttgtagagat ggagttttat tatgttgttt aggttgattt     3540
taaatttttg gttttaagta gtttttttgt tttagttttt taaagtgttg ggattatagg     3600
tgtgagttat tagttttggt ttttaaaattg ttttaatatt gttgggtatg gtggtttatg    3660
tttgtaattt tagtattttg ggaggttgag gtgggtggat tatgaggtta ggagattgag     3720
attattttgg tttatatagt gaaattttgt ttgtattaaa aatataaata aataaaaaaa     3780
aattagttgg gtgtggtggt gggtgtttgt agttttagtt atttgggagg ttgaggtagg     3840
agaatggtgt gaatttggga ggtagagttt gtagtaagtt gagattgtgt tattgtattt     3900
tagtttgggt gatagagtga gatttaaaaa aaaaattgtt ttaataaata aatagtattt     3960
ttttattggg ttgttgtgag aggggtttaa tgtagagtgg gtatgttata attatttgta     4020
agtgattgat ttagggattt taggaagttt tattgggagg ggtatgtaga ggggttagga     4080
aagtggaaat tattggttat ttttttagtg gggttggagg ttgtagattt tttatggttt     4140
tattatattg tttttgtttg aatattttta gaggggtgag gggttttgga ataaaggttt     4200
tagtgtatga aatgaggttt gggttgggtt taatgtttta agtttagggt ttgagggaga     4260
ttaagtagag tttagtattt tgggtatttt attgagagtt agtttggttg tggttagtta     4320
tgtatttagg tgagtagtga tttttttttga gttgttttgt ttattgttat gtggtgagga    4380
ttattagttt tggggattgt tgggagtatt atatttataa gttatgaggt agaagttatg     4440
gtttttattt ttagtggatt tttttttata ttgagatttt ttttttttga gatggagttt     4500
tgttttgtta tttaggttag agtgtagtgg tgtgattttg atttattgta attattgttt     4560
tttgggttta agtgattttt ttgttttagt tttttgagta gttgggatta taggtgttta     4620
ttaataggtt tggttaattt ttgtattttt agtagagatg gggttttatt atgttggtta     4680
gattggtttt aaatttttga ttttaggtga tttgtttgtt ttggtttttt aaagtgttgg     4740
gattataggt gtgagttatt gtatttggtt ttttttttga gttttattat attagggata     4800
gtataaatat gtttttttttt tattgttttg atttggggat ttattttttt tttggtgagt    4860
gatttgggat aggttatttt aaagttaggt atatttattt tgtaatatgg tttttttttt     4920
gtggttttttg tggttttatt tttgattttt tttttattat ttaaggtttt attttttttt    4980
ttattgtaga tataggggtt tttaaagttt tttttttat ttgttttggt tttattataa      5040
atttattta ttattttagg gttagttttg aggtggttgt tttttttgat tttttttattt     5100
```

```
ttaattggat ttaggttttt atggttagtt ttgggtagga tatagttatt atggatagtt     5160
tatgtttttt ttagtttaat atagatgagt atgttttttt tttaatatga tttttaattt     5220
tattagagtt aataaattat taattttttta gtgtttaggt tagaaaatta gatggtttta     5280
ggttttattt tgttatttgt tagtgtttat gttttttttg aagtttttgt tttttttttt     5340
tttttttttt tttttttttt tgagatggga ttttattttg ttatttaggt tggtttgtag     5400
tggtgtgatt atagtttatt gtaattttta tttttaggt ttaagtaatt tttttatgtt     5460
agtgttttg agtagttggg attataggta ttattattat gtttagttaa ttttttgta     5520
gagataaggt tttattatgt tgtttatatt ggttttaaat ttttgggttt aagagattta     5580
tttgttttg ttttttaaag ggttgggatt ataggtatgt gttattgtta gtttttgttt     5640
tttttatttt gtggtgatgt ggagtgagaa ggttttagtt ttagttttag atgttataat     5700
ttttagtgag gtatttgatt gtatggaatt tttgtttttt ttatgtaaat tgggtatata     5760
tagtttttta tagagttatg atgaagattt aataaattag tatgaagagt gtttaatgta     5820
tggtagttaa tatgataaat atttattaaa aattgttttt tttgtttgaa atttttttta     5880
tttttagtta ttgttttagg ttaggtttta tgagtttaga tttttgagtt attgggatag     5940
tttttttttt gtttagatat tattattggt ttattgggat ggtatttttt aatagtatgt     6000
tattttagtt ttttttggt tttttgttga ttttttttta tttttttttt tttttttttt     6060
tgagatggag ttttgttttg ttttttaggt tggagtgtag tgatgtgatt ttagtttatt     6120
gtagttttg ttttttaggt ttaagtgatt tttttttttt agttttttga gtagtttgga     6180
ttatagttat gtgttattat gtttggttaa tttttgaata tttagtagag atgggggttt     6240
tattatgttg gttaggttgg ttttaagttt ttggggttaa gtgatttgtt tgttttggtt     6300
ttttaaagtg ttgggattat ggggatgagt tattattttt attttgattt ttgaaatttt     6360
ttattttggt atttaaggtt ttttatgaat tggttttaat tttgtttttt gttggatttt     6420
ttagtttatt ttgtttttttt taagggaatt gtttttgttt ttttagtagt ttgtattatt     6480
ttattgttgt gtagatggtt tttttttgttt gttgagagtt tttttgtttt ttgaaggttt     6540
tttttttgagt atttgatgta gaattttagg gtatgttgtt tgtttttttt tagttatatt     6600

gagtgtgttt attttattta ttggttgtgt gatttttatt aagagaatta atttttttgt     6660
gttttaattt tttttttggt aaaataaaga tagtattaat atttaattat gtaaagtatt     6720
tagaataggg gttgtttttt attgaatgtt agaaaatgtt agatgttatt tttaaaaaag     6780
aaaaaatagg ttgggtatgg tggtttatgt ttgtaatttt agtattttgg gaggttgagt     6840
tagaaggatt gtttgaatta gttggagttg gaggttatag tgggttatga ttttattatt     6900
gtatgttagt ttgggtgata tagtaagatt ttgtgtaaaa aaaaagaaaa gaagaaataa     6960
aaaaataaat tgaggttagg tgtagtggtt tatgtttgta attttagtat tttgggaggt     7020
tgaggtgggt agattatttg aggttaggag tttgagatta gtttggttaa tatggtaaaa     7080
ttttattttt attgaaaata taaaatttag ttgggtgtgg tggtgtatat ttgtaatttt     7140
agttatttgg gaggttgaag taggagaatt gtttgaattt gggaggtgga ggttgtagtg     7200
aggagggatt gtgttattgt tttttagttt gggtgatata ataagattgt tttaaaaaaa     7260
ttaaaaatta aaaaataaat taaggtagaa gtttaaaata aagttttagt ggtggatgtt     7320
ttgattttttg ttgggtattt tagaagtttt atttttttatt tttttatgga gttttttttt     7380
ttgaattttg ttgtttggtg attatgtttg taatttttttt ttatattatt tgtttttttt     7440
tttttttttt ttaaaaattg agattgggtt ttgttttatt gtttaggtta gagtgtagtg     7500
gtgtgattat agtttattgt agtttttatt ttttaggttt aagtaatttt gttattttag     7560
tttttttgagt agttgggatt ataggtatgt gttattatgt ttggttaatt ttttgtattt     7620
ttagtggaga tagggtgttg ttatattgtt taggtttgtt ttgaattttt gaatttaagt     7680
gatttgttta ttttagtttt aaaaagtgat gggattatag gtgtgagtta ttatgtttta     7740
tttttatttt atttttttat ttaggaaatt ttatattttt tgagattttt gttttggttt     7800
tgttttggtt taaggtaggt agatttttgt ttttggtttt ttaggatggt tttattgggt     7860
gatttttagg tttttatgta ttttttattt tttgttataa ttttgatttt atttttttttt     7920
tatttgtggg tgtgtgtttg gatattgggg ttggtgtgat tgggttatgt atgtttgtaa     7980
gtagtttgga agatattgag ggtggtttgg tttttttttaa ggtttggttt gttttttttt     8040
tttgtaagag tatgaatatt tagaaggtag gagggatatt ttttttaatt ttttttttttg     8100
tttgggtgtt agtagggggt tgaattgtag gtattggttt aattggaggt tttttttatt     8160
tttttttaaga tttagatttt agagatttag gatgataata gaggttaagg tgttagttta     8220
tgtttgtaag tttagtattt ttggaggtta aggtaggagg attgtttgag gttataagtt     8280
taagattagt ttgggtttta atagtgagat tttgttttta tataaaattt aaaaaattag     8340
ttgagtattg tggtgtatat ttataatttt agttatttgg gaggttgaag taggaggatt     8400
attttagttt gggagatgga ggttgtaatg agttgagatg gtattattgt attttagttt     8460
gggtaataga gtaagattat tttgaaaaaa gaaaaatata taaaagaaaa taaaatgagg     8520
ttaggagtgg tggtttatgt ttgttatttt agtattttgg gaggttgagg tgggaagatt     8580
atttgaggtt agaagtttta gattagtttg gttaatatgg taaaatttgt ttttattaaa     8640
aatataaaaa ttagttgggt gtggtggtgt atgtttgtaa ttttagttat tttagaggtt     8700
```

```
gatataggag aattgtgtga gtttaggagg aagaggttgt aatgagttaa gattgtatta    8760
ttgtatttta gtttgggtga tagagtgaga ttttgtttta aaaagaaaaa aaaatagata    8820
aataaaatga taataggaag gtttgtaggg attttttagt taaaatattt tatgttataa    8880
atggtaaatt aaggttatga agttataata ggggatggta ttaggaggta tattttggtt    8940
tttttattt tttgattagt ttgtttttt tggtaggttt tgtttagggt aatataggtt    9000
ttgggtattt tattttattt gaagtagttt gtgtgttagt ttgtgtttag ggtttggagg    9060
tattggttat tatagatttt ttggttgtag tttgtatata tgggtaattt gtttttttgta    9120
gggtgggaag ggagttggga agtattttta ggaagtgtga tttggtgtag gtttagtttg    9180
ttttgtttat tttatatata tttatatttg tattaatttt tgtatattgt atatgtgtag    9240
tgtttaggta gggtagttgt tgtgttaaat aggagaagaa agggtgttgg tttgagtttt    9300
gttgagatat tgattaggtt gttaagtttt tttggttttg ttttttatg tattagttta    9360
ggaggaggat taggtttatt tttttgttat agtattggat ttggtaaggt aaatgaggag    9420
aaaggaagta gtggttttag ggataaggta gtgggtgata tttttattat agttttttt    9480
tttttttttt ttgtttagga agaagtatta agagggtttt ttaaaggtag ggattggata    9540
gggttggatt agtgtatttt gggttgaagt tagttgggag gttaagggta ggaagtttgg    9600
aggtgttggt tttaatagat ttttggtaat gaatgttttg gtttggtaag gggagggggt    9660
tgtagatttg ggtattttta gatatttgtt ttagggaggt ttggtatttt ttttagttag    9720
ttataggtat gtttagttat ttttggggtt tggtattttt ggagttggtg ggttttggaa    9780
aatgttttag atttgttttt ttttattgta tagaagggga gattgagttt tagagatggt    9840
atggtttgtt tagttatata gttaggtttt aaggttataa tagtttgatt ttttgtgaag    9900
atgtaggtat tttaggtagg taggggttat aggaaggtgg gtttttattt taattaggtt    9960
tttatgggtt tgggaagagt tggtggtagg gggaggtgtg gagttaggtt ttgttttggg   10020
gttaggtgga tttaagtgat aggttagtga aggagattta aggaggggtt tttagatttt   10080
gttttaggtt gggggagttt ttgaatttta attgtagtgt agtattgtgt tttgggtttt   10140
agtgagggag aaggggttaa agaggaggtt aggatgagtt atgtttttag ttaagattga   10200
gggtaggtat tgggtttggg gtatggatag ggagagtgga tgaggttttt gtagtatatg   10260
gtttttggtt ttttggatgt ttatttttgt tttatttatt tggtttttaa ttattgatat   10320
tggggggggta attatttagt ttttaaatgt atagaagggg aaattgagga ttaggaagtt   10380
agtgtttggt ttaaggttat agagttgggt aggtgtttgg tggttattgg gatttggggt   10440
tgtggttttt ttagttgttt tttttttttga gattggataa agggtggttt tagggtgagg   10500
tttgggtggt ggatagagta tgtggatgga ggttgatgtg gggtgggtag tgtttgggag   10560
ttttttgtgg ggggatgtgg gaggaggaaa aagggtttag gggtttttag atttgtagga   10620
gggtagtttg ttgggggttg gggaggggga ttttgggtgg gagggggata gggaggggtt   10680
tgtgggtttt gtaaggatga gtttattttg gaggaggtag gggtgtggtt ttttggtaga   10740
tttggttttt ttttgtgttt tatgttgttt tgggtatttt ttttaggttt ttgttttata   10800
ttttgtggtt tgtgtatttt ttttttttat atgtttttttt ttttaggtgt aataaagtag   10860
tgttaatttt atgtatttga tggggggtgg ggtttggtgg ggttggggtt gggggtggtt   10920
gggtttgtga gtttgtttat tgttgttggt ttggggaggt gggggtggag ggtgtgggtt   10980
gggttgggtt gggttggggg tgttttttgg atattggtgg ggaaattggt ttggtgttgt   11040
ggtggtttgg ggagtgagtg gaggggtgga ttgaggtggg gtttgtgagt tgtggtatgg   11100
gggtggggtg tgggtggttg gtggtgggaa tggttttgtg tggttttgtt tttattagaa   11160
aattttgttt ttatgtttaa ggttatgttt ttagaggtta tgttttgttg tggtttggtt   11220
tgggggtgtt gtgagttggt gtggtgtgtt ttgttttttt ttgttttttt tgtttttttat   11280
ttattttttt tttttttagt gataggtttt ggttgggagt tgtgaggttt agttgttttg   11340
gagggttgtt agttttgttg gggtaggtta ggaggatgta ggagtttaga gaaggggttt   11400
ttgtttttttt gtgtgaggtt tttatagatt taaattagtt gtagttggga atttggatta   11460
gagttggaga ggttttgtgg atttttagga tattggtggt tgttttgggg aggggaggga   11520
ggtgggttgg gttttgtttt tttttttgag gtttgtattg aggttaggtt gggtttgggg   11580
aaggtgatgg tttgtgtttt aggttttgtg aatgagtagt attagagaaa ggagtgagtt   11640
tttgttaggt ggtgat                                                   11656
```

<210> 253
<211> 13705
<212> DNA
<213> Artificial Sequence

<220>
<223> chemically treated genomic DNA (Homo sapiens)

<400> 253

```
gatgggaaag ggagggtgag ggttgggggt ggggattgta tgaaatggag gtgtttgatt      60
gtgaaggggg tttttttttgt gtggttatgg ggaggagttt ggggaggtta gtgtgggtgg     120
tagttttagg gaggttgaga gtattgttag gtttggggtt gtgatgttgg gtggatttgg     180
tagggtttgg aattttttttt tggttggttt gggttggggt ggtgggaggg gagaggttgg     240
attgtttttt tgtagttttt ttttattgag ttaagagggg ttttgaggga ggatggaggt     300
agggaaggaa tagttttggt tttatttagt ttatttgtag atattttatt tatatataga     360
tttgaagtta tgtaatatat tttgattttg atatatattt taataaatta gatgtattag     420
tttatggtta ttttaatttg tagatattat ttatttagat ggatatttta aaatttagaa     480
tttttagttt ataaagatat atttttttatt tagaggtata tggtatattt tagatatggt     540
taatttgaag atgtaaattt tattatatat aaatatttag tttatatttt aatttaaata     600
tatattttga attgtaagtt ttaaagtata aatatattaa gttatataaa tttttgattt     660
atgatgtttg ggtatataga tgtaatttaa tttaagtaat attttagttt attatggatg     720
ttttttttta gatatatttt aaatttagaa gtttagtatt ttaagtttat agaaatgttt     780
taggataatt ttgtgttgtt atatgtatgt agtggagtag agatattttt gttttagttt     840
gggggttttt ttaagtgagt ttaggttttt ttagataaat tattattaga ttagggagag     900
gagtgaattt attggatgag ttattttttt tttttattgg tattagtttt tatgtatttg     960
agtttttggt tgtgggttat ggagttggtg tttgggaatg ggatttgttt atagggagtt    1020
agaaagtagt ttttagtttt agttttgtgt taatttgttg ttagattttg gaaaagttta    1080
gtttttttta ggttttttttt tttttgggtt tttgtagtgt agtagtattg ggaaggtttt    1140
tttggttgtt tatgtggtgg gggagtagtg tggagtgtat ttttatttaa tttttgtttt    1200
gtgtagggat ttttgtaatt tttgtttttt attttagtgt atattggagg aggggaattg    1260
aaggtaaatg taggggttgg ggttgtttgt ttgtgttttt tagagttggg tttttttttt    1320
tgttgtgtgt agttgtgatt gtatgtatgt gtgtatgttt tgggagagtt tgtgtgtggg    1380
gtttggggag ggggtggttt ttaggttgag ttatgagagt ttgggttttt gtggtaggag    1440
ttgggggttg gtaagtttta gttttgtttt ttttttgatt ttggattttg ttgttgattg    1500
ggttttttat tttttttttt tagtgatttt tattttgttt tgttttttat tttgagtagt    1560
tttttaggga agttgttgtt attattgagg ttttttaggt ttttgatgaa gtttttgtag    1620
gttatgtgtt ttttgatgtt ttaaggaagg gaatggaggt ttgtgagttt ggagggtttt    1680
tggtttttta ggttttgtat gtttggttag gggtttgttt tttggttttt gtttgtttgt    1740
ttttatttat atggttgtgg agatttgtgt tgagtagtat gagtgtatag gttagtgtgt    1800
gggtgttgtt taggggagag ttgattttta gttttggttg tttttttaggg atttttttta    1860
tagttatttt ttttaatttt ttattttatt tttatttttt ttggttggta atttttttatt    1920
ttttgaattt tgttagtaaa ttttttttat aaagagtttt tttttttagta gatttgttag    1980
taaggatttg tttttggttt ttttaagtat tgagggttag ttgttttaat gtattagttg    2040
ttttaatgtt tttttgtttt taagtttgga ttatgagaaa gtgaattgta gtatagtttt    2100
gtagattagt gtagttaaat agaatttttt ttttagttaa gtattggttt gtataggtat    2160
gtaaattttt gtttttttat aatatattta attttagtaa tatgtttgtt tatatgtata    2220
gttttatata tatgtagtta tgatagtgtt gtttgtatat atgtaagttt atatgtttat    2280
atgtatgtat atttattttt tgttgtatat atatttatgt ttttgtatat atatatatagg    2340
tttagtttat tttgttattg tggttagtta aggatatttt tgtttttgga atttttttatt    2400
ttttgaggat agggttttag gattgtattg gaagtattgt tgggagaagt gggttagtat    2460
gtgtttttgt ttttgggttt tatttattaa ggttagtttt tttagaaata ttttggagaa    2520
ggggtaagag agggttgttt agtaattagg agtttttagt ttttgttttt ggggtgatag    2580
gaggttttga gagtatagta gttggggtgg gaaggggagg agagaaaggg gatgagggta    2640
tgtattttga tttttttattt attattttta tttaaggttt ttttattagg tagttttggt    2700
ttttggttat gtttagttat tttgggaagg gtgatgttag gatggtttgt gggggataga    2760
ggggtttaaa ggaggtattt tggggtttta atatgaattt tggagagtgg gggttgtttt    2820
ttaaggggtt aatatttatt tgagagtttg gtttagagtt atgtttgtga agataaagaa    2880
tttgaggtat tttttagtta ttagtttgtt gaagttattg ttgtgggtag ggtagagaga    2940
taggtagggt tttaagggga agtgtagggg gttaggtggg gattatttaa tgattagggg    3000
atattagatg gagagttgtt tttaggatgt attttttttg tttttatttt tagttttagt    3060
ttagtggttt tatttttttgt ttaggtgttg ggttatattg gtttttttga agttatttag    3120
ttggtatagt tttttggtta ggtgttgtgt agtttttagg tttgtttttt gtttattgga    3180
taaggtgttt gtgtttttta gggttagttg ttttgtgttg tttagttttg agtttgagtt    3240
ggatattagt tggtttaggg gtggtttgtt gttggggtag aatattagtt taggttgggg    3300
tagggttttt aggagttatt tagatttttt ttttgttttt aggtttagaa gatggagaag    3360
gggagggagt ggagggatat ggggaaagta tttttttttt ttttagggaa ggagattaga    3420
ttttttttat tagtttattt tattattatt attttttttgt ggaagttttg ttttaaattt    3480
aatttatatt tgttttgttg ttgtgggagt tttggttgtt tttttaagtt tttaggggtt    3540
tatatttgtt attagatata gattatttgt ttttattttg gttttatata gaatatagtt    3600
ttttgttttt tagattatgg ataaagttgg aatagatggg ttattatgga ggaattgtgg    3660
```

```
agggttaggg aaagatttga gggtagtagg gatattggtt tgaaggtttt tttttttttg 3720
tttttttaggg agaggaattt ttttttggtg tttttttttt gtttatatag aagtaggttt 3780
tggagttagg gggttaaggg ttggggtgta tatttttatt tttttttttt tttttttttt 3840
ttattttagt tgttttattt gtaaagttat ttgttagttt agagatagag gttggaaatt 3900
tttggttgtt aggtaatttg gtttttttgg ttattttttt ttgttgttat ggtttttatg 3960
attgagaggg tagttggtag aggggagagt agaaaggtta gattgtgaaa ggattttttg 4020
gagttggatg agagatgttt gattaggtag gggtgatatg ggagggaggt agtgggagag 4080
gttggtggtt gtatttttgg tgaagttttt aggttgggtg agggtaaggt tataagaatt 4140
ttgagtaggt taaagatttt gttaagggaa gaaagggttt tttaggagag taagaagggt 4200
atttttttgg gatttttttaa tgttgaatta tgttttttta agttattgga tttgagatta 4260
agaggggtgt tgtgagggat ttttttttat agttaatggt agtagttttt taaataggaa 4320
aataggattt gtatttttta attattttta tttaggggtt aggaatataa attaattttt 4380
atatttagga tttgtttttt atagtagttg tttttggagt tttgggggtt ttaagtttga 4440
gggataaaag gaattattta tttgttaagg ggtgttgttt ttagtttaag gttgtttttt 4500
gagtggtagg gattaggggg tttttttttt ggggttagtt tggttttggt ttttgttttt 4560
tttttttttt ttttttgtgt ttaaggatta ttagtagtag agttggtatt agaatttggt 4620
ttaagagggg taagtggggt gggagttggt gggttgggtt ggggtgtagg gggttgggga 4680
ggtagtttaa aggtgaagaa ggggttttgg gttgggttag gtgaggttaa ggtttttagt 4740
gaggtgaggt tggtatagga ggtgtttttg gtttggtgtg attttaggat ggttttgaat 4800
atataattga aagagttagg tttattagtt gaggggtttg ttttaggtag aaagggtata 4860
ggtgatttga gaggtatttg gtttttttggt ttgtaggggt agggagaatt tttgattagg 4920
ggtttagatt atagggttgg ggtaggattt tattgaattt ttatattgat tttgttaagg 4980
ggattattat tttttttttta ttggttttag tttattaata aaaaagagtt tttagggtag 5040
ggtgttaagt tatttagtgt tttttggggt atattagttt tattagtttt tagtatttta 5100
tttttttttt ttttgttaat tttttttttt tttttttttt tgtgatttgt tttttttttg 5160
ttattttatt tttattttta gtaatatttt aggagaagat gaggttttaa gtgatattgg 5220
ggtgtgagat ggaaaggaat gtttttatat tttttatttt tttattagga tttaggattt 5280
agatttatgt ttttgtgttt ttgggtttga ttgtggtttt tggtttttta ttttgttttt 5340
tgttttgtta gaatttttgg ttttttgtt ttttatattt ttagtgtttt gttaagtttt 5400
aggtttttgt ttagaatttt ttgttatata tattgttttg ttagttttat atttattttt 5460
attttaggtt ttttgagttt ggttttgttt gtgggttagt gtgaaggagt aagttgtttt 5520
ttttttttat tttttttttt tattttttta tttattgggt ttttttagag gttttaaata 5580
ttttgttgtt tatatttttt tttttatttg ttttattgtt gtttttattt ttattgttat 5640
tgttgaggtt gggatgaggg ttagggagtg ggttgggttg tggggtaggt gggtaggtag 5700
tgtttggtgg gtttttagag ttgggttgat ttttgatggt tgagttggtt ttttttttgtt 5760
tagttagtat tttattgatg ttagttttgt ggtagtattt taggggtatt gtgtttaggt 5820
ttgttttgga gtatttggtt gtttgtttta tagttatttt agagttttgt tttgagttta 5880
ttttaggtgg agatgggggg gtttgttttg ggggtttagt atttgtagtg tgtataggta 5940
tttaggttag gaggttattt atgttagttt tttgttttaa ttttgtttta ggatttttgt 6000
ttattgtttt atattgatag gaagtttttt tattgtttag ttattatttt ttttgttata 6060
tatttagttt gtttttggtg ttagtggagt ttattttaaa ttaagttttt gttaaatggt 6120
tttgaggagt tgggggaaga ttagtttttt tttgtttttt ttatttattt atttatttat 6180
tttgagatag ggttttattg tgttattgag gttgaagtgt agtggtgtaa ttataattta 6240
ttgtagtttt gattgtttgg atttaaggga ttttttttatt taattttttt agtagttagg 6300
attataggtg tatattatta tatttagtta attaaatttt tggtagagat ttgtttgttg 6360
tttaggttgg ttttggattt ttggttttga gtagtttttt tgttttggtt ttttaaaata 6420
ttggggttat tgagttttgt tgagtttttt ttttatttta tggaatgttt tttaatattt 6480
ttttgttttt ttttttttatt atttagtttt ttttaggtgt tttgtttagt tttatttttt 6540
agtttttttat tatagtgtag atggaaagat tgaggtttgg ggagatattg gttgatttaa 6600
gagttaattt taaggattgg gggaagtttg aaggagggtt ttttattgag ttttagttgt 6660
tgtggggaag gtttttgtttt gttttttttg ttttagtttt aggttttatt tgagttgggg 6720
gggtttaagg agttatagaa gaatttttat ttggttttag tgattttttg ggtatgagag 6780
gagatttttt ggggggagga ttaggtattt ttttgagtta atgagggaga tataggtatt 6840
tagagataaa gttagatata taaggatata agaatatggg atagaaatag aaattaaaat 6900
atgtattgat agagatggag gtttagagat atagagataa atatagaggg taagagagag 6960
gtagatttag aatagattaa aggatttaga gtgaatagta gataagttta tgtttgatag 7020
gaggttgagt tttgtttttt tattgggggt ttagggagtt taggggaagt tgtatatttg 7080
gtttaggaat ttagtgttag taggtttttt gaagagtgtg gttaggtgtt tagggggggtt 7140
ttttagttta ttggggagag aggagtaaag gttatttgtt ttaatttggg gtggggttgg 7200
tggtttatgt ataaggtttt ggttgttagg tagggttgag tttttttttgg tggggagtgg 7260
gtttgatgtg gatgtttttta gttgtaattt ttggttggag ttaggtttaa gggttggggt 7320
```

```
gggtttggga ggagaaattt tatttagatt ttagttttga tttaagtttt ttggagtagg    7380
tagtttattt agtggtttta tattggtttt ttttataaag tggaagatga ttatagagtt    7440
ttgggttttg ggtgggggtt gtttagtggg tgaagagggt gtttagggtg agggagtaat    7500
atggggtgag ggggggttat gtagaggtgt atagggtgtt gttatatgtt ttagtttttg    7560
gttttgagga tttggatttg ttatttgttg tagtaaggag tttgtgttgt tatatatgtt    7620
ggttgggggg ttttggggta ttgggttttt ggggagttag tggtgtgggt attgtggtgg    7680
ggagatggta tagttgtttt ttgagtagaa gtgtatggta ttttgggtta tgttggggtt    7740
gggggttagg ttgggggggt agggatggtg aggttaggtg gggagtaagg gggttgtatg    7800
ttttttagat aggtttgtaa gagaggaagg ggagtatggg gtgaattgtt aaggagttag    7860
atggatagga tagtagagat agggaaggga aagttggtta gggtttttga gtattaggga    7920
tttagtatat tttggtaata tgggattatt gattttatgt ttggggatta tttttataga    7980
ggtttgatga gaaaagattt atatatttag tgggtttttg gttttttttt ttttgtttga    8040
ggttttgagt attattttgg gaggagagag gaagtaattt taaagttgag tgagtttgga    8100
gtttttatgg tattttggag ttatttttag aatagggggaa gttggtattt tattttttgg    8160
aaaaggggtg ttagttagtt gttattatta tatagttttg tgatttttat agtttttttt    8220
atggaggttt tttatttagg gaagggaaat ttaggttttt tggaaggaga gggaagagga    8280
ataggtgtta tatagggatg ttgaagggtt gagaggtata gtatggaagt ggggagaaaa    8340
gtttgagtag aggtttttat ttttaatata tatatattga gtgggaaagg gatatttttt    8400
tttttttttt tttttttga gatggagttt tgtttttgtt gtttaggttg gagtggaatg    8460
gtgttatttt agtttattgt aatttttgtt ttttaagttt aagtgatttt tttgttttag    8520
atttttgaat agtttggatt ataggtgttt agtattatgt ttggttaatt tttgtatttt    8580
tagtagagat ggagttttat tatgttgttt aggttagttt taaatttttg attttagggg    8640
atttatttat tttggttttt taaagtgttg ggattatagg tgtgagttat tgtatttggt    8700
tagggatatt tttttttta tataggtata gataggtatt tatatatata taaatatata    8760
tatatatata tatatatata tatgtatata tatatataag gataaatatg ttagtatata    8820
gttaaatata tttataggta atattttttg gtatatatat agatatatat atatatatat    8880
tgtttaaaga tatattttta tgtattttta tagttataat aagtatgtgt gtaatgaaaa    8940
ggtatatgtt tgttttatat atatattatt attaaatagg aatatataaa gaggtattta    9000
aatatatata taagttgtta tttttagtg tatatagagg tttaggtgta ttagatattt    9060
atagagattt attgtttata gttatatata tatatagatg tatatatata gatgtatata    9120
tttaagtagg gatatatatt gttttttttt ttttatttat tggggttttt tgaggggaat    9180
ttgtatttat attttttttt tttttttttt tttttagggg taagatagat tttgtttagg    9240
agagattata attaggtaaa ggggttttttg gtggtaatgt aggggagggg tagaggttag    9300
gagtagtaat tttgttaggt aggttttgga agtttaatgg ggttttagtg tatatttggt    9360
gtagttttaa tttaatgtta ttttagtatt tattgttgtt tgtttagagt tgagattgag    9420
gagagataga ggagaggttg ggtttagttg agttgtgaag gtagtgtggt ttttgtttgt    9480
tttaggtttg tttgtttttt agtttttttt tttatttttt atgtgttgtt taggtaatgt    9540
taattttttt tattttttgt ttttttttag ggtttagagt tttggaggtt gttagttttt    9600
tttgttgtta ggtttttgtt gggtttttttt gttaatttaa gatttgtttt agataaggag    9660
gagggtgtat ggaatttttg gttttatttt ttagattttt atgtgtttta ttttatttgt    9720
gtttatgttt tttttgttta ttgtttttttg agttatatgt aaggtttggt tgtatatgtg    9780
tgttttgttt tagattttat gtgtgttttt aggaggttgt tatatgattt gttatatgtt    9840
atatgtgttt ttgtttttat gagtatgtgg ttgtgttaag tatatggtat gggtgtagat    9900
tggatttgtt ttttaaattt aggagattat taattatgga aaggagattt agggaatatt    9960
gttttgaatt tgttaggggt tttgagtttt ggatttagga gatttaattt tgattatttt    10020
tttaggatgt agtagggggga tgttaaatta gattttagga agaatttttt tgttgtttga    10080
gttaggtgtt gttatgtttg tttaggggta agaggttaga tgggatgaat tagggattgg    10140
aagatgtggg ggattggtaa tttttgtgtt tttggttagg gtagaagggg gttttgttat    10200
tttaagaaag agttgttgga gttattatgg taattatttt ttttgggagg gttagggtgg    10260
gtagatggtg attttggaag gaagaagggg tttgtttggg tagttagggg gtgtgaggat    10320
gtgtgaggtg ggggtgtgtg gggtttattg tggggagggg tggtgtggat tttgtgtggt    10380
gttgaagggg atagtgttag atttgttgta ttgtgtaggt gtagaggtgt gaataaagag    10440
gggaggggga tgaggtgtgt ggttggaggt ggtgtgtgtt tatgtggttt tatttttgta    10500
ggggtttgtt tttttagtta tttttttttt aagtaggttg aggaagaaga gatagtaagt    10560
ttttatttaa tttgattttt tttttgtttt tttttgttgt tagttttttt ttttttgttg    10620
ttttgtgggg tggggaaatg tggaaggaat tttgggaaaa ttagatgttt gggtttttta    10680
tttttgtttg gtttgatttt ggataagggg tttttttttt agggttttgg ttttttttatt    10740
tttaaaatgg attgatagta tagatttgtt tattttttag aggtgtttga agttttgatg    10800
agataataga tgagaagtgg tttttgttgt gatgagttag ttgaagtggt tgtattggag    10860
tgttagattg ttagtggtgt agtggttttt gtgtttttta tattttttta tatttatttt    10920
gtggatgatg ggggaaggta ttatttttgg ggtttttga agtggatttt gggattggga    10980
```

```
tttggggatg tggtgagagg tagttttgaa gttgagttta gaggtttggg gtgttattgt   11040
tttagggtgg atttagtttt agattttata attaaattta aggttagttg agaagtatta   11100
ttattggtgg gttgagttga gtttttattt tttttaggat tttggatttt tggaggaaat   11160
gaaagggatt tagttggaaa tatagttttt ttttgtgttt tttatatttt tttttttttgg  11220
tggaaatgtg gtttaaattg ttatgaagtg gaagaggttg gatagttttt tataattaat   11280
taggaatggg aggtttgagt aggtttattt gatgagggaa atattatagt tttgttaatt   11340
tatttattat tttaataatt atttattgaa tatttgttat gtggtagata gtaagagttt   11400
ggatagtttt tttttgtttt ttaaagttgt taaatgtgat tagagggtag aggagaggtt   11460
ggtaatttaa gagttttggt tttgtttttt ttgtgtttgg ttttgtttgt ttttttttatt  11520
attattttgt ttagaggtgg ttattggttt aagaggatga tagaagttag tttttgatgt   11580

ttattgtatt ttttagtttt aggttttaag ttttgtagtt gagaaggtga ggtttaatgt   11640
tatattattg gtttgttttg tttttgtttt ggggtttatt tgaaaagttt ttagattgga   11700
tttttggtga gatggtattt gattaaatag gtttgttttt ataggttaag gaggtgggtt   11760
tggtgtggtt attttaaggt taaagtgaga aaattttatt gtgtatgtgt aatagatagt   11820
tgatggagtt attgatggag ttgtttggag gggagtaaga gtttggagtt gttaggtatt   11880
gagtattgga ggggttgaga gggaagagga atttgaggga ggggagttga gttggggttg   11940
ggtttggggg ttgtttttgg gagttggggt gtaggaatat aatggaggtg gatttgtagg   12000
taatagtggt tttgggatgt tagtgttaat gtttttttat ttgtaggttt ttggatttgt   12060
tttgggaaga tgtgttgttt ttatatgttt tgaattgggt tttgttgtgt tagttgtttt   12120
ggttgtagtg tgttagttgg gttttttggt tgttggtgta gttttatttg gttgggttgt   12180
gttgttttga tgttgtgtag gtgagttggg ggttgaagtt ttgtttttgt ttgggtattg   12240
ttttgttttt agtttagttt ttagttttgt agtatgtttt tttggatttt tttgggttgt   12300
tggggttgtt tggaagggat ttatgtatta aagggtaaat atttggtagt gatttagagg   12360
gaaagtgggg tttttttttgg gagagtagga ggttgttaga aaagaattta ggttaggggt  12420
gtataggtgg ttgaggagtg tgggatgggt tgagagttgg ggtgtttttt tgtttgtagg   12480
tgggtttgta gattttgtgg gttgtattgg tttggttgtt gtgggatgtt gaggggttgt   12540
aggagttggt attggtgttg tgttatgaat ggttgttaga tgaggatttg gtgttggtgt   12600
tggtgtggaa tttgtagttg tggagtgtgg tgttgggtgg ttgtgggtaa ttgagttgtt   12660
gggtgtttgg ggttttggtt gagggttgtt tatgtttgta gtgtttgttg tttgtgtatt   12720
gtgattgggt ggatgggttg gtgttgtgtg gttttgttga ttgttgtttg gttttggagg   12780
agttggattt tattgtttgt tgttagttta aggatgaggt tattgtgtat ttggtgtaga   12840
ggtgtggtgt tggtttttgt agtttttttt tggttgttaa tgttaatgtg ggggatgttg   12900
tggtttaaga gttggtttgg aattgtttag aattttatta ttttgatttt attggttgtt   12960
tttgtgttgg aagtgatggt gttaggtgtt tgggtttgat agggtgggag gagggtagtt   13020
atttagtttt tgttggtatg gggtgggttg tagttgttaa aaggttggat tgagggtttt   13080
gaattttttt gtaaattata gtatttttat tttgaataga aagggttttt aggattgttt   13140
aggttagaga gtttatagtt taaaaatttt ttttaattaa tgtttatata ttgggagatt   13200
ttatatagaa agttgatttt tggttttttt ttaataaggg ggagattttg taattttagg   13260
tggagttgag ggttaggttt ttttttaaa tgagatagga agtttttagt ttattggaag   13320
ttaatttagg tgtttttttt gtgtagggat ggaaaaagta tgtttttgtt ttgagttgga   13380
gggagattgg gattttgttg gagttagttt gaggttgagt gagtttgggt ttttatttta   13440
ttttattaaa ggtgtggtgg ttttatgttt ttagttttat tttgtttttt ttttaggata   13500
ttggttgagt attgttttgt gttgtgtttg ttgtgggtgt ggtattgtta ttatgtggtg   13560
gagtttagtt tgagttgttt gtggaagtgt ggtgtggata ttgatgtgga gttgttgttg   13620
tattaggttt tggtgttgtt gtaggatatg gtgggttttg tattttttgt taatttgtag   13680
gtttgatttg tttgttaatt ggagt                                         13705
```

<210> 254
<211> 13705
<212> DNA
<213> Artificial Sequence

<220>
<223> chemically treated genomic DNA (Homo sapiens)

<400> 254

```
gttttgattg gtaggtgggt tagatttgta ggttgataaa aggtgtgaag tttgttatat      60
tttgtagtag tattagggtt tggtgtagtg gtggttttat gttgatgttt atgttgtgtt     120
tttgtaagtg gtttaggttg gattttgtta tatggtggta gtgttgtatt tgtagtgaat     180
```

```
gtagtatggg gtagtatttg gttaatgttt tgagggagga gtagggtgag gttagggata     240
tgaggttatt atatttttgg tggggtaagg tggaggtttg ggtttattta gttttggatt     300
ggttttggta aaattttggt ttttttttag tttggggtag gggtatattt tttttgtttt     360
tatataaggg aggtgtttgg attggttttt agtgagttgg agattttttg ttttatttaa     420
ggggggaatt tggtttttag ttttgtttag gattgtaaga tttttttttt gttgaagaaa     480
agttagaagt tgattttta tgtgaaattt tttaatgtgt aaatattggt tgaaaaaagt      540
ttttaaatta tgggtttttt ggtttaggta attttagagg ttttttttgt ttagaatggg     600
ggtgttgtgg tttgtaggaa gatttagaat ttttagtttg gtttttttaat aattatagtt    660
tgttttatat tagtagaggt taagtgattg tttttttttt gttttattag gtttaggtat     720
ttgatattgt tgtttttgat gtggaggtag ttggtgaggt taaggtggtg gagttttggg     780
tagttttgag ttaatttttg aattgtggtg tttttatgt tggtgttgat ggttagagag      840
aggttgtgga gattagtgtt gtgtttttgt gttaggtata tgatggtttt gtttttgagt     900
tggtggtagg tggtgagatt tagtttttttt agggttgggt agtgattagt gaggttgtgt    960
agtgttagtt tgtttattta gttatagtgt gtgagtgata ggtgttgtag gtgtgggtag    1020
tttttggtta aagttttaag tgtttggtga tttagttgtt tgtagttgtt taatgttata    1080
ttttgtagtt gtggatttg tgttagtatt ggtattaggt ttttgtttga tagttatttg     1140
tgatatggtg ttagtgttag tttttgtagt tttttggtat tttgtagtag ttgggttaat    1200
gtggtttgtg ggatttgtgg atttatttgt gggtggggag gtattttaat ttttagtttg    1260
ttttgtattt tttagttgtt tatgtatttt tgatttgagt ttttttttgg tagtttttttg   1320
tttttttagg agagatttta tttttttttt gagttgttat tgagtatttg tttttttggtg   1380
tgtggatttt ttttgggtag ttttggtggt ttaagggggt ttagaagggt atattgtggg    1440
gttgggggtt gggttagagg tggggtggta tttaggtagg ggtggggttt tagttttttgg   1500
tttatttgtg tggtattgaa gtgatgtagt ttggttaggt gaagttgtat tagtgattgg    1560
aaggtttggt taatgtgttg tagttggagt agttggtgta gtgggatttg gtttaggatg    1620
tgtgggagta gtatgttttt ttagggtagg tttaggaatt tgtgagtaaa ggggtgttgg    1680
tattggtgtt ttggggttat tattatttgt gagtttgttt ttgttatgtt tttgtgtttt    1740
agttttaag agtggttttt agatttagtt ttggtttggt tttttttttt taggtttttt     1800
tttttttttg gtttttttgg tgtttggtat ttgatggttt tgggtttttg tttttttttg    1860
gatggtttta ttggtggttt tattggttgt ttattgtgtg tgtgtagtaa gatttttttg    1920
ttttggtttt ggagtggttg tattaaattt gttttttttgg tttgtagaga tagatttatt   1980
taattgagtg ttgttttatt gaggatttga tttgaaagtt ttttgggtgg attttgaaat    2040
ggaggtggaa tagattggtg gtgtaatatt aggttttgtt tttttggtta tagggtttga    2100
ggtttgaagt tggagaatgt ggtgggtatt agagattggt ttttgttgtt tttttgagtt    2160
agtagttgtt tttgagtgaa gtggtggtgg gaggagtagg taaggttaaa tgtggagggg    2220
gtggagttag gatttttagg ttattggttt tttttttgtt ttttggttgt gtttagtgat    2280
tttgaaaaat ggggagaaat tatttaggtt tttattgttt gttatgtagt aggtgtttaa    2340
taagtaattg ttgaaataat gaatgaattg gtgaggttat aatgtttttt ttattaagta    2400
aatttgttta aattttttat ttttgattaa ttatgaagag ttatttagtt ttttttattt    2460
tgtaatagtt tgggttgtgt ttttgttggg ggagagagat gtagggggtg tgaagaaaga    2520
ttatattttt aattgggttt tttttatttt ttttaaaggt ttagagtttt aagaagaatg    2580
agagtttagt ttagtttgtt agtggtagtg ttttttggtt ggttttgggt ttagttgtag    2640
ggtttggggt tgggtttgtt ttgggataat gatgttttag atttttggat ttagttttag    2700
ggttgttttt tattgtgttt ttaggtttta gttttaggat ttgttttggg gaattttagg    2760
agtagtgttt ttttttatta tttatgggat gggtgtgggg ggatgtgaag gatgtgagag    2820
ttattgtatt gttgatggtt tggtgttttg gtgtaattgt tttagttggt ttattataat    2880
gaaaattatt ttttatttgt tattttgtta aggttttggg tatttttgaa aggtgggtag    2940
gtttatgtta ttagtttatt ttaaagatgg ggaaattgag gttttggaga ggaagttttt    3000
tgtttagaat taagttagat gggaatagag aatttagata tttggttttt ttagaatttt    3060
ttttatgttt ttttattttg taaggtagtg agaagggaga agttagtagt ggggagagat    3120
aagaaagggg ttaagttagg tgagggtttg ttattttttt tttttagtt tgtttaggga     3180
gggggtagtt gggaggatag atttttgtaa agatagagtt atgtgagtgt gtattatttt    3240
tggttgtgtg ttttgttttt tttttttttt tgtttgtgtt tttgtgtttg tgtagtgtga    3300
taaatttggt gttgtttttt ttagtattat gtggagtttg tgttgttttt ttttgtggtg    3360
agttttgtgt gtttttgttt tgtatgtttt tgtgtttttt gattgtttga atagattttt    3420
tttttttttt gaggttatta tttgtttgtt ttggtttttt taaagagagt agttgttata    3480
gtaattttag tggtttttttt ttaaaatagt agggtttttt tttattttgg ttaagggtat   3540
aagggttgtt ggttttttat gtttttggt ttttagttta ttttgtttaa tttttttattt    3600
ttgggtaggt gtgatgatat ttaatttagg tagtaaggaa gttttttttg aagtttgatt    3660
taatattttt ttgttgtatt ttgggagggt ggttagggtt ggattttttg ggtttgagat    3720
ttaaaatttt tggtgggttt aagatggtgt tttttagatt tttttttttat agttgatggt   3780
tttttggatt tagggggtgg gtttagttta tatttatatt gtgtgtttgg tgtggttgtg    3840
```

```
tgtttgtggg ggtgggagtg tgtgtggtat gtaataggtt atgtggtagt tttttaggga    3900
tatgtgtggg gtttggagtg ggatatgtgt gtgtagttgg gttttatgtg tggtttaagg    3960
ggtggtgggt gagagagatg tggatataag tggggtgggg tatgtgaggg tttggggaat    4020
agaattaggg gttttatgtg tttttttttt tgtttggggt gggttttagg ttggtgggag    4080
agtttagtgg ggatttagtg ataagggggg ttggtagttt ttggggtttt gggttttggg    4140
agagggtggg gggtgggggg gattagtgtt gtttgagtgg tgtgtagggg gtagggggag    4200
ggattgagag gtgggtgggt ttggagtaaa tgggagttgt gttgttttta tagtttagtt    4260
gggtttagtt ttttttttgt ttttttttgg ttttagtttt gggtgagtag tggtgagtat    4320
tgaggtggta ttgagttggg gttgtattgg gtatgtgttg ggattttgtt gagtttttgg    4380
aatttgtttg gtagggttgt tatttttgat ttttgttttt tttttgtatt gttattagag    4440
gtttttttat ttggttatgg ttttttttgg gtgggatttg ttttattttt ggggagagag    4500
ggagaggaga ggaatatggg tgtagatttt ttttaagaga ttttagtagg tggggggggag    4560
gggatggtgt gtgtttttgt ttgagtgtgt atatttgtat gtgtatattt gtatgtgtat    4620
gtgattgtga gtagtaggtt tttgtgagtg tttgatatat ttggattttt gtgtgtattg    4680
gagaatgata gtttgtgtat atatttgggt atttttttgt gtgtttttgt ttgatgatgg    4740
tgtgtgtgta aaataagtat gtgttttttt attatatata tatttgttat gattgtgaga    4800
gtgtatgagg gtgtgttttt gagtagtgtg tgtgtgtgtg tgtttgtgtg tgtgttagag    4860
ggtgttgttt atgaatgtgt ttaattgtgt attgatatat ttgtttttat gtatgtgtgt    4920
gtgtgtgtgt gtgtgtgtgt gtgtgtgtgt gtttgtgtgt gtgtaggtgt ttgtttgtat    4980
ttgtatgaag aaaagaatgt ttttggttgg gtgtggtggt ttatgtttgt aattttaata    5040
ttttgggagg ttaaagtggg tggatttttt gaggttagga gtttgaaatt agtttggata    5100
atatggtgaa attttatttt tattaaaaat ataaaaatta gttaggtata gtgttgagtg    5160
tttgtaattt aagttattta ggagtttgag gtaggagaat tatttgaatt tgggaggtgg    5220
aggttgtagt gagttgagat agtgttattt tattttagtt tgggtagtag gggtgaaatt    5280
ttattttaaa aaaaaaaaaa aaaaagaaga atgttttttt tttatttagt atgtatatat    5340
tgggggtagg agtttttgtt taggtttttt tttttatttt tatgttgtgt tttttagttt    5400
tttagtattt ttatataata tttgtttttt tttttttttt ttttaggagg tttgagtttt    5460
ttttttttga gtgaggggtt tttataagag agattatagg ggttataggg ttgtatgatg    5520
gtgatagttg gttgatattt ttttttagga gggtggggta ttaatttttt ttattttaga    5580
agtagtttta aagtgttata agaattttgg gtttatttaa ttttgaagtt gttttttttt    5640
tttttttagg gtgatgttta gggttttagg tagaaggaaa ggagttaggg gtttattggg    5700
tgtataagtt tttttttgtt aggtttttat ggaagtagtt tttaggtgtg ggattaatga    5760
ttttatatta ttagggtgtg ttaggttttt gatatttaga ggttttagtt aatttttttt    5820
tttttatttt tgttgtttta tttatttaat tttttggtag tttattttat gttttttttt    5880
ttttttttata ggtttgtttg aagagtatgt agttttttta ttttttgttt ggtttttgtta    5940
tttttgtttt tttagtttga tttttggttt tagtatggtt tagggtgtta tgtgtttttg    6000
tttggaaggt gattgtgtta tttttttatt atgatgttta tgttgttggt tttttgaagg    6060
tttggtgttt tagagttttt tagttagtat gtatggtagt ataggttttt tgttatggtg    6120
agtggtaggt ttaggttttt gaggttggga attgggatgt gtgatagtat tttgtatatt    6180
tttgtgtggt ttttttttat tttgtgttgt ttttttattt tgggtatttt ttttatttat    6240
tgggtagttt ttatttgggg tttagagttt tgtggttatt ttttgttttg tggagaaggt    6300
tagtgtgagg ttattgaatg ggttatttgt tttagggggt ttgagttgga gttgggattt    6360
gggtggggtt ttttttttta ggtttatttt agtttttggg tttggtttta attggaagtt    6420
atggttggaa gtatttatat tagatttatt ttttgttaga ggaggtttgg ttttatttgg    6480
tagttggaat tttgtatatg ggttgttagt tttattttag gttggagtag atggttttta    6540
tttttttttt tttaatgggt tggggggttt ttttgagtgt ttggttatat tttttggagg    6600
atttgttgat attggatttt tgaattaggt atgtaatttt ttttgggttt tttgggtttt    6660
tagtgaaggg gtaaggttta gtttttttgtt agatatgggt ttgtttgttg tttattttga    6720
atttttttaat ttgttttaaa tttgtttttt ttttgttttt tgtgtttgtt tttgtgtttt    6780
tgaattttta tttttgttga tgtatgtttt aatttttgtt tttgttttat atttttgtgt    6840
ttttgtatgt ttggttttat ttttgggtgt ttgtgttttt tttgttggtt tagggggata    6900
tttggttttt tttttgggaa gttttttttt atgtttagag aattgttaga gttaaatggg    6960
aattttttta tggtttttttg gatttttttta gtttaggtaa ggtttgggat tgaggtaggg    7020
agaatagggt agggtttttt ttatagtagt tgaggtttag tagggagttt ttttttaaat    7080
tttttttaat ttttggaatt ggtttttgag ttagttaatg ttttttttagg ttttaatttt    7140
tttatttgtg ttatgatggg aggttggagg ataggattgg gtagggtatt tggaggaagt    7200
tgagtaatgg agggagggga tagaaggata ttgaggggta ttttatggag tagaaaagag    7260
gtttagtagg gtttagtggt tttagtattt tgggaggtta aggtaggagg attgtttaaa    7320
gttaggagtt taagattagt ttggataata agtaagtttt tattaaaaat ttaattagtt    7380
ggatgtagtg gtgtgtattt atagttttag ttattgggga ggttgggtgg gaggattttt    7440
tgagtttagg tagttaaggt tgtagtgagt tatgattgta ttattgtatt ttagttttag    7500
```

```
tgatatagtg agattttgtt ttaaaataaa taaataaata aataaaaaag atagaaaaag    7560
gttggttttt ttttagtttt ttaaagttat ttgataagaa tttgatttaa aatgagtttt    7620
attggtatta ggaataagtt aagtatgtag taggaggaat ggtggttaga tagtgaaaga    7680
attttttgtt aatatgaggt agtgggtaga gattttagag tagaattagg atagggagtt    7740
gatgtggata attttttgat ttggatgttt atgtatatta taggtgttaa gtttttagag    7800
taggtttttt tatttttatt tggggtgggt ttaaggtagg gttttggggt ggttgtgggg    7860
tgagtagtta agtattttga gatagatttg gatatggtgt ttttgaggtg ttattgtgag    7920
attgatattg atgaggtgtt ggttgagtgg gaggaggttg atttggttat tgaaagttag    7980
tttagttttg agggtttatt aggtattgtt tatttatttg ttttatggtt tggtttattt    8040
tttggttttt attttagttt tggtagtggt aatgaggatg aggatgatga tgaggtaggt    8100
ggggaagaag atgtggatga tgaggtgttt gaggtttttg aaggggtttg gtgagtgggg    8160
aagtgggagg gggaagtggg agagggaagt ggtttgtttt tttgtattgg tttataggta    8220
gagttgggtt taaagggttt gggatagaga tgggtgtggg gttggtgggg tagtgtgtat    8280
agtaggagat tttagataga ggtttgaggt ttggtagaat attggggatg tggggagtaa    8340
agaggttagg ggttttagta gaataggggg tagggtgaga ggttagaggt tatagttaag    8400
tttaggaata taggagtatg agtttggatt ttggattttg gtgggagagt ggggagtgtg    8460
gggatatttt tttttgtttt atattttagt gttgtttgga gttttatttt tttttaagat    8520
gttgttagaa atagaaatgg gatggtagaa gggaagtgga ttatggaaaa ggaggagaaa    8580
gggagattag tagggaggga ggggatgggg tgttggggat tggtagggtt ggtgtgtttt    8640
aggggggtatt gaatagtttg gtgttttatt ttagaggttt ttttttgtta gtgggttgga    8700
gttagtgaaa aggggatgat aattttttta gtagagttag tgtgagagtt tagtgaaatt    8760
ttgttttagt tttgtgattt gggtttttaa ttagagattt tttttgtttt tgtaggttag    8820
ggagttggat gtttttttaag ttatttgtgt ttttttttatt tgggatgagt tttttggttg    8880
atgggtttga ttttttttagt tgtgtgtttg aagttatttt ggagttatat tgggttaaag    8940
gtatttttta tattagtttt gttttgttgg aggttttggt tttatttggt ttaatttaga    9000
gttttttttt tatttttgag ttgttttttt aatttttttgt attttggttt gatttattag    9060
tttttgtttt atttgttttt tttgaattgg attttggtat tagttttgtt gttgatggtt    9120
tttggatata gagaggggag gaggaggagg tagaggttag agttaagttg gttttaggga    9180
gggagttttt tagtttttgt tatttagagg atagttttgg gttgggggta gtatttttttg    9240
gtaggtaagt aatttttttt gtttttttaga tttgaggttt ttagggtttt agaaatagtt    9300
gttgtaaggg atagattttg agtgtgagga ttggtttata tttttgattt ttgagtaggg    9360
gtggttaagg gatgtagatt ttgttttttt atttggagga ttattgttat tagttgtgag    9420
ggagagtttt ttatagtatt ttttttggtt ttaagtttag tggtttgaga aagtatgatt    9480
tagtattgaa ggattttagg agaatatttt ttttgttttt ttggagggtt tttttttttt    9540
ttggtagagt ttttagtttg tttagaattt ttgtgatttt atttttattt agtttgaagg    9600
ttttattaaa gatgtagtta ttagtttttt ttattgtttt tttttttatgt tatttttgtt    9660
taattaggta tttttttgttt agttttagga agttttttta tagtttaatt tttttgtttt    9720
tttttttgtt agttattttt ttagttatgg aagttatggt aataggaggg ggtggttagg    9780
gagattgggt tgtttagtaa ttaggagttt ttggtttttg tttttgggtt gataggtggt    9840
tttgtgagta gagtagttgg ggtgggaagg ggaggaaaga aagggaatga gggtatgtat    9900
tttgattttt aatttttttaa ttttagagtt tgtttttgtg tgggtagagg gggagtatta    9960
aggagaggtt ttttttttta aaaagtagga gggggaaagt ttttggatta gtatttttgt   10020
tgtttttaaa ttttttttttg gttttttata gtttttttgt ggtagtttat ttattttagt   10080
tttgtttata gtttaagaga taaaggatta tgttttgtgt agggttaggg tgggaataga   10140
taatttatgt ttaatagtaa atgtaggttt ttgggagttt gaggaagtag ttagaatttt   10200
tatagtagta agatggatgt gggttagatt tgaggtagga tttttataga gaggtggtaa   10260
tgatggagta ggttgatgga gagaatttaa tttttttttt tggaaaaaag aagaatgttt   10320
tttttatgtt tttttatttt tttttttttt ttattttttg ggtttggagg tagaaggagg   10380
gtttggatgg tttttaagga ttttgtttta gtttgagttg gtgttttatt ttggtagtga   10440
attattttttg agttagttgg tgtttgattt agatttagag ttggatagta tagagtggtt   10500
ggttttggga agtatagata ttttgtttaa tgggtagaaa gtggatttgg aggttgtgta   10560
gtgtttggtt aagaggttgt attgattaga tggttttagg aaggttgatg tggtttggta   10620
tttgggtaag aagtaaagtt attaggttgg gattgaggat gggggtagag ggaatgtatt   10680
ttgaaggtag tttttttattt aatgtttttt ggttattggg taatttttat ttgatttttt   10740
gtattttttt ttgagatttt atttgttttt ttgttttgtt tatagtaatg attttagtaa   10800
attggtggtt ggggagtatt ttaagttttt tgtttttatg ggtatgattt tggattaagt   10860
ttttaggtgg gtgttgattt tttggaggat agtttttatt ttttggggtt tgtgttaggg   10920
ttttaggatg tttttttttga gtttttttgt tttttataag ttgttttgat attattttttt   10980
ttaagatggt tgggtatggt taagggttag agttgtttgg taaagggatt ttgggtaggg   11040
gtgatgggtg gagggttagg gtgtatattt ttatttttttt tttttttttt tttttttatt   11100
ttagttgttg tatttttaaa gtttttttgtt attttagaga tagaggttgg aaatttttgg   11160
```

```
ttgttaggta attttttttt gttttttttt tagggtgttt ttgaaggagt tggttttaat   11220
gggtgagatt taggaatgag agtgtgtgtt ggtttatttt ttttagtgat atttttagtg   11280
taattttgaa gttttgtttt tagagggtga ggagttttga gagtaggggt gtttttagtt   11340
ggttatagtg gtaaaatggg ttgggttttg tgtgtgtgta taggagtatg agtgtgtgta   11400
tagtagggaa tggatgtata tgtgtgtagg tatgtgaatt tgtatgtgtg tagatagtgt   11460
tgttatggtt gtatgtatgt ggaattatat atgtgggtag gtatattgtt agggttgagt   11520
gtgttgtgaa gaggtaggag tttgtatgtt tgtgtaggtt agtgtttggt tggagagggg   11580
attttgtttg gttgtattgg tttataggat tgtgttgtgg tttatttttt tgtggtttag   11640
gtttagaggt aagggaatgt tggggtaatt agtatgttgg ggtaattagt ttttagtatt   11700
tgagggagtt agggatgggt ttttgttgat ggatttgttg gaagaggagt tttttgtggg   11760
gagggtttat tagtggggtt taggaggtag gaagttgttg gttaaagggg gtggggatga   11820
aatgggaggt tggggaagat ggttgtggga agggtttttg ggaggtagtt aaggttgaaa   11880
gttagttttt ttttagatgg tgtttatatg ttgatttgtg tgtttatgtt gtttaatatg   11940
gatttttatg gttatgtgag tggaggtggg tgggtaggag ttggaaaata aatttttggt   12000
tagatatgta ggatttggga ggttaagggt tttttaaatt tataggtttt tgtttttttt   12060
tttagaatat tgggaagtgt atgatttgtg gggattttat tgggaatttg gagggtttta   12120
atgatggtgg tgattttttt agggagttgt ttaaggtggg gggtggggta gggtagaagt   12180
tgttggaggg gaaggatgga ggatttagtt ggtgatggag tttagagttg gagggagggt   12240
agggttgggg tttgttgatt tttggttttt gttatggaag tttgggtttt tgtgatttag   12300
tttggggatt gttttttttt taggttttat gtgtaggttt ttttggaata tgtgtatatg   12360
tgtgtggttg tgattgtgta tggtggggga ggggatttgg ttttggaaaa tgtgagtagg   12420
tggttttggt ttttgtgttt gtttttagtt tttttttttt agtgtgtgtt ggggtggaag   12480
gtgggagttg tgggggtttt tgtatgggat agggattgga tgggggtata ttttatattg   12540
tttttttgtt gtgtggatag ttaaaaagat ttttttggtg ttgttgtgtt gtaggaattt   12600
ggagggagag gggtttgaga gggattaagt ttttttaagg tttggtagtg agttagtgta   12660
aagttggagt tagaaattgt tttttggttt tttgtgagta ggttttgttt ttaggtgtta   12720
gttttatggt ttgtaattga gggtttagat atgtaggggt tggtgttggt aggagaaggg   12780
agtggtttat ttagtgggtt tatttttttt tttgatttgg tggtagttta tttagagagg   12840
tttggattta tttgaagaaa tttttaggtt aggataaagg tgtttttgtt ttattatata   12900
tatgtaatag tataggattg ttttagggtg tttttgtgag tttggggtgt tgggtttttg   12960
agtttggggt atatttgaga gggaatgttt atggtgagtt ggggtgttgt ttgagttgag   13020
ttgtatttgt gtgtttgggt gttgtgaatt aagggtttgt atggtttggt gtgtttgtgt   13080
tttagggttt atgatttaga gtgtgtgttt gagttggggt gtaggttgga tgtttatgtg   13140
tggtggggtt tatattttttg aattggttgt gtttgaggta tgttatatgt ttttgagtag   13200
ggggtgtatt tttgtgagtt aaagatttta agttttgaag tgtttatttg aatgggtagt   13260
gtttgtgagt tggggtgatt gtgaattggt gtgtttggtt tgttggggtg tatgttagaa   13320
ttgaggtgtg ttatgtgatt ttagatttat atatgggtgg ggtatttgtg ggtgagttgg   13380
atgaggttag ggttgttttt tttttgtttt tgttttttttt tgaggttttt tttggtttag   13440
tggaaaaggg ttgtagggaa gtggtttgat tttttttttt ttattatttt aatttagatt   13500
agttagaagg gagttttaga ttttgttggg tttgtttagt gttatagttt taggtttagt   13560
ggtgttttta gtttttttgg agttgttatt tatgttggtt tttttaggtt ttttttttgtg   13620
gttgtgtggg gagggttttt tttgtgattg ggtgttttta ttttatgtgg tttttgtttt   13680
tagtttttgt tttttttttt ttgtt                                         13705
```

<210> 255
<211> 3574
<212> DNA
<213> Artificial Sequence

<220>
<223> chemically treated genomic DNA (Homo sapiens)

<400> 255

```
tatattgttt tttttggtga gttattttat tttaagtatt tttttttatt atatgatttt      60
gttttatttt tttattgtat taagaatatt tgaaatgtat atgtttatat ttatatagtt     120
tatttttttt tgagaatgga agttttatag aaggtgaaat aaattttgtt agttgttgtt     180
agttgtatag taagtttttt gtagaaaatg tttattggtt atattttttt aaaaagtgaa     240
taaattttaa gattaagatt ttaaattaaa ttattttagt ttttaattgt ttatagtgat     300
atattgagaa attgagtgat ttttaagaga aaggttattt tgaggttggg gttgaattaa     360
aatttagggg gtttatatta ggttagtatt ttggaattgg ttgttttgta agattttggg     420
```

```
agtattaaat tggtatgaga tatttgataa tgaggttagt tatgtattga gtttaggttt     480
tatttttgtt gttttttttga tgttagaaaa agtatgagtt gttgtatagt ttttttttatt     540
aaggtgataa attatatttt tgttgggttt tgtattttgt aaattataaa tttattttttt     600
tttttttttt tatattgaaa aatgtaaaaa tttattgtag agaggttaaa atttttttta     660
aatgtaaata aatattgttt tttagtagag gtgttttttt ttatttttgt tatatatatt     720
attgtggatt tgtttgagaa agtatgtgat tttggtggta gttaggtttt taatagagaa     780
tgagggtttg tataaatttt gggtttgtgt tgtagagtaa gataaaggat tttgaattgt     840
agtattattt tttttagttt aaggtagttt gtgaaaggta ttgggagtgg tgtgatgtgg     900
gtattgtgaa atttttttttg ttgttatatg tatttgtgtt ttttttaatt atagtttagt     960
ataaatttta ggtgggtgat ttggtaggta aatggtgttt aggtagttgg gtaatttatt    1020
gggttttagt gggtgttggg gagttttatg tgttaggagg tggataattt aggggggtaag    1080
gggtttgtat aagggtgaag atgtgttttt tttttggtat tgatttatgt gtttaaaggt    1140
ttttgtggta atgtttaggg atgtgaatta ggtgttgggg gttggggagg aggtgggatt    1200
ggaagtgaga gtagtttgtg tttaagattg tgtaagaggt ggttttttgt ttgttggttt    1260
tagtatattt tggtttttga aaaaatatta tttttgtttt ttgttatttt ttgtatttta    1320
gaaaattttt tttagggtag gaaaggatag tgagttattg taagtatttt ttagtaattt    1380
ttttttgtgt ttttttattt ttttgtgtta tagaggggta gagtattagg aaggatggta    1440
aatatgtttt ggtttagtgg tggtttgtgg ttgttattta gttggttgta tgtagtattt    1500
tttggatatt atatgatttt ttttgggttt tgtggatata ggaaattttt attaaaggtt    1560
ggggatttaa atatttttag aatatggttg tgtttttggg ttagttgtgt ttttttttta    1620
gatgtgtgtg gtttttggtt tttgtaaaaa tggaattaga tttggtatta tattttgtta    1680
tagttatagg aaaatatatt aaagttatag gaaaataaat taaaaagtaa gttatagttt    1740
atgtaaaggt gaatttgggg gttttggtgt ttttttttgt tgttgtgtgt gtttgggaag    1800
tataggagat ttggattttt ttttttttaa aagtaatatt tgaaagtatt gggtatagtt    1860
gagttttttt tgtttttatg tttgtagaga tgatttttat gttatttttt tttttgatat    1920
ttaaattttt ttttgttgtt tgtttagttt tttttgggaa tggataatta ttttattttt    1980
ggaaataaag ggaaaggtgg taaaaggtga tgtggaggtg tttgtttttt tggggttttt    2040
atttttggta gtttgatgtt ttttgtgtta aggtttggtt gtggaggttg ggaaaatgtg    2100
gtttttgtta gtaagggttg ggtagggagt ttggtgtggt ttggtggatt ttggaggaag    2160
gtgaagggtg gaggtttttg gtgatttttt ggtggagtgg tgtggttgtt ttagtttgtg    2220
tttgtttggt tttttttttt tttgggttga ttgtattaga attttttttt attattaatt    2280
tttatttata ttttgtttgt tggttttgta tttatattga tatgtattgg tgttgagggt    2340
tatgaattgg agttattttt ttattggggg atgtgtggtt tttttgtaat tatggtttgt    2400
gatggtttaa tgtggatagg ttattttttt ttgttttgtg tgtggttttt tttttttttg    2460
tgtgtttgtt ttttaattaa agttgtgtgt tagtgagttg tgttggttat atggtgttat    2520
tgttggtggt tgtattttgt tgtttttttt ttgtagtttt ttgatgatgt agtgattgtg    2580
gttggtggag attgagtaat ggggaaaggg tgagggaggg gttgtggggt gtgtgtgtgt    2640
ttggtgtatt gaagttggta ttgggagtag aggttgtgtg ttgttttggt atttgttttt    2700
tttttttttg gggtttggtg gtgtgtttgg aatttagttg tggttgtagt tggagaatga    2760
gagtttttag ggatgggggt ggggtggtta aaggggtatt ggtggaggat aggagggttg    2820
tgtgttttgt tggggtttgt attttgggta tgttagagtt ttgttggggt tgaaggagtg    2880
agggagttgt attgtgggaa gggtgtagta tgaggatggt tgtgggagtg gtaggtttga    2940
gtgtagtttg gagagttgtg ggtagtgttt gggattatgg gtagtgttgg agtggagggt    3000
tggttttggg tatagtttgt agatggtttg ggttggtggg aaggggattg aagtggtagg    3060
ggtggtgggg agtttgggta tttttaaaaa tttttttgttt ttaatttgaa gtgggaggta    3120
tttttttttt tttggattta gtatttgtag atttggttag tttttttttga gatttgtttt    3180
gtggagtttt gttttgggga ttgggtgtga ggtgaattag aaggatttgt tgagtattga    3240
gaaagtattg gtttaggtgt aaagagttgg gggttagttt ttggagggtt tggagatttt    3300
atgtggtttt gattgaagga tttggtgtgt ttaggttaat ggagttgggg tgttttaagt    3360
ttgggggatg agggagtggt atggaggggga gtaggggtaa ttgggtttgg tttttttttt    3420
ttttgtttat tttttgtttt agatatagtt ttttttttatt ggttgggtat ttgtttgtag    3480
aggagttgtg ttttaaaatg gtgataggaa attttatttg tgttttagtt aattagattg    3540
ttatagtgtg aatttgtagg gtggaaatgt gagt                                3574
```

<210> 256
<211> 3574
<212> DNA
<213> Artificial Sequence

<220>
<223> chemically treated genomic DNA (Homo sapiens)

<400> 256

```
gtttgtgttt ttattttgta aatttgtgtt atgatagttt aattggttga ggtgtaggtg      60
ggattttttg ttgttatttt aagatataat ttttttgtaa gtaagtgttt agttagtgaa     120
agggggttgt gtttgaaata aagagtaagt gggaaaaaag ggaattaggt ttagttgttt     180
ttgttttttt ttatgttgtt tttttgtttt ttggatttgg agtgttttag ttttgttaat     240
ttggatgtat taggtttttt ggttgggatt atgtggggtt tttaggtttt ttaagagttg     300
gtttttggtt ttttgtgttt aggttggtgt ttttttggtg tttagtaaat ttttttggtt     360
tattttatat ttagttttta aagtagagtt ttatagggta gattttagga agggttgatt     420
aggtttgtgg gtgttggatt tggaagaggg gaggtgtttt ttgttttagg ttagggataa     480
agggttttta aaggtgtttg agttttttgt tgtttttgtt attttggttt ttttttttgtt    540
aatttgggtt atttgtaggt tgtgtttagg gttagttttt tgttttaatg ttgtttgtag     600
ttttaggtgt tgtttgtggt tttttgggtt gtgtttggat ttgttatttt tgtggttatt     660
tttatgttgt gtttttttta taatgtaatt tttttgtttt tttggttttg gtggggtttt     720
ggtgtattta aaatgtaggt tttagtggaa tgtatagttt ttttgttttt tgttggtgtt     780
tttttagttg ttttattttt atttttaagg gtttttattt tttagttgta gttgtagttg     840
ggttttgaat atgttgttgg gttttgggag ggagaggggt aggtgttagg gtggtgtgta     900
gtttttgttt ttggtgttag ttttagtgtg ttgggtatat gtgtgttttg taattttttt     960
tttatttttt ttttgttgtt taatttttgt tagttgtggt tgttgtattg ttagagagtt    1020
gtaagaggga ggtagtaagg tgtggttgtt agtagtgata ttatgtgatt ggtatggttt    1080
attgatatgt agttttggtt aaagagtggg tgtataggag gggaggagat tgtgtgtggg    1140
atggggagga atggtttgtt tgtgttaaat tattataagt tatggttgtg gaagggttat    1200
gtgtttttta gtaggagaat gattttgatt tgtgattttt agtgttggtg tatgttgatg    1260
taagtgtgag gttggtgagt agagtgtgag tgggggttgg tggtgagggg agattttgat    1320
gtaattggtt tggaaaggag gagagttagg tgagtgtgga ttggggtggt tatgttattt    1380
tgttagaagg ttgttaggag tttttgtttt ttattttttt ttgaaatttg ttaggttatg    1440
ttaagttttt tgtttaattt ttattgatgg gggttatatt tttttggttt ttgtagttag    1500
attttgatat aaaggatatt aaattgttga gggtaaaaat tttggaaggg tggatatttt    1560
tatattgttt tttgttattt ttttttttat ttttggaggt aaggtgatta tttgtttttg    1620
gggagggttg ggtgagtggt aaggggagat ttagatgttg gaggaggagg taatgtagaa    1680
attatttttg tgggtgtgaa agtagagaga gtttggttgt gtttgatgtt tttggatgtt    1740
gtttttagga gaggaaaagt ttaggttttt tgtgtttttt gaatgtatgt ggtagtggga    1800
ggaagtgtta gggtttttga gtttattttt gtgtgggttg tggtttattt tttggtttgt    1860
tttttgtga ttttggtgtg tttttttgtg gttgtggtag agtgtggtat tagatttggt    1920
tttattttta tgagagttag gaattatgta tgtttggagg gagagtgtag ttgatttgag    1980
ggtgtgattg tgttttggga gtgtttgaat ttttggtttt tggtgaaaat tttttgtgtt    2040
tgtaaggttt agaggagatt gtgtgatgtt tggggggtgt tgtgtgtggt tggttgggta    2100
gtggttgtgg attattatta aattggggta tgtttgttgt tttttttggt gttttgtttt    2160
tttgtaatgt ggggaagtag gagggtgtgg ggagaaatta ttaaaggatg tttgtagtga    2220
tttattgttt ttttttgttt tgaagaggat tttttaaaat gtagagggtg gtaggggata    2280
gaggtagtgt ttttttgaga gttgggatgt gttggagtta gtgaataaaa ggttattttt    2340
tgtgtggttt tgggtgtaga ttgtttttgt ttttagtttt attttttttt taatttttgg    2400
tatttagttt gtgtttttaa atgttgttgt agaggttttt aaatgtgtgg gttggtgttg    2460
ggaggagagt gtatttttgt ttttgtgtga gtttttttgtt ttttaaatta tttatttttt    2520
ggtgtatggg gttttttagt atttattggg atttagtagg ttatttggtt gtttgggtat    2580
tgtttgtttg ttaaattgtt tgtttgaggt ttgtgttgaa ttgtagttag agaaggtata    2640
agtgtatatg gtagtaaaag gaattttgtg gtatttgtgt tatgttgttt ttagtgtttt    2700
ttataggttg ttttgggttg gggaaggtgg tgttataatt tagagttttt tgttttgttt    2760
tgtggtgtgg atttggggtt tgtataaatt tttgtttttt gttagaaatt taattgttgt    2820
taggattatg tattttttttg gatgaatttg tggtgatgtg tgtagtaaaa gtgaaaaaag   2880
atgtttttgt tagaaaatgg tatttatttg tgtttggaag gggttttgat ttttttgtag    2940
tgggttttta tattttttaa tataaggggg aggaggaaaa taaatttatg gtttgtagaa    3000
tgtagggttt agtgagggtg taatttgtta ttttgataaa agaaattgtg tagtaatttg    3060
tgtttttttt gatgttagaa ggataatgga aataaaattt gagtttggtg tatggttggt    3120
tttgttgtta aatgttttat gttaatttgg tatttttgaa attttgtaaa atagttaatt    3180
ttggggtatt gatttggtgt ggattttttg gattttggtt tgattttagt tttaaaatgg    3240
tttttttttt gagagttatt tgatttttta gtatgttgtt gtaagtaatt ggaaattgga    3300
atagtttggt ttgaggtttt aattttgagg tttatttgtt ttttaaaaaa atgtggttaa    3360
taaatatttt ttatagaggg tttattgtgt gattggtagt aattaataag atttgtttta    3420
tttttatga agtttttgtt tttaggggga aatagattat ataaatgtag gtatatatat     3480
```

tttaggtatt tttagtgtaa tgaagaaata aaataggatt atgtgatggg ggaggatgtt 3540
tgaaatagag tgatttattg ggaggggtgg tgtg 3574

<210> 257
<211> 3506
<212> DNA
<213> Artificial Sequence

<220>
<223> chemically treated genomic DNA (Homo sapiens)

<400> 257

tagtggtaaa gttttgttta tgttaagtaa taaaggataa gttagttttt gttgtgatta 60
ttttgttgta ttgataagtt atgtattttt atttaaggat ttaaattttt attttttta 120
agaattgggt taaaattgat aaattaaatt tatttatggt ttattgatta aaggttgttg 180
tataataagt ttttgttatg tttagtagtt ggatttatag tgttagaaat ttataattgt 240
ttgatttttt tttttattat attgtgaaaa ttgtttttta aatgtaatta attttaaaat 300
tttaatagta ttgtggttag gtgtggtggt ttattattgt aatattaata ttaggtatag 360
gtgaggggat tgaggttagg atattgaaat tagtttggga aatatatgga gatttggttt 420
ttggaaaaat aattagtttt gtgtggtggt gggtgtgagg ttttggttaa ttgggaggtt 480
atagtgagtt atgatgatat tgtattatag tttgtgtgat ggtttatgtt agtaagtttt 540
ggagtatttg aaataagttg tgttgggtat tttatttatt ggagagtgat tagtgattga 600
tgtttattta tagtgattag agatgtatgt tttgatagta gtataaattt agtaggtgtg 660
aataaatggt aaagagaaat tgggtaaata agtattatgg ttttttagtt gagaaagtgg 720
gggttttaaa aagggttttt tgttgataga aagggatgtt taattattga aattgtagag 780
ggtgtggttt tggtgtttga gtgtgtagat tatatttatg gtggtgattg ttttgtgttt 840
ggtgtgtttt gtataggtta tggtgttttg gattatgttt tttaggaata tttttagtat 900
tttgtgagtt tttttgtaga tgaggttgga gatgtgtttt atgttgttgt ggtgagtaag 960
gtgttggatg gttggtttgg tgatgttttg gatattgttg tgtagtattt tatggtggtg 1020
tttagtgttg tttttgttaa gatttttttt gtttttgttg tggttagata tgatgagtaa 1080
gaggagtttt atttaatgtt ttgtgaggat tttggtttga ggtagtgttt ttatatgata 1140
gttggtggat tgaattgaga atttgaaaga agttggtggg aagttttgtt ttggtggggg 1200
aggggaaatt taaagggtta aattgaaata gggggaaaaa aaaagtgagt tttttgtttt 1260
tgtgttttga attttgtaat gtgtatagta ttttgttatt atgttatgag gttttaaaaa 1320
attgtttttg aatgtagaag atatatatta atattgtggg aaatataaga aaggataaga 1380
aattaagaaa ttataatgtt attttattat ataggttagt taattatgta ttttgtagag 1440
tagttgtata tattttttta agaaaatgta tatagtgttg tatatggagt tttgtaattt 1500
ttttatattg attataattt aattaatttt tattaaagag ataaaagtga tgttttggtg 1560
tttatgtttt ttaggaatta ttaatagtta taattagttt tttagtaatt ttttaattgg 1620
ttgtatttta aaaataatgt tttttatatt taatataaat gtattttttt tttatatttg 1680
ggattaatat tgaaatttat gattttatta tattaaaatt taaattttat tatattaata 1740
tttaaaattg tattagaggt tttatgattt ggtattatgg gtttttgtat tatttttttt 1800
ttaaattttt taatttgttt tattaaggtt tttggataat tttagagatt ttttgtgaag 1860
tttgaataaa attttttga gattttgata attgtattag ttttaggatt taattggaat 1920
agaattaaaa tttttaaaat aagtttttat aattagaaaa ttggtgtttg taggttttgt 1980
gtgtggggtt tttttttttt tttggaagga gttttgtttt gttgttaggt tggagggtag 2040
tggtgtaatt ttggtttatt gtaattttta ttttttgggt ttaagtgatt tttatgtttt 2100
aatttttgga ttagtgtgga ttataggtat gtgttattat gtttagttaa ttttttgtat 2160
ttttagtaaa gaataagttt tattatgttg gataggatgt ttttgatttt ttgatattgt 2220
gatttgtttg ttttagtttt ttaaagtgtt gggattatag gtgtgagttg ttttgtttgg 2280
atgtttgtag gtttttaaaa agatattttt atatgattta attaaagatt tgttattaat 2340
tattatggag taattttgat tttgtatatt tgattttttt ttttattaat aaatataggg 2400
ttatattttg aatttttttt tttttttttt tttttgtgga gatggagttt tgtttttttg 2460
tttaggttgg agtgtaatgg tttaattttg gtttattgta atttttattt tttgggttta 2520
agtgattttt ttattttagt tttttttgag tagttgggat tataggtatt tattattatg 2580
tttggttaat ttttttgtat ttttagtaga gatggggttt tattatgttg gttaggttag 2640
tttttaattt ttgattttgt gatttatatg ttttggtttt ttaatgtgtt gggattatag 2700
gtttgagtta ttgtgtttgg ttattttgaa attattttaa tattaatgaa aattataaaa 2760
tttttataat tattttaata taattttta gtatttaatt ttttgtttag aaagtagtta 2820

agagttgaaa ttttggaaat agaaaatttt ggttttagat taaatgttta ttttttagat 2880
ttgagttgat ttgttattga ttagatatga ggtgatttgt ttatttttt taaattttg 2940
tttatttata aagttagaat aggatagtga tggtattgtt ggggtgtaga atgatttttt 3000
aaaatgtggt ttttgggtat gttgagtgtt tttgaatatt gaaaggtttt agaaataagt 3060
tttagaatta aagtttttt aattttgttt ttttttttat atatgtgaag ggattttgat 3120
attttttaat tggattaaga aaattttta ttagaagtaa taattgtttt ttattttttt 3180
tttgttattt tattattgta gaaaagaata ttgaatatgg attttttaag ataatgtttg 3240
tttttggtt ttatttaaat tattaagata tattaggtgt tgtggttttt tttattaatt 3300
ttagtattgt gggaggttga ggtaggtgga ttttttgagt ttaggagttt gagattagtt 3360
tggttaatat ggtgaatttt tgtttttata aaatatataa aaaattagtt aggtggtgtt 3420
atatgtttgt aattttagtt atttgggagg ttgaggtagg agaattattt gaatttggga 3480
ggtggaggtt gtagtgagtt gagatt 3506

<210> 258
<211> 3506
<212> DNA
<213> Artificial Sequence

<220>
<223> chemically treated genomic DNA (Homo sapiens)

<400> 258

aattttggtt tattgtaatt tttgtttttt aggtttaagt gatttttttg ttttagtttt 60
ttaagtagtt gggattatag gtatgtgata ttatttggtt aattttttgt atattttgta 120
gagataggga tttgttatgt tggttaggtt ggttttgaat ttttgagttt aagggattta 180
tttgttttga ttttttatag tgttgggatt agtgggagga gttatagtat ttagtatgtt 240
ttggtaattt aaatgagatt gagaggtaga tattattttg aaaaatttat atttagtatt 300
ttttttgta ataatgaaat aatagggagg ggatagaagg taattgttat ttttggtaag 360
aaattttttt ggtttgatta ggaaatgtta gagtttttt gtatgtatgg gaaagaagat 420
gaagttagag gggttttgat tttgaagttt atttttgagg ttttttaatg tttaaaagtg 480
tttagtatgt ttaagaatta tattttgggg aattattttg tattttaata gtattgttat 540
tattttattt taattttgta gatgagtaaa gatttaaaaa gaataaataa gttattttgt 600
gtttgattgg taataagtta gtttgagttt gaaaggtaaa tatttaattt aaaattaaag 660
tttttattt ttagagtttt aatttttaat tattttttaa ataaagaatt gaatattaag 720
agattatatt agaatagtta taggagtttt ataattttta ttgatgttaa aataatttta 780
gagtggttga gtgtagtggt ttaagtttgt aattttagta tattgggagg ttgaggtata 840
tggattatga ggttgggagt tggagattag tttggttaat atggtgaaat tttgttttta 900
ttaaagatat aaaaaaatta gttgggtgtg gtggtgagtg tttgtaattt tagttattta 960
ggggaggttg aggtgggaga attgtttgaa tttgggaggt ggaggttgta gtgagttaag 1020
attgggttat tgtattttag tttgagtgaa agggtgagat tttgttttta taaaaaaaaa 1080
aaaaaaaaaa aaaatttaga gtataatttt gtatttatta ataagaaaga aaattaaata 1140
tatggaatta aagttgtttt ataatggttg ataataaatt tttagttggg ttatataaaa 1200
gtgttttttt aaaaatttat aagtgtttaa gtgaagtggt ttatgtttgt aattttagta 1260
ttttgggagg ttgaggtggg tggattatga tgttaagaga ttaagagtat tttgtttaat 1320
atggtgaaat ttgtttttta ttaaaaatat aaaaaattag ttgggtgtgg tggtgtatgt 1380
ttgtagtttg tgttaatttg gaggttgagg tatgagaatt gtttgaattt gggaggtgga 1440
ggttgtagtg agttgagatt gtattattgt tttttggttt gatgatagag tgagattttt 1500
tttaaaaaaa aaaaaaaatt ttatatataa aatttataaa tattaatttt ttagttataa 1560
gagtttgttt taaggatttt aattttattt taattaagtt ttaaagttaa tgtaattatt 1620
aaaattttga agagatttta tttaaatttt ataaaaggtt tttaaagttg tttagaaatt 1680
ttggtgaaat agattaggaa atttggaaag gaaataatgt ggagatttgt agtattaaat 1740
tatgagattt ttaatataat tttaaatatt aatgtaataa aatttaaatt ttggtgtaat 1800
aaaattataa attttaatat tggttttaag tatagagaaa aagtatattt atgttgaatg 1860
tggaaaatat tatttttaaa atatagttga ttaaaaaatt gttggggaat tgattataat 1920
tattgataat ttttaagaaa tatagatatt aaaatattat ttttattttt ttaatagaaa 1980
ttggttaaat tataattaat ataaggaggt tataaaattt tatatataat attgtatata 2040
ttttttgga aaaatatgtg taattgtttt gtaaaatata tgattaatta gtttgtgtga 2100
tgggataata ttgtagtttt ttaatttttt gtttttttt gtatttttta tagtattgat 2160
gtatattttt tgtgtttaaa agtaattttt taaagtttta taatgtggta ataaaatatt 2220
atgtatgtta taaaatttag aatatggaaa taagaagttt gtttttttt ttttttatt 2280

```
ttggtttggt tttttagatt tttttttttt tattggggtg ggattttttg ttgatttttt    2340
ttaggttttt agtttggttt gttaattgtt gtataaaggt gttgttttag gttagagttt    2400
ttataaagtg ttgggtgaga ttttttttgt ttgttatgtt tggttgtggt aaaggtggga    2460
agggttttgg taaaggtggt gttaagtgtt attgtaaagt attgtgtgat aatatttagg    2520
gtattattaa gttggttatt tggtgttttg tttgttgtgg tggtgtgaag tgtatttttg    2580
gttttattta tgaggagatt tgtggggtgt tgaaggtgtt tttggagaat gtgatttggg    2640
atgttgtgat ttatatagag tatgttaagt gtaagatggt tattgttatg gatgtggttt    2700
atgtgtttaa gtgttagggt tgtatttttt atggttttgg tggttgagtg ttttttttta    2760
ttaataaaag gtttttttta gggtttttat tttttagtt gaggagttgt gatgtttgtt    2820
tgtttagttt tttttatta tttgtttgtg tttgttgagt ttgtgttgtt attggagtat    2880
gtgtttttag ttgttgtaag taggtattag ttattaattg tttttagta aataaaatat    2940
ttaatataat ttgttttagg tgttttagag tttattgata tgggttgttg tgtagattgt    3000
agtgtagtgt tattatggtt tattgtagtt ttttgattag ttggaatttt gtgtttatta    3060
ttatgtaagg ttaattattt ttttaaagat tgggtttttg tgtgtttttt aggttagttt    3120
tgatattttg gttttaattt ttttgtttat gtttaatgtt ggtattatag tagtgagtta    3180
ttatgtttgg ttatgatatt gttgaggttt tagggttagt tatatttaag gggtaatttt    3240
tgtagtgtag tggggaggaa agttaagtag ttataggttt ttggtgttgt gaatttaatt    3300
gttgaatata gtaagaattt attatgtaat aatttttaat tagtggattg taaataagtt    3360
tagtttattg gttttggttt aatttttgag aaaggtgaga atttgaattt ttgagtagaa    3420
atatgtagtt tattagtata ataaagtgat tataataaag attaatttat tttttgttat    3480
ttaatgtggg tagagtttta ttgttg                                         3506
```

<210> 259
<211> 22489
<212> DNA
<213> Artificial Sequence

<220>
<223> chemically treated genomic DNA (Homo sapiens)

<400> 259

```
ggttgttggg agatattgtg gttagtgggg tggggggttt gggtatttgt ttttagattt      60
ttggttattt taagtgatag gaaggggggt tagtgaggat atgaatagtg gttgttttta     120
tttttaaggg ttggttttat tgttttttta tttttgtttg gtttagtagt tttgggagta     180
gttattttgt agtttatgtg tgtggggatt ttgttgtgtt ttgagttgtt gaagtagttt     240
tttattgggt gttgtgttgg gtttttgttt ttattttgta gggaggaagt atagttgggg     300
gagtggtttt ttgagggttt ttgggagttt agattttatt tttggaggta gttttgggga     360
ggtttggtgg agattttgtt tttttgttga agttgttgtt gaagagtgat aggtttgttt     420
agtgttgggg gagatttggg atttttagag ggagtgtagg atagggtagg gtagggtagg     480
gttatgattt ttggtgatgg ttaggttgtt atgggttgaa ttgttatttt tgggtttgtg     540
ggtaggtgtt tagtttttta ggttttattt tttttattta aggaatggga tatgtatatt     600
tttgttttta gggtggtgta gagaatagga tggtgttttt gagtagtgtt tttaatgttg     660
tttggggttt gggaatgtat taggatttga taaataaata ttagttttta atattttgtt     720
ttttgtaagg agaaggttag gaatgttttg tttagagaga aggtgagttt atgagtagta     780
gatagttggt gtttaatagg tgtttgtttt tttttagagt tttagggtgt tagttggtta     840
gagtgagttt tgttgattag ggagttgtgg tagttggttg ggggttggtt tttggggtag     900
gtgggatgtt tgtggggttg gtgtaggttt aggttttgtt tgggagtgtt ttgttttgag     960
tattggtttg gatatttgag ttgagtttta gataggtgtt ttggggttga tttggtagga    1020
gtagttgtta tttttgggta gttgagttta gtagtgaggg ttggtagaat gatttagatg    1080
aaatattgaa tttaatagta aaattatttt ttttgaggga aaatatgatt tgtagttagt    1140
tggtgtgtat atatggggtt tggaaaagaa gagaattttg gagatataaa tggttttatg    1200
tgttgttgtt ttggtttggt tgttaagtta ttaaattttt gtatagggaa aagtgataaa    1260
gtaaatattt attttttttg ttgtttttag aagtgaaagg tttttgagat gggaaatgta    1320
gatggtttgt agagtgtttt atttaaataa aattattaga tgtttgaaga ttttgggaga    1380
aatgggttta gatgaattat ttgtgtaaag ttatttagta ttttgtaggg gttttgaagt    1440
ttggtgttgg taaggtgttt gttgttggag taggggtggt tgatggattt ggtgtggggt    1500

gggtgggagg agttgtagat ttagatgtag gaggtttggt ggggttattg atttatgttt    1560
ggtttggggt tttgtttttg gggttgggag gggattaggt tggggaagtt ggggaggggg    1620
tttttttttt ttttttttta gttgttaggt tgttagtggg agatataggt tttggggatt    1680
```

aggttttgta atttttttttg attttgatgt ttgagattgg tttggtgttg gttttgaggt 1740
ttttgtgtaa ttttagtttg ggttggttgt ggaggatttt tgagggtttt ttggtaaggt 1800
tttttttttt ttagttggtt atggtatata ggattttatt tttttgtatg tttagggtag 1860
gtattgttaa ttgagttttg tattttttttt aattttttgt attagggttt attaggttta 1920
tttatttttg tgtttagatt atttggttga tgggtggttt tgttttagga ggaatagggg 1980
ttttttttgtt tttttagagt ttttgttttg ggttttgtat ttatgttttg gatgtgttgt 2040
tttttttata tttgggagtt atgttgattt tttttgtgtt tttttgggga gatatttttt 2100
tgtattagag attggttttt tggggtttgg gtgtgggggt ggggatttgg atgttggggg 2160
tgtagaggtt atggttttgt tattttttttt gattttgtta tgagagtatg attagggttt 2220
gttggttttt tgggttttga tgtttttggg tttggtattt ttttggaagt tttatttatt 2280
tttttttttt tttttttta taaggatttt tttattgtaa ttttgtatga ttttatttta 2340
gttgattaaa tttgtaaaaa tggttaggtt tggtggttta tgtttgtaat tttagtattt 2400
tgggaggttg aggtaggtag attatttgag gttaggagtt taagattagt ttggttaata 2460
tggtgaaatt ttgtttttat taaaaatata aaaaatagtt gggtgtggtg gtaggtgttt 2520
ataattttag ttatttagga ggttgaggta ggagaattag ttgaatttgg gaggtggagg 2580
ttgtaatgag ttgagataat gttattgtat tttagttagg gtaatagagt aagattttat 2640
tttaataaat aaataaataa atttgtaaag atgttatttt tgtgtaaggt tatatgttga 2700
ggttttgggt ggatatgaat ttgggggata ttgtttagtt taggatagga attaattgta 2760
gtagggtagt tgttttgggt atatagtttg attggtattt atttttggta ggttttttgg 2820
agtttggttt ttttattatg gggtgggggt tttagttatt tttttggggg agtttttaat 2880
ttgaggtttt ttgtgttggg gtttagttag ggtgggggtt gtattttatt tttggagtag 2940
gtatgttggg ttttgatagt aagttgggga gggttttagg gttagaggag gatttagggg 3000
ttgaaggtag tgatgggttt agtaggtagt agggagggta gggtagggga ggggtgttaa 3060
ggtttataag tgagaggagg taggagggag gatatggtat ttatagttgt ggtggaggag 3120
ggagggtgtt tataaggtgg gtttttagtt tggagtttta ggtatttttt gttgtggtta 3180
ttgattgttg gggaattgat tagttttata gtatggtatg gagttgggtt aggtagggtt 3240
ggatgtttgt taggggtatt gtaggtgggt tattaggggt tggtttgggg tttagttgtt 3300
atttagttga tatgggatta ttaatggttg ttatggttta ggtatttttt ttttgttatg 3360
ttggggatat tgtgatatag gtttttaggt tagtgaggat agtgtgggtg ttggagtttg 3420
gagggagggg agtggtattg gggaggtttt tgttgagttt ggatagtggg gtaggtggaa 3480
gggtgtgtgt ttggttgtgt attttgggtt tggtgagtgt ggttgagggg tatagttgtt 3540
tttggttggg gttgtagtga gtgtttagtt tatttggtta taggttggtt attagggatt 3600
tggtgtttgt tatttatagg attgaggatt ttaatagtag ttttagttta ggttttgaaa 3660
agttgagtag agaagggtag gaggtttttg tatttttggg atttattttt ggtaggttgg 3720
gttgggttgt gggggatata tttgttttgg gggatgtggg agggggggtgt ttttgggttt 3780
gtagttgttg gttttgagga tagatgtagt ttttttagta atagttgggt tggaggtttg 3840
ggagttaaga gagattagag tttaggttag tttggttttt attagggatt agttattttg 3900
gttatagatg ttagtagggg ggatttttag ggggtgggta ttaggttggg gtgggtagtt 3960
ggggtgggtt ggggttgtag tgttttttta tttagttttg ggtataggat gagttagttt 4020
ttatttttgt ttatttagta ttttgtaggg gttgttgttt tgttgagtgt aggatgttga 4080
ttttaggatg ttattatttg atgttgttta ggtttttttt ttagaatatt tgtttagtga 4140
ttatagttat tttagttttt tttattgatt ttaggtagag taaggggttt tatttgtatt 4200
tagtaggtgt agattgggta gggtagggtt ttaggagggt gtatgtttgg ggagtttttg 4260
tatgtagata gtggggggtt agggaggagt tatgtgttgg gtataaggta gtttttgtag 4320
gtttttttgtt gggtttgttt tagtatggag ggattggtag tatatagtag gtgtttagta 4380
aatgttgggg ggtggggata agtgagtatt tatgggggga agtattggtt gtggttttgt 4440
tatttgtttt ggtagtattt ttatttaggt tttggatggg tggtgattat tgtttttttg 4500
tttttggggt tttgattttt agtatttggt agttggggat ttggtttttt ttaaggtttg 4560
ggagtagtta agtggttagg tattaagtgg taaatttagg ttagggttgg gtgatggttt 4620
ggagggtttt tgtggtagtt gagttttgtt ttttttggtt ttgtttattt ttttggtgtt 4680
gtttattttt ttggtttgta tatttttttg gtttgtatat ttttttggtt tgtatatttt 4740
tttggtttat ttaggttgtt attggtttga gggttttagt gaggatgagt tggagatgga 4800
tattgagggg gttgatgggt ttggggtagg ttggttgggg tagggggggtt gggtttgttt 4860
agtggttgtt tgtttttaga agaatgatat tttgtttttt tttttgtttg aggaggtggg 4920
gtgagggtta gattttagta tttatgtttt tgttttttttt ttttttgaaa agtttataga 4980
tttttatttg aattgtttta gtagagttta ggtggggtat tatttttatt tatatatata 5040
aagggttgag atagtgtagt ttggtgtgtg agtagttttg gtgtttttag gttggggtgt 5100
tgtttgtttg tttatttggt tatggttttt aatagttggt ttagtaaagg ataggtagat 5160
tttatgggtt aaggatgtgt tggtttggtt ggtgtggggt taggtgggta gtttgttagg 5220
atttggggat tttgagttag ttgttgtagt aggtgtggtt ggtttagtta ggtaagtttt 5280
gttgttggga gggttgttta ttagttgttg gagggtgtgg ggtggttggg ttggttattt 5340

gttggtagta aagttagtag agagtgggtg gggttggggg aggtgggagg gttttagttt 5400
tatttttagg gtttagtttt tttgggtttt gtagttaggt taggaaggtt agttgggttg 5460
gttttgggtt ttaggtttta gtttggaaat taggggtttt aattttggta tgagaaagtt 5520
tttgttatag ggtaggagga gttttatata gaatttttaa tttttgtttt tgggtttgag 5580
tttggtagga ttggatgttg gatattggtg ttgttgggag ttttgagaat tgtatgtgtg 5640
ttttttagtg gttagtgttg gttgtgtttt gtttgatttt ttaggttttg atgtggggtt 5700
ttggggttta gggagttgag gtttggggtg ggggttgtat ggtggtgtgg tttttttaga 5760
ttgtaggatt agagggtagt tttgaaagtt tttttggtta gggagttggt ttgtagagtt 5820
gaaggggagg agggggttag gaggttgttt gtggttgtgg ttttgaaaat taggttttgt 5880
taagggtttt tgagggttag ttggggaggg aagggtatat agtgtgttta gagtaggatt 5940
ttagggtgtt tgggtttagt gtttttgggg ataaagggag atggagtaga ggggttttag 6000
ggaggtttgt tttgtggtgg tataagagtt attgtgaata ttttgttttg gtgaggttgt 6060
gttttaggtg ttaatagtta tattttagta tattaggtgt aagttatttt ttataggttt 6120
tagtttgttg taggaatttt aagttttagg ttgggaagga atgggtaggg ttgttgtagg 6180
gattgtggtt ttgtaggatg agggtttgtt aggggtgttt aggggatagt gggttgggat 6240
gtgttggggt tgttgtaggg attgtggttt tgtggggtga gggttggttg gggtgttggg 6300
gttgtttttg ggtggttagg ttttatttga gagtttggtt atgggattta aggttagagg 6360
gtggttgagt tgagggttgt ttgttttggg ggggtttttg gttttaaatt tatttatttg 6420
gggggtggta ttgaggtagg tttttaaggg tagaaagggg agtgatttgg taaaggtagt 6480
tttgtgttgt tttttttttt ttgtttatgt tatgtatatt ttaatgaaat aagaaatttt 6540
ttttgatttt tttgtgggtt tggtgatagt ggtattttgt ttagtttaga gtttttaatt 6600
ttttgaggta gtaggagtat gtaggtgggt gggtgtagga gttggagtga atatttttgg 6660
gtatagtagg agtagaattt tgtgtagttt tgtggtagtg tttagggtag tgtttgtgat 6720
ttgtgaagtt ttagagggtg tgtgttatag tgtttttagt tttgttgttt aaggatgttt 6780
ggagtgttta atagtttagt ggattttttg tttttttttg agggtagagg gttagtgtga 6840
tagttttttg tattttaagt ttttgtttag tatttaggaa atattaggtt gtatgaagaa 6900
attgaaggat agtaaatgta agggatttta tggttgatgg agggaggggg atttggaaag 6960
gggggtggag ggggaaggtg atggaagagg tttttagagg gagggagagt tggagagggg 7020
gtggaggggg aaggtgatgg aagaggtttt tagagggagg gggagttgga gagggggtgg 7080
agggggaagg tgatggaaga ggttgttaga gggaggggag ttggagaggg ggtggagggg 7140
gaaggtgatg gatgaggttt ttatagggag tgggagttgg agagggggtg gagggggaag 7200
gtggtggaag aggtttttag tgggaggggg agttggagag gggtggaggg gggaaggtga 7260
tagtttttg aagttttgtt gtttttatta gatttttttt taaagttttg ttgtttagtt 7320
gttttttga ggttatgttg tttttttttg atgttaaatt gttattttg atggttagtt 7380
gttttttttt tttttgtttg ttttgttttt tgttaataag gttaggagtt tttatgggta 7440
taggatgggg gtggggtgag gtagtggtgg ttttggaaaa ggtagtattg gagtagggaa 7500
attggaatgt gtgtttttat ttagggttat gtttttaggt ttgagggtgg ggttttttag 7560
gaattttatt tttttttatt tagaattttt gtttttttgtt tttattattg agggtttgtg 7620
aggttgatag tatttatttt taaagaagga ttggtttggg tagggttgtt aggagtatta 7680
gggtagggtt tgttaattta gaggtttttg aggaatagat ttaggtttgg gaatgggttt 7740
atggtagggt ttttttggaa atgtttggta gagtgggtag gggatgttat tgtgattttg 7800
tattgttggg gatattaggt ttagaggggt tgattgatta gttaaggttg tataatagag 7860
ttgttgggag tgggtttttg ttaggttgtg ggatataggg attttggttt ttgttgtttt 7920
tgttttagtt ttagtttagg atttattgtg gttgttattg agttttagtt ttagagttgt 7980
ttattaagtt tgattttatg tagggtttat tgtgtttatt agagggtgtt agaagttggt 8040
ggttttgata ttgtggttta aatgtaggga ttgtttggtg ggggttgttg taattttttt 8100
tttggtgtgt ttaggttttt ttttgagggt ttttagggtt ggggtttggt tggggagaga 8160
attaggtgtg gtggagttgg ggagtttggt gttgggtagt gggtggtttg tgtttgggga 8220
ttttggtgtt atttgttagg tggtagggag gttggttttt tttaggagtt tttatttttt 8280
gaggtggttt taagttttg ttgttgtttt ttttgtttta tgttttattt ttttgtttaa 8340
tttttttgat agggtttggg tagggatgaa gtagggtttt tttattttgg agttgggagg 8400
aggggggtgg gggtgttatt aagtatgggg tttaggtaga tttagtttat gggggatatg 8460
ggggttttag ggtgaggatt tttgggggt tggttggggg gtgtttagag tttatggaga 8520
gtggtttggt gggggaggtt ttgttagtag atttttttt tttttatttg gagttttggt 8580
ttttattagt ttttattttt tgtttagtgt ttggtttttg tttgtttttg gttaatgttg 8640
ttatttgttt ttttagttaa agattttatg tttatgtgat atgattatga taggagttgt 8700
ggggggtttt agggaggttg gtgttgggtt tgaatttgtt ttgtgaaggt tttgagtttt 8760
tggttgttgt attttgattg ttttgtgtga agggaaggga gttatagtta tgttataaga 8820
gaaggggttg ttgttttttt aatttttgtt tttttgtttg ttttaatttg tttgggattt 8880
tttagagttg gttgggggtt ttagtttagt tatttaagtt gtattttgtt ttaggaaatt 8940
tttatgggtt ttgattagtg tttattaggg tttttaggat attttggggg tgggaagtgg 9000

```
gggtaggttt tatgtttgta ttttatttgt gagtttttgg agttttttttg tatattttat     9060
agggttggag gggttttgtg ttttagatgg agttttgttt tgttgtttgg gttggagtgt     9120
agtggtgtga ttttggttta ttgtaagttt tgttttttgg gtttaaggga ttttttttgtt    9180
ttagtttttt aagtagttgg gattataggt atgtgttatt atgtttggtt aatttttgtg     9240
tttttagtag agatggggtt ttattatgtt ggttaggttg gttttgaatt tttgatttaa     9300
gtaatttgtt tattttagtt ttttagagtg ttgggattat aggtgtgagt tattatatta     9360
ggtatgtttt tgagataagg ttttgttttg tttttttatat tggagtgtag tggtgttatt    9420
atgttttaag gtaattttaa atttttgggt ttaagtgatt tttttatttt agttttttta     9480
gtagttggga ttgtaggtgt gtgttattat gttttataag ttttttaatt ttttgtagag     9540
attggggttt tgttatgttg tttaggttgg ttttttgaat ttttgatttt aagtgatttt     9600
tttattttgg ttttatagaa agtgttggga ttataggtgt gagttattgt gtttagttta     9660
gggttagagg ttttttttttg ttttttttttt aatgtgtagg tgtttttttga tgttttttat  9720
tttttttgtt gggtttgggt tttgatttat tttttttgatg ttttgagtag ggattggaga    9780
gtgtttttaa tgtgaaaata ttggtttttt tgtagtttag ttttagtttt ttatttgtta     9840
ttatatgggg aggtattatt tgggtgattt ttttagggtt gttttttaat ttttatattt     9900
tttttttggtt atatttaatt tgttattgaa gttattagtt gaggtttttta tattaatagt   9960
tatatttttt gtgttaaagg ttttgtgtgg ttgttttttt aatttgttgg gtttttttta     10020
tgttattata ttgtttttta tttttatttt tattttttta gttattttaa atttatgtat     10080
aatttttttt tttttttttt gatagatttt tattttgtgg tttaggttgg agtgtagtgg     10140
tgtgattttg gtttattgta atttttattt tttaggttta agttattttt ttgttttagt     10200
tttttaagta gttgggatta taggtgttta ttattatgtt ttgttaattt tttgtatttt     10260
tagtagatat ggggttttat tttgttggtt aggttggttt taaattttttg attttaagtg    10320
atttgttttt tttagttttt taaagtgtta ggattatagg tgtgtgttat tatgtttggt     10380
ttataatttt ttaattgttt tattattatt tttggttttt aagatattat tgattgggtg     10440
tagtggttta tatttgtaat tttagaattt tgggagattg aggtaggtgg attgtttgag     10500
tttaggagtt ggagatagtt taggtaagtt ggtgagattt tttttataaa aaaatagaaa     10560
atgtttattg gatttggtgg tattgtttat agttttagtt atttgggagg ttgaggtagg     10620
aggataattt gagtttaggg gtttaaggtt gtagtgagtt atggttgtgt tattgtattt     10680
tagtttgggt gatagagtaa gattttattt ttttaaaaaa aaaatggaat attaaaaatt     10740
agaagatggt gttgtgtttg ttagtttttt ggggtgtgag tttattattg tgggttattt     10800
tttttatgga gtttaagttt tagggggata tatttttatg gagggtgtat gtttgttttt     10860
gttggagttt gggtggtttt taggttttgg aattagattt tatattttttt attttgttta    10920
ttggttgtta gtggtgtttt atttgtgttt tggtgataga ggtagtgttt tgggggtata     10980
ggtattggtg ttagttttga gtttgaattt tagtttggtt ttagttagtt gtatggttga     11040
gggtgggtgg gggatgtttt tgttttgatt ttttatattt ttatttatag tgtagtttta     11100
ttttttttat aggagttgga gggttatatg ggaaaatagg tatggtagat gtaaaaggtt     11160
tagtggaaat gttggttttt tttgttgtta ttattgtatt tatttggtaa ttttgtatta     11220
taatttgaga tttagaagat tttatgtttt aggtggggtt gaataggata gattttgaag     11280
agttggtttt tagattggat tgttaataat taggttggat tttgtgtttt tagttggtta     11340
gtgttttggt aaggtaggag tgggtgaaat ttttagtttt tttagggagt agttagatga     11400
tgatatattt atatttgttt atagggaggg agtgtgataa ggtttgtggt tttttgtgtg     11460
agaattggtt agtgtttttt ggggtttggg atggggtttt agtatttatt tttattattg     11520
gggtttggga gggagggttg gagagttttt atttggtgtt gttggtggtt atttttttaga    11580
tagtgtgggt gggtttgtag gttttgggta aatgtggtgt tgttatagtt ttgttttata     11640
gggatagtgg ttagtagttg tttttggttt tgttgttttt aatgaagtta tagatggtag     11700
tttttaaatg tttttagaaa tggtagagag gtttttagag ttgatgtgta aataaattgt     11760
tttttaggtt tggattatat aattttgttg ggattaagta taggtttggg ttttttggtt     11820
gtttttttagg gtttggtttt gttgagagga ggaagtttgg tttttgggag ggggttttag    11880
gtgttttttt tatggagttt agtaatttgg taggtggggg tgggtattgt ttttttttttg    11940
ggattttatt tttttggtaa ttagttgttt tttttttttt tggggtttta ggtggttttt     12000
atggaggggt gggtgggatt gaggtttgag ttttggggag gagttggttg gtgagttatg     12060
tattttagtg gttattgtgg ttagttgggg aggttgaatt ttaggtttta gtatttagaa     12120
gttggtggag tgggttgaat tgagtttttt taaaaatgta tgttgaagtt ttgattttgg     12180
gtatttgtga gtattggaaa tggggttttt gtgaatgatt gagttaagat gaggttggat     12240
ggggagggtt ttaaaagtaa tgattggtat tttgtaagga ggttgttgat agtagggaga     12300
agttgggtgt gtgatgttag aggtagagat tggagagagg tagtttagga atattatggg     12360
tggttggtag tgtggagtgg tagggttttt ttttggaggt tttggaggga gtgtggtttt     12420
gtggatattt tgattttagg tttgtggttt tagggttgga ggggttgtgt ttttgtggtt     12480
ttagttttta gtttgtggtt ttttgttatg gtagttttgg gagatttata taggtggtga     12540
ttgtggatag gatttttgat ttttgggtta tagttttttt attggttttt gttatggggt     12600
aaataggttt ggggtggtgt ttaggttggg gaattgtttt agttgtttgt tgtttttgtt     12660
```

gtggttgatg gggttgtttt ggttgtagtt ttggttttgg ttttttgagg ttttgtattt 12720
tttttggtat ttattggttg ggtttttggg gtttttggtt attttgtggg tgttgattga 12780
attgggataa atgttgtgtg tagttaaggg gtgatttaag gttggttagg tttagagttg 12840
attttaggtt gggaatagta gtagtgatgg tgttggtatt tattgtgttt tttttagggt 12900
ttggttgtgg gagttgaggg gatgtgtttg tggtttttgt ggtttgagtt tggaggttag 12960
tgtttagtgg tgtggtattt gggtattttt gttatttgtt tgtagttagt gtgtttttgt 13020
ggggggtagt gaggtgattg ggtttttgtt gggtgttttt ggaaggttta gaggtaagga 13080
tttttaggta gagagtttgg gggtggtttt aggaaatatt tgttattgag ggggtgttgt 13140
gaagggaagg ggtgggtgtt aatgggtagt agtgagtagg ttattgttgt gggtagattt 13200
taggaggtag tattttggag ttgtttttat tatagggaag tggttaggtt gtagtggttg 13260
tattttaggg tatggattta ttatattttt ggttgttttt atgtttaggt taggtttttt 13320
gtggttagag atggttagag ttataggggt tgtaggaagt ggttgtaggt atggttaggg 13380
gtaggtggta tttgttttga gggtttgtat gtggtttttt tgttttgtta gtttttgtta 13440
tgtgggtttt gggaaattgg tgtttaattt tatgagtagt tttgggtttt ggggttttag 13500
attttttaat gttgttgtgt ttgtaggagg tgttttgggg ttttagattt tttaatgttg 13560
ttgtgtttgt gggaggtgtt gtgggggttt tggatttttt aatgttgttg tgtttgtggg 13620
aggtgttgtg gggggttttag atttttttaat gttgttgtgg ttatgggagg tgttgtgggt 13680
agggttgatt ttggggttta gttggttttt ttttgtttaa tgttagagaa gggaagtgtt 13740
gagtgtttga gggggattag taggggaagg gggtatagtt aggtttatgg ggtgattagg 13800
agtagagatt ttattttatt aaagtatata tattggttgt gtttgagggg aaaggagttt 13860
agggaggtgg atgaggtggg tagggggtgt ttgttgttgg tttggatgtt tagggggggta 13920
ttttattgtt tttggggttg gtaggtttta gattttttaa gtttatggtg ttaaatgtga 13980
ttttttatat tgggtggtag tggttataat tgtgaggggt aggtagtggt tgtttggatg 14040
gggatgtgtt tgttttttga tgaggagttt gtgttttatt taagtttttt atttgtattt 14100
ttggtggggt atttgtggtt tagtttggta gggattttga gtttgggttt gtgggggtgt 14160
ggtttttggg ggagggtaaa ttttggtggg tattggttat tggtgagttg gtgttgtggt 14220
tatagttttt atatttttag ttgttgtagg tttttgtttt tgtatgttat ttagtgttat 14280
gtttttgtat ttatgtagag ttatgtttta tttgtgtgtt tatgttgatt tattattgat 14340
tttttttgag tgaggagttt ttgttaaatg ttattaggtt tttagttttt agatgaaatg 14400
aaatggttga tgaattatta tattttaata tattaggaaa gatggattta tttgtatttg 14460
atgagtaaaa tgtatttttt tattgattga aaagttgatg aggtaaataa attagggaaa 14520
atggtttttt ttttattttt ttttgagtag taaattaagt gatttaaaag agttttgaat 14580
gagttaataa taaaatgttt ttaaatgtgg aaatgtgaag tgtaaatatt ttagttttat 14640
tgttagtgtt tattgaggat atagtttgtt ttttgttttg ggaagttggg ggttgtggag 14700
gattttggtt atttggatat ttagttatat tttaaagttt ttaaggggag atggggtttt 14760
tagtaagttt gtttggatta gttaagatga ggggttttag gagttaatat agatggagtt 14820
gttaggtgtg gtggtttaag tttgttattt taatattttg ggaggttaag gtgagaggat 14880
tatttgagtt taagagttta aggttagttt gggttatata gggagatttt ttttattttt 14940
aaaaagattt ttaaaaatta gttaggtttg atggtatttg tagttttagt tatttgggag 15000
gttgaggtgg gaggattgtt tgagtttagg tattagaggt tttagtgagt tatgattata 15060
ttattgtatt ttagtgatag gtgagatttt gtttaaaaaa tattttataa taagagagag 15120
agagggaggg aggagtttgt attggttgaa ttattttagt gttttggggt tgttgtagtg 15180
taggtttata atattagaaa tttatttttt tttagttttg gatgttatga gtttaaaatt 15240
atggtgtggg tagtatgtag tatatttttt ttggaggttt tggggaggat tttttttttt 15300
tttagttttg ggggttttat gtttttgttg tggttgatgt tttagttttt gtttttgttt 15360
ttatagggtt tgttttgtgt atttgttttt attttgtttt gtttttttatg gaggaatatt 15420
agtgattgat ttgggattta gttttaattt agggtgattt tgtttttaga tttttaatta 15480
tatttgtaaa gttttattat tagggaaggt tgtattttga ggttttgggt ggatatgaat 15540
tttgggggta ggatattgtt tagtttagtg ttgggtgttg ttgtgggtta ggttttttga 15600
tttttgtaag tttatgatgg ttatagagtt ttaaggagga ggttttttgg aggaggtggt 15660
ttagtttatt agagaattag tatgtttttt ttttgggttt tgggattagt atttttaatg 15720
ataagttttt ttggattatg ttgatttggg ttgagatttg ttgtttgttg tgtgttttgg 15780
gtggaggtta gataggttgt gtatttttaa gttttgatat gttttttttt taaagggtgg 15840
aggttttata gggtgagtgt aggttgggga tgggttgtgg ggttgttggg aggggattag 15900
tgggggtttg tagtgttttt gggagggttt ggggttgggg gtaattgaga tttgttttgg 15960
tgggtgagtg gagaagtatt tgggggggttt tttttagtgt tggagagaaa tgggtgatga 16020
agtttgggaa aggtgtgggg ggtttgaagt gtgtattatg gtgagaaggt tgtgtgtggt 16080
tggattttag tttttttgtg tttgtgaaag gttgagttgt gttggttggg ggagggttgg 16140
aggttgttgg gggttggggg atgaagagtt gggggtagtt tagggtagtg tattttttgt 16200
ggaggtagga atggtaggtg gtgtgatttt tattaggatt tttgttattg gaattgtggg 16260
tttttgttgg gttggaggtg tttgagagat ttttgtggaa tggtgtgtga atttgtggtt 16320

gtggtggtgg gtttggatgg agatggatgt tggatgggtt gtttttgag tttggttttt 16380
gtggtggggt tggtttttg tttttgtatt tggtagattt gatgtggagt ttgagggtgg 16440
ttgttggttt ggggtgggga aggggttttt tagattgggg gttttatttg ttgggtttgg 16500
ggagtttatt tggtttttt tgattggttt ggagttgtag gtgaggttat tttaggaagt 16560
tgttgtattt gagggagttt gggttgtttt gggggatgtt ggggtttgat ttttggggtt 16620
gggggttgta tgggtgggtt ggggtttgtt tgggttgggg tttgtggttg tgtggggttt 16680
ggattggtgt ttgtttggtt tgggggtttt tagttgtatg tagtttgggt tgaggttggg 16740
ggtttgtggt tgtgtggggt ttgggttggg gtttgtttgg tttgggggtt tttagttgta 16800
tgtagtttgg gttgaggttt gtagttgtgt ggggtttggt ttttatggtt tttgtgtttg 16860

gattttgttt tttaattatt ttttttgtat gtttttttgg aaaaggatat ggggtttagg 16920
gtggtggatg tggatatttt ttgaaattta ttagttttgg tttgtgatgt ttggatggag 16980
gggttatagt tatgttggtt tttagttttt ttttagatat tttagttttt ggaggtaggg 17040
tttttttgtg aggtgtatta tttttgagtg ttttatatgt ttgtttttgg gtattttttt 17100
gggtggtggt gtggtgtggt tttgtgtttt tatttaaatt ttatatggaa atggagtttg 17160
tagtgtgatg gtggggtttg gtggggagtg attgggtagt aggggtggat tttttgtgaa 17220
tggtttggtt ttgttttttt tggtgttgtt tttgagattg tgagtggtta tttaattgtg 17280
ttttgtattt gttttgtttt ttttgttttt gtttatgtta tgtgagatat ttgtttttgt 17340
tttgtttttt attatggttg gaagtttttt ggggtttttt tagaagtaga aatatttgtg 17400
ttttttatat agtttataaa gtgtgagtta attaaatttt ttttttata aattatttag 17460
ttttagggtt ttttttgttt gtttgtttgt ttgttttggt ggaggtgggg gtggatagtg 17520
tttttttttg ttgtttagga gggagtgtag tggggtgatt ttagtttaat gtatttttttg 17580
tttttgggtt gaagtgattt ttttgtttta gtttttttgag tagttgggat tatagttatg 17640
tgttattatg tttggttaat ttttatattt ttagtagaga tggggtttta ttatgttggt 17700
taggttggtt ttgagttttt aattttatga ttttttttatt tttaatgttt tgttttggtt 17760
ttttatagtg ttgggattat aggtgtgagt tattatgttt ggttgtgggt attttttagt 17820
agtgtaagga tagattattg tatttggtga atgtttttga tggtagtaat gaggtatatt 17880
tgggggtgtt ttgtggtaga tatatttgaa ggtggttggg gttgttagtg agggaatttg 17940
ggaggagtta attggagatt taagtttttt ttatgagaaa tatttgagtt ttggtttgtt 18000
ttgtggaatt tgggttatgt tggggattga ggttttttgt tttgggttta atggaggttg 18060
tgggtggagg ttgttggggg agggtgttaa gtgaaaatgt tgtagggagg gtgtgttttt 18120
ggtgggggtt gtggttttgt ggtggttgtg gtttttgttg tggttgtgat ttttgtggtg 18180
gttgtgattg tgggttttgt ggtggttgtg gtttttgaga tggttgtggt tttgtagtgg 18240
gtgtagtttt tttattttat ttgttgttgt tggatttttt tttttgtgtg tagtttttgt 18300
taggatttta tgtttggatt ttggttttgg gttttttttt tggttttgtg agattggtgt 18360
tatttttatt ggagttaata ggtgtttggt atagttattt ttagtttttt ttgtttttgg 18420
atgagttggt ggttgttttt atgtaggaat gttgatagtt tttggtatta tatagggtta 18480
ggattttggt tgtgattttt tttttagtga ttttttgtgg gattgatggt tagagagggg 18540
gtttgttggt tttgtagtgt ttttttttatt attagttata ttgagttttg ggtaggagtt 18600
ttttggtttt ggttttattt attttaggag gatttagtga gtttagtttt tatttgggat 18660
attgtagagg ttaagtttat tattgtgagg tttagtgggt gttgggagtt atggtaattt 18720
tgtagggatg gtttttggtt ttttttttag ggattttatt ttagttgtga tttgttttat 18780
ggtatttgta ttgtgatatt ttttttgttg tttatgtggt tatgagggtt gttttttgta 18840
tgtttatttt tgagttttag ttttaaagta gtgttgggtt tattaggttt ttttgtggat 18900
gttgtttttg tttgtgtggt gggatgttaa tagtttttgt gttatgtggt tgttaatttt 18960
ggaagtttgt tttttttttt tttttttgt tatggttttg tttttatatt ttgttgtttg 19020
agttagtgtt tatggttgta ttagatgtta ggtttggttt tagttgaggg gttggtgttg 19080
tgtatgtagg tagggggttt ggtaatgggg ttatattatt tgtatgaatg gttttgttat 19140
ttttttttgt tttgtttagt ataggttgag tttatagttt agatagtagt gttttgagag 19200
ttgtttggta tattttggga agaggatgaa ttttgttgta tggggaggtt ttggttttga 19260
gagttgtttg gtatattttg ggaagagggt gaattttgtt gtatggggag tttttggtag 19320
gagtggggtt gtttggtgat tgtgtgaagt attttttggg atgaattagg gttttgttaa 19380
agttgaggtt gggttgggat ttttggggtt ttattgtttg ggatgaggtg ggttttagtt 19440
gggttgtggt aggggttagg aggtatttgt atttgttttg attttttttt ttatatttgt 19500
agagtttttt tatttattga aaagtagggt gttttgggtg ggttttggtt tgagtattta 19560
gagatttagt tttaagttaa gttttttatt ttttttgagt tttagttttt ttattttttaa 19620
atgaagatgt ttaaggagag taggttttgt tttttagttg gatttattag agtgtataga 19680
ggtgtttagg agagtaggtt ttgtttttta gttggattta ttagagtgta tagaggtgtt 19740
taggtgagta ggttttgttt tttagttggg tttattagag tgttgttgta ggtttgaggt 19800
tttatttggg tggtataatt tgttggtgga ggaaagagtt gttgtagttt aggtgggatt 19860
ggggggtttt tatgattttt taggagggtg ggggttttgt gtgggagttt tttattagtt 19920

```
ttagatgtgg tttttagggt atatttattt aaatttttta tttttatttt tttttaagtg   19980
taggtttggt agtggttttt atttggggtt gggagggggt aggtttttgg tgtttatggg   20040
atgttatttt atattgggta ttggtttagg tgttagggga tagagtgggt attttagtgg   20100
gttgtgtggg tgagggtttt agtattgttt ttgtttttta ttttgaaggt tggagtagtt   20160
tgggaagttg tattttaatg agtattttat tttaatagag tgtttattga agtttgagat   20220
tatggttagg agggttagtt agggttttag gggtgggatt taggtttttg tagaaggttt   20280
tatttttgaa attttttgag ggttttttttg tttttgtttg gattttgggt tatgttttgt   20340
ttttggtagt tgtagtttgt attttatttt agatgttttg gtgggaagtt tttgttgttg   20400
ggtttttagg ttggaatttg taggatgtta gggtattttg tgtttttggg gatgttttgt   20460
ggtagttttt tgaggttttt agatattagg atattttttt agtttagttt tagtaatata   20520
tgttggtagt gtttgtgtta gtgttttttt tttttgttgg gaggttggtg atttgttgtg   20580
ttgttttggt ggggggttga tttttgattt tttgtttggt gggttgtgtt gtattagtta   20640
gggttgggag gtgtatgtgg atttgtgttt attggaattt tgttgtgatt agatttgggg   20700
gtgatgttga gatttattag gttttgtggt tttttgtgaa tatttgtagg ttgttggggg   20760
tagtggttga tggaataagt gtggggtggt ttgggttttt tggaggaagt gttgtttgag   20820
ttgttagggg agttatttgt agaaagggag gtggggtgta ttaggtaggg gggtagttag   20880
ggtaaatgtt tgatgtttag agggtgatag tgggtagtgg ggttttttgg aggagtggag   20940
ggagggtttt gttggttgta gggtagagtg ttttatattt tttttggttg ttatttttag   21000
ggtttagttt agtttgggga ttaagatgag aggtttttgg ggtgttttag gtgagttgga   21060
gttagttttt gttgtttttg aataattata ggggagttag tttagtgatt aagtttttgt   21120
ttggtggggg aggggtatgg gttttgtgtt tgggagttgt tgtttttagt tttaggagag   21180
ggggtgttat gggttgtggg ataggttata gttagggtgg ggtttttgga gaggggtttg   21240
aggattgttt ttgtggagtt tatttagtta ttattggttg ttatggttat tggtagtttt   21300
taatttttat ggtgttgggt ttgatttttg tagtgtttta ggtgggtatg attttatttg   21360
atttttttgg ggtgggtggg attgaggtta ggggattttt gtttagaatt tagggtggtt   21420
gatgtgggga ggggtattgt ggggttgata ggtttttttt ttggttatta ggtttttagg   21480
agggtttagg tgtttggagg tggggggtgt tttagtttga tgttggggat gtgagtagat   21540
ttagtgttta tttatttgta gggttgagtt tttattgggt gttgggttgt gatttgtata   21600
ttggattttg tatttttggg gtatttagtg attttagaat agtgggattt tagaggtttt   21660
ttagggagtt ttagaatatg atagatgggg gtttaggtgt gaggtagtgg gagttggagg   21720
aatatagtta ggggttgagg tgagaattat gaggatggtg ggtagaggtt gaggggaggg   21780
gtagtagatg gagatggggg aggtgttagg ggttataaag gggtttttta gaggatttgt   21840
tttgggaagt tattgttgtg ggagggtggg agggatatag tttaggtatt tttagagagg   21900
atggaaaggt ttatggggtg gattttgggt aggggatttg ggattagttt gtagtttttt   21960
ttgagggttt ggttatggtt gtaggttggg gaggagggga ggggatttg gggttaggtg   22020
ttatggtttt ttaagtattt ttgtttgatg gtaaatataa tatgtgttgt ttgtagaata   22080
tgtggaaata ttgaaatgtt taaaggagag aatatggttt tggtatttgg tttttaaggg   22140
agttgttggt gtttgtttaa taggagttga ttttatttgt gaatagatgg taggtggttg   22200
tttttatgtt tttttagaga ggggtttttt gagttttgtt gttatttggg ggtgttttat   22260
tttatttagt tagtggtggg gttttttggg ttggtataga gttaggggag ggggttgggg   22320
gatttttttgg tgggaaaatg tttggtgatt tttagttttt gtgtttagtg ggaggaggtg   22380
tttggttttg ttttttataa ttagtggtgt ttatggtggg tttggggatt agtattttgg   22440
gagtttttta ggaatgtaga tttttaggtt tttattgttt ggggagttg              22489
```

<210> 260
<211> 22489
<212> DNA
<213> Artificial Sequence

<220>
<223> chemically treated genomic DNA (Homo sapiens)

<400> 260

```
tgatttttta ggtagtgagg gtttgggaat ttgtattttt gagaagtttt tgggatgttg      60
atttttgggt ttgttgtgag tgttgttggt tgtaagaagt gggattgagt gttttttttt     120
gttgagtgtg gaggttgggg attattggat atttttttat taggaggttt tttagttttt     180
ttttttgatt ttgtgttgat ttagggagtt ttgttattgg ttgaatgaag tgaggtgttt     240
ttaggtgata gtagaatttg gagggttttt ttttgagggg atatgggagt agttgtttgt     300
tgtttgttta taggtggggt tggtttttgt tgaataggtg ttagtagttt ttttgaaggt     360
tgggtattgg gattatgttt ttttttttaa gtgttttagt gtttttatat gttttgtaag     420
```

```
tagtgtgtgt tatgtttatt attgagtaag agtgtttaga ggattatggt atttggtttt     480
aaggtttttt tttttttttt ttagtttgtg gttatggtta ggtttttagg ggaggttgta     540
ggttggtttt aggtttttttg tttagaattt gttttataag tttttttatt ttttttggag     600
gtatttgggt tgtatttttt ttattttttt atagtagtgg ttttttaagg tagattttttt     660
gagaattttt tttgtgattt ttaatgtttt ttttattttt atttgttgtt ttttttttta     720
gtttttgttt attattttta tggtttttgt tttagttttt ggttgtgttt ttttagtttt     780
tattgttttg tatttgggtt tttatttgtt atgttttggg gtttttttgag aagtttttgg     840
ggttttattg ttttggaatt attgggtgtt ttagggggtgt agggtttggt gtgtgagtta     900
tggtttagtg tttagtggga gtttagtttt gtgggtgggt gggtgttgga tttatttata     960
tttttaatat taagttggga tattttttat ttttaagtat ttgggttttt ttgggggttt    1020
gatggttaga gaggggattt gttggtttta tagtgttttt ttttatattg gttattttga    1080
gttttgggta ggagtttttt ggttttagtt ttatttattt taggaggatt aaatgaggtt    1140
atgtttgttt gggatgttgt agaggttaaa tttagtattg tgaggattag gggttgttag    1200
tagttatggt aattgatggt ggttggatgg attttgtagg gatggttttt agattttttt    1260
ttagggattt tattttaatt gtgatttgtt ttatggtttg tgatattttt ttttttgagg    1320
ttgaggatag tagtttttgg atataaagtt tgtgtttttt ttttattagg tagaggtttg    1380
gttattgggt tgattttttt gtaattattt aaaagtaata ggggttgatt ttagtttatt    1440
tggaatattt tagaggtttt ttgttttagt ttttagattg ggttgggttt tgggggtggt    1500
ggttaggagg agtgtggggt attttgtttt gtagttaata gggttttttt tttgtttttt    1560
taaagggttt tgttgtttat tgttattttt tgagtattag gtgtttgttt tggttgtttt    1620
tttgtttggt gtattttatt ttttttttttg taggtaattt ttttggtagt ttaagtaatg    1680
tttttttttgg gaagtttggg ttgttttata tttgttttgt tgattgttgt ttttaatagt    1740
ttgtagatgt ttataggagg ttatggggtt tggtgggttt tgatgttgtt tttgggtttg    1800
attatagtag gattttaata aatatgggtt tatgtgtatt ttttggtttt ggttggtgta    1860
gtatagtttg ttagatagga ggttagagat tagtttttta ttggggtagt atagtgggtt    1920
attagttttt tgataggaga agaaaatatt ggtgtaggtg ttgttggtat gtgttgttgg    1980
gattgggttg ggagggtgtt ttggtgtttg agagttttag ggagttattg tagagtgttt    2040
ttaagagtat agggtgtttt gatattttgt ggattttagt ttggggattt ggtagtaggg    2100
gtttttttatt aaggtgtttg gagtgagatg taggttgtag ttgttaaggg tagagtgtgg    2160
tttggggttt agatagaaat agggagattt ttaaagggtt ttaaggatgg ggttttttgt    2220
aggggtttga gttttgtttt tggggttttg gttgattttt ttggttgtgg ttttgagttt    2280
tagtgggtat tttgttaaaa tggggtgttt attaaaatgt agtttttttag gttattttag    2340
tttttaggat gggaggtggg ggtagtgttg aggtttttat ttatataatt tgttggggtg    2400
tttatttgt tttttggtat ttggattagt gtttagtgtg gagtgatgtt ttatgagtat    2460
taaagatttg ttttttttta gttttaggta gaagttattg ttgggtttgt atttggggga    2520
aggtgaggat gaggaatttg agtgaatatg ttttggaaat tgtgtttgga gttggtgaga    2580
gatttttata taagattttt atttttttgg gggattatgg ggatttttta gttttatttg    2640
ggttatggta gtttttttttt ttgttagtag gttgtattat ttaggtgagg ttttaggttt    2700
gtagtagtat tttgataggt ttagttgggg ggtggggttt gtttgtttag gtatttttgt    2760
gtattttgat aggtttagtt ggggggtggg gtttgttttt ttgggtattt ttgtgtattt    2820
tgataggttt agttgggggg tggggtttgt tttttttggg tatttttatt tgaagatggg    2880
gaaattgagg tttagagagg gtaaagggtt tggtttggag ttgagttttt ggatgtttag    2940
gttggggttt atttaggata ttttgttttt tagtgagtgg aggggttttg taggtgtgag    3000
gagggagatt agggtagatg taggtatttt ttggtttttg ttgtagttta gttgggattt    3060
gttttatttt agataatgag gttttaggag ttttggttta gttttagttt tggtagggtt    3120
ttggtttatt ttgggaggtg ttttatgtgg ttattaggta gttttatttt tgttaaaggt    3180
tttttatatg gtaagattta tttttttttt gggatgtgtt aggtagtttt tggggttaaa    3240
gtttttttat atggtaagat ttattttttt tttggggtgt gttaggtagt ttttggggta    3300
ttgttgtttg ggttgtggat ttagtttgtg ttgggtaggg tagggggaag tagtaaagtt    3360
gtttatgtag atggtatagt tttattatta ggtttttttgt ttgtgtgtgt gatattagtt    3420
ttttagttgg ggttaaattt agtatttggt gtagttgtgg gtgttgattt aggtagtggg    3480
gtgtgaaggt aggattatag tggggggagg aggaaggggg tgagttttta aaattaatag    3540
ttatatgatg tggagattat taatatttta ttgtatagat agaggtggtg tttatagagg    3600
ggtttggtga gtttagtatt gttttagggt tggggtttgg gaatggatgt gtaaagggta    3660
gtttttatag ttatatggat agtaaggagg gtgttatggt ataggtgttg tgggataggt    3720
tatagttagg gtggggtttt tggagagggg attgaggatt atttttgtgg agttgttatg    3780
gtttttggta tttattgagt tttatagtgg tgggtttgat ttttgtggtg ttttaggtgg    3840
agattggatt tattggattt ttttggggtg ggtgggatta aggttagggg atttttgttt    3900
agaatttagt gtggttgatg gtgggagggg tattgtaggg ttgataggtt tttttttttgg    3960
ttattagttt tgtaggaagt tattggggga ggggttatag ttaggatttt gattttgtgt    4020
gatgttaaag gttgttggta tttttgtgtg ggagtggtta ttaatttgtt tagagataga    4080
```

```
gggagttggg ggtagttgtg ttgaatgttt attggtttta gtggagatgg tgttagtttt    4140
atgaggttag agaagagatt tagagttagg atttagatat ggggttttag tgggggttat    4200
atatagggga gagggtttag tggtagtagg tggggtggga gaattatatt tattgtaaaa    4260
ttataattat tttaaaaatt ataattattg taaaatttat agttataatt attgtaaaaa    4320
ttgtaattat aataaaaatt atgattatta taaaattata atttttgtta gaagtatgtt    4380
tttttatag tatttttatt tagtattttt ttttaataat ttttatttgt agtttttgtt    4440
gaatttaaaa tagaggattt taatttttag tatggtttgg gttttatagg ataggttagg    4500
gtttagatgt tttttataga gaggatttgg gtttttggtt ggtttttttt agattttttt    4560
gttggtaatt ttaattattt ttaggtgtgt ttgttatggg gtatttttag gtgtgtttta    4620
ttattgttgt taggggtgtt tattaggtgt ggtggtttgt ttttgtattg ttagaaaata    4680
tttatgattg ggtgtggtgg tttatgtttg taattttaat attgtgggag gttaaggtgg    4740
ggtgttgggg gtagggggat tatgaggtta agagtttgag attaatttgg ttaatatggt    4800
gaaattttgt ttttattaaa agtataaaaa ttagttaggt atggtggtat ataattgtaa    4860
ttttagttat ttaggaggtt gaggtaggag aattatttta atttaggagt agagggtgta    4920
ttgagttgag attgttttat tgtatttttt tttgggtgat agagagagat gttgtttatt    4980
tttgttttta ttaaaataaa taaataaata aataaaaaaa attttgagat tgggtaattt    5040
atagagaaaa gagtttgatt ggtttatgtt ttgtaggtta tatggaaagt ataggtgttt    5100
ttgtttttgg agaggtttta ggaagttttt aattgtggtg gaaggtgaag tgggagtagg    5160
tgttttatat ggtgtgagtg ggaatgagaa agatagagta ggtgtgggat atagttaaat    5220
ggttatttat aattttgagg atagtattag gagggatggg gttaagttat ttatgagaaa    5280
tttgtttttg ttatttagtt atttttttatt aggttttatt gttatattgt agattttatt    5340
tttatgtgag gtttgggtgg ggatgtagag ttgtattata ttattattta ggaggatgtt    5400
taggagtagg tgtgtggggt gtttagggat gatatgtttt atggggggat tttgttttta    5460
ggggttaggg tgtttgggaa gaggttggga gttggtgtgg ttgtggtttt tttatttagg    5520
tattatagat tagggttggt gggttttagg gggtgtttat atttattatt ttgaatttta    5580
tgtttttttt tagggaaatg tgtagaggaa atggttggag agtgaagttt aggtataggg    5640
gttatgagag ttggatttta tgtagttgta gattttgatt tgaattgtat gtggttgagg    5700
atttttaagt taggtagatt ttgatttaga ttttatgtgg ttgtggattt ttgattttga    5760
tttaaattgt atgtggttga ggatttttaa gttaggtaga tgttaattta gattttatgt    5820
ggttgtggat tttaatttag atagattttg atttatttgt gtagttttta gttttagaag    5880
ttaaatttta gtgtttttta gggtggtttg ggttttttta gatatagtag tttttttgagg    5940
tggttttatt tgtaatttta gattaattag agaaggttaa atgggttttt taagtttagt    6000
aggtgggatt tttgatttag agagtttttt ttttgtttta ggttaatagt tatttttggg    6060
ttttgtatta ggtttgttaa atgtaggggt gaggggttga ttttgttatg ggaattaaat    6120
ttaggaaata gtttatttag tgtttatttt tatttggatt tgttgttgtg gttgtaggtt    6180
tatgtgttgt tttgtggaga ttttttaggt gtttttggtt tggtagaaat ttgtggtttt    6240
gatggtagag gttttggtgg gaattgtatt gtttgttgtt tttgtttttg tggaggatgt    6300
gttgttttaa attgtttttg gtttttttatt ttttaatttt tggtagtttt tgattttttt    6360
ttggttggtg tagtttagtt ttttgtagat gtgggggagt tggaatttga ttatgtgtag    6420
tttttttatt gtggtgtatg ttttgggttt tttatgtttt ttttaggttt tattatttat    6480
tttttttttag tgttgaagga aattttttag atgttttttt atttatttat tgaagtaggt    6540
tttggttgtt tttagttttg ggtttttttg agaatattgt aagtttttat tggttttttt    6600
ttggtaattt tatggtttat ttttaatttg tatttatttt gtggggtttt tattttttgg    6660
aggaaggatg tgttaaagtt tggaggtgtg tggtttgttt aatttttgtt taaggtgtgt    6720
ggtaggtgat aggttttagt ttggattagt gtggtttaaa aggatttatt gttaaaagtg    6780
ttggttttag aatttaaggg gaagatatgt tggtttttttg atgagttggg ttgttttttt    6840
taggaagttt ttttttttgga gttttgtagt tattgtgggt ttgtaagagt taaagggttt    6900
ggtttatagt ggtgtttggt attgagttgg atagtgtttt gtttttaaaa tttatgtttg    6960
tttagaattt tagaatgtaa tttttttttgg tagtagggtt ttgtagatgt aattaaggat    7020
ttggggatga ggttattttg gattgaggtt gagttttaag ttagttattg gtgttttttt    7080
atgaagagta gagtagaata gagatagatg tatagagtag gttttgtgag gatagaggta    7140
gggattggag tgttggttgt agtaggggtg tggagttttt agagttggaa gagggaggga    7200
tttttttttag ggtttttgga aggagtgtgt tgtgtgttgt ttatattgtg attttggatt    7260
tgtggtattt agaattggga gagaataaat ttttgatgtt gtaagtttgt attatagtag    7320
ttttaggatg ttgaaatagt ttagttggtg taggtttttt tttttttttt tttttttttgt    7380
tgtgagatat tttttagatg gggttttatt tgttgttgga gtgtagtggt gtgattatag    7440
tttattggag tttttaatgt ttgggtttaa gtgatttttt tattttagtt ttttgagtag    7500
ttgggattat aggtgttatt agatttggtt aatttttaaa aattttttta gagatggggg    7560
aggttttttt atgtggttta ggttggtttt gaatttttgg gtttaagtga ttttttttgtt    7620
ttggtttttt aaagtgttag gatggtaggt ttgagttatt atgtttggta gttttatttg    7680
tgttgatttt tgaagttttt tattttggtt aatttggata ggtttgttgg ggattttatt    7740
```

```
tttttttgga gattttggga tgtggttgga tgtttaagtg gttggggttt tttatagttt    7800
ttagtttttt agggtgggag gtaggttgtg tttttggtgg gtattgatag tgaggttgaa    7860
atgtttgtat tttatatttt tatatttagg ggtattttgt tgttgattta tttaaagttt    7920
ttttaagttg tttaatttgt tgtttagagg gaaatgaaaa gggagttatt ttttttgatt    7980
tgtttgtttt attaattttt taattaataa aaaagtgtat tttgtttatt ggatgtaaat    8040
aggtttattt tttttaatat gttgaaatat agtgatttat tagttatttt attttatttg    8100
gaagttgaaa gtttaatggt gtttggtgag aatttttttat ttgagaagag ttggtaataa    8160
gttagtatga atgtataggt ggggtgtggt tttgtgtggg tgtagaagtg tggtgttggg    8220
tgatgtatgg aaatggggat ttgtagtggt tgggagtgtg aaggttgtgg ttatgatgtt    8280
ggtttattgg tgattagtgt ttattggggt ttgttttttt ttagaggtta tatttttata    8340
gatttaaatt tagggttttt gttaggttgg attgtagatg ttttgttagg gatgtaggta    8400
ggggatttga atgaggtgta aattttttgt taaaggataa gtatgttttt gtttaggtag    8460
ttattgtttg tttttttatag ttgtagttgt tgttgtttag tatggggggt tatgtttggt    8520
attgtgagtt tagggggttt ggagtttgtt agttttaaag atagtaagat gttttttttag    8580
gtgtttaggt tagtgatggg tattttttgt ttattttatt tattttttttg ggtttttttt    8640
tttttagatg tagttagtgt gtgtgttttg gtggggtggg gtttttgttt ttggttattt    8700
tatgagtttg attgtatttt tttttttttgt tgattttttt taaatattta gtattttttt    8760
tttttagtat taggtgggga ggaattagtt gggttttggg gttagttttg tttatagtat    8820
tttttatggt tatagtagtg ttggggggatt tgaggttttt atagtatttt ttatggatat    8880
ggtagtgttg ggggggtttga ggtttttata gtatttttta tggatatgat agtattggga    8940
gatttgaggt tttggagtat tttttatgga tatggtagtg ttggggggatt tgaggtttta    9000
gagtttaggg ttgtttatgg aattgggtgt taattttttg ggatttatat ggtagaggtt    9060
ggtggggtag ggaagttatg tgtaggtttt tgaagtagat gttatttgtt tttgattatg    9120
tttgtggtta tttttttgtaa tttttgtgat tttggttgtt tttgattata ggaggtttgg    9180
tttgggtgtg ggaatggtta ggagtgtggt gagtttatgt tttggggtat agttattata    9240
gtttggttat tttttttatgg tagggatagt tttggggtgt tgtttttttgg ggtttgttta    9300
tagtggtgat ttgtttatta ttgtttattg gtatttattt ttttttttta tagtattttt    9360
ttaatgatgg gtgttttttg gggttatttt taaatttttt gtttgaagat ttttgttttt    9420
gagtttttta ggggtattta atagagattt ggttattttg ttgtttttta tgaggatata    9480
ttggttgtag gtaggtgata gaggtgttta ggtgttatat tgttgggtgt tgatttttag    9540
atttagatta tagaggttat agatatgttt ttttggtttt tatggttggg ttttgggagg    9600
gatatggtgg atgttgatat tattattgtt gttgtttttg gtttgggatt agttttgggt    9660
ttggttggtt ttgagttatt ttttagttgt atatagtgtt tgttttgatt taattagtat    9720
ttatggggtg attagaggtt ttgagagttt agttagtggg tgttaggaag ggtgtgaggt    9780
tttaaggagt tagggttgga gttgtagttg ggatgatttt gttagttata gtaaggatgg    9840
taggtagttg gggtagtttt ttggtttaag tattatttta gatttattta ttttgtggta    9900
ggggttggtg gggaaattgt ggtttggagg ttaggggttt tgtttatagt tattgtttgt    9960
gtgagttttt tggggttgtt ataataaaga gttatgaatt gggagttaaa gttatggaaa   10020
tgtgattttt ttagttttgg ggttgtaagt ttgagattga ggtgtttgta gggttatatt   10080
tttttttgaag tttttagggg agggttttat tgttttgtat tgttagttat ttgtggtgtt   10140
tttgggttgt ttttttttag tttttgtttt tgatgttatg tgtttgattt ttttttgttg   10200
ttagtagttt ttttataagg tgttggttgt tgtttttggg atttttttttg tttgatttta   10260
ttttaatttg attatttata aagattttat ttttagtgtt tgtaggtatt tggggttagg   10320
gttttgatat atgtttttgg aggggtttaa tttaatttat tttattagtt tttgagtgtt   10380
ggggtttggg atttaatttt tttggttggt tgtagtgatt attgggatat atggtttatt   10440
agttagtttt tttttggggt ttaggtttta gttttattta ttttttttatg aggattattt   10500
ggggtttttag agaagggaga ggtagttgat tgttggggag atggagtttt gggggagggg   10560
tggtgtttat ttttatttgt taggttgttg ggttttatgg agggggtatt tgagattttt   10620
ttttaagagt taggtttttt ttttttagta gagttaggtt ttgaggggta gttggagggt   10680
ttaggtttgt gtttggtttt agtagaattg tgtggtttga gtttgagaag taatttattt   10740
gtatgttagt tttgggagtt tttttgttat ttttggagat atttaagagt tgttatttat   10800
aattttatta gggatggtgg ggttaggggt agttgttgat tgttgttttt gtagggtggg   10860
gttgtggtaa tattatattt gtttagggtt tgtgaatttg tttgtgttgt ttgaggagtg   10920
gttattgatg gtattagatg gaggtttttt agtttttttt tttgagtttt ggtagtgaga   10980
gtgggtgttg aaattttatt ttgggtttta aaggatattg gttgattttt gtgtagaaag   11040

ttataggttt tgttgtattt tttttttgtg gatgggtgtg gatgtgttat tatttgattg   11100
tttttttggga gggttgggag ttttatttgt ttttgttttg ttaaggtatt gattggttgg   11160
aggtgtggaa tttggtttgg ttgttggtgg tttgatttgg ggattggttt tttgaggttt   11220
gttttgttta gttttgtttg gggtataggg ttttttaggt tttgggttgt ggtgtggggt   11280
tgttagataa atgtagtaat ggtagtaagg agaattggta tttttattgg gtttttttatg   11340
```

```
tttattatgt ttgttttttt atgtggtttt ttgatttttg tgagggaggt gaggttgtat   11400
tataggtgag gatgtggggg attaaggtag gggtattttt tatttatttt tagttatgta   11460
gttgattggg gttgggttgg ggtttagatt taaagttgat gttggtgttt gtatttttgg   11520
ggtgttgttt ttgttattag gatatggatg gggtattatt ggtagttagt gggtggggtg   11580
ggggatatgg agtttggttt taggatttag aggttattta ggttttagta aaggtaaata   11640
tgtatttttt gtggggatat gttttttttgg ggtttgggtt ttatagaaga aatggtttgt   11700
aatggtgagt ttatattttg agggattggt agatatggta ttattttta atttttaatg   11760
ttttattttt tttttaaaga gatgaagttt tgttttgtta tttaggttgg aatatagtgg   11820
tataattata gtttattgta gttttggatt tttaggttta agttattttt ttgttttagt   11880
tttttaagta gttgggatta taggtagtgt tattaagttt aataaatgtt ttttgttttt   11940
ttgtagaaag agttttatta gtttgtttag gttgttttta gtttttgggt ttaagtagtt   12000
tatttgtttt ggttttttag agttttaaga ttatgggtgt gagttattgt atttagttaa   12060
tagtatttta agaattagaa ataataatag aataattaag agattatagg ttaggtgtgg   12120
tggtgtatgt ttgtaatttt agtattttgg gaggttgagg agggtagatt atttgaggtt   12180
aggagtttga gattagtttg gttaataagg tgaaatttta tgtttattaa aaatataaaa   12240
aattagtggg atatggtggt gggtgtttgt aattttagtt atttgggaag ttgaagtagg   12300
agaatggttt gaatttggga ggtggaggtt gtagtgagtt aagattatgt tattgtattt   12360
tagtttgggt tatagagtga gaatttgtta aaaaaaaaaa aaaaagatta tatatgagtt   12420
taaaatgatt aaagagataa aagtagaaat gaaaaataat ataatagtat gaaaaggatt   12480
tagtagatta gaaaaataat tatataggat ttttaatatg aggaatatgg ttgttggtat   12540
aaaaatttta gttgatggtt ttaatagtag attagatgtg gttaaaagag aatatgggag   12600
ttggaagata gttttgagga agttatttag atgatgtttt tttatatagt agtaggtaag   12660
agattgaaat taagttgtag aaaggttagt gtttttatat tgaagatatt ttttaatttt   12720
tgtttagaat attggaaaaa tgaattgaga tttagattta ataaagaggg tgggagatat   12780
taggaaatat ttatatatta aaaaaaaaat aaaaaaaaat ttttaatttt aggttgagtg   12840
tagtggttta tgtttgtaat tttagtattt tttgtgaggt taaggtggga ggattatttg   12900
aggttaggag tttaagagat tagtttggat aatatagtga gattttaatt tttataaaaa   12960
attaaaaaat ttgtggggta tggtggtata tatttgtagt tttagttatt ggggaggttg   13020
aggtgggagg attatttgag tttaggagtt tgaggttgtt ttgaggtgtg atggtattat   13080
tgtattttag tgtgggggat agagtgaggt tttgttttaa aaatatgttt ggtgtggtgg   13140
tttatgtttg taattttagt attttgggag gttgagatag gtagattgtt tgggttagga   13200
gtttaagatt agtttgatta atatggtgaa attttatttt tattgaaaat ataaaaatta   13260
gttaggtgtg gtggtatgtg tttgtagttt tagttatttg ggaggttgag gtaggagagt   13320
tttttgaatt tgggaggtgg agtttgtagt gagttgagat tatattattg tattttagtt   13380
tgggtaatag agtgagattt tgtttgaaat ataaaatttt tttagttttg tgaagtgtgt   13440
aaagaggttt tggaagtttg tagatgagat gtaggtataa agtttgtttt tattttttgt   13500
ttttagaatg ttttggggat tttggtgggt attggttagg gtttgtgggg gtttttttggg   13560
gtaggatgta gtttgggtga ttgggttgag gtttttagtt gattttgggg ggttttaggt   13620
aggttggggt agatgggagg gtgggggttg gggaggtagt agtttttttt tttgtggtgt   13680
ggttgtgatt tttttttttt tatatgaagt agttaggatg tggtagttgg gggtttgggg   13740
tttttatggg gtaagtttaa gtttagtgtt ggttttttttg ggattttttta tagtttttgt   13800
tatgattatg ttatgtggat atgggatttt tggttgaaga gataaatggt agtattgatt   13860
aggggtaggt agaggttgga tattgagtag gaagtggggg ttgatgggag ttgggatttt   13920
gggtggggga agggagggtt tgttggtaag gttttttttta ttaggttgtt ttttgtgggt   13980
tttgagtgtt ttttgattag ttttttaggg gtttttattt tgggattttt atgtttttta   14040
tgaattgaat ttgtttggat tttatgtttg gtgatgtttt tatttttttt tttttagttt   14100
tagggtgggg ggattttgtt ttgtttttgt ttggattttg ttgggggaat taaatagggg   14160
gatggggtgt ggggtagaag ggatagtagt aggggtttag ggttatttta gaaagtgggg   14220
gtttttggag gggattagtt tttttgttat ttggtgggtg atattggggt ttttaggtat   14280
aggttgttta ttgtttagtg ttggattttt tagttttatt atatttggtt ttttttttga   14340
ttaggttttg attttgggga tttttgagga ggggtttgga tatgttgggg gggggggttgt   14400
agtagttttt attggatggt ttttgtgttt gaattgtggt gttgggattg ttggttttttg   14460
gtgtttttttg gtgggtatag tgggttttgt gtgaggttgg gtttggtggg tggttttgag   14520
gttggagttt agtgatagtt atagtgggtt ttgaattggg gttggggtag aggtagtgag   14580
agttggggtt tttgtgtttt atggtttggt agggatttgt ttttagtagt tttgttgtgt   14640
ggttttggtt ggttagttag ttttttgag tttggtgttt ttggtaatat ggggttataa   14700
tgatgttttt tgtttatttt gttaggtatt tttaggaaag ttttgttgtg ggtttgtttt   14760
taggtttgaa tttgtttttt ggggattttt ggattggtag gttttgtttt ggtgtttttg   14820
atagttttgt ttaggttaat ttttttttgg ggatgggtat tgttagtttt atgggttttt   14880
agtgataggg ataggagata gaaattttag gtagaaaaag atggggtttt tgaggaattt   14940
tatttttaag tttggaagta tagttttgag tgagaatatg tattttagtt tttttgtttt   15000
```

aatgttgttt tttttaaaat tattattgtt ttgttttatt tttattttgt atttataaaa 15060
atttttgatt ttattggtag agaatagagt aggtaaggag aggagaagta gttggttatt 15120
ggagatggta gtttgatatt agagaggagt agtgtgattt tagagggata gttggatagt 15180
aggattttgg agaagagttt ggtagagatg gtaggatttt aggggattat tatttttttt 15240
ttttattttt tttttagttt ttttttttat tgagagtttt ttttattatt tttttttttt 15300
attttttttt tagtttttgt tttttgtgag agtttttgttt attattttttt ttttttattt 15360
tttttttagt tttttttttt ttgatagttt tttttattat tttttttttt tatttttttt 15420
ttagtttttt ttttttttga gagtttttttt tattattttt tttttttatt tttttttttag 15480
tttttttttt ttttgagagt ttttttttatt attttttttt tttattttttt tttttaggtt 15540
tttttttttt tattggttat aaaatttttt gtatttatta tttttttaatt tttttatgta 15600
atttgatatt ttttggatgt tggataagag tttgggatat agaaagttgt tatattgatt 15660
ttttgttttt gagaaaaggt agagggttta ttgagttgtt aaatatttta gatattttttg 15720
gatagtaaag ttaaaagtat tgtaatatat attttttgag gttttatggg ttataggtat 15780
tgttttagat attgttatag gattgtatgg agttttgttt ttgttgtgtt tagaagtgtt 15840
tattttagtt tttgtatttg tttatttgtg tgtttttgtt gttttgaggg gttgagagtt 15900
ttgggttaag taagatattg ttgttattag gtttataaag gggttaagga aaattttttg 15960
ttttattggg gtgtgtgtgg tatggatggg agaggagggg taatataggg ttgtttttat 16020
taggttattt tttttttgt ttttggggat ttgttttagt gttgtttttt aggtagatgg 16080
gtttggggtt agggattttt ttaaagtagg taatttttag tttggttatt ttttgatttt 16140
gggttttatg gttgggtttt taggtagggt ttggttattt aaaagtagtt ttagtatttt 16200
aattaatttt tattttataa ggttatagtt tttatagtag ttttagtatg ttttggtttg 16260
ttgtttttttg agtattttttg ataaattttt gttttgtaag attatagttt ttatggtagt 16320
tttgtttatt tttttttggt ttggagttta gggtttttgt aataggttgg ggtttatgga 16380
gagtggtttg tatttggtgt gttggggtgt ggttgttaat atttggggtg tagttttatt 16440
aaagtagggt gtttgtagtg gtttttatat tattatgggg tgggtttttt tgaggttttt 16500
ttgttttatt ttttttttgtt tttagaagtg ttgagtttga gtgttttggg gttttgtttt 16560
gaatatattg tgtgtttttt tttttttagt tggtttttag agatttttgg tagggtttga 16620
tttttaggat tatggttgta agtaattttt tggttttttt tttttttttta gttttgtaaa 16680
ttagtttttt gattaaggag atttttagaa ttgtttttttg attttgtaat ttgagaggat 16740
tatattattg tgtagttttt attttgggtt ttggtttttt gagttttagg attttgtatt 16800
agggtttgaa gaattaggtg gagtatggtt ggtgttggtt attgggggt atatgtgtag 16860
tttttaaggt ttttagtagt gttggtgttt ggtgtttagt tttgttaggt ttaggtttga 16920
ggataggggt tgggggtttt atgtggggtt ttttttgttt tgtggtgagg gttttttttat 16980
gttaagattg aaatttttgg tttttaggtt aagatttagg gtttagaatt agtttagttg 17040
atttttttgg tttggttgta gggtttaggg aagttggatt ttgggggtgg gattgggatt 17100
tttttgtttt ttttagtttt atttattttt tgttggtttt gttgttggtg agtggttagt 17160
ttggttgttt tatatttttt agtagttggt gggtgatttt tttggtagta ggatttgttt 17220
ggttgggttg gttatatttg ttgtagtagt tggtttaaaa tttttaaatt ttggtaagtt 17280
gtttgtttgg ttttgtattg gttaggttag tgtatttttg gtttgtaggg tttgtttgtt 17340
ttttgttgaa ttggttgttg gggattgtgg ttaggtgggt aggtggatag tattttagtt 17400
tggagatatt agggttgttt atatgttagg ttgtattgtt ttagtttttt gtgtgtgtgg 17460
gtgggggtgg tattttgttt gggttttgtt ggagtaattt agatgagagt ttgtaggttt 17520
tttaggagaa agaaaaatag agatatgggt gttggggttt gatttttatt ttattttttt 17580
gggtagggga gaaagtaggg tgttattttt ttgagggtag gtggttgttg gataggttta 17640
gtttttttgt tttggttagt ttgttttagg tttgttaatt tttttagtgt ttattttttgg 17700
tttattttta ttgggatttt tgggttagtg gtagtttggg taggttagga gggtgtgtag 17760
gttaggaggg tgtgtaggtt aggagggtgt gtaggttagg agggtgggta gtattaggag 17820
ggtgggtagg attaggagga gtggggttta gttgttatag gggtttttta agttgttatt 17880
taattttggt ttgagtttgt tatttgatat ttggttattt ggttgttttt aggttttggg 17940
ggaggttggg tttttggttg ttggatgttg ggagttagag ttttaggagt agagaggtgg 18000
tggttgttgt ttatttaggg tttaggtggg ggtgttgtta aggtaggtgg tgaaattgta 18060
gttagtgttt tttttgtgg gtgtttgttt atttttgttt tttggtattt gttgagtatt 18120
tattgtgtgt tgttggtttt tttgtgttga gatagattta gtaaggggtt tgtggaaatt 18180
gttttgtgtt tagtatgtgg tttttttttg gttttttatt gtttgtgtgt gggggttttt 18240
taagtatgta ttttttttggg attttgtttt gtttagtttg tatttgttgg gtgtaggtgg 18300
ggtttttttgt tttgtttggg gttagtggga ggggttgggg tggttgtggt tattaagtag 18360
gtattttgag gggagagttt gggtagtatt agatggtgat attttggggt tagtgttttg 18420
tatttagtaa gatagtagtt tttgtggagt gttgagtgga tagaggtggg ggttggtttg 18480
ttttgtgttt aaggttgggt gggagagtat tgtagttttg gtttatttta gttgtttatt 18540
ttagtttggt gtttattttt tgaaagtttt ttttgttggt atttgtagtt gaggtaattg 18600
atttttggtg ggggttgggt tggtttgggt tttggttttt tttggttttt gagtttttgg 18660

tttagttgtt gttgaggagg ttgtgtttgt ttttggggtt aatagttgta ggtttggaag 18720
tgtttttttt ttatattttt tagagtaggt gtgtttttta tagtttaatt tagtttgtta 18780
aggatgggtt ttagggatat gggggttttt tgtttttttt tgtttaattt tttggggttt 18840
gggttgggat tgttgttgga gtttttagtt ttgtgggtag tagatgttaa gtttttggtg 18900
gttagtttgt ggttaagtgg gttgagtatt tattgtagtt ttgattaaga gtagttgtgt 18960
tttttagtta tgtttattag atttaaagtg tatggttggg tatatatttt tttatttgtt 19020
ttattgttta ggtttggtgg gggttttttt gatgttattt tttttttttt aggttttgat 19080
atttatattg tttttattga tttgagagtt tgtgttatag tgtttttagt gtggtaggag 19140
agggatgttt gagttatggt agttattggt gattttgtgt tagttgggtg atagttgaat 19200
tttaggttag tttttggtag tttatttgta gtgtttttgg tgggtattta gttttgtttg 19260
gtttaatttt atgttatgtt atgaggttga ttaatttttt agtagttggt ggttatagtg 19320
gagggtgttt gagattttaa attggggatt tattttgtgg atattttttt tttttatta 19380
tagttgtggg tattatgttt tttttttttgt ttttttttgt ttatgggttt tgatattttt 19440
tttttgtttt gttttttttg ttgtttgttg gatttattat tgtttttagt ttttgagttt 19500
ttttttggtt ttggaatttt ttttagtttg ttattagggt ttagtatatt tgttttaggg 19560
atggggtgta gtttttattt tgattagatt ttggtatagg agattttagg ttagggattt 19620
ttttagggaa gtaattggag tttttgtttt atggtggagg ggttaggttt tgggggattt 19680
gttaggggta agtgttagtt agattgtgtg tttgggatag ttgttttgtt atagttggtt 19740
tttgttttgg gttgaatggt gtttttttaga tttatattta tttagaattt tagtgtgtga 19800
ttttatatgg agatggtatt tttgtaaatt tatttattta tttattgaga tggagtttta 19860
ttttgttgtt ttggttggag tgtaatggtg ttattttagt ttattgtaat ttttgttttt 19920
taggtttaat tgattttttt gttttagttt tttgagtagt tgggattata ggtgtttgtt 19980
attatgtttg gttatttttt gtatttttag tagagatagg gttttgttat gttggttggg 20040
ttggttttga atttttgatt ttaggtgatt tgtttgtttt agttttttaa agtgttggga 20100
ttataggtat gagttattga gtttggttat ttttgtaaat ttaattagtt aagatgaggt 20160
tatgtaggat tatggtgaaa gggtttttgt gagaaaagga aaagaggaga atgggtaggg 20220
tttttaggga ggtgttgggt ttggggatgt tagggtttag ggggttaata ggttttggtt 20280
atgtttttat ggtagagtta gagaagatga tgaggttatg gtttttgtat ttttaatatt 20340
taaatttttg tttttatgtt tagattttgg ggggttgatt tttggtgtag agaggtgttt 20400
ttttaggaaa gtgtagaggg ggttggtgtg gtttttaggt gtggggaggg tggtatattt 20460
gagatgtgga tgtaggattt agagtgggag ttttggggaa gtaggaagat ttttgttttt 20520
tttggggtag ggttgtttat tggttaggtg gtttgggtat agaagtgggt gggtttggtg 20580
ggttttgatg taaagggttg gagggagtgt agggtttgat tagtgatgtt tgttttgggt 20640
atatggagga gtggggtttt atgtgttatg gttagttgag agggagaagg ttttgttggg 20700
aaatttttaa aggtttttttg tagttagttt gagttgaggt tgtatagggg ttttagagtt 20760
agtattaggt tagttttaga tgttgggatt agaaggggtt gtagggtttg gtttttaaga 20820
tttgtgtttt ttattgatgg tttggtagtt gggaaagaga agggagaaat tttttttttta 20880
atttttttag tttggttttt ttttgatttt agggatagag ttttgggtta gatgtgaatt 20940
agtggtttta ttaaattttt tatatttgag tttgtgattt tttttgttta ttttgtatta 21000
gatttattag ttatttttgt tttagtgatg gatattttgt tgatattggg ttttggggtt 21060
tttgtgaagt gttaagtggt tttgtgtagg tgatttattt gggtttattt tttttagggt 21120
ttttaagtat ttgatgattt tatttggatg gagtattttg taggttgttt gtattttttta 21180
ttttaggaat tttttatttt tgagagtgat gggagaggtg gatgtttatt ttattgtttt 21240
tttttgtgta ggagtttaat aatttagtag ttgggttgag gtggtgatgt gtagggttgt 21300
ttgtgttttt ggaatttttt tttttttag gttttatgtg tatgtgttgg ttggttgtgg 21360
attgtgtttt tttttgaaag aagtggtttt gttattaagt ttagtgtttt gtttagattg 21420
ttttattagt ttttgttgtt gggtttagtt gtttggggat agtggttgtt tttgttgggt 21480
tggttttgaa atgtttgttt gaggtttagt ttaggtgttt aggttagtgt ttggggtagg 21540
gtatttttag ataaagtttg agtttatgtt ggttttatag gtattttatt tgttttaggg 21600
gttgattttt ggttggttgt tatagttttt tggttagtga ggtttatttt ggttagttgg 21660
tattttaagg ttttgaagaa gagtgagtat ttgttaagta ttgattgttt gttgtttgtg 21720
gatttgtttt ttttttaaat gggatgtttt tggttttttt tttgtagagg gtgaggtgtt 21780
gagagttggt gtttatttat taagttttgg tgtattttta ggttttagat ggtgttgaag 21840
gtattgtttg gaggtattgt tttgtttttt gtattatttt gggggtgaag gtgtgtgtgt 21900
tttatttttt aggtgaggaa aatgaggttt ggggagttga gtgtttgttt gtaggtttag 21960
agatggtagt ttggtttatg gtagtttggt tgttattggg aattatggtt ttgttttgtt 22020
ttgttttgtt ttgtattttt tttgagagtt ttgggttttt tttggtgttg ggtaggtttg 22080
ttattttttta gtagtagttt tagtagaaga gtagagtttt tattgggttt ttttagggtt 22140
gtttttaggg gtaggatttg agtttttagg ggtttttggg gggttatttt tttagttgtg 22200
tttttttttt gtaggatggg gataggggtt tagtatagta tttagtggga ggttgttttg 22260
atagtttagg gtatggtagg gtttttatat gtatgggttg tagggtggtt gtttttagag 22320

ttgttgggtt aggtagagat gaaggggtag tgaggttagt ttttgagggt gggggtggtt 22380
attgtttatg tttttattgg tttttttttt tgttatttga ggtggttgga gatttgggga 22440
taagtgtttg agttttttgt tttattgatt atagtgtttt ttagtggtt 22489

<210> 261
<211> 4419
<212> DNA
<213> Artificial Sequence

<220>
<223> chemically treated genomic DNA (Homo sapiens)

<400> 261

tgtgttgtgt gggtagggag tagttttttt tggttgttga ggtgttttta atttggttgg 60
agagataagt gtattataag aaatagaggt agttaagtgt ttgggttatt attaagtaag 120
gttgaaaagg ggagggtagg tagattggga tttgtgggtg ttaattaatg ttatggtggt 180
ggagagtaaa ggggatgaat atagtttggg gttatttttg gagtttttta gtgtttgttt 240
gtggttgtgt ttggttggtt gtggattttg gtgggtgttg taaagtggtg ggattgttag 300
tgtagagttt tggtttttttg ttttgttttt gggtttgagt attggagttt ttggtgtttg 360
tggggagaag ttttggattg agaaatatgg gagggttttg ttagtggttg taggtgtggt 420
agttattttg gggatttagt gagaatgggg ttgtttggtt ttgtgtgaat tttttatgtg 480
ggtgtagttt ttgagttgtt gagggaggtg gtggtaatgt tgtttagtgt tagtagaggg 540
tagttttgag gttgtgagtt tgaatggtga ttttgttaaa ttgtgggttt ttttttagtt 600
tttggatttt gtggggtaga ggtggttttg gagtttagag attagtgatt ttagtttgta 660
ggagtttggt gtagaggttt aagggtattt ttgggatgtg gttaagttat agtttttagg 720
tagttttatt ttgtgatggt aagggtttag agggtggagg gggattagat gttttaggag 780
gggttagaaa gttaatgtat atagggaatt tgtttttaag taatagattt taagtatgtg 840
gaaatttttt aaatgatgtt ggtgagagtt atgatagttt ttgtattttt ggtgagggaa 900
gttggaggtt agtagtggga tggtttttgg gtgtgtggga gatagaggga ttagtgattt 960
ttgttggggg tagagggatt gtggattaag gatttagata tatttaattg gggattttag 1020
ttttgattgt ggttatttaa ttggtgttga atttgagtaa tttattatat tgttttagtt 1080
tttgattaat tatttttgta aaatgggtat tgtgggtttt gagttttgta agggtgttag 1140
ggatttgaga tagtaggaat ttttttattg ttttagtaat tttgggtttt tgggtttgta 1200
ggtgggttga aaggattttt ttattgtatg tttgtttggt gtggttgttt tagagattga 1260
ggtttgttag gttttgtaat ttagttgtgt gtggttagtt atggttttgg aattgttggg 1320
attgttttag tggtattttg gttagttttt gatttttttgt tttgggtatt agggttattt 1380
agttttagaa gatagtgttt attagtatag gaattgagat tttatatttg gtgtagtggg 1440
ttttttagga agtttggtga agagttaagg tttgttggat ttgaggttgt ttggggtgtt 1500
aaatagattt atttatgagt atatttggtt aatatattta agtttaatag tgggtgggat 1560
ttttggttgg ttttgatttt ttttatagtg tgtgtaaatg ggtatttatt agattttttt 1620
tgtttgtttt tttatttgta aattttgaaa ggagagtatt ttttggggtg attttgaaaa 1680
ttatgttaga attgaaaagg tttgtgtaaa tgtgaggtgt aatgatgatt tattagaagt 1740
agttagtttt gtatgtgttt gtaggataga tgttagaatg ttttataaat gtgtgtatat 1800
atatattatg tatgtgtgaa ggtgtatatt tgtttttaga tatttgataa atgtatatat 1860
gtatgtgtgt atttttggtt gagagtaaag gggttaatat agggtttata taggatatta 1920
ttttgtagtt ttgtgtgtgt atagtatata tgtgagtatg attgtttgtg tttggagata 1980
ttgtatggta ttggaggagt tgtttagttg ttggtgttat ttgggaagta gggttttggg 2040
tgttgttttg gagtgtggga aagtttggat ttgggtttgt tggttagtgt tttggtgttg 2100
ttgggttttt ttattttttta tttgttggtt ggtgagtggt tttagtttta agatttttga 2160
ttttgttggt gaggggaggg agtgagaagg agtgtgtttt gggtttaaga ggttgtaggg 2220
ttttattttt taggtttgtt gtttttttttt tatttttttta ggttatattt ttatttaaaa 2280
taaagaaggg tgtttgattt atttaggtta gggaagttgg tttttgggag tttttttgttt 2340
ttataagtta tggtgaaggg aggtgaagtt agggtttgtt atggtttggg agaaaatgtg 2400
gttgtagggt ttttttgggt tgtagtgttg gtttggggtt ttaagattgt taaggtgtgt 2460
gtgggaggtg tgtagtgtgg ttattgaaga gttttttatt attgtattgg taggttgttg 2520
ggtttgtttt tttttttatgt ttttaagggt tttgagttgt gtagtatttg tattatttag 2580
aagtgtttgt ggagaaaatg attttaggtg taagtttaag gttgttgttt gtaaaggtaa 2640
tgttgtggag attgggtttt atagtgattt gggtttttaa gttattgaaa gttatagggt 2700
agggttataa atattttttt gttttttttg tagaattgtg tggttgatag gagagtgttg 2760
tgtagaaaat ttgaggttgg gtgttggagg agtttttgtg gtttggagaa ggtatagagg 2820

tgtttttgag aggtgtagtt ggaataggtg atgtatgggt ttggatttgg ttggttaatt 2880
gagtttagtg gttgtgaaaa ttagagttgt tatagaggtt gagtatgatt ttatttttgg 2940
ggattgggtt tggtggggag ttattgtttt taatgttttt agagatttta agtaggataa 3000
tataatgttg gtaggagtaa ggtgtaagga atttagtggt agaagttttt tggggggggtg 3060
gggaaaggta gatttgtgtt ttgtttgagt ttgggggtgg ggatagtttt tggttttgta 3120
gtttttgttt tggggattag ttgggtttat ttaatttttt tatatggggt tgaaaggggt 3180
taatgggatg gttttgttgt tttttttttg ggattttata gggttttgat ttggttttta 3240
tttttgtatg gttagtagtt gtgggggttt taggaaggag gataaaggtt tggtgttatt 3300
gtgggtgggg gtttggtttt ttggtttttt gtttatattt atagttttta gtggttgttg 3360
ggggaagatt tttaaaaata tgtgttgaat tttttttttt tttttttag aaataaataa 3420
ataaaaaagg taaaaggtga attttttttt atttttgtt tatagagatt aaagtttttt 3480
gggattttgt tttttttttt tttttgattg tttagaaata tttgtttttt tttgtatata 3540
gaggaaaatg tggggaaagg ttttttaaaa tttggtttta tattattatt attattaagg 3600
ataattgggt aggttgaggt tttaatgtgg atgatttgag ttggttttgt gttggggttt 3660
tgtagttatt gttttgtgtg tttagtattt ttgggggtga ttagggtttt tgtgtttttg 3720
tttgttgttt ggtagttgag agtattttgt gtttagattg gttgatttat tttttttga 3780
atttgtttta gagttggtaa gggggattta gtttgtgttt taagatttgg gtttgtagtg 3840
ttgttaatag gtttggggat atgaggtgtt ttaggttggg gtttttttgg ttgttggttt 3900
tttttgtttt ttatttgttg gtggtgtttt ggttgtttgt aattgattta atttgttttt 3960
tgtgtttgtt ttttaggttt tttgtttttt tataaaggtt taggggagtt ttgtttatag 4020
gttgattttg taatttttgg tttggtggtt atttttgtt tttttgaaaa agaaaaggaa 4080
aaaaaaaaaa aaaaagaaaa aattaattag ttgagggagg tgtgtgagga ttggaggtgt 4140
tagttggata gtttatgagt aggtttttgg gttggtgttg ttttgtgggt tagtatggtt 4200
tttttagag aaaatttttt aaatgtgtga agattgtttt gggggaagtg agagggaggt 4260
tggaggagtt ttgggtgggg ttttagtgtt tattagttgt gtttttaggg tttgggtgtt 4320
tgttgtaatg gtaattgtgt gagttttatt tttatggtta aggggttagg gtagggtgga 4380
tgtaattgtg tgtgtttggt tttggaaggt gtttgtagg 4419

<210> 262
<211> 4419
<212> DNA
<213> Artificial Sequence

<220>
<223> chemically treated genomic DNA (Homo sapiens)

<400> 262

tttgtgagta ttttttgggg ttaggtgtat gtgattgtat ttattttgtt ttagtttttt 60
ggttgtggga gtgaggttta tgtggttgtt gttgtagtga atatttaagt tttgaaggta 120
tagttggtgg gtgttgagat tttgtttggg gttttttttaa tttttttttt gttttttttta 180
aagtggtttt tatatgttta ggaggttttt tttgagaaag gttgtgttga tttgtgaagt 240
ggtattgatt tagggattta tttataggtt gtttggttgg tgtttttagt ttttatgtgt 300
tttttttgat tggttgattt tttttttttt tttttttttt tttttttttt ttttagaaaa 360
gtagaaggta gttattgggt tagaggttgt agggttagtt tgtgggtgag gttttttttag 420
gtttttgtgg agaaatggga aatttgaggg gtaaatgtag gaagtgggtt gggttaattg 480
tgggtgattg aggtgttgtt agtgggtggg gagtgagagg ggttagtagt tgggaagatt 540
ttggtttgga gtgttttgtg tttttgggtt tgttggtggt gttgtaagtt taggttttgg 600
ggtgtgagtt aagtttttttt tgttagtttt aaataaaatt tgggggagaa tgagttggtt 660
agtttgggta tagggtgttt ttgattgttg ggtggtgggt ggaagtgtag gggttttgat 720
tgtttttaga ggtgttgagt gtatagggta gtggttgtag agttttggtg tgaggttaat 780
ttggattatt tatgttggga ttttagtttg tttggttgtt tttagtaata ataatagtat 840
gaaattgagt tttaaaaaat tttttttgt attttttttt atgtataagg gagaataagt 900
atttttaggt agttaaagaa aaaaaaaggg taggatttta ggaaatttta atttttatgg 960
atgaggagtg aaggggaatt tgtttttttgt tttttttgtt tgtttgtttt tagaagagaa 1020
aaaaaggaaa tttgatatat atttttaaaa attttttttt aataattgtt aaaggttgtg 1080
agtgtgagta gagagttagg aaattgagtt tttgtttgtg gtgatattgg gtttttgttt 1140
tttttttga agtttttgtg attgttgatt gtgtagagat aaaggttggg ttagggtttt 1200
gtggaatttt gagggagggg tggtggggtt gttttattgg tttttttaa ttttatgtag 1260
gggggttagg tgggtttagt tgatttttgg aataggggtt ataaggttag gggttgtttt 1320
tatttttagg tttagatgag gtatggattt gttttttttt atttttttga aaggtttttg 1380

ttgttaaatt ttttgtattt tgtttttatt agtattatat tgttttattt aaagtttttg 1440
gaggtgttag ggatagtggt tttttattgg atttgatttt tagaaatgga attgtgttta 1500
atttttgtgg tgattttggt ttttgtgatt gttgggtttg gttggttggt tagatttgaa 1560
tttgtgtatt gtttgtttta gttgtgtttt ttaggggtgt ttttgtgttt tttttgggtt 1620
gtggagattt ttttagtgtt tggttttgaa ttttttgtgt aatgtttttt tgttagttgt 1680
atggttttgt agagaaggtg ggagagtgtt tgtgattttg ttttgtagtt tttggtgatt 1740
taaaaattta ggttgttgtg gaatttagtt tttatggtat tgtttttgta ggtagtagtt 1800
ttgggtttat atttgaggtt gtttttttta tagatatttt tgggtgatgt gagtgttgtg 1860
tagtttagaa tttttggaag tatgagagga ggatgagttt ggtggtttgt tggtgtggtg 1920
atggagggtt ttttggtagt tatattgtgt gtttttttata tatattttaa tagttttggg 1980
gttttgggtt agtgttgtag tttaaagaga ttttgtagtt gtattttttt ttaggttgtg 2040
gtaaattttg gttttgtttt tttttattat ggtttgtgag ggtaggaggt ttttgggggt 2100
tgattttttt gatttgaatg agttaggtat ttttttttat tttaagtggg ggtgtgattt 2160
gaggagataa aggaaggatg atagatttgg ggagtggggt tttgtagttt tttgggtttg 2220
gagtgtattt ttttttgttt ttttttttta ttggtaggat tgggggtttt gaaattggag 2280
ttgtttattg gttggtggat gagagataaa ggagtttggt ggtgttgaag tgttgattag 2340
tagatttgga tttggatttt tttgtgtttt agagtagtat ttgaaatttt atttttttagg 2400
tggtattgat ggttgagtag tttttttggt gttatatggt gtttttaggt atagatagtt 2460
gtgtttgtgt gtgtattgtg tatgtataag attgtaggat gatgttttgt atgagttttg 2520
tattagtttt tttgttttta gttagaggta tatgtatatg tgtgtgtatt tgttaagtgt 2580
ttagggatag gtgtgtattt ttatatatat gtgatgtgta tgtgtatata tttgtagaat 2640
gttttagtat ttattttata ggtatgtata gggttggttg tttttaataa gttattatta 2700
tattttgtat ttatataggt ttttttagtt ttggtatgat ttttagaatt attttgggaa 2760
atattttttt tttaaagttt atagatgagg aaataagtaa aaagaattta atgggtattt 2820
gtttgtatat attgtgagag agattgaggt tagttaaaga ttttgtttat tgttgaattt 2880
gggtgtgttg gttgaatgtg tttatagatg ggtttgttta gtgttttagg tgattttagg 2940
tttggtgggt tttggttttt tgttaggttt tttgggagat ttattgtgtt gggtgtgggg 3000
ttttaatttt tgtgttgata agtattattt tttggagttg ggtgatttta gtgtttaggg 3060
tgggaagttg ggagttggtt ggagtgttgt tgaggtgatt ttggtggttt taaggttatg 3120
gttgattatg tgtaattggg ttgtaaagtt tggtgggttt tggtttttaa agtagttatg 3180
ttaggtaaat gtgtgatgaa aggatttttt tagtttattt ataaatttag gaatttaggg 3240
ttgttggagt agtgaggaaa tttttgttgt tttgaatttt tagtgttttt atgagatttg 3300
gagtttataa tgtttatttt atagaagtga ttagttggag gttagagtgg tatagtgagt 3360
tgtttaagtt taatgttagt tgagtggtta tagttgggat tggaattttt aattaagtgt 3420
gtttggattt ttggtttata gtttttttgt ttttagtaag gattgttggt tttttttgttt 3480
tttgtgtgtt taggagttat tttgttatta gtttttgatt tttttttgtta agaatgtgaa 3540
aattattata gtttttatta gtattattta aaaggttttt atatgtttgg aatttgttgt 3600
ttgaaaataa attttttgtg tgtgttggtt ttttaatttt ttttaaggta tttggttttt 3660
ttttattttt taagtttttg ttgttgtaga gtgaggttgt ttaagggttg tagtttgatt 3720
atattttggg ggtgtttttg ggtttttgtg ttgggttttt gtagattgag gttgttggtt 3780
tttgggtttt aaggttgttt ttattttgta gaatttggga gttggaaggg agtttgtggt 3840
ttggtgaggt tgttgtttgg gtttatggtt ttgaggttgt tttttgttgg tattgggtga 3900
tattattatt gttttttttg gtggtttagg agttgtattt atgtgagggg tttgtgtaga 3960
gttaggtgat tttattttta ttgggttttt agagtggttg ttgtatttgt agttattgat 4020
gagatttttt tgtatttttt aatttgagat ttttttttgt agatattaga ggttttggtg 4080
tttggattta gggataaggt agagagttgg agttttgtgt tggtaatttt gttgttttgt 4140
ggtgtttgtt gggatttgtg gttaattagg tatggttata ggtggatgtt gggggatttt 4200
ggggatggtt ttaggttgtg tttgtttttt ttattttttg ttattatagt attgattggt 4260
gtttatgggt tttgatttat ttgttttttt tttttaatt ttatttgatg gtggtttagg 4320
tgtttggttg tttttgtttt ttgtagtgta tttgtttttt taattaaatt aagagtgttt 4380
tgatgattgg aaggggttgt tttttatttg tatggtata 4419

<210> 263
<211> 1918
<212> DNA
<213> Artificial Sequence

<220>
<223> chemically treated genomic DNA (Homo sapiens)

<400> 263

```
tattggatat gattattgta tataatatag aagatattaa gggttaagta atgatgtata      60
aatggggttt attgtttaaa tttatagaaa gtaaaatgtg gaaaattagt aattttaatt     120
gatagtgtat atttaaagaa tttatattgg ttgggtgtgg tggtttatgt ttgtaatttt     180
agtattttgg gaggttgagg tgggaggatt atttgaggtt aagagattga gattagtttg     240
attaatatgg tgaaattttg tttttattga aaatataaaa aaattagtta ggtgtggtgg     300
tatatgtttg tagttttagt tatttgggag ggtgaggtag gagaattgtt tgaatttagg     360
agatagaggt tgtagtgagt tgagattgtg ttattgtatt tgtttgggag atagagtgag     420
attttgtttt aaaataaaaa ataaataaaa agaatttatt taatagaatt aagtattaat     480
ataataaata tgaagaattt tagatttttg gtttttaaaa aatatataaa gatgatattt     540
ttttaaaata tttttataaa atatattgag attgtgatgt tttatattga ttgtatgaaa     600
ataatgaaaa agaattagta ttgttttatt ataaaagttt tattaatgta aatttataaa     660
ttttttttta aatattttga gttaatttta attttatgat agaaatttat tatttttagt     720
aaaaatagtt ggtatttggg aaattaaagg tttaaaaatt aagaatagta attaaagaaa     780
tttgataaaa tagttttttt aaaattttta tttatattat aaggggaaat tttgattatg     840
tttttttttt tttattaatt gtagaattta atattaagga ttatataatt ttatattttt     900
ttttgagaaa aagtaaaggt tttgtgttgt agtaataatg taagatatgg agggaagttt     960
tatttaagat tttttgtttt gttttttttt taaagttatt ttagaatatt agggagggtt    1020
gagaggtaag gtatgaaggg tgtaatattt aatatgagta atgtgtgtga tgtatttggt    1080
taaaatgtat atagaggatt tgtttttgtt tttagataga agtttttttgt tttgtagtta    1140
tgagggttaa ttgttgaggt tttatagttt tttttttttt tatatttgga ttgttatgtt    1200
ttttatttat tattttgatg tagaggtaga tttaggattt ttgtatttgt taaggatttt    1260
ttggtaagtt tatggggtgg gagtggttat aagatggagt ttgtttggtt ttggtttttt    1320
tggtttatat aagtttgttt ttttttaat ttttaatttt tatagttttt tatttttttta    1380
tttttgattt attttgtgtt attgatgttt ttggtttttg tttgtagtaa gtttattttt    1440

attattattt tttgtataaa agtttgtatt tattaggtta aagaggggaa ttaatgtttg    1500
taggaattgt tttattgaat tgtttggttg tgttttttgt tagattttat ttgttgttgt    1560
ggattgtata taattatttt tggtatgttt tgtgtatgta ttattttttt tattttgttt    1620
ttttttgtt taaatatgtg attttttttt gtttttgttt atgtttattt ttgttttttt    1680
attttttttt aggaaggagg agggagttgg gggtgttaaa agtgtagtga tttttttttt    1740
ttttttgttt ttgtttttgt atttttttgtt ataatgtttt ttgggttgtt agtgttttga    1800
tgtttttttgg gaaaatagtt tatttttttt tttttttttt tttttgtttt taattaatta    1860
gttatttgtt agagagggat atgtgtagtg agtgtttttt gtttttttta tttgaatt      1918
```

<210> 264
<211> 1918
<212> DNA
<213> Artificial Sequence

<220>
<223> chemically treated genomic DNA (Homo sapiens)

<400> 264

```
ggtttgggta gaagagatgg gaggtattta ttgtgtatgt tttttttga tgggtggttg      60
gttggttgaa ggtaggagga gggggagggg aggaaatgag ttattttttt agaaggtgtt     120
gaggtgttag tgatttggaa gatattgtag tgggaggtat aggagtgggg gtggggagga     180
ggaggaagtt gttatgtttt taatattttt agtttttttt tttttttttaa gggaaagtgg     240
aggaatggaa gtgggtgtgg atggagatga aaggaggtta tgtgtttggg tgggagaggg     300
gtggggtggg agaggtagtg tgtgtgtggg gtatgttggg agtggttgtg tatggtttgt     360
agtggtaggt gaagtttagt agaggatgtg gttaggtgat ttggtgaagt gatttttgta     420
ggtgttggtt tttttttttg atttggtaaa tgtaggtttt tatgtgagag gtaatggtgg     480
gggtaaattt gttgtaaatg aaggttaggg gtgttggtgg tgtaaggtga attgaaagtg     540
ggaggatgga aggttgtgga gattgggaat tgggaagggg gtaggtttgt ataggttggg     600
aaggttagga ttaggtgagt tttgttttgt ggttattttt gttttgtgag tttgttgagg     660
aatttttgat aagtgtaggg attttgagtt tatttttgta ttggggtagt aggtgaggag     720
tgtgatggtt tgagtgtaag agagaaggga attgtgaagt tttagtaatt gatttttatg     780
attgtaggat ggaggatttt tatttaggga tagagataag ttttttgtat gtattttgat     840
tagatgtatt atatgtgttg tttatattgg atattgtgtt ttttatgttt tattttttaa     900
ttttttttgg tattttggag tggttttggg gaaggagtag gtagggaagt tttgagtgga     960
```

gttttttttt atgttttgtg ttgttgttat aatataaagt ttttgttttt ttttggagag 1020
ggatgtggga ttgtgtagtt tttaatgttg agttttatga ttgatgaagg agaagggatg 1080
tgattaaagt ttttttttat agtgtagatg agagttttaa aaggattgtt ttgttaagtt 1140
tttttggtta ttatttttag tttttgagtt tttggttttt taaatgttag ttgtttttgt 1200
tgaaaataat gaatttttat tataaaatta gaattaattt aaaatattta agaaaggatt 1260
tataaattta tattagtaaa gtttttatag tgaaatagtg ttggtttttt tttattgttt 1320
ttatataatt aatataaagt attatagttt taatatgttt tgtaaagata ttttgaagga 1380
atattatttt tgtatgtttt ttaaaaatta agaatttaaa atttttgta tttattatgt 1440
tggtatttaa ttttgttggg tgggtttttt ttgtttgttt tttgttttga gatggagttt 1500
tgttttgttt tttagatgag tgtagtggtg tgattttggt ttattgtaat ttttgttttt 1560
tgggtttaag tagttttttt gttttatttt tttgagtagt tgaaattata ggtatgtgtt 1620
attatgtttg gttaattttt ttgtgttttt agtagggatg gggttttatt atgttggtta 1680
ggttggtttt gattttttga ttttaaatga tttttttgtt ttggtttttt aaagtgttgg 1740
gattataggt gtgagttatt gtgtttggtt ggtgtaggtt ttttaggtgt atattattag 1800
ttaaagttat taatttttta tgttttgttt tttgtaagtt tgggtagtgg attttgttta 1860
tgtattattg tttagttttt agtatttttt gtattgtgta tggtaattat gtttgatg 1918

<210> 265
<211> 13670
<212> DNA
<213> Artificial Sequence

<220>
<223> chemically treated genomic DNA (Homo sapiens)

<400> 265

agtgttggga ttatatgagt gagttattat gtttggtttg ggtttttgtt tgtttgtttt 60
taagatatgg ttttgttttg tttttaggtt ggagtgtagt ggtatgatta tagtttattg 120
tagttttgat tttataagtt taagtgattt ttttatttta gtttttgag gagttaggat 180
tataggtata tgtttttatg tttagtagtt aaattttttt tttttttttt aagagataag 240
gggttttgta atgtttttaa ggttggtttt aaaatttttg gtttgttgtg attttttttt 300
tttggttttt taaagtgttg ggattgaagg tatgaattat tatgtttgtt ttttagtagt 360
ttgtgatgtt aggaattata tttttatttt ttgatgtttt tttttttttt attgttgggg 420
tattattttt gtttggttta ttttttattt tttttttttt tttttttttt gagatggagt 480
ttagttttgt tatttaggtt gtagtgtagt ggtgtgattt tggtttatta taatttttat 540
tttttgggtt taagtgattt ttttgtttta gtttttttgag tagttgggat tataggtgtt 600
tattattatg tttggttaat atttgtattt ttagtagaga tggggttttg ttgtgttgga 660
taggtttgtt ttaaattttt gattttgtgt tttttttgtt ttggtttttt aaagtgttgg 720
gattataggt gtgagttatt gtgtttagat tttttttttg agatggagtt tttttttttt 780
gtttaggttg gagtgtagtg gtatgatttt ggtttattgt aatttttgtt ttttgggttt 840
aagtgatttt tttgttttag tttttgagt agttgggatt ataggtatgt gttattatgt 900
ttggttaatt ttttgtaatt ttggtagagg tggggtttta ttatgttagt taggatggtt 960
ttgatttttt gattttgtga tttgtttgtt ttggtttttt aaagtgttgg gattataggt 1020
gtgagttatt gtgtttggtt ttattttttta tttttgtggt tttttttttt tgtttgtttt 1080
tgaggtgttg ggggttttta gggtttagtt tttagttttg tttttttattt tgttttaggg 1140
agattttatt tttgttttta gtttaagagt ttttttgttt tataatatat tgatgtttgt 1200
tgggttattt ttgtttttag tttataatat gtttttaagt ttttattttg ggatttagtg 1260
ttgttagatt gaatatattt taaatttgtt tttgtatttt tttagtgtat ttgttttttt 1320
tttagtgttt ggatttgagt tttgtttttt attgtttgga ttatgtttat tttttttttga 1380
ggttttttttg tttttttggtt tggttttttt attatttttt aagtaatatt ttagatatat 1440
ggatttgttt gagatttttt tttgttggta tgtgaagttt tttatagttt tatgtttttt 1500
tagtttaaat ttttttttta gttttatgga aatgttaaat tattttggtg tgggaggttt 1560
ttgtgggttg gggtgtagtt ttgtaaggtt tagttttaag attgtttttt ttagggagtt 1620
tttttttttt tttttaggt aagtgttttt ttttagttgt ttattttatt agggtttgat 1680
tatattttgt tggtagtgtt agagtttggt tttgtttttt attatttttt ttagaattgg 1740
tggtagggat agatgaatgg atgaatgaat gaatgggaag tgagaatttt attattggat 1800
tattaatgta gtttttggaa tgagtgagtg agtgagtgag tgagtgagtg agtgatggaa 1860
agtggtttgg aggtagggag agggataggt tgattatgtg aggaatttta gtttaagttt 1920
ttgaggtttt tgtttttgtt ttaaaagatg tatgtgttaa gtttttgggt tagaaaaaaa 1980
aaaaaaaaat gggttgggat tggtttaggt tgggggaggt gggtttatga tgagatatga 2040

```
tttttattg gtttatttgt taggatttg tttaattgat gtgattgttt tttttagaag    2100
atttttgttt tttttttgtt ggtggttgtg gaggtggaag aaggtgtttt tttgttgttt    2160
tagtaattgt tagtggattt ttgttgtttg ggtaattgtt ttaggttttt tgttagtttt    2220
tgtttgggtt tttttggtta tttggataat tgttatttgt tttggttaat tattaatttg    2280
tttttgtta ttttggtaat tattgttgga tttatttatt tttggatgtt gttttggttt    2340
tgtttgtagg tttttttgtt ttaaattttt gttgtgggtt ttgatgtttg tgattttatg    2400
ttttaggggt tttttttttt ttttgtttgt tttttttgttt ttttggggtg gggggtattg    2460
tttttttttt ggtagggggg ttttgggttg gatttggttt tgggggtttt tgtttggttg    2520
ggatagtggg attgttttgt gttgtggttg tgtgtagttt tttgtaggtt ttggtgtttg    2580
tatttttgtg tgggattgtt taattgttat agggagtttt ttaaaatgta agttatgttt    2640
tttttttgtt gaaaattttt ttttggtttt ttttgagaat tgaatttaag tttttttgtag    2700
tttttggtga tttggttgtg tggttttttt tttgaagttt ttttgtttat tttgttttag    2760
taaggttttg gtttgtgtgt tgtaatttta atgatttaag ttttttttttg tttgggattt    2820
ttttagttat tattttttat tttattaaaa agtgtatttt agatgggtgt ggtggtttat    2880
gtttggaatt ttagtatttt gggaggttga ggtgggagga ttatttgagt tgaggagttt    2940
gagattattt tgggtagtat agtgagatgt ttgtttttat aaaaaatttt tgggaggttg    3000
aggtaggtgg attgtttgag gttaggtgtt ggagattagt ttggttaata tggtgaaatt    3060
ttgtttttgt taaaattata aaaattagtt aggtgtggtg gtgtgtgttt gtaattttag    3120
ttattgggga ggttgaggta ggagaattat ttgaatttag gaggtggaag ttgtagtgag    3180
ttgagatggt gttattgtat ttttgtttgg gtaatagagt aagtttttat ttttaaaaaa    3240
aaaattaaaa attagtttgg tatggtggtg tgtttttata gttttagtta tttaggaagt    3300
tgaagtagga ggattgattg agtttaggag gtagaggttg ttgtgagttt atgttatatt    3360
attgtatttt agtttgggta atagagtgag attttgtttt aaaataataa taaataagtg    3420
tatttttttag ttagggtttt atttgtgagt ttttagtgaa gtttttttttg agttttgtta    3480
gtttttttttt ttttaaattt tttttggttt atattttgtt attaggattt agaatattgt    3540
ttggtattta tgttgagtaa aaagatgttg tgttaggtgt agtggtttat gtttgtagtt    3600
ttagtatttt gggaggttga ggtgggtgga taatgaggtt aggaatttga gattagtttg    3660
gttaatatgg tgaaatttta tttttattaa aaatataaaa atgggttagg tgtggtggtt    3720
tatgtttgta gttttagtat tttgggaggt tgagatgggt gaattatgag gttaggagat    3780
tgagattatt ttggttaata tggtgaaatt ttgtttttat taaaaatata aaaaattagt    3840
tgggtgtggt ggtgggtgtt tgtagtttta gttatttggg aggttgaggt aggaaaatgg    3900
tgtgaatttg ggaggtggag tttgtagtga gttgagattg tattattgta ttttattttg    3960
ggatagagtg agattttgtt ttaaaaaaaa aaaaaaaaaa aaaaaatata aaaatgagtt    4020
gggtgtggtg gtggatattt gtaattttag ttgtttagga ggtagagata ggagaattgt    4080
ttgagtttgg gagggggttg tagtgagttg agattgtgta attgtatttt agtttgggtg    4140
atagagtaag attttatttt ataaataaat aaataaaaaa aggatattgt gttggggata    4200
agggaggata gagggggatg attagtaggt aggtttattt tagagatagg agaatgtatt    4260
ttttagggtt ttttagtttt tatggtatag gtgatgatgg taatattttt aaaaagttag    4320
gtataagtta gggagatata agttgatgtt atggttagtt tgagattggg tggtgtattt    4380
tttatatata attgttattt tttatttatt tttattttag agggttttag agttagtgga    4440
aattgtggtt aatttggagt agaagttttt aggagttgga gaggtaggtt ttagatttta    4500
gatggttttt ttgggagttg gtttttttttt gtttggaatt tagaatttta tttgttttaa    4560
tagagatgag agttgttttt ttagggattt gagataggtt agtttaggtt ggggtaagag    4620
aatttgattt gattaggttt ggggttatga tggggggtga gggaaggggt ttgtgggagt    4680
tttttggga ttggttggag aggaatgagg aatttgtata tttatttgtt tgtttatgag    4740
ggtttgtttt tggggaggtt gttaggggtt aggtagggag gttttttgtt tttttttttt    4800
tttttgaga tggagtttg ttttgttgtt aggttagagt gtagtggtgt gattttagtt    4860
tattgtaagt tttatttttt gggtttatgt tatttttttg ttttagtttt ttgagtagtt    4920
aggattatag gtgtttatta ttatgtttgg ttaatttttt gtatttttag tagagatagt    4980
attttattag gttagttagg atggttttga ttttttgatt ttgtgatttg tttattttag    5040
ttttttaaag tgttgggatt ataggtttga gttattgtgg ttggttttga tttttatttt    5100
taagttagga ttatttttat tagtgttgtt atatataatt ttttttattg atgtggtaat    5160
tgagtatttg aaatatagtt ggtatggtta ggtatagtgg tttatgtttg taattttagt    5220
attgtgggag gttaaggtgg gtagattatt tgaggttagt agtttgagat tagtttggtt    5280
aatatggtga aattttattt ttattgaaaa tattaaaatt agttaggttt ggtgatgttt    5340
ttgtagtttt agttatttgg gaagttgagg tataagaatt gtttgaattt ggtaggtgga    5400
ggttgtagtg agtttagatt gtgtttattg tattttattt agtttgggtg atatagtaag    5460
attttgtttt aaggggaaaa aaatgaaata tagttggtgg gattgagtat tgtattttta    5520
ttgtatttta ttttaattaa tatatattta agttgaagta gttaaatgtg gttagtagta    5580
ttgtatttag agtagtattg agttgaagat ttaggtagga ggttgaaggt agggatagga    5640
aggttagggt ttaaggtttg tgtagttatg gtagagaagt gattttggta aagtgtagag    5700
```

tgaggtttta ggaagggagg aggtgggata tttggttttg gtatttggtt ttattggaag 5760
agaggtatat ttagttttta gggttaaagt gtttgttata tatatatgta tttatgtttg 5820
tgtgtgagta taggggttgg tgtgttttga ggtatttata ggtgtgtttg tgtgggattt 5880
aggagtttta tgtttatgag tgatttaaga gtttatttat gtatggatat tgtatggatt 5940
tttaggtata tatagataat gggtgtgtgt ttttttttga gtatttatgt tttttgggta 6000
ggttagtaag gattattagt ttagtaggtt ttgtaagaag gtttatatgt attggtgtat 6060
tttttggggg ttgttttgtg ggatttaatg ttttatgttt ggtaggatgt gggtttttaa 6120
gtggtttggt ataaagtggt tgttgatggt ttttattagt tttagtttta agtaagtgtt 6180

ttttttttt tatagtagta atgtgggtgt ggttagtttt tggggtttta gggggaagtt 6240
tttgtggtta ggatagatag ataggtagat agataggtag aaggatggtt gtttttttatt 6300
tttttgtttt tgtaaatttt ttgagtttat gtttggtaat tggttttatt tgaagaggtt 6360
ttggtagtag ttgaggggtt tagtgaggtt attaggttgt gagaagttat aaaggaaggt 6420
tttgagtttg ttgggggtta agtatgggat gtttgttttg tggtgggtga gggtggtttt 6480
tagaatttag gtatatagta ggattttggt tttagtgttt agtttgttta gttttgattt 6540
gttttttttt tttttttaat ttgtattttt tgtatttgaa agttagatat agatagtagt 6600
tttttgggta gttagggtag ttggaatagg aatatgtagt gggaatggaa gaattggttg 6660
atgtggtgta gggaatagtt tggggtggga gggttggggg agagatataa ggtttgtttt 6720
tggggtggtt ttatatattt gtatttttat atatatttag gttagggata taagttttag 6780
gaatgttatt gttgtttgtt tgggtttgtg aaggtattag atattttttt tggtttgggt 6840
attagttata tttttttttg ttatgtttta ttatttatag gatgggggta ttttttggga 6900
atttttatat ttaaaggtgt gttttttttg tttaggttag ggtgtgtttt ttttgtttta 6960
tttagttaat atttattgag taattattgt atgttaggtt ttgttttaga tatagtagtg 7020
aatgagatag ttaaaatttt ttgtttattt taggtaattt aaggaaattt ttgtatttat 7080
aattttttttt tttttttttt tttttttttt tttttgagat agagttttgt tttgttgttt 7140
aggttggagt gtagtggtgt gattttggtt tattgtgagt tttgtttttt gggtttatgt 7200
tatttttttg ttttagtttt ttggtagttg ggattatagg tatttgttat tatgtttggt 7260
taattttttt tgtattttta gtagagatag ggttttatag tgttagttag gatggttttg 7320
atttttggat tttatgattt atttgtttta gtttttttaaa gttttgggat tatagatgtg 7380
aattattgta tttggttttt aaaaaataat tttttaaaaa ttagtagggt gtggtggtgt 7440
gtgtttatag ttttagttat ttgagaagta gaagtgggag gattgtttga gtttaggagt 7500
ttgaggttgt agtgagttgt aattatagta ttgtatttta gtttggatta tagaataaga 7560
ttgttttaaa ggaaaaaaaa taaaatataa aaaaagatat ttttaaaaga taaaaaattt 7620
tttgttttta gggagttgat agtttagatg gggagataga taagtaaata ataataggtt 7680
gggtgtggtg gtttatgttt gtaattttag tattttggga agttaaggtg ggtggattat 7740
ttgagattag gagttagata ttaatttggg taatatagtg aaaatttgtt tttattaaaa 7800
atataaaaat tagttgggtg tgttggggta tatttgtagt ttttgttatt ttggaggttg 7860
aggaaggaga gttatttgaa tttaggaggt agaggttgtt aggtagggtg gtttattttt 7920
gtaattttag tattttggga ggttgaggtt ggtagattgt ttgagtttag gagtttaaga 7980
ttagtttggt taatatgttg aaattttatt tttattaaaa atataaaata attagttggt 8040
tatggtggta tatatttgta gttttagtta ttgaggaggt tgaggtaaga gaattatttg 8100
aatttgagag gtggaggttg ttgtgagttg atattgtgtt attgtattgt atagagtgag 8160
attttatttt aagaaaaaaa aaaaaaaaag gggttaggtg tggtggttta tgtttatatt 8220
tttagtattt tgagaggtta aggtgggtag attataaggt taggagatta agattatttt 8280
ggttaatatg gtgaaatttt gtttttatta aaatataaaa aattagtttg gtatggtggt 8340
ttgtaatttt agttatttgg gaggttgagg taggggaatt gtttgaattt gggaggtgga 8400
ggttgtagtg aattgagatt atgttattgt attttagttt ggtgatagag taagattttg 8460
ttttaaaaaa aaaaaaaaaa aaaaaaaaaa gaaaagaaaa gaaaaagaaa aaaaataata 8520
aaagagagag agagagtata gttatgatta tagtgttgta tattaatatt aggtgatatg 8580
atagatattg ttaatttttt tagttttagt ttttttagt tggaatatgg ggataatata 8640
atttttataa gtttaggagt tttaagtagg ttattgtgtg tatatttttt gttattgtaa 8700
gtgttataaa aaaatgtgga ttatttttaa gtgtattgtg atttatttta ttttatttta 8760
tttttgagat agagttttat tttgttattt aggttggagt gtagtggtgt gattttggtt 8820
tattgtaatt tttatttttt aggtttaagt gatttttttg ttttagtttt ttgagtagtt 8880
gagattataa gtgtgtatta ttatgtttgg ttgatttttt tgtattttta gtagagatgg 8940
ggttttatta tgttggttag gttggtttttg aatttttggg tttaagtaat ttattggttt 9000
tggtttttta aagtgtaggg attataggta ggagttatta tgtttggtta tattttattt 9060
tatttattta ttttttgaga tggagttttg tttttgttgt ttaggttgga gtgtaatggt 9120
atgattttag tttattgtaa tttttatttt ttaggtttaa gagatttttt tgtattagtt 9180
ttttgagtag ttgggattat aggtatgtat tattaatgtt tggttaatta tatttttttt 9240
ttttttttgt agagatagtt ggggttttgt tatgttgatt agattggttt tgaatttttg 9300

gttttaagtg attttttat tttagttttt tggagggttg ggattatagg tgtgagttat 9360
tgggtttggt gtgttattaa tttttgaatg aggagtttta taattgaagg gatttgggtt 9420
gattgttgta tttttagttt ttgatgtgtt gataggtata tagagaattg aaaatataga 9480
tatatgtttt tgttttttg tatataagta tattttagtg ttttggattt ttatatatat 9540
tttggtatat tgatatgggg gtattattga ttataattat ttgtttgatt agggatgggt 9600
agtagatgga gaaatgttag gtaaggaggg tattttagtt atgggttata aggatgtatt 9660
ttgagtaatt tgtgggaatt taaggagttt gtataagtga ggtgtttagt aggattgaag 9720
tagggatggg ggaagtaggt tttagtaagg agaaagtggg ggtatgtgtg tgtgtaggat 9780
gagggaggtt atgtttagga tgggggtatg tttgtattta ggtttaggat gatattttg 9840
atgtttatta gtagtaggtt gattgtgtag tagtttatat tttgaggtgt atttgagggg 9900
ttatagtttt gtaagtttat agttataata tggaagtggt tttggaattt ttggagttgg 9960
taatgttagg agaatttgtt aggtgggtga gggagggagg ttgagttagg gtttttaggt 10020
tgtatttgta ttttatgtat attagtaatt attggtaaat ttttttttt gttttaaatt 10080
ttttttaaaa ggaggatggg gaattttagg tagaggtaga ggttgaggtt gaagttagtt 10140
atattttgaa tttagggaat tttaattaat tttaatattt ttttgttttg tttttttagg 10200
gtttagggat tttgggtatg ttggtttagt atttttattt ttatttatgt ttagtttgtt 10260
ttgatttata tagttttttt ttttgttttt tgggagttta agttttattt gtttttgagt 10320
tttgtgggtt ttaggttttt ggttttagat gtattagttt ttagggaagt tgtgtagaaa 10380
tagtatgagg ggtttgttat tttgtttagt tgagatatag tgtagatgta ggtttgagtt 10440
ttagggggag ggtatagttt gaagtttggg gaattttttt ttttgaggtt tgaattgttt 10500
tgattttatg tgattttgta gttttgatag tgtttttgtg tgattatttt gaggtttagg 10560
aaattgtgtt tatttagtga ggggttgttt aggtaggtgg gggatgtgtt ttgatggtgt 10620
ttgtagtagt tgtgttgggg ttggtatagt atgtgtgtga gtgttatgta gttgtagatt 10680
gttgtggtta ttagtgttat tgagaatatt aggttttata tgaaggtgtg tagtagtttt 10740
agtgataggt gtgatggtgt tagtagtgtg gttattatta gttttggtat gttgttgtgt 10800
tttgggatta tggtggtgtt gttggttagg ggtatttgta gtagtggtga agtggtgtta 10860
gggtttaggg gtttattgtt tttggtttgg ggttttttg tgttgttggg atttgtggga 10920
tgtgttgaag gaagaggtgg gtggttttga tagaaaatag ttagagtgta ttatttattt 10980
gagtgttagg taaatatttg ggtgtgatag ggataggaaa taagggtagg gtgtggaggt 11040
tggggaggaa gaggttggaa agggggggaaa taaatgggtg gggtttagta ggttttgtgt 11100
ggggtttagg gttggggtgg ggtttaggaa gatttagtag tgggtgggtg agggtttaaa 11160
ggtggtaatt ttgggttggg tgtggtggtt tatgtttgta attttagtat tttggtaggt 11220
tgaggtgggt ggattatttg agattaagag tttgagatta gtttgggtaa tgtggtgaaa 11280
ttttgttttt attaaaaata taaaaattag ttgggtgtgg tggtgggtgt ttgtagtttt 11340
agttatttgg gaggttgagg taggagaatt gtttgaattt gggatgtgga ggttgtagtg 11400
agttgagatt gtgttattgt attttagttt gggttataat agggaaattt tgttttaaaa 11460
aagaaaaaaa aaaaaggtaa ttttgagttt agataaattt taaggagggg attttggatt 11520
tttagttaag tgggtgatat ttggagtgag gggtggggta tatgtagagt aggtgtggtt 11580
tataagttaa aaaggagaaa gagttggaat ggtgggtttg gtttatgtgg gtgggtgggg 11640
agagggtgga ttttagagga ggtgaggttt aatattgggt gaagaaggtg ggagtttggg 11700
ttaatgagtt gatggtaggt tggggagggg gtggtggggt ggggtgggta atgggtaatg 11760
agatggaggg tggggttggg atttaatatg gtgggttagg agggtttgga agatgaagaa 11820
gagggatagg taatgttagg tttaggatta ggagggaggt gtgggtggta ttagtggttg 11880
gaggaggttt tgggaggttt ggttgatggt tgttgtttgg tgttatttat ttaggggtgt 11940
gtgatttttt ttttggtttg gttttaaaga tttagtagta ttgattttat ttagttgtgg 12000
ttttaatggt gggtttagtg gttttggttt ggtgttgttt ttttgttggt ttaataggtt 12060
tgttagtttg tttttgtatg tttgtgattg gatggtggtg gttattgatg tttaagtgat 12120
aggttttggt ttgggagtta atttgtaggt gttgaggttt aggttttgag agtgggttga 12180
ggaggtgtgg atattttgat ttttaggggg taggtttggt ttttttgagg aggatttggt 12240
ttatgaatga ttggagtttt ggggttttgg ttgaaagagg aagtgggata gggttgggtg 12300
tgatggggtt tagagttata gagttttgtg gttttgttgt ttttgtaaga agttagtttt 12360
tggttaggtg tggtggttta tgtttgtaat tttagtattt tgggaggttg aggtgggtgg 12420
attatgaggt taggagattg agattatttt aatatggtga aattttgttt ttattaaaaa 12480
tataaaaaat tagttaggtg tggtggtggg tgtttgtagt tttagttatt agggaggttg 12540
aggtaggaga atggtgtgaa tttaggaggt ggaggttgta gtgagttgag attgtgttat 12600
tgtattttag ttagggtgat agagtgagat tttgttttaa aaaaaaaaaa aaaagtagtt 12660
agtttttttt ttttattttg taatttttttt ttgatatttt tgaatatttt agggatagtt 12720
attatttaat tatagagtaa ttttattaag tttaagtagt ataattatat ttttgtagta 12780
tagattatga atttgaaatt tgaggatgaa gttatttgtt tgaagatata tattttgtaa 12840
aatagttatt tgtaaagatg tagggaaaaa ggtagtgatt tgtggttata tttttttttt 12900
tttttggaag tagttattgg aatgttttta gttttttttt ttttttttag atggagtttt 12960

```
gattatagtt tattgtagtt ttgaattttt gggtttaagt attataattt gtagttggga   13020
ttataggttt gtgttattat atttagttaa ttttttaaaa attttttaga gagatgattt   13080
gttatattgt ttaggttggt tttaaatttt tggttttaag taatttttaa gtttttattt   13140
tttaaagtgg gattatagat gtaagttatt gtgtttgatt tttatttttt gatttaatta   13200
atgtatgata ttagttattg atttgtttat attattattt tggatgtgtt gtttttttgtt  13260
tttttgttag ttttttttaa aaatatttta tttgttattt tttgtttttt tttatttttt   13320
atttttattt atatattttt gtattgttaa ggttgttaaa tattatattg ttttgggatt   13380
ataattaagt ttatgaagtt gttatttaaa ggtgaatagt ggtttatgtt ttgttttagt   13440
gaataatgta ttttaagagt tgaatagatt attatgatta tattgtttat agtgtataaa   13500
tttttgtttt ttttggatta tttaattatt attttttttt tgttgtgtga ttagtttata   13560
ttaaagtttt gggggttttt ttgtttttgt tttttttgag atgggagttt tattatatta   13620
tttaagttgg aatgtagtgg tgtgattttg gtttattgta atttttgttt              13670
```

<210> 266
<211> 13670
<212> DNA
<213> Artificial Sequence

<220>
<223> chemically treated genomic DNA (Homo sapiens)

<400> 266

```
aggtggaggt tgtggtgagt tgagattata ttattgtatt ttagtttggg tgatatggtg      60
agatttttat tttaaaaaaa ataaaaataa aaaaattttt aaagttttga tgtaggttaa     120
ttatataata aaagaaaggt agtggttaag tagtttaggg agaataaaag tttgtatatt     180
atgggtaatg taattatagt ggtttatttg gtttttgaga tgtattgttt attgaggtag     240
aatataaatt attatttatt tttaagtggt aattttatgg atttagttat ggttttagga     300
tagtataatg tttagtagtt ttgatagtat aaaggtatat ggataaaagt gggaggtgga     360
aaggggtaag aggtgataag tggagtgttt ttaaaggaaa ttaataggga aataaaaaat     420
ggtatgtttg gggtagtgat gtgagtaagt tagtggttga tgttatatat tggttaagtt     480
aagaaataaa ggttaggtat ggtggtttgt atttgtaatt ttattttggg aggtggaggt     540
ttgaggattg tttgaggtta ggagtttgag attagtttgg ataatatagt aagttgtttt     600
tttgaaaagt ttttgaaaaa ttagttgagt gtggtggtat aggtttgtag ttttaattat     660
aggttgtagt gtttgagttt aggagtttga ggttgtagtg agttatgatt gaaattttat     720
ttgaaaaaaa aagaaaaagt taaaaatgtt ttagtggttg tttttgggag gaaggggagg     780
tgtagttata gattgttgtt tttttttta tatttttgtg gatggttatt ttataagatg     840
tatgttttta ggtaaatgat tttatttttg aattttaggt ttatgattta tattatagga     900
atgtgattat gttatttaga tttaataggg ttgttttatg gttaagtgat ggttgttttt     960
aaaatgttta aggatattgg gagaaggttg taaaatagga ggaagaagtt ggttgttttt    1020
tttttttttt ttgagatgga gttttgtttt gttgttttgg ttggagtgta gtggtgtaat    1080
tttagtttat tgtaattttt gtttttgggg tttatgttgt tttttgtttt tagttttttt    1140
agtagttggg attataggtg tttgttatta tgtttggtta atttttgta ttttagtag    1200
agatagggtt ttattatgtt aggatggttt tgatttttg attttgtgat ttgtttgttt    1260
tggtttttta aagtgttggg attataggtg tgagttattg tgtttggtta gaagttggtt    1320
ttttgtaggg atagtagggt tgtaaggttt tgtgatttta ggttttatta tatttggttt    1380
tgttttattt ttttttttgg ttagaatttt agaattttag ttatttatag gttgggtttt    1440
ttttggggga attaggtttg tttttttagga attagggtat ttatgttttt ttggtttgtt    1500
tttggagttt gggttttaat atttgtagat tggtttttgg gttaggattt gttgtttgaa    1560
tattagtggt tgttgttgtt taattataga tgtatagggg tgggttggtg ggtttgttgg    1620
gttagtagga ggatggtgtt gggttgaggt tgttggattt gttgttggaa ttatagttgg    1680
gtgaagttag tgttgttagg tttttggagt tagattgaag ggagggttgt gtgtttttgg    1740
gtgggtagta ttaggtggtg gttgttggtt gggtttttttg aagttttttt tagttgttaa   1800
tattgtttgt gtttttttttt tagttttaga tttggtattg tttgtttttt ttttttgttt   1860
tttagatttt tttgatttgt tatattaggt tttggttttg ttttttgttt tattgtttat    1920
tgtttatttt attttattgt tttttttttg gtttattgtt agtttattgg tttaggtttt   1980
tgttttttttt gtttaatgtt aggttttatt tttttagga tttatttttt ttttgtttat   2040
ttgtataagt taggtttatt attttaattt tttttttttt ttggtttgta ggttgtattt   2100
attttgtata tgttttgttt tttattttag gtgttgttta tttaattgaa gatttaggat   2160
ttttttttta aggtttgttt gggtttggaa ttgttttttt tttttttttt ttttgagatg   2220
gagttttttt gttgtgattt aggttggagt atagtggtgt gattttagtt tattgtaatt   2280
```

```
tttatgtttt gggtttaagt gattttttg ttttagtttt ttaagtagtt gggattatag    2340
gtgtttgtta ttatgtttag ttaattttg tatttttagt agagatgggg ttttattatg    2400
ttgtttaggt tggttttgag tttttgattt taggtgattt gtttgttttg gtttattaaa    2460
gtgttgggat tataggtgtg agttattgta tttggtttgg aattgttgtt tttagatttt    2520
tatttatttg ttgttgagtt tttttgggtt ttgttttggt tttaagtttt gtataggatt    2580
tgttaggttt tgtttattta tttttttttt ttttaatttt ttttttttta gtttttgtat    2640
tttatttttg ttttttgttt ttgttgtgtt taggtgttta tttggtattt aggtgagtgg    2700
tgtgttttgg ttgttttttg ttggagttgt ttgttttttt ttttagtgtg ttttataaat    2760
tttgatggta tggaggggtt ttaggttaag ggtgatgggt ttttgagttt tgatattgtt    2820
ttgttgttgt tgtaggtgtt tttggttggt agtgttgttg tggttttgga gtgtggtgat    2880
atgttggagt tggtggtgat tgtgttgttg gtgttgttgt gtttgttgtt gaagttgttg    2940
tgtgttttta tgtggagttt ggtgtttttg gtggtgttgg tggttgtggt ggtttatggt    3000
tgtatagtgt ttatgtatgt gttgtgttgg ttttggtgtg gttgttgtgg gtgttgttgg    3060
agtgtgtttt ttgtttgttt gagtgatttt ttgttgggtg agtatggttt tttgaatttt    3120
aaggtgattg tatgggggtg ttattggggt tgtggggttg tgtggggttg agatggttta    3180
agttttggga gagagggttt tttaggtttt aggttgtgtt ttttttttag agtttgggtt    3240
tgtgtttgta ttatgttttg gttggatgag gtaatggatt ttttatgttg tttttgtatg    3300
gtttttttga gaattggtat gtttggggtt aggggtttga ggtttatggg atttgggggt    3360
aggtggagtt tgggttttta agaggtaggg aggagggttg tatagattgg gataagttga    3420
atatagatgg ggataggggt attgagttag tatatttagg atttttaggt tttgaaggga    3480
taggataaag gggtattgag gttgattgaa gtttttttgga tttagggtgt agttggtttt    3540
agttttagtt tttgtttttg tttgggattt tttatttttt ttttaggggga agtttagagt    3600
aggaaggagg gtttgttaat ggttgttgat gtgtgtaggg tgtaggtgta atttgagggt    3660
tttgatttag tttttttttt ttgtttgttt ggtaggtttt tttggtgtta ttagttttgg    3720
gagttttaga gttgttttta tgttgtggtt gtggatttgt gaggttatgg tttttttggat    3780
gtattttggg atgtggattg ttatataatt gatttgttgt tggtggatat taaagatgtt    3840
attttaggtt tgggtgtaga tatattttta ttttaggtgt ggtttttttt attttgtata    3900
tatgtatatt tttgtttttt ttttgttgga atttattttt tttattttttg ttttagtttt    3960
attgggtatt ttatttatgt gagtttttttg aatttttata ggttatttga agtgtatttt    4020
tgtggtttat gattggggtg ttttttttgt ttggtatttt tttatttatt atttattttt    4080
ggttgagtgg atggttgtgg ttagtggtgt ttttatgttg gtgtattaag gtgtgtgtgg    4140
gagtttggga tattgggatg tatttatgtg taagggggta gggatatgtg tttgtgtttt    4200
taatttttttg tatatttgtt aatatattag gggttgaggg tgtagtaatt aatttaggtt    4260
tttttggttg tggaattttt tatttggaaa ttaatgatat gttagattta gtggtttata    4320
tttgtaattt tagttttttg ggaggttgag gtgggaggat tatttgaggt taggagttta    4380
agattagttt ggttaatata gtaagatttt aattgttttt ataaaaaaaa aaaaaaaatg    4440
taattagtta ggtattggtg gtgtatgttt gtaattttag ttatttggga ggttgatgta    4500
ggagaatttt ttgaatttgg gaggtggagg ttgtagtgag ttgagattat gttattgtat    4560
tttagtttag gtaataagag taaaatttta ttttaaaaaa taaataaata aaataaagta    4620
tggttaggtg tggtggtttt tgtttgtaat ttttgtattt tgggaggtta aagttggtgg    4680
attgtttgag tttaggggtt taagattagt ttggttaata tggtgaaatt ttgttttttat    4740
taaaaatata aaaaaattag ttaggtatgg tagtgtatgt ttgtagtttt agttatttgg    4800
gaggttgagg taagagaatt atttgaattt gagaagtgga ggttgtagta agttaagatt    4860
gtattattgt attttagttt ggatgataga gtgagatttt gttttaaaaa taaaataaaa    4920
taaaataaat tataatatat ttaggagtaa tttatatttt tttatagtat ttatagtaat    4980
aaggaatatg tatatagtaa tttatttaag gtttttgggt ttatgaaggt tgtattattt    5040
ttatgtttta gttggaggaa attgaggttg gggaggttaa taatgtttat tatattattt    5100
gatgttaata tgtagtattg tggttataat tgtgtttttt tttttttttt tgttgttttt    5160
tttttttttt tttttttttt tttttttttt tttttttttt ttttttgaga tagagttttg    5220
ttttgttgtt aggttggagt gtagtggtgt gattttagtt tattgtaatt tttgtttttt    5280
aggtttaagt gattttttttg ttttagtttt ttaagtagtt gggattatag gttattatgt    5340
tgggttaatt ttttgtattt tagtagagat ggggttttat tatgttggtt aagatggttt    5400
tgattttttg attttgtgat ttgtttattt tggtttttta aagtgttggg agtataggtg    5460
tgagttatta tgtttggttt tttttttttt tttttttttt gagatggagt tttattttgt    5520
gtagtgtagt ggtgtaatgt tggtttatgg taatttttgt tttttaggtt taagtgattt    5580
ttttgtttta gttttttttag tagttgagat tatagatgtg tgttattatg attggttaat    5640
tattttgtat ttttagtaga aatggggttt tagtatgttg gttaggttgg ttttgaattt    5700
ttgggtttaa gtgatttgtt ggttttagtt ttttaaagtg ttgggattat aggagtgagt    5760
tattttgttt ggtaattttt attttttggg tttaagtgat tttttttttt tagtttttag    5820
agtagtgggg attgtagata tgttttaata tgtttagtta atttttgtat ttttagtgga    5880
gatgggtttt tattatgttg tttaggttag tgtttaattt ttgattttaa gtgatttatt    5940
```

tgttttggtt ttttaaagtg ttgggattat aggtgtgagt tattgtattt agtttattat 6000
tgtttatttg tttgtttttt tatttagatt gttagttttt tgagggtagg gagtttttg 6060
tttttggggg gtgttttttt ttgtgtttg ttttttttt tttgagatag ttttgttttg 6120
tggtttaggt tggagtgtag tgttgtaatt gtagtttatt gtagttttag atttttgggt 6180
ttaagtgatt tttttatttt tgttttttaa gtagttggga ttataggtat atattattat 6240
attttgttaa tttttaaaaa attattttt ggaggttggg tgtggtggtt tatgtttgta 6300
attttagaat tttgggaggt tgaggtgggt ggattatgag gtttggagat tgagattatt 6360
ttggttaata ttgtgaaatt ttgtttttat taaaaatata aaaaaaatta gttggatgtg 6420
gtggtgggtg tttgtagttt tagttattgg gaggttgagg taggagaatg gtgtgaattt 6480
gggaggtgga gtttgtagtg agttaagatt atattattgt attttagttt gggtgataga 6540
gtgagatttt gttttaaaaa aaaaaaaaaa aaaaaaaaaa aaaaaaaatt atagatatag 6600
gggtttttt aagttgttta ggatgaatag ggagttttga ttgttttatt tattgttgtg 6660
tttagaataa ggtttggtat gtagtagttg tttaataaat gttagttgaa taaagtagag 6720
gaaatgtatt ttggtttggg tagaggaagt atatttttgg atatgggggt ttttaggaga 6780
tgtttttatt ttgtggatag tgaaatatgg taggagggaa tgtagttggt gtttaggtta 6840
ggaggggtat ttagtatttt tatagattta ggtaggtagt agtggtattt ttgaggtttg 6900
tgtttttgat ttgggtatgt gtaggggtgt aggtgtatgg ggttatttta ggagtaggtt 6960
ttatattttt tttttaattt ttttatttta gattattttt tgtattatat tagttagttt 7020
ttttgttttt attatatgtt tttgttttag ttgttttggt tgtttgagaa gttgttgttt 7080
atgtttgatt tttaggtata gagggtgtag gttgaaggga ggagggaagt aggttggggt 7140
tgggtaggtt gggtattgaa gttagagttt tgttgtgtat ttagattttg aagattattt 7200
ttatttattg taagataggt attttatgtt tgatttttag tgagtttgag gttttttttt 7260
ataatttttt atagtttggt ggttttattg ggttttttaa ttattattga aattttttta 7320
ggtgagatta gttgttaagt ataagtttag gagatttgta ggggtagggg ggtgggaggt 7380
aattattttt ttgtttgttt gtttgtttgt ttgtttgttt tggttatagg aatttttttt 7440
tggaatttta ggagttgatt atatttatat tgttgttgtg gggggagaag gatatttatt 7500
tggagttggg gttggtggaa gttattggta gttgttttgt gttgggttgt ttggaggttt 7560
atattttgtt aggtataggg tattggattt tatagagtaa tttttaggag atgtattagt 7620
atatgtgggt ttttttgtaa gatttgttgg attagtggtt tttgttggtt tgtttaggag 7680
gtatggatat ttaggaagga atatatattt attatttgtg tgtgtttggg agtttatata 7740
atgtttatat atgggtgaat ttttgaattg tttataggta tgggattttt ggattttata 7800
taagtgtatt tatgggtgtt ttgggatata ttaattttta tatttatata taggtatgga 7860
tgtatgtgtg tgtaataaat attttgattt tgggaattgg gtatattttt tttttagtgg 7920
agttagatgt tgagattgag tgttttattt tttttttttt tggggtttta ttttgtgttt 7980
tgttaaagtt gttttttttgt tatagttgta tagattttaa attttgattt ttttgttttt 8040
gtttttgatt ttttgtttgg gtttttagtt tagtgttatt ttgaatataa tattgttgat 8100
tatatttggt tattttagtt taaatgtatg ttaattgaaa tgaaatataa tgaaaatgta 8160
gtgtttagtt ttattagttg tattttattt tttttttttt gagatagagt tttgttgtgt 8220
tgtttaggtt ggatggagtg tagtggatgt aatttaggtt tattgtaatt tttatttgtt 8280
gggtttaagt gatttttgtg ttttagtttt ttgagtagtt gggattatag gagtgttatt 8340
aggtttgatt aattttagta tttttagtag agatggggtt ttattatgtt ggttgggttg 8400
gttttgaatt gttgatttta agtgatttgt ttattttggt tttttatagt gttgggatta 8460
taggtgtgag ttattgtgtt tggttatatt agttgtattt taagtgttta attgttgtat 8520
tagtgaagaa agttgtgtgt ggtagtgttg atgagggtaa ttttagttta agagtagggg 8580
ttagggttga ttatagtggt ttaaatttgt aattttagta ttttgggagg ttgaggtggg 8640
tggattatga ggttaggaga ttgagattat tttggttaat ttggtgaaat gttgttttta 8700
ttaaaaatat aaaaaattag ttgggtgtgg tggtgggtgt ttgtagtttt agttatttag 8760
gaggttgagg taggagaatg gtatgaattt gggaagtgga gtttgtggtg agttgagatt 8820
gtgttattgt attttagttt ggtaatagag tgagatttta ttttaaaaaa aaaaaaaaag 8880
agtagggggt ttttttgttt ggtttttggt aattttttta agggtagatt tttatgagta 8940
ggtaggtggg tgtgtagatt ttttattttt ttttagttag ttttaggaga gtttttataa 9000
gttttttttt ttatttttttg ttatgatttt aggtttggtt aggttaagtt tttttgtttt 9060
agtttgggtt ggtttgtttt agatttttgg gaaaatagtt tttgtttttg ttaggataag 9120
tgagattttg gattttgggt aggaagggat tagtttttag aaaagttatt tgggatttga 9180

ggtttgtttt tttagttttt ggaaattttt gttttaaatt agttatagtt tttgttgatt 9240
ttggagtttt ttggggtggg gatgggtggg gggtggtggt tatatgtgga gggtgtgtta 9300
tttagtttta ggttgattgt agtattagtt tgtatttttt tggtttgtgt ttggtttttt 9360
ggggatgttg ttattattat ttgtattatg aggattgaga ggttttgaga aatatatttt 9420
tttgtttttg agataggttt gtttgttggt tatttttttt tgtttttttt tatttttagt 9480
ataatatttt ttttttgttt gtttgtttgt gagatggagt tttgttttgt tgtttaggtt 9540

ggagtgtaat tgtgtgattt tggtttattg tagttttttt ttaggtttaa gtgatttttt 9600
tgtttttgtt ttttgaatag ttgggattat aggtgtttgt tattatattt agtttatttt 9660
tgtatttttt tttttttttt ttttttttga gatggagttt tgttttgttt taggatggag 9720
tgtagtggtg tgattttggt ttattgtaag ttttgttttt tgggtttata ttattttttt 9780
gttttagttt tttgagtagt tgggattata ggtgtttatt attatgtttg gttaattttt 9840
tgtattttta gtagagatag ggttttattg tgttagttag gatggttttg atttttttgat 9900
tttatgattt atttgttttg gtttttttaaa gtgttgggat tataggtgtg agttattgta 9960
tttggtttat ttttgtattt ttagtagaga tggggtttta ttatgttggt taggttggtt 10020
ttgaattttt gattttatta tttgtttatt ttggtttttt aaagtgttgg gattataggt 10080
gtgagttatt gtgtttggta taatattttt ttatttaata tagatattag gtagtgtttt 10140
aggttttagt gatagagtgt gagttagaaa gagtttaagg aggagagagt tgatagggtt 10200
tagggaaggt tttattgaag atttataggt gggattttag ttagaggatg tatttatttg 10260
ttgttgtttt gagataggggt tttattttgt tgtttaggtt ggagtgtagt ggtgtgatgt 10320
aagtttatag tagtttttat tttttgggtt taattaattt ttttgtttta gtttttttgag 10380
tagttgggat tataggggta tattattatg ttaggttaat ttttaatttt tttttttgaga 10440
atggaggttt gttttattgt ttaggtggga gtgtagtggt gttattttag tttattgtaa 10500
tttttatttt ttgggtttaa gtgatttttt tgttttagtt tttttagtag ttaggattat 10560
agatatatgt tattatattt ggttaatttt tgtaatttta gtagagatgg ggttttatta 10620
tgttggttag gttgattttt aatgtttgat tttaagtagt ttgtttgttt tggtttttta 10680
aaaatttttt atagagatag atgttttatt atgttgttta gagtggtttt aaattttttg 10740
gtttaagtga tttttttatt ttggtttttt aaagtgttgg gattttaggt atgagttatt 10800
gtgtttgttt aggatgtatt ttttggtgga gtgggaagta gtgattagga aggttttagg 10860
tgggaagaag tttgggttat tggagttata atatgtaggt tagggttttg ttaaggtaga 10920
gtgagtgagg aggttttaga gaagaggtta tatagttaga ttgttagggg ttgtgaggag 10980
tttggatttg atttttaagg ggagttaggg gagggttttt agtagggaag ggatatggtt 11040
tgtattttga agagtttttt gtggtggttg gatagtttta tgtaagagtg tgagtgttga 11100
agtttgtggg gggttgtgtg tggttatagt atagggtggt tttattgttt tgattgagta 11160
gggattttta gagttgggtt taatttgggg tttttttgtt aggggaggag taatgttttt 11220
tgttttgggg aggtgagaag atgggtagga ggaggaggga gtttttgaga tgtggggttg 11280
tggatgttgg agtttgtggt ggaggtttgg ggtgaggagg tttgtggata ggattaggat 11340
ggtgtttagg ggtaggtggg tttggtgatg gttgttgggg tgatggggga tgggttgatg 11400
gttgattgga atgggtagtg gttgtttgag tgattggagg ggtttaggta aaggttgatg 11460
ggaggtttgg gatagttgtt tgggtgatgg agatttgttg atggttgtta gggtgatgga 11520
aaggtgtttt tttttgtttt tgtggttgtt ggtgggaggg aggtgggggt tttttgggga 11580
agatgattgt gttgattggg tagagttttg gtgagtaagt taatagaaaa ttgtgttttg 11640
ttgtagattt gttttttttta gtttgagtta attttggttt gtttttttttt tttttttttg 11700
gtttaagggt ttggtgtgtg tattttttag ggtgggagtg gggattttgg gggtttgggt 11760
tgggattttt tgtatgattg gtttgttttt tttttttgttt ttagattatt ttttattatt 11820
tatttattta tttatttatt tatttattta ttttgagggt tgtattgatg gtttagtagt 11880
agaattttta tttttttattt atttatttat ttatttattt gtttttgtta ttagttttag 11940
gaggggtggt ggaagataaa gttgggtttt gatattgtta gtagggtgtg attaggtttt 12000
ggtggaatga gtaattgggg aggagtattt atttgggggga agggagggaa ggtttttttgg 12060
aggaagtagt tttggagttg aattttgtaa agttgtgttt tgatttatag aagtttttta 12120
tgttgggatg atttagtatt tttgtgagat tgggaggaaa gtttgggtta ggaagatgtg 12180
agattataaa aggttttata tgttagtagg ggagggtttt ggatagattt gtgtgtttgg 12240
aatgttattt gggggatggt gagaaggttg gattagagga tgggaaggtt ttagaggagg 12300
atgggtgtgg tttaggtagt ggagaatgag atttaagttt aggtattgga agaggagtag 12360
atatgttgag aaggtataga ggtaggtttg ggatatattt agtttgatgg tgttggattt 12420
tggggtaggg atttagaagt atgttatggg ttaaagataa aaatagttta gtaagtattg 12480
gtgtattgtg gggtagagag gtttttaagt tgggagtgag ggtgaagttt ttttagagta 12540
gaatggagaa taagattgga gattgaattt tgaggatttt taatatttta gagataggta 12600
gaggagagag gttataaagg taggaggtga ggttaggtat ggtggtttat gtttgtaatt 12660
ttagtatttt gggaggttga ggtgggtaga ttataaggtt aggagattga gattgttttg 12720
gttaatatgg tgaaatttta tttttattaa aattataaaa aattagttgg gtatggtggt 12780
atatgtttgt agttttagtt atttgggagg ttgaggtagg agaattattt gaatttggga 12840
ggtagaggtt gtagtgagtt aagattatgt tattgtattt tagtttgggt gagagaggga 12900
gattttattt taaaaaaaaa gtttaggtat agtggtttat atttgtaatt ttagtatttt 12960
gggaggttga ggtgggagga atatgaagtt aggagtttga gataagtttg tttagtatag 13020
tgaaattttg tttttattaa aaatataaat attagttagg tgtggtggtg ggtgtttgta 13080
attttagtta tttgggaggt tgaggtagga gaattgtttg aatttgggag gtggaggtta 13140
tagtgagtta agattatatt attgtattat agtttggatg atagagttag attttatttt 13200

aaaaaaaaaa aaaaaaaaaa aggtaggagg tgaattaggt aaaagtgatg ttttagtaat 13260
ggaggaggga ggagtgttaa ggaataggga tgtagttttt ggtgttataa gttgttgaga 13320
gataagtgtg gtggtttatg tttttaattt tagtattttg ggaggttgag gaaggaggat 13380
tatggtagat taagagtttt gagattagtt ttggaaatat tgtaagattt tttgtttttt 13440
aaaaaaaaaa aaaaaaattt aattgttggg tatggaggtg tgtgtttgta gttttagttt 13500
tttaggaggt tgagatggga ggattgtttg agtttgtgag gttaagatta taatgagttg 13560
tgattatgtt attgtatttt agtttggaga tagagtaaga ttgtgtttta aaaataaata 13620
aataaaaatt taggttgggt gtagtggttt atttgtgtaa ttttagtatt 13670

<210> 267
<211> 3479
<212> DNA
<213> Artificial Sequence

<220>
<223> chemically treated genomic DNA (Homo sapiens)

<400> 267

gttggagtga agtttgtggg atgttgtaga gaaaattatt taaaagtatg gtattaattt 60
tgaggagatt gtggttgagg agagttggga ttatgtgtag ttttaggtaa gttttttttt 120
tttagttttt ttttggattg atttgtttta gttttgtgtt tggaggagat tttattttaa 180
tgtgggtttg atttttagtt ttatgatttt aatatatttt aatttttttt aggtttgatg 240
tttttttattt tttagatgat ataattgttt ttggtgttgt ttggtttttt agtatttaga 300
ttttggtgtg gttttgttat ttattttgag agattttgtt tttgttttttg ttttgattag 360
aggttgttag gtttttagtt ttaagatttt agtgagatat ggttttttat tttgtagtga 420
ttttggtttt tattggattt tgtttttgat gagatttata tggttttgta tttttattta 480
tttgttgggt attagaggtt taattgtatt atttatttaa tttttagttt atagtgtgat 540
tttgtagttt tttttttagt tttaggttat tttgtgattt gtttttttta gttttatgtt 600
tttgtaatgt tttttggttt tggttttatt gtgattttag tttattgttt tttattttat 660
atttattatt tagttttttg ttttttgatt tgattttata tttattattt tgttttttga 720
tttttaatat tatatgtttt tagttttggt ttttttgatt tgtatttttt ttttagttgt 780
gttgttgtgg ttggtattat ttgtaggatt ggttttaagt ttttattttg agaggtaatg 840
ggttggaggt gtattgtgtg ttttgttttt gttataattt gttggtgatt aatgatttta 900
taattttaga taaggtgatt ttgttttagt ttagtaggtt tggatatttg gtgtggttta 960
gatatagtgt agatatttgt gttgtttttg tagagagtta tatttattgt gaggtgggta 1020
ggggtttgga gtataaattt gttgaatggg gtggggtttg tgggaggggg agggttgtta 1080
gtgagtagtg gtttgagaaa gggtggggtt tatgagaaaa ggaaaggtat ggtaggaggg 1140
gtggggtttt ttgaaggggg tggggtttgt aggaagggta gggtttaaag aaaaggttgg 1200
gttggttttg gaatatagat gtttagggag gggttaggtt tggaaaaggt gaagtgaggg 1260
tgtattagta aggagattag agtgttttgg tgattagggg agtgggtaga tgtttgaaga 1320
tggtgagggt tggtttgaaa agaatgttgg gtttgggttt gagagtttta gaaagaattt 1380
ttttaatttt tttttatatt ttttagggtt gtgtgtaggg tttttagaag tggttgtata 1440
ataattttat ttttatagtg ggtatttgtt tgggtgttgg tttatttgag gtgatttggt 1500
tttgttttta tttgtgattg gttttaaatt tttagatggt tttgtttttt attttgtgtt 1560
ttttggttgt ttgggaagga tgagtttagg gtggagtgta gtttattttg gttttttttgt 1620
tgttttattt agtattggag ggtgggggtg gtttagtttt agggagtttt gattgggtgt 1680
atgtagggaa agttttttgt tattggttgt gatttttttt tttttttttt gtagatgatt 1740
gttatggtgt tgagtgtata gttatagtgt agggtatttt gttggaaatg tgagttgtat 1800
gtgttgggtg ttggggattt gagttttggg tttagtttga ttgttgatgg tggtggtggg 1860
tatagtggta gtttgtgggg tggttggggt atggtgggtg tttgttttaa ttggggagat 1920
aaggtattgt agggtaagtt gtttatggtt ttggggtttt ggttgttgtg ggtttaagat 1980
gaggattagt ttgatattgg aagtgtgggt gtagaattag aggaggtata attagaggtt 2040
gaggtagagg gggaagatag atgagttttt aaaataaagg attttgggtt tgtttttgat 2100
tttatttttt tttagttttt atttttattt gtagtagtta tttttgtttt attagttagt 2160
tttgtggtag tttttgttga gttttgtttt tttttattta tttttttttt tagttttttt 2220
ttgtttaatt tatggtttta tttttgattt tttttgtttg ggtgggtatt atttttgttt 2280
tgttagtatt tttttgattt ttttgatttt attttttttt tttatagttt gggtttatga 2340
tgtttttgat atttttgtgt agaaatttgg attatgttta taattggttt gtttgatatt 2400
gttaggtttt gttttttttta aagttttgtt ttgtttttgt tatgtgtgtt gggtattttt 2460
ttgttgtttg taaatatttg tttaattttt agtttgtttg gagtttttttg tttttatatt 2520

```
tttttgggga tttatgtggt tgtgtgtttt tgttgttgtt attttttta tgggatttgg    2580
ggtttttgtt tatagttttt tgtttttatt tgttggttta ttattattta taagattatt    2640
ttttatttaa attttaaata aattttttgt gtttttggta aaatagtttt atttttttgtt   2700
agaatataaa taaataaatt ttgagagggg aggaaggaaa ttgtttagtt tagggtttat    2760

ttaggagagg gatgagatta gaaagtttaa tatattgttt gtgtagtgga gataaagtta    2820
agattttagt atttatttat aaatattttg ttatattaaa aaaaaaaaaa atgtttaggg    2880
tttatttggt tttgtttttg tatagaaagg gtttattttt attttgtgat tttataggtt    2940
atggagtgag ggtggtagag aggggtagaa attttagggg gaggggtggt tgggaaaaag    3000
taaaggggat aagttaatgt gtaattagtg tttttaaga tatgtagagg agtgggggtg     3060
gtttgttagg ggttgaaaag aaaagttagt gttgtatttg gggggttgtt ttatttttgt    3120
ttttataatt tttgatattt tggagtgatt tgttttttta gatatttatt gtgaggtttt    3180
aatattgggg tttatttttt ttttagtttt agtttgttta gtttttttaat taagttttgg   3240
gggttttttt aatggggggg atggttttag ttgtttaggt ttttgaggtt aatttttttta   3300
tattatagtt tttttttaa ataagaagta tgaggtgagt tggaggattt tttttgggag     3360
gagggtgttt tggggggtga gttagttttg gggttttttt ttagtttttg attaggtggt    3420
aaatgtgatg ttgggtttta tgtttgttgg tggagatttt gaagatatag gtggtgtag     3479
```

<210> 268
<211> 3479
<212> DNA
<213> Artificial Sequence

<220>
<223> chemically treated genomic DNA (Homo sapiens)

<400> 268

```
ttgtattgtt tatgtttttg aggtttttat tagtgagtgt ggggtttagt attatattta      60
ttgtttggtt agggattgaa gggggatttt aaaattggtt tattttttag aatatttttt     120
ttttagggag ggtttttttag tttattttat gtttttttatt tggggagagg gttgtgatgt   180
aaaggggttg attttagagg tttaagtaat tggggttatt tttttatta gaggggtttt      240
taaaatttgg ttggagagtt gaataggttg ggattgagga aaggataaat tttgatattg     300
ggattttata gtgggtgttt gaaaggatag attattttgg agtattaggg attgtgggga     360
taggagtgag ataatttttt aggtatagta ttggtttttt tttttagttt ttgataggtt     420
atttttattt ttttgtatgt tttggagagt gttagttata tattggtttg ttttttttat     480
ttttttttag ttattttttt ttttgaaatt tttgtttttt tttattattt ttattttatg     540
atttgtgaga ttataaagtg aagatgaatt ttttttgtgt aggagtagag ttaggtgggt     600
tttggatatt tttttttttt ttaatataat gagatattta taagtgggtg ttagggtttt     660
gattttattt ttgttgtata agtagtgtgt tgaatttttt aattttattt ttttttaag      720
tgagttttga gttagatggt ttttttttttt ttttttaaa gtttgtttgt ttgtgttttg     780
atagaggata aagttatttt attaaaaatg taggggattt atttgaggtt tgggtgaaaa     840
ataattttgt gggtggtggt aggttgatag atggggatag gaagttgtgg atgaaagttt     900
taggttttgt gggagaggtg atagtagtag gggtatgtag ttatgtgggt ttttagggga     960
atgtgaaggt ggagggtttt aggtgaattg gggattaaat aaatatttat aggtagtagg    1020
gaagtgttta gtgtatgtga tgggggtggg gtgggatttt ggggagggtg gggtttaatg    1080
gtattgagtg agttggttgt agatgtggtt taggttttttg tataggaata ttgagagtgt   1140
tatgaatttg agttatagag aaaggagatg aggttagaga agttgaaggg gtgttggtga    1200
gatgggggtg atgtttattt gggtgagaaa ggttaggggt ggggttgtga attgggtggg    1260
aaggggttgg agaaggggat aggtagaaaa gggtggggtt tgatgggaat tgttgtaggg    1320
ttggttgatg aggtgggaat agttgttata agtgggggta gaggttgaga aggagtaagg    1380
ttggaagtaa gtttagggtt ttttattttg gaggtttatt tgtttttttt ttttgtttta    1440
gtttttaatt gtgttttttt taattttgtg tttgtatttt tagtgttagg ttggtttttg    1500
ttttgaattt atagtggtta gagttttagg gttatgggta gtttgttttg tggtgttttg    1560
tttttttagt tggggtaggt atttgttatg ttttagttat tttatagatt gttgttgtgt    1620
ttgttgttgt tgttagtgat taggttgggt ttggggtttg aatttttagt gtttggtatg    1680
tgtggtttgt atttttggtg gagtgttttg tgttgtagtt gtatgtttag tattatgata    1740
gttatttgtg gagaaagggg agggagattg tagttaatag taggaggttt tttttgtgta    1800
tatttaatta gggtttttttg aagttgggtt gtttttattt tttggtgttg ggtggagtgg   1860
tgggagggtt ggggtgggtt gtgttttgtt ttgagtttgt ttttttttagg taattgaagg   1920
atgtgaaatg aagggtaggg ttatttaggg atttagggtt gattgtgggt gggggtgggg    1980
```

```
ttagattatt ttgagtaggt tgatgtttag gtaaatgttt attatggaaa taaggttgtt    2040
gtgtagttat ttttggaggt tttgtgtgta gttttggggg gtgtagggaa aagttaaagg    2100
gatttttttt gggattttta agtttaggtt tagtgttttt tttaggttaa tttttattgt    2160
ttttaggtat ttatttgttt ttttagttat tagggtattt tagttttttt attagtgtat    2220
ttttgtttta ttttttttaa gtttggtttt tttttagata tttgtatttt agagttagtt    2280
tagttttttt tttaggtttt gttttttttg tagattttgt tttttttaga gggttttgtt    2340
tttttttgttg tatttttttt ttttttgtag attttgtttt tttttaggtt gttatttatt    2400
gatagttttt tttttttttgt aggttttgtt ttatttgata ggtttatgtt ttgaattttt    2460
gtttattttg tggtagatgt ggtttttttgt agggatagtg tagatgtttg tattgtgttt    2520
ggattgtgtt aggtgtttaa gtttgttgag ttggggtaag attattttat ttaggattgt    2580
ggagttgttg gttgttgata agttgtagta ggagtagggt atgtggtgtg tttttgattt    2640
gttatttttt aggatgagga tttggaatta gttttgtggg tagtgttagt tgtagtagtg    2700
tagttgggag agggatatga gttagaggga ttgggattgg agatatatga tgttggggat    2760
taggggataa ggtgatgggt atgaggttag gttggggagt aggggattaa gtggtgggtg    2820
tggagtgggg gatagtgggt tggggttatg atggggttgg agttagggga tattatggga    2880
atatggggtt gggaaagatg agttatgggg tgatttgggg ttggggaagg agttgtaggg    2940
ttgtgttgtg gattagagat tgggtgggtg gtgtaattga gtttttgata tttgataggt    3000
gaataggagt gtgagattat gtgagtttta ttgggggtgg ggtttggtgg aagttagagt    3060
tattatgggg taggaagttg tgttttgtta gggttttggg attaggggtt tgataatttt    3120
tggttggggt gggggtgggg gtggagtttt ttgaggtaga tggtgaagtt atgttagggt    3180
ttgggtattg ggagattagg tgatgttggg gatagttgtg ttatttgggg agtggggagt    3240
gttgggtttg ggagggattg gggtgtgtta ggattgtaga gttgggggtt gggtttatgt    3300
tggggtggag ttttttttaa gtatagggtt gaggtgggtt agtttgggag ggaattggag    3360
gagggggggtt tatttggaat tgtatatagt tttagttttt tttggttgtg gttttttttgg    3420
ggttggtgtt gtatttttgg atggtttttt ttatgatgtt ttgtaagttt tgttttagt     3479
```

<210> 269
<211> 10857
<212> DNA
<213> Artificial Sequence

<220>
<223> chemically treated genomic DNA (Homo sapiens)

<400> 269

```
ttttagtgtt ttgggggggtt gtggtaggag aattgttttg ggagtaggag ttgtaggttg      60
tagtgagtta tgattagttt gggtgattga gtgagatttt gtttttaaaa taaatatata     120
agtttgggtg tggtggttta tgtttgtaat tttagtattt tgggaggttg aggtgggtgg     180
attatgaggt taagaaattg agattatttt ggttaatatg gtgaaatttt gtttttatta     240
aaaatataaa aattagttgg gtgtggtggt gtgtgtttgt agttttagtt atttgggagg     300
ttgaggtagg agaattgttt gaatttggga ggtagaggtt gtagtgagtt gagattgtgt     360
tattgtattt tagtttggtg atagagtgag attttgtttt agaataaata aataaaagga     420
tagaaaggtg agtataaata tttttaattt ataatatttt tttgtattgt taatgtttta     480
gatatgtgtt attattttta gtaaattttt ttaggtgttt gtaggatggg ttaaggaagg     540
tgatgagtat tagttgtttt gttgaggttg ttttgatgtt atatgatttt ttaattatat     600
gatttttaga aatggggtgt ggggtgagag gaagtaggga ggagagtgat ttgagtagaa     660
aagaaatata gtattttagg ttggttttat ttttatattg ataagtagtt aatgggagtg     720
ggtagttttg atttttggtt aatggaaatt gaggtaggtg ggttattgtg ttggggtttg     780
tagtttgagt gttatttggt tgttgttgtt taaggattgt ggagttggat gtaggtagga     840
gagtggttgt gtagattttt tgtttgtttt tgtttagggg tttgttaggg ttatgtgagt     900
ttgaggtttt tttggagttt tagttggaga taatagaaga attgtttatt gaaatttttt     960
gggggttttg atatattagg gggagtttta tgggaaagag gaagtagtaa ttgtagtgat    1020
gttttgttag aaggggtttt ttattttttt agtatttttt taaagtaggg attatattat    1080
ttttgattta gttttatttt tgttggtagg tgttggtttt tttttttttt ttggtggtgg    1140
tgggtggttt ttgtggtggt ttggagttgg aggggtgtgt gattttgggt tgggagtttt    1200
gagggtttgg gaatgagatt tgagattttg gttttttgaa ggtagtaggg atttgggagt    1260
ggtgattgaa tttggtttgg ttttttttta tttttttttg ttgtgggtgg gatgagttag    1320
tttttgtttt ttttagttat ttttttttgt ttatttgtag ttaggttggt tttttttttg    1380
ggaatttttt ttttttttgg tatttggagt tggggggtgt tatttagtgg aagataatgg    1440
agttagggtt ttgaagaggt tgttgttttt tttggttgtt ttggtggtgt agggtggtat    1500
```

```
gagagattgt gatttgtttt tttatttttg tttttgtatg tgagtgtttg tatttagtag    1560
aagtatatat tatatttttt taatttaggg taaataggag gggttatatg tataggtaat    1620
ttattaggga gttgaatatt tttgtgtaga tagattttttt tttttagtaa gttatggtag    1680
tggatagttt gttgagaata tttaggaagt aggtggtgtt agttgtaggt gttttgtttg    1740
ggagttgtgg ggttgaggag agggtttatt gtttaggatt agtgaatttt atttttattt    1800
gtttaggagg tggtttttttg gggatgttga gttaggggag gggtatttga ggaaagttag    1860
gtggagtaga gaggatgtga gtgattgggt gggtgagatt ttttgttttt ttttttgtag    1920
tggtatttat atttagattt gtggggtaat tgaggtatag atagagagta atttttttagg    1980
tttttatagt tggtaatttt aggattagga tttaagtgtg atttttaggt agtttttgta    2040
ttttgttttt gttgtatttg ttgtattatt tttaggtatt gtttattgtg ttattagtga    2100
tatgaattta ggtttaatat gttttggggt tattaaagtt tgatgttatt atgatttgat    2160
gtgtgatgtg ttataggtgt tttttggtat ttttatggaa ttggttttag gattttaaaa    2220
tttgtgggtg tttaagtttt tgagataaaa tggtgtaata tttgtatata atttatatat    2280
attttaaatt atttttagat tatttatatt taatataatg gaaatgatat gttggttggg    2340
tgtggtggtt tatgtttgta attttattat tttgggaggt tgtggtaggt ggattatttg    2400
aggtttggag tttgagatta gtttgattaa tatggtgaaa tttttatttt tattaaaaat    2460
ataaaaatta gttaggtgtg gtagtgtata tttataattt tatttatttg ggaggttgag    2520
gtaggagaat tgtttgaatt tgggaggtgg agtttgtagt aagttgagat tgtgttattg    2580
tattatagtt tgggtgatag agtaggattt tattttaaaa aaaaaagaga aaaagaaaaa    2640
gaaatgttat gtaaatagtt gtgattttga attgtttagg gaataataag aaagaattat    2700
ttgtagatgt ttagtataga tgtatttatt gtaagtttaa ttatattgta taatttagta    2760
atgatgtaat attttttaggg gtttttttgt tttgtttttt gagatagaat tttagtttta    2820
ttttgttatt taggttggag tatgttggtg tgatttttgt ttattgtaat ttttattttt    2880
tgggtttaag tgattttttt gttttagttt tttgagtagt tgggattgta ggtgtgtgtt    2940
attatgtatg gttaattttt gtatttttaa tagagatggg gttttattat gttggttagg    3000
ttggttttga atttttgatt ttgggatttg tttatttggg tttttttaaag tgttgggatt    3060
ataggtgtta gttattgtgt ttaatatatt ttgatttttg gttggatatg gagggttgat    3120
tgtatttaat atttttaagt tttagttttt ttttttaaaa taaaggtgtg gttgggtgtg    3180
gtggtttaag tttgtaattt tagtatttag ggaggttgag gtgggtggat tagttgaggt    3240
taggagttta agattagttt gattaatatg gtgaaatttt tttttttgtta aaaatataaa    3300
aattagttag gtgtggtggt gagtgtttgt agttttagtt atttgtttga atttgggagg    3360
tagaggttgt agtgagttga gattgtgtta ttgaatttga gtatgggtaa tagagtaaga    3420
ttgttttaaa aaaaaaaaaa aaaaggggggt gagtagatgt ggtggtatgt ttttatagtt    3480
ttagttattt agtaggaggt taaggttgga ggattgtttg attttaggag tttgagttta    3540
gtttgggtaa tatggtaata ttttattttt aaaaataaaa taaaagtaaa ggtattaatt    3600
attattttgg atggttgttg taaagaaata tatataaaat aatggagagt tttgtaattg    3660
gtttttaaga ggtttaatag atattattgt ttttgttttt ttattatgag ttatttttttt    3720
ggttatttta ttgaattagt tgggttagtt gagtttggga gaagagttgt ttaggaagtg    3780
agaggttgtt ttttatagag atttaaggtt tagttttttt tggtgattta gatgggtagt    3840
ttagtgggta tatgtggttt ttttttatat gtggttgagt tttattttta gaatagatgg    3900
agaggtaagg gtagggttta gtatgtttga ggaattttag agggttttgg tggtgtgggg    3960
gatttttaga atataggtgt tttaagggtt gatttagttt ttgtgttttt tttttttgggt    4020
gaggaggggga tatttatggg tagatggtga tttttgggga aggtagttta gattttattg    4080
gttattatat tttttttttt ataatttttt tatttttgtg gttttttatg ttattatgtg    4140
gttgtttttt gtaaggtttt agtggggtgt aggtatgaat atagtgttag gtaaggaggt    4200
atttggaggg gaattttggt ttttttttggg gggatttttt ttttgtattt tagttttgtt    4260
ttttttttatg gttattgatg tttttttttt attttagagg tggtttatat ttgtatagat    4320
tagatttata aaaattatgt ttttttgatt tttataagtt tgtttagtga ggtttaggta    4380

ttaggttatg tgttggggat ttagatttat atatatatgt atgttagtat ttatgtttat    4440
aggtttgtat atgttggggt aagtgttata tatggggtgt tgtaggaagt tgatttttag    4500
tttttgtaga tttttgtttg tttggatagg gaggtgttga gaaggtttag gtagttttgg    4560
gttaggattt tggtttgggg ttagggtatt gagtgatttt agaattaagg gtggtgtggg    4620
tttaagtagt tgttagatgt tttttggtat tttgtaggta gattatgtgg attttggtga    4680
gttgggtggt tttaatagta gggttggtgg ttggaatgtg gtgtttagat ggttagtttt    4740
gttttgtggt ttgttgtttg gattttggag gagttagtta tagttgttgt tgtttttttt    4800
tggtgagtgt tttttagttt aggtaagagt tggtagtttg ggtttttttta aagggttatt    4860
ttggattggt tagaggagga tgttaggtat aagtttgtgg tttattattt tttttgtttt    4920
tttaggataa atggtttata atattgagta ggtatttggg tggtttttgt taggttgatg    4980
tttattgttt tgttggttat ttttgtattt ttattgtttt agggattttt agttgttgtt    5040
tttttttaga ggtgagtgtg ttattagttt agtggagggg tttaggtttg tatttatgtt    5100
```

```
tttttgtat tttattattt gtagataaaa gggttttgtt aatgtaggtt tttttgtgtt 5160
ttataggttg tggtatgtgg ggatggttat tattgttgtt tatggggttt ttattgtagt 5220
gtagatgggt gattttgttt ttaaagatta ggtgtagttg gggtgtgggt gtagggtagg 5280
tagatgggta gtatgtggag tttggaattt aggagtttag ttggtggggg tagttttgat 5340
ttttgttttt gtgtttttat ttatgtggta tttgtattaa gtaatagttt tgttgtggat 5400
agaggggtag tattggggat aggagggtgt gggagaaagt gtaagatttt aggtttaggt 5460
gttgtggggg tggggagagg ttgagttggg ttggtttaat attaatttat ggttagtggg 5520
tgtttttttt ttatgttatt ttgttgaggg agggattgga ttgtgaggag ggtgagttag 5580
gtttgtttag gagattattg agttttagtg ttatttttaa attttagtag ttgggtttgt 5640
aggttttggt gttattagtt ttttgtgtga tgggggagtt atttttttttg agtgggttgg 5700
tagtattttg ggttattttg tttataggta ataattttgt gggtgttatt tagtgttttg 5760
atagttagtt tgaatgtttg gattttttta tgtgttgtgt tatggttgat ggtttttggg 5820
ggtgttgttt tatgttttag gtataaattt gggggagatg ggggtatgtg gagggaagtg 5880
ggggtagagt tgggggttag gggtaggggg tgaagatgga gttaggatta tttttttttta 5940
ggtttttttgt tgtgaagata gggtgtattg ttgtttgtat ggtgtttttt gtgatttggt 6000
ttatatttgt tgtattatat ttatgggtat ttattttttg gtaaagaagt tttttgttta 6060
gaggattaat agggtaggtg aggaggtggg agagtattag gttaggggtt ggggtggggt 6120
tttattgatt ttaagtgtag gaaaaagttt tttttatttt ggttgttttt tatgtttgtt 6180
ttttttttag tggttttgtt tagtttggtt atgtgtttgg atgtatggtt ttggtgtttt 6240
gatggtttta tttgttgtga gttgtttagt gggaagtatg gttgttgttt aatgtttaat 6300
gtgagtgagg ggttggagtt agtttggttg tgtgttttta gttatttggt tttgatatgt 6360
attttatagg ggttttgtgg tatggggttg gttggttgtt tgttgggagt ttggttgatg 6420
tagggtttat gttatttttt agtgggggat tggggtagtg ttagttatta gtttggttgt 6480
tttttgtgtg ttattgagtt tggaagtgat aaagatttat ttttgttttt atttaggtta 6540
tttgttgttt tgattatttg tattgttgtt tttaagatat tgtgtgtgat ttgatttaga 6600
gtaagtgttt ttttaaggag aatgttatta tggatttttt tattaagttg tttgtgtata 6660
taggtattag aggtagggtg tagatatagg ggtggggttt tttttttttt tttttaggtt 6720
tggttttagg attattgtaa ggtggtgtaa gtggtatttt ttatttttaa tatttggttt 6780
tagttgtgga gttggtaaag ggttgatatt tttgagggtt tttagtgtta tttttgattt 6840
gttttttttg ttttttttat agtgggggat gtgaaatgtg atatggaggt gagttgttta 6900
gatggttata tttgttgttg tttatagttg ggggtttggg gttgttgttt ttttatttag 6960
gtatttaggg gtggtgggtg ggtgggttga gtatagtgtg gtaggtagtt gggttttagt 7020
gtttatttgt ttttttttat ttgttttagg ttgtgtgttg tgaggattat atatattgtt 7080
gttttgtggg gtttatgtgt gatatgtaga agggtatttg tgaatagggg ttttattagg 7140
tgttttggat ggagaaggtt ttagtttatt ttagtttgtt agatttataa gttttgaaga 7200
gagatgtttt ttgtgataat gttagtagtt gttttttttt tgatatttgt tgttaattta 7260
tgtttgggga gtggggttgt tgtttaattt tagaggtata tgggaggggga tagtattttg 7320
gtttgggtag gtgggtggtt aagtttttat tgttttttgt tttttgtata gtttataggt 7380
gatatttagt tttgatagat ttgtttttag ttggaggtgt tgtaagtagg agaggtgggt 7440
tggagtaggt aggggtttgg tattgtgttt tatatagtgg ttatttataa tgtttttttt 7500
tgtttatttt ttaggttgtt tgttgtttgg attattagta ttgttgtttt tagggttata 7560
tgtgtgtagt tgaggggtag tgttagtgag gaagtgagat tgtggttgga ttggagaaga 7620
tgtttgtttg ttgggttttt ttattttatt ttagagatat tggttgtgat tagtatatta 7680
gttgtttggt ggggtagatt tgttgtttga gtttgggtgg gagttgggtt tgttgttagt 7740
tgttttatgt gagtgttttt ttgtttgttt ttggataggg gagttaagtt tagtgagggg 7800
ataggaatat aatgttattt tgtgtttttt tttttgttag gttgtgtgtt gtgaggattg 7860
ttagtattgt tgtttggttg gttatatttg taatgtgaag gtttgatttt gtgagaagga 7920
agtggttttt gtttagtttg ttattttttt ggtttgtagt ttttatgtgg gtgtgaagga 7980
tgtggagtgt ggggaaggat atttttgtta tgataattag atttgttgtt gagataattg 8040
atagggttgg gtttgttgtt tttattgtta ggttagtgtt aatttttatt ttggggttgg 8100
gtatggttag ggattaggtt ttattttgtt taattttttt gttttttttt gattatttag 8160
ggtgtttgtt gtgttgattg gtgttattgt tgttttgttg gtttttgttg tgtagttagg 8220
ggtattaagt gtttgtgtag ggaggttttg tgttgggatg tttttttgag ggatttagtt 8280
ttgagatagt tgttgtgagg gatagtattg aagattttgt agtttttggg attttatttg 8340
gagggtgttt tttgtttagg tttttttagt attttttttt aattaaattt tttttggatt 8400
ttattttgag tttttttatta ttatgggagg tggggtttta atttaaggtt ttttttgtta 8460
gaagggggtt gtggtaaaag ttatattata agttgttatt ttttttttgt tttagtggat 8520
tttgtggtta ggtgtttttt tttatttata ggggtgtttg tgtgtgtgtg tgtgtgtgtt 8580
ttaataaagt ttgtatattt ttttaatagt gtttgatttg ttgttttgtt tgtttttttt 8640
agggttttag aatagggggtt tatgtttatt gttaatattt tttttttttta ttttatagaa 8700
agatatatat agttttaatt ttattagttt tatatttgtt gttgtttata ttggttgtat 8760
```

```
tatattattt tttagttttt agagttgttt tagttattag ttttgtgata ttgtagttta     8820
gagatgggtt agttagtaag tagtattttt tttttttttta ggggtttata aagaatgttt    8880
tttttttttt tagtttttat attagtagtt gaggttgggt tatttttttt gtttttttat     8940
aatagagttt tttatgtata gttatgttta tataggtatt gttttttttt agttttttttt    9000
tttggtattt ttttgtgggg gtttggattt tttttttttt ttggtttgga ggtagatata     9060
gggtttttttg taagatatga tttagtatta attatggaat agttttaagg tttttgggtt    9120
tttttttggt ttggggttgg gagttatgtg tgagggtttt tgtgggtttt tggggtgtgt     9180
attttgggtg tgggtttgtg tgggaggggt ggtgttaggt tttgtgtggg tgttatttga     9240
tgttgaatat tattagtggt tgtttttttt gttgttgtta tgggtttttt ttgttttttg     9300
ttttgttgtt tatttttgtt tttagtttgt tttttgggtt ggtgagtgag ttgtgttgta     9360
gtttgttggt gttgttgtgg gagttgtttt tgtggttgtg gttttgtttt tggggtattg     9420
tggttgtttg gtttttgggt gttgttattt tgtttagtag tggtgttagt gagttgtttt     9480
ttggagagta gaggtttgtg tggtttttgt tgttgttggg ttttgtgttt ttgttgagtg     9540
ttaggtgtgg gggtgtgggt ggtggtggtg gtgtggtagg tggggtgtgt ttttgttttt     9600
tttttgtggg ttggtggtgt gtgggttgtg ttttgtggat atttatgttt gagtttaggt     9660
tggtgttggt gttgtgtgtt ttgttgtttg tttgtaggtt gatgtaagag tggtgtattt     9720
gtgagttgga gtgtttgttg agggatatga attaggtgtg tgggtgtggt tttatgttta     9780
gttttagtag ttttttttttg gttagggttt gtagtttttt gttgagttgt gttatggtgg    9840
atttgttgaa tagtataggg atggtgattg agttattgat gggtgttgtg tgtttggttt     9900
tttagttttt gttgttgttt ttgttgtttt tgttttttgg gtttgagtgg tttggtattt     9960
gtgggtgttg ggggttgggt tggggaaaag tgtgtgttgg gttgggtgtt gtgttttttg    10020
gtggggttgg tttgttgttt atgttggtgg tgtttgtttt gttgttgagg tgtatgtagt    10080
gtgtggggat tattagggtg ggtatgatgt tgtggtagat gttgttttttg aggtggttga   10140
tggagttgta gaagatgagt tgttttgttt ggtttagtga tggtggttgt gttagttggt    10200
ttattgattg tgatagtagg aagttggggg ttttgttttt ggttgttttt ttgtggttta    10260
ggtagtggtt gaagggtggt ggtggtgagg gtggtttgtg taggaaggtt gtagtttttg    10320
agggtttttg ttggtgtttt tttaggttgg gttttagttt gttggggttt ttggttgagt    10380
gagtgttttt gagtttggtg tttttgtttt tgtgggtatt tgaaggtttg gtggttgggt    10440
ggttggtaga gtgtggtttg gtgtggaaga agttattttg ggaggaggtt aagttttggg    10500
agttggagaa ggttgagtgg agggggtttg gaggtggagt tttggggttt tttgatgggg    10560
tgggttttag gggtttttta tagatgaggt ggagttggga tattgaggtg gtttggtttt    10620
gtttgttatt gttagagggt tttgagagtt gtagtttgtg gtgttgttgt tttggtttta    10680
ggtagtgttt tttggaaggg agggagtaga aggggttgtt taggaaaggg tgagggttaa    10740
agtaggtttt gaattttttt agatgtaaga taagagatga atatagtgga agaggtgggg    10800
agaatgtttg gggtgtttag gagagatggt ggggagttgt tgtgtttgtt tggtgat       10857
```

<210> 270
<211> 10857
<212> DNA
<213> Artificial Sequence

<220>
<223> chemically treated genomic DNA (Homo sapiens)

<400> 270

```
attgttaaat ggatatggtg gttttttgtt attttttttg agtgttttag gtattttttt       60
tattttttttt attatatttg tttttttgttt tgtatttaag ggggtttggg gtttattttg     120
gtttttattt ttttttgagt ggtttttttt gttttttttt ttttaggagg tgttgtttga     180
agttgaggta atagtattgt aagttgtagt ttttggggtt ttttgatggt aataaatgag     240
attaggttat tttgatgttt tagttttatt ttatttgtgg gggattttttg gaatttgttt    300
tgttggggga ttttgaggtt ttgtttttag gtttttttta tttggttttt tttagttttt     360
gggatttggt ttttttttgg gatgattttt tttgtattaa gttgtgtttt gttagttgtt     420
tggttgttga gtttttgggt gtttgtgggg gtgagagtgt tgggtttaag ggtgtttgtt     480
tggttgaggg ttttggtggg ttggagtttg gtttagagga gtattggtgg gggtttttgg     540
gggttgtggt ttttttgtat gagttgtttt tgttgttgtt gttttttgat tattatttgg     600
gttataaggg ggtggttgag ggtaagattt ttgatttttt gttgttgtag ttggtggatt     660
agttggtgtg gttgttgttg ttgggttagg tggggtagtt tattttttgt ggttttattg     720
attattttaa ggataatgtt tattgtaatg ttatgtttat tttggtgatt tttgtgtatt     780
atgtgtgttt tggtggtgag gtgggtgttg ttggtgtggg tgatgagttg gttttgttgg     840
agggtatggt atttggtttg gtgtgtgttt ttttttagtt tgatttttag tgtttgtaga     900
```

```
tgttgggtta tttgggtttg gggggtgagg gtggtggggg tggtggtgag ggttgggggg     960
ttgggtgtgt ggtgtttgtt agtggtttag ttattatttt tgtgttattt aatgagttta    1020
ttatggtgta gtttaatggg gagttgtagg ttttgattga gaagaagttg ttggaattgg    1080
gtgtgaagtt gtatttgtgt gtttggtttg tgttttttga tgggtgtttt aatttgtaag    1140
tgtgttattt ttatattgat ttgtaggtgg gtggtggggt atgtagtatt gatgttagtt    1200
tggatttggg tgtagatgtt tatgaggtgt ggtttgtgtg ttgttggttt gtgagggagg    1260
agtgggagtg tgttttgttt gttgtgttgt tgttgttgtt tgtgtttttg tgtttggtgt    1320
ttagtgagga tatggagttt agtagtagtg agagttgtat gggtttttgt tttttggagg    1380
ataatttgtt gatgttgttg ttggatgagg tggtggtgtt tgagggttgg gtggttatgg    1440
tattttgggg gtggggttgt agttgtgggg atagtttttg tagtagtgtt agtgagttgt    1500
ggtgtgattt gtttattagt ttggaggatg agttgggggt ggaggtgggt gatgaggtgg    1560
gagataagaa gagtttgtgg tagtggtggg aggagtggtt gttgatggtg tttaatgtta    1620
agtagtgttt gtgtagggtt tggtattgtt ttttttgtgt gggtttgtat ttagggtgtg    1680
tgttttgggg gtttgtgggg gtttttgtgt gtagttttta gttttaggtt ggagaggggt    1740
ttaggggttt tggagttgtt ttgtggttgg tgttgggttg tgttttgtaa gggattttgt    1800
gtttgttttt aagttgggga ggaggggggg tttagatttt tatggggagg tgttggggag    1860
gagggttggg gggaagtagt gtttgtgtag atgtggttgt atatagaaag ttttattgtg    1920
ggaaagtagg aggggtgatt tagttttagt tgttagtgtg agggttggga agggaggggg    1980
tgttttttgt ggattttttgg gaggggaggg ggtgttgttt gttgattagt ttatttttgg    2040
gttatgatgt tatagggttg gtggttggga tagttttggg gattaggggg taatgtgata    2100
tagttgatgt gggtagtagt aaatataaaa ttggtgaggt taaggttgtg tatgtttttt    2160
tgtggggtag gggaggggag tgttggtagt ggatgtgaat ttttgttttg gggttttggg    2220
gagggtaggt agggtggtaa attagatatt gttaagaaag tgtataaatt ttattgaaat    2280
gtatatgtgt atatatataa atatttttgt ggatagggaa aagtatttgg ttatagggtt    2340
tattgaaatg gggaggggat ggtagtttgt aatgtggttt ttgttataat ttttttttga    2400
tagggaaggt tttagattga ggttttattt tttatggtga tggggagttt agaatggggt    2460
ttagggagaa tttggttagg gggaggtgtt agggaggttt gagtagaggg tattttttga    2520
gtggggtttt gagggttgta gagtttttag tattgttttt tatagtagtt gttttaaggt    2580
tgggtttttt aaaggggtgt tttagtgtgg ggttttttttg tgtaaatatt tggtattttt    2640
ggttgtgtag tggaagttag taggatagta gtggtgttga ttagtataat agatgttttg    2700
gatggttaga ggggggtgag agggttggat gaggtgggat ttggtttttg gttatattta    2760
gttttaggat gggggttggt attgatttgg tggtagggat agtaggttta gttttgttgg    2820
ttgttttggt agtaggtttg gttattatgg tagaagtgtt ttttttttata ttttatgttt    2880
tttatattta tgtgagggtt atgggttagg aaggtggtag gttgggtaga gattattttt    2940
tttttgtagg attgagtttt tatgttgtag gtgtagttag ttgggtagta gtgttggtga    3000
tttttgtagt atatagtttg gtggggaagg gagtatagaa tggtattatg tttttgtttt    3060
tttattgggt ttagtttttt tatttagggg taggtaggga ggtatttata tggggtaatt    3120
ggtagtaggt ttagttttta tttaggtttg ggtagtaggt ttgttttatt gggtagttgg    3180
tgtgttggtt atagttgatg tttttggggt gggataagga agtttggtgg gtaggtattt    3240
tttttagttt agttatgatt ttgttttttt gttgatattg ttttttagtt atatatgtgt    3300
agttttgggg gtagtagtgt tggtggtttg agtagtagat agtttggggg gtgggtagga    3360
aagggtgttg taggtagtta ttatgtgggg tgtagtgttg agtttttatt tattttagtt    3420
tgtttttttt gtttatagta tttttagttg gggatgaatt tgttagagtt gggtattatt    3480
tatgggttat gtggagggta gaaagtaata ggagtttggt tatttatttg tttaggttaa    3540
gatgttgttt ttttttatat atttttggga ttggatagta gttttatttt ttagatgtga    3600
gttggtagta ggtattggag gagggatagt tgttgatatt attatagggg atattttttt    3660
ttaaggtttg tgggtttggt aggttgaggt gagttggggt ttttttttatt tagggtattt    3720
ggtggggttt ttgtttatag gtattttttt gtgtgttata tgtaaatttt gtgggatagt    3780
agtgtatgtg gtttttatag tatatagttt agggtagatg aagaagggta ggtgggtatt    3840
ggggtttggt tgtttgttat attgtgttta gtttatttat ttgttatttt tgggtatttg    3900
ggtaaaaggg tagtagtttt aggtttttga ttgtagatgg tagtaggtat agttatttgg    3960
gtagtttatt tttatgttat attttatatt ttttattgtg agggaagtag agaggatagg    4020
ttagaagtgg tattggggat ttttaggggt attaattttt tgttggtttt atagttggaa    4080
ttaggtgttg aagatggagg gtattgttta tattattttg tagtgatttt aaggttaggt    4140
ttaaaaggga ggaaaggggg ttttattttt gtatttgtat tttgtttttg gtatttgtgt    4200
gtgtaggtag tttagtgagg aggtttgtgg tagtgttttt tttggagagg tatttatttt    4260
ggattaggtt atatatagtg ttttgggggt agtagtgtag gtgattggag tagtaggtgg    4320
tttgagtggg gataggggtg ggtttttgtt atttttaggt ttagtagtat atagggagta    4380
gttaggttga tggttggtat tgttttaatt ttttattagg gggtagtatg aattttgtat    4440
tagttaggtt tttagtaagt agttagttag ttttatgtta tagagttttt gtaaggtgtg    4500
tgttagggtt aggtggttgg gggtatatag ttaagttggt tttagttttt tatttatgtt    4560
```

```
gggtattggg tagtagttat attttttatt gggtagttta tagtaggtag aattattagg    4620
gtattgggat tgtgtgtttg gatatatgat tgagttggat aaggttattg gaagaggagt    4680
aaatgtgagg ggtagttagg atggaggaaa ttttttttta tatttggagt taatgaggtt    4740
ttgttttagt ttttggtttg atgttttttt attttttat ttgttttgtt agttttttgg     4800
gtagggagtt tttttgttag ggggtgggtg tttgtgggtg tgatgtagtg ggtgtgaatt    4860
aggttgtaga aggtattgtg tggatagtag tgtattttgt ttttatagta ggaagtttga    4920
gaaaaaatgg ttttgatttt gtttttattt tttgtttttg gtttttaatt ttgtttttat    4980
ttttttttat atatttttat ttttttaga tttgtatttg gggtatgggg tagtattttt     5040
aggagttatt gattataata tagtatgtgg agaagtttgg gtatttgaat tgattattag    5100
ggtattggat ggtatttatg gagttgttat ttgtggataa gatgatttag gatattatta    5160
gtttatttag ggaaggtgat tttttattta tataaggagt tggtggtatt agggtttgta    5220
agtttagtta ttggggtttg agggtgatat taaggtttag tgatttttta ggtaggttta    5280
atttattttt tttataattt agtttttttt ttagtaagat ggtatgggga aggggtattt    5340
attgattatg ggttggtatt agattggttt agtttgattt tttttattt ttataatgtt     5400
tggatttgga gttttgtatt ttttttgta tttttttatt tttagtgttg ttttttttgtt    5460
tatagtaggg ttgttgttta gtatagatgt tatatgaatg agggtataag ggtaggaatt    5520
agggttgttt ttgttagttg ggtttttggg ttttagattt tatatgttgt ttgtttgttt    5580
gttttgtatt tatattttag ttgtatttga tttttggaag taggattgtt tgtttgtatt    5640
gtagtggaag ttttgtgggt agtagtgatg gttattttg tatgttatgg tttgtggaat     5700
atagagaaat ttgtattggt agggtttttt tatttgtagg tggtagagtg taggaaaagt    5760
ataaatgtag atttaagttt ttttattggg ttgatggtat gtttattttt gggaaggggt    5820
agtaattgga agtttttgag atggtaaaga tgtaggagtg gttggtagag tagtgggtat    5880
taatttggta ggggttattt agatgtttgt ttagtgttgt gggttatttg ttttagaaag    5940
atagggaaaa tgataaatta tagatttgtg tttggtgttt ttttttggtt aatttaagat    6000
gattttttgg gaaaatttag gttgttagtt tttgtttagg ttgaggggta tttattagaa    6060
ggggatggta gtagttgtag ttggtttttt tggggtttag gtagtaggtt atagggtaga    6120
attgattatt tgggtattgt gttttagtta ttagttttgt tgttaaggtt atttagttta    6180
ttagggttta tatggtttgt ttgtaaagta ttaaggaatg tttggtaatt gtttaagttt    6240
atgttatttt tgattttagg gttatttagt attttagttt taggttaagg ttttggttta    6300
ggattgtttg agtttttta atattttttt gtttaggtag gtagaaattt gtaggggttg     6360
agagttagtt ttttatagtg ttttgtgtgt gatatttgtt ttagtatgtg tggatttgta    6420
agtatgaatg ttgatatgtg tatatgtgtg ggtttgagtt tttagtatat ggtttaatgt    6480
ttgggtttta ttgggtaggt ttatgagagt taggaagatg tgatttttgt gggtttgatt    6540
tgtgtagatg tgggttattt ttggggtgag gggaaggtat tagtagttat gggagaggat    6600
agggttaggg tgtagggagg gagttttttt aggaaaagtt agggtttttt tttagatgtt    6660
tttttgtttg atattatgtt tatatttgta ttttgttaag gttttgtggg aagtggttat    6720
atgatgatat gggaaattat aggggtgaga aagttgtgaa aaaggaaata tggtggttag    6780
tggagtttgg gttgtttttt ttagaggtta ttatttgttt atgaatgttt ttttttttatt    6840
tagagaaaag gatatagaag ttgggttagt ttttgagata tttgtgtttt gagggttttt    6900
tatattatta gggtttttttg agatttttta agtatgttaa attttgtttt tgttttttta    6960
tttattttgg aagtgaaatt tagttatatg tggagagaga ttatgtgtgt ttattgggtt    7020
gtttatttga gttattagga ggaattgagt tttgagtttt tgtggagagt agtttttttat   7080
tttttaaata attttttttt taggtttagt tagtttagtt agtttagtgg ggtggttaga    7140
gaggtaattt ataatgaaga agtaaaaata gtaatgtttg ttgagttttt tgggagttag    7200
ttataagatt ttttattatt ttatatatat tttttgtaa taattattta aagtagtaat     7260
taatattttt atttttattt tatttttaga gatgaggtat tgttatgttg tttaggttgg    7320
atttagattt ttgggattaa gtaatttttt aattttggtt ttttattaag tagttgggat    7380
tgtgggagtg tgttattatg tttgtttatt tttttttttt tttttttttt gagatagttt    7440
tgttttgttg tttatgtttg agtttagtgg tatgatttta gtttattgta atttttgttt    7500
tttaagttta agtaagtagt tgggattata ggtgtttgtt attatgtttg gttaattttt    7560
gtatttttag tagagagggg gttttattat attggttagg ttggttttga atttttgatt    7620
ttagttgatt tatttatttt agttttttta agtgttggga ttataggttt gaattattat    7680
atttagttat atttttattt taaaggagga aattgaagtt tagagatgtt aagtatagtt    7740
agtttttat atttaattag ggattaaaat atattgggtg tggtggttaa tgtttgtaat     7800
tttagtattt tgggaggttt aggtgggtgg attttaaggt taggagttta agattagttt    7860
gattaatgtg gtaaaatttt atttttatta aaaatataaa aattagttat gtgtggtagt    7920
gtatatttgt aattttagtt atttaagagg ttgaggtagg agaattgttt gagtttagga    7980
ggtggaggtt gtagtgagta gagattatgt taatatattt tagtttgggt gatagagtga    8040
gattgagatt ttgttttaaa aaataaaata aaaaaatttt tgaaaatgtt atattgttgt    8100
tgagttatat aatgtagtta ggtttatgat gggtgtattt atattgaata tttatagata    8160
gttttttttt attatttttt aaataattta ggattataat tatttatatg gtattttttt    8220
```

```
tttttttttt tttttttttt tgagatggag ttttgttttg ttatttaggt tgtagtatag    8280
tggtgtgatt ttagtttatt gtgaattttg ttttttaggt ttaagtaatt tttttgtttt    8340
agttttttaa gtaggtggga ttataggtgt gtgttattat atttggttaa tttttgtatt    8400
tttagtagag atgggggttt tattatgttg gttaggttgg ttttaaattt tagattttag    8460
gtgatttatt tgttatggtt ttttaaagtg gtgggattat aggtatgagt tattatgttt    8520
agttgatatg ttatttttat tgtattaggt ataagtaatt tagaaatgat ttaaagtatg    8580
tataggttat atgtaaatat tatattattt tattttaggg gtttgagtat ttatagattt    8640
tggggttttg gaattagttt tgtgaagata ttaagggata tttataatat gttatatatt    8700
aggttatggt gatgttaggt tttgatggtt ttagagtgta ttggatttgg gtttatatta    8760
ttagtggtat gatgggtagt gtttgggaat ggtgtaatag gtataataga aatagggtat    8820
agggattgtt tgaaaattgt atttgggttt taattttaga attgttaatt gtgagggttt    8880
gagaagttgt tttttgtttg tgttttagtt attttatgag tttaggtgtg gatattattg    8940
tgggggggagg ggtaggaaat tttatttatt tagttattta tatttttttt gttttatttg    9000
gttttttttta agtgtttttt ttttaattta gtatttttaa gaggttattt tttggataga    9060
taaggatgaa gtttattggt tttggatagt ggattttttt tttagtttta tagtttttag    9120
gtaaagtatt tgtagttggt attgtttgtt ttttgggtgt ttttagtagg ttgtttgttg    9180
ttatggtttg ttgggaaggg gagtttgttt gtataggagt gtttggtttt ttggtgaatt    9240
atttgtgtat gtggtttttt ttgtttattt taaattgagg gagtatagtg tatgttttta    9300
ttgaatataa gtatttgtat atagaaatag ggatgaggag gtgagttgta gttttttatg    9360
ttattttata ttgttaaaat agttagaggg gatagtagtt tttttaagat tttagttttg    9420
ttattttttta ttaggtggta tttttttaatt ttgagtgtta aggggagagg aaatttttaa    9480
aaggggagtt aatttagttg taaatgagta ggaaaaagtg gttgggagag gtggaagtta    9540
gtttgtttta tttgtaataa gaggaagtaa ggaggagtta gattaggttt agttattatt    9600
tttaagtttt tattattttt gagaagttaa ggttttaggt tttgttttta ggtttttgga    9660
gtttttagtt tagggttgtg tgtttttttg gttttaggtt gttgtgggaa ttatttatta    9720
ttattaggag aggggaagaa gttagtattt attgataggg gtggagttgg gttaagaatg    9780
gtgtggtttt tgttttgggg gaatgttggg gaggtagaaa gtttttttta atggggtgtt    9840
attgtaatta ttgttttttt tttttataa aattttttttt agtgtattag aatttttaag    9900
gagttttagt aagtggtttt tttgttgttt ttggttgaga ttttagggga attttaagtt    9960
tatatggttt tggtgggttt ttgggtagga gtaggtgaga ggtttgtgtg gttgtttttt   10020
tatttgtgtt tgattttgtg gtttttgggt agtagtaatt gggtagtgtt tagattatag   10080
attttagtgt gatgatttgt ttattttagt ttttattggt tagggattag ggttatttgt   10140
ttttattggt tatttattaa tatagaggtg gggttagttt ggaatgttgt gttttttttt   10200

tatttaaatt attttttttt ttgttttttt ttgttttata ttttattttt agggattatg   10260
tgattggaga attatgtgat gttgggatag ttttagtagg gtagttggtg tttgttgttt   10320
tttttaattt attttgtagg tgtttggggg agtttgttag agatgatagt gtgtgtttgg   10380
ggtattgata gtgtgaggga gtgttatgaa ttgggaatat ttgtgtttgt ttttttattt   10440
ttttgtttgt ttgttttgag atggagtttt attttgttgt taggttggag tgtagtggta   10500
tgattttggt ttattgtaat ttttgttttt tgggtttaag tgattttttt gttttagttt   10560
tttgagtagt tgggattata ggtgtgtatt attatgttta gttaattttt gtatttttag   10620
tagagatggg gttttattat gttggttagg atggttttga ttttttgatt ttgtgatttg   10680
tttattttgg tttttttaaag tgttaagatt ataggtatga gttattgtgt ttggatttgt   10740
gtgtttgttt tagagatagg gttttgttta gttgtttaag ttgattatag tttattgtgg   10800
tttgtaattt ttgtttttaa agtgattttt ttattgtagt tttttaaggt gttggga      10857

<210> 271
<211> 5465
<212> DNA
<213> Artificial Sequence

<220>
<223> chemically treated genomic DNA (Homo sapiens)

<400> 271

ttttggattt ggtaaggtgt ggtggtttat gtgtataatt ttagtatttt gggaagttta      60
ggtgggtgga ttatatgagg ttaggagttt tagattagtt tggttaatat ggtaaaattt     120
tatttttatt aaaaatataa aaattagttg ggtatggtgg tgtatatttg tagttttagt     180
tatttgggag gttgaggtag gggaatagtt tgaatttggg atgtggaggt tgtagtgtgt     240
taagattgtg ttattttgtt ttaatttgtg taatagagtg agattgttta aagaaaaaaa     300
```

```
aaaaatttta gatttaatgt tagttgtttg tgttagtttg gggatttttt atttataggg     360
tgtagtttgg ttaagaggaa ggagttatat aatatggttg atggaatttt taatatttaa     420
gtttaggggt atatgtgtag gatgtgtagg tttgttatat aggtaaatgt gtattatggt     480
ggtttattgt atagattatt ttattattta ggtattaagt ttagtatttg ttagttattg     540
tttttgatgt tttttttttt tttatttttt atttttttgat atgttttagt ttgtgttgtt     600
ttttttatgt gtttatgttt ttttaggttg atgtggtttt gagtgatttt tttttgtttt     660
ttttttttaa aaataagatt taaaaggtta tgttttttat gggttggagg tgttgttttg     720
atttgttaag tatagtttag gatttagagg ttgttgtggt gaattattgt aggtatttgg     780
aagttattaa ggtgtttttt ttttgtattt tagttttaag tgagggaaat aagtttatgg     840
ttaagggaga attatttatt aatttgaatt tattaaaata tataaatgtg tttattagaa     900
atggttggtt ggagaagttt agtattaaat tattttgttt ttgaaattta ttagttttat     960
gaaatagttt gttagagaga gatttgtaat ttttgaattt gttgattatt aatttgtata    1020
ggtgttttgt gttttgagat ttgagaaaat gataaaatat gtttattttt tggttgggta    1080
tagtgattta tgtttgtaat tttaatattt tgagaggtta agttgggtag attatttgag    1140
gttaggagtt tgagattagt ttggttaata tggtaaaatt tttgttttta ttaaaaatat    1200
aaaaattagt tgagggtggt ggtgggtgtt tttaatttta gttattttgg aggttgagtt    1260
ataagaattg tgggaagtgg aggttgtagt gagttgagat ggggttatag tattttagtt    1320
tgggtgatag agtgggattt agttttaaaa aaaaaaaaaa agtatttttt aatttttttgt   1380
tagttttttgt tttttttttta ttttgttttt tttaggtttt tgaaaagtat tttttttaat  1440
ttttttgtg tatatgggga aaatatgtgt ttattttat tatggtgttg aaatttattt      1500
ttttttttaa attgtttttg tagattgttt tagtataaaa tgtggtaaga ggttaaaggg    1560
agagatatag attagttttt tggtttttgt tttttaattt taagttatat ttgtgaaatt    1620
tatgggtttt tttatagttt ttaaaaatta agggtgtttt ttgttttttt tttagatgtt    1680
ttttttttat tgttggtagt gagtgggaga tagtttagtg tggggtaggg gagtattggg    1740
tttggagatg gaaggtagtg ttaaaagtgt tgttggaaaa tttttgagtg ttaattgttg    1800
tttgtgtgag tttttaaatt tataaatttt taatatttgt agtttttggg ttttttagga    1860
tttttattga ttttggtggt agagagggta ggtttgagat gtagtgattt gagggtatat    1920
ggttaatttt tgttatttta agattatatt ggggaattag attgattttt ttaattttga    1980
atttgttttg gttttgggtg gtttaaaggg ttttttttgt tgtatttttg ttaaaattaa    2040
attgtttggt ataagttggt aagtaattat tttgttggga gagggaagga agagtaggtg    2100
tagttttaga ttaatttttt tgtattgttt tttttagatg gttaagttag ttgtgtttta    2160
ttgaaaaggg tgtattgttt atgtttgaaa tgtagttgtt gggggttgag aaattatttt    2220
tatttggttt gagggttagg gatgtaggag tgtagtagtg tggaggggtt ttgtgttggt    2280
ttggtgttgt ttgtggtttt gtttttgttt taagggtatg gtgttggtat gaggatattg    2340
atgttgtggt gtaattgttg tttttttgtag taattttgga gtttggtttt tggttgtttt   2400
ttggttttgt gtaggttgtt ttttttttgat gtggagtttt attttgttat ttgttgttta   2460
gatgatttta taaataagtt tttgaagtgt ggaggtagga ggtggggtgt agtgtggggg    2520
taggaggtgg gttagggtta gggtagaggt tgtggttgtg tgtttttatt ggttgggatg    2580
tagtgagttg tttggagttt ggtgtgggtg gggtttgttg gtgtgtgttt gtttatatat    2640
ttgtgttggt gtttgttttt tgttttttgt gtttgttttg tgtttgtttt aagttggttg    2700
atggagtttt taaagtgggt tgttggttgt gggagtttta tatttgtgag tggattgtta    2760
tatgggtttg gtgtttgttg tgtttttgtt ttagtgtttt tgaggtggtt gtataatttt    2820
tggtagtgtt ttgagattgt atggttagtt tagtttggtt tttgattttt tttgattttt    2880
agtatttgag ttattttttt ttttttttga aaatttagaa aagtgatttt tttttttaggg   2940
aaaaaggaat ttgggttttt tttttttttgt tttttttttg ggtttgatag tttttattta   3000
tttttttttt ggattttgtt tttgtgtgta ggtttttttt agttattttt ttttattttt    3060
gtttttgtat ttagtttgtt gtgtgttatt ttttatttga ttgtgtgggg tgtttgggat    3120
ttgtgtgtat tttggatttt tattatttgt ttgggttgtg gggagtggat gagggttata    3180
gtttttttatt tgttagggag tttaggtgtt tggtgtttga atgttttaaa gggttatagt   3240
gataatgata gttgataagg agaagaaaag gtaagtgggt gtttgggttg attagggggt    3300
tggtttgagg ttagggttgg gttgtgtggg gtaggtgtga ttgagagtgg ttgggagaag    3360
agtgttgaga ggtttttgga gtttgaggtt tggtttggag ggttgtgagg gggagagtat    3420
gtgtttggtt tgggggtgtg gattagtagt tttgtagtgg agatttttgt ggtttggagg    3480
agttggggat ttgtgtgtat ggtgaggtta ggaggttgag ggagatggtt tggaggttgg    3540
aggtgttttt gtggtgtttt tgaaaatttt ttagttgttt tgtagtagat tattgtggtt    3600
attggtgttt ggaaatgttt gtgggtgtat gtttttgatt ttttttggtt ttttattttg    3660
gggtagtatt tatgttttta ttttttttttg gatttgtgga gtttttttaa atgtgttttt   3720
gtgtttgtgt tgtggtttaa gtgggtaaag gggggtggga ggtggagagt ggtttaggga    3780
tatgatttta ttgaggagtt aggatttttt agagtgtttt gttgttgggt tttagatttt    3840
attgaaggtt ggagaattta tttttttgtg ttgtattttg attttgggga gagtaggtag    3900
tgagtgattt ggattgtttt gtgggggtag atggtttggt gtgtaggaag tggatggtaa    3960
```

```
tattggatgt ttttggtagg tttgggtgtt tgtttttgaa gaggatagag aagggtagtt    4020
atgatttgtt tgtttgtttt tgtgagtttt ttggtattgg gtgagaggta attttggtta    4080
ttttttgttg ttttttgtt ttttttggt tgtttttagt ttagttgggt ttggtgttat    4140
ttgagtgagg gtttgagttt tttgtgtggt tgtgtagaag taggtagggt tttaattttt    4200
gtaaatgtgt gtggtttgtt gtttgttttt tttttttttt tgtttttatt tgtgtggttt    4260
tagtggtttg gagtttttag ttttgtgttt ggttgtggtt tggtgaggtt atgttgtggg    4320
aagttggaat ttaatgtgtg gttggttgaa ttgtttgagt tgtgggaaat tagtggtgga    4380
gtggtggtat agaggtgtga ggatgaggag gattgatttg ttaagggagg gaggtgattg    4440
gttgggaggg tttattgttg aggttgattt gtttttgttt taatttttat tatgggattg    4500
gagttagaaa ttgtgtattt tttggttgaa agtagtggtt tttattttgg ggtattgata    4560
aggatttgat aaatgggagt gaattgtgtt tgttttggtt tggtgtttgg gttttttatt    4620
gtgggattgt taggggaggg gattgggttt tgtttttttt ttttggttgt agttttttttt    4680
tttttgttat tgttgtgttt ttagatttaa gataagtgag ggagttttga gattgtagtt    4740
ggtatatttt tagggttttt ggagtagttt tggggtttta tgttttttga aggtagtaga    4800
gtaaggaaaa gatttttaag attttagtgt gttttgtaaa aattaattat aatgttaata    4860
atgattgaat tattgaaaag taaagttata gatagtgttt gtagatgttt ttgggtttgg    4920
agttggggag ggaatttgtg tattttataa ttgtgatgtt tattgggaat gtggtaattg    4980
ttgaggtggg gtttgatagt aattttgata aatgtgtgaa gtgttagttt tttttgtgtt    5040
ttttagtgtt gggttttgtg tagttttgta ttgtatatat tgagaaattt gggagtaggg    5100
gaaaaatgat tttgatatat ttagttatag tatttggttt gttagtggat tgttggaaag    5160
agagaagaag tgggaatggg agtgggaaga atgttgtgta attagatttt taattattgg    5220
tgagagtggt tgttttaaga attattgtgg ggattgtttg tgaaatgttt tggaggttgt    5280
tgtgtgagtt gtggttttgg gttggtggtt atgtgagggt gtttgttttt tttgatttgt    5340
ttggattttt gtgttgtaga atggtagggt aaatagtgtg tttttttatta tttagagttt    5400
tttaaggggt tgtttaattt ttattgggtt aattagtttg agatttattt attttttgtg    5460
tttgg                                                                 5465
```

<210> 272
<211> 5465
<212> DNA
<213> Artificial Sequence

<220>
<223> chemically treated genomic DNA (Homo sapiens)

<400> 272

```
ttaggtatga aaagtaaatg agttttaaat tgattagttt aataggaatt ggataatttt      60
ttaaggggtt ttagatggtg aaggatatgt tgtttgtttt gttattttgt agtataaaaa     120
tttaggtagg ttgaagaggg tggatatttt tgtgtggtta ttagtttgga gttgtagttt     180
atatagtggt ttttaaggtg ttttgtaggt agtttttata gtggttttta aagtggttgt     240
ttttgttaat aattgggagt ttaattatgt aatgtttttt ttgtttttat ttttgttttt     300
tttttttttt taatagtttg ttaatgaatt aggtattgta gttgggtgtg ttgaaattat     360
ttttttttg tttttaagtt ttttggtata tatggtataa aattgtataa aatttggtgt     420
taaggagtgt aggaggagtt gatattttat atatttgtta agattattgt taggttttat     480
tttggtgatt gttatatttt tgataggtat tgtagttgta aaatatataa gttttttttt     540
tggttttgga tttgaaagtg tttgtaggtg ttgtttataa ttttgttttt tgatggttta     600
gttattatta gtattatgat tggtttttat aaaatgtatt ggaattttaa gagtttttttt    660
tttgttttgt tgtttttaaa agatatggaa ttttaggatt gttttgaggg ttttgggaat     720
gtgttggttg tagttttagg gttttttttat ttgttttggg tttgagggtg tgatggtgat    780
gagaaagggg ggattatggt taggaaaggg gagtagggtt taattttttt ttttagtgat     840
tttgtagtga agggtttggg tgttgagtta ggataggtgt gatttgtttt tgtttattaa     900
atttttattg gtgttttgag gtgggaattg ttgtttttga ttggaggata tatggttttt     960
ggttttagtt ttgtagtgga agttggaata agagtaaatt aattttggta gtgagttttt    1020
ttggttagtt atttttttttt tttagtgagt tggttttttt tatttttgta tttttatatt   1080
gttgttttgt tgttggtttt ttgtggttta ggtggtttag ttggttgtgt gttggatttt    1140
agttttttgt agtatagttt tgttaagttg tggttaagtg tggggttgga agttttaggt    1200
tattggggtt gtatgggtgg agataggagg agaaagggga taggtggtga gttgtatata    1260
tttgtagaag ttgaggtttt gtttgttttt gtgtggttgt gtagagggtt tgaatttttg    1320
tttgggtagt gttgggtttg gttggattgg gagtggttgg gagagggtga gagggtagta    1380
agaaatggtt agagttgttt tttatttagt gttgagaggt ttgtgagggt aggtgggtag    1440
```

```
attgtggttg tttttttttg tttttttttgg agatagatat ttggatttgt tagggatatt    1500
tagtgttgtt gtttgttttt tatatattga attatttgtt tttgtagggt aatttaggtt    1560
atttattatt tattttttttt agagttaagg tgtggtatga ggaagtgggt tttttaattt    1620
ttggtggggt ttggagtttg gtagtggggt gttttgaagg gttttggttt tttggtagga    1680
ttgtgttttt gagttgtttt ttgttttttg tttttttttg tttatttggg ttgtggtgtg    1740
ggtataggag tgtatttgga aggattttgt gaatttagag aaaaatagag atgtggatgt    1800
tgttttaaaa tgagagatta gagaaagttg aggatatgta tttataaata tttttaagtg    1860
ttggtggtta tggtaatttg ttgtggggtg gttgggagat ttttaaaggt attgtggaag    1920
tatttttgat ttttaggtta tttttttttgg ttttttggtt ttgttgtgtg tgtaaatttt    1980
tgattttttt gagttgtaga agtttttatt gtaaagttgt tgatttgtgt ttttaaattg    2040
ggtatatgtt ttttttttta tagtttttta aattgagttt tgagttttga agattttttta    2100
gtattttttt tttaattatt tttggttgtg tttgttttgt gtagtttggt tttggttttg    2160
gattggtttt ttgattggtt tggatgtttg tttatttttt tttttttttg ttagttgtta    2220
ttgttgttgt ggttttttga gatgtttaga tgttgagtat ttgggttttt tggtgggtgg    2280
ggggttgtgg tttttgtttg tttttttgtgg tttgggtggg tggtgaaggt ttgaggtgtg    2340
tgtaggtttt gagtgttttg tgtagttagg tggaaggtgg tgtgtggtgg gttaggtgtg    2400
ggggtggggg tggagaaagg tgattgggag gaatttgtgt gtggaggtgg ggtttgggga    2460
aggagtgggt ggaggttgtt agatttgaaa agaggatgga gagaagggaa tttaagtttt    2520
tttttttttg gaaaaggagt tatttttttg agtttttaaa gaaaaaaaaa agtggtttga    2580
atgttgggag ttggaaggag ttggaggttg ggttgagttg attatatagt tttaggatat    2640
tgttgaggat tgtatggttg ttttaggagt gttggggtgg aagtgtagtg ggtattggat    2700
ttgtgtggtg gtttgtttgt gagtgtaaag tttttgtggt tggtagttta ttttaaaaat    2760
tttgttggtt ggtttggggt gggtatgggg tgggtgtgga gggtgggggg tgggtattgg    2820
tgtgggtgtg tgggtgggta tgtgttggta ggttttgttt gtgttgagtt ttgggtggtt    2880
tattgtgttt tggttaatgg ggatgtgtgg ttgtagtttt tgttttgatt ttggtttgtt    2940
ttttgttttt gtgttgtgtt ttgtttttttg tttttgtatt ttgaggattt gtttatgagg    3000
ttgtttgggt ggtgggtagt ggggtgggat tttgtgttgg ggagaggtag tttgtgtagg    3060
gttgaggggt ggttgggagt tgggttttgg gattgttgtg ggggatggta gttgtgttat    3120
agtgttggtg tttttgtatt ggtgttgtgt ttttggaatg agggtaggat tgtgggtagt    3180
gttgggttag tgtggagttt ttttatgttg ttgtgttttt gtgtttttgg tttttgggtt    3240
aggtggggat aatttttttgg tttttagtgg ttgtgttttg ggtgtgggtg atgtgttttt    3300
tttggtggga tgtagttgat ttgattgttt gggagaagta gtgtaaggaa attgatttag    3360
ggttgtgttt atttttttttt tttttttttta gtagggtggt tgtttattgg tttgtgttag    3420
gtggtttggt tttggtgggg atgtggtaga ggaggttttt tgagttgttt gaggttgggg    3480
tgagtttaga attggagaag ttagtttggt tttttagtgt gattttggag tgataagagt    3540
tggttatgtg tttttaagtt attgtatttt aggtttgttt tttttgttgt tagggttgat    3600
ggaagttttg ggaaatttaa aggttatagg tgttggaaat ttgtagattt aagggtttat    3660
ataggtaatg gttagtgttt agggattttt tagtggtgtt tttgatgttg ttttttattt    3720
ttgggtttag tgtttttttg ttttgtgttg agttgttttt tatttgttat tggtgatggg    3780
gaagggatat ttggggagag gataggggat gtttttagtt tttgaaggtt gtggaaggat    3840
ttatgagttt tataggtgta atttgaagtt ggaaagtgag ggttaaagaa ttggtttata    3900
tttttttttt tagttttttg ttatatttta tattagaatg atttatagaa gtgatttgaa    3960
gagaagggta ggttttagtg ttataataaa aatgaatatg tgtttttttt gtgtatatag    4020
aaagggttgg gggagatatt ttttaggagt ttggagaagg tgaagtgggg agagggtaaa    4080
aattgatgaa gaattgggaa atattttttt tttttttttg agattgagtt ttattttgtt    4140
gtttaggttg gagtgttgtg gttttatttt gatttattgt aatttttgtt ttttgtgatt    4200
tttgtggttt agtttttgga gtaattggaa ttaaaggtgt ttattattat ttttagttaa    4260
tttttgtatt tttagtagag atgggagttt tattatgttg gttaggttgg ttttaaattt    4320
ttgattttaa gtgatttgtt taatttggtt ttttaaagta ttgggattat aggtgtgagt    4380
tattgtgttt ggttgggaag tagatgtatt ttgttatttt tttagatttt ggggtatgga    4440
gtatttatat aggttgatag ttaatgaatt taaagattgt aaattttttt ttagtgaatt    4500
attttatggg gttggtgggt tttaggaatg gggtgatttg gtattgggtt tttttagtta    4560
gttattttta gtggatatat ttatgtattt taatgagttt aaattggtag atagtttttt    4620
tttaattata ggtttatttt ttttatttgg gattagagtg tgggaaaagg gtattttggt    4680
ggtttttagg tgtttatagt agtttattat aatagttttt gagttttgga ttgtgtttga    4740
tagattgagg taatattttt aatttataga gaatatagtt ttttagattt tgtttttaaa    4800
gaggaaaggt aaggaggggt tatttaagat tatattaatt taagaagata tggatatatg    4860
ggaggaatag tatagattgg ggtgtgttag agggtgaggg gtgggaggag ggagagtatt    4920
aggaataata gttaatggat gttgggttta atatttaggt gatgggatga tttgtgtagt    4980
ggattattat ggtatatatt tatttatgta ataaatttgt atattttgta tatgtatttt    5040
tgagtttaaa tgttagaaat tttattaatt atgttatata gttttttttt tttggttaga    5100
```

ttgtatttta taagtgaagg atttttaggt taatataaat agttggtatt gagtttggga 5160
tttttttttt tttttagat aattttattt tgttgtgtag gttggagtgg agtggtatga 5220
ttttggtgta ttgtaatttt tatgttttgg gtttaagtta ttttttgtt ttagtttttt 5280
aagtagttag gattatagat gtgtattatt atgtttggtt aatttttgta tttttagtag 5340
agatggagtt ttgttatgtt ggttaggttg gtttggaatt tttggtttta tgtgatttat 5400
ttatttgggt tttttagagt gttgggatta tatgtataag ttattgtatt ttgttaagtt 5460
tggga 5465

<210> 273
<211> 22169
<212> DNA
<213> Artificial Sequence

<220>
<223> chemically treated genomic DNA (Homo sapiens)

<400> 273

ggatgtgtta gtttttttttt ttggatttga tgaggtatga atgtattttt aataaagtta 60
ggttttttttg ttgtggtttt ttgggtgggt gtttgtggtt tgggttatga gttatgttgg 120
gtaattttat tatggggaag agggtaggaa gttgggagtt attgtttttg tgtttggttg 180
ttattttggt atgagggtga ttgttggttt tgttttgttt ttatggttga gggtttttgg 240
gatgtggtgg gagatggggg agtttatttt ttaaatttgg tgtattttgt agggttgttg 300
tatttataaa aaggttgatt ttatatagga tttgtttttt tgggttttgg tttaggttgg 360
ggtgagattg gttttttgtg ttatttgtgg aagagtttgt ttagtaaatt gatagtgtta 420
atagaatgtt aagggggtat ttatttagtt ttaaataatt aatttaagtt ttttagagat 480
gtataatgga ttttgaggtt aggggattgt gtagataggg ttgatatttg atgataaatt 540
tttatttgtt aattagagta aggttggggt gtgttattaa ttttagtgtt aggtggagta 600
gaaggaataa tatgattatt ttttggggaa aaattttaat tagtaattaa ggagttggta 660
ggttttttat tgggtgagaa tagtgtttat ttggtgttta ttagattgaa tagttttgtg 720
taagtgaggt tgggtgagtg ttttgtggtt attggttttt attgtggtgg taggtgttgt 780
gggggttggg tttgggtagt atttgggttt ggtttgattt ggagatgttt ttggtagtgg 840
ttatttatag attgtgattg tgaggatttt gatggggttg ttgtgtgtgt ttgggaattg 900
taggtgtgga tgttagaggt aaagttttt ttgtgtatgt ttagtatatt agagtttttt 960
agtttttttt tatagagttt ttagtttttt tttatagagt ttttagtttt tttttataga 1020
gttttttagt ttttttttat agagtttttg agtttttttt tatagagttt ttagtttttt 1080
tttatagagt tttttagttt ttttttatag agtttttaag tttgttttta tagagttttt 1140
agtttttttt tatagagttt ttgagtttgt ttttataggt tttattggat attaggtttt 1200
agattttttt ttaggaaatt gtaggaatta tagtagggta gttttataga tttgagggaa 1260
ttgttgtttt atttttgttt tatagagttt tggtgtttgg gttgtagttg aggttgaggg 1320
gttgaggatt tggaggatgg gattttttgt ttagtgggtg tagttaggtg ggagggggta 1380
ggtttttagg gtttataagg ttttttgggg atttatttga agttattgtt ttgtattagg 1440
tagggtgtgt ggagagggtt ttgtttaatt gtgtgggttt aatttggagt gtagatggta 1500
ggatgttttt agttttgggt ttttgagttt gtgagtgggg ggtttagagg tgaggttttt 1560
atagaagttt ttttttagg ggtaatgggt gtagggtagg tggggagtat ttaggatttt 1620
ttttaggggt aatgggtgta gggtgggagt gggtggaggg gggagtattt aggatttttt 1680
ttggggtaat gggtgtatgg tgggtggagg ggggagtatt taggattttt tttaggggta 1740
atgggtgtag ggtgggtgga gaggggagta tttaggattt tttttggggt aatgggtgta 1800
gggtgggttg gggggagtat ttaggatttt ttttaggggt aatgggtgta gggtgggagt 1860
gggtggaggg gggagtattt aggatttttt ttggggtaat gagtgtaggg tgggtggaga 1920
ggggagtatt taggattttt tttaggggta atgggtgtag ggtgggtgga ggggggatta 1980
tttaggattt tttttagggg taatgggtgt agggtgggag tgggtggagg ggggagtatt 2040
taggattttt tttggggtaa tgagtgtagg gtgggtggag aggggagtat ttaggatttt 2100
ttttggggta atgggtgtat ggtgggtggg ggggagtatt taggattttt tttaggagta 2160
atgggtatag ggtagggggg tgtattgagg atattgttgg ggagtttttt tttttagaat 2220
tttataggtt ttggagatat attgaaagta attttaaaat gttaggattg tttataattg 2280
ttttgtatta tttttggggt aattaggtat tttgatgtta atatgtatgt attttttttga 2340
tagggagaag ttttttagtt gtttgtagat agggttttgt tgttaagggt tattggttgg 2400
tttggtgttt tgtaggggat atggtgtttt tttgttttaa gggtaatttg gagaggatat 2460
tatttgtgtt ttagagtgag gggttaggtt tttagttttt ttttgttata tgggtttgga 2520
tttaggttag ggtatagtta gttattttaa gttgattttg agggtttagg gggagaagta 2580

gtttggagtt aattatatag agttttgttt agggaaggga aggttttgga gtaggtagat 2640
tgggtgggtg ggggttgaga tgtttgtttg ttttatggat gtgtttaaag ttttattgtt 2700
ttttgttgtt ttagttattg ttgttttttt ggtttggagt tttttgttgt tgattttgtt 2760
ttagttagtg tggaagttgt tttgtggtgg gttttatttg aaagttatag taggtgtatt 2820
tagatggggt tttatagtat tgattttgta tataggaggt tatgaaggtt gtagtgatta 2880
tttttagttt gtggttaagt ttgtttattt tgtggtttgg tttttaaggg gtatgttttt 2940
tttttgttta ggggggtttg tatttgttgt gtttatttat attttagggt ttagtatgta 3000
gtagatgttt aataaagatt ttttatttg tatttttata gtattaggtg tgtggatgtt 3060
tagtgattgt ggttttattg tgtgtttttt gggggttaag ggatgttgaa tagtttggtg 3120
gttggtatga gtgaggttta gggataggtt gtgagtttgt tttggagttt tgagtaggat 3180
aggggttgtg ggtatgttag tttattttat aggtttattt gggaatatgt taggattttt 3240
tttaggggt tttgtggttg ttgggtggtt gtattggttt agagtgagtg ttattttttt 3300
taggaagtta ttttggatgt ttttttaata tttgtttttt ttggattgtt tgggagttgg 3360
gatgttgggg tttattttttg gttagttttt gatgtttttg gaggttaaaa tgtagttttt 3420
agaagaatgt agttttagtt tatgtgtgat atggatgaat tttaaatata ttatgtttag 3480
tgaaagaagt taggggtaaa agattattta tggtataatt ttatttatag gtaatgttta 3540
gaagaggtga atttagaggt agaaagtggg ttagtggttg ttaggggttg gggtagttag 3600
ggtagagtga tagtatttgg gtatagtttt tgttttgtgt agtgagaaag ttttggaatt 3660
agatagtggt tgaatgaatt tgtgaatgga ttagatgtta agtaatagta tgttttagtt 3720
taaaagttat atttttagtt tggataatat agtaagattt tgtttttgta aaaaaattta 3780
aaattagtta ggtatggtgg tgtatatttg tggttttagt tattttggag gtggaggtag 3840
gaggattgtt tgagtttagg agtttgaggt tgtagttagt tgtgatttta ttattttatt 3900
ttagtttgtg tgatagggtg agattttatt ttaaaaaata aaaaagtggt tatgtatagt 3960
ggtttatgtt tgtaatttta gtattttgag aggttaaggt aggtggatta tttgaggtta 4020
ggagtttgag attagtttga ttaatatggt gaaattttgt ttttattaag aatataaaaa 4080
ttagttggga gtggtggtat gtgtttgtag ttttagttat ttaggagtat tgtgtgaatt 4140

tgggaggtag aggttatagt gagttaagat tgtgttattt taatttagtt tgggtgataa 4200
agtaagattt tattttaaaa aataaataaa aaaaggtaaa ttttgtaata tgaataagtt 4260
attatttaga atttttttat atatatattt atttttaaat tagtttttag ttttttttgt 4320
ttttagggag tagggtgggg tggggtaggg ttataaggag aatttaatta ggtaattttg 4380
gaaaagggtt ttttttttatt tttttatttt tgtagttatt gttttttttt tagttggggt 4440
gattattgag tttagtgtag gttttgttgt agggtttata gttattatta tgtttttagt 4500
ttgttttagg gttttgttgg ttgtagttgg aaggggaatt atatatagat ggggaaattg 4560
aggtttaggg aggggatagt gtggggagta tagtaaatta gagttagttt tgttttatag 4620
ttttttggag ttttgggttt tataggagaa agtgttagtg ttgtttggga aatttgaggt 4680
tgttaaatgt agtttggttt ataggtgtga tgttttaaag ttagttgttt gttaaagttg 4740
ttggtatttt ttttttagtt tggggaaaga tttggaaagt tttggatatg tttgtatatg 4800
aaggtgaaag ttgatgttag tttgttttag atttttatgt ttttgtagtt atagtttttt 4860
tatttgtgtg ttggggatga aaattgtatt ggtatatttg gagggtgggt ggttgtttgt 4920
tagggttttt atgaggttgg gtaatagtgg tgttttgagt gtggagttgt tattgttgta 4980
ttattgagga aggtaagtta gatgtttaat tgattttgta aagttatatt tttttattat 5040
ttatagatga gatatgaaat aggaaaggtt ttttttttat agtaaaaggg tgtagttttt 5100
tggtttggtt tggaattgtt tagtgggtat tggtggttgt tgttgtttat tataggtgta 5160
gggaatgagg aatgaagtta gttttggttt ttgtggggtt tttagtttgt tgtttgtatt 5220
tgggggtttt tgtatttagt tttgttttgt ggaagtattt tttttggtgt attgtttgtt 5280
ttgttttatt ttttttttgt tagggttgtt gttttttttt ttttttttt ttttttttatt 5340
attatttttt ttaatggaaa atatgttggt tgttgtttag aggtagggtt tgttttaagg 5400
agttagtttt ttttagagta ttttaggttt ttattttagt ttttgtttta ggtttttttg 5460
tttttgatgt ttttttagtt tgagtaggtg agattttta tttgatattg tgtggggttt 5520
tttttgttgt tttttgtttt tttgagatag agttttattt tgttatttag gttggagtgt 5580
agtggtatga ttttagttta ttgtagtttt tgttttttgg gtttaaagga ttttttttgtt 5640
ttagtttttt aagtagttgg gattataggt atatgttatt gtgtttgggt aatttttgta 5700
tttttagtag agatgggggt tttgttatgt tggttaggtt ggttttgaat ttttgatttt 5760
aggtgatttg tttattttgg atgttgtatg tttttaaata tgtttttttta attttggtgt 5820
ttttgaagat taggttttgt ataaatagga ggttttgtta taggtttttt tgtttagttt 5880
agtatgagga ggagaattaa tttgtttttt ttttggttat gtttaagatg aggtaggtga 5940
tttaatggaa agagttagat tgggtgatag gagttatttt gttttttttt agttttttag 6000
gttggttgta gggttaagtt atttttgtt tgagtttttt tttttttttt ttaaggggaa 6060
gttatgtttt tagtttgttt gtagttggga aatgatgggg tagtttaggt tggttggggt 6120
tgtgtttagg gatatttgtt tgggagaagg agaggggtag ggttagtagg aattttgtta 6180

ttgttgtgtg attttggggt tgatggtgat ttttttgggt tttttttttt tatttattgg 6240
tgtttgattt agatagtttt atggggttgt tgggagttat gttaggttat tattgggttg 6300
gtatattttt aatatgtttt atatgtgagt taggtgtttt ttttatgttt tgttgggaag 6360
tgaattttat aggagtaggg gttatgtttg ttttgttttt attgggggtt tggtttggag 6420
tagaatgtag tatatagtag gtgtttaaat gtgtttgtga attggagtta ttttgggaga 6480
tggatttttt tggagttgga gttattttgg gagatggatt tttttagagt tggtaagttt 6540
tttttttaggt agtgttaggt ttagtttatt aggtagtatt tttaggattt tttgaattta 6600
tttgttaatt aattaaatat ttaggaagga ggttttggaa attagtattg ttagttaatg 6660
gggagaaggt ggggaagaaa ggagtgttga ttaggtgttt tttttatatg gtttttatgt 6720
aatttttata tattattaag agatttagtg tgtattagtt tggttgatat gttggggaaa 6780
ttgaggtttg tatgatttga tgatttaagg ttatagagtt agtggttagg ttaggtttta 6840
gatatgaggt tgtttaaagt taaagtgtat tatttagtag gtagttggat attttttttgg 6900
aggtgttggg tgtgatagtt tattttttatt ttttttagtt tttggaagat gttttgtgtt 6960
attttgattt tgttgttagt agttaatatt ttagttgttt tttgttttaa tgtttagttt 7020
agatgtaggt agttttggga gagtgtttta gattaaggat gtagttttat ttagatagat 7080
agggtttttg tagttttttt tagttttttt taaaataaag agaaaaaagg tggagttttt 7140
tttagtattt tttgtttata ttgatggagt tgatgagggg gttagagaag agttaagggt 7200
tatagtgatt ttttgggttt gtttagaagg ttattatttt tgattatagt tattatggtt 7260
atattttgta tagtagttgt ttagagatta attatttttt gtttattagt atgtgttgtt 7320
aggtgttttt tatttggtta ttggttgatt aggttatggt gatattttga tttttttggga 7380
aggtattttg tattaataaa attatttttt ttaattttgg gttttgtaat tatttgattg 7440
gtagtttttg gtgaaggttt tttttttttg ggatggaaag aaatgattta tttagtatta 7500
gtgagttagg tattgtgatg tttattatga ttatgttttt tttatagtgg aggtagtaaa 7560
tggtattaaa agttaaagtt ttttttgggg ttatagttgg atattatggg gttaggattt 7620
ttatttaggt tggtagggtt ttaagagttt gtattttaga tttttttgag aaatagtgtg 7680
ggtttttttt tttgttgggg gagttaggtt ttttgtaggt gttttggtta atttttggat 7740
agattttttg ggttttttga gaaggatggg taggaatggg aaggtagagg tatttgaggg 7800
gtgtatgttg ggtggggttt aagtttaggg agggagtttg gggttttttt tgaagtggtt 7860
gtaatgtttt ttagattgtt gtgtgagttt tgatttgtgt ttttataagt tgggtttggt 7920
tgtggttttg gtttttaatt ttgtggttta gaagtaggag tttgaggatg tgtttttgtt 7980
ttattttttt gttggatttg tggggtgtgt ataatggggt ttttgtgggt tgagtttggt 8040
tgtttgttag gttaaattga agtaggtttt ttgttaagaa ggagggggtg ttaggtttta 8100
gtaaagggga taaggttagg tattaaagtt atggttggtg gtttgttttg ggttggaaat 8160
agtttattgg gattttaggt ttggggaatg ggaaagtggg tttatgatat tagtgttttg 8220
tggttttgtg gtagggtttt aatttagata ttagggagtt gtagtaattt gtattttaaa 8280
gtatagtgtt ttggtttagg gagattttag ttttagattt ttgtgggtta ggttgtgttt 8340
atttttttggt gttattgaga agaagtaata ggtttttaat ttttttagta tggtgttttt 8400
tttatttttt taaagttttt tgtaggagta agaagttttt tttagagttt tgaatgggtg 8460
agaggtaaag tttgagttgg gagtgtagtg gtggggaagg gggttatata ttttgggaag 8520
ttatgtataa ttaattatat ggtattaatt tgtttttttt aataatagtt gttgtatttt 8580
ttttggattt tagttgttgg tttttgaatg aaaggaaata agatttaggg tattaagtgt 8640
ttgtgtggtt tttgtagagg gagaaggggg ttttaaaaag taaaaaaaaa aaaaaaaaaa 8700
aaagtagtgt gtatttatta gtgtttgata aagatataat tggttttgtt tattaaattg 8760
tttatagatt tgtttaattg gttttagttt ggggagggtg ggggttgggg agggaggggg 8820
tttgttgttt ataggttggg tagttggtat aggtaggagt tgggttgggg ttgttaaggg 8880
aatgggattt tttgtagttg gaatgagggt gtgtagattg gttggggagt tatttttttta 8940
tttgtagttt ttgggggggtg gagggaattt ttttttttaga tattaaatat ttttttatta 9000
tttttattag ggatggattt aagttgaagg gtattggtta tagtagaata gtttttttatg 9060
gttgatgggt atttataggt aggtaggagg gagtggtttt gtatgatttt ttggtttttt 9120
tagtgtttat tttatatttg aggttggtag attagaaggg ttatgggatt ttgaggttag 9180
agtttgtaga tgttaggata gaaattagat ttaataatta ttttatattt tatgttaaaa 9240
tatgagtttt attttttttt ttttagtttt agtttgaggt tgggaagagg gagttggggt 9300
aaatagagag tttaaaggtg ggtttgattt tgaagagtta atttttttttg aagaggtatt 9360
atttatttgg aggtggtatt atttgtttag gaagtattat ttgtgtttag aggaggtatt 9420
atttgtttag aggaggtatt attatttaga ggaggtatta tttatttaga ggaggtatta 9480
ttatttagag gaggtattat ttatttagga ggtggtatta tttgtgttta gaggaggtat 9540
tatgtattta gaggaggtat tatttgttta ggaggtggta ttatttgtgt ttagaggagg 9600
tattgttgtt agtttaggag gtattatgta tttagaggag gtattatttg tttaggaggt 9660
ggtattattt gtgttagagg aggtattatt attagtttaa gaggtattat tatttattta 9720
ggaggtatta tttttgagag gaggtattat ttgtgtttag gaggtattat ttgtttagag 9780
gaggtgtttt aagttggtag ggtggtgggg gttgggtttt ttagtaaatg gtttagagtt 9840

```
tttttggta gattaattgt tttttgggga tttaaagaag aattggggta ttgggatttt     9900
tatttagtga ttttggagtt tgaggtggtt tatggaggag tgtttaagtt tttagtagtt     9960
agattagttt tatatgtagt ataattgttg gtgattaagt aattgttaga ttttgtttaa    10020
taaaagttag tatgtgattg tgtttagatt ttgggttatt tttagaagat aatggtttag    10080
tgttttgttt tttgtgtggt tgtagaaggt aggggttttg tttttgataa agtaataggt    10140
tttgtagggg tggtatttat atgtaggttt ttgtgttatt ggttgtttta tattttttgt    10200
tatttaggta ggttttattg ggttgggagt attgtgggtt taggtagggg taggagaaga    10260
gaggttagtt ttatttgtat tattattatt gaaggttttg gttattttgg gggtggggat    10320
ttgtatttag ttaaggggta ttgtttgttt tttattttgg atgtattttg tttagtatta    10380
tgggttgttg gggtatgggt ttgtaggaag ttttttttatg aaattaaagg ggtgttaata    10440
ttttgtttag agttttgtag ttagaaatgg ggggaagggg agtagtaggg aatatgtttt    10500
gtttgaagta gggtggggtg gtttgatgtg ttgtttggat gtagtggggg aaaagtagag    10560
ataaagtggg ttattttttta aagtgttgtt ttttattttg gaggtttatt gaggagtttg    10620
gtagggtagg gtttttttatt tagttgttga gattgttttt gtttaaattt gttttgtaag    10680
ggggtagagt tggatggggg gagggggggt ggttatattt agtttttgtt tgtgggaaaa    10740
tgtgatagtt gtttgtttgt tattttgttt gttaataaag tgttggtttt tgttttagaa    10800
ttagagtggt ttattgtgga gttggggttg tttttagggg tattgggata gtattttggg    10860
gatgtggttt tgtttttgtt gatatttaat atttgttttt gtgggtgtgt atttttaggg    10920
tagtttttat atatttatag gagggggatg aaggtttttt atttggaagg aatttgattt    10980
tattttgaga agggattaat tatttaagtt tttttttttt tattttattg ggttgagaa    11040
atgggggagg ggggtgtgat atagagttgt attttttgga agaggataga taatttttgt    11100
ttttaatttt tggagaaaga gtggattaat tatttgattt tttagagatt ttgtttgttg    11160
tgtggtgggg agaaatgtaa gagtttttta tttgtgtttt gtttttttgtt tttaagtttt    11220
ttaaatttta attttgtaaa gtaaaaggaa agtttagttt ttttgggtgt ggtggttttt    11280
tatgtaattt ttttttttaaa ttgagagttg ggttgggggg tattggtggg tggggtgatt    11340
gggagtttgg ggatttagtt tggttgtgtg ggttagtgaa ggtgaatgtg gtgttgtaga    11400
gtttatattt tgtgttttaa tatttgtttt ggtgtgggtt agaggtgaag aagaaagaag    11460
ataaatggtt tggagaagta ataggaaatt aaaattgatt ttatttaaat tgaaaaatag    11520
taggtagttt gtttgtttgg ttgattggtg gtaagtttta tgtgtggtgg gtgaagggtg    11580
ggtttggagt agggtttttg gggttttagt attttatatt ggtttttttaa tttggtttta    11640
ggtatgggtg gtgtgagttt gtttttatgg ggggattgag ggggaaggtt ggtttttttt    11700
gattttggga ttttagaatt ttatgttttg ggttgtttgt tttttttttt ttatgttggt    11760
tttttgttg tttgtttttta gttgtggggt gtgtgtgttg ggtgttgtta aagtttttaa    11820
agggtgtgga aagtgggggt ttgtgggtgg gtgggtgttt attttgtgtg gtgagtgtgg    11880
gtagtagtgt taggtagagt aggggtgtta ggagtggtgg tatgtttttt tattggttgt    11940
tttttgtgtt tgggtggtgg tttttttgtgt tggttggtgg ggttgggatg tatatgtgtg    12000
gtgtatggtt ttggggtggt ggtggatggt tttgtttttt ttttttttggt tggttggtgg    12060
tgttgtgttt ttgtaggttt ttgggtttgg ttttgtgttt ggtttgtttt tttgttgtgt    12120
ttgtgtttgt gtttgtgttt tgtgttttgt gtttttgtgt ttttttttgt ggttgaggta    12180
ttagtgaggt ttagagttga ggtgtgtttt gtttttgttg gttttgtttt gtttggtttt    12240
atttttgttg gtttttttgt ttttttttgtg gttatttttt tttgtatgtt ttgtttgttt    12300
ttgtgttttt ttgagttgag ttgtgtgttt tgagtttagt ttttttgggg aggagggatt    12360
tggtggtgtg gtgtgggttg ggagtgggtg tttgggatta tttttttgttt gaaagttttt    12420
agaggttaaa agtttgagtt ggggttgtgg aaggatttgg ttttggtggt tgttgtgttt    12480
ggtgataatt ggtgattgaa atttgtatgt gaataagtgg aggagtttgg ggtggaatgg    12540
ggagggtgag gttgtggata gttgtgtgta ttgtgtgttg ggttgggtgg ggagtagttg    12600
aggttatgtt ggggggatttt gggtttgggg tagggttttg ggagagtggt ttagttgtgt    12660
gtgtgttttt agtttagtgg ttttattttt ttttgagggt tttgtggttg tggttttagg    12720
tgtttagagg attgattgtt gttagatatt tttggttgtg attgtttgag tatttgattg    12780
tgagggatgg gggtgttgtg gtttttgggt aggggatttt gggtgttttg agggagtagt    12840
tgggattagt gttttgtgga tggattgtgt tttgtttttt taagggggat ttgtgttgat    12900
atgtttttg gttattgttt ttttttttgtt tgggtgttgt ttgtttaagg tggttttaga    12960
aagtataaat gggttgggtg gatttgttgt tggtgttggt gtagtttttt ttatgtgttt    13020
tttttggttg atttattttt ttattatgat gttaggtatg ggtattttgt gttaagtttg    13080
gttaagtgtt aggagtttaa aatgtttgtt atagttttttg tgtaaagttt atggttttgt    13140
gttagggata gatagtgttt ttttttttatt ttaatatagg gtggtgagtg tgatggtaga    13200
aggtgtgtgt tttggtgttt tttttggagt aggagggtta agtttgtttt gtggggagat    13260
tgaaaggttg ggagggattt aggattgtgg gggtttgggg ggagggttgt agaaatttta    13320
gagtatggtg gtagaggtag gtatagaaag taggataaat tttatatttg attttagtaa    13380
gtttttttttg gtatattttt tgttaaatgt tgagtatttt ggaaatagtt agtgtaggtg    13440
gtttgttttg tttttgtagt tgttgttggg agttttttttt gtttgtgttt ttttagaatt    13500
```

```
tggttttatt tttgtaggtt tggggtaata ttttggggtt agtgttttag gagaggaggt   13560
gtttggaggt agtagagatg tttatgtaat agtagttagg tagtttggag gagtagattt   13620
tttatagaag gaagtgtggg gttgtgtagt ttttaggttg aggattttgt ttattatgat   13680
gttttttgtt tttggtgggt gggggtgggg tggggtatag ggaggtttga aggttttagg   13740
tttggtgttg attgggtgtg ggtgtgattg tttgtgtgtt tttattggta tttttgtttg   13800
tgggttttag gttagaagta atggtttatg tagtttaggt ttgggggtta gtttttagta   13860
ttttgtggag gggtggtttg ggaggggagg aggggatttt tggatagggg gtttttttttg   13920
gtttgtttta gttgtgttga tagtattttt attttgggtt tttattttttt attagtagag   13980
aagagagtgg tttatttggt gtttatttgt taggttattt agtagttagg tatgtttata   14040
agtttgtgtt atggatggtg tggttgggtg tataggtttg agggaagtat aggttttggt   14100
tttttagttt ttaatttttg tattatttat agttgggagt attttatgga tagttgtggt   14160
tgaggagaag ttagggatga gggggatggg ttttattttt ttattagttt gggaaggggg   14220
taggtttttg gtaagtaggt gagaagtttt tttatttata agttgtttgg gttttttatt   14280
tttttttgtt aggttagtgg gtgtgggtag ttgggggagg ttggggtttt gatataatag   14340
gagtatattt gttggtttag ggtttggaga ttttttttta taggttggga ggtagttttg   14400
ggtagtgggt attgtgggga ttaaagagaa ataagagtag gttgtgtgta gggtatttta   14460
gttattttag aaatttaatg ggagggtggg ggttaggatt ttaagaagtt gaattggggt   14520
gtagtgttgt taggtttttt ttgtaggagt tattttgggt tttttgtttt aatttgtggt   14580
gggagttggg gtaaggtttt aggggtattt tgaatgaatg gggttgtttt taagttggtt   14640
agggagtttg gaagtagatg agatggggtt gtagagtttg gtttgtggag gggagaggta   14700
gatgtgtttt gtagtttttt tttttttaga ggttttttgt gggtgttgag gggagagtgg   14760
tttaggtgtt gtattttagg agattgaatt gggttagttt tttttttgttt gttgttagag   14820
aaatgggggt attattgtta gggtgagtta aggaggtttt gttgatattg gtttaattag   14880
gttttttttt tgtggttgtt gtggggattt ttgagagtag agtatatttt taagtgtgtg   14940
gagaggtaag tatagttggt gagtatatgg ggatagtgat ggagtttatg gagatttagg   15000
tgattttaaa atattttagg gttggttttg gttttagaga gatgtggttt gagtagaggg   15060
gggttagtag tgatttggtg ggtgggtgta tttgaggggt gtttgtgttg aaagttttgt   15120
agtagatttt aaggagtagt tagggttgag atttttggtg ttgagtttaa ggtttttttt   15180
tttttgtag gagtttattt tggtttttgt agtttttatg atttgtggt gttgggggggt   15240
ggttagtatg atgtttttta tttttttagga gggaagttga ggtttagaga tattatgagg   15300
atgttagttt aaggtttagt taggttttgg tttttagttg ttgttattta gtagggtaag   15360
tgggattttt aggaggtgtt gggggatatt tttttttttt tttatattta tagtttgttt   15420
attgtttaag gtagtaggtt ggatttggtg tagggattat ggtttggtgt ggttaggttg   15480
gagtggtttt tgggattgaa agtagggggt gtgggggttt tgaggtgggg ttgagtgtgg   15540
ggatgttttg tggtgggtat tttgttgggt tttgggtggg ggttattatt aggtagttgt   15600
gggaatattt tagattattt aggagtaggg tttttttatt gtttgaagtt gattgtaaaa   15660
aatatttgat ttgtgtgtaa agtttaagtt tagaggtttt tagggtgttt agtttttagg   15720
gtgtttttta tgtagttttt atgtttttat ttattgtagt ttttgattat tgggtatatt   15780
gtggggtttt tgttggttgt tgggttttag gttttgttgt attaagtgat ttttaaagtt   15840
ttatttgttt tttttattt ataaagaatt tgggatattt ttttgaggtt gggtattttt   15900
ttagatgtgg ttttagtgtt ttgtttgtta tttggttttt tgtgttgggt ggtttgttgt   15960
gatgtatttg gtagtttttg ttttaggatg gatttgggtt ggtttttgtt ttgagaatag   16020
agaatggttt ttgttttgag gaggttgggg tttggaattt gttttttggt tttttgttgt   16080
gtgtagatag ttattgttat ttaggtttag gggtgtttgt aggtgttggg tattttattt   16140
tggtttttat aggggttttt tatgttttgt attttttggt ttgtgttggg gatttttttg   16200
ttttgttgtt gtaggtttg ttttttgttg agggggtttt tttttttatgt gtttttgaga   16260
tttttttagtt attttagagg tttttggttg tttgagtatt tttgattatg aagttttttt   16320
ggtttatttt ttgtgtgtga ggtagagttt atttttatta ttttgattta tattagaggt   16380
tttggaaggt ttgttgattg atttgtgatt gtaagaagtt gaaggaatgt gggtgtagat   16440
ttggtagggg ttttttttgg tgtaaagtta attggttgtt tatgtaagtt tgttgttgtg   16500
gttagttttt ttgttaggtg tttggtattt tttttggatt tagttgttag gatagtgatt   16560
tttagttgtg gttaggattt tgtttggggt tttgtttatt tagtgttttt agtggttttt   16620
atgtagaaat tgtttttttt gttgtttatg aggtatggga tagggttagt atttgagagg   16680
taggttttgt tgtttttttt tgtatagagg ttgggggagt gagttgtttg ggtaagtttt   16740
tgggtaggta ttgggttttt tgagtaggtg agttgagaag gttttagttg gtgtaggttt   16800
tgagagttgt aggattggtt atggggtaga tttggtattg ttattttaga gttgggtttg   16860
gggggtggtt ggtgtagtgt agattttagg tggaggttat ttgtttgttt tttttatgtg   16920
gtttagggtt tagatttatt tgtttgtttt tggaagtttt tagattgttt agagtataga   16980
ttttagtggg aaagatagta gataggtttg ggtatggttt tttagttttg agaagtgggg   17040
agtttattta gattttttta ggattttagg ttttttttagt agtttataga gtgtgggtgg   17100
agtttgggtt ggggttgggt tatttgggag ttaaaggttt atttttgttg tttttaggga   17160
```

gttttaggga gatggtagag gtagtgtttg gttggggttt tggttggtgt gttgtttgag 17220
gggtgtatgt attgggaagt tggttaggtt tgtttaaatt agggggtttt taggaggaga 17280
gatattagga ggatgggtat agaggtggtt tagtattagg gttggtattt ttaggtaggg 17340
gttgtggggg gttggattag atagggaggt tgttatgatg tttggttgtt ggatgggggt 17400
atagaggttt tgagtggggt ggtagagttt ggttattagg tttatttgta tttttatgt 17460
ttttttatta tttttagatt tttatgattt taaattattt agttttaggg atttttgtag 17520
agtttgtggt gggggtgatt ttttatttg tttggttttt gtttatttta ttttagaatt 17580
ttttttaaaa agtaagagtt ttgggggatg aaattttgag gttttatttt ttgggttttg 17640
gtataaattt tgagtttttt ttgagggttg agttattggg aaagttggga tgggttggtt 17700
ttttttttt ttttttttt ttttttttt ttttttttt tggttttttt ttttttttt 17760
ttgttttatt ttttttttt gttttttttt ttttttttt tttttgttt tttttatttt 17820
ttttttttt tttgttttg ttttttttt tttttgaggg taaaaatagt tggaaaattt 17880
ttttaaaaaa gtttatttta attttagagg gataatgttg gtgttttaaa gtatagtttt 17940
ttgttttgga ggagttattg gtagagttag gagtttttga aggattttga ttatattttt 18000
tattttattt tgatttgtgt ttggtgtttt aagtggagtt tgggttattt ttgattttat 18060
tgttggggtg atggagaagt agttgtttga ggtgagagtt ttttaggtgg ttggggtttt 18120
tttttatttg ttttagatat atttaaataa gttgagaatt tttgtttttt tatattttt 18180
tttgtggatt tttatggatt tttttgtaa atgaatatgg gtttggaaat tggggtgtgg 18240
gttatagaga gtatggggaa ttttgtttgt gaagtaatat ggagtatgaa tataagtagg 18300
tattgtatat ttgtgagtta tttgggtttt tgggggaagg taaaaagttt taataagttt 18360
taagtaagat taggatgttt ttatagtttt gttttgatag ttaaggaaaa aatttgttaa 18420
aaattagagg tgttgttgag tgttggtgtg ggaatgttag tttagttaga gttttgtttt 18480
gggttattta aataaaaata gatatgttgg tggtaaatgt tagttgaata tgtaggagag 18540
tggtgttttt tgggtttgga gaagggtttg tagttgtatt ttttttttt ttttttttg 18600
gtttttttag tttttttatt attatgatta gtattttatt ggtaaatatt tggtgtaata 18660
atttttaaaa attagtaaga aaagtttgag tgttttatgt ggttgtgatt gtgtggtttt 18720
agttggaagt atggattttt agtgtttttt agagtttgtt tttgagggat attttaatta 18780
tttgagggtt tttgtatgat ataaattaga ttgattattt ttagagtgga gagggggata 18840
aatttatttg tagtaagtga aggttagagg ttgttttggg atggtattgt ttgtttttag 18900
gggtttgtgg tggtgtagta gatatgggga tttttgggtt ttttttttt tttgttgggg 18960
atgttatata gatttatgtt tagggatttg aatttagagt ttgttttagg ggtggtgaat 19020
gtatttggga tattgaggat tttttattg ggtgtatttt atggtttttt ggttttttt 19080
tttgagatgg agttttattt tgttgtttag gttggagtgt tatggggtga tttttgttta 19140
ttattatttt tgttttttgg gtttaagtaa ttttttgtt ttagtttttg agtagttggg 19200
attataggta tgtgttatta tgttagttaa tttttttatt tttaatagag atagggtttt 19260
attatgttgg ttaggttggt tttgaatttt taattttggg tgatttgttt gttttggttt 19320
tttgaagtgt tgggattata ggtgtgagtt attgtatttg gttagttttt tggttttgaa 19380
gggtttttgg gagggtttta gagaatgttt tggttttttt atttttgggt ttttattgat 19440
tgaggttagg gttgggaagg ggtgatgagt ttaggatttt tttggtgaga gtgagggttt 19500

atgttggggg gttttttttt gttatttttt tttggtgata tggggttggt tggagagttt 19560
ttttgtgttt gggagttttt tagtgtaggg ggggaagggg gttattgtaa tgaatttgat 19620
attagttttt tttttttttg ttttgttggt agtttttatt tatatggggg gtgggtttgt 19680
ttttttttt ttaaagttgg gggttttgag tgtggttggg gtaatgtttg gttttttta 19740
gaatagtggg gtttggaatt tatttaaggg aagaagagtg aaggaatgta gatgttgatt 19800
gtatggagtt tttttgggtt gtagttttta gagtttgggt gttttgtgag ttagaaagag 19860
aatggttggg gagagggttg tgtgaggttt tgtttttta agattgttgt ggaggattga 19920
tggggtttag ggattttgat tgaggtgtgt tgagtatagt agttttggat aggttgtatt 19980
ggtttttttg tttgttattt gtttatttgt tggtgggtag gaatagattt gatgttgtaa 20040
gaagaggttt ttaggatttt gttttttta ttggttgagt ttggattgtg gagttagttg 20100
tttttatatt gggtatagat gattgattta ggttttgaga agatttaaat agtagtagga 20160
tgtgtatttg gtaggggtag gagggttttt tttggattta aaagtgtatt tgtggagttt 20220
tttttttgta gtgtaggttt tggggggata gtggttagga tggaggtttt tgtggaaagg 20280
gggagatgtg aatagtgagt tttttgtatg taggtatttg ggaaagaata ttttagttaa 20340
gtggttttga gtatattttg attttggtta ttttggtttt gtggattaat tttggggttt 20400
aggattaatg atagttagtg tttgttggtg tttttagttt gggttaggtt tgtgggttgg 20460
tttttttaat gatggggtaa gtttagtttt gtttatattt ttttttttt tttttgtgtt 20520
tttttttatt tttaggagtg ggttttaggt ttgttatttg ttatttatgg ggggttgtgg 20580
gttgatttgt attttttaa aagatttgtt gtatattttt agagtttgtg agtaggattt 20640
gttttggaga tgggtttttg tagatgttgt tgagttaaga taaggttatg ttggagtggg 20700
gtttgtttta atttaatgat tggggttttt ggtaggagag ggaggtttgg ggagaaggtg 20760

```
ttgggaagat aggtagaggt tggagatagt gtagttttaa gttgggagta taggggttgt   20820
tggtatttaa tagaagttgg aggaggtggg aaggattttt tttagagttt tgggaggggg   20880
tttggttttg tgatatttag attttggatt tttggttttt ggaattgaga gagtatatat   20940
ttttgtggtt tgaagttatt aaatttttgg taatttgttt tggttaggaa attaatatta   21000
gggataaagg tgttttaatg tagataaaga tagtttttga attaattaag ttttgtgtaa   21060
ggttgttggg attgattggg ggaagtgagg gatttagtag ttttttttgat gtttgttagg   21120
tattggtttt tatatatgtt tgttggtggt ggttatggta ggttgttttt taggtgaaat   21180
tatataatga gtagttttaa gtttgttgat ttatatagta gaggttgttt ggtttggtgt   21240
tttttttggt tggaggattt tgttttttttt ttttgtttgt ttgtggttat tttggttgta   21300
gaggagattg gtagatgatg tttttatttt tggttggggt aaaagttttg tttaaatatg   21360
ataatggtga aatttggaat agtgatagag ttaatttatg attgaggtag ggttggaggt   21420
tttttggaaa atgtatggag aaatatgagg gtttagttgt gttttgggtt gttttatgag   21480
tttagttttt tttgttttag tttttttttt aaagaaagga taaaattatt gaaagggttt   21540
gtttgggtta taaatgtttt aaattggttt ttgtgtggaa attaaataag ggtaggtttt   21600
agtttttggg atttaggtga aatttgaggt ttgtttgagg ttatagtgtt tttttgagga   21660
tgttagtttt gttggttgtg tttggggtta ggttgaggta tagtgggtta ggaggttgag   21720
tttttttaaag gagttgtatt gttttttttt tagaatatga aattaatgtt tgttattttg   21780
aatttttatg gataagattt tttggaagtt agtttgtgtt agggttagtg tttttttaggg   21840
ttgatattta tatttttgta tttttagtgt gatttgggat agtggtagaa tttgttgggt   21900
tttttttatg ggagtgtttt tttttttttt tttttttttt tttttttttt ttttttttga   21960
gttggagttt tatttttgtt gtttaggttg gagtataatg gtgtgatttt ggtttattgt   22020
aatttttatt ttttgggttt aagtagtttt tttgttttag ttttttgagt agttgggatt   22080
ataggtgtat attattatgt ttggttaatt tttgtatttt tagtagagat gaggttttat   22140
ggtgttggtt aagttggttt tgaatttttt                                     22169
```

<210> 274
<211> 22169
<212> DNA
<213> Artificial Sequence

<220>
<223> chemically treated genomic DNA (Homo sapiens)

<400> 274

```
aggagtttga gattagtttg gttaatattg tgaaatttta tttttattaa aaatataaaa      60
attagttagg tatggtggtg tgtatttgta attttagtta tttgggaggt tgagatagga     120
gaattgtttg aatttaggag gtggaggttg tagtgagttg agattatatt attgtatttt     180
agtttgggta ataagagtga aattttgatt taaaaaaaaa aaaaaaaaaa aaaaagaaaa     240
gaaaaaaaaa aagtattttt gtagagagaa tttagtgggt tttgttattg ttttgagtta     300
tgttgagaat gtgagggtgt aggtgttagt tttgggggat gttggttttg atataggttg     360
gtttttaagg agttttgttt atgaggattt aaggtggtag atattgattt tatattttaa     420
ggaaaagata gtgtagtttt tttgggaagt ttggtttttt ggtttgttat gttttagttt     480
ggttttagat atagttaata gggttgatgt ttttgggaag atgttgtgat tttagatagg     540
ttttgggttt tatttgaatt ttaggggttg ggatttattt ttgtttagtt tttatgtgga     600
agttaatttg ggatgtttgt gatttagatg aattttttta gtggttttgt tttttttttg     660
gagaagggat taggatgaag aaggttggat ttatggggtg gtttagggta tagttgggtt     720
tttgtgtttt tttgtgtgtt ttttagaaag tttttagttt tgttttaatt atgggttggt     780
tttgttgtta ttttgagttt tattgttgtt atatttgagt agaatttttg ttttgattag     840
ggatgagaat gttgtttgtt agtttttttt gtagttggag tggttatagg taggtagagg     900
gggaaggtag agtttttttag ttaggaagag tgttaggtta ggtagttttt gttgtgtaaa     960
ttgataggtt taaggttgtt tattatgtgg ttttatttga agagtgattt gttgtagtta    1020
ttgttagtaa gtgtgtatgg aggttggtgt ttggtgagtg ttggggaagt tgttgagttt    1080
tttatttttt ttagttaatt ttggtagttt tgtgtaaagt ttggttaatt taaaaattgt    1140
ttttgtttgt attaaaatat ttttattttt ggtattaatt ttttggttag aataaattat    1200
taaaaatttg gtggttttaa attgtagaaa tgtgtgtttt tttagttttg aaggttagaa    1260
atttaaaatt tgggtgttat aggattgggt tttttttttaa ggttttaggg aggatttttt    1320
ttattttttt tagtttttgt tgggtgttgg taatttttgt gtttttagtt tggggttgta    1380
ttgtttttag tttttgtttg ttttttttagt gttttttttt taaatttttt ttttttgtta    1440
agggttttag ttgttggatt agggtaggtt ttattttagt atggttttgt tttaatttga    1500
tgatatttgt aaagatttat ttttaaaata ggttttgttt ataagttttg ggggtatata    1560
```

gtgagttttt tgggggaata tgagttaatt tatagttttt tatgagtgat gagtgataga 1620
tttggagttt atttttgggg gtggagggaa gtatagagga ggaggagaag ggtgtggatg 1680
gggttgagtt tgttttatta ttgaagggat tagtttatag gtttggttta ggttggaggt 1740
attagtgagt attggttgtt attaatttg ggttttaggg ttggtttata gggttagggt 1800
ggttagggtt ggggtgtgtt tagagttatt tggttggaat gtttttttt agatgtttgt 1860
atgtaggagg tttattgttt atattttttt ttttttatga gggtttttgt tttggttatt 1920
gtttttttag agtttatgtt gtagggaagg ggttttgtga atatattttt gagtttaggg 1980
gaggtttttt tatttttatt aggtgtatgt tttgttgttg tttggatttt tttaaaattt 2040
aagttggtta tttgtgttta gtgtgagggt ggttggtttt atagtttagg tttagttaat 2100
gggagaggta gggttttggg ggttttttt tatagtatta ggtttgtttt tgtttattaa 2160
taggtggata gatgatagat agagaggttg gtgtggtttg tttagagttg ttgtgtttgg 2220
tatattttag ttagggtttt tgggttttgt tggtttttg tagtggtttt ggaagaatag 2280
ggttttatat agtttttttt ttagttgttt tttttttggt ttatagagtg tttaggtttt 2340
aggggttgta gtttgggggg gttttatgta gttggtattt gtgtttttt attttttttt 2400
ttttgaatga attttaagtt ttattgtttt aaggaggatt agatgttatt ttaattatat 2460
ttaggatttt tagttttggg gaaaggagga tagatttgtt tttatatga gtgagggttg 2520
ttagtagggt ggagagagag ggggttggtg ttagatttat tgtagtggtt tttttttttt 2580
tttatattga ggaattttta ggtatggagg gattttttag ttagttttat gttattaagg 2640
agaggtggta gaagggagtt ttttaatata gatttttgtt tttgttagaa gagttttggg 2700
tttattattt ttttttagtt ttgattttag ttgatgggaa tttaagagtg aggaaattaa 2760
gatatttttt gaggtttttt tagagatttt ttaagattaa aaggttggtt aggtgtagtg 2820
gtttatgttt gtaattttaa tattttggga ggttaaggta ggtagattat ttgaggttag 2880
gagtttaaga ttagtttgat taatatggtg aaattttatt tttattaaaa atagaaaaat 2940
tagttggtgt ggtggtatat atttgtaatt ttagttattt ggaggttgag ataggagaat 3000
tgtttgaatt tgggaggtgg aggtggtagt gagtggagat tgttttatga tattttagtt 3060
tgggtaataa agtgagattt tgttttaaaa aaaaagatta aaaggttatg agatgtattt 3120
ggtagggggg tttttagtgt tttagatata tttgttattt ttggggtagg ttttgaattt 3180
aggtttttga atgtgggttt gtgtggtatt tttagtaagg aggggaaggg gtttaggggt 3240
ttttatgttt gttgtattat tataagtttt tgggaatagg taatgttatt ttagggtagt 3300
ttttggtttt tatttgttat aagtgagttt atttttttt ttattttaga gatagttggt 3360
ttgatttatg ttgtgtaaag gtttttaagt gattagagta ttttttggag ataggttttg 3420
ggaggtgttg ggaatttatg tttttagttg gggttatgta gttatagtta tatgaaatgt 3480
ttagattttt tttgttgatt tttgaaaatt attgtattaa atgtttatta gtgaaatgtt 3540
gattgtgatg ataagaaggt tggggaggtt aagagaaaag ggaagagaga gtgtagttgt 3600
aaattttttt ttgggtttga gaagtattgt ttttttgtgt gtttaattgg tatttgttat 3660
taatatgttt gtttttattt aaatggttta gagtaaggtt ttgattaaat tagtattttt 3720
atgttaatat ttagtagtgt ttttggtttt tggtagattt ttttttagt tgttaaagta 3780
aaattataga agtgttttgg ttttatttaa ggtttgttgg ggttttttgt ttttttttgg 3840
agatttgaat agtttgtaga tgtgtggtat ttgtttatgt ttgtgttttg tgttatttta 3900
tgagtagaat tttttgtgtt ttttgtaatt tatattttga tttttaggtt tatgtttatt 3960
tgtagagggg gtttgtgggg gtttgtgggg gggagtatgg ggggatggaa gtttttagtt 4020
tgtttaaatg tgtttggagt aaatgggggg aaattttggt tatttgagaa atttttgttt 4080
tgggtggttg tttttttatt attttagtgg tgaggttaag gatggtttag gttttatttg 4140
gggtattagg tataggttgg ggtggggtgg ggggtgtggt taaagttttt taggggtttt 4200
tggttttgtt ggtggttttt ttggggtaaa ggattgtgtt ttgggatatt aatattgttt 4260
ttttaagatt aaaataaatt tttttaaggg ggttttttag ttatttttat ttttagaaaa 4320
gaagaaaggt aaagatggag gaagggaaga agatgaaagg aatgaaagaa gagagggaga 4380
gagggaggta agaaagagaa gtaaagtaaa gagaaaaaga gggagattga gaaaggggga 4440
aggaggaagg aaggagaggg agggggaggg gttagtttat tttggttttt ttagtgattt 4500
agtttttgga ggaggtttgg ggtttgtatt agagtttaag gggtggagtt ttgaagtttt 4560
gtttttgggg gtttttattt tttggagaaa gttttgaggt gaggtgggta ggggttaggt 4620
agggtggaag attattttta ttataggttt tgtaggggtt tttgggattg agtagtttgg 4680
gattatgggg gtttgggggt agtgaggggg tatggggggt gtagatggat ttggtggtta 4740
agttttgtta ttttatttag agtttttgtg tttttattta atagttaggt attgtgatag 4800
ttttttttgtt tggtttagtt ttttatagtt tttatttgga ggtgttagtt ttggtgttaa 4860
gttgtttttg tgtttatttt tttgatgttt ttttttttgg aagtttttttg atttgggtag 4920
gtttggttaa ttttttagtg tatgtatttt ttaaatggta tgttaattag ggttttagtt 4980
aggtattgtt tttgttgttt ttttgggatt ttttggaagt agtagaaatg ggtttttggt 5040
ttttaagtga tttaatttta gtttaggttt tatttatgtt ttgtgggttg ttgaggggat 5100
ttggggtttt ggaaaggttt gagtgggttt tttattttttt agggttggga gattatattt 5160
aggtttattt attgtttttt ttattgaggt ttgtgttttg agtagtttag gggtttttag 5220

```
ggataggtag gtgggtttga gttttgagtt atgtggaaag gatgggtagg tggtttttat     5280
ttgagatttg tattgtatta gttatttttt aggtttagtt ttagaatgat aatgttaggt     5340
ttgttttatg gttagttttg tagtttttga ggtttatatt agttggggtt tttttagttt     5400
atttgtttag gaggtttagt gtttgtttgg ggatttgttt gggtaattta ttttttttagt    5460
ttttgtgtag aggggaatag tagggtttgt tttttggatg ttgattttat tttatgtttt     5520
atgggtagta agggggtag ttttttgtatg gggattattg gagatattga gtagataggg     5580
ttttgggtag agttttggtt atagttgggg attattgttt tggtaattgg gtttagggag     5640
ggtattaggt gtttggtggg agggttggtt atagtagtag gtttgtatgg atagttagtt     5700
gattttgtat taagaaaagt ttttgttggg tttgtgttta tattttttta gtttttttgta    5760
gttataggtt agttagtaaa ttttttggag tttttggtgt gggttggaat ggtagggatg     5820
ggttttgttt tatatataga gagtgggtta ggaaggtttt atggttaggg gtgtttagat     5880
ggttgagagt ttttgggatg gttgaggagt tttagggata tgtgggaaag agattttttt     5940
ggtaaaggat gaggtttgta atagtagagt agggaagttt ttggtataga ttgaggggtg     6000
tagggtatgg ggagtttttg tgggggttag ggtgagatgt ttagtatttg taggtatttt     6060
tgggtttggg tgatggtggt tgtttgtata tagtagggga ttaggaagta gattttagat     6120
tttagttttt ttagaataag ggttgttttt tgtttttaga ataagggttg gtttgaattt     6180
attttagagt aaggattgtt aggtgtgtta tagtggattg tttagtatag gggattaggt     6240
agtaagtgag atattgaaat tatatttgga aaaatgttta attttgagga aatgttttaa     6300
gtttttttata agtggagaaa ggtagatggg gttttaaaaa ttatttggtg tagtagaatt    6360
tgaggtttgg tggttagtga aggttttatg gtgtgtttga tggttagggg ttgtagtgga     6420
tggggatgtg gaggttgtgt aggaggtatt ttggaggttg gatattttgg aggtttttgg     6480
gtttgagttt tgtatatagg ttagatgttt tttatagtta gttttaaatg gtgaaggggt     6540
tttattttta ggtggtttgg aatgttttta tagttgtttg gtggtggttt ttatttagga     6600
tttagtaggg tatttgttat aggatgtttt tgtatttggt tttgttttag agtttttatg     6660
tttttttgttt ttagttttag gggttatttt aatttggtta tgttaagtta tggtttttgt    6720
attgagttta gtttgttgtt ttgggtggtg aatagattgt gagtgtgggg gaggggagga     6780
gtgtttttta atattttttg ggggttttat ttattttatt ggatgatagt agttgaggat     6840
taaggtttgg ttgggttttg agttggtatt tttatgatgt ttttgagttt tagtttttttt    6900
tttgaaaaat ggggagtatt atgttagttg ttttttagtg ttatagggtt atgggggttg     6960
taagagttaa gatgaatttt tgtaggaagg aaaggggttt tgggtttagt attgggagtt     7020
ttagttttgg ttgtttttta gaatttattg tagagttttt aatataggta ttttttaaat     7080
atatttattt attaggttat tgttgatttt tttttatttg gattatattt ttttggggtt     7140
ggagttggtt ttggggtgtt ttaaggttat ttgggttttt atgagttttg ttgttgtttt     7200
tgtgtgttta ttagttgtgt ttattttttt atgtatttag gggtgtgttt tgtttttgaa     7260
agtttttata gtagttgtaa aggaggggtt tggttgggtt aatgttagta gggttttttt     7320
agtttatttt gataatggta tttttatttt tttggtagta aataaggggg aattgattta     7380
gtttagtttt ttggggtgta atgtttaagt tatttttttt ttagtatttg taggggggttt    7440
ttgaagaaga gagggttgta ggatatgttt gttttttttt tttgtaaatt aggttttgtg     7500
gttttatttt atttgttttt aggtttttg gttagtttgg gaatagtttt atttatttgg      7560
ggtattttttg gaattttgtt ttagttttta ttataagttg ggataggaag tttaaagtga    7620
tttttatagg ggaggtttgg tggtattgta ttttaattta gttttttggg gttttagttt     7680
ttattttttt attgggtttt tggagtggtt ggggtgtttt gtatataatt tgtttttgtt     7740
ttttttttagt ttttgtaatg tttgttgttt agggttgttt tttgatttgt aggagaaggt    7800
ttttaggttt taggttagta gatgtatttt tgttgtgtta ggattttagt ttttttttgat    7860
tgtttatatt tgttggtttg ataaagagga atggggagtt tggataattt gtgaatgaaa     7920
aagtttttta tttatttgtt aggggtttat ttttttttta ggttggtggg gaggtgaggt     7980
ttattttttt tatttttggt ttttttttgg ttatagttgt ttatggggta tttttggttg     8040
tgggtggtgt aggggttggg agttgggagg ttgaggtttg tgtttttttt gggtttgtgt     8100
atttggttgt attatttgta atgtgggttt atgagtgtgt ttggttgttg ggtggtttga     8160
tgggtggata ttgaatgggt tattttttttt tttgttggta gaggatggag gtttaaggtg    8220
gaggtgttgt tggtatagtt ggggtagatt aggggagatt ttttatttag ggattttttt     8280
tttttttttt aggttgtttt tttgtaaaat gttgaggatt ggtttttggg tttgggttat     8340
atgggttatt atttttggtt tggggtttat aggtagagat gttaatggag gtatataggt     8400
agttgtattt gtatttgatt agtattaggt ttgaggtttt tgggtttttt tatgttttat     8460
tttattttta tttattagga gtagaggatg ttgtggtggg tagagttttt agtttgggag     8520
ttgtatagtt ttgtgttttt ttttatggaa aatttgtttt tttaggttgt ttggttgttg     8580
ttatataaat atttttgttg tttttaggtg tttttttttt tggggtgttg attttaagat     8640
gttattttag gtttgtagaa gtgaggttag attttgggag gatgtaggtg agggaggttt     8700
ttagtagtag ttatagggt gggataggtt gtttgtattg gttgttttta gagtgtttag      8760
tatttggtaa gaggtgtgtt aggaaaggtt tgttggggtt aggtgtgaga tttgttttgt     8820
tttttgtgtt tgtttttgtt attatgtttt ggggtttttg tagttttttt tttaggtttt     8880
```

```
tatagtttta ggtttttttt agtttttttgg ttttttgta gggtaggttt agttttttta     8940
ttttaggagg ggtgttggga tatgtgtttt ttgttattgt atttattatt ttgtgttggg     9000
gtggggaggg ggtgttgttt gtttttggta tgaggttgtg aattttgtgt agggattatg     9060
gtaggtattt tggattttttg gtgtttaatt aggtttggta tagggtgttt gtgtttggta    9120
ttgtggtgga gaaataaatt agttggaagg agtatgtggg aagggttgtg ttggtgttag     9180
tggtagattt gtttgatttg tttgtgtttt ttggggttat tttaggtagg tggtgtttgg     9240
gtaggaggag gatggtgatt gaggagtgtg ttgatgtggg ttttttttgg gggagtgggg     9300
tataatttgt ttgtgaggta ttggttttgg ttgttttttt ggggtgtttg gagtttttttg    9360
tttagaggtt gtggtgtttt tattttttgt ggttaagtgt ttgggtaatt atggttaggg     9420
atgtttggtg gtgattgatt ttttggatgt ttaaagttgt ggttatgggg tttttgggag     9480
ggagtgaagt tgttgggtta ggggtgtata tatggttagg ttattttttt agagttttgt     9540
tttgggtttg gggtttttta atgtggtttt agttgttttt tgtttggttt agtgtatggt     9600
gtatatggtt gtttgtggtt ttgtttttttt tattttgttt tgggtttttt tgtttattta    9660
tatgtaaatt ttagttgtta gttgttgttg agtgtggtaa ttgttagagt tggatttttt     9720
tgtagttttg gtttaaattt ttggtttttg aaaattttta aatgagaagt agttttaggt     9780
gtttgttttt gatttatgtt gtgttgttgg gttttttttt tttggagagg ttgggtttgg     9840
gatgtgtggt ttagtttggg gaggtgtaaa ggtggatggg gtgtgtggga ggaggtggtt     9900
gtggaggggg tggggggatt ggtgggggtg gggttgggtg gggtggggtt ggtgggggtg     9960
gagtgtattt tgattttgag ttttattagt gttttggttg tgggagggag tgtaagggtg    10020
tggggtgtgg ggtgtgggtg tgggtgtgag tgtagtgaag gaatgagttg ggtgtggagt    10080
tgggtttggg ggtttgtgag agtatagtgt tgttagttag ttggggaaga gagggtggga    10140
ttgtttgttg ttgttttggg attgtatgtt gtgtgtgtgt gttttagttt tgttggttag    10200
tgtaggaggt tgttgtttgg gtgtagaggg tagttggtgg ggaggtatgt tgttgttttt    10260
ggtgtttttg ttttgtttgg tgttgttgtt tgtgtttgtt gtatgaggta ggtgtttatt    10320
tatttgtgag tttttatttt ttgtgttttt tggaaatttt ggtggtgttt ggtgtgtgtg    10380
ttttatggtt gggagtgggt ggtggggagg ttagtatgga gagggaaaag tgggtggttt    10440
ggggtgtggg gttttggagt tttgggatta gggaggattg attttttttt ttgatttttt    10500
tgtggaggtg gatttgtgtt gtttgtgttt ggagttgagt taggaggttg gtgtggggtg    10560
ttggggtttt ggaggtttta ttttgggttt gttttttatt tgttgtgtgt ggggtttgtt    10620
gttggttggt tgggtgggtg ggttgtttat tattttttga tttgaataga gttggtttttg   10680
gtttttttgtt gttttttttgg gttatttatt ttttttttt tttgtttttg gtttatgttg   10740
gggtggatgt tggggtgtgg agtgtgggtt ttgtggtgtt gtgtttgttt ttattgattt    10800
gtgtggttgg gttgggtttt tgggtttttg gttgttttgt ttgttggtgt tttttagttt    10860
ggtttttagt ttgggggagg ggttgtgtaa gaagttgttg tgtttggggg gattgaattt    10920
tttttttgtt ttgtggagtt gaagtttgga aagtttgggg gtggagagtg ggatgtgggt    10980
ggggggtttt tatatttttt tttgttgtat agtgagtggg gtttttgggg aattgagtga    11040
ttaatttatt ttttttttga gagttggagg tgagaattat ttgttttttt ttagaaagtg    11100
tggttttgtg ttatattttt tttttttgtt ttttagtttt ggtaagatgg ggagggaggg    11160
gtttgagtaa ttgatttttt tttgagatgg ggttgaattt tttttgaatg ggggattttt    11220
attttttttt tgtgggtgta tgggggttgt tttggagatg tgtgtttgtg gaggtaggta    11280
ttgggtgttg gtggaggtgg ggttgtgttt ttagggtgtt gttttggtgt ttttggaggt    11340
ggttttgatt ttataatggg ttgttttgat tttgaggtgg aggttggtgt tttgttggtg    11400
ggtggggtag tgggtgagta gttgttgtat ttttttatgg gtgggagttg agtgtggtta    11460
tttttttttt ttttgtttag ttttgttttt ttgtagaatg ggtttgaata gaggtaattt    11520
tggtggttgg atggggggtt ttgttttgtt agatttttta gtgagttttt agggtggggg    11580
gtaatgtttt ggagagtagt ttattttgtt tttgtttttt ttttattgtg tttaggtggt    11640
atattaggtt attttatttt gttttaagta ggatgtgttt tttgttgttt tttttttttt    11700
tatttttgat tatagaattt tgggtagaat gttgatgttt ttttggtttt atggaggggt    11760
tttttgtgga tttgtgtttt agtaatttat gatattgagt agagtgtgtt tggggtaggg    11820
ggtggatgat gtttttttggt tgggtgtagg tttttgtttt tagggtaatt agggtttttg   11880
gtggtggtgg tgtgggtgag attgattttt tttttttgt ttttgtttag gtttgtgatg    11940
tttttagttt ggtgagattt gtttgaatgg tgggaagtgt gaagtggtta atggtatgga    12000
ggtttgtgtg tgagtattat ttttgtggga tttgttgttt tgttgagggt agagttttttg   12060
tttttttgtag ttgtgtaggg gatagaatat taggttattg ttttttggag atggtttaga   12120
gtttgggtat ggttatgtgt tgatttttat tggataaagt ttggtaatta tttaattatt    12180
agtaattatg ttgtgtgtgg agttggtttg gttgttgagg gtttgggtat ttttttgtgg    12240
gttattttag gttttggggt tattaagtgg gagttttagt attttaattt ttttttaagt    12300
ttttaagaaa tagttgattt gttaggggaa gttttggatt atttattgag aggtttagtt    12360
tttattattt tgttaatttg gggtgttttt tttgggtggg tgatgttttt tgggtatagg    12420
tgatgttttt ttttgggggt gatgtttttt aggtgggtgg tgatgttttt tgggttggta    12480
gtgatgtttt ttttggtata ggtgatgtta ttttttgggt gggtgatgtt tttttgggt     12540
```

```
gtgtgatgtt ttttgggttg gtggtaatgt tttttttggg tataggtgat gttatttttt   12600
gggtgggtga tgtttttttt gggtgtgtga tgtttttttt gggtatgggt gatgttattt   12660
tttgggtggg tgatgttttt tttgggtggt gatatttttt ttaggtgggt gatgtttttt   12720
ttgggtggtg atattttttt taggtgggtg atgttttttt taggtatagg tgatattttt   12780
tgggtaggtg atgttatttt tgggtgggtg atgttttttt aaaggaaatt gatttttttaa   12840
aattggattt gtttttgggt tttttgtttg ttttagtttt ttttttttgg ttttaggttg   12900
gggttgggga agagaaggtg gaatttatgt tttggtgtgg ggtgtggggt aattattgag   12960
tttggttttt gttttggtat ttgtaaattt tgattttaaa attttgtggt ttttttggtt   13020
tgttagtttt agatgtaaaa tgggtattga gggggttggg aagttgtgtg gaattgtttt   13080
tttttgtttg tttgtgagtg tttattagtt gtggggaatt gttttgttgt ggttgatgtt   13140
ttttaatttg agtttatttt tgatgagaat aatgaggagg tgtttggtgt ttggaaaaag   13200
agtttttttt gtttttttagg agttatagat gaagaaatgg tttttttagtt agtttgtgtg   13260
tttttatttt aattgtagaa ggttttattt ttttgataat tttagtttgg tttttgtttg   13320
tgttgattgt ttagtttgtg ggtagtaggt tttttttttt tttggttttt attttttttta   13380
aattgaagtt aattaagtag gtttgtgagt agtttaatgg ataaagttga ttgtgttttt   13440
gttagatatt aatgaatgtg tattattttt tttttttttt tttttttttgt tttttggggt   13500
tttttttttt ttttgtagaa gttgtatgga tgtttgatgt tttaaatttt gttttttttt   13560
atttagagat tgatagttgg gatttagggg aagtgtagta gttattgttg ggggaggtgg   13620
attaatgttg tgtgattaat tatgtgtggt tttttagaat gtataatttt tttttttgtt   13680
attgtgtttt tggtttggat tttgtttttt atttatttgg ggttttggga gaagtttttt   13740
gtttttgtag aggattttgg gagggtggga ggaatattgt gttgggaaaa ttgggggttt   13800
gttgtttttt tttagtggta ttagggagta ggtatagttt ggtttatagg ggtttgggat   13860
tgaggttttt ttgggttaga gtgttgtgtt ttggggtata gattgttgta gttttttggt   13920
gtttgggtta aaattttgtt atagggttgt aaaatattgg tgttatgggt ttattttttt   13980

attttttaaa tttgggattt tggtgagttg tttttagttt aaggtaggtt gttgattatg   14040
gttttggtgt ttggttttgt ttttttttgtt ggggtttggt gttttttttt ttttggtggg   14100
aagtttgttt tagtttggtt tgataggtag ttgagtttag tttatagaga ttttattgtg   14160
tatattttat aagtttaatg agaagatggg atggaagtgt gtttttaggt ttttgttttt   14220
gggttgtaga gttggaggtt agggttatag ttgagtttgg tttgtggagg tataggttag   14280
ggtttatata gtagtttggg ggatattgta gttgttttgg agaaggtttt gggttttttt   14340
tttgggtttg agttttattt aatatatatt ttttaggtgt ttttgttttt ttgtttttgt   14400
ttattttttt tagaaggttt aggaggtttg tttaggagtt agttagggta tttgtaggag   14460
gtttggtttt tttagtaagg ggagaaattt atattgtttt ttgaaagagt ttggggtgta   14520
ggtttttgga gttttgttag tttgagtggg aattttggtt ttgtggtatt tagttgtgat   14580
tttggaaaga gttttggttt ttggtgttat ttgttgtttt tgttgtagaa ggggtatggt   14640
tatgatgggt gttatagtgt ttggtttatt agtattgggt gaattatttt tttttatttt   14700
aaagagaagg aatttttatt ggaagttgtt aattaggtgg ttgtagggtt tgggattgga   14760
aaaagtggtt ttgttggtgt aaaatgtttt tttaggaggt tagagtgttg ttgtggtttg   14820
gttagttagt ggttaggtag aaagtatttg gtagtatatg ttggtgggtg gaggatggtt   14880
ggtttttggg tagttgttgt gtgaggtgtg gttgtggtgg ttgtggttgg agatggtggt   14940
tttttgagta ggtttagggg gttattgtgg tttttggttt tttttttagtt tttttattag   15000
ttttgttagt gtgagtaagg ggtattgaag agggttttat tttttttttt tttgttttga   15060
ggagagttgg gaagagttgt agggatttta tttgtttggg tgagattatg tttttggttt   15120
gaaatgtttt tttagggttg tttgtgtttg ggttgggtgt tgaggtaggg ggtagttaag   15180
atgttggttg ttgatggtag agttagaatg gtatggggtg tttttttagga gttgggagaa   15240
atgggggtag gttgttatat ttagtatttt taggaggatg tttagttgtt tgttgggtgg   15300
tgtgttttag ttttggataa ttttgtgttt gaggtttggt ttggttatta gttttgtgat   15360
tttgggttat taggttatgt aagttttagt tttttttagta tgttagttgg gttgatatat   15420
gttggatttt ttggtggtgt gtgaggattg tatgagaatt gtgtggagag ggtatttggt   15480
tagtgttttt tttttttttg tttttttttt gttggttgat agtgttggtt tttagagttt   15540
tttttttgaa tgtttaattg attgatgagt gaatttagag aattttaaag gtgttgtttg   15600
gtgggttggg tttggtattg tttggggagg gatttgttgg ttttggggaa gtttattttt   15660
taggatggtt ttagttttgg ggaagtttat tttttagggt ggttttagtt tatagatatg   15720
tttgagtgtt tgttgtgtgt tgtgttttgt tttaggttag gtttttggtg gggatagggt   15780
agatatggtt tttgtttttg tggagtttat tttttagtaa ggtgtgagag aggtatttag   15840
tttatatgtg ggatatgttg ggggtgtgtt ggtttggtgg tggtttggta tagtttttgg   15900
tagttttgta aagttgtttg gattaggtat tgataagtga gaagaagaga tttagagaag   15960
ttgttattag ttttagggtt atatagtagt ggtagaattt ttattagttt tgtttttttt   16020
tttttttttaa gtgaatgttt ttaaatatag ttttagttag tttgagttgt tttgttattt   16080
tttgattata agtggattgg gggtgtggtt ttttttttaaa agaagaggaa ggaggtttag   16140
```

```
gtgggaagtg atttggtttt gtagttggtt tgggaggttg gggagggatg gggtagtttt   16200
tgttatttgg tttggttttt tttattgagt tatttgtttt gttttgggtg tggttagggg   16260
aggaataggt tgattttttt ttttatgttg agttgagtgg aaaggtttgt gataggattt   16320
tttgtttatg tagaatttgg tttttaaggg tgttagggtt agaaaaatat gtttaaaaat   16380
atgtagtatt tgaggtgggt agattatttg aggttaggag tttaagatta gtttggttaa   16440
tatggtgaaa tttttgtttt tattaaaaat ataaaaatta tttaggtgta gtggtgtgtg   16500
tttgtaattt tagttatttg ggaagttgag gtaggaggat tttttgaatt taggagatgg   16560
aggttgtagt gagttgagat tgtgttattg tattttagtt tgggtgataa agtgagattt   16620
tgttttaaaa aaataaaaaa taataaaaaa aattttatat agtgttaaat agaaagtttt   16680
atttatttag attggggaga tattaggaat aggaaggttt ggggtggaga ttggggtggg   16740
agtttggagt gttttgaagg aagttggttt tttggggtag gttttgtttt tgaatagtag   16800
ttaatatgtt ttttattgaa aagaataata atagaaaaag agagagggag aaagaaaata   16860
gtaattttgg taggaagagg gtagggtagg gtagatagtg tattagggga agtgttttta   16920
tagggtagag ttgggtgtgg ggatttttag atgtgggtgg taaattggag attttatagg   16980
agttgggatt ggttttattt tttatttttt gtatttgtga tgggtagtag tggttattgg   17040
tgtttattgg gtagttttag gttgggttga gggattgtat ttttttgttg tggggagggg   17100
atttttttg ttttatgttt tgtttataaa tagtgaaagg atgtggtttt gtagaattga   17160
ttgggtgttt agtttgtttt ttttaataat gtagtgatga tagttttata tttaggatgt   17220
tgttgttatt tagttttgta aaagttttgg tagatggttg tttatttttt aggtgtgtta   17280
gtgtagtttt tatttttagt gtataggtgg gaaagttgtg gttgtgaggg tgtggggatt   17340
tgaggtaggt tggtgttagt ttttattttt atgtgtaggt atatttagaa ttttttggat   17400
ttttttttgg gttgaggagg aaatattagt agttttaatg agtggttggt tttggggtat   17460
tatatttgtg ggttgggttg tgtttgatgg ttttgagttt tttaggtagt gttggtgttt   17520
tttttgtgg ggtttggagt tttgaggggt tgtgaagtag ggttggtttt gatttgttgt   17580
gtttttata ttgttttttt tttgggtttt agttttttta tttgtatgtg attttttttt   17640
taattatagt tagtgggatt ttggggtggg ttgaaggtgt ggtggtggtt gtgggttttg   17700
tggtgaggtt tgtgttgggt ttggtggttg ttttagttgg ggaaggggta gtggttgtag   17760
gggtgggagg gtggagggaa gtttttttt aaagttgttt ggttgggttt tttttgtggt   17820
tttgttttat tttattttgt tttttgggag taaagggagt taaggattgg tttggggatg   17880
gatatgtata tggagggatt ttgggtgatg atttatttat attataaaat ttgttttttt   17940
ttgtttgttt tttgagatgg agttttgttt tgttatttag attggattag agtgatgtag   18000
ttttggttta ttgtaatttt tgtttttag atttatgtga tgtttttgag tagttgggat   18060
tataggtata tattattatt tttagttaat ttttgtattt ttagtagggg tagggtttta   18120
ttatattggt taggttggtt ttaaattttt aattttaggt gatttatttg ttttggtttt   18180
ttaaagtgtt gggattatag gtgtgagtta ttgtgtatgg ttattttttt gtttttgag   18240
atggggtttt attttgttat ataggttgga gtgaagtggt gggattatgg ttgattgtag   18300
ttttaaattt ttgggtttaa gtgatttttt tgtttttgtt tttagagtag ttgggattat   18360
aggtgtgtat tattatgttt agttaatttt aaattttttt gtagaagtgg ggttttgtta   18420
tgttgtttag gttgaaaatg tgatttttga attaaagtat gttgttgttt gatatttagt   18480
ttatttataa gtttatttaa ttattattta gttttgggat tttttattg tataaaatag   18540
aaattgtatt taaatattgt tattttgttt tgattgtttt agtttttggt aattattaat   18600
ttgtttttg tttttgaatt tgttttttt ggatgttgtt tatgaatgga attgtgttat   18660
aggtggtttt ttgtttttgg tttttttat tgagtgtgat gtgtttaagg tttatttatg   18720
ttatgtgtgg attagaattg tatttttttg aggattatat tttagttttt agaagtatta   18780
gggattggtt agaggtggat tttagtattt tagtttttag atagtttaga ggaggtaggt   18840
gttgggaggg tgtttggggt ggttttttgg aagagatggt gtttattttg ggttggtgta   18900
gttatttgat agttatagag ttttttagag ggagttttgg tatattttta ggtgaatttg   18960
taggatggat tggtgtgttt gtggttttg ttttgtttag gattttgaga taggtttata   19020
atttgttttt gggttttatt tatgttagtt gttaagttgt ttagtatttt ttagttttta   19080
ggaggtatat agtggggttg tggttattga gtgtttatat gtttggtgtt gtaagaatgt   19140
aaagtagagg gtttttattg agtatttatt atatgttgaa ttttggggta tgggtgggta   19200
tggtagatgt aggttttttt gggtagggga ggagtatgtt ttttaagaat taggttatag   19260
gatggatggg tttggttata agttaggggt agttattgta gtttttatgg tttttttgtgt  19320
gtaaagttag tgttgtggga ttttatttgg gtgtatttgt tgtggttttt aagtgaggtt   19380
tattatggag tagtttttat gttggttggg atagggttag tggtagggag ttttagatta   19440
aaggagtggt ggtggttggg gtagtggggg gtagtgaggt tttggatata tttatggggt   19500
gggtaggtat tttagttttt atttgtttag tttgtttgtt ttggggtttt ttttttttg   19560
gatagagttt tgtgtgattg gttttaggtt gttttttttt ttggattttt agggttagtt   19620
tagagtgatt ggttatgttt tggtttagat ttaggtttgt gtgataggag agagttagga   19680
atttggtttt ttattttgag atatagatgg tgttttttttt aagttgtttt tgggatgggg  19740
aagtgttatg ttttttgtag ggtattaggt tagttagtgg tttttgatag tgaggttttg   19800
```

```
tttgtaggta gttgggggat ttttttttgt tgggaggatg tgtgtgtgtt agtattgggg   19860
tgtttggttg ttttaggggt ggtgtagggt ggttataggt ggttttagta ttttggggtt   19920
gtttttagtg tatttttgag atttgtggaa ttttgggagg agaaattttt tagtagtgtt   19980
tttagtgtgt ttttttattt tgtatttgtt gtttttggga gggattttga gtgtttttttt   20040
ttatttatta tgtatttatt gttttaggag ggattttgag tgttttttttt tttatttatt   20100
ttgtatttat tgttttggga gggattttga gtgtttttttt ttttatttat ttttattttg   20160
tatttattgt ttttgggagg gattttgagt gatttttttt ttatttattt tgtatttatt   20220
gtttttggga gggattttga gtgtttttttt ttttatttat tttgtattta ttgttttggg   20280
agggattttg agtgtttttt tttttattta tttttatttt gtatttattg tttttgggag   20340
ggattttgag tgtttttttt gatttatttt gtatttattg ttttaggagg gattttgagt   20400
gtttttttttt ttatttattt tgtatttatt gtttttggga gggattttga gtgtttttttt   20460
ttttatttat tatgtattta ttgttttggg agggattttg agtgtttttt tttttattta   20520
tttttatttt gtatttattg tttttgggag ggattttgag tgttttttgt ttattttgta   20580
tttattgttt ttgggaggga gatttttgta gaggttttat ttttgaattt tttatttata   20640
ggtttagggg tttaggttg ggggtgtttt gttgtttata ttttagattg agtttgtatg   20700
gttaaatagg gtttttttttg tatgttttgt ttaatgtaaa gtggtggttt tgggtgggtt   20760
tttaaggggt tttgtgaatt ttgggggttt ggttttttttt atttggttgt atttgttggg   20820
tgggagattt tattttttga gtttttagtt ttttagtttt agttgtagtt tagatattag   20880
ggttttgtgg ggtgggggtg gggtagtggt ttttttaggt ttgtggggtt gttttgttgt   20940
ggtttttgta attttttgga aggaggtttg gggtttggtg tttagtgggg tttgtgggga   21000
tggatttggg ggttttgtgg ggagggattg ggggttttgt ggggatggat ttgggggttt   21060
tgtggggagg gattgggggg ttttgtgggg agggattggg ggttttgtgg ggagggattt   21120
gggggttttg tgaggaggga ttggggggtt ttgtggggag ggattggggg ttttgtgggg   21180
agggattggg ggttttgtga gagggaattg gggggttttg atgtgttggg tgtgtatagg   21240
ggaggttttg tttttgatat ttatatttgt agtttttagg tatatatagt ggttttattg   21300
gggtttttgt aattatagtt tgtgagtggt tattattaag ggtgtttttg gattaggtta   21360
ggtttagatg ttgtttggat ttaattttta tagtgtttgt tgttatagta gggattaatg   21420
gttatagaat atttatttgg ttttatttgt atggggttgt ttagtttggt gggtattagg   21480
tgggtgttgt ttttgtttag tgggaggttt gttagttttt tggttgttag ttgagatttt   21540
tttttaagaa atagttgtgt tgtttttttt gttttgtttg gtattgaggt tggtgatatg   21600
ttttgatttt gttttaattg gtagatgaga atttgttatt agatgttgat tttgtttatg   21660
tagttttttg gttttggagt ttattgtgta tttttgaagg gtttgaattg gttgtttaaa   21720
gttgggtaaa tgtttttttg atattttgtt gatattgtta atttgttgaa taaatttttt   21780
tataggtagt ataggaggtt agttttgttt tagtttgagt taaggtttag ggaggtaggt   21840
tttgtgtgga gttagttttt ttgtgagtgt agtggttttg taggatgtat tgggtttgag   21900
aagtggattt ttttgttttt tgttatattt taaaagtttt tagttatgag ggtaggatga   21960
agttgatggt tgtttttgtg ttgagatgat aattgggtat agaggtggtg gtttttagtt   22020
ttttgttttt ttttttgtag tggggttatt tagtgtgatt tatggtttga gttataggta   22080
tttgtttagg gagttatggt ggggagattt ggttttatta gagatgtgtt tgtgttttat   22140
taagtttaaa ggaggaaatt ggtgtgttt                                      22169
```

<210> 275
<211> 3286
<212> DNA
<213> Artificial Sequence

<220>
<223> chemically treated genomic DNA (Homo sapiens)

<400> 275

```
agaatttgtg gttaatttta gaggattggt gagggttggt ttgggatgaa taaatatttg      60
atatttgatg gttttttttt tgtggtgatt tttattaagt tgaagtggtg tgtgtgtggg     120
taggtgggag ttgggatttg gtttgtgtgg tgttgtgtat ttggattttg aggttatgtt     180
ggtgagtgtg gtgtgggggg tggggagtgg gatagtggga ttgtttgggt taagtgattg     240
ttttaaagat attttttatt aagatttttt tgtatttgag ttttattttt ttttggttta     300
ggaaagtttt tgtgtaagtg gaagatgatt ttttttataa agtgttgtat gttattgtgt     360
tttggattgt gtatgaatat tatttagttg tgggtgaagt tttttatggt gggttttttt     420
tttatttggg tgtggtgttt tagttttagt tttatttgta tggtatattg tgggtagggg     480
gaggagagat agttgtgaat aataggaagg tgaggttttg gtagtgaatg ttgtgtttga     540
tatttttttt ttttttttg aaatgtatat aaaaggaaat ggtggtgttt tttgtattta     600
```

```
tgttttatgt gtgtgggtgt gtatatttta tatttttaaa tatatttgtt tgtttggttt    660
ggtttggttt taggtgtttt gggttttgta tttatattgg ataggattgt aatggaatgt    720
tatttttagt tgggaagggg gttggggaaa agagggagaa tatatttagt tatgataagt    780
atgataataa atgtattgga aataaattag aaggtgatgt tggggtggtt ttttggtgtg    840
ggaggggagg tggttgggat ttagggggat tgaagggttt ttggtgagtt ttttttttga    900
aagtattgtg gtgataggta tatgttgagg aagtttttgt gtatgtgtgt gtgtgtgtgt    960
gtggagtgtt gtgttaggtg aaatggaaat tataggtagt ttttgttgat gggtataaat   1020
ttttgtgttt ttgttatggg gtttgtgtat aataaaaatg agatgttgtt atatatattg   1080
aaatatatat atatgtttgt aggaaaatat gttgaggtat ttataattta gagtattttg   1140
tatttttttag tgaaaagttt tatgtgattg gtgaggttag ttgaattgag tttttgtaat   1200
atatttttaa gagtttaagt ggttttaaag tattttttat ttttttttaa aaaatgaaat   1260
ttagaaaggt agggtggtgg gtggatagtt gttgagtttt ggtttgtgtt tagtattttt   1320
tggtgttggg gtaaagttgt gtgtggtttt gttgttgtta gttttgtata ttttggttta   1380
tatatgtgtg ttgtggagaa tgtattattg tagtggttgt ggtgttttgt ggaggtgtgg   1440
ttgttgaggt tgtttgttgt gtttttggat tgtgtttgta gtttttggtg gtggtggttg   1500
aaatatggtt gagttattat ttgttttttt ttattgtgtg tgtgtgtgtg tgtgagtgtg   1560
tgtgtgtgag tgagagggag tgtgtgagtg tgttttttgt gattgtaaag aggtgaggag   1620
ggagggaggt gtgggggtg gaggggtgag tgtgagtgtg tgtgagtgtg tgtgtgtttg    1680
tgtgtttgtg tgtgtgttgg gggggggtgg gggaggaggg gagtggaggt tgagggagag   1740
gtggtagttt tttgtaagtt gtattaattt ttttttaatt ggaattgtgg agtgttggtg   1800
ttgtttgggt tgtggtggtg tagggtgagg aaggaaagtg ggggagggtt tttattatta   1860
tttttgtgtg tatttattga gttagttatg tttgggggtt tggaggtttg ttggggtgtt   1920
gtgtggtatt tttttttttt ttgttttttt ttagttgtaa tttatgaaga ggttgttatg   1980
aatgagagtg tgtttaggag tggagggtag atggtggata ttttttgttt tttttttttt   2040
gtgtttgttt gtttgtttgt ttgtttatta aatttagttt taaaagtaaa agtagtagta   2100
gtagtagtag ttttagggta aagaagatga ttgtggagta tgtgttgtgg tgtttgatat   2160
ttgggttttg agttttatag ggtggaggga gggtttgtgg gtggggtgga gtgtgtgggg   2220
aggtagagtg agtgagttag ggggagtttg aaagagttaa ttatttgtta ttttttgaaa   2280
ttttggtggt ttgtagtgtt atattggaaa gttttgttta aagtaatagg tttttgttgg   2340
gggggtggta gggagggat gtaggagtgg ggagagtggt tagttgggga gttattttag    2400
tttttgtgta aattttgttt gttttataat aaaaaatgga ttaaaattgt tggaatgtag   2460
ttattgttta gagtttttag tagttggaga gggggtgtta aattaagttt ttagttagta   2520
tgtatgggga ggagggagtg ggtttttttt tattttttat tatgtatttt tgtggttttg   2580
tgttgaaaag ataagaattt atatttaaat aaatttattt aaagagagtg agagaaatgt   2640
tattttgtaa aaaatgagta tgaaaatagt aatgattgag gttgttttaa tttgtaatga   2700
ttttttggtt ttttgtttta tttatttata ggtatttgtt tgttattttt attttttgtt   2760
tttttgttat tgttattgaa aatttgaaaa tgaaatgaat aagggagaaa taaggatgtt   2820
attttttgt aagggaatgt tttttgtttt ggtaattttt ggttgggaag ttttataaag    2880
tggtgtttag gtgaaagtaa gatatttggg agtgttgggt atagattttt ggggattagg   2940
tttggagttg ttttttttgg ggggtttttt tttttgatag tttattgagt tgtattagaa   3000
tgtggaggat tgttttttga ggatgtgtgg ggtaattggg agttttaaaa tagtagataa   3060
agttaatatt gattgtaatg tgtgtaaatt ggattataat ttttaggtgt attggtgata   3120
attagtgtaa tttattttg agtattaatt tggttagatt tttgggtgat agttagttgt    3180
aggagtgttt ttagagttgt gtttttgtaa atgtttttat ttgtgttgta tttgatttta   3240
gtggttggag atagaatagt gtggttatgt gtggggtgtg gagagg                  3286
```

<210> 276
<211> 3286
<212> DNA
<213> Artificial Sequence

<220>
<223> chemically treated genomic DNA (Homo sapiens)

<400> 276

```
ttttttgtg ttttatgtgt ggttgtgttg ttttattttt ggttattgag attaaatata      60
atgtgagtga gaatatttgt agaggtgtga ttttgggaat atttttgtga ttggttgtta    120
tttgggagtt taattggatt aatatttaga gatggattat gttggttgtt attaatatgt    180
ttaaagatta taatttaatt tatgtatatt atagttagta ttaattttat ttgttatttt    240
aagatttttg gttgttttgt gtgtttttag aggataattt tttatgtttt gatgtaattt    300
```

```
agtaggttgt taggagggga agttttttga agaaaataat tttaagtttg gtttttgagg     360
gtttgtattt ggtattttta ggtgttttgt ttttatttgg gtattgtttt atgaagtttt     420
ttagttgggg attattaaag tggaaggtgt ttttttgtaa ggaaatagta tttttgtttt     480
tttttgttt atttatttt taaatttttg gtggtggtgg tagaagggtg gggagtgagg     540
gtggtgggtg ggtgtttgtg agtggatggg gtagggggtt aggggattgt tgtgggttag     600
aatagtttta gttattgtta tttttatgtt tgttttttgt aggatggtat ttttttttgtt   660
tttttaagt aagtttgttt gggtgtgagt ttttgttttt ttagtataaa attatagaaa     720
tatgtggtgg aaagtaaaag aaagtttatt tttttttttt tgtgtgtatt agttaaggat    780
ttgatttaat attttttttt tggttgttga gggttttaag tagtggttat attttagtag    840
ttttaatttg tttttattg tagggtgagt agagtttata taggggttgg gatggttttt     900
tagttggttg ttttttttat ttttgtgttt ttttttgtt atttttttag taggaatttg     960
ttattttaag tgaggttttt tagtgtggtg ttgtgggttg ttggggtttt gggaggtggt   1020
aggtgattgg ttttttttgag ttttttttgg tttgtttgtt ttgttttttt gtgtgttttg  1080
ttttgtttgt aagttttttt tttgttttgt gaggtttgga gtttgggtgt taggtgttat   1140
ggtgtatgtt ttgtaattat tttttttatt ttggagttgt tgttgttgtt gttgttttttg   1200
tttttggggt tgagtttaat aagtgagtga gtgagtaagt gagtgtgggg ggaaaaaggt   1260
agagaatgtt tgttatttat tttttgtttt tgggtgtgtt tttatttata gtagtttttt   1320
tatgaattat agttgagggg gggtggagga ggggggggta ttatataata ttttagtaaa   1380
tttttgggtt tttaggtatg gttagtttgg taagtatgtg taaaaataat aataaaagtt   1440
tttttttatt tttttttttt gttttgtgtt gttgtagttt agatagtgtt agtgttttgt   1500
ggttttaatt agagaaaggt tggtatggtt tgtagggagt tgttgttttt tttttagttt   1560
ttgttttttt ttttttttat tttttttgg tgtgtatata gatatataga tatatatata    1620
tttatatatg tttatatttg ttttttttatt ttttgtgttt tttttttttt ttgttttttt  1680
gtagttataa gaagtgtatt tatatatttt tttttgttta tatgtgtgta tttatatata    1740
tatatatata tggtggaagg aggtgaataa taatttagtt atattttagt tgttgttgtt    1800
gggagttgtg ggtatagttt ggggatgtgg tgagtagttt tggtggttgt atttttgtaa    1860
agtgttgtgg ttgttatgat ggtgtgtttt ttgtggtgtg tgtgtgtgag ttgaagtgtg    1920
tggggttgat agtggtgggg ttgtatgtaa ttttgtttta gtgttgggag gtgttgagtg    1980
tgagttgagg tttggtggtt gtttgtttgt tgttttgttt ttttgaattt tattttttgg    2040
ggggaggtgg gagatatttt ggagttattt gggtttttga aagtgtgttg tgggggtttg    2100
gtttgattgg ttttgttgat tgtgtggggt tttttgttgg ggggtgtggg gtgttttaag    2160
ttatggatgt tttggtgtgt ttttttgtgg gtgtgtgtgt gtgttttagt gtgtatgatg    2220
atgttttatt tttgttgtgt atgaatttttg tggtaagagt atgggagttt gtgtttatta   2280
gtagaggttg tttgtagttt ttattttgtt tggtatagtg ttttatatgt atgtatatat    2340
atgtatataa agattttttt ggtgtgtgtt tgttgttgtg gtatttttgg ggaggggggtt  2400
tgttaggagt tttttggttt ttttgggttt tagttatttt tttttttatg ttgggaaatt   2460
gttttggtat tgtttttttga tttgtttttg atgtatttgt tgttgtgttt gttgtggttg  2520
agtgtgtttt tttttttttt tttgattttt tttttgattg gggataatat tttgttataa   2580
ttttgtttga tgtagatgtg gggtttgggg tgtttggggt taagttgggt tgggtgggtg   2640
ggtatatttg ggggtgtgga gtgtgtgtgt ttatgtgtgt gaaatgtgga tgtagagggt   2700
attgttgttt tttttatgt gtgttttaag aaggaggaaa aaaatgttag gtgtaatgtt     2760
tattgttgaa atttgtttt tttgttattt atggttgttt tttttttttt tgtttgtagt     2820
gtgttgtgta ggtgaagttg gagttggggt attgtgttta ggtgaggaaa aaatttattg    2880
tggagggttt tatttatgat tggatggtgt ttgtatgtgg tttggagtat agtaatatat    2940
agtattttgt ggagaaagtt gttttttatt tgtatgaaag ttttttttagg ttaaaaagag  3000

gtagggtttg aatataaaag ggttttgata aaaaatgttt ttgaaataat tgtttggttt   3060
gggtggtttt gttgttttgt tttttgtttt ttgtgttgtg tttgttggtg tagttttggg   3120
atttgggtgt gtggtgttgt gtgggttagg ttttggtttt tgtttgtttg tgtgtgtgtt   3180
attttggttt ggtgaaagtt attgtagaga aaggattgtt aggtgttgag tgtttattta   3240
ttttaagtta gtttttatta gtttttgggg gttagttgtg ggtttt                  3286
```

<210> 277
<211> 23717
<212> DNA
<213> Artificial Sequence

<220>
<223> chemically treated genomic DNA (Homo sapiens)

<400> 277

```
tttttgttgt ggtattttag tatgatggag gtttattttt ggttttttttg gagtatgaat      60
attttttgag gagtattggt tttggttttg taggttttgt tgagattggt ttagattttt     120
tggtttagga gtaattgtta tttaattttt tatttgtaaa ttttgttata gaattggttt     180
ataattgttt ttttggaatt ttggtggatt agttttgttt ttgtgttttt tttttttgta     240
gagttattgt tttttttaggt tttttaagtg tatttaagag tgggagtttg gttgttttta     300
gtatttgtgt tttgttttgt agtttgataa gtttgatgtt aaggtatttt tttgagagtt     360
ggtttagtag ttgttggtag gagagtggtt ttagggtagt gggaagggga tttttgggga     420
gagaatgagg tttgtgtaag ggttttggaa ggatttagga gttgttttgg tttttttttt     480
ttaggttttg gttgggtgtt gtgggttgtg ttttagttga gtttggtgtg gttgtttaat     540
ggtgtttttt tgtgattgag gatgaagggg tttagagagg ttgtgtttaa gtgatgttgt     600
gaggttaggt tgtgttttgg gttagggtgg tttttttgtt attgttgttt ttggttgtta     660
ggattaagta tagtatgtag tgttgtgaaa ggggtggttt tggaaggtgt gaaggggttt     720
ttgggttttg tgaatatggt ggggttgggg tggagttgtg ggtttttggt tgagtggttg     780
tttagagttt tgtgggatat ggatggtttt agtttggttt tttgtgagtt ttatgtgttt     840
gtgatgttgt tgatgtgtag atgttgtggt tgtaggtttt gtttttttgt gtttttttgta     900
aggtttttttg ggagttgtag ttttagtttt tttttgggtt gttttatttt ttgataattt     960
tggagtgtgg tgttttttgg ggattgaggt tttgggtttg gttgttttta gtagtgaggg    1020

taggagaagt ggtttgggtg gttggtggat tgggagggtg ttatttgagg attataattt    1080
ttggtttgtt ttgtgtaggt attgttttga gtttttgttt tgttggtgtt gtgtagtttt    1140
gtgggagttg tagtttgggt gtgtgattta tagtgttgtg tgtgttttgg gtgtgttgta    1200
gttgtggggt ttgggttggg tgttatgtta tgaaggtagt gtaaggtggg tgttggatgt    1260
gggtaggggt tggggttgga gttgggttgg ggtgttttgt ggttgaggag tgggaggttg    1320
tttgggtagt gtggttggtg gtgagagggg ttttgttgtt ttttggaggt agaagttgtg    1380
gattggttgg tgggggtgag tgggtttggg ggttggggtt gtgttgtttg ggttgtgagg    1440
atgaggtggt gttgttgggt tgtggaggtg agttttgttg gttgggtttg gttgggtgtt    1500
tagttgggga ttttgggtgg ggagtgtggg tttggagggt ggtggggagt gtgggtttgg    1560
tggggtgtag gaggttttgg gggttgggtg tgttgggttt tgagttaggg taggatggt     1620
agggtttttg gagtggggtt agtttgttgt ttgttggtgt ttttaggttg gtgggggtta    1680
gggttgggtg atgttttgta gaggaattta gaggaaggtg ggaagttggt agggatttgg    1740
gttatgtggg ttatttgagg ttagttttgg gggttgtgta tattggaggt ggttttggtt    1800
ttgtttgttt ttgagtgttt ttatatttgg gtttattgtg tggttttggt tgttttaggg    1860
atgttgtgtt tggtgtgggt attatttttt ttatttgggg aatggtttgg gggtggttat    1920
ttggtgtgga ggggttggtg ggagttttgt ttgtgttagt tatgttagtg tttttttttg    1980
tgtttttttg gggttgttgt ggtattagtt gggaagttgt aaatttgttg tttttttgtt    2040
ggttgtagaa ggggtttttt tagttttgat tgggggaatt tttgtgtggt tagttggttt    2100
tggttgggtg agtttttggg gtttttttat tgttttggag tttgttattg gattgtaggt    2160
aataagattt ggaaagtaat ggtgaggttt agaatttgag gttaggtttg ggtgtgtggg    2220
gttagtgggg aagattggga ttttgttggg tgttgaagag ttgtgtgttt tttttttggg    2280
aaatttggat tttttttttt gtttaggttt gaggggtgtg gaaatgtgag ttatagttta    2340
gtgtgggagt gttttgtaat tgtttttgtg tttttagagt ttggggttgg gtaaggatat    2400
aggggtgatt tttttttttg gttttgagtg attttttaat tgtgaagtgg ggtaagagaa    2460
attgtttttt tggtttgttt gggggaggta ggttttgtag gaaattagtt tggtttggtt    2520
ttgggttggg tgtgttttgt ttttttttgag gttttttttgt ttgtgggagg agtatttttt    2580
ttttttttgt ttttgtttgt gtttgaggtt ttttagagtt ttttgattgg tagaggagtt    2640
agtgattttt gagttttttag gagagtttga ggtttagtaa ttgtataatt gtgtagggtt    2700
ttttgtttgg gtttttatag attgtggttt ttgagagtag gaagtggttg gtttttttgta    2760
atgttgtatt tgtgtgatag ttttaagttt tgttggtttt ttttttgggt atttttttatt    2820
tgtttttttgt tttggttttg ggtagtattt tggtaatttt agtgttatag gtgaggaatt    2880
gaagttggag atagttgttt atggtagggg aggggttgag ggaggttgag tgtttggttt    2940
ttttttttttg ttggttttat tagtttatat gaggtaattg gagggaagga ttttgttgtt    3000
tggaaaattt tttggaatta taggtgttgt ttatgggttt gagttgttta gtgaagaatt    3060
tggttgttgg tggttgagtt gtttttggtt tagttttttg gtttgtggtg tgatgttttt    3120
atttatggta ataggtgtgg ataggtgttg agaaggagga tgatattatt aaataaaata    3180
gtttggggtt tttgaaaaat agtgatagaa atttaggaag gttggtgatt tgttaggttt    3240
gtgtttgttt tgggtggtta tttggttttg gattgtgagg ttgaaatgat aggagatatg    3300
gagggtgggt ttgggtggtt tttttataag atatagaaga aggaggtttt taatttgggg    3360
gttgttttgt gtgttttgtt tttttgtttt tttgttttgt ttttttggtt ttgttttttg    3420
atttttggta tagtaggagt gtttagaagt ttagtgggat ttgtttttttg tttaaagttt    3480
ttttgtattt tttgtattta tttggtgtgg atgtgggttt ttttattatt aggttgtgtt    3540
```

```
ttagttgtgt ggagttattg gttttttatg ttttatgttg gagagaatgt tgaggtttgt    3600
gtggttttta tgtgattttg tagttggttt ttggtttttt attgtgggat ttgtttgtag    3660
tatttatatt tgggattgtt ttgttttttt gttggatgtg gtgttgaggg tagggtttgt    3720
ttttgttttt tggtaggttt agtgtttagt gttatgtagt tgtgtgtaga gtaggtggtt    3780
agtgagtatt tggggttggt ttttattata ggtagttttg aatatttggg ttttggtagt    3840
ttagggggat ttttgtgtgt tttaggaaag tagtatagta gtttttgtgg tgtggatatg    3900
gttatatttg agtttggttt gtttttgagt ttgagagaat aggtttgttg gtttgtgatt    3960
tttgtatgtg tgttttgttg tagggaggtt gtgggttggg gttggattta gatttgttta    4020
gttatatttt gttattatgt gggttatata tagttgggtg gagatttttt ggtgtgtggt    4080
ttagggaggt gtttgggtgt ggagtttgtg gatagttata ttgtattatt aggatagggg    4140
ttgttgttgg gtttataggg tgtatggaat ggggtttgtg ggaggatggg ggtattttag    4200
tttagggtag ggattttgtg ggttttttaa gatgagttgt gttaggttgt tgttttgtat    4260
attttgaggg gtttgtttga gtgttgtatg ggaagttagt agtgtggttg tttttttttag   4320
aatttattta gggtattagg gtggatttat gagttttgtt gggttatggg tattagtttt    4380
gttattttgg tttttagata taatgatttg ttagattttt tgaggttaaa gggaagtttt    4440
aagggttgtt atttttgttt ttttggtggt tttagatggg atgttaggag gatgtagggt    4500
tattgttgtt ttttaaatgt tatagtgatt gtagtgtttg ttttttttgtg gaaggagttt   4560
gttttgttaa tgagggagtt tttttttatg gttttttttt tattttttttt taaaaagttg   4620
ttttttattt taatattttt taagttttat ttttttgagt tagggttgtg ttttgttgtt    4680
tatgttggag tgtagttgta attatagttt gttgtagttt taaattttttg ggtttaagtg   4740
atttttttgt tttagttttt taaatattgg gattataggt gggtgttatt atatttagtt    4800
tttgtttttt gtaagtgaag tttttttaggt aggttgaatt ttttatattt tttgtttttg   4860
tttgattttt atttttttatt ttttattttt ttgtttttttg taatgagaaa ggtaataagt  4920
tttggagttt tttggagttt ttgttttttt tgaaaaggtg gttgtagaga ggttggagtt    4980
gatggggaaa gaggtgttat gaggtttttt ttgggttagg gttatatggt ttttaaggtt    5040
tttgtttttt tttttggggt gataggtgtt tttagggtta tatggttttt aaggttttttg   5100
tttttttttt tggggtgata ggtgttttta gggttatatg gtttttaagg tttttgtttt    5160
ttttttttggg atgataggtg tttagggtta tatggttttt aaggtttttg tttttttttt   5220
tggggtgata ggtgtttttt gtgttttaga ggtttttgtt tggttattag tgaggttatt    5280
gggttgttta gattagggtt gtttttgtta ttatatggta agtagagaag tagagaagtg    5340
ttaaaaaatta tgagtgtttg tttggttttt ttttttttgtt aaaaatagaa gttggggttg  5400
ggtgtggtgg ttgatatttg taattttagt ttttttagag gttgaggtag gtgggttgtt    5460
tgaggttagg agtttgagat tagtttgatt aaaatggtga aattttattt ttattaaaaa    5520
taaaaaaatt agttgggtgt ggtggtgtgt gtttgtaatt ttagatattt aggaggttga    5580
gattgtagaa ttgtttgaat ttgggaggtg gaggttgtag ttagttggga tggtattatt    5640
gtattttagt ttaggtgata gagttatatt ttgttttaaa aaaaaaaaaa agaaaaaaaa    5700
gtagaatttg gttgggttta gtggtttatg tgtgtaattt tagtattttg gaaggttaag    5760
gtgggtgaat tatatgaggt taggaggtag agattagtat ggttaatgtg atgaaatttt    5820
tatttttatt aaaaatataa aaattagttg ggtgtggtgg tgtgtgtttg taattttaga    5880
tgtttgggag gttgagatat tagaattgtt tgaatttggg aggtagaggt tgtagtgagt    5940
tgagatggta ttattgtatt ttagtttagg tgatagagtg atattttgtt ttaaaaaaaa    6000
aaaaaaagtt agttgttgtg gtttatgttt gtaattttag tattttggga ggttaaggtg    6060
ggtggattat ttgaggttag gagattgaga ttattttggt taatatggtg aaattttatt    6120
tttgttaaaa atataaaaaa attagttggg tttggtggta ggtgtttgta gtttagtta     6180
tttgggaggt tgaggtagga gaatggtgtg aatttaggag gtggagtttg tagtgagttg    6240
agattatatt attgtatttt agtttgggta atagagtgag attttgttag agaaaaaaaa    6300
aaaaaaaaata ggtgatgttt gtgttattgg atatatttgt atattatttt tatgttgatt   6360
ataaaagagg gtgaggggtt gggtatggtg gtttatgttt ggaattttag tattgggga     6420
ggttgaggtg ggtggattat ttgaggtttg gagtttgaga ttagtttgat taatatggtg    6480
aaattttgtt tttattaaaa atataaaaat tagttgggta tggtggtggg tgtttgtaat    6540
ttgagttatt tgggaggttg aggtaggaga atagtttgaa tttgggaggt ggaggttgta    6600
gtgagttgag attgtattat tgtattttag tttgggtgat agagtgaggt tttattttaa    6660
aaaataaaat aaaaaattta aaattaataa aaaagagggt gatggttgag tgaggagttt    6720
atggtgggtt ttatataggg tttaggaagg atagggtggg aagtgtgagt attttgttta    6780
ttgtagtggg tttgttatgg agagtatgat tttagagaag taaagttgtt tggtttgaag    6840
tagtggttgt tttgttgata gttgtagtgg ttgggtggta tttgggttgg ggtttggggg    6900
agtttaggtg gttatttggt tgtgtgtttg tgtttgttta agttgggtat ttttttttgg    6960
tagtgtttgg gaagtttggt tgttttttttt ggttggtgtt gttttgtagt tttgtttatg   7020
gatttttgtt tatggtttgt tttttgtagt agggttgggt tgtgggaggt ttggatagga    7080
ttttgaatgg ttttgtttgt ggttttggtt ggttggaggt tgattgtatt ggataggtta    7140
agggttttaa attagggtag tgaggagttt gttgagtttt tgttgttttt tgggttgttt    7200
```

```
ttggtgtttg gatagatatt taaggataga agtttggata gtttttaagg tgaagggttt    7260
tttggggttg attttttggg attttggttt ttgtagagag ggttggaggt ggggttttgt    7320
gttttagggt tagagttttt ttggtggaag ttgaggtgtt tgattaaatg tttttttttt    7380
taggtagtag tttagatgtg gtatagagag ggtttgggag tttgttgtag ttttaggtga    7440
gtttgttttt aagttttgtg gtttgtttat gggtttgttt gtgttgtaga attgtttgtg    7500
gagtagtagg gttggagtgt ttatggttgg ggggtttttt ggagttttttg atttatagaa   7560
tttttggagg ttggtagttt tgtttgtttt gaagagtttt gtttttttag ttgtttgggt    7620
tgtggttttg ttgtgggagg tgatggtgag ggtggtttgg ggttttgata ggggtgtttg    7680
tttttttggg gatttttttt gtgttgtggt tttggttttt agatggagtt ttttattttt    7740
atggtagtgt tggggttggg gttggtgagg agtttgtgta tggtttttat tgttattttt    7800
agttaggagg gtgtttggtt tttggggagt ggtttgtatt tgtttttgga gaatgttgtt    7860
ttgttagttg ttatttgggg tgggtggttg gagtttatgt ttttttttag ggtggggttt    7920
tggttttgtt ttttgtaggg atgatgggaa gttttgtttt tagtttttttt atttgattta   7980
tgtaggagtt ttttgttttt ggggttattt tttttatagg ggttttagtg ttagtgggga    8040
taaaggtaga gttgtttgtt tttaggttgt gggtattgat tgagttttgt tttggtagtg    8100
gttggttttt gtttttttgtt tggattagta gtttttggtt tggtgggtgt agttttaggg   8160
tgtagtgggg ttgttgtggg gtaaggttag gttattagtt gtttggatat aggggttttt    8220
gtttttttatt tgttgttttt tatattttta gtttgggtag gggtgatatt ggggtatttt   8280
tgttggggtt gttgtggttt ttatggtggt aagatggttt tgtgaaagat tgatggagat    8340
gtagggttta ggagtaggtg ttgtgtgagt gggatatgtt tgtgttattt gtggggtagg    8400
ggagggtttg ggggtatagt tgtgtatatg attagttagt gagatagtga ggagtgtatg    8460
tggagtggat atagttgtgg gtgttgtttg tgtaaggttg ggggttgttg tgttagtttt    8520
gtttttatgt gggtttttag ttttttaggt agggatgttg attttgggag gtgtttttgt    8580
ttgtatttat tggagtttta ggagtatgtg ttttgttttt atgggttatt tttattgagt    8640
ttattattta ttgagaggta ttttgagatt ttgttttgtt atgggtgggt tgttaaggtt    8700
agtattagag atttttagta gattttagtg gttgtagatg gagtggggtg gtaatggtta    8760
tttttgggat ttagttttgt gttgagtttt gggtagtgtg attattttgg ttttttttgt    8820
gtatgttttt tggttggatg tttttttgttg tttttatggg gtgtgtgtgt ggtatatagg   8880
atagggattg gttagttggt tttgtttatt aattatttgt ttttataggg tagtggtggt    8940
tttatttttg taatttttttg aggttggatt taggttattg ttttgtttaa aattagggta   9000
ttggttttta gggagggtgg ggagttgtat agttggattt gtgggggtta ggtatggatt    9060
tatatagttt ggggtttttt gtattttgtt tttttaggga gtttagaggt gggtgtggtt    9120
gtagtttggt tttgtggtag ttatgagtta tggttatttt tgaggtgttg ttttgagagt    9180
tagttttgtg gttatggttt ttgttggttt ttatttgtga gtatttgaaa tagtttggtt    9240
tatagtagag gtttgggggg tatatgtagt ttatgggagt agtatagatg tttggttggt    9300
ggtaggtttt ataggagtag tagtggttta gggtatgttt ttggggttag gggttagagt    9360
ttggtatgtt ttgtgggggt tttattttgt tgttttttgg tttggtttgg gtatattttt    9420
tggtgatagt gtttttgtat gtttttttttt ttttttggtt atttagatag ggagttagga   9480
tgggaattat ggattgattt gatttgtgtt tttggggtta ttgtattggg tgatttatgt    9540
gtgtttaggt tttgaggtgg gttggatttg ggagtggtag gtgagtgttt tttttaagag    9600
tttttggttg gttgtagtag gttttgtttt tgttgttttt agtggttttg gatatgataa    9660
tttttttgtt gaggtttggt tggagttagt atttatggtt gagatgtagt tagttttggt    9720
tgggtgttgt ttttgggggt ggttgttggt gttgtttttt ttttttttta ttatttttttg   9780
tttttttttt ttggggtttt tttattggag tttttttttt aaatgtagtt tttttttttt    9840
aggggaatgt gggttgggag tttatttttt ttttttttga gatggagttt tgttttgttg    9900
ttttggttgg agtgtagtgg tgtggttttg gtttattgta atttttgttt tttggattta    9960
ggttattttt ttgttttagt tttttaagta gttgggatta taggtgtttg ttattatgtt   10020
tagttaattt tttgtgtgtt tttagtagaa atagggtttt tttattttgt gtaggttagt   10080
tttgaatttt tgattttgtg atttatttat tttggttttt tatagtgttg ggattatagg   10140
tgtgagttat tgtatttggt ttgggagttt tttttgtagt tgtttatttt gtattagtat   10200
ttgtttatta tgagtggatg gaatataggg ggatttagga tttatagttg tatttgggaa   10260
ggttagtagg gtggattttg ggaagagtat tggtgttagt tttttatagt tatttttgtg   10320
atgggatttt taggttggta tgtggttggt agttgtgttt tgggagtttt tgtttttttag  10380
tgggttttgt tagttagggt agggtttttg gtattttttta tggggttttt gatttttttg  10440
gtagtggatt ggggtggttt agtgatatta tttttggaga tttttttggtg tgtgttggaa  10500
aaattttgga gagggtttaa tagagttagt tgtttagttt ttgtgatagg gatgtttgtt   10560
tttttttgta gggttttttt tttttttttg agtatttatg ggaggggatt gtgttgtttt   10620
tgtttttttta ggataggttt ttttttttgtt tagatggggt ttatagggtt agttgtttga 10680
gattggtttt agtttaagaa aggttttttg agtgagtgta ggggagtagg aggttggtga   10740
gtagtttgtt ttttgttttg ggtgtagttg ggtattattt ttgttttggg tttaaaggta   10800
ttagtgagga gtaggtgggg tttggtagga gtttgtgttt ttagtggtat gtgtgatttt   10860
```

```
aggtgttatg tgggtgagtg tgtgggtggg tgagtggtga aggagttgag gttggttttg   10920
gtaggtgtgt agtttttata tagaatgggt agggtttttg ttagagttaa attagtttag   10980
ggtttagtgt aagtagtttt tgttttgtgg tatttttgtt agttgttgtt tttatttaag   11040
ttttgttttg ttttgatttg tttagggttt ggttatagaa ggggagtgtt ggaaatgttt   11100
aatttgtttt ttgtgaggga tttttgtgtg gtttgggttg gggaatgtag gggttgtttg   11160
ggtggttttg gtgtttggtg tagttatgtg agaggttttg ggttgatgtg tgggtttttt   11220
gttagtgagt ttgttttgtg tgtgttgggt ttttggtttt gtttttgtgt ggtatttgag   11280
attttgtgga ttttgtttgt gttttgtatg tttgagtgtt gttttttgtg gaggttgtta   11340
gtattggtgg ttgtagggtt tgtagtgtgg tatgtttaga gatttggttt ggagaatggg   11400
ttttggatgt ttttgttatg ttttatgttt gttttatatg gatgtagtga tgttttagat   11460
attttggatt atatagagta tgttgttaag gtttgttttg tttgtttttt tttatttgtt   11520
ttatgtggat attaggagat ttgttatgat ttttgtggtt ttgtttatat tgtgtggtgt   11580
tttttggtgt ggttgttagg tgttttttg agggtaggtg tgagtatagg gtggtttttg    11640
tggagaagtg tttgtaggtg agtgagttag gttgtgtttt gttgattttg aggtttgaat   11700
tttatggaat ttttattttt tatagagtat tgtttttttt tttgattttt ttttaattat   11760
ttaaaaatgt aaaagttatt gttgatttgt gggttggatt aggtttaggg ttgggttttg   11820
gtttgtattt tatattggtt ttgtgtgttt tgtgattgtt ttgtttatgg ggtaggtttg   11880
agttattata ggatggtagg gtttttaggg tgtagggttt gtaatgtttt aattttttta   11940
tgattttagt atttttagag aaaatggatt tttgtgttga gtgaagtggt ttaggtgaat   12000
gttattatta tatgggggat ttggtggttt ttgttttagg ttttaaagga gaggggattt   12060
agaatttttt gttttatttt tttagttttt gtttttgaag tttttgtttt taaggttgtg   12120
gggaggaatt agggaagggt tggtgtgttt gttgtgtgat tttaggtgtt tgtgttttgt   12180
tgagtttagt ttttgtggtt ttagagttgt aggttttttt tgggtgttag agtattgatt   12240
ggaaaggttt tttatatttt tgtttttttgt tgtttttttat tgttgtgggg gagaagttgg   12300
aattgtattt ttattttttt tagtttgttt atttagggtt gtttttaggt tttttttgagt   12360
tttaaaggat atgtttttttg gtgtttttttt ttttttttggt tttgtttttt aggttggaat   12420
gtagttgtgg tgtttatgtt attttttat gttagttttt tgagtagtta ggtttatagg   12480
tgtatattat tatatttgga tagtgtttag ttttttataa agatagggtt ttattgtgtt   12540
gtttaggttg gttttaaatt tttgggttta ggtgattttt tatgtatggt agttttgata   12600
tttatttgat tttttttttt taagttttat tttgatgttg gttgtgtgtt ttagagattt   12660
aattagaagt tggaagaagg gttgtgatgt gtgtgatagt aggaattgtt ggttaattta   12720
tagttggtta gtttgttggg atgttatttt ggttttaatt ttggtaggga gaggggagtg   12780
tattggattt tggggagagt agttgtttgt atgggtaggg tttttttgtt ttaggattaa   12840
tgtttttaat tgtatttgat ttttttttag ttagaggggg taaggtggtt tttttagaag   12900
tataggtggg ttgtgtgtta gaggttttgg atagtttgtg tttttttttag gtttttttagt   12960
aggggtggtt tttgtttttt tggaggggag atttaggaa ttggtttttg ttttttagtta   13020
tagttttata gggtttttgt tttttggggt ttggttttgt gggtgtataa ggtatggttg   13080
ggtttttgtt tttttggaag tttttagagt ggtatgtggg tagttgtgtg aagtggtttt   13140
ttagattttt atttagtttg gtttgtagta gttttttatg ttttttgtta tttttttttgt   13200
ttatttttgg gtagatgttt ttttgatttt tttttttgtg ataaagtaaa agtgtgtgat   13260
tttttttttt attaaggaat aaaatgtttt tgaatattat agatgtgatg ttgggttttt   13320
gggggttggg gggtgtttta gggttttgag ggaggagttt tgtgggtgtt gaagttgggt   13380
tgtaggtttt gtattgagat gttttttttag tttattttag gtggttgggt atgttgggaa   13440
gttttttttgg gttgtgtgtg tgaattatgt tgtttttttt ttattttttt tttttgtttg   13500
tttatttttg ttgtgtgtta ttttgtttag ttggttgttt tgtttttttt ttttatgttt   13560
tgttttgttt gttgttggtt agaggtgatt aggtttggtt tttagtatgg ttgttggttg   13620
gggggttatg gggagttttg tttttttttt ggttgtagag attgagttgg attatagttt   13680
ttttgggga ttgttttatt tgggtttttt ggtagtggtt ggttggtgta ggagtggttt   13740
aggggggttt gtttggggag gtgttttggt tagttggtag tattagtttt tatggttttg   13800
ggtttgaagg taagaggtgg ggaggggtgg ttttggaggg tttttggggg tagagggttt   13860
tgtttttagg gtgttttttt tttggtagag gtgtttgtag agaagtgggt ttttgttttt   13920
tttagttgtg gtagtttttt tttttagggt gtaggagttg agtaggtttg agattttggt   13980
tttggtttgg ttggttggtg gtggggatgg gggttagtgt gtttaatttt ggtggttttg   14040
tgttttttat aggttgtgtt gttttatgta gagaagttgt ttttgaagtt ttttttttggg   14100
ttggggttgt tggtggttgt aggttttggg tatattgttt gttgtttatg gttttaaagt   14160
tgttagtatt tgttgggttt tgtttgttgt ttttttgggg gttataggtt atatggtttg   14220
gtggttgaat tttatgtgtt ggttttttat agttgttatt atggttttgg ttatttgttg   14280
tgttttttag gagttgttat taggttttga ggtgtttggt ttggaggtag tttaggtggt   14340
gaggtttgga ttggttggat tttattgatg tttttgttgt tattttgttt tggtttttat   14400
tggaggtagg gatatttagg ggtttggggt tttggggtta gtgttgtagt ggttgttgtt   14460
gtttttatag tgggtttagg tttggttttt gaaggtaatg tgtttgtgtt ttatatttaa   14520
```

aagggtttgt taggttgtat ttggtggtag tttttatggg ggatgatgtt ttgttgtttg 14580
ttggttttta ggtgggaggg atgggtgtgg agttgtaggt agtgggtaag gttatggtag 14640
gtggttaggt ttgggaatta gtttttaggt ggtatttatg ttgaggtttg agttttggtt 14700
gataaggatt ggggatgtag gggattagtt aggggttgtg tgggggagtt ttggagtttt 14760
gtttgtgagt tattttgatg aatagggaga tgtggtagag gtagggttgg ggaggggttg 14820
tgtgggggag ttttggagtt ttgtttgtga gttattttga tggatagaga gatgtggtaa 14880
aggtagggtt ggggaggggt tgtgtggggt ttggtggttg gtagaggaag ggtttgtgtt 14940
ttttttgtgt gtggtggtat tggagtgttt tttagttttt ggtgttattg tattagttgt 15000
tttttagtga gtatttttat gtgtgttgat aatagttttg ttgaggtaga atttattaga 15060
tttatttgtt aaaagagtgt agtttggtta tttttagtgg tttgtagagt tgtgtagttg 15120
ttattgtaat ttagtgttgt agtattttat agtttatagg gatttttagt tttatagttt 15180
agtgttatag tattttgtag tttagaggga tttttagttt tagatggtta ttgattggtt 15240
ttttgttttg gtttgttttg tgtagaaggt gtgtggatgt ggggttatag gatgtggttg 15300
gttgggtttg ttttgtgtag aaggtgtgtg gatgtggggt tataggatgt ggttggttgg 15360
gtttgttttg tgtagaaggt gtgtggatgt ggggttatag gatgtggttg gttgggtttg 15420
ttttgtgtag aaggtgtgtg gatgtggggt tataggatgt ggttggttgg gtttgttttg 15480
tgtagaaggt gtgtggatgt ggggttatag gatgtggggt tttgtgtttg gttttttttat 15540
ttggtattgt gttttttaga tttatgtatt tgtagtttga ggtagtttta tttattgttt 15600
ttttgtggga tgaattgtat tttatttatt tatggatgtt aggattgttt ttatgtggtt 15660
tttgtggata atgttgtgaa tattgtttat gaggttttgt gtggatttgt ttttatttat 15720
tttgtataga tatttaggag tgggattgtt ggggtgtgtg gtgaattttt ataatgtttt 15780
gaggaattgt taagttattt tttgtagtgg ttgtattatt tagtttttat tggtaatgtt 15840
ggggagggtt ttggtgtttt tatttttttg atagttttttg ttgttgtttg tttttgtgat 15900
tttggttgtt ttagggggtg ggagttggtg tttttttggt tttgatttgt gtttttttgg 15960
tggttagtta tgtggggttt ttttttttgt tttatttatt tgtgtttgtt ttttttttat 16020
ttatttattt atttatttat ttagagatag ggttttattt tgttatttag gttggagtat 16080
agtggtgtga ttatagttta ttgtagtttt tattttttgg gtttaagaga atttttttatt 16140
ttagtttttt aagtagttgg aattataggt gtgtattatt atagttggtt aattagaaaa 16200
aaaaaatttg gttaggtgta gtagtttatg tttgtaattt tagtattttg ggaggttgag 16260
gtgggtagat tatttgaggt taggagttta agattagttt ggttaatatg gtgaaatttt 16320
atttttatta aaataaaaat tagttgggtg tggtgatggg tatttataat tttagttatt 16380

agggaggttg aagtaggaga attgtttgaa tttgggaggt ggaggttgta gtgagttaag 16440
attgtattat tgtattttag tgtgggtgat agagtgagat tttgttttaa aataaaaata 16500
taaattttga gtttgggagg gtttaggttg taatgagttg tgattgtgtt attgtatttt 16560
agtttgggtg atatagtgag atgttagatg gtgttttaaa aaatttaaat agaaaaatgt 16620
agaaaggatt gttgtttttt tttttgtttt ttagagggta gaggattttt ggtttttgtt 16680
ttttgagtat taggttgagt tttagatttt gggatggttt ttttagtttg tagtttttgg 16740
tagttttggg ttatattttt gggattttta gggattggtt tgggattatt gggggtggtg 16800
gttgtggttt tggttatggg gagggaggta gagttgtggg gtaggtgtgg ggtttgttgt 16860
gttgggtatg tggggtgtgt gttagatttt ggatttggtt tgtggggtta ggttgtggtg 16920
tatgatgatg ttatgttgtt tttttttttt tattttggtt ttttgtttgg tttttttttg 16980
ggttggtatt tttggggtta tttttgtttg tgtttgttgt tgtggggttt gggttttagg 17040
tagtttttgt ttgttgttgt aaataggttt ttaaggtgta tggtgaggag atttttaggt 17100
tagagatggg tgagagtggt gggtgagtag attgtgggaa ggtagttggg tttttatatg 17160
tggagttttt tttattatgt gtatgtatag gtgatttttg ttgtgttttg atggagggtg 17220
ttgggattgg ggagggttag tgtgatagtt ttggtgttta ttgaagagtt gtttgtttgg 17280
tttgttgttt tggtggttgt tgaatttttt taatgtttta ggatagtagg tgttgtggtt 17340
ttatgattgt ttttttatat ggatgaaggg gtggtgtata ggttggtgga gtttgggaga 17400
gttagggtag tttgtggtta tgtggttatg tggttggtgt ggggttgagg tttttgtata 17460
gttgatagag tgtttggtgt ttttaattag taagttattt atgggttggg tattggtggt 17520
ttagtataga gaggtaataa gggtagattt tttttggtaa ggttgattgg tgttgggttg 17580
tggttagatt aggagattgt agtttagttt gagggaggta ttatgtttgt tttttagagt 17640
attgagtaga gtttttttatg gtattttttg ttttgggtat agtaggagga gttgttgatt 17700
aagtttggta ttttggtgtg ttttgagagt tgtttgggta taggttgtgg ggttgttttg 17760
gttggatggg gtatttgtgt tttttggtgt ggttttaggg ttttggtggt tttgattgag 17820
gttttggggg atagaatgta gttttttgtt ttttattttt tttaagaaag gggttgtttt 17880
ttaggattgg ggttgtgtta gttataggtt tgtggttttt atgttttggg tgttgttgtt 17940
ttggttgttt tagggtgggt tttgttttgt atgtagtgag tggttttatt attttttttt 18000
tgaattggag ttgagtgttg gtaatgttta gataattggg gtgtgtgggt tttttgtgtt 18060
ggattttttt gtatagttgt tttgattttt tatttagttt ggtttttgtg tagtaaggag 18120

tggtttgttg ttttttttat gttgtttttt ttatgttgtt tttttatgt tgtttttttt 18180
atgttgttgt ttttttatg ttgttttttt tatgttgttt tttttatgtt gttgtttttt 18240
ttatgttgtt ttttttatgt tgtttttttt atgttgtttt ttttatgttg ttttttttat 18300
gttgtttttt tttatgttgt tgtttttttt tatgttgttg ttttttttta tgttgtttgt 18360
tttgagtttg tttttttga aggtaattat tttgtagtag tgagggtttt ttgttgagta 18420
tgtgtggttg tgattttagg agtgttttgg gttgagaatt ttaaaagggt tttgtttagg 18480
tttggttttg ggtttttggg tttttgatgt gagtttttat ttttaggttt tggtggtttg 18540
gttttaggag gttaaggttg tagtgagttg tgtgtgagtt attgtattat tttgggtaat 18600
tagattttat ttttaaagtt taaaataata tttttgtaag ggttttgggt ttttgagttt 18660
atgaatttgt ttaggttttg tagaggtgag ggtggtttta gagtggttgg ggggttgtgg 18720
agattagaat tagggtagaa gggtagggag ggtgggggtt ttgttgaggt tttttattgt 18780
tgatttttga tttttggttt tttttattt tggtttattg agagaggttg gtatagtgta 18840
gattgtgggg ttagtgatat ttttttagt gtttttagtt atagaagggt tgtggtttgt 18900
ttttgttttt tttatttgtg ttttggggtt atgtttgaat tttaggtttt gttttgattt 18960
tattgaaatt tttttgaagt gtagatgttt ttttgattta tttgggtttt gttaggatga 19020
ttttgaaagg tgttttttag tagatatatt ttgttggtga ttgtttggtt ttggggattt 19080
gggtttttat tttttttttt ggagttggtg aggttgtttt tgttagttgg tgataattat 19140
agggttaaag ggagggtgtg ttttaggagg tttagtttat ttttgtttgt atttgtggtt 19200
agtggggtgt agagtttgtg aaggtatttt ttggagtggg ggttgtaaag attatggtta 19260
ttgtgttatt tatagggtt taggttggat gtttgggttg tgttgttttg gtagttaggt 19320
tgaggaatgt gtaggtgtgg ttgggagttt gttggttata gttgtattta tgtttttttt 19380
tgagtggtga tggtggggag aggtttggtt gtttatgggt agattgtttg ggtttttata 19440
gaagtttgtt gttagagtag atttttggtt ttgggttttt ttgttttagt tggatgtttt 19500
gagttttgtt gggtttagtt tattttttgtt tattttgtta ggggtagttt agagttttg 19560
ttatgtgggg ttgagtgtag ggaaggtagt attatggagt ttgtttggtt ttatagaata 19620
tttgtgttgt ttgtggagat tttttgtttt tgtgtttttt atgaagaggt aggtttttttt 19680
ttttttgaa ttaaaagggt ttagatggag tataagattt aggttatatt tataagaata 19740
attgattttt ttgttaggta gagtagttag tttgttttgg aggttgtgtt gtggtgtggg 19800
gaggtttttt gtttagatta gtttgtgttt tttttaggtt ttggtttatg tatttgtttt 19860
tttgtaggtt tgagtatttg ttttttatg tttaggatga tttggttttg agtttggttg 19920
ggatttttg ttatttgttg tttataggtt tggtgataga gaaagtagtg ttttggaata 19980
gtagtttggt tgtggggtgt ttagtttttt ttgtaggatt ttgtgtggtt taaggggttt 20040
gttgagtttt taatgggtaa gaggggtttt gagttatgga tttattttgg gtggaggtag 20100
tttttattgt tgtttgtttt aaatgtttag ttttaggttt ttggttttgt agttttgtag 20160
ttatagatgg gtgtttttt taggttgaga ggagggtggg gtttagttat ttaggatttt 20220
atagggtggt tttgatgagt atttttttt gtagttatat ttatttgttt gggtttatgg 20280
ttatggttgg agagtggttt tgtgtgtgtt gtattttttt gaatttggtt ggttagtgtt 20340
agtgttttat ttgtgaggtt ttttggtata agtttgattt taattatatt ttgtggttta 20400
gtgtggagga gtagaaatgg ttttgtgttt gttgtatttt ttgtaatttt ttgggtaagg 20460
aggtttgtga ggtgtgtggt tttattttgg agtttgtgtt tggggttgtt tttttgttag 20520
tttttaatgg ggtttttttt aagttatttg ttattttggg ggagtttaag ggtagttgtt 20580
aggaggaagt aggtttagtg aggattgtgg ggttggtggt tatggagttt gttagggggt 20640
agtgtgagga taaggatgag gaggagaagg aggagtagga ggaggaggag ggagtggtgg 20700
agtttagagg gggttgggtg tgtttgtgtt gtatgttgta taatatgttt gtggttagtt 20760
tttgttttgt ttgtgggggt ttatgtaggt ttttgttgtt atggattttt tttgaggttt 20820
tggtggtttt ggaagtggtg gttttggttg gtttttatgt tgtgtttgtt gtgtttttat 20880
ttggtttttt tggggaaggt gttgaggtta attttttagt tattagttag ggtttagttg 20940
ttgaattaga gttgtttagg gttttgtttt ttagtttttt tttgtttatt ttgtagaata 21000
attttgtgtt gtgtagttga tgtgaggttt ttttttagtt gtagttattg gtgtttgagg 21060
ttgtttagtt gttatttttt gttggttgta ggggagtttt ttagggtttg ggttgggttg 21120
gggtttttttg tttagtagag ttgttgtttg gtaagtggtt gagtgtgttg gaggaagagg 21180
ttatggaggg tggtattagt tgtgtagagg ttggtagttt tattttgggt agtgatatta 21240
ttgatttggt tggagatatt gtgtgttata tgtttgttag ttttttagt tttgatttta 21300
ttatttggtt atgtgttaag tgtatgttta gaaattttat agtggttttt aggtgtttgg 21360
tttgtggttg ttttaaattg tatggttttt aggagtatgg tgagtttttt atttattgtt 21420
ttgattgtgg ggttgataag tttagttttt gtggtagaag ttgtggatgg gtgtttttgg 21480
tttagaaggt tgtttgtgtt ttgtttgagt gtttgggtta gtgggtttgt tttgtttgta 21540
ttttgtttaa tgtattgtgg gttaagtatt gtgttgtttg ttatatgttt tagtttttgg 21600
tggtttagtg gtggggggtt gtgtttttga ggtgtaggga gagtatgtat gtggagtagt 21660
ggtggtagat agatgagggt gaggttaagg tattttggga gaatattgtg gttttttgtt 21720
gggaggtgag gtgtttttttt gtttttttttt tgtttttgag ttttttgttt ttgtttttttt 21780

```
tttttttaggt tttgggtttt ggtgatgggt tggggaggag tttataaggt ttaagatgtg   21840
atggggaggg tagtattttt tgttttgagg ttttaagttt ttaatggttt tggggtttag   21900
tttggttttt tgatttttgg tttttgttgg tgattgagat gtatgatttt tatttttttt   21960
aattttttat tgtggttagg agggttttga gttttgtttg atgtgttgta ggaggtgagg   22020
gtttttttagg ggtagtgtgg agattttgtt gaagtgtgag gttgggatgt atttgtgttt   22080
tgttttttag agtttaggat ggtattattt atgggttaat tggggtgtag tttgtatttt   22140
ttttagtagg gttagggtta gagttgtgtt gttatttatt gggggttgtg ttgttttttt   22200
gggtttatat gttttttgtt ttttatttgt tgttagttgg tggtttttttt attagggttg   22260
agggtttttt tgatataggg ttggtgttag gtttgtggtt gtttatgttt tatttgtttt   22320
tttggggttt aggtagtgtt tttgggtgtt tttttgggtg gggttttgtt tttgttaggt   22380
ggagtgaggt tattttgttt agtttgagta tatggttgtg gtttttgtag aataatgtga   22440
gttttgtgga tgatagtttt ttttttgggt ttgagtttgt tggttttttt gtgggtgata   22500
gtgtgtagta gtgtgtgagg tagtggttgt gattttagga gattaattgt tttgtttta   22560
gggattatag ggttatgtgg tttgtgtttt atatattgtg gtttttagat attttgtagg   22620
ggttgttggg gaattgttgg tgaggttttt tttaaggttg gggtggggtg ggtgggtggg   22680
tgattggttg tggtttttgt gaggttattt tgaggttttg tgtaggtttt tgagtgtttt   22740
ggtggtgttg gtggagtggt tggatttggt ggagtgggtg atggttatgt gtagtttgtg   22800
tgtagagggt gtttattagg tgtggttgtg taaggatggt atgtggatta tggtgttggt   22860
ggatgatatg ttgttttgtg atgaggttgg ttgttttttt tttttatagg tggggtggtt   22920
tgtagggtgg gtatgggtgg taggggtagt ttttgatttt agttttgaaa ataaggttgg   22980
ttgttttttt tttttttagg gtgggtagtg tggggggtgg gtgggggtgg tttttgattt   23040
ggtttttttgt aggtgtagtg gaagtagttg tgggtggttt ttattgagaa ggtgttggtt   23100
aagttgtatg gtttttattt tgtgttttag gtgggttgtg ttattgaagg tttggttatg   23160
tttattggtg ttttttgtga gagtttggtg ttgtagttta gttttattaa tttttgtgag   23220
gagtttgttg atattgattt tatttgggtt aaaatgttga gttttaagga ggttgggtaa   23280
gaggaggggt attgtgtggt tagttgtgtt gggaggagag gtgaggtgga gtggtgggag   23340
atgtggggtt ggtggttttt gatttagttt tgtttttatg aggtgttttt ggtgtgggtt   23400
gatttgtgt ggttaggtta tagttttaat tattttttttt tttttgggtg gggttggagt   23460
tggttttttt atgttaggaa ggttatattt tggttaggtt gtggggtgag attgatgttg   23520
ttattttgga gtaggtgtga gtggtgtgtt gagtttgatt tgggttgtgg ggtgagattg   23580
atgttgttat ttgttttggg gtaggtgtga gtggtgtgtt gagtttggtt atattttggt   23640
tagtgggttt gtgttttgat ttttaggggt ttttttggag tgttttggtg tgagtatttt   23700
gaagtttttg gagtttt                                                   23717
```

<210> 278
<211> 23717
<212> DNA
<213> Artificial Sequence

<220>
<223> chemically treated genomic DNA (Homo sapiens)

<400> 278

```
agggttttgg gagttttaag gtgtttgtat tggggtattt tgaggaggtt tttgggggtt      60
gaggtataga tttgttgatt agagtgtggt taaatttgat atgttgttta tgtttgtttt     120
gaggtggatg atggtattag ttttgtttta tggtttaggt taaatttgat atgttgttta     180
tatttgtttt ggggtgatgg tattggtttt gttttatggt ttggttagga tgtggttttt     240
ttggtatgag aaggttagtt ttagttttgt ttagggaggg ggaggtgatt ggggttgtgg     300
tttggttatg tggggttagt ttatgttagg ggtattttgt ggaagtagaa ttgggttagg     360
ggttattagt tttatatttt ttattgtttt gttttgtttt tttttttaat gtggttgatt     420
atgtagtgtt tttttttttta tttagttttt ttagaattta gtattttggt ttagatgagg     480
ttagtgttaa tgggtttttt gtgggggtta gtggagttga gttgtagtgt taggttttta     540
taggggggtgt tggtgagtgt ggttaggttt ttgatggtgt ggtttgtttg gagtgtaaag     600
taggagttgt gtagtttggt tagtgttttt ttgatgaggg ttatttatag ttgtttttgt     660
tgtgtttgta gagggttggg ttagaggtta tttttgtttg ttttttatat tatttgtttt     720
gggagaagag gaagtagttg gttttgtttt tggggttggg gttagaggtt gtttttgttg     780
tttgtgttta ttttgtaggt tgttttattt gtgagaagag gaggtagttg gttttattat     840
agggtagtat gttgtttatt agtattgtgg tttatgtgtt gtttttgtat agttgtattt     900
ggtaggtgtt ttttgtatat aggttgtgtg tgattattat ttgttttatt aggtttggtt     960
gttttgttag tattgttagg gtgtttagga atttgtgtag agttttaggg tgattttgtg    1020
```

gggattgtgg ttggttgttt gtttatttgt tttattttgg ttttggagaa ggttttatta 1080
gtagtttttt agtagttttt gtaggatgtt tgagggttgt agtgtgtgga atatagatta 1140
tgtggttttg tggtttttga agatggagta gttgattttt tggggttgta gttattgttt 1200
tatatgttgt tgtatgttgt tatttgtggg gaagttgata gatttgggtt tgggagggaa 1260
gttgttattt atgaagttta tattgttttg tagagattat ggttatgtgt ttaggttggg 1320
tagggtggtt ttgttttatt tggtagggat aagattttat ttaggagagt gtttaaaagt 1380
attgtttgag ttttaaaggg atgggtggag tgtggatagt tatagatttg gtgttggttt 1440
tgtgttgagg aagtttttag ttttagtgga gaggttattg gttggtgata ggtgaggagt 1500
aggggatgtg tgggtttagg gggatagtgt agtttttagt gggtggtagt atgattttgg 1560
ttttggtttt gttggggagg gtgtaggttg tgttttagtt gatttgtggg tgatgttatt 1620
ttggatttta gagggtggag tatgggtata ttttgatttt gtattttagt ggggttttta 1680
tgttgttttt gggaagtttt tgttttttgt ggtatattag atggggtttg gggttttttt 1740
ggttatggtg gagggttgag gggggtgaag gttgtgtatt ttggttatta gtaaggatta 1800
agggttagga ggttaagttg gattttaggg ttattgggag tttggagttt tggggtggag 1860
ggtgttgttt tttttattat gttttaggtt ttgtgggttt ttttttagtt tattgttaga 1920
gtttaaagtt taggaagggg agggtgggag taggaggttt aggagtagga agagggtgag 1980
ggggtgtttt atttttttggt agaaggttat gatgtttttt tagagtgttt tggttttgtt 2040
tttgtttgtt tgttgttgtt gttttatgtg tatgtttttt ttgtgtttta ggggtgtggt 2100
tttttgttgt tgggttatta ggagttgagg tgtgtggtag gtggtgtagt gtttggtttg 2160
tagtgtgttg agtagggtat aggtagggta ggtttattgg tttgggtgtt tgggtaggat 2220
gtgggtggtt ttttgggttg aggatatttg tttgtagttt ttgttgtagg ggttgggttt 2280
gttggtttta tagttggggt agtgggtggg gggtttgtta tgtttttgga agttgtgtag 2340
tttggagtag ttgtaggttg agtatttggg ggttattgtg gggtttttga gtgtgtattt 2400
ggtatatgat taggtggtga agttggggtt ggaggggttg gtgggtgtgt aatgtatggt 2460
gtttttggtt aggttaatga tgttattgtt tgaggtggag ttgttggttt ttatgtggtt 2520
ggtgttattt tttgtggttt tttttttag tatatttagt tgtttgttag atagtaattt 2580
tgttaggtgg gaggttttag tttagtttga gttttggggg gttttttttgt agttggtaga 2640
gggtgatggt tgggtagttt taggtattgg tggttgtagt tgggggggaa ttttgtgttg 2700
gttgtgtggt atggggttgt tttgtagggt ggatgagaag gggttgaagg gtgggatttt 2760
gggtggtttt ggtttggtag ttgggttttg gttggtggtt ggggggttgg ttttggtatt 2820
ttttttgggg aggttaggtg gaggtgtggt aggtatgatg tggaagttgg ttggggttat 2880
tatttttggg attattaggg ttttaggagg gatttgtggt agtgagagtt tgtgtgggtt 2940
tttgtagatg gagtaggagt tggttatggg tgtgttgtgt agtgtgtaat gtggatatgt 3000
ttagtttttt ttgggttttg ttgttttttt tttttttttt tgtttttttt tttttttttt 3060
gtttttgttt ttgtattgtt ttttggtggg ttttgtggtt attagttttg tagtttttat 3120
tggatttgtt tttttttggt agttgttttt gggttttttt aggatggtgg gtggtttggg 3180
gaggattttg ttgaggattg gtaggaaggt agttttaggt gtaggttttg gggtgaagtt 3240
gtatattttg taggtttttt tgtttaggaa gttgtggaag gtgtagtggg tgtagggtta 3300
tttttgtttt tttatgttga gttgtaggat gtggttgagg ttgggtttgt gttggggagt 3360
tttgtagatg gagtattggt gttggttggt tgggtttagg aaggtgtagt gtatatagga 3420
ttattttttg attgtggtta tagatttggg tgagtgggtg tggttgtaag ggaaggtgtt 3480
tattagggtt attttgtgag gttttgagtg gttggatttt attttttttt tggtttgggg 3540
aaggtatttg tttgtggttg tagagttatg gggttgaggg tttagggttg agtatttgag 3600
gtaggtggtg gtgggggttg tttttattta gaatggattt atggtttagg gttttttttg 3660
tttgttggag gtttagtaga ttttttgagt tatataggat tttatagaga gggttgagta 3720
ttttatagtt gggttgttgt tttagggtat tgtttttttt gttattaggt ttgtggatgg 3780
tagatagtag gggttttggg ttaaatttag gattaggttg ttttgggtgt ggaagaatag 3840
gtgtttagat ttatggaagg gtaggtgtat gggttggggt ttggggaagg tataggttga 3900
tttgagtaag gggttttttt atattatagt atagttttttg agatagattg gttgttttgt 3960
ttggtagaaa aattaattgt ttttgtaaat atggtttgaa ttttgtgttt tatttaaatt 4020
tttttggttt aggaagaaag aaaatttgtt tttttataag aagtataaaa ataaaaagtt 4080
tttgtgagta atatgggtgt tttgtggagt tgagtgaatt ttgtgatgtt gttttttttg 4140
tgtttagttt tgtgtggtga gggttttgag ttgtttttgg tagagtggat gggggtggat 4200
tgggtttggt gaggtttagg gtgtttagtt ggagtagaga gatttaaggt tgggaatttg 4260
ttttaatagt gggtttttgt gaaagtttaa gtaatttgtt tgtagatagt taggtttttt 4320
tttgttatta ttatttggag aagggtatgg atgtagttgt ggttggtggg tttttggttg 4380
tatttgtatg ttttttggtt tggttgttga ggtggtatag tttaggtgtt tggtttgggt 4440
ttttgtgggt ggtatagtgg ttatgatttt tgtagttttt attttgggag gtgtttttat 4500
agattttgta ttttgttgat tgtaggtgta ggtagaggtg ggttgggttt tttgggatat 4560
attttttttt tggttttgta gttgttattg attagtaagg atagttttat tagttttaga 4620
gaggagaatg agaatttggg tttttaggat taaataattg ttagtggggt gtgtttgttg 4680

```
ggagatattt tttggagtta ttttggtaaa atttaggtgg gttaggaaga tgtttgtatt    4740
ttaggagggt tttaatgaag ttagaataag gtttaaagtt taaatgtggt tttgggatat    4800
gggtgaggag ggtgaaggta ggttatggtt tttttgtggt tggagatgtt gaggggagtg    4860
ttattgattt tgtagtttgt gttgtgttgg tttttttttgg tgggttgagg tggaaggggg    4920
ttaggggtta ggggttagtg gtgggagatt ttggtagggt ttttattttt tttgtttttt    4980
tgttttggtt ttggttttta taatttttta gttattttag ggttattttt atttttgtag    5040
ggtttgggta ggtttatggg tttagagatt tgaaattttt ataaagatgt tgttttgaat    5100
tttagagatg gggtttgatt gtttaggatg gtgtagtggt ttgtgtatgg tttattgtag    5160
ttttgatttt ttgaggttag attgttaaag tttggaagta aaagtttgtg ttaggagttt    5220
aaagatttaa agttaggttt gagtgaagtt tttttggaat ttttaattta gaatgttttt    5280
gaagttgtag ttatatgtgt ttagtaagga gtttttatta ttgtaggatg gttatttttg    5340
aagggagtgg gtttagggta gatgatgtga ggggagatga tgatgtgggg ggagatgatg    5400
atgtgagggg aggtgatgtg ggggaggtga tgtgggggag gtgatgtggg ggaggtgatg    5460
tgggggaggt gatgtggggg agatgatgat gtgggggaga tgatgtgggg gaggtgatgt    5520
gggggaggtg atgatgtggg ggaggtgatg tgggggaggt gatgtggggg aggtgatgtg    5580
ggggaggtga tgggttgttt tttattgtat agaggttaag ttggatggaa gattgaggta    5640
attgtataga aaaatttagt ataaaagatt tgtgtgtttt agttatttgg gtgttgttga    5700
tatttagttt taatttaaaa gggaggtgat gaagttgttt gttgtgtgta agatagagtt    5760
tgttttgaag tagttgggat agtagtattt ggagtgtgga ggttgtgggt ttgtgattgg    5820
tgtagtttta gttttgggag gtggtttttt ttttaggaaa agtgaggagt aggaagttgt    5880
attttgtttt ttggggtttt ggttaaagtt attagagttt tgaggttata ttgagaaata    5940
tagatgtttt gtttggttgg ggtagttttg taatttgtgt ttagatagtt tttggggtat    6000
attggggtgt tagatttggt tggtagtttt ttttgttgtg tttagggtgg gggatattat    6060
ggggggtttt gtttagtgtt ttgaggggta ggtgtggtgt tttttttgag ttgggttgta    6120
gtttttttggt ttggttatag tttggtgttg gttagttttg ttaggggggg tttatttttg    6180
ttgttttttt gtgttgagtt gttggtgttt ggtttgtaga tgatttattg attgagggtg    6240
ttaggtattt tgttagttgt atagggattt tagttttatg ttggttgtat ggttgtatgg    6300
ttgtaggttg ttttggtttt tttaggtttt attagtttgt gtattatttt tttatttatg    6360
tgggggagtg gttatggggt tatagtattt gttgttttgg ggtgttggga gaatttagtg    6420
gttattaggg tagtgagtta ggtaggtagt tttttagtaa atgttagggt tgttgtgttg    6480
attttttttta gttttggtgt tttttgttgg agtatggtag gggttgtttg tgtatgtatg    6540
tggtggggag ggttttgtgt gtggaggttt agttgttttt ttgtagtttg tttgtttatt    6600
gtttttgttt atttttagtt tgggggtttt tttattatgt attttggggg tttgtttgta    6660
gtagtagata ggagttgttt ggagtttagg ttttatagta gtgggtatgg gtaggagtgg    6720
ttttagggat gttgatttag gggagggtta ggtaggaggt tagggtgaaa gagaaagagt    6780
ggtgtggtgt tattatatat tatggtttga ttttgtaggt taggtttaaa gtttaatata    6840
tgttttatat atttggtata gtagatttta tgtttgtttt gtggttttgt tttttttttt    6900
atagttagag ttatggttgt tatttttggt agttttaggt tagtttttgg ggattttggg    6960
agtgtggttt gaggttgttg ggagttgtag attggagagg ttattttgag gtttgaaatt    7020
tggtttggtg tttaaaaagt aggggttgag agttttttgt tttttggaaa atgagaaagg    7080
gaatgataat tttttttata ttttttttgtt taagtttttt gagatattgt ttagtgtttt    7140
attgtgttgt ttaagttgga gtgtggtggt atgattgtag tttattgtag tttggatttt    7200
tttaagttta gggtttgtat ttttattttg agatagagtt ttattttgtt gtttatattg    7260
gagtgtagtg gtgtaatttt ggtttattgt aatttttgtt ttttaagttt aagtgatttt    7320
tttgttttag ttttttttagt agttaggatt ataggtgttt gttattatgt ttagttaatt    7380
tttgttttag tagagatggg gttttattat gttggttagg ttggttttga atttttgatt    7440
ttaggtgatt tatttgtttt ggttttttaa agtgttggga ttataggtgt gagttattgt    7500
gtttggttaa attttttttt tttaattaat tagttatggt ggtgtatgtt tgtagtttta    7560
gttatttggg aggttgagat gggaggtttt tttgaattta ggaagtagaa gttgtagtga    7620
gttgtgatta tattattgta ttttagtttg ggtgatagag taagattttg tttttaaata    7680
aataaataag tgaatgaatg aaaaaaagat aggtatgggt ggatgagata gggagagagg    7740
ttttatatgg ttggttatta gggaaatgta gattaaagtt aaggggatat tagtttttat    7800
tttttgggat ggttagaatt ataaggatag ataatggtag gagttgttga gggagtggag    7860
atgttaggat tttttttagt gttgttggtg ggaattaggt aatgtagttg ttgtggaaga    7920
tggtttggta gtttttttaag atattatgaa agtttattat atgttttagt aattttattt    7980
ttaggtattt atgtaagata aatgaaaata ggtttatgta aaattttgta aataatgttt    8040
atagtattgt ttatagaagt tatgtggaag tagttttagt gtttatgaat agataaaatg    8100
tggtttattt tgtaaaggag tagtgaatga aattgttttg ggttgtaggt gtatgaattt    8160
ggagggtatg gtgttgagtg aagaggttag atatggggtt ttatgttttg tggttttatg    8220
tttatgtatt ttttgtataa ggtaaattta gttagttatg ttttgtggtt ttatatttat    8280
gtattttttg tataaggtaa atttagttag ttatgttttg tggttttata tttatgtatt    8340
```

```
ttttgtataa ggtaaattta gttagttatg ttttgtggtt ttatatttat gtatttttttg    8400
tataaggtaa atttagttag ttatgttttg tggttttata tttatgtatt ttttgtataa    8460
ggtaaattaa gataggaagt tggttagtgg ttgtttgggg ttgagggttt ttttgggttg    8520
tgggatgttg taatattgga ttgtggggtt gggggttttt gtgggttgtg gaatgttgta    8580
atgttggatt gtaatgatgg ttgtataatt ttataaatta ttaagagtga ttaaattgta    8640
ttttttttaat aggtgagttt ggtaagtttt attttaataa agttgttgtt ggtatatgtg    8700
gagatgttta ttgggaggtg gttggtgtgg taatattagg aattgggagg tgttttggtg    8760
ttattatatg taggaggagt atagattttt tttttgttga ttattaggtt ttatatggtt    8820
ttttttttggt tttgtttttg ttgtgttttt ttgtttatta gggtaatttg tagatagggt    8880
tttagggttt ttttatatgg ttttttttttg gttttgtttt tgttgtgttt ttttgtttat    8940
tggggtaatt tgtagatagg gttttagggt tttttttatat agtttttggt taattttttg    9000
tattttttagt ttttgttggt tggagtttag gttttagtgt ggatgttatt tggaggttgg    9060
tttttaggtt tggttatttg ttatggtttt gtttattgtt tgtagtttta tgtttgtttt    9120
ttttatttgg gggttaatag atagtaggat attgtttttt gtgggagttg ttgttaggtg    9180
tagtttggta ggttttttttg agtgtgagat gtaggtatat tgtttttaga agttaggttt    9240
ggatttgttg tgagagtaat ggtggttatt gtagtattgg ttttgaggtt ttgggttttt    9300

gagtgttttt gtttttagtg agggttagga tggggtgatg gtaggagtat tggtggggtt    9360
tagttagttt agattttatt atttggattg tttttagatt aggtatttta gaatttgatg    9420
gtagtttttg agggatgtag tgggtagtta aggttatggt gatagttgtg aggagttagt    9480
atgtggagtt tggttattaa gttatgtggt ttgtgatttt tggggggggta gtaggtggag    9540
tttagtgagt gttggtagtt ttgaaattgt gggtagtagg taatatgttt agagtttgta    9600
gttgttagtg attttagttt aaaaagaagt tttaggaata gttttttttgt gtggagtagt    9660
atagtttgtg agaaatatag ggttgttggg attgggtgta ttagttttta tttttattat    9720
taattagtta ggttgaggtt ggggttttag atttgtttag tttttgtatt ttggggaaga    9780
gagttgttat aattggaagg agtagggatt tatttttttg taagtatttt tgttaaggag    9840
ggggtatttt gagaatgagg ttttttgttt ttgggagttt tttggggtta tttttttttta    9900
ttttttattt ttgggtttgg ggttgtgggg gttggtgttg ttggttgatt agggtatttt    9960
tttagatggg tttttttggg ttgtttttat attagttagt tgttgttggg ggatttgggt   10020
agggtggttt tttggggaag ttgtggtttg atttggtttt tgtggttagg gaggaggtgg   10080
agttttttat ggtttttttgg ttggtggttg tgttgagagt tggatttggt tatttttggt   10140
tagtagtggg tgaagtggag tatggaggga agagatagga tggttggttg ggtagagtga   10200
tatataataa aaataaatag atagagagag aaaatagaga agaaatagta tgatttatat   10260
atatagtttg ggaggatttt ttagtatgtt tagttgtttg gggtaggttg aggggatatt   10320
ttagtgtagg gtttgtggtt tgattttggt atttataggg tttttttttttt ggggttttag   10380
gatatttttt ggtttttaaa agtttggtat tatgtttgtg gtatttagag atgttttatt   10440
ttttggtgag aaaagagatt atatgttttt gttttattat agaaggaagg gttaagagaa   10500
tgtttattta aaggtaggta ggggaggtag tagagggtgt agggagttgt tgtaggttag   10560
gttggatgaa gatttggggg gttgttttat atagttgttt atatgttatt ttggagattt   10620
ttagaaggat gggagtttga ttatgttttg tgtatttata gagttaggtt ttgagaaata   10680
aggattttgt gaggttatgg ttgagggtgg gggttggttt ttggggtttt ttttttagag   10740
gggtgagagt tatttttgtt gggaggtttg ggagaggtat aggttgttta gggtttttga   10800
tatgtggttt atttgtgttt ttgggaaggt tattttattt tttttggttg aggagaaatt   10860
aaatatagtt gagaatgtta attttgagat gggaggattt tgtttgtgta ggtagttgtt   10920
ttttttagag tttgatatat tttttttttt ttgttaagat tggggttaag atggtgtttt   10980
ggtaggttgg ttggttgtgg gttgattagt agtttttgtt gttatatatg ttatagtttt   11040
ttttttagtt tttgattaag tttttgggat atatagttag tgttagggta gagtttagga   11100
ggaaagggtt aagtaagtgt taaagttgtt gtgtgtgggg gattatttga gtttaagagt   11160
ttgagattag tttgggtaat atagtgagat tttgtttttta tagaaaatta aatattattt   11220
gggtgtggtg gtgtgtattt gtagatttag ttatttagga ggttgatgtg ggaggatggt   11280
gtgagtatta tagttgtatt ttagtttggg agatagagtt aaaaaaaaaa aaagtattaa   11340
agaatatatt ttttggagtt tagggaggtt tgggggtagt tttaggtgag tgggttgggg   11400
ggggtggggg tgtagtttta gtttttttttt tgtgatgatg gggagtagta gagggtaggg   11460
atgtgggggg tttttttagt tagtgttttg atatttggga ggggtttgta gttttgaggt   11520
tatagggggtt gagtttagtg aggtatgagt atttgggatt gtatagtaga tgtgttagtt   11580
ttttttttggt ttttttttat agttttgggg atagaagttt taagggtagg ggttgagggg   11640
gtgagataga gaattttgga tttttttttt tttgaggttt ggggtagaag ttgttgagtt   11700
ttttgtgtgg tgataatgtt tgtttgaatt attttatttg gtatgagggt ttgttttttt   11760
tgaaggtgtt ggggttgtgg gagggttgaa gtattgtggg ttttgtattt tggagatttt   11820
gttattttgt ggtggtttag gtttgttttg taggtagaat ggttatagga tatatggggt   11880
tagtgtgggg tgtggattag agtttagttt taagtttggt ttagtttgta agttaataat   11940
```

```
ggtttttata tttttaaatg gttggaaaaa aattaaaaaa aaaaataata ttttgtgaga   12000
ggtgggagtt ttatgaaatt taggttttgg ggttagtagg gtatagtttg gtttatttgt   12060
ttgtaggtgt ttttttatag ggattatttt gtatttatat ttgtttttga aggggtattt   12120
ggtggttatg ttggagagtg ttgtgtagtg tggatggggt tatgggagtt gtggtggatt   12180
ttttggtatt tatgtaagat aggtggaaag gaatagataa aataagtttt ggtgatgtgt   12240
tttgtgtgat ttgaggtatt tgagatattg ttgtgtttgt gtgaagtgaa tgtaaaatat   12300
gatggagata tttaggattt gttttttagg ttgggttttt ggatgtgtta tattataggt   12360
tttgtagtta ttagtgttgg tggtttttat aaggggtagt gtttaaatat gtaggatgta   12420
ggtgaggttt atggggtttt gaatgttata tggaagtaag gttgggagtt tagtatgtat   12480
ggagtaggtt tgttagtagg gagtttatgt attaatttag ggttttttgt gtggttatgt   12540
tagatgttgg aattatttaa atggttttttg tgttttttag tttaggttat gtaggagttt   12600
tttatgggag atgggttggg tatttttgat attttttttt tgtagttaaa ttttgagtgg   12660
gttagggtgg ggtagggttt gggtgggagt ggtgattagt ggaggtgtta tgggatagaa   12720
gttgtttgta ttgggttttg agttggtttg gttttgatag gagttttgtt tgttttgtgt   12780
gaggattgta tatttgttag ggttaatttt agttttttttg ttatttattt atttatgtat   12840
ttatttatgt ggtatttggg gttatgtgtg ttgttagaaa tataggtttt tattaggttt   12900
tgtttgtttt ttattgatgt ttttaggttt aaggtagaag tggtgtttag ttgtatttag   12960
ggtaggaagt gagttgttta ttagtttttt gttttttttgt gtttatttag aaaatttttt   13020
ttgagttgga gttagtttta ggtaattgat tttgtgggtt ttatttggat aggagggagg   13080
tttgttttag gagagtaggg atagtatagt ttttttttgt gggtgtttgg aagagaagag   13140
aggattttgt agagaaaagt aaatgttttt gttatagagg ttggatagtt ggttttgtta   13200
agttttttttt ggggtttttt tagtatatgt taggggattt ttagagatgg tattgttgaa   13260
ttgttttgat ttgttgttag aggagttaag agttttgtga ggggtgttgg gggttttgtt   13320
ttggttggtg aggtttatta aggagtaaga gtttttggga tatagttgtt agttatgtgt   13380
taatttgagg gttttattgt aagggtgatt gtgggaaatt ggtattgatg tttttttttga   13440
aatttatttt gttggttttt ttgagtgtag ttgtgggttt tgggtttttt tgtgttttgt   13500
ttatttatga tggatagatg ttagtatagg atgagtagtt atggaaaagg tttttaggtt   13560
aggtgtagtg gtttatgttt gtaattttag tattgtggga ggttgaggtg ggtggattat   13620
gaggttagga gtttaagatt agtttgtgta agatggagaa attttgtttt tattaaaaat   13680
atataaaaaa ttagttgggt gtggtggtgg gtgtttgtaa ttttagttat ttgggaggtt   13740
gaggtaggag aatggtttga atttgggagg tggaggttgt agtgagttga gattgtgtta   13800
ttgtatttta gttggggtga tagagtgaga ttttgtttta aaaaaaaaaa aaataggttt   13860
ttagtttgtg tttttttaga gggagggaat tgtatttagg aggaagattt tgatgggagg   13920
gttttaggaa agaggagtaa gaagtggtgg gaaggaggga gggtagtatt agtagttatt   13980
tttagggata gtgtttagtt agggttgatt gtattttggt tgtgggtgtt ggttttggtt   14040
agattttggt aggggggtta ttatgtttag ggttattgag gatagtaggg atagggtttg   14100
ttgtggttag ttagaggttt ttgggaggaa tgttatttg ttatttttag gtttggttta   14160
ttttagggtt tgggtatatg tgaattattt agtgtagtgg ttttgaggat gtaggttagg   14220
ttagtttgtg gtttttattt tggtttttttg tttgaataat tggggggggg ggggggtgtg   14280
taggggtatt gttattaagg agtgtgttta ggttaggttg agggatagta gggtgaggtt   14340
tttgtagagt atgttgaatt ttgattttttg attttaggga tatgttttgg gttgttgttg   14400
tttttgtagg gtttgttgtt ggttaagtat ttgtgttgtt tttgtgagtt gtatgtgttt   14460
tttgggtttt tgttgtgggt taggttgttt taggtgtttg tgaatgggaa ttagtaggag   14520
ttgtgattat agggttgatt tttagggtgg tgttttaaga gtggttgtga tttatgattg   14580
ttatagagtt aggttgtagt tatgtttgtt tttgggtttt ttggaggggt agggtgtgga   14640
gggttttggg ttgtgtggat ttatgtttga tttttataag tttaattgtg tagttttttg   14700
tttttttttgg gagttgatgt tttagtttta aatggggtgg tggtttagat ttagttttag   14760
agaattgtag aggtgaggtt gttattgttt tgtggggata agtggttaat gagtagggtt   14820
aattggttgg tttttgtttt gtatgttata tatatatttt gtgagggtag taaggagtat   14880
ttagttaggg gatgtgtatg agaagggtta ggatgattat attgtttggg gtttagtata   14940
gggttgagtt ttggaggtga ttattgttgt tttgttttat ttgtggttat tgaggtttgt   15000
tgggggtttt tggtgttggt tttgatagtt tatttgtggt aggatagggt tttgaggtgt   15060
tttttggtag gtggtgggtt tagtgggggt gatttgtggg gatggaatgt atgtttttga   15120
ggttttagtg gatgtaggta aaggtatttt ttggggttag tgtttttatt tggggagttg   15180
ggagtttata tgggagtagg gttggtatag tgatttttag ttttgtgtgg gtggtattta   15240
tagttgtgtt tattttgtat atattttttta ttgttttgtt aattaattat gtatatggtt   15300
gtatttttga gttttttttt gttttgtagg tgatataggt gtgttttatt tatatagtat   15360
ttgtttttgg gttttatatt tttgttaatt ttttatagag ttattttgtt attatgggaa   15420
ttatagtgat tttaataagg atgttttggt gttatttttg tttgagttgg gggtgtgggg   15480
ggtagtagat ggaaagtggg ggtttttgtg tttaggtagt tggtggtttg gttttgtttt   15540
atggtaattt tattgtgttt taaaattgta tttattagat tagaggttgt tggtttaggt   15600
```

```
aggaaataag agttagttat tattgggatg aagtttagtt ggtgtttata atttgaagat   15660
aggtaatttt gtttttgttt ttattgatgt taaggttttt gtggaggaga tgattttaag   15720
gataggaagt ttttgtatga gttgggtggg agggttgagg atagggtttt ttattgtttt   15780
tataagaaat aaggttaggg ttttgttttg ggggaggatg tggattttga ttgtttgttt   15840
taaatgataa ttggtaggat agtatttttt agaaataggt gtaggttgtt ttttagggat   15900
taggtatttt tttggttgag gatagtggtg ggggttgtgt ataggttttt tattagtttt   15960
agttttagtg ttgttgtggg gatggggagt tttatttggg ggttggggtt atagtatagg   16020
ggaggttttt aaagggataa atatttttgt tgggattttg ggttgttttt attgttgttt   16080
tttatgatag gattatggtt taaatggttg ggagaatgag gtttttttagg gtaggtaggg   16140
ttgttggttt ttaggggttt tgtgaattaa gggttttgag aggtttttta gttgtgggta   16200
ttttaatttt gttgttttat aaatagtttt gtagtgtaag taaatttata aatgagttgt   16260
agggtttggg aataaattta tttgaagttg tagtaagttt ttaggttttt tttgtgttgt   16320
gtttgggttg ttgtttagaa agggaggtat ttaattaggt attttagttt ttattaggag   16380
gattttgatt ttggaatata gagttttgtt tttagttttt tttgtaggag ttagagtttt   16440
gggaaattag ttttgggaga ttttttgttt tagaagttgt ttgagttttt gtttttgggt   16500
gtttgtttga gtgttaaagg tagtttgggg agtagtaggg gtttagtagg ttttttatta   16560
ttttggttta aggtttttga tttgtttagt gtggttaatt tttggttagt taggattgta   16620
gatagggtta tttaagattt tgtttggatt ttttatggtt tgattttgtt ataaggagta   16680
ggttgtgggt aagagtttat gagtggggtt gtagagtagt attagttagg gagggtggtt   16740
gagtttttta ggtattgtta gggaaaggtg tttagtttgg gtaggtatag gtatatagtt   16800
aggtggttat ttagattttt ttggatttta gtttgagtgt tatttggtta ttatagttgt   16860
tagtagggtg gttattgttt tgagttaggt ggttttgttt ttttaaggtt atgtttttta   16920
tggtggattt attgtggtgg gtaggatgtt tatgtttttt gttttgtttt ttttgggttt   16980
tgtgtagggt ttattatgga ttttttattt agttattatt tttttttttg ttggttttaa   17040
atttttttatt ttattttttg agatagagtt ttgttttgtt atttaggttg gggtgtagtg   17100
gtgtaatttt agtttattgt agtttttgtt ttttaggttt aagttatttt tttgttttag   17160
ttttttaagt agtttggatt ataggtgttt gttattgtgt ttagttaatt tttgtatttt   17220
tagtaaagat gaggttttat tatgttggtt aggttggttt tgaattttag attttaggtg   17280
atttatttgt tttggttttt tttagtgttg ggattttagg tatgagttat tatgtttggt   17340
tttttatttt tttttgtgat tagtatgagg atggtgtgta ggtgtgttta gtggtatagg   17400
tattatttgt tttttttttt tttttttttg atagagtttt gttttgttgt ttaggttgga   17460
gtgtagtggt gtgattttgg tttattgtaa gttttatttt ttgggtttat gttatttttt   17520
tgttttagtt ttttgagtag ttgggattat aggtgtttgt tattaggttt ggttaatttt   17580
tttgtatttt tagtagagat ggagttttat tgtgttagtt aggatggttt taattttttg   17640
attttaggtg atttatttgt tttgattttt taaagtgttg ggattatagg tgtgagttat   17700
agtggttggt tttttttttt tttttgagat agggtattat tttgttattt aggttggagt   17760
gtagtggtgt tattttgatt tattgtaatt tttgtttttt gggtttaagt aattttagtg   17820
ttttagtttt ttaaatattt gggattatag gtatatatta ttatgtttag ttaatttttg   17880
tatttttagt agagatgggg gttttgttat gttggttatg ttggttttta tttttttgatt   17940
ttatgtgatt tatttatttt ggttttttaa agtgttagga ttatatgtat gagttattga   18000
gtttaattga attttatttt tttttttttt tttttttttt gagatggagt atagttttgt   18060
tgtttaggtt ggagtgtagt ggtgttattt tggttaattg taatttttgt tttttaggtt   18120
taagtaattt tatagtttta gtttttttgag tatttgggat tataggtata tattattata   18180
tttagttaat ttttttattt ttagtagaga tggggttttg ttattttggt taggttggtt   18240
ttgaattttt gattttaggt aatttatttg ttttggtttt tgaaagggtt gggattatag   18300
gtgttagtta ttgtatttag ttttgatttt tatttttaat aggaaaaaag gattagataa   18360
gtatttgtgg tttttggtat ttttttattt ttttgtttgt tatgtggtgg tagaagtagt   18420
tttggtttgg gtagtttggt gattttattg gtggttaagt agaagttttt gggatatagg   18480
gggtatttgt tattttagag aaggggatgg gggttttggg agttgtgtgg ttttgggtat   18540
ttgttatttt agagaaggag atggaggttt tgggagttgt gtggttttgg gggtatttgt   18600
tattttagag agggagatgg aggttttggg agttgtgtgg ttttgggggt atttgttatt   18660
ttagagaagg agatgggggt tttgggagtt gtgtggtttt aatttagagg aggttttgtg   18720
gtattttttt ttttattagt tttagttttt ttgtagttat ttttttagga ggggtaggaa   18780
ttttagaaag ttttaaaatt tattgttttt tttattatag gggataggga ggtggggggt   18840
agggagtggg gattaggtag gggtagaagg tgtaagaggt ttaatttgtt tgggggattt   18900
tatttataaa gggtagaggt tgggtgtggt ggtgtttatt tgtagtttta gtatttggga   18960
ggttgaggta ggaagattat ttgagtttag gagtttgagg ttgtagtgag ttatgattgt   19020
aattgtattt tagtgtgggt aatagagtat gattttggtt taaaaaaata aaatttaaaa   19080
aatgttaaaa taaagggtaa ttttttgaag agaagtgagg ggaaggttat gaggaggaat   19140
tttttttatta gtggagtaag ttttttttat agaggagtag atgttgtagt tattgtgata   19200
tttaaaaagt agtgatggtt ttgtgttttt ttgatatttt gtttaggatt gttagggagg   19260
```

```
tggggatggt aatttttgaa gttttttttt gattttaggg aatttgataa attgttgtat   19320
ttggaggtta ggatggtgag gttgatgttt atgatttgat gggatttatg ggtttatttt   19380
ggtgttttgg atgagttttg gggagggtgg ttatattgtt ggttttttat gtagtattta   19440
ggtaggtttt ttagagtgtg taaagtaata gtttgatgta gtttattttg gggggtttat   19500
ggggttttttg ttttgggtta ggatgttttt attttttat agattttatt ttgtgtattt   19560
tgtgggttta gtagtagttt ttgttttagt ggtataatgt ggttgtttat agattttatg   19620
tttagatatt tttttgggtt atgtattggg aggtttttat ttagttgtgt gtgatttata   19680
tagtgatagg gtgtggttga atgggtttag gtttagtttt aatttatagt tttttttgtag   19740
tgagatatat atgtaaagat tataggttag taagtttgtt tttttagatt tagaaatagg   19800
ttgagtttag gtgtggttgt gtttatgtta taggggttgt tgtgttattt ttttggagta   19860
tgtaggagtt tttttgggtt gttagaattt aggtatttag aattgtttgt ggtgagggtt   19920
aattttaggt gtttattgat tgtttgtttt gtgtatggtt gtatagtgtt agatattagg   19980
tttattaaga aatagaaatg gattttgttt ttggtgttat atttagtagg gagatagggt   20040
ggttttaagt gtaagtgttg taggtagatt ttatagtggg gagttaggga ttagttatag   20100
ggttatatga gaattgtata gattttagtg ttttttttag tgtggggtat gagaggttag   20160
tggttttata tagttgaggt atagtttggt ggtgagaaaa tttatattta tattgagtgg   20220
gtatgaggga tgtggaaggg ttttaggtag agaataagtt ttattgggtt tttgggtatt   20280
tttgttgtgt taggggttag agggtaggat tagagggata gagtaggaaa ataggagagt   20340
aaagtgtatg aggtagtttt tgggttgggg gttttttttt tttgtgtttt atgaggaaat   20400
tatttaagtt tattttttgt gtttttttgtt attttagttt tatggtttag ggttgagtgg   20460
ttatttagga tagatataga tttgataagt tattagtttt tttgggtttt tgttattgtt   20520
ttttaaagat tttaagttgt tttgtttaat gatgttgttt tttttttttag tatttgttta   20580
tgtttgttgt tatgagtaga ggtattatat tgtgggttag agggttggat taggggtaat   20640
ttggttatta atggttgagt tttttattga gtggtttagg tttatgaata atatttgtgg   20700
ttttggagga ttttttgggt agtagaattt ttttttttaa ttgttttgtg tgaattggtg   20760
gaattagtag gagggagaga ttaggtgttt agttttttttt ggttttttttt ttgttatgaa   20820
tagttgtttt tagttttggt tttttatttg tggtattggg gttattggag tgttgtttaa   20880
ggttagggta agaagtaagt gaggggtgtt tggagagaga gttagtaggg tttgaggttg   20940
ttatatagat gtagtattat aggggattag ttattttttg tttttagaga ttatggtttg   21000
tggaggttta gataagaggt tttgtgtagt tatgtagtta ttgagttttg ggttttttttg   21060
gggatttaga ggttattggt tttttttgtta attaaggagt tttggagagt tttagatata   21120
gatagaagta ggaaaaagag gggtgttttt tttgtaagta gaaggatttt ggaggaggta   21180
aggtatattt aatttaaggt taggttaagt tggtttttttg taggatttgt tttttttaga   21240
taggttagaa aaataatttt ttttgtttta ttttatagtt gagaagttat ttagaattgg   21300
aagggagaat tatttttata tttttgttta gttttagatt ttggaaatgt ggaggtaatt   21360
atggggtatt tttatattga attgtgattt atgtttttgt atttttttaga tttgggtaga   21420
ggaagagatt tgggtttttt gaaggagaag tatataattt tttagtattt aatggagttt   21480
tagttttttt tattggtttt atatatttag gtttggtttt aggttttggg ttttattatt   21540
gtttttttggg ttttgttatt tgtagtttgg taataaattt tagagtagta agggggtttt   21600
gagagtttgt ttggttaggg ttagttgatt atataagagt ttttttggtt aggattagag   21660
gggtttttttt tgtagttagt gggaggatag tagatttgta atttttttagt tggtgttgta   21720
gtagttttag aggaatatgg aagagaatat tggtatggtt ggtatagatg gggtttttgt   21780
tagttttttt atgttaggtg gttgttttta ggttgttttt taagtgaggg gagtgatgtt   21840
tgtattaagt ataatatttt taaggtggtt aggattgtat gatgggtttg gatataaggg   21900
tatttaaggg taggtagggt tgaggttgtt tttggtgtgt gtggtttttg gggttggttt   21960
taagtgattt atatggttta ggtttttgtt gatttttttgt tttttttttgg gtttttttgt   22020
gaagtattgt ttagttttgg tttttattag tttggaggtg ttagtgggtg gtgggttgat   22080
tttattttgg gggttttgtt gtttttgttt tggtttggag tttggtgtgt ttggtttttg   22140
aggtttttttg tgttttgttg agtttgtgtt ttttgttgtt ttttgggttt gtgtttttttg   22200
tttggggttt ttggttgggt gtttggttgg gtttggttga tgggatttat ttttatagtt   22260
tggtggtgtt gttttgtttt tgtggtttgg gtagtgtggt tttggttttt gggtttgttt   22320
gtttttgttg gttgatttat aatttttgtt tttggagagt ggtggggttt tttttgttgt   22380
tggttgtgtt gtttggatgg ttttttgttt tttagttgtg gggtgttttg atttggtttt   22440
ggttttggtt tttgtttgtg tttggtgttt gttttatgtt gtttttgtag tgtagtgttt   22500
ggtttgggtt ttgtggttat ggtgtgtttg gggtgtatgt ggtgttgtga attgtgtgtt   22560
tggattatgg tttttatagg gttgtgtggt gttgatgggg tggaagtttg gggtggtgtt   22620
tgtgtgaggt aggttgggag ttgtagtttt tgggtggtgt ttttttagtt tgttgattgt   22680
ttgggttatt tttttttgttt ttgttgttgg agatggttgg gtttgaggtt ttggtttttg   22740
ggaggtgttg tgttttagga ttattaggga gtggggtggt ttgaggagga gttggaatta   22800
taatttttag agggttttgt gaggggtatg gaggggtggg gtttgtagtt gtggtgtttg   22860
tgtattagtg gtgttataga tgtgtggggt ttgtggggag ttgggttggg gttgtttgtg   22920
```

ttttgtgggg ttttgggtgg ttgtttgatt gaggatttat gattttgttt tgattttgtt 22980
gtgtttgtga gatttggaga tttttttgtg ttttttagga ttgttttttt tgtgatgttg 23040
tgtgttgtgt ttggttttgg tggttaagag tggtggtggt aaggggattg ttttggtttg 23100
agatgtggtt tggttttgta gtgttgtttg ggtatagttt ttttaggttt ttttgttttt 23160
ggttgtggag ggatgttatt aaatggttat gttgggttta attgggatgt ggtttatggt 23220
gtttaattag ggtttggggg agggaagttg gggtgatttt tgggtttttt taggattttt 23280
gtgtaggttt tatttttttt ttggaggttt tttttttgtt gttttgaggt tatttttttg 23340
ttagtagttg ttgggttggt ttttgggaga atgttttggt gttaagttta ttaggttgta 23400
gggtgggatg tgagtgttga gggtagttag atttttgttt ttaggtgtat ttgaggagtt 23460
tgagaaggta gtggttttgt gggagaggga gatataggag tagggttggt ttgttgagat 23520
tttggagaga tggttgtggg ttgattttgt ggtgagattt ataagtggga ggttgggtga 23580
tgattgtttt tgggttagaa aatttaagtt agttttagta agatttataa ggttagggtt 23640
agtgtttttt agaggatatt tatgttttag gaaagttaag gatgggtttt tgttatgttg 23700
agatgttgta gtaggga 23717

<210> 279
<211> 17265
<212> DNA
<213> Artificial Sequence

<220>
<223> chemically treated genomic DNA (Homo sapiens)

<400> 279

gtaggtggat tatgaggtta ggagtttgag attagtttta ttaatatgtt gaaattttgt 60
ttttattaaa aatataaaaa ttagttaggt atggtggtat atgtttgtaa ttttagttat 120
ttaggaggtt gaggtaggat aattttttga atttgggagg tggaggttgt agtgagttga 180
gattgtatta ttgtatttta gtttgggtga tagaatggaa tgagattttg ttttaaaaaa 240
aaaaaaaaaa aaaaagaagt tttagatttt ggttgtgttg gttgtaaaag gagagattta 300
gtaagtgggg gttgtgttgt agattgttat ttataatgga tgggttattg agtaggtttg 360
gttaattggg tgtttttttg ttggagggtt agtatattag attgtaggtg gtgtgggtta 420
gtaaggtatt aggggatgtg ttatatatat agtttatttt tgtttagtta tgtatggata 480
ttttgggttt ttgagtaaat ttgtttttat gtggtgtgat tatatggagt tatagatatt 540
tagtaaggat atgtagtttg tatattttttg gtattttaga tatagtgatt tgtattaggg 600
tttgaggttt ttttagggaa tttatttttt agaattattt agaaataagt tatttttttat 660
ttgtttagta aaggtttgtt gagaggtgta tagtgtttgg agtttaagtt gtgttaaggt 720
ggtaggattt ttagtttagt ttaggatttt tagtagagtt tttattttag tgtggaggtt 780
tgagaatgtg aggaaggagt tgtttagtat ggatgagttt aggtagttgt tgatgtttag 840
tatttattgt ttggtaggtg tggggtttag gtttttagtt atttgtagga tgatggtagt 900
ggttagtggg tggtagttta gatttgttta ggatagggat gagaagttat tttttttagta 960
gataggatag agtttggtgt tatttaatag aatgttttag aatgttggat atatgggata 1020
tttgtattgt ttgtgatggt agttttttgt gatatgtgtt attgaatatt tgatagtaga 1080
ttggtgtagt taaggaatag agttttgaat tttattttttt tttttttttt aagatggagt 1140
tttgttttgt tatttaggta ggagtgtatt ggtgtaattt tagtttattg taatttttat 1200
tttttgtgtt taggtgattg ttttggttta gttttttgag tagttgggat tataggtgtt 1260
tgttatgatg tttagttaat tttttgtatt tttagtagag atggggtttt attgtgttgg 1320
ttaggttggt tttggaattt ttgattttaa gtgatttgtt tgtttttgtt ttttaaagtg 1380
ttgggattat aggtgtgagt tattatgttt ggttattgtt ttattttaat taattttaat 1440
atgagtagtt atatgtggtt tttggttttt gttatggatt tgggagtaat tttttttggt 1500
tgtggtttat gtgtttttttt tgtgtgttgt tggggttagt ttgtatgtaa ttttttgagg 1560
attttatgtg tgtattttta aggggtgtgg ttttttgttt ttgtatgaat gggaagagtt 1620
tttatttgtt gtatttttgg aaagagtttg tgaaggattg gtgttaattt ttttttaatt 1680
gtttagaaaa attttattgt gaaggttttg agtttaagtt ttttttttgtg ggattttttt 1740
tttttttttt ttggagatgg agtttttgttt tattgtttag gatggagtgt agtggtgtaa 1800
ttttggttta ttgtaagttt tgttttttgg gtttatgtta tttttttgtt ttagtttttt 1860
gagtagttgg gattataggt gtttgttatt atgtttagtt aagtttttgt atttgtagta 1920
gagatagggt tttattgtgt tggttaggtt ggttttgaat ttttgatttt aattgatttg 1980
tttgttttgg ttttttaaag tgttgggatt ataggtgtga gttattgtgt ttggtttttt 2040
ttaatgtttt atatagatgg ggttttgtta tgttgtttag gttggtttta aatttttgga 2100
tttagatttg tttattttgg ttttttgaag tgttgggatt ataggtgtga gttattatat 2160

ttggtttggt tattgggagg tttttaaggg attaatttgg tttttttatt tgttatagat 2220

gtattgagat ttttttttgg agtgtatttt ggaagtgtgt atagttgtgt gtttgttttt 2280
tttgagttat ttggtgtgtt gttgtgtagt tgtttgtggt gtttgtttag tgaagtgttt 2340
ttgttttttt atgattttgg tttttgagtt tttttttttt ttttttggtt agtttagtta 2400
aggttgttta agtgtgttga tttttttga gtagtttttg gtggatgttt ttttttttgg 2460
ttgtagtatt ggaaagtggt ggttttgggt atggtgttga ggtttaggtt ttatttttag 2520
tatttttggg tttttagttt tagtttattt tgttttggga tatttggaag agaaagagga 2580
tggttaggta gatgggatag ataattatgt tggatattag gttaatagtg attgtgttga 2640
tgtttagtgg gtttagtttg gatatatgtt ttgtgttgtg tggaggagag gaggttatat 2700
aggtgaggtt gaggggtagg aagggttggg taggaagagg ttgttttgat ttttatggta 2760
tttatttgta ggtagagtta ttaatagttt tgtattatat ggtgttggtg tttaggaaga 2820
ggtagatgag gagaatgtag taggtggttt tttggatgtg agtgaaatag ttatgaggtg 2880
gttggtttta tatggagagt tagatgtgtt tgttaaagaa gttatgttgt agtttagtta 2940
ttagtaggtg ttggaagtgt aatagggttg tgtgatttag aaggtaggga gggttgtatt 3000
gtaggaggtt atggggtagg attattttgt ttaatttttt atggagtggg aatatggaat 3060
gaggttttat ttgtagttag tatttttttt tttattaggt ttttgttggt ttttgttttt 3120
attgaaagtt agttattgat taggaagaag gtgttgtgtg ttgtttgtag gtttttgatg 3180
atgatgtgtt gtaggaatta ggtagggttg agttttgtag aggtgtggga gggaggttag 3240
gtttgtaggg tgttttaatg tgggggtaga ggggtagagt ttggtagggt ttgtatagat 3300
ttttgttgtt gtgttatatt tggatttttt atatgttatt taggttgtgt ggggtggtga 3360
tttggaagat gtttagattg ttgtggtgga aggttttgtt gttgtttagg tgttggtggt 3420
tgttttggtt gtttatttta tatagtatga tgtttatgtg ggttgtggta tttggagggt 3480
aagagggagg ggtgggaggt ttggtttgtt gtttaatatg tgtggtattt taggtaagtt 3540
attttagttt tggtttttgt gtatttaagg agttatatag gtagttttgg ttttgtatgg 3600
ttttgttata tatgaggagt tgtggttatt gtaatttgtt taataaatag taggatttta 3660
aggatatgat taagttatat ggaaagaatt gtaatttgtg atatgtaaat atggttgtat 3720
atgttttagt ttatattata attagtgatt tgtgttgtat atttgtttat gttttagtta 3780
tgttataatt agtgatttgt attatatatt tgtttttttag tttatgtata gattgtgaag 3840
tgtgaagttg tgttattttt ttttttagtg atagatttag gtgatagtat tttttttttt 3900
ttttttttg agatggagtt ttgttgtgtt atttaggttg gagtgtagtg gtgtaatttt 3960
agtttattgt aagttttgtt ttttgggttt atgttatttt tttgttttag tttttttgagg 4020
agttgggatt ataggtgttt gttattatgt tgggttaatt tttttgtatt ttttagtaga 4080
gatagggttt tattgttagt taggatggtt ttggtttttt gattttgtga tttgtttgtt 4140
ttggtttttt aaagtgtttg gattataggt gtgagttatt atgtttggtt gatagttttt 4200
aaaagtaggt aattaaaaga attgggaaat gaagatgaaa gtaatatgga aataaaaaat 4260
gggaatatag ttaggtgtgg tggtttatat ttgttatttt agtattttgg taggttgagg 4320
taggtggatt atttgaggtt aggagtttgt ttggttgata tggtgaaaaa ttaattgggt 4380
gtggtggtgt gtatttgtat ttttagttat ttaggagaat tgtttaaggg gaattgttta 4440
aatttaggag ttggaagttg ttgtgagtta agattatgtt attgtatttt agtttgggta 4500
atagagtgag attttgtttt taaaaaaaga aaaatgaaaa taaaaaggga atgttagaag 4560
ggtaatttta ataaaggaaa atggaggtat tgaagaaata gttatgggga gggtgttggt 4620
atttttgttt gagagatgag ttatgtagtt aggattgtgg gtggatgtat ggttttttat 4680
agtggtagtg atgtttatgt tatttgtggg gttatgttat tgtgtagaat gtgggttgtt 4740
tattttgatt gattggtatt tatttttagt taggagttgt tttagttttt agggatagtg 4800
agtgtttatg aggttatttt taggatgaat atatgagttt tttatatagt attgtaaaaa 4860
ttgttttgtt ttgatgtttg tgatgagatt tatttttaga gggtgtaatt agtatagtta 4920
gtgagagtag gggaggtttt gttattttgt tgtgtttttt atttgagttt tggttttagt 4980
ttgttttgat gaggattttg tatttgaagt ggttttgttg tttatagaag gggatggtgt 5040
agttttggtt ggtatttaat tggtttagtt tgtgtaggat ggtggttatg attatgtagg 5100
ttattaggta tatagtatat gttagtatga tgatgtagtt tatatttgtt gttggttttt 5160
gtgaagatat agttgttggg tttaggaggt tatgtgtaag ttgtgttttt ttaggataga 5220
gttgagttta tttaggtttt ttttgatttt gtagaggttt ttaggagtat agggttattt 5280
ataggaaata taaagtgtat atggtttggg ggtatgaaga ggttggtgtt gaaggtggtg 5340
aggtggtggg tgaggtagat ggtttggtgg ggtgaggttt tttttagggg tagtagtttt 5400
tttgtttgtt atattatatt ttttttgttg aagtattggt atagggatgt gtataagttt 5460
atggatattt ttagtgttga ttagtggaag tggttggaga ggtttagatg gtaatttttt 5520
attgggtttt tggttttggg aagggaaggg gtagtggatg tgagtttagg ttttgttagg 5580
ttggatgttg gagttttaga gtttatattt ggtttagttt ttttgttgtt tgttgttttt 5640
atgggggtttg taatttgggt aatgttgatt tatgatgttt tgttttgttt tgttaggttg 5700
gtttgtagag tttattttgg ggaaatgaag aaggtgtagg gttggtggtt ggtattttgg 5760

agggattttg ggtggatttt tttgttagtt gagtagttgt gtttattggg ttggggtttt 5820
gagtgtaggt agattgttag gtagggtttg ggttttttag ataggtagtg gtttggggta 5880
gaatgtgtag gttatatgtt tgttgggaag tttaattatt tgggggatat ttatgatggt 5940
ttttttgagt ttattttttg ttatgggttt gaaaggttat aggagttttt gtattagagt 6000
tggtatttgt ttttttgtgg tttttagttt ttttttggtt aggtttttaa tagtttatga 6060
aatagtaaat tttattagag atatttatgg aagttttatg agaaatgttt tttttttaag 6120
aataaggtta gggggttgtg tgtgttttat ttgttgtata tattgtttag tagtgtatag 6180
ttgagttgta gatgtagtat ggttataggg ttgttgttgt ttagggtgat tatagtattg 6240
atggaggttt ggggttggat tataatggag ttggtggagt tgtggtggtt ttgggtagtt 6300
tagtttgagt tgttgggtat ttttatggtg atggtgtgtt ttgaggttag ttgtttgatg 6360
gggatttggg tgttggtttg tgtttggaat gttattgagg ttattttggt ggagatggtg 6420
tagttgttga tatagttaaa gagaaaggga ttggagttta ttagaaagat gagttgtatt 6480
atgttattga ggttggtggg ggttttgttg aaagtttagg ggatggagag gtggtagtta 6540
ggttttgggg tgttgttata gtatagtagg ttttgtgggt ttgagtgttt gttttggttt 6600
atgatttttt tgtttgttag tgttatgggt tttttgttga gtatgtggga gtgtgtgagg 6660
atgtgtatga gggtagaggt taggttgtag gtttgggatg ttattatttg taatggtgat 6720
ttggttttta gttttgagtg ttgtggtgtt tgtatgtttg agttggttag gtggatgagg 6780
tttttgtag ataggtgtga ggttagtgta gagataggga ggtagaggga gggtgggggt 6840
aggtaaaaag ggggagttgg agggtggggg ttgggagaaa gggggaattt gagggggtag 6900
agagtgaggt gtaggtagaa ggaagaggga agttggagag agagtggtgg agggggaggg 6960
ggaaggggat ggggatgagg atgaagatga gggggatgat ggggagaggg aggaaaaagg 7020
aaggaaaagg gtagagaaaa gagaaagggg agaagaagag gagtagggtg aaagggaggg 7080
gaaggggata agggggataa gggaggggaa gggggataag ggaggggaag gaggataagg 7140
gggataagaa agatgagggg aatggataaa aggatgggga ggattggggg ggaaatggag 7200
aaaagggag agagatggag aaaagggatg gtaataggga agggggaggg ggaggagaat 7260
gggaattggg ggaggggat aaggatggga attgggggag ggggatgagg atgggaattg 7320
ggggagtggg atgaggatgg gaattggggg agtgggatga ggatgggaat tgggggaggg 7380
ggatgaggat gggaattggg gggaggggag ggggatgaag atgggatggg gtaaaggtga 7440
ggtggttgtg gggaggaggg aggtagagga aagggtggta tggggtggat gggttatgtg 7500
gggtgggtgg gtggtgtggg gtatgggttg tggtatttgt gatgttgagg atgttgtttt 7560
tgatggtggt gggtgttatg gtgtttgtgg tggtttttgt ttgtaggatg tgtattatgg 7620
tttttagttt gtgtagtgtt tgttttaggt atgagtggta tatgagtttt ttgttgggtt 7680
tttgtgtgga gttagtagtg tttagttttg tttttggttt tattttttgt atatgttttt 7740
tttttatat gggtttttat ttggttttta tttttagttt tgtagttgga gagtttattt 7800
gattggattt ttatagtttt ttttattaagt attttttgtg gttttgtatg atttagggtt 7860
tttatttggg gaatatgtga tgtagtttat tgattatata aggtattttt ttatatgaga 7920
gaaggaggag ggtagaaggg agagaggaga gggaagtgga gaaaaggggg gagaggagag 7980
ggaaggagag agaaggggga gaggagaggg gaggggagag aagggggag aggagggagg 8040
gggaggaggg gaggaaggag gaggggaggg gtgaggggat ggaggggttg ggggaggagt 8100
ggaggggttt agtgggatga ggtgagggga aggtttaggg gaggggagga ggggaagggt 8160
taggggaggg gaggggttgg gggaggaggt aggggttagg ggaggggga gggattaggg 8220
gagggaaggg ggaggggagg ggttagggga gggaagggga ggggaggggt taggggaggg 8280
aagggggagg ggaggggaga gtggagggta tagagtagta ttttttagt ttttttttat 8340
atttgggttt ttttttatat tttgggtttt ttgagaggta ttttgtgttt atataggata 8400
gtagaatggt tgaggttatt gaagtaggtt agagattgag gaatgttatg gtaggaagga 8460
gtttaggttg gaggtttagt tttttggtta agttgtttgt ttgttttggg gggttgaatt 8520
tagtattttg gtaggtatgt ggggtgggga gagtatgtgg ggttatttta ttatgtattg 8580
ggttagtgta gtagtgattt gttggatgtt atttatagtg tggattttta gggatattag 8640
agtttttgtg atgtttttgt gtatttgggt ttggtgttgt tgtttgtgtt tgggttttgt 8700
tgttatgttt agggtttgtt tgtattgggg taggtagggg gtatagtaag ttgttagtag 8760
ggtaggaggt tggtaggagg ttagtagatg tttatgattt ttggggtgta gttatgtgtt 8820
agatgttgtg tgatgtgggt attgatttgt aatattgagt tgttttttta tgggtaaaat 8880
agggtaagta tatgggtttt tttgggtggg ggttgtattg tggaaagtag atgttggaga 8940
gggtttggtg ggtgtggttg ttgggagtgg aatgttgggg tgttgtttta gggttattgg 9000
gatttatttt tggggtttgg gatgagtttt ttgtaaagtt ttaggtaggg gaataggttt 9060
tggtttttag tatgtatgta gtagatgtga ggttttttttt taggttgtat ttattttgtt 9120
tagtatagtg attagggtta gtgagtattt gatgatgtgt tggggattgg tttgttgtag 9180
tagttttggg agtatattag tggtgagttt gtgtagttag attgtgagtt ttattgtgtt 9240
gttgttgggt tttgggaggg tgatggttag agatttatga gtagaggggg gtggtgagta 9300
ggtggtagtt ttgggggtgt ttttttatgg tttggtttat ttgttgaggg tgattatagt 9360
ggtttttagt tggttttgta ttattatggt taggtttatt ttgaagtgtg gtttgaaatt 9420

tgggggtagt atggttttgt agttggagag gttgttttttg tagatataga attttttgta 9480
gtggttttgg tgatagtgtt gtagtagtag ggtgtatatt agtggggtgt tagtattttt 9540
tgtgttatgt tagtttgagg gatggttttt atggtattat gggagggttt tgtgatttta 9600
tagagttggg ggattttgtt gtttttttta tgtaggtttg tatttattta tgtatttgaa 9660
gtgtattttg gtggtgagag tgtgtatagt gtttagtggg aagaggtggt aagagttttt 9720
tagtggtggg tggttggggg atagggggat ggaggtgtag tttttttttt tgttagagtg 9780
gtttagtatt gttagtgtga aggtgtattt tttgttgttt tgtagtatgt tttgttgtag 9840
tattagttat atgtttgtgt tgtttgtgga tgtggtggtt ttatttagta ttagtgtttt 9900
gttgttgaat gtatgtgtag tttattgttg taggtagaag ggatggtgag ggggtgtaat 9960
tttttgtttt gttagtttta tttttgtttg taggttttgt ttttttggtt atgggattta 10020
tttttaattt gtttatattt tgtttaatat ttattttttg tttggagttg ttgttgtaat 10080
tgaggtagtg gtttttttagg tatatgtagg agttgtggtt tattttgtat atggtttgtg 10140
ttttgtagga tatatatttt aaggatataa tgggtatttg gttattgtgg attagtattt 10200
ggtgtgggag tggggttatt tttaatatag gtttatttgg tttgttggaa ggtttggggg 10260
atttgtggag gatgttgttt ttaaatttta ggttttttag gggtttggtt attgttggtg 10320
agtttatttt tttttaggat atttatttgg tttgttattt tttaatttgg gtggtggaag 10380
ggtatatata gggtagagga tattggggtg tgtgttttgg tgtattggag ttagttggat 10440
tttggtagga ggtaggtaat gtttattgag ggtttttggg gggatgtgtg tgggaataga 10500
tgtatgtgtg ggtgtgagga ttgtagttgt tatgtaggtt tgatttatag atttttgtag 10560
tttttagtta gggtttgggt taggaggttg agttgggatg gaatttgttt ttatattttt 10620
tttttagatg atttttttgg gtagattttt aattaggtta gttgaggaaa gtagggattg 10680
gggaatagat atttattttg gggtattagt aggttttagt tagggaggtg tatatgttta 10740
tagagggtag ggaggtgtat atgtttatag gtatttgttg tgtttggttt ttgaaggtat 10800
tagatgttag taaggttagg atgtatttat ttgtggggat aggtttaagt ggggtagttg 10860
tggtattttt atttgttttt tatttgtttg gtagaagttt tttgtttgag gagtttgggg 10920
tgaatggttg tatttgtggt ttagttttaa gggttttagg tttttaagtt atgtgtggga 10980
tggagtatag gtgtagtagt attgagggtt gtttggtgag gatggtattg tttttaagtg 11040
tagttgtatt tggggtagta gagtagtaag agttaggttg tggtgggggg tagtttaggg 11100
ggtttagttt ttttgtttat ttttttttatg tttggttttt tttttatttt agtaggggtt 11160
taagttatta gtttaggtga ggttatagtg agggttgttg gggaggaagt ggggtagttt 11220
gattggggga ttgggggggt tttgtgttta gagtttgaaa ggtagtggtt tttttatttt 11280
tttatttttt atttggggta gtgtaggtgt gggtgatatt gtgttttatt agtatttggg 11340
ttattgaggg gtttggaatt gggaaggatt tgttgtatgg aggttggaat tggtggaggg 11400
tttgtttttt atttttaaag gtttatttgt tggggtggtg ggaggtagtg agtgaattgg 11460
gataggggtg tgtagtggtg gggtataggt gtgtggtggt ggggtagggg gtgtttggga 11520
tttagttgag gttttatttt gtagttatgt tttgggtttt tatttagggt ttatttggtt 11580
gtgttgaggt ttttttttggt ttttgtgtag ttttagggtt tttgtgtatt tagtattttt 11640
taatagatag ggaaattgag gtttagaaaa gtaatttttt gatgtggggt tttttggttg 11700
aggttggagt tgggataaga gtttggtgtt tggataagag tttggtgttt attttaaatt 11760
ggtttttgag ttatttatag gaaggttatt tttatggttg agtttattag tatgtttgtt 11820
gttagtgtta gtgtggtatt gggggatagt atggttggta ttgtagagat ttgtaggttt 11880
tgtattttgt ttatttgttt tatggtgatg ttgtagttta tggtgatgtt gtttatgtgg 11940
ttggaggttg ttagttgtag ggataggtat tagtgggttt aggtggtagg tgagaggttt 12000
gttttgtttg gtgttttttt ttttatttat tatagttatg gtagtgtttt tgggtagtat 12060
gaagtagagt agaaggtaga ggtgaaggtg gagtttgttt ttgttttgtt ttattttttt 12120
tttttttttat ttttgtttat ttattgagag tttgaagatt gttgtgtttt gataaatgat 12180
attgaagatt atgttttgga aggttaggga ttgtttgttg ttgatggttt attggaagat 12240
tatgtttgag ttggttttta ttatggggtt gtatttttgt ggggggattg gtgttagttt 12300
gggttttgtg gaggattttg tttttagttt gttgtttttt ggatatattt tttgttgggt 12360
tggagagttt tatgtggggt atagaggaga ggaggtgttt ggggttttgt atgttatgag 12420
agttaagttt gggttgggat attgattgtt tggtttttta gttagttatg tagtattatt 12480
aatgttttaa tttttttgtg ttttagtttt tttatttgta aagtaaattt agtattagtt 12540
ataaagagtt tatttttttt tgagtttttt tagatttttt tggggttttt gttttagttt 12600
tttagttaga gagtttggag tagtgagggg aggtatatgg gttttatagg gatagtattt 12660
atattggttt atagaattta ggataggttg tataggttat gttattttttg atggtttttt 12720
ttaaggtttt tggtgaaggg gtaggtattt gtaagatgga atagttttgt tttttatgta 12780
tttagtatgg tggtattgtg ggtagtttgt agtttttttat taggggtttg gattttattt 12840
taaaagttat ttgttttagt taggtgagaa tatagtagag ggtgtgagag atttatgggg 12900
atttgtgtga ggttagggag tggagtttta aatttatttt gtgaaatgag atggtggaat 12960
gagttagtgg agttgttgtt agagttgtga tttttttagga atttaaagtt tattaggtag 13020
tttgaggagt tagttagtag gatttgtttg gggttgatgt ttttagtaat tgggttgttg 13080

tttttattgg gaagttaggt tttaagtttt gtgtgagtat tttgtttgta ggtatttgtt 13140
tgggggttgg tggtggagtt ttggttatat ttattattgg gattttttgt agtatatggg 13200
ttttggggtt gggtgtggtg tggaggttat agatgggttt ttttgttgtt atttgtaggt 13260
tgaggttggt ttggttggtg ttgtttttta ttataatgtt tattatgtgt ttttttttat 13320
tgagttatgg tagtgttatt attgagaata gggtattatt ggttttttag gggtagttgg 13380
gtatgaaggt ggttattagg gtagggtagg tatttttaaa gtgggtgttg atattgtttt 13440
taggttagtg agttgtggtt gtggtgttgg tattagagtt tatttggagt attgaggttg 13500
agttgttggt gggtatgtag aggtggttgt tgtggggggt agggtagatg atttgtagtt 13560
tagttattgg ggagattatg ttaaagttgt aggataggtt gtgtttggat atgttattgt 13620
ttatttttgt ttggatttta tagtgtttag gtagttgtag tttagggttg ggttttaggt 13680
ttagtagtat ggtgagttgt tttgtggttg taagtagttg tagggtatag gtagggtagg 13740
tttaagtgtt ttttagttgg gttggtaagt ggggtagtta tgatgaggtg ttggtggaga 13800
ggtatggggt ttggggatta gggtggttgg gagttggtga gtagtgtagg agggtgttag 13860
ggttagtgtt gtgttgtaag ttaatgaggt tattagggag tatgaggata ttttggttgt 13920
ggagggtgat ttgtggagag ggaggtaggg ttgtattatg tttttatggt tatggtttgt 13980
ggatttttgt atgatggatg agggtggata tgtagggttt tttgttttgt tagttatatt 14040
ttagggagtt tttttatagt gtttgtgata aggataggta ggatagttgt agatagggggg 14100
atatatgggg agaggatata ggttaagatt tggtagatag gaaggagtgg ttgtgttggg 14160
agagaggaag aggaggtata gtttgtgtta agggtttagg tgagagtttt ttttattggg 14220
agaggggtaa gggtattgta gaggttggag gttggaggtt ggaggttgga ggttagaggt 14280
ggtaaggatg tgggaggggt ttgtaggttg ggtgtgtttg ttgtgtagat ttagattttg 14340
ggtagtagat aggaaggtgg tttgaggaga tgtagggaat agatttaggt tagggttata 14400
tattgagtat tgtgtggggg gttttgtggg aatggagaag aggaattttt tttatagtgt 14460
ataggggggtt ttgagtagtt ttggttttga tgtgtagtta ttggtgtagg tttgggggtg 14520
gtaggaggtg tttagtggta agtagatgtt ggttttaggg tattagtgtt tttttggtat 14580
gtatgtgggg attagttggg ttttgttgtt tggggatttg tttttaggtt tgttgttgtt 14640
ttttggggtt tttgtgagga ggggagggtg ttggggtttt tatttgttta tgggttattg 14700
ttagagatgt ttaattgttt gtattagtga gtttggtttt gttgtgagga taggtttttt 14760
tgtttgggta gtatttttag tttagtgttg tgtttttgtt tttggttagt tgattgattt 14820
aggttggttt ttaggtaggt tttatttgat ttatttttag gatatttgga atgagttggt 14880
gtttttggaa tttttgtttt gtttatttaa gattgggaat tattttgatg gttataggat 14940
tagtagatgt gagagtttag agaggttatt ttgagttttg tggtgtttat tattttagag 15000
ttttatttgt tgtgttgagg agttggtata tttgtagttg tagttgggtg ggttgttgga 15060
gtttttgggt tttaaatttg gttgtggtga ggaaggtttt atggtttaga tgtaggtttg 15120
ggaatattat gatttggtgg gtagggggtt gttttagttt tatagatttt attttagttt 15180
gaagtttaga ttttttttat ttgaattttg taggaagagg ggagggaagg agagtgagtt 15240
attggatttt tataggtttg gtttttgttg tttgagagga agattttgat ggaaattgtt 15300
tatgggggt aggatttttg atttgttttt taggaataat ttatttatat ttagagaaaa 15360
ggtttggggg taatgtgagt aaatgttttt ttttttgtat tttggatttt tttaattatt 15420
tttattgggt ataagtaata ttaaggtttt taagtttttt ggtgagattt atagtgggta 15480
gggtaggtga ggtttttagg ggtaggtagg agggtaggtt atagaatgtg gggggttgat 15540
tatttttatg ggtttgtgtg gggttgagag gttgttttgt tgtgttagag gtattagggg 15600
tttttatagg tttttattgg gtgtttttat gaggaggttt ttggtatttt gtattgggtt 15660
tggggtggta agtgtatagt gaggtgttgg gttagggttt aggatattag gatgaataga 15720
ttggggattg agttgtttga gaattttttt attagttttt tttttttagt ttaggtttta 15780
ttgtgggtgt ttggtaggtt tttaattata gttagtgttt taggtttttg tttggttttt 15840
tgtatatttt taggttgtag tttgtagatg tagttgtgtg gtgttgagta taggttggtg 15900
ttatattatt tggtgggttt gagttggatg tagtgtttgg ttgtggttgg gtgtggtttt 15960
ttgggtagtt agttttggta gtttttttagg ttgaaggttt tgttttgtgg tgttgggttt 16020
atttttattt tttgtatagt tgagaagttg atttatatgt ttaggttttt gggggtgggt 16080
gtgggatggt agggggttta gggtattttt ttattttttt atttttatag tagtttgttg 16140
ggagttttgt tattgttttt ttttttagat ggggaaattg aggtttagag tttggagagt 16200
agggtttatt agtttaggtt tatagtagta tttatttatg gggtttgtgg gtattggtag 16260
ggatttttgt gtaggttatt ttttgtatgg tgtgtttagg agtgtttgga ggttgttttt 16320
agtttgtgtt tatttttgta tttgtagagt tgataggaat ggttttattg gttggtgtta 16380
tttgtttatt agggttggtt tagtttttat tttttttttt ttgagatttt ttagttgtag 16440
gttttgtttt attgttttag agatttttta atttatggtt ttttgggggg tggggtaggt 16500
atttggtgat ttgggagatt aggaagtgtt gtatggtggg attgtttatt attgttaggg 16560
tggttttggt ttaggtttga tattgttttt gtgtttgtag ttaggttgtt ttttttatta 16620
ttaggtggta gtagtgttta ttgttagaga agatttttgt gtttgagggg tagagtgggt 16680
gtattgttgg agattagtgg gaatgagggt gttaatggtt agtgtgggtt taagatgggg 16740

gtattaggtt ttgttttatt tggatggttt tggggaggaa ggggagtggg tagtagatat 16800
ttattttggg ttggtttttt gtttagggtt atgatgttgt aggtggtttt taggtttgaa 16860
ttattgtggt tttggatgtt gaggttgagg tttttgttat tttgtattga ggatgggtat 16920
atgagtttta gggtggtagg tgttgttttt atttgtatgt ttgtttttag tagggttgag 16980
ttggttttta gggttagtat ggttgttatg tgatagtgtt taggtagtat atagtgatgt 17040
gaggtagttg gtttagtggt atttattttg ggggagttgt ttttgaagtt ttagtgtgtg 17100
gtagtgatag ggagtggggt agtgatgtgg aaggttgtta gttggttgga ggttaggggt 17160
ttgtggggtt ttattagggt ggtttttggg gaggtaggga agatgtgttg gaggagggtg 17220
gggtttttat aggtgggggt aggaggtggt ggggggttgg agtag 17265

<210> 280
<211> 17265
<212> DNA
<213> Artificial Sequence

<220>
<223> chemically treated genomic DNA (Homo sapiens)

<400> 280

ttgttttggt tttttgttgt tttttgtttt tatttgtagg ggttttattt ttttttagta 60
tgtttttttt gtttttttag gggttatttt ggtggggttt tatggatttt tggtttttgg 120
ttagttagta gtttttttata ttgttgtttt gttttttgtt attgttatat gttgggattt 180
tggagatggt tttttgaggt tggatgttgt tgggttggtt gttttgtatt gttatgtgtt 240
gtttgggtgt tattatgtga tggttgtgtt ggttttgggg attggtttag ttttgttggg 300
gatagatgtg taggtggaag tggtatttgt tgttttggag tttgtgtgtt tgtttttggt 360
gtagagtgat gagagttttg attttagtat ttagaattgt ggtggtttag gtttggaggt 420
tgtttatagt attgtggttt tgggtgagga gttggtttga ggtgagtgtt tgttgtttat 480
tttttttttt ttttagggtt atttagatgg ggtagagttt ggtatttttg ttttgggttt 540
atattgattg ttgatatttt tgtttttatt ggtttttagt ggtgtatttg ttttgttttt 600
tggatatgga gatttttttt ggtaatgggt attgttattg tttggtggtg gagaaggtgg 660
tttggttgta ggtgtaggag tagtgttggg tttgggttgg ggttattttg gtaatggtgg 720
atagttttgt tgtgtagtgt tttttggttt tttgggttat taggtgtttg ttttattttt 780
tgaggggtta taggttggga gatttttgaa gtagtggggt agagtttgtg gttggggagt 840
tttaggagga gggaggtggg agttgggttg gttttggtga gtaggtggtg ttggttggtg 900
gggttgtttt tgttagtttt gtagatgtag aggtggatgt gagttggggg tagtttttgg 960
atatttttgg gtatgttata tgggaggtgg tttgtatggg gatttttgtt ggtgtttata 1020
ggttttgtgg gtgggtgttg ttgtgagttt gggttggtgg gttttgtttt ttgggttttg 1080
agttttagtt tttttatttg gaaaggggga tagtgatggg gtttttagtg ggttgttgtg 1140
agggtgggag gatggaggag tgttttgagt tttttgttat tttatatttg tttttaggag 1200
tttagatatg tggattggtt ttttgattgt gtagggggtg gaggtgggtt tagtgttgta 1260
gggtgaggtt tttagtttgg agagttgtta gaattggttg tttggggagt tatatttagt 1320
tatagttgag tattgtgttt ggtttgggtt tattgggtgg tgtaatattg atttgtgttt 1380
agtgttgtat agttatgttt gtgagttgtg gtttggaggt gtgtgagggg ttaggtaggg 1440
gtttgagatg ttggttgtgg ttaggggttt gttgagtgtt tgtggtggag tttgggttga 1500
ggaggagggg ttggtggggg ggtttttggg tggtttggtt tttagtttgt ttgttttggt 1560
gttttgggtt ttggtttggt gttttattgt gtatttgtta ttttaggttt agtgtaggat 1620

gttgagaatt tttttgtggg agtgtttagt ggggatttgt agggattttt gatgtttttg 1680
gtatggtagg atggtttttt agttttgtat gagtttgtgg aggtagttgg ttttttatgt 1740
tttataattt gttttttttgt ttgtttttgg aggttttgtt tgttttgttt attgtgggtt 1800
ttgttaaaaa atttgggggt tttaatgttg tttgtgttta gtgaagatgg ttgggaaaat 1860
ttagagtgta gagaggaaag tgtttattta tattattttt aggttttttt tttgagtgtg 1920
ggtgagttat ttttgaaagg taggttaggg gttttgtttt ttatggatag tttttattgg 1980
agtttttttt ttgagtgata ggagttaggt ttgtgggggt ttgatggttt gttttttttt 2040
tttttttttt ttttgtgaag tttgggtggg gggagtttgg gttttaggtt gggatggggt 2100
ttgtggagtt gaggtggttt tttgtttatt aggttatggt atttttgggt ttgtgtttga 2160
gttgtgaagt tttttttatt atggttgaat ttgggattta ggagttttgg tggtttgttt 2220
agttgtggtt gtaggtgtat tggtttttta gtatagtagg tgggattttg gggtggtggg 2280
tgttgtagga tttggggtgg ttttttttgag tttttatgtt tgttggtttt gtggttatta 2340
gagtggtttt tagttttagg tggatagagt aggggtttta gagatattag tttattttag 2400

```
gtgttttggg ggtggattgg gtggggtttg tttggggatt ggtttgggtt agttagttgg    2460
ttggagatag ggatgtagta ttgggttggg agtgttgttt gggtggggag atttgttttt    2520
atagtaaggt taggtttgtt ggtgtaggta gttgggtatt tttgatggtg gtttgtgggt    2580
gaatgagggt tttaatattt tttttttttt gtagggattt tggagaatgg tagtgagttt    2640
gagagtaggt ttttggataa taggatttag ttggtttttg tgtgtatgtt aggggggatgt    2700
tggtgttttg gagttaatat ttgtttgttg ttggatgttt tttgttattt ttaggtttgt    2760
gttaatggtt gtatgttagg gttagggtta tttggggttt tttatgtgtt atggagagag    2820
tttttttttt ttgtttttgt ggggtttttt gtgtagtatt tggtgtgtgg ttttgatttg    2880
ggtttgtttt ttgtattttt ttaggttatt tttttgtttg ttgtttaggg tttgggtttg    2940
tgtagtaggt atatttagtt tgtaggtttt ttttatgttt ttgttatttt tgatttttga    3000
tttttgattt ttaattttttg atttttgtag tgtttttgtt tttttttttag tgggagaagt    3060
ttttgtttgg gtttttggta tgagttgtgt tttttttttt tttttttttag tatagttgtt    3120
tttttttgtt tgttaggttt tggtttgtgt tttttttttg tgtgtttttt tgtttgtaat    3180
tgttttgttt gtttttgtta tgagtattgt ggggaggttt tttgaggtgt ggttgatgaa    3240
gtggggagtt ttgtgtgttt atttttattt gttgtgtagg ggtttatggg ttatgattgt    3300
gaggatgtga tgtagtttttg tttttttttt tataggttat tttttatagt taggatgttt    3360
ttatgttttt tggtgatttt gttggtttgt agtatgatgt tggttttggt gttttttttgt    3420
attgtttgtt ggtttttggt tattttggtt tttaggtttt gtattttttt gttaatgttt    3480
tgttatggtt gttttatttg ttagtttagt tggagggtat ttgggtttgt tttgtttgtg    3540
ttttgtggtt gtttgtagtt atggaatagt ttattgtgtt gttgggtttg aggtttaatt    3600
ttgggttgtg gttgtttggg tgttatgagg tttgggtaga ggtgggtaat ggtgtgttta    3660
ggtataattt gttttgtagt tttgatgtgg tttttttagt ggttgggttg tgggttattt    3720
atttttgtttt ttgtgatggt tgtttttatg tgtttattaa tggtttagtt ttggtgtttt    3780
aggtggattt tggtgttagt gttatggtta tggtttgttg gtttgggggt agtgttagtg    3840
tttgttttga gaatgtttgt tttgttttgg tggttatttt tgtgtttggt tgtttttggg    3900
agattaatga tattttgttt ttagtggtag tattgttgtg gtttggtgag ggggagtatg    3960
tgatggatgt tgtggtggaa aatagtgtta gttgggttaa ttttagtttg tgggtgatgg    4020
tggaggagtt tatttgtggt ttttgtgtta tgtttagttt tgaggtttgt gtattgtagg    4080
gagttttagt ggtgagtatg gttgaggttt tattattagt ttttaggtag gtgtttgtag    4140
atagggtgtt tatatagggt ttgaggtttg gtttttttagt gagggtagta gtttagttat    4200
tggggatgtt ggttttgggt aggttttgtt ggttggtttt ttaggttatt tggtgggttt    4260
taaattttttg gaaagttatg gttttgatag tggttttgtt aatttatttt attgttttat    4320
tttatgaaat gaatttaaaa ttttgttttt tgattttata tgagttttttg tgagtttttt    4380
atgtttttttg ttgtgttttt gtttggttaa agtaagtggt ttttgaggtg gagtttgaat    4440
ttttgatggg aaattgtggg ttgtttgtag tgttattatg ttgggtatat gggggatagg    4500
gttgttttat tttgtgggta tttgtttttt tattaggggt tttgggaggg gttattagaa    4560
atggtgtgat ttgtgtagtt tgttttgggt tttgtaagtt agtgtaggtg ttgtttttgt    4620
gaggtttgtg tgtttttttt tattgttttg agtttttttgg ttgaggagtt ggggtaggag    4680
ttttgggagg gtttgagaag atttagagag aggtggattt tttgtagttg gtattaggtt    4740
tgttttatag atggggaaat tgaggtatag agaggttgag gtattagtag tattatatgg    4800
ttggttggag agttggatag ttagtgtttt agtttgggtt tggtttttat ggtatgtaga    4860
gttttgggta tttttttttt tttgtgtttt gtgtgggatt ttttagtttg atgggaggtg    4920
tgtttaggag gtgataggtt aagggtagag tttttttatag agtttaggtt gatattagtt    4980
tttttgtaga ggtatagttt tgtggtggag gttggtttgg atatggtttt ttggtggatt    5040
attaatgata agtagttttt gatttttttag aatgtggttt ttaatgttat ttattagagt    5100
gtggtggttt ttaagttttt agtaggtggg tgggagtggg gaggggaggg gatggggtgg    5160
ggtgggggtg ggttttattt ttatttttgt tttttgtttt gttttatgtt gtttgaggat    5220
gttgttatgg ttgtggtgag tggagggagg gatgttaagt agggtaggtt ttttatttgt    5280
tatttgggtt tattgatgtt tgtttttgta gttgatggtt tttaattatg tgagtaatgt    5340
tattgtgaat tataatatta ttgtggagtg gatgaatagg atgtagggtt tgtgggtttt    5400
tatagtgtta gttgtgttgt tttttaatgt tatgttggta ttgatggtgg gtgtgttggt    5460
ggatttggtt gtggaggtgg tttttttgtg agtgatttag gggttggttt ggggtgggta    5520
ttaggttttt gtttgggtat taggttttttg ttttggtttt agttttagtt gagggatttt    5580
atattagggg gttgtttttt tgagttttgg tttttttgtt tgttgggagg tattgggtgt    5640
ataggagttt tgaggttgta tgggagttgg gagaggtttt agtatagttg ggtgggtttt    5700
gaatggaggt ttggggtgtg attgtagagt ggagttttgg ttgggtttta agtatttttt    5760
gttttgttat tgtgtatttg tgttttgtta ttgtgtattt ttgttttggt ttatttattg    5820
tttttattg ttttggtagg tggatttttg gggatgggga gtaggttttt tattagtttt    5880
agtttttgta taatgagttt tttttggttt tagattttttt ggtggtttag gtgttggtgg    5940
agtataatgt tatttatatt tatgttgttt taggtgaggg atgagggggt gagggggtta    6000
ttgtttttta ggttttgagt atggggtttt tttagttttt tagttaagtt gttttgtttt    6060
```

```
tttttaata gtttttattg tgattttatt tgggttgatg gtttaggttt ttattggggt    6120
gagggagggg ttaggtgtgg gaggagtgga tagggaagtt gggtttttg aattgttttt    6180
tattgtggtt tggtttttgt tgttttgttg ttttgagtgt agttgtattt ggaggtggtg    6240
ttgtttttat taggtagttt ttagtgttgt tgtatttgtg ttttgttttg tatgtggttt    6300
gggagtttgg gatttttaag gttgggttgt aggtgtagtt gtttattttg ggtttttttag    6360
gtggggggtt tttgttgagt gggtggggag taggtggggg tgttgtggtt gttttatttg    6420
ggtttgtttt tataggtgag tatgttttga ttttgttggt atttaatgtt tttgagaatt    6480
ggatgtagta ggtgtttgtg agtgtgtgtg tttttttgtt ttttgtgagt gtgtgtgttt    6540
ttttgattgg ggtttgttgg tattttagag tgggtgtttg ttttttagtt tttgtttttt    6600
ttagttggtt tgattggggg tttgtttaga ggggttgttt gaggggaggg tgtgggagta    6660
ggttttattt tggtttagtt ttttgattta ggttttggtt aagggttgta ggagtttgtg    6720
agttaggttt atgtggtaat tgtggttttt atatttatat atatgtttgt ttttatatgt    6780
attttttttag gggtttttag tgagtattgt ttgtttttttg ttagggttta gttggtttta    6840
gtatattaga atgtatattt tagtgttttt tgttttgtgt atgttttttt gttgtttagg    6900
ttggaaggtg gtaaattgga tgagtatttt gggagggggt gagtttattg gtagtggtta    6960
ggtttttggg aaatttggag tttgggagta gtattttta tgggtttttt agatttttta    7020
gtaggttaaa tagatttgtg ttggaggtaa ttttattttt atgttaggtg ttgatttgta    7080
gtggttgggt gtttattgtg tttttggagt gtgtgtttg taaggtatag gttgtgtatg    7140
aagtgagttg tagtttttat gtgtatttgg agggttgttg ttttaattgt agtagtggtt    7200
ttaagtgagg ggtgagtgtt gagtggggtg tgggtgggtt ggggatgggt tttatggttg    7260
aggggatggg gtttgtaggt agaagtgggg ttgatagggt agagggttgt gttttttttat    7320
tattttttttt gtttgtagtg gtgggttgta tgtatgttta gtaataagat gttggtgttg    7380
gatgagatta ttatatttat gggtagtgta ggtatgtgat tggtgttgtg gtggggtgtg    7440
ttgtgggatg gtgagggata tatttttatg ttgatggtgt tgggttgttt tggtaaggag    7500
gagggttgtg tttttatttt tttgtttttt aattgtttgt tgttgggggg tttttgttgt    7560
ttttttttat tgggtgttgt gtatgttttt attattaagg tgtattttga atgtatgggt    7620
gagtgtaggt ttgtgtaggg ggagtagtgg gattttttga ttttgtgagg ttatggagtt    7680
tttttgtgat gttgtgggga ttgtttttta ggttggtatg atgtggagga tgttggtgtt    7740
ttgttggtgt atgttttgtt gttgtagtgt tgttgttagg gttattgtga ggagttttgt    7800
gtttataagg gtagtttttt tggttatgga gttgtgttgt ttttgggttt taggttatat    7860
tttgaggtgg gtttggttgt ggtggtgtag gattagttgg gagttgttgt ggttgttttt    7920
aataggtgag ttaggttgtg ggagggtgtt tttgagattg ttatttattt attatttttt    7980
tttgtttgta ggtttttggt tattattttt ttagagttta atggtagtgt aatggggttt    8040
atagtttggt tgtatgggtt tattgttagt gtgtttttgg ggttgttgtg gtaggttgat    8100
ttttagtatg ttattgagta tttgttggtt ttggttattg tgttgaatga ggtgagtgta    8160
gtttgggagg ggattttata tttgttgtat gtgtgttggg gattaagatt tgttttttttg    8220
tttggagttt tgtggagggt ttattttggg ttttagagat aaattttagt gatttttgaag    8280
tagtattttg atgttttgtt tttagtagtt atatttattg ggtttttttt ggtgtttgtt    8340
tttttataatg tagtttttgt ttaggagggt ttatgtgttt attttgtttt gtttatgaag    8400
aaatagttta gtgttgtggg ttagtgttta tattatatag tatttagtat gtaattgtat    8460
tttgggagtt gtgggtattt gttggttttt tgttggtttt ttgttttgtt gatagtttgt    8520
tgtgtttttt gtttgtttta gtatgagtgg gttttggatg tggtggtaga gtttaagtat    8580
gagtggtagt gttgagttta gatatgtaag aatattatgg agattttggt gtttttgagg    8640
gtttatattg tggatgatat ttagtagatt gttgttgtgt tggtttagtg tatggtagga    8700
tggttttata tgtttttttt gttttgtatg tttgttaggg tattgggttt agttttttag    8760
ggtagatggg tagtttggtt gaggagttga gtttttagtt tgggtttttt tttgttatgg    8820
tgttttttgg tttttgattt gttttagtag ttttagttat tttgttgttt tgtgtgaatg    8880
tagggtgttt tttgggggat ttagggtgta aagaggggtt tagatgtggg gagggattaa    8940
gaagatgttg ttttgtgttt tttatttttt tttttttttt tttttttttt ttttagtttt    9000
tttttttttt tttttttttt taattttttt tttttttttt ttttttttta gttttttttt    9060
ttttttttag tttttgtttt ttttttagt tttttttttt ttttagtttt tttttttttt    9120
ttttttttta gatttttttt ttattttatt ttgttgagtt tttttatttt ttttttagtt    9180
tttttatttt tttatttttt tttttttttt tttttttttt tttttttttt tttttttttt    9240
tttttttttt tttttttttt tttttttttt tttttttttt tttttttttt tttttttttt    9300
tttttttat tttttttttt tttttttttt ttgttttttt ttttttttta tgtgaagagg    9360
tgttttgtgt ggttggtggg ttgtattatg tgttttttag gtggaggttt tgggttatgt    9420
agagttataa aaaatgttta gtgaggaggt tgtggggggtt tagttaagtg ggttttttag    9480
ttgtagggtt gggggtggga gttaggtgag gatttgtgta gagaggaggg tgtgtgtaag    9540
gagtggggtt aggagtgggg ttggatattg ttggttttat atagggggttt agtagggagt    9600
ttgtatgttg tttgtgtttg aagtagatgt tgtataagtt ggaggttatg atgtgtattt    9660
tgtaggtaga gattattgtg ggtattgtga tgtttattgt tattggagat agtattttta    9720
```

atattatagg tgttgtggtt tgtgttttat gttatttgtt tgttttatgt ggtttgtttg 9780
ttttatgttg ttttttttt tgttttttt tttttataa ttgttttgtt tttgttttat 9840
tttattttg ttttttttt tttttttaa tttttattt tattttttt tttttaatttt 9900
tatttttatt ttgtttttt aatttttatt tttattttgt tttttttaatt tttatttta 9960
tttttttttt ttaattttta tttttatttt tttttttaa tttttatttt tttttttttt 10020
tttttttttt attattattt ttttttttta ttttttttt tttttttta tttttttttt 10080
gattttttt gtttttttgt ttattttttt tatttttttt atttttttta tttttttttt 10140
tttttttat ttttttttt tttttttatt tttttatt ttttttttt tttttatt 10200
tgttttttttt ttttttttt tttttttttt tttattttt ttttttttttt ttttttttt 10260
tttttattat tttttttatt tttgtttta tttttatttt tttttttttt tttttttatt 10320
atttttttt tagttttttt ttttttttg tttgtatttt gtttttttgtt tttttaggtt 10380
ttttttttt tttagtttttt atttttggt ttttttttt tgtttgtttt tatttttttt 10440
ttgttttttt gtttttgtat tgattttatg tttgtttgta ggagatttta tttatttggt 10500
tagtttagat gtgtgggtat tgtagtgttt agagttggga gttgagttat tattgtggat 10560
ggtggtgttt taggtttata atttgatttt tgtttttatg tgtattttta tgtgtttttg 10620
tgtgtttaat gaggagtttg tgatgttggt gggtgaggag attatggttt agggtaagtg 10680
tttggatttg tggagtttgt tgtgttatgg tggtgtttta gggtttggtt gttattttt 10740
tattttttag gtttttagta gggtttttgt taattttagt gatgtggtgt agtttgtttt 10800
tttggtggat tttaatttttt tttttttgg ttatattagt aattatattg tttttattaa 10860
ggtggttttg atggtgtttt agatataggt tggtgtttag atttttattg agtggttggt 10920
tttagagtgt gttattattg tgaaggtgtt taataatttg gattgggttg tttggggtta 10980
ttgtagtttt gttaatttttg ttgtggttta gttttaggtt tttgttggtg ttgtggttat 11040
tttggatagt agtagttttg tggttgtgtt gtatttgtag tttaattata tgttgttgga 11100
tggtgtgtgt agtgggtggg gtatatgtgg tttttttggtt ttgtttttgg ggggaaggtg 11160
tttttttgtag ggtttttatg ggtgtttttg gtgaaatttg ttgttttatg ggttgttggg 11220
ggtttggttg gagaggagtt gggggttatg gagaagtagg tgttagtttt ggtgtagagg 11280
tttttatggt tttttaggtt tgtggtagag ggtgggttta ggagggttat tgtgggtgtt 11340
ttttgggtgg ttgagttttt tggtaggtgt gtgatttgtg tgttttgttt taggttgtta 11400
tttgtttgag gaatttgagt tttatttggt agtttatttg tatttggagt tttggtttaa 11460
tgagtgtaat tgtttggtta gtaggaggat ttgtttagag tttttttagg gtgttgatta 11520
ttggttttat atttttttta tttttttggg gtgagttttg tgggttggtt tggtagggta 11580
gggtagggta ttatgggtta gtattgtttg ggttatgggt tttgtgggga tggtaggtag 11640
tgaggggatt ggattgggta tgggttttgg gattttgata tttaatttgg tggagtttgg 11700
gtttatgttt attgtttttt tttttttttag gattagagat ttagtgggga gttattgttt 11760
gaattttttt agttattttt gttggttggt gttggaggtg tttgtgggtt tgtatatgtt 11820
tttgtgttag tattttagtg aggaggatgt ggtgtggtgg atagaggggt tgttgttttt 11880
ggaggagatt ttgttttgtt aggttgtttg ttttatttgt tattttattg tttttggtgt 11940
tagtttttttt atgtttttaa gttatgtatg ttttgtgttt tttgtgagtg attttgtgtt 12000
tttgggagtt tttgtagagt tgaggagggt ttgggtgggt ttggttttat tttgagaagg 12060
tatagtttgt atgtgatttt ttgggtttgg tggttgtgtt tttataggag ttgatagtgg 12120
atgtaaatta tattgttatg ttgatatgtg ttgtgtgttt ggtgatttat atggttatgg 12180
ttgttatttt gtataagttg gattagttgg atgttagttg gggttgtgtt atttttttttt 12240
gtgggtagtg gggttgtttt aagtatgaga tttttgttaa gataggttgg ggttggggtt 12300
taggtgaggg gtgtggtggg gtggtagggt tttttttgtt tttattggtt gtgttggttg 12360
tattttttgg gagtgagttt tgttgtaggt gttagaataa ggtagttttt gtagtgttgt 12420
gtgaagggtt tgtgtgttta ttttgggaat gattttgtga gtatttattg tttttgagga 12480
ttaggatagt ttttagttgg aagtaggtgt tagttagtta gggtgggtag tttatgtttt 12540
gtatagtagt gtggttttat aagtgatatg agtattgtta ttattgtggg agattatgta 12600
tttatttgtg attttgattg tatagtttgt tttttagatg gaggtgttag tatttttttt 12660
gtggttgttt ttttaatatt tttatttttt tttattggaa ttgttttttt ggtatttttt 12720
ttttgttttt gttttttttt ttttggagat ggagttttat tttgttgttt aggttggagt 12780
gtaatggtgt gattttggtt tatagtaatt tttagttttt gggtttaagt gatttttttt 12840
aagtgatttt tttgagtagt tgggagtata ggtgtatgtt attatattta gttaattttt 12900
tattatgtta gttaggtgaa tttttgattt taggtgattt gtttgttttg gtttgttaga 12960
gtgttgggat gataggtgtg agttattata tttggttgtg tttttatttt ttatttttgt 13020
gttgttttta tttttatttt ttagtttttt tgattattta tttttaaaaa ttgttggttg 13080
ggtgtggtgg tttatatttg taatttgagt attttgggag gttgaggtag gtaaattatg 13140
gggttaggag attgagatta ttttggttaa tggtgaaatt ttgtttttat taaaaaatat 13200
aaaaaaatta gtttggtgtg gtggtaggta tttgtagttt tagttttttg ggagattgag 13260
gtaggagaat ggtgtgagtt tgggaggtgg agtttgtagt gagttgagat tgtgttattg 13320
tattttagtt tgggtgatat agtaagattt tattttaaaa aaaaaaagaa aaaaaatatt 13380

gttatttggg tttgttattg ggagaggagg tgatatagtt ttatgttttg tagtttgtgt 13440
atgaattgag ggatgggtgt gtggtgtggg ttattggttg tggtgtgatt gaggtgtgga 13500
taggtgtgta gtgtgggtta ttggttgtgg tgtggattga ggtgtgtgta gttatgtttg 13560
tatgttataa gttatagttt tttttgtgta atttaattat gtttttgagg ttttgttgtt 13620
tattggataa attgtagtaa ttgtagtttt ttgtgtatgg tagagttgtg taaagttggg 13680
attgtttgtg tggtttttttg agtgtgtgga ggttaaagtt gagatgattt gtttgggatg 13740
ttatatgtgt tgggtagtag attgagtttt ttattttttt tttttgtttt ttaggtatta 13800
tggtttatgt gggtattatg ttgtatgggg tggatagttg gagtggttat tggtatttgg 13860
atggtgatag agtttttttat tgtaatagtt tggatatttt ttggattgtt attttgtata 13920
gtttgggtag tgtgtggaag atttgagtgt ggtatgataa taaaggtttg tgtggatttt 13980
gttaagtttt gttttttttgt ttttgtattg gggtgttttg tgagtttgat tttttttttg 14040
tgtttttgta gggtttagtt ttgtttggtt tttgtagtat attattgtta gggatttgta 14100
gatggtatgt agtatttttt ttttggttaa tgattggttt ttggtggaga tggaggttaa 14160
tgggggtttg gtggagaagg aggtgttggt tgtgagtaag gttttgtttt atgtttttat 14220
tttgtgggag gttgggtagg gtggttttgt tttgtggttt tttgtagtgt ggtttttttt 14280
gtttttttagg ttatgtagtt ttgttgtgtt tttggtgttt gttggtggtt gagttgtagt 14340
gtggtttttt tgataagtat atttggtttt ttatatggga ttggttattt tgtagttgtt 14400
ttatttgtat ttagagggtt atttgttgtg ttttttttat ttgttttttt ttgggtgtta 14460
atgttgtgtg gtatggggtt gttggtgatt ttgtttatag gtgggtgttg taggggttgg 14520
gatagttttt ttttgtttag ttttttttgt tttttagttt tatttgtgtg gttttttttt 14580
ttttatatag tatggggtat gtgtttaggt tgagtttgtt gagtgttgat atagttgttg 14640
ttggtttggt gtttagtgtg gttgtttatt ttgtttattt ggttattttt tttttttttt 14700
ggatgttttg gagtaaggtg ggttggggtt ggggatttgg gagtattggg aatggagttt 14760
gggttttggt attatgttta gggttgttat tttttagtgt tgtagttaga gggaaaggtg 14820
tttattaaag gttgtttggg aagggttaat atatttgagt agttttagtt agattgatta 14880
gggagaaaga gagaagattt agaagttaga attgtgaaag aatgagggta ttttgttaag 14940
tagatgttat ggataattgt atagtagtat gttagataat ttagaagaag taagtatgtg 15000
gttgtgtatg tttttgaaat gtattttaga agaaaatttt agtatattta tagtaagtga 15060
agaggttgag ttagtttttt agaaattttt tagtggttgg gttgggtgtg gtggtttatg 15120
tttgtaattt taatatttta ggaggttgag gtgggtggat ttgagtttag gagtttgaga 15180
ttagtttggg taatatagta agattttatt tatataaaat attaaaaagg gttaggtatg 15240
gtggtttatg tttgtaattt taatattttg ggaggttgag gtgggtagat tagttgaggt 15300
taggagtttg agattagttt ggttaatata atgaaatttt gtttttatta taaatataaa 15360
aatttagttg ggtatggtgg tgggtgtttg tagttttagt tatttgagag gttgaggtag 15420
gagaatggta tgaatttagg aggtggagtt tgtagtgagt tgagattgtg ttattgtatt 15480
ttattttggg taatggagta agattttatt tttaaaaaaa agaaaaaaaa aattttataa 15540
agaaaagttt gggtttagag tttttatgat agaatttttt taagtagtta aggaagaatt 15600
aatattaatt ttttatagat tttttttaag aatatagtag gtgggaattt tttttattta 15660
tatggaaatg ggaggttgta ttttttagga atgtatatgt ggggttttta agaggttata 15720
tgtaaattaa ttttagtagt atatagagaa ggtgtataag ttgtgattag gaggggttgt 15780
ttttgagttt gtggtaggaa ttagaggtta tatgtggttg tttgtattaa agttaattaa 15840
aatggaatga tggttgggtg tggtggttta tatttgtaat tttagtattt tgggaggtgg 15900
aggtgggtag attatttgag gttaggagtt ttaagattag tttggttaat atagtgaaat 15960
tttgttttta ttaaaaatat aaaaaaattag ttgggtattg tggtaggtat ttgtaatttt 16020
agttatttag gaggttgagt taggataatt gtttgaatgt gggaggtgga ggttgtagtg 16080
agttgagatt gtgttagtgt atttttgttt gggtgataga gtgagatttt attttaaaaa 16140
aaaaaaaaaa atgaaattta aaattttgtt ttttagttgt attagtttgt tgttaagtgt 16200
ttagtggtat atgttgtgag gggttgttat tatggatggt gtagatgttt tatatattta 16260
gtattttagg atattttgtt agatggtatt gggttttgtt ttgtttgttg aggaggtggt 16320
tttttatttt tgttttgagt aggtttgagt tgttgtttgt tgattattgt tgttgttttg 16380
taggtggttg ggagtttgag ttttatattt gttgggtagt aggtgttgga tattgatagt 16440
tgtttggatt tgtttgtgtt ggatagtttt ttttttatgt ttttaggttt ttatgttgag 16500
gtgagggttt tattgggggt tttgggttgg gttgggggtt ttgttgtttt ggtgtagttt 16560
ggattttaga tattgtgtat tttttagtag gtttttgttg gataggtgaa gagtgatttg 16620
tttttggatg attttaagag gtgggttttt tagagaaatt ttgagttttg gtgtaggtta 16680
ttgtgtttgg agtattgggg gtgtgtgggt tgtgtgtttt tgttgggtgt ttgtggtttt 16740
atgtggttat attatgtggg agtaggtttg tttggaagtt tagggtgttt gtgtgtgatt 16800
ggatgggggt gggttgtgtg tgtgatatat tttttggtat tttgttgatt tgtgttattt 16860
gtagtttggt gtgttggttt tttagtgagg gaatgtttag ttggttggat ttgtttagtg 16920
atttgtttat tgtgggtagt aatttgtggt ataattttta tttattgggt tttttttttt 16980

```
ataattaata taattgaaat ttagggtttt tttttttttt tttttttttt tgagatagag   17040
ttttatttta ttttgttatt taggttggag tgtaatggta tgattttggt ttattgtaat   17100
ttttgttttt tgggtttaag ggattgtttt gttttagttt tttgagtagt tgggattata   17160
ggtgtgtgtt attatgtttg gttaattttt gtatttttgg tagagatggg gttttagtat   17220
gttggtgagg ttggttttga atttttaatt ttgtgatttg tttgt                   17265
```

<210> 281
<211> 2204
<212> DNA
<213> Artificial Sequence

<220>
<223> chemically treated genomic DNA (Homo sapiens)

<400> 281

```
agggataaat ttatttaaat tttttttaa tttgttattt ttgggatttt aattagaatt      60
gggttgtttt tttgtggttt ttttgatgtt ttattttaa ttagtttata gtttttgggt     120
tttgtttttt tttttattgt atttttaaaa tgtaaggtgt aatgataata gtaatgtttt     180
agttattttt gagatttttt aaattttgga tttgtttttt tttttttttt taaagttttt     240
tgaatttttt ttagtggata aatgaaggga ttgaagttgg ataaaattat aatttaggag     300
attagggatt tattttattt tgttttttgt gtttggaatt agataattta ggatgttggg     360
tttgagaagt ttggataaaa aagttaggaa aaattgagtt ttgttttgat ttttagtgtt     420
gtggtgggtt ggtatttgag ttggtaatgt gtgtggtagg ttatgtaaga aaagggatgg     480
aggagttgtt ttagtgggag ttagaaagga tgtggtgttt ttggttgtaa tttttatttt     540
ttttatttag tagggtagga ggtatttaat ttggaggaga aaggggtggg ggaggtgaaa     600
tagagattgg agagttatga gggttgggtt gttgagagta ggagaatata ttgtgttata     660
tatttttatt tttttatata tgttgtagat ataaattatt gatggttttt atgtgttgtg     720
tttgtgagtg gaggtgttta aagagggggt agatgagtta tttttgaga tggaattggg     780
ggttttatgt ttgttgtttt tagtagtata attaattttt gaatatttaa attgtgtatt     840
tttggtgtat tattatttta tttaaggtta tgggttttgt tttttttttt tttttttttt     900
tttttttattt ttttttattt tttttttttt ttttattatt gggttttttt tagtagttgt     960
atgtagggga ggagtatgga atgtttttt tatagttaat tattatttgg ttgtttttt      1020
attggttgag ttttttgta ttgtagtaaa tgggttatgg attttagtaa ttagagttat    1080
gtgttattgg tgatataatt aatgagggtg tgagatttgt gtaagtaggg ggaggtatgt    1140
gttgggttgt atgtgtttgg gagggggga aggggtgggg tttattgaga gaggtggagg    1200
tgggtagata gatttggaga gatggtgaag gagttggaaa ttgagttagg gttgagttgg    1260
ttgataatag gtaaggagat ttggaggata aagtgagttg ttagggagta tttgggagtg    1320
gatttggggg ttgaatgtag attttggtat tgggagggtt tgtattatgt ggtaggtgtg    1380
agtttagaga gattggtgta gggattttga ttttgaaaga ttgggagagg gtaggaggtt    1440
agtttttatg ggggtggttt ttgtaggatt attaggtggg tttgtgtgtt tagattgatt    1500
gtttgtgggt ggtatggttt taggtggaga ggtttgttgt ggatttaggt tggtgaatag    1560
ttttggtgaa taatgggtgg aggggaaggt gtttttaag ttggtattta ttttgaattg     1620
agggaagaaa agaatggagg ttgtgttaga ttgagagttg ttttgtggtg gttagagagg    1680
gaattttaag tttttaatgg gtgggggtgg gggtagaaat gttttttttt attgtgtttg    1740
agattggtaa tattggggag ggggagaata agtattattt tatgtagtat attttaattt    1800
tttagtttat tttttaattt ttaaatttg gttttaaatt tttttgtttt gtttttagt      1860
tgtaggtttt gatttttggg tattttagga gttgttttat agtttttgtt tttggattta    1920
tggattttt atttagtgtt ttttttatt ttttttattt ttttttttg tttttttta        1980
tttttttagt gaatagtgat tttggttttt tttttgatag tgagagggag gataaggggg    2040
tttatgggtt taggttagat attgttgggt agaggggagg tttatggttt agtttgggtt    2100
ttatttgttg taggtattga ttgaaggttt ttggtaatta gtatatatta ttttatttga    2160
aatagtgggg tttggttttt tttatggtag gatttggggt gaaa                     2204
```

<210> 282
<211> 2204
<212> DNA
<213> Artificial Sequence

<220>
<223> chemically treated genomic DNA (Homo sapiens)

<400> 282

```
ttttgtttta gattttgtta taggagaagt tgaattttgt tgttttggat gagatggtgt      60
atgttggtta ttggaaattt ttgattgatg tttgtagtgg ataggatttg ggttgggttg     120
tgaatttttt ttttgtttaa tagtgtttgg tttgggttta tgggtttttt tgtttttttt     180
tttattatta gaggggaagt taaagttatt gtttattggg gaggtgggaa aagatgaaga     240
gagagagtag gaggaatagg aagaaatgtt agatggagaa tttataggtt tagaagtagg     300
ggttatagaa tagtttttgg agtatttaaa gattggaatt tgtaattgaa gagtaaagtg     360
agggagtttg aaattagagt ttaaggatta ggaaatgaat tgggagattg gggtgtattg     420
tgtgggataa tatttgtttt tttttttttt aatattattg gttttaaata taatagaaaa     480
agatattttt atttttattt ttgtttatta agagtttggg attttttttt tggttgttgt     540
agagtagttt ttggtttgat gtggtttttg tttttttttt tttttagttt agagtaaata     600
ttgatttgaa agatattttt ttttttgttt gttgtttgtt agggttattt gttagtttga     660
gtttgtagta agttttttta tttgaggtta tgttatttat aagtagttaa tttgggtatg     720
tgaatttatt tggtgatttt atgggaattg tttttataga gattggtttt ttgttttttt     780
ttaatttttt aagattagga tttttgtgtt ggtttttttg ggtttgtatt tgttatgtga     840
tgtaaatttt tttaatatta agatttgtat ttagttttta aatttatttt taaatatttt     900
ttaatagttt gttttgtttt ttgaattttt ttatttgttg ttagttggtt tagttttggt     960
ttggttttta gttttttttgt tgtttttttg ggtttattta tttatttttg tttttttttag    1020
tgggttttgt tttttttttt ttttttagat atgtgtggtt tggtatgtgt ttttttttgt    1080
ttatatagat tttgtgtttt tattggttgt gttgttggta atatgtgatt ttagttattg    1140
aagtttatag tttatttgtt atagtataaa ggggtttagt tagtagagag gtagttaggt    1200
gatgattggt tgtaagggaa gtattttgtg tttttttttt atatgtggtt gttggagagg    1260
atttggtgat gagaaaagga ggaggatgga aaaagagtgg aaaagaaggg aggggaggaa    1320
aagggtggag tttgtggttt taaataggat ggtgatgtgt tagagatgtg tggtttgagt    1380
gtttagagat tggttgtgtt attgaaggtg gtggatgtgg gatttttggt tttgttttgg    1440
gaagtaattt atttgttttt tttttgaata tttttgttta tgagtgtagt atgtggaaat    1500
tgttagtgat ttgtgtttgt aatgtgtgtg agagaatgga ggtgtgtgat atggtatatt    1560
tttttgtttt tggtggttta gtttttgtga ttttttggtt tttgttttat tttttttatt    1620
tttttttttt ttaagttggg tgttttttgt tttgttgggt gaggagggtg gggattgtaa    1680
ttgagaatat tgtgtttttt ttggttttttg ttggggtggt tttttttattt tttttttttat    1740
ataatttatt atgtatgtta ttagtttaga tgttagtttg ttatgatgtt agaggttaaa    1800
atgaaattta gttttttttg atttttttat ttaggttttt tgggtttggt gttttaaatt    1860
atttgatttt aagtatgggg aatagaatgg ggtaggtttt tgattttttta ggttatggtt    1920
ttgtttagtt ttagtttttt tatttgtttg ttggagaaga tttgaaagat tttgaaggag    1980
gggagggagg taagtttaga gtttagagag ttttagaagt aattgggatg ttattattgt    2040
tgttgtgttt tgtattttga gagtataatg gagaggagag tagggtttaa ggattgtgag    2100
ttggttgaag ataggatgtt ggagagattg taaggggggta gtttaatttt aattagggtt    2160
ttgaggatag tgaattaaaa aaaaatttaa gtgagtttgt tttt                      2204
```

<210> 283
<211> 13135
<212> DNA
<213> Artificial Sequence

<220>
<223> chemically treated genomic DNA (Homo sapiens)

<400> 283

```
tttgtgtttg tttttgattt tattttttaa attagatgtt tagtttggtg taaatgtagg      60
agtggagttt ttagggggat gtggattttt ttttttttatt tttattttgg tttttgtttt     120
tttttttttg tttttatttt ggtttttgtt tttttttttt tgtttttatt ttggtttttg     180
tttttttttt tttgttttta ttttggtttt tgtttttttt tttttgtttt tattttggtt     240
tttgtttttt tttttttgtt tttattttgg tttttgtttt tttttttttg tttttatttt     300
ggtttttgtt tttttttttt tgtttttatt ttggtttttg tttttttttt tttgttttta     360
ttttggtttt tgtttttttt tttttgtttt tattttggtt tttgtttttt tttttttgtt     420
tttattttgg tttttgtttt tttttttttg tttttatttt ggtttttgtt ttttttttta     480
taggaggagt ttgggggttt ttgtgttata ggggtgtggt ttagggtatt aggtggggtg     540
gtgggtattg ggtttagagt ggttttgggt tgtttagttt tttttttttt ttttaggatt     600
```

agtgattttt ttagttttag gatatttttt gggttagggt ttagggtaga gttaatgttt 660
ttttagatat tttttggttg taaattttag gttgggggtt tttggggata gggatgtagg 720
tgtttaagga tatgattttt taggtagtgg atgtttttgt ttgggtggag ggtatggtta 780
tgagagtagg gtttgtgttt tggtattttg gtgtttgggt tttttgtttt taaggtttgt 840
tttgttttag tttagttttt tttggttagg tttattattg ttagtaaata tttttatatt 900
gttgtttttt tagatggtta tggtagtttt tttggggttt gtgtttgtag tttttatgta 960
gtttggttgg ttgatggaaa tgtatattta tattttgttt tattttattt tgagttgggg 1020
gtttggggat tataggtttt atttgtatag gttgtatttg gggaatgggt ttgtggtaga 1080
aaatgtgtgg tgtggggtgg ggggttttaa ttaggtagtt ttaatttgga ttttttttta 1140
tttttgtttt atttttttgt ttttagtata tggtggtgga gtgtgtgggg ttagtttggt 1200
gggggtttttg ttttttgatt tgtatttaga gaatagagta ttttatggga gagagtagtt 1260
tggaatgtag tttgagtaat gttgatttta tattagtata tttagtgttt ttatgttttt 1320
gttggtttag gttttggaga ttatgatggt atttatagtg gattttgtaa aggagtgtgg 1380
ggattttgaa ggttaggtta ttttttagag gggggtttta tgttaaagta gatggtgttg 1440
gtgttgtagg gtgtttgaga tttatggtgg agtttgggtt tggtgtgtgg gaggtggtta 1500
tgatggtgtt tttttttag gaattttggg agggatttta ggatttagtg ttagggtagt 1560
tttggtaggt gtagtgaggt agtgatttgt gggggttaga tgtgggtttg ttttatgtgg 1620
gtagggatta gttaggtatg ggggtttagt ggatttgagt ggggttatag agttttggag 1680
gatttttttg tataatgaat ttttgtttaa aaatagagat taagaggaag ttttattttg 1740
tagttttgga aatgggtggt tgtgtgtttg gggtttttgt tatgtgggag gatagtttag 1800
aggtattttg gttttaggta gttggttttt aggttgtgtt ttttttattg gttgggtatt 1860
ggttggaatg gtatgaggat tagttttatt gtttttagag gggttttggg ttattgggtg 1920
tggtggtgtt ggaggagttt tgtttgttgt gggttttgga gggtatggta gtagagtttt 1980
tggttttttt aggtagagtg gtatttgttt tatagattgt gttagttttg gttgtaggtg 2040
ttggggtttt attgtgtttt ttggggggt ttggattttt taaggttttg atttgaagtt 2100
ttgaggtttt atggatgttt gtttttggtt tgataaatga gatggttgtg gtggggagga 2160
gttttaggtg gtttgggatt ttttggtgtt tgttgttggg aggtggaggg atttagagtt 2220
gaggttgtgt ttaggattgt tttgggtgtt gggtgatgtt aatattttgt gttggttttt 2280
gttaaggggt tggtttttgg gtttttgttg ttaagtgtgt ggtgtggttt tggtttttag 2340
gttagatatt gttgatatgg gtttttttttg ggaaggtagt gggatttgtg gttttgttta 2400
ggggttgttt ttgtttgatg tagtgtggtt tttgtattga aatgggttag agataggtga 2460
ggttagagtg ggatttggat agtagggata gtgtaggttt tagtttttat tggaggtttt 2520
tgggagtggg tatatggtat aggtgtttgg ggttgtttat tttgattagg tgggaattta 2580
gatgtttttt gtttttaagg tgtttttaga gtttagtttt gggaaaggga tggttttttt 2640
ttgtttggtt gtgttttttt atttatattt tgggttttgg ggtttggata agggttaggt 2700
gatttttgaa gatagagatg tgggttttag agtattatgt attttagggg gtgataagta 2760
ggttgtagtt ttaagatttt gaatgtgatt ttatttagaa gaagggggttt tgtagggggt 2820
tatgttaggg ttttaggtgt gattgtttag ggttttttgg gtaggttttg aaataatggt 2880
tagggtttat ttgagagaga ggtagagaga gaagatgtag aggtggggga gatgttgtgg 2940
gattagggga tagagatggg taatgtagtt atagatgttt gagttggtag gggttgggaa 3000
ggtagatagt gttttttttg gagttttggg aagtgtgtgg ttttgtttgt tttttggttt 3060
tggattttttg aattttagga tagttagatt aaaggtttgt tgttgaagtt ggtagttttg 3120
gggtatttgg gtattgtggg gtttgttggg tatttgtaga tagggttgtt ttagggatgg 3180
ggtatgttta gttttgatgg agaatggttt gtttttttaaa ggtatggtta tttgttttta 3240
tgatagattt ttgtataggg ggatggaggt tgggtgagtt ttgtttatgg ttttggagtt 3300
gtgtggttgt gtgttggtta tttagtttgt gtttgtggtt ttgtatataa aatgggtttt 3360
aattgggttg atttttgggt ttgtttgatt tagtattatt gaagtgatgg ttgtttagga 3420
gggtttggtg gaggttggtt gtgtttgttt ttgggtggag ttggatttta aggaggagta 3480
ggtattgggg gtttgttttg ttgtggtggg tttttttttt ttgtagattt tatgttttgg 3540
gaataggaaa gagtatggaa gttatgggga ttaaaggggg gtgggtaggg ggtgttggtt 3600
aaatttagtt gagatttaga gtagggggta ttagttaaat ttggttttgg ttttgttttg 3660
tgggtgggtt ttttggggat ttgggtaggg gtggttgaga tttagagtta ggtaaatttg 3720
tttggggata aatttataga gtattttggg attggtttag agatgggaaa ttttataggg 3780
ggatgtgata gttgttgtta tttgaggatt gtggtggggg gattattgta gttttgaaaa 3840
ggagagaaag gtagagtgag gatttgggat ggtttggtat aggtatatgt ggttagggat 3900
gtttagtttg gatgatgtgg gttatttatg gttttgttag gttgtagggt tggagttgtg 3960
ggggagaaag tggagagtta ttttggagtt taggagttgt tttaatttta gtttagtgta 4020
gtgtttggga tagtagagtg aggagttgtt ttagttttag tttagtgtgg tgtttttgga 4080
gtagtagagg ttttggggtt gtagggtttt ttttaatttt tttttgtggt gtattagttt 4140
ggtttttggt tttgttatat ttttttgggg tatttatagg tgtgggggtt tttttagtat 4200
tttaggtgag tttttgtttt ttggatttat ttttttgttt tagtagaggg gataagtttt 4260

```
ttttatgaga agtgtttttt taggttttag ggttttaggg gaagggttgt tttttttattt 4320
ttttgggagg agttttaggt atgaggtggt tttgagtttt atggttgtgg gtttttgttt 4380
ttatggtgtt tttgtgatgg gggtgttgtg gtattgtggt taatttggtt ttttgttttg 4440
tggttattag ggttgattaa gttttgagtt ttgttttgtt ttaagggtgt ttataggtag 4500
agatgttgag taggttgggt tttttggtgt ttggttgtgt aggagatttt ggtagtgggt 4560
ttttgattgg aggttgggtt ttgagatttt gagagaggtt gagggggatg gataggggtt 4620
ttatagtagt taggtggggt tgtttagaga tttgggagga ggttttagtt tggttattat 4680
agtttggtta agtgtttatt ttggggtttg aattgttggt tggattattt gtttggtagg 4740
gattttgtgg gggttggttt tggataatga gggtaggtat tatggatttt gtttagaatt 4800
tgtttttgtt tgaaggtatt atagttataa ggttgttttt ttagtgggaa ggggttagtg 4860
tttaggtttg gggtgattgt gtgtatggtg tggtgtaggt tggggtgtgg gttttgtatt 4920
agtttggtta gttttgattt taggagaggg gaggggggat tatggttgtt ggaggagttt 4980
aggtaagttt aattgttgag agttatttag ttttgtttta tttgtttttt gtgatgtttt 5040
gagggttttg gggagatgtt tttgttggaa gagggtttta gtagaggttt tgggttttga 5100
ggaaggtttt ttggtgtttt gattttttaa atttatatat ggttgttttt gggaagtgat 5160
gggttttttt atattgggat ttaaaagtta taggataggt tttgtttttt tttggttttt 5220
gttagtttgt tttggtttgg gatattttag ttttaggttt atttttatgg ttggatgttg 5280
tttgttttgt gtttttttgt ggggtaggtg ttggtttggg agatattttt gttttttttg 5340
tagtaggttt tggggagttt attggagtgt ttgaggtaga agaatttagt tatggagagg 5400
atgaggttgg agttaatgaa gaatgtgttt aggaagaggg aggtggttat gtgtggagtt 5460
tttgtaaata gatattgttg agtagatggg taggatggtg gttttggttt tttgtttggt 5520
ttttggtgtt ttttgtatat ggtagatgtg ttgttttttg aatatgtttg agttttatgg 5580
gattggttgt gggtgttgag ggttttgtat atgggtttgg gttatttttg taggtttagt 5640
tatgtttggg tatttggttg tggggtttgg gaaagttatt atttgttttg gttttagttt 5700
tttttaggag tttggatgtt gggagtgtta taggggttga ttttggttgg gttttgtgga 5760
aatatttttt tgattttagt tgtttttgaa agttaggggg tttttagttt tgtttttttt 5820
tttgggttta gtataggttt tggggttttt atatagggta gagttttaag ttggtagtag 5880
gtgtttttgt attttattta tttttatttg tttgggttag gataggttag ggttgttggt 5940
ttggggtttg ggatagttgg taatggttgt tgggtgtgtt ttgagatgtt ggggaggaga 6000
ttttttaatt ttgagatata ggtgtgtttg ttaggtttgg gggttttgtt ttttttgtt 6060
ttagtataga gttataggag gttaggagtt tatttaggtt aattttattt taaaatattg 6120
ttatttaggt tagtaatgga tttaggttag tttagggttg tgagtgtgaa tttgagagtg 6180
tgtagtgtgg ttgtgagtgt gaatttgaga gtgtgtggtg ttagtgtgag ttgtgagtgt 6240
gagttttaag tgtgaattta agagtgtgtg gtgtggttgt gagttatgag tgtgaatttg 6300
agagtgtgtg gttgtgagtt gtgtgatttt gagagagtgt ggtgtggtta ttagggtttt 6360
ttttaattat ggtttttttag agatgtggta gagtttttta tgatgtttta tattttttat 6420
ttatattagt tgattttaag ttgtaggggt tgttatgttt ttttttggtt gttttgtaga 6480
attttgagtt tgttgtggtt gtgttaaggg tgagggtttg tattggtttt gatggttttt 6540
agtttttagg atattggtag aggtggttgt ggtttgttga gatttattag gatgtggttg 6600
tttgggggtt tttgagtttt tgtttatggg gaatggtgta tttgggttag taattgtggg 6660
atagtagaat tgtgggaagt ttgtgtgtat ggagtttatg gggatattta tgtgtatttt 6720
gaagggattt taggatggaa atatgaaagt gaggggttga gggggtggag tagtttttat 6780
tatagagttg ttttgaatgg tttagtagat ttgggaagag ggggttttttg gggttgtttt 6840
ttagtggata gtggttttgg ggtaaggtgg ggatatagat agtaattttt atagtgtttt 6900
ttatagttgg agttgtgaga agggagttgg ggggagttgg gtagatttat agtttttata 6960
tttggagttg ttgtaggttg ggagggtttt gtttttatag tggatgtagt tggtgttttt 7020
gtttgtgttt tagtgtttgt aatagtttgt gtggggtaga ggatgggtat gttagttaaa 7080
gtaaaattta tattagttgg gtttttttggg aatgattttt tatttgagtt ggtgagataa 7140
tttttatttt agatattaat atgttttgga gggtaggtgg gtggtagata aggatatttt 7200
tagggagtat ggtggggttg tgagtatttt atgttgagta ttttatattg tgtattttga 7260
ttttgagtat tttgatttta agtattttat gttgtatttt ttatgttgag tattttggtt 7320
ttgagtattt tatgttgtat tttttatgtt gagtatttta gttttgagta ttttatgttg 7380
tattttatgt tgagtatttt ggttttgagt attttgattt tgagtatttt atattgtgta 7440
ttttagagag tggtaggtgg tagtatttag aatgaatagg tttgttggag ggatttgtgt 7500
tttgtaggat tgtaggttgg gtttggtttt tattatttag tttttatttt atagattttt 7560
taagaatttt tattgtaatg tttgttttag gtttttttta agattttttt tatgtttttt 7620
ttaggatttt ttaatggttt tttttaggat ttttttttta tggtttttta taggattttt 7680
gtatgttttt tttagggttt tttatgtttt tttgggattt ttttatgttt ttttgggatt 7740
tttttatgtt tttttaggat ttttttatgg ttttttttag gattttttat gtttttttta 7800
agattttttat ttgtagtgtt tggagtggtt gttttgtatt ttgtgttttt ttatgttgtt 7860
taagtaggat aggtttattt gttagtagta tagttttttg ttttaagttg attttgattt 7920
```

ttggtttatt tgttattttt atattttggt attttttttt gggtttttt tgggggattt 7980
tgtagaatta agttttattt ttttgttagg ttgatgttgt gtttgaagtt ttatttttgt 8040
ttattggtta gggtgttttt aaggatagga attgattttt attttttgat tttttaaata 8100
tttttaatat ttagtttaga gttgagtaga tatattttttg atgttgattg tggtgtggtt 8160
gttggtgtgg gttatgggtg gattatatgt tttgattttt ttttatagat tgatttgaat 8220
ataaatgggt tgtggtgata tggggatttt gtggtggaga gtgggtttga gattttggta 8280
tggttgtgat aggggttatt ttgtagatat attttttgga agaatggaag tttatttgtt 8340
tagttttatt agggtggagt tgtgtttttt tagtattggg ggtagttttt gtttttttggt 8400
attttttggt atagggtttg ggtgatttta gtgaggtttt gtttgagagt ttttgttttg 8460
gaggttgaag aatggagttt atggtaggga gttaaatatt agtgtagtat tgtttttaga 8520
agtgtttgtt gtttttttga gtggagatgt gatttggttt ggggttgtat gtagggggaa 8580
gtgtatttaa tattgtaggt ttggtttttt tttatgtgtg ttagatggtt tttattttgt 8640
gtttgtgtat ggggttattt tggtttttgg attttatgat ttgttggttt ggttttttta 8700
tgggtattgt gattgttgta tagttgtttg tatttataga gtgtttgggg tatataattt 8760
tttataggag atgttatggt tttagtattt ttgttgttat ggggtgttag gggagtggtt 8820
tttatttagt tattagggtt ttagtattag atgatgagga ttttaaggtg tttagttttt 8880
ttttagtttt tttgtttttt tttagagtgg tgtttttttt tttttatttt gtgtgtgttt 8940
tagatgttat tattgttttt ttgtgggttt tttaattttt atgtttagtt ttggattttt 9000
gtgtttagtt ttattggttg ttatagttgt tgttttgatt atgttttttt tatgaggttt 9060
ttgagttttt aggagtagtt tttttgggtt tttttattat gggagggttg gatttttagg 9120
atgggagtgt taaggttttt ggaggtatag ttggggtatt ttagttttaa gtttattaag 9180
ggttgttaag gagtggtttt tgtatttttt tattttgttt tttttttttt tggttgtttt 9240
tattggttgt ttttattagt tttaattttt tgatatttag ttgtttttag atagtttttt 9300
ttgtagtttt tttagaattt tgatgaatag ggtttgtttg tggtatgttg atataggttg 9360
gtatttttgt ggtgttttgt tgattggtat tttattttat gtattagggg ttagtagtat 9420
ttttgtttga gtatggatag gtaaaaatgt tttgggtttt gttagaggtt tttggagtta 9480
ggatagtttt gtgagaatgg ttgagtttat gggttatttt tttagatttt tttttaggta 9540
ttttttttgat tttagttttt gtttttgttt ttagtttttt gttgggttag ttttttttatg 9600
aggttgttgt ttaaggttgt ttgttagaaa ggaggtttag atgttttttt ttaagtttta 9660
tagtttttat tgttttattt atttttttagg ttttgtagta tagatgagta ttgtttttgt 9720
tattgagggg agggttttgg gtatatatat attagtggtt taggggttgg tttggaattt 9780
gtttgggata aagtggttta tttggattat atagatttgt gtttgggtag ttggtgttga 9840
tgtttattat atttatatag tatttgggta gttgggtttg gaggggatat ggtagtgaaa 9900
ttttaggatg tgtttgttag gtaggtttat gtttagagta gtagtggttg aggggtgttg 9960
tgggagtata ttttaatgtt tgtagttttg gttttttttg gagttagtat tgggtggttt 10020
tttgtttttt gtgggtttgg gagtaaggag gggttttata gttatagtgt atatttttgt 10080
gggattttgg gtgggtggtt tttaaggaaa agttgggttt ggaagagtaa attattttta 10140
ggaggtggta tttgtaggtt atggtagtgt tattgatatt ttttgtaagt ttttggaggt 10200
tttttattag gggtttttgt ttgattttat agaggaagtg tttgggtttg gttggtattt 10260
taggggatga atttatgttt aggtgtttag gtattgtata ggtgtgttgg gtgttggtat 10320
ttatggtgtg tgtgagggtt tattttttga ttataggtag attggatagg tttgggggt 10380
ttagggtatt tttagtttgt gtgttgatta tagttgtgtt gtttttgtg atttgaatgt 10440
agtgaatggt gagggtgttt aggggtgatt gtggagtgtg ggatagaatt tatattgtta 10500
gggaggggtt taggtttggt tttttaaatg ttttagttag gatagtgagg ggtttttatt 10560
ttttatttgt tttgtttggg agtttggagt tgttgtagtt atattttttg atagtgatgt 10620
gtggtatgtt gtgttgttat tgttggtaga ggtgttttta ttgtgggtta ggatgtttta 10680
ttgtgggggt tttgtttgat ttagttattt ttgagtaggt gtttatttag taggtagtgg 10740
agggtttttt tttgtgaggt ataggttttt ttgattttag tagtggattt ggttagtatt 10800
tttggggttg ggatattagt gttttaaagg tggaagggtt agattttttta ggggaagggt 10860
ttattgtttt ttatagaggt tttggtttag gttgtgtgga tatgtgaggg gggtatttat 10920
tgtgtttttt atggatttgt tggtgatttt tatgagaagg ttagttagga gggtgaggtg 10980
ttgtagtgtt atgttaggag tagatgtgta gagtttgtta tagggaggag tatgtggagt 11040
taaagagatg gagtggggtt gaggtagggt gggtggggtt aaatgaaagt ggggttaaga 11100

gatgttgggg gggtgtgggt taaggtaagg agggtagagt taaatggaga tgggtggggt 11160
tgtggtggag ggtggggtta aatggaagtg ggtggggttg tggtggaggg tggggttaaa 11220
tggaagtggg tggggttgtg gtagggaggg tggggttaaa tggagatggg gtggggtggg 11280
gttgtggtag atgattgagt taaatggaga tggggtaggg ttgtggtagg ggtgtagggt 11340
tatgataggg aaggtggagt taaatggagg tggggtgaaa gagtgaaggt ttggggtttt 11400
tggaggtatg ggtggggtga ggtgtaggta gggttttatt tgttttttta ggatggggat 11460
tattgatatt agttttttgt ttgtttgggt gtttaggggt tagttttggg agtttatggg 11520

tttagttgag ttttgtagat tttggtttag ttttgtagat gaagatagta ggtgaggttg 11580
tggttatgtg agggtaattt aggtgggttg tggtttatgg tgtggggttt gggtttggtt 11640
tgggtttttt gtttatattt ttgttggttt ttggttgttg agtagggtgt tttggagggg 11700
ttgtttttat gagtgttttt tttgagttgg ttttttggtt ttttgtgggt gagagtgttt 11760
gggatatagg tttttgttgg gatatgggtt tttggtttgg tttttgttta gggattaggt 11820
atggagttta gtggtttagg ttgtatttgg tggatattgg tttttttgta taaatttttt 11880
tttttttttt tgtattttta gttataggat ttgttattat ggttttgttt ttttaaggga 11940
ggaaatgttt gttaggtgta taaattttta agtggtgtta ttgtattggt ttaggggttt 12000
ttttggtaag ttattttgag gggggttgag gtttagttag gtttgtgggg ttttttttga 12060
tgggtttagg ttggggtttt gggataaggg ttgtgagtag tagggagatg gaggttttgg 12120
aggatatagt tttagttttt gtatagggat ggtttatttt ttgtataggg atggtttatt 12180
ttgttattta ttttatgttg gatttaggtt atggttttat aaattgatta ggttgtttag 12240
gtgtgagttt gttggaggat tttggaaggt gggggtggtt ggaatgtttg agtttaggag 12300
ttggagattt atttgggtaa tatagtttgt ttttataaaa agtttaaaat tagttgagtt 12360
tggtggtggg agtttgaggt tttagttatt taggaggttg aggtgggatg attatttgag 12420
tttagaaggt ggaggttgta gtgaatggag attgtatttt agtttgtgta aggggaattt 12480
tgttttaaaa aaaaaaagta tgtttttttt tgatttagtt tttttttttt gatgtgaaat 12540
ttttttagat ggaatgtgtt gaagttatag atatgttgtt tgttttattt atagagtgta 12600
attaagtttt agtttgtttt tttgtttttt taatatttgt ttagtagaga ttgttttttt 12660
ttgtttagtg gaaaatttta gatattatag attttttgt ttttttttttg gttaaagggt 12720
attttgaggg ttatattaag ttataaaata ttattttgat ttaggaatta gaagtttaag 12780
attttaatta atatttttat ttgttattta gttaatttta tggagattta ttttatatat 12840
aataaattat atttgtgtaa agtatataag tgggtgagtg tgattatttt tttgaagttg 12900
ttattatagt taggatggtg tttggtttta tatagttgtt agtatttgtt gtggttttta 12960
taagtttggg tttttggtag ttaggagttg attagattgt agttgtattt tttagggttt 13020
tatataaagg ggttgtatat attgattttt agattttttt atgttgtttt atttgtattt 13080
tttttttttt tatagttgga ggatttttta ttgtgttggt taaatgttat tgttt 13135

<210> 284
<211> 13135
<212> DNA
<213> Artificial Sequence

<220>
<223> chemically treated genomic DNA (Homo sapiens)

<400> 284

aggtggtgat gtttggttaa tatagtggaa aattttttga ttgtggaaag gaagaaagtg 60
tggataaaat aatatggaag gatttaaagg ttagtgtgtg taattttttt gtgtaagatt 120
ttagaagata tagttgtagt ttggttagtt tttggttgtt gggagtttgg gtttgtgggg 180
gttgtagtga gtgttggtag ttgtgtggga ttgggtattg ttttggttgt ggtggtagtt 240
ttaagggggt ggttatattt atttgtttgt gtattttata tggatgtggt ttattgtatg 300
taaagtagat ttttatgaag ttgattaaat agtagatgaa agtgttgatt gaaattttgg 360
atttttggtt tttgaattaa aatgatgttt tgtgatttaa tgtggttttt aggatgtttt 420
ttaattagag agggagtaga agggtttgtg atgtttggag tttttattg agtaggggaa 480
gatggttttt gttgggtaaa tgttaaaggg ataaaaggat aaattaagat ttgattgtat 540
tttgtgggtg gggtgagtag tatgtttgtg attttaatat gttttatttg aaaggatttt 600
atattagaga ggaggagttg aattagagga gagtatgttt tttttttttg agatggagtt 660
ttttttgtat aggttggagt gtagttttg tttattgtag ttttattttt ttgggtttag 720
gtgattattt tattttagtt ttttgagtag ttgggatttt aggtttttat tattaggttt 780
gattaatttt aaatttttttg taaagatggg ttgtgttgtt taggtgggtt tttaattttt 840
gagtttaagt attttgatta tttttatttt ttagagtttt ttggtaggtt tatatttggg 900
tagtttggtt agtttgtgag gttatgattt gaatttagta tggaatgggt gatagagtgg 960
attgtttttg tgtggagagt ggattgtttt tgtgtagaga ttggagttgt gtttttttagg 1020
gtttttgttt ttttgttgtt tatagttttt gttttaggat tttggtttag gtttattaag 1080
aggagtttta taggtttggt tgggttttag ttttttttgg ggtaatttgt tagggaggtt 1140
tttgagttag tgtagtgata ttatttgagg atttgtgtgt ttggtaggtg tttttttttt 1200
taggaaggta gggttatggt aataggtttt gtggttggaa gtgtaggggg gagagagggg 1260
gtttgtgtgg gaggattggt gtttattaag tgtagtttgg gttattgagt tttgtatttg 1320
gtttttgagt aaaggttggg ttgagggttt gtattttagt gagggtttgt attttaggta 1380

tttttatttg taggaggtta ggaggttagt ttgggggaaa tatttgtgga gatagttttt 1440
ttaggatgtt ttgtttgata gttaggggtt ggtaagggtg tggatagagg gtttggattg 1500
gatttagatt ttgtattgta ggttatggtt tatttgggtt gtttttgtgt gattatggtt 1560
ttatttgttg tttttatttg taggattggg ttggggtttg tagggtttag ttgagtttat 1620
gagtttttag agttaatttt tgaatattta ggtgggtaaa gggttgatgt tggtagtttt 1680
tattttggag gggtaggtaa ggttttgttt atattttgtt ttatttgtgt ttttaaaggt 1740
tttaagtttt tattttttta ttttattttt atttggtttt atttttttttg ttgtggtttt 1800
gtatttttgt tatggttttg ttttattttt atttaattta gttatttgtt gtggttttgt 1860
tttattttat ttttatttgg ttttattttt tttgttatag ttttgtttat ttttatttgg 1920
ttttattttt tattatagtt ttgtttattt ttatttggtt ttattttttta ttatagtttt 1980
atttattttt atttggtttt gttttttttg ttttggtttg tatttttttta atatttttttg 2040
attttatttt tatttggttt tatttatttt gttttagttt tattttattt ttttggtttt 2100
gtatgttttt ttttgtggta ggttttgtgt atttgttttt ggtatggtgt tgtggtattt 2160
tgtttttttg gttggttttt ttgtgggagt tgttagtaag tttatggaga atatggtagg 2220
tgtttttttt atgtgtttat gtggtttaag ttagagtttt tgtggagggt ggtgggtttt 2280
ttttttagag ggtttaattt ttttattttt aggatattga tgttttagtt ttagaggtgt 2340
tgattaggtt tattgttggg gttagagggg tttgtgtttt gtagaggggga gtttttttgtt 2400
gtttgttggg tgagtgtttg tttaggaatg gttgggttag gtggggtttt tatggtgagg 2460
tattttggtt tgtaatggga gtatttttgt tagtggtgat agtatagtgt gttgtgtatt 2520
gttattgggg agtgtagttg tggtggtttt agatttttag gtggagtggg tggagggtgg 2580
ggatttttta ttgttttggt tggggtgttt gagggattgg atttgggttt ttttttggtg 2640
gtgtggattt tattttgtat tttgtagtta tttttggatg tttttattgt ttattgtatt 2700
taggttatga gaaatagtat agttgtgatt aatatataag ttgagggtat tttgagtttt 2760
ttaggtttgt ttagtttgtt tgtggttagg gaatgggttt ttatatatat tgtaagtgtt 2820
agtatttagt gtatttgtgt agtatttggg tatttgggtg tgaatttgtt ttttgaaatg 2880
ttagttaggt ttgggtattt tttttgtggg gttaagtgga aatttttgat ggagagtttt 2940
tggagatttg tagagaatgt tggtggtgtt gttatggttt gtgggtgttg tttttttgggg 3000
gtggtttatt tttttaggtt tagttttttt ttggagattg tttatttagg gttttatagg 3060
gatgtgtatt gtggttgtga ggtttttttt tgtttttagg tttatagggg atagagggtt 3120
atttgatgtt gattttagaa gaggttggag ttgtggatat tgagatgtgt ttttatggta 3180
tttttttagtt gttgttgttt tgggtgtgag tttgtttggt aggtgtgttt tggagtttta 3240
ttgttgtgtt ttttttaggt ttagttgttt gagtgttgtg tggatgtggt gggtgttaat 3300
gttagttgtt taggtgtaag tttgtgtggt ttaggtaagt tgttttgttt taagtagatt 3360
ttagattggt ttttgagtta ttggtgtgtg tgtgtttagg gttttttttt tggtgataag 3420
gatagtgttt atttgtgttg tgggatttgg ggagtggatg gggtagtgag ggttgtggag 3480
tttggggaag gatatttgag tttttttttt ggtgggtggt tttgagtggt agttttatga 3540
gaagattggt ttagtagagg gttgggagta gaggtaggag ttgagattag aaaggtgttt 3600
ggagaagggt ttgggggagt ggtttatgag tttagttatt tttatggggt tgttttggtt 3660
ttaggggttt ttggtaaagt ttaagatatt tttgtttgtt tgtgtttggg tagaggtgtt 3720
gttggttttt ggtgtgtgga gtggaatgtt aattaatagg atgttatgag gatgttggtt 3780
tgtgttagtg tgttatgggt aggttttgtt tattagaggt tttgggaggt tgtggaaggg 3840
attgtttggg ggtagttggg tgttaggagg ttgaggttgg tgagggtagt tagtgggagt 3900
agttagggga ggggaggata gagtgagaag gtgtaagggt tattttttgg tggtttttgg 3960
tgaatttggg gttgagatgt tttagttgta tttttaaggg ttttggtatt tttattttgg 4020
gggtttagtt tttttatgat ggaagaattt agaagggttg tttttgggga tttaggggtt 4080
ttgtgaggag ggtgtggtta agatggtggt tgtggtagtt agtgaggttg ggtgtggggg 4140
tttgaggttg ggtgtggggg ttggggagtt tataggaagg tagtgatgat atttgaggta 4200
tatgtagggt aggaggagga gggtattgtt ttgagggaga atagaggggt tgggaggaag 4260
ttgagtattt tggggttttt attgtttgat gttgaagttt tggtggttgg gtgagaatta 4320
tttttttggt attttatggt agtaagagtg ttggggttgt ggtgtttttt gtgagggatt 4380
gtgtatttta ggtattttgt gggtgtaggt agttgtgtag tagttatggt gtttatggag 4440
gggttaggtt agtgggttgt gaagtttaag ggttgaggtg gttttatgta taggtgtagg 4500
gtggggattg tttggtgtgt gtggggaggg gttgggtttg tggtgttgga tatgtttttt 4560
tttgtgtgtg gttttaagtt aggttatgtt tttgtttggg gaggtagtgg gtatttttgg 4620
ggatagtgtt gtgttggtgt ttggtttttt gttgtggatt ttgtttttttg gtttttgggg 4680
tagagatttt tagatagagt tttgttgaag ttgtttgagt tttgtgttag gagatgttgg 4740
gagatagagg ttgtttttag tgttgggaga gtgtagtttt attttggtag gattgagtgg 4800
gtggattttt atttttttaa gaggtgtgtt tgtagagtga tttttgttat aattgtgttg 4860
gggttttagg tttatttttt attgtggggt ttttgtgttg ttgtggtttg tttgtattta 4920
gattagtttg tgaggaaggg ttgaggtgtg tggtttattt gtggtttgta ttagtagttg 4980
tattgtagtt ggtattaaga gtgtgtttat ttagttttgg gttgagtgtt ggagatattt 5040

```
agaaagttaa ggagtaaaag ttaatttttg tttttaaagg tgttttggtt ggtgggtgga     5100
aatgaggttt taggtgtagt gttggtttgg tggggggatg aggtttggtt ttgtaggatt     5160
ttttgagaga gatttggggg agggtgttag ggtgtggagg tgatagatgg attaagggtt     5220
gggattagtt tggggtagga ggttgtgttg ttggtgggtg ggtttatttt gtttgggtgg     5280
tatgagggaa tatggggtgt gaggtagttg ttttaggtat tgtagatggg ggttttgggg     5340
gaggtgtggg gggttttggg ggaggttgtg ggggggtttt ggggaggtgt gggagggttt     5400
tagggaggtg tggggaggtt ttggggaggt gtgggggatt ttgggggagg tatgtggggg     5460
ttttgtggga ggttatgggg ggggggtttt gggggaggtt gttgggggat tttaggagag     5520
gtgtgggggg ggttttgggg gaggtttggg gtaggtgttg tagtgggggt ttttggggag     5580
tttgtggggt gaaggttggg tggtggaagt tgggtttagt ttgtagtttt gtagagtatg     5640
agttttttta ataggtttgt ttattttgga tgttgttgtt tattattttt tgaagtatgt     5700
agtgtggagt atttagggtt ggggtgttta gggttggggt gtttggtgtg gagtgtagtg     5760
tggagtattt ggggttgggg tgtttggtgt ggagagtgta gtgtggagta tttggggttg     5820
gggtgtttgg tgtggagagt gtagtgtgga gtatttgggg ttggagtatt tagggttagg     5880
gtgtgtagtg tggagtattt agtgtggggt gtttatggtt ttgttatgtt ttttgggggt     5940
gtttttgttt gttgtttatt tattttttag agtatgttag tatttggaat gaaagttatt     6000
ttattaattt aaatagagaa ttatttttag gagatttagt tggtgtggat tttgttttgg     6060
ttgatatgtt tgttttttgt tttatgtagg ttgttatagg tgttggaatg tggatgggag     6120
tgttagttgt gtttgttgtg ggaatggaat ttttttagtt tataatggtt ttgagtgtag     6180
aagttgtagg tttgtttggt tttttttggt ttttttttta tagttttagt tgtgggagat     6240
attgtgggag ttgttgtttg tgtttttatt ttgttttaga gttattgttt attggggaat     6300
agttttggga gttttttttt tttgagtttg ttgggttatt tgggatagtt ttgtggtgag     6360
ggttgttttg tttttttagt tttttgtttt tatattttttg ttttgagatt tttttagggt     6420
gtatgtgggt gtttttgtgg gttttgtgta tataggtttt ttatagtttt gttattttat     6480
agttgttggt ttgggtgtgt tattttttat gaatagaagt ttagggattt ttgggtggtt     6540
atattttggt aagttttagt aggttatagt tatttttgtt ggtgttttgg gggttggggg     6600
ttgttggagt tagtgtagat ttttattttt gatgtagtta tagtaggttt agggttttgt     6660
aaaatagtta gaggggagtg tggtagtttt tgtagtttgg ggttagttgg tgtgggtggg     6720
ggatgtgggg tgttatgggg ggttttgtta tatttttaaa gggttgtagt taggggaggt     6780
tttgatggtt atattgtatt tttttagggt tatatagttt atagttatat gtttttaggt     6840
ttatatttat ggtttatggt tatattgtat gtttttaggt ttatatttaa ggtttatatt     6900
tatggtttat gttgatatta tatattttta ggtttatgtt tatggttata ttgtatgttt     6960
ttaggtttat atttatggtt ttgagttggt ttgggtttat tgttgatttg agtggtagtg     7020
ttttagggtg gggttggttt ggatgggttt ttggtttttt gtggttttgt gttggggtag     7080
gaaggagtga ggtttttaag tttggtaaat atgtttgtgt tttagggttg gggagttttt     7140
tttttagtat tttagagtat atttagtagt tattgttaat tgttttgagt tttaggttag     7200
tagttttggt ttgttttggt ttaggtaggt ggaggtgagt ggggtgtagg agtatttgtt     7260
gttagtttga ggttttgttt tgtgtggggg ttttagggtt tgtgttgagt ttggaggaag     7320
gaataaggtt gagaattttt tggtttttag gggtaattgg aattggagag gtgtttttgt     7380
agagtttggt tagggttagt ttttgtaatg tttttaatat ttgagttttt gagagaagtt     7440
gaggttgggg taggtggtaa ttttttttaga ttttgtggtt agatgtttag gtatggttga     7500
gtttgtaggg atgatttagg tttatgtgta gagtttttgg tatttatagt tggttttgtg     7560
aggtttaggt atgtttagag agtagtgtat ttgttatgtg taggggggtat tggggggttag     7620
gtgaggagtt agggttattg ttttgtttgt ttgtttagtg gtgtttgttt gtaggggttt     7680
tgtgtgtggt tgtttttttt tttttgggta tgttttttat tagttttggt tttatttttt     7740
ttgtagttgg gttttttttat tttaagtgtt ttagtaaatt ttttagggtt tgttatagaa     7800
gaaataaagg tattttttgg attagtattt gttttatggg agggtatagg gtaggtggta     7860
tttagttgtg gagatgggtt tgagattgga gtgttttagg ttagagtagg ttggtagagg     7920
ttgggaggga gtagggtttg ttttgtggtt tttgggtttt ggtgtgagaa ggtttgttat     7980
tttttggaaa tagttgtgtg tggatttgaa gggttgaagt attaaggggt tttttttggg     8040
gtttagaatt tttgttggag tttttttttg gtagaggtgt ttttttgggg tttttagggt     8100
attatgaggg gtgagtggag tggggttggg tggttttttgg tagttggatt tgtttgggtt     8160
tttttagtag ttatggtttt tttttttttt ttttggggtt agggttggtt aggttggtgt     8220
agggtttata ttttagttta tgttatgttg tatatatggt tattttgggt ttgggtattg     8280
attttttttt attggagaga tggttttgtg attgtggtgt ttttaggtgg gggtgggttt     8340
tgagtagggt ttgtggtgtt tgtttttgtt gtttagggtt ggtttttgta gggtttttgt     8400
taggtagatg gtttagttag tagtttggat tttaaggtga gtatttggtt agattgtgat     8460
agttgagttg gggttttttt ttaggttttt gggtagtttt atttggttgt tgtgagattt     8520
ttgtttattt tttttaattt tttttggggt tttagggttt agtttttagt tgggagtttg     8580
ttgttagggt tttttgtata gttaggtatt gagagatttg gtttgtttag tatttttgtt     8640
tatgggtatt tttggagtag agtagggttt agggtttggt tagttttggt ggttatggga     8700
```

```
tgagaagtta ggttggttat gatgttatgg tatttttatt gtgggggtat tatggaggta    8760
gaagtttatg gttgtggggt ttaaagttat tttgtgtttg aggttttttt tgggagggtg    8820
ggagggtagt ttttttttttg ggattttgga gtttggggag atgtttttta tgaggggggt    8880
ttgttttttt tgttgaggtg ggaaggtggg tttagggagt ggggatttat ttggggtgtt    8940
agggaggttt ttgtatttgt gagtgttttg gaggaatgtg gtaggattga gggttaggtt    9000
gatgtattgt agggagaggt tgggggagat tttgtagttt tggggttttt gttgttttga    9060
ggatgttgta ttgggttgag gttgaggtag ttttttgttt tgttgttttg gatgttgtat    9120
tgggttgagg ttgaggtagt ttttgggttt tagggtgatt ttttgttttt tttttttatag    9180
ttttggtttt gtagtttggt gaagttgtaa gtaatttata ttatttgggt tgagtgtttt    9240
tggttgtgtg tgtttgtgtt aggttatttt gagtttttat tttgtttttt tttttttttt    9300
aaggttgtaa tgattttttt gttatagttt ttaggtaata gtagttgttg tatttttttg    9360
tggggttttt tatttttggg ttagttttag ggtgttttgt gggtttgttt ttaaatgggt    9420
ttgtttggtt ttggattttg gttattttttg tttgggtttt taggagattt atttataagg    9480
tagggttagg gttggatttg gttggtgttt tttgttttgg attttagttg gatttggttg    9540
gtgtttttttg tttgtttttt tttaattttt gtgattttta tgtttttttt tgtttttgag    9600
atgtggggtt tgtaggaaag gaggatttat tatgataggg tagattttta atatttgttt    9660
tttttgaag tttagtttta tttgaggata gatgtagttg gtttttgtta ggtttttttg    9720
agtagttatt gttttagtgg tgttgggtta ggtggattta agagttagtt tggttaggat    9780
ttattttatg tgtggggtta tagatatagg ttgagtgatt ggtgtatagt tatatagttt    9840
taaggttatg agtagggttt atttggtttt tgttttttttg tgtagaagtt tgttatgggg    9900
gtaggtgatt gtgtttttag gggatagatt gtttttttatt aaggttggat gtgtttttgtt    9960
tttaaagtag ttttgtttgt aagtgtttag tagattttat ggtgtttgag tgttttgggg   10020
ttgttagttt taatagtaga tttttaattt gattatttta gagtttagaa gtttaaaatt   10080
aaggggtaaa taaggttata tgtttttttga ggttttgggg aggatgttgt ttgttttttt   10140
agtttttgtt ggtttaggta tttgtggttg tgttgtttgt ttttgttttt tggttttatg   10200
gtgttttttt tatttttgtg tttttttttt ttgttttttt tttaggtggg ttttggttgt   10260
tgttttaggg tttgtttggg gaattttggg tgattatatt tgagattttg atgtaatttt   10320
ttgtaaagtt ttttttttta aataaggtta tatttgaggt tttgggatta tggtttgttt   10380
gttgtttttt gaggtgtatg atattttggg atttatattt ttgtttttgg ggattgtttg   10440
gtttttgttt aggttttggg gtttagggtg taaatgagag gatatagttg ggtaggagga   10500
agttgttttt tttttgagtt ggagttttga gggtgtttta ggggtagggg gtatttgggt   10560
ttttatttgg ttagagtaaa tagttttggg tgtttgtgtt atatgtttat ttttgagagt   10620
ttttggtgag ggttgaggtt tgtattgttt ttgttgttta ggttttattt tgattttgtt   10680
tgtttttgat ttgttttagt atggaagttg tgttatgtta ggtgggagtg gtttttggat   10740
agggttatgg attttgttgt ttttttgggg gaggtttgta ttagtaatgt ttgatttgga   10800
ggttgagatt atgttatgta tttggtggta gggatttgga ggttgatttt ttggtgggaa   10860
ttagtataaa gtgttggtat tgtttggtgt ttgggatagt tttgggtata gttttggttt   10920
tgagtttttt tgtttttttag tgatggatgt taaagggttt tgggttgttt gaggtttttt   10980
tttattatag ttattttgtt tattggatta ggagtaggta tttatgagat tttagagttt   11040
tagattgagg ttttgggggg tttgggtttt tttaggaaat atggtgaggt tttagtgttt   11100
gtagttaaag ttggtatgat ttatggggta ggtgttgttt tgtttagaaa agttaggggt   11160
tttgttgttg tgttttttttag agtttatagt gggtaggatt tttttagtat tattatattt   11220
agtggtttga gatttttttg agaatagtga ggttggtttt tgtgttgttt tagttggtgt   11280
ttggttagtg gggaggatat agtttaggaa ttagttgttt gagattaggg tgtttttggg   11340
ttgttttttt gtgtggtgga gattttaagt atgtagttat ttatttttgg agttgtagga   11400
tagagttttt ttttgatttt tgtttttaag tagaaattta ttgtgtagaa aagtttttta   11460
gagttttgtg gttttgtttg gatttgttgg atttttatgt ttggttgatt tttgtttatg   11520
tggggtaggt ttatatttaa tttttataag ttattgtttt attgtatttg ttaaggttgt   11580
tttggtgttg agttttgggg ttttttttgg agtttttggg agaaaggtgt tgttgtggtt   11640
gtttttttgta tgttaggttt gggttttatt gtgggtttta gatgttttgt ggtattggta   11700
ttgtttgttt tagtatggga ttttttttttg aggggtggtt tggtttttgg ggtttttatg   11760
ttttttttgtg aagtttattg tgggtgttat tatggttttt gggatttggg ttagtgggaa   11820
tgtgggggta ttgggtgtgt tgatataaag ttggtattat ttaagttgtg ttttgagttg   11880
ttttttttttg taggatgttt tgttttttaa gtgtaggttg gggagtaaag tttttgttag   11940
gttggtttta tgtattttgt tattgtgtgt tggggtaag gaggtgggat gggggtggag   12000
gagagtttag gttggggttg tttggttggg attttttgtt ttgtgttgta tatttttttgt   12060
tataggttta tttttttagat gtaatttgtg taggtgggat ttgtggtttt tgaattttta   12120
gtttggagtg aggtgggata gagtgtgggt gtgtgttttt attggttggt tgggttgtgt   12180
gggggttgta gatatgggtt ttgggagggt tgttatggtt atttgggggg gtagtagtgt   12240
gggggtgttt gttgatagtg atgggtttgg ttagagaggg ttgagttggg gtaggatagg   12300
ttttgagggt agagggtttg ggtattagga tgttagagtg tgggttttgt ttttgtaatt   12360
```

```
gtgtttttta tttaggtaga aatgtttatt gtttggaggg ttgtgttttt agatatttgt   12420
atttttgttt ttaaaaattt ttagtttgag gtttgtagtt agggagtatt tgggggagta   12480
ttggttttgt tttgggtttt ggtttaagaa gtgttttggg gttgggaggg ttattggttt   12540
tggggagaaa gggagggatt ggatagttta aggttatttt gagtttagta tttattgttt   12600
tatttgatgt tttgggttat gtttttgtga tgtgggagtt tttagatttt ttttgtgggg   12660
aaggagatag ggattgagat gggagtagag ggaaggagat agggattgag gtgggggtgg   12720
agggaaggag atagggattg aggtgggggt ggagggaagg agatagggat tgaggtgggg   12780
gtggagggaa ggagataggg attgaggtgg gggtggaggg aaggagatag ggattgaggt   12840
gggggtggag ggaaggagat agggattgag gtgggggtgg agggaaggag atagggattg   12900
aggtgggggt ggagggaagg agatagggat tgaggtgggg gtggagggaa ggagataggg   12960
attgaggtgg gggtggaggg aaggagatag ggattgaggt gggggtggag ggaaggagat   13020
agggattgag gtgggggtgg agggaaggag tttatatttt tttggaaatt ttatttttgt   13080
atttgtatta ggttgggtgt ttggtttgga ggataaaatt ggaaataggt atgaa        13135
```

<210> 285
<211> 4852
<212> DNA
<213> Artificial Sequence

<220>
<223> chemically treated genomic DNA (Homo sapiens)

<400> 285

```
tttggttgat tgtaagtttt gtttttttggg tttattttat ttttttgttt tagttttttg      60
agtagttggg attataggtt tttgttatta ggtttggtta aatttttttt ttttttttgag     120
atggattttt attttgttgt taggttggag tgtagtggtg tgattttggt ttattgtaag     180
ttttgttttt tgggtttatg ttattttttt gttttagttt tttgagtagt tgggattata     240
agtgtttgtt attatgtttg gttaattttt tgtattttta gtagagatgg ggttttgtta     300
tgttagttag gatggttttg atttttttgat tttgtgattt gtttattttg gtttttttaga     360
gtgttggggt aataggtatg agttatgatg tttagttttg gatatatatt ttgaaaggta     420
atggggtgat aaagagagga agattgggtt gggtgtggtg gtttatgttt gtaattttag     480
ttttgggtgg ttgaggtgag tggattatga ggttaggagt ttgagattag tttgattaat     540
atggtgaaat tttgttttta ttaaaaatat aaaaattagt tgggtatggt ggtgtatgtt     600
tgtaatttta gttattttgg aggttgaggt aggagaattg tttgaatttg ggaggtggag     660
aggttgtagt gggttgagat tgtgttattg tattttagtt tgggtaatag agtaagattt     720
tgttttaaaa aaaaaaaaaa aaaagagtaa gattgtagaa gtggtatttg ggtgtttggg     780
tgggaggaat ttgggagttt gatttttaga tgtaagtttg aaatatttag gttgggtgtg     840
gtggtttata tttgtaattt tagtattttg ggaggttgag gtgggtagat tatgaggtta     900
ggagatgaga ttattttgat taatatagtg aaattatgtt tttattaaaa aatataaaaa     960
attagttagg tgtagtggta ggtgtttgta gttttagtta ttggggaggt tgaggtagaa    1020
tggtgtgaat ttgggaggag tttgtagtga gttgagattg tgttattgta ttttagtatt    1080
ttagtttggg taatagagta agattttatt ttaaaaaaaa gaaatattta ttagatattt    1140
aattgggtat gtatattaga tagttggata tatgattttg gagttagggg atgagtttag    1200
aaagaaatat aaattatgga gttgttagtg tagagatggt gtagaaagtt ttgatgttga    1260
aggttgggta tagtggttta tttttataat tttagtattt tgggatgttt aggtgggagg    1320
attgtttgag tgagtttaat attagtttgg gtaataaagg ttggtattat aaaggtttgt    1380
ttttataaaa aaataaaaag ttagttaggt gtggtagttt aagtttgttg ttttagttat    1440
ttaggagatt gaggtgggag gattatttga gttttggagg ttgaggttgt agtgaattat    1500
aattgtgtta ttgtatttta gttttgggga tatagtaaga ttttagtgtt ataaatatat    1560
aagttttgat atggaaagag attattatat tttatgtgtt tttttatttt tgtttatttt    1620
attatttggt gtttttaagt ttaatatttt ttttgttgat ttgaggttat atagtttggt    1680
gaaagagggt tggattttttg gtttttttaat atatttatat atgtttgttt aaaattttta    1740
gtgttttatg tttttagttt ataaagtata gatatagttt taatgagagg tttttatatt    1800
aggttagaga atgttagttt tttttatttt tatagagatt ttaaaaagta tattttagat    1860
aaaaaagaaa aataagtaat taagattatt ttagggttgg gtatggtggt ttatatttgt    1920
aattttatta ttttgggagg ttgaggtggg tggattattt gaagttggga gttttagatt    1980
agtttgatta aggagaaatt ttgtttttat taaaaatata aaattagttg ggtgtggtgg    2040
tgtgtgattg taattttagt tatttgggag gttgaggtag gagaattgtt tgaattttgg    2100
aggtggaggt tgtagtgagt tgaattttat tattgtattt tagtttgggt gatagagtga    2160
gaatttgttt taaaaaaaaa aaatgttttt tttataagga attttgagga tttgttggtt    2220
```

taattgtttg gatataaaga tatatatata tgaagttgga gagataggaa gaagtgttta 2280
atatagaata ttttaagtat ttttattttt ttttaaggat taaatttttt tagagatttt 2340
ttttgtttta gttttttagg agttttttga agatgttagg agttgtttaa gaaggttgga 2400
agatggttgt tgggaaggaa ttgttggtag ttggtaatat tttattttaa ggaggtggtg 2460
gtaatagttt tttgggtagt agatattatg gatttttttt tttttgggaa ttagaggttt 2520
ttttgttttt ggtttgaatt ttttagagat aaagaatttt attttttttt atatttgtgt 2580
gggattgttg gggaggagag ggggtggagg ggttagattt ttatttttat gaaatatttg 2640
tgggtttaaa gaggagtttg gattttgatt ggtttaggag gtgtgtgggg ttgtggggtt 2700
tttttggttt gggttaggtg tgttgttaag atgtttgggg aaggggttgt ttggtgtagg 2760
ttggggatgt ggagtgtttg gggtgttttt atgtaggtta tatattggtt gtttgtattt 2820
tttttttttt tttttaattt tatttttttg tgtttttttt ttggggagtt tttgtttttt 2880
tttttttttt gtggttgttt ttgttttttt ttttttttta gatttttttt tggtttgttt 2940
tttttttggt agtttatttt tattttgttt ttttgtgtgt tttttggttt ttgtttttta 3000
gttttggttt ttttttgttg gtttttggtt tttgtggttt ttttttttgtt ttttttgagg 3060
tggtggtggt tttttttata atagtgtggt ttgttttttt ttttttggtt tttggtgtat 3120
ttttgtttgt tttttttttt gtgtatgtgt tttttggttg gtggtggttg tggtggtggt 3180
ggttatgagt ggttttgttt ttttaaaggg aaatgttgag gtgttggggg tgattgtggg 3240
ggagggggat tttgagttgt ggagattttt gggagaggtg atagattttt ttttttggag 3300
taggagggtt ttgggtggat ttagtttttt attttttttt gaggaggagg ggggggaaat 3360
ggaggtttgg ggtggttgag gtgagttttg gggtgggggg ttggggtggg gaaggggggg 3420
tgtggggtgg ggagatgagg tgggtttggt tgggttaggg ggggttgaag tgagttttgg 3480
ggatgagttt gggggtggtt gggtgtgagt ttttgttttt gaggtgaagg tggtggtggt 3540
ggtagtagag gtggtggtga ggtttttatg ggttggtggt gggttttagt tgtggttttt 3600
atgtttttgt atgttggtgg gatggtgttt gggttttgga ggagttgtgt tagtggaggt 3660
ttgttgttgt gttgttgagg tgagtttgtt aaattttttt tttttttttt agtttttgat 3720
tttttgtatt ttgttttttt tttatttttt tttttgtttt ggtgtttggt gttgttttgg 3780
attattatga ttatgagatt tgtggtgttt aaggtggttg tggtttttgt tggtggtaat 3840
tttgagtagt gattggatta tgagtgggtt gtggtgttgg gtgggtttga ggatgagttt 3900
ggggtggttg aagtttattt tttttttgg tattgtaagt ttaaggagtt ggggtttttg 3960
ttggtttttt tttagggtgg gagttttgtg ttttttttgg ttggttgtgg tggtaagggt 4020
tggggtttgt tatttttggt tggggtggtt tttgggtagt aggaagagag ttggggtggt 4080
ttggtgtttt tgttttgttt gtttttggtt attaagtaag ttggtattgg gggggagttt 4140
gttgtagttg gagttggttg tagtttttgg tttaagtatt aggtggtgtt gtttatttag 4200
atgggttttt ttgtggtggt ggttaaagag tttatgtttt gggttgggga taagggtggg 4260
gtggtttttt ttgttgttat tgttttggat ttggtgggat ttttattatt atttttgttt 4320
gggttgttat tttttgtgtt tattgttatt gttgggattt tggtggttag tgagggtaga 4380
tggaagagta tgaggaagag tttttttggg ggtggtggtg gtttgggagt ttttagttag 4440
gttgtttgtt ttaaatagat ttttttgttg taattggatt ttattgaata gtagtagtag 4500
tagttgtagg ttaaggaaaa ggagattgag gagttgaagt tagagagaga tatggtatgg 4560
gaggggttaa tttgtatttg ggttgaggag tgagttgtgt gtatgtttgg gggaaggggg 4620
ttgtaggagg ttaaggtatt tttgggttag gggtaaagga ggttggttat tggtaaagga 4680
gtattttagg tgttgggttt ttgggagtta ggtttataga tatgttgggt tggagggaag 4740
ggttaaaggg taattttta gggggagggg gaggggtttt aggtgttaaa gggttaattt 4800
aaaatgatag ttttagatgt gtgggaaaga tgtttagtgg aaaaagatgt ga 4852

<210> 286
<211> 4852
<212> DNA
<213> Artificial Sequence

<220>
<223> chemically treated genomic DNA (Homo sapiens)

<400> 286

ttatgttttt ttttattaaa tgtttttttt atatgtttgg ggttgttatt ttaaattaat 60
tttttggtat ttgaaatttt tttttttttt tttgagaggt tgttttttaa tttttttttt 120
taatttaatg tgtttgtgag tttggttttt agagatttag tgtttaagat attttttttgt 180
tggtggttaa ttttttttat ttttaatttg gggtgtttt aatttttat agttttttt 240
ttttgagtat gtgtataatt tgttttttgg tttgaatgta gattaatttt ttttgtattg 300
tgtttttttt tgattttagt tttttgattt tttttttttt ggtttgtagt tgttgttgtt 360

```
gttgtttgat gaggtttaat tgtagtagaa ggatttgttt gaggtaggtg gtttgattgg    420
aggtttttga gttgttatta tttttgagag ggtttttttt tatatttttt tatttgtttt    480
tgttggttgt tagggttttg gtggtggtgg tgggtgtgag gggtggtggt ttgggtagag    540
gtagtggtgg gggttttgtt gggtttgagg tggtggtagt gggggaggtt gttttatttt    600
tgtttttagt ttagggtgta ggttttttgg ttgttgttat gagagagttt gtttgaatgg    660
gtagtattgt ttgatatttg ggttgggggt tgtagttggt tttggttgtg gtaggttttt    720
ttttaatgtt ggtttgtttg gtggttgggg gtggataggg taagggtatt gaattgtttt    780
agtttttttt ttgttgtttg ggggttgttt tggttgggag taataagttt tggtttttgt    840
tgttgtagtt ggttggggaa ggtgtggggt ttttgttttg ggaggaggtt agtggggggtt    900
ttggtttttt gagtttatgg tgttggggga ggaagtgggt tttggttgtt ttaggtttgt    960
ttttgggttt gtttagtgtt gtagtttgtt tgtagtttag ttgttgttta ggattgttgt   1020
tggtaggggt tgtggttgtt ttgaatattg tggattttat ggttatagtg gtttggagta   1080
gtgttgggta ttgaggtgga ggagggggtg gggaaggggt gaggtgtggg gggttgagga   1140
ttgagggggg ggaggggagt ttggtgggtt tgttttagta gtgtggtagt aggtttttgt   1200
tagtgtggtt tttttggggt ttgggtgtta ttttgttggt gtgtggagat gtggaggttg   1260
tggttgaggt ttgttgttgg tttatggggg ttttgttgtt gtttttgttg ttgttgttgt   1320
tgtttttgtt ttagaggtag gagtttgtat ttagttgttt ttgagtttat ttttggagtt   1380
tgttttggtt ttttttggtt tggttgggtt tgttttgttt ttttattttg tatttttttt   1440
tttttgtttt ggttttttgt tttggagttt gttttaattg ttttgagttt ttattttttt   1500
tttttttttt ttaggagggg gtggagggtt gggtttgttt aaagtttttt tattttggga   1560
gaggggggttt gttgtttttt ttgggggttt ttgtggtttg gggttttttt tttttatagt   1620
tatttttggt gttttagtgt tttttttttaa aaaaatggag ttgtttgtag ttgttattgt   1680
tgtagttgtt gttaattgga ggatgtatgt gtggggaggg gagtgggtgg gagtgtattg   1740
ggagttgaga ggagggaggt gggttgtgtt attgtgaaag gggttgttgt tgttttgagg   1800
ggggtaggga ggggattgtg ggggttgggg gttggtgagg ggaggttggg gttgaggggt   1860
gaggattgag gagtgtgtgg aggagtgggg tgaaggtggg ttgttgaggg aggggtgagt   1920
tgggggaggg tttggagggg gaagaagggt gagggtggtt gtgggaagaa ggggaaagtg   1980
gaggtttttt gggaaaaaag tgtgagagaa tggagttgag aggaaagaag gaggatatag   2040
gtggttaatg tgtggtttat gtgggagtgt tttgagtgtt ttatgttttt ggtttgtgtt   2100
gggtggtttt ttttttgggt atttggtaa tgtgtttggt ttaagttgga ggggttttgt   2160
ggtttgtat gttttttgga ttagttagga tttaggtttt tttttgagtt tgtgggtgtt   2220
ttatgggggt agaagtttaa ttttttttatt tttttttttt tttagtagtt ttatgtgggt   2280
atgggagaga atgaagtttt ttgtttttaa ggatttaaa ttagaaatgg aggatttttt   2340
ggtttttaga gggaggaaaa tttatgatgt ttgttgttta gggagttatt gttattgttt   2400
ttttgggatg aagtattgtt agttattaat agtttttttt taatggttat tttttagttt   2460
ttttaaatga tttttagtat ttttgggagg tttttgaagg attgaagtaa aggaaatttt   2520
tgaagggatt tagtttttga aagggagtag ggatatttag ggtgttttgt gttgagtgtt   2580
tttttttatt tttttagttt tatgtatgtg tgtttttatg tttaagtaat tgagttaata   2640
agtttttaga atttttttgtg aaaaaaatgt tttttttttt tgagatggat ttttattttg   2700
ttgtttaggt tggagtgtaa tggtgaaatt tagtttattg taatttttgt ttttggggtt   2760
taagtgattt ttttgtttta gttttttgag tagttgggat tatagttatg tgttattatg   2820
tttagttaat tttgtatttt tagtagagat aggatttttt tttggttagg ttggtttgga   2880
atttttgatt ttaggtgatt tgtttgtttt ggttttttaa agtggtggga ttataggtgt   2940
gagttattgt gtttagtttt gaaatagttt taattgtttg tttttttttt ttgtttgagg   3000
tgtgtttttt aaaattttta tggagatgga gaagattgat atttttttggt ttgatgtgaa   3060
aattttttat taaaattgtg tttgtgtttt gtgggttgaa agtatgaaat attggaaatt   3120
ttaaataaat gtatgtgaat gtgttaagaa gttaagagtt tggttttttt ttattagatt   3180
gtatggtttt agattagtaa gggaagtatt gagtttaggg atattaagta atggagtgag   3240
tagggatgag agggtatata gaatgtggtg attttttttt atgttaaggt ttgtgtgttt   3300
gtggtattgg ggttttgttg tgtttttaag gttagagtat agtggtataa ttatggttta   3360
ttgtagtttt aatttttagg gtttaagtga ttttttttatt ttagtttttt aagtagttgg   3420
gataataggt ttgggttgtt atatttggtt aatttttttat ttttttgtag agatgggttt   3480
ttgtaatatt agtttttgtt gtttaggttg gtattgaatt tgtttaagtg atttttttat   3540
ttgggtgttt taaagtgttg ggattatagg agtgagttat tgtgtttagt ttttagtgtt   3600
aagatttttt gtattatttt tatgttgata gttttgtaat ttatattttt ttttggattt   3660
attttttaat tttagaatta tatatttaat tgtttagtat atatatttag ttggatgttt   3720
aataggtatt tttttttttt gaggtggagt tttgttttgt tgtttaggtt ggagtgttgg   3780
agtgtagtgg tatgattttg gtttattgtg agtttttttt gggtttatgt tattttgttt   3840
tagttttttt agtagttggg attataggtg tttgttatta tgtttggtta attttttgta   3900
tttttagta gagatgtggt tttattgtgt tagttaggat ggttttattt tttgattttg   3960
tgatttgttt gttttggttt tttagagtgt tgggattata ggtgtgaatt attgtgtttg   4020
```

gtttaggtat tttaaattta tatttaaaaa ttaagttttt gaatttttt tatttaaata 4080
tttaaatatt atttttatag ttttattttt tttttttttt ttttttgaga tggagttttg 4140
ttttgttgtt taggttggag tgtagtggta taattttagt ttattgtaat tttttttattt 4200
tttgggttta agtgattttt ttgttttagt ttttagagta gttgggatta taggtatgta 4260
ttattatgtt tagttaattt ttgtattttt agtagagatg ggttttatt atgttggtta 4320
ggttggtttt aaatttttga ttttatgatt tgtttatttt agttatttaa agttgggatt 4380
ataggtgtaa gttattgtgt ttagtttagt tttttttttt ttgttatttt attgtttttt 4440
aaaatgtatg tttaaggttg gatgttgtgg tttgtgtttg ttattttagt attttgggag 4500
gttgaggtgg gtggattata aggttaggag attgagatta ttttggttaa tatggtgaaa 4560
ttttgttttt attaaaaata taaaaaatta gttaagtatg gtggtgggtg tttgtagttt 4620
tagttatttg ggaggttgag gtaggagaat ggtgtgaatt taggaggtag agtttgtagt 4680
gagttgagat tgtgttattg tattttagtt tggtgataga gtgagagttt gttttaaaaa 4740
aaaaaaaaaa tttagttgag tttggtggta ggggtttgta gttttagtta tttgggaggt 4800
tgaggtagga gaatggggtg aatttgggag gtagagtttg tagttagttg ag 4852

<210> 287
<211> 2519
<212> DNA
<213> Artificial Sequence

<220>
<223> chemically treated genomic DNA (Homo sapiens)

<400> 287

tatgtaatgt tggattttaa attggtttta atttttaaaa agtttatagg tatggatata 60
aatttttagt ttttgtggtt gtggttgttt tatgtatttt ttttagtggt ggatataaat 120
gttaatgtat ttgttattat ttaaggatat aaaaatatag aaggtggtat tttttgtttt 180
ttttttgaa gattttttt agagttgtta gatgaatgtt tatgttattt tttttatta 240
aaggtggatt tagttaatag gagattggat tgtgattata gtgtgtttgt gtttagtttt 300
ggggaaagtt ttatgttagt gagtggtttt tggtttgttt tgtagattgg ttaagtatgt 360
ggaggttgtg tggttttatt attaatattt ttgaaggata atagtttatt aagttttatt 420
gaaagttttg gatttgttgt ggagtttggg ttggtggggg atgaggttga gtaatttttt 480
ttagaggtgg ttatgtggag aaggagtaat tttttttttt tttttttttt ttttttgtta 540
ttatttgtgg tttagagaat tgttgtttgt tgttattgat atgtatagat agaatttaaa 600
gaaaggtaaa gagttttgtt tggtattggt gttgtgtggg ttaaatttgt gtttgtggag 660
gggtgtgtag agggtattgg gtgttgggag taggtggtgt agtattaggt gagtttgtta 720
tggtgatggt attattgttt agggtgtttt ggggtttgga aataaaatgt gttgttgggg 780
tagtgtaggg gttggagaga attattttg tttttttttt gagtttttgt gggtgtattt 840
ttgatagttg atttattttt tgtaaaaata aatttttaaa tttagttgtt taatttgttt 900
ttttgggtga taaagggggg gttgttaaag aagtggttaa atttttgtgg ttagtgaggg 960
gttgggggtg ttgagggtat tgagagggga aggaggaggg atgtgaggtt tagtagtggg 1020
ttttaggtga ggttggattt ggaggaagtg gaggtttttt gggaatgtgg agttttgggt 1080
gtaggagtgg gaggatggag gtgtttggtt gttgatgggt atggggtgat gggtgggggg 1140
ttgtgtaggg tttgagggag tttggggagg gttgtttggg ggtttttgaa ggtgtttggg 1200
tagggtgttg gtggttttag gttgataatg gaggggtgt ggagtagttg ggtggaggtt 1260
agggatttga gtttgggggt gggttgtgtt ggggaaggtt aggttgggga tgttgggtgg 1320
tggggtgagg gttgggggtt gttgtgtgga tatttgggtg ttgtgggtgg gtgtgtgttt 1380
tggggttggt gtggaagagg gtgtgaggtt gggtattttt ttttggtttt ttttttgggg 1440
tgtttggtgt tgatgtgtgt tgtgttttta ttgggaaatg gtggtttggt tgtggtttgt 1500
ttgggtagtt tggtttttgt tttgtgtggg aaaaaataat aataataaaa atattgaaaa 1560
agggaataaa agtaaattgg ttgtggtgta ggaaagggtt ttgttgggtg ttggatgttg 1620
tgtgtattgt ttgtggggtg tggtggttgg ggattagttt tgtttttttt ggtttttagt 1680
ttttagtttt ggtgtatttt agttttagtt tggttagttt tttggattgt ttgtgggtgt 1740
ttttttttgt tttttttgg gggggtgttt ttagtttttg ttttttttgt tttttataat 1800
aatggttaaa ggggtattgg ttttttaagg gttaaatgag tttttttttag gagttttttg 1860
ttgtgtgtag tggattattg tgggtttgat tgaggtttgg tttttgttag ggttttttgg 1920
taatggtttt ggatgagtga agggatgaaa ggtgtgagaa gaaatgggtt gagaaattaa 1980
agtgtggggt tttgagtgtt tgaggggatg tgatgggaat tgtggttttg ttgttttgat 2040
tttaagggta gttattttgt tatagttatg ttggaattga ggtgtttttt tagtttttgt 2100
ttattttaga ggaatttaaa ataagggttt agttttaagt tttatagatt ttaggatttt 2160

```
ttaggggtta ttagtttatt gtggtaaatg gttttttat tttgtttatt tgtgtttgtt    2220
ttgaaattta attgttattt tattgggttg tatagttgtt attgaaatgg tttaataggt    2280
gagagaaaaa gaaaatatta ttaagagtga gaatgtgttt ttgtgtgtgt tgttttgaat    2340
tataaattaa gttatatata atttttaatt tgttttaata tattttttat ttttaaatta    2400
aatgttaaga gttttaagat ttggaaatat attggtgatt tttagaattg ttttagttta    2460
aaattatata taaataaata gaggtattgt ttggtggaaa agtttatttt gagttgtat     2519
```

<210> 288
<211> 2519
<212> DNA
<213> Artificial Sequence

<220>
<223> chemically treated genomic DNA (Homo sapiens)

<400> 288

```
atgtgattta agataaattt ttttgttggg tagtgttttt atttatttat atgtaatttt      60
aggttaaaat aattttaaaa attattagta tgtttttaaa ttttggagtt tttaatattt     120
agtttgaaaa taaaaagtgt gttaaaatgg attgagaatt atgtgtagtt tagtttgtgg     180
tttaaaataa tatatatgaa gatatgtttt tgtttttaat aatgtttttt ttttttttta     240
tttattgaat tgttttagta gtaattgtgt agtttagtga ggtgataatt ggattttaaa     300
ataaatataa atgagtaaag tgaggaaatt atttgttgtg gtggattaat ggtttttagg     360
ggattttggg gtttgtgggg tttagaattg ggtttttgtt ttgaattttt ttgaggtgga     420
tagaagttgg aaggatattt tagttttagt gtggttgtga tagggtaatt gtttttaaaa     480
ttagggtagt ggagttatgg tttttattgt gtttttttaa atatttaggg ttttgtattt     540
tggtttttta gtttattttt ttttgtgttt tttatttttt tatttattta gggttgttgt     600
taaaaaattt tggtaaggat taggttttag ttaggtttat ggtggtttat tgtatgtgat     660
gggaaatttt tggaggaagt ttgtttggtt tttggggaat taatgttttt ttggttattg     720
ttgtaaggaa taaagagggt aagaattgag ggtgtttttt taaggaaagg tgggggagga     780
tatttatggg tgatttgggg ggttggttgg gttggggttg aggtgtgttg gggttggagg     840
ttgggagttg ggggaggtag ggttggtttt taattgttgt gttttgtgag tgatatgtat     900
ggtgtttggt gtttggtaga gttttttttt gtgttgtagt tagtttattt ttattttttt     960
ttttgatatt tttattatta ttattttttt ttgtgtaaag tgggagttag attgtttagg    1020
tgagttatgg ttgggttgtt attttttggt gaaagtgtag tgtgtgttgg tattgggtgt    1080
tttggaaagg gaattgaagg ggagtgtttg gttttgtgtt ttttttttgtg ttggttttgg    1140
ggtgtatgtt tgtttgtggt gtttgggtgt ttgtgtagta atttttagtt tttgttttgt    1200
tatttggtgt ttttgatttg attttttttg gtgtggtttg tttttggatt tgggttttta    1260
atttttgttt ggttgttttg tgttttttttt attgttagtt tggagttgtt ggtgttttgt    1320
ttgggtattt ttagaggttt ttgagtggtt ttttttgggt ttttttgggt tttgtgtagt    1380
tttttattta ttgttttgtg tttattggtg gttgggtgtt tttgtttttt tgtttttgtg    1440
tttgaaattt tgtattttta ggagattttt gtttttttttg ggtttagttt tgtttggggt    1500
ttgttgttga attttgtatt tttttttttt tttttttttag tatttttgat gtttttggtt    1560
ttttgttggt tgtaggaatt tgattatttt tttagtagtt ttttttttat tgtttaagga    1620
agtaagttgg gtagttggat ttaagaattt gtttttgtag agggtgggtt ggttattgag    1680
ggtgtgtttg taggggtttg ggggagaggt agaggtggtt ttttttggtt tttgtattgt    1740
tttggtggtg tgttttgttt ttagattttg gagtatttta aataataatg ttattgttat    1800
ggtgggttta tttggtgttg tgttgtttgt ttttggtgtt tggtgttttt tgtgtgtttt    1860
tttatgggtg taggtttggt ttatgtggtg ttggtgttgg gtaggatttt ttgttttttt    1920
ttgggtttta tttgtgtgtg ttaatggtgg taagtggtag tttttttgggt tgtggatagt    1980
agtgggggga ggaggaggag ggagaaggga ttgttttttt tttatataat tatttttagg    2040
aggagttgtt tagttttatt ttttgttagt ttaggtttttg tgatgggttt ggggttttttg    2100
gtggggtttg atgagttatt gttttttagg agtattaatg gtgaaattgt gtggtttttg    2160
tgtatttggt tgatttgtga gatgagttga gggttatttg ttaatatgag gttttttttta    2220
gaattgggta taaatgtgtt atggttgtga tttagttttt tgttagttgg atttattttt    2280
ggtgggagaa ggtggtgtgg gtgtttattt ggtagttttg gggagagttt ttgaagggga    2340
aagtgaaaaa tgttattttt tatattttttg tgtttttaaa taataataag tatattaatg    2400
tttgtgttta ttattgggaa gagtgtatag ggtggttata gttatgagag ttgagaattt    2460
atatttgtgt ttgtaggttt tttagaaatt aagattgatt tagaatttag tgttgtata     2519
```

<210> 289

<211> 9783
<212> DNA
<213> Artificial Sequence

<220>
<223> chemically treated genomic DNA (Homo sapiens)

<400> 289

```
ggttatatta gatttttttt taattgtttt tttgaatttt tagatgattt agagtggttg      60
gttgattttg ttggtttatt ttatatttag ggaaggaatt tgttttggag atattaagag     120
atattgtatt tttaatttta aaattagagt gagggtggga tttggttttg ggattttagt     180
tttaaatttt aggatatttt tttttggttt gagtatgaag aaggaggatt tttaatttta     240
tttttttttt tttttttttt ttttatatat atatatatat atatatatat atatatatta     300
tatatatata tatatatata tatatatata tatatatata tatatatata tatatatata     360
tatatatata tagagttgga tttttttttta tttttttttat ttttaatata agtgggtttt     420
aggaattgtt tagggtggtg tttagatttt gttgagtagt ggtaggtttt aatggttata     480
ttttgttttt atttttgtta tatttttttt ttggtttgga ggaattttta ttttatttta     540
ttttaggttt tttgtggttg tttattttgg gggttggggt gtttgagttg aagttttttt     600
gtttttttgtt tggtggggta ggttttggga agttgggagg agaagggaag gggttttgat     660
ttggatttta ggaggttaga gtttttgttg gtaggtttgg gtttttttgt tgtagtttgg     720
ggaggggttg atgtggtttg attattgttt agtttggatg ttgattttgt ttggagggaa     780
tggggtttat gtttaggttg aagttttttt ttgtatgggt tttgattttt gggttagtga     840
ggaagggaaa gtgttttttt ggggatgagt ttttaagatg ttttattgtt tgtagttttg     900
gtttagggta ttttttgttt tgttgttttt agttgtgtgg ttggtgttgg aggtttagat     960
tgattttagg gttttgtttt gtttttttttt agtgttgtgg tggggagggt gggtttggta    1020
gggggagggt tggttggggg tggtttttgg tttggtttgt tttggttttg tgtagggttg    1080
tagtgtggtg gtttttttttg tagaaaaaaa attgggggtt aggggtgttt attttttttag    1140
gttggggaga gttatgggga gatttttgga ttgtggagga gttgggtgag tgggggggagt    1200
atgatttggg tggatttaga ttttgtttag tttttaattt tttaggggag gattggtgtt    1260
gggaaagtta agaggtgttt ggggtggggt taggaagggt tgttgttgtt tttttgtggt    1320
taagtggtta tttgtttttg ttgtatttgg gattttgtgg gaagtgggat tgggaaaggg    1380
tatttgttgg gaggttttag ggtttggtgt gggtgtgttg gttgggggga gatgtttatt    1440
tttttgtttt taggattgta tagtttaatg ttatttttta aatttgaaga tttttttagag    1500
ttgtttagat ttagattatt gttttttttt ataatttgtt tttttttttg ggtttggaat    1560
ttttaggagt ttaattttta tttatttttt aaagggtgtg tttagaaatg tttttggatt    1620
ttagttgtga attttgtttt tatttttagg tttgatttgg tgtataggat atttatttat    1680
atgtgtattt tttttttttg taagggagtt gaggaggtgg gagtagaatg aggatgttta    1740
ttaatatttt attggtttgt gtttgggata ttatatagtt taattttttt aatatttatt    1800
atagattttt ggggaaaata tatttatttt tttttgttta tagagaagtt gaggtttaga    1860
gaaattaagt ggtttatata attgtttttg atttgtgata gtaagaattt atatagttgg    1920
gtttgtttgg ttttaaattg gtggtttggt tttgttattt tatgggtttt tttagtttgt    1980
tttttttttt gttgtttagt agagtgagtg gtatataata ggttgttaat aaatatttga    2040
tgaatgaatg aatgttagga gttatataga ttttaaatat ttgaagtgta atttagatat    2100
ttgataaatg tgaaaatagg tttgtagtag tttggtttta ggttttttaa ttgatttaat    2160
gaatattttt tgagggtgaa ttgagagtta tgtttggaag atttattttt gtttttaaat    2220
aatttagagt ttaaatgagg aatgtaattt taagatagtt aatatttatt gaatttatat    2280
tatatggatt tgggtttata tatattaatt ataagaattt tatgaggtag ttatttttat    2340
tttattgaga agtaaattga ggtttagaga ggttatagag ttagtgttgg gtttgatatt    2400
agaatttaaa tagttgaatt ttagagtttt tgtttttaat ttttattttt tattgatttt    2460
taatgtagat agttataata ttggaggtta ttgttgtagt gtgggtatgt gtgggttgtg    2520
tgatatttag ttttgaataa taggtgaagt ttttaagata aggtgatgtg tagatttttt    2580
taattttaat tttttttttga gataaggttt ttgttgttta agtgtgtagt ggtaagattt    2640
tagtttattg tagttttaat tttttaggtt ttaatgattt ttttatttta gttttttttag    2700
tagttgagat tataggtgtg tattattata tttaattttt gtattttttg tatagatgag    2760
gttttgttat attgtttagg ttggtgaggg gtttttttta attattttgt ttattttttat    2820
ttttgttttt tttaatttat tttgggttta atttgttgtt tttttttttag ttttttaagg    2880
tggaaataga ttattgattt gagattttta tttttttttta ttataagtaa tatttttaat    2940
tttttttggat gtttaattgt tttttatagg tatttattat tatatttagt taattttttta    3000
tttttttgta gagatggggt tttattattg tttagggtgg ttttaaattt ttgggtttag    3060
```

```
tagttttttt attttttgttt tttaaagtgt tgggattata ggtgtgaatt attgtgttta    3120
gtagtagatt ttgaataagt tggagttatt aaaagtttat agttatgtaa attttaggaa    3180
gttggtggtg gtggtaaaaa atgaagttgg agaaggaagt agtggttgga ttatgaaggt    3240
ttttatatgt tatgtatagg agttgagtgt tgattttgtt ttaggtgtaa taaggagtta    3300
ttaaatttag gaggttaata tagtaaaagg tggtggtgat tgaaggtggg gagagatgga    3360
gataagtaga gaaatatttt taaaggttta tagttaataa tatttagtga ttaattattt    3420
tgatgagaaa gaaatgaaag ttaagggtga ttaattttaa gatttttaat tggagtagta    3480
gagttggtgt ttattgaggg gatatattag gagaaggaat atgttatgga gggtagttgt    3540
gaatttagtt tgaggtattt gaattgaagt gttagtaggt tatttaggag atatttggga    3600
gatgattggt atgtgtgggt ttaaagttta ggaagggttt gggtagatgg gtgaggttat    3660
agaagtggat gatgatattt atagtaaatg tgtggagtgg aaagagtggg ggtggaataa    3720
agaatttggg ggatattata tttttagaat tgttttggtg ttttttttat tattttatag    3780
agtttttttt ttattttttta gtttttagtt aaagtaaata tagatatata tttttattta    3840
ttttgtttat ttgttaatag ttgtaatttt ggatatagtg gatattttttt ttttagttaa    3900
gttgttatta aagaagggtt agggagataa ttttagaatt tgggttgtga gaggagtaag    3960
tatggagagg ttagtgggag ggatggtgtt tagtttttta ggagtaattg tttggttttt    4020
ttagggggat gatgaagata gagatgttat ttttggaatt tagtttgttg ttggggagat    4080
gttagttatg gttttggggg gtattttggg tttttaggat tagagtttgg gttagagttg    4140
tatatttgtt aggagtatat atgtatattt atgtattaat atgtatgtgg gaagatagat    4200
atataggttt atatgtgggg ttttaagtat tgtttttttt tgattttttt tttattaatg    4260
ttattttttt attattatgt tatgtagttt ttatttgttg tggttattag gtttataggt    4320
tgatagtatt ttgtttatag tggtagttat tttatagtta gtagtgatat tttataggtt    4380
atttgttttt aggattagta tattatttgg gtagtgttta tattgtgtta ggttatatat    4440
ttgtatttgg tgggagaaaa gggtagagag gattgaggtg tggggtttgt tggataggga    4500
gtttggagat aaagggattg tagatgaaag ttggtttgag atttttagag aaggtaaggt    4560
gtggtttaag gggtttattt tagggaagta ggagagaaag ggtttgatgg gtagggtgga    4620
attgtagatt ttaaggttgt ggttttttaa gttttgggtt attttaatgg tggttattat    4680
ttgagtttga aagaaatgag gagggagtgt tattgtagtt ttttgttaga ttttttgaaa    4740
ttaaagattt tttttatttt tatttttagg tagaattttt attttttag ataagttgtg    4800
ataggttttt tttttttttt ttatttttat tatggagttt atttttttgt tttttttata    4860
gattagagga aatttgagat tttttagttt gattttttttg taggttttgg ggaggggggga    4920
gtagaggagg tattatttag gtattaaagt tttttttttt taggagttgt atatttttat    4980
ataaattttt tagaagggtt atattttttt aatttttagt ttttagtttt tattttgttg    5040
tgggtgaggg attggttgtg ttgttatggg aatgggtttt agttttgagg gaagtgttat    5100
tagttggtta tgttttggtt ttggtataat atttttttgtt ttagtttttt gggtagaatt    5160
ttgtgggggga atttaaggtg ggtgaattta ttgtttagta gttttggttt taggaagttt    5220
tggagttggg gatgattaga atagagagtt aggggtattt atgtttatag tatatggtgg    5280
tatatagatg atgttgatta tagtagattt ttaaatagag tttattatgt gttatatgtt    5340
attttaatat tagtaaatat taatttattt atttttttata atagttttta aggtaggaat    5400
tattgatgta tagagaggtt aagggaggga attgttttgg ttatataatt agtaaagtat    5460
ttagattagg atgtattttg ggtttttagt tttatagtta tatgtgttta ggtattaagt    5520
tggattgttt tttagggggtt tttgttgttt tttttttta ttttagtttt tattttttgt    5580
tttagttggg gttatagtgg gagtattgga ttagtttagt gttgtagttt ttttgttgta    5640
atttttttatt ggtttagttt tgtattttat agtttatata gtggtttttt tagtaaagtg    5700
tttttttgtgt ttgtggtggg ggtgtgagag tttttagggt tatggggtgt agggaggtat    5760
aagatttgat aaagaatggg ggtagggaat ttggttttta tttataagta gttgaagatg    5820
ttggtgttta gtttttgggg taaatttttg ggatattgga atgattttat tattttggat    5880
aatgatggtt ttgtttaaag aaagaaaagg aattgggatt tggtttttttt tagatttatt    5940
tatgaaaaat tttagaaaga ttaggttttt tttataatga ttttttagat tttttattta    6000
gattttttata tttgtttttg attttattta atggatattt taggttattt tttatgtttg    6060
ttatttttttt tgattttttt taattttgat ttttaatttt tgttatttta ttttttttgta    6120
ttttttttaat ttagagagtg tggtttttttg ttgttatttt ggagtttttt tttttatatt    6180
tgttattgtt tgtattggtt atgtatattt tgtttagtag gtagattata attagtgtat    6240
agtagttttt ttttatttgg tggttatgtt gtttttgggt tatttgttta ttttgttata    6300
taaggaaggg ttagaggtgg gtagaaggag aggttttatt atatagttat tttttttttta    6360
tttttttttat ttgtagaaat taattggttt agttaggtta tattggaaga taggagggaa    6420
gattggggtg taattgtttt tttttttttt tttttgaatt aagttttgtt taggttttta    6480
aaggtgttgt gggtttttag ggtaattttt tttggttttt atatagagtt ggttagagtg    6540
gttgtttagt aggtttttgg tttaggttat ttatttatta tgagttggaa ggtattttta    6600
atggtttttta ttattaggtt tttgtggttt attttttttt gtgaagatta gtaggattga    6660
gggttaagta agtgagaggg attggaggaa tttggggttt ttggagtggt tgggaggtag    6720
```

```
aggggtggtg gtgaagggtt ttgaagtatt tttattaggt tttttagttg tttttggata    6780
tggtgatgta ggagttgtga ggttatttta gtagttttgt tttttgtatg tttattaaat    6840
aggttttagt agtagaagta gagtttttgg tggaggaaaa gttggaggtg agttttgttt    6900
gtgtgtgtgt gtgtgtgtgt gtgtgtgtgt gttatgtaag gtgaataata agattttggg    6960
tatttatata aatatttgag aggttagtgt ttgttattga aggtaaatta ggtttattta    7020
tataaggttt tgtatgtgtt aagagaaagg aaaggtttaa aaatttaaat ttagaggata    7080
atagatttaa aatttagtat tggtaattaa attatttata aatatgtgag tttttatatt    7140
ttaggtggga aagtaatatt tatgtttata tggtagaatt taattgatga atattaggta    7200
aaggttttgt gttagaaggt agatagttag tgagtttatt aggtttttg tatggtttag     7260
atttaaaatt agaggtttga gtttttgggg agaggtgtta ttgtgttttt gggaaagggg    7320
atgggtttta tttggttttg gttaggtttt ttaggtattt ttgtaatatt tttttgattt    7380
tttatatata ttattttata gtaagtttgg tttttattgt gttgattttt gttagtattg    7440
atgattttgt taggttagaa tgattatatt aggttggata ttagattagg ttttggaata    7500
atttttattt tagatatata ttgtttttttg tgtttgttgg ttaagttgag aaaggttata   7560
gttatttggg atttaggtta gagggaggag gagaagggag ggttggtaag gtgtaatgag    7620
tattgtttgg gtggttagtt ttattatttt atagaaaggt tattaggatt ttttggttaa    7680

ttgttatgtt ttttgtttgt tttatttttt ttatttttgg taaaaaaaaa agaaaaaaaga   7740
aaaaggtatt tttagtggtg tttttggggg aggggttagt tggtagtaga agttttattt    7800
ttttgttagt tagattttat attttatttt taattattat taattttttt agtgttttgt    7860
ttttagtatt ttatttttat gggtatattt atttttattt taaaattatt tagtgaaagt    7920
ttttgttgtt ttaataatga ggaagttttt tagtttagat tgggaaagag gaattgaaat    7980
agagtagaag tgaaagttta tttggattgg gtaggagtta tatatttata aatttttttt    8040
atataggagg ttaagggagg tggtggtggg tttatgggtt attagttatt ttttttagtg    8100
aattgagtgt ttttattttt tttggtgttt tgtgtttttg gtagatgtat ttaaaatata    8160
tatagtttgg gggttagttg gttgttttgg gtagggggtt tgtttttttg ttaggttagt    8220
atgagttggt aggttgaagg gtatttaaat gggatttttt tttattttgg gtgttttttt    8280
ttttgttttt ggggaagttg gtggtttttt ggtgttgtgg gttatttttg ggtgtggagg    8340
gggaggggaa ttatggtaag agggataggg aattggggtg ttggagttgg gaagtgggtg    8400
agtgagtttt tttaattgtt atgtttttta ttaaaaagaa ttttgttttt ttgtgaaatt    8460
ttttttttt tagtttttaa gttgttgggt tgtgatgggg aattggttaa tttattttt      8520
gttgggtttt ttgtttttttg ggttttgggg tagaataggg ttttgaggtg gtgtgggtga   8580
gggtagttaa tgagtgggaa ggatgtgaag aaggtgatat aggttgtttt ttattggaaa    8640
atgggttggg agggggtgtg gtttgaagat tagaggatag taaaggttta tggaaaaggg    8700
agtttttggt ttggatgtgg tgtggggaat gtgttttagt gttggggatg tggagttggg    8760
gagaggggtg ggagttttat tttggtttgg gtgttttagg gggtgtttat ttggtgtagt    8820
ttgtgtttta gggtaggtgg tggtttgtgt ttgggggggtt ttgtatagtt tggtttgtgt   8880
ggttattggt gtgttgtagt tttttggggt ttagttgtaa aaaggttggt ttggagaagt    8940
ttgtttgagt tttaatagtt tttgtgggtg ttgtgttttt gtttttaatt tttgtgggag    9000
ggtttttggg tgtttttgga gtggtggtgg gtggtgttga ggtggtgttg ggtaggtttg    9060
gggatttggg gtgtggaggt ggagtgtttt tggagtttat taggtgtgtt atggttgagt    9120
gtatttggtt tagtatgttg tttttttgtt ttgtttttgg ttgtggtttt gtttttgttt    9180
aggttttgtt tttatggttt tgtgtgggtg gtgtttgtgt tagttggttt ttttttgtta    9240
ttggttttttg gttagttggg tgggttttgg gtgaaatttg tttttgtttt gggttatttt   9300
aggaattggt ttatgggttt ttggtttttg ttttgtttta ttttggtttg gttttgtttt    9360
tgttttttag gggtaggttt tgttgttgtg agaagggggt ggagagaagg aaattttatt    9420
ttagttgtgt gttagattag atatagttag aatgttttta ttgtgatggg ggttgaggga    9480
taagggggtg tggttttttt gtttttggtt tggtgtggtt agttagttta gagagttatg    9540
gagttaatga ttttagagta aaataggtgt ttgggagttg ttgggtatga ggtgttaggt    9600
gtgatattat ttttggatgg ggttgttgga gtgagagtgg gtaagttggt attgggtggg    9660
gttgtgggat taatgatgta ggttgtagtt gattattttg gaattaggag ttggggtaga    9720
tttttgggtt tttgatgtgt tgtttttttgt gaagattatt tttagatttg ggagagtggt   9780
tgt                                                                  9783
```

<210> 290
<211> 9783
<212> DNA
<213> Artificial Sequence

<220>
<223> chemically treated genomic DNA (Homo sapiens)

<400> 290

```
gtaattgttt ttttagattt agaggtggtt tttatgggga atagtgtatt aggagtttag      60
gaatttattt tggtttttga ttttggggtg gttggttgta gtttgtgtta ttaattttgt     120
aattttattt ggtattagtt tgtttgtttt tgttttagta gttttattta gaggtggtgt     180
tatatttggt gttttatatt tagtggtttt taagtgtttg ttttgttttg aggttattag     240
ttttatggtt ttttgggttg gttggttata ttgaattgag agtgaggaga ttgtattttt     300
ttgtttttta gtttttgtta taatggagat attttagttg tgtttaattt agtgtatggt     360
tgaaataaag tttttttttt tttgtttttt ttttatagta atagaattta tttttaagaa     420
atggaggtgg ggttaagttg gaatggggtg gggtagaggt tggggatttg tgggttggtt     480
tttggggtgg tttagagtaa gggtaagttt tgtttgggat ttgtttagtt ggttgggagt     540
tagtagtagg gaagggttgg ttggtgtgag tattgtttat gtggagttat gggggtgggg     600
tttgggtggg ggtggggttg tggttgaggg tggggtaggg aggtagtatg ttaaattggg     660
tgtgtttggt tgtggtgtat ttggtgagtt ttgggggtgt tttgtttttg tgttttaaat     720
ttttggattt gtttaatgtt gttttggtgt tgtttgttgt tgttttagaa gtgtttagga     780
gtttttttgt gaaggttggg agtgggagtg tgatgtttgt gaaggttgtt gaggtttgag     840
tgagtttttt tagattgatt tttttgtagt tgagttttgg ggggttgtga tgtgttgatg     900
attatgtggg ttgggttgtg taaagttttt tggatatggg ttgttgtttg ttttggagta     960
taggttatgt tgagtgagtg ttttttgggg tatttaaatt aggatggggt ttttattttt    1020
tttttagtt ttgtattttt ggtgttagga tgtgtttttt atgttgtgtt tgggttagga    1080
gttttttttt ttgtggattt ttgttatttt ttggtttttg ggttgtattt tttttaatt    1140
tattttttag tggggggtag tttgtgttat ttttttttatg ttttttttgt ttattgattg    1200
tttttgttta tgttgtttta ggattttgtt ttgttttaga gtttggaggg tggagagttt    1260
ggtgaaggat gagttggtta gttttttgtt gtggtttggt agtttaaagg ttaagggaaa    1320
aggggtttta tgaaggagtg gggttttttt taatagggga tatagtggtt gggaagattt    1380
gtttatttgt tttttggttt tagtgtttta gttttttgtt tttttattg tagtttttttt    1440
tttttttat atttagaaat agtttgtgat attaggaggt tgttagtttt tttaggagtg    1500
gggaggggga tgtttggggt agaggagggt tttatttaga tgttttttag tttgttaatt    1560
tgtgttggtt tggtaaagaa gtggattttt tgtttggagt ggttggttgg tttttgggtt    1620
gtgtgtattt taaatgtatt tgttgggaat gtagagtatt gagggagatg gggtgtttta    1680
gtttgttgag gaaggtggtt ggtggtttat ggatttatta ttattttttt tagtttttttg   1740
tgtgggagga gtttatgggt atgtggtttt tgtttagttt aggtgggttt ttattttttat   1800
tttattttag tttttttttt ttgatttggg ttggagagtt tttttattgt taaggtagta    1860
gaaatttttg ttggatggtt ttaggataag ggtgggtgtg tttatgagga tgaggtgttg    1920
gggataaggt attgggagag ttgatagtgg ttgagggtgg gatgtagggt ttagttagta    1980
gggaagtaaa atttttgtta ttaattagtt ttttttttag aagtattatt aggaatgttt    2040
tttttttttt tttttttttt ttgttaggga tgggggaagt gaggtaggta ggggatatag    2100
tggttggtta gaaggttttg gtggtttttt tgtgaagtgg tagggttggt tatttaaata    2160
gtatttatta tattttgtta gttttttttt tttttttttt ttttggtttg gattttaggt    2220
ggttgtgatt ttttttagtt tggttagtgg gtgtaggaga tggtgtgtgt ttaaggtggg    2280
ggttatttta ggatttggtt tggtgtttaa tttgatgtgg ttattttggt ttggtagggt    2340
tattaatgtt ggtaagagtt ggtataatga ggattaggtt tgttgtgaag tggtgtatgt    2400
ggaaggttgg aggagtgtta taggagtatt tagggagttt agttgaggtt aggtaagatt    2460
tatttttttt tttagagata tagtgatatt ttttttttagg gatttaggtt tttggtttta    2520
aatttgagtt atgtggaaga tttgatgaat ttattggtta tttatttttt ggtatagaat    2580
ttttatttgg tatttattag ttagatttta ttatgtaagt atgaatgtta tttttttatt    2640
taaaatgtag aggtttatat atttatggat gatttaattg ttagtgttga gttttggatt    2700
tgttattttt tgagtttggg tttttgagtt tttttttttt tttgatatat atgaagtttt    2760
atgtgaataa atttaatttg tttttagtag taaatattga tttttttagat gtttgtatgg    2820
atgtttagag ttttgttatt tattttgtat aatatatata tatatatata tatatatata    2880
tataaataaa atttattttt aattttttttt ttattaggga ttttgttttt attattgggg    2940
tttatttgat gggtatgtag gggtggagt tgttgaaatg gttttatggt ttttgtattg    3000
ttatatttga gagtagttaa aggatttggt agagatattt taggattttt tgttattatt    3060
tttttgtttt ttaattattt tggggatttt agatttttttt gatttttttt atttgtttgg    3120
tttttagttt tgttggtttt tatagaagga agtgagttat gagagtttgg tagtgggggt    3180
tgttgagaat gtttttttagt ttatggtgag tgggtgattt gggttaagag tttgttaggt    3240
aattattttg gttaatttttg tgtggaagtt agagggggtt attttgagaa tttataatat    3300
ttttgagagt ttgagtagag tttggtttag aggggaggga gaagggggtag ttatatttta    3360
gttttttttt ttgtttttta gtgtagtttg gttgggttag ttgattttta tgggtggagg    3420
gggtaggggg agggtgattg tgtgatggaa tttttttttt tatttatttt tgattttttt    3480
```

```
ttatgtggta ggatgagtag atggtttggg agtggtgtgg ttattaagtg gaggggggtt    3540
gttgtgtatt ggttgtgatt tatttgttag gtaaggtgta tgtggttaat gtaggtgata    3600
gtaggtatgg gggagggggt tttaaggtgg tgatagaggg ttatattttt tgagttgggg    3660
gaatatagga gagtaaggtg gtaggggtta aaaattaaga ttggagggag ttaagagggg    3720
tgatagatat aaggagtggt ttgaaatatt tattgggtgg ggttaggggt agatatgggg    3780
gtttgggtaa gagatttgaa aggttattat ggagagggtt tggttttttt ggaatttttt    3840
atgagtgggt ttgaagagaa ttaggtttta attttttttt tttttttggg tagggttatt    3900
attgtttgga atggtgaaat tattttaatg ttttgggagt ttattttgga gattgagtgt    3960
tagtgttttt agttgtttgt aagtaggaat taaatttttt atttttattt tttgttgggt    4020
tttgtgtttt tttgtatttt atggttttga gaatttttat atttttatta taggtataga    4080
aagtatttta ttgggaaggt tgttgtgtgg attatggagt gtggggttga gttggtggga    4140
ggttgtagta gggaggttgt agtattgggt tggtttagtg tttttattgt ggttttagtt    4200
gaggtaggaa gtagagattg aggtggggga aggaaatagt aaaggttttt gaaaggtagt    4260
ttggtttagt gtttaaatgt atgtggttgt ggagttggaa gtttgggatg tattttggtt    4320
tgagtgtttt gttaattgtg tgattggagt agttttttt tttaattttt ttgtgtatta    4380
atagtttttg ttttaagggt tgttgtgagg agtaaatgag ttaatatttg ttaatattag    4440
aataatgtgt ggtatatggt aaattttgtt taagggtttg ttgtagttaa tgttatttgt    4500
gtgttattgt gtgttgtgag tataatgttt tttggttttt tgttttgatt atttttaatt    4560
ttagggtttt ttgaaattag agttgttagg tagtgaattt atttattttg agttttttttg    4620
tagagttttg tttaaggagt tggggtagag gatgttgtat tgggattaga atatgattgg    4680
ttggtaatat ttttttaga gttggggttt gtttttatgg taatataatt agtttttgt    4740
ttgtagtaag gtggaagttg gaggttggga gttgggagga tgtggttttt ttaaggggtt    4800
tgtgtgaggg tatgtagttt ttaggggaga ggggttttga tgtttgggtg atgtttttttt    4860
tattttttttt tttttagggt ttataaaaag attgagttgg aggattttag gtttttttttg    4920
gtttgtgggg agggtaaaaa ggtaaatttt atggtagagg tgggagaggg gaggaggatt    4980
tattataatt tgtttaggga ggtggaggtt ttatttgagg gtaggggtgg aagggatttt    5040
tggttttaga gaatttagta gaggattata gtggtatttt ttttttattt tttttaggtt    5100

tgggtgatgg ttattattgg ggtgatttga ggtttgggag attatagttt taaggtttgt    5160
agttttattt tgtttattaa gttttttttt ttttgttttt ttgaggtgag ttttttaaat    5220
tatattttgt tttttttggg agttttaggt tagtttttat ttgtagtttt tttattttta    5280
agtttttttgt ttagtaaatt ttatatttta atttttttttg ttttttttttt ttattaggta    5340
tgagtgtatg atttgatata atatgagtat tgtttagatg atgtgttagt tttgggaata    5400
gatggtttgt gggatgttat tattgattgt gaggtagttg ttattgtgga tagggtgttg    5460
ttggtttatg agtttaatga ttatagtagg taggggttgt atggtatggt ggtaagggga    5520
tggtattggt gaaagaaggg ttaaagggaa gtaatgtttg ggattttata tgtgggtttg    5580
tgtatttgtt tttttatata tatgttggta tatgaatgtg tatgtgtgtt tttggtaggt    5640
atatagtttt ggtttaagtt ttggttttgg gggtttgggg tattttttga gattgtggtt    5700
ggtgtttttt taataataag ttgggttttg gggatgatat ttttgttttt gttatttttt    5760
tgggagggtt aggtagttat ttttgagggg ttgaatatta ttttttttat tagttttttt    5820
atatttattt tttttatagt ttaaattttg aagttgtttt tttgattttt ttttagtggt    5880
aatttaattg aagaagggat gtttgttata tttaaaatta tagttattgg taaataaatg    5940
agatggataa aggtgtgtgt ttgtatttgt tttgattaga aattgaaaga taagaagaaa    6000
gttttgtggg gtggtggaaa gagtattaga atagttttag aaatgtggtg ttttttaggt    6060
tttttatttt atttttattt tttttatttt atgtatttgt tgtgagtgtt attatttatt    6120
tttgtggttt tatttatttg tttagatttt ttttgagttt tagatttata tatattagtt    6180
attttttgaa tgttttttgg atagtttgtt ggtattttaa tttaaatatt ttaaattaaa    6240
tttatgattg tttttttatag tatgtttttt tttttggtat atttttttaa tgggtattag    6300
ttttgttgtt ttagttagaa attttggagt tggttatttt tggtttttat tttttttttta    6360
ttaaggtaat tgattattaa gtattgttga ttatagattt ttaaaagtat tttttttattt    6420
atttttattt tttttttgttt ttagttatta ttatttttta ttatattggt tttttaaatt    6480
tagtggtttt ttattgtgtt tagaataaaa ttaatattta atttttgtgt atggtatata    6540
aggattttta tgatttagtt attgtttttt tttttagttt tattttttat tattattatt    6600
aatttttttgg ggtttgtata attgtgggtt tttagtagtt ttaatttatt taaaatttat    6660
tgttggatat ggtggtttat gtttgtaatt ttaatatttt gggaggtaga ggtgagagga    6720
ttgttaagtt taggagttta agattatttt gggtaatagt gagattttat ttttataaaa    6780
aaataaaaaa ttagttgaat gtagtggtga gtgtttgtgg gaagtagtta agtatttaag    6840
gaaattaaga gtattgttta tagtagaaaa gaataaagat tttaaattag tgatttgttt    6900
ttattttaag aggttagaaa aagaatagta aattaaattt aaagtaagtt aagggaagta    6960
aaaataaaga tgagtaaaat aattagaaaa ggttttttat tagtttgggt aatatagtaa    7020
gattttattt gtataaaaaa tataaaaatt aggtgtgatg gtatatgttt gtagttttag    7080
```

```
ttgttgggga agttgaagtg ggaaaattat tggagtttgg aaagttgagg ttgtagtgag    7140
ttgagatttt gttattgtat atttaggtaa tagagatttt gttttaaaaa aaaattaaaa    7200
ttaaaaaaat ttatatatta ttttattttg agaattttat ttgttgttta ggattgggta    7260
ttgtatagtt tatatatgtt tatattatag taatgatttt tggtattgta attgtttata    7320
ttaggagtta gtagggagta ggaattaaga ataggggttt tagagtttag ttgtttgggt    7380
tttagtgtta gatttagtat tagttttgta attttttga attttaattt gtttttttagt    7440
aaaatggaga tagttatttt atgggatttt tgtagttaat gtatgtggat ttaggtttat    7500
gtaatatgga tttaataaat gttagttatt ttgaaattgt atttttatt tagattttaa    7560
gttgtttgaa ggtagaaatg gattttttga atatagtttt tagtttattt ttgagaaatg    7620
tttattgagt tagttaagag gtttggggtt aaattgttgt ggatttgttt ttatatttgt    7680
taaatgtttg gattgtattt tagatattta aagtttgtat gatttttaat atttatttat    7740
ttattaaata tttgttgata atttattatg tgttatttat tttgttagat aatgggggaa    7800
gggataggtt ggaggagttt gtgaggtagt agggttagat tattagtttg gagttagata    7860
ggtttggttg tgtgaatttt tgttgttata agttaaaaat agttgtgtaa gttatttgat    7920
tttttttgaat tttagttttt ttatgggtaa aaggggatgg gtatattttt tttagagatt    7980
tgtgatgaat attgaaaggg ttaagttgtg tagtgtttta agtataagtt aatgaaatgt    8040
tggtggatgt ttttatttta tttttatttt tttagttttt ttatagggaa aggagatatg    8100
tgtgtaggta aatattttat gtgttaagtt agatttagga gtgggaatag aatttatagt    8160
tgggatttag aagtattttt aggtgtattt tttgaggagt aggtaggaat tggattttttg   8220
gaaattttag gtttagggaa gaaagtgaat tgtgaggagg aatggtggtt tgggtttgaa    8280
tggttttgag aggtttttaa gtttgggggga tgatgttggg ttgtatgatt ttaggaataa   8340
gagggtggat atttttttttt aattaatata tttgtgttgg gttttgggat tttttggtgg   8400
atatttttttt ttagttttgt tttttgtgag gttttaggtg tagtaggagt gggtggttgt   8460
ttggttatag gagggtagta gtggtttttt ttgattttat tttgagtatt ttttaatttt    8520
tttgatgttg gttttttttt aggggattga aggttgggta gagtttgagt ttatttgggt    8580
tgtgtttttt ttgtttgttt ggtttttttg tagtttagga atttttttgt ggtttttttt    8640
gatttggagg ggtggatgtt tttggttttt agtttttttt ttgtggaggg ggttgttgtg    8700
ttgtggtttt gtgtggggtt ggggtgggtt gagttaaggg ttgtttttgg ttgatttttt    8760
ttttgttggg tttgtttttt ttgttgtggt gttggaggag ggtggggtgg ggttttgggg    8820
ttagtttgag tttttggtat tggttgtgta gttggaggtg gtggagtgga aggtattttg    8880
ggttggggtt gtgggtggtg gggtgttttg ggaatttgtt tttaggagag tatttttttt    8940
tttttgttgg tttggagatt ggggtttgtg tgaggagggg ttttggtttg ggtgtggatt    9000
ttgttttttt taagtggagt tggtatttgg gttgggtggt ggttggattg tgttggtttt    9060
tttttgggtt gtagtgaggg ggtttggatt tgttggtagg ggttttggtt ttttgaggtt    9120
tgagttggag tttttttttt tttttttta gtttttttgga atttgttttg ttgggtgagg   9180
ggtgagggaa ttttaattta gatgttttag tttttagggt gggtggttgt aggggattta    9240
gagtgggatg gggtgggaat ttttttaggt taggagggag gtatggtggg aatagggggtg   9300
ggatgtgatt attggggttt gttattgttt ggtagggttt ggatgttgtt ttggatggtt    9360
tttggagttt gtttgtgttg aggataaggg agatggggaa ggatttaatt ttgtgtgtgt    9420
gtgtgtgtgt gtgtgtgtgt gtgtgtgtgt gtgtgtgtgt gtgtgtgtgt gtgtgtgtgt    9480
gtgtggtgtg tgtgtgtgtg tgtgtgtgtg tgtgtgtgta gagagagaga gagagagaga    9540
aaatgagatt gagaattttt ttttttttata tttgaattaa gaaagagtat tttggggttt   9600
gggattggga ttttgaggtt gagttttgtt tttattttgg ttttaaagtt gagaatgtag    9660
tatttttttgg tatttttaga atggattttt tttttaggtg tagggtgggt tggtaaagtt   9720
aattggttgt tttggattat ttgggaattt aagagagtga ttggagagag gtttgatgta    9780
gtt                                                                  9783
```

<210> 291
<211> 13537
<212> DNA
<213> Artificial Sequence

<220>
<223> chemically treated genomic DNA (Homo sapiens)

<400> 291

```
tgtataatta ttgttaattt ggtttttagt agaggtttgt gggttaggga gtgtattgat      60
ttgtattgtt aaaggaattt tttgtttttt ttagtgttag ttgaagagtt ttttgtagtt     120
atttttttat tttgagagaa gagaaataag aggagatgaa tttttggttt ttttttatgg     180
ggtgtataga ttagtgataa tgttagtttt ataatttgga tttatttgtt tgttttttaa     240
```

ttattgttat ttttttaagt ataaagtttg tgtttataat tagatatttt ttatatgtta 300
tattggtaag tttattttat tattttatag agggatggtg attttttta gtgttttata 360
ttgattgtgg tttttttattt gttgattgat tgatttataa atttgttttt tatttatttg 420
tttgtttatt gaaggtaaat ttgttattgg gaatatagta gttttttaat aattatttaa 480
tgtatggtta tttttttag gatttagaat attaaaggga tgtgtttttt tttttaaat 540
tatttgtttg tgttttttt agttgggtgt tttagtttt attttggttt ggttattatt 600
ttattttgtt tttttgtggt ttttaggttt tgggttggga ttttagtttt tgtttgtagg 660
tgtggtttag agggtgtgat atgtgttgga gtggggtagt ggaggagggg atagggatgt 720
ggtagttata tttgtatttt tttttttttt tgggtatttt tgagggtttt tttgttttta 780
gtttttgag tttttgaagt tttttatgtg gtaagagtga gttgtaggaa taagtgggga 840
attttggttg tggtttttt ttttgtttta taaaaaagaa attgattttt gtttggagtt 900
tgtgaaaagt ggggtttggg gtttagttaa tggggtgttt tgtggtgtgg gttgagtgga 960
gttagtgtga attgtttagt tgtggtttta attaatttgt tttttgtgtg gtttgtttgg 1020
ttgtttttgt tgtagttgta ttagtggttt attgtttggt tttgttgggt tgtgggattt 1080
atttttttta aatagttggt tttgtttagg gtagtttgag tggaagtttt ttattgataa 1140
tttgttttaa atagatagat ttgttagaag tgatttttgg gaaggaagta aaaagttatt 1200
ggtttgaagg ttgggtttaa aatagggatt ttgtgtttta tttgtggttg tgttgttttt 1260
ttgtgttttt ggttttgtag ttttgaagtt ttgtgggggt ttagattatt gtatttgttg 1320
agttagatat tttgtggatt tgatttttgt tttatttatt tgtaaaatgg agtagttggg 1380
tgttggtaag ttgtaagttt aagagttttg gttttattag tgtgtatgat gtttattttt 1440
tttttttttt tttatatttt ttttaaaaat tgtaagttag aaaatttggt taggttttg 1500
tgatggtgag aaatggtttt gaaggttggt tggttgttgt aatgtggttt ggattgtgta 1560
tgtgttgttt tgttattgtt ttttggtttt tttttaataa agatgttaat tgtgttaaat 1620
tataggtggt gtttttgtaa gattagttat tgttttttta taagttaaga tataaaatgt 1680
tttgtattga atggtttatg atttggagtg tttaatttgg gagttgagtt gttagatttt 1740
ttttttttta tttattagag ttattttttt tttaaatgat tttatttaat aagtaaaatt 1800
taaatggggt gggggggat ttaatatgtt gtggatgaaa agaatagtta ttgatttttt 1860
tatatttaat ggtatgtagg agttttttaa aatatagttt tatattaaat agatttgtga 1920
attttaagta gaattgaatt gtattaatta gttgatgggt ttgtttattt tttattatta 1980
gatgagaaat ataaaggttg ggaatttata gtgtttaatt ttattgtatt taagtaattt 2040
tttttttag tttagaaaat tttttattag ttgtatatga gagaaaaatt gaagtaaaaa 2100
aaaaaaaatt tgttttttgt ttatttatta ggtgaatata tattttagta ttatatagag 2160
gaaattttag gtggttttta gtaagatttt agtggttaag agttataatt tagaaaagat 2220
gataatatat atatagtgtt aatagtattt aaataaatat tttagataat agaataatgg 2280
ttattaatta tgatgttttg ggtttttgat taattttta tgtttgtttg tagttaaata 2340
gttgtaggat tttttttttt ttaaaaattg tttgtgtatt attttaaaaa ttgtatttaa 2400
tagttatatt ttttagagta gtttttttgt gtggaattga agtgaatggt ttagtaatag 2460
tgatgaagtt agtaagtagt aagaatgttt tttatgttaa aagaatttta attattttgt 2520
tagaaatggg ttatattttt atttttgaa atgtagaagt tgtttgttat tgtatgattt 2580
ttagtatggt ttatatgttt aaataaatgg aattatttaa ttttgtttat atttgtatta 2640
gtgtttttaa aattgattaa gttgtgtaat tgtattaaag tgtatttttt ttggggtttg 2700
aaaatattaa agaaattaag agagggtaag attattttgt aaaattgttt ttttattttt 2760
ttagataaat attatatttt aaaaattatt tggtgattta aagattaaaa tgaatgtatt 2820
tttgtatatt ttaaatatgt aattttaatg attatttagt tattgtagtt ggaaatatat 2880
atatttaggg atatagatat gtataattat gaatatttgg gtaattatat attgtaatta 2940
attattaatg tatggattaa aattaaatta tttttattgt ttttattgtt atttagtatt 3000
taataagtat ttatttggta ttaaaagatg gttaaaatat attaaataat gttagttttt 3060
taaaaaggta ttaaaatatt tatattttag atagatatat atatgttgta tgtagtgtat 3120
gttggtgttt taaatatttt attatttaat gttttgattt gatagaaatg ttagatttta 3180
aattattgtt ttatttattt taatagttat atttgaaatt ttagaggtta aggagaaatg 3240
tttgtaattt atttttttag ttgtgaattt ttgtatttga aaggttaaat tttttttttt 3300
ttagtaggta attattttat gttttgaagt atgagatttg attattttta ttattatttt 3360
agtttgtata gatatagatg ttattaatgg ttgtagttta tttaattttt ttttaaaatg 3420
tagttgtagt atttgatttg gggatttttt gtgaatttta ttggttgatt ttgttttgta 3480
taaagtatgt ggggtttggg ggagggagaa gagagaggga ggtttgtttt gttttggagg 3540
gttttttttt tttttttttg aattagagtt ttttaaatag ttatttggta tttttgtaag 3600
tgatgtgaat tttgaaatta tttattagaa taggataata gagtattgtg tgaattagat 3660
ttgttggaaa taaagtatag tagaatgtat gatgtttttt attttattgg tagagattaa 3720
atatgtggtt ttgatatatt tatttatata ataaagaatt gtagtttatt ttgaaatata 3780
ttggttatat ttaaattgga gtatttggtt ttatataata attgttttta taagtgttga 3840
aatagtaatt tggataaagt aggttttaaa gttgttgtga agggaagata gggatgtttt 3900

taaaatttga aaggtttaga ttggtaattt aggtagatta aatttagtga ttaattggtt 3960
aatttaatta ttgttttttt tttagtattt atgatttgtg tttttaagta ttgtttttgt 4020
aggttatatt attttttta taaaatagag aggatatttt ataggttttt tatattttgg 4080
aatatagtaa attttaagta ttggtagaaa gggaatttag aaaaaaattt tgtgaagtaa 4140
aggaggttga tgttgtataa atattgaatt atagttttaa gatgtataaa atattgtttt 4200
taattttaat tattttatta gtaagtaagt gaaattgttt taattggagt ttttagaagt 4260
ttttttggga aatgagaggt agttggatgt agtagaatgt agtggaatga gtattttaaa 4320
gagtattgga tttagtatta ggagatatgg gttttgggga tgatttttgt tttttagtga 4380
gtaagtaagg ataagttgtt tttaaggttt ttattttttt tatttataaa atgattatat 4440
tagaggagtg ttttgtaatt tatgtgtttt gtgattttat gttaaatgga agttaagtta 4500
gattgtgtaa tttataggaa attaggaggt tgaagaaagt ttggtagtta gtattttttt 4560
ggatgtagtt tgtgttttgt ttttttgttt ttttagttat ttttttgttt ttagaaagtt 4620
aaaaatgatt tgtattgttt tttaaggttt ggagatttag aggatttgtt tttgtttttt 4680
ttttatttta ttgttggggt tgagtaaaaa tgagtttttt attttaagtt tttagtagttt 4740
ttattgagta ttattaggta ggtttattta tatattttat atttataaag tattattatg 4800
tgttagggtg taagggatta gaaagatgtt tttatttgat tatttttgga gttagagaat 4860
gttaagatgt agtgggattt tagaagtttt ggagttttag tttgatttta gaattgaatt 4920
tttgtttaat taagggtgtg aataaatgta aagatatgat ttgttatttg ttaaggttgt 4980
ggggtgaggg gggtggttat atagagtatt gttttttgt tgttagtagt gtttattttt 5040
ttatttgtgg ggagtaatag attatttttg gtaatatagt gtttaaaagt gttaagaaaa 5100
attttgtaat ttaggattta ttggatttta gagaataagt gtttttttt tggattgttt 5160
ttgtttattt ggggggtaaa tttggataag aattaaataa tttgtagaga gatttatagt 5220
agaattaaga gtaagtttgt aaatattgtt tttttatttt ggtatttagt aagtatttat 5280
tatgtgttag atattgtttt atgatttatt tttttttta ttatttattg ggtaaataat 5340
agttataggt tgaattatga tgtggaagta aatgtaaatt aatttttttt tttatataaa 5400
taaataaata aataaataaa taaaaatttg tgttagaatg tttttggata ttaaatatta 5460
aaatttagtt aagtttagtt ggtaggtttt tttagtgatt tttttggttt gagtattttt 5520
taattttgta tattggtaag gggaatggta ttttaaagga gtgatttttt agaggttgaa 5580
tggtttttgt gaggggttga ttataggggga ttggggaagg ggtttttta atattaattt 5640
tattagtttt gttagttgat gggaaagttt gtttttttat ttttggtatt ttaatttaga 5700
agaatgtgtg tgtgtggagg gggtgttaat ggaaaaaatg ttattgtttt tagagaagtt 5760
agtgaatggt agggttagta gttaatttta gatagtttga aattttgagg ggtgttttgt 5820
atttttgttt tttttatata atttagttaa taggattttt tttaataggg tattagtaga 5880
ggaatgagtt ataatataat atagttttta gaggattgta gaattagtag atttttttaa 5940
tataaggtta gtttttagg gtaattttgg atagtttatg ttattgtata ttggtttagg 6000
gtttttgta tatattttta gtgttttgtg ttttatttta atttaaaata tttattattt 6060
tttatgtgtt tgttttttgt tatatggaga aaggagttat gttattaatt tgttatattt 6120
ttagggtttg gtatatagtg ggtgtttagt atttgatgag tgggtagatt gatggatggg 6180
tagaaatatt ttttgattta tggttttttt atgattttat tttttatttt ttggattaaa 6240
atgtaaatat tttttgataa aagaattttt tttgtgtttt aggttttggt tttggttgag 6300
agttttatat ttataagtgt ttatagagga aattaggggt tatgtttgaa tttgtttaga 6360
agggttttag aatttttgag ggtgtggtta tttttattat tttttaaagt aataaattta 6420
tttttgtaat ttaaggtttt gtttttgtta aaagataatt ttgagttaga tggtatttgt 6480
tttttattaa ttatttttt taatggattt ataggaaaaa atttttgaaa gttaagattg 6540
aatagggtga gaattttttt ataaataata atttggaatt tagagaggtt tagttatttg 6600
tttaaggtta tatagtttgt tggtatagtt ttgaattttt attttgtggt ttttagtgtt 6660
tagtttggtt gtgtttttag tggtttttta gggtatttgt aggtgatttt tttttatttg 6720
tttaatattt atgtagtatt tattatgaat taggtagtat gttggggttt gtggatagaa 6780
agatgaagag ttatagtttt tgttttgtaa aggttttttt ttttgagttg tatttatggt 6840
agttttaagt ttttatgttt taaatttgag gtttttgtg gtttgtagta gttttgtttg 6900
agtagaaggt agtttgtatt aaggttttag gtttatttgg gatttggtgg gtgatggggt 6960
tttagttttt tttgtttatt ttggagtttg ttgttgttgt tattattgaa ttagttatgt 7020
tagaaagtta ttatgatgat tttagaattg tattgtgatt tatgtaaagt gtgttgataa 7080
ttatagttgg gtgtatagaa tataggatgt atatgttgtg ggtgtgtgtg tgtagtgttg 7140
tgtttttgt tagggttgaa tggggtgtgt gtgtattttg taggtatttt tttgatatta 7200
tgtttgattg gggattggtg ataggaaaaa gagggaagat atttattttg attttgagat 7260
aatttgatga gttttgggag tattttttag aagttttggt gtttataaat gagttagttt 7320
ggtaggagtg tttgagagta ggggaagagg tggtttggag tttaggaaag ttattgttta 7380
gtttttgtat aggtaaagta attagtagtt tttttttttg aaatagattt ttaaatgttt 7440
tttttgtaga taggtttggg tttagttatt atttttata ttgtttttat ttttgtttta 7500
gataaattag ttttattttt gaagtttttt gttttgaatt aaagaatttt ataattgtgt 7560

```
ttgtgttatg ggggtggtgg tggtaggtgg ttgaagtaag tggatttttg gtgtgaatta    7620
gaatttgagg attttaaatg gagtttggta ttttattgtg ttttttaaat tttgtttgaa    7680
attttttttt tttttgggtg ttgagtttga gaaagtgtta tgttgttggt tgggttagtt    7740
ttataagtgg ttgtataagt tggttgttaa aaaatgttga tttttttttt tgttatttaa    7800
taaattttta tgtgtttatg gttttgtttt ataatttttt aattttgttt taatttgaaa    7860
aattgaggag gagggaataa attgagagat aaagattttt ttattttgtt ttttttttgg    7920
gattttagtt ttggttgtgg tgttttatta aggaggatat aggttttggt gtgtgtggtg    7980
tgtgagattt tgagtttgag gttgagttaa ggttgggtag aaagttgtaa ttatgtgttg    8040
ttggagttta ttggagtgta tagtttgttt tttttgggat gtttaggtgg aggatttgtt    8100
gtgttttttt agggtttggg ggattttagt atttatttgt atgtataggt gaattttgat    8160
tgaaagtttg ggatgatatt gagtttggag aaagagggat tggggggtgg gttggtggaa    8220
ttgtagagtg ttggttatag tttttttttt tgtgaatgtt gagtggaggg tgggaggtgt    8280
aatttttgat ttttggttgg gtttatgttt tgaggaggga ttggtaagtt tttgtttgat    8340
aagtttaagt tgttgagaga gtttaaatag aattaatttt ttagagattg gggattattg    8400
tttttttggt atgtgttttt ttagtgttgt gtatagagta aggtgtgaga gagtttagga    8460
attgtgggaa ggtaagtgga atggggaggg ggtaggggat gagggttttt ttttattatt    8520
tttttgtttg gagagtggga gtttgtaatg tttgttgagg atgagtggtg ggagggaatg    8580
ttttgttttt tagttgtttt ggtgtagata atggaggtga taagagattt gtttagtgtt    8640
ggatgggtta gttttgtttg gggaagttgg tggtattttt tttttggttg gtgtttaaat    8700
ttattgtgtg aaggttgaag gaatgtggaa tttttagaag attttatttt tagttttgat    8760
tttttgtaga tatgtgtaaa atgagtaaat tatttatttt gggttagatt taagttttat    8820
ttaatagaag gggtattgga ttaagaatga attaatttat atggttatgt ttggtgtgta    8880
taattatatg ttagtatata gttatttaat tttttttgtg aatttatttg gtattttgga    8940
gagaggggga aaagtgtttt gagaaagttt tgttattttt tttttttttt tttgttgtga    9000
aatatatgaa tttattttta ttatgagttg tattgttttt attattatgt atgtatagag    9060
ttatattttt atatttatat tttttaattt gtaagttttg atagtgtttg tatttttttt    9120
gggagttgtt tggaggttta ttaatattat tagtatttaa tttttttttt ttttttattt    9180
tttttttgta tatggttgat gttagatttt gttaggagtt gggggaaaag ttaagtgggt    9240
tagggatgtt ttattttttt ttttgtggtt gtttgttaga gtgtttttgg agatattata    9300
ggggatttag tttgtagtga taggtataaa gttatggggt aatgaatttt ggggattttt    9360
tgttgttgtg tgtgtggttt tttttggaaa tttggatttg gttgtttttt tttttggaag    9420
attttttagt aatttagttt ttttatttg tgtttgggtt ttggtagtgt ggagtttgtt    9480
tgtttttgag attgtggtag tgtttttttt ttttttttgg gagattagtg gttggtagag    9540
attgtttata ttttgtatgt ttatgtagag ggagagattg aagattgagt gataggaatg    9600
gggaaaaaga gggattttgt tttgtaggaa ggttattttt gtgtttttat ttttgttttt    9660
taatattttt tttttgttgt tttttttttt tttttagttt ttttgtttat ttttttattt    9720
gtttgtttat gtgtgtgttt atattaagta gtatttttag tagttgtggt tttgtaagag    9780
ttgggaagaa atttaaggat gtttaaattt ttattgttgg atgaatttttg agtgtttagg    9840
gagtagtgta gtgtgtgatt gatatttatt tgttttgttt aggagttttg taggttggag    9900
ggtagttgga gagtggtggt gtttggtggt gaggtgggtg ttgttggttg ggatttgggt    9960
agtgtttatt aattgttttg ttttgggata gttagtatga gtaagttagt tggattaata    10020
agtgggtatt ttttgggttg ttgtggggtt taggtgtgta gtttggggtg agtgagtggg    10080
gaggttgggg gaggttttgt ttggagtgtt gtgaatttga gtttttgaga gggattttag    10140
tgggtgtgtg tgtttggttt agatttgtag attgtgagtt ggagtatttt gtggagaggg    10200
gagagttgtt ttgttgtttt tggattgttt gatttttttt gtttggtgtg gtgagaaggt    10260
tatgatttgt gtagtttatt agttggatga gttgttttta tttagttgtt aggtgttgga    10320
tggggggggtt atgggggtgg gaattgggtt gtagttttag gtggtagtat aataatatat    10380
ttgtttaaaa ttttgagttt tagtgtgtaa aagtaatttt gtgtaaagtg gattttgaat    10440
ggaatgtttt gtattttgtt tttagttatt ttaaataatt ttgtaaattg ggaagtagaa    10500
ataatttaaa agttatattt tttttaattt taaatttgtg taggttataa ttggggaatt    10560
taaagtatgg tgaattattt tagtaaagag aggattaaat ttttaatttt aaggattttt    10620
tgagttggag tttagtagag gtaggagtgt gtggtttgtt tttttgtgt gtttttttt    10680
ttttttgaat ttttttagtt gttggttttt tgaatgttag gttgtagttg atttttttatt    10740
attttgagat ttatgagtgt agggttaagt gtgtgtgtga gggtatttgt ttgtattttg    10800
tttgtggaat ttaagaatgt gtaggtttga gttagtgttg agtaggtgtg gttatggtgt    10860
ttagattttt tgggggtatt ttagttttg ttatttagtg gtttatggtt gtgggtttta    10920
gggtttgagg ttggggatta ttgttgttgt tgtagttttt atattttgaa gttgttttgt    10980
tgtttgtgtt gtttttgtag atagtatttt tggtgttttg tgtgtttttt tttttttttt    11040
ttttttttta tttgttttta ttgttttgag tttttgaaag ttgggagaat tggagatatt    11100
tttgaggatt ggtaatgaag ttttatttaa gtgggatgta atttttgttt ttttattttt    11160
ttttaagaag gaggttgtgt ttttatttg ttttgttttg ggtgttgatt tttaaaaaat    11220
```

tagagtaaaa tgaatgtgaa taaaaagaaa aggagaaatg ttttgagttg gggtagaggg 11280
agtagagaag gagtttttat tgtggttgga atgtagagtg gattttggtt taggattggg 11340
ttttttttta ggtttgggtt tattttggtt tttgttgttg gaatttttag gaggtaaagt 11400
gattttgatt tttgttgttt gtatttttgg gtgtgagtgt tttttttagg aggtttagga 11460
ggttgttttt gttgtatttt gagtttttgt tgtaaaaatt gaagtttgtg ggttttggta 11520
ggttttttag tttgtttgtt ttgggatagg tttttgttta tatttttgga agtaaggagt 11580
tttgggtttt tttgtttttt ttggggtgtg gagtttttgg ggtttttgaa aggtgaggtt 11640
ttagaggtga gggaggggtg agtggggagt tttgtttgtt tgtggttgtg tttttgttgt 11700
ggattaggag gtaggttaat tttttggatt ttgggggaaa aattatagtg ggttttttgt 11760
ggaaattttg gttgttttaa tttgttaaga gtttgagtga ggttttggaa gtttttagtt 11820
ttggtttagg ttgggatgtg gttgttgagt ttttttaggt ttgtaggata gtggttttg 11880
ttggtggtgt tgttgtattt aggttttttt tagattggtg gtggtagtta atttgagatt 11940

tgtgtttttt gggtttgggg tagttaggag gttggtgtgt agtgggttgg gttaggattg 12000
ggttgagtag atagagttgt agtttttgtt ttgtttggtt ttttgtggtt ggagagtaga 12060
gtgatgttat ttggagtttt gtttgggtgg taatgagatt ttggttagtt atttttgtag 12120
tttagattaa tttttttttaa tagtttttga tggtaattat agtaattgaa gttgttatat 12180
atttttaggt aattatgata tataaaaagt tttgagggga ttttttggtg agggaagtta 12240
agaatggttt tttagtttta ggaaattttg gtttgtttta tgttggagtt tgtttggttt 12300
gttaaatgag aggagttttg taatggggtt aattagtttg ttttgtgatt ttaagttatt 12360
ttaatgtggt tgggtggtgg agtttgaggt ataggtttgt tatgttttgg aatttttgtg 12420
tttttttttt ttgggttttg ttttagtttg gttgtttgtt tttaatttgt tttgggagtt 12480
gtggtttaga ggtttgttta gaggtaggat ttgtattggt ggtggtttag agggtaatag 12540
tttggaagtt tgggtggggg aatttgagga ggggttttta ttttgtattg gttttttttt 12600
tatttttatt gggtttttta gagatgttgt tatgggtttt ttgtttggat gggaagtggg 12660
gaaaaagaga ttttattatt gtttttttta tttatttatt ttggtatttt taatagtgtt 12720
atagggaaga ggatagttgg ggtgatagta ttgttgggtt ttaaatgtgg attttggagt 12780
ggttagaggg tgtgtgttaa gtagaggatg gttgtaagat atggaaatta agtttattgt 12840
ttttagagtg agtgtagtgt agtggagttt tagatttggt ttttgagtga ttaggaattt 12900
ttttgggtgt agtgataggt ttgaaggagt ggaggataag atttgttgtg tttttgtttg 12960
gtttttttta gttttttttt tttatagtat aatttttttt tttgttttgt gggagggggg 13020
tgttgagttg gtggttgttg tgttgtgtgt tttttttgtt tgtttttgtt gttgtttgaa 13080
tttttatgtt atttttttttg atgggttatt ggtggtttta taggtaggtg ttgttgtggg 13140
gttgtaatta tttggttgag tttggttgtt attgaatatt tttttatttt taagtatggt 13200
tttttgtgtt tttttttttt tatttttttt atttgagatg agtttgggat ttatggtgta 13260
agtttgtttg gatgttttgg gttttaggag taagtttata tttgtggtgt tttgaggttt 13320
tttttgtgtt atgtttttat ttttttttatt tttttttgttt agatgtttta ttgttgtttt 13380
tttttatttt aattttattg tagtttttaa tttaagtgtt tttggtagta gttgttgaat 13440
tttagggagg tttgaggttg ttggggttta aattggaggt ttttggatag gtagaagaag 13500
agaaaagtaa gtgttaataa gttaattgtt attaatt 13537

<210> 292
<211> 13537
<212> DNA
<213> Artificial Sequence

<220>
<223> chemically treated genomic DNA (Homo sapiens)

<400> 292

agttgataat aattgattta ttaatatttg tttttttttt tttttattta tttgaaggtt 60
tttagtttgg gttttagtgg ttttaggttt ttttgaaatt tgatagttgt tgttgaaagt 120
gtttgggttg gggattatga tgggattgag gtgggaaggg gtggtagtgg aatgtttggg 180
tgaggggagt aggggaggtg agggtgtggt gtggggaggg ttttgaggta ttgtaggtgt 240
aaatttgttt ttggaatttg aggtgtttgg gtgagtttgt attgtgggtt ttagatttgt 300
tttaggtgga gggggtgggg gaaggagagt gtgggggatt gtgtttgggg gtgagggggt 360
gtttggtaat gattaggttt ggttgggtaa ttataatttt gtggtagtat ttgtttatag 420
agttattagt gatttgttaa aaagagtagt atggaatttt ggatggtgat aaaggtgggt 480
aggggaggtg tgtggtgtgg tggttattgg tttggtgttt ttttttttata gggtgggagg 540
ggagattgtg ttgtgggagg gaggggttgg agagggttag gtaagggtat agtgggtttt 600

```
gttttttatt tttttgggtt tgttattgtg tttaagggag tttttggttg tttaggaatt      660
gggtttggag ttttgttgta ttgtatttat tttggaagtg gtaaatttgg tttttatatt      720
ttataattat tttttgttta atatgtgttt tttgattgtt ttagggttta tgtttggggt      780
ttagtagtat tgttatttta gttgtttttt tttttgtggt attgttgaag atgttaagat      840
gagtgagtgg gaggagtagt ggtggggttt tttttttttt atttttttatt tggataggag      900
gtttatgata atatttttgg agaatttgat gagggtggag ggaagattag tgtagggtgg      960
gggtttttttt ttggattttt ttgtttgagt ttttggattg ttgtttttta ggttattatt     1020
agtgtgagtt ttgtttttga gtagattttt gagttgtggt ttttggggta gattagaaat     1080
aggtaattgg gttggggtgg ggtttaggag ggagggatgt gaaaattttg gggtatagtg     1140
ggtttgtgtt ttagattttg ttatttagtt atgttaaggt gatttggggt tatgagataa     1200
gttggttgat tttgttgtaa agtttttttt atttagtaag ttgagtgagt tttgatgtga     1260
ggtgagttgg agtttttttga agttggaaaa ttgtttttaa tttttttttgt taggggggttt  1320
ttttggggtt ttttgtgtgt tataattgtt taaagatata tggtagtttt gattattgta     1380
attattatta gaggttgttg aaagaagtta gtttgggttg taggggtgat tggttagggt     1440
tttgttatta tttaggtggg gttttgggtg atattgtttt gttttttagt tgtgagaagt     1500
tgggtaaggt gggggttgta gttttgtttg tttgatttag ttttgatttg gtttgttgtg     1560
tgttgatttt ttagttgttt tgggtttgag ggatgtaagt tttgggttgg ttgttgttat     1620
tggtttggaa agggtttaaa tgtagtggtg ttattggtgg gggttgttgt tttgtgggtt     1680
tgagaaagtt tagtagttgt gttttgattt gagttggggt tggaggtttt taaggtttta     1740
tttaggtttt tggtaagtta gaatggttaa agtttttgta aggagtttgt tgtggttttt     1800
tttttaaagt ttggagggtt ggtttgtttt ttagtttata gtgggggtgt agttgtaggt     1860
gggtagagtt ttttgtttat tttttttttg tttttgaggt tttatttttt aagggtttta     1920
ggggttttat attttgaaaa ggatgaaaga gtttggggtt ttttattttt aggggtgtag     1980
gtgagggttt gttttggggt gagtgagtta ggaggtttgt tgagatttat gggttttagt     2040
ttttgtaatg gaggtttaga atgtaataga aatggttttt tgagtttttt gggaaggata     2100
tttatgtttg ggaatgtggg taataaaaat tgaggttgtt ttatttttttg gagattttaa    2160
tagtgagggt tagggtaggt ttgagtttaa agggaaattt agttttgggt tagggtttgt     2220
tttgtatttt ggttgtggtg agggtttttt ttttgttttt tttgttttag tttgaaatat     2280
tttttttttt tttttgttt atgtttattt tgttttaatt ttttaaaggt tagtatttaa      2340
agtaaaataa aatgaaaata taattttttt tttggagggg ggtaggaggg tgggaattgt     2400
attttattta agtgagattt tattattagt ttttagaagt atttttgatt tttttaattt     2460
ttaaagattt aggataatga gggtgagtga aagggggagg gggagggaag gaatgtatag     2520
agtgttaaaa atgttatttg tgaaaataat ataagtagta aggtaatttt gggatatggg     2580
gattgtggtg gtaatggtag tttttagttt tagattttgg agtttgtagt tgtgggttat     2640
tggatggtgg gaattgaaat gtttttggga gatttgagta ttgtgattgt gtttgtttaa     2700
tgttggtttg ggtttgtata tttttgggtt ttgtaggtag ggtgtaagta aatgtttttg     2760
tatatatatt tagttttgtg tttgtgagtt ttggggtggt gagaggttag ttgtggtttg     2820
gtgtttggag agttggtagt taaggaagtt tgaggaagga gagaagtgta tggagggagt     2880
aggttgtgta tttttgtttt tgttgggttt tggtttgaaa agtttttggg attagggatt     2940
tggtttttttt tttgttagag tggtttgtta tattttaaat tttttagtta tgatttatgt    3000
ggatttagga ttaaggaaaa tgtgattttt aaattgtttt tgttttttag tttgtaggat     3060
tatttgaaat agttagaaat ggggtgtaaa gtattttatt taaaatttgt tttgtataga     3120
gttgtttttg tgtgttggag tttggagttt tgagtgagtg tgttattgtg ttattgtttg     3180
gagttgtggt ttagttttttg tttttatggt ttttttattt agtatttggt ggttaaatgg    3240
agataattta tttgattggt gggttgtgtg ggttgtaatt tttttattgt attagatagg     3300
ggggattaag taatttagag gtaatgaaat ggtttttttt tttttatgaa atgttttaat     3360
ttatggttta taggtttggg ttgaatgtat atgtttgttg gaattttttt taggggttta     3420
gatttgtagt gttttaggta ggattttttt tagttttttt gtttgtttgt tttaggttgt     3480
gtgtttaggt tttatggtgg tttgagaggt atttatttgt tgatttggtt ggtttgttta     3540
tgttggttgt tttggggtgg agtggttggt gggtgttgtt tgagttttgg ttggtagtgt     3600
ttgttttgtt gttgggtgtt gttgtttttt agttgttttt tagtttgtaa ggtttttggg     3660
tgggataggt gggtgttagt tgtgtgttgt gttgtttttt gggtgtttag aatttgttta     3720
atagtggaaa tttaagtatt tttaagtttt tttttggttt ttgtaaaatt gtagttgttg     3780
ggaatgttgt ttgatatgga tatatatatg gatagataga tggagagatg gataggaaga     3840
ttgaggaaga aggaagaata gtaagagagg gatgttgaaa gatagagatg gagatatgaa     3900
aatggttttt ttatggagtg aaattttttt ttttttttat ttttgttatt tagtttttga     3960
tttttttttt tatataggta tgtagagtgt gggtaatttt tgttgattgt tggtttttta     4020
aggaaagaag gaaaatatta ttgtagtttt agaggtagat gggttttgtg ttgttggaat     4080
ttaagtgtag agtgggaaaa ttagattgtt gggaaatttt ttgagggaag aggtggttag     4140
gtttgggttt ttggggagag ttgtgtatgt agtggtgaag ggtttttgaa gtttattatt     4200
ttgtgatttt gtgtttgttg ttgtgggttg ggttttttgt aatattttta gaagtgtttt     4260
```

```
gataggtagt tgtggggagg ggaatagggt gtttttggtt tatttagttt tttttttaat    4320
ttttggtggg gtttgatgtt agttatgtgt ggggagggga tgggaagagg gagggggtta    4380
aatgttaatg atattaatga atttttaagt ggtttttaaa gaaaatgtag gtgttgttgg    4440
agtttatgag ttagaaagtg tgaatatggg agtgtggttt tgtgtgtgtg tgatggtgag    4500
gatggtgtgg tttgtaataa aaatgaattt gtatatttta tggtaagaag gaagggggagg   4560
ggtgatgggg tttttttaaa atgttttttt tttttttttt agagtgttag atagatttgt    4620
ggaagaaatt gggtggttgt gtgttggtgt gtgattgtgt gtgttggata tggttatgtg    4680
agttggttta tttttggttt ggtgtttttt ttgttgggta aaatttggat ttggtttgag    4740
gtgagtaatt tatttatttt gtatatattt atggaaagtt agggttgagg atggggtttt    4800
ttggaggttt tgtgtttttt tggtttttgt gtagtgggtt tgggtattag ttgaggggag    4860
gatgttgtta gttttttttaa gtagagttgg tttatttgat gttgagtgaa ttttttgttg   4920
tttttattat ttgtattggg gtggttggag ggtagagtgt tttttttttgt tgtttgtttt   4980
tggtgggtgt tgtgggtttt tgttttttgg gtggaggaat agtgaagaga ggtttttatt    5040
ttttattttt tttttatttt gtttgttttt ttgtgatttt tgagtttttt tgtgttttgt    5100
tttgtgtgtg gtgttgggag agtgtatgtt gagggagtga tgatttttga tttttaagag    5160
attaatttta tttaagtttt tttggtagtt tgaatttgtt gaataagagt ttgttagttt    5220
tttttttaggg tgtggatttg gttagaggtt agaggttata tttttttgttt tttgtttgat  5280
gtttgtgggg aggggagttg tggttggtgt tttgtaattt tgttagttta tttttttggtt   5340
tttttttttt tagatttggt gttatttttgg gtttttaatt agagtttgtt tgtgtgtgta   5400
agtgaatgtt ggagtttttt gagttttggg agggtgtagt aggtttttttg tttgggtgtt   5460
ttagggggag tgggttgtgt gttttagtgg gttttgatag tatgtgattg taattttttg    5520
tttagttttg gtttggtttt gagtttgggg ttttgtatat tatatatatt agagtttgtg    5580
tttttttttag tggggtgttg tgattaaggt tggggttttg gggaaagagt aagatggggg   5640
gatttttatt ttttagttta tttttttttt tttggttttt tgaattggga tgagattggg    5700
ggattgtggg atgaggttat aagtgtgtag gggtttatta ggtgatagga ggagaaatta    5760
gtgtttttttg atagttaatt tgtatagttg tttgtggagt tagtttgatt ggtggtgtag   5820
tgtttttttg aatttggtgt ttagagaggg gaggaatttt gggtaggatt tgagaaatat    5880
aataagatgt taggttttgt ttggaatttt taggttttgg tttatgttag aaatttattt    5940
gttttagtta tttattatta ttattttttat aatgtggatg tgattatggg gtttttttggt  6000
ttaggataaa gaattttaag ggtggaatta atttatttgg ggtgaagatg agggtagtgt    6060
gggggtgat gattagattt aggtttgttt gtaggggaag tatttagggg tttgttttga     6120
gaaggagaat tattaattgt tttgtttgtg tgggggttgg atgatggttt ttttgggttt    6180
taaattattt ttttttttat ttttgaatgt ttttgttagg ttggtttatt tgtaggtatt    6240
aaaattttttg gaaaatattt ttaaggttta ttaaattgtt ttaaaattaa aatgggtatt   6300
tttttttttt tttttgttat tagtttttga ttaagtatgg tgttaagaag atgtttatgg    6360
aatatatata tattttattt agttttggtg aaaggtataa tattgtgtgt atatatttgt    6420
agtatatgta ttttgtgttt tgtgtgtttg gttgtaatta ttggtgtgtt ttgtataaat    6480
tataatatag ttttgaaatt attgtgatga ttttttggta tgattggttt agtggtaatg    6540
gtggtaataa gttttagggt aggtgggagg ggttgaggtt ttgttgttta ttaggtttta    6600
aataagtttg gagttttagt gtgggttgtt ttttatttag gtaaagttgt tgtaggttat    6660
gggggatttt ggatttaagg tgtagaaatt tggggttatt atgggtatag tttagagaag    6720
aaagtttttg taggataaag attatggttt tttattttttt tatttatagg ttttagtatg   6780
ttgtttgatt tatagtaggt gttgtataaa tgttgggtaa atgaagggaa gttatttgta    6840
gatgttttga ggggttattg gaggtatagt taggttaaat gttggaaatt atgagatagg    6900
gatttagggt tgtgttaata agttgtgtga ttttaggtga gtggttagat tttttttgggt   6960
tttagattgt tatttgtaaa aggattttta ttttgtttgg ttttaatttt taaagatttt    7020
tttttgtgag tttattaaag gaaatgattg ataaggggta aatgttattt ggtttagaat    7080
tattttttaa tagagataag attttaagtt ataaggataa gtttattgtt ttggaaggtg    7140
gtggggatag ttatattttt agaggttttg aaattttttt aaataggttt agatatagtt    7200
tttaattttt tttgtaggtg tttgtgagta tggagttttt aattaaaatt aaagtttaaa    7260
atgtagggaa ggtttttttta ttgaaggatg tttatatttt ggtttaagag ataggaaata   7320
agattatgag aggattatgg attaaggggt atttttgttt atttattaat ttatttattt    7380
attaaatatt gagtatttat tatatgttag attttgggga tatagtagat taatgatatg    7440
attttttttt ttatgtagta gggagtagat atataaaaga tgataaatgt tttagattgg    7500
gatgaaatat aggatattgg agatatatat aaggagtttt aaattagtgt gtggtgatat    7560
aggttgttta gaattgtttt ggaaagttgg ttttgtgtta ggagaattta ttgattttat    7620
agttttttga ggattgtatt gtattatagt ttatttttttt gttagtgttt tgttaaagag   7680
aattttatta attaggttgt atgagagagg taggggtata gagtattttt taaagtttta    7740
gattatttga ggttgattgt tagttttgtt atttattagt ttttttaaaa atggtgatat    7800
ttttttttatt aatatttttt ttatatatat atattttttt aaattaaaat attgaaggtg   7860
aaaggataag ttttttttatt agttagtaga gttgatggaa ttggtgttaa agagattttt   7920
```

```
tttttaattt tttgtgattg attttttata gaggttattt aatttttaga gagttatttt    7980
tttaaaatat tatttttttt attgatatgt agagttaaga gatgtttaga ttaaggaaat    8040
tgttgggaaa atttattagt tgagtttggt tgagttttga tgtttagtat ttaaaaatat    8100
tttgatataa gtttttgttt gtttgtttgt ttgtttgttt atatgaagga ggaagttgat    8160
ttgtatttgt ttttatatta taatttaatt tatggttgtt gtttgtttag tggatggtga    8220
gagaggaggt gggttatgga gtagtgtttg gtatatagta ggtgtttgtt aagtgttaaa    8280
tgaaagaaat agtgtttgta ggtttgtttt tgattttgtt gtggattttt ttataagttg    8340
tttgattttt atttaaattt attttttaaa taaataagaa taatttagaa aagagatgtt    8400
tgtttttttag aatttagtgg gttttgagtt gtagggtttt ttttgatatt tttgagtatt    8460
gtattgttaa aagtagtttg ttattttttta taagtaggaa gatgaatgtt gttaataata    8520
aggagataat gttttgtgtg attatttttt ttattttata attttaataa ataataagtt    8580
atgtttttgt atttatttat atttttggtt ggataaaaat ttagttttaa agttaggttg    8640
agattttgag gtttttagag ttttgttgta ttttgatatt ttttggtttt aagaataatt    8700
aagtagaaat atttttttag ttttttgtat tttggtatat agtggtgttt tgtaaatgtg    8760
gaatgtatga gtgaatttgt ttgatgatat ttaatgaaag ttattgaagt ttgaggtgaa    8820
gggtttattt ttgtttagtt ttagtaatgg ggtaggaagg gaatagggat aggtttttttg    8880
aatttttaaa ttttgggaag tagtgtaggt tatttttggt tttttgaaaa taaaagaatg    8940
attagagaaa taagaaagta gaatatagat tatatttagg gagatattgg ttgttaagtt    9000
ttttttagtt ttttaatttt ttgtgaatta tatagtttaa tttggttttt atttagtgtg    9060
gaattataga gtatataagt tataaggtat ttttttaatg tagttatttt atagatgagg    9120
aaagtgagaa ttttagagat aatttgtttt tgtttattta ttgagagata gagattattt    9180
ttagaattta tattttttga tgttgagttt agtgtttttt gaaatattta ttttattgta    9240
ttttattgta tttagttatt ttttattttt tagaagggtt tttagaagtt ttaattaaag    9300
taattttatt tatttattag tgaagtaatt agaattaaga atagtgtttt gtgtattttg    9360
aaattatagt ttagtatttg tgtaatgtta gtttttttta ttttataggg ttttttttta    9420
aattttttttt ttgttagtat ttaaaatttg ttgtgtttta aggtatagga aatttgtgag    9480
gtgttttttt tgttttatag aagggatgat atgatttata ggagtaatat ttagagatat    9540
aaattataga tgttgagaaa aaggtaatga ttagattgat taattaattg ttgaatttga    9600
tttgtttgga ttgttaattt aggtttttta gattttaaaa atgtttttgt tttttttttta    9660
tagtagtttt aggatttatt ttatttagat tattgtttta atatttgtag aaataattgt    9720
tatgtaaaat taggtatttt agtttgaatg tggttaatat attttagaat aagttataat    9780
tttttattat gtggatgagt atattaggat tatatatttg atttttgtta atgaagtgaa    9840
aagtattata tattttatta tgttttgttt ttaatagatt tagtttatat agtattttgt    9900
tgttttattt tagtaagtaa ttttagaatt tatattattt ataaagatgt tgagtggtta    9960
tttagggaat tttgatttaa aaaaaaaaaa aaaaaatttt ttaaaataaa ataaattttt   10020
tttttttttt tttttttttt aaattttatg tgttttgtgt aagatggagt taattagtgg   10080
gatttatagg aggtttttaa gttaaatgtt gtagttgtgt tttaaaaaag ggttggataa   10140
attatagttg ttaataatat ttgtatttat gtaagttaag atgatgatag gggtaattag   10200
attttatgtt ttagggtata agatgattgt ttgttgggag gaggaggatt tggtttttta   10260
ggtataggag tttataattg aggaggtagg ttgtagatat ttttttttag tttttaaaat   10320
tttaggtgta attgttggga taggtgaaat agtgatttga aatttaatat ttttattaga   10380
ttaggatatt aaataatgag atatttgaaa tattaatatg tattatatat aatatatgtg   10440
tatttattta ggatataagt attttgatat ttttttggaa ggttgatatt gtttgatata   10500
ttttgattat tttttagtgt taagtggata tttgttaagt attaaataat aatgaaaata   10560
gtggaggtaa tttgatttta gtttatgtat taatagttag ttataatgtg taattattta   10620
agtatttata attgtatata tttgtatttt taaatatatg tatttttagt tgtagtagtt   10680
aaataattat tggaattgta tatttggagt gtatagaaat gtatttattt taatttttgg   10740
attattaagt aatttttaaa atgtaatatt tatttggaag agtgagaaag taattttgta   10800
aaataatttt attttttttt gattttttta atattttttaa attttaagaa aggtatattt   10860
tagtatagtt gtataattta attaatttttg aaaatattag tgtaagtgtg agtagggtta   10920
aataatttta tttatttaga tatgtaaatt atattgaagg ttatatagta gtagatagtt   10980
tttatatttt agagagtaaa aatgtagttt atttttagta gagtggttag agtttttttg   11040
gtatgaaaga tatttttatt atttattggt tttattgtta ttgttaaatt atttatttta   11100
gttttatata gagggattgt tttggagaat atggttgtta aatgtaattt ttaaaatgat   11160
gtataaatag tttttaaagg gggaaaaatt ttgtagttgt ttggttgtag atagatatgg   11220
gaaattagtt agaagtttaa agtattatag ttaataatta ttattttgtt gtttgggatg   11280
tttgtttaaa tattattgat attgtgtatg tattgttatt ttttttaaat tgtagttttt   11340
aattattgaa attttgttaa agattattta gggttttttt tgtatgatgt tgaagtgtgt   11400
atttatttga tggataaata aaaaatagat tttttttttt ttgttttaat tttttttttta   11460
tgtgtaatta gtaggaaatt ttttggatta aagggagaga ttgtttaagt atagtagaat   11520
tagatgttgt ggatttttaa tttttgtatt ttttatttag tgatagaaga tgaataaatt   11580
```

```
tattaattga ttaatataat ttagttttgt ttaaaattta tagatttgtt tggtatgaga   11640
ttgtatttta aaagattttt atatgttatt aagtgtgaag aaattagtaa ttattttttt   11700
tatttatagt atgttgagtt tttttttatt ttatttaaat tttgtttatt aagtgaggtt   11760
atttgggaga agagtggttt tggtgggtgg ggaaaagaaa atttaatagt ttaattttta   11820
agttgagtgt tttagattat gggttattta gtataaagtg ttttatattt tgatttatga   11880
ggggataata gttggtttta taaaggtatt atttgtgatt tagtatggtt gatatttttg   11940
ttaaggagga gttggagggt agtggtgaaa tagtatgtgt atgatttgga ttgtattatg   12000
gtgattgatt gatttttaag attgtttttt gttgttgtgg aggtttggtt aggtttttttg   12060
gtttatagtt tttggaaagg gtgtggggag agagaaagga aatgagtgtt gtatatgttg   12120
gtggagttaa agtttttgga tttatggttt gttagtgttt agttgtttta ttttatagat   12180
gggtaaaatg gaggttaggt ttgtggagtg tttggtttag taggtgtagt ggtttggatt   12240
tttgtggggt tttgggattg tagggttggg ggtgtggggg ggtggtgtgg ttgtgggtgg   12300
gatgtagagt ttttgttttg ggtttaattt ttgggttggt ggttttttgt tttttttttg   12360
aaggttgttt ttgataaatt tatttatttg gggtaaattg ttagtgagaa atttttgttt   12420
gaattgtttt ggataaaatt ggttgtttga aaagggtaaa ttttgtggtt tggtgaggtt   12480
gaataatggg ttgttggtgt ggttgtggtg ggggtggttg ggtgggttgt ataaagggtg   12540
gattaattgg ggttgtggtt gagtggtttg tgttagtttt atttagtttg tgttgtgggg   12600
tgttttattg attgggtttt gagttttatt ttttataaat tttaaataaa agttaatttt   12660
ttttttataa ggtgggggag ggggttgtgg ttaagatttt ttatttgttt ttgtgatttg   12720
tttttgttgt atgggggggtt ttgggggttt aaggaattgg aggtagaagg gtttttgggg   12780
atgtttgaga ggggaaagag atgtgggtgt ggttgttgtg tttttgtttt tttttttgtt   12840
gttttgtttt gatgtgtgtt gtattttttg ggttgtattt ataggtaggg gttgagattt   12900
tagtttgagg tttggaagtt gtgaagaagt gggatgaggt ggtggttgga ttgaggtgaa   12960
ggttggggta tttggttggg ggaagtgtag gtaggtgatt tgggaggaaa gaatatgttt   13020
ttttgatatt ttaaattttg ggaagggtag ttatgtatta gatagttatt gagaaattat   13080
tgtgtttttg atggtgaatt tgtttttaat ggatagataa gtgggtgggg ggtagattta   13140
taagttaatt agttagtagg tgagggattg tggttagtat ggggtgttaa aggaagttat   13200
tattttttttg taaaatgatg agatggattt gttggtgtaa tatgtagaag atatttaatt   13260
atagatatag attttgtatt tggagaagtg gtggtgatta gaaaataaat aaataaattt   13320
aaattgtagg attaatattg ttattggttt atatattttg taggaaggaa ttaggagttt   13380
gtttttttttt gtttttttttt ttttaaaata ggagaatgat tgtagagggt tttttagttg   13440
atattgagag gagtaggaaa tttttttaat aatataaatt gatatatttt ttagtttata   13500
agtttttgtt ggaaattgga ttggtggtgg ttgtata                            13537
```

<210> 293
<211> 2567
<212> DNA
<213> Artificial Sequence

<220>
<223> chemically treated genomic DNA (Homo sapiens)

<400> 293

```
ttattgaggt gttatatgta tttaaaaatt attattagaa agagaatttg aatttttttga      60
tttagagttt ggtttattga atttatttaa gttttattta gtgttagatt tatttggtat     120
tttttttttt ttagtgagga aattttgatt aggtttaagg taggaaggat aaatatgagt     180
agagaggtaa aaaaagttag aattaaattg taagaggttt aatgtggtat ttatattaat     240
ttattggttt tttttaatat tatatttata tatagttatt tggtttttttt tttattgtgg     300
ggtgtgagag gtgatgagta ataattttttg gttatgtttg ataattttgt agttgttggt     360
ttttgagatt aagggaatat tatttatttg tttttatttt gttttaattg ggttgtagat     420
ttttatgatt gggatattat agatataata gattttttta ttgaagatgt aggtttttttg     480
aaagttattg tgttttagag aaatgtattt tagttatata ttgtggttag gattataata     540
gaatatgtgt ttattgttga gagtatagag atagttttga attattatta ggttaaagga     600
taaaaaagaa tggggtagtg gagttattaa tgtttattgg ttttttttaa tattgtagta     660
ttttattttt tttaatttaa ttttagtaat agggttttgt tttttttaaaa tgtagatata     720
gttattgaga tatgttatat tttatgtttt atgatatagt aagtgatttg taagttgttg     780
ttaattattt tgagttggtt tatatattta gaggtttgtt ttgggttgag tagttagata     840
gatgttatgg ttaggagaga tatagatagt taaagtggtg atagttttag tgtgagttag     900
ataggttaaa ggtgatggta ttttgtaaag gttttttgag tatggattat agtagagaaa     960
gggatttttg gggtgtttaa agaagattat tatttttttg gtatatatat ttagtttttg    1020
```

gggtggattt tttgttgtag atataagaga gttgttgttg ttattgttgt tgttgttgtt 1080
tggtattggt attagagatt tgtataatag gttattatag tgtatttgta gggtttttt 1140
aataatgttt aggtagtatt tttttatgat gggtttggtt agtagttttt ttaagtagtt 1200
ttgatttttt ttagtgaagt gtatttagtg tatgtatttg aagattttg tagtattggg 1260
attttgtttt ttgggagtag tttttagtta ttgtattgtt atatggtata ttgtttgttt 1320
attttttatg tttagattat ttaagtgtag gatagttagt agttgggtta gggttagatt 1380
tgttagagtt ttttttgaa ttgaatttat gtggttgagt tgtttgaagt tttgagttat 1440
ataggattgg gtttgggatt gtagtttgtg atggttaaag gtatttgtaa atttgaggat 1500
ggtggtgtag ttggttaggt taagatgttg ggttaagaag gaggtatagg ttttttgtat 1560
atattttagt tgtagtatgt tggaggttgt gtataggtgt tttatgttgg ttttattgag 1620
agatatatga tttgtgtagt agtagttgat taatattttg aaggatttgg tgtttatatt 1680
gtgtatggtt atgttggttt gttggttttt gtatatgtta tttgtgaata tgtttttgaa 1740
gtagggatat gttgtggtta gtatattgtg gttgtagggg aataggtgat ttgtgttagg 1800
tttgttgtta ggtgttatta ttttgatggt tatattatat agtagttgtg tattgtagaa 1860
ggatttgagt tgtgttagta gggttgtaga atgggttgtg tttttttaatt tttttggatt 1920
tgtgaaaaag gttgaaattg tgggtttgtg aatttttttg ggttgttttt tattatgggg 1980
attggttagg tggtgagagt gtggggtgtt tttttgggat tgtatggtgg agatggtgat 2040
gggtgttgat ggtgagaaat gttgtaggat ttggttttgg ttttttttta atttttttttt 2100
gttgatgtta agatagtagt gtttttgaag agatagtagt atgagtttat ttattgaatt 2160
taattttatt ttgtagaatt gtttttgtt attgtttaga tagtggttga tttatttttt 2220
tttattttg tgttttttt ttgtgtttgt ttgtttttat aggatttgtt ggtttggagt 2280
tgtagaagtt gttattgtag tgtgggtgat aatgttaggt gtttaggaga attattgttt 2340
ttaggttgtt ttttgtgttt tgagggttgt tgggattttt ggatttttgg gttgagattg 2400
agggaaagaa tttgttttgt atgttgggag tagtagtttg tgtaatattg tgattttttt 2460
ttggtgtttt ttgttttagt tgatgatatt tgagagtgtt aattttttta gatttgattt 2520
tgtggtttgg aattgtggtt ttggaattta gagtagttgt tttgttt 2567

<210> 294
<211> 2567
<212> DNA
<213> Artificial Sequence

<220>
<223> chemically treated genomic DNA (Homo sapiens)

<400> 294

gaatgaggtg attattttgg attttaaaat tatagtttta aattataaaa ttgaatttag 60
gaaaattggt gtttttgagt gttgttagtt aaaataagga atattagaga aggattgtga 120
tattatataa attattattt ttgatatgta aagtggattt tttttttgg ttttggtttg 180
ggagtttgag agttttagta gtttttgggg tgtgaaaggt aatttgggag tggtagtttt 240
tttgggtgtt tagtattgtt gtttatgttg tagtagtggt ttttgtggtt ttaagttagt 300
gggttttgtg aaggtgagta gatgtggaga aaggatgtgg gagtgagaga gggtgagtta 360
gttattgttt aaatgataat gggaggtggt tttgtggggt agggttgaat ttagtaaatg 420
ggtttgtgtt gttgtttttt tggagatgtt gttattttag tgttagtgag ggaaggttga 480
ggaggagtta gagttgggtt ttgtagtgtt ttttgttatt agtgtttgtt gttattttta 540
ttatgtagtt ttgggaagat gttttgtgtt tttgttgttt agttagtttt tgtggtggga 600
agtggtttaa gaagatttat aaatttatag ttttggtttt ttttatgggt ttagaggaat 660
taaaggatat ggtttatttt gtagttttgt tggtatagtt taagtttttt tatgatgtgt 720
ggttgttgtg tgatgtgatt attgaggtgg tgatgtttgg tagtgggttt ggtatgggtt 780
gtttgttttt ttgtaattgt aatgtgttgg ttgtggtatg tttttatttt aagagtatgt 840
ttataggtgg tatgtatgag agttagtagg ttagtgtgat tatgtatgat gtggatgttg 900
agtttttga ggtgttggtt gattattgtt atatgggttg tgtgtttttt agtgaggtta 960
atgtggagtg tttgtatgtg gtttttgata tgttatagtt ggaatatgtg tgggaagttt 1020
gtgttttttt tttagtttga tgttttgatt tgattaattg tattgttatt tttaagtttg 1080
tagatgtttt tggttattgt aagttgtgat tttaggttta gttttatata gtttagaatt 1140
ttaagtaatt tagttatatg ggtttaattt gggaggagat tttagtagat ttgattttgg 1200
tttagttgtt ggttgttttg tgtttggata gtttggatgt ggagagtgag tagatagtgt 1260
gttatgtggt agtgtagtgg ttggaggttg tttttaaaga gtggggtttt agtgttgtag 1320
aagtttttaa gtgtgtgtgt tggatgtatt ttattgaaga agattaggat tatttagaag 1380
ggttgttgat taagtttatt gtgaagaagt attgtttgga tgttattgaa ggggttttgt 1440

agatgtgtta tggtgatttg ttgtataagt ttttggtgtt agtgttaaat agtagtagta 1500
gtagtagtag tagtaatttt tttgtatttg tagtagaaaa tttattttag agattgggta 1560
tgtgtgttaa ggagatggtg atttttttg gatattttag agattttttt ttttgttgtg 1620
atttatattt gggggatttt tataaagtgt tgttattttt gatttgtttg gtttatatta 1680
ggattgttat tattttagtt gtttgtattt tttttgatta tgatatttat ttagttgttt 1740
agtttaggat agatttttgg gtgtataaat tagtttagaa tagttggtag taatttgtag 1800
attgtttgtt gtgttgtgag ggtatggatg tggtatattt taatggttat atttatattt 1860
tgggggggtg agattttatt attggagtta agttgaagga agtggaatgt tataatgtta 1920
agagaaatta gtgggtattg gtggttttat tgttttattt tttttattt tttgatttaa 1980
tggtaatttg agattatttt tatgttttta atagtaagtg tatgttttgt tatgatttta 2040
gttataatat gtggttgaag tgtgtttttt tgaagtgtaa tgatttttag gaagtttgtg 2100
tttttaatga ggagatttat tgtatttgtg atattttagt tatgaaggtt tataatttag 2160
ttagggtaga atggaggtaa atgaataata tttttttggt tttagagatt aataattata 2220
gaattattaa gtatggttaa aaattgttgt ttattatttt ttgtatttta tagtggaaaa 2280
agaattgggt gattgtgtat gaatatgata ttaggggaga ttaatggatt aatataggta 2340
ttatattagg tttttttgtag tttgatttta attttttttg tttttttgtt tgtgtttatt 2400
ttttttgttt tgaatttggt tagagttttt ttattgaaga agaagaaata ttaagtgagt 2460
ttagtattga atgggattta ggtggattta gtgagttaga ttttgagtta ggaagtttaa 2520
gttttttttt tgatgatgat ttttgggtgt gtgtagtgtt ttagtga 2567

<210> 295
<211> 10664
<212> DNA
<213> Artificial Sequence

<220>
<223> chemically treated genomic DNA (Homo sapiens)

<400> 295

gtgtgggggg tgtggtttga agagattaaa tggaggtgga ggtatgggtt aaattgaatt 60
ttttaggtta tgggaggggt atggttgagg gttttttttg tttttagatg tgatggtgtg 120
gtttaatgag tgggttatgg gttgggtgtt tgggttgggt ttgaaggaat ttgttatgaa 180
ttttatggag agtggggtat atggggtatt gtttgttttg gatgagattt ttgattattt 240
tgatttggtt ttgtttttgt agattttttat gtagaatgta taggtgagtt gttgttgggt 300
ttggagtatg ttgggtgttt ttattttgta gattgtatgt tttaattgtt tttttttattt 360
ttttttttta ggtttggtag tttttggaga aggaatttag taattttatt tttttaggta 420
tagataggtg gttggatgag gtgggtgtgg taatagttta gagggttttg tttttagtgg 480
ttttttgaga ggttgagttg agggggtggg gtttgattat taatggttat ggttggaggt 540
ggggttagga gaggggtggg gttaaaggag aggtgagatt tggagagggg tggagttaaa 600
ggaaggggtg gagttagata aggtaggagt tttttattgg ttgtttggtt tttatattta 660
ggatagtgtt aagtttttta gttgtttttt attttggtgg aagatgtttt gggagaagga 720
tttttgaggt gtaatttttg atttagttga gatgttgttt ttttaatttt gtttggttgt 780
agtgggagtt ttgggttttt tggggttttt tttttgtaag ttgtagttag aaggtggggg 840
gtttttaaat gtgatagttt ttttttgttt ttttagggtg gggtatggat tattttagta 900
gtttgggttt tggaatggtt tagttttttt ttgtttattt ttgaggaatg gattgtttta 960
atgttttgga tttttttgtt aggatgaaat ggataaattt tatttttttt ttttagggtg 1020
ggagtgaatt tgtttaatgt gagtttgagt ttttggttgt ggggaaggga gggaaattta 1080
tgtggagttt ggtgattgtt gtgatattgg gaagggaagt ttaaagggag gaattttgga 1140
gaggaatagg gaggagggtg ttttaggttg aattgttgtt tgtttttttt ttaggttaga 1200
tttttgggag tttttgggta gatggtgttt tggtttggat ttatttttgt tagtgtaggt 1260
ttttaggtga ggattgtgtt gggtgatttt gggggtgtgg ggtggtatgg tggattttta 1320
ttgttttatg tgttgtttgg tgttatgtag gtgattttat ttggatggaa gaattttttt 1380
gaggttgggt tgtttttttt tttgtttgga ttgtggtttt gttggggaga gtgggtgggg 1440
gagtttgtgt tgaggattgg attatttgta tagattagtg ggagtgtgtg tgtttgtttt 1500
gtataggggtt ggggtttgga ttaaattata tgaattggat tgagaggggg aagaagtggg 1560
gaggaagaaa ttttgtttta aatgtttgtt tttttttttt ttattttgta atttattgat 1620
tagtttttgt tgggagatgg gtgtttttta tttgtgggaa gggggtgggg tttttttttt 1680
gggttgtatg tggggagagg ttgttttttt tttttttttt tgtttagttg tggggtttaa 1740
gttttttttt tttgtttgaa aggaggggag gggggatttg ttgttataag ttttgttttt 1800
tgtgttattt agttttgttt tgttgtgttt ggttgttggt tttttgggtt atttgtgggt 1860

```
gggttttttt tttttttttt tgtgttttgt gtttttgggg ttttattgtt ttttgtgttg    1920
tggttgtgtt tgtgttttgg gggtaggggg tgtagaatgg tgtttttttt ttttttttgg    1980
ttttggggtt tgtatgggag aatttttttt ttatgatgtt gttgggtgat gtggtgtggg    2040
ggtaggggga tggtggggga gtttttgttt ttgattttttg gttggttttt ttgttttagg    2100
tgttatttag tgattatggg taaagagaat tgttttaaaa agttttttttg ttgattgatt    2160
ttttttttgt gggggggggg ggggttgttt aattaattat tttgagttag gagtttaggt    2220
tttttatttt ttaagtttag gtttaggttt ttagatttta tttgtatttt ttgatgtttt    2280
gagattttta gattttatgt ttgataatga ggttttgggg agtatgttta tttggagaaa    2340
taaatatagt ttgtggaaag gggttggaag gtagggggag gtatatttgt gttgtagttg    2400
agtgattggg ttagggtttt ggtgttttta gtatgtttgt tagggttttg gagatgagaa    2460
tgttagaagt tagatagttg gtgtttaaag tttattttag gatgtttaga gattattaat    2520
ttgtttattt tatggatggg gaaatggagt tttagagtta tttgataatt tatagtgaga    2580
taagtatttt tttaaatggg gtttttatat gttttttttt tatatattta ttgataaagg    2640
gatgaggaga aatagttaat gtagtttttt gtgggatagt agagaaaaga ggtttgaggg    2700
gtattgtatg agtaaaatta ggtagttatt tagtatagtg tgtgagatta gtgttagttt    2760
tatttttttt tttttaattt tttttttttaa ttgaattttt tttattggat tttttttttg    2820
ttttgggtga ttttgttttt tagtagtttt gtttttttga tttttatttg agttggtttt    2880
gtttattagg ttttaagttt gtattttaat tttttattta tttttgtttt ttggttttag    2940
taattttatt tggtgttttt tgagttaggt tttggtttat gtgtttagag ttttggttat    3000
gttttttttt ttagttttgt tttattagtt tattgggtta ggttttgttt ttattaggat    3060
ttattatgtt gggtttggta ttttttttag gtttttttag ggttttgttt atgtttattt    3120
ggagtgtaga ttgttttgta gattagtggg tagagatagt agaattgata gtgttggagg    3180
tggggatggg gagagagagt gagtgtgggg tgtggttggg ggtggagtta ggatggggtt    3240
gggattagat ttggattaga ggttaggttt aggttaaggt tggagttagg attgtttgtg    3300
gagttaaggt tgtattaggg gtaaggggtt gggttagagg ttaggattta gggttggggg    3360
tttttagagg gttttggtgt attttgaggt ttagtggagt tttgagtgtg aggtgaggtt    3420
ttatttggta ttggttgtag tgtttattta tgtttttttt attatagtga tagtttttat    3480
atttagtgta ttgttgggga ttgggggagt ttggttagat ggaaatttta atgttatttg    3540
ggaggtattt gtttttggtt tatttttgta ttttggatgt ttttttagat tttatttttt    3600
ttatttttaa tattgagttt tgtatttgtt ttttaaatta gttttttttt tttttttttg    3660
tttttggttt tttttttttt tttttttttt tttttttttt ttgaggtgga gttttttttt    3720
gttgtttagg ttggagtgta gtggtgtaat tttagtttat tgtaattttt gtttttttggg    3780
tttaagtgat ttttttgttt tggtttttttg agtagttggg attataggtg tgtgttatta    3840
tgtttggtta attttttttgt atttttttagt agaaatgggg ttttattgtg ttagttagga    3900
tggttttgat tttttgattt tgtgttttgt ttattttggt tttttaaagt gttgagatta    3960
taggtgtgag ttattatatt tggttttgtt tttggttttt tattttaggg tgattttatg    4020
ttttttttgtt agggtgggtt taggtgtttt ttttatttgt tttttgttta tttttttttt    4080
agtttattag tttttgtgtt attttttttt tttttgattt tttttgttat agatttttgt    4140
ttttgttttt atattttaag atgtagtttg ggttttgttt tttttttttt ggattattgt    4200
tttagttttt tagtttttag tttgttttta ttttatatgt ttgtagaagg attttttttt    4260
ttttagaatt gattttggtt tgtttttgtt ttgagttttt ttatagtatt attttagggt    4320
ttttgggata gattttgggg atattttggt gggtggattt gggttgggga tatatattgt    4380
agaaggagta gtagttatat agggattttg gttggtagtt tttatatttt tgagtatttt    4440
gtggatttgt ggggatagga gggtagtagt gatttaggtt gatttggttt tttttttattt    4500
atattagttt aggttattta tttgtgtttt tattgttttt ttttttgttg gaggtatttt    4560
tagttttttt ttttttgttt agtgggttgg ggatgatggt gaagtggggt ttattttttt    4620
ttagtttttt tttttttttt tttttttttt tgttgtggtt tgggtttagt ggggttttaa    4680
ttttagtttt tttttttttt ttttttttgt tttttttttt tttttttttt tttttgtttt    4740
tttttttttt ttttttttgg tttaggttga ggttatgatt tttttttttta ttttttttat    4800
ttttttggtt ttggttgtta tgatgggtgt tttttttttt ttgttttgaa tttttattgt    4860
tttttttatg ggagttgggg tttttttttt tttttttttt ttttttagaa ttattttttt    4920
ttaaatggtt tttatttttg attattgtgt gttttggggg gtgatggttt atttttttga    4980
tggatttttt ttgattatgg ttagtttttt tattttggtg gttttgtttg tggttggggg    5040
tttggtggtt aagtttagta gagatttttt tatttttttt tttggggatt ttgtggtggt    5100
gatggtgagg gatttttgtt ttatatttat tttggaagtt tttttttatta tttttgtggt    5160
tttgaggttg gtggtttgta atatagtggt tgaattggat tgtgattttt tttttttttt    5220
ttttttttatt tataaggtta tggtggatat gttggggatt ttggtgttaa tgtttagtgg    5280
atatattttt attttttttt ttgtttttgt ggttttggtg tttgtggtta gggatatgat    5340
ggtggtgttt atttgagata gttttaattt tttttttttt gtggttttgg tgtttgtggg    5400
tttggtgttt atatttagtg gagatattat tattattatt gttatttttt ttatggtttt    5460
ggtgtttgtg gttggggttg tgatgatggt gtttatttga gatattttta tttttttttaa    5520
```

```
ttttgtggtt ttggtgtttg tgagtttggt gtttatattt aatggagata ttattattat     5580
tattattatt attattatta ttattattat tgttattttt tttatggttt tggtgtttgt     5640
ggttggggtt atgatgatgg tgtttatttg agatattttt atttttttta tttttatggt     5700
tttggtgttt gtgggtttgg tgtttatatt tagtggaggt tttttttttt tttttttttt     5760
tttttttttt tttttttttt ttattatttt ttttattatg gttttggtgt ttatgtttga     5820
ggatatgatg gtgatgttta ttggatataa ttttattttt atttttgttt tgatggttgt     5880
ggtttttgtg gttggggagg tggtgtttgt gtttagttga gtttttttgtg tttttatttt     5940
tgtggttttt gtgtttatgg gtttgtttat tatgttttgt aaagattttt ttatttgaga     6000
tatttttatt tttgattttg tggttttggg atttgtggtt tgggagtagg tgtttatatt     6060
ttttggagat atttttattt ttttttttat ggtttgggtg gttttagaaa tgatgtttat     6120
tttttgtgga aatattttttg tttttatttg gatggttttt agggttgtgg aggtggtggt     6180
gaagttttgt tgatgttttt ttggagagtt tggtgatttt agtgttgttg ttttggtttt     6240
tgaagtttag tttatttttt ttgagttgtt gggttatggt tggggggagg agtaggttgg     6300
ttattttagt ttagaggata gaagtgtgta gttatggttt atggtggttt atggggatgg     6360
ataggtagta ttggttttag ttgtttttgt ggtaatgtgg ataaatgttg gggtttttgt     6420
ttttttgggg ttggtttttt tttttttttg tgtttttttt ttgtttttttt ggtttttgtt     6480
tatttttttt tggtttttgt tgtttggttt tggatatttt tttgaggttg tttttggagt     6540
tttttgattt ttttttgggg tttttttttt ttattttttta gttaataggg tttttttttt     6600
tttttttttt tagttaaatt tatttttagt tttgagttat tttgggttag tttttgtttg     6660
tttgtttttt gttttttttt tttttagttg gggaggggat tagtgagggg tttttttttg     6720
gttaggagat ggtggttaag ggatttgatt ttgaattatt aataagttta tgtttggtag     6780
ttgtaaagat aaaggttaga tttttagtag gtttttgggg gagtttgggg tatttggggg     6840
aggtagaaga gatttattag aggggagaga tttttgggat ggaaggattg ggggtttgat     6900
tgtggggtgt ttttagttgg aatgatatgt gttggtgaga gagtgatgtt agtattgagg     6960
ttttagaatg gggggaaagg aatatggttt ttaatatatg tgtttatgat tttttgtttt     7020
tggaatttag atttgggggt agggattggg ttaggttagg gttataaata tagttgggag     7080
gggtaggggg atttaggtta tggaggttat agttgttttt attatagagg gttggtagga     7140
gataagtggt tttgtttgtt tttgtgtgtt agtatttttt atttttttatt ttttatgatt     7200
gtttttatta gggttttttt ttttgtttat gggttttttt tttttttttaa ttttgttatt     7260
tgttttttta gggtttttgt tttttttttta tgggattgtt tttttttttt attttgggtt     7320
tttgttttat ttttatttta gtgttagttt ttttttaagt ttgtgttttt tttttttttt     7380
taaatttttg gttttttttt tttgagtttt tgtttttttt ttaatttttt ggtttttgta     7440
tttttttttg ttttttgttt tagttaaggt gttttttttt tatttttggt atttattttt     7500
ttgggttttt gtttttattt ttttttagag tttttgtttt tttttgtttt agagttttttg     7560
tttatttttt tttgggtttt tgtttttttt tttttgggtt tttgtttttt tttttttggg     7620
tttttgtttt tttttttttg tggattttttg tttttttttt tttgggtttt tgtttttttt     7680
tttttgtggg tttttgtttt tttttttttg tggattttttg tttttttttt tttgggtttt     7740
tgtttttttt tttttgtggg tttttgtttt tttttttttt tgggttttttg tttttttttt     7800
tttgggtttt tgtttttttt ttttagggtt tttgtttttt tttgttttag agtttttgtt     7860
tttttttttt tgggttttttg tttttttttt ttgggttttt gtattttttt ttgtgggttt     7920
ttgttttttt tttttgggtt tttgtttttt tatttttggg tttttgtttt tttttttttg     7980
ggtttttgtt tttttttttt tgggttttttg tttttttttt tttttgggtt tttgtttttt     8040
tttttttggg tttttgtttt tttttttttg ggtttttgtt tttgtttttt tgggttttttg     8100
tttttttttt tttgggtttt tgtttttttt tttttttggg tttttgtttt tttttttttg     8160
ggtttttgtt tttttttttt tgggttttttg tttttgtttt tttgggtttt tgtttttttt     8220
tttttgggtt tttgtttttt tttttttggg tttttgtttt ttttttttgg gtttttgttt     8280
ttttttttgt gggtttttgt tttttttttt tgggttttttg tttttttttt tttgggtttt     8340
tgtttttttt tttttgggtt tttgtttttt tttttttggg tttttgtttt tttttttttg     8400
ggtttttgtt tttttttttt tgtgggtttt tgttttattt tttttgggtt tttgttttat     8460
tttttttggg tttttgtttt ttttttttgg gtttttgttt tttttttttt gtgggttttt     8520
gttttttttt ttttttgttt atttttgggt ttttgttgtt ttattttttg atttttttgtt     8580
ttttaagttt ttgttttttt tttttttgggt taaattgttt tttttttgtt tggtattttt     8640
ttttttgagt ttgttttttt tttgttattg gtttttaatt tttttttgttt ttattttgtg     8700
tttgtttttg gagttttttt tgtgtgtttt tttttttttg gtttggattt ttgtattttt     8760
taggttgttg tttttttgtt tttttattgt ggtttgggat ttgttttttt tttgtttttt     8820
gttttggtgt ttttaagatt ttttgttttt tagattttgt tttgttttag gttaggttgg     8880
aaagtggagg atttggtttg ttttgggtgg gtttggaagt agagttggtg gagggagtgt     8940
tggggttttg ggttgtagga ggttgtggtg gttgtggtag tatggtggtg ttggagaagg     9000
agtaggtgag tgttggatta gggtttgtgg gagtgtggag ttggggattt tgtttttagg     9060
tttttagaga ggagggtgtt ggatatttag atttttgggt tagatggagg agggggatgg     9120
gagggtttag gttttagggt ttagggtatg gggttgaga ttttttgagtt tggagtggga     9180
```

```
gggtgttggg ggtttggatt tttgtgtttg tggggagtgt atggggtggg tggttttgtg    9240
ttttatagga tagaattggg tgttttttta ttttattttt atttttatat ttgtttttgt    9300
ttttagagtt ggatttttaa gatttttaag aagaagattt gtatgatgtt tatagttgat    9360
tttatagatt tggggtgagg agtttgtttt tggattgatt ttagagggtt tgtggtttgt    9420
tgattttgtt tgtggatggt tatgttttag ttttgttttt tttttatttt tggatttggg    9480
gatttttta agggtgtttt ttgttgatgt ttttttttta ggagtgtttg ggtttgtgta    9540
gatattttt atttttatt tgttattttt ttgtttaggg ttagtgtaga tgttttaggg    9600
tttggttttg ttttttttt ttttgatgtt ttttttatgt gtttttgttt ttttgtgtta    9660
tttttgtgt tttgtttttt ttttttggg tatttttgtt ttatttattt atttatttat    9720
ttatttattt atttatttat ttatgtaaga aatttttatt tagtgttttt aaggtttttt    9780
tagtttaatt taatggtttt gttagggttt taattttttt tgggatatgt gttgtgtggt    9840
tttgggtaaa ttattttatt tttttgggtt ttgtttttta tttgggtatt aaaagaggtt    9900
tgattttttg ggggttatat gagaattaaa taagttaatg tttgtgatat ttttggtata    9960
gtgtttagta taggatgttg ggaaatattt gtagttttta ttgggaggtg gtaattatta   10020
ttgtgggtgg gttttgtttt ggtggttggg gatattaatt gagttagata gggtgtgtgt   10080
gtttgtgagg agtttttgtt ttttagtgat ggttttttaa gtggatttag gttttttagt   10140
ttttttttt ttttttttt ttttgagatg gtgttttgtt ttgtttttta ggttggagtg   10200
tagtggtgtg attttggttt attgtaagtt ttgttttttg ggtttatgtt gtttttttgt   10260
tttagttttt tgagtagttg ggattatagg tgtttgttat tatgtttggt taattttttg   10320
tattttagta gagatggggt tttattgtgt tggttaggat gattttgatt ttttgatttt   10380
gtgatttgtt tgttttagtt ttttaaagta ttgggattat aggtgtgagt tattgtgttg   10440
ggttaatttt ttgtattttt tttttagtag agatggggtt gtattgtgtt agttagatgg   10500
ttttgatttt ttgattttat gatttgttgg ttttggtttt ttaaagtgtt aggattatag   10560
gtgtgagtta ttgtatttgg tttaggtttt ttagtttttt gtttttttgtt tttttgagat   10620
agagtttggt tttgttgttt aggttggagt gtagtgttgt gata                    10664
```

<210> 296
<211> 10664
<212> DNA
<213> Artificial Sequence

<220>
<223> chemically treated genomic DNA (Homo sapiens)

<400> 296

```
tattgtaata ttgtatttta gtttgggtga tagagttaga ttttgtttta aaaaaataaa      60
aaataaaaaa ttagaaagtt tgggttaggt gtggtggttt atatttgtaa ttttagtgtt     120
ttgggaggtt gaggttggtg gattatgagg ttaggagatt gagattattt ggttaatatg     180
gtgtaatttt gtttttatta aaaaaaaaat ataaaaaatt ggtttagtgt gatggtttat     240
gtttgtaatt ttagtatttt gaggggttga ggtgggtgga ttataaggtt aggagattga     300
gattattttg gttaatatgg tgaaattttg tttttattaa aatataaaaa attagttggg     360
tgtggtggtg ggtgtttgta gttttagtta tttgggaggt tgaggtagga aaatggtgtg     420
aatttgggag gtggagtttg tagtgagttg agattgtgtt attgtatttt agtttggggg     480
atagagtgag atattgtttt aaaaaaaaaa aaaaaaaaaa aaaaattaga aagtttggat     540
ttgtttgaga ggttgttatt gaagggtggg agtttttgt ggatgtatat attttattta      600
gtttaattgg tgtttttggt tattaagata gggtttgttt gtggtaataa ttgttatttt     660
ttagtgggag ttgtagatat tttttagtgt tttgtgttgg gtgttgtgtt aggaatatta     720
taagtattaa tttgtttaat ttttataata attttaggag attaagtttt ttttgatgtt     780
tagatgagaa atgggattta gagaggtgaa atgatttgtt tgaagttata tagtatgtgt     840
tttaggggaa attggaattt tggtagggtt gttgaattag gttaaaggga ttttgggagt     900
gttgagtgag ggttttttgt atggatggat gaatgaatga atgaatgaat gaatgaatga     960
atgaaataga aatatttaaa agggaaagat agagatatag aaagtgatat gaagagatag    1020
aagtatatag aagagatatt ggggaggaaa gaagtagaat tgagttttgg agtgtttata    1080
ttgattttga gtgggagaat ggtgaatggg gaatgagggg tgtttatata gatttaaatg    1140
tttttagagg gagagtgttg gtaaggaatg tttttgggaa ggtttttgag tttaggaatg    1200
gggaaggagt agggttgggg tgtgattatt tgtgggtggg gttagtgggt tgtggatttt    1260
ttggggttag tttaggggtg aattttttat tttggatttg tggagttaat tatgaatgtt    1320
gtgtaggttt tttttttgaa gattttgggg atttagtttt ggggatagga ataaatgtag    1380
gggtggaagt ggggtgagag ggtatttagt tttgttttat gggatataga gttatttatt    1440
ttgtgtgttt tttatggata taggagtttg ggtttttagt gtttttttgt tttagattta    1500
```

```
ggagttttga tttttatgtt ttggattttg gaatttggat ttttttattt tttttttttg    1560
tttgatttag gaatttgggt gtttagtgtt tttttttttg ggagtttgga ggtgaggttt    1620
ttagttttgt gtttttgtag attttggttt ggtgtttatt tgtttttttt ttggtattat    1680
tatgttgttg tggttgttgt aattttttgt agtttagggt tttggtgttt tttttgttgg    1740
ttttgttttt agatttgttt agagtaaatt ggatttttta ttttttagtt tagtttgggg    1800
tggggtgagg tttggggggt ggggagtttt ggagatgtta aagtgggagg tggggagagg    1860
gtgggtttta ggttgtgata aggggataga gggatagtga tttggggggt gtagaggttt    1920
gggttggggg gagagagata tataggggag attttaaggg taagtgtgag atgggaatag    1980
aaggagttgg gggttagtgg tggagaggga atagatttag aaaggaggat gttagatagg    2040
gaggggataa tttaatttaa agaggggggag ataaagattt aggggataga gaattagaag    2100
gtggggtagt agggatttag ggatagatag agagagaggg ggatagagat ttatagagag    2160
agggggatag agatttaggg agaggggata gagatttaga gagagtggga tagagattta    2220
gagagagtgg gatagagatt tatagagaga gggggataga gatttaggga gaggaggata    2280
gagatttaga gaggggggga tagagattta gagagggggg gatagagatt tagggagggg    2340
gggatagaga tttagggaga ggggatagag atttatggag agggggatag agatttaggg    2400
agaggggata gagatttaga gagaggggga tagagattta gagagagggg gatagagatt    2460
tagagagatg gggatagaga tttagagaga gggggataga gatttagaga gaggggaata    2520
gagatttaga gagagaggag gatagagatt tagagagagg gggatagaga tttagagaga    2580
tggggataga gatttagaga gagggggata gagatttaga gagaggggaa tagagattta    2640
gagagagagg aggatagaga tttagagaga gggggataga gatttagaga gggggggata    2700
gagatttagg gatgggggga tagagattta gggagagggg atagagattt atggagaggg    2760
gtatagagat ttagggagag gggatagaga tttagagaga gggggataga gattttgaga    2820

tagaggggga tagagatttt gagagagggg gatagagatt tagagagagg ggaatagaga    2880
tttagagaga gagggggata gagatttata gagagagggg aatagagatt tagagagagg    2940
gggatagaga tttatagaga gagggggata gagatttata gagagagggg aatagagatt    3000
tagagagagg gggatagaga tttatagaga gagggggata gagatttagg gagaggggga    3060
tagagattta gagagagggg gatagagatt tagggagagg tggatagaga ttttgagata    3120
gagggggata gagattttga gagagagtgg ggatagagat ttagagaggt ggatgttaga    3180
gatggggagg agatattttg attgaggtaa ggggtagagg gggatgtaaa aattaaagaa    3240
ttgggggaag ggtaggaatt tagaaaggag gggttagaga tttaggggag agagagggat    3300
atagatttgg gggaggattg atattaagat aagagtggga tagaagttta gagtgagagg    3360
gagaggtaat tttatggagg agggataggg attttgagag agtagatgat agaattgaag    3420
agagaggagg atttgtgaat aggaaaggag attttagtgg gggtagttat gaagggtgag    3480
gagtaggaaa tattgatata tagagatggg taaggttatt tgttttttgt tagtttttttg    3540
tgatggggat agttgtggtt tttgtaattt gagttttttt atttttttta gttgtattta    3600
tagttttggt ttagtttagt ttttgttttt agatttgagt tttagggata ggaggttatg    3660
ggtatgtgtg ttgggggtta tgtttttttt tttttatttt agggttttaa tattgatatt    3720
atttttttat tagtatgtat tattttagtt ggaaatattt tgtaattgaa tttttagttt    3780
ttttgtttta ggagtttttt ttttttgata agtttttttt gttttttttta ggtgttttag    3840
gtttttttaa aaatttgtta aaagtttagt ttttgttttt gtagttgtta aatgtgagtt    3900
tgttggtagt ttaaagttaa gtttttttggt tattgttttt tggttaggga gagatttttt    3960
attggttttt tttttagttg ggaggaggag gagtaggagg taggtaggtg gggattgatt    4020
tagaataatt tagggttgag agtaaattta gttggagaga ggggaggggga aggattttat    4080
tggttgggga atggggaggg agggttttag agggggggtta gggggttttg gagatagttt    4140
tagaggggtg tttagagtta ggtagtaagg attagaggaa ggtggataga gattgaaagg    4200
ataaaggaga gatatagaga aaaaaagaga ttaattttaa agggataaag attttaatgt    4260
ttgtttatat tgttgtagag gtagttggag ttagtgttgt ttgtttgttt ttatgggtta    4320
ttataggtta tggttgtatg tttttgtttt ttgggttggg gtggttagtt tgtttttttt    4380
tttggttatg atttagtagt ttagagggga tgggttaggt tttagaaatt ggaataatag    4440
tattggagtt gttgggtttt ttgaggaggt attagtagag ttttgttatt atttttatag    4500
ttttagagat tatttagatg agaataagga tgtttttata gagaatgggt attatttttg    4560
gagttattta gattgtgaaa aggaggatga agatgttttt aaggaatatg ggtatttatt    4620
tttaggttat aggttttaag attataaagt tggagatgag ggtgttttag gtgaggaggt    4680
ttttgtagag tatggtgggt aggtttgtgg gtatagaggt tatgggagtg aagatatgga    4740
agatttagtt gagtataggt attatttttt tagttatagg agttatagtt attaagatga    4800
ggatgaggat gaagttgtgt ttagtgagta ttattattat atttttaggt atggatattg    4860
aggttatgat ggggaagatg atgaaggaga agaggaggag gaggaggagg aggaggaaga    4920
ggaggttttt attgagtatg gatattaggt ttataggtat tgaggttatg ggagtgaaga    4980
ggatgaggat gttttagatg gatattatta ttatggtttt agttataggt attaaggtta    5040
tgaagaagat gatgatgatg atgatgatga tgatgatgat gatgatgatg atgatgtttt    5100
```

```
tattgaatat agatattagg tttataggta ttaaggttat gggattgaag aggatgaaga 5160
tgttttagat ggatattatt attgtgattt tagttatagg tattgaagtt atgaagaaga 5220
tgataatgat gatgatgatg tttttattga gtatggatat taggtttata ggtattaaga 5280
ttatagaaag gaagaggttg aggttgtttt aggtgaatat tattattatg tttttgatta 5340
taggtattaa ggttatagag atgaggaaga agatgaggat gtgtttattg aatgttggta 5400
ttagggtttt taatatgttt attatggttt tgtagatgag gaagaggaag aagaggagat 5460
tatagtttag tttggttatt atgttgtaag ttattaattt tgaggttata agagtgatga 5520
agaggatttt taagatgagt ataaaataga agttttttat tattattatt atagagtttt 5580
tagggaggaa gatgaggagg tttttgttga gtttggttat taggttttta gttataggta 5640
aagttattaa gatgaagaaa ttggttatgg ttaaagaggg tttattaaag agatgagtta 5700
ttatttttta ggatatatag tggttaagga tagaagttat ttgaggaagg atgattttga 5760
ggaggaaaag gagaaggaag aggattttgg tttttatgag gaagatgatg aaagtttaga 5820
gtagggagag aaaggtattt attatggtag ttgggattag gaagatgagg aggatgagga 5880
ggaaggttat ggttttagtt tgaattagga ggaggaagaa gaggaagata aggaggagga 5940
ggaggaggaa gaagatgagg agaggaggga agagagggtt gaggttgggg ttttattgag 6000
tttagattat agtgaggagg aagaggagga ggaggagggg ttggaggaag atgagttttg 6060
ttttattatt atttttaatt tattggatag gagagaggag gttggaggtg tttttagtga 6120
ggaggaaagt ggtgaggata taggtaagtg gtttgggttg gtgtgggtgg gaaggaattg 6180
agttagtttg ggttattgtt gtttttttgt ttttgtaggt ttataggatg tttaggagta 6240
tgggaattat tagttagggt ttttgtgtgg ttattgtttt ttttgtaatg tatgttttta 6300
gtttagattt atttattaag gtgtttttag aatttgtttt aggggttttg gggtggtgtt 6360
gtgggagggt ttagagtaag ggtaggttag ggttagtttt gaaagaaaag agattttttt 6420
gtaggtatgt gaggtgagga tagattgaag gttgggaggt tggggtagtg gtttaggaag 6480
gagaggataa gatttaagtt gtattttggg gtgtggggat agagataggg gtttgtggta 6540
aagagagttg ggggaaggag ggatggtata ggggttgata ggttaggggg agaataggta 6600
gagagtgggt ggagagagta tttgagttta ttttgatagg gagatatggg gttgttttga 6660
gatagaaaat taggggtagg gttgggtgtg gtggtttatg tttgtaattt tagtatttta 6720
agaggttagg gtgggtggaa tatgaggtta ggagattgag attattttgg ttaatatggt 6780
gaaattttat ttttattaaa aaatataaaa aaattagttg ggtgtggtgg tatgtatttg 6840
taattttagt tatttaggag gttgaggtag gagaattgtt tgaatttggg agatggaggt 6900
tgtagtgagt tgaggttgtg ttattgtatt ttagtttggg tgatagagag agattttgtt 6960
ttaagaaaaa aaaggaaaaa aagggaaagg aaggaaatta ggggtagggg aggggagggg 7020
aggttggttt ggggggtaga tatagagttt agtgttagag ataaagaggg tagagtttgg 7080
ggaagtgttt agggtgtagg ggtgggttag ggtgggtat tttttaagtg gtgttagagt 7140
ttttatttaa ttaggttttt ttggttttta gtgatgtatt gaatgtgaga gttgttattg 7200
tgatgaggag aatatgggtg agtattgtga ttagtgttag gtgagatttt attttatatt 7260
taaggtttta ttgggtttta ggatgtattg ggattttttg aggattttta gttttaaatt 7320
ttggtttttg atttaatttt ttatttttga tgtaattttg attttataag tgattttgat 7380
tttaatttta atttagattt agtttttggt ttgggtttgg ttttggtttt attttaattt 7440
tgtttttggt tatgttttat gtttattttt ttttttgtt tttgttttta gtattgttag 7500
ttttgttatt tttgtttgtt ggtttgtgaa atggtttgtg ttttaggtga gtatgggtgg 7560
ggttttggaa aaatttgagg aaggtgttgg gtttagtgtg gtgaattttg gtgggggtgg 7620
agtttggttt ggtaggttgg tggggtgggg ttaagagggg aggtgtggtt aaggttttga 7680
gtatgtgggt tggggtttgg tttgaggaat attaggtgga gttgttggag ttaagggata 7740
gaaatgagtg gagggttgag atgtgggttt agagtttaat gggtgaggtt aatttaagta 7800
ggaattgaag gggtggggtt attggagaat ggaattattt agaatgaaaa aggggtttgg 7860
tagaagggat ttagttggaa gaaggaatta gggaaaagga ggtggagtta gtattaattt 7920
tgtatgttat attgagtggt tgtttaattt tgtttgtgta gtatttttta agtttttttt 7980
ttttgttgtt ttataggaag ttatgttgat tatttttttt tgttttttta ttagtaagtg 8040
tgtaaaaggg gagtgtgtaa aagttttgtt tagagaagtg tttgttttgt tatgagttat 8100
tagatgattt tgggatttta tttttttgtt tatgaaatgg gtaggttaat gatttttaag 8160
tgttttgggg tgggttttga atattagttg tttaattttt ggtgtttttg tttttagggt 8220
tttggtagat atgttggaaa tgttggaatt ttgatttagt tgtttgattg tgatgtaggt 8280
gtattttttt ttgtttttta attttttttt atgagttata tttatttttt tgaataaatg 8340
tgtttttttga aattttattg ttagatgtgg ggtttgggaa ttttagggta ttggggaatg 8400
tgggtagggt ttagagattt ggatttagat ttgagggatg aggggtttgg atttttggtt 8460
tgaggtggtt ggttgaatag tttttttttt ttttgtaaaa aaaaaaattag ttaataagga 8520
agttttttga aatagttttt tttatttgtg attattgagt gatgtttggg gtggggaggt 8580
tgattgagag ttgggggtga gggttttttt attgtttttt tgtttttatg ttatgttgtt 8640
tagtggtatt gtggaaagag gatttttttta tgtaaatttt ggagttagag gagaagggga 8700
agtgttattt tgtgtttttt atttttgggg tatggatatg gttatagtat ggggggtggt 8760
```

gaggttttgg ggatatgaga tatgggggag ggagagggga atttgtttgt agatgatttg 8820
ggggattggt gattggatgt ggtggggtgg agttgagtgg tatagggggt gaggtttgta 8880
gtagtgagtt tttttttttt tttttttgga tgaagaagga agatttgggt tttgtgattg 8940
ggtaggaaaa aggggaggga gtagtttttt tttgtatgtg gtttgggagg gaagttttgt 9000
tttttttttg tgggtaaagg atatttgttt tttaatagaa attgattaat aaattataaa 9060
gtagagaaaa ggaaagtgga tgtttggggt gggatttttt tttttttgtt tttttttttt 9120
tttagtttag tttatgtggt ttggtttagg ttttggtttt gtgtgaggtg ggtgtgtgta 9180
tttttgttgg tttgtataga tggtttagtt tttggtgtga gttttttttgt ttgttttttt 9240
tggtgaggtt atagtttggg taggagaggg aatagtttag ttttgggaag attttttttgt 9300
ttgagtgagg ttatttgtat gatgttgggt agtgtgtgga gtagtgaaga tttattgtgt 9360
tgttttgtat ttttaaggtt gtttagtatg gtttttattt ggaggtttgt attagtagga 9420
ataggtttgg attgaaatgt tgtttgtttg ggaattttta gaagtttggt ttggagggag 9480
agtgagtagt ggtttagttt ggagtatttt tttttttgtt ttttttttagg attttttttt 9540
ttggattttt tttttttgatg ttataatgat tgttgggttt tatatgggtt tttttttttt 9600
ttttgtagtt aaggatttaa atttgtattg ggtgagttta tttttgtttt gggaaggggg 9660
agtgggattt atttatttta ttttgatggg ggagtttgga atgttggagt agtttatttt 9720
ttaggggtgg gtgagaaagg attaggttat tttagaattt ggattattga ggtggtttat 9780
gttttatttt agggaagtga ggaagggttg ttgtatttgg gagtttttta tttttttggtt 9840
gtagtttgtg gagagggagt tttggagagt ttagggtttt tgttgtagtt gaatgaaagt 9900
tggggggtaa tattttagtt gagttgggag ttatgttttg gaggtttttt ttttggaata 9960
ttttttgtta ggatggggag tggttgaaag atttggtgtt gttttaggta taggagttgg 10020
atagttggtg agggattttt gttttgtttg attttgtttt tttttttgat tttatttttt 10080
tttgggtttt attttttttt taattttgtt ttttttttgg ttttgttttt aattgtgatt 10140
attaatagtt aggttttgtt tttttagttt agttttttga ggagttgttg ggagtagagt 10200
tttttgagtt gttgttgtgt ttattttgtt tagttgtttg tttgtgttta aggagataag 10260
gttgttgaat tttttttttta gaagttgttg ggtttggaaa gaggaggtgg agggggtggt 10320
tggagtgtgt agtttgtgag gtggggatgt ttagtatgtt ttgggtttag tggtagttta 10380
tttgtgtatt ttgtgtgggg atttgtagga gtaaggttag gttggagtag ttgaaggttt 10440
tgtttagggt gagtagtgtt ttgtgtattt tgttttttgt gaggtttgtg gtaaattttt 10500
ttaggtttag tttggatatt taatttatga tttgtttatt ggattatatt attatgtttg 10560
gggatggaag ggatttttag ttgtgttttt tttgtggttt ggagagttta gtttagttta 10620
tatttttatt tttatttggt ttttttgggt tatgtttttt atgt 10664

<210> 297
<211> 22069
<212> DNA
<213> Artificial Sequence

<220>
<223> chemically treated genomic DNA (Homo sapiens)

<400> 297

tattggtgtt gtgagagaaa ttttgagtgt tggttggtga gttaggttta gatggggtta 60
gatggggtgg ttgtattagg ttaggttttt tttaattttg aatttgatgt tgggtagaga 120
atagttttat tattttgtag tttagggtgt agatttttgg gtgggtgttt tttgagagtt 180
ttttttttaaa ggtttttatg aagtttttaa ggtgtttttt ttggttattt tgtatgtgtt 240
gttgggggtg gagaaggtag gtgagggtag ttttggggtg tagtgggggt gggggtggta 300
gtagtagtat atttttgagt tttttgggat tattttattt atttattatg tttatttatt 360
ggttgggatt ttggtgagta ggtggggaag gggtttgtt aagaagatat agggaggatg 420
gttttttagg atgggaagtg gttagggata gggtttgggg tttatgggtg ttatgttttt 480
tgggtaatat aagaaaggtt gtttgggggt ttagattttg agtttaggat tgtgttgtgt 540
tttgagattt tatattaatg atggggaggt agaagtttgt tgagaatagg tagaggggat 600
tgggatgtta gggtttattt ttgtgatttg gtagttgata gagttggtgg tagttgagta 660
gtatatgtat tgggtttttt agtttttggt tattgtaggg gatatagata tatagttagg 720
gtttggttag gtttatttta tagtttttgg ggtattgatt atttgtattt attgagtttt 780
attagggagt gaggattgag gataggaggt tttagttttt tggtggagtt ggaagtagat 840
taggttttgg tagttgtgtg gtagttagga tgtttaggtt tggatttagg gagattagtg 900
ttttttgtag aggtttttttg gtagtgttgt tttgggtagt attttttagg tatttttgag 960
gtgatggttt taggggttgt tatagggttg tgtaaggttg gttatgtgtt ttgagtgtgg 1020
ttgtgtggtt gtttggggga ttgaatagaa ttattaggtt aagggtggga taagtttttt 1080

tgtttttggg gttagtaatt tttatatttt tatggattaa gggtatagga aggtagggat 1140
atagggtttt ttaggtggtg gtatttgaat tttgttatgg gaaagttttt gttttatttt 1200
tgtataaaga ataggtggag agaaaatgag gttaagggtt agttttggtt ttgttaaaga 1260
tggtgtagtt tagggatttt gagtgggatg gagggtttgg gtaaagtttt attttagttt 1320
tgggggtttt aagggagggt ttaggttttg ggggttatgg gtgggaaggg gaaggatttg 1380
ggtaaggttt tattttggtt ttggtgtagt tgtttttagt ggtaataata ttttttgttt 1440
tggatgtagt ggtttgaaga ggatatgagg tatttggtgt tatagtggta gtaggttttg 1500
taggaagttg tgggtgggga tttaggtgga agttgaggtt tatgttgggg tttgtatgtt 1560
tggttttgtt tatattttgt atttgtttat ttaggttgtt gtaggttagt gatttatagt 1620
ttttgggttt ttttttttta gttgtgaaga tgattgtgtt tttgggttgt tttgggtgtg 1680
ggaaggggta gttgtttttt ttgagttggt tttgggtgag taggtatttt ttgaggtggt 1740
tgtatagggt agtttttgtg gataggtata gtggttagtt tttgtttggg atggtttatg 1800
gggtggtaga tattattggg aaaggtggtt gggttggtag gggtttatga agtattgtgt 1860
ggtatagtag gggttgggtt ggtagaggtt tatgaagtat tgtgtggtag agtaggggta 1920
gggttttatt ttttaggttt tttatttggg ggttgtttgg atggttgagt gagaagttgg 1980
ttttggtggt taatttgttt tttaatatta ttttgtggga tataattttg tggttattgg 2040
tttttggaag gaagggaggg aggggaggag ggtgtgaagt agttggttta gatttttgtt 2100
ttgtttgttt ttttatttat tttgtttttt ttagattttt gttttaattt tttttttttg 2160
ggatagtatt ttattgattt tgggttttag tttgatttga ataggagagg tagatatgtg 2220
gtgagttagg gatgtagggt agttgttagt tttgtaggtt ttagagttgt atggaggttt 2280
tatatttaaa gtttttgttt ttttttttta agtgaagggg gaagggtggg gagaatagta 2340
tggggggattt aatggttttt gagggttttg agtttttatg ggttatttag tttgtgatgt 2400
taatttaggg tttttttgag tgttaatgta ggtggggatg ggggtagggg ttggtgatag 2460
tttaatgtag tttggagagg ttgtaatgaa atgtttaggt ttttgttttt ttttttttta 2520
gagtttaatg tatggagttt gtttagtttt gggtttttttg ttttgaagaa gttgttgtag 2580
ttttattagt aggtggtttt ttggtttggt ttggagattt tatttagtgg aggaaggtgg 2640
ttgatagtga ggatattagg gttaggtttg ttttatatag aagtttggga attaggtagt 2700
aggtagtttt tttatagtat tgtgtttttt ttttgtttga gattgtggtt gagtttaggg 2760
ttgtgaaggt tttagtgagg gttttagttt agttgttatt ttaagagtaa ttttgagttt 2820
tttttgttta tttgttagga tttattgttg aggttgggta tagtgttggg ggttaggttt 2880
ttgagttttt tgagggtgtt tatggttata tttaggtaga tgtttttgtt ggggttgtgg 2940
ttataggtta tttttttttt gtttagtgtt tgggggatag gtggtgttta gttgaggtta 3000
tgtttgtttg gtgttgggga gttttagtgg tggggttgtt atggggttgg ggatttgtat 3060
tttttttagg tagttttggg ttatagttgt ggttgagagg ttgggggatg tttggggatt 3120
attttggatt tttagttttt atttgattgg ttagatagga attttaggat tttatttttt 3180
ttatggtttt ttggttggag gtatagaatt tgagatattt tttgatgaat aggttgagat 3240
agtgttggtg gatgatggtg gggattttgt ttttaaattt tttggggata atgggttttt 3300
ttaaatttta gagggaaggt aaaagttgtt atgggtgagg gtttttataa ggtttttagt 3360
ggggaatgta gatgtgatga ggttgtgttt atggttggag gtaagagtga ggtgtgttgg 3420
gatagattag tttggttgta gtagggatag atatgttaag gtaagatagg agtagtaggg 3480
gaaattgagg gtagtggaat taaggttatg ggaaattgag gttatggggg tataagggaa 3540
tttgttgtat tttttgttta attttttttag aaatttaaag ttgtttaaaa aatataaagt 3600
ttattaatta aaaataaaat aaggttaagt gtggtggttt atgtttataa ttttagtatt 3660
ttgggaggtt gaggtaggag gattatttga ggataagagt ttaagattag tttggataat 3720
gtggtaaaat tttgttttta taaaataaaa aataaaatta ttagttgggg tgtgatgata 3780
ttggtttgtg gttttagtta tttaggaggt tgaggtggaa ggattatttg agtttaggag 3840
gttgaggttg tagtgagtta tgattgtgtt tttgtatttt agtttgggta atagagattt 3900
tgtttttaaa taaaaaaaaa aaatattaat taattaaaaa aataaaataa gataaaataa 3960
aaaaatattg ttagggtggt tgggtatggt ggtttatgtt tgtaatttta gtattttggg 4020
aggttgaggt gggtggatta tgaggttaga agattgagat tattttggtt aatatggtga 4080
aattttgttt ttattaaaaa tataaaaata gttaggtgtg gtggtgggtg tttgtagttt 4140
tagttattta ggaggttgag gttagagaat ggtgtgaatt taggaagtag agtttgtagt 4200
gagttaagat tgtgttattg tattttagtt tgggtgatag agtgagattt tattttaaaa 4260
aaaaaaaaaa aatattaagg tatggggagg gaaggtgaag gttgtattgt ggtgtggagt 4320
atgaaggttt gtagggtagg ggtgaggttg gttttttgta ggttttatgt ggttgggttt 4380
tagggtgtgg ttttgggggt ggtggttttg gttgagatgg gaggggttat ttgtaaggat 4440
ggattgtggg tgatttgttt agggatgatg gtggtggtag ttttggatgt tatttttgat 4500
aatgtgtgtg tttttagttg ttggttattt ggtttagggt tgttggagga ttggttgttg 4560
ttggttgtaa gggttttttt ggtatttgtg ggggttggtg gggtataggg ggtgtgggta 4620
tagggtgagt ggttgtttat tattatgtgg tggagtatta ggtagttgtg gttgtttagt 4680
gttagtagta tgtgtatttt tgtgtgtagg gggttgggtt agggtgaggg gttggggttg 4740

```
tgtaatggta ttaggtttta gttgttttta ggtaagttta ttgaggtaga ggatataggt    4800
taggttgtgg aggttgttga ggatatgtta ttggtggggt ggtttaggtt tttgttatgt    4860
gttttgattt taagtttaag gtgttgatgt ttgagggaaa ggttttggtt tttggataga    4920
ggattgaagg gttttgggtg tttttatttg tttattggtt ttgttttgtt ttgtggtttt    4980
tgtgttttgt tttgtttttt gatgaagtta taaattagag ggaggaggtt aggtttgtag    5040
gggttgtttt ggagggttgg ttatgtgggt agttgtaatt tgggtatgta tgtttgtgtg    5100
gtagggggt ttttggattg ggggtttggt attgatttag gaaggggagt tgtgagtagg    5160
gatatttggt tttagtttta gagtaatatt ttttgaaatg ttatttggtt ttggaaggtg    5220
taagggaggt aggatgagtt tgtttatgtt attgtgggtt gtttagtaag gaagtaggtt    5280
gtttgttagg ttggtatgtg ttttttgtag tggagggttt gttttttagg aatggataga    5340
gaatttttag atttttttgg ttgtatgttg gggtgagtt taggtagtta taggagtttt    5400
ttaagttaga tgagtttgtt ttgtggtatt gttagtattt gggatgttag atttttttta    5460
ggtggtgggt tttaagggta ttgtgatagt ttagtattta ttatagatta gtaataggat    5520
aatttgagtg tggagatata gatgggaagt gtgtggggtt ttgggatagg tttaatttaa    5580
tgattttttt tttttggggt taaggtttta gttgtgaggg ggttttgggg aggggaggtt    5640
agagtagttt ggagagtttt ttttaaggag ggttgtggga tttatgggat ttgtagggga    5700
attgttgggg ttggttttaa ggttttttag ttagtgttag ggaggtaggg gttttaaatt    5760
agtaggttgt ttagggtggt ttttggatag tagttatgtt tttttaggga gtttgttaga    5820
tatatagatt ttttttagtt tttagattag aatttgtatt ttttaggagt tttttggggg    5880
atttttattt gtgatttgtt tttagggata tttttttgtt ttatagatat tattgatgtg    5940
aagatgtagg agataggata attttttgtg aagggttttg tttatttatt attgaggttt    6000
ggtttgattt tttataagat tttgttgggg gggaagtgtt ttttggagta aaggaaatat    6060
agttttattt ttgggaagat agtatttatt tttattagag gttatgtttt ttatttatat    6120
gttaggagaa agttatattt gtagaagttt gtttttttatt taatgttagg tattttgata    6180
tggtttttta ttttttggaa ttataggttt aggttttggt ttagtttagt ttttagtagg    6240
ttgttggatt tttagtaatt ttggagttgg gttagggaag gaaggtgttg ggttaggttt    6300
tttgtatttt ttaatagttt ttattttata gataggtagg ttgagggttt tagggagggg    6360
gtttaaagtt attttatgag taagtattat gtggtttagg tgagtttaga gattttaggg    6420
tgttattttt tttgtttata tgtttatggt tttttggtat aggtttttttg ggttaggatt    6480
tatttgtagt taggtggggg ttggggatgt gggtgatttt tttttttttg ttaggggtgg    6540
aaatgtggat ggagggtgat ttttttgtta gtttggtggg ggtttgtagt aagtttgttg    6600
atgtttttttg tgtttgttgg gtttgtaggt agtatatttt ttgtgttgtt ggggggttggg    6660
ttgggggtat tgtgggattt ttttttgggg gttttatttg tgttattata aatgttaagg    6720
tttggtagta tagtgggggt atttggagta ggtgattttg ggtaggtttg ggagagggtg    6780
gttggagttt gggggttgga ggtattattt ttttggttgt tttttgagtt gttgggtata    6840
ttggggggaa gtagtagggg gtagtggttt ttaatgttat gtggttttag gtttttttat    6900
ttttagtgtt ttttggtgtt taggttatgt ttttagtttt gataaggtat gggtgggtgg    6960
gattattttg attatattgg tattttaatt taattttatt atgtttaatt ttttttaaag    7020
tttttttttt ggggaggttt tgggattgga gtaattggga ttttttttggt tgttgatttt    7080
ggagttaggt tttttgtttg ttttgtatta ttttgttatg tttgtatagg ggtttgataa    7140
gtgttattgt ttttgggtta tagaggatat tggagtttag agttggataa ttggtttagg    7200
tttaggttgt atatggtgta gtaggttgtt tgttgtattt tgggggaggt tattttgggg    7260
ttgttgattt gttttgtttt ttgttttagt attgtggtaa tttaatagga aggggtaggg    7320
ttagtttttt ttggaatttg ggtagtgtta aagataaggt gtttttaaaa agtttatgga    7380
aaatgtgtgt tgtgatgaaa tttgtatggt ttttaagttt ttttgtttta aaataaattg    7440
atattaattt gttataatat gtttgaatta gatggagttt gaggtattaa aaaaaagtaa    7500
gatattagtt agaatagagt tttagttaga gtaataggag ttttgttaaa attgaagtaa    7560
atgttaagta ttaaatttat ggtaaaattt gggtggaaga atgaatgggg aaattattga    7620
tgttttataa aaagtgttat agatttatag gaataatgtt ttaaagaaat tagaagttta    7680
tgaatggata atttatttta tgtatttatt tttttgagat agagttttgt tttgttgttt    7740
aggttggagt gtaatggtat gattttggtt tattgaattt tttatttttt gggtttaagt    7800
aatttttttg ttttagtttt ttgagtagtt gggattatag gtgtgtgtta ttgtgtttag    7860
ttaatatttt gtatttttag tagagatggg gttttattat gttggttagg ttggttttga    7920
atttttgatt ttgtgatttg tttattttttg ggtgtagtgt tttatgtttg taattttagt    7980
attttgggag gttgaggtag gtagattatg agattagttt ggttaatatg gtgaaatttt    8040
gtttttatta aagatataaa aattagtttg gtgtggtggt gggtatttgt aattttaggt    8100
atttgggagg ttgaagtagg aaaattattt aagtttggga ggtggaagtt gtagtgagta    8160
gagattgtgt tattgtattt tagtttgggt aatagagtga gattttgttt taaaaaaaaa    8220
aaaaaaaaaa aaaataatta aaaaataaat aaatagaaaa tagagggatg aagttgggag    8280
tagtggttta tgtttgtaat tttaatgttg ggaagttgag gtaggaggat tatttgaggt    8340
taggagttgg aagattagtt tgggtgataa agtaagattt tttttttta taattttttt    8400
```

```
tttttgagat agagttttgt tttattattt aggttggagt gtagtggtgt aattttggtt    8460
tattgtaatt tttgtttttt gagtttaagt gatttttttg ttttagtttt tttagtagtt    8520
ggtattatag gtatttatta ttatgtttag ttaatttttg tatttttagt atagatgggg    8580
ttttgttatg ttggttaggt tggttttgaa tttttggttt taggtgattt atttgttttg    8640
gtttttttaaa gtgttgggat tataggtgtg agttattaaa ttaggttaat tttgttttta    8700
aaaaaaaaaa aaaaaaaggt ttttgttggt ttggtttttg tggattttat tttttggttt    8760
tgtttggaaa ggtgggagat tttttttttt tgtttgggga atagagtggt tagatttgaa    8820

agtttttttt ttttattttt tttttttttg gggggttaag atatatgttt gtttgttagt    8880
ataggtttgt ttttgttgtt aatattaata tattttaatt ttaaattgtt tttggtaagt    8940
ggggaatgga atagtttagt ttttaggtat agtagttgag ggtttagggt taatagtttt    9000
tgggttttgt gtgggtgtag ttggaggggt gggtaggtgt tatatgtttt gtagatgttt    9060
aaggttattt tttttgggta tttagggttt tagtttggag attttagggt tgtagaatga    9120
aagtatattg ggaaagtgag gttattttgg gttgaattag ttgttgtggg ttgagtattt    9180
gggtttagtt tgtgtggagt gagttttttt tggtggttaa tttggggata tggggttagg    9240
agatttaggg gtttagggtt ggtttaggag agtaagagtt tgttatgttg ggagtaggga    9300
ggagggaggg gaggagaaag gagttggagg ggatgaggag gaggtaagga gaggagatag    9360
aggagttgga agagaagaga tagagatggg ggaagggagg aggggagaag agagggggaa    9420
gggaggaaag gggaggtgag gggagagggg ttagaagggt gtgttggagg tggatttttt    9480
tgaggtgggt gggtgggtta atattttttt gttagttagt gggaatagat tgtgtgtatg    9540
tggttataga agtttggttg gtggttatgg gttagtgatt tttgggtggg tgaggggtaa    9600
taggagtaat agggttgttt gggtgttttt gagtttttga gattttaatg ggtttaggga    9660
ttagtattga ggagttgttt ttattttgtg tttttgagtt aattggaaat tattttagat    9720
agaaggtttt ttattaggtt tttggttttg tttttgtttg agtttagttt tagtatttgt    9780
gtgttttatt tgttgggtta gttggttttt gtttttttgtt ttgatgttgg tggatttgtt    9840
gaagatttgt aggtattttt ttatggggtt ggagttaaag tttatttttt tttttagggt    9900
tgagtagttt atatttgttt ttgtgttgga ggtggaggag gaggaggagg aggaggagga    9960
tggggtgggg gagggggggtg gggaggtttt gagttgtttg ggtggttttt gtgtttttgg   10020
gaagtttagg ttggagtttg agttggagtt tgagtttgag ttgaggttgg ggagggtaga   10080
gggttatatg tttggtattt ttggttttgt ggttttgttt ttatttttgt ttttaaagag   10140
gttggtgtgg tttagggttt gttttttttag tttgggggtg ttttgggagg tgtttttgtt   10200
tagtgagtgg gtttttttgtt ttattagttt ttttgatggg gttttggtgt tttttttgtt   10260
gggtagttgg agggggtggg gtgggttttt gttggttgtt ggttgggagt ttttgtttgt   10320
ggggtttggt tgttgtgttg gttggttagg tttttttttt tttgaggtgg ttataggagt   10380
ggttgaagat ggtttttttt tggttttttt tggttgtttt gttttgtgtt gggggttgtt   10440
ggtttgtggg ttttttttgt ttgtatgggg ttttttttata ggttgttttt ggtttttgtt   10500
tttggttttg tttttttttt gggtttttgtt tttgtagttg ggggtaggtg gggttttggt   10560
tttttttttt ttgggttttt tttttttggg gtagttttta ttttgtgagg attggatttg   10620
ggttggggag gtgggtttgg ttgggggtgg ttggaggttt tttatgttgt attgtatggt   10680
tgtttttttg gttttttgta ggttgttttt tttggttttt aggttttttt tggagggtgg   10740
ttgtttggtg gttttgattt tagggttggt tttggttttg ggggtgttgg ttgtggtggg   10800
tttgttattt gggattgtgg tggttttatt ttttgagttt gagaattttg tattgtaatt   10860
gttaaagggg ttatagagtt ttttgggagt aggtgtgtag ggtttattgt tgtgggagtt   10920
ttggggttgt ttggtggttt gtttattttg ggagttggtt ttgttgaggt gttgggttga   10980
gtagttggag agagggttat attttaggtt tgtgggtggt ttggagttgt ttattatgta   11040
tttgttattg ggaatggggt ggttgtgttg ttggggttgg tttatagttt tggggatgta   11100
ttttagggta ttgttatttt ttttattttt gttttgattt ttgtttaggg atgttagtga   11160
gtatttgttg tttggtttgg taggggtggt tagtggggtg ggttggtagt tggtattagg   11220
gtttaatagg ttgtggttgt tggggttgta gttgaaggtt agtgggaagg tatttttgtt   11280
tgggtttata gtggggttgg tgttggggtt gtggggtgtt agggtggggg ttttggtgga   11340
gtggtgttta tgggttgttt ttagtagttt ttggtagtgt tgttgtttta gttttatttt   11400
attgtgtatg gttttgatgg tttggttgat tagttttaat tttagtatat ttgggtgtgg   11460
tttttttttt aggtttaggg tgttattttt tttttgtgtg gggggtttgg gtagtttagg   11520
gttgtagggg ttgtaattta gttttgaagg gaatagagag tatagttgtg attagtttgt   11580
tggagaggag tttgtagtat gggggtggtt ttggttttaa attgtaggga agggaagaga   11640
tttgtttttg aaatttatta tgtattggtg tttgttaagg ttttggggtg tttagttggg   11700
tataggttat ggtgttattt taggttttgt gttgggtttt gttttgtttt gattaatggg   11760
tgttggatgg gtttgggttg tgtgatatat agttttttgg gtattggagg tttttttata   11820
tggtttatga aatgtgtata atgattatgg tggggtggat atttgatagg ttggaatttt   11880
ttggggtagg tgggtgtgga gtttttagta tttagggtgt tgggttgagg taggattggg   11940
ttgggttgat aattatagtt tgatggtgga tataggagag tttttttttta gttaatatgg   12000
```

ttttatttag ttagtagtgt gtttgtggtg tggtggaggt gtgttagtag tgtgttagtg 12060
gtggtgtggt ggtggtgtgt ttgtggtgat gtggttgagt agggttgggg gatggggata 12120
tgtttagttg atgggttatt ttgtaaaatt tataggaaga tttttttttt ttgagatagt 12180
ttttttgttg tttaagttgg agtatagtgg tgtgattttg gtttattgta gtttttattt 12240
tttgagttta agtaaatttt ttgttttagt tttttaagta gttgggatta taggtgtgtg 12300
ttattatatt tagttaattt ttgtattttt agtagagatg ggttttattt atgttggtta 12360
gttttgaatt tttgatttta agtgatttat tgtatttggt ttattttaaa ttaatttttt 12420
aattttaaaa tttttttgtag ttgggtgtgg tggtgtatgt ttgtaatttt aatattttgg 12480
gaggttaagg tgggtggatt atttgaggtt gggagtttaa gattagttag attgatatgg 12540
agaaattttg tttttattaa aaatataaaa ttagttgggt gtggtggtgt atgtttgtaa 12600
ttttagttat ttaggaggtt gaggtaggag aattgtttga atttgggagg tagaggtttt 12660
ggtgagttga gattatatta ttgtatttta gtttgtgtga taagagtgaa attttgtttt 12720
aataaaaaaa aatttgtaga ggtggagttt tattatattg gttaggttgg ttttgaattt 12780
gtagatttaa gtaatttttt tattttagtt tttaagatag ttgggattat agtttaagtt 12840
gttgtgtttg gtttaaagta tatggttgaa atgttttttt ggggggtagt tgggaatatg 12900
agatttgtat gttatttatg tggttttaga atatagtgta gtagattttt ataattttaa 12960
tgttattaat gttagtatag aagttagaat tatagaagtt ttttgtgggg ataggttgaa 13020
ggagtttaaa gaaaatatga gtaagtggag tttagtattt tagtgaagta ataaaaaatta 13080
agtttaaata tttattttag aaatttggaa gttgattatt agaaatttta ttgagtatgg 13140
ttaggtatag tggtttatgt tgattatttt agtattttgg gaggttgagg tgggaggata 13200
ttgtttgagt ttaggagttt aagattagtt tggatgatat agattttatt ttttaaaaaa 13260
atatttaaaa ataagttgag tatggtgatg tatatttgtg tttttagtta tgtgggaggt 13320
taaagtggga gaattatttg agtttagggg tttgaggttg tagtgagtta ggattgtgtt 13380
tttgtatttt ggtttggata ataaaataag attttagttt ttaaaaaaat aaaataaagt 13440
tttattgaat gtaattttgt aatattttta aaggttttta aaggttaata ggtttgattt 13500
tttggagttg gtaggaaatg gtgttaaaga tgtttgtgtt ttaatttttg gaatttgtag 13560
atggtatatt tttttagatg gtaaatgggg ttttagatgg ggtgaaattt tttttgtttg 13620
ttattgaggg ggttatggat aggttgagat tttgtagagt agttttgtaa ttattttatg 13680
tagttttaat attattattt ttagatttgg aatttttaga atttgtgtta tagatagatt 13740
gtatagggta ggtatagatg tgtttggtgt agtttttatt gtaaaaatat aataataaat 13800
aatgtaggag atgatttaga agttgaaatg taggttattt gtggttaagg gggaggtgtg 13860
tgttttggag ggatttttaa gttatattgt tatggggaag aagaggggta aggaatgttg 13920
tatattttta tttttgaaaa gtagttttat aaatttagtg agattagatt ttaataaagg 13980
tgtatgggtt tatattaagt ttgtagtttt atggggtata gtaaatgtaa tttggttttt 14040
atttatgttg tatttaatat taattgttta agagggatgt gaatgttgat atatattttt 14100
gataggttga taaggagttg tgatattatg tagtaaaagg taatagtttt atgggaattt 14160
gtttgtatta agatgttatt tggttttgat ttgattttga ttgaatgtgt agtttgtttg 14220
aaggttgggt tttaggtgtg ttttttttat ttagaaaatt tgtttgtttt ttggtttatt 14280
tggagattga tttttatagt gtgttaagat ttttgtgtag agaaatggtt ggatgtagtt 14340
aaggggaggt aggtataaga ttttttattg tgggtgatgt agagaatagt aatagaattg 14400
gaggtttttt ttatggtttt tatttatatt tttttaagtt ttagtaggta taagaaagaa 14460
atggttggtt ttggttggtt gtggtggttt atatttgtaa ttttagtatt ttgggaggtt 14520
gaggtgggtg gattatttga ggttaggagt ttgagattag tttgattaat aaggaaaaat 14580
tttattttta ttaaaaatat aaaaaaatta tttagttgtg gtgatttatg tttgtaattt 14640
tagttatttg ggaggttgag gtaagagaat tgtttgaatt tgggaggtgg aagttgtagt 14700
gagttaagat tgagttattg tattttattt tgggtaataa gagtaaaatt ttattttaaa 14760
aaaaaaaaaa aaaaaaaaag aaagaaaaag aaaaagaaaa aaagaaatgg ttggtttttt 14820
aagttgtttt tttgagatat atttattagt agaataaaag ttttttttagg taagttttta 14880
ttttaatttt tgggatttgt ggttaggtta ttttatttag ttaatagatt gtaggtgtga 14940
tttgaataaa ggattttgag ggtgggtgaa tattttgtat tatttgggaa tttagtgttt 15000
ttatagggtt ttttaaagag ggaggtagga gggtgagagt tagagagatt ggaaaaggtt 15060
gtgttgtggg tttgaagggg aggaaggtgt tttgagttga gggatgtagg tgtttttaga 15120
tgttgggaaa ggtgggtatt gatttttttt ggaagttttt gggaggttta gttttgttta 15180
tagtttgatt tgagtttagt gaggttgatt ttggattttt tagtttagtg ttgagagaga 15240
gtgtgttgtt ttaaggttta tggggatgaa tgtagtttta ttgggtgatg aaagtgtagt 15300
ggaaaatagt tgtttgttgt ttgtatttgt gaaggtggaa ggtttttttt gtttttggga 15360
attttgtagg gatagtggga ggttgggatt tggaggttgt agaggaggtg ggaggaggtt 15420
tggttatggg tagttgttgt tttttttttg aaattatagt agtgttgtgt ttatttttaa 15480
aattatattt agggattagg tatggtggtt tatgtttgtt attttagtat ggtggtttat 15540
gtttgttatt ttaggtatgg tggtttatgt ttgttatttt agtattttgg gagattaagg 15600
tgggtagatt attagtttag gagtttaaga ttattttggt taatatggtg aaattttatt 15660

tttattaaaa atataaaatt agttaggtat gggggtgtgt atttgtaatt ttagttattg 15720
aggaggttga ggtaggagaa ttgtttgaat ttaggaggta gaggttgtag tgagttaaga 15780
ttatattatt atattttagt ttggtgatag agtaagattt tgttttaaaa aaaaaaaaaa 15840
aaaaaaaaat tatatttagt gttaggttta gtggtttatg tttgtaattt tagtattttg 15900
gaaggttaag atgagtagat tgtttgagtt taagattagt ttaggtaata tggtgagatt 15960
ttgtttttat aaaaaaatat aaaaaatgag ttgggtgtga tggttgtatg tttgtggttt 16020
tagttatttg gaaggttgag gtgggaggat tttttgagtt taagaggtta aggttttaat 16080
gagttgtgat tatattattg tattttagtt tgggtaatag agtaagattt tgtttttaaa 16140
ataaataaaa ttaagtagta tttagataag aggaaggatt tgtgtgtttg ttttggatta 16200
tttggaattt tgtgggattg tgtggagggt tatgaggtat gggtaggtag gggaatatta 16260
tatagaagtt ataggttttt ttttgtttgt gttaggttat agttaaggta tttttaggaa 16320
atgttatttt atgtgtttag taggtgtaga ggggtttatt ttggtattat tttatagtta 16380
aggattttgg gagttagtga gggtatttaa ttaggattag taattgtggt tgttagtttt 16440
tttgtgtggg gagggatagt tgttaggtta ggaggatgtt tttggtaggg aaatggttgg 16500
ttggggttgg gtgttagttt ttatttaggg ggagggttgt ggaggttagt agtagtttaa 16560
gggtgttttt ttggtttatt ttattggggg aggttttggt tttttttga tttagttggt 16620
tgtagagtat gttattttgt tatttgtttg gtgaataggg taattagtgt tagttttttt 16680
ttagttttgg aagtttggtt gtttagtgat gtggaggggt taggttaagg ttattgtggt 16740
gtgagttgta gagatttatg aaggggaggt gatagagtat aattattgag gaggagtaag 16800
gttaggttag ggagatattg gttttttttt ttaaggtttt agttaatggg gatgtagaga 16860
ggaggaagag tttgtttaat gggaaggttt tttttttag gtaggggtgg gtagtagtga 16920
gattgggttt taggagggta ggtgggggtg gaggggattg aggtattttt aaggggttaa 16980
ggataaggga ttttatttag gggtttttat ttttttgttt tggattttag ggttttaaga 17040
tatagtttgt aagattaatt tagtttaggg gagttaagag ttataggtaa tatatatatt 17100
agttagtaga tatttgtgag tttttttttaa aggaaggaaa tttagatttt tggatattgt 17160
ggattttgaa aggagagatt atttttttaa aaggtgtttt tggaaggttt tggaaggaat 17220
agtatttgtt ttttaggagt ggagttaggt ttttaagtag agtgtaggat aggtgataag 17280
ggtttaattt gggggatgtg ggtttgtttt tttaattgga agtttgtgtg agtatttgtt 17340
ttgttgtttt gtggagatag gtattgttta ggttggagtg tagtggtatt atgatggttt 17400
gttgaagttt tgtttttttg gttttagtga tttttttgtt ttagttttgt aagtagttgg 17460
gattatttgt gtgtattatt atatttagtt aatttttttt ttttttttggt agagatgggg 17520
gtttgttgt aattgtttag gttggtttta aatttttaag tttaagtggt tttttttgttt 17580
tggtttttta aagtattgtt tgttttttaa gggttgtgaa atttgtttta gtttttagtt 17640
aatagatggg aaagagtagg ggtatttgga ggttggaggg tatattttgt agaggggggtt 17700
ttagtatttt tatttttggg tggaggttgt tattattttt ataaagattg tattaaaaag 17760
aaagtaggta tgttaggtgg gtttgagaag attgagatta ggatttgtat tatggttttt 17820
ttttttgtg gttattgggt ttttttattt tgtagtttgt gatttattgg tgttggaagg 17880
tagtattttg gtttgggtgt tttagttagg tttggaagtt ttatatagga agttgtttgg 17940
ggtttggtta tggttgtttg tagtttagaa tagggggtat ggtgtgttga gtttttgaaa 18000
ggttatttga agttggtata tattattgtt tttttggatt tttttgtttt attatgtttt 18060
agagggttgg atttgggtat ttgttaggat tggaaaggtt ttagggtggt atgtgtatgt 18120
tgttaggtgt tttgggagtt tatttagggg gttatattta aaggttgagt ttgggttagg 18180
tgtggtggtt tatatttgta atgttagtat tttgggaggt ggaggtggga ggattattga 18240
ggttaggagt ttgaaattag tttggttaat atggtgaaat tttatttttta ttaaaaaata 18300
taaaaaaaaa ttagttaggt ttggtggtag gtgtttgtaa ttttagttag ttgggaagtt 18360
gaggtaggag aattatttaa atttgggaag tggaggttgt agtgagttga gaatgtatta 18420
ttgtatttta atttgggtga tagagttata tatgtatata tatatatata tatatatata 18480
tatatatata tataaatgaa tgttgatttt ggaagtagga ggggtttgtt taggttttgt 18540
tttatggttg ggaattattt tggataggag taggtgggtt ttttttttgga tgtggtttag 18600
gttagtttgt ttttgggaaa tatggtgaat tggttgatag gaaaagttta tttttagagg 18660
tttgggagtt gttttttttt ttttgtttta ttttaaggtt aggattggga ggtattattt 18720
agttttagtg gttttgagag gttataatgt ttggttggtt atttttttta gtttttttag 18780
ttagggttta gtttagggta atgtagttta gttttgtttt ggtttaggag agagtttatt 18840
ttttatatta tttattttga ttgtattttt tgagattagt gttttgtagt tttgggatgg 18900
gttggatgat aaagatgata tttttttttta gggttttagg tgtggggttt gggattagta 18960
ttatttgttt aaggttattt tttggttttt aaattttttt ttggtttaga aattttttat 19020
tttgggtatt tattttttttt tttgttttta ttgtttttgt tttattatat ttaatttatt 19080
tatatagttg ttttgttttt agtaatgggg agaatttggt atgagtttta attttatatg 19140
gagattgaaa ggtagtaaaa atgataagat tatggagtat gaagatttta atttgaattt 19200
ttagaatgtt ttttagtgag atttgtttaa atttttgttt ttttttagtt agtttatttt 19260
aaagatgagg atatttagtg ttagtgagag ttggtagttt ttagttttat aaggagtttt 19320

```
aggtttttt tatttagatg gggagattta gtggttttaa agaatttggt aggttggttg   19380
gatatgatgg tttatgtttg taattttagt attgtaggag gttgagatgg gtggattatt   19440
tgaggttagg agtttgagat tagtttggat aataaggtaa aattttgttt ttattaagaa   19500
aatataaatt agttgggtgt ggtggtaggt gtttatagtt ttagttattt gggaggttga   19560
gataggagaa ttatttgaat ttgggaggtg gagattgtgg tgagttaaga ttatgttatt   19620
gtattgtagt ttgggtaata gagtaagatt gttttaaaaa aaaaaaaaaa aaaaaaaaag   19680
aatttggtag gtttagtttt tgttttggat attgtttggt gggtttgtag ggtttttaat   19740
gtaattaggt attagtagga ttggtttttt tagtttaggt agtgggtttt gtatgttggt   19800
ttagaatagg ttaggtagag attttagatg gaaatagata gttttttttg tttggggttt   19860
agttggataa aaaagataag tttgatattg tggtatgaga taagtaagat tatttttaat   19920
ttagtttttg ggggagttat ttgggtggtt tgaggttttg aaggtggaga tagaagagga   19980
tatttttaaa atagttatag tggtagagaa ggtatggggt tgatttttgt gaagtgtaag   20040
gtaggggttg gttgtgttta tgggagtgtt tattttataa tagatttttt gggaagaggg   20100
gtttggagga tagtattttt gaagtttatt ttttgtggat ggtagagtaa ttataaggtt   20160
tttataaatg gttatatgtt tattttgggg gtttttgttt ttggggtttt ttttttaatt   20220
tttatagtgg gatgtttttt agttttattt ttggatatgt gggtgtttgg ggttttttat   20280
gtatagttta ttggatagat aggaatttgt tgtttagagg taaagggatt agtattttgt   20340
atttagagga ggttttatag tttttagatt atatggggta gaaatagggt tatttggaga   20400
gggttttgag gaaaagttta gttagtgttg ggggtttgga tttttattat ttagtagttt   20460
ttaatatttt ttttgaaatt taaggtggtt tagttttgtt ttattttttta tattagattt   20520
tatttaggtt ttagagtttg ttgtggaggt tgggttgtgt ttgtagtagt ggtatttggg   20580
gtttatttta ttgtttttag ggtttttttt gatttttagg gttgtgattt gagttttttt   20640
tttgttttaa ggtattgttt tttttatttt gatagtgtag tgtgtagtgt taggattgtt   20700
tagtaggtgg agttatatgg taggtttag gggagaaaaa gaaaaaggtt ttggtttgat   20760
tggggattat tagttttgtt ttttggggtg ttttgggtat ttatttattt tatggggtgg   20820
ttgtttttat atgttatgat tggatgtgtt gttgttttaa ggtttatagt ttgaggttag   20880
gggataggga tggtaattag gatagaggtt attgtttttt aaaataatgt agaaataggt   20940
tgggtgtagt agtttatggt tttaattttt gtgttttggg aggttgagtt taaggttgag   21000
gtaggtggat gatttgaggt gaggagtttg agattagttt ggttaatatg gggaaatttt   21060
gtttttatta aaattataaa aaattagttg ggtgtggggg tgtttataat tttagttatt   21120
taggaggttg aggtaggaga attgtttgaa tttggaaggt ggagtttgta gtgagttgag   21180
atggtgttat tgtattttag tttgggtaat aagaatgaaa ttttatttta aaaaaaaaaa   21240
aaaaaattag ttgggtgtgg tggtttatat ttgtaatttt agtattttgg aaagttgaag   21300
tgggtggatt atttgaggtt gggagtttga gattagtttg agtaatatgt agaaattttg   21360
tttttattta aaaaaatata aaattagtta gttgtggtgg tatatgtttg taattttagg   21420
taattttatg tttgggtgta gtagtttatg tttgtaattt tagttatttg ggaggttgag   21480
gtaggagaat tgtttgaatt tgggaggtgg atgttgtggt gagttgagat tgtgatattg   21540
ttttttagtt tgggtgatag ggtgagattt ttttttaaaa aaaaaaaaaa attgaaatag   21600
aagggtataa atggtaaagg ataagaattg gtagggagga gttagtggga tttttttttt   21660
ttatgttttt ttatttttgt taagagttta ttttggttgt gagttatttt tatggtttat   21720
atagagtaag ttaagagttt agtttttagt tgggtatggt agtttatgtt tgtaatttta   21780
gtatttggga ggataaggtt ggtggatttt ttgagtttag gagtttgaga ttagtttggg   21840
taatatggtg aaattttgtg tttattaaaa atataaaaat tagtagttgg gtatggtggt   21900
ttatgtttat aattttagta ttttggaaag ttgaggtggg tggattgttt tgagtttagg   21960
agtttgagat tagtttgggt aatatggtga aattttgttt ttattaaaat ataaaaaatt   22020
agttgggtgt ggtggtgtgt ttttgtaatt ttagttattt gggaggttg               22069
```

<210> 298
<211> 22069
<212> DNA
<213> Artificial Sequence

<220>
<223> chemically treated genomic DNA (Homo sapiens)

<400> 298

```
tagtttttttg agtagttggg attataggag tatgttgtta tgtttagtta atttttgta     60
ttttagtaga gatagagttt tattgtgttg tttaggttgg ttttaaattt ttgagtttaa    120
ggtagtttgt ttgttttagt tttttgaagt gttgggatta taggtgtgag ttattgtgtt    180
tagttgttga tttttgtatt tttagtagat atagggtttt attatgttgt ttaggttggt    240
```

```
tttgaatttt tgggtttaag agatttatta gttttgtttt tttaagtgtt ggggttatag     300
gtgtgagtta ttgtgtttgg ttgagagttg gatttttaat ttattttgtg tggattatga     360
aagtggttta tagttaaggt gggtttttgg tagggatgga gaggtatagg gggagaaggt     420
tttgttggtt tttttttgtt ggtttttgtt ttttgttatt tgtatttttt tgttttagtt     480
tttttttttt tttgaaaagg agttttgttt tgttatttag gttggagagt aatgttatga     540
ttttggttta ttgtaatgtt tgttttttga gtttaaatga tttttttgtt ttagtttttt     600
gagtagttgg gattataggt gtgagttatt gtgtttaggt atgggattat ttgggattat     660
aggtatgtgt tattatagtt ggttaatttt gtattttttt aagtagagat aggatttttg     720
tatgttgttt aggttggttt tgaattttta attttaggtg atttgtttgt tttgattttt     780
taaagtgttg ggattatagg tgtgagttat tatgtttagt tggttttttt tttttttttt     840
gagatggagt tttgttttttg ttgtttaggt tggagtgtgg tggtgttatt ttagtttatt     900
gtagatttta ttttttgggt ttaagtaatt tttttgtttt agttttttga gtagttgggg     960
ttataggtat ttttatattt ggttaatttt ttgtagtttt agtagggatg gggttttttt    1020
atgttggtta ggttggtttt aaatttttta ttttaaatta tttatttgtt ttggttttgg    1080
gtttagtttt ttagagtgtg gggattagag ttgtgagtta ttgtgtttag tttgttttta    1140
tattgttttg aaaggtagtg atttttgttt tggttattgt ttttgttttt tagttttgag    1200
ttgtaagttt tgggatagta gtgtgtttag ttatggtatg tggggatggt tgttttatga    1260
ggtgaataaa tgtttaggat gttttagaga gtagagttgg tggtttttgg ttaggttggg    1320
attttttttt tttttttttt tggattttgt tgtgtggttt tatttgttgg gtggttttgg    1380
tattgtatat tgtgttgttg aggtggggag ggtggtgttt tggagtagag gggaggtttg    1440
gattgtagtt ttgggggttg aggagaattt tgggggtagt ggggtagatt ttaggtgttg    1500
ttgttgtagg tatagtttag tttttatagt aagttttgag atttgggtgg agtttggtgt    1560
ggggggtggg gtagagttgg gttattttgg attttagaga gggtattgga agttgttggg    1620
tggtggaggt ttaggttttt ggtattggtt ggattttttt ttgaagtttt ttttaggtga    1680
ttttgttttt gttttgtgtg gtttaggagt tgtgggattt tttttgggtg tagagtgttg    1740
gttttttttgt ttttgagtag tgggtttttta tttgtttagt gggttgtgta tggagggttt    1800
tgggtgtttg tatgtttaag gatggggttg gaggatgttt tattgtgagg gttggagggg    1860
aggttttagg agtagggatt tttagagtgg atgtatggtt atttgtgggg attttatggt    1920
tattttgttg tttgtaggag gtggatttta ggggtgttgt tttttagatt ttttttttta    1980
gagggtttgt tatgggatgg gtgtttttgt ggatataatt agtttttgtt ttgtgttttg    2040
tgagggttag ttttgtgttt tttttgttgt tgtggttgtt ttgggaatgt tttttttttgt    2100
ttttattttt aaagttttag gttgtttaga tggtttttttt aggggttagg ttgggggtag    2160
ttttgtttat tttatgttgt agtgttaggt ttattttttt tgtttagtta agttttaggt    2220
gggagggatt gtttgttttt atttggggtt tttgtttggt ttgttttggg ttagtatgtg    2280
gggtttattg tttgggttgg aggggttagt tttgttgatg tttggttgtg ttgagagttt    2340
tgtgggttta ttgaatggtg tttggagtag gggttgggtt tgttgggttt tttttttttt    2400
tttttttttt ttttgagata gttttgtttt gttgtttagg ttgtagtgtg atggtgtaat    2460
tttggtttat tgtagttttt gtttttttggg tttaagtgat tttttttgttt tagtttttta    2520
agtagttggg attataggtg tttgttgtta tgtttggtta atttgtgttt ttttagtaga    2580
gatggggttt tattttattg tttaggttgg ttttgaattt ttgattttag gtgatttatt    2640
tgttttggtt ttttatagtg ttgggattat aggtatgagt tattgtgttt agttagtttg    2700
ttgggttttt tagagttatt gagtttttttt gtttgggtgg gaggggtttg gggtttttttg    2760
tggggttagg agttgttggt ttttgttggt attgggtgtt tttatttttg aaatgggttg    2820
attgagagaa gataggagtt tggatgggtt ttattagaga atgttttgga agtttaggtt    2880
gggattttttg tgttttatga ttttgttatt tttgttgttt tttagttttt gtgtggggtt    2940
ggagtttatg ttaggttttt tttattgttg gggataggat agttgtgtgg gtggattgag    3000
tgtggtgggg tgggggtagt gggagtagag aggagagtgg atgtttaggg tgaggggttt    3060
ttgggttaag ggaaggtttg ggggttagga gatggttttg ggtagatagt gttggtttta    3120
ggttttatat ttagaatttt gggaagaaat attattttttg ttatttagtt tgttttgggg    3180
ttgtagggta ttggttttag gagatgtggt taggatgggt ggtgtaggga gtgggttttt    3240
ttttaggttg ggataaggtt gggttgtgtt gttttgggtt gagttttggt tggagaggtt    3300
ggggaagata gttagttagg tgttgtggtt ttttaggatt attggggttg ggtggtattt    3360
tttagttttg gttttggggt gaagtaggga ggaaggggta gtttttaggt ttttggaggt    3420

aggttttttt tgttagttaa tttattatgt tttttagaaa tagattgatt tgggttatat    3480
ttaggaagga gtttatttgt ttttatttaa agtaattttt agttgtggga tggggtttgg    3540
atgggttttt tttattttta gagttggtat ttatttatgt atgtgtgtgt gtatgtatgt    3600
atgtatgtgt gtatgtatgt atagttttgt tgtttaggtt ggagtgtagt ggtgtgtttt    3660
tggtttattg taatttttat tttttaggtt taggtgattt ttttgtttta gtttttttgat    3720
tagttgggat tataggtgtt tgttattagg tttggttaat ttttttttgt atttttttagt    3780
agaggtggag ttttattatg ttggttaggt tggttttgaa tttttgattt tggtgatttt    3840
```

```
tttgttttg ttttttaaag tgttagtatt ataagtgtga gttattatgt ttggtttaaa 3900
tttggttttt agatgtgatt ttttgggtgg gtttttaggg tatttagtag tatgtatatg 3960
ttattttggg gtttttttag ttttgatagg tatttagatt taattttttg aggtatggtg 4020
ggatagggag gtttagggag gtagtggtgt gtattagttt taggtgattt tttaggggtt 4080
tagtatgttg tattttttgt tttgagttgt aggtagttat ggttgagttt taggtaattt 4140
tttgtgtgaa atttttagat ttggttggag tgtttaggtt aggatgttgt tttttagtat 4200
tagtgggtta taggttgtag ggtgggggag tttagtgatt atagggagga gaggttgtgg 4260
tgtagatttt gattttagtt tttttggatt tgtttagtat gtttgttttt tttttggtgt 4320
agtttttgtg gaggtggtag tagtttttat ttgggagtga aaatgttgaa atttttttttg 4380
tagagtgtgt tttttggttt ttaggtgttt ttgtttttttt ttatttgttg gttaaaggtt 4440
ggggtaggtt ttatagtttt taaaagataa gtagtgtttt gggaggttga ggtgagaaga 4500
ttgtttgagt ttagaagttt gagattagtt tgggtaatta tagtgagatt tttgttttta 4560
ttaaaaaaag aaaagaatta gttggatgtg gtggtgtatg taggtggttt tagttatttg 4620
tgaggttgag gtgggaggat tgttggagtt ggggaggtaa agttttagtg agttattatg 4680
gtattattgt attttagttt gggtaatgtt tgtttttata aaataataaa ataagtattt 4740
atataggttt ttagttagaa agataagttt atgtttttta gattggattt ttattatttg 4800
ttttgtgttt tgtttggggg tttggtttta tttttgggag gtaggtgtta ttttttttaa 4860
gatttttttaa aagtattttt tggggaaatg gttttttttt ttagagttta taatgtttag 4920
aggtttgaat tttttttttt tgggaagggt ttgtgggtgt ttgttggttg gtgtgtgtat 4980
tgtttgtgat ttttggtttt tttgggttgg gttgatttttg taggttgtgt tttagagttt 5040
tggggtttag gataaggagg tggaagtttt tgagtggagt tttttatttt tagtttttta 5100
gagatgtttt agttttttttt atttttgttt gtttttttgg ggtttagttt tattgttgtt 5160
tatttttgtt tggggaggag agttttttttg ttaggtgggt ttttttttttt ttttgtattt 5220
ttgttagttg gagttttggg aggggagatt agtgtttttt tgatttggtt ttgtttttttt 5280
ttagtaattg tgttttgtta tttttttttt atgaattttt gtagtttatg ttatagtggt 5340
tttggtttgg tttttttatg ttattgggta gttgggtttt tggggttggg ggagggttgg 5400
tattagttgt tttgtttatt aggtgggtgg tagggtggtg tgttttgtag ttggttgggt 5460
tagggaggag ttggagtttt ttttagtgaa gtgggttggg agggtgtttt tgagttgttg 5520
ttggtttttg tagttttttt tttgggtggg ggttgatatt tagttttagt tggttgtttt 5580
tttgttggga gtattttttt ggtttggtag ttgttttttt ttgtatagag gaattggtag 5640
ttatagttgt tgattttagt tgggtgtttt tgttgatttt tagggttttt ggttgtgaga 5700
tggtgttaga atgggttttt ttgtgtttgt tgggtatatg gggtgatgtt ttttggaaat 5760
gttttggttg tagtttggta taggtaaaga agaatttgtg gtttttgtgt gatgtttttt 5820
tgtttgtttg tattttgtgg ttttttatat agttttgtgg agttttgagt ggtttaaaat 5880
agatatatgg atttttttttt ttgtttaaat gttatttaat tttatttgtt ttagagatag 5940
gattttgttt tgttgtttag gttggaatgt ggtggtgtga ttatagttta ttgaagtttt 6000
gatttttttag gtttaagaga ttttttttatt ttagtttttt aagtagttgg gattataggt 6060
atgtaattat tatatttagt ttattttttg tattttttttg tagagatggg gttttgttgt 6120
gttgtttagg ttggttttga atttaagtag tttgtttatt ttggtttttt aaagtgttgg 6180
gattataggt gtgagttatt gagtttggta ttaagtgtaa tttttttttt tttttttttt 6240
tttgagatag agttttgttt tgttgttagg ttggagtgta gtggtgtgat tttggtttat 6300
tgtaattttt gtttttttggg tttaagtgat ttttttgttt tagttttttt agtagttggg 6360
attataggtg tatgtttttta tgtttggtta attttgtatt tttagtagag atggggtttt 6420
attatgttgg ttaggatggt tttgaatttt tgagttggtg atttgtttgt tttggttttt 6480
taaagtgttg ggatgatagg tgtgagttat tgtgtttggg atgataggtg tgagttattg 6540
tgttgggatg ataggtgtga gttattgtgt ttggttttta aatgtaattt taaagatggg 6600
tataatattg ttgtgatttt aagggaaagg tggtagttgt ttgtgattag gttttttttt 6660
atttttttttg tagttttttgg gttttggttt tttgttgttt ttgtagggtt tttgggaata 6720
ggaggggttt tttgtttttg taggtatagg tagtgggtag ttgttttttta ttgtattttt 6780
attatttggt gaggttgtat ttgtttttgt gggttttaaa gtagtatatt tttttttagt 6840
gttgggttgg gaagtttaaa gttagttttg ttgggtttaa gttaagttgt gggtaggatt 6900
gggttttttg aaggtttttg gggagagtta gtgtttgttt tttttagtat ttggaggtat 6960
ttgtattttt tggtttaggg tgtttttttt tttttaaat ttatagtata gtttttttta 7020
gtttttttga tttttatttt tttgtttttt tttttaaagg atttgtgag gatattgggt 7080
ttttggataa tgtaagatgt ttatttattt ttagggtttt ttgtttaagt tgtatttgta 7140
gtttattggt tgagtgaggt ggtttggtta taggttttag ggattaggat ggggatttat 7200
ttggggaggt ttttgttttg ttggtgggtg tgttttgggg gagtagtttg ggaaattagt 7260
tatttttttt tttttttttt tttttttttt tttttttttt tttttttttt ttgagatgga 7320
gttttgtttt tgttgtttag gatagagtgt aatggtttaa ttttggttta ttgtaatttt 7380
tgtttttttag gtttaagtga ttttttttgtt ttagtttttt gagtagttgg gattataggt 7440
atgggttatt atggttgggt aatttttttg tatttttagt agagatgggg ttttttttttg 7500
```

ttggttaggt tggttttgaa tttttgattt taggtgattt gtttgtttta gttttttaaa 7560
gtgttgggat tataggtgtg agttattata attggttgaa attagttatt ttttttttat 7620
gtttgttggg gtttgagggg gtgtgggtgg gggttatagg ggaagttttt agttttgttg 7680
ttgtttttttg tgttgtttat agtgggaggt tttgtgtttg tttttttttg gttgtgttta 7740
gttatttttt tgtatggaag ttttagtatg ttgtgggggt tgatttttag gtgggttgaa 7800
gggtaggtag gtttttgag tggaagaaat atatttgagg tttgattttt agataagttg 7860
tatgtttagt tagaattaag ttagagttaa gtggtgtttt ggtgtgggta ggtttttatg 7920
gggttgttgt tttttgttat atggtgttat agttttttat tagtttgttg gggatgtgtg 7980
ttagtattta tgtttttttt gggtagttgg tgttgggtgt agtataaatg aaggttagat 8040
tgtatttgtt gtattttatg ggattgtggg tttggtgtgg gtttgtgtgt ttttgttgga 8100
atttgatttt attgggtttg tggagttgtt ttttaaaggt gagagtgtgt aatatttttt 8160
gttttttttt ttttttatag tagtgtgatt tggaggtttt tttagggtgt atgttttttt 8220
tttaattata ggtgatttgt gttttggttt ttaggttatt ttttgtgtta tttattgttg 8280
tgtttttgta gtgaaggttg tattagatgt atttgtattt gttttgtgtg gtttgtttgt 8340
ggtatgggtt ttgaggattt tgagtttgaa ggtggtggtg ttgaagttgt atgggatggt 8400
tgtgaaattg ttttgtaaga ttttggtttg tttatggttt ttttagtgat gggtaaggga 8460
gattttattt tatttagagt tttatttgtt atttaagggg atatattatt tataggtttt 8520
aggaattagg atgtagatgt ttttggtgtt attttttgtt aattttagag gattagattt 8580
gttgattttt agagattttt agagatgtta tggaattata tttagtggaa ttttatttta 8640
ttttttttaag agttggggtt ttgttttgtt gtttaggtta gagtgtagag gtataatttt 8700
ggtttgttgt agttttaagt ttttgggttt aagtgatttt tttattttag tttttttatat 8760
agttaggagt ataggtgtgt attattatgt ttagtttatt tttaagtatt tttttaagag 8820
atgggatttg tgttatttag gttggtttta aatttttgag tttaagtagt attttttttgt 8880
tttagttttt taaagtgttg agatgattgg tgtgagttat tgtgtttggt tgtgtttagt 8940
agaattttta atggttaatt tttaagtttt tggagtaaat atttgaattt ggtttttatt 9000
gttttgttgg aatattgggt tttatttgtt tatgtttttt ttagattttt ttggtttgtt 9060
tttatgggag gtttttgtgg ttttgatttt tgtgttgatg ttggtggtat tgaggttgtg 9120
agggtttgtt atattatgtt ttggaattat gtaaataata tgtgagtttt atgtttttag 9180
ttgtttttttg ggaagatatt ttagttgtgt gttttaagtt aggtatagta gtttgggtta 9240
tagttttagt tattttggag gttgaggtgg gaggattgtt tgagtttatg agtttaaagt 9300
tagtttggtt aatatggtga gattttattt ttataaattt ttttttgttg agatggggtt 9360
ttatttttgt tgtgtaggtt ggagtgtaat ggtgtgattt tggtttattg aaatttttgt 9420
tttttaggtt taagtgattt ttttgtttta gttttttgag tagttgggat tataggtatg 9480
tgttattatg tttggttaat tttgtatttt tagtagagat ggggtttttt tatgttggtt 9540
tggttggttt tgaatttttg attttaggtg atttgtttgt tttggttttt taaaatgttg 9600
ggattatagg tgtgtgttat tgtgtttagt tataaaaagt tttgaaatta aaaaattaat 9660
ttaaagtagg ttaggtgtag tggattattt gaggttagga gtttgagatt ggttaatatg 9720
gtgaaatttt atttttatta aaaatataaa aattagttgg gtgtgatggt gtatgtttgt 9780
aattttagtt atttgggggg ttgaggtagg aggtttgttt gaatttggga gatagaggtt 9840
gtggtgagtt aagattgtat tattatattt tagtttgggt agtagagaga ttgttttaaa 9900
aaaaaaaaat ttttttgtgg attttgtagg gtggtttatt aattgggtat gtttttattt 9960
tttggttttg tttagttata ttattgtagg tatattgtta ttatattatt gttggtatat 10020
tgttggtata tttttgttat attgtgggta tattgttggt tgggtggggt tgtgttggtt 10080
gaggaagggt ttttttgtgt ttattgttag gttgtggttg ttggtttagt ttagttttgt 10140
tttagtttgg tattttgaat gttgggagtt ttatatttgt ttgttttggg gagttttagt 10200
ttgttgagtg tttattttat tatggttatt gtgtgtattt tatgagttgt gtgagggagt 10260
ttttagtatt tagggagttg tgtgttatgt agtttaggtt tatttagtgt ttattggtta 10320
gggtaaggtg gggtttggta tagggtttga gatggtgtta tggtttgtat ttagttgggt 10380
attttagagt tttggtgggt gttagtgtgt ggtgggtttt ggaggtaggt tttttttttt 10440
ttttgtagtt tgagattggg gttgttttta tgttgtgggt ttttttttgg taggttggtt 10500
atagttgtgt tttttgtttt ttttagggtt gggttatgat ttttataatt ttgagttgtt 10560
taagtttttt gtgtagaggg agaatggtat tttgggtttg ggggaggagt tgtgtttgga 10620
tgtgttggag ttggagttgg ttaattaggt tattgaggtt gtgtgtagtg aggtggagtt 10680
ggagtagtgg tgttattggg agttgttgga gatgatttgt gagtattgtt ttgttgaggt 10740
ttttgttttg gtgttttgtg gttttaatgt tagttttatt gtgggtttgg atgaggatgt 10800
tttttttattg gtttttgatt atagttttgg tagttatggt ttattaagtt ttgatgttgg 10860
ttattagttt attttattgg ttgtttttgt tgagttgggt agtaagtatt tgttggtgtt 10920
tttggatagg ggttagggta gaggtggagg gggtggtggt gttttggaat atgtttttaa 10980
ggttgtgagt tagttttggt ggtatagttg ttttgttttt agtggtaagt atgtggtgga 11040
taattttagg ttatttatag atttggagta tgattttttt tttaattatt tggtttggta 11100
ttttagtagg gttagttttt gggatgagtg ggttgttaag tggttttggg gtttttgtgg 11160

tagtgagttt tatatatttg tttttaagaa gttttgtgat ttttttggta gttgtgatgt 11220
aaggttttta gatttagaag atgaggttgt tatggtttta ggtaatgagt ttattatggt 11280
tagtattttt aaagttaggg ttgattttga gattaaggtt attgggtagt tattttttaa 11340
agagggtttg gaggttgagg ggggtggttt gtgggagatt aaggagatgg ttgtgtagtg 11400
tgatgtgggg gatttttagt tgtttttagt taagtttgtt tttttagttt aggtttagtt 11460
tttataggat gggggttgtt ttaaggaggg aaaatttaag aagaaaaaaa ttggggtttt 11520
atttgttttt agttgtaaag atggggttta ggggaaggat aagattaagg ataagggttg 11580
agggtggttt gtggagaagt tttgtgtgga taagaagggt ttgtaggtta gtagtttttg 11640
gtgtaaggta gagtggttgg aagggattaa gaagaagtta tttttggtta tttttgtggt 11700
tattttaggg aaagggaggt ttgattggtt agtgtggtgg ttgagtttta taagtgggga 11760
tttttgattg gtggttggta gaggtttatt ttgttttttt tagttgtttg ataggaagag 11820
tattaaggtt ttgttgggga agttagtgga gtggaaagtt tgtttattag atgagggtgt 11880
tttttaggat gtttttaagt tgaagaagtg ggttttgagt tatgttgatt tttttgggga 11940
tgagagtgag gatgaggttg tagggttagg ggtgttgagt gtgtggtttt ttgttttttt 12000
tagttttagt ttggatttag attttgattt agattttagt ttgggttttt tggaggtgta 12060
ggggttgttt aagtggttta aggttttttt gttttttttt tttgttttat tttttttttt 12120
tttttttttt ttttttattt ttagtgtggg ggtggatgtg gattatttgg ttttggagaa 12180
ggaggtggat tttgattttg attttatgga ggagtgtttg tggattttta atgagtttat 12240
tagtgttaag atggaggata gaggttggtt ggtttggtag gtgaggtgtg tgggtgttgg 12300
ggttgggttt gggtagaggt agagttgagg gtttggtgag agattttta tttggagtgg 12360
tttttagttg gtttggaggt atggggtgga ggtagttttt tagtgttggt ttttgagttt 12420
gttgaaattt taggagttta ggagtatttg ggtagttttg ttgtttttgt tgttttttat 12480
ttatttagag gttattggtt tgtggttatt agttaggttt ttgtggttat gtgtatatgg 12540
tttgttttta ttgattggta gggggatatt ggtttattta tttattttag gggaatttat 12600
ttttggtata ttttttagt ttttttttt ttgttttttt ttttttttt ttttttttt 12660
ttttttttt ttttttttt ttattttgt ttttttttt ttagtttttt tgttttttt 12720
ttttgttttt tttttatttt ttttggtttt ttttttttt ttttttttt ttttattttt 12780
agtgtggtga gtttttgttt ttttgggtta gttttggatt tttgggtttt ttggttttgt 12840
gtttttaaat tggttattag ggggagttta ttttatatag attgagttta agtgtttagt 12900
ttgtagtagt tggtttggtt taaggtggtt ttgttttttt agtgtgtttt tattttgtag 12960
ttttggggtt tttaagttgg ggttttgggt gtttaggaga ggtagttttg ggtatttgta 13020
gaatgtgtga tatttgttta ttttttgat tgtatttata taaagtttag ggattgttgg 13080
ttttgagttt ttagttgttg tgtttggggg ttggattgtt ttatttttta tttgttgggg 13140
gtagtttgag gttggggtgt gttggtgttg gtagtgaggg tagatttgtg ttgatggatg 13200
gatatgtgtt ttagtttttt aaggaagaga agagtgagga gaaggggttt ttgggtttga 13260
ttattttgtt ttttgggtag aagaggagga ttttttattt ttttaagtaa ggttaggagg 13320
taaggtttgt agaggttagg ttagtaggag ttttttttt ttttttttt aaagatgagg 13380
ttggtttggt ttggtggttt atgtttgtaa ttttagtatt ttgggaggtt aaggtgggtg 13440
gattatttga ggttaggagt ttgagattag tttggttagt atggtaaaat tttatttgta 13500
ttaaaaatat aaaaattagt tgggtatggt gatgggtgtt tgtaatatta gttattagag 13560
aggttgaggt aggagaattg tttgaattta ggaggtagag gttatagtga gttaagattg 13620
tattattgta ttttagtttg ggtgatagag taagattttg ttttaaaaaa aaaaaattat 13680
aaaaaaaggg ggttttgttt tgttatttag gttggttttt taatttttga ttttaagtga 13740
ttttttgtt ttggtttttt agtgttggga ttataggtgt gagttattgt ttttagtttt 13800
attttttgt tttttgtttg tttgtttttt ggttgttttt ttttttttt tttttttga 13860
gatggagttt tgttttgtta tttaggttgg agtgtagtgg tatgattttt gtttattgta 13920
atttttgttt tttgggttta agtgattttt ttgttttagt tttttgagta tttgggatta 13980
taggtgttta ttattatatt aggttaattt ttgtattttt agtagagatg gggttttatt 14040
atgttggtta gattggtttt gtgatttgtt tattttggtt ttttaaaatg ttgggattat 14100
aagtatgagg tattgtgttt agaggtgggt agattatgag gttaggagtt tgagattagt 14160
ttggttaata tggtgaaatt ttgtttttat taaagatata aaatattagt tgggtgtggt 14220
ggtatgtatt tgtaatttta gttatttggg aggttgaggt aggagaattg tttgaatttg 14280
ggaggtggag gatttaatga gttaagattg tgttattgtg ttttagtttg ggtgataagg 14340
taagattttg ttttaaaaag ataaatatat aaaatgagtt atttatttat aaatttttga 14400
tttttttggg atattgtttt tgtaaatttg tgatgttttt tgtaaagtat tagtgatttt 14460
tttatttgtt tttttattta agttttatta taaatttgat gtttaatatt tgttttaatt 14520
ttagtagaat ttttgttgtt ttgattgggg ttttgtttta attgatgttt tattttttt 14580
tagtgtttta aattttgttt ggtttagata tgttatgata agttagtatt agtttatttt 14640
ggggtaaaaa aatttgaaag ttatgtaagt tttgttataa tgtatgtttt ttatgagttt 14700
tttgaagata ttttattttt gatgttgttt gagttttaga gggagttggt tttgtttttt 14760
tttgttagat tgttatggtg ttggggtgag ggatagagta ggttagtagt tttagggtga 14820

```
ttttttttag agtgtggtag atggtttgtt gtgttgtgtg tggtttgggt ttgggttggt   14880
tgtttagttt tgagttttag tgttttttgt agtttggaga tagtagtgtt tgttaggttt   14940
ttgtgtagat gtggtgaggt agtgtaggat aagtagagag tttggttttg ggttagtag   15000
ttaggagagt tttagttgtt ttagttttag agttttttta gaggaggggt tttgaggaag   15060
attaggtgta gtgggattgg attggagtgt tagtgtaatt ggggtagttt tgtttatttg   15120
tgttttatta gggttgagga tatagtttgg gtattgggag atattgggag tgggggagtt   15180
tggagttata tggtattgag gattattgtt ttttgttgtt ttttttagt gtgtttagta   15240
gtttagaagg tagttaggga ggtggtgttt ttggtttttg agttttagtt attttttttt   15300
aagtttgttt ggggttgttt gttttaggtg tttttgttgt gttgttaggt tttagtattt   15360
gtggtgatgt aggtggagtt tttgaggagg ggttttgtgg tgtttttggt ttggtttttg   15420
atggtgtagg aggtgtgtta tttgtgggtt tagtaggtgt agagggtatt ggtgagtttg   15480
ttgtaggttt ttgttaggtt ggtagagaag ttgtttttttg tttatatttt tgtttttggt   15540
gagaagagga ggattgttta tatttttaat ttttgtttgg ttgtaggtga gttttggttt   15600
agggggtttg tgttgggggg ttgtgggtat gtgggtagga gaggtggtat tttgaggttt   15660
ttgagtttat ttgaattata tggtgtttgt ttatgggatg attttgggtt ttttttttgg   15720
ggtttttagt ttgtttgttt gtgaaatggg gattgttgag gggtgtagga ggtttggttt   15780
agtatttttt ttttttgatt tagttttggg gttattagga gtttaatagt ttgttgggga   15840
ttgggttgaa ttagggtttg agtttgtggt tttagggaat ggggagttat gttggggtat   15900
ttggtattgg gtggggagta ggtttttgta ggtgtggttt ttttttggtg tgtgagtggg   15960
gggtgtggtt tttgatggaa atgagtgttg ttttttttagg agtggggttg tattttttttt   16020
attttgaggg gtatttttttt tttagtaggg ttttgtagaa agttgggtta ggttttagtg   16080
gtgggtggat aggatttttt atgggggggtt gttttgtttt ttgtgttttt atattagtgg   16140
tgtttgtaga gtgagaaagt atttttggag gtaggttata gatgagggtt ttttagaaag   16200
tttttaaaaa atgtaggttt tggtttggag gttgggaaag gtttgtgtgt ttggtaagtt   16260
ttttgggagg gtatggttgt tgtttgagga ttattttgag tagtttgttg gtttggagtt   16320
tttgtttttt tggtgttggt tggagagttt tagggttagt tttggtgatt tttttgtaga   16380
ttttatggat tttatggttt tttttaggga gagtttttta ggttattttg atttttttttt   16440
tttagagttt ttttatggtt gagattttag ttttagggag gggaaattat tgagttgggt   16500
ttattttagg attttatatg tttttttgttt gtgtttttgt gtttgggttg ttttgttgtt   16560
gatttgtggt gggtgttgag ttgttgtagt gtttttgggg tttgttgttt gaagggagtt   16620
tggtgtttta agtgttggta gtgttgtagg gtgggtttat ttggtttgga ggatttttgt   16680
ggttgtttgg gtttgttttt agtgtgtagt tgagggagtt tggaggtttt ttgtttgttt   16740
ttgaagagta aatttttttat tgtaagaggt gtgtgttagt ttggtgggta gtttgttttt   16800
ttgttgggtg gtttgtggta gtatgagtag atttattttg tttttttttgt atttttttagg   16860
gttagatggt attttgaggg atgttgtttt gagattaggg ttagatgttt ttgtttatag   16920
tttttttttt tgggttggtg ttgagttttt agtttagaag ttttttttgtt atatagatgt   16980
atatgtttag gttgtagttg tttgtgtggt tagtttttttg aagtagtttt tgtaggtttg   17040
gttttttttt tttggtttgt gattttatta aagggtaagg tagaatatag aggttgtagg   17100
gtagggtaaa gttaatgagt aggtgggggt atttgagatt ttttggtttt ttgtttggga   17160
gttagggttt tttttttaga tattgatgtt ttgggtttgg ggttagaata tgtggtagag   17220
gtttggatta ttttattggt gatgtgtttt tagtggtttt tgtagtttga tttgtgtttt   17280
ttgttttgat ggatttgttt gaaggtagtt agagtttgat gttattgtgt agttttagtt   17340
ttttattttg gtttggtttt ttgtatgtgg agatgtatgt gttgttggtg ttgagtggtt   17400
gtggttgttt ggtgttttgt tatgtggtga tgggtagtta tttgttttgt gtttatattt   17460
tttgtgtttt gttagttttt ataggtgtta agaggatttt tgtggttagt ggtagttagt   17520
tttttaatgg ttttgagtta ggtggttagt agttgaaaat atgtatattg ttggggatgg   17580
tgtttaagat tattattatt attattttta agtgaattgt ttatagttta tttttatagg   17640
tagtttttttt tattttggtt aggattatta tttttaagat tatgttttga ggtttggtta   17700
tatgggattt gtggagggtt agttttgttt ttgttttgtg ggtttttgtg ttttatattg   17760
tagtatagtt tttatttttt ttttttatgt tttggtgttt tttttttttt tttgagatgg   17820
agttttgttt tgttgtttag gttggagtgt agtggtatga ttttggttta ttgtaagttt   17880
tgttttttgg gtttatgtta ttttttggtt ttagtttttt gagtagttgg gattataggt   17940
gtttgttatt atgtttggtt gtttttgtat ttttagtaga aatggggttt tattatgtta   18000
gttaggatgg ttttgatttt ttgattttgt gatttatttg ttttggtttt ttaaagtgtt   18060
gggattatag gtgtgagtta ttatgtttga ttgttttggt ggtgtttttt tgttttgttt   18120
tgttttgttt ttttggttgg ttggtgtttt ttttttttgt ttagagatag ggtttttgtt   18180
gtttaggtta gagtgtaggg gtgtaattat ggtttattgt aattttgatt ttttgggttt   18240
aagtgatttt tttattttag tttttttgagt agttgggatt ataagttggt gttattatat   18300
tttagttaat aattttgttt tttattttgt agagataggg ttttgttatg ttgtttaggt   18360
tgattttgaa tttttgtttt taagtgattt ttttgtttta gttttttaaa gtgttgggat   18420
tataggtgtg agttattatg tttggttttg ttttgttttt aattaataga ttttatgttt   18480
```

```
tttgagtaat tttaggtttt taggaaaatt gagtagaaag tatagtaagt ttttttgtgt   18540
ttttatggtt ttagtttttt atgattttag ttttgttgtt tttagttttt tttgttgttt   18600
ttgttttgtt ttggtgtgtt tgtttttatt gtagttaggt tgatttgttt tagtgtattt   18660
tatttttgtt tttagttatg ggtatggttt tattgtgttt gtgtttttttg ttggaggttt   18720
tgtggaggtt tttatttatg gtagttttttg tttttttttt agagtttaaa gaaatttatt   18780

atttttaaag agtttggggg taaagttttt attgttattt gttagtgtta ttttaatttg   18840
tttattgagg agtgttttaa gttttgtatt tttaattagg aggttataga gaaggtgagg   18900
ttttggagtt tttatttggt tggttgggtg gggattaggg gtttagggtg gtttttagat   18960
attttttggt tttttagttg tagttgtgat ttggagttgt ttgggagaga tgtaagtttt   19020
tgattttatg gtgattttat tgttggggtt ttttggtatt aggtaggtat ggttttagtt   19080
gggtattgtt tgttttttag gtattgaatg aggagaaggt ggtttatgat tgtagtttta   19140
gtaagaatat ttatttgaat gtggttgtga atatttttaa gaagtttagg ggtttggttt   19200
ttagtgttgt gtttggtttt agtagtgagt tttggtgggt gggtggggga ggtttagggt   19260
tgtttttggg gtggtagttg ggttgggatt tttgttggga tttttatagt tttgagttta   19320
gttatagttt tagataaaag gaaagtgtgg tgttgtgggg gggttgtttg ttgtttggtt   19380
tttaggtttt tgtgtaggat ggatttggtt ttggtgtttt tgttgttagt tgtttttttt   19440
tgttgagtgg ggtttttaag ttgagttgag gggttgtttg ttaatggagt tgtggtagtt   19500
tttttaaagt aggaggttta gggttgggtg agttttatgt attaagtttt ggggaggaag   19560
aaagtggaga tttgggtgtt ttattgtagt tttttaggt tgtattaagt tattattggt   19620
ttttgttttt gtttttgttt gtattagtat ttggaggggt tttgggttgg tgttgtaggt   19680
tgggtggttt atgaaggttt aaagtttttg aaagttatta gattttttgt gttgtttttt   19740
ttgttttttt tttttgttt gaggggaggg gatgaagatt ttgggtgtgg ggtttttgtg   19800
tagttttggg gtttgtagag ttggtggttg ttttgtgttt ttggtttatt gtatgtttgt   19860
tttttttgtt tgggttaggt tggaatttgg ggttagtggg gtgttgtttt agaaaggggg   19920
agttgaggta gaggtttgag gagggtaggg tgggtgggag agtaggtggg gtaggggttt   19980
gagttagtta ttttatattt tttttttttt tttttttttt ttttagaaat tagtggttgt   20040
agggttgtgt tttatgaggt ggtgttgggg ggtaggttgg ttgttaagat tagtttttttg   20100
tttagttgtt taagtagttt ttgggtggag gatttgaaag gtaaggtttt gtttttgttt   20160
tgttatatgg tgttttgtgg gtttttgttg gtttagtttt tgttgtgtta tatggtgttt   20220
tgtgggtttt tgttggttta gttgtttttt ttggtggtgt ttattgtttt gtgggttatt   20280
ttaggtaggg gttgattatt gtgtttgttt ataggggttg ttttgtatag ttgttttagg   20340
gagtatttgt ttatttagga ttagtttaag gagaatggtt attttttttt gtatttagag   20400
tggtttgggg gtgtaattat ttttatagtt gaggagaaga ggtttaagga ttgtgagttg   20460
ttggtttgtg atggtttagg tgggtaggtg tgggatgtgg gtaaggttgg gtatgtgggt   20520
tttagtgtga gttttagttt ttatttgggt ttttatttat agttttttgt aggatttgtt   20580
gttgttgtgg tattgagtat tttgtgtttt ttttaggttg ttgtatttgg gatgaggagt   20640
gttattatta ttggggatgg ttgtgttgga attggggtaa ggttttattt gggttttttt   20700
ttttttttatt tatggttttt ggaatttggg tttttttttg aggtttttag aattggggta   20760
aggttttatt taggtttttt attttattta gggtttttgg gttgtattgt ttttggtaga   20820
gttggggtta gtttttggtt ttattttttt tttatttgtt ttttatgtaa aagtaagata   20880
agagtttttt tatgataagg tttaaatgtt attatttgaa aggttttgtg tttttgtttt   20940
tttgtgtttt tggtttatgg aaatgtggag gttgttggtt ttaggagtgg aggggtttgt   21000
tttatttttg gtttggtggt tttatttaat tttttaggta attatgtagt tatgtttagg   21060
gtatatggtt agttttgtat agttttgtgg tggtttttaa agttattgtt ttagggatat   21120
ttggagggtg ttgtttgggg tagtgttgtt aagaggtttt tgtagagggt attggttttt   21180
ttggatttag gtttgggtgt tttggttgtt atatgattgt tggggtttgg tttgttttta   21240
gttttattaa gaggttgagg ttttttgttt ttagttttta ttttttagta gggtttggta   21300
gatgtaggtg gttagtgttt tgggggttgt ggagtgggtt tggttgggtt ttgattgtgt   21360
gtttgtgttt tttgtagtgg ttggaggttg ggagatttag tatatgtgtt gtttggttgt   21420
tgttggtttt gttggttgtt aagttgtaaa ggtgagtttt ggtgttttag tttttttttgt   21480
ttgtttttaa tagatttttg ttttttttatt gttggtgtgg ggttttggga tatagtatag   21540
ttttgggttt agggtttggg tttttgggta gtttttttttg tgttatttag agaatgtagt   21600
atttgtaggt tttaggtttt gtttttggtt atttttttatt ttgggaagtt gttttttttg   21660
tgtttttttg gtaagttttt tttttatttt gtttattagg gttttaatta gtgaatgggt   21720
gtgatggatg gataggatgg ttttgaaggg tttagagatg tgttgttgtt gttattttttg   21780
tttttattgt attttgaagt tgtttttatt tgtttttttt attttagta atatgtgtag   21840
gatggttgga aggagtgttt tgagggtttt gtgaagattt ttgagaaaga gtttttagga   21900
gatatttatt tggggattta tgttttggat tgtgagatgg tgaggttgtt ttttgtttag   21960
tgttgggttt aggattgggg aggatttggt ttggtgtggt tgttttattt ggttttattt   22020
gggtttggtt tattggttag tgtttagggt tttttttgta gtattggtg              22069
```

<210> 299
<211> 9724
<212> DNA
<213> Artificial Sequence

<220>
<223> chemically treated genomic DNA (Homo sapiens)

<400> 299

```
tttttggtg tttaaggtag ggttagtttt atttttagga gatggggggt tttttatta      60
ttggggtttt tgtgtttttta ggtgtatgga ggagggtagt tttatggtgt tggagttttt     120
ttaatatggt ttgtttaggt agtattttgg tatggttttt tggggttggg gttttgggtt     180
aggtttgggt tttatggtat ttgaggaagg tgttttttag gaaggtggtt agttttattt     240
ggttgttttg tggaaggagg gaggtatggg ggatggaggg gtgtggtttg ttttgtgtgt     300
ttttttgtt aggttgtgtt gtattgttta gtgtattttg tggaggaagt tatagtgtat     360
gagttttgag gtgaggtttt gggtgttgtt tgttgaggta atggtgtaag gtgaggtatg     420
aggttgttgt ttatttgttg tttggggtta ttgtttgttg tttaagtttt ttgtttagtt     480
tatttgggag taggttgtag gttagttgtt ggtgtagttg gttttttagt attagaagta     540
gagtgagttg gggaggtggt ggggtgtggt tggttggggg tttagggttt tgttatgttt     600
ttgtttttt tatggtagtg ttgttttttt aggttttgtt tatatgttag tgtgttttgt     660
ttttgtttt ttttaggttg tattgtattt ggatgatttg tagtgggggga aggttgggat     720
ttataaattt agtagtaaga ttatatgttg tggttttaaa ggggttggga agggatagta     780
aagattggaa agggggggaat ttttaagagg agtttatttt attgttagtg gtttggtaag     840
ttaaggaaag ttttgagaag agttgatatt aggggttggg tattgaaggt tgaataggag     900
tttttattgt ggagagaata atttgaataa agagggaatt gtgggtgtgg ggaaaggggag     960
gttgggtggg ataggattgt gttgaagtgt atggtgtttg tgggattagg ttttttagag    1020
tttgggtgtt agttgaggat ttttatgatt tttgttggta gttaggagta ggtagtttgt    1080
ttgttaggat atgaattttg gttgtattgt attgagagag gtttttggtt aggaggggggt    1140
gttgtaggaa aagtttgggg agatattagg atatagatgt ttgggaatta gtattaggtg    1200
ggggatggga ggggtggggg gggggtggga ggggtgtggg ggtgggggaa gattgttggt    1260
agggtggaag agtttgggtg agttttggag gaaggatgtt tggagtaggt ttagggtagg    1320
tagtaggatg taggggaagg atttgagggg atagggagtt ttttggaaaa ggagagtgtt    1380
tagtttgttt tataattttt atgattttta taaggggggtt agtttggatt tggtttttgg    1440
tagttaggag gagagttggg ttgtggggat gtaggtttta agagaaggag gggtattttt    1500
tatttggttg ttagaaggtt tgagggatgg tattgagtag taggagaggg gatttatttt    1560
tttggtttta gagttaaagg gttttggtgt tggggttttg gtttttttgga ttttttttgg    1620
gggtggggtt agtatatggg gtagttttag gggttgagtg gttgtaaagt ttattagtgg    1680
gtagatttta tttttgggga tataataggg tggttggggg tttaggtttg atagttgttt    1740
ggtttttaat tttatattag tttttatttg atattttttta ggaatttgtt tagttttatg    1800
aaggagattt ttgagtattt ggtatggaag tgggaggtat atgaggggtt ggttggggtg    1860
aggggagggg aggtgtgggg aggggatgtg tggggagggg aggtttttgg gtgttgattg    1920
tttattgtta ttgtagtggg ggtttgtggg gttggggaag ttttgttaag attgtgtgta    1980
ggtttatggt tttggttttt tttttatta tatttttagg tatgaggttt gtggttataa    2040
gagtattaag gtggtgtgtt taggttgtgg gttttgtagg gtttttttta tgttttaatt    2100
tttattttt tagttatttt ttgggttggt ttgttgttta tattggtgtt ggatgaagta    2160
gtgggttgtt aggagtttta gtgagtgtat tttgaagagt atgtggtgga agaggaggtt    2220
gtgggtagag ggttggtggg tagggttttg ggttaggtag taaaggatga atttttgggg    2280
tatagggagg agaggttggt ggtgtgtaag ggtttttttgt tttggttttg tagtttgagg    2340
agaggtggtt tttgagtttt ttatgttttt ttttagggat atataggata tgtagggatg    2400
tttataggga ggagttttag tagtagtagt taggttagga tttggttttt tgagtttgag    2460
gatagttggg ttttgggatt ggagtattat gttttgtttt ttttgatttt taaaaaattg    2520
tggggagaag ttatgatttt aaagttatat ttgtaagttt tttatttatg gtttttttgat    2580
tggtttagtt tgggtttaat attggagatg attttgatgg gtttttattt tgtttatatt    2640
tgtgtttagt ttggttttag tttgttatta gttttgatta ggtattttta tttttttgagg    2700
ttatggagtt aagtttagat tttgagtaga gggggttgtg gatgtagttt ttattttata    2760
ttttttttg tttgtttggt atttattatg tttttttata agttggagtt ttattatttt    2820
ttggaagttt aaagttaagg atttggagtg aagtagattt aggtgtagat gttggtttta    2880
ttatttattt taaagttttg ggtaaaggat ttattttttt tgaattttgg tttttttata    2940
aaatgggtat ggtgatggta gttatagagg gagggttttt tttgtttttt tgtttttaga    3000
```

atgtgttgat tttttagggt ttttaggttt ggaattagta tgtgttttta ttgaggtttt 3060
ttttgggagg ggtgtagtta gttggtatat tttttaattg tataaattgt gatgtttttt 3120
taggtttgtt tggagtattt tgttagtttt agttgagtat ggtgagttag ttggggaggg 3180
gtagggtaag ttgtttattt gtatgttggg gttatttagt gagtgtttgg tgtgagtaat 3240
ggtttttttt gtgatttggg tgtttttatt ggtttggatt tttagtatag ttatttgggg 3300
tatagagtta ggttaggtat ggggaaggga ttatataggt gagttaggta gggtatagtt 3360
aggaggaggg tagagtataa ggtggagggt gggggtagta tttttagtgt atatatttta 3420
aaggagaaga tgtttatagt ggttttattg gttattttttg aatagaagag agtataggat 3480
atgtaggaga gaagtgtagg gtaggtatgg ggtatttgga tttagtatta tttattttta 3540
tattttgggg ggaagaagtg taggttttga agtttttttt gtttagtgtg gatggggttt 3600
tggagattat ttggaagtgt tgtgggaggg tgtagagttg agtgggtggg gatttttttta 3660
ggattttgtt tttttaaagt ttgtatttat tattggagaa gatttggtgt tatattgtta 3720
aggggtggga gaaagagtgg agttagttgt tggggtatta gaattttttt gtttttggtt 3780
ttaggtattt ttttttgttt tgtttttttta tttagttttg ttttgttagt attggagttg 3840
attttgatga ggttgttgtg ttgaatgaag atggtgttgt tggttaggtt tttgtggatg 3900
attggggggt tgtaggtgtg taggaagttg tagatgttgg ggagggggaga gtaggaggag 3960

ttggttagga ggttttggag agatgggggt ttggtggtgt tgtgtttagg ttagtttagg 4020
tttttatttg agtgtagata ggatttgtgt gtattagtgt ttttaggttt ggtggtggat 4080
gtatgatttt gttggttggg tgggtgtagg agaggtggtt gggtttgtgg agtttgtttt 4140
gtatttttgt ttagtttttg tttgaggttg ttgggtattt ttttattatt ttagtttttg 4200
aggttgtttt aattttgttt tgtttttgtg gttgttttag tttgtttttt atatttgggt 4260
gtttatggtt ttgtggtttt ttttggtttt tttgaggaat tgtttgaggt tgtttgatga 4320
tatgtatttt gtgatgaaga tgatttgtat ggtgtgagtt taggattttt attagttgtg 4380
ggtttgtttt tatgtttgtt ttatttagtt gtggtttttg tttgtttggg gttttgtttg 4440
tgtttatttt tgtgtaggtt ttagaggtat ttagttagta tttgtgtaat tttatgatgt 4500
ttgggtggtt tattagtatt agttgtttga atatggtttg gatttttttt tggggaggga 4560
gggtggttgt tgggtggtta gtaggtggtt atttaagtag gatgaggagg gggtagtggt 4620
tttattttgt gtgttgtgaa ggtttttttg tttttgaagt ggagtttgtt ttatattatt 4680
tttatttttt tttttgtgtt tatggttagg aaggtgtttt gaagttttgg tatgtttttt 4740
tggtttattt gggggtgaat aaagggttat gtgtgttttg gtgtgtgtta gggttgtggg 4800
tgaggatttg gtttttgttt atattttttt agtgggttta agtgtgggtt gtaggttttg 4860
agttattttg tgggaaggtg gggtttggag ggtagttgtt tttaggagtt aagggttttt 4920
atttaagggt tgggttaagg ggatttggag taggttttag ggggttgagg gggattttta 4980
ggaagttagt ggttgagttt agaggtatgg ggaggtggtt ttgggaggag attggttttt 5040
agggagtttt agggtgagtg ttaggttaaa ggggtttagg ggtggttgat ttgtgggttg 5100
tgaggggttg tggagaggtt tttagttgtt gtgttttttt agtgttttta tgttttgtgt 5160
agtttttagg ttttttttgt tttgggaggg tggtgttatt ttgtgtttta atttttgtgt 5220
ttatttgttt ttgttgtttt tgttagtgat tatatgggtt tttttttagg atgttgtttt 5280
tgtttttgtt tttgtttttt tgtttttgtt tttgttttttg ggtttgtttt ggtgttggtt 5340
ttggggttgt tatggtttgg ttagtttagg ttattgtttt ttgtgtgatt tgttgttggt 5400
gtagtttttt ttggtttgtt ttggttttgt gtttagtagt ttagtttaga gttgttgtgg 5460
tagtttagag ttttagtttt ggttttggga attgggaggt gtgggtgtgt ggtggatggg 5520
tggggtttgg gggtggagtt ggtgtgtatt ttttgagttg tttgtgtttg ttggagttag 5580
agtagagttt ttttgagagt tgttgagttt gggttgtgtt taggttttgt ttttttggag 5640
gttttgtttt gtgaagtttt gttttagttt ttgttttttt tttgtttttt aggtttgttt 5700
tgtgaagttt ttttttaggt tttgtttttt ttttgttttt taagtttttt ttgtgaagtt 5760
ttgttttagg atttgtttta tttttgtgaa tttttgtttt gttttagttt ttgtttttgt 5820
ttgtttttag ggtttttttgt gaagttttat tttaggtttt gttttatttt tttgaagttt 5880
tgtttttatg aggttttgtt ttgtgtagag gaattgtttt taaatttttg tttttttgga 5940
ggtttgtttt tgtggatatt tggagtttgg agtgtttatt ttttttgtgg ttgttttttt 6000
aggatttggg ggaagatttg gggtatttgg gggtttttaa gtttatggta tttttttagtt 6060
gtagaagatt ttgtgggttt gaaagtggga tttgtttttt tggttatgta tttatttttt 6120
ggttgatttt taagggatag gttgaagggg tattgagggt gggggaaagg tttattagta 6180
ggttgattta ggttgatttt gattggtgat tatgaatttt tttttggttt gtttattagt 6240
gttttttttt gtttataagt ttattttttt atttttttagg atggtgattt tttttttttt 6300
ttttaaatgt gtattttttt tttgtggttg gtttttatgg gatagtggaa ttatgagggg 6360
ggagtttatt tggtttatat gatttttttt tttggaggtt gttttaagta tattttttag 6420
ttatgttgat tattggggtt gtttttgatt tttatttaaa atttttttta gtttatagtt 6480
tttatggttt ttttgtggtt tttttttttt tttatggtgg gtttttattt tttatttttt 6540
tttttggag tttaaattgg aattttgttt tttttttttg tgtagtttta tgtattggtt 6600

```
ttttgggatg tttttttttt tttttggtag aggtgtattt ggttttgttg tgttttttgt    6660
ggtttttttt ttttgattag ttttggttat taggtatttt gtgtgtggtt tttattaatg    6720
ttaggtgatt ttgttgaggt tttttgtagt aaaggtttgg ggtgtttaaa tttttttattt    6780
tgaaattggt tttgattagt ttttgggaga tattttttaa gtttttggaa tgttttgttt    6840
gatttgaatg tttgtgttta ttttggttat attagatttt atgttaagtt tgtttaattt    6900
gtagtttaga gggtgtattt ggttaaggat agttttgaat atggtttaat ataaatttat    6960
aaattttttt attgagattt tttttaaagt ttattagttg ttgttagtgt taagtgtatt    7020
ttatttaagg gtattgttta ggataatttt tttaatgtgt gttaggaaag tttatagatt    7080
ggatattttt ggtttgtgtt aataatatgg tttatggggg gatttgggt tatgtggtat    7140
tagtttgatt tttgtagggg ttggaggtta aagttagtta tttgggaggt tagttatgtt    7200
tgtgtgatta atttaataaa aattttggat attgaggtat ggagggtttt tttggttgtt    7260
gatatgttgt gtttattgtt atattttagt gttgggaggt tgagtattgt ttttatgttt    7320
gtagtgggtg gggatatttg tagatttgtg ttggtttgtt ttgggttttg ttttgtgagt    7380
ttttgttatt gttgatttta atgtatttat tgttgtaata aatagtattt tttttgagtt    7440
ttgtgagttt ttttagtgaa ttattgaatt tgagggtggt tttggggagt ttttttagtg    7500
gttttttttt atttggtatg agggtttaaa tatgaaggtt tagtaattgg ttttattatt    7560
ttattttatg ttagttgttt tttttagttg agtttatttt aagtatatgg ttttgtgtta    7620
ttggatatga taggtatttt tttttagggt ttttgttttg gttatatttt tgtttggaat    7680
gtttttttta aatattttt gaggttattt tttgagaggt ttgttttgat tatgaatttt    7740
gatattatag tttttatatt tttttgtttg tttgatttga tttttttttgg agtatttatt    7800
ttatttgatt tgtatttggg tttttttttat tttttttttt tattgtttgt gtttttgttg    7860
tgatttgagt ttttgtaggg taggagtgtt tgtttgttgt ttttgttttt tttttggtgt    7920
gaggatggta tttggtatat agtaggtttt tagtaaatat ttgttgaatg gatttgtggg    7980
tgggtagggg tgtgttttgg tgggtgtggt ttagagaagt ttttaaggag gtaagttttg    8040
agtagaagaa ttttaatgaa tttttttttt tttttttgtt tttataatta gatatatatg    8100
ttttgtggta ggttatatag tgttttttaa aagtttatat tttgattttt agtatttagg    8160
agtatagttt tatttgaaaa aaaaaaaatg gtttttgtag atgtaaataa gtttaggatt    8220
ttgagatgtt ggggtgggtt ttaaatttaa tgtttgatgt ttttattaga gaaagaaaag    8280
ggatatttgg ggtgtagaga aataaggtag atagttatat gaatatggag gttagtgttg    8340
gagggatgta tttataggtt aaggtgtgtt tgggatttga ggatatgagt tgttttagta    8400
taataaaata tataaaataa gaatagttgt attgatatag attttagata tgattatata    8460
tgaatattat taattattag tttgtagtaa ttatttttta ttttaatatt ataataattt    8520
ttattttata attatagttt aggaaaaatt aggttatata gagataggag ttaaggggat    8580
atagtgagga gtaattagaa gataagagtg tgagtttttt gttatgttta gatagggtta    8640
ttagagggtt ttttggttta gtggtaatgt tagtgtttgg gaagatgttt gttgttaagt    8700
ggattttggt ttagtggtag gtgtagtgtt aaggaaaaat atttgttatt tagtagattg    8760
ggaaagggag ttttttttgt tttggtggag tttaaagaag attttatttt tttattttt    8820
gtggagggtt tgatattagt taggtttgtt tgtagttatt tggaggttta attgtttttt    8880
tgtgatgttg tgttttagtg gttatatttt tagtttgttt ttatgtttta ttttgtatat    8940
ttggttttgt tttttagata gtagtagtaa attagtgaaa gtattaaaag tttttgatat    9000
gtagaaataa tggtgtaggt tgttttttttt tttgtttttt tttttttttt gttttggttg    9060
ttaggtaggg aagggttttt tgtttagtgg atatatgatt tatgtggttt tatttattat    9120
tggagatggt ttatttttttt tattttgttt ttttgttttg tatttaataa atattagtgt    9180
agtttggtat ttggggttat tattggtttt tgtgatttgg tggtagtggt tttttgggtt    9240
tagttgtttt tttttttatt tttgttttgt gtttttattt ttatattttt ttgtttttgt    9300
atatggggag agatttattg tttttgtggg gttggatttt atttgggatt gttagggtta    9360
tgagtggtga ggagagagtt ttgggatgga tttttttttg gagtttttgg aagtattagt    9420
tttattgata ttttgatttt gtatttttag tttttagtat agtgagtgaa tttgtttgtg    9480
ttgttttatg ttatttttttt atttttggtg ttgttttagt atgatttttg tggtaagaat    9540
agtaatgtga taatagttat gtgtttgtgt gtagggatgt gttttagtat ttatttttat    9600
gtatagagtg ggttgtttta ttgtttgttt atgtatagaa gtggtggttg ttgtggtata    9660
tttttttttgg ttttatttag gttgtgtttt agggtggggg tgtatttttt tttttgtttg    9720
gttt                                                                 9724
```

<210> 300
<211> 9724
<212> DNA
<213> Artificial Sequence

<220>
<223> chemically treated genomic DNA (Homo sapiens)

<400> 300

aggttgagta gaagggaggg tatgtttttg ttttggggta tagtttgagt gaggttaggg 60
agggtgtgtt gtagtggtta ttatttttgt gtgtgggtag gtagtaggat gatttatttt 120
gtgtatggga ataaatgttg ggatgtattt ttgtatgtga atatatagtt gttgttatgt 180
tattgttttt attatggggg ttatgttgga gtagtattag agatggagga gtagtgtaaa 240
atagtataaa tagatttgtt tattgtgttg gaggttggaa gtgtaaaatt aaggtattgg 300
tagggttgat gtttttggag gttttgaggg agaatttatt ttaggatttt tttttatta 360
tttatggttt tggtagtttt aagtagggtt tagttttata gggatggtgg gtttttttt 420
gtgtgtagag atgagagagt gtagaaataa agatataaga tagagataaa agaaaaggta 480
gttgggtttg ggggattatt attattaagt tgtggagatt ggtagtggtt ttgaatgtta 540
ggttgtattg atatttattg gatataagat aaaggggtag gataaggaga gtgagttatt 600
tttaatgata ggtaaggtta tgtgggttat gtgtttattg gatagggggt tttttttgt 660
ttggtagttg aggtagagag agagaggaga tagagagaaa gatagtttat gttattattt 720
ttgtatatta gagattttta gtatttttat taatttgtta ttgttattta gaaggtagag 780
ttaggtgtat aggatggaat atgaaggtgg attaggagtg tgattgttga agtatagtat 840
tatagggaga tggttaggtt tttggataat tgtaggtgag tttgattaat gttaggtttt 900
ttataagagg tggaggagta gagttttttt taaattttat tggggtaagg gagatttttt 960
tttttggttt gttaagtagt aggtgttttt ttttgatatt gtgtttattg ttagattaag 1020
gtttatttgg taataggtat ttttttagat gttggtgtta ttgttagatt aaggagtttt 1080
ttggtggttt tgtttgggta taatagaagg tttgtatttt tgttttttgg ttattttta 1140
ttatgttttt ttagttttta tttttgtatg gtttggtttt ttttaggtta tgattataga 1200
gtgaggatta ttataatatt ggaataaaga gtaattgtta taaattaatg attaatgata 1260
tttatatata attatattta agatttatat tagtataatt attttattt tatatatttt 1320
attatattgg aatagtttgt gtttttgagt tttaggtatg ttttgatttg tgggtatatt 1380
tttttaatat tggtttttat gtttatgtga ttgtttattt tattttttg tattttaagt 1440

attttttttt tttttttgat aaggatatta aatattggat ttagggttta ttttagtatt 1500
ttaagatttt aaatttattt atatttgtaa agattatttt ttttttttta aataaggttg 1560
tgtttttagg tattggggat taggatgtag atttttgggg gatattatgt gatttattat 1620
agaatatgtg tgtttggtta tgggggtgga gaaaggggag gaaatttatt gagatttttt 1680
tgtttaaggt ttattttttt aggggttttt ttggattata tttgttaaaa tgtatttttg 1740
tttatttata gatttattta ataagtgttt gttgagggtt tgttgtgtgt tgggtattgt 1800
ttttgtatta gagaaagagt agggataatg agtagatatt tttgttttgt gggagtttga 1860
attgtaatag ggatatagat agtgaagaag agagatgaga aaaatttaga tgtaagttag 1920
gtggggtgaa tattttggga aaagttaagt taagtaggtg aaagggtgtg gggattgtga 1980
tgttaggatt tgtggttagg ataggttttt taggaagtga ttttagagga tgtttgggaa 2040
gggtgttttg ggtaaaggta taattagggt agagattttg aggggaagtg tttgttatgt 2100
ttgatggtat agggttgtgt atttggggtg ggtttagttg aagaaggtag ttgatgtgag 2160
gtagggtaat gggattaatt attgggtttt tatgtttagg tttttatatt aagtgagaag 2220
ggattattgg gggggttttt taaaattatt tttaagttta atggtttatt agaaggattt 2280
atagaatttg gaaagggtat tatttattat agtaatggat gtgttaaaat tagtagtggt 2340
aaggatttat agaatagaat ttaggataga ttagtatggg tttgtaggtg tttttattta 2400
ttgtaggtat ggggatagtg tttaattttt tagtattgag gtgtgataat gagtatagta 2460
tgttagtagt tagggaagtt ttttgtattt tagtgtttag ggtttttatt gggttggtta 2520
tataggtatg gttgattttt taggtggttg attttggttt ttagtttttg tagaggttag 2580
gttgatgtta tatggtttaa ggtttttttg tagattatat tgttagtata gattaggggt 2640
gtttaatttg tgggtttttt tggtatatat tggaagaatt gttttgggta atatttttaa 2700
ataaaatata tttaatatta ataatagttg atgagtttta aaaaaaattt taataagaaa 2760
gtttatgaat ttgtgttggg ttatatttaa agttgttttt ggttgaatgt atttttttggg 2820
ttgtgggttg gataagttta gtatagaatt tggtgtggtt gaggtaaata tagatattta 2880
aattaggtag aatattttaa gggtttagag ggtgtttttt aggagttggt tagggttagt 2940
tttagggtgg aggatttgaa tattttaaat ttttattgta gagggtttta gtaggattat 3000
ttggtattgg tggaaattat atataaggtg tttggtgatt aaggttggtt agaaaagaga 3060
gattataggg gatgtagtag ggttagatgt atttttgtta aagaaagaaa gaggtgtttt 3120
aaagggttag tgtatggagt tgtgtggagg ggaggagtgg agttttaatt tgggttttag 3180
agagagaagg taaaggatga ggatttgttg tgaaggaggg aggagattat aggaaggttg 3240
tgggggttgt gggttgaagg gggttttagg tgagaattgg ggatagtttt ggtggttggt 3300
gtggttggag ggtgtgttta gagtggtttt tggagggaga ggttgtgtga gttgggtggg 3360
tttttttttt atggttttat tgttttatgg gagttagttg tggaaaggaa atgtgtgttt 3420

```
gaggagaaaa gagaaaattg ttattttgga aagtgagaag atgggtttgt gggtgggagg    3480
aggtattggt gggtgggtta gggagagatt tgtggttatt gattaaagtt agtttgggtt    3540
agtttgttgg tgggtttttt ttttgttttt ggtgtttttt tgatttgttt tttgggggtt    3600
ggttggggaa tgagtgtgtg gttaggagag tgggttttgt ttttgagttt gtggggtttt    3660
ttgtagttgg agggtgttgt gggtttgggg gtttttagat gttttgggtt tttttttaga    3720
ttttagggga gtaattgtga gagaagtggg tgttttgggt tttaggtgtt tgtagggtgg    3780
ggtttttgag ggggtgggga tttgggggtg gttttttttgt gtggggtggg gttttatagg    3840
ggtggggttt tgaggaggtg aggtggggtt tggggtgggg ttttgtggag gattttggga    3900
atgggtgggg gtggggatta aaatggggtg gggatttgtg ggggtggggt gggttttggg    3960
gtggggtttt gtgaaggagg tttggaggat gaggagggga tagggtttgg ggaggggttt    4020
tgtggagtag gtttgggaga tgaggaggaa atagggattg gggtggggtt ttgtggggtg    4080
gggtttttgg gggggtgggg tttgggtgtg gtttgggttt ggtagttttt ggggaagttt    4140
tgttttggtt ttggtaggta taggtggttt gggaggtgtg tgttggtttt gtttttgggt    4200
tttgtttgtt tgttgtgtgt ttgtgttttt taatttttag agttggggtt ggggttttag    4260
gttgttgtgg tggttttagg ttgggttgtt gggtgtgagg ttagggtggg ttgggagagg    4320
ttgtgttggt ggtggattgt gtagagggtg gtggtttggg ttggttgaat tatggtggtt    4380
ttggagttgg tgttgaggtg ggtttgggaa tgggagtggg agtgggagga tgagagtgag    4440
gatgagagtg atattttgga ggaaagtttg tgtggttgtt ggtaaaagtg atgggagtag    4500
gtgggtgtgg gggttggggt atggggtgat attgtttttt tagagtggga ggggtttgga    4560
ggttgtgtgg ggtgtggggg tgttgggaga gtgtggtggt tggaaatttt tttgtggttt    4620
tttgtggttt gtgaattagt tatttttgga ttttttttggt ttggtgtttg ttttgggatt    4680
ttttgagggt tagttttttt ttaggattat ttttttatgt ttttggattt agttgttagt    4740
tttttgggga ttttttttga ttttttgaaa tttgttttaa atttttttag tttagttttt    4800
ggataggagt ttttggtttt tgggggtagt tattttttaa gttttatttt tttgtagagt    4860
ggtttagggt ttgtagttta tgtttgggtt tattggaaag gtgtggatag ggattaaatt    4920
tttatttata attttgatat atattagggt atatataatt ttttatttat ttttaggtaa    4980
attaagggaa tatgttaggg ttttagagta ttttttttagt tatggatatg gaggaggggg    5040
tagaggtggt gtggaatgag ttttattttg gagataggaa ggtttttgtg gtgtatgagg    5100
tgagattgtt gttttttttt tattttgttt gggtgattat ttgttgatta tttagtgatt    5160
attttttttt tttaggagaa gatttagatt gtgtttgagt agttggtgtt ggtggattat    5220
ttgaatattg tgaagttgta taagtattgg ttggatattt ttgaggtttg tgtgagggtg    5280
agtatgggta aggttttggg taggtagaga ttatagttag gtggggtggg tatggggatg    5340
aatttgtagt tggtggaaat tttgagtttg tattgtgtag gttatttttta ttatagagta    5400
tgtgttatta ggtagtttta agtaattttt taaaaagatt aagaagaatt ataaggttat    5460
gaatgtttgg gtatggggag tgggttgggg tagttatggg gataggatgg ggttggggta    5520
gttttgggga ttgggatggt gagggggtgt ttggtggttt tggatagggg ttgggtgagg    5580
atgtggggtg ggttttgtag gtttagttgt tttttttgtg tttatttgat tgatggagtt    5640
gtgtgtttgt tgttaggttt ggaagtgttg gtgtatgtag attttgtttg tgtttaggtg    5700
agggtttggg ttggtttggg tgtggtgtta ttgagttttt atttttttag agtttttttga    5760
ttggtttttt ttattttttt ttttttaatg tttgtagttt tttgtatgtt tgtagttttt    5820
taattattta tgggaatttg attagtgata ttatttttat ttagtataat ggttttatta    5880
agattggttt tggtgttggt ggggtagggt tgggtggggg aatggggtag gggaaggtgt    5940
ttggagttaa gggtagagga gttttgatgt tttagtagtt aattttattt tttttttttgt    6000
tttttggtag tgtggtattg aattttttttt aatggtaagt gtggattttg gggggatggg    6060
gttttggaga ggtttttgtt tgtttagttt tgtgtttttt tatagtattt ttagatgatt    6120
tttgaagttt tatttgtgtt gagtgagagg aattttggaa tttgtatttt ttttttttag    6180
agtatggagg tgagtggtgt tgggtttggg tgttttgtgt ttattttgtg tttttttttt    6240
atgtgttttg tgtttttttt tgtttagagg tggttgatgg gattgttgtg gatatttttt    6300
tttttgggat gtgtgtgttg gaggtattgt ttttgttttt tattttgtgt tttgtttttt    6360
ttttaattgt gttttgtttg gtttatttgt gtggtttttt ttttatgttt gatttggttt    6420
tgtgttttag atggttgtat tggaaattta gattaatggg gatatttggg ttatagagga    6480
ggttattgtt tgtgttaggt atttgttgag tgattttaat atgtgggtaa gtagtttgtt    6540
ttgttttttt ttagttggtt tattgtgttt agttagggtt agtaaggtgt tttaggtaga    6600
tttgggagaa tattatagtt tgtgtagttg aggggtatat tagttgattg tattttttttt    6660
aaagaggatt ttagtgggag tatatattgg ttttaggttt gaaggtttta aggggttagt    6720
atattttaag gataagaagg taagagagat tttttttttg tggttgttat tattatgttt    6780
gttttataaa gaaattaagg tttagaggag atgagttttt tgtttaaggt tttagagtga    6840
gtggtggagt taatatttgt atttgggttt gttttatttt aagtttttga ttttaagttt    6900
ttaggaggtg atgaggtttt agtttgtggg gggatatggt gggtgttaag taggtaaaaa    6960
gaggtatggg atggaagttg tatttatagt tttttttgtt tggagtttag atttaatttt    7020
atggttttag gagatggggg tgtttggttg gggttggtgg tgagttggag ttgggttggg    7080
```

```
tatggatgtg gatagagtgg ggatttatta aggttgtttt tagtgttggg tttgagttga    7140
gttgattaaa aggttatggg taggaggttt gtaggtgtga ttttaggatt atggtttttt    7200
tttatgattt tttgggggtt gagggaagta gagtatggtg ttttaatttt agaatttagt    7260
tgtttttaag tttagaggat taggttttgg tttggttgtt gttgttggga ttttttttta    7320
tgggtatttt tgtatgtttt gtgtgttttt gaggagggat atggggaatt taggggttat    7380
ttttttttga attgtggggt tagagtagag agtttttgta tattattagt tttttttttt    7440
tgtgttttag gagtttattt tttgttgttt ggtttgggat tttgtttgtt ggtttttttgt   7500
ttatagtttt ttttttttatt gtgtgttttt tgaggtgtat ttgttgaagt ttttggtagt   7560
ttattgtttt atttagtatt agtgtgagta gtaggttggt ttggggggtg gttggggagg    7620
tggaggttgg gatgtggaag gggttttgta ggatttatgg tttgagtata ttgttttgat    7680
gtttttgtgg ttgtagattt tatgtttgag aatgtggtgg aggagaagat taaggttatg    7740
gatttgtatg tggttttggt ggagtttttt tggttttgta ggtttttgtt gtagtggtgg    7800
tgagtagtta gtatttagaa gttttttttt tttatatatt tttttttgt atttttttt      7860
tttttgtttt ggttagtttt ttatgtgttt tttattttta tgttaggtat ttggaagttt    7920
tttttatgga gttggataaa tttttggagg atgttaggtg agaattgatg taaggttggg    7980
ggttaggtag ttgttaggtt tgaattttta gttattttgt tgtgttttta ggaatggaat    8040
ttatttattg atgaattttg tagttatttg atttttgggg ttgttttgtg tgttggtttt    8100
atttttggag gaggtttaaa aggttaagat tttgatgtta gagtttttttg attttgagat   8160
tagaaaggtg agtttttttt tttgttgttt agtgttattt tttagatttt ttaatagtta    8220
gataagaggt gttttttttt tttttagggt ttgtattttt atagtttagt ttttttttg     8280
gttgttagag attagattta ggttgatttt tttgtggggg ttatgggggt tataggatag    8340
gttgggtatt tttttttttt gggaggtttt ttgttttttt agattttttt tttgtatttt    8400
gttgtttgtt ttgaatttat tttaagtatt ttttttttag aatttatttg ggttttttta    8460
ttttgttagt aatttttttt tatttttgta ttttttttat ttttttttta ttttttttat    8520
tttttatttg gtgttgattt ttggatattt atgttttggt gttttttttag attttttttg   8580
tagtattttt ttttggttag gggttttttt tggtgtagtg tagttagaat ttatgtttta    8640
ataggtaaat tgtttgtttt tggttgttag tagagattat gggagttttt agttgatgtt    8700
taggttttgg gaggtttgat tttgtaggta ttgtgtattt tggtgtggtt ttgttttgtt    8760
taattttttt tttttatgt ttatagtttt tttttgttt aagttgtttt ttttgtggtg      8820
aaagttttta tttagttttt agtatttagt ttttaatgtt gatttttttt gaagtttttt    8880
ttagtttgtt aagttattag tagtgagatg ggtttttttt agagattttt tttttttttag   8940
tttttgttgt ttttttttgg tttttttggg gttgtggtat gtggttttgt tgttgggttt    9000
gtgagtttta gttttttttt gttgtaggtt atttagatgt agtgtaattt ggagagaagt    9060
gaggataagg tgtgttggta tgtgagtggg gtttgggagg gtggtgttgt tgtggggaag    9120
gtgggagtgt ggtggagttt tgaattttta gttgattatg ttttgttgtt tttttagttt    9180
attttgtttt tggtgttgga agattggttg tattggtagt tgatttatga tttgttttta    9240
agtaggttgg gtaggggatt tgggtggtgg gtggtgattt taggtggtgg gtgggtagtg    9300
gttttatgtt ttgttttatg ttattgtttt agtggatagt gtttaggatt ttgttttgga    9360
gtttgtgtat tatggttttt tttatgaggt gtgttgggtg gtgtggtgtg gtttggtgga    9420
gggggtgtat ggggtaggtt gtgttttttt gtttttatg tttttttttt tttgtaggat     9480
gattggatga agttggttgt ttttttggag agtatttttt ttaagtattg tgggatttag    9540
gtttgatttg gagttttagt tttaggggat tatgttgggg tgttgtttgg gtaggttatg    9600
ttggggagat tttagtattg tggggttgtt tttttttatg tgtttgggag tataaaggtt    9660
ttggtagtga aggaattttt tgttttttga gagtggggtt gattttgttt tgggtgttga    9720
gggg                                                                9724
```

<210> 301
<211> 1301
<212> DNA
<213> Artificial Sequence

<220>
<223> chemically treated genomic DNA (Homo sapiens)

<400> 301

```
aagtagttgg tgttgttttt gaatatattt gggtagggtg agtaggatgt ttttgtttag      60
ggagattttt tttttggtt ttggggaagg tgttttttat ttttgtagga ggttgggttt      120
gtaggatgta gtattgggtg ggtgagagaa agtttgggta gaagagtatg gagttggtga     180
ttggatgtga gtaagggggtg aatggagagt gttttttttg tgttaatgtt gttgttttgg    240
gtgttgaaaa gttgggaatt ttttggaaag tagtggtatt ttttttttg gaaggtgggt     300
```

```
gtgggggttt taggttttgt atttgttaaa tttgttttgt tatttgggag gggtttgtta     360
gatgtgtttt tttttagttt tattttgggg agaatgatgg gggtgggggt gtgggggtga     420
gtatagttgg gtatttttgt atatttgtag gtgggatttt ggggtgtttt tgtttttttt     480
ttttgggtag ttgtatgttg ggatttttat tttatttgtg tgtgaatttt tgttatttgg     540
gatttaagtg tttgtggttt gagtgttgtg gtaaatagaa ggtatttgtg tgggttttta     600
agggtgtttg tgtgttgggt ttgggggagt atgattgggg tatttgtagt ggggaggaag     660
aggtgttgtt ggttggggtg agttgtttgt gttaaggttg ttttttgtag ggtgagtttg     720
tgtatgatga tgtgtgggtg attgtgaata attttaatgt gtggtttggt gggtgttttg     780
ttttgttgag gtatttttat taatgatttt ttgggttagg gtatggttga gaatattagt     840
tataagtttt agtggttgtt tagtgttttt atttttaagt gtatttttgt ttagggtttt     900
tttttgagtt agggttgggt gtagttgtgg gattggtttg aagggaattt ttattttttta     960
ttatgttttg gttttttttgt ttttttgttt tttttatttt agttgtttat gttattaggt    1020
ttttaggttt tttttaggtt aatttgggtt tttgggatta ggtgggaggt gtgtgttggg    1080
gagtttttttt tgagttttta gagtttttttg aggtgtttgt gttttaggtt gtttgttttt    1140
tttggataga tttgggaatg ggtggttgga gtggttattt tatttttttt ttgttagttt    1200
ttttgggtga gtaagaaaat tagttttata tagttggttt tggaggtgtg gtgtgtgtgg    1260
aaggggtggg ggaaatgttg tttttagggt tgtaattttt t                         1301
```

<210> 302
<211> 1301
<212> DNA
<213> Artificial Sequence

<220>
<223> chemically treated genomic DNA (Homo sapiens)

<400> 302

```
gagaaattat ggttttgaaa atagtatttt ttttgttttt tttatatata ttatattttt      60
ggggttaatt gtgtaggatt ggtttttttg tttatttaga ggggttggtg gaaggaagt      120
gaggtggttg ttttagttgt ttatttttag gtttgtttag agaaaataaa tagtttagaa     180
tgtagatatt ttgggaaatt ttggaaattt gaagggaatt ttttagtgtg tgtttttttgt    240
ttggttttag gaatttagat tagtttggag aggatttggg agtttggtgg tgtgaatagt     300
tggagtggga gaggtaggga ggtagagagg ttaaggtgtg atgagaaata gaaatttttt     360
ttaagttagt tttgtaatta tatttggttt tggtttaaag ggaagtttta gataggagtg     420
tatttgaagg tggggatgtt gagtggttgt taggatttgt ggttggtgtt tttggttatg     480
ttttggttta agaagttgtt ggtgaagatg ttttagtgga gtggggtgtt tgttgggttg     540
tatgttgggg ttgtttatgg ttgtttatat gttgttgtgt atgaatttgt tttgtaggga     600
gtggttttag tataggtagt ttgttttggt tagtagtatt tttttttttt tattgtgaat     660
gttttgatta tgtttttttta gatttaatat ataaatattt ttgggaattt atgtgggtgt    720
tttttgttta ttgtagtgtt taagttgtgg gtgtttgagt tttagatggt gaaagtttgt     780
gtgtaggtgg agtggggatt ttggtgtgtg gttgtttagg agaggagggt aagggtattt     840
tggggttttg tttgtgggtg tgtgggggtg tttggttgtg tttattttta tgtttttgtt     900
tttattgttt tttttaaaat ggggttgagg aggggtatat ttggtggatt ttttttaggt     960
gatgaggtag gtttagtagg tgtgaagttt ggaattttta tatttatttt ttagggagaa    1020
aagtgttgtt gtttttttggg aagtttttgg ttttttagta tttggaataa tagtattggt   1080
gtaggggaga tgttttttat ttgttttttg tttgtgttta gttgttggtt ttgtgttttt    1140
ttgtttaggt ttttttttat ttgtttagtg ttgtgttttg tagatttagt tttttgtggg    1200
gatagggagt atttttttta aagttaggag aaggggtttt tttggataag gatattttgt    1260
ttgttttgtt tgggtgtgtt tagaggtgat attggttatt t                         1301
```

<210> 303
<211> 22355
<212> DNA
<213> Artificial Sequence

<220>
<223> chemically treated genomic DNA (Homo sapiens)

<400> 303

```
aggtgaatat atagtatttt ttgggtttta tattagtttt gggtgttagg gatatagtag      60
tgaatagaat agatggagtt tttttttttt gtagtggggg gttgtaatag gggggtttat     120
tggataagga gtatattttt attagagaga atattttagt tgggtgagtg gttttgtagg     180
gggttagttt gggggaattt ttggagatgg tgggaattga ataaagtttt tggggaggtt     240
aggtatggtg gtttatattt gtgattttaa tatttgggga ggttgaggtt atagggagtt     300
atgattatat tattgtattt tagtttgggt gatagtgaga ttttgtttta aaaatttaaa     360
aattgggtta ggtgtagtgg tttatgtttg taattttagt attttgggag gttaaggagg     420
gtggattatt tgaggttagg agtttgagat tagtttggtt aatatggtga aattttgttt     480
gtattaaaaa tattagagat taggttaggt atagtgattt atatttgtaa ttttagtatt     540
ttgggagatt gaggtgggtg gtttatttga ggttgggagt ttgagattat tttgattaat     600
atagagaaat tttgttttta ttaaaaatat aaaattagtt gggtatggtg gtatatattt     660
gtaattttag ttatttggga ggttgtaatt ttagttattt gggaggttga ggtaggagaa     720
ttgtttgaat ttgggaggtg gagattgtag tgagttgaga ttgtattatt gtattttagt     780
ttgggtaata agagtaaaat tttattttaa aaaaaaaaaa aatataaaag attaggtgga     840
ggttgtagtg agttgagatt gtgttattgt attttagttt gggtaataga gtaagatttt     900
gttttaaaaa aaaaaaaaat ttaaaaagtg ttttgaagga gttggggggg gagagaagat     960
agggttgtta gggttttgag tgtagtgtta gtttgtgtgg ggtatgtagg ttgtgtttta    1020
tgtttgtgtt gggagagaga gtagatgggg tgtttggttt tgtgagtggt tgtgagtatg    1080
tgtgtttgag ggtaaggggg atttgtttt ggttgtatgg gtgttaggtt tatggtatgt     1140
tttgggtgtg tgttgtgtgt gtgtgtattt gtatggtatg tgtgtgtatg ttgatatggt    1200
agttggtgta tttgtggttt ttgttggtag atatgtgttt tttagttttt tgtttttttt    1260
tatttgtttt ttttttattt tttatttttt tgtttttatt ttattttttt gagttgttat    1320
gtggttatta ttatttttgt ggtttttgtt tttagggttt ttgtgtattt ttttttttta    1380
gtgtttattt gaattttttt ttttattttt ttatgtttta gtttgtggtt tggtagtttt    1440
tttttaggt tttggggtta tttttggaag gtttggtggg ttttgttagg gaaggggttt     1500
ataggatttt ttgagggttg tggttgttaa ttgttgagtg tagggtgtga gtgggatttg    1560
gatatttttt gagggtggga attaggtagt gttttaagag atgttaggtg tatagggggtt   1620
gggggttggg ttggggggag tataggtttt tgggagttag aaggggattg gggttgtggt    1680
ttaggttatg gatagagtag aggtgtaggg atttgaagaa gtagagggag aagtttggtt    1740
aatatagtga gatttttattt ttatttaaaa gataaaaatt agttgggtgt ggtggtgtgt   1800
atttataatt ttagttattt aggaggttga ggtaggagaa ttatttgaat ttgggagggg    1860
gatgttgtag tgagttgaga ttatgttatt gtattttagt ttgggtgata gagtgagaat    1920
ttattttaaa aaaaaaaaaa aatagtggga ggtgatgtgg tgttgtagag gtggtagtgg    1980
ttaggatagt gggaggggta tgggaggtga ggtttttagg ttagttatga atgttgggtt    2040
tggttgtgtt ttaaggtttt tagagttttt tttttgtttt tgtttggttt tttaggaggg    2100
gagttagggg gtatagtttt tgtaggggtt ggtaggatta gataggggta atgttgggtt    2160
ttttgtattg ggattgaggt gtttttttgt ttttttttatt ggttgagatt agggttttgg   2220
gtatatggga gggatggggt ttagttgtta gtatggagtg ggtttgtttg tttatattag    2280
tttttgggta ttagttatta ggagtttgta gaattgtggg gagaaaaggt ggatgaggta    2340
ggaggaaaag aggggagagg ggtttgttgg ttgagggttt tggagagtgg taggttttag    2400
tttttgtggg aggggtattt tggtaggtgt gtgtagttgt tgttagggtt tttaggatgt    2460
gatgtgttag gattggtttt tttggaggtt atgttagtat tttggttttt gtttttgtat    2520
tttgttgtta gtttagttag ggagatttta gttttttttgg gaagttgttt ttttgtttta   2580
ggtttttttt tggtttgttt ttgtagtgtt ttttttgggt atttgggttt tagttttggt    2640
ttttgtttgt ttgtttgttt tttgtttttt tatttttaat gttgtttgtt tattttaggg    2700
tgtttgttgt ttgtttgttg tttgtttatt tgttgtagta tgtttgagtt gtttgtatgt    2760
aggttgttgg atgagttaat ttttgtttgg tttagtattg gttttatttg gttttgttag    2820
tttttgtagg agttgtgttt ttgagttttt tttttagagg atgtaggttt tttgaggttt    2880
tgttttgttt ttatgggttt tagttttgta ggttaggtta ggaggttttt atgttagttt    2940
attggtttga ggggggtttt ttttgatttt ggaatagaag atagggtaag gtagtttgaa    3000
gtttgggttt tgtgtttgtt gtttttttag tgtggttttg tttgtttttg tttgggagtt    3060
aattttagtg ttattagtag agttatagtt tgggttagtt ttggtaggta ttttgttttg    3120
gtttatttat tttgtgggtg ttgtggtttt gggtttgttt tttgggtaga agtgggagga    3180
agtgtatata tttgttaggg aggtttttttg gttgagggat attttaggtt tttattttgt   3240
ttttttgtta gtgtagattt ggggtttttt tttttttgtt agtttttttt attttttttt    3300
aggttagggg atgagataag ggattttgag aatagtttgg ggtgggaggg agattttagt    3360
ttttttagtt tagtagggta gggggttgtg ttagtttagt ttgggaaatt taggagggga    3420
aggtgagggt tttgattttt ttttggagtg ttagatgtgg agttgatatt agttgggtgt    3480
tgattttttt ttttgttatt ggtttttatt tttttagttt tgtagttttt gttggttaat    3540
gatgggggta gttgtttgtt atttttagag agttttttgt agtattttat gtttttttgtt   3600
tggttttgtt atatgttggg gtttttgtta attttgttta tattttgtag tatggagagg    3660
```

tgagttaggg gtttagtagg gttgggtggg aagtagtatt gaggggttat tgtgttagtt 3720
ttgggaggag ggtggttaaa ttggtgggaa ttttggtatg ggggattggg gtggttaatt 3780
taaggttagg atttattttt attggtattt gttttatgtt tttagtattg agattgatta 3840
gaagttgtag gagattatga agtagatggg ttatttgatt attgggggtt aggtattatt 3900
tttattgtgg tggggagagg gaggaggttt agttaggttg gatttatttt gggagtgtta 3960
gtgggggggta gggggtggtg gtagaggttt tagggatttt ttaatttttt tttttttttt 4020
agtgttatta ggtagaaatt aatgatttgg agaatttggg tgagatgggt agtggtattt 4080
gtggttaggt gtggaagatg tgtttttgga agattggtta tgttattgtt gttaaggtga 4140
gttttggtgg ttattttggt tgtgttttat attttaggtt ggtgttgagg ttttttttttg 4200
tttttgtttg tgtagtaaat gtggtgtttt gggaataagg aggagaataa gtgtattttt 4260
atggatttgg atgtggtgtt gaagagttat gattgttttt atattgtgta gtgttttggg 4320
atgtttatta ttaatgtgag tatttggttg tgttttgtag tgttttttttt tttttttattt 4380
ttgttttttt ttagggagta gagtttttgg ggggtgggtt ggaagatata gtttttttgg 4440
gtgttttttt ttttgtagat ggatgttttt attgttatgg agtttatggg tatttgtgtt 4500
gagaagttta agaagtggat gtagggtttt atttttgagt gtattttggg taagatgata 4560
gtggtggtga gtgattaggt ggggtttgta ttgggtagga tgatagaggt ggtgagtgat 4620
taggtggggt gtgtattggg taagatgata gtggtggtga gtggttaggt ggggtgtgta 4680
ttgggtaggt ggttttgggt ttgtgttttt tgttatatgt atttttttttt gtgtgtttgt 4740
agattgtgaa ggtgttgtat tatttgaagg agaagtatgg tgttatttat tgtgatgtta 4800
agttttttaa tattttgttg gatgagtggg gttagattaa gttttgtgat tttggtatta 4860

gtggttgttt ggtggatttt aaagttaaga tgtggagtgt tggttgtgtt gtttatatgg 4920
tagtgagtgg gggtttttta gtgggggagg gggtgggggt tgggaggttg gttttagttt 4980
tggagatatg tttttttttt tttttttttgt agtttgagtg tattgatttt ttagatttta 5040
ttaagttgga ttatgatatt tgggttgatg tatggagttt gggtattttg ttggtgagtt 5100
ggggtttttt tttgtttttt agttaggagt gagggttttt gggggatttg gagggaggag 5160
aatataaatt tgtttagttt tgtttgtttt tttttaggt ggagttggta ataggatagt 5220
tttttttataa gaattgtaag atggattttg aggtttttat taaagtttta taggaagagt 5280
ttttgttttt gtttggatat atgggttttt tgggggattt ttagtttttt gttaaagatt 5340
ggtgagaatt ttttttttatt tgggaggtta gggatagttt gttgttttgg gtagttgggg 5400
aggtaatggt aggaggggaa tggaggttgt attttgggat gggtggatgt gattgtgggt 5460
gggtttttgg ttggtggttg ttataggagt tggagttggt gtttagggag ttatgagttg 5520
ggtttggatt tagtttgagg ggatagttag ttttgtggta tttggtgggg tggttgttgg 5580
agattgtgag tatgtttttg taataagtat agttataagt ttaggagggt tatgtttgat 5640
ttttttaggg gagttttatt tatttttttg ggggatttta gttagttttg gttttttaag 5700
ttaggttttt gttatttggt tttaggttag gaggttttgg gatttatagt tgttttttgt 5760
tgttagttgg ggttttgagg atatttatag tttttttttt tatatatatt ttggggattt 5820
ttggtttttt ggggagggtt tgagtatagg ggtggggttg gtattttttt tttttttgtt 5880
tttatatttg tttttttttt ttttgtttta gttttattaa agattatagg aagagattaa 5940
agtataataa gttatttgtg agtatttgag ttttttttagt ttttgttttg tttttgtgga 6000
ggtgtgaggt taggagggaa gattgttttt ttttaagttt tattttttttg ggtttatagg 6060
aatatagttt tattaagtgt tatgagatgt tggaggtgga tgtggtgttt tggtttaagg 6120
atgttatggt gaagattgag ttattgtgga ttagtggtgt tttgagttag ttttatttgt 6180
ttttttttag gtagttgttt ggtggtggtt agttttatag gggttaggg gtatggttat 6240
aggttttttt ttttatttgg ttatttagtt gtttgttagg ggagatttgg gatttggatg 6300
gttatttagg attgaggata gagagtgggg ggtgtttatt tattttttttt gttttgggtt 6360
tattaagttt ttgttttttt tattttgggg ttagttggtt gtgtgtgttt tttgatagat 6420
attgtgaatg gaagatagta ggttgtgatt agagttgttg tttatttagt tgtagttttt 6480
gggttggggt ggtttttagg ggttaggaga gagttttgga gttttgtagt tattatgtat 6540
gtttttagtg tgttgtgttt tttgttattt ttatgtgttt gtttttttttt tgttgttttt 6600
tgtttttttgt tttattttttt tgtttttgtt tggttttttt gttatttttt ttgtttttgt 6660
ttttttttttg gtttgagttt gggtttagtt atttttttgat gggttttttg ggtttgtata 6720
ggttttttat ggtgtaatga gttagtggtt tttagttagg tagtgtgggt attgttattg 6780
tggttggatg gggttgtgtg tttgtgtttt tttttttttt tttttttttt ttttgatttt 6840
aggggggtttt ttttggagtt tattgtattt tattgtattt ggtggggtgt ttggggtggg 6900
ggtggaggag agtttgtttt tgtggggttg ttggtatttt tagaaatttt tattaaagtt 6960
atgattagtt gtttgtggtg gggttttaat tgtttttggg tattggggag ttgggttggg 7020
gttgttgttt tggggttttt gggggttata gtttggggtg agttgaagat tttaggggat 7080
gtggaggggt ttgtggggtt ttggttgtat aggatggttt ttagggaagg tggttttggg 7140
gtatggtgta gagtaggtga ttggagggaa ttggtgatgg agtggggtta agggaggggt 7200
ttggagggag ttagggatgg agggtagagg gagtggatgt gggggtttga ggatgtgtga 7260

```
taagttttag taggggtggg ggttgggttg agggtggggg tgtgaggtgg ttatttttat     7320
tgtgtttttg gttgtttttt tatttattta tatttggttt agtttatgtt gaggtttagg     7380
attgggaagg attgggtgag tgtattgggg atttaggtta ggtgtttttt ggagtttgtt     7440
ggggtggtta gagtaggagg gggtgtgttt tttttttgtg ggtgttgtat gtaaattaag     7500
tggataagaa aaaataataa aataaaaaat aagaaaaaaa aaatataaaa ttttgtaaaa     7560
ttataaagaa aatttaatat taaaggtgta gaagttggtt ggttgtggtg ggggtagtgt     7620
aggtgtagta tttttttttt tttatttagt ttgttgattt ttgttattat tatgatattt     7680
ataaaatgtt gtatgtttaa aaagttatag atgttatttt tttttttgttt ttagggagga     7740
aagttatttt tttttgtttt ttggtttttt tgttaggggt taggggtttg ttgggtgggg     7800
gtgttaatag gtttggtttt tttttttttt gtatttagtt atgggggttt ttgtgattgt     7860
tggaaggttg tatggttggt tttagggtta gtataggttt gaggttgggt tgtttggttt     7920
tatttttatt taattttatt ttttgtttta tgagtgtgtg tttgtttatt tttatttttt     7980
ttagtgttaa gtggggagtt ttgggggagt tttttttgtt ttttagtttt ttttaagatt     8040
tttttttttg ttattagtta tttttttgga ttaggtagag ggtggattgg gtgggtaggg     8100
gtttgagggt ggtttgggtt agtttattag ttaatggatt ttttttttagg ttgttagtgt     8160
tgttttgttt tttttaaaa taaaatgttt ttgtttgtaa atttttagat gtttgagaat     8220
aaattttttt tttttttttt attgagtttg tggtgtgtta tgggtttggt agggtgaggt     8280
gggaaggggg tggttgtggg gggaggtttt tgtattgagg ggagattgag gtaggtttgg     8340
gagttagatg ggaggtgttt gtagtgattt gttgatgggt attaagtagt tggtgtaagg     8400
gatttttttgt ttttagtggt tataggttgg gtagtagagg atttttaat tttttttaggt     8460
tttgggtatt ggttttggtg ttagtttttt agtttgttat ttttgttttt tgtttttttt     8520
tttgttaagg atagggttgt ttgtttatgt gtgggatttg ggtgtagttt agttggtgtt     8580
tgttttggtt ttaaatattt tgattttagt tggtttagta tgttgggtat tgtgttgttg     8640
ttggttttgt ttttagggat tattatttta tttagtgggt tatttggtat gtatgggtag     8700
ggaggtgatg ggtttgttat gtttttattt ttgttttatt tttttttattt tttttgagat     8760
ggagttttgt tttgttatgt aggttggagt gtagtggtgt aatttttatt tattgtaatt     8820
tttgtttttt tggtttaagt gatttttttg ttttagtttt ttgagtagtt gggattatag     8880
gtatgtatta ttatgtttgg ttaattgttg tatttttagt agagatagtg ttttttttatg     8940
ttggttaggt tggttttgaa tttttgattt taggtgattt gtttgtttttg aattttttaa     9000
agtgttggga ttataggtgt gagttattgt gttttattga tgttattttt ttttaattag     9060
atttttggtg gtggttttta tgggggtggg gatagggtag gggagtgggg tggggttttt     9120
gttttttatt ttaagggtgg tttatatttt tattttggta aagttttttg tagttatgga     9180
gagggttaga gttgtgggag gggggttagtt tatttaggtg ttttttagaa gtgggtgtgg     9240
tgtgtggatg gataaggggg tttatgtttg taattttagt atttaagaga ggttgaggtg     9300
agaggattgt ttgaagttag gagttttaga ttagtttggg taatatagat tttgtatggt     9360
tgtggtagtg agtggttgtg gttatagtta tttgagaggt tgaagtagga ggatggttgg     9420
agtttaggag gtggaggttg tagtgagttg tatttgtatt attgtatttt agtttgggtg     9480
atagagggag atttttgtttt agaaaataag aaaaatggat gtgtaggtaa ttttttgtat     9540
ttggagatta gttgttttat taggattttg tttagtattt gaaggtagat attagtgtgt     9600
gtttaggggt atgaagaggg gaggggtttt atggttgttt tagtttggat aagtggaagt     9660
gttttagatt ttggtggttt ggttgggttt tggttgtgtt taggagagtt ttttgggagg     9720
gtattttttt tatttttttt ttttgttggt gatagtattt atttttttttt agaaagggga     9780
gttgtttgtt ttgttgtgtt tttaagagta gttttgggga aggtggggga gttttgattt     9840
tatttagttg gattttatta ttgggtgttt tttattgttt ttttttttgtt ttagggaggg     9900
ttttgttagt tttggaggtt tttttagggg tgtatggttt tgtaattggt ttagttgttg     9960
ggttgtgtga gttttagggg ttgttagggg gtatattatt gttagggggg aaagtggtgt    10020
ggagtttatt atgggtgaat tggttgttgt aattgtattt ttttttttaaa ggtggtggtg    10080
ggggtgaggt ggttgggtta ttttttttgg aggtttaaag ggtagtgtgt gttttttttt    10140
ttttgggttg tttttttttg ttttgttagt ttttttgttt tttgtggtgt ttggtttttg    10200
gttgtgtaga ttttttttta gtttgaagtt gtttgatata gaggatgttt tttttttgttg    10260
tgtggggttg tttgaggttt tggttgatag ttgtgtgttt gtgtaggtgg ggattttggg    10320
gatattaggg gtggatgggg gttggtgggg atgggtgatt tttgatggtg tttttgtttt    10380
ttaggtggtt ttggtttgtt tttttttgtg ttggggtttg gttttgtatt gttgtttagt    10440
gatgttgttt ttatgtttgg ttttggggtt tgttttggtt ttgttgtgtg agggttgttt    10500
tgttgatatt tttgttgttt ttttgttatt gttgttgttg agtttgggtg ttgtttgttt    10560
tgtgtgtttt agtttttgtt tgtgtttggt ttttaatgtt ttggtgtagt ttttgtattg    10620
ttagttgagt tgttgttgtt gtttttgggg agtttgttgg gtgtttgtgt ttttgatgtt    10680
gttgttgttg ttgttgttat tgtgggtgtg tgggtgtaga gtttggaatt tgggtgttgg    10740
ggttttttgg gggttgggta tgggtgattt tggtagtgga aagaggatat ttttggggtt    10800
tgaggatttg ggggttgggg gaggtggggt gtaagggttt atttttgttg atagagtttt    10860
tttggggata gtgtggatat tgggtttttt tatggatagt tttggaattt tttagggttg    10920
```

```
ggggttggat gtagaaggtt attaagggga tatattttg ggaatttggt gttgggtttt   10980
atatgttagg tgttgggaga tgtggattat gtgattttgt ggggttggga tttggggttt   11040
ttaggggttg ggtggttagt tatttgtatg tgtaggggtt gggtttaggg tattttaagg   11100
ggttggattt ttaggggttt tattttggtt gtggtggaga gttggattta ggttttttt    11160
gggggttttg ggattagag ttatgatttt ggtgttttga tgtttttgtt ttttttttat   11220
tttttttttt tttagtgttt gttttaggat gaggtgtgtg ttgatattgg tagtggtagt   11280
gttgagggtt tggttgttga tggtttttat ttgtatatgt tgatgtagtt tattgtggtt   11340
attgtgttgt ggttgttttt tagtgagtat gtagtttttt tttgtatggg gttgtggggt   11400
ggtagagtgg gtgggaagga tgtttgggag ttggatttag ttttagtgtg gtatttttta   11460
tagggttgtt taagagtgtt tttggttgtg tagtgtgttt tgagtttttt gtgttggttt   11520
ttgtggtttt ggaatttgtg ttggtggtgg tgttggtgtt ggtagttttt gtgttgggtg   11580
ttgtgttggt tgttgggttg ggttttgttt gtgtgtattt aggtattgat gatttttgtt   11640
aagttgggtt tttagtttaa ttttgtgtgt tgggatttgg ggtggttgtg attttgtttg   11700
tggggtttga ttaattttag ttaggtttgt ttttgtgatg tttgtagtag tttttagggg   11760
ttgtttttag tttggtgtgg tgtgtagtag tttttttgta gtttgttttt tttttttagt   11820
gttttatgtt tttggtttgt ttgtgagagt tttgtttagt ggtttttagt ttaggaggtt   11880
ttagtgagga aggtaggtat ggaggtggag ggagttgggt gaggtaggag atttgatagt   11940
gatgtttttt agtgtttagt ttgtgttagg tattgtggtt tttttgtttg tttgtttgtt   12000
tgtttgtttg ttttttgaga tggagttttg tttttgttgt ttaggtggga gtgtaatggt   12060
gtgattttgg tttattgtaa tttttgtttt ttgggtttaa gtgatttttt tattttagtt   12120
ttttgagtag ttgggattat aggtatgtgt tattatattt ggttaatttt gtatttttag   12180
tagagatggg gttttttttat gttggttagg ttggttttgt atatttgatt ttaggtgatt   12240
ttattgtttt ggttttttaa agtgttgaga ttataggtgt gagttattgt gtttagtttt   12300
taggtattgt tgtaaatgtt ttatgtttat gaattaattt aattttttta atttttaggt   12360
ggatattatt ataattttta ttttagagat gagaatgatg aggtattttt tgaattaagt   12420
aatttgttaa aagttataat attgggtgga gtttggattt gaatttaggt agtgggttat   12480
ttgtggtagg tgtgggtttt gggttgagtt aggggtatta ggtgaggaga ggtattaggt   12540
gaggagaatt tagtatttgg atttggtttt tatttttatt ttggtatgta gattgatgga   12600
agaggttagt ggtttgttgg gaagggaggt ggtttgtgta gtgtttttttt ttttgttgag   12660
ttttagttat ttttttttagg gatggtgtgt ttttaatatg gtttggagat atatttagtt   12720
attaatttgg gattaggatt aataggattg gatttgtttt tttggatttt ggatttgatg   12780
aggttatgat ttttgtgttt tttttttttt tttgtttttt ttatttgtgt ttaaaataaa   12840
tttttggatt gattggttaa gttttggtgt agttagtgag tgtgtaatgt gtggggtggg   12900
ggataggtgg ggaggtttgg ggagaaagtt gggagtagga tttggggggt atttttgaga   12960
aattgtgatt tataggggtg gagtttgggg atggggttta tatgtttata tatttgtgtg   13020
tatttttagg ttatgggaga ttgtagggtg ttttgtttta aatagaaagt gatatttggt   13080
tgggtgtggt ggtttttgtt tgtaatttta gtatattggg aggttgaggt gggtggatta   13140
tttgaggtta ggagttttag attagtgtgg ttgatatggt gaaattttgt ttttattaaa   13200
aaaagtaata aaaaaagaat tagtagggtg tagtggtgtg tgtttataat tttagttgtt   13260
ggggaggttg aggtaggaga attgtttgaa tttaggaggt agaggttgta gtgagttgag   13320
attgtgttat tgtattgtag tttgggtaat aagagtaaaa atttgtttaa aaaaaaaaaa   13380
aaagtggtat ttaattttat aagtgtaagt ttaaaataaa tttttttttt tttttttttt   13440
tttatttgga tatttgttta tttgtttttt gtgatttttt ttaagttagg tttttttttgt  13500
tttttattat ttttgtttgg atttgttttt agttttgttt tgatttttttg tttttgtttt  13560
agtttttttt tggtttggat tttgtttttt ttttagattg ttagtttagt ttagagtttt   13620
ttaaattttt tttgtttgag ttttatttta ttttatatag ataagagtgg gattattagt   13680
tttagtttat agaggaggaa attgaggttt ggtaaggtta aattatttgt ttaaagtttt   13740
taggtttgtt tgattttaga gtttaggttt ttgattgatt ggatttttgg atttgatttt   13800
ttggttagaa tttatttttt tgtgagaagt aaagtatata gtagtagttt tatgagttag   13860
tttagtaata gagggagggt atgggattta aggtgttgtt tttattttat gaatgagata   13920
aagtgtaggt tgagggtttt ttttgttttg ttttagtttt ggtgttgatt tttttttat    13980
ttttattggg gtggtttttt gggtttttaa atgtgaagtt atgtttttat gtgtgtttgt   14040
ttttgtgtgg ttttttttga ttttggtggt ttttgtagtt ttgtttattg gagaagtgat   14100
tttatagtgt ttgttttttt ttgagtggta tgaagttatt ttttaggtgg tgagtggttt   14160
tggtgtgtta tttgggtgag gtgattggtt ttgtgatttg gagtgtttgt gagaagtggt   14220
agttagtgtg atttttgtag gtgtggtagg gttagttgga gttggatgtt attgagttgg   14280
tttgggagtt gtggggttgg agtgaggttg aggtgagatg tggtttttgg gattggagtt   14340
aggttggagg tggggttggg gtggggtgga gagtgggtgg ggttgtggta ggagttagtg   14400
gggtggggtt aggaatgtaa gtgagtgggg gtgtggtgtg gggttgagtg gtggggtgtt   14460
agtgggtgag tttgggtggg gtgaggggtg gggtgtggat gggaagagaa gatttagggg   14520
tggggtgagt ttgggagtta gatgtgggtg gggtataagt gaagtggagg ggtggggtga   14580
```

tgttgggggt ggggtgtgtg tgttgttgag gtttggagag tgggtgggtg agtttgaggg 14640
gtaggatgag tttgggggtg gggtgtgggt gggttaatag taagatttaa gggtggggtt 14700
gttttgggag taaggtgtgg gaggggtgtg ggtgggatga gggtaagttt taaaggtggg 14760
gtgagttttg gggtaatgtg tgggtgaggt gagggttaaa tgggtggaat gaattgggag 14820
taagatgttg ttgagtttgg gtgaaaagta atgtatgttg ttggatagga tatgagtttg 14880
aagtgagagg gaatagagaa gaggggatga agaagggagt tgaatggggt agtagttagt 14940
atagggttgg gttttggttg tgtgaaggag ttggaattgg tttgggtttt ataatttttt 15000
taggtagttg gttatgtttt gggtggggta ggggttttgg tagttattgg gatttttaat 15060
attgggttag taggagatta aggtgtagta gtggggttta gagtatataa gggattgggt 15120
tttggttttt ttgttgtagt tttgagttta tagaagtttt attttgttgt tttttttttt 15180
tttagatttg ggtttttttt tagtagttta agggttgtgt agtttgggag gttatttaga 15240
aagtgtattt gggtggtttt tagggattaa ggtgttggga ggtatagttt ttagttttta 15300
tagaggtgag gtagatgaat agggtgaggt tgtttagggt aggtttttgg tgggtaggtg 15360
ggagttgtgg gggataattt gattagggga tttttaggag gagtagggtt tgggggggtta 15420
ttgtaggttg atttttgggt tgattttgta tttttggttt tatttaggtt tggatggatt 15480
tggttgagaa gataataggg ttattggaag aagttttggt agtggttttt ttgtaggtat 15540
tgttattttt ttaggtaggt tagggtgttt tagaggatag ggttatatgg gttaaattat 15600
gtgaatttgg gttttgggtt tttttgttag ttttgtggat tttgttagtt attggatatt 15660
ttgagtttta gtgtttttgg ttttgaggtt attttttgtt tgaggagtat tttagggtgg 15720
tagggaggat gggggaatgt gtagatggtg agaagatttt ttagtttttt tttttttttg 15780
attgttttgt ttgttaggtg tttattattg tggttatgga attggttatt tttttgtatt 15840
ttaagttttt taagtttatg taggtttgtg gaaagttttt gtttttgagt gtttttggag 15900
gataggaaga ttttgttttt gaaatattta gttttgtttt tgttgtattt agttttgtat 15960
ttaggatttt tgattttgtt tttgagaaat tttttgagtt gttggttggt ttttttattg 16020
aagaagattt tgttgtggat tttgagaaga tttataagta tttgtttttt gtttttttgaa 16080
gtggttgtag ttttgagttt ttagtagttg gtgagaaggg tgagggaggg ggtaggagta 16140
gagggattaa gggtttttgg tagtaggtag gggtgttttt ttattttttt tttttattta 16200
ttattagagt ttgttgtggt ttttgatttg tttatgttat ttttagagga gttgttattt 16260
ttgttttgta tagttttggt tgagtatatg atggagatgt atttatgttt gatagttttt 16320
tagtttattt ttgttggagg gagtttgggg tttgtagtag aaggggatgg ggttggtttt 16380
aaggtattag aggagatttt tttagttatt gagaaggttg agtatagtga attgaaattg 16440
ttttggtaag tagttgggat ttgttttttg aatttgtttt tggtgttttt ggagtttttg 16500
ggttgggtag ttatttttgt taggtgaggg gtatggtggg taggaggtta tattaggttt 16560
tttgttttgt tttttggttt tgtttggttg gttttggttt tttttgagga ttttgttatg 16620
ggggaaggtt tttgagatga tgtttagttg tggggtgggt ttttaagatg ttttatattt 16680
tgggggtttt agaaatggaa tttttgttgt ataggggttg ggtgggggtt gtaggatttt 16740
atttataagt tttttgatgt taaggatagg tggatagggt tggttttttt tagtttttaa 16800
gttttattgt gttttgttgt ttgttggggg gttatagttg gtattgttta ttgtaaaggt 16860
ttttgtatat tttttttttt tttgtatatt ttggggttag tatttttatt ttttttaatt 16920
gattagttgt ggttattttt tgttgttagg tttttttttt ttttgggggt attttgtgat 16980
tatgaataaa gttattattt tttttttttt ttatttgtga tttaaagatg gaagtggggg 17040
ttttgaagtg ttagagtttg ggggttatag ggttgggggt gtgtgttagg gaggagagtt 17100
gttatttgat tggtgtgatg aggtttgggg tgaggagttt tttggtgatt gtgaagttga 17160
taagtgtgga tggggagagt tggtagggta ttttggggat aggtgttagg gatgggtttt 17220
agagtagggt aaaattatat tgtataggtg gaggggagaa ggtagtagag agtgggggtg 17280
atttggtgga aggagttgag ttttaggttg ggtggttaga ggaagataga taggggtggg 17340
ttgttttgag tggtaggagg ttagtgggag tggagtgttt tttggggtgg ttttgggatt 17400
ttaaggattt ttagaaggtg gggagggagg ggtattggag ttagggttga ttttaaattt 17460
ttgggggtgt tgggattttg tttgaggttt tttttattag ttttatttgt tttgtgggtg 17520
tggattgagg gttgggtttt agagagggaa gtagatgttt ttttatggta ttgtgtttta 17580
tgtttagtta gtaatttaag ttggtaaatt ttttgttttt ggagtttgtg tatttattta 17640
gtttgttttt tatagaaatg tttatttttt tttttatata ttgtatatgg tttttttggg 17700
gtgtatattt tttgggaggg ttttggagta tagttagttt attttgtttg tggttattgt 17760
tttattgtat tagatttata aatgttgtta atggtggtgg gtagggggag gaggtttagg 17820
ttgtaggtat ggttggttgt tggagttttt ttttagggtt ggtagagggt gtgtttttta 17880
gtttttttata gggttgagtt tttttttatta tttttggtgt ttttttagat taatagaatt 17940
gtgtgtggag gggaggtagt tgggggttat atattagttt ttttttttaa agttggtgaa 18000
aggtgtttgt gattttggtg tttttgtagt tttgggaagt tttaaaaata tgtaaaagtt 18060
agatttgggt tttattttta gttttattag atttagttta tgtgattgtg ggtaagttag 18120
ttattggagt tgtttggttt ttggtttttt ggtttgtaga atagggatgt taatagggtt 18180
tggtttattt ttatgatttt gaagaggaga taggttataa ataggaggtg ttaatttagt 18240

atgttatttg gtatgtagta agtgtttaat aatagttttt attattatgt ttggtttaga 18300
ggtagggagg agtggaatgg tatgggaggg ttgtgggagg tatggtaggg gggttagggg 18360
taagtggtag gagggtggat ggggggtagt ggtgggtatt ggggtagggt gtgttgattt 18420
gttttggggt ttgggttggg ggtagaaatg agtttgttta tgttgttttg ttatggtagg 18480
tgttatgtat ttttgatata gttgttatgg taggtttttg ggttttgttt ttgggatagg 18540
tggtgtaggg taaattggta tgtagtgttt gttttgtggg tttgggagag tttgttttgt 18600
agggattaga gtttaaggat gggtttaata tttagttaag gtggggttga tgatggttag 18660
ataatagggg agggaggagg gataaggggg tttttatttt tagggatgta taatagtaga 18720
gttatttata tgttggggag ggggtggtgt gggggttttt tttttgtttg ggatttagag 18780
ttgggggtgg gggttagttg ggtgggtgag atgtgtagag aggaagggat agggtggata 18840
aaggtatgag gtggggtttt ggttaggtta ggaagggata tgggaggggt tttgaggggg 18900
aggggttggg ttttttttaa tttttttttt ttttttttttg ggttgggggt tttggggtgg 18960
gattttgagt gtagttttgg ttttgtggtg ttttttgttg ggttggtttt ttgagatttt 19020
ggtgtagggt tggttagggg gtgttgtgtt ttttatatta atgtgtagtt gaattgtttg 19080
tgtttgagtg tttttttgtg gttggtttag gatgaggtgg gtatgtggtt gtaggggttg 19140
agtaggatgt gtatgtagtg tattattaat agtattagta ttatgagtag gtagagtatg 19200
aaggtgaata ttgtgtgttg ttttgtgttt aggtttgtgt ttattggggg ttttgttgat 19260
ggtggtaggg gttttggtga tagtgtttat gttgtgttta tggtttatat tgttgtgtgt 19320
tttgtggagg aggggatttg ttttgggtgt tggggatgag gttgtgtttt ttttttttgtg 19380
tttttttttgt tttgtttttt tttgttgttg ttgttgttgt ttttttttaat gtgtttgatt 19440
ttattttttg tttgtgtttt ggtttttggg ttatgtttat gtttggtttg ttggattgtt 19500
gttattatta tgtggggatt taattttggt tgtgtgtgta ggtgggggtg ttgagagttg 19560
ggaaatggag ttgtgtttgg tttgggttgt atataatagg tgtgaaagtg tggttgtggt 19620
ttgggtgtgt ggtgtgggga tggtttttgt ggtttttgtt tttgtttttt ttgtttttgg 19680
tggtttggga tttttttttt tattgttttt gttttggtgt gtttttttatt ttggttttga 19740
tttggatggg gattgattgt ttggtggttg tgtttttttta gggatttgtg ttggttgtgt 19800
gtttggtttt tttttggtgg tggtggtggt ggtttgggtt ttggtttagt ttattttatt 19860
tgtttttgga gtggtggaga ttgaggtagg taagtagtgt gtgagttggg ttgttgtggt 19920
tgtttttatg gtgatggggg tggggttgtt gtggggggtg gggttgtagg gggtggaggt 19980
tgtgtaggga gtggtgtgtg agttagtgat tttgtttggg tttgtgggag gatggaattt 20040
tagttttgag gatttgtgag tatttatgat tttgttgggg agtgggtagt tgttattttt 20100
atttttttttg gtattttggg ttttttagtt tgttttgtgt ttgtattttt ttttgtgata 20160
gattttattt gagatttttt gtttgtgggg ggattttagt ggttttaaaa tatttatatg 20220

ggtatttaag ttttttttggt tttggggatt ttaattttta gagagtttag gtaggttatt 20280
gggatgagta agttttttta gttttgtttg ttattggttt tgttttttaa tttagggttt 20340
agttttgggt aaggtgttta tttattgtgg ttgtagtttt ttatttgatt ttgattgttt 20400
tttatagtag ttgtttgttt ttatttttat tattgttttg gttatttttg tgttagagaa 20460
gatattttag atttgtagt tgttttttttg taatttggtg ttttattttt tttaaatttt 20520
aggtttatgt ataagatgga agagtattgt taaaattaaa aaatggattt aaagtggttt 20580
ggttgatgtg gttagtgggt tattgagttg tgggttttgg gttttatttt gttgtggggg 20640
gagtttttgg gttttggggt tttttggtat tgtgtgattt gtgtgtattt taggtgatta 20700
ggtgttggga tttttgtagg gtagagtaat agggtagggg ttggttttgt gggggagtgt 20760
ttttagttgt tgtgtatttg taatagtttg ttgttttttt ttgggttagt tggttttgta 20820
gttgttttgg tagagttggg ggtagagttt gtagttttgt ttttagaggt ttggagttgt 20880
tgtaggtggt gttgtggtgt ttttgtgttt gatgatttag gttggggtta tttggttttg 20940
gtattatatt tagagaattt gtttggtgtt ggaggttgag atgtttagga gttgttaggt 21000
tgtgtagggg ttgagtttgt tttggttttg gtagagtgtt tatatgtata gtatttgtag 21060
tattttgttt tgtagggtgg ggtgggaagg gtattgttga gaggggttgag gtgatttagg 21120
tagttttggg ggggggttag gttttgtggg gtaggagtta ttggtaattt ttttaggggt 21180
tgggagtaga ggttagtata attgtttaga tttaggaatt atttagtttt ggtaaaaata 21240
gataggtttg ttggtttttg atttttttta attttaaatt ttggtttaga ggttttttta 21300
tatttttatt tagttttaga gttttagggt tttgagttgg ggtttttgtt aggtggttat 21360
atatagattt tggggatagg tggtgttatg tgtttgtttt attttatttt tttatttatt 21420
gggttatttt ttattatttt gtttttgggg tttttgatta ttattattag ttgtgtttgg 21480
aaggtgatga ttagtattgg tttttgtttt attattttgt ttatggttag ttgtagaggg 21540
gttgagaggg gggatgtgtt ttagaggata gtttggttgt ttttttggag gtgaaatttt 21600
tgtgtttagg ttttgttagg agaaggtttt agtattaggt ttagtattgg ttttttttttg 21660
tgggttttgt tttttaggtt aggattaggt tttttttgag atttttttttt tggataagtt 21720
taagattttt tggattagtt tatggttttt agtttttggt aaggttattt tttgggtttt 21780
gtttgggtta tagtttttat ttttgatggt ggtttgggtt tttattgtga ggtgtttatg 21840

```
ttgatgttgt taatgtttag gatgtgagaa tggaattgga gatttagtgt tttggtttgt   21900
tggatggggt gggttagttg gttgtaagtt tgtaatgaga ggttgatatg tttgtgtgat   21960
gttatttagg gtgggtatta tgttatatag agttgttgtt tatatatttt ggtgtttagg   22020
tgttttgttg ttagttagta atggtagagg ttaagaattt aggtttaagt ggggaggtag   22080
gtgttattta tttattggtt tagattatat gttttttatt tttgggaata atattttatt   22140
tagatgattt aatgaaaata ggattaatta gtaaagtggg tgattttttt ggaataaaat   22200
tttaaattta tttttagggt taagttggtg gtttttttggt tgatttttaa tatttttata   22260
tttgttgtat tttgaatttt ggtttgagat taaatgtttt ttttaggatt tgtttttagt   22320
ttgaggttgt ttttgttatt gattagtttt tagtg                              22355
```

<210> 304
<211> 22355
<212> DNA
<213> Artificial Sequence

<220>
<223> chemically treated genomic DNA (Homo sapiens)

<400> 304

```
tattgggggt tggttagtga tagaggtagt tttaggttga ggatagattt tagaagaggt      60
gtttgatttt aggttaagat ttagagtgtg gtgaatatgg aagtattaaa gattagttaa     120
ggggttgtta gtttagtttt gggagtagat ttgggatttt gttttaagag aattatttat     180
tttgttgatt aattttgttt ttattgggtt atttggatag agtgttgttt ttaggagtgg     240
gaagtgtgtg atttgggtta gtaagtgggt ggtatttgtt tttttatttg gatttgggtt     300
tttggttttt gttattgttg gttggtggta gggtatttgg gtgttagggt gtgtgggtag     360
tagttttgtg tggtgtggtg tttattttgg gtggtattat gtaggtgtgt tggtttttta     420
ttgtagattt atagttagtt ggtttatttt atttagtagg ttaaggtatt gggtttttag     480
ttttattttt gtattttaga tattgataat gttgatgtaa gtattttgta gtgggggttt     540
gggttattat tgggggtggg aattatggtt tagataaggt ttaggaggtg gttttgttag     600
gggttggggg ttatgggttg atttaagagg ttttgggttt gtttaagggg agggttttga     660
gggaggtttg gttttggttt gagaggtaag gtttatgggg agggattggt gttgggtttg     720
gtgttggagt tttttttag tagggtttag gtgtaggggt tttattttta ggggagtagt      780
tgggttgttt tttgaggtat atttttttt ttggtttttt tgtagttggt tatgggtaag      840
atgatggagt aggggttggt gttgattatt attttttagg tatagttggt gatggtggtt     900
aggaatttta aaggtgaggt ggtggagggt gatttggtga gtgagggagt gggatggggt     960
aggtatatga tattgtttgt ttttaagatt tgtgtgtggt tatttggtag agattttagt    1020
ttagagtttt gaagttttgg gattgggtgg ggatgtggag aaatttttgg gttagagttt    1080
ggggttgagg gaggttggag attagtagat ttgtttgttt ttattaaggt tgagtggttt    1140
ttgagtttag gtaattgtgt tggtttttgt ttttagtttt tggggaagtt gttggtggtt    1200
tttgttttat agagtttggt tttttttag agttgtttgg gttattttaa ttttttaat      1260
agtgtttttt ttattttatt ttgtaggata aggtgttgtg gatgttgtat gtgtgggtgt    1320
tttgttgaga ttaggatgag tttaattttt atgtggtttg gtggtttttg gatattttgg    1380
tttttagtat tgagtagatt ttttgagtgt ggtgttggag ttaggtagtt ttggtttggg    1440
ttattaggta tagaggtatt gtaatattat ttgtggtaat tttagatttt tgggaataag    1500
attgtgggtt ttgtttttag ttttgttagg atggttgtaa gattagttgg tttgggaggg    1560
gataatgggt tgttgtgggt gtgtggtagt tggagatatt tttttgtagg gttaattttt    1620
gttttgttgt tttgttttgt aggggttttg gtgtttggtt atttggggtg tatataggtt    1680
atatagtgtt aagaggtttt agggtttagg gatttttttt atagtagggt gggatttggg    1740
atttgtggtt tagtggtttg ttagttatgt tagttaagtt attttaggtt tattttttaa    1800
ttttaatagt gtttttttat tttgtgtata agtttgagat ttggaaagaa taaaatattg    1860
aattgtagaa gagtagttat ggagtttgaa gtgttttttt tggtgtggga gtggttgagg    1920
tgatggtggg ggtgggagta ggtggttgtt gtgggaggtg gttaaggttg agtgaggaat    1980
tgtagttgta gtgggtggat gttttgttta gggttggatt ttgagttgga agatgaggtt    2040
agtgatagat aaaattaggg ggatttattt attttagtga tttgtttgag tttttttgaga   2100
attgaggttt ttagggttgg gggagtttga gtgtttatat gagtgtttta gggttattga    2160
ggttttttta taaataggga gttttgagtg gggtttattg tggaggagaa tgtaggtata    2220
gggtaggttg gggggtttga agtgttaggg gaggtgggag tggtagttgt ttattttttg    2280
gtgaggttgt gggtgtttgt gggtttttgg ggttggggtt ttgttttttt gtaggtttgg    2340
gtggggttgt tggtttgtgt gttgtttttt gtgtagtttt tgttttttgt agttttgttt    2400
tttgtggtgg ttttgttttt gttgttatag gaatagttgt ggtggtttgg tttgtgtgtt    2460
```

gtttgtttgt tttggttttt gttgttttag gggtgggtgg ggtgggttga gttggagttt 2520
gggttgttgt tgttgttgtt ggggagaggt tgaatgtgta gttggtgtgg gtttttgaga 2580
gagtgtggtt gttgggtggt tggtttttgt ttgggttgaa gttagggtga ggggtgtgtt 2640
ggggtggggg tggtggggga gggggtttta ggttgttgag aataaaggga gtgggggtag 2700
gggttgtggg gattgttttt atgttgtgtg tttgggttgt ggttgtgttt ttgtatttgt 2760
tgtgtgtggt ttaggttggg tgtggttttg ttttttggtt tttagtgttt ttatttgtgt 2820
gtatggttag agttgggttt ttgtgtggtg gtggtggtga tttggtgggt tgggtatggg 2880
tgtagtttgg ggattgaggt gtgggtgggg gatggggttg ggtatgttaa gggaggtggt 2940
ggtggtggtg gtaggagggg gtgaagtgga ggaggtgtgg gagggagggt gtagttttat 3000
ttttggtgtt tggggtgggt tttttttttt gtagggtgtg tggtgatgtg agttatgagt 3060
gtgatgtgga tgttgttgtt ggagttttg ttgttgttga tggggttttt ggtgggtgtg 3120
ggtttggatg tggagtagtg tatggtgttt gtttttgtgt tttgtttgtt tgtggtgttg 3180
gtgttgttga tggtgtgttg tgtgtgtatt ttgtttgatt tttatagttg tatgtttgtt 3240
ttgttttgga ttgattataa ggaggtgttt gagtgtgggt agtttgatta tgtgttggtg 3300
tgaggggtgt ggtgtttttt agttggtttt gtgttggggt tttagagggt tggtttggtg 3360
gggggtgttg tggggttagg attgtgttta ggattttgtt ttggagtttt tagtttgggg 3420
gagaggggga gggagttggg agaagtttag tttttttttt ttgagatttt ttttatgttt 3480
tttttggtt tggttggagt tttattttgt gtttttattt gttttgtttt tttttttttg 3540
tgtattttat ttgtttggtt ggtttttatt tttgattttg aattttgggt gggaggagga 3600
tttttgtatt gttttttttt tagtgtgtaa gtggttttgt tattgtgtgt ttttggaagt 3660
ggggattttt ttgttttttt tttttttttt gttgtttggt tgttgttaat tttattttga 3720
ttgagtgttg agtttgtttt tgggttttgg tttttgtggg gtaggttttt ttgggtttat 3780
ggggtggatg ttgtatatta atttgttttg tattgtttgt tttggagatg gagtttgaag 3840
gtttgttatg gtggttgtgt tgaggatgtg tggtgtttgt tgtggtggga tagtgtgggt 3900
aggtttattt ttgtttttaa tttgggtttt aggataggtt agtgtgtttt gttttggtgt 3960
ttattgttgt tttttatttg ttttttgtt atttgttttt gattttttg ttgtgttttt 4020
tgtagttttt ttatgttgtt ttattttttt ttgtttttga gttgaatatg ataataaaag 4080
ttattattga gtgtttattg tatgttaagt agtgtgttga gttggtattt tttgtttata 4140
gtttgttttt tttttgggat tgtgagaatg agttaggttt tgttagtatt tttattttat 4200
aggttaggag attgagggtt aggtagtttt agtgattgat ttgtttatag ttatatggtt 4260
agagtttggt agggttggaa gtggaattta ggtttggttt ttgtatgttt ttaaagtttt 4320
ttagggttgt aaagatatta agattataag tattttttat tggttttggg gggggggtt 4380
gatgtgtaat ttttagttgt tttttttta tatataattt tgttgatttg gggaggtgtt 4440
gggggtggtg agagagattt agttttgtgg ggaattggga ggtgtatttt ttgttagttt 4500
tggagggagg ttttgatgat tgattgtatt tgtagtttaa gttttttttt tttgtttatt 4560
attattggta gtgtttgtgg gtttggtgtg gtggggtggt gattatggat ggagtgggtt 4620
ggttgtgttt taaggttttt ttaagagatg tgtattttag gggagttgtg tgtagtgtgt 4680
ggggggagga atgagtgttt ttataaaagg taggttgggt gaatgtatag gttttgagaa 4740
tggagaattt attgatttgg attattagtt gggtgtggga tgtggtgttg tgggaggata 4800
tttgtttttt tttttaggat ttgatttttg gtttatattt atagggtagg tgaagttgat 4860
ggaggggggtt ttaagtgggg ttttagtatt tttaggggtt tggggttagt tttgatttta 4920
gtattttttt ttttttgttt tttagggatt tttgaggttt taaagttatt ttaggggata 4980
ttttattttt attagttttt tgttatttgg ggtagtttat ttttatttat ttttttttaa 5040
ttgtttagtt tggggtttaa ttttttttat tgggttattt ttattttttg ttgtttttttt 5100
tttttatttt gtgtgatgtg attttgtttt attttgaaat ttatttttga tgtttgtttt 5160
taaggtgttt tgttggtttt ttttatttat atttgttagt tttatagtta ttaaggggtt 5220
ttttatttta ggttttatta tattagttag gtggtagttt tttttttgg tatatatttt 5280
tagttttgtg atttttaagt tttgatattt tagggttttt atttttattt ttaagttata 5340
ggtgaaagag aaagagaatg ataattttat ttatggttat agaatatttt tgggaagggg 5400
aaggatttgg tagtagggag taattatgat tggttagttg aagaaggtga ggatgttggt 5460
tttgaggtgt ataggaaggg ggaaaatgtg tgagggtttt tatggtgggt agtgttagtt 5520
atggtttttt agtagataat aaggtatagt ggggtttggg gattgggggga ggttagtttt 5580
gtttgtttgt ttttgatatt aggaggtttg tgagtggagt tttgtaattt ttatttaatt 5640
tttgtataat gggggtttta tttttgagat ttttaaagta tggggtattt tagaggtttg 5700
ttttatagtt gagtattatt ttaaggattt ttttttatga tgggattttt agaaagagtt 5760
agggttagtt gagtagaatt gaggggtagg gtggggggtt tggtgtggtt ttttgtttgt 5820
tatgtttttt atttggtagg ggtggttgtt tgatttagaa gttttagggg tattaggaat 5880
gggtttaggg gatagatttt agttgtttgt taaggtgatt ttagtttgtt gtgtttggtt 5940
tttttggtgg ttgggggggt tttttttggt gttttggagt tagttttatt tttttttgtt 6000
gtaggtttta ggtttttttt agtgggaatg ggttgggggg ttgttaggtg taggtatgtt 6060
tttgttatat gtttaattag ggttgtgtag ggtaggagtg gtagtttttt tggaagtgat 6120

atgagtaggt tgaggattat agtggatttt agtgatggat ggaggagagg ggtaagggga 6180
tatttttgtt tgttgttagg gatttttgat ttttttgttt ttgttttttt ttttattttt 6240
tttattagtt gttgagagtt tggggttgtg gttattttgg gagatagagg ataagtattt 6300
gtagattttt ttaaagttta tagtaaagtt ttttttagtg gagggattag ttgatgattt 6360
agaaggtttt ttaggggtag ggttaggagt tttgggtgtg gagttaggtg tagtaggggt 6420
agagttaggt attttagggg tggggttttt ttgtttttta ggggtgttta ggagtaaagg 6480
ttttttatgg gtttgtgtgg gtttgggggg tttggagtgt aggagggtga ttggttttgt 6540
ggttgtgatg gtgagtattt ggtaggtaga ataattaggg aggaagagga gttgggaagt 6600
tttttttattg tttatatatt tttttatttt ttttgttatt ttaagatgtt ttttagataa 6660
gagatggttt tagagttagg gatattgagg tttgaggtgt ttagtgattg atggagttta 6720
tagagttggt aaaagggttt aggatttagg tttatatgat ttggtttatg tgattttgtt 6780
ttttgggata ttttgatttg tttgggggaa tggtggtatt tgtgagaaag ttattgttag 6840
ggttttttttt agtggttttg ttattttttt agttagattt gtttagattt gagtgaagtt 6900
agaggtatag ggttagttta gggattagtt tgtaatggtt ttttaagttt tgtttttttt 6960
ggggattttt taattaggtt atttttgta atttttattt atttattagg gatttgtttt 7020
ggatagtttt attttgttta tttgttttat ttttatgggg gttgggggtt gtgtttttttg 7080
gtgttttggt ttttgaaggt tatttggatg tatttttttga atggtttttt gggttatgtg 7140
gtttttgagt tgttggagga agatttggat ttagaaagga aagaggtggt ggaatgaggt 7200
ttttatgagt ttagggttgt agtagagaag ttaaagttta attttttgta tgttttgggt 7260
tttgttattg tgttttagtt ttttgttggt ttggtgttag gagttttagt gattgttaag 7320
gtttttattt tatttagagt gtgattagtt atttaaggaa gttgtaaggt ttaggttagt 7380
tttggttttt ttatgtagtt agggtttaat tttatgttga ttattatttt gtttaatttt 7440
ttttttttgtt ttttttttt tattttttttt tattttaagt ttgtattttg tttaatagta 7500
tgtgttgttt tttgtttgga tttggtagta ttttattttt agtttatttt gtttgtttag 7560
tttttgtttt atttatgtat tattttgagg tttgttttgt ttttaaagtt tgttttttatt 7620
ttgtttatgt ttttttttatg ttttattttt aaaatggttt tgtttttaag ttttgttatt 7680
aatttgttta tgttttgttt ttaggtttgt tttgtttttt aaatttattt atttattttt 7740
taaattttag tggtatgtat gttttgtttt tggtattgtt ttgttttttt gttttattta 7800
tgttttgttt atgtttagtt tttaagtttg ttttgttttt aggttttttt tttttgttta 7860
tgttttgttt tttattttgt ttaggtttat ttattaatgt tttgttattt agttttatgt 7920
tatgtttttg tttgtttata tttttggttt tgttttgttg gtttttgttg tagttttgtt 7980
tattttttgt tttattttag ttttgttttt agtttggttt tggttttgag aattgtattt 8040
tattttagtt ttgttttggt tttgtagttt ttgggttagt ttggtggtgt ttggttttgg 8100
ttggttttgt tgtgtttgta ggagttgtat tagttgttgt tttttgtgag tgttttaggt 8160
tgtgggattg gttattttgt ttagatagtg tgttggggtt gtttgttgtt tgggaggtgg 8220
ttttatgttg tttagaaaga ggtaggtgtt gtaaggttgt ttttttgatg ggtggagtta 8280
tgggggttgt tgaggttaga aagaattgtg tggaggtgga tgtgtgtggg ggtgtggttt 8340
tgtgtttgga ggtttgggga attgttttaa tggaagtgaa ggggagattg gtattggaat 8400
tggggtgggg tgaaggagat ttttagtttg tattttattt tatttatgaa gtggagatga 8460
tattttggat tttatgtttt ttttttgttg ttggattaat ttatgaagtt attattgtgt 8520
gttttgtttt ttgtagaggg gtgggttttg gttgaaaggt taagtttgaa gatttagttg 8580
gttaagagtt tgggttttgg ggttagatag gtttggggat tttgggtaag tgatttagtt 8640
ttgttgagtt ttagtttttt tttttgtaga ttggggttga tagttttatt tttgtttgtg 8700
taaggtagag tggagtttgg gtaggaggga tttaaggagt tttagattag attgataatt 8760
taagaggagg gtgaagttta gattaggagg gaattggagt agaggtagaa ggttgaagtg 8820
gagttagggt agggtttggg tgggggtaat ggagagtgga gaaggtttgg tttgagaaag 8880
attgtaggag gtagatgggt aggtgtttgg gtaaggagag gagggagggg gaaggtttat 8940
tttaaattta tgtttgtggg gttgggtgtt gtttttttttt ttttttttaga tagatttttg 9000
tttttgttgt ttaggttgta gtgtaatggt gtgattttgg tttattgtaa tttttgtttt 9060
ttgggtttaa gtgatttttt tgttttagtt tttttagtag ttgggattat aagtatgtgt 9120
tattatgttt tgttaatttt ttttttgtta ttttttttttag tagggatggg gttttattat 9180
gttggttatg ttggtttgga atttttgatt ttaagtgatt tatttatttt ggttttttaa 9240
tgtgttggga ttataggtgg gagttattgt gtttggttgg gtgttgtttt ttgtttgaga 9300
taaaatgttt tatagttttt tatggtttgg gagtgtatat gggtatatgg gtatatgggt 9360
tttgttttta ggttttattt ttgtaagtta tgatttttta ggaatatttt ttaagttttg 9420
tttttagttt tttttttaag ttttttttatt tattttttttat tttgtatgtt gtgtgtttat 9480
tgattgtatt agaatttagt tggttagttt agaggtttat tttgagtata ggtgggaggg 9540
atagggggag gagagaagtg taggggttgt ggttttatta ggtttgaggt ttagggaggt 9600
gggtttggtt ttgttggttt tggttttgaa ttggtagttg ggtgtatttt tggattatgt 9660
tggggtgta ttatttttgg gaggggtggt tgaggtttag tgagggaggg gatgttatgt 9720
gggttatttt ttttttttagt agattattag ttttttttttat tggtttgtat gttggggtga 9780

gagtgggagt tgggtttgaa tgttgagttt tttttgttta gtgttttttt ttatttaatg 9840
tttttggttt gatttaaagt ttgtgtttgt tgtgggtgat ttattgtttg agtttgaatt 9900
taagttttat ttagtattgt gatttttgat aagttattta atttagagaa tgttttatta 9960
tttttgtttt taaaatgggg attataatag tgtttgttta gaggttaagg ggattaagtt 10020
agtttatagg tgtaaaatat ttataatagt gtttggaggt tgggtgtagt ggtttatgtt 10080
tgtaatttta gtattttggg agattgaagt ggtaggatta tttgaggttg ggtgtgtgag 10140
attagtttga ttaatatgga gaaattttgt ttttattaaa aatataaaat tagttgggtg 10200
tggtggtgta tgtttgtaat tttagttatt taggaggttg aggtaggaga attgtttgaa 10260
tttgggaggt agaggttgtg gtgagttgag attgtgttat tgtatttttg tttgggtaat 10320
aagagtgaaa ttttgtttta aaaaataaat aaataaataa ataaataaat aaaaaaatta 10380
tagtgtttgg tatgaattaa gtgttaggaa gtgttattgt tagatttttt attttattta 10440
gtttttttta tttttatatt tatttttttt attgggattt tttgggttgg ggattgttgg 10500
gtgaggtttt tgtgggtggg ttaggggtgt ggggtgttgg aagggagagg tgagttgtag 10560
aagggttgtt gtgtgttatg ttaggttaag ggtggttttt gggggttgtt gtgggtattg 10620
tggaggtagg tttagttagg gttgattagg ttttgtaggt gggattgtgg ttgttttggg 10680
ttttgatgtg tgggattaga ttggggggttt ggtttggtgg aggttgttgg tatttgagtg 10740
tgtatagatg agatttagtt tggtggttag tgtggtgttt agtatgaagg ttgttaatat 10800
tagtgttatt attggtgtgg gttttagggt tgtgggggtt ggtgtgggag gtttagggtg 10860
tattgtatgg ttggggatat ttttgggtgg ttttgtggga agtgttatgt tgagattggg 10920
tttagttttt aggtgttttt tttatttgtt ttgttgtttt atggttttat gtggaggagg 10980
attgtgtgtt tattgggggg tggttgtggt atggtgatta tgataggttg tgttagtgtg 11040
tgtaggtggg ggttgttagt agttaggttt ttggtgttgt tattgttagt gttggtgtat 11100
gtttgtttt gaggtagata ttgggggaga ggaaggtggg gaagagatgg ggatattagg 11160
gtattgggat tgtggttttg gtttttaggg tttttaagag agatttgagt ttagtttttt 11220
attatagtta agatgggatt tttggaaatt tggtttttta gggtgttttg agtttagttt 11280
ttatgtatat aagtggttag ttgtttagtt tttaaggatt ttaaatttta gttttataag 11340
attgtatggt ttgtgttttt tagtatttag tatgtgaggt ttagtattag gtttttagaa 11400
atgtgttttt ttagtggttt tttgtattta atttttagtt ttgaaggatt ttagagttgt 11460
ttatgggggga atttagtgtt tatattgttt ttggagaaat tttgttggta gagatggatt 11520
tttgtgtttt attttttttta atttttagat ttttagattt tgagagtatt ttttttttat 11580
tgttagagtt gtttgtgttt agtttttgaa gggttttagt gtttggattt taggttttgt 11640
gtttgtgtat ttgtgatggt ggtggtggtg gtggtggtgt tagaggtgta ggtgtttggt 11700
ggattttttg gaggtggtgg tagtggttta gttggtagtg taggaattgt attgaggtgt 11760
tgaagattgg gtgtaggtgg aagttgaaat gtgtggggtg ggtggtattt gggtttggtg 11820
gtggtggtgg tgggaaggtg atagaggtgt tggtggggta gtttttatgt agtagagtta 11880
gggtgggttt tggggttggg tgtgaggatg gtgttattga gtagtggtgt agggttaggt 11940
tttagtgtgg ggagggatgg gttaaggttg tttgggggat ggaggtgtta ttagggattg 12000
tttgttttta ttggttttta tttgttttttg gtgtttttgg ggtttttatt tgtatgaata 12060
tgtggttgtt ggttgggatt ttgagtggtt ttgtgtggtg aggaaagatg ttttttgtgt 12120
tggatagttt taggttgaag aggggtttgt gtagttaggg gttgggtgtt gtggggaatg 12180
ggggagttgg tggggtgggg aagggtggtt taaaggagag aaaatgtgtg ttgtttttta 12240
ggtttttggg aaaagtgatt tgattatttt gtttttgttg ttgtttttgg aaaagggatg 12300
tggttgtggt ggttgattta tttatgatgg gtttgtgtt atttttttttt ttgatggtga 12360
tgtgtttttt ggtgattttt gggatttgta tagtttagta attgagttgg ttgtagagtt 12420
atgtgttttt agagaagttt ttgaagttgg tgaagttttt tttggggtgg aagagaagtg 12480
gtgaggagta tttgatggtg gggtttggtt gggtgaagtt agagtttttt tattttttttt 12540
agggttgttt ttggggatgt agtaggatag gtggtttttt tttttaaggg gagataggtg 12600
ttgttattaa tgaggaaggg aagtgaagag gatatttttt taagaggttt ttttgggtat 12660
agttaaagtt tagttaggtt attaagattt gagatgtttt tatttgttta ggttgaggta 12720
gttatgggat tttttttttt tttgtgtttt taaatatatg ttgatgtttg tttttaggtg 12780
ttgagtgggg ttttggtaaa atggttaatt tttaaatatg aaagattgtt tgtatattta 12840
tttttttttgt tttttgagat agggtttttt tttgttgttt aggttggagt gtagtggtgt 12900
gaatatagtt tattgtagtt tttatttttt gggttttagt tatttttttg ttttagtttt 12960
ttgagtagtt atgattatag ttatttgtta ttatggttat atggggttta tgttgtttag 13020
gttggtttgg aatttttggt tttaagtgat ttttttattt tagttttttt taagtgttgg 13080
gattataggt atgagttttt ttgtttattt gtatattata tttatttttg aagagtattt 13140
gggtagattg gttttttttt ataatttgg tttttttttgt ggttgtaggg agttttgttg 13200
aagtggggat gtgagttatt tttgggatgg gggatagaga ttttatttta ttttttttgtt 13260
ttgtttttat ttttatgagg attattatta aaggtttagt tgaaagagag tgatattggt 13320
ggggtgtggt ggtttatatt tgtaatttta gtattttgaa aagtttgagg tgggtggatt 13380
gtttgaggtt aggagtttga gattagtttg gttaatatgg ggaaatgttg tttttattaa 13440

```
aaatataata attagttagg tgtggtggtg tgtgtttgta gttttagtta tttaggaggt   13500
tgaggtagga gaattatttg aattggggag gtggaggttg tggtgagtgg agattgtgtt   13560
attgtatttt agtttgtgtg gtagagtaag attttatttt aaaaaaaata aagagggtga   13620
aatagggata gaagtatgat agatttgtta tttttttgtt tatatatatt aggtggtttg   13680
ttgggtaagg tggtgatttt tgggagtagg gttagtagta gtatggtgtt tagtatgttg   13740
gattagttga ggttggagtg tttggggtta gagtagatat tagttgggtt gtatttaggt   13800
tttgtatgtg ggtaggtggt tttgtttttg gtaaggaggg aggtagggag tgggggtggt   13860
agattgaggg gttgatatta gagttaatgt ttaaggtttg aggggattaa gggatttttt   13920
gttgtttgat ttgtggttat tgggggtagg gggtttttta tattagttgt ttgatattta   13980
ttggtaagtt attataaata tttttgtttt agtttttaaa tttgttttag ttttttttta   14040
atgtagaaat ttttttttgt aattattttt tttttatttt gttttgttag gtttatgata   14100
tattatagat ttggtggaag aaaaggaagg ggtttatttt taagtgttta agggtttata   14160
aatgagggta ttttgtttta aaaaggggta gggtgatatt ggtggtttga ggaggggttt   14220
attggttggt gggttggttt gagttatttt taggtttttg tttatttggt ttgttttttg   14280
tttggtttag agggatggtt ggtgatgagg ggggaggttt tgggagaggt tgggaggtag   14340
gagagatttt tttagggttt tttatttaat attgaagggg gtgggggtgg gtggatatat   14400
atttataaaa tagaaaataa agttaaataa aaataaaatt aggtagtttg gttttgggtt   14460
tgtgttggtt ttgggattag ttatgtaatt ttttggtaat tgtagaggtt tttatggttg   14520

gatgtagggg aggaaagggt taggtttgtt ggtatttttta tttggtagat ttttggtttt  14580
tgataaaggg gttaaggggt aagagaaggt ggtttttttt tttgggggta gagagaagat   14640
gatatttatg atttttttaaa tatgtggtgt tttgtgaata ttatgataat aataagagtt  14700
aataggttaa gtgagaggag agggatgtta tgtttatgtt gtttttatta tggttagttg   14760
gtttttgtgt ttttggtgtt ggattttttt tgtgatttta tggggttttg tgtttttttt   14820
tttttgtttt ttgttttgtt attttttttt gtttatttga tttgtatgta atatttataa   14880
aaaggaaata tatttttttt tgttttggtt attttagtag gttttggggg gtatttggtt   14940
tgggtttttg gtgtatttat ttggtttttt ttagttttgg attttaatgt ggattgggtt   15000
aggtgtgggt gagtggaggg atggttaggg gtatgatggg agtgattatt ttgtattttt   15060
atttttagtt tggtttttat ttttgttgga gtttgttata tgtttttaaa tttttatatt   15120
tattttttttt gtttttttatt tttgattttt tttggatttt ttttttggtt ttgttttgtt  15180
attgattttt tttggttatt tgttttgtat tatgttttaa gattattttt tttgaaggtt   15240
attttgtgtg gttagggttt tgtagatttt tttatatttt ttgaggtttt taatttattt   15300
taagttgtgg tttttggaga ttttagagta gtagttttag tttagttttt tagtgtttga   15360
gggtggttgg ggttttatta taagtagtta attgtgattt tggtaaaagt ttttgaaggt   15420
attgataatt ttatgagaat aagttttttt ttgtttttat tttaaatatt ttattaagta   15480
taataaaata taataaattt taaaaaggat tttttgagat taaagagaga gagagagaga   15540
gagagagagt gtgagtgtgt agttttgttt agttgtagtg gtaatgttta tattgtttgg   15600
ttgggggtta ttgatttatt gtgttatggg agatttatgt agatttaggg gatttgttag   15660
gaggtggttg ggtttaggtt taggttagaa gagagataga ggtagggagg gtgatgggag   15720
agttagataa ggatagagag gtagagtagg ggatagaggg tgatggaaga ggaatgggtg   15780
tgtgggagtg gtgaaggata tagtatgttg ggagtgtgta tggtggttgt gggattttag   15840
ggtttttttt tggtttttgg gggttgtttt ggtttagagg ttgtggttga atgaatagtg   15900
attttgattg tggtttgttg tttttttgttt atagtgtttg ttgggggatg tatatggttg  15960
gttgattttg gggtgggaag ggtgggggtt tggtaggttt ggggtggggg gggtgggtgg   16020
gtattttttta tttttttgttt ttagttttag gtggttgttt aggttttagg tttttttttgg 16080
taggtagttg ggtggttaag tggggagggg ggtttgtggt tatgtttttg gtttttttgtg  16140
gggttggttg ttgttaagta gttatttgaa gaagggtagg tggggttggt ttaggatgtt   16200
gttagtttgt ggtgatttag tttttgttat gatatttttg aattaggatg ttatgtttat   16260
ttttagtgtt ttgtagtgtt tgatgaagtt gtgtttttgt gggtttgagg gggtggggtt   16320
taggaggaga tggttttttt ttttggtttt gtgtttttgt agggatagga tggggattgg   16380
gagggtttag gtatttataa gtagtttatt atattttggt ttttttttgt gatttttagt   16440
aaggttagag taagagaagg gaagatagat gtgagaatga gggaggaggg gatgttggtt   16500
ttattttttgt gtttaggttt tttttaaggg gttaggggtt tttaagatgt gtgtggggga  16560
aggagttgtg gatgttttta ggattttggt tgatagtggg gagtaattgt gagttttgga   16620
gtttttttggt ttagggttag gtggtaaggg tttggtttgg ggggttagag ttggttgagg  16680
tttttttagaa aggtgggtgg ggtttttttg agagaattga atatggtttt tttgaatttg  16740
tagttgtgtt tgttgtagaa gtgtgtttat aatttttaat agttattttg ttaaatgtta   16800
tagggttggt tgtttttttta ggttaggttt aaatttagtt tatggttttt tgggtattag  16860
ttttagtttt tgtggtagtt gttagttagg agtttgttta tagttgtatt tgtttatttt   16920
agggtgtggt ttttattttt tttttgttat tgtttttttta gttgtttaga gtagtgggtt  16980
gtttttgatt ttttaagtgg agggaggttt ttattagttt ttgatgaagg attggaagtt   17040
```

```
ttttgagaag tttatgtgtt tgggtagaag tgggggtttt ttttgtagga ttttggtgag   17100
gattttaaag tttgttttgt agtttttgta gggaaattgt tttgttgtta gttttatttg   17160
ggagggagat gggtagggtt ggataggttt atgttttttt ttttttgagt tttttagaag   17220
tttttatttt tggttggaga atagggggagg gttttaattt attaatgaga tgtttaggtt   17280
ttatatgttg gtttggatgt tatagtttgg tttggtgggg tttgggggggt taatgtgttt   17340
gggttgtagg gggggggagga gaagatgtat ttttaaggtt ggggttggtt ttttagtttt   17400
tatttttttt tttgttgggg ggtttttatt tattgttatg taggtggtat agttggtgtt   17460
ttgtgttttg gttttggagt ttattaggtg gttgttgatg ttgaagttgt agagtttgat   17520
ttggttttgt ttgtttagta ggatgttgga gggtttgatg ttgtggtgga tgatattgtg   17580
tttttttttt aggtagtata gtgtttttat aatttgtagg tatgtagagg ggggtgtatg   17640
tggtaggggg tatagattta aggttatttg tttggtgtat gttttatttg gttatttatt   17700
gttattgtta ttttgtttag tgtatgtttt gtttggttat ttattgtttt tgttattttg   17760
tttagtgtaa gttttgtttg gttatttatt gttattgtta ttttgtttag aatgtgtttg   17820
gggatggggt tttgtatttg ttttttgagt tttttagtgt aggtgtttat gagttttatg   17880
gtgatgaaga tgtttgtttg tagggagagg ggtatttggg ggagttgtgt tttttggttt   17940
atttttttaga ggttttgttt tttaggaagg ggtaggggtg agggaggagg agatgttgta   18000
gggtgtggtt aggtatttat gttggtgatg aatgttttaa agtattgtat gatgtagggg   18060
tagttgtggt tttttagtat tatatttagg tttatgagga tgtgtttgtt tttttttttg   18120
tttttggagt gttgtatttg ttgtataggt agggatagga gggagtttta gtattggttt   18180
ggggtgtggg gtgtagttgg ggtagttgtt aaggtttatt ttaatggtaa tgatgtggtt   18240
ggtttttttgg aagtgtattt tttatatttg gttgtaggtg ttgttgttta ttttgtttaa   18300
gtttttttagg ttgttgattt ttgtttggta gtgttgggag gagagggagg gttggagggt   18360
ttttggggtt tttgttattg tttttttgttt tttattggtg tttttaggat gggtttagtt   18420
tggttgggtt tttttttttt ttttgttata gtgaaggtgg tatttggttt ttgatggtta   18480
ggtagtttgt ttgttttatg atttttttgta gtttttggtt aattttaatg ttggggatat   18540
ggagtaggtg ttggtggagg tgggttttga ttttaggttg gttattttag ttttttatat   18600
tggggttttt attggtttaa ttattttttt tttagggttg gtatggtggt tttttaatgt   18660
tgtttttttat ttaattttgt tgggtttttg gtttattttt ttatgttgtg gggtgtgaat   18720
agggttgatg ggagttttag tatgtggtgg ggttgggtgg ggggtgtggg gtgttgtggg   18780
gagtttttttg aggatggtga gtggttgttt ttattgttgg ttagtgggag ttgtagggtt   18840
agggggggtga gaattagtgg tagggagggg ggttagtgtt tagttggtgt tagttttata   18900
tttggtattt tagggagggg ttgggatttt tatttttttt ttttgggttt tttgagttag   18960
gttggtatag tttttttgttt tgttgggttg gaggagttgg ggtttttttt ttattttaga   19020
ttgtttttttag ggtttttttgt tttatttttt gatttggaaa gagatggagg aggttagtag   19080
gggagaggga gttttaagtt tatgttgatg gagaaatgag atggggattt gaggtgtttt   19140
ttggttaggg ggttttttttg ataggtgtgt gtattttttt ttatttttgt ttaggaagta   19200
ggtttagggt tatagtattt ataggatgga taggttaggg tggagtgttt gttagggttg   19260
gtttaggttg tggttttgtt ggtagtattg aagttggttt ttagataaga gtgagtaggg   19320
ttatgttggg gaggtagtgg gtatgagatt taggttttag gttgttttgt tttatttttt   19380
gttttggggt tagggagggt tttttttggg ttagtgggtt gatatgaggg ttttttggtt   19440
tggtttgtag agttggggtt tatgggggta ggataggatt ttagggggtt tgtatttttt   19500
gggaggagag tttgggggta tagtttttgt aggggttggt gggattaggt ggggttaatg   19560
ttgggttagg tgggggttgg tttatttagt agtttgtatg tgggtagttt gggtatgtta   19620
tagtgggtgg gtaggtggtg ggtaggtagt aggtattttg gggtgggtag gtggtattag   19680
gggtgggagg gtaggagata gatagataga taggaattaa gattagggtt taggtgttta   19740
gaggaggtgt tgtagagata ggttagagag gggtttggga taggagggta gtttttttgga   19800
ggagttaggg tttttttggt tgggttggta atagggtgta ggaatagagg ttgggatgtt   19860
aatgtggttt ttagaggagt taattttgat atgttatatt ttagaggttt tgataatggt   19920
tatatatatt tattaaggtg ttttttttat aggagttggg gtttgttgtt ttttaaagtt   19980
tttagttaat aagttttttt tttttttttt ttttttgttt tgtttatttt ttttttttat   20040
agttttgtag gtttttggtg attggtgttt agggattaat atgaatagat aaatttgttt   20100
tatgttggta gttaaatttt gttttttttg tgtgtttagg attttgattt tagttagtag   20160
gaggggtagg aagatgtttt agttttaatg taggaggttt agtattgttt ttatttggtt   20220
ttgttagttt ttgtaggagt tgtgtttttt agtttttttt ttggaggatt aggtaggagt   20280
aggggaaggg ttttggggat tttgggatat agttaggttt agtgtttatg attggtttag   20340
aggttttatt ttttgtgttt tttttgttgt tttggttatt gttgttttttg tagtattatg   20400
ttatttttta ttgttttttt tttttttttga gataggtttt tgttttgtta tttaggttgg   20460
agtgtagtgg tgtgatttta atttattgta atattttttt tttaggttta agtgattttt   20520
ttgttttagt tttttgagta gttgggatta taggtatatg ttattatatt tagttaattt   20580
ttgtttttttg agtagagatg gggtttgtt atgttggtta ggtttttttt tttgtttttt   20640
taggtttttg tgtttttgtt ttgtttatgg tttgagttat agttttagtt tttttttggt   20700
```

```
ttttgagggt ttgtgttttt tttaatttag tttttaattt ttgtgtattt ggtatttttt   20760
gagatattgt ttagtttttg tttttagaga gtatttgagt tttgtttata ttttgtattt   20820
agtggttgat gattatagtt tttgaggagt tttgtgggtt ttttttttga tagagtttat   20880
taggtttttt gagggtggtt ttggagtttg gggaggaggt tgttggatta tagattggag   20940
tatgggggga tggaagggag ggtttaggta ggtattgggg gaaggaggta tataaaggtt   21000
ttgagaatag aggttataga ggtggtgatg gttatatggt agtttaggag ggtggagtga   21060
gagtaagagg gtgaggatga gaaagagaat ggatgagaga gagtgagagg ttggagggta   21120
tgtgtttgtt gataggagtt ataggtgtat tggttgttat gttaatatgt atgtatatgt   21180
tgtgtaagtg tatgtatgta taatatatat ttggggtatg ttgtggattt aatatttatg   21240
tagttgggat agagtttttt ttgtttttgg atatatatat ttataattat ttataggggtt   21300
aggtatttta tttatttttt tttttaatat aggtgtgggg tatagtttgt atgttttata   21360
taggttggtg ttgtgtttag agttttggtg gttttgtttt tttttttttt ttaatttttt   21420
taagatgttt tttaaatttt tttttttttt gagatggagt tttgttttgt tgtttaggtt   21480
ggaatgtagt ggtgtgattt tagtttattg taatttttat ttaatttttt atgttttttt   21540
ttttttgag atggagtttt gtttttgttg tttaggttga agtgtaatgg tgtgatttta   21600
gtttattgta atttttgttt tttgggttta agtggttttt ttgttttagt tttttgagta   21660
gttaggatta tagtttttttg agtagttggg attataggta tgtgttatta tgtttggtta   21720
attttgtatt tttagtggag atggggtttt tttatgttgg ttagggtggt tttgaatttt   21780
tgattttagg tgagttattt gttttggttt tttaaagtgt tggaattata ggtgtgagtt   21840
attgtgtttg gtttaatttt tagtattttt aatgtagata gggttttatt atgttggtta   21900
ggttggtttt gaatttttga ttttaagtga tttatttttt ttggtttttt aaagtattga   21960
aattataggt atgagttatt gtgtttggtt tagtttttaa atttttgaga taggattttg   22020
ttgttattta ggttggggtg tagtggtgtg attatggttt tttgtagttt taattttttt   22080
gagtgttggg attataggtg tgagttattg tgtttggttt ttttagaggt tttgtttaat   22140
ttttattgtt tttagaggtt tttttaggtt ggttttttgt agggttgttt atttagttag   22200
aatgtttttt ttggtgggaa tgtgtttttt gtttagtggg tttttttatt gtagtttttt   22260
gttatagagg agaggggttt tatttgtttt atttattgtt gtgtttttgg tgtttagggt   22320
tggtatggaa tttgggggggt gttatgtgtt tattt                             22355
```

<210> 305
<211> 11000
<212> DNA
<213> Artificial Sequence

<220>
<223> chemically treated genomic DNA (Homo sapiens)

<400> 305

```
gaaaggtgag tgttggttga attttatttg attttagtta ttttttagga gtggtttggt      60
gtagattata atatttgtta tttatgtatt tatgtatttt tttttgaaag gagttgtaga     120
gtttttggaa ttttattttt tagtgaggaa aattttttag atttttattg ttttttggag     180
ggtgggggta gggttggtga aaatatgaga aagttttttt ggtgtatttt atttgttttt     240
atgtgtaaat agttgtatgg tttttttttt attaatttta ttagaaggta tattagtaat     300
aattaaggat gttataggaa aatgggaagg ttgttttgta gattgtgtgt tttgtataat     360
gatttgggga gtgaatgggg gagtatgttt ggttttggtt ttggttgtta taagatgaga     420
ataattagta aattttttttt tattatttaa ttttgtgtgg taataaaatg gatttttttt     480
tatgaatgtt tggttggttt atttattatt ttttttttat tagtaatttt ttgttttgta     540
tttagttttt taattttttta tttttttttg ttttttagtg tatatagtgt tttttatgtt     600
aggttgggtt tgtttttatt ttgtaattat aaaggttatt aaagtaatta ttgttggttt     660
tgattggaaa agttggttat taattagttt tttttttgttt ttggtgtagt ggattagttg     720
gtgttgggat tgagttaatg gaaatgtggg ttaaatgaga aaagatgttt ggattttttt     780
gtttggatgt gtggtttgga ggaggattgt gaggtttttt agtggtggga ggtggtggag     840
ttgttgtgga gagggaggga ggttttggag agggagggaa ggaaaggggg agggagaggg     900
aaagggggga gagggagagg aggtgtgggg tgggggaggg gagtgagtag ggagttggga     960
gagggaggag gggtgggaat taggggagtg gtttgaattt tgtttggttt ggattttgta    1020
gttattgttg ggttttgttg ttgggttgtt ttttgtgtgg agatttggtt tgtgtttttt    1080
ttttggtttt tttttttttt tttttttttg tttttttttt tttggtttat atagtttttt    1140
ttagaaagaa gttattttag agttgtgtga tttagtttta gagtttgttg ggtttgttt     1200
attgggtttt ttgtgtgttt ttttttgttt ttttttgggt atagtttgtt tatgaaattt    1260
aaggtgttgg ttatgtgtta tttttttttgg gttggggttt ttttggtttt tgtgtgggtg    1320
```

ttggtttttt tgggtgggtg gtagttttgt tttgtgtttt tttgggttgg tgtgagggat 1380
tttagagtgt gtggggggtt ttaggtgggg aggagtggtt gtgattttgg atgtgagagg 1440
tgtgtgggtt taagtggtgt tgggataggg gttgggtttt ttgtttattt tagggtttat 1500
gtttgtttgt ggggttgtgt tttttggagt tatatttggg tgggagggtt ttttttgggg 1560
gtttggtttg tgttttttgt tgttttttgt tttttgtttt ttgtttttttg tgggttttgt 1620
ttggttttgg ttgatggaat aagaataata aaggaagtgg tgtgattggt ggagggggtg 1680
tttgttgttt ttttattttt tattttggtt gggttgatgg tttttgtgtg tttttatttg 1740
ttttagtgtg gaggtgattt tggttttgtt gttttggtgt tgggaggata ttgtgtgttt 1800
atgtggatga ttgtgtttgg gaatagggat tttttgaatt tttttgtttt gtgttgtgag 1860
gtttatatgt gtgtggtgat tattttggtt ttgagggggt ggtggggtta tggtgtgtgg 1920
tggatttggt agtggtattt aggttgtgtg tgtgggtgtg tggggttttg aggtgtaggg 1980
tgattgttgg ttttggtttt ttgaggaagg ggtataggtg ttgtttgtgg ttatttgggt 2040
taggtgaggg taggggatgt gtgggggttg gaggaggagg tttggtttgt gggtggtagg 2100
agggatttgg ggttagttga ggttgttttt agggaggtag atatttgttg ttgttgggat 2160
ttttgatatg ttttgtatgt gtgggagtgg aattgggttt gttttggagg ttttttttgg 2220
tgtgtttgga tttatttaaa ggttaaagaa aaatgttaag gagagtgatt gtttggagag 2280
tttttggttt tttttttggg tttttgttgg gttttaggag tttttggagg ggaagtaagg 2340
agttttagtg ttttggagtt ttgtttttat taattttgtt tggtttttttg ttgtttaggt 2400
ttgtgttgtt ttagtagagg aggggtggga gttgttttag gtttattttt ttgtgttggt 2460
ttataaggag gtttttttttg taatttggga gaggttgggg gaggtttgga gggtgattgt 2520
gtggggttgt gggatttgga tagttgggga gttgtgattg tgggtagtgt tggggttgag 2580
ggtgaggagg gttttattgt agggaagggt gggatgttgg atagagggat tgattggggt 2640
ttattggttt gatatagaag ttgttggatt ttagggtttg gttgggaaat ttgtttttta 2700
tttgtttgtg ggtgttgtag aagaaattga agaaagaggt ttttttttta tgtttaggat 2760
ggttttaagt gttttattta gtgttgagta ggagggtgat gtggagggaa aaattgttgg 2820
ttgtaagtta ggttttagga attttggaag aaatgaagat ttaaagaatt ttttttttaag 2880
tgattgagtt ttaataattt tataatgatt tgattttttt tttgtggagt taaaaaaaat 2940
ttttaaattt ttgttagtta tggtttttaa gagaaagaaa tgagtttaaa agtgagtttg 3000
aggtatagtt ttgatttagg tgtggttttg gtggttttta gtgttatttt ttttatgggt 3060
attgattttt taaggttgta ttatggtggg tggattttaa agagagttta gattaattag 3120
aggtatttta ttagttttga tttgaaattg aaagaaaatt atgtttaaat atttatgatt 3180
ttgatattta tagttaggtg tttttgaaaa gtggaatatt ttttttttgta tagtggtagt 3240
ttaatgattg aaagtttgga atagagaaga agtgaggagg aggaggaggg agaaggaggt 3300
ttggtaatat agtggtaggg gtgtgttttg ggtgggggag atgtatagat aggtatattt 3360
tagtgttgag tttttagagg taggaatagt ataatttaaa aaaataatta ggaatgatta 3420
agttaatttt attttaggtt agatttattt tgagaataag atgaagtata atattgtata 3480
tttttttttt ttaattgttt gtatttgtaa ttttttaaat ttagaataga tagtattgta 3540
aaagttgtga tttaaaataa gtgtatgatt atagagataa tttgtggagt tgttttagaa 3600
tttagtattt gtagatattg ttttgattaa tttaaattat tttgattgtg tttttgagtg 3660
gaggtgtatg aggtttttat tttatgtttt tgtgagttat ttatgggaaa atgtttttag 3720
aatttttttta gttgtttatg tggtaagttt aaaagaaaag aaaaaaggat atgttatgtt 3780
taaatttaat tgtgagagtt gatatagtta tataaatata aaattttatg gtaaatatgt 3840
gttgatgggt agttagattt agtttttttt taaggttgtg tgatataatt agttaatagg 3900
agtagtttat tttttgagtt atgatgtgta gtattaaaat atgtttgtat tataaagatg 3960
tagggatata ggtgtttggt gttagttaat gttaaatttt taatattgtt gatgttttta 4020
tttgtattat ttttaaggaa ttagatttta gttttagttt tattttattt ttgtgtgtat 4080
ttaaattatt aaattataaa tatttttttt ttttaaaatt tagaagttag ttttagagat 4140
tattaatgag ttgttgtaat aagtttattt ttggtttgtt ggatgaattt ttttagaatt 4200
attaggaaat tttattttaa atttattaaa aggtattatt aaaatataat atttatttta 4260
gggttgatga atattttaag tttttaggtt atagtaagtg atttaatttg aaaatatggg 4320
ggaaggggtg ttttaaaata ttttaaaagt attttttaaa gttgttaatg gaattatttt 4380
taatttggaa tttgttatag aattttaata aatgagagaa gttaggtgga aataattatg 4440
tattaatttt ttgtattata aaattttttt agaagttatt attagagtaa gaggtattgt 4500
ggtttgaatt tagggtggga gaagtttttt gtgtaattga aagttattgt ttttttgtgt 4560
agatgaaatt gtatttgtaa atttgttatt taagtggtta aaaaatttaa aaaagtaaaa 4620
gaaaagaatt tttaaggaaa aattattttt aaatgtaaat tttaatgaaa ataagttggg 4680
atatattgtt ttaaaatggg ttatttgttg agtttttttta ggtatatatt taaaagtgaa 4740
tatataagaa aggtaataaa ttgtttattt gttattttta agtattttta aaatattagt 4800
tttaaagaaa tattgtgttt taagatatta gggaagattt atgatattga tgtttaaagg 4860
gggaagtatt tttgtgtaaa ttaattttaa ttagtagatt gtttttggtg ggagagaggg 4920
atttagaggt tgagtagggg aaggatgttt ttgagaaagt tgtttttagt tttttttaga 4980

tttttgttg gtgtttttat gatatttaaa tttagattta gtggattata tttaaggtat 5040
tggtttttga tttttatttt tggtttgttt gtttggtttt taggtttgtt tggatgtatg 5100
tgtgtttttt atattaggtg ttttttggga tggggtttgg ttttttttatt ttgtaagaat 5160
tagatttttt ttaggattgg gggggttatt gttttgtttt ttttatttgg atttagaagt 5220
tttaatttaa gatttgttta gtgttgtttg ttagaatttt tttgtggtat gattttgagt 5280
agagggtgag ggaaaggagg gttagtgttt ggttttgttt tttgtttggg tttttttttt 5340
tgttttttttg tttttttttt tttgttggtt tattgtattt ttataggagt tataaagatt 5400
aggttttagt gttggatata aaggttaagg gtttttggga gttgtgggta tttagagtta 5460
ggagaggggt tttggtgtgg agggtgttgt ggtttagttt tttggttata gagtgtgttg 5520
gttgtgaggg aggattaagg tttttaatta tgtttgtgtt tatttgtgaa ttttggtgtt 5580
tgtagttttt gggtggtgta gttgttattg ttgttgtttt ttttggagtg tagaggggtg 5640
tgtttgggag ggggtgggag ggtatttttt tttgtgttgg tgaaaggaag agtttttagt 5700
tttttttttt gtataaattt ttattttttt ttttgtttga ttgtgtttta aaatttggga 5760
ttggtgatgt aaggatagtt tggtttggtg aagatttttt ggatattttg tgttttggat 5820
tttaggtggg tggttttatg gtgttggttg ggatgtgtag tttggggggt gggattatgt 5880
ttgggatttt ggggttgttg tttgaggatg gaggttggtg atttgaattg tgttgttttt 5940
agggttgtgg tggggttttt tttttagttg tttagtttgt agtgtttagt ttagtgtatt 6000
gaggattaaa aagggtggga gatttttttt ggttttaaaa ggtaattgtg ttgagatttt 6060
aattttaatt tgggggtttg tttgattttt tggagtaatg ttttttttaaa attatggagt 6120
aggttagtgt ttggaattgt ataggtagtt atgaaggaag gaaatgaagt tttttgtttt 6180
aatagttttg ggtttagtta taagtttttt tttggtgagt atgtggttgt tttatttggg 6240
gtgggggagg tgttgagaaa gtgtagtttt ttttggggga ggagagatgg ttttttagag 6300
gtgtttttat ttgtttttgg aatgtttttt tttgtaggtt tttttggttt tttgtaattt 6360
gttgtttgtt gggatatttt tatggtttgg aggtgtgttt ttggattttg gtgaggaggg 6420
tgaagagtgt tttaaaatgg gttgagagga aaatgtgttt atattggtgt tttttttattt 6480
tggttgtttg tttttttttt tttttttttt tatatgtttt ttatttttgt gtttgtattt 6540
ttttattttt agtattttgt aatggttgga attgtaggga gtggggagta atgtgaaagg 6600
ttgagagaag agtgttgggg agaagttttg gaggttttag attttttggg gtggtttttg 6660
tggggtgttg ggttaggata gttgttaatt ttttaaattt gtgtgtttag aaggagttgt 6720
tttttgtttg tttttttttta taggtttatt ttagatttat ttggggatga ttttaggtgt 6780
agtttttttt tgttggttta agtttttgat aggagttttg tggtgaggta gagatttttt 6840
gaattgagtt ttttgatgtt ttagttgttt gttagagttt ttatgtgtgt tttttagttt 6900
ttgaaagttt ttttttttgt tttattattt atgtgtttgg tgatgttttt gtgggttttt 6960
ttttgttttt ttgtttttat tttagttttt tttgtttttt ttagggttta tttaattgta 7020
ttatgagtgt ttgtgttttg tttggtattt ttggttattt gtagttgggt tggtgtttag 7080
tgtttgtgtg ggaggaggtg ggggtgtgtg ttgggttgag tggttttggg gtgtagtagt 7140
tgtgggtagt ggtagtgtta ttattatagt tagttgtttt tttgtgttgg agttatagat 7200
tagtggggtt atttgttttt gattataaat ttttagggtg gttggaggag ataaaggtgt 7260
tgtgtgttgt tgttttagta gtagtggttg tgtggtggta tgggtttgga gtttgtaggt 7320
gttggagttt tgtgtggttg tttggtttag ttttgtgtgt tttgttttgg tttggttttt 7380
gggttaggta tgttgggttg gggtgtggtg tgggtgggtt tagggtgggt tttggatggt 7440
tttggtaggg ttgttggttg tgtgtgtagt attaattagt gtggttgttg tgttttgttt 7500
tatatttggt ttggtgtgta tttggttggt ggtggtgtgg tttgtggttt agttattggt 7560
tgttatagtg atgttttttt tttttttttat atgttgttga tagtaattgg ttaaggggtt 7620
gtggtttttt aaatattttt ttttgtttga gattatgaat ggatattagt tttgttagtt 7680
attggtataa tgttgaagtt agggttttttg tttttttttt ttttttagtg tttttttttt 7740
ttattgttta tattgggtaa gtttagggta gtattgaggg gagagagatt ttggttagaa 7800
aagttaaggt gtgtggtttt gttgtgattt ttgttttggg aggggtgggt ggggtgtgtt 7860
gttattttttg gtaagagaaa attgtatggt atattgtttt ttttagttat tttttttagt 7920
gttgttgttt gggttttttt gttttttttt taaatggaag taggttaatg atttagggtg 7980
agtagatgag agtttttttg tttattttat tagggttttt ttgagtgttt aggttttgat 8040
gtgtgtagaa agtgttttgt gagatgtgga attggtgaga ttttgttatt atttggttta 8100
ggttaatgtt ggttgttagt ttaaaatgtt gaagggagat ggtggtgaaa atttaggtgt 8160
gtgagttgtg tttttgtttt tttttttatt taggttaatg gtttggttta gattattgtt 8220
ttgtgggtga aaggagtgga gtagaattat aggttatgtt tttagttgtt tgagtttgtt 8280
tatataattt ttggtttttg gtgtagttag gtttgtagtg ggtgagaaga gttagggttt 8340
tggggtgatt aggattattg agtatttttt ttttgtgttt tttgtttatg tttttttttt 8400
ttaggttttg gattttgggt tatagttata ggttggggta ttttggtgtg tggtgttttt 8460
gtggagttgg tgagagttgg agagttgaaa tttaatattt gtgtgtataa tgtgtttttt 8520
gaggtttata gttgtaggaa taaggagaag aaaggaagag gggggtgtag attttgttat 8580
atgtagatga tagtttgtgg agggtttttt agtttttttt tttttggaaa ttttagtggt 8640

tttttttag gtttttttt tttattgttt ggttattgtt ttttgtgttt gggatagttg 8700
gagatggtgg tttgtgttat ttgatgtttt ttttgttgta tatttggttg tttgtggtgt 8760
ttgtttttgg ttttttggg gttaattttt ttatattttt tatttttta ggttaggttt 8820

aggtggtgtt tttatagttt ttttttttt tttttgtatt ttggtgttgt ttttagattt 8880
gtttggtgta gagggttttg tttggtgttt tttttttata aatttagttt tgtaaaattt 8940
gggggtgagt tttagggggt agggtttaga ggtgggggtt gtttatagat atgagtatgg 9000
tgttgtttgt tgtaggtttt tgtttaaaag tggttgaagg attttgtttt ttttgattgt 9060
aggtttaggt tgagttttgt atttattttt gtatttatag ggtaaggtta gagtgtttta 9120
taaagtttta gggtaaagaa gtggttttgt attgggttat ggtttttgta ggtttgagtg 9180
ggttgtagga ggtttggtgt ggtttggtgg gggtgtgaga ggtgagaaat tgtggtgtta 9240
ttggtgtttt ttgtttatga gtgaattttt ttagtaggtg gggatggggt atattatttt 9300
ggttttttttg ttttttttgt ttagggtaag gtttgtaaaa atttttgtat tttggtttgg 9360
gggtggtgtg gtggtgggta ggttagagag aagtggtatt tgggtttttt tagtgtttgg 9420
gatgtttttt tagggatttt tttgggtaaa gagttgagtg gtagtggttt ttgttttttt 9480
ttgttatttt tttttggtgt tttggtgttt ttagttaggt tttagggtga ttttaggttt 9540
ggttttgggt agagatagag gaggggaggg gaggtggtgt tggggggtgg ggggttggag 9600
atggtgggag gaggatttgg gagttggggt gggaagaggg gaagatagtg gagagaaggg 9660
atggggaggg gagggggttt ggaggagggg atttgttgtt tttagtttgg ttggtaattg 9720
gttttttttat agtaatggtt agtgtgtgtg tttgtgtgtg tgtgtgtgtt tgatgtgtgt 9780
gtgtttgtgg tgtttttggg attttttgtg ggggttggga ggggattgta gaattttagg 9840
gtggtgatag agtaaatttt tttaggattg gaggtttgat ttttgtggtg ttgggagtgt 9900
tttagggttt ggttgtgttg tgttggtgat ggggtaggtt tggggaatgt gtgtaggttt 9960
tgggattggt tgttggggta ttggggtatt ttgtttttgt tgttttgatt tttttgttgt 10020
ggttgtaggg ttttggggta ttggggagtg ggtgggtttt tttagtgatt tggagtatag 10080
gggtgggtga tggtgttttt attatggttt tgtttggttt gagtgtttgg tgtatttgag 10140
gtttgggttg gggagtagtt ggggaattgt gtgttgtttt ggggagtttt tggttaggtt 10200
ggttttgggt agaggttaga ggggttttgg tgtatttttt ttgtttgtgg tgattttgta 10260
ttattttttt tgttagtttg tggttttttt tgttttggat tgttggagtt ttatttaggt 10320
tggggttttt agtaggtggt tgggagtttt ttgaggtggt taggagtggt tggtagttgt 10380
tttttttggt taggttgtgt ttagtttttt agtttttatg gtggtgtaga ttgtatgtgg 10440
ttattttggg ttttggatag ggttaggggg atgtagattt tgttgtttgg gggttttggg 10500
gtggttatgg ggttggtgat tttagagtaa ggtggagttt agtataggtt agagagttgg 10560
ttttgttttt tttatagata tgatttgata gtgaatatat aaataaatat aattttttttt 10620
gttagttttt tttttgttg ttttagtgtt ttatttttaa tattaaagta ttgtattttt 10680
gatttgattt taatttaatg atttgttttt ttagtgtttt gaggaaagat gggtgggagg 10740
tggtattaat ttagtttaat ttgggttatt ttaaaaattg tgagagggtt tgaaaggttt 10800
tatgtgggtt tattagtaga atagtatatt tttttgttta tgttttggtg tatttgtggt 10860
aatatatttt gggtggtaga tggttattta tgtttgagag tttttaattt atttttttttt 10920
tttttattat tttttttta aggtgtggtt tgattttagt ggggttgtag aattggtttt 10980
gttaaagttg aaggtggatt 11000

<210> 306
<211> 11000
<212> DNA
<213> Artificial Sequence

<220>
<223> chemically treated genomic DNA (Homo sapiens)

<400> 306

ggtttgtttt tagttttggt gaggttagtt ttgtagtttt attgaggtta ggttgtattt 60
tagaggaggg gtggtgggag gaaggggagt gagttggagg tttttagata taaataattg 120
tttgttattt agaatatatt gttgtaggtg tattaaggtg tggatgagga agtgtgttgt 180
tttgttggtg aatttgtatg gaatttttta ggttttttta tgatttttaa agtgatttaa 240
attgaattga attggtgtta ttttttattt attttttttt aaagtattag aaagataggt 300
tattaaatta agattaaatt aggaatgtgg tgttttgatg ttggaagtga aatattgaga 360
taatggaaga aggattaat gaaaagaatt gtatttattt gtgtatttat tgttaggtta 420
tgtttgtgaa gaaagtaaaa ttagtttttt ggtttgtatt aggttttgtt ttgttttggg 480
gttgttggtt ttgtggttgt tttaggattt ttagatggta gggtttgtgt ttttttagtt 540

ttgtttgagg tttagggtgg ttgtgtgtgg tttgtgttgt tgtggagatt gaggggttgg 600
atatggtttg gttgagggag atggttattg gttatttttg gttattttgg agagtttttg 660
gttgtttgtt ggagatttta gtttggatga ggttttggtg atttagggtg aggagagttg 720
tgggttggtg gggaaggtgg tgtgaggttg ttgtgggtgg ggagggtgtg ttagggtttt 780
tttggttttt gtttggagtt ggtttggttg ggagtttttt gaggtgatgt gtggtttttt 840
agttgttttt tggtttagat tttaggtgtg ttgggtgttt gggttgggta gggttatggt 900
gggggtgttg ttgtttgttt ttgtgttttg ggttgttggg aagatttgtt tattttttgg 960
tgttttaagg ttttgtgatt gtagtgggag ggttggggta gtgagggtag gatgttttaa 1020
tgttttagtg gttgattttg gggtttgtgt gtgtttttttg agtttatttt gttgttagtg 1080
tggtgtagtt agattttagg gtgtttttag tattgtggag gttgggtttt tagttttgga 1140
ggggtttgtt ttgttgttat tttagggttt tgtgattttt ttttagtttt tgtggggagt 1200
tttgggaata ttatgggtat atatatatta gatatgtgtg tatatataga tgtgtgtgtt 1260
ggttgttgtt atggagaggt tggttattag ttaggttgag ggtagtaggt tttttttttt 1320
gagttttttt tttttttgt tttttttttt tgttgttttt tttttttttt gttttggttt 1380
ttgagttttt tttttgttgt ttttggtttt ttgtttttta gtgttatttt tttttttttt 1440
ttttgttttt gtttagggtt gggtttgggg ttgttttgga gtttggttgg ggatgttgag 1500
gtattggagg agggtggtgg ggagggatag gaattgttat tatttgattt tttgtttgag 1560
aaggttttg ggagggtatt ttgggtgttg ggggaatttg agtattgttt ttttttgatt 1620
tgtttgttgt tgtgttgttt ttgagttagg gtgtgggggt ttttgtgggt tttgttttgg 1680
gtagggggga tgagaggggtt aaggtgatgt attttgtttt tgtttattgg aggggtttat 1740
ttatgggtag aggatgttag tgatgttgta gtttttttgtt ttttgtgttt ttgttgagtt 1800
gtattgggtt ttttgtagtt tgtttaggtt tgtgggaatt atggtttgat gtgaggttat 1860
ttttttgttt tggagtttta tgaagtgttt tggttttgtt ttataaatat aaaaataaat 1920
gtggaattta gtttgggttt gtagttagag gaaatggggt tttttggtta tttttagata 1980
ggggtttgta gtggatggta ttgtatttat atttgtggat aatttttgtt tttgagtttt 2040
gtttttttggg gtttgttttt aggttttata gaattaggtt tgtgagagaa aagtgttagg 2100
taaggttttt tgtattgagt aggtttaggg gtagtgttag agtgtaaggg aggggagggg 2160
agattgtgga agtgttgttt gggtttggtt tggggaggtg gggggtgtgg aggagttggt 2220
tttggagggg ttgggagtgg gtgttgtggg tagttgggtg tgtggtggga ggggtgttga 2280
gtggtgtggg ttgttgtttt tagttgtttt gggtgtaggg aataatggtt aggtggtggg 2340
agaggggggt ttaggagggg gttgttgggg tttttagaaa aggagaaatt aaagagtttt 2400
ttgtagattg ttatttgtgt gtgataagat ttgtgttttt tttttttttt tttttttttg 2460
tttttgtagt tgtgggtttt ggagagtgta ttgtatatat agatgttggg ttttagtttt 2520
ttggtttttg ttagttttgt gggggtgttg tgtattgggg tgttttaatt tgtggttgtg 2580
gtttggggtt tagggtttgg ggagagagag tatgggtggg aggtgtgggg gggaggtgtt 2640
tagtggtttt agttgttttg agattttagt tttttttgtt tgttgtgggt ttgattgtgt 2700
taggggttgg gggttatgtg ggtaggtttg aatgattggg agtgtggttt gtgattttgt 2760
tttatttttt ttgtttgtag agtggtggtt taggttagat tattgatttg ggtaggggga 2820
agggtaagga tgtagtttgt atgtttgggt ttttattgtt gttttttttt ggtgttttgg 2880
gttggtggtt gatattggtt tggattaaat ggtggtaaag ttttattagt tttgtgtttt 2940
gtaagatatt ttttatgtgt attgggattt aaatatttgg gaggattttg gtaaagtgag 3000
tagaggggtt tttgtttatt tgttttgaat tattggtttg tttttatttg gaaagggagt 3060
aaaaaggttt gaatggtaat attggaaaag gtaattggga agaatgatat gttgtgtaat 3120
tttttttttgt taagagtggt gatgtatttt atttattttt tttaggataa aggttatagt 3180
agagttgtgt gttttgattt ttttggttga ggtttttttt tttttggtgt tattttgaat 3240
ttgtttagta tgaatgatga aagggagaaa tattgaaaag aaagaaaagg tgaaggtttt 3300
ggttttggtg ttgtattaat gattgataaa gttgatgttt atttatagtt ttgggtggga 3360
gagggtgttt ggagagttgt ggttttttag ttaattgttg ttagtaatat gtgggggggg 3420
gggggggatat tgttatagta attggtggtt gggttgtgag ttgtgttgtt gttggttggg 3480
tgtgtgttgg gttggatata aggtggggtg tggtggttgt gttggttggt gttatgtgtg 3540
tggttggtgg ttttattggg attgtttgga gtttgttttg agtttgtttg tgttgtgttt 3600
tggtttagtg tgtttagttt gggggttggg ttggggtgga gtatgtggag ttgggttgag 3660
tggttgtgta gagttttggt gtttgtgggt tttgagtttg tgttgttgtg tagttgttgt 3720
tgttggagta gtgatgtgtg gtatttttgt tttttttggt tgttttagaa gtttgtggtt 3780
agaagtggat gattttgtta gtttgtggtt ttagtgtagg gaagtagttg gttgtgatga 3840
tggtgttgtt gttatttgta gttgttgtgt tttgaggttg tttggtttgg tgtatgtttt 3900
tatttttttt tgtgtgggtg ttgggtgttg gtttagttgt aggtaattag gggtgttggg 3960
tggggtgtgg gtgtttgtag tgtggttggg tgggttttgg aagaggtgag agaggttggg 4020
atgggggtgg agggagtagg aagggtttat gagagtatta ttaggtgtgt aggtggtggg 4080
gtgaggaaga gagtttttga aggttggagg gtgtgtatgg gggttttaat gggtaattag 4140
ggtgttggag agtttagttt aggggggtttt tgttttgttg taggattttt gttaaaggtt 4200

```
tgggttggta ggggagagtt gtgtttggga ttatttttaa gtgggtttag ggtgggtttg    4260
tgaggaggag tgggtaaagg gtagtttttt ttgagtgtat agatttggag ggttggtagt    4320
tgttttggtt tagtgttttg tagagattgt tttaaggagt ttaggatttt tgggattttt    4380
ttttagtgtt tttttttgg tttttgtgt tgttttttat tttttgtagt tttgattatt    4440
gtagggtgtt gggggtgggg gggtgtaggt atggagatgg gaggtgtgtg ggaaaggagg    4500
agggaagggg tgagtagtta ggatggagag gtgttagtgt gggtgtattt tttttttagt    4560
ttattttggg gtgtttttta ttttttttat tagggtttgg gagtgtgttt ttgagttatg    4620
ggagtatttt agtgggtggt gaattgtagg gaattgaggg ggtttgtagg gagaggtgtt    4680
ttagaggtag gtggggatgt ttttgagagg ttattttttt ttttttaaag agaattgtgt    4740
ttttttagtg ttttttttat tttaggtgag gtggttgtgt gtttgttaga gagaaatttg    4800
tgattggatt tgaagttgtt ggaatgaggg attttatttt ttttttttgt aattatttgt    4860
gtagttttga atattggttt gttttgtggt tttagagaag tgttgtttta gggaattggg    4920
tgaattttta agttagagtt aaaattttag tataattgtt ttttagggtt ggagagagtt    4980
ttttgttttt tttggttttt agtgtgttga gttgggtgtt gtgggttgga tggttgggag    5040
aggggttttg ttatagtttt gaggatgatg tggtttaagt tattgatttt tgtttttgga    5100
tagtggtttt ggggttttgg atgtggtttt gtttttttgag ttgtgtgttt tggttggtat    5160
tgtggagtta tttgtttgga gtttggagtg tagggtgttt gaggggtttt tgttaaatta    5220
ggttgttttt gtgttgttga ttttagattt taggatgtga ttgaataaga gaggaagtga    5280
aggtttgtgt ggaggaggga gttggaagtt ttttttttta ttgatataga ggaaagtgtt    5340
tttttgtttt tttttaagta tattttttttg tgttttagag aagatggtgg tggtggtggt    5400
tgtgttgttt gggggttgtg agtgttggga tttatgggtg gatgtgggtg tggttgggga    5460
ttttggtttt tttttgtggt tggtgtgttt tgtggttagg aggttgggtt gtagtatttt    5520
ttgtgttggg attttttttt tggttttgaa tatttgtggt ttttgggaat ttttggtttt    5580
tgtgtttggt gttggggttt gatttttatg atttttgtgg ggatgtggtg aattggtgag    5640
gggggaggga tggggaagtg ggggagaaag tttaggtagg ggatggggtt gaatgttgat    5700
tttttttttt tttgtttttt gtttaaagtt gtgttataga ggggttttga taagtgatgt    5760
taggtgggtt ttgggttggg gtttttgagt ttgggtagag gaggtgaagt ggtaattttt    5820
ttagttttga agagggtttg gtttttgtgg ggtggggagg ttaggttttg ttttgagaag    5880
tatttggtgt ggaaggtgtg tgtgtgttta ggtaggtttg gggattaggt gggtgggtta    5940
aaaatagggg ttaggagttg gtgttttaag tgtgatttgt tgagtttgag tttgggtgtt    6000
atgaaaatgt tagtggagag tttgaaagag gttgggggtg gtttttttag gggtattttt    6060
tttttgttta gtttttgggt tttttttttt tattggaggt aatttgttgg ttagaattag    6120
tttgtataag ggtgtttttt tttttggata ttagtattat aaattttttt tgatgtttta    6180
aaatatagta ttttttttaaa attgatgttt taggagtgtt taaagatggt aaatagataa    6240
tttattgttt tttttgtata tttattttta agtgtatgtt tggaaaaatt taataaatag    6300
tttattttaa aatagtatat tttaatttat ttttattaaa gtttatattt aagaataatt    6360
ttttttttgaa gatttttttt ttttgttttt ttgggttttt tggttatttg aatggtagat    6420
ttgtaagtat aattttattt gtatggagga ataatagttt ttagttgtat aaagggtttt    6480
ttttattttg aatttagatt gtagtgtttt ttgttttgat aatagttttt gaaaagattt    6540
tgtaatgtag agaattaata tatgattatt tttgtttgat ttttttttatt tattagaatt    6600
ttgtggtaaa ttttaaatta aaagtagttt tgttaatagt tttaaaaaat atttttaaaa    6660
tattttaaga tatttttttt tttgtgtttt taagttggat tatttgttat ggtttgaaaa    6720
tttaaaatgt ttattagttt taaagtgagt attatatttt agtaatgttt tttagtaaat    6780
ttagaatgaa attttttaat aattttaaga ggatttattt agtaaattaa agataaattt    6840
gttatagtaa tttattaatg gtttttaaaa ttgattttta agttttaaga gaaaaaggta    6900
tttataattt agtaatttaa atgtatataa agataggatg aggttgggat tgaaatttga    6960
ttttttagaa atgatataaa tgaaagtatt aataatgtta gaagtttaat attaattggt    7020
attgagtatt tgtattttta tgtttttatg atgtaagtat attttaatat tgtatattgt    7080
gatttagaga atgaattgtt tttgttaatt ggttatatta tataattttg gaaaaggatt    7140
aaatttggtt atttgttaat atatatttat tataaagttt tgtatttgtg tggttgtatt    7200
agtttttata attgagtttg ggtataatat gttttttttt tttttttttt aagtttatta    7260
tatggatagt taaaaaagtt ttgaaggtat ttttttgtaa atggtttata agggtatggg    7320
gtggaagttt tatgtatttt tatttagggg tatggttaaa ataatttaaa ttggttagga    7380
tagtgtttgt agatgttaag ttttaagata gttttataaa ttgtttttgt ggttatatat    7440
ttattttgaa ttataatttt tgtaatattg tttattttaa atttgaggag ttgtaggtgt    7500
agatggttga gaggaagaaa tatgtgatat tgtattttat tttattttta agatagattt    7560
aatttgggat aaaattaatt taattatttt tgattatttt tttaaattat attattttttg    7620
tttttgagaa tttagtattg aagtgtgttt atttgtgtat ttttttttatt tgggatgtat    7680
ttttgttgtt gtgttattag gttttttttt tttttttttt tttttttatt tttttttttgt    7740
tttaaatttt tagttattga gttgttgttg tatagaaggg ggtgttttgt tttttaaaaa    7800
tgtttgattg taaatgttaa agttataaat atttaaatat aatttttttt taattttaag    7860
```

```
ttaagattga taagatgttt ttaattagtt tgggtttttt ttggggttta tttgttgtgg    7920
tgtagttttg gggggttggt gtttatgggg gagatggtgt tgaggattat taaagttata    7980
tttgggttaa gattgtgttt taaatttatt tttaaattta tttttttttt ttaaaggtta    8040
tggttagtag gagtttgaag attttttta attttataaa aagggagtta agttattgta    8100
aagttattgg aatttaatta tttagaaaaa aattttttaa atttttgttt tttttgggat    8160
ttttaagatt tgatttgtag ttaatagttt ttttttttgt attatttttt tgtttagtgt    8220
tgggtgggat atttgaaatt gttttgggtg tggagggaag attttttttt ttggtttttt    8280
ttgtggtgtt tgtgggtggg taggaagtgg gtttttagt tgggttttgg gatttagtga    8340
tttttgtgtt aggttggtgg attttagttg gttttttttgt ttagtgtttt gttttttttt    8400
gtagtaagat ttttttttgtt tttagtttg gtgttgtttg tggttatgat tttttggttg    8460
tttaggtttt gtggttttgt gtggttgttt tttgggtttt ttttggtttt ttttaggttg    8520
taggaggggt tttttgtgg attggtgtgg ggaggtgggt ttggggtggt ttttattttt    8580
tttttgttgg gatagtgtgg gtttaggtag tagggagttg ggtgaggttg gtggagatag    8640
gattttaggg tattggggtt ttttgttttt tttttgggga tttttgggat ttagtgggga    8700
tttgggagga gagttaggag ttttttaggt aattgttttt tttgatattt ttttttgatt    8760
tttgagtaaa tttaagtgtg ttaagggagg tttttaaagt aggtttggtt ttgtttttgt    8820
gtgtgtggag tgtgttgagg gttttggtga tagtaggtgt ttgttttttt gggaatagtt    8880
ttggttggtt ttgggttttt tttgttgttt gtagattagg tttttttttt tggtttttgt    8940
gtgtttttttg tttttatttg gtttgagtga ttgtgggtag tgtttgtgtt ttttttttgg    9000
ggagttggag ttggtggttg ttttgtgttt tggaatttta tatatttgtg tgtatagttt    9060
gggtgttatt gttgggtttg ttatatgttg tggttttgtt gtttttttag aattagggtg    9120
gttattgtgt gtgtgtgggt tttatggtgt ggggtaggaa ggtttgggaa gtttttgttt    9180
ttgggtgtag ttatttatgt gagtgtgtgg tgttttttttg atgttggggt agtggggttg    9240
gggttgtttt tgtgttgggg tggatggagg tgtgtggggg ttattggttt ggttgggatg    9300
aggggtggga gggtggtggg tgtttttttt gttggttgtg ttgttttttt tgttgttttt    9360
gttttgttgg ttaaggttag gtgggatttg tgggaggtga gaggtgaggg gtgaggggtg    9420
gtgaggggtg taggttgggt ttttgggaaa agttttttttg tttaggtatg gttttggggg    9480
atgtggtttt gtaggtgaat atggatttg gggtgggtag aggatttggt ttttgttttg    9540
gtgttgtttg gatttatatg ttttttgtat ttggggttgt ggttgttttt ttttgtttgg    9600
ggttttttgt gtgttttagg gtttttttgtg ttggtttagg agggtatagg gtggggttgt    9660
tgtttatttg ggagaattag tgtttgtgtg gggattgagg agattttggt ttgggggagg    9720
tggtgtgtgg ttggtgtttt aggttttgtg aatgggttgt gtttggggag gggtggggag    9780
gggtgtgtag gagatttggt gggtggattt tggtggattt tggggttggg ttgtgtggtt    9840
ttagagtgat ttttttttgg aagaggttgt gtgggttgga gggagggggg tggaggggag    9900
gggaggggag gagattgggg agggggtgtg gattggattt ttatgtgaag ggtgatttgg    9960
tggtgagatt tagtggtggt tgtggggttt ggattaaatg gggtttgggt tgtttttttg   10020
gtttttgttt tttttttttt ttttggtttt ttgtttattt tttttttta ttttgtgttt   10080
tttttttttt tttttttttt tttttttttt tttttttttt tttttttttt ttttaaagtt   10140
tttttttttt tttgtggtgg ttttattgtt ttttgttgtt ggagaatttt gtggtttttt   10200
tttgagttgt gtatttaggt aggaaaattt gggtgttttt ttttatttaa tttgtgtttt   10260
tattaattta gttttggtat tagttaattt gttgtattga aggtaaaagg aggttaatta   10320
atgattagtt tttttagtta agattggtga taattgtttt ggtggttttt atgattataa   10380
gataaaaatg ggtttggttt gatataagag gtattgtgta tgttgggaga tgggaggaaa   10440
tgaagagttg ggaggttgaa tatggagtag aaaattatta atagaagaga gataatgaat   10500
aggttggtta ggtatttatg gggaaaaatt tattttgtta ttatgtgaaa ttaggtggtg   10560
gaaaggggtt tgttaattgt ttttatttg tagtagttag gattaaggtt gggtgtgttt   10620
ttttatttat tttttgagtt attgtgtagg gtgtgtggtt tgtggggtgg tttttttatt   10680
tttttgtggt gtttttaatt gttgttaata tattttttaa tgaagttaat gaggggagag   10740
ttgtgtaatt atttgtatgt ggagataagt gaaatgtatt gaaagggttt ttttatattt   10800
ttattagttt tatttttgtt ttttaagaag tagtgagaat ttgaagggtt ttttttgttg   10860
agaagtgaag ttttaaaggt tttgtagttt tttttaagga aaaatatgta ggtgtatagg   10920
tggtaagtgt tgtggtttgt attaggttat ttttggagag tgattggagt taggtgggat   10980
ttgattagtg tttgtttttt                                               11000
```

<210> 307
<211> 1743
<212> DNA
<213> Artificial Sequence

<220>
<223> chemically treated genomic DNA (Homo sapiens)

<400> 307

ttgtttttta aattttgttg taattgtgaa tgtttgtgat gttattatat ttttagtata 60
agagaattgg agtattttg gttttttta taattgaagg gtttttagtg tagggtgtag 120
ggggagaggt ttgaagtttt ggagatagtg tttgagggat ggaggttgtg tttggttgtg 180
tttaggtttg ggtggttttg ggagttgaga ttttgtagga tgtagtgggg gggggttagg 240
gtttgatttt ttatttttgt gagttagggt tgtttatgtt tttattgttt tttataggta 300
atgtttagtt tttttttttt ttatttttg aaatattttg gttttgtttt gttttgtttg 360
ttgtttgtga ttttgatttt gatttgtttg tgatggtgtt ttttttagga ggtagtggag 420
gattagggtt aggttgttta ttttggtggt ggtggtggtt tttggttaag gtggattttg 480
gggaggatag tagttgggtt gtggtttgtt tttaggggga gtgggtgtag ggttgtgagt 540
gggtagtgtt tttgttttgt atgaataaag tggttaggtg tgttgggttg aggtagtatt 600
gtttttttgg gagtgtgggg ggtggggagg atggtggggg tatttatttg agggttggtg 660
gagggtgtgg gtgggatagg tttaggagat gggattgtgg tgggttgggg ttttgtgttg 720
agggtgtatg ggttatgggg aaggggtgat tggtgtgatg tttgtggtgg ggtttgggag 780
ttttgtgttg tgggttgggg gttggggttt gagtttgatt ttgtggttgt gaggtatggg 840
aattggtttg ttgggttagg tagtggtgtt aggtagtagg ggtagtgatg ttgttgtttt 900
tttttttttt tgtttttgtt tttgttttgt tgtgttttttg tttttgttgg gtttttgttg 960
ttgttgtttt agtttggtg tttttgggt tttttttttt tattaaggtt gggttgattt 1020
gtagggatta ttttggaaag tgaggggttg ttgttgtttt ttgtagttgt tggtggttat 1080
ttttaatttt tttttttttt ttgtttttt tattttttgt tggttgtttg attgattgat 1140
tggatttttt tttttttttt tttgttttt ttattgagtt ggttgtagaa attgtagttg 1200
tttagttttt attttttttt gttttttttt tttttttttt tattttttttt tttttttttg 1260
gtttttgtt tttgttttat ttttagtggt tgtttttgtt tttgtttgta gatagtgttg 1320
ttggatgttt ttagttggat tttaatttta tttttttag tttttttttt tattgttttt 1380
tagatgtgtt ttttttttgt tttgtgtttt tttttttttt tttattttg tttttttttg 1440
tggtgtttat ttttttagt tttagttttt gaaaggattt agttgagaaa ggataattgg 1500
gttttgtttt ttttaatttt atattttta gttggatgtt gttagaggtg atggagaaat 1560
gtaaaggtgt gttaatttta ttttagttag agtataaaag aattgtttat gttagttatt 1620
gtattatgta gttttgaaat ttttgtatgt tttttgtgt ggtttttgtt tgtgatggga 1680
ttgtagttga ggagtttggt tttaaatggt gttttttgtt agtgattttg agttggttgt 1740
tta 1743

<210> 308
<211> 1743
<212> DNA
<213> Artificial Sequence

<220>
<223> chemically treated genomic DNA (Homo sapiens)

<400> 308

taggtaattg atttaaggtt attgatggaa ggtattattt gagattgagt tttttaatta 60
tagttttatt atggatggga gttatatagg agagtgtgta ggagttttga agttgtgtag 120
tataatagtt ggtgtgggta gtttttttat attttggtta gagtaaaatt aatgtatttt 180
tgtgtttttt tattgttttt gatagtattt agttaaaggg tgtagggtta aggaaagtgg 240
aatttagttg tttttttta gttgagtttt tttagaaatt gggattgagg agggtgagtg 300
ttgtggagag gggtaggggt gggagaggag agaggggtgt ggggtgggga agaggtgtgt 360
ttggaaggta gtggggagag ggattgagag gagtggggtt gaagtttagt tgggagtatt 420
tagtggtgtt gtttgtagat aggggtgaag gtagttgttg agagtgaggt aagagtggga 480
agttgaggga agggaaggaa gtaaggaaag aggaaggaaa ggtaggaggg ggtagaagtt 540
gagtggttgt aatttttgtg gttggtttag taggaggagt aggaggggaa agaggaggat 600
ttagttagtt agttagatag ttagtgggag gtggagaaag taggaggagg aggaggatta 660
aagatggtta ttaatagttg tgggaaatgg taataatttt ttattttttg ggatggtttt 720
tgtgggttgg tttggttttg atggagagaa gaaatttgag gagtgttgag gttgaggtgg 780

tggtggtggg gatttagtga ggatgaggat gtggtggagt agggatgggg gtaggagaag 840
ggaaaggtgg tggtgttgtt gtttttgttg tttagtattg ttgtttggtt tggtggattg 900
gtttttatat tttgtggttg tagaattgag tttgggtttt ggtttttggt ttgtggtgtg 960

gggtttttgg gttttgttgt ggatgttgtg ttggttgttt tttttttgta gtttgtgtgt 1020
ttttggtgtg gagttttggt ttgttgtggt tttgtttttt gggtttgttt tgtttgtgtt 1080
ttttgttggt ttttaggtga gtatttttgt tatttttttt gtttttgtg tttttggaga 1140
ggtggtgttg ttttggtttg gtgtgtttgg ttgttttgtt tgtgtggggt ggaggtgttg 1200
tttatttgtg gttttgtgtt tgtttttttt agggataggt tgtggtttag ttgttgtttt 1260
ttttagggtt tgttttggtt gggagttgtt gttgttgtta gggtgggtgg tttggtttta 1320
gtttttgtt gtttttggg gagggtgttg ttgtggatgg gttggggttg gggttatggg 1380
tggtgagtgg agtggagtgg aattgggata ttttggggga taggggagga gagggttgga 1440
tgttatttgt gggaagtagt gggggtgtag gtggttttgg tttgtagggg taggggatta 1500
ggttttggtt ttttttgtt gtattttgtg gggttttagt ttttggagtt atttggattt 1560
gagtgtagtt gagtgtggtt tttattttt gggtgttgtt tttagggttt tgggtttttt 1620
tttttatgtt ttatgttgga agtttttga ttatggaagg agttagagat gttttagttt 1680
ttttgtattg gggatgtggt ggtattgtaa atatttatag ttatggtggg atttaagaag 1740
tag 1743

<210> 309
<211> 10196
<212> DNA
<213> Artificial Sequence

<220>
<223> chemically treated genomic DNA (Homo sapiens)

<400> 309

aagtgtatgt ttattgagga ggtagttata gttataatta tattgttata gttggtgtat 60
atttgtattg ggttatagtt atgattatat attaattata gttatatata tagatagaaa 120
tatgtagtta tttgtaggta tttttatttg aattttgttg tatatagtta tatagtatat 180
agttgtatat tattatgggg ataaatgtag taatatttaa ttaggtataa gttttttata 240
gttgtatata taatgttgta tgtggttttg tttttttta tatatatatt agtagttata 300

gtttttgagg ttgtgttgta tatggtgtga gtgttggaaa tatatttatt ataagggtag 360
tttgttataa ttgtgtgatt atttattata tatttttata tagatgtaag tgtatgtttt 420
taattttatt tttattgttg tataaagttg aggtttggtt tttggttttg tttttttttg 480
ggttttttat tttaggtttt attagttttt gaagtagtag aatttttttt ggtattgggg 540
tagttgttgg taaagttttg agggttttgt tagtgtgtga gttggaagtt gaattattgt 600
gagaagtata gttttgttga ggagattttt gggaagtgta gttagagatg tattaaggtt 660
ggataagatg gtttttgatt gttagaattt ttgtttgtgt tttgtgttag ttttgttgaa 720
aggattgttt tttttagtta aattgtttgt gttttagagg ttttatggag ttttgtttta 780
ggtttgggtt ttggttttta aagaaagtta ttttgatgtg tttgagatgg gtttagtttt 840
tggatttgtt ttattgtttt taggtgttgg atagttttag aaagaggata agtttgtatg 900
gaaagataat tgtataattg agtttttttgg gagatgtagt ttggagttgg gaaggtggtt 960
ggattttttg aaagttattt taattgtttt tatagataag ttggtgttgg gttgttggtt 1020
tgagtattgt gtgtttagag tgtgtaggtg taaatatttt gggttgttgt tttggttttt 1080
ttgtttgggt gtgaattttg tttattgttt tttagtgtag aggggttatg ggattagagg 1140
ttgtgagatt tagaagtggt tggggatgta gaaggggtgt agattaggaa tttgtgattt 1200
gatgggggga ggttgaaggg aaaggaattg ttaaatgtgg gataggtttg gagaggtaga 1260
aagagaggag tttgaaattg taaagggtag gtgttatttt gatttttgtg tgttttgtga 1320
taaatgagtg gggtaggaag taagaggagt tttatttttt ttttttgtaa ttatttgtgt 1380
atttggatta agtgtatttt atggttagta tttgattgtt tttgatttat aaaggtattg 1440
tatatatagt tatgtatagt ttaataataa ggatatatgt tttttttatg gttttttttt 1500
tttttttttt ttatggtttt tttttgatgt tgagttaaag ttggattgta ttgttgtttt 1560
tttggtttat tgtaattttt ttgtttgatt tttttgtttt agtttgttga gtgtttgtga 1620
ttgtaggtgt gtgttgttat gtttgattgg tttttgtatt tttttggttt ttattaaata 1680
tagggttttg ttgtgttggt tgggttggtt tttagttttt aattgtgagt ggtttgttag 1740
ttttggtttt ttgaggtgtt gggattgtag atggagtttt gtttatttag tgtttaatgg 1800
tgtttaggtt ggagtgtagt ggtgtgattt tggtttgtta taatttttttt ttagttgttt 1860
gttttggttt tttaaagagt tgagattgta gtttttgttt tgttgttatt ttatttggga 1920
agtgaggagt ttttttgttt ggttgtttat tgtttgggat gtgaggagtt tttttgtttg 1980
gttgtttagt ttggaaagtg aggagtgttt ttgttaggtt gtttattgtt tgggatgtgg 2040
ggagtgtttt tgttttgttg ttttgtttgg gatgtgagga atgtttttgt ttggttgtga 2100

ttttgtttgg gaggtgagga gtgtttttgt ttggttgttt tgtttgagaa gtgagatttt 2160
ttgtttggta attgttttgt ttgagaagtg aggagttttt ttgtttggta gttgttttgt 2220
ttgagaagtg aggagttttt ttgtttggta gttattttgt ttgggaagtg aggagtgttt 2280
ttgtttggta gttattttat ttgggaggga ggtgggggtt atttttgtta ggttagttgt 2340
tttgtttggg agggaggtgg gggggttagt tttttgtttg gttagttgtt ttgtttggga 2400
gggaggtggg ggggttagtt ttttgtttgg ttagttgttt tgtttgggag gtgaggggtg 2460
tttttgtttg gttattttta ttgggaattg aggagttttt ttgtttggtt agttgttttg 2520
tttgggaggg aggtgggggg gggttagttt tttgtttggt tagttttttt gtttgggagg 2580
tgaggggtgt ttttgtttgg ttgtttttat tgggaagtga ggagtttttt tgtttggtta 2640
tgattttatt tgggaggtgt atttaatagt ttattgagaa tgggttatga tgataatggt 2700
ggtgttgtgg aatagaaagg tgggaaaggt ggggaaaaga ttgagaaatt ggatggttgt 2760
tgtgtttgtg tagaaagatg tagatatggg agttttttta ttttgttttg tattaagaaa 2820
aatttttttg ttttgggatt ttgttgattt gtgattttat ttttaatttt gtgtttttttg 2880
aaatatgtgt tgtgtttatt tagggttaaa tggattaagg gtggtgtaag atgtgttttg 2940
ttaaatagat gtttgaaggt agtatgtttg ttaagagtta ttattatttt ttaattttaa 3000
gtatttaggg atataaatat tgttgaaggt tgtagggttt tttgtttagg aaaattagag 3060
atttttgttt atttgtttat ttgttgatat tttttttatt attgttttat gattttgtta 3120
aatttttttt tgtaagaaat atttaagaat gattaattaa aaaaaaataa ataaataaat 3180
aataaggata tattttggtt gggtgtggtg gtttatgttt gaaattttag tattttggga 3240
ggttgagggg tttgagatta gtttgggtaa tatggtgtaa attttgttta tattaaaaat 3300
gtaaaaatta gttgggtatg gtggtgtgtg tttgtagttt tagttatttg ggaggttgag 3360
gtaggagaat tgtttgaatt taggaggtag aggttgtagt gagttaagat tgtattattg 3420
tattttagtt tgggtgatag agtgagattt ggttttaaaa aaaaaaagga tatattttga 3480
gaaatgtatt gttgggtaat tttattatta tggagtgtat tgatataaat ttagatggta 3540
tagttgattg aatatttagg ttatatggta taatttgttg tttttaaatt ataaagttat 3600
atagtatgtt attgtattga atatagttgg aatattgtag ggattagttt tataaggttt 3660
gtgggttttt ttttttgtgt gtagagatta gaggtggtag aaataaagat ataagataaa 3720
gagataaaag atatttgggt ttgggtgagt tattattatt aagatataga gattgatagt 3780
ggttttgaat gttaggttgt attgttattt attggatata agataagggg ggtagggtaa 3840
ggagtgtgag ttatttttaa tgataggtaa ggttatgtgg gttatgtgtt tattggatag 3900
ggggtttttt tttgtttggt agttgaagtg gagagagaga gaagatagtt tatgtattat 3960
ttttgtatat tagagatttt tagtattttt attaatttttg ttattgttat ttagaaggta 4020
gggttaggtg tataggatgg aatatgaaag tggattagta gtgtgattat tgaagtatag 4080
tattatagga agatggttag atttttagat aattgtgggt gggtttgatt tatgttaggt 4140
tttttataag atatggtgga gtagagtttt ttttaaattt ttttggggaa agggagattt 4200
tttttgttgg tttgttaagt agtgggtgtt ttttttttggt attgatgtta ttgttagatt 4260
ataatttgtt tggtaatagg tgttttttta gatgttaatg ttttgttaga ttaaggagtt 4320
ttttggtggt tttgtttggg tataatagag gtttatatat ttgtttttttg gttatttttt 4380
attatgtttt tttagtttat atttttgtat ggtttggttt tttttaggtt atgattgtag 4440
agtgaagatt attataatat ttgaataaag agtaattatt ataaattaat gattagtgat 4500
atttgtgtat aattatattt gtgatttgta tttagtgtaa tttttgttat tttatatatt 4560
ttattatatt gaaatagttt atgtttttgg ttttttgttt tggtatttgg gtggtttgtt 4620
gtttatagaa tataatggta agtatttatg tatttaaata tatttaaata tataaaaggt 4680
atagtaaaaa aaatagtatg aaaagtaaaa aatggtatat ttgtataggg tatttattaa 4740
gaatggaggt tgtaggattg gaagttgttt tggagagttg atgagaggtg agtgaatgtg 4800
aaggttagga tattatagta taatatttta gattttataa atattgtata tttaagtaat 4860
attatatgta tttaaagttt tttttttata ataaattaat ttttgtttgt tgtaattttt 4920
ttattttata aatgtttaaa tttttaaaag ttttttgatt tttttgtaat attgtttagt 4980
ttaaaatata aatatattgt atagtttatg tgggatttgt attttttggt aaggaaatgt 5040
tgtaggttag atgttatttt tatgtggtta tatagaggta gaaagattgt atatatgata 5100
ttgttttttt taattatggg tttgtatttt aatatattgt aataatgttt aatatttatg 5160
gggttataga aaataggatt tattatggtt ggagaatttt tagagttaat ttaagtttag 5220
gaatgttggt aatgagaatg attagtagga attagtgtaa tgatttatat agttgaaatt 5280
attaattggg ttaggttggt ggtttatgtt tataatttta gtattttgtg aggttgaggt 5340
gggttgatta tttgaggtta ggagtttgag attagtttgg gtaatatggt gaaattttgt 5400
ttttattaaa aatataaaaa ttagttaggt atggtggtat atatttgtga tattagttat 5460
ttgggaggtt aaggtaggag aattgtttga atttgggagg tagaggttgt agttagttaa 5520
gattatgtta ttgtatttaa gtttgggtga tagagtaaag attttatttt aaaaaaaaag 5580
ttaaaataga ataaaatata tagttattat tttttgttt ggggtaattt taaagttttt 5640
tttttgttat aaaaatagga atgtattatg taaaggtttg aaataggtta tttttttttt 5700
tttttttttt gagttggagt tttgtttttg ttgtttaggt aggttggagt gtaatggtat 5760

gattttggtt tattataatt tttatttttt gggtttaagt aattttttttg ttttagtttt 5820
ttaagtagtt gggattatag gtatgtatta ttatgtgtgg ttaattttgg gtttttagta 5880
gagatgggat ttttttatgt tggttaggtt ggttttgaat ttttgatttt aggtgatttg 5940
tttgttttgg tttgttaaag tgttgggatt ataggtatga gttattgtgt ttggaaaata 6000
ggttattttt ttaaataaaa aggtaatgat ttataaaagt ttatgaatag aatatgtaat 6060
tagttgatat aaatttaata ggatttgttt aaaattagtt aaatttaata tatttgaagt 6120
gtttttgtat aaagtattat atgaagaaaa gaagaattta ttaatgtttt aaaaaaatgg 6180
gttttttat agataatatg ataatttatt attaaaagtg ttttgggatg gttgggtgta 6240
gtggtttatg tttgtaattt tagtattttg ggaggttgag gtgggtggat tatgaggtta 6300
ggagattgag attattttgg ttaatatagt gaaattttat ttttattaaa aatataaaaa 6360
attagttggg tatggtggtg ggtgtttgta gttttagtta tttgggaggt tgaggtagga 6420
gaatggtgtg aatttgggag gtagggtttg tagtgagttg agattgtgtt attgtatttt 6480
agtttgggtg atagagtaag attttatttt aaaaaaaaaa aaaaaaaaaa agtgttttag 6540
gagatataag gaaatgtaat attatttttt ttgaatattt ttagtttaag attttttatt 6600
taataaaata ggaaaggatt tgaaattgag aaaatgtatt tgagtataaa tagtttgtga 6660
aatataatat tttttttgt attattagag ggttttattg aatttataat taatttgttt 6720
atttagtatg taatttagat gtgagtgagg ggttgggtgt agtggtttat ttttgtaatt 6780
atagtatttt ggaaggtaga ggtgggtgag ttatttgagg ttaggagttt gagattagtt 6840
tgattaatat ggtggaattt agtttttatt aaaaatataa aaattagttg ggtgtggtgg 6900
tgtatatttg taattttagg tattttggag gttgaggtag gagaattgtt tgaatttggg 6960
aggtagagat tgtagtgagt ttagattttg ttattgtatt ttagtttggg tgatagagta 7020
agattttatt ttaaaataaa ataaaataaa aaaaatagat gtgagaggat agaggtgttt 7080
tataggagtt tgtattgttt ttaattttat gtgtgtaggt atttattata ggtaaatagt 7140
tttttttatat tttgtagtta tatgattttg ttattagtaa aaagaggtaa atagtttttg 7200
tagaatgaag ggattatagt tatgggatta gagttataga atgaagtttt tttttaata 7260
tttttttaatt tgatgtttga atttttttttt ttttttttt gagatagttt tgtttttttg 7320
tttaggttag agtgtagtgg tatgattttg gtttattgtt atttttgtg tttaaataat 7380
tttttttgttt tagtttttgg agtagttggg attataggtg tttgttatta tatttagtta 7440
atttttgtat ttttagtaga gatggggttt tattatgttg gttaggttgg ttttaaattt 7500
tttattttag gtgatttatt tgttttgggt ttttaagtgt tgggattata ggtatgagtt 7560
attgtgtatg gtttgatgtt tgaattttta atgtagtatt ttagagaagg gtgtttggga 7620
tttttttgtgt ttaagggatt gataaagaaa aaagttttat ttattatttg ggatttgatg 7680
tatagatgaa aaatttaata tataataatg gaagttggtt gttagtaaat tatattttag 7740
ttttttagta tttttataag agatgatatt tttagttgtt tagtttttgt agttttagta 7800
tggtaatatt aatgatgttt atgtttagaa ttagttaaaa agattataat ttgttttttt 7860
tagtttattt attttttatt ggtttatggt tttaagtgta tttgaatgat tatttttggg 7920
tattttttgt tattgtttat tagggtgttg ttgattgttt ttatgttttg tgggttggtt 7980
gggaagggga gtttgggaag gatgtgttat ttttaggagg ttgtaagtta ttgggatgtt 8040
tttagggatg atttttttta tggttgtagg aagttttttt ggagttatgt ttatgatgtt 8100
tggtggatat ttgtatgttg tattgttgag tttgggatga attgtttgtt ggtttattgt 8160
tgattaaggt gttgatgtga taaataattt tttgtttata tagtttttta agtttttttgg 8220
gatgtgattt gtgatgattt ggtggatttt ggtggtagtt gttttttttta tttttagtga 8280
tgtttgtatg ttgttttagg tagtgtgagg agtgaagatg agatttggtg tattttttaa 8340
tggagtttga gtaaagttaa agggttttga tttgtgtaag ttaagggttg tttttttttat 8400
tttgtttttt ttgaggattt gtgttaaggt tttttttattt attaggttat tatgggttgt 8460
gtttataagg aatgtttttt gttttatttg ttttatagta aagttattga tgaggtggtg 8520
gttatgttta ttgagattgt agtgtaatga gatatagtta ttttgatata gtaaattttg 8580
tagggtgtat tagggttttt tttgtatgtt ttgggatttt ttttattttgt tttataagta 8640
ggggttataa aatatgatgt tgaatttaaa ggttttggtt taaattgtaa ttgtttgttt 8700
tatgtaattg aagtttatga ggtttagtgt tttttatgaa tgagggttat ttttatggtt 8760
atttgagaa tttgttttat gttttgaatt tgtgtgtttt tttatagtgt ttggtatagt 8820
tatatatttt tttggtagag attgaggatg tggtagatag tggagttggt tgtttttttt 8880
atggttgtgg aggggatgtt gtatatagta atttggagtt tattggtagt tttgatgttt 8940
atgttgttgt agttatttgt ttatttatat gattattttt agggttttga atttttttag 9000
gtttttttttt gtttagaatt tttttgtgga ttttttgtgt ggattgtgtg ttatagaagg 9060
ttatagtggt taggtttttt aggatgggta tttttatggt gtaggttata gttatttagt 9120
ggtgttagta gtgggagggg gtgtaggggg ttgtttgtga tttgagggtg gatagtttta 9180
taagttttgt ttaattgttg ttttgtgatt ttgtgtttat ttattagggt tattttttat 9240
ggaattttgt aatttttaga ttaaaaggta aagtaggttt ggtatggtgt tttatgtttg 9300
taattttagt atttgggagg ttgaggtagg tagattattt gaggttagga gtttgagatt 9360
agtttggtta atatggggaa atattatttt tattaaaaat ataaaaatta gttgggtatg 9420

```
gtggtgtgtt tttgtaattt tagttatttg ggaggttgag gtaggagaat tatttgaatt    9480
tgggagttag aggttgttgt gagttgagat tatgttattg tattttagtt tgagtggtag    9540
agtgagattt tgtttttaaa aattaaatta aaataaataa taataataaa ataaaaggta    9600
aagtagtttt ttaaaaattt atggaaattt gtaggagtgt gtgtgtatga tgttattatg    9660
aatttaatat atatttgttt ataaatttta tagtttatag gatgggttgt ttattttttt    9720
tttttttttt tttttttttt tgttgttagg ttggagtgta gtggtgtgat tttagtttat    9780
tgtaattttt attttttggg tttaaatgat tgttttgttt tagtttttttg agtagttggg    9840
attataggtg tatattatta tatttggtta ttttttgtat tttagtagag atggggtttt    9900
attatgttgg ataggatggt tttgattttt tgaatttgtg atttatttgt tttggttttt    9960
taaagtgttg ggattatagg tgtgagttat tagttgttta ttttttaag ggaatgtagt   10020
tttgttagtt taaaaatatt taaggtgatg aaatttttgt tttgggtagt tagtatttat   10080
agtggaaagg tttttgtttt ttttatttta tttggaggtt gtgttgtttt agtttagtgt   10140
agtatggttt ttggtttagt tttttgtttt ttgggttgtt tttaagttat tttaaa        10196
```

<210> 310
<211> 10196
<212> DNA
<213> Artificial Sequence

<220>
<223> chemically treated genomic DNA (Homo sapiens)

<400> 310

```
tttaaggtgg tttgggagtg gtttgaggag tgaaggattg aattaggagt tatgttgtat      60
tgaattgagg tagtatagtt tttaggtggg gtgggagggg tgggagtttt tttgttgtgg     120
atgttgattg tttaggatga gggtttttatt attttaaatg tttttgaatt gatgaagttg     180
tattttttta aaaagatgga tagttagtgg tttatatttg taattttagt gttttgggag     240
gttaaggtag gtggattatg agtttagaag attgagatta ttttgtttaa tatggtgaaa     300
ttttgttttt attaaaatat aaaaaatagt taggtgtggt ggtgtgtatt tgtaatttta     360
gttatttagg aggttgaggt aggataattg tttgaatttg ggaggtggag attgtagtga     420
gttgagattg tgttattgta ttttagtttg gtgataaaaa gaaagaaaga aagaaagaaa     480
agatggatag tttattttgt gaattataga gtttgtggat agatatgtat tgggtttata     540
gtggtgttat gtatatatat ttttgtgagt ttttgtaagt ttttagagga ttgttttgtt     600
ttttgttttg ttgttgttgt ttgttttggt ttggtttttg gagatagagt tttattttgt     660
tatttaggtt ggagtgtagt ggtatgattt tagtttatag taattttga tttttgggtt      720
taagtgattt ttttgtttta gttttttgag tagttgggat tataggagtg tgttattatg     780
tttagttaat ttttatattt ttagtaaaga tggtgttttt ttatgttggt taggttggtt     840
ttaaattttt gattttaagt gatttgtttg ttttagtttt ttaagtgttg ggattatagg     900
tatgaggtat tatgttaggt ttgttttgtt ttttgatttg agagttgtaa agttttataa     960
agaatggttt tggtggataa gtataaaatt atgagatagt gattggatag aatttgtgaa    1020
gttatttatt tttagattat gaatagtttt ttgtattttt ttttgttgtt ggtgttgttg    1080
gatggttgtg atttgtattg tggagatgtt tattttgaag gatttggtta ttgtggtttt    1140
ttgtgatgta tagtttatgt aggagattta tgagaagatt ttaaataagg gaggatttgg    1200
agaagtttaa ggttttgaga gtgattgtgt ggataggtaa gtggttatga taatgtggat    1260
attaaggttg ttagtgagtt ttggattatt atgtgtaata ttttttttat agttgtggaa    1320
gaggtagtta attttattat ttgttatatt tttaattttt gttggaggaa tatgtggttg    1380
tattagatat tgtgggaagg tgtgtgggtt tagagtgtgg agtagatttt tgaggtggtt    1440
atgggagtgg tttttatttg tggaagatgt taggttttat gggttttggt tgtatgaggt    1500
aggtggttgt agtttgagtt aaggtttttg gatttagtgt tgtattttat gatttttatt    1560
tgtaggataa gatagagagg ttttagggta tgtagagggg attttgatat attttgtagg    1620
gtttgttgta ttagagtgat tgtgttttgt tgtattgtaa ttttaatgaa tataattatt    1680
attttgttaa tgattttatt ataaagtaga tgagataggg agtatttttt gtgaatgtag    1740
tttatggtgg tttggtggat gagaaagttt tagtataggt ttttaaggag gataggatag    1800
gaggggtagt ttttggtttg tatgaattgg agttttttag ttttgtttag attttgttga    1860
aagatgtgtt aaattttatt tttatttttt atattgttta ggatagtatg taggtgttat    1920
tggagatgaa ggaggtagtt gttattgaga tttgttgagt tattataggt tgtgttttag    1980
aaagtttaag aaattgtgtg aataaggagt tatttgttat attagtgttt tggttagtaa    2040
tagattagta agtaatttat tttgagttta atggtgtaat atatagatat ttgttaggta    2100
ttgtgggtgt ggttttagga ggattttttg tagttatgga aggaattatt tttggaggtg    2160
ttttaatgat ttataatttt ttgagagtga tatatttttt ttaagttttt ttttttaatt    2220
```

agtttataaa atatggggat aattgatagt attttaataa gtaatagtag agaatgttta 2280
aaggtaatta tttagatata tttgggatta tgagttagtg aaaaatagat gaattaagag 2340
aaataaatta tggttttttt aattgatttt ggatataggt attattgatg ttgttgtgtt 2400
aaaattataa gaattagata attgaagatg ttgtttttta tggaagtgtt gaaagattag 2460
gatgtgattt attaataatt gatttttgtt attgtgtgtt aagtttttta tttgtgtatt 2520
aaattttaaa taataaatgg agtttttttt tttattggtt ttttgggtat aggaggtttt 2580
gaatattttt ttttaggatg ttgtattgag agtttaaata ttgggttatg tgtagtggtt 2640
tatgtttgta attttagtat ttgggagttt gaggtgggtg gattatttga ggtaaggagt 2700
ttgagattag tttggttaat atggtgaaat tttgttttta ttaaaaatat aaaaaattagt 2760
tgggtgtggt ggtgagtgtt tgtaatttta gttattttgg aggttgaggt aggagaattg 2820
tttgaatata ggaggtggta gtaagttaag attgtgttat tgtattttag tttgggtgaa 2880
agagtaagat tgttttaagg aaaaaagaaa aagagtttaa atattaaatt aaaaaatatt 2940
aagaggaaag ttttattttg tgattttagt tttgtgattg tagttttttt attttatagg 3000
ggttgtttat ttttttttgt tagtagtaag attatataat tataaaatgt ggagaaattg 3060
tttgtttgtg gtagatattt gtatatatag gattgaagat agtataggtt tttgtgagat 3120
atttttgttt ttttatattt gttttttttg ttttgttttg ttttgagatg gagttttgtt 3180
ttgttattta ggttggagtg taatggtgag atttggattt attgtaattt ttgtttttta 3240
ggtttaagtg attttttttgt tttagttttt ggagtattta ggattataag tgtatattat 3300
tatgtttagt taatttttgt atttttggta gagattaggt tttattatgt tggttagatt 3360
ggttttgaat ttttgatttt aggtgatttg tttgtttttg ttttttaaag tgttgtgatt 3420
ataggagtga gttattgtgt ttggtttttt atttatattt gaattgtata ttgagtgggt 3480
aagttggtta taagtttagt aaaatttttt gatgatgtaa aaaaaagtat tatgttttat 3540
aagttgtttg tatttaaata tattttttta gttttagatt ttttttttatt ttattgagtg 3600
gaaagttttg aattaaaagt gtttaggaag aataatgttg tattttttta tgtttttttga 3660
aatatttttt tttttttttt ttttttgaga tggagttttg ttttgttgtt tgggttggag 3720
tgtagtggta tgattttggt ttattgtagg ttttgttttt taggtttatg ttattttttt 3780
gttttagttt tttgagtagt tgggattata ggtatttgtt attatgtttg gttaattttt 3840
tgtattttta gtagagatgg ggttttattg tgttagttag gatggttttg atttttttgat 3900
tttgtgattt atttgttttg gttttttaaa gtgttgggat tataggtgta agttattgta 3960
tttggttgtt ttgaaatatt tttaatggta agttgttata ttgtttatga agaaatttat 4020
tttttttaaga tattaataaa tttttttttt ttttatgtga tattttatat aagaatattt 4080
tagatgtatt agatttgatt gattttaaat aaattttatt agatttgtat taattagtta 4140
tatgttttat ttatagattt ttgtgaatta ttgttttttt gtttaaaaag atggtttatt 4200
ttttaggtat agtggtttat gtttgtaatt ttagtatttt gatagattga ggtaggtaga 4260
ttatttgaga ttgggagttt gagattagtt tgattaatat ggagaaattt tgtttttttatt 4320
agaaatttaa aattagttat gtgtggtagt gtatgtttgt aattttagtt atttgggagg 4380
ttgaggtagg agaattgttt gaatttagga gatggaggtt gtggtgagtt gagattatgt 4440
tattgtattt tagtttgttt gggtaataag agtgaaattt taatttaaaa aaaaaaaaaa 4500
aaaagatggt ttattttaag tttttgtata gtatattttt gtttttgtga taaaagaaaa 4560
attttaaaat tgttttaaat agaaaaataa tggttatatg ttttatttta ttttaatttt 4620
ttttttgaga tggagttttt gttttgttat ttaagtttga atgtagtggt atgattttgg 4680
ttggttgtaa tttttgtttt ttaagtttaa gtagtttttt tgttttggtt ttttgagtag 4740
ttggtattat aggtgtgtgt tattatgttt agttaatttt tgtattttta gtagagatgg 4800
ggttttatta tgttgtttag gttagtttta aatttttgat tttaggtgat tagtttattt 4860
tagttttata aagtgttggg attataggtg tgagttatta gtttggttta gttgatagtt 4920
ttaattatgt aaattattat attgattttt attgattatt tttattgtta gtatttttga 4980
atttaaattg gttttggaaa ttttttaatt atagtgaatt ttattttttta tgattttata 5040
aatattgggt attgttgtgg tatgttagaa tatgagtttg tggttgggaa aggtagtatt 5100
atatatgtag tttttttatt tttatgtggt tatataagaa tgatatttga tttgtaatat 5160
tttttttatta agagatatgg attttatatg ggttgtgtaa tgtatttgtg ttttaagtta 5220
agtgatatta tagaaaagtt aaaaagtttt taaaaattg aatatttata aagtagaaaa 5280
gttatagtaa gtaagggtta atttattatg gaagaaaaat tttaaatata tgtagtgttg 5340
tttaagtgta taatgtttat aaagtttaga gtgttgtatt gtaatgtttt agtttttata 5400
tttatttatt ttttattgat tttttagagt aatttttagt tttgtagttt ttatttttgg 5460
taagtatttt atataagtgt attatttttt attttttatg ttgttttttt tattgtattt 5520
tttgtgtgtt tagatatgtt tagatatata aatatttatt attgtgtttt gtgggtagta 5580
agttatttag gtgttgaggt aagagattga gggtatgggt tgttttagta taataaaata 5640
tataaaataa taagaattat attagatata gattatagat atgattatat ataagtatta 5700
ttaattatta gtttgtagta attatttttt atttaaatat tataataatt tttattttat 5760
aattataatt taggaaaaat taggttatat agaggtatga gttgaaggga tatggtgaga 5820
agtgattaga agataagtgt gtgagttttt gttatgtttg gatagggtta ttagagggtt 5880

ttttggttta gtggaatgtt agtgtttggg aagatgtttg ttattaagtg gattgtggtt 5940
tagtagtagt gttagtgtta aggaaaagta tttattattt agtagattag taaagggagt 6000
tttttttttt ttgggggagt ttagagaaga ttttatttta ttatgttttg tggagggttt 6060
gatatgagtt aggtttgttt gtagttattt ggaggtttaa ttgttttttt gtgatgttgt 6120
gttttagtgg ttatattatt ggtttgtttt tatgttttat tttgtatatt tggttttgtt 6180
ttttagatag tagtaataaa attagtgaaa gtattaaaag tttttgatat gtagaaataa 6240
tgtgtaagtt gttttttttt ttttttgtt ttagttgtta aatagggaag ggttttttgt 6300
ttagtggata tgtgatttat gtgattttat ttattattgg agatggttta tattttttat 6360
tttgtttttt ttgttttgta tttaataaat aatagtgtag tttggtattt ggggttatta 6420
ttagtttttg tgttttggtg gtagtggttt atttgggttt aggtgttttt tatttttttg 6480
ttttgtgttt ttatttttat tatttttagt ttttgtatat agggagaaaa atttatagat 6540
tttgtagggt tggtttttat agtgttttaa ttgtatttag tatagtaata tattgtatag 6600
ttttgtagtt taggagtaat aggttatatt atgtagttta agtgtttagt tagttatatt 6660
atttaggttt gtgttagtat attttataat gatgaaattg tttaataatg tatttttttag 6720
aatgtatttt tttttttttt gagattgagt tttattttgt tgtttaggtt ggagtgtagt 6780

ggtgtaattt tggtttattg taatttttgt tttttgggtt taagtgattt ttttgtttta 6840
gtttttttgag tagttgggat tataggtgtg tattattatg tttggttaat ttttgtattt 6900
ttagtataga tggggtttat attatgttgt ttaggttggt tttaaatttt ttagtttttt 6960
aaagtgttgg gattttaggt gtgaattatt gtatttggtt agaatatatt tttattattt 7020
atttatttat ttttttttaa ttgattattt ttgggtgttt tttgtagagg gggatttggt 7080
agggttatag gatgatagtg gagggaatgt tagtagataa ataagtgaat aaaggttttt 7140
ggttttttta ggtagaggat tttgtggttt ttggtagtgt ttgtgttttt gggtatttaa 7200
gattagggag tggtgatgat ttttaatgag tatgttgttt ttaagtattt gtttaataaa 7260
gtatattttg tattgttttt aatttattta attttgagtg gatatagtat atgttttaga 7320
gagtataggg ttgggggtaa ggttatagat taataggatt ttaaggtaga agaatttttt 7380
ttagtataga ataaaatgaa aagattttta tatttatgtt tttttatata gatatggtaa 7440
ttatttgatt ttttaatttt tttttttattt tttttgtttt tttattttat aatattgtta 7500
ttgttattat ggtttgtttt taatgagttg ttgggtatat tttttagatg gggttgtggt 7560
taggtagagg ggttttttat tttttagtag gggtggttgg gtagaggtgt tttttatttt 7620
ttggatggag aggttggttg ggtgggggt tgattttttt ttattttttt tttggatggg 7680
gtggttggtt gggtagaggg gtttttttaat ttttagtagg ggtggttagg tagaggtgtt 7740
ttttgttttt tggatggggt ggttggttgg gtgggggatt gattttttta tttttttttt 7800
gaatggggtg gttggttggg tggggggttg atttttttat ttttttttttg gatggggtgg 7860
ttggtttggt gggggtgatt tttattttttt ttttggatgg ggtggttgtt gggtggagat 7920
gtttttttatt ttttagatgg ggtggttgtt ggatggaggg gtttttttatt ttttagatgg 7980
ggtggttgtt gggtggaggg gtttttttatt ttttagatgg ggtggttgtt aggtagaggg 8040
ttttattttt tagatggggt ggttgggtag agatgttttt tattttttag atggggttgt 8100
ggttaggtag aggtgttttt tatattttag atggggtggt ggggtagagg tgtttttttat 8160
atttagatg atgggtggtt tggtagagat gtttttttatt ttttagattg ggtagttagg 8220
tagaggggtt ttttatattt tagatgatgg gtggttaggt agagaagttt tttattttttt 8280
agatggggtg gtggtggggt agaggttgta attttggttt tttgggggggt taaagtaggt 8340
ggttgggagg aggttgtagt gagttgagat tatgttattg tattttagtt tgggtattat 8400
tgagtattga gtgaataaga ttttgtttgt aattttggta ttttgggagg ttgaggttgg 8460
tggattattt gtggttagga gttggagatt agtttggtta atatagtgaa attttgtgtt 8520
tggtggagat taaaaaaata tgaaaattag ttaggtgtgg tggtgtgtgt ttgtaattgt 8580
aggtatttgg taggttgagg taggagaatt aggtagggag gttgtagtga gttgagaggg 8640
tagtagtata gtttagtttt ggtttggtat tagagggaga ttgtggaaag agagggagag 8700
ggagattgtg gggagagtat atatttttat tattaaatta tgtatgattg tgtatataat 8760
atttttatag gttaaaagta gttaaatatt ggttatgaga tatatttagt ttaagtatat 8820
aaataattgt agaaggagaa agtgaaattt tttttatttt ttgttttatt tatttattat 8880
aaagtatata gaagttagaa taatatttgt tttttgtaat tttagatttt tttttttttg 8940
tttttttaaa tttgttttat gtttggtgat tttttttttt ttgatttttt tttattaggt 9000
tgtaggtttt tggtttatat ttttttttgtg tttttaatta tttttgagtt ttataatttt 9060
tggttttata attttttttgt attaaggagt aataaataaa atttatgttt gaataaagag 9120
gttaaaatag taatttagga tgtttgtgtt tgtgtatttt gggtgtatag tatttaggtt 9180
ggtagtttag tattggttta tttgtgagag taattaagat gatttttgga gggtttaatt 9240
gttttttttag ttttgaatta tattttttag aaggtttagt tgtgtgatta tttttttatg 9300
tagatttgtt tttttttttga ggttgtttag tgtttggggg tagtaggatg ggtttgggaa 9360
ttaagtttat tttaaatatg ttggagtggt tttttttggg ggttaaggtt tgagtttgag 9420
gtgggatttt atgaggtttt tggggtgtga gtggtttgat taggggggggt gattttttttg 9480

gtggggttga tgtaaggtgt ggataggaat tttgatgatt gggaattatt ttgtttggtt 9540
ttgatatgtt tttgattatg ttttttagag gttttttttgg taagattgta tttttttgtgg 9600
taatttagtt tttaatttat gtgttggtgg gatttttagg gttttattag taattatttt 9660
agtgttgggg agggttttgt tgttttgaaa gttggtaagg tttgaggtga gaaatttggg 9720
agagggtggg gttgggggtt aagttttggt tttgtgtggt ggtggaggtg agattgaaag 9780
tgtgtgtttg tgtttgtgtg gaggtgtgtg gtgaatgatt gtgtgattgt gataggttgt 9840
ttttgtgatg agtgtgtttt taatgtttat attgtgtgta gtatggtttt ggagattgtg 9900
gttgttggtg tgtgtgtgag agggagtagg attatgtgtg gtattgtgtg tgtggttgtg 9960
gggagtttgt gtttggttga gtgttattgt gtttgttttt gtagtggtgt gtgattatat 10020
gttgtgtgat tgtgtgtggt agagtttagg tggggatatt tgtgagtaat tgtatgtttt 10080
tatttgtgtg tgtggttgtg gttaatgtgt gattgtgatt gtgatttggt ataggtgtgt 10140
gttgattgtg ataatgtggt tgtgattgtg attgtttttt tggtaagtgt gtattt 10196

<210> 311
<211> 1231
<212> DNA
<213> Artificial Sequence

<220>
<223> chemically treated genomic DNA (Homo sapiens)

<400> 311

ttatttaatt ggtgatatag tttggggttt tgttgttttt tggtggttat tatggatttt 60
tgttttttttt atagaaggta gttattgtaa tgtgtgtggt tttagttgtg ttatatttgg 120
tttttgtgat tagggtttga ggaatttgtg ttatgaagtg tgtgtttgtt attgtaggga 180
ttattagttt tgatgatttt attgtgtgtg tgttggtgtt tgatagtttg taagtgagtg 240
agggaggtga gtaggtggtg gtttggtttg ttatttgtta tttttggtta tattgttagt 300
tgtgttagtt ttgtttgatt gttgtgtttg gaaagaaatt tttgtaattt tattataggt 360
gttgttgttt tttagttggt ttttgtttat gtttggtggg gtttttttttt ggttatttgg 420
ttatttgggg ttgtttgttt ttttttttta ttattttggt tgtttttttt ttttattttt 480
ttagtttttt ttttggtttg tttttgtgtt ttattttttg tttttgtgtt ttttgttttt 540
ttttttaatg gttttgtttt tttgagtttg ggttttttat tgggtttggt agtttttttt 600
taggtttttt tgtggattgt atgttggtgt tggtgtttga gttagttgag tttgttttag 660
agtttggttt ttttttttta gtatttggtt ggagtttgtg tggtttttttt ttttagtgtg 720
tgttgttttt ttagggtttt tggtttttgt agttgattaa gtgaggtaga atgggtagtg 780
tttgggtttt ttttgagttt gtgatttttt ttttaaagtt ttttttttgtt gtggtttttg 840
tgggattttg tgagttgggt tgtttagatt tatgagtgtt tgggaggttt tttgttgggt 900
tttggattag aaaatagttt tgaaaattaa gtgaaattgg ttatagatag gttttttggt 960
ggttgtgttt tgatgttaat gttaaatttt agtagtggtg gtggtttttg ttttgtttta 1020
tatagttagt ttaaaaggtt tttatatttt gatttttttt tttagaaaat tgagagtttt 1080
ggttataatt gatttatttt gtaaattggt agaggaatgg tggtatttga atttttttagt 1140
attgagttgt ttattttgga tgtttatagg tataaggatt ttttgaaaga agatatttag 1200
aaagtagatt ttgttattag ttatgtaggt a 1231

<210> 312
<211> 1231
<212> DNA
<213> Artificial Sequence

<220>
<223> chemically treated genomic DNA (Homo sapiens)

<400> 312

tatttgtgtg attaataata agatttgttt tttgaatgtt tttttttaag gaatttttgt 60
atttgtaaat atttagagta aatgatttag tattgaaggg tttaggtatt attgtttttt 120
tattaatttg taggataagt tggttgtaat taaggttttt gatttttttga aagaggaaat 180
tagaatgtga gggtttttta aattggttat gtaaaataga gtaggaatta ttattattat 240
taaagtttga tgttaatatt ggagtatggt tgttaaaaag tttatttgtg gttgatttta 300
tttagttttt aaaattattt tttggtttgg ggtttgatga ggaatttttt aagtatttat 360

gaatttaagt gatttagttt gtagagtttt gtgaaagtta tagtaggaaa agattttgga 420
aaaggagttg taaatttggg gggaatttag gtattatttg ttttgtttta tttgattaat 480
tgtgagaatt gggagttttg aggggatgat gtgtgttgag gagaggaatt gtgtgggttt 540
tggttaagtg ttgaggggaa ggagttgggt tttgaggtga gtttggttgg tttagatgtt 600
ggtgttgatg tgtggtttat aggggggttt gaggaaaaat tgttgagttt ggtggaaaat 660
ttgagtttgg aggggtggag ttgttggaag gaggggtgga ggatgtggag gtggagaata 720
gagtgtgggg gtagattgga aaaggagttg aagaaatgag ggagaggagt ggttggaata 780
atgggaagag gaaataggta attttgagtg gttgggtagt tggagaaggg ttttgttggg 840
tgtgggtaga agttagttaa ggggtagtgg tatttgtggt agagttgtgg gggttttttt 900
ttgagtgtga tggttaggta aggttaatgt ggttggtagt atggttggag atggtgggtg 960
gtgagttaag ttgttgtttg tttgtttttt ttatttattt gtagattgtt gggtgttgat 1020
atatatgtaa tgaggttgtt aaagttggtg gtttttatgg taataaatat gtattttatg 1080
gtgtgggttt tttaagtttt gattgtaagg gttggatatg atgtaattga ggttatgtat 1140
gttgtagtga ttgttttttg tgagggaggt ggaaatttgt aatggttgtt agggggtggt 1200
ggggttttgg gttgtgttat tggttgggtg g 1231

<210> 313
<211> 16425
<212> DNA
<213> Artificial Sequence

<220>
<223> chemically treated genomic DNA (Homo sapiens)

<400> 313

ggtttttgtg ttttggtttt ttttggtgtt ttttttttgt ttgttttttt ttagtattat 60
gatttggtgt tttttattta gtttagtgtt gtttgttaga gttttgggaa ttttttaagt 120
tttgtaaaag ttttttttgg ttaggttgta gttaatgttt gtagttagag ggtgggtggg 180
tagggttgat ggtggttttt tagttgttag gtaagagatt tttagttgga gggtgggtga 240
gtagggttga tggtgttttt ttagttgttg ggtgagagat ttttaggtgt ttaggttttt 300
tgggagggtg tggttggggt tttttgggtt tggtgtttgg ggagttggtt agtggttatt 360
tatttttgag ttttttagat ttgtgtttat ttggtatatg tgttgtgttt tagggagagt 420
tgttgtgttt tggggttttg gtgttttggt tttgatttgg tttggtttta gttttgggtt 480
tggagttttt attttttggg tattagtttt tgtagtgttt tgtttttttgg tattttttga 540
gagatgtatt ttaaaaatag gattgattta ttgttggtga tgaggagagg gtggtttgag 600
tgtgaggggg tgggtgttag ttgttggaga agtttttgtt aggtaggagg gatgagtggg 660
tttttagttt tgatttttttg gtttttgttt atgtttgaaa atgaaattgg tggtttgggt 720
ggttgttagt tttttgtggt tgatagaaga aagtattata gttgggtttt taaataatga 780
gaatttattt attgagtttt gggagttaga gttttgtagt tgaggggttt gtagggttat 840
gttttttttta ggtttttttag aggggttttt ttttgttttt tttagttttt gttagatata 900
ggtgtttttt ggttgtggtt ttagtgtttt agtttttgtt tttgttttta tttaggtgat 960
ttttttttgtg tttgtgtttt ttgttttttt ttttataagg atatttgttg ttgtgtttag 1020
ataatttagg attttgaatt tgattatata tgtagaaatt ttgtttttaa ataaggatat 1080
ataggtttttg ggttgggag tggatatgtt ttgttggggg ttatgtttag tttttgtttg 1140
taagtgtttt tggattttta ggtgttttta aggtgtagga tgtgagtttg tatttgtaga 1200
tagagtttag aaaagttaga taggttgagg ggtggtgttt gtgaattaga aagaggtttg 1260
agggttgagt gtgggtttag tatgtgtttt attattagtt ttgtaggggt agaggtttag 1320
ggtggggttg gttggtttgt ggaaggtgtg agaagttgtt gtggtaaatt taatttttgg 1380
ttaggagttg gtttattgtg gtttttgggt taaattttgt tggaatttgt ttttgtaaat 1440
aaagttttat tggtttgtag ttatttttat atgtgttatt tgtggttgtt ttttagttaa 1500
gtatggtaaa ggagatttta agtttaatta agttaaaagg gtgatgttgg gttttttata 1560
ggtaagtttt tgattttagt tttgattgta ttattgggaa tgtaatttgt tgaataaagg 1620
aggtgttggt tgggggtaat ggaaggaatt ggttgttatt aggtgttgga gttgtttgtt 1680
agggtgtggt taaggtgtta ttattataga gtagagttat aggggaggaa aggtttttgt 1740
ttgatttttg ggtgattagt ttattaaggt tttgaggaag ttgaggtttt tgaggtggaa 1800
ggtggtggat ggttggttag tgtgtgtttt ttgttgagta ttgagggtga gttatgggtt 1860
atttggtttt tttgtgtatg ggtagttaga ttgtagggaa gatgttttgg ttttatagtt 1920
ggtaatgtag atagatgtgt atttgaatat gtaagtatgt attgagtgtt tattgtatat 1980
agggttttta tattttttttg tagatggttt ttatggtatt gttgtaggtg ggggtgttat 2040
tgtttttagg ttatagatga ggaaattgag gttaagattt taaggttata tagttgggag 2100

```
gttagagtag ggatttttag gggttttgtt ttttagggga agggagtgaa ggggtggttt    2160
gtatggtgtg tgattatttt tagtggaagt tttaatatgg gtgtggttta gtttttttagg    2220
gtttttttaag gtagaggtgg tttgggtttt tgtagtgatt ttataatata ttttaattgt    2280
agatggtgga gattgtgggt tgttttttgg tttaagtggt tttggggttt ttgttttgtg    2340
tggagttatt tgtgtttatg ttgtttttat atattgttat tttttgggtg ttttggtgat    2400
gtatttgagg gatattgttg ttatggtgaa ttttgattat gttgttgtgt ttgatttttt    2460
ttttggggtt gggtgttttg tttttttgttt tggagttttt agagggggtt aggagattgt    2520
ttggaaagtt ttagttgggt aggtggagtt ttgtaggtgt gggaatgagg gggttttttt    2580
tttaaattgg gaatgtttag ttttttttta gttgtaaatg ttttagatgt tgtggataga    2640
tttttttaatg ggaagtgaat gtattgttgt tgtgtttata ttatgttttg tttttgtgaa    2700
ttaagttttt tggtatgtag ttgtgtttaa gggtttatat agggtttgag gttgttttta    2760
tgtagtaagg gtggtggtgt tgagtagtgg ttgtagagtt tgtgtggttt tgaaagttgt    2820
atgtttgttt tttggttttt tgtagaggaa gtttgttgat tgttggtttt tgtggttata    2880
tagatttttt ggttttgtgg gttgtggtta tgtggtttgg ggaggtgggt atggttgggg    2940
agggaagttg agtattatgt ttgttttgga ttttattgta aaaatgagtg tttgtgttaa    3000
atttatttgg gattttggtt tttatgtggt tttgtagggt ttgtgtgaag agtgagtatt    3060
tgttttttag agttttagat gatatgttat tttgtaattt tttttgggga tatttttttt    3120
aatttttttt tgtatgttta tagggatttg gggtgtttgt atgaggtttt aaaatttata    3180
tttttgttga agttgggtat tttggagttt agttgttggg gtttaggtgg gaggaggttt    3240
ggttattaag tttggagtta tgttgggagg gttgggagtt tttttgtgtt gttttttttg    3300
ttttttttagg tagatttgtt gtttttgtta tttataggag tgtttttggg ttaggtggtt    3360
tgtttaaggt tatataggga ttgtgttgtg ttaggtttga gttgtatttg gtggagtaga    3420
tagtattagt tggttgtttg tggtgatagt gatgttggaa aatgggaagg tgggggtgga    3480
gatggggatt tgagttgttt tggagggttt gggaggtaga atgtaggtag gtttttagta    3540
gttgaggaag agattagggt ttgttgtgtt gggtagtggt tgggtggatg gggatgggat    3600
ttaaggttta ttgatttttt ttatttttttg gggttgaagt ttagttataa tttgggaatt    3660
aaaggaatta ggagggggtgg tgggttttga ggtagtaggg agtgggtata ggtgtggttg    3720
gtagggtggt aggataggag tggattggta gaattagggt aggaggttat agttattggg    3780
taggttttat gttgggtttt taagtttgtg attatagatt ttgtagtttt tagtgtggtt    3840
taggggggttt tagggtttgg gatagggtaa atttgttagt gaaggtttag aatttagtta    3900
tgggagttag tgttgggggt tgggtataga tatgaatatt ttgagtttat ttttgttttt    3960
gtagtttgtg ggttttgtaa ggggagggga ttgttttatt ttgtatgttg ggagttgaag    4020
ttttggtatg gttgagtggt tggtttgagg ttatgatttt agtttgggtt tttgtattgt    4080
tatggttttt ttggggatgt taagggtgtg ggtaggtggt gttggttggt gtttttgatg    4140
gttatgtttt ttgtagattg tttgttgatg ttggtttata aggataagtt ggagtgtatt    4200
aagggtttgt gggagtgtag tagtttgatg ttagaggata tttgtgggtt ggagtttggt    4260
ttgttttatg agggtttggt ttatatgttg gttattgttt gtttgtttta ggttattatg    4320
ttgggttttg atagttatga ggttatgtgt gtgtgggatg tttggatttg ttatgtgttt    4380
ggtgagggtg agtgatgggg gttggggttg ggtgggggtt ttttgtttag gtgtgttggg    4440
gttttttatt aatgtgggtt gtagaggtgg gagtggtttt agggaattgt gtaggaagat    4500
gggatattta tgggtgtttt agggtggatt gagttttagt ttgggggtgt ttatgagggt    4560
ttgggagggg gagtttgttg ggttatggtg tttttagagt tttttagttt gattgttagt    4620
tataattgga gtttttaatt taagttggtg gtttttggag ggatgtggtt ttttaaagtt    4680
ttgtttttttg ttttttatat tggatagtgg agtttagagt ttggttagga aggagagttt    4740
taggttgggt ggttttattt tagttttttt ttaaaatagg atgggaggga gggagggaat    4800
agagggaggg gagagggagg ggaagggagt gttttttttgt ggaattggtt ttggaagagg    4860
tggaatgtta gttttagttt tgtaggggtt ttgaatgggt gtttgggggt ttttgggggt    4920
tgagttgttt tggggttgtg agtagaggtg ggtggtgatg gggttttggg tatttagtgg    4980
tagagtaagt ataattttgt attttttaag aaattaaaat tagtgttaaa atttttataat    5040
gagtaaaata gtagaatgtt tattgagttg agttttggtt ttattttggt atttatttaa    5100
taattttagt agttatagtt gttttttttt taaaaatttg ttattttagg ttgggtatgg    5160
tggtttatat ttgtaatttt agtattttgg gaggttgagg tgggtggatt atttgagttt    5220
aggagataga gattagatta ttttgggtaa tgtggtgaaa ttttgttttt attaaaaata    5280
taaaaatgat ggttaggtgt agtggtttat gtttgtaaat tttgtgtttt gggaggttaa    5340
ggtgggtaga ttatttgagg ttaggatttt gagattagtt tggttaatat agtgaagttt    5400
tattttttatt aaaaatggaa aaattagttt ggtgtggtgg tgggtatatg taattttagt    5460
tatttaggag gttgagatag gagaattgtt tgaatttggg aggtagaggt tgtaatgagt    5520
tgagattgtg ttattgtatt ttagtttggg taatagagta ggattttatt ttaaaaaaaa    5580
aagagttggg aggtggaggt tgtggtgatt tgaggttgtg ttattgtttt ttagtttggg    5640
tgatagagtg agattttgtt ttaaaaaaaat aaatataaaa aaatttgtta ttttgattag    5700
tatggatttt gtataatttt atttttttaaa atattgatta aaatattttg gtttttgatg    5760
```

```
gttgagtttt ttggttttgg aggtgagtgt tttgtttttt tagttttgtt tttaaggtag    5820
tagttttttt tgttttattg agtggggaga gagaagttgg gggtttagtg ggaggaggtg    5880
tggttggttt tgtaggaggg tgtaggggaa ggagatatta ggagggaaga attgtatttt    5940
tttagaaagt ttttttgttg ggggtttttt ttagtttggt ttagtggtat gggtttgttt    6000
tttttatttg ttggtgtagg gttatggtat agaggttgtt gggtattgtt taggtagttt    6060
tattttgata gaatgtggtt tgataaggat gtgtttgagg ttatagtttg gagtaggtag    6120
aggaggtaga gaagggggag gtgggatggg aatagggggtt tttttggttt tgggggttgg    6180
agatggagta ggtggggttt ttggtgggga ggttgttgtt tgtttggtgt gttgatttgg    6240
gaagataggg gaggtgggga tagtatttag gattattgtt ttttttgagg tttttagggg    6300
taggggtttg ggtagagtta gtgtatagga ttatgggggt gtttagggtt tgggtgaatg    6360
gttatttgga tggagagagg gttggtggtg ttttgaggtt tttgtggtta ggttggggtt    6420
ttgatataga ttttagattt tttatatttg aattggaggt tgggggattt ttgagttttg    6480
gttttttttt ttgtgatgga gttatatagt tgtggagaga gagttttttt ggagaggtgt    6540
tgggggtggg agtttagtta gggtatttag ttgttttgtt ttttttagta tttttgtggt    6600
gggaggggag ggaggtggag gtttgaggtt aggttttttt gggatttttt tgtttttgaa    6660
taaagatagt ttttgggagg atggtaagga gagaagtgta gtttttaggt ttttttgtgg    6720
ttgtgaggag tgttatttgt ttattttgtt gttgttgttt ttttggttga tatttgggtg    6780
gttgttttta tttgttttag tttggttttt gtttttgttg ttttttttttg agatgttttt    6840
ttattttatt tgtttatagt ggtttttgtt gttttttttt atgatgtttt tttgttttgt    6900
ttgattgtga tgttttttttg ttttgtttat ttttgatgtt tttttatttt atttgtttat    6960
gatgtttttt tgtttttgttt gtttgtgatg tttttttatt ttatttgttt gtgatgtttt    7020
tttattttat ttgtttgtga tgttttttta ttttgtttgt ttgtgatgtt tttttgtttt    7080
gtttgtttgt agtgtatagg ttttatgtga tagtggtttt aggtattaag ttggagagtg    7140
gtttggttat tttgtatttt tgtaatgatg tttttgtttt ggttagggat atttttttgg    7200
ttgttatggg gtagtggaag ttgtttgatt tttggtgtta tggggttgtg ttaagtggat    7260
ttatttttga aggtgggatt aggtgtgggt attgtaagta tggatgtgtg gggttattgg    7320
gtagtagtag tatttttttat tttttttgag aattgttggt tttgggttgg ttgattttat    7380
ttgtaggttg gtttgttggt atttttagaa tgtgttggta ggtggatgga gttttttttat    7440
gtttgatgtt taaggttttt tgtttgtagg aattttagtg tggagtgttg aggaggggtt    7500
ttggttattt ttgttttttt ttattggagg gagagagttt agggatgtga ggaggggttt    7560
gaggttatag agttttagag ttgtttatat ttagatttta tggaaaggaa aaaataaaga    7620
ataaatgtat ttagtagtgg ggagtgtgag tttttgtata agtattgtag aaatatattt    7680
ttggattaag aatttttggt ggatatgtta gttattttgt ttgtgggttg tttagagttg    7740
ttttaatgtt tttagtgagg tggttaggtt gtgggtgggg atggtttgag ttttttgggt    7800
ttgtgaaggt ttgagagttg tttttattta gttaggggag tgagggaaat tggagtagga    7860
aggtatttag ttttgtttag agagtttgag gtgtttgttg gggttgtgta ggagtaggtt    7920
gttttagtaa ttttttttat ttttttttat gttgggtgta gatgtgttag gttgttttgt    7980
ggatttgtag tttttgtgga gggtgtgatg tttttttttt tttttttgga ggggtagttg    8040
tttattggtt gggttgaagg tgggtagagg gatttttttgt gggtttggtt tggggttgag    8100
aggattgagg gattgtattt ggtttatatt ggtgggggtt ttggtggagt ttggatgtgt    8160
ttgggtggag gaaatgtagg taaggatatg ggttatattg gttgtttggg attttatttt    8220
gtaaaggtta gttggggtat gggtgtaagt ttatttgggt gttatgtttt gttatttttt    8280
gtgttgggtg ttgggagtaa atttagtagg ttttgtggta ggagttagtt ttgttggggt    8340
ttttgaggag atgatatttg atttttgaaa ggggaatagg gtagtgaggt ttaggtttta    8400
tggattgggg gtgtatatag ttagttgtag gaggtatgtg tggtagagtt ggtttggagt    8460
tttggttttt ggggttggta ggggttatag ttagagttta aagggttttg gtttttttgg    8520
gattggagag ttagtatgga tgggtagggt tgttgtttat gttagttgtg agttggtgtg    8580
gggggtgggg tggggggtgg tttttgggag agttaggata tttgttgggg ttttatttgt    8640
ttttaggttt tttttgtggt aggttagagg tttagtattg gggttttggg agttattggg    8700
gagtgggttg gtggtggttg ggtatagtgg gtgggtttag tgtttttgtt ttttgttgtg    8760
tgggaagtta gagttgtagg ttgtagttgt tttgatatag gtagtttgtt ttgtttgttg    8820
tgtgttaggt ttgggggtgt ttggaggagg gggatttata tttttttttt tgtttgtgga    8880
atttttttggt atgggtaggt agttggttgg ggtggtgttg tgtgtttatt ttttaagttg    8940
gggtatagta ggaggatttg tttttttgga gatttgttta ttttgtttgg gttataatgg    9000
aatggggata ttggatgttt tttttttgag gttgtggagg tttatatttt tgttgaggtg    9060
tgggtggttt tttttttgtt ggtaaataat tttatatttg gggttgtgtt tttttttagg    9120
gtgaggttat tgtgttgttt ttttgggttg tttaaaagtt gggggtattt ttgtggggtt    9180
gtttgttttg ttgttgtttt tttggggtaa gttgttagag tagttttatt tgtaaatttg    9240
taaattaggt tggttttata gaatttgaaa atttgtaaat tgggttggtt ttatagaatt    9300
tataaatttg tagattgggt tggttttata gaatttgtaa atggggggttg gttttataga    9360
atttgttttt gtagtttagt gttgaatgtt tttgtttttg gtggtatttt ggtttgagag    9420
```

aggaagggtt ttgggaattt ggttattgat ggttgtatat tttgtagggt aggtgggttt 9480
tggaggttag gttggaaggt ttggttgagt tatttatttt tgtttttagt aggttttgtt 9540
gggttgtatt tttttggaga taggtgtggt tattattttg ggtggtttag gttgggtttt 9600
agatttggtt aagtggtggt gtttttaatg tagtattgag tgtaggttgt ttggttggag 9660
ggatggggag tttgtggtat gtgtgggtag tttggggttt tttgtgggag ggagttggta 9720
atttgggtgg tttaggggta gtggtattag ttttttgggg tttttgtagt agatgatttt 9780
taagagaggg ttaagtgttt agaatgggtt ttttttgtag tttggaggtt agaaatttta 9840
aattaagggg tttttagggt tggttttttt taaaagtttt tggggaggat ttatgggaag 9900

gttttttttg tttttttttag ttgttggtgg ttttggtggt tttggtttgt ggttgtgtta 9960
ttttagtttt tgtgtttgtt ggtttttgtg tgagtttttt ttttagtttt ttgtagtttg 10020
tggatttagg gtttatttgg atgatttagg gtgattttat tttgagatta ttaattttat 10080
ttgtagaggt ttttgtttat aggttttggt atgtgaatat attttgtggg ttattatttg 10140
gttgataatt ggggttattg ggatggagaa atatttagaa tttgaatttt aataagtttg 10200
tgggtgttgt tatatttagt ttggaggtta tagttgggtt ggagttgtgg gaaggttttg 10260
gaagtttttg ggtgtaggaa atttatattt tttttttttt tagggatagg ttttagtttg 10320
gtttttgatt tttagaattg ttttatttgg ttttgttata gttagttttg tagagagtag 10380
agagggtgat tttggtttaa ggtgatggtt gtgttttatt tattttgaga tattgggtgt 10440
ggttgtgtat tatgggggtt ttggtggggg ttgggtggag ataggtttgg ttggttttgg 10500
tgtttggggg gtagaggttt ttttgtggat agttgtttgt ttagaatggg ataggaggtg 10560
gtttggtgat agtaattttt gggtttttag tgtggtttaa ttttttgttt ttgttggtta 10620
tgggtttgta gtgtggattt ttggagtttg gtttgagtgt atttggtata tggtaggtga 10680
tggtttgtgg atagtttagg tttgtaggtt ttagttttat atatgggtaa gtgattgggt 10740
tttgttttttg gggatggatt gtgtgtggtg gagagtgatg tagagagggt tatttgtttt 10800
gggataggtt tagtttgtag tatagatggt ggtgggtttt agagagggtg agtttattta 10860
gagtttagga agggagggtt ttggaggtgt agggggatgt ttgagttggg ttttgagggt 10920
gaatggaagt ttttggaggt gttttttttt ttttttttta tttgtttttt ttagttgggt 10980
ttagtttaag gattatattt gtttttttgt ttagtttttg ggggttgttt ggagtatttg 11040
aaatttattt tgatttttaa aaatgattaa gaatgtggtt aggtatagtg gtttatgttt 11100
gtaattttag tattttggga ggtggaggtg ggaggattat ttgaggttag gagtttgaga 11160
ttagtttggt taatatggtg aaattttatt tttattaaaa atataaaaat taggtaggtg 11220
tggtagtagg tgtttgtaat tttagttatt ttagaggttg aggtgggaaa attatttgaa 11280
tttgggagtt ggaggttgta gtgagttgag attttgttat tgtagtttag ttaaggtaat 11340
agagttaggt tttgttttaa aaaaagaaga gtaaagaatg aaaatttgaa ttaggaggtt 11400
tttgttatat gaatgtggta tttgtttagt tttggagagt tttttgttgg ttatttttgg 11460
ttattgtagt ttgtgggggt agtttggata gagttggttg gaattttggt ttttgttagg 11520
tagtagggag tgtttttagt agttttttat tgttgggtgt tgggtttagg gattagatgg 11580
tttggtttta gaggttgtgt gagattgagt gaggtatttt tagtttttag agggtgtgag 11640
gggagttagg aggggtatag attttaggtt gggggtattg tggggaagtt ttttggaggg 11700
gtgagtttta ggggattttt gtagggtgaa taggagttag tttagttgtt agagggtaag 11760
gggtggaggg ggggtgaaaag ggtattttag atatttttga gtatgaatgg gtgttttgaa 11820
gggttgtgta ggagttttgt tgggggggat aggaggtgtg agggtggagg aggaggagga 11880
gatagggttg ggttgtagag agttggttaa ttggattagt gtttgtttgt attattgttt 11940
tggagtagta gggagttatt gaaggtgttt gagtatgggg agtgttttat atgttaatgt 12000
tgattttggg gtgtagatga gtttggagga agaggttggt aggggttaga gaaggttttg 12060
attgaggttt agaagatgga gggtgaggtt gttttaggta gtagttgttt ggttgggggga 12120
tgggaatttt gtggattgta ggaatgttaa taaggtggtg atggataagg ggaggagggt 12180
tggtttggga agggggggtt ttttagagtg gtgtttttgg attggggttg ttttaggagg 12240
gtagaggagg ggtgggagtt aatatttatt gttaggtttt aggtttttttt ttatttgaga 12300
tttattaggt ggggttattt ttaagtgtat aagaagttgt tattttgggg gatggagagg 12360
gttgagggtt atgttattaa gatttttttag ttttgtttat gtttatttgg tatgagttat 12420
ttaggatatt ttggttattt tgatgtttgt tttttgagga gttgggatta tgagtattta 12480
agatttgat attaaggggt agttatgagg gtatttaaga atatttagat ggaaattatt 12540
ttagatttaa ttggtttatt aaatgtttgt gtggaggagt attttttttt ttaagttggt 12600
gagttttgga gttagtgtaa tagttttttt attggagatg tgtttttttag gaagtaggtg 12660
tgtttgtaat gtgggtgagg tttgtttgtt gtttagtaat tttagttttt gtttagagtt 12720
ggtgttgttt tgagttttgt agtttttggt tttttgtttg tgttttgttg attttgtttt 12780
gttttttttgt gtttttttatg tttttttttt ttattttttat gttttttatt ttttataata 12840
gggtttttat atagtttttt gggatatttg tttgtgttta aagatgtttg atgtgttttt 12900
tttattttttt tttattgttt atttttttaat agttgagtta atttttattt attttgttat 12960
atttagtggt tggtggttgt tttttggttg gtaattttgt gggttaggga tttggatggg 13020

gtttagtgag gatagtttat ttttgtttta tgaggtgttg tttggggttt tttgggtagt 13080
ggtatttggt tatggtgggg tttaaaggtt taggaaagta ttgtgttggt tgttggttgg 13140
gttgttttag ttttttttta ggaattttat tttttgatag gatattttgg gttttttaat 13200
aggttttaag agagtagaag ttgttaggtt tttgatggtt ttggggtagg gttgggagag 13260
tattttttat gttattttat gttagagtta ttattggttg gttttgattt tagggaggga 13320
gtggattagt ttttggagaa ggagtggttt ttgagggtag ggatagggga aatagagtgg 13380
ttggttttgt tgtagttata tttgtgtgat atagtaaaat ggggtaggtt ttgttttttt 13440
tttttttttt aaatatatag gtaaatagga aatatgtgta ttttgttttg gggaaggggt 13500
agggggggtgg ttagtagttt tttatttatg tttttttatt taaggttgag tattggggt 13560
tttttggtta tgggtgtttg ggtggatatt gagattatag atggtttgtg ttttttgttg 13620
gtaaggtgtt tagtttggta gttgggatgg tagtaggtgg ggttgaattt tagaggaggt 13680
gtttgtgaga gtattataga ggggttgttg gggttagggg attgaagggg gttagaggtt 13740
aagaaaagta ttatgaaagg tgttgttatg tggtttgagt ttggaaaggt ttggggtgtt 13800
ttaggtggtt aggggtgtgg agggtggggg tggtagtttt ttgagtggag atgtaggggt 13860
gtggagggtg gggatggtag ttttttgagt ggagatgtag gggtgtggag ggtggggatg 13920
gtagtttttt gagtggagat gtaggggtgt ggagggtggg gatggtagtt ttttgagtgg 13980
agatgtaggg gtgtggaggg tggggatggt agtttttttga gtggagatgg taggtagatt 14040
tggtgtgtag agggagtttt gagtattttt aagattgggg ggggttgggt gggagtgggg 14100
gtggagtatg aatttaggga ggtttagtga gagtttagtt gtgtggtgtt ttgggaggtt 14160
atggtttaag attttggtag attagtattg gagatgggta ttttttttta gggttattga 14220
tgttagtttt gttggtatgg atgatggggg tggttggtga gggtgttgtg tgggtatgtg 14280
ggagtttgag gttggtggtg ggttgtgttt taggttttgt ttggttgatt gtgtttttgg 14340
ggtgagtgat gttttgtaga tgttgtatgg aatgtggggt tggttttatt tttttttgtaa 14400
tgtggttttt tatagttggg ggatttattg ttttgtggtt attgttagtt gtttgtaagt 14460
ttaggattag agagtttggt tttttgtgag agttggggtt gtgttggttg agttttttat 14520
agggaggggt ggattatggg gatagggagt gattgtttgg tattgagtga gttagttagt 14580
agatagtatg atggtttttt ttttgtttgt ttttagggtt tggtgtaggt tttttttttgt 14640
ttattttatt tagtttgggg gttttatttt attatggttt ttatgagttt tttggggtat 14700
ttttttggat ggtattgtag tgggtattgg tttttttttt atgtttattt ttaattttga 14760
ttaggagtat tttttattta tagtaggatt ggaagtttta ttgggttaga aggtgtatag 14820
ggggttaggg gatttggatt tgagtttatt ttgtggttgt gtgatggtgg ataggtggtg 14880
tgtttttttt gggtttttagt gtttttttttg ttaaagtagg aatggtgttg ttttatgatt 14940
tggtttgggg atattatgtg gaaggaagta tttatagtat aaggtgttgt ttgtttttgt 15000
gtgtttgggg tggttgttaa ggggttggta gtgttgttat gtgttggggt tggtttgagt 15060
tttgtgtttt tggaatttat agtttgtgtt ttgtgaaggt agagtatgat tgggtagtag 15120
gtagtggggg ttttaatgtt gagatttttt tttttttttaa tttttgttta ttttggtgtt 15180
tttttttttg tgtatttttt tttgggtaat gggtagaggt tttggagtgg gtgggatttt 15240
tgtgaggtga tgttagttgg agtgtttagt tgggtgtagt tagtttagtg gtttatttat 15300
gtggttgtta gggttttatt tatttttggt tgggagggta gtgtttttgt gatttgtagt 15360
tggggttttt ttatgggtgg tggttgtatt gttttgttag tgatgttggg ttggtttttg 15420
ggggtttttt tgttgttgtg ggggtgtagg ggtggatgtt ggttttagga tgttgtatat 15480
ggttttaggg tagtatttat atattggtag ttttagtttt ttttgaaaga tgattgagga 15540
ggtggggagg atgtggtgtt ggttagtagt gatggttgta gtatgtgtgg gtaggtgttg 15600
tgttatattt tttataaata tttgaatgta gtttttttaa tagtttgggg gaaggaagtg 15660
tggttatatg gtagtttttt tgggatgtga ttttagtttt aggtggtata gttttttttt 15720
tttgtggttt tggagtttttg aggggatttt atttgatgtt tttggggttt tttggttgtt 15780
ttatttgttt ttaagatggt tgtgggggta gggttgttgg gattttgggg tagaggaggg 15840
attgattaag tgtttgttgg gtggtagagt agttatattt ttttattttt ttaagtaagt 15900
ttggaaggta ggtatgtggg tgggggtatt tatttgttta gggtagggta tttttttttt 15960
tttttttatg ttttgttggt tgtaggattt aggatgggtg gggtagtatt tattttagtg 16020
tttaagtttg aagggtgtgg tggggttttt ggggagttta gaggttttgg ggatttagtt 16080
ttatagagtt aggtggggtg ttggttttgg gtatttggat ttatggggtt aggtggggtg 16140
ttggttttgg gtatttgatt ttgtggggtt aggtagggtg ttattgttgg tttttggtgg 16200
agtttgttgt ggttttttgt gttttttttt tttagggttt ttagtttgtt tttgggttag 16260
attgatttgt ttttgttttt tttttgtagg ggttggtgtt tttttttttgt ttttggttga 16320
gggggagtag attagttttt tgtttgattg tattgtttga ggtatttttt ttattaaggg 16380
tttttttggg ttgtggttgg ttttattagg tgtgtgtggg agttt 16425

<210> 314
<211> 16425
<212> DNA

<213> Artificial Sequence

<220>
<223> chemically treated genomic DNA (Homo sapiens)

<400> 314

```
aggtttttat atgtatttgg tagaattggt tgtagtttaa aggggttttt ggtgggggag      60
atgttttgga tgatgtagtt gaataggaag ttgatttgtt tttttttggt tgaggatagg     120
aagaagatgt tagtttttgt agaggaagga tagagatagg ttagtttggt ttgagggtgg     180
gttggggatt ttgaagagga ggaatatagg aaattgtagt aaattttatt aagagttgat     240
aatgatattt tgtttggttt tatgaagtta gatgtttaga gttgatattt tgtttggttt     300
tgtgaatttg ggtgtttaga gttgatattt tatttggttt tgtgaagttg ggtttttaga     360
gtttttggat tttttaggga ttttgttgta ttttttaaat ttgggtgttg gggtgagtgt     420
tgttttgttt attttaggtt ttgtagttga tagggtatgg ggaggaggga gggggtgttt     480
tgttttggat agatgggtgt ttttgtttat atatttgttt tttgggtttg tttgggggga     540
tggggggatg tggttgtttt gttatttagt aggtatttga ttagtttttt ttttgtttta     600
aggttttagt gattttgttt ttatagttgt tttgagggta aatgaagtag ttaggagatt     660
ttagagatat taagtagggt tttttttaggg ttttaaggtt ataggagaag ggagttgtgt     720
tatttggagt tggggttata ttttaggaag gttgttatgt gattatattt tttttttttg     780
ggttgttggg agggttgtat ttagatgttt gtaaaaggtg tggtatggta tttgtttata     840
tatgttgtag ttgttattgt tggttagtat tgtgtttttt ttattttttt ggttattttt     900
taggggaagt tggagttgtt agtgtgtggg tgttgttttg aggttgtgtg tagtattttg     960
gggttagtat ttatttttgt atttttatag tagtaggagg gtttttaggg attagtttag    1020
tgttattggt agagtgatgt agttgttatt tgtggagaag ttttagttgt gagttatggg    1080
ggtgttgttt ttttggttag gggtgaatga agttttggtg gttgtgtgaa tgggttattg    1140
ggttggttgt atttggttgg atattttggt tggtgttatt ttataggaat tttgtttgtt    1200
ttggggtttt tgtttattgt ttaaagaaag gtatgtagga gggaaggtgt tggggtgggt    1260
aggagttggg gggaagggaa attttggtat tagaattttt gttgtttgtt gtttagttat    1320
gttttgtttt tatggaatat aggttgtaga ttttagggat atggaattta ggttagtttt    1380
gatatgtggt gatgttgttg gtttttttggt agttattttg ggtatatgag ggtgagtggt    1440
attttgtgtt gtggatgttt tttttttatgt agtgttttta aattgggtta tgggatagtg    1500
ttatttttat tttagtaaaa aggatattga ggtttagaga aggtatgtta tttgtttatt    1560
attatatagt tatgaggtgg atttaagttt aagtttttttg gtttttttgtg tattttttgg    1620
tttgatagag tttttagttt tattgtgggt gaggggtgtt tttgattaga attggaggtg    1680
ggtatggagg ggaggttaat gtttgttgtg atgttattta ggaggatgtt ttgagaggtt    1740
tatgagggtt gtgatggggt ggggttttta agttgggtag ggtgggtaga agagagtttg    1800
tattaggttt tgggggtagg taaggagaag gttgttatgt tgtttgttgg ttggtttatt    1860
tagtgttagg tagttgtttt ttgtttttgt ggtttatttt tttttgtgaa gggtttagtt    1920
ggtatagttt tagtttttat gaaaggttag gttttttaat tttgagttta tagatagttg    1980
gtagtggtta tgggataatg agtttttttga ttataaaaaa ttatattatg gaaggagtgg    2040
agttggtttt gtattttatg tggtgtttgt ggagtgttat ttattttgaa gatatggtta    2100
attgggtaag gtttggagta tagtttattg ttagttttgg gtttttatgt gtttatgtga    2160
tatttttgtt agttattttt attatttatg ttagtagagt tgatattagt ggttttggag    2220
gaagatgttt atttttaatg ttggtttgtt aaggttttgg gttatggttt tttaggatat    2280
tgtatgatta ggtttttgtt gggttttttt gggtttatgt tttattttttg tttttattta    2340
gtttttttttg gttttgggaa tgtttaaagt ttttttttgta tattaggttt gtttgttatt    2400
tttatttagg aggttgttgt ttttattttt tatattttta tatttttatt taggaggttg    2460
ttgtttttat tttttatatt tttatatttt tatttaggag gttgttgttt ttatttttta    2520
tatttttata tttttattta ggaggttgtt gtttttattt ttttatattt tatattttta    2580
tttaggaggt tgttattttt atttttttata tttttggtta tttagaatat tttagatttt    2640
tttagattta ggttatataa tgatattttt tatgatgttt tttttggttt ttggtttttt    2700
ttagtttttt gattttgata gtttttttgt ggtgttttta tggatgtttt ttttggggtt    2760
tagttttatt tgttgttgtt ttggttgtta ggttgagtat tttgttggta gggagtataa    2820
gttgtttgtg gttttagtgt ttatttaagt gtttataatt aggagatttt tagtgtttgg    2880
ttttgggtgg aaagatatag gtgagaggtt gttgattgtt tttttgtttt ttttttaaga    2940
tagggtgtgt atgtttttttg tttgtttgtg tgtttgggga aagggggggag aatagggttt    3000
gttttatttt gttatattat ataggtgtga ttgtagtaaa gttggttatt ttgttttttt    3060
tatttttgtt tttagaggtt gtttttttttt tagaggttgg tttatttttt ttttggaatt    3120
gaagttagtt agtgatggtt ttggtataaa gtggtatgag ggatgttttt ttagttttgt    3180
tttaagattg ttagaggttt ggtagttttt gttttttttgg aatttattga gaagtttaag    3240
```

```
gtgttttatt ggagggtgag gtttttggag ggaaattgag gtaatttagt taatagttgg    3300
tatagtgttt ttttgggttt ttgggtttta ttatagttaa atgttattgt ttgagggatt    3360
ttaggtagta ttttatgggg tagagatgag ttgtttttat tgagttttgt ttagattttt    3420
ggtttataga attgttagtt agggaatagt tgttagttgt taggtgtggt agggtaaata    3480
ggagttagtt tagttgttag agggtaagtg gtggaggggg gtgaaaaggg tatgttagat    3540
atttttgagt ataaatgggt gttttaaagg gttgtatagg agttttgttg tgggggatag    3600
gaggtgtgag ggtaagagga aagagtatgg aagatgtaga gaagtaggat agagttagta    3660
gaatataagt agaaggttag gagttgtggg gtttagggtg gtgttaattt tggatagagg    3720
ttggggttgt tgagtggtag gtaaatttta tttatgttat agatgtattt gtttttttgag    3780
aaatatgttt ttagtgaaaa agttgttgta ttaattttag gatttattgg tttgagaaag    3840
aaaatgtttt tttatgtaga tatttaatga gttaattaga tttaagatgg tttttatttg    3900
aatattttttg agtgtttttg tggttgtttt ttgatgttag aattttaagt gtttataatt    3960
ttagtttttt agagaataaa tattggggtg attagggtgt tttggatggt ttatgttagg    4020
taggtatggg taggattgag aagttttgat aatatgattt ttagtttttt ttatttttta    4080
aggtggtagt tttttgtata tttgggagtg gttttattta gtgggttttg ggtggaggaa    4140
ggtttggagt ttggtagtgg gtgttggttt ttattttttt tttgtttttt tgaagtagtt    4200
ttagtttagg ggtattattt tagaaggttt tttttttttg ggttggtttt tttttttttg    4260
tttattatta ttttgttaat attttgtaa tttataggggt ttttattttt taattaggta    4320
gttgttgttt ggggtagttt tgtttttat tttttggatt ttaattagaa ttttttttgg    4380
tttttgttag tttttttttt taagtttatt tgtattttgg ggttaatatt agtatataaa    4440
atatttttta tgtttaagta tttttaatgg tttttttgttg ttttgagata atggtgtaaa    4500
taaatattgg tttaattagt tagtttttta taatttagtt ttgttttttt tttttttttt    4560
attttttatgt tttttatttt ttttaatagg gtttttatat agtttttttgg gatatttatt    4620
tgtgtttaaa gatgtttgaa gtgtttttttt tattttttttt tattttttat tttttgatag    4680
ttgagttaat ttttatttat tttgtagaag ttttttgagg tttgtttttt tagagagttt    4740
ttttgtagtg tttttagttt ggggtttgtg tttttttttgg ttttttttttgt atttttttgag    4800
gattgaaaat gttttattta gttttatgta gtttttgagg ttggattgtt tgatttttag    4860
gtttagtgtt tggtaatgag aaattgttga aggtgttttt tgttgtttga tagaggttag    4920
ggttttagtt agttttgttt aggttgtttt tgtaggttgt agtggttaaa ggtggttggt    4980
agggggtttt ttagagttgg gtagatgttg tgtttatgta gtaagagttt tttagtttag    5040
gtttttattt tttatttttt tttttttgag atagagtttg gttttgttgt tttggttgga    5100
ttgtaatggt gggattttag tttattgtaa tttttaattt ttaggtttaa gtgatttttt    5160
tattttagtt tttggagtag ttgggattat aggtatttgt tattatattt gtttaatttt    5220
tgtattttta gtagagatgg ggttttatta tgttgattag gttgatttttg aatttttgat    5280
tttaggtgat tttttttattt ttgtttttta aagtgttggg attatagata tgagttattg    5340
tgtttggtta tatttttaat tatttttaaa agttaaaatg agttttaaat gttttgggtg    5400
gtttttagag gttgggtaaa gaggtagata tggtttttgg attggattta gttggagaga    5460
gtaggtaagg ggggaaaaag gggtattttt taaaagtttt tatttatttt taaggtttag    5520
tttaaatgtt tttttgtgtt tttaaagttt ttttttttta ggttttgggt gggtttattt    5580
tttttggggt ttattattgt ttgtgttgta agttgagttt gttttagggt gagtggtttt    5640
ttttgtatta tttttttatta tgtatagttt atttttgaag gtggggttta gttatttgtt    5700
tgtgtgtgag gttggagttt gtagatttga gttgtttata gattgttgtt tgttatgtgt    5760
taagtgtgtt tgggttaagt tttaggagtt tatattatag gtttgtgatt aatagagatg    5820
gggaattggg ttatattggg gatttggggg ttgttgttat taagttattt tttgttttat    5880
tttgaatagg tagttgttta tagagagatt tttgtttttt aggtattaag attagttagg    5940
tttgttttta tttgattttt attagggttt ttgtgatgta tagttatgtt tagtgtttta    6000
ggatgagtga ggtgtagtta ttgttttggg ttagggttat ttttttttgtt ttttgtagag    6060
ttggttgtgg tggggttaag tgaggtggtt ttggggattg ggggttaggt taggatttgt    6120
ttttggggaa ggagggaatg tggatttttt atatttaaga atttttaggg ttttttttatg    6180
atttttagttt agttgtgatt tttagattga atgtgatgat atttatagat ttattgggat    6240
ttagattttg gatatttttt tgttttagtg gttttagttg ttggttaaat ggtggtttat    6300
aaaatgtgtt tatatattag ggtttgtgaa taggagtttt tgtggatgga attgatggtt    6360
ttgggatggg gttattttgg gttatttagg tgggttttaa gtttatagat tgtaagagat    6420
tgaagaggag gtttatgtag gagttagtag gtgtagggat tggagtggta tagttatgag    6480
ttgaggttgt tggggttatt agtggttgga aggggtagga aggatttttt tatgggtttt    6540
ttttaagagt ttttggaaaa agttaatttt gaggattttt tgatttgggg tttttggttt    6600
ttaggttgta gaaggaattt gttttggata tttggttttt ttttgggggt tatttgttat    6660
agaagtttta ggaagttgat gttgttgttt ttggattatt tgagttgttg gttttttttt    6720
gtaggaggtt ttaggttgtt tatatgtgtt gtagattttt tgtttttttta gttagatggt    6780
ttgtatttag tgttgtgttg agaatattat tgtttggttg agtttaagat ttagtttaaa    6840
ttatttaggg tggtggttat gtttgttttt aggaggatat agtttagtag ggtttgttgg    6900
```

ggatagggat gggtggtttg gttaagtttt ttagtttggt ttttagaatt tatttatttt 6960
atggggtgtg tagttgttgg tgattgaatt tttagagttt tttttttttt aggttagggt 7020
attattgggg atgagaatat ttagtattga gttgtaaagg tgggttttgt ggggttagtt 7080
tttgtttgtg ggttttgtgg ggttagttta gtttgtaggt ttgtgggttt tgtggggtta 7140
gtttggtttg taggttttta ggttttgtgg ggttagtttg gtttgtaggt ttgtgggtgg 7200
ggttgtttta atagtttgtt ttggagaggt agtagtaaga tagatggttt tataagagtg 7260
tttttagttt ttgagtagtt taggaaaatg gtatagtagt tttgttttgg ggagaagtat 7320
agttttaggt gtgggattgt ttattagtgg aaaaggggtt atttatattt tggtagggat 7380
gtgaattttt atggttttag aggaaaaata tttagtgttt ttattttatt atggtttaga 7440
taaaataagt aggtttttaa ggaaatgggt tttttgttg tgttttggtt tagagggtga 7500
gtatgtggtg ttattttggt tggttgtttg tttgtgttaa ggaattttat agatagaaag 7560
gaaggtatgg gttttttttt tttaggtatt tttaaattta gtatataata gataggatag 7620
gttgtttgtg ttaggataat tatagtttgt agttttggtt ttttatatga taaaggatag 7680
ggatgttggg tttatttatt gtgtttagtt attgttaatt tattttttag tggtttttga 7740
agttttagta ttgaattttt ggtttgttgt agggagggtt tgggggtaga tgggatttg 7800
ataggtgttt tagttttttt gggaattatt ttttatttta ttttttgtat tagtttatag 7860
ttggtatagg tagtggtttt gtttatttat attggttttt taattttaga ggggttaagg 7920
tttttttggat tttggttatg gtttttgtta gttttagggg ttgagatttt gggttagttt 7980
tgttgtatgt gtttttttgta gttggttgtg tatgtttttta gtttgtggag tttgagtttt 8040
attgttttgt ttttttttta agggttagat gttgtttttt taaaggtttt aatagggttg 8100
gtttttgtta taagatttat tgggtttgtt tttagtgttt agtgtagaag gtggtagaat 8160
gtggtattta gatgggtttg tgtttgtgtt ttagttggtt tttgtaggat gaggttttag 8220
gtggttagtg tggtttgtgt ttttgtttgt gtttttttta tttagatata tttaggtttt 8280
attagagttt ttattagtgt gagttaagtg tagtttttta gtttttttag ttttaaatta 8340
ggtttatagg aggttttttt gtttgttttt agtttggtta atgagtagtt gtttttttag 8400
agagaagaga ggaggtgtta tattttttgt ggggattgta ggtttatagg gtaatttggt 8460
gtatttgtat ttaatatggg agaaagtgga gggggttgtt ggaataattt gtttttgtat 8520
agttttggtg ggtattttag atttttgggg tagggttggg tgttttttttg ttttgatttt 8580
ttttattttt ttggttgagt gaggatagtt tttaggtttt tataggttta gaggatttag 8640
gttattttta tttatggttt ggttatttta ttggggatat tagggtagtt ttgggtggtt 8700
tataggtggg gtggttggta tgtttattag gaatttttgg tttaagaata tgtttttgtg 8760
gtgtttatgt gggagtttat atttttattt gttaaatgtg tttgtttttt gttttttttt 8820
ttttatgaaa tttggatatg agtggttttg aggttttgtg gttttaggtt tttttttgta 8880
tttttgaatt tttttttttt agtgaggaag agtagaggta gttgaggttt tttttgata 8940
ttttatattg aggtttttgt gggtagagga ttttggatat tgagtgtggg gagattttgt 9000
ttatttgttg gtgtattttg gaaatgttag taggttagtt tgtaagtggg gttggttggt 9060
ttgaagttag tagtttttag gggaagtggg gggtgttgtt gttgtttagt gattttatat 9120
atttgtattt atagtattta tatttggttt tgtttttaaa gatgaatttg tttggtatgg 9180
ttttgtagtg ttggaggtta gatagttttt attgttttgt gatagttggg gggatgtttt 9240
tggttaagat gaggatatta ttgtagaggt gtagggtagt tgggttgttt tttaatttgg 9300
tgtttggagt tattgttata tggaatttat gtattgtggg taggtggggt aagagggtat 9360
tatgggtggg tagggtgaga ggatattatg ggtgggtggg gtgagagggt attatgggtg 9420
ggtggggtga gagggtatta tgggtaggtg gggtgagagg gtattatggg tgggtgaggt 9480
gagagggtat tgagggtggg tggggtgaga gggtattatg gttaggtggg gtgagagggt 9540
attatgagag gaggtaatag gggttattgt ggataggtgg ggtgagaggg tattttggga 9600
ggaggtagta ggggtagggg ttaggttggg gtaggtgggg gtagttgttt aggtgttagt 9660
tggggaggta gtgatggtag gatgagtaaa tggtattttt tatagttatg agggggtttg 9720
ggggttgtat tttttttttt gttatttttt taggaattat ttttgtttag gggtaaggaa 9780
gttttagagg ggtttgattt tggattttta tttttttttt tttttattat ggaggtgtta 9840
gggaggatag ggtagttgag tgttttggtt gggtttttat ttttggtatt tttttaggaa 9900
ggtttttttt ttatagttgt gtggttttat tatagaaggg gaaattgagg tttagggatt 9960
ttttagtttt tagtttgggt gtggagagtt tggagtttgt attgaggttt tagtttggtt 10020
atgggaattt tagggtatta ttggtttttt ttttatttag gtggttattt atttaaattt 10080
tgagtatttt tatggttttg tgtattggtt ttgtttaggt ttttgttttt gaaagtttta 10140

gagaggatag tggttttgaa tgttgttttt atttttttttg tttttttagg ttagtatatt 10200
aggtagatag tagttttttt attaggggtt ttatttgttt tatttttaat ttttagagtt 10260
aaagggattt ttgtttttat tttatttttt tttttttgt tttttttatt tgttttaagt 10320
tgtggtttta ggtgtatttt tgttaggtta tgttttatta agatgaagtt gtttgggtgg 10380
tgtttagtgg tttttgtgtt atggttttgt gttagtggat gaaggaagta gatttatgtt 10440
gttgagttag gttggaggga atttttaatg ggagaatttt ttggaaagat gtagtttttt 10500

```
ttttttaatg tttttttttt ttgtgttttt ttgtagagtt aattgtattt ttttttatta   10560
agtttttagt tttttttttt ttatttaatg gagtaggagg ggttgttgtt ttggggataa   10620
gattggggga gtgggatatt tatttttaag gttaaagaat ttagttatta agggttagga   10680
tattttaatt aatattttaa aaagtaaagt tatgtagagt ttatgttgat taaaatagta   10740
agttttttttg tgtttgtttt tttgagatag ggttttattt tgttgtttag gttagagggt   10800
agtggtataa ttttaggtta ttgtaatttt tattttttag tttttttttt tttgagatgg   10860
agttttattt tgttgtttag gttggagtgt aatggtgtga ttttagttta ttgtaatttt   10920
tgttttttag gtttaagtga tttttttgtt ttagtttttt gagtagttgg gattatatgt   10980
gtttattatt atattaggtt aatttttta tttttagtag agatggggtt ttgttatgtt   11040
ggttaggttg gttttgaagt tttgatttta ggtgatttgt ttgttttggt tttttaaagt   11100
ataaggttta taggtatgag ttattgtatt tggttattat ttttgtattt ttagtagaga   11160
tgaggttttg ttatgttgtt tagggtggtt tggtttttat tttttgggtt taggtgattt   11220
atttatttta gttttttgaa gtattggaat tataggtatg agttattgtg tttagtttaa   11280
aatagtaagt ttttaaaggg agggtagttg tggttgttgg gattattggg tgggtgttag   11340
ggtaggatta ggatttaatt tagtggatat tttattgttt tgtttattat ggggttttgg   11400
tattgatttt ggttttttag gagatgtagg attatgtttg ttttgttgtt gagtgtttag   11460
ggttttatta ttatttattt ttgtttatag ttttagggta gtttagtttt tagaggtttt   11520
taggtattta tttgaagttt ttgtagggtt gggattgatg ttttattttt tttagggtta   11580
gttttatagg ggagtatttt tttttttttt tttttttttt tttttatttt tttttttttt   11640
tttattttgt tttgggggga agttggggtg ggattgttta gtttggggtt tttttttttta   11700
gttaggtttt gggtttttatt gtttagtgta ggaagtaaag ggtagggttt tgggaagtta   11760
tgtttttttta aaggttatta gtttgggtta aaggttttag ttgtaattaa tggttaggtt   11820
agggagtttt aaagatatta tagtttagta gatttttttt tttaaatttt tgtgggtatt   11880
tttaggttgg agtttagttt attttaaggt atttatgaat gttttatttt tttgtatggt   11940
tttttggagt tgtttttatt tttgtagttt atgttggtga ggggttttgg tatatttgaa   12000
tggggagttt ttgtttggtt ttggtttttg ttatttattt ttgttgagtg tatagtggat   12060
ttgggtattt tatgtatata tggttttgtg gttgttaaag tttagtatga tggtttggga   12120
taggtagata atggttagtg tgtggattaa gtttttgtag ggtaggttgg gttttagttt   12180
gtagatgttt tttagtgtta ggttgttgtg tttttgtagg tttttgatat gttttgattt   12240
gtttttgtag attagtatta gtaggtagtt tgtaaggagt gtggttgtta gggatgttag   12300
ttaatattgt ttgtttgtat ttttggtgtt tttggggagg ttgtgatagt gtagagattt   12360
gggttggggt tatgatttta agttagttat ttagttatgt taaggttttg atttttggta   12420
tataaaatgg ggtgattttt tttttttgta agatttatag gttgtaagag tggaaatgaa   12480
tttagaatat ttatgtttgt gtttagtttt tgatgttggt ttttatgatt gggttttgga   12540
tttttattgg tggatttgtt ttgttttagg ttttggagtt ttttgggtta tattgagggt   12600
tgtaggattt gtggttatgg gtttgggggt ttagtgtggg gtttgtttgg tggttgtggt   12660
tttttgtttt ggttttgtta gtttgttttt attttgttgt tttgttagtt atatttgtgt   12720
ttatttttttg ttgttttgag atttattgtt ttttttggtt tttttagttt ttaggttgtg   12780
gttgagtttt agttttaggg ggtaggggag gttgatgggt tttgggtttt atttttattt   12840
atttggttat tgtttagtat agtaggtttt agtttttttt ttggttgttg gggatttgtt   12900
tgtgttttgt tttttggatt ttttagggta atttgggttt ttgtttttat ttttattttt   12960
ttatttttta atgttattgt tattataggt aattggttgg tgttgtttat tttattaggt   13020
gtagtttaaa tttggtatag tatagttttt gtgtggtttt gggtaaatta tttggtttgg   13080
ggatattttt gtgaatggtg ggggtagtag gtttgtttga gagagtagag aggatggtgt   13140
aggaagattt ttggtttttt tggtatggtt ttaggtttga tgattaggtt tttttttatt   13200
taggttttgg tagttgggtt ttaggatatt tggttttggt gagggtgtag gttttgggat   13260
tttatataaa tattttaaat ttttgtgagt atgtaggagg ggattgggga gaatgttttt   13320
gaagaggatt gtagaatgat gtgttatttg aagttttgaa aagtaggtgt ttatttttta   13380
tataggtttt gtaaagttat gtgagaatta aaattttaaa tgaatttgat gtagatattt   13440
atttttgtaa tggagtttag agtagatatg gtgtttagtt tttttttttta gttgtgttta   13500
ttttttttggg ttatatggtt atagtttgta gagttagggg gtttgtgtga ttatagggggt   13560
tagtggttag taagtttttt ttgtaagggg ttagagggta agtatgtagt ttttagggtt   13620
atataggttt tgtggttatt atttagtatt gttatttttg ttgtgtgaaa gtagttttag   13680
atttgtgta gatttttggg tgtagttgta tgttaggaag tttgatttat aaaagtaagg   13740
tgtggtgtgg gtgtggtgat agtgtgttta ttttttattg gaggatttgt ttgtagtgtt   13800
tgaaatgttt atagttagaa aaaagttagg tatttttaat ttgagaaaga agttttttta   13860
tttttgtatt tatagaattt tgtttgttta gttgaggttt tttagataat tttttggttt   13920
tttttggaaa ttttaaggta ggaagtagga tgtttggttt taagagagaa attaaatata   13980
gtggtgtaat tggaatttgt tgtaatagta atgtttttta aatgtgttat tagagtattt   14040
aagaaatgat ggtgtgtggg aatagtatga atatgggtgg ttttgtgtag gatgggggtt   14100
ttagggttgt ttgagttgga ggatagtttg tagtttttat tgtttgtagt tgaagtgtgt   14160
```

```
tgtgagattg ttgtaggggt ttgggttatt tttgttttgg gggattttaa aaagttgagt   14220
tatatttatg ttgaaatttt tattgaggat aattatgtat tatatagatt attttttat    14280
tttttttttt taaggggtaa ggtttttggg aatttttgtt ttgatttttt agttgtatgg   14340
ttttgggatt ttggttttag ttttttattt tgtgatttgg ggatagtggt atttttattt   14400
gtagtaatgt tgtaaggatt atttgtagga aggtgtggag gttttgtgtg tagtaagtat   14460
ttaatatatg tttgtatatt taggtatata tttgtttgtg ttattagtta taaggttaga   14520
gtgttttttt tgtaatttgg ttgtttatgt atagagggat taggtgattt atggtttatt   14580
tttagtgttt agtaaggggt atatattggt taattattta ttattttta ttttgagggt    14640
tttagttttt ttggagtttt ggtgggttga ttatttagaa gttaggtagg gattttttt    14700
tttttgtggt tttgttttgt ggtggtggta ttttggttat attttggtag atagttttgg   14760
tatttgataa taattagttt tttttattgt ttttaattag tatttttttt gtttaataag   14820
ttatattttt aataatatag ttaggattgg ggttaggagt ttgtttgtaa agagtttgat   14880
gttatttttt tggtttgatt gagtttaaag ttttttttat tatatttagt tggaaagtag   14940
ttatagatga tatgtgtggg aatggttgtg agttaataaa attttattta taaaaatagg   15000
ttttggtagg atttggttta gagattatag tgagttgatt tttggttaag agttgaattt   15060
gttatgatgg ttttttatat tttttataag ttagttggtt ttattttaga tttttatttt   15120
tgtaaggttg gtggtggggt gtatgttgaa tttatgtttg atttttagat tttttttga    15180
tttatggatg ttatttttg gtttgtttga tttttttgga ttttgtttat aaatgtagat    15240
ttatattttg tattttggga atgtttgaaa atttaggagt atttataggt aggggttgag   15300
tgtggttttt agtaagatgt gtttattttt gattttggaa tttgtatgtt tttatttggg   15360
aatagggttt ttgtatatgt aattgagttt aggattttgg attgtttgaa tgtagtagta   15420
agtgtttttg taagggggag aataggaggt gtagatatag ggaaggttat ttgaatgggg   15480
atggaggtag ggattggagt gttgaggtta tagttaaggg atatttgtgt ttaataggag   15540
ttgggagagg taggaaggag tttttttggg aagtttggag ggagtgtggt tttatgggtt   15600
ttttgattgt agagttttaa tttttaggat ttagtgggtg aatttttatt gtttgaaggt   15660
ttagttgtgg tgtttttttt tgttggttat aggaaattga tgattattta aattattgat   15720
tttattttta aatatgaata gaagttgaga ggttagagtt gggggtttgt ttgtttttt    15780
tgtttggtag aagttttttt ggtagttgat gtttattttt ttatgtttag gttgttttt    15840
ttttattatt aataataaat tgattttgtt tttaaagtgt attttttggg gagtgttagg   15900
gggtaaagtg ttgtaggaat tggtattaga gagatggaag ttttaggttt agggttgaga   15960
ttaagttagg ttaaggttgg gatattagag ttttaggatg tagtggtttt ttttgggata   16020
tagtatgtgt gttgggtaga tataggtttg ggggatttag gagtggatgg ttattggtta   16080
gtttttttggg tgttaggttt agagggtttt agttatattt ttttaggagg tttgggtatt  16140
tgggagtttt ttatttagtg gttgaggaag tattattggt tttgtttatt tattttttgg   16200
ttgggggttt tttgtttggt ggttgaggaa ttattattag ttttgtttat ttattttttg   16260
gttgtgggta ttggttgtaa tttggttagg gaaggttttt gtagaatttg gagggttttt   16320
ggggttttgg taggtagtat tgggttgggt ggagggtatt gggttgtgat gttgaaagga   16380
gataaatgag gaagaggtat taggagaggt taaggtatag agatt                   16425
```

<210> 315
<211> 2031
<212> DNA
<213> Artificial Sequence

<220>
<223> chemically treated genomic DNA (Homo sapiens)

<400> 315

```
ttttattgga tgttattgta tatgtgtttt gtttgtagtt gatgatgtag ttatagggta      60
agtgggttta gtgtttggat aagataaata ttggggttat agtggtggtt agtagggtaa     120
gtagaaagtt gaatgttttt aagggaaggt tttgaaattt tatgatgttt tggattatta     180
tgtgtatttg taagggtaag agaattttag atttaattta aaattatatt ttgtattttt     240
taagtttttt ttttatgtat gttgttttag tttttttttt agttttttt tttgatggat      300
agtggggatt tttgttatgg agttggtttt ttgtttttgg tgatgggttt ttgggaatgg     360
tggggtatat ttttagggtt gggaggggtt ttagtttttg agtgttggat ttgtgtttgt     420
tgtttattta agattttatt agaaaggtat agtttataat gtgggggatg ttagttagga     480
aataggtgtt tgtgaaattt gtatttgatt ttttgaatgg tttttgttgt ttttttggat     540
ttttattttt gtttatgtgt gtttgttata gtggggtgtt attttgttat tttttaattg     600
gttttttttt ggaaggagga ataaaagttt gttttgtttt ttgttttttt tttttataat     660
tgattttttt ttttttttat tttgttttgt tggagatggg tgttgttttt tttaggtggg     720
```

```
aatggaatgg tgagaaaggg atgatagaag gttgtgggga aaaaattttt taattttttt      780
tttttttttt tgaatatttt ttgattttgt tgtgtgagat aatattttttg gttattattg     840
atatggaaaa ttaagttgtt tgtgatggaa agtgattttt atgtttttga tttttgaaag      900
gagaagtatt tagtagtaaa ggttttgtag ttaattagtt ttgttttgaa ttgttttttt      960

ttaagtgtat gtttaaaatt tagtgagata tatttggaag tggattttat tgtgtgtttt     1020
ttggtgattt tttaaattgg tgatgttggt tttaatattt atttatattg tttggtatat     1080
atatttgtgt tttgtgagta attgtatttt tattattatg ggtagttaaa ggatgatttt     1140
tgtaagtgtt aggattaaag tgaagtgttt ttgtgttatg tttatggtga agtttttggt     1200
gttatagaga gtttttttggt ttttatgttt gtaggtttta gttttagtgg tagtgggtgt    1260
atttggtttg aatatggtgt tggagtttgg agagtatttt ttagagtgtt gtagatttgg     1320
gtttggagta tttttttgggg atagggaaat tatttttttt gtagtaggag gggttttagg    1380
aattgaagtt ttatgggaaa ttttttggta gttggagtgg agttttggtg gttttttttga    1440
atatagtaat tggtgagtga gtgggattga gaggtttatg aggagtttga aggttaaagt     1500
gtatatgata gagaggaata gtatgtagga gttggagtag tttattaggt agttttaggg     1560
tgtgtgtatg aaggtgtttt agttgtatag tgggagtgtt gagagtagta gattggttgt     1620
ttatatggtt agtattatgt tgagtatttg gtttgatttt ttggaggttg tttggttttt     1680
tatatttttg tgtattgtat aaaagtttgta agagattagg agagttgttt ttaagttgtt    1740
agagaggttt tggtataaat atattaaggt agaggtggtg tatagaaatt ggaagtaatt     1800
ggggattttg tttggttatt gtaaaaatat gaagatggtt attgatagga tgtttatgag     1860
attatttata gattaggaag ttataagtat ggatataata gttttgtttt gtatttttag     1920
tagggaaatt agtgaataaa tgttgtttat taaggttgta aaagttatga gatatgttaa     1980
gtaaaaaaga tagatattta gggtttttgg tggatttaaa aggtttgtag a              2031
```

<210> 316
<211> 2031
<212> DNA
<213> Artificial Sequence

<220>
<223> chemically treated genomic DNA (Homo sapiens)

<400> 316

```
tttatggatt ttttaaattt gttaggaatt ttgaatattt atttttttttg tttgatatgt      60
tttatgattt ttgtagtttt ggtgggtagt atttatttat taattttttt gttgaaaatg     120
tagaatagaa ttgttgtgtt tatgtttgtg gttttttggt ttgtggatga ttttatgagt     180
gttttgttgg tgattatttt tatgtttttg tagtggttaa atgaggtttt tggttatttt     240
taatttttgt gtattatttt tgttttaatg tatttatgtt agggtttttt tagtaatttg     300
aaggtgattt ttttagtttt ttataatttt tatatgatgt atagaggtgt ggggagttag     360
atagttttta gaagattggg ttaggtgttt ggtgtggtgt tgattgtgtg ggtagttagt     420
ttgttgtttt tggtgttttt gttgtgtggt tggggtgttt ttgtgtgtat gttttggggt     480
tgtttggtgg attgttttag tttttatgta ttattttttt ttattgtgta tgttttggtt     540
tttggatttt ttgtgggttt tttagtttta tttatttatt gattgttgtg tttggaggag     600
ttgttgagat tttattttaa ttattaggaa atttttttgtg gagttttaat ttttgggatt    660
ttttttattg tggggagagt ggtttttttg tttttagagg atgttttagg tttgagtttg     720
tggtgttttg gggatgttt tttgagtttt gatattgtgt ttggattggg tgtgtttgtt      780
gttgttgggg ttgaagtttg taggtgtgag aattggggga ttttttatgg tattaggagt     840
tttattgtga gtgtagtgta gaagtgtttt gttttgattt tagtgtttat aaaagttgtt     900
ttttggttgt ttatgatggt aaaagtgtag ttgtttataa agtgtgagtg tgtgtgttag     960
atagtgtaaa tgagtgttgg gattggtgtt gttggtttgg ggagttatta agagatatat    1020
agtgaagttt gtttttaagt gtatttttgtt gggttttaga tatgtattta gaagaaggta    1080
gtttgggata gggttagttg gttgtgaaat ttttattatt aaatgttttt ttttttaaaa     1140
gttaggggta tggggattgt tttttgttgt gagtgattta gttttttata ttggtggtgg    1200
ttaaggatat tgttttatat agtaaaatta gagagtattt agaaaaggaa agagggaatt    1260
aaaaggtttt ttttttataa ttttttgtta tttttttttt gttgttttat ttttgtttag    1320
agaggatgat gtttattttt agtagggtgg gatagagggg aggagagatt agttgtggag    1380
ggagaagata gggaataaaa taggtttttg tttttttttt taggaggaag ttgattaaga    1440
ggtggtagag tggtgtttta ttgtgatgag tgtgtatggg tggaggtgag ggtttaggga    1500
ggtgataaag gttatttgga gaattagatg tgggttttat ggatgtttgt tttttagtta    1560
gtgtttttta tgttgtagat tgtgtttttt tggtggagtt ttgaataggt agtgggtgta    1620
```

gatttggtat ttaaaagttg aaattttttt taattttggg ggtgtgtttt gttgttttta 1680
ggagtttatt attaaggata gggagttagt tttgtagtag aggtttttgt tgtttattag 1740
gagaaggaat tggaaaggga attgaggtag tgtgtgtgga gaaaaggttt gagaagtata 1800
aagtataatt ttaggttagg tttgaagttt ttttgttttt gtagatgtat atggtggttt 1860
agaatgttgt ggggttttag agtttttttt tggagatatt tagtttttta tttattttgt 1920
tggttattat tgtaatttta gtgtttgttt tgtttaaatg ttggatttat ttgttttgtg 1980
gttgtattat taattgtagg tagaatgtat atgtagtggt gtttgatggg a 2031

<210> 317
<211> 2237
<212> DNA
<213> Artificial Sequence

<220>
<223> chemically treated genomic DNA (Homo sapiens)

<400> 317

gtttgtgata gatgtttgta ggtatatggt aaggaaaata gattaaaata ttaagaaata 60
gtaagaggtt ggattgaggg tttttttattt gtatgggaaa ttttattttg tttattattt 120
ttagaaataa aaaggaaaat gattttaaag atataggtta aaggttgaag gggtgtagaa 180
ttattttttg ttttttttt tttatttttt ttaaatattt tagaatgagt taaatttttt 240
gaattttgta gtatagataa taagtaattt agtaagattg gatggggatg attgagggtt 300
aatttattat aggattatag gaaatttttt agttttaaat ttgaatgtat ttgtgattat 360
ggtaatattt tttatttgat taaaagttat taaatgtttt gataagtgtt tatagttgtt 420
tttttgatta ttgaagtggt tttgaaaaga gtttttgggg ttaggtggtt gatttattgt 480
gtttttttgta tatttttatt ttgagttggt gtatttggtg attgttttgg tgtttttgga 540
tatggtgtgt ttggttagtt tgttgggtag tagtaggtgt atggttgttt ggattttgtg 600
ggatgtgatg gtggagtgtt tgttgtagtg tgttaggtgg gatgtttttt ttgtgatgtg 660
tttgaagatg ttgttgatga aggagtttat gatgtttatg gttttggatg agatgttggt 720
gttggggtgg atttgtttta gtattttgta tatgtaaatg gagtagtttt ttttgtggtt 780
gtgtttgtgt tttttgttgt tttttttttg tatttttgta atagtttttt tggagttttt 840
tttgggagta ggagtggatt ttgttggatt tggtattttt gtgtgaaaaa agagaaaaga 900
gatttaaaga agtaatttga attattgtaa aatgggttgg ttatgtgtta tttatagagt 960
ttgtatgtaa atgaagattt gtaaagtgtt gtgtttttat tggttaattt ataatggtgt 1020
tgttagaggg ggtggagttt atgtaaataa tagattttag ttttgttttt ttaatggtta 1080
ttgtaataaa agttttgtta ttttatttaga atggtttatt ttatattaat tagttttaag 1140
ttaggtgaag tgatagtttt tatggtttgg ggtttttttt tggtttttat tttttattag 1200
atttgtatat ggaagaaatt ttaatgttag ttagataagt taattttttt gttttgtttt 1260
tgtattttta ttttatattt tgtggaaatt tttttttttt ttttgaaata ttattttatt 1320
tatttttaaa aattgttaga tatttatttt tgtttgttta aattattatt ttggtttgga 1380
tattgtttgt agtatgtaat gaggaagatg ttgagtttta attaagttta tagtttttaa 1440
ataaattagg aaatgggttt ataattttta agggatttgt tttattttta tatttaggat 1500
tttttatatt tagaaatttt ttatagtttt ttgtttttat gtaaaatttt atttatagaa 1560
ttgggagttg gatttttagt tttaaattag tgtgaaaatg tttggttgga attataagga 1620
ttatgttttt gtggtttttt ttattgttgg ttaatttatg gttgggtttt gggttttgta 1680
atgttattta ttatgagtgt ttttggtttt tgattgttgt gtaaggtaga tgtatattag 1740
atggagagtt ggtatagttg tttttgatga aaatggtagt ggttttgaaa agagttttta 1800
gattgattat ttaaaaatat gttttaggtt tgttttttgt ggatgtggtg ggttaattgg 1860
atgtttttgg gtatgatggt tatgtgtttg gtatggatgg tgtataggtt tgtgtttttg 1920
aatagtttta ttaggtaggt tttgttggtt ttttgtagtg ttattatggt tgagttttgg 1980
aagtgtaggt ttgttttaaa gttttgtgtg attttgtgta ttagttgttg gaagggtagt 2040
ttgtggatta gtagttttgt agatttttgg tagtgttgga ttttttgtag agttatggtg 2100
ttgggttggt agtggtgtgg tttttttatt ttgtttgtgg ttggtgtgtt tttgtgggtt 2160
gttttggtag ttagttgttt ttttggggtt ttgttgttgg ttgatttgtg ggtagtttgt 2220
ttagtatggg ttatgtt 2237

<210> 318
<211> 2237
<212> DNA
<213> Artificial Sequence

<220>
<223> chemically treated genomic DNA (Homo sapiens)

<400> 318

```
agtatggttt gtattaagta gattgtttgt aagttgattg gtggtaaggt tttgaggaag      60
tagttggtta ttaaagtggt ttgtaagagt gtgttggtta tgggtggggt gaagaagttg     120
tattgttatt ggtttggtat tgtggttttg tgggagattt ggtgttatta gaagtttatg     180
gagttgttga tttgtaagtt gtttttttag tggttggtat gtgagattgt gtaggatttt     240
aagatggatt tgtgttttta gagtttggtt gtgatggtgt tgtaggaggt tagtgaggtt     300
tatttggtgg ggttgtttga agatatgaat ttgtgtgtta tttatgttaa gtgtgtgatt     360
attatgttta aggatattta gttggtttgt tgtatttgtg gggagtgggt ttaaggtata     420
tttttaagtg gttgatttaa aggttttttt tagagttatt gttgttttta ttaagagtag     480
ttgtattggt tttttatttg atgtgtgttt gttttgtgta gtggttaggg gttaggggta     540
tttgtggtgg gtgatgttat agaatttaaa gtttagttgt gagttggttg gtagtagaga     600
aagttgtaga ggtatggttt ttgtggtttt ggttgagtgt ttttatgttg gtttaaggtt     660
agaggtttgg tttttaattt tgtgggtaaa gttttgtatg ggggtaagag attatgggga     720
gtttttagat gtggaagatt ttgagtgtaa gggtggggtg agttttttaa aaattataaa     780
tttgtttttt ggtttattta ggaattataa gtttaattaa agtttagtat ttttttttatt     840
gtatattata ggtggtgttt aagttagaat agtaatttaa gtgggtggga gtaaatattt     900
ggtgattttt aaaaatgggt gaggtagtat tttaaggaag agaaaaaaat ttttataaag     960
tatagaataa aaatgtaaaa atgaggtaag agagttaatt tgtttagttg gtgttaagat    1020

ttttttgta tgtaagttta atggaaagtg aaaattaaga aaaaatttta agttgtagaa    1080
attattgttt tatttagttt aaaattggtt agtgtgaaat ggattatttt gataggataa    1140
taagattttt gttatagtga ttattaagga agtaagatta gaatttatta tttatataga    1200
ttttattttt tttgatgata ttgttgtaag ttaattaatg aaagtgtagt attttgtgag    1260
tttttatttg tatatgggtt ttataagtag tgtataatta gtttgttttg tggtagtttg    1320
gattattttt ttaagttttt tttttttttt tttgtgtaaa aatgttggat ttagtgaaat    1380
ttgtttttgt ttttaagaag ggttttaaaa aggttgttat gaaagtgtag aagaaggatg    1440
gtaagaagtg taagtgtagt tgtaaggaga gttattttgt ttatgtgtat aaggtgttga    1500
agtaggttta ttttgatatt ggtattttgt ttaaggttat gggtattatg aatttttttg    1560
ttaatgatat ttttgagtgt attgtgggag aggtgttttg tttggtgtat tataataagt    1620
gttttattat tatattttgt gagatttaga tggttgtgtg tttgttgttg tttggtgagt    1680
tggttaagta tgttgtgttt gagggtatta aggtggttat taagtatatt agtttgaagt    1740
aagagtgtgt aagggatgta atagattaat tatttaattt taaaggtttt ttttagagtt    1800
attttagtaa ttgagaaagt agttgtaaat atttgttaga gtgtttgata gtttttggtt    1860
aggtagggag tgttattatg gttatggatg tatttgggtt taggattaag gagtttttttg   1920
tagttttgta atgagttggt ttttagttat ttttgtttag ttttattgag ttgtttgtta    1980
tttgtgttat gagatttgaa aggtttgatt tgttttggga tatttgggag ggatggaggg    2040
aaggaggtgg ggggtggttt tatatttttt tggtttttaa tttgtatttt taaagttgtt    2100
tttttttttg tttttgaaag taatgaatga gatgagattt tttatataga tgagaaattt    2160
ttggtttgat tttttattat tttttagtgt tttaatttgt ttttttttatt atatatttat    2220
aggtgtttgt tataaat                                                   2237
```

<210> 319
<211> 4182
<212> DNA
<213> Artificial Sequence

<220>
<223> chemically treated genomic DNA (Homo sapiens)

<400> 319

```
agatttttttg aatgtttgat gaaatggatt tttgtgaaaa ttaatagtga atatttattg     60
ttgtattgta tgaagttttt gaaatgtaat taaaagtttt tattgagttt ttgagttttg    120
gtttgtgtgt atttttttgg ttgtggatat tttattgtgt agagttttgt tttttttgttg   180
tttttagttt ttgatgattt ttttgttttt gttttgtttg gtgatagatg ttttattgtt    240
tttaattgga ggtatttttt gtgggagtgg ttaattggga gttttggtag gtggggaggt    300
```

```
tgggttagtt agatttggag gtttaatttt aatatggttg aagtaagtag tgttaattta     360
ggtagtggtt gtgaggaaaa aaggtatgag gggttgtttt tggaatttgt gttatttggt     420
attattattt tgagggtgaa gtttttttgat attatggtgg atattttttt ttagaagttg     480
gttgttgttg gtaggtaaag tggatgtagt tgtggtggga gtgtttgttg gtattgaagt     540
ttaaattttg tgaggttagg atggttgtag gttggtgtgt ggtgatgtgg gtttgtgttg     600
ggggtggggt agttggatga ggtgatttag ttaaattttg agttttagga gttagggtat     660
ttatgtattg ataatttgta gtggattggg atagttagtt attttttttt ataggaagtt     720
ttgtttttat taaaatttta ggtggttggg aggaaagata gagtttttgt aaattagagt     780
agggtttttt atttttttat tatttttgag atagggtttt gtgttgttgt ttaggttgga     840
gtgtagtggt gtgattattg tagatttgat tttttgggtt taagtgattt ttttgttgta     900
gtttttttaag tagttgggat tataggtttg tgtttttgtg tttggttata gattagggtt     960
taagttgtgg tttattatta tgtgagtaag atgtaagttt tttttttttt gttggttttt    1020
tgttagttaa gtgatagtaa tagtattttt tttatagggt tgttttgaga attaaatgag    1080
taaatatatt taaggtgttt aggataggat ttgatatata gaaagatttg ttagttatta    1140
tgagaatatt tttttattg ggagattgtt aggattaaat gtgttattat gaaaagaaat    1200
attttttttg ttttgatttt tattttattt atttttattt ttattttttg agatggagtt    1260
ttgttttgtt gtttaggttg gagtagtggt gtgattttgg tttattgtaa tttttgtttt    1320
ttgggtttaa aggatttttt tgttttagtt ttttaagtag ttgggattat aggtgtatat    1380
tattatattt ggttaatttt ttgtatttgt attagtagag atgaggtttt attatgttgg    1440
ttaggttggt ttggaatttt tgattttgtg atttatttat tttggttttt taaagtatta    1500
ggattatagg tgtgagttat tgtgtttggt ttttattttt atataattta tatgatttga    1560
gagttttgtt tatgatttta ttattttagt atattagtgt tgaaatgttt ttaggagttt    1620
ggtgttttga ttatagtttt ttttttttta gtttttttta tttttatttt tttgtttttt    1680
ggttttatta ggatttttat ttgtttttag tttatttttt ttgggtttag tattttattt    1740
tttttatgta taattaatga tttgttttta tttatgattt attaaatatt tgagtgttag    1800
ttggggatat tatgaggtag ataaggtttt ttgttttttt ggagtttgta tttaatgtgt    1860
agagatgtta ataatattat tatataggtt ggtaagatag taaggttatt attatgtgtt    1920
aatagtgatt tatgtttgta atttttgtgt tttgggaggt tgaggtagga ggattatttg    1980
agtttggaag tttgaggttt tggtgagtta taatagtttt attgtatttt agtttaggtg    2040
atagagtaag attttgtttt taaaaaagaa aaaaaaaaaa gataatgaat aggtaatgat    2100
attattagat aggatgtttg gggtgtttat tttgagatgg tattttagtt ggaatttgaa    2160
ggatgagaag gaattagatt attattatta ttattattat tatttttttt ttttaggtag    2220
tgtttagttt tgttgtttag gttggagtgt agtggtttga ttttggttta ttgtagtttt    2280
tatttgttgg gtttaagtga tttttttttt ttagtttttt gagtagttga gattataggt    2340
gtttgttatt atggttagtt aatttttgta tttttagta gagatagggt tttattatgt    2400
tggttaggtt ggttttgaat tgagtttaag tgatttattt atttttgttt tttaaagtgt    2460
tgggattata ggtatgagtt attgtattta ggtggaatta tttttttgaa gaattataga    2520
atgaatgtat aggaaaaggg gatagtttgt gtaaagtttt tgaggtgatt agatatttta    2580
ggaattggta gagatttttt tggttatagt atagtgatta gagtaaaatg agatgaggtt    2640
ggagaggtag gtaatagttt tattatgtag ggtttagttt gttataataa tgaatttgat    2700
aaaataattt gtaattattt ttatgaatta attatggtat gttattatta tatgttttgg    2760
tatggttttt tttttgttgt atttattttt ttttatttat ttttgttttt gttttttttt    2820
ttatagtttt tatttttttt tagttatatg ttttttttatt tatttattta tttatttatt    2880
tttatttttt ttagagatga ggttttgttg tgttgtttaa gttgatttta agttttgagg    2940
tttaagtgat tttttttattt tagtttttta gagtgttagg attataggta tgagttatag    3000
tgtttgtttt atttatttat ttatgagata gggttttgtt ttgttgttta ggttggagtg    3060
taatggtata atttaggttt attgtaattt ttgtttttttg ggtttaagtg atttttttgt    3120
tttagttttt tgagtggttg ggattatagg tatgtattat tatgtttagt taatttttgt    3180
atttttagta gagatggggt tttgttatgt tggttaggtt ggtgttgaat ttttgatttt    3240
aggtgatttg tttgttttgg ttatttattt ataatataaa tatttattga ttattgatag    3300
tgttaataaa tttgttttag ttttagtagg gttttagttg ttggagataa ggtagtgatt    3360
aagatggata aggttttaat tttttttggag tagatatttt ggtattatta aaaaaaaata    3420
aaattgagtt tggtgtagtg gtttatattt gtaattttag tattttgaga ggtagaggtg    3480
ggtggattat ttgaggttag aagtttgaga ttagtttggt taatgtagtg aaattttatt    3540
tttattataa atataaaaat tagttgggtt tagtggtgta tgtttgtaat tttagttatt    3600
tgggaggttg aagtaggaga attgtttgaa tttgggaagt ggaggtttta gtgagttaag    3660
attttattat tgtattttag tttgggtaat agagtgagat tttattttaa aaaaataaat    3720
aaaataaaat aaaattagtg taaaggaata gagagtagtt gatatgttgt tttttggtga    3780
tttgttgttg gagaggttgg gattttggat gtgtgtgggg ttttggttta ttggtgattt    3840
ggttagttgg ttgtgttttt gtttgagttg tttgttgggg tttgtgggtt tgttgatttt    3900
ttgtgtgttt gagtgttttg atttttggtg ttggtttggg tttgggtttt tgatttattt    3960
```

gggggtggtg gggaaggtgg tgagggttat tttgttttg tgtgtttttt gttgtgggtg 4020
tttggggtgg ttgtgataat tttattttat tggttttgtg ttgtgtgtgt taggtgtttt 4080
tgtttttgtt gggttgtttg ttgttttttt tttggagtgg ggagttggtt agggttgatt 4140
gatttgttgg ttggttggtt tgtttttgtt tttggggggt tt 4182

<210> 320
<211> 4182
<212> DNA
<213> Artificial Sequence

<220>
<223> chemically treated genomic DNA (Homo sapiens)

<400> 320

gagtttttg ggagtggagg tgggttggtt ggttagtgag ttgattggtt ttggttaatt 60
ttttattttg gggaaggggt ggtggataat ttagtggaga tgagaatgtt tgatatgtat 120
ggtatggagt tagtggggtg gggttgttgt ggttgttttg ggtgtttgta gtggagagtg 180
tatgggggta gggtagtttt tgttgttttt tttgttgttt ttgggtgggt tagagatttg 240
gatttgggtt ggtattggga gttgggatgt ttggatgtgt gagagattag taggtttgtg 300
ggttttagta ggtggtttaa gtaggagtat ggttggttag ttgggttatt ggtaggttag 360
agttttgtat gtatttagag ttttaatttt tttagtgata ggttgttaga ggatagtgtg 420
ttagttgttt tttatttttt tatattgatt ttattttatt ttatttattt ttttgagata 480
gagttttatt ttgttgttta ggttagaatg taatggtggg attttggttt attgaaattt 540
ttgtttttta ggtttaagtg attttttttgt tttagttttt tgagtagttg ggattatagg 600
tatgtgttat taggtttggt taatttttat atttgtagta gagatggggt tttgttatat 660
tggttaggtt ggttttgaat ttttgatttt aagtgattta tttgtttttg tttttttaaag 720
tgttgggatt ataggtgtga gttattgtgt taggtttaat ttttattttt tttaatggta 780
ttagaatgtt tattttaaga agattggaat tttgtttatt ttgattattg ttttattttt 840
agtaattaga attttattag aattagaata aatttattga tattgttaat ggttagtaaa 900
tatttgtgtt ataaataagt ggttgaggta ggtggattat ttgaggttag gagtttaata 960
ttagtttggt taatatggta aaattttatt tttattaaaa atatgaaaat tagttgggta 1020
tggtggtgta tgtttgtaat tttagttatt tgggaggttg agataggaga attgtttgaa 1080
tttaagaggt aggggttgta gtgagtttag attgtgttat tatattttaa tttgagtaat 1140
agagtaagat tttgttttat aaataaataa atgggatggg tattgtggtt tatgtttgta 1200
attttagtat tttgggaggt tgaggtggga ggattatttg agttttagag tttgagatta 1260
gtttgggtaa tatagtaaga ttttattttt aaaagaaata aaaataaata aataaataaa 1320
taaatggggg gatatataat taagagaaag tggggattat gggaggggag ataagggtaa 1380
gaatgagtag ggaaaaataa atataatagg ggagggattg tgttaggata tatgatgatg 1440
atgtgttatg attggtttat aaaagtgatt ataaattatt ttattaaatt tattattatg 1500
gtaaattagg ttttgtatga taaggttgtt atttgttttt ttagttttat tttattttat 1560
tttgattatt atgttgtagt taagaaggtt tttgttagtt tttggaatat ttgattattt 1620
tagggatttt gtataggttg tttttttttt ttatatattt gttttgtaat tttttaaaaa 1680
ggtggttttg tttgggtgta gtggtttatg tttgtaattt tagtattttg ggaggtagag 1740
gtgagtggat tgtttgagtt tagtttgaga ttagtttggt taatatggtg aaattttgtt 1800
tttattaaaa aatataaaaa ttagttggtt gtggtagtag gtatttgtaa ttttaattat 1860
ttgggaggtt gagggaggag aattatttga atttggtagg tggagattat agtgagttga 1920
gattgagtta ttgtatttta gtttgggtaa tagagttaga tattgtttaa aaaaaaaaaa 1980
taataataat aataataata ataatttggt tttttttttat tttttaagtt ttagttgaaa 2040
tgttatttta gaatgggtgt tttaagtatt ttatttagtg gtattattat ttgtttatta 2100
tttttttttt tttttttttt tagaggtagg gttttgtttt gttatttagg ttggagtgta 2160
atggggttgt tataatttat tgaagtttta aattttttagg tttaaatgat ttttttgttt 2220
tagtttttta aagtatgggg attataggta tgagttattg ttgatatata ataatggttt 2280
tattatttta ttagtttatg tgatgatgtt gttggtgttt ttatgtattg aatgtaagtt 2340
ttaaaaagat aagaggtttt gtttgtttta tggtattttt agttgatatt taaatatttg 2400
atgagttatg agtgaagata aattattaat tatgtgtagg aaaggtaaag tgttgaattt 2460
aggaggaatg gattgggaat agatgagagt tttgatgaag ttaaagaata gggaagtggg 2520
gatagagagg gttagagaag gagaggttgt ggttagggta ttgaattttt ggaagtgttt 2580
taatattgat gtgttggggt ggtgggatta tgggtaaaat ttttgagtta tataaattat 2640
ataaaagtga aggttgggtg tggtggttta tgtttgtaat tttggtattt tgggaggttg 2700
aggtgggtgg attatgaggt taggagtttt agattagttt ggttaatatg gtgaaatttt 2760

gtttttatta gtatagatat aaaaaattag ttgggtgtgg tagtgtgtat ttgtaatttt 2820
agttatttgg gaggttgagg taggggaatt ttttgaattt gggagatgga ggttgtaatg 2880
agttaagatt gtgttattgt tttagtttgg gtgataggat gagattttgt tttaaaaaat 2940
aaaaataaaa ataaataaaa taaaagttga gataggaaaa atgttttttt ttatggtaat 3000
atatttagtt ttggtaattt tttagtgaag aagatatttt tataatagtt aataaatttt 3060
tttatgtgtt aggttttgtt ttaggtgttt taggtgtatt tatttattta atttttaaaa 3120
tgattttatg agaaaggtat tattgttgtt atttgattga taagaagttg atgagggaga 3180
gggggtttat gttttgttta tatagtggtg ggttgtgatt taaattttga tttgtagttg 3240
ggtgtgggag tatgagtttg tagttttggt tatttgggaa gttgtggtag gaagattgtt 3300
tgagtttagg aggttgagtt tgtagtgatt atgttattgt attttagttt gggtgataat 3360
gtaaggtttt attttaaaga taataaaaaa ataaaaaatt ttgttttaat ttgtaaaggt 3420
tttatttttt tttttaatta tttgaaattt tagtgaaaat ggggtttttt gtaggaagga 3480
gtagttagtt attttggttt gttataggtt attagtgtgt gaatattttg atttttaagg 3540
tttaggattt gattgggttg ttttgtttga ttgttttgtt tttaatgtgg atttatgtta 3600
ttgtgtgtta gtttgtggtt gttttgattt tgtgggattt gagttttggt gttaataaat 3660
atttttattg tggttgtgtt tattttattt gttggtggtg attagttttt gaagaaaagt 3720
gtttattatg gtgttgagga gtttttatttt tgaaatggta gtgttgggtg gtatagattt 3780
tgaagatgat tttttatgtt tttttttttt atagttgttg tttagattgg tgttatttgt 3840
tttggttatg ttgaagttga atttttaaat ttaattggtt tggttttttt gtttgttgga 3900
gtttttgatt ggttgttttt gtgaagggtg tttttgattg gaagtagtag aatgtttgtt 3960
attgagtagg gtgggggtgg ggaagttatt ggaggttgag ggtagtgggg aggtgaggtt 4020
ttgtgtggtg ggatgtttgt gattggaaaa atatgtgtaa gttaaagttt gggggtttaa 4080
taaaaatttt taattatatt ttagagattt tgtatagtgt aatagtgaat atttattgtt 4140
aatttttata agagtttatt ttattaaatg tttagagagt tt 4182

<210> 321
<211> 20
<212> DNA
<213> Artificial Sequence

<220>
<223> Detection primer for CCND2

<400> 321

tttggaggga tagaatgtga 20

<210> 322
<211> 23
<212> DNA
<213> Artificial Sequence

<220>
<223> Detection primer for CCND2

<400> 322

aaaaacaacc ttaactcaaa cat 23

<210> 323
<211> 27
<212> DNA
<213> Artificial Sequence

<220>
<223> Detection primer for CDKN2A

<400> 323

agattattag tttttatttg agggatt 27

<210> 324
<211> 21
<212> DNA
<213> Artificial Sequence

<220>
<223> Detection primer for CDKN2A

<400> 324

ccaccctaac tctaaccatt c 21

<210> 325
<211> 20
<212> DNA
<213> Artificial Sequence

<220>
<223> Detection primer for CD44

<400> 325

tttttgtttg ggtgtgtttt 20

<210> 326
<211> 19
<212> DNA
<213> Artificial Sequence

<220>
<223> Detection primer for CD44

<400> 326

cacttaactc caatccccc 19

<210> 327
<211> 22
<212> DNA
<213> Artificial Sequence

<220>
<223> Detection primer for EDNRB1

<400> 327

gaagggatga atgaataaaa gt 22

<210> 328
<211> 22
<212> DNA
<213> Artificial Sequence

<220>

<223> Detection primer for EDNRB1

<400> 328

caaaaacttc tcaaatcaac aa 22

<210> 329
<211> 22
<212> DNA
<213> Artificial Sequence

<220>
<223> Detection primer for ELK1

<400> 329

atatgggatt gatggaagat ag 22

<210> 330
<211> 23
<212> DNA
<213> Artificial Sequence

<220>
<223> Detection primer for ELK1

<400> 330

tccaataaac acaaacctaa atc 23

<210> 331
<211> 24
<212> DNA
<213> Artificial Sequence

<220>
<223> Detection primer for FOS

<400> 331

tttttattta ggatgaggga tatt 24

<210> 332
<211> 18
<212> DNA
<213> Artificial Sequence

<220>
<223> Detection primer for FOS

<400> 332

actacttccc acccaacc 18

<210> 333
<211> 23

<212> DNA
<213> Artificial Sequence

<220>
<223> Detection primer for GSTP1

<400> 333

ttggttttat gttgggagtt ttg 23

<210> 334
<211> 24
<212> DNA
<213> Artificial Sequence

<220>
<223> Detection primer for GSTP1

<400> 334

ctactatcta tttactccct aaac 24

<210> 335
<211> 22
<212> DNA
<213> Artificial Sequence

<220>
<223> Detection primer for RARB

<400> 335

gggagttttt aagttttgtg ag 22

<210> 336
<211> 19
<212> DNA
<213> Artificial Sequence

<220>
<223> Detection primer for RARB

<400> 336

ttaatctttt tcccaaccc 19

<210> 337
<211> 22
<212> DNA
<213> Artificial Sequence

<220>
<223> Detection primer for PTGS2

<400> 337

agagggggta gtttttattt tt 22

<210> 338
<211> 24
<212> DNA
<213> Artificial Sequence

<220>
<223> Detection primer for PTGS2

<400> 338

aacatatcaa cctttcttaa cctt 24

<210> 339
<211> 22
<212> DNA
<213> Artificial Sequence

<220>
<223> Detection primer for RASSF1

<400> 339

agtgggtagg ttaagtgtgt tg 22

<210> 340
<211> 19
<212> DNA
<213> Artificial Sequence

<220>
<223> Detection primer for RASSF1

<400> 340

ccccaaaatc caaactaaa 19

<210> 341
<211> 23
<212> DNA
<213> Artificial Sequence

<220>
<223> Detection primer for ESR2

<400> 341

agttggagaa attgaaaaga tta 23

<210> 342
<211> 22
<212> DNA
<213> Artificial Sequence

<220>
<223> Detection primer for ESR2

<400> 342

taacaaaccc aaaacctctc ta 22

<210> 343
<211> 22
<212> DNA
<213> Artificial Sequence

<220>
<223> Detection primer for DRG1

<400> 343

tttagttgtg aaaaagggat tt 22

<210> 344
<211> 23
<212> DNA
<213> Artificial Sequence

<220>
<223> Detection primer for DRG1

<400> 344

ccctataacc tccacactat ctc 23

<210> 345
<211> 21
<212> DNA
<213> Artificial Sequence

<220>
<223> Detection primer for DRG1

<400> 345

gttttggagg gagtagagat t 21

<210> 346
<211> 18
<212> DNA
<213> Artificial Sequence

<220>
<223> Detection primer for DRG1

<400> 346

cccatcccac aattaaaa 18

<210> 347
<211> 23
<212> DNA

<213> Artificial Sequence

<220>
<223> Detection primer for CMYA3

<400> 347

tgttttaggt ttgatggatt aga 23

<210> 348
<211> 18
<212> DNA
<213> Artificial Sequence

<220>
<223> Detection primer for CMYA3

<400> 348

actccccaaa atcccact 18

<210> 349
<211> 23
<212> DNA
<213> Artificial Sequence

<220>
<223> Detection primer for ONECUT2

<400> 349

gaagaggtgt tgagaaatta aaa 23

<210> 350
<211> 21
<212> DNA
<213> Artificial Sequence

<220>
<223> Detection primer for ONECUT2

<400> 350

cccaccctaa cttaccataa a 21

<210> 351
<211> 19
<212> DNA
<213> Artificial Sequence

<220>
<223> Detection primer for MX1

<400> 351

tgtaggagag gttgggaag 19

<210> 352
<211> 22
<212> DNA
<213> Artificial Sequence

<220>
<223> Detection primer for MX1

<400> 352

ccaaacataa catccactaa aa 22

<210> 353
<211> 22
<212> DNA
<213> Artificial Sequence

<220>
<223> Detection primer for DOCK10

<400> 353

gtttttaagt gttgggtgat tt 22

<210> 354
<211> 21
<212> DNA
<213> Artificial Sequence

<220>
<223> Detection primer for DOCK10

<400> 354

tacctcttcc ctctaccaaa c 21

<210> 355
<211> 24
<212> DNA
<213> Artificial Sequence

<220>
<223> Detection primer for BTG4

<400> 355

aagagtttta ggaaatgtgt tttt 24

<210> 356
<211> 22
<212> DNA
<213> Artificial Sequence

<220>
<223> Detection primer for BTG4

<400> 356

ttctactcac caaaccctct ac 22

<210> 357
<211> 22
<212> DNA
<213> Artificial Sequence

<220>
<223> Detection primer for DMRTC2

<400> 357

taaggtaagg gaaggttaga aa 22

<210> 358
<211> 22
<212> DNA
<213> Artificial Sequence

<220>
<223> Detection primer for DMRTC2

<400> 358

attaccacaa cctccaataa aa 22

<210> 359
<211> 25
<212> DNA
<213> Artificial Sequence

<220>
<223> Detection primer for GPR7

<400> 359

ttattatttt agatggagtg aggtt 25

<210> 360
<211> 18
<212> DNA
<213> Artificial Sequence

<220>
<223> Detection primer for GPR7

<400> 360

acccaaatta ccccacaa 18

<210> 361
<211> 23
<212> DNA
<213> Artificial Sequence

<220>
<223> Detection primer for FAT

<400> 361

agatgtttta tatttgtttg gga 23

<210> 362
<211> 21
<212> DNA
<213> Artificial Sequence

<220>
<223> Detection primer for FAT

<400> 362

tactcacccc aatcttcact a 21

<210> 363
<211> 22
<212> DNA
<213> Artificial Sequence

<220>
<223> Detection primer for ISL1

<400> 363

taaaaatgga agggaagata ga 22

<210> 364
<211> 20
<212> DNA
<213> Artificial Sequence

<220>
<223> Detection primer for ISL1

<400> 364

atctcccaaa aacaatcaca 20

<210> 365
<211> 22
<212> DNA
<213> Artificial Sequence

<220>
<223> Detection primer for GPRK5

<400> 365

ttgtggttat tttgagatgg ta 22

<210> 366
<211> 18
<212> DNA
<213> Artificial Sequence

<220>
<223> Detection primer for GPRK5

<400> 366

ccctccccct aactaaaa 18

<210> 367
<211> 26
<212> DNA
<213> Artificial Sequence

<220>
<223> Detection primer for SLC35F2

<400> 367

tttatttatt aggtgaagag tttgtt 26

<210> 368
<211> 19
<212> DNA
<213> Artificial Sequence

<220>
<223> Detection primer for SLC35F2

<400> 368

tcctcctacc accctaaaa 19

<210> 369
<211> 21
<212> DNA
<213> Artificial Sequence

<220>
<223> Detection primer for C14orf59

<400> 369

ttggagagat gtgttggtta g 21

<210> 370
<211> 22
<212> DNA
<213> Artificial Sequence

<220>
<223> Detection primer for C14orf59

<400> 370

aaaactcctc ccctctataa at 22

<210> 371
<211> 24
<212> DNA
<213> Artificial Sequence

<220>

<223> Detection primer for SNRPN

<400> 371

tttttagaat aaaggatttt aggg 24

<210> 372
<211> 22
<212> DNA
<213> Artificial Sequence

<220>
<223> Detection primer for SNRPN

<400> 372

cccctctcat tacaacaata ct 22

<210> 373
<211> 24
<212> DNA
<213> Artificial Sequence

<220>
<223> Detection primer for ARHGEF18

<400> 373

ttttaggaat gtaggatata aggg 24

<210> 374
<211> 20
<212> DNA
<213> Artificial Sequence

<220>
<223> Detection primer for ARHGEF18

<400> 374

ccccacataa aaacctatcc 20

<210> 375
<211> 22
<212> DNA

<213> Artificial Sequence

<220>
<223> Detection primer for SNX8

<400> 375

tagggtttga tgatgtgatt tt 22

<210> 376
<211> 21
<212> DNA
<213> Artificial Sequence

<220>
<223> Detection primer for SNX8

<400> 376

tccctcctaa ctcaacacta a 21

<210> 377
<211> 19
<212> DNA
<213> Artificial Sequence

<220>
<223> Detection primer for FBN2

<400> 377

gagagggagg gttaaggtt 19

<210> 378
<211> 22
<212> DNA
<213> Artificial Sequence

<220>
<223> Detection primer for FBN2

<400> 378

actactacct cctttcccaa at 22

<210> 379
<211> 22
<212> DNA
<213> Artificial Sequence

<220>
<223> Detection primer for HOXB5

<400> 379

gtggaaaaag gagagtaaat tg 22

<210> 380
<211> 20
<212> DNA
<213> Artificial Sequence

<220>
<223> Detection primer for HOXB5

<400> 380

ctcctcaatt ctcaccaaaa 20

<210> 381
<211> 20
<212> DNA
<213> Artificial Sequence

<220>
<223> Detection primer for LIMK1

<400> 381

agggaggttt ggtgtatttt 20

<210> 382
<211> 22
<212> DNA
<213> Artificial Sequence

<220>
<223> Detection primer for LIMK1

<400> 382

aaaccctact tcctacaaac aa 22

<210> 383
<211> 22
<212> DNA
<213> Artificial Sequence

<220>
<223> Detection primer for PSD/Q9H469

<400> 383

aaggtattat ttttggggtt tt 22

<210> 384
<211> 22
<212> DNA
<213> Artificial Sequence

<220>
<223> Detection primer for PSD/Q9H469

<400> 384

aactatccaa cctcttccac tt 22

<210> 385
<211> 18
<212> DNA
<213> Artificial Sequence

<220>
<223> Detection primer for SLC38A1

<400> 385

gggtgttggg gagtttta 18

<210> 386
<211> 25
<212> DNA
<213> Artificial Sequence

<220>
<223> Detection primer for SLC38A1

<400> 386

cttacaataa ctcactatcc tttcc 25

<210> 387
<211> 25
<212> DNA
<213> Artificial Sequence

<220>
<223> Detection primer for SLC38A1

<400> 387

agagtgtggt attagatttg gtttt 25

<210> 388
<211> 25
<212> DNA
<213> Artificial Sequence

<220>
<223> Detection primer for SLC38A1

<400> 388

acaaacatcc tttaataatt tctcc 25

<210> 389
<211> 20
<212> DNA

<213> Artificial Sequence

<220>
<223> Detection primer for HIST1H4J

<400> 389

ttagttgaga aagtgggggt 20

<210> 390
<211> 21
<212> DNA
<213> Artificial Sequence

<220>
<223> Detection primer for HIST1H4J

<400> 390

ctacctcaaa ccaaaatcct c 21

<210> 391
<211> 20
<212> DNA
<213> Artificial Sequence

<220>
<223> Detection primer for Q96S01

<400> 391

gtgagagtgg gtgttgaaat 20

<210> 392
<211> 23
<212> DNA
<213> Artificial Sequence

<220>
<223> Detection primer for Q96S01

<400> 392

accccaatca actacataac taa 23

<210> 393
<211> 25
<212> DNA
<213> Artificial Sequence

<220>
<223> Detection primer for FOXL2

<400> 393

attttaggtg taagtttaag gttgt 25

<210> 394
<211> 18
<212> DNA
<213> Artificial Sequence

<220>
<223> Detection primer for FOXL2

<400> 394

atctaccttt ccccaccc 18

<210> 395
<211> 22
<212> DNA
<213> Artificial Sequence

<220>
<223> Detection primer for ORC4L

<400> 395

gttgagaggt aaggtatgaa gg 22

<210> 396
<211> 25
<212> DNA
<213> Artificial Sequence

<220>
<223> Detection primer for ORC4L

<400> 396

ttaattcccc tctttaacct aataa 25

<210> 397
<211> 20
<212> DNA
<213> Artificial Sequence

<220>
<223> Detection primer for ABHD9

<400> 397

gtgttagggt ttaggggttt 20

<210> 398
<211> 18
<212> DNA
<213> Artificial Sequence

<220>

<223> Detection primer for ABHD9

<400> 398

cctttccaac ctcttcct 18

<210> 399
<211> 21
<212> DNA
<213> Artificial Sequence

<220>
<223> Detection primer for CD37

<400> 399

tagatgggga taggaagttg t 21

<210> 400
<211> 18
<212> DNA
<213> Artificial Sequence

<220>
<223> Detection primer for CD37

<400> 400

cctcatcaac caacccta 18

<210> 401
<211> 22

<212> DNA
<213> Artificial Sequence

<220>
<223> Detection primer for GRN

<400> 401

ttattaggag aggggaagaa gt 22

<210> 402
<211> 18
<212> DNA
<213> Artificial Sequence

<220>
<223> Detection primer for GRN

<400> 402

cattccaaac taacccca 18

<210> 403

<211> 22
<212> DNA
<213> Artificial Sequence

<220>
<223> Detection primer for EPAS1

<400> 403

tattggatgt ttttggtagg tt 22

<210> 404
<211> 18
<212> DNA
<213> Artificial Sequence

<220>
<223> Detection primer for EPAS1

<400> 404

aatcacctcc ctccctta 18

<210> 405
<211> 19
<212> DNA
<213> Artificial Sequence

<220>
<223> Detection primer for NOTCH1

<400> 405

ggaggggttt gagtaattg 19

<210> 406
<211> 20
<212> DNA
<213> Artificial Sequence

<220>
<223> Detection primer for NOTCH1

<400> 406

cccaccctaa aaactcacta 20

<210> 407
<211> 22
<212> DNA
<213> Artificial Sequence

<220>
<223> Detection primer for MLLT3

<400> 407

gattaggttt ggagttgttt tt 22

<210> 408
<211> 22
<212> DNA
<213> Artificial Sequence

<220>
<223> Detection primer for MLLT3

<400> 408

aatcacatcc atctttcact tt 22

<210> 409
<211> 21
<212> DNA
<213> Artificial Sequence

<220>
<223> Detection primer for SOLH

<400> 409

ggggataagt ggttaatgag t 21

<210> 410
<211> 18
<212> DNA
<213> Artificial Sequence

<220>
<223> Detection primer for SOLH

<400> 410

cccaactccc caaataaa 18

<210> 411
<211> 24
<212> DNA
<213> Artificial Sequence

<220>
<223> Detection primer for SEQ ID NO: 44

<400> 411

atagtgtgga tttttaggga tatt 24

<210> 412
<211> 20
<212> DNA
<213> Artificial Sequence

<220>

<223> Detection primer for SEQ ID NO: 44

<400> 412

caaaacctat tcccctacct 20

<210> 413
<211> 25
<212> DNA
<213> Artificial Sequence

<220>
<223> Detection primer for Q8N365

<400> 413

gtagtaaatg ggttatggat tttag 25

<210> 414
<211> 18
<212> DNA
<213> Artificial Sequence

<220>
<223> Detection primer for Q8N365

<400> 414

ccataccacc cacaaaca 18

<210> 415
<211> 26
<212> DNA
<213> Artificial Sequence

<220>
<223> Detection primer for Q9NWV0

<400> 415

ttttagtagt ggatttggtt agtatt 26

<210> 416
<211> 21
<212> DNA
<213> Artificial Sequence

<220>
<223> Detection primer for Q9NWV0

<400> 416

catctcttaa ccccactttc a 21

<210> 417
<211> 22

<212> DNA
<213> Artificial Sequence

<220>
<223> Detection primer for SEQ ID NO: 47

<400> 417

gaggaaagaa ggaggatata gg 22

<210> 418
<211> 21
<212> DNA
<213> Artificial Sequence

<220>
<223> Detection primer for SEQ ID NO: 47

<400> 418

tccctctaaa aaccaaaaat c 21

<210> 419
<211> 21
<212> DNA
<213> Artificial Sequence

<220>
<223> Detection primer for H2AFY2

<400> 419

gttaaagggg tattggtttt t 21

<210> 420
<211> 25
<212> DNA
<213> Artificial Sequence

<220>
<223> Detection primer for H2AFY2

<400> 420

tttctttttc tctcacctat taaac 25

<210> 421
<211> 21
<212> DNA
<213> Artificial Sequence

<220>
<223> Detection primer for RHOC

<400> 421

gtaagaggga tagggaattg g 21

<210> 422
<211> 21
<212> DNA
<213> Artificial Sequence

<220>
<223> Detection primer for RHOC

<400> 422

aacacccaaa ccaaaataaa a 21

<210> 423
<211> 19
<212> DNA
<213> Artificial Sequence

<220>
<223> Detection primer for NR2E1

<400> 423

ggagtttgtg aaaagtggg 19

<210> 424
<211> 26
<212> DNA
<213> Artificial Sequence

<220>
<223> Detection primer for NR2E1

<400> 424

actcaacaaa tacaataatc taaacc 26

<210> 425
<211> 21
<212> DNA
<213> Artificial Sequence

<220>
<223> Detection primer for KBTBD6

<400> 425

gaaggttgag gaggagttag a 21

<210> 426
<211> 27
<212> DNA
<213> Artificial Sequence

<220>
<223> Detection primer for KBTBD6

<400> 426

actataccaa caaaactaca aaataaa 27

<210> 427
<211> 19
<212> DNA
<213> Artificial Sequence

<220>
<223> Detection primer for TRPM4

<400> 427

ggttggaaag tggaggatt 19

<210> 428
<211> 22
<212> DNA
<213> Artificial Sequence

<220>
<223> Detection primer for TRPM4

<400> 428

ccaactctaa aaacaaaaac aa 22

<210> 429
<211> 21
<212> DNA
<213> Artificial Sequence

<220>
<223> Detection primer for TCEB3BP1

<400> 429

gagttggttt tgttgaggtg t 21

<210> 430
<211> 22
<212> DNA
<213> Artificial Sequence

<220>
<223> Detection primer for TCEB3BP1

<400> 430

aaaatacctt cccactaacc tt 22

<210> 431
<211> 19
<212> DNA

<213> Artificial Sequence

<220>
<223> Detection primer for Q8NCX8

<400> 431

tagtggaatt atgaggggg 19

<210> 432
<211> 21
<212> DNA
<213> Artificial Sequence

<220>
<223> Detection primer for Q8NCX8

<400> 432

ccaaatacac ctctaccaaa a 21

<210> 433
<211> 20
<212> DNA
<213> Artificial Sequence

<220>
<223> Detection primer for SEQ ID NO: 55

<400> 433

taggatttgt ggttggtgtt 20

<210> 434
<211> 20
<212> DNA
<213> Artificial Sequence

<220>
<223> Detection primer for SEQ ID NO: 55

<400> 434

ccttaccctc ctctcctaaa 20

<210> 435
<211> 21
<212> DNA
<213> Artificial Sequence

<220>
<223> Detection primer for SNAPC2

<400> 435

ggtttagggt attttaaggg g 21

<210> 436
<211> 21
<212> DNA
<213> Artificial Sequence

<220>
<223> Detection primer for SNAPC2

<400> 436

aaactaaatc caactcccaa a 21

<210> 437
<211> 19
<212> DNA
<213> Artificial Sequence

<220>
<223> Detection primer for PTPRN2

<400> 437

ggggaggtgt ttagtggtt 19

<210> 438
<211> 22
<212> DNA
<213> Artificial Sequence

<220>
<223> Detection primer for PTPRN2

<400> 438

ccctctactc actttaccaa aa 22

<210> 439
<211> 24
<212> DNA
<213> Artificial Sequence

<220>
<223> Detection primer for WDFY3

<400> 439

tgttggtggt tatttttaat tttt 24

<210> 440
<211> 21
<212> DNA
<213> Artificial Sequence

<220>
<223> Detection primer for WDFY3

<400> 440

acccaattat cctttctcaa c 21

<210> 441
<211> 22
<212> DNA
<213> Artificial Sequence

<220>
<223> Detection primer for ZNF566

<400> 441

gaggatttgt gttaaggttt tt 22

<210> 442
<211> 22
<212> DNA
<213> Artificial Sequence

<220>
<223> Detection primer for ZNF566

<400> 442

ccaactccac tatctaccac at 22

<210> 443
<211> 21
<212> DNA
<213> Artificial Sequence

<220>
<223> Detection primer for Q9NP73

<400> 443

ggggttttaa gagttggttt t 21

<210> 444
<211> 20
<212> DNA
<213> Artificial Sequence

<220>
<223> Detection primer for Q9NP73

<400> 444

cctccctcac tcacttacaa 20

<210> 445
<211> 20
<212> DNA

<213> Artificial Sequence

<220>
<223> Detection primer for SEQ ID NO: 61

<400> 445

tggggatatt ggatgttttt 20

<210> 446
<211> 18
<212> DNA
<213> Artificial Sequence

<220>
<223> Detection primer for SEQ ID NO: 61

<400> 446

ccttccaacc taacctcc 18

<210> 447
<211> 22
<212> DNA
<213> Artificial Sequence

<220>
<223> Detection primer for Q86SP6

<400> 447

ggagttttaa tttttgggat tt 22

<210> 448
<211> 23
<212> DNA
<213> Artificial Sequence

<220>
<223> Detection primer for Q86SP6

<400> 448

cccaacactc atttacacta tct 23

<210> 449
<211> 16
<212> DNA
<213> Artificial Sequence

<220>
<223> Detection oligonucleotide for ONECUT2

<400> 449

tagaggcgcg ggttat 16

<210> 450
<211> 16
<212> DNA
<213> Artificial Sequence

<220>
<223> Detection oligonucleotide for ONECUT2

<400> 450

tagaggtgtg ggttat 16

<210> 451
<211> 16
<212> DNA
<213> Artificial Sequence

<220>
<223> Detection oligonucleotide for ONECUT2

<400> 451

ttgcgattgg tacgta 16

<210> 452
<211> 17
<212> DNA
<213> Artificial Sequence

<220>
<223> Detection oligonucleotide for ONECUT2

<400> 452

tgtgattggt atgtagt 17

<210> 453
<211> 16
<212> DNA
<213> Artificial Sequence

<220>
<223> Detection oligonucleotide for ONECUT2

<400> 453

ttttgtgcgt acggat 16

<210> 454
<211> 17
<212> DNA
<213> Artificial Sequence

<220>
<223> Detection oligonucleotide for ONECUT2

<400> 454

tttttgtgtg tatggat 17

<210> 455
<211> 16
<212> DNA
<213> Artificial Sequence

<220>
<223> Detection oligonucleotide for ONECUT2

<400> 455

ttaagcgggc gttgat 16

<210> 456
<211> 16
<212> DNA
<213> Artificial Sequence

<220>
<223> Detection oligonucleotide for ONECUT2

<400> 456

ttaagtgggt gttgat 16

<210> 457
<211> 17
<212> DNA
<213> Artificial Sequence

<220>
<223> Detection oligonucleotide for MX1

<400> 457

gttacgagtt tattcga 17

<210> 458
<211> 19
<212> DNA
<213> Artificial Sequence

<220>
<223> Detection oligonucleotide for MX1

<400> 458

ggttatgagt ttatttgaa 19

<210> 459
<211> 16
<212> DNA
<213> Artificial Sequence

<220>
<223> Detection oligonucleotide for MX1

<400> 459

aacgcgcgaa agtaaa 16

<210> 460
<211> 16
<212> DNA
<213> Artificial Sequence

<220>
<223> Detection oligonucleotide for MX1

<400> 460

ttgggaatgt gtgaaa 16

<210> 461
<211> 16
<212> DNA
<213> Artificial Sequence

<220>
<223> Detection oligonucleotide for MX1

<400> 461

gagttttcgt cgattt 16

<210> 462
<211> 17
<212> DNA
<213> Artificial Sequence

<220>
<223> Detection oligonucleotide for MX1

<400> 462

aggagttttt gttgatt 17

<210> 463
<211> 16
<212> DNA
<213> Artificial Sequence

<220>
<223> Detection oligonucleotide for MX1

<400> 463

tatgcgcggg aagatt 16

<210> 464
<211> 16
<212> DNA
<213> Artificial Sequence

<220>
<223> Detection oligonucleotide for MX1

<400> 464

gtatgtgtgg gaagat 16

<210> 465
<211> 16
<212> DNA
<213> Artificial Sequence

<220>
<223> Detection oligonucleotide for DOCK10

<400> 465

gatcggaatt cgggtt 16

<210> 466
<211> 16
<212> DNA
<213> Artificial Sequence

<220>
<223> Detection oligonucleotide for DOCK10

<400> 466

attggaattt gggttg 16

<210> 467
<211> 16
<212> DNA
<213> Artificial Sequence

<220>
<223> Detection oligonucleotide for DOCK10

<400> 467

tagtagtcgc gttttt 16

<210> 468
<211> 17
<212> DNA
<213> Artificial Sequence

<220>
<223> Detection oligonucleotide for DOCK10

<400> 468

agtagttgtg tttttgg 17

<210> 469
<211> 16
<212> DNA
<213> Artificial Sequence

<220>
<223> Detection oligonucleotide for DOCK10

<400> 469

attttcgcgg gaagtt 16

<210> 470
<211> 16
<212> DNA
<213> Artificial Sequence

<220>
<223> Detection oligonucleotide for DOCK10

<400> 470

gtgatttttg tgggaa 16

<210> 471
<211> 16
<212> DNA
<213> Artificial Sequence

<220>
<223> Detection oligonucleotide for BTG4

<400> 471

agttagcgtt tgtcgg 16

<210> 472
<211> 17
<212> DNA
<213> Artificial Sequence

<220>
<223> Detection oligonucleotide for BTG4

<400> 472

tagttagtgt ttgttgg 17

<210> 473
<211> 16
<212> DNA
<213> Artificial Sequence

<220>
<223> Detection oligonucleotide for BTG4

<400> 473

ttcggcgtcg atgtat 16

<210> 474
<211> 17
<212> DNA
<213> Artificial Sequence

<220>
<223> Detection oligonucleotide for BTG4

<400> 474

tttggtgttg atgtatt 17

<210> 475
<211> 16
<212> DNA
<213> Artificial Sequence

<220>
<223> Detection oligonucleotide for DMRTC2

<400> 475

gggtattcga cgtttt 16

<210> 476
<211> 17
<212> DNA
<213> Artificial Sequence

<220>
<223> Detection oligonucleotide for DMRTC2

<400> 476

aggggtattt gatgttt 17

<210> 477
<211> 16
<212> DNA
<213> Artificial Sequence

<220>

<223> Detection oligonucleotide for DMRTC2

<400> 477

attcgaagcg ttattg 16

<210> 478
<211> 17
<212> DNA
<213> Artificial Sequence

<220>
<223> Detection oligonucleotide for DMRTC2

<400> 478

tttgaagtgt tattggt 17

<210> 479
<211> 16
<212> DNA
<213> Artificial Sequence

<220>
<223> Detection oligonucleotide for DMRTC2

<400> 479

aaatcgtatt ggtcgt 16

<210> 480
<211> 18
<212> DNA
<213> Artificial Sequence

<220>
<223> Detection oligonucleotide for DMRTC2

<400> 480

aaattgtatt ggttgttt 18

<210> 481
<211> 18
<212> DNA
<213> Artificial Sequence

<220>
<223> Detection oligonucleotide for DMRTC2

<400> 481

aattcgtgta tagatcgg 18

<210> 482
<211> 18
<212> DNA
<213> Artificial Sequence

<220>
<223> Detection oligonucleotide for DMRTC2

<400> 482

atttgtgtat agattggg 18

<210> 483
<211> 16
<212> DNA
<213> Artificial Sequence

<220>
<223> Detection oligonucleotide for DMRTC2

<400> 483

aaaagtagcg cgagtt 16

<210> 484
<211> 16
<212> DNA
<213> Artificial Sequence

<220>
<223> Detection oligonucleotide for DMRTC2

<400> 484

agtagtgtga gtttgg 16

<210> 485
<211> 16
<212> DNA
<213> Artificial Sequence

<220>
<223> Detection oligonucleotide for GPR7

<400> 485

gaacgtagtc gcggtt 16

<210> 486
<211> 16
<212> DNA
<213> Artificial Sequence

<220>
<223> Detection oligonucleotide for GPR7

<400> 486

ggaatgtagt tgtggt 16

<210> 487
<211> 16
<212> DNA
<213> Artificial Sequence

<220>
<223> Detection oligonucleotide for GPR7

<400> 487

attttggcga attcgg 16

<210> 488
<211> 16
<212> DNA
<213> Artificial Sequence

<220>
<223> Detection oligonucleotide for GPR7

<400> 488

tggtgaattt ggggga 16

<210> 489
<211> 16
<212> DNA
<213> Artificial Sequence

<220>
<223> Detection oligonucleotide for GPR7

<400> 489

ttagtcggta ggcgtt 16

<210> 490
<211> 17
<212> DNA
<213> Artificial Sequence

<220>
<223> Detection oligonucleotide for GPR7

<400> 490

atttagttgg taggtgt 17

<210> 491
<211> 16
<212> DNA
<213> Artificial Sequence

<220>
<223> Detection oligonucleotide for GPR7

<400> 491

ttttcgtagt cggcgg 16

<210> 492
<211> 17
<212> DNA
<213> Artificial Sequence

<220>
<223> Detection oligonucleotide for GPR7

<400> 492

tttttgtag ttggtgg 17

<210> 493
<211> 18
<212> DNA
<213> Artificial Sequence

<220>
<223> Detection oligonucleotide for FAT

<400> 493

taagcgttaa tagaacga 18

<210> 494
<211> 19
<212> DNA
<213> Artificial Sequence

<220>
<223> Detection oligonucleotide for FAT

<400> 494

agtgttaata gaatgaaat 19

<210> 495
<211> 17
<212> DNA
<213> Artificial Sequence

<220>
<223> Detection oligonucleotide for FAT

<400> 495

ttgtatttcg tcgttat 17

<210> 496
<211> 18
<212> DNA
<213> Artificial Sequence

<220>
<223> Detection oligonucleotide for FAT

<400> 496

ttttgttgtt ataggagt 18

<210> 497
<211> 16
<212> DNA
<213> Artificial Sequence

<220>
<223> Detection oligonucleotide for FAT

<400> 497

ataggagtcg tcgaga 16

<210> 498
<211> 17
<212> DNA
<213> Artificial Sequence

<220>
<223> Detection oligonucleotide for FAT

<400> 498

ataggagttg ttgagag 17

<210> 499
<211> 16
<212> DNA
<213> Artificial Sequence

<220>
<223> Detection oligonucleotide for ISL1

<400> 499

ttaatgcggt cggtta 16

<210> 500
<211> 16
<212> DNA
<213> Artificial Sequence

<220>
<223> Detection oligonucleotide for ISL1

<400> 500

agttaatgtg gttggt 16

<210> 501
<211> 16
<212> DNA
<213> Artificial Sequence

<220>
<223> Detection oligonucleotide for GPRK5

<400> 501

tttgattcgc ggtcgg 16

<210> 502
<211> 17
<212> DNA
<213> Artificial Sequence

<220>
<223> Detection oligonucleotide for GPRK5

<400> 502

atttgatttg tggttgg 17

<210> 503
<211> 16
<212> DNA
<213> Artificial Sequence

<220>
<223> Detection oligonucleotide for GPRK5

<400> 503

tatcgtcggt cgagtt 16

<210> 504
<211> 17
<212> DNA
<213> Artificial Sequence

<220>
<223> Detection oligonucleotide for GPRK5

<400> 504

tttattgttg gttgagt 17

<210> 505
<211> 16
<212> DNA
<213> Artificial Sequence

<220>
<223> Detection oligonucleotide for GPRK5

<400> 505

atgtcgagag ttcgta 16

<210> 506

<211> 17
<212> DNA
<213> Artificial Sequence

<220>
<223> Detection oligonucleotide for GPRK5

<400> 506

gttgagagtt tgtatgt 17

<210> 507
<211> 16
<212> DNA
<213> Artificial Sequence

<220>
<223> Detection oligonucleotide for SLC35F2

<400> 507

tttcggcgtt taaaat 16

<210> 508
<211> 19
<212> DNA
<213> Artificial Sequence

<220>
<223> Detection oligonucleotide for SLC35F2

<400> 508

tttttggtgt ttaaaattt 19

<210> 509
<211> 16
<212> DNA
<213> Artificial Sequence

<220>
<223> Detection oligonucleotide for SLC35F2

<400> 509

attttcgaag tgtcgg 16

<210> 510
<211> 16
<212> DNA
<213> Artificial Sequence

<220>
<223> Detection oligonucleotide for SLC35F2

<400> 510

tttgaagtgt tgggtt 16

<210> 511
<211> 16
<212> DNA
<213> Artificial Sequence

<220>
<223> Detection oligonucleotide for SLC35F2

<400> 511

tttcggaaga cgggag 16

<210> 512
<211> 16
<212> DNA
<213> Artificial Sequence

<220>
<223> Detection oligonucleotide for SLC35F2

<400> 512

ttttggaaga tgggag 16

<210> 513
<211> 16
<212> DNA
<213> Artificial Sequence

<220>
<223> Detection oligonucleotide for SLC35F2

<400> 513

aattcggtcg tcgttt 16

<210> 514
<211> 17
<212> DNA
<213> Artificial Sequence

<220>
<223> Detection oligonucleotide for SLC35F2

<400> 514

agaatttggt tgttgtt 17

<210> 515
<211> 16
<212> DNA
<213> Artificial Sequence

<220>

<223> Detection oligonucleotide for C14orf59

<400> 515

gacgtaggga cggaga 16

<210> 516
<211> 16
<212> DNA
<213> Artificial Sequence

<220>
<223> Detection oligonucleotide for C14orf59

<400> 516

gatgtaggga tggaga 16

<210> 517
<211> 20
<212> DNA
<213> Artificial Sequence

<220>
<223> Detection oligonucleotide for C14orf59

<400> 517

tatcgtggtt ttttacgtat 20

<210> 518
<211> 20
<212> DNA
<213> Artificial Sequence

<220>
<223> Detection oligonucleotide for C14orf59

<400> 518

attgtggttt tttatgtata 20

<210> 519
<211> 16
<212> DNA
<213> Artificial Sequence

<220>
<223> Detection oligonucleotide for C14orf59

<400> 519

gtgttcgaga gcgagt 16

<210> 520
<211> 17

<212> DNA
<213> Artificial Sequence

<220>
<223> Detection oligonucleotide for C14orf59

<400> 520

tgtttgagag tgagtgt 17

<210> 521
<211> 16
<212> DNA
<213> Artificial Sequence

<220>
<223> Detection oligonucleotide for C14orf59

<400> 521

tttattcggt gttcga 16

<210> 522
<211> 17
<212> DNA
<213> Artificial Sequence

<220>
<223> Detection oligonucleotide for C14orf59

<400> 522

tatttggtgt ttgagag 17

<210> 523
<211> 16
<212> DNA
<213> Artificial Sequence

<220>
<223> Detection oligonucleotide for SNRPN

<400> 523

tttttgcggt cgcgta 16

<210> 524
<211> 17
<212> DNA
<213> Artificial Sequence

<220>
<223> Detection oligonucleotide for SNRPN

<400> 524

ttttgtggtt gtgtagg 17

<210> 525
<211> 16
<212> DNA
<213> Artificial Sequence

<220>
<223> Detection oligonucleotide for SNRPN

<400> 525

agtatgcgcg ttagtt 16

<210> 526
<211> 17
<212> DNA
<213> Artificial Sequence

<220>
<223> Detection oligonucleotide for SNRPN

<400> 526

tgagtatgtg tgttagt 17

<210> 527
<211> 17
<212> DNA
<213> Artificial Sequence

<220>
<223> Detection oligonucleotide for SNRPN

<400> 527

tagcggtagg tttcgta 17

<210> 528
<211> 17
<212> DNA
<213> Artificial Sequence

<220>
<223> Detection oligonucleotide for SNRPN

<400> 528

tagtggtagg ttttgta 17

<210> 529
<211> 16
<212> DNA
<213> Artificial Sequence

<220>
<223> Detection oligonucleotide for SNRPN

<400> 529

tttgcgttag attcgt 16

<210> 530
<211> 17
<212> DNA
<213> Artificial Sequence

<220>
<223> Detection oligonucleotide for SNRPN

<400> 530

tgtgttagat ttgttgt 17

<210> 531
<211> 16
<212> DNA
<213> Artificial Sequence

<220>
<223> Detection oligonucleotide for ARHGEF18

<400> 531

gtcgattcgg ttgatt 16

<210> 532
<211> 16
<212> DNA
<213> Artificial Sequence

<220>
<223> Detection oligonucleotide for ARHGEF18

<400> 532

aaggttgatt tggttg 16

<210> 533
<211> 16

<212> DNA
<213> Artificial Sequence

<220>
<223> Detection oligonucleotide for ARHGEF18

<400> 533

ggcggtttcg aagatt 16

<210> 534
<211> 16

<212> DNA
<213> Artificial Sequence

<220>
<223> Detection oligonucleotide for ARHGEF18

<400> 534

agatgggtgg ttttga 16

<210> 535
<211> 17
<212> DNA
<213> Artificial Sequence

<220>
<223> Detection oligonucleotide for ARHGEF18

<400> 535

aagtcggtta tgagcga 17

<210> 536
<211> 17
<212> DNA
<213> Artificial Sequence

<220>
<223> Detection oligonucleotide for ARHGEF18

<400> 536

aagttggtta tgagtga 17

<210> 537
<211> 16
<212> DNA
<213> Artificial Sequence

<220>
<223> Detection oligonucleotide for ARHGEF18

<400> 537

tggtcgatac ggtatt 16

<210> 538
<211> 16
<212> DNA
<213> Artificial Sequence

<220>
<223> Detection oligonucleotide for ARHGEF18

<400> 538

ttgtggttga tatggt 16

<210> 539
<211> 16
<212> DNA
<213> Artificial Sequence

<220>
<223> Detection oligonucleotide for SNX8

<400> 539

aggacgcgat agggat 16

<210> 540
<211> 16
<212> DNA
<213> Artificial Sequence

<220>
<223> Detection oligonucleotide for SNX8

<400> 540

aggatgtgat agggat 16

<210> 541
<211> 16
<212> DNA
<213> Artificial Sequence

<220>
<223> Detection oligonucleotide for SNX8

<400> 541

atttcgtcgt atgtga 16

<210> 542
<211> 18
<212> DNA
<213> Artificial Sequence

<220>
<223> Detection oligonucleotide for SNX8

<400> 542

attttgttgt atgtgaag 18

<210> 543
<211> 16
<212> DNA
<213> Artificial Sequence

<220>
<223> Detection oligonucleotide for SNX8

<400> 543

gtcgtttgcg tattta 16

<210> 544
<211> 18
<212> DNA
<213> Artificial Sequence

<220>
<223> Detection oligonucleotide for SNX8

<400> 544

ggttgtttgt gtatttaa 18

<210> 545
<211> 16
<212> DNA
<213> Artificial Sequence

<220>
<223> Detection oligonucleotide for SNX8

<400> 545

attgtatacg cgcgtt 16

<210> 546
<211> 17
<212> DNA
<213> Artificial Sequence

<220>
<223> Detection oligonucleotide for SNX8

<400> 546

ttgtatatgt gtgttgg 17

<210> 547
<211> 16
<212> DNA
<213> Artificial Sequence

<220>
<223> Detection oligonucleotide for FBN2

<400> 547

taaagcgagt agacgg 16

<210> 548

<211> 18
<212> DNA
<213> Artificial Sequence

<220>
<223> Detection oligonucleotide for FBN2

<400> 548

tataaagtga gtagatgg 18

<210> 549
<211> 16
<212> DNA
<213> Artificial Sequence

<220>
<223> Detection oligonucleotide for HOXB5

<400> 549

atagttttcg gcgggt 16

<210> 550
<211> 17
<212> DNA
<213> Artificial Sequence

<220>
<223> Detection oligonucleotide for HOXB5

<400> 550

tatagttttt ggtgggt 17

<210> 551
<211> 16
<212> DNA
<213> Artificial Sequence

<220>
<223> Detection oligonucleotide for HOXB5

<400> 551

tttttcggcg tagata 16

<210> 552
<211> 17
<212> DNA
<213> Artificial Sequence

<220>
<223> Detection oligonucleotide for HOXB5

<400> 552

tgttttttgg tgtagat 17

<210> 553
<211> 16
<212> DNA
<213> Artificial Sequence

<220>
<223> Detection oligonucleotide for HOXB5

<400> 553

agtcgagggc gttaga 16

<210> 554
<211> 16
<212> DNA
<213> Artificial Sequence

<220>
<223> Detection oligonucleotide for HOXB5

<400> 554

agttgagggt gttaga 16

<210> 555
<211> 16
<212> DNA
<213> Artificial Sequence

<220>
<223> Detection oligonucleotide for HOXB5

<400> 555

ttttcgagga attcgt 16

<210> 556
<211> 18
<212> DNA
<213> Artificial Sequence

<220>
<223> Detection oligonucleotide for HOXB5

<400> 556

ttttttgagg aatttgtt 18

<210> 557
<211> 16
<212> DNA
<213> Artificial Sequence

<220>

<223> Detection oligonucleotide for LIMK1

<400> 557

tatcggatta tcgcgg 16

<210> 558
<211> 17
<212> DNA
<213> Artificial Sequence

<220>
<223> Detection oligonucleotide for LIMK1

<400> 558

attggattat tgtgggg 17

<210> 559
<211> 18
<212> DNA
<213> Artificial Sequence

<220>
<223> Detection oligonucleotide for LIMK1

<400> 559

gtcggtagtt tatcggat 18

<210> 560
<211> 18
<212> DNA
<213> Artificial Sequence

<220>
<223> Detection oligonucleotide for LIMK1

<400> 560

gttggtagtt tattggat 18

<210> 561
<211> 16
<212> DNA
<213> Artificial Sequence

<220>
<223> Detection oligonucleotide for LIMK1

<400> 561

taggagacgt tacgtt 16

<210> 562
<211> 17

<212> DNA
<213> Artificial Sequence

<220>
<223> Detection oligonucleotide for LIMK1

<400> 562

agatgttatg ttagggt 17

<210> 563
<211> 16
<212> DNA
<213> Artificial Sequence

<220>
<223> Detection oligonucleotide for PSD/Q9H469

<400> 563

tttttcgaag cggatt 16

<210> 564
<211> 17
<212> DNA
<213> Artificial Sequence

<220>
<223> Detection oligonucleotide for PSD/Q9H469

<400> 564

tttgaagtgg attttgg 17

<210> 565
<211> 16
<212> DNA
<213> Artificial Sequence

<220>
<223> Detection oligonucleotide for PSD/Q9H469

<400> 565

gaaacgcggt ttaaat 16

<210> 566
<211> 18
<212> DNA
<213> Artificial Sequence

<220>
<223> Detection oligonucleotide for PSD/Q9H469

<400> 566

ggaaatgtgg tttaaatt 18

<210> 567
<211> 16
<212> DNA
<213> Artificial Sequence

<220>
<223> Detection oligonucleotide for SLC38A1

<400> 567

tttgcggtaa cgttta 16

<210> 568
<211> 17
<212> DNA
<213> Artificial Sequence

<220>
<223> Detection oligonucleotide for SLC38A1

<400> 568

ttgtggtaat gtttagg 17

<210> 569
<211> 16
<212> DNA
<213> Artificial Sequence

<220>
<223> Detection oligonucleotide for SLC38A1

<400> 569

tagcggtcgc ggatta 16

<210> 570
<211> 16
<212> DNA
<213> Artificial Sequence

<220>
<223> Detection oligonucleotide for SLC38A1

<400> 570

gtagtggttg tggatt 16

<210> 571
<211> 16
<212> DNA
<213> Artificial Sequence

<220>
<223> Detection oligonucleotide for SLC38A1

<400> 571

ttagggacgc gaatta 16

<210> 572
<211> 16
<212> DNA
<213> Artificial Sequence

<220>
<223> Detection oligonucleotide for SLC38A1

<400> 572

agggatgtga attagg 16

<210> 573
<211> 17
<212> DNA
<213> Artificial Sequence

<220>
<223> Detection oligonucleotide for SLC38A1

<400> 573

tgcgtttaag atcgcgt 17

<210> 574
<211> 17
<212> DNA
<213> Artificial Sequence

<220>
<223> Detection oligonucleotide for SLC38A1

<400> 574

tgtgtttaag attgtgt 17

<210> 575
<211> 16
<212> DNA
<213> Artificial Sequence

<220>
<223> Detection oligonucleotide for SLC38A1

<400> 575

atttcggttt tcgaaa 16

<210> 576
<211> 19
<212> DNA

<213> Artificial Sequence

<220>
<223> Detection oligonucleotide for SLC38A1

<400> 576

atattttggt ttttgaaaa 19

<210> 577
<211> 18
<212> DNA
<213> Artificial Sequence

<220>
<223> Detection oligonucleotide for SLC38A1

<400> 577

agagcgtagt tgattcga 18

<210> 578
<211> 18
<212> DNA
<213> Artificial Sequence

<220>
<223> Detection oligonucleotide for SLC38A1

<400> 578

agagtgtagt tgatttga 18

<210> 579
<211> 16
<212> DNA
<213> Artificial Sequence

<220>
<223> Detection oligonucleotide for SLC38A1

<400> 579

aggaattacg tacgtt 16

<210> 580
<211> 17
<212> DNA
<213> Artificial Sequence

<220>
<223> Detection oligonucleotide for SLC38A1

<400> 580

tatgtatgtt tggaggg 17

<210> 581
<211> 16
<212> DNA
<213> Artificial Sequence

<220>
<223> Detection oligonucleotide for SLC38A1

<400> 581

tttgtaacgc ggggaa 16

<210> 582
<211> 16
<212> DNA
<213> Artificial Sequence

<220>
<223> Detection oligonucleotide for SLC38A1

<400> 582

ttttgtaatg tgggga 16

<210> 583
<211> 16
<212> DNA
<213> Artificial Sequence

<220>
<223> Detection oligonucleotide for HIST1H4J

<400> 583

tatggcggtg atcgtt 16

<210> 584
<211> 17
<212> DNA
<213> Artificial Sequence

<220>
<223> Detection oligonucleotide for HIST1H4J

<400> 584

tttatggtgg tgattgt 17

<210> 585
<211> 16
<212> DNA
<213> Artificial Sequence

<220>
<223> Detection oligonucleotide for HIST1H4J

<400> 585

ttacggcgtt tcggat 16

<210> 586
<211> 17
<212> DNA
<213> Artificial Sequence

<220>
<223> Detection oligonucleotide for HIST1H4J

<400> 586

ttatggtgtt ttggatt 17

<210> 587
<211> 16
<212> DNA
<213> Artificial Sequence

<220>
<223> Detection oligonucleotide for HIST1H4J

<400> 587

atgcgtttta cgtcgt 16

<210> 588
<211> 18
<212> DNA
<213> Artificial Sequence

<220>
<223> Detection oligonucleotide for HIST1H4J

<400> 588

agatgtgttt tatgttgt 18

<210> 589
<211> 16
<212> DNA
<213> Artificial Sequence

<220>
<223> Detection oligonucleotide for HIST1H4J

<400> 589

tattgtcgcg tagtat 16

<210> 590
<211> 17
<212> DNA

<213> Artificial Sequence

<220>
<223> Detection oligonucleotide for HIST1H4J

<400> 590

ggatattgtt gtgtagt 17

<210> 591
<211> 16
<212> DNA
<213> Artificial Sequence

<220>
<223> Detection oligonucleotide for HIST1H4J

<400> 591

atcgaaatcg tagagg 16

<210> 592
<211> 17
<212> DNA
<213> Artificial Sequence

<220>
<223> Detection oligonucleotide for HIST1H4J

<400> 592

attgaaattg tagaggg 17

<210> 593
<211> 16
<212> DNA
<213> Artificial Sequence

<220>
<223> Detection oligonucleotide for Q96S01

<400> 593

atcggttttt cgaggt 16

<210> 594
<211> 17
<212> DNA
<213> Artificial Sequence

<220>
<223> Detection oligonucleotide for Q96S01

<400> 594

attggttttt tgaggtt 17

<210> 595
<211> 16
<212> DNA
<213> Artificial Sequence

<220>
<223> Detection oligonucleotide for Q96S01

<400> 595

ggtcgatttt cgcgta 16

<210> 596
<211> 17
<212> DNA
<213> Artificial Sequence

<220>
<223> Detection oligonucleotide for Q96S01

<400> 596

tggttgattt ttgtgta 17

<210> 597
<211> 16
<212> DNA
<213> Artificial Sequence

<220>
<223> Detection oligonucleotide for FOXL2

<400> 597

aatcgtgcgg ttgata 16

<210> 598
<211> 16
<212> DNA
<213> Artificial Sequence

<220>
<223> Detection oligonucleotide for FOXL2

<400> 598

tgtagaattg tgtggt 16

<210> 599
<211> 16
<212> DNA
<213> Artificial Sequence

<220>
<223> Detection oligonucleotide for FOXL2

<400> 599

aaaattcgag gtcggg 16

<210> 600
<211> 16
<212> DNA
<213> Artificial Sequence

<220>
<223> Detection oligonucleotide for FOXL2

<400> 600

aaaatttgag gttggg 16

<210> 601
<211> 16
<212> DNA
<213> Artificial Sequence

<220>
<223> Detection oligonucleotide for FOXL2

<400> 601

ttttcgcggt tcggag 16

<210> 602
<211> 16
<212> DNA
<213> Artificial Sequence

<220>
<223> Detection oligonucleotide for FOXL2

<400> 602

tttgtggttt ggagaa 16

<210> 603
<211> 16
<212> DNA
<213> Artificial Sequence

<220>
<223> Detection oligonucleotide for FOXL2

<400> 603

aataggcgat gtacgg 16

<210> 604
<211> 16
<212> DNA

<213> Artificial Sequence

<220>
<223> Detection oligonucleotide for FOXL2

<400> 604

taggtgatgt atgggt 16

<210> 605
<211> 16
<212> DNA
<213> Artificial Sequence

<220>
<223> Detection oligonucleotide for FOXL2

<400> 605

ttggtcggtt aatcga 16

<210> 606
<211> 17
<212> DNA
<213> Artificial Sequence

<220>
<223> Detection oligonucleotide for FOXL2

<400> 606

tttggttggt taattga 17

<210> 607
<211> 16
<212> DNA
<213> Artificial Sequence

<220>
<223> Detection oligonucleotide for FOXL2

<400> 607

tagcggtcgc gaaaat 16

<210> 608
<211> 16
<212> DNA
<213> Artificial Sequence

<220>
<223> Detection oligonucleotide for FOXL2

<400> 608

agtttagtgg ttgtga 16

<210> 609
<211> 16
<212> DNA
<213> Artificial Sequence

<220>
<223> Detection oligonucleotide for CMYA3

<400> 609

tttacgcggg gtttta 16

<210> 610
<211> 17
<212> DNA
<213> Artificial Sequence

<220>
<223> Detection oligonucleotide for CMYA3

<400> 610

tttatgtggg gttttag 17

<210> 611
<211> 17
<212> DNA
<213> Artificial Sequence

<220>
<223> Detection oligonucleotide for CMYA3

<400> 611

ttacgtcgtt attaggt 17

<210> 612
<211> 19
<212> DNA
<213> Artificial Sequence

<220>
<223> Detection oligonucleotide for CMYA3

<400> 612

ttttatgttg ttattaggt 19

<210> 613
<211> 16
<212> DNA
<213> Artificial Sequence

<220>
<223> Detection oligonucleotide for CMYA3

<400> 613

tatttggacg tcgggt 16

<210> 614
<211> 16
<212> DNA
<213> Artificial Sequence

<220>
<223> Detection oligonucleotide for CMYA3

<400> 614

tatttggatg ttgggt 16

<210> 615
<211> 16
<212> DNA
<213> Artificial Sequence

<220>
<223> Detection oligonucleotide for CMYA3

<400> 615

tttgtcggaa agcgga 16

<210> 616
<211> 16
<212> DNA
<213> Artificial Sequence

<220>
<223> Detection oligonucleotide for CMYA3

<400> 616

tttgttggaa agtgga 16

<210> 617
<211> 16
<212> DNA
<213> Artificial Sequence

<220>
<223> Detection oligonucleotide for ORC4L

<400> 617

attcggatcg ttacgt 16

<210> 618
<211> 18
<212> DNA
<213> Artificial Sequence

<220>
<223> Detection oligonucleotide for ORC4L

<400> 618

atttggattg ttatgttt 18

<210> 619
<211> 16
<212> DNA
<213> Artificial Sequence

<220>
<223> Detection oligonucleotide for ORC4L

<400> 619

ataagacgga gttcgt 16

<210> 620
<211> 17
<212> DNA
<213> Artificial Sequence

<220>
<223> Detection oligonucleotide for ORC4L

<400> 620

aagatggagt ttgtttg 17

<210> 621
<211> 16
<212> DNA
<213> Artificial Sequence

<220>
<223> Detection oligonucleotide for ORC4L

<400> 621

ttgcgttatc gacgtt 16

<210> 622
<211> 18
<212> DNA
<213> Artificial Sequence

<220>
<223> Detection oligonucleotide for ORC4L

<400> 622

attttgtgtt attgatgt 18

<210> 623
<211> 16
<212> DNA
<213> Artificial Sequence

<220>
<223> Detection oligonucleotide for ORC4L

<400> 623

gagtaacgcg tgtgat 16

<210> 624
<211> 17
<212> DNA
<213> Artificial Sequence

<220>
<223> Detection oligonucleotide for ORC4L

<400> 624

tatgagtaat gtgtgtg 17

<210> 625
<211> 16
<212> DNA
<213> Artificial Sequence

<220>
<223> Detection oligonucleotide for ABHD9

<400> 625

tatttgggcg cgatag 16

<210> 626
<211> 16
<212> DNA
<213> Artificial Sequence

<220>
<223> Detection oligonucleotide for ABHD9

<400> 626

atttgggtgt gatagg 16

<210> 627
<211> 16
<212> DNA
<213> Artificial Sequence

<220>
<223> Detection oligonucleotide for ABHD9

<400> 627

gtgggacgcg ttgaag 16

<210> 628
<211> 16
<212> DNA
<213> Artificial Sequence

<220>

<223> Detection oligonucleotide for ABHD9

<400> 628

tgtgggatgt gttgaa 16

<210> 629
<211> 16
<212> DNA
<213> Artificial Sequence

<220>
<223> Detection oligonucleotide for ABHD9

<400> 629

ggcggtttcg atagaa 16

<210> 630
<211> 16
<212> DNA
<213> Artificial Sequence

<220>
<223> Detection oligonucleotide for ABHD9

<400> 630

gggtggtttt gataga 16

<210> 631
<211> 16
<212> DNA
<213> Artificial Sequence

<220>
<223> Detection oligonucleotide for CCND2

<400> 631

ataagtcgtt cgaggt 16

<210> 632
<211> 16
<212> DNA
<213> Artificial Sequence

<220>
<223> Detection oligonucleotide for CCND2

<400> 632

aagttgtttg aggtgt 16

<210> 633
<211> 16
<212> DNA
<213> Artificial Sequence

<220>
<223> Detection oligonucleotide for CCND2

<400> 633

tagcggttac gtagga 16

<210> 634
<211> 17
<212> DNA
<213> Artificial Sequence

<220>
<223> Detection oligonucleotide for CCND2

<400> 634

agtggttatg taggaaa 17

<210> 635
<211> 17
<212> DNA
<213> Artificial Sequence

<220>
<223> Detection oligonucleotide for CCND2

<400> 635

tacgtgtttt aacgtat 17

<210> 636
<211> 19
<212> DNA
<213> Artificial Sequence

<220>
<223> Detection oligonucleotide for CCND2

<400> 636

tgatatgtgt tttaatgta 19

<210> 637
<211> 17
<212> DNA
<213> Artificial Sequence

<220>
<223> Detection oligonucleotide for CCND2

<400> 637

ttagggtcgt cgtaggt 17

<210> 638
<211> 17
<212> DNA
<213> Artificial Sequence

<220>
<223> Detection oligonucleotide for CCND2

<400> 638

ttagggttgt tgtaggt 17

<210> 639
<211> 16
<212> DNA
<213> Artificial Sequence

<220>
<223> Detection oligonucleotide for CCND2

<400> 639

ttagtacggt cggttt 16

<210> 640
<211> 17
<212> DNA
<213> Artificial Sequence

<220>
<223> Detection oligonucleotide for CCND2

<400> 640

gttagtatgg ttggttt 17

<210> 641
<211> 16
<212> DNA
<213> Artificial Sequence

<220>
<223> Detection oligonucleotide for CDKN2A

<400> 641

taggtatcgc gtacgt 16

<210> 642
<211> 18
<212> DNA
<213> Artificial Sequence

<220>
<223> Detection oligonucleotide for CDKN2A

<400> 642

ttaggtattg tgtatgtt 18

<210> 643
<211> 16
<212> DNA
<213> Artificial Sequence

<220>
<223> Detection oligonucleotide for CDKN2A

<400> 643

ttcgcgtcgt ggagta 16

<210> 644
<211> 17
<212> DNA
<213> Artificial Sequence

<220>
<223> Detection oligonucleotide for CDKN2A

<400> 644

ttttgtgttg tggagta 17

<210> 645
<211> 16
<212> DNA
<213> Artificial Sequence

<220>
<223> Detection oligonucleotide for CDKN2A

<400> 645

tagtcgcgcg taggta 16

<210> 646
<211> 17
<212> DNA
<213> Artificial Sequence

<220>
<223> Detection oligonucleotide for CDKN2A

<400> 646

tagttgtgtg taggtat 17

<210> 647
<211> 16
<212> DNA
<213> Artificial Sequence

<220>
<223> Detection oligonucleotide for CDKN2A

<400> 647

taggtatcgt gcgata 16

<210> 648
<211> 18
<212> DNA
<213> Artificial Sequence

<220>
<223> Detection oligonucleotide for CDKN2A

<400> 648

gtaggtattg tgtgatat 18

<210> 649
<211> 17
<212> DNA
<213> Artificial Sequence

<220>
<223> Detection oligonucleotide for CD44

<400> 649

taggttcggt tcgttat 17

<210> 650
<211> 18
<212> DNA
<213> Artificial Sequence

<220>
<223> Detection oligonucleotide for CD44

<400> 650

taggtttggt ttgttatt 18

<210> 651
<211> 16
<212> DNA
<213> Artificial Sequence

<220>
<223> Detection oligonucleotide for CD44

<400> 651

gtttcgcgtt taggga 16

<210> 652
<211> 17
<212> DNA
<213> Artificial Sequence

<220>
<223> Detection oligonucleotide for CD44

<400> 652

gttttgtgtt tagggat 17

<210> 653
<211> 17
<212> DNA
<213> Artificial Sequence

<220>
<223> Detection oligonucleotide for CD44

<400> 653

gttcgtttcg gatatta 17

<210> 654
<211> 19
<212> DNA
<213> Artificial Sequence

<220>
<223> Detection oligonucleotide for CD44

<400> 654

tttgttttgg atattatgg 19

<210> 655
<211> 16
<212> DNA
<213> Artificial Sequence

<220>
<223> Detection oligonucleotide for CD44

<400> 655

tttggcgtag atcggt 16

<210> 656
<211> 16
<212> DNA
<213> Artificial Sequence

<220>
<223> Detection oligonucleotide for CD44

<400> 656

tttggtgtag attggt 16

<210> 657
<211> 16
<212> DNA
<213> Artificial Sequence

<220>
<223> Detection oligonucleotide for EDNRB1

<400> 657

ttcgtttttc gggaag 16

<210> 658
<211> 17
<212> DNA

<213> Artificial Sequence

<220>
<223> Detection oligonucleotide for EDNRB1

<400> 658

tttgtttttt gggaagg 17

<210> 659
<211> 16
<212> DNA
<213> Artificial Sequence

<220>
<223> Detection oligonucleotide for EDNRB1

<400> 659

tagagtcgga ttcgtt 16

<210> 660
<211> 17
<212> DNA
<213> Artificial Sequence

<220>
<223> Detection oligonucleotide for EDNRB1

<400> 660

agttggattt gtttgta 17

<210> 661
<211> 16
<212> DNA
<213> Artificial Sequence

<220>
<223> Detection oligonucleotide for EDNRB1

<400> 661

gtattttcgt agcgtt 16

<210> 662
<211> 18
<212> DNA
<213> Artificial Sequence

<220>
<223> Detection oligonucleotide for EDNRB1

<400> 662

tggtattttt gtagtgtt 18

<210> 663
<211> 16

<212> DNA
<213> Artificial Sequence

<220>
<223> Detection oligonucleotide for EDNRB1

<400> 663

atttcgagta aacggt 16

<210> 664
<211> 17
<212> DNA
<213> Artificial Sequence

<220>
<223> Detection oligonucleotide for EDNRB1

<400> 664

tttgagtaaa tggtgga 17

<210> 665
<211> 16
<212> DNA
<213> Artificial Sequence

<220>
<223> Detection oligonucleotide for ELK1

<400> 665

tgtcgtacgt tatgtt 16

<210> 666
<211> 17
<212> DNA
<213> Artificial Sequence

<220>
<223> Detection oligonucleotide for ELK1

<400> 666

ggtgttgtat gttatgt 17

<210> 667
<211> 16
<212> DNA
<213> Artificial Sequence

<220>
<223> Detection oligonucleotide for ELK1

<400> 667

tgggcgtagt agtcgg 16

<210> 668
<211> 17
<212> DNA
<213> Artificial Sequence

<220>
<223> Detection oligonucleotide for ELK1

<400> 668

atgggtgtag tagttgg 17

<210> 669
<211> 17
<212> DNA
<213> Artificial Sequence

<220>
<223> Detection oligonucleotide for ELK1

<400> 669

attgggtttc gcgtagg 17

<210> 670
<211> 17
<212> DNA
<213> Artificial Sequence

<220>
<223> Detection oligonucleotide for ELK1

<400> 670

attgggtttt gtgtagg 17

<210> 671
<211> 16
<212> DNA
<213> Artificial Sequence

<220>
<223> Detection oligonucleotide for ELK1

<400> 671

ttgattggcg gacgag 16

<210> 672
<211> 16
<212> DNA
<213> Artificial Sequence

<220>
<223> Detection oligonucleotide for ELK1

<400> 672

ttgattggtg gatgag 16

<210> 673
<211> 17
<212> DNA
<213> Artificial Sequence

<220>
<223> Detection oligonucleotide for FOS

<400> 673

tacggatttg gtcgttt 17

<210> 674
<211> 17
<212> DNA

<213> Artificial Sequence

<220>
<223> Detection oligonucleotide for FOS

<400> 674

tatggatttg gttgttt 17

<210> 675
<211> 16
<212> DNA
<213> Artificial Sequence

<220>
<223> Detection oligonucleotide for FOS

<400> 675

ttcgattagt tcggat 16

<210> 676
<211> 19
<212> DNA
<213> Artificial Sequence

<220>
<223> Detection oligonucleotide for FOS

<400> 676

tattttgatt agtttggat 19

<210> 677
<211> 18
<212> DNA
<213> Artificial Sequence

<220>
<223> Detection oligonucleotide for FOS

<400> 677

tttcgtggtt ttatcgta 18

<210> 678
<211> 18
<212> DNA
<213> Artificial Sequence

<220>
<223> Detection oligonucleotide for FOS

<400> 678

ttttgtggtt ttattgta 18

<210> 679
<211> 16
<212> DNA
<213> Artificial Sequence

<220>
<223> Detection oligonucleotide for FOS

<400> 679

gtcgagcgta gagtat 16

<210> 680
<211> 16
<212> DNA
<213> Artificial Sequence

<220>
<223> Detection oligonucleotide for FOS

<400> 680

taggaggttg agtgta 16

<210> 681
<211> 16
<212> DNA
<213> Artificial Sequence

<220>
<223> Detection oligonucleotide for GSTP1

<400> 681

gtcggtcgta gagggg 16

<210> 682
<211> 16
<212> DNA
<213> Artificial Sequence

<220>
<223> Detection oligonucleotide for GSTP1

<400> 682

gttggttgta gagggg 16

<210> 683
<211> 16
<212> DNA
<213> Artificial Sequence

<220>
<223> Detection oligonucleotide for GSTP1

<400> 683

ttcgcggttt tcgagt 16

<210> 684
<211> 17
<212> DNA
<213> Artificial Sequence

<220>

<223> Detection oligonucleotide for GSTP1

<400> 684

tttgtggttt ttgagtt 17

<210> 685
<211> 16
<212> DNA
<213> Artificial Sequence

<220>
<223> Detection oligonucleotide for GSTP1

<400> 685

gtcgcgcgta tttatt 16

<210> 686
<211> 17
<212> DNA
<213> Artificial Sequence

<220>
<223> Detection oligonucleotide for GSTP1

<400> 686

gggttgtgtg tatttat 17

<210> 687
<211> 16
<212> DNA
<213> Artificial Sequence

<220>
<223> Detection oligonucleotide for GSTP1

<400> 687

gagtcgtcgc gtagtt 16

<210> 688
<211> 17
<212> DNA

<213> Artificial Sequence

<220>
<223> Detection oligonucleotide for GSTP1

<400> 688

ggagttgttg tgtagtt 17

<210> 689
<211> 16
<212> DNA
<213> Artificial Sequence

<220>
<223> Detection oligonucleotide for CD37

<400> 689

atcgagagcg ttatga 16

<210> 690
<211> 17
<212> DNA
<213> Artificial Sequence

<220>
<223> Detection oligonucleotide for CD37

<400> 690

aggaatattg agagtgt 17

<210> 691
<211> 16
<212> DNA
<213> Artificial Sequence

<220>
<223> Detection oligonucleotide for CD37

<400> 691

tatcgagcga gtcggt 16

<210> 692
<211> 17
<212> DNA
<213> Artificial Sequence

<220>
<223> Detection oligonucleotide for CD37

<400> 692

tattgagtga gttggtt 17

<210> 693
<211> 16
<212> DNA
<213> Artificial Sequence

<220>
<223> Detection oligonucleotide for CD37

<400> 693

ttagcgtacg tgacgg 16

<210> 694
<211> 16
<212> DNA
<213> Artificial Sequence

<220>
<223> Detection oligonucleotide for CD37

<400> 694

agtgtatgtg atgggg 16

<210> 695
<211> 16
<212> DNA
<213> Artificial Sequence

<220>
<223> Detection oligonucleotide for CD37

<400> 695

gggtacgtag ttacgt 16

<210> 696
<211> 17
<212> DNA
<213> Artificial Sequence

<220>
<223> Detection oligonucleotide for CD37

<400> 696

tatgtagtta tgtgggt 17

<210> 697
<211> 16
<212> DNA
<213> Artificial Sequence

<220>
<223> Detection oligonucleotide for GRN

<400> 697

tagcgcgatg attcgt 16

<210> 698

<211> 17
<212> DNA
<213> Artificial Sequence

<220>
<223> Detection oligonucleotide for GRN

<400> 698

agtgtgatga tttgttt 17

<210> 699
<211> 16
<212> DNA
<213> Artificial Sequence

<220>
<223> Detection oligonucleotide for GRN

<400> 699

taatcgggta gcgttt 16

<210> 700
<211> 17
<212> DNA
<213> Artificial Sequence

<220>
<223> Detection oligonucleotide for GRN

<400> 700

tagtaattgg gtagtgt 17

<210> 701
<211> 16
<212> DNA
<213> Artificial Sequence

<220>
<223> Detection oligonucleotide for GRN

<400> 701

ttcgatttcg cggttt 16

<210> 702
<211> 17

<212> DNA
<213> Artificial Sequence

<220>
<223> Detection oligonucleotide for GRN

<400> 702

gtttgatttt gtggttt 17

<210> 703
<211> 16
<212> DNA
<213> Artificial Sequence

<220>
<223> Detection oligonucleotide for GRN

<400> 703

tttaacgggg cgttat 16

<210> 704
<211> 16
<212> DNA
<213> Artificial Sequence

<220>
<223> Detection oligonucleotide for GRN

<400> 704

aatggggtgt tattgt 16

<210> 705
<211> 16
<212> DNA
<213> Artificial Sequence

<220>
<223> Detection oligonucleotide for EPAS1

<400> 705

ttcggcgtta ttcgag 16

<210> 706
<211> 17
<212> DNA
<213> Artificial Sequence

<220>
<223> Detection oligonucleotide for EPAS1

<400> 706

ggtttggtgt tatttga 17

<210> 707
<211> 16
<212> DNA
<213> Artificial Sequence

<220>
<223> Detection oligonucleotide for EPAS1

<400> 707

tattcgtgcg gtttta 16

<210> 708
<211> 17
<212> DNA
<213> Artificial Sequence

<220>
<223> Detection oligonucleotide for EPAS1

<400> 708

atttgtgtgg ttttagt 17

<210> 709
<211> 16
<212> DNA
<213> Artificial Sequence

<220>
<223> Detection oligonucleotide for EPAS1

<400> 709

gaatttaacg cgcggt 16

<210> 710
<211> 17
<212> DNA
<213> Artificial Sequence

<220>
<223> Detection oligonucleotide for EPAS1

<400> 710

ggaatttaat gtgtggt 17

<210> 711
<211> 16
<212> DNA
<213> Artificial Sequence

<220>
<223> Detection oligonucleotide for EPAS1

<400> 711

ttcgcgagtt tttcgg 16

<210> 712
<211> 18
<212> DNA
<213> Artificial Sequence

<220>
<223> Detection oligonucleotide for EPAS1

<400> 712

tttgtgagtt ttttggta 18

<210> 713
<211> 16
<212> DNA
<213> Artificial Sequence

<220>
<223> Detection oligonucleotide for NOTCH1

<400> 713

tttacgggcg ggagtt 16

<210> 714
<211> 16
<212> DNA
<213> Artificial Sequence

<220>
<223> Detection oligonucleotide for NOTCH1

<400> 714

ttttatgggt gggagt 16

<210> 715
<211> 16
<212> DNA
<213> Artificial Sequence

<220>
<223> Detection oligonucleotide for NOTCH1

<400> 715

tagcgggcga gtagtt 16

<210> 716
<211> 16
<212> DNA

<213> Artificial Sequence

<220>
<223> Detection oligonucleotide for NOTCH1

<400> 716

tagtgggtga gtagtt 16

<210> 717
<211> 16
<212> DNA
<213> Artificial Sequence

<220>
<223> Detection oligonucleotide for NOTCH1

<400> 717

aggcggtttc gatttt 16

<210> 718
<211> 16
<212> DNA
<213> Artificial Sequence

<220>
<223> Detection oligonucleotide for NOTCH1

<400> 718

tggaggtggt tttgat 16

<210> 719
<211> 16
<212> DNA
<213> Artificial Sequence

<220>
<223> Detection oligonucleotide for NOTCH1

<400> 719

atttcggcgg ttggat 16

<210> 720
<211> 17
<212> DNA
<213> Artificial Sequence

<220>
<223> Detection oligonucleotide for NOTCH1

<400> 720

aggtaatttt ggtggtt 17

<210> 721
<211> 20
<212> DNA
<213> Artificial Sequence

<220>
<223> Detection oligonucleotide for MLLT3

<400> 721

taattcggtt agattttcgg 20

<210> 722
<211> 20
<212> DNA
<213> Artificial Sequence

<220>
<223> Detection oligonucleotide for MLLT3

<400> 722

taatttggtt agatttttgg 20

<210> 723
<211> 16
<212> DNA
<213> Artificial Sequence

<220>
<223> Detection oligonucleotide for MLLT3

<400> 723

tttagagtcg cgtttt 16

<210> 724
<211> 17
<212> DNA
<213> Artificial Sequence

<220>
<223> Detection oligonucleotide for MLLT3

<400> 724

tagagttgtg tttttgt 17

<210> 725
<211> 16
<212> DNA
<213> Artificial Sequence

<220>
<223> Detection oligonucleotide for MLLT3

<400> 725

tagaatagcg cggtta 16

<210> 726
<211> 18
<212> DNA
<213> Artificial Sequence

<220>
<223> Detection oligonucleotide for MLLT3

<400> 726

gatagaatag tgtggtta 18

<210> 727
<211> 16
<212> DNA
<213> Artificial Sequence

<220>
<223> Detection oligonucleotide for SOLH

<400> 727

taggggacgt gtacga 16

<210> 728
<211> 16
<212> DNA
<213> Artificial Sequence

<220>
<223> Detection oligonucleotide for SOLH

<400> 728

taggggatgt gtatga 16

<210> 729
<211> 16
<212> DNA
<213> Artificial Sequence

<220>
<223> Detection oligonucleotide for SOLH

<400> 729

gacggaacgt atgttt 16

<210> 730
<211> 16
<212> DNA
<213> Artificial Sequence

<220>
<223> Detection oligonucleotide for SOLH

<400> 730

tggggatgga atgtat 16

<210> 731
<211> 16
<212> DNA
<213> Artificial Sequence

<220>
<223> Detection oligonucleotide for SOLH

<400> 731

attcgtgggg acggaa 16

<210> 732
<211> 16
<212> DNA
<213> Artificial Sequence

<220>
<223> Detection oligonucleotide for SOLH

<400> 732

atttgtgggg atggaa 16

<210> 733
<211> 17
<212> DNA
<213> Artificial Sequence

<220>
<223> Detection oligonucleotide for SEQ ID NO: 44

<400> 733

tagggttcgt tcgtatt 17

<210> 734
<211> 18
<212> DNA
<213> Artificial Sequence

<220>
<223> Detection oligonucleotide for SEQ ID NO: 44

<400> 734

tttagggttt gtttgtat 18

<210> 735
<211> 16
<212> DNA
<213> Artificial Sequence

<220>
<223> Detection oligonucleotide for SEQ ID NO: 44

<400> 735

ttacgatttt cggggt 16

<210> 736
<211> 17
<212> DNA
<213> Artificial Sequence

<220>
<223> Detection oligonucleotide for SEQ ID NO: 44

<400> 736

tttatgattt ttggggt 17

<210> 737
<211> 16
<212> DNA
<213> Artificial Sequence

<220>
<223> Detection oligonucleotide for SEQ ID NO: 44

<400> 737

aagtagacgt cggaga 16

<210> 738
<211> 16
<212> DNA
<213> Artificial Sequence

<220>
<223> Detection oligonucleotide for SEQ ID NO: 44

<400> 738

tagatgttgg agaggg 16

<210> 739
<211> 16
<212> DNA
<213> Artificial Sequence

<220>
<223> Detection oligonucleotide for SEQ ID NO: 44

<400> 739

tgtcgttacg tttagg 16

<210> 740
<211> 17
<212> DNA
<213> Artificial Sequence

<220>
<223> Detection oligonucleotide for SEQ ID NO: 44

<400> 740

gttgttatgt ttagggt 17

<210> 741
<211> 16
<212> DNA
<213> Artificial Sequence

<220>
<223> Detection oligonucleotide for Q8N365

<400> 741

agattcggag agacgg 16

<210> 742
<211> 17
<212> DNA
<213> Artificial Sequence

<220>
<223> Detection oligonucleotide for Q8N365

<400> 742

tagatttgga gagatgg 17

<210> 743
<211> 16
<212> DNA
<213> Artificial Sequence

<220>
<223> Detection oligonucleotide for Q8N365

<400> 743

agatcggcgt agggat 16

<210> 744
<211> 16
<212> DNA
<213> Artificial Sequence

<220>
<223> Detection oligonucleotide for Q8N365

<400> 744

agattggtgt agggat 16

<210> 745
<211> 16
<212> DNA
<213> Artificial Sequence

<220>
<223> Detection oligonucleotide for Q8N365

<400> 745

tacgtgttat cggcga 16

<210> 746
<211> 18
<212> DNA
<213> Artificial Sequence

<220>
<223> Detection oligonucleotide for Q8N365

<400> 746

tatgtgttat tggtgata 18

<210> 747
<211> 16
<212> DNA
<213> Artificial Sequence

<220>
<223> Detection oligonucleotide for Q8N365

<400> 747

tacgtgtcgg gtcgta 16

<210> 748
<211> 17
<212> DNA
<213> Artificial Sequence

<220>
<223> Detection oligonucleotide for Q8N365

<400> 748

gtatgtgttg ggttgta 17

<210> 749
<211> 16
<212> DNA
<213> Artificial Sequence

<220>
<223> Detection oligonucleotide for Q9NWV0

<400> 749

gtcgcgtgga tacgtg 16

<210> 750
<211> 16
<212> DNA
<213> Artificial Sequence

<220>
<223> Detection oligonucleotide for Q9NWV0

<400> 750

ggttgtgtgg atatgt 16

<210> 751
<211> 16
<212> DNA
<213> Artificial Sequence

<220>
<223> Detection oligonucleotide for Q9NWV0

<400> 751

ttggcgattt ttacga 16

<210> 752
<211> 18
<212> DNA
<213> Artificial Sequence

<220>
<223> Detection oligonucleotide for Q9NWV0

<400> 752

ttggtgattt ttatgaga 18

<210> 753
<211> 16
<212> DNA
<213> Artificial Sequence

<220>
<223> Detection oligonucleotide for Q9NWV0

<400> 753

tgtcgtagcg ttatgt 16

<210> 754
<211> 17
<212> DNA
<213> Artificial Sequence

<220>
<223> Detection oligonucleotide for Q9NWV0

<400> 754

aggtgttgta gtgttat 17

<210> 755
<211> 16
<212> DNA
<213> Artificial Sequence

<220>
<223> Detection oligonucleotide for SEQ ID NO: 47

<400> 755

gtaacgcgtt tggttt 16

<210> 756
<211> 16

<212> DNA
<213> Artificial Sequence

<220>
<223> Detection oligonucleotide for SEQ ID NO: 47

<400> 756

tggtaatgtg tttggt 16

<210> 757
<211> 16
<212> DNA
<213> Artificial Sequence

<220>
<223> Detection oligonucleotide for SEQ ID NO: 47

<400> 757

ttcgagcgtt ttacgt 16

<210> 758
<211> 18
<212> DNA
<213> Artificial Sequence

<220>
<223> Detection oligonucleotide for SEQ ID NO: 47

<400> 758

ttttgagtgt tttatgtt 18

<210> 759
<211> 16
<212> DNA
<213> Artificial Sequence

<220>
<223> Detection oligonucleotide for SEQ ID NO: 47

<400> 759

ttacgtggga gcgttt 16

<210> 760
<211> 16
<212> DNA
<213> Artificial Sequence

<220>
<223> Detection oligonucleotide for SEQ ID NO: 47

<400> 760

ttatgtggga gtgttt 16

<210> 761
<211> 16
<212> DNA
<213> Artificial Sequence

<220>
<223> Detection oligonucleotide for SEQ ID NO: 47

<400> 761

tagttttacg cgggta 16

<210> 762
<211> 17
<212> DNA
<213> Artificial Sequence

<220>
<223> Detection oligonucleotide for SEQ ID NO: 47

<400> 762

agttttatgt gggtatg 17

<210> 763
<211> 16
<212> DNA
<213> Artificial Sequence

<220>
<223> Detection oligonucleotide for H2AFY2

<400> 763

atgaaaggcg cgagaa 16

<210> 764
<211> 16
<212> DNA
<213> Artificial Sequence

<220>
<223> Detection oligonucleotide for H2AFY2

<400> 764

atgaaaggtg tgagaa 16

<210> 765
<211> 16
<212> DNA
<213> Artificial Sequence

<220>
<223> Detection oligonucleotide for H2AFY2

<400> 765

gaatcgtggt ttcgtt 16

<210> 766
<211> 16
<212> DNA
<213> Artificial Sequence

<220>
<223> Detection oligonucleotide for H2AFY2

<400> 766

ggaattgtgg ttttgt 16

<210> 767
<211> 16
<212> DNA
<213> Artificial Sequence

<220>
<223> Detection oligonucleotide for H2AFY2

<400> 767

tagtttatcg cggtaa 16

<210> 768
<211> 18
<212> DNA
<213> Artificial Sequence

<220>
<223> Detection oligonucleotide for H2AFY2

<400> 768

tttattgtgg taaatggt 18

<210> 769
<211> 16
<212> DNA
<213> Artificial Sequence

<220>
<223> Detection oligonucleotide for RHOC

<400> 769

tgcggttcga agatta 16

<210> 770
<211> 16
<212> DNA
<213> Artificial Sequence

<220>
<223> Detection oligonucleotide for RHOC

<400> 770

ggtgtggttt gaagat 16

<210> 771
<211> 16
<212> DNA
<213> Artificial Sequence

<220>
<223> Detection oligonucleotide for RHOC

<400> 771

gaacgcgttt tagcgt 16

<210> 772
<211> 17
<212> DNA
<213> Artificial Sequence

<220>
<223> Detection oligonucleotide for RHOC

<400> 772

ggaatgtgtt ttagtgt 17

<210> 773
<211> 16
<212> DNA
<213> Artificial Sequence

<220>
<223> Detection oligonucleotide for RHOC

<400> 773

ttttagcgtc ggggat 16

<210> 774
<211> 16
<212> DNA
<213> Artificial Sequence

<220>
<223> Detection oligonucleotide for RHOC

<400> 774

ttttagtgtt ggggat 16

<210> 775
<211> 16
<212> DNA
<213> Artificial Sequence

<220>
<223> Detection oligonucleotide for NR2E1

<400> 775

tagtcgtatt agcggt 16

<210> 776
<211> 18
<212> DNA
<213> Artificial Sequence

<220>
<223> Detection oligonucleotide for NR2E1

<400> 776

tagttgtatt agtggttt 18

<210> 777
<211> 16
<212> DNA
<213> Artificial Sequence

<220>
<223> Detection oligonucleotide for NR2E1

<400> 777

tgcgttttat tcgcgg 16

<210> 778
<211> 17
<212> DNA
<213> Artificial Sequence

<220>
<223> Detection oligonucleotide for NR2E1

<400> 778

tgtgttttat ttgtggt 17

<210> 779
<211> 16
<212> DNA
<213> Artificial Sequence

<220>
<223> Detection oligonucleotide for NR2E1

<400> 779

tttcgaagtt tcgcgg 16

<210> 780
<211> 16
<212> DNA
<213> Artificial Sequence

<220>
<223> Detection oligonucleotide for NR2E1

<400> 780

tttgaagttt tgtggg 16

<210> 781
<211> 16
<212> DNA
<213> Artificial Sequence

<220>
<223> Detection oligonucleotide for NR2E1

<400> 781

tagcgcgaat cgttta 16

<210> 782
<211> 17
<212> DNA
<213> Artificial Sequence

<220>
<223> Detection oligonucleotide for NR2E1

<400> 782

agtgtgaatt gtttagt 17

<210> 783
<211> 16
<212> DNA
<213> Artificial Sequence

<220>
<223> Detection oligonucleotide for KBTBD6

<400> 783

agacgtttcg cgtttt 16

<210> 784
<211> 17
<212> DNA
<213> Artificial Sequence

<220>
<223> Detection oligonucleotide for KBTBD6

<400> 784

gaagatgttt tgtgttt 17

<210> 785
<211> 16
<212> DNA
<213> Artificial Sequence

<220>
<223> Detection oligonucleotide for KBTBD6

<400> 785

tttcgggaag acgttt 16

<210> 786
<211> 16
<212> DNA
<213> Artificial Sequence

<220>
<223> Detection oligonucleotide for KBTBD6

<400> 786

agttttggga agatgt 16

<210> 787
<211> 16
<212> DNA
<213> Artificial Sequence

<220>
<223> Detection oligonucleotide for KBTBD6

<400> 787

tattagcgtt cgtcgt 16

<210> 788
<211> 18
<212> DNA
<213> Artificial Sequence

<220>
<223> Detection oligonucleotide for KBTBD6

<400> 788

gttattagtg tttgttgt 18

<210> 789
<211> 16
<212> DNA
<213> Artificial Sequence

<220>
<223> Detection oligonucleotide for TRPM4

<400> 789

taggtgagcg tcggat 16

<210> 790
<211> 16
<212> DNA
<213> Artificial Sequence

<220>
<223> Detection oligonucleotide for TRPM4

<400> 790

taggtgagtg ttggat 16

<210> 791

<211> 16
<212> DNA
<213> Artificial Sequence

<220>
<223> Detection oligonucleotide for TRPM4

<400> 791

agtagagtcg gcggag 16

<210> 792
<211> 16
<212> DNA
<213> Artificial Sequence

<220>
<223> Detection oligonucleotide for TRPM4

<400> 792

agtagagttg gtggag 16

<210> 793
<211> 16
<212> DNA
<213> Artificial Sequence

<220>
<223> Detection oligonucleotide for TCEB3BP1

<400> 793

gacgttagcg agtatt 16

<210> 794
<211> 16
<212> DNA
<213> Artificial Sequence

<220>
<223> Detection oligonucleotide for TCEB3BP1

<400> 794

agggatgtta gtgagt 16

<210> 795
<211> 16
<212> DNA
<213> Artificial Sequence

<220>
<223> Detection oligonucleotide for TCEB3BP1

<400> 795

tgtcgggtcg agtagt 16

<210> 796
<211> 16
<212> DNA
<213> Artificial Sequence

<220>
<223> Detection oligonucleotide for TCEB3BP1

<400> 796

tgttgggttg agtagt 16

<210> 797
<211> 16
<212> DNA
<213> Artificial Sequence

<220>
<223> Detection oligonucleotide for Q8NCX8

<400> 797

ttacggcggg ttttta 16

<210> 798
<211> 18
<212> DNA
<213> Artificial Sequence

<220>
<223> Detection oligonucleotide for Q8NCX8

<400> 798

ttatggtggg tttttatt 18

<210> 799
<211> 17
<212> DNA
<213> Artificial Sequence

<220>
<223> Detection oligonucleotide for Q8NCX8

<400> 799

ttcggtttat acgattt 17

<210> 800
<211> 20
<212> DNA
<213> Artificial Sequence

<220>
<223> Detection oligonucleotide for Q8NCX8

<400> 800

atttggttta tatgattttt 20

<210> 801
<211> 16
<212> DNA
<213> Artificial Sequence

<220>
<223> Detection oligonucleotide for Q8NCX8

<400> 801

ttacgtcgat tatcgg 16

<210> 802
<211> 18
<212> DNA
<213> Artificial Sequence

<220>
<223> Detection oligonucleotide for Q8NCX8

<400> 802

tatgttgatt attggggt 18

<210> 803
<211> 16
<212> DNA
<213> Artificial Sequence

<220>
<223> Detection oligonucleotide for SEQ ID NO: 55

<400> 803

aatttacgcg ggtgtt 16

<210> 804
<211> 16
<212> DNA
<213> Artificial Sequence

<220>
<223> Detection oligonucleotide for SEQ ID NO: 55

<400> 804

ggaatttatg tgggtg 16

<210> 805

<211> 16
<212> DNA
<213> Artificial Sequence

<220>
<223> Detection oligonucleotide for SEQ ID NO: 55

<400> 805

tagttcgttt cggtta 16

<210> 806
<211> 17
<212> DNA
<213> Artificial Sequence

<220>
<223> Detection oligonucleotide for SEQ ID NO: 55

<400> 806

agtttgtttt ggttagt 17

<210> 807
<211> 17
<212> DNA
<213> Artificial Sequence

<220>
<223> Detection oligonucleotide for SEQ ID NO: 55

<400> 807

gtcgtgtacg aattcgt 17

<210> 808
<211> 18
<212> DNA
<213> Artificial Sequence

<220>
<223> Detection oligonucleotide for SEQ ID NO: 55

<400> 808

gttgtgtatg aatttgtt 18

<210> 809
<211> 16
<212> DNA
<213> Artificial Sequence

<220>
<223> Detection oligonucleotide for SEQ ID NO: 55

<400> 809

agcgtttaag tcgcgg 16

<210> 810
<211> 17
<212> DNA
<213> Artificial Sequence

<220>
<223> Detection oligonucleotide for SEQ ID NO: 55

<400> 810

tagtgtttaa gttgtgg 17

<210> 811
<211> 16
<212> DNA
<213> Artificial Sequence

<220>
<223> Detection oligonucleotide for SNAPC2

<400> 811

ttacgatttc ggtgtt 16

<210> 812
<211> 18
<212> DNA
<213> Artificial Sequence

<220>
<223> Detection oligonucleotide for SNAPC2

<400> 812

tagagttatg attttggt 18

<210> 813
<211> 16
<212> DNA
<213> Artificial Sequence

<220>
<223> Detection oligonucleotide for SNAPC2

<400> 813

aggcgtgcgt cgatat 16

<210> 814
<211> 17
<212> DNA
<213> Artificial Sequence

<220>

<223> Detection oligonucleotide for SNAPC2

<400> 814

aggtgtgtgt tgatatt 17

<210> 815
<211> 16
<212> DNA
<213> Artificial Sequence

<220>
<223> Detection oligonucleotide for SNAPC2

<400> 815

gtagcgtcga gggttt 16

<210> 816
<211> 16
<212> DNA
<213> Artificial Sequence

<220>
<223> Detection oligonucleotide for SNAPC2

<400> 816

gtagtgttga gggttt 16

<210> 817
<211> 16
<212> DNA
<213> Artificial Sequence

<220>
<223> Detection oligonucleotide for SNAPC2

<400> 817

atacgttgac gtagtt 16

<210> 818
<211> 19
<212> DNA
<213> Artificial Sequence

<220>
<223> Detection oligonucleotide for SNAPC2

<400> 818

ttgtatatgt tgatgtagt 19

<210> 819
<211> 16

<212> DNA
<213> Artificial Sequence

<220>
<223> Detection oligonucleotide for PTPRN2

<400> 819

gacgtagttc gtacgt 16

<210> 820
<211> 17
<212> DNA
<213> Artificial Sequence

<220>
<223> Detection oligonucleotide for PTPRN2

<400> 820

ggatgtagtt tgtatgt 17

<210> 821
<211> 16
<212> DNA
<213> Artificial Sequence

<220>
<223> Detection oligonucleotide for PTPRN2

<400> 821

ttagtcgttt cgagat 16

<210> 822
<211> 18
<212> DNA
<213> Artificial Sequence

<220>
<223> Detection oligonucleotide for PTPRN2

<400> 822

gttttagttg ttttgaga 18

<210> 823
<211> 16
<212> DNA
<213> Artificial Sequence

<220>
<223> Detection oligonucleotide for PTPRN2

<400> 823

ttacgcgtat cgggat 16

<210> 824
<211> 18
<212> DNA
<213> Artificial Sequence

<220>
<223> Detection oligonucleotide for PTPRN2

<400> 824

tatgtgtatt gggattta 18

<210> 825
<211> 16
<212> DNA
<213> Artificial Sequence

<220>
<223> Detection oligonucleotide for PTPRN2

<400> 825

ttcgcgtttc gtaaga 16

<210> 826
<211> 19
<212> DNA
<213> Artificial Sequence

<220>
<223> Detection oligonucleotide for PTPRN2

<400> 826

tttgtgtttt gtaagatat 19

<210> 827
<211> 16
<212> DNA
<213> Artificial Sequence

<220>
<223> Detection oligonucleotide for WDFY3

<400> 827

tattgagtcg gtcgta 16

<210> 828
<211> 17
<212> DNA
<213> Artificial Sequence

<220>
<223> Detection oligonucleotide for WDFY3

<400> 828

attgagttgg ttgtaga 17

<210> 829
<211> 16
<212> DNA
<213> Artificial Sequence

<220>
<223> Detection oligonucleotide for WDFY3

<400> 829

tagatagcgt cgttgg 16

<210> 830
<211> 17
<212> DNA
<213> Artificial Sequence

<220>
<223> Detection oligonucleotide for WDFY3

<400> 830

tagatagtgt tgttgga 17

<210> 831
<211> 17
<212> DNA
<213> Artificial Sequence

<220>
<223> Detection oligonucleotide for ZNF566

<400> 831

ataaaatacg acgttga 17

<210> 832
<211> 20
<212> DNA
<213> Artificial Sequence

<220>
<223> Detection oligonucleotide for ZNF566

<400> 832

ataaaatatg atgttgaatt 20

<210> 833
<211> 16
<212> DNA

<213> Artificial Sequence

<220>
<223> Detection oligonucleotide for ZNF566

<400> 833

agcgtttttt acgaat 16

<210> 834
<211> 19
<212> DNA
<213> Artificial Sequence

<220>
<223> Detection oligonucleotide for ZNF566

<400> 834

tagtgttttt tatgaatga 19

<210> 835
<211> 17
<212> DNA
<213> Artificial Sequence

<220>
<223> Detection oligonucleotide for Q9NP73

<400> 835

tattacggat tttcgtt 17

<210> 836
<211> 19
<212> DNA
<213> Artificial Sequence

<220>
<223> Detection oligonucleotide for Q9NP73

<400> 836

gttattatgg atttttgtt 19

<210> 837
<211> 16
<212> DNA
<213> Artificial Sequence

<220>
<223> Detection oligonucleotide for Q9NP73

<400> 837

tgcgttatat tcggtt 16

<210> 838
<211> 18
<212> DNA
<213> Artificial Sequence

<220>
<223> Detection oligonucleotide for Q9NP73

<400> 838

agttgtgtta tatttggt 18

<210> 839
<211> 16
<212> DNA
<213> Artificial Sequence

<220>
<223> Detection oligonucleotide for Q9NP73

<400> 839

aattcgcgtt atgaag 16

<210> 840
<211> 18
<212> DNA
<213> Artificial Sequence

<220>
<223> Detection oligonucleotide for Q9NP73

<400> 840

ttgaggaatt tgtgttat 18

<210> 841
<211> 16
<212> DNA
<213> Artificial Sequence

<220>
<223> Detection oligonucleotide for Q9NP73

<400> 841

gtcggcgttc gatagt 16

<210> 842
<211> 17
<212> DNA
<213> Artificial Sequence

<220>
<223> Detection oligonucleotide for Q9NP73

<400> 842

tgttggtgtt tgatagt 17

<210> 843
<211> 16
<212> DNA
<213> Artificial Sequence

<220>
<223> Detection oligonucleotide for SEQ ID NO: 61

<400> 843

agaattcgta aacggg 16

<210> 844
<211> 16
<212> DNA
<213> Artificial Sequence

<220>
<223> Detection oligonucleotide for SEQ ID NO: 61

<400> 844

atttgtaaat gggggt 16

<210> 845
<211> 16
<212> DNA
<213> Artificial Sequence

<220>
<223> Detection oligonucleotide for SEQ ID NO: 61

<400> 845

ttcggttatc gacggt 16

<210> 846
<211> 17
<212> DNA
<213> Artificial Sequence

<220>
<223> Detection oligonucleotide for SEQ ID NO: 61

<400> 846

tttggttatt gatggtt 17

<210> 847
<211> 16
<212> DNA
<213> Artificial Sequence

<220>
<223> Detection oligonucleotide for Q86SP6

<400> 847

atcgtgagcg tagcgt 16

<210> 848
<211> 17
<212> DNA
<213> Artificial Sequence

<220>
<223> Detection oligonucleotide for Q86SP6

<400> 848

attgtgagtg tagtgta 17

<210> 849
<211> 16
<212> DNA
<213> Artificial Sequence

<220>
<223> Detection oligonucleotide for Q86SP6

<400> 849

taggcgtgag aatcgg 16

<210> 850
<211> 16
<212> DNA
<213> Artificial Sequence

<220>
<223> Detection oligonucleotide for Q86SP6

<400> 850

taggtgtgag aattgg 16

<210> 851
<211> 16
<212> DNA
<213> Artificial Sequence

<220>
<223> Detection oligonucleotide for Q86SP6

<400> 851

atcgtgttcg gatcgg 16

<210> 852
<211> 17
<212> DNA
<213> Artificial Sequence

<220>
<223> Detection oligonucleotide for Q86SP6

<400> 852

tattgtgttt ggattgg 17

<210> 853
<211> 16
<212> DNA
<213> Artificial Sequence

<220>
<223> Detection oligonucleotide for Q86SP6

<400> 853

aggttcgagt ttgcgg 16

<210> 854
<211> 17
<212> DNA
<213> Artificial Sequence

<220>
<223> Detection oligonucleotide for Q86SP6

<400> 854

taggtttgag tttgtgg 17

<210> 855
<211> 16
<212> DNA
<213> Artificial Sequence

<220>
<223> Detection oligonucleotide for Q86SP6

<400> 855

tataaagcgc gagtgt 16

<210> 856
<211> 17
<212> DNA
<213> Artificial Sequence

<220>
<223> Detection oligonucleotide for Q86SP6

<400> 856

tataaagtgt gagtgtg 17

<210> 857
<211> 16
<212> DNA
<213> Artificial Sequence

<220>
<223> Detection oligonucleotide for RARB

<400> 857

atgtcgagaa cgcgag 16

<210> 858
<211> 17
<212> DNA
<213> Artificial Sequence

<220>
<223> Detection oligonucleotide for RARB

<400> 858

ggatgttgag aatgtga 17

<210> 859
<211> 16
<212> DNA
<213> Artificial Sequence

<220>
<223> Detection oligonucleotide for RARB

<400> 859

agcgattcga gtaggg 16

<210> 860
<211> 16
<212> DNA
<213> Artificial Sequence

<220>
<223> Detection oligonucleotide for RARB

<400> 860

agtgatttga gtaggg 16

<210> 861
<211> 16
<212> DNA
<213> Artificial Sequence

<220>
<223> Detection oligonucleotide for RARB

<400> 861

tatcgtcggg gtagga 16

<210> 862
<211> 16
<212> DNA
<213> Artificial Sequence

<220>
<223> Detection oligonucleotide for RARB

<400> 862

tattgttggg gtagga 16

<210> 863
<211> 16
<212> DNA
<213> Artificial Sequence

<220>
<223> Detection oligonucleotide for RARB

<400> 863

aacgtattcg gaaggt 16

<210> 864
<211> 17
<212> DNA
<213> Artificial Sequence

<220>
<223> Detection oligonucleotide for RARB

<400> 864

gaatgtattt ggaaggt 17

<210> 865
<211> 16
<212> DNA
<213> Artificial Sequence

<220>
<223> Detection oligonucleotide for PTGS2

<400> 865

agcgttttcg agagtt 16

<210> 866
<211> 17
<212> DNA
<213> Artificial Sequence

<220>
<223> Detection oligonucleotide for PTGS2

<400> 866

gagtgttttt gagagtt 17

<210> 867
<211> 16
<212> DNA
<213> Artificial Sequence

<220>
<223> Detection oligonucleotide for PTGS2

<400> 867

ttacgtcggg atagat 16

<210> 868
<211> 16
<212> DNA
<213> Artificial Sequence

<220>
<223> Detection oligonucleotide for PTGS2

<400> 868

ggaagttatg ttggga 16

<210> 869
<211> 16
<212> DNA
<213> Artificial Sequence

<220>
<223> Detection oligonucleotide for PTGS2

<400> 869

tatcgtttta ggcgta 16

<210> 870
<211> 19
<212> DNA
<213> Artificial Sequence

<220>
<223> Detection oligonucleotide for PTGS2

<400> 870

tttattgttt taggtgtat 19

<210> 871
<211> 17
<212> DNA
<213> Artificial Sequence

<220>
<223> Detection oligonucleotide for RASSF1

<400> 871

tacgggtatt ttcgcgt 17

<210> 872
<211> 18
<212> DNA
<213> Artificial Sequence

<220>
<223> Detection oligonucleotide for RASSF1

<400> 872

atatgggtat ttttgtgt 18

<210> 873
<211> 16
<212> DNA
<213> Artificial Sequence

<220>
<223> Detection oligonucleotide for RASSF1

<400> 873

agagcgcgtt tagttt 16

<210> 874
<211> 17
<212> DNA
<213> Artificial Sequence

<220>
<223> Detection oligonucleotide for RASSF1

<400> 874

gagagtgtgt ttagttt 17

<210> 875
<211> 16
<212> DNA
<213> Artificial Sequence

<220>
<223> Detection oligonucleotide for ESR2

<400> 875

taggaacgtt cgacgt 16

<210> 876
<211> 18
<212> DNA
<213> Artificial Sequence

<220>
<223> Detection oligonucleotide for ESR2

<400> 876

tttaggaatg tttgatgt 18

<210> 877
<211> 16
<212> DNA
<213> Artificial Sequence

<220>
<223> Detection oligonucleotide for ESR2

<400> 877

atggcgtttt tcgtag 16

<210> 878
<211> 17
<212> DNA
<213> Artificial Sequence

<220>
<223> Detection oligonucleotide for ESR2

<400> 878

tagatggtgt tttttgt 17

<210> 879
<211> 17
<212> DNA
<213> Artificial Sequence

<220>
<223> Detection oligonucleotide for ESR2

<400> 879

ataagcgatt taacgat 17

<210> 880

<211> 19
<212> DNA
<213> Artificial Sequence

<220>
<223> Detection oligonucleotide for ESR2

<400> 880

agtgatttaa tgataagtt 19

<210> 881
<211> 16
<212> DNA
<213> Artificial Sequence

<220>
<223> Detection oligonucleotide for ESR2

<400> 881

atttcgagga ttacgt 16

<210> 882
<211> 19
<212> DNA
<213> Artificial Sequence

<220>
<223> Detection oligonucleotide for ESR2

<400> 882

atattttgag gattatgtt 19

<210> 883
<211> 17
<212> DNA
<213> Artificial Sequence

<220>
<223> Detection oligonucleotide for DRG1

<400> 883

tagtcgttaa aacgtag 17

<210> 884
<211> 19
<212> DNA
<213> Artificial Sequence

<220>
<223> Detection oligonucleotide for DRG1

<400> 884

agttgttaaa atgtagatt 19

<210> 885
<211> 17
<212> DNA
<213> Artificial Sequence

<220>
<223> Detection oligonucleotide for DRG1

<400> 885

atcgtattgg ttcgcgg 17

<210> 886
<211> 17
<212> DNA
<213> Artificial Sequence

<220>
<223> Detection oligonucleotide for DRG1

<400> 886

attgtattgg tttgtgg 17

<210> 887
<211> 16
<212> DNA
<213> Artificial Sequence

<220>
<223> Detection oligonucleotide for DRG1

<400> 887

tttacgtttt gcgatt 16

<210> 888
<211> 18
<212> DNA
<213> Artificial Sequence

<220>
<223> Detection oligonucleotide for DRG1

<400> 888

agttttatgt tttgtgat 18

<210> 889
<211> 16
<212> DNA
<213> Artificial Sequence

<220>
<223> Detection oligonucleotide for DRG1

<400> 889

atttttacgc ggaatt 16

<210> 890
<211> 18
<212> DNA
<213> Artificial Sequence

<220>
<223> Detection oligonucleotide for DRG1

<400> 890

gtgattttta tgtggaat 18

<210> 891
<211> 16
<212> DNA
<213> Artificial Sequence

<220>
<223> Detection oligonucleotide for DRG1

<400> 891

attcgaagta tcgcgt 16

<210> 892
<211> 18
<212> DNA
<213> Artificial Sequence

<220>
<223> Detection oligonucleotide for DRG1

<400> 892

atttgaagta ttgtgttt 18

<210> 893
<211> 17
<212> DNA
<213> Artificial Sequence

<220>
<223> Detection oligonucleotide for DRG1

<400> 893

atttcgaatt tcgagta 17

<210> 894
<211> 18
<212> DNA
<213> Artificial Sequence

<220>
<223> Detection oligonucleotide for DRG1

<400> 894

tttgaatttt gagtagag 18

<210> 895
<211> 16
<212> DNA
<213> Artificial Sequence

<220>
<223> Detection oligonucleotide for DOCK10

<400> 895

tatattgtcg gggaga 16

<210> 896
<211> 16
<212> DNA
<213> Artificial Sequence

<220>
<223> Detection oligonucleotide for DOCK10

<400> 896

atattgttgg ggagag 16

<210> 897
<211> 16
<212> DNA
<213> Artificial Sequence

<220>
<223> Detection oligonucleotide for BTG4

<400> 897

gtgttgcgta gagata 16

<210> 898
<211> 16
<212> DNA
<213> Artificial Sequence

<220>
<223> Detection oligonucleotide for BTG4

<400> 898

ggtgttgtgt agagat 16

<210> 899
<211> 17
<212> DNA
<213> Artificial Sequence

<220>

<223> Detection oligonucleotide for BTG4

<400> 899

ttggtttttc ggaataa 17

<210> 900
<211> 18
<212> DNA
<213> Artificial Sequence

<220>
<223> Detection oligonucleotide for BTG4

<400> 900

ggtttttgg aataagat 18

<210> 901
<211> 16
<212> DNA
<213> Artificial Sequence

<220>
<223> Detection oligonucleotide for GPR7

<400> 901

attgagggcg tataga 16

<210> 902
<211> 17
<212> DNA
<213> Artificial Sequence

<220>
<223> Detection oligonucleotide for GPR7

<400> 902

tgagggtgta tagattt 17

<210> 903
<211> 16
<212> DNA
<213> Artificial Sequence

<220>
<223> Detection oligonucleotide for GPR7

<400> 903

ggagatcgaa gtttgt 16

<210> 904
<211> 16
<212> DNA
<213> Artificial Sequence

<220>
<223> Detection oligonucleotide for GPR7

<400> 904

ggggagattg aagttt 16

<210> 905
<211> 17
<212> DNA
<213> Artificial Sequence

<220>
<223> Detection oligonucleotide for ISL1

<400> 905

agattttgcg aaagata 17

<210> 906
<211> 19
<212> DNA
<213> Artificial Sequence

<220>
<223> Detection oligonucleotide for ISL1

<400> 906

ttagattttg tgaaagata 19

<210> 907
<211> 19
<212> DNA
<213> Artificial Sequence

<220>
<223> Detection oligonucleotide for ISL1

<400> 907

aagatatcga aattaagtt 19

<210> 908
<211> 20
<212> DNA
<213> Artificial Sequence

<220>
<223> Detection oligonucleotide for ISL1

<400> 908

aagatattga aattaagttt 20

<210> 909
<211> 16
<212> DNA
<213> Artificial Sequence

<220>
<223> Detection oligonucleotide for GPRK5

<400> 909

agattttcga gggaga 16

<210> 910
<211> 17
<212> DNA
<213> Artificial Sequence

<220>
<223> Detection oligonucleotide for GPRK5

<400> 910

agatttttga gggagat 17

<210> 911
<211> 17
<212> DNA
<213> Artificial Sequence

<220>
<223> Detection oligonucleotide for FBN2

<400> 911

aattggtcgt tagtttt 17

<210> 912
<211> 18
<212> DNA
<213> Artificial Sequence

<220>
<223> Detection oligonucleotide for FBN2

<400> 912

gttaattggt tgttagtt 18

<210> 913
<211> 16
<212> DNA
<213> Artificial Sequence

<220>
<223> Detection oligonucleotide for FBN2

<400> 913

ttagggatcg gatttg 16

<210> 914
<211> 17
<212> DNA
<213> Artificial Sequence

<220>
<223> Detection oligonucleotide for FBN2

<400> 914

attagggatt ggatttg 17

<210> 915
<211> 17
<212> DNA
<213> Artificial Sequence

<220>
<223> Detection oligonucleotide for LIMK1

<400> 915

tttaattcga aagggaa 17

<210> 916
<211> 18
<212> DNA
<213> Artificial Sequence

<220>
<223> Detection oligonucleotide for LIMK1

<400> 916

ttaatttgaa agggaaag 18

<210> 917
<211> 16
<212> DNA
<213> Artificial Sequence

<220>
<223> Detection oligonucleotide for PSD/Q9H469

<400> 917

aggttagtcg agaagt 16

<210> 918
<211> 17
<212> DNA
<213> Artificial Sequence

<220>
<223> Detection oligonucleotide for PSD/Q9H469

<400> 918

aggttagttg agaagta 17

<210> 919
<211> 16
<212> DNA
<213> Artificial Sequence

<220>
<223> Detection oligonucleotide for PSD/Q9H469

<400> 919

attgttacga agtgga 16

<210> 920
<211> 17
<212> DNA
<213> Artificial Sequence

<220>
<223> Detection oligonucleotide for PSD/Q9H469

<400> 920

ttgttatgaa gtggaag 17

<210> 921
<211> 16
<212> DNA
<213> Artificial Sequence

<220>
<223> Detection oligonucleotide for Q96S01

<400> 921

tattgatcgg ttggag 16

<210> 922

<211> 17
<212> DNA
<213> Artificial Sequence

<220>
<223> Detection oligonucleotide for Q96S01

<400> 922

tattgattgg ttggagg 17

<210> 923
<211> 18
<212> DNA
<213> Artificial Sequence

<220>
<223> Detection oligonucleotide for ABHD9

<400> 923

agttagagcg tattattt 18

<210> 924
<211> 20
<212> DNA
<213> Artificial Sequence

<220>
<223> Detection oligonucleotide for ABHD9

<400> 924

aatagttaga gtgtattatt 20

<210> 925
<211> 16
<212> DNA
<213> Artificial Sequence

<220>
<223> Detection oligonucleotide for MLLT3

<400> 925

ttagtggtcg gagata 16

<210> 926
<211> 16
<212> DNA
<213> Artificial Sequence

<220>
<223> Detection oligonucleotide for MLLT3

<400> 926

agtggttgga gataga 16

<210> 927
<211> 17
<212> DNA
<213> Artificial Sequence

<220>
<223> Detection oligonucleotide for SOLH

<400> 927

tatatttcgt gagggta 17

<210> 928
<211> 18
<212> DNA
<213> Artificial Sequence

<220>
<223> Detection oligonucleotide for SOLH

<400> 928

tatattttgt gagggtag 18

<210> 929
<211> 17
<212> DNA
<213> Artificial Sequence

<220>
<223> Detection oligonucleotide for Q9NWV0

<400> 929

atttttacga gaaggtt 17

<210> 930
<211> 18
<212> DNA
<213> Artificial Sequence

<220>
<223> Detection oligonucleotide for Q9NWV0

<400> 930

gatttttatg agaaggtt 18

<210> 931
<211> 16
<212> DNA
<213> Artificial Sequence

<220>

<223> Detection oligonucleotide for H2AFY2

<400> 931

atgggaatcg tggttt 16

<210> 932
<211> 16
<212> DNA
<213> Artificial Sequence

<220>
<223> Detection oligonucleotide for H2AFY2

<400> 932

atgggaattg tggttt 16

<210> 933
<211> 16
<212> DNA
<213> Artificial Sequence

<220>
<223> Detection oligonucleotide for RHOC

<400> 933

aggtttacgg aaaagg 16

<210> 934
<211> 17
<212> DNA
<213> Artificial Sequence

<220>
<223> Detection oligonucleotide for RHOC

<400> 934

aaggtttatg gaaaagg 17

<210> 935
<211> 17
<212> DNA
<213> Artificial Sequence

<220>

<223> Detection oligonucleotide for KBTBD6

<400> 935

tagagtcggg ttttgta 17

<210> 936

<211> 17
<212> DNA
<213> Artificial Sequence

<220>
<223> Detection oligonucleotide for KBTBD6

<400> 936

tagagttggg ttttgta 17

<210> 937
<211> 16
<212> DNA
<213> Artificial Sequence

<220>
<223> Detection oligonucleotide for TRPM4

<400> 937

atgggggtcg agattt 16

<210> 938
<211> 16
<212> DNA
<213> Artificial Sequence

<220>
<223> Detection oligonucleotide for TRPM4

<400> 938

atgggggttg agattt 16

<210> 939

<211> 16
<212> DNA
<213> Artificial Sequence

<220>
<223> Detection oligonucleotide for TCEB3BP1

<400> 939

ggtagtcggt attagg 16

<210> 940
<211> 17
<212> DNA
<213> Artificial Sequence

<220>
<223> Detection oligonucleotide for TCEB3BP1

<400> 940

tggtagttgg tattagg 17

<210> 941
<211> 16
<212> DNA
<213> Artificial Sequence

<220>
<223> Detection oligonucleotide for TCEB3BP1

<400> 941

tgtagtcgaa ggttag 16

<210> 942
<211> 17
<212> DNA
<213> Artificial Sequence

<220>
<223> Detection oligonucleotide for TCEB3BP1

<400> 942

tgtagttgaa ggttagt 17

<210> 943
<211> 18
<212> DNA
<213> Artificial Sequence

<220>
<223> Detection oligonucleotide for Q8NCX8

<400> 943

ggagtttatt cggtttat 18

<210> 944
<211> 19
<212> DNA
<213> Artificial Sequence

<220>
<223> Detection oligonucleotide for Q8NCX8

<400> 944

ggagtttatt tggtttata 19

<210> 945
<211> 18
<212> DNA
<213> Artificial Sequence

<220>
<223> Detection oligonucleotide for WDFY3

<400> 945

aattgtagtc gtttagtt 18

<210> 946
<211> 19
<212> DNA
<213> Artificial Sequence

<220>
<223> Detection oligonucleotide for WDFY3

<400> 946

aaattgtagt tgtttagtt 19

<210> 947
<211> 18
<212> DNA
<213> Artificial Sequence

<220>
<223> Detection oligonucleotide for ZNF566

<400> 947

tattttttcg gtagagat 18

<210> 948
<211> 18
<212> DNA
<213> Artificial Sequence

<220>
<223> Detection oligonucleotide for ZNF566

<400> 948

ttttttggt agagattg 18

<210> 949
<211> 16
<212> DNA
<213> Artificial Sequence

<220>
<223> Detection oligonucleotide for ZNF566

<400> 949

atgggttgcg tttata 16

<210> 950

<211> 17
<212> DNA
<213> Artificial Sequence

<220>
<223> Detection oligonucleotide for ZNF566

<400> 950

tgggttgtgt ttataag 17

<210> 951
<211> 16
<212> DNA
<213> Artificial Sequence

<220>
<223> Detection oligonucleotide for SEQ ID NO: 61

<400> 951

tttagggcga ggttat 16

<210> 952
<211> 17
<212> DNA
<213> Artificial Sequence

<220>
<223> Detection oligonucleotide for SEQ ID NO: 61

<400> 952

tttagggtga ggttatt 17

<210> 953
<211> 17
<212> DNA
<213> Artificial Sequence

<220>
<223> Detection oligonucleotide for SEQ ID NO: 61

<400> 953

tgtatatttc gtagggt 17

<210> 954
<211> 18
<212> DNA
<213> Artificial Sequence

<220>
<223> Detection oligonucleotide for SEQ ID NO: 61

<400> 954

ttgtatattt tgtagggt 18

<210> 955
<211> 16
<212> DNA
<213> Artificial Sequence

<220>
<223> Detection oligonucleotide for PTGS2

<400> 955

atttgagcgg ttttga 16

<210> 956
<211> 17
<212> DNA
<213> Artificial Sequence

<220>
<223> Detection oligonucleotide for PTGS2

<400> 956

aatttgagtg gttttga 17

<210> 957
<211> 17
<212> DNA
<213> Artificial Sequence

<220>
<223> Detection oligonucleotide for RASSF1

<400> 957

agtaaatcgg attagga 17

<210> 958
<211> 18
<212> DNA
<213> Artificial Sequence

<220>
<223> Detection oligonucleotide for RASSF1

<400> 958

agtaaattgg attaggag 18

<210> 959
<211> 17
<212> DNA
<213> Artificial Sequence

<220>
<223> Detection oligonucleotide for DRG1

<400> 959

tgtatgaacg tgtagtt 17

<210> 960
<211> 17
<212> DNA
<213> Artificial Sequence

<220>
<223> Detection oligonucleotide for DRG1

<400> 960

gtgtatgaat gtgtagt 17

<210> 961
<211> 28536
<212> DNA
<213> Homo Sapiens

<400> 961

gctctgggag ctaggtttct ctcgctggat gggaaaagga ggtgcggaaa ggggcaggcg 60
gcctgcgggc tagcggggat gagagttgtg gggagaacgg aggagagagt aaaattattc 120
cttttggaag tccaagggaa gggagctgag gtgagagaaa tggcctctct taggtcccaa 180
gtctccgccc ccagcctgca ccactcaagt cggggaagtg acaactcttc ctaaacctcg 240
acttccattt tttgtttcct gcagattgat tgatgtcctc gcgttgttct agttcagcaa 300
ccaaactagg ccgaggtaca cctcttttac ccagagctcc caaatcactc accaacctgc 360
aggagaccta aatcctccgg gcaggtctcg ttagcgcttc cagcccccat ccccaaatcc 420
cctcttctag ctccttcctt cccctccgcc ctttggccct ccccgccccc taccaggctg 480
agaggcggag aggccgggac cgctgggccc tgcggctcca gaagccaaag tccgcccgtg 540
gggaccgttt tcctctccac tgctctcaag tgaacaacat ggtgggaggt gaccctcgtc 600
caaagggctt ttatctgccc cccactgctg cgtgatccct ccgcaagtcg gaggcagagc 660
tttaaaaaga aagaaagtgt gcgttgagca ctgatcttaa atcggggaca ttatctgcga 720
tctctttaag tggcagcagc agtttcttgc agaacaaaga ctcagtgttc gagctccaaa 780
agcttctagg taaagtttca ttcagatgaa agcaactaag gcgaaaaaac aatgatattg 840
tcggctccag aaaatcggcg gttacttttt gctctaagtt ccccagcgtg gtgtttgttc 900
tcgcccactt tggttgtgcg gggggttgac aagcataaca aaagatctca gcacatccct 960
ccccccacct ccacaacgac cctcctagcg ccatgaggat gaattctctg ggactaagtg 1020
gtatttgttt ctatttgtta tgaaatcaaa tttcacattt ttttaaagct gaaaatcgcg 1080
gataaagaag gatcgggtgc ttaaagttca tttaaacact cacaccgaac tcattccctg 1140
tttagatgtc agaggatggc agggagggcc agggctgtaa ttcatcttga tttgcttcat 1200
tgtcctgcag tttgcaaact ataatcctgt ataatttcag gaacaagcag gtttagaatt 1260
aatgggtcaa tattatttaa aacaaaacac cgggagcatg acctttgcct caactacata 1320
ttgctcgaca agactcagga aactccactc taacctctca acccctgagt tctttgagaa 1380
actgaagggc tgtagttatt agaaatcatc tttgtttctt ttaatgtctc caaaggattt 1440
ggaaatagta atgcaatata acatgaagga tctctctact taagcctgaa gataaacgtg 1500
gaagcctaaa tattttgaac tggagatgtt tccctgtaaa tttattatag atgtattcct 1560
cctgagagaa atgcatataa actatacatg tatacatgca caaatatttc tccagaacaa 1620
tttgctagag gtgtaggttt ccccgtaaag gagtaaactt gagagtcatt ttaagctgat 1680
ggacttgcta aatttctttc ttcttttttc tttttcatat tatttgctag ccataatgga 1740
atcctctagg tttaagccaa agaaaaattg gagagacaaa attagatttt gtagcccttt 1800
tcccccccgg gaatgccttt ttttttcttt ttagtttctg atgaatggct atcatttatt 1860
tctaccaaat ttaaataagg actgctgcct tgtatgttta actaggcagg cagagggaac 1920
tggtttgttt aggaagcagt gactgagatg tcctggccaa gttagtgaca gaggagggga 1980

gaaagaatcc agaccaattt gtatgcagta tattttactc ccatgaaata aaacacattt 2040
gtttcatatt tgctgaaaag taaaacaata atattgtacg aaatgttata cacagggtag 2100
gttgtacata gcagtttcag aaacatcatt gcatccacca gagaaactat tctaaaactg 2160
atattcacac atttttata ataataataa tatgttagaa acatacagtg tggcatttag 2220
tatatacact cccttgctcg caagcgaaaa atcctaatcg cttctgtata acatgcttta 2280
ttttaaagcc taacctttaa aaacactgtt gtgatattac taacaactgc ttttataaaa 2340
ttaatttgac atttcgatat atatacatcc tttcagtcat ttaaatgtta acaatgctaa 2400
acttaaaaaa taacaagctt atagtaatgt taaaatgtca tatccagtca aacatttgtt 2460
tgtgtatgtg tccttgcaac tgttagaaat acttgtagtg aaagatgtca gacactgagg 2520
acatcccttt gaaatcaaag gagctctctc tttgattcag tggtttcctt ttctctatat 2580
agcttctctt tctctccctt tctttagtgc ccacgacctt ctagcataat tcccagtctt 2640
tcaagggcgg agttgcccca tccggcaagg tcctaggatc ccggcgctgt gggtgcggct 2700
cacacgggcc ggtccactgc atactggcaa gcactcaggt tggaggccgg gttctgcacg 2760
ctggcgtagc cgaagctgga gtgctgcttt gctttcagtc tcaggctggc caggctcgag 2820
ttacacgtgt ccctataaac atacggagga gtcggcggcg cgtaaggaca ggcaggcgtc 2880
ggcaccgcgg aattcagcga cgggctactc aggttgttca agttattcag gctgttgaga 2940
ctggagcccg ggacgcctgt cactgctgag ggcaccatgc tggacgacat gctcatggac 3000
gagatagagt tgggtgggga aaacatgctc tgtgatgaca gggggttgac gttcatagag 3060
ttgaagaagg ggaagctctt ggtggatagg gaggcggatg taaggccctt ggcggcccag 3120
ttgttgtagg aatagcctgg gtacatgtcg tcgtagggct gcatgagccc attgaactgc 3180
ggcccgaagc cattcttgca tagctcggcc tgctggttgc gctccctctt tctccatttg 3240
gcccgacgat tcttgaacca aacctggggg cggttggggc aagggagcaa acagatgcca 3300
cagtgcagat tactaaaact tccatcggag gccaaccccc gccttccccc gacacacacg 3360
ctagcgcact cacacaccct ggcctcgctt cactgcaccg ccctgcacac caagatacca 3420
gggccagctt tcagttactg gcccgggtct ccaccaagcg caggagacct ggtctgctct 3480
ggcctgcgag ctgggactcg gagctacgcc acaaacctca gccgaacgca tggagacctg 3540
cggacggttt gatcactcag ccaggcgttt ctccaggtcc aaaaacactt aatgtaaaac 3600
aaacgcgggg cagcaggctt ttccaaccct tcccggggca ccttgcaaac ttgcttccat 3660
tccaaagcca cagacccacg gatgaggaga aggggctgga agggcactag aggatcgctc 3720
tttctcccac gcaattcctc ccttccttcc ctgacctcca ctgtcgtccc ccaccccctg 3780
gtacgtgctc ccttaacagg gactaggccg ccaacactct ttctcgccta gcaaaacaac 3840
caaataaaga gcaaaagacc acctcttcgt cagctcgtta actccaggag cttggcatat 3900
taaactccgg gaacccggaa agggtagttt tggagattcc cccttctttc gctctgcctc 3960
ttctttaccc taagcccacc acaggcctgt ccgcgcgcca ggcccagccg ggtcgtttgg 4020
ctttgcaggc ggccacccag gccggccggc ttccacccgt gtccggtggc ccagccgcaa 4080
ccccgatccc aatccacatc gggcctccct gtcgccccag acggcggctt ttgtgtattg 4140
gagagaggcc tggcctgaga tatccgagct gacaccagtg atgtttcaca ttacacatct 4200
ccgccgggcc cagccgtgta atccgctttt tctctttttc ctttcattct tgatttcctt 4260
tttatccccc ttcctctttg cacccgactg ctataaaaag cacgcctcac tcccacttgg 4320
ctcgacaagc agccgccctg gaaggagagg cagctgcaag gagagcccag cgccgcggct 4380
acaaagcact agggtggagc tgcggaatag cgggcggggt gggagggcgt tttcgaagga 4440
tcccagaaaa cccatagact ctgtctttaa ttacttgcca tttctaccct aggccatcta 4500
aactttgctc aggcgagaag agtacgtgag aggcccgttc ccttgatgtg caagagagct 4560
aatgaaagac tgaccttgct caaaaccacg ccgcccagga cccagctctg gctctggaca 4620
gttaaactaa aaccattttc aacttcttcc cggcctttta tccaccagca tagcctcatg 4680
ccttgcacaa atgccaccca gagagtgtct tcattccctc tgatttggga gagcattttg 4740
gtctttattc tttttatcgt tgttttcttc tttttgtttg ctctgctcta accgggggct 4800
ttattttttc tacccagagc acttaatttt tttttttaa cagcaaagcc tctggatgcc 4860
gcttgatttg cttgattctg ttttctgctt ccagaatcct aacaaatttg gaatcttcca 4920
ccgaccagca taaaccagga cgttgctatt gggttattta tttgagctca tttttgccaa 4980
tccataaagt acagatttgc tacaaagtta aggtaagccc tttttacaaa actatgatta 5040
taatttagaa gagggggtgt gagtttcaat ttccagagtt caactcctga gagaagataa 5100
ataaaccaag cagaaaagtc tttcttcttt ttttctttct ccttctaaga ggactagtag 5160
ttgtgtatta aaactttgct cccggagatc acaaaactag gaaatagggt gtgtgggaga 5220
gacctgaatg gccgaaacaa ccgtaaagaa ggtgtaagaa gcgcgagccc aggagggaaa 5280
aagctgggcc agggccggga caaaggtttc ccagggaggg ccaactcttc cgtgtctctg 5340
gcgggttttc cttgttaaag gctcacaggt tggagcctgt tcgcggctct tggcctggta 5400
gggattttat tagctctgct ctggcaactg caagccagga acacaatgtc ctgtgcaggg 5460
gattgcccat gcagcccagc tcgtgagatc gcgggatggc ggggcagtga gccggtgccg 5520
ctctgggagc ctgagccagg gcggcagtcc tgtcggcctc ggagagggaa ctgtaatctc 5580
gcaaccaggc cgccgcgagg ccttctgcct ttgcaaagct gcgccccacc ggcgccctcc 5640

```
caggcggcgc tgccttccac attctctcct ggtctacttg gcctgtacct ccacaacatc    5700
ctccccccat ccctcccaga ctccgtgctg gctcctaccc ggactcgggc ttccgtaagg    5760
ttggtccaca cagcgatttc ttcgcgtgtg gacatgtccg ggtagcggtt cctctggaaa    5820
gtggcctcca gctcctggag ctgctggctg gtaaagtgag tccgctgccg cctttgccgc    5880
ttcttcttag acgggtcctc ggcgcccacg tcctcattct tcccctgctg gcttttatct    5940
ttctctgaaa acgaaacaca cacactttcc cgtcagcatg cccacctgca acgcggacgc    6000
caactggacc ggcggcagaa gccgtggaag agctgggctg cctggcgccg gaggagggtg    6060
cgcgcggcgg ctccgggccg cgaggagcgc tgcgcctgtg ggtgtgcag gcgcaagtgt    6120
gggtgtccgc gccccatttc ctcccctccc ccagcgccgc acgttttatt tacatgttta    6180
tctcactgca gcggcacatt cacttttata gcctgtgctt tcaagtatat ttatacacct    6240
ctgcgcagac acaccaaatc tcctgggacg cgcacacgcg cgtggtttac agaccccccct    6300
ccccctcgca gaaagctcag atttccatgc ggtttgggaa ggctaggaaa agatgtgggg    6360
attcggttgg gcaccgaagt tcgccggccc tttcccaaaa aaaaaaaaaa aatgcctctt    6420
cgcgaagggc atttctgagt ggtttcaggc aatttcctaa cgagtggagc tcctcgggag    6480
ctgaaagccg agaggaaaac agggacagag gtcggcggcc tctgaaggtc ctcgaatcaa    6540
gatgctggga tttttgtgac ccaggaaaca gaagggaggc cagggtacga atagagaggg    6600
cggcagaatt gctcgcgccc ttagcgcccc aggagccggg ccggtcgagg gagaactaaa    6660
gggatgcggg gtagtcaaaa ttccggctcc cggaagttct gcggggagcc aggcgaacga    6720
ccactcccac cacgcctccc cccggagggg ctgacttcct tggggcgaga gggagcgggt    6780
ggcgcagagc agctgagcgg gaatgtctgc agggcggcgc ggcgccttac ctgcggcctc    6840
cgggctggag gtgtcggaga tggtgtgcac ctccagcctg tgcttggagg agtccagcga    6900
ccggggctga ccgggagcca gaaccgaagc catggctaac ggctggggat ggtgacagga    6960
agatgaggag acggccgaca gcttggtccc cgctgctcgg tgctccaagt gaagcgggcc    7020
tttcatgcag ttcatggacg agggagcgcg acgctctact agtccttggc tactgccccg    7080
ccgagccccc gtagccgccg ctgcccgctc cgggtcgcgc tctaggcgcg gagtttcccc    7140
gctgcgggga gagccagggg acgcaacccc cgccgagttc tcaagccaag ctgcccccgt    7200
ctcctccgga aggctcaagc gaaaaagtcc ggagacggaa agtcagcggg caaacgaaga    7260
catgggatgt gggcagaagg gcaccactca gagcgtcttt agggagcagg cttccaagct    7320
ccaaagcgaa acaagagtgg gcaaagaccc ccttcttctc tccctccctc ccccaagaac    7380
ccctccaata aggaaagcta acgccgaccg cgctctgccc gcccccccccc cacgcggcag    7440
ccctgacaga gaagtgtcaa gagtgacagg gacaggtagg tgatattaga tcccctgcgg    7500
cggcagcagc cgctgcagcc acgacgcggc cctctgagcg caccctccgc aacgcgcaca    7560
cgcacacccc tcgggcggtc gaacaggagc cgggccttgc cgcagctcag ctccaggcac    7620
ccaggcgagc gacggaccag atctgcggct ccgcgcttcc ctgttggcct aacatcttaa    7680
aaccagaggc gggcttcctg gtgccgagac gtcactccgc cgcggccctc cccagccctc    7740
tccgcctccg cctcctccca gacccttctc cgggtgcgac tgacgtggct ccgcaccaat    7800
caggacgccc cgagccgcgg tggagggact gtcctgcctg cacctatcag cagtgcgggg    7860
ccgggctact gcctcgccgt gcgcactggg tctacacagg caagctcccg ggaattcagc    7920
tcctgcccag cccaaggcga tccggctttt agtacgaacc caaaggtgaa gagatgaggc    7980
taggagtcga aggcttggga gaagagagtg gaatggtcaa gaagagaaag gtacaaggat    8040
caacaagaca cccactcttt gtgtctcact acatccattt ccaatccccc accccatata    8100
aaaaggagac acgttactta aaactagaaa atttgaaaaa cagcaacaaa tcacctctcc    8160
gatcttaaat tttccaaaca gcctgtcaag tgaatgctgc gctaatctga agaagcttta    8220
attgcaaaga agacagagcc ctgaaaaggc aggctaataa attagaaatc gagaagcaaa    8280
tggacccgtc aaaagaaaat taccttgact ttaaacgaac aactgtttgg tggttcactc    8340
tggatttata caagaataaa aagtcgcctc agatcacgtt ctctgtgatg cttattagtc    8400
cccagacaga aaacacacaa tagaagagaa accctaaccc agcgttttca aaatgctgaa    8460
agcttatcca ttctacttaa cgttgattaa gacacatatc ctagatcttt caaattcctt    8520
gtacactgta ttaagctcgt cctaacccga gagagccacg ctttaaattc gactctcttg    8580
tttactttat tatcaatcag atttaaatcc ataaagcctg tagaatcaac aaccttgagc    8640
taattatata tgaaatatgc cttaatgaat ttccatacaa ttaagaatgt tgccaaataa    8700
ccaatttcaa ggataatttt taacagtcat tttcttttcc cagtgagctc aaggctgtct    8760
tgagccatta aagtccaagc aggcagaagg ggtgtgtgtg agctaagggc gaaaagccta    8820
gaactgcgct caactagcaa aagcaaaacc ttatttatat aaaacaaaaa aaatcacctt    8880
tggagacatc aactctttat agcactgttt ccaagcaaat ttaatttcca aagaaattaa    8940
agaaagaaat ccaaacatat tcaaaataat ttttgaaagt ccttttgtcc cccagcatag    9000
gtcagctgga gaggacaaac taatctcctc tgggtttctg catgggcgat tgttttacta    9060
tggagttagt gttatcatct ctgaatgtgt atttgtttga cattacagtc aatgatttgc    9120
aatgccagca tgaagtatct ttaaaacact ccctccttgt ccttgttcac aagattggga    9180
aacttattcg atgtggaaca aagtggatga agcagactac aaatatattt gcaacttatg    9240
tgtctctcct ttgccctgac cacccccaaa ccctatctgc aactcctccc cattttaaac    9300
```

ttgcagtcca aagacgcaca tgagaattgt ttttcagtct ttcttcacca gtatcatccc 9360
actttaagaa taatttagct gcaagggagg aatttcttca tagtaagctt taaatcagca 9420
tttctgcttt taacctttta ttccacttta ccccattcca cacatacaga cacctgctca 9480
gagtaaaaca catcctcatg tgacaggtct gcattagctg aggctcatac atccagctat 9540
attaggtcct gcaatcttat cactaaatta tacacattac actagcagcc tgttggtaaa 9600
gaaggttaaa ttaatttaca ttctgctcat tatctggtgc ttaaatgacg cattttatcc 9660
cggagatttg gcggagaatc tccttctcag accccacagc gtttcactga agacaatgct 9720
cttacatttg tagtggtttt taatctgata agactctaat ttgcttaagt cttttaaata 9780
agggttttaa atgtttctag ccgttttctt attgaatttc ctctaattcc cccaagatca 9840
taaagtatat gtgtaaagta aatatttcct cccattgcac tgccagccga tgacctataa 9900
ctaagtcaat aagaatccag ctcttttctg ctgaatgtgt ttactaatca tattccagtt 9960
tcttctttta aacctcagaa tagctgtggt ccccacaata ccatgcccct taaagcttca 10020
tttcatgaag ggactccatc acattaaaga atgaaaaaaa tctccactgt agttagtata 10080
cacagcccct cactccttgt ttttcaagat tcaaacccca gagctgcaaa tatttttgga 10140
agcttgggtg ttaatgcctt attttagaaa gccgagaagc cccacagagc catatagatt 10200
tctaaaccca tctctcataa acccacagaa ttttgataaa agctctggtg gctccactct 10260
accgatggaa ctttcatcac gacaaatata catgtatgaa ggacctcaat cagcctccaa 10320
agtggttgaa aaacccaagg gcacgtgact gctcctcata gtgccaacgt gtgcgagatg 10380
ttggaagcac tggggatcag cagcagccta gatgcctaaa aagataaggt gtcctaattt 10440
gtgtggaccc attgaagtca agtggtgaat aaagacaatt atctagataa ttcagattaa 10500
agtaaaagca aaaccatatc tatttgtata tatatattca catccatttt atattacaga 10560
cacacacacg cacacacaca ctggctctgt aaacaactga ctcaaagtga ggattttctt 10620
tgcatttttc cagcaggagt ttcaacattc tcctaatctc ctaatcactt tacacactca 10680
cagcagcggc gattgggtga cattcttctc aggccccctg tgtggcagga caccaacatg 10740
ataagtctgc atggggaaaa ggaggtatgt ggtgggaact aagaaacact gtccagtgaa 10800
aactctgtgg catggtggtg gttgattttg gagatttaat gtacataaga cttgtgggtg 10860
cacaggcata ggcagcatgg atgagaaagg ggccagaaga aaataaatct tatgcatttt 10920
gtgattccag cattactgtg acctctggct aagttcttct taattggttt tagaaattac 10980
tatgagttca gcctctaaca cagaaatttc caatacggag aacattggtg ggatcctggt 11040
agggaaacta gaggtgctgc atggctcacg tggggcaaag aaggaaagcc cagtgccggc 11100
gtgaggtttt gagtctggga gacatcaggg gttgtctcga ttggggtttc ttgcttattc 11160
tttcaaagaa agaccctaga ggagggaaat gtgtgacatg gggtcagccg tgttttgtgc 11220
tggtatttgc caccgattac cagtcttaaa gtcttattta atttcacact cttcagtgtt 11280
agttgtgcaa agtccctctg gccatggcag tgagcggttg ggctgtgccg ccaaactctc 11340
cgtatcaatc tggcctggga ctcaaccaag tgatctctga cttttggaaa gagtctgtct 11400
tcagagttca cccagaagat ggcttaatta gacatctccc tgagctgtta ggccttagac 11460
gggtgggagt cctgccctgc ccaagctagc tcaaggacga ggcccgcctg gactcagctt 11520
ggagccacgt gatgggcgtg agtgtgtgag ctcctggtaa ggcgcagagg tcagatggag 11580
accttgcatc ctgcccgaga agtgccccac cccctccaat atctggcttt tctctgcata 11640
caaaccaagc tgaaaacagt ccactaccca ccacccctca tagctatgga accaaataac 11700
ccagaaatta aaagcttcac tgtagctgtc cttttcccca tttcctaaat ggaatttaaa 11760
aagctctggc ttgtcaaaag gggaagatta ttttctgaat tggaagtctg tagatatatt 11820
gagcaacagc caccctctct gggtccctgc aaatggtacc catttttcca acccacagct 11880
ctagctgctc aaccatttga gatttggggt aactacctgg gggaacagtg ttcagatggc 11940
agtgggagtt accacctcac agtggcctgg ggaagagaag agaaagagat tagaggaggg 12000
ggcatttgct aaaatcactc aacgaacatg ctgttaatgc ttcctcacat ttgcatgtta 12060
ctgccacagt tttcctaggt gtcactgagt ctccagaaag caactacttg ccgaactaag 12120
taaaataagg agaatggtat agcacatgtg tttggagaag gggaaggaag ggtggaatat 12180
gaaattgagc atagatatcc aggtcaggaa agaaggaagt ggcaaggggc taaatgaagt 12240
cccacccccc cgccactttt ttaaacaaca gttggattaa acactcatct gtcttcctct 12300
ttttctcttt tcttccctct ccagcttatg tccatctccc ttttctattt cttttgctct 12360
cccttctttc tgttttctct ctcccagaca tgctggtagc taacattcag cattagttgt 12420
catggtgacc ataaatcacc taaatccaaa aatacccacc tataatctga gatgaagctt 12480
ttatttccct agcgaaataa tattttaaaa gctgtcagct gacaaaaaaa aaggaaccca 12540
cctcattgta gtaacccaag taatatatta cttctaaagg tttaaattaa aatgtcagcc 12600
tgttaaaaac atgctggtag agtcttggac actcttcccg tcagatcctc acaaagaagt 12660
tgactctgtc attcctggtc gctctttaac atacacacac aattctgtat gctctctccc 12720
tttctattaa tttcttcctc ccctaccaac tactttagtt ctcaaagact ctacgcattg 12780
ggttctaaaa gaaaagaatc tcctcctgga tcagaaaaca gcctcattgg ttgctgaagt 12840
gaaagatgtg gggtttaggg ggaaaggtta ttaggaccat aatggcggtg gtggtaggag 12900
gtcatcctag aggagttaag aagaaaaaaa aatgcaggga gaaggactgg aggtggaaag 12960

```
acagagtaac agaaaactga gctgggtggt caggtgctgc ggtgaagtct agccccgaaa   13020
tgataggtat atattctccc actcctgcct ttttctcctc tgagagaaaa ctttcctagt   13080
cagagaccct ggggggtagg aggcgggcaa acgccgctgc agttgggcct tttgtcttct   13140
accttgggct tgttgtcttt tgcctatctt ggattacagg gtattcgcct agatgaagag   13200
ccattaatta cccattcagt caatattagg aagacgacaa agctttttac ataggattaa   13260
gaagacatca gattgttcca ttagtatatt cctgctcacg aataagcttt cgttatacat   13320
ttccttttcc cccgagccgc tctaacaact gctgcatata ttagcagcgg gcggtgagga   13380
ataacagccg aaccagcctt aagaaacttt gtgtaccgag tcagcagcga ggaacgcgac   13440
ctgtgaagac cacttttgcg ggcagggatc cgcagggact gactactttc ggacaactgg   13500
tacaatttcc cctgggggtg aaaaaccaca acgcggcggg gcacctttca agtgagctgc   13560
agatttgatt ggcgcggggg gtgggggagg ggaggggaga atgggatggc ggaggtcggg   13620
cggaggaaag aaaatggaaa actctcctta cccctatttc gttgcttttt cctcaagcct   13680
catgcccccct tcggcaagca cctgtccctt ccgcgctagc cacagctaga gttctccacc   13740
tttctctaca catccccttc tttctgacaa ggctcaggac cttggtcacc accccacgca   13800
ccaccatttt gcgttttgct agagacggtc tgggctgatc tcgctggtgt ccatgttagg   13860
attaaaatcc cttcatgacg gcgaggaaaa ctgtattatt tgctttcagg gggtacatta   13920
ggagtccacg tagtatatgc tccgcaaaca ttcgctgatt gaatgagagg cgcgggggcg   13980
gggcggcgga gaggggtctg cggccgccaa ggccgccagg gttaacccag gtccttcgaa   14040
gaaggttggg accgagctgc tgccgcgtgt gaaggtgtgt gtcgcggctg gggtgccac   14100
aacgggccat ggagcccacc tcccagaggg aggaagtctg tgcacaccag cgacttgggt   14160
cgaacacttt cagcccatcc actgggccaa agctatttaa caattaatgc gttcctgggg   14220
gaggccgggg gaagtacccg ccccttgtcg ggacgcaaag ccaggcgtca ggtccaaagg   14280
ggctgcagtg cagtccgatt tcagcacgga acccagagcc gccgccctga aactttttaa   14340
gccagtgaca gaggagggtc agtccgtcct cctcctgagg gtgaagacca ttttatgagt   14400
tcctttcagg acccccaaag caagaaaagc caaagaaagg tctctttgct ctagggggtc   14460
gttctcagct ggcttctaca ccatcttctg ctagctgcgt ccaccctctc tcaaacctct   14520
ggcttttagg ggtctccagt cgctctcgtg ctaccccccgt ttcggggttc tatccaggct   14580
gggcatccgc ccaatggata ccaaggagaa tggactcac tagggaagga aggcagagcc   14640
tggactgctt agaggtggac tctgcctata cagaacgttt agtctttaac gaggacatgg   14700
tatttttggg cggcgttggg ggcagaggcg gagagggtag cgtaatagac tatcacggcc   14760
ttttgaagaa gtcatatgtc attgtgaact ttttttctct ttaaaagcaa agaaaaactt   14820
taaaaaaaca caagaaataa atccttttgt tttacaagca ggctgtggcc aggactttgg   14880
atattccata agttaactta aaatcaggga aggataggtg ctctatcctt cagcagtgct   14940
atagctttgc ccactcgtgt gatttttttt tgtcgtctac tagttcctta aaagccaatt   15000
aaatcaagat tttcagcatt tcttcccatt acatgccttt tcttaattaa tggcattaaa   15060
cgtgtttagg cagcaatttt tcttttcggt caaaaagtag aaaaagacat attgagtgta   15120
ggggaagagc ctttcacgtg cactaaaaca atgcgggcct gaaagtaatg gttaagaaag   15180
caatcattca ttatttctag ttctttatgt gctagttatc aaaagcgaac gaccaggggc   15240
gcctgcgcgg cttgtgactg gcgcaagcag aactcctctg ctcttagccg tttcctgctc   15300
acctacgagg accccatcct accgtaggct tctccacctg tcctcagaat gcattcccca   15360
tgtctaggaa acctggggta gggactgggg gaaggagaca ttttgcgtct ctctggctcc   15420
cagcacaata agaaatgtca gcctcggctt ggcgactgcg agcccgcccc gcgcgaagcg   15480
agattgggga gctcctctag tctggccgga gccagggctg agcccgcgca aagcattctc   15540
cccagaagtc atcgctgctc ttgattttta accatatccc aaacatacta cggtctaata   15600
atttattttt actaccgatt atcaaacaga agaagaattt aacacaatct aaatgacaga   15660
aatcacgcga cgttatctct gcattgcccc catttaaccc catggggtta atcccggaca   15720
agtgagcgtt taactggccc agcagggcga ctggcggtgt agtgcagtgt ccgggcgtga   15780
agcactggat cgctttagac gcttcatttc aacaaatgat cattttctct cagattcacg   15840
gggaaactcc aatgcaagac ctttgttttc ttcttagtaa tacggtttgt ttttttgacc   15900
ggggtcccaa atcgcccccc tccatcccat attacatttg tattcttaca ctttaatccg   15960
gaaagagggg gttaggggcc gagggctgtg gggggggggg ggctttatct gctcacatta   16020
tcaaaggtca atagcctcct aaggctaggc acttatttac tacggagcca ggaaaataga   16080
ggaacagtaa atttgagggg tttttttcca tgtatttgaa aagaaaggca tcttccctcc   16140
tccatccctt acaccccccc ctccgcccca tagaacaagt ttcaattcag gaaaggcctg   16200
tggcgtaggt tggagacttt ttaacttttt acataagcct gtagacctcc tctggcaatg   16260
tccctcgatt cctccagagt gaaaccagcc aatcaagcaa cgacatcgcc aaaacccaag   16320
gcctggcaac cagtacttca ggcaggttcg cgtcgacagg ggcaaacctc cattctaccc   16380
tgggctgcta agcacagtgg ctgccctcca gctcttcagg gatgttgtcg gccccccgcc   16440
cctcccccaa ccagccaaag caaaccttcg ccaactcaag tttcccttgc ttgcctcccg   16500
cgatgaaccg cgcacccaca agtctgggtg gggcgtggct gagagcctga gtgactgagt   16560
gggccctggt ggtgctgcgc gcagcgggat ataacgagcg acagaggtcg ctgttggacc   16620
```

acttttccac gccagcctag acgccgaggt ttgctggagc gtgcgcaggg gatcagacca 16680
cagggagcga gcgagaggga gagagaggtg ttgggtctca ggagtgcagt ataatttggg 16740
gaaaggaatt aacgtccctg ggacggctgc ttcccgtccc acccagaggc ggagtgtcta 16800
agttcaagca gcaggcgcgt caggtctggc ggcctcgcct tcttgcgctt cgcccgaggc 16860
ccagagtccc ggaggcgggt gcccagcgcg cggcctgcgc ttccctcccg gccttaccat 16920
tggcgccagg atgctgccgc gggaagaact tgctgctggc tgcctctttt cggctctcag 16980
gagagtccgt gaactcgacc tttttgattt cggagtcttt ggagaagaga cattcaacgg 17040
ccgccggctg cacgcctggg ccacgcgcgc gcccgcccca cgtgcggaga gaggcgtccc 17100
ggaccgcggc cgaaaggagc cggggacggg aggaggggga ggggcgaggc aggccggagg 17160
agaaagaggg acaaagagca aagacccagt tagaggaaag agctgacggc atccccgtcc 17220
cccggaccct ctggcaaccc ggggcaggat ggcgtactct ttctgcgcct ccccggttgc 17280
cggcggttcc agccgggagg agtaggctgg ggggcttcgg tacacagcgc gccgctgctt 17340
cctagtccac cgccccgcca cagggagagg ccactggcga tttggttctg atttccttcc 17400
tgcccaggct ggcccctcgg ggaagcgccc ctcgctgggg cctcgccgca gggccagtgc 17460
cctcttgccg ccctcacgtg gcgcggcctc ccgtccgatg acccgggcag gagaaggggg 17520
ttcttaccta attgcacaca cgccgacacc agtttgcggc agttggtctc cattccccgt 17580
tatctgcaaa acagaagaga aggaaggctg taagaagcgg cggccgtcga gtgagcaggg 17640
ctcagatgag actacgttac actagctgtt aggcgcctac tgtgtgctag gcttcaggcg 17700
tgtttttccg acctgacagc ctctggctgt gcagcagcat ctccagccta gcctcgggcc 17760
caggacattt atttatcaag aggggatctc cctccagagt tgccgcaaaa gtgcccagag 17820
gttagaggac tataaagcca cagtgtgctg gggaggctgt ggacctactt tcaagaatct 17880
cggtgccggg gttaagaact catctgaacg caatggcagc gggagtgggt gggtggagag 17940
gacttttctc tttgggaagc tgtatgcaaa gaccaccctt cagtgcttgt ccattagttg 18000
gagctcggta aacatttgta gaacatcagc gccaacgtgc ccctgtccta gacagcagtt 18060
tccctcggtt ttctgtaatt ctgaaacgaa cgggctcttg gcctagggtg tttcaggagc 18120
gagctgagtt cgggcctctc atccatcagg agccactttt ccatatttag ccacactttc 18180
tcctagagat actaacccgg ccatttattt acttactaca aataattacc ctaaagtatg 18240
atttaagatc gcagaggaga gacactgggt ggactgagcg agactgagga gagcagggca 18300
aacgttcttg gagggtttac tgcccgccaa ggacggagaa atagctctgg tataactgct 18360
acccagttct ccccctttct ctcccgggcg agccaaatct ttttcacgtt ttcaaccaca 18420
acgcagcgag ccaagcattt aacgcgctcc cccttctctg ccacaggcaa gccgggagag 18480
gtgggtctcg aggggctcca ccgggtgggt agaagagccg cggttgcttt aaagacaaga 18540
aaagaaggtc cagggctccc taggcccctt cgatctcagc gcctgcctct ctccacgcta 18600
accagggtac gccgacgacc ggagggccca cttcgcgcgg gtgcggggat cggggtggga 18660
gcaagcgttg tcgggttggc ggaggcacag aggcggggca gggagctgcg ggcctgcctc 18720
tggcctgagt accgtttccc tgcgtctcgg cctctcctga agggagctgg gccctgggga 18780
gcctctggcc aaggtcgctg cctacaggag gggctgcccg gcgctgtggc gtggggatcc 18840
agggtgggga cggccaggcg gttcccccac ccgctagcga gaacgcgggc ggggactctg 18900
ccgattcgat ccttgtgggc ccgtgggccc agaagtagca gtttggcggc tccagattta 18960
gtgattctgc agtaaaatca caggattagt ccctgattga gatgtctgtt cgtgagatat 19020

tacaaaattc attatcacag ctttctacta actcgatatg aagtaacaca gatgggattt 19080
tattagtcca gacttcaaat gtttacttat gataatttcg gaggaaattt gcatgtcatc 19140
atcatttcga taatcttttt tttttatatg tctgaactgg ctgcattatt agctggtagc 19200
cggagcactg cagatggtaa ctgcaaatag tttttattta tttatttttt ttaaagaatg 19260
aaatatacaa aagaaaaaga ttgcgttgct tggtgtaaag tcagtcaatt attacacatt 19320
cttcccccca cttccccgtg cttcagtgct gaagaccaaa caaagcaata caaaacaaat 19380
cttcaagaac tcatagagct ccactctaag gactgaaaag aaggtcaagg cgtgttcctt 19440
agctcactcc tacatgtcct tgtgacttgg agatttattt tgcagtcaaa atgagccttg 19500
agacttgcat ttttatgctt catttaatga ccaggcctac tagaagaact gagtctaaat 19560
aactggggaa gataattttt taaaaagaga cctccaattc ccgcctgctg atccttaaac 19620
ttgccctatc aagacaagtc ttctgtgaga aatttggctg ccagacttcg gaactggctt 19680
caatggctaa tctcacaaat tgagatggga gacttttcct gatgggaggt agttctcacc 19740
cccaaagttc atgttctagt tggaatgtat atgccaagga ctcttgtttt ggccaacttg 19800
ggttttatat tgtgagcaca caaaaagcac tacacggcta acggaggacg aggaaccatg 19860
gcaaagcagg caggcaagcc ctaagaaata aaacaatttg ctaaaaaata atttctgatg 19920
actaccgcaa gactgaaagt gcaggaaaaa tacagttcga ataatcccag atcctttcac 19980
atttcccccc ttttcataca ctttgttacc ccataacaaa atctttaatg gaaagtttaa 20040
aaataaacag cacaggaaca tgtgttttaa atgaactaaa ttgtgaaatt agccagtaaa 20100
ttaatttgta gtaagtaatt atttaaggaa attaaaatac tgctcagttc agttctgtat 20160
tttaccatgt gtatgcgttc tttacaacca attaatataa gtgctttagg aacatttgaa 20220

gacaaacacg cttaacttaa ggaacaaagc acctaaataa tttaagtgta attttgctga 20280
gttaaagtaa aacattccac aaatgaagtg gctatttaat tttttaggga aagtttggtt 20340
attgaaatgt tgtatgctca tgttacatca acaaaaatct tcaatttatt ttgcttatgt 20400
gctttgtttt cttgatatta ttggtatttg aattttagat ggatttctgc caaaatgata 20460
ttttgtgtga taaaagcatc tttagttttg attgatagac taaaacaaat gcaaggaaat 20520
ttctttaaat cagattaatt tttcataaaa atattttaga atgtatgaat tctgatattt 20580
acatttataa tggtaaaagt tttttccgtt tagtttagta agacaatact cacacaaaag 20640
agtaaaaaaa aatcacacca ccttatgata gtttgatttc taaattgctt aagaaagtaa 20700
agtggttaaa ctggaaaaga ggaacatatt tcggaggttt agaatcgaaa attttttttct 20760
taatctccag ctggaaaata attctctgca tccatttaaa gtgtatctcc tgaagtgcca 20820
gattggagtt gactggtgat caatttaaag gagttacaat ccaaagaaat ggtgagagct 20880
tggcatccag gcctggctcc caggtaattc gcttgggcct gagaggtcac taactgccag 20940
ttaagatgga atctttttct tttctttttt ttcccaatgg ataacaatgg gaaggggggct 21000
aatcttccag tagctgaaac tttgtaccca gcccttttatc ttgagaatgc taatccttgg 21060
cccgaggatt tgttcctgca gtgttggcac cgagatttaa gggaagatac ctcgttttaa 21120
atgccagcca cggtctggct tccctctcga cttcagcacc ctgtagattg ttagtgtctg 21180
tggcggggga cgaaaggaac agggctttgc aaggtctgtt tgccgactgc gttaccttgg 21240
gcgaaactta gccccaaaag ccacaaatca cctacggtga agattctccg aagtggaaca 21300
aatttccaga ctcgcattat ctcacatccc tgcgggatag atggcctcca cttaccggct 21360
accgggagag agctgctgtc tccgcgtccc actgcttccc ggggcgattt ccagcgagcc 21420
gagcctccgg ctgcacggca agcgcccgaa agccgggcct gagaggactg cagggctcct 21480
gagggtgcca agttccgaag gagtccacgg gtgcactggg gcctccgaaa tctagccgcc 21540
actggcagtt tctttctgct cctctccagc tttctcgctc ggtctcgcac tctctctcct 21600
ctccctccct ctcatccctc tctcttccct ctgctcctac tccgtgtggg gagtgacgtg 21660
acgtcagcag agattccacc aaactccact gcacagtggc gcgcgggcgg ccggccgagc 21720
ccggctgcgc ggctggcgat ccaggagcga gcacagcgcc cgggcgagcg ccgggggggag 21780
cgagcagggg cgacgagaaa cgaggcaggg gagggaagca gatgccagcg ggccgaagag 21840
tcgggagccg gagccgggag agcgaaagga gaggggacct ggcggggcac ttaggagcca 21900
accgaggagc aggagcacgg actcccactg tggaaaggag gaccagaagg gaggatggga 21960
tggaagagaa gaaaaagcaa tctgcgccaa cccggcagcc ctaataaatc aaaggggggag 22020
cgccagggca gcgggggagac agaaacgtac ttttggggag caaatcagga cgggctggga 22080
ggaagcgaca gggaaagtgg cccaagagac ggaacaaagg acaatgttca tggggttgtt 22140
tgggacgagg cgtgtggagt gtgggtgtga gcgtgcgtgt gtgaccttct ttcaggcctg 22200
cagagttgag gaaagaggtc acagcaaaga gggactgcgg agggaggaaa gtgagagacc 22260
ggtagagggc gggagtggag gtgggcgcgg tggggatggg agaggatgag tgaagagaaa 22320
tctagaagaa tggagtgagc tagtgggaga gggtgggagg gccacagccg ggagcgaacg 22380
agctaggctt gtcagctggg gaaggccggg acgctgggcc cagcttagct gggacaccgc 22440
gcccgaggtc aaggcgggtg gaccaggcat gctgagagtg tcggcgcaca ggtgggcacg 22500
gccacgcact gacccagtgt tcacgaaggg tttgcactgg acaaggctca gacgctcata 22560
gagtctagaa tttcctctgc tgtacctaca ttcaacaagt tcaccctggg tcacggatat 22620
ctcatttttt aaaatgacga ggttaaggtt cctggcgagg atggtattaa attgcacggg 22680
atagaagtgg gggtggggga gagagtttcc ctcaagtcca catttgctcc tgcaaagcaa 22740
agagtatgtg aaattacagg gcatattctc actcgaaaag tgtgccttac ttctgaaccc 22800
tgattttctg atttcttgac ttgagcaaag atgtgtattt tggtagtgag cagaatattt 22860
tggctctgtc ctgcctctga gtggaaggac tataaatata attcgcctgg aggaccaggt 22920
gtgaaggctt ctgccaggca tatgggacaa tgttttttca atctcaaggg catcctgtta 22980
atgtatgttt ttggaaagtg ccggaacaca gccattgctc ctggattcgg attttcccac 23040
caatattaat tcctgcttga gagcaaaact caggcccgct attaaaaaga catctctttg 23100
gtccctaatt gagaataaag ttccctctaa aagttgtatt gctcttccta aatcaatata 23160
ccaatactcg caattttaga aatatatagt gactcgggag aatgtgcata aaatagatac 23220
gtttaaaaaa gcttggcgct taaaactaac cctagtcact atataggtgc tgggctttcc 23280
ctacttttgg gggctgtctg gaacatgtta tgtgttttct tgaattactc cgtgttttga 23340
attcatttga gttagcagta aaaacaggca aacaaacttg ctcaatttgt tttgagtgct 23400
aaatcccttc actttgaaat agctaacagt cgacagatgg actcatttta tggaaagggt 23460
tagcctcttc agccacgaag aaaactgatt agagatctac attttaagcc atttctaacc 23520
tccacgtaac atccgtgaaa actcaaactt tctctcttta cccagtggaa actcaaagca 23580
gtgttattta aggggagaga aatgaggggg aaaatgccca cgtgctgttt aattgtattt 23640
cctctctgac tctgagaatt tctatttctg gtttttgaaa tctcgccgag gcaagaaaat 23700
caaatttctt caacaagtcc cacaactgaa ctctagttac aggacaccgg aaagtgcagt 23760
ccgagaaaga catcttcacc tctgcccatc gacgattttt gcagcctccc cattcctctg 23820
agtaatgggc taataatcct cccttttttt cttttcattt tgtagagatt aagaggcgct 23880

cgtagcagaa cggccttgcc ttcagctggt ggcgaggata ggcaatctca tggaaaagtt 23940
ggaagagaat gagaaaacca aagacagaaa gattcagaga tccgcggaga gacacaggga 24000
gagggaaggg agttgcgctg aaaagacgca aagatacgcg cgtgcaactc cctccccttt 24060
caggtttcag aggtttgcaa accagggctg agaggaaggg gctcgggaag ctcacgttcc 24120
tctcgccccc cttctgtctg gagtctcgcc cgccagaggc tggttaaccc cagtcccggc 24180
cgccgcagac actgcgctga gcttttgggt cctcgccttg cccagcgcca gtgcagctga 24240
agtgagcagc tggtgggaaa tgcaaatggc tcctggagaa atagaagata cagaatgatt 24300
ctcattccct cctcgagtgt gtggaaggag ctggacatac gtttcacgct cctaatcctt 24360
cttttacatt tttagtcata ctcctattaa acaactaatt aatgcccaga atcaccaggg 24420
aatacattag gcatgcaatc gtagaagcag ggtgctgggg ggctacaaac caccgagctg 24480
atttaagacg tggatttcag gtttttccct tgtcaaagca gtaaaggaag agcgggcctt 24540
ggcgactgta tttagattcc gattacttca aattagaagg gggtggaggg agcgtccaag 24600
caaagcaagc aattctctgc cctgcagatg taaacaagat tgtagcatca aaggtactag 24660
ctcctttagg gctagatcgc ctggactggg agcctgggga aggggagaca ctaaccttac 24720
gtatctgtga atttcaagga tgttacattt ttacataaac aactccagtg cggattctct 24780
ggaatggggg gagtaatacc cccattctag aatattaaaa cattttcccc ccaaagcgta 24840
tatccttttt atttcctcaa aattttgaac tatgtctaaa gataatagtc ttccagtaaa 24900
ctggagcatt ggaccatttc cttcaccctt tcctaccgac attttgatga tctgatttta 24960
atgtgtgggg ggcacaggga attaaataca gcccataaaa ctaagcttag atgaaacagt 25020
gctggctaag tgggttcaga taatttttaa tgagaatctc aattacactc ctccccccaa 25080
tatgttgaga caagtgacag aaccgttaga atggtaatca aattggaaag ctcagggaga 25140
acaacaattt cgtgatcaaa ttggggcaaa accgtggaca aatgtggggt gacctccgcc 25200
aactccctgt cacccaagag tcaggatttg ggaaaggtac agtattattt cagagcccgc 25260
tgtgacgggc tgtgtgctac catttacttt cttcactctg gattatgatc tcaaccctgg 25320
caagcaattt ccccagctcc ttatctgaca acaagcgagt atgtaaatac caatggctag 25380
cgatgtttaa ctgcctcaaa cattattgat ttgttggctg ttctaaattg tcttcctagt 25440
ccaggtcttg tttccgaatt gtctattcta gaggtttgat ccatgcctcc gatgctacaa 25500
tacaataatt gtttttttaa aaaaggcatt taagatgaac caattgattt gcatataaat 25560
taaaattact atgcgttgcc gattccggtg ttttataatc atttcgaaat tagtacttaa 25620
ccacttgagc taaaagaata tataaatgcc tgtattgact cactaatgaa ttacccaatt 25680
aaaacgtccg ggcaatgctg ggcgctggaa agattgttaa atcaagacat attacaggag 25740
ggatatgaag attagaaagg taacagacca atatctcgca ctcaaaacgg agtttccggt 25800
gattcccagc tttaattttg gagcaggggt ctttctcctc tgctgttaaa aagattttgt 25860
gcttgtttgt gagtgagtgc attcaagtgg aaggaacgct cccacggcta cggtggctca 25920
ggccctttgc tcggaccggg accttacagt tctaacccag gagcgttaaa ctctggaaga 25980
ctccgggcca gccctggagg tgcgtggccc cgcaagtcgc caggccaagc ttgctttttc 26040
tgtctgcccc tccggcaggc tgggcgcgct atggcagtga gctttccgcg caaacggaga 26100
gctggaacca aagctgacat ttaatagata tgctaactga gcacttacct tcgtcctgag 26160
aataggaata aaaggtagct cttcttaaga gaggcggtgc aaaggcacgc tataggagtt 26220
cagaaaaggc tggcggcggg aaatctgtag cctggggggct agtcaacatc ccctttcatt 26280
tcaagcactt attgatttgc tgttgtcatc tttggcgacg cagaaggaca cttgaaagaa 26340
tttctgatgg ggctctgatc tgagaaagga ggtgacctgc ccaggctccc accaaattct 26400
taattaccac atcaactgct ttttttatc ccccacccga cctccttcct tttgcttatt 26460
cttaactttt taattattca gaaactccct tacctctcag tggcttccct tctgcagcag 26520
ttttctattc gaacctttc cccgcctttc cgtggtaggg cctgtatatt gatccctctg 26580
acctttggca catctgggcc ctctgaaatc tctcaatctt ttcagatttg aggatggcag 26640
gctccaccct ctccactgtg tgcacacact cagagatatg aaaacttaca cagactgcct 26700
tcaaacccag ggtatctaac agatgttccc tttccagttc gtctcttgat ctgaaatgcc 26760
tgcctgattc caacttggat accactcttt cttgcccttc ctttttcaaa gcagtttgga 26820
catgtgtgca agtgagccca gaacagctcc acccatattc tttaccaaac tgtaaataaa 26880
agaagaacta atgaagtaga ttggcatata gattgcatca agagcccgaa tccccagttt 26940
ctggattccc cattcaactc tggctgtcat ctacattgac agagtcattc taagcagagg 27000
cccagagaaa cctgcattgt gggacaacag gtaaagccac agtaaaaagt ggaataattt 27060
taaagtcatt ttattagaat gtaaattgta tttctgggtt ttgttcgtaa ccacctagtt 27120
ttaatatata cagagttaga caggaaaaaa taggtcaaca cagttattgg tactagagaa 27180
gacaaattcc atgggctcct cagtgaaaag aagatcccca aagtctataa tttttgatca 27240
tttaatttca tttataattg tgggaatgaa taagacacca actgctttat gtatttcatt 27300
tacatcaacc aatttgtgtt tccatcaaaa gcagttatac agaatttctt ttaacttctg 27360
gtagcaagtt cagaaaatga agcttacagc caccctgaac tggatacatc tcttgagctg 27420
accatttctg taagtgcagg aatataatat tgttcttcta tggtcttttt gcactctttt 27480
agggtttgca agtccttatc aggtctgaca tcactgtttg ggtctacatt cattacaagc 27540

```
aaatttgatt actatgctga ttttaaaaca gcctatttgg ccagcataat cttagttttc   27600
aaattataaa aaccttttaa tatacgaagt tcccagtttt tacctcctct agctccttgc   27660
tcattcaaaa cttctatttt aattggtgta agtaataata atttgtatta ctatttgtac   27720
tccttttact tttttggaga ttgggctgga ttccagagag aacaccagca tcaccaccac   27780
cacaaacaac aaaatctaaa agtaaagccc ttatttgcat gataattggt acttggaatg   27840
cttctgactt actcaatgcc accttataaa ggtaccttgt aaactttctt ggaatttcta   27900
gcaagagctt gtagtaactg gaacaactct ctgggaagat atcctctttg atgggctttc   27960
agtttctgga ggaatagatt gagagcaatt agggagggag gggacattgg aaattggcag   28020
ctacgtcagc tgaaacaagc ctgggttcag taaggtgact gatgttgtgg ttgattccct   28080
accccgagtt tctctttaat tggggcactg actcttccca ctttgggatc ccaaggcact   28140
cggtgtgtat gcagattcct cccttgtggt cttcaccatg tggtttcgta gcaggtctct   28200
ggttcaatga tattttatag tcatagccct catattcatt atcatgatct caatgtttag   28260
gcttttagtg tatttatatt aaacctgctt tattagtaag ctggagcaca caggagagat   28320
gggggcaagt aaggactcag cagagctcaa attcagacat gtttaaatgg ctttgactgt   28380
gtaaagtgtg gcaatgcttc ctgctgccct agctttccac tctaagcttc acatgtcctc   28440
tggctaatga agtgtgatat aggccacatg ctaggaataa tagtatttgt tgagaacaaa   28500
gtgaactcag gaaacctggt atacacaaaa tgcatc                              28536
```

<210> 962
<211> 28536
<212> DNA
<213> Artificial Sequence

<220>
<223> chemically treated genomic DNA (Homo sapiens)

<400> 962

```
gttttgggag ttaggttttt ttcgttggat gggaaaagga ggtgcggaaa ggggtaggcg      60
gtttgcgggt tagcggggat gagagttgtg gggagaacgg aggagagagt aaaattattt     120
tttttggaag tttaagggaa gggagttgag gtgagagaaa tggtttttt taggttttaa     180
gttttcgttt ttagtttgta ttatttaagt cggggaagtg ataatttttt ttaaatttcg     240
atttttattt tttgtttttt gtagattgat tgatgttttc gcgttgtttt agtttagtaa     300
ttaaattagg tcgaggtata ttttttttat ttagagtttt taaattattt attaatttgt     360
aggagattta aatttttcgg gtaggtttcg ttagcgtttt tagtttttat ttttaaattt     420
tttttttag ttttttttt tttttcgtt ttttggtttt tttcgttttt tattaggttg     480
agaggcggag aggtcgggat cgttgggttt tgcggtttta gaagttaaag ttcgttcgtg     540
gggatcgttt ttttttttat tgtttttaag tgaataatat ggtgggaggt gattttcgtt     600
taaagggttt ttatttgttt tttattgttg cgtgattttt tcgtaagtcg gaggtagagt     660
tttaaaaaga aagaaagtgt gcgttgagta ttgattttaa atcggggata ttatttgcga     720
tttttttaag tggtagtagt agttttttgt agaataaaga tttagtgttc gagttttaaa     780
agtttttagg taaagtttta tttagatgaa agtaattaag gcgaaaaaat aatgatattg     840
tcggttttag aaaatcggcg gttatttttt gttttaagtt ttttagcgtg gtgtttgttt     900
tcgtttattt tggttgtgcg gggggttgat aagtataata aaagatttta gtatattttt     960
tttttttattt ttataacgat ttttttagcg ttatgaggat gaattttttg ggattaagtg    1020
gtatttgttt ttatttgtta tgaaattaaa ttttatattt ttttaaagtt gaaaatcgcg    1080
gataaagaag gatcgggtgt ttaaagttta tttaaatatt tatatcgaat ttattttttg    1140
tttagatgtt agaggatggt agggagggtt agggttgtaa tttattttga tttgttttat    1200
tgttttgtag tttgtaaatt ataattttgt ataattttag gaataagtag gtttagaatt    1260
aatgggttaa tattatttaa aataaaatat cgggagtatg atttttgttt taattatata    1320
ttgttcgata agatttagga aattttattt taatttttta atttttgagt tttttgagaa    1380
attgaagggt tgtagttatt agaaattatt tttgtttttt ttaatgtttt taaaggattt    1440
ggaaatagta atgtaatata atatgaagga tttttttatt taagtttgaa gataaacgtg    1500
gaagtttaaa tattttgaat tggagatgtt tttttgtaaa tttattatag atgtattttt    1560
tttgagagaa atgtatataa attatatatg tatatatgta taaatatttt tttagaataa    1620
tttgttagag gtgtaggttt tttcgtaaag gagtaaattt gagagttatt ttaagttgat    1680
ggatttgtta aatttttttt tttttttttt ttttttatat tatttgttag ttataatgga    1740
attttttagg tttaagttaa agaaaaattg gagagataaa attagatttt gtagtttttt    1800
tttttttcgg gaatgttttt tttttttttt ttagtttttg atgaatggtt attatttatt    1860
tttattaaat ttaaataagg attgttgttt tgtatgttta attaggtagg tagagggaat    1920
tggtttgttt aggaagtagt gattgagatg ttttggttaa gttagtgata gaggagggga    1980
```

gaaagaattt agattaattt gtatgtagta tattttattt ttatgaaata aaatatattt 2040
gttttatatt tgttgaaaag taaaataata atattgtacg aaatgttata tatagggtag 2100
gttgtatata gtagttttag aaatatatt gtatttatta gagaaattat tttaaaattg 2160
atatttatat attttttata ataataataa tatgttagaa atatatagtg tggtatttag 2220
tatatatatt tttttgttcg taagcgaaaa attttaatcg tttttgtata atatgtttta 2280
ttttaaagtt taatttttaa aaatattgtt gtgatattat taataattgt ttttataaaa 2340
ttaatttgat atttcgatat atatatattt ttttagttat ttaaatgtta ataatgttaa 2400
atttaaaaaa taataagttt atagtaatgt taaaatgtta tatttagtta aatatttgtt 2460
tgtgtatgtg tttttgtaat tgttagaaat atttgtagtg aaagatgtta gatattgagg 2520
atattttttt gaaattaaag gagttttttt tttgatttag tggttttttt ttttttatat 2580
agtttttttt tttttttttt tttttagtgt ttacgatttt ttagtataat ttttagtttt 2640
ttaagggcgg agttgtttta ttcggtaagg ttttaggatt tcggcgttgt gggtgcggtt 2700
tatacgggtc ggtttattgt atattggtaa gtatttaggt tggaggtcgg gttttgtacg 2760
ttggcgtagt cgaagttgga gtgttgtttt gtttttagtt ttaggttggt taggttcgag 2820
ttatacgtgt ttttataaat atacggagga gtcggcggcg cgtaaggata ggtaggcgtc 2880
ggtatcgcgg aatttagcga cgggttattt aggttgttta agttatttag gttgttgaga 2940
ttggagttcg ggacgtttgt tattgttgag ggtattatgt tggacgatat gtttatggac 3000
gagatagagt tgggtgggga aaatatgttt tgtgatgata gggggttgac gtttatagag 3060
ttgaagaagg ggaagttttt ggtggatagg gaggcggatg taaggttttt ggcggtttag 3120
ttgttgtagg aatagtttgg gtatatgtcg tcgtagggtt gtatgagttt attgaattgc 3180
ggttcgaagt tatttttgta tagttcggtt tgttggttgc gttttttttt tttttatttg 3240
gttcgacgat ttttgaatta aatttggggg cggttggggt aagggagtaa atagatgtta 3300
tagtgtagat tattaaaatt tttatcggag gttaattttc gtttttttc gatatatacg 3360
ttagcgtatt tatatatttt ggtttcgttt tattgtatcg ttttgtatat taagatatta 3420
gggttagttt ttagttattg gttcgggttt ttattaagcg taggagattt ggtttgtttt 3480
ggtttgcgag ttgggattcg gagttacgtt ataaatttta gtcgaacgta tggagatttg 3540
cggacggttt gattatttag ttaggcgttt ttttaggttt aaaaatattt aatgtaaaat 3600
aaacgcgggg tagtaggttt ttttaatttt tttcggggta ttttgtaaat ttgtttttat 3660
tttaaagtta tagatttacg gatgaggaga aggggttgga agggtattag aggatcgttt 3720
ttttttttac gtaatttttt tttttttttt ttgattttta ttgtcgtttt ttattttttg 3780
gtacgtgttt ttttaatagg gattaggtcg ttaatatttt ttttcgttta gtaaaataat 3840
taaataaaga gtaaaagatt atttttcgt tagttcgtta attttaggag tttggtatat 3900
taaatttcgg gaattcggaa agggtagttt tggagatttt tttttttttc gttttgtttt 3960
ttttttattt taagtttatt ataggtttgt tcgcgcgtta ggtttagtcg ggtcgtttgg 4020
ttttgtaggc ggttatttag gtcggtcggt ttttattcgt gttcggtggt ttagtcgtaa 4080
tttcgatttt aatttatatc gggttttttt gtcgttttag acggcggttt ttgtgtattg 4140
gagagaggtt tggtttgaga tattcgagtt gatattagtg atgttttata ttatatattt 4200
tcgtcgggtt tagtcgtgta attcgttttt tttttttttt tttttatttt tgattttttt 4260
tttatttttt tttttttttg tattcgattg ttataaaaag tacgttttat ttttatttgg 4320
ttcgataagt agtcgttttg gaaggagagg tagttgtaag gagagtttag cgtcgcggtt 4380
ataaagtatt agggtggagt tgcggaatag cgggcggggt gggagggcgt tttcgaagga 4440
ttttagaaaa tttatagatt ttgtttttaa ttatttgtta tttttatttt aggttattta 4500
aattttgttt aggcgagaag agtacgtgag aggttcgttt ttttgatgtg taagagagtt 4560
aatgaaagat tgattttgtt taaaattacg tcgtttagga tttagttttg gttttggata 4620
gttaaattaa aattattttt aatttttttt cggtttttta tttattagta tagttttatg 4680
ttttgtataa atgttattta gagagtgttt ttattttttt tgatttggga gagtattttg 4740
gttttttattt tttttatcgt tgtttttttt tttttgtttg ttttgtttta atcgggggtt 4800
ttattttttt tatttagagt atttaatttt tttttttaa tagtaaagtt tttggatgtc 4860
gtttgatttg tttgattttg ttttttgttt ttagaatttt aataaatttg gaatttttta 4920
tcgattagta taaattagga cgttgttatt gggttattta tttgagttta tttttgttaa 4980
tttataaagt atagatttgt tataaagtta aggtaagttt tttttataaa attatgatta 5040
taatttagaa gagggggtgt gagttttaat ttttagagtt taatttttga gagaagataa 5100
ataaattaag tagaaaagtt tttttttttt tttttttttt ttttttaaga ggattagtag 5160
ttgtgtatta aaattttgtt ttcggagatt ataaaattag gaaatagggt gtgtgggaga 5220
gatttgaatg gtcgaaataa tcgtaaagaa ggtgtaagaa gcgcgagttt aggagggaaa 5280
aagttgggtt agggtcggga taaaggtttt ttagggaggg ttaatttttt cgtgtttttg 5340
gcgggttttt tttgttaaag gtttataggt tggagtttgt tcgcggtttt tggtttggta 5400
gggattttat tagttttgtt ttggtaattg taagttagga atataatgtt ttgtgtaggg 5460
gattgtttat gtagtttagt tcgtgagatc gcgggatggc ggggtagtga gtcggtgtcg 5520
ttttgggagt ttgagttagg gcggtagttt tgtcggtttc ggagagggaa ttgtaatttc 5580
gtaattaggt cgtcgcgagg ttttttgttt ttgtaaagtt gcgttttatc ggcgtttttt 5640

```
taggcggcgt tgttttttat attttttttt ggtttatttg gtttgtattt ttataatatt    5700
ttttttttat tttttttaga tttcgtgttg gtttttattc ggattcgggt tttcgtaagg    5760
ttggtttata tagcgatttt ttcgcgtgtg gatatgttcg ggtagcggtt tttttggaaa    5820
gtggttttta gtttttggag ttgttggttg gtaaagtgag ttcgttgtcg tttttgtcgt    5880
ttttttttag acgggttttc ggcgtttacg tttttatttt ttttttgttg gtttttattt    5940
tttttgaaa acgaaatata tatatttttt cgttagtatg tttatttgta acgcggacgt    6000
taattggatc ggcggtagaa gtcgtggaag agttgggttg tttggcgtcg gaggagggtg    6060
cgcgcggcgg tttcgggtcg cgaggagcgt tgcgtttgtg gggtgtgtag gcgtaagtgt    6120
gggtgttcgc gtttttattt tttttttttt ttagcgtcgt acgttttatt tatatgttta    6180
ttttattgta gcggtatatt tatttttata gtttgtgttt ttaagtatat ttatatattt    6240
ttgcgtagat atattaaatt ttttgggacg cgtatacgcg cgtggtttat agattttttt    6300
ttttttcgta gaaagtttag atttttatgc ggtttgggaa ggttaggaaa agatgtgggg    6360
attcggttgg gtatcgaagt tcgtcggttt tttttaaaa aaaaaaaaaa aatgtttttt    6420
cgcgaagggt atttttgagt ggttttaggt aattttttaa cgagtggagt ttttcgggag    6480
ttgaaagtcg agaggaaaat agggatagag gtcggcggtt tttgaaggtt ttcgaattaa    6540
gatgttggga tttttgtgat ttaggaaata gaagggaggt tagggtacga atagagaggg    6600
cggtagaatt gttcgcgttt ttagcgtttt aggagtcggg tcggtcgagg gagaattaaa    6660
gggatgcggg gtagttaaaa tttcggtttt cggaagtttt gcggggagtt aggcgaacga    6720
ttatttttat tacgtttttt ttcggagggg ttgatttttt tggggcgaga gggagcgggt    6780
ggcgtagagt agttgagcgg gaatgtttgt agggcggcgc ggcgttttat ttgcggtttt    6840
cgggttggag gtgtcggaga tggtgtgtat ttttagtttg tgtttggagg agtttagcga    6900
tcggggttga tcgggagtta gaatcgaagt tatggttaac ggttggggat ggtgatagga    6960
agatgaggag acggtcgata gtttggtttt cgttgttcgg tgttttaagt gaagcgggtt    7020
ttttatgtag tttatggacg agggagcgcg acgttttatt agtttttggt tattgtttcg    7080
tcgagttttc gtagtcgtcg ttgttcgttt cgggtcgcgt tttaggcgcg gagtttttttc    7140

gttgcgggga gagttagggg acgtaatttt cgtcgagttt ttaagttaag ttgttttcgt    7200
tttttttcgga aggtttaagc gaaaaagttc ggagacggaa agttagcggg taaacgaaga    7260
tatgggatgt gggtagaagg gtattattta gagcgttttt agggagtagg tttttaagtt    7320
ttaaagcgaa ataagagtgg gtaaagattt tttttttttt tttttttttt ttttaagaat    7380
tttttttaata aggaaagtta acgtcgatcg cgttttgttc gttttttttt tacgcggtag    7440
ttttgataga gaagtgttaa gagtgatagg gataggtagg tgatattaga ttttttgcgg    7500
cggtagtagt cgttgtagtt acgacgcggt tttttgagcg tatttttcgt aacgcgtata    7560
cgtatatttt tcgggcggtc gaataggagt cgggttttgt cgtagtttag ttttaggtat    7620
ttaggcgagc gacggattag atttgcggtt tcgcgttttt ttgttggttt aatattttaa    7680
aattagaggc gggtttttttg gtgtcgagac gttatttcgt cgcggttttt tttagttttt    7740
ttcgttttcg tttttttttta gatttttttt cgggtgcgat tgacgtggtt tcgtattaat    7800
taggacgttt cgagtcgcgg tggagggatt gttttgtttg tatttattag tagtgcgggg    7860
tcgggttatt gtttcgtcgt gcgtattggg tttatatagg taagttttcg ggaatttagt    7920
ttttgtttag tttaaggcga ttcggttttt agtacgaatt taaaggtgaa gagatgaggt    7980
taggagtcga aggtttggga gaagagagtg gaatggttaa gaagagaaag gtataaggat    8040
taataagata tttatttttt gtgttttatt atatttattt ttaatttttt attttatata    8100
aaaaggagat acgttattta aaattagaaa atttgaaaaa tagtaataaa ttatttttttc    8160
gattttaaat tttttaaata gtttgttaag tgaatgttgc gttaatttga agaagtttta    8220
attgtaaaga agatagagtt ttgaaaaggt aggttaataa attagaaatc gagaagtaaa    8280
tggattcgtt aaaagaaaat tattttgatt ttaaacgaat aattgtttgg tggtttattt    8340
tggatttata taagaataaa aagtcgtttt agattacgtt ttttgtgatg tttattagtt    8400
tttagataga aaatatataa tagaagagaa attttaattt agcgttttta aaatgttgaa    8460
agtttattta ttttatttaa cgttgattaa gatatatatt tttagatttt taaatttttt    8520
gtatattgta ttaagttcgt tttaattcga gagagttacg ttttaaattc gatttttttg    8580
tttattttat tattaattag atttaaattt ataaagtttg tagaattaat aattttgagt    8640
taattatata tgaaatatgt tttaatgaat ttttatataa ttaagaatgt tgttaaataa    8700
ttaattttaa ggataatttt taatagttat tttttttttt tagtgagttt aaggttgttt    8760
tgagttatta aagtttaagt aggtagaagg ggtgtgtgtg agttaagggc gaaaagttta    8820
gaattgcgtt taattagtaa aagtaaaatt ttatttatat aaaataaaaa aaattatttt    8880
tggagatatt aattttttat agtattgttt ttaagtaaat ttaattttta aagaaattaa    8940
agaaagaaat ttaaatatat ttaaaataat ttttgaaagt ttttttgttt tttagtatag    9000
gttagttgga gaggataaat taatttttttt tgggtttttg tatgggcgat tgttttatta    9060
tggagttagt gttattattt ttgaatgtgt atttgtttga tattatagtt aatgatttgt    9120
aatgttagta tgaagtattt ttaaaatatt ttttttttgt ttttgtttat aagattggga    9180
aatttattcg atgtggaata aagtggatga agtagattat aaatatattt gtaatttatg    9240
```

```
tgttttttt ttgttttgat tatttttaaa ttttatttgt aatttttttt tattttaaat     9300
ttgtagttta aagacgtata tgagaattgt tttttagttt ttttttatta gtattatttt     9360
attttaagaa taatttagtt gtaagggagg aattttttta tagtaagttt taaattagta     9420
tttttgtttt taatttttta ttttatttta ttttatttta tatatataga tatttgttta     9480
gagtaaaata tatttttatg tgataggttt gtattagttg aggtttatat atttagttat     9540
attaggtttt gtaattttat tattaaatta tatatattat attagtagtt tgttggtaaa     9600
gaaggttaaa ttaatttata ttttgtttat tatttggtgt ttaaatgacg tattttattt     9660
cggagatttg gcggagaatt tttttttag attttatagc gttttattga agataatgtt     9720
tttatatttg tagtggtttt taatttgata agattttaat ttgtttaagt tttttaaata     9780
agggttttaa atgtttttag tcgttttttt attgaatttt ttttaatttt tttaagatta     9840
taaagtatat gtgtaaagta aatattttttt tttattgtat tgttagtcga tgatttataa     9900
ttaagttaat aagaatttag tttttttttg ttgaatgtgt ttattaatta tattttagtt     9960
tttttttta aattttagaa tagttgtggt ttttataata ttatgttttt taaagtttta    10020
ttttatgaag ggattttatt atattaaaga atgaaaaaaa tttttattgt agttagtata    10080
tatagttttt tattttttgt tttttaagat ttaaatttta gagttgtaaa tatttttgga    10140
agtttgggtg ttaatgtttt attttagaaa gtcgagaagt tttatagagt tatatagatt    10200
tttaaattta tttttataa atttatagaa ttttgataaa agttttggtg gttttatttt    10260
atcgatggaa tttttattac gataaatata tatgtatgaa ggattttaat tagtttttaa    10320
agtggttgaa aaatttaagg gtacgtgatt gtttttttata gtgttaacgt gtgcgagatg    10380
ttggaagtat tggggattag tagtagttta gatgtttaaa aagataaggt gttttaattt    10440
gtgtggattt attgaagtta agtggtgaat aaagataatt atttagataa tttagattaa    10500
agtaaaagta aaattatatt tatttgtata tatatattta tatttatttt atattataga    10560
tatatatacg tatatatata ttggttttgt aaataattga tttaaagtga ggattttttt    10620
tgtatttttt tagtaggagt tttaatattt ttttaatttt ttaattattt tatatattta    10680
tagtagcggc gattgggtga tattttttttt aggtttttttg tgtggtagga tattaatatg    10740
ataagtttgt atggggaaaa ggaggtatgt ggtgggaatt aagaaatatt gtttagtgaa    10800
aattttgtgg tatggtggtg gttgattttg gagatttaat gtatataaga tttgtgggtg    10860
tataggtata ggtagtatgg atgagaaagg ggttagaaga aaataaattt tatgtatttt    10920
gtgattttag tattattgtg atttttggtt aagttttttt taattggttt tagaaattat    10980
tatgagttta gtttttaata tagaaatttt taatacggag aatattggtg ggattttggt    11040
agggaaatta gaggtgttgt atggtttacg tggggtaaag aaggaaagtt tagtgtcggc    11100
gtgaggtttt gagtttggga gatattaggg gttgtttcga ttggggtttt ttgtttattt    11160
ttttaaagaa agatttttaga ggagggaaat gtgtgatatg gggttagtcg tgttttgtgt    11220
tggtatttgt tatcgattat tagttttaaa gttttattta attttatatt ttttagtgtt    11280
agttgtgtaa agtttttttg gttatggtag tgagcggttg ggttgtgtcg ttaaattttt    11340
cgtattaatt tggtttggga tttaattaag tgatttttga tttttggaaa gagtttgttt    11400
ttagagttta tttagaagat ggtttaatta gatattttttt tgagttgtta ggttttagac    11460
gggtgggagt tttgttttgt ttaagttagt ttaaggacga ggttcgtttg gatttagttt    11520
ggagttacgt gatgggcgtg agtgtgtgag tttttggtaa ggcgtagagg ttagatggag    11580
atttgtattt ttgttcgaga agtgttttat ttttttttaat atttggtttt tttttgtata    11640
taaattaagt tgaaaatagt ttattattta ttatttttta tagttatgga attaaataat    11700
ttagaaatta aaagttttat tgtagttgtt tttttttta tttttaaat ggaatttaaa      11760
aagttttggt ttgttaaaag gggaagatta tttttgaat tggaagtttg tagatatatt     11820
gagtaatagt tatttttttt gggtttttgt aaatggtatt tatttttta atttatagtt     11880
ttagttgttt aattatttga gatttggggt aattatttgg gggaatagtg tttagatggt    11940
agtgggagtt attattttat agtggtttgg ggaagagaag agaaagagat tagaggaggg    12000
ggtatttgtt aaaattattt aacgaatatg ttgttaatgt tttttatat ttgtatgtta     12060
ttgttatagt tttttaggt gttattgagt ttttagaaag taattatttg tcgaattaag     12120
taaaataagg agaatggtat agtatatgtg tttggagaag gggaaggaag ggtggaatat    12180
gaaattgagt atagatattt aggttaggaa agaaggaagt ggtaaggggt taaatgaagt    12240
tttatttttt cgttattttt ttaaataata gttggattaa atatttattt gttttttttt    12300
ttttttttt ttttttttt ttagtttatg tttattttt tttttattt tttttgtttt       12360
ttttttttt tgttttttt tttttagata tgttggtagt taatatttag tattagttgt     12420
tatggtgatt ataaattatt taaatttaaa aatatttatt tataatttga gatgaagttt    12480
ttattttttt agcgaaataa tattttaaaa gttgttagtt gataaaaaaa aaggaattta   12540
ttttattgta gtaatttaag taatatatta tttttaaagg tttaaattaa aatgttagtt    12600
tgttaaaaat atgttggtag agttttggat attttttcg ttagattttt ataaagaagt     12660
tgattttgtt atttttggtc gttttttaat atatatatat aattttgtat gttttttttt    12720
tttttattaa ttttttttt ttttattaat tattttagtt tttaaagatt ttacgtattg     12780
ggttttaaaa gaaaagaatt tttttttgga ttagaaaata gttttattgg ttgttgaagt    12840
gaaagatgtg gggtttaggg ggaaaggtta ttaggattat aatggcggtg gtggtaggag    12900
```

gttattttag aggagttaag aagaaaaaaa aatgtaggga gaaggattgg aggtggaaag 12960
atagagtaat agaaaattga gttgggtggt taggtgttgc ggtgaagttt agtttcgaaa 13020
tgataggtat atattttttt atttttgttt tttttttttt tgagagaaaa ttttttttagt 13080
tagagatttt gggggggtagg aggcgggtaa acgtcgttgt agttgggttt tttgtttttt 13140
attttgggtt tgttgttttt tgtttatttt ggattatagg gtattcgttt agatgaagag 13200
ttattaatta tttatttagt taatatatagg aagacgataa agttttttat ataggattaa 13260
gaagatatta gattgtttta ttagtatatt tttgtttacg aataagtttt cgttatatat 13320
tttttttttt ttcgagtcgt tttaataatt gttgtatata ttagtagcgg gcggtgagga 13380
ataatagtcg aattagtttt aagaaatttt gtgtatcgag ttagtagcga ggaacgcgat 13440
ttgtgaagat tatttttgcg ggtagggatt cgtagggatt gattattttc ggataattgg 13500
tataattttt tttgggggtg aaaaattata acgcggcggg gtattttttta agtgagttgt 13560
agatttgatt ggcgcggggg gtgggggagg ggaggggaga atgggatggc ggaggtcggg 13620
cggaggaaag aaaatggaaa atttttttta tttttatttc gttgtttttt ttttaagttt 13680
tatgtttttt tcggtaagta tttgtttttt tcgcgttagt tatagttaga gttttttattt 13740
ttttttttata tatttttttt tttttgataa ggtttaggat tttggttatt attttacgta 13800
ttattatttt gcgttttgtt agagacggtt tgggttgatt tcgttggtgt ttatgttagg 13860
attaaaattt ttttatgacg gcgaggaaaa ttgtattatt tgtttttagg gggtatatta 13920
ggagtttacg tagtatatgt ttcgtaaata ttcgttgatt gaatgagagg cgcgggggcg 13980
gggcggcgga gaggggtttg cggtcgttaa ggtcgttagg gttaatttag gtttttcgaa 14040
gaaggttggg atcgagttgt tgtcgcgtgt gaaggtgtgt gtcgcggttg ggggtgttat 14100
aacgggttat ggagtttatt ttttagaggg aggaagtttg tgtatattag cgatttgggt 14160
cgaatatttt tagtttattt attgggttaa agttatttaa taattaatgc gtttttgggg 14220
gaggtcgggg gaagtattcg ttttttgtcg ggacgtaaag ttaggcgtta ggtttaaagg 14280
ggttgtagtg tagttcgatt ttagtacgga atttagagtc gtcgttttga aattttttaa 14340
gttagtgata gaggaggggtt agttcgtttt ttttttgagg gtgaagatta ttttatgagt 14400
tttttttagg atttttaaag taagaaaagt taaagaaagg tttttttgtt ttagggggtc 14460
gtttttagtt ggtttttata ttattttttg ttagttgcgt ttattttttt ttaaattttt 14520
ggtttttagg ggtttttagt cgttttcgtg ttattttcgt ttcggggttt tatttaggtt 14580
gggtattcgt ttaatggata ttaaggagaa tgggatttat tagggaagga aggtagagtt 14640
tggattgttt agaggtggat tttgtttata tagaacgttt agtttttaac gaggatatgg 14700
tatttttggg cggcgttggg ggtagaggcg gagagggtag cgtaatagat tattacggtt 14760
ttttgaagaa gttatatgtt attgtgaatt tttttttttt ttaaaagtaa agaaaaaattt 14820
taaaaaaata taagaaataa atttttttgt tttataagta ggttgtggtt aggattttgg 14880
atattttata agttaattta aaattaggga aggataggtg ttttattttt tagtagtgtt 14940
atagttttgt ttattcgtgt gatttttttt tgtcgtttat tagtttttta aaagttaatt 15000
aaattaagat ttttagtatt tttttttatt atatgttttt ttttaattaa tggtattaaa 15060
cgtgtttagg tagtaatttt ttttttcggt taaaaagtag aaaaagatat attgagtgta 15120
ggggaagagt tttttacgtg tattaaaata atgcgggttt gaaagtaatg gttaagaaag 15180
taattattta ttatttttag ttttttatgt gttagttatt aaaagcgaac gattaggggc 15240
gtttgcgcgg tttgtgattg gcgtaagtag aatttttttg tttttagtcg ttttttgttt 15300
atttacgagg attttatttt atcgtaggtt tttttatttg tttttagaat gtatttttta 15360
tgtttaggaa atttggggta gggattgggg gaaggagata ttttgcgttt ttttggtttt 15420
tagtataata agaaatgtta gtttcggttt ggcgattgcg agttcgtttc gcgcgaagcg 15480
agattgggga gtttttttag tttggtcgga gttagggttg agttcgcgta aagtattttt 15540
tttagaagtt atcgttgttt ttgattttta attatatttt aaatatatta cggtttaata 15600
atttattttt attatcgatt attaaataga agaagaattt aatataattt aaatgataga 15660
aattacgcga cgttattttt gtattgtttt tatttaattt tatggggtta atttcggata 15720
agtgagcgtt taattggttt agtagggcga ttggcggtgt agtgtagtgt tcgggcgtga 15780
agtattggat cgttttagac gttttatttt aataaatgat tatttttttt tagatttacg 15840
gggaaatttt aatgtaagat ttttgttttt tttttagtaa tacggtttgt ttttttgatc 15900
ggggttttaa atcgtttttt tttattttat attatatttg tatttttata ttttaattcg 15960
gaaagagggg gttaggggtc gagggttgtg gggggggggg ggttttattt gtttatatta 16020
ttaaaggtta atagtttttt aaggttaggt atttatttat tacggagtta ggaaaataga 16080
ggaatagtaa atttgagggg tttttttttta tgtatttgaa aagaaaggta tttttttttt 16140
tttatttttt atattttttt tttcgtttta tagaataagt tttaatttag gaaaggtttg 16200
tggcgtaggt tggagatttt ttaatttttt atataagttt gtagattttt tttggtaatg 16260
tttttcgatt tttttagagt gaaattagtt aattaagtaa cgatatcgtt aaaatttaag 16320
gtttggtaat tagtatttta ggtaggttcg cgtcgatagg ggtaaatttt tattttattt 16380
tgggttgtta agtatagtgg ttgtttttta gtttttttagg gatgttgtcg gtttttcgtt 16440
tttttttttaa ttagttaaag taaattttcg ttaatttaag ttttttttgt ttgtttttcg 16500
cgatgaatcg cgtatttata agtttgggtg gggcgtggtt gagagtttga gtgattgagt 16560

gggttttggt ggtgttgcgc gtagcgggat ataacgagcg atagaggtcg ttgttggatt 16620
atttttttac gttagtttag acgtcgaggt ttgttggagc gtgcgtaggg gattagatta 16680
tagggagcga gcgagaggga gagagaggtg ttgggtttta ggagtgtagt ataatttggg 16740
gaaaggaatt aacgtttttg ggacggttgt ttttcgtttt atttagaggc ggagtgttta 16800
agtttaagta gtaggcgcgt taggtttggc ggtttcgttt ttttgcgttt cgttcgaggt 16860
ttagagtttc ggaggcgggt gtttagcgcg cggtttgcgt tttttttttcg gttttattat 16920
tggcgttagg atgttgtcgc gggaagaatt tgttgttggt tgtttttttt cggtttttag 16980
gagagttcgt gaattcgatt tttttgattt cggagttttt ggagaagaga tatttaacgg 17040
tcgtcggttg tacgtttggg ttacgcgcgc gttcgtttta cgtgcggaga gaggcgtttc 17100
ggatcgcggt cgaaaggagt cggggacggg aggaggggga ggggcgaggt aggtcggagg 17160
agaaagaggg ataaagagta aagatttagt tagaggaaag agttgacggt attttcgttt 17220
ttcggatttt ttggtaattc ggggtaggat ggcgtatttt ttttgcgttt tttcggttgt 17280
cggcggtttt agtcgggagg agtaggttgg ggggtttcgg tatatagcgc gtcgttgttt 17340
tttagtttat cgtttcgtta tagggagagg ttattggcga tttggttttg attttttttt 17400
tgtttaggtt ggtttttcgg ggaagcgttt ttcgttgggg tttcgtcgta gggttagtgt 17460
tttttgtcg tttttacgtg gcgcggtttt tcgttcgatg attcgggtag gagaaggggg 17520
tttttattta attgtatata cgtcgatatt agtttgcggt agttggtttt tatttttcgt 17580
tatttgtaaa atagaagaga aggaaggttg taagaagcgg cggtcgtcga gtgagtaggg 17640
tttagatgag attacgttat attagttgtt aggcgtttat tgtgtgttag gttttaggcg 17700
tgtttttttcg atttgatagt ttttggttgt gtagtagtat ttttagttta gtttcgggtt 17760
taggatattt atttattaag aggggatttt tttttagagt tgtcgtaaaa gtgtttagag 17820
gttagaggat tataaagtta tagtgtgttg gggaggttgt ggatttattt ttaagaattt 17880
cggtgtcggg gttaagaatt tatttgaacg taatggtagc gggagtgggt gggtggagag 17940
gatttttttt tttgggaagt tgtatgtaaa gattattttt tagtgtttgt ttattagttg 18000
gagttcggta aatatttgta gaatattagc gttaacgtgt ttttgtttta gatagtagtt 18060
tttttcggtt ttttgtaatt ttgaaacgaa cgggtttttg gtttagggtg ttttaggagc 18120
gagttgagtt cgggtttttt atttattagg agttattttt ttatatttag ttatattttt 18180
ttttagagat attaattcgg ttatttattt atttattata aataattatt ttaaagtatg 18240
atttaagatc gtagaggaga gatattgggt ggattgagcg agattgagga gagtagggta 18300
aacgtttttg gagggtttat tgttcgttaa ggacggagaa atagttttgg tataattgtt 18360
atttagtttt tttttttttt ttttcgggcg agttaaattt tttttacgtt tttaattata 18420
acgtagcgag ttaagtattt aacgcgtttt tttttttttg ttataggtaa gtcgggagag 18480
gtgggtttcg aggggtttta tcgggtgggt agaagagtcg cggttgtttt aaagataaga 18540
aaagaaggtt tagggttttt taggtttttt cgattttagc gtttgttttt ttttacgtta 18600
attagggtac gtcgacgatc ggagggttta tttcgcgcgg gtgcggggat cggggtggga 18660
gtaagcgttg tcgggttggc ggaggtatag aggcggggta gggagttgcg ggtttgtttt 18720
tggtttgagt atcgtttttt tgcgtttcgg ttttttttga agggagttgg gttttgggga 18780
gtttttggtt aaggtcgttg tttataggag gggttgttcg gcgttgtggc gtggggattt 18840
agggtgggga cggttaggcg gtttttttat tcgttagcga gaacgcgggc ggggatttttg 18900
tcgattcgat ttttgtgggt tcgtgggttt agaagtagta gtttggcggt tttagattta 18960
gtgattttgt agtaaaatta taggattagt ttttgattga gatgtttgtt cgtgagatat 19020
tataaaattt attattatag tttttttatta attcgatatg aagtaatata gatgggattt 19080
tattagttta gattttaaat gtttatttat gataatttcg gaggaaattt gtatgttatt 19140
attatttcga taattttttt tttttatatg tttgaattgg ttgtattatt agttggtagt 19200
cggagtattg tagatggtaa ttgtaaatag tttttattta tttattttttt ttaaagaatg 19260
aaatatataa aagaaaaaga ttgcgttgtt tggtgtaaag ttagttaatt attatatatt 19320
ttttttttta tttttcgtg ttttagtgtt gaagattaaa taaagtaata taaaataaat 19380
ttttaagaat ttatagagtt ttattttaag gattgaaaag aaggttaagg cgtgtttttt 19440
agtttatttt tatatgtttt tgtgatttgg agatttattt tgtagttaaa atgagttttg 19500
agatttgtat ttttatgttt tatttaatga ttaggtttat tagaagaatt gagtttaaat 19560
aattggggaa gataattttt taaaaagaga tttttaattt tcgtttgttg atttttaaat 19620
ttgttttatt aagataagtt ttttgtgaga aatttggttg ttagatttcg gaattggttt 19680
taatggttaa ttttataaat tgagatggga gattttttttt gatgggaggt agtttttatt 19740
tttaaagttt atgttttagt tggaatgtat atgttaagga tttttgtttt ggttaatttg 19800
ggttttatat tgtgagtata taaaaagtat tatacggtta acggaggacg aggaattatg 19860
gtaaagtagg taggtaagtt ttaagaaata aaataatttg ttaaaaaata atttttgatg 19920
attatcgtaa gattgaaagt gtaggaaaaa tatagttcga ataattttag atttttttat 19980
attttttttt tttttatata ttttgttatt ttataataaa atttttaatg gaaagtttaa 20040
aaataaatag tataggaata tgtgttttaa atgaattaaa ttgtgaaatt agttagtaaa 20100
ttaatttgta gtaagtaatt atttaaggaa attaaaatat tgtttagttt agttttgtat 20160
tttattatgt gtatgcgttt tttataatta attaatataa gtgttttagg aatatttgaa 20220

```
gataaatacg tttaatttaa ggaataaagt atttaaataa tttaagtgta attttgttga   20280
gttaaagtaa aatattttat aaatgaagtg gttatttaat tttttaggga aagtttggtt   20340
attgaaatgt tgtatgttta tgttatatta ataaaaattt ttaatttatt ttgtttatgt   20400
gttttgtttt tttgatatta ttggtatttg aattttagat ggatttttgt taaaatgata   20460
ttttgtgtga taaaagtatt tttagttttg attgatagat taaaataaat gtaaggaaat   20520
ttttttaaat tagattaatt ttttataaaa atattttaga atgtatgaat tttgatattt   20580
atatttataa tggtaaaagt ttttttcgtt tagtttagta agataatatt tatataaaag   20640
agtaaaaaaa aattatatta ttttatgata gtttgatttt taaattgttt aagaaagtaa   20700
agtggttaaa ttggaaaaga ggaatatatt tcggaggttt agaatcgaaa attttttttt   20760
taatttttag ttggaaaata atttttgta tttatttaaa gtgtattttt tgaagtgtta   20820
gattggagtt gattggtgat taatttaaag gagttataat ttaaagaaat ggtgagagtt   20880
tggtatttag gtttggtttt taggtaattc gtttgggttt gagaggttat taattgttag   20940
ttaagatgga attttttttt tttttttttt tttttaatgg ataataatgg gaagggggtt   21000
aatttttag tagttgaaat tttgtattta gttttttatt ttgagaatgt taatttttgg   21060
ttcgaggatt tgtttttgta gtgttggtat cgagatttaa gggaagatat ttcgttttaa   21120
atgttagtta cggtttggtt tttttttcga ttttagtatt ttgtagattg ttagtgtttg   21180
tggcgggggga cgaaaggaat agggttttgt aaggtttgtt tgtcgattgc gttattttgg   21240
gcgaaattta gttttaaaag ttataaatta tttacggtga agatttttcg aagtggaata   21300
aatttttaga ttcgtattat tttatatttt tgcgggatag atggttttta tttatcggtt   21360
atcgggagag agttgttgtt ttcgcgtttt attgtttttc ggggcgattt ttagcgagtc   21420
gagttttcgg ttgtacggta agcgttcgaa agtcgggttt gagaggattg tagggttttt   21480
gagggtgtta agtttcgaag gagtttacgg gtgtattggg gttttcgaaa tttagtcgtt   21540
attggtagtt tttttttgtt ttttttagt tttttcgttc ggtttcgtat tttttttttt   21600
tttttttttt tttattttt tttttttttt ttgtttttat ttcgtgtggg gagtgacgtg   21660
acgttagtag agattttatt aaattttatt gtatagtggc gcgcgggcgg tcggtcgagt   21720
tcggttgcgc ggttggcgat ttaggagcga gtatagcgtt cgggcgagcg tcgggggggag   21780
cgagtagggg cgacgagaaa cgaggtaggg gagggaagta gatgttagcg ggtcgaagag   21840
tcgggagtcg gagtcgggag agcgaaagga gaggggattt ggcggggtat ttaggagtta   21900
atcgaggagt aggagtacgg atttttattg tggaaaggag gattagaagg gaggatggga   21960
tggaagagaa gaaaaagtaa tttgcgttaa ttcggtagtt ttaataaatt aaagggggag   22020
cgttagggta gcggggagat agaaacgtat ttttggggag taaattagga cgggttggga   22080
ggaagcgata gggaaagtgg tttaagagac ggaataaagg ataatgttta tggggttgtt   22140
tgggacgagg cgtgtggagt gtgggtgtga gcgtgcgtgt gtgatttttt tttaggtttg   22200
tagagttgag gaaagaggtt atagtaaaga gggattgcgg agggaggaaa gtgagagatc   22260
ggtagagggc gggagtggag gtgggcgcgg tggggatggg agaggatgag tgaagagaaa   22320
tttagaagaa tggagtgagt tagtgggaga gggtgggagg gttatagtcg ggagcgaacg   22380
agttaggttt gttagttggg gaaggtcggg acgttgggtt tagtttagtt gggatatcgc   22440
gttcgaggtt aaggcgggtg gattaggtat gttgagagtg tcggcgtata ggtgggtacg   22500

gttacgtatt gatttagtgt ttacgaaggg tttgtattgg ataaggttta gacgtttata   22560
gagtttagaa ttttttttgt tgtatttata tttaataagt ttattttggg ttacggatat   22620
tttattttt aaaatgacga ggttaaggtt tttggcgagg atggtattaa attgtacggg   22680
atagaagtgg gggtggggga gagagttttt tttaagttta tatttgtttt tgtaaagtaa   22740
agagtatgtg aaattatagg gtatattttt attcgaaaag tgtgttttat ttttgaattt   22800
tgattttttg atttttgat ttgagtaaag atgtgtattt tggtagtgag tagaatattt   22860
tggttttgtt ttgtttttga gtggaaggat tataaatata attcgtttgg aggattaggt   22920
gtgaaggttt ttgttaggta tatgggataa tgttttttta attttaaggg tattttgtta   22980
atgtatgttt ttggaaagtg tcggaatata gttattgttt ttggattcgg atttttttat   23040
taatattaat ttttgtttga gagtaaaatt taggttcgtt attaaaaaga tatttttttg   23100
gtttttaatt gagaataaag ttttttttaa aagttgtatt gttttttttta aattaatata   23160
ttaatattcg taattttaga aatatatagt gattcgggag aatgtgtata aaatagatac   23220
gtttaaaaaa gtttggcgtt taaaattaat tttagttatt atataggtgt tgggtttttt   23280
ttattttgg gggttgtttg gaatatgtta tgtgtttttt tgaattattt cgtgttttga   23340
atttatttga gttagtagta aaaataggta aataaatttg tttaatttgt tttgagtgtt   23400
aaatttttt attttgaaat agttaatagt cgatagatgg atttatttta tggaaagggt   23460
tagttttttt agttacgaag aaaattgatt agagatttat attttaagtt atttttaatt   23520
tttacgtaat attcgtgaaa atttaaattt ttttttttta tttagtggaa atttaaagta   23580
gtgttattta aggggagaga aatgaggggg aaaatgttta cgtgttgttt aattgtattt   23640
ttttttgat tttgagaatt tttatttttg gtttttgaaa tttcgtcgag gtaagaaaat   23700
taaatttttt taataagttt tataattgaa ttttagttat aggatatcgg aaagtgtagt   23760
tcgagaaaga tatttttatt tttgtttatc gacgattttt gtagtttttt tattttttg   23820
```

agtaatgggt taataatttt tttttttttt ttttttattt tgtagagatt aagaggcgtt 23880
cgtagtagaa cggttttgtt tttagttggt ggcgaggata ggtaatttta tggaaaagtt 23940
ggaagagaat gagaaaatta aagatagaaa gatttagaga ttcgcggaga gatataggga 24000
gagggaaggg agttgcgttg aaaagacgta aagatacgcg cgtgtaattt tttttttttt 24060
taggttttag aggtttgtaa attagggttg agaggaaggg gttcgggaag tttacgtttt 24120
tttcgttttt tttttgtttg gagtttcgtt cgttagaggt tggttaattt tagtttcggt 24180
cgtcgtagat attgcgttga gtttttgggt tttcgttttg tttagcgtta gtgtagttga 24240
agtgagtagt tggtgggaaa tgtaaatggt ttttggagaa atagaagata tagaatgatt 24300
tttatttttt tttcgagtgt gtggaaggag ttggatatac gttttacgtt tttaattttt 24360
tttttatatt tttagttata tttttattaa ataattaatt aatgtttaga attattaggg 24420
aatatattag gtatgtaatc gtagaagtag ggtgttgggg ggttataaat tatcgagttg 24480
atttaagacg tggattttag gttttttttt tgttaaagta gtaaaggaag agcgggtttt 24540
ggcgattgta tttagatttc gattatttta aattagaagg gggtggaggg agcgtttaag 24600
taaagtaagt aattttttgt tttgtagatg taaataagat tgtagtatta aaggtattag 24660
tttttttagg gttagatcgt ttggattggg agtttgggga aggggagata ttaattttac 24720
gtatttgtga attttaagga tgttatattt ttatataaat aattttagtg cggatttttt 24780
ggaatggggg gagtaatatt tttattttag aatattaaaa tatttttttt ttaaagcgta 24840
tatttttttt attttttaa aattttgaat tatgtttaaa gataatagtt ttttagtaaa 24900
ttggagtatt ggattatttt ttttattttt ttttatcgat attttgatga tttgatttta 24960
atgtgtgggg ggtataggga attaaatata gtttataaaa ttaagtttag atgaaatagt 25020
gttggttaag tgggtttaga taatttttaa tgagaatttt aattatattt tttttttaa 25080
tatgttgaga taagtgatag aatcgttaga atggtaatta aattggaaag tttagggaga 25140
ataataattt cgtgattaaa ttggggtaaa atcgtggata aatgtggggt gattttcgtt 25200
aatttttgt tatttaagag ttaggatttg ggaaaggtat agtattattt tagagttcgt 25260
tgtgacgggt tgtgtgttat tatttatttt ttttattttg gattatgatt ttaattttgg 25320
taagtaattt ttttagtttt ttatttgata ataagcgagt atgtaaatat taatggttag 25380
cgatgtttaa ttgttttaaa tattattgat ttgttggttg ttttaaattg tttttttagt 25440
ttaggttttg ttttcgaatt gtttatttta gaggtttgat ttatgttttc gatgttataa 25500
tataataatt gtttttttaa aaaaggtatt taagatgaat taattgattt gtatataaat 25560
taaaattatt atgcgttgtc gatttcggtg ttttataatt atttcgaaat tagtatttaa 25620
ttatttgagt taaaagaata tataaatgtt tgtattgatt tattaatgaa ttatttaatt 25680
aaaacgttcg ggtaatgttg ggcgttggaa agattgttaa attaagatat attataggag 25740
ggatatgaag attagaaagg taatagatta atatttcgta tttaaaacgg agttttcggt 25800
gatttttagt tttaattttg gagtaggggt tttttttttt tgttgttaaa aagattttgt 25860
gtttgtttgt gagtgagtgt atttaagtgg aaggaacgtt tttacggtta cggtggttta 25920
ggttttttgt tcggatcggg attttatagt tttaatttag gagcgttaaa ttttggaaga 25980
tttcgggtta gttttggagg tgcgtggttt cgtaagtcgt taggttaagt ttgttttttt 26040
tgtttgtttt ttcggtaggt tgggcgcgtt atggtagtga gtttttcgcg taaacggaga 26100
gttggaatta aagttgatat ttaatagata tgttaattga gtatttattt tcgttttgag 26160
aataggaata aaaggtagtt ttttttaaga gaggcggtgt aaaggtacgt tataggagtt 26220
tagaaaaggt tggcggcggg aaatttgtag tttgggggtt agttaatatt tttttttatt 26280
ttaagtattt attgatttgt tgttgttatt tttggcgacg tagaaggata tttgaaagaa 26340
tttttgatgg ggttttgatt tgagaaagga ggtgatttgt ttaggttttt attaaatttt 26400
taattattat attaattgtt tttttttatt ttttattcga tttttttttt tttgtttatt 26460
tttaattttt taattattta gaaatttttt tattttttag tggttttttt tttgtagtag 26520
tttttttattc gaattttttt ttcgtttttt cgtggtaggg tttgtatatt gatttttttg 26580
atttttggta tatttgggtt ttttgaaatt ttttaatttt tttagatttg aggatggtag 26640
gttttatttt ttttattgtg tgtatatatt tagagatatg aaaatttata tagattgttt 26700
ttaaatttag ggtatttaat agatgttttt tttttagttc gttttttgat ttgaaatgtt 26760
tgtttgattt taatttggat attatttttt tttgtttttt tttttttaaa gtagtttgga 26820
tatgtgtgta agtgagttta gaatagtttt atttatattt tttattaaat tgtaaataaa 26880
agaagaatta atgaagtaga ttggtatata gattgtatta agagttcgaa tttttagttt 26940
ttggattttt tatttaattt tggttgttat ttatattgat agagttattt taagtagagg 27000
tttagagaaa tttgtattgt gggataatag gtaaagttat agtaaaaagt ggaataattt 27060
taaagttatt ttattagaat gtaaattgta tttttgggtt ttgttcgtaa ttatttagtt 27120
ttaatatata tagagttaga taggaaaaaa taggttaata tagttattgg tattagagaa 27180
gataaatttt atgggttttt tagtgaaaag aagattttta aagtttataa tttttgatta 27240
tttaatttta tttataattg tgggaatgaa taagatatta attgttttat gtattttatt 27300
tatattaatt aatttgtgtt tttattaaaa gtagttatat agaatttttt ttaattttttg 27360
gtagtaagtt tagaaaatga agtttatagt tattttgaat tggatatatt ttttgagttg 27420
attatttttg taagtgtagg aatataatat tgttttttta tggttttttt gtattttttt 27480

```
agggtttgta agtttttatt aggtttgata ttattgtttg ggtttatatt tattataagt   27540
aaatttgatt attatgttga ttttaaaata gtttatttgg ttagtataat tttagttttt   27600
aaattataaa aattttttaa tatacgaagt ttttagtttt tattttttttt agtttttttgt   27660
ttatttaaaa tttttatttt aattggtgta agtaataata atttgtatta ttatttgtat   27720
tttttttatt tttttggaga ttgggttgga ttttagagag aatattagta ttattattat   27780
tataaataat aaaatttaaa agtaaagttt ttatttgtat gataattggt atttggaatg   27840
tttttgattt atttaatgtt attttataaa ggtattttgt aaattttttt ggaattttta   27900
gtaagagttt gtagtaattg gaataatttt ttgggaagat atttttttttg atgggttttt   27960
agtttttgga ggaatagatt gagagtaatt agggagggag gggatattgg aaattggtag   28020
ttacgttagt tgaaataagt ttgggtttag taaggtgatt gatgttgtgg ttgatttttt   28080
atttcgagtt tttttttaat tggggtattg atttttttta ttttgggatt ttaaggtatt   28140
cggtgtgtat gtagattttt tttttgtggt ttttattatg tggtttcgta gtaggttttt   28200
ggtttaatga tattttatag ttatagtttt tatatttatt attatgattt taatgtttag   28260
gtttttagtg tatttatatt aaatttgttt tattagtaag ttggagtata taggagagat   28320
gggggtaagt aaggatttag tagagtttaa atttagatat gtttaaatgg ttttgattgt   28380
gtaaagtgtg gtaatgtttt ttgttgtttt agttttttat tttaagtttt atatgttttt   28440
tggttaatga agtgtgatat aggttatatg ttaggaataa tagtatttgt tgagaataaa   28500
gtgaatttag gaaatttggt atatataaaa tgtatt                              28536
```

<210> 963
<211> 28536
<212> DNA
<213> Artificial Sequence

<220>
<223> chemically treated genomic DNA (Homo sapiens)

<400> 963

```
gatgtatttt gtgtatatta ggttttttga gtttattttg tttttaataa atattattat      60
ttttagtatg tggtttatat tatattttat tagttagagg atatgtgaag tttagagtgg     120
aaagttaggg tagtaggaag tattgttata ttttatatag ttaaagttat ttaaatatgt     180
ttgaatttga gttttgttga gtttttattt gtttttattt tttttgtgtg ttttagttta     240
ttaataaagt aggtttaata taaatatatt aaaagtttaa atattgagat tatgataatg     300
aatatgaggg ttatgattat aaaatattat tgaattagag atttgttacg aaattatatg     360
gtgaagatta taagggagga atttgtatat atatcgagtg ttttgggatt ttaaagtggg     420
aagagttagt gttttaatta aagagaaatt cggggtaggg aattaattat aatattagtt     480
attttattga atttaggttt gttttagttg acgtagttgt taatttttaa tgtttttttt     540
tttttttaatt gtttttaatt tatttttttta gaaattgaaa gtttattaaa gaggatattt     600
ttttagagag ttgttttagt tattataagt ttttgttaga aattttaaga aagtttataa     660
ggtattttta taaggtggta ttgagtaagt tagaagtatt ttaagtatta attattatgt     720
aaataagggt tttattttta gattttgttg tttgtggtgg tggtgatgtt ggtgtttttt     780
ttggaattta gtttaatttt taaaaaagta aaaggagtat aaatagtaat ataaattatt     840
attatttata ttaattaaaa tagaagtttt gaatgagtaa ggagttagag gaggtaaaaa     900
ttgggaattt cgtatattaa aaggttttta taatttgaaa attaagatta tgttggttaa     960
ataggttgtt ttaaaattag tatagtaatt aaatttgttt gtaatgaatg tagatttaaa    1020
tagtgatgtt agatttgata aggatttgta aattttaaaa gagtgtaaaa agattataga    1080
agaataatat tatatttttg tatttataga aatggttagt ttaagagatg tatttagttt    1140
agggtggttg taagttttat tttttgaatt tgttattaga agttaaaaga aattttgtat    1200
aattgttttt gatggaaata taaattggtt gatgtaaatg aaatatataa agtagttggt    1260
gttttattta tttttataat tataaatgaa attaaatgat taaaaattat agattttggg    1320
gattttttttt ttattgagga gtttatggaa tttgtttttt ttagtattaa taattgtgtt    1380
gatttatttt tttttgttta attttgtata tattaaaatt aggtggttac gaataaaatt    1440
tagaaatata atttatattt taataaaatg attttaaaat tattttattt tttattgtgg    1500
ttttatttgt tgttttataa tgtaggtttt tttgggtttt tgtttagaat gattttgtta    1560
atgtagatga tagttagagt tgaatgggga atttagaaat tggggattcg ggtttttgat    1620
gtaatttata tgttaattta ttttattagt ttttttttta tttatagttt ggtaaagaat    1680
atgggtggag ttgttttggg tttatttgta tatatgttta aattgttttg aaaaaggaag    1740
ggtaagaaag agtggtattt aagttggaat taggtaggta ttttagatta agagacgaat    1800
tggaaaggga atatttgtta gatattttgg gtttgaaggt agtttgtgta agtttttata    1860
tttttgagtg tgtgtatata gtggagaggg tggagtttgt tatttttaaa tttgaaaaga    1920
```

ttgagagatt ttagagggtt tagatgtgtt aaaggttaga gggattaata tataggtttt 1980
attacggaaa ggcggggaaa aggttcgaat agaaaattgt tgtagaaggg aagttattga 2040
gaggtaaggg agtttttgaa taattaaaaa gttaagaata agtaaaagga aggaggtcgg 2100
gtgggggata aaaaaaagta gttgatgtgg taattaagaa tttggtggga gtttgggtag 2160
gttatttttt tttttagatt agagttttat tagaaatttt tttaagtgtt tttttgcgtc 2220
gttaaagatg ataatagtaa attaataagt gtttgaaatg aaaggggatg ttgattagtt 2280
tttaggttat agatttttcg tcgttagttt tttttgaatt tttatagcgt gtttttgtat 2340
cgtttttttt aagaagagtt atttttttatt tttattttta ggacgaaggt aagtgtttag 2400
ttagtatatt tattaaatgt tagttttggt tttagttttt cgtttgcgcg gaaagtttat 2460
tgttatagcg cgtttagttt gtcggagggg tagatagaaa aagtaagttt ggtttggcga 2520
tttgcggggt tacgtatttt tagggttggt tcggagtttt ttagagttta acgtttttgg 2580
gttagaattg taaggtttcg gttcgagtaa agggtttgag ttatcgtagt cgtgggagcg 2640
ttttttttat ttgaatgtat ttatttataa ataagtataa aattttttta atagtagagg 2700
agaaagattt ttgttttaaa attaaagttg ggaattatcg gaaatttcgt tttgagtgcg 2760
agatattggt ttgttatttt tttaattttt atattttttt tgtaatatgt tttgatttaa 2820
taattttttt agcgtttagt attgttcgga cgttttaatt gggtaattta ttagtgagtt 2880
aatataggta tttatatatt tttttagttt aagtggttaa gtattaattt cgaaatgatt 2940
ataaaatatc ggaatcggta acgtatagta attttaattt atatgtaaat taattggttt 3000
attttaaatg tttttttttaa aaaaataatt attgtattgt agtatcggag gtatggatta 3060
aatttttaga atagataatt cggaaataag atttggatta ggaagataat ttagaatagt 3120
taataaatta ataatgtttg aggtagttaa atatcgttag ttattggtat ttatatattc 3180
gtttgttgtt agataaggag ttggggaaat tgtttgttag ggttgagatt ataatttaga 3240
gtgaagaaag taaatggtag tatatagttc gttatagcgg gttttgaaat aatattgtat 3300
ttttttttaaa ttttgatttt tgggtgatag ggagttggcg gaggttattt tatatttgtt 3360
tacggttttg ttttaatttg attacgaaat tgttgttttt tttgagtttt ttaatttgat 3420
tattatttta acggttttgt tatttgtttt aatatattgg ggggaggagt gtaattgaga 3480
tttttattaa aaattatttg aatttattta gttagtattg ttttatttaa gtttagtttt 3540
atgggttgta tttaattttt tgtgtttttt atatattaaa attagattat taaaatgtcg 3600
gtaggaaagg gtgaaggaaa tggtttaatg ttttagttta ttggaagatt attattttta 3660
gatatagttt aaaattttga ggaaataaaa aggatatacg ttttgggggg aaaatgtttt 3720
aatattttag aatgggggta ttattttttt tattttagag aattcgtatt ggagttgttt 3780
atgtaaaaat gtaatatttt tgaaatttat agatacgtaa ggttagtgtt tttttttttt 3840
taggttttta gtttaggcga tttagtttta aaggagttag tatttttgat gttataattt 3900
tgtttatatt tgtagggtag agaattgttt gttttgtttg gacgtttttt ttattttttt 3960
ttaatttgaa gtaatcggaa tttaaatata gtcgttaagg ttcgtttttt ttttattgtt 4020
ttgataaggg aaaaatttga aatttacgtt ttaaattagt tcggtggttt gtagtttttt 4080
agtattttgt ttttacgatt gtatgtttaa tgtatttttt ggtgattttg ggtattaatt 4140
agttgtttaa taggagtatg attaaaaatg taaaagaagg attaggagcg tgaaacgtat 4200
gtttagtttt ttttatatat tcgaggaggg aatgagaatt attttgtatt ttttattttt 4260
ttaggagtta tttgtatttt ttattagttg tttattttag ttgtattggc gttgggtaag 4320
gcgaggattt aaaagtttag cgtagtgttt gcggcggtcg ggattggggt taattagttt 4380
ttggcgggcg agattttaga tagaaggggg gcgagaggaa cgtgagtttt tcgagttttt 4440
tttttttagt tttggtttgt aaatttttga aatttgaaag gggagggagt tgtacgcgcg 4500
tatttttgcg tttttttagc gtaatttttt tttttttttt tgtgtttttt cgcggatttt 4560
tgaatttttt tgtttttggt ttttttattt ttttttaatt tttttatgag attgtttatt 4620
ttcgttatta gttgaaggta aggtcgtttt gttacgagcg ttttttaatt tttataaaat 4680
gaaaagaaaa aaagggagga ttattagttt attatttaga ggaatgggga ggttgtaaaa 4740
atcgtcgatg ggtagaggtg aagatgtttt tttcggattg tatttttcgg tgttttgtaa 4800
ttagagttta gttgtgggat ttgttgaaga aatttgattt ttttgtttcg gcgagatttt 4860
aaaaattaga aatagaaatt tttagagtta gagaggaaat ataattaaat agtacgtggg 4920
tatttttttt tttatttttt tttttttaaa taatattgtt ttgagttttt attgggtaaa 4980
gagagaaagt ttgagttttt acggatgtta cgtggaggtt agaaatggtt taaaatgtag 5040
atttttaatt agttttttttc gtggttgaag aggttaattt tttttataaa atgagtttat 5100
ttgtcgattg ttagttattt taaagtgaag ggatttagta tttaaaataa attgagtaag 5160
tttgtttgtt tgtttttatt gttaatttaa atgaatttaa aatacggagt aatttaagaa 5220
aatatataat atgttttaga tagtttttaa aagtagggaa agtttagtat ttatatagtg 5280
attagggtta gttttaagcg ttaagttttt ttaaacgtat ttattttatg tatatttttt 5340
cgagttatta tatattttta aaattgcgag tattggtata ttgatttagg aagagtaata 5400
taatttttag agggaatttt atttttaatt agggattaaa gagatgtttt tttaatagcg 5460
ggtttgagtt ttgtttttaa gtaggaatta atattggtgg gaaaattcga atttaggagt 5520
aatggttgtg tttcggtatt ttttaaaaat atatattaat aggatgtttt tgagattgaa 5580

```
aaaatattgt tttatatgtt tggtagaagt ttttatattt ggttttttag gcgaattata    5640
tttatagttt ttttatttag aggtaggata gagttaaaat attttgttta ttattaaaat    5700
atatattttt gtttaagtta agaaattaga aaattagggt ttagaagtaa ggtatatttt    5760
tcgagtgaga atatgttttg taattttata tattttttgt tttgtaggag taaatgtgga    5820
tttgagggaa attttttttt ttatttttat ttttatttcg tgtaatttaa tattattttc    5880
gttaggaatt ttaatttcgt tattttaaaa aatgagatat tcgtgattta gggtgaattt    5940
gttgaatgta ggtatagtag aggaaatttt agattttatg agcgtttgag ttttgtttag    6000
tgtaaatttt tcgtgaatat tgggttagtg cgtggtcgtg tttatttgtg cgtcgatatt    6060
tttagtatgt ttggtttatt cgttttgatt tcgggcgcgg tgttttagtt aagttgggtt    6120
tagcgtttcg gttttttta gttgataagt ttagttcgtt cgttttcggt tgtggttttt    6180
ttattttttt ttattagttt attttatttt tttagatttt tttttattta ttttttttta    6240
tttttatcgc gtttattttt attttcgttt tttatcggtt ttttattttt tttttttcgt    6300
agtttttttt tgttgtgatt ttttttttta attttgtagg tttgaaagaa ggttatatac    6360
gtacgtttat atttatattt tatacgtttc gttttaaata attttatgaa tattgttttt    6420
tgtttcgttt tttgggttat ttttttgtc gttttttttt agttcgtttt gatttgtttt    6480
ttaaaagtac gtttttgttt tttcgttgtt ttggcgtttt ttttttgatt tattagggtt    6540
gtcgggttgg cgtagattgt tttttttttt tttttatttt attttttttt ttggtttttt    6600
tttttatagt gggagttcgt gtttttgttt ttcggttggt ttttaagtgt ttcgttaggt    6660
tttttttttt ttcgtttttt cggtttcggt tttcgatttt tcggttcgtt ggtatttgtt    6720
tttttttttt gtttcgtttt tcgtcgtttt tgttcgtttt tttcggcgtt cgttcgggcg    6780
ttgtgttcgt ttttggatcg ttagtcgcgt agtcgggttc ggtcggtcgt tcgcgcgtta    6840
ttgtgtagtg gagtttggtg gaatttttgt tgacgttacg ttattttta tacggagtag    6900
gagtagaggg aagagagagg gatgagaggg agggagagga gagagagtgc gagatcgagc    6960
gagaaagttg gagaggagta gaaagaaatt gttagtggcg gttagatttc ggaggtttta    7020
gtgtattcgt ggattttttc ggaatttggt atttttagga gttttgtagt ttttttaggt    7080
tcggttttcg ggcgtttgtc gtgtagtcgg aggttcggtt cgttggaaat cgtttcggga    7140
agtagtggga cgcggagata gtagtttttt ttcggtagtc ggtaagtgga ggttatttat    7200
ttcgtaggga tgtgagataa tgcgagtttg gaaatttgtt ttatttcgga gaatttttat    7260
cgtaggtgat ttgtggtttt tggggttaag tttcgtttaa ggtaacgtag tcggtaaata    7320
gattttgtaa agttttgttt ttttcgtttt tcgttataga tattaataat ttatagggtg    7380
ttgaagtcga gagggaagtt agatcgtggt tggtatttaa aacgaggtat ttttttttaa    7440
atttcggtgt taatattgta ggaataaatt ttcgggttaa ggattagtat ttttaagata    7500
aagggttggg tataaagttt tagttattgg aagattagtt ttttttttat tgttatttat    7560
tgggaaaaaa aagaaaagaa aaagatttta ttttaattgg tagttagtga tttttaggt    7620
ttaagcgaat tatttgggag ttaggtttgg atgttaagtt tttattattt ttttggattg    7680
taattttttt aaattgatta ttagttaatt ttaatttggt attttaggag atatatttta    7740
aatggatgta gagaattatt ttttagttgg agattaagaa aaaaattttc gattttaaat    7800
tttcgaaata tgtttttttt ttttagttta attattttat ttttttaagt aatttagaaa    7860
ttaaattatt ataaggtggt gtgatttttt tttatttttt tgtgtgagta ttgttttatt    7920
aaattaaacg gaaaaaattt ttattattat aaatgtaaat attagaattt atatatttta    7980
aaatattttt atgaaaaatt aatttgattt aaagaaattt ttttgtattt gttttagttt    8040
attaattaaa attaaagatg tttttattat ataaaatatt attttggtag aaatttattt    8100
aaaatttaaa tattaataat attaagaaaa taaagtatat aagtaaaata aattgaagat    8160
ttttgttgat gtaatatgag tatataatat tttaataatt aaattttttt taaaaaatta    8220
aatagttatt ttatttgtgg aatgttttat tttaatttag taaaattata tttaaattat    8280
ttaggtgttt tgttttttaa gttaagcgtg tttgttttta aatgttttta aagtatttat    8340
attaattggt tgtaaagaac gtatatatat ggtaaaatat agaattgaat tgagtagtat    8400
tttaattttt ttaaataatt atttattata aattaattta ttggttaatt ttataattta    8460
gtttatttaa aatatatgtt tttgtgttgt ttatttttaa atttttttatt aaagattttg    8520
ttatggggta ataaagtgta tgaaaagggg ggaaatgtga aaggatttgg gattattcga    8580
attgtatttt ttttgtattt ttagttttgc ggtagttatt agaaattatt ttttagtaaa    8640
ttgttttatt ttttagggtt tgtttgtttg ttttgttatg gtttttcgtt tttcgttagt    8700
cgtgtagtgt tttttgtgtg tttataatat aaaatttaag ttggttaaaa taagagtttt    8760
tggtatatat attttaatta gaatatgaat tttgggggtg agaattattt tttattagga    8820
aaagtttttt attttaattt gtgagattag ttattgaagt tagtttcgaa gtttggtagt    8880
taaatttttt atagaagatt tgttttgata gggtaagttt aaggattagt aggcgggaat    8940
tggaggtttt tttttaaaaa attatttttt ttagttattt agatttagtt tttttagtag    9000
gtttggttat taaatgaagt ataaaaatgt aagttttaag gtttattttg attgtaaaat    9060
aaatttttaa gttataagga tatgtaggag tgagttaagg aatacgtttt gatttttttt    9120
ttagttttta gagtggagtt ttatgagttt ttgaagattt gttttgtatt gttttgtttg    9180
gtttttagta ttgaagtacg gggaagtggg gggaagaatg tgtaataatt gattgatttt    9240
```

atattaagta acgtaatttt tttttttgt atattttatt ttttaaaaaa aataaataaa 9300
taaaaattat ttgtagttat tatttgtagt gtttcggtta ttagttaata atgtagttag 9360
tttagatata taaaaaaaaa agattatcga aatgatgatg atatgtaaat ttttttcgaa 9420
attattataa gtaaatattt gaagtttgga ttaataaaat tttatttgtg ttattttata 9480
tcgagttagt agaaagttgt gataatgaat tttgtaatat tttacgaata gatattttaa 9540
ttagggatta attttgtgat tttattgtag aattattaaa tttggagtcg ttaaattgtt 9600
atttttgggt ttacgggttt ataaggatcg aatcggtaga gttttcgttc gcgttttcgt 9660
tagcgggtgg gggaatcgtt tggtcgtttt tattttggat ttttacgtta tagcgtcggg 9720
tagttttttt tgtaggtagc gattttggtt agaggttttt tagggtttag ttttttttag 9780
gagaggtcga gacgtaggga aacggtattt aggttagagg taggttcgta gtttttgtt 9840
tcgtttttgt gttttcgtta attcgataac gtttgttttt atttcgattt tcgtattcgc 9900
gcgaagtggg tttttcggtc gtcggcgtat tttggttagc gtggagagag gtaggcgttg 9960
agatcgaagg ggtttaggga gttttggatt tttttttttt gtttttaaag taatcgcggt 10020
ttttttattt attcggtgga gtttttcgag atttattttt ttcggtttgt ttgtggtaga 10080
gaagggggag cgcgttaaat gtttggttcg ttgcgttgtg gttgaaaacg tgaaaaagat 10140
ttggttcgtt cgggagagaa agggggagaa ttgggtagta gttatattag agttattttt 10200
tcgtttttgg cgggtagtaa attttttaag aacgtttgtt ttgttttttt tagtttcgtt 10260
tagtttattt agtgtttttt ttttgcgatt ttaaattata ttttagggta attatttgta 10320
gtaagtaaat aaatggtcgg gttagtattt ttaggagaaa gtgtggttaa atatggaaaa 10380
gtggtttttg atggatgaga ggttcgaatt tagttcgttt ttgaaatatt ttaggttaag 10440
agttcgttcg ttttagaatt atagaaaatc gagggaaatt gttgtttagg atagggggtac 10500
gttggcgttg atgttttata aatgtttatc gagttttaat taatggataa gtattgaagg 10560
gtggtttttg tatatagttt tttaaagaga aaagtttttt ttatttattt attttcgttg 10620

ttattgcgtt tagatgagtt tttaatttcg gtatcgagat ttttgaaagt aggtttatag 10680
tttttttagt atattgtggt tttatagttt tttaattttt gggtattttt gcggtaattt 10740
tggagggaga tttttttttg ataaataaat gttttgggtt cgaggttagg ttggagatgt 10800
tgttgtatag ttagaggttg ttaggtcgga aaaatacgtt tgaagtttag tatatagtag 10860
gcgtttaata gttagtgtaa cgtagtttta tttgagtttt gtttattcga cggtcgtcgt 10920
tttttatagt tttttttttt ttttgttttg tagataacgg ggaatggaga ttaattgtcg 10980
taaattggtg tcggcgtgtg tgtaattagg taagaatttt tttttttgt tcgggttatc 11040
ggacgggagg tcgcgttacg tgagggcggt aagagggtat tggttttgcg gcgaggtttt 11100
agcgaggggc gtttttttcga ggggttagtt tgggtaggaa ggaaattaga attaaatcgt 11160
tagtggtttt tttttgtggc ggggcggtgg attaggaagt agcggcgcgt tgtgtatcga 11220
agtttttttag tttatttttt tcggttggaa tcgtcggtaa tcggggaggc gtagaaagag 11280
tacgttattt tgtttcgggt tgttagaggg ttcgggggac ggggatgtcg ttagtttttt 11340
tttttaattg ggtttttgtt ttttgttttt tttttttttt cggtttgttt cgtttttttt 11400
ttttttttcg ttttcggttt ttttcggtcg cggttcggga cgtttttttt cgtacgtggg 11460
gcgggcgcgc gcgtggttta ggcgtgtagt cggcggtcgt tgaatgtttt tttttttaaag 11520
atttcgaaat taaaaaggtc gagtttacgg attttttttga gagtcgaaaa gaggtagtta 11580
gtagtaagtt tttttcgcgg tagtattttg gcgttaatgg taaggtcggg agggaagcgt 11640
aggtcgcgcg ttgggtattc gttttcggga ttttgggttt cgggcgaagc gtaagaaggc 11700
gaggtcgtta gatttgacgc gtttgttgtt tgaatttaga tatttcgttt ttgggtggga 11760
cgggaagtag tcgttttagg gacgttaatt ttttttttta aattatattg tatttttgag 11820
atttaatatt tttttttttt tttcgttcgt tttttgtggt ttgatttttt gcgtacgttt 11880
tagtaaattt cggcgtttag gttggcgtgg aaaagtggtt taatagcgat ttttgtcgtt 11940
cgttatattt cgttgcgcgt agtattatta gggtttattt agttatttag gtttttagtt 12000
acgttttatt tagatttgtg ggtgcgcggt ttatcgcggg aggtaagtaa gggaaatttg 12060
agttggcgaa ggtttgtttt ggttggttgg gggagggggcg gggggtcgat aatattttttg 12120
aagagttgga gggtagttat tgtgtttagt agtttagggt agaatggagg tttgtttttg 12180
tcgacgcgaa tttgtttgaa gtattggttg ttaggttttg ggttttggcg atgtcgttgt 12240
ttgattggtt ggttttattt tggaggaatc gagggatatt gttagaggag gtttataggt 12300
ttatgtaaaa agttaaaaag tttttaattt acgttatagg ttttttttga attgaaattt 12360
gttttatggg gcggaggggg gggtgtaagg gatggaggag ggaagatgtt ttttttttta 12420
aatatatgga aaaaaatttt ttaaatttat tgttttttta ttttttttggt ttcgtagtaa 12480
ataagtgttt agttttagga ggttattgat ttttgataat gtgagtagat aaagtttttt 12540
tttttttata gttttcggtt tttaattttt ttttttcgga ttaaagtgta agaatataaa 12600
tgtaatatgg gatggagggg ggcgatttgg gatttcggtt aaaaaaataa atcgtattat 12660
taagaagaaa ataaaggttt tgtattggag ttttttcgtg aatttgagag aaaatgatta 12720
tttgttgaaa tgaagcgttt aaagcgattt agtgttttac gttcggatat tgtattatat 12780
cgttagtcgt tttgttgggt tagttaaacg tttatttgtt cgggattaat tttatggggt 12840

```
taaatggggg taatgtagag ataacgtcgc gtgattttg ttatttagat tgtgttaaat   12900
tttttttttg tttgataatc ggtagtaaaa ataaattatt agatcgtagt atgtttggga   12960
tatggttaaa aattaagagt agcgatgatt tttggggaga atgttttgcg cgggtttagt   13020
tttggtttcg gttagattag aggagttttt taatttcgtt tcgcgcgggg cgggttcgta   13080
gtcgttaagt cgaggttgat attttttatt gtgttgggag ttagagagac gtaaaatgtt   13140
tttttttttt agtttttatt ttaggttttt tagatatggg gaatgtattt tgaggatagg   13200
tggagaagtt tacggtagga tggggttttc gtaggtgagt aggaaacggt taagagtaga   13260
ggagttttgt ttgcgttagt tataagtcgc gtaggcgttt ttggtcgttc gtttttgata   13320
attagtatat aaagaattag aaataatgaa tgattgtttt tttaattatt atttttaggt   13380
tcgtattgtt ttagtgtacg tgaaaggttt ttttttata tttaatatgt ttttttttat   13440
tttttgatcg aaaagaaaaa ttgttgttta aatacgttta atgttattaa ttaagaaaag   13500
gtatgtaatg ggaagaaatg ttgaaaattt tgatttaatt ggtttttaag gaattagtag   13560
acgataaaaa aaaattatac gagtgggtaa agttatagta ttgttgaagg atagagtatt   13620
tatttttttt tgattttaag ttaatttatg gaatatttaa agttttggtt atagtttgtt   13680
tgtaaaataa aaggatttat tttttgtgtt tttttaaagt ttttttttgt ttttaaagag   13740
aaaaaaagtt tataatgata tatgattttt ttaaaaggtc gtgatagttt attacgttat   13800
ttttttcgtt tttgttttta acgtcgttta aaaatattat gttttcgtta aagattaaac   13860
gttttgtata ggtagagttt atttttaagt agtttaggtt ttgttttttt tttttagtga   13920
gttttatttt ttttggtatt tattgggcgg atgtttagtt tggatagaat ttcgaaacgg   13980
gggtagtacg agagcgattg gagattttta aaagttagag gtttgagaga gggtggacgt   14040
agttagtaga agatggtgta gaagttagtt gagaacgatt ttttagagta aagagatttt   14100
tttttggttt tttttgtttt ggggggtttg aaaggaattt ataaaatggt ttttattttt   14160
aggaggagga cggattgatt ttttttttgtt attggtttaa aaagttttag ggcggcggtt   14220
ttgggtttcg tgttgaaatc ggattgtatt gtagtttttt tggatttgac gtttggtttt   14280
gcgtttcgat aaggggcggg tatttttttc ggtttttttt aggaacgtat taattgttaa   14340
atagttttgg tttagtggat gggttgaaag tgttcgattt aagtcgttgg tgtgtataga   14400
tttttttttt ttgggaggtg ggttttatgg ttcgttgtgg tatttttagt cgcgatatat   14460
atttttatac gcggtagtag ttcggtttta atttttttcg aaggatttgg gttaattttg   14520
gcggttttgg cggtcgtaga tttttttttcg tcgtttcgtt ttcgcgtttt ttatttaatt   14580
agcgaatgtt tgcggagtat atattacgtg gatttttaat gtattttttg aaagtaaata   14640
atatagtttt tttcgtcgtt atgaagggat tttaatttta atatggatat tagcgagatt   14700
agtttagatc gtttttagta aaacgtaaaa tggtggtgcg tggggtggtg attaaggttt   14760
tgagttttgt tagaaagaag gggatgtgta gagaaaggtg gagaatttta gttgtggtta   14820
gcgcggaagg gataggtgtt tgtcgaaggg ggtatgaggt ttgaggaaaa agtaacgaaa   14880
taggggtaag gagagttttt tatttttttt ttttcgttcg attttcgtta ttttattttt   14940
tttttttttt ttttattttt cgcgttaatt aaatttgtag tttatttgaa aggtgtttcg   15000
tcgcgttgtg gttttttatt tttaggggaa attgtattag ttgttcgaaa gtagttagtt   15060
tttgcggatt tttgttcgta aaagtggttt ttataggtcg cgtttttcgt tgttgattcg   15120
gtatataaag ttttttaagg ttggttcggt tgttattttt tatcgttcgt tgttaatata   15180
tgtagtagtt gttagagcgg ttcgggggaa aaggaaatgt ataacgaaag tttattcgtg   15240
agtaggaata tattaatgga ataatttgat gttttttttaa ttttatgtaa aaagttttgt   15300
cgttttttta atattgattg aatgggtaat taatggtttt ttatttaggc gaatattttg   15360
taatttaaga taggtaaaag ataataagtt taaggtagaa gataaaaggt ttaattgtag   15420
cggcgtttgt tcgtttttta ttttttaggg tttttgatta ggaaagtttt tttttagagg   15480
agaaaaaggt aggagtggga gaatatatat ttattatttc ggggttagat tttatcgtag   15540
tatttgatta tttagtttag ttttttgtta ttttgttttt ttattttttag tttttttttt   15600
tgtatttttt ttttttttta attttttttag gatgattttt tattattatc gttattatgg   15660
ttttaataat tttttttttt aaattttata tttttttattt tagtaattaa tgaggttgtt   15720
ttttgattta ggaggagatt tttttttttt agaatttaat gcgtagagtt tttgagaatt   15780
aaagtagttg gtaggggagg aagaaattaa tagaaaggga gagagtatat agaattgtgt   15840
gtgtatgtta aagagcgatt aggaatgata gagttaattt ttttgtgagg atttgacggg   15900
aagagtgttt aagattttat tagtatgttt ttaataggtt gatattttaa tttaaatttt   15960
tagaagtaat atattatttg ggttattata atgaggtggg tttttttttt tttgttagtt   16020
gatagttttt aaaatattat ttcgttaggg aaataaaagt tttattttag attataggtg   16080
ggtatttttg gatttaggtg atttatggtt attatgataa ttaatgttga atgttagtta   16140
ttagtatgtt tgggagagag aaaatagaaa gaagggagag taaaagaaat agaaaaggga   16200
gatggatata agttggagag ggaagaaaag agaaaaagag gaagatagat gagtgtttaa   16260
tttaattgtt gtttaaaaaa gtggcggggg ggtgggattt tatttagttt tttgttattt   16320
tttttttttt tgatttggat atttatgttt aattttatat tttatttttt tttttttttt   16380
tttaaatata tgtgttatat tatttttttt attttattta gttcggtaag tagttgtttt   16440
ttggagattt agtgatattt aggaaaattg tggtagtaat atgtaaatgt gaggaagtat   16500
```

taatagtatg ttcgttgagt gattttagta aatgtttttt tttttaattt tttttttttt 16560
tttttttag gttattgtga ggtggtaatt tttattgtta tttgaatatt gtttttttag 16620
gtagttattt taaattttaa atggttgagt agttagagtt gtgggttgga aaaatgggta 16680
ttatttgtag ggatttagag agggtggttg ttgtttaata tatttataga tttttaattt 16740
agaaaataat tttttttttt tgataagtta gagtttttta aattttattt aggaaatggg 16800
gaaaaggata gttatagtga agtttttaat ttttgggtta tttggtttta tagttatgag 16860
gggtggtggg tagtggattg tttttagttt ggtttgtatg tagagaaaag ttagatattg 16920
gaggggggtgg ggtatttttc gggtaggatg taaggttttt atttgatttt tgcgttttat 16980
taggagttta tatatttacg tttattacgt ggttttaagt tgagtttagg cgggtttcgt 17040
ttttgagtta gtttgggtag ggtaggattt ttattcgttt aaggtttaat agtttaggga 17100
gatgtttaat taagttattt tttgggtgaa ttttgaagat agattttttt taaaagttag 17160
agattatttg gttgagtttt aggttagatt gatacggaga gtttggcggt atagtttaat 17220
cgtttattgt tatggttaga gggattttgt ataattaata ttgaagagtg tgaaattaaa 17280
taagatttta agattggtaa tcggtggtaa atattagtat aaaatacggt tgattttatg 17340
ttatatattt ttttttttta gggttttttt ttgaaagaat aagtaagaaa ttttaatcga 17400
gataattttt gatgtttttt agatttaaaa ttttacgtcg gtattgggtt tttttttttt 17460
gttttacgtg agttatgtag tatttttagt ttttttatta ggattttatt aatgtttttc 17520
gtattggaaa tttttgtgtt agaggttgaa tttatagtaa tttttaaaat taattaagaa 17580
gaatttagtt agaggttata gtaatgttgg aattataaaa tgtataagat ttattttttt 17640
ttggtttttt ttttatttat gttgtttatg tttgtgtatt tataagtttt atgtatatta 17700
aatttttaaa attaattatt attatgttat agagttttta ttggatagtg ttttttagtt 17760
tttattatat attttttttt ttttatgtag atttattatg ttggtgtttt gttatatagg 17820
gggtttgaga agaatgttat ttaatcgtcg ttgttgtgag tgtgtaaagt gattaggaga 17880
ttaggagaat gttgaaattt ttgttggaaa aatgtaaaga aaatttttat tttgagttag 17940
ttgtttatag agttagtgtg tgtgtgcgtg tgtgtgtttg taatataaaa tggatgtgaa 18000
tatatatata taaatagata tggttttgtt tttattttaa tttgaattat ttagataatt 18060
gtttttattt attatttgat tttaatgggt ttatataaat taggatattt tatttttta 18120
ggtatttagg ttgttgttga tttttagtgt ttttaatatt tcgtatacgt tggtattatg 18180
aggagtagtt acgtgttttt gggtttttta attattttgg aggttgattg aggtttttta 18240
tatatgtata tttgtcgtga tgaaagtttt atcggtagag tggagttatt agagttttta 18300
ttaaaatttt gtgggtttat gagagatggg tttagaaatt tatatggttt tgtggggttt 18360
ttcggttttt taaaataagg tattaatatt taagttttta aaaatatttg tagttttggg 18420
gtttgaattt tgaaaaataa ggagtgaggg gttgtgtata ttaattatag tggagatttt 18480
ttttattttt taatgtgatg gagttttttt atgaaatgaa gttttaaggg gtatggtatt 18540
gtggggatta tagttatttt gaggtttaaa agaagaaatt ggaatatgat tagtaaatat 18600
atttagtaga aaagagttgg atttttattg atttagttat aggttatcgg ttggtagtgt 18660
aatgggagga aatatttatt ttatatatat attttatgat tttgggggaa ttagaggaaa 18720
tttaataaga aaacggttag aaatatttaa aatttttatt taaaagattt aagtaaatta 18780
gagttttatt agattaaaaa ttattataaa tgtaagagta ttgtttttag tgaaacgttg 18840
tggggtttga gaaggagatt tttcgttaaa ttttcgggat aaaatgcgtt atttaagtat 18900
tagataatga gtagaatgta aattaattta attttttttta ttaataggtt gttagtgtaa 18960
tgtgtataat ttagtgataa gattgtagga tttaatatag ttggatgtat gagttttagt 19020
taatgtagat ttgttatatg aggatgtgtt ttattttgag taggtgtttg tatgtgtgga 19080
atggggtaaa gtggaataaa aggttaaaag tagaaatgtt gatttaaagt ttattatgaa 19140
gaaatttttt ttttgtagtt aaattatttt taaagtggga tgatattggt gaagaaagat 19200
tgaaaaataa tttttatgtg cgtttttgga ttgtaagttt aaaatgggga ggagttgtag 19260
atagggtttg ggggtggtta gggtaaagga gagatatata agttgtaaat atatttgtag 19320
tttgttttat ttattttgtt ttatatcgaa taagtttttt aattttgtga ataaggataa 19380
ggagggagtg ttttaaagat attttatgtt ggtattgtaa attattgatt gtaatgttaa 19440
ataaatatat atttagagat gataatatta attttatagt aaaataatcg tttatgtaga 19500
aatttagagg agattagttt gtttttttta gttgatttat gttgggggat aaaaggattt 19560
ttaaaaatta ttttgaatat gtttggattt ttttttttaa tttttttgga aattaaattt 19620
gtttggaaat agtgttataa agagttgatg tttttaaagg tgattttttt tgttttatat 19680
aaataaggtt ttgtttttgt tagttgagcg tagttttagg tttttcgttt ttagtttata 19740
tatatttttt ttgtttgttt ggattttaat ggtttaagat agttttgagt ttattgggaa 19800
aagaaaatga ttgttaaaaa ttatttttga aattggttat ttggtaatat ttttaattgt 19860
atggaaattt attaaggtat attttatata taattagttt aaggttgttg attttatagg 19920
ttttatggat ttaaatttga ttgataataa agtaaataag agagtcgaat ttaaagcgtg 19980
gtttttttcgg gttaggacga gtttaatata gtgtataagg aatttgaaag atttaggata 20040
tgtgttttaa ttaacgttaa gtagaatgga taagttttta gtattttgaa aacgttgggt 20100
tagggttttt tttttattgt gtgtttttttg tttggggatt aataagtatt atagagaacg 20160

tgatttgagg cgatttttta tttttgtata aatttagagt gaattattaa atagttgttc 20220
gtttaaagtt aaggtaattt ttttttgacg ggtttatttg tttttcgatt tttaatttat 20280
tagtttgttt ttttagggtt ttgttttttt tgtaattaaa gttttttttag attagcgtag 20340
tatttatttg ataggttgtt tggaaaattt aagatcggag aggtgatttg ttgttgtttt 20400
ttaaattttt tagttttaag taacgtgttt ttttttttata tggggtgggg gattggaaat 20460
ggatgtagtg agatataaag agtgggtgtt ttgttgattt ttgtattttt ttttttttga 20520
ttattttatt tttttttttt aagttttcga tttttagttt tattttttta tttttgggtt 20580
cgtattaaaa gtcggatcgt tttgggttgg gtaggagttg aattttcggg agtttgtttg 20640
tgtagattta gtgcgtacgg cgaggtagta gttcggtttc gtattgttga taggtgtagg 20700
taggatagtt tttttatcgc ggttcggggc gttttgattg gtgcggagtt acgttagtcg 20760
tattcggaga agggtttggg aggaggcgga ggcggagagg gttggggagg gtcgcggcgg 20820
agtgacgttt cggtattagg aagttcgttt ttggttttaa gatgttaggt taatagggaa 20880
gcgcggagtc gtagatttgg ttcgtcgttc gtttgggtgt ttggagttga gttgcggtaa 20940
ggttcggttt ttgttcgatc gttcgagggg tgtgcgtgtg cgcgttgcgg agggtgcgtt 21000
tagagggtcg cgtcgtggtt gtagcggttg ttgtcgtcgt aggggattta atattattta 21060
tttgtttttg ttatttttga tattttttttg ttagggttgt cgcgtggggg gggggcgggt 21120
agagcgcggt cggcgttagt ttttttttatt ggaggggttt ttgggggagg gagggagaga 21180
agaagggggt ttttgtttat ttttgtttcg ttttggagtt tggaagtttg ttttttaaag 21240
acgttttgag tggtgttttt ttgtttatat tttatgtttt cgtttgttcg ttgatttttc 21300
gttttcggat tttttcgttt gagtttttcg gaggagacgg gggtagtttg gtttgagaat 21360
tcggcggggg ttgcgttttt tggttttttt cgtagcgggg aaatttcgcg tttagagcgc 21420
gattcggagc gggtagcggc ggttacgggg gttcggcggg gtagtagtta aggattagta 21480
gagcgtcgcg ttttttcgtt tatgaattgt atgaaaggtt cgttttattt ggagtatcga 21540
gtagcgggga ttaagttgtc ggtcgttttt ttattttttt gttattattt ttagtcgtta 21600
gttatggttt cggttttggt tttcggttag tttcggtcgt tggatttttt taagtatagg 21660
ttggaggtgt atattatttt cgatattttt agttcggagg tcgtaggtaa ggcgtcgcgt 21720
cgttttgtag atattttcgt ttagttgttt tgcgttattc gttttttttc gttttaagga 21780
agttagtttt ttcgggggga ggcgtggtgg gagtggtcgt tcgtttggtt tttcgtagaa 21840
ttttcgggag tcggaatttt gattatttcg tattttttta gttttttttc gatcggttcg 21900
gtttttgggg cgttaagggc gcgagtaatt ttgtcgtttt ttttattcgt attttggttt 21960
ttttttttgtt ttttgggtta taaaaatttt agtattttga ttcgaggatt tttagaggtc 22020
gtcgattttt gtttttgttt ttttttcggt ttttagtttt cgaggagttt tattcgttag 22080
gaaattgttt gaaattattt agaaatgttt ttcgcgaaga ggtatttttt tttttttttt 22140
gggaaagggt cggcgaattt cggtgtttaa tcgaattttt atattttttt ttagtttttt 22200
taaatcgtat ggaaatttga gtttttttgcg agggggaggg gggtttgtaa attacgcgcg 22260
tgtgcgcgtt ttaggagatt tggtgtgttt gcgtagaggt gtataaatat atttgaaagt 22320
ataggttata aaagtgaatg tgtcgttgta gtgagataaa tatgtaaata aaacgtgcgg 22380
cgttggggga ggggaggaaa tggggcgcgg atatttatat ttgcgtttgt atattttata 22440
ggcgtagcgt ttttcgcggt tcggagtcgt cgcgcgtatt ttttttcggc gttaggtagt 22500
ttagtttttt tacggttttt gtcgtcggtt tagttggcgt tcgcgttgta ggtgggtatg 22560
ttgacgggaa agtgtgtgtg tttcgttttt agagaaagat aaaagttagt aggggaagaa 22620
tgaggacgtg ggcgtcgagg attcgtttaa gaagaagcgg taaaggcggt agcggattta 22680
ttttattagt tagtagtttt aggagttgga ggttattttt tagaggaatc gttattcgga 22740
tatgtttata cgcgaagaaa tcgttgtgtg gattaatttt acggaagttc gagttcgggt 22800
aggagttagt acggagtttg ggagggatgg ggggaggatg ttgtggaggt ataggttaag 22860
tagattagga gagaatgtgg aaggtagcgt cgtttgggag ggcgtcggtg gggcgtagtt 22920
ttgtaaaggt agaaggtttc gcggcggttt ggttgcgaga ttatagtttt tttttcgagg 22980
tcgataggat tgtcgttttg gtttaggttt ttagagcggt atcggtttat tgtttcgtta 23040
tttcgcgatt ttacgagttg ggttgtatgg gtaatttttt gtataggata ttgtgttttt 23100
ggtttgtagt tgttagagta gagttaataa aatttttatt aggttaagag tcgcgaatag 23160
gttttaattt gtgagttttt aataaggaaa attcgttaga gatacggaag agttggtttt 23220
ttttgggaaa tttttgtttc ggttttggtt tagttttttt ttttttgggt tcgcgttttt 23280
tatatttttt ttacggttgt ttcggttatt taggtttttt ttatatattt tattttttag 23340
ttttgtgatt ttcgggagta aagttttaat atataattat tagttttttt agaaggagaa 23400
agaaaaaaag aagaaagatt tttttgtttg gtttatttat ttttttttag gagttgaatt 23460
ttggaaattg aaatttatat tttttttttt aaattataat tatagttttg taaaaagggt 23520
ttattttaat tttgtagtaa atttgtattt tatggattgg taaaaatgag tttaaataaa 23580
taatttaata gtaacgtttt ggtttatgtt ggtcggtgga agattttaaa tttgttagga 23640
ttttggaagt agaaaataga attaagtaaa ttaagcggta tttagaggtt ttgttgttaa 23700
aaaaaaaaaa ttaagtgttt tgggtagaaa aaataaagtt ttcggttaga gtagagtaaa 23760
taaaaagaag aaaataacga taaaaagaat aaagattaaa atgttttttt aaattagagg 23820

```
gaatgaagat atttttttggg tggtatttgt gtaaggtatg aggttatgtt ggtggataaa   23880
aggtcgggaa gaagttgaaa atggttttag tttaattgtt tagagttaga gttgggtttt   23940
gggcggcgtg gttttgagta aggttagttt tttattagtt tttttgtata ttaagggaac   24000
gggtttttta cgtatttttt tcgtttgagt aaagtttaga tggtttaggg tagaaatggt   24060
aagtaattaa agatagagtt tatgggtttt ttgggatttt tcgaaaacgt tttttttattt   24120
cgttcgttat ttcgtagttt tattttagtg ttttgtagtc gcggcgttgg gtttttttttg   24180
tagttgtttt tttttttagg gcggttgttt gtcgagttaa gtgggagtga ggcgtgtttt   24240
ttatagtagt cgggtgtaaa gaggaagggg gataaaaagg aaattaagaa tgaaaggaaa   24300
aagagaaaaa gcggattata cggttgggtt cggcggagat gtgtaatgtg aaatattatt   24360
ggtgttagtt cggatatttt aggttaggtt ttttttttaat atataaaagt cgtcgtttgg   24420
ggcgataggg aggttcgatg tggattggga tcggggttgc ggttgggtta tcggatacgg   24480
gtggaagtcg gtcggtttgg gtggtcgttt gtaaagttaa acgattcggt tgggtttggc   24540
gcgcggatag gtttgtggtg ggtttagggt aaagaagagg tagagcgaaa gaaggggggaa   24600
tttttaaaat tatttttttc gggttttcgg agtttaatat gttaagtttt tggagttaac   24660
gagttgacga agaggtggtt ttttgttttt tatttggttg ttttgttagg cgagaaagag   24720
tgttggcggt ttagtttttg ttaagggagt acgtattagg gggtggggga cgatagtgga   24780
ggttagggaa ggaagggagg aattgcgtgg gagaaagagc gatttttttag tgtttttttta   24840
gtttttttttt tttattcgtg ggtttgtggt tttggaatgg aagtaagttt gtaaggtgtt   24900
tcgggaaggg ttggaaaagt ttgttgtttc gcgtttgttt tatattaagt gtttttggat   24960
ttggagaaac gtttggttga gtgattaaat cgttcgtagg tttttatgcg ttcggttgag   25020
gtttgtggcg tagtttcgag ttttagttcg taggttagag tagattaggt tttttgcgtt   25080
tggtggagat tcgggttagt aattgaaagt tggttttggt attttggtgt gtagggcggt   25140
gtagtgaagc gaggttaggg tgtgtgagtg cgttagcgtg tgtgtcgggg gaaggcgggg   25200
gttggttttc gatggaagtt ttagtaattt gtattgtggt atttgtttgt tttttttgttt   25260
taatcgtttt taggtttggt ttaagaatcg tcgggttaaa tggagaaaga gggagcgtaa   25320
ttagtaggtc gagttatgta agaatggttt cgggtcgtag tttaatgggt ttatgtagtt   25380
ttacgacgat atgtatttag gttattttta taataattgg gtcgttaagg gttttatatt   25440
cgtttttttta tttattaaga gtttttttttt ttttaatttt atgaacgtta atttttttgtt   25500
attatagagt atgttttttttt tatttaattt tatttcgttt atgagtatgt cgtttagtat   25560
ggtgttttta gtagtgatag gcgtttcggg ttttagtttt aatagtttga ataatttgaa   25620
taatttgagt agttcgtcgt tgaatttcgc ggtgtcgacg tttgtttgtt tttacgcgtc   25680
gtcgattttt tcgtatgttt atagggatac gtgtaattcg agtttggtta gtttgagatt   25740
gaaagtaaag tagtatttta gtttcggtta cgttagcgtg tagaattcgg tttttaattt   25800
gagtgtttgt tagtatgtag tggatcggtt cgtgtgagtc gtatttatag cgtcgggatt   25860
ttaggatttt gtcggatggg gtaatttcgt ttttgaaaga ttgggaatta tgttagaagg   25920
tcgtgggtat taaagaaagg gagagaaaga gaagttatat agagaaaagg aaattattga   25980

attaaagaga gagtttttttt gattttaaag ggatgttttt agtgtttgat atttttttatt   26040
ataagtattt ttaatagttg taaggatata tatataaata aatgtttgat tggatatgat   26100
attttaatat tattataagt ttgttatttt ttaagtttag tattgttaat atttaaatga   26160
ttgaaaggat gtatatatat cgaaatgtta aattaatttt ataaaagtag ttgttagtaa   26220
tattataata gtgtttttaa aggttaggtt ttaaaataaa gtatgttata tagaagcgat   26280
taggattttt cgtttgcgag taagggagtg tatatattaa atgttatatt gtatgttttt   26340
aatatattat tattattata aaaaatgtgt gaatattagt tttagaatag tttttttttggt   26400
ggatgtaatg atgtttttga aattgttatg tataatttat tttgtgtata atatttcgta   26460
taatattatt gttttatttt ttagtaaata tgaaataaat gtgttttatt ttatgggagt   26520
aaaatatatt gtatataaat tggtttggat tttttttttt tttttttgtt attaatttgg   26580
ttaggatatt ttagttattg tttttaaat aaattagttt tttttgtttg tttagttaaa   26640
tatataaggt agtagttttt atttaaattt ggtagaaata aatgatagtt atttattaga   26700
aattaaaaag aaaaaaaaag gtattttcgg gggggaaaag ggttataaaa tttaattttg   26760
ttttttttaat tttttttttgg tttaaattta gaggatttta ttatggttag taaataatat   26820
gaaaaagaaa aaagaagaaa gaaatttagt aagtttatta gtttaaaatg atttttaagt   26880
ttatttttttt acggggaaat ttatattttt agtaaattgt tttggagaaa tatttgtgta   26940
tgtatatatg tatagtttat atgtattttt tttaggagga atatatttat aataaattta   27000
tagggaaata tttttagttt aaaatattta ggtttttacg tttattttta ggtttaagta   27060
gagagatttt ttatgttata ttgtattatt atttttaaat tttttggaga tattaaaaga   27120
aataaagatg atttttaata attatagttt tttagttttt taaagaattt aggggttgag   27180
aggttagagt ggagtttttt gagttttgtc gagtaatatg tagttgaggt aaaggttatg   27240
ttttcggtgt tttgttttaa ataatattga tttattaatt ttaaatttgt ttgtttttga   27300
aattatatag gattatagtt tgtaaattgt aggataatga agtaaattaa gatgaattat   27360
agttttggtt ttttttgtta ttttttgata tttaaatagg gaatgagttc ggtgtgagtg   27420
```

```
tttaaatgaa ttttaagtat tcgatttttt tttattcgcg atttttagtt ttaaaaaaat   27480
gtgaaatttg attttataat aaatagaaat aaatattatt tagttttaga gaatttattt   27540
ttatggcgtt aggagggtcg ttgtggaggt ggggggaggg atgtgttgag attttttgtt   27600
atgtttgtta atttttcgta taattaaagt gggcgagaat aaatattacg ttggggaatt   27660
tagagtaaaa agtaatcgtc gattttttgg agtcgataat attattgttt tttcgtttta   27720
gttgttttta tttgaatgaa attttattta gaagtttttg gagttcgaat attgagtttt   27780
tgttttgtaa gaaattgttg ttgttattta aagagatcgt agataatgtt ttcgatttaa   27840
gattagtgtt taacgtatat tttttttttt tttaaagttt tgttttcgat ttgcggaggg   27900
attacgtagt agtggggggt agataaaagt tttttggacg agggttattt tttattatgt   27960
tgtttatttg agagtagtgg agaggaaaac ggtttttacg ggcggatttt ggtttttgga   28020
gtcgtagggt ttagcggttt cggtttttttc gtttttttagt ttggtagggg gcggggaggg   28080
ttaaagggcg gaggggaagg aaggagttag aagaggggat ttggggatgg gggttggaag   28140
cgttaacgag atttgttcgg aggatttagg ttttttgtag gttggtgagt gatttgggag   28200
ttttgggtaa aagaggtgta tttcggttta gtttggttgt tgaattagaa taacgcgagg   28260
atattaatta atttgtagga aataaaaaat ggaagtcgag gtttaggaag agttgttatt   28320
ttttcgattt gagtggtgta ggttgggggc ggagatttgg gatttaagag aggttatttt   28380
ttttatttta gttttttttt tttggatttt taaaaggaat aattttattt tttttttcgt   28440
ttttttttata atttttattt tcgttagttc gtaggtcgtt tgtttttttt cgtatttttt   28500
tttttttattt agcgagagaa atttagtttt tagagt                             28536
```

<210> 964
<211> 28536
<212> DNA
<213> Artificial Sequence

<220>
<223> chemically treated genomic DNA (Homo sapiens)

<400> 964

```
gttttgggag ttaggttttt tttgttggat gggaaaagga ggtgtggaaa ggggtaggtg      60
gtttgtgggt tagtggggat gagagttgtg gggagaatgg aggagagagt aaaattattt     120
tttttggaag tttaagggaa gggagttgag gtgagagaaa tggttttttt taggttttaa     180
gtttttgttt ttagtttgta ttatttaagt tggggaagtg ataattttttt ttaaattttg     240
atttttattt tttgtttttt gtagattgat tgatgttttt gtgttgtttt agtttagtaa     300
ttaaattagg ttgaggtata tttttttttat ttagagtttt taaattattt attaatttgt     360
aggagattta aattttttgg gtaggttttg ttagtgtttt tagtttttat ttttaaattt     420
tttttttttag tttttttttt tttttttgtt ttttggtttt ttttgttttt tattaggttg     480
agaggtggag aggttgggat tgttgggttt tgtggtttta gaagttaaag tttgtttgtg     540
gggattgttt tttttttttat tgtttttaag tgaataatat ggtgggaggt gatttttgtt     600
taaagggttt ttatttgttt tttattgttg tgtgattttt ttgtaagttg gaggtagagt     660
tttaaaaaga aagaaagtgt gtgttgagta ttgattttaa attggggata ttatttgtga     720
tttttttaag tggtagtagt agttttttgt agaataaaga tttagtgttt gagttttaaa     780
agtttttagg taaagtttta tttagatgaa agtaattaag gtgaaaaaaat aatgatattg     840
ttggttttag aaaattggtg gttatttttt gttttaagtt ttttagtgtg gtgtttgttt     900
ttgtttattt tggttgtgtg gggggttgat aagtataaata aaagatttta gtatattttt     960
tttttttattt ttataatgat ttttttagtg ttatgaggat gaatttttttg ggattaagtg    1020
gtatttgttt ttatttgtta tgaaattaaa ttttatattt ttttaaagtt gaaaattgtg    1080
gataaagaag gattgggtgt ttaaagttta tttaaatatt tatattgaat ttattttttg    1140
tttagatgtt agaggatggt agggagggtt agggttgtaa tttattttga tttgttttat    1200
tgttttgtag tttgtaaatt ataattttgt ataattttag gaataagtag gtttagaatt    1260
aatgggttaa tattatttaa aataaaatat tgggagtatg atttttgttt taattatata    1320
ttgtttgata agatttagga aattttattt taatttttta atttttgagt tttttgagaa    1380
attgaagggt tgtagttatt agaaattatt tttgtttttt ttaatgtttt taaaggattt    1440
ggaaatagta atgtaatata atatgaagga ttttttttatt taagtttgaa gataaatgtg    1500
gaagtttaaa tattttgaat tggagatgtt tttttgtaaa tttattatag atgtattttt    1560
tttgagagaa atgtatataa attatatatg tatatatgta taaatatttt tttagaataa    1620
tttgttagag gtgtaggttt ttttgtaaag gagtaaattt gagagttatt ttaagttgat    1680
ggatttgtta aatttttttt tttttttttt tttttttatat tatttgttag ttataatgga    1740
atttttttagg tttaagttaa agaaaaattg gagagataaa attagatttt gtagtttttt    1800
tttttttttgg gaatgttttt tttttttttt ttagtttttg atgaatggtt attatttatt    1860
```

```
tttattaaat ttaaataagg attgttgttt tgtatgttta attaggtagg tagagggaat    1920
tggtttgttt aggaagtagt gattgagatg ttttggttaa gttagtgata gaggagggga    1980
gaaagaattt agattaattt gtatgtagta tattttattt ttatgaaata aaatatattt    2040
gttttatatt tgttgaaaag taaaataata atattgtatg aaatgttata tatagggtag    2100
gttgtatata gtagttttag aaatattatt gtatttatta gagaaattat tttaaaattg    2160
atatttatat atttttata ataataataa tatgttagaa atatatagtg tggtatttag    2220
tatatatatt tttttgtttg taagtgaaaa attttaattg tttttgtata atatgtttta    2280
ttttaaagtt taatttttaa aaatattgtt gtgatattat taataattgt ttttataaaa    2340
ttaatttgat attttgatat atatatattt ttttagttat ttaaatgtta ataatgttaa    2400
atttaaaaaa taataagttt atagtaatgt taaaatgtta tatttagtta aatatttgtt    2460
tgtgtatgtg tttttgtaat tgttagaaat atttgtagtg aaagatgtta gatattgagg    2520
atattttttt gaaattaaag gagttttttt tttgatttag tggttttttt tttttttatat    2580
agtttttttt tttttttttt tttttagtgt ttatgatttt ttagtataat ttttagtttt    2640
ttaagggtgg agttgtttta tttggtaagg ttttaggatt ttggtgttgt gggtgtggtt    2700
tatatgggtt ggtttattgt atattggtaa gtatttaggt tggaggttgg gttttgtatg    2760
ttggtgtagt tgaagttgga gtgttgtttt gtttttagtt ttaggttggt taggtttgag    2820
ttatatgtgt ttttataaat atatggagga gttggtggtg tgtaaggata ggtaggtgtt    2880
ggtattgtgg aatttagtga tgggttattt aggttgttta agttatttag gttgttgaga    2940
ttggagtttg ggatgtttgt tattgttgag ggtattatgt tggatgatat gtttatggat    3000
gagatagagt tgggtgggga aaatatgttt tgtgatgata gggggttgat gtttatagag    3060
ttgaagaagg ggaagttttt ggtggatagg gaggtggatg taaggttttt ggtggtttag    3120
ttgttgtagg aatagtttgg gtatatgttg ttgtagggtt gtatgagttt attgaattgt    3180
ggtttgaagt tatttttgta tagtttggtt tgttggttgt gttttttttt tttttatttg    3240
gtttgatgat ttttgaatta aatttggggg tggttggggt aagggagtaa atagatgtta    3300
tagtgtagat tattaaaatt tttattggag gttaattttt gttttttttt gatatatatg    3360
ttagtgtatt tatatatttt ggttttgttt tattgtattg ttttgtatat taagatatta    3420
gggttagttt ttagttattg gtttgggttt ttattaagtg taggagattt ggtttgtttt    3480
ggtttgtgag ttgggatttg gagttatgtt ataaatttta gttgaatgta tggagatttg    3540
tggatggttt gattatttag ttaggtgttt ttttaggttt aaaaatattt aatgtaaaat    3600
aaatgtgggg tagtaggttt ttttaatttt ttttggggta ttttgtaaat ttgtttttat    3660
tttaaagtta tagatttatg gatgaggaga aggggttgga agggtattag aggattgttt    3720
ttttttttat gtaatttttt tttttttttt ttgattttta ttgttgtttt ttattttttg    3780
gtatgtgttt ttttaatagg gattaggttg ttaatatttt tttttgttta gtaaaataat    3840
taaataaaga gtaaaagatt atttttttgt tagtttgtta attttaggag tttggtatat    3900
taaattttgg gaatttggaa agggtagttt tggagatttt tttttttttt gttttgtttt    3960
ttttttattt taagtttatt ataggtttgt ttgtgtgtta ggtttagttg ggttgtttgg    4020
ttttgtaggt ggttatttag gttggttggt ttttatttgt gtttggtggt ttagttgtaa    4080
ttttgatttt aatttatatt gggttttttt gttgttttag atggtggttt ttgtgtattg    4140
gagagaggtt tggtttgaga tatttgagtt gatattagtg atgttttata ttatatattt    4200
ttgttgggtt tagttgtgta atttgttttt tttttttttt tttttatttt tgattttttt    4260
tttatttttt tttttttttg tatttgattg ttataaaaag tatgttttat ttttatttgg    4320
tttgataagt agttgttttg gaaggagagg tagttgtaag gagagtttag tgttgtggtt    4380
ataaagtatt agggtggagt tgtggaatag tgggtggggt gggagggtgt ttttgaagga    4440
ttttagaaaa tttatagatt ttgtttttaa ttatttgtta tttttatttt aggttattta    4500
aattttgttt aggtgagaag agtatgtgag aggtttgttt ttttgatgtg taagagagtt    4560
aatgaaagat tgattttgtt taaaattatg ttgtttagga tttagttttg gttttggata    4620
gttaaattaa aattattttt aattttttttt tggtttttta tttattagta tagttttatg    4680
ttttgtataa atgttattta gagagtgttt ttattttttt tgatttggga gagtattttg    4740
gtttttattt tttttattgt tgtttttttt tttttgtttg ttttgtttta attgggggtt    4800
ttattttttt tatttagagt atttaatttt tttttttttaa tagtaaagtt tttggatgtt    4860
gtttgatttg tttgattttg ttttttgttt ttagaatttt aataaatttg gaatttttta    4920
ttgattagta taaattagga tgttgttatt gggttattta tttgagttta tttttgttaa    4980
tttataaagt atagatttgt tataaagtta aggtaagttt tttttataaa attatgatta    5040
taatttagaa gagggggtgt gagttttaat ttttagagtt taatttttga gagaagataa    5100
ataaattaag tagaaaagtt tttttttttt tttttttttt ttttttaaga ggattagtag    5160
ttgtgtatta aaattttgtt tttggagatt ataaaattag gaaatagggt gtgtgggaga    5220
gatttgaatg gttgaaataa ttgtaaagaa ggtgtaagaa gtgtgagttt aggagggaaa    5280
aagttgggtt agggttggga taaaggtttt ttagggaggg ttaatttttt tgtgtttttg    5340
gtgggttttt tttgttaaag gtttataggt tggagtttgt ttgtggtttt tggtttggta    5400
gggattttat tagttttgtt ttggtaattg taagttagga atataatgtt ttgtgtaggg    5460
gattgtttat gtagtttagt ttgtgagatt gtgggatggt ggggtagtga gttggtgttg    5520
```

```
ttttgggagt ttgagttagg gtggtagttt tgttggtttt ggagagggaa ttgtaatttt     5580
gtaattaggt tgttgtgagg ttttttgttt ttgtaaagtt gtgttttatt ggtgtttttt     5640
taggtggtgt tgttttttat attttttttt ggtttatttg gtttgtattt ttataatatt     5700
ttttttttat tttttttaga ttttgtgttg gtttttattt ggatttgggt ttttgtaagg     5760
ttggtttata tagtgatttt tttgtgtgtg gatatgtttg ggtagtggtt tttttggaaa     5820
gtggttttta gtttttggag ttgttggttg gtaaagtgag tttgttgttg tttttgttgt     5880
ttttttttag atgggttttt ggtgtttatg ttttttattt tttttgttg gtttttattt     5940
ttttttgaaa atgaaatata tatattttt tgttagtatg tttatttgta atgtggatgt     6000
taattggatt ggtggtagaa gttgtggaag agttgggttg tttggtgttg gaggagggtg     6060
tgtgtggtgg ttttgggttg tgaggagtgt tgtgtttgtg gggtgtgtag gtgtaagtgt     6120
gggtgtttgt gttttatttt tttttttttt ttagtgttgt atgttttatt tatatgttta     6180
ttttattgta gtggtatatt tatttttata gtttgtgttt ttaagtatat ttatatattt     6240
ttgtgtagat atattaaatt ttttgggatg tgtatatgtg tgtggtttat agattttttt     6300
tttttttgta gaaagtttag atttttatgt ggtttgggaa ggttaggaaa agatgtgggg     6360
atttggttgg gtattgaagt ttgttggttt ttttttaaaa aaaaaaaaaa aatgtttttt     6420
tgtgaagggt atttttgagt ggttttaggt aattttttaa tgagtggagt tttttgggag     6480
ttgaaagttg agaggaaaat agggatagag gttggtggtt tttgaaggtt tttgaattaa     6540
gatgttggga tttttgtgat ttaggaaata gaagggaggt tagggtatga atagagaggg     6600
tggtagaatt gtttgtgttt ttagtgtttt aggagttggg ttggttgagg gagaattaaa     6660
gggatgtggg gtagttaaaa ttttggtttt tggaagtttt gtggggagtt aggtgaatga     6720
ttatttttat tatgtttttt tttggagggg ttgatttttt tggggtgaga gggagtgggt     6780
ggtgtagagt agttgagtgg gaatgtttgt agggtggtgt ggtgttttat ttgtggtttt     6840
tgggttggag gtgttggaga tggtgtgtat ttttagtttg tgtttggagg agtttagtga     6900
ttggggttga ttgggagtta gaattgaagt tatggttaat ggttggggat ggtgatagga     6960
agatgaggag atggttgata gtttggtttt tgttgtttgg tgttttaagt gaagtgggtt     7020
ttttatgtag tttatggatg agggagtgtg atgttttatt agtttttggt tattgttttg     7080
ttgagttttt gtagttgttg ttgtttgttt tgggttgtgt tttaggtgtg gagttttttt     7140
gttgtgggga gagttagggg atgtaatttt tgttgagttt ttaagttaag ttgtttttgt     7200
ttttttttgga aggtttaagt gaaaaagttt ggagatggaa agttagtggg taaatgaaga     7260
tatgggatgt gggtagaagg gtattattta gagtgttttt agggagtagg tttttaagtt     7320
ttaaagtgaa ataagagtgg gtaaagattt tttttttttt tttttttttt ttttaagaat     7380
ttttttaata aggaaagtta atgttgattg tgttttgttt gtttttttttt tatgtggtag     7440
ttttgataga gaagtgttaa gagtgatagg gataggtagg tgatattaga ttttttgtgg     7500
tggtagtagt tgttgtagtt atgatgtggt tttttgagtg tattttttgt aatgtgtata     7560
tgtatatttt ttgggtggtt gaataggagt tgggttttgt tgtagtttag ttttaggtat     7620
ttaggtgagt gatggattag atttgtggtt ttgtgttttt ttgttggttt aatattttaa     7680
aattagaggt gggtttttttg gtgttgagat gttattttgt tgtggttttt tttagttttt     7740
tttgtttttg tttttttttta gatttttttt tgggtgtgat tgatgtggtt ttgtattaat     7800
taggatgttt tgagttgtgg tggagggatt gttttgtttg tatttattag tagtgtgggg     7860
ttgggttatt gttttgttgt gtgtattggg tttatatagg taagtttttg ggaatttagt     7920
ttttgtttag tttaaggtga tttggttttt agtatgaatt taaaggtgaa gagatgaggt     7980
taggagttga aggtttggga gaagagagtg gaatggttaa gaagagaaag gtataaggat     8040
taataagata tttatttttt gtgttttatt atatttattt ttaatttttt attttatata     8100
aaaaggagat atgttattta aaattagaaa atttgaaaaa tagtaataaa ttattttttt     8160
gattttaaat tttttaaata gtttgttaag tgaatgttgt gttaatttga agaagtttta     8220
attgtaaaga agatagagtt ttgaaaaggt aggttaataa attagaaatt gagaagtaaa     8280
tggatttgtt aaaagaaaat tattttgatt ttaaatgaat aattgtttgg tggtttattt     8340
tggatttata taagaataaa aagttgtttt agattatgtt ttttgtgatg tttattagtt     8400
tttagataga aaatatataa tagaagagaa attttaattt agtgttttta aaatgttgaa     8460
agtttattta ttttatttaa tgttgattaa gatatatatt ttagattttt taaatttttt     8520
gtatattgta ttaagtttgt tttaatttga gagagttatg ttttaaattt gatttttttg     8580
tttattttat tattaattag atttaaattt ataaagtttg tagaattaat aattttgagt     8640
taattatata tgaaatatgt tttaatgaat ttttatataa ttaagaatgt tgttaaataa     8700
ttaattttaa ggataatttt taatagttat tttttttttt tagtgagttt aaggttgttt     8760
tgagttatta aagtttaagt aggtagaagg ggtgtgtgtg agttaagggt gaaaagttta     8820
gaattgtgtt taattagtaa aagtaaaatt ttatttatat aaaataaaaa aaattatttt     8880
tggagatatt aatttttttat agtattgttt ttaagtaaat ttaattttta aagaaattaa     8940
agaaagaaat ttaaatatat ttaaaataat ttttgaaagt ttttttgttt tttagtatag     9000
gttagttgga gaggataaat taattttttt tgggttttttg tatgggtgat tgttttatta     9060
tggagttagt gttattattt ttgaatgtgt atttgtttga tattatagtt aatgatttgt     9120
aatgttagta tgaagtattt ttaaaatatt ttttttttgt ttttgtttat aagattggga     9180
```

```
aatttatttg atgtggaata aagtggatga agtagattat aaatatattt gtaatttatg    9240
tgtttttttt ttgttttgat tatttttaaa ttttatttgt aattttttttt tattttaaat    9300
ttgtagttta aagatgtata tgagaattgt tttttagttt tttttatta gtattatttt    9360
attttaagaa taatttagtt gtaagggagg aattttttta tagtaagttt taaattagta    9420
tttttgtttt taatttttta ttttatttta ttttatttta tatatataga tatttgttta    9480
gagtaaaata tatttttatg tgataggttt gtattagttg aggtttatat atttagttat    9540
attaggtttt gtaattttat tattaaatta tatatattat attagtagtt tgttggtaaa    9600
gaaggttaaa ttaatttata ttttgtttat tatttggtgt ttaaatgatg tattttattt    9660
tggagatttg gtggagaatt tttttttag attttatagt gttttattga agataatgtt    9720
tttatatttg tagtggtttt taatttgata agattttaat ttgtttaagt tttttaaata    9780
agggttttaa atgtttttag ttgttttttt attgaatttt ttttaatttt tttaagatta    9840
taaagtatat gtgtaaagta aatatttttt tttattgtat tgttagttga tgatttataa    9900
ttaagttaat aagaatttag tttttttttg ttgaatgtgt ttattaatta tattttagtt    9960
ttttttttta aattttagaa tagttgtggt ttttataata ttatgttttt taaagtttta   10020
ttttatgaag ggattttatt atattaaaga atgaaaaaaa tttttattgt agttagtata   10080
tatagttttt tattttttgt tttttaagat ttaaatttta gagttgtaaa tatttttgga   10140
agtttgggtg ttaatgtttt attttagaaa gttgagaagt tttatagagt tatatagatt   10200
tttaaattta tttttataa atttatagaa ttttgataaa agttttggtg gttttatttt   10260
attgatggaa tttttattat gataaatata tatgtatgaa ggattttaat tagtttttaa   10320
agtggttgaa aaatttaagg gtatgtgatt gtttttata gtgttaatgt gtgtgagatg   10380
ttggaagtat tggggattag tagtagttta gatgtttaaa aagataaggt gttttaattt   10440
gtgtggattt attgaagtta agtggtgaat aaagataatt atttagataa tttagattaa   10500
agtaaaagta aaattatatt tatttgtata tatatattta tatttatttt atattataga   10560
tatatatatg tatatatata ttggttttgt aaataattga tttaaagtga ggattttttt   10620
tgtatttttt tagtaggagt tttaatattt ttttaatttt ttaattattt tatatattta   10680
tagtagtggt gattgggtga tatttttttt aggtttttg tgtggtagga tattaatatg   10740
ataagtttgt atggggaaaa ggaggtatgt ggtgggaatt aagaaatatt gtttagtgaa   10800
aattttgtgg tatggtggtg gttgattttg gagatttaat gtatataaga tttgtgggtg   10860
tataggtata ggtagtatgg atgagaaagg ggttagaaga aaataaattt tatgtatttt   10920
gtgattttag tattattgtg atttttggtt aagttttttt taattggttt tagaaattat   10980
tatgagttta gtttttaata tagaaatttt taatatggag aatattggtg ggattttggt   11040
agggaaatta gaggtgttgt atggtttatg tggggtaaag aaggaaagtt tagtgttggt   11100
gtgaggtttt gagtttggga gatattaggg gttgttttga ttggggtttt ttgtttattt   11160
ttttaaagaa agattttaga ggagggaaat gtgtgatatg gggttagttg tgttttgtgt   11220
tggtatttgt tattgattat tagttttaaa gttttattta attttatatt ttttagtgtt   11280
agttgtgtaa agtttttttg gttatggtag tgagtggttg ggttgtgttg ttaaattttt   11340
tgtattaatt tggtttggga tttaattaag tgatttttga tttttggaaa gagtttgttt   11400
ttagagttta tttagaagat ggtttaatta gatatttttt tgagttgtta ggttttagat   11460
gggtgggagt tttgttttgt ttaagttagt ttaaggatga ggtttgtttg gatttagttt   11520
ggagttatgt gatgggtgtg agtgtgtgag tttttggtaa ggtgtagagg ttagatggag   11580
attttgtatt ttgtttgaga agtgttttat ttttttttaat atttggtttt tttttgtata   11640
taaattaagt tgaaaatagt ttattattta ttattttta tagttatgga attaaataat   11700
ttagaaatta aaagttttat tgtagttgtt ttttttttta tttttaaat ggaatttaaa   11760
aagttttggt ttgttaaaag gggaagatta ttttttgaat tggaagtttg tagatatatt   11820
gagtaatagt tattttttttt gggtttttgt aaatggtatt tattttttta atttatagtt   11880
ttagttgttt aattatttga gatttggggt aattatttgg gggaatagtg tttagatggt   11940
agtgggagtt attattttat agtggtttgg ggaagagaag agaaagagat tagaggaggg   12000
ggtatttgtt aaaattattt aatgaatatg ttgttaatgt ttttttatat ttgtatgtta   12060
ttgttatagt tttttttaggt gttattgagt ttttagaaag taattatttg ttgaattaag   12120
taaaataagg agaatggtat agtatatgtg tttggagaag gggaaggaag ggtggaatat   12180
gaaattgagt atagatattt aggttaggaa agaaggaagt ggtaaggggt taaatgaagt   12240
tttatttttt tgttattttt ttaaataata gttggattaa atatttattt gtttttttttt   12300
tttttttttt tttttttttt ttagtttatg tttatttttt tttttattt tttttgtttt   12360
tttttttttt tgtttttttt tttttagata tgttggtagt taatatttag tattagttgt   12420
tatggtgatt ataaattatt taaatttaaa aatatttatt tataatttga gatgaagttt   12480
ttattttttt agtgaaataa tattttaaaa gttgttagtt gataaaaaaa aaggaattta   12540
ttttattgta gtaatttaag taatatatta tttttaaagg tttaaattaa aatgttagtt   12600
tgttaaaaat atgttggtag agttttggat attttttttg ttagattttt ataaagaagt   12660
tgattttgtt atttttggtt gtttttttaat atatatatat aattttgtat gttttttttt   12720
tttttattaa tttttttttt ttttattaat tattttagtt tttaaagatt ttatgtattg   12780
ggttttaaaa gaaaagaatt ttttttttgga ttagaaaata gttttattgg ttgttgaagt   12840
```

```
gaaagatgtg gggtttaggg ggaaaggtta ttaggattat aatggtggtg gtggtaggag   12900
gttattttag aggagttaag aagaaaaaaa aatgtaggga gaaggattgg aggtggaaag   12960
atagagtaat agaaaattga gttgggtggt taggtgttgt ggtgaagttt agttttgaaa   13020
tgataggtat atattttttt atttttgttt tttttttttt tgagagaaaa tttttttagt   13080
tagagatttt ggggggtagg aggtgggtaa atgttgttgt agttgggttt tttgtttttt   13140
attttgggtt tgttgttttt tgtttatttt ggattatagg gtatttgttt agatgaagag   13200
ttattaatta tttatttagt taatattagg aagatgataa agttttttat ataggattaa   13260
gaagatatta gattgtttta ttagtatatt tttgtttatg aataagtttt tgttatatat   13320
tttttttttt tttgagttgt tttaataatt gttgtatata ttagtagtgg gtggtgagga   13380
ataatagttg aattagtttt aagaaatttt gtgtattgag ttagtagtga ggaatgtgat   13440
ttgtgaagat tatttttgtg ggtagggatt tgtagggatt gattattttt ggataattgg   13500
tataattttt tttgggggtg aaaaattata atgtggtggg gtatttttta agtgagttgt   13560
agatttgatt ggtgtggggg gtgggggagg ggagggggaga atgggatggt ggaggttggg   13620
tggaggaaag aaaatggaaa atttttttta tttttatttt gttgtttttt ttttaagttt   13680
tatgtttttt ttggtaagta tttgtttttt ttgtgttagt tatagttaga gtttttttatt   13740
ttttttttata tattttttttt tttttgataa ggtttaggat tttggttatt attttatgta   13800
ttattatttt gtgttttgtt agagatggtt tgggttgatt ttgttggtgt ttatgttagg   13860
attaaaattt ttttatgatg gtgaggaaaa ttgtattatt tgtttttagg gggtatatta   13920
ggagtttatg tagtatatgt tttgtaaata tttgttgatt gaatgagagg tgtgggggtg   13980
gggtggtgga gaggggtttg tggttgttaa ggttgttagg gttaatttag gtttttttgaa   14040
gaaggttggg attgagttgt tgttgtgtgt gaaggtgtgt gttgtggttg ggggtgttat   14100

aatgggttat ggagtttatt ttttagaggg aggaagtttg tgtatattag tgatttgggt   14160
tgaatatttt tagtttattt attgggttaa agttatttaa taattaatgt gtttttgggg   14220
gaggttgggg gaagtatttg ttttttgttg ggatgtaaag ttaggtgtta ggtttaaagg   14280
ggttgtagtg tagtttgatt ttagtatgga atttagagtt gttgttttga aattttttaa   14340
gttagtgata gaggagggtt agtttgtttt ttttttgagg gtgaagatta ttttatgagt   14400
ttttttttagg atttttaaag taagaaaagt taaagaaagg ttttttttgtt ttagggggtt   14460
gtttttagtt ggtttttata ttattttttg ttagttgtgt ttattttttt ttaaattttt   14520
ggtttttagg ggtttttagt tgtttttgtg ttatttttgt tttggggttt tatttaggtt   14580
gggtatttgt ttaatggata ttaaggagaa tgggatttat tagggaagga aggtagagtt   14640
tggattgttt agaggtggat tttgtttata tagaatgttt agtttttaat gaggatatgg   14700
tatttttggg tggtgttggg ggtagaggtg gagagggtag tgtaatagat tattatggtt   14760
ttttgaagaa gttatatgtt attgtgaatt tttttttttt ttaaaagtaa agaaaaattt   14820
taaaaaaata taagaaataa atttttttgt tttataagta ggttgtggtt aggattttgg   14880
atattttata agttaattta aaattaggga aggataggtg ttttattttt tagtagtgtt   14940
atagttttgt ttatttgtgt gatttttttt tgttgtttat tagtttttta aaagttaatt   15000
aaattaagat ttttagtatt tttttttatt atatgttttt ttttaattaa tggtattaaa   15060
tgtgtttagg tagtaatttt ttttttttggt taaaaagtag aaaaagatat attgagtgta   15120
ggggaagagt tttttatgtg tattaaaata atgtgggttt gaaagtaatg gttaagaaag   15180
taattattta ttattttttag tttttatgt gttagttatt aaaagtgaat gattaggggt   15240
gtttgtgtgg tttgtgattg gtgtaagtag aattttttttg tttttagttg ttttttgttt   15300
atttatgagg attttatttt attgtaggtt tttttatttg tttttagaat gtattttttta   15360
tgtttaggaa atttggggta gggattgggg gaaggagata ttttgtgttt ttttggtttt   15420
tagtataata agaaatgtta gttttggttt ggtgattgtg agtttgtttt gtgtgaagtg   15480
agattgggga gttttttttag tttggttgga gttagggttg agtttgtgta aagtattttt   15540
tttagaagtt attgttgttt ttgattttta attatatttt aaatatatta tggtttaata   15600
atttattttt attattgatt attaaataga agaagaattt aatataattt aaatgataga   15660
aattatgtga tgttattttt gtattgtttt tatttaattt tatgggggtta attttggata   15720
agtgagtgtt taattggttt agtagggtga ttggtggtgt agtgtagtgt ttgggtgtga   15780
agtattggat tgttttagat gttttatttt aataaatgat tatttttttttt tagatttatg   15840
gggaaatttt aatgtaagat ttttgttttt tttttagtaa tatggtttgt tttttttgatt   15900
ggggttttaa attgtttttt tttattttat attatatttg tatttttata ttttaatttg   15960
gaaagagggg gttaggggtt gagggttgtg gggggggggg ggttttattt gtttatatta   16020
ttaaaggtta atagtttttt aaggttaggt atttatttat tatggagtta ggaaaataga   16080
ggaatagtaa atttgagggg tttttttttta tgtatttgaa aagaaaggta tttttttttt   16140
tttatttttt atattttttt ttttgtttta tagaataagt tttaatttag gaaaggtttg   16200
tggtgtaggt tggagatttt ttaatttttt atataagttt gtagattttt tttggtaatg   16260
ttttttgatt tttttagagt gaaattagtt aattaagtaa tgatattgtt aaaatttaag   16320
gtttggtaat tagtatttta ggtaggtttg tgttgatagg ggtaaatttt tattttattt   16380
tgggttgtta agtatagtgg ttgtttttta gtttttttagg gatgttgttg gtttttttgtt   16440
```

```
ttttttttaa ttagttaaag taaatttttg ttaatttaag tttttttttgt ttgttttttg   16500
tgatgaattg tgtatttata agtttgggtg gggtgtggtt gagagtttga gtgattgagt   16560
gggttttggt ggtgttgtgt gtagtgggat ataatgagtg atagaggttg ttgttggatt   16620
atttttttat gttagtttag atgttgaggt ttgttggagt gtgtgtaggg gattagatta   16680
tagggagtga gtgagaggga gagagaggtg ttgggtttta ggagtgtagt ataatttggg   16740
gaaaggaatt aatgtttttg ggatggttgt tttttgtttt atttagaggt ggagtgttta   16800
agtttaagta gtaggtgtgt taggtttggt ggttttgttt ttttgtgttt tgtttgaggt   16860
ttagagtttt ggaggtgggt gtttagtgtg tggtttgtgt ttttttttttg gttttattat   16920
tggtgttagg atgttgttgt gggaagaatt tgttgttggt tgttttttttt tggtttttag   16980
gagagtttgt gaatttgatt tttttgattt tggagttttt ggagaagaga tatttaatgg   17040
ttgttggttg tatgtttggg ttatgtgtgt gtttgtttta tgtgtggaga gaggtgtttt   17100
ggattgtggt tgaaaggagt tggggatggg aggaggggga ggggtgaggt aggttggagg   17160
agaaagaggg ataaagagta aagatttagt tagaggaaag agttgatggt atttttgttt   17220
tttggatttt ttggtaattt ggggtaggat ggtgtatttt ttttgtgttt ttttggttgt   17280
tggtggtttt agttgggagg agtaggttgg ggggttttgg tatatagtgt gttgttgttt   17340
tttagtttat tgttttgtta tagggagagg ttattggtga tttggttttg attttttttt   17400
tgtttaggtt ggttttttgg ggaagtgttt tttgttgggg ttttgttgta gggttagtgt   17460
tttttttgttg tttttatgtg gtgtggtttt ttgtttgatg atttgggtag gagaaggggg   17520
tttttattta attgtatata tgttgatatt agtttgtggt agttggtttt tatttttttgt   17580
tatttgtaaa atagaagaga aggaaggttg taagaagtgg tggttgttga gtgagtaggg   17640
tttagatgag attatgttat attagttgtt aggtgtttat tgtgtgttag gttttaggtg   17700
tgttttttttg atttgatagt ttttggttgt gtagtagtat ttttagttta gttttgggtt   17760
taggatattt atttattaag aggggatttt tttttagagt tgttgtaaaa gtgtttagag   17820
gttagaggat tataaagtta tagtgtgttg gggaggttgt ggatttattt ttaagaattt   17880
tggtgttggg gttaagaatt tatttgaatg taatggtagt gggagtgggt gggtggagag   17940
gattttttttt tttgggaagt tgtatgtaaa gattattttt tagtgtttgt ttattagttg   18000
gagtttggta aatatttgta gaatattagt gttaatgtgt ttttgtttta gatagtagtt   18060
ttttttggtt ttttgtaatt ttgaaatgaa tgggtttttg gtttagggtg ttttaggagt   18120
gagttgagtt tgggtttttt atttattagg agttattttt ttatatttag ttatattttt   18180
ttttagagat attaatttgg ttatttattt atttattata aataattatt ttaaagtatg   18240
atttaagatt gtagaggaga gatattgggt ggattgagtg agattgagga gagtagggta   18300
aatgtttttg gagggtttat tgtttgttaa ggatggagaa atagttttgg tataattgtt   18360
atttagtttt tttttttttt tttttgggtg agttaaattt tttttatgtt tttaattata   18420
atgtagtgag ttaagtattt aatgtgtttt tttttttttg ttataggtaa gttgggagag   18480
gtgggttttg aggggtttta ttgggtgggt agaagagttg tggttgtttt aaagataaga   18540
aaagaaggtt tagggttttt taggtttttt tgattttagt gtttgttttt ttttatgtta   18600
attagggtat gttgatgatt ggagggttta ttttgtgtgg gtgtggggat tggggtggga   18660
gtaagtgttg ttgggttggt ggaggtatag aggtggggta gggagttgtg ggtttgtttt   18720
tggtttgagt attgtttttt tgtgttttgg tttttttttga agggagttgg gttttgggga   18780
gtttttggtt aaggttgttg tttataggag gggttgtttg gtgttgtggt gtggggattt   18840
agggtgggga tggttaggtg gtttttttat ttgttagtga gaatgtgggt ggggatttttg   18900
ttgatttgat ttttgtgggt ttgtgggttt agaagtagta gtttggtggt tttagattta   18960
gtgattttgt agtaaaatta taggattagt ttttgattga gatgtttgtt tgtgagatat   19020
tataaaattt attattatag tttttttatta atttgatatg aagtaatata gatgggattt   19080
tattagttta gattttaaat gtttatttat gataattttg gaggaaattt gtatgttatt   19140
attattttga taatttttttt tttttatatg tttgaattgg ttgtattatt agttggtagt   19200
tggagtattg tagatggtaa ttgtaaatag tttttattta tttatttttt ttaaagaatg   19260
aaatatataa aagaaaaaga ttgtgttgtt tggtgtaaag ttagttaatt attatatatt   19320
ttttttttta ttttttttgtg ttttagtgtt gaagattaaa taaagtaata taaaataaat   19380
ttttaagaat ttatagagtt ttattttaag gattgaaaag aaggttaagg tgtgtttttt   19440
agtttatttt tatatgtttt tgtgatttgg agatttattt tgtagttaaa atgagttttg   19500
agatttgtat ttttatgttt tatttaatga ttaggtttat tagaagaatt gagtttaaat   19560
aattggggaa gataattttt taaaaagaga tttttaattt ttgtttgttg atttttaaat   19620
ttgttttatt aagataagtt ttttgtgaga aatttggttg ttagattttg gaattggttt   19680
taatggttaa ttttataaat tgagatggga gatttttttt gatgggaggt agtttttatt   19740
tttaaagttt atgttttagt tggaatgtat atgttaagga tttttgtttt ggttaatttg   19800
ggttttatat tgtgagtata taaaaagtat tatatggtta atggaggatg aggaattatg   19860
gtaaagtagg taggtaagtt ttaagaaata aaataatttg ttaaaaaata atttttgatg   19920
attattgtaa gattgaaagt gtaggaaaaa tatagtttga ataattttag atttttttat   19980
attttttttt tttttatata ttttgttatt ttataataaa atttttaatg gaaagtttaa   20040
aaataaatag tataggaata tgtgttttaa atgaattaaa ttgtgaaatt agttagtaaa   20100
```

ttaatttgta gtaagtaatt atttaaggaa attaaaatat tgtttagttt agttttgtat 20160
tttattatgt gtatgtgttt tttataatta attaatataa gtgttttagg aatatttgaa 20220
gataaatatg tttaatttaa ggaataaagt atttaaataa tttaagtgta attttgttga 20280
gttaaagtaa aatattttat aaatgaagtg gttatttaat tttttaggga aagtttggtt 20340
attgaaatgt tgtatgttta tgttatatta ataaaaattt ttaatttatt ttgtttatgt 20400
gttttgtttt tttgatatta ttggtatttg aattttagat ggatttttgt taaaatgata 20460
ttttgtgtga taaaagtatt tttagttttg attgatagat taaaataaat gtaaggaaat 20520
ttttttaaat tagattaatt ttttataaaa atattttaga atgtatgaat tttgatattt 20580
atatttataa tggtaaaagt tttttttgtt tagtttagta agataatatt tatataaaag 20640
agtaaaaaaa aattatatta ttttatgata gtttgatttt taaattgttt aagaaagtaa 20700
agtggttaaa ttggaaaaga ggaatatatt ttggaggttt agaattgaaa attttttttt 20760
taatttttag ttggaaaata attttttgta tttatttaaa gtgtattttt tgaagtgtta 20820
gattggagtt gattggtgat taatttaaag gagttataat ttaaagaaat ggtgagagtt 20880
tggtatttag gtttggtttt taggtaattt gtttgggttt gagaggttat taattgttag 20940
ttaagatgga attttttttt tttttttttt tttttaatgg ataataatgg gaagggggtt 21000
aatttttag tagttgaaat tttgtattta gttttttatt ttgagaatgt taatttttgg 21060
tttgaggatt tgtttttgta gtgttggtat tgagatttaa gggaagatat tttgttttaa 21120
atgttagtta tggtttggtt ttttttttga ttttagtatt ttgtagattg ttagtgtttg 21180
tggtgggga tgaaaggaat agggttttgt aaggtttgtt tgttgattgt gttattttgg 21240
gtgaaattta gttttaaaag ttataaatta tttatggtga agatttttg aagtggaata 21300
aatttttaga tttgtattat tttatatttt tgtgggatag atggttttta tttattggtt 21360
attgggagag agttgttgtt tttgtgtttt attgtttttt ggggtgattt ttagtgagtt 21420
gagtttttgg ttgtatggta agtgtttgaa agttgggttt gagaggattg tagggttttt 21480
gagggtgtta agttttgaag gagtttatgg gtgtattggg gtttttgaaa tttagttgtt 21540
attggtagtt ttttttgtt ttttttagt ttttttgttt ggttttgtat ttttttttt 21600
tttttttttt tttatttttt tttttttttt ttgtttttat tttgtgtggg gagtgatgtg 21660
atgttagtag agattttatt aaattttatt gtatagtggt gtgtgggtgg ttggttgagt 21720
ttggttgtgt ggttggtgat ttaggagtga gtatagtgtt tgggtgagtg ttggggggag 21780
tgagtagggg tgatgagaaa tgaggtaggg gagggaagta gatgttagtg ggttgaagag 21840
ttgggagttg gagttgggag agtgaaagga gaggggattt ggtggggtat ttaggagtta 21900
attgaggagt aggagtatgg atttttattg tggaaaggag gattagaagg gaggatggga 21960
tggaagagaa gaaaaagtaa tttgtgttaa tttggtagtt ttaataaatt aaagggggag 22020
tgttagggta gtggggagat agaaatgtat ttttggggag taaattagga tgggttggga 22080
ggaagtgata gggaaagtgg tttaagagat ggaataaagg ataatgttta tggggttgtt 22140
tgggatgagg tgtgtggagt gtgggtgtga gtgtgtgtgt gtgatttttt tttaggtttg 22200
tagagttgag gaaagaggtt atagtaaaga gggattgtgg agggaggaaa gtgagagatt 22260
ggtagagggt gggagtggag gtgggtgtgg tggggatggg agaggatgag tgaagagaaa 22320
tttagaagaa tggagtgagt tagtgggaga gggtgggagg gttatagttg ggagtgaatg 22380
agttaggttt gttagttggg gaaggttggg atgttgggtt tagtttagtt gggatattgt 22440
gtttgaggtt aaggtgggtg gattaggtat gttgagagtg ttggtgtata ggtgggtatg 22500
gttatgtatt gatttagtgt ttatgaaggg tttgtattgg ataaggttta gatgtttata 22560
gagtttagaa tttttttgt tgtatttata tttaataagt ttattttggg ttatggatat 22620
tttattttt aaaatgatga ggttaaggtt tttggtgagg atggtattaa attgtatggg 22680
atagaagtgg gggtggggga gagagttttt tttaagttta tatttgtttt tgtaaagtaa 22740
agagtatgtg aaattatagg gtatattttt atttgaaaag tgtgttttat ttttgaattt 22800
tgatttttg atttttgat ttgagtaaag atgtgtattt tggtagtgag tagaatattt 22860
tggttttgtt ttgtttttga gtggaaggat tataaatata atttgtttgg aggattaggt 22920
gtgaaggttt ttgttaggta tatgggataa tgttttttta attttaaggg tattttgtta 22980
atgtatgttt ttggaaagtg ttggaatata gttattgttt ttggatttgg atttttttat 23040
taatattaat ttttgtttga gagtaaaatt taggtttgtt attaaaaaga tatttttttg 23100
gtttttaatt gagaataaag tttttttaa aagttgtatt gttttttta aattaatata 23160
ttaatatttg taattttaga aatatatagt gatttgggag aatgtgtata aaatagatat 23220
gtttaaaaaa gtttggtgtt taaaattaat tttagttatt atataggtgt tgggtttttt 23280
ttatttttgg gggttgtttg gaatatgtta tgtgtttttt tgaattattt tgtgttttga 23340
atttatttga gttagtagta aaaataggta aataaatttg tttaatttgt tttgagtgtt 23400
aaattttttt attttgaaat agttaatagt tgatagatgg atttatttta tggaaagggt 23460
tagttttttt agttatgaag aaaattgatt agagatttat attttaagtt atttttaatt 23520
tttatgtaat atttgtgaaa atttaaattt tttttttta tttagtggaa atttaaagta 23580
gtgttattta aggggagaga aatgaggggg aaaatgttta tgtgttgttt aattgtattt 23640
tttttttgat tttgagaatt tttatttttg gtttttgaaa ttttgttgag gtaagaaaat 23700
taaatttttt taataagttt tataattgaa ttttagttat aggatattgg aaagtgtagt 23760

ttgagaaaga tatttttatt tttgtttatt gatgattttt gtagtttttt tattttttttg 23820
agtaatgggt taataatttt tttttttttt ttttttattt tgtagagatt aagaggtgtt 23880
tgtagtagaa tggttttgtt tttagttggt ggtgaggata ggtaatttta tggaaaagtt 23940
ggaagagaat gagaaaatta aagatagaaa gatttagaga tttgtggaga gatataggga 24000
gagggaaggg agttgtgttg aaaagatgta aagatatgtg tgtgtaattt tttttttttt 24060
taggttttag aggtttgtaa attagggttg agaggaaggg gtttgggaag tttatgtttt 24120
ttttgttttt tttttgtttg gagttttgtt tgttagaggt tggttaattt tagttttggt 24180
tgttgtagat attgtgttga gtttttgggt ttttgttttg tttagtgtta gtgtagttga 24240
agtgagtagt tggtgggaaa tgtaaatggt ttttggagaa atagaagata tagaatgatt 24300
tttatttttt ttttgagtgt gtggaaggag ttggatatat gttttatgtt tttaattttt 24360
tttttatatt tttagttata tttttattaa ataattaatt aatgtttaga attattaggg 24420
aatatattag gtatgtaatt gtagaagtag ggtgttgggg ggttataaat tattgagttg 24480
atttaagatg tggattttag gttttttttt tgttaaagta gtaaaggaag agtgggtttt 24540
ggtgattgta tttagatttt gattatttta aattagaagg gggtggaggg agtgtttaag 24600
taaagtaagt aattttttgt tttgtagatg taaataagat tgtagtatta aaggtattag 24660
ttttttttagg gttagattgt ttggattggg agtttgggga aggggagata ttaattttat 24720
gtatttgtga attttaagga tgttatattt ttatataaat aattttagtg tggatttttt 24780
ggaatggggg gagtaatatt tttattttag aatattaaaa tatttttttt ttaaagtgta 24840
tatttttttt attttttttaa aattttgaat tatgtttaaa gataatagtt ttttagtaaa 24900
ttggagtatt ggattatttt ttttattttt ttttattgat attttgatga tttgatttta 24960
atgtgtgggg ggtataggga attaaatata gtttataaaa ttaagtttag atgaaatagt 25020
gttggttaag tgggtttaga taatttttaa tgagaatttt aattatattt tttttttttaa 25080
tatgttgaga taagtgatag aattgttaga atggtaatta aattggaaag tttagggaga 25140
ataataattt tgtgattaaa ttggggtaaa attgtggata aatgtggggt gatttttgtt 25200
aattttttgt tatttaagag ttaggatttg ggaaaggtat agtattattt tagagtttgt 25260
tgtgatgggt tgtgtgttat tatttatttt ttttattttg gattatgatt ttaattttgg 25320
taagtaattt ttttagtttt ttatttgata ataagtgagt atgtaaatat taatggttag 25380
tgatgtttaa ttgttttaaa tattattgat ttgttggttg ttttaaattg tttttttagt 25440
ttaggttttg tttttgaatt gtttatttta gaggtttgat ttatgttttt gatgttataa 25500
tataataatt gttttttttaa aaaaggtatt taagatgaat taattgattt gtatataaat 25560
taaaattatt atgtgttgtt gattttggtg ttttataatt attttgaaat tagtatttaa 25620
ttatttgagt taaaagaata tataaatgtt tgtattgatt tattaatgaa ttatttaatt 25680
aaaatgtttg ggtaatgttg ggtgttggaa agattgttaa attaagatat attataggag 25740
ggatatgaag attagaaagg taatagatta atattttgta tttaaaatgg agtttttggt 25800
gatttttagt tttaattttg gagtaggggt tttttttttt tgttgttaaa aagattttgt 25860
gtttgtttgt gagtgagtgt atttaagtgg aaggaatgtt tttatggtta tggtggttta 25920
ggtttttttgt ttggattggg attttatagt tttaatttag gagtgttaaa ttttggaaga 25980
ttttgggtta gttttggagg tgtgtggttt tgtaagttgt taggttaagt ttgttttttt 26040
tgtttgtttt tttggtaggt tgggtgtgtt atggtagtga gttttttgtg taaatggaga 26100
gttggaatta aagttgatat ttaatagata tgttaattga gtatttattt ttgttttgag 26160
aataggaata aaaggtagtt ttttttaaga gaggtggtgt aaaggtatgt tataggagtt 26220
tagaaaaggt tggtggtggg aaatttgtag tttgggggtt agttaatatt tttttttatt 26280
ttaagtattt attgatttgt tgttgttatt tttggtgatg tagaaggata tttgaaagaa 26340
tttttgatgg ggttttgatt tgagaaagga ggtgatttgt ttaggttttt attaaatttt 26400
taattattat attaattgtt tttttttatt ttttatttga tttttttttt tttgtttatt 26460
tttaattttt taattattta gaaatttttt tattttttag tggttttttt tttgtagtag 26520
ttttttattt gaattttttt tttgtttttt tgtggtaggg tttgtatatt gatttttttg 26580
atttttggta tatttgggtt ttttgaaatt ttttaatttt tttagatttg aggatggtag 26640
gttttatttt ttttattgtg tgtatatatt tagagatatg aaaatttata tagattgttt 26700
ttaaatttag ggtatttaat agatgttttt tttttagttt gttttttgat ttgaaatgtt 26760
tgtttgattt taatttggat attatttttt tttgtttttt tttttttaaa gtagtttgga 26820
tatgtgtgta agtgagttta gaatagtttt atttatattt tttattaaat tgtaaataaa 26880
agaagaatta atgaagtaga ttggtatata gattgtatta agagtttgaa tttttagttt 26940
ttggattttt tatttaattt tggttgttat ttatattgat agagttattt taagtagagg 27000
tttagagaaa tttgtattgt gggataatag gtaaagttat agtaaaaagt ggaataattt 27060
taaagttatt ttattagaat gtaaattgta tttttgggtt ttgtttgtaa ttatttagtt 27120
ttaatatata tagagttaga taggaaaaaa taggttaata tagttattgg tattagagaa 27180
gataaatttt atgggttttt tagtgaaaag aagattttta aagtttataa tttttgatta 27240
tttaatttta tttataattg tgggaatgaa taagatatta attgttttat gtattttatt 27300
tatattaatt aatttgtgtt tttattaaaa gtagttatat agaatttttt ttaatttttg 27360
gtagtaagtt tagaaaatga agtttatagt tattttgaat tggatatatt ttttgagttg 27420

```
attattttttg taagtgtagg aatataatat tgttttttta tggttttttt gtattttttt   27480
agggtttgta agtttttatt aggtttgata ttattgtttg ggtttatatt tattataagt   27540
aaatttgatt attatgttga ttttaaaata gtttatttgg ttagtataat tttagttttt   27600
aaattataaa aattttttaa tatatgaagt ttttagtttt tatttttttt agttttttgt   27660
ttatttaaaa tttttatttt aattggtgta agtaataata atttgtatta ttatttgtat   27720
tttttttatt tttttggaga ttgggttgga ttttagagag aatattagta ttattattat   27780
tataaataat aaaatttaaa agtaaagttt ttatttgtat gataattggt atttggaatg   27840
tttttgattt atttaatgtt attttataaa ggtattttgt aaattttttt ggaattttta   27900
gtaagagttt gtagtaattg gaataatttt ttgggaagat attttttttg atgggttttt   27960
agtttttgga ggaatagatt gagagtaatt agggagggag gggatattgg aaattggtag   28020
ttatgttagt tgaaataagt ttgggtttag taaggtgatt gatgttgtgg ttgatttttt   28080
attttgagtt ttttttttaat tggggtattg attttttttta ttttgggatt ttaaggtatt   28140
tggtgtgtat gtagattttt tttttgtggt ttttattatg tggttttgta gtaggttttt   28200
ggtttaatga tattttatag ttatagtttt tatatttatt attatgattt taatgtttag   28260
gtttttagtg tatttatatt aaatttgttt tattagtaag ttggagtata taggagagat   28320
gggggtaagt aaggatttag tagagtttaa atttagatat gtttaaatgg ttttgattgt   28380
gtaaagtgtg gtaatgtttt ttgttgtttt agtttttttat tttaagtttt atatgttttt   28440
tggttaatga agtgtgatat aggttatatg ttaggaataa tagtatttgt tgagaataaa   28500
gtgaatttag gaaatttggt atatataaaa tgtatt                               28536
```

<210> 965
<211> 28536
<212> DNA
<213> Artificial Sequence

<220>
<223> chemically treated genomic DNA (Homo sapiens)

<400> 965

```
gatgtatttt gtgtatatta ggtttttttga gtttattttg tttttaataa atattattat      60
ttttagtatg tggtttatat tatattttat tagttagagg atatgtgaag tttagagtgg     120
aaagttaggg tagtaggaag tattgttata ttttatatag ttaaagttat ttaaatatgt     180
ttgaatttga gttttgttga gtttttattt gtttttattt tttttgtgtg ttttagttta     240
ttaataaagt aggtttaata taaatatatt aaaagtttaa atattgagat tatgataatg     300
aatatgaggg ttatgattat aaaatattat tgaattagag atttgttatg aaattatatg     360
gtgaagatta taagggagga atttgtatat atattgagtg ttttgggatt ttaaagtggg     420
aagagttagt gttttaatta aagagaaatt tggggtaggg aattaattat aatattagtt     480
attttattga atttaggttt gttttagttg atgtagttgt taatttttaa tgtttttttt     540
ttttttaatt gtttttaatt tattttttta gaaattgaaa gtttattaaa gaggatattt     600
ttttagagag ttgttttagt tattataagt ttttgttaga aattttaaga aagtttataa     660
ggtattttta taaggtggta ttgagtaagt tagaagtatt ttaagtatta attattatgt     720
aaataagggt tttattttta gattttgttg tttgtggtgg tggtgatgtt ggtgtttttt     780
ttggaattta gtttaatttt taaaaaagta aaaggagtat aaatagtaat ataaattatt     840
attatttata ttaattaaaa tagaagtttt gaatgagtaa ggagttagag gaggtaaaaa     900
ttgggaattt tgtatattaa aaggttttta taatttgaaa attaagatta tgttggttaa     960
ataggttgtt ttaaaattag tatagtaatt aaatttgttt gtaatgaatg tagatttaaa    1020
tagtgatgtt agatttgata aggatttgta aattttaaaa gagtgtaaaa agattataga    1080
agaataatat tatattttttg tatttataga aatggttagt ttaagagatg tatttagttt    1140
agggtggttg taagttttat tttttgaatt tgttattaga agttaaaaga aattttgtat    1200
aattgttttt gatggaaata taaattggtt gatgtaaatg aaatatataa agtagttggt    1260
gttttattta tttttataat tataaatgaa attaaatgat taaaaattat agattttggg    1320
gatttttttt ttattgagga gtttatggaa tttgtttttt ttagtattaa taattgtgtt    1380
gatttatttt tttttgttta attttgtata tattaaaatt aggtggttat gaataaaatt    1440
tagaaatata atttatattt taataaaatg attttaaaat tattttattt tttattgtgg    1500
ttttatttgt tgttttataa tgtaggtttt tttgggtttt tgtttagaat gattttgtta    1560
atgtagatga tagttagagt tgaatgggga atttagaaat tggggatttg ggtttttgat    1620
gtaatttata tgttaattta ttttattagt ttttttttta tttatagttt ggtaaagaat    1680
atgggtggag ttgttttggg tttatttgta tatatgttta aattgttttg aaaaaggaag    1740
ggtaagaaag agtggtattt aagttggaat taggtaggta ttttagatta agagatgaat    1800
tggaaaggga atatttgtta gatattttgg gtttgaaggt agtttgtgta agtttttata    1860
```

```
tttttgagtg tgtgtatata gtggagaggg tggagtttgt tatttttaaa tttgaaaaga    1920
ttgagagatt ttagagggtt tagatgtgtt aaaggttaga gggattaata tataggtttt    1980
attatggaaa ggtggggaaa aggtttgaat agaaaattgt tgtagaaggg aagttattga    2040
gaggtaaggg agtttttgaa taattaaaaa gttaagaata agtaaaagga aggaggttgg    2100
gtgggggata aaaaaaagta gttgatgtgg taattaagaa tttggtggga gtttgggtag    2160
gttatttttt tttttagatt agagttttat tagaaatttt tttaagtgtt tttttgtgtt    2220

gttaaagatg ataatagtaa attaataagt gtttgaaatg aaaggggatg ttgattagtt    2280
tttaggttat agatttttttg ttgttagttt tttttgaatt tttatagtgt gtttttgtat    2340
tgtttttttt aagaagagtt atttttttatt tttattttta ggatgaaggt aagtgtttag    2400
ttagtatatt tattaaatgt tagttttggt tttagttttt tgtttgtgtg gaaagtttat    2460
tgttatagtg tgtttagttt gttggagggg tagatagaaa aagtaagttt ggtttggtga    2520
tttgtggggt tatgtatttt tagggttggt ttggagtttt ttagagttta atgtttttgg    2580
gttagaattg taaggttttg gtttgagtaa agggtttgag ttattgtagt tgtgggagtg    2640
ttttttttat ttgaatgtat ttatttataa ataagtataa aatttttttta atagtagagg    2700
agaaagattt ttgttttaaa attaaagttg ggaattattg gaaattttgt tttgagtgtg    2760
agatattggt ttgttatttt tttaattttt atattttttt tgtaatatgt tttgatttaa    2820
taattttttt agtgtttagt attgtttgga tgttttaatt gggtaattta ttagtgagtt    2880
aatataggta tttatatatt tttttagttt aagtggttaa gtattaattt tgaaatgatt    2940
ataaaatatt ggaattggta atgtatagta attttaattt atatgtaaat taattggttt    3000
attttaaatg tttttttttaa aaaaataatt attgtattgt agtattggag gtatggatta    3060
aatttttaga atagataatt tggaaataag atttggatta ggaagataat ttagaatagt    3120
taataaatta ataatgtttg aggtagttaa atattgttag ttattggtat ttatatattt    3180
gtttgttgtt agataaggag ttggggaaat tgtttgttag ggttgagatt ataatttaga    3240
gtgaagaaag taaatggtag tatatagttt gttatagtgg gttttgaaat aatattgtat    3300
tttttttaaa ttttgatttt tgggtgatag ggagttggtg gaggttattt tatatttgtt    3360
tatggttttg ttttaatttg attatgaaat tgttgttttt tttgagtttt ttaatttgat    3420
tattatttta atggttttgt tatttgtttt aatatattgg ggggaggagt gtaattgaga    3480
tttttattaa aaattatttg aatttattta gttagtattg ttttatttaa gtttagtttt    3540
atgggttgta tttaattttt tgtgtttttt atatattaaa attagattat taaaatgttg    3600
gtaggaaagg gtgaaggaaa tggtttaatg ttttagttta ttggaagatt attattttta    3660
gatatagttt aaaattttga ggaaataaaa aggatatatg ttttgggggg aaaatgtttt    3720
aatattttag aatggggggta ttattttttt tattttagag aatttgtatt ggagttgttt    3780
atgtaaaaat gtaatatttt tgaaatttat agatatgtaa ggttagtgtt tttttttttt    3840
taggttttta gtttaggtga tttagtttta aaggagtag tatttttgat gttataattt    3900
tgtttatatt tgtagggtag agaattgttt gttttgtttg gatgtttttt ttattttttt    3960
ttaatttgaa gtaattggaa tttaaatata gttgttaagg tttgtttttt ttttattgtt    4020
ttgataaggg aaaaatttga aatttatgtt ttaaattagt ttggtggttt gtagtttttt    4080
agtattttgt ttttatgatt gtatgtttaa tgtatttttt ggtgattttg ggtattaatt    4140
agttgtttaa taggagtatg attaaaaatg taaaagaagg attaggagtg tgaaatgtat    4200
gtttagtttt ttttatatat ttgaggaggg aatgagaatt attttgtatt ttttattttt    4260
ttaggagtta tttgtatttt ttattagttg tttattttag ttgtattggt gttgggtaag    4320
gtgaggattt aaaagtttag tgtagtgttt gtggtggttg ggattggggt taattagttt    4380
ttggtgggtg agatttttaga tagaaggggg gtgagaggaa tgtgagtttt ttgagttttt    4440
tttttttagt tttggtttgt aaatttttga aatttgaaag gggagggagt tgtatgtgtg    4500
tatttttgtg ttttttttagt gtaatttttt tttttttttt tgtgtttttt tgtggatttt    4560
tgaatttttt tgtttttggt ttttttattt ttttttaatt tttttatgag attgtttatt    4620
tttgttatta gttgaaggta aggttgtttt gttatgagtg ttttttaatt tttataaaat    4680
gaaaagaaaa aaagggagga ttattagttt attatttaga ggaatgggga ggttgtaaaa    4740
attgttgatg ggtagaggtg aagatgtttt ttttggattg tattttttgg tgttttgtaa    4800
ttagagttta gttgtgggat ttgttgaaga aatttgattt ttttgttttg gtgagatttt    4860
aaaaattaga aatagaaatt tttagagtta gagaggaaat ataattaaat agtatgtggg    4920
tatttttttt tttatttttt tttttttaaa taatattgtt ttgagttttt attgggtaaa    4980
gagagaaagt ttgagttttt atggatgtta tgtggaggtt agaaatggtt taaaatgtag    5040
atttttaatt agtttttttt gtggttgaag aggttaattt tttttataaa atgagtttat    5100
ttgttgattg ttagttattt taaagtgaag ggatttagta tttaaaataa attgagtaag    5160
tttgtttgtt tgtttttatt gttaatttaa atgaatttaa aatatggagt aatttaagaa    5220
aatatataat atgttttaga tagtttttaa aagtagggaa agtttagtat ttatatagtg    5280
attagggtta gttttaagtg ttaagttttt ttaaatgtat ttattttatg tatatttttt    5340
tgagttatta tatattttta aaattgtgag tattggtata ttgatttagg aagagtaata    5400
taatttttag agggaatttt atttttaatt agggattaaa gagatgtttt tttaatagtg    5460
```

ggtttgagtt ttgtttttaa gtaggaatta atattggtgg gaaaatttga atttaggagt 5520
aatggttgtg ttttggtatt ttttaaaaat atatattaat aggatgtttt tgagattgaa 5580
aaaatattgt tttatatgtt tggtagaagt ttttatattt ggttttttag gtgaattata 5640
tttatagttt ttttatttag aggtaggata gagttaaaat attttgttta ttattaaaat 5700
atatattttt gtttaagtta agaaattaga aaattagggt ttagaagtaa ggtatatttt 5760
ttgagtgaga atatgttttg taattttata tattttttgt tttgtaggag taaatgtgga 5820
tttgagggaa attttttttt ttatttttat ttttattttg tgtaatttaa tattattttt 5880
gttaggaatt ttaattttgt tattttaaaa aatgagatat ttgtgattta gggtgaattt 5940
gttgaatgta ggtatagtag aggaaatttt agattttatg agtgtttgag ttttgtttag 6000
tgtaaatttt ttgtgaatat tgggttagtg tgtggttgtg tttatttgtg tgttgatatt 6060
tttagtatgt ttggtttatt tgttttgatt ttgggtgtgg tgttttagtt aagttgggtt 6120
tagtgttttg gtttttttta gttgataagt ttagtttgtt tgtttttggt tgtggttttt 6180
ttattttttt ttattagttt attttatttt tttagatttt tttttattta ttttttttta 6240
tttttattgt gtttattttt atttttgttt tttattggtt ttttattttt ttttttttgt 6300
agtttttttt tgttgtgatt ttttttttta attttgtagg tttgaaagaa ggttatatat 6360
gtatgtttat atttatattt tatatgtttt gttttaaata attttatgaa tattgttttt 6420
tgttttgttt tttgggttat ttttttttgtt gttttttttt agtttgtttt gatttgtttt 6480
ttaaaagtat gtttttgttt ttttgttgtt ttggtgtttt ttttttgatt tattagggtt 6540
gttgggttgg tgtagattgt tttttttttt tttttatttt attttttttt ttggtttttt 6600
tttttatagt gggagtttgt gtttttgttt tttggttggt ttttaagtgt tttgttaggt 6660
tttttttttt tttgtttttt tggttttggt ttttgatttt ttggtttgtt ggtatttgtt 6720
tttttttttt gttttgtttt ttgttgtttt tgtttgtttt ttttggtgtt tgtttgggtg 6780
ttgtgtttgt ttttggattg ttagttgtgt agttgggttt ggttggttgt ttgtgtgtta 6840
ttgtgtagtg gagtttggtg gaatttttgt tgatgttatg ttatttttta tatggagtag 6900
gagtagaggg aagagagagg gatgagaggg agggagagga gagagagtgt gagattgagt 6960
gagaaagttg gagaggagta gaaagaaatt gttagtggtg gttagatttt ggaggtttta 7020
gtgtatttgt ggattttttt ggaatttggt atttttagga gttttgtagt ttttttaggt 7080
ttggtttttg ggtgtttgtt gtgtagttgg aggtttggtt tgttggaaat tgttttggga 7140
agtagtggga tgtggagata gtagtttttt tttggtagtt ggtaagtgga ggttatttat 7200
tttgtaggga tgtgagataa tgtgagtttg gaaatttgtt ttattttgga gaatttttat 7260
tgtaggtgat ttgtggtttt tggggttaag ttttgtttaa ggtaatgtag ttggtaaata 7320
gattttgtaa agttttgttt tttttgtttt ttgttataga tattaataat ttatagggtg 7380
ttgaagttga gagggaagtt agattgtggt tggtatttaa aatgaggtat tttttttttaa 7440
attttggtgt taatattgta ggaataaatt tttgggttaa ggattagtat ttttaagata 7500
aagggttggg tataaagttt tagttattgg aagattagtt tttttttttat tgttatttat 7560
tgggaaaaaa aagaaaagaa aaagatttta ttttaattgg tagttagtga ttttttaggt 7620
ttaagtgaat tatttgggag ttaggtttgg atgttaagtt tttattattt ttttggattg 7680
taattttttt aaattgatta ttagttaatt ttaatttggt attttaggag atatatttta 7740
aatggatgta gagaattatt ttttagttgg agattaagaa aaaaattttt gattttaaat 7800
ttttgaaata tgtttttttt ttttagttta attattttat ttttttaagt aatttagaaa 7860
ttaaattatt ataaggtggt gtgatttttt tttatttttt tgtgtgagta ttgttttatt 7920
aaattaaatg gaaaaaattt ttattattat aaatgtaaat attagaattt atatatttta 7980
aaatattttt atgaaaaatt aatttgattt aaagaaattt ttttgtattt gttttagttt 8040
attaattaaa attaaagatg tttttattat ataaaatatt attttggtag aaatttattt 8100
aaaatttaaa tattaataat attaagaaaa taaagtatat aagtaaaata aattgaagat 8160
ttttgttgat gtaatatgag tatataatat tttaataatt aaattttttt taaaaaatta 8220
aatagttatt ttatttgtgg aatgttttat tttaatttag taaaattata tttaaattat 8280
ttaggtgttt tgttttttaa gttaagtgtg tttgttttta aatgttttta aagtatttat 8340
attaattggt tgtaaagaat gtatatatat ggtaaaatat agaattgaat tgagtagtat 8400
tttaattttt ttaaataatt atttattata aattaattta ttggttaatt ttataattta 8460
gtttatttaa aatatatgtt tttgtgttgt ttatttttaa atttttattt aaagattttg 8520
ttatggggta ataaagtgta tgaaaagggg ggaaatgtga aaggatttgg gattatttga 8580
attgtatttt ttttgtattt ttagttttgt ggtagttatt agaaattatt ttttagtaaa 8640
ttgttttatt ttttagggtt tgtttgtttg ttttgttatg gtttttttgtt ttttgttagt 8700
tgtgtagtgt tttttgtgtg tttataatat aaaatttaag ttggttaaaa taagagtttt 8760
tggtatatat attttaatta gaatatgaat tttgggggtg agaattattt tttattagga 8820
aaagtttttt attttaattt gtgagattag ttattgaagt tagttttgaa gtttggtagt 8880
taaatttttt atagaagatt tgttttgata gggtaagttt aaggattagt aggtgggaat 8940
tggaggtttt tttttaaaaa attatttttt ttagttattt agatttagtt tttttagtag 9000
gtttggttat taaatgaagt ataaaaatgt aagttttaag gtttattttg attgtaaaat 9060
aaatttttaa gttataagga tatgtaggag tgagttaagg aatatgtttt gatttttttt 9120

ttagttttta gagtggagtt ttatgagttt ttgaagattt gttttgtatt gttttgtttg 9180
gtttttagta ttgaagtatg gggaagtggg gggaagaatg tgtaataatt gattgatttt 9240
atattaagta atgtaatttt ttttttttgt atattttatt ttttaaaaaa aataaataaa 9300
taaaaattat ttgtagttat tatttgtagt gttttggtta ttagttaata atgtagttag 9360
tttagatata taaaaaaaaa agattattga aatgatgatg atatgtaaat tttttttgaa 9420
attattataa gtaaatattt gaagtttgga ttaataaaat tttatttgtg ttattttata 9480
ttgagttagt agaaagttgt gataatgaat tttgtaatat tttatgaata gatattttaa 9540
ttagggatta attttgtgat tttattgtag aattattaaa tttggagttg ttaaattgtt 9600
atttttgggt ttatgggttt ataaggattg aattggtaga gtttttgttt gtgtttttgt 9660
tagtgggtgg gggaattgtt tggttgtttt tattttggat ttttatgtta tagtgttggg 9720
tagttttttt tgtaggtagt gattttggtt agaggttttt tagggtttag tttttttttag 9780
gagaggttga gatgtaggga aatggtattt aggttagagg taggtttgta gtttttttgtt 9840
ttgtttttgt gtttttgtta atttgataat gtttgttttt attttgattt ttgtatttgt 9900
gtgaagtggg tttttttggtt gttggtgtat tttggttagt gtggagagag gtaggtgttg 9960
agattgaagg ggtttaggga gttttggatt tttttttttt gtttttaaag taattgtggt 10020
ttttttattt atttggtgga gtttttttgag atttattttt tttggtttgt ttgtggtaga 10080
gaagggggag tgtgttaaat gtttggtttg ttgtgttgtg gttgaaaatg tgaaaaagat 10140
ttggtttgtt tgggagagaa agggggagaa ttgggtagta gttatattag agttattttt 10200
ttgtttttgg tgggtagtaa atttttaag aatgtttgtt ttgttttttt tagttttgtt 10260
tagtttattt agtgtttttt ttttgtgatt ttaaattata ttttagggta attatttgta 10320
gtaagtaaat aaatggttgg gttagtattt ttaggagaaa gtgtggttaa atatggaaaa 10380
gtggtttttg atggatgaga ggtttgaatt tagtttgttt ttgaaatatt ttaggttaag 10440
agtttgtttg ttttagaatt atagaaaatt gagggaaatt gttgtttagg ataggggtat 10500
gttggtgttg atgttttata aatgtttatt gagttttaat taatggataa gtattgaagg 10560
gtggtttttg tatatagttt tttaaagaga aaagtttttt ttatttattt atttttgttg 10620
ttattgtgtt tagatgagtt tttaattttg gtattgagat ttttgaaagt aggtttatag 10680
tttttttagt atattgtggt tttatagttt tttaattttt gggtattttt gtggtaattt 10740
tggagggaga tttttttttg ataaataaat gttttgggtt tgaggttagg ttggagatgt 10800
tgttgtatag ttagaggttg ttaggttgga aaaatatgtt tgaagtttag tatatagtag 10860
gtgtttaata gttagtgtaa tgtagtttta tttgagtttt gtttatttga tggttgttgt 10920
tttttatagt tttttttttt ttttgttttg tagataatgg ggaatggaga ttaattgttg 10980
taaattggtg ttggtgtgtg tgtaattagg taagaatttt ttttttttgt ttgggttatt 11040
ggatgggagg ttgtgttatg tgagggtggt aagagggtat tggttttgtg gtgaggtttt 11100
agtgaggggt gtttttttga ggggttagtt tgggtaggaa ggaaattaga attaaattgt 11160
tagtggtttt tttttgtggt ggggtggtgg attaggaagt agtggtgtgt tgtgtattga 11220
agttttttag tttatttttt ttggttggaa ttgttggtaa ttggggaggt gtagaaagag 11280
tatgttattt tgttttgggt tgttagaggg tttgggggat ggggatgttg ttagtttttt 11340
tttttaattg ggtttttgtt ttttgttttt tttttttttt tggtttgttt tgttttttttt 11400
tttttttttg tttttggttt tttttggttg tggtttggga tgtttttttt tgtatgtggg 11460
gtgggtgtgt gtgtggttta ggtgtgtagt tggtggttgt tgaatgtttt ttttttaaag 11520
atttgaaat taaaaaggtt gagtttatgg atttttttga gagttgaaaa gaggtagtta 11580
gtagtaagtt tttttttgtgg tagtattttg gtgttaatgg taaggttggg agggaagtgt 11640
aggttgtgtg ttgggtattt gtttttggga ttttgggttt tgggtgaagt gtaagaaggt 11700
gaggttgtta gatttgatgt gtttgttgtt tgaatttaga tattttgttt ttgggtggga 11760
tgggaagtag ttgttttagg gatgttaatt ttttttttta aattatattg tatttttgag 11820
atttaatatt tttttttttt ttttgtttgt tttttgtggt ttgatttttt gtgtatgttt 11880
tagtaaattt tggtgtttag gttggtgtgg aaaagtggtt taatagtgat ttttgttgtt 11940
tgttatattt tgttgtgtgt agtattatta gggtttattt agttatttag gtttttagtt 12000
atgttttatt tagatttgtg ggtgtgtggt ttattgtggg aggtaagtaa gggaaatttg 12060
agttggtgaa ggtttgtttt ggttggttgg gggaggggtg gggggttgat aatatttttg 12120
aagagttgga gggtagttat tgtgtttagt agtttagggt agaatggagg tttgtttttg 12180
ttgatgtgaa tttgtttgaa gtattggttg ttaggttttg ggttttggtg atgttgttgt 12240
ttgattggtt ggttttattt tggaggaatt gagggatatt gttagaggag gtttataggt 12300
ttatgtaaaa agttaaaaag tttttaattt atgttatagg tttttttttga attgaaattt 12360
gttttatggg gtggaggggg gggtgtaagg gatggaggag ggaagatgtt ttttttttta 12420
aatatatgga aaaaaatttt ttaaatttat tgtttttttta tttttttggt tttgtagtaa 12480
ataagtgttt agttttagga ggttattgat ttttgataat gtgagtagat aaagtttttt 12540
tttttttata gtttttggtt tttaattttt ttttttttgga ttaaagtgta agaatataaa 12600
tgtaatatgg gatggagggg ggtgatttgg gattttggtt aaaaaaataa attgtattat 12660
taagaagaaa ataaaggttt tgtattggag tttttttgtg aatttgagag aaaatgatta 12720
tttgttgaaa tgaagtgttt aaagtgattt agtgttttat gtttggatat tgtattatat 12780

tgttagttgt tttgttgggt tagttaaatg tttatttgtt tgggattaat tttatggggt 12840
taaatggggg taatgtagag ataatgttgt gtgatttttg ttatttagat tgtgttaaat 12900
tttttttttg tttgataatt ggtagtaaaa ataaattatt agattgtagt atgtttggga 12960
tatggttaaa aattaagagt agtgatgatt tttggggaga atgttttgtg tgggtttagt 13020
tttggttttg gttagattag aggagttttt taattttgtt ttgtgtgggg tgggtttgta 13080
gttgttaagt tgaggttgat attttttatt gtgttgggag ttagagagat gtaaaatgtt 13140
tttttttttt agttttttatt ttaggttttt tagatatggg gaatgtattt tgaggatagg 13200
tggagaagtt tatggtagga tggggttttt gtaggtgagt aggaaatggt taagagtaga 13260
ggagttttgt ttgtgttagt tataagttgt gtaggtgttt ttggttgttt gtttttgata 13320
attagtatat aaagaattag aaataatgaa tgattgtttt tttaattatt atttttaggt 13380
ttgtattgtt ttagtgtatg tgaaaggttt ttttttata tttaatatgt ttttttttat 13440
tttttgattg aaaagaaaaa ttgttgttta aatatgttta atgttattaa ttaagaaaag 13500
gtatgtaatg ggaagaaatg ttgaaaattt tgatttaatt ggtttttaag gaattagtag 13560
atgataaaaa aaaattatat gagtgggtaa agttatagta ttgttgaagg atagagtatt 13620
tatttttttt tgattttaag ttaatttatg gaatatttaa agttttggtt atagtttgtt 13680
tgtaaaataa aaggatttat tttttgtgtt tttttaaagt ttttttttgt ttttaaagag 13740
aaaaaaagtt tataatgata tatgattttt ttaaaaggtt gtgatagttt attatgttat 13800
ttttttgtt tttgttttta atgttgttta aaaatattat gtttttgtta aagattaaat 13860
gttttgtata ggtagagttt atttttaagt agtttaggtt ttgttttttt tttttagtga 13920
gttttatttt ttttggtatt tattgggtgg atgtttagtt tggatagaat tttgaaatgg 13980
gggtagtatg agagtgattg gagattttta aaagttagag gtttgagaga gggtggatgt 14040
agttagtaga agatggtgta gaagttagtt gagaatgatt ttttagagta aagagatttt 14100
tttttggttt tttttgtttt ggggggtttg aaaggaattt ataaaatggt ttttattttt 14160
aggaggagga tggattgatt tttttttgtt attggtttaa aaagttttag ggtggtggtt 14220
ttgggttttg tgttgaaatt ggattgtatt gtagtttttt tggatttgat gtttggtttt 14280
gtgttttgat aaggggtggg tatttttttt ggtttttttt aggaatgtat taattgttaa 14340
atagttttgg tttagtggat gggttgaaag tgtttgattt aagttgttgg tgtgtataga 14400
tttttttttt ttgggaggtg ggttttatgg tttgttgtgg tatttttagt tgtgatatat 14460
atttttatat gtggtagtag tttggtttta atttttttttg aaggatttgg gttaattttg 14520
gtggttttgg tggttgtaga tttttttttg ttgttttgtt tttgtgtttt ttatttaatt 14580
agtgaatgtt tgtggagtat atattatgtg gatttttaat gtattttttg aaagtaaata 14640
atatagtttt ttttgttgtt atgaagggat tttaatttta atatggatat tagtgagatt 14700
agtttagatt gtttttagta aaatgtaaaa tggtggtgtg tggggtggtg attaaggttt 14760
tgagttttgt tagaaagaag gggatgtgta gagaaaggtg gagaatttta gttgtggtta 14820
gtgtggaagg gataggtgtt tgttgaaggg ggtatgaggt ttgaggaaaa agtaatgaaa 14880
taggggtaag gagagttttt tattttttttt tttttgtttg atttttgtta ttttattttt 14940
tttttttttt ttttattttt tgtgttaatt aaatttgtag tttatttgaa aggtgttttg 15000
ttgtgttgtg gtttttttatt tttaggggaa attgtattag ttgtttgaaa gtagttagtt 15060
tttgtggatt tttgtttgta aaagtggttt ttataggttg tgttttttgt tgttgatttg 15120
gtatataaag ttttttaagg ttggtttggt tgttattttt tattgtttgt tgttaatata 15180
tgtagtagtt gttagagtgg tttgggggaa aaggaaatgt ataatgaaag tttatttgtg 15240
agtaggaata tattaatgga ataatttgat gtttttttaa ttttatgtaa aaagttttgt 15300
tgttttttta atattgattg aatgggtaat taatggtttt ttatttaggt gaatattttg 15360
taatttaaga taggtaaaag ataataagtt taaggtagaa gataaaaggt ttaattgtag 15420
tggtgtttgt ttgttttttta ttttttaggg tttttgatta ggaaagtttt tttttagagg 15480
agaaaaaggt aggagtggga gaatatatat ttattatttt ggggttagat tttattgtag 15540
tatttgatta tttagtttag ttttttgtta ttttgttttt ttatttttag tttttttttt 15600
tgtatttttt ttttttttta attttttttag gatgattttt tattattatt gttattatgg 15660
ttttaataat tttttttttt aaattttata tttttttattt tagtaattaa tgaggttgtt 15720
ttttgattta ggaggagatt tttttttttt agaatttaat gtgtagagtt tttgagaatt 15780
aaagtagttg gtaggggagg aagaaattaa tagaaaggga gagagtatat agaattgtgt 15840
gtgtatgtta aagagtgatt aggaatgata gagttaattt ttttgtgagg atttgatggg 15900
aagagtgttt aagattttat tagtatgttt ttaataggtt gatattttaa tttaaatttt 15960
tagaagtaat atattatttg ggttattata atgaggtggg tttttttttt tttgttagtt 16020
gatagttttt aaaatattat tttgttaggg aaataaaagt tttattttag attataggtg 16080
ggtatttttg gatttaggtg atttatggtt attatgataa ttaatgttga atgttagtta 16140
ttagtatgtt tgggagagag aaaatagaaa gaagggagag taaaagaaat agaaaaggga 16200
gatggatata agttggagag ggaagaaaag agaaaaagag gaagatagat gagtgtttaa 16260
tttaattgtt gtttaaaaaa gtggtggggg ggtgggattt tatttagttt tttgttattt 16320
tttttttttt tgatttggat atttatgttt aattttatat tttatttttt tttttttttt 16380
tttaaatata tgtgttatat tattttttttt attttattta gtttggtaag tagttgtttt 16440

ttggagattt agtgatattt aggaaaattg tggtagtaat atgtaaatgt gaggaagtat 16500
taatagtatg tttgttgagt gattttagta aatgtttttt tttttaattt tttttttttt 16560
ttttttttag gttattgtga ggtggtaatt tttattgtta tttgaatatt gtttttttag 16620
gtagttattt taaattttaa atggttgagt agttagagtt gtgggttgga aaaatgggta 16680
ttatttgtag ggatttagag agggtggttg ttgtttaata tatttataga tttttaattt 16740
agaaaataat tttttttttt tgataagtta gagtttttta aattttattt aggaaatggg 16800
gaaaaggata gttatagtga agtttttaat ttttgggtta tttggtttta tagttatgag 16860
gggtggtggg tagtggattg tttttagttt ggtttgtatg tagagaaaag ttagatattg 16920
gaggggggtgg ggtatttttt gggtaggatg taaggttttt atttgatttt tgtgttttat 16980
taggagttta tatatttatg tttattatgt ggttttaagt tgagtttagg tgggttttgt 17040
ttttgagtta gtttgggtag ggtaggattt ttatttgttt aaggtttaat agtttaggga 17100
gatgtttaat taagttattt tttgggtgaa ttttgaagat agattttttt taaaagttag 17160
agattatttg gttgagtttt aggttagatt gatatggaga gtttggtggt atagtttaat 17220
tgtttattgt tatggttaga gggattttgt ataattaata ttgaagagtg tgaaattaaa 17280
taagatttta agattggtaa ttggtggtaa atattagtat aaaatatggt tgattttatg 17340
ttatatattt ttttttttta gggttttttt ttgaaagaat aagtaagaaa ttttaattga 17400
gataattttt gatgtttttt agatttaaaa ttttatgttg gtattgggtt tttttttttt 17460
gttttatgtg agttatgtag tatttttagt tttttttatta ggattttatt aatgtttttt 17520
gtattggaaa tttttgtgtt agaggttgaa tttatagtaa tttttaaaat taattaagaa 17580

gaatttagtt agaggttata gtaatgttgg aattataaaa tgtataagat ttattttttt 17640
ttggtttttt ttttatttat gttgtttatg tttgtgtatt tataagtttt atgtatatta 17700
aattttttaaa attaattatt attatgttat agagttttta ttggatagtg ttttttagtt 17760
tttattatat attttttttt ttttatgtag atttattatg ttggtgtttt gttatatagg 17820
gggtttgaga agaatgttat ttaattgttg ttgttgtgag tgtgtaaagt gattaggaga 17880
ttaggagaat gttgaaattt ttgttggaaa aatgtaaaga aaatttttat tttgagttag 17940
ttgtttatag agttagtgtg tgtgtgtgtg tgtgtgtttg taatataaaa tggatgtgaa 18000
tatatatata taaatagata tggttttgtt tttattttaa tttgaattat ttagataatt 18060
gtttttattt attatttgat tttaatgggt ttatataaat taggatattt tatttttttta 18120
ggtatttagg ttgttgttga tttttagtgt ttttaatatt ttgtatatgt tggtattatg 18180
aggagtagtt atgtgttttt gggtttttta attattttgg aggttgattg aggtttttta 18240
tatatgtata tttgttgtga tgaaagtttt attggtagag tggagttatt agagttttta 18300
ttaaaatttt gtgggtttat gagagatggg tttagaaatt tatatggttt tgtggggttt 18360
tttggttttt taaaataagg tattaatatt taagttttta aaaatatttg tagttttggg 18420
gtttgaattt tgaaaaataa ggagtgaggg gttgtgtata ttaattatag tggagatttt 18480
ttttattttt taatgtgatg gagttttttt atgaaatgaa gttttaaggg gtatggtatt 18540
gtggggatta tagttatttt gaggtttaaa agaagaaatt ggaatatgat tagtaaatat 18600
atttagtaga aaagagttgg atttttattg atttagttat aggttattgg ttggtagtgt 18660
aatgggagga aatatttatt ttatatatat attttatgat tttgggggaa ttagaggaaa 18720
tttaataaga aaatggttag aaatatttaa aatttttatt taaaagattt aagtaaatta 18780
gagttttatt agattaaaaa ttattataaa tgtaagagta ttgtttttag tgaaatgttg 18840
tggggtttga gaaggagatt ttttgttaaa tttttgggat aaaatgtgtt atttaagtat 18900
tagataatga gtagaatgta aattaattta atttttttta ttaataggtt gttagtgtaa 18960
tgtgtataat ttagtgataa gattgtagga tttaatatag ttggatgtat gagttttagt 19020
taatgtagat ttgttatatg aggatgtgtt ttattttgag taggtgtttg tatgtgtgga 19080
atggggtaaa gtggaataaa aggttaaaag tagaaatgtt gatttaaagt ttattatgaa 19140
gaaatttttt ttttgtagtt aaattatttt taaagtggga tgatattggt gaagaaagat 19200
tgaaaaataa tttttatgtg tgtttttgga ttgtaagttt aaaatgggga ggagttgtag 19260
atagggtttg ggggtggtta gggtaaagga gagatatata agttgtaaat atatttgtag 19320
tttgttttat ttattttgtt ttatattgaa taagtttttt aattttgtga ataaggataa 19380
ggagggagtg ttttaaagat attttatgtt ggtattgtaa attattgatt gtaatgttaa 19440
ataaatatat atttagagat gataatatta attttatagt aaaataattg tttatgtaga 19500
aatttagagg agattagttt gttttttttta gttgatttat gttgggggat aaaaggattt 19560
ttaaaaatta ttttgaatat gtttggattt tttttttttaa tttttttgga aattaaattt 19620
gtttggaaat agtgttataa agagttgatg tttttaaagg tgattttttt tgttttatat 19680
aaataaggtt ttgtttttgt tagttgagtg tagttttagg tttttttgttt ttagtttata 19740
tatatttttt ttgtttgttt ggattttaat ggtttaagat agttttgagt ttattgggaa 19800
aagaaaatga ttgttaaaaa ttatttttga aattggttat ttggtaatat ttttaattgt 19860
atggaaattt attaaggtat attttatata taattagttt aaggttgttg attttatagg 19920
ttttatggat ttaaatttga ttgataataa agtaaataag agagttgaat ttaaagtgtg 19980
gttttttttgg gttaggatga gtttaatata gtgtataagg aatttgaaag atttaggata 20040

tgtgttttaa ttaatgttaa gtagaatgga taagttttta gtattttgaa aatgttgggt 20100
tagggttttt tttttattgt gtgtttttttg tttggggatt aataagtatt atagagaatg 20160
tgatttgagg tgattttttta tttttgtata aatttagagt gaattattaa atagttgttt 20220
gtttaaagtt aaggtaattt ttttttgatg ggtttatttg tttttttgatt tttaatttat 20280
tagtttgttt ttttagggtt ttgttttttt tgtaattaaa gtttttttag attagtgtag 20340
tattttatttg ataggttgtt tggaaaattt aagattggag aggtgatttg ttgttgtttt 20400
ttaaattttt tagttttaag taatgtgttt ttttttttata tggggtgggg gattggaaat 20460
ggatgtagtg agatataaag agtgggtgtt ttgttgattt ttgtattttt tttttttttga 20520
ttattttatt tttttttttt aagtttttga tttttagttt tatttttttta tttttgggtt 20580
tgtattaaaa gttggattgt tttgggttgg gtaggagttg aatttttggg agtttgtttg 20640
tgtagattta gtgtgtatgg tgaggtagta gtttggtttt gtattgttga taggtgtagg 20700
taggatagtt tttttattgt ggtttggggt gttttgattg gtgtggagtt atgttagttg 20760
tatttggaga agggtttggg aggaggtgga ggtggagagg gttggggagg gttgtggtgg 20820
agtgatgttt tggtattagg aagtttgttt ttggttttaa gatgttaggt taatagggaa 20880
gtgtggagtt gtagatttgg tttgttgttt gtttgggtgt ttggagttga gttgtggtaa 20940
ggtttggttt ttgtttgatt gtttgagggg tgtgtgtgtg tgtgttgtgg agggtgtgtt 21000
tagagggttg tgttgtggtt gtagtggttg ttgttgttgt aggggattta atattattta 21060
tttgtttttg ttatttttga tattttttttg ttagggttgt tgtgtggggg gggggtgggt 21120
agagtgtggt tggtgttagt tttttttatt ggaggggttt ttgggggagg gagggagaga 21180
agaagggggt ttttgtttat ttttgttttg ttttggagtt tggaagtttg ttttttaaag 21240
atgttttgag tggtgttttt ttgtttatat tttatgtttt tgtttgtttg ttgatttttt 21300
gtttttggat ttttttgttt gagtttttttg gaggagatgg gggtagtttg gtttgagaat 21360
ttggtggggg ttgtgttttt tggttttttt tgtagtgggg aaattttgtg tttagagtgt 21420
gatttggagt gggtagtggt ggttatgggg gtttggtggg gtagtagtta aggattagta 21480
gagtgttgtg tttttttgtt tatgaattgt atgaaaggtt tgttttattt ggagtattga 21540
gtagtgggga ttaagttgtt ggttgttttt ttattttttt gttattattt ttagttgtta 21600
gttatggttt tggttttggt ttttggttag ttttggttgt tggatttttt taagtatagg 21660
ttggaggtgt atattatttt tgatattttt agtttggagg ttgtaggtaa ggtgttgtgt 21720
tgttttgtag atatttttgt ttagttgttt tgtgttattt gtttttttttt gttttaagga 21780
agttagtttt tttgggggga ggtgtggtgg gagtggttgt ttgtttggtt ttttgtagaa 21840
tttttgggag ttggaatttt gattattttg tatttttttta gtttttttttt gattggtttg 21900
gtttttgggg tgttaagggt gtgagtaatt ttgttgtttt ttttatttgt attttggttt 21960
ttttttttgtt ttttgggtta taaaaatttt agtattttga tttgaggatt tttagaggtt 22020
gttgattttt gtttttgttt tttttttggt ttttagtttt tgaggagttt tatttgttag 22080
gaaattgttt gaaattattt agaaatgttt tttgtgaaga ggtatttttt tttttttttt 22140
gggaaagggt tggtgaattt tggtgtttaa ttgaattttt atattttttt ttagtttttt 22200
taaattgtat ggaaatttga gttttttgtg agggggaggg gggtttgtaa attatgtgtg 22260
tgtgtgtgtt ttaggagatt tggtgtgttt gtgtagaggt gtataaatat atttgaaagt 22320
ataggttata aaagtgaatg tgttgttgta gtgagataaa tatgtaaata aaatgtgtgg 22380
tgttgggggga ggggaggaaa tggggtgtgg atatttatat ttgtgtttgt atattttata 22440
ggtgtagtgt tttttgtggt ttggagttgt tgtgtgtatt tttttttggt gttaggtagt 22500
ttagtttttt tatggttttt gttgttggtt tagttggtgt ttgtgttgta ggtgggtatg 22560
ttgatgggaa agtgtgtgtg ttttgttttt agagaaagat aaaagttagt aggggaagaa 22620
tgaggatgtg ggtgttgagg atttgtttaa gaagaagtgg taaaggtggt agtggattta 22680
ttttattagt tagtagtttt aggagttgga ggttattttt tagaggaatt gttatttgga 22740
tatgtttata tgtgaagaaa ttgttgtgtg gattaatttt atggaagttt gagtttgggt 22800
aggagttagt atggagtttg ggagggatgg ggggaggatg ttgtggaggt ataggttaag 22860
tagattagga gagaatgtgg aaggtagtgt tgtttgggag ggtgttggtg gggtgtagtt 22920
ttgtaaaggt agaaggtttt gtggtggttt ggttgtgaga ttatagtttt ttttttgagg 22980
ttgataggat tgttgttttg gtttaggttt ttagagtggt attggtttat tgttttgtta 23040
ttttgtgatt ttatgagttg ggttgtatgg gtaatttttt gtataggata ttgtgttttt 23100
ggtttgtagt tgttagagta gagttaataa aatttttatt aggttaagag ttgtgaatag 23160
gttttaattt gtgagttttt aataaggaaa atttgttaga gatatggaag agttggtttt 23220
ttttgggaaa tttttgtttt ggttttggtt tagttttttt ttttttgggt ttgtgttttt 23280
tatatttttt ttatggttgt tttggttatt taggtttttt ttatatattt tattttttag 23340
ttttgtgatt tttgggagta aagttttaat atataattat tagtttttttt agaaggagaa 23400
agaaaaaaag aagaaagatt tttttgtttg gtttatttat tttttttttag gagttgaatt 23460
ttggaaattg aaatttatat tttttttttt aaattataat tatagttttg taaaaagggt 23520
ttattttaat tttgtagtaa atttgtattt tatggattgg taaaaatgag tttaaataaa 23580
taatttaata gtaatgtttt ggtttatgtt ggttggtgga agattttaaa tttgttagga 23640
ttttggaagt agaaaataga attaagtaaa ttaagtggta tttagaggtt ttgttgttaa 23700

aaaaaaaaaa ttaagtgttt tgggtagaaa aaataaagtt tttggttaga gtagagtaaa 23760
taaaaagaag aaaataatga taaaaagaat aaagattaaa atgttttttt aaattagagg 23820
gaatgaagat attttttggg tggtatttgt gtaaggtatg aggttatgtt ggtggataaa 23880
aggttgggaa gaagttgaaa atggttttag tttaattgtt tagagttaga gttgggtttt 23940
gggtggtgtg gttttgagta aggttagttt tttattagtt tttttgtata ttaagggaat 24000
gggtttttta tgtatttttt ttgtttgagt aaagtttaga tggtttaggg tagaaatggt 24060
aagtaattaa agatagagtt tatgggtttt ttgggatttt ttgaaaatgt tttttattt 24120
tgtttgttat tttgtagttt tattttagtg ttttgtagtt gtggtgttgg gtttttttg 24180
tagttgtttt tttttttagg gtggttgttt gttgagttaa gtgggagtga ggtgtgtttt 24240
ttatagtagt tgggtgtaaa gaggaagggg gataaaaagg aaattaagaa tgaaaggaaa 24300
aagagaaaaa gtggattata tggttgggtt tggtggagat gtgtaatgtg aaatattatt 24360
ggtgttagtt tggatatttt aggttaggtt ttttttaat atataaaagt tgttgtttgg 24420
ggtgataggg aggtttgatg tggattggga ttggggttgt ggttgggtta ttggatatgg 24480
gtggaagttg gttggtttgg gtggttgttt gtaaagttaa atgatttggt tgggtttggt 24540
gtgtggatag gtttgtggtg ggtttagggt aaagaagagg tagagtgaaa gaagggggaa 24600
tttttaaaat tattttttttt gggtttttgg agtttaatat gttaagtttt tggagttaat 24660
gagttgatga agaggtggtt ttttgttttt tatttggttg ttttgttagg tgagaaagag 24720
tgttggtggt ttagtttttg ttaagggagt atgtattagg gggtgggggga tgatagtgga 24780
ggttagggaa ggaagggagg aattgtgtgg gagaaagagt gattttttag tgttttttta 24840
gttttttttt tttatttgtg ggtttgtggt tttggaatgg aagtaagttt gtaaggtgtt 24900
ttgggaaggg ttggaaaagt ttgttgtttt gtgtttgttt tatattaagt gtttttggat 24960
ttggagaaat gtttggttga gtgattaaat tgtttgtagg tttttatgtg tttggttgag 25020
gtttgtggtg tagttttgag ttttagtttg taggttagag tagattaggt tttttgtgtt 25080
tggtggagat ttgggttagt aattgaaagt tggttttggt attttggtgt gtagggtggt 25140
gtagtgaagt gaggttaggg tgtgtgagtg tgttagtgtg tgtgttgggg gaaggtgggg 25200
gttggttttt gatggaagtt ttagtaattt gtattgtggt atttgtttgt tttttttgttt 25260
taattgtttt taggtttggt ttaagaattg ttgggttaaa tggagaaaga gggagtgtaa 25320
ttagtaggtt gagttatgta agaatggttt tgggttgtag tttaatgggt ttatgtagtt 25380
ttatgatgat atgtatttag gttattttta taataattgg gttgttaagg gttttatatt 25440
tgttttttta tttattaaga gttttttttt ttttaatttt atgaatgtta atttttttgtt 25500
attatagagt atgttttttt tatttaattt tattttgttt atgagtatgt tgtttagtat 25560
ggtgttttta gtagtgatag gtgttttggg ttttagtttt aatagtttga ataatttgaa 25620
taatttgagt agtttgttgt tgaattttgt ggtgttgatg tttgtttgtt tttatgtgtt 25680
gttgattttt ttgtatgttt atagggatat gtgtaatttg agtttggtta gtttgagatt 25740
gaaagtaaag tagtatttta gttttggtta tgttagtgtg tagaatttgg tttttaattt 25800
gagtgtttgt tagtatgtag tggattggtt tgtgtgagtt gtatttatag tgttgggatt 25860
ttaggatttt gttggatggg gtaattttgt ttttgaaaga ttgggaatta tgttagaagg 25920
ttgtgggtat taaagaaagg gagagaaaga gaagttatat agagaaaagg aaattattga 25980
attaaagaga gagttttttt gattttaaag ggatgttttt agtgtttgat attttttatt 26040
ataagtattt ttaatagttg taaggatata tatataaata aatgtttgat tggatatgat 26100
attttaatat tattataagt ttgttatttt ttaagtttag tattgttaat atttaaatga 26160
ttgaaaggat gtatatatat tgaaatgtta aattaatttt ataaaagtag ttgttagtaa 26220
tattataata gtgtttttaa aggttaggtt ttaaaataaa gtatgttata tagaagtgat 26280
taggattttt tgtttgtgag taagggagtg tatatattaa atgttatatt gtatgttttt 26340
aatatattat tattattata aaaaatgtgt gaatattagt tttagaatag tttttttggt 26400
ggatgtaatg atgtttttga aattgttatg tataatttat tttgtgtata atattttgta 26460
taatattatt gttttatttt ttagtaaata tgaaataaat gtgttttatt ttatgggagt 26520
aaaatatatt gtatataaat tggtttggat tttttttttt tttttttgtt attaatttgg 26580
ttaggatatt ttagttattg ttttttaaat aaattagttt tttttgtttg tttagttaaa 26640
tatataaggt agtagttttt atttaaattt ggtagaaata aatgatagtt atttattaga 26700
aattaaaaag aaaaaaaaag gtatttttgg gggggaaaag ggttataaaa tttaattttg 26760
tttttttaat tttttttttgg tttaaattta gaggatttta ttatggttag taaataatat 26820
gaaaaagaaa aaagaagaaa gaaatttagt aagtttatta gtttaaaatg atttttaagt 26880
ttattttttt atggggaaat ttatattttt agtaaattgt tttggagaaa tatttgtgta 26940
tgtatatatg tatagtttat atgtattttt tttaggagga atatatttat aataaattta 27000
tagggaaata tttttagttt aaaatattta ggtttttatg tttattttta ggtttaagta 27060
gagagatttt ttatgttata ttgtattatt atttttaaat tttttggaga tattaaaaga 27120
aataaagatg atttttaata attatagttt tttagttttt taaagaattt aggggttgag 27180
aggttagagt ggagtttttt gagttttgtt gagtaatatg tagttgaggt aaaggttatg 27240
tttttggtgt tttgttttaa ataatattga tttattaatt ttaaatttgt ttgtttttga 27300
aattatatag gattatagtt tgtaaattgt aggataatga agtaaattaa gatgaattat 27360

agttttggtt ttttttgtta ttttttgata tttaaatagg gaatgagttt ggtgtgagtg 27420
tttaaatgaa ttttaagtat ttgatttttt tttatttgtg atttttagtt ttaaaaaaat 27480
gtgaaatttg attttataat aaatagaaat aaatattatt tagttttaga gaatttattt 27540
ttatggtgtt aggagggttg ttgtggaggt ggggggaggg atgtgttgag atttttttgtt 27600
atgtttgtta attttttgta taattaaagt gggtgagaat aaatattatg ttggggaatt 27660
tagagtaaaa agtaattgtt gattttttgg agttgataat attattgttt ttttgtttta 27720
gttgttttta tttgaatgaa attttattta gaagtttttg gagtttgaat attgagtttt 27780
tgttttgtaa gaaattgttg ttgttattta aagagattgt agataatgtt tttgatttaa 27840
gattagtgtt taatgtatat tttttttttt tttaaagttt tgtttttgat ttgtggaggg 27900
attatgtagt agtggggggt agataaaagt tttttggatg agggttattt tttattatgt 27960
tgtttatttg agagtagtgg agaggaaaat ggtttttatg ggtggatttt ggtttttgga 28020
gttgtagggt ttagtggttt tggttttttt gtttttttagt ttggtagggg gtggggaggg 28080
ttaaagggtg gaggggaagg aaggagttag aagaggggat ttggggatgg gggttggaag 28140
tgttaatgag atttgtttgg aggatttagg ttttttgtag gttggtgagt gatttgggag 28200
ttttgggtaa aagaggtgta ttttggttta gtttggttgt tgaattagaa taatgtgagg 28260
atattaatta atttgtagga aataaaaaat ggaagttgag gtttaggaag agttgttatt 28320
tttttgattt gagtggtgta ggttgggggt ggagatttgg gatttaagag aggttatttt 28380
ttttattttta gttttttttt tttggatttt taaaaggaat aattttattt ttttttttgt 28440
ttttttata atttttattt ttgttagttt gtaggttgtt tgtttttttt tgtatttttt 28500
tttttattt agtgagagaa atttagtttt tagagt 28536

<210> 966
<211> 20
<212> DNA
<213> Artificial Sequence

<220>
<223> chemically treated genomic DNA (Homo sapiens)

<400> 966

ggagtggagg aaattgagat 20

<210> 967
<211> 22
<212> DNA
<213> Artificial Sequence

<220>
<223> chemically treated genomic DNA (Homo sapiens)

<400> 967

ccacacaaca aatactcaaa ac 22

<210> 968
<211> 33
<212> DNA
<213> Artificial Sequence

<220>
<223> chemically treated genomic DNA (Homo sapiens)

<400> 968

tgggtgtttg taatttttgt tttgtgttag gtt 33

<210> 969
<211> 22
<212> DNA
<213> Artificial Sequence

<220>
<223> chemically treated genomic DNA (Homo sapiens)

<400> 969

gtaggggagg gaagtagatg tt 22

<210> 970
<211> 24
<212> DNA
<213> Artificial Sequence

<220>
<223> chemically treated genomic DNA (Homo sapiens)

<400> 970

ttctaatcct cctttccaca ataa 24

<210> 971
<211> 20
<212> DNA
<213> Artificial Sequence

<220>
<223> chemically treated genomic DNA (Homo sapiens)

<400> 971

agtcggagtc gggagagcga 20

<210> 972
<211> 27
<212> DNA
<213> Artificial Sequence

<220>
<223> chemically treated genomic DNA (Homo sapiens)

<400> 972

agttggagtt gggagagtga aaggaga 27

<210> 973
<211> 144
<212> DNA
<213> Artificial Sequence

<220>
<223> chemically treated genomic DNA (Homo sapiens)

<400> 973

gtaggggagg gaagtagatg ttagcgggtc gaagagtcgg gagtcggagt cgggagagcg 60
aaaggagagg ggatttggcg gggtatttag gagttaatcg aggagtagga gtacggattt 120
ttattgtgga aaggaggatt agaa 144

<210> 974
<211> 22
<212> DNA

<213> Artificial Sequence

<220>
<223> chemically treated genomic DNA (Homo sapiens)

<400> 974

aacatctact tccctcccct ac 22

<210> 975
<211> 32
<212> DNA
<213> Artificial Sequence

<220>
<223> chemically treated genomic DNA (Homo sapiens)

<400> 975

gttagtagag attttattaa attttattgt at 32

<210> 976
<211> 14
<212> DNA
<213> Artificial Sequence

<220>
<223> chemically treated genomic DNA (Homo sapiens)

<400> 976

ttcggttgcg cggt 14

<210> 977
<211> 16
<212> DNA
<213> Artificial Sequence

<220>
<223> chemically treated genomic DNA (Homo sapiens)

<400> 977

tttggttgtg tggttg 16

<210> 978
<211> 164
<212> DNA
<213> Artificial Sequence

<220>
<223> chemically treated genomic DNA (Homo sapiens)

<400> 978

gttagtagag attttattaa attttattgt atagtggcgc gcgggcggtc ggtcgagttc 60
ggttgcgcgg ttggcgattt aggagcgagt atagcgttcg ggcgagcgtc gggggggagcg 120
agtaggggcg acgagaaacg aggtagggga gggaagtaga tgtt 164

<210> 979
<211> 84

<212> DNA
<213> Artificial Sequence

<220>
<223> chemically treated genomic DNA (Homo sapiens)

<400> 979

taagagtaat aatggatgga tgatggatag atgaatggat gaagaaagaa aggatgagtg 60
agagaaagga agggagatgg gagg 84

<210> 980
<211> 25
<212> DNA
<213> Artificial Sequence

<220>
<223> chemically treated genomic DNA (Homo sapiens)

<400> 980

taagagtaat aatggatgga tgatg 25

<210> 981
<211> 17
<212> DNA
<213> Artificial Sequence

<220>
<223> chemically treated genomic DNA (Homo sapiens)

<400> 981

cctcccatct cccttcc 17

<210> 982
<211> 25
<212> DNA
<213> Artificial Sequence

<220>
<223> chemically treated genomic DNA (Homo sapiens)

<400> 982

atggatgaag aaagaaagga tgagt 25

<210> 983
<211> 30
<212> DNA
<213> Artificial Sequence

<220>
<223> chemically treated genomic DNA (Homo sapiens)

<400> 983

tttttgtaaa gatagttttg atttaagtat 30

<210> 984
<211> 21

<212> DNA
<213> Artificial Sequence

<220>
<223> chemically treated genomic DNA (Homo sapiens)

<400> 984

caaactttct ccctaaaaac c 21

<210> 985
<211> 17
<212> DNA
<213> Artificial Sequence

<220>
<223> chemically treated genomic DNA (Homo sapiens)

<400> 985

cgccgccaac acgatcg 17

<210> 986
<211> 27
<212> DNA
<213> Artificial Sequence

<220>
<223> chemically treated genomic DNA (Homo sapiens)

<400> 986

caccaccaac acaatcaacc ctaacac 27

<210> 987
<211> 29
<212> DNA
<213> Artificial Sequence

<220>
<223> chemically treated genomic DNA (Homo sapiens)

<400> 987

tgattattat gtttaaggat atttagttg 29

<210> 988
<211> 28
<212> DNA
<213> Artificial Sequence

<220>
<223> chemically treated genomic DNA (Homo sapiens)

<400> 988

caataactct aaaaaaaacc tttaaatc 28

<210> 989
<211> 20
<212> DNA

<213> Artificial Sequence

<220>
<223> chemically treated genomic DNA (Homo sapiens)

<400> 989

cgctccccgc gaatacgacg 20

<210> 990
<211> 29
<212> DNA
<213> Artificial Sequence

<220>
<223> chemically treated genomic DNA (Homo sapiens)

<400> 990

taaacccact ccccacaaat acaacaaac 29

<210> 991
<211> 19
<212> DNA
<213> Artificial Sequence

<220>
<223> chemically treated genomic DNA (Homo sapiens)

<400> 991

catccctaca cttccaaac 19

<210> 992
<211> 21
<212> DNA
<213> Artificial Sequence

<220>
<223> chemically treated genomic DNA (Homo sapiens)

<400> 992

ggagttgtta ggagaaaagt t 21

<210> 993
<211> 21
<212> DNA
<213> Artificial Sequence

<220>
<223> chemically treated genomic DNA (Homo sapiens)

<400> 993

cgaacaccca accgacaaac g 21

<210> 994
<211> 26
<212> DNA
<213> Artificial Sequence

<220>
<223> chemically treated genomic DNA (Homo sapiens)

<400> 994

caaacaccca accaacaaac atctca 26

<210> 995
<211> 20
<212> DNA
<213> Artificial Sequence

<220>
<223> chemically treated genomic DNA (Homo sapiens)

<400> 995

ttgtagggtt tttttgggtt 20

<210> 996
<211> 23
<212> DNA
<213> Artificial Sequence

<220>
<223> chemically treated genomic DNA (Homo sapiens)

<400> 996

ctcaaaaccc ttaaaaacat aaa 23

<210> 997
<211> 21
<212> DNA
<213> Artificial Sequence

<220>
<223> chemically treated genomic DNA (Homo sapiens)

<400> 997

ataaccacac tacgcgcctc c 21

<210> 998
<211> 25
<212> DNA
<213> Artificial Sequence

<220>
<223> chemically treated genomic DNA (Homo sapiens)

<400> 998

ataaccacac tacacacctc ccaca 25

<210> 999
<211> 20
<212> DNA
<213> Artificial Sequence

<220>
<223> chemically treated genomic DNA (Homo sapiens)

<400> 999

ggtgttaggg tttaggggtt 20

<210> 1000
<211> 26
<212> DNA
<213> Artificial Sequence

<220>
<223> chemically treated genomic DNA (Homo sapiens)

<400> 1000

ccaaatattt acctaacact caaata 26

<210> 1001
<211> 25
<212> DNA
<213> Artificial Sequence

<220>
<223> chemically treated genomic DNA (Homo sapiens)

<400> 1001

aactattttc tatcgaaacc gcccg 25

<210> 1002
<211> 30
<212> DNA
<213> Artificial Sequence

<220>
<223> chemically treated genomic DNA (Homo sapiens)

<400> 1002

aactattttc tatcaaaacc acccacctct 30

<210> 1003
<211> 22
<212> DNA
<213> Artificial Sequence

<220>
<223> chemically treated genomic DNA (Homo sapiens)

<400> 1003

gtaggggagg gaagtagatg tt 22

<210> 1004
<211> 24
<212> DNA
<213> Artificial Sequence

<220>

```
<223> chemically treated genomic DNA (Homo sapiens)

<400> 1004

ttctaatcct cctttccaca ataa                                              24

<210> 1005
<211> 20
<212> DNA
<213> Artificial Sequence

<220>
<223> chemically treated genomic DNA (Homo sapiens)

<400> 1005

agtcggagtc gggagagcga                                                   20

<210> 1006
<211> 27
<212> DNA
<213> Artificial Sequence

<220>
<223> chemically treated genomic DNA (Homo sapiens)

<400> 1006

agttggagtt gggagagtga aaggaga                                           27
```

## Description

FIELD OF THE INVENTION

Aspects of the present invention relate to human DNA sequences that exhibit heterogenous expression patterns in prostate cancer patients, and more particularly to novel compositions and methods for providing a prognosis of said patients.

CROSS-REFERENCE TO RELATED APPLICATIONS

This application claims the benefit of priority to United States Provisional Application Serial Numbers: 60/632,426, filed 02 December 2004 and entitled METHODS AND NUCLEIC ACIDS FOR THE ANALYSIS OF GENE EXPRESSION ASSOCIATED WITH THE PROGNOSIS OF PROSTATE CELL PROLIFERATIVE DISORDERS; 60/662,220, filed 14 March 2005 of same title; 60/723,125 filed 03 October 2005 of same title; 60/_,_,filed November 30, 2005 of same title; 60/633,250 filed 02 December 2004 and entitled METHODS AND NUCLEIC ACIDS FOR THE ANALYSIS OF GENE EXPRESSION ASSOCIATED WITH THE DEVELOPMENT OF PROSTATE CELL PROLIFERATIVE DISORDERS; and 60/723,054 filed 03 October 3 2005 of same title, all of which are incorporated by reference herein in their entireties.

SEQUENCE LISTING

A Sequence Listing, pursuant to PCT Administrative Instructions Part 8, Section 801 (a), has been provided on compact disc (1 of 1) as a 3.25 MB text file (476_0001.txt), which is incorporated by reference herein in its entirety.

BACKGROUND

*Prostate cancer*. Prostate cancer is the most common malignancy among men in the United States (~200,000 new cases per year), and the sixth leading cause of male cancer-related deaths worldwide (~204,000 per year). Prostate cancer is primarily a disease of the elderly, with approximately 16% of men between the ages of 60 and 79 having the disease., According to some estimates at autopsy, 80% of all men over 80 years of age have some form of prostate disease (e.g., cancer, BPH, prostatitis, etc). Benign prostate hypertrophy is present in about 50% of men aged 50 or above, and in 95% of men aged 75 or above. Prostate cancer, based on these reports, is often not a disease that men die from, but more typically-with. Recent evidence suggests that the incidence of prostate cancer may in fact be declining, likely as result of better treatment, better surgery, and earlier detection.

*Diagnosis of prostate cancer; molecular approaches*. Current guidelines for prostate cancer screening have been suggested by the American Cancer Society and are as follows: At 50 years of age, health care professionals should offer a blood test for prostate specific antigen (PSA) and perform a digital rectal exam (DRE). It is recommended that high risk populations, such as African Americans and those with a family history of prostate disease, should begin screening at 45 years of age. Men without abnormal prostate pathology generally have a PSA level in blood below 4ng/ml. PSA levels between 4ng/ml and 10ng/ml (called the 'Grey Zone') have a 25% chance of having prostate cancer. The result is that 75% of the time, men with an abnormal DRE and a PSA in this *grey zone* have a negative, or a seemingly unnecessary biopsy. Above the grey zone, the likelihood of having prostate cancer is significant (> 67%) and increases even further as PSA levels go up. Numerous methods exist for measuring PSA (percent-free PSA, PSA velocity, PSA density, etc.), and each has an associated accuracy for detecting the presence of cancer. Yet, even with the minor improvements in detection, and the reported drops in mortality associated with screening, the frequency of false positives remains high. Reduced specificity results in part from increased blood PSA associated with BPH, and prostatis. It has also been estimated that up to 45% of prostate biopsies under current guidelines are falsely negative, resulting in decreased sensitivity even with biopsy.

TRUS guided biopsy is considered the 'gold standard' for diagnosing prostate cancer. Recommendations for biopsy are based upon abnormal PSA levels and or an abnormal DREs. For PSA there is a grey zone where a high percentage of biopsies are perhaps not necessary. Yet the ability to detect cancer in this grey zone (PSA levels of 4.0 to 10 ng/ml) is difficult without biopsy. Due to this lack of specificity, 75% of men undergoing a biopsy do not have cancer. Yet without biopsy, those with cancer would be missed, resulting in increased morbidity and mortality. Unfortunately, the risks associated with an unnecessary biopsy are also high.

Molecular markers would offer the advantage that they can be used to efficiently analyze even very small tissue samples, and samples whose tissue architecture has not been maintained. Within the last decade, numerous genes have been studied with respect to differential expression among benign hyperplasia of the prostate and different grades of prostate cancer. However, no single marker has as yet been shown to be sufficient for the prognostic classification of prostate tumors in a clinical setting.

Alternatively, high-dimensional mRNA-based approaches may, in particular instances, provide a means to distinguish between different tumor types and benign and malignant lesions. However, application of such approaches as a routine diagnostic tool in a clinical environment is impeded and substantially limited by the extreme instability of mRNA, the rapidly occurring expression changes following certain triggers (e.g., sample collection), and, most importantly, by the large amount of mRNA needed for analysis which often cannot be obtained from a routine biopsy (see, e.g., Lipshutz, R. J. et al., Nature Genetics 21:20-24, 1999; Bowtell, D. D. L. Nature Genetics Suppl. 21:25-32, 1999).

Aberrant genetic methylation in prostate cancer has been observed in several genes including GSTPi, AR, p16 (CDKN2a/INK4a), CD44, CDH1. Genome-wide hypomethylation for example of the LINE-1 repetitive element has also been associated with tumor progression (Santourlidis, S., et al., Prostate 39:166-74, 1999).

*Prostate Cancer Treatment Options*. There are many treatment strategies available to patients diagnosed with prostate cancer, and the decision for the patients and physicians is often unclear. Because prostate cancer can be a slowly developing disease, many men choose a treatment approach called watchful waiting, or conservative management. As the names imply, this approach does not include any radical therapy intended to cure the patient. Instead, the disease is carefully monitored using PSA tests and DREs. The ideal patient for this approach is one whose tumor is slow growing and non-invasive, and who is therefore likely to die of other causes before the prostate cancer becomes problematic.

For younger patients with localized disease, curative treatment is more appropriate. Radical prostatectomy is used to remove the prostate and hopefully all traces of the tumor. The surgical margins, seminal vesicles, and sometimes lymph nodes are tested for the presence of cancer, and in each case the presence of cancer correlates with reduced disease free survival Overall, about 70% of men remain free of disease ten years after surgery (Roehl, et al., 2004). Radical prostatectomy is a significant surgery, with side effects including blood loss, incontinence, and impotence. The rate of intraoperative and postoperative complications is estimated to be less than 2% (Lepor, et al., 2001).

Radiation therapy is also used to attempt to cure prostate cancer patients. Patients can choose either external beam radiation or brachytherapy (radioactive seed implants). The rates of survival and the side effects are similar to radical prostatectomy (D'amico, et al., 1998). For both radical prostatectomy and radiation therapy, the probability of survival is highly dependent on the stage and differentiation of the tumor. Localized indolent tumors are more likely to be cured.

Hormonal therapy is often used for patients whose cancer has spread beyond the prostate or for patients whose cancer has recurred after prostatectomy or radiation therapy. In other words, hormonal therapy is used to control cancer but not to cure it. Hormonal therapy is sometimes used in conjunction with other therapies such as radiation or as a neo-adjuvant therapy prior to surgery. The goal of hormonal therapy is to reduce the stimulatory effect of androgens on the prostate tumor. The reduction in hormones is achieved through orchiectomy, lutenizing hormone-releasing hormone (LHRH) analogs, and antiandrogens. Side effects of hormonal therapy can include impotence, hot flashes, fatigue, and reduced libido. Eventually, prostate tumors become insensitive to androgens and hormonal therapy is no longer effective.

After the tumor has spread outside the capsule and hormonal therapy has failed, chemotherapy can be used to relieve pain or delay the progression of the disease. The response to chemotherapy is variable, and lives are extended for only a minority of patients. Bisphosphonates are used to reduce the osteolytic activity of tumors metastasised to the bones.

*Prostate Cancer Prognosis Estimation*. DRE, TRUS, biopsy, and PSA provide initial staging information on the tumor, but MRI, CT scans, ProstaScint scans and bone scans are used to determine the spread of the cancer beyond the prostatic capsule. These tests are not used on every prostate cancer patient, but only those with some likelihood of metastases. If metastases can be confirmed, the patient will receive treatment designed to slow the progression of the disease. If no metastases are detected, a patient is a candidate for potentially curative treatments such as prostatectomy and radiation therapy. Prior to the removal of the prostate, lymph nodes are sometimes dissected as a final test for metastases. If metastases are present in the dissected nodes, the surgery may be aborted. Analysis of the tissue surgically removed during prostatectomy is the final and gold standard staging technique for those patients who choose to undergo surgery. Frequently, analysis of the surgical specimens shows that the patient was originally *understaged* by the diagnostic tests (Bostwick, 1997).

An accurate estimation of prognosis is crucial for selection of the most appropriate treatment for each patient. Since organ confined prostate cancer cannot lead to death, estimation of prognosis is also an estimation of the presence or likelihood of development of metastases. A patient who is likely to develop cancer outside of the prostatic capsule will receive more extensive diagnostic work-up, including MRI and CT scans, and possibly more radical treatments, including surgery and radiation.

An initial prognostic assessment is made from the results of a PSA test, DRE, and biopsy analysis. The size, location, and method of detection of the tumor are combined to give a staging score on the TNM scale. Patients with higher stage tumors and high PSA values are more likely to have cancer that has spread or will spread outside of the prostate. A histological analysis of the biopsy allows a pathologist to determine the Gleason score. The Gleason score is a composite of the two most prevalent grades in the tissue sample, and the grades can range between one and five. A higher grade indicates more extreme dedifferentiation, and higher composite scores correlate with higher probability for metastasis and reduced disease free survival.

Prostate cancer nomograms have been developed and modified to predict the risk of cancer recurrence after primary therapy based on PSA levels, Gleason grading, and pre-operative staging information (Kattan et al 2003; Kattan et al 1998; Potter et al 2001). The data is derived from actual patient survival rates in cohorts of thousands of patients at multiple institutions. As with all prognostic measurements in prostate cancer, the estimate of recurrence risk by the nomogram is also an estimate of the likelihood of presence of cancer outside the prostatic capsule. Because the clinical characteristics of the cancers that patients are presenting with have changed with the widespread use of PSA, the nomograms are out of date and are not widely used. However, the general process of integrating Gleason, stage, and PSA information is still used.

Patients with cancer that has spread to lymph nodes or other metastatic sites are treated with systemic therapies such as hormonal therapies. Patients with localized disease (T1-T3) are candidates for definitive, curative treatments such as surgery or radiation. Those patients with localized disease who are thought to be low-risk are ideal candidates for watchful waiting. Those with intermediate risk are ideal for monotherapy such as surgery or radiation. Those with high risk localized disease should be considered for multimodal therapies or clinical trials.

After surgery, more prognostic information is available because the tumor spread can be directly analyzed. During some prostatectomies, the lymph nodes are directly dissected and the nodal status is confirmed. In all surgeries, the tumor spread to the seminal vesicles and the margin status are checked. Positive nodes, seminal vesicles, and margins all indicate an inferior prognosis and may suggest that the patient should receive adjuvant treatment.

*Molecular prostate cancer prognostic markers; deficiencies of prior art approaches*. As an alternative to current approaches to the prognostic classification of prostate carcinoma patients a variety of molecular approaches are currently being explored. It is anticipated that the development of suitable molecular markers will have significant advantages over current approaches in terms of accuracy, cost-effectiveness and/or patient invasiveness. A variety of molecular markers have been discovered including monoclonal antibodies. In a study by Xu et al (ICDB/95613763) 114 cases of prostate cancer showed that 57% of the bone marrow specimens had elevated OVX1 levels (greater than 7.2 U/ml). In other experiments, OVX1 levels were about 2-fold higher in serum samples from androgen-independent than from androgen-dependent prostate cancer patients (p less than 0.001), suggesting that serum OVX1 levels may be able to predict the progression of prostate cancer, since this disease when it progresses typically becomes androgen-independent. Expression of the PSCA protein and mRNA has been positively correlated with adverse tumor characteristics, such as increasing pathological grade (poor cell differentiation), worsening clinical stage and androgen-independence and speculatively with prostate carcinogenesis (Jpn J Clin Oncol, 4:414-9, 2004). Other prospective mRNA analysis markers include Hepsin. Expression of Hepsin showed significant difference between patients at lower risk (pT2, G2 and Gleason score less than 7) and higher risk (pT3/4, G3 and Gleason score 7 or greater) for relapse (J Urol, 171:187-91, 2004).

The GSTPi gene is the most well characterized prostate carcinoma diagnostic marker. Zhou et al. (J Urol, 171: 2195-8, 2004) recently correlated expression of the GSTPi gene with Gleason grade and cancer volume. Furthermore, use of the gene GSTPi as a marker for the detection of prostate carcinomas located in the peripheral zone (*i.e*., with a high likelihood of metastasis) has also been described in U.S. patent application serial number 10/350,763, which is hereby incorporated by reference in its entirety.

Another methylation marker which may be suitable for the prognostic classification of prostate carcinomas is uPA. Rabbani et al. (The FASEB Journal 17:1081-1088, 2003) have shown that the uPA promoter is hypermethylated in hormone-responsive PrEC and LNCaP cells and hypomethylated in hormone-insensitive PC-3 cells. De-methylation of the promoter in the LNCaP cell lines resulted in increase of mRNA analysis and resulted in an increase in the invasive capacity. Singal et al., analysed methylation of a gene panel consisting of glutathione s-transferase Pi1 (GSTP1), retinoic acid receptor beta (RARB), CD44, E-cadherin (ECAD) and RAS association domain family protein 1A (RASSF1A) in prostate cancer. A methylation index (MI) was calculated as the total number of genes methylated, higher MI was noted in stage III as compared to stage II disease, and in Gleason score 7 as compared to Gleason score 6 samples. Singal et al. thus concluded that the results suggest that the methylation of the gene panel in correlated with clinicopathological features of poor prognosis.

*Pronounced need in the art*. Significantly, however, none of the heretofore mentioned markers are sufficiently developed to provide a marker for the prognosis of prostate cell proliferative disorders that is sufficiently robust and/or accurate for effective use in a clinical setting.

While accurate diagnosis of prostate carcinoma is important, the most pressing need in prostate cancer treatment is for information to guide the treatment planning decision. Leaders in the field agree that many patients with clinically insignificant disease receive unnecessary radical treatments such as prostatectomy or radiation therapy. However, twenty percent of patients who do receive these curative treatments experience disease recurrence. A molecular test could help select patients for the optimal treatment choice and thereby reduce over and under treatment

Currently the therapy choice is made based on the likelihood of spread of the disease. Low-risk patients are candidates for watchful waiting. Medium and high risk patients should receive surgery or radiation, and the high risk patients are candidates for additional adjuvant treatments. Staging, Gleason, and PSA are currently used to estimate this risk, but the combined information from these tests is insufficient. Very few patients are recommended for watchful waiting because clinicians cannot be sure which cancers are indolent. Furthermore, many patients who receive monotherapy experience a recurrence.

Gleason grading currently plays a primary role in prognostic assessment. Patients with localized disease and high Gleason scores (8-10) always undergo radical treatments. Patients with low Gleason scores (2-5) have the option of deferring curative treatment and opting for watchful waiting, however many chose to undergo curative therapy soon after diagnosis. For patients with mid-range Gleason scores, which is the majority of patients diagnosed today, clinicians must use other less-reliable prognostic indicators for further information.

Accordingly there is a pronounced need in the art for a novel, effective prognostic test, and in particular one that would predict the probability that a cancer has or is likely to spread outside of the prostate based on the methylation patterns of biopsy samples. This type of information is highly valuable in the diagnostic and treatment planning processes. This information would initially be used in reaching the decision about whether imaging tests are necessary to check for metastasis for a complete diagnostic work-up. Surgery is unnecessary for any patient whose cancer has already spread, but if a patient is not selected for imaging the metastases will not be detected until surgery or later.

Furthermore there is a pronounced need in the art for a novel and effective prostate cell proliferative disorder molecular classification test, and in particular one that would be suitable for the analysis of biopsy samples to improve the stratification of patients into low, intermediate, and high risk categories so that optimal treatment plans can be selected for each patient. With accurate stratification, patients and doctors can choose watchful waiting with confidence that there is little risk for early recurrence. This test would therefore reduce the number of unnecessary surgeries and radiation treatments.

Additionally, with improved estimations of which patients are likely to recur with monotherapy, physicians can make better use of available adjuvant treatments. If a patient chooses to undergo surgery, the test can be repeated on prostatectomy samples to verify the assessment of his need for adjuvant therapy. The benefits of different adjuvant therapy approaches are still being worked out in clinical trials, and a molecular test could provide valuable information to stratify patients for this additional treatment or for clinical trials.

PITX2 (Paired-like homeodomain transcription factor 2), also known as PTX2, RIEG1, or ARP1, encodes a member of the RIEG/PITX homeobox family, which is in the bicoid class of homeodomain proteins. PITX2 encodes several alternative transcripts, and mutations in the gene lead to the autosomal-dominant disorder Rieger's syndrome, a developmental disorder predominantly affecting the eye (Semina et *al.,* 1996). The protein acts as a transcription factor and is involved in the development of several major organs. It is induced by the WNT pathway, and mediates cell-type specific proliferation by inducing growth-regulating genes (Kioussi et al. 2002).

Toyota *et al*. (2001) found hypermethylation of the gene in a large proportion of acute myeloid leukemias. Several studies by the applicant (see WO 2005/059172) have demonstrated that hypermethylation of PITX2 is associated with poor prognosis for breast cancer patients.

The GPR7 marker is located in a CpG island in the promoter region of an intronless gene on chromosome 10. GPR7, or G-protein receptor 7, is a receptor for neuropeptide W and neuropeptide B (Shimomura et *al.* 2002; Tanaka *et al.* 2003). The expression of GPR7 has been studied in the brain, and is expressed mainly in the cerebellum and frontal cortex (O'Dowd *et al.* 1995). Ishii *et al*. (2003) studied the phenotype of mice lacking a functional copy of GPR7. The mice developed adult-onset obesity and metabolic defects such as decreased energy expenditure and increased blood levels of glucose and insulin. Interestingly, these phenotypes were only detected in male mice. The GPR7 ligands, neuropeptides W and B, have also been implicated in metabolism and obesity in separate studies (Samson *et al.* 2004; Levine *et al.* 2005). GPR7, which is similar in sequence to opioid receptors, may also have a role in pain signaling (Zaratin et *al.* 2005).

SEQ ID NO: 63 is located within the regulatory region of HIST2H2BF on chromosome 1 in a region with several histone genes. The histone content and status of chromatin can influence the expression of the encoded gene. Methylation and altered expression of a histone gene in prostate cancer could cause chromatin changes throughout the genome that alter gene expression in ways that result in more aggressive tumor properties. There are no published articles on the function of this particular histone.

The marker referred to as SEQ ID NO: 35 is located on chromosome 3 downstream of the FOXL2 (Forkhead transcription factor) gene and within or near predicted genes or ESTs. Although it is downstream, it is anticipated that methylation of this marker effects the expression of FOXL2, which is mutated in the blepharophimosis-ptosis epicanthus inversus syndrome (BPES). This syndrome is characterized by eye, craniofacial, and ovarian abnormalities. Methylation of the marker may also affect the expression of the EST, or the EST may be shown to be an alternative exon for the FOXL2 gene.

SUMMARY OF THE INVENTION

The present invention provides novel and efficacious methods and nucleic acids for providing a prognosis of prostate cell proliferative disorders.

The invention solves this longstanding need in the art by providing genes, genomic sequences and/or regulatory regions thereof according to Table 11 (or to one or more of those), the expression thereof being indicative of the prognosis of prostate cell proliferative disorders or features thereof. It is particularly preferred that said genes, genomic sequences and/or regulatory regions are selected from the group consisting PITX2, SEQ ID NO: 63, GPR7 and SEQ ID NO: 35. Further preferred is the gene PITX2. In a particularly preferred embodiment of the invention, the methylation status of CpG positions of genes, genomic sequences and/or regulatory regions thereof according to Table 11 (or to one or more of those) is indicative of the prognosis of prostate cell proliferative disorders or features thereof. It is particularly preferred that said genes, genomic sequences and/or regulatory regions are selected from the group consisting PITX2, SEQ ID NO: 63, GPR7 and SEQ ID NO: 35.Further preferred is the gene PITX2. It is particularly preferred that said prostate cell proliferative disorder is a prostate carcinoma or prostate neoplasm.

To enable this analysis the invention provides a method for the analysis of biological samples for genomic methylation associated with the development of prostate cell proliferative disorders. Said method is **characterised in that** at least one nucleic acid, or a fragment thereof, from the group consisting of SEQ ID NO:1 to SEQ ID NO:64 and SEQ ID NO: 961 (preferably SEQ ID Nos: 35, 63, 19 and most preferably SEQ ID NO: 961) is/are contacted with a reagent or series of reagents capable of distinguishing between methylated and non methylated CpG dinucleotides within the genomic sequence, or sequences of interest.

It is particularly preferred that the method and nucleic acids according to the invention are utilised for at least one of: prognosis of; treatment of; monitoring of; and treatment and monitoring of prostate cell proliferative disorders.

The present invention provides a method for ascertaining genetic and/or epigenetic parameters of genomic DNA. The method has utility for the improved prognostic classification of prostate cell proliferative disorders, more specifically by enabling the improved identification of and differentiation between aggressive and non-aggressive forms of said disorder. The invention presents several substantial improvements over the state of the art. Although some methylation assays for the detection of cancer are known, there is currently no molecular classification system for the *prognostic* classification of tumours.

The DNA source may be any suitable source. Preferably, the source of the DNA sample is selected from the group consisting of cells or cell lines, histological slides, biopsies, paraffin-embedded tissue, bodily fluids, ejaculate, urine, blood, and combinations thereof. Preferably, the source is biopsies, bodily fluids, ejaculate, urine, or blood.

Specifically, the present invention provides a method for providing a prognosis of prostate cell proliferative disorders, comprising: obtaining a biological sample comprising genomic nucleic acid(s); contacting the nucleic acid(s), or a fragment thereof, with one reagent or a plurality of reagents sufficient for distinguishing between methylated and non methylated CpG dinucleotide sequences within a target sequence of the subject nucleic acid, wherein the target sequence comprises, or hybridizes under stringent conditions to, a sequence comprising at least 16 contiguous nucleotides of SEQ ID NO:1 to SEQ ID NO:64 and SEQ ID NO: 961, (preferably SEQ ID Nos: 35, 63, 19 and most preferably SEQ ID NO: 961) said contiguous nucleotides comprising at least one CpG dinucleotide sequence; and determining, based at least in part on said distinguishing, the methylation state of at least one target CpG dinucleotide sequence, or an average, or a value reflecting an average methylation state of a plurality of target CpG dinucleotide sequences. Preferably, distinguishing between methylated and non methylated CpG dinucleotide sequences within the target sequence comprises methylation state-dependent conversion or non-conversion of at least one such CpG dinucleotide sequence to the corresponding converted or non-converted dinucleotide sequence within a sequence selected from the group consisting of SEQ ID NO:65 to SEQ ID NO:320 and SEQ ID NO: 962 to SEQ ID NO: 965, and contiguous regions thereof corresponding to the target sequence. Preferably said sequence is selected from the goup consisting of SEQ ID Nos: 133,134,261,262, 189,190,317,318, 101,102,229,230 and most preferably said sequence is selected from the goup consisting of SEQ ID Nos: 962 - 965

Additional embodiments provide a method for providing a prognosis of prostate cell proliferative disorders, comprising: obtaining a biological sample having subject genomic DNA; extracting the genomic DNA; treating the genomic DNA, or a fragment thereof, with one or more reagents to convert 5-position unmethylated cytosine bases to uracil or to another base that is detectably dissimilar to cytosine in terms of hybridization properties; contacting the treated genomic DNA, or the treated fragment thereof, with an amplification enzyme and at least two primers comprising, in each case a contiguous sequence at least 9 nucleotides in length that is complementary to, or hybridizes under moderately stringent or stringent conditions to a sequence selected from the group consisting SEQ ID NO:65 to SEQ ID NO:320 and SEQ ID NO: 962 to SEQ ID NO: 965 (preferably said group consists of SEQ ID Nos: 133,134,261,262, 189,190,317,318, 101,102,229,230 and most preferably said group consists of SEQ ID Nos: 962 - 965) and complements thereof, wherein the treated DNA or the fragment thereof is either amplified to produce an amplificate, or is not amplified; and determining, based on a presence or absence of, or on a property of said amplificate, the methylation state of at least one CpG dinucleotide sequence selected from the group consisting of SEQ ID NO:1 to SEQ ID NO:64 and SEQ ID NO: 961 (preferably said group consists of SEQ ID Nos: 35, 63, 19 and most preferably is SEQ ID NO: 961), or an average, or a value reflecting an average methylation state of a plurality of CpG dinucleotide sequences thereof.

Preferably, at least one such hybridizing nucleic acid molecule or peptide nucleic acid molecule is bound to a solid phase. Preferably, determining comprises use of at least one method selected from the group consisting of: hybridizing at least one nucleic acid molecule comprising a contiguous sequence at least 9 nucleotides in length that is complementary to, or hybridizes under moderately stringent or stringent conditions to a sequence selected from the group consisting of SEQ ID NO:65 to SEQ ID NO:320, (preferably said group consists of SEQ ID Nos: 133,134,261,262, 189,190,317,318, 101,102,229,230 and most preferably said group consists of SEQ ID Nos: 962 - 965) and complements thereof; hybridizing at least one nucleic acid molecule, bound to a solid phase, comprising a contiguous sequence at least 9 nucleotides in length that is complementary to, or hybridizes under moderately stringent or stringent conditions to a sequence selected from the group consisting of SEQ ID NO:65 to SEQ ID NO:320 and SEQ ID NO: 962 to SEQ ID NO: 965, (preferably said group consists of SEQ ID Nos: 133,134,261,262, 189,190,317,318, 101,102,229,230 and most preferably said group consists of SEQ ID Nos: 962 - 965), and complements thereof; hybridizing at least one nucleic acid molecule comprising a contiguous sequence at least 9 nucleotides in length that is complementary to, or hybridizes under moderately stringent or stringent conditions to a sequence selected from the group consisting of SEQ ID NO:65 to SEQ ID NO:320 and SEQ ID NO: 962 to SEQ ID NO: 965 (preferably said group consists of SEQ ID Nos: 133,134,261,262, 189,190,317,318, 101,102,229,230 and most preferably said group consists of SEQ ID Nos: 962 - 965), and complements thereof, and extending at least one such hybridized nucleic acid molecule by at least one nucleotide base; and sequencing of the amplificate.

Further embodiments provide a method for providing a prognosis of prostate cell proliferative disorders, comprising: obtaining a biological sample having subject genomic DNA; extracting the genomic DNA; contacting the genomic DNA, or a fragment thereof, comprising one or more sequences selected from the group consisting of SEQ ID NO:1 to SEQ ID NO:64 and SEQ ID NO: 961 (preferably said group consists of SEQ ID Nos: 35, 63, 19 and most preferably said sequence is SEQ ID NO: 961) or a sequence that hybridizes under stringent conditions thereto, with one or more methylation-sensitive restriction enzymes, wherein the genomic DNA is either digested thereby to produce digestion fragments, or is not digested thereby; and determining, based on a presence or absence of, or on property of at least one such fragment, the methylation state of at least one CpG dinucleotide sequence of one or more sequences selected from the group consisting of SEQ ID NO:1 to SEQ ID NO:64 and SEQ ID NO: 961 , or an average, or a value reflecting an average methylation state of a plurality of CpG dinucleotide sequences thereof. Preferably said group consists of SEQ ID Nos: 35, 63, 19 and most preferably said sequence is SEQ ID NO: 961. Preferably, the digested or undigested genomic DNA is amplified prior to said determining.

Additional embodiments provide novel genomic and chemically modified nucleic acid sequences, as well as oligonucleotides and/or PNA-oligomers for analysis of cytosine methylation patterns within sequences from the group consisting of SEQ ID NO:1 to SEQ ID NO:64 and SEQ ID NO: 961.

## BRIEF DESCRIPTION OF THE DRAWINGS

Figure 1 shows a ROC plot for a SEQ ID NO:19 MSP assay run on 26 frozen radical prostatectomy samples from patients with early PSA recurrence, and on 30 samples from patients with no PSA recurrence after at least 48 months.

Figure 2 shows a ROC plot for a SEQ ID NO:35 MSP assay run on 26 frozen radical prostatectomy samples from patients with early PSA recurrence and 30 samples from patients with no PSA recurrence after at least 48 months.

Figure 3 shows a ROC plot for a SEQ ID NO: 37 MSP assay run on 26 frozen radical prostatectomy samples from patients with early PSA recurrence and 30 samples from patients with no PSA recurrence after at least 48 months.

Figure 4 shows a ROC plot for a SEQ ID NO: 7 MSP assay run on 26 frozen radical prostatectomy samples from patients with early PSA recurrence and 30 samples from patients with no PSA recurrence after at least 48 months.

Figure 5 shows a ROC plot for a SEQ ID NO: 63 MSP assay run on 26 frozen radical prostatectomy samples from patients with early PSA recurrence and 30 samples from patients with no PSA recurrence after at least 48 months.

Figure 6 shows a ROC plot for a SEQ ID NO: 8 MSP assay run on 26 frozen radical prostatectomy samples from patients with early PSA recurrence and 30 samples from patients with no PSA recurrence after at least 48 months.

Figure 7 shows a ROC plot for a SEQ ID NO: 64 MSP assay run on 26 frozen radical prostatectomy samples from patients with early PSA recurrence and 30 samples from patients with no PSA recurrence after at least 48 months.

Figure 8 shows a gel electrophoresis analysis on 12 DNA samples. 200 ng per DNA was applied to a 0.8 % agarose gel. The gel was run for 4 hours at 80 Volt. The size marker (Invitrogen, No.: 10496-016) contains the following fragments: 10.000 bp, 6.000 bp.

Figure 9 shows ALF Express analyses of multiplex PCR products (8plex, mPCR SetD2) compared to single PCR products (sPCR Set D2). The size standard (lanes 1,4) contained fragments of the following lengths: 50, 100, 150, 200, 250, 300, 350, 400, 450, 500 bp. All fragments could be amplified. An undesired side product (220 bp) was observed.

Figure 10 shows performance of multiplex PCR. Lane 1: 100bp marker. Lanes 2-11: multiplex PCR performance of 10 test samples, lane 12: positive control, lane 13: H2O control.

Figure 11 shows Tumor vs. Lymphocyte samples, ranked by Wilcoxon statistics. Bonferroni corrected p-values (upper) and AUCs (lower) are shown to the right of the data matrix. Each column represents one sample; each row one oligonucleotide. Oligonucleotides are grouped per marker candidate. The indicated markers are ordered from top to bottom with increasing AUC. On the right side of each marker Bonferroni corrected Wilcoxon p-value and AUC are given. Below the AUC sensitivity at a specificity of - 0.75 are given enclosed in brackets. Methylation data are centered and normalized to one standard deviation for individual oligonucleotides. The color represents the relative distance of the oligonucleotide methylation status from the mean value. Light grey represents hypomethylated CpGs within an oligonucleotide while dark grey indicates hypermethylated CpGs within an oligonucleotide.

Figure 12 shows high Gleason vs. Low Gleason marker rankings. The plot displays uncorrected p-values from the genewise Wilcoxon rank statistics analysis. Lower and upper dotted lines show 5% Bonferroni and FDR limits, respectively.

Figure 13 shows high Gleason vs. Low Gleason methylation matrix of the 10 markers with best AUC. Gleason scores are shown above each group of samples. Each column represents one sample; each row one oligonucleotide (1, 2, or 3 CpG sites each). Oligonucleotides are grouped per marker candidate. The indicated markers are ordered from top to bottom with increasing AUC. On the right side of each marker Bonferroni corrected Wilcoxon p-value and AUC are given. Below the AUC sensitivity at a specificity of - 0.75 are given enclosed in brackets. Methylation data are centered and normalized to one standard deviation for individual oligonucleotides. The color represents the relative distance of the oligonucleotide methylation status from the mean value. Light grey represents hypomethylated CpGs within an oligonucleotide while dark grey indicates hypermethylated CpGs within an oligonucleotide.

Figure 14 shows Early Recurrence vs. No recurrence marker rankings. The plot gives uncorrected p-values from the genewise Wilcoxon rank test analysis. Lower and upper dotted lines show 5% Bonferroni and FDR limits, respectively.

Figure 15 shows Early Recurrence vs. No recurrence methylation matrix of the 10 markers with best AUC. Each column represents one sample; each row one oligonucleotide (1, 2, or 3 CpG sites each). Oligonucleotides are grouped per marker candidate. The indicated markers are ordered from top to bottom with increasing AUC. On the right side of each marker Bonferroni corrected Wilcoxon p-value and AUC are given. Below the AUC sensitivity at a specificity of ~ 0.75 are given enclosed in brackets. Methylation data are centered and normalized to one standard deviation for individual oligonucleotides. The color represents the relative distance of the oligonucleotide methylation status from the mean value. Light grey represents hypomethylated CpGs within an oligonucleotide while dark grey indicates hypermethylated CpGs within an oligonucleotide.

For Figures 16-88, each figure shows the sequence of the analysed amplificate of each respective SEQ ID NO. In each figure, an analyzed amplificate is displayed in a 'wrapped' series of panels, where the first row (top row) in each panel shows the *genomic* sequence being amplified (the genomic sequence row), and where forward and reverse amplification primers (defining an 'amplicon') are shown in the panel row (the primer display row) immediately below the first row. The row below the primer display row (or, in panels not displaying a primer, the row below the genomic display row) is the bisulfite converted sequence of the amplificate (the bisulfite converted sequence row; wherein CpG positions are marked red). The remaining rows displayed in some panels, show the sequences of detection oligonucleotides (CG and TG oligos) used to analyze the amplificate.

Figure 16 shows an Amplificate of SEQ ID NO:14.
Figure 17 shows an Amplificate of SEQ ID NO:15.
Figure 18 shows an Amplificate of SEQ ID NO:16.
Figure 19 shows an Amplificate of SEQ ID NO:17.
Figure 20 shows an Amplificate of SEQ ID NO:18
Figure 21 shows an Amplificate of SEQ ID NO:19
Figure 22 shows an Amplificate of SEQ ID NO:20
Figure 23 shows an Amplificate of SEQ ID NO:21
Figure 24 shows an Amplificate of SEQ ID NO:22
Figure 25 shows an Amplificate of SEQ ID NO:23
Figure 26 shows an Amplificate of SEQ ID NO:24
Figure 27 shows an Amplificate of SEQ ID NO:25
Figure 28 shows an Amplificate of SEQ ID NO:26
Figure 29 shows an Amplificate of SEQ ID NO:27
Figure 30 shows an Amplificate of SEQ ID NO:28

Figure 31 shows an Amplificate of SEQ ID NO:29
Figure 32 shows an Amplificate of SEQ ID NO:30
Figure 33 shows an Amplificate of SEQ ID NO:31
Figure 34 shows an Amplificate of SEQ ID NO:32 (amplificate A)
Figure 35 shows an Amplificate of SEQ ID NO:32 (amplificate B)
Figure 36 shows an Amplificate of SEQ ID NO:33
Figure 37 shows an Amplificate of SEQ ID NO:34
Figure 38 shows an Amplificate of SEQ ID NO:35
Figure 39 shows an Amplificate of SEQ ID NO:13
Figure 40 shows an Amplificate of SEQ ID NO:36
Figure 41 shows an Amplificate of SEQ ID NO:37
Figure 42 shows an Amplificate of SEQ ID NO:1
Figure 43 shows an Amplificate of SEQ ID NO:2
Figure 44 shows an Amplificate of SEQ ID NO:3
Figure 45 shows an Amplificate of SEQ ID NO:4
Figure 46 shows an Amplificate of SEQ ID NO:5
Figure 47 shows an Amplificate of SEQ ID NO:6
Figure 48 shows an Amplificate of SEQ ID NO:7
Figure 49 shows an Amplificate of SEQ ID NO:38
Figure 50 shows an Amplificate of SEQ ID NO:39
Figure 60 shows an Amplificate of SEQ ID NO:40
Figure 61 shows an Amplificate of SEQ ID NO:41
Figure 62 shows an Amplificate of SEQ ID NO:42
Figure 63 shows an Amplificate of SEQ ID NO:43
Figure 64 shows an Amplificate of SEQ ID NO:44
Figure 65 shows an Amplificate of SEQ ID NO:45
Figure 66 shows an Amplificate of SEQ ID NO:46
Figure 67 shows an Amplificate of SEQ ID NO:47
Figure 68 shows an Amplificate of SEQ ID NO:48
Figure 69 shows an Amplificate of SEQ ID NO:49
Figure 70 shows an Amplificate of SEQ ID NO:50
Figure 71 shows an Amplificate of SEQ ID NO:51
Figure 72 shows an Amplificate of SEQ ID NO:52
Figure 73 shows an Amplificate of SEQ ID NO:53
Figure 74 shows an Amplificate of SEQ ID NO:54
Figure 75 shows an Amplificate of SEQ ID NO:55
Figure 76 shows an Amplificate of SEQ ID NO:56
Figure 77 shows an Amplificate of SEQ ID NO:57
Figure 78 shows an Amplificate of SEQ ID NO:58
Figure 79 shows an Amplificate of SEQ ID NO:59
Figure 80 shows an Amplificate of SEQ ID NO:60
Figure 81 shows an Amplificate of SEQ ID NO:61
Figure 82 shows an Amplificate of SEQ ID NO:62
Figure 83 shows an Amplificate of SEQ ID NO:8
Figure 84 shows an Amplificate of SEQ ID NO:9
Figure 85 shows an Amplificate of SEQ ID NO:10
Figure 86 shows an Amplificate of SEQ ID NO:11
Figure 87 shows an Amplificate of SEQ ID NO:12 (amplificate A)
Figure 88 shows an Amplificate of SEQ ID NO:12 (amplificate B)
Figure 89 shows the distribution of follow up times of patients as analysed in Example 5. The white bars represent the distribution of all censored (no PSA relapse) patients. The grey bars show the distribution of the PSA-free survival time for all of the relapse patients. Frequency is shown on the Y-axis and time (months) is shown on the X-axis.
Figure 90 shows Kaplan-Meier survival analysis of the PITX2 marker (A & B) and ROC curve analysis (C) of the marker PITX2 in differentiating between prostate cancer patients according to Example 5. Proportion of recurrence-free patients is shown on the Y-axis, time in years is shown on the x-axis.
Figure 91 shows Kaplan-Meier survival analysis of the GPR7 marker (A & B) and ROC curve analysis (C) of the marker PITX2 in differentiating between prostate cancer patients according to Example 5. Proportion of recurrence-free patients is shown on the Y-axis, time in years is shown on the x-axis.

Figure 92 shows Kaplan-Meier survival analysis of the SEQ ID NO: 63 marker (A & B) and ROC curve analysis (C) of the marker PITX2 in differentiating between prostate cancer patients according to Example 5. Proportion of recurrence-free patients is shown on the Y-axis, time in years is shown on the x-axis.

Figure 93 shows Kaplan-Meier survival analysis of the SEQ ID NO: 35 marker (A & B) and ROC curve analysis (C) of the marker PITX2 in differentiating between prostate cancer patients according to Example 5. Proportion of recurrence-free patients is shown on the Y-axis, time in years is shown on the x-axis.

Figure 94 shows Kaplan-Meier survival analysis of the ABHD9 marker (A & B) and ROC curve analysis (C) of the marker PITX2 in differentiating between prostate cancer patients according to Example 5. Proportion of recurrence-free patients is shown on the Y-axis, time in years is shown on the x-axis.

Figure 95 shows Kaplan-Meier survival analysis of the CCND2 marker (A & B) and ROC curve analysis (C) of the marker PITX2 in differentiating between prostate cancer patients according to Example 5. Proportion of recurrence-free patients is shown on the Y-axis, time in years is shown on the x-axis.

Figure 96 shows Kaplan-Meier survival analysis of PITX2 performance on sub-populations based on stage according to Example 5.

Figure 97 shows Kaplan-Meier survival analysis of PITX2 performance on sub-populations based on Gleason score according to Example 5.

Figure 98 shows Kaplan-Meier survival analysis of PITX2 performance on sub-populations based on nomogram score according to Example 5.

Figure 99 shows Kaplan-Meier survival analysis of SEQ ID NO: 63 performance on sub-populations based on High Gleason score according to Example 5.

Figure 100 shows Kaplan-Meier survival analysis of SEQ ID NO: 63 performance on sub-populations based on poor nomogram score according to Example 5.

Figure 101 shows Kaplan-Meier survival analysis of SEQ ID NO: 35 performance on T2 sub-populations according to Example 5.

Figure 102 shows the detected amplificate in both frozen and PET samples in the early biochemical relapse vs. no biochemical relapse comparisons using the assay of SEQ ID NO: 19 shown in Table 12 as detailed in Example 6.

Figure 103 shows the detected amplificate in both frozen and PET samples in the early biochemical relapse vs. no biochemical relapse comparisons using the assay of SEQ ID NO: 63 shown in Table 12 as detailed in Example 6.

Figure 104 shows the detected amplificate in both frozen and PET samples in the early biochemical relapse vs. no biochemical relapse comparisons using the assay of SEQ ID NO: 35 shown in Table 12 as detailed in Example 6.

Figure 105 shows the detected amplificate in both frozen and PET samples in the early biochemical relapse vs. no biochemical relapse comparisons using the assay of SEQ ID NO: 37 shown in Table 12 as detailed in Example 6.

Figure 106 shows the detected amplificate in PET samples only in the early biochemical relapse vs. no biochemical relapse comparisons using the assay of SEQ ID NO: 19 shown in Table 12 as detailed in Example 6.

Figure 107 shows the detected amplificate in PET samples only in the early biochemical relapse vs. no biochemical relapse comparisons using the assay of SEQ ID NO: 63 shown in Table 12 as detailed in Example 6.

Figure 108 shows the detected amplificate in PET samples only in the early biochemical relapse vs. no biochemical relapse comparisons using the assay of SEQ ID NO: 35 shown in Table 12 as detailed in Example 6.

Figure 109 shows the detected amplificate in PET samples only in the early biochemical relapse vs. no biochemical relapse comparisons using the assay of SEQ ID NO: 37 shown in Table 12 as detailed in Example 6.

Figure 110 shows the detected amplificate in frozen samples only in the early biochemical relapse vs. no biochemical relapse comparisons using the assay of SEQ ID NO: 19 shown in Table 12 as detailed in Example 6.

Figure 111 shows the detected amplificate in frozen samples only in the early biochemical relapse vs. no biochemical relapse comparisons using the assay of SEQ ID NO: 63 shown in Table 12 as detailed in Example 6.

Figure 112 shows the detected amplificate in frozen samples only in the early biochemical relapse vs. no biochemical relapse comparisons using the assay of SEQ ID NO: 35 shown in Table 12 as detailed in Example 6.

Figure 113 shows the detected amplificate in frozen samples only in the early biochemical relapse vs. no biochemical relapse comparisons using the assay of SEQ ID NO: 37 shown in Table 12 as detailed in Example 6.

Figure 114 shows the detected amplificate in both frozen and PET samples in the High Gleason vs. Low Gleason comparisons using the assay of SEQ ID NO: 19 shown in Table 12 as detailed in Example 6.

Figure 115 shows the detected amplificate in both frozen and PET samples in the High Gleason vs. Low Gleason comparisons using the assay of SEQ ID NO: 63 shown in Table 12 as detailed in Example 6.

Figure 116 shows the detected amplificate in both frozen and PET samples in the High Gleason vs. Low Gleason comparisons using the assay of SEQ ID NO: 35 shown in Table 12 as detailed in Example 6.

Figure 117 shows the detected amplificate in both frozen and PET samples in the High Gleason vs. Low Gleason comparisons using the assay of SEQ ID NO: 37 shown in Table 12 as detailed in Example 6.

Figure 118 shows the detected amplificate in PET samples only in the High Gleason vs. Low Gleason comparisons using the assay of SEQ ID NO: 19 shown in Table 12 as detailed in Example 6.

Figure 119 shows the detected amplificate in PET samples only in the High Gleason vs. Low Gleason comparisons using the assay of SEQ ID NO: 63 shown in Table 12 as detailed in Example 6.
Figure 120 shows the detected amplificate in PET samples only in the High Gleason vs. Low Gleason comparisons using the assay of SEQ ID NO: 35 shown in Table 12 as detailed in Example 6.
Figure 121 shows the detected amplificate in PET samples only in the High Gleason vs. Low Gleason comparisons using the assay of SEQ ID NO: 37 shown in Table 12 as detailed in Example 6.
Figure 122 shows the detected amplificate in frozen samples only in the High Gleason vs. Low Gleason comparisons using the assay of SEQ ID NO: 19 shown in Table 12 as detailed in Example 6.
Figure 123 shows the detected amplificate in frozen samples only in the High Gleason vs. Low Gleason comparisons using the assay of SEQ ID NO: 63 shown in Table 12 as detailed in Example 6.
Figure 124 shows the detected amplificate in frozen samples only in the High Gleason vs. Low Gleason comparisons using the assay of SEQ ID NO: 35 shown in Table 12 as detailed in Example 6.
Figure 125 shows the detected amplificate in frozen samples only in the High Gleason vs. Low Gleason comparisons using the assay of SEQ ID NO: 37 shown in Table 12 as detailed in Example 6.

## DETAILED DESCRIPTION OF THE INVENTION

Definitions:

As used herein the term expression shall be taken to mean the transcription and translation of a gene. The level of expression of a gene may be determined by the analysis of any factors associated with or indicative of the level of transcription and translation of a gene including but not limited to methylation analysis, loss of heterozygosity (hereinafter also referred to as LOH), RNA expression levels and protein expression levels.
Furthermore the activity of the transcribed gene may be affected by genetic variations such as but not limited genetic mutations (including but not limited to SNPs, point mutations, deletions, insertions, repeat length, rearrangements and other polymorphisms).
As used herein the term "prognosis" shall be taken to mean a prediction of the progression of the disease (for example but not limited to regression, stasis and metastasis), in particular aggressiveness and metastatic potential of a prostate tumor.
As used herein the term "prognostic marker" shall be taken to mean an indicator of a prediction of the progression of the disease, in particular aggressiveness and metastatic potential of a prostate tumor.
As used herein the term "prognostic classification" shall be taken to mean the classification of a prostate cell proliferative disorder according to a prediction of the progression of the disease, in particular aggressiveness and metastatic potential of a prostate tumor.
It is preferably used to define patients with high, low and intermediate risks of death or recurrence after treatment that result from the inherent heterogeneity of the disease process. As used herein the term "aggressive" as used with respect to prostate tumor shall be taken to mean a prostate cell proliferative disorder that has the biological capability to rapidly spread outside of the prostate. Indicators of tumor aggressivness standard in the art include but are not limited to tumor stage, tumor grade, Gleason grade, nodal status and survival. As used herein the term "survival" shall not be limited to mean survival until mortality (wherein said mortality may be either irrespective of cause or prostate cell proliferative disorder related) but may be used in combination with other terms to define clinical terms, for example but not limited to "recurrence-free survival" (wherein the term recurrence shall include both localized and distant recurrence); metastasis free survival; disease free survival (wherein the term disease shall include prostate cancer and diseases associated therewith). The length of said survival may be calculated by reference to a defined start point (e.g. time of diagnosis or start of treatment) and a defined end point (e.g. death, recurrence or metastasis).
The term "Observed/Expected Ratio" ("O/E Ratio") refers to the frequency of CpG dinucleotides within a particular DNA sequence, and corresponds to the [number of CpG sites / (number of C bases x number of G bases)].
The term "CpG island" refers to a contiguous region of genomic DNA that satisfies the criteria of

(1) having a frequency of CpG dinucleotides corresponding to an "Observed/Expected Ratio" >0.6, and
(2) having a "GC Content" >0.5. CpG islands are typically, but not always, between about 0.2 to about 1 kb, or to about 2kb in length.

The term "methylation state" or "methylation status" refers to the presence or absence of 5-methylcytosine ("5-mCyt") at one or a plurality of CpG dinucleotides within a DNA sequence. Methylation states at one or more particular CpG methylation sites (each having two CpG CpG dinucleotide sequences) within a DNA sequence include "unmethylated," "fully-methylated" and "hemi-methylated."
The term "hemi-methylation" or "hemimethylation" refers to the methylation state of a palindromic CpG methylation site, where only a single cytosine in one of the two CpG dinucleotide sequences of the palindromic CpG methylation site is methylated (e.g., 5'-CC$^{M}$GG-3' (top strand): 3'-GGCC-5' (bottom strand)).

The term 'AUC' as used herein is an abbreviation for the area under a curve. In particular it refers to the area under a Receiver Operating Characteristic (ROC) curve. The ROC curve is a plot of the true positive rate against the false positive rate for the different possible cutpoints of a diagnostic test. It shows the tradeoff between sensitivity and specificity depending on the selected cutpoint (any increase in sensitivity will be accompanied by a decrease in specificity).The area under an ROC curve (AUC) is a measure for the accuracy of a diagnostic test (the larger the area the better, optimum is 1, a random test would have a ROC curve lying on the diagonal with an area of 0.5; for reference: J.P. Egan. Signal Detection Theory and ROC Analysis, Academic Press, New York, 1975).

The term "hypermethylation" refers to the average methylation state corresponding to an increased presence of 5-mCyt at one or a plurality of CpG dinucleotides within a DNA sequence of a test DNA sample, relative to the amount of 5-mCyt found at corresponding CpG dinucleotides within a normal control DNA sample.

The term "hypomethylation" refers to the average methylation state corresponding to a decreased presence of 5-mCyt at one or a plurality of CpG dinucleotides within a DNA sequence of a test DNA sample, relative to the amount of 5-mCyt found at corresponding CpG dinucleotides within a normal control DNA sample.

The term "microarray" refers broadly to both "DNA microarrays," and 'DNA chip(s),' as recognized in the art, encompasses all art-recognized solid supports, and encompasses all methods for affixing nucleic acid molecules thereto or synthesis of nucleic acids thereon.

"Genetic parameters" are mutations and polymorphisms of genes and sequences further required for their regulation. To be designated as mutations are, in particular, insertions, deletions, point mutations, inversions and polymorphisms and, particularly preferred, SNPs (single nucleotide polymorphisms).

"Epigenetic parameters" are, in particular, cytosine methylations. Further epigenetic parameters include, for example, the acetylation of histones which, however, cannot be directly analyzed using the described method but which, in turn, correlate with the DNA methylation.

The term "bisulfite reagent" refers to a reagent comprising bisulfite, disulfite, hydrogen sulfite or combinations thereof, useful as disclosed herein to distinguish between methylated and unmethylated CpG dinucleotide sequences.

The term "Methylation assay" refers to any assay for determining the methylation state of one or more CpG dinucleotide sequences within a sequence of DNA.

The term "MS.AP-PCR" (Methylation-Sensitive Arbitrarily-Primed Polymerase Chain Reaction) refers to the art-recognized technology that allows for a global scan of the genome using CG-rich primers to focus on the regions most likely to contain CpG dinucleotides, and described by Gonzalgo et al., Cancer Research 57:594-599, 1997.

The term "MethyLight™" refers to the art-recognized fluorescence-based real-time PCR technique described by Eads et al., Cancer Res. 59:2302-2306, 1999.

The term "HeavyMethyl™" assay, in the embodiment thereof implemented herein, refers to an assay, wherein methylation specific *blocking* probes (also referred to herein as *blockers*) covering CpG positions between, or covered by the amplification primers enable methylation-specific selective amplification of a nucleic acid sample.

The term "HeavyMethyl™ MethyLight" assay, in the embodiment thereof implemented herein, refers to a HeavyMethyl™ MethyLight™ assay, which is a variation of the MethyLight™ assay, wherein the MethyLight™ assay is combined with methylation specific *blocking* probes covering CpG positions between the amplification primers.

The term "Ms-SNuPE" (Methylation-sensitive Single Nucleotide Primer Extension) refers to the art-recognized assay described by Gonzalgo & Jones, Nucleic Acids Res. 25:2529-2531, 1997.

The term "MSP" (Methylation-specific PCR) refers to the art-recognized methylation assay described by Herman et al. Proc. Natl. Acad. Sci. USA 93:9821-9826, 1996, and by US Patent No. 5,786,146.

The term "COBRA" (Combined Bisulfite Restriction Analysis) refers to the art-recognized methylation assay described by Xiong & Laird, Nucleic Acids Res. 25:2532-2534, 1997.

The term "MCA" (Methylated CpG Island Amplification) refers to the methylation assay described by Toyota et al., Cancer Res. 59:2307-12, 1999, and in WO 00/26401A1.

The term "hybridization" is to be understood as a bond of an oligonucleotide to a complementary sequence along the lines of the Watson-Crick base pairings in the sample DNA, forming a duplex structure.

"Stringent hybridization conditions," as defined herein, involve hybridizing at 68°C in 5x SSC/5x Denhardt's solution/1.0% SDS, and washing in 0.2x SSC/0.1% SDS at room temperature, or involve the art-recognized equivalent thereof (e.g., conditions in which a hybridization is carried out at 60°C in 2.5 x SSC buffer, followed by several washing steps at 37°C in a low buffer concentration, and remains stable). Moderately stringent conditions, as defined herein, involve including washing in 3x SSC at 42°C, or the art-recognized equivalent thereof. The parameters of salt concentration and temperature can be varied to achieve the optimal level of identity between the probe and the target nucleic acid. Guidance regarding such conditions is available in the art, for example, by Sambrook et al., 1989, Molecular Cloning, A Laboratory Manual, Cold Spring Harbor Press, N.Y.; and Ausubel et al. (eds.), 1995, Current Protocols in Molecular Biology, (John Wiley & Sons, N.Y.) at Unit 2.10.

The terms "array SEQ ID NO," "composite array SEQ ID NO," or "composite array sequence" refer to a sequence, hypothetical or otherwise, consisting of a head-to-tail (5' to 3') linear composite of all individual contiguous sequences of a subject array (e.g., a head-to-tail composite of SEQ ID NO:1-71, in that order).

The terms "array SEQ ID NO node," "composite array SEQ ID NO node," or "composite array sequence node" refer to a *junction* between any two individual contiguous sequences of the "array SEQ ID NO," the "composite array SEQ ID NO," or the "composite array sequence."

In reference to composite array sequences, the phrase "contiguous nucleotides" refers to a contiguous sequence region of any individual contiguous sequence of the composite array, but does not include a region of the composite array sequence that includes a "node," as defined herein above.

Overview:

The present invention provides for molecular genetic markers that have novel utility for providing a prognosis of prostate cell proliferative disorders. In particular embodiments said markers may be used for classifying the tumor according to aggressiveness and/or invasiveness. It is particularly preferred that the method and nucleic acids according to the invention are utilised for at least one of: prognosis of; treatment of; monitoring of; and treatment and monitoring of prostate cell proliferative disorders.

The term 'prognosis' is taken to mean a prediction of outcome of disease progression (wherein the term progression shall be taken to also include recurrence after treatment). Prognosis may be expressed in terms of overall patient survival, disease- or relapse-free survival, increased tumor-related complications and rate of progression of tumour or metastases, wherein a decrease in any of said factors (with the exception of increased tumor-related complications rate of progression) as relative to a pre-determined level, is a 'negative' outcome and increase thereof is a 'positive' outcome. A decrease in tumor-related complications and/or rate of progression of tumour or metastases as relative to a pre-determined level, is considered a 'positive' outcome and increase thereof is a, 'negative' outcome.

Hereinafter prognosis may also be referred to in terms of 'aggressiveness' wherein an aggressive cancer is determined to have a high risk of negative outcome and wherein a non-aggressive cancer has a low risk of negative outcome.

In one aspect the prognostic marker according to the present invention is used to provide an estimate of the risk of negative outcome. Characterisation of a prostate cancer in terms of predicted outcome enables the physician to determine the risk of recurrence and/or death. This aids in treatment selection as the absolute reduction of risk of recurrence and death after treatments such as adjuvant hormonal, chemo-, and radiation therapy can be determined based on the predicted negative outcome. The absolute reduction in risk attributable to treatment may then be compared to the drawbacks of said treatment (e.g. side effects, cost) in order to determine the suitability of said treatment for the patient.

Conversely, wherein a cancer is characterised as non-aggressive (i.e. positive outcome with low risk of death and/or recurrence) the patient will derive low absolute benefit from adjuvant or other treatment and may be appropriately treated by watchful waiting. Therein lies a great advantage of the present invention. By providing a means for determining which patients will not significantly benefit from treatment the present invention identifies suitable candidates for watchful waiting and prevents the over-prescription of therapies.

According to the predicted outcome (i.e. prognosis) of the disease an appropriate treatment or treatments may be selected. Wherein a cancer is characterised as aggressive it is particularly preferred that adjuvant treatment such as, but not limited to, hormonal, chemo- or radiation therapy is provided in addition to or instead of further treatments.

The herein described markers have further utility in predicting outcome of a patient after surgical treatment. This will hereinafter also be referred to as a 'predictive' marker. Over expression of the genes according to Table 11 (in particular FOXL2, SEQ ID NO: 35, SEQ ID NO: 63, HIST2H2BF, GPR7 and most preferably PITX2), are associated with negative outcome of prostate cancer patients. Patients with predicted positive outcome (i.e. hypomethylation or over-expression) after said treatment will accordingly have a decreased absolute reduction of risk of recurrence and death after treatment with post surgical adjuvant therapies. Patients with predicted negative outcome (i.e. hypermethylation) after said treatment will accordingly have a relatively larger absolute reduction of risk of recurrence and death after post surgical adjuvant treatment. Accordingly patients with a negative outcome after said treatment will be considered more suitable candidates for adjuvant treatment than patients with a positive outcome. Patients with a positive outcome may accordingly be prevented from over prescription of adjuvant treatment.

*Bisulfite modification of DNA is an art-recognized tool used to assess CpG methylation status*. 5-methylcytosine is the most frequent covalent base modification in the DNA of eukaryotic cells. It plays a role, for example, in the regulation of the transcription, in genetic imprinting, and in tumorigenesis. Therefore, the identification of 5-methylcytosine as a component of genetic information is of considerable interest. However, 5-methylcytosine positions cannot be identified by sequencing, because 5-methylcytosine has the same base pairing behavior as cytosine. Moreover, the epigenetic information carried by 5-methylcytosine is completely lost during, e.g., PCR amplification.

The most frequently used method for analyzing DNA for the presence of 5-methylcytosine is based upon the specific reaction of bisulfite with cytosine whereby, upon subsequent alkaline hydrolysis, cytosine is converted to uracil, which corresponds to thymine in its base pairing behavior. Significantly, however, 5-methylcytosine remains unmodified under these conditions. Consequently, the original DNA is *converted* in such a manner that methylcytosine, which originally could not be distinguished from cytosine by its hybridization behavior, can now be detected as the only remaining cytosine using standard, art-recognized molecular biological techniques, for example, by amplification and hybridization, or by sequencing. All of these techniques are based on differential base pairing properties, which can now be fully exploited.

The prior art, in terms of sensitivity, is defined by a method comprising enclosing the DNA to be analyzed in an agarose matrix, thereby preventing the diffusion and renaturation of the DNA (bisulfite only reacts with single-stranded DNA), and replacing all precipitation and purification steps with fast dialysis (Olek A, et al., A modified and improved method for bisulfite based cytosine methylation analysis, Nucleic Acids Res. 24:5064-6, 1996). It is thus possible to analyze individual cells for methylation status, illustrating the utility and sensitivity of the method. An overview of art-recognized methods for detecting 5-methylcytosine is provided by Rein, T., et al., Nucleic Acids Res., 26:2255, 1998.

The bisulfite technique, barring few exceptions (e.g., Zeschnigk M, et al., Eur J Hum Genet. 5:94-98, 1997), is currently only used in research. In all instances, short, specific fragments of a known gene are amplified subsequent to a bisulfite treatment, and either completely sequenced (Olek & Walter, Nat Genet. 1997 17:275-6, 1997), subjected to one or more primer extension reactions (Gonzalgo & Jones, Nucleic Acids Res., 25:2529-31, 1997; WO 95/00669; U.S. Patent No. 6,251,594) to analyze individual cytosine positions, or treated by enzymatic digestion (Xiong & Laird, Nucleic Acids Res., 25:2532-4, 1997). Detection by hybridization has also been described in the art (Olek et al., WO 99/28498). Additionally, use of the bisulfite technique for methylation detection with respect to individual genes has been described (Grigg & Clark, Bioessays, 16:431-6, 1994; Zeschnigk M, et al., Hum Mol Genet., 6:387-95, 1997; Feil R, et al., Nucleic Acids Res., 22:695-, 1994; Martin V, et al., Gene, 157:261-4, 1995; WO 9746705 and WO 9515373).

The present invention provides for the use of the bisulfite technique , in combination with one or more methylation assays, for determination of the methylation status of CpG dinuclotide sequences within sequences from the group consisting of SEQ ID NO:1 to SEQ ID NO:64 and SEQ ID NO: 961. Preferably said group consists of SEQ ID Nos: 35, 63, 19 and most preferably said sequence is SEQ ID NO: 961 According to the present invention, determination of the methylation status of CpG dinuclotide sequences within sequences from the group consisting of SEQ ID NO:1 to SEQ ID NO:64 and SEQ ID NO: 961 and SEQ ID NO: 961 has prognostic utility.

*Methylation Assay Procedures.* Various methylation assay procedures are known in the art, and can be used in conjunction with the present invention. These assays allow for determination of the methylation state of one or a plurality of CpG dinucleotides (e.g., CpG islands) within a DNA sequence. Such assays involve, among other techniques, DNA sequencing of bisulfite-treated DNA, PCR (for sequence-specific amplification), Southern blot analysis, and use of methylation-sensitive restriction enzymes.

For example, genomic sequencing has been simplified for analysis of DNA methylation patterns and 5-methylcytosine distribution by using bisulfite treatment (Frommer et al., Proc. Natl. Acad. Sci. USA 89:1827-1831, 1992). Additionally, restriction enzyme digestion of PCR products amplified from bisulfite-converted DNA is used, e.g., the method described by Sadri & Hornsby (Nucl. Acids Res. 24:5058-5059, 1996), or COBRA (Combined Bisulfite Restriction Analysis) (Xiong & Laird, Nucleic Acids Res. 25:2532-2534, 1997).

COBRA. COBRA analysis is a quantitative methylation assay useful for determining DNA methylation levels at specific gene loci in small amounts of genomic DNA (Xiong & Laird, Nucleic Acids Res. 25:2532-2534, 1997). Briefly, restriction enzyme digestion is used to reveal methylation-dependent sequence differences in PCR products of sodium bisulfite-treated DNA. Methylation-dependent sequence differences are first introduced into the genomic DNA by standard bisulfite treatment according to the procedure described by Frommer et al. (Proc. Natl. Acad. Sci. USA 89:1827-1831, 1992). PCR amplification of the bisulfite converted DNA is then performed using primers specific for the CpG islands of interest, followed by restriction endonuclease digestion, gel electrophoresis, and detection using specific, labeled hybridization probes. Methylation levels in the original DNA sample are represented by the relative amounts of digested and undigested PCR product in a linearly quantitative fashion across a wide spectrum of DNA methylation levels. In addition, this technique can be reliably applied to DNA obtained from microdissected paraffin-embedded tissue samples. Typical reagents (e.g., as might be found in a typical COBRA-based kit) for COBRA analysis may include, but are not limited to: PCR primers for specific gene (or bisulfite treated DNA sequence or CpG island); restriction enzyme and appropriate buffer; gene-hybridization oligo; control hybridization oligo; kinase labeling kit for oligo probe; and labelled nucleotides. Additionally, bisulfite conversion reagents may include: DNA denaturation buffer; sulfonation buffer; DNA recovery reagents or kits (e.g., precipitation, ultrafiltration, affinity column); desulfonation buffer; and DNA recovery components.

Preferably, assays such as "MethyLight™" (a fluorescence-based real-time PCR technique) (Eads et al., Cancer Res. 59:2302-2306, 1999), Ms-SNuPE (Methylation-sensitive Single Nucleotide Primer Extension) reactions (Gonzalgo & Jones, Nucleic Acids Res. 25:2529-2531, 1997), methylation-specific PCR ("MSP"; Herman et al., Proc. Natl. Acad.

Sci. USA 93:9821-9826, 1996; US Patent No. 5,786,146), and methylated CpG island amplification ("MCA"; Toyota et al., Cancer Res. 59:2307-12, 1999) are used alone or in combination with other of these methods.

*MethyLight*™. The MethyLight™ assay is a high-throughput quantitative methylation assay that utilizes fluorescence-based real-time PCR (TaqMan™) technology that requires no further manipulations after the PCR step (Eads et al., Cancer Res. 59:2302-2306, 1999). Briefly, the MethyLight™ process begins with a mixed sample of genomic DNA that is converted, in a sodium bisulfite reaction, to a mixed pool of methylation-dependent sequence differences according to standard procedures (the bisulfite process converts unmethylated cytosine residues to uracil). Fluorescence-based PCR is then performed either in an "unbiased" (with primers that do not overlap known CpG methylation sites) PCR reaction, or in a "biased" (with PCR primers that overlap known CpG dinucleotides) reaction. Sequence discrimination can occur either at the level of the amplification process or at the level of the fluorescence detection process, or both.

The MethyLight™ assay may be used as a quantitative test for methylation patterns in the genomic DNA sample, wherein sequence discrimination occurs at the level of probe hybridization. In this quantitative version, the PCR reaction provides for unbiased amplification in the presence of a fluorescent probe that overlaps a particular putative methylation site. An unbiased control for the amount of input DNA is provided by a reaction in which neither the primers, nor the probe overlie any CpG dinucleotides. Alternatively, a qualitative test for genomic methylation is achieved by probing of the biased PCR pool with either control oligonucleotides that do not "cover" known methylation sites (a fluorescence-based version of the "MSP" technique), or with oligonucleotides covering potential methylation sites.

The MethyLight™ process can by used with a "TaqMan®" probe in the amplification process. For example, double-stranded genomic DNA is treated with sodium bisulfite and subjected to one of two sets of PCR reactions using TaqMan® probes; e.g., with either biased primers and TaqMan® probe, or unbiased primers and TaqMan® probe. The TaqMan® probe is dual-labeled with fluorescent "reporter" and "quencher" molecules, and is designed to be specific for a relatively high GC content region so that it melts out at about 10˚C higher temperature in the PCR cycle than the forward or reverse primers. This allows the TaqMan® probe to remain fully hybridized during the PCR annealing/extension step. As the Taq polymerase enzymatically synthesizes a new strand during PCR, it will eventually reach the annealed TaqMan® probe. The Taq polymerase 5' to 3' endonuclease activity will then displace the TaqMan® probe by digesting it to release the fluorescent reporter molecule for quantitative detection of its now unquenched signal using a real-time fluorescent detection system.

Typical reagents (e.g., as might be found in a typical MethyLightTM-based kit) for MethyLightTM analysis may include, but are not limited to: PCR primers for specific gene (or bisulfite treated DNA sequence or CpG island); TaqMan® probes; optimized PCR buffers and deoxynucleotides; and Taq polymerase.

*Ms-SNuPE.* The Ms-SNuPE technique is a quantitative method for assessing methylation differences at specific CpG sites based on bisulfite treatment of DNA, followed by single-nucleotide primer extension (Gonzalgo & Jones, Nucleic Acids Res. 25:2529-2531, 1997). Briefly, genomic DNA is reacted with sodium bisulfite to convert unmethylated cytosine to uracil while leaving 5-methylcytosine unchanged. Amplification of the desired target sequence is then performed using PCR primers specific for bisulfite-converted DNA, and the resulting product is isolated and used as a template for methylation analysis at the CpG site(s) of interest. Small amounts of DNA can be analyzed (e.g., microdissected pathology sections), and it avoids utilization of restriction enzymes for determining the methylation status at CpG sites.

Typical reagents (*e.g.*, as might be found in a typical Ms-SNuPE-based kit) for Ms-SNuPE analysis may include, but are not limited to: PCR primers for specific gene (or bisulfite treated DNA sequence or CpG island); optimized PCR buffers and deoxynucleotides; gel extraction kit; positive control primers; Ms-SNuPE primers for specific gene; reaction buffer (for the Ms-SNuPE reaction); and labelled nucleotides. Additionally, bisulfite conversion reagents may include: DNA denaturation buffer; sulfonation huffer; DNA recovery regents or kit (e.g., precipitation, ultrafiltration, affinity column); desulfonation buffer; and DNA recovery components.

*MSP.* MSP (methylation-specific PCR) allows for assessing the methylation status of virtually any group of CpG sites within a CpG island, independent of the use of methylation-sensitive restriction enzymes (Herman et al. Proc. Natl. Acad. Sci. USA 93:9821-9826, 1996; US Patent No. 5,786,146). Briefly, DNA is modified by sodium bisulfite converting all unmethylated, but not methylated cytosines to uracil, and subsequently amplified with primers specific for methylated versus unmethylated DNA. MSP requires only small quantities of DNA, is sensitive to 0.1% methylated alleles of a given CpG island locus, and can be performed on DNA extracted from paraffin-embedded samples. Typical reagents (e.g., as might be found in a typical MSP-based kit) for MSP analysis may include, but are not limited to: methylated and unmethylated PCR primers for specific gene (or bisulfite treated DNA sequence or CpG island), optimized PCR buffers and deoxynucleotides, and specific probes.

MCA. The MCA technique is a method that can be used to screen for altered methylation patterns in genomic DNA, and to isolate specific sequences associated with these changes (Toyota et al., Cancer Res. 59:2307-12, 1999). Briefly, restriction enzymes with different sensitivities to cytosine methylation in their recognition sites are used to digest genomic DNAs from primary tumors, cell lines, and normal tissues prior to arbitrarily primed PCR amplification. Fragments that show differential methylation are cloned and sequenced after resolving the PCR products on high-resolution polyacrylamide gels. The cloned fragments are then used as probes for Southern analysis to confirm differential methylation of these regions. Typical reagents (e.g., as might be found in a typical MCA-based kit) for MCA analysis may include, but are not limited to: PCR primers for arbitrary priming Genomic DNA; PCR buffers and nucleotides, restriction enzymes and appropriate buffers; gene-hybridization oligos or probes; control hybridization oligos or probes.

Genomic Sequences According to SEQ ID NOS:1-64 and SEQ ID NO: 961 (preferably SEQ ID Nos: 35, 63, 19 and most preferably SEQ ID NO: 961), and Non-naturally Occuring Treated Variants Thereof According to SEQ ID NOS: 65-320 and SEQ ID Nos: 962 - 965 (preferably SEQ ID Nos: 133,134,261,262, 189,190,317,318, 101,102,229,230 and most preferably SEQ ID Nos: 962-965), were Determined to have Utility for Providing a Prognosis and/or Treatment of Prostate Cell Proliferative Disorders.

In one embodiment the invention provides a method for providing a prognosis of prostate cell proliferative disorders in a subject. In a particularly preferred embodiment the invention provides a method for the classification based on aggressiveness of a prostate cell proliferative disorder.

Said method comprises the following steps:

i) determining the expression levels of one or more genes or gene sequences according to Table 11 and/or regulatory regions thereof; and
ii) determining the prognosis of said prostate cell proliferative disorders according to said level of expression.

Said expression level may be determined by any means standard in the art including but not limited to methylation analysis, loss of heterozygosity (hereinafter also referred to as LOH), RNA expression levels and protein expression levels.

Accordingly said method may be enabled by means of any analysis of the expression of a RNA transcribed therefrom or polypeptide or protein translated from said RNA, preferably by means of mRNA expression analysis or polypeptide expression analysis. Accordingly the present invention also provides prognostic assays and methods, both quantitative and qualitative for detecting the expression of the genes, genomic sequences and/or regulatory regions according to Table 11 in a subject with a prostate carcinoma or neoplasms and determining therefrom upon the prognosis and/or prediction of treatment outcome in said subject. It is particularly preferred that said genes, genomic sequences and/or regulatory regions are selected from the group consisting PITX2, SEQ ID NO: 63, GPR7 and SEQ ID NO: 35. Further preferred is the gene PITX2.

Aberrant expression of mRNA transcribed from the genes or genomic regions according to Table 11, in particular SEQ ID NO: 35, SEQ ID NO: 63 and GPR7 and most preferably PITX2 are associated with prognosis and/or prediction of treatment outcome of prostate carcinoma. To detect the presence of mRNA encoding a gene or genomic sequence, a sample is obtained from a patient. The sample may be any suitable sample comprising cellular matter of the tumour, most preferably the primary tumour. Suitable sample types include tumours cells or cell lines, histological slides, paraffin embedded tissues, biopsies, tissue embedded in paraffin, bodily fluids (such as but not limited to prostatic massage fluid and urine) or any other suitable biological sample and all possible combinations thereof.

In a particularly preferred embodiment of the method said source is primary tumour tissue. The sample may be treated to extract the RNA contained therein. The resulting nucleic acid from the sample is then analysed. Many techniques are known in the state of the art for determining absolute and relative levels of gene expression, commonly used techniques suitable for use in the present invention include in situ hybridisation (e.g. FISH), Northern analysis, RNase protection assays (RPA), microarrays and PCR-based techniques, such as quantitative PCR and differential display PCR or any other nucleic acid detection method.

Particularly preferred is the use of the reverse transcription/polymerisation chain reaction technique (RT-PCR). The method of RT-PCR is well known in the art (for example, see Watson and Fleming, supra).

The RT-PCR method can be performed as follows. Total cellular RNA is isolated by, for example, the standard guanidium isothiocyanate method and the total RNA is reverse transcribed. The reverse transcription method involves synthesis of DNA on a template of RNA using a reverse transcriptase enzyme and a 3' end oligo dT primer and/or random hexamer primers. The cDNA thus produced is then amplified by means of PCR. (Belyavsky et al, Nucl Acid Res 17: 2919-2932, 1989; Krug and Berger, Methods in Enzymology, Academic Press, N.Y., Vol.152, pp. 316-325, 1987 which are incorporated by reference). Further preferred is the "Real-time" variant of RT- PCR, wherein the PCR product is detected by means of hybridisation probes (E.g TaqMan, Lightcycler, Molecular Beacons & Scorpion) or SYBR green. The detected signal from the probes or SYBR green is then quantitated either by reference to a standard curve or by comparing the Ct values to that of a calibration standard. Analysis of housekeeping genes is often used to normalize the results.

In Northern blot analysis total or poly(A)+ mRNA is run on a denaturing agarose gel and detected by hybridization to a labelled probe in the dried gel itself or on a membrane. The resulting signal is proportional to the amount of target RNA in the RNA population. Comparing the signals from two or more cell populations or tissues reveals relative differences in gene expression levels. Absolute quantitation can be performed by comparing the signal to a standard curve generated using known amounts of an in vitro transcript corresponding to the target RNA. Analysis of housekeeping genes, genes whose expression levels are expected to remain relatively constant regardless of conditions, is often used to normalize the results, eliminating any apparent differences caused by unequal transfer of RNA to the membrane or unequal loading of RNA on the gel.

The first step in Northern analysis is isolating pure, intact RNA from the cells or tissue of interest. Because Northern blots distinguish RNAs by size, sample integrity influences the degree to which a signal is localized in a single band. Partially degraded RNA samples will result in the signal being smeared or distributed over several bands with an overall loss in sensitivity and possibly an erroneous interpretation of the data. In Northern blot analysis, DNA, RNA and oligonucleotide probes can be used and these probes are preferably labelled (e.g. radioactive labels, massa labels or fluorescent labels). The size of the target RNA, not the probe, will determine the size of the detected band, so methods such as random-primed labeling, which generates probes of variable lengths, are suitable for probe synthesis. The specific activity of the probe will determine the level of sensitivity, so it is preferred that probes with high specific activities, are used..

In an RNase protection assay, the RNA target and an RNA probe of a defined length are hybridized in solution. Following hybridization, the RNA is digested with RNases specific for single-stranded nucleic acids to remove any unhybridized, single-stranded target RNA and probe. The RNases are inactivated, and the RNA is separated e.g. by denaturing polyacrylamide gel electrophoresis. The amount of intact RNA probe is proportional to the amount of target RNA in the RNA population. RPA can be used for relative and absolute quantitation of gene expression and also for mapping RNA structure, such as intron/exon boundaries and transcription start sites. The RNase protection assay is preferable to Northern blot analysis as it generally has a lower limit of detection.

The antisense RNA probes used in RPA are generated by in vitro transcription of a DNA template with a defined endpoint and are typically in the range of 50-600 nucleotides. The use of RNA probes that include additional sequences not homologous to the target RNA allows the protected fragment to be distinguished from the full-length probe. RNA probes are typically used instead of DNA probes due to the ease of generating single-stranded RNA probes and the reproducibility and reliability of RNA:RNA duplex digestion with RNases (Ausubel *et al*. 2003), particularly preferred are probes with high specific activities.

Particularly preferred is the use of microarrays. The microarray analysis process can be divided into two main parts. First is the immobilization of known gene sequences onto glass slides or other solid support followed by hybridization of the fluorescently labelled cDNA (comprising the sequences to be interrogated) to the known genes immobilized on the glass slide. After hybridization, arrays are scanned using a fluorescent microarray scanner. Analyzing the relative fluorescent intensity of different genes provides a measure of the differences in gene expression. DNA arrays can be generated by immobilizing presynthesized oligonucleotides onto prepared glass slides. In this case, representative gene sequences are manufactured and prepared using standard oligonucleotide synthesis and purification methods. These synthesized gene sequences are complementary to the genes of interest (most preferably PITX2) and tend to be shorter sequences in the range of 25-70 nucleotides. Alternatively, immobilized oligos can be chemically synthesized in-situ on the surface of the slide. In situ oligonucleotide synthesis involves the consecutive addition of the appropriate nucleotides to the spots on the microarray; spots not receiving a nucleotide are protected during each stage of the process using physical or virtual masks. In expression profiling microarray experiments, the RNA templates used are representative of the transcription profile of the cells or tissues under study. RNA is first isolated from the cell populations or tissues to be compared. Each RNA sample is then used as a template to generate fluorescently labelled cDNA via a reverse transcription reaction. Fluorescent labeling of the cDNA can be accomplished by either direct labeling or indirect labeling methods. During direct labeling, fluorescently modified nucleotides (e.g., Cy®3- or Cy®5-dCTP) are incorporated directly into the cDNA during the reverse transcription. Alternatively, indirect labeling can be achieved by incorporating aminoallyl-modified nucleotides during cDNA synthesis and then conjugating an N-hydroxysuccinimide (NHS)-ester dye to the aminoallyl-modified cDNA after the reverse transcription reaction is complete. Alternatively, the probe may be unlabelled, but may be detectable by specific binding with a ligand which is labelled, either directly or indirectly. Suitable labels and methods for labelling ligands (and probes) are known in the art, and include, for example, radioactive labels which may be incorporated by known methods (e.g., nick translation or kinasing). Other suitable labels include but are not limited to biotin, fluorescent groups, chemiluminescent groups (e.g., dioxetanes, particularly triggered dioxetanes), enzymes, antibodies, and the like.

To perform differential gene expression analysis, cDNA generated from different RNA samples are labelled with Cy®3. The resulting labelled cDNA is purified to remove unincorporated nucleotides, free dye and residual RNA. Following purification, the labeled cDNA samples are hybridised to the microarray. The stringency of hybridisation is determined by a number of factors during hybridisation and during the washing procedure, including temperature, ionic strength, length of time and concentration of formamide. These factors are outlined in, for example, Sambrook et al. (Molecular Cloning: A Laboratory Manual, 2nd ed., 1989). The microarray is scanned post-hybridization using a fluorescent microarray scanner. The fluorescent intensity of each spot indicates the level of expression for that gene; bright spots correspond to strongly expressed genes, while dim spots indicate weak expression.. Once the images are obtained, the raw data must be analyzed. First, the background fluorescence must be subtracted from the fluorescence of each spot. The data is then normalized to a control sequence, such as an exogenously added RNA, or a housekeeping gene panel to account for any nonspecific hybridization, array imperfections or variability in the array setup, cDNA labeling, hybridization or washing. Data normalization allows the results of multiple arrays to be compared.

The present invention further provides for methods for the detection of the presence of the polypeptide encoded by said gene sequences in a sample obtained from a patient.

Aberrant levels of polypeptide expression of the polypeptides encoded by the genes and/or genomic regions according to Table 11 (in particular SEQ ID NO: 35, SEQ ID NO: 63, GPR7 and most preferably PITX2) are associated with prostate cancer and neoplasms prognosis and/or treatment outcome.

Any method known in the art for detecting polypeptides can be used. Such methods include, but are not limited to masss-spectrometry, immunodiffusion, immunoelectrophoresis, immunochemical methods, binder-ligand assays, immunohistochemical techniques, agglutination and complement assays (e.g., see Basic and Clinical Immunology, Sites and Terr, eds., Appleton & Lange, Norwalk, Conn. pp 217-262, 1991 which is incorporated by reference). Preferred are binder-ligand immunoassay methods including reacting antibodies with an epitope or epitopes and competitively displacing a labelled polypeptide or derivative thereof.

Certain embodiments of the present invention comprise the use of antibodies specific to the polypeptide encoded by the genes and/or genomic regions according to Table 11 (in particular SEQ ID NO: 35, SEQ ID NO: 63, GPR7 and most preferably PITX2).

Such antibodies are useful for prostate cancer prognostic and/or predictive applications. In certain embodiments production of monoclonal or polyclonal antibodies can be induced by the use of the coded polypeptide as an antigene. Such antibodies may in turn be used to detect expressed polypeptides as markers for prostate cancer prognosis. The levels of such polypeptides present may be quantified by conventional methods. Antibody-polypeptide binding may be detected and quantified by a variety of means known in the art, such as labelling with fluorescent or radioactive ligands. The invention further comprises kits for performing the above-mentioned procedures, wherein such kits contain antibodies specific for the investigated polypeptides.

Numerous competitive and non-competitive polypeptide binding immunoassays are well known in the art. Antibodies employed in such assays may be unlabelled, for example as used in agglutination tests, or labelled for use a wide variety of assay methods. Labels that can be used include radionuclides, enzymes, fluorescers, chemiluminescers, enzyme substrates or co-factors, enzyme inhibitors, particles, dyes and the like. Preferred assays include but are not limited to radioimmunoassay (RIA), enzyme immunoassays, e.g., enzyme-linked immunosorbent assay (ELISA), fluorescent immunoassays and the like. Polyclonal or monoclonal antibodies or epitopes thereof can be made for use in immunoassays by any of a number of methods known in the art.

In an alternative embodiment of the method the proteins may be detected by means of western blot analysis. Said analysis is standar in the art, briefly proteins are separated by means of electrophoresis e.g. SDS-PAGE. The separated proteins are then transferred to a suitable membrane (or paper) e.g. nitrocellulose, retaining the spacial separation achieved by electrophoresis. The membrane is then incubated with a generic protein (e.g. milk protein) to bind remaining sticky places on the membrane. An antibody specific to the protein of interest is then added, said antibody being detectably labelled for example by dyes or enzymatic means (e.g. alkaline phosphatase or horseradish peroxidase). The location of the antibody on the membrane is then detected.

In an alternative embodiment of the method the proteins may be detected by means of immunohistochemistry (the use of antibodies to probe specific antigens in a sample). Said analysis is standard in the art, wherein detection of antigens in tissues is known as immunohistochemistry, while detection in cultured cells is generally termed immunocytochemistry. Briefly the primary antibody to be detected by binding to its specific antigen. The antibody-antigen complex is then bound by a secondary enzyme conjugated antibody. In the presence of the necessary substrate and chromogen the bound enzyme is detected according to colored deposits at the antibody-antigen binding sites. There is a wide range of suitable sample types, antigen-antibody affinity, antibody types, and detection enhancement methods. Thus optimal conditions for immunohistochemical or immunocytochemical detection must be determined by the person skilled in the art for each individual case.

One approach for preparing antibodies to a polypeptide is the selection and preparation of an amino acid sequence of all or part of the polypeptide, chemically synthesising the amino acid sequence and injecting it into an appropriate animal, usually a rabbit or a mouse (Milstein and Kohler Nature 256:495-497, 1975; Gulfre and Milstein, Methods in Enzymology: Immunochemical Techniques 73:1-46, Langone and Banatis eds., Academic Press, 1981 which are incorporated by reference). Methods for preparation of the polypeptides or epitopes thereof include, but are not limited to chemical synthesis, recombinant DNA techniques or isolation from biological samples.

In the final step of the method the prognosis of the patient is determined, whereby overexpression is indicative of negative prognosis. The term overexpression shall be taken to mean expression at a detected level greater than a pre-determined cut off which may be selected from the group consisting of the mean, median or an optimised threshold value.

Another aspect of the invention provides a kit for use in providing a prognosis of a subject with prostate cancer, comprising: a means for detecting polypeptides of a gene or genomic region according to Table 11 (in particular SEQ ID NO: 35, SEQ ID NO: 63 and GPR7 and most preferably PITX2). The means for detecting the polypeptides comprise preferably antibodies, antibody derivatives, or antibody fragments. The polypeptides are most preferrably detected by means of Western blotting utilizing a labelled antibody. In another embodiment of the invention the kit further comprising means for obtaining a biological sample of the patient. Preferred is a kit, which further comprises a container suitable for containing the means for detecting the polypeptides in the biological sample of the patient, and most preferably further comprises instructions for use and interpretation of the kit results. In a preferred embodiment the kit for use in determining treatment strategy for a patient with prostate cancer or neoplasms, comprises: (a) a means for detecting polypeptides of a gene or genomic region according to Table 11 (in particular SEQ ID NO: 35, SEQ ID NO: 63 and GPR7 and most preferably PITX2); (b) a container suitable for containing the said means and the biological sample of the patient comprising the polypeptides wherein the means can form complexes with the polypeptides; (c) a means to detect the complexes of (b); and optionally (d) instructions for use and interpretation of the kit results.

The kit may also contain other components such as buffers or solutions suitable for blocking, washing or coating , packaged in a separate container.

Another aspect of the invention relates to a kit for use in providing a prognosis of a subject with prostate cancer, said kit comprising: a means for measuring the level of transcription of a gene or genomic region according to Table 11 (in particular SEQ ID NO: 35, SEQ ID NO: 63 and GPR7 and most preferably PITX2). In a preferred embodiment the means for measuring the level of transcription comprise oligonucleotides or polynucleotides able to hybridise under stringent or moderately stringent conditions to the transcription products of a gene or genomic region according to Table 11 (in particular SEQ ID NO: 35, SEQ ID NO: 63 and GPR7 and most preferably PITX2). In a most preferred embodiment the level of transcription is determined by techniques selected from the group of Northern blot analysis, reverse transcriptase PCR, real-time PCR, RNAse protection, and microarray. In another embodiment of the invention the kit further comprises means for obtaining a biological sample of the patient. Preferred is a kit, which further comprises a container suitable for containing the means for measuring the level of transcription and the biological sample of the patient, and most preferably further comprises instructions for use and interpretation of the kit results.

In a preferred embodiment the kit for use in determining treatment strategy for a patient with prostate cancer comprises (a) a plurality of oligonucleotides or polynucleotides able to hybridise under stringent or moderately stringent conditions to the transcription products of a gene or genomic region according to Table 11 (in particular SEQ ID NO: 35, SEQ ID NO: 63 and GPR7 and most preferably PITX2); (b) a container suitable for containing the oligonucleotides or polynucleotides and a biological sample of the patient comprising the transcription products wherein the oligonucleotides or polynucleotide can hybridise under stringent or moderately stringent conditions to the transcription products, (c) means to detect the hybridisation of (b); and optionally, (d) instructions for use and interpretation of the kit results. The kit may also contain other components such as hybridization buffer (where the oligonucleotides are to be used as a probe) packaged in a separate container. Alternatively, where the oligonucleotides are to be used to amplify a target region, the kit may contain, packaged in separate containers, a polymerase and a reaction buffer optimized for primer extension mediated by the polymerase, such as PCR.

Most preferably a kit according to the embodiments of the present invention is used for the determination of expression step of the methods according to other aspects of the invention.

In a further aspect, the invention provides a further method for providing a prognosis of a subject with prostate cancer comprising the following steps. In the first step of the method a sample is obtained from the subject. Suitable sample types include tumours cells or cell lines, histological slides, paraffin embedded tissues, biopsies, tissue embedded in paraffin, bodily fluids (such as but not limited to prostatic massage fluid and urine) or any other suitable biological sample and all possible combinations thereof.. Commonly used techniques suitable for use in the present invention include in situ hybridisation (e.g. FISH), Northern analysis, RNase protection assays (RPA), microarrays and PCR-based techniques, such as quantitative PCR and differential display PCR or any other nucleic acid detection method.

Particularly preferred is the use of the reverse transcription/polymerisation chain reaction technique (RT-PCR). The method of RT-PCR is well known in the art (for example, see Watson and Fleming, supra).

The RT-PCR method can be performed as follows. Total cellular RNA is isolated by, for example, the standard guanidium isothiocyanate method and the total RNA is reverse transcribed. The reverse transcription method involves synthesis of DNA on a template of RNA using a reverse transcriptase enzyme and a 3' end oligo dT primer and/or random hexamer primers. The cDNA thus produced is then amplified by means of PCR. (Belyavsky et al, Nucl Acid Res 17: 2919-2932, 1989; Krug and Berger, Methods in Enzymology, Academic Press, N.Y., Vol.152, pp. 316-325, 1987 which are incorporated by reference). Further preferred is the "Real-time" variant of RT- PCR, wherein the PCR product is detected by means of hybridisation probes (E.g TaqMan, Lightcycler, Molecular Beacons & Scorpion) or SYBR green. The detected signal from the probes or SYBR green is then quantitated either by reference to a standard curve or by comparing the Ct values to that of a calibration standard. Analysis of housekeeping genes is often used to normalize the results.

In Northern blot analysis total or poly(A)+ mRNA is run on a denaturing agarose gel and detected by hybridization to a labelled probe in the dried gel itself or on a membrane. The resulting signal is proportional to the amount of target RNA in the RNA population. Comparing the signals from two or more cell populations or tissues reveals relative differences in gene expression levels. Absolute quantitation can be performed by comparing the signal to a standard curve generated using known amounts of an in vitro transcript corresponding to the target RNA. Analysis of housekeeping genes, genes whose expression levels are expected to remain relatively constant regardless of conditions, is often used to normalize the results, eliminating any apparent differences caused by unequal transfer of RNA to the membrane or unequal loading of RNA on the gel.
The first step in Northern analysis is isolating pure, intact RNA from the cells or tissue of interest. Because Northern blots distinguish RNAs by size, sample integrity influences the degree to which a signal is localized in a single band. Partially degraded RNA samples will result in the signal being smeared or distributed over several bands with an overall loss in sensitivity and possibly an erroneous interpretation of the data. In Northern blot analysis, DNA, RNA and oligonucleotide probes can be used and these probes are preferably labelled (e.g. radioactive labels, massa labels or fluorescent labels). The size of the target RNA, not the probe, will determine the size of the detected band, so methods such as random-primed labeling, which generates probes of variable lengths, are suitable for probe synthesis. The specific activity of the probe will determine the level of sensitivity, so it is preferred that probes with high specific activities, are used..
In an RNase protection assay, the RNA target and an RNA probe of a defined length are hybridized in solution. Following hybridization, the RNA is digested with RNases specific for single-stranded nucleic acids to remove any unhybridized, single-stranded target RNA and probe. The RNases are inactivated, and the RNA is separated e.g. by denaturing polyacrylamide gel electrophoresis. The amount of intact RNA probe is proportional to the amount of target RNA in the RNA population. RPA can be used for relative and absolute quantitation of gene expression and also for mapping RNA structure, such as intron/exon boundaries and transcription start sites. The RNase protection assay is preferable to Northern blot analysis as it generally has a lower limit of detection.

The antisense RNA probes used in RPA are generated by in vitro transcription of a DNA template with a defined endpoint and are typically in the range of 50-600 nucleotides. The use of RNA probes that include additional sequences not homologous to the target RNA allows the protected fragment to be distinguished from the full-length probe. RNA probes are typically used instead of DNA probes due to the ease of generating single-stranded RNA probes and the reproducibility and reliability of RNA:RNA duplex digestion with RNases (Ausubel *et al*. 2003), particularly preferred are probes with high specific activities.

Particularly preferred is the use of microarrays. The microarray analysis process can be divided into two main parts. First is the immobilization of known gene sequences onto glass slides or other solid support followed by hybridization of the fluorescently labelled cDNA (comprising the sequences to be interrogated) to the known genes immobilized on the glass slide. After hybridization, arrays are scanned using a fluorescent microarray scanner. Analyzing the relative fluorescent intensity of different genes provides a measure of the differences in gene expression. DNA arrays can be generated by immobilizing presynthesized oligonucleotides onto prepared glass slides. In this case, representative gene sequences are manufactured and prepared using standard oligonucleotide synthesis and purification methods. These synthesized gene sequences are complementary to the genes of interest (such as PITX2 or other genes according to Table 11) and tend to be shorter sequences in the range of 25-70 nucleotides. Alternatively, immobilized oligos can be chemically synthesized in situ on the surface of the slide. In situ oligonucleotide synthesis involves the consecutive addition of the appropriate nucleotides to the spots on the microarray; spots not receiving a nucleotide are protected during each stage of the process using physical or virtual masks. In expression profiling microarray experiments, the RNA templates used are representative of the transcription profile of the cells or tissues under study. RNA is first isolated from the cell populations or tissues to be compared. Each RNA sample is then used as a template to generate fluorescently labelled cDNA via a reverse transcription reaction. Fluorescent labeling of the cDNA can be accomplished by either direct labeling or indirect labeling methods. During direct labeling, fluorescently modified nucleotides (e.g., Cy®3- or Cy®5-dCTP) are incorporated directly into the cDNA during the reverse transcription. Alternatively, indirect labeling can be achieved by incorporating aminoallyl-modified nucleotides during cDNA synthesis and then conjugating an N-hydroxysuccinimide (NHS)-ester dye to the aminoallyl-modified cDNA after the reverse transcription reaction is complete. Alternatively, the probe may be unlabelled, but may be detectable by specific binding with a ligand which is labelled, either directly or indirectly. Suitable labels and methods for labelling ligands (and probes) are known in the art, and include, for example, radioactive labels which may be incorporated by known methods (e.g., nick translation or kinasing). Other suitable labels include but are not limited to biotin, fluorescent groups, chemiluminescent groups (e.g., dioxetanes, particularly triggered dioxetanes), enzymes, antibodies, and the like.
To perform differential gene expression analysis, cDNA generated from different RNA samples are labelled with Cy®3. The resulting labelled cDNA is purified to remove unincorporated nucleotides, free dye and residual RNA. Following purification, the labeled cDNA samples are hybridised to the microarray. The stringency of hybridisation is determined by a number of factors during hybridisation and during the washing procedure, including temperature, ionic strength, length of time and concentration of formamide. These factors are outlined in, for example, Sambrook et al. (Molecular Cloning: A Laboratory Manual, 2nd ed., 1989). The microarray is scanned post-hybridization using a fluorescent microarray scanner.
The fluorescent intensity of each spot indicates the level of expression for that gene; bright spots correspond to strongly expressed genes, while dim spots indicate weak expression. Once the images are obtained, the raw data must be analyzed. First, the background fluorescence must be subtracted from the fluorescence of each spot. The data is then normalized to a control sequence, such as an exogenously added RNA, or a housekeeping gene panel to account for any nonspecific hybridization, array imperfections or variability in the array setup, cDNA labeling, hybridization or washing. Data normalization allows the results of multiple arrays to be compared.
The present invention further provides for methods for the detection of the presence of the polypeptide encoded by said gene sequences in a sample obtained from a patient.
Aberrant levels of polypeptide expression of the polypeptides encoded by the gene according to Table 11 (in particular FOXL2, HIST2H2BF and GPR7 and most preferably PITX2) are associated with prostate cancer prognosis and/or treatment outcome.

Any method known in the art for detecting polypeptides can be used. Such methods include, but are not limited to masss-spectrometry, immunodiffusion, immunoelectrophoresis, immunochemical methods, binder-ligand assays, immunohistochemical techniques, agglutination and complement assays (e.g., see Basic and Clinical Immunology, Sites and Terr, eds., Appleton & Lange, Norwalk, Conn. pp 217-262, 1991 which is incorporated by reference). Preferred are binder-ligand immunoassay methods including reacting antibodies with an epitope or epitopes and competitively displacing a labelled polypeptide or derivative thereof.

Certain embodiments of the present invention comprise the use of antibodies specific to the polypeptide encoded by the a gene selected from Table 11, preferably FOXL2, HIST2H2BF or GPR7 and most preferably PITX2.

Such antibodies are useful for prostate cancer prognostic and/or predictive applications. In certain embodiments production of monoclonal or polyclonal antibodies can be induced by the use of the coded polypeptide as an antigene. Such antibodies may in turn be used to detect expressed polypeptides as markers for prostate cancer prognosis. The levels of such polypeptides present may be quantified by conventional methods. Antibody-polypeptide binding may be detected and quantified by a variety of means known in the art, such as labelling with fluorescent or radioactive ligands. The invention further comprises kits for performing the above-mentioned procedures, wherein such kits contain antibodies specific for the investigated polypeptides.

Numerous competitive and non-competitive polypeptide binding immunoassays are well known in the art. Antibodies employed in such assays may be unlabelled, for example as used in agglutination tests, or labelled for use a wide variety of assay methods. Labels that can be used include radionuclides, enzymes, fluorescers, chemiluminescers, enzyme substrates or co-factors, enzyme inhibitors, particles, dyes and the like. Preferred assays include but are not limited to radioimmunoassay (RIA), enzyme immunoassays, e.g., enzyme-linked immunosorbent assay (ELISA), fluorescent immunoassays and the like. Polyclonal or monoclonal antibodies or epitopes thereof can be made for use in immunoassays by any of a number of methods known in the art.

In an alternative embodiment of the method the proteins may be detected by means of western blot analysis. Said analysis is standar in the art, briefly proteins are separated by means of electrophoresis e.g. SDS-PAGE. The separated proteins are then transferred to a suitable membrane (or paper) e.g. nitrocellulose, retaining the spacial separation achieved by electrophoresis. The membrane is then incubated with a generic protein (e.g. milk protein) to bind remaining sticky places on the membrane. An antibody specific to the protein of interest is then added, said antibody being detectably labelled for example by dyes or enzymatic means (e.g. alkaline phosphatase or horseradish peroxidase). The location of the antibody on the membrane is then detected.

In an alternative embodiment of the method the proteins may be detected by means of immunohistochemistry (the use of antibodies to probe specific antigens in a sample). Said analysis is standard in the art, wherein detection of antigens in tissues is known as immunohistochemistry, while detection in cultured cells is generally termed immunocytochemistry. Briefly the primary antibody to be detected by binding to its specific antigen. The antibody-antigen complex is then bound by a secondary enzyme conjugated antibody. In the presence of the necessary substrate and chromogen the bound enzyme is detected according to coloured deposits at the antibody-antigen binding sites. There is a wide range of suitable sample types, antigen-antibody affinity, antibody types, and detection enhancement methods. Thus optimal conditions for immunohistochemical or immunocytochemical detection must be determined by the person skilled in the art for each individual case.

One approach for preparing antibodies to a polypeptide is the selection and preparation of an amino acid sequence of all or part of the polypeptide, chemically synthesising the amino acid sequence and injecting it into an appropriate animal, usually a rabbit or a mouse (Milstein and Kohler Nature 256:495-497, 1975; Gulfre and Milstein, Methods in Enzymology: Immunochemical Techniques 73:1-46, Langone and Banatis eds., Academic Press, 1981 which are incorporated by reference). Methods for preparation of the polypeptides or epitopes thereof include, but are not limited to chemical synthesis, recombinant DNA techniques or isolation from biological samples.

In a particularly preferred embodiment the expression level of the genes, genomic sequences and/or regulatory regions according to Table 11 is determined by analysis of the level of methylation of said genes, genomic sequences and/or regulatory regions thereof. It is preferred that the level of methylation of said genes, genomic sequences and/or regulatory regions thereof is determined by determing the methylaiton status or level of at least one CpG dinucleotide thereof. It is further preferred that the level of methylation of said genes, genomic sequences and/or regulatory regions thereof is determined by determing the methylaiton status or level of a plurality of CpG dinucleotides thereof. It is particularly preferred that said genes, genomic sequences and/or regulatory regions are selected from the group consisting PITX2, SEQ ID NO: 63, GPR7 and SEQ ID NO: 35. Further preferred is the gene PITX2. Said analysis comprises the following steps:

i) contacting genomic DNA obtained from the subject with at least one reagent, or series of reagents that distinguishes between methylated and non-methylated CpG dinucleotides within at least one target region of the genomic DNA, wherein said contiguous nucleotides comprise at least one CpG dinucleotide sequence; and
ii) classifying the prostate cell proliferative disorders according to its prognosis as determined from the methylation status of said target regions analysed in i).

Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants e.g. by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA. Preferably, the source of the DNA sample is selected from the group consisting of cells or cell lines, histological slides, biopsies, paraffin-embedded tissue, bodily fluids, ejaculate, urine, blood, and combinations thereof. Preferably, the source is biopsies, bodily fluids, ejaculate, urine, or blood. The genomic DNA sample is then treated in such a manner that cytosine bases which are unmethylated at the 5'-position are converted to uracil, thymine, or another base which is dissimilar to cytosine in terms of hybridization behavior. This will be understood as 'treatment' herein.

The above described treatment of genomic DNA is preferably carried out with bisulfite (hydrogen sulfite, disulfite) and subsequent alkaline hydrolysis which results in a conversion of non-methylated cytosine nucleobases to uracil or to another base which is dissimilar to cytosine in terms of base pairing behavior.

The treated DNA is then analysed in order to determine the methylation state of one or more target gene sequences (prior to the treatment) associated with the development of prostate carcinoma. It is particularly preferred that the target region comprises, or hybridizes under stringent conditions to at least 16 contiguous nucleotides of at least one gene or genomic sequence selected from the group consisting the genes and genomic sequences as listed in Table 11. It is particularly preferred that said gene or genomic sequence is selected from the group consisting PITX2, SEQ ID NO: 63, GPR7 and SEQ ID NO: 35. Further preferred is the gene PITX2. It is further preferred that the sequences of said genes as described in the accompanying sequence listing are analysed. The method of analysis may be selected from those known in the art, including those listed herein. Particularly preferred are MethyLight™, MSP™ and the use of blocking oligonucleotides as will be described herein. It is further preferred that any oligonucleotides used in such analysis (including primers, blocking oligonucleotides and detection probes) should be reverse complementary, identical, or hybridize under stringent or highly stringent conditions to an at least 16-base-pair long segment of the base sequences of one or more of SEQ ID NO:65 to SEQ ID NO:320 and SEQ ID NO: 962 to SEQ ID NO: 965 and sequences complementary thereto. It is preferred that any oligonucleotides used in such analysis (including primers, blocking oligonucleotides and detection probes) should be reverse complementary, identical, or hybridize under stringent or highly stringent conditions to an at least 16-base-pair long segment of the base sequences of one or more of SEQ ID Nos: 133,134,261,262, 189,190,317,318, 101,102,229,230 and most preferably SEQ ID Nos: 962 - 965 and sequences complementary thereto

Aberrant methylation, of one or more genes or genomic sequences taken from those listed in Table 11, and more preferably the genes or genomic sequences according to PITX2, SEQ ID NO: 63, GPR7 and SEQ ID NO: 35 are associated with the prognosis of prostate cell proliferative disorders. Analysis of one or a plurality of the sequences enables the prognostic classification of prostate cell proliferative disorders. More preferably hypermethylation of one or more of said genes or genomic sequences is associated with poor prognosis. Hypermethylation is in general associated with under expression of mRNA and accordingly polpeptides.

In one embodiment the method discloses the use of one or more genes or genomic sequences selected from the group consisting the genes according to Table 11 as markers for providing a prognosis of prostate cell proliferative disorders. It is particularly preferred that said gene or genomic sequence is selected from the group consisting PITX2, SEQ ID NO: 63, GPR7 and SEQ ID NO: 35. Further preferred is the gene PITX2.

Said use of the genes and/or sequences may be enabled by means of any analysis of the expression of the gene, by means of mRNA expression analysis or protein expression analysis. However, in the most preferred embodiment of the invention, the detection of prostate cell proliferative disorders is enabled by means of analysis of the methylation status of said genes or genomic sequences and their promoter or regulatory elements. Methods for the methylation analysis of genes are described herein.

In one embodiment the method discloses the use of one or more genes or genomic sequences selected from the group consisting of the genes according to Table 11 as markers for providing a prognosis of prostate cell proliferative disorders. It is particularly preferred that said gene or genomic sequence is selected from the group consisting PITX2, SEQ ID NO: 63, GPR7 and SEQ ID NO: 35. Further preferred is the gene PITX2.

Said use of the genes and/or sequences may be enabled by means of any analysis of the expression of the gene, by means of mRNA expression analysis or protein expression analysis. However, in the most preferred embodiment of the invention, the detection of prostate cell proliferative disorders is enabled by means of analysis of the methylation status of said genes or genomic sequences and their promoter or regulatory elements. Methods for the methylation analysis of genes are described herein.

Aberrant levels of mRNA expression of the genes, genomic sequences or genes regulated by genomic sequences according to Table 11 are associated with prognosis of prostate cell proliferative disorders. Accordingly, increased or decreased levels of expression of said genes or sequences are associable with factors associated with the prognosis of prostate cell proliferative disorders, including but not limited to disease agressivity and progression. It is particularly preferred that said gene or genomic sequence is selected from the group consisting PITX2, SEQ ID NO: 63, GPR7 and SEQ ID NO: 35. Further preferred is the gene PITX2.

To detect the presence of mRNA encoding a gene or genomic sequence in a prognostic classification of prostate cell proliferative disorders, a sample is obtained from a patient. Preferably, the source of the sample is selected from the group consisting of cells or cell lines, histological slides, biopsies, paraffin-embedded tissue, bodily fluids, ejaculate, urine, blood, and combinations thereof. Preferably, the source is biopsies, bodily fluids, ejaculate, urine, or blood. The sample may be treated to extract the nucleic acids contained therein. The resulting nucleic acid from the sample is subjected to gel electrophoresis or other separation techniques. Detection involves contacting the nucleic acids and in particular the mRNA of the sample with a DNA sequence serving as a probe to form hybrid duplexes. The stringency of hybridisation is determined by a number of factors during hybridisation and during the washing procedure, including temperature, ionic strength, length of time and concentration of formamide. These factors are outlined in, for example, Sambrook et al. (Molecular Cloning: A Laboratory Manual, 2d ed., 1989). Detection of the resulting duplex is usually accomplished by the use of labelled probes. Alternatively, the probe may be unlabeled, but may be detectable by specific binding with a ligand which is labelled, either directly or indirectly. Suitable labels and methods for labelling probes and ligands are known in the art, and include, for example, radioactive labels which may be incorporated by known methods (e.g., nick translation or kinasing), biotin, fluorescent groups, chemiluminescent groups (e.g., dioxetanes, particularly triggered dioxetanes), enzymes, antibodies, and the like.

To increase the sensitivity of the detection in a sample of mRNA transcribed from the gene or genomic sequence, the technique of reverse transcription/polymerisation chain reaction can be used to amplify cDNA transcribed from the mRNA. The method of reverse transcription /PCR is well known in the art (for example, see Watson and Fleming, supra).

The reverse transcription /PCR method can be performed as follows. Total cellular RNA is isolated by, for example, the standard guanidium isothiocyanate method and the total RNA is reverse transcribed. The reverse transcription method involves synthesis of DNA on a template of RNA using a reverse transcriptase enzyme and a 3' end primer. Typically, the primer contains an oligo(dT) sequence. The cDNA thus produced is then amplified using the PCR method and EYA4 specific primers. (Belyavsky et al, Nucl Acid Res 17:2919-2932, 1989; Krug and Berger, Methods in Enzymology, Academic Press,N.Y., Vol.152, pp. 316-325, 1987 which are incorporated by reference).

The present invention may also be described in certain embodiments as a *kit* for use in providing a prognosis of a prostate cell proliferative disorder state through testing of a biological sample. A representative kit may comprise one or more nucleic acid segments that selectively hybridise to the mRNA and a container for each of the one or more nucleic acid segments. In certain embodiments the nucleic acid segments may be combined in a single tube. In further embodiments, the nucleic acid segments may also include a pair of primers for amplifying the target mRNA. Such kits may also include any buffers, solutions, solvents, enzymes, nucleotides, or other components for hybridisation, amplification or detection reactions. Preferred kit components include reagents for reverse transcription-PCR, in situ hybridisation, Northern analysis and/or RPA.

Particular embodiments of the present invention provide a novel application of the analysis of methylation levels and/or patterns within said sequences that enables a prognostic classification and thereby improved treatment of prostate cell proliferative disorders. Treatment of prostate cell proliferative disorders is directly linked with disease prognosis in particular aggressiveness, and the disclosed method thereby enables the physician and patient to make better and more informed treatment decisions.

FURTHER IMPROVEMENTS

The present invention provides novel uses for genomic sequences selected from the group consisting of SEQ ID NO:1 TO SEQ ID NO:64 AND SEQ ID NO: 961 (preferably SEQ ID Nos: 35, 63, 19 and most preferably SEQ ID NO: 961). Additional embodiments provide modified variants of SEQ ID NO:1 TO SEQ ID NO:64 AND SEQ ID NO: 961, as well as oligonucleotides and/or PNA-oligomers for analysis of cytosine methylation patterns within the group consisting SEQ ID NO:1 TO SEQ ID NO:64 AND SEQ ID NO: 961 (preferably SEQ ID Nos: 35, 63, 19 and most preferably SEQ ID NO: 961).

An objective of the invention comprises analysis of the methylation state of one or more CpG dinucleotides within at least one of the genomic sequences selected from the group consisting of SEQ ID NO:1 TO SEQ ID NO:64 AND SEQ ID NO: 961 and sequences complementary thereto. Preferably said group consists of SEQ ID Nos: 35, 63, 19 and most preferably said sequence is SEQ ID NO: 961.

The disclosed invention provides treated nucleic acids, derived from genomic SEQ ID NO:1 to SEQ ID NO:64 and SEQ ID NO: 961 (preferably SEQ ID Nos: 35, 63, 19 and most preferably SEQ ID NO: 961), wherein the treatment is suitable to convert at least one unmethylated cytosine base of the genomic DNA sequence to uracil or another base that is detectably dissimilar to cytosine in terms of hybridization. The genomic sequences in question may comprise one, or more, consecutive or random methylated CpG positions. Said treatment preferably comprises use of a reagent selected from the group consisting of bisulfite, hydrogen sulfite, disulfite, and combinations thereof. In a preferred embodiment of the invention, the objective comprises analysis of a non-naturally occurring modified nucleic acid comprising a sequence of at least 16 contiguous nucleotide bases in length of a sequence selected from the group consisting of SEQ ID NO: 65 TO SEQ ID NO: 320 AND SEQ ID Nos: 962 - 965 (preferably said group consists of SEQ ID Nos: 133,134,261,262, 189,190,317,318, 101,102,229,230 and most preferably said group consists of SEQ ID Nos: 962 - 965). Particularly preferred is a non-naturally occurring modified nucleic acid comprising a sequence of at least 16 contiguous nucleotide bases in length of a sequence selected from the group consisting of SEQ ID NO: 65 to SEQ ID NO: 320 and SEQ ID NO: 962 to SEQ ID NO: 965 that is not identical to or complementary to SEQ ID NO: 1 to SEQ ID NO: 64 and SEQ ID NO: 961 or other human genomic DNA. Further preferred is a non-naturally occurring modified nucleic acid comprising a sequence of at least 16 contiguous nucleotide bases in length of a sequence selected from the group consisting of SEQ ID Nos: 133,134,261,262, 189,190,317,318, 101,102,229,230, 962 - 965 that is not identical to or complementary to SEQ ID Nos: 961, 35, 63 and 19 or other human genomic DNA.

It is further preferred that said sequence comprises at least one CpG, TpA or CpA dinucleotide and sequences complementary thereto. The sequences of SEQ ID NO:65 TO SEQ ID NO:320 AND SEQ ID NO: 962 TO SEQ ID NO: 965 provide non-naturally occurring modified versions of the nucleic acid according to SEQ ID NO:1 TO SEQ ID NO: 64 AND SEQ ID NO: 961, wherein the modification of each genomic sequence results in the synthesis of a nucleic acid having a sequence that is unique and distinct from said genomic sequence as follows. For each sense strand genomic DNA, e.g., SEQ ID NO:1, four converted versions are disclosed. A first version wherein "C" is converted to "T," but "CpG" remains "CpG" (*i.e.*, corresponds to case where, for the genomic sequence, all "C" residues of CpG dinucleotide sequences are methylated and are thus not converted); a second version discloses the complement of the disclosed genomic DNA sequence (*i.e.* antisense strand), wherein "C" is converted to "T," but "CpG" remains "CpG" (*i.e.*, corresponds to case where, for all "C" residues of CpG dinucleotide sequences are methylated and are thus not converted). The 'upmethylated' converted sequences of SEQ ID NO:1 to SEQ ID NO:64 and SEQ ID NO: 961 correspond to SEQ ID NO:65 to SEQ ID NO:728. A third chemically converted version of each genomic sequences is provided, wherein "C" is converted to "T" for all "C" residues, including those of "CpG" dinucleotide sequences (*i.e.*, corresponds to case where, for the genomic sequences, all "C" residues of CpG dinucleotide sequences are *un*methylated); a final chemically converted version of each sequence, discloses the complement of the disclosed genomic DNA sequence (*i.e.* antisense strand), wherein "C" is converted to "T" for all "C" residues, including those of "CpG" dinucleotide sequences (*i.e.*, corresponds to case where, for the complement (antisense strand) of each genomic sequence, all "C" residues of CpG dinucleotide sequences are *un*methylated). The 'downmethylated' converted sequences of SEQ ID NO:1 to SEQ ID NO:64 and SEQ ID NO: 961 correspond to SEQ ID NO:65 to SEQ ID NO:320 and SEQ ID NO: 962 to SEQ ID NO: 965.

In an alternative preferred embodiment, such analysis comprises the use of an oligonucleotide or oligomer for detecting the cytosine methylation state within genomic or treated (chemically modified) DNA, according to SEQ ID NO: 1 to SEQ ID NO:320 and SEQ ID NO: 961 to SEQ ID NO: 965. Said oligonucleotide or oligomer comprising a nucleic acid sequence having a length of at least nine (9) nucleotides which hybridizes, under moderately stringent or stringent conditions (as defined herein above), to a treated nucleic acid sequence according to SEQ ID NO:1 to SEQ ID NO:320 and SEQ ID NO: 961 to SEQ ID NO: 965 and/or sequences complementary thereto, or to a genomic sequence according to SEQ ID NO:1 to SEQ ID NO:64 and SEQ ID NO: 961 and/or sequences complementary thereto.

Thus, the present invention includes nucleic acid molecules (e.g., oligonucleotides and peptide nucleic acid (PNA) molecules (PNA-oligomers)) that hybridize under moderately stringent and/or stringent hybridization conditions to all or a portion of the sequences SEQ ID NO: 1 to SEQ ID NO: 320 and SEQ ID NO: 961 to 965 , or to the complements thereof. Particularly preferred is a nucleic acid molecule that hybridizes under moderately stringent and/or stringent hybridization conditions to all or a portion of the sequences SEQ ID NO: 65 to SEQ ID NO: 320 and SEQ ID NO: 962 to SEQ ID NO: 965 but is not identical to or complementary to SEQ ID NO: 1 to SEQ ID NO: 64 and SEQ ID NO: 961 or other human genomic DNA. Further preferred is a nucleic acid molecule that hybridizes under moderately stringent and/or stringent hybridization conditions to all or a portion of the sequences SEQ ID Nos: 133,134,261,262, 189,190,317,318, 101,102,229,230, 962 - 965 but is not identical to or complementary to SEQ ID Nos: 961, 35, 63 and 19 or other human genomic DNA.

The hybridizing portion of the hybridizing nucleic acids is typically at least 9, 15, 20, 25, 30 or 35 nucleotides in length. However, longer molecules have inventive utility, and are thus within the scope of the present invention.

Preferably, the hybridizing portion of the inventive hybridizing nucleic acids is at least 95%, or at least 98%, or 100% identical to the sequence, or to a portion thereof of SEQ ID NO: 1 to SEQ ID NO: 320 and SEQ ID NO: 961 to 965, or to the complements thereof.

Hybridizing nucleic acids of the type described herein can be used, for example, as a primer (e.g., a PCR primer), or a diagnostic and/or prognostic probe or primer. Preferably, hybridization of the oligonucleotide probe to a nucleic acid sample is performed under stringent conditions and the probe is 100% identical to the target sequence. Nucleic acid duplex or hybrid stability is expressed as the melting temperature or Tm, which is the temperature at which a probe dissociates from a target DNA. This melting temperature is used to define the required stringency conditions.

For target sequences that are related and substantially identical to the corresponding sequence of SEQ ID NO:1 to SEQ ID NO:64 and SEQ ID NO: 961 (such as allelic variants and SNPs), rather than identical, it is useful to first establish the lowest temperature at which only homologous hybridization occurs with a particular concentration of salt (e.g., SSC or SSPE). Then, assuming that 1% mismatching results in a 1˚C decrease in the Tm, the temperature of the final wash in the hybridization reaction is reduced accordingly (for example, if sequences having > 95% identity with the probe are sought, the final wash temperature is decreased by 5˚C). In practice, the change in Tm can be between 0.5˚C and 1.5˚C per 1% mismatch.

Examples of inventive oligonucleotides of length X (in nucleotides), as indicated by polynucleotide positions with reference to, e.g., SEQ ID NO:1, include those corresponding to sets (sense and antisense sets) of consecutively overlapping oligonucleotides of length X, where the oligonucleotides within each consecutively overlapping set (corresponding to a given X value) are defined as the finite set of Z oligonucleotides from nucleotide positions:

$$n \text{ to } (n + (X-1));$$

where n=1, 2, 3,...(Y-(X-1));

where Y equals the length (nucleotides or base pairs) of SEQ ID NO: 1 (7572);

where X equals the common length (in nucleotides) of each oligonucleotide in the set (e.g., X=20 for a set of consecutively overlapping 20-mers); and

where the number (Z) of consecutively overlapping oligomers of length X for a given SEQ ID NO of length Y is equal to Y-(X-1). For example Z= 7572-19= 7553 for either sense or antisense sets of SEQ ID NO:1, where X=20.

Preferably, the set is limited to those oligomers that comprise at least one CpG, TpG or CpA dinucleotide.

Examples of inventive 20-mer oligonucleotides include the following set of oligomers (and the antisense set complementary thereto), indicated by polynucleotide positions with reference to SEQ ID NO:1:

1-20, 2-21, 3-22, 4-23, 5-24, .....7553 -7572.

Preferably, the set is limited to those oligomers that comprise at least one CpG, TpG or CpA dinucleotide.

Likewise, examples of inventive 25-mer oligonucleotides include the following set of 2,256 oligomers (and the antisense set complementary thereto), indicated by polynucleotide positions with reference to SEQ ID NO:1:

1-25, 2-26, 3-27, 4-28, 5-29, ...... 7553 -7572.

Preferably, the set is limited to those oligomers that comprise at least one CpG, TpG or CpA dinucleotide.

The present invention encompasses, for each of SEQ ID NO:1 to SEQ ID NO:320 and SEQ ID NO: 961 to SEQ ID NO: 965 (sense and antisense), multiple consecutively overlapping sets of oligonucleotides or modified oligonucleotides of length X, where, e.g., X= 9, 10, 17, 20, 22, 23, 25, 27, 30 or 35 nucleotides.

The oligonucleotides or oligomers according to the present invention constitute effective tools useful to ascertain genetic and epigenetic parameters of the genomic sequence corresponding to SEQ ID NO:1 to SEQ ID NO:64 and SEQ ID NO: 961 . Preferred sets of such oligonucleotides or modified oligonucleotides of length X are those consecutively overlapping sets of oligomers corresponding to SEQ ID NO:1 to SEQ ID NO:320 and SEQ ID NO: 961 to SEQ ID NO: 965 (and to the complements thereof). Preferably, said oligomers comprise at least one CpG, TpG or CpA dinucleotide.

Particularly preferred oligonucleotides or oligomers according to the present invention are those in which the cytosine of the CpG dinucleotide (or of the corresponding converted TpG or CpA dinculeotide) sequences is within the middle third of the oligonucleotide; that is, where the oligonucleotide is, for example, 13 bases in length, the CpG, TpG or CpA dinucleotide is positioned within the fifth to ninth nucleotide from the 5'-end.

The oligonucleotides of the invention can also be modified by chemically linking the oligonucleotide to one or more moieties or conjugates to enhance the activity, stability or detection of the oligonucleotide. Such moieties or conjugates include chromophores, fluorophors, lipids such as cholesterol, cholic acid, thioether, aliphatic chains, phospholipids, polyamines, polyethylene glycol (PEG), palmityl moieties, and others as disclosed in, for example, United States Patent Numbers 5,514,758, 5,565,552, 5,567,810, 5,574,142, 5,585,481, 5,587,371, 5,597,696 and 5,958,773. The probes may also exist in the form of a PNA (peptide nucleic acid) which has particularly preferred pairing properties. Thus, the oligonucleotide may include other appended groups such as peptides, and may include hybridization-triggered cleavage agents (Krol et al., BioTechniques 6:958-976, 1988) or intercalating agents (Zon, Pharm. Res. 5:539-549, 1988). To this end, the oligonucleotide may be conjugated to another molecule, e.g., a chromophore, fluorophor, peptide, hybridization-triggered cross-linking agent, transport agent, hybridization-triggered cleavage agent, etc.

The oligonucleotide may also comprise at least one art-recognized modified sugar and/or base moiety, or may comprise a modified backbone or non-natural internucleoside linkage.

The oligonucleotides or oligomers according to particular embodiments of the present invention are typically used in 'sets,' which contain at least one oligomer for analysis of at least one of the CpG dinucleotides of genomic sequences SEQ ID NO:1 to SEQ ID NO:64 and SEQ ID NO: 961 and sequences complementary thereto, or to the corresponding CpG, TpG or CpA dinucleotide within a sequence of the treated nucleic acids according to SEQ ID NO: 65 to SEQ ID NO:320 and SEQ ID NO: 962 to SEQ ID NO: 965 and sequences complementary thereto. However, it is anticipated that for economic or other factors it may be preferable to analyze a limited selection of the CpG dinucleotides within said sequences, and the content of the set of oligonucleotides is altered accordingly.

Therefore, in particular embodiments, the present invention provides a set of at least two (2) (oligonucleotides and/or PNA-oligomers) useful for detecting the cytosine methylation state in treated genomic DNA (SEQ ID NO: 65 to SEQ ID NO:320 and SEQ ID NO: 962 to SEQ ID NO: 965), or in genomic DNA (SEQ ID NO:1 to SEQ ID NO:64 and SEQ ID NO: 961 and sequences complementary thereto). These probes enable diagnosis and/or classification of genetic and epigenetic parameters of prostate cell proliferative disorders. The set of oligomers may also be used for detecting single nucleotide polymorphisms (SNPs) in treated genomic DNA (SEQ ID NO:65 to SEQ ID NO:320 and SEQ ID NO: 962 to SEQ ID NO: 965), or in genomic DNA (SEQ ID NO:1 to SEQ ID NO:64 and SEQ ID NO: 961 and sequences complementary thereto).

In preferred embodiments, at least one, and more preferably all members of a set of oligonucleotides is bound to a solid phase.

In further embodiments, the present invention provides a set of at least two (2) oligonucleotides that are used as 'primer' oligonucleotides for amplifying DNA sequences of one of SEQ ID NO:1 to SEQ ID NO:320 and SEQ ID NO: 961 to SEQ ID NO: 965 and sequences complementary thereto, or segments thereof.

It is anticipated that the oligonucleotides may constitute all or part of an "array" or "DNA chip" *(i.e.,* an arrangement of different oligonucleotides and/or PNA-oligomers bound to a solid phase). Such an array of different oligonucleotide- and/or PNA-oligomer sequences can be characterized, for example, in that it is arranged on the solid phase in the form of a rectangular or hexagonal lattice. The solid-phase surface may be composed of silicon, glass, polystyrene, aluminum, steel, iron, copper, nickel, silver, or gold. Nitrocellulose as well as plastics such as nylon, which can exist in the form of pellets or also as resin matrices, may also be used. An overview of the Prior Art in oligomer array manufacturing can be gathered from a special edition of Nature Genetics (Nature Genetics Supplement, Volume 21, January 1999, and from the literature cited therein). Fluorescently labeled probes are often used for the scanning of immobilized DNA arrays. The simple attachment of Cy3 and Cy5 dyes to the 5'-OH of the specific probe are particularly suitable for fluorescence labels. The detection of the fluorescence of the hybridized probes may be carried out, for example, via a confocal microscope. Cy3 and Cy5 dyes, besides many others, are commercially available.

It is also anticipated that the oligonucleotides, or particular sequences thereof, may constitute all or part of an "virtual array" wherein the oligonucleotides, or particular sequences thereof, are used, for example, as 'specifiers' as part of, or in combination with a diverse population of unique labeled probes to analyze a complex mixture of analytes. Such a method, for example is described in US 2003/0013091 (United States serial number 09/898,743, published 16 January 2003). In such methods, enough labels are generated so that each nucleic acid in the complex mixture (*i.e.*, each analyte) can be uniquely bound by a unique label and thus detected (each label is directly counted, resulting in a digital read-out of each molecular species in the mixture).

It is particularly preferred that the oligomers according to the invention are utilised for at least one of: prognosis of; treatment of; monitoring of; and treatment and monitoring of prostate cell proliferative disorders. This is enabled by use of said sets for providing a prognosis of a biological sample isolated from a patient. Particularly preferrred are those sets of oligomer that comprise at least two oligonucleotides selected from one of the following sets of oligonucleotides.

In one embodiment of the method, this is achieved by analysis of the methylation status of at least one target sequence comprising, or hybridizing under stringent conditions to at least 16 contiguous nucleotides of a gene or sequence selected from the group consisting the genes and sequences according to Table 11 and complements thereof. It is particularly preferred that said gene or genomic sequence is selected from the group consisting PITX2, SEQ ID NO: 63, GPR7 and SEQ ID NO: 35 and their sequences as listed in the accompanying sequence listing. Further preferred is the gene PITX2.

The present invention further provides a method for ascertaining genetic and/or epigenetic parameters of the genomic sequences according to SEQ ID NO:1 to SEQ ID NO:64 and SEQ ID NO: 961 within a subject by analyzing cytosine methylation and single nucleotide polymorphisms. In a preferred embodiment the present invention further provides a method for ascertaining genetic and/or epigenetic parameters of the genomic sequences according to SEQ ID Nos: 35, 63, 19 and most preferably SEQ ID NO: 961 within a subject by analyzing cytosine methylation and single nucleotide polymorphisms. Said method comprising contacting a nucleic acid comprising one or more of SEQ ID NO:1 to SEQ ID NO:64 and SEQ ID NO: 961 (preferably one or more of SEQ ID Nos: 35, 63, 19 and most preferably SEQ ID NO: 961) in a biological sample obtained from said subject with at least one reagent or a series of reagents, wherein said reagent or series of reagents, distinguishes between methylated and non-methylated CpG dinucleotides within the target nucleic acid.

Preferably, said method comprises the following steps: In the *first* step, a sample of the tissue to be analysed is obtained. The source may be any suitable source. Preferably, the source of the DNA sample is selected from the group consisting of cells or cell lines, histological slides, biopsies, paraffin-embedded tissue, bodily fluids, ejaculate, urine, blood, and combinations thereof. Preferably, the source is biopsies, bodily fluids, ejaculate, urine, or blood.

The genomic DNA is then isolated from the sample. Genomic DNA may be isolated by any means standard in the art, including the use of commercially available kits. Briefly, wherein the DNA of interest is encapsulated in by a cellular membrane the biological sample must be disrupted and lysed by enzymatic, chemical or mechanical means. The DNA solution may then be cleared of proteins and other contaminants e.g. by digestion with proteinase K. The genomic DNA is then recovered from the solution. This may be carried out by means of a variety of methods including salting out, organic extraction or binding of the DNA to a solid phase support. The choice of method will be affected by several factors including time, expense and required quantity of DNA.

Once the nucleic acids have been extracted, the genomic double stranded DNA is used in the analysis.

In the second *step* of the method, the genomic DNA sample is treated in such a manner that cytosine bases which are unmethylated at the 5'-position are converted to uracil, thymine, or another base which is dissimilar to cytosine in terms of hybridization behavior. This will be understood as 'pretreatment' or 'treatment' herein.

The above-described treatment of genomic DNA is preferably carried out with bisulfite (hydrogen sulfite, disulfite) and subsequent alkaline hydrolysis which results in a conversion of non-methylated cytosine nucleobases to uracil or to another base which is dissimilar to cytosine in terms of base pairing behavior.

In the *third step* of the method, fragments of the treated DNA are amplified, using sets of primer oligonucleotides according to the present invention, and an amplification enzyme. The amplification of several DNA segments can be carried out simultaneously in one and the same reaction vessel. Typically, the amplification is carried out using a polymerase chain reaction (PCR). The set of primer oligonucleotides includes at least two oligonucleotides whose sequences are each reverse complementary, identical, or hybridize under stringent or highly stringent conditions to an at least 16-base-pair long segment of the base sequences of one of SEQ ID NO:65 to SEQ ID NO:320 and SEQ ID NO: 962 to SEQ ID NO: 965 (preferably one of SEQ ID Nos: 133,134,261,262, 189,190,317,318, 101,102,229,230 and most preferably one of SEQ ID Nos: 962 - 965) and sequences complementary thereto.

In an alternate embodiment of the method, the methylation status of preselected CpG positions within the nucleic acid sequences comprising one or more of SEQ ID NO:1 to SEQ ID NO:64 and SEQ ID NO: 961 (preferably one or more of SEQ ID Nos: 35, 63, 19 and most preferably SEQ ID NO: 961) may be detected by use of methylation-specific primer oligonucleotides. This technique (MSP) has been described in United States Patent No. 6,265,171 to Herman. The use of methylation status specific primers for the amplification of bisulfite treated DNA allows the differentiation between methylated and unmethylated nucleic acids. MSP primers pairs contain at least one primer which hybridizes to a bisulfite treated CpG dinucleotide. Therefore, the sequence of said primers comprises at least one CpG dinucleotide. MSP primers specific for non-methylated DNA contain a "T' at the position of the C position in the CpG. Preferably, therefore, the base sequence of said primers is required to comprise a sequence having a length of at least 9 nucleotides which hybridizes to a treated nucleic acid sequence according to one of SEQ ID NO: 65 to SEQ ID NO: 320 and SEQ ID NO: 961 to 965 (preferably SEQ ID Nos: 133,134,261,262, 189,190,317,318, 101,102,229,230 and most preferably SEQ ID Nos: 962 - 965) and sequences complementary thereto, wherein the base sequence of said oligomers comprises at least one CpG dinucleotide.

A further preferred embodiment of the method comprises the use of blocker oligonucleotides. The use of such blocker oligonucleotides has been described by Yu et al., BioTechniques 23:714-720, 1997. Blocking probe oligonucleotides are hybridized to the bisulfite treated nucleic acid concurrently with the PCR primers. PCR amplification of the nucleic acid is terminated at the 5' position of the blocking probe, such that amplification of a nucleic acid is suppressed where the complementary sequence to the blocking probe is present. The probes may be designed to hybridize to the bisulfite treated nucleic acid in a methylation status specific manner. For example, for detection of methylated nucleic acids within a population of unmethylated nucleic acids, suppression of the amplification of nucleic acids which are unmethylated at the position in question would be carried out by the use of blocking probes comprising a 'CpA' or 'TpA' at the position in question, as opposed to a 'CpG' if the suppression of amplification of methylated nucleic acids is desired.

For PCR methods using blocker oligonucleotides, efficient disruption of polymerase-mediated amplification requires that blocker oligonucleotides not be elongated by the polymerase. Preferably, this is achieved through the use of blockers that are 3'-deoxyoligonucleotides, or oligonucleotides derivitized at the 3' position with other than a "free" hydroxyl group. For example, 3'-O-acetyl oligonucleotides are representative of a preferred class of blocker molecule.

Additionally, polymerase-mediated decomposition of the blocker oligonucleotides should be precluded. Preferably, such preclusion comprises either use of a polymerase lacking 5'-3' exonuclease activity, or use of modified blocker oligonucleotides having, for example, thioate bridges at the 5'-terminii thereof that render the blocker molecule nuclease-resistant. Particular applications may not require such 5' modifications of the blocker. For example, if the blocker- and primer-binding sites overlap, thereby precluding binding of the primer (e.g., with excess blocker), degradation of the blocker oligonucleotide will be substantially precluded. This is because the polymerase will not extend the primer toward, and through (in the 5'-3' direction) the blocker-a process that normally results in degradation of the hybridized blocker oligonucleotide.

A particularly preferred blocker/PCR embodiment, for purposes of the present invention and as implemented herein, comprises the use of peptide nucleic acid (PNA) oligomers as blocking oligonucleotides. Such PNA blocker oligomers are ideally suited, because they are neither decomposed nor extended by the polymerase.

Preferably, therefore, the base sequence of said *blocking oligonucleotides* is required to comprise a sequence having a length of at least 9 nucleotides which hybridizes to a treated nucleic acid sequence according to one of SEQ ID NO:65 to SEQ ID NO:320 and SEQ ID NO: 962 to SEQ ID NO: 965 and sequences complementary thereto, wherein the base sequence of said oligonucleotides comprises at least one CpG, TpG or CpA dinucleotide. More preferably the base sequence of said *blocking oligonucleotides* is required to comprise a sequence having a length of at least 9 nucleotides which hybridizes to a treated nucleic acid sequence according to one of preferably SEQ ID Nos: 133,134,261,262, 189,190,317,318, 101,102,229,230 and most preferably SEQ ID Nos: 962 - 965 and sequences complementary thereto, wherein the base sequence of said oligonucleotides comprises at least one CpG, TpG or CpA dinucleotide.

The fragments obtained by means of the amplification can carry a directly or indirectly detectable label. Preferred are labels in the form of fluorescence labels, radionuclides, or detachable molecule fragments having a typical mass which can be detected in a mass spectrometer. Where said labels are mass labels, it is preferred that the labeled amplificates have a single positive or negative net charge, allowing for better detectability in the mass spectrometer. The detection may be carried out and visualized by means of, e.g., matrix assisted laser desorption/ionization mass spectrometry (MALDI) or using electron spray mass spectrometry (ESI).

Matrix Assisted Laser Desorption/Ionization Mass Spectrometry (MALDI-TOF) is a very efficient development for the analysis of biomolecules (Karas & Hillenkamp, Anal Chem., 60:2299-301, 1988). An analyte is embedded in a light-absorbing matrix. The matrix is evaporated by a short laser pulse thus transporting the analyte molecule into the vapour phase in an unfragmented manner. The analyte is ionized by collisions with matrix molecules. An applied voltage accelerates the ions into a field-free flight tube. Due to their different masses, the ions are accelerated at different rates. Smaller ions reach the detector sooner than bigger ones. MALDI-TOF spectrometry is well suited to the analysis of peptides and proteins. The analysis of nucleic acids is somewhat more difficult (Gut & Beck, Current Innovations and Future Trends, 1:147-57, 1995). The sensitivity with respect to nucleic acid analysis is approximately 100-times less than for peptides, and decreases disproportionately with increasing fragment size. Moreover, for nucleic acids having a multiply negatively charged backbone, the ionization process via the matrix is considerably less efficient. In MALDI-TOF spectrometry, the selection of the matrix plays an eminently important role. For desorption of peptides, several very efficient matrixes have been found which produce a very fine crystallisation. There are now several responsive matrixes for DNA, however, the difference in sensitivity between peptides and nucleic acids has not been reduced. This difference in sensitivity can be reduced, however, by chemically modifying the DNA in such a manner that it becomes more similar to a peptide. For example, phosphorothioate nucleic acids, in which the usual phosphates of the backbone are substituted with thiophosphates, can be converted into a charge-neutral DNA using simple alkylation chemistry (Gut & Beck, Nucleic Acids Res. 23: 1367-73, 1995). The coupling of a charge tag to this modified DNA results in an increase in MALDI-TOF sensitivity to the same level as that found for peptides. A further advantage of charge tagging is the increased stability of the analysis against impurities, which makes the detection of unmodified substrates considerably more difficult.

In the *fourth step* of the method, the amplificates obtained during the third step of the method are analysed in order to ascertain the methylation status of the CpG dinucleotides prior to the treatment.

In embodiments where the amplificates were obtained by means of MSP amplification, the presence or absence of an amplificate is in itself indicative of the methylation state of the CpG positions covered by the primer, according to the base sequences of said primer.

Amplificates obtained by means of both standard and methylation specific PCR may be further analyzed by means of hybridization-based methods such as, but not limited to, array technology and probe based technologies as well as by means of techniques such as sequencing and template directed extension.

In one embodiment of the method, the amplificates synthesised in *step* three are subsequently hybridized to an array or a set of oligonucleotides and/or PNA probes. In this context, the hybridization takes place in the following manner: the set of probes used during the hybridization is preferably composed of at least 2 oligonucleotides or PNA-oligomers; in the process, the amplificates serve as probes which hybridize to oligonucleotides previously bonded to a solid phase; the non-hybridized fragments are subsequently removed; said oligonucleotides contain at least one base sequence having a length of at least 9 nucleotides which is reverse complementary or identical to a segment of the base sequences specified in the present Sequence Listing; and the segment comprises at least one CpG , TpG or CpA dinucleotide.

In a preferred embodiment, said dinucleotide is present in the central third of the oligomer. For example, wherein the oligomer comprises one CpG dinucleotide, said dinucleotide is preferably the fifth to ninth nucleotide from the 5'-end of a 13-mer. One oligonucleotide exists for the analysis of each CpG dinucleotide within the sequence according to SEQ ID NO:1 to SEQ ID NO:64 and SEQ ID NO: 961, and the equivalent positions within SEQ ID NO:65 to SEQ ID NO:320 and SEQ ID NO: 962 to SEQ ID NO: 965. Said oligonucleotides may also be present in the form of peptide nucleic acids. The non-hybridized amplificates are then removed.The hybridized amplificates are then detected. In this context, it is preferred that labels attached to the amplificates are identifiable at each position of the solid phase at which an oligonucleotide sequence is located.

In yet a further embodiment of the method, the genomic methylation status of the CpG positions may be ascertained by means of oligonucleotide probes that are hybridised to the bisulfite treated DNA concurrently with the PCR amplification primers (wherein said primers may either be methylation specific or standard).

A particularly preferred embodiment of this method is the use of fluorescence-based Real Time Quantitative PCR (Heid et al., Genome Res. 6:986-994, 1996; also see United States Patent No. 6,331,393) employing a dual-labeled fluorescent oligonucleotide probe (TaqMan™ PCR, using an ABI Prism 7700 Sequence Detection System, Perkin Elmer Applied Biosystems, Foster City, California). The TaqMan™ PCR reaction employs the use of a nonextendible interrogating oligonucleotide, called a TaqMan™ probe, which, in preferred embodiments, is designed to hybridize to a GpC-rich sequence located between the forward and reverse amplification primers. The TaqMan™ probe further comprises a fluorescent "reporter moiety" and a "quencher moiety" covalently bound to linker moieties (e.g., phosphoramidites) attached to the nucleotides of the TaqMan™ oligonucleotide. For analysis of methylation within nucleic acids subsequent to bisulfite treatment, it is required that the probe be methylation specific, as described in United States Patent No. 6,331,393, (hereby incorporated by reference in its entirety) also known as the MethyLight™ assay. Variations on the TaqMan™ detection methodology that are also suitable for use with the described invention include the use of dual-probe technology (Lightcycler™) or fluorescent amplification primers (Sunrise™ technology). Both these techniques may be adapted in a manner suitable for use with bisulfite treated DNA, and moreover for methylation analysis within CpG dinucleotides.

A further suitable method for the use of probe oligonucleotides for the assessment of methylation by analysis of bisulfite treated nucleic acids In a further preferred embodiment of the method, the *fifth step* of the method comprises the use of template-directed oligonucleotide extension, such as MS-SNuPE as described by Gonzalgo & Jones, Nucleic Acids Res. 25:2529-2531, 1997.

In yet a further embodiment of the method, the *fourth step* of the method comprises sequencing and subsequent sequence analysis of the amplificate generated in the *third step* of the method (Sanger F., et al., Proc Natl Acad Sci USA 74:5463-5467, 1977).

Best mode

In the most preferred embodiment of the method the genomic nucleic acids are isolated and treated according to the first three steps of the method outlined above, namely:

a) obtaining, from a subject, a biological sample having subject genomic DNA;
b) extracting or otherwise isolating the genomic DNA;
c) treating the genomic DNA of b), or a fragment thereof, with one or more reagents to convert cytosine bases that are unmethylated in the 5-position thereof to uracil or to another base that is detectably dissimilar to cytosine in terms of hybridization properties; and wherein
d) amplifying subsequent to treatment in c) is carried out in a methylation specific manner, namely by use of methylation specific primers or *blocking oligonucleotides*, and further wherein
e) detecting of the amplificates is carried out by means of a real-time detection probe, as described above.

Preferably, where the subsequent amplification of d) is carried out by means of methylation specific primers, as described above, said methylation specific primers comprise a sequence having a length of at least 9 nucleotides which hybridizes to a treated nucleic acid sequence according to one of SEQ ID NO:65 to SEQ ID NO:320 and SEQ ID NO: 962 to SEQ ID NO: 965 and sequences complementary thereto, wherein the base sequence of said oligomers comprises at least one CpG dinucleotide. More preferably, where the subsequent amplification of d) is carried out by means of methylation specific primers, as described above, said methylation specific primers comprise a sequence having a length of at least 9 nucleotides which hybridizes to a treated nucleic acid sequence according to one of SEQ ID Nos: 133,134,261,262, 189,190,317,318, 101,102,229,230 and most preferably SEQ ID Nos: 962 - 965 and sequences complementary thereto, wherein the base sequence of said oligomers comprises at least one CpG dinucleotide.

In an alternative most preferred embodiment of the method, the subsequent amplification of d) is carried out in the presence of *blocking oligonucleotides*, as described above. Said *blocking oligonucleotides* comprising a sequence having a length of at least 9 nucleotides which hybridizes to a treated nucleic acid sequence according to one of SEQ ID NO:65 to SEQ ID NO: 320 and SEQ ID NO: 961 to 965 and sequences complementary thereto, wherein the base sequence of said oligomers comprises at least one CpG, TpG or CpA dinucleotide. Preferably said *blocking oligonucleotides* comprising a sequence having a length of at least 9 nucleotides which hybridizes to a treated nucleic acid sequence according to one of SEQ ID Nos: 133,134,261,262, 189,190,317,318, 101,102,229,230 and most preferably SEQ ID Nos: 962 - 965 and sequences complementary thereto, wherein the base sequence of said oligomers comprises at least one CpG, TpG or CpA dinucleotide. Step e) of the method, namely the detection of the specific amplificates indicative of the methylation status of one or more CpG positions according to SEQ ID NO:1 to SEQ ID NO:64 and SEQ ID NO: 961 (preferably SEQ ID Nos: 35, 63, 19 and most preferably SEQ ID NO: 961) is carried out by means of real-time detection methods as described above.

Additional embodiments of the invention provide a method for the analysis of the methylation status of genomic DNA according to the invention SEQ ID NO:1 to SEQ ID NO:64 and SEQ ID NO: 961, (preferably SEQ ID Nos: 35, 63, 19 and most preferably SEQ ID NO: 961) and complements thereof without the need for pretreatment.

In the *first step* of such additional embodiments, the genomic DNA sample is isolated from tissue or cellular sources. Preferably, such sources include cell lines, histological slides, body fluids, or tissue embedded in paraffin. In the *second step*, the genomic DNA is extracted. Extraction may be by means that are standard to one skilled in the art, including but not limited to the use of detergent lysates, sonification and vortexing with glass beads. Once the nucleic acids have been extracted, the genomic double-stranded DNA is used in the analysis.

In a preferred embodiment, the DNA may be cleaved prior to the treatment, and this may be by any means standard in the state of the art, in particular with methylation-sensitive restriction endonucleases.

In the *third step*, the DNA is then digested with one or more methylation sensitive restriction enzymes. The digestion is carried out such that hydrolysis of the DNA at the restriction site is informative of the methylation status of a specific CpG dinucleotide.

In the *fourth step*, which is optional but a preferred embodiment, the restriction fragments are amplified. This is preferably carried out using a polymerase chain reaction, and said amplificates may carry suitable detectable labels as discussed above, namely fluorophore labels, radionuclides and mass labels.

In the *fifth step* the amplificates are detected. The detection may be by any means standard in the art, for example, but not limited to, gel electrophoresis analysis, hybridization analysis, incorporation of detectable tags within the PCR products, DNA array analysis, MALDI or ESI analysis.

Subsequent to the determination of the methylation state of the genomic nucleic acids the prognosis of the prostate cancer is deduced based upon the methylation state of at least one CpG dinucleotide sequence of SEQ ID N0: 1 to SEQ ID NO:64 and SEQ ID NO: 961, or an average, or a value reflecting an average methylation state of a plurality of CpG dinucleotide sequences of SEQ ID NO:1 to SEQ ID NO:64 and SEQ ID NO: 961. Preferably said prognosis is based upon the methylation state of at least one CpG dinucleotide sequence of the genes PITX2, SEQ ID NO: 63, GPR7 and SEQ ID NO: 35 (SEQ ID Nos: 35; 63, 19 and most preferably SEQ ID NO: 961), or an average, or a value reflecting an average methylation state of a plurality of CpG dinucleotide sequences of SEQ ID Nos: 35, 63, 19 and most preferably SEQ ID NO: 961. Hypomethylation of said CpG positions are associated with good prognosis, and hypermethylation is associated with poor prognosis. Hypermethylation is in general associated with under expression of mRNA and accordingly polpeptides. The cut-off point for determining hypo and hyper methylation is may be the median methylation level for a given population, or is preferably an optimized cut-off level. For the analysis of SEQ ID NO: 35 it is preferred that the cut-off is between 30% and 40% methylation, and most preferably 36.42%. For the analysis of SEQ ID NO: 63 it is preferred that the cut-off is between 2% and 10% methylation, and most preferably 5.96%. For the analysis of GPR7 it is preferred that the cut-off is between 20% and 10% methylation, and most preferably 16.65%. For the analysis of PITX2 it is preferred that the cut-off is between 20% and 10% methylation, and most preferably 14.27%. Wherein the methods according to the present invention of expression analysis (most preferably by means of methylation analysis), of the herein described markers (preferably PITX2, SEQ ID NO: 63, GPR7 and SEQ ID NO: 35 ) are used to determine the prognosis of a prostate cancer said methods are preferably used in combination with other clinical prognostic variables used to determine prognosis most preferably Gleason score but also including nomogram score and PSA level (i.e. that said variables are factored in or taken into account). In a preferred embodiment of the invention prognostic expression analysis of each of the markers PITX2 and SEQ ID NO: 35 as herein described is carried out on patients who present with organ confined (T2) prostate cancer. PITX2 and SEQ ID NO: 35 have a distinct advantage in determinig the prognosis of T2 prostate cancer, whereas it is current clinical practise that all patients presenting with T2 prostate cancer are deemed to have a good prognosis. According to the present invetion said T2 patients with poor prognosis (hypermethylation) would have a prognosis more typical of patients presenting with T3 (non organ-confined) prostate cancer and may be treated appropriately. Furthermore prognostic expression analysis of each of the markers PITX2 and SEQ ID NO: 63 have further utility in the analysis of patients presenting high Gleason score (8 or higher). Said patients are currently considered to have a poor prognosis, however by means of the herein described method of PITX2 and SEQ ID NO: 63 expression analysis it is for the first time possible to differentiate high Gleason score patients with a poor prognosis (hypermethylation) from those with a good prognosis. Currently all patients with high Gleason score are considered candidates for post-surgical adjuvant treatment, accordingly the method according to the invention enables the prevention of over-treatment of said patients. Furthermore prognostic expression analysis of the marker SEQ ID NO: 63 has further utility in the analysis of patients presenting with poor prognosis based on nomogram score.

Kits

Moreover, an additional aspect of the present invention is a kit comprising, for example: a bisulfite-containing reagent; a set of primer oligonucleotides containing at least two oligonucleotides whose sequences in each case correspond, are complementary, or hybridize under stringent or highly stringent conditions to a 16-base long segment of the sequences SEQ ID NO: 1 to SEQ ID NO: 320 and SEQ ID NO: 961 to 965; oligonucleotides and/or PNA-oligomers; as well as instructions for carrying out and evaluating the described method. In a further preferred embodiment, said kit may further comprise standard reagents for performing a CpG position-specific methylation analysis, wherein said analysis comprises one or more of the following techniques: MS-SNuPE. MSP, MethyLight ™, HeavyMethyl™, COBRA, and nucleic acid sequencing. However, a kit along the lines of the present invention can also contain only part of the aforementioned components. Preferably said kit comprises a bisulfite-containing reagent; a set of primer oligonucleotides containing at least two oligonucleotides whose sequences in each case correspond, are complementary, or hybridize under stringent or highly stringent conditions to a 16-base long segment of the sequences SEQ ID Nos: 133,134,261,262, 189,190,317,318, 101,102,229,230 and most preferably SEQ ID Nos: 962 - 965; oligonucleotides and/or PNA-oligomers; as well as instructions for carrying out and evaluating the described method. In a further preferred embodiment, said kit may further comprise standard reagents for performing a CpG position-specific methylation analysis, wherein said analysis comprises one or more of the following techniques: MS-SNuPE, MSP, MethyLight ™, HeavyMethyl™, COBRA, and nucleic acid sequencing. However, a kit along the lines of the present invention can also contain only part of the aforementioned components.

The described invention further provides a composition of matter useful for providing a prognosis of prostate cancer patients. Said composition comprising at least one nucleic acid 18 base pairs in length of a segment of a nucleic acid sequence selected from the group consisting SEQ ID Nos: 133,134,261,262, 189,190,317,318, 101,102,229,230, 962 - 965, and one or more substances taken from the group comprising : magnesium chloride, dNTP, taq polymerase, bovine serum albumen, an oligomer in particular an oligonucleotide or peptide nucleic acid (PNA)-oligomer, said oligomer comprising in each case at least one base sequence having a length of at least 9 nucleotides which is complementary to, or hybridizes under moderately stringent or stringent conditions to a pretreated genomic DNA according to one of the SEQ ID Nos: 133,134,261,262, 189,190,317,318, 101,102,229,230, 962 - 965 and sequences complementary thereto. It is preferred that said composition of matter comprises a buffer solution appropriate for the stabilization of said nucleic acid in an aqueous solution and enabling polymerase based reactions within said solution. Suitable buffers are known in the art and commercially available.

In one embodiment the invention provides a method for providing a diagnosis of prostate carcinoma or neoplasm in a subject. Said method comprises the following steps:

i) determining the expression levels of one or more genes or gene sequences of or according to CDRN2A, ELK1, GSTP1, RARB, PTGS2, RASSF1, ESR2, ONECUT2, BTG4, SLC35F2, HOXB5, LIMK1, HIST1H4J, SEQ ID NO: 35, EPAS1, NOTCH1, SEQ ID NO: 55, PTPRN2, Q9NP73, MX1, DOCK10, CCND2, ISL1, SNAPC2, GRN, H2AFY2, WDFY3, FOS, FAT, Q86SP6, SLC38A1, SNRPN, GPRK5, FBN2, ARHGEF18, RHOC, KBTBD6, NR2E1, PSD, DRG1, Q8N365, SEQ ID NO: 44, Q96S01, CD37, CMYA3, SEQ ID NO: 61, Q8NCX8 and ZNF566 and/or regulatory regions thereof; and

ii) determining the presence or absence of said prostate carcinoma or neoplasm according to said level of expression.

Said expression level may be determined by any means standard in the art including but not limited to methylation analysis, loss of heterozygosity (hereinafter also referred to as LOH), RNA expression levels and protein expression levels.

Accordingly said method may be enabled by means of any analysis of the expression of a RNA transcribed therefrom or polypeptide or protein translated from said RNA, preferably by means of mRNA expression analysis or polypeptide expression analysis.

Accordingly the prognostic assays and methods, both quantitative and qualitative for detecting the expression of the genes, genomic sequences and/or regulatory regions according to Table 11 as applied to CDRN2A, ELK1, GSTP1, RARB, PTGS2, RASSF1, ESR2, ONECUT2, BTG4, SLC35F2, HOXB5, LIMK1, HIST1H4J, SEQ ID NO: 35, EPAS1, NOTCH1, SEQ ID NO: 55, PTPRN2, Q9NP73, MX1, DOCK10, CCND2, ISL1, SNAPC2, GRN, H2AFY2, WDFY3, FOS, FAT, Q86SP6, SLC38A1, SNRPN, GPRK5, FBN2, ARHGEF18, RHOC, KBTBD6, NR2E1, PSD, DRG1, Q8N365, SEQ ID NO: 44, Q96S01, CD37, CMYA3, SEQ ID NO: 61, Q8NCX8 and ZNF566 are suitable for said diagnostic purposes. Furthermore the compositions of matter, kits and nucleic acids as described above for analysis of the genes and sequences according to Table 11 above are also of use in the analysis of CDRN2A, ELK1, GSTP1, RARB, PTGS2, RASSF1, ESR2, ONECUT2, BTG4, SLC35F2, HOXB5, LIMK1, HIST1H4J, SEQ ID NO: 35, EPAS1, NOTCH1, SEQ ID NO: 55, PTPRN2, Q9NP73, MX1, DOCK10, CCND2, ISL1, SNAPC2, GRN, H2AFY2, WDFY3, FOS, FAT, Q86SP6, SLC38A1, SNRPN, GPRK5, FBN2, ARHGEF18, RHOC, KBTBD6, NR2E1, PSD, DRG1, Q8N365, SEQ ID NO: 44, Q96S01, CD37, CMYA3, SEQ ID NO: 61, Q8NCX8 and ZNF566 and therefore are also applicable in the detection of prostate carcinoma or neoplasms.

While the present invention has been described with specificity in accordance with certain of its preferred embodiments, the following EXAMPLES and TABLES serve only to illustrate the invention and are not intended to limit the invention within the principles and scope of the broadest interpretations and equivalent configurations thereof.

**TABLES 1-12**

TABLE 1: Experimental set-up of MeST screening with number of samples in pools.

| Experiment | Number of comparisons | "Aggressive" group | "Nonaggressive" group |
|---|---|---|---|
| PSA recurrence vs. no recurrence | 3 | 5 tumors from patients who had PSA recurrences <2yr after surgery | 5 tumors from patients without PSA recurrence after at least 4 years follow up |
| Late stage vs Early stage | 1 | 5 stage III and IV tumors | 5 stage I and II tumors |
| High Gleason vs. Low Gleason | 1 | 5 grade 4 or 5 tumors | 5 grade 1, 2, or 3 tumors |
| NAT (PSA recurrence) vs. NAT (no recurrence) | 1 | 4 normal samples from tissue adjacent to tumors from patients who had PSA recurrences <2yr after surgery | 4 normal tissue samples adjacent to tumors from patients without PSA recurrence after at least 4 years follow up |
| Peripheral Zone vs. Transition Zone | 1 | 5 peripheral zone tumors | 5 transition zone tumors |

TABLE 2. Summarized results of MeST Screening experiments

| Experiment: | |
|---|---|
| Total number of MeSTs<br>scoring > 0 | 441<br>278 |
| MeSTs from MCA<br>scoring > 0 | 242<br>126 |
| MeSTs from AP-PCR<br>scoring > 0 | 199<br>152 |

TABLE 3: Total number of MeSTs and number of positive MeSTs from each screening comparison.

| Experiment | Number of MeSTs | Number of Positive MeSTs | Percent Positive MeSTs |
|---|---|---|---|
| PSA recurrence vs no recurrence I | 41 | 28 | 68% |
| PSA recurrence vs. no recurrence II | 113 | 74 | 65% |
| PSA recurrence vs. no recurrence II | 69 | 43 | 62% |
| Late stage vs Early stage | 64 | 39 | 61% |
| High Gleason vs. Low Gleason | 63 | 48 | 76% |
| NAT (PSA recurrence) vs NAT (no recurrence) | 103 | 66 | 64% |
| Peripheral Zone vs. Transition Zone | 76 | 48 | 63% |

TABLE 4 Summary of Real-time PCR data for seven candidates. P values are from a Wilcoxon test and are uncorrected for multiple comparisons. The sensitivity is calculated when the specificity is set at 0.85 or greater.

| Candidate | Gene Name | AUC | Sensitivity | Specificity | P value (Wilcoxon) |
|---|---|---|---|---|---|
| SEQ ID NO: 19 | GPR7 | 0.76 | 0.50 | 0.87 | 0.0008 |
| SEQ ID NO: 35 | Genomic region downstream of FOXL2 | 0.75 | 0.54 | 0.87 | 0.0016 |
| SEQ ID NO: 37 | ABHD9 | 0.7 | 0.38 | 0.86 | 0.0115 |
| SEQ ID NO: 7 | GSTP1 | 0.69 | 0.38 | 0.87 | 0.0176 |
| SEQ ID NO: 63 | HIST2H2BF regulatory region | 0.68 | 0.35 | 0.87 | 0.0241 |
| SEQ ID NO: 8 | RARB | 0.68 | 0.50 | 0.85 | 0.0214 |
| SEQ ID NO: 64 | PMF1 | 0.47 | 0.15 | 0.87 | 0.7138 |

TABLE 5 A summary of the samples used in the chip study. For the outcome categories, "No recurrence" means the patient did not have a relapse and at least 48 months follow up information was available. "Early recurrence" indicates that the patient experienced PSA relapse in less than 2 years after surgery. "Other" includes patients with no follow up information and patients who did not fit the criteria for the recurrence categories.

| Gleason Categories | Sample Number | Outcome categories |
|---|---|---|
| A) Low Gleason (1+2,2+1 2+2, 2+3, 3+2, and 3+3) | 135 | 1. No recurrence (n=42) |
| | | 2. Early recurrence (n=6) |
| | | 3. Other (n=87) |
| B) Intermediate Gleason (2+4, 4+2, 3+4, 4+3, 2+5, 5+2) | 73 | 1. No recurrence (n=34) |
| | | 2. Early recurrence (n=33) |
| | | 3. Other (n=6) |

(continued)

| Gleason Categories | Sample Number | Outcome categories |
|---|---|---|
| C) High Gleason (3+5, 5+3, 4+4, 4+5, 5+4, and 5+5) | 99 | 1. No recurrence (n=9) |
| | | 2. Early recurrence (n=26) |
| | | 3. Other (n=64) |
| D) No Gleason information | 4 | 1. No recurrence (n=3) |
| | | 2. Early recurrence (n=0) |
| | | 3. Other (n=1) |

TABLE 6 Corrected Wilcoxon p-value I AUC / Sensitivity / Specificity of the amplificates. Sensitivity is reported at a fixed specificity of -0.75.

| Marker | p-value | AUC | Sensitivity | Specificity |
|---|---|---|---|---|
| SEQ ID NO: 19 | 2.05E-06 | 0.72 | 0.58 | 0.75 |
| SEQ ID NO: 41 | 2.17E-06 | 0.71 | 0.51 | 0.75 |
| SEQ ID NO: 37 | 4.15E-06 | 0.71 | 0.55 | 0.75 |
| SEQ ID NO: 51 | 8.68E-06 | 0.71 | 0.51 | 0.75 |
| SEQ ID NO: 35 | 2.60E-05 | 0.70 | 0.48 | 0.75 |
| SEQ ID NO: 4 | 5.52E-05 | 0.69 | 0.51 | 0.75 |
| SEQ ID NO: 17 | 5.89E-05 | 0.69 | 0.51 | 0.75 |
| SEQ ID NO: 16 | 6.82E-05 | 0.69 | 0.47 | 0.75 |
| SEQ ID NO: 9 | 9.30E-05 | 0.69 | 0.57 | 0.75 |
| SEQ ID NO: 47 | 1.80E-04 | 0.68 | 0.46 | 0.75 |
| SEQ ID NO: 49 | 2.05E-04 | 0.68 | 0.49 | 0.75 |
| SEQ ID NO: 42 | 3.84E-04 | 0.68 | 0.45 | 0.75 |
| SEQ ID NO: 57 | 4.34E-04 | 0.68 | 0.49 | 0.75 |
| SEQ ID NO: 1 | 7.44E-04 | 0.67 | 0.51 | 0.75 |
| SEQ ID NO: 7 | 8.06E-04 | 0.67 | 0.43 | 0.75 |
| SEQ ID NO: 62 | 8.06E-04 | 0.67 | 0.49 | 0.75 |
| SEQ ID NO: 8 | 9.30E-04 | 0.67 | 0.47 | 0.75 |
| SEQ ID NO: 58 | 1.24E-03 | 0.67 | 0.46 | 0.75 |
| SEQ ID NO: 3 | 2.79E-03 | 0.66 | 0.48 | 0.75 |
| SEQ ID NO: 13 | 3.84 E-03 | 0.66 | 0.45 | 0.75 |
| SEQ ID NO: 23 | 8.68E-03 | 0.65 | 0.43 | 0.75 |
| SEQ ID NO: 29 | 9.30E-03 | 0.65 | 0.41 | 0.75 |
| SEQ ID NO: 39 | 9.30E-03 | 0.65 | 0.41 | 0.75 |
| SEQ ID NO: 5 | 1.05E-02 | 0.65 | 0.47 | 0.75 |
| SEQ ID NO: 56 | 0.03 | 0.64 | 0.46 | 0.75 |
| SEQ ID NO: 10 | 0.10 | 0.62 | 0.41 | 0.75 |
| SEQ ID NO: 2 | 0.11 | 0.62 | 0.40 | 0.75 |
| SEQ ID NO: 6 | 0.13 | 0.62 | 0.40 | 0.75 |

(continued)

| Marker | p-value | AUC | Sensitivity | Specificity |
|---|---|---|---|---|
| SEQ ID NO: 50 | 0.25 | 0.61 | 0.42 | 0.75 |
| SEQ ID NO: 33 | 0.36 | 0.61 | 0.47 | 0.75 |
| SEQ ID NO: 15 | 0.37 | 0.61 | 0.35 | 0.75 |
| SEQ ID NO: 60 | 0.39 | 0.61 | 0.43 | 0.75 |
| SEQ ID NO: 55 | 0.46 | 0.60 | 0.31 | 0.75 |
| SEQ ID NO: 52 | 0.47 | 0.60 | 0.38 | 0.75 |
| SEQ ID NO: 20 | 0.87 | 0.60 | 0.33 | 0.75 |
| SEQ ID NO: 21 | 0.87 | 0.60 | 0.42 | 0.75 |
| SEQ ID NO: 24 | 1.00 | 0.59 | 0.35 | 0.75 |
| SEQ ID NO: 54 | 1.00 | 0.59 | 0.36 | 0.75 |
| SEQ ID NO: 30 | 1.00 | 0.59 | 0.36 | 0.75 |
| SEQ ID NO: 43 | 1.00 | 0.42 | 0.22 | 0.75 |
| SEQ ID NO: 48 | 1.00 | 0.58 | 0.35 | 0.75 |
| SEQ ID NO: 45 | 1.00 | 0.58 | 0.32 | 0.75 |
| SEQ ID NO: 26 | 1.00 | 0.58 | 0.38 | 0.75 |
| SEQ ID NO: 31 | 1.00 | 0.57 | 0.42 | 0.75 |
| SEQ ID NO: 38 | 1.00 | 0.56 | 0.34 | 0.75 |
| SEQ ID NO: 28 | 1.00 | 0.56 | 0.36 | 0.75 |
| SEQ ID NO: 32 | 1.00 | 0.56 | 0.33 | 0.75 |
| SEQ ID NO: 40 | 1.00 | 0.56 | 0.30 | 0.75 |
| SEQ ID NO: 27 | 1.00 | 0.55 | 0.34 | 0.75 |
| SEQ ID NO: 61 | 1.00 | 0.45 | 0.21 | 0.75 |
| SEQ ID NO: 11 | 1.00 | 0.54 | 0.34 | 0.75 |
| SEQ ID NO: 44 | 1.00 | 0.54 | 0.28 | 0.75 |
| SEQ ID NO: 53 | 1.00 | 0.54 | 0.21 | 0.75 |
| SEQ ID NO: 22 | 1.00 | 0.54 | 0.28 | 0.75 |
| SEQ ID NO: 18 | 1.00 | 0.47 | 0.20 | 0.75 |
| SEQ ID NO: 59 | 1.00 | 0.47 | 0.25 | 0.75 |
| SEQ ID NO: 36 | 1.00 | 0.47 | 0.22 | 0.75 |
| SEQ ID NO: 12 | 1.00 | 0.53 | 0.24 | 0.75 |
| SEQ ID NO: 34 | 1.00 | 0.52 | 0.27 | 0.75 |
| SEQ ID NO: 46 | 1.00 | 0.48 | 0.19 | 0.75 |
| SEQ ID NO: 25 | 1.00 | 0.51 | 0.25 | 0.75 |
| SEQ ID NO: 14 | 1.00 | 0.51 | 0.24 | 0.75 |

TABLE 7: Corrected Wilcoxon p-value / AUC / Sensitivity / Specificity of the amplificates. Sensitivity is reported at a fixed specificity of ~0.75.

| Marker | p-value | AUC | Sensitivity | Specificity |
|---|---|---|---|---|
| SEQ ID NO: 19 | 2.42E-04 | 0.72 | 0.59 | 0.76 |
| SEQ ID NO: 35 | 2.48E-04 | 0.72 | 0.59 | 0.76 |
| SEQ ID NO: 37 | 0.05 | 0.66 | 0.37 | 0.76 |
| SEQ ID NO: 20 | 0.07 | 0.66 | 0.44 | 0.76 |
| SEQ ID NO: 13 | 0.08 | 0.65 | 0.46 | 0.76 |
| SEQ ID NO: 9 | 0.11 | 0.65 | 0.37 | 0.76 |
| SEQ ID NO: 41 | 0.11 | 0.65 | 0.37 | 0.76 |
| SEQ ID NO: 57 | 0.11 | 0.65 | 0.41 | 0.76 |
| SEQ ID NO: 51 | 0.12 | 0.65 | 0.49 | 0.76 |
| SEQ ID NO: 4 | 0.22 | 0.64 | 0.40 | 0.76 |
| SEQ ID NO: 1 | 0.24 | 0.64 | 0.41 | 0.76 |
| SEQ ID NO: 10 | 0.57 | 0.62 | 0.38 | 0.76 |
| SEQ ID NO: 15 | 0.61 | 0.62 | 0.38 | 0.76 |
| SEQ ID NO: 17 | 0.74 | 0.62 | 0.32 | 0.76 |
| SEQ ID NO: 58 | 0.93 | 0.62 | 0.40 | 0.76 |
| SEQ ID NO: 62 | 1.00 | 0.61 | 0.49 | 0.76 |
| SEQ ID NO: 43 | 1.00 | 0.39 | 0.11 | 0.76 |
| SEQ ID NO: 29 | 1.00 | 0.61 | 0.37 | 0.76 |
| SEQ ID NO: 16 | 1.00 | 0.60 | 0.35 | 0.76 |
| SEQ ID NO: 23 | 1.00 | 0.60 | 0.40 | 0.76 |
| SEQ ID NO: 5 | 1.00 | 0.60 | 0.44 | 0.76 |
| SEQ ID NO: 42 | 1.00 | 0.60 | 0.32 | 0.76 |
| SEQ ID NO: 49 | 1.00 | 0.59 | 0.37 | 0.76 |
| SEQ ID NO: 3 | 1.00 | 0.59 | 0.40 | 0.76 |
| SEQ ID NO: 47 | 1.00 | 0.59 | 0.35 | 0.76 |
| SEQ ID NO: 7 | 1.00 | 0.59 | 0.27 | 0.76 |
| SEQ ID NO: 28 | 1.00 | 0.59 | 0.37 | 0.76 |
| SEQ ID NO: 2 | 1.00 | 0.59 | 0.29 | 0.76 |
| SEQ ID NO: 18 | 1.00 | 0.42 | 0.14 | 0.76 |
| SEQ ID NO: 45 | 1.00 | 0.58 | 0.38 | 0.76 |
| SEQ ID NO: 8 | 1.00 | 0.58 | 0.25 | 0.76 |
| SEQ ID NO: 46 | 1.00 | 0.42 | 0.13 | 0.76 |
| SEQ ID NO: 30 | 1.00 | 0.58 | 0.32 | 0.76 |
| SEQ ID NO: 39 | 1.00 | 0.58 | 0.35 | 0.76 |
| SEQ ID NO: 54 | 1.00 | 0.57 | 0.32 | 0.76 |
| SEQ ID NO: 25 | 1.00 | 0.43 | 0.17 | 0.76 |
| SEQ ID NO: 6 | 1.00 | 0.57 | 0.30 | 0.76 |

(continued)

| Marker | p-value | AUC | Sensitivity | Specificity |
|---|---|---|---|---|
| SEQ ID NO: 11 | 1.00 | 0.57 | 0.41 | 0.76 |
| SEQ ID NO: 36 | 1.00 | 0.57 | 0.33 | 0.76 |
| SEQ ID NO: 60 | 1.00 | 0.57 | 0.32 | 0.76 |
| SEO ID NO: 61 | 1.00 | 0.43 | 0.13 | 0.76 |
| SEQ ID NO: 55 | 1.00 | 0.56 | 0.27 | 0.76 |
| SEQ ID NO: 33 | 1.00 | 0.56 | 0.27 | 0.76 |
| SEQ ID NO: 21 | 1.00 | 0.56 | 0.21 | 0.76 |
| SEQ ID NO: 56 | 1.00 | 0.56 | 0.32 | 0.76 |
| SEQ ID NO: 24 | 1.00 | 0.56 | 0.27 | 0.76 |
| SEQ ID NO: 38 | 1.00 | 0.55 | 0.37 | 0.76 |
| SEQ ID NO: 48 | 1.00 | 0.55 | 0.33 | 0.76 |
| SEQ ID NO: 26 | 1.00 | 0.45 | 0.22 | 0.76 |
| SEQ ID NO: 59 | 1.00 | 0.54 | 0.38 | 0.76 |
| SEQ ID NO: 34 | 1.00 | 0.47 | 0.22 | 0.76 |
| SEQ ID NO: 52 | 1.00 | 0.53 | 0.22 | 0.76 |
| SEQ ID NO: 50 | 1.00 | 0.53 | 0.27 | 0.76 |
| SEQ ID NO: 14 | 1.00 | 0.47 | 0.21 | 0.76 |
| SEQ ID NO: 22 | 1.00 | 0.47 | 0.27 | 0.76 |
| SEQ ID NO: 32 | 1.00 | 0.52 | 0.29 | 0.76 |
| SEQ ID NO: 53 | 1.00 | 0.52 | 0.25 | 0.76 |
| SEQ ID NO: 44 | 1.00 | 0.48 | 0.27 | 0.76 |
| SEQ ID NO: 40 | 1.00 | 0.49 | 0.13 | 0.76 |
| SEQ ID NO: 27 | 1.00 | 0.50 | 0.32 | 0.76 |
| SEQ ID NO: 12 | 1.00 | 0.50 | 0.21 | 0.76 |
| SEQ ID NO: 31 | 1.00 | 0.50 | 0.16 | 0.76 |

TABLE 8: Corrected Wilcoxon p-value / AUC / Sensitivity / Specificity of the amplificates. Sensitivity is reported at a fixed specificity of ~0.75.

| Marker | p-value | AUC | Sensitivity | Specificity |
|---|---|---|---|---|
| SEQ ID NO: 19 | 2.42E-04 | 0.72 | 0.59 | 0.76 |
| SEQ ID NO: 35 | 2.48E-04 | 0.72 | 0.59 | 0.76 |
| SEQ ID NO: 37 | 0.05 | 0.66 | 0.37 | 0.76 |
| SEQ ID NO: 20 | 0.07 | 0.66 | 0.44 | 0.76 |
| SEQ ID NO: 13 | 0.08 | 0.65 | 0.46 | 0.76 |
| SEQ ID NO: 9 | 0.11 | 0.65 | 0.37 | 0.76 |
| SEQ ID NO: 41 | 0.11 | 0.65 | 0.37 | 0.76 |
| SEQ ID NO: 57 | 0.11 | 0.65 | 0.41 | 0.76 |
| SEQ ID NO: 51 | 0.12 | 0.65 | 0.49 | 0.76 |

(continued)

| Marker | p-value | AUC | Sensitivity | Specificity |
|---|---|---|---|---|
| SEQ ID NO: 4 | 0.22 | 0.64 | 0.40 | 0.76 |
| SEQ ID NO: 1 | 0.24 | 0.64 | 0.41 | 0.76 |
| SEQ ID NO: 10 | 0.57 | 0.62 | 0.38 | 0.76 |
| SEQ ID NO: 15 | 0.61 | 0.62 | 0.38 | 0.76 |
| SEQ ID NO: 17 | 0.74 | 0.62 | 0.32 | 0.76 |
| SEQ ID NO: 58 | 0.93 | 0.62 | 0.40 | 0.76 |
| SEQ ID NO: 62 | 1.00 | 0.61 | 0.49 | 0.76 |
| SEQ ID NO: 43 | 1.00 | 0.39 | 0.11 | 0.76 |
| SEQ ID NO: 29 | 1.00 | 0.61 | 0.37 | 0.76 |
| SEQ ID NO: 16 | 1.00 | 0.60 | 0.35 | 0.76 |
| SEQ ID NO: 23 | 1.00 | 0.60 | 0.40 | 0.76 |
| SEQ ID NO: 5 | 1.00 | 0.60 | 0.44 | 0.76 |
| SEQ ID NO: 42 | 1.00 | 0.60 | 0.32 | 0.76 |
| SEQ ID NO: 49 | 1.00 | 0.59 | 0.37 | 0.76 |
| SEQ ID NO: 3 | 1.00 | 0.59 | 0.40 | 0.76 |
| SEQ ID NO: 47 | 1.00 | 0.59 | 0.35 | 0.76 |
| SEQ ID NO: 7 | 1.00 | 0.59 | 0.27 | 0.76 |
| SEQ ID NO: 28 | 1.00 | 0.59 | 0.37 | 0.76 |
| SEQ ID NO: 2 | 1.00 | 0.59 | 0.29 | 0.76 |
| SEQ ID NO: 18 | 1.00 | 0.42 | 0.14 | 0.76 |
| SEQ ID NO: 45 | 1.00 | 0.58 | 0.38 | 0.76 |
| SEQ ID NO: 8 | 1.00 | 0.58 | 0.25 | 0.76 |
| SEQ ID NO: 46 | 1.00 | 0.42 | 0.13 | 0.76 |
| SEQ ID NO: 30 | 1.00 | 0.58 | 0.32 | 0.76 |
| SEQ ID NO: 39 | 1.00 | 0.58 | 0.35 | 0.76 |
| SEQ ID NO: 54 | 1.00 | 0.57 | 0.32 | 0.76 |
| SEQ ID NO: 25 | 1.00 | 0.43 | 0.17 | 0.76 |
| SEQ ID NO: 6 | 1.00 | 0.57 | 0.30 | 0.76 |
| SEQ ID NO: 11 | 1.00 | 0.57 | 0.41 | 0.76 |
| SEQ ID NO: 36 | 1.00 | 0.57 | 0.33 | 0.76 |
| SEQ ID NO: 60 | 1.00 | 0.57 | 0.32 | 0.76 |
| SEQ ID NO: 61 | 1.00 | 0.43 | 0.13 | 0.76 |
| SEQ ID NO: 55 | 1.00 | 0.56 | 0.27 | 0.76 |
| SEQ ID NO: 33 | 1.00 | 0.56 | 0.27 | 0.76 |
| SEQ ID NO: 21 | 1.00 | 0.56 | 0.21 | 0.76 |
| SEQ ID NO: 56 | 1.00 | 0.56 | 0.32 | 0.76 |
| SEQ ID NO: 24 | 1.00 | 0.56 | 0.27 | 0.76 |
| SEQ ID NO: 38 | 1.00 | 0.55 | 0.37 | 0.76 |

(continued)

| Marker | p-value | AUC | Sensitivity | Specificity |
|---|---|---|---|---|
| SEQ ID NO: 48 | 1.00 | 0.55 | 0.33 | 0.76 |
| SEQ ID NO: 26 | 1.00 | 0.45 | 0.22 | 0.76 |
| SEQ ID NO: 59 | 1.00 | 0.54 | 0.38 | 0.76 |
| SEQ ID NO: 34 | 1.00 | 0.47 | 0.22 | 0.76 |
| SEQ ID NO: 52 | 1.00 | 0.53 | 0.22 | 0.76 |
| SEQ ID NO: 50 | 1.00 | 0.53 | 0.27 | 0.76 |
| SEQ ID NO: 14 | 1.00 | 0.47 | 0.21 | 0.76 |
| SEQ ID NO: 22 | 1.00 | 0.47 | 0.27 | 0.76 |
| SEQ ID NO: 32 | 1.00 | 0.52 | 0.29 | 0.76 |
| SEQ ID NO: 53 | 1.00 | 0.52 | 0.25 | 0.76 |
| SEQ ID NO: 44 | 1.00 | 0.48 | 0.27 | 0.76 |
| SEQ ID NO: 40 | 1.00 | 0.49 | 0.13 | 0.76 |
| SEQ ID NO: 27 | 1.00 | 0.50 | 0.33 | 0.76 |
| SEQ ID NO: 12 | 1.00 | 0.50 | 0.21 | 0.76 |
| SEQ ID NO: 31 | 1.00 | 0.50 | 0.16 | 0.76 |

TABLE 9: Primers according to EXAMPLE 3.

| *Gene* | *Primers:* | *Amplificate Length:* |
|---|---|---|
| CCND2 (SEQ ID NO: 1) | AAAAACAACCTTAACTC AAACAT (SEQ ID NO: 322) TTTGGAGGGATAGAAT GTGA (SEQ ID NO: 321) | 504 |
| CDKN2A (SEQ ID NO: 2) | AGATTATTAGTTTTTATT TGAGGGATT (SEQ ID NO: 323) CCACCCTAACTCTAACC ATTC (SEQ ID NO: 324) | |
| CD44 (SEQ ID NO: 3) | TTTTTGTTTGGGTGTGT TTT | 403 |

(continued)

| Gene | Primers: | Amplificate Length: |
|---|---|---|
| | (SEQ ID NO: 325) CACTTAACTCCAATCCCCC (SEQ ID NO: 326) | |
| EDNRB1 (SEQ ID NO: 4) | CAAAAACTTCTCAAATCAACAA (SEQ ID NO: 328) GAAGGGATGAATGAATAAAAGT (SEQ ID NO: 327) | 446 |
| ELK1 (SEQ ID NO: 5) | TCCAATAAACACAAACCTAAATC (SEQ ID NO: 330) ATATGGGATTGATGGAAGATAG (SEQ ID NO: 329) | |
| FOS (SEQ ID NO: 6) | TTTTTATTTAGGATGAGGGATATT (SEQ ID NO: 331) ACTACTTCCCACCCAACC (SEQ ID NO: 332) | |
| GSTP1 (SEQ ID NO: 7) | CTACTATCTATTTACTCCCTAAAC (SEQ ID NO: 334) TTGGTTTTATGTTGGGAGTTTTG (SEQ ID NO: 333) | 347 |
| RARB (SEQ ID NO: 8) | GGGAGTTTTTAAGTTTTGTGAG (SEQ ID NO: 335) TTAATCTTTTTCCCAACCC (SEQ ID NO: 336) | 488 |

(continued)

| Gene | Primers: | Amplificate Length: |
|---|---|---|
| PTGS2 (SEQ ID NO: 9) | AACATATCAACCTTTCT TAACCTT (SEQ ID NO: 338) AGAGGGGGTAGTTTTT ATTTTT (SEQ ID NO: 337) | 344 |
| RASSF1 (SEQ ID NO: 10) | AGTGGGTAGGTTAAGT GTGTTG (SEQ ID NO: 339) CCCCAAAATCCAAACTA AA (SEQ ID NO: 340) | 319 |
| ESR2 (SEQ ID NO: 11) | AGTTGGAGAAATTGAAA AGATTA (SEQ ID NO: 341) TAACAAACCCAAAACCT CTCTA (SEQ ID NO: 342) | 441 |
| DRG1 (SEQ ID NO: 12) | TTTAGTTGTGAAAAAGG GATTT (SEQ ID NO: 343) CCCTATAACCTCCACAC TATCTC (SEQ ID NO: 344) | 436 |
| DRG1 (SEQ ID NO: 12) | CCCATCCCACAATTAAA A (SEQ ID NO: 346) GTTTTGGAGGGAGTAG AGATT (SEQ ID NO: 345) | |
| CMYA3 (SEQ ID NO: 13) | ACTCCCCAAAATCCCA CT (SEQ ID NO: 348) | 488 |

(continued)

| Gene | Primers: | Amplificate Length: |
|---|---|---|
| | TGTTTTAGGTTTGATGG ATTAGA (SEQ ID NO: 347) | |
| ONECUT2 (SEQ ID NO: 14) | GAAGAGGTGTTGAGAA ATTAAAA (SEQ ID NO: 349) CCCACCCTAACTTACCA TAAA (SEQ ID NO: 350) | 462 |
| MX1 (SEQ ID NO: 15) | TGTAGGAGAGGTTGGG AAG (SEQ ID NO: 351) CCAAACATAACATCCAC TAAAA (SEQ ID NO: 352) | 341 |
| DOCK10 (SEQ ID NO: 16) | TACCTCTTCCCTCTACC AAAC (SEQ ID NO: 354) GTTTTTAAGTGTTGGGT GATTT (SEQ ID NO: 353) | 459 |
| BTG4 (SEQ ID NO: 17) | AAGAGTTTTAGGAAATG TGTTTTT (SEQ ID NO: 355) TTCTACTCACCAAACCC TCTAC (SEQ ID NO: 356) | 199 |
| DMRTC2 (SEQ ID NO: 18) | TAAGGTAAGGGAAGGT TAGAAA (SEQ ID NO: 357) ATTACCACAACCTCCAA TAAAA (SEQ ID NO: 358) | 477 |

(continued)

| Gene | Primers: | Amplificate Length: |
|---|---|---|
| GPR7 (SEQ ID NO: 19) | TTATTATTTTAGATGGA GTGAGGTT (SEQ ID NO: 359) ACCCAAATTACCCCACA A (SEQ ID NO: 360) | 442 |
| FAT (SEQ ID NO: 20) | TACTCACCCCAATCTTC ACTA (SEQ ID NO: 362) AGATGTTTTATATTTGT TTGGGA (SEQ ID NO: 361) | 444 |
| ISL1 (SEQ ID NO: 21) | ATCTCCCAAAAACAATC ACA (SEQ ID NO: 364) TAAAAATGGAAGGGAA GATAGA (SEQ ID NO: 363) | 412 |
| GPRK5 (SEQ ID NO: 22) | TTGTGGTTATTTTGAGA TGGTA (SEQ ID NO: 365) CCCTCCCCCTAACTAAA A (SEQ ID NO: 366) | 416 |
| SLC35F2 (SEQ ID NO: 23) | TTTATTTATTAGGTGAA GAGTTTGTT (SEQ ID NO: 367) TCCTCCTACCACCCTAA AA (SEQ ID NO: 368) | 366 |
| C14orf59 (SEQ ID NO: 24) | AAAACTCCTCCCCTCTA TAAAT (SEQ ID NO: 370) | 492 |

(continued)

| Gene | Primers: | Amplificate Length: |
|---|---|---|
| | TTGGAGAGATGTGTTG GTTAG (SEQ ID NO: 369) | |
| SNRPN (SEQ ID NO: 25) | CCCCTCTCATTACAACA ATACT (SEQ ID NO: 372) TTTTTAGAATAAAGGAT TTTAGGG (SEQ ID NO: 371) | 407 |
| ARHGEF18 (SEQ ID NO: 26) | TTTTAGGAATGTAGGAT ATAAGGG (SEQ ID NO: 373) CCCCACATAAAAACCTA TCC (SEQ ID NO: 374) | 454 |
| SNX8 (SEQ ID NO: 27) | TCCCTCCTAACTCAACA CTAA (SEQ ID NO: 376) TAGGGTTTGATGATGT GATTTT (SEQ ID NO: 375) | 273 |
| FBN2 (SEQ ID NO: 28) | GAGAGGGAGGGTTAAG GTT (SEQ ID NO: 377) ACTACTACCTCCTTTCC CAAAT (SEQ ID NO: 378) | 193 |
| HOXB5 (SEQ ID NO: 29) | CTCCTCAATTCTCACCA AAA (SEQ ID NO: 380) GTGGAAAAAGGAGAGT AAATTG (SEQ ID NO: 379) | 356 |

(continued)

| Gene | Primers: | Amplificate Length: |
|---|---|---|
| LIMK1 (SEQ ID NO: 30) | AAACCCTACTTCCTACA AACAA (SEQ ID NO: 382) AGGGAGGTTTGGTGTA TTTT (SEQ ID NO: 381) | |
| PSD/Q9H469 (SEQ ID NO: 31) | AAGGTATTATTTTTGGG GTTTT (SEQ ID NO: 383) AACTATCCAACCTCTTC CACTT (SEQ ID NO: 384) | 333 |
| SLC38A1 (SEQ ID NO: 32) | GGGTGTTGGGGAGTTT TA (SEQ ID NO: 385) CTTACAATAACTCACTA TCCTTTCC (SEQ ID NO: 386) | 334 |
| SLC38A1 (SEQ ID NO: 32) | ACAAACATCCTTTAATA ATTTCTCC (SEQ ID NO: 388) AGAGTGTGGTATTAGAT TTGGTTTT (SEQ ID NO: 387) | 317 |
| HIST1 H4J (SEQ ID NO: 33) | TTAGTTGAGAAAGTGG GGGT (SEQ ID NO: 389) CTACCTCAAACCAAAAT CCTC (SEQ ID NO: 390) | 421 |
| Q96S01 (SEQ ID NO: 34) | ACCCCAATCAACTACAT AACTAA (SEQ ID NO: 392) | 498 |

(continued)

| Gene | Primers: | Amplificate Length: |
|---|---|---|
| | GTGAGAGTGGGTGTTG AAAT (SEQ ID NO: 391) | |
| Genomic region downstream of FOXL2 (SEQ ID NO: 35) | ATTTTAGGTGTAAGTTT AAGGTTGT (SEQ ID NO: 393) ATCTACCTTTCCCCACC C (SEQ ID NO: 394) | 473 |
| ORC4L (SEQ ID NO: 36) | GTTGAGAGGTAAGGTA TGAAGG (SEQ ID NO: 395) TTAATTCCCCTCTTTAA CCTAATAA (SEQ ID NO: 396) | 477 |
| ABHD9 (SEQ ID NO: 37) | GTGTTAGGGTTTAGGG GTTT (SEQ ID NO: 397) CCTTTCCAACCTCTTCC T (SEQ ID NO: 398) | 209 |
| CD37 (SEQ ID NO: 38) | CCTCATCAACCAACCCT A (SEQ ID NO: 400) TAGATGGGGATAGGAA GTTGT (SEQ ID NO: 399) | 466 |
| GRN (SEQ ID NO: 39) | CATTCCAAACTAACCCC A (SEQ ID NO: 402) TTATTAGGAGAGGGGA AGAAGT (SEQ ID NO: 401) | 466 |
| EPAS1 (SEQ ID NO: 40) | TATTGGATGTTTTTGGT AGGTT | 479 |

(continued)

| Gene | Primers: | Amplificate Length: |
| --- | --- | --- |
| | (SEQ ID NO: 403) AATCACCTCCCTCCCTTA (SEQ ID NO: 404) | |
| NOTCH1 (SEQ ID NO: 41) | CCCACCCTAAAAACTCACTA (SEQ ID NO: 406) GGAGGGGTTTGAGTAATTG (SEQ ID NO: 405) | 424 |
| MLLT3 (SEQ ID NO: 42) | GATTAGGTTTGGAGTTGTTTTT (SEQ ID NO: 407) AATCACATCCATCTTTCACTTT (SEQ ID NO: 408) | |
| SOLH (SEQ ID NO: 43) | CCCAACTCCCCAAATAAA (SEQ ID NO: 410) GGGGATAAGTGGTTAATGAGT (SEQ ID NO: 409) | 389 |
| SEQ ID NO: 44 (SEQ ID NO: 44) | ATAGTGTGGATTTTTAGGGATATT (SEQ ID NO: 411) CAAAACCTATTCCCCTACCT (SEQ ID NO: 412) | 448 |
| Q8N365 (SEQ ID NO: 45) | GTAGTAAATGGGTTATGGATTTTAG (SEQ ID NO: 413) CCATACCACCCACAAACA (SEQ ID NO: 414) | 475 |

(continued)

| Gene | Primers: | Amplificate Length: |
|---|---|---|
| Q9NWVO (SEQ ID NO: 46) | TTTTAGTAGTGGATTTG GTTAGTATT (SEQ ID NO: 415) CATCTCTTAACCCCACT TTCA (SEQ ID NO: 416) | 330 |
| SEQ ID NO: 47 (SEQ ID NO: 47) | TCCCTCTAAAAACCAAA AATC (SEQ ID NO: 418) GAGGAAAGAAGGAGGA TATAGG (SEQ ID NO: 417) | 335 |
| H2AFY2 (SEQ ID NO: 48) | GTTAAAGGGGTATTGG TTTTT (SEQ ID NO: 419) TTTCTTTTTCTCTCACC TATTAAAC (SEQ ID NO: 420) | 490 |
| RHOC (SEQ ID NO: 49) | GTAAGAGGGATAGGGA ATTGG (SEQ ID NO: 421) AACACCCAAACCAAAAT AAAA (SEQ ID NO: 422) | 440 |
| NR2E1 (SEQ ID NO: 50) | GGAGTTTGTGAAAAGT GGG (SEQ ID NO: 423) ACTCAACAAATACAATA ATCTAAACC (SEQ ID NO: 424) | 429 |
| KBTBD6 (SEQ ID NO: 51) | ACTATACCAACAAAACT ACAAAATAAA (SEQ ID NO: 426) | 228 |

(continued)

| Gene | Primers: | Amplificate Length: |
|---|---|---|
| | GAAGGTTGAGGAGGAG TTAGA (SEQ ID NO: 425) | |
| TRPM4 (SEQ ID NO: 52) | GGTTGGAAAGTGGAGG ATT (SEQ ID NO: 427) CCAACTCTAAAAACAAA AACAA (SEQ ID NO: 428) | 438 |
| TCEB3BP1 (SEQ ID NO: 53) | GAGTTGGTTTTGTTGAG GTGT (SEQ ID NO: 429) AAAATACCTTCCCACTA ACCTT (SEQ ID NO: 430) | 325 |
| Q8NCX8 (SEQ ID NO: 54) | TAGTGGAATTATGAGG GGG (SEQ ID NO: 431) CCAAATACACCTCTACC AAAA (SEQ ID NO: 432) | 300 |
| SEQ ID NO: 55 (SEQ ID NO: 55) | CCTTACCCTCCTCTCCT AAA (SEQ ID NO: 434) TAGGATTTGTGGTTGGT GTT (SEQ ID NO: 433) | 384 |
| SNAPC2 (SEQ ID NO: 56) | GGTTTAGGGTATTTTAA GGGG (SEQ ID NO: 435) AAACTAAATCCAACTCC CAAA (SEQ ID NO: 436) | 362 |

(continued)

| Gene | Primers: | Amplificate Length: |
|---|---|---|
| PTPRN2 (SEQ ID NO: 57) | CCCTCTACTCACTTTAC CAAAA (SEQ ID NO: 438) GGGGAGGTGTTTAGTG GTT (SEQ ID NO: 437) | 378 |
| WDFY3 (SEQ ID NO: 58) | TGTTGGTGGTTATTTTT AATTTTT (SEQ ID NO: 439) ACCCAATTATCCTTTCT CAAC (SEQ ID NO: 440) | 435 |
| ZNF566 (SEQ ID NO: 59) | GAGGATTTGTGTTAAG GTTTTT (SEQ ID NO: 441) CCAACTCCACTATCTAC CACAT (SEQ ID NO: 442) | 457 |
| Q9NP73 (SEQ ID NO: 60) | GGGGTTTTAAGAGTTG GTTTT (SEQ ID NO: 443) CCTCCCTCACTCACTTA CAA (SEQ ID NO: 444) | |
| SEQ ID NO: 61 (SEQ ID NO: 61) | TGGGGATATTGGATGT TTTT (SEQ ID NO: 445) CCTTCCAACCTAACCTC C (SEQ ID NO: 446) | 497 |
| Q86SP6 (SEQ ID NO: 62) | CCCAACACTCATTTACA CTATCT (SEQ ID NO: 448) | 342 |

(continued)

| Gene | Primers: | Amplificate Length: |
|---|---|---|
| | GGAGTTTTAATTTTTGG GATTT (SEQ ID NO: 447) | |

TABLE 10: Detection oligonucleotides according to Example 3.

| No: | Gene | Oligo: |
|---|---|---|
| 1 | ONECUT2 (SEQ ID NO: 14) | TAGAGGCGCGGGTTAT (SEQ ID NO: 449) |
| 2 | ONECUT2 (SEQ ID NO: 14) | TAGAGGTGTGGGTTAT (SEQ ID NO: 450) |
| 3 | ONECUT2 (SEQ ID NO: 14) | TTGCGATTGGTACGTA (SEQ ID NO: 451) |
| 4 | ONECUT2 (SEQ ID NO: 14) | TGTGATTGGTATGTAGT (SEQ ID NO: 452) |
| 5 | ONECUT2 (SEQ ID NO: 14) | TTTTGTGCGTACGGAT (SEQ ID NO: 453) |
| 6 | ONECUT2 (SEQ ID NO: 14) | TTTTTGTGTGTATGGAT (SEQ ID NO: 454) |
| 7 | ONECUT2 (SEQ ID NO: 14) | TTAAGCGGGCGTTGAT (SEQ ID NO: 455) |
| 8 | ONECUT2 (SEQ ID NO: 14) | TTAAGTGGGTGTTGAT (SEQ ID NO: 456) |
| 9 | MX1 (SEQ ID NO: 15) | GTTACGAGTTTATTCGA (SEQ ID NO: 457) |
| 10 | MX1 (SEQ ID NO: 15) | GGTTATGAGTTTATTTGAA (SEQ ID NO: 458) |
| 11 | MX1 (SEQ ID NO: 15) | AACGCGCGAAAGTAAA (SEQ ID NO: 459) |
| 12 | MX1 (SEQ ID NO: 15) | TTGGGAATGTGTGAAA (SEQ ID NO: 460) |
| 13 | MX1 (SEQ ID NO: 15) | GAGTTTTCGTCGATTT (SEQ ID NO: 461) |
| 14 | MX1 (SEQ ID NO: 15) | AGGAGTTTTTGTTGATT (SEQ ID NO: 462) |
| 15 | MX1 (SEQ ID NO: 15) | TATGCGCGGGAAGATT (SEQ ID NO: 463) |
| 16 | MX1 (SEQ ID NO: 15) | GTATGTGTGGGAAGAT (SEQ ID NO: 464) |
| 17 | DOCK10 (SEQ ID NO: 16) | GATCGGAATTCGGGTT (SEQ ID NO: 465) |
| 18 | DOCK10 (SEQ ID NO: 16) | ATTGGAATTTGGGTTG (SEQ ID NO: 466) |
| 19 | DOCK10 (SEQ ID NO: 16) | TAGTAGTCGCGTTTTT (SEQ ID NO: 467) |
| 20 | DOCK10 (SEQ ID NO: 16) | AGTAGTTGTGTTTTTGG (SEQ ID NO: 468) |
| 21 | DOCK10 (SEQ ID NO: 16) | ATTTTCGCGGGAAGTT (SEQ ID NO: 469) |
| 22 | DOCK10 (SEQ ID NO: 16) | GTGATTTTTGTGGGAA (SEQ ID NO: 470) |
| 23 | BTG4 (SEQ ID NO: 17) | AGTTAGCGTTTGTCGG (SEQ ID NO: 471) |
| 24 | BTG4 (SEQ ID NO: 17) | TAGTTAGTGTTTGTTGG (SEQ ID NO: 472) |
| 25 | BTG4 (SEQ ID NO: 17) | TTCGGCGTCGATGTAT (SEQ ID NO: 473) |
| 26 | BTG4 (SEQ ID NO: 17) | TTTGGTGTTGATGTATT (SEQ ID NO: 474) |
| 27 | DMRTC2 (SEQ ID NO: 18) | GGGTATTCGACGTTTT (SEQ ID NO: 475) |
| 28 | DMRTC2 (SEQ ID NO: 18) | AGGGGTATTTGATGTTT (SEQ ID NO: 476) |
| 29 | DMRTC2 (SEQ ID NO: 18) | ATTCGAAGCGTTATTG (SEQ ID NO: 477) |
| 30 | DMRTC2 (SEQ ID NO: 18) | TTTGAAGTGTTATTGGT (SEQ ID NO: 478) |
| 31 | DMRTC2 (SEQ ID NO: 18) | AAATCGTATTGGTCGT (SEQ ID NO: 479) |

(continued)

| *No:* | *Gene* | *Oligo*: |
|---|---|---|
| 32 | DMRTC2 (SEQ ID NO: 18) | AAATTGTATTGGTTGTTT (SEQ ID NO: 480) |
| 33 | DMRTC2 (SEQ ID NO: 18) | AATTCGTGTATAGATCGG (SEQ ID NO: 481) |
| 34 | DMRTC2 (SEQ ID NO: 18) | ATTTGTGTATAGATTGGG (SEQ ID NO: 482) |
| 35 | DMRTC2 (SEQ ID NO: 18) | AAAAGTAGCGCGAGTT (SEQ ID NO: 483) |
| 36 | DMRTC2 (SEQ ID NO: 18) | AGTAGTGTGAGTTTGG (SEQ ID NO: 484) |
| 37 | GPR7 (SEQ ID NO: 19) | GAACGTAGTCGCGGTT (SEQ ID NO: 485) |
| 38 | GPR7 (SEQ ID NO: 19) | GGAATGTAGTTGTGGT (SEQ ID NO: 486) |
| 39 | GPR7 (SEQ ID NO: 19) | ATTTTGGCGAATTCGG (SEQ ID NO: 487) |
| 40 | GPR7 (SEQ ID NO: 19) | TGGTGAATTTGGGGGA (SEQ ID NO: 488) |
| 41 | GPR7 (SEQ ID NO: 19) | TTAGTCGGTAGGCGTT (SEQ ID NO: 489) |
| 42 | GPR7 (SEQ ID NO: 19) | ATTTAGTTGGTAGGTGT (SEQ ID NO: 490) |
| 43 | GPR7 (SEQ ID NO: 19) | TTTTCGTAGTCGGCGG (SEQ ID NO: 491) |
| 44 | GPR7 (SEQ ID NO: 19) | TTTTTTGTAGTTGGTGG (SEQ ID NO: 492) |
| 45 | FAT (SEQ ID NO: 20) | TAAGCGTTAATAGAACGA (SEQ ID NO: 493) |
| 46 | FAT (SEQ ID NO: 20) | AGTGTTAATAGAATGAAAT (SEQ ID NO: 494) |
| 47 | FAT (SEQ ID NO: 20) | TTGTATTTCGTCGTTAT (SEQ ID NO: 495) |
| 48 | FAT (SEQ ID NO: 20) | TTTTGTTGTTATAGGAGT (SEQ ID NO: 496) |
| 49 | FAT (SEQ ID NO: 20) | ATAGGAGTCGTCGAGA (SEQ ID NO: 497) |
| 50 | FAT (SEQ ID NO: 20) | ATAGGAGTTGTTGAGAG (SEQ ID NO: 498) |
| 51 | ISL1 (SEQ ID NO: 21) | TTAATGCGGTCGGTTA (SEQ ID NO: 499) |
| 52 | ISL1 (SEQ ID NO: 21) | AGTTAATGTGGTTGGT (SEQ ID NO: 500) |
| 53 | GPRK5 (SEQ ID NO: 22) | TTTGATTCGCGGTCGG (SEQ ID NO: 501) |
| 54 | GPRK5 (SEQ ID NO: 22) | ATTTGATTTGTGGTTGG (SEQ ID NO: 502) |
| 55 | GPRK5 (SEQ ID NO: 22) | TATCGTCGGTCGAGTT (SEQ ID NO: 503) |
| 56 | GPRK5 (SEQ ID NO: 22) | TTTATTGTTGGTTGAGT (SEQ ID NO: 504) |
| 57 | GPRK5 (SEQ ID NO: 22) | ATGTCGAGAGTTCGTA (SEQ ID NO: 505) |
| 58 | GPRK5 (SEQ ID NO: 22) | GTTGAGAGTTTGTATGT (SEQ ID NO: 506) |
| 59 | SLC35F2 (SEQ ID NO: 23) | TTTCGGCGTTTAAAAT (SEQ ID NO: 507) |
| 60 | SLC35F2 (SEQ ID NO: 23) | TTTTTGGTGTTTAAAATTT (SEQ ID NO: 508) |
| 61 | SLC35F2 (SEQ ID NO: 23) | ATTTTCGAAGTGTCGG (SEQ ID NO: 509) |
| 62 | SLC35F2 (SEQ ID NO: 23) | TTTGAAGTGTTGGGTT (SEQ ID NO: 510) |
| 63 | SLC35F2 (SEQ ID NO: 23) | TTTCGGAAGACGGGAG (SEQ ID NO: 511) |
| 64 | SLC35F2 (SEQ ID NO: 23) | TTTTGGAAGATGGGAG (SEQ ID NO: 512) |
| 65 | SLC35F2 (SEQ ID NO: 23) | AATTCGGTCGTCGTTT (SEQ ID NO: 513) |
| 66 | SLC35F2 (SEQ ID NO: 23) | AGAATTTGGTTGTTGTT (SEQ ID NO: 514) |
| 67 | C14orf59 (SEQ ID NO: 24) | GACGTAGGGACGGAGA (SEQ ID NO: 515) |
| 68 | C14orf59 (SEQ ID NO: 24) | GATGTAGGGATGGAGA (SEQ ID NO: 516) |
| 69 | C14orf59 (SEQ ID NO: 24) | TATCGTGGTTTTTTACGTAT (SEQ ID NO: 517) |

(continued)

| *No:* | *Gene* | *Oligo*: |
|---|---|---|
| 70 | C14orf59 (SEQ ID NO: 24) | ATTGTGGTTTTTTATGTATA (SEQ ID NO: 518) |
| 71 | C14orf59 (SEQ ID NO: 24) | GTGTTCGAGAGCGAGT (SEQ ID NO: 519) |
| 72 | C14orf59 (SEQ ID NO: 24) | TGTTTGAGAGTGAGTGT (SEQ ID NO: 520) |
| 73 | C14orf59 (SEQ ID NO: 24) | TTTATTCGGTGTTCGA (SEQ ID NO: 521) |
| 74 | C14orf59 (SEQ ID NO: 24) | TATTTGGTGTTTGAGAG (SEQ ID NO: 522) |
| 75 | SNRPN (SEQ ID NO: 25) | TTTTTGCGGTCGCGTA (SEQ ID NO: 523) |
| 76 | SNRPN (SEQ ID NO: 25) | TTTTGTGGTTGTGTAGG (SEQ ID NO: 524) |
| 77 | SNRPN (SEQ ID NO: 25) | AGTATGCGCGTTAGTT (SEQ ID NO: 525) |
| 78 | SNRPN (SEQ ID NO: 25) | TGAGTATGTGTGTTAGT (SEQ ID NO: 526) |
| 79 | SNRPN (SEQ ID NO: 25) | TAGCGGTAGGTTTCGTA (SEQ ID NO: 527) |
| 80 | SNRPN (SEQ ID NO: 25) | TAGTGGTAGGTTTTGTA (SEQ ID NO: 528) |
| 81 | SNRPN (SEQ ID NO: 25) | TTTGCGTTAGATTCGT (SEQ ID NO: 529) |
| 82 | SNRPN (SEQ ID NO: 25) | TGTGTTAGATTTGTTGT (SEQ ID NO: 530) |
| 83 | ARHGEF18 (SEQ ID NO: 26) | GTCGATTCGGTTGATT (SEQ ID NO: 531) |
| 84 | ARHGEF18 (SEQ ID NO: 26) | AAGGTTGATTTGGTTG (SEQ ID NO: 532) |
| 85 | ARHGEF18 (SEQ ID NO: 26) | GGCGGTTTCGAAGATT (SEQ ID NO: 533) |
| 86 | ARHGEF18 (SEQ ID NO: 26) | AGATGGGTGGTTTTGA (SEQ ID NO: 534) |
| 87 | ARHGEF18 (SEQ ID NO: 26) | AAGTCGGTTATGAGCGA (SEQ ID NO: 535) |
| 88 | ARHGEF18 (SEQ ID NO: 26) | AAGTTGGTTATGAGTGA (SEQ ID NO: 536) |
| 89 | ARHGEF18 (SEQ ID NO: 26) | TGGTCGATACGGTATT (SEQ ID NO: 537) |
| 90 | ARHGEF18 (SEQ ID NO: 26) | TTGTGGTTGATATGGT (SEQ ID NO: 538) |
| 91 | SNX8 (SEQ ID NO: 27) | AGGACGCGATAGGGAT (SEQ ID NO: 539) |
| 92 | SNX8 (SEQ ID NO: 27) | AGGATGTGATAGGGAT (SEQ ID NO: 540) |
| 93 | SNX8 (SEQ ID NO: 27) | ATTTCGTCGTATGTGA (SEQ ID NO: 541) |
| 94 | SNX8 (SEQ ID NO: 27) | ATTTTGTTGTATGTGAAG (SEQ ID NO: 542) |
| 95 | SNX8 (SEQ ID NO: 27) | GTCGTTTGCGTATTTA (SEQ ID NO: 543) |
| 96 | SNX8 (SEQ ID NO: 27) | GGTTGTTTGTGTATTTAA (SEQ ID NO: 544) |
| 97 | SNX8 (SEQ ID NO: 27) | ATTGTATACGCGCGTT (SEQ ID NO: 545) |
| 98 | SNX8 (SEQ ID NO: 27) | TTGTATATGTGTGTTGG (SEQ ID NO: 546) |
| 99 | FBN2 (SEQ ID NO: 28) | TAAAGCGAGTAGACGG (SEQ ID NO: 547) |
| 100 | FBN2 (SEQ ID NO: 28) | TATAAAGTGAGTAGATGG (SEQ ID NO: 548) |
| 101 | HOXB5 (SEQ ID NO: 29) | ATAGTTTTCGGCGGGT (SEQ ID NO: 549) |
| 102 | HOXB5 (SEQ ID NO: 29) | TATAGTTTTTGGTGGGT (SEQ ID NO: 550) |
| 103 | HOXB5 (SEQ ID NO: 29) | TTTTTCGGCGTAGATA (SEQ ID NO: 551) |
| 104 | HOXB5 (SEQ ID NO: 29) | TGTTTTTTGGTGTAGAT (SEQ ID NO: 552) |
| 105 | HOXB5 (SEQ ID NO: 29) | AGTCGAGGGCGTTAGA (SEQ ID NO: 553) |
| 106 | HOXB5 (SEQ ID NO: 29) | AGTTGAGGGTGTTAGA (SEQ ID NO: 554) |
| 107 | HOXB5 (SEQ ID NO: 29) | TTTTCGAGGAATTCGT (SEQ ID NO: 555) |

(continued)

| No: | Gene | Oligo: |
|---|---|---|
| 108 | HOXB5 (SEQ ID NO: 29) | TTTTTTGAGGAATTTGTT (SEQ ID NO: 556) |
| 109 | LIMK1 (SEQ ID NO: 30) | TATCGGATTATCGCGG (SEQ ID NO: 557) |
| 110 | LIMK1 (SEQ ID NO: 30) | ATTGGATTATTGTGGG (SEQ ID NO: 558) |
| 111 | LIMK1 (SEQ ID NO: 30) | GTCGGTAGTTTATCGGAT (SEQ ID NO: 559) |
| 112 | LIMK1 (SEQ ID NO: 30) | GTTGGTAGTTTATTGGAT (SEQ ID NO: 560) |
| 113 | LIMK1 (SEQ ID NO: 30) | TAGGAGACGTTACGTT (SEQ ID NO: 561) |
| 114 | LIMK1 (SEQ ID NO: 30) | AGATGTTATGTTAGGGT (SEQ ID NO: 562) |
| 115 | PSD/Q9H469 (SEQ ID NO: 31) | TTTTTCGAAGCGGATT (SEQ ID NO: 563) |
| 116 | PSD/Q9H469 (SEQ ID NO: 31) | TTTGAAGTGGATTTTGG (SEQ ID NO: 564) |
| 117 | PSD/Q9H469 (SEQ ID NO: 31) | GAAACGCGGTTTAAAT (SEQ ID NO: 565) |
| 118 | PSD/Q9H469 (SEQ ID NO: 31) | GGAAATGTGGTTTAAATT (SEQ ID NO: 566) |
| 119 | SLC38A1 (SEQ ID NO: 32) | TTTGCGGTAACGTTTA (SEQ ID NO: 567) |
| 120 | SLC38A1 (SEQ ID NO: 32) | TTGTGGTAATGTTTAGG (SEQ ID NO: 568) |
| 121 | SLC38A1 (SEQ ID NO: 32) | TAGCGGTCGCGGATTA (SEQ ID NO: 569) |
| 122 | SLC38A1 (SEQ ID NO: 32) | GTAGTGGTTGTGGATT (SEQ ID NO: 570) |
| 123 | SLC38A1 (SEQ ID NO: 32) | TTAGGGACGCGAATTA (SEQ ID NO: 571) |
| 124 | SLC38A1 (SEQ ID NO: 32) | AGGGATGTGAATTAGG (SEQ ID NO: 572) |
| 125 | SLC38A1 (SEQ ID NO: 32) | TGCGTTTAAGATCGCGT (SEQ ID NO: 573) |
| 126 | SLC38A1 (SEQ ID NO: 32) | TGTGTTTAAGATTGTGT (SEQ ID NO: 574) |
| 127 | SLC38A1 (SEQ ID NO: 32) | ATTTCGGTTTTCGAAA (SEQ ID NO: 575) |
| 128 | SLC38A1 (SEQ ID NO: 32) | ATATTTTGGTTTTTGAAAA (SEQ ID NO: 576) |
| 129 | SLC38A1 (SEQ ID NO: 32) | AGAGCGTAGTTGATTCGA (SEQ ID NO: 577) |
| 130 | SLC38A1 (SEQ ID NO: 32) | AGAGTGTAGTTGATTTGA (SEQ ID NO: 578) |
| 131 | SLC38A1 (SEQ ID NO: 32) | AGGAATTACGTACGTT (SEQ ID NO: 579) |
| 132 | SLC38A1 (SEQ ID NO: 32) | TATGTATGTTTGGAGGG (SEQ ID NO: 580) |
| 133 | SLC38A1 (SEQ ID NO: 32) | TTTGTAACGCGGGGAA (SEQ ID NO: 581) |
| 134 | SLC38A1 (SEQ ID NO: 32) | TTTTGTAATGTGGGGA (SEQ ID NO: 582) |
| 135 | HIST1H4J (SEQ ID NO: 33) | TATGGCGGTGATCGTT (SEQ ID NO: 583) |
| 136 | HIST1H4J (SEQ ID NO: 33) | TTTATGGTGGTGATTGT (SEQ ID NO: 584) |
| 137 | HIST1H4J (SEQ ID NO: 33) | TTACGGCGTTTCGGAT (SEQ ID NO: 585) |
| 138 | HIST1H4J (SEQ ID NO: 33) | TTATGGTGTTTTGGATT (SEQ ID NO: 586) |
| 139 | HIST1H4J (SEQ ID NO: 33) | ATGCGTTTTACGTCGT (SEQ ID NO: 587) |
| 140 | HIST1H4J (SEQ ID NO: 33) | AGATGTGTTTTATGTTGT (SEQ ID NO: 588) |
| 141 | HIST1H4J (SEQ ID NO: 33) | TATTGTCGCGTAGTAT (SEQ ID NO: 589) |
| 142 | HIST1H4J (SEQ ID NO: 33) | GGATATTGTTGTGTAGT (SEQ ID NO: 590) |
| 143 | HIST1H4J (SEQ ID NO: 33) | ATCGAAATCGTAGAGG (SEQ ID NO: 591) |
| 144 | HIST1H4J (SEQ ID NO: 33) | ATTGAAATTGTAGAGGG (SEQ ID NO: 592) |
| 145 | Q96S01 (SEQ ID NO: 34) | ATCGGTTTTTCGAGGT (SEQ ID NO: 593) |

(continued)

| *No:* | *Gene* | *Oligo*: |
|---|---|---|
| 146 | Q96S01 (SEQ ID NO: 34) | ATTGGTTTTTGAGGTT (SEQ ID NO: 594) |
| 147 | Q96S01 (SEQ ID NO: 34) | GGTCGATTTTCGCGTA (SEQ ID NO: 595) |
| 148 | Q96S01 (SEQ ID NO: 34) | TGGTTGATTTTTGTGTA (SEQ ID NO: 596) |
| 149 | GENOMIC REGION DOWNSTREAM FROM FOXL2 (SEQ ID NO: 35) | AATCGTGCGGTTGATA (SEQ ID NO: 597) |
| 150 | GENOMIC REGION DOWNSTREAM FROM FOXL2 (SEQ ID NO: 35) | TGTAGAATTGTGTGGT (SEQ ID NO: 598) |
| 151 | GENOMIC REGION DOWNSTREAM FROM FOXL2 (SEQ ID NO: 35) | AAAATTCGAGGTCGGG (SEQ ID NO: 599) |
| 152 | GENOMIC REGION DOWNSTREAM FROM FOXL2 (SEQ ID NO: 35) | AAAATTTGAGGTTGGG (SEQ ID NO: 600) |
| 153 | GENOMIC REGION DOWNSTREAM FROM FOXL2 (SEQ ID NO: 35) | TTTTCGCGGTTCGGAG (SEQ ID NO: 601) |
| 154 | GENOMIC REGION DOWNSTREAM FROM FOXL2 (SEQ ID NO: 35) | TTTGTGGTTTGGAGAA (SEQ ID NO: 602) |
| 155 | GENOMIC REGION DOWNSTREAM FROM FOXL2 (SEQ ID NO: 35) | AATAGGCGATGTACGG (SEQ ID NO: 603) |
| 156 | GENOMIC REGION DOWNSTREAM FROM FOXL2 (SEQ ID NO: 35) | TAGGTGATGTATGGGT (SEQ ID NO: 604) |
| 157 | GENOMIC REGION DOWNSTREAM FROM FOXL2 (SEQ ID NO: 35) | TTGGTCGGTTAATCGA (SEQ ID NO: 605) |
| 158 | GENOMIC REGION DOWNSTREAM FROM FOXL2 (SEQ ID NO: 35) | TTTGGTTGGTTAATTGA (SEQ ID NO: 606) |
| 159 | GENOMIC REGION DOWNSTREAM FROM FOXL2 (SEQ ID NO: 35) | TAGCGGTCGCGAAAAT (SEQ ID NO: 607) |
| 160 | GENOMIC REGION DOWNSTREAM FROM FOXL2 (SEQ ID NO: 35) | AGTTTAGTGGTTGTGA (SEQ ID NO: 608) |
| 161 | CMYA3 (SEQ ID NO: 13) | TTTACGCGGGGTTTTA (SEQ ID NO: 609) |
| 162 | CMYA3 (SEQ ID NO: 13) | TTTATGTGGGGTTTTAG (SEQ ID NO: 610) |
| 163 | CMYA3 (SEQ ID NO: 13) | TTACGTCGTTATTAGGT (SEQ ID NO: 611) |
| 164 | CMYA3 (SEQ ID NO: 13) | TTTTATGTTGTTATTAGGT (SEQ ID NO: 612) |
| 165 | CMYA3 (SEQ ID NO: 13) | TATTTGGACGTCGGGT (SEQ ID NO: 613) |
| 166 | CMYA3 (SEQ ID NO: 13) | TATTTGGATGTTGGGT (SEQ ID NO: 614) |
| 167 | CMYA3 (SEQ ID NO: 13) | TTTGTCGGAAAGCGGA (SEQ ID NO: 615) |
| 168 | CMYA3 (SEQ ID NO: 13) | TTTGTTGGAAAGTGGA (SEQ ID NO: 616) |
| 169 | ORC4L (SEQ ID NO: 36) | ATTCGGATCGTTACGT (SEQ ID NO: 617) |
| 170 | ORC4L (SEQ ID NO: 36) | ATTTGGATTGTTATGTTT (SEQ ID NO: 618) |
| 171 | ORC4L (SEQ ID NO: 36) | ATAAGACGGAGTTCGT (SEQ ID NO: 619) |
| 172 | ORC4L (SEQ ID NO: 36) | AAGATGGAGTTTGTTTG (SEQ ID NO: 620) |
| 173 | ORC4L (SEQ ID NO: 36) | TTGCGTTATCGACGTT (SEQ ID NO: 621) |
| 174 | ORC4L (SEQ ID NO: 36) | ATTTTGTGTTATTGATGT (SEQ ID NO: 622) |

(continued)

| *No:* | *Gene* | *Oligo*: |
|---|---|---|
| 175 | ORC4L (SEQ ID NO: 36) | GAGTAACGCGTGTGAT (SEQ ID NO: 623) |
| 176 | ORC4L (SEQ ID NO: 36) | TATGAGTAATGTGTGTG (SEQ ID NO: 624) |
| 177 | ABHD9 (SEQ ID NO: 37) | TATTTGGGCGCGATAG (SEQ ID NO: 625) |
| 178 | ABHD9 (SEQ ID NO: 37) | ATTTGGGTGTGATAGG (SEQ ID NO: 626) |
| 179 | ABHD9 (SEQ ID NO: 37) | GTGGGACGCGTTGAAG (SEQ ID NO: 627) |
| 180 | ABHD9 (SEQ ID NO: 37) | TGTGGGATGTGTTGAA (SEQ ID NO: 628) |
| 181 | ABHD9 (SEQ ID NO: 37) | GGCGGTTTCGATAGAA (SEQ ID NO: 629) |
| 182 | ABHD9 (SEQ ID NO: 37) | GGGTGGTTTTGATAGA (SEQ ID NO: 630) |
| 183 | CCND2 (SEQ ID NO: 1) | ATAAGTCGTTCGAGGT (SEQ ID NO: 631) |
| 184 | CCND2 (SEQ ID NO: 1) | AAGTTGTTTGAGGTGT (SEQ ID NO: 632) |
| 185 | CCND2 (SEQ ID NO: 1) | TAGCGGTTACGTAGGA (SEQ ID NO: 633) |
| 186 | CCND2 (SEQ ID NO: 1) | AGTGGTTATGTAGGAAA (SEQ ID NO: 634) |
| 187 | CCND2 (SEQ ID NO: 1) | TACGTGTTTTAACGTAT (SEQ ID NO: 635) |
| 188 | CCND2 (SEQ ID NO: 1) | TGATATGTGTTTTAATGTA (SEQ ID NO: 636) |
| 189 | CCND2 (SEQ ID NO: 1) | TTAGGGTCGTCGTAGGT (SEQ ID NO: 637) |
| 190 | CCND2 (SEQ ID NO: 1) | TTAGGGTTGTTGTAGGT (SEQ ID NO: 638) |
| 191 | CCND2 (SEQ ID NO: 1) | TTAGTACGGTCGGTTT (SEQ ID NO: 639) |
| 192 | CCND2 (SEQ ID NO: 1) | GTTAGTATGGTTGGTTT (SEQ ID NO: 640) |
| 193 | CDKN2A (SEQ ID NO: 2) | TAGGTATCGCGTACGT (SEQ ID NO: 641) |
| 194 | CDKN2A (SEQ ID NO: 2) | TTAGGTATTGTGTATGTT (SEQ ID NO: 642) |
| 195 | CDKN2A (SEQ ID NO: 2) | TTCGCGTCGTGGAGTA (SEQ ID NO: 643) |
| 196 | CDKN2A (SEQ ID NO: 2) | TTTTGTGTTGTGGAGTA (SEQ ID NO: 644) |
| 197 | CDKN2A (SEQ ID NO: 2) | TAGTCGCGCGTAGGTA (SEQ ID NO: 645) |
| 198 | CDKN2A (SEQ ID NO: 2) | TAGTTGTGTGTAGGTAT (SEQ ID NO: 646) |
| 199 | CDKN2A (SEQ ID NO: 2) | TAGGTATCGTGCGATA (SEQ ID NO: 647) |
| 200 | CDKN2A (SEQ ID NO: 2) | GTAGGTATTGTGTGATAT (SEQ ID NO: 648) |
| 201 | CD44 (SEQ ID NO: 3) | TAGGTTCGGTTCGTTAT (SEQ ID NO: 649) |
| 202 | CD44 (SEQ ID NO: 3) | TAGGTTTGGTTTGTTATT (SEQ ID NO: 650) |
| 203 | CD44 (SEQ ID NO: 3) | GTTTCGCGTTTAGGGA (SEQ ID NO: 651) |
| 204 | CD44 (SEQ ID NO: 3) | GTTTTGTGTTTAGGGAT (SEQ ID NO: 652) |
| 205 | CD44 (SEQ ID NO: 3) | GTTCGTTTCGGATATTA (SEQ ID NO: 653) |
| 206 | CD44 (SEQ ID NO: 3) | TTTGTTTTGGATATTATGG (SEQ ID NO: 654) |
| 207 | CD44 (SEQ ID NO: 3) | TTTGGCGTAGATCGGT (SEQ ID NO: 655) |
| 208 | CD44 (SEQ ID NO: 3) | TTTGGTGTAGATTGGT (SEQ ID NO: 656) |
| 209 | EDNRB1 (SEQ ID NO: 4) | TTCGTTTTTCGGGAAG (SEQ ID NO: 657) |
| 210 | EDNRB1 (SEQ ID NO: 4) | TTTGTTTTTTGGGAAGG (SEQ ID NO: 658) |
| 211 | EDNRB1 (SEQ ID NO: 4) | TAGAGTCGGATTCGTT (SEQ ID NO: 659) |
| 212 | EDNRB1 (SEQ ID NO: 4) | AGTTGGATTTGTTTGTA (SEQ ID NO: 660) |

(continued)

| *No:* | *Gene* | *Oligo*: |
|---|---|---|
| 213 | EDNRB1 (SEQ ID NO: 4) | GTATTTTCGTAGCGTT (SEQ ID NO: 661) |
| 214 | EDNRB1 (SEQ ID NO: 4) | TGGTATTTTTGTAGTGTT (SEQ ID NO: 662) |
| 215 | EDNRB1 (SEQ ID NO: 4) | ATTTCGAGTAAACGGT (SEQ ID NO: 663) |
| 216 | EDNRB1 (SEQ ID NO: 4) | TTTGAGTAAATGGTGGA (SEQ ID NO: 664) |
| 217 | ELK1 (SEQ ID NO: 5) | TGTCGTACGTTATGTT (SEQ ID NO: 665) |
| 218 | ELK1 (SEQ ID NO: 5) | GGTGTTGTATGTTATGT (SEQ ID NO: 666) |
| 219 | ELK1 (SEQ ID NO: 5) | TGGGCGTAGTAGTCGG (SEQ ID NO: 667) |
| 220 | ELK1 (SEQ ID NO: 5) | ATGGGTGTAGTAGTTGG (SEQ ID NO: 668) |
| 221 | ELK1 (SEQ ID NO: 5) | ATTGGGTTTCGCGTAGG (SEQ ID NO: 669) |
| 222 | ELK1 (SEQ ID NO: 5) | ATTGGGTTTTGTGTAGG (SEQ ID NO: 670) |
| 223 | ELK1 (SEQ ID NO: 5) | TTGATTGGCGGACGAG (SEQ ID NO: 671) |
| 224 | ELK1 (SEQ ID NO: 5) | TTGATTGGTGGATGAG (SEQ ID NO: 672) |
| 225 | FOS (SEQ ID NO: 6) | TACGGATTTGGTCGTTT (SEQ ID NO: 673) |
| 226 | FOS (SEQ ID NO: 6) | TATGGATTTGGTTGTTT (SEQ ID NO: 674) |
| 227 | FOS (SEQ ID NO: 6) | TTCGATTAGTTCGGAT (SEQ ID NO: 675) |
| 228 | FOS (SEQ ID NO: 6) | TATTTTGATTAGTTTGGAT (SEQ ID NO: 676) |
| 229 | FOS (SEQ ID NO: 6) | TTTCGTGGTTTTATCGTA (SEQ ID NO: 677) |
| 230 | FOS (SEQ ID NO: 6) | TTTTGTGGTTTTATTGTA (SEQ ID NO: 678) |
| 231 | FOS (SEQ ID NO: 6) | GTCGAGCGTAGAGTAT (SEQ ID NO: 679) |
| 232 | FOS (SEQ ID NO: 6) | TAGGAGGTTGAGTGTA (SEQ ID NO: 680) |
| 233 | GSTP1 (SEQ ID NO: 7) | GTCGGTCGTAGAGGGG (SEQ ID NO: 681) |
| 234 | GSTP1 (SEQ ID NO: 7) | GTTGGTTGTAGAGGGG (SEQ ID NO: 682) |
| 235 | GSTP1 (SEQ ID NO: 7) | TTCGCGGTTTTCGAGT (SEQ ID NO: 683) |
| 236 | GSTP1 (SEQ ID NO: 7) | TTTGTGGTTTTTGAGTT (SEQ ID NO: 684) |
| 237 | GSTP1 (SEQ ID NO: 7) | GTCGCGCGTATTTATT (SEQ ID NO: 685) |
| 238 | GSTP1 (SEQ ID NO: 7) | GGGTTGTGTGTATTTAT (SEQ ID NO: 686) |
| 239 | GSTP1 (SEQ ID NO: 7) | GAGTCGTCGCGTAGTT (SEQ ID NO: 687) |
| 240 | GSTP1 (SEQ ID NO: 7) | GGAGTTGTTGTGTAGTT (SEQ ID NO: 688) |
| 241 | CD37 (SEQ ID NO: 38) | ATCGAGAGCGTTATGA (SEQ ID NO: 689) |
| 242 | CD37 (SEQ ID NO: 38) | AGGAATATTGAGAGTGT (SEQ ID NO: 690) |
| 243 | CD37 (SEQ ID NO: 38) | TATCGAGCGAGTCGGT (SEQ ID NO: 691) |
| 244 | CD37 (SEQ ID NO: 38) | TATTGAGTGAGTTGGTT (SEQ ID NO: 692) |
| 245 | CD37 (SEQ ID NO: 38) | TTAGCGTACGTGACGG (SEQ ID NO: 693) |
| 246 | CD37 (SEQ ID NO: 38) | AGTGTATGTGATGGGG (SEQ ID NO: 694) |
| 247 | CD37 (SEQ ID NO: 38) | GGGTACGTAGTTACGT (SEQ ID NO: 695) |
| 248 | CD37 (SEQ ID NO: 38) | TATGTAGTTATGTGGGT (SEQ ID NO: 696) |
| 249 | GRN (SEQ ID NO: 39) | TAGCGCGATGATTCGT (SEQ ID NO: 697) |
| 250 | GRN (SEQ ID NO: 39) | AGTGTGATGATTTGTTT (SEQ ID NO: 698) |

(continued)

| ***No:*** | ***Gene*** | ***Oligo***: |
|---|---|---|
| 251 | GRN (SEQ ID NO: 39) | TAATCGGGTAGCGTTT (SEQ ID NO: 699) |
| 252 | GRN (SEQ ID NO: 39) | TAGTAATTGGGTAGTGT (SEQ ID NO: 700) |
| 253 | GRN (SEQ ID NO: 39) | TTCGATTTCGCGGTTT (SEQ ID NO: 701) |
| 254 | GRN (SEQ ID NO: 39) | GTTTGATTTTGTGGTTT (SEQ ID NO: 702) |
| 255 | GRN (SEQ ID NO: 39) | TTTAACGGGGCGTTAT (SEQ ID NO: 703) |
| 256 | GRN (SEQ ID NO: 39) | AATGGGGTGTTATTGT (SEQ ID NO: 704) |
| 257 | EPAS1 (SEQ ID NO: 40) | TTCGGCGTTATTCGAG (SEQ ID NO: 705) |
| 258 | EPAS1 (SEQ ID NO: 40) | GGTTTGGTGTTATTTGA (SEQ ID NO: 706) |
| 259 | EPAS1 (SEQ ID NO: 40) | TATTCGTGCGGTTTTA (SEQ ID NO: 707) |
| 260 | EPAS1 (SEQ ID NO: 40) | ATTTGTGTGGTTTTAGT (SEQ ID NO: 708) |
| 261 | EPAS1 (SEQ ID NO: 40) | GAATTTAACGCGCGGT (SEQ ID NO: 709) |
| 262 | EPAS1 (SEQ ID NO: 40) | GGAATTTAATGTGTGGT (SEQ ID NO: 710) |
| 263 | EPAS1 (SEQ ID NO: 40) | TTCGCGAGTTTTTCGG (SEQ ID NO: 711) |
| 264 | EPAS1 (SEQ ID NO: 40) | TTTGTGAGTTTTTTGGTA (SEQ ID NO: 712) |
| 265 | NOTCH1 (SEQ ID NO: 41) | TTTACGGGCGGGAGTT (SEQ ID NO: 713) |
| 266 | NOTCH1 (SEQ ID NO: 41) | TTTTATGGGTGGGAGT (SEQ ID NO: 714) |
| 267 | NOTCH1 (SEQ ID NO: 41) | TAGCGGGCGAGTAGTT (SEQ ID NO: 715) |
| 268 | NOTCH1 (SEQ ID NO: 41) | TAGTGGGTGAGTAGTT (SEQ ID NO: 716) |
| 269 | NOTCH1 (SEQ ID NO: 41) | AGGCGGTTTCGATTTT (SEQ ID NO: 717) |
| 270 | NOTCH1 (SEQ ID NO: 41) | TGGAGGTGGTTTTGAT (SEQ ID NO: 718) |
| 271 | NOTCH1 (SEQ ID NO: 41) | ATTTCGGCGGTTGGAT (SEQ ID NO: 719) |
| 272 | NOTCH1 (SEQ ID NO: 41) | AGGTAATTTTGGTGGTT (SEQ ID NO: 720) |
| 273 | MLLT3 (SEQ ID NO: 42) | TAATTCGGTTAGATTTTCGG (SEQ ID NO: 721) |
| 274 | MLLT3 (SEQ ID NO: 42) | TAATTTGGTTAGATTTTTGG (SEQ ID NO: 722) |
| 275 | MLLT3 (SEQ ID NO: 42) | TTTAGAGTCGCGTTTT (SEQ ID NO: 723) |
| 276 | MLLT3 (SEQ ID NO: 42) | TAGAGTTGTGTTTTTGT (SEQ ID NO: 724) |
| 277 | MLLT3 (SEQ ID NO: 42) | TAGAATAGCGCGGTTA (SEQ ID NO: 725) |
| 278 | MLLT3 (SEQ ID NO: 42) | GATAGAATAGTGTGGTTA (SEQ ID NO: 726) |
| 279 | SOLH (SEQ ID NO: 43) | TAGGGGACGTGTACGA (SEQ ID NO: 727) |
| 280 | SOLH (SEQ ID NO: 43) | TAGGGGATGTGTATGA (SEQ ID NO: 728) |
| 281 | SOLH (SEQ ID NO: 43) | GACGGAACGTATGTTT (SEQ ID NO: 729) |
| 282 | SOLH (SEQ ID NO: 43) | TGGGGATGGAATGTAT (SEQ ID NO: 730) |
| 283 | SOLH (SEQ ID NO: 43) | ATTCGTGGGGACGGAA (SEQ ID NO: 731) |
| 284 | SOLH (SEQ ID NO: 43) | ATTTGTGGGGATGGAA (SEQ ID NO: 732) |
| 285 | SEQ ID NO: 44 (SEQ ID NO: 44) | TAGGGTTCGTTCGTATT (SEQ ID NO: 733) |
| 286 | SEQ ID NO: 44 (SEQ ID NO: 44) | TTTAGGGTTTGTTTGTAT (SEQ ID NO: 734) |
| 287 | SEQ ID NO: 44 (SEQ ID NO: 44) | TTACGATTTTCGGGGT (SEQ ID NO: 735) |
| 288 | SEQ ID NO: 44 (SEQ ID NO: 44) | TTTATGATTTTTGGGGT (SEQ ID NO: 736) |

(continued)

| No: | Gene | Oligo: |
|---|---|---|
| 289 | SEQ ID NO: 44 (SEQ ID NO: 44) | AAGTAGACGTCGGAGA (SEQ ID NO: 737) |
| 290 | SEQ ID NO: 44 (SEQ ID NO: 44) | TAGATGTTGGAGAGGG (SEQ ID NO: 738) |
| 291 | SEQ ID NO: 44 (SEQ ID NO: 44) | TGTCGTTACGTTTAGG (SEQ ID NO: 739) |
| 292 | SEQ ID NO: 44 (SEQ ID NO: 44) | GTTGTTATGTTTAGGGT (SEQ ID NO: 740) |
| 293 | Q8N365 (SEQ ID NO: 45) | AGATTCGGAGAGACGG (SEQ ID NO: 741) |
| 294 | Q8N365 (SEQ ID NO: 45) | TAGATTTGGAGAGATGG (SEQ ID NO: 742) |
| 295 | Q8N365 (SEQ ID NO: 45) | AGATCGGCGTAGGGAT (SEQ ID NO: 743) |
| 296 | Q8N365 (SEQ ID NO: 45) | AGATTGGTGTAGGGAT (SEQ ID NO: 744) |
| 297 | Q8N365 (SEQ ID NO: 45) | TACGTGTTATCGGCGA (SEQ ID NO: 745) |
| 298 | Q8N365 (SEQ ID NO: 45) | TATGTGTTATTGGTGATA (SEQ ID NO: 746) |
| 299 | Q8N365 (SEQ ID NO: 45) | TACGTGTCGGGTCGTA (SEQ ID NO: 747) |
| 300 | Q8N365 (SEQ ID NO: 45) | GTATGTGTTGGGTTGTA (SEQ ID NO: 748) |
| 301 | Q9NWV0 (SEQ ID NO: 46) | GTCGCGTGGATACGTG (SEQ ID NO: 749) |
| 302 | Q9NWV0 (SEQ ID NO: 46) | GGTTGTGTGGATATGT (SEQ ID NO: 750) |
| 303 | Q9NWV0 (SEQ ID NO: 46) | TTGGCGATTTTTACGA (SEQ ID NO: 751) |
| 304 | Q9NWV0 (SEQ ID NO: 46) | TTGGTGATTTTTATGAGA (SEQ ID NO: 752) |
| 305 | Q9NWV0 (SEQ ID NO: 46) | TGTCGTAGCGTTATGT (SEQ ID NO: 753) |
| 306 | Q9NWV0 (SEQ ID NO: 46) | AGGTGTTGTAGTGTTAT (SEQ ID NO: 754) |
| 307 | SEQ ID NO: 47 (SEQ ID NO: 47) | GTAACGCGTTTGGTTT (SEQ ID NO: 755) |
| 308 | SEQ ID NO: 47 (SEQ ID NO: 47) | TGGTAATGTGTTTGGT (SEQ ID NO: 756) |
| 309 | SEQ ID NO: 47 (SEQ ID NO: 47) | TTCGAGCGTTTTACGT (SEQ ID NO: 757) |
| 310 | SEQ ID NO: 47 (SEQ ID NO: 47) | TTTTGAGTGTTTTATGTT (SEQ ID NO: 758) |
| 311 | SEQ ID NO: 47 (SEQ ID NO: 47) | TTACGTGGGAGCGTTT (SEQ ID NO: 759) |
| 312 | SEQ ID NO: 47 (SEQ ID NO: 47) | TTATGTGGGAGTGTTT (SEQ ID NO: 760) |
| 313 | SEQ ID NO: 47 (SEQ ID NO: 47) | TAGTTTTACGCGGGTA (SEQ ID NO: 761) |
| 314 | SEQ ID NO: 47 (SEQ ID NO: 47) | AGTTTTATGTGGGTATG (SEQ ID NO: 762) |
| 315 | H2AFY2 (SEQ ID NO: 48) | ATGAAAGGCGCGAGAA (SEQ ID NO: 763) |
| 316 | H2AFY2 (SEQ ID NO: 48) | ATGAAAGGTGTGAGAA (SEQ ID NO: 764) |
| 317 | H2AFY2 (SEQ ID NO: 48) | GAATCGTGGTTTCGTT (SEQ ID NO: 765) |
| 318 | H2AFY2 (SEQ ID NO: 48) | GGAATTGTGGTTTTGT (SEQ ID NO: 766) |
| 319 | H2AFY2 (SEQ ID NO: 48) | TAGTTTATCGCGGTAA (SEQ ID NO: 767) |
| 320 | H2AFY2 (SEQ ID NO: 48) | TTTATTGTGGTAAATGGT (SEQ ID NO: 768) |
| 321 | RHOC (SEQ ID NO: 49) | TGCGGTTCGAAGATTA (SEQ ID NO: 769) |
| 322 | RHOC (SEQ ID NO: 49) | GGTGTGGTTTGAAGAT (SEQ ID NO: 770) |
| 323 | RHOC (SEQ ID NO: 49) | GAACGCGTTTTAGCGT (SEQ ID NO: 771) |
| 324 | RHOC (SEQ ID NO: 49) | GGAATGTGTTTTAGTGT (SEQ ID NO: 772) |
| 325 | RHOC (SEQ ID NO: 49) | TTTTAGCGTCGGGGAT (SEQ ID NO: 773) |
| 326 | RHOC (SEQ ID NO: 49) | TTTTAGTGTTGGGGAT (SEQ ID NO: 774) |

(continued)

| *No:* | *Gene* | *Oligo*: |
|---|---|---|
| 327 | NR2E1 (SEQ ID NO: 50) | TAGTCGTATTAGCGGT (SEQ ID NO: 775) |
| 328 | NR2E1 (SEQ ID NO: 50) | TAGTTGTATTAGTGGTTT (SEQ ID NO: 776) |
| 329 | NR2E1 (SEQ ID NO: 50) | TGCGTTTTATTCGCGG (SEQ ID NO: 777) |
| 330 | NR2E1 (SEQ ID NO: 50) | TGTGTTTTATTTGTGGT (SEQ ID NO: 778) |
| 331 | NR2E1 (SEQ ID NO: 50) | TTTCGAAGTTTCGCGG (SEQ ID NO: 779) |
| 332 | NR2E1 (SEQ ID NO: 50) | TTTGAAGTTTTGTGGG (SEQ ID NO: 780) |
| 333 | NR2E1 (SEQ ID NO: 50) | TAGCGCGAATCGTTTA (SEQ ID NO: 781) |
| 334 | NR2E1 (SEQ ID NO: 50) | AGTGTGAATTGTTTAGT (SEQ ID NO: 782) |
| 335 | KBTBD6 (SEQ ID NO: 51) | AGACGTTTCGCGTTTT (SEQ ID NO: 783) |
| 336 | KBTBD6 (SEQ ID NO: 51) | GAAGATGTTTTGTGTTT (SEQ ID NO: 784) |
| 337 | KBTBD6 (SEQ ID NO: 51) | TTTCGGGAAGACGTTT (SEQ ID NO: 785) |
| 338 | KBTBD6 (SEQ ID NO: 51) | AGTTTTGGGAAGATGT (SEQ ID NO: 786) |
| 339 | KBTBD6 (SEQ ID NO: 51) | TATTAGCGTTCGTCGT (SEQ ID NO: 787) |
| 340 | KBTBD6 (SEQ ID NO: 51) | GTTATTAGTGTTTGTTGT (SEQ ID NO: 788) |
| 341 | TRPM4 (SEQ ID NO: 52) | TAGGTGAGCGTCGGAT (SEQ ID NO: 789) |
| 342 | TRPM4 (SEQ ID NO: 52) | TAGGTGAGTGTTGGAT (SEQ ID NO: 790) |
| 343 | TRPM4 (SEQ ID NO: 52) | AGTAGAGTCGGCGGAG (SEQ ID NO: 791) |
| 344 | TRPM4 (SEQ ID NO: 52) | AGTAGAGTTGGTGGAG (SEQ ID NO: 792) |
| 345 | TCEB3BP1 (SEQ ID NO: 53) | GACGTTAGCGAGTATT (SEQ ID NO: 793) |
| 346 | TCEB3BP1 (SEQ ID NO: 53) | AGGGATGTTAGTGAGT (SEQ ID NO: 794) |
| 347 | TCEB3BP1 (SEQ ID NO: 53) | TGTCGGGTCGAGTAGT (SEQ ID NO: 795) |
| 348 | TCEB3BP1 (SEQ ID NO: 53) | TGTTGGGTTGAGTAGT (SEQ ID NO: 796) |
| 349 | Q8NCX8 (SEQ ID NO: 54) | TTACGGCGGGTTTTTA (SEQ ID NO: 797) |
| 350 | Q8NCX8 (SEQ ID NO: 54) | TTATGGTGGGTTTTTATT (SEQ ID NO: 798) |
| 351 | Q8NCX8 (SEQ ID NO: 54) | TTCGGTTTATACGATTT (SEQ ID NO: 799) |
| 352 | Q8NCX8 (SEQ ID NO: 54) | ATTTGGTTTATATGATTTTT (SEQ ID NO: 800) |
| 353 | Q8NCX8 (SEQ ID NO: 54) | TTACGTCGATTATCGG (SEQ ID NO: 801) |
| 354 | Q8NCX8 (SEQ ID NO: 54) | TATGTTGATTATTGGGGT (SEQ ID NO: 802) |
| 355 | SEQ ID NO: 55 (SEQ ID NO: 55) | AATTTACGCGGGTGTT (SEQ ID NO: 803) |
| 356 | SEQ ID NO: 55 (SEQ ID NO: 55) | GGAATTTATGTGGGTG (SEQ ID NO: 804) |
| 357 | SEQ ID NO: 55 (SEQ ID NO: 55) | TAGTTCGTTTCGGTTA (SEQ ID NO: 805) |
| 358 | SEQ ID NO: 55 (SEQ ID NO: 55) | AGTTTGTTTTGGTTAGT (SEQ ID NO: 806) |
| 359 | SEQ ID NO: 55 (SEQ ID NO: 55) | GTCGTGTACGAATTCGT (SEQ ID NO: 807) |
| 360 | SEQ ID NO: 55 (SEQ ID NO: 55) | GTTGTGTATGAATTTGTT (SEQ ID NO: 808) |
| 361 | SEQ ID NO: 55 (SEQ ID NO: 55) | AGCGTTTAAGTCGCGG (SEQ ID NO: 809) |
| 362 | SEQ ID NO: 55 (SEQ ID NO: 55) | TAGTGTTTAAGTTGTGG (SEQ ID NO: 810) |
| 363 | SNAPC2 (SEQ ID NO: 56) | TTACGATTTCGGTGTT (SEQ ID NO: 811) |
| 364 | SNAPC2 (SEQ ID NO: 56) | TAGAGTTATGATTTTGGT (SEQ ID NO: 812) |

(continued)

| No: | Gene | Oligo: |
|---|---|---|
| 365 | SNAPC2 (SEQ ID NO: 56) | AGGCGTGCGTCGATAT (SEQ ID NO: 813) |
| 366 | SNAPC2 (SEQ ID NO: 56) | AGGTGTGTGTTGATATT (SEQ ID NO: 814) |
| 367 | SNAPC2 (SEQ ID NO: 56) | GTAGCGTCGAGGGTTT (SEQ ID NO: 815) |
| 368 | SNAPC2 (SEQ ID NO: 56) | GTAGTGTTGAGGGTTT (SEQ ID NO: 816) |
| 369 | SNAPC2 (SEQ ID NO: 56) | ATACGTTGACGTAGTT (SEQ ID NO: 817) |
| 370 | SNAPC2 (SEQ ID NO: 56) | TTGTATATGTTGATGTAGT (SEQ ID NO: 818) |
| 371 | PTPRN2 (SEQ ID NO: 57) | GACGTAGTTCGTACGT (SEQ ID NO: 819) |
| 372 | PTPRN2 (SEQ ID NO: 57) | GGATGTAGTTTGTATGT (SEQ ID NO: 820) |
| 373 | PTPRN2 (SEQ ID NO: 57) | TTAGTCGTTTCGAGAT (SEQ ID NO: 821) |
| 374 | PTPRN2 (SEQ ID NO: 57) | GTTTTAGTTGTTTTGAGA (SEQ ID NO: 822) |
| 375 | PTPRN2 (SEQ ID NO: 57) | TTACGCGTATCGGGAT (SEQ ID NO: 823) |
| 376 | PTPRN2 (SEQ ID NO: 57) | TATGTGTATTGGGATTTA (SEQ ID NO: 824) |
| 377 | PTPRN2 (SEQ ID NO: 57) | TTCGCGTTTCGTAAGA (SEQ ID NO: 825) |
| 378 | PTPRN2 (SEQ ID NO: 57) | TTTGTGTTTTGTAAGATAT (SEQ ID NO: 826) |
| 379 | WDFY3 (SEQ ID NO: 58) | TATTGAGTCGGTCGTA (SEQ ID NO: 827) |
| 380 | WDFY3 (SEQ ID NO: 58) | ATTGAGTTGGTTGTAGA (SEQ ID NO: 828) |
| 381 | WDFY3 (SEQ ID NO: 58) | TAGATAGCGTCGTTGG (SEQ ID NO: 829) |
| 382 | WDFY3 (SEQ ID NO: 58) | TAGATAGTGTTGTTGGA (SEQ ID NO: 830) |
| 383 | ZNF566 (SEQ ID NO: 59) | ATAAAATACGACGTTGA (SEQ ID NO: 831) |
| 384 | ZNF566 (SEQ ID NO: 59) | ATAAAATATGATGTTGAATT (SEQ ID NO: 832) |
| 385 | ZNF566 (SEQ ID NO: 59) | AGCGTTTTTTACGAAT (SEQ ID NO: 833) |
| 386 | ZNF566 (SEQ ID NO: 59) | TAGTGTTTTTTATGAATGA (SEQ ID NO: 834) |
| 387 | Q9NP73 (SEQ ID NO: 60) | TATTACGGATTTTCGTT (SEQ ID NO: 835) |
| 388 | Q9NP73 (SEQ ID NO: 60) | GTTATTATGGATTTTTGTT (SEQ ID NO: 836) |
| 389 | Q9NP73 (SEQ ID NO: 60) | TGCGTTATATTCGGTT (SEQ ID NO: 837) |
| 390 | Q9NP73 (SEQ ID NO: 60) | AGTTGTGTTATATTTGGT (SEQ ID NO: 838) |
| 391 | Q9NP73 (SEQ ID NO: 60) | AATTCGCGTTATGAAG (SEQ ID NO: 839) |
| 392 | Q9NP73 (SEQ ID NO: 60) | TTGAGGAATTTGTGTTAT (SEQ ID NO: 840) |
| 393 | Q9NP73 (SEQ ID NO: 60) | GTCGGCGTTCGATAGT (SEQ ID NO: 841) |
| 394 | Q9NP73 (SEQ ID NO: 60) | TGTTGGTGTTTGATAGT (SEQ ID NO: 842) |
| 395 | SEQ ID NO: 61 (SEQ ID NO: 61) | AGAATTCGTAAACGGG (SEQ ID NO: 843) |
| 396 | SEQ ID NO: 61 (SEQ ID NO: 61) | ATTTGTAAATGGGGGT (SEQ ID NO: 844) |
| 397 | SEQ ID NO: 61 (SEQ ID NO: 61) | TTCGGTTATCGACGGT (SEQ ID NO: 845) |
| 398 | SEQ ID NO: 61 (SEQ ID NO: 61) | TTTGGTTATTGATGGTT (SEQ ID NO: 846) |
| 399 | Q86SP6 (SEQ ID NO: 62) | ATCGTGAGCGTAGCGT (SEQ ID NO: 847) |
| 400 | Q86SP6 (SEQ ID NO: 62) | ATTGTGAGTGTAGTGTA (SEQ ID NO: 848) |
| 401 | Q86SP6 (SEQ ID NO: 62) | TAGGCGTGAGAATCGG (SEQ ID NO: 849) |
| 402 | Q86SP6 (SEQ ID NO: 62) | TAGGTGTGAGAATTGG (SEQ ID NO: 850) |

(continued)

| *No:* | *Gene* | *Oligo*: |
|---|---|---|
| 403 | Q86SP6 (SEQ ID NO: 62) | ATCGTGTTCGGATCGG (SEQ ID NO: 851) |
| 404 | Q86SP6 (SEQ ID NO: 62) | TATTGTGTTTGGATTGG (SEQ ID NO: 852) |
| 405 | Q86SP6 (SEQ ID NO: 62) | AGGTTCGAGTTTGCGG (SEQ ID NO: 853) |
| 406 | Q86SP6 (SEQ ID NO: 62) | TAGGTTTGAGTTTGTGG (SEQ ID NO: 854) |
| 407 | Q86SP6 (SEQ ID NO: 62) | TATAAAGCGCGAGTGT (SEQ ID NO: 855) |
| 408 | Q86SP6 (SEQ ID NO: 62) | TATAAAGTGTGAGTGTG (SEQ ID NO: 856) |
| 409 | RARB (SEQ ID NO: 8) | ATGTCGAGAACGCGAG (SEQ ID NO: 857) |
| 410 | RARB (SEQ ID NO: 8) | GGATGTTGAGAATGTGA (SEQ ID NO: 858) |
| 411 | RARB (SEQ ID NO: 8) | AGCGATTCGAGTAGGG (SEQ ID NO: 859) |
| 412 | RARB (SEQ ID NO: 8) | AGTGATTTGAGTAGGG (SEQ ID NO: 860) |
| 413 | RARB (SEQ ID NO: 8) | TATCGTCGGGGTAGGA (SEQ ID NO: 861) |
| 414 | RARB (SEQ ID NO: 8) | TATTGTTGGGGTAGGA (SEQ ID NO: 862) |
| 415 | RARB (SEQ ID NO: 8) | AACGTATTCGGAAGGT (SEQ ID NO: 863) |
| 416 | RARB (SEQ ID NO: 8) | GAATGTATTTGGAAGGT (SEQ ID NO: 864) |
| 417 | PTGS2 (SEQ ID NO: 9) | AGCGTTTTCGAGAGTT (SEQ ID NO: 865) |
| 418 | PTGS2 (SEQ ID NO: 9) | GAGTGTTTTTGAGAGTT (SEQ ID NO: 866) |
| 419 | PTGS2 (SEQ ID NO: 9) | TTACGTCGGGATAGAT (SEQ ID NO: 867) |
| 420 | PTGS2 (SEQ ID NO: 9) | GGAAGTTATGTTGGGA (SEQ ID NO: 868) |
| 421 | PTGS2 (SEQ ID NO: 9) | TATCGTTTTAGGCGTA (SEQ ID NO: 869) |
| 422 | PTGS2 (SEQ ID NO: 9) | TTTATTGTTTTAGGTGTAT (SEQ ID NO: 870) |
| 423 | RASSF1 (SEQ ID NO: 10) | TACGGGTATTTTCGCGT (SEQ ID NO: 871) |
| 424 | RASSF1 (SEQ ID NO: 10) | ATATGGGTATTTTTGTGT (SEQ ID NO: 872) |
| 425 | RASSF1 (SEQ ID NO: 10) | AGAGCGCGTTTAGTTT (SEQ ID NO: 873) |
| 426 | RASSF1 (SEQ ID NO: 10) | GAGAGTGTGTTTAGTTT (SEQ ID NO: 874) |
| 427 | ESR2 (SEQ ID NO: 11) | TAGGAACGTTCGACGT (SEQ ID NO: 875) |
| 428 | ESR2 (SEQ ID NO: 11) | TTTAGGAATGTTTGATGT (SEQ ID NO: 876) |
| 429 | ESR2 (SEQ ID NO: 11) | ATGGCGTTTTTCGTAG (SEQ ID NO: 877) |
| 430 | ESR2 (SEQ ID NO: 11) | TAGATGGTGTTTTTTGT (SEQ ID NO: 878) |
| 431 | ESR2 (SEQ ID NO: 11) | ATAAGCGATTTAACGAT (SEQ ID NO: 879) |
| 432 | ESR2 (SEQ ID NO: 11) | AGTGATTTAATGATAAGTT (SEQ ID NO: 880) |
| 433 | ESR2 (SEQ ID NO: 11) | ATTTCGAGGATTACGT (SEQ ID NO: 881) |
| 434 | ESR2 (SEO ID NO: 11) | ATATTTTGAGGATTATGTT (SEQ ID NO: 882) |
| 435 | DRG1 (SEQ ID NO: 12) | TAGTCGTTAAAACGTAG (SEQ ID NO: 883) |
| 436 | DRG1 (SEQ ID NO: 12) | AGTTGTTAAAATGTAGATT (SEQ ID NO: 884) |
| 437 | DRG1 (SEQ ID NO: 12) | ATCGTATTGGTTCGCGG (SEQ ID NO: 885) |
| 438 | DRG1 (SEQ ID NO: 12) | ATTGTATTGGTTTGTGG (SEQ ID NO: 886) |
| 439 | DRG1 (SEQ ID NO: 12) | TTTACGTTTTGCGATT (SEQ ID NO: 887) |
| 440 | DRG1 (SEQ ID NO: 12) | AGTTTTATGTTTTGTGAT (SEQ ID NO: 888) |

(continued)

| ***No:*** | ***Gene*** | ***Oligo***: |
|---|---|---|
| 441 | DRG1 (SEQ ID NO: 12) | ATTTTTACGCGGAATT (SEQ ID NO: 889) |
| 442 | DRG1 (SEQ ID NO: 12) | GTGATTTTTATGTGGAAT (SEQ ID NO: 890) |
| 443 | DRG1 (SEQ ID NO: 12) | ATTCGAAGTATCGCGT (SEQ ID NO: 891) |
| 444 | DRG1 (SEQ ID NO: 12) | ATTTGAAGTATTGTGTTT (SEQ ID NO: 892) |
| 445 | DRG1 (SEQ ID NO: 12) | ATTTCGAATTTCGAGTA (SEQ ID NO: 893) |
| 446 | DRG1 (SEQ ID NO: 12) | TTTGAATTTTGAGTAGAG (SEQ ID NO: 894) |
| 447 | DOCK10 (SEQ ID NO: 16) | TATATTGTCGGGGAGA (SEQ ID NO: 895) |
| 448 | DOCK10 (SEQ ID NO: 16) | ATATTGTTGGGGAGAG (SEQ ID NO: 896) |
| 449 | BTG4 (SEQ ID NO: 17) | GTGTTGCGTAGAGATA (SEQ ID NO: 897) |
| 450 | BTG4 (SEQ ID NO: 17) | GGTGTTGTGTAGAGAT (SEQ ID NO: 898) |
| 451 | BTG4 (SEQ ID NO: 17) | TTGGTTTTTCGGAATAA (SEQ ID NO: 899) |
| 452 | BTG4 (SEQ ID NO: 17) | GGTTTTTTGGAATAAGAT (SEQ ID NO: 900) |
| 453 | GPR7 (SEQ ID NO: 19) | ATTGAGGGCGTATAGA (SEQ ID NO: 901) |
| 454 | GPR7 (SEQ ID NO: 19) | TGAGGGTGTATAGATTT (SEQ ID NO: 902) |
| 455 | GPR7 (SEQ ID NO: 19) | GGAGATCGAAGTTTGT (SEQ ID NO: 903) |
| 456 | GPR7 (SEQ ID NO: 19) | GGGGAGATTGAAGTTT (SEQ ID NO: 904) |
| 457 | ISL1 (SEQ ID NO: 21) | AGATTTTGCGAAAGATA (SEQ ID NO: 905) |
| 458 | ISL1 (SEQ ID NO: 21) | TTAGATTTTGTGAAAGATA (SEQ ID NO: 906) |
| 459 | ISL1 (SEQ ID NO: 21) | AAGATATCGAAATTAAGTT (SEQ ID NO: 907) |
| 460 | ISL1 (SEQ ID NO: 21) | AAGATATTGAAATTAAGTTT (SEQ ID NO: 908) |
| 461 | GPRK5 (SEQ ID NO: 22) | AGATTTTCGAGGGAGA (SEQ ID NO: 909) |
| 462 | GPRK5 (SEQ ID NO: 22) | AGATTTTTGAGGGAGAT (SEQ ID NO: 910) |
| 463 | FBN2 (SEQ ID NO: 28) | AATTGGTCGTTAGTTTT (SEQ ID NO: 911) |
| 464 | FBN2 (SEQ ID NO: 28) | GTTAATTGGTTGTTAGTT (SEQ ID NO: 912) |
| 465 | FBN2 (SEQ ID NO: 28) | TTAGGGATCGGATTTG (SEQ ID NO: 913) |
| 466 | FBN2 (SEQ ID NO: 28) | ATTAGGGATTGGATTTG (SEQ ID NO: 914) |
| 467 | LIMK1 (SEQ ID NO: 30) | TTTAATTCGAAAGGGAA (SEQ ID NO: 915) |
| 468 | LIMK1 (SEQ ID NO: 30) | TTAATTTGAAAGGGAAAG (SEQ ID NO: 916) |
| 469 | PSD/Q9H469 (SEQ ID NO: 31) | AGGTTAGTCGAGAAGT (SEQ ID NO: 917) |
| 470 | PSD/Q9H469 (SEQ ID NO: 31) | AGGTTAGTTGAGAAGTA (SEQ ID NO: 918) |
| 471 | PSD/Q9H469 (SEQ ID NO: 31) | ATTGTTACGAAGTGGA (SEQ ID NO: 919) |
| 472 | PSD/Q9H469 (SEQ ID NO: 31) | TTGTTATGAAGTGGAAG (SEQ ID NO: 920) |
| 473 | Q96S01 (SEQ ID NO: 34) | TATTGATCGGTTGGAG (SEQ ID NO: 921) |
| 474 | Q96S01 (SEQ ID NO: 34) | TATTGATTGGTTGGAGG (SEQ ID NO: 922) |
| 475 | ABHD9 (SEQ ID NO: 37) | AGTTAGAGCGTATTATTT (SEQ ID NO: 923) |
| 476 | ABHD9 (SEQ ID NO: 37) | AATAGTTAGAGTGTATTATT (SEQ ID NO: 924) |
| 477 | MLLT3 (SEQ ID NO: 42) | TTAGTGGTCGGAGATA (SEQ ID NO: 925) |
| 478 | MLLT3 (SEQ ID NO: 42) | AGTGGTTGGAGATAGA (SEQ ID NO: 926) |

(continued)

| ***No:*** | ***Gene*** | ***Oligo***: |
|---|---|---|
| 479 | SOLH (SEQ ID NO: 43) | TATATTTCGTGAGGGTA (SEQ ID NO: 927) |
| 480 | SOLH (SEQ ID NO: 43) | TATATTTTGTGAGGGTAG (SEQ ID NO: 928) |
| 481 | Q9NWV0 (SEQ ID NO: 46) | ATTTTTACGAGAAGGTT (SEQ ID NO: 929) |
| 482 | Q9NWV0 (SEQ ID NO: 46) | GATTTTTATGAGAAGGTT (SEQ ID NO: 930) |
| 483 | H2AFY2 (SEQ ID NO: 48) | ATGGGAATCGTGGTTT (SEQ ID NO: 931) |
| 484 | H2AFY2 (SEQ ID NO: 48) | ATGGGAATTGTGGTTT (SEQ ID NO: 932) |
| 485 | RHOC (SEQ ID NO: 49) | AGGTTTACGGAAAAGG (SEQ ID NO: 933) |
| 486 | RHOC (SEQ ID NO: 49) | AAGGTTTATGGAAAAGG (SEQ ID NO: 934) |
| 487 | KBTBD6 (SEQ ID NO: 51) | TAGAGTCGGGTTTTGTA (SEQ ID NO: 935) |
| 488 | KBTBD6 (SEQ ID NO: 51) | TAGAGTTGGGTTTTGTA (SEQ ID NO: 936) |
| 489 | TRPM4 (SEQ ID NO: 52) | ATGGGGGTCGAGATTT (SEQ ID NO: 937) |
| 490 | TRPM4 (SEQ ID NO: 52) | ATGGGGGTTGAGATTT (SEQ ID NO: 938) |
| 491 | TCEB3BP1 (SEQ ID NO: 53) | GGTAGTCGGTATTAGG (SEQ ID NO: 939) |
| 492 | TCEB3BP1 (SEQ ID NO: 53) | TGGTAGTTGGTATTAGG (SEQ ID NO: 940) |
| 493 | TCEB3BP1 (SEQ ID NO: 53) | TGTAGTCGAAGGTTAG (SEQ ID NO: 941) |
| 494 | TCEB3BP1 (SEQ ID NO: 53) | TGTAGTTGAAGGTTAGT (SEQ ID NO: 942) |
| 495 | Q8NCX8 (SEQ ID NO: 54) | GGAGTTTATTCGGTTTAT (SEQ ID NO: 943) |
| 496 | Q8NCX8 (SEQ ID NO: 54) | GGAGTTTATTTGGTTTATA (SEQ ID NO: 944) |
| 497 | WDFY3 (SEQ ID NO: 58) | AATTGTAGTCGTTTAGTT (SEQ ID NO: 945) |
| 498 | WDFY3 (SEQ ID NO: 58) | AAATTGTAGTTGTTTAGTT (SEQ ID NO: 946) |
| 499 | ZNF566 (SEQ ID NO: 59) | TATTTTTTCGGTAGAGAT (SEQ ID NO: 947) |
| 500 | ZNF566 (SEQ ID NO: 59) | TTTTTTTGGTAGAGATTG (SEQ ID NO: 948) |
| 501 | ZNF566 (SEQ ID NO: 59) | ATGGGTTGCGTTTATA (SEQ ID NO: 949) |
| 502 | ZNF566 (SEQ ID NO: 59) | TGGGTTGTGTTTATAAG (SEQ ID NO: 950) |
| 503 | SEQ ID NO: 61 (SEQ ID NO: 61) | TTTAGGGCGAGGTTAT (SEQ ID NO: 951) |
| 504 | SEQ ID NO: 61 (SEQ ID NO: 61) | TTTAGGGTGAGGTTATT (SEQ ID NO: 952) |
| 505 | SEQ ID NO: 61 (SEO ID NO: 61) | TGTATATTTCGTAGGGT (SEQ ID NO: 953) |
| 506 | SEQ ID NO: 61 (SEQ ID NO: 61) | TTGTATATTTTGTAGGGT (SEQ ID NO: 954) |
| 507 | PTGS2 (SEQ ID NO: 9) | ATTTGAGCGGTTTTGA (SEQ ID NO: 955) |
| 508 | PTGS2 (SEQ ID NO: 9) | AATTTGAGTGGTTTTGA (SEQ ID NO: 956) |
| 509 | RASSF1 (SEQ ID NO: 10) | AGTAAATCGGATTAGGA (SEQ ID NO: 957) |
| 510 | RASSF1 (SEQ ID NO: 10) | AGTAAATTGGATTAGGAG (SEQ ID NO: 958) |
| 511 | DRG1 (SEQ ID NO: 12) | TGTATGAACGTGTAGTT (SEQ ID NO: 959) |
| 512 | DRG1 (SEQ ID NO: 12) | GTGTATGAATGTGTAGT (SEQ ID NO: 960) |

TABLE 11: Genes and sequences according to sequence listing.

| Gene (HUGO or SPTREMBL ID or EST Gene ID) | Ref.-Seq | Genomic SEQ ID NO: | Sense methylated converted SEQ ID NO: | Antisense methylated converted SEQ ID NO: | Sense unmethylated converted SEQ ID NO: | Antisense unmethylated converted SEQ ID NO: |
|---|---|---|---|---|---|---|
| CCND2 | NM_001759 | 1 | 65 | 66 | 193 | 194 |
| CDRN2A | NM_000077 | 2 | 67 | 68 | 195 | 196 |
| CD44 | NM_000610 | 3 | 69 | 70 | 197 | 198 |
| EDNRB1 | NM_000115 | 4 | 71 | 72 | 199 | 200 |
| ELK1 | NM_005229 | 5 | 73 | 74 | 201 | 202 |
| FOS | NM_005252 | 6 | 75 | 76 | 203 | 204 |
| GSTP1 | NM_000852 | 7 | 77 | 78 | 205 | 206 |
| RARB | NM_000965 | 8 | 79 | 80 | 207 | 208 |
| PTGS2 | NM_000963 | 9 | 81 | 82 | 209 | 210 |
| RASSF1 | NM_170715 | 10 | 83 | 84 | 211 | 212 |
| ESR2 | NM_001437 | 11 | 85 | 86 | 213 | 214 |
| DRG1 | NM_004147 | 12 | 87 | 88 | 215 | 216 |
| CMYA3 | | 13 | 89 | 90 | 217 | 218 |
| ONECUT2 | NM_004852 | 14 | 91 | 92 | 219 | 220 |
| MX1 | NM_002462 | 15 | 93 | 94 | 221 | 222 |
| DOCK10 | | 16 | 95 | 96 | 223 | 224 |
| BTG4 | NM_017589 | 17 | 97 | 98 | 225 | 226 |
| DMRTC2 | NM_033052 | 18 | 99 | 100 | 227 | 228 |
| GPR7 | NM_005285 | 19 | 101 | 102 | 229 | 230 |
| FAT | NM_005245 | 20 | 103 | 104 | 231 | 232 |
| ISL1 | NM_002202 | 21 | 105 | 106 | 233 | 234 |
| GPRK5 | NM_005308 | 22 | 107 | 108 | 235 | 236 |
| SLC35F2 | NM_017515 | 23 | 109 | 110 | 237 | 238 |

(continued)

| Gene (HUGO or SPTREMBL ID or EST Gene ID) | Ref.-Seq | Genomic SEQ ID NO: | Sense methylated converted SEQ ID NO: | Antisense methylated converted SEQ ID NO: | Sense unmethylated converted SEQ ID NO: | Antisense unmethylated converted SEQ ID NO: |
|---|---|---|---|---|---|---|
| C14orf59 | NM_174976 | 24 | 111 | 112 | 239 | 240 |
| SNRPN | NM_003097 | 25 | 113 | 114 | 241 | 242 |
| ARHGEF18 | NM_015318 | 26 | 115 | 116 | 243 | 244 |
| SNX8 | NM_013321 | 27 | 117 | 118 | 245 | 246 |
| FBN2 | NM_001999 | 28 3 | 119 | 120 | 247 | 248 |
| HOXB5 | NM_002147 | 29 | 121 | 122 | 249 | 250 |
| LIMK1 | NM_002314 | 30 | 123 | 124 | 251 | 252 |
| PSD; Q9H469 | NM_002779; NM_ 024326 | 31 | 125 | 126 | 253 | 254 |
| SLC38A1 | NM_030674 | 32 | 127 | 128 | 255 | 256 |
| HIST1H4J | NM_003495 | 33 | 129 | 130 | 257 | 258 |
| Q96S01 | Not applicable | 34 | 131 | 132 | 259 | 260 |
| Genomic region downstream of FOXL2 | NM_023067 | 35 | 133 | 134 | 261 | 262 |
| ORC4L | NM_002552 | 36 | 135 | 136 | 263 | 264 |
| ABHD9 | NM_024794 | 37 | 137 | 138 | 265 | 266 |
| CD37 | NM_001774 | 38 | 139 | 140 | 267 | 268 |
| GRN | NM_002087 | 39 | 141 | 142 | 269 | 270 |
| EPAS1 | NM_001430 | 40 | 143 | 144 | 271 | 272 |
| NOTCH1 | NM_017617 | 41 | 145 | 146 | 273 | 274 |
| MLLT3 | NM_004529 | 42 | 147 | 148 | 275 | 276 |
| SOLH | NM_005632 | 43 | 149 | 150 | 277 | 278 |
| ENSESTG00002636932 | Not applicable | 44 | 151 | 152 | 279 | 280 |
| Q8N365 | NM_144697 | 45 | 153 | 154 | 281 | 282 |

(continued)

| Gene (HUGO or SPTREMBL ID or EST Gene ID) | Ref.-Seq | Genomic SEQ ID NO: | Sense methylated converted SEQ ID NO: | Antisense methylated converted SEQ ID NO: | Sense unmethylated converted SEQ ID NO: | Antisense unmethylated converted SEQ ID NO: |
|---|---|---|---|---|---|---|
| Q9NWV0 | NM_017891 | 46 | 155 | 156 | 283 | 284 |
| ENST00000339569 | Not applicable | 47 | 157 | 158 | 285 | 286 |
| H2AFY2 | NM_018649 | 48 | 159 | 160 | 287 | 288 |
| RHOC | NM_005167 | 49 | 161 | 162 | 289 | 290 |
| NR2E1 | NM_003269 | 50 | 163 | 164 | 291 | 292 |
| KBTBD6 | NM_152903 | 51 | 165 | 166 | 293 | 294 |
| TRPM4 | NM_017636 | 52 | 167 | 168 | 295 | 296 |
| TCEB3BP1 | NM_020695 | 53 | 169 | 170 | 297 | 298 |
| Q8NCX8 | NM_178564 | 54 | 171 | 172 | 299 | 300 |
| Genomic sequence | Genomic sequence | 55 | 173 | 174 | 301 | 302 |
| SNAPC2 | NM_003083 | 56 | 175 | 176 | 303 | 304 |
| PTPRN2 | NM_002847 | 57 | 177 | 178 | 305 | 306 |
| WDFY3 | NM_014991 | 58 | 179 | 180 | 307 | 308 |
| ZNF566 | NM_032838 | 59 | 181 | 182 | 309 | 310 |
| Q9NP73 | NM_018466 | 60 | 183 | 184 | 311 | 312 |
|  | NM_173660 | 61 | 185 | 186 | 313 | 314 |
| Q86SP6 | Not applicable | 62 | 187 | 188 | 315 | 316 |
| HIST2H2BF regulatory region | NM_003529 | 63 | 189 | 190 | 317 | 318 |
| PMF1 | NM_000711 | 64 | 191 | 192 | 319 | 320 |
| PITX2 | NM_000325 | 961 | 962 | 963 | 964 | 965 |

TABLE 12: Primers and Probes for MSP-MethyLight™ Assays.

| | Forward | Reverse | Probe |
|---|---|---|---|
| SEQ ID NO: 19 | tgcggattttggcgaattc | aaaaacccgccgactacgaa | aactaaaacgcctaccgactaaatatccgcct |
| SEQ ID NO: 35 | gagttttcgcggttcgga | cgcgaccgctaaactcg | cgaccaaatccgaacccgtacatcg |
| SEQ ID NO: 37 | cggtatgtcgtcgcgtttc | ctaacaccgcttcgccg | cctaaccgacaacgccgccgtaat |
| SEQ ID NO: 7 | agttgcgcggcgatttc | gccccaatactaaatcacgacg | cggtcgacgttcggggtgtagcg |
| SEQ ID NO: 63 | atggcgtataggttcgtgttttc | cttccaacgactaatacgcgaa | cgcttccaaaactcgaccgtaataacgc |
| SEQ ID NO: 8 | atataaactaaaaaacgaaacgataaacga | ttttacgttttttatttgcggc | cgaacgaacgcaaacgaaacaccg |
| SEQ ID NO: 64 | ccactaactccgtaccgtacgtat | ggttagcgagtcgatcggtt | acgttctcgtctccgctaaattatccgc |
| SEQ ID NO: 43 | tcgtttttttagtcgtttgggtc | tatcgaaaccccgaaccg | caccgtcgcctcccacgaca |
| SEQ ID NO: 40 | ttttcgtttttttcggtcgtt | gaccgcgcaaaaaactcg | cgctcgaataacgccgaacccg |
| SEQ ID NO: 32 | ctcgcacaaaaacgaaaatacg | agttcgcgtttttaaacgttgtc | ccgacaccgacccacgcgt |
| SEQ ID NO: 37 | cggtatgtcgtcgcgtttc | ctaacaccgcttcgccg | cctaaccgacaacgccgccgtaat |
| SEQ ID NO: 9 | aaacgaaaactctacccgaatacg | cgcggttttggcgttt | ccgaaatccccgatacgcgacg |
| SEQ ID NO: 19 | tgcggattttggcgaattc | aaaaacccgccgactacgaa | aactaaaacgcctaccgactaaatatccgcct |
| SEQ ID NO: 34 | gtatttatttggtaatttcgtattataattcgag | gactaaaaacgcgaaatccga | acgatccgatctaaaaaccgactcttcgaa |
| SEQ ID NO: 35 | gagttttcgcggttcgga | cgcgaccgctaaactcg | cgaccaaatccgaacccgtacatcg |

Table 13: Components for all QM assays according to Example 5

| Component | Company | Stock conc. |
|---|---|---|
| Reaction buffer ROX | Eurogentec | 10x |
| MgCl2 | Eurogentec | 50mM |
| DNTPs | MBI | 25mM each |
| Forward primer | TIB Molbiol | 6,25μM |
| Reverse primer | TIB Molbiol | 6,25μM |
| cg Probe | Eurogentec | 4μM |
| tg Probe | Eurogentec | 4μM |
| HotGoldStar-Taq | Eurogentec | 5U/μl |
| Water | Fluka | |

Table 14: Optimized Reaction conditions for all QM assays according to Example 5

| Gene | dNTPs | Buffer | $MgCl_2$ | Primers | Probes | Taq | Baseline | Threshold | Annealing |
|---|---|---|---|---|---|---|---|---|---|
| PITX2 | 250μM | 1x | 3mM | 625 nM | 200 nM | 1U | 3/23 | 0,05 | 62°C |
| Chr3-EST | 200μM | 1x | 3.5mM | 625 nM | 200 nM | 1U | 6/22 | 0,05 | 60°C |
| ABHD9 | 200μM | 1x | 25mM | 625 nM | 200 nM | 1U | 6/25 | 0,08 | 60°C |
| GPR7 | 250μM | 1x | 3mM | 625 nM | 150 nM | 1U | 6/24 | 0,05 | 60°C |
| HIST 2H2BF | 250μM | 1x | 3mM | 625 nM | 250 nM | 1U | 3/22 | 0,05 | 60°C |
| CCND2 | 250μM | 1x | 3mM | 625 nM | 250 nM | 1U | 3/22 | 0,08 | 60°C |

Table 15: Cycle program for QM assays according to Example 5. For annealing temperatures see Table 14.

| | T [°C] | t | Cycles |
|---|---|---|---|
| Initial denat. | 95.0 | 10min | |
| Denaturation | 95.0 | 15sec | 45x (PITX2 50x) |
| Annealing | Variable | 60sec | |

Table 16. Clinical characteristics of the patient population according to Example 5. Age is given as the mean, and all other variables are given as the number of patients. Not all information was available for all patients.

| Clinical Variable | | Baylor | Stanford | VMMC | Total |
|---|---|---|---|---|---|
| Age (mean) | | 61.1 | 61.7 | 61.1 | 61.3 |
| PSA | 0-4 | 25 | 33 | 18 | 76 |
| | 4-10 | 120 | 139 | 99 | 358 |
| | >10 | 60 | 72 | 30 | 162 |
| Gleason Score | 5-6 | 137 | 164 | 118 | 419 |
| | 7 | 37 | 44 | 19 | 100 |
| | 8-10 | 26 | 31 | 25 | 82 |

(continued)

| Clinical Variable | | Baylor | Stanford | VMMC | Total |
|---|---|---|---|---|---|
| Stage | Organ-confined | 110 | 211 | 113 | 434 |
| | Not organ-confined | 94 | 33 | 35 | 162 |
| PSA-based recurrence | | 22 | 10 | 13 | 45 |
| Decision to treat based recurrence | | 3 | 14 | 4 | 21 |
| Total Samples | | 206 | 244 | 162 | 612 |

Table 17: Performance of the six markers according to Example 5 using the median methylation level as a cut-off.

| | P value | Events in hypomethylated group | Events in hypermethylated group | AUC (5 years) |
|---|---|---|---|---|
| PITX2 | 0.000017 | 15 | 49 | 0.64 |
| GPR7 | 0.0016 | 20 | 45 | 0.64 |
| HIST2H2BF regulatory region | 0.018 | 22 | 43 | 0.60 |
| SEQ ID NO: 35 | 0.0059 | 21 | 44 | 0.61 |
| ABHD9 | 0.018 | 22 | 43 | 0.58 |
| CCND2 | 0.22 | 27 | 38 | 0.61 |

Table 18: Results of the Cox regression analysis for PITX2 according to Example 5. Using stepwise regression the marker remains in the model. P-values refer to the null-hypothesis "hazard ratio equals zero".

| Variable | P value | Hazard Ratio | Lower Confidence Interval | Upper Confidence Interval |
|---|---|---|---|---|
| PITX2 | 0.0043 | 2.222 | 1.284 | 3.845 |
| Disease Stage | 0.0692 | 1.713 | 0.965 | 3.061 |
| Gleason category | 0.0107 | 1.798 | 1.146 | 2.821 |
| PSA | 0.075 | 1.254 | 0.977 | 1.609 |
| Nomogram category | 0.0866 | 2.187 | 0.894 | 5.353 |

Table 19: Results of the Cox regression analysis for SEQ ID NO: 63 according to Example 5. The marker remains in the model.

| Variable | P value | Hazard Ratio | Lower Confidence Interval | Upper Confidence Interval |
|---|---|---|---|---|
| SEQ ID NO: 63 | 0.0239 | 2.918 | 1.152 | 7.393 |
| Disease stage | 0.0599 | 1.735 | 0.977 | 3.081 |
| Gleason category | 0.0106 | 1.799 | 1.146 | 2.822 |
| PSA | 0.0732 | 1.25 | 0.979 | 1.596 |
| Nomogram category | 0.0384 | 2.526 | 1.051 | 6.071 |

Table 20. Primer and probe sequences of assays according to Example 5.

| CCND2 | | | |
|---|---|---|---|
| Primers + Probes | Sequence | Label 5' | Label 3' |
| Forward primer | Ttttgtaaagatagttttgatttaagtat (SEQ ID NO: 983) | | |
| Reverse primer | caaactttctccctaaaaacc (SEQ ID NO: 984) | | |
| CG-probe | Cgccgccaacacgatcg (SEQ ID NO: 985) | FAM | BHQ1 |
| TG-probe | Caccaccaacacaatcaaccctaacac (SEQ ID NO: 986) | HEX | BHQ1 |
| | | | |
| SEQ ID NO: 63 | | | |
| Primers + Probes | Sequence | Label 5' | Label 3' |
| Forward primer | tgattattatgtttaaggatatttagttg (SEQ ID NO: 987) | | |
| Reverse primer | caataactctaaaaaaaacctttaaatc (SEQ ID NO: 988) | | |
| CG-probe | Cgctccccgcgaatacgacg (SEQ ID NO: 989) | FAM | BHQ1 |
| TG-probe | Taaacccactccccacaaatacaacaaac (SEQ ID NO: 990) | HEX | BHQ1 |
| | | | |
| GRP7 | | | |
| Primers + Probes | Sequence | Label 5' | Label 3' |
| Forward primer | Catccctacacttccaaac (SEQ ID NO: 991) | | |
| Reverse primer | Ggagttgttaggagaaaagtt (SEQ ID NO: 992) | | |
| CG-probe | Cgaacacccaaccgacaaacg (SEQ ID NO: 993) | FAM | BHQ1 |
| TG-probe | Caaacacccaaccaacaaacatctca (SEQ ID NO: 994) | HEX | BHQ1 |
| | | | |
| Chr3_EST | | | |
| Primers + Probes | Sequence | Label 5' | Label 3' |
| Forward primer | ttgtagggtttttttgggtt (SEQ ID NO: 995) | | |
| Reverse primer | Ctcaaaacccttaaaaacataaa (SEQ | | |
| | ID NO: 996) | | |
| CG-probe | Ataaccacactacgcgcctcc (SEQ ID NO: 997) | FAM | BHQ1 |
| TG-probe | Ataaccacactacacacctcccaca (SEQ ID NO: 998) | Yakima Yellow | I BHQ1 |
| | | | |
| ABHD9 | | | |
| Primers + Probes | Sequence | Label 5' | Label 3' |
| Forward primer | Ggtgttagggtttaggggtt (SEQ ID NO: 999) | | |
| Reverse primer | Ccaaatatttacctaacactcaaata (SEQ ID NO: 1000) | | |
| CG-probe | Aactattttctatcgaaaccgcccg (SEQ ID NO: 1001) | FAM | BHQ1 |
| TG-probe | Aactattttctatcaaaaccacccacctct (SEQ ID NO: 1002) | Yakima Yellow | BHQ1 |

(continued)

| ABHD9 | | | |
|---|---|---|---|
| Primers + Probes | Sequence | Label 5' | Label 3' |
| | | | |
| PITX2 | | | |
| Primers + Probes | Sequence | Label 5' | Label 3' |
| Forward primer | Gtaggggagggaagtagatgtt (SEQ ID NO: 1003) | | |
| Reverse primer | Ttctaatcctcctttccacaataa (SEQ ID NO: 1004) | | |
| CG-probe | Agtcggagtcgggagagcga (SEQ ID NO: 1005) | FAM | TAMRA |
| TG-probe | Agttggagttgggagagtgaaaggaga (SEQ ID NO: 1006) | VIC | TAMRA |
| CCND2 | | | |
| Primers + Probes | Sequence | Label 5' | Label 3' |
| Forward primer | tttttgtaaagatagttttgatttaagtat | | |
| Reverse primer | caaactttctccctaaaaacc | | |
| CG-probe | cgccgccaacacgatcg | FAM | BHQ1 |
| TG-probe | caccaccaacacaatcaaccctaacac | HEX | BHQ1 |
| | | | |
| SEQ ID NO: 63 | | | |
| Primers + Probes | Sequence | Label 5' | Label 3' |
| Forward primer | tgattattatgtttaaggatatttagttg | | |
| Reverse primer | caataactctaaaaaaaacctttaaatc | | |
| CG-probe | cgctccccgcgaatacgacg | FAM | BHQ1 |
| TG-probe | taaacccactccccacaaatacaacaaac | HEX | BHQ1 |
| | | | |
| GRP7 | | | |
| Primers + Probes | Sequence | Label 5' | Label 3' |
| Forward primer | catccctacacttccaaac | | |
| Reverse primer | ggagttgttaggagaaaagtt | | |
| CG-probe | cgaacacccaaccgacaaacg | FAM | BHQ1 |
| TG-probe | caaacacccaaccaacaaacatctca | HEX | BHQ1 |
| | | | |
| Chr3_EST | | | |
| Primers + Probes | Sequence | Label 5' | Label 3' |
| Forward primer | ttgtagggttttttgggtt | | |
| Reverse primer | ctcaaaacccttaaaaacataaa | | |

(continued)

| Chr3_EST | | | |
|---|---|---|---|
| Primers + Probes | Sequence | Label 5' | Label 3' |
| CG-probe | ataaccacactacgcgcctcc | FAM | BHQ1 |
| TG-probe | ataaccacactacacacctcccaca | Yakima Yellow | BHQ1 |
| | | | |
| ABHD9 | | | |
| Primers + Probes | Sequence | Label 5' | Label 3' |
| Forward primer | ggtgttagggtttaggggtt | | |
| Reverse primer | ccaaatatttacctaacactcaaata | | |
| CG-probe | aactattttctatcgaaaccgcccg | FAM | BHQ1 |
| TG-probe | aactattttctatcaaaaccacccacctct | Yakima Yellow | BHQ1 |
| | | | |
| PITX2 | | | |
| Primers + Probes | Sequence | Label 5' | Label 3' |
| Forward primer | gtaggggagggaagtagatgtt | | |
| Reverse primer | ttctaatcctcctttccacaataa | | |
| CG-probe | agtcggagtcgggagagcga | FAM | TAMRA |
| TG-probe | agttggagttgggagagtgaaaggaga | VIC | TAMRA |

Table 21

| SEQ ID NO | Classification | Tissue Type | AUC | Sensitivity | Specificity |
|---|---|---|---|---|---|
| 19 | Recurrance | Frozen & PET | 0.62 | 0.35 | 0.86 |
| 63 | Recurrance | Frozen & PET | 0.68 | 0.34 | 0.85 |
| 35 | Recurrance | Frozen & PET | 0.6 | 0.27 | 0.85 |
| 37 | Recurrance | Frozen & PET | 0.61 | 0.18 | 0.85 |
| 19 | Recurrance | PET | 0.58 | 0.5 | 0.88 |
| 63 | Recurrance | PET | 0.63 | 0.17 | 0.89 |
| 35 | Recurrance | PET | 0.69 | 0.33 | 0.89 |
| 37 | Recurrance | PET | 0.69 | 0.17 | 0.89 |
| 19 | Recurrance | Frozen | 0.62 | 0.34 | 0.86 |
| 63 | Recurrance | Frozen | 0.68 | 0.31 | 0.86 |
| 35 | Recurrance | Frozen | 0.6 | 0.28 | 0.85 |
| 37 | Recurrance | Frozen | 0.6 | 0.2 | 0.85 |
| 19 | Gleason | Frozen & PET | 0.72 | 0.49 | 0.86 |
| 63 | Gleason | Frozen & PET | 0.73 | 0.39 | 0.86 |
| 35 | Gleason | Frozen & PET | 0.62 | 0.21 | 0.86 |
| 37 | Gleason | Frozen & PET | 0.76 | 0.48 | 0.86 |
| 19 | Gieason | PET | 0.72 | 0.36 | 0.89 |

(continued)

| SEQ ID NO | Classification | Tissue Type | AUC | Sensitivity | Specificity |
|---|---|---|---|---|---|
| 63 | Gleason | PET | 0.7 | 0.38 | 0.86 |
| 35 | Gleason | PET | 0.56 | 0.12 | 0.86 |
| 37 | Gleason | PET | 0.77 | 0.5 | 0.86 |
| 19 | Gleason | Frozen | 0.74 | 0.56 | 0.87 |
| 63 | Gleason | Frozen | 0.76 | 0.58 | 0.86 |
| 35 | Gleason | Frozen | 0.65 | 0.28 | 0.87 |
| 37 | Gleason | Frozen | 0.77 | 0.47 | 0.87 |

## EXAMPLES

Investigation Overview

The objective of the present investigation is to develop genetic markers that can identify prostate cancer patients that have aggressive tumors with metastatic potential. It was decided to use methylation analysis to identify differentially expressed genomic markers.

The investigation started with a genome-wide screening step to discover novel markers. This approach utilizes molecular biology methods for the determination of differential methylation between predefined groups of patient samples. Differentially methylated sequences identified using said genomic screening methods are herein referred to as Methylation Sequence Tags (also referred to as MeSTs). The genome-wide screening step identifies differentially methylated CpG sites. Additional information concerning the area surrounding the identified CpG site is obtained by BLAST analysis of the sequence found in the screening step (MeST or Methylation Sequence Tag) and mapping to the human genome.

Following identification of candidates by genome-wide screening, MSP MethyLight™ assays were developed for a subset of the promising candidates. These assays were used to analyze methylation in 56 prostatectomy samples with clinical outcome information. MSP MethyLight™ assays are sensitive and quantitative and they rely on CpG co-methylation; therefore, this assay format provides complementary data to DNA array technology.

All of the promising MeST candidates and some additional candidates were then analyzed by methylation oligonucleotide array using the applicant's proprietary chip technology as described in further detail below. This process provides information concerning the preliminary performance of the MeSTs or candidate genes if an appropriate sample set is used. Candidate markers will be selected based on data obtained from the chip data analysis. Selection is mainly based on the AUC of the marker in the discrimination between the desired classes.

To complete the development process, the candidate markers selected for assay development from the chip study were tested in real-time PCR assays for further validation on the target population and on the sample material used for a potential diagnostic or prognostic test (paraffin embedded tissues).

### EXAMPLES 1: MeST Screening

Experimental Design

Pooled genomic DNA from prostate cancer samples was used for genome-wide screening of markers associated with tumor aggressiveness. Two different methods were applied: Methylation Specific-Arbitrarily Primed Polymerase Chain Reaction (MS-APPCR) (Liang et *al.*, 1998) and Methylated CpG Island Amplification (MCA) (Toyota et al., 1999). These technologies distinguish between methylated and unmethylated CpG sites through the use of methylation sensitive enzymes n general, genomic DNA is cut with a methylation sensitive restriction enzyme. Methylated fragments are preferentially amplified because cleavage at unmethylated sites prevents amplification of the unmethylated targets. Methylated sequence tag (MeST) fragments obtained using these techniques are sequenced and mapped to the human genome using the BLAST utility in the Ensembl database (www.ensembl.org).

The primary definition of tumor aggressiveness for the screening phase was based on PSA recurrence after radical prostatectomy. An aggressive tumor was defined as one that recurred in less than 24 months. A non-aggressive tumor was defined as one that did not recur after at least 48 months of follow up with regular PSA testing. Five samples in each category were pooled, and there were three pools for each category. The median time to PSA recurrence for the patients with aggressive tumors was 5.1 months. The median follow up time for patients without recurrence was 60.3 months. None of these patients received neo-adjuvant or adjuvant therapies before PSA relapse.

We also included four other comparisons with alternative indicators of aggressiveness. Samples with Gleason grades four and five were compared to samples with Gleason grades one, two, and three. Late stage tumors (III and IV) were compared to early stage tumors (I and II). Peripheral zone tumors were compared to transition zone tumors. Lastly, normal tissues adjacent to tumors in patients with early PSA recurrence (<2 years) were compared to normal tissues adjacent to tumors in patients with no PSA recurrence (>4 years follow up).

Screening

The MeST screening process usually results in a large number of sequences that represent potential markers. Some of them are redundant or cannot be matched to the genome. The remaining sequences are selected using a scoring procedure assessing:

Appearance using multiple methods
Appearance in multiple pool comparisons of the same type
Location in CpG island
Location in promoter region
Location near or within gene sequence
Association of nearby gene with cancer
Class of gene (transcription factor, growth factor, etc.)
Repetitive element (negative score)

In this scoring scheme, a MeST sequence receives one point for each of the positive criteria (first seven criteria), and receives a score ot minus 8 tor having repetitive sequence content greater than 50 % (negative score). In the latter case the MeST always has an overall negative score. Tables 2 and 3 summarize the results of the MeST screening experiments.

Using the scoring criteria and literature based investigation of potential gene function, 80 genes were selected for chip amplicon design. MeSTs from the comparisons based on PSA recurrence were prioritized, but many of the MeSTs from the other comparisons were included in the amplicon design list.

**EXAMPLE 2: Real-time PCR Study**

Experimental Design

MSP assays were developed on the Taqman™ 7900 for strong candidates from MeST screening in order to obtain early data on the performance of the MeSTs as markers of prostate cancer aggressiveness.

As used herein the term MSP-MethyLight shall be taken to mean an assay comprising the amplification of a bisulfite treated sequence by means of methylation specific primers and the detection of resultant amplificates by means of MethyLight detection oligonucleotides (also referred to as 'probes').

MSP-MethyLight assays were developed for a number of MeSTs (see Table 12). The assays were tested on artificially methylated DNA and dilutions of methylated DNA in unmethylated DNA to ensure assay performance. All assays were able to amplify as little as 100 picograms of methylated DNA in the presence or absence of 20 to 100 nanograms of unmethylated DNA. Most of the assays were quantitative between 0.1 and 100% methylation.

Of the 36 assays, 21 were methylated in a pool of prostate tumor DNAs. These 21 assays were first tested on 46 samples from the screening process. These 46 samples included 14 prostatectomies from patients who recurred in less than 24 months and 18 prostatectomies from patients who did not recur after at least 48 months. In addition, there were fourteen patients without follow up information; nine were high Gleason (Score 8-10) and 5 were low Gleason (Score 2-6). The data from this experiment were used to choose seven assays for an independent sample set.

The second sample set consisted of 26 frozen radical prostatectomy samples from patients with early PSA recurrence, with a median time to PSA recurrence of 6 months, and 30 samples from patients with no PSA recurrence after at least 48 months (median follow up time was 60 months). The MethyLight assays were used to measure the amount of DNA methylated at each locus by comparing the threshold cycle (Ct) to a standard curve of methylated DNA. A control assay was used to measure the total amount of DNA. All samples were run in triplicate for all assays. The primer and probe sequences are listed in Table 12. The ratio of the methylated DNA to the total amount of DNA was used to indicate the methylation status of each candidate in each sample.

Results

The methylation values for each assay were used to construct ROC curves (Figures 2 to 8) and calculate sensitivity, specificity, and p values. The data are summarized in Table 4. The AUC values for some of the candidates suggest that the methylation of the marker could have prognostic value. Six candidates had an AUC of 0.68 or greater. The strongest candidates, SEQ ID NO: 19 (GPR7) and SEQ ID NO: 35 (genomic region downstream of FOXL2), were significant by a Wilcoxon test after Bonferroni correction. When the specificity is set to 87% for these two assays, the sensitivity is around 50% for each. (SEQ ID NOs correlated to gene names in table 11.)

**EXAMPLE 3: Chip Study**

In the chip study, a gene panel composed of candidate genes and selected MeSTs were analyzed on 329 samples using the applicant's microarray technology.

Sample Set

The sample set included 329 frozen samples obtained from radical prostatectomies. Only samples with an estimated percent tumor of at least 70% were used, and the median estimated percent tumor (by volume) was 90%. Some sample providers achieved high percent tumor either by coring out a section of the frozen prostate known to contain tumor or by dissecting normal tissue away from the tumor. Patients who received neo-adjuvant therapy were not excluded from the study. Clinical information on disease free survival was not used for any patient receiving adjuvant therapy prior to disease recurrence.

Gleason scores were available for almost all prostatectomies. For some samples, the Gleason score of the portion of the tumor provided to the applicant was also available. The sample set consisted of samples that qualified for one of the two extreme Gleason categories (high or low), samples from patients that qualified for the two relapse categories (early or no relapse), or samples that fell into multiple categories (e.g., high Gleason and early recurrence). Within the sample set, there were 135 samples with low Gleason scores (1+2, 2+1, 2+2, 2+3, 3+2, and 3+3). There were 99 samples with high Gleason scores (3+5, 5+3, 4+4, 4+5, 5+4, and 5+5). For some of the samples, clinical follow up information was available. Sixty-five patients experienced PSA recurrence in less than two years, and 88 patients did not recur after at least 4 years follow up.

For control purposes additional samples were included. In order to control the quality and the functionality of oligos, unmethylated (phi-29 DNA) and artificially methylated DNAs (Promega) were used. Additionally, 16 DNA samples from lymphocytes were processed in parallel to the test samples.

Array

The array contained oligos representing 62 different candidates. Fifty-one of the candidates were MeSTs. One MeST, SEQ ID NO:32, was represented by two non-overlapping amplicons, both near exon one of the gene. For all of the MeSTs, the amplicon was designed as close to the MeST sequence as possible in a CpG rich region. An amplicon for the X chromosome gene ELK1 was included for analysis of male and female control lymphocyte samples.

Other analysed genes included CCND2 (Cyclin D2 Padar et al 2003), CD44 (Woodson et al 2004), EDNRB1 (endothelin receptor B; Woodson et al 2004; Nelson et al 1997), GSTP1 (glutathione S-transferase pi; Maruyama et al 2002), RARB (retinoic acid receptor, beta; Singal et al 2004), PTGS2 (prostaglandin-endoperoxide synthase 2; Yegnasubramanian et al 2004), RASSF1 (Ras association domain family 1; Liu et al 2002), ESR2 (estrogen receptor 2; Zhu et al 2004), DRG1 (developmentally regulated GTP binding protein 1; Bandyopadhyay et al 2003), and CDKN2A (p16; Halvorsen et al 2000). DRG1 was represented with two amplicons. In all cases, CpG rich areas near the promoter or exon 1 were targeted. For p16, the CpG rich area encompassing exon 2 was used because higher methylation rates have been noted in the literature (Nguyen et al 2000).

A complete overview of all analyzed genes can be found in Table 11.

Statistical Methods; Analysis of Chip Data

From Raw Hybridization Intensities to Methylation Ratios

The log methylation ratio (log(CG/TG)) at each CpG position is determined according to a standardized preprocessing pipeline that includes the following steps:

- For each spot the median background pixel intensity is subtracted from the median foreground pixel intensity. This gives a good estimate of background corrected hybridization intensities;
- For both CG and TG detection oligonucleotides of each CpG position, the background corrected median of the 4 redundant spot intensities is taken;

- For each chip and each CG/TG oligo pair, the log(CG/TG) ratio is calculated; and
- For each sample, the median of log(CG/TG) intensities over the redundant chip repetitions is taken.

This log ratio has the property that the hybridization noise has approximately constant variance over the full range of possible methylation rates (Huber et al., 2002).

Principle Component Analysis

The principle component analysis (PCA) projects measurement vectors (e.g. chip data, methylation profiles on several CpG sites etc.) onto a new coordinate system. The new coordinate axes are referred to as principal components. The first principal component spans the direction of the largest variance of the data. Subsequent components are ordered by decreasing variance and are orthogonal and uncorrelated to each other. Different CpG positions contribute with different weights to the extension of the data cloud along different components. PCA is an unsupervised technique, i.e. it does not take into account any group or label information of the data points (for further details see e.g. Ripley, 1996). PCA is typically used to project high dimensional data (in our case methylation-array data) onto lower dimensional subspaces in order to visualize or extract features with high variance from the data. In the present report we used 2 dimensional projections for statistical quality control of the data. We investigated the effect of different process parameters on the chip data and excluded that changing process parameters caused large alterations in the measurement values. A robust version of PCA was used to detect single outlier chips and exclude them from further analysis (Model *et al.,* 2002).

$T^2$ Control Charts

To control the general stability of the chip production process we use methods from the field of multivariate statistical process control (MVSPC). Our major tool is the $T^2$ control chart, which is used to detect significant deviations of the chip process from normal working conditions (Model *et al.,* 2002). The $T^2$ chart is constructed as follows:

1. Order the chip data with respect to a process parameter (e.g. hybridization date or spotting robot);
2. Define a historic data set, which describes the chip process under normal working conditions (e.g. the first 75 hybridized chips). In the chart, data from the historical data set are indicated by a speciai pioi symbol; and
3. Compute the distance of every new chip to the historic data set. If the distance of several consecutive chips exceeds a given control limit the process has to be regarded as out of control.
Use of $T^2$ charts to monitor the chip production process allows us to efficiently detect and eliminate most systematic error sources.

Hypothesis testing

Our main task is to identify markers that can make a significant contribution to the class prediction of samples. A significant contribution is detected when the null-hypothesis that a prediction model including the marker does not improve classification performance over a model without the marker can be rejected with $p<0.05$. Because we apply this test to a whole set of potential markers, we have to correct the p-values for multiple testing. We do this by applying the conservative Bonferroni correction, which simply multiplies the single marker p-values with the number of potential markers tested. We also give results with the less conservative False Screening Rate (FDR) method (Dudoit et al 2002). Throughout this report a marker (sometimes also simply refered to as gene or amplicon) is a genomic region of interest (ROI). It usually consists of several CpG positions in the respective area. For testing the null hypothesis that a marker has no predictive power we use the Wilcoxon rank sum tests to compare groups. A significant test result ($p<0.05$) indicates a shift between the distributions of the respective methylation logratios, i.e. $ln(CG/TG)$. The mean of all oligos for each mrker was used to combine CpGs before Wilcoxon statistics were generated. This approach has the advantage that it favors markers showing co-methylation.
A significant p-value for a marker means that the methylation of this ROI has some systematic correlation to the question of interest as given by the two classes. In general a significant p-value using the Wilcoxon rank sum test also implies good classification performance.

Class prediction by ROC analysis

Receiver Operation Characteristic (ROC) analysis was used to estimate how well the CpG ensemble of a selected marker can differentiate between different tissue classes. An ROC curve is a plot of true positive rate (sensitivity) versus false positive rate (1 - specificity) for a marker over all possible test thresholds. The Area Under the Curve (AUC) of an ROC curve gives the probability of properly classifying a random sample and can thus be used to evaluate overall marker performance. The AUC is related to the Wilcoxon test statistic and comparative ranking of markers by AUCs or Wilcoxon p-values is equivalent. The mean of all oligos for each marker was used to combine CpGs before ROC analysis. This approach has the advantage that it favors markers showing co-methylation.

Experimental Performance

DNA Extraction

Samples were received from external collaborators either as frozen tissues or extracted genomic DNA. DNA from tissue samples was isolated at Epigenomics Berlin using the Qiagen DNA Mini Kit.
The DNA quality of all delivered and extracted samples was first assessed by photometrical measurements. Extinctions at 260 nm and 280 nm as well as A260/280 ratios were determined and the resulting concentrations were calculated. For most of the DNAs used, A260/280 ratios between 1.6 and 1.9 were determined indicating sufficient purity. For some samples ratios in the range of 1.2-1.5 were calculated. Nevertheless, these DNAs were processed as well.
After photometrical measurements 200 ng of the genomic DNAs were applied to a 0.8% agarose gel and gel electrophoresis was performed. Figure 8 shows a typical gel image. No or only minor signs of degradation were observed, indicating a good overall quality of the DNAs used.
Bisulfite Treatment and Multiplex PCR
Total genomic DNAs from all selected samples as well as control DNAs were bisulfite treated converting unmethylated cytosines to uracil. Methylated cytosines are conserved. Bisulfite treatment was performed using Epigenomics' dioxane bisulfite treatment process. In order to avoid a joint processing of all samples with the same biological background resulting in a potential process-bias in the data later on, the samples were randomly grouped into processing batches. Batches of 50 samples were randomized for the Gleason score and PSA outcome. Two independent bisulfite reactions were performed per DNA sample. After bisulfitation, 11.25 ng of each sample was used in 8 subsequent multiplex PCR (mPCR) reactions containing 8 primer pairs each.
For monitoring the mPCR results, gel electrophoresis was performed for all PCR products. To find the best composition of eight primer pairs in a mPCR-set, ALF analysis was used, comparing a mixture of single PCR products with different variants of mPCR-sets.

ALF Express Analyses:

For evaluation of the amplified fragments, mPCR products of Promega DNA were analyzed using the ALF Express-technology. The results for those mPCRs were compared to the mixture of single PCR products. Figure 9 illustrates the result for an 8-plex PCR. All 64 fragments (eight 8-plex-PCRs) selected for the study could be amplified in the performed mPCR experiments. In some cases undesired side products were obtained.

Agarose Gel Electrophoresis:

As mentioned above two independent bisulfite reactions and PCRs were performed per DNA sample and the PCR products obtained were applied to a 2% agarose gel. In Figure 10 a typical gel image is shown illustrating the mPCR performance for 10 samples. No visible PCR product implies failure of bisulfite treatment or PCR amplification. The PCR was then repeated with twice the amount of DNA (22.5 ng). Bisulfite treated DNAs that failed again were excluded from the study. f we obtained only one hybridization probe (64 pooled PCR products) from a sample, 4 chips were hybridized using this single probe. If the probes from two independent bisulfite treatments of a sample were successfully amplified, both probes were hybridized onto two chips each.
Four (4) out of 331 samples processed (including control samples) could not be amplified, despite several attempts. These samples were not further processed.

Results of Chip Study

Our primary analysis was a comparison of samples with high Gleason scores (8-10) and low Gleason scores (2-6 with no grade 4 or 5 component). These classes are categories A and C in Table 5. For our second comparison, we used a group of samples from patients with early PSA recurrence after surgery (<2 years) and a group with no recurrence (>4 years follow up). These classes are A1, B1, C1, and D1 for the no recurrence group and A2, B2, and C2 for the recurrence group (see Table 5). These two sample sets (Gleason and clinical outcome) overlapped somewhat. Our third comparison analyzed only patients with intermediate Gleason (3+4, 4+3, 2+5, 5+2, 2+4, 4+2), to determine whether methylation of our candidates sequences correlates with early recurrence in these patients. Therefore, only categories B1 and B2 were included.

*Tumor Tissue vs. Lymphocytes*

In order to evaluate the diagnostic value of the chip, sixteen lymphocyte samples were included into the study. Prostate cancer tissues and lymphocytes were compared using Wilcoxon rank sum statistics. A ranked display for the ten best amplificates is given in Figure 12. Whereas the lymphocyte group is somewhat homogeneous, the figure displays larger variability for the prostate cancer samples. Differences between groups are significant at the 0.05 level (after 5% false discovery rate correction) for amplificates of CDRN2A, ELK1, GSTP1, RARB, PTGS2, RASSF1, ESR2, ONECUT2, BTG4, SLC35F2, HOXB5, LIMK1, HIST1H4J, SEQ ID NO: 35, EPAS1, NOTCH1, SEQ ID NO: 55, PTPRN2, Q9NP73, MX1, DOCK10, CCND2, ISL1, SNAPC2, GRN, H2AFY2, WDFY3, FOS, FAT, Q86SP6, SLC38A1, SNRPN, GPRK5, FBN2, ARHGEF18, RHOC, KBTBD6, NR2E1, PSD, DRG1, Q8N365, SEQ ID NO: 44, Q96S01, CD37, CMYA3, SEQ ID NO: 61, Q8NCX8 and ZNF566

Candidate markers for Gleason

High Gleason vs. Low Gleason Comparison

Wilcoxon rank statistics were used to analyze differences in methylation profiles of patients classified as high Gleason (Score 8-10) and low Gleason (Score 2-6, no grade 4 or 5 component). The high Gleason class consists of 98 samples and the low Gleason class consists of 135 samples. Figure 12 shows the results of this analysis.
For 25 amplificates, the Bonferroni corrected p-value of the Wilcoxon test is below 0.05. For a discussion of biological relevance see section below. Figure 13 displays the methylation matrix of the 10 best markers.
The AUC/sensitivity/specificity of the candidate marker amplificates are given in Table 6. Figure 13 shows the High Gleason vs. Low Gleason methylation matrix of the 10 markers with best AUC. Each column represents one sample; each row one oligonucleotide (1, 2, or 3 CpG sites each). Oligonucleotides are grouped per marker candidate. The indicated markers are ordered from top to bottom with increasing AUC. On the right side of each marker Bonferroni corrected Wilcoxon p-value and AUC are given. Below the AUC sensitivity at a specificity of - 0.75 are given enclosed in brackets. Methylation data are centered and normalized to one standard deviation for individual oligonucleotides. The coior represents the relative distance of the oligonucleotide methylation status from the mean vaiue. Light grey represents hypomethylated CpGs within an oligonucleotide while dark grey indicates hypermethylated CpGs within an oligonucleotide.

Candidate markers for PSA recurrence

Early Recurrence vs. No Recurrence Comparison

We next analyzed differences in methylation profiles of patients classified as early recurrence (PSA relapse in less than 24 months) and no recurrence (no PSA relapse after at least 4 years).
For three (3) amplificates the Bonferroni corrected p-value of the likelihood-ratio (LR) test is below 0.05. For a discussion of biological relevance see below.
The AUC/sensitivity/specificity of the top marker amplificates are given in Table 7.
Figure 15 shows Early Recurrence vs. No recurrence methylation matrix of the 10 markers with best AUC. Each column represents one sample; each row one oligonucleotide (1, 2, or 3 CpG sites each). Oligonucleotides are grouped per marker candidate. The indicated markers are ordered from top to bottom with increasing AUC. On the right side of each marker Bonferroni corrected Wilcoxon p-value and AUC are given. Below the AUC sensitivity at a specificity of ~ 0.75 are given enclosed in brackets. Methylation data are centered and normalized to one standard deviation for individual oligonucleotides. The color represents the relative distance of the oligonucleotide methylation status from the mean value. Light grey represents hypomethylated CpGs within an oligonucleotide while dark grey indicates hypermethylated CpGs within an oligonucleotide.

Candidate markers for PSA recurrence in patients with intermediate Gleason Scores

Early Recurrence vs. No Recurrence Comparison

Finally, we analyzed differences in methylation profiles of intermediate Gleason samples from patients classified as early recurrence (PSA relapse in less than 24 months) and no recurrence (no PSA relapse after at least 4 years). Intermediate Gleason included all patients with scores 2+5, 5+2, 3+4, 4+3, 2+4, 4+2, 1+5, 5+1, and these patients are a subset of the group used in section 6.6.2. The majority were Gleason 3+4 or 4+3. Although no amplificates displayed a Bonferroni corrected p-value below 0.05, several markers showed promising AUCs (see Table 8). It is likely this comparison was underpowered due to the small sample set for this comparison.

For a discussion of biological relevance see below.

Co-methylation revealed by microarray analysis

Due to the design of the current chip study, we were able to determine areas within marker fragments that were co-methylated. In this design, at least two oligo pairs, each containing 1, 2 or 3 CpG sites, were included for each marker fragments analyzed. Details of CpG sites targeted in the array analysis can be found in figures 16 onwards. Evidence of co-methylation is apparent in the ranked matrix figures. In the ranked matrix figures from the microarray analysis each marker fragment is grouped horizontally. Since each marker fragment represents one to three amplicons and a minimum of four and up to thirty individual CpG sites, extensive information concerning the methylation status of the fragment can be determined. Consecutive dark grey boxes within the grouping of a fragment in a vertical direction indicate co-methylation of the oligonucleotides (and CpGs within that oligo). These data will be further analysed for the most discriminatory areas within a fragment and this information will be utilized for real-time PCR assay design.

Results Summary

In the primary analysis, a comparison of high and low Gleason samples, 25 markers met the criteria for statistical significance using very conservative statistical methodology. This comparison relies on Gleason as a surrogate indicator of aggressiveness, but it was used as the primary analysis because Gleason information was available for nearly all tumors. Fewer samples were available for the additional analysis, based on time to PSA relapse, but still two markers reached statistical significance.

Discussion

Biological Aspects

MeST Screening

The MeST Screening process was very successful, yielding over 400 candidates. In the real-time PCR and chip studies, the MeST candidates performed well. In the real-time PCR study, three MeSTs outperformed GSTP1. In the chip study, the top five candidates in the Gleason comparison are all MeSTs. Therefore, the screening process contributed valuable candidate markers for distinguishing aggressive and non-aggressive tumors.

Furthermore, MeSTs from all of the screening comparisons were represented in the list of top scoring candidates. The top candidate marker in the Gleason comparison, GPR7, was discovered in two outcome comparisons, the Gleason comparison, and the comparison based on stage. Another top performer, DOCK10, was discovered in the comparison based on prostatic zone. Of the top markers, only ABHD9 was discovered in the comparison of normal tissue adjacent to tumors from patients in the two outcome categories. We conclude that all of the screening genome-wide screening comparisons yielded important candidate markers.

<u>Candidate evaluation by MethyLight</u>

Many candidate MeSTs were chosen for real-time PCR assay development while samples were being collected for the chip study. The assays were pre-screened on a pooled DNA sample from many prostate tumor samples. This step allowed pre-selecting only those assays that could potentially be informative Over one-third of the assays were ruled out at this step. Next, the remaining assays were tested on the DNA from the screening samples in order to prioritize the assays. Then, on the final set of 56 independent samples, six of the seven prioritized assays performed well.

The success of the real-time PCR experiment suggests that there is significant co-methylation in these markers. Therefore, real-time PCR assays, which all require some degree of co-methylation, will be suitable candidates for a final assay choice. The real-time PCR experiment relied heavily on quantification of methylation differences: For nearly all of the assays, the difference between the early recurrence group and the non-recurrence group was a quantitative methylation difference. The final assay type wiii need strong quantitative abilities.

<u>Candidate evaluation by Methylation Array</u>

The chip experiment was highly successful, demonstrating marker potential for many candidate sequences. In the comparison of high and low Gleason samples, 25 amplicons were significantly different. The vast majority of these are hypermethylated in high Gleason samples. The three candidates analyzed by real-time PCR were among the top six markers in this Gleason comparison. Therefore there is consistency between the two methods of measuring methylation.

The comparison based on patient PSA relapse characteristics had lower sample numbers. Despite these low numbers, we were still able to prove that at least three (3) of our candidate markers can significantly distinguish patients experiencing early relapse from patients not experiencing relapse. In general, methylation is higher in patients experiencing early recurrence. In this comparison, the top three candidates in the real-time PCR study were the top three most significant markers in the chip analysis. The AUCs for GPR7, SEQ ID NO: 35 (downstream of FOXL2), and ABHD9 were 0.72, 0.72, and 0.66 respectively in the chip clinical outcome data and 0.76, 0.75, and 0.70 respectively in the real-time PCR clinical outcome data.

Treatment for patients with high and low Gleason is often clear. Anyone with high Gleason will be recommended for aggressive treatment, including definitive treatment (surgery or radiation) and possibly adjuvant therapy. Patients with low Gleason have the option of deferring definitive treatment. While there are still some uncertainties for these patients, the best options are even less clear for patients with intermediate Gleason levels. Furthermore, the majority of patients being diagnosed with prostate cancer today have intermediate Gleason scores of 6 or 7. These are the patients that can be helped the most by a molecular classification test. The amplicons with the highest AUCs in the comparison based on clinical outcome were GPR7 (AUC=0.72) and SEQ ID NO: 35 (AUC=0.72). When this comparison was restricted to patients with intermediate Gleason scores (1+5, 5+1, 2+4, 4+2, 3+4, 4+3, 2+5, 5+2), the AUC for both of these markers was still 0.72 or greater. These results suggest that a methylation-based assay will provide information even for patients with middle range Gleason scores.

Biology of Marker Genes

Several interesting markers were identified by the real-time PCR and chip studies. One of the real-time PCR markers is a G protein coupled receptor (GPR7; SEQ ID NO:19), however very little is known about the gene product. A second marker from the real-time study is located in a CpG island in the promoter of the gene Abhydrolase Domain containing 9 (ABHD9; SEQ ID NO:37). The closest gene to SEQ ID NO:35 is FOXL2. The MeST is in a CpG island several kilobases downstream of this gene. SEQ ID NO:63 is in an area with several histone genes.

Additional markers emerged in the chip study. NOTCH1 (SEQ ID NO:41) controls a signalling pathway that regulates interactions between adjacent cells. Many labs have studied the role of this gene in carcinogenesis and metastasis. Little is known about many of the candidates, including DOCK10 (SEQ ID NO:16), SEQ ID NO:51, which is in the promoter of a gene called Kelch repeat and BTB (POZ) domain-containing 6, and SEQ ID NO:17, which is located between an EST and B-cell Translocation Gene 4. BTG4 has been shown to have growth inhibitory properties (Buanne et al 2000).

PTGS2 (SEQ ID NO:9) is the only gene previously shown in the literature to be more methylated in prostate tumors of patients who recurred soon after prostatectomy (Yegnasubramanian et al 2004). PTGS2, also known as cyclo-oxygenase (COX2), is a further promising candidate in both the Gleason and the clinical outcome analyses, with AUCs of 0.69 and 0.65 respectively. GSTP1 is the most highly studied methylation marker in prostate cancer, and while there are no published data directly demonstrating its prognostic value, there is some evidence that its methylation correlates with Gleason grade (Maruyama et al 2002). However, this correlation was not confirmed in another study (Woodson et al 2004). In the instant data, GSTP1 methylation significantly correlates with Gleason grade, but the AUC in the clinical outcome comparison is only 0.58.

Medical Aspects

The methylation candidates that have emerged from our Gleason comparison are informative prognostic markers. We have shown that some of these candidates that correlate with Gleason categories can also predict PSA relapse, even in patients with intermediate Gleason scores. Therefore it is likely that our analysis based on Gleason will provide markers that provide additional information to Gleason.

As individual markers, the chip candidates reach 40-60% sensitivity when the specificity is set at 75%. In the real-time study, the sensitivity of three of the markers was higher, reaching 50-60% at a specificity of 85%. The enhanced performance in the real-time might be due to the quantitative abilities of MSP-MethyLight. Approximately 20% of patients experience relapses within 5-10 years after surgery. If this were set as the prevalence of aggressive tumors in the radical prostatectomy population, then a marker such as ours with 50% sensitivity and 85% specificity would have a negative predictive value of 0.87 and a positive predictive value of 0.45. Therefore, a marker with this performance would define a group of patients with only a 13% chance of recurrence after surgery and a group of patients with a 45% chance of recurrence. The first group could just be monitored for PSA rise, and the second group would be candidates for adjuvant therapies.

While these candidates have been studied in prostatectomy samples, they will also be useful for analysis of biopsies. A marker that predicts outcome after prostatectomy correlates with the aggressiveness and metastatic potential of the tumor, and these properties will also be present in the biopsy. After biopsy and staging tests, patients opt for watchful waiting, definitive curative therapy, or a combination of treatments (such as surgery plus radiation or androgen ablation). A molecular test with high negative predictive value would allow more patients to choose watchful waiting. A molecular test with sufficiently high positive predictive value would select a subset of patients who should not receive radiation or surgery only. Thus, these candidate methylation markers have the potential to reduce both under and over treatment of prostate cancer.

**EXAMPLE 4: Real time quantitative methylation analysis**

Genomic DNA was analyzed using the Real Time PCR technique after bisulfite conversion.

The QM assay (= Quantitative Methylation Assay) is a Real-time PCR based method for quantitative DNA methylation detection. The assay principle is based on non-methylation specific amplification of the target region and a methylation specific detection by competitive hybridization of two different probes specific for the CG or the TG status, respectively. For the present study, TaqMan probes were used that were labeled with two different fluorescence dyes ("FAM" for CG specific probes, "VIC" for TG specific probes) and were further modified by a quencher molecule ("TAMRA" or "Minor Groove Binder/non-fluorescent quencher").

Evaluation of the QM assay raw data is possible with two different methods:

1. Measuring absolute fluorescence intensities (FI) in the logarithmic phase of amplification Difference in threshold cycles (Ct) of CG and TG specific probe.

In the following series of quantitative methylation assays the amount of sample DNA amplified is quantified by reference to the gene GSTP1 to normalize for input DNA. For standardization, the primers and the probe for analysis of the GSTP1 gene lack CpG dinucleotides so that amplification is possible regardless of methylation levels. As there are no methylation variable positions, only one probe oligonucleotide is required.

The reactions are calibrated by reference to DNA standards of known methylation levels in order to quantify the levels of methylation within the sample. The DNA standards were composed of bisulfite treated phi29 amplified genomic DNA (i.e. unmethlyated), and/or phi29 amplified genomic DNA treated with Sss1 methylase enzyme (thereby methylating each CpG position in the sample), which is then treated with bisulfite solution. Seven different reference standards were used with 0%, (i.e. phi29 amplified genomic DNA only), 5%, 10%, 25%, 50%, 75% and 100% (i.e. phi29 Sss1 treated genomic only).

<u>Bisulfite treatment</u>

Bisulfite treatment was carried out based on the method disclosed by Olek et al. Nucleic Acids Res. 1996 Dec 15;24(24):5064-6, and optimized to the applicant's laboratory workflow.

<u>Quantification Standards</u>

The reactions are calibrated by reference to DNA standards of known methylation levels in order to quantify the levels of methylation within the sample. The DNA standards were composed of bisulfite treated phi29 amplified human genomic DNA (Promega) (i.e. unmethlyated), and/or phi29 amplified genomic DNA treated with Sss1 Methylase enzyme (thereby methylating each CpG position in the sample), which is then treated with bisulfite solution. Seven different reference standards were used with 0%, (i.e. phi29 amplified genomic DNA only), 5%, 10%, 25%, 50%, 75% and 100% (i.e. phi29 Sss1 treated genomic only). 2000 ng batches of human genomic DNA (Promega) were treated with bisulfite. To generate methylated MDA DNA, 13 tubes of 4.5 μg MDA-DNA (700 ng/μl) was treated with Sss1.

Control assay

The GSTP1-C3 assay design makes it suitable for quantitating DNAs from different sources, including fresh/frozen samples, remote samples such as plasma or serum, and DNA obtained from archival specimen such as paraffin embedded material. The following oligonucleotides were used in the reaction to amplify the control amplificate:
Control Primer1: GGAGTGGAGGAAATTGAGAT (SEQ ID NO:966)
Control Primer2: CCACACAACAAATACTCAAAAC (SEQ ID NO:967)
Control Probe: FAM-TGGGTGTTTGTAATTTTTGTTTTGTGTTAGGTT-TAMRA (SEQ ID NO:968)

| | |
|---|---|
| Cycle program (40 cycles): | 95˚C, 10 min |
| | 95˚C, 15 sec |
| | 58˚C, 1 min |

Assay design and reaction conditions

Two assays were developed for the analysis of the gene PITX2(SEQ ID NO:961)
Assay 1:
Primers: GTAGGGGAGGGAAGTAGATGTT (SEQ ID NO:969)
TTCTAATCCTCCTTTCCACAATAA (SEQ ID NO:970)
Probes: FAM-AGTCGGAGTCGGGAGAGCGA-TAMRA (SEQ ID NO:971)
VIC-AGTTGGAGTTGGGAGAGTGAAAGGAGA -TAMRA (SEQ ID NO:972)

Amplicon (SEQ ID NO:973):
GtAGGGGAGGGAAGtAGATGttAGCGGGtCGAAGAGTCGGGAGtCGGAGtCGGGAGAGCGAAAGGAG
AGGGGAttTGGCGGGGtAtTTAGGAGttAAtCGAGGAGtAGGAGtACGGAtTtttAtTGTGGAAAGGAGGAttA
GAA

Length of fragment: 143 bp
Positions of primers, probes and CpG dinucleotides ar highlighted.
PCR components (supplied by Eurogentec) : 3 mM MgCl2 buffer, 10x buffer, Hotstart TAQ, 200 μM dNTP, 625 nM each primer, 200 nM each probe

| | |
|---|---|
| Cycle program (45 cycles): | 95˚C, 10 min |
| | 95˚C, 15 sec |
| | 62˚C, 1 min |

Assay 2 :
Primers: AACATCTACTTCCCTCCCCTAC (SEQ ID NO:974)
GTTAGTAGAGATTTTATTAAATTTTATTGTAT (SEQ ID NO:975)
Probes: FAM-TTCGGTTGCGCGGT-MGBNQF (SEQ ID NO:976)
VIC-TTTGGTTGTGTGGTTG- MGBNQF (SEQ ID NO:977)

Amplicon (SEQ ID NO:978):
GTtAGtAGAGATTttAttAAAtTttAtTGtAtAGTGGCGCGCGGGCGGtCGGtCGAGtCGGttGCGCGGTGGC
GATttAGGAGCGAGtAtAGCGttCGGGCGAGCGtCGGGGGGAGCGAGtAGGGGCGACGAGAAACGAGG
tAGGGGAGGGAAGtAGATGtt

Length of fragment: 164 bp
The positions of probes, primers and CpG positions are highlighted.
The probes cover three co-methylated CpG positions.
PCR components (supplied by Eurogentec): 2,5 mM MgCl2 buffer, 10x buffer, Hotstart TAQ, 200 μM dNTP, 625 nM each primer, 200 nM each probe

| | |
|---|---|
| Program (45 cycles): | 95˚C, 10 min |
| | 95˚C, 15 sec |
| | 60˚C, 1 min |

The extent of methylation at a specific locus was determined by the following formulas:

Using absolute fluorescence intensity: methylation rate= 100 * I (CG) / (I(CG) + I(TG))

(I = Intensity of the fluorescence of CG-rpobe or TG-probe)

Using threshold cycle Ct: methylation rate= 100*CG/(CG+TG)= 100/(1+TG/CG)= 100/(1+2^delta(ct))

(assuming PCR efficiency E=2; delta (Ct)=Ct (methylated) - Ct (unmethylated)

**Example 5: Validation**

The main goal of this phase of the investigation was to confirm the significance of previously identified marker candidates and optimize methylation cut-offs. The markers should be suitable to split patients who undergo prostatectomy into two groups: one with a high chance of PSA recurrence and one with a low chance of PSA recurrence. In addition, the markers should provide additional information to Gleason grade analysis. Markers meeting these criteria will have an important clinical role in selection of prostatectomy patients for adjuvant therapy.
The applicant had previously identified several markers with significantly higher methylation levels in patients who experienced PSA recurrence within 24 months of surgery compared to patients who did not experience PSA recurrence (see above Examples 1 to 4). Six of these markers were transferred to a real-time platform (QM Assay). These assays were used to analyze the methylation levels of 612 paraffin embedded prostatectomy samples from a cohort of node-negative patients from three institutions.
The primary aim of the invention was to provide markers that can differentiate between patients with low chance for PSA recurrence after surgery and those with a high chance for PSA recurrence. The performance of these markers as compared to traditional prognostic indicators such as Gleason grading and stage information is also provided.
It is a further aim of the present invention to determine where the markers are most informative in relation to current clinical prognostic assessment and accordingly provide particularly preferred use embodiments of the present invention. It is particularly preferred that a molecular test according to the present invention is combined, either formally or informally, with information from other prognostic sources, in particular Gleason grading.

Methods: QM Assay Description

Each QM-assay was developed to enhance performance without drastically altering standard conditions in order to allow future multiplexing. Primer and probe concentrations, $MgCl_2$ concentration and annealing temperature were optimized under fixed buffer and polymerase conditions. The assays were designed and optimized to ensure quantitative methylation analysis of each marker between 10 and 100 percent methylation. The assay products were checked on an agarose gel and no undesired products were detected. The results of the optimization procedure are shown in the following tables.

Sample Set

Paraffin-embedded prostatectomy tissue samples from 605 patients were analyzed. The samples were provided by the Baylor College of Medicine SPORE, Stanford University Department of Urology, and Virginia Mason Hospital in Seattle. The samples from Stanford and Virginia Mason were prepared by first finding the surgical block with the highest percent tumor, then sectioning the block. Three tubes were prepared, each with three 10 micron thick sections. The procedure was slightly different at Baylor. A core of tissue was removed from the tumor within the prostatectomy block, and then this core was cut into 10 micron sections. Ten sections were included into each of three tubes.
An adjacent section was mounted on a slide and H&E stained for histological analysis. A pathologist reviewed these slides for an independent determination of Gleason grading and percent tumor. The Gleason results were used for all analyses in this report. The original provider Gleason values are available, but they were not used for analysis due to known and hypothetical biases among the providers. Stanford, for instance, uses a percentage Gleason 4/5 for reporting grade, while the other two providers use the traditional system. The measured Gleason values provided an independent and uniform measurement.
A few samples were found to have no tumor cells on the H&E slide, and these patients were omitted from the analysis. In addition, we found a few patients that did not have a PSA nadir after surgery. These patients were also excluded from the study. In total, 612 patients were included in the data analysis.
Due to their coring technique, the percent tumor of the samples provided by Baylor were higher than the other providers.
All patients, aged 40-80, undergoing surgery at the three institutions during certain years were included in the study, with the exception of patients who received neo-adjuvant or adjuvant therapy (before PSA rise) and patients with positive nodes at the time of surgery. For Baylor, the time period was 1993-1998, for Virginia Mason it was 1996-2000, and for Stanford it was 1996-1999.

The overall cohort is similar to other prostatectomy cohorts described in the literature, such as the cohort collected by William Catalona and described in 2004 (Roehl et *al.*). The patient cohorts from each provider are similar for nearly all clinical parameters. One exception is the type of recurrence. While other institutions typically wait until the patient's PSA rises to 0.2 ng/ml or higher after surgery, the Stanford Department of Urology treats many patients when their PSA rises to 0.05. Therefore, Stanford has a higher rate of recurrence based on the decision to treat criteria and a lower rate of recurrence based on the PSA level (0.2ng/ml) criteria. See section 6.1 for a summary of the event definition criteria. Figure 89 provides a histogram of follow-up times for the patient cohort (all three providers included). The white bars consist of the patients who did not have a recurrence before they were censored, and the shaded bars consist of the patients who experienced recurrence. By selecting patients who received surgery from 1993-2000, we have ensured that the median follow-up time of the cohort (66 months) is long enough to have a significant number of patients who have relapsed.
For deparaffination, the 627 provided PET samples were processed directly in the tube in which they were delivered by the providers. One ml (Virginia Mason and Baylor) or 1.8 ml (Stanford) of limonene was added to each tube and incubated at room temperature for 10 minutes in a thermomixer with occasional vortexing. The samples were centrifuged at 16,000 x g for 5 minutes. The limonene supernatant was removed, and if no pellet was detected, centrifugation was repeated at higher speed and the remaining limonene was removed. For samples from Stanford, the deparaffination process was repeated once with 1.6 ml of limonene to get rid of residual paraffin.
For lysis of the tissue, 190 μl lysis buffer and 20 μl proteinase K was added to each deparaffinated sample. For Stanford samples, 570 μl lysis buffer and 60 μl proteinase K was used. After vortexing, samples were centrifuged briefly and incubated on a thermoshaker at 60°C for 40 hours. After the incubation, samples were checked to ensure that lysis was complete, and the proteinase was then inactivated at 95°C for 10 minutes. If the lysed samples were not directly used for DNA extraction, they were stored at -20°C.
The lysates were randomized based on the sample provider and PSA recurrence. The DNA was isolated using a QIAGEN DNeasy Tissue kit with a few modifications. 400 μl buffer AUE was distributed to collection tubes and 200 μl of lysate were added. The samples were mixed by shaking for 15 seconds. The lysate/buffer mixtures were applied to the 96-well DNeasy plate columns. The plate was sealed and centrifuged at 5790xg for 10 minutes. The columns were washed once with 500 μl of AW1 and then 500 μl AW2. The DNA was eluted with 120 μl buffer AE. Therefore, the final volume of extracted DNA was approximately 120μl. The DNA was stored at -20°C.

Bisulfite Treatment

The CFF real-time PCR assay was used to quantify the DNA concentration of the samples after extraction.
CFF sequence:
TAAGAGTAATAATGGATGGATGATGGATAGATGAATGGATGAAGAAAGAAAGGATGAGTGAGAGAA AGGAAGGGAGATGGGAGG (84bp) (SEQ ID NO: 979)
CFF-Forward primer TAAGAGTAATAATGGATGGATGATG (SEQ ID NO: 980)
CFF-Reverse primer CCTCCCATCTCCCTTCC (SEQ ID NO: 981)
CFF TaqMan probe ATGGATGAAGAAAGAAAGGATGAGT (SEQ ID NO: 982)

We adjusted the concentration of each genomic DNA sample so that 1ug of CFF1 measured DNA was present in 44 μl. The bisulfite treatment of genomic DNA derived from paraffin embedded tissue was performed using a 96 well protocol. Forty-four μl genomic DNA (with approximately 1μg of amplifiable DNA), 83 μl 4.9M bisulfite solution (pH 5.45-5.5), and 13 μL DME solution were pipetted into the wells of the plate. The samples were thoroughly mixed then placed in a thermocycler with the following program:

- 5:00 min denaturation of DNA at 99°C
- 22:00 min incubation at 60°C
- 3:00 min denaturation of DNA at 99°C
- 1:27:00 hours incubation at 60°C
- 3:00 min denaturation of DNA at 99°C

- 2:57:00 hours incubation at 60˚C
- Cooling at 20˚C

After the incubations, each sample was divided into two 70 μ•L aliquots. Each aliquot was combined with 280 μL of prepared Buffer AVL/Carrier RNA and 280 μL ethanol. The wells were sealed and the samples were mixed vigorously for 15 seconds. The plate was incubated for 10 minutes at room temperature. The first aliquot was applied to the QIAamp 96 plate and the plate was centrifuged for four minutes at 5790 x g. The process was repeated with the second aliquot so that both aliquots were applied to the same binding column. The columns were washed with 500 μL buffer AW1, then 500 μL 0.2 M NaOH, and then twice with 500 μL buffer AW2. The DNA was eluted with 100 μL elution buffer (Qiagen) pre-heated to 70 deg C. The bisDNAs were stored at -20˚C.

The bisulfite treated DNA samples were stored in 8 x 96 well plates (plate 01-08). The samples and controls were combined onto two 384-well PCR reaction plates for each QM assay. Each QM assay plate contained the samples of 4 x 96 well plates (85 wells actually used per plate) and 1x96 well plate with standard DNA (7 mixtures of the calibration DNA and water for the no template control PCR reaction). The QM assay plates were run three times.

The 384-well PCR plates were pipetted with the TECAN workstation. The pipetting program transferred first 10 μl of the mastermix and then 10 μl of the respective DNA into the designated well. The master mix was pipetted in a falcon tube and distributed to 8 x 500 μl screw cap vials for automatic pipetting with TECAN workstation.

All QM assays were run on an ABI TAQMAN 7900HT real-time device (SDS 2.2. software) with a reaction volume of 20 μl. PITX2 and CCND2 assays were run with 9600 emulation, and the other assays were not. An automatic sample setup was used to transfer the correct sample names and detector/reporter dyes to the TAQMAN software. The cycling conditions were manually adjusted and ROX was used as passive reference dye. All 384 well PCR plates we analyzed with the SDS2.2 software using the manual analysis settings (baseline setting with start and stop values and manual threshold) to produce results files for each run individually.

Methods: Evaluation of Marker Performance

<u>Definition of Events</u>

After a successful prostatectomy on a patient with non-metastatic disease, there should be no prostate cells left in his body and therefore his PSA levels should drop to zero. A patient's PSA levels are typically measured every 6-12 months after surgery to ensure that the patient remains free of prostate cancer. If PSA becomes detectable and rises to a certain level, the doctor and patient may decide on additional therapy. Therefore, the return and rise of PSA levels are the primary indication of disease recurrence.

A post-surgical PSA relapse is typically indicated by either a gradual or rapid rise in levels over a series of sequential tests. Depending on the clinical characteristics of the patient or the approach of the institution, patients may be treated as soon as PSA is detected, when it reaches a certain threshold, or when clinical symptoms accompany the PSA rise. Most institutions consider a PSA level of 0.2 ng/ml to be significant, and if a patient's PSA reaches this level and is confirmed to be rising in subsequent tests, he will be offered additional therapy. Stanford Department of Urology, one of the sample providers, considers 0.05 ng/ml to be a PSA recurrence, and considers treatment for patients when their PSA reaches this level.

An event in this study includes all PSA-based recurrences. A PSA level of 0.2 ng/ml, confirmed in subsequent tests, has been demonstrated to provide the best sensitivity and specificity for detection of recurrence (Freedland *et al*. 2003). Rise of PSA to this level normally precedes any development of clinical recurrence; therefore, nearly all of the patients in this study are free of clinical recurrence at the time of PSA recurrence. Because Stanford often treats patients with PSA recurrence before they reach this cut-off of 0.2ng/ml, many of their recurrence patients would be censored in the present study if the PSA level of 0.2ng/ml was the only considered event. Therefore, patients from any of the three institutions who receive therapy due to PSA levels are also considered an event in this study. To summarize, an event is defined in the present study as any rise in PSA to 0.2 ng/ml (confirmed in subsequent test) OR a decision to treat the patient based on PSA criteria.

<u>Raw QM Data Processing</u>

All analyses in this report are based on the CT evaluation. Assuming optimal real-time PCR conditions in the exponential amplification phase, the concentration of methylated DNA ($C_{metn}$) can be determined by

$$C_{meth} = \frac{100}{1 + 2^{(CT_{CG} - CT_{TG})}} [\%],$$

where
$CT_{CG}$ denotes the threshold cycle of the CG reporter (FAM channel) and
$CT_{TG}$ denotes the threshold cycle of the TG reporter (VIC channel).

The thresholds for the cycles were determined by visual inspection of the amplification plots (ABI PRISM 7900 HT Sequence Detection System User Guide). The values for the cycles ($CT_{CG}$ and $CT_{TG}$) were calculated with these thresholds by the ABI 7900 software. Whenever the amplification curve did not exceed the threshold, the value of the cycle was set to the maximum cycle e.g. 50.

The R software package, version 2.2. (Gentleman and Ihaka 1997), was used for the statistical analysis. In addition, we used the "survival" package, version 2.11-5 (http://cran.at.r-project.org/src/contrib/Descriptions/survival.html), for survival analysis.
Proprietary code was used for k-fold-cross validation, ROC analysis and plot functions.
Each dataset is represented in a proprietary data object, called "Annotated Data Matrix" (ADM). This data object contains the measurements after quality control and averaging, as well as all necessary annotations for the samples and assays.

QM Assay calibration curves

A series of mixtures of methylated MDA-DNA and unmethylated MDA-DNA, ranging from 0 to 100 percent methylated, were included in triplicate on each QM PCR plate. These DNAs were used to ensure uniform QM assay performance on all PCR plates. All assays showed strong quantitative abilities between 10 and 100%, and some assays were able to consistently distinguish 5% methylated DNA from unmethylated DNA.

Statistical Methods

After quality control, each assay was statistically analyzed.

Cox Regression

The relation between recurrence-free survival times (RFS) and covariates were analyzed using Cox Proportional Hazard models (Cox and Oates 1984; Harrel 2001).

The hazard, i.e. the instantaneous risk of a relapse, is modeled as

$$h(t \mid x) = h_0\,(t) \cdot exp(\beta x) \qquad (3)$$

and

$$h(t \mid x_1,\ldots,x_k) = h_0\,(t) \cdot exp(\beta_1 x_1 + \ldots + \beta_k x_k) \qquad (4)$$

for univariate and multiple regression analyses, respectively, where k is 10, m is 100t is the time measured in months after surgery, $h_0(t)$ is the (unspecified) baseline hazard, $x_i$ are the covariates (e.g. measurements of the assays) and $\beta_i$ are the regression coefficients (parameters of the model). $\beta_i$ will be estimated by maximizing the partial likelihood of the Cox Proportional Hazard model

Likelihood ratio tests are performed to test whether methylation is related to the hazard. The difference between 2Log(Likelihood) of the full model and the null-model is approximately $\chi^2$-distributed with $k$ degrees of freedom under the nuii hypotheses $\beta_1 = ... = \beta_k = 0$.

The assumption of proportional hazards wre evaluated by scaled Schoenfeld residuals (Themau and Grambsch 2000). For the calculation, analysis and diagnostics of the Cox Proportional Hazard Model the R functions "coxph" and "coxph.zph" of the "survival" package are used.

Stepwise Regression Analysis

For multiple Cox regression models a stepwise procedure (Venables and Ripley 1999; Harrel 2001) was used in order to find sub-models including only relevant variables. Two effects are usually achieved by these procedures:

- Variables (methylation rates) that are basically unrelated to the dependent variable (DFS/MFS) are excluded as they do not add relevant information to the model.
- Out of a set of highly correlated variables, only the one with the best relation to the dependent variable is retained.

Inclusion of both types of variables can lead to numerical instabilities and a loss of power. Moreover, the predictor's performance can be low due to over-fitting.

The applied algorithm aims at minimizing the Akaike information criterion (AIC).

The AIC is related to the performance of a model, smaller values promise better performance. Whereas the inclusion of additional variables always improves the model fit and thus increases the likelihood, the second term penalizes the estimation of additional parameters. The best model will present a compromise model with good fit and usually a small or moderate number of variables. Stepwise regression calculation with AIC are done with the R function "step".

Kaplan-Meier Survival Curves and Log-Rank Tests

Survival curves were estimated from RFS data using Kaplan-Meier estimator for survival (Kaplan and Meier, 1958). Log-rank tests (Cox and Oates 1984) are used to test for differences of two survival curves, e.g. survival in hyper- vs. hypomethylated groups. In addition, a variant of the Log-rank test usually referred to as the Generalized Wilcoxon test was applied (for description see Hosmer and Lemeshow 1999). For the Kaplan-Meier analysis the functions "survfit" and "survdiff" of the "survival" package are used.

Independence of single markers and marker panels from other covariates

To check whether the present markers give additional and independent information, other relevant clinical factors were included in the Cox Proportional Hazard model and the p-values for the weights for every factor were calculated (Wald-Test) (Thernau *et al.* 2000). For the analysis of additional factors in the Cox Proportional Hazard model, the R function "coxph" is used.

Multiple Test Corrections

No correction for multiple testing was done.

Density Estimation

For numerical variables, kernel density estimation was performed with a Gaussian kernel and variable bandwidth. I he bandwidth is determined using Silverman's "rule-of-thumb" (Silverman 1986). For the calculation of the densities the R function "density" is used.

Analysis of Sensitivity and Specificity

The method of calculating sensitivity and specificity using the Bayes-formula was based on the Kaplan-Meier estimates (Heagerty *et al.* 2000) for the survival probabilities in the marker positive and marker negative groups for a given time $T_{Threshold}$ .The ROCs were calculated for different reference times $T_{Threshold}$ (3 year, 4 years, 5 years, 6 years).

k-fold Crossvalidation

For the analysis of model selection and model robustness k-fold crossvalidation (Hastie *et al.* 2001) was used. The set of observations is randomly split into k chunks. In turn, every chunk was used as a test set, whereas the remaining k-1 chunks constitute the training set. This procedure is repeated m times.

<u>Results</u>

The 605 samples were processed as described above. All samples were analyzed with six marker QM assays with three replicates. The data were filtered for quality control, and analyzed as described in the methods section. The clinical performance of each marker is summarized below and the Kaplan-Meier survival curves and ROC curves according to figures 90 to 95. P-values for comparison of survival curves reported in the graphs are based on the ordinary Log-rank test. The results of using the Generalized Wilcoxon test are essentially the same (data not shown).

The performance of the markers was first examined using the median methylation level as a cut-off. Since this cut-off was fixed before looking at the data, the p values can be used to judge the performance of the markers. Any marker with a significant p value using the median methylation as a cut-off is considered to be validated. The median methylation level might not be the best cut-off for all markers, and for these markers the prognostic separation can be further optimized by choosing the methylation cut-off that results in the lowest p value. Since the cut-off is optimized specifically for p value, the p value no longer can be used to indicate statistical significance.

For judging the significance of the marker performance using the median methylation as a cut-off, we used a p value of 0.005 (assuming correction for 6 comparisons). Based on p-value (less than 0.008) and event separation, PITX2 is the strongest candidate. GPR7along with SEQ ID NO:35. Therefore, these two markers are considered validated markers of post-surgical prostate cancer prognosis., SEQ ID NO: 63 was not significant using the median methylation level as a cut-off (p values 0.018 and 0.0059), but perform well when the methylation cut-off is optimized. See Table 17 for results.

Figure 90A shows the Kaplan-Meier survival analysis of the PITX2 marker of the 585 patient samples that passed the quality control filter using the optimized methylation cut-off value (13.5%). Figure 90B shows the Kaplan-Meier survival analysis of the PITX2 marker using the predefined median methylation value as a cut-off, the p-value was 0.000017. Figure 90C shows the ROC curve analysis of the PITX2 marker after 5 years of follow-up. The median methylation cut-off is marked as a triangle, and the optimized methylation cut-off is shown as a diamond. The AUC was 0.64. Figure 91A shows the Kapian-Meier survivai anaiysis of the GPR7 marker of the 596 patient sampies that passed the quality control filter using the optimized methylation cut-off value (18.06%). Figure 91B shows the Kaplan-Meier survival analysis of said marker using the predefined median methylation value as a cut-off, the p-value was 0.0016. Figure 91C shows the ROC curve analysis of said marker after 5 years of follow-up. The median methylation cut-off is marked as a triangle, and the optimized methylation cut-off is shown as a diamond. The AUC was 0.64.
Figure 92A shows the Kaplan-Meier survival analysis of the SEQ ID NO:63 marker of the 599 patient samples that passed the quality control filter using the optimized methylation cut-off value (5.79%). Figure 92B shows the Kaplan-Meier survival analysis of said marker using the predefined median methylation value as a cut-off, the p-value was 0.018. Figure 92C shows the ROC curve analysis of said marker after 5 years of follow-up. The median methylation cut-off is marked as a triangle, and the optimized methylation cut-off is shown as a diamond. The AUC was 0.60. Figure 93A shows the Kaplan-Meier survival analysis of the SEQ ID NO:35 marker of the 598 patient samples that passed the quality control filter using the optimized methylation cut-off value (36.77%). Figure 93B shows the Kaplan-Meier survival analysis of said marker using the predefined median methylation value as a cut-off, the p-value was 0.059. Figure 93C shows the ROC curve analysis of said marker after 5 years of follow-up. The median methylation cut-off is marked as a triangle, and the optimized methylation cut-off is shown as a diamond. The AUC was 0.61.
Figure 94A shows the Kaplan-Meier survival analysis of the ABHD9 marker of the 592 patient samples that passed the quality control filter using the optimized methylation cut-off value (28.41%). Figure 94B shows the Kaplan-Meier survival analysis of said marker using the predefined median methylation value as a cut-off, the p-value was 0.018. Figure 94C shows the ROC curve analysis of said marker after 5 years of follow-up. The median methylation cut-off is marked as a triangle, and the optimized methylation cut-off is shown as a diamond. The AUC was 0.58.
Figure 95A shows the Kaplan-Meier survival analysis of the CCND2 marker of the 604 patient samples that passed the quality control filter using the optimized methylation cut-off value (2.22%). Figure 95B shows the Kaplan-Meier survival analysis of said marker using the predefined median methylation value as a cut-off, the p-value was 0.22. Figure 95C shows the ROC curve analysis of said marker after 5 years of follow-up. The median methylation cut-off is marked as a triangle, and the optimized methylation cut-off is shown as a diamond. The AUC was 0.61.

Evaluation of Markers on Clinical Subsets of the Patients

Several clinical prognostic factors are commonly used for assessing prostate cancer. Histological analysis of the tumor with quantification of the tumor differentiation state using the Gleason grading system is a particularly important prognostic indicator in current clinical practice. The analysis was continued by determining whether the markers could improve Gleason analysis by subdividing patients within a Gleason category. We also investigated whether the markers could add information to other prognostic indicators, such as nomogram risk estimation (Han *et al.* 2003) and disease stage. For these analyses, we used Kaplan-Meier analysis to determine whether our markers are still informative on population sub-groups, and Cox regression analysis to determine whether the markers provide information independent of the prognostic clinical variables. Gleason score (using Charite Gleason calls) was divided into three groups (6 or lower, 7, and 8 through 10), stage was divided into two groups (T2/organ-confined and T3/non-organ confined), PSA was divided into four groups (0 to 4 ng/ml, 4 to 10 ng/ml, 10 to 20 ng/ml, and greater than 20 ng/ml), and nomogram estimation of 5 year PSA-free survival was divided into two groups (90 to 100% and 0 to 89%).

PITX2

With Cox regression modeling, PITX2 is a valuable prognostic marker independent of other clinical prognostic information (Table 18). In other words, PITX2 methylation adds more information to Gleason than either PSA or disease stage. The hazard ratio for PITX2 is 2.2. In the survival analysis of sub-groups, PITX2 has the potential to be a significant marker for all prostate cancer patients. It is particularly interesting to see strong separation within the patient sub-group with organ-confined disease (Figure 96). Patients with organ-confined disease (T2) should be cured by surgery. Those that are not cured by surgery must have had some cells leave the prostate before surgery, and therefore had tumor cells with aggressive characteristics early in the development. PITX2 can separate the T2 group into a hypomethylated group with a very small chance for recurrence (~5%) and a hyper-methylated group with a prognosis more like T3 patients.
Figure 96 shows the survival analysis of PITX2 performance on sub-populations based on stage. The upper left plot shows the performance of disease stage as a prognostic marker. The upper right plot shows the performance of PITX2 on pT2 patients. The lower left plot shows the performance of PITX2 on pT3 patients.
PITX2 is also capable of stratifying patients within Gleason sub-categories. Figure 97 shows that survival analysis on low Gleason patients (Score 5 or 6) and high Gleason patients (Score 8, 9, or 10) results in low p values. Patients with high Gleason scores are currently candidates for clinical trials on post-surgical adjuvant therapies. But the PITX2 values suggest that this is not a uniform group. PITX2 hypomethylated, high Gleason patients have 85% probability of disease free survival at ten years, while hypermethylated high Gleason patients have a very low chance (~35%). These patients with high likelihood for disease recurrence are the patients who should be selected for adjuvant therapy or clinical trials.
Figure 97 shows the survival analysis of PITX2 performance on sub-populations based on Gleason score categories. The upper left plot shows the performance of Gleason score as a prognostic marker. Gleason 5 and 6 patients are marked A, Gleason 7 patients are marked B, and Gleason 8, 9, and 10 patients are marked C. The upper right plot shows the performance of PITX2 on Gleason 5 and 6 patients. The lower left plot shows the performance of PITX2 on Gleason 7 patients. The lower right plot shows the performance of PITX2 on Gleason 8, 9, and 10 patients.
Prostate cancer nomograms are created based on large cohorts of patients. They mathematically combine information from stage, Gleason, and pre-operative PSA levels into one prognostic indicator. As Figure 98 shows, the nomogram by itself is very strong. But PITX2 is capable of further sub-dividing the patients.
Figure 98 shows the survival analysis of PITX2 performance on sub-populations based on nomogram risk estimation. The upper left plot shows the performance of the nomogram as a prognostic marker. The upper right plot shows the performance of PITX2 on patients with a 90% chance of 5-year PSA-free survival according to the nomogram. The lower left plot shows the performance of PITX2 on patients with less than 90% chance of 5-year PSA-free survival according to the nomogram.

SEQ ID NO:63

With Cox regression analysis, SEQ ID NO:63 is a valuable prognostic marker independent of other clinical prognostic information (Table 19). The hazard ratio is 2.9. In the survival analysis of sub-groups, SEQ ID NO:63 seems to have the potential to be a significant marker for some sub-groups, such as high Gleason patients (Figure 99) and patients with poor nomogram-based prognosis (Figure 100).
Figure 99 shows the survival analysis of SEQ ID NO:63 performance on Gleason score 8, 9, and 10 patients.
Figure 100 shows the survival analysis of SEQ ID NO:63 performance on patients with less than 90% chance of 5-year PSA-free survival according to the nomogram.
SEQ ID NO:35 is a marker for some sub-groups, such as pT2 patients (Figure 101).

Discussion

PITX2, SEQ ID NO:35, and GPR7 all show significant prognostic information when the median methylation level is used as a cut-off. Setting the methylation cut-off even higher than the median improves the performance of these three markers. This has the effect of decreasing the marker positive group and increasing the specificity of the test. The median methylation level is not optimal for SEQ ID NO: 63. Instead, a lower cut-off more clearly separates the good and bad prognosis groups for this marker. The optimized methylation cut-off values for these four markers all fall in the range for which their respective assays are technically well suited.
The patients whose samples were analyzed in this study are representative of the population who would be targeted for a prostatectomy test. Therefore, it is possible to speculate on the information these markers could provide for future patients. PITX2, for example, has a sensitivity of around 60% and a specificity of 70%. In the Kaplan-Meier analysis in Figure 90, the marker positive group has approximately three times the risk of recurrence after ten years that the marker negative group has. In Figure 97, Gleason 8-10 patients that are positive for PITX2 have a 65% chance for PSA recurrence in 10 years. In contrast, the Gleason 8-10 patients who were marker negative had only a 15% chance of PSA relapse. The addition of the methylation marker information to the Gleason stratification will allow clinicians to identify a poor prognosis sub-group who can most benefit from adjuvant therapy. If these methylation markers are incorporated into the patient selection procedure for adjuvant therapy clinical trials, clinicians may begin to see a clear benefit to the addition of early adjuvant treatments for poor prognosis patients.

In addition to adding information to Gleason, PITX2 and some of the other markers can also stratify patients with organ-confined disease. Patients with disease that is truly confined to the organ will be cured by complete removal of the organ. Patients with disease that appears to be confined to the organ, but have undetected micrometastases, will not be cured by surgery. These two groups of patients, both with small operable lesions, have tumors with very different capacities for metastases. PITX2 and some of the other markers seem to be detecting these underlying differences in basic tumor aggressiveness. The ability of these markers to add information to currently used markers is essential. Gleason and staging already provide significant prognostic information, a new test that would not replace but complement these traditional sources of information is both more valuable and more likely to be readily adopted in clinical practice.

In the analysis of the markers on sub-groups of patients, the markers often seemed strongest on patients with poor prognosis based on traditional clinical variables. Gleason 8-10 patients and patients with low nomogram probability for PSA free survival are well stratified by the present markers into good and poor prognosis groups. For a prostatectomy test, these are the ideal patients to target, since the test would be used to select a group of poor prognosis patients who can most benefit from adjuvant therapy. For T3 patients (non-organ-confined disease), the marker SEQ ID NO: 63 is preferred. Overall, this analysis demonstrates that the present markers are especially well suited for identifying poor prognosis patients.

**Example 6: Test of assays on paraffin embedded tissue.**

In the following analysis, methylation within paraffin embedded prostate tissue samples was analysed by means of the assays shown in Table 12 for the analysis of SEQ ID NO: 19, 35, 37 and 63 . This was then compared to the same measurement carried out upon the frozen samples described in Example 2.

Samples

The samples were paraffin embedded prostatectomy samples or fresh frozen tissues as described in Example 3. The samples were sectioned, the tissue was lysed, the DNA was then extracted and bisulfite converted.

309 paraffin embedded samples were available, of these all samples with at least 1 ng of DNA per PCR were included in the analysis, with between 1 and 10ng of DNA per PCR being used.

Reagents:
1 x Taqman PCR Buffer A
0.25mM dNTPs
3.5mM $MgCl_2$
900nM each primer
300nM probe
1 unit AmpliTaq Gold
Thermal Cycling profile:
Step 1. 95˚C->10min (Taq Activation)
Repititions:1
Step 2. 95˚C->15s (Denaturation)
63,0˚C->1 min (Annealing/Extension)
Repititions:50
The following categories were compared

1. Early biochemical relapse (PSA relapse after prostatectomy in less than 24 months) vs. no biochemical relapse (no significant rise in PSA during at least 4 years of PSA monitoring after surgery)
132 samples were available in the non-relapse group and 59 samples available in the early relapse group.

2. High Gleason (score =8-10) vs Low Gleason (Score =2-6 with no grade 4 or 5 component) 59 samples were available in the high Gleason group and 64 samples available in the low Gleason group.

## Results

Results are shown in Figures 102 to 125, and were calculated using the Wilcoxon test as described above. Figure 102 shows the detected amplificate in both frozen and PET samples in the early biochemical relapse vs. no biochemical relapse comparisons using the assay of SEQ ID NO:19 shown in Table 12.
Figure 103 shows the detected amplificate in both frozen and PET samples in the early biochemical relapse vs. no biochemical relapse comparisons using the assay of SEQ ID NO:63 shown in Table 12.
Figure 104 shows the detected amplificate in both frozen and PET samples in the early biochemical relapse vs. no biochemical relapse comparisons using the assay of SEQ ID NO:35 shown in Table 12.
Figure 105 shows the detected amplificate in both frozen and PET samples in the early biochemical relapse vs. no biochemical relapse comparisons using the assay of SEQ ID NO:37 shown in Table 12. Figure 106 shows the detected amplificate in PET samples only in the early biochemical relapse vs. no biochemical relapse comparisons using the assay of SEQ ID NO:19 shown in Table 12.
Figure 107 shows the detected amplificate in PET samples only in the early biochemical relapse vs. no biochemical relapse comparisons using the assay of SEQ ID NO:63 shown in Table 12.
Figure 108 shows the detected amplificate in PET samples only in the early biochemical relapse vs. no biochemical relapse comparisons using the assay of SEQ ID NO:35 shown in Table 12.
Figure 109 shows the detected amplificate in PET samples only in the early biochemical relapse vs. no biochemical relapse comparisons using the assay of SEQ ID NO:37 shown in Table 12.
Figure 110 shows the detected amplificate in frozen samples only in the early biochemical relapse vs. no biochemical relapse comparisons using the assay of SEQ ID NO:19 shown in Table 12.
Figure 111 shows the detected amplificate in frozen samples only in the early biochemical relapse vs. no biochemical relapse comparisons using the assay of SEQ ID NO:63 shown in Table 12.
Figure 112 shows the detected amplificate in frozen samples only in the early biochemical relapse vs. no biochemical relapse comparisons using the assay of SEQ ID NO:35 shown in Table 12.
Figure 113 shows the detected amplificate in frozen samples only in the early biochemical relapse vs. no biochemical relapse comparisons using the assay of SEQ ID NO:37 shown in Table 12.
Figure 114 shows the detected amplificate in both frozen and PET samples in the High Gleason vs. Low Gleason comparisons using the assay of SEQ ID NO:19 shown in Table 12.
Figure 115 shows the detected amplificate in both frozen and PET samples in the High Gleason vs. Low Gleason comparisons using the assay of SEQ ID NO:63 shown in Table 12.
Figure 116 shows the detected amplificate in both frozen and PET samples in the High Gleason vs. Low Gleason comparisons using the assay of SEQ ID NO:35 shown in Table 12.
Figure 117 shows the detected amplificate in both frozen and PET samples in the High Gleason vs. Low Gleason comparisons using the assay of SEQ ID NO:37 shown in Table 12.
Figure 118 shows the detected amplificate in PET samples only in the High Gleason vs. Low Gleason comparisons using the assay of SEQ ID NO:19 shown in Table 12.
Figure 119 shows the detected amplificate in PET samples only in the High Gleason vs. Low Gleason comparisons using the assay of SEQ ID NO:63 shown in Table 12.
Figure 120 shows the detected amplificate in PET samples only in the High Gleason vs. Low Gleason comparisons using the assay of SEQ ID NO:35 shown in Table 12.
Figure 121 shows the detected amplificate in PET samples only in the High Gleason vs. Low Gleason comparisons using the assay of SEQ ID NO:37 shown in Table 12.
Figure 122 shows the detected amplificate in frozen samples only in the High Gleason vs. Low Gleason comparisons using the assay of SEQ ID NO:19 shown in Table 12.
Figure 123 shows the detected amplificate in frozen samples only in the High Gleason vs. Low Gleason comparisons using the assay of SEQ ID NO:63 shown in Table 12.
Figure 124 shows the detected amplificate in frozen samples only in the High Gleason vs. Low Gleason comparisons using the assay of SEQ ID NO:35 shown in Table 12.
Figure 125 shows the detected amplificate in frozen samples only in the High Gleason vs. Low Gleason comparisons using the assay of SEQ ID NO:37 shown in Table 12.

**Claims**

**1.** A method for providing a prognosis of a subject with a prostate cell proliferative disorder, comprising the steps of:

a) providing a biological sample from said subject;
b) determining the expression status of the gene PITX2 in said sample; and
c) determining the prognosis of said subject based on said expression.

**2.** The method according to claim1, wherein at least one further prognostic variable is factored in when determining the prognosis of c).

**3.** The method according to claim 2, wherein said prognostic variable is selected from the group consisting of nomogram, PSA level and Gleason score.

**4.** The method according to any of claims 1 to 3, wherein said sample is selected from the group consisting of cells or cell lines, histological slides, biopsies, paraffin-embedded tissue, bodily fluids, ejaculate, urine, blood, and combinations thereof.

**5.** The method according to any of claims 1 to 4, wherein the expression is determined by measuring the level of at least one of mRNA, cDNA or polypeptide.

**6.** The method according to claim 5, wherein the expression is determined by use of at least one technique selected from the group consisting of Northern blot analysis, reverse transcriptase PCR, real-time PCR, RNAse protection, and microarray analysis.

**7.** A kit for use in providing a prognosis of a subject with a prostate cell proliferative disorder, comprising a means for detecting the polypeptides of the gene PITX2.

**8.** A kit for use in providing a prognosis of a subject with a prostate cell proliferative disorder, comprising a means for measuring the level of mRNA transcription of the gene PITX2.

**9.** Use of a method according to any of claims 1 to 6, or a kit according to any of claims 7 to 9 for providing the prognosis of a subject with a prostate cell proliferative disorder.

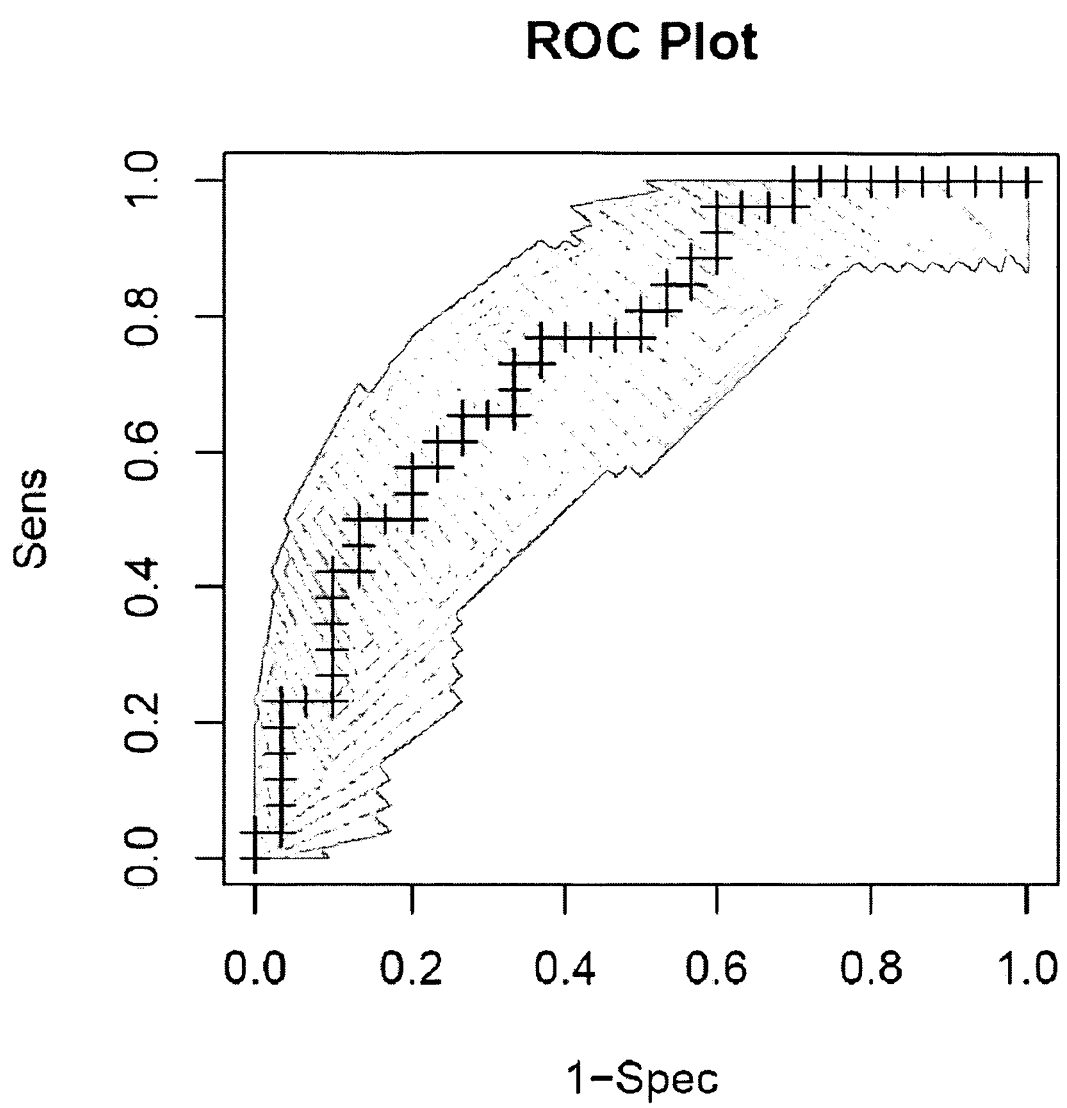


**Figure 1**

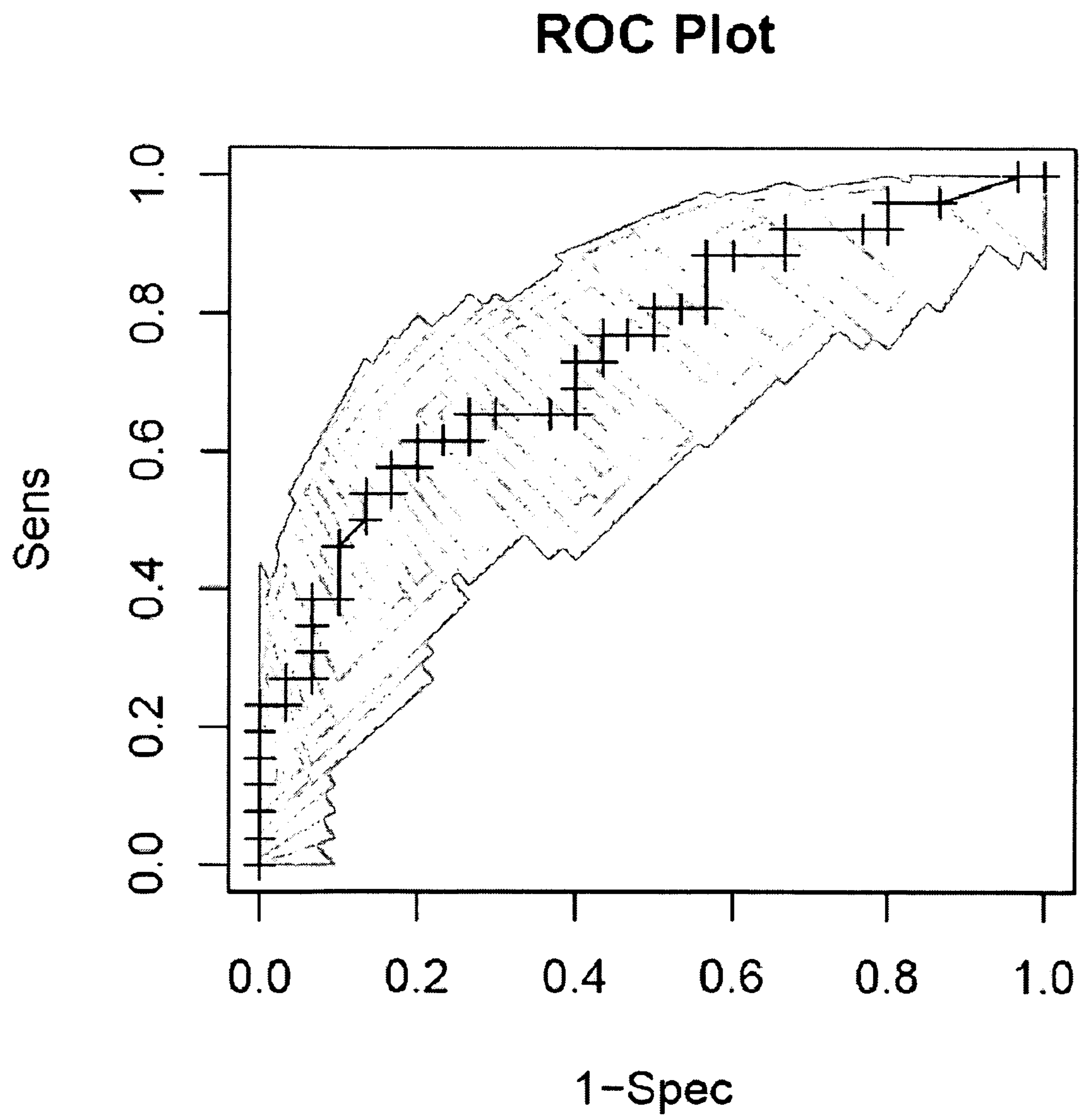


**Figure 2**

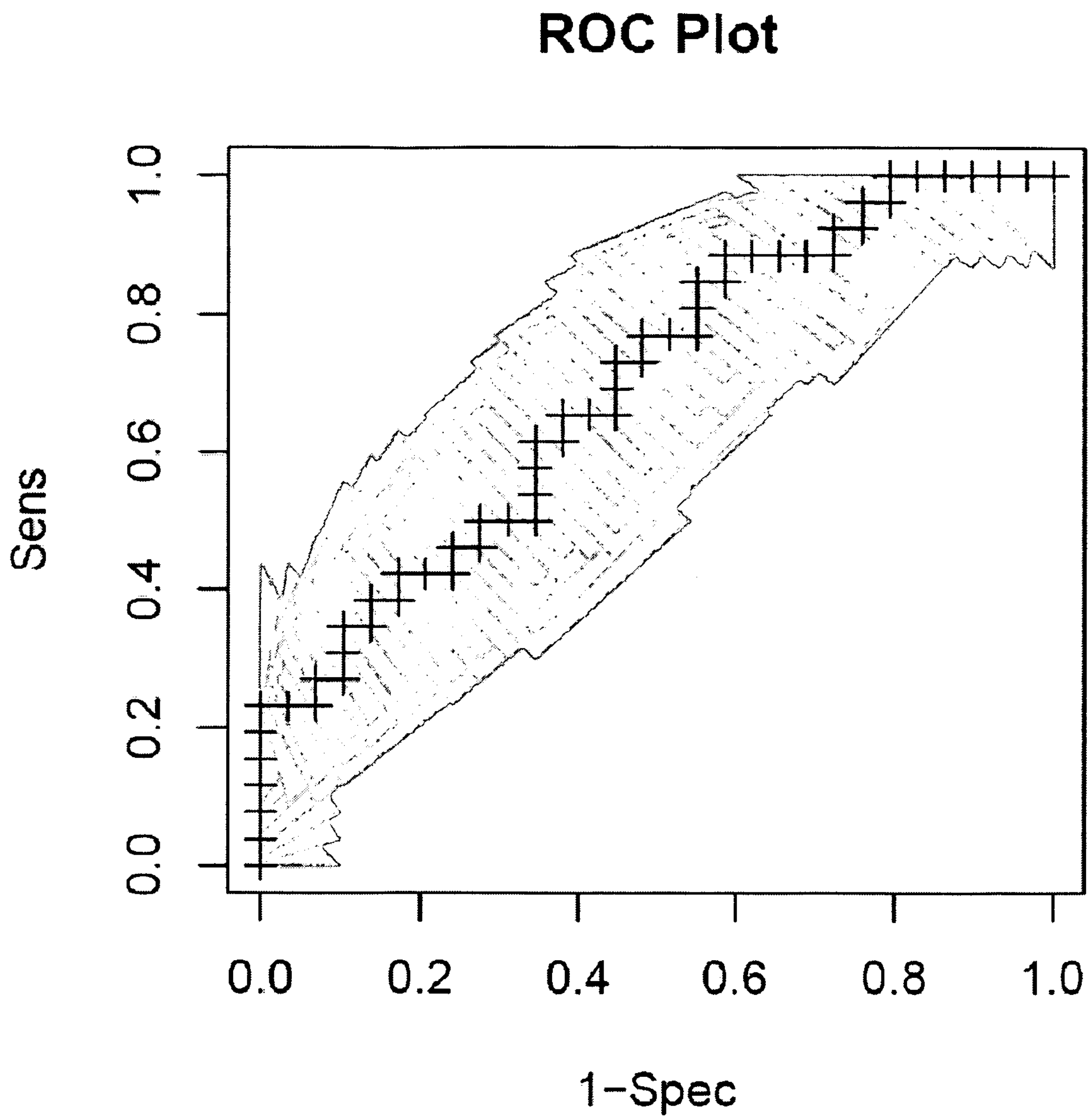


Figure 3

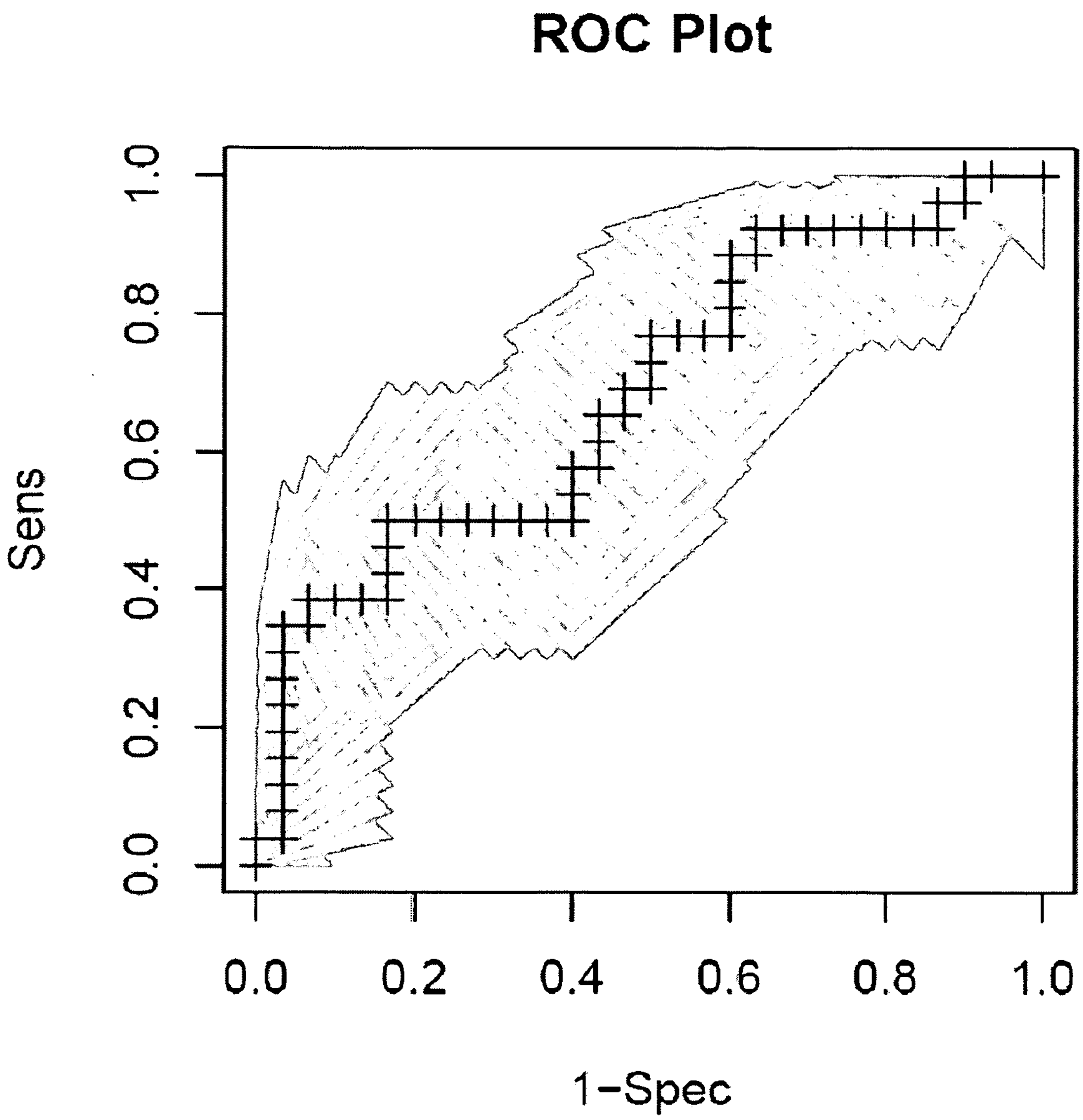


**Figure 4**

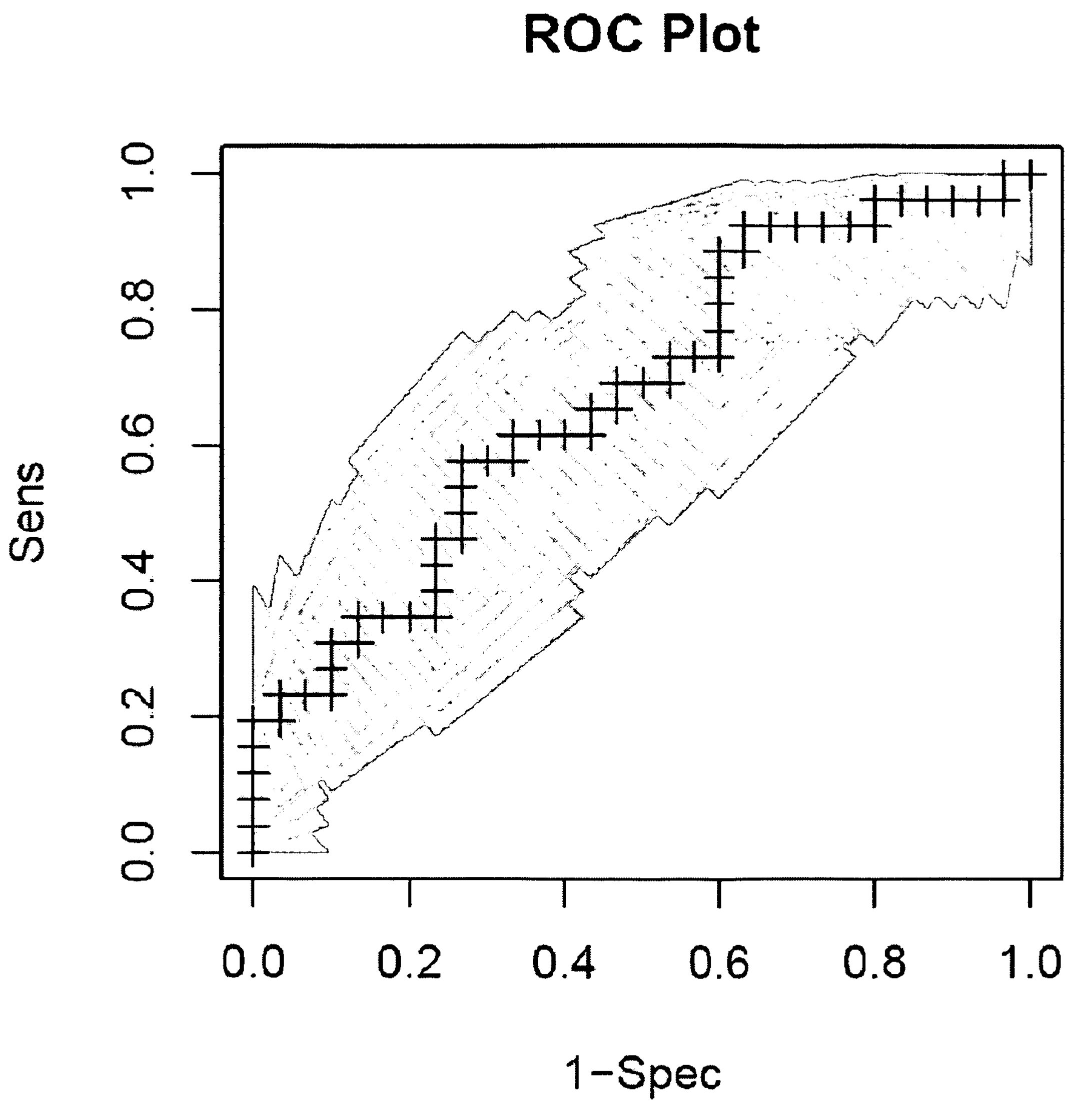


Figure 5

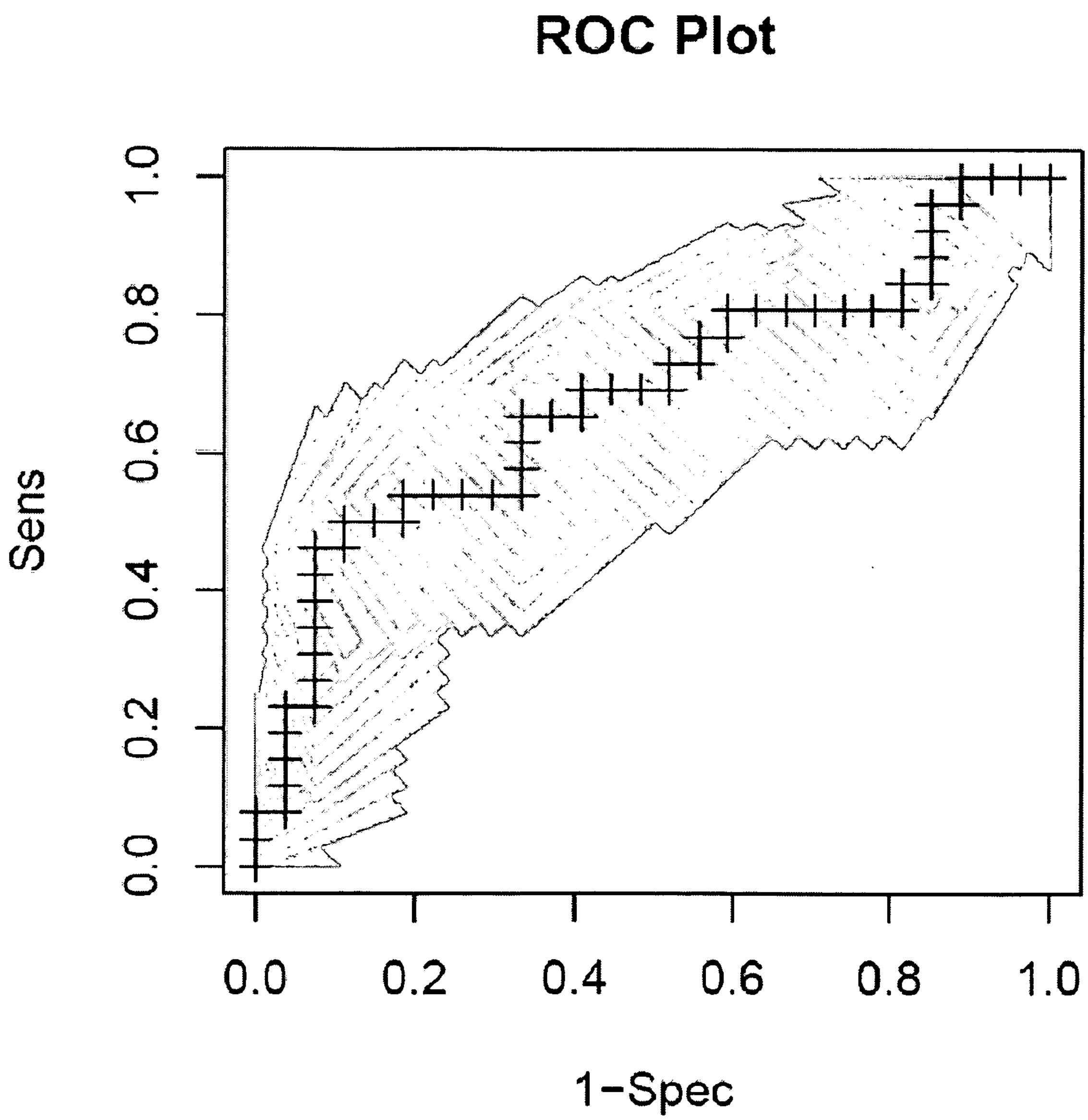


Figure 6

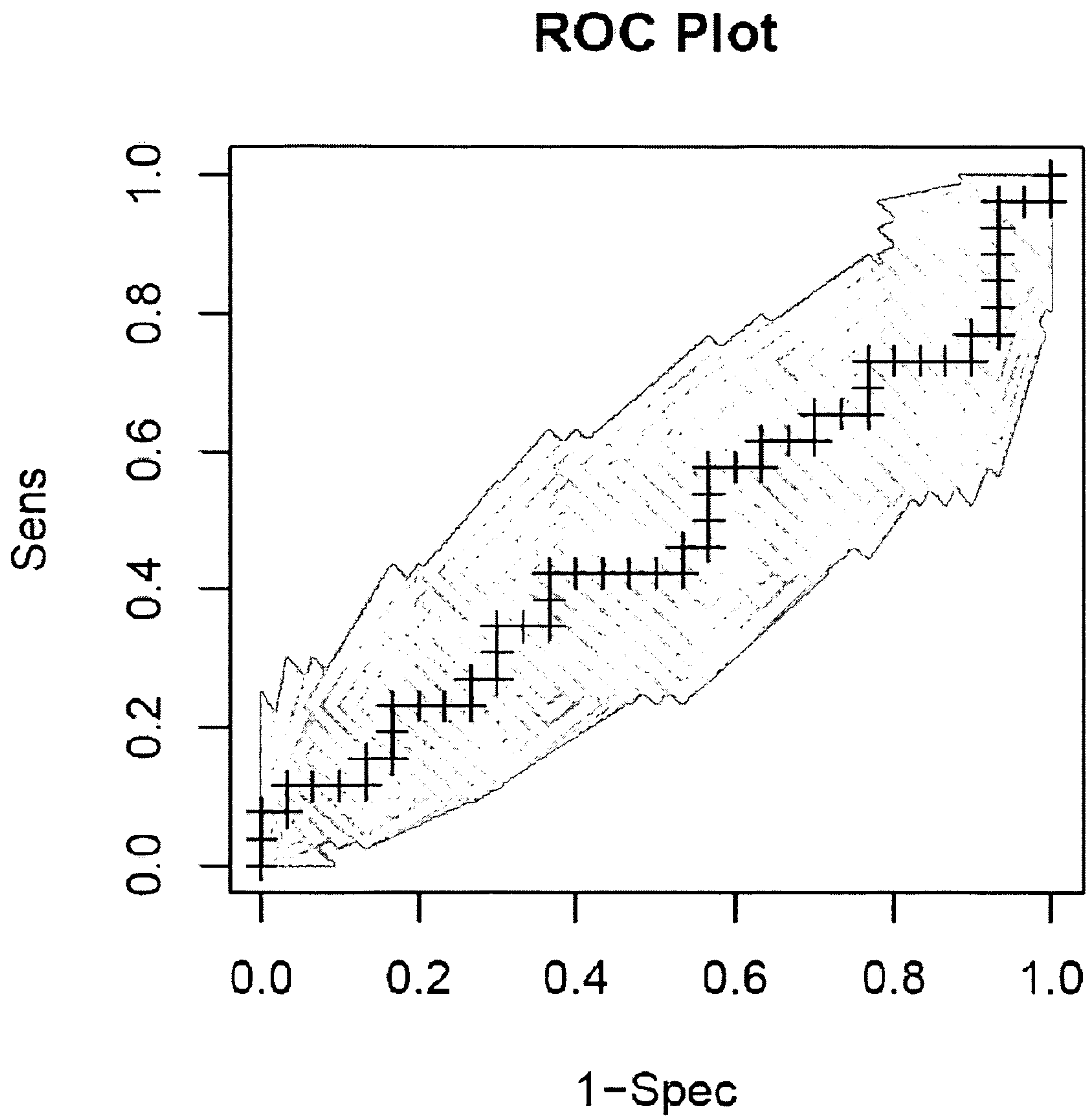


Figure 7

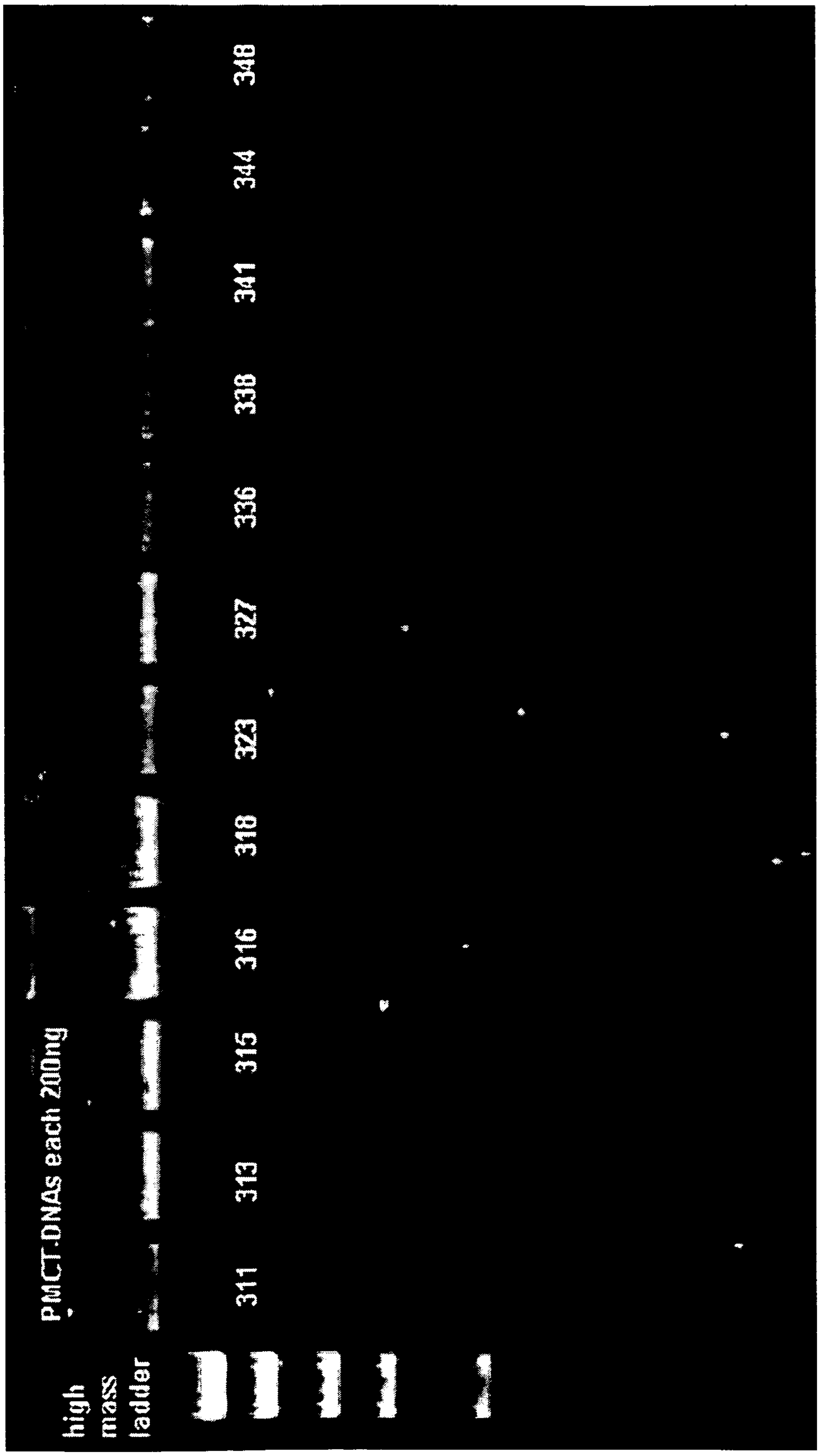
high mass ladder
PMCT-DNAs each 200ng
311
313
315
316
318
323
327
336
338
341
344
348

Figure 8

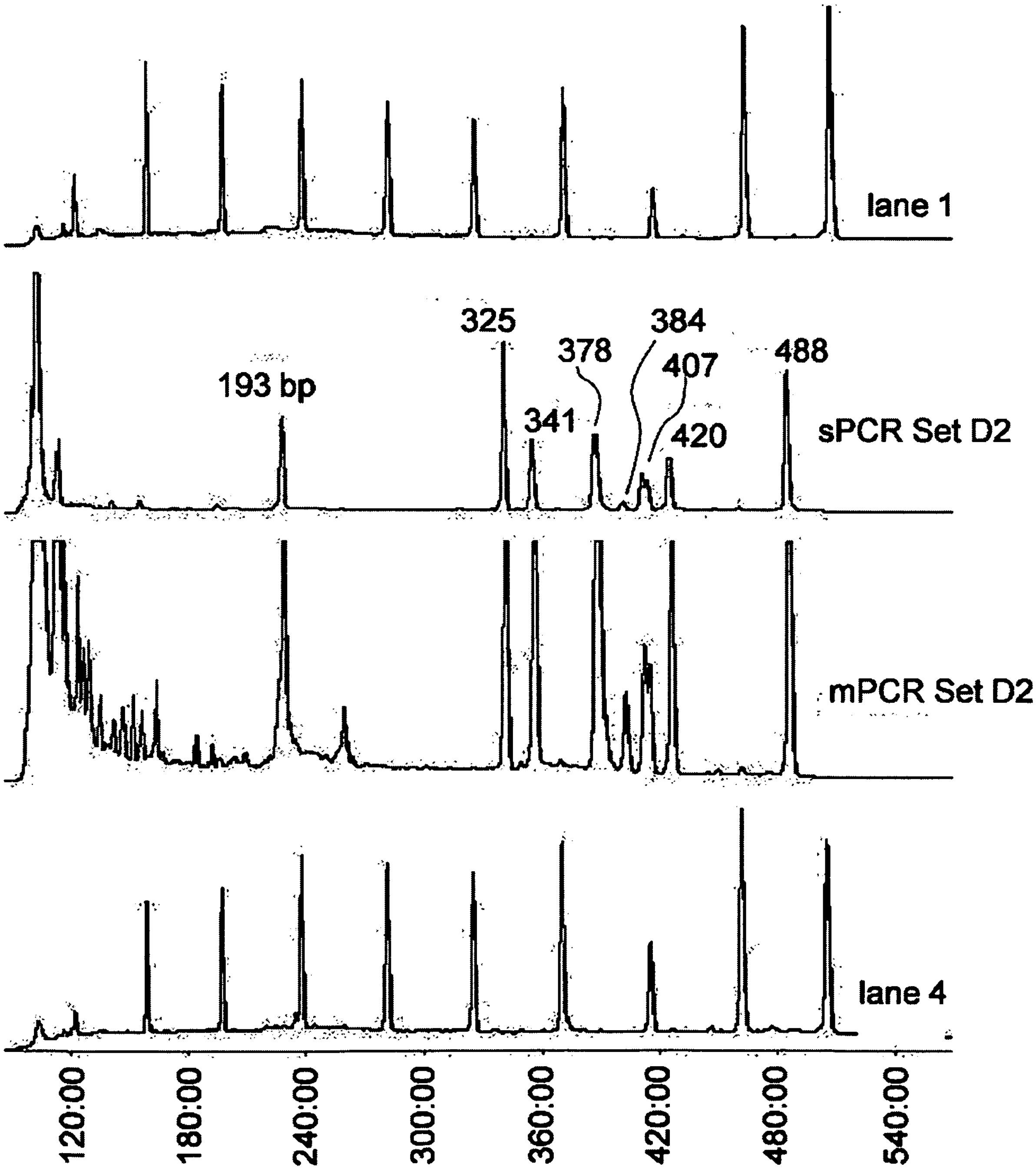
lane 1
193 bp
325
341
378
384
407
420
488
sPCR Set D2
mPCR Set D2
lane 4
120:00
180:00
240:00
300:00
360:00
420:00
480:00
540:00

Figure 9

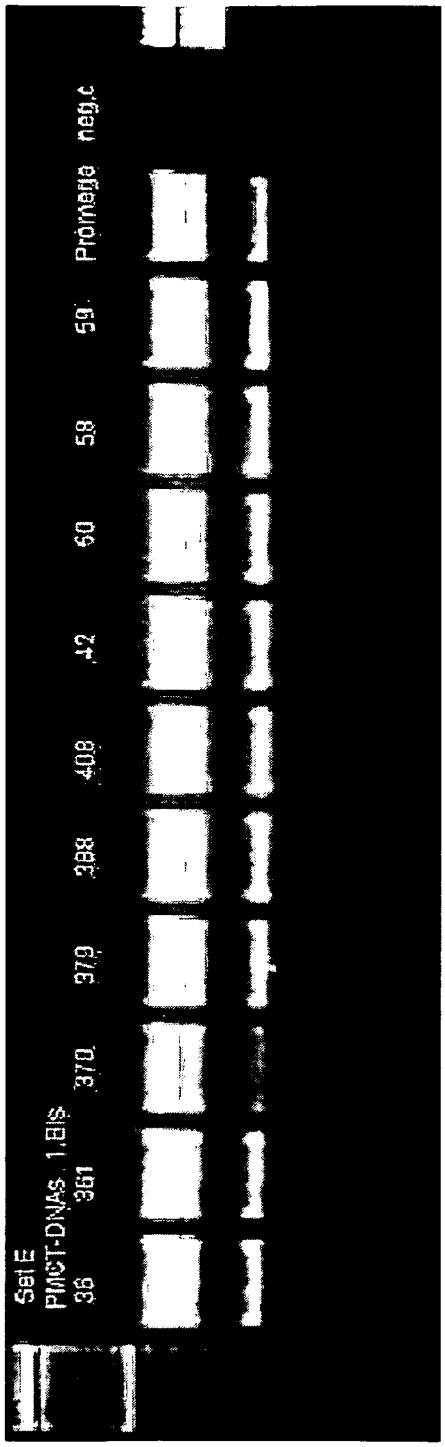
Set E
PMCT-DNAs 1.Bis
36
361
370
379
388
408
42
60
58
59
Promega
neg.c

Figure 10

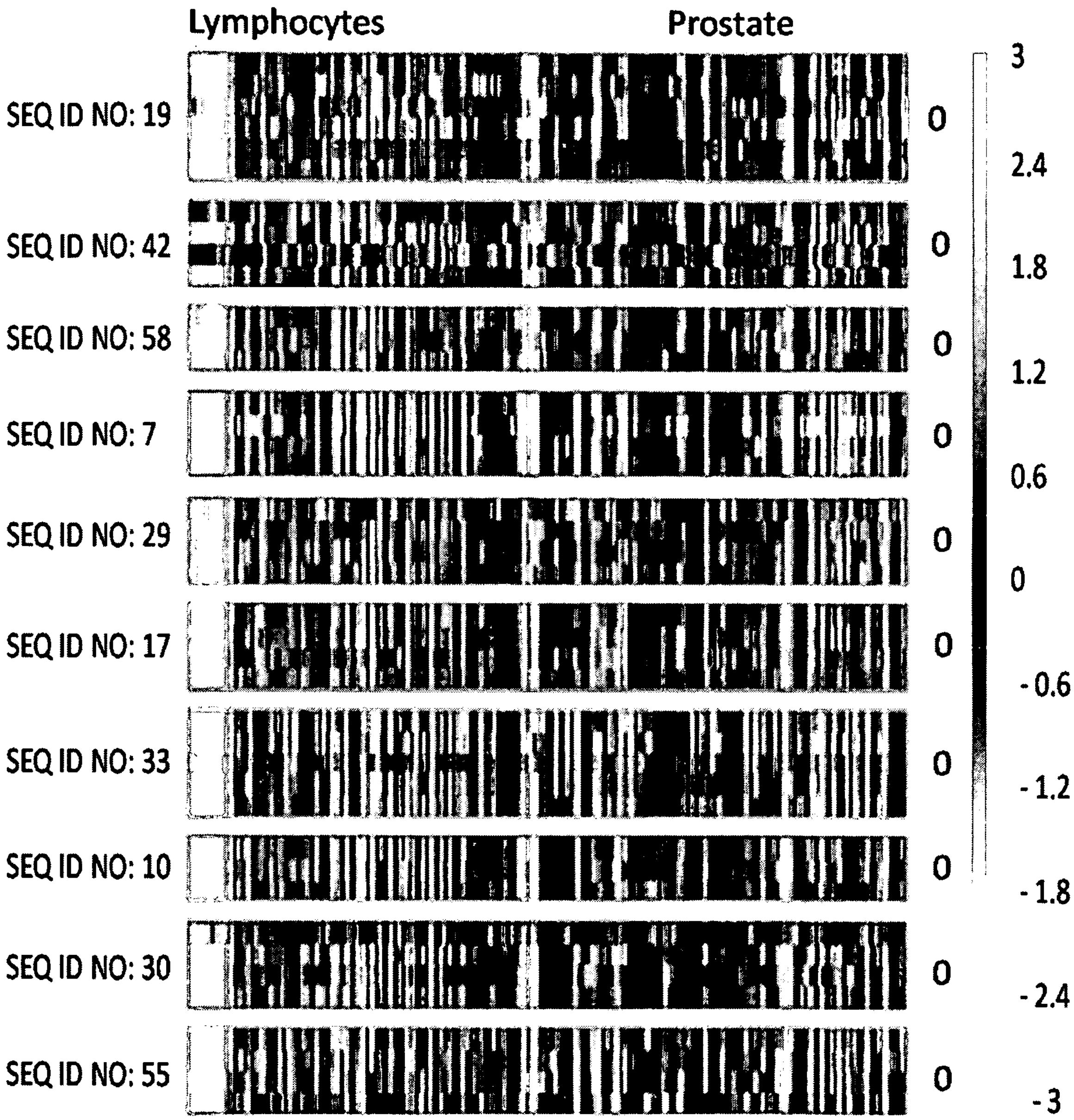
Lymphocytes
Prostate
SEQ ID NO: 19
SEQ ID NO: 42
SEQ ID NO: 58
SEQ ID NO: 7
SEQ ID NO: 29
SEQ ID NO: 17
SEQ ID NO: 33
SEQ ID NO: 10
SEQ ID NO: 30
SEQ ID NO: 55
0
0
0
0
0
0
0
0
0
0
3
2.4
1.8
1.2
0.6
0
- 0.6
- 1.2
- 1.8
- 2.4
- 3

Figure 11

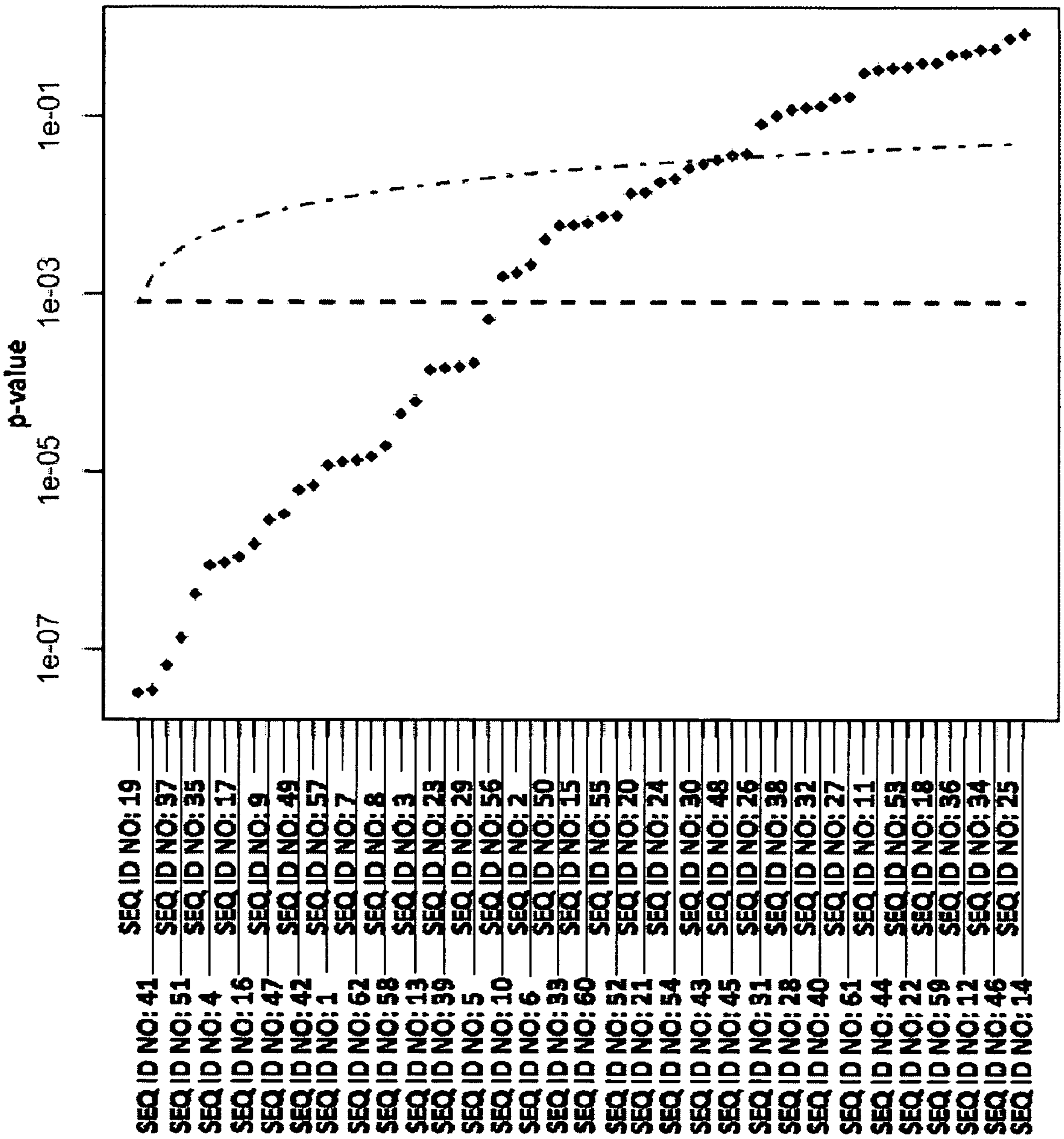
p-value
1e-01
1e-03
1e-05
1e-07
SEQ ID NO: 19
SEQ ID NO: 41
SEQ ID NO: 37
SEQ ID NO: 51
SEQ ID NO: 35
SEQ ID NO: 4
SEQ ID NO: 17
SEQ ID NO: 16
SEQ ID NO: 9
SEQ ID NO: 47
SEQ ID NO: 49
SEQ ID NO: 42
SEQ ID NO: 57
SEQ ID NO: 1
SEQ ID NO: 7
SEQ ID NO: 62
SEQ ID NO: 8
SEQ ID NO: 58
SEQ ID NO: 3
SEQ ID NO: 13
SEQ ID NO: 23
SEQ ID NO: 39
SEQ ID NO: 29
SEQ ID NO: 5
SEQ ID NO: 56
SEQ ID NO: 10
SEQ ID NO: 2
SEQ ID NO: 6
SEQ ID NO: 50
SEQ ID NO: 33
SEQ ID NO: 15
SEQ ID NO: 60
SEQ ID NO: 55
SEQ ID NO: 52
SEQ ID NO: 20
SEQ ID NO: 21
SEQ ID NO: 24
SEQ ID NO: 54
SEQ ID NO: 30
SEQ ID NO: 43
SEQ ID NO: 48
SEQ ID NO: 45
SEQ ID NO: 26
SEQ ID NO: 31
SEQ ID NO: 38
SEQ ID NO: 28
SEQ ID NO: 32
SEQ ID NO: 40
SEQ ID NO: 27
SEQ ID NO: 61
SEQ ID NO: 11
SEQ ID NO: 44
SEQ ID NO: 53
SEQ ID NO: 22
SEQ ID NO: 18
SEQ ID NO: 59
SEQ ID NO: 36
SEQ ID NO: 12
SEQ ID NO: 34
SEQ ID NO: 46
SEQ ID NO: 25
SEQ ID NO: 14

Figure 12

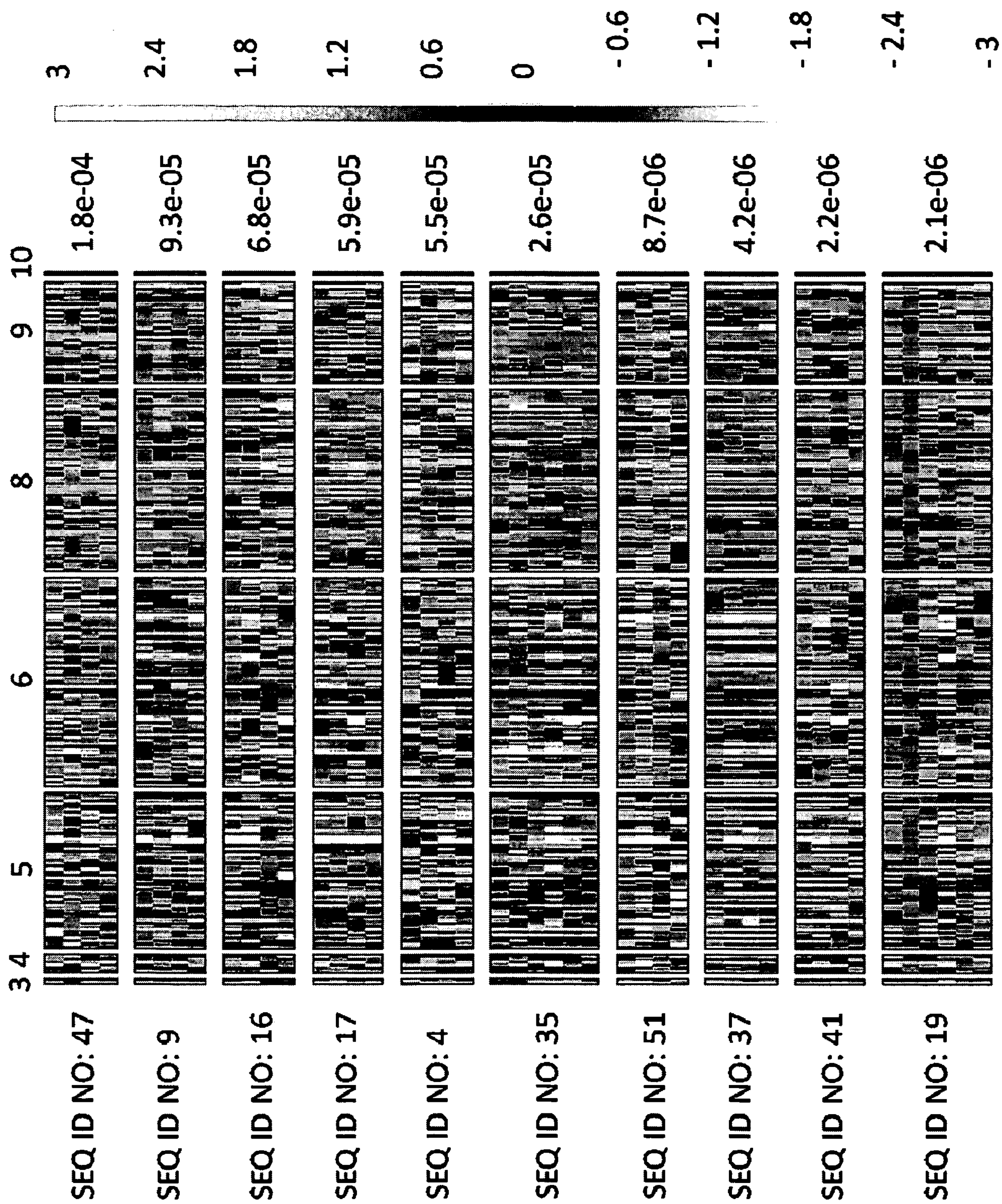
3
2.4
1.8
1.2
0.6
0
-0.6
-1.2
-1.8
-2.4
-3
1.8e-04
9.3e-05
6.8e-05
5.9e-05
5.5e-05
2.6e-05
8.7e-06
4.2e-06
2.2e-06
2.1e-06
10
9
8
6
5
3 4
SEQ ID NO: 47
SEQ ID NO: 9
SEQ ID NO: 16
SEQ ID NO: 17
SEQ ID NO: 4
SEQ ID NO: 35
SEQ ID NO: 51
SEQ ID NO: 37
SEQ ID NO: 41
SEQ ID NO: 19

Figure 13

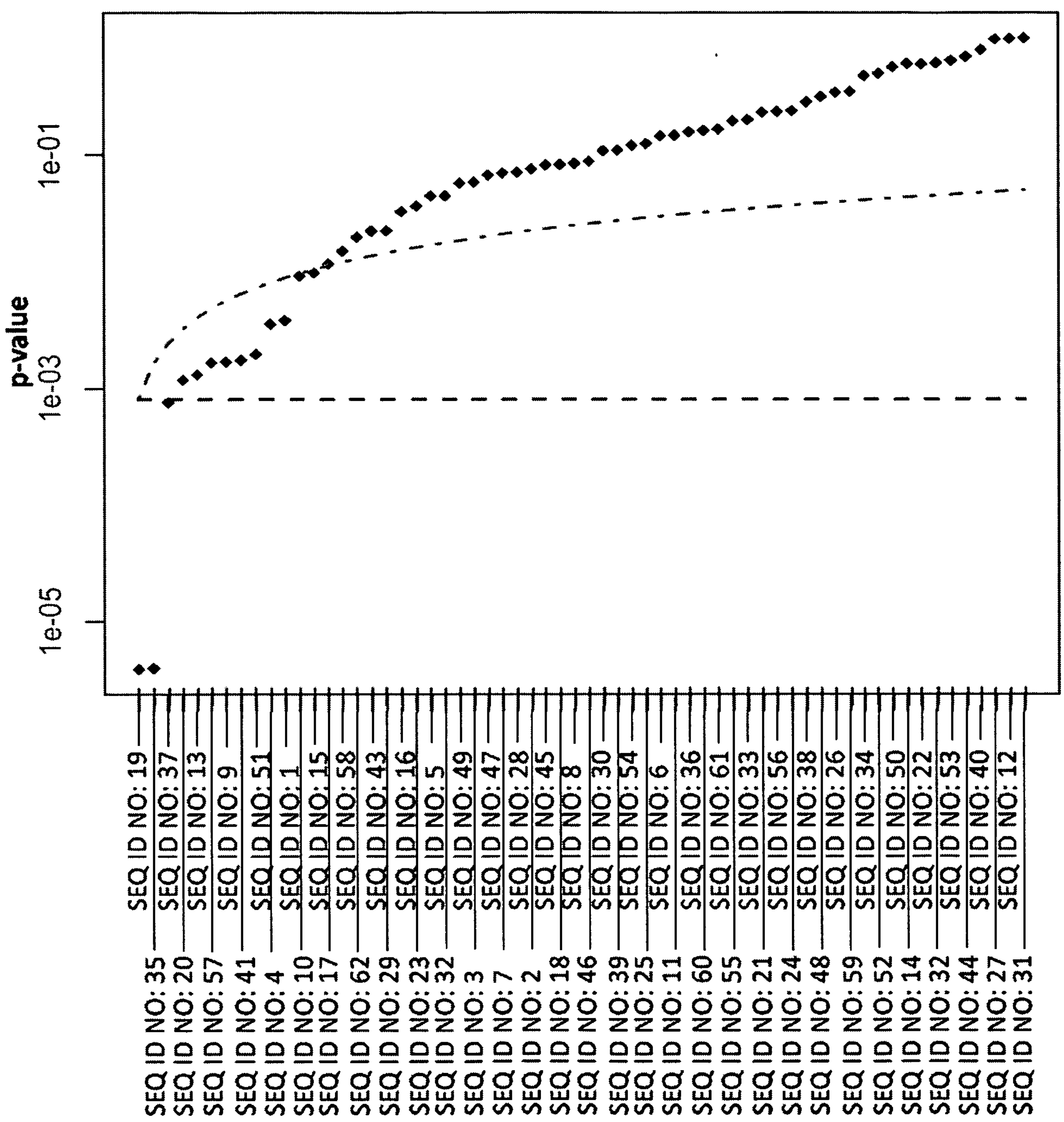
p-value
1e-01
1e-03
1e-05
SEQ ID NO: 19
SEQ ID NO: 35
SEQ ID NO: 37
SEQ ID NO: 20
SEQ ID NO: 13
SEQ ID NO: 57
SEQ ID NO: 9
SEQ ID NO: 41
SEQ ID NO: 51
SEQ ID NO: 4
SEQ ID NO: 1
SEQ ID NO: 10
SEQ ID NO: 15
SEQ ID NO: 17
SEQ ID NO: 58
SEQ ID NO: 62
SEQ ID NO: 43
SEQ ID NO: 29
SEQ ID NO: 16
SEQ ID NO: 23
SEQ ID NO: 5
SEQ ID NO: 32
SEQ ID NO: 49
SEQ ID NO: 3
SEQ ID NO: 47
SEQ ID NO: 7
SEQ ID NO: 28
SEQ ID NO: 2
SEQ ID NO: 45
SEQ ID NO: 18
SEQ ID NO: 8
SEQ ID NO: 46
SEQ ID NO: 30
SEQ ID NO: 39
SEQ ID NO: 54
SEQ ID NO: 25
SEQ ID NO: 6
SEQ ID NO: 11
SEQ ID NO: 36
SEQ ID NO: 60
SEQ ID NO: 61
SEQ ID NO: 55
SEQ ID NO: 33
SEQ ID NO: 21
SEQ ID NO: 56
SEQ ID NO: 24
SEQ ID NO: 38
SEQ ID NO: 48
SEQ ID NO: 26
SEQ ID NO: 59
SEQ ID NO: 34
SEQ ID NO: 52
SEQ ID NO: 50
SEQ ID NO: 14
SEQ ID NO: 22
SEQ ID NO: 32
SEQ ID NO: 53
SEQ ID NO: 44
SEQ ID NO: 40
SEQ ID NO: 27
SEQ ID NO: 12
SEQ ID NO: 31

Figure 14

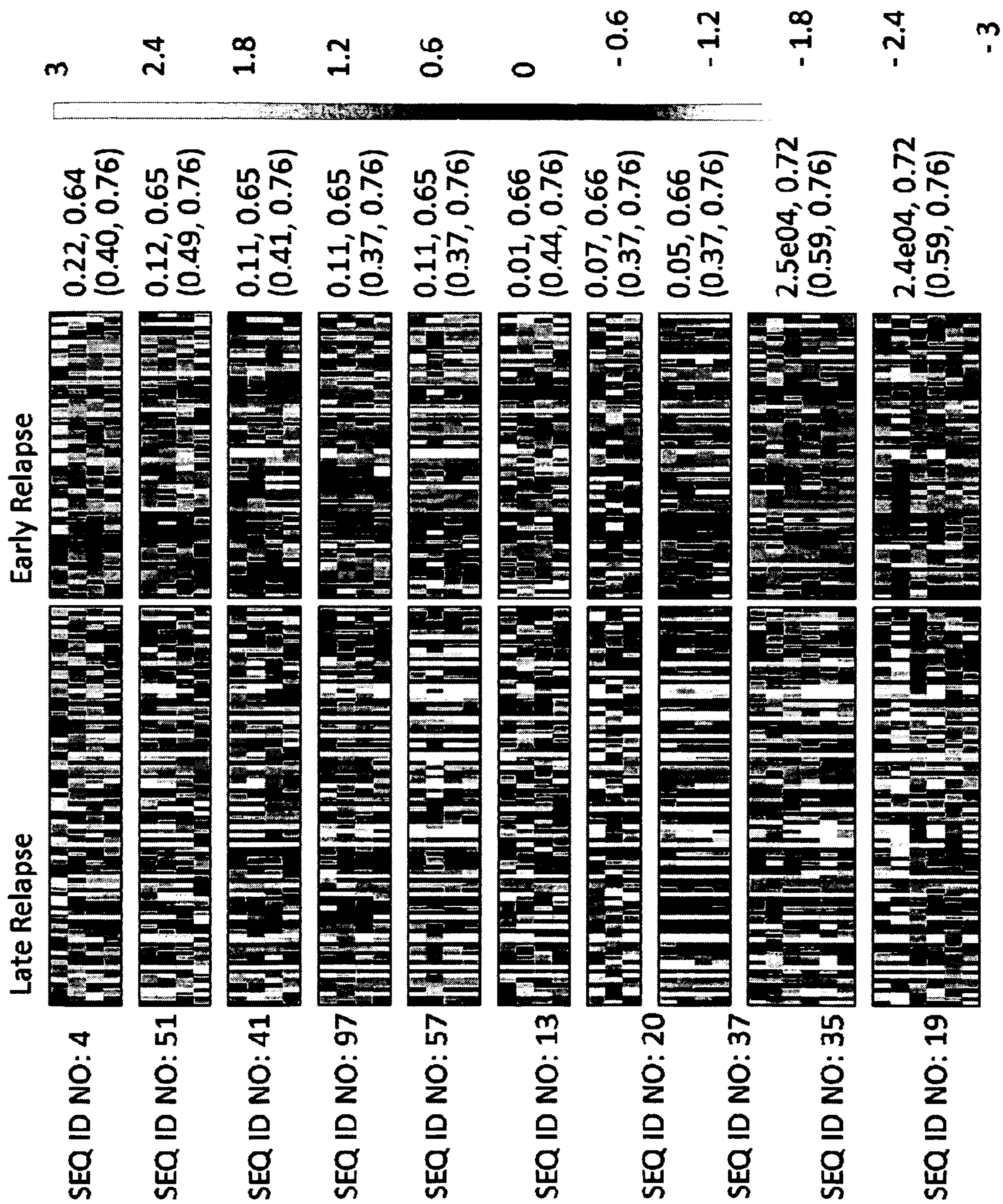
Late Relapse
Early Relapse
3
2.4
1.8
1.2
0.6
0
-0.6
-1.2
-1.8
-2.4
-3
SEQ ID NO: 4
0.22, 0.64 (0.40, 0.76)
SEQ ID NO: 51
0.12, 0.65 (0.49, 0.76)
SEQ ID NO: 41
0.11, 0.65 (0.41, 0.76)
SEQ ID NO: 97
0.11, 0.65 (0.37, 0.76)
SEQ ID NO: 57
0.11, 0.65 (0.37, 0.76)
SEQ ID NO: 13
0.01, 0.66 (0.44, 0.76)
SEQ ID NO: 20
0.07, 0.66 (0.37, 0.76)
SEQ ID NO: 37
0.05, 0.66 (0.37, 0.76)
SEQ ID NO: 35
2.5e04, 0.72 (0.59, 0.76)
SEQ ID NO: 19
2.4e04, 0.72 (0.59, 0.76)

Figure 15

Figure 16 Amplificate of SEQ ID NO: 14

```
  1 GAAGAGGTGCTGAGAAATTAAAAATTCAGGTTAGTTAATGCATCCCTGCCGCCGGCTGCAGGCTCCGCCTTTGCATTAAG  80
  1 GAAGAGGTGTTGAGAAATTAAAA                                                          80
  1 GAAGAGGTGtTGAGAAATTAAAAATTtAGGTTAGTTAATGtATtttTGtCGtCGGtTGtAGGtTtCGttTTTGtATTAAG  80
  1                                                                            TTAAG  80
  1                                                                            TTAAG  80

 81 CGGGCGCTGATTGTGCGCGCCTGGCGACCGCGGGGAGGACTGGCGGCCCGCGGGAGGGGACGGGTAGAGGCGCGGGTTAC 160
 81 CGGGCGtTGATTGTGCGCGttTGGCGAtCGCGGGGAGGAtTGGCGGttCGCGGGAGGGGACGGGTAGAGGCGCGGGTTAt 160
 81 CGGGCGTTGAT                                                   TAGAGGCGCGGGTTAT 160
 81 TGGGTGTTGAT                                                   TAGAGGTGTGGGTTAT 160

161 ATTGTTCTGGAGCCGGCTCGGCTCTTTGTGCCTCCTCTAGCGGCCAAGCTGCGAGGTACAGCCCTCTATTGTTCTAGGAG 240
161 ATTGTTtTGGAGtCGGtTCGGtTtTTTGTGttTttTtTAGCGGttAAGtTGCGAGGTAtAGtttTtTATTGTTtTAGGAG 240

241 CACAGAAACCTCCTGTGTGGGCGGCGGGTGCGCGAGCTAGAGGGAAAGATGCAGTAGTTACTGCGACTGGCACGCAGTTG 320
241 tAtAGAAAttTttTGTGTGGGCGGCGGGTGCGCGAGtTAGAGGGAAAGATGtAGTAGTTAtTGCGAtTGGtACGtAGTTG 320
241                                                         TTGCGATTGGTACGTA     320
241                                                          TGTGATTGGTATGTAGT   320
```

```
321 CGCGCTTTTGTGCGCACGGACCCCGCGCGGTGTGCGTGGCGACTGCGCTGCCCCTAGGAGCAAGCCACGGGCCCAGAGGG 400
321 CGCGtTTTTGTGCGtACGGAtttCGCGCGGTGTGCGTGGCGAtTGCGtTGttttTAGGAGtAAGttACGGGtttAGAGGG 400
321     TTTTTGTGTGTATGGAT                                                           400
321      TTTTGTGCGTACGGAT

401 GCAAAATGTCCAGGTCCCCCGCTGGGAAGGACACACTATACCCTATGGCAAGCCAGGGTGGG                  480
401                                         TTTATGGTAAGTTAGGGTGGG                   480
401 GtAAAATGTttAGGTttttCGtTGGGAAGGAtAtAtTATAtttTATGGtAAGttAGGGTGGG                  480
```

Figure 17: Amplificate of SEQ ID NO: 15

```
  1 TGCAGGAGAGGTTGGGAAGGGGTGGGGGACGGGGCTCGGGGGAGGTCTCCGAGGGACTCTAGTAAGCGGGGAAGGGCGCC  80
  1 TGTAGGAGAGGTTGGGAAG                                                              80
  1 TGtAGGAGAGGTTGGGAAGGGGTGGGGGACGGGGtTCGGGGGAGGTtTtCGAGGGAtTtTAGTAAGCGGGGAAGGGCGtC  80

 81 GGGAAAGTTTCAGATCCACGGCTGCGCGGGCCACGAGCCCACCCGAACGCCGACCACTGCTTTCCGTCGACTTCTATTTC 160
 81 GGGAAAGTTTtAGATttACGGtTGCGCGGGttACGAGtttAttCGAACGtCGAttAtTGtTTTtCGTCGAtTTtTATTTt 160
 81                         GGTTATGAGTTTATTTGAA                                      160
 81                          GTTACGAGTTTATTCGA                                       160

161 CTGGGAACGCGCGAAAGCAAACCCAAGTCAGACTGCGGAGGTCGCTGGGGAGGGAAGGTTCAAGGAGTTCTCGCCGATCC 240
161 tTGGGAACGCGCGAAAGtAAAtttAAGTtAGAtTGCGGAGGTCGtTGGGGAGGGAAGGTTtAAGGAGTTtTCGtCGATtt 240
161 TTGGGAATGTGTGAAA                                                AGGAGTTTTTGTTGATT 240
161     AACGCGCGAAAGTAAA                                                 GAGTTTTCGTCGATTT 240

241 TGCTGAATAAAGGGGGTTCCGAGCTGGGCCGAGATGGGGCATGCGCGGGAAGACCCCTGCCCGCTGTTCCCCCCCACCGC 320
241                                                                                T 320
241 TGtTGAATAAAGGGGGTTtCGAGtTGGGtCGAGATGGGGtATGCGCGGGAAGAttttTGttCGtTGTTtttttttAtCGt 320
241                                        GTATGTGTGGGAAGAT                          320
241                                         TATGCGCGGGAAGATT
```

| | | |
|---|---|---|
| 321 | CCCAGTGGATGCCATGCCTGG | 400 |
| 321 | **TTTAGTGGATGTTATGTTTGG** | 400 |
| 321 | tttAGTGGATGttATGttTGG | 400 |

Figure 18: Amplificate of SEQ ID NO: 16

```
459 GCTTCTAAGTGCTGGGTGATTTCCGCGGGAAGTCCCTGGGGCAGCAGCCAGCATCTGATGCTAGGATCCTATCCATACCA 380
459 GTTTTTAAGTGTTGGGTGATTT                                                           380
459 GtTTtTAAGTGtTGGGTGATTTtCGCGGGAAGTtttTGGGGtAGtAGttAGtATtTGATGtTAGGATttTATttATAttA 380
459                   ATTTTCGCGGGAAGTT                                               380
459                 GTGATTTTTGTGGGAA                                                 380

379 ACCCAGATGTTCCTGCGTTGCAGTAGGTCCACACTGCCGGGGAGAGGTGGCAAGAGACAAATCAACTCTAGTGGAAACGG 300
379 AtttAGATGTTttTGCGTTGtAGTAGGTttAtAtTGtCGGGGAGAGGTGGtAAGAGAtAAATtAAtTtTAGTGGAAACGG 300
379                                 ATATTGTTGGGGAGAG                                 300
379                                TATATTGTCGGGGAGA                                  300

299 GAAGCGAGTGGGAACAACAGCCTCACAGCAAGACCGGGGCTCAGCAGCCGCGCTCTTGGCCTGCGTTTGGTGGACCGGAA 220
299 GAAGCGAGTGGGAAtAAtAGttTtAtAGtAAGAtCGGGGtTtAGtAGtCGCGtTtTTGGttTGCGTTTGGTGGAtCGGAA 220
299                                         AGTAGTTGTGTTTTTGG                  ATTGGAA 220
299                                        TAGTAGTCGCGTTTTT                   GATCGGAA 220

219 TCCGGGTTGTGTGTTTCATTTTCTTTTTTGCCAGAAAATATCATTAGGAACTAAACGGCTCTTTTTAAGAGTGTGAAAGT 140
219 TtCGGGTTGTGTGTTTtATTTTtTTTTTTGttAGAAAATATtATTAGGAAtTAAACGGtTtTTTTTAAGAGTGTGAAAGT 140
219 TTTGGGTTG                                                                        140
219 TTCGGGTT
```

```
139 GTTACTCTTGCCAGTCACCAATGAGGAGGGCGCCAAAAGCTTCCCAGAGCCAGTTCCCACAGCCAGGACTATTCTCTAAA 60
139 GTTAtTtTTGttAGTtAttAATGAGGAGGGCGttAAAAGtTTtttAGAGttAGTTtttAtAGttAGGAtTATTtTtTAAA 60
```

```
59 GCCAGAGGTCGGAAAGTTGAACAGAACGGAAGCAAATTGCCTGGCAGAGGGAAGAGGCA -20
59                                         GTTTGGTAGAGGGAAGAGGTA -20
59 GttAGAGGTCGGAAAGTTGAAtAGAACGGAAGtAAATTGttTGGtAGAGGGAAGAGGtA -20
```

Figure 19: Amplificate of SEQ ID NO: 17

```
1 AAGAGTCCCAGGAAATGTGCCCCCCGGGATTACTGGTATTTGCTGGCTCCTCGGAACAAGATGCCAACTTGGCTAAGCAG 80
1 AAGAGTTTTAGGAAATGTGTTTTT                                                         80
1 AAGAGTtttAGGAAATGTGttttCGGGATTAtTGGTATTTGtTGGtTttTCGGAAtAAGATGttAAtTTGGtTAAGtAG 80
1                                          TTGGTTTTTCGGAATAA                       80
1                                            GGTTTTTTGGAATAAGAT                    80

81 TTCTGGATCTCGGCGTCGATGTATCCCCCTAGCGAATCTCAGCTGGTGCTGCGCAGAGACAGCAGTCAGCGTCTGCCGGT 160
81 TTtTGGATtTCGGCGTCGATGTATtttttTAGCGAATtTtAGtTGGTGtTGCGtAGAGAtAGtAGTtAGCGTtTGtCGGT 160
81         TTCGGCGTCGATGTAT                           GGTGTTGTGTAGAGAT  TAGTTAGTGTTTGTTGG 160
81         TTTGGTGTTGATGTATT                           GTGTTGCGTAGAGATA  AGTTAGCGTTTGTCGG

161 GGCGCGGCCCAGGAGGAGCAGAGGGTCTGGTGAGTAGAA 240
161                  GTAGAGGGTTTGGTGAGTAGAA 240
161 GGCGCGGtttAGGAGGAGtAGAGGGTtTGGTGAGTAGAA 240
```

Figure 20: Amplificate of SEQ ID NO: 18

```
  1 CAAGGCAAGGGAAGGCCAGAAACGAGGCAACCCGTGCACAGACCGGGCTTGGTCAACGCCCCAGGGGCGGGGCCAGGCGG  80
  1 TAAGGTAAGGGAAGGTTAGAAA                                                             80
  1 tAAGGtAAGGGAAGGttAGAAACGAGGtAAttCGTGtAtAGAtCGGGtTTGGTtAACGttttAGGGGCGGGGttAGGCGG  80
  1                                AATTCGTGTATAGATCGG                                80
  1                                 ATTTGTGTATAGATTGGG                               80

 81 GCCGTTCCTGGGGGGCGGGGCCAGCCTCGGTCCAATAAGGGGTACTCGACGCCCCATTGGCCACCTGCCTCGAAAGGGGG 160
 81 GtCGTTttTGGGGGGCGGGGttAGttTCGGTttAATAAGGGGTAtTCGACGttttATTGGttAttTGttTCGAAAGGGGG 160
 81                                           AGGGGTATTTGATGTTT                      160
 81                                             GGGTATTCGACGTTTT                     160

161 AAGACAGTCTGGGCGGGCAGGACGTGCGGAGGGAGAGGCAGCGGTGGCGCGAGGCTCAACTCGAAGCGCTATTGGTGGGA 240
161 AAGAtAGTtTGGGCGGGtAGGACGTGCGGAGGGAGAGGtAGCGGTGGCGCGAGGtTtAAtTCGAAGCGtTATTGGTGGGA 240
161                                                                ATTCGAAGCGTTATTG      240
161                                                                 TTTGAAGTGTTATTGGT    240

241 CTGATAGTCTTGTGCGCCAAGAAGCTGGCAGAAGGGAAGGGGCGGGACATCAGTTCAGGATCTCAAACCGCATTGGTCGT 320
241 tTGATAGTtTTGTGCGttAAGAAGtTGGtAGAAGGGAAGGGGCGGGAtATtAGTTtAGGATtTtAAAtCGtATTGGTCGT 320
241                                                                     AAATCGTATTGGTCGT 320
241                                                                     AAATTGTATTGGTTGT 320
```

321 CTGCCTCGGCTGAGAGAGGCGATGCTTGAAGTTCATTGGTCTTTGTGAGACAGGGTAAGAAAAGCAGCGCGAGCTTGGCG 400
321 tTGttTCGGtTGAGAGAGGCGATGtTTGAAGTTtATTGGTtTTTGTGAGAtAGGGTAAGAAAAGtAGCGCGAGtTTGGCG 400
321 AAAAGTAGCGCGAGTT 400
321 TT AGTAGTGTGAGTTTGG

401 GTTCCTCTGGCCACTTTCTCACAGTGTCTTTGGGCGTCTTCTTGACTGAATCTGACTCCATTGGAGGCTGTGGTAAC 480
401 **TTTTATTGGAGGTTGTGGTAAT** 480
401 GTTttTtTGGttAtTTTtTtAtAGTGTtTTTGGGCGTtTTtTTGAtTGAATtTGAtTttATTGGAGGtTGTGGTAAt 480

Figure 21: Amplificate of SEQ ID NO: 19

```
  1 CTATTATTTCAGATGGAGTGAGGTTGCACGACTGGGATGGAAGAAAGGAATCCCTTAAATTTGGGGGAATTTCTGTTCTC  80
  1 TTATTATTTTAGATGGAGTGAGGTT                                                         80
  1 tTATTATTTtAGATGGAGTGAGGTTGtACGAtTGGGATGGAAGAAAGGAATtttTTAAATTTGGGGGAATTTtTGTTtTt  80

 81 TGTTCTAAGACCATTTTACTTGGGGTGTGGGGGTGGGCGCGGCGGTCAGGGCAGTGGAACGCAGTCGCGGCTGCGCCATC 160
 81 TGTTtTAAGAttATTTTAtTTGGGGTGTGGGGGTGGGCGCGGCGGTtAGGGtAGTGGAACGtAGTCGCGGtTGCGttATt 160
 81                                                        GGAATGTAGTTGTGGT           160
 81                                                         GAACGTAGTCGCGGTT          160

161 CCTGCACTTCCAGGCGCGCGGGAGGGACCGGCGGGGACGCGAGCTGCGGACTCTGGCGAACTCGGGGGAGGCAGACAGGG 240
161 ttTGtAtTTttAGGCGCGCGGGAGGGAtCGGCGGGGACGCGAGtTGCGGAtTtTGGCGAAtTCGGGGGAGGtAGAtAGGG 240
161                                                  ATTTTGGCGAATTCGG                 240
161                                                      TGGTGAATTTGGGGGA             240

241 GGAGGCGGACACCCAGCCGGCAGGCGTCTCAGCCTCCCCGCAGCCGGCGGGCTTTTCTCCTGACAGCTCCAGGAAAGGCA 320
241 GGAGGCGGAtAtttAGtCGGtAGGCGTtTtAGttTttCGtAGtCGGCGGGtTTTTtTttTGAtAGtTttAGGAAAGGtA 320
241          ATTTAGTTGGTAGGTGT      TTTTTTGTAGTTGGTGG                                 320
241            TTAGTCGGTAGGCGTT      TTTTCGTAGTCGGCGG                                 320
```

| | | |
|---|---|---|
| 321 | GACCCCTTCCCCAGCCAGCCAGGTAAGGTAAAGACTGCTGTTGAGCTTGCTGTTACTGAGGGCGCACAGACCCTGGGGAG | 400 |
| 321 | GAttttTTttttAGttAGttAGGTAAGGTAAAGAtTGtTGTTGAGtTTGtTGTTAtTGAGGGCGtAtAGAtttTGGGGAG | 400 |
| 321 | ATTGAGGGCGTATAGA GGGGAG | 400 |
| 321 | TGAGGGTGTATAGATTT GGAG | 400 |
| 401 | ACCGAAGCTTGCCACTGCGGGATTCTGTGGGGTAACCTGGGT | 480 |
| 401 | **TTGTGGGGTAATTTGGGT** | 480 |
| 401 | AtCGAAGtTTGttAtTGCGGGATTtTGTGGGGTAAttTGGGT | 480 |
| 401 | ATTGAAGTTT | 480 |
| 401 | ATCGAAGTTTGT | |

Figure 22: Amplificate of SEQ ID NO: 20

```
  1 AGATGTTTTACATCTGTCTGGGAATCCCGATTTTCCAGGCTCCCTGAGAAATCTATCTGCCTGGAAGAAGTTGCACTTCG  80
  1 AGATGTTTTATATTTGTTTGGGA                                                           80
  1 AGATGTTTTAtATtTGTtTGGGAATttCGATTTTttAGGtTtttTGAGAAATtTATtTGttTGGAAGAAGTTGtAtTTCG  80
  1                                                                       TTGTATTTCG  80
  1                                                                            TTTTG  80

 81 TCGCCACAGGAGCCGCCGAGAGCCAGGGATGGAGATTTCAGACTTAGGACTCTGCCACGATTCTACTGATCCTTAAGCGC 160
 81 TCGttAtAGGAGtCGtCGAGAGttAGGGATGGAGATTTtAGAtTTAGGAtTtTGttACGATTtTAtTGATttTTAAGCGt 160
 81 TCGTTAT                                                                  TAAGCGT 160
 81 TTGTTATAGGAGT                                                              AGTGT 160
 81      ATAGGAGTCGTCGAGA                                                            160
 81      ATAGGAGTTGTTGAGAG                                                           160

161 CAACAGAACGAAATGGACATGCCTATTAGCTACCACAAATCGAATTATTTTATATGAGAGAGAGAAATGTGGTTCAAGTT 240
161 tAAtAGAACGAAATGGAtATGttTATTAGtTAttAtAAATCGAATTATTTTATATGAGAGAGAGAAATGTGGTTtAAGTT 240
161 TAATAGAACGA                                                                      240
161 TAATAGAATGAAAT
```

```
241 CCACATTTGTCCTTTAAATAATCGACCCTCCCTAACCCTGTCCCCAAAGGCTCGTGGAAATTAGGGAGGGGGTTGGGGAG 320
241 ttAtATTTGTttTTTAAATAATCGAtttTtttTAAtttTGTttttAAAGGtTCGTGGAAATTAGGGAGGGGGTTGGGGAG 320

321 ACGGTTCCAGAAACAAAACGCTTTCCTCCAGCACGGCCTCTACATCCAGTCCACCCAAGCTGATTTTTGTAACTCATATA 400
321 ACGGTTttAGAAAtAAAACGtTTTttTttAGtACGGttTtTAtATttAGTttAtttAAGtTGATTTTTGTAAtTtATATA 400

401 AAGAATGGGCCCGGGTGGGTGGTCAGTGAAGACTGGGGTGAGCA                                    480
401                         TAGTGAAGATTGGGGTGAGTA                                    480
401 AAGAATGGGttCGGGTGGGTGGTtAGTGAAGAtTGGGGTGAGtA                                    480
```

Figure 23: Amplificate of SEQ ID NO: 21

```
  1 TAAAAATGGAAGGGAAGACAGATCCAATTTGAAATCCTCCTTGAGAAAAAACAAATCAAAACTAGTTCCTGGGGAAGAAA  80
  1 TAAAAATGGAAGGGAAGATAGA                                                            80
  1 TAAAAATGGAAGGGAAGAtAGATttAATTTGAAATttTttTTGAGAAAAAAtAAATtAAAAtTAGTTttTGGGGAAGAAA  80

 81 GCCTCAGCTAGGTCAGGAGGAAACTTACGCATCTTTGATTCCCTTTCCGCTTTGGAGAGGCATTACCCGTTCAAGCCCAG 160
 81 GttTtAGtTAGGTtAGGAGGAAAtTTACGtATtTTTGATTtttTTTtCGtTTTGGAGAGGtATTAttCGTTtAAGtttAG 160
 81                                                                               AG 160
 81                                                                                  160

161 CCAATGCGGCCGGCCAGGATTTACAGCTCTTATCAAGGGTTAGATTTTGCGAAAGATATAAAGAAAGGAGTTTCCTAACA 240
161 ttAATGCGGtCGGttAGGATTTAtAGtTtTTATtAAGGGTTAGATTTTGCGAAAGATATAAAGAAAGGAGTTTttTAAtA 240
161 TTAATGTGGTTGGT                                      TTAGATTTTGTGAAAGATA              240
161 TTAATGCGGTCGGTTA                                       AGATTTTGCGAAAGATA              240

241 CACCGGGTCTTCTCACAGCAACAAGACACCGAAATTAAGCCTTCTATGGTTTGTGTCTGTAGGACTTTTTAGATAAAACT 320
241 tAtCGGGTtTTtTtAtAGtAAtAAGAtAtCGAAATTAAGttTTtTATGGTTTGTGTtTGTAGGAtTTTTTAGATAAAAtT 320
241                               AAGATATCGAAATTAAGTT                                 320
241                               AAGATATTGAAATTAAGTTT
```

```
321 GGCAGCCCCATTAGATAAGAAATGGCTTTTTAGAAGCTTTGGGGGAAGCGCGGGGGTTCTTCGTGGCCTTCCTGTGACTG 400
321                                                                         TGTGATTG 400
321 GGtAGttttATTAGATAAGAAATGGtTTTTTAGAAGtTTTGGGGGAAGCGCGGGGGTTtTTCGTGGttTTttTGTGAtTG 400

401 TCTTTGGGAGAC                                                                     480
401 TTTTTGGGAGAT                                                                     480
401 TtTTTGGGAGAt                                                                     480
```

Figure 24: Amplificate of SEQ ID NO: 22

```
  1 TTGTGGTCACTCTGAGATGGCAGATTGGGGTCCCTGTTGTCCTTGGACAGATGAGAATGCCGAGAGCTCGTATGCCTTGC  80
  1 TTGTGGTTATTTTGAGATGGTA                                                            80
  1 TTGTGGTtAtTtTGAGATGGtAGATTGGGGTtttTGTTGTttTTGGAtAGATGAGAATGtCGAGAGtTCGTATGttTTGt  80
  1                                                             ATGTCGAGAGTTCGTA      80
  1                                                               GTTGAGAGTTTGTATGT   80

 81 CCAAGGGCACACAGCAAGGCCGGGTGCCCATGCGGCTGTCCCAGGGACCCACTGACCCTGCTGTCCCCCTCAGGCCACAT 160
 81 ttAAGGGtAtAtAGtAAGGtCGGGTGtttATGCGGtTGTtttAGGGAtttAtTGAtttTGtTGTtttttTtAGGttAtAT 160

161 TAGGATCTCAGACCTGGGCTTGGCTGTGAAGATCCCCGAGGGAGACCTGATCCGCGGCCGGGTGGGCACTGTTGGCTACA 240
161 TAGGATtTtAGAttTGGGtTTGGtTGTGAAGATtttCGAGGGAGAtTGATtCGCGGtCGGGTGGGtAtTGTTGGtTAtA 240
161                                 AGATTTTCGAGGGAGA                                 240
161                                 AGATTTTTGAGGGAGAT                                240
161                                                ATTTGATTTGTGGTTGG                 240
161                                                 TTTGATTCGCGGTCGG                 240
```

```
241 TGGGTGAGTGCTGGGCTGCCTGTGTCAATGCACCTTGAGACCCACCACCTGCTCACCGCCGGCCGAGCCCCCAGAGCCTG 320
241 TGGGTGAGTGtTGGGtTGttTGTGTtAATGtAttTTGAGAtttAttAttTGtTtAtCGtCGGtCGAGtttttAGAGttTG 320
241                                                                  TTTATTGTTGGTTGAGT           320
241                                                                    TATCGTCGGTCGAGTT

321 CCCGCAGAGCCTCTCGCCTCTTCATGCACTGCTGATCACACACAGAGTCACAGTCTCTGCAGGGCTGCTGGGGGGCGGCC 400
321                                                                               TT 400
321 ttCGtAGAGttTtTCGttTtTTtATGtAtTGtTGATtAtAtAtAGAGTtAtAGTtTtTGtAGGGtTGtTGGGGGGCGGtt 400

401 TTAGCCAGGGGGAGGG                                                                 480
401 TTAGTTAGGGGGAGGG                                                                 480
401 TTAGttAGGGGGAGGG                                                                 480
```

Figure 25: Amplificate of SEQ ID NO: 23

1 TCCACTCACCAGGTGAAGAGTTTGCCTTTTATCCTGCGCAGCAGGCTGGAGAACTCGGCCGCCGCTCCCTCCGCGAGGGG 80
1 **TTTATTTATTAGGTGAAGAGTTTGTT** 80
1 TttAtTtAttAGGTGAAGAGTTTGttTTTTATttTGCGtAGtAGGtTGGAGAAtTCGGtCGtCGtTtttTtCGCGAGGGG 80
1 AGAATTTGGTTGTTGTT 80
1 AATTCGGTCGTCGTTT 80

81 CTCTGGGGCGCCGGGGCCCGCTGGCGAGTCTGCCTCCATCGGCGCCCTTGCGCGTCTCCGGCGCCCAAAACCCCCGAAGT 160
81 tTtTGGGGCGtCGGGGttCGtTGGCGAGTtTGttTttATCGGCGtttTTGCGCGTtTtCGGCGtttAAAAttttCGAAGT 160
81 TTTTTGGTGTTTAAAATTT 160
81 TTTCGGCGTTTAAAAT 160
81 ATTTTCGAAGT 160
81 TTTGAAGT 160

161 GCCGGGTCCCGGAAGACGGGAGGCGGTGCCTGTGCGGCCGGCTGGGGCTCCGCTCTGGCCCTGCGCTCCCTCCCTCGCTC 240
161 GtCGGGTttCGGAAGACGGGAGGCGGTGttTGTGCGGtCGGtTGGGGtTtCGtTtTGGtttTGCGtTtttTtttTCGtTt 240
161 TTTCGGAAGACGGGAG 240
161 TTTTGGAAGATGGGAG 240
161 GTCGG 240
161 GTTGGGTT

```
241 TCTTGCTCGCGTCCCTCTCGCTCCTCCGCTCGGCTCCTTCAGCGACCAATCATTAACTGTCAAGCCCTAAGGGCCAGACA 320
241 TtTTGtTCGCGTtttTtTCGtTttTtCGtTCGGtTttTTtAGCGAttAATtATTAAtTGTtAAGtttTAAGGGttAGAtA 320

321 ATACATTTGCAGATTACAACCTGGCACCTCCAGGGTGGCAGGAGGA                                   400
321                                TTTTAGGGTGGTAGGAGGA                                   400
321 ATAtATTTGtAGATTAtAAttTGGtAttTttAGGGTGGtAGGAGGA                                   400
```

(The second 321 line, TTTTAGGGTGGTAGGAGGA, is printed in bold.)

Figure 26: Amplificate of SEQ ID NO: 24

| | | |
|---|---|---|
| 492 | TTGGAGAGATGTGCTGGCCAGGACCCGCTGACACAGGTATTCTGGTTGCGCCCCCACCTCACCCGGTGCCCGAGAGCGAG | 413 |
| 492 | **TTGGAGAGATGTGTTGGTTAG** | 413 |
| 492 | TTGGAGAGATGTGtTGGttAGGAttCGtTGAtAtAGGTATTtTGGTTGCGtttttAttTtAttCGGTGttCGAGAGCGAG | 413 |
| 492 | TGTTTGAGAGTGAG | 413 |
| 492 | GTGTTCGAGAGCGAG | 413 |
| 492 | TATTTGGTGTTTGAGAG | 413 |
| 492 | TTTATTCGGTGTTCGA | 413 |
| 412 | TGTGAGTGTATCGATTGTTTCAATTGATTTTTTTAAAGCTAGATAGCAGAGGCTGCGTGTCTGTACACGCAGATATATAC | 333 |
| 412 | TGTGAGTGTATCGATTGTTTtAATTGATTTTTTTAAAGtTAGATAGtAGAGGtTGCGTGTtTGTAtACGtAGATATATAt | 333 |
| 412 | TGT | 333 |
| 412 | T | 333 |
| 332 | ATACACAAATACAGATACTCAAGCGCACACAGATTACACGTACAGAGTCACAGACAAGGACACACACACACACATCGTGG | 253 |
| 332 | ATAtAtAAATAtAGATAtTtAAGCGtAtAtAGATTAtACGTAtAGAGTtAtAGAtAAGGAtAtAtAtAtAtAtATCGTGG | 253 |
| 332 | ATTGTGG | 253 |
| 332 | TATCGTGG | 253 |

| | | |
|---|---|---|
| 252 | TCCCTCACGCACACATACCTCCAAAGAGCCTTGGGTAACCTGGTGAGGTCCCTGAGTGCTGCCCAAGATGTGTGTGTGGG | 173 |
| 252 | TttttTtACGtAtAtATAttTttAAAGAGttTTGGGTAAttTGGTGAGGTtttTGAGTGtTGtttAAGATGTGTGTGTGGG | 173 |
| 252 | TTTTTTATGTATA | 173 |
| 252 | TTTTTTACGTAT | 173 |
| 172 | TGGGGACGCAGGGACGGAGAAAGGGGAGTGTGAAGCAGGATTTCCAGCAGGTCAAGTGCCGCTGATTCTCCTGCCCCCGA | 93 |
| 172 | TGGGGACGtAGGGACGGAGAAAGGGGAGTGTGAAGtAGGATTTttAGtAGGTtAAGTGtCGtTGATTtTttTGttttCGA | 93 |
| 172 | GACGTAGGGACGGAGA | 93 |
| 172 | GATGTAGGGATGGAGA | |
| 92 | CCCCAAATCCTTCCCTTTCTCCTCCTAGGGAGGTCTGAACCAGAAATCCCAAACCTGGGCTTGTTTCCACATCCACAGAG | 13 |
| 92 | **ATTTATAGAG** | 13 |
| 92 | ttttAAATttTTtttTTTtTttTttTAGGGAGGTtTGAAttAGAAATtttAAAttTGGGtTTGTTTttAtATttAtAGAG | 13 |
| 12 | GGGAGGAGTCCT | -67 |
| 12 | **GGGAGGAGTTTT** | -67 |
| 12 | GGGAGGAGTttT | -67 |

Figure 27: Amplificate of SEQ ID NO: 25

```
407 TCTCCAGAACAAAGGACTTTAGGGCCCAAATTCCGTTTATTCAGTACTCCAAGTCCTAAAAACTTGGAATATCTGATGAA 328
407 TTTTTAGAATAAAGGATTTTAGGG                                                         328
407 TtTttAGAAtAAAGGAtTTTAGGGtttAAATTtCGTTTATTtAGTAtTttAAGTttTAAAAAtTTGGAATATtTGATGAA 328

327 TAAAAGTGGCCGCTCCCCAGGCTGTCTCTTGAGAGAAGCCACCGGCACAGCTGACCTTGCCCGCTCCATCGCGTCACTGA 248
327 TAAAAGTGGtCGtTttttAGGtTGTtTtTTGAGAGAAGttAtCGGtAtAGtTGAttTTGttCGtTttATCGCGTtAtTGA 248

247 CCGCTCCTCAGACAGATGCGTCAGGCATCTCCGGCGGCCGCTCCACTCTGCGCCAGACTCGCTGCAGCAGCGGCAGGCTT 168
247 tCGtTttTtAGAtAGATGCGTtAGGtATtTtCGGCGGtCGtTttAtTtTGCGttAGAtTCGtTGtAGtAGCGGtAGGtTT 168
247                                                  TGTGTTAGATTTGTTGT  TAGCGGTAGGTTT 168
247                                                TTTGCGTTAGATTCGT     TAGTGGTAGGTTT 168

167 CGCACACATCCCCGCCTGAGCATGCGCGCCAGCCTGCCTCTGCGGCCGCGCAGGCGTGCTTGTTTGCCGCAGTGCAGGGG  88
167 CGtAtAtATtttCGttTGAGtATGCGCGttAGttTGttTtTGCGGtCGCGtAGGCGTGtTTGTTTGtCGtAGTGtAGGGG  88
167 CGTA               AGTATGCGCGTTAGTT  TTTTGTGGTTGTGTAGG                              88
167 TGTA             TGAGTATGTGTGTTAGT  TTTTTGCGGTCGCGTA
```

```
87 TCCCAGCTCCCTCCCTCACCGGAATGACCTGGGGGGAGGGGGCTACTGGACCCCTAGGGCCCCACAGCACTGTTGCAATG   8
87                                                                 AGTATTGTTGTAATG   8
87 TtttAGtTtttTtttTtAtCGGAATGAttTGGGGGGAGGGGGtTAtTGGAttttTAGGGttttAtAGtAtTGTTGtAATG   8
```

```
7 AGAGGGG   -72
7 AGAGGGG   -72
7 AGAGGGG   -72
```

Figure 28: Amplificate of SEQ ID NO: 26

1 TCCCAGGAATGCAGGACACAAGGGGGCAGCCTACCTCAGGGCAGGGCCAGCGGGGTTCCTCATCCTTAGGCCAGTCCCCG 80
1 **TTTTAGGAATGTAGGATATAAGGG** 80
1 TtttAGGAATGtAGGAtAtAAGGGGGtAGttTAttTtAGGGtAGGGttAGCGGGGTTttTtATttTTAGGttAGTtttCG 80

81 GGGCTCAGATGATTCCAGGGAAGGCCGACCCGGCTGACTGCCACCTCCACCATCACTCTGCAGAGCGGTTGAGCATGAAA 160
81 GGGtTtAGATGATTttAGGGAAGGtCGAttCGGtTGAtTGttAttTttAttATtAtTtTGtAGAGCGGTTGAGtATGAAA 160
81 AAGGTTGATTTGGTTG 160
81 GTCGATTCGGTTGATT 160

161 GACCAGCTGATCGCACAGAGCCTCCTAGAGAAACAGCAGATCTACCTGGAGATGGCCGAGATGGGCGGCCTCGAAGACCT 240
161 GAttAGtTGATCGtAtAGAGttTttTAGAGAAAtAGtAGATtTAttTGGAGATGGtCGAGATGGGCGGttTCGAAGAttT 240
161 AGATGGGTGGTTTTGA 240
161 GGCGGTTTCGAAGATT 240

241 GCCCCAGCCCCGAGGCCTATTCCGTGGAGGGGACCCATCCGAGACCCTGCAGGGGGAGCTAATTCTCAAGTCGGCCATGA 320
241 GttttAGtttCGAGGttTATTtCGTGGAGGGGAtttATtCGAGAtttTGtAGGGGGAGtTAATTtTtAAGTCGGttATGA 320
241 AAGTCGGTTATGA 320
241 AAGTTGGTTATGA 320

```
321 GCGAGAGTAAGTTGGCTGCCCACACCTCAAGGGTGCAGTCTTGCCGGGGTGGGCTCCTCAGGGAACCCCAGGCCAGGCTC 400
321 GCGAGAGTAAGTTGGtTGtttAtAttTtAAGGGTGtAGTtTTGtCGGGGTGGGtTttTtAGGGAAttttAGGttAGGtTt 400
321 GCGA                                                                             400
321 GTGA                                                                             400

401 ACGGCTCATTGTGGCCGACACGGCACCCTGTCCAGGACAGGCTTCTATGTGGGG                           480
401                                    GGATAGGTTTTTATGTGGGG                           480
401 ACGGtTtATTGTGGtCGAtACGGtAtttTGTttAGGAtAGGtTTtTATGTGGGG                           480
401          TTGTGGTTGATATGGT                                                         480
401             TGGTCGATACGGTATT
```

Figure 29: Amplificate of SEQ ID NO: 27

```
273 CAGGGTCTGATGATGTGATTTTCACCCCGCCGCATGTGAAGGCCACAGAGCAGATCATTCAGCTCACGCTGAAGGGCACA 194
273 TAGGGTTTGATGATGTGATTTT                                                           194
273 tAGGGTtTGATGATGTGATTTTtAtttCGtCGtATGTGAAGGttAtAGAGtAGATtATTtAGtTtACGtTGAAGGGtAtA 194
273                                ATTTTGTTGTATGTGAAG                                194
273                                ATTTCGTCGTATGTGA                                  194

193 CGGGCGCTCAGGGCAGCAGAGAGCAGAGGACGCGACAGGGATGTGTGATGCTGGCTCAGGCCTGGGCATCCGAGTTGATG 114
193 CGGGCGtTtAGGGtAGtAGAGAGtAGAGGACGCGAtAGGGATGTGTGATGtTGGtTtAGGttTGGGtATtCGAGTTGATG 114
193                                   AGGACGCGATAGGGAT                               114
193                                   AGGATGTGATAGGGAT                               114

113 AGCGCTGGCTGGCGTTCAGCGCCTGGAAACTGCACACGCGCGCTGGGCCGCTTGCGCATTTAAGGAAGTGCAGCCTCATC  34
113 AGCGtTGGtTGGCGTTtAGCGttTGGAAAtTGtAtACGCGCGtTGGGtCGtTTGCGtATTTAAGGAAGTGtAGttTtATC  34
113                                                       GGTTGTTTGTGTATTTAA          34
113                                     ATTGTATACGCGCGTT   GTCGTTTGCGTATTTA           34
113                                      TTGTATATGTGTGTTGG

 33 GGAAGAGGCCTGCCAGTGCTGAGCCAGGAGGGA                                                -46
 33            TTAGTGTTGAGTTAGGAGGGA                                                -46
 33 GGAAGAGGttTGttAGTGtTGAGttAGGAGGGA                                                -46
```

Figure 30: Amplificate of SEQ ID NO: 28

1 GAGAGGGAGGGCCAAGGTCCGGGCGCCGAGAGGAGGGGCCCGGGGGGCCGGCTGGAGGGGGCGAGGCGCTAGGCGGCTTT 80
1 **GAGAGGGAGGGTTAAGGTT** 80
1 GAGAGGGAGGGttAAGGTtCGGGCGtCGAGAGGAGGGGttCGGGGGGtCGGtTGGAGGGGGCGAGGCGtTAGGCGGtTTT 80

81 GCTTCCCGTCCCCGTCAATTGGCCGCTAGCTCTGTTTTGACTGCCTGTACAAAGCGAGTAGACGGCGGGGGATGGGGCGA 160
81 GtTTttCGTtttCGTtAATTGGtCGtTAGtTtTGTTTTGAtTGttTGTAtAAAGCGAGTAGACGGCGGGGGATGGGGCGA 160
81 GTTAATTGGTTGTTAGTT TATAAAGTGAGTAGATGG A 160
81 AATTGGTCGTTAGTTTT TAAAGCGAGTAGACGG 160

161 TTAGGGACCGGATTTGGGAAAGGAGGCAGCAGC 240
161 **ATTTGGGAAAGGAGGTAGTAGT** 240
161 TTAGGGAtCGGATTTGGGAAAGGAGGtAGtAGt 240
161 TTAGGGATTGGATTTG 240
161 TTAGGGATCGGATTTG

Figure 31: Amplificate of SEQ ID NO: 29

356 GTGGAAAAAGGAGAGCAAACTGCTCAGCGCGTCTCAGCTCAGTGCCGAGGAGGAGGAAGAAAAACAGGCCGAGTGAAGGT 277
356 **GTGGAAAAAGGAGAGTAAATTG** 277
356 GTGGAAAAAGGAGAGtAAAtTGtTtAGCGCGTtTtAGtTtAGTGtCGAGGAGGAGGAAGAAAAAtAGGtCGAGTGAAGGT 277

276 GCTGGAAAGGGAGGGAGGACGCGAGGGGAAAGGCCTGTGGGGAGCCGAGGGCGTCAGAGAGACCCGGGAAGGAAGGCTCT 197
276 GtTGGAAAGGGAGGGAGGACGCGAGGGGAAAGGttTGTGGGGAGtCGAGGGCGTtAGAGAGAttCGGGAAGGAAGGtTtT 197
276 AGTCGAGGGCGTTAGA 197
276 AGTTGAGGGTGTTAGA 197

196 CGGGTGGGGGAGCCAGGAGACCTGCTCTCCGGCGCAGACAGGCGGGGCCCAGCGCTCTCCTGGACGCCCCCGCCCGCACA 117
196 CGGGTGGGGGAGttAGGAGAttTGtTtTtCGGCGtAGAtAGGCGGGGtttAGCGtTtTttTGGACGttttCGttCGtAtA 117
196 TTTTTCGGCGTAGATA ATA 117
196 TGTTTTTTGGTGTAGAT TATA 117

116 GCTCCCGGCGGGTGCTCTGAGGCCTCACTACTCGAGCCCACCCAGCATCCCGCGCGCCCTTCCTTCCCGAGGAACTCGCC 37
116 GtTttCGGCGGGTGtTtTGAGGttTtAtTAtTCGAGtttAtttAGtATttCGCGCGtttTTttTTttCGAGGAAtTCGtt 37
116 GTTTTCGGCGGGT TTTTTTGAGGAATTTGTT 37
116 GTTTTTGGTGGGT TTTTCGAGGAATTCGT

```
36 TCAGCCTGATCAGGCTTCCTGGTGAGAACTGAGGAG   -43
36               TTTTGGTGAGAATTGAGGAG   -43
36 TtAGttTGATtAGGtTTttTGGTGAGAAtTGAGGAG   -43
```

Figure 32: Amplificate of SEQ ID NO: 30

420 AGGGAGGCCTGGTGCATTCCTCCATGGGGGTGGGGAATGCGGAGGCTGGGCCACCGGAGGTGGTGGCGGTGGCAGGGGCG 341
420 **AGGGAGGTTTGGTGTATTTT** 341
420 AGGGAGGttTGGTGtATTttTttATGGGGGTGGGGAATGCGGAGGtTGGGttAtCGGAGGTGGTGGCGGTGGtAGGGGCG 341

340 GCAGGAGACGCCACGTCAGGGTCACTGCCCCACTCGGTGCACAGCAGGGGGCCGGCAGTTCACCGGATCACCGCGGGGGT 261
340 GtAGGAGACGttACGTtAGGGTtAtTGttttAtTCGGTGtAtAGtAGGGGGtCGGtAGTTtAtCGGATtAtCGCGGGGGT 261
340 AGATGTTATGTTAGGGT ATTGGATTATTGTGGGG 261
340 TAGGAGACGTTACGTT TATCGGATTATCGCGG 261
340 GTCGGTAGTTTATCGGAT 261
340 GTTGGTAGTTTATTGGAT 261

260 GGGGCGGAGGACGGAAGTTGGTGAGGGGGGTGGTCTACAGGGCACCTCCCGTCCCAGGGCTCTAACCTAATTCGAAAGGG 181
260 GGGGCGGAGGACGGAAGTTGGTGAGGGGGGTGGTtTAtAGGGtAttTttCGTtttAGGGtTtTAAttTAATTCGAAAGGG 181
260 TTAATTTGAAAGGG 181
260 TTTAATTCGAAAGGG 181

| | | |
|---|---|---|
| 180 | AAAGGAGTGCTGCAGCCGGGAGGCCAGGCCGACTTTGGGTCCTGCCCTCGCAGAAGCCCCCACGCCTGGTGCAGACCAAT | 101 |
| 180 | AAAGGAGTGtTGtAGtCGGGAGGttAGGtCGAtTTTGGGTttTGtttTCGtAGAAGtttttACGttTGGTGtAGAttAAT | 101 |
| 180 | AAAG | 101 |
| 180 | AA | |

| | | |
|---|---|---|
| 100 | GACTCGCTTCCTGCCCTTACACCGATCACACATGACCTCGGTCACAGGGGTGCCACATCACAGGAGTGCCCCTCACTCCT | 21 |
| 100 | **TT** | 21 |
| 100 | GAtTCGtTTttTGtttTTAtAtCGATtAtAtATGAttTCGGTtAtAGGGGTGttAtATtAtAGGAGTGttttTtAtTttT | 21 |

| | | |
|---|---|---|
| 20 | GCCTGCAGGAAGCAGGGCCC | -59 |
| 20 | **GTTTGTAGGAAGTAGGGTTT** | -59 |
| 20 | GttTGtAGGAAGtAGGGttt | -59 |

Figure 33: Amplificate of SEQ ID NO: 31

```
  1 AAGGCACTACTCCTGGGGTTCCCCGAAGCGGATCCTGGGACTGGGACCTGGGGACGCGGTGAGAGGCAGCCCTGAAGCTG  80
  1 AAGGTATTATTTTTGGGGTTTT                                                            80
  1 AAGGtAtTAtTttTGGGGTTttCGAAGCGGATttTGGGAtTGGGAttTGGGGACGCGGTGAGAGGtAGtttTGAAGtTG  80
  1                   TTTTTCGAAGCGGATT                                                80
  1                      TTTGAAGTGGATTTTGG                                            80

 81 AGTCCAGAGGTCTGGGGCGTCATTGTCCCAGGGCGGACCCAGCCCCAGACCCTACAACTAAACCCAAGGCCAGCCGAGAA 160
 81 AGTttAGAGGTtTGGGGCGTtATTGTttAGGGCGGAtttAGttttAGAtttTAtAAtTAAAtttAAGGttAGtCGAGAA 160
 81                                                                   AGGTTAGTCGAGAA 160
 81                                                                   AGGTTAGTTGAGAA 160

161 GCACTACCACTGGCGGGCTGAGCTGAGCTCTCATTCTTCTTAGGACTCTGGACCTTTGGAGGAAATGAAAGGGACCCAGT 240
161 GtAtTAttAtTGGCGGGtTGAGtTGAGtTtTtATTtTTtTTAGGAtTtTGGAttTTTGGAGGAAATGAAAGGGAtttAGT 240
161 GT                                                                               240
161 GTA                                                                              240
```

241 TGGAAATATAGTCTTTCTTCGCGCCCCCTACATCTCTCTCCCCCGGCGGAAACGCGGCCCAAACTGTTACGAAGTGGAAG 320
241 **AAGTGGAAG** 320
241 TGGAAATATAGTtTTTtTTCGCGtttttTAtATtTtTtTttttCGGCGGAAACGCGGtttAAAtTGTTACGAAGTGGAAG 320
241 GGAAATGTGGTTTAAATT 320
241 GAAACGCGGTTTAAAT 320
241 ATTGTTACGAAGTGGA 320
241 TTGTTATGAAGTGGAAG

321 AGGCTGGATAGCT 400
321 **AGGTTGGATAGTT** 400
321 AGGtTGGATAGtT 400

Figure 34: Amplificate of SEQ ID NO: 32a

```
  1 GGGTGCTGGGGAGCCCCATGCGCCAGGAGGTGGATAATTTAGGGGGCAAGGGGCTCGCACAAGGGCGAAGATGCGCTCTC  80
  1 GGGTGTTGGGGAGTTTTA                                                                80
  1 GGGTGtTGGGGAGttttATGCGttAGGAGGTGGATAATTTAGGGGGtAAGGGGtTCGtAtAAGGGCGAAGATGCGtTtTt  80

 81 CTCCCGGCACCGACCCACGCGTTTAAAGGCCTCTGCGGCAACGTTTAGGGACGCGAACTAGGTGCCGGGGGTTGGGGAGG 160
 81 tTttCGGtAtCGAtttACGCGTTTAAAGGttTtTGCGGtAACGTTTAGGGACGCGAAtTAGGTGtCGGGGGTTGGGGAGG 160
 81                                 TTTGCGGTAACGTTTA                                 160
 81                                  TTGTGGTAATGTTTAGG                               160
 81                                              TTAGGGACGCGAATTA                     160
 81                                                AGGGATGTGAATTAGG                   160

161 AGGTGGGACTGGAAGCGAGAGCAGTCTGCGCCCAAGACCGCGCAAGAGGTGGCCTTTTGTTCGCTGGCTCCAGCACATCC 240
161 AGGTGGGAtTGGAAGCGAGAGtAGTtTGCGtttAAGAtCGCGtAAGAGGTGGttTTTTGTTCGtTGGtTttAGtAtATtt 240
161                            TGCGTTTAAGATCGCGT                                ATATTT 240
161                            TGTGTTTAAGATTGTGT                                  ATTT 240
```

```
241 CGGCTCTCGAAAAAACACTACCTCTGTCCCCTGCCACCCTCTGCATTTTAGAAAATCCTCTTCAGGGCAGGAAAGGACAG 320
241                                                                       GGAAAGGATAG 320
241 CGGtTtTCGAAAAAAtAtTAttTtTGTttttTGttAtttTtTGtATTTTAGAAAATttTtTTtAGGGtAGGAAAGGAtAG 320
241 TGGTTTTTGAAAA                                                                     320
241 CGGTTTTCGAAA

321 TGAGTCACTGCAAG                                                                    400
321 TGAGTTATTGTAAG                                                                    400
321 TGAGTtAtTGtAAG                                                                    400
```

Figure 35: Amplificate of SEQ ID NO: 32b

```
317 AGAGTGTGGTACCAGATCTGGTTCCATTTTTACGAGAGCCAGGAACCACGCACGTCTGGAGGGAGAGCGCAGCTGACCCG 238
317 TCCTTTCC                                                                         238
317 AGAGTGTGGTAttAGATtTGGTTttATTTTTACGAGAGttAGGAAttACGtACGTtTGGAGGGAGAGCGtAGtTGAttCG 238
317                                                AGGAATTACGTACGTT       AGAGCGTAGTTGATTCG 238
317                                                                       AGAGTGTAGTTGATTTG 238
317                                                     TATGTATGTTTGGAGGG                   238

237 AGGGCGCGACCGTGTTCTGGGAGTGTTTGAATCCCCGGCCTTTGGTGAAAATTTCCTGTGTCCGCAAGGCCCAGAGGAGA 158
237 AGGGCGCGAtCGTGTTtTGGGAGTGTTTGAATtttCGGttTTTGGTGAAAATTTttTGTGTtCGtAAGGtttAGAGGAGA 158
237 A                                                                                158
237 A                                                                                158

157 TCGTGTGATGTCCGGGGGGTGCTGCGTGCGGCCGGCTGGGTAGCGGCCGCGGACCACCACTAAACCGGGGCATGTTTGCC  78
157 TCGTGTGATGTtCGGGGGGTGtTGCGTGCGGtCGGtTGGGTAGCGGtCGCGGAttAttAtTAAAtCGGGGtATGTTTGtC  78
157                                                TAGCGGTCGCGGATTA                      78
157                                               GTAGTGGTTGTGGATT                       78
```

| | | |
|---|---|---|
| 77 | GTCCTTCCTGGTGCTCTGCCCCTCTGTAACGCGGGGAAGTAGGAGGGCGCGGGGAGAAATTACTAAAGGATGCTTGC | -2 |
| 77 | **TAAAACTCCCCAACACCC** | -2 |
| 77 | GTttTTttTGGTGtTtTGttttTtTGTAACGCGGGGAAGTAGGAGGGCGCGGGGAGAAATTAtTAAAGGATGtTTGt | -2 |
| 77 | TTTGTAACGCGGGGAA | -2 |
| 77 | TTTTGTAATGTGGGGA | |

Figure 36: Amplificate of SEQ ID NO: 33

1 TCAGCTGAGAAAGTGGGGGCCCTAAAAAGGGCCTTTTGTTGATAGAAAGGGACGCTCAACCACCGAAACCGTAGAGGGTG 80
1 **TTAGTTGAGAAAGTGGGGGT** 80
1 TtAGtTGAGAAAGTGGGGGtttTAAAAAGGGttTTTTGTTGATAGAAAGGGACGtTtAAttAtCGAAAtCGTAGAGGGTG 80
1 ATCGAAATCGTAGAGG 80
1 ATTGAAATTGTAGAGGG 80

81 CGGCCCTGGCGCTTGAGCGCGTAGACCACATCCATGGCGGTGACCGTCTTGCGCTTGGCGTGCTCTGTATAGGTCACGGC 160
81 CGGtttTGGCGtTTGAGCGCGTAGAttAtATttATGGCGGTGAtCGTtTTGCGtTTGGCGTGtTtTGTATAGGTtACGGC 160
81 TTTATGGTGGTGATTGT TTACGGC 160
81 TATGGCGGTGATCGTT TTATGGT 160

161 GTCCCGGATCACGTTCTCCAGGAACACCTTCAGCACCCCGCGAGTCTCCTCGTAGATGAGGCCGGAGATGCGCTTCACGC 240
161 GTttCGGATtACGTTtTttAGGAAtAttTTtAGtAtttCGCGAGTtTttTCGTAGATGAGGtCGGAGATGCGtTTtACGt 240
161 GTTTCGGAT AGATGTGTTTTATGT 240
161 GTTTTGGATT ATGCGTTTTACGT 240

241 CGCCGCGGCGAGCAAGGCGCCGGATGGCCGGCTTGGTGATGCCCTGGATATTGTCGCGCAGTACTTTACGGTGGCGCTTA 320
241 CGtCGCGGCGAGtAAGGCGtCGGATGGtCGGtTTGGTGATGtttTGGATATTGTCGCGtAGTAtTTTACGGTGGCGtTTA 320
241 TGT GGATATTGTTGTGTAGT 320
241 CGT TATTGTCGCGTAGTAT

```
321 GCGCCGCCTTTGCCAAGACCCTTCCCGCCTTTGCCGCGGCCAGACATGACGAGCAAGAGGAGTCTCACCCAACGCTTTGT 400
321 GCGtCGttTTTGttAAGAtttTTttCGttTTTGtCGCGGttAGAtATGACGAGtAAGAGGAGTtTtAtttAACGtTTTGT 400

401 GAGGACTCTGGCCTGAGGCAG                                                             480
401 GAGGATTTTGGTTTGAGGTAG                                                             480
401 GAGGAtTtTGGttTGAGGtAG                                                             480
```

Figure 37: Amplificate of SEQ ID NO: 34

```
498 GTGAGAGTGGGTGCTGAAACCCCATCCCGGGCCCCAAAGGACACTGGCCGATTCTCGCGCAGAAAGCCACAGGCCTTGTC 419
498 GTGAGAGTGGGTGTTGAAAT                                                             419
498 GTGAGAGTGGGTGtTGAAAttttATttCGGGttttAAAGGAtAtTGGtCGATTtTCGCGtAGAAAGttAtAGGttTTGTC 419
498                                                       GGTCGATTTTCGCGTA           419
498                                                      TGGTTGATTTTTGTGTA           419

418 GCACTCCCTCCCTGTGGACGGGTGTGGATGTGTCATCATCTGACTGCTCCCTGGGAGGGCTGGGAGTTTCACCCGCTCCT 339
418 GtAtTttttTtttTGTGGACGGGTGTGGATGTGTtATtATtTGAtTGtTtttTGGGAGGGtTGGGAGTTTtAttCGtTttT 339

338 GCCTTGCCAAGGCACTGACCGGCTGGAGGCGCGGAATCCGGCCTGGTTGTTGGCGGTCCGATCTGGGGACCGGCTCTTCG 259
338 GttTTGttAAGGtAtTGAtCGGtTGGAGGCGCGGAATtCGGttTGGTTGTTGGCGGTtCGATtTGGGGAtCGGtTtTTCG 259
338             TATTGATTGGTTGGAGG                                            ATTGGTTTTTG 259
338             TATTGATCGGTTGGAG                                             ATCGGTTTTTCG 259

258 AGGTCTGTCCTGTTCAGCCCCGCCTGGGGCATAGGGTCTTCTAGGTCTCGGGTTGTGGTGCGGGGTTGCCAGATAAATGC 179
258 AGGTtTGTttTGTTtAGtttCGttTGGGGtATAGGGTtTTtTAGGTtTCGGGTTGTGGTGCGGGGTTGttAGATAAATGt 179
258 AGGTT                                                                            179
258 AGGT
```

```
178 AGTAATGGCAGCAAGGAGAACCGGCATTTCCACTGGGCCTTTTACGTCTACCATGCCTGTTTTCCCATGTGGCCCTCCGA  99
178 AGTAATGGtAGtAAGGAGAAtCGGtATTTttAtTGGGttTTTTACGTtTAttATGttTGTTTTtttATGTGGtttTtCGA  99

 98 CTCCTGTGAGGGAGGTGAGGCTGCACTATAGGTGAGGATGTGGGGGATCAAGGCAGGGGCATCCCCCACCCACCCTCAGC  19
 98                                                                            TTAGT  19
 98 tTttTGTGAGGGAGGTGAGGtTGtAtTATAGGTGAGGATGTGGGGGATtAAGGtAGGGGtATtttttAtttAtttTtAGt  19

 18 CATGCAGCTGACTGGGGC                                                                -61
 18 TATGTAGTTGATTGGGGT                                                                -61
 18 tATGtAGtTGAtTGGGGt                                                                -61
```

Figure 38: Amplificate of SEQ ID NO: 35

```
  1 ACCTCAGGTGTAAGCCCAAGGCTGCTGCCTGCAAAGGCAATGCCGTGGAGACTGGGTTCCACAGCGACCTGGGTTTTTAA  80
  1 ATTTTAGGTGTAAGTTTAAGGTTGT                                                          80
  1 AttTtAGGTGTAAGtttAAGGtTGtTGttTGtAAAGGtAATGtCGTGGAGAtTGGGTTttAtAGCGAttTGGGTTTTTAA  80

 81 GTCACCGAAAGCTACAGGGCAGGGTCACAAACACTCTCCCGCCTTCTCTGCAGAACCGTGCGGCTGACAGGAGAGCGTTG 160
 81 GTtAtCGAAAGtTAtAGGGtAGGGTtAtAAAtAtTtTttCGttTTtTtTGtAGAAtCGTGCGGtTGAtAGGAGAGCGTTG 160
 81                                                TGTAGAATTGTGTGGT                  160
 81                                                     AATCGTGCGGTTGATA             160

161 CGCAGAAAATTCGAGGCCGGGCGCTGGAGGAGTCTCCGCGGCCCGGAGAAGGCACAGAGGCGCCCCTGAGAGGCGCAGCT 240
161 CGtAGAAAATTCGAGGtCGGGCGtTGGAGGAGTtTtCGCGGttCGGAGAAGGtAtAGAGGCGttttTGAGAGGCGtAGtT 240
161      AAAATTCGAGGTCGGG           TTTTCGCGGTTCGGAG                                 240
161      AAAATTTGAGGTTGGG             TTTGTGGTTTGGAGAA                               240

241 GGAACAGGCGATGCACGGGTTCGGATCTGGCCGGCCAACCGAGCCCAGCGGTCGCGAAAACCAGAGTCGCCACAGAGGTT 320
241 GGAAtAGGCGATGtACGGGTTCGGATtTGGtCGGttAAtCGAGtttAGCGGTCGCGAAAAttAGAGTCGttAtAGAGGTT 320
241   AATAGGCGATGTACGG       TTTGGTTGGTTAATTGA   TAGCGGTCGCGAAAAT                    320
241     TAGGTGATGTATGGGT       TTGGTCGGTTAATCGA                                      320
241                                         AGTTTAGTGGTTGTGA
```

321 GAGCACGATTCCATTTCTGGGGATCGGGTCCGGTGGGGAGCCACTGTCCCTAACGCCTCCAGAGACTTTAAGTAGGACAA 400
321 GAGtACGATTttATTTtTGGGGATCGGGTtCGGTGGGGAGttAtTGTtttTAACGttTttAGAGAtTTTAAGTAGGAtAA 400

401 TATAATGCTGGTAGGAGCAAGGTGCAAGGAATTTAGCGGCAGAAGCCTTTCGGGGGGGTGGGGAAAGGCAGAT 480
401 **GGGTGGGGAAAGGTAGAT** 480
401 TATAATGtTGGTAGGAGtAAGGTGtAAGGAATTTAGCGGtAGAAGttTTTCGGGGGGGTGGGGAAAGGtAGAT 480

Figure 39: Amplificate of SEQ ID NO: 13

```
488 TGTCCTAGGTCTGATGGACCAGACCCCAGCGGGCCTGAACTTTGTCGGAAAGCGGAGCCGCCAGGCATTTGGCAGCTGCC 409
488 TGTTTTAGGTTTGATGGATTAGA                                                          409
488 TGTttTAGGTtTGATGGAttAGAttttAGCGGGttTGAAtTTTGTCGGAAAGCGGAGtCGttAGGtATTTGGtAGtTGtt 409
488                                         TTTGTCGGAAAGCGGA                         409
488                                         TTTGTTGGAAAGTGGA                         409

408 AGAGGCGCACCAGGCCGGGTCTCAACCTGGGAGGTCAGCGGGCCAAAGCGGCCTGGGGAGGCAGTGGCCCCACCTGGACG 329
408 AGAGGCGtAttAGGtCGGGTtTtAAttTGGGAGGTtAGCGGGttAAAGCGGttTGGGGAGGtAGTGGttttAttTGGACG 329
408                                                                       TATTTGGACG 329
408                                                                       TATTTGGATG 329

328 CCGGGCCGTCGGGCGTCGGAGCCCCGCTCTGCCTCCACCAAGCTGTCCCGCTGCGCCCTACGCCGCCACCAGGCAGGAGG 249
328 tCGGGtCGTCGGGCGTCGGAGtttCGtTtTGttTttAttAAGtTGTttCGtTGCGtttTACGtCGttAttAGGtAGGAGG 249
328 TCGGGT                                                   TTACGTCGTTATTAGGT       249
328 TTGGGT                                                 TTTTATGTTGTTATTAGGT       249

248 AGCCGCTACCAGTACCTCAGAGTGGGACTCTCCATCCTCAAGTCTGCGCTCCAGAAAAGTGTAGCAAGCCTCCCACGCGG 169
248 AGtCGtTAttAGTAttTtAGAGTGGGAtTtTttATttTtAAGTtTGCGtTttAGAAAAGTGTAGtAAGttTtttACGCGG 169
248                                                                        TTTATGTGG 169
248                                                                        TTTACGCGG 169
```

```
168 GGCTTCAGACACCTAAAAGTCGCCACTCCCCCAAGAGTAACCTCCACCCCCACCCCCACCCCCAACTTCTCTTTCCTCCT  89
168 GGtTTtAGAtAttTAAAAGTCGttAtTtttttAAGAGTAAttTttAtttttAtttttAtttttAAtTTtTtTTTttTttT  89
168 GGTTTTAG                                                                          89
168 GGTTTTA
```

```
 88 TTTCTTCAGCAATGTGGACCAACAGTTACTTTTCCCTCTCTTCCCTTCCCCTCCGAAGTCCCCGGGGCAAAGTGGGACTT   9
 88                                                                       AGTGGGATTT   9
 88 TTTtTTtAGtAATGTGGAttAAtAGTTAtTTTTtttTtTtTTttttTTttttTtCGAAGTtttCGGGGtAAAGTGGGAtTT   9
```

```
  8 TGGGGAGC                                                                         -71
  8 TGGGGAGT                                                                         -71
  8 TGGGGAGt                                                                         -71
```

Figure 40: Amplificate of SEQ ID NO: 36

1 GTTGAGAGGTAAGGCATGAAGGGCGCAATATCCAATATGAGCAACGCGTGTGATGCATCTGGTCAAAATGCATACAGAGG 80
1 **GTTGAGAGGTAAGGTATGAAGG** 80
1 GTTGAGAGGTAAGGtATGAAGGGCGtAATATttAATATGAGtAACGCGTGTGATGtATtTGGTtAAAATGtATAtAGAGG 80
1 TATGAGTAATGTGTGTG 80
1 GAGTAACGCGTGTGAT 80

81 ACTTGTCTCTGTCCCTAGATAGAAGTCCTCCGTCCTGCAGTCATGAGGGTCAATTGCTGAGGCTTCACAGTTCCCTTCTC 160
81 AtTTGTtTtTGTtttTAGATAGAAGTttTtCGTttTGtAGTtATGAGGGTtAATTGtTGAGGtTTtAtAGTTtttTTtTt 160

161 TCTTACACTCGGACCGTCACGCTCCTCACCTACTACCCCGATGCAGAGGTAGACTCAGGATCCCTGCACTTGTCAAGGAT 240
161 TtTTAtAtTCGGAtCGTtACGtTttTtAttTAtTAtttCGATGtAGAGGTAGAtTtAGGATtttTGtAtTTGTtAAGGAT 240
161 ATTCGGATCGTTACGT 240
161 ATTTGGATTGTTATGTTT 240

241 TCCTCGGCAAGCTCACGGGGCGGGAGTGGCCACAAGACGGAGCTCGCCTGGTCCTGGCCTTCCCGGCCTATACAAGCCTG 320
241 TttTCGGtAAGtTtACGGGGCGGGAGTGGttAtAAGACGGAGtTCGttTGGTttTGGttTTttCGGttTATAtAAGttTG 320
241 ATAAGACGGAGTTCGT 320
241 AAGATGGAGTTTGTTTG 320

321 CCCCCTTCCCAATTCCCAATCTCCACAGCCTTCCATCCTCCCACTTTCGATTCACCTTGCGCCACCGACGCCCCTGGCCT 400

321 tttttTTtttAATTtttAATtTttAtAGttTTttATttTtttAtTTTCGATTtAttTTGCGttAtCGACGttttTGGttT 400

321 ATTTTGTGTTATTGATGT 400

321 TTGCGTTATCGACGTT

401 TCGTTTGCAGCAAGTTTACCCCCACCATTACCTCTCGCATAAAAGCCTGCATTTACCAGGTCAAAGAGGGGAACCAA 480

401 **TTATTAGGTTAAAGAGGGGAATTAA** 480

401 TCGTTTGtAGtAAGTTTAtttttAttATTAttTtTCGtATAAAAGttTGtATTTAttAGGTtAAAGAGGGGAAttAA 480

Figure 41: Amplificate of SEQ ID NO: 37

1 GTGTCAGGGCTCAGGGGCCCATCGCCCTTGGCCTGGGGCCCCTCCGTGCCGTCGGGATTTGTGGGACGCGCTGAAGGAAG 80
1 **GTGTTAGGGTTTAGGGGTTT** 80
1 GTGTtAGGGtTtAGGGGtttATCGtttTTGGttTGGGGttttTtCGTGtCGTCGGGATTTGTGGGACGCGtTGAAGGAAG 80
1 TGTGGGATGTGTTGAA 80
1 GTGGGACGCGTTGAAG 80

81 AGGCGGGCGGCTCCGACAGAAAACAGCCAGAGCGCACCACTCACCTGAGTGCCAGGTAAACACCTGGGCGCGACAGGGAC 160
81 AGGCGGGCGGtTtCGAtAGAAAAtAGttAGAGCGtAttAtTtAttTGAGTGttAGGTAAAtAttTGGGCGCGAtAGGGAt 160
81 GGGTGGTTTTGATAGA AGTTAGAGCGTATTATTT TATTTGGGCGCGATAG 160
81 GGCGGTTTCGATAGAA ATTTGGGTGTGATAGG 160
81 AATAGTTAGAGTGTATTATT

161 AGGAAACAAGGGTAGGGTGCGGAGGCTGGGGAGGAAGAGGTTGGAAAGG 240
161 **AGGAAGAGGTTGGAAAGG** 240
161 AGGAAAtAAGGGTAGGGTGCGGAGGtTGGGGAGGAAGAGGTTGGAAAGG 240

Figure 42: Amplificate of SEQ ID NO: 1

504 TCTGGAGGGATAGAATGTGATCCTCGAGCAGAAACCATCGTGTTTCTGGGGGCTCTTCGGGCGCCGTGGGGCGACGCGGT 425
504 **TTTGGAGGGATAGAATGTGA** 425
504 TtTGGAGGGATAGAATGTGATttTCGAGtAGAAAttATCGTGTTTtTGGGGGtTtTTCGGGCGtCGTGGGGCGACGCGGT 425

424 GCAGCGTCTAGGGCCGCCGCAGGCCGGGGGCAGGGCTCCCAGCGGTTCCCCCGCGGCCAGCGGCCACGCAGGAAAAACCC 345
424 GtAGCGTtTAGGGtCGtCGtAGGtCGGGGGtAGGGtTtttAGCGGTTttttCGCGGttAGCGGttACGtAGGAAAAAttC 345
424 TTAGGGTCGTCGTAGGT AGTGGTTATGTAGGAAA 345
424 TTAGGGTTGTTGTAGGT TAGCGGTTACGTAGGA 345

344 GCTTCCTCGCCCCTGCATCTGCTGACAAGCCGCCCGAGGTGTCTGCGCTGAGCGTGGCCACACCGATGCAGCTTTCTAGG 265
344 GtTTttTCGttttTGtATtTGtTGAtAAGtCGttCGAGGTGTtTGCGtTGAGCGTGGttAtAtCGATGtAGtTTTtTAGG 265
344 AAGTTGTTTGAGGTGT 265
344 ATAAGTCGTTCGAGGT 265

264 AAATGGCCCGGGAGGGGGAGGGGGCGAGTGAGGGATTAGGTCCGGCTCCCCCTCGCTCCCCCTGCGCAAACACCACCACC 185
264 AAATGGttCGGGAGGGGGAGGGGGCGAGTGAGGGATTAGGTtCGGtTtttttTCGtTtttttTGCGtAAAtAttAttAtt 185

| | | |
|---|---|---|
| 184 | CCTTCCTTTCCCCGGAGGCATGAGGCCTCCACGCTCGGTCCTTCGCCCGCATCCTCGCCGACCCCTCCGGTTTCGCGCCT | 105 |
| 184 | ttTTttTTTtttCGGAGGtATGAGGttTttACGtTCGGTttTTCGttCGtATttTCGtCGAttttTtCGGTTTCGCGttT | 105 |
| 104 | CACTCGCCAGGCTTTCTCCCTGGGAGCCGACTGCGGTGAAGTCGCCGCCAGCACGGTCGGCCCTGACACGTGCTCTAACG | 25 |
| 104 | tAtTCGttAGGtTTTtTtttTGGGAGtCGAtTGCGGTGAAGTCGtCGttAGtACGGTCGGtttTGAtACGTGtTtTAACG | 25 |
| 104 | TTAGTACGGTCGGTTT TACGTGTTTTAACG | 25 |
| 104 | TGATATGTGTTTTAATG | 25 |
| 104 | GTTAGTATGGTTGGTTT | 25 |
| 24 | CATGCTTGAGTCAAGGCTGTCTTT | -55 |
| 24 | **ATGTTTGAGTTAAGGTTGTTTTT** | -55 |
| 24 | tATGtTTGAGTtAAGGtTGTtTTT | -55 |
| 24 | TAT | -55 |
| 24 | TA | |

Figure 43: Amplificate of SEQ ID NO: 2

```
  1 AGATCATCAGTCCTCACCTGAGGGACCTTCCGCGGCATCTATGCGGGCATGGTTACTGCCTCTGGTGCCCCCCGCAGCCG  80
  1 AGATTATTAGTTTTTATTTGAGGGATT                                                      80
  1 AGATtATtAGTttTtAttTGAGGGAttTTtCGCGGtATtTATGCGGGtATGGTTAtTGttTtTGGTGtttttCGtAGtCG  80
  1                                                                           TAGTCG  80
  1                                                                           TAGTTG  80

 81 CGCGCAGGTACCGTGCGACATCGCGATGGCCCAGCTCCTCAGCCAGGTCCACGGGCAGACGGCCCCAGGCATCGCGCACG 160
 81 CGCGtAGGTAtCGTGCGAtATCGCGATGGtttAGtTttTtAGttAGGTttACGGGtAGACGGtttTAGGtATCGCGtACG 160
 81 CGCGTAGGTA                                                      TTAGGTATTGTGTATG 160
 81 TGTGTAGGTAT                                                      TAGGTATCGCGTACG 160
 81    GTAGGTATTGTGTGATAT                                                            160
 81     TAGGTATCGTGCGATA                                                             160

161 TCCAGCCGCGCCCCGGCCCGGTGCAGCACCACCAGCGTGTCCAGGAAGCCCTCCCGGGCAGCGTCGTGCACGGGTCGGGT 240
161 TttAGtCGCGtttCGGttCGGTGtAGtAttAttAGCGTGTttAGGAAGtttTttCGGGtAGCGTCGTGtACGGGTCGGGT 240
161 TT                                                                               240
161 T                                                                                240
```

241 GAGAGTGGCGGGGTCGGCGCAGTTGGGCTCCGCGCCGTGGAGCAGCAGCAGCTCCGCCACTCGGGCGCTGCCCATCATCA 320
241 GAGAGTGGCGGGGTCGGCGtAGTTGGGtTtCGCGtCGTGGAGtAGtAGtAGtTtCGttAtTCGGGCGtTGtttATtATtA 320
241 TTTTGTGTTGTGGAGTA 320
241 TTCGCGTCGTGGAGTA

321 TGACCTGCCAGAGAGAACAGAATGGTCAGAGCCAGGGTGG 400
321 **GAATGGTTAGAGTTAGGGTGG** 400
321 TGAttTGttAGAGAGAAtAGAATGGTtAGAGttAGGGTGG 400

Figure 44: Amplificate of SEQ ID NO: 3

```
  1 CCCTTGCTTGGGTGTGTCCTTCGCTCGCTCCCTCCCTCCGTCTTAGGTCACTGTTTTCAACCTCGAATAAAAACTGCAGC  80
  1 TTTTTGTTTGGGTGTGTTTT                                                              80
  1 tttTTGtTTGGGTGTGTttTTCGtTCGtTtttTtttTtCGTtTTAGGTtAtTGTTTTtAAttTCGAATAAAAAtTGtAGt  80

 81 CAACTTCCGAGGCAGCCTCATTGCCCAGCGGACCCCAGCCTCTGCCAGGTTCGGTCCGCCATCCTCGTCCCGTCCTCCGC 160
 81 tAAtTTtCGAGGtAGttTtATTGtttAGCGGAttttAGttTtTGttAGGTTCGGTtCGttATttTCGTttCGTttTtCGt 160
 81                                              TAGGTTCGGTTCGTTAT                   160
 81                                              TAGGTTTGGTTTGTTATT                  160

161 CGGCCCCTGCCCCGCGCCCAGGGATCCTCCAGCTCCTTTCGCCCGCGCCCTCCGTTCGCTCCGGACACCATGGACAAGTT 240
161 CGGttttTGtttCGCGtttAGGGATttTtAGtTttTTTCGttCGCGtttTtCGTTCGtTtCGGAtAttATGGAtAAGTT 240
161         GTTTCGCGTTTAGGGA                                 GTTCGTTTCGGATATTA       240
161         GTTTTGTGTTTAGGGAT                                 TTTGTTTTGGATATTATGG    240

241 TTGGTGGCACGCAGCCTGGGGACTCTGCCTCGTGCCGCTGAGCCTGGCGCAGATCGGTGAGTGCCCGCCGCAGCCTGGGC 320
241 TTGGTGGtACGtAGttTGGGGAtTtTGttTCGTGtCGtTGAGttTGGCGtAGATCGGTGAGTGttCGtCGtAGttTGGGt 320
241                                           TTTGGCGTAGATCGGT                       320
241                                           TTTGGTGTAGATTGGT
```

```
321 AGCAAGATGGGTGCGGGGTGCTCAGCGCGGACCCGGCGGCAGCCCCTCCGGCTGAGTCGGCCCTGGGGGACTGGAGTCAA 400
321                                                                 GGGGGATTGGAGTTAA 400
321 AGtAAGATGGGTGCGGGGTGtTtAGCGCGGAttCGGCGGtAGttttTtCGGtTGAGTCGGtttTGGGGGAtTGGAGTtAA 400

401 GTG                                                                              480
401 GTG                                                                              480
401 GTG                                                                              480
```

Figure 45: Amplificate of SEQ ID NO: 4

```
446 GAAGGGATGAATGAATAAAAGTACTTGTCTGATGGCAGCAGAGACCCCGAGCAAACGGTGGAGGCTACACTGTCTGGCAT 367
446 GAAGGGATGAATGAATAAAAGT                                                           367
446 GAAGGGATGAATGAATAAAAGTAtTTGTtTGATGGtAGtAGAGAtttCGAGtAAACGGTGGAGGtTAtAtTGTtTGGtAT 367
446                                               TTTGAGTAAATGGTGGA                GTAT 367
446                                             ATTTCGAGTAAACGGT                 TGGTAT 367

366 TCTCGCAGCGTTTCGTCAGAGCCGGACCCGCCTGCAGCTCAAGGGAGGCGTGCTCCTCTCCCAGAGCAGGCTGGAACCCA 287
366 TtTCGtAGCGTTTCGTtAGAGtCGGAttCGttTGtAGtTtAAGGGAGGCGTGtTttTtTtttAGAGtAGGtTGGAAtttA 287
366 TTTCGTAGCGTT       AGTTGGATTTGTTTGTA                                             287
366 TTTTGTAGTGTT    TAGAGTCGGATTCGTT                                                 287

286 GCTGGGTTCCGCCTCCCGGGAAGGTGGTCTCCATTCGTCGCTCTGCATCTGGTTTGTCAGATCCGAGAGGTAAACATTCG 207
286 GtTGGGTTtCGttTttCGGGAAGGTGGTtTttATTCGTCGtTtTGtATtTGGTTTGTtAGATtCGAGAGGTAAAtATTCG 207
286        TTTGTTTTTTGGGAAGG                                                         207
286        TTCGTTTTTCGGGAAG

206 GGCTTGGTGTTGAATTAAAATCATTGATTGAACCTTATTCTGGGGCTTCGGTTTGGCTTACTAGTTTGGGATTTTAAAAA 127
206 GGtTTGGTGTTGAATTAAAATtATTGATTGAAttTTATTtTGGGGtTTCGGTTTGGtTTAtTAGTTTGGGATTTTAAAAA 127
```

| | | |
|---|---|---|
| 126 | AATAAAAATTAAGCCTATAGAGAGGGCAAATTAAAATTAGGTTGGGTAAAGGAAGGAGCGCGAGTGTTTGAAGCCGTTTG | 47 |
| 126 | AATAAAAATTAAGttTATAGAGAGGGtAAATTAAAATTAGGTTGGGTAAAGGAAGGAGCGCGAGTGTTTGAAGtCGTTTG | 47 |

| | | |
|---|---|---|
| 46 | GAGGGAACAGCGGTTTCCAAGTTCCTGCTGACTTGAGAAGTCTCTG | -33 |
| 46 | **TTGTTGATTTGAGAAGTTTTTG** | -33 |
| 46 | GAGGGAAtAGCGGTTTttAAGTTttTGtTGAtTTGAGAAGTtTtTG | -33 |

Figure 46: Amplificate of SEQ ID NO: 5

```
  1 ACATGGGATTGATGGAAGACAGCCCCCAAGGACGGGGGTGGGTGGCCCTGTTTTTCTCTGATTGGCGGACGAGGGACTTC  80
  1 ATATGGGATTGATGGAAGATAG                                                            80
  1 AtATGGGATTGATGGAAGAtAGttttAAGGACGGGGGTGGGTGGtttTGTTTTTtTtTGATTGGCGGACGAGGGAtTTt  80
  1                                                               TTGATTGGCGGACGAG    80
  1                                                               TTGATTGGTGGATGAG    80

 81 TAGACCCCTGCCAAAACACCAAGGGGGCGGTGTCGCACGCCATGCTGCTCAGCGGAAGCAGGGCTTGTATAGAAATGGGC 160
 81 TAGAttttTGttAAAAtAttAAGGGGGCGGTGTCGtACGttATGtTGtTtAGCGGAAGtAGGGtTTGTATAGAAATGGGC 160
 81                       GGTGTTGTATGTTATGT                                   ATGGGT 160
 81                         TGTCGTACGTTATGTT                                   TGGGC 160

161 GTAGCAGCCGGCGGGAGTGGGTCGGACTGGGCTCCGCGTAGGCGGGCAGGGCAGCTCTACGGTTAGACAAGACCATAGAG 240
161 GTAGtAGtCGGCGGGAGTGGGTCGGAtTGGGtTtCGCGTAGGCGGGtAGGGtAGtTtTACGGTTAGAtAAGAttATAGAG 240
161 GTAGTAGTTGG                ATTGGGTTTCGCGTAGG                                       240
161 GTAGTAGTCGG                ATTGGGTTTTGTGTAGG

241 CTGGGTAAAGACGAATTTAGAACACAGCGGAGGTAGGAGGGCAGGATGGCTGTCAGGCACACGAAAGAGCATTGAGTGGC 320
241 tTGGGTAAAGACGAATTTAGAAtAtAGCGGAGGTAGGAGGGtAGGATGGtTGTtAGGtAtACGAAAGAGtATTGAGTGGt 320
```

```
321 AGAAACGAAATGCTTTCAGAGGGCAGGGTTGAACTGCGGCCAGAGTTAAAAAGGGGAGGAACCTGGCTCTGCATTGATTG 400
321 AGAAACGAAATGtTTTtAGAGGGtAGGGTTGAAtTGCGGttAGAGTTAAAAAGGGGAGGAAttTGGtTtTGtATTGATTG 400

401 GCGGTGGCTGGACTCAACCTAGAATAGGGGCCCGACTAGGGAGAGCGGGATCCAGGTCTGTGCCCATTGGA     480
401                                                   GATTTAGGTTTGTGTTTATTGGA     480
401 GCGGTGGtTGGAtTtAAttTAGAATAGGGGttCGAtTAGGGAGAGCGGGATttAGGTtTGTGtttATTGGA     480
```

Figure 47: Amplificate of SEQ ID NO: 6

1 CTTTCATCCAGGATGAGGGACATTTAAGATGAAATGTCCGTGGCAGGATCGTTTCTCTTCACTGCTGCATGCGGCACTGG 80
1 **TTTTTATTTAGGATGAGGGATATT** 80
1 tTTTtATttAGGATGAGGGAtATTTAAGATGAAATGTtCGTGGtAGGATCGTTTtTtTTtAtTGtTGtATGCGGtAtTGG 80

81 GAACTCGCCCCACCTGTGTCCGGAACCTGCTCGCTCACGTCGGCTTTCCCCTTCTGTTTTGTTCTAGGACTTCTGCACGG 160
81 GAAtTCGttttAttTGTGTtCGGAAttTGtTCGtTtACGTCGGtTTTttttTTtTGTTTTGTTtTAGGAtTTtTGtACGG 160
81 TACGG 160
81 TATGG 160

161 ACCTGGCCGTCTCCAGTGCCAACTTCATTCCCACGGTCACTGCCATCTCGACCAGTCCGGACCTGCAGTGGCTGGTGCAG 240
161 AttTGGtCGTtTttAGTGttAAtTTtATTtttACGGTtAtTGttATtTCGAttAGTtCGGAttTGtAGTGGtTGGTGtAG 240
161 ATTTGGTCGTTT TATTTTGATTAGTTTGGAT 240
161 ATTTGGTTGTTT TTCGATTAGTTCGGAT 240

241 CCCGCCCTCGTCTCCTCCGTGGCCCCATCGCAGACCAGAGCCCCTCACCCTTTCGGAGTCCCCGCCCCCTCCGCTGGGGC 320
241 ttCGtttTCGTtTttTtCGTGGttttATCGtAGAttAGAGttttTtAtttTTTCGGAGTtttCGtttttTtCGtTGGGGt 320
241 TTTCGTGGTTTTATCGTA 320
241 TTTTGTGGTTTTATTGTA 320

```
321 TTACTCCAGGGCTGGCGTTGTGAAGACCATGACAGGAGGCCGAGCGCAGAGCATTGGCAGGAGGGGCAAGGTGGAACAGG 400
321 TTAtTttAGGGtTGGCGTTGTGAAGAttATGAtAGGAGGtCGAGCGtAGAGtATTGGtAGGAGGGGtAAGGTGGAAtAGG 400
321                                                     TAGGAGGTTGAGTGTA                              .                   400
321                                                           GTCGAGCGTAGAGTAT

401 TGAGGAACTCTAGCGTACTCTTCCTGGGAATGTGGGGGCTGGGTGGGAAGCAGC                          480
401                                                           GGTTGGGTGGGAAGTAGT                          480
401 TGAGGAAtTtTAGCGTAtTtTTttTGGGAATGTGGGGGtTGGGTGGGAAGtAGt                          480
```

Figure 48: Amplificate of SEQ ID NO: 7

```
347 TTGGCCCCATGCTGGGAGCTCTGAGCCCCATCCCCGGGGACGCGGGCCGCGCGTACTCACTGGTGGCGAAGACTGCGGCG 268
347 TTGGTTTTATGTTGGGAGTTTTG                                                          268
347 TTGGttttATGtTGGGAGtTtTGAGttttATtttCGGGGACGCGGGtCGCGCGTAtTtAtTGGTGGCGAAGAtTGCGGCG 268
347                                                        GTCGCGCGTATTTATT          268
347                                                      GGGTTGTGTGTATTTAT           268

267 GCGAAACTCCAGCGAAGGCCTCGCGGCCTCCGAGCCTTATAAGGGTGGTCCCGCCCCGCTCCGCCCCAGTGCTGAGTCAC 188
267 GCGAAAtTttAGCGAAGGttTCGCGGttTtCGAGttTTATAAGGGTGGTttCGtttCGtTtCGttttAGTGtTGAGTtAC 188
267                      TTTGTGGTTTTTGAGTT                                           188
267                      TTCGCGGTTTTCGAGT                                            188

187 GGCGCCGGCCGCTCTTCTGGAGGGTCCCGCGGACTCCCGCCGGCCCCAGCCCCGGCGGCCGCTGCACCCCGGGCGTCGGC 108
187 GGCGtCGGtCGtTtTTtTGGAGGGTttCGCGGAtTttCGtCGGttttAGtttCGGCGGtCGtTGtAtttCGGGCGTCGGt 108
187                                                                           GTCGGT 108
187                                                                           GTTGGT 108

107 CGCAGAGGGGCGCCCTGGAGTCCCCGGAGTCGCCGCGCAGCTGGCCGGGGAAGCCTTTCCCTCTTTCCCAGGTCCCCAGC  28
107 CGtAGAGGGGCGtttTGGAGTtttCGGAGTCGtCGCGtAGtTGGtCGGGGAAGttTTTtttTtTTTtttAGGTttttAGC  28
107 CGTAGAGGGG              GAGTCGTCGCGTAGTT                                          28
107 TGTAGAGGGG             GGAGTTGTTGTGTAGTT
```

| | | |
|---|---|---|
| 27 | GGGGCCTAGGGAGTAAACAGACAGCAG | -52 |
| 27 | **GTTTAGGGAGTAAATAGATAGTAG** | -52 |
| 27 | GGGGttTAGGGAGTAAAtAGAtAGtAG | -52 |

Figure 49: Amplificate of SEQ ID NO: 38

```
466 CAGATGGGGACAGGAAGCTGTGGACGAAAGCCCCAGGTCCCGTGGGAGAGGTGACAGCAGCAGGGGCACGCAGCCACGTG 387
466 TAGATGGGGATAGGAAGTTGT                                                              387
466 tAGATGGGGAtAGGAAGtTGTGGACGAAAGttttAGGTttCGTGGGAGAGGTGAtAGtAGtAGGGGtACGtAGttACGTG 387
466                                                                   TATGTAGTTATGTG 387
466                                                                GGGTACGTAGTTACGT  387

386 GGTCCCCAGGGGAATGTGAAGGCGGAGGGCTCCAGGCGAACTGGGGATTAAACAAATATTTACAGGCAGCAGGGAAGTGC 307
386 GGTttttAGGGGAATGTGAAGGCGGAGGGtTttAGGCGAAtTGGGGATTAAAtAAATATTTAtAGGtAGtAGGGAAGTGt 307
386 GGT                                                                              307
386                                                                                  307

306 CCAGCGCACGTGACGGGGGCGGGGCGGGACTTTGGGGAGGGCGGGGCCTAACGGTATCGAGCGAGCCGGTTGTAGACGTG 227
306 ttAGCGtACGTGACGGGGGCGGGGCGGGAtTTTGGGGAGGGCGGGGttTAACGGTATCGAGCGAGtCGGTTGTAGACGTG 227
306   AGTGTATGTGATGGGG                                     TATTGAGTGAGTTGGT          227
306 TTAGCGTACGTGACGG                                       TATCGAGCGAGTCGGT          227

226 GTCCAGGTTTCTGCACAGGAATATCGAGAGCGTCATGAACCCGAGCTATAGAGAAAGGAGATGAGGTCAGAGAAGTCGAA 147
226 GTttAGGTTTtTGtAtAGGAATATCGAGAGCGTtATGAAttCGAGtTATAGAGAAAGGAGATGAGGTtAGAGAAGTCGAA 147
226                       ATCGAGAGCGTTATGA                                           147
226                 AGGAATATTGAGAGTGT
```

| | | |
|---|---|---|
| 146 | GGGGTGCTGGCGAGACGGGGGTGATGCCCACCCGGGCGAGAAAGGTCAGGGGTGGGGCCGTGAATTGGGCGGGAAGGGGC | 67 |
| 146 | GGGGTGtTGGCGAGACGGGGGTGATGtttAttCGGGCGAGAAAGGTtAGGGGTGGGGtCGTGAATTGGGCGGGAAGGGGt | 67 |
| 66 | TGGAGAAGGGGATAGGTAGAAAAGGGCGGGGCTCGACGGGAACTGCCGCAGGGCTGGCTGATGAGG | -13 |
| 66 | **TAGGGTTGGTTGATGAGG** | -13 |
| 66 | TGGAGAAGGGGATAGGTAGAAAAGGGCGGGGtTCGACGGGAAtTGtCGtAGGGtTGGtTGATGAGG | -13 |

Figure 50: Amplificate of SEQ ID NO: 39

466 CCACCAGGAGAGGGGAAGAAGCCAGCACCTACCGACAGGGGTGGAGCTGGGTCAAGAATGGTGTGGTCCCTGCTTTGGGG 387
466 **TTATTAGGAGAGGGGAAGAAGT** 387
466 ttAttAGGAGAGGGGAAGAAGttAGtAttTAtCGAtAGGGGTGGAGtTGGGTtAAGAATGGTGTGGTtttTGtTTTGGGG 387

386 GAATGCTGGGGAGGTAGAAAGCCCCTTCTAACGGGGCGTCACTGCAATTACTGCTTCCTCTTTCCCATAAAACTCCCCCT 307
386 GAATGtTGGGGAGGTAGAAAGttttTTtTAACGGGGCGTtAtTGtAATTAtTGtTTttTtTTTtttATAAAAtTtttttT 307
386 AATGGGGTGTTATTGT 307
386 TTTAACGGGGCGTTAT 307

306 AGTGTATCAGAACCCCCAAGGAGTTTCAGTAAGCGGTTCTTCTGTTGTCTCCGGCTGAGACTCCAGGGGAACCTCAAGCT 227
306 AGTGTATtAGAAtttttAAGGAGTTTtAGTAAGCGGTTtTTtTGTTGTtTtCGGtTGAGAtTttAGGGGAAttTtAAGtT 227

226 CACATGGCCCTGGCGGGCCCCTGGGCAGGAGCAGGCGAGAGGTCTGCGCGGCCGCTCTCCTACCTGCGTCCGACTCCGCG 147
226 tAtATGGtttTGGCGGGttttTGGGtAGGAGtAGGCGAGAGGTtTGCGCGGtCGtTtTttTAttTGCGTtCGAtTtCGCG 147
226 TTCGATTTCGCG 147
226 GTTTGATTTTGTG 147

| | | |
|---|---|---|
| 146 | GTCCTTGGGCAGCAGCAACCGGGTAGCGCTCAGACTACAGACCCCAGCGCGATGACCCGCCTACCTCAGTTTCCATTGGC | 67 |
| 146 | GTttTTGGGtAGtAGtAAtCGGGTAGCGtTtAGAtTAtAGAttttAGCGCGATGAttCGttTAttTtAGTTTttATTGGt | 67 |
| 146 | GTTT TAATCGGGTAGCGTTT AGTGTGATGATTTGTTT | 67 |
| 146 | GTTT TAGTAATTGGGTAGTGT TAGCGCGATGATTCGT | |
| 66 | CAGGGATCAGGGCTACCCGCTCCCATTGGCTACTTATCAATATAGAGGTGGGGCCAGCCTGGAATG | -13 |
| 66 | **TGGGGTTAGTTTGGAATG** | -13 |
| 66 | tAGGGATtAGGGtTAttCGtTtttATTGGtTAtTTATtAATATAGAGGTGGGGttAGttTGGAATG | -13 |

Figure 60: Amplificate of SEQ ID NO: 40

```
  1 CACTGGATGTCCCTGGCAGGTCCGGGTGTCTGTCTCCGAAGAGGACAGAGAAGGGCAGCCACGATCTGCCCGCCTGCCCT  80
  1 TATTGGATGTTTTTGGTAGGTT                                                            80
  1 tAtTGGATGTtttTGGtAGGTtCGGGTGTtTGTtTtCGAAGAGGAtAGAGAAGGGtAGttACGATtTGttCGttTGtttT  80
  1                                                                               TT  80
  1                                                                               TT  80

 81 CGCGAGCCTCTCGGCACTGGGTGAGAGGCAACTCTGGCCATTTCTTGCTGCCCTCTCGCCCTCTCCCGGCCGCTCCCAGT 160
 81 CGCGAGttTtTCGGtAtTGGGTGAGAGGtAAtTtTGGttATTTtTTGtTGtttTtTCGtttTtTttCGGtCGtTtttAGT 160
 81 CGCGAGTTTTTCGG                                                                   160
 81 TGTGAGTTTTTTGGTA                                                                 160

161 CCAGCCGGGCCCGGCGCTACCCGAGCGAGGGTTCGAGCCCTCTGCGCGGCCGCGCAGAAGCAGGCAGGGCCTCAACTTCT 240
161 ttAGtCGGGttCGGCGtTAttCGAGCGAGGGTTCGAGtttTtTGCGCGGtCGCGtAGAAGtAGGtAGGGttTtAAtTTtT 240
161       GGTTTGGTGTTATTTGA                                                          240
161         TTCGGCGTTATTCGAG                                                         240

241 GCAAATGTGTGCGGCTCGCCGCCTGTCCCCTTTCTCCTCCTGTCTCCACCCGTGCGGCCCCAGTGGCCTGGAGCTTCCAG 320
241 GtAAATGTGTGCGGtTCGtCGttTGTttttTTTtTttTttTGTtTttAttCGTGCGGtttAGTGGttTGGAGtTTttAG 320
241                                              TATTCGTGCGGTTTTA                    320
241                                               ATTTGTGTGGTTTTAGT                  320
```

```
321 CCCCGCGCTTGGCCGCGGCTTGGCGAGGCTATGCTGCGGGAAGCTGGAATCCAACGCGCGGCCGGCTGAACCGCCTGAGC 400
321 tttCGCGtTTGGtCGCGGtTTGGCGAGGtTATGtTGCGGGAAGtTGGAATttAACGCGCGGtCGGtTGAAtCGttTGAGt 400
321                                             GGAATTTAATGTGTGGT                     400
321                                              GAATTTAACGCGCGGT

401 CGCGGGAAACCAGCGGCGGAGCGGCGGTATAGAGGTGCGAGGATGAGGAGGACCGACTCGCTAAGGGAGGGAGGTGACT 480
401                                                              TAAGGGAGGGAGGTGATT 480
401 CGCGGGAAAttAGCGGCGGAGCGGCGGTATAGAGGTGCGAGGATGAGGAGGAtCGAtTCGtTAAGGGAGGGAGGTGAtT 480
```

Figure 61: Amplificate of SEQ ID NO: 41

424 GGAGGGGCTTGAGTAATTGATCCCTTCTCGAGATGGGGTCGAATTCCTTCCGAATGGGGGACCTTCATCCCCCTCCTGTG 345
424 **GGAGGGGTTTGAGTAATTG** 345
424 GGAGGGGtTTGAGTAATTGATtttTTtTCGAGATGGGGTCGAATTttTTtCGAATGGGGGAttTTtATtttttTttTGTG 345

344 GGTGTATGGGGGCTGCCCTGGAGATGCGCGCCCGCGGAGGCAGGTATTGGGTGTCGGCGGAGGCGGGGCCGCGTCCCCAG 265
344 GGTGTATGGGGGtTGtttTGGAGATGCGCGttCGCGGAGGtAGGTATTGGGTGTCGGCGGAGGCGGGGtCGCGTttttAG 265

264 GGTGCTGTCCCGGTGCCCCTGGAGGCGGCCCCGACTCCACAATGGGCCGCTCTGATTCTGAGGCGGAGGCCGGCGCTTTG 185
264 GGTGtTGTttCGGTGttttTGGAGGCGGtttCGAtTttAtAATGGGtCGtTtTGATTtTGAGGCGGAGGtCGGCGtTTTG 185
264 AGGCGGTTTCGATTTT 185
264 TGGAGGTGGTTTTGAT 185

184 TTGGCGGGCGGGGTAGCGGGCGAGCAGCTGTCGCATTTTCCCACGGGCGGGAGCTGAGTGTGGCCACCCCCCCTCCCCCC 105
184 TTGGCGGGCGGGGTAGCGGGCGAGtAGtTGTCGtATTTTtttACGGGCGGGAGtTGAGTGTGGttAtttttttTtttttC 105
184 TAGCGGGCGAGTAGTT TTTACGGGCGGGAGTT 105
184 TAGTGGGTGAGTAGTT TTTTATGGGTGGGAGT 105

| | | |
|---|---|---|
| 104 | GTCCAGCTCTGCCCCCTTGCAGAACGGGTTTGAACAGAGGCAATCTCGGCGGCTGGATGGGGGGCCCTGCCCTGCCAGAC | 25 |
| 104 | GTttAGtTtTGtttttTTGtAGAACGGGTTTGAAtAGAGGtAATtTCGGCGGtTGGATGGGGGGtttTGtttTGttAGAt | 25 |
| 104 | ATTTCGGCGGTTGGAT | 25 |
| 104 | AGGTAATTTTGGTGGTT | |
| 24 | TCCTCAGTGAGCCTCCAGGGTGGG | -55 |
| 24 | **TAGTGAGTTTTTAGGGTGGG** | -55 |
| 24 | TttTtAGTGAGttTttAGGGTGGG | -55 |

Figure 62: Amplificate of SEQ ID NO: 42

```
  1 GACCAGGCTTGGAGTTGTTTCTTCGGGGGGCTTCCCCTCCTGACAGCCTACTGAGTTGCATCAGAACGTGGAGGATTGT  80
  1 GATTAGGTTTGGAGTTGTTTTT                                                          80
  1 GAttAGGtTTGGAGTTGTTTTtTTCGGGGGGtTTttttTttTGAtAGttTAtTGAGTTGtATtAGAACGTGGAGGATTGT  80

 81 CCTCTGAGGACGCGCGGGGCAACCGGGAGTCTTAAAATAGCAGATAAAGTTAATACTGACTGTAATGTGCGTAAATTGGA 160
 81 ttTtTGAGGACGCGCGGGGtAAtCGGGAGTtTTAAAATAGtAGATAAAGTTAATAtTGAtTGTAATGTGCGTAAATTGGA 160

161 TTATAATCTTTAGGCGTATTGGTGACAACCAGCGTAATCCATCTCTGAGTATTAATCCGGTTAGACTCCCGGGTGACAGC 240
161 TTATAATtTTTAGGCGTATTGGTGAtAAttAGCGTAATttATtTtTGAGTATTAATtCGGTTAGAtTttCGGGTGAtAGt 240
161                                                         TAATTCGGTTAGATTTTCGG        240
161                                                         TAATTTGGTTAGATTTTTGG        240

241 CAGTCGCAGGAGTGTTCCCAGAGTCGCGCCTCTGCAAATGTTCTCACTCGCGTTGTATTTGATCTCAGTGGCCGGAGATA 320
241 tAGTCGtAGGAGTGTTtttAGAGTCGCGttTtTGtAAATGTTtTtAtTCGCGTTGTATTTGATtTtAGTGGtCGGAGATA 320
241                 TTTAGAGTCGCGTTTT                                    TTAGTGGTCGGAGATA 320
241                   TAGAGTTGTGTTTTTGT                                   AGTGGTTGGAGATA 320
241                                                                                 GATA 320
241                                                                                   TA 320
```

The third line of the first block is printed in bold; the primer/probe sequences in the last two blocks are underlined.

321 GAACAGCGCGGCCACGCGTGGGGCGCGGAGAGGAGTATCAACTACTGGCTTCCTTTACAGACGCAGATAACCTTTAAAAA 400
321 GAAtAGCGCGGttACGCGTGGGGCGCGGAGAGGAGTATtAAtTAtTGGtTTttTTTAtAGACGtAGATAAttTTTAAAAA 400
321 GA 400
321 GAATAGTGTGGTTA 400
321 GAATAGCGCGGTTA

401 GGAAACGGATAAGATCTAGACGATTGATTTGAAAGTGAAAGATGGATGTGACT 480
401 **AAAGTGAAAGATGGATGTGATT** 480
401 GGAAACGGATAAGATtTAGACGATTGATTTGAAAGTGAAAGATGGATGTGAtT 480

Figure 63: Amplificate of SEQ ID NO: 43

```
389 GGGGACAAGTGGTTAATGAGCAGGGCCAACTGGCCGGTCCCTGTCCTGTATGCCACACACACACCCCGTGAGGGCAGCAA 310
389 GGGGATAAGTGGTTAATGAGT                                                            310
389 GGGGAtAAGTGGTTAATGAGtAGGGttAAtTGGtCGGTtttTGTttTGTATGttAtAtAtAtAtttCGTGAGGGtAGtAA 310
389                                                                TATATTTTGTGAGGGTAG 310
389                                                                TATATTTCGTGAGGGTA  310

309 GGAGCATCCAGCCAGGGGACGTGCACGAGAAGGGCCAGGATGATCACACTGCCCGGGGCTCAGCACAGGGCTGAGTCCCG 230
309 GGAGtATttAGttAGGGGACGTGtACGAGAAGGGttAGGATGATtAtAtTGttCGGGGtTtAGtAtAGGGtTGAGTttCG 230
309          TAGGGGACGTGTACGA                                                        230
309          TAGGGGATGTGTATGA                                                        230

229 GAGGTGACCATTGCCGCCCCGCTCCATCTGCGGCCACTGAGGTCTGCTGGGGGTCTCTGGTGCTGGCCTTGACAGCCCAC 150
229 GAGGTGAttATTGtCGtttCGtTttATtTGCGGttAtTGAGGTtTGtTGGGGGTtTtTGGTGtTGGttTGAtAGtttAt 150

149 CCGTGGCAGGACAGGGTCTCGAGGTGCCTCTCGGTAGGTGGTGGGCTCAGTGGGGGTGACCCGTGGGGACGGAACGCATG 70
149 tCGTGGtAGGAtAGGGTtTCGAGGTGttTtTCGGTAGGTGGTGGGtTtAGTGGGGGTGAttCGTGGGGACGGAACGtATG 70
149                                                                     GACGGAACGTATG 70
149                                                                  TGGGGATGGAATGTAT 70
149                                                              ATTCGTGGGGACGGAA     70
149                                                              ATTTGTGGGGATGGAA     70
```

| | | |
|---|---|---|
| 69 | CTCCTGAGGCTCCAGTGGATGCAGGCAAAGGCACCTCCCGGGGTCAGCGTCCCTACCTGGGGAGCTGGG | -10 |
| 69 | **TTTATTTGGGGAGTTGGG** | -10 |
| 69 | tTttTGAGGtTttAGTGGATGtAGGtAAAGGtAttTttCGGGGTtAGCGTtttTAttTGGGGAGtTGGG | -10 |
| 69 | | |

Figure 64: Amplificate of SEQ ID NO: 44

```
  1 ACAGTGTGGACCCTCAGGGACACCAGAGTCTCCGTGATGTTCTTGCGTATCTGGGCTCGGCGCTGCCGCTCGTGCTTGGG  80
  1 ATAGTGTGGATTTTTAGGGATATT                                                          80
  1 AtAGTGTGGAtttTtAGGGAtAttAGAGTtTtCGTGATGTTtTTGCGTATtTGGGtTCGGCGtTGtCGtTCGTGtTTGGG  80

 81 CTCTGCCGCCACGTCCAGGGCCCGCTCGTACTGGGGCAGGCAGGGGGCACAGCAAGCTGTCAGCAGGGCAGGAGGCCGGC 160
 81 tTtTGtCGttACGTttAGGGttCGtTCGTAtTGGGGtAGGtAGGGGGtAtAGtAAGtTGTtAGtAGGGtAGGAGGtCGGt 160
 81    TGTCGTTACGTTTAGG                                                              160
 81     GTTGTTATGTTTAGGGT                                                            160
 81              TTTAGGGTTTGTTTGTAT                                                  160
 81                TAGGGTTCGTTCGTATT                                                 160

161 AGGAGGCCAGCAGATGCCCACGACTCCCGGGGTGCAGTTACGTGCTAGATGCTGTGTGATGTGGGCACTGACCCGCAACA 240
161 AGGAGGttAGtAGATGtttACGAtTttCGGGGTGtAGTTACGTGtTAGATGtTGTGTGATGTGGGtAtTGAttCGtAAtA 240
161                TTTATGATTTTTGGGGT                                                 240
161                 TTACGATTTTCGGGGT                                                 240

241 CTGAGCTGTTTCTTCATGGGCAAAACAGGGTAAGCACATGGCCCTCCTGGGCGGGGGCTGCATTGTGGAAAGCAGACGC 320
241 tTGAGtTGTTTtTTtATGGGtAAAAtAGGGTAAGtAtATGGGtttTttTGGGCGGGGGtTGtATTGTGGAAAGtAGACGt 320
241                                                                       AAGTAGACGT 320
241                                                                          TAGATGT 320
```

| | | |
|---|---|---|
| 321 | CGGAGAGGGCCCGGTGGGTGTGGCTGCTGGGAGCGGAACGTCGGGGTGCTGCTTCAGGGTCACTGGGATTTATCTCTGGG | 400 |
| 321 | CGGAGAGGGttCGGTGGGTGTGGtTGtTGGGAGCGGAACGTCGGGGTGtTGtTTtAGGGTtAtTGGGATTTATtTtTGGG | 400 |
| 321 | CGGAGA | 400 |
| 321 | TGGAGAGGG | |

| | | |
|---|---|---|
| 401 | GCCCGGGATGAGCCCTCCGCAAAGCTCCAGGCAGGGGAACAGGTCTTG | 480 |
| 401 | **AGGTAGGGGAATAGGTTTTG** | 480 |
| 401 | GttCGGGATGAGtttTtCGtAAAGtTttAGGtAGGGGAAtAGGTtTTG | 480 |

Figure 65: Amplificate of SEQ ID NO: 45

1 GTAGCAAATGGGCTATGGACTTCAGTAACTAGAGTCACGTGTTACCGGCGACACAACCAATGAGGGCGCGAGATCTGTGT 80
1 **GTAGTAAATGGGTTATGGATTTTAG** 80
1 GTAGtAAATGGGtTATGGAtTTtAGTAAtTAGAGTtACGTGTTAtCGGCGAtAtAAttAATGAGGGCGCGAGATtTGTGT 80
1 TACGTGTTATCGGCGA 80
1 TATGTGTTATTGGTGATA 80

81 AAGCAGGGGGAGGCACGTGCCGGGCCGCACGTGTCTGGGAGGGGGGAAGGGGCGGGGCCCACTGAGAGAGGCGGAGGTG 160
81 AAGtAGGGGGAGGtACGTGtCGGGtCGtACGTGTtTGGGAGGGGGGAAGGGGCGGGGtttAtTGAGAGAGGCGGAGGTG 160
81 GTATGTGTTGGGTTGTA 160
81 TACGTGTCGGGTCGTA 160

161 GGTAGATAGACCCGGAGAGACGGCGAAGGAGCTGGAAACCGAGCCAGGGCTGAGCCGGCTGACAACAGGTAAGGAGATTC 240
161 GGTAGATAGAttCGGAGAGACGGCGAAGGAGtTGGAAAtCGAGttAGGGtTGAGtCGGtTGAtAAtAGGTAAGGAGATTC 240
161 TAGATTTGGAGAGATGG 240
161 AGATTCGGAGAGACGG 240

241 GGAGGACAAAGCGAGCTGTTAGGGAGTATTTGGGAGTGGATTTGGGGGCTGAATGCAGATCTTGGTATTGGGAGGGTTTG 320
241 GGAGGAtAAAGCGAGtTGTTAGGGAGTATTTGGGAGTGGATTTGGGGGtTGAATGtAGATtTTGGTATTGGGAGGGTTTG 320

```
321 CATCACGTGGCAGGTGCGAGCCCAGAGAGACCGGCGCAGGGATCCTGATCTTGAAAGATTGGGAGAGGGCAGGAGGCCAG 400
321 tATtACGTGGtAGGTGCGAGtttAGAGAGAtCGGCGtAGGGATttTGATtTTGAAAGATTGGGAGAGGGtAGGAGGttAG 400
321                                         AGATCGGCGTAGGGAT                         400
321                                         AGATTGGTGTAGGGAT

401 TCTCTATGGGGGCGGTTCCCGTAGGATCACCAGGTGGGTTCGCGTGCCCAGATTGACTGCTTGTGGGTGGCATGG      480
401                                                                 TGTTTGTGGGTGGTATGG      480
401 TtTtTATGGGGGCGGTTttCGTAGGATtAttAGGTGGGTTCGCGTGtttAGATTGAtTGtTTGTGGGTGGtATGG      480
```

Figure 66. Amplificate of SEQ ID NO: 46

```
  1 CCCCAGCAGTGGACCTGGTCAGCACCTCTGGGGCTGGGACATCAGTGTCCTAAAGGTGGAAGGGTTAGACCCTCTAGGGG  80
  1 TTTTAGTAGTGGATTTGGTTAGTATT                                                        80
  1 ttttAGtAGTGGAttTGGTtAGtAttTtTGGGGtTGGGAtATtAGTGTttTAAAGGTGGAAGGGTTAGAtttTtTAGGGG  80

 81 AAGGGCCCACCGCCCTCCACAGAGGCTCTGGCTTAGGCCGCGTGGACACGTGAGGGGGGCACCTACCGTGTTCTCCATGG 160
 81 AAGGGtttAtCGtttTttAtAGAGGtTtTGGtTTAGGtCGCGTGGAtACGTGAGGGGGGtAttTAtCGTGTTtTttATGG 160
 81                                           GGTTGTGTGGATATGT                       160
 81                                            GTCGCGTGGATACGTG                      160

161 ACTTGCTGGCGACTCCCACGAGAAGGCCAGCCAGGAGGGCGAGGTGCCGCAGCGCCATGCCAGGAGCAGATGCGCAGAGC 240
161 AtTTGtTGGCGAtTtttACGAGAAGGttAGttAGGAGGGCGAGGTGtCGtAGCGttATGttAGGAGtAGATGCGtAGAGt 240
161      TTGGCGATTTTTACGA                           AGGTGTTGTAGTGTTAT                240
161      TTGGTGATTTTTATGAGA                            TGTCGTAGCGTTATGT              240
161           GATTTTTATGAGAAGGTT                                                     240
161            ATTTTTACGAGAAGGTT

241 CTGCCACAGGGAGGAGCATGCGGAGCCAAAGAGATGGAGTGGGGCTGAGGCAGGGTGGGTGGGGCCAAATGAAAGTGGGG 320
241                                                                      TGAAAGTGGGG 320
241 tTGttAtAGGGAGGAGtATGCGGAGttAAAGAGATGGAGTGGGGtTGAGGtAGGGTGGGTGGGGttAAATGAAAGTGGGG 320
```

```
321 TCAAGAGATG                                                    400
321 TTAAGAGATG                                                    400
321 TtAAGAGATG                                                    400
```

Figure 67: Amplificate of SEQ ID NO: 47

335 GAGGAAAGAAGGAGGATACAGGCGGCCAATGTGTGGCCTACGTGGGAGCGCCCCGAGCGCTCCACGTCCCCGGCCTGCGC 256
335 **GAGGAAAGAAGGAGGATATAGG** 256
335 GAGGAAAGAAGGAGGATAtAGGCGGttAATGTGTGGttTACGTGGGAGCGtttCGAGCGtTttACGTtttCGGttTGCGt 256
335 TTTTGAGTGTTTTATGTT 256
335 TTCGAGCGTTTTACGT 256
335 TTACGTGGGAGCGTTT 256
335 TTATGTGGGAGTGTTT 256

255 CGGGCGGCCCCTTCCCCGGGCATCTTGGCAACGCGCCTGGCCCAAGCCGGAGGGGCCCCGCGGCCCCGCACGCCTCCTGG 176
255 CGGGCGGttttTTtttCGGGtATtTTGGtAACGCGttTGGtttAAGtCGGAGGGGtttCGCGGtttCGtACGttTttTGG 176
255 GTAACGCGTTTGGTTT 176
255 TGGTAATGTGTTTGGT 176

175 ACCAGTCAGGATCCAGGCTCCTCTTTGAGCCCGCGGGTGTTTCATGGGGGTAGAAGTCTAACCCCTCCACCCCCTCTCCT 96
175 AttAGTtAGGATttAGGtTttTtTTTGAGttCGCGGGTGTTTtATGGGGGTAGAAGTtTAAttttTttAttttTtTttT 96

| | | |
|---|---|---|
| 95 | CCCCAGCAGTCCCACGCGGGTATGGGAGAGAATGAAGTTCTTTGTCTCTAAGGGATTCAAACCAGAAACGGAGGGACCTC | 16 |
| 95 | **GATTTT** | 16 |
| 95 | ttttAGtAGTtttACGCGGGTATGGGAGAGAATGAAGTTtTTTGTtTtTAAGGGATTtAAAttAGAAACGGAGGGAttTt | 16 |
| 95 | AGTTTTATGTGGGTATG | 16 |
| 95 | TAGTTTTACGCGGGTA | |
| 15 | TGGTTCCCAGAGGGA | -64 |
| 15 | **TGGTTTTTAGAGGGA** | -64 |
| 15 | TGGTTtttAGAGGGA | -64 |

Figure 68: Amplificate of SEQ ID NO: 48

1 GCCAAAGGGGCATTGGTTCCCCAAGGGCCAAACGAGCTTCCTCCAGGAGTTTCCCGTCGCGTGCAGTGGACCACCGTGGG 80
1 **GTTAAAGGGGTATTGGTTTTT** 80
1 GttAAAGGGGtATTGGTTttttAAGGGttAAACGAGtTTttTttAGGAGTTTttCGTCGCGTGtAGTGGAttAtCGTGGG 80

81 CCTGACTGAGGCCTGGTCCTTGCCAGGGTTTTTTGGCAACGGCCCTGGATGAGTGAAGGGATGAAAGGCGCGAGAAGAAA 160
81 ttTGAtTGAGGttTGGTttTTGttAGGGTTTTTTGGtAACGGtttTGGATGAGTGAAGGGATGAAAGGCGCGAGAAGAAA 160
81 ATGAAAGGCGCGAGAA 160
81 ATGAAAGGTGTGAGAA 160

161 TGGGCTGAGAAACCAAAGTGCGGGGCCCTGAGTGTTTGAGGGGACGCGATGGGAACCGTGGCTCCGCTGCCCTGATTTTA 240
161 TGGGtTGAGAAAttAAAGTGCGGGGtttTGAGTGTTTGAGGGGACGCGATGGGAAtCGTGGtTtCGtTGtttTGATTTTA 240
161 ATGGGAATCGTGGTTT 240
161 ATGGGAATTGTGGTTT 240
161 GGAATTGTGGTTTTGT 240
161 GAATCGTGGTTTCGTT 240

241 AGGGCAGTTACCCTGTCACAGCCACGCTGGAACTGAGGTGTCCTTCCAGCTTCTGTCCACCTCAGAGGAATTCAAAACAA 320
241 AGGGtAGTTAtttTGTtAtAGttACGtTGGAAtTGAGGTGTttTTttAGtTTtTGTttAttTtAGAGGAATTtAAAAtAA 320

```
321 GGGCCCAGTTCTAAGCCCCACAGACCCCAGGATCCCCTAGGGGCCATTAGTCCACCGCGGCAAATGGTTTCCTCACTTTG 400
321 GGGtttAGTTtTAAGttttAtAGAttttAGGATttttTAGGGGttATTAGTttAtCGCGGtAAATGGTTTttTtAtTTTG 400
321                                                                TAGTTTATCGCGGTAA                400
321                                                                   TTTATTGTGGTAAATGGT

401 CTCATTTGTGTTTGTTTTGAAATCCAATTGTCACCTCACTGGGCTGCACAGTTGCTACTGAAACGGTTCAATAGGTGAGA 480
401                                                                  GTTTAATAGGTGAGA 480
401 tTtATTTGTGTTTGTTTTGAAATttAATTGTtAttTtAtTGGGtTGtAtAGTTGtTAtTGAAACGGTTtAATAGGTGAGA 480

481 GAAAAAGAAA                                                                       560
481 GAAAAAGAAA                                                                       560
481 GAAAAAGAAA                                                                       560
```

Figure 69: Amplificate of SEQ ID NO: 49

```
  1 GTAAGAGGGACAGGGAACTGGGGCGCTGGAGCCGGGAAGCGGGTGAGCGAGTCTTCCCAACCGCTATGTCCCCTATTAAA  80
  1 GTAAGAGGGATAGGGAATTGG                                                             80
  1 GTAAGAGGGAtAGGGAAtTGGGGCGtTGGAGtCGGGAAGCGGGTGAGCGAGTtTTtttAAtCGtTATGTttttTATTAAA  80

 81 AAGAACCCCGCTCCTTCGTGAAACCCCTTTTCCCTTAGCCTTTAAGCTGCCGGGCCGCGACGGGGAACTGGCCAACTCAT 160
 81 AAGAAtttCGtTttTTCGTGAAAttttTTTTtttTTAGttTTTAAGtTGtCGGGtCGCGACGGGGAAtTGGttAAtTtAT 160

161 CCTTCGCCGGGCTCTCCGCCCTCCGGGCTCTGGGGCAGAACAGGGTCCTGAGGCGGCGTGGGCGAGGGCAGTCAATGAGC 240
161 ttTTCGtCGGGtTtTtCGtttTtCGGGtTtTGGGGtAGAAtAGGGTttTGAGGCGGCGTGGGCGAGGGtAGTtAATGAGC 240

241 GGGAAGGACGTGAAGAAGGTGACACAGGCTGCCCCCCACTGGAAAATGGGTTGGGAGGGGGTGCGGCCCGAAGACCAGAG 320
241 GGGAAGGACGTGAAGAAGGTGAtAtAGGtTGtttttAtTGGAAAATGGGTTGGGAGGGGGTGCGGttCGAAGAttAGAG 320
241                                                             GGTGTGGTTTGAAGAT     320
241                                                                TGCGGTTCGAAGATTA  320
```

```
321 GATAGCAAAGGTCCACGGAAAAGGGAGCTCCTGGCCCGGACGCGGCGTGGGGAACGCGTCCTAGCGCCGGGGATGCGGAG 400
321 GATAGtAAAGGTttACGGAAAAGGGAGtTttTGGttCGGACGCGGCGTGGGGAACGCGTttTAGCGtCGGGGATGCGGAG 400
321         AAGGTTTATGGAAAAGG                                        GGAATGTGTTTTAGTGT          400
321          AGGTTTACGGAAAAGG                                         GAACGCGTTTTAGCGT          400
321                                                                         TTTTAGCGTCGGGGAT    400
321                                                                         TTTTAGTGTTGGGGAT

401 CTGGGGAGAGGGGTGGGAGCCCCATCCTGGTTTGGGTGCC                                         480
401                     TTTTATTTTGGTTTGGGTGTT                                         480
401 tTGGGGAGAGGGGTGGGAGttttATttTGGTTTGGGTGtt                                         480
```

Figure 70: Amplificate of SEQ ID NO: 50

```
  1 GGAGTTTGTGAAAAGTGGGGCTCGGGGCCCAGTCAATGGGGCGCCCCGCGGCGCGGGCTGAGTGGAGCTAGCGCGAACCG  80
  1 GGAGTTTGTGAAAAGTGGG                                                              80
  1 GGAGTTTGTGAAAAGTGGGGtTCGGGGtttAGTtAATGGGGCGtttCGCGGCGCGGGtTGAGTGGAGtTAGCGCGAAtCG  80
  1                                                                      TAGCGCGAATCG  80
  1                                                                       AGTGTGAATTG  80

 81 CTCAGCCGCGGCCCCAATTAATCCGCCCTTTGTGCGGCCCGCCCGGCCGCCCCCGCCGCAGCCGCACCAGCGGCCCATTG 160
 81 tTtAGtCGCGGttttAATTAATtCGtttTTTGTGCGGttCGttCGGtCGttttCGtCGtAGtCGtAttAGCGGtttATTG 160
 81 TTTA                                                     TAGTCGTATTAGCGGT       160
 81 TTTAGT                                                   TAGTTGTATTAGTGGTTT     160

161 TTCGGCCTCGCCGGGCCGCGGGATTTACCCTTTTCAAACAGCCGGTTTTGTCCAGGGCAGTTCGAGCGGAAGTTTCTCAC 240
161 TTCGGttTCGtCGGGtCGCGGGATTTAtttTTTTtAAAtAGtCGGTTTTGTttAGGGtAGTTCGAGCGGAAGTTTtTtAt 240

241 TGACAATTTGCCCCAAATAGATAGATTTGTCAGAAGCGACCTTCGGGAAGGAAGCAAAAAGCCACCGGCCCGAAGGTTGG 320
241 TGAtAATTTGttttAAATAGATAGATTTGTtAGAAGCGAttTTCGGGAAGGAAGtAAAAAGttAtCGGttCGAAGGTTGG 320
```

```
321 GCCCAAAACAGGGACTCTGCGTCCCACCCGCGGCCGCGCCGCCCCCCCGCGCCCCCGGCCCTGCAGTCCCGAAGCCCCGC 400
321 GtttAAAAtAGGGAtTtTGCGTtttAttCGCGGtCGCGtCGtttttttCGCGttttCGGtttTGtAGTttCGAAGtttCGC 400
321                 TGCGTTTTATTCGCGG                                              TTTCGAAGTTTCGC 400
321                 TGTGTTTTATTTGTGGT                                              TTTGAAGTTTTGT 400

401 GGGGGTCCAGACCACTGCACCTGCTGAGC                                                    480
401    GGTTTAGATTATTGTATTTGTTGAGT                                                    480
401 GGGGGTttAGAttAtTGtAttTGtTGAGt                                                    480
401 GG                                                                               480
401 GGG
```

Figure 71: Amplificate of SEQ ID NO: 51

```
228 GAAGGTTGAGGAGGAGCCAGAGCCGGGTCCTGCAGCGTTTCTCGCCATCAGCGCCCGTCGCCATCTCCACCATGCAGTCC 149
228 GAAGGTTGAGGAGGAGTTAGA                                                            149
228 GAAGGTTGAGGAGGAGttAGAGtCGGGTttTGtAGCGTTTtTCGttATtAGCGttCGTCGttATtTttAttATGtAGTtt 149
228                 TAGAGTCGGGTTTTGTA           TATTAGCGTTCGTCGT                     149
228                 TAGAGTTGGGTTTTGTA         GTTATTAGTGTTTGTTGT                     149
228                                                                              TTT 149
228                                                                            AGTTT 149

148 CGGGAAGACGCCCCGCGCTCTCGCCGCCTAGCCAGTCCCCGTGGTGGGAAGCGGCCCAAGAAGATTCACAAACCCACAGT  69
148 CGGGAAGACGtttCGCGtTtTCGtCGttTAGttAGTttCGTGGTGGGAAGCGGtttAAGAAGATTtAtAAAtttAtAGT  69
148      AGACGTTTCGCGTTTT                                                             69
148    GAAGATGTTTTGTGTTT                                                              69
148 CGGGAAGACGTTT                                                                     69
148 TGGGAAGATGT

 68 TTCGGCCTTTTTCACGGGTCCAGAGGAATTAAAGGACACGGCCCATTCTGCAGCCCTGCTGGCACAGC            -11
 68                                         TTTATTTTGTAGTTTTGTTGGTATAGT            -11
 68 TTCGGttTTTTTtACGGGTtAGAGGAATTAAAGGAtACGGtttATTtTGtAGtttTGtTGGtAtAGt            -11
```

Figure 72 Amplificate of SEQ ID NO: 52

```
  1 GGCTGGAAAGTGGAGGATCCGGTTTGCTCTGGGCGGGTCTGGAAGCAGAGCCGGCGGAGGGAGCGCCGGGGCCCTGGGCT  80
  1 GGTTGGAAAGTGGAGGATT                                                               80
  1 GGtTGGAAAGTGGAGGATtCGGTTTGtTtTGGGCGGGTtTGGAAGtAGAGtCGGCGGAGGGAGCGtCGGGGtttTGGGtT  80
  1                                           AGTAGAGTCGGCGGAG                        80
  1                                           AGTAGAGTTGGTGGAG                        80

 81 GCAGGAGGTTGCGGCGGCCGCGGCAGCATGGTGGTGCCGGAGAAGGAGCAGGTGAGCGCCGGACCAGGGTCTGCGGGAGC 160
 81 GtAGGAGGTTGCGGCGGtCGCGGtAGtATGGTGGTGtCGGAGAAGGAGtAGGTGAGCGtCGGAttAGGGTtTGCGGGAGC 160
 81                                                TAGGTGAGCGTCGGAT                  160
 81                                                TAGGTGAGTGTTGGAT                  160

161 GCGGAGCTGGGGACCTCGCCTCCAGGCTCCCAGAGAGGAGGGCGCTGGACACCCAGATTCCTGGGTCAGACGGAGGAGGG 240
161 GCGGAGtTGGGGAttTCGttTttAGGtTttAGAGAGGAGGGCGtTGGAtAtttAGATTttTGGGTtAGACGGAGGAGGG 240

241 GGATGGGAGGGTCCAGGTTCCAGGGTCCAGGGCATGGGGGTCGAGACTCCTGAGTCTGGAGCGGGAGGGCGCTGGGGGCC 320
241 GGATGGGAGGGTttAGGTTttAGGGTttAGGGtATGGGGGTCGAGAtTttTGAGTtTGGAGCGGGAGGGCGtTGGGGGtt 320
241                                 ATGGGGGTCGAGATTT                                 320
241                                 ATGGGGGTTGAGATTT
```

```
321 CGGACTCCTGTGTCCGTGGGGAGCGCACGGGGTGGGTGGCTCTGTGTCCCATAGGACAGAACTGGGTGCCCTCTCACCCC 400
321 CGGAtTttTGTGTtCGTGGGGAGCGtACGGGGTGGGTGGtTtTGTGTtttATAGGAtAGAAtTGGGTGtttTtTtAtttt 400

401 ACTTCCACCCCTACATTTGTTCCTGTCCCCAGAGCTGG                                           480
401                           TTGTTTTTGTTTTTAGAGTTGG                                 480
401 AtTTttAttttTAtATTTGTTttTGTttttAGAGtTGG                                           480
```

Figure 73: Amplificate of SEQ ID NO: 53

1 GAGCTGGCCCTGCTGAGGTGCCGGGCCGAGTAGTTGGAGAGAGGGTCATACTCCAGGTCTGTGGGTGGCCTGGAGTTGTC 80
1 **GAGTTGGTTTTGTTGAGGTGT** 80
1 GAGtTGGtttTGtTGAGGTGtCGGGtCGAGTAGTTGGAGAGAGGGTtATAtTttAGGTtTGTGGGTGGttTGGAGTTGTt 80
1 TGTCGGGTCGAGTAGT 80
1 TGTTGGGTTGAGTAGT 80

81 CACCACGTACTTGCCACTGGGAACGGGGCGGCTGTGCCGCCGGGGCTGGCTCACAGCCTTGGGGACGTATTCCAGGGCAC 160
81 tAttACGTAtTTGttAtTGGGAACGGGGCGGtTGTGtCGtCGGGGtTGGtTtAtAGttTTGGGGACGTATTttAGGGtAt 160

161 CGCCACCCCCTCCACCTCTGCCCTGACCCCTGTCCAGGGACGCCAGCGAGTACTTGCTGCCCGGCTCGGCAGGGGCGGCC 240
161 CGttAtttttTttAttTtTGtttTGAttttTGTttAGGGACGttAGCGAGTAtTTGtTGttCGGtTCGGtAGGGGCGGtt 240
161 AGGGATGTTAGTGAGT 240
161 GACGTTAGCGAGTATT 240

241 AGTGGGGTGGGCTGGTAGCCGGCATCAGGGCTTAATAGGCCGTGGCTGCCGGGGCTGTAGTCGAAGGCCAGTGGGAAGGC 320
241 **AAGGTTAGTGGGAAGGT** 320
241 AGTGGGGTGGGtTGGTAGtCGGtATtAGGGtTTAATAGGtCGTGGtTGtCGGGGtTGTAGTCGAAGGttAGTGGGAAGGt 320
241 TGGTAGTTGGTATTAGG TGTAGTCGAAGGTTAG 320
241 GGTAGTCGGTATTAGG TGTAGTTGAAGGTTAGT

| | | |
|---|---|---|
| 321 | ATCCT | 400 |
| 321 | **ATTTT** | 400 |
| 321 | ATttT | 400 |

Figure 74: Amplificate of SEQ ID NO: 54

```
  1 CAGTGGAACCATGAGGGGGGAGCCCACCCGGCTCACACGACCTCTCCCTCCGGAGGCCGCTCTAAGCACACCCTCCAGCC  80
  1 TAGTGGAATTATGAGGGGG                                                                80
  1 tAGTGGAAttATGAGGGGGGAGtttAttCGGtTtAtACGAttTtTtttTtCGGAGGtCGtTtTAAGtAtAtttTttAGtt  80
  1                   GGAGTTTATTCGGTTTAT                                            TT  80
  1                   GGAGTTTATTTGGTTTATA                                            T  80
  1                          ATTTGGTTTATATGATTTTT                                       80
  1                           TTCGGTTTATACGATTT                                         80

 81 ACGCCGACCACCGGGGCTGTCCCCGATTCTCACCTAAAACCCCCTTCAGCCCACAGCCCCCACGGCCTTCCTGTGGTCTC 160
 81 ACGtCGAttAtCGGGGtTGTtttCGATTtTtAttTAAAAtttttTTtAGtttAtAGttttTACGGttTTttTGTGGTtTt 160
 81 ACGTCGATTATCGG                                                                  160
 81 ATGTTGATTATTGGGGT                                                               160

161 CTCCCTCCTTCACGGCGGGTCCTCATCCTTTACCTTCTCTCTCTGGAGCCCAAATTGGAACTCCGCTCCTCCCCTCCGCG 240
161 tTttttTttTTtACGGCGGGTttTtATttTTTAttTTtTtTtTtTGGAGtttAAATTGGAAtTtCGtTttTttttTtCGCG 240
161          TTACGGCGGGTTTTTA                                                         240
161          TTATGGTGGGTTTTTATT

241 CAGCTCCATGCACTGGCCCTTTGGGACGCCTCTTTCTTTCTTTGGCAGAGGTGCATCTGG                     320
241                                       TTTTGGTAGAGGTGTATTTGG                     320
241 tAGtTttATGtAtTGGtttTTTGGGACGttTtTTTtTTTtTTTGGtAGAGGTGtATtTGG                     320
```

Figure 75: Amplificate of SEQ ID NO: 55

```
384 TAGGACTTGTGGCTGGTGTTCTCGGCCATGCCCTGGCCCAAGAAGTCGTTGGTGAAGATGCCTCAGCGGAGCGGGGCGCC 305
384 TAGGATTTGTGGTTGGTGTT                                                             305
384 TAGGAtTTGTGGtTGGTGTTtTCGGttATGtttTGGtttAAGAAGTCGTTGGTGAAGATGttTtAGCGGAGCGGGGCGtt 305

304 CGCCGGGCCGCACGTTGGGGTTGTTCACGGTCGCCCACACGTCGTCGTGCACGAACTCGCCCTGCAGGGAGCGGCCTTAG 225
304 CGtCGGGtCGtACGTTGGGGTTGTTtACGGTCGtttAtACGTCGTCGTGtACGAAtTCGtttTGtAGGGAGCGGttTTAG 225
304                                           GTTGTGTATGAATTTGTT                     225
304                                           GTCGTGTACGAATTCGT                      225

224 CACAGGCAGCTCGCCCCGGCCAGCAGCACCTCTTCCTCCCCACTGCGAATGCCCCGATCATGCTCCCCCAGACCCAACAC 145
224 tAtAGGtAGtTCGtttCGGttAGtAGtAttTtTTttTttttAtTGCGAATGtttCGATtATGtTtttttAGAtttAAtAt 145
224       AGTTTGTTTTGGTTAGT                                                          145
224      TAGTTCGTTTCGGTTA                                                            145

144 ACAAACACCCTTGGGAACCCACGCGGGTGCCTTCTGTTTACCGCAGCGCTCAAGCCGCGGGCGCTTGAGTCCCAGATGGC  65
144 AtAAAtAtttTTGGGAAtttACGCGGGTGttTTtTGTTTAtCGtAGCGtTtAAGtCGCGGGCGtTTGAGTtttAGATGGC  65
144              AATTTACGCGGGTGTT               AGCGTTTAAGTCGCGG                      65
144            GGAATTTATGTGGGTG                TAGTGTTTAAGTTGTGG
```

| | | |
|---|---|---|
| 64 | GAAAGTTCGCGCGCAGGTGGAGTGGGGATCCCGGCGTGCGGCTGCCCAGGAGAGGAGGGCAAGG | -15 |
| 64 | **TTTAGGAGAGGAGGGTAAGG** | -15 |
| 64 | GAAAGTTCGCGCGtAGGTGGAGTGGGGATttCGGCGTGCGGtTGtttAGGAGAGGAGGGtAAGG | -15 |

Figure 76: Amplificate of SEQ ID NO: 56

```
  1 GGCTCAGGGCACCCTAAGGGGCCGGATTTCCAGGGGTCCCATCTTGGCTGTGGTGGAGAGCTGGACTCAGGTCTCTCTTG  80
  1 GGTTTAGGGTATTTTAAGGGG                                                              80
  1 GGtTtAGGGtAtttTAAGGGGtCGGATTTtAGGGGTtttATtTTGGtTGTGGTGGAGAGtTGGAtTtAGGTtTtTtTTG  80

 81 GGGGCCCTGGGGACCAGAGCCACGATCCCGGTGCCCTGATGTCCCCGTCTCTTCCCCACCTTCCTCTCCCCCAGTGTCTG 160
 81 GGGGtttTGGGGAttAGAGttACGATttCGGTGtttTGATGTtttCGTtTtTTttttAttTTttTtTtttttAGTGTtTG 160
 81               TAGAGTTATGATTTTGGT                                                 160
 81                    TTACGATTTCGGTGTT                                              160

161 CCTCAGGACGAGGCGTGCGCCGACACTGGCAGTGGCAGCGCCGAGGGCCTGGCTGCTGACGGCCCCCACCTGCACACGCT 240
161 ttTtAGGACGAGGCGTGCGtCGAtAtTGGtAGTGGtAGCGtCGAGGGttTGGtTGtTGACGGttttAttTGtAtACGtT 240
161           AGGCGTGCGTCGATAT        GTAGCGTCGAGGGTTT                   TTGTATATGTT 240
161           AGGTGTGTGTTGATATT       GTAGTGTTGAGGGTTT                       ATACGTT 240

241 GACGCAGCCTATCGTGGTCACCGTGCCGCGGCCGCCCCCCAGTGAGCACGCAGTCCTCCTCCGCATGGGGCCGTGGGGCG 320
241 GACGtAGttTATCGTGGTtAtCGTGtCGCGGtCGtttttAGTGAGtACGtAGTttTttTtCGtATGGGGtCGTGGGGCG 320
241 GATGTAGT                                                                         320
241 GACGTAGTT
```

```
321 GCAGAGCGGGTGGGAAGGACGCCTGGGAGCTGGACCCAGTCT          400
321                            TTTGGGAGTTGGATTTAGTTT   400
321 GtAGAGCGGGTGGGAAGGACGttTGGGAGtTGGAtttAGTtT          400
```

Figure 77: Amplificate of SEQ ID NO: 57

```
378 GGGGAGGTGCTCAGTGGTCCTAGTCGCCCCGAGACCCTAGCTCTTCTCGCCCGCTGCGGGCCTGACTGCGCCAGGGGCCG 299
378 GGGGAGGTGTTTAGTGGTT                                                              299
378 GGGGAGGTGtTtAGTGGTttTAGTCGtttCGAGAtttTAGtTtTTtTCGttCGtTGCGGGttTGAtTGCGttAGGGGtCG 299
378                    TTAGTCGTTTCGAGAT                                              299
378                 GTTTTAGTTGTTTTGAGA                                               299

298 GGGGTTATGTGGGCAGGCTCGAACGACTGGGAGCGTGGCCTGTGATTCTGCTCCACTCCTTTCGCCCGCAGAGCGGTGGT 219
298 GGGGTTATGTGGGtAGGtTCGAACGAtTGGGAGCGTGGttTGTGATTtTGtTttAtTttTTTCGttCGtAGAGCGGTGGT 219

218 CTAGGCCAGACCATTGACCTGGGTAGGGGGAAGGGCAAGGACGCAGCTCGCACGCCTGGGTTTTCACCGCCGTCTCCCTT 139
218 tTAGGttAGAttATTGAttTGGGTAGGGGGAAGGGtAAGGACGtAGtTCGtACGttTGGGTTTTtAtCGtCGTtTtttTT 139
218                                        GACGTAGTTCGTACGT                          139
218                                       GGATGTAGTTTGTATGT                          139

138 CGGCGTTTTGGGCTGGCGGCCGACATTGGCCTGGACCAAATGGTGGCAAAGTCTCACCAGTTCCGCGTCTCGCAAGACAC  59
138 CGGCGTTTTGGGtTGGCGGtCGAtATTGGttTGGAttAAATGGTGGtAAAGTtTtAttAGTTtCGCGTtTCGtAAGAtAt  59
138                                                                TTTGTGTTTTGTAAGATAT  59
138                                                                TTCGCGTTTCGTAAGA     59
```

| | | |
|---|---|---|
| 58 | TTTCTACGCGCATCGGGACCTAAACACTCGGGAGGACCCTGGTAAAGTGAGCAGAGGG | -21 |
| 58 | **TTTTGGTAAAGTGAGTAGAGGG** | -21 |
| 58 | TTTtTACGCGtATCGGGAttTAAAtAtTCGGGAGGAtttTGGTAAAGTGAGtAGAGGG | -21 |
| 58 | TATGTGTATTGGGATTTA | -21 |
| 58 | TTACGCGTATCGGGAT | |

Figure 78: Amplificate of SEQ ID NO: 58

```
  1 TGTTGGTGGCCATCTTTAATCCTCCTCCTCCTCCTGCTTTCTCCACCTCCCGCTGGCTGTCTGACTGACTGACTGGATCC  80
  1 TGTTGGTGGTTATTTTTAATTTTT                                                          80
  1 TGTTGGTGGttATtTTTAATttTttTttTttTttTGtTTTtTttAttTttCGtTGGtTGTtTGAtTGAtTGAtTGGATtt  80

 81 TCCTCTTTCCCCTCCTGCTCCTCCTACTGAGCCGGCCGCAGAAATTGCAGCCGCTCAGCTTCTACCCCCTCCTGCCTTTC 160
 81 TttTtTTTttttTttTGtTttTttTAtTGAGtCGGtCGtAGAAATTGtAGtCGtTtAGtTTtTAtttttTttTGttTTTt 160
 81                         TATTGAGTCGGTCGTA  AATTGTAGTCGTTTAGTT                         160
 81                          ATTGAGTTGGTTGTAGA                                           160
 81                                          AAATTGTAGTTGTTTAGTT                         160

161 CTTCCTCTTTCCTTACTTCCTTCCCTTCCCTCGGCTTCCCGCTCTTGCCTCACTCTCAGCGGCTGCCTTCGCCCCTGTCT 240
161 tTTttTtTTTttTTAtTTttTTtttTTtttTCGGtTTttCGtTtTTGttTtAtTtTtAGCGGtTGttTTCGttttTGTtT 240

241 GCAGACAGCGCCGCTGGATGCTCCCAGCTGGACTTCAACCCCACTCCTCTCAGTCCCTCTCCCCACTGCCTTCCAGACGC 320
241 GtAGAtAGCGtCGtTGGATGtTtttAGtTGGAtTTtAAtttAtTttTtTtAGTtttTtTttttAtTGttTTttAGACGC 320
241  TAGATAGCGTCGTTGG                                                                320
241  TAGATAGTGTTGTTGGA     .
```

```
321 GCCTCTTCCCCGCCCCGCGCCCCTCTCTCCTCTCCCACCCCTGCCCCTCTCCGCGGCGCTCACCCTCCTCAGTCCCAGTT 400
321 GttTtTTtttCGtttCGCGttttTtTtTttTtTtttAttttTGttttTtTtCGCGGCGtTtAtttTttTtAGTtttAGTT 400

401 TCTGAAAGGACTCAGCTGAGAAAGGACAACTGGGT                                              480
401                     GTTGAGAAAGGATAATTGGGT                                        480
401 TtTGAAAGGAtTtAGtTGAGAAAGGAtAAtTGGGT                                              480
```

Figure 79: Amplificate of SEQ ID NO: 59

```
  1 GAGGACCTGTGCTAAGGCTTTCTCATCCACCAGGCCACCATGGGCTGCGTTCACAAGGAATGCTCCCTGTCTCATCTGCT  80
  1 GAGGATTTGTGTTAAGGTTTTT                                                            80
  1 GAGGAttTGTGtTAAGGtTTTtTtATttAttAGGttAttATGGGtTGCGTTtAtAAGGAATGtTtttTGTtTtATtTGtT  80
  1                                               ATGGGTTGCGTTTATA                    80
  1                                                TGGGTTGTGTTTATAAG                  80

 81 TTATAGTAAAGTCATTGACGAGGTGGTGGTTATGTTCATTGAGATTGCAGTGCAACGAGACACAGTCACTCTGATACAGC 160
 81 TTATAGTAAAGTtATTGACGAGGTGGTGGTTATGTTtATTGAGATTGtAGTGtAACGAGAtAtAGTtAtTtTGATAtAGt 160

161 AAACCCTGCAGGGTGTATCAGGGTCCCCTCTGCATGCCCTGGGACCTCTCTATCTTGTCCTACAAGTAGGGGTCATAAAA 240
161 AAAtttTGtAGGGTGTATtAGGGTttttTtTGtATGtttTGGGAttTtTtTATtTTGTttTAtAAGTAGGGGTtATAAAA 240
161                                                                           ATAAAA 240
161                                                                           ATAAAA 240

241 TACGACGCTGAATCCAAAGGCCTTGGCTCAAACTGCAACCGCCTGCCTCATGCAACCGAAGCCCATGAGGCCTAGCGTCT 320
241 TACGACGtTGAATttAAAGGttTTGGtTtAAAtTGtAAtCGttTGttTtATGtAAtCGAAGtttATGAGGttTAGCGTtT 320
241 TACGACGTTGA                                                             TAGTGTTT 320
241 TATGATGTTGAATT                                                           AGCGTTT 320
```

```
321 TCCACGAATGAGGGCCACTCCCATGGCCACCTCGAGAATCTGCTCCACGCTCTGAACCCGCGCGCCTTCCCACAGTGTCT 400
321 TttACGAATGAGGGttAtTtttATGGttAttTCGAGAATtTGtTttACGtTtTGAAttCGCGCGttTTtttAtAGTGTtT 400
321 TTTATGAATGA                                                                       400
321 TTTACGAAT                                                                         400

401 GGTACAGCCACATATTCCTCCGGCAGAGATTGAGGATGTGGCAGATAGTGGAGTTGG                        480
401                                  ATGTGGTAGATAGTGGAGTTGG                        480
401 GGTAtAGttAtATATTttTtCGGtAGAGATTGAGGATGTGGtAGATAGTGGAGTTGG                        480
401             TATTTTTTCGGTAGAGAT                                                    480
401               TTTTTTTGGTAGAGATTG
```

Figure 80: Amplificate of SEQ ID NO: 60

```
  1 GGGGTCTCAAGAGCTGGCTCCTACACATAAAGGGCTTAGAACAGTGGCTGGTACCTAGGCCACCAATAGTCTTTTGCCCC  80
  1 GGGGTTTTAAGAGTTGGTTTT                                                            80
  1 GGGGTtTtAAGAGtTGGtTttTAtAtATAAAGGGtTTAGAAtAGTGGtTGGTAttTAGGttAttAATAGTtTTTTGtttt  80

 81 ACCCAACCGGTGACACAGCCCGGGGCCCCGCCGCCCCCTGGCGGCCATTACGGATTTCCGCCTCCCTCACAGAAGGCAGT 160
 81 AtttAAtCGGTGAtAtAGttCGGGGtttCGtCGtttttTGGCGGttATTACGGATTTtCGttTtttTtAtAGAAGGtAGT 160
 81                                             GTTATTATGGATTTTTGTT                  160
 81                                               TATTACGGATTTTCGTT                  160

161 CACTGCAACGTGCGTGGCCTCAGTTGCGTCATATCCGGCCCTTGCGATCAGGGCTTGAGGAACCCGCGCCATGAAGTGCG 240
161 tAtTGtAACGTGCGTGGttTtAGTTGCGTtATATtCGGtttTTGCGATtAGGGtTTGAGGAAttCGCGttATGAAGTGCG 240
161                      AGTTGTGTTATATTTGGT               TTGAGGAATTTGTGTTAT          240
161                         TGCGTTATATTCGGTT                   AATTCGCGTTATGAAG      240

241 TGTTTGTTACCGTAGGGACCACCAGCTTTGACGACCTCATTGCGTGTGTGTCGGCGCCCGACAGTCTGCAAGTGAGTGAG 320
241                                                                 TTGTAAGTGAGTGAG 320
241 TGTTTGTTAtCGTAGGGAttAttAGtTTTGACGAttTtATTGCGTGTGTGTCGGCGttCGAtAGTtTGtAAGTGAGTGAG 320
241                                                  TGTTGGTGTTTGATAGT               320
241                                                   GTCGGCGTTCGATAGT
```

| | | |
|---|---|---|
| 321 | GGAGG | 400 |
| 321 | **GGAGG** | 400 |
| 321 | GGAGG | 400 |

Figure 81: Amplificate of SEQ ID NO: 61

1 TGGGGACACTGGATGTTTTTCCTCTGAGGCCGTGGAGGTTCACATCCCTGCCGAGGTGTGGGTGGCCCCTTTTCCGCTGG 80

1 **TGGGGATATTGGATGTTTTT** 80

1 TGGGGAtAtTGGATGTTTTTttTtTGAGGtCGTGGAGGTTtAtATtttTGtCGAGGTGTGGGTGGttttTTTTtCGtTGG 80

81 TAAACAATCCCACACCTGGGGCTGTGCTTCTCCCCAGGGCGAGGCTACTGTGCCGTTTTCCTGGGCTGCTCAAAAGCTGG 160

81 TAAAtAATtttAtAttTGGGGtTGTGtTTtTttttAGGGCGAGGtTAtTGTGtCGTTTTttTGGGtTGtTtAAAAGtTGG 160

81 TTTAGGGCGAGGTTAT 160

81 TTTAGGGTGAGGTTATT 160

161 GGGCACTCTTGTGGGGCCGTCTGTCTTGCTGCTGCCTCTCCGGGGCAAGCTGTTAGAGCAGCCCCACCCGCAAACCTGCA 240

161 GGGtAtTtTTGTGGGGtCGTtTGTtTTGtTGtTGttTtTtCGGGGtAAGtTGTTAGAGtAGttttAttCGtAAAttTGtA 240

241 AACCAGGCTGGCCCCACAGAACCTGAAAACCTGCAAACCGGGCTGGCCCCACAGAACCCACAAACCTGCAGACTGGGCTG 320

241 AAttAGGtTGGttttAtAGAAttTGAAAAttTGtAAAtCGGGtTGGttttAtAGAAtttAtAAAttTGtAGAtTGGGtTG 320

```
321 GCCCCACAGAACCCGCAAACGGGGGCTGGCCCCACAGAACCCGCCTTTGCAGCTCAGTGCTGAATGTTCTCGTCCCCGGT 400
321 GttttAtAGAAttCGtAAACGGGGGtTGGttttAtAGAAttCGttTTTGtAGtTtAGTGtTGAATGTTtTCGTtttCGGT 400
321           AGAATTCGTAAACGGG                                                             400
321              ATTTGTAAATGGGGGT                                                          400

401 GGTACCCTGGCCTGAGAGAGGAAGGGCTCTGGGAATTCGGTCACCGACGGCTGCACACCCCGTAGGGTAGGTGGGTTCTG 480
401                                                                                G 480
401 GGTAtttTGGttTGAGAGAGGAAGGGtTtTGGGAATTCGGTtAtCGACGGtTGtAtAtttCGTAGGGTAGGTGGGTTtTG 480
401                                            TTCGGTTATCGACGGT                          480
401                                            TTTGGTTATTGATGGTT                         480
401                                                           TTGTATATTTTGTAGGGT         480
401                                                            TGTATATTTCGTAGGGT

481 GAGGCCAGGCTGGAAGG                                                                  560
481 GAGGTTAGGTTGGAAGG                                                                  560
481 GAGGttAGGtTGGAAGG                                                                  560
```

Figure 82: Amplificate of SEQ ID NO: 62

```
342 GGAGCTTCAATTCCTGGGACCCCTCCTACTGCGGGGAGAGTGGTTTCCCTGTCCCCAGAGGATGCTCCAGGCCCGAGTCT 263
342 GGAGTTTTAATTTTTGGGATTT                                                             263
342 GGAGtTTtAATTttTGGGAttttTttTAtTGCGGGGAGAGTGGTTTtttTGTttttAGAGGATGtTttAGGttCGAGTtT 263
342                                                                     AGGTTCGAGTTT 263
342                                                                    TAGGTTTGAGTTT 263

262 GCGGCGCTCTGGGGGATGCTCTCCGAGCTCCGACACCGTGTTCGGACCGGGTGCGCCCGCTGCCGCTGGGGCTGAAGCCT 183
262 GCGGCGtTtTGGGGGATGtTtTtCGAGtTtCGAtAtCGTGTTCGGAtCGGGTGCGttCGtTGtCGtTGGGGtTGAAGttT 183
262 GCGG                                   ATCGTGTTCGGATCGG                               183
262 GTGG                                  TATTGTGTTTGGATTGG                               183

182 GCAGGCGTGAGAACCGGGGGACTCTCTATGGCACCAGGAGCTTCACCGTGAGCGTAGCGCAGAAGCGCTTCGCTTTGATC 103
182 GtAGGCGTGAGAAtCGGGGGAtTtTtTATGGtAttAGGAGtTTtAtCGTGAGCGTAGCGtAGAAGCGtTTCGtTTTGATt 103
182  TAGGCGTGAGAATCGG                          ATTGTGAGTGTAGTGTA                          103
182  TAGGTGTGAGAATTGG                          ATCGTGAGCGTAGCGT                           103
```

| | | |
|---|---|---|
| 102 | CTAGCGCTTACAAAAGTCGTCCTTTGGCTGCCCATGATGGTAAAAGTGCAGTTGCTCACAAAGCGCGAGTGTGTGTGCCA | 23 |
| 102 | **A** | 23 |
| 102 | tTAGCGtTTAtAAAAGTCGTttTTTGGtTGtttATGATGGTAAAAGTGtAGTTGtTtAtAAAGCGCGAGTGTGTGTGttA | 23 |
| 102 | TATAAAGTGTGAGTGTG | 23 |
| 102 | TATAAAGCGCGAGTGT | |
| 22 | GACAGTGTAAATGAGTGTTGGG | -57 |
| 22 | **GATAGTGTAAATGAGTGTTGGG** | -57 |
| 22 | GAtAGTGTAAATGAGTGTTGGG | -57 |

Figure 83: Amplificate of SEQ ID NO: 8

```
  1 GGGAGTTTTTAAGCTCTGTGAGAATCCTGGGAGTTGGTGATGTCAGACTAGTTGGGTCATTTGAAGGTTAGCAGCCCGGG  80
  1 GGGAGTTTTTAAGTTTTGTGAG                                                            80
  1 GGGAGTTTTTAAGtTtTGTGAGAATttTGGGAGTTGGTGATGTtAGAtTAGTTGGGTtATTTGAAGGTTAGtAGttCGGG  80

 81 TAGGGTTCACCGAAAGTTCACTCGCATATATTAGGCAATTCAATCTTTCATTCTGTGTGACAGAAGTAGTAGGAAGTGAG 160
 81 TAGGGTTtAtCGAAAGTTtAtTCGtATATATTAGGtAATTtAATtTTTtATTtTGTGTGAtAGAAGTAGTAGGAAGTGAG 160

161 CTGTTCAGAGGCAGGAGGGTCTATTCTTTGCCAAAGGGGGGACCAGAATTCCCCCATGCGAGCTGTTTGAGGACTGGGAT 240
161 tTGTTtAGAGGtAGGAGGGTtTATTtTTTGttAAAGGGGGGAttAGAATTtttttATGCGAGtTGTTTGAGGAtTGGGAT 240
161                                                                             GGAT 240
161                                                                               AT 240

241 GCCGAGAACGCGAGCGATCCGAGCAGGGTTTGTCTGGGCACCGTCGGGGTAGGATCCGGAACGCATTCGGAAGGCTTTTT 320
241 GtCGAGAACGCGAGCGATtCGAGtAGGGTTTGTtTGGGtAtCGTCGGGGTAGGATtCGGAACGtATTCGGAAGGtTTTTT 320
241 GTTGAGAATGTGA                                   TATCGTCGGGGTAGGA    GAATGTATTTGGAAGGT     320
241 GTCGAGAACGCGAG                                  TATTGTTGGGGTAGGA     AACGTATTCGGAAGGT     320
241             AGCGATTCGAGTAGGG                                                          320
241             AGTGATTTGAGTAGGG
```

```
321 GCAAGCATTTACTTGGAAGGAGAACTTGGGATCTTTCTGGGAACCCCCCGCCCCGGCTGGATTGGCCGAGCAAGCCTGGA 400
321 GtAAGtATTTAtTTGGAAGGAGAAtTTGGGATtTTTtTGGGAAtttttCGtttCGGtTGGATTGGtCGAGtAAGttTGGA 400

401 AAATGGTAAATGATCATTTGGATCAATTACAGGCTTTTAGCTGGCTTGTCTGTCATAATTCATGATTCGGGGCTGGGAAA 480
401                                                                      GGGTTGGGAAA 480
401 AAATGGTAAATGATtATTTGGATtAATTAtAGGtTTTTAGtTGGtTTGTtTGTtATAATTtATGATTCGGGGtTGGGAAA 480

481 AAGACCAA                                                                         560
481 AAGATTAA                                                                         560
481 AAGAttAA                                                                         560
```

Figure 84: Amplificate of SEQ ID NO: 9

```
  1 AGAGGGGGTAGTCCCCACTCTCCTGTCTGATCCCTCCCTCTCCTCCCCGAGTTCCACCGCCCCAGGCGCACAGGTTTCCG  80
  1 AGAGGGGGTAGTTTTTATTTTT                                                             80
  1 AGAGGGGGTAGTttttAtTtTttTGTtTGATtttTtttTtTttTttCGAGTTtAtCGttttAGGCGtAtAGGTTTtCG  80
  1                                                 TTTATTGTTTTAGGTGTAT                80
  1                                                   TATCGTTTTAGGCGTA                 80

 81 CCAGATGTCTTTTCTTCTTCGCAGTCTTTGCCCGAGCGCTTCCGAGAGCCAGTTCTGGACTGATCGCCTTGGATGGGATA 160
 81 ttAGATGTtTTTTtTTtTTCGtAGTtTTTGttCGAGCGtTTtCGAGAGttAGTTtTGGAtTGATCGttTTGGATGGGATA 160
 81                              GAGTGTTTTTGAGAGTT                                 160
 81                               AGCGTTTTCGAGAGTT                                 160

161 CCGGGGGAGGGCAGAAGGACACTTGGCTTCCTCTCCAGGAATCTGAGCGGCCCTGAGGTCCGGGGGCGCAGGGAATCCCC 240
161 tCGGGGGAGGGtAGAAGGAtAtTTGGtTTttTtTttAGGAATtTGAGCGGtttTGAGGTtCGGGGGCGtAGGGAATtttt 240
161                                    AATTTGAGTGGTTTTGA                           240
161                                     ATTTGAGCGGTTTTGA                           240

241 TCTCCCGCCGCCGCCGCCGTGTCTGGTCTGTACGTCTTTAGAGGGTCGAGGAAGTCACGTCGGGACAGACTGGGGCGAGT 320
241 TtTttCGtCGtCGtCGtCGTGTtTGGTtTGTACGTtTTTAGAGGGTCGAGGAAGTtACGTCGGGAtAGAtTGGGGCGAGT 320
241                                              GGAAGTTATGTTGGGA                  320
241                                                  TTACGTCGGGATAGAT
```

| | | |
|---|---|---|
| 321 | AAGGTTAAGAAAGGCTGACATGTT | 400 |
| 321 | **AAGGTTAAGAAAGGTTGATATGTT** | 400 |
| 321 | AAGGTTAAGAAAGGtTGAtATGTT | 400 |

Figure 85: Amplificate of SEQ ID NO: 10

```
  1 AGTGGGTAGGCCAAGTGTGTTGCTTCAGCAAACCGGACCAGGAGGGCCAGGGCCGGATGTGGGGACCCTCTTCCTCTAGC  80
  1 AGTGGGTAGGTTAAGTGTGTTG                                                            80
  1 AGTGGGTAGGttAAGTGTGTTGtTTtAGtAAAtCGGAttAGGAGGGttAGGGtCGGATGTGGGGAtttTtTTttTtTAGt  80
  1                                  AGTAAATCGGATTAGGA                                80
  1                                  AGTAAATTGGATTAGGAG                               80

 81 ACAGTAAAGCTGGCCTCCAGAAACACGGGTATCTCCGCGTGGTGCTTTGCGGTCGCCGTCGTTGTGGCCGTCCGGGGTGG 160
 81 AtAGTAAAGtTGGttTttAGAAAtACGGGTATtTtCGCGTGGTGtTTTGCGGTCGtCGTCGTTGTGGtCGTtCGGGGTGG 160
 81                              ATATGGGTATTTTTGTGT                                  160
 81                               TACGGGTATTTTCGCGT                                  160

161 GGTGTGAGGAGGGGACGAAGGAGGGAAGGAAGGGCAAGGCGGGGGGGGCTCTGCGAGAGCGCGCCCAGCCCCGCCTTCGG 240
161 GGTGTGAGGAGGGGACGAAGGAGGGAAGGAAGGGtAAGGCGGGGGGGGtTtTGCGAGAGCGCGtttAGtttCGttTTCGG 240
161                                                         GAGAGTGTGTTTAGTTT        240
161                                                          AGAGCGCGTTTAGTTT

241 GCCCCACAGTCCCTGCACCCAGGTTTCCATTGCGCGGCTCTCCTCAGCTCCTTCCCGCCGCCCAGTCTGGATCCTGGGG  320
241                                                             TTTAGTTTGGATTTTGGGG  320
241 GttttAtAGTtttTGtAtttAGGTTTttATTGCGCGGtTtTttTtAGtTttTTttCGtCGtttAGTtTGGATttTGGGG  320
```

Figure 86: Amplificate of SEQ ID NO: 11

```
  1 AGCTGGAGAAACTGAAAAGATCACAAGCGACTTAACGATAAGCCCCTTCTTCCTTTTAAAGACCGAGAGGAGGGTAGAGG  80
  1 AGTTGGAGAAATTGAAAAGATTA                                                           80
  1 AGtTGGAGAAAtTGAAAAGATtAtAAGCGAtTTAACGATAAGttttTTtTTttTTTTAAAGAtCGAGAGGAGGGTAGAGG  80
  1                       ATAAGCGATTTAACGAT                                          80
  1                          AGTGATTTAATGATAAGTT                                      80

 81 GGAGTAGTGCCTGAGCCCACGTGACCGAGCCAGGGAGCCCGACGGTCTCAGGAACGCCCGACGCCGCGCGTGACCTCTAA 160
 81 GGAGTAGTGttTGAGtttACGTGAtCGAGttAGGGAGttCGACGGTtTtAGGAACGttCGACGtCGCGCGTGAttTtTAA 160
 81                                              TTTAGGAATGTTTGATGT                  160
 81                                                TAGGAACGTTCGACGT                  160

161 GTGGGAGCACCCTCGAACCGACTCCTGGTCCACCCACAAGGATAGTGGCGCACAGATGGCGCTCCCCGCAGCCCCAGTCT 240
161 GTGGGAGtAtttTCGAAtCGAtTttTGGTttAtttAtAAGGATAGTGGCGtAtAGATGGCGtTttCGtAGttttAGTtT 240
161                                                         TAGATGGTGTTTTTTGT        240
161                                                            ATGGCGTTTTTCGTAG      240

241 CAGATTTAAGAGGTCTGGAGTAGGGCCTGAGAATATGCATTTCCAACCAGGTCCTGGGGGATGCCGACACTGATACAGCC 320
241 tAGATTTAAGAGGTtTGGAGTAGGGttTGAGAATATGtATTTttAAttAGGTttTGGGGGATGtCGAtAtTGATAtAGtt 320
```

(Line 2 of positions 1–80, AGTTGGAGAAATTGAAAAGATTA, is printed in bold; the primer sequences in the shorter lines are underlined.)

| | | |
|---|---|---|
| 321 | AGTCTGGGGACCACACTTCGAGGATCACGTCCTCAGCCCCTGACTCACATAAGTGTCATTCAGAACAGATGTCTGATTCT | 400 |
| 321 | AGTtTGGGGAttAtAtTTCGAGGATtACGTttTtAGttttTGAtTtAtATAAGTGTtATTtAGAAtAGATGTtTGATTtT | 400 |
| 321 | ATATTTTGAGGATTATGTT | 400 |
| 321 | ATTTCGAGGATTACGT | |
| 401 | AAGGAGCCAGTGGTGAAACCAGAGAGGCTTTGGGTTTGTCA | 480 |
| 401 | **TAGAGAGGTTTTGGGTTTGTTA** | 480 |
| 401 | AAGGAGttAGTGGTGAAAttAGAGAGGtTTTGGGTTTGTtA | 480 |

Figure 87: Amplificate of SEQ ID NO: 12a

```
  1 CCCAGCTGTGAAAAAGGGACCCAGGACCCGCAGATGCTGGCGAGGAACAGGGGTGCGGAGCATGAGTCGGCTTTGCACCA  80
  1 TTTAGTTGTGAAAAAGGGATTT                                                            80
  1 tttAGtTGTGAAAAAGGGAtttAGGAttCGtAGATGtTGGCGAGGAAtAGGGGTGCGGAGtATGAGTCGGtTTTGtAttA  80

 81 CGCCCTGGGGTCTCCAGCACTGAAGCGTTCAGTACCATGCAGAGCAGCTGGGAGCGGGTCCCGCTGGGCGGGGCCGAGCT 160
 81 CGtttTGGGGTtTttAGtAtTGAAGCGTTtAGTAttATGtAGAGtAGtTGGGAGCGGGTttCGtTGGGCGGGGtCGAGtT 160

161 GCGCGCGACCCTCGGCGCGCTCGGGGAGGCCCAGACAGGGGTGGCCTCTCTGGCCTCCGCTCCCGCGGGCTCTATGACAC 240
161 GCGCGCGAtttTCGGCGCGtTCGGGGAGGtttAGAtAGGGGTGGttTtTtTGGttTtCGtTttCGCGGGtTtTATGAtAt 240
161                                                                               AT 240
161                                                                               AT 240

241 CGCACTGGCTCGCGGGACGGGGCGGGGTCGGGTGAGGGGGAGGAGACAGCCCCACGCTTTGCGACTCGCGGTGACCCCTA 320
241 CGtAtTGGtTCGCGGGACGGGGCGGGGTCGGGTGAGGGGGAGGAGAtAGttttACGtTTTGCGAtTCGCGGTGAttttTA 320
241 CGTATTGGTTCGCGG                                          AGTTTTATGTTTTGTGAT      GTGATTTTTA 320
241 TGTATTGGTTTGTGG                                             TTTACGTTTTGCGATT       ATTTTTA 320
```

```
321 CGCGGAACTCTCTCGCGGTAATTCGAAGTACCGCGCCTGCGTGCTGCAGTAGCGCCTGGTGGCGGTGGCAGTTTGCCCGC 400
321 CGCGGAAtTtTtTCGCGGTAATTCGAAGTAtCGCGttTGCGTGtTGtAGTAGCGttTGGTGGCGGTGGtAGTTTGttCGC 400
321 TGTGGAAT                    ATTCGAAGTATCGCGT                                      400
321 CGCGGAATT                   ATTTGAAGTATTGTGTTT

401 GGGTGTGTGAAGGGAGACAGTGTGGAGGCCACAGGG                                              480
401           GAGATAGTGTGGAGGTTATAGGG                                                 480
401 GGGTGTGTGAAGGGAGAtAGTGTGGAGGttAtAGGG                                              480
```

Figure 88: Amplificate of SEQ ID NO: 12b

497 GTCTTGGAGGGAGTAGAGACCCATCTTCAGCCTGCCTCACCCCCTTGGATAAAGAAAATGCCCCCTTTCTATCATTCAAA 418
497 **CCCTATAACCTCCACACTA** 418
497 GTtTTGGAGGGAGTAGAGAtttATtTTtAGttTGttTtAtttttTTGGATAAAGAAAATGtttttTTTtTATtATTtAAA 418

417 ATTATTTTTAGGTCGGTTTTGTTTACTCCGAACCCCGAGTAGAGCACATGGGGAGCCCAAAATAAGTGCATACTTGAATG 338
417 **TCTC** 338
417 ATTATTTTTAGGTCGGTTTTGTTTAtTtCGAAtttCGAGTAGAGtAtATGGGGAGtttAAAATAAGTGtATAtTTGAATG 338
417 TTTGAATTTTGAGTAGAG 338
417 ATTTCGAATTTCGAGTA 338

337 GATAATTCCCAGAGAACTGCTGCTTGCAACATAGTTTGTAATACCAAAAAATTGAAATCTATATTAACACCCATCAGTAG 258
337 GATAATTtttAGAGAAtTGtTGtTTGtAAtATAGTTTGTAATAttAAAAAATTGAAATtTATATTAAtAtttATtAGTAG 258

257 GAAAATGGATAAATGAATAATGCCTCTATCCTGTTTTTGAAATATGATTACAGCCGTTAAAACGTAGATCTGTGGTCTGA 178
257 GAAAATGGATAAATGAATAATGttTtTATttTGTTTTTGAAATATGATTAtAGtCGTTAAAACGTAGATtTGTGGTtTGA 178
257 AGTTGTTAAAATGTAGATT 178
257 TAGTCGTTAAAACGTAG 178

177 CTGGCTACCCAGGACATGTTAAATGGGAAAACAACTAGCAGAAGGGCACCTACGTAGTGATACCATTTATGTTATTTTTA 98
177 tTGGtTAtttAGGAtATGTTAAATGGGAAAAtAAtTAGtAGAAGGGtAttTACGTAGTGATAttATTTATGTTATTTTTA 98

---

97 AAATTAAATGTACATTTATATAGGTGTACGTGTATGAACGTGCAGTTTAAAGGCCTAAACAGAAAAATACATCAGAAGCT 18
97 **AAATC** 18
97 AAATTAAATGTAtATTTATATAGGTGTACGTGTATGAACGTGtAGTTTAAAGGttTAAAtAGAAAAATAtATtAGAAGtT 18
97 <u>TGTATGAACGTGTAGTT</u> 18
97 <u>GTGTATGAATGTGTAGT</u>

---

17 CTTAACTGTGGGATGGG -62
17 **CCTTTTTCACAACTAAA** -62
17 tTTAAtTGTGGGATGGG -62

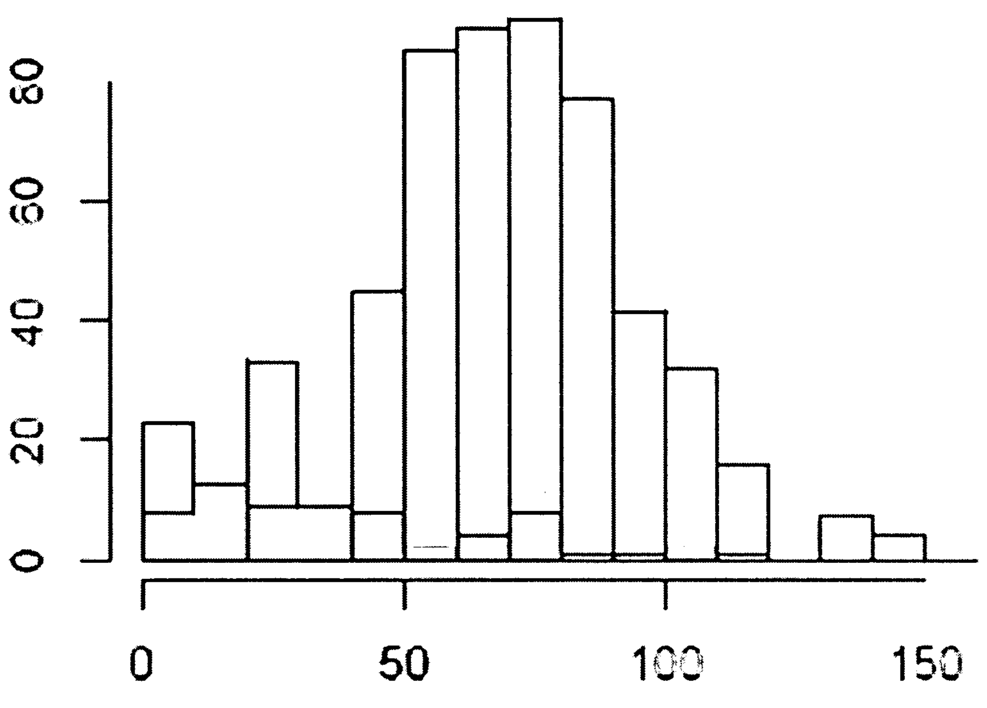


Figure 89

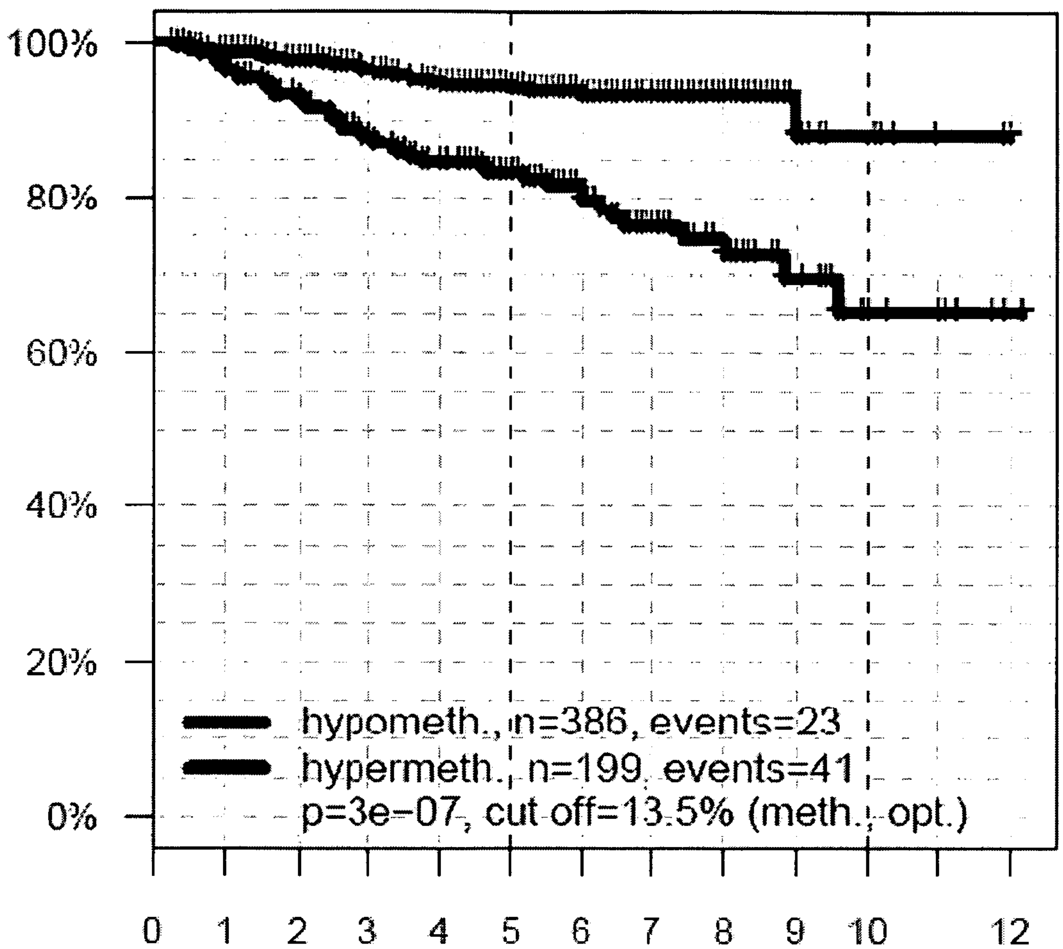
100%
80%
60%
40%
20%
0%
hypometh., n=386, events=23
hypermeth., n=199, events=41
p=3e-07, cut off=13.5% (meth., opt.)
0
1
2
3
4
5
6
7
8
9
10
12

Figure 90 A

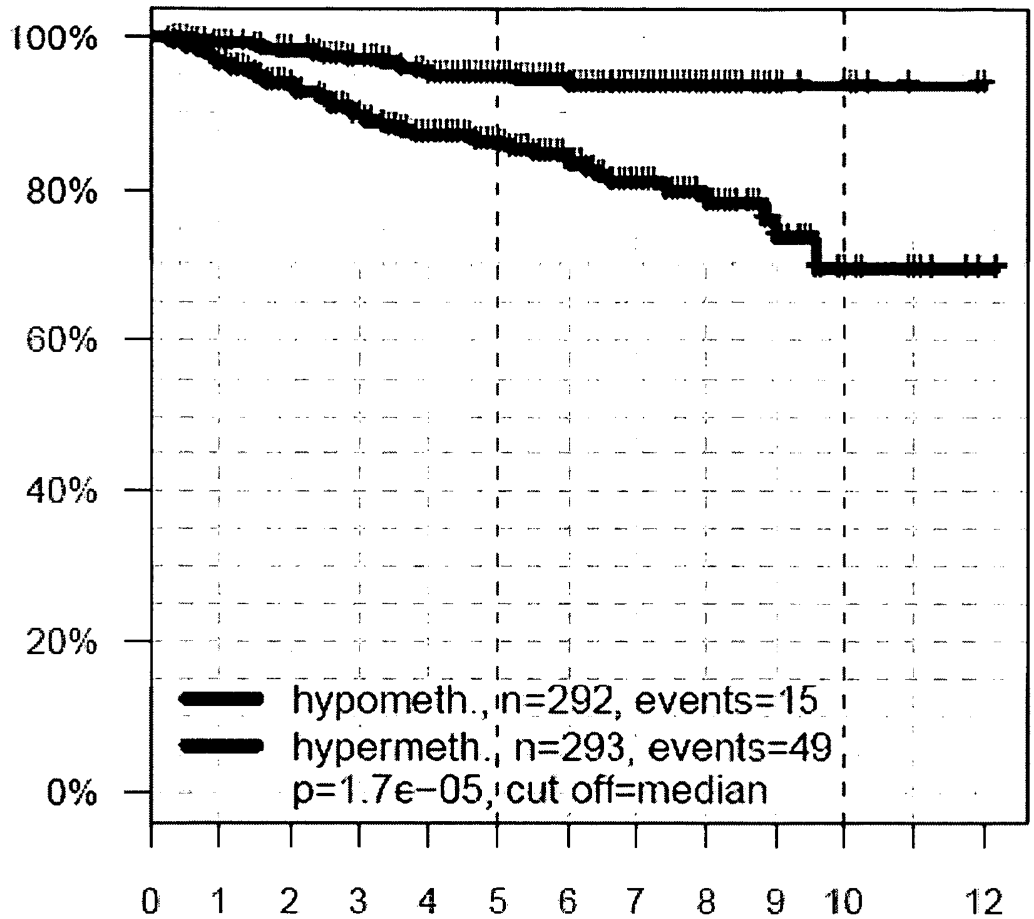
100%
80%
60%
40%
20%
0%
0
1
2
3
4
5
6
7
8
9
10
12
hypometh., n=292, events=15
hypermeth., n=293, events=49
p=1.7e−05, cut off=median

Figure 90 B

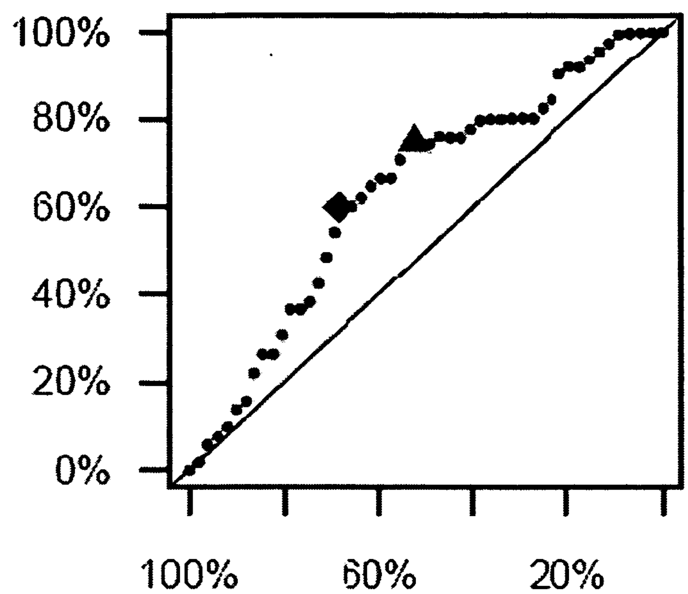


Figure 90 C

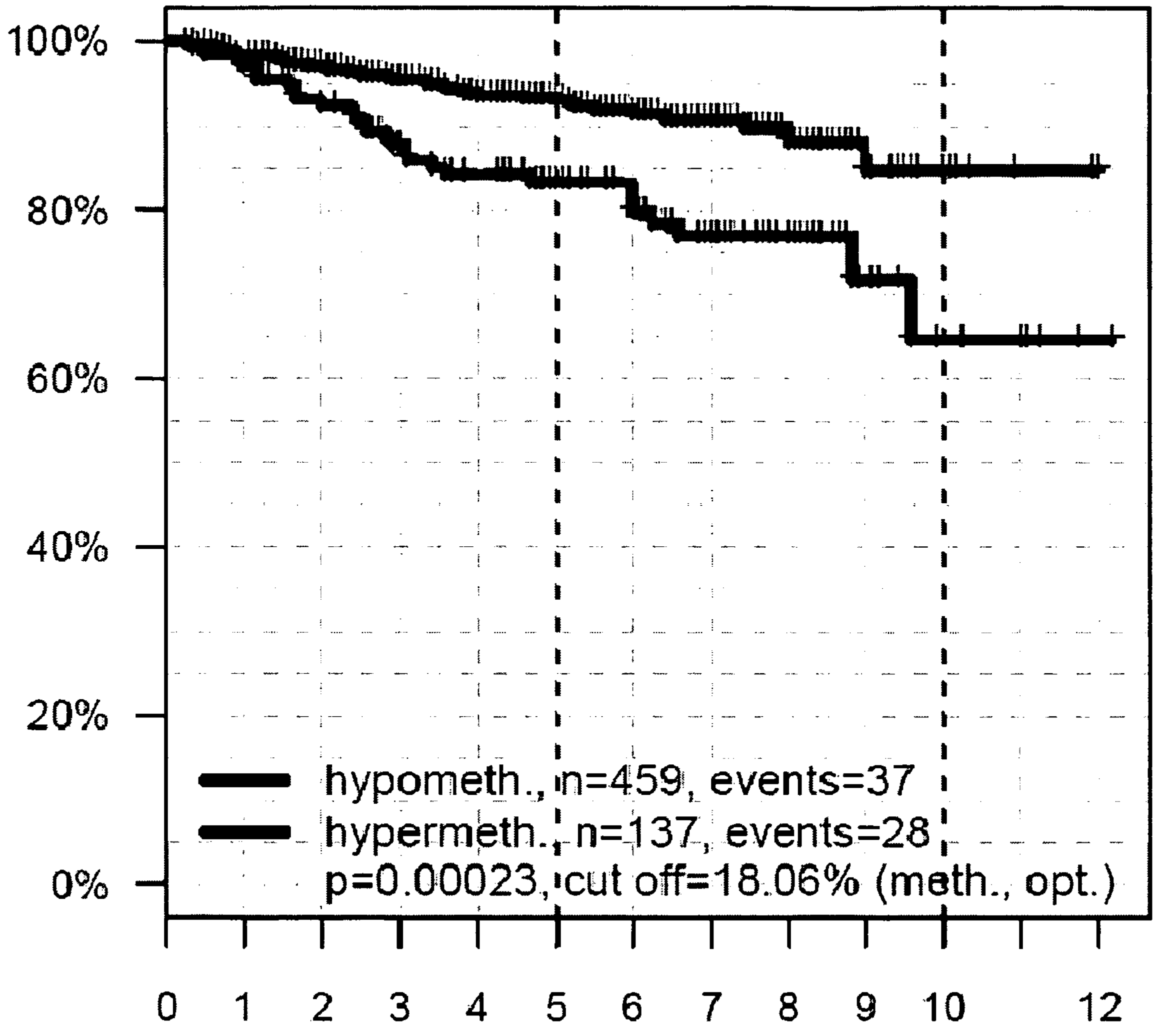
100%
80%
60%
40%
20%
0%
hypometh., n=459, events=37
hypermeth., n=137, events=28
p=0.00023, cut off=18.06% (meth., opt.)
0 1 2 3 4 5 6 7 8 9 10 12

Figure 91 A

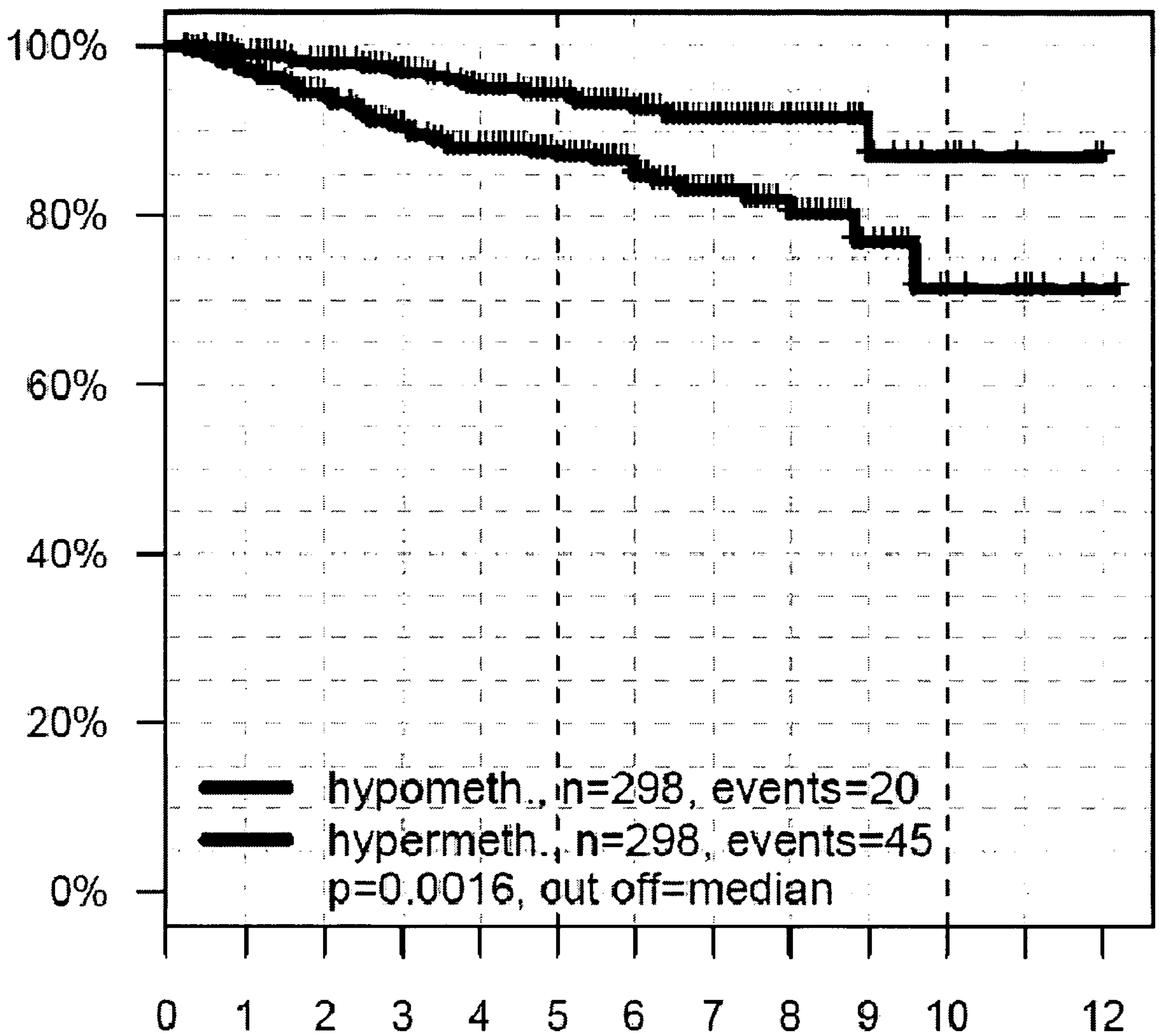
100%
80%
60%
40%
20%
0%
hypometh., n=298, events=20
hypermeth., n=298, events=45
p=0.0016, out off=median
0 1 2 3 4 5 6 7 8 9 10 12

Figure 91 B

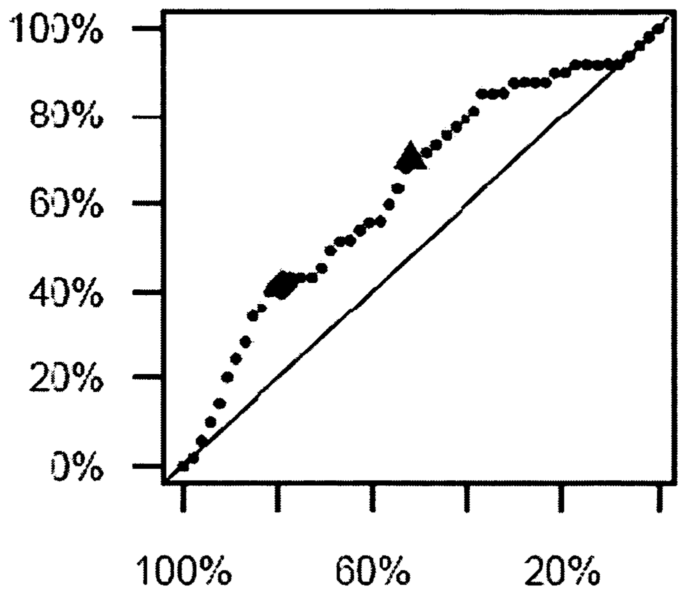


Figure 91 C

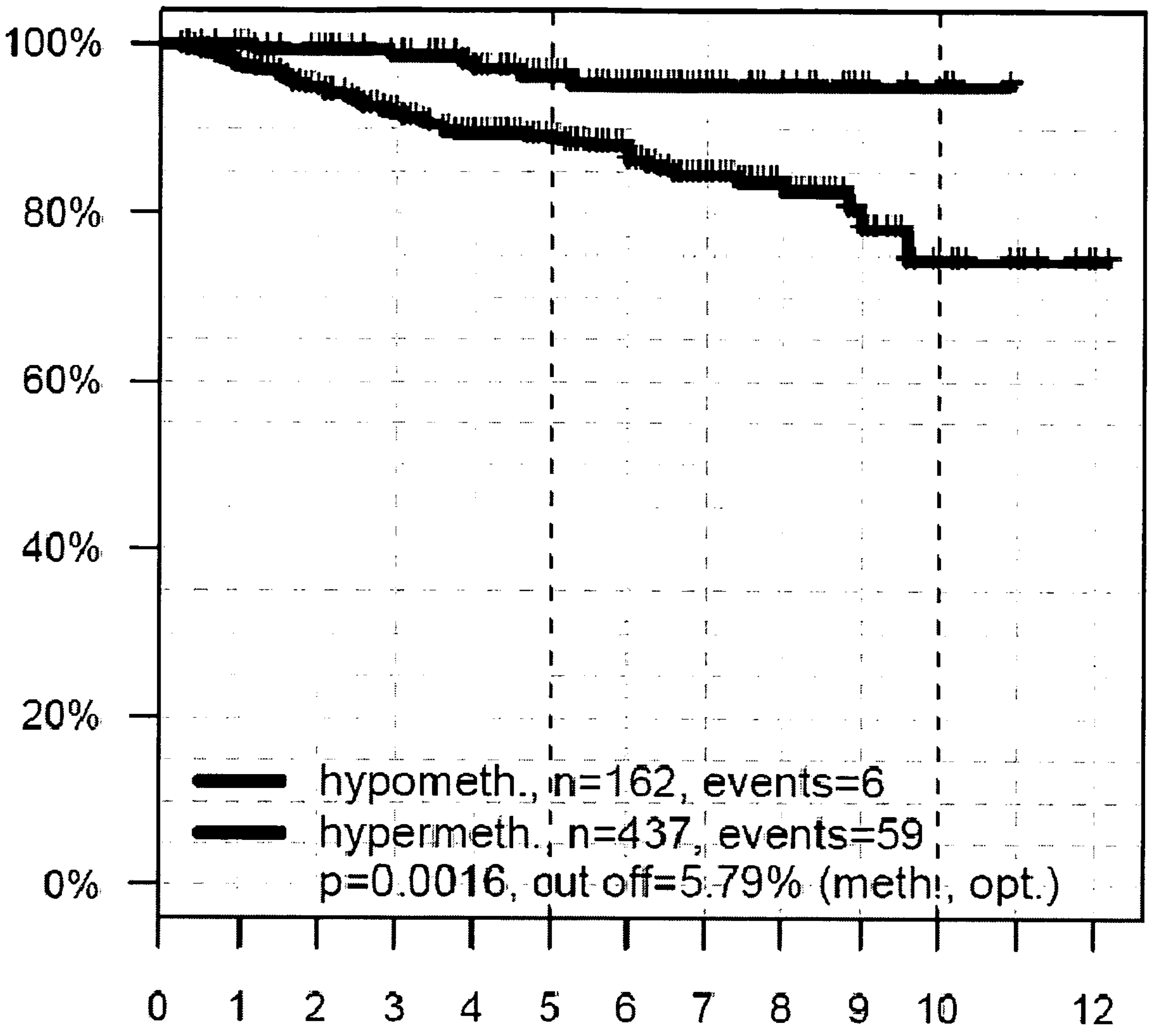
100%
80%
60%
40%
20%
0%
hypometh., n=162, events=6
hypermeth., n=437, events=59
p=0.0016, cut off=5.79% (meth., opt.)
0 1 2 3 4 5 6 7 8 9 10 12

Figure 92 A

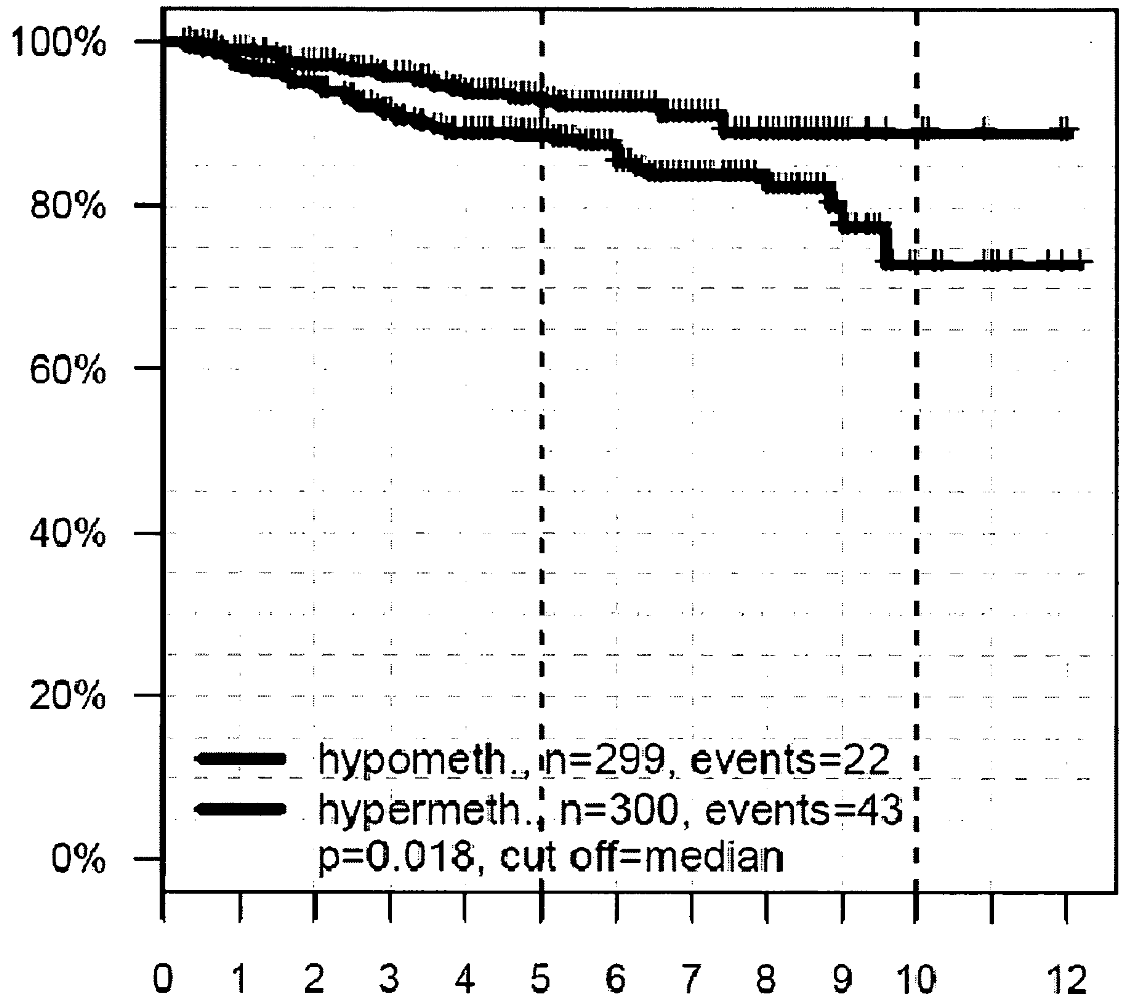
100%
80%
60%
40%
20%
0%
0
1
2
3
4
5
6
7
8
9
10
12
hypometh., n=299, events=22
hypermeth., n=300, events=43
p=0.018, cut off=median

Figure 92 B

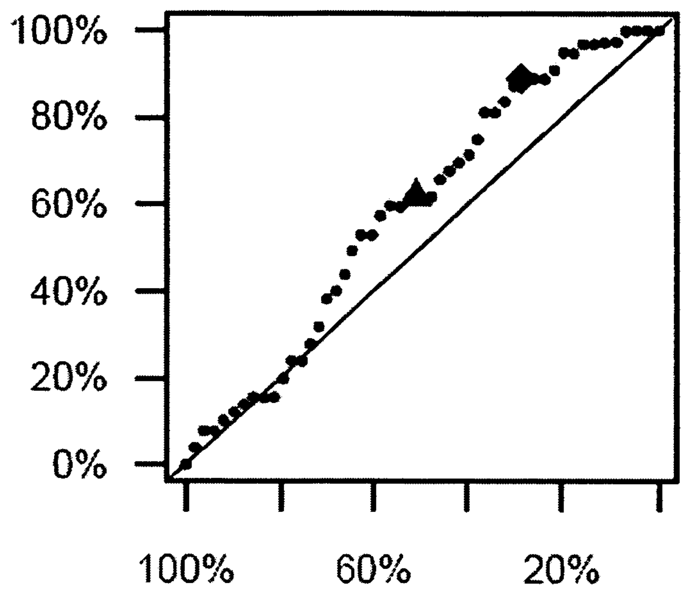


Figure 92 C

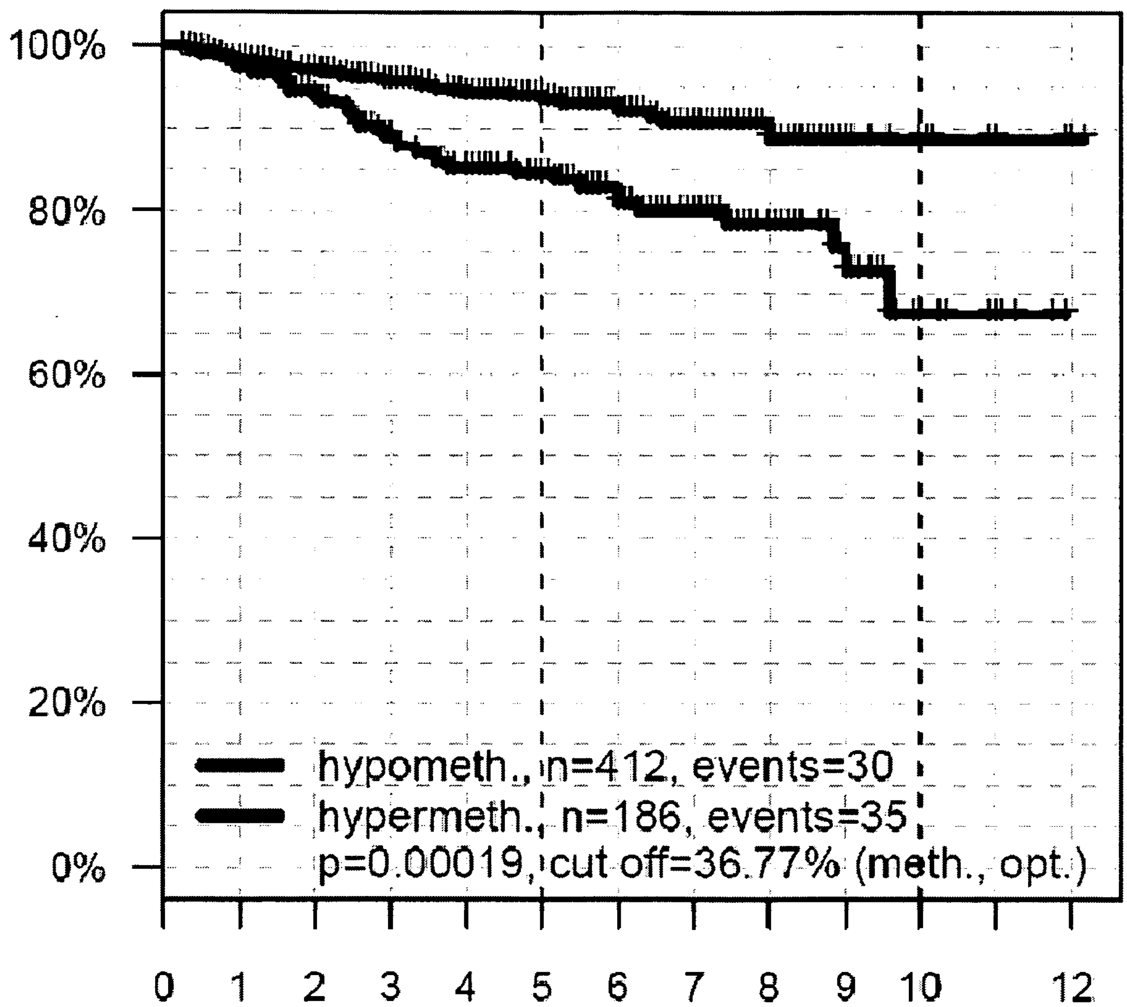
100%
80%
60%
40%
20%
0%
hypometh., n=412, events=30
hypermeth., n=186, events=35
p=0.00019, cut off=36.77% (meth., opt.)
0 1 2 3 4 5 6 7 8 9 10 12

Figure 93 A

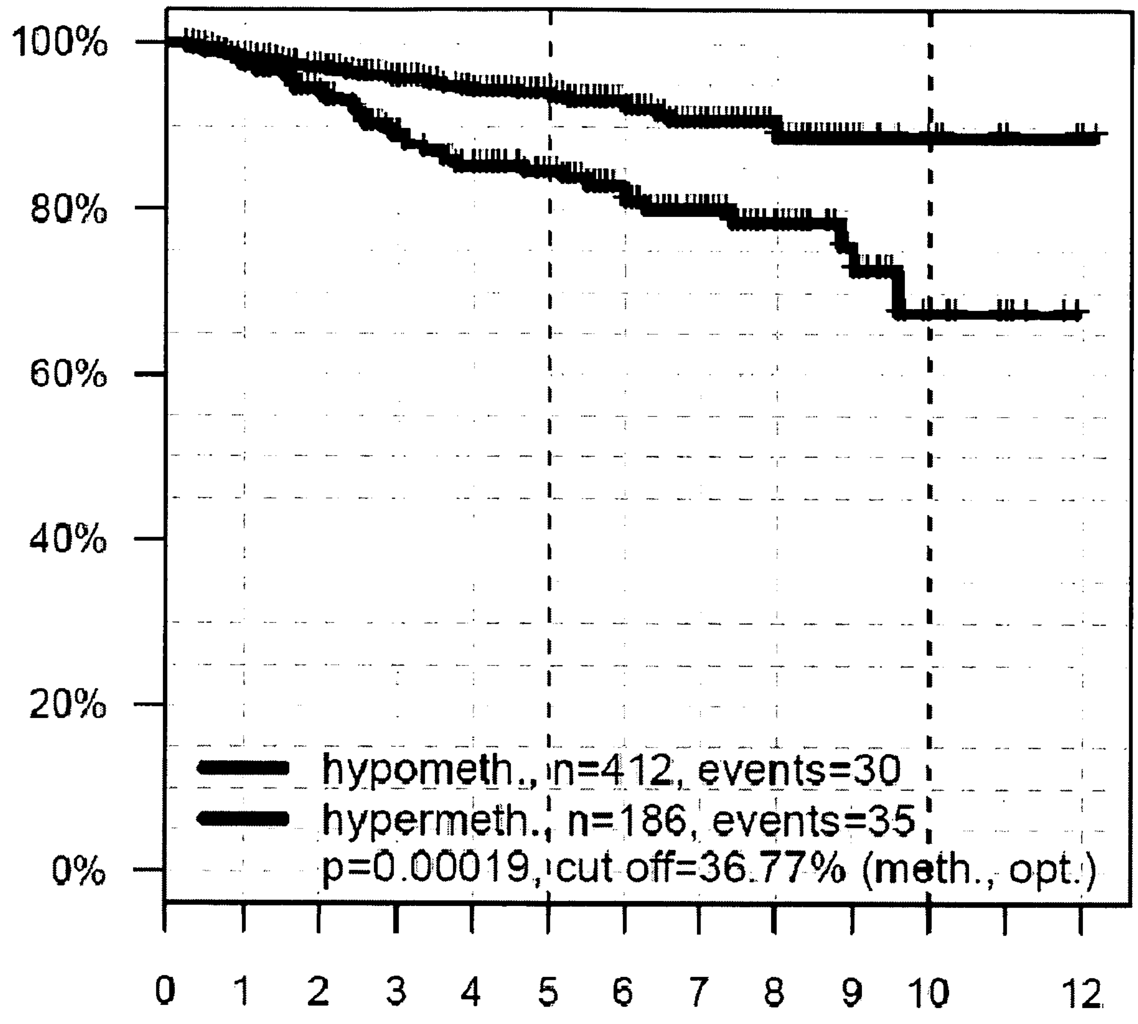
100%
80%
60%
40%
20%
0%
hypometh., n=412, events=30
hypermeth., n=186, events=35
p=0.00019, cut off=36.77% (meth., opt.)
0
1
2
3
4
5
6
7
8
9
10
12

Figure 93 B

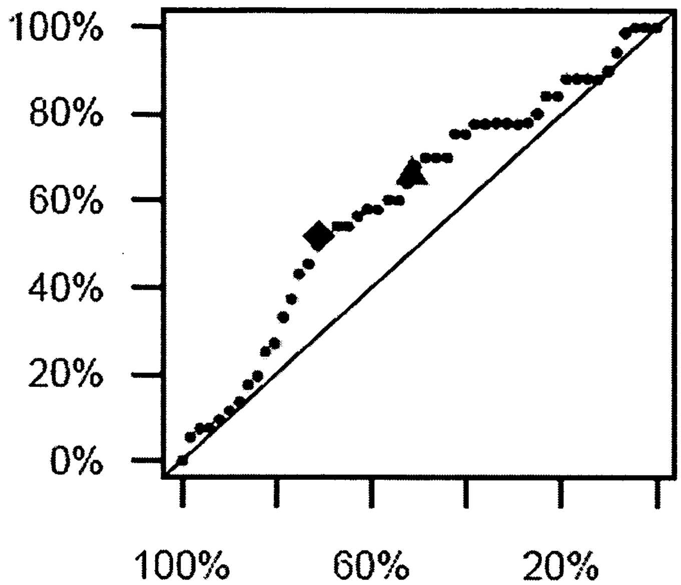


Figure 93 C

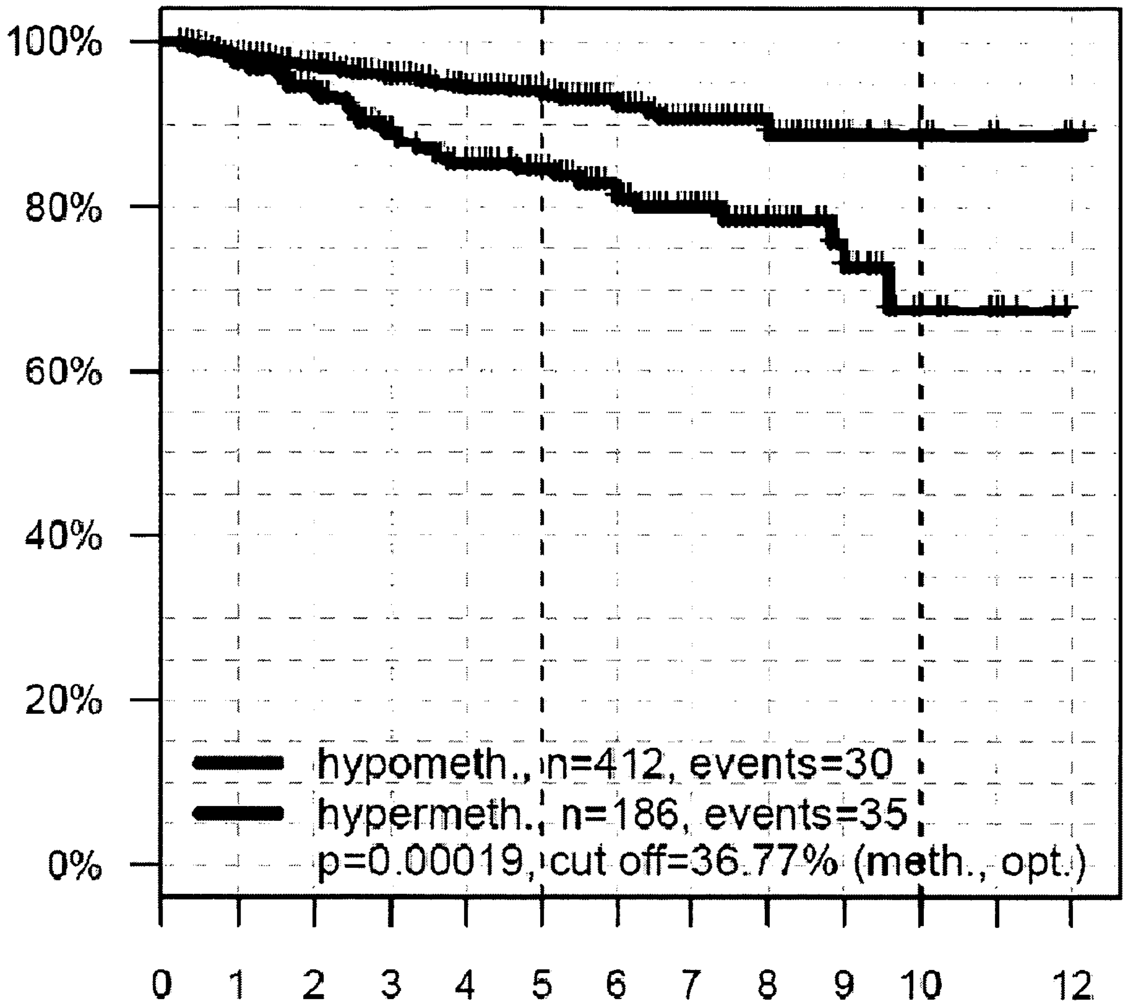
100%
80%
60%
40%
20%
0%
hypometh., n=412, events=30
hypermeth., n=186, events=35
p=0.00019, cut off=36.77% (meth., opt.)
0
1
2
3
4
5
6
7
8
9
10
12

Figure 94 A

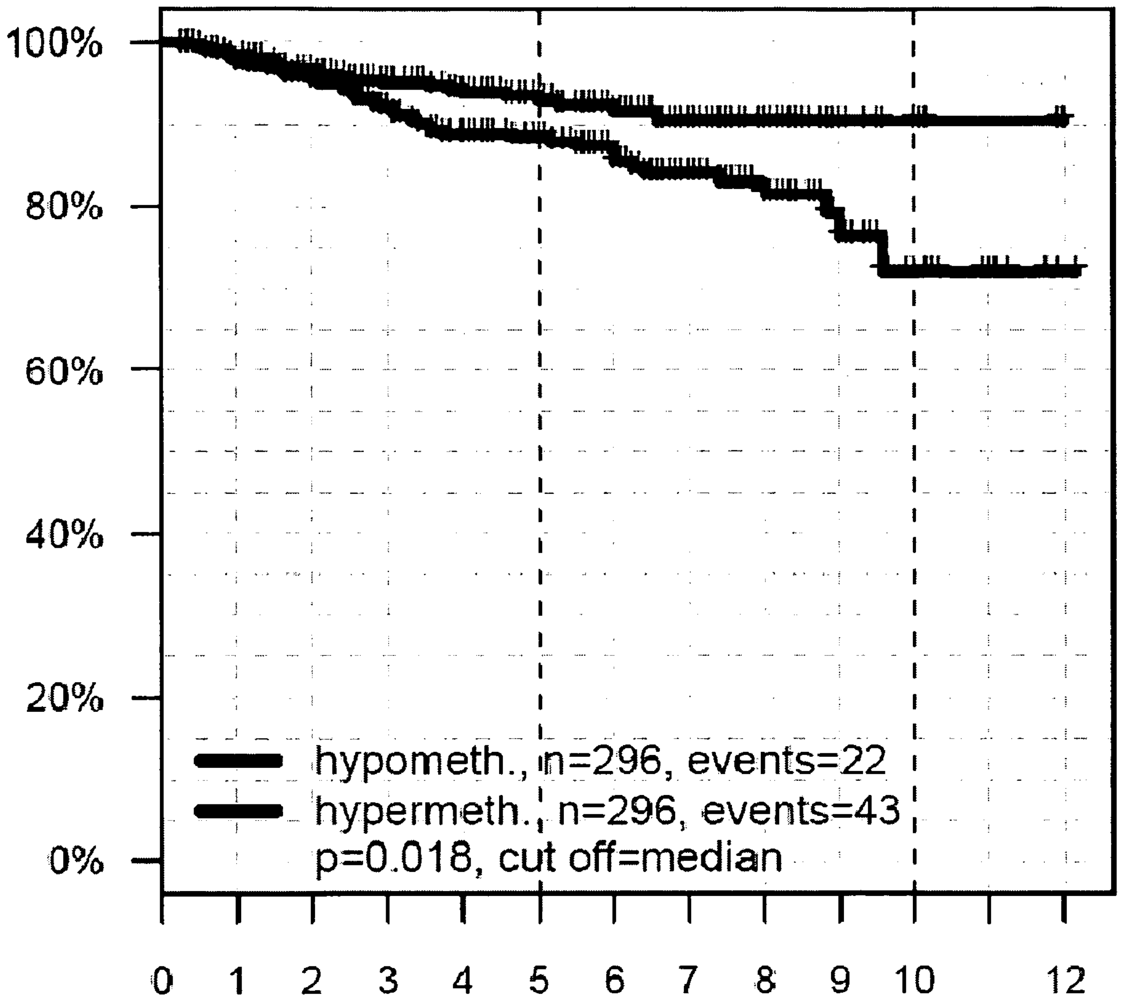
100%
80%
60%
40%
20%
0%
0 1 2 3 4 5 6 7 8 9 10 12
hypometh., n=296, events=22
hypermeth., n=296, events=43
p=0.018, cut off=median

Figure 94 B

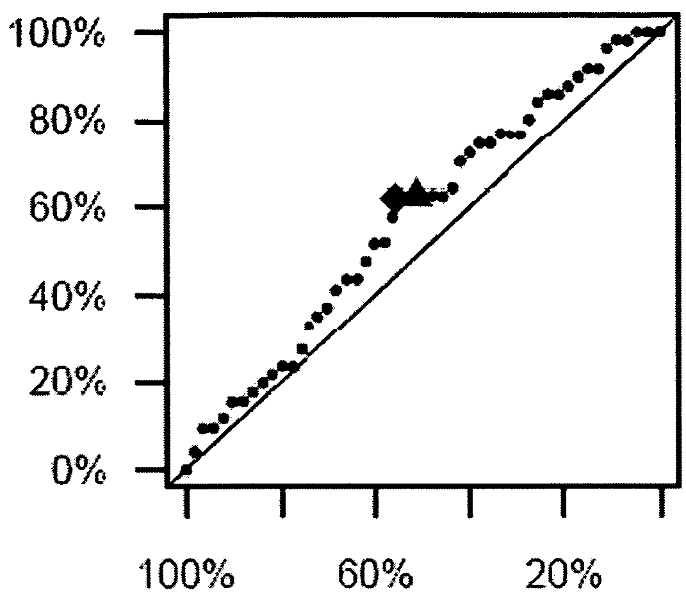


Figure 94 C

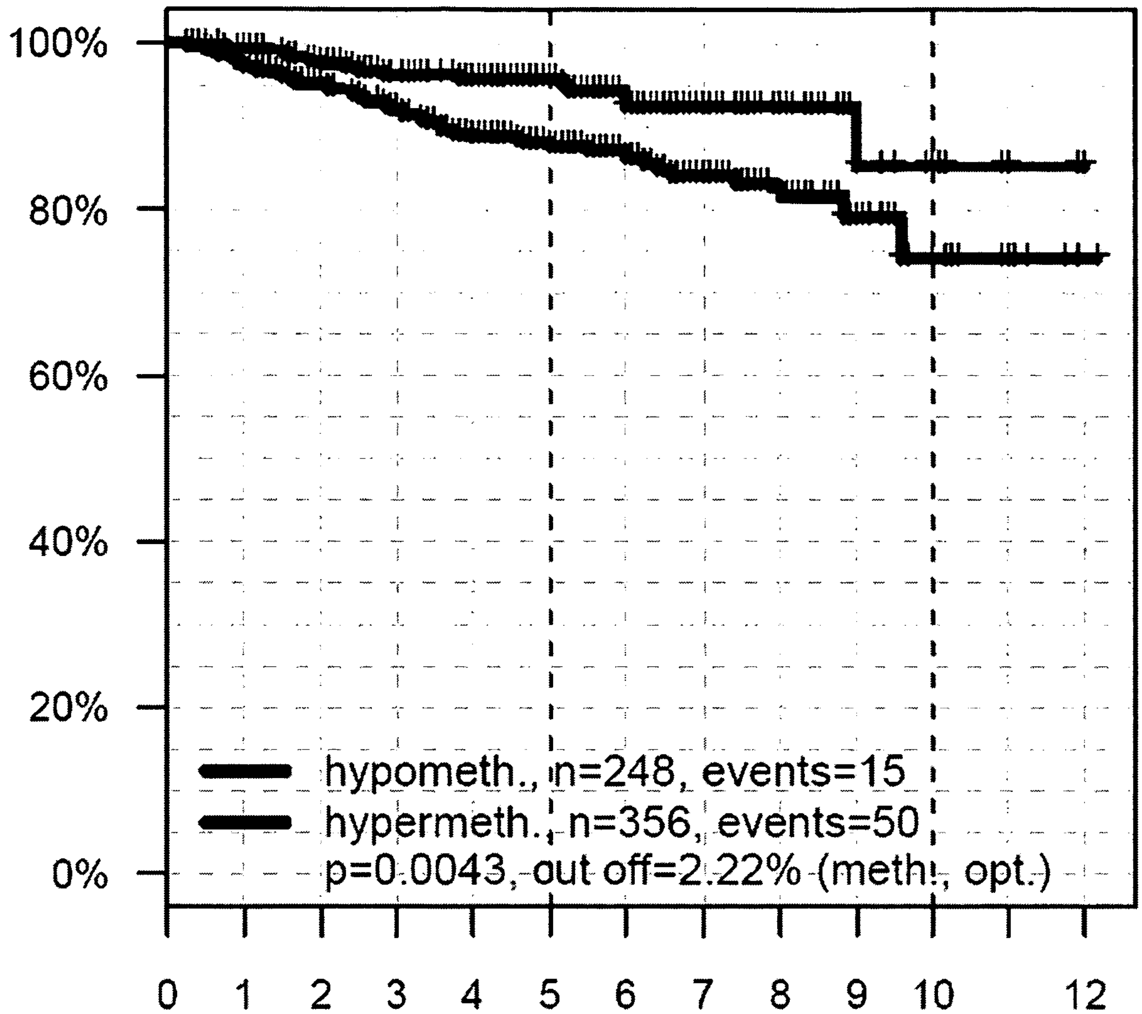
100%
80%
60%
40%
20%
0%
hypometh., n=248, events=15
hypermeth., n=356, events=50
p=0.0043, cut off=2.22% (meth., opt.)
0
1
2
3
4
5
6
7
8
9
10
12

Figure 95 A

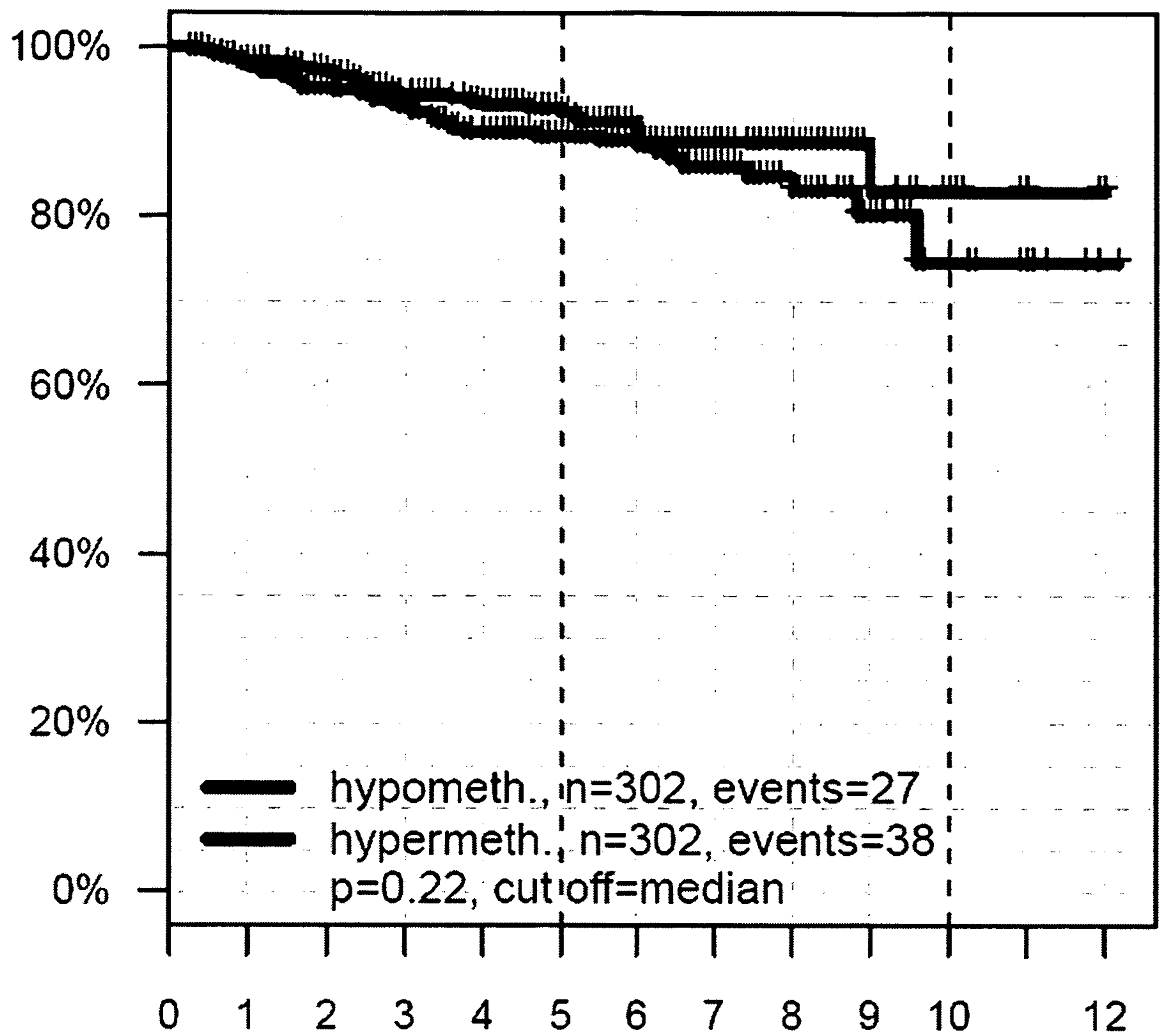
100%
80%
60%
40%
20%
0%
hypometh., n=302, events=27
hypermeth., n=302, events=38
p=0.22, cut off=median
0
1
2
3
4
5
6
7
8
9
10
12

Figure 95 B

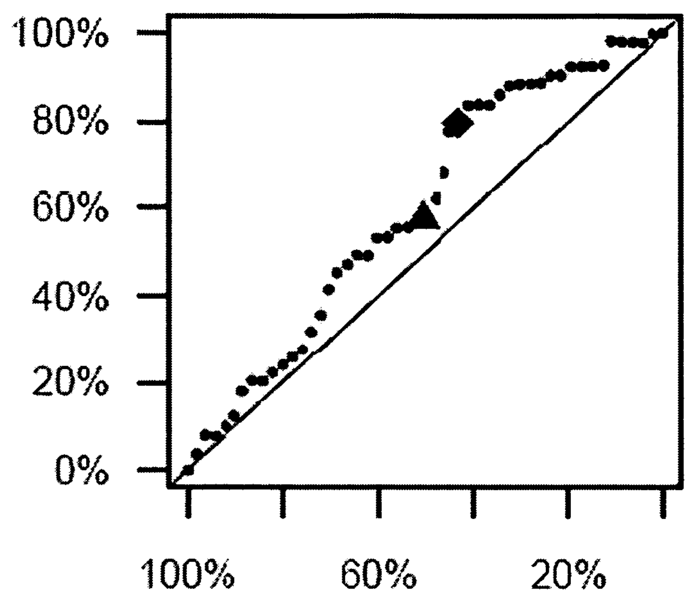


Figure 95 C

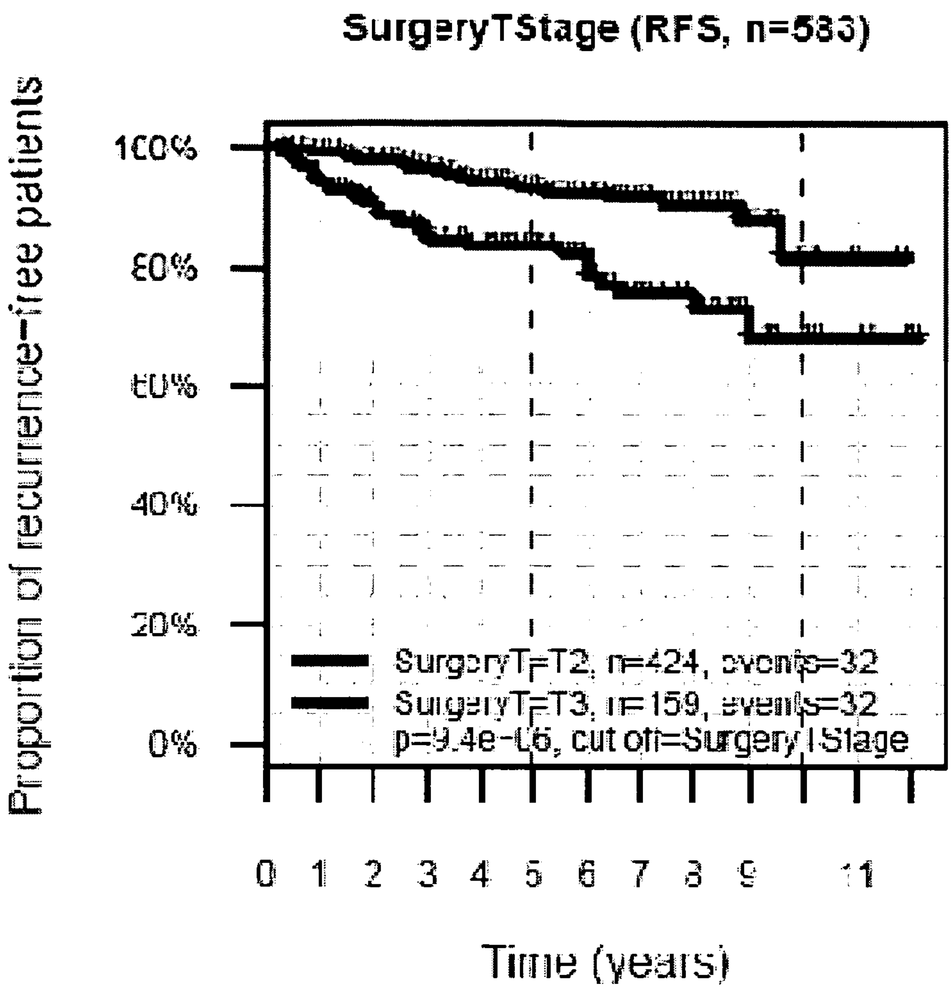

SurgeryTStage (RFS, n=583)
Proportion of recurrence-free patients
100%
80%
60%
40%
20%
0%
SurgeryT=T2, n=424, events=32
SurgeryT=T3, n=159, events=32
p=9.4e-06, cut off=SurgeryTStage
0 1 2 3 4 5 6 7 8 9 11
Time (years)


Figure 96/1

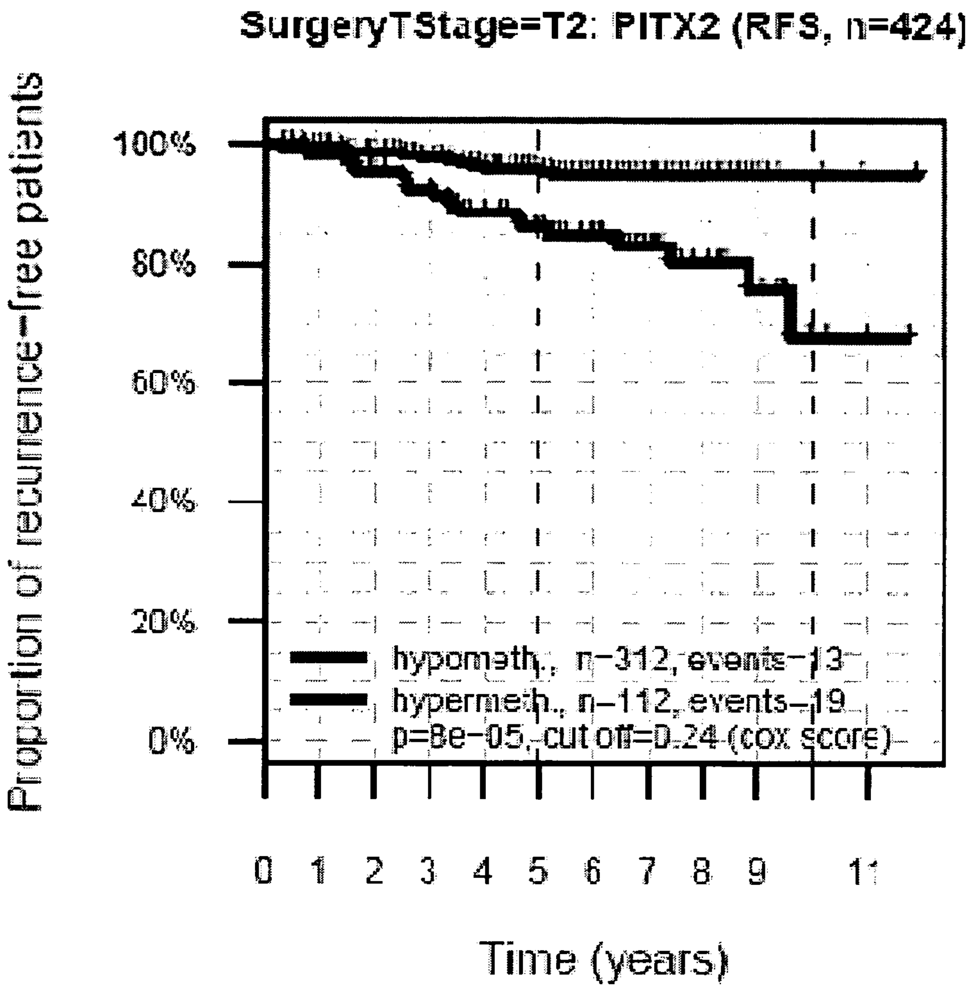
SurgeryTStage=T2: PITX2 (RFS, n=424)
Proportion of recurrence-free patients
100%
80%
60%
40%
20%
0%
hypometh., n=312, events=13
hypermeth., n=112, events=19
p=8e-05, cut off=0.24 (cox score)
0 1 2 3 4 5 6 7 8 9 11
Time (years)

Figure 96/2

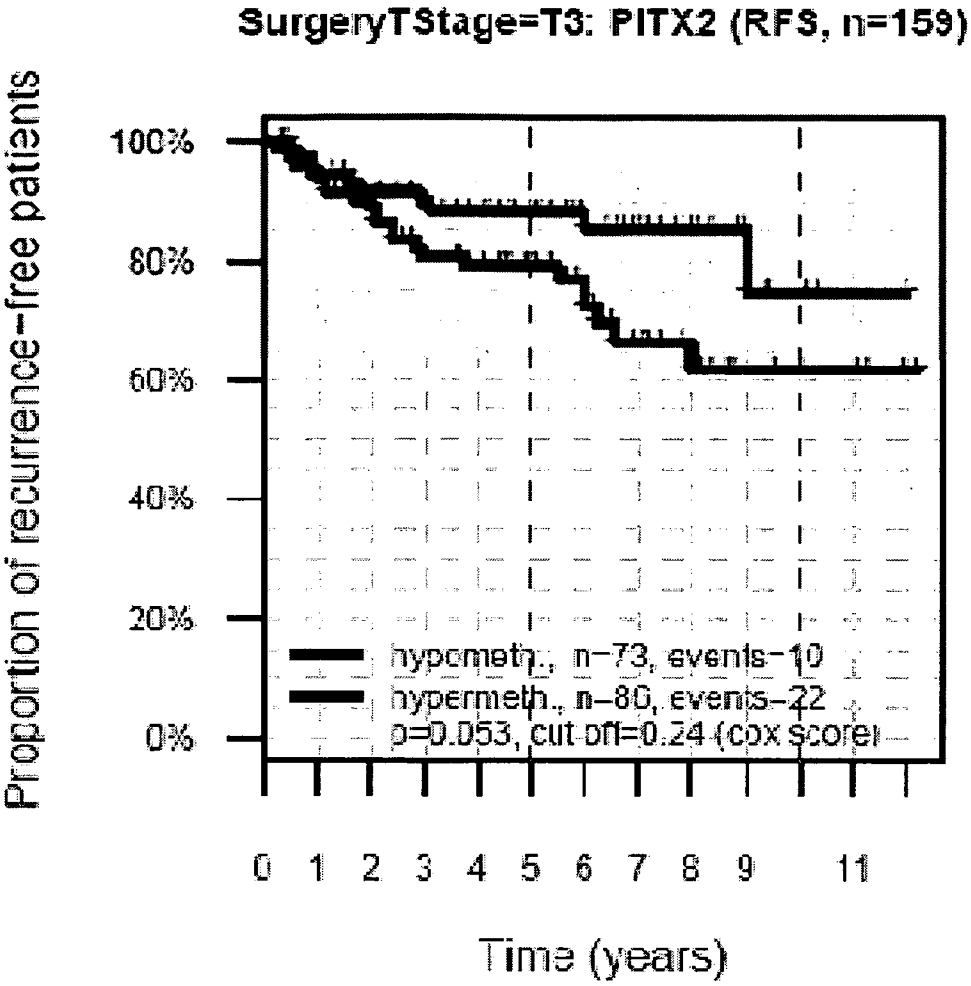
SurgeryTStage=T3: PITX2 (RFS, n=159)
Proportion of recurrence-free patients
100%
80%
60%
40%
20%
0%
hypometh., n=73, events=10
hypermeth., n=80, events=22
p=0.053, cut off=0.24 (cox score)
0 1 2 3 4 5 6 7 8 9 11
Time (years)

Figure 96/3

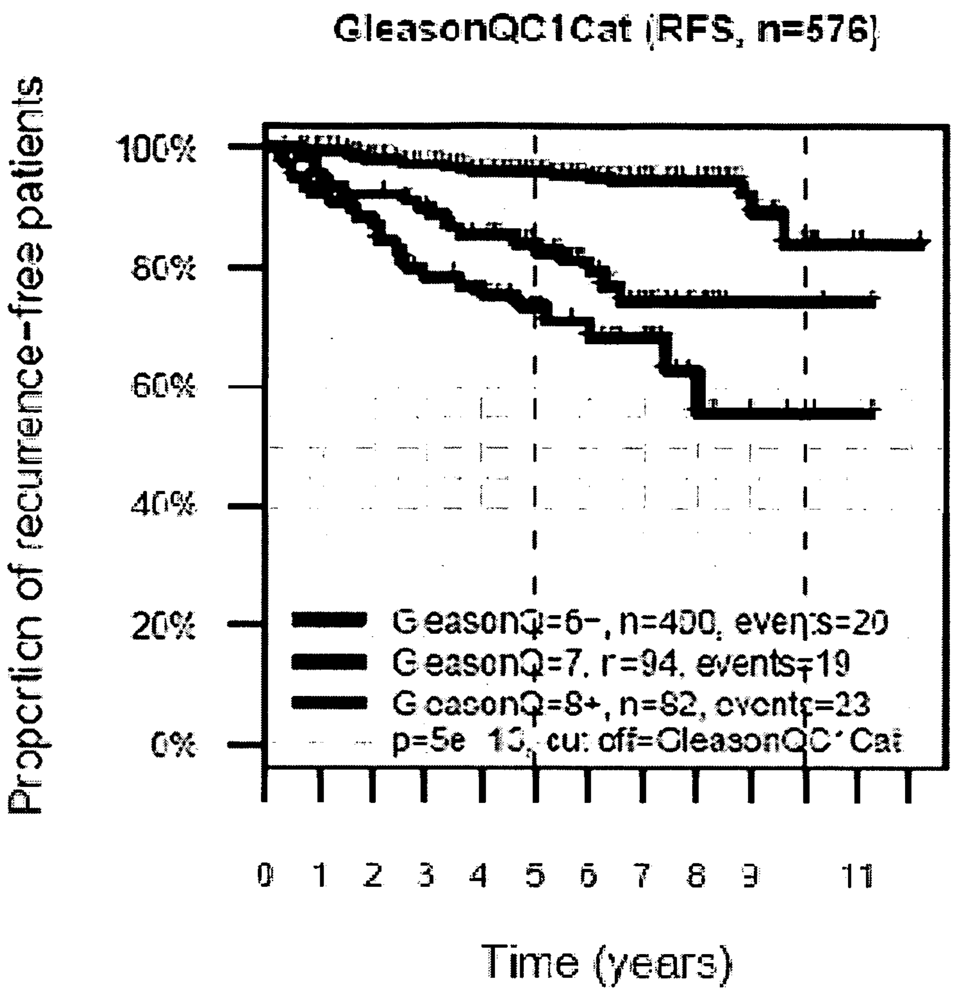

GleasonQC1Cat (RFS, n=576)
Proportion of recurrence-free patients
100%
80%
60%
40%
20%
0%
0 1 2 3 4 5 6 7 8 9 11
Time (years)
G easonQ=6-, n=400, events=20
G easonQ=7, r=94, events=19
G easonQ=8+, n=82, events=23
p=5e 13, cut off=GleasonQC1Cat


Figure 97/1

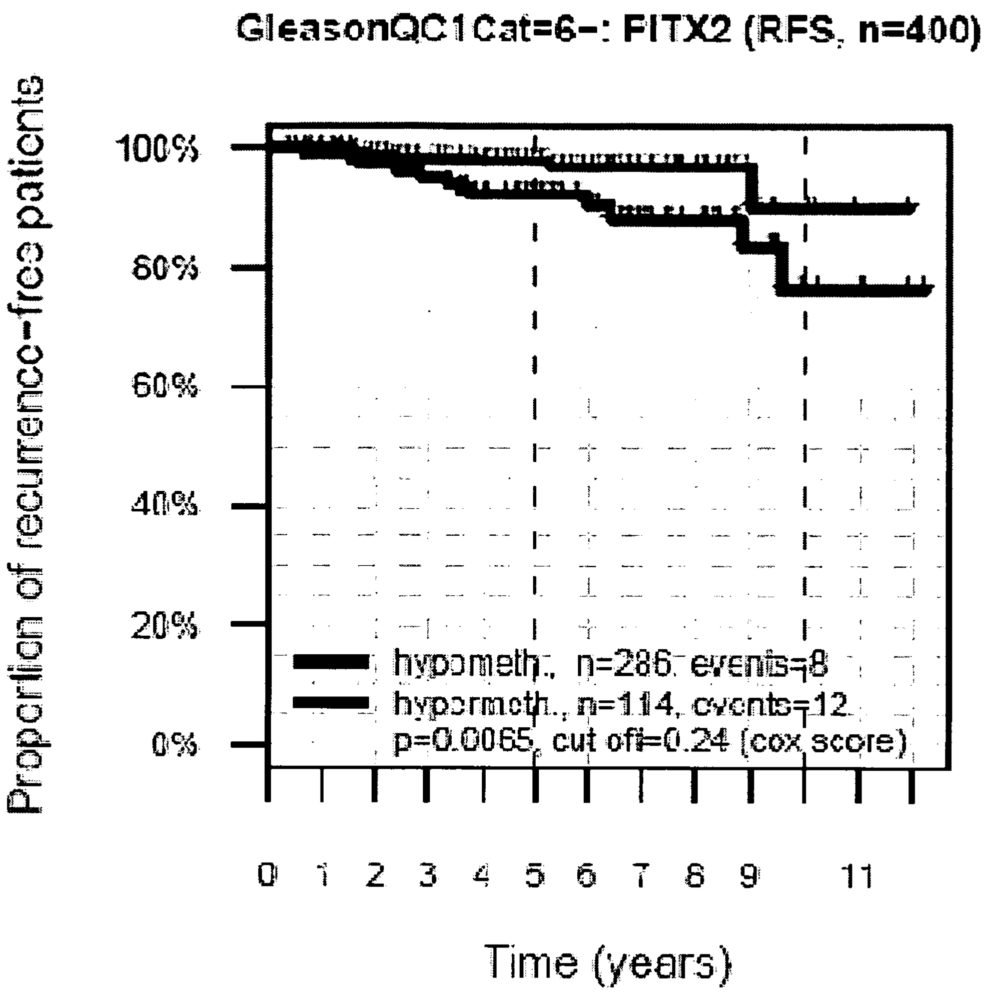
GleasonQC1Cat=6-: PITX2 (RFS, n=400)
Proportion of recurrence-free patients
100%
80%
60%
40%
20%
0%
hypometh., n=286, events=8
hypermeth., n=114, events=12
p=0.0065, cut off=0.24 (cox score)
0 1 2 3 4 5 6 7 8 9 11
Time (years)

Figure 97/2

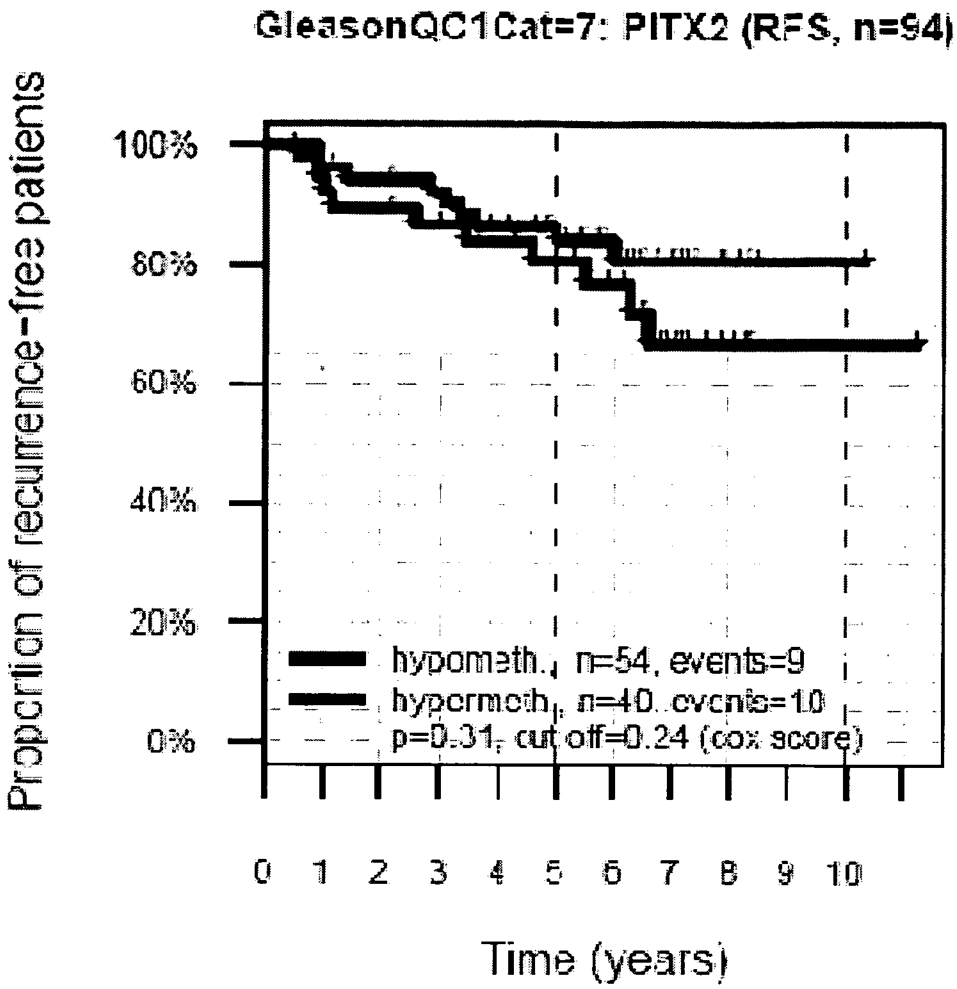
GleasonQC1Cat=7: PITX2 (RFS, n=94)
Proportion of recurrence-free patients
100%
80%
60%
40%
20%
0%
hypometh., n=54, events=9
hypermeth., n=40, events=10
p=0.31, cut off=0.24 (cox score)
0 1 2 3 4 5 6 7 8 9 10
Time (years)

Figure 97/3

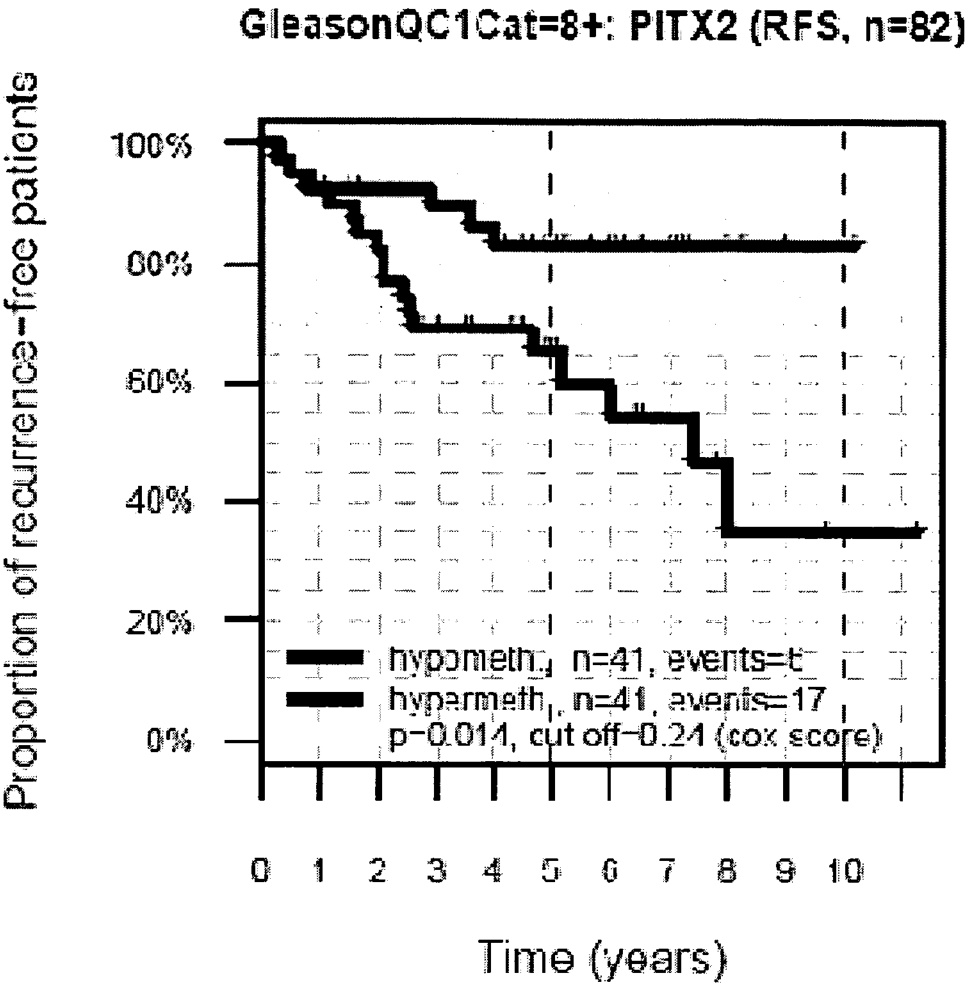
GleasonQC1Cat=8+: PITX2 (RFS, n=82)
Proportion of recurrence-free patients
100%
80%
60%
40%
20%
0%
hypometh., n=41, events=6
hypermeth., n=41, events=17
p=0.014, cut off=0.24 (cox score)
0 1 2 3 4 5 6 7 8 9 10
Time (years)

**Figure 97/4**

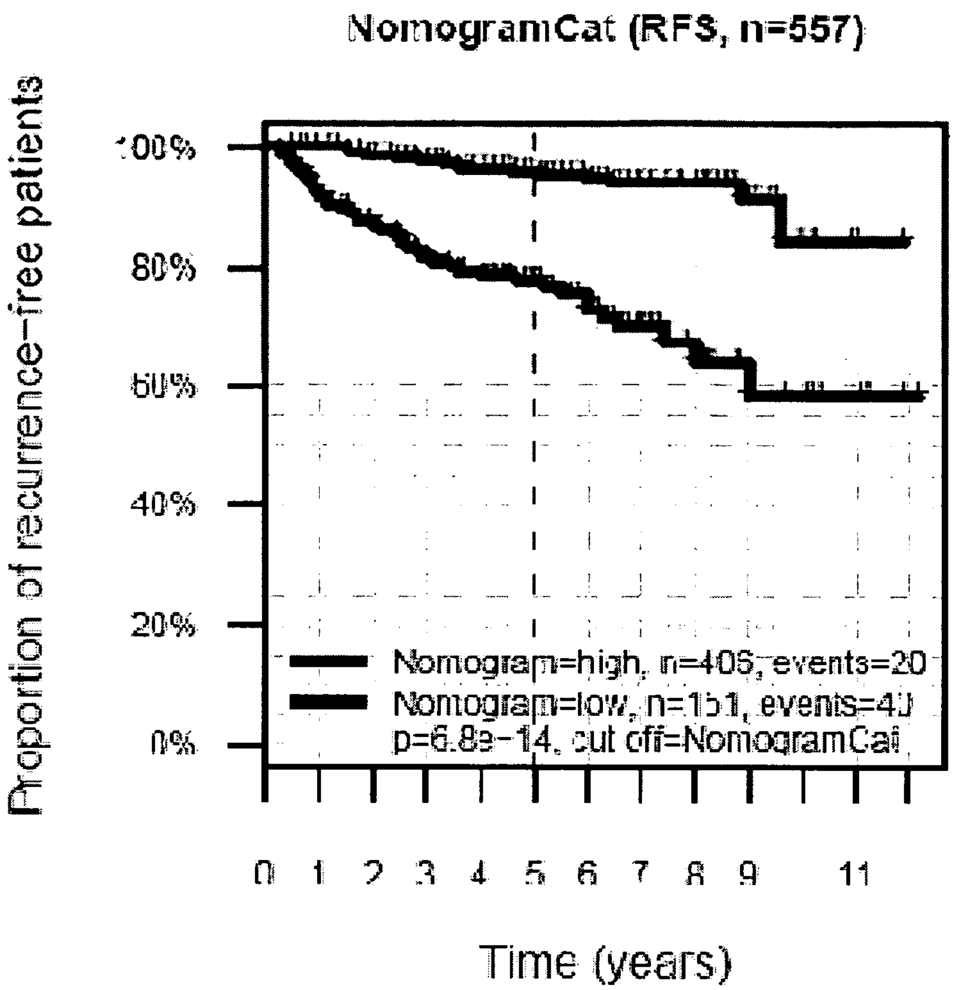
NomogramCat (RFS, n=557)
Proportion of recurrence-free patients
100%
80%
60%
40%
20%
0%
Nomogram=high, n=406, events=20
Nomogram=low, n=151, events=40
p=6.8e-14, cut off=NomogramCat
0
1
2
3
4
5
6
7
8
9
11
Time (years)

Figure 98/1

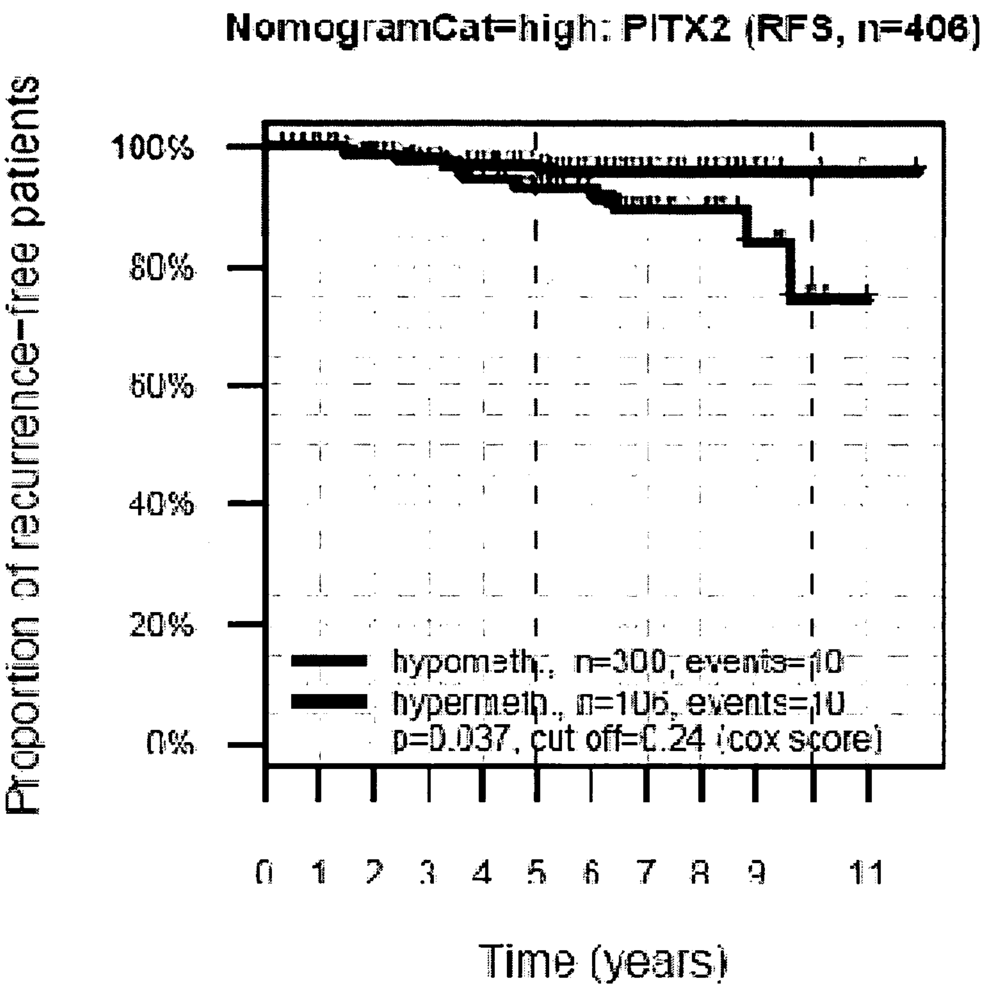
NomogramCat=high: PITX2 (RFS, n=406)
Proportion of recurrence-free patients
100%
80%
60%
40%
20%
0%
hypometh., n=300, events=10
hypermeth., n=106, events=10
p=0.037, cut off=0.24 (cox score)
0 1 2 3 4 5 6 7 8 9 11
Time (years)

Figure 98/2

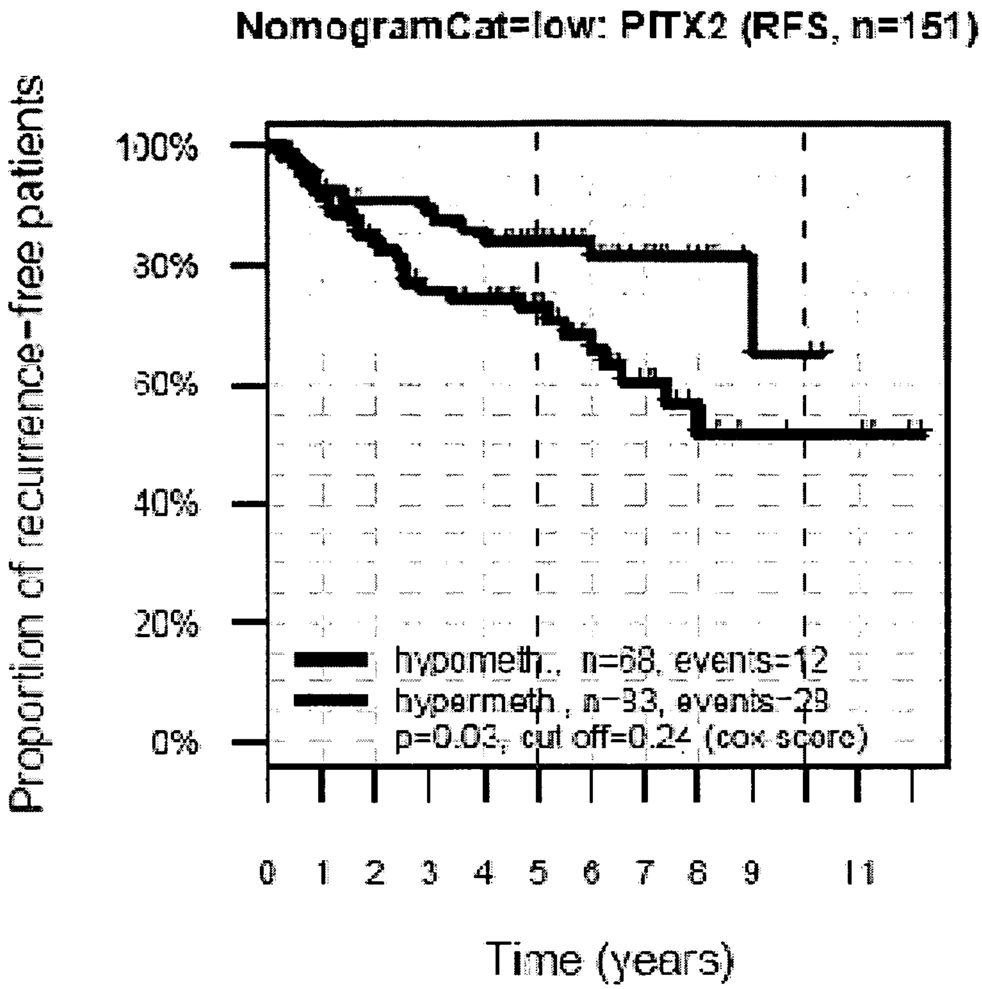
NomogramCat=low: PITX2 (RFS, n=151)
Proportion of recurrence-free patients
100%
80%
60%
40%
20%
0%
hypometh., n=68, events=12
hypermeth., n=83, events=28
p=0.03, cut off=0.24 (cox score)
0 1 2 3 4 5 6 7 8 9 11
Time (years)

Figure 98/3

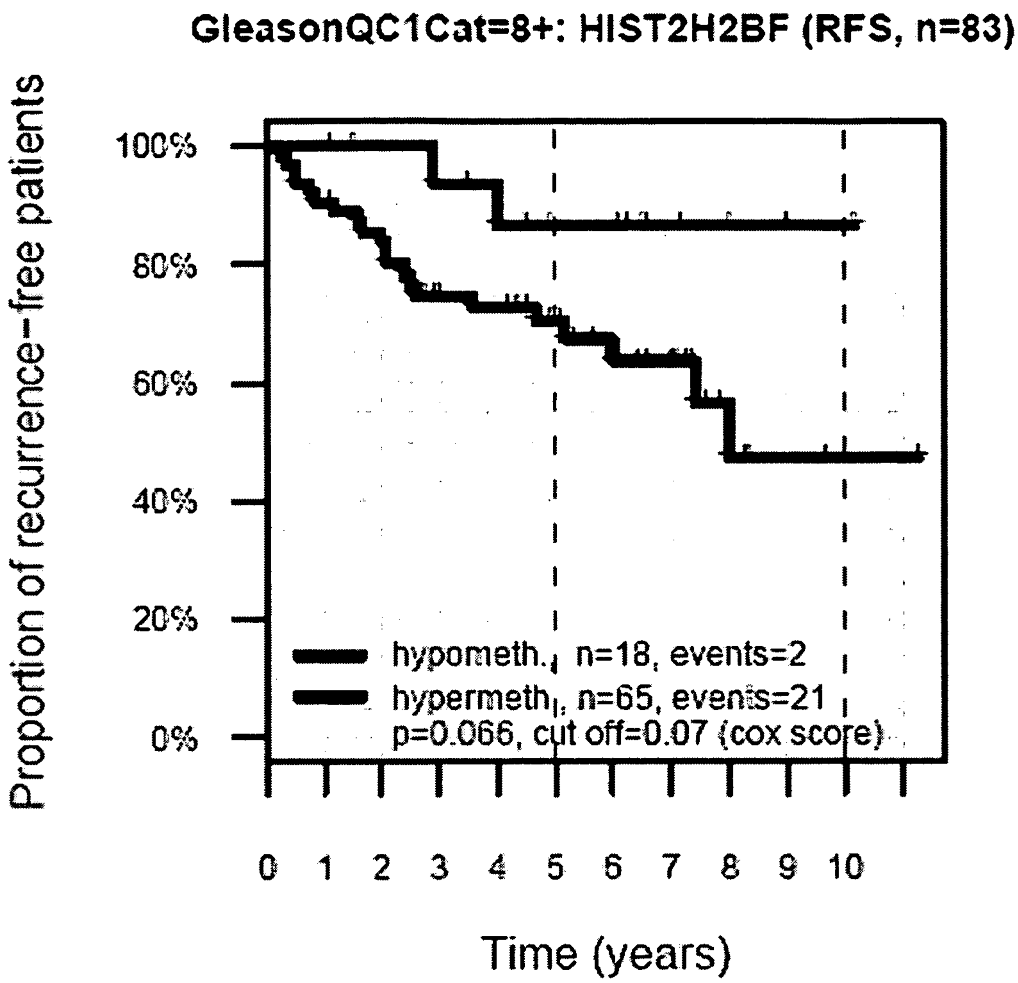
GleasonQC1Cat=8+: HIST2H2BF (RFS, n=83)
Proportion of recurrence-free patients
100%
80%
60%
40%
20%
0%
hypometh., n=18, events=2
hypermeth., n=65, events=21
p=0.066, cut off=0.07 (cox score)
0
1
2
3
4
5
6
7
8
9
10
Time (years)

Figure 99

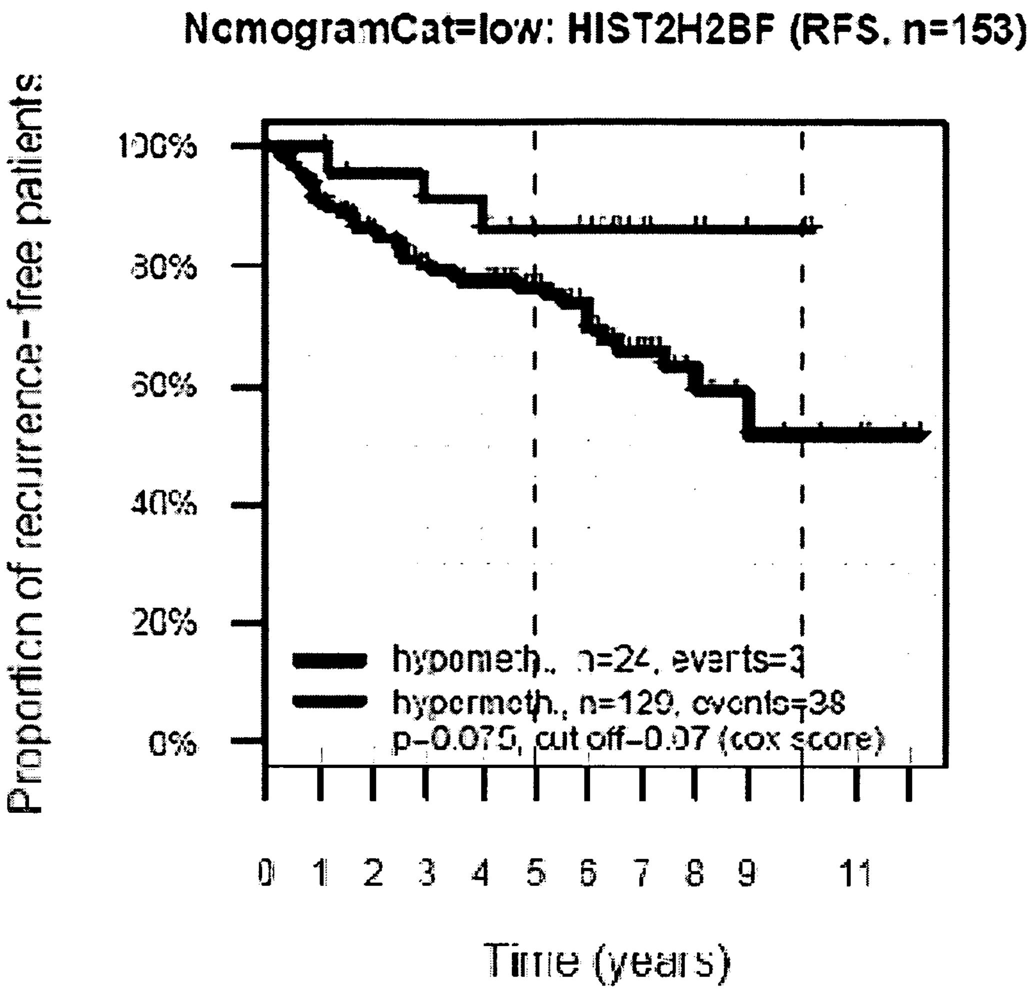
NomogramCat=low: HIST2H2BF (RFS. n=153)
Proportion of recurrence-free patients
100%
80%
60%
40%
20%
0%
hypometh., n=24, events=3
hypermeth., n=129, events=38
p=0.075, cut off=0.07 (cox score)
0
1
2
3
4
5
6
7
8
9
11
Time (years)

Figure 100

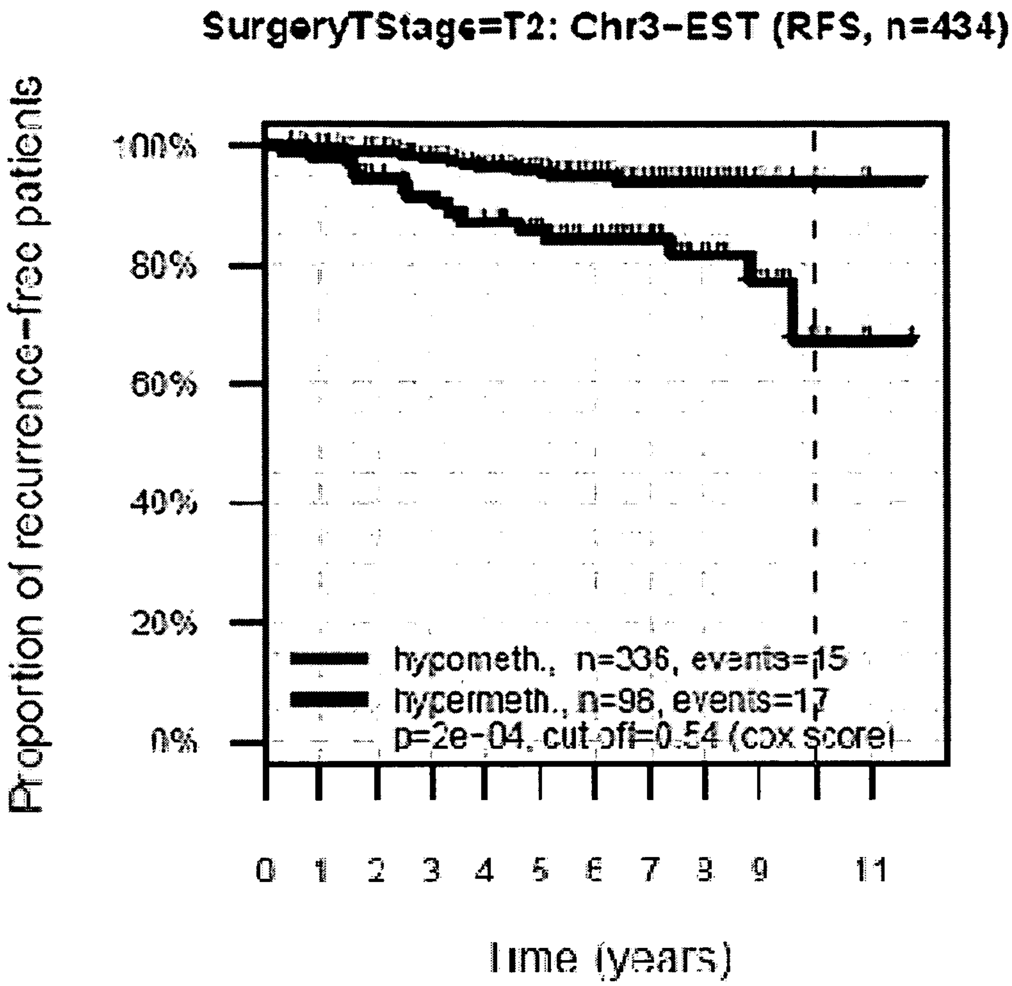
SurgeryTStage=T2: Chr3-EST (RFS, n=434)
Proportion of recurrence-free patients
100%
80%
60%
40%
20%
0%
hypometh., n=336, events=15
hypermeth., n=98, events=17
p=2e-04, cut off=0.54 (cox score)
0
1
2
3
4
5
6
7
8
9
11
Time (years)

Figure 101

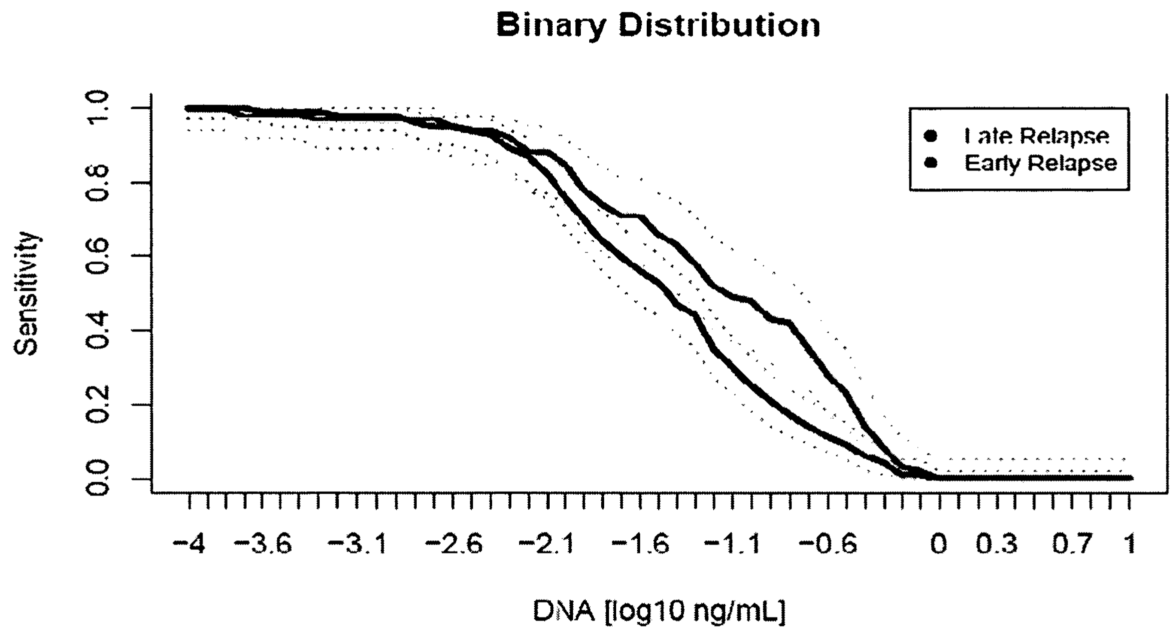
Binary Distribution
Late Relapse
Early Relapse
Sensitivity
0.0 0.2 0.4 0.6 0.8 1.0
-4 -3.6 -3.1 -2.6 -2.1 -1.6 -1.1 -0.6 0 0.3 0.7 1
DNA [log10 ng/mL]

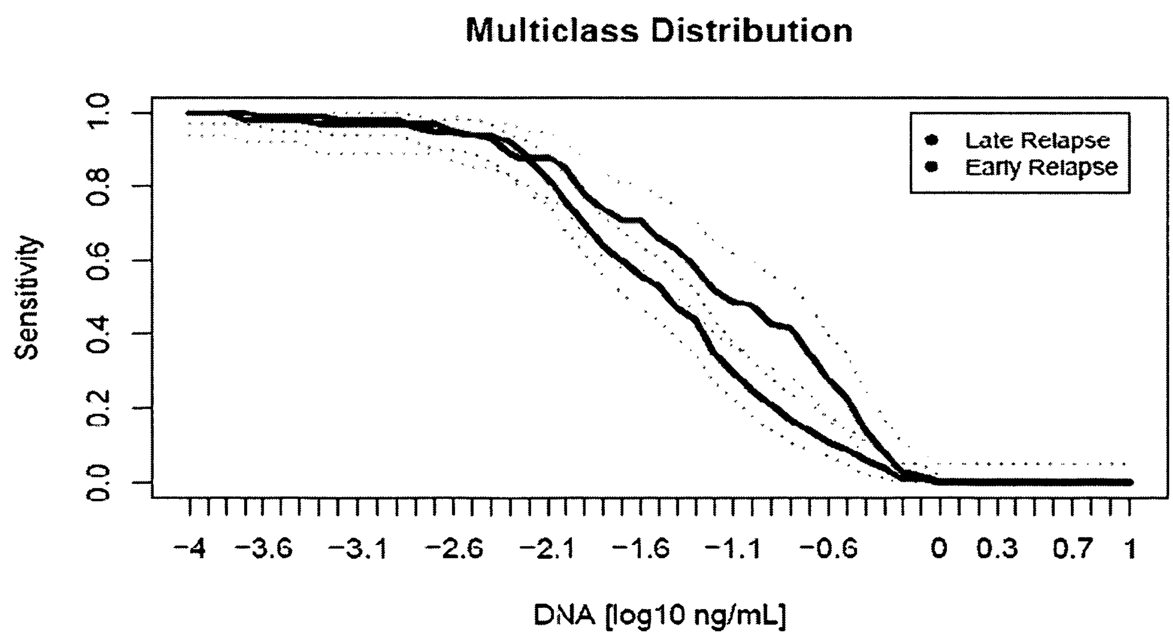
Multiclass Distribution
Late Relapse
Early Relapse
Sensitivity
0.0 0.2 0.4 0.6 0.8 1.0
-4 -3.6 -3.1 -2.6 -2.1 -1.6 -1.1 -0.6 0 0.3 0.7 1
DNA [log10 ng/mL]

Figure 102/1

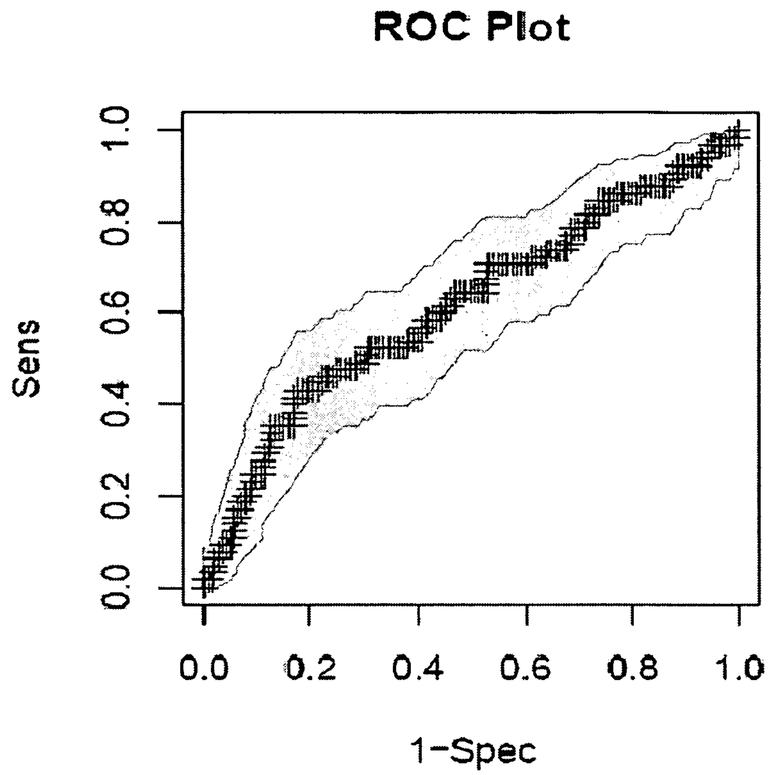


| | |
|---|---|
| AUC | 0.62 [0.54,0.68] |
| Sens / Spec | 0.35 / 0.86 |
| Wilcoxon *P* | 0.0072 |
| Early Relapse (pos) | 65 |
| Late Relapse (neg) | 140 |

Figure 102/2

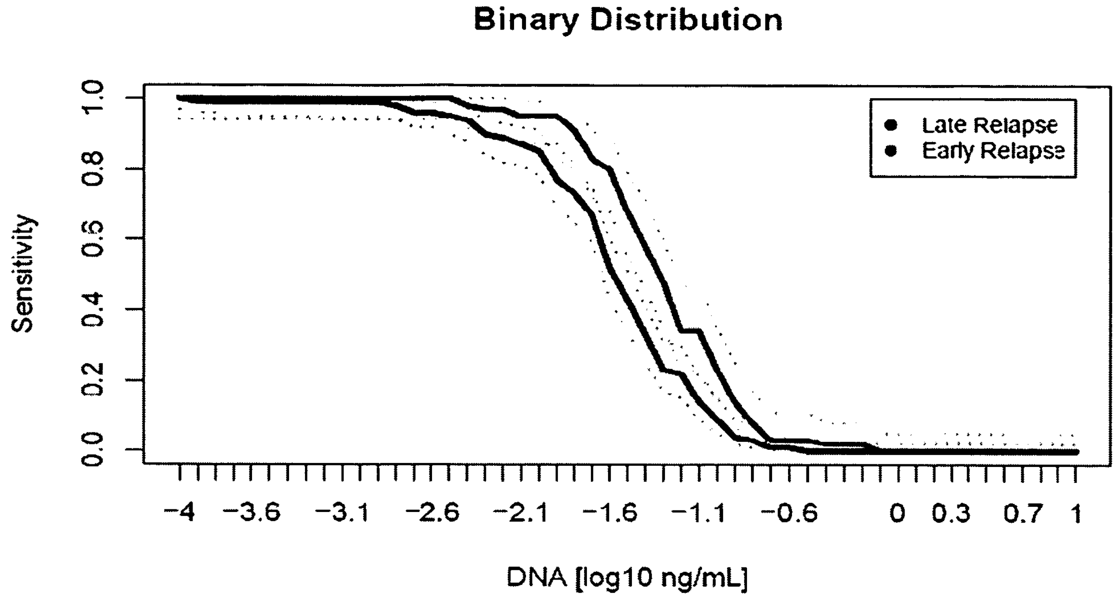
Binary Distribution
Sensitivity
0.0
0.2
0.4
0.6
0.8
1.0
Late Relapse
Early Relapse
-4
-3.6
-3.1
-2.6
-2.1
-1.6
-1.1
-0.6
0
0.3
0.7
1
DNA [log10 ng/mL]

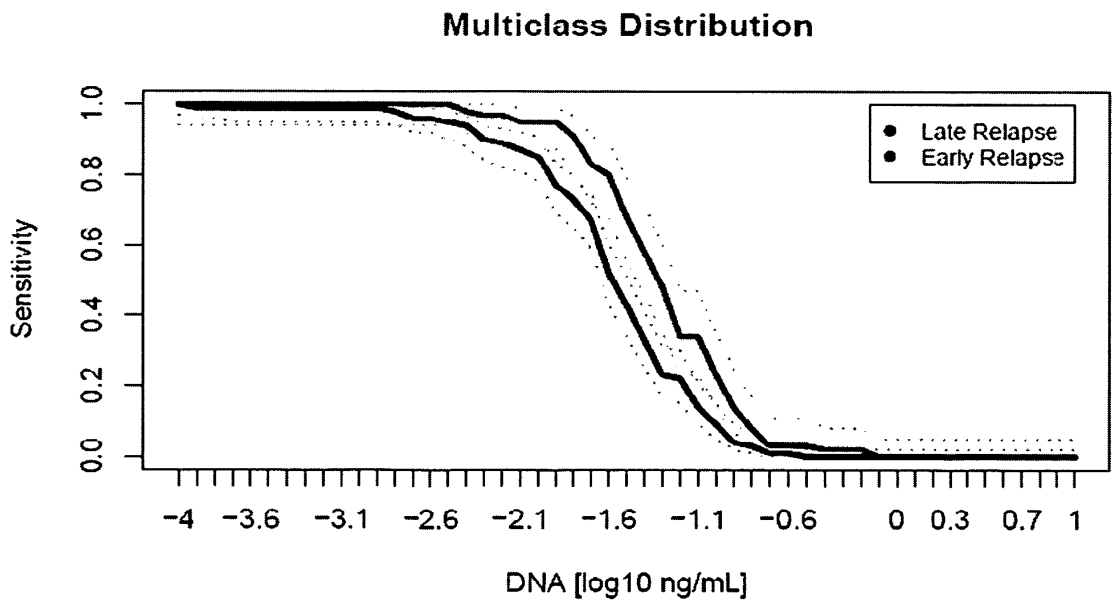
Multiclass Distribution
Sensitivity
0.0
0.2
0.4
0.6
0.8
1.0
Late Relapse
Early Relapse
-4
-3.6
-3.1
-2.6
-2.1
-1.6
-1.1
-0.6
0
0.3
0.7
1
DNA [log10 ng/mL]

Figure 103/1

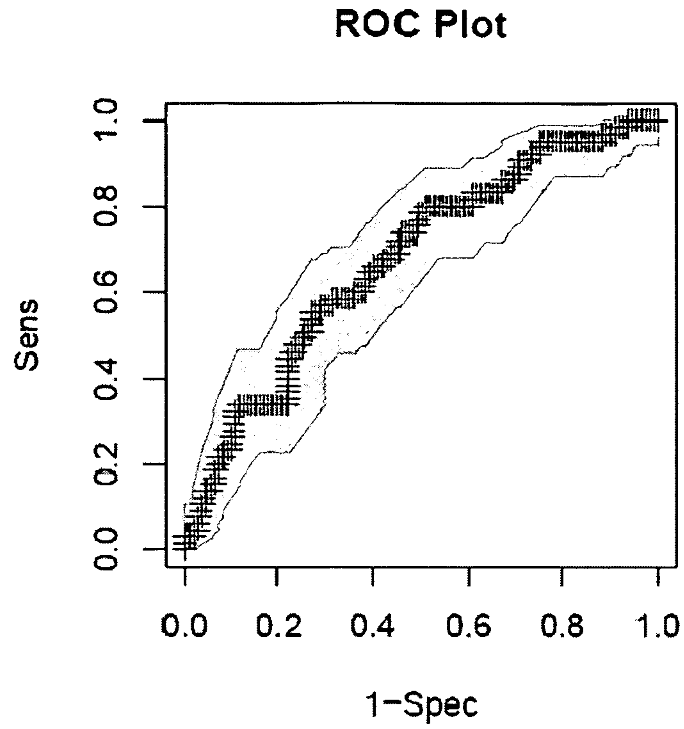


| | |
|---|---|
| AUC | 0.68 [0.61,0.74] |
| Sens / Spec | 0.34 / 0.85 |
| Wilcoxon *P* | 1e-04 |
| Early Relapse (pos) | 65 |
| Late Relapse (neg) | 141 |

**Figure 103/2**

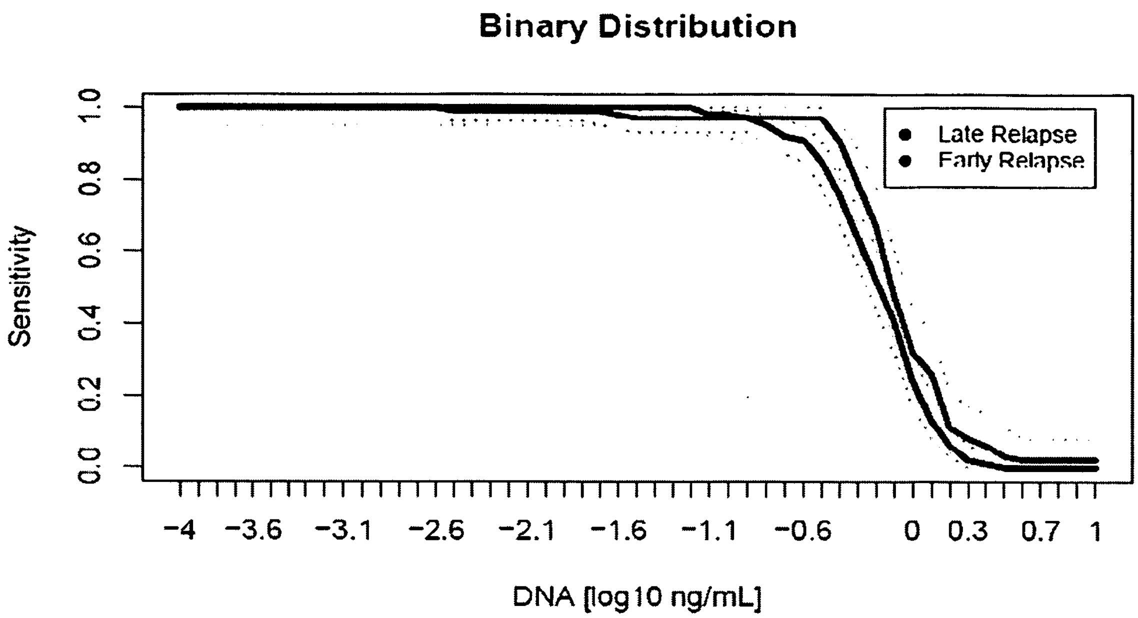
Binary Distribution
Sensitivity
1.0
0.8
0.6
0.4
0.2
0.0
Late Relapse
Early Relapse
-4 -3.6 -3.1 -2.6 -2.1 -1.6 -1.1 -0.6 0 0.3 0.7 1
DNA [log10 ng/mL]

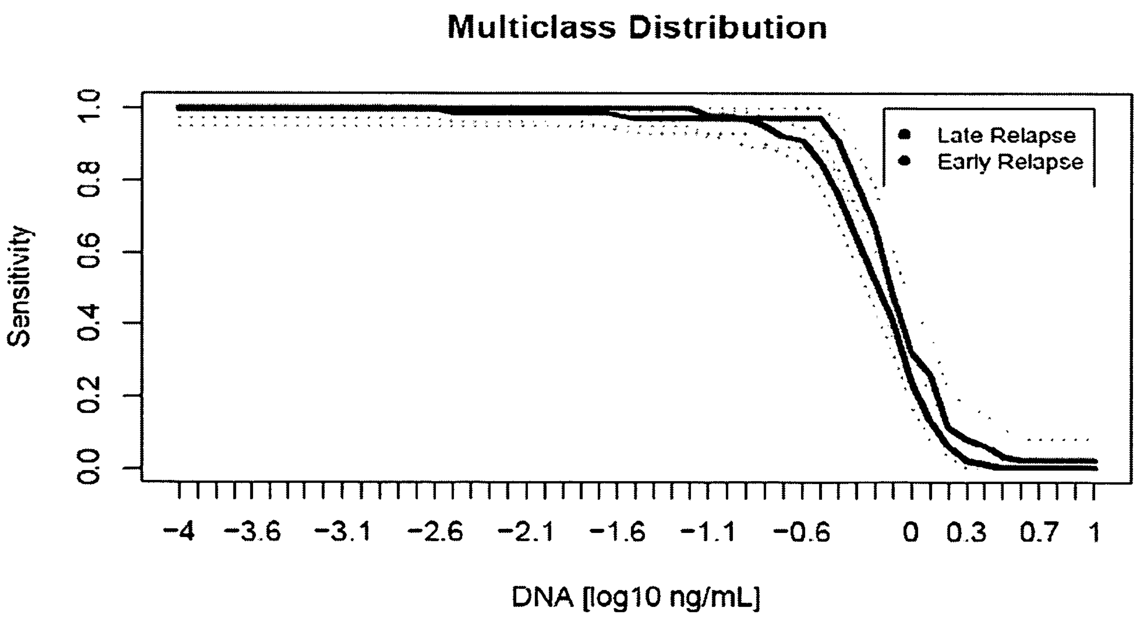
Multiclass Distribution
Sensitivity
1.0
0.8
0.6
0.4
0.2
0.0
Late Relapse
Early Relapse
-4 -3.6 -3.1 -2.6 -2.1 -1.6 -1.1 -0.6 0 0.3 0.7 1
DNA [log10 ng/mL]

Figure 104/1

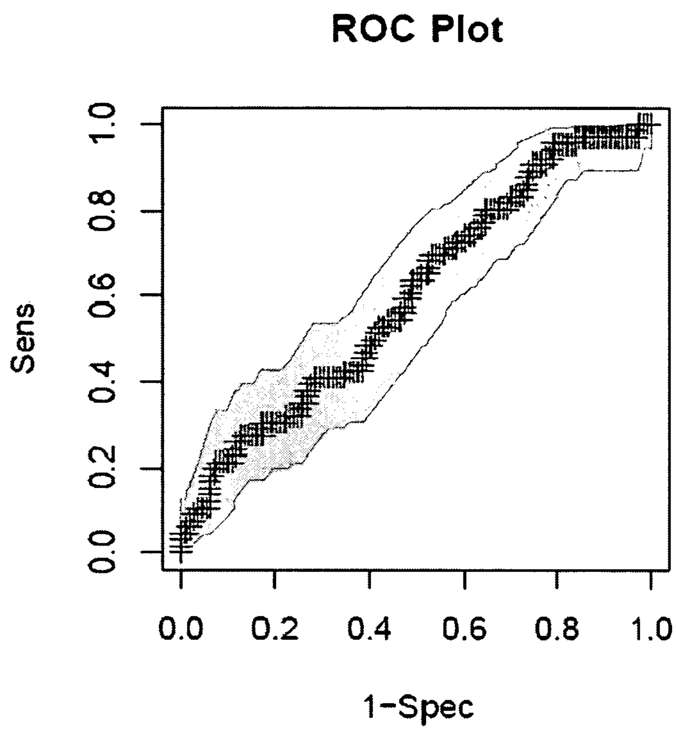


| | |
|---|---|
| AUC | 0.6 [0.53,0.67] |
| Sens / Spec | 0.27 / 0.85 |
| Wilcoxon *P* | 0.0178 |
| Early Relapse (pos) | 66 |
| Late Relapse (neg) | 143 |

**Figure 104/2**

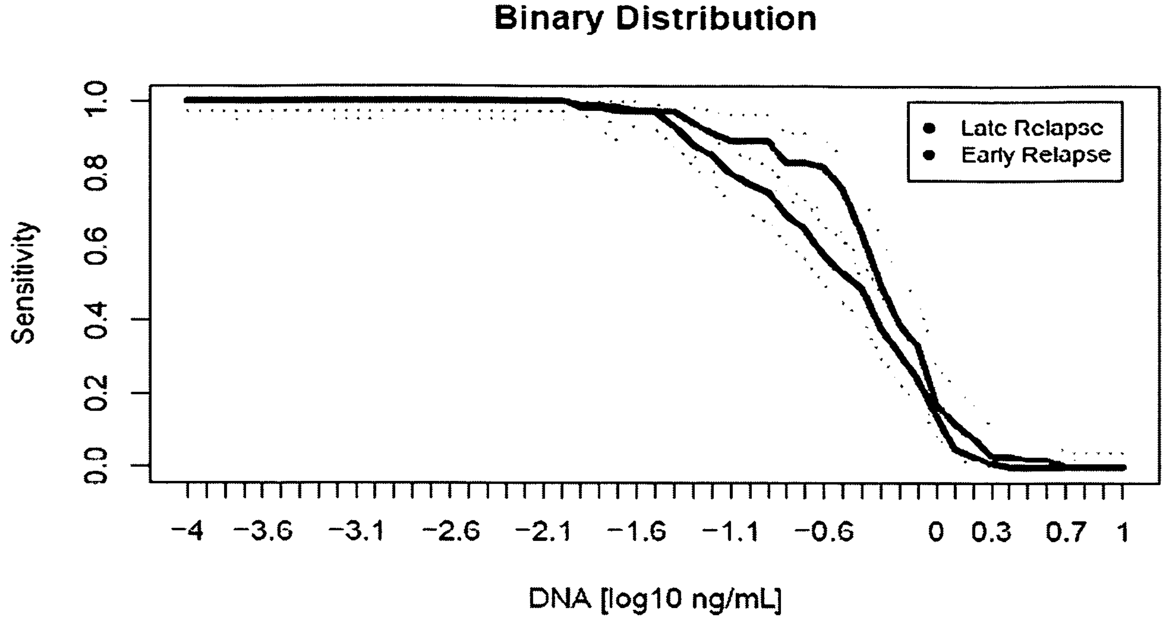
Binary Distribution
Late Relapse
Early Relapse
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

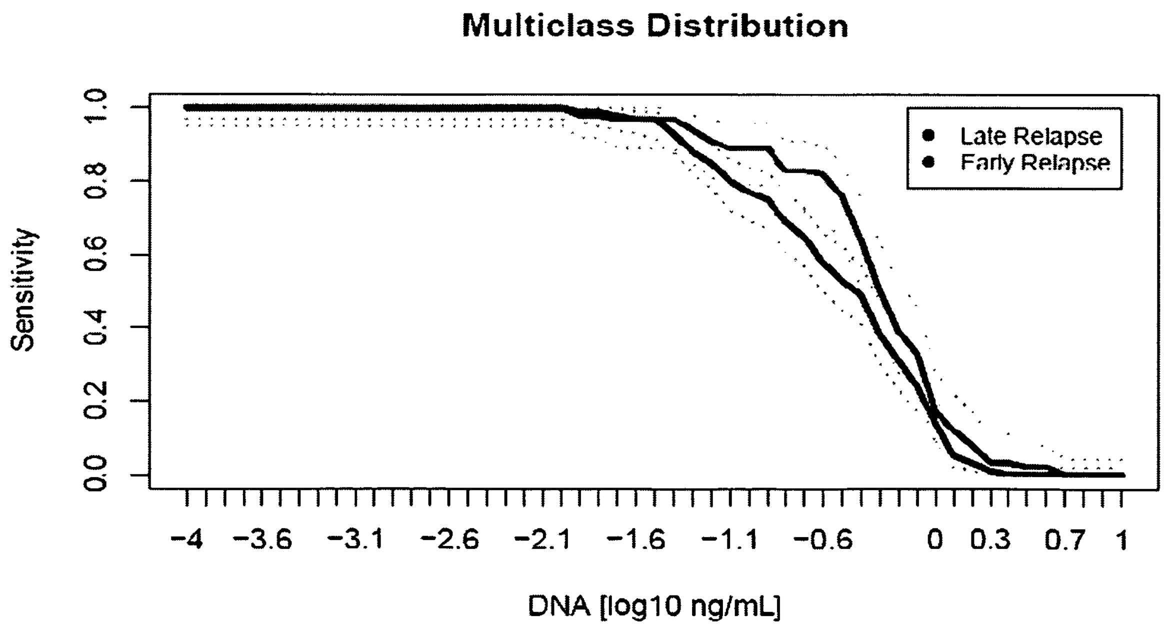
Multiclass Distribution
Late Relapse
Early Relapse
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

Figure 105/1

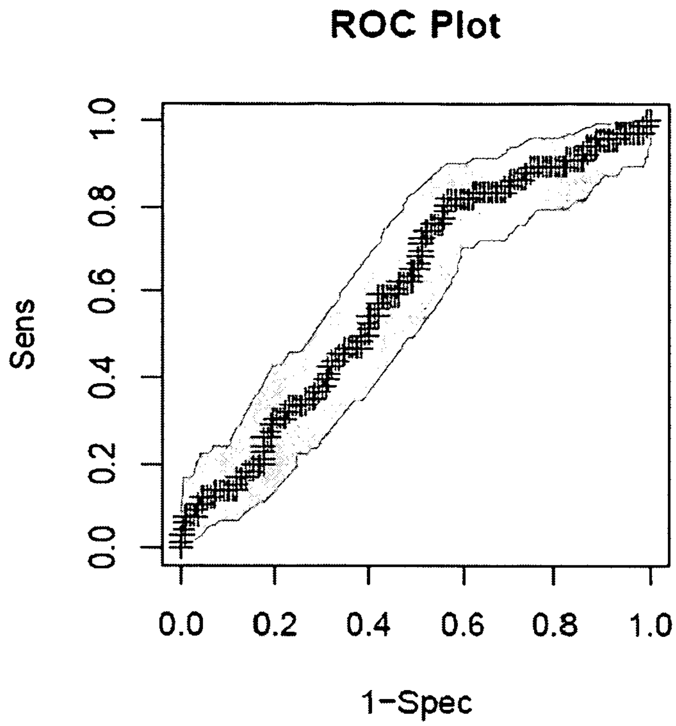


| | |
|---|---|
| AUC | 0.61 [0.53,0.67] |
| Sens / Spec | 0.18 / 0.85 |
| Wilcoxon *P* | 0.0146 |
| Early Relapse (pos) | 66 |
| Late Relapse (neg) | 143 |

**Figure 105/2**

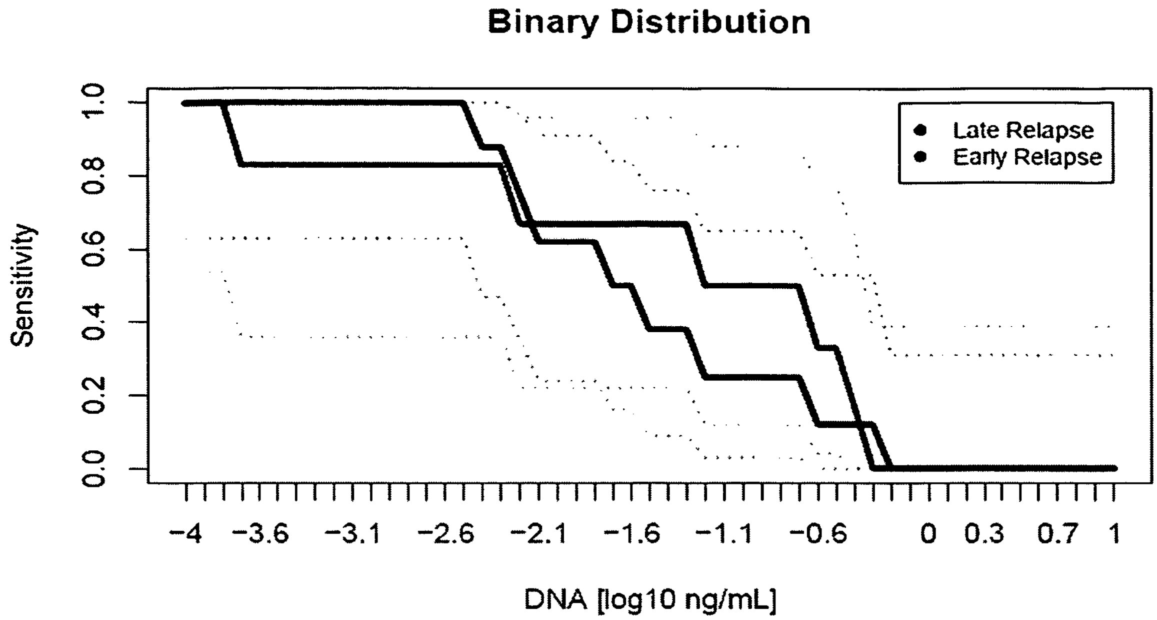
Binary Distribution
Late Relapse
Early Relapse
Sensitivity
0.0
0.2
0.4
0.6
0.8
1.0
−4
−3.6
−3.1
−2.6
−2.1
−1.6
−1.1
−0.6
0
0.3
0.7
1
DNA [log10 ng/mL]

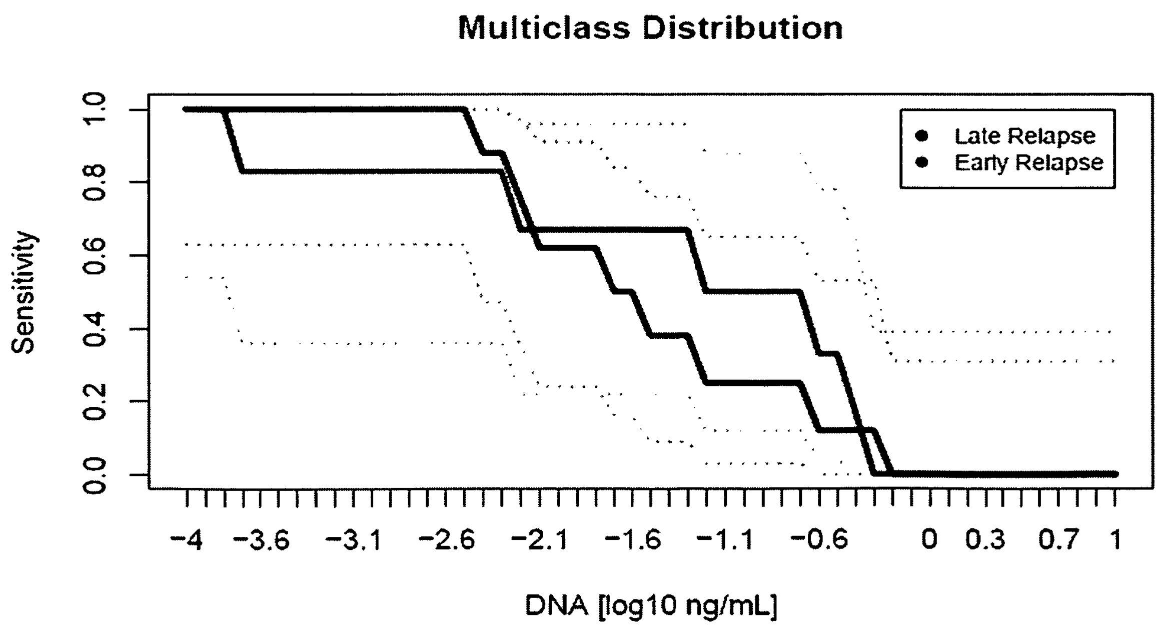
Multiclass Distribution
Late Relapse
Early Relapse
Sensitivity
0.0
0.2
0.4
0.6
0.8
1.0
−4
−3.6
−3.1
−2.6
−2.1
−1.6
−1.1
−0.6
0
0.3
0.7
1
DNA [log10 ng/mL]

Figure 106/1

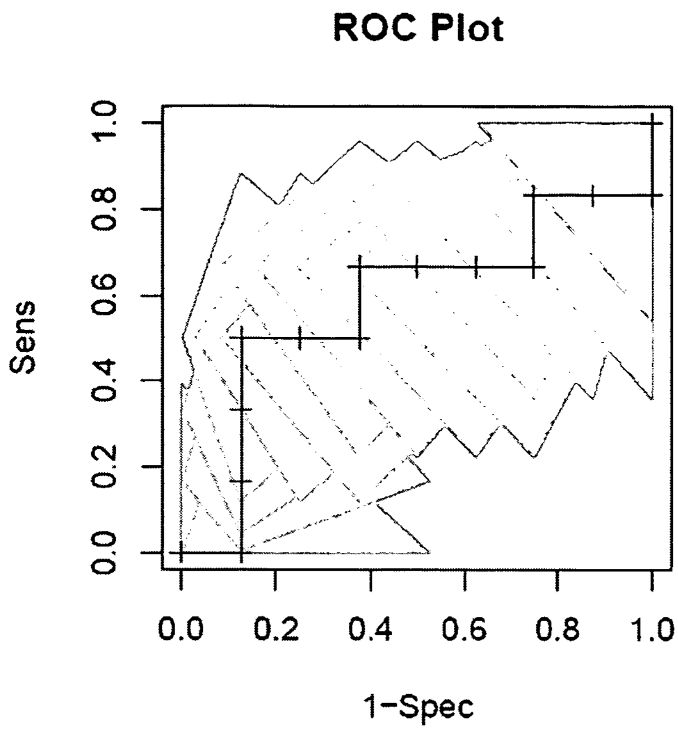


| | |
|---|---|
| AUC | 0.58 [0.29,0.82] |
| Sens / Spec | 0.5 / 0.88 |
| Wilcoxon *P* | 0.662 |
| Early Relapse (pos) | 6 |
| Late Relapse (neg) | 8 |

Figure 106/2

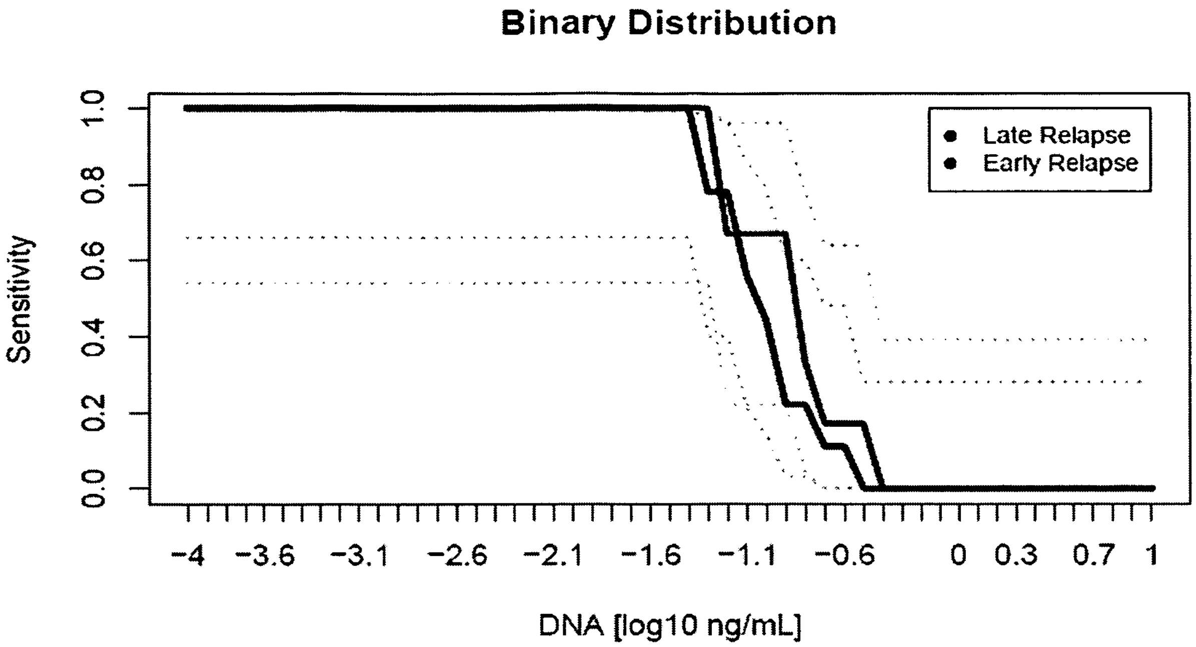
Binary Distribution
Late Relapse
Early Relapse
Sensitivity
1.0
0.8
0.6
0.4
0.2
0.0
-4 -3.6 -3.1 -2.6 -2.1 -1.6 -1.1 -0.6 0 0.3 0.7 1
DNA [log10 ng/mL]

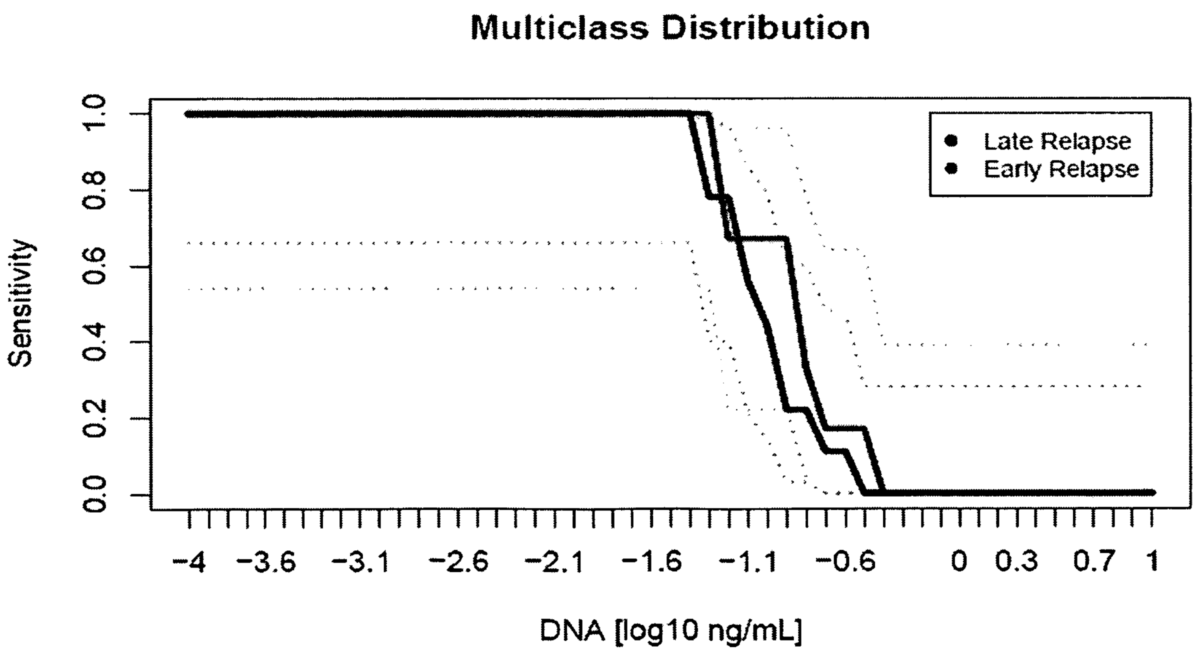
Multiclass Distribution
Late Relapse
Early Relapse
Sensitivity
1.0
0.8
0.6
0.4
0.2
0.0
-4 -3.6 -3.1 -2.6 -2.1 -1.6 -1.1 -0.6 0 0.3 0.7 1
DNA [log10 ng/mL]

Figure 107/1

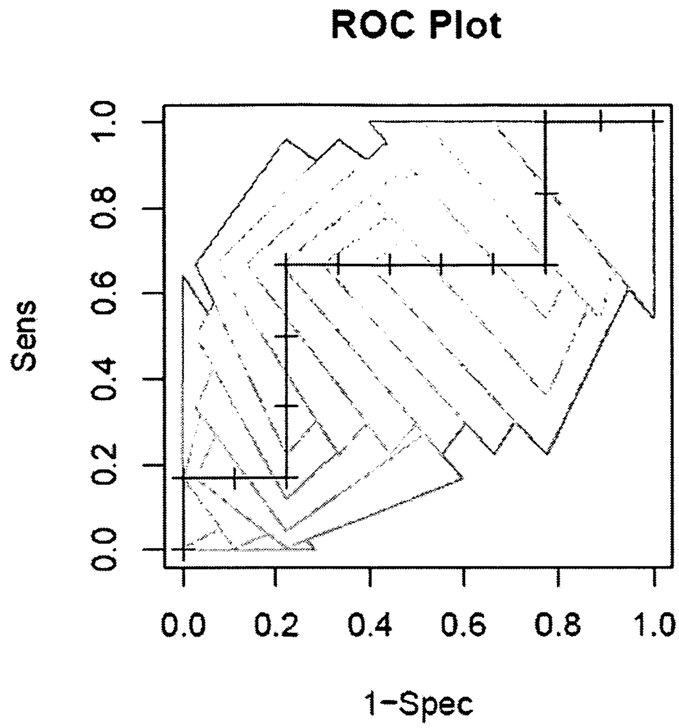


| | |
|---|---|
| AUC | 0.63 [0.32,0.84] |
| Sens / Spec | 0.17 / 0.89 |
| Wilcoxon *P* | 0.4559 |
| Early Relapse (pos) | 6 |
| Late Relapse (neg) | 9 |

**Figure 107/2**

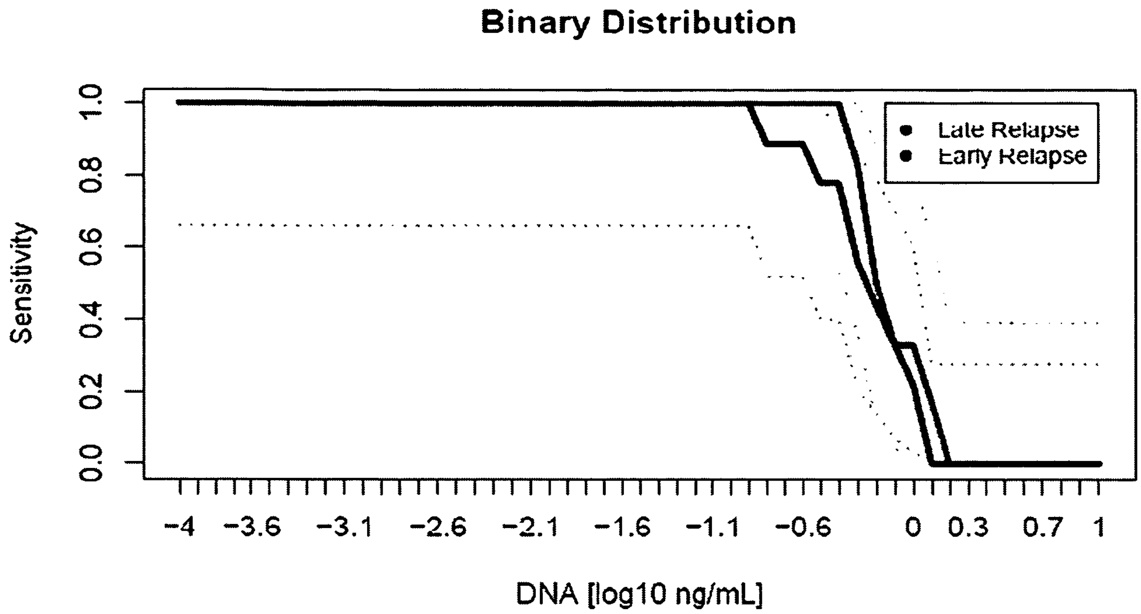
Binary Distribution
Late Relapse
Early Relapse
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

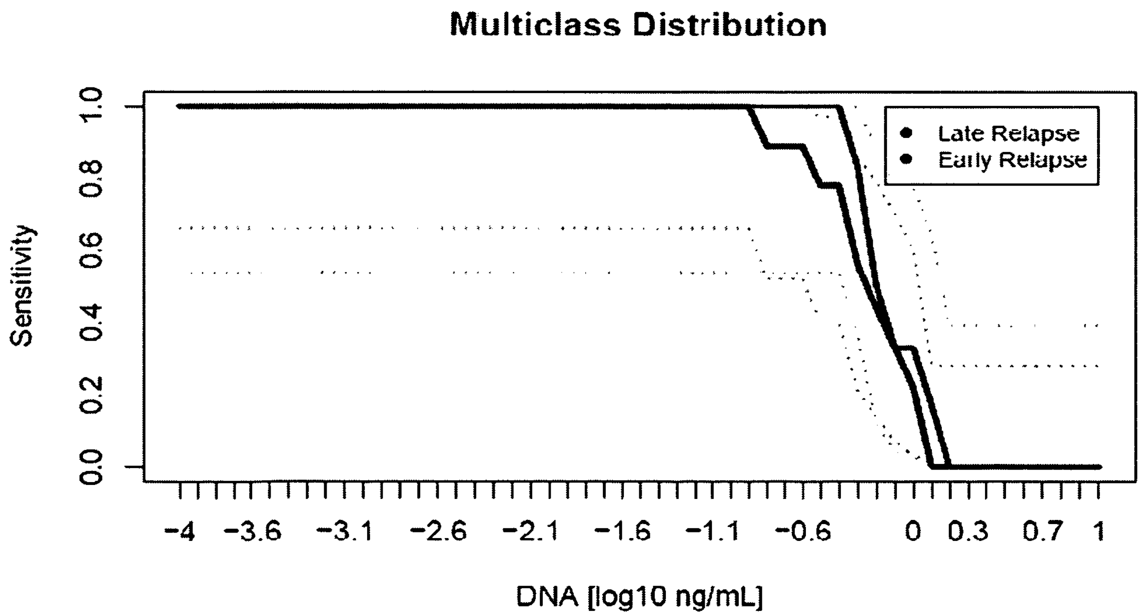
Multiclass Distribution
Late Relapse
Early Relapse
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

Figure 108/1

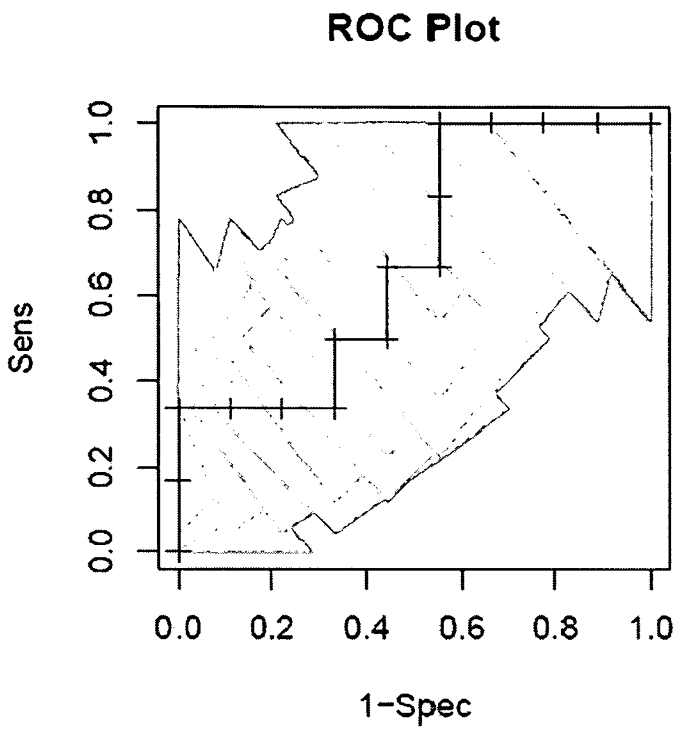


| | |
|---|---|
| AUC | 0.69 [0.38,0.88] |
| Sens / Spec | 0.33 / 0.89 |
| Wilcoxon *P* | 0.2721 |
| Early Relapse (pos) | 6 |
| Late Relapse (neg) | 9 |

**Figure 108/2**

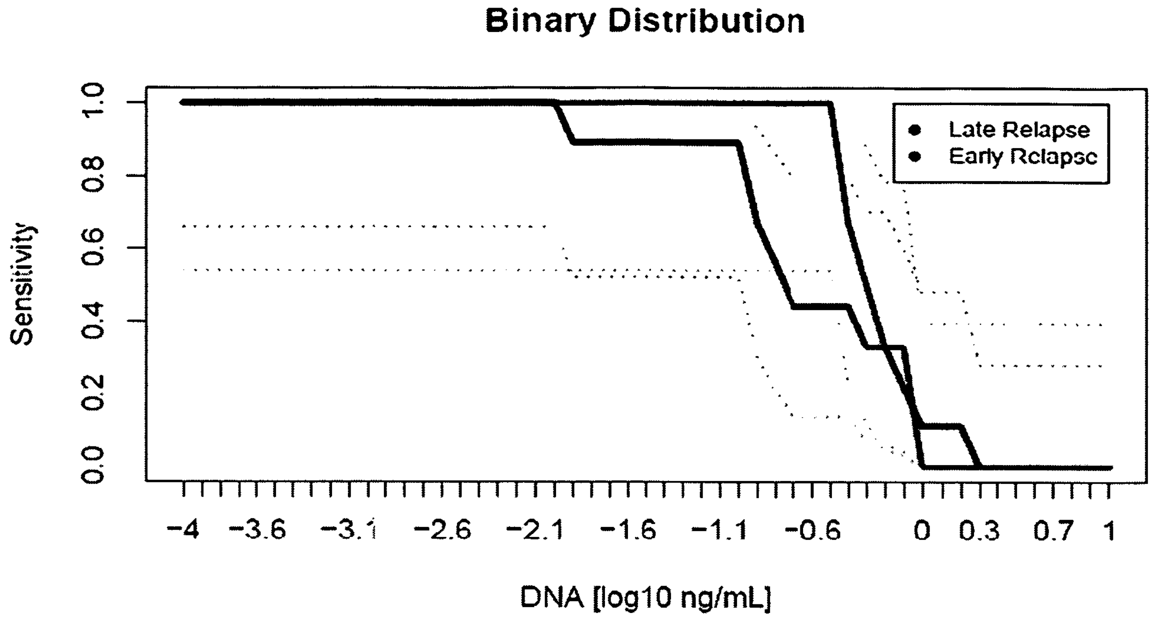
Binary Distribution
Late Relapse
Early Relapse
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

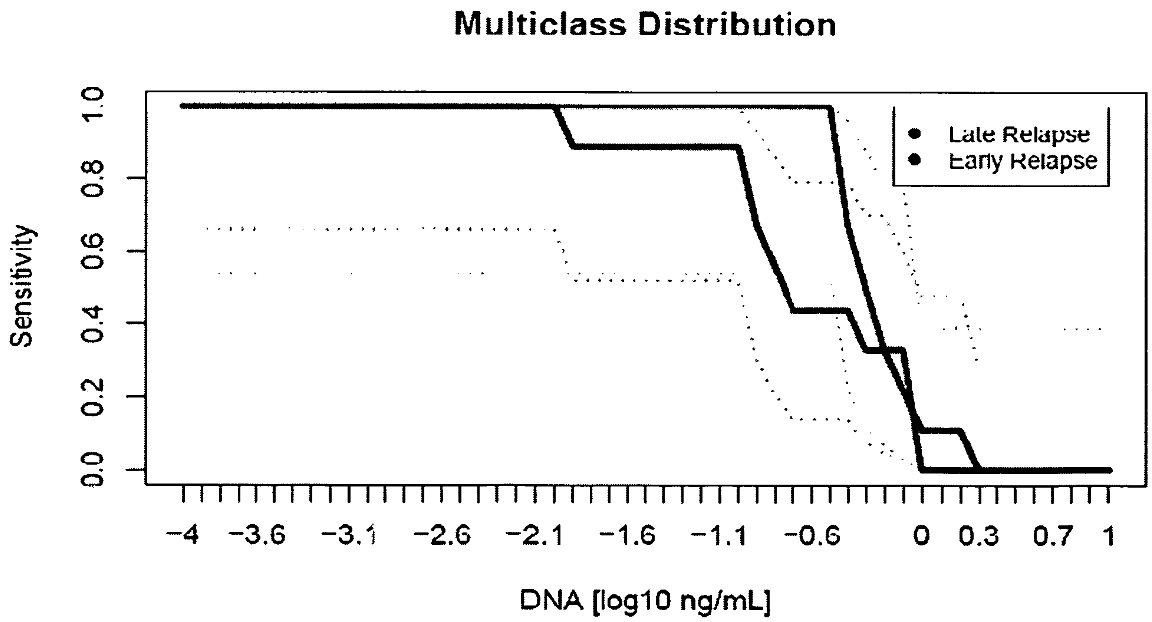
Multiclass Distribution
Late Relapse
Early Relapse
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

Figure 109/1

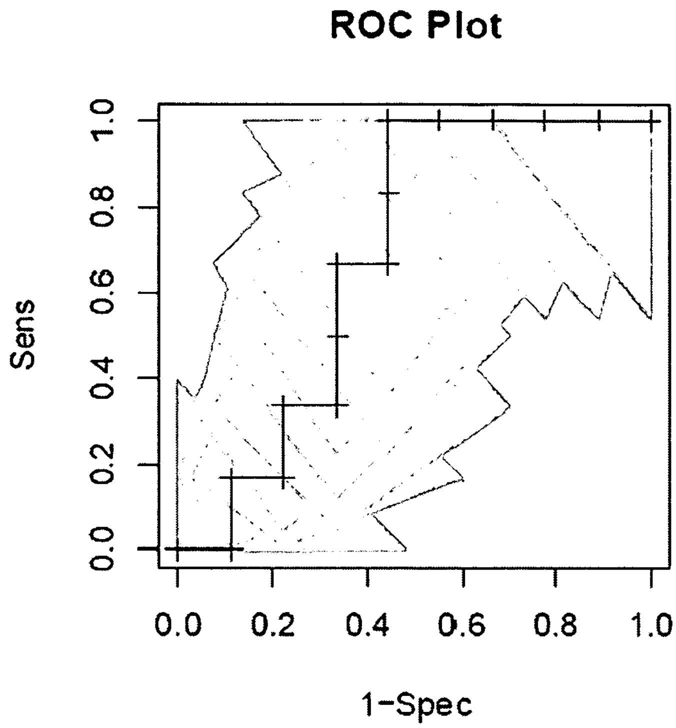


| | |
|---|---|
| AUC | 0.69 [0.38,0.88] |
| Sens / Spec | 0.17 / 0.89 |
| Wilcoxon *P* | 0.2721 |
| Early Relapse (pos) | 6 |
| Late Relapse (neg) | 9 |

**Figure 109/2**

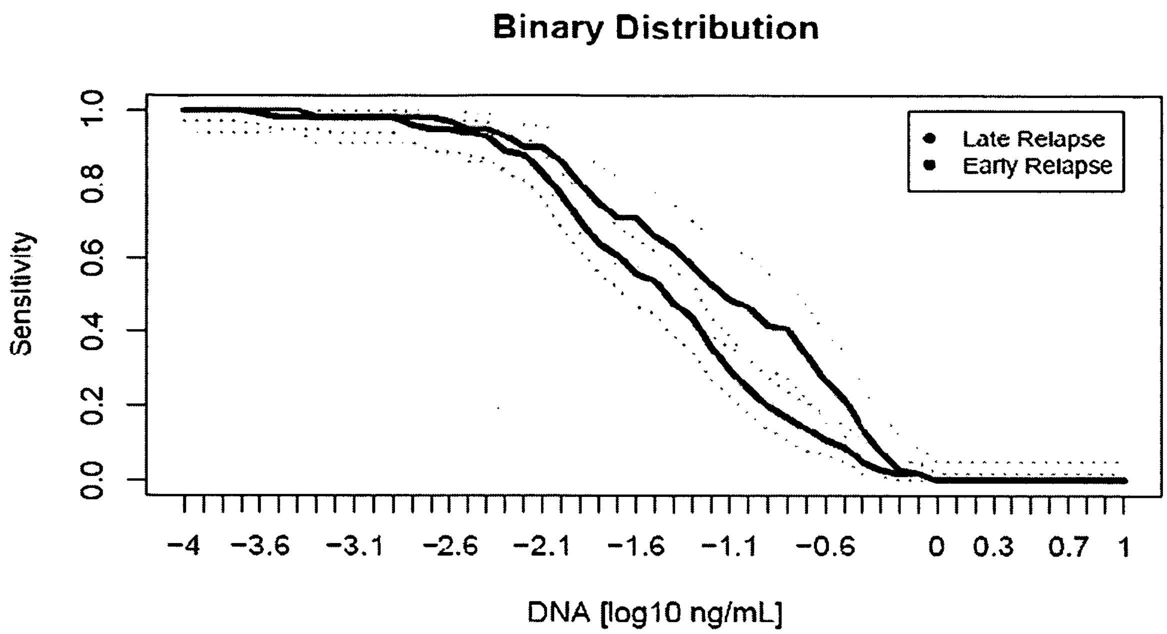
Binary Distribution
Late Relapse
Early Relapse
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

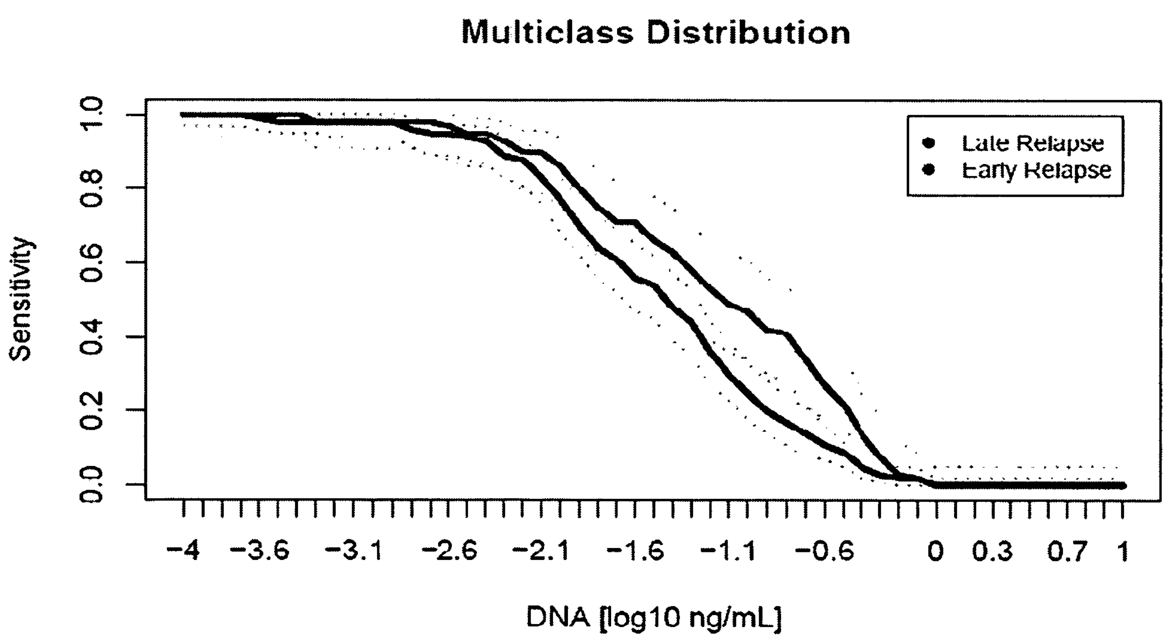
Multiclass Distribution
Late Relapse
Early Relapse
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

Figure 110/1

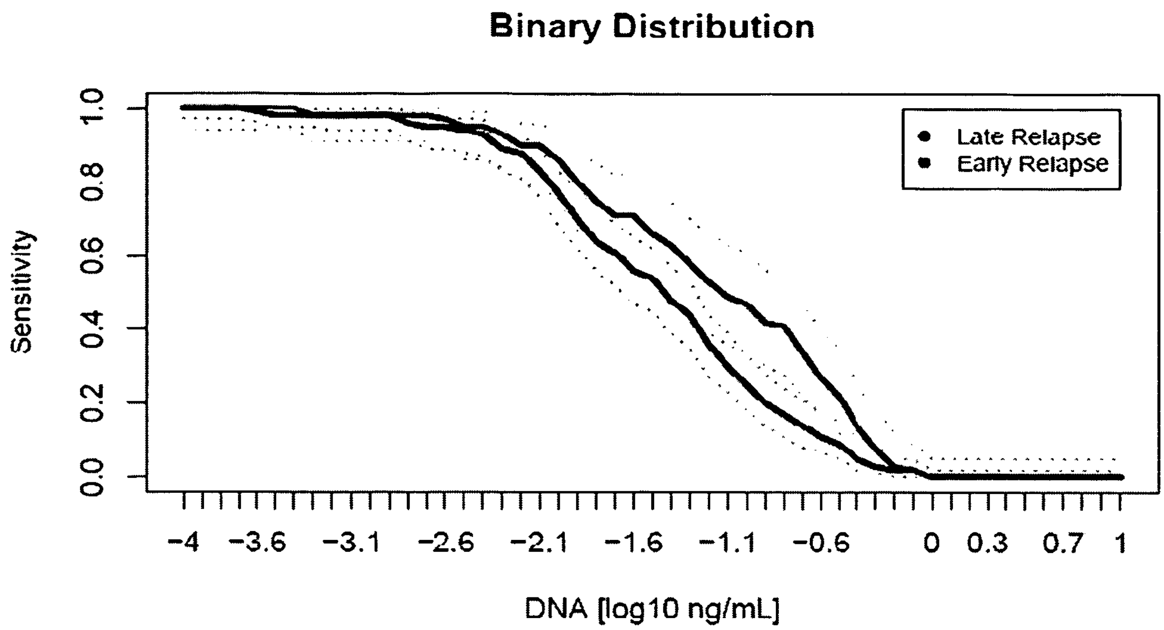
Binary Distribution
Late Relapse
Early Relapse
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

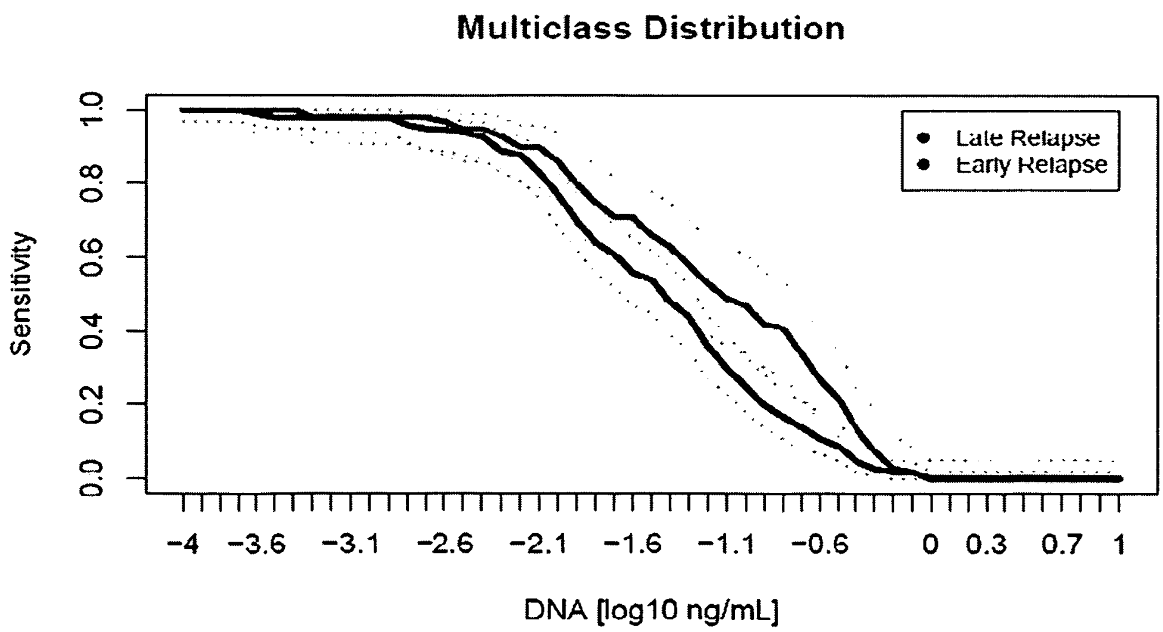
Multiclass Distribution
Late Relapse
Early Relapse
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

Figure 110/1

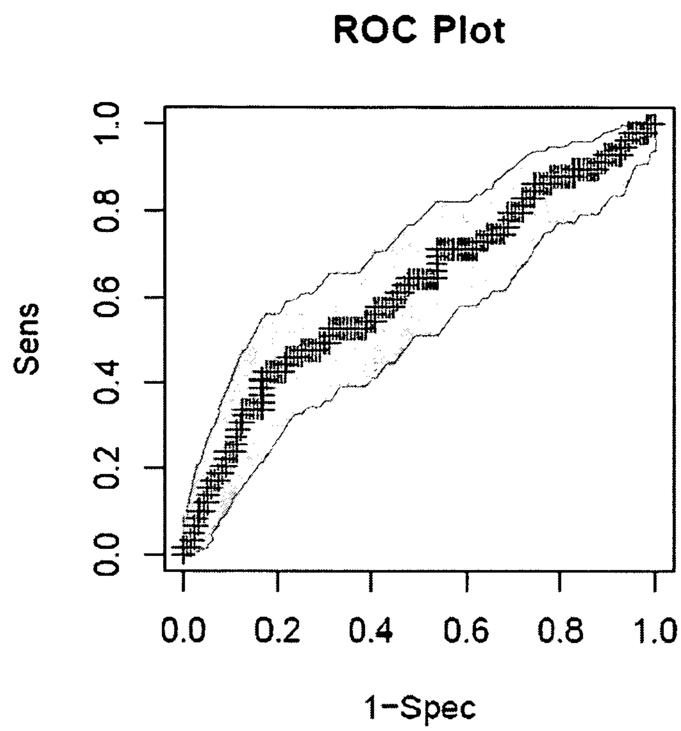


| | |
|---|---|
| AUC | 0.62 [0.54,0.69] |
| Sens / Spec | 0.34 / 0.86 |
| Wilcoxon *P* | 0.0085 |
| Early Relapse (pos) | 59 |
| Late Relapse (neg) | 132 |

Figure 110/2

## Binary Distribution

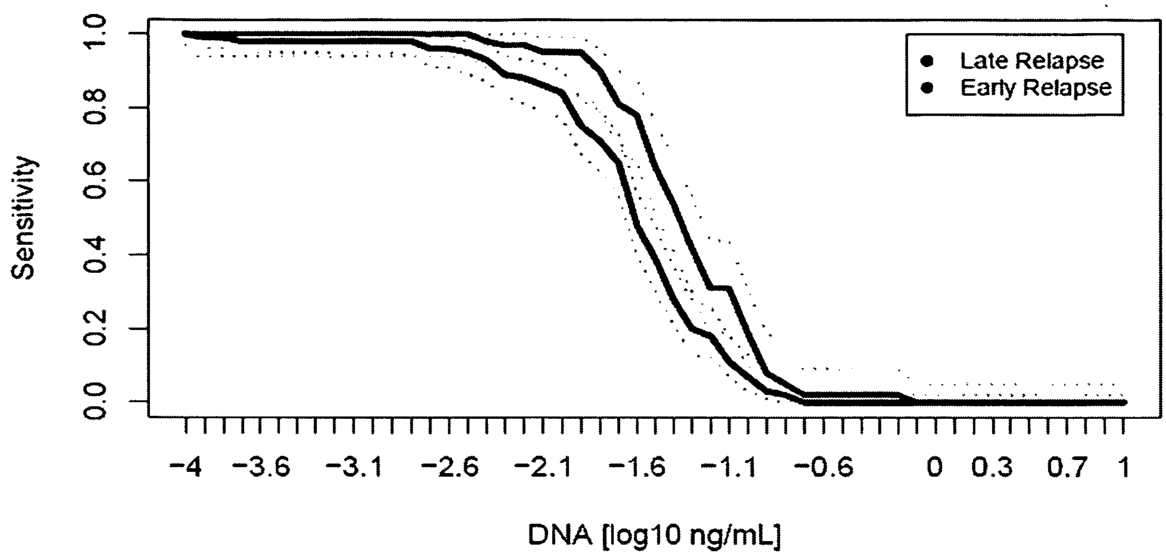


## Multiclass Distribution

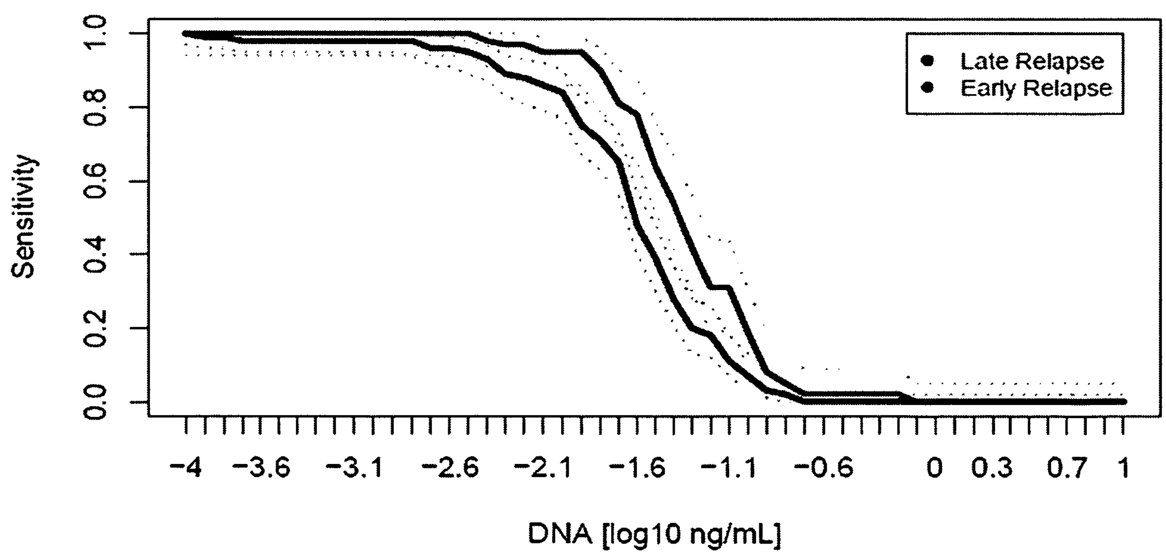


Figure 111/1

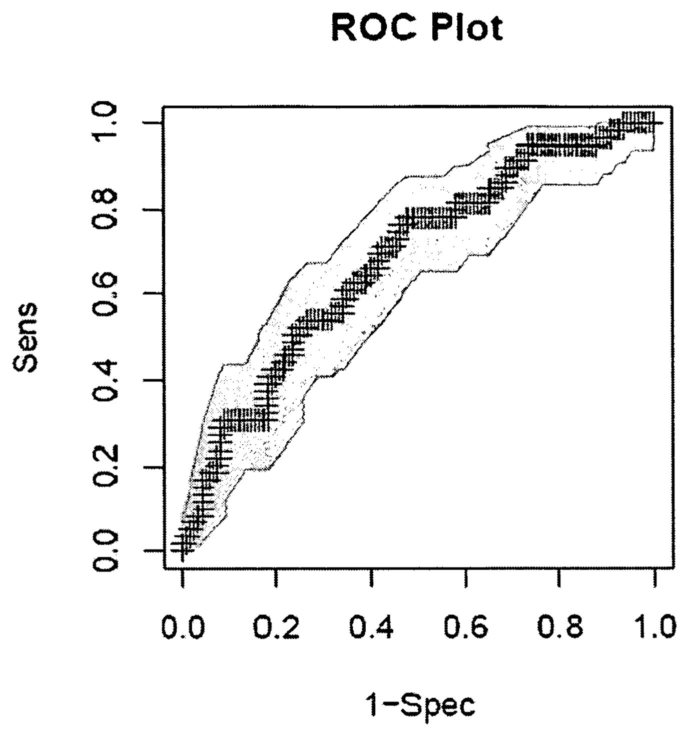


| | |
|---|---|
| AUC | 0.68 [0.61,0.75] |
| Sens / Spec | 0.31 / 0.86 |
| Wilcoxon *P* | 1e-04 |
| Early Relapse (pos) | 59 |
| Late Relapse (neg) | 132 |

**Figure 111/2**

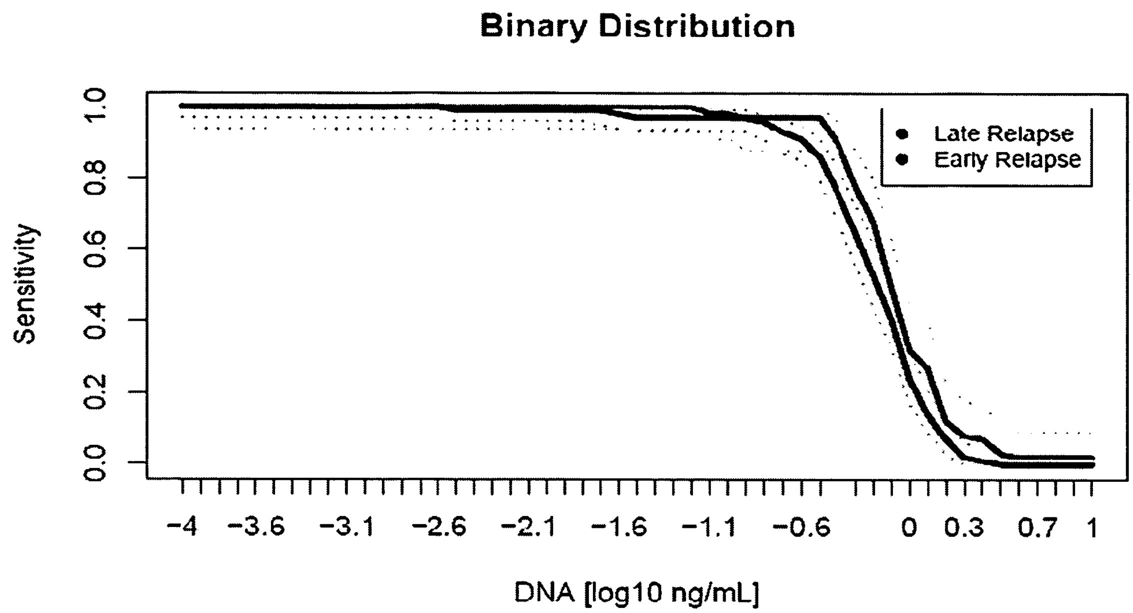
Binary Distribution
Sensitivity
0.0
0.2
0.4
0.6
0.8
1.0
Late Relapse
Early Relapse
-4
-3.6
-3.1
-2.6
-2.1
-1.6
-1.1
-0.6
0
0.3
0.7
1
DNA [log10 ng/mL]

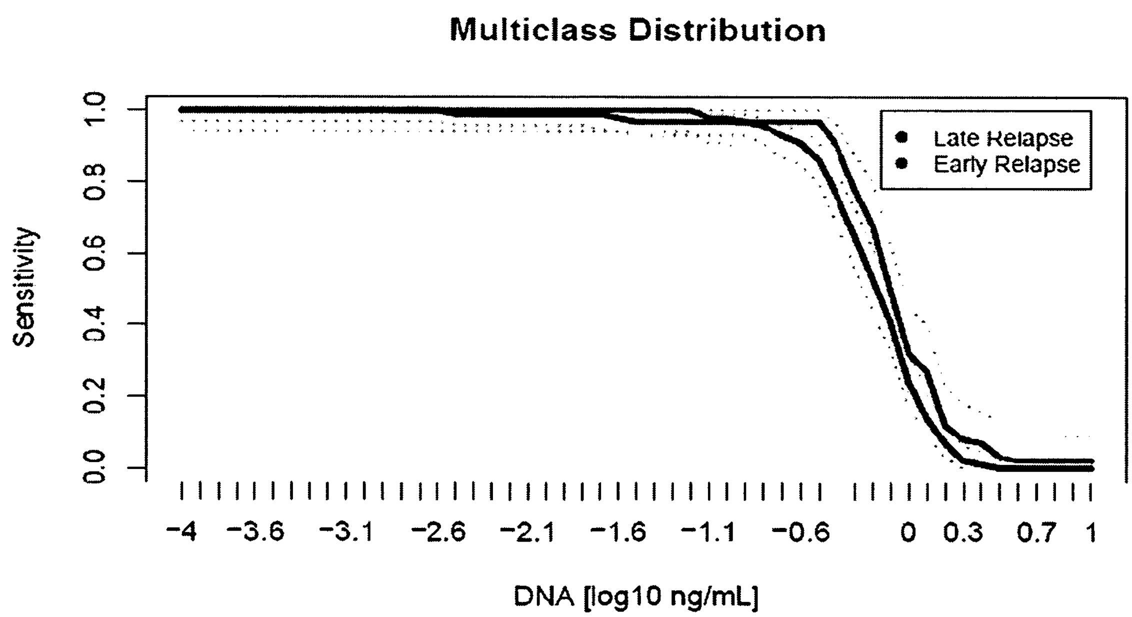
Multiclass Distribution
Sensitivity
0.0
0.2
0.4
0.6
0.8
1.0
Late Relapse
Early Relapse
-4
-3.6
-3.1
-2.6
-2.1
-1.6
-1.1
-0.6
0
0.3
0.7
1
DNA [log10 ng/mL]

Figure 112/1

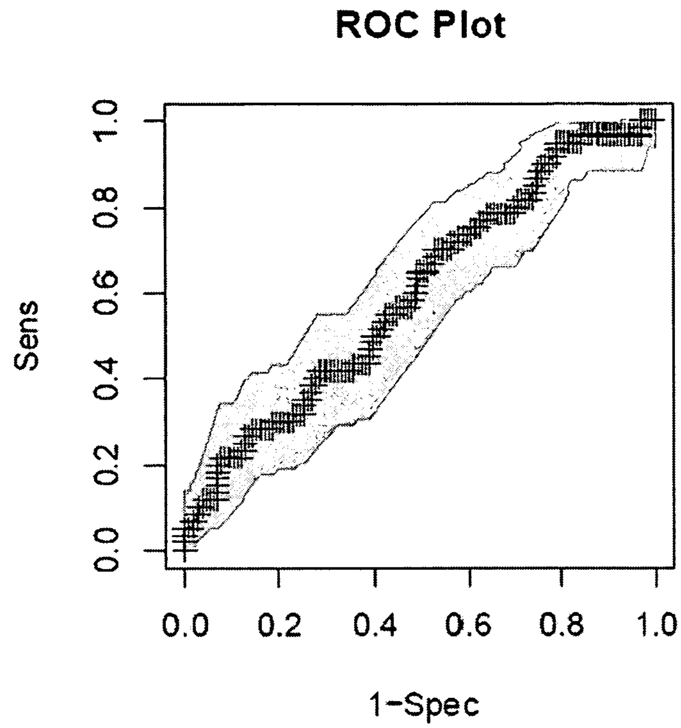


| | |
|---|---|
| AUC | 0.6 [0.53,0.67] |
| Sens / Spec | 0.28 / 0.85 |
| Wilcoxon *P* | 0.0264 |
| Early Relapse (pos) | 60 |
| Late Relapse (neg) | 134 |

**Figure 112/2**

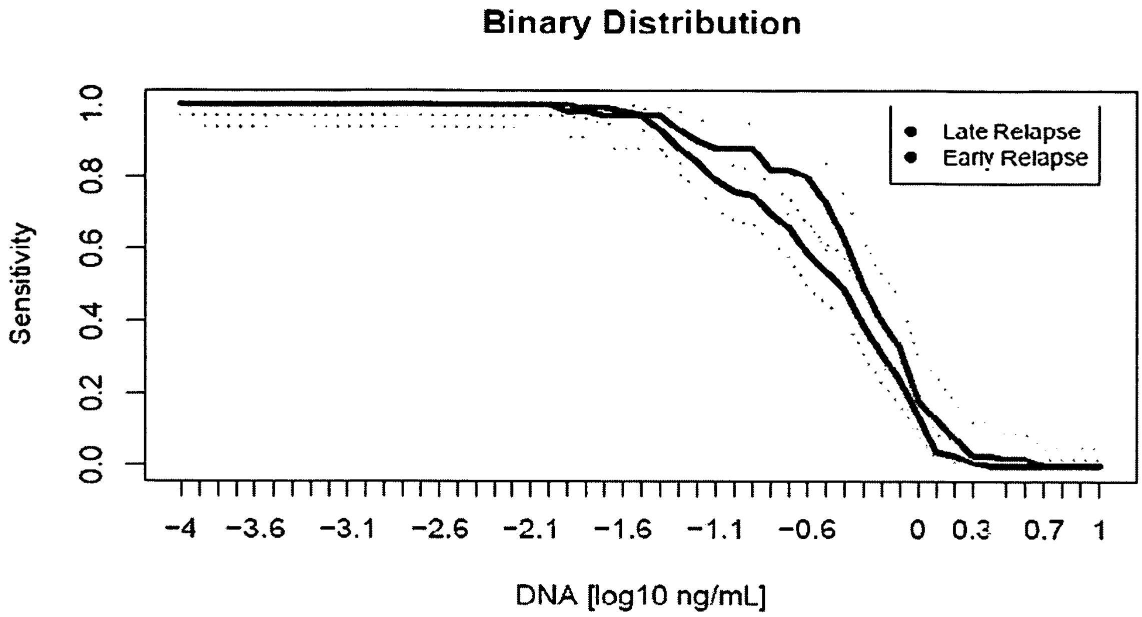
Binary Distribution
Late Relapse
Early Relapse
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

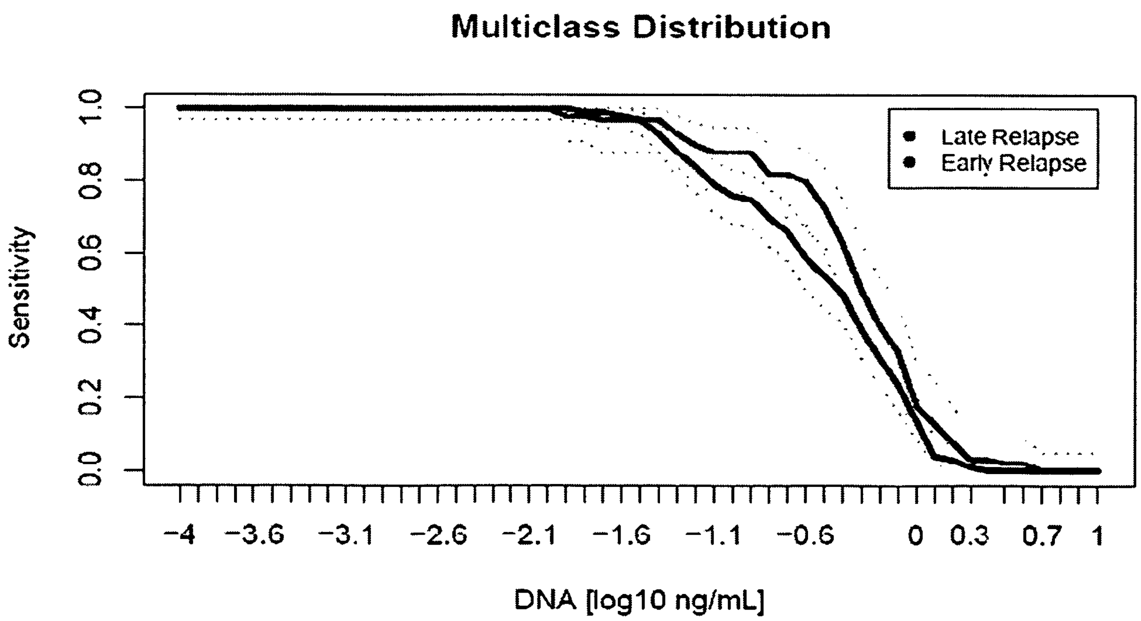
Multiclass Distribution
Late Relapse
Early Relapse
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

Figure 113/1

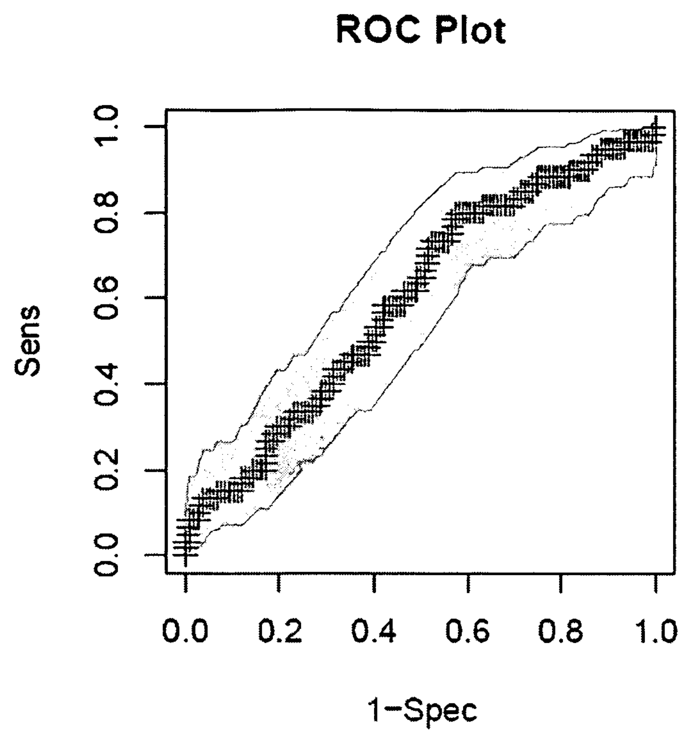


| | |
|---|---|
| AUC | 0.6 [0.53,0.67] |
| Sens / Spec | 0.2 / 0.85 |
| Wilcoxon *P* | 0.0272 |
| Early Relapse (pos) | 60 |
| Late Relapse (neg) | 134 |

**Figure 113/2**

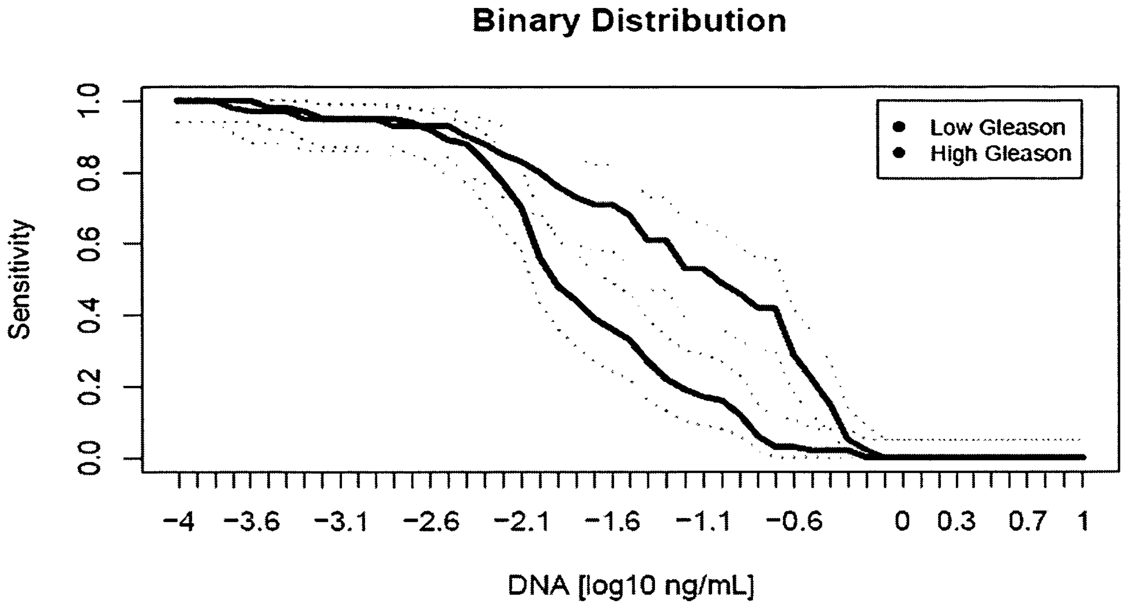
Binary Distribution
Low Gleason
High Gleason
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

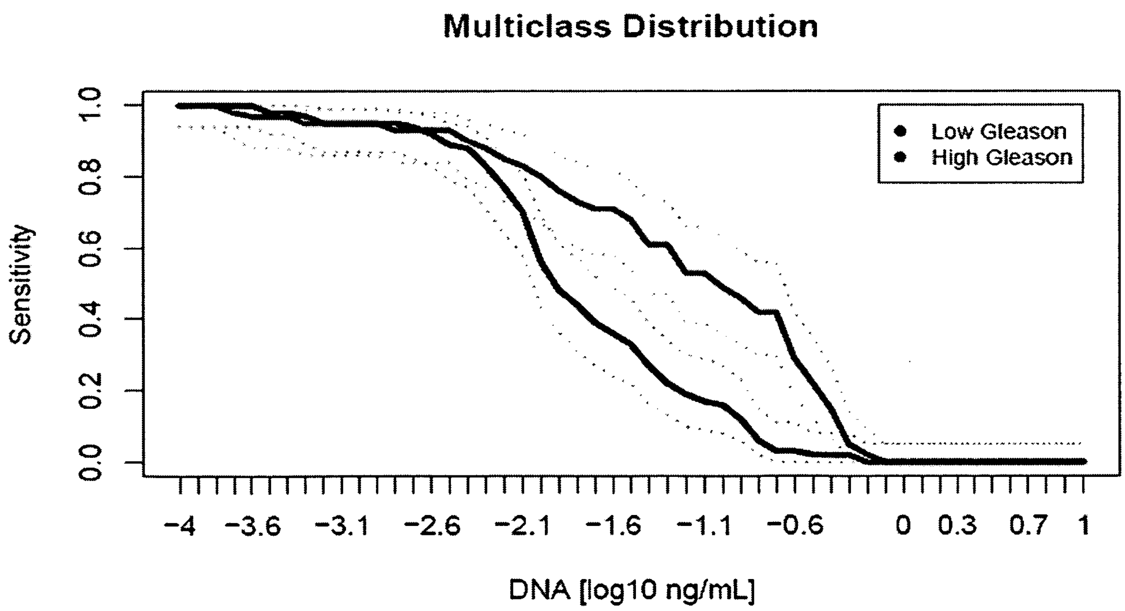
Multiclass Distribution
Low Gleason
High Gleason
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

Figure 114/1

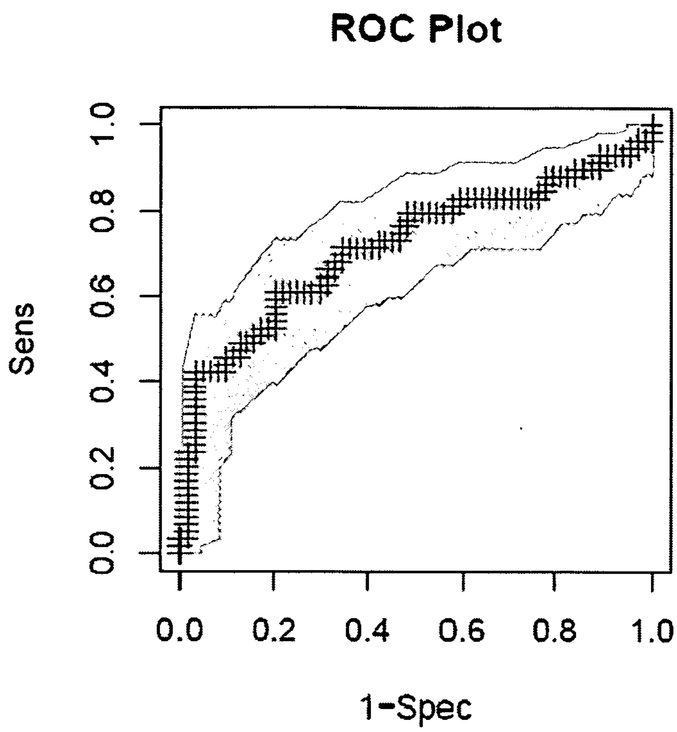


| | |
|---|---|
| AUC | 0.72 [0.63,0.79] |
| Sens / Spec | 0.49 / 0.86 |
| Wilcoxon *P* | 0 |
| High Gleason (pos) | 59 |
| Low Gleason (neg) | 64 |

**Figure 114/2**

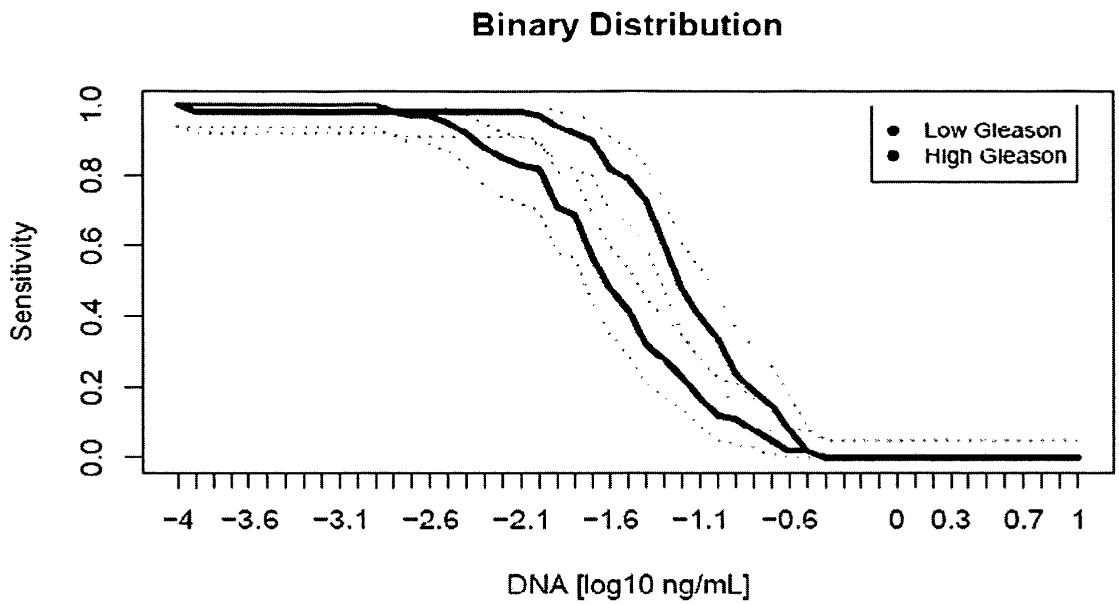
Binary Distribution
Sensitivity
0.0
0.2
0.4
0.6
0.8
1.0
Low Gleason
High Gleason
-4
-3.6
-3.1
-2.6
-2.1
-1.6
-1.1
-0.6
0
0.3
0.7
1
DNA [log10 ng/mL]

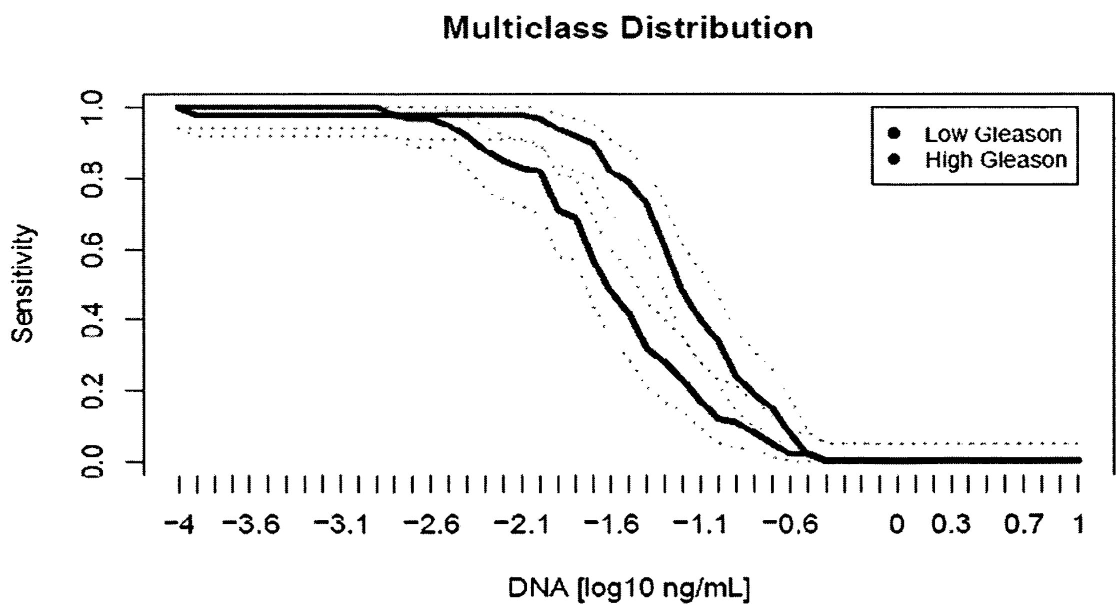
Multiclass Distribution
Sensitivity
0.0
0.2
0.4
0.6
0.8
1.0
Low Gleason
High Gleason
-4
-3.6
-3.1
-2.6
-2.1
-1.6
-1.1
-0.6
0
0.3
0.7
1
DNA [log10 ng/mL]

Figure 115/1

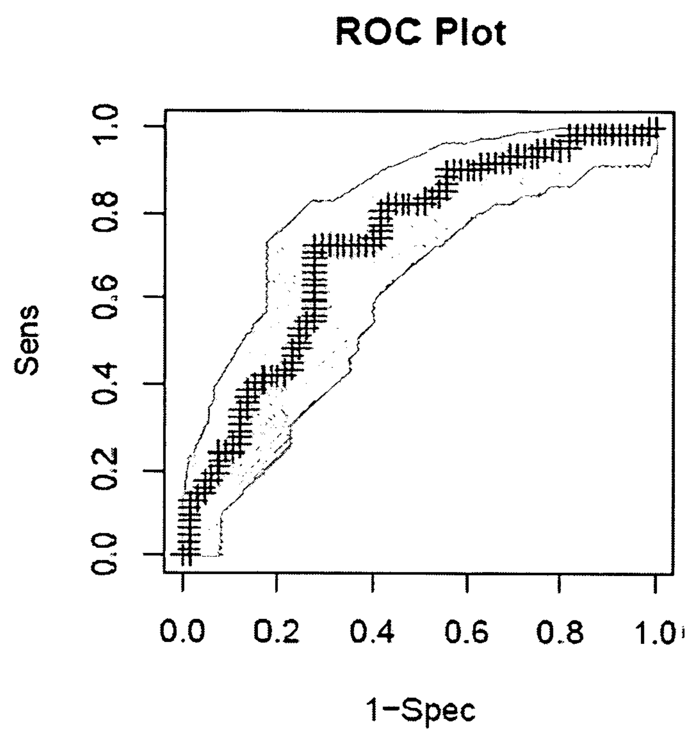


| | |
|---|---|
| AUC | 0.73 [0.64,0.8] |
| Sens / Spec | 0.39 / 0.86 |
| Wilcoxon *P* | 0 |
| High Gleason (pos) | 62 |
| Low Gleason (neg) | 65 |

**Figure 115/2**

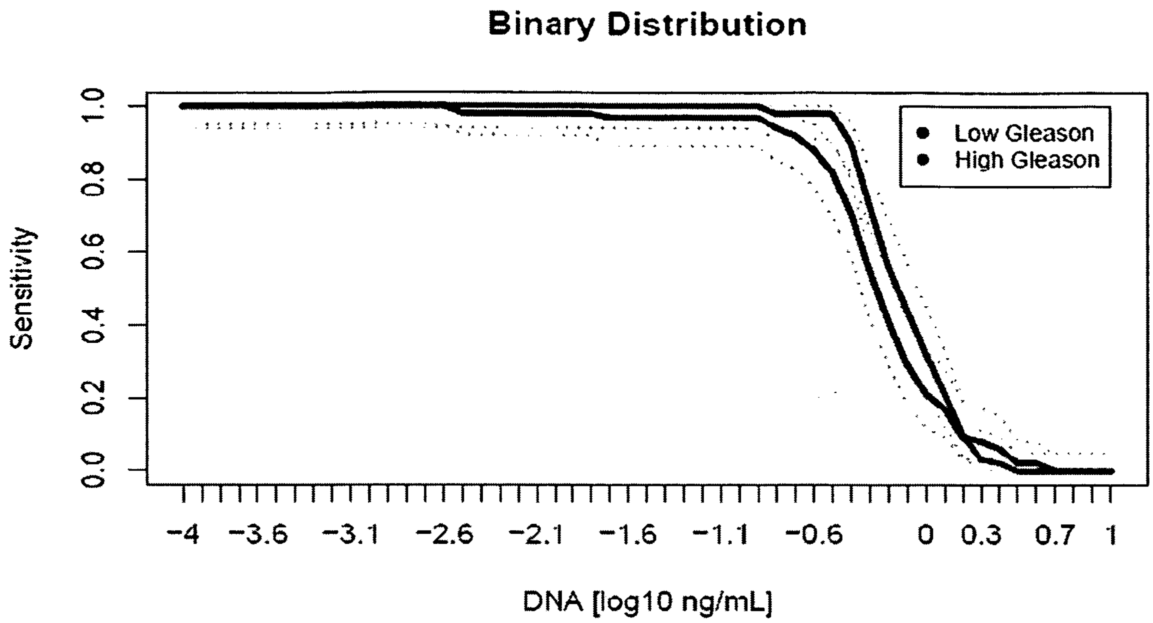
Binary Distribution
Sensitivity
0.0
0.2
0.4
0.6
0.8
1.0
Low Gleason
High Gleason
-4
-3.6
-3.1
-2.6
-2.1
-1.6
-1.1
-0.6
0
0.3
0.7
1
DNA [log10 ng/mL]

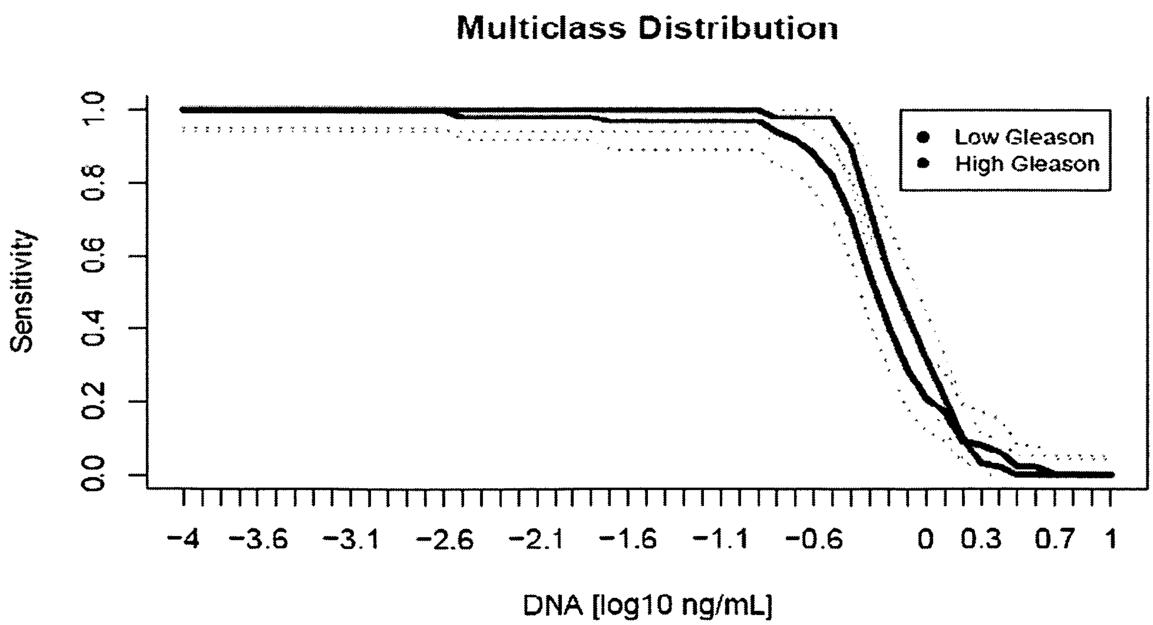
Multiclass Distribution
Sensitivity
0.0
0.2
0.4
0.6
0.8
1.0
Low Gleason
High Gleason
-4
-3.6
-3.1
-2.6
-2.1
-1.6
-1.1
-0.6
0
0.3
0.7
1
DNA [log10 ng/mL]

Figure 116/1

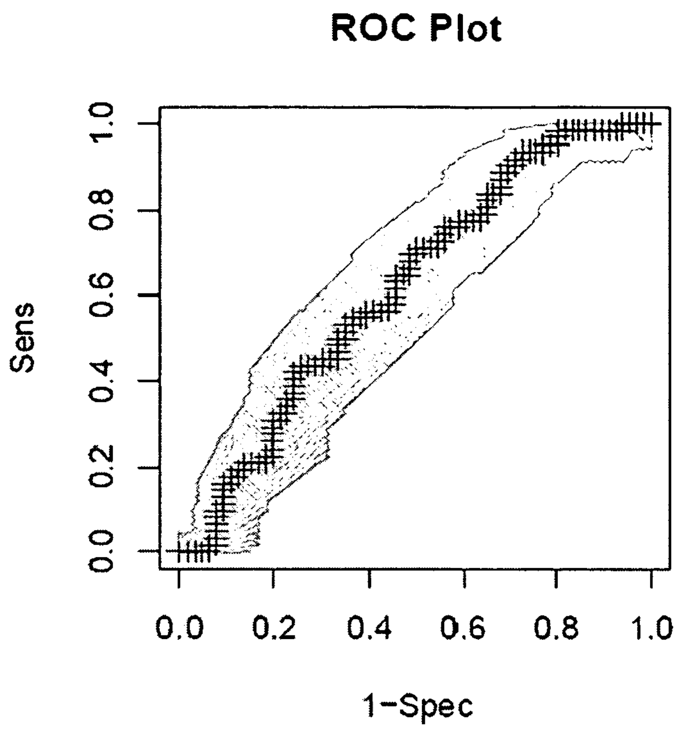


| | |
|---|---|
| AUC | 0.62 [0.53,0.7] |
| Sens / Spec | 0.21 / 0.86 |
| Wilcoxon *P* | 0.0191 |
| High Gleason (pos) | 62 |
| Low Gleason (neg) | 66 |

**Figure 116/2**

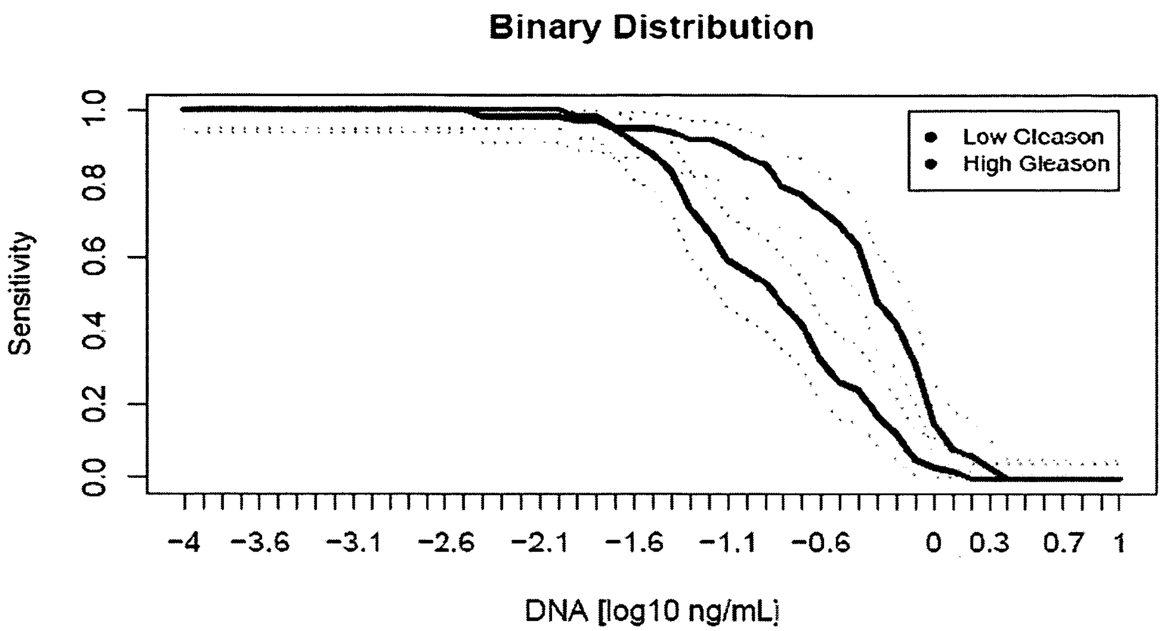
Binary Distribution
Low Gleason
High Gleason
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

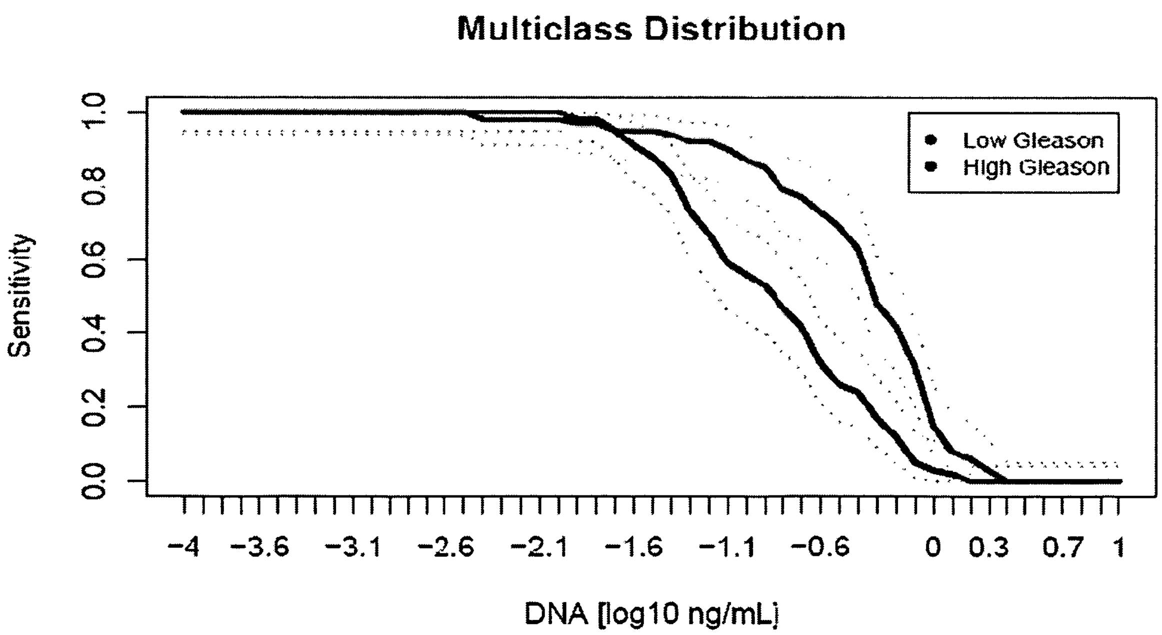
Multiclass Distribution
Low Gleason
High Gleason
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

Figure 117/1

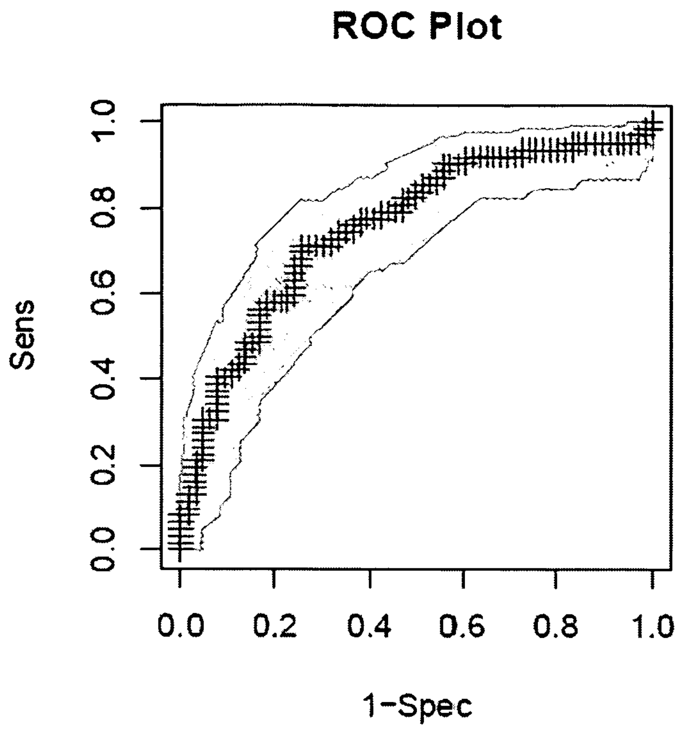


| | |
|---|---|
| AUC | 0.76 [0.67,0.83] |
| Sens / Spec | 0.48 / 0.86 |
| Wilcoxon *P* | 0 |
| High Gleason (pos) | 62 |
| Low Gleason (neg) | 66 |

**Figure 117/2**

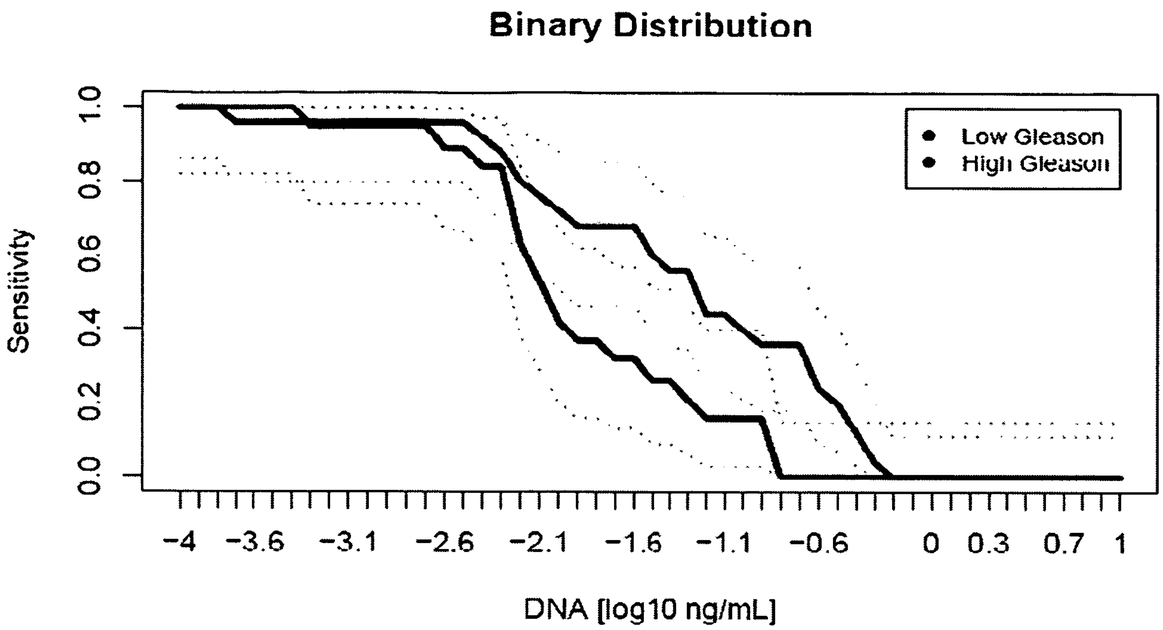
Binary Distribution
Low Gleason
High Gleason
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

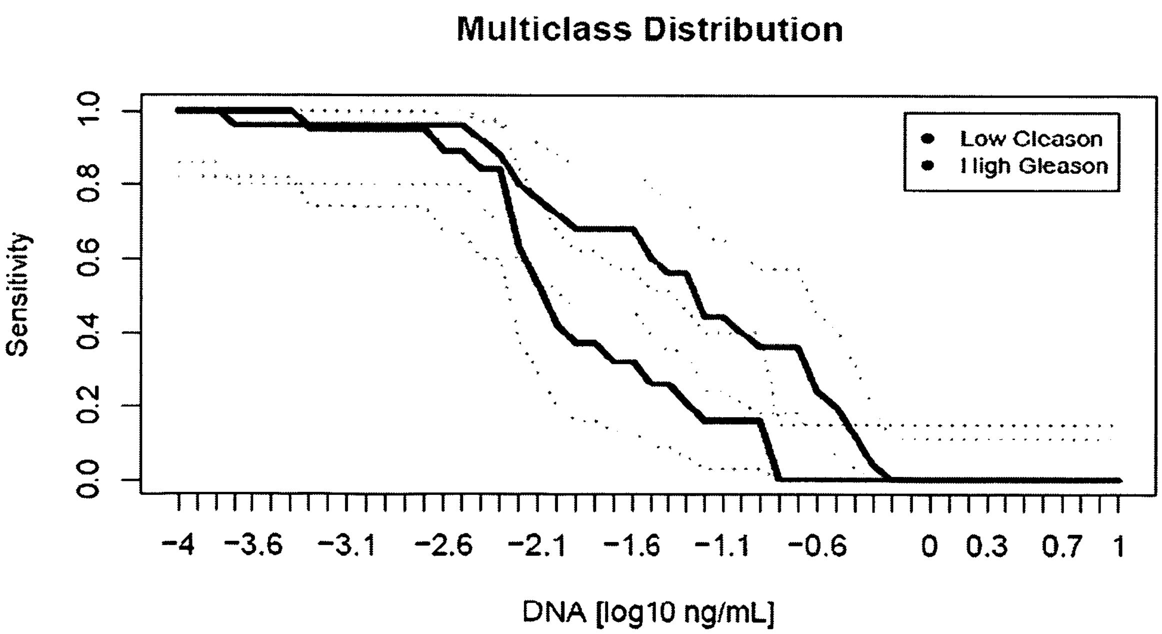
Multiclass Distribution
Low Gleason
High Gleason
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

Figure 118/1

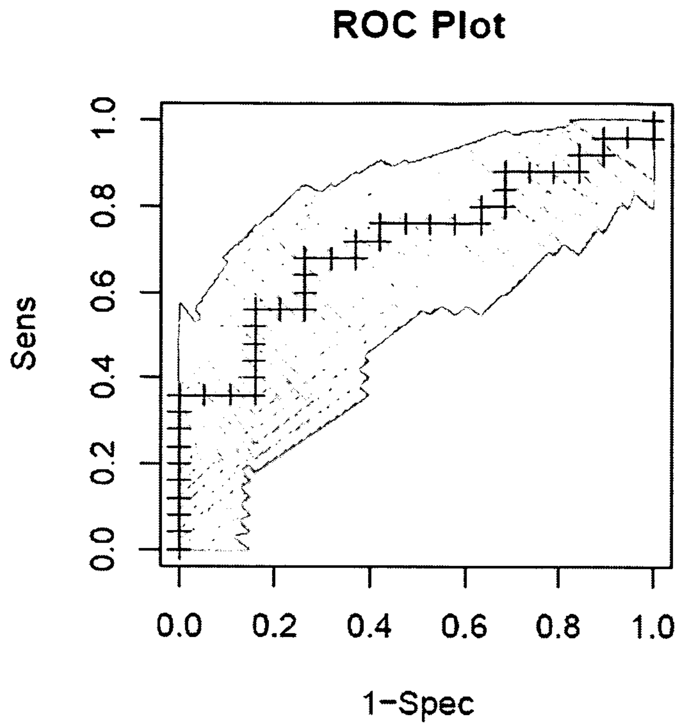


| | |
|---|---|
| AUC | 0.72 [0.55,0.83] |
| Sens / Spec | 0.36 / 0.89 |
| Wilcoxon *P* | 0.0146 |
| High Gleason (pos) | 25 |
| Low Gleason (neg) | 19 |

Figure 118/2

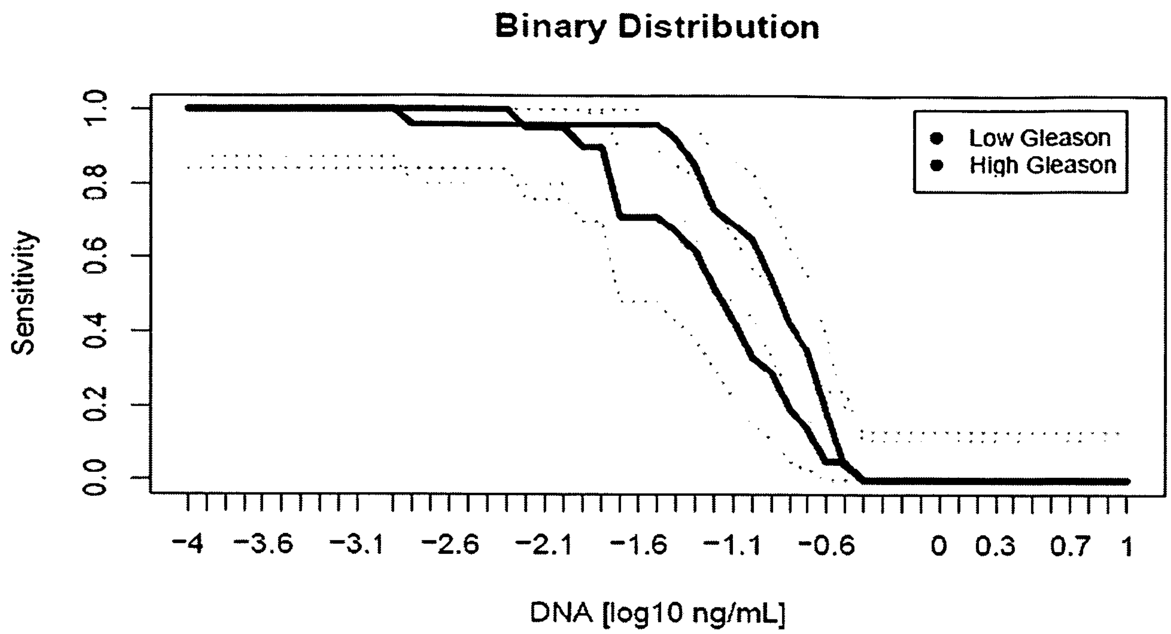
Binary Distribution
Sensitivity
1.0
0.8
0.6
0.4
0.2
0.0
Low Gleason
High Gleason
-4 -3.6 -3.1 -2.6 -2.1 -1.6 -1.1 -0.6 0 0.3 0.7 1
DNA [log10 ng/mL]

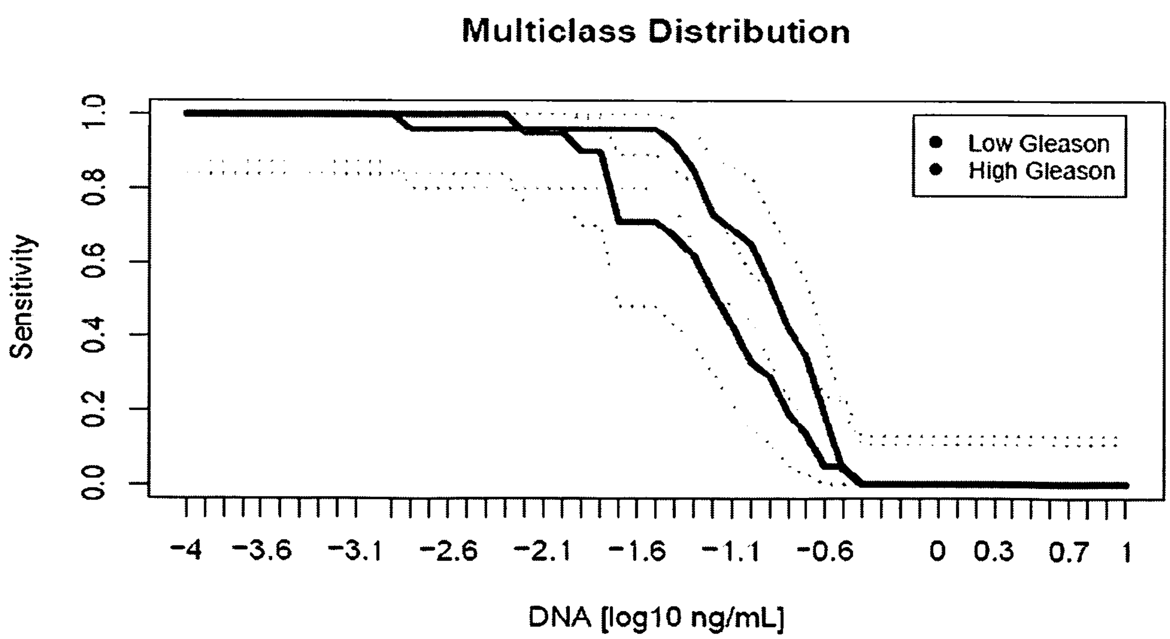
Multiclass Distribution
Sensitivity
1.0
0.8
0.6
0.4
0.2
0.0
Low Gleason
High Gleason
-4 -3.6 -3.1 -2.6 -2.1 -1.6 -1.1 -0.6 0 0.3 0.7 1
DNA [log10 ng/mL]

Figure 119/1

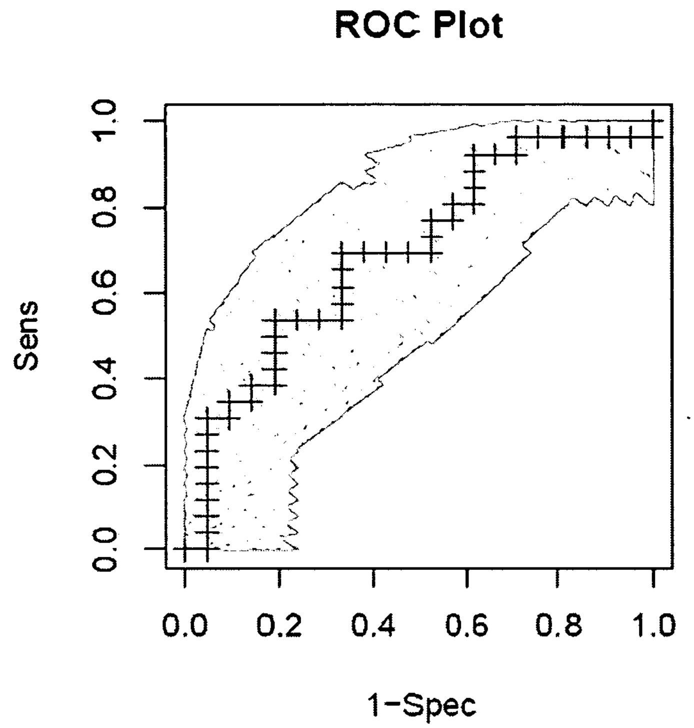


| | |
|---|---|
| AUC | 0.7 [0.55,0.83] |
| Sens / Spec | 0.38 / 0.86 |
| Wilcoxon *P* | 0.0217 |
| High Gleason (pos) | 26 |
| Low Gleason (neg) | 21 |

Figure 119/2

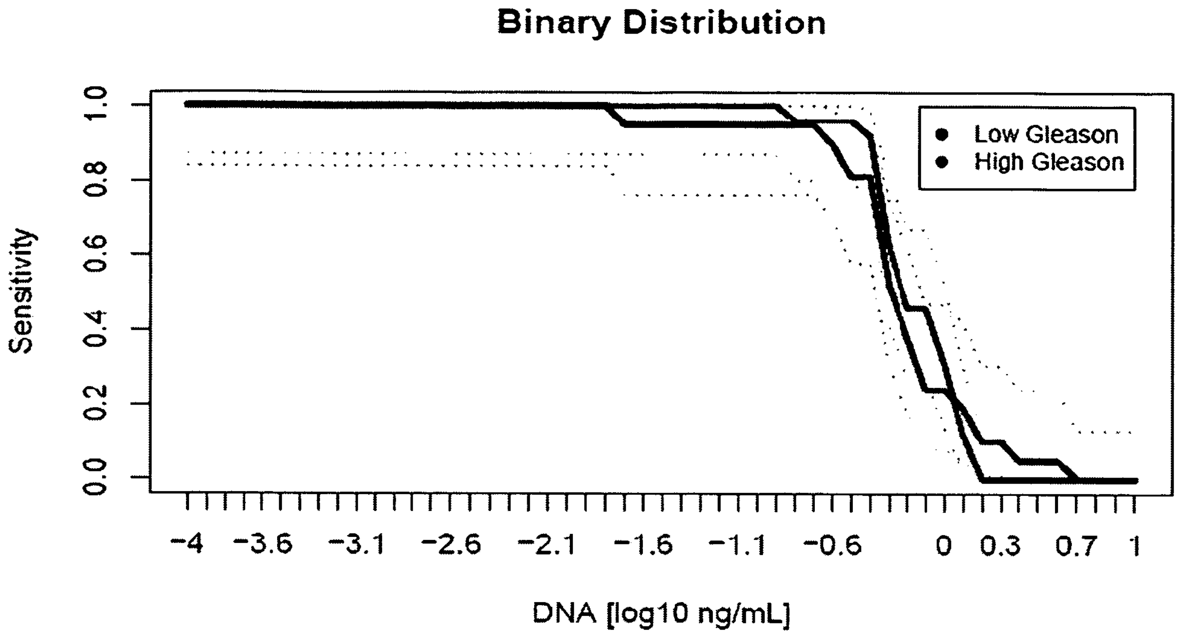
Binary Distribution
Low Gleason
High Gleason
Sensitivity
0.0
0.2
0.4
0.6
0.8
1.0
−4
−3.6
−3.1
−2.6
−2.1
−1.6
−1.1
−0.6
0
0.3
0.7
1
DNA [log10 ng/mL]

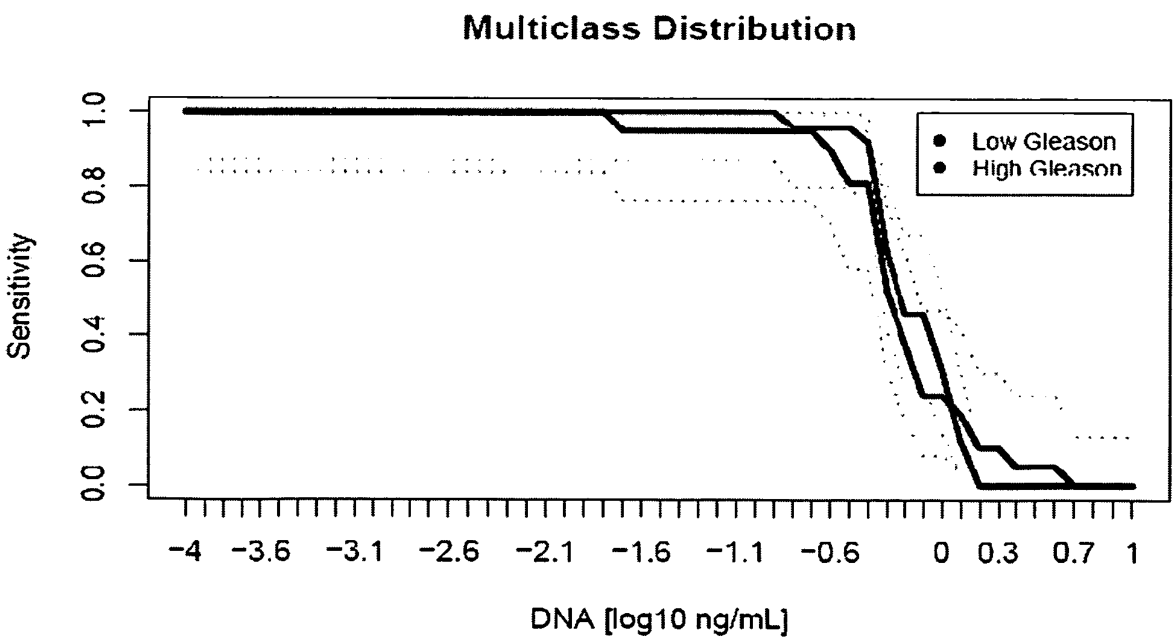
Multiclass Distribution
Low Gleason
High Gleason
Sensitivity
0.0
0.2
0.4
0.6
0.8
1.0
−4
−3.6
−3.1
−2.6
−2.1
−1.6
−1.1
−0.6
0
0.3
0.7
1
DNA [log10 ng/mL]

Figure 120/1

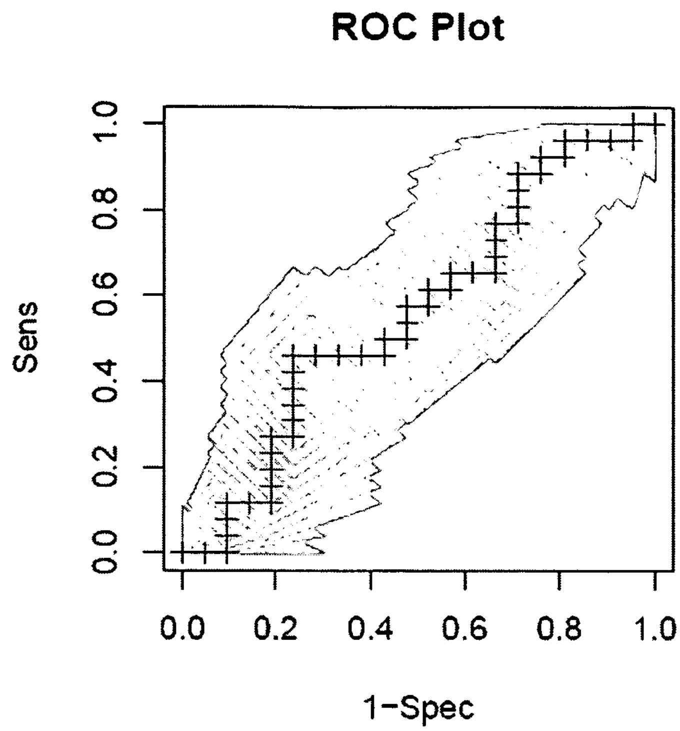


| | |
|---|---|
| AUC | 0.56 [0.4,0.7] |
| Sens / Spec | 0.12 / 0.86 |
| Wilcoxon *P* | 0.4771 |
| High Gleason (pos) | 26 |
| Low Gleason (neg) | 21 |

**Figure 120/2**

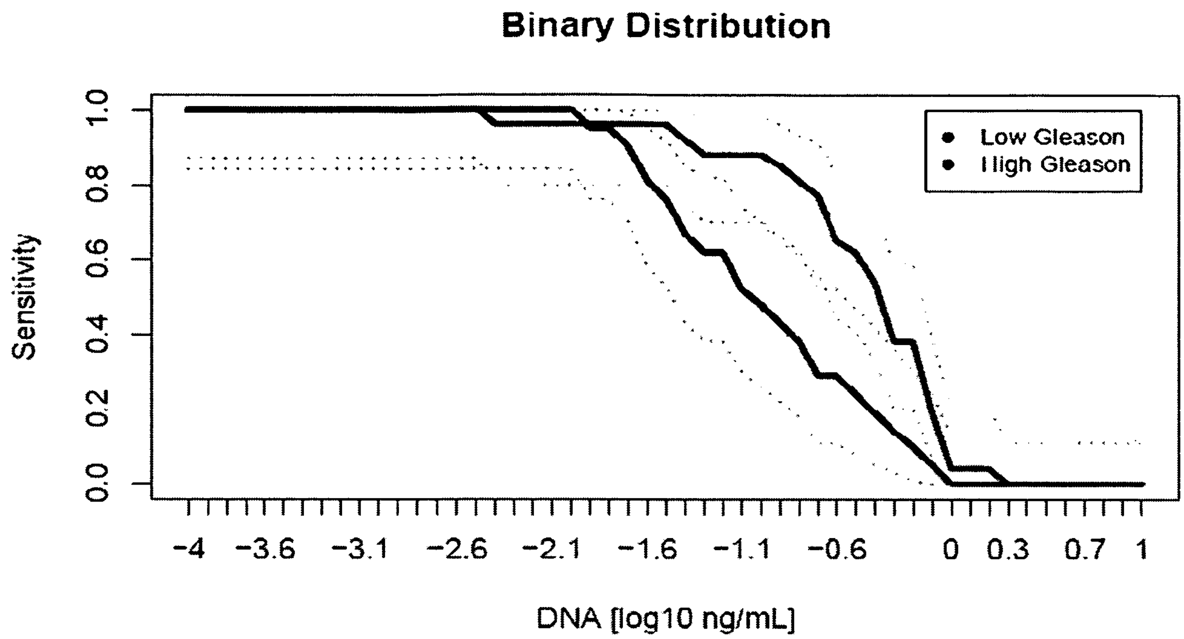
Binary Distribution
Low Gleason
High Gleason
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

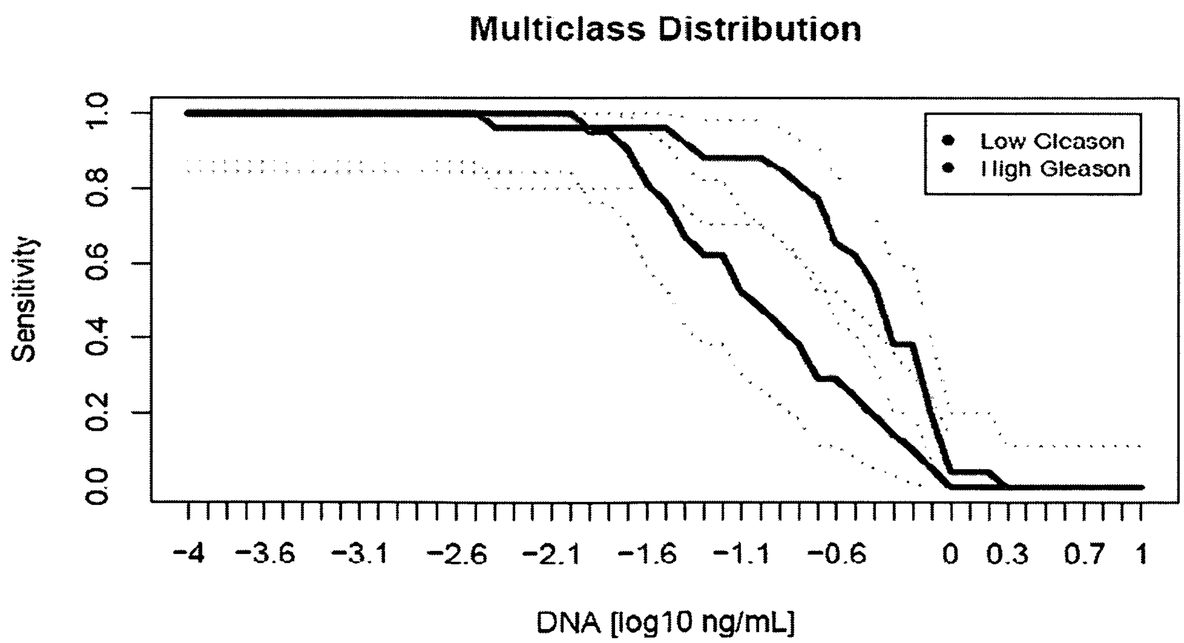
Multiclass Distribution
Low Gleason
High Gleason
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

Figure 121/1

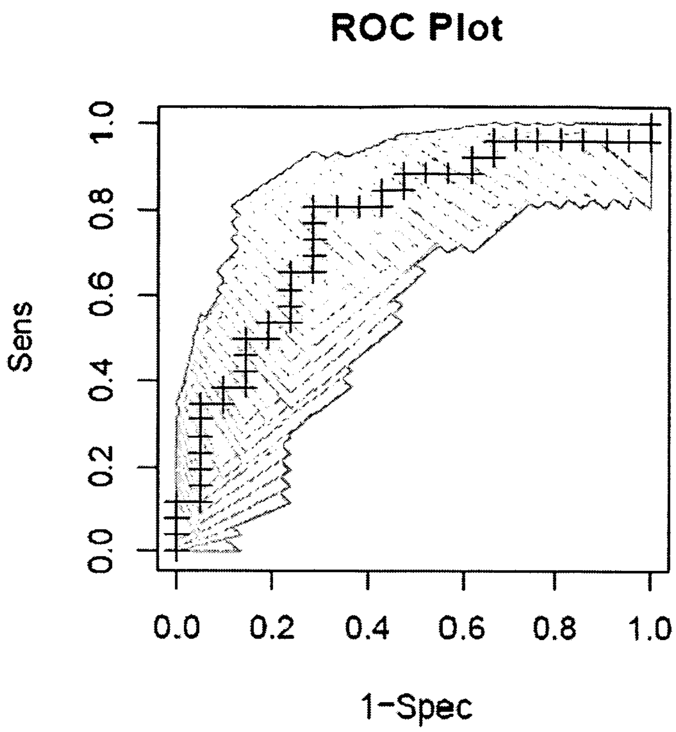


| | |
|---|---|
| AUC | 0.77 [0.62,0.88] |
| Sens / Spec | 0.5 / 0.86 |
| Wilcoxon *P* | 0.0014 |
| High Gleason (pos) | 26 |
| Low Gleason (neg) | 21 |

**Figure 121/2**

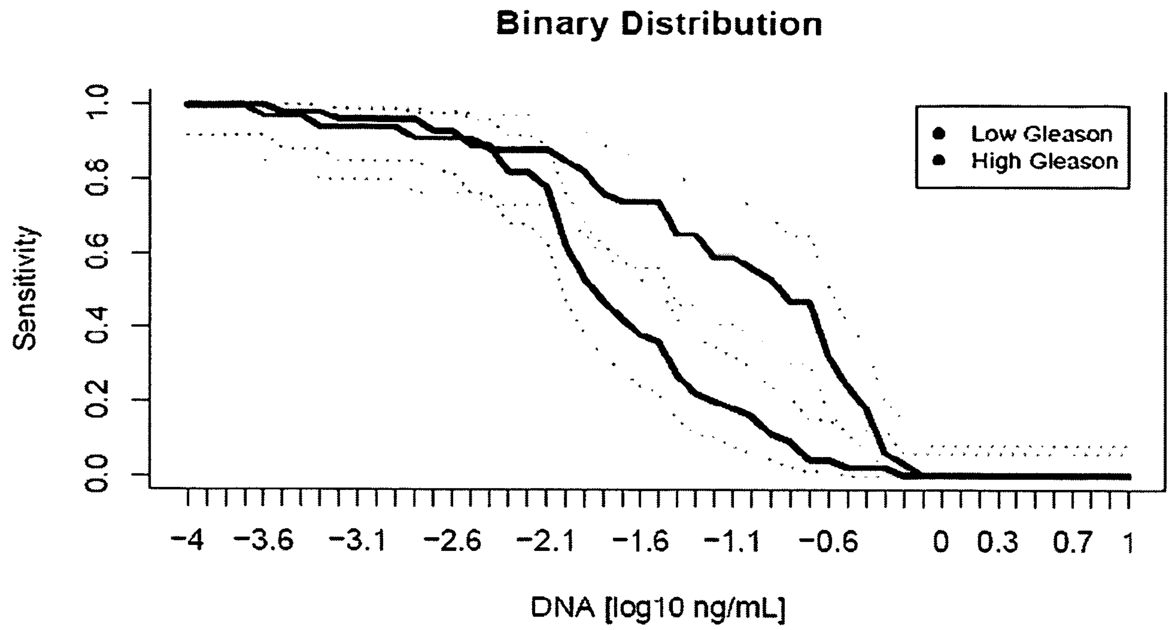
Binary Distribution
Sensitivity
0.0
0.2
0.4
0.6
0.8
1.0
Low Gleason
High Gleason
-4 -3.6 -3.1 -2.6 -2.1 -1.6 -1.1 -0.6 0 0.3 0.7 1
DNA [log10 ng/mL]

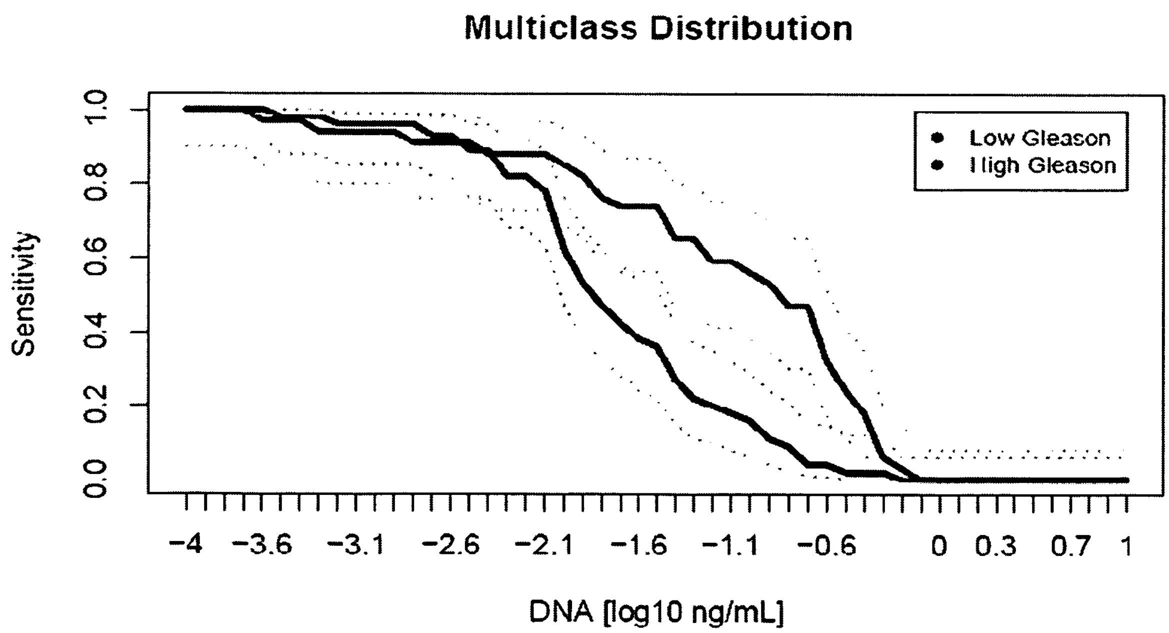
Multiclass Distribution
Sensitivity
0.0
0.2
0.4
0.6
0.8
1.0
Low Gleason
High Gleason
-4 -3.6 -3.1 -2.6 -2.1 -1.6 -1.1 -0.6 0 0.3 0.7 1
DNA [log10 ng/mL]

Figure 122/1

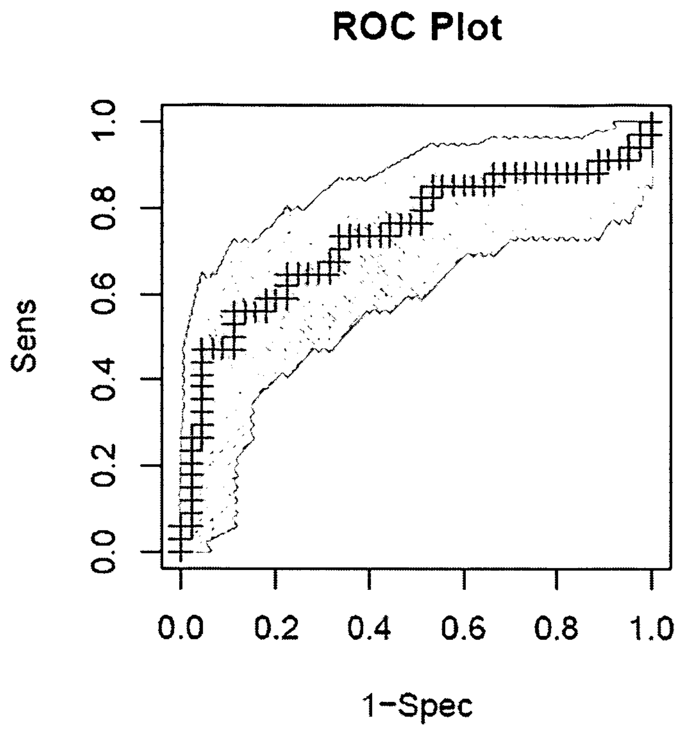


| | |
|---|---|
| AUC | 0.74 [0.62,0.83] |
| Sens / Spec | 0.56 / 0.87 |
| Wilcoxon *P* | 2e-04 |
| High Gleason (pos) | 34 |
| Low Gleason (neg) | 45 |

Figure 122/2

Binary Distribution

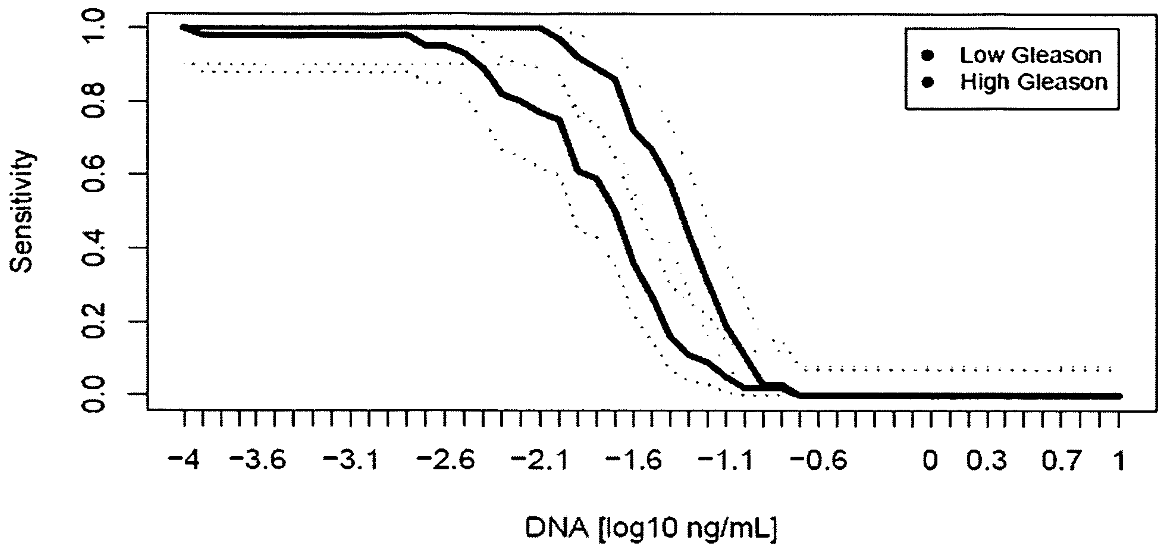


Multiclass Distribution

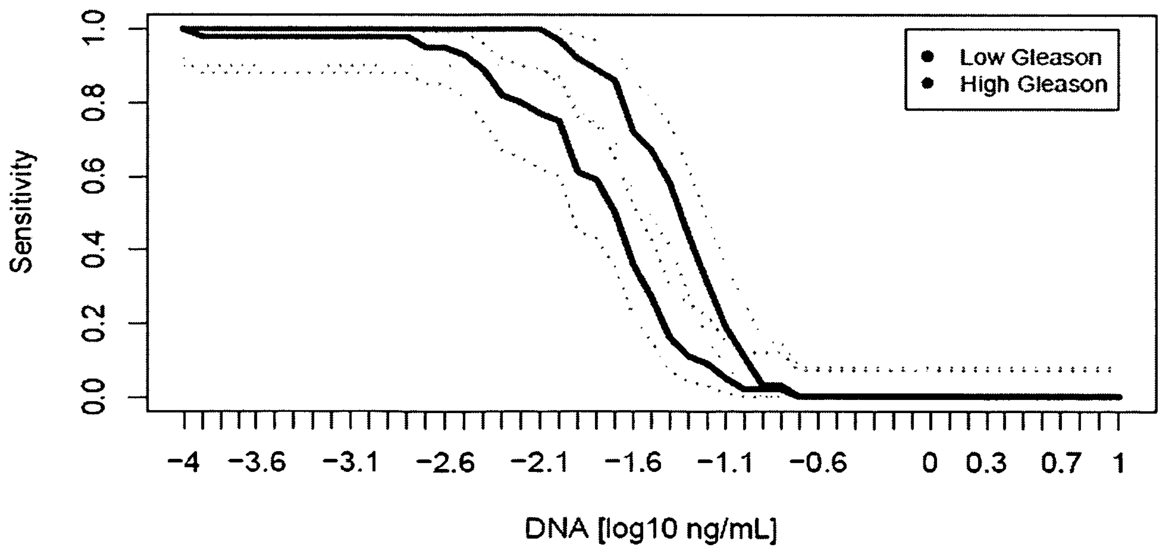


Figure 123/1

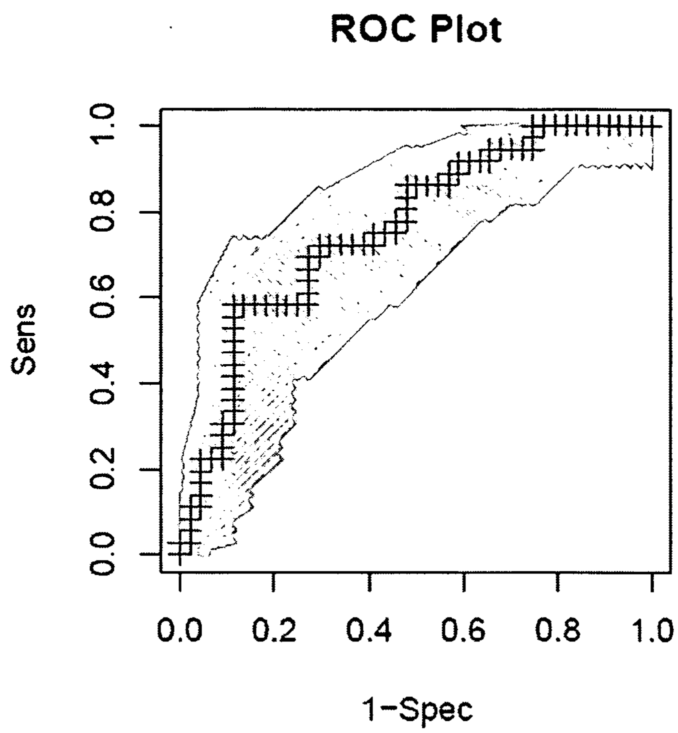


| | |
|---|---|
| AUC | 0.76 [0.65,0.85] |
| Sens / Spec | 0.58 / 0.86 |
| Wilcoxon *P* | 0 |
| High Gleason (pos) | 36 |
| Low Gleason (neg) | 44 |

**Figure 123/2**

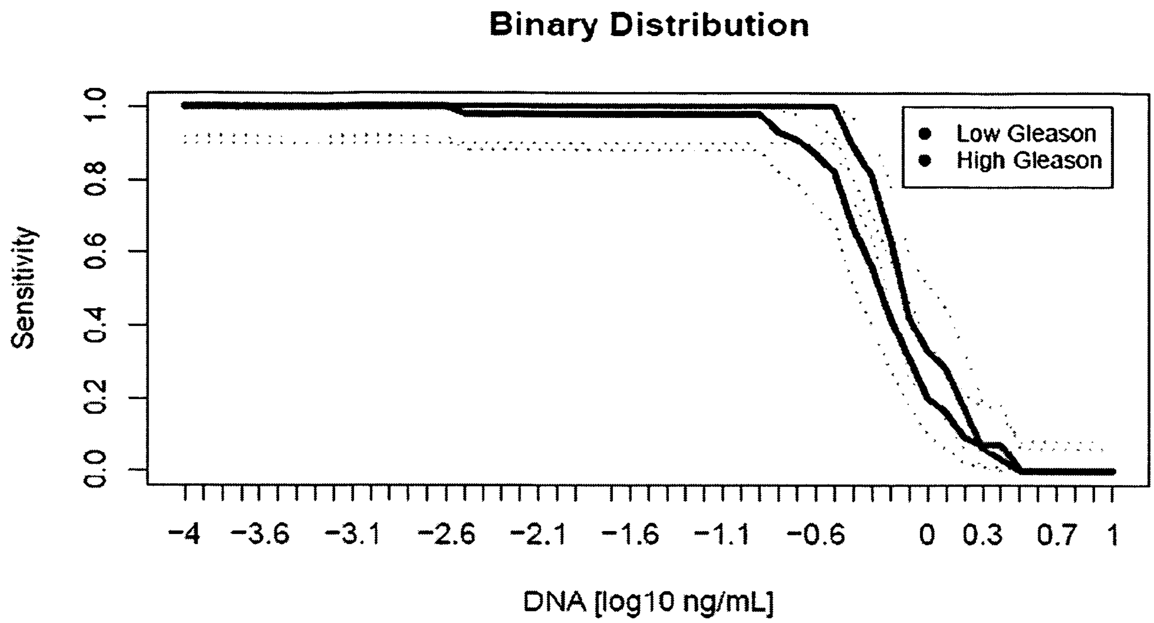
Binary Distribution
Low Gleason
High Gleason
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

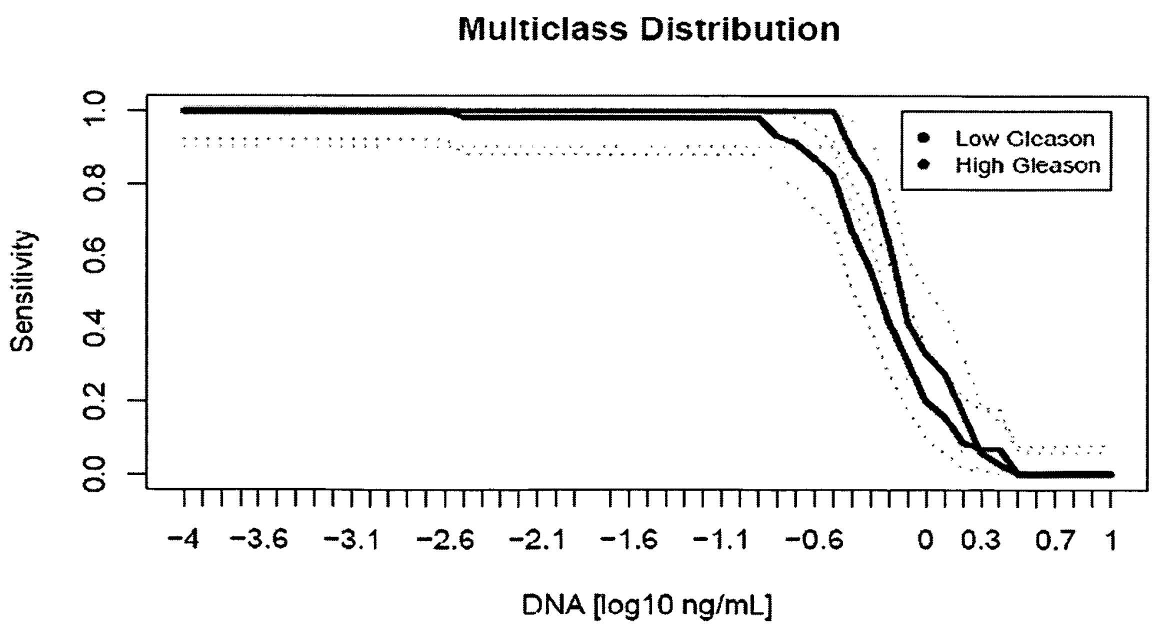
Multiclass Distribution
Low Gleason
High Gleason
Sensitivity
0.0
0.2
0.4
0.6
0.8
1.0
-4
-3.6
-3.1
-2.6
-2.1
-1.6
-1.1
-0.6
0
0.3
0.7
1
DNA [log10 ng/mL]

Figure 124/1

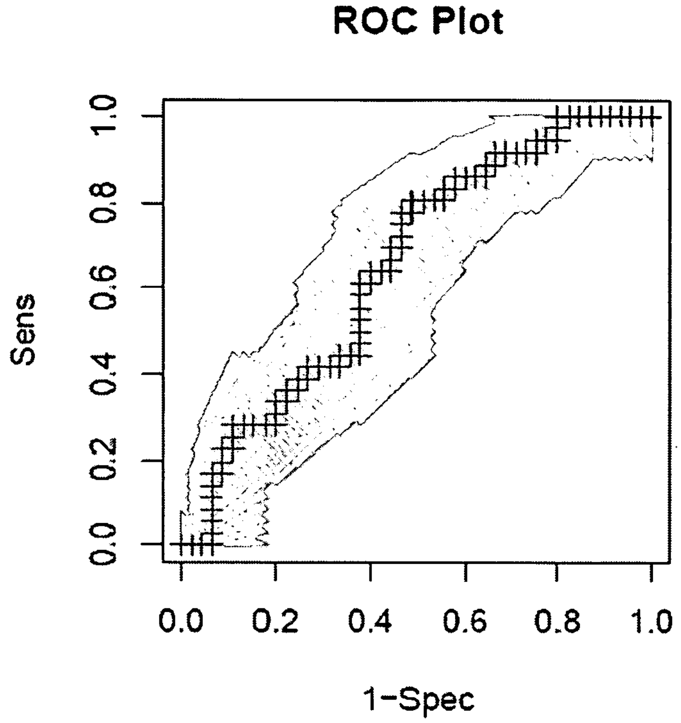


| | |
|---|---|
| AUC | 0.65 [0.54,0.76] |
| Sens / Spec | 0.28 / 0.87 |
| Wilcoxon *P* | 0.0186 |
| High Gleason (pos) | 36 |
| Low Gleason (neg) | 45 |

Figure 124/2

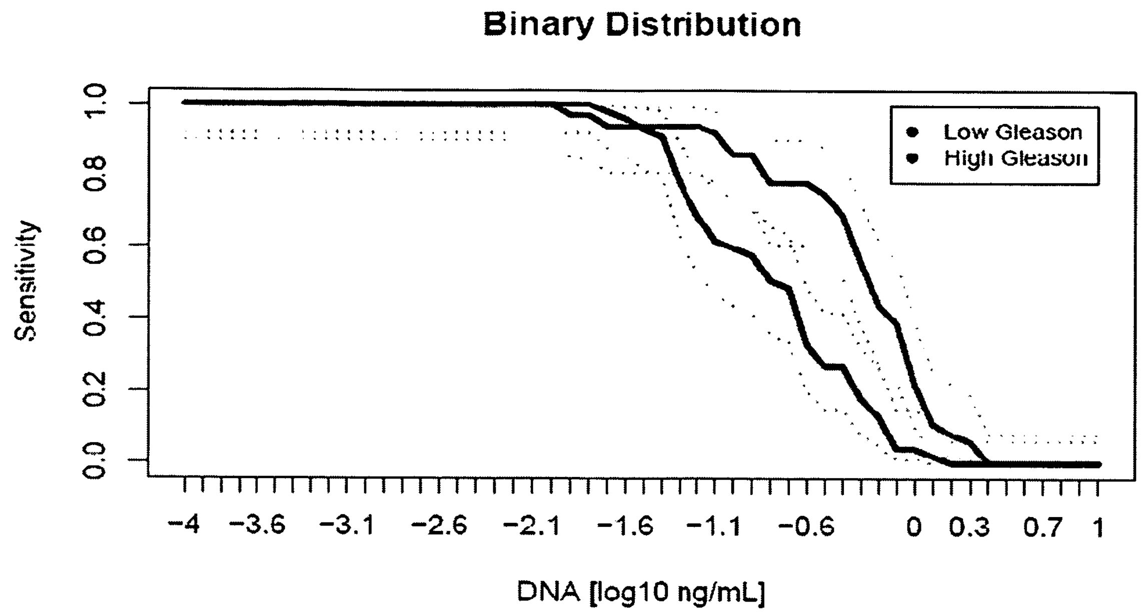
Binary Distribution
Sensitivity
0.0
0.2
0.4
0.6
0.8
1.0
Low Gleason
High Gleason
-4
-3.6
-3.1
-2.6
-2.1
-1.6
-1.1
-0.6
0
0.3
0.7
1
DNA [log10 ng/mL]

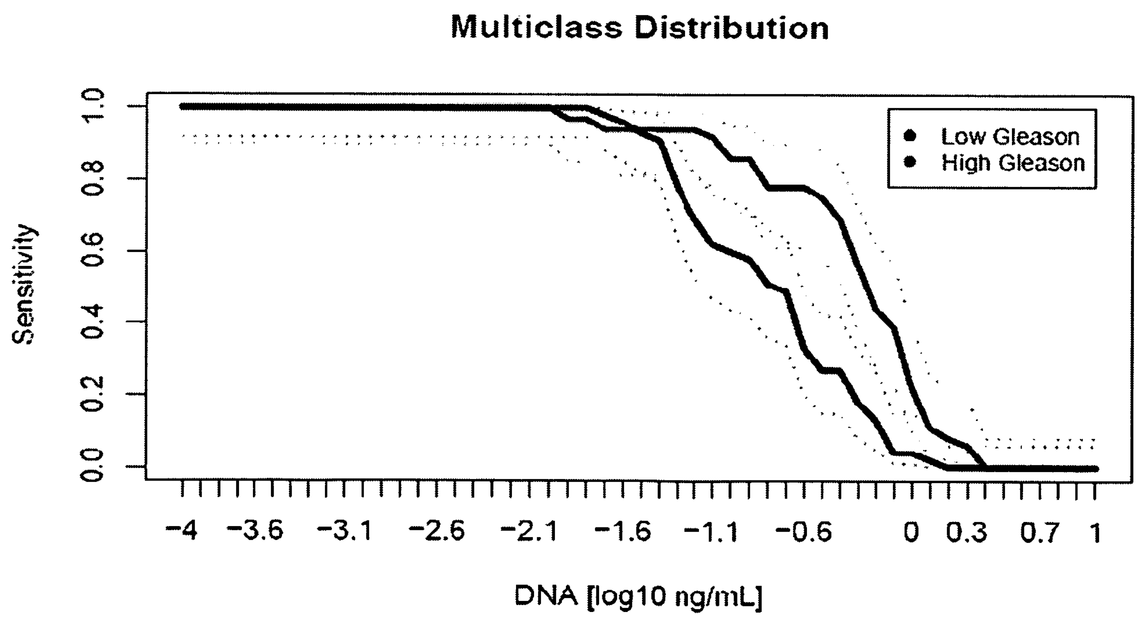
Multiclass Distribution
Sensitivity
0.0
0.2
0.4
0.6
0.8
1.0
Low Gleason
High Gleason
-4
-3.6
-3.1
-2.6
-2.1
-1.6
-1.1
-0.6
0
0.3
0.7
1
DNA [log10 ng/mL]

Figure 125/1

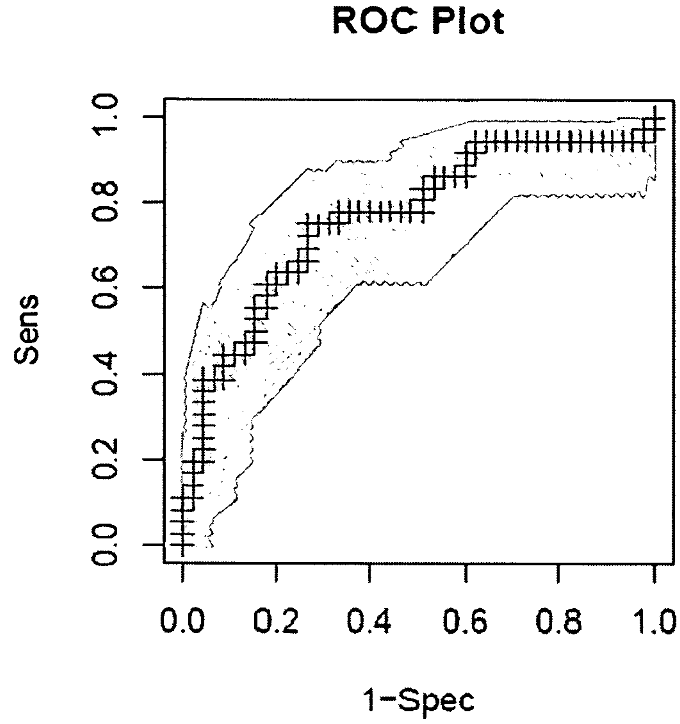


| | |
|---|---|
| AUC | 0.77 [0.66,0.85] |
| Sens / Spec | 0.47 / 0.87 |
| Wilcoxon *P* | 0 |
| High Gleason (pos) | 36 |
| Low Gleason (neg) | 45 |

Figure 125/2

Europäisches Patentamt
European Patent Office
Office européen des brevets

# EUROPEAN SEARCH REPORT

Application Number
EP 09 01 0002

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 01/19845 A (THE JOHNS HOPKINS UNIVERSITY SCHOOL OF MEDICINE) 22 March 2001 (2001-03-22) * claims 10,12,24 * ----- | 1-6,9 | INV. C12Q1/68 |
| Y | EP 1 379 694 A (EPIGENOMICS AG) 14 January 2004 (2004-01-14) * the whole document * ----- | 1-6,9 | |
| Y | WO 02/070742 A (EPIGENOMICS AG) 12 September 2002 (2002-09-12) * the whole document * ----- | 1-6,9 | |
| A | WO 2004/035803 A (EPIGENOMICS AG; FOEKENS, JOHN; HARBECK, NADIA; KOENIG, THOMAS; MAIER,) 29 April 2004 (2004-04-29) * the whole document * ----- | 1-13 | |
| X | KOZLOWSKI K ET AL: "Variation in residual PITX2 activity underlies the phenotypic spectrum of anterior segment developmental disorders." HUMAN MOLECULAR GENETICS 1 SEP 2000, vol. 9, no. 14, 1 September 2000 (2000-09-01), pages 2131-2139, XP002571915 ISSN: 0964-6906 * the whole document * ----- | 7 | TECHNICAL FIELDS SEARCHED (IPC) C12Q |
| X | GAGE P J ET AL: "Pituitary homeobox 2, a novel member of the bicoid-related family of homeobox genes, is a potential regulator of anterior structure formation." HUMAN MOLECULAR GENETICS MAR 1997, vol. 6, no. 3, March 1997 (1997-03), pages 457-464, XP002571916 ISSN: 0964-6906 * the whole document * ----- | 8 | |

The present search report has been drawn up for all claims

1

| Place of search | Date of completion of the search | Examiner |
|---|---|---|
| The Hague | 8 March 2010 | Gabriels, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 09 01 0002

This annex lists the patent family members relating to the patent documents cited in the above-mentioned European search report.
The members are as contained in the European Patent Office EDP file on
The European Patent Office is in no way liable for these particulars which are merely given for the purpose of information.

08-03-2010

| Patent document cited in search report | | Publication date | Patent family member(s) | | | Publication date |
|---|---|---|---|---|---|---|
| WO 0119845 | A | 22-03-2001 | AU | 783645 | B2 | 17-11-2005 |
| | | | AU | 7586900 | A | 17-04-2001 |
| | | | AU | 2006200264 | A1 | 23-02-2006 |
| | | | CA | 2388419 | A1 | 22-03-2001 |
| | | | EP | 1218399 | A1 | 03-07-2002 |
| | | | JP | 2003509039 | T | 11-03-2003 |
| | | | US | 2009148848 | A1 | 11-06-2009 |
| | | | US | 6783933 | B1 | 31-08-2004 |
| | | | US | 2005026207 | A1 | 03-02-2005 |
| EP 1379694 | A | 14-01-2004 | AT | 325894 | T | 15-06-2006 |
| | | | CA | 2439779 | A1 | 12-09-2002 |
| | | | DE | 60211324 | T2 | 08-02-2007 |
| | | | WO | 02070741 | A2 | 12-09-2002 |
| | | | ES | 2264728 | T3 | 16-01-2007 |
| | | | JP | 2004532013 | T | 21-10-2004 |
| WO 02070742 | A | 12-09-2002 | AT | 429511 | T | 15-05-2009 |
| | | | CA | 2439854 | A1 | 12-09-2002 |
| | | | EP | 1364069 | A1 | 26-11-2003 |
| | | | JP | 2004529630 | T | 30-09-2004 |
| WO 2004035803 | A | 29-04-2004 | AT | 437962 | T | 15-08-2009 |
| | | | AU | 2003300504 | A1 | 04-05-2004 |
| | | | AU | 2009251178 | A1 | 21-01-2010 |
| | | | CA | 2500255 | A1 | 29-04-2004 |
| | | | EP | 1554407 | A2 | 20-07-2005 |
| | | | EP | 2157191 | A2 | 24-02-2010 |
| | | | ES | 2330328 | T3 | 09-12-2009 |
| | | | JP | 2006515513 | T | 01-06-2006 |
| | | | US | 2006121467 | A1 | 08-06-2006 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 60632426 B
- US 60662220 B
- US 60723125 B
- US 60633250 B
- US 60723054 B
- US 350763 A
- WO 2005059172 A
- US 5786146 A
- WO 0026401 A1
- WO 9500669 A
- US 6251594 B
- WO 9928498 A, Olek
- WO 9746705 A
- WO 9515373 A
- US 5514758 A
- US 5565552 A
- US 5567810 A
- US 5574142 A
- US 5585481 A
- US 5587371 A
- US 5597696 A
- US 5958773 A
- US 20030013091 A
- US 09898743 B
- US 6265171 B, Herman
- US 6331393 B


### Non-patent literature cited in the description


- **Lipshutz, R. J. et al.** *Nature Genetics,* 1999, vol. 21, 20-24
- **Bowtell, D. D. L.** *Nature Genetics,* 1999, vol. 21, 25-32
- **Santourlidis, S. et al.** *Prostate,* 1999, vol. 39, 166-74
- *Jpn J Clin Oncol,* 2004, vol. 4, 414-9
- *J Urol,* 2004, vol. 171, 187-91
- **Zhou et al.** *J Urol,* 2004, vol. 171, 2195-8
- **Rabbani et al.** *The FASEB Journal,* 2003, vol. 17, 1081-1088
- **J.P. Egan.** Signal Detection Theory and ROC Analysis. Academic Press, 1975
- **Gonzalgo et al.** *Cancer Research,* 1997, vol. 57, 594-599
- **Eads et al.** *Cancer Res,* 1999, vol. 59, 2302-2306
- **Gonzalgo ; Jones.** *Nucleic Acids Res.,* 1997, vol. 25, 2529-2531
- **Herman et al.** *Proc. Natl. Acad. Sci. USA,* 1996, vol. 93, 9821-9826
- **Xiong ; Laird.** *Nucleic Acids Res.,* 1997, vol. 25, 2532-2534
- **Toyota et al.** *Cancer Res.,* 1999, vol. 59, 2307-12
- **Sambrook et al.** Molecular Cloning, A Laboratory Manual. Cold Spring Harbor Press, 1989
- Current Protocols in Molecular Biology. John Wiley & Sons, 1995
- **Olek A et al.** A modified and improved method for bisulfite based cytosine methylation analysis. *Nucleic Acids Res.,* 1996, vol. 24, 5064-6
- **Rein, T. et al.** *Nucleic Acids Res.,* 1998, vol. 26, 2255
- **Zeschnigk M et al.** *Eur J Hum Genet.,* 1997, vol. 5, 94-98
- **Olek ; Walter.** *Nat Genet.,* 1997, vol. 17, 275-6
- **Gonzalgo ; Jones.** *Nucleic Acids Res.,* 1997, vol. 25, 2529-31
- **Xiong ; Laird.** *Nucleic Acids Res.,* 1997, vol. 25, 2532-4
- **Grigg ; Clark.** *Bioessays,* 1994, vol. 16, 431-6
- **Zeschnigk M et al.** *Hum Mol Genet.,* 1997, vol. 6, 387-95
- **Feil R et al.** *Nucleic Acids Res.,* 1994, vol. 22, 695
- **Martin V et al.** *Gene,* 1995, vol. 157, 261-4
- **Frommer et al.** *Proc. Natl. Acad. Sci. USA,* 1992, vol. 89, 1827-1831
- **Sadri ; Hornsby.** *Nucl. Acids Res.,* 1996, vol. 24, 5058-5059
- **Xiong ; Laird.** *Nucleic Acids Res,* 1997, vol. 25, 2532-2534
- **Toyota et al.** *Cancer Res,* 1999, vol. 59, 2307-12
- **Eads et al.** *Cancer Res.,* 1999, vol. 59, 2302-2306
- **Belyavsky et al.** *Nucl Acid Res,* 1989, vol. 17, 2919-2932
- **Krug ; Berger.** Methods in Enzymology. Academic Press, 1987, vol. 152, 316-325

- **Sambrook et al.** Molecular Cloning: A Laboratory Manual. 1989
- Basic and Clinical Immunology. Appleton & Lange, 1991, 217-262
- **Milstein ; Kohler.** *Nature,* 1975, vol. 256, 495-497
- **Gulfre ; Milstein.** Methods in Enzymology: Immunochemical Techniques. Academic Press, 1981, vol. 73, 1-46
- **Krol et al.** *BioTechniques,* 1988, vol. 6, 958-976
- **Zon.** *Pharm. Res.,* 1988, vol. 5, 539-549
- Nature Genetics Supplement. January 1999, vol. 21
- **Yu et al.** *BioTechniques,* 1997, vol. 23, 714-720
- **Karas ; Hillenkamp.** *Anal Chem.,* 1988, vol. 60, 2299-301
- **Gut ; Beck.** *Current Innovations and Future Trends,* 1995, vol. 1, 147-57
- **Gut ; Beck.** *Nucleic Acids Res.,* 1995, vol. 23, 1367-73
- **Heid et al.** *Genome Res.,* 1996, vol. 6, 986-994
- **Gonzalgo ; Jones.** *Nucleic Acids Res,* 1997, vol. 25, 2529-2531
- **Sanger F. et al.** *Proc Natl Acad Sci USA,* 1977, vol. 74, 5463-5467
- **Olek et al.** *Nucleic Acids Res.,* 15 December 1996, vol. 24 (24), 5064-6